# EUROPEAN PATENT APPLICATION

(11) **EP 1 186 614 A2**
(43) Date of publication of application: **13.03.2002**
(21) Application number: 01306112.2
(22) Date of filing: 16.07.2001
(51) Int. Cl.: C07K 14/195, G06F 17/50

(54) **Crystal structure of the 30S ribosome bound to antibiotics**

(30) Priority: 14.07.2000 GB 0017376; 19.09.2000 GB 0022943; 07.12.2000 GB 0029872; 07.12.2000 GB 0029870; 07.12.2000 GB 0029871; 03.05.2001 GB 0110885
(71) Applicant: Medical Research Council, London W1B 1AL (GB)
(72) Inventor: Ramakrishnan, Venkatraman, MRC Lab. of Molecular, Cambridge, Cambridgeshire CB2 2QH (GB); Brodersen, Ditlev E. c/o MRC Lab.of Molecular Bio., Cambridge, Cambridgeshire CB2 2QH (GB); Carter, Andrew, Philip c/o MRC Lab. of Molecular, Cambridge, Cambridgeshire CB2 2QH (GB)
(74) Representative: Brasnett, Adrian Hugh

(57) **Abstract**

The invention provides high resolution X-ray crystal structures of the 30S ribosome, obtained from *Thermus thermophilus* 30S subunit, having a tetragonal space group P4₁2₁2 to which are bound an antibiotic selected from the group paromomycin, streptomycin, spectinomycin, tetracycline, pactamycin and hygromycin B. An advantageous feature of the structure is that it diffracts at about 3Å resolution. The invention also provides a crystal of 30S having the three dimensional atomic coordinates of the 30S ribosome, the coordinates being provided in any one of tables 1 to 4. The data may be used for the rational design and modelling of inhibitors for the 30S ribosome, which have potential use as antibiotics.

## Description

### Field of the Invention

The present invention relates to the provision of a high resolution crystal structure of the prokaryotic 30S ribosome subunit, and the use of this structure in drug discovery.

### Background of the Invention

The wealth of information made available through efforts in structural genomics and advances in computation has allowed structure-based drug design to emerge as a valuable tool in medicinal chemistry. In the past combinatorial chemistry, coupled with high-throughput approaches, shifted attention away from the more structure-based methods. Large-scale determination of protein structures is reversing the drug discovery process by starting with the protein structure and using it to identify and design new ligands. It is the integration of structure-based methods, virtual screening, and combinatorial chemistry that will provide the basis for more efficient drug design in the future, significantly reducing the time of the design cycle and the cost per marketed drug. Significant advances have already been made in AIDS, arthritis and cancer and in the treatment of hypertension e.g. captopril.

Translation of the genetic code occurs on the ribosome, a large nucleoprotein complex that consists of two subunits. In bacteria, the two subunits are denoted 30S and 50S. The 50S subunit contains the catalytic site of peptidyl transferase activity, while the 30S subunit plays a crucial role in decoding messenger RNA. Protein synthesis is a complex, multistep process that requires several extrinsic GTP-hydrolysing protein factors during each of the main stages of initiation, elongation and termination. Despite several decades of work, the molecular details of the process are poorly understood, and the elucidation of the mechanism of translation is one of the fundamental problems in molecular biology today. A recent collection of articles summarizes the state of understanding of the field [1].

A contribution to this problem was made by Yonath and coworkers, who after nearly a decade of work showed that structures as large as the 50S ribosomal subunit would form crystals that diffract beyond 3Å resolution [2]. Originally, it was not clear that phase information from such a large asymmetric unit could be obtained to high resolution, but the development of bright, tunable synchrotron radiation sources, large and accurate area detectors, vastly improved crystallographic computing, and the advent of cryo-crystallography have all contributed to making structural studies of the ribosome more tractable. In our work, the use of anomalous scattering from the LIII edges of lanthanides and osmium has also played a critical role in obtaining phases.

The 30S ribosomal subunit (hereafter referred to as 30S) from *Thermus thermophilus* was originally crystallized by Trakhanov *et al.* in 2-methyl-2,4-pentanediol (MPD) [3] and soon afterwards by Yonath and coworkers in a mixture of ethyl-butanol and ethanol [4]. Subsequent work by both groups showed that the MPD crystal form diffracted to about 9-12Å resolution [5, 6]. The diffraction limit of these crystals did not improve beyond 7Å resolution for almost a decade, but more recently both Yonath and coworkers [7, 8] and we [9] obtained crystals of the MPD form that exhibit significantly improved diffraction. However, unlike the crystals obtained by the Yonath group [6], our crystals do not require soaking in tungsten clusters or heat treatment in order to obtain high resolution diffraction.

We have previously described the structure of the 30S at 5.5Å resolution [9]. We were able to place all seven proteins whose structures were known at the time, infer the structure of protein S20 to be a three-helix bundle, trace the fold of an entire domain of 16S RNA, and identify a long RNA helix at the interface that contains the decoding site of the 30S. Proteins S5 and S7 were also placed in electron density maps of the 30S obtained by Yonath and coworkers.

The 30S ribosomal subunit is a major target for antibiotics. The ribosome is a useful target for antibiotics since the structure of the 30S is widely conserved between prokaryotes, allowing for broad spectrum antibiotics. However, resistance to current antibiotics is currently a major problem in the field of medicine. There are presently very few new antibiotics available which can be used to treat the highly resistant strains of bacteria such as MRSA (methicilin resistant *Staphylococcus aureus)* which are becoming increasingly widespread.

Understanding the interaction of antibiotics with the ribosome at the molecular level is important for two reasons. Firstly, antibiotics act by interfering with various aspects of ribosome function. Thus understanding their interaction will help shed light on mechanisms involved in translation. Secondly, a detailed knowledge of antibiotic interactions with the ribosome could aid the development of new drugs against increasingly resistant strains of bacteria. Although antibiotics were characterized several decades ago, a detailed knowledge of their mechanism will in general require a three-dimensional structure of their complex with the ribosome.

The low (greater than 3Å resolution) crystal structures described above do not provide sufficiently detailed resolution for useful modelling of the crystal structure of the 30S and there is thus a need for a high resolution structure which can be used usefully in the development of novel therapeutics.

### Summary of the Invention

We have now solved and refined the structure of the 30S bound to a number of antibiotics at 3Å resolution. The structure contains all of the ordered regions of 16S RNA and 20 associated proteins, and contains over 99% of the RNA sequence and 95% of the protein sequences, with the missing parts being exclusively at the termini of RNA or polypeptide chains.

The refined atomic resolution models of the 30S presented here allows the interpretation of a vast amount of biochemical data on its function in precise structural terms. The structure will also serve as a basis for the interpretation in molecular terms of lower resolution models of various functional states by electron-microscopy or X-ray crystallography. The 30S structure will help produce testable models for various aspects of ribosome function.

In a first aspect, the present invention provides a crystal of the *Thermus thermophilus* 30S subunit bound to an antibiotic Z(where Z is defined below), having a tetragonal space group P4₁2₁2 with unit cell dimensions, for each of the antibiotics Z, of:

| Z | a(Angstroms) | b(Angstroms) | c(Angstroms) |
|---|---|---|---|
| Paromomycin (Tables 1A/B) | 401.375 | 401.375 | 175.887 |
| Paromomycin | 401.2 | 401.2 | 176.4 |
| Streptomycin | 401.375 | 401.375 | 175.887 |
| Spectinomycin | 401.375 | 401.375 | 175.887 |
| Tetracycline | 401.158 | 410.158 | 176.944 |
| Pactamycin | 401.719 | 401.719 | 177.002 |
| Hygromycin B | 402.063 | 402.063 | 175.263 |

An advantageous feature of these structures are that they diffract beyond 3Å resolution. Another feature of the structures are that they was obtained in a method which did not involve soaking crystals in heavy atom (e.g. tungsten or tantalum) clusters or heat activation. Furthermore, it is specifically of the 885-888/910-912 base pairing confirmation of 16S RNA. These features, both singly and in combination all contribute to features of the invention which are advantageous.

In a second aspect, the invention also provides a crystal of 30S having the three dimensional atomic coordinates of Table n, where Table n is any one of Tables 1 to 4. The binding of the antibiotics paromomycin, spectinomycin and streptomycin are shown in the coordinates of Table 1A, but these have been refined further from our original data. The refined coordinates are shown in Table 1B. Reference herein to "Table 1" is a reference to either of Table 1A or 1B (or where the context permits, both). Further, Table 1C represents a further data set for the antibiotic paromomycin bound to the 30S ribosome, and thus where the antibiotic Z is paromomycin, reference to Table 1 (or Table n where 1 is included) means any one of 1A, 1B or 1C.

Thus, for example, where it is stated that the invention refers to computer readable media with "atomic coordinate data according to Table 1 recorded thereon", this means that the media has either the data of Table 1A, or the data of Table 1B (and in the case of paromomycin, or Table 1C) or both (or in the case of paromomycin, any two or all three of said tables), recorded thereon. Likewise, reference to Table n where n is 1, also means either or both of 1A and/or 1B.

Equally, it will be understood that Table 1A or Table 1B may be provided with the data derived from the 30S ribosome with only a single antibiotic data set provided, and such a table forms a part of the invention.

We have also observed that 30S crystals do not contain the S1 subunit protein. In our studies, we have found that by selectively removing this protein prior to crystallization, we have been able to obtain the improved resolution described herein. Although the atomic co-ordinates provided in Table n below allows those of skill in the art to bypass the need to undertake the crystallization of the 30S, this crystallization method nonetheless forms a further aspect of the invention.

Accordingly, there is provided a method for crystallizing a the 30S subunit to obtain a high resolution structure of a 30S subunit, which method comprises providing a 30S subunit, selectively removing the S1 subunit therefrom, crystallizing the 30S and soaking into the crystals antibiotic X.

In a further aspect, the present invention provides a method for identifying a potential inhibitor of the 30S comprising the steps of:
a. employing a three-dimensional structure of 30S, or at least one sub-domain thereof, to characterize at least site bound by antibiotic X, the three-dimensional structure being defined by atomic coordinate data according to any one of Tables 1 to 6; and
b. identifying the potential inhibitor by designing or selecting a compound for interaction with the active site.

In a further aspect, the present invention provides computer readable media with either (a) atomic coordinate data according to Tables n recorded thereon, said data defining the three-dimensional structure of 30S or at least one sub-domain thereof, or (b) structure factor data for 30S recorded thereon, the structure factor data being derivable from the atomic coordinate data of Tables n.

### Description of the Drawings.

Figure 1 shows the binding of paromomycin to the 30S ribosome.
Figure 2 shows the binding of spectinomycin to the 30S ribosome.
Figure 3 shows the binding of streptomycin to the 30S ribosome.
Figure 4A shows the binding of tetracycline (primary site) to the 30S ribosome.
Figure 4B shows the binding of tetracycline (secondary site) to the 30S ribosome.
Figure 5 shows the binding of pactamycin to the 30S ribosome.
Figure 6 shows the binding of hygromycin B to the 30S ribosome.
Figure 7 is Tables 1A, 1B and 1C, providing the coordinates of the 30S ribosome bound to the antibiotics paromomycin, spectinomycin and streptomycin (1A and 1B), and paromomycin alone (1C).
Figure 8 is Table 2, providing the coordinates of the 30S ribosome bound to tetracycline (primary and secondary sites).
Figure 9 is Table 3, providing the coordinates of the 30S ribosome bound to pactamycin.
Figure 10 is Table 4, providing the coordinates of the 30S ribosome bound to hygromycin B.

### Detailed Description of the Invention.

### Definitions.

The term "sub-domain" includes the following:
(a) and element selected from the following:
   at least one complete element of secondary structure, i.e. an alpha helix or a beta sheet, or RNA helix, as described in the detailed description below;
   a group of two or more such elements which interact with each other;
   at least one subunit protein;
   a subgroup of subunit proteins, for example a group which includes two or more proteins which are found to interact with each other;
   any of the above, when being protein or element thereof being used in conjunction with all or part of the 16S RNA structure associated with said elements or proteins;
(b) a space of volume defining a region around any one particular atom of interest (e.g. an atom involved in binding to an antibiotic), the volume being less than the total volume of the tetragonal space of the complete crystal. For example, the coordinates of atoms in a volume of from about 500 to about 15,000Å³ may be selected and used for the present invention. Such a space may be a sphere having a diameter of from about 10Å to about 30Å, centred around a point of interest; and
(c) a collection of at least 10, e.g. at least 25 such as at least 50, more preferably at least 100, even more preferably at least 500 atoms and most preferably at least 1000 atoms defined by the coordinates of Table n, wherein at least 2 of said atoms, and preferably at least 50% of said atoms of the collection are located within 50Å of each other.

By "fitting", is meant determining by automatic, or semi-automatic means, interactions between one or more atoms of an potential inhibitor molecule and one or more atoms or binding sites of the 30S, and calculating the extent to which such interactions are stable. Various computer-based methods for fitting are described further herein.

By "root mean square deviation" we mean the square root of the arithmetic mean of the squares of the deviations from the mean.

An "active site" of the 30S is any part of this structure involved in binding to antibiotic Z, or regions of the antibiotic whose conformation is altered by the binding of Z. It also includes any part of this structure involved in tRNA or mRNA binding, synthesis or translocation, including regions of the complex not directly associated with tRNA or mRNA binding but which are required for the ribosome to function, for example those regions which undergo structural changes associated with protein synthesis or are target sites for regulation by co-factors, phosphorylation or acetylation.

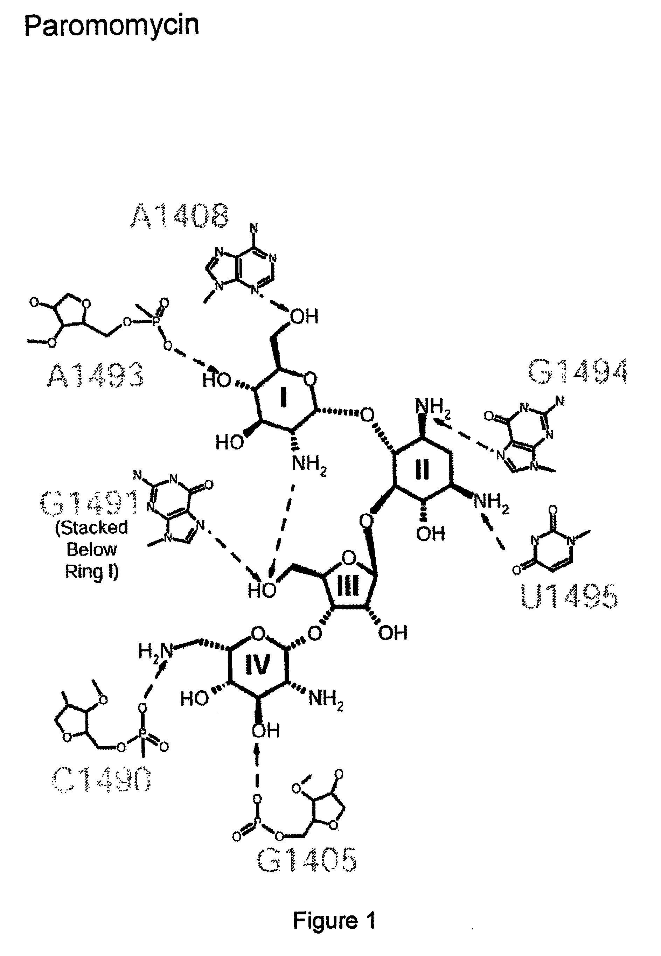

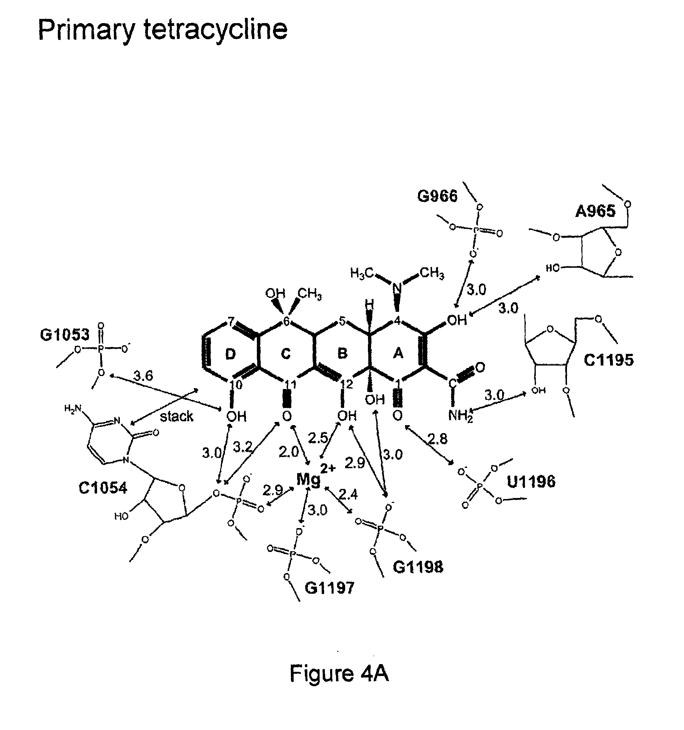

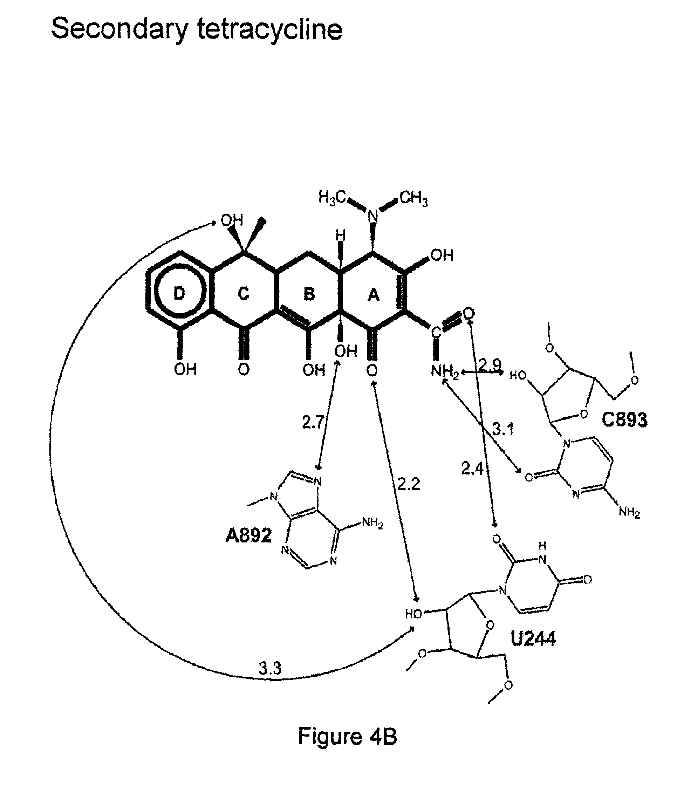

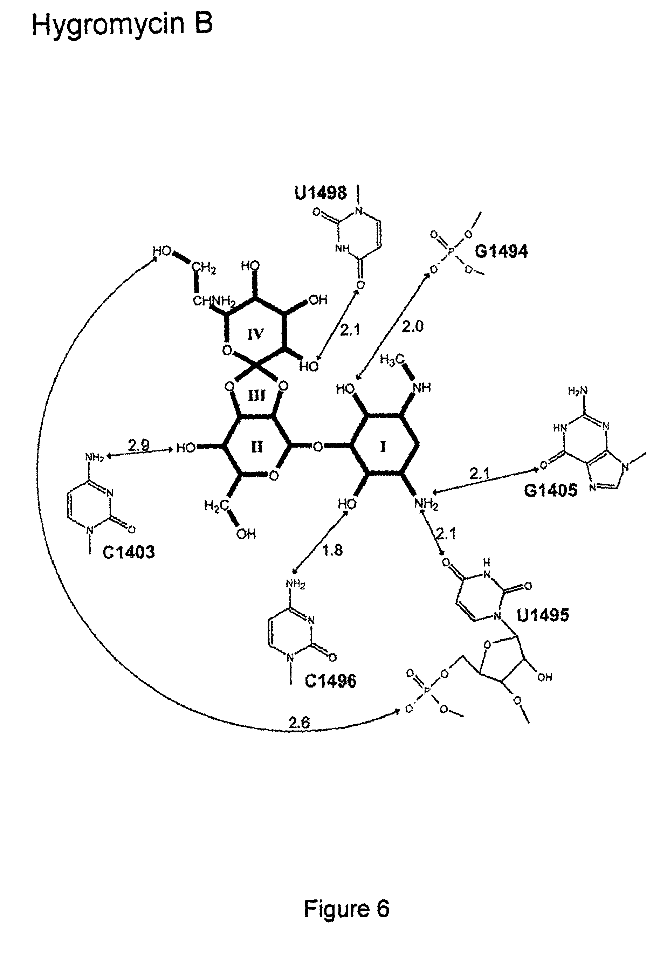

Particular regions of the 30S include those identified herein as antibiotic binding regions based on the data provided in Table n, and in the Figures 1-6. Regions further include the three tRNA sites, i.e. the aminoacyl (A), peptidyl (P) and (exit) E sites. Other active sites are those which undergo movement during translocation of tRNAs from the A to P sites and the P to E sites.

"Computer readable media" refers to any media which can be read and accessed directly by a computer. Such media include, but are not limited to: magnetic storage media such as floppy discs, hard disc storage medium and magnetic tape; optical storage media such as optical discs or CD-ROM; electrical storage media such as RAM and ROM; and hybrids of these categories such as magnetic/optical storage media.

A "computer system" refers to the hardware means, software means and data storage means used to analyse the atomic coordinate data of the present invention. The minimum hardware means of the computer-based systems of the present invention comprises a central processing unit (CPU), input means, output means and data storage means. Desirably a monitor is provided to visualise structure data. The data storage means may be RAM or means for accessing computer readable media of the invention. Examples of such systems are microcomputer workstations available from Silicon Graphics Incorporated and Sun Microsystems running Unix based, Windows NT or IBM OS/2 operating systems.

### Table n.

The coordinates of Tables 1 to 4 provide a measure of atomic location in Angstroms, to a third decimal place. In order to use the information in these Tables for the purposes described herein as being aspects of the present invention, these coordinates may be varied by up to ± 1.0, such as by up to ± 0.7, preferably no more than up to ±0.5 Angstroms, without departing from the scope of the invention.

Furthermore, varying the relative atomic positions of the atoms of the structure so that the root mean square deviation of the 16S RNA or S2-S20 protein backbone atoms is less than 1.5Å (preferably less than 1.0Å and more preferably less than 0.5Å) when superimposed on the coordinates provided in Table n for these structures, will generally result in a structure which is substantially the same as the structure of Table n respectively in terms of both its structural characteristics and potency for structure-based drug design of 30S ligands.

Thus for the purposes described herein as being aspects of the present invention, it is within the scope of the invention if: the coordinates of Table n are transposed to a different origin and/or axes; the relative atomic positions of the atoms of the structure are varied so that the root mean square deviation of conserved residue backbone atoms is less than 1.5Å (preferably less than 1.0Å and more preferably less than 0.5Å) when superimposed on the coordinates provided in Table n for the conserved residue backbone atoms; and/or the number and/or positions of water molecules is varied. Reference herein to the use of the coordinates of Table n thus includes the use of coordinates in which one or more individual values of the Table are varied in this way.

Table n includes coordinates of metal ions which are selected from zinc, cobalt and magnesium. Some or all of these ions may optionally be discarded from the Tables when using the data. The Tables also list the coordinates of a 26 amino acid peptide, Thx, as well as a 6 nucleotide fragment of mRNA, NNNUCU, designated as molecule X. Both the coordinates of both these molecules may likewise optionally be discarded, i.e. so that the coordinates of the 16S RNA and the proteins S2 to S20 alone are modeled and used in applications of the invention.

There are a few N- or C-terminal sequences of the S2 to S20 proteins which were not resolved in the structure of Table n, together with a some of the 5' and 3' residues of the 16S RNA. These are not essential for the purposes of the present invention.

This invention provides those of skill in the art a means to provide 30S crystals of *T*.*thermophilus*. The conservation of ribosome structure, particularly regions of structure essential for function, between prokaryotes, for example prokaryotes which are human pathogens, such as Staphylococcus spp, and the like, allows the structure herein to be useful in the provision of anti-bacterial agents in general. Thus, the structures may be used to solve 30S subunits by the technique of molecular replacement. In such a method, x-ray diffraction data are obtained from crystals of a 30S subunit from another species, e.g. a species of a bacteria pathogenic to humans. The coordinates of Table n may be used to find the orientation of the unknown molecule in the crystal, and electron density maps calculated. These maps can then be interpreted with the sequence of the species in question, and the coordinates of our 30S structure can be used to help and speed interpretation. In this way, the structure of our 30S facilitates the determination of structures of 30S subunits and whole ribosomes from other organisms.

Accordingly, the invention provides a method for the determination of the structure of a bacterial 30S from a species other than *T. thermophilus* which method comprises:
(a) crystallising the 30S of said species to obtain a crystal;
(b) performing X-ray crystallography on said crystal to obtain X-ray diffraction data;
(c) providing the structure data of Table 1; and
(d) using molecular replacement to calculate an electron density map of the 30S.

The crystallisation step (a) is optionally performed with an antibiotic Z, either in a co-crystallisation or by soaking the antibiotic following crystal formation. Thus the calculated electron density map may be that of the 30S - antibiotic complex.

In such a method the 30S may be prepared by removal of the S1 subunit, as described herein.

The electron density map obtained may then be used to calculate the atomic coordinate date of the 30S, optionally with bound antibiotic Z. The atomic coordinate date thus obtained may be used to for the design and analysis of new and specific ligands for 30S as described herein.

### The 30S crystal structure.

The high resolution structure provided herein provides a crystal with unit cell dimensions which are provided in the accompanying table to 3 decimal places, as set out above. However, those of skill in the art wishing to reproduce the crystallization described herein and obtain such crystals will appreciate that a degree of experimental variability and error will mean that crystals of the invention will be obtained with a unit cell dimension within, but not exactly corresponding to, this size. Thus crystals of the invention may generally be defined as having unit cell dimensions a, b, and c as defined above which vary in the case of a by ± 4.0Å, b by ± 4.0Å and c by ± 5.0Å, preferably a by ± 1.0Å, b by ± 1.0Å and c by ± 2.0Å. More preferably the variance is no more than a ± 0.7Å, b ± 0.7Å and c ± 1.4Å, for example no more than a ± 0.7Å, b ± 0.7Å an c ± 0.7Å ,and even more preferably no more than a ± 0.2Å, b ± 0.2Å and c ± 0.4Å. These unit cell sizes are believed to define novel and more highly resolved unit cell sizes than has previously been possible in the art.

### Production of crystals.

To obtain crystals according to the present invention, we have found that selective removal of the S1 subunit protein is advantageous. This may be achieved by the use of hydrophobic interaction chromatography column (poros-ET). 30S ribosomal subunits lacking the S1 subunit may suitably be separated from those containing the S1 subunit by running a column using a reverse ammonium sulfate gradient from 1.5M to 0.5M, with 20mM Hepes, pH 7.5, and 10mM acetate. The 30S subunits lacking S1 are eluted first, giving the first major peak. During elution of the 30S peak the ammonium sulfate concentration is maintained at a constant level. Once the 30S peak has eluted the ammonium sulfate concentration is then further reduced to elute the 30S + S1 fraction.

An alternative method for the selective removal of the S1 subunit protein is by preparative sepharose or by gel electrophoresis. Gel electrophoresis may suitably be carried out by first preparing and mixing a 3% acrylamide, 0.5% agarose cylindrical gel, and pouring this gel into a BioRad Prep Cell. 30S ribosomal subunits are then loaded onto the gel and continuously eluted as they emerge form the other end of the gel. The 30S fraction lacking the S1 subunit comes off first, giving the first major peak. The 30S + S1 fraction gives the trailing peak (or shoulder) and can be discarded.

Once the S1 is removed, the crystals may be formed, using suitable conditions. These include the use of 13-17% v/v methyl-2,4-pentanediol in the presence of 200-300 (e.g. about 250) mM KCl, 50-100 (e.g. about 75) mM ammonium chloride, 15-30 (e.g. about 15 or about 25) mM MgCl₂ at a pH of 6.0 - 7.5 (e.g about pH 6.3 - 6.7 such as pH 6.5) in 50 - 150 (e.g. about 100) mM sodium or potassium cacodylate or MES (2-(N-morpholino)ethane sulphonic acid).

In a particular aspect, the conditions may comprise the use of 250 mM KCl, 75 mM NH₄Cl, 25 mM MgCl₂, 6 mM 2-mercaptoethanol in 0.1 M potassium cacodylate or 0.1 M MES (2-N-morpholinoethanesulfonic acid) at pH 6.5 with 13-17% MPD as the precipitant.

The crystals may be grown by any suitable method known as such to those of skill in the art. Suitably, the crystals may be grown over a period of 4-8 weeks at about 4 C. Once crystals are obtained, the antibiotic Z may be soaked into the crystals. The antibiotic may be used in any convenient soluble form at a concentration range of from 10 to 500 µM, preferably from 50 to 100 µM, such as about 80 µM. The structure of the crystals so obtained may be resolved, and crystals which resolve to a resolution of at least 3Å selected. Crystals which resolve to a resolution of at least 3Å obtainable by such a method are a further aspect of the invention.

### Use of structure of Table n.

The determination of the three-dimensional structure of 30S provides a basis for the design of new and specific ligands for 30S. For example, knowing the three-dimensional structure of 30S, computer modelling programs may be used to design different molecules expected to interact with possible or confirmed active sites, such as binding sites or other structural or functional features of 30S.

The high resolution models of the 30S provided by Table n can be used to examine and determine the binding of the antibiotics paromomycin, streptomycin, spectinomycin, tetracycline, pactamycin and hygromycin B to the 30S ribosome, and by using this information, the skilled person in the art can design ligand which may compete with these antibiotics and which can overcome the resistance of bacterial cells to these antibiotics.

A candidate ligand, particular one which acts as an inhibitor molecule may be any available compound. A number of commercial sources of libraries of compound structures are available, for example the Cambridge Structural Database. Such libraries may be used to allow computer-based high throughput screening of many compounds in order to identify those with potential to interact with the active site of a ribosome.

More specifically, a potential ligand capable of modulating 30S activity can be examined through the use of computer modelling using a docking program such as GRAM, DOCK, or AUTODOCK (see Walters et al., *Drug Discovery Today,* Vol.3, No.4, (1998), 160-178, and Dunbrack et al., *Folding and Design,* 2, (1997), 27-42) to identify potential ligands of 30S. This procedure can include computer fitting of potential ligands to 30S to ascertain how well the shape and the chemical structure of the potential ligand will bind to the enzyme.

Also computer-assisted, manual examination of the active site structure of 30S may be performed. The use of programs such as GRID (Goodford, *J. Med. Chem.*, 28, (1985), 849-857) - a program that determines probable interaction sites between molecules with various functional groups and the enzyme surface - may also be used to analyse the active site to predict partial structures of ligands for the site.

Computer programs can be employed to estimate the attraction, repulsion, and steric hindrance of the two binding partners (e.g. the 30S and a potential ligand). Generally the tighter the fit, the fewer the steric hindrances, and the greater the attractive forces, the more potent the potential ligand since these properties are consistent with a tighter binding constant. Furthermore, the more specificity in the design of a potential ligand, the more likely it is that the ligand will not interact with other proteins as well. This will tend to minimise potential side-effects due to unwanted interactions with other proteins.

In one aspect, the above described methods may be used to perform a computer-based method of rational drug design which comprises:
providing the structure of the 30S ribosome defined by the coordinates found in Table n;
providing the structure of a candidate inhibitor molecule;
fitting the candidate to the structure of the 30S to provide a result; and
comparing the result with a structure comprising the 30S of the Table together with the antibiotic coordinate data of said Table.

In the case where Table n is Table 2 (tetracycline), the comparison may be with one or other, or both, bound tetracycline molecules.

The 30S ribosome used in the present invention comprises an additional small protein molecule, Thx, as well as a short sequence of nucleotides designated molecule X. It will be understood that the phrase "the structure of the 30S ribosome as defined by the coordinates of Table n" and the like (where n is any one of 1 to 4) as used above and elsewhere herein is reference to the coordinates defined by atoms of the 16S RNA and proteins S2 to S20 of the Table n, including or not including the Thx and molecule X coordinates, optionally in conjunction with any or all of the metal ions defined by Table n.

The data of Table n indicate that the primary contacts between antibiotic Z and the 30S are mediated by the 16S RNA. Thus in the above aspect of the invention, those of skill in the art may choose to use the data of Table n relating to the 16S RNA and one of the antibiotics Z in the process of drug design. Accordingly, there is also provided a computer-based method of rational drug design which comprises:
providing the structure of the 16S RNA of the 30S ribosome as defined by the coordinates of Tables n;
providing the structure of a candidate inhibitor molecule;
fitting the structure of candidate to the structure of the 16S RNA of the 30S to provide a result; and
comparing said result with a structure comprising the 16S RNA of the 30S of said Table together with the antibiotic structure of said Table.

In an alternative aspect, the method of the invention may utilise the coordinates of atoms of interest of the 30S which are in the vicinity of an antibiotic Z binding region in order to model the pocket in which Z binds. These coordinates may be used to define a space which is then screened "*in silico"* against a candidate inhibitor molecule. Thus the invention provides a computer-based method of rational drug design which comprises:
providing the coordinates of at least one atom of Table n of the 30S ribosome;
providing the structure of a candidate inhibitor molecule;
fitting the structure of candidate to the coordinates of the 30S ribosome provided to obtain a result; and
comparing said result with a structure comprising the coordinates of the 30S ribosome provided and at least one atom from one antibiotic structure of Table n.

In this embodiment, the at least one atom of the 30S ribosome provided will preferably be within a distance of 50, preferably 10 Angstroms of at least one of the atoms of either of the antibiotic molecules described in Table n.

In practice, it will be desirable to model a sufficient number of atoms of the 30S ribosome as defined by the coordinates of Table n which represent a binding pocket. Binding pockets and other features of the interaction of antibiotic Z with the 30S ribosome are described in the accompanying examples. Thus, in this embodiment of the invention, there will preferably be provided the coordinates of at least 5, preferably at least 10, more preferably at least 50 and even more preferably at least 100 atoms such as at least 500 atoms and most preferably at least 1,000 atoms of the 30S ribosome. Of these atoms provided, at least one will preferably be within the distance mentioned above of the antibiotic molecules described in Table n.

Likewise, when a candidate is fitted to the selected coordinates of the 30S ribosome the comparison with antibiotic is preferably made by reference to at least 3, such as at least 5, for example at least 8, more preferably at least 16 of the atoms of either of the antibiotic Z structure provided in Table n.

In another aspect, the method of the invention may utilise a sub-domain of interest of the 30S which is in the vicinity of a antibiotic binding region. Thus, the invention provides a computer-based method of rational drug design which comprises:
providing the coordinates of at least a sub-domain of the 30S ribosome;
providing the structure of a candidate inhibitor molecule; fitting the structure of the candidate to the coordinates of the 30S ribosome sub-domain provided to obtain a result; and
comparing said result with a structure comprising the coordinates of the 30S ribosome of same sub-domain provided and at least one atom from the antibiotic Z structure of Table n.

In a further aspect, the accompanying examples and drawings show the specific sites of interaction of the antibiotic Z with the 30S ribosome. These data may be used to design ligands which interact with at least one of the sites of interaction of each identified antibiotic Z, and preferably at least 50% of the sites of interaction identified for each separate antibiotic Z in each of Figures 1 to 6. Such ligands may be designed by providing atomic coordinate data for at least one of the following nucleic acid or amino acid residues of the 30S:
Group I: G1405, A1408, C1490, G1491, A1493, G1494 and U1495;
Group II: G1064, C1066, G1068 and C1192;
Group III: U14, C526, G527, A913, A914, C1490, G1941 and S12Lys45;
Group IV: A965, G966, G1053, C1054, C1195, U1196, G1197 and G1198;
Group V: U244, A892 and C893;
Group VI: G693, A694, C788, C795, C796, S7Gly81 and optionally U1540;
Group VII: C1403, G1405, G1494, U1495, C1496 and U1498,
providing a potential ligand, and
fitting said ligand to the 30S to determine the interaction of the ligand with at least one chemical group present in the nucleic acid or amino acid residue of the selected group.

Preferably at least half the members of each group are used, and more preferably from half to t members of each group are used, where t represents a number which is more than half and at least T, preferably T-1 and more preferably T-2 where T is the total number of members of each group, subject to the requirement that t is greater than T/2 (i.e. for group II 2, 3 or 4 members may all be used, and for group V, 2 or 3 members may be used).

In another aspect, in place of *in silico* methods, high throughput screening of compounds to select compounds with ribosome binding activity may be undertaken, and those compounds which show ribosome binding activity may be selected as possible candidate inhibitors, and further crystallized with 30S (e.g. by co-crystallization or by soaking) for x-ray analysis. The resulting x-ray structure may be compared with that of Table n for a variety of purposes. For example, where the contacts made by such compounds overlap with those may by antibiotic Z, novel molecules comprising residues which contain contacts of both Z and the other inhibitor may be provided.

Having designed or selected possible binding ligands, these can then be screened for activity. Consequently, the method preferably further comprises the further steps of:
obtaining or synthesising the potential ligand; and
contacting the potential ligand with 30S to determine the ability of the potential ligand to interact with 30S.

More preferably, in the latter step the potential ligand is contacted with 30S under conditions to determine its function, for example in a cell free translation system.

Instead of, or in addition to, performing such an assay, the method may comprise the further steps of:
obtaining or synthesising said potential ligand;
forming a complex of 30S and said potential ligand; and
analysing said complex by X-ray crystallography to determine the ability of said potential ligand to interact with 30S.

Detailed structural information can then be obtained about the binding of the potential ligand to 30S, and in the light of this information adjustments can be made to the structure or functionality of the potential ligand, e.g. to improve binding to the active site. Steps c. to e. may be repeated and re-repeated as necessary.

Another aspect of the invention includes a compound which is identified as an ligand of 30S by the method of the above aspects of the invention.

In another aspect, the invention provides a method of analysing a 30S-ligand complex wherein the ligand has been obtained by the methods of the invention described above, comprising the steps of (i) cocrystallising the 30S with the ligand or soaking the ligand into crystals of the 30S; (ii) collecting X-ray crystallographic diffraction data from the crystals of the 30S-ligand complex and (iii) using the three-dimensional structure of 30S of Table n, or at least one sub-domain thereof, to generate a difference Fourier electron density map of the 30S-ligand; and (iv) modelling the ligand in the difference Fourier electron density.

Therefore, 30S-ligand complexes can be crystallised and analysed using X-ray diffraction methods, e.g. according to the approach described by Greer et al., *J*. *of Medicinal Chemistry,* Vol. 37, (1994), 1035-1054, and difference Fourier electron density maps can be calculated based on X-ray diffraction patterns of soaked or co-crystallised 30S and the solved structure of uncomplexed 30S. These maps can then be used to determine the structure of the ligand bound to the 30S and/or changes the conformation of 30S.

Data obtained from a ligand bound to 30S may be used to improve the ligand, for example by adding or removing functional groups, substituting groups or altering its shape to obtain improved candidates, which may then be screened, solved in complex as described herein above, in an iterative process.

Electron density maps can be calculated using programs such as those from the CCP4 computing package (Collaborative Computational Project 4. The CCP4 Suite: Programs for Protein Crystallography, *Acta Crystallographica,* D50, (1994), 760-763.). For map visualisation and model building programs such as "O" (Jones et al., *Acta Crystallograhy,* A47, (1991), 110-119) can be used.

The high resolution data provided herein allows those of skill in the art who have obtained structures of worse resolution of the 30S to refine such structures in the light of the data of Table n. Thus in a further aspect, the invention provides a method for modelling a structure of a 30S ribosome which comprises providing an atomic model of a structure at a resolution of worse than 3Å (e.g. a resolution of worse than 5 Angstroms, such as 5-12 Å), comparing the structure obtained with the data of Table n, and refining said model obtained to resolve the structure in order to provide a higher resolution structure. Such a process will be useful for the refinement of a 30S itself, or the 30S in various functional states as part of the 70S ribosome (e.g. bound to mRNA, elongation factors or the like).

Such a method will be useful in providing the structure of the 30S ribosome from other bacterial sources, since the overall secondary and tertiary structure of such ribosomes will be highly conserved in comparison to the *T*. *thermophilus* structure provided herein. The data provided herein may be used to in a process of modelling the 30S of other species ab initio by homology modelling using energy minimization criteria.

By providing such computer readable media, the atomic coordinate data can be routinely accessed to model 30S or a sub-domain thereof. For example, RASMOL is a publicly available computer software package which allows access and analysis of atomic coordinate data for structure determination and/or rational drug design.

On the other hand, structure factor data, which are derivable from atomic coordinate data (see e.g. Blundell et al., in *Protein Crystallography,* Academic Press, New York, London and San Francisco, (1976)), are particularly useful for calculating e.g. difference Fourier electron density maps.

In another aspect, the present invention provides systems, particularly a computer systems, intended to generate structures and/or perform rational drug design for 30S ligand complexes, the systems containing either (a) atomic coordinate data according to Table n, said data defining the three-dimensional structure of 30S or at least one sub-domain thereof, or (b) structure factor data for 30S, said structure factor data being derivable from the atomic coordinate data of Table n.

Mutant strains resistant to the action of these antibiotics can arise through mutation of a protein subunit of the 30S or through mutation or modification in the 16S RNA (e.g. 2'O-methylation), or modification (e.g. acetylation) of the antibiotic). The sites of mutations in some cases are known or can be identified. Where such sites are identified through, for example, primary sequence data, the invention provides a means to model the structure of the mutants.

There is thus provided a method which comprises providing the structure of the 30S ribosome of Table n, changing one amino acid or nucleotide of said structure to provide a mutant 30S, and modeling the structure of the mutant 30S to provide a structure of the mutant. The mutant may be used in the manner described above for the wild type, e.g. stored in computer readable form, modeled to provide ligands, and the like. The modeling may be based upon the predicted behavior of the atoms of the changed amino acid based upon its interaction with the surrounding atoms in the model provided herein.

This process may be iterative, e.g. to produce successive mutations into the 30S structure, for example 2, 3, 4, or 5 to 10 mutations.

Regions of 30S which may be subject to this aspect of the invention include those regions identified in the accompanying examples as regions of the 30S involved in binding to antibiotics.

In a further aspect, the present invention provides a means to solve or interpret electron density maps of the whole 70S ribosome at low or high resolution, and thus solve the structure of the whole 70S ribosome.

In particular, the invention provides a method for the determination of the structure of a bacterial 70S ribosome which method comprises
(a) crystallising the 70S of said species to obtain a crystal;
(b) performing X-ray crystallography on said crystal to obtain X-ray diffraction data;
(c) providing the structure data of Table 1; and
(d) using molecular replacement to calculate an electron density map of the 70S.

The crystallisation step (a) is optionally performed with an antibiotic Z, either in a co-crystallisation or by soaking the antibiotic following crystal formation. Thus the calculated electron density map may be that of the 30S - antibiotic complex.

The invention is illustrated below by the following examples, their accompanying Figures and Tables. In Table n there is shown in each row Atom number, element type, residue (amino acid, nucleotide, etc), number in molecule (for proteins N to C terminal direction, for nucleic acid 5' to 3' direction), X, Y and Z co-ordinates, occupancy, B factor (Å²) and an identifier for the member of the 30S.

Throughout the accompanying example, we use the numbering system for *E. coli* 16S RNA, as well as the standard helix numbering, denoted H1-H45, for the secondary structure elements [19] with some modifications as shown in Figure 1. The most significant differences between the *E. coli* and *T. thermophilus* sequences are a shorter H6 and H10, and insertions in H9 and H33a. Any insertions in *T. thermophilus* relative to E. coli are indicated in the coordinates with an insertion letter after the nucleotide number, following the practice for tRNA.

### EXAMPLES

### Example 1 - Crystallization of Paromomycin, Spectinomycin and Streptomycin to the 30S Ribosome.

### Crystallization of the 30S.

Because we observed that the 30S crystals completely lacked ribosomal protein S1, care was taken to remove S1 selectively from the 30S prior to crystallization. Crystals were obtained in 13-17% MPD over a range of pH in the salt and magnesium conditions described by Trakhanov et al [3]. The crystals were largest and most reproducibly obtained at a pH of 6.5 in 0.1 M cacodylate or MES buffer. Crystals took approximately 6 weeks at 4 C to grow to their maximum size. The largest crystals, which were required for high resolution data collection, grew to a size of 80-100 x 80-100 x 200-300 microns. The activity of redissolved crystals in poly(U)-directed protein synthesis was comparable to that of freshly isolated 30S subunits.

### Data collection.

Crystals were transferred to 26% MPD by vapor diffusion in two steps over a period of 6 days. All crystals (except for those soaked in osmium hexammine or osmium pentammine) also contained 1 mM cobalt hexammine in the cryoprotectant. Crystals were flash-cooled by plunging into liquid nitrogen, and data collection was done in a cryostream at 90-100 K.

A large fraction of crystals was screened at beamlines 9.6 or 14.1 at the SRS at Daresbury Laboratories, using two short exposures at least 40 degrees apart. These crystals were then analyzed for diffraction limits, cell dimensions and mosaic spread. Only crystals of similar cell dimensions and with reasonable mosaic spread were used for data collection.

Potential derivatives were screened on beamlines X25 at the NSLS at Brookhaven National Laboratory and BM-14 at the ESRF (Grenoble). Data to about 4.5Å were obtained from X25. High resolution data were collected at SBC ID-19 at the APS in Argonne National Laboratory, and ID14-4 at the ESRF. In all cases, derivative data were collected at the peak of the fluorescence at the LIII edge to maximize anomalous differences. At X25 and SBC ID-19, the kappa goniostat was used to rotate precisely about a mirror plane so that small anomalous differences could be measured accurately. Each crystal typically yielded 3-10 degrees of data. Data were integrated and scaled using HKL-2000 [10].

### Structure determination.

Previously determined phases at 5.5 Å [9] were used to locate heavy atom sites using anomalous difference Fourier maps. Initially, these sites were used for phasing to 3.35 Å using the program SOLVE [11], followed by density modification with SOLOMON [12], using the procedure implemented in SHARP [13]. Optimization of the various parameters in the procedure was required to obtain interpretable maps. The RNA and some of the proteins were built using the SOLVE maps. The sequence of Thermus thermophilus 16S RNA [14] was used for the structure. For proteins, a combination of previously published sequences and new ones from the Göttingen Thermus genome sequencing project were used. Improved maps were obtained by calculating experimental phases to 3.2Å using SHARP followed by density modification and phase extension to 3.05Å with DM [15]. The improved maps allowed us to build all the ordered parts of the structure. The model was built using O [16], and refined using the program CNS [17]. Maximum likelihood refinement was used, initially with both amplitudes and experimental phase probability distributions to 3.35Å, and subsequently with amplitudes to 3.05Å.

### Results

The 30S subunit from Thermus thermophilus consists of a 1522 nucleotide 16S ribosomal RNA [14] and 21 associated proteins, of which 20 have known counterparts in E. coli. Protein S21 is not present in Thermus, and protein S1 has been removed from the 30S prior to our crystallization. In addition, a 26 residue peptide, Thx, is present in Thermus 30S subunits [18].

Experimentally phased maps clearly showed main chain density for RNA and protein, individual bases (which were often of sufficient quality to distinguish purines from pyrimidines), and large well-ordered side chains of proteins. These maps were used to build 16S RNA and the previously unknown proteins S2, S3, S9, S10, S11, S12, S13, S14 and Thx. In addition, regions that were disordered in isolated structures or had changed significantly were also built. This often consisted of significant portions of the N- and C-terminal tails of the proteins, sometimes including entire domains that were unfolded in isolation. Proteins with small cores and long loops, such as S16 and S17, had to be substantially rebuilt, since these loops were generally disordered in the solution NMR structures. Finally, the entire structure was rebuilt after an initial round of refinement. Our current model consists of nucleotides 5-1511 of Thermus thermophilus 16S RNA (corresponding to 5-1534 of E. coli 16S RNA) and all of the ordered regions of the associated 20 proteins. The current model has been refined against 3.05Å data with a conventional R-factor of 0.213, a free R-factor of 0.256 and good geometry. For the proteins, 94% of the residues were in the core or allowed regions of the Ramachandran plot, 3.9% in the generously allowed region and 1.8% in the disallowed region.

### Paromomycin.

Paromomycin is a member of the aminoglycoside family of antibiotics which increases the error rate of the ribosome. This family is thought to reduce the dissociation rate of A-site tRNA from the ribosome. Recent experiments suggest that it affects both initial selection and proofreading. Crystals were obtained and the data collected is provided in Table 1. Figure 1 shows the interactions between 16S RNA residues and paromomycin.

Paromomycin binds in the major groove of H44 in a location that is in agreement with mutagenesis and protection data. It is also in general agreement with an NMR structure of paromomycin bound to an RNA fragment corresponding to its binding site [20]. Contacts are shown in Figure 1. Ring IV contacts the backbone of both sides of helix in an orientation that differs from the NMR structure, while ring III makes only weak contacts with the RNA. Ring II forms tight interactions with both bases and backbone of the RNA, while Ring I inserts into the RNA helix and helps flip out bases A1192 and A1493 when compared to the structure in the absence of paromomycin. Ring I mimics a nucleotide base, stacking against G1491 and hydrogen-bonding with A1408. In addition it forms a tight H bond interaction with the phosphate backbone of A1493 which helps lock the flipped out bases in place. Except as noted, many of the interactions are similar to those reported in the NMR structure [20] although the bases are flipped out to a far greater degree, and consequently, we do not see a base pair between A1408 and A1493.

We have modelled the codon and anticodon in the A site using a superposition of the 7.8Å structure of the 70S ribosome with tRNA and mRNA bound. The flipped out bases point directly into the A site and are positioned to interact with the minor groove of the helix formed by the codon-anticodon interaction. It is probable that the A-site codon-anticodon helix must undergo some degree of rotation during or after GTP hydrolysis by EF-Tu, in a conformational change to a proofreading state of the decoding site. However, there are rather strict covalent and steric constraints on the A-site anticodon and especially the codon, which is covalently attached to both the P-site codon and downstream message. Thus, despite some rotational uncertainty in the orientation of the codon-anticodon helix, it appears unlikely that the 1492-1493 bases could interact with any portion of the codon-anticodon helix other than its minor groove, though interactions with other portions of the A-site tRNA anticodon loop are not ruled out. This model provides clues as to how paromomycin increases the affinity of the A site for tRNA. It seems likely that in the absence of paromomycin some energy is required to flip out A1492-A1493 so they can contact the tRNA, and presumably this energetic cost is compensated by the formation of favourable interactions with tRNA. By binding to H44, paromomycin forms a structure in which these bases are already flipped out, thus reducing the energetic cost of both cognate and non-cognate tRNA binding and increasing tRNA affinity for the A site.

This structure is in general agreement with a previously proposed model in which A1492 and A1493 would make contact with the minor groove of the mRNA-tRNA duplex [21]. In that model, it was suggested that these bases hydrogen bond with the 2'-OH of the message. Given the rotational uncertainty of the positioning of the A-site tRNA in our model, we cannot determine that these adenines hydrogen-bond to the message. However, two scenarios appear possible, within the limits of the model. The adenines may hydrogen-bond to 2' OH groups of only the tRNA anticodon stem-loop, or they may hydrogen-bond to 2'OH groups of both the tRNA and the message. Both possibilities are attractive because they offer a direct explanation for increased affinity (and lower dissociation rate) of tRNA in the presence of the antibiotic. Finally, the degree to which the bases are flipped out in our structure allows a possible reconciliation of the proposal that the mRNA binds in the major groove of H44 23 with the notion that A1492 and A1493 inspect the minor groove of the codon-anticodon interaction.

Rings I and II of paromomycin are found in a number of other antibiotics including gentamycin. An NMR structure of gentamycin bound to the same fragment of H44 showed that these two rings interact with RNA in the same way as in paromomycin [20]. This suggests that other aminoglycosides that bind to the decoding centre on H44 induce errors in translation by the same mechanism as paromomycin.

### Spectinomycin

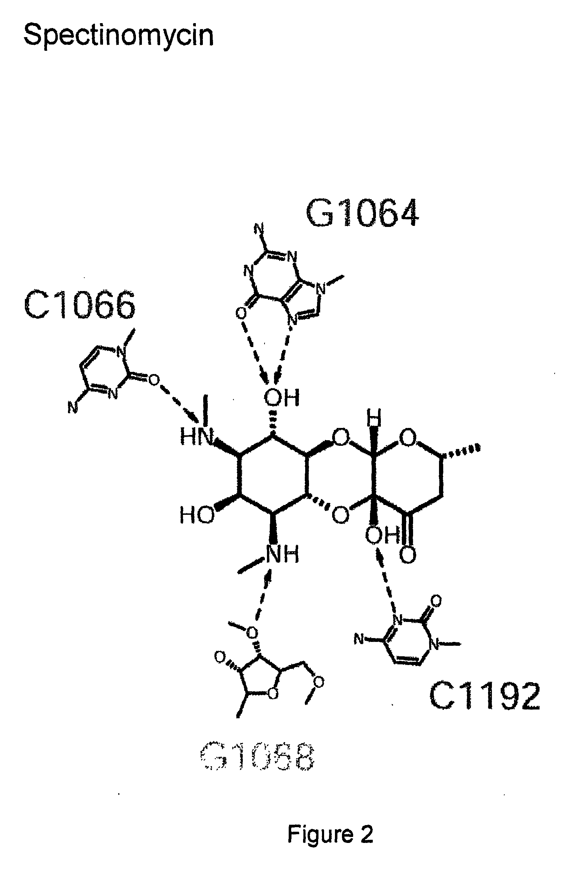

In contrast to the flexible aminoglycosides, the fused ring system in spectinomycin makes it a rigid molecule. It binds in the minor groove at one end of H34. It makes a single contact with a backbone phosphate and makes hydrogen bonds to a number of bases (Figure 2). The most interactions are made with G1064 and C1192, consistent with protection studies [22] and mutagenesis data which showed that any combination of substitutions at these bases gave resistance to spectinomycin [23]. These two bases are held too far apart to form Watson-Crick base pairs, but are able to make a single hydrogen bond.

A loop of S5 and part of H28 of 16S RNA approach the spectinomycin binding site, but in this state do not make direct contacts with it. It is possible, however, that in other conformations of the 30S, spectinomycin is in more direct contact with these regions.

A superposition of the A, P and E site tRNAs from the 70S ribosome onto our 30S structure shows that a number of highly specific contacts from the head stabilize these tRNAs. A movement of tRNA from one site to the other must necessarily involve movement of elements of the head. Such movements would involve H34 and a possible rearrangement of the connections between it and helices 35 and 38. The structure suggests that the rigid spectinomycin molecule binds near this pivot point of the head and sterically blocks movement although it is also possible that it acts to stabilize the upper stem of H34 [23]. As mentioned above, mutations in S5 that cause resistance to spectinomycin [24] do not make direct contacts with the antibiotic. Rather, they map to a loop that stabilizes the interaction between H1 and the H35-H36 region which is directly connected to H34. An attractive hypothesis is that the mutants destablize this interaction, and by thus removing the network of interactions that stabilizes the conformation of the head to the body via S5, allows it to move even when spectinomycin is bound.

### Streptomycin

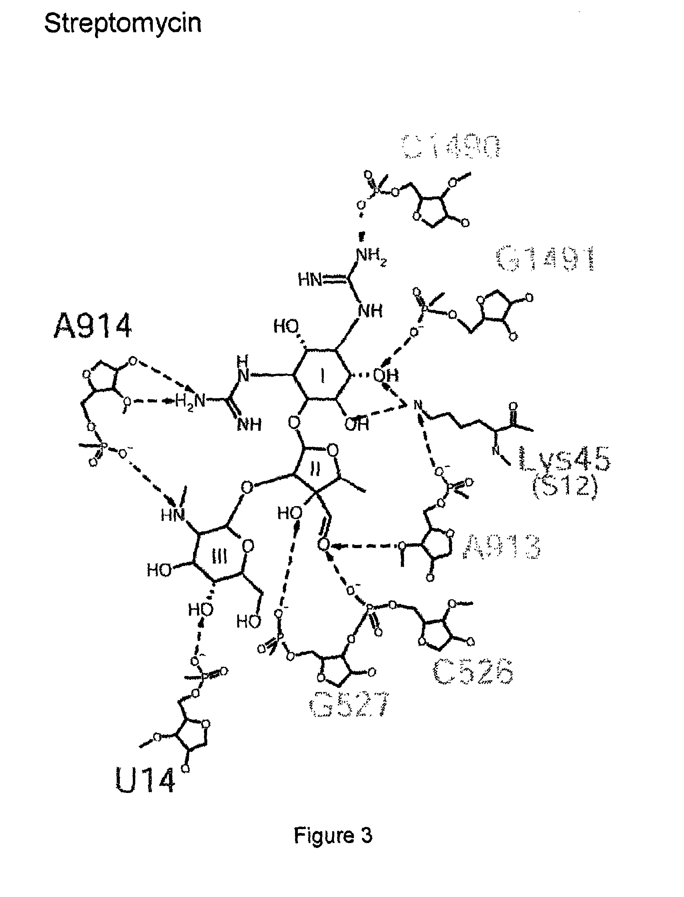

Unlike paromomycin, which can bind to an isolated fragment of ribosomal RNA, streptomycin is tightly bound to the phosphate backbone of 16S RNA from 4 different parts of the molecule via both salt links and hydrogen bonds (Figure 3). It also makes contact with a lysine (K45) from ribosomal protein S12. The four regions of 16S RNA (1490, 915, 526, and 13) had all been implicated in streptomycin binding on the basis of protection [22], crosslinking [25] and mutagenesis data [26-28] (reviewed in [29]).

It has been proposed that translational fidelity involves a switch between two states of the ribosome, an error prone or ram (885) state, characterized by nucleotides 910-912 pairing with 885-887, and a restrictive or hyperaccurate (888) state in which 910-912 pair with 888-890 [30].

The structure of the 30S reported here, like that of the 70S ribosome at 7.8 Å [31] is in the 885 pairing configuration, and hence presumably in the ram state. The tight interactions described above suggest that streptomycin preferentially stabilizes this form. The 888 state A site has a low tRNA affinity, while the 885 state has a higher affinity [30, 32]. Therefore by stabilizing the 885 state streptomycin would be expected to increase initial binding of non-cognate tRNAs. The preferential stabilization of the 885 state would also make the transition to the 888 state more difficult, thereby also affecting proofreading. Thus our results offer a structural rationale for the observed properties of streptomycin.

This stabilization of the 885 state by streptomycin suggested by our structure can explain much of the genetic data on the antibiotic. Mutations in S12 lead to a hyper-accurate phenotype [32-38] (reviewed by [39]) . A weak phenotype manifests itself as streptomycin resistance, where as a strong phenotype (often the result of multiple mutations) leads to streptomycin dependence. Most of these mutants are to varying degrees more hyperaccurate and slower than wild type ribosomes, consistent with destabilization of the 885 state with respect to the 888 state.

All the mutations in S12 map to the a protein loops that connect and hold in place the 908-915 and 524-527 regions, with the exception of one mutant K56 (E.coli K53) which contacts H44 (Figure 3). Thus S12 stabilizes the same region that is stabilized by streptomycin. In the resistance mutations, the 885 state is destabilized sufficiently so that the additional stabilization induced by streptomycin does not trap the ribosome in this state. In the streptomycin dependent mutants, the 885 state is so destabilized that the 888 (hyperaccurate) form predominates and protein synthesis becomes very slow. Streptomycin can then help stabilize the 885 state sufficiently to restore the balance between the two states and help restore translation.

The K45R (E.coli K42) mutation is resistant to streptomycin but is not hyperaccurate [39]. K45 forms a salt bridge with phosphate A913 and thus contributes to stabilization of the 885 state. It also makes direct hydrogen bonding contacts to two OH groups on streptomycin (Figure 3). Mutation of this lysine to arginine, would disrupt the hydrogen bonding and thereby reduce the affinity of the 30S for streptomycin, leading to resistance. However, the mutation would leave the salt bridge intact, so that the 885 form is not destabilized and thus translation remains normal.

A number of mutations in rRNA also lead to hyperaccuracy [26, 27, 40-43]. Some of these nucleotides are involved in hydrogen bonding interactions in regions close to the streptomycin binding site. Thus the mutations disrupt interactions that help to stabilize the 885 state. Others such as A915 make no contacts with any other bases. It is possible that mutation of this base leads to more favourable contacts in the 888 state, thus acting by stabilizing the 888 state rather than destabilizing the 885 state.

The ram mutations lead to error-prone ribosomes and are generally found as suppressors of streptomycin resistance. These mutations in S4 [35, 44, 45] and S5 [12] can counter the effect of hyperaccurate mutations in S12 (reviewed by [39]). All ram mutants in S4 and S5 map near the interface between the two proteins with the exception of S52 (E.coli S49) which makes a direct hydrogen bond to the backbone of rRNA. At lower resolution [9], it appeared that the ram mutations destablized the S4-S5 interface. However, at atomic resolution, we see that two of the mutations V56 (E.coli V53) in S4 and G99 (E.coli G103) in S5 are not in contact with the other protein, and it is not obvious from our structure how they would affect stability of the ram state. In fact, there is a cleft between the two proteins which could close up on the 885-888 transition. This leads us to suggest that these residues (and perhaps the corresponding surfaces of S4 and S5) are involved in intimate contacts in the 888 state, and these contacts would be disrupted by the ram mutations. In this model, ram mutations act by destabilizing the 888 state, and thus shifting the equilibrium to the error prone 885 state. The observation that ram mutations increase the affinity of ribosomes for streptomycin [46] is consistent with this model, since the ribosome would be preferentially in the 885 form. A definitive test of this model must await an atomic resolution structure of the 888 form. Nevertheless, our results provide a structural basis for the notion that a delicate balance exists between the 885 and 888 states for optimal translation, and also explains how disruption of this balance leads to the various phenotypes observed.

### Example 2 - Crystallization of Tetracycline to the 30S Ribosome.

Tetracyclines are antibiotics of broad specificity and have been used since the 1940's against a wide range of both Gram-negative and Gram-positive bacteria [47]. These drugs were the first so-called 'broad-spectrum' antibiotics and have been used extensively in both human and veterinary medicine. However, in later years, the widespread use of tetracyclines has been limited by the emergence of significant microbial resistance to these antibiotics. Tetracyclines bind to the 30S ribosomal subunit [48] where it affects exclusively the binding of aminoacylated tRNA to the A-site [49]. There is no effect on the binding of tRNA to the P-site nor on the fidelity of translation [50]. Consistent with the inhibition of tRNA binding to the A-site during translation, tetracycline also prevents binding of both release factors RF-1 and 2 during termination, regardless of the stop codon [51]. In contrast to most antibiotics, resistance to tetracycline is usually not caused by mutations in 16S RNA or ribosomal proteins, but by the presence of several external protein factors which apparently mimic the structure and function of the elongation factors [52-54].

The crystal structure of tetracycline, or 4-(dimethylamino)-1,4,4a,5,5a,6,11,12a-octahydro-3,6,10,12,12a-pentahydroxy-6-methyl-1,11-dioxo-2-naphtacenecarboxamide, in complex with the 30S ribosomal subunit was determined at 3.0A resolution. 30S crystals were prepared as described in example 1 above and soaked post crystallisation in 80µM tetracycline. X-ray diffraction data were collected at beamline ID14-4 at the European Synchrotron Radiation Facility in Grenoble, France. The location of the antibiotic within the 30S subunit was identified from difference Fourier maps calculated after a few rounds of maximum-likelihood based refinement of the native 30S structure against the measured structure factor amplitudes.

We have found two strong binding sites for tetracycline within the 30S subunit, one located near the acceptor site for aminoacylated tRNA (the A-site) between the head and the body and another which is present at the interface between three domains in the body of the subunit. The discovery of more binding sites is not surprising, since tetracycline is known to have one primary binding site on the 30S in addition to multiple secondary sites on both subunits [55]. In its primary binding site within the 30S, tetracycline binds exclusively to the 3' major domain in the upper part of the crevice between the head of the 30S and the shoulder and right above the small gap between the stem-loop of H18 of the 5' domain and the long H44 of the 3' minor domain that constitutes the binding site for aminoacylated tRNA. The molecule fits into a small pocket created by residues in H34 that deviate from the canonical A-form RNA double helical conformation in combination with a part of the small H31 stem-loop structure. The contacts to H31 are quite tenuous, however, and the binding of the antibiotic to 16S RNA seems to depend almost exclusively on the interaction with H34. In contrast to what has been proposed earlier, there are no proteins involved in the primary binding of tetracycline. The second binding site of tetracycline that we observe clearly in the difference maps (although perhaps not as clearly as the primary binding site), is located in the body of the subunit, in close proximity to the penultimate H44 and sandwiched between the functionally important H27 in the central domain and the very top of H11 in the 5' domain of 16S RNA. The binding site it confined on one side by a major groove of H27 (residues 891-894:908-911) and the edge of the curved H11 (residues 242-245). The bulged-out base U244, which reaches across and makes a non Watson-Crick bases pair with C893 in H27 forms the bottom of the binding site. Again, all interaction between the antibiotic and the ribosome is mediated by the RNA, however, the long N-terminal extension of S12 comes very close to the tetracycline (~ 8A, Arg19). The binding pocket is approximately 14A wide and 7A deep.

### Example 3 - Crystallization of Pactamycin to the 30S Ribosome.

Pactamycin was isolated from *S*. *pactum* as a potential new human antitumor antibiotic and is a potent inhibitor of translation in both eukaryotes and prokaryotes [56]. It is believed to inhibit the initiation process, i.e. the initiation factor mediated binding of fMet-tRNA to the ribosome [57], by sequestering the initiation complex in the A-site [58]. This effectively prevents the formation of entire 70S ribosomes and thus halts the translation. The effect upon binding is similar for prokaryotes and eukaryotes [59].

The crystal structure of pactamycin, or 2-hydroxy-6-methylbenzoic acid [5-[(3-acetylphenyl)amino]-4-amino-3-[[(dimethylamino)-carbonyl]amino]-1,2-dihydroxy-3-(1-hydroxyethyl)-2-methylcyclopentyl]methyl ester, in complex with the 30S ribosomal subunit was determined at 3.1Å resolution. 30S crystals were prepared as described as above and soaked post crystallisation in 80µM pactamycin. X-ray diffraction data were collected at beamline ID14-4 at the European Synchrotron Radiation Facility in Grenoble, France. The location of the antibiotic within the 30S subunit was identified from difference Fourier maps calculated after a few rounds of maximum-likelihood based refinement of the native 30S structure against the measured structure factor amplitudes.

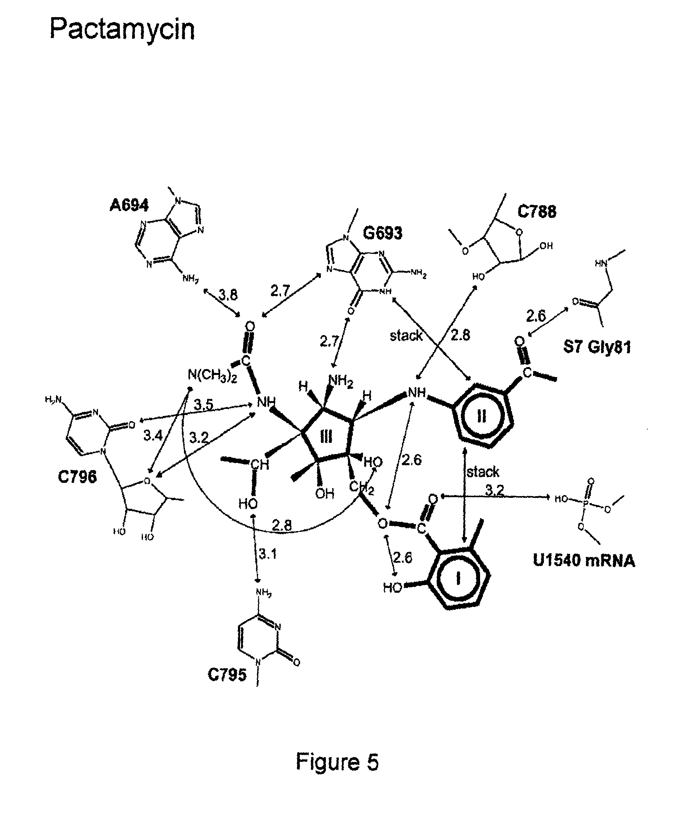

Pactamycin binds to the upper part of the platform, very close to the cleft in the subunit that is responsible for binding of the three tRNA molecules. We have only found a single strong binding site of pactamycin in the 30S. The antibiotic interacts primarily with residues at the apices of the H23b stem loop in the central domain of 16S RNA in addition to a couple of bases from the nearby H24a (Figure 5). The site of binding is very close to the 3' minor domain and the ultimate H45, but there is not direct interaction with this region. In its binding to the RNA, pactamycin extends the stacking of bases in the tetra loop of H23b and mimics RNA both with respect to the bases and the sugar phosphate backbone. In this region, The H24a loop forms a regular helical stem loop to which the H23b stem loop is attached with interactions mainly between bulging bases in H23b and the backbone of H24a. The bases near the apices of H23b curve around and pack into the major groove of H24b, and this trend is extended by two "bases" by a single pactamycin molecule. The antibiotic folds up so that the two distal 6-carbon rings are stacked against each other like nucleotides with the 5-carbon in between resembling a sugar ring. The NCON(CH₃)₂ extension on the central ring even to some extent mimics the phosphate ester moiety of RNA. The nearest proteins are S7 and S11, and there appears to be a weak hydrogen bond to the backbone carbonyl of Gly81 of S7.

In Figure 5 we have indicated an interaction with a residue "U1450mRNA". From our data this residue is not continuous with the main 16S RNA sequence, but appears as part of a stretch of mRNA which appears in the crystal. However, it is believed that it represents the very end (3' end) of 16S RNA, based on sequence, thus it has been given sequence numbers from the 16S in this figure (1539-1544). However, in the table of coordinates, these residues have been separately numbered 1-6, the coordinates being shown immediately following those of the 16S RNA.

Even though pactamycin has been described as binding primarily to the ribosomal P-site [57, 60], the observed protections for this antibiotic can be regarded as pertaining rather to the E-site. This notion is in more agreement with the present structure, in which the two "bases" of pactamycin coincide with the two last bases of the E-site codon of mRNA as observed in the native 30S structure [61]. In fact, pactamycin together with the first base in the E-site codon of the native structure form a triplet codon mimic in approximately the right position for interaction with an E-site tRNA. However, in the antibiotic bound structure, the actual position of the bases in the E-site codon is shifted remarkably, in a way that precludes a possible interaction with an E-site bound tRNA. In the native 30S structure, a kink is observed in the backbone of the messenger RNA at the interface between the P- and E-sites, however, the overall path of the mRNA is still relatively straight and leads between the long 73-90 beta hairpin of S7 and the stem loops of H23 and H24a of the platform. In the pactamycin-bound structure, however, the mRNA in the E-site is pushed towards the back of the subunit, and in between H28 of the head and the hairpin of S7. This is a remarkable distortion that comprises on average 12.5A for the last of the bases in the E-site codon.

### Example 4 - Crystallization of Hygromycin B to the 30S Ribosome.

Hygromycin B is a monosubstituted 2-deoxystreptamine-containing aminoglycoside antibiotic originally isolated as a secondary antibiotic substance from *S*. *hygroscopicus* [62]. It is an unusual aminoglycoside antibiotic in that it is active against both prokaryotic and eukaryotic cells and differs in structure from other aminoglycosides [63]. The drug works primarily by inhibiting the translocation step of elongation [63-65] and to a lesser extent causes misreading of messenger RNA [50, 65]. The antibiotic affects EF-2 (EF-G) mediated translocation of A-site bound tRNA to the P-site in eukaryotes, but does not affect either binding of the factor to the ribosome or the hydrolysis of the bound GTP, a process that has been shown to be separate from translocation [63]. The inhibition of translocation is accompanied by an increase in the affinity of the A-site for aminoacylated tRNA [50].

The crystal structure of hygromycin B, or O-6-amino-6-deoxy-L-glycero-D-galacto-heptopyranosylidene- (1→2-3) -O-β-D-talopyranosyl- (1→5)-2-deoxy-N³-methyl-D-streptamine, in complex with the 30S ribosomal subunit was determined at 3.1A resolution. 30S crystals were prepared as described above, and soaked post crystallisation in 80µM hygromycin. X-ray diffraction data were collected at beamline ID14-4 at the European Synchrotron Radiation Facility in Grenoble, France. The location of the antibiotic within the 30S subunit was identified from difference Fourier maps calculated after a few rounds of maximum-likelihood based refinement of the native 30S structure against the measured structure factor amplitudes.

Hygromycin B has a single clear binding site within the 30S consistent with the finding that it has a monophasic effect on translation [66]. It binds close to the very top of the long, penultimate helix 44 of 16S RNA, in a region that contains the A-, P-, and E-site tRNA binding sites. The antibiotic is in contact only with 16S RNA (not any proteins), and only with helix 44. In fact, it is located in the major groove of the helix, very close to the helical axis, and thus surrounded by residues from both RNA strands in the region 1490-1500 and 1400-1410. Hygromycin B almost exclusively contacts the bases, as opposed to the backbone, of RNA, and would on this basis be expected to be highly sequence-specific. The nearest protein is S12, which is known to be important in decoding, but is more than 14A away from the hygromycin binding site. Binding of hygromycin B does not seem to induce any significant alterations in the structure of RNA, and appears to be governed by strong base-specific hydrogen-bonds spanning more than three sequential bases in one strand of helix 44. This is possible because the structure of the three-ring antibiotic is unfolded in its binding site within the 30S and thus makes the molecule about 13Å long.

Hygromycin B binds to the 30S in an important functional region which is also the target for other antibiotics such as paromomycin and gentamycin. Both these antibiotics bind further down helix 44 than does hygromycin and thus affect adenosines A1492 and A1493 which have been implicated as crucial in decoding. Interestingly, Ring II of paromomycin, which is also found in other aminoglycoside antibiotics including gentamycin, adopts an almost identical orientation as Ring I of hygromycin, only about 3A further down the helix, or exactly what corresponds to one residue. This indicates that this type of six-ring is an important general means of antibiotic binding, since abolishment of the interaction with RNA (in the case of hygromycin) leads to resistance [67, 68].

References:
1. Garrett, R. A. et al. (eds.) The Ribosome. Structure, Function, Antibiotics and Cellular Interactions (ASM Press, Washington, D.C., 2000).
2. von Böhlen, K. et al. Characterization and preliminary attempts for derivatization of crystals of large ribosomal subunits from Haloarcula marismortui diffracting to 3 Å resolution. J. Mol. Biol. 222, 11-15 (1991).
3. Trakhanov, S. D. et al. Crystallization of 70 S ribosomes and 30 S ribosomal subunits from Thermus thermophilus. FEBS Lett. 220, 319-322 (1987).
4. Glotz, C. et al. Three-dimensional crystals of ribosomes and their subunits from eu- and archaebacteria. Biochem. Int. 15, 953-960 (1987).
5. Yonath, A. et al. Characterization of crystals of small ribosomal subunits. J. Mol. Biol. 203, 831-834 (1988).
6. Yusupov, M. M., Tischenko, S. V., Trakhanov, S. D., Ryazantsev, S. N. & Garber, M. B. A new crystalline form of 30 S ribosomal subunits from Thermus thermophilus. FEBS Lett. 238, 113-115 (1988).
7. Yonath, A. et al. Crystallographic studies on the ribosome, a large macromolecular assembly exhibiting severe nonisomorphism, extreme beam sensitivity and no internal symmetry. Acta Crystallogr A54, 945-55 (1998).
8. Tocilj, A. et al. The small ribosomal subunit from Thermus thermophilus at 4.5 A resolution: pattern fittings and the identification of a functional site. Proc Natl Acad Sci U S A 96, 14252-7 (1999).
9. Clemons, W. M., Jr. et al. Structure of a bacterial 30S ribosomal subunit at 5.5 Å resolution. Nature 400, 833-840 (1999).
10. Otwinowski, Z. & Minor, W. in Methods in Enzymology (eds. Carter, C. W. J. & Sweet, R. M.) 307-25 (Academic Press, New York, 1997).
11. Terwilliger, T. & Berendzen, J. Automated MAD and MIR structure determination. Acta Cryst D55, 849-861 (1999).
12. Abrahams, J. P. Bias reduction in phase refinement by modified interference functions: introducing the gamma correction. Acta Cryst. D53 (1997).
13. de la Fortelle, E. & Bricogne, G. in Methods in Enzymology (eds. Carter, C. W., Jr. & Sweet, R. M.) 472-93 (Academic Press, New York, 1997).
14. Hartmann, R. K. & Erdmann, V. A. Thermus thermophilus 16S rRNA is transcribed from an isolated transcription unit. J Bacteriol 171, 2933-41 (1989).
15. Cowtan, K. & Main, P. Miscellaneous algorithms for density modification. Acta Crystallogr D Biol Crystallogr 54, 487-93 (1998).
16. Jones, T. A. & Kjeldgaard, M. Electron-density map interpretation. Meth. Enzymol. 277B, 173-207 (1997).
17. Brünger, A. T. et al. Crystallography & NMR system: A new software suite for macromolecular structure determination. Acta Crystallogr D Biol Crystallogr 54, 905-21 (1998).
18. Choli, T., Franceschi, F., Yonath, A. & Wittmann-Liebold, B. Isolation and characterization of a new ribosomal protein from the thermophilic eubacteria, Thermus thermophilus, T. aquaticus and T. flavus. Biol Chem Hoppe Seyler 374, 377-83 (1993).
19. Mueller, F. & Brimacombe, R. A new model for the three-dimensional folding of Escherichia coli 16 S ribosomal RNA. I. Fitting the RNA to a 3D electron microscopic map at 20 A. J Mol Biol 271, 524-44 (1997).
20. Fourmy, D., Recht, M. I., Blanchard, S. C. & Puglisi, J. D. Structure of the A site of Escherichia coli 16S ribosomal RNA complexed with an aminoglycoside antibiotic. Science 274, 1367-71 (1996).
21. Yoshizawa, S., Fourmy, D. & Puglisi, J. D. Recognition of the codon-anticodon helix by ribosomal RNA. Science 285, 1722-5 (1999).
22. Moazed, D. & Noller, H. F. Interaction of antibiotics with functional sites in 16S ribosomal RNA. Nature 327, 389-394 (1987).
23. Brink, M. F., Brink, G., Verbeet, M. P. & de Boer, H. A. Spectinomycin interacts specifically with the residues G1064 and C1192 in 16S rRNA, thereby potentially freezing this molecule into an inactive conformation. Nucleic Acids Res 22, 325-31 (1994).
24. Wittmann-Liebold, B. & Greuer, B. The primary structure of protein S5 from the small subunit of the Escherichia coli ribosome. FEBS Lett 95, 91-8 (1978).
25. Gravel, M., Melancon, P. & Brakier-Gingras, L. Cross-linking of streptomycin to the 16S ribosomal RNA of Escherichia coli. Biochemistry 26, 6227-32 (1987).
26. Montandon, P. E., Wagner, R. & Stutz, E. E. coli ribosomes with a C912 to U base change in the 16S rRNA are streptomycin resistant. Embo J 5, 3705-8 (1986).
27. Pinard, R., Payant, C., Melancon, P. & Brakier-Gingras, L. The 5' proximal helix of 16S rRNA is involved in the binding of streptomycin to the ribosome. Faseb J 7, 173-6 (1993).
28. Melancon, P., Lemieux, C. & Brakier-Gingras, L. A mutation in the 530 loop of Escherichia coli 16S ribosomal RNA causes resistance to streptomycin. Nucleic Acids Res 16, 9631-9 (1988).
29. Spahn, C. M. & Prescott, C. D. Throwing a spanner in the works: antibiotics and the translation apparatus. J Mol Med 74, 423-39 (1996).
30. Lodmell, J. S. & Dahlberg, A. E. A conformational switch in Escherichia coli 16S ribosomal RNA during decoding of messenger RNA. Science 277, 1262-7 (1997).
31. Cate, J. H., Yusupov, M. M., Yusupova, G. Z., Earnest, T. N. & Noller, H. F. X-ray crystal structures of 70S ribosome functional complexes [see comments]. Science 285, 2095-104 (1999).
32. Pape, T., Wintermeyer, W. & Rodnina, M. V. Conformational switch in the decoding region of 16S rRNA during aminoacyl-tRNA selection on the ribosome. Nat Struct Biol 7, 104-7 (2000).
33. Funatsu, G. & Wittmann, H. G. Ribosomal proteins. 33. Location of amino-acid replacements in protein S12 isolated from Escherichia coli mutants resistant to streptomycin. J Mol Biol 68, 547-50 (1972).
34. Ito, T. & Wittmann, H. G. Amino acid replacements in proteins S5 and S12 of two Escherichia coli revertants from streptomycin dependence to independence. Mol Gen Genet 127, 19-32 (1973).
35. van Acken, U. Proteinchemical studies on ribosomal proteins S4 and S12 from ram (ribosomal ambiguity) mutants of Escherichia coli. Mol Gen Genet 140, 61-8 (1975).
36. Timms, A. R. & Bridges, B. A. Double, independent mutational events in the rpsL gene of Escherichia coli: an example of hypermutability? Mol Microbiol 9, 335-42 (1993).
37. Timms, A. R., Steingrimsdottir, H., Lehmann, A. R. & Bridges, B. A. Mutant sequences in the rpsL gene of Escherichia coli B/r: mechanistic implications for spontaneous and ultraviolet light mutagenesis. Mol Gen Genet 232, 89-96 (1992).
38. Tubulekas, I., Buckingham, R. H. & Hughes, D. Mutant ribosomes can generate dominant kirromycin resistance. J Bacteriol 173, 3635-43 (1991).
39. Kurland, C. G., Hughes, D., Ehrenberg, M. Limitations of translational accuracy. (ed. Neidhardt, F. C., Curtiss, III, R., Ingraham, J.L., Lin, E.C.C., Low, K.B., Magasanik, B., et al) (American Society for Microbiology Press, Washington, DC, 1996).
40. Montandon, P. E., Nicolas, P., Schurmann, P. & Stutz, E. Streptomycin-resistance of Euglena gracilis chloroplasts: identification of a point mutation in the 16S rRNA gene in an invariant position. Nucleic Acids Res 13, 4299-310 (1985).
41. Leclerc, D., Melancon, P. & Brakier-Gingras, L. Mutations in the 915 region of Escherichia coli 16S ribosomal RNA reduce the binding of streptomycin to the ribosome. Nucleic Acids Res 19, 3973-7 (1991).
42. Melancon, P., Boileau, G. & Brakier-Gingras, L. Cross-linking of streptomycin to the 30S subunit of Escherichia coli with phenyldiglyoxal. Biochemistry 23, 6697-703 (1984).
43. Powers, T. & Noller, H. F. A functional pseudoknot in 16S ribosomal RNA. Embo J 10, 2203-14 (1991).
44. Donner, D. & Kurland, C. G. Changes in the primary structure of a mutationally altered ribosomal protein S4 of Escherichia coli. Mol Gen Genet 115, 49-53 (1972).
45. Funatsu, G., Puls, W., Schiltz, E., Reinbolt, J. & Wittmann, H. G. Ribosomal proteins. XXXI. Comparative studies on altered proteins S4 of six Escherichia coli revertants from streptomycin dependence. Mol Gen Genet 115, 131-9 (1972).
46. Bock, A., Petzet, A. & Piepersberg, W. Ribosomal ambiguity (ram) mutations facilitate diyhydrostreptomycin binding to ribosomes. FEBS Lett 104, 317-21 (1979).
47. Chopra, I., Hawkey, P. M. & Hinton, M. (1992) *J Antimicrob Chemother* **29,** 245-77.
48. Ross, J. I., Eady, E. A., Cove, J. H. & Cunliffe, W. J. (1998) *Antimicrob Agents Chemother* **42,** 1702-5.
49. Geigenmuller, U. & Nierhaus, K. H. (1986) *Eur J Biochem* **161,** 723-6.
50. Eustice, D. C. & Wilhelm, J. M. (1984) *Biochemistry* **23,** 1462-7.
51. Brown, C. M., McCaughan, K. K. & Tate, W. P. (1993) *Nucleic Acids Res* **21,** 2109-15.
52. Kolesnikov, I. V., Protasova, N. Y. & Gudkov, A. T. (1996) *Biochimie* **78,** 868-73.
53. Manavathu, E. K., Fernandez, C. L., Cooperman, B. S. & Taylor, D. E. (1990) *Antimicrob Agents Chemother* **34,** 71-7.
54. Burdett, V. (1996) *J Bacteriol* **178,** 3246-51.
55. Oehler, R., Polacek, N., Steiner, G. & Barta, A. (1997) *Nucleic Acids Res* **25,** 1219-24.
56. Bhuyan, B. K., Dietz, A. & Smith, C. G. (1961) *Antimicrob Agents Chemother,* 184-90.
57. Woodcock, J., Moazed, D., Cannon, M., Davies, J. & Noller, H. F. (1991) *Embo J* **10,** 3099-103.
58. Egebjerg, J. & Garrett, R. A. (1991) *Biochimie* **73,** 1145-9.
59. Tejedor, F., Amils, R. & Ballesta, J. P. (1985) *Biochemistry* **24,** 3667-72.
60. Cohen, L. B., Goldberg, I. H. & Herner, A. E. (1969) *Biochemistry* **8,** 1327-35.
61. Carter, A. P., Clemons Jr, W. M., Brodersen, D. E., Morgan-Warren, R. J., Wimberly, B. T. & Ramakrishnan, V. (2000) *Nature* **407,** 340-8.
62. Mann, R. L. & Bromer, W. W. (1958) *J. Am. Chem*. *Soc.* **80,** 2714-6.
63. Gonzales, A., Jimenez, A., Vasquez, D., Davies, J. E. & Schindler, D. (1978) *Biochim Biophys Acta* **521,** 459-69.
64. Cabanas, M. J., Vazquez, D. & Modolell, J. (1978) *Biochem Biophys Res Commun* **83,** 991-7.
65. Eustice, D. C. & Wilhelm, J. M. (1984) *Antimicrob Agents Chemother* **26,** 53-60.
66. Zierhut, G., Piepersberg, W. & Bock, A. (1979) *Eur J Biochem* **98,** 577-83.
67. Spangler, E. A. & Blackburn, E. H. (1985) *J Biol Chem* **260,** 6334-40.
68. Moazed, D. & Noller, H. F. (1990) *J Mol Biol* **211,** 135-45.

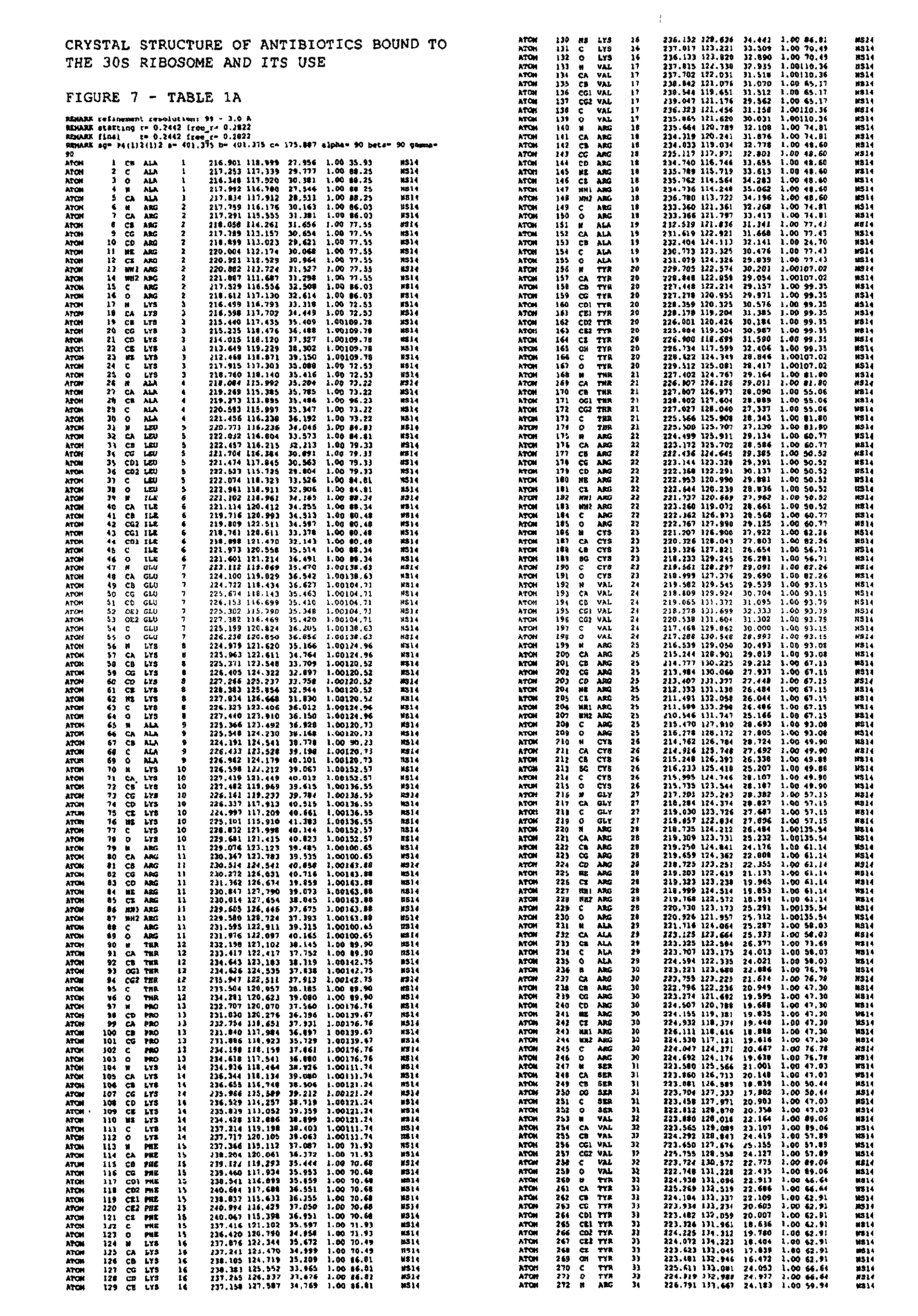

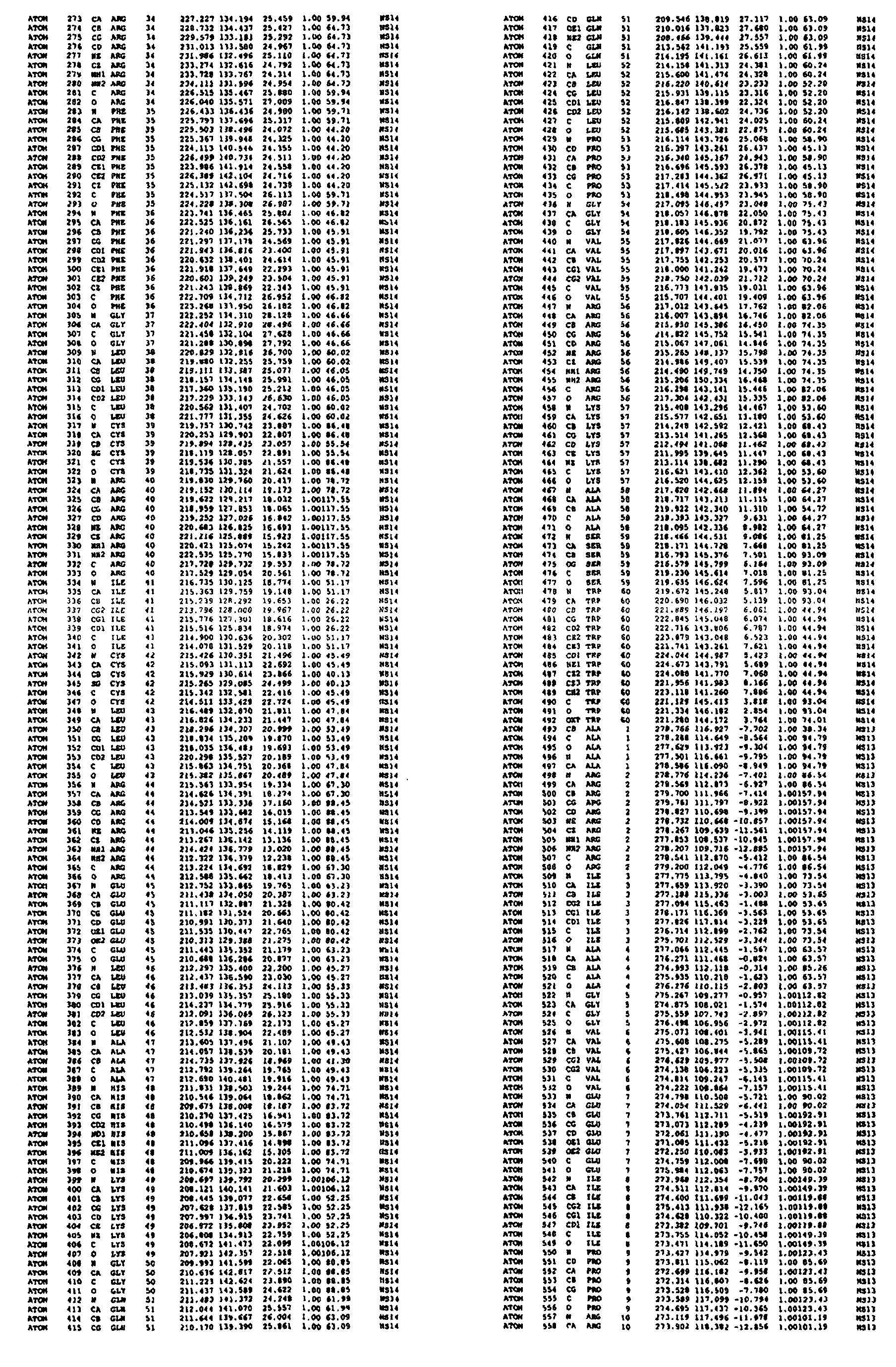

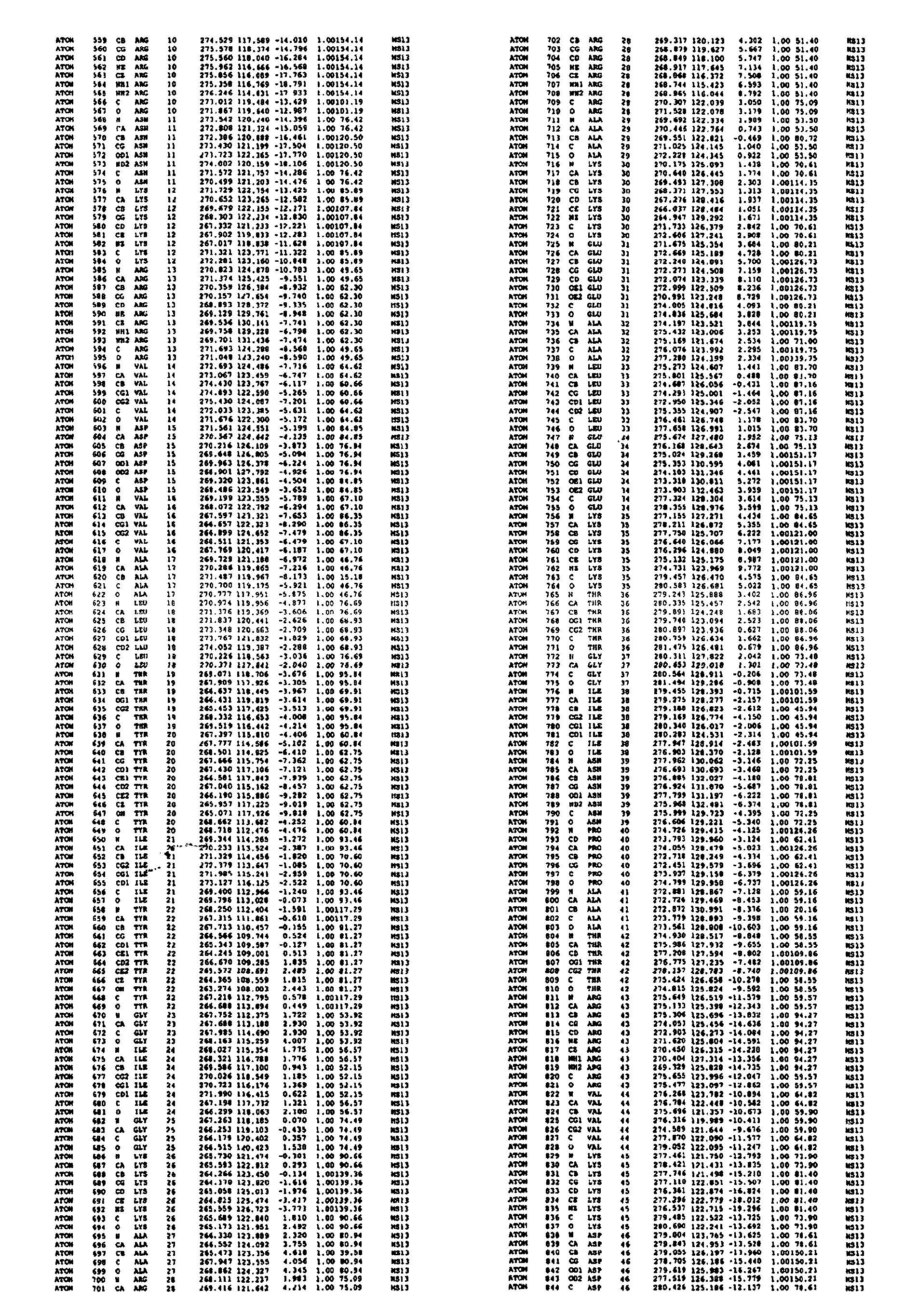

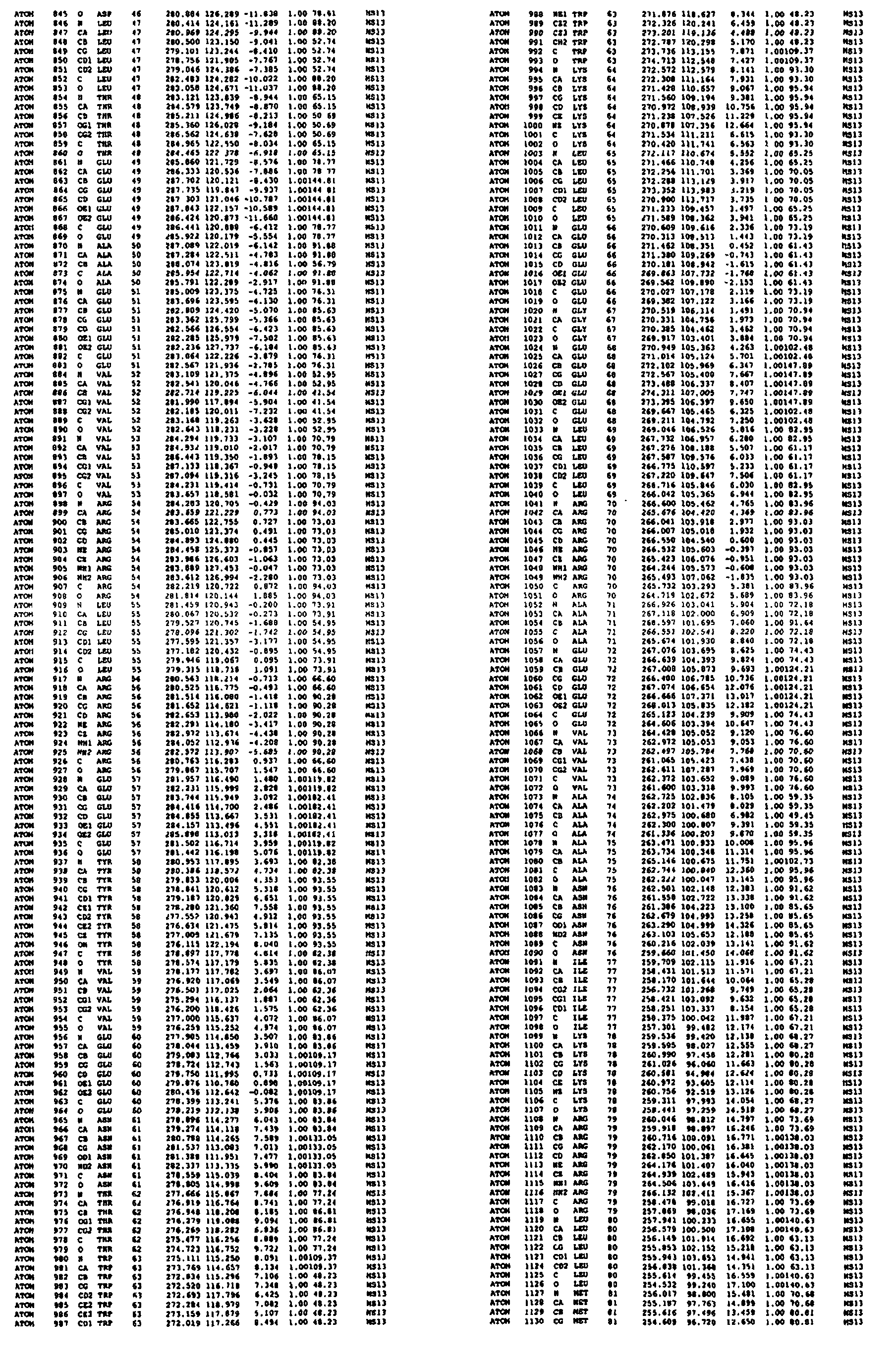

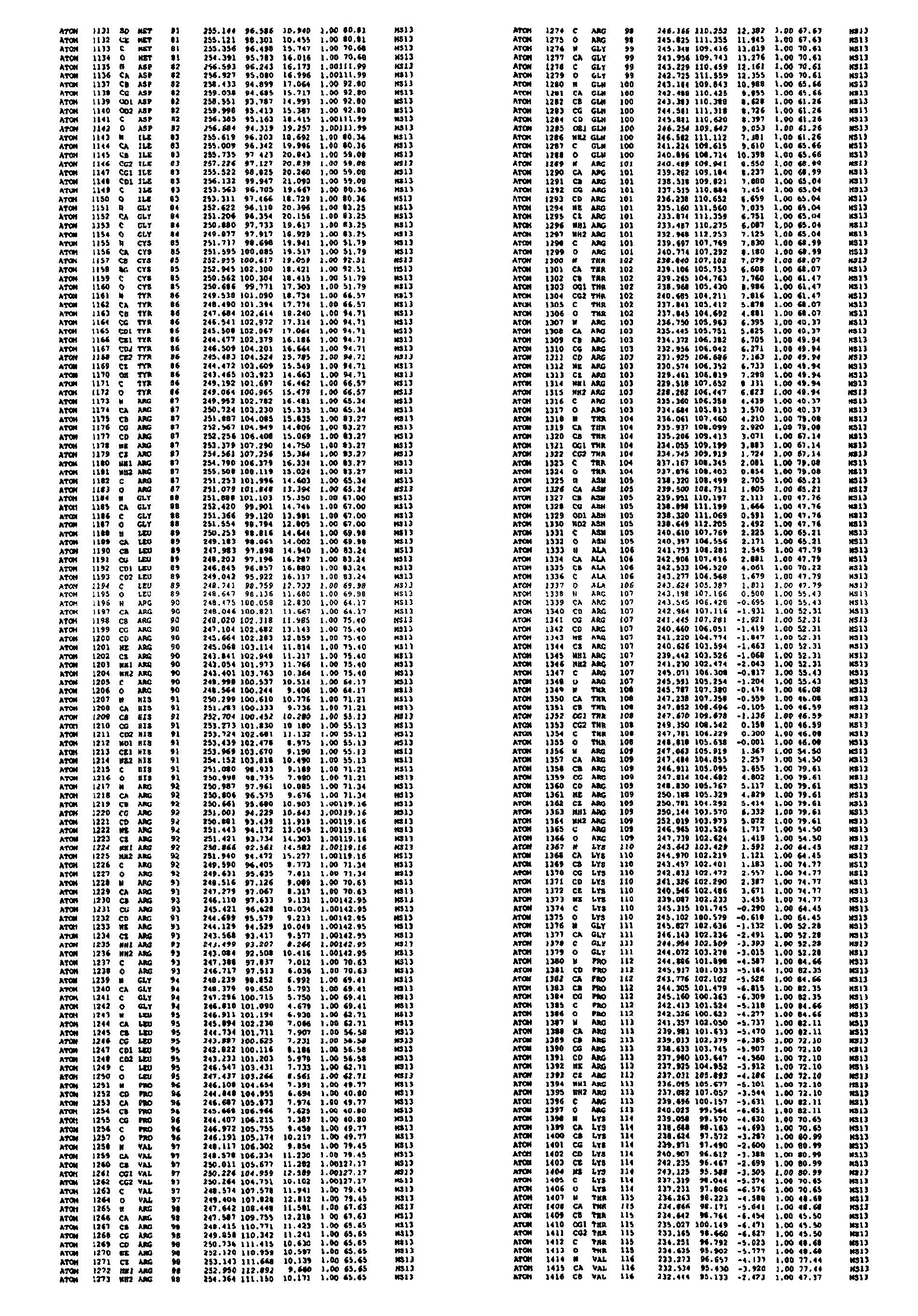

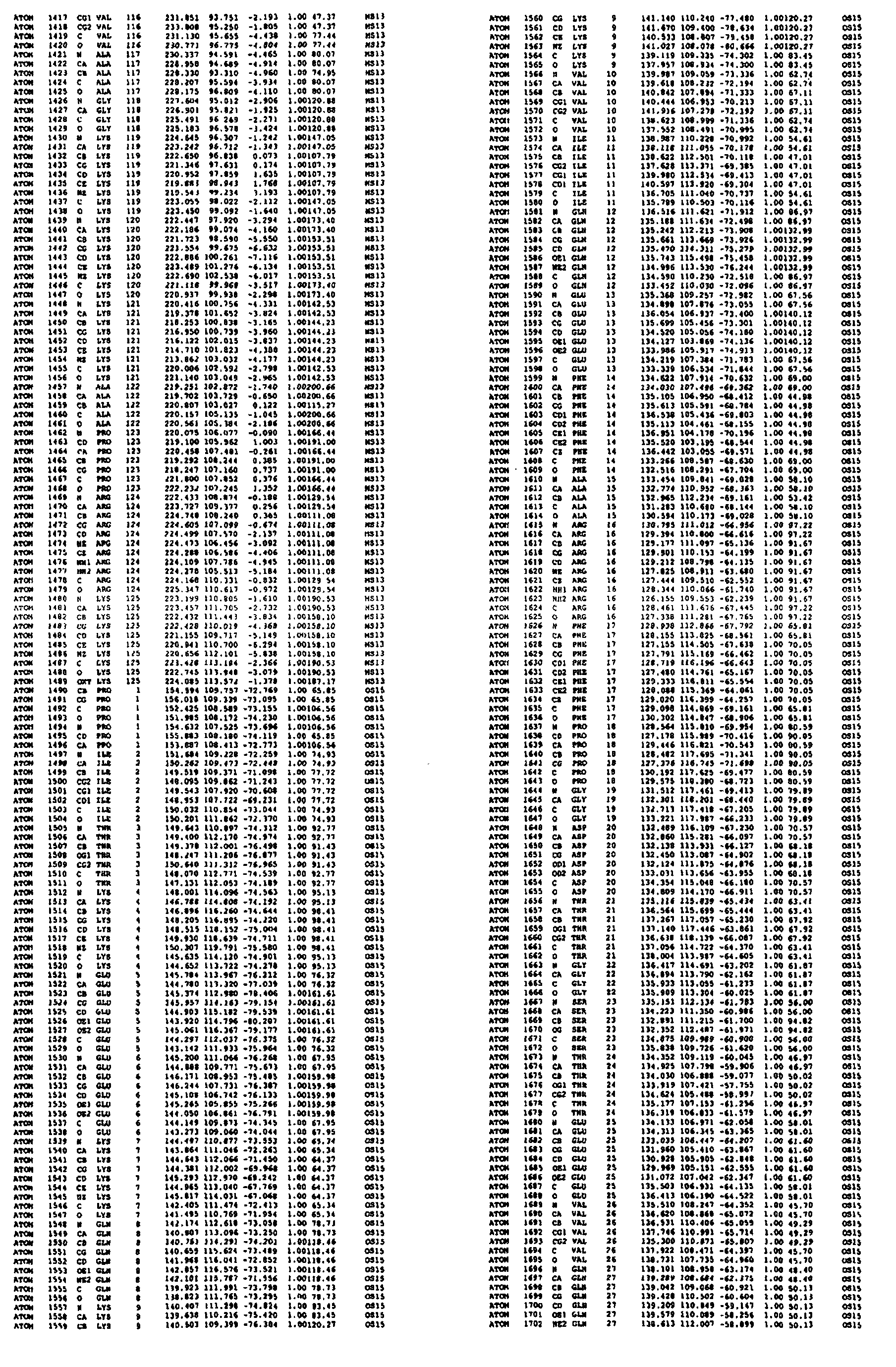

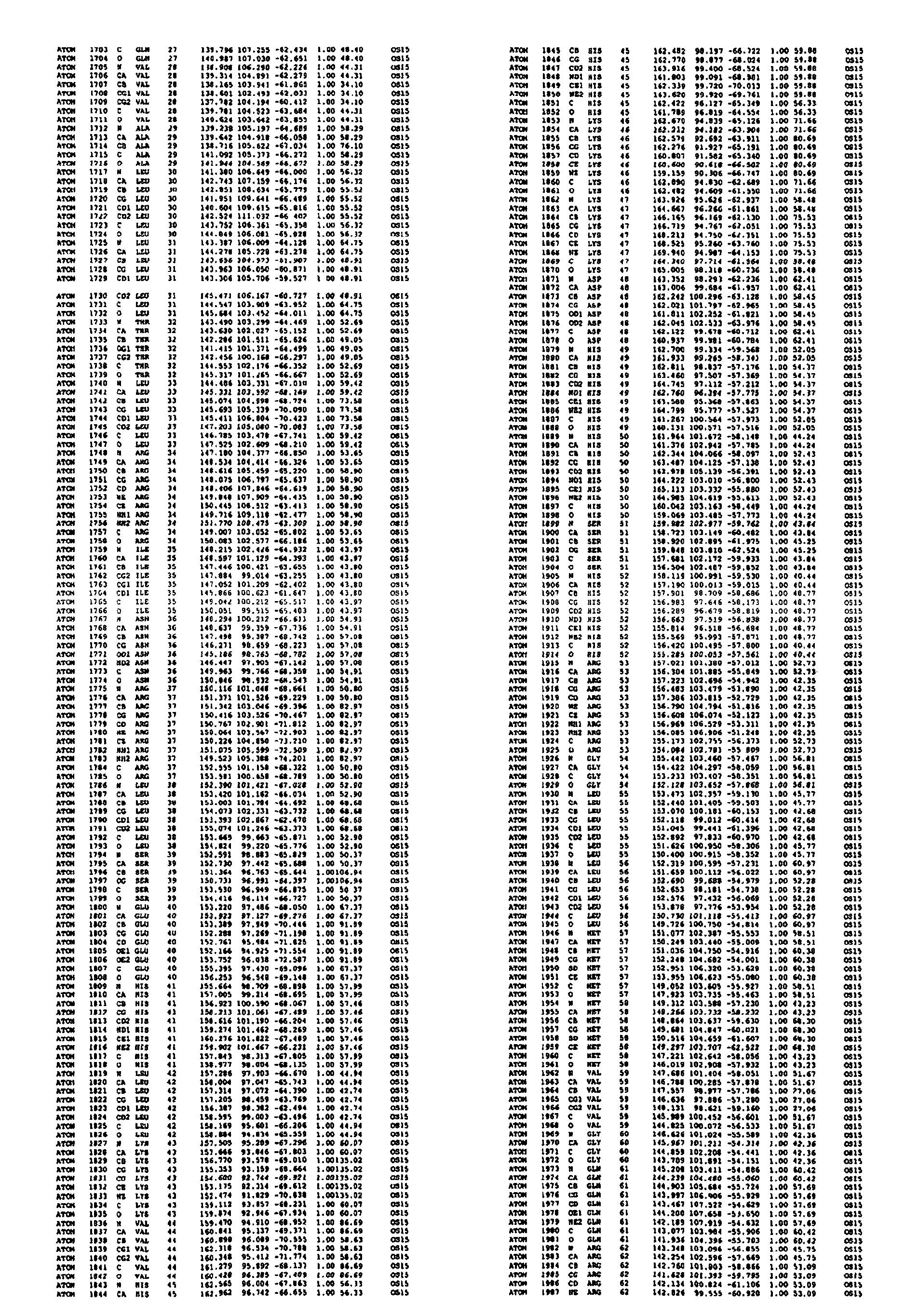

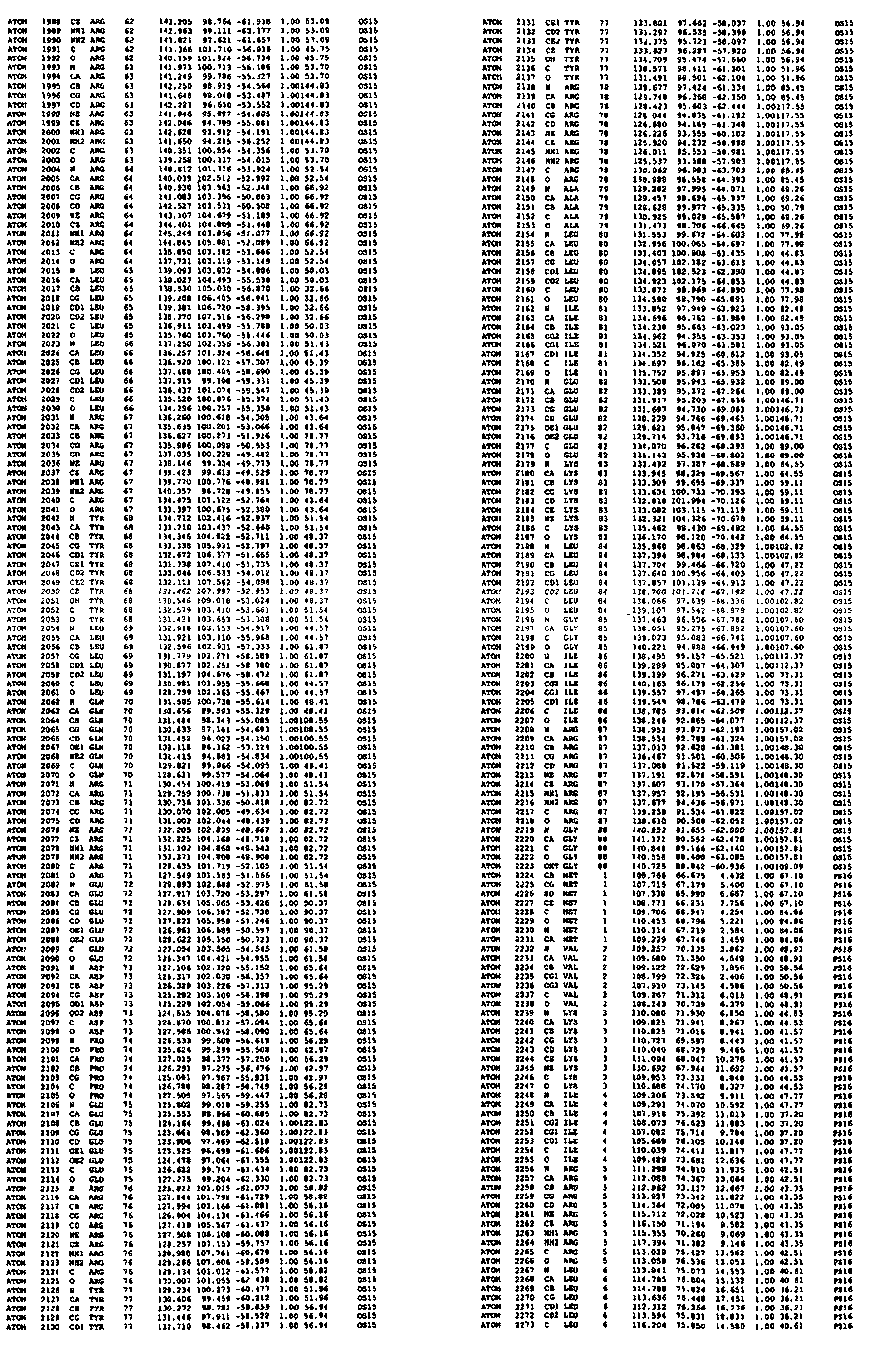

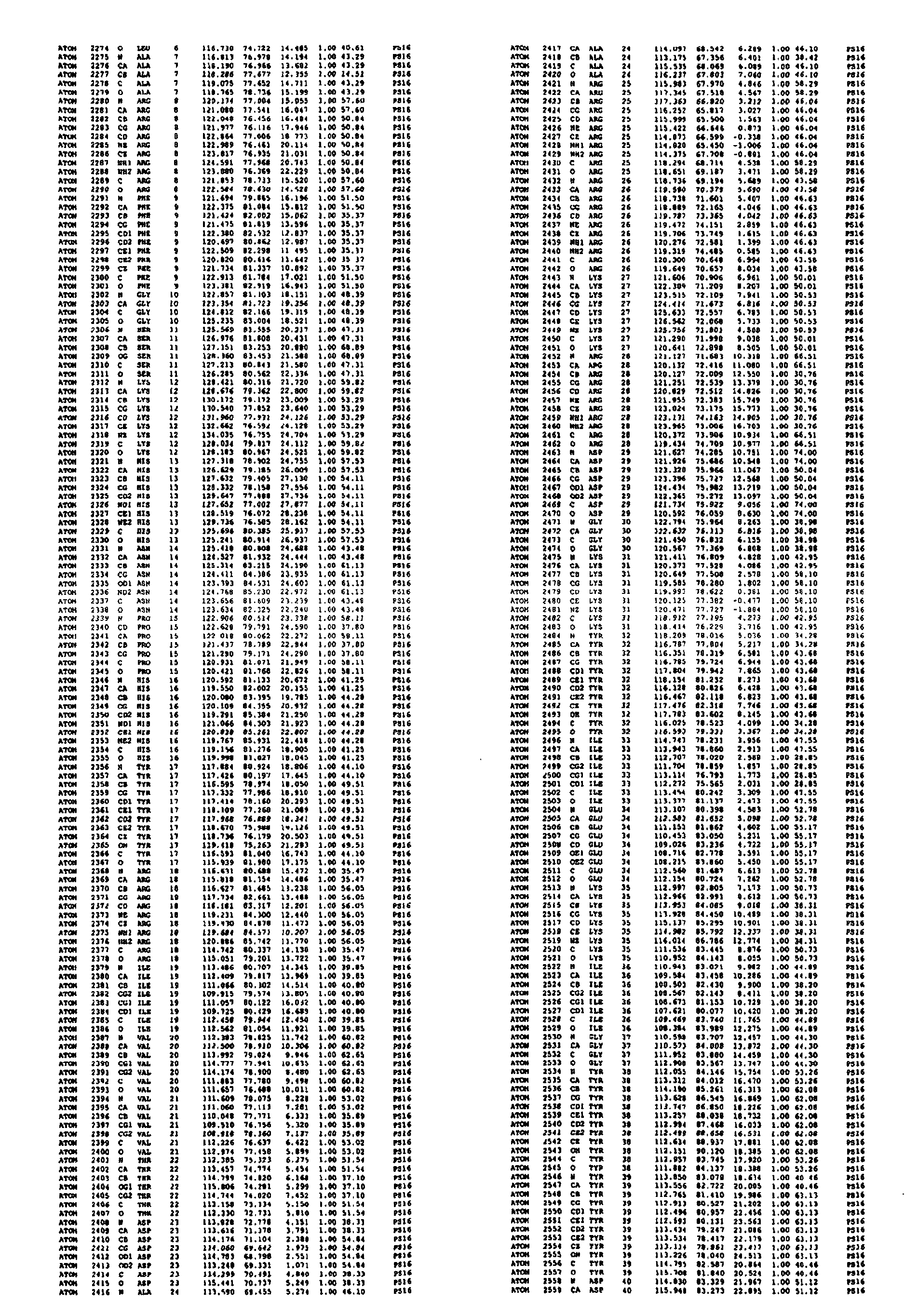

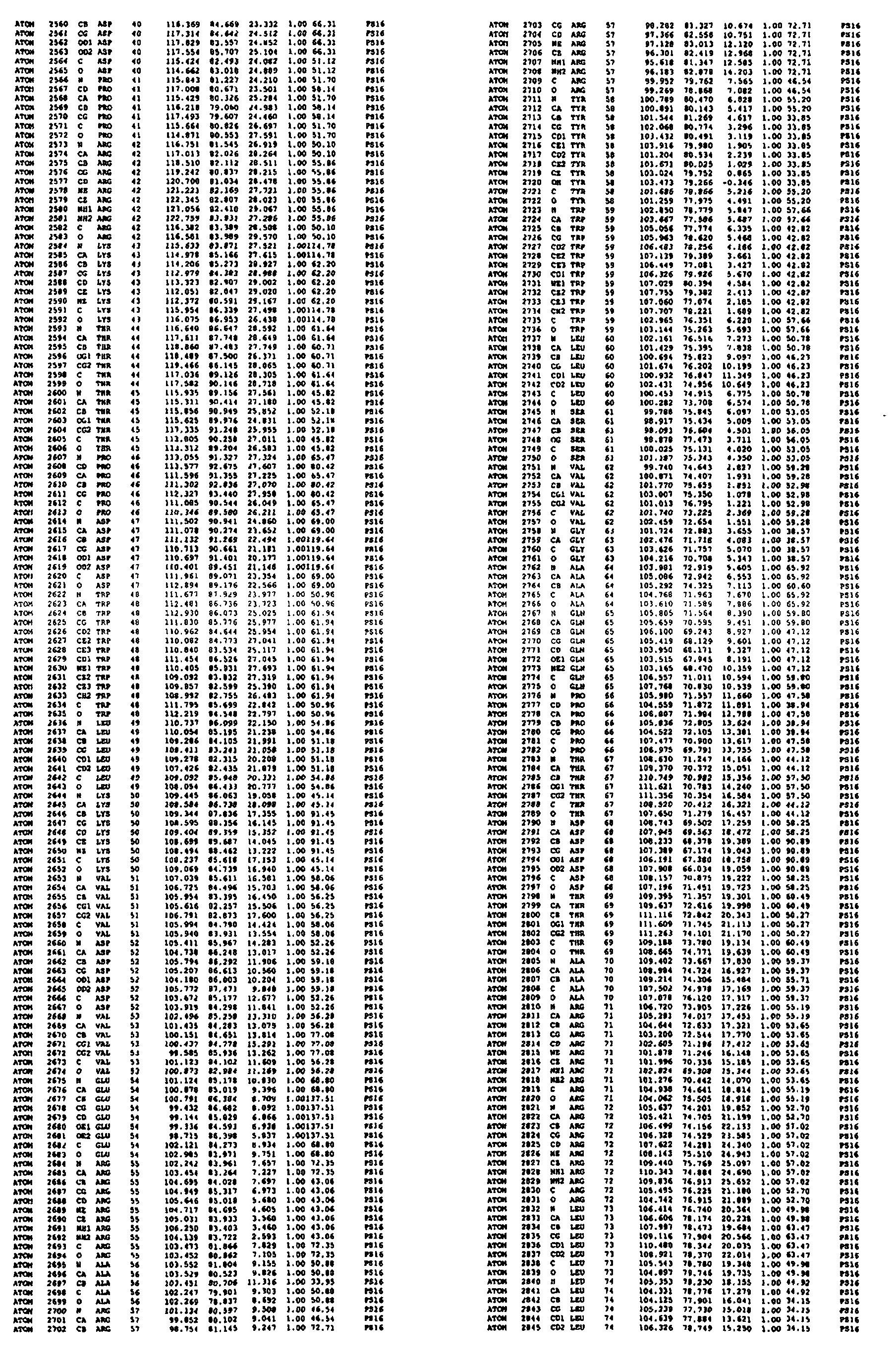

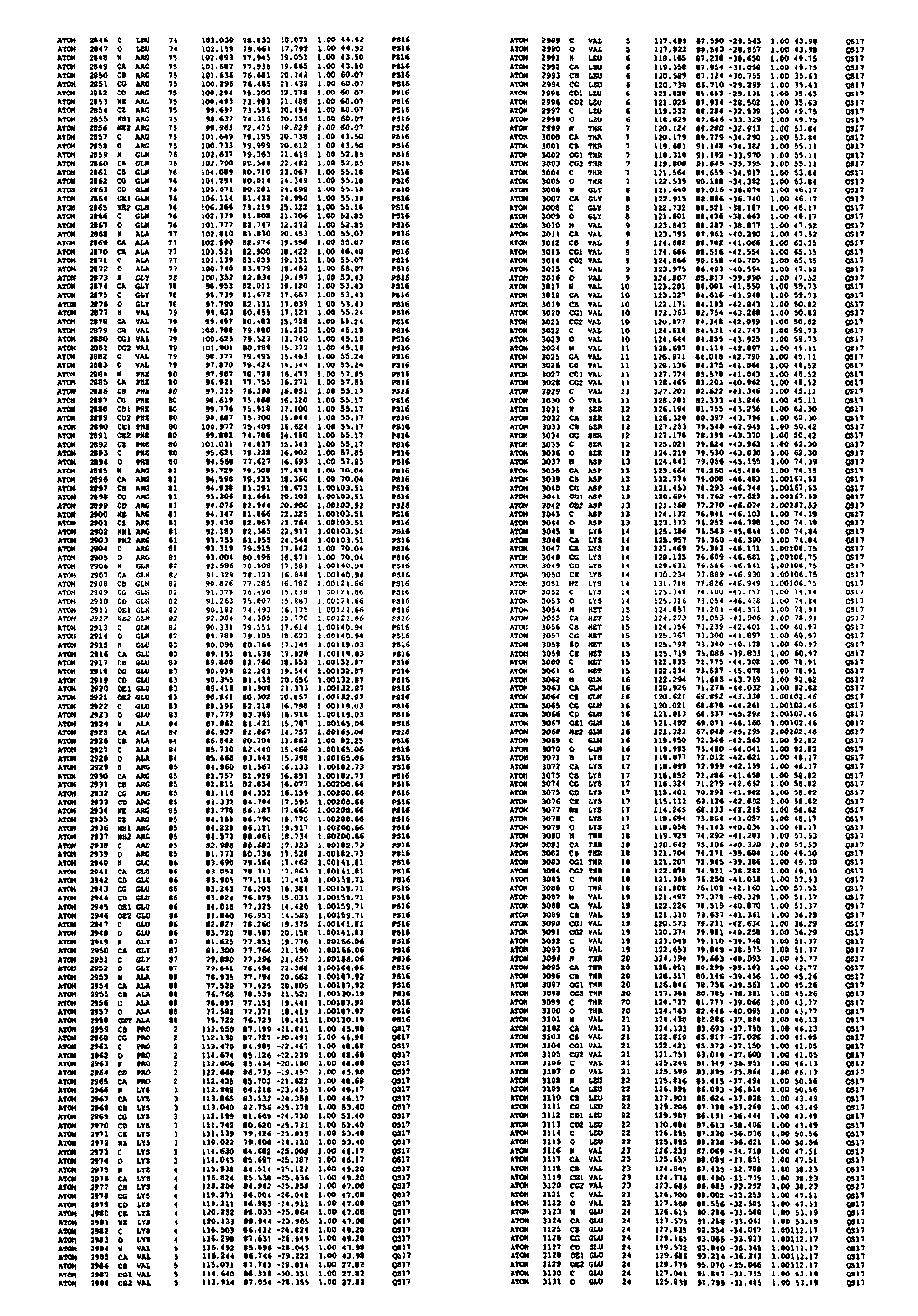

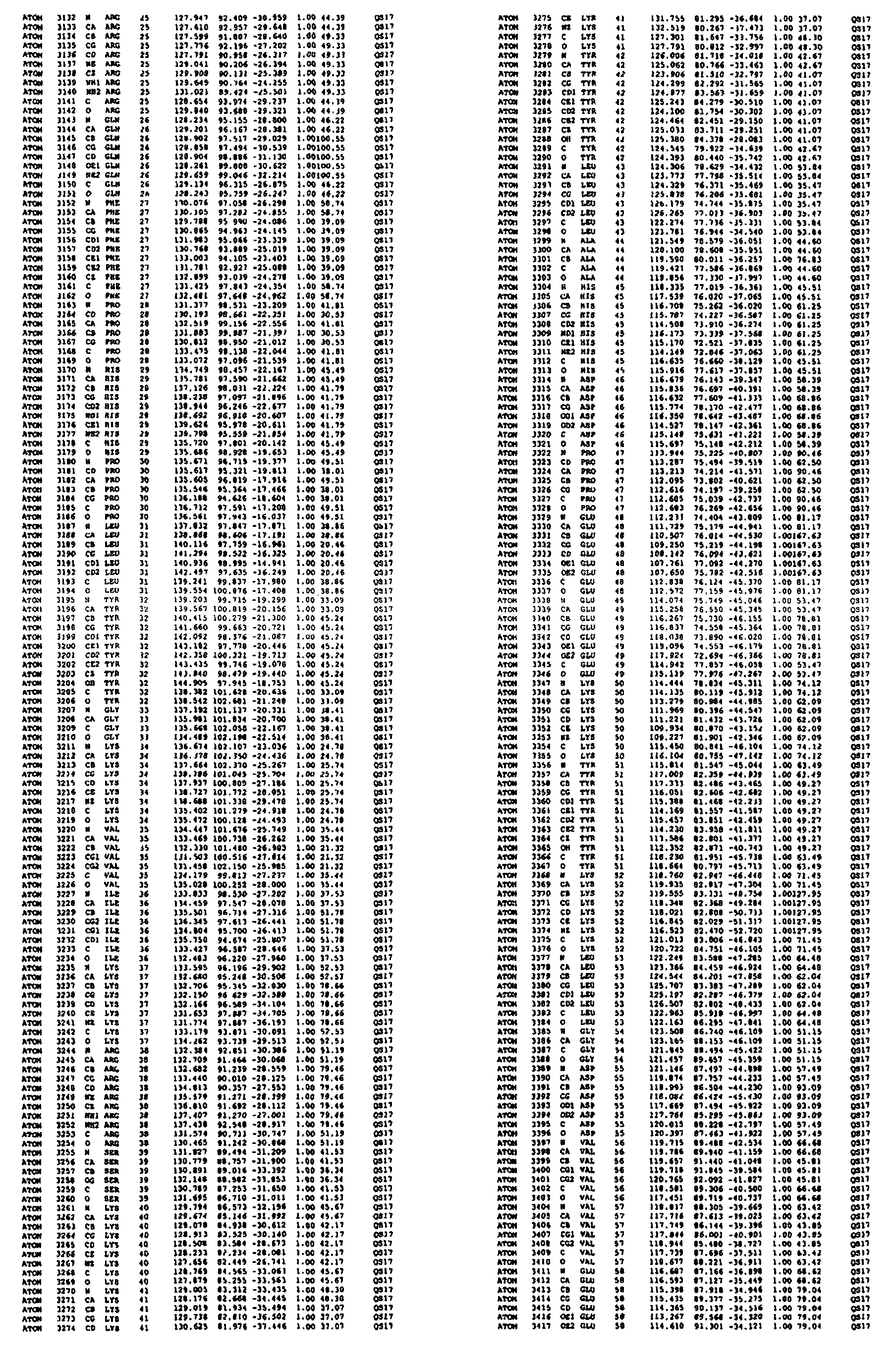

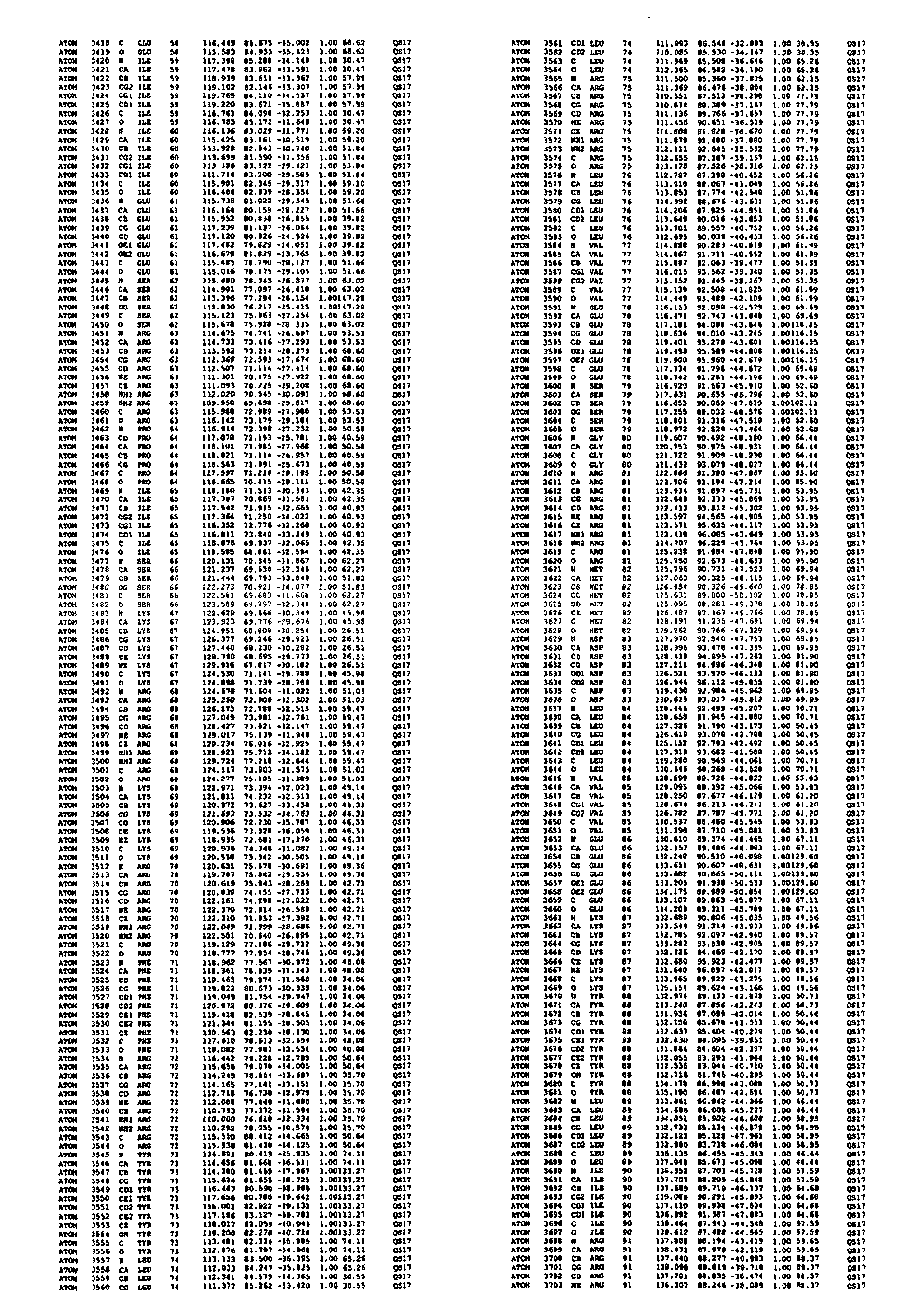

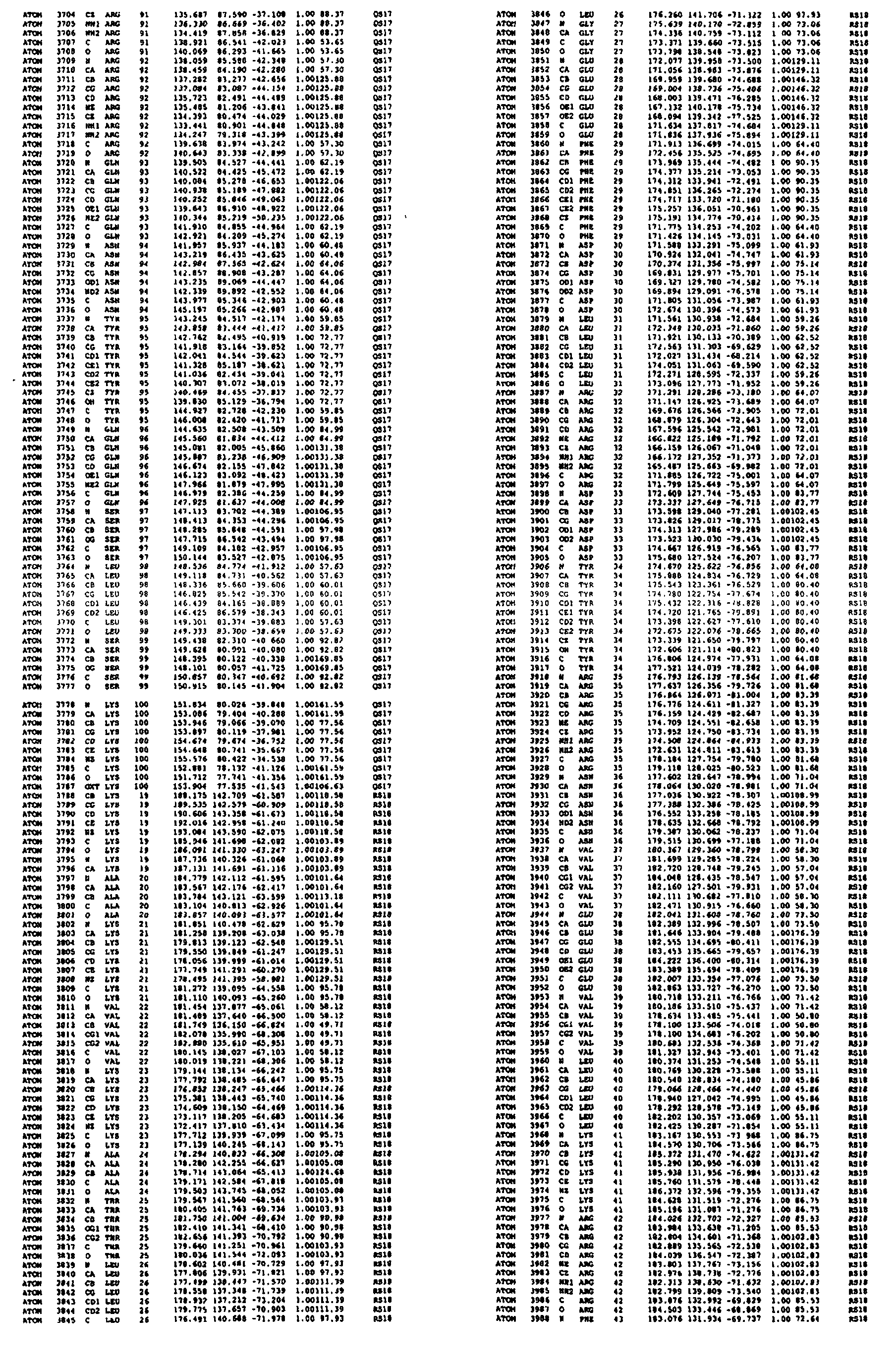

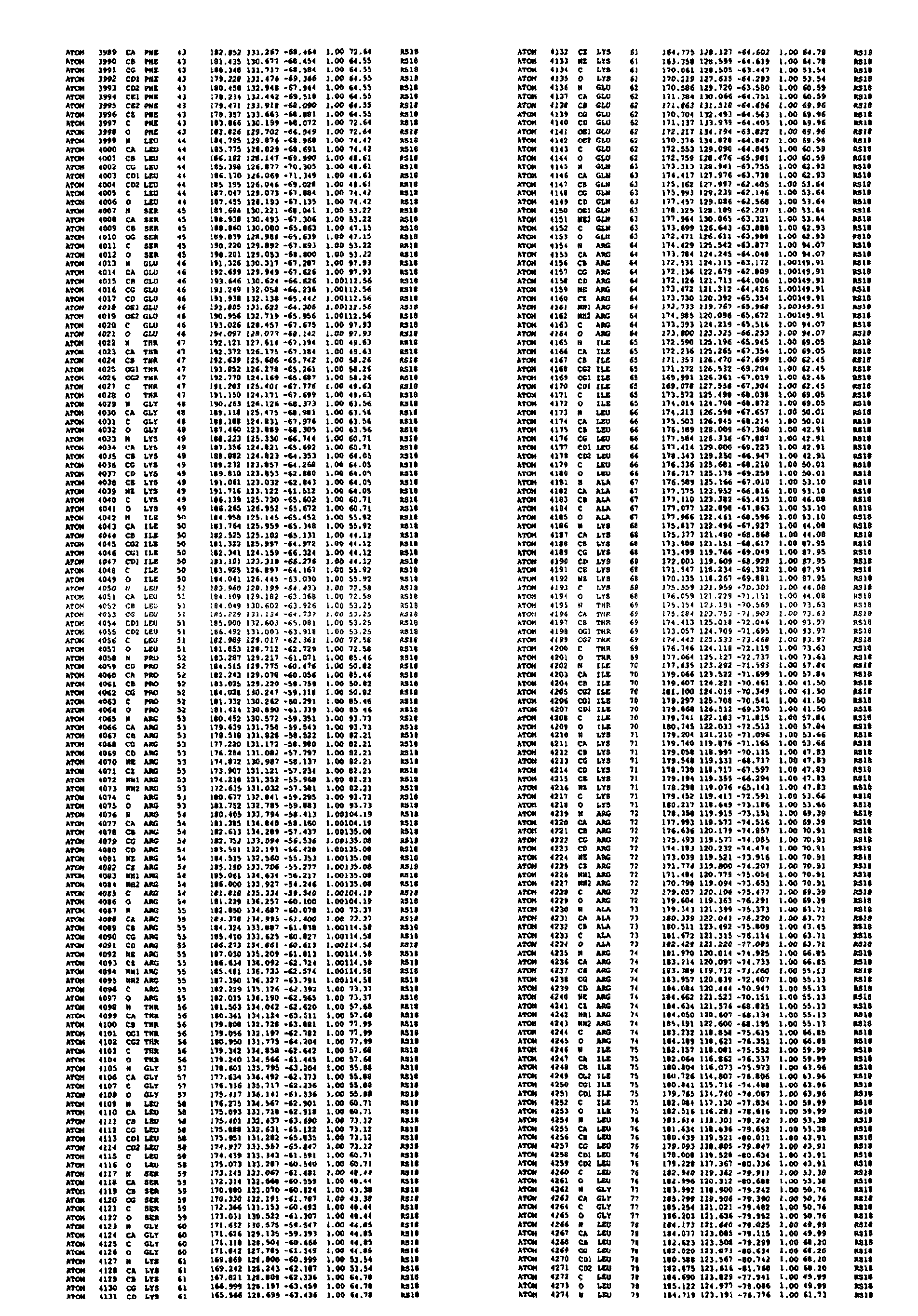

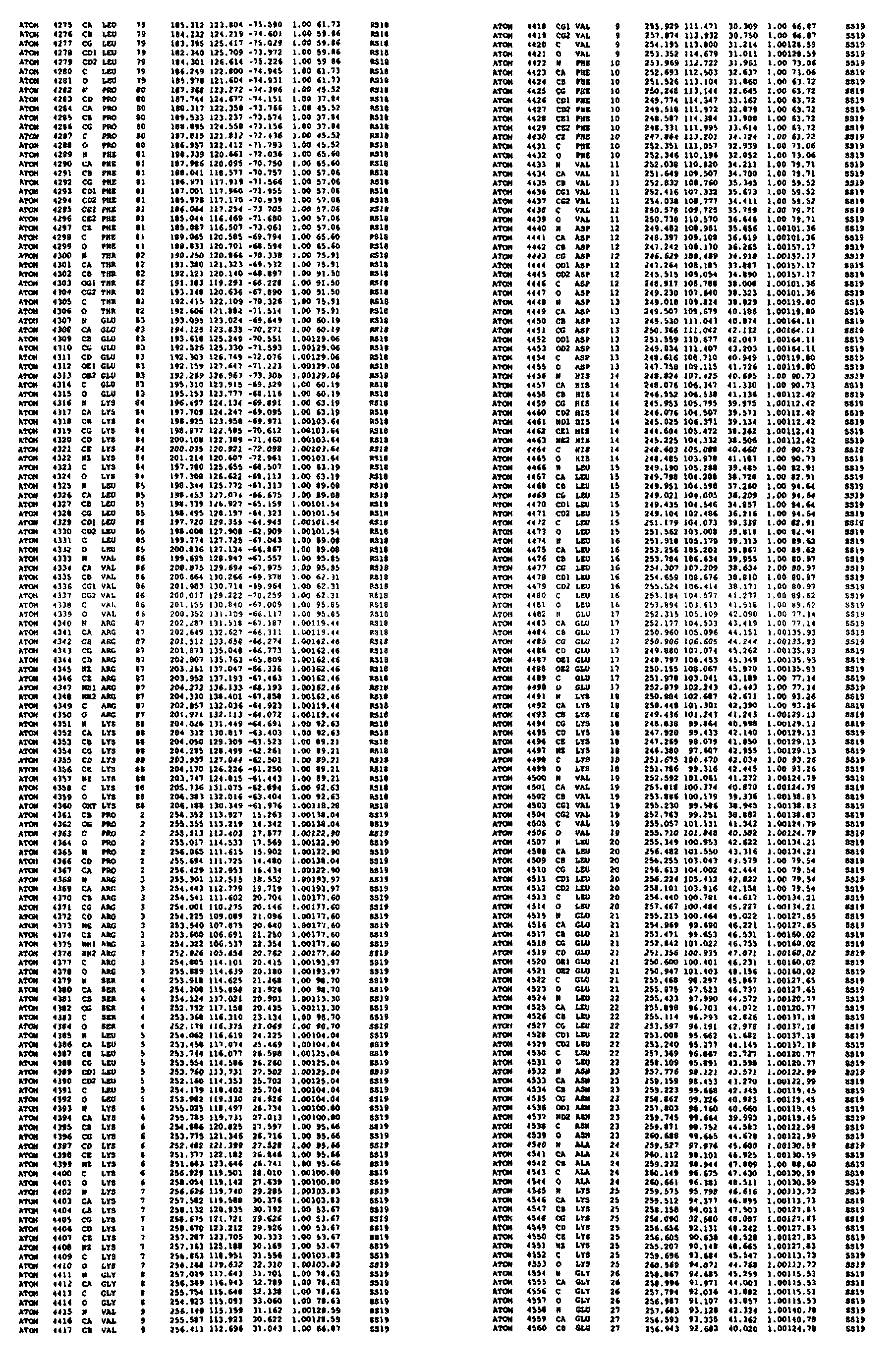

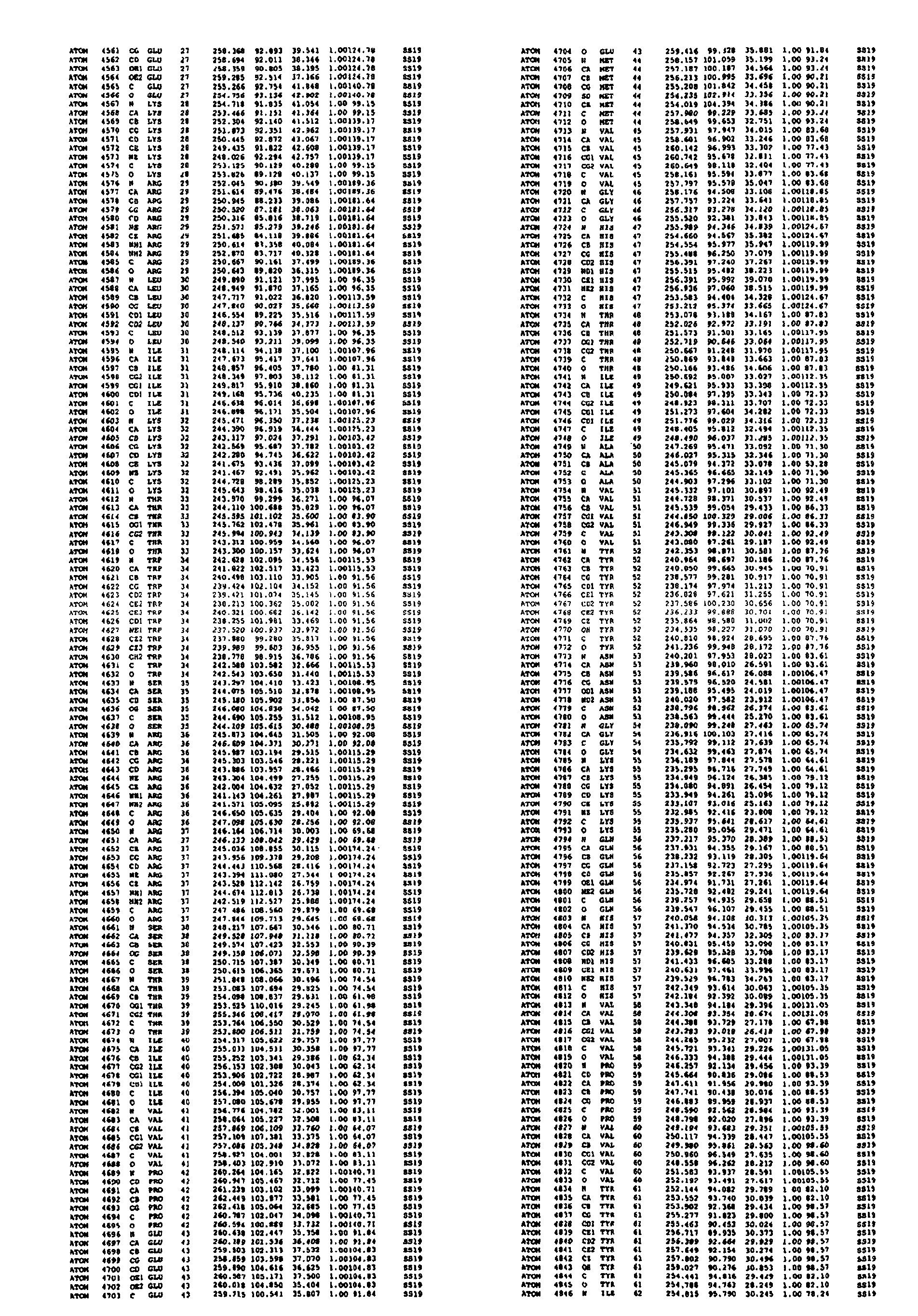

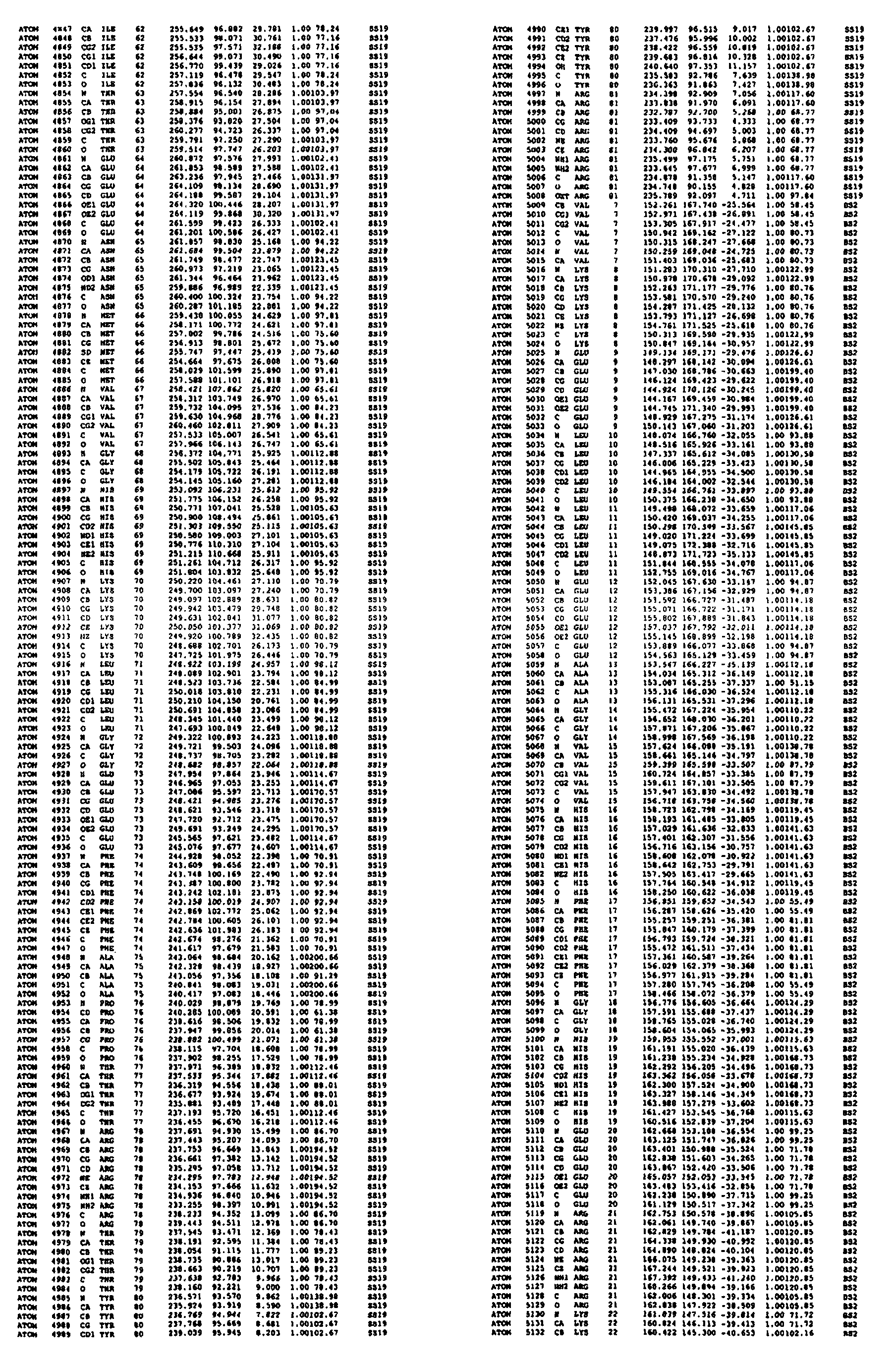

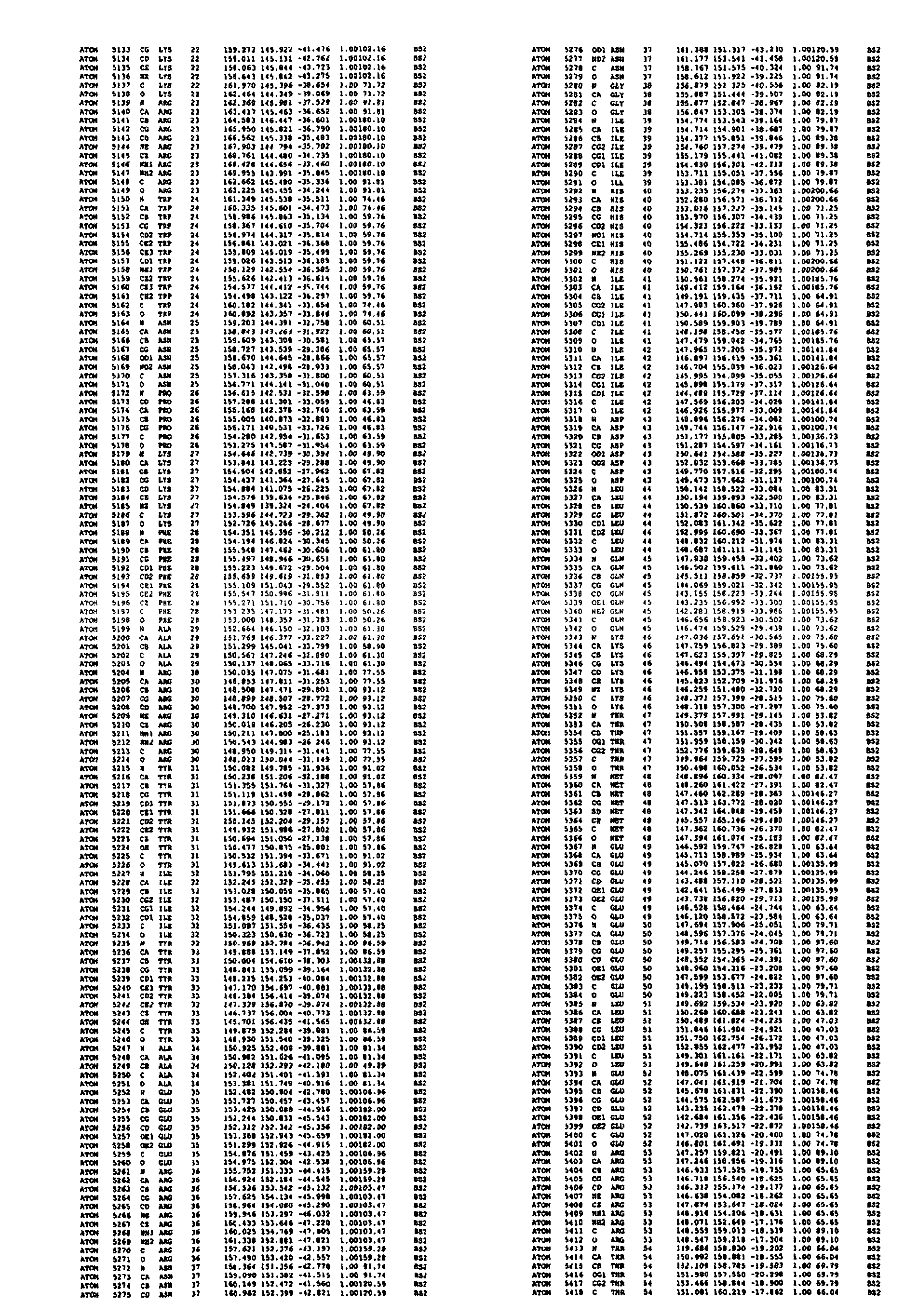

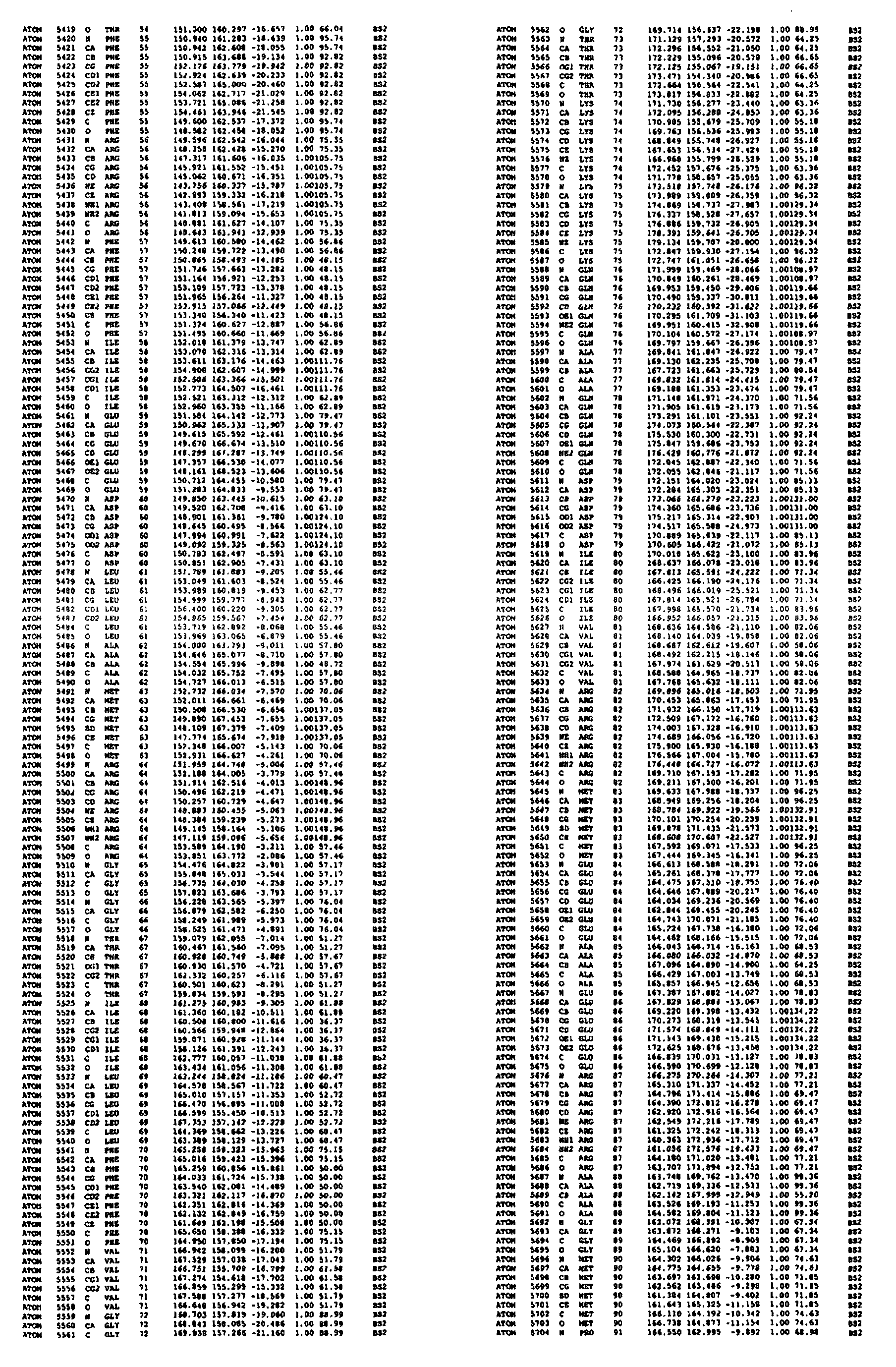

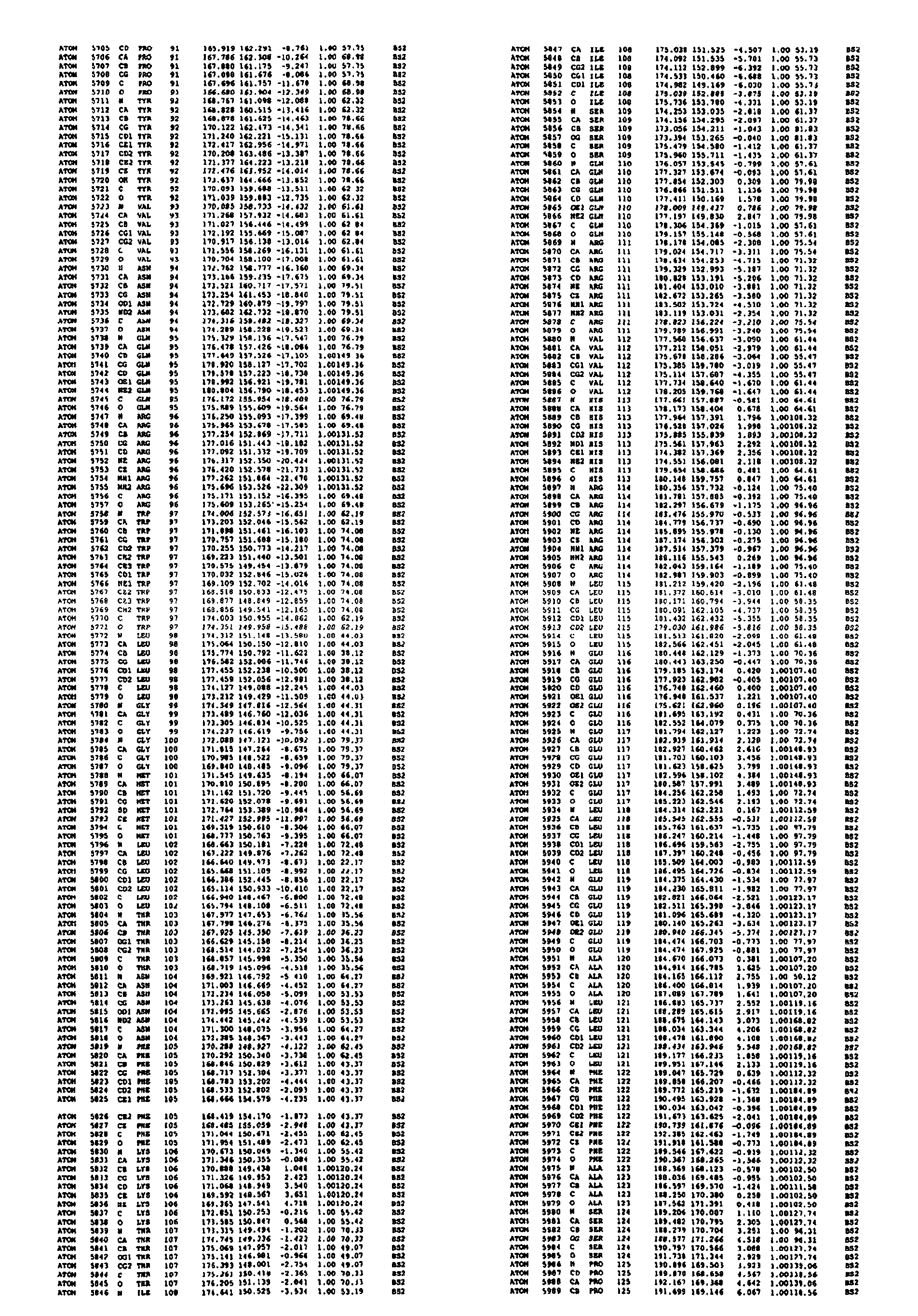

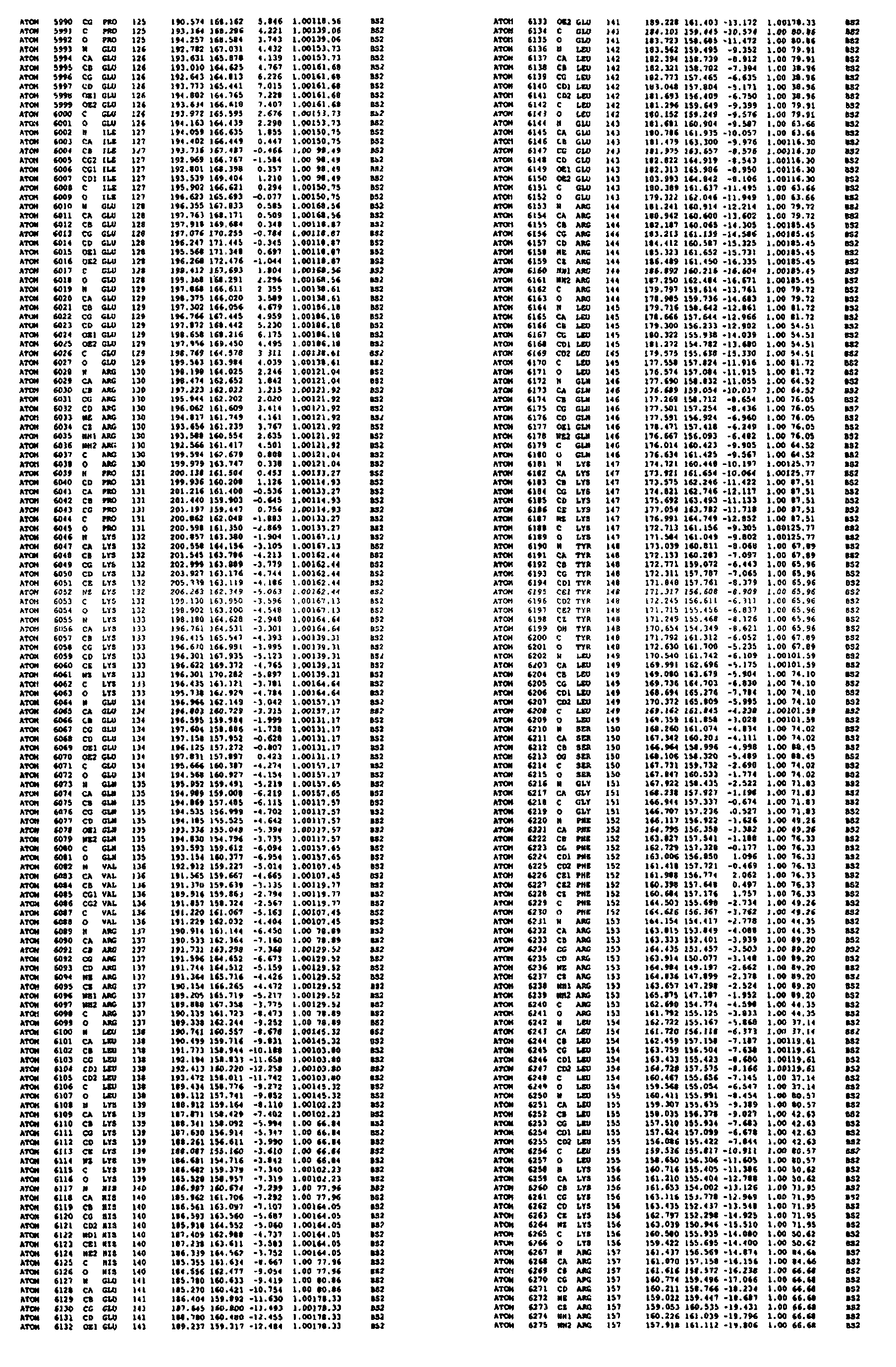

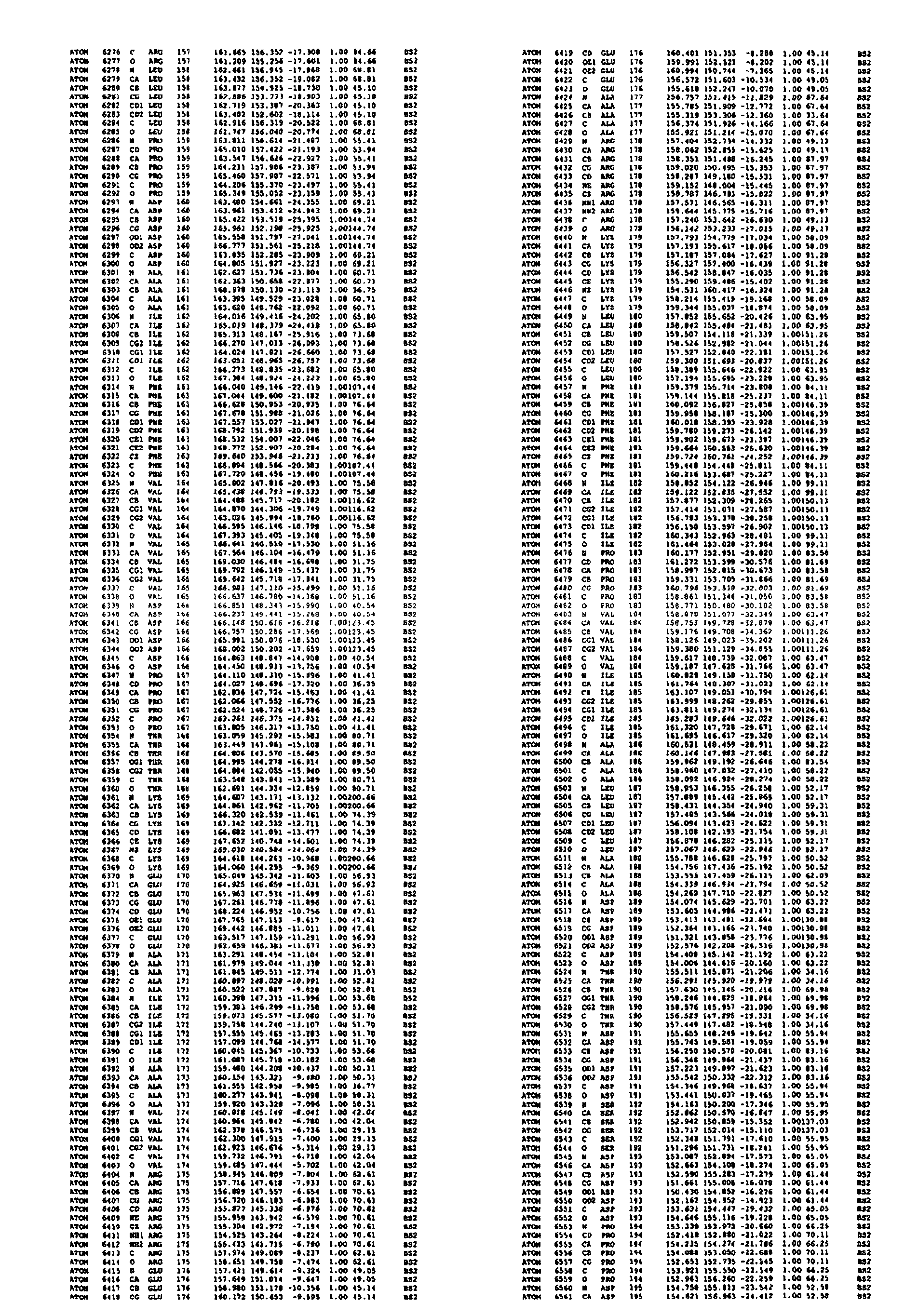

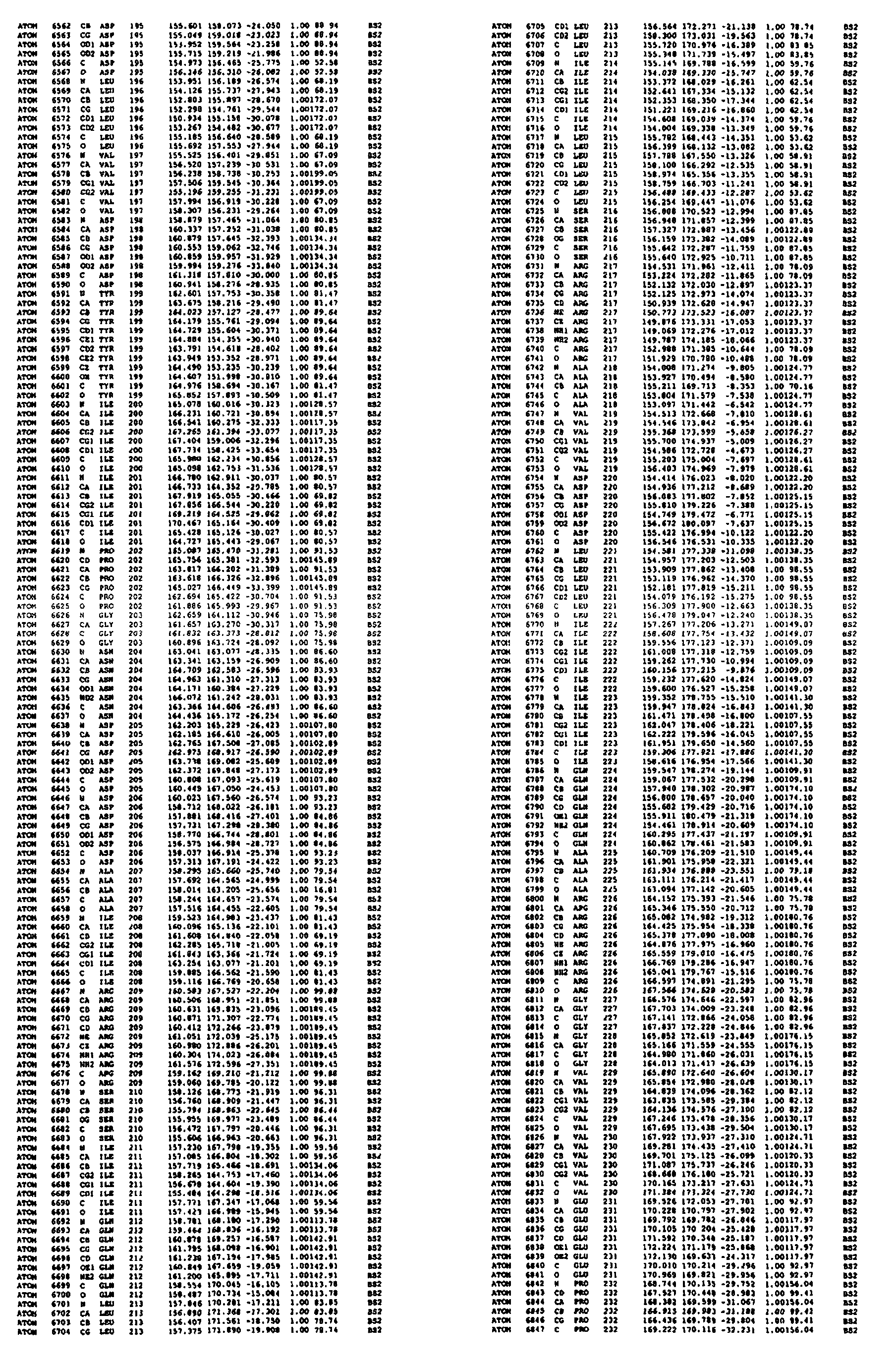

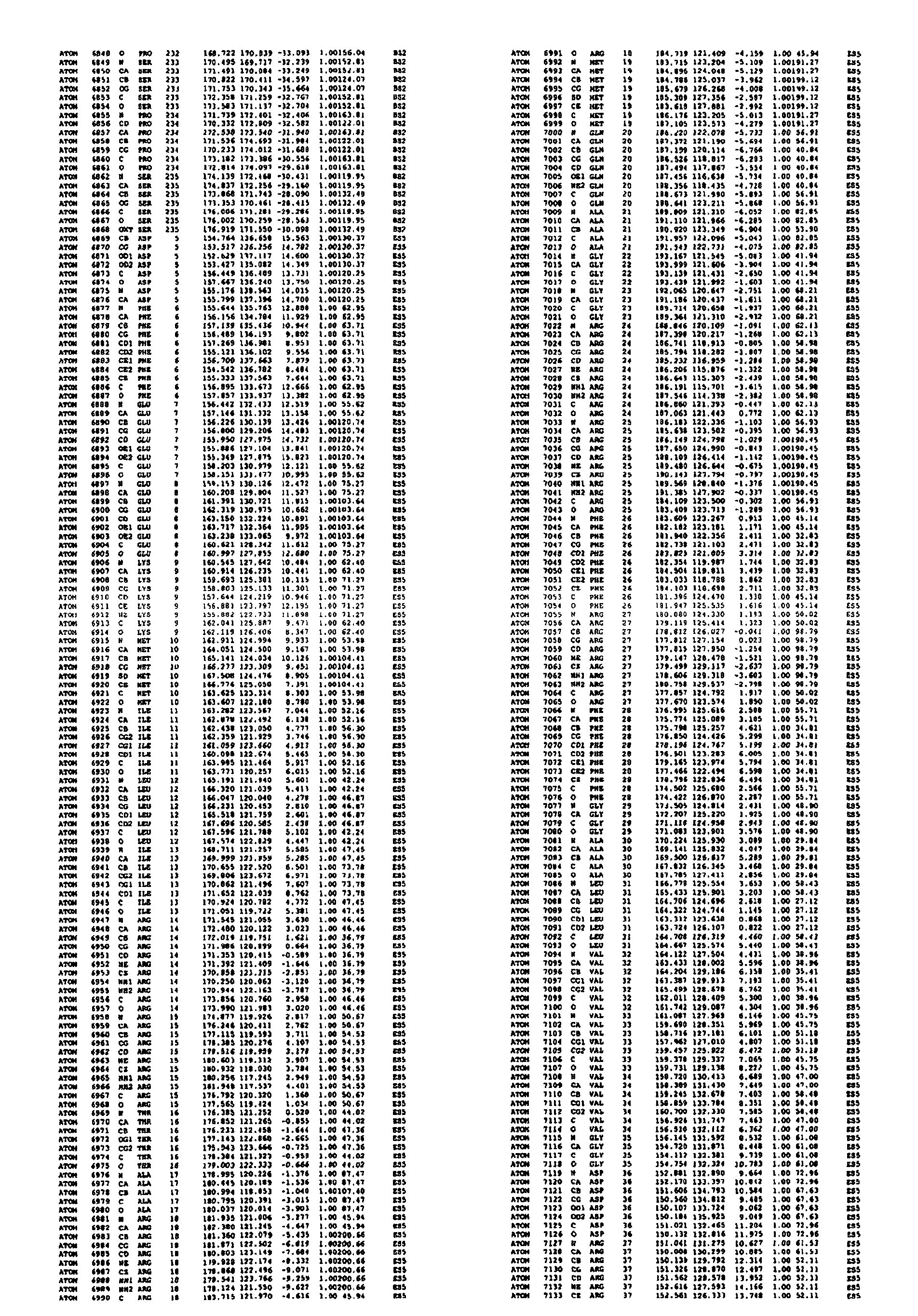

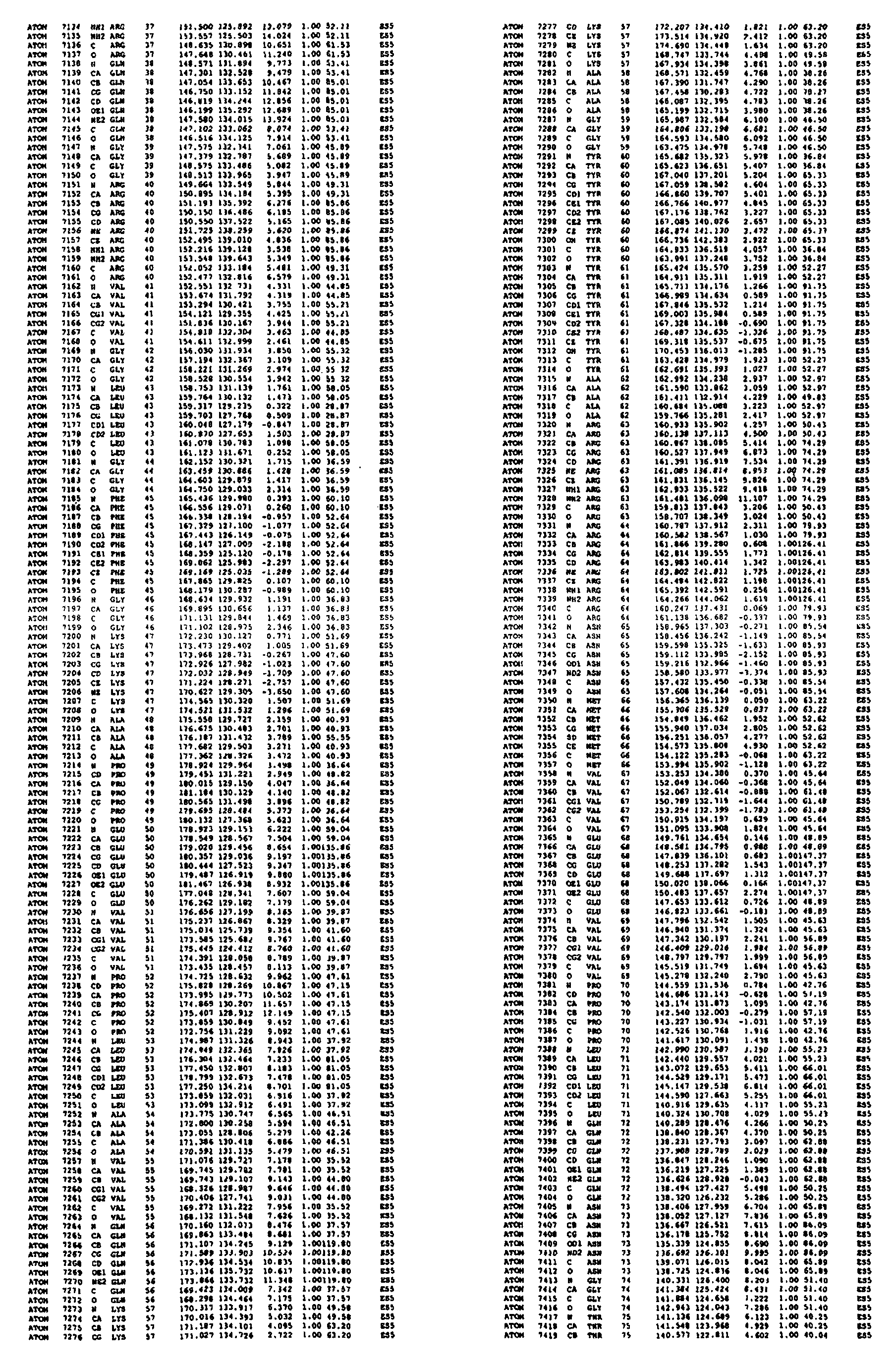

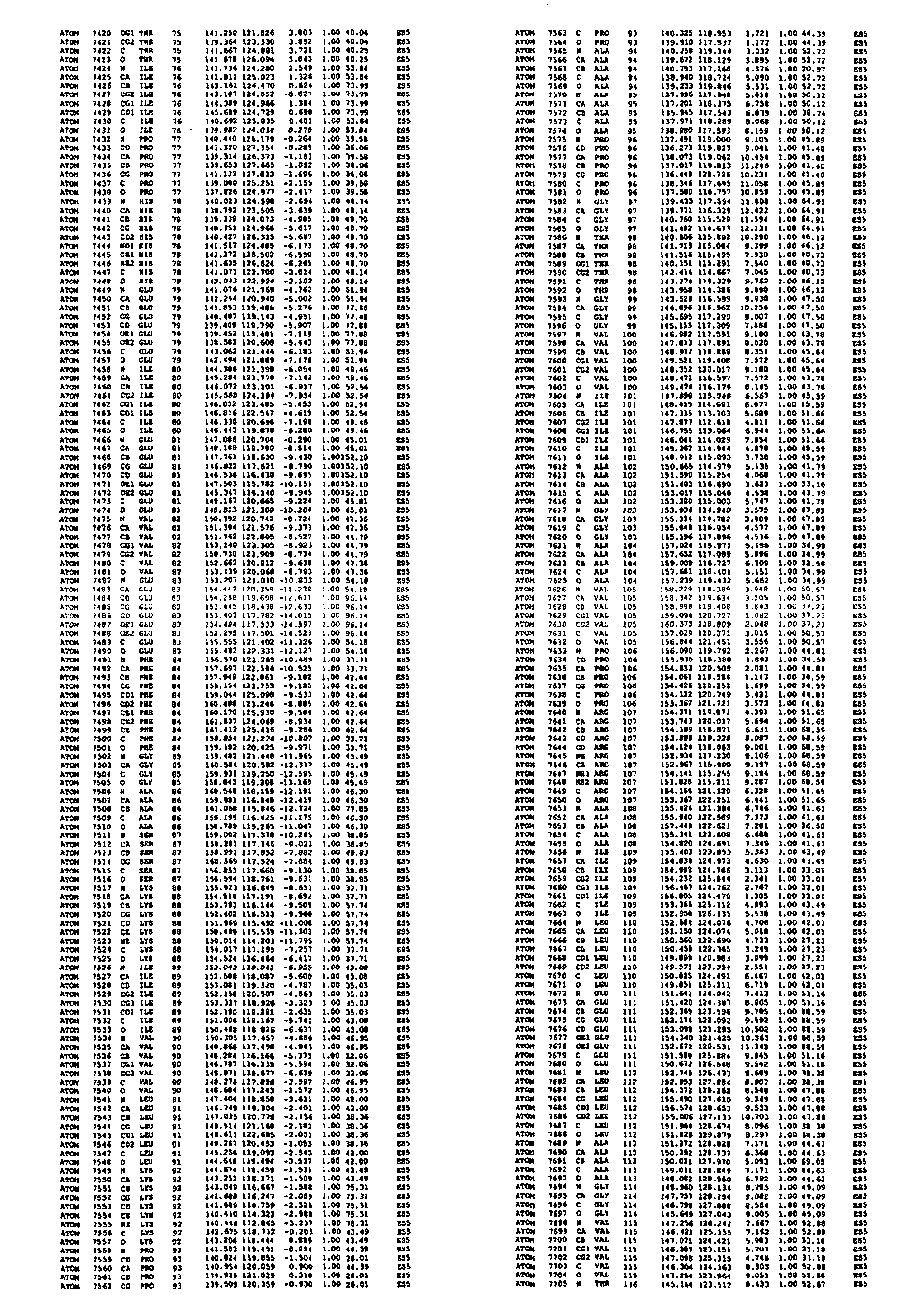

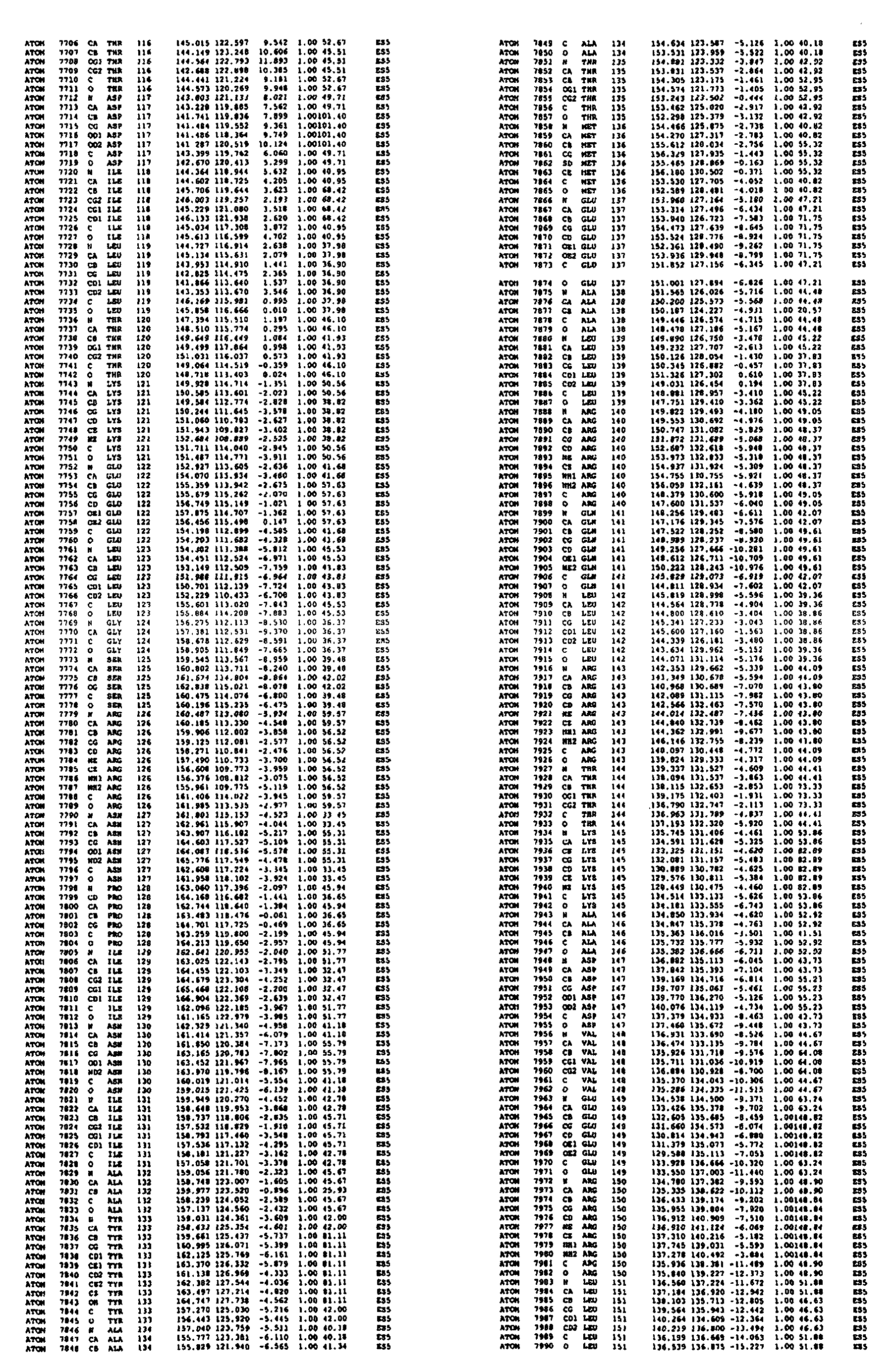

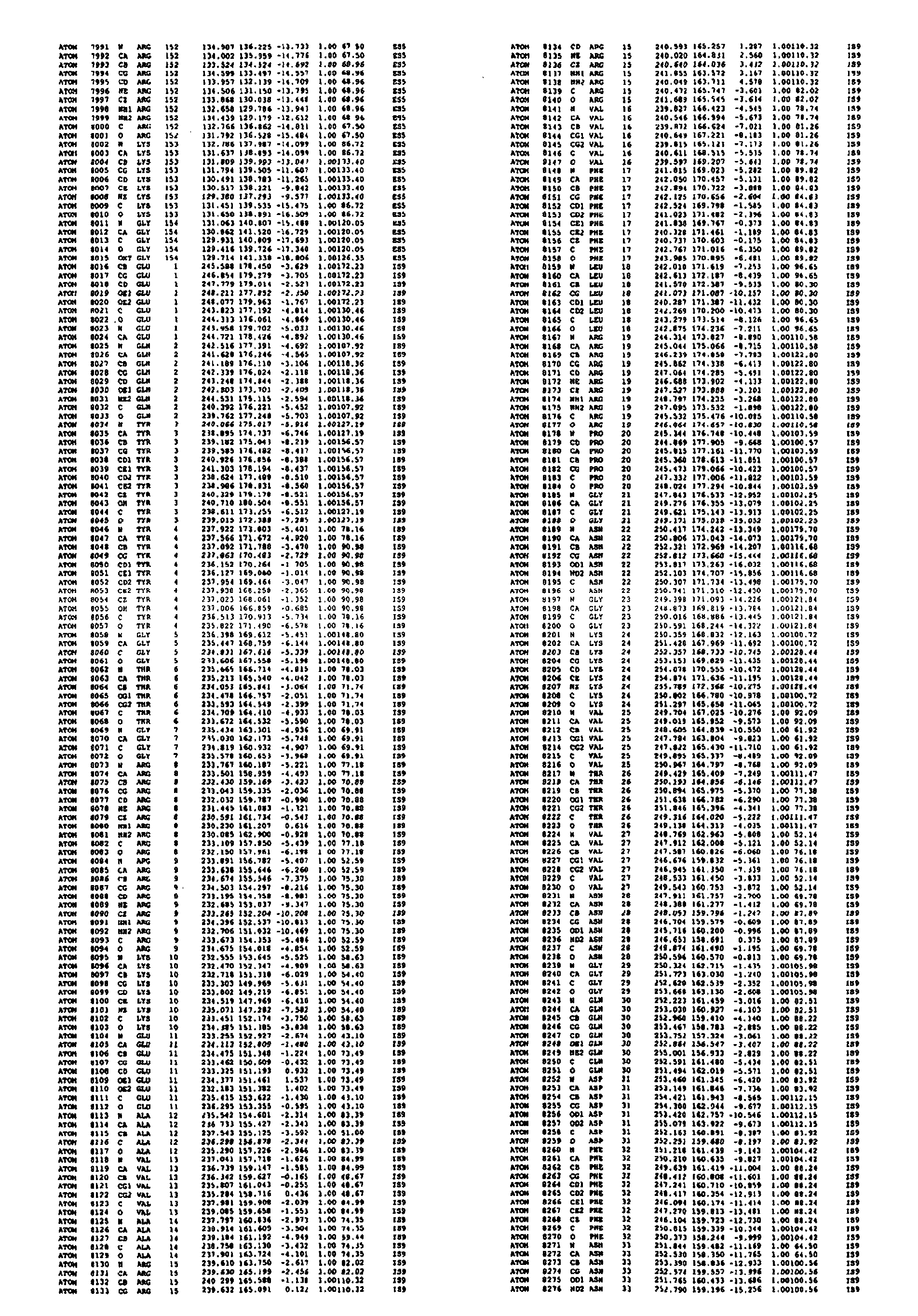

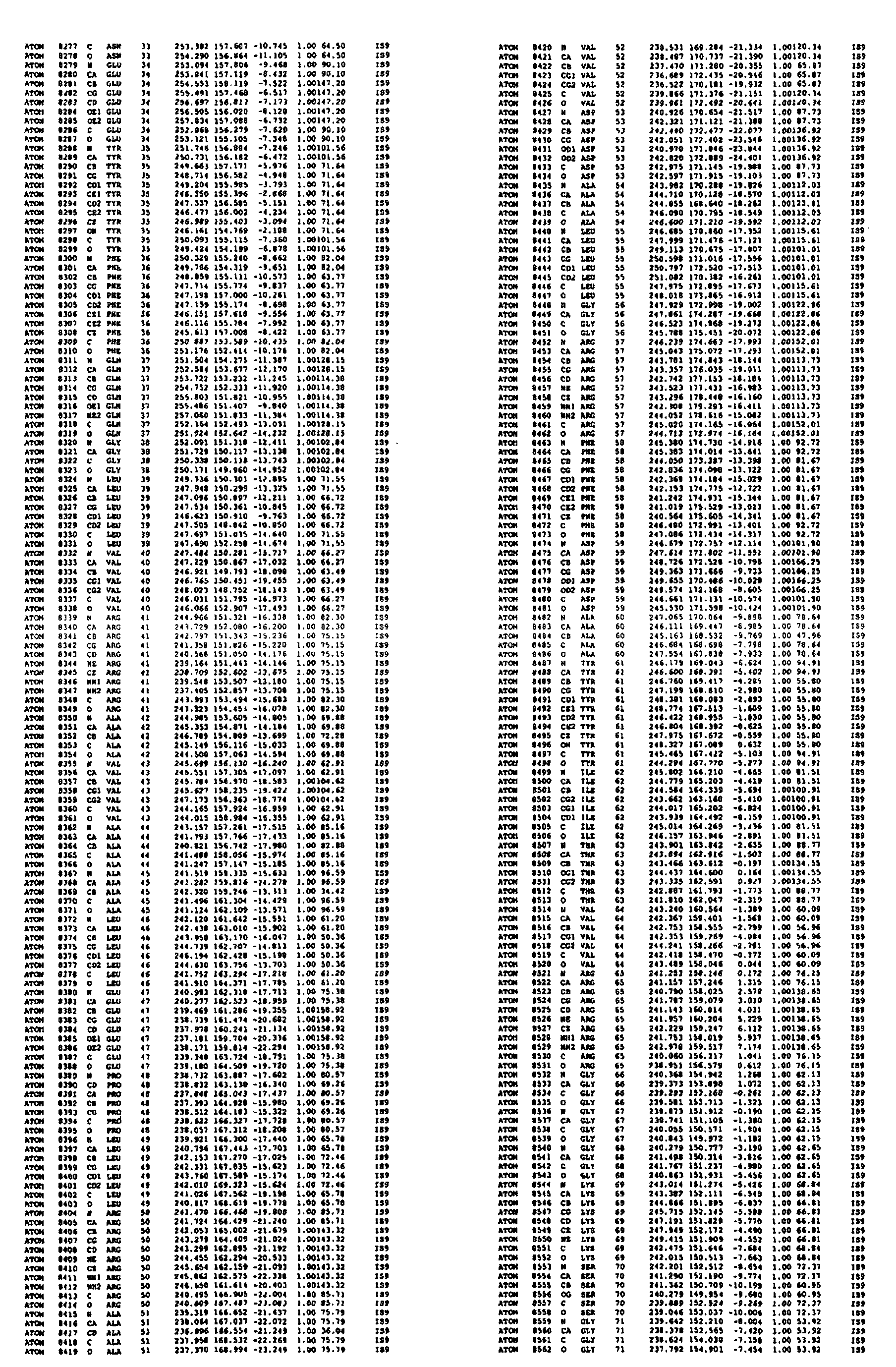

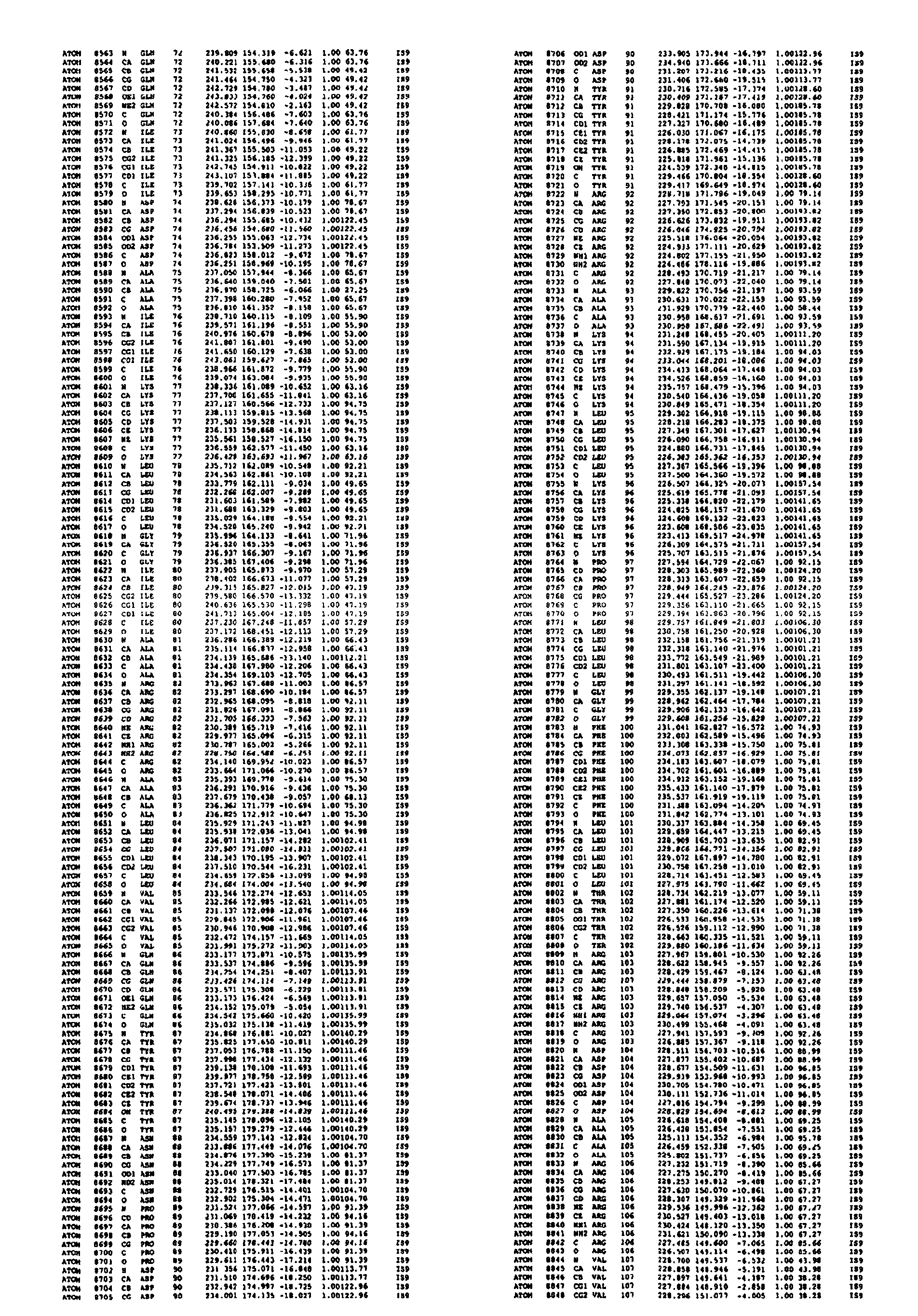

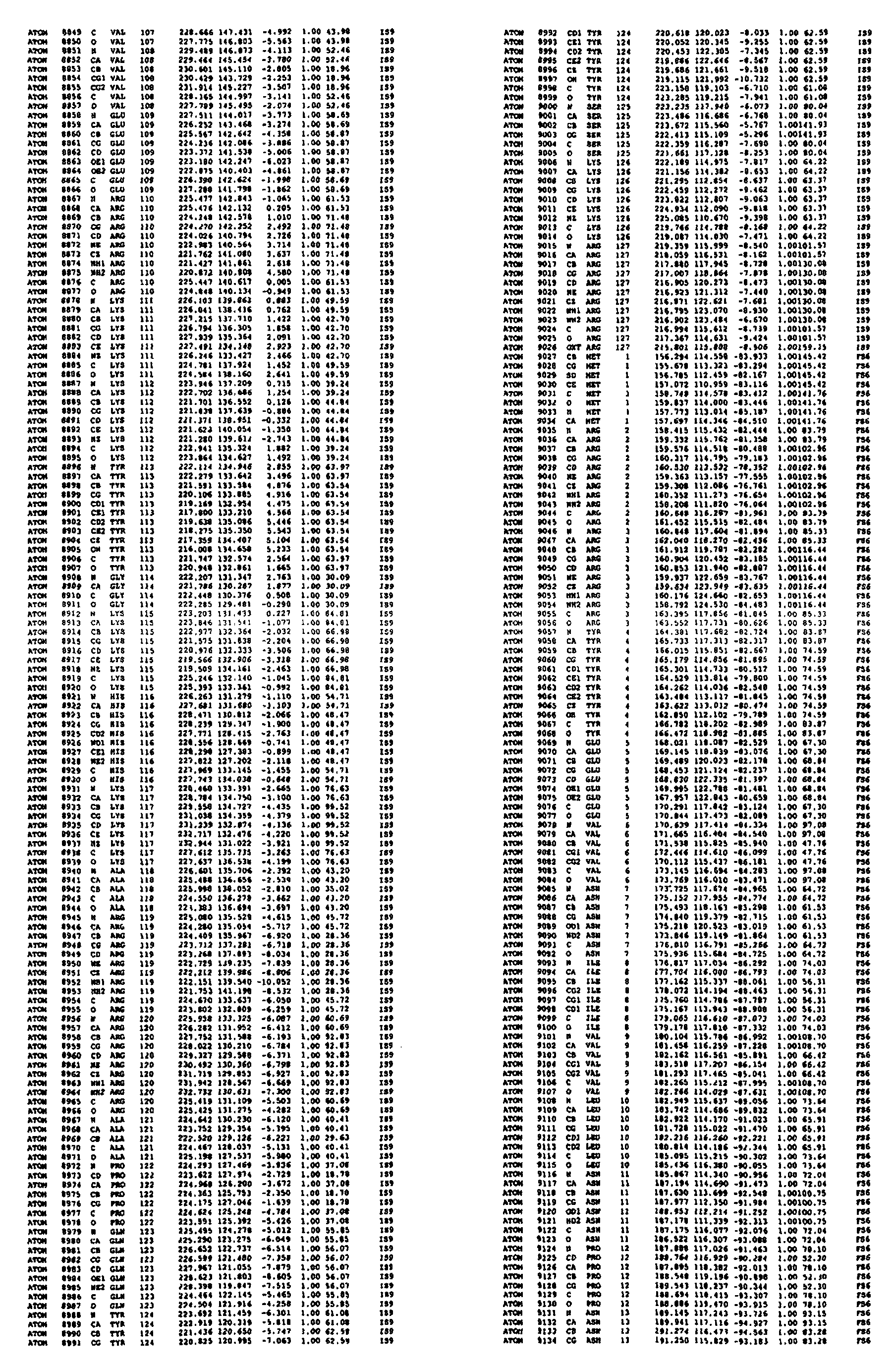

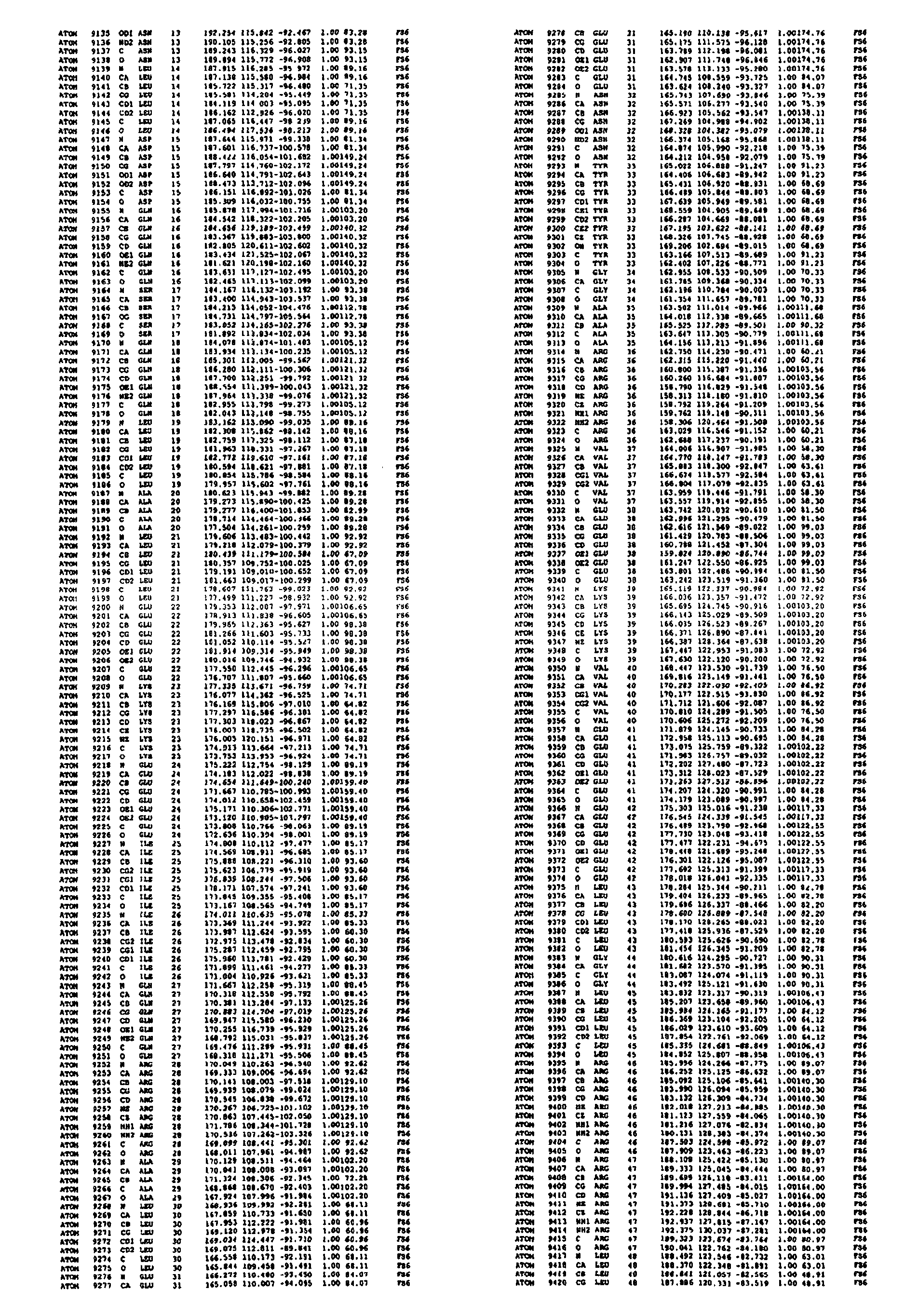

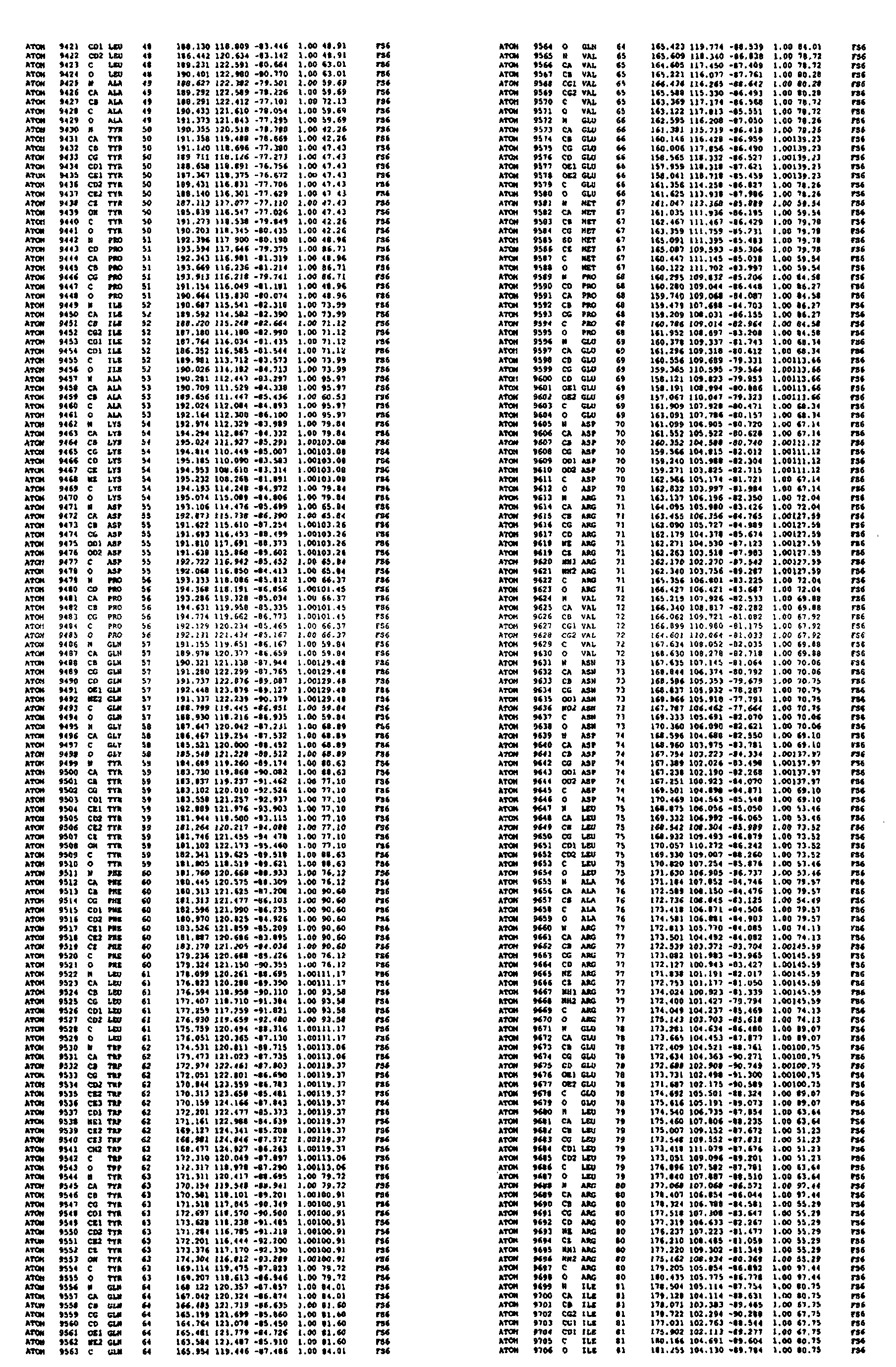

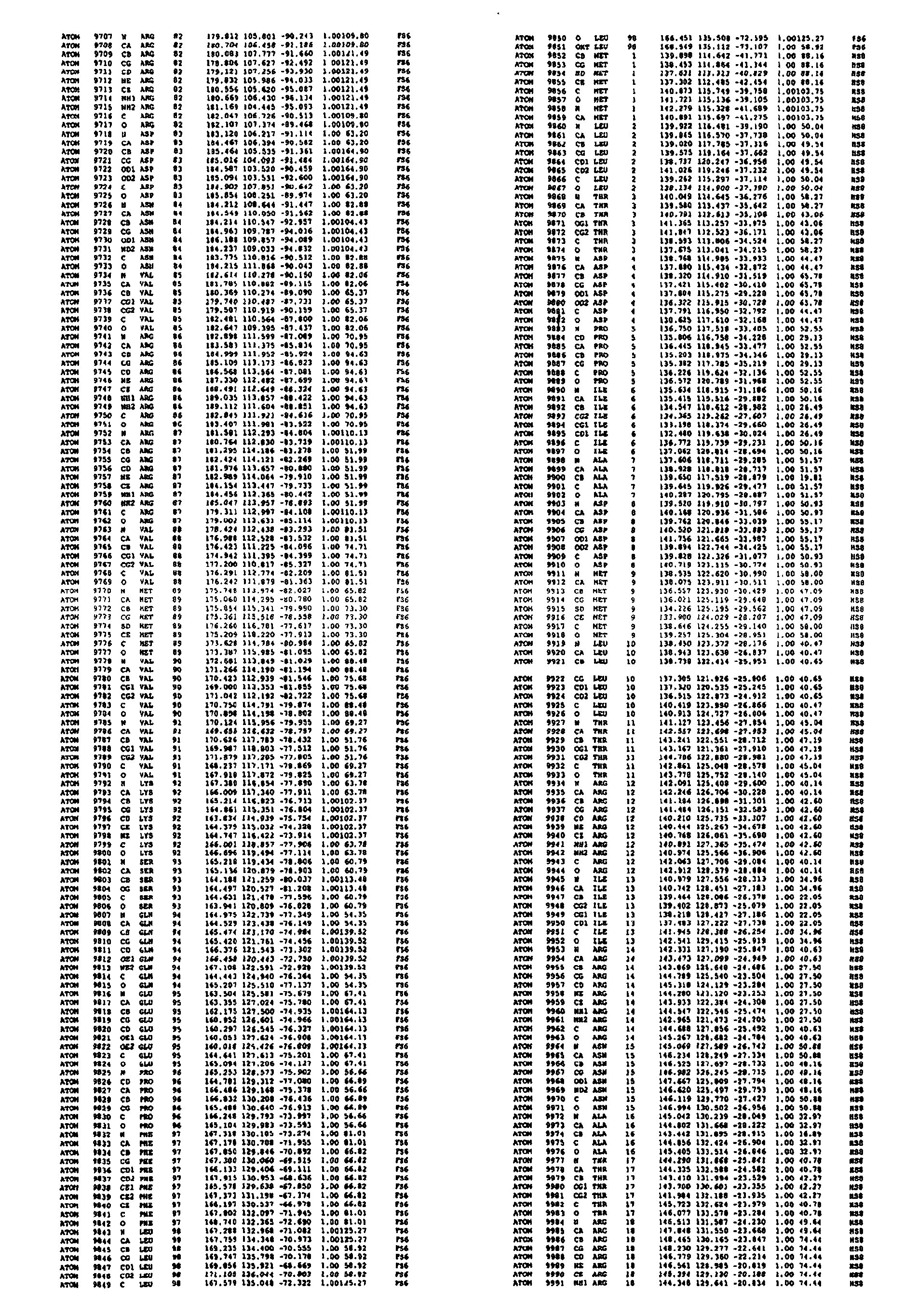

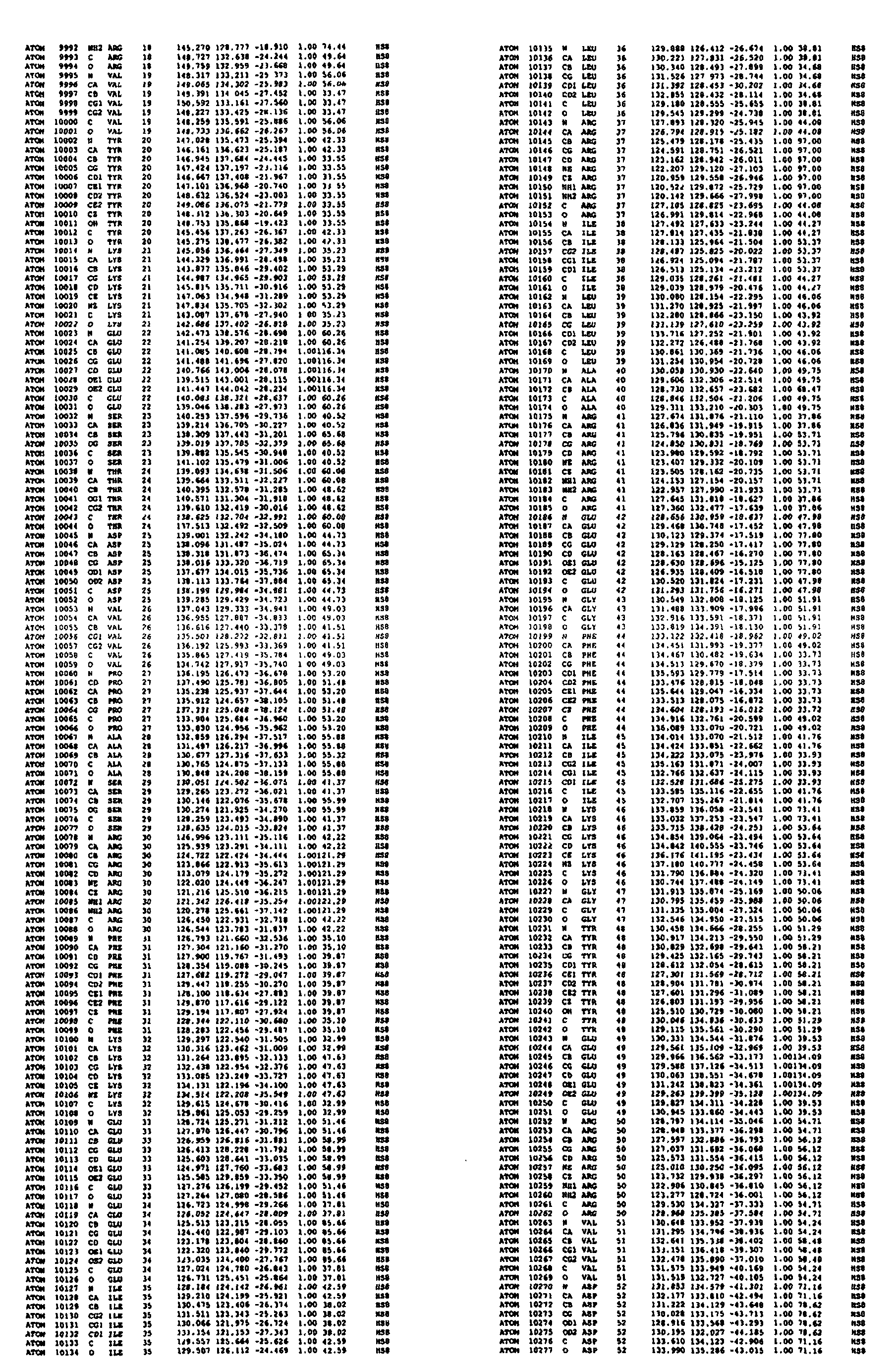

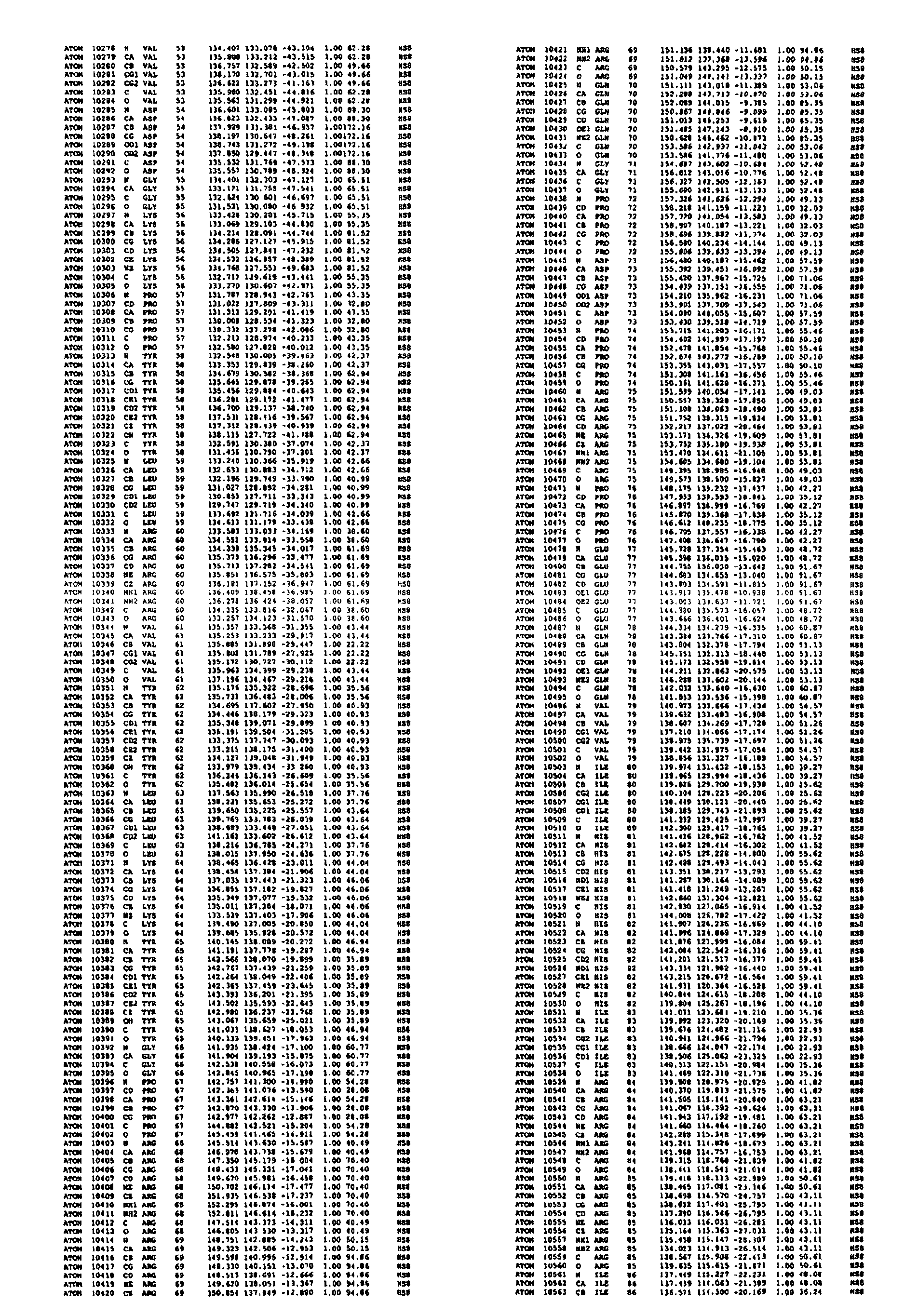

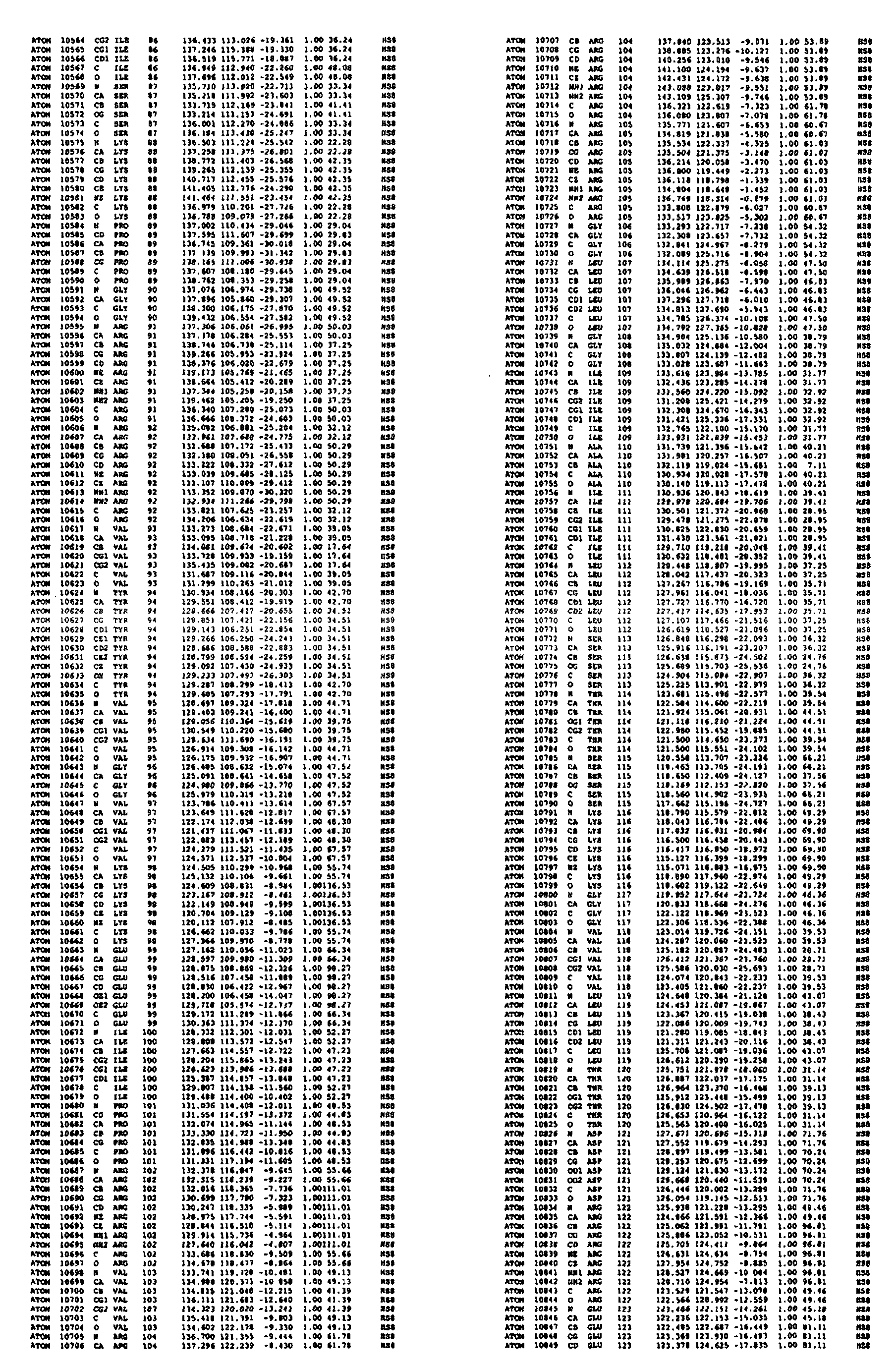

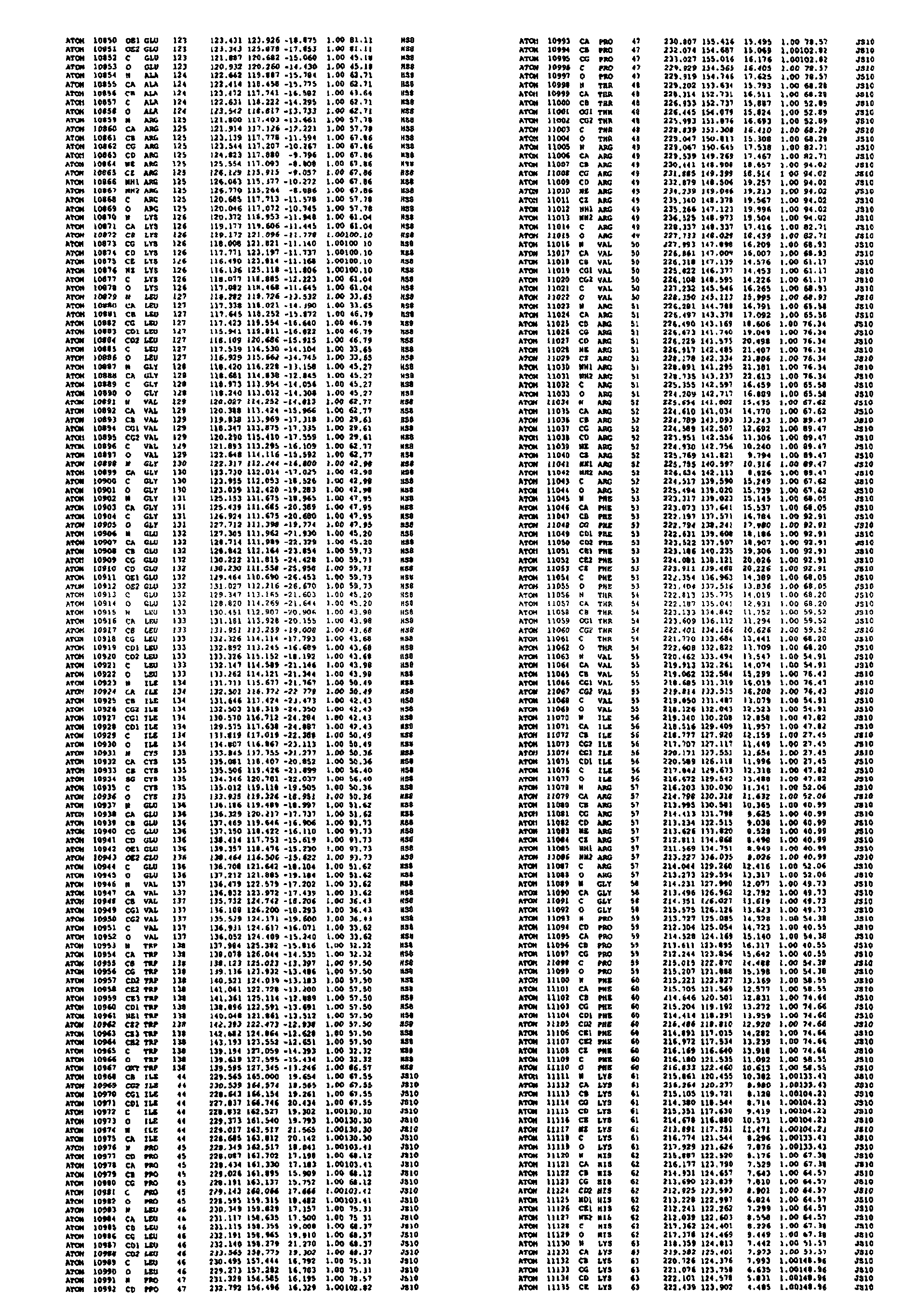

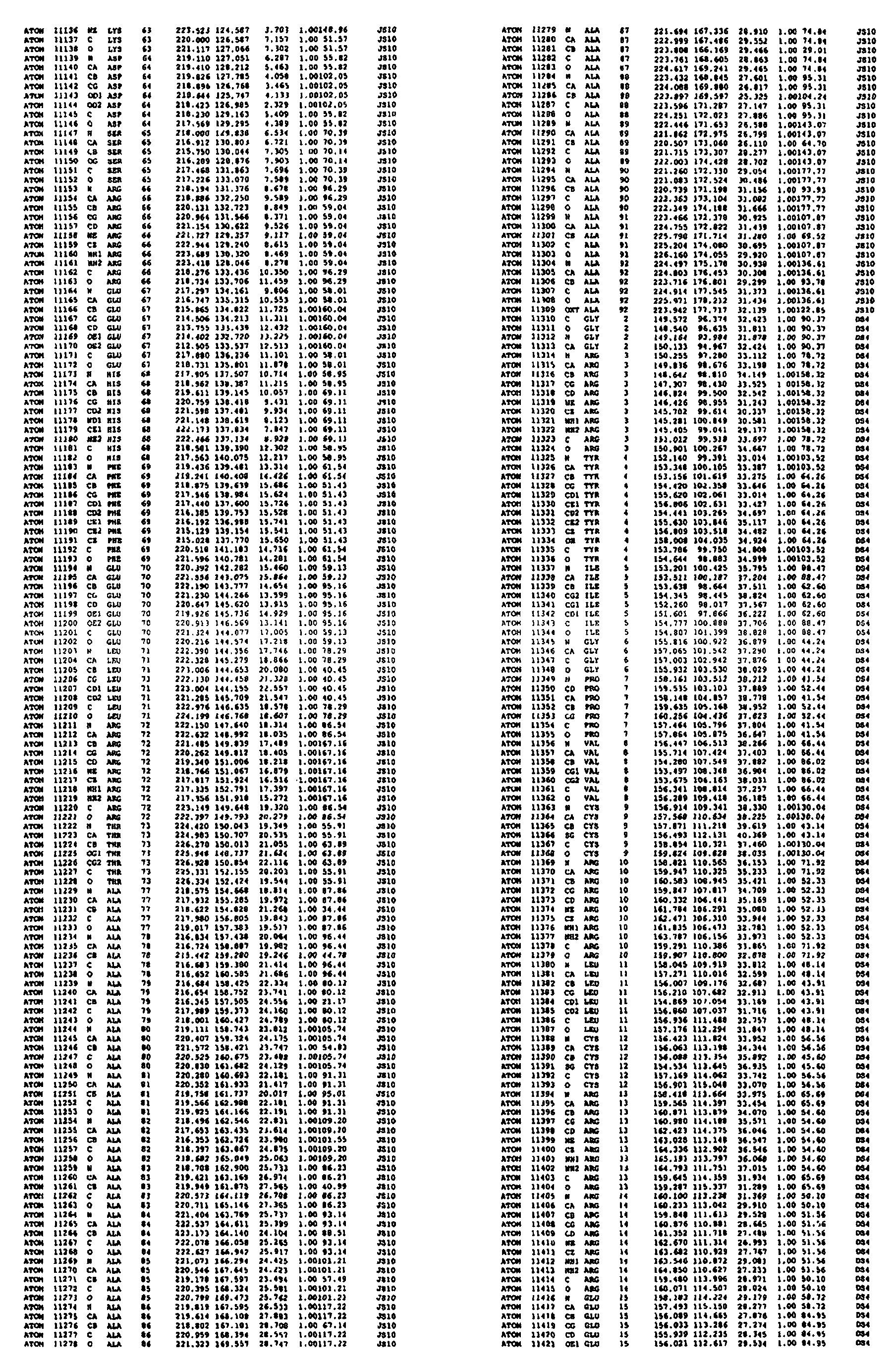

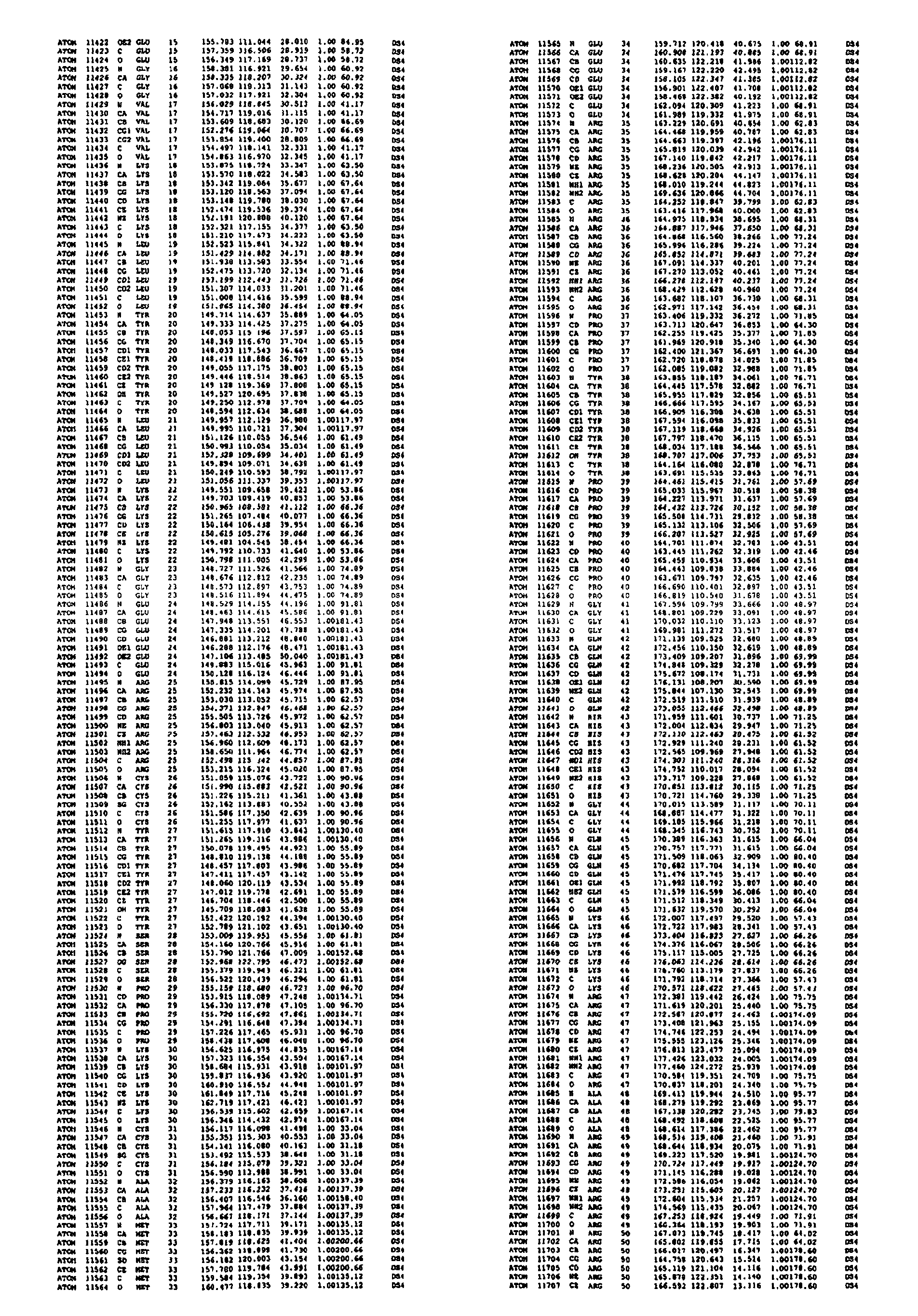

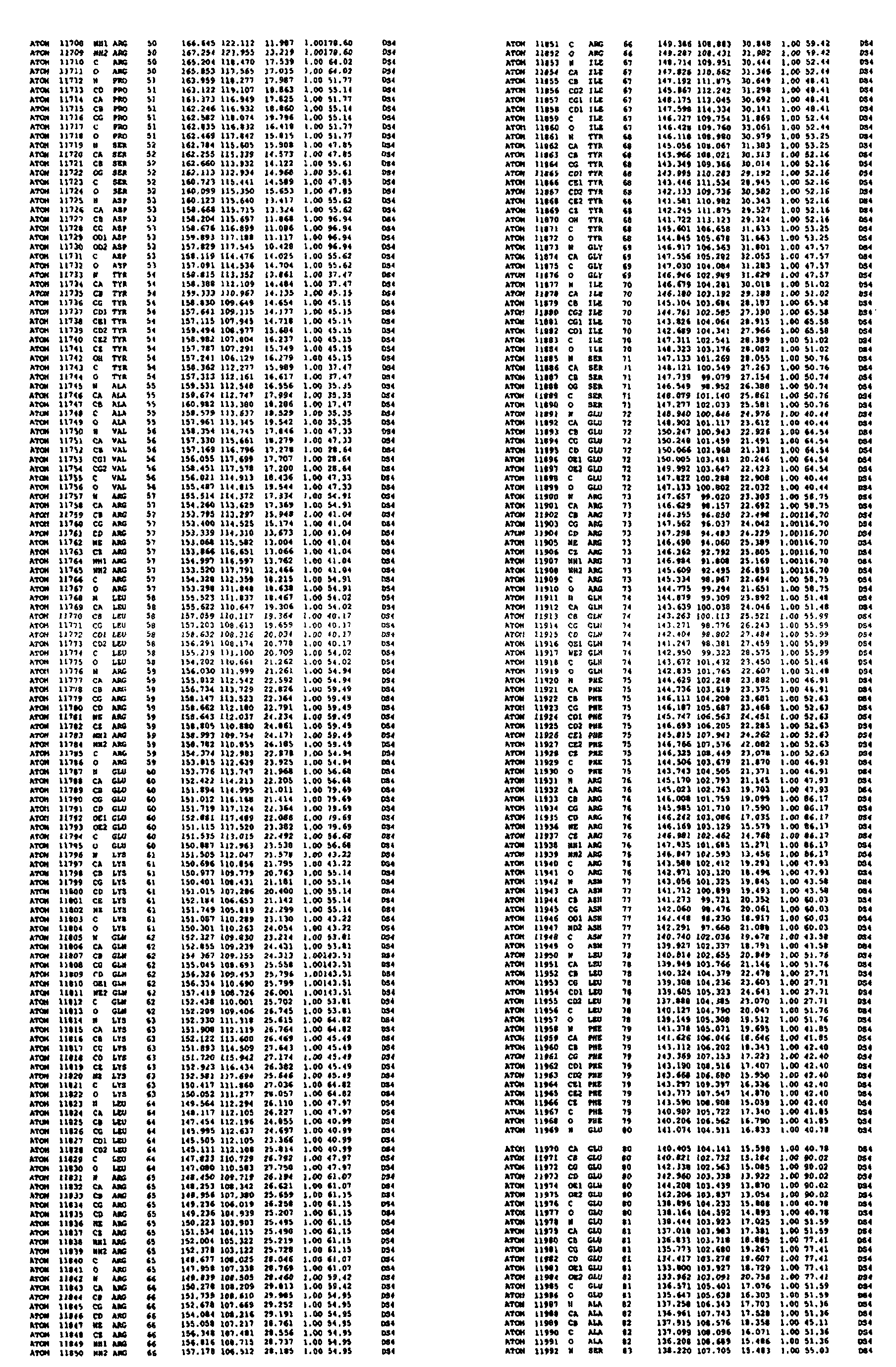

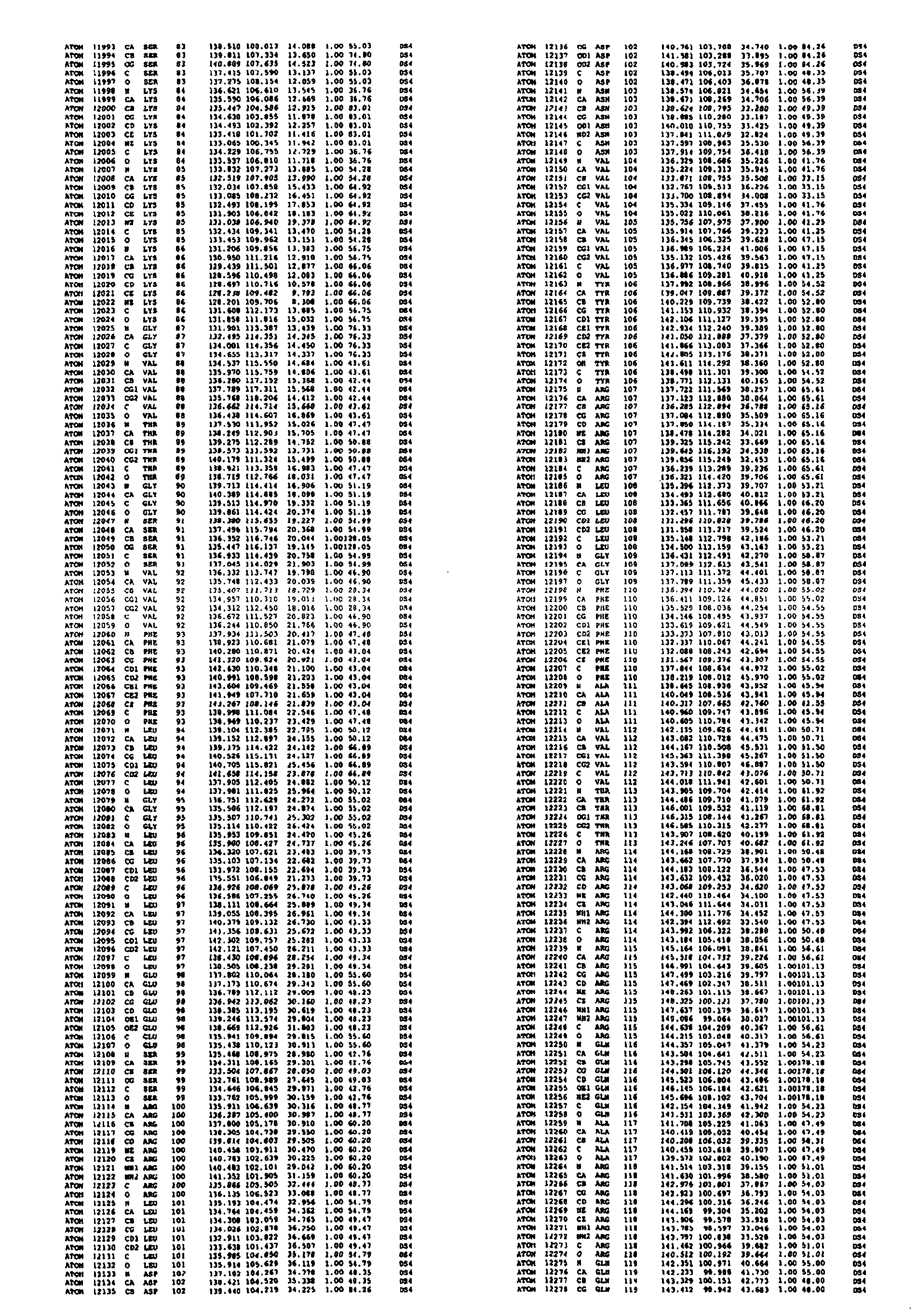

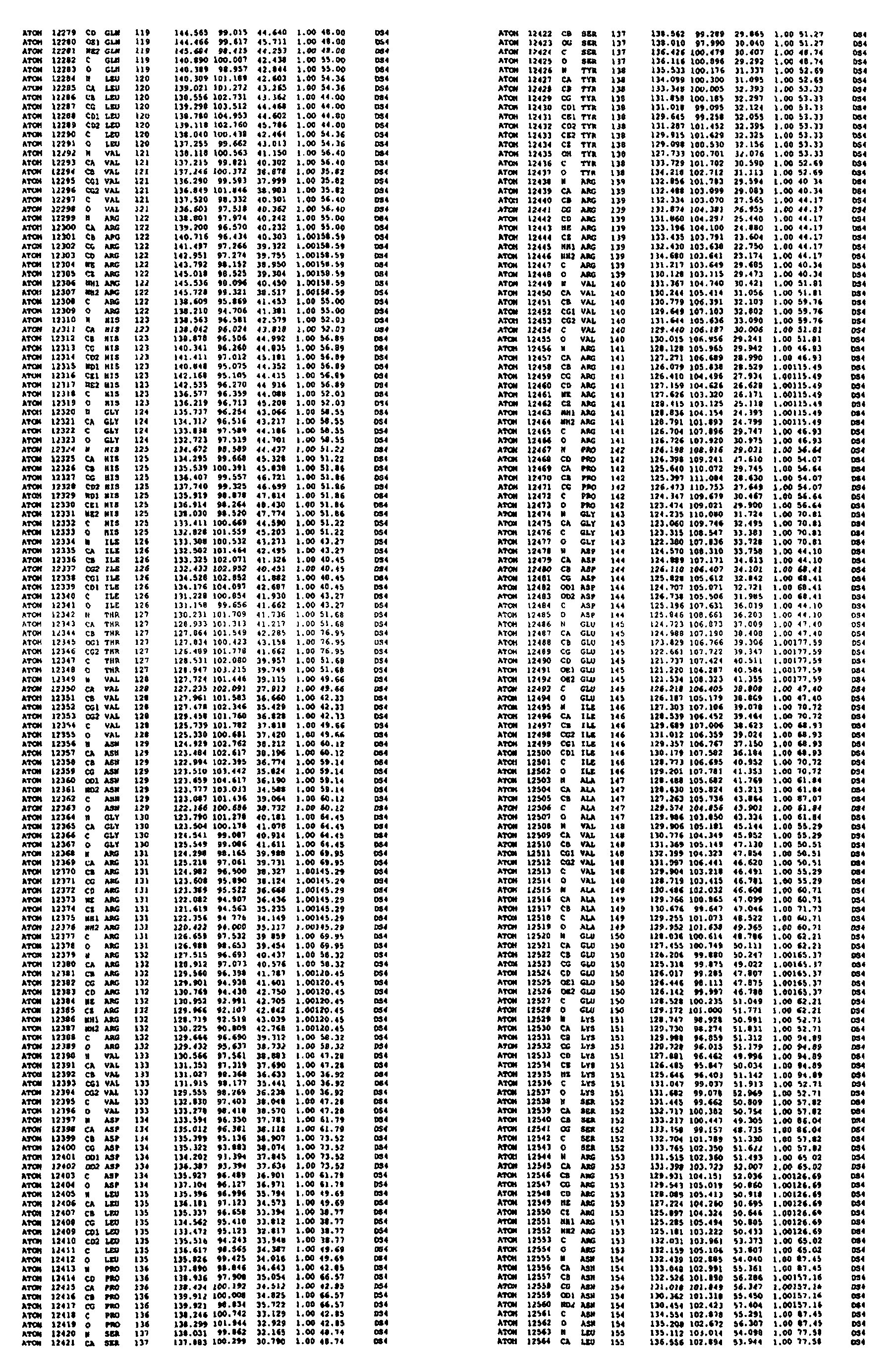

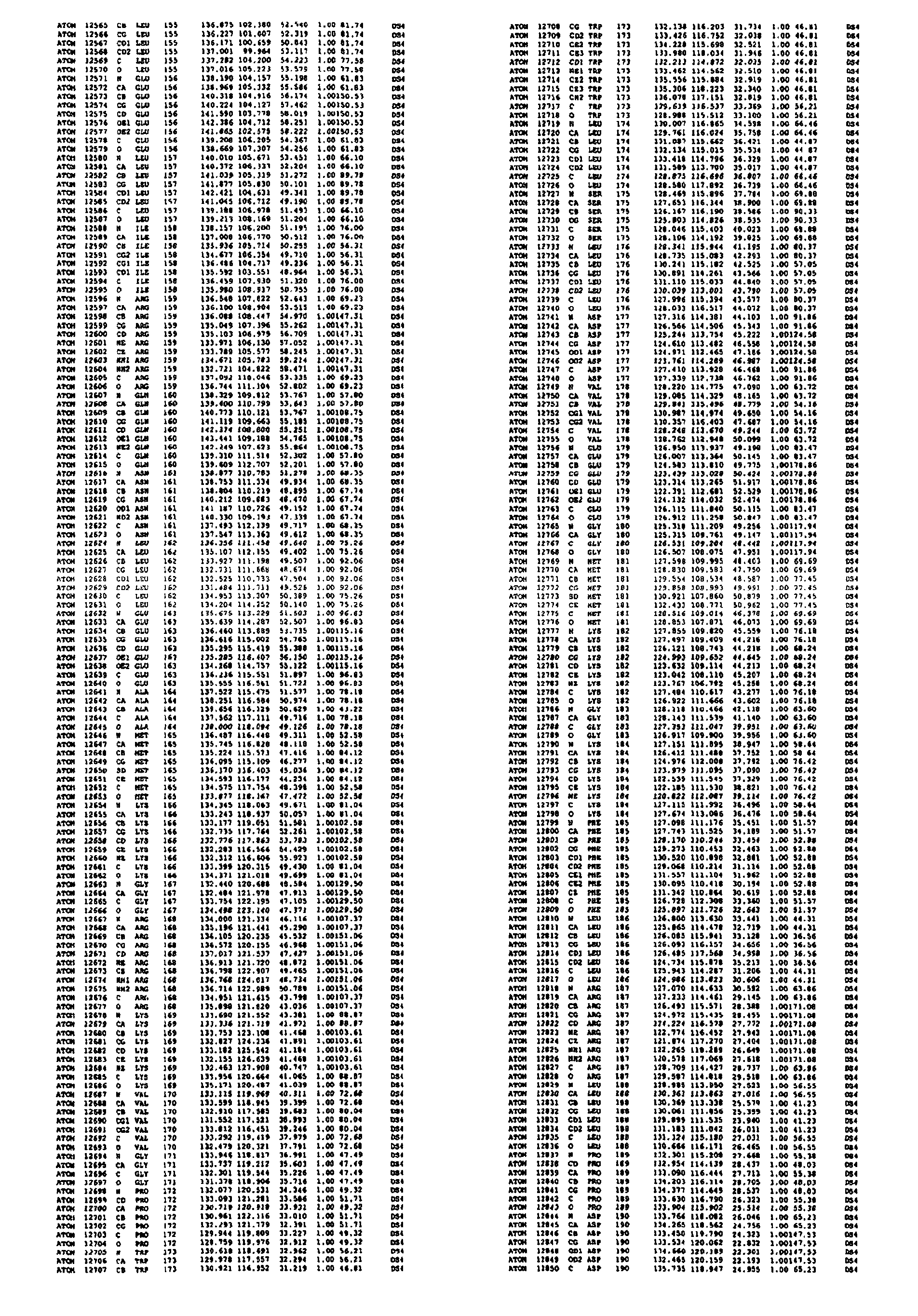

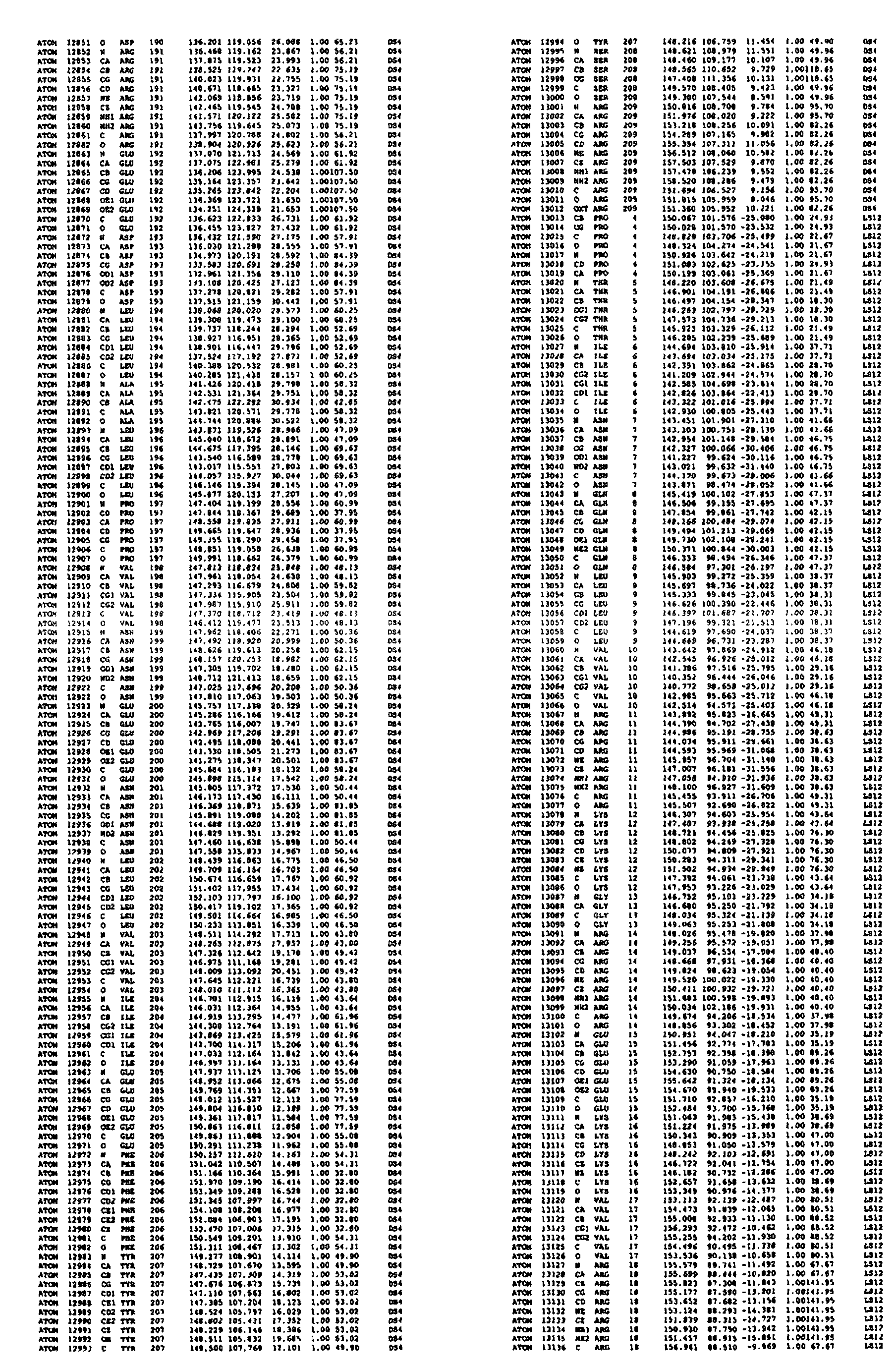

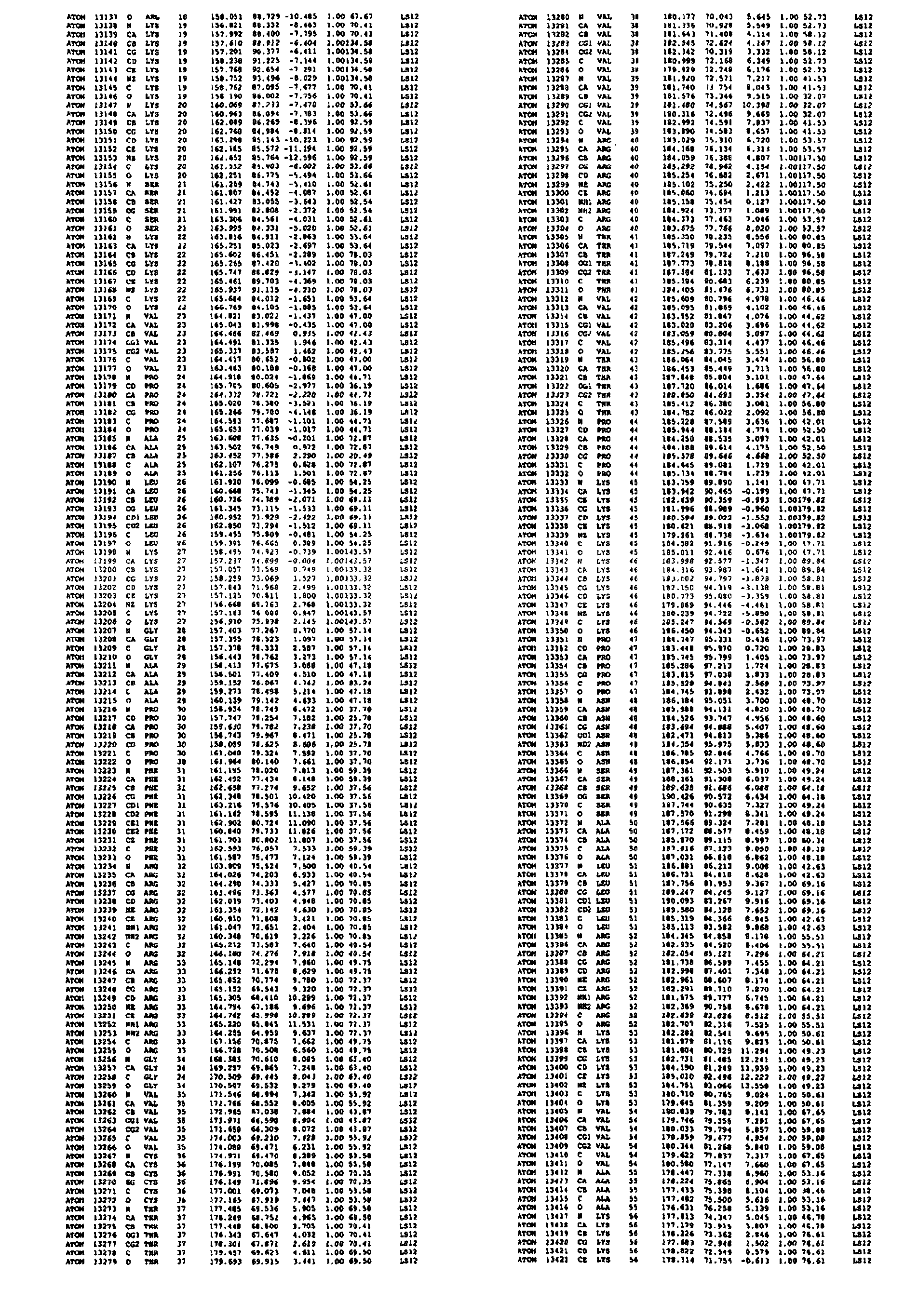

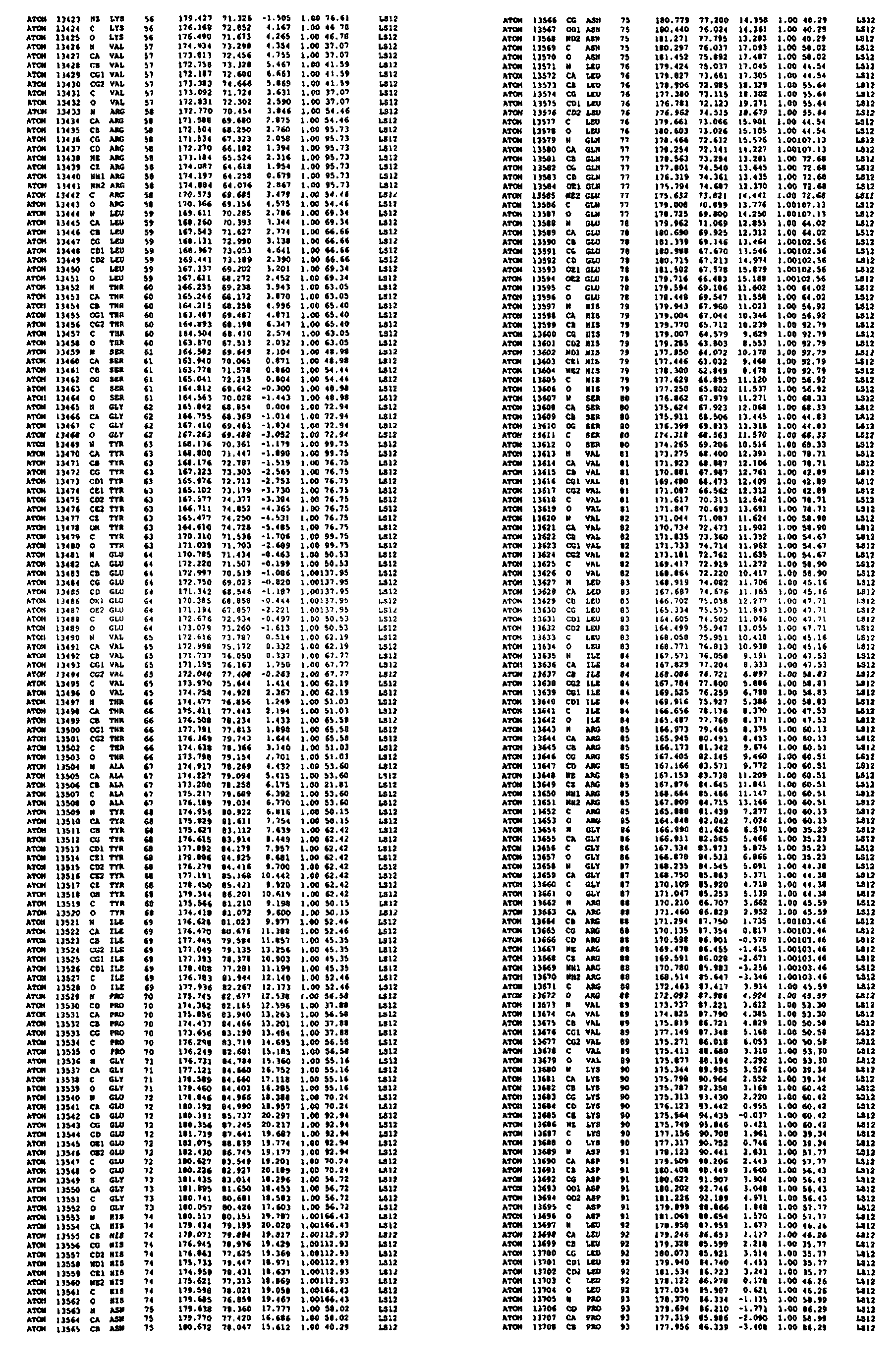

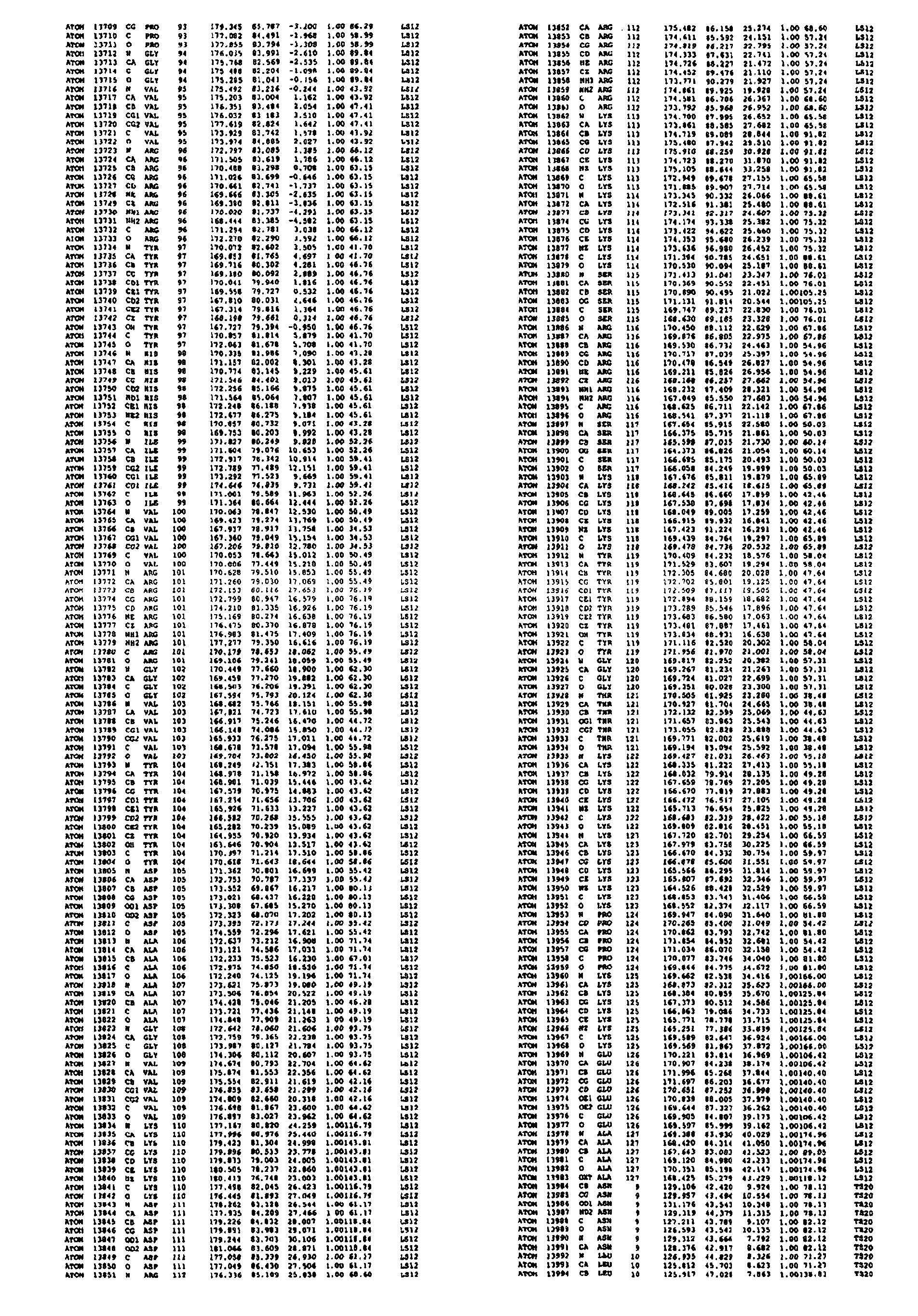

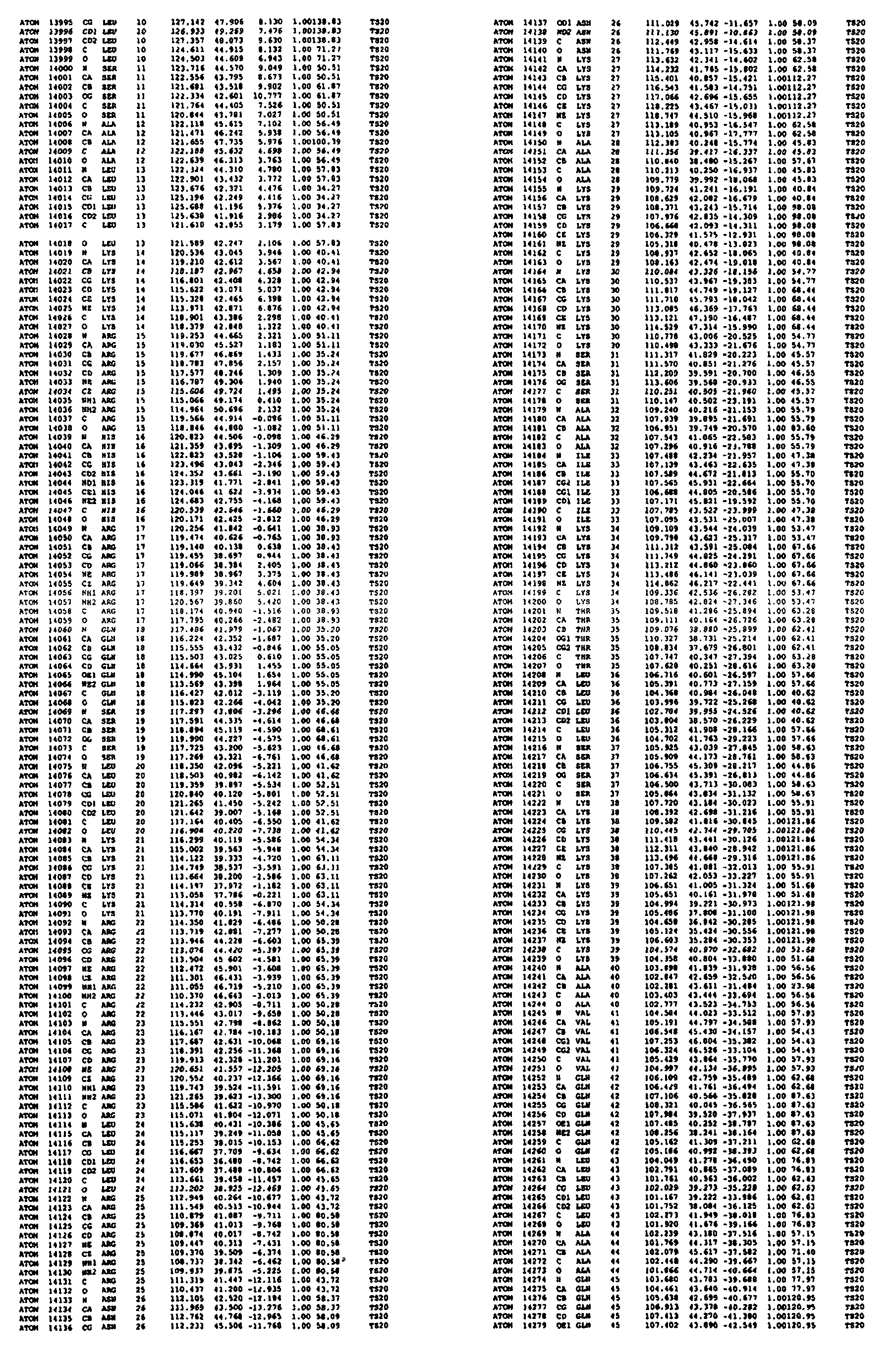

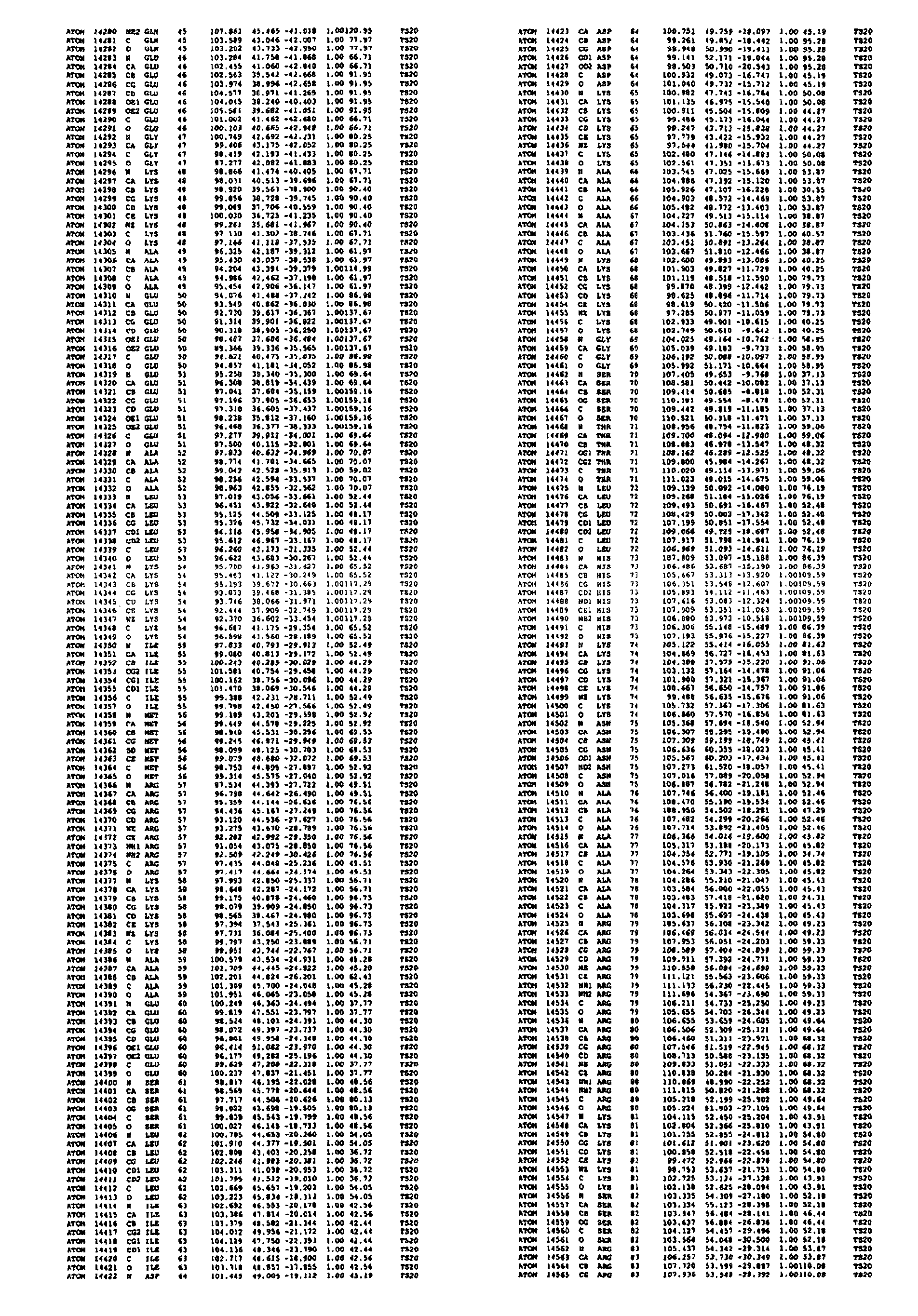

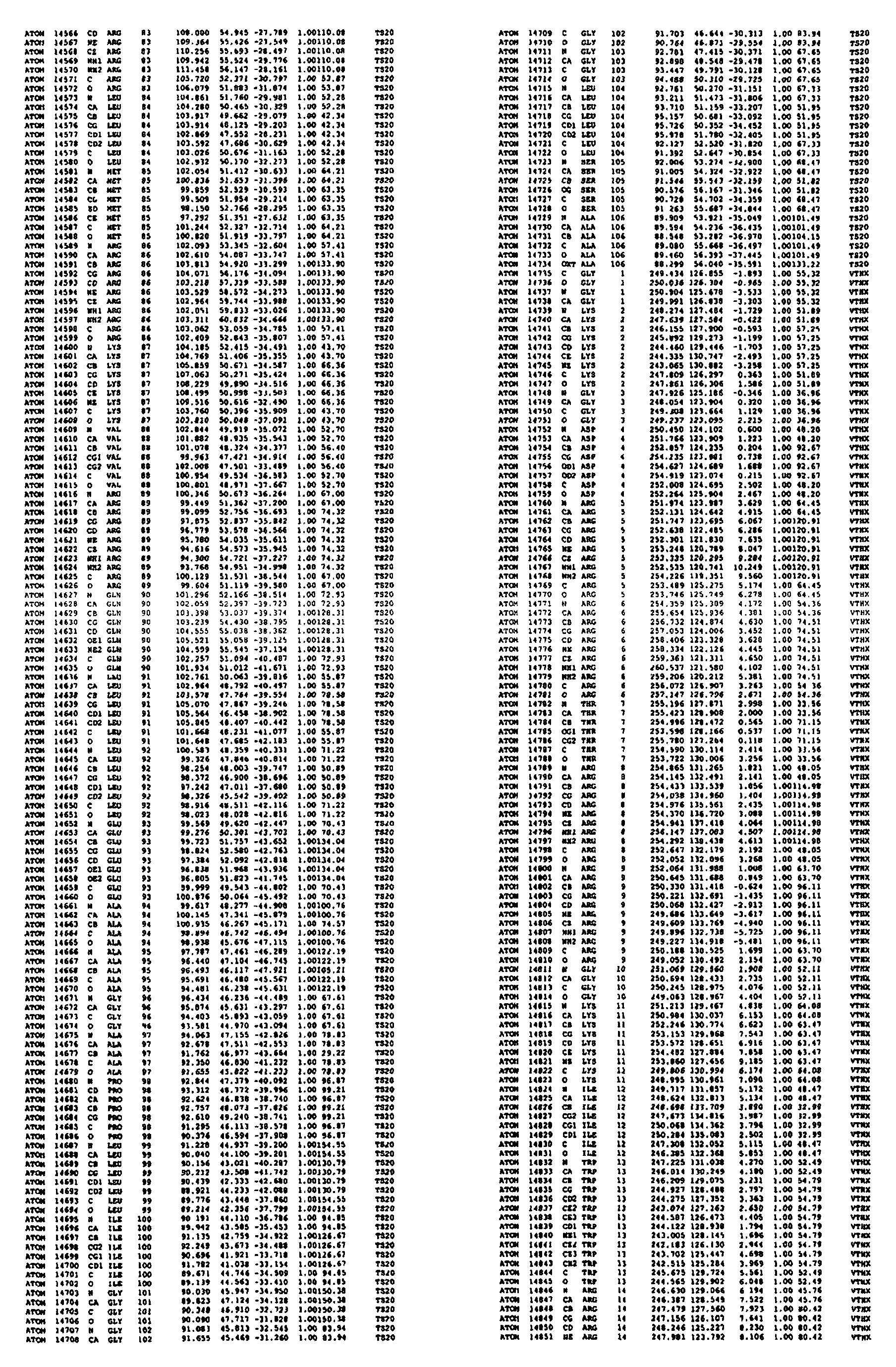

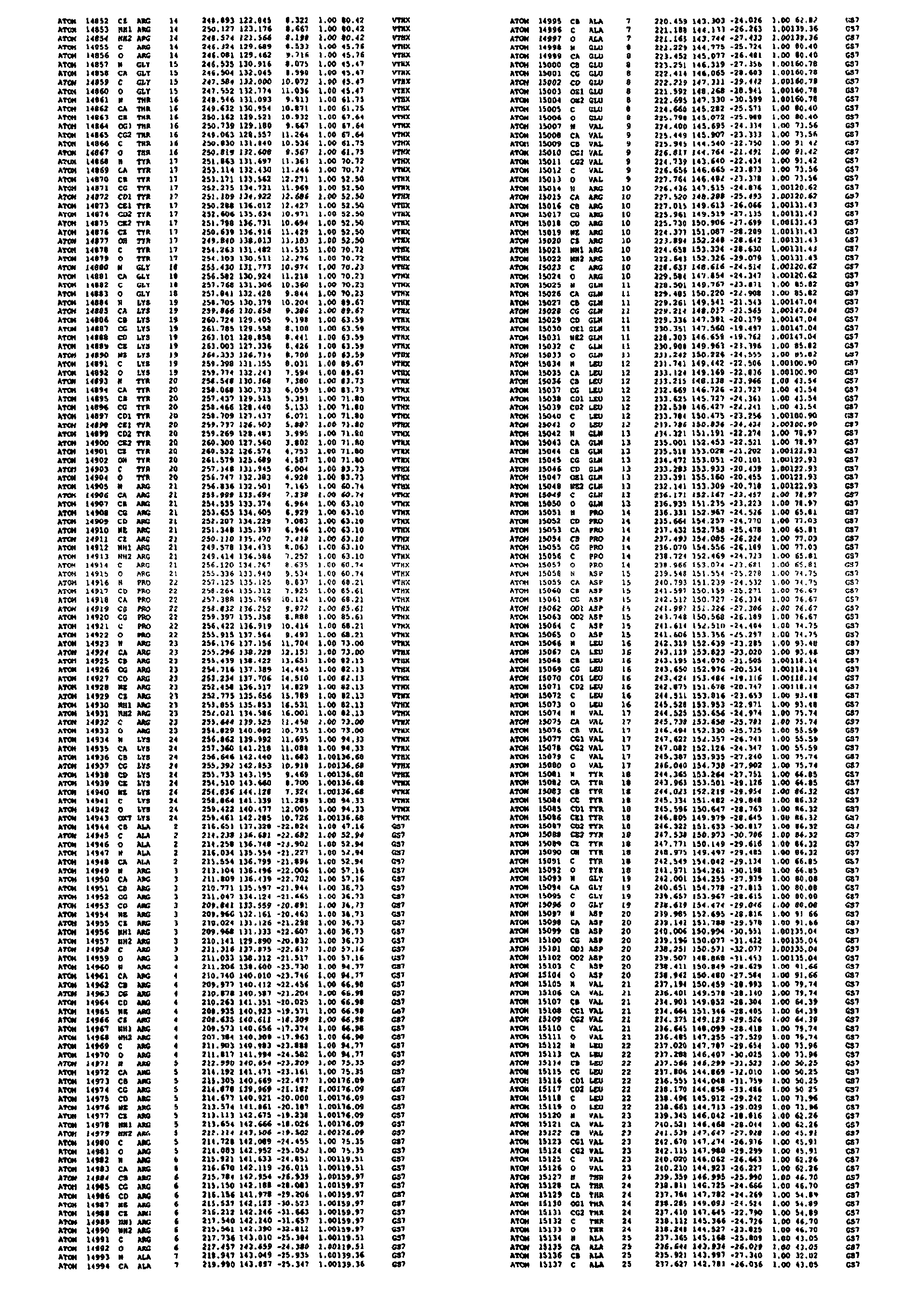

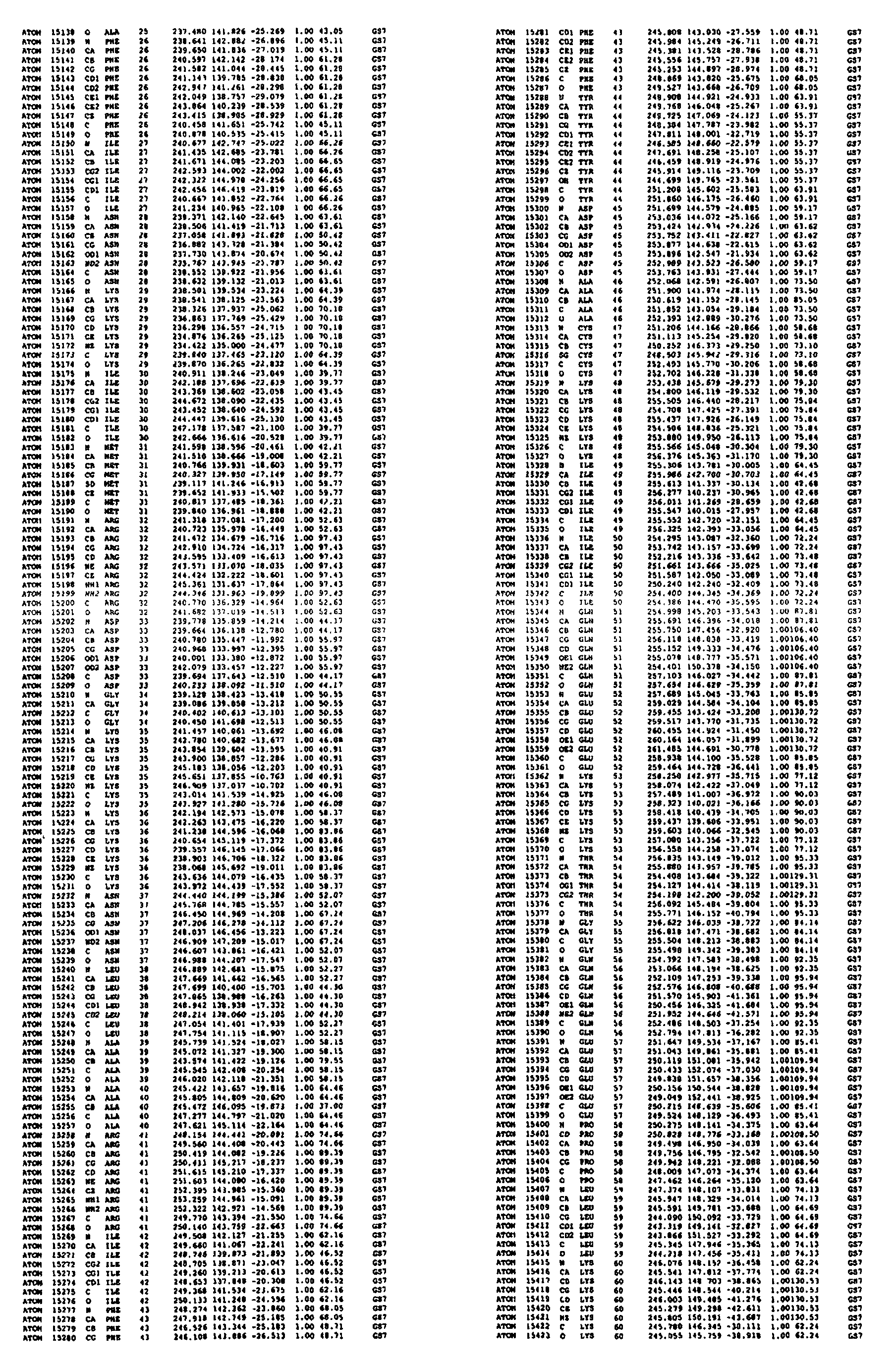

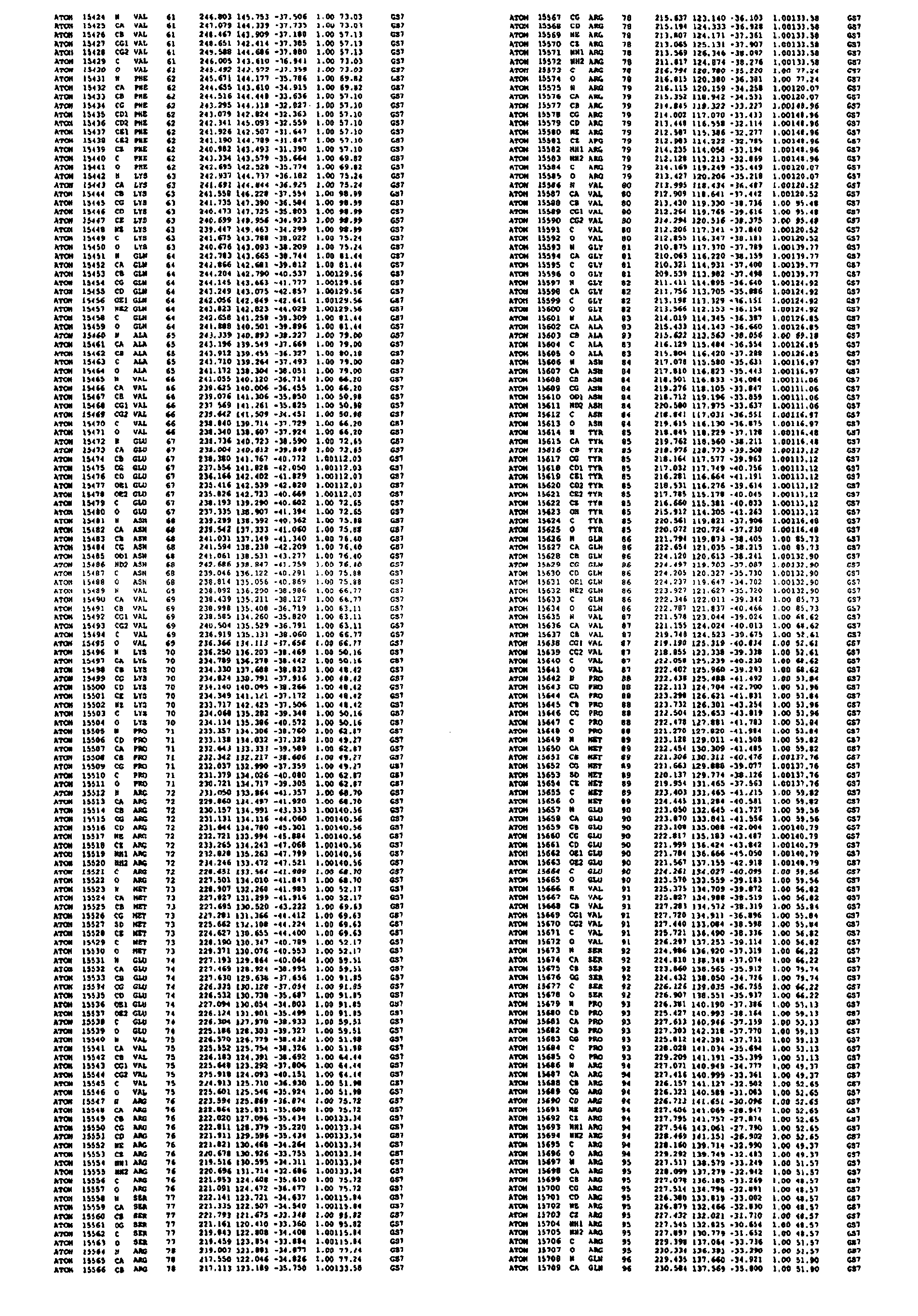

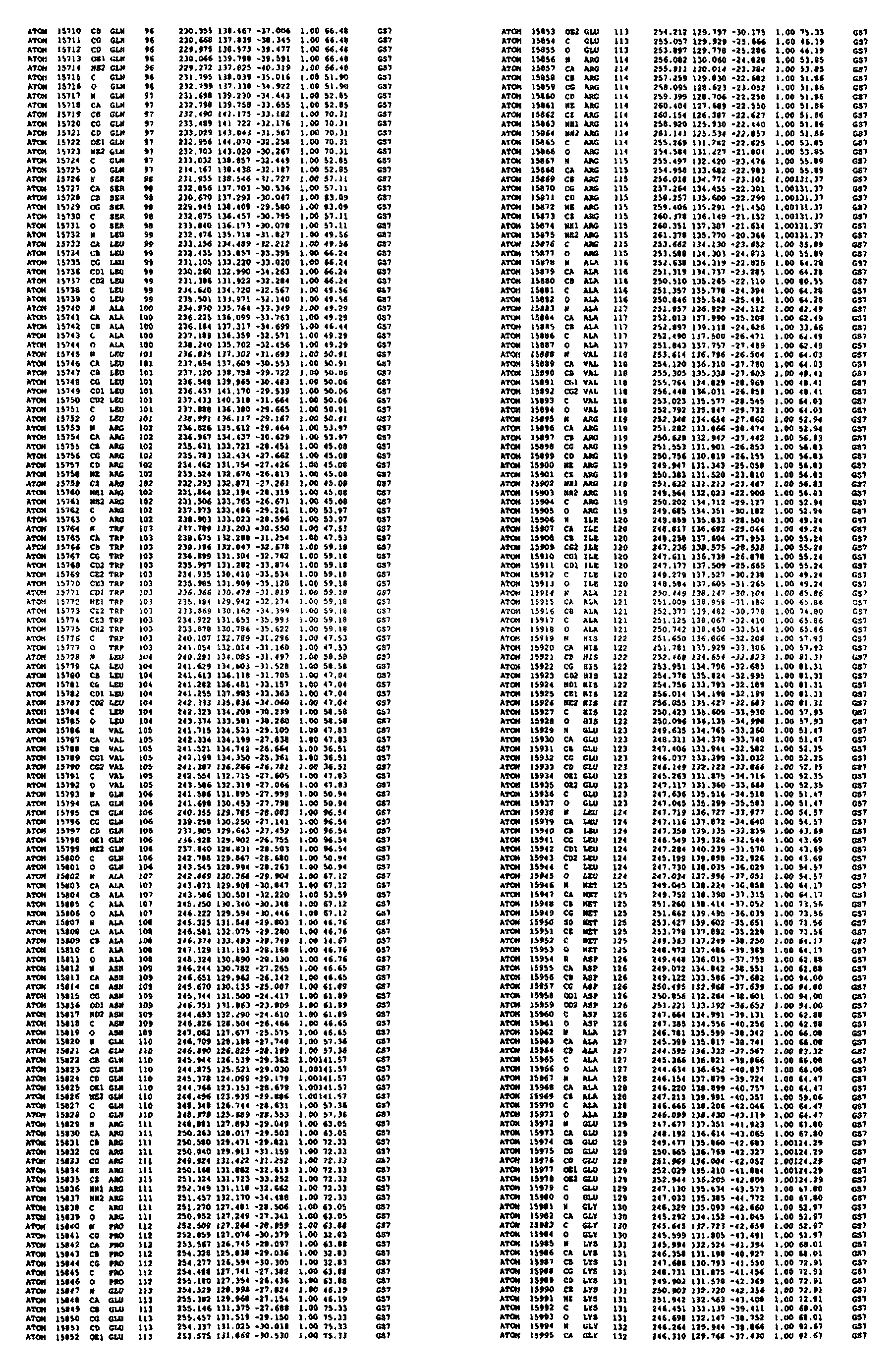

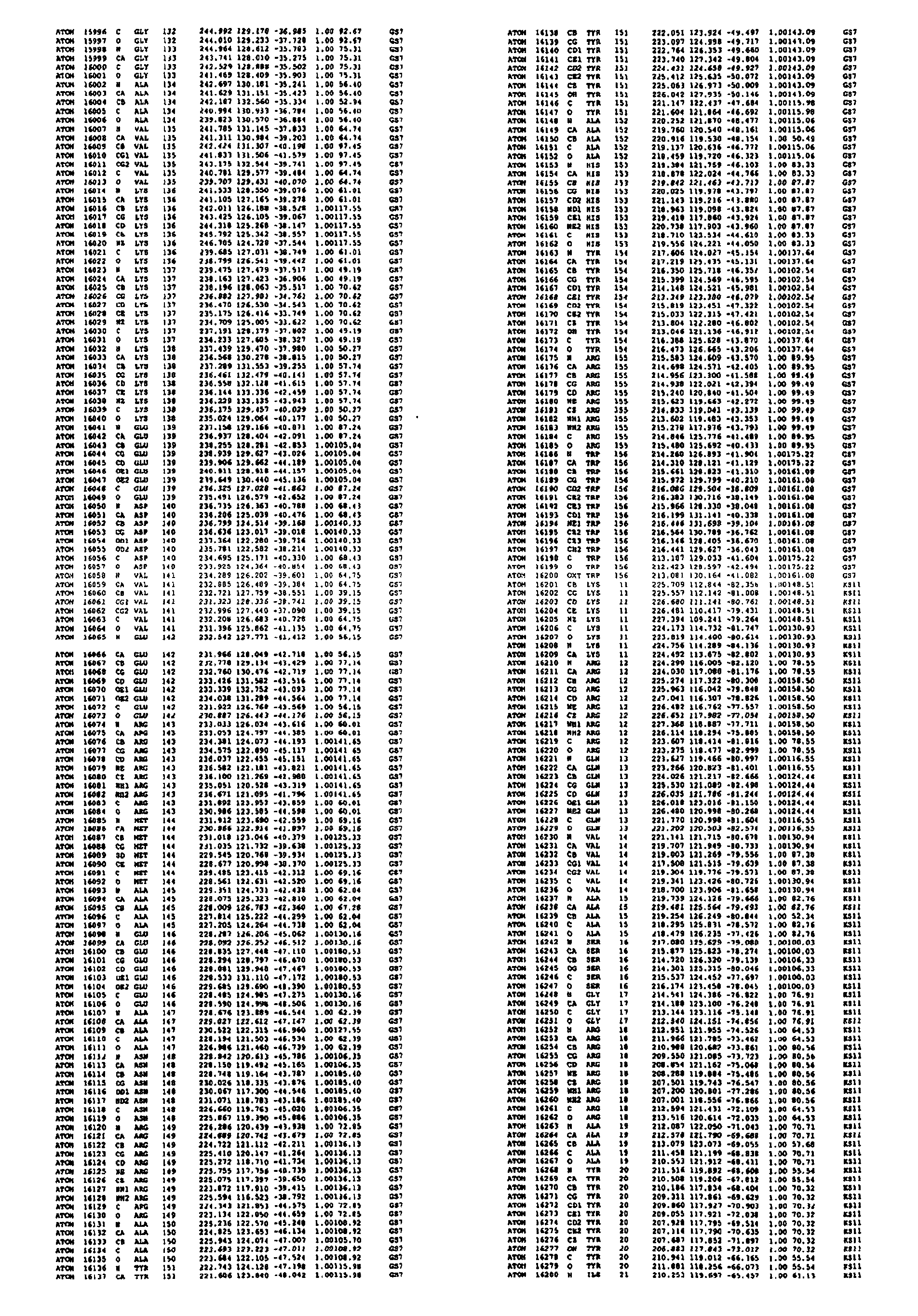

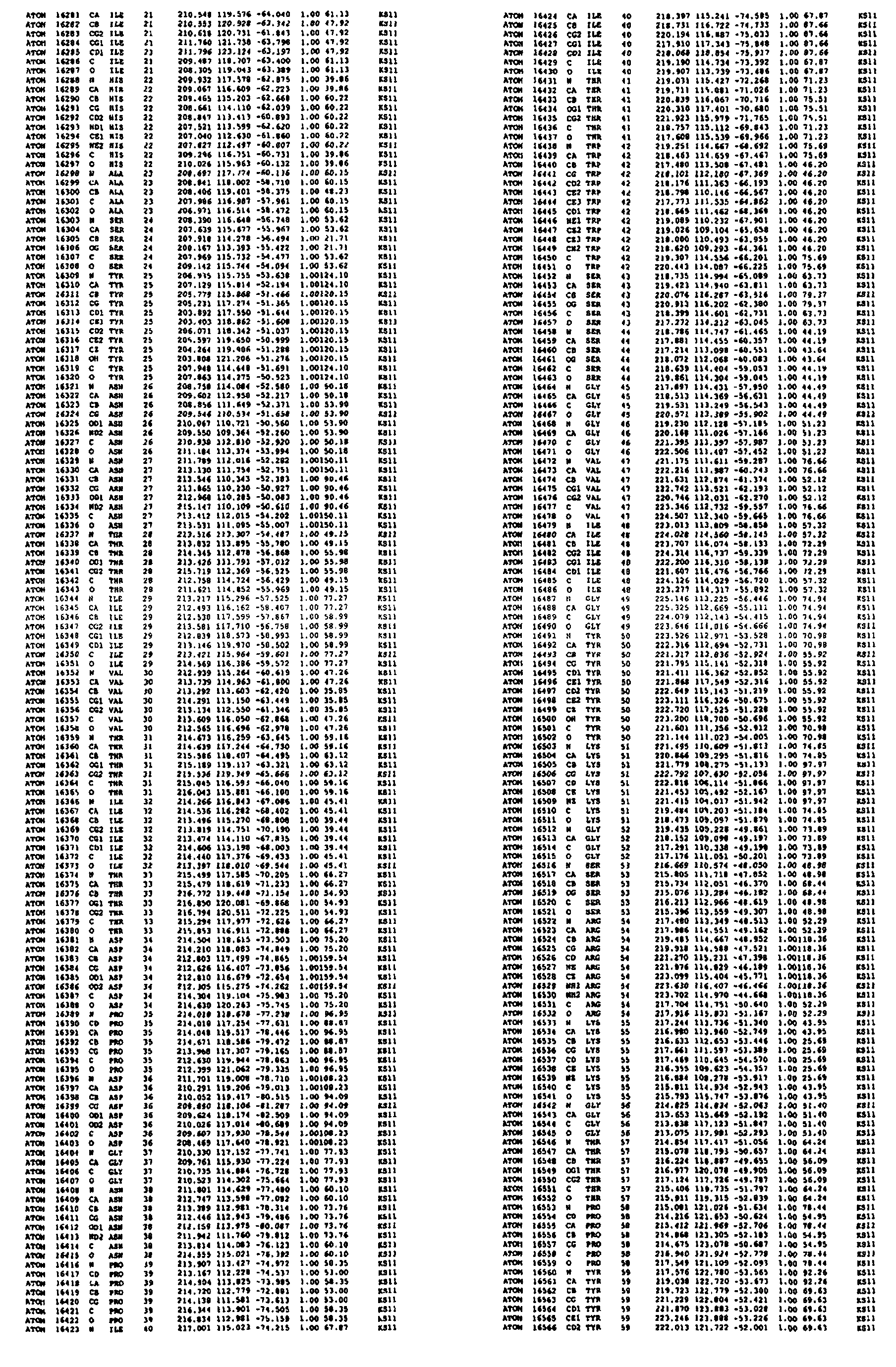

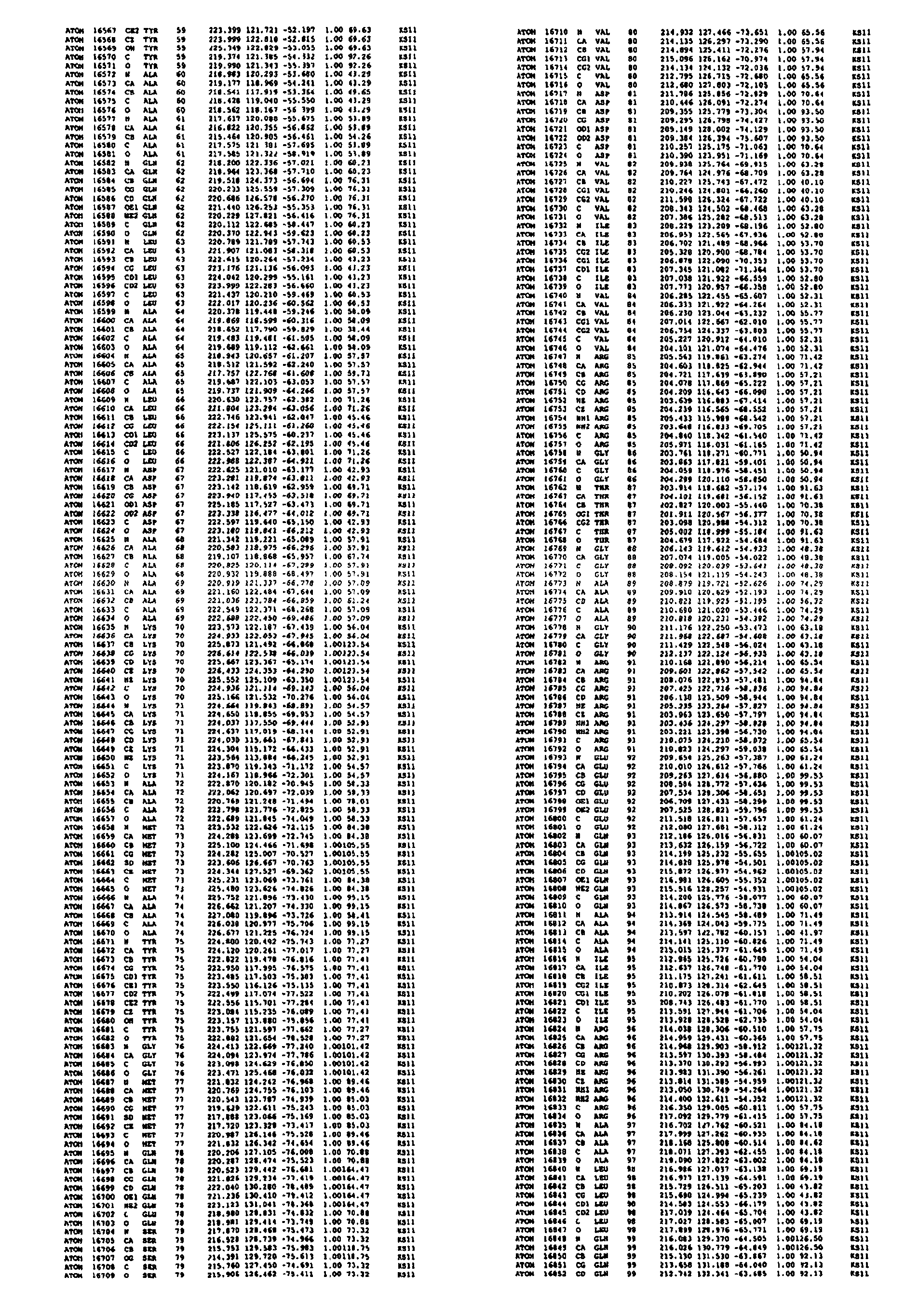

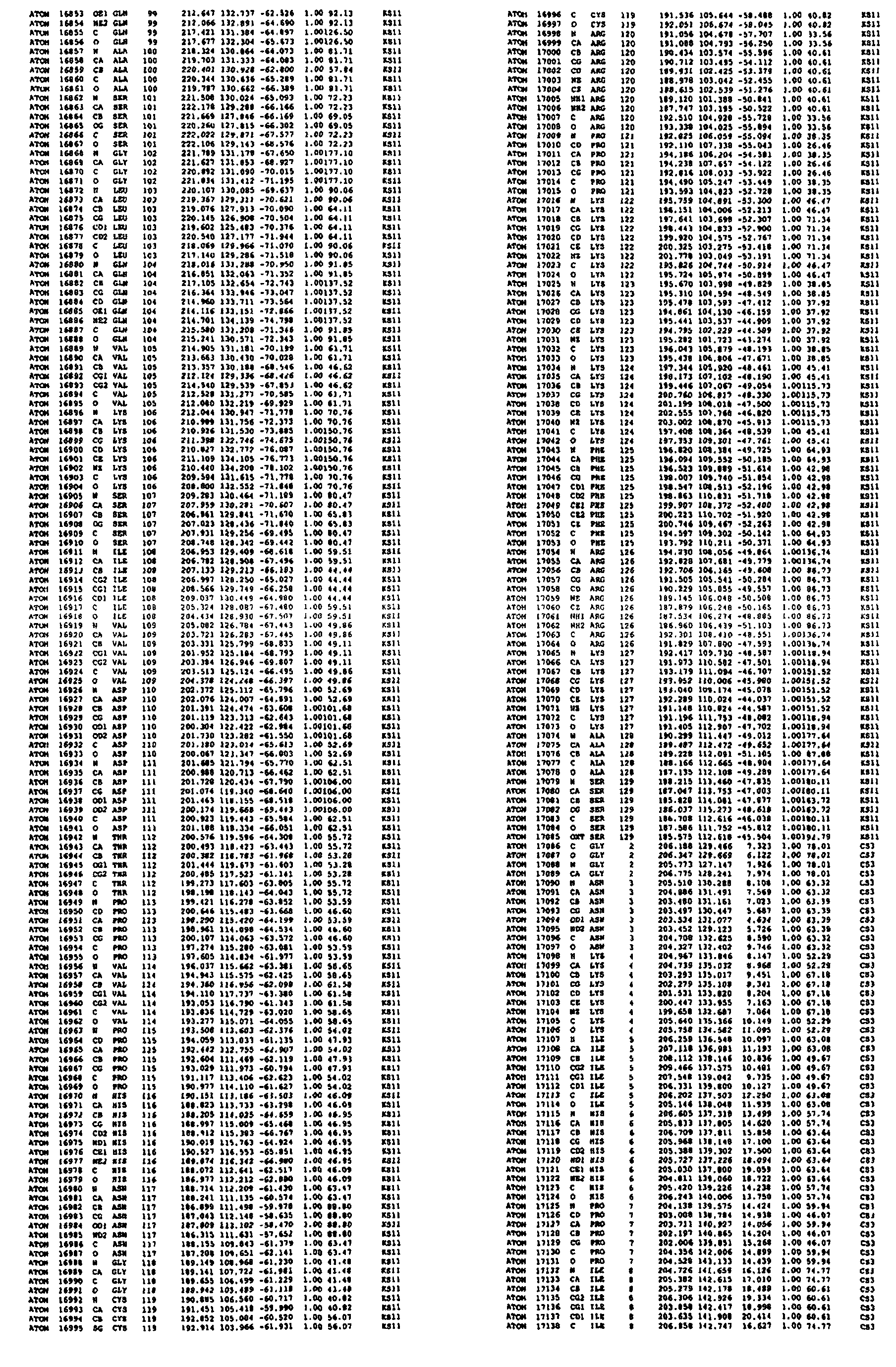

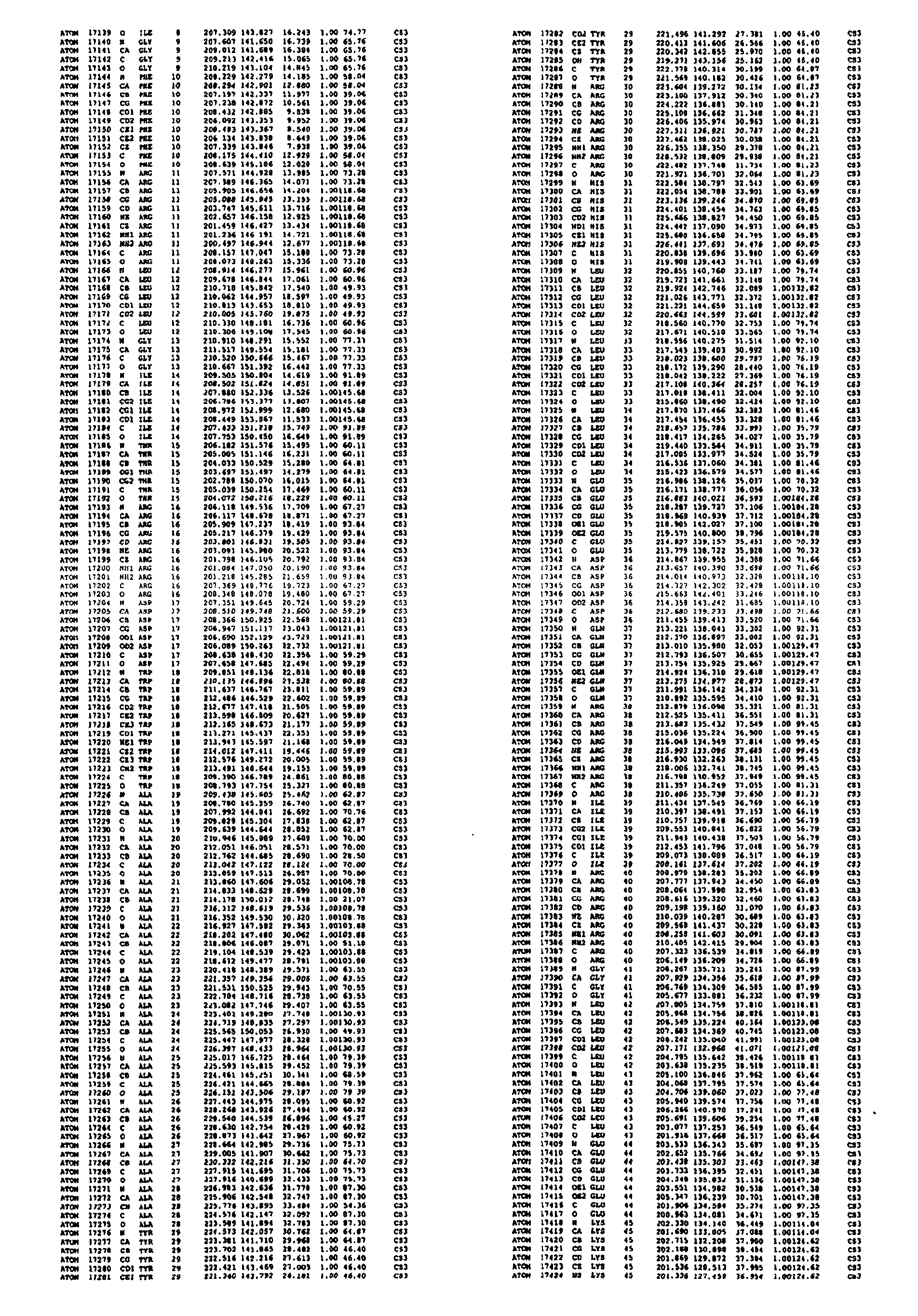

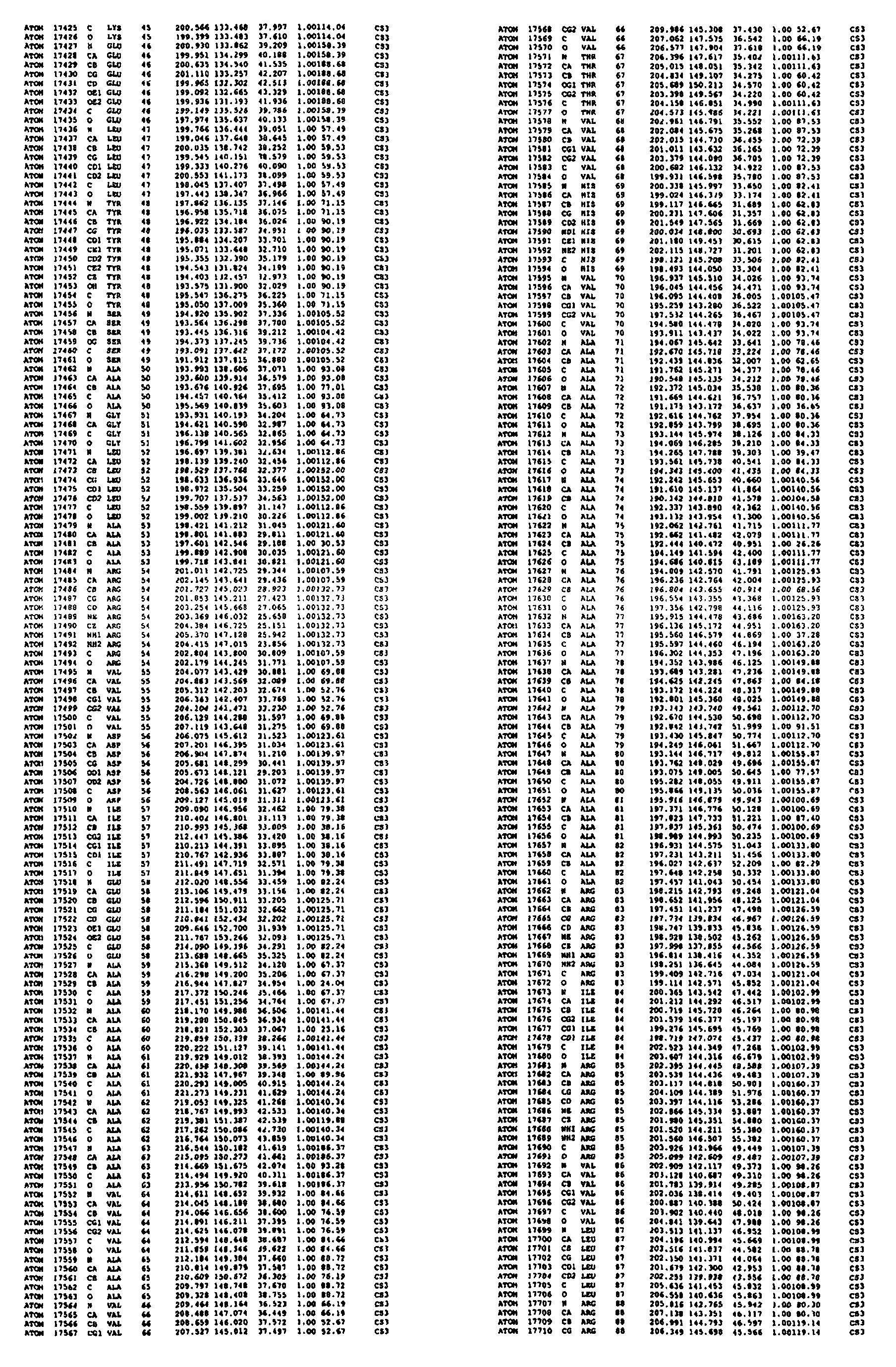

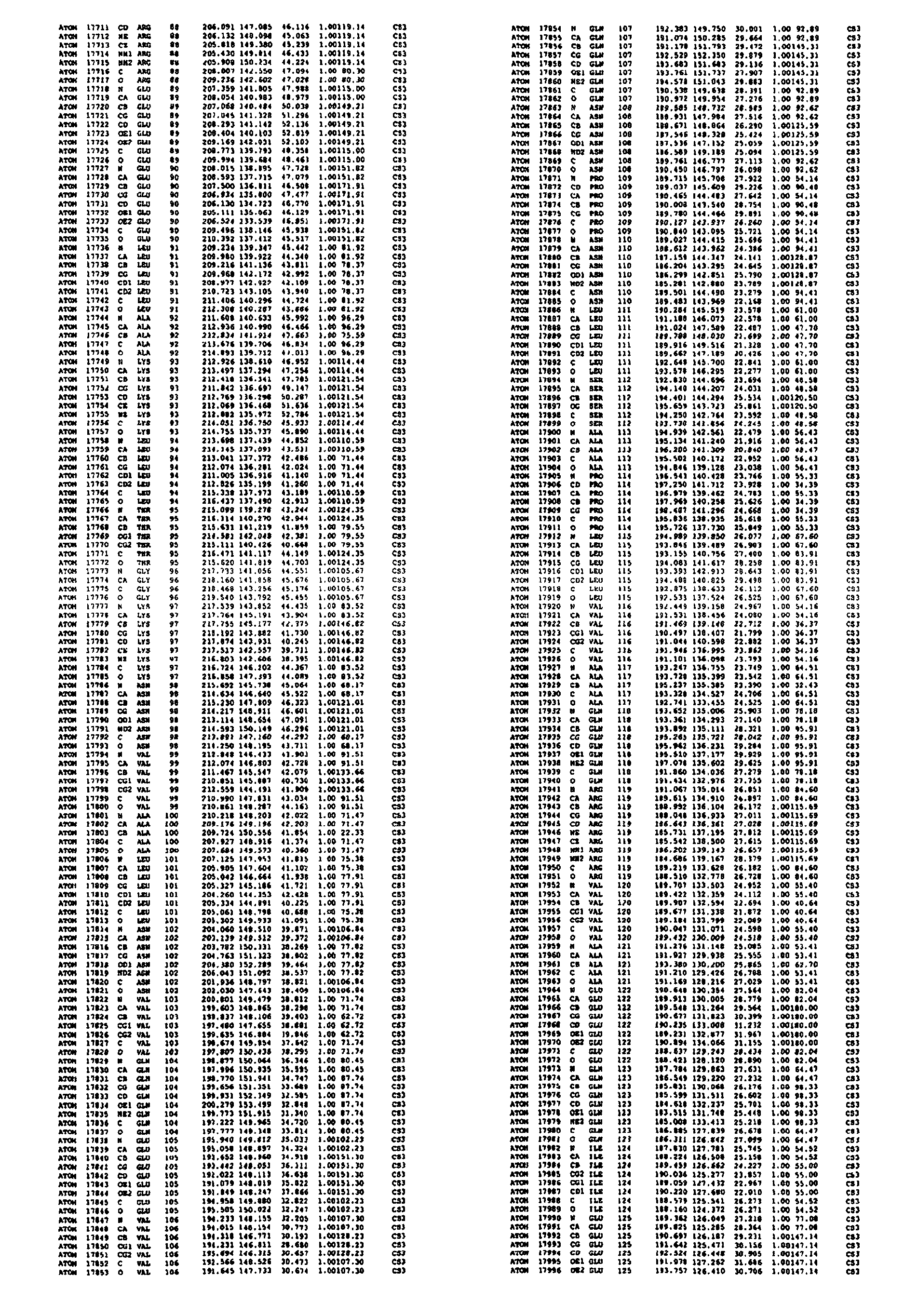

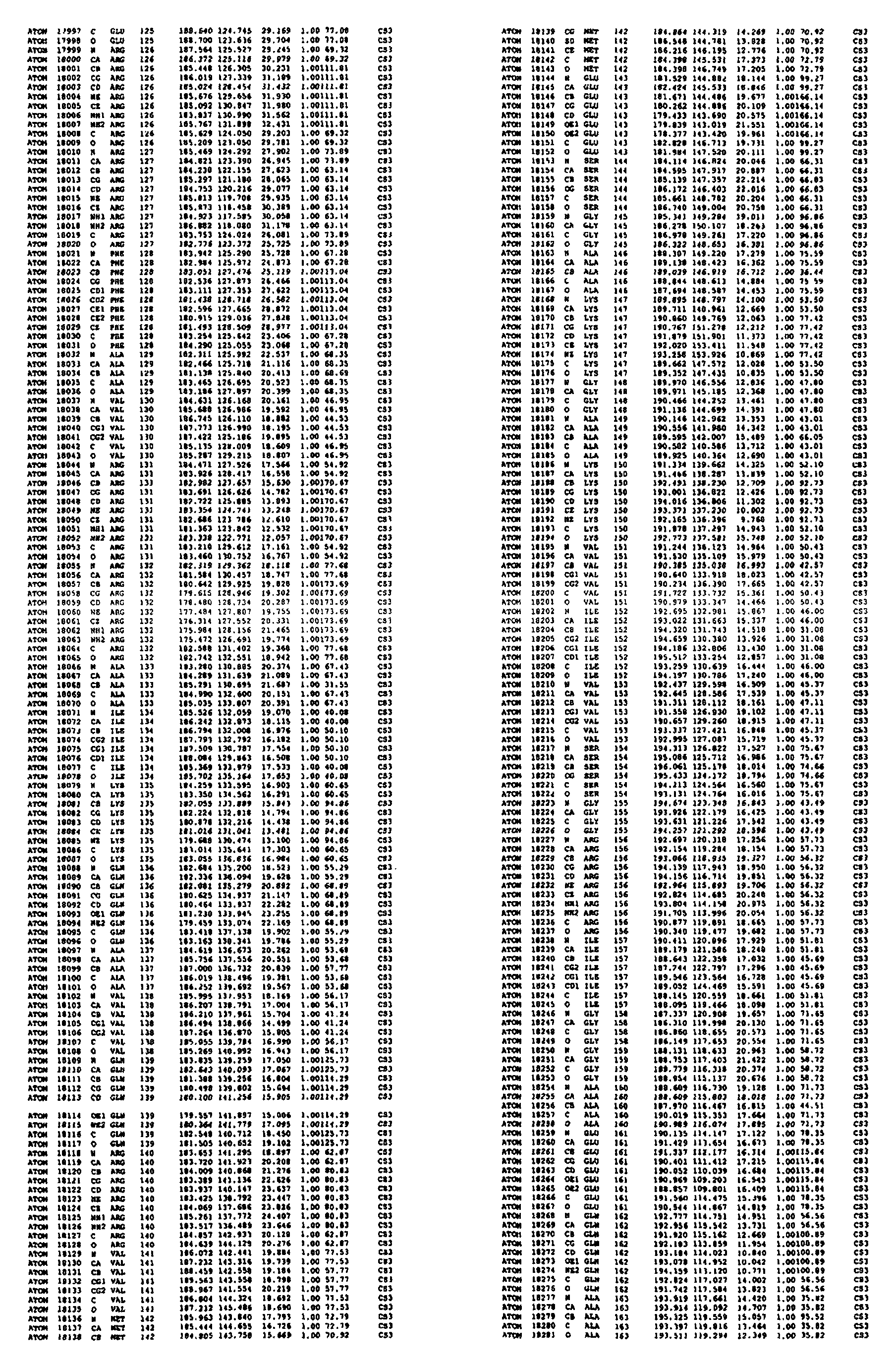

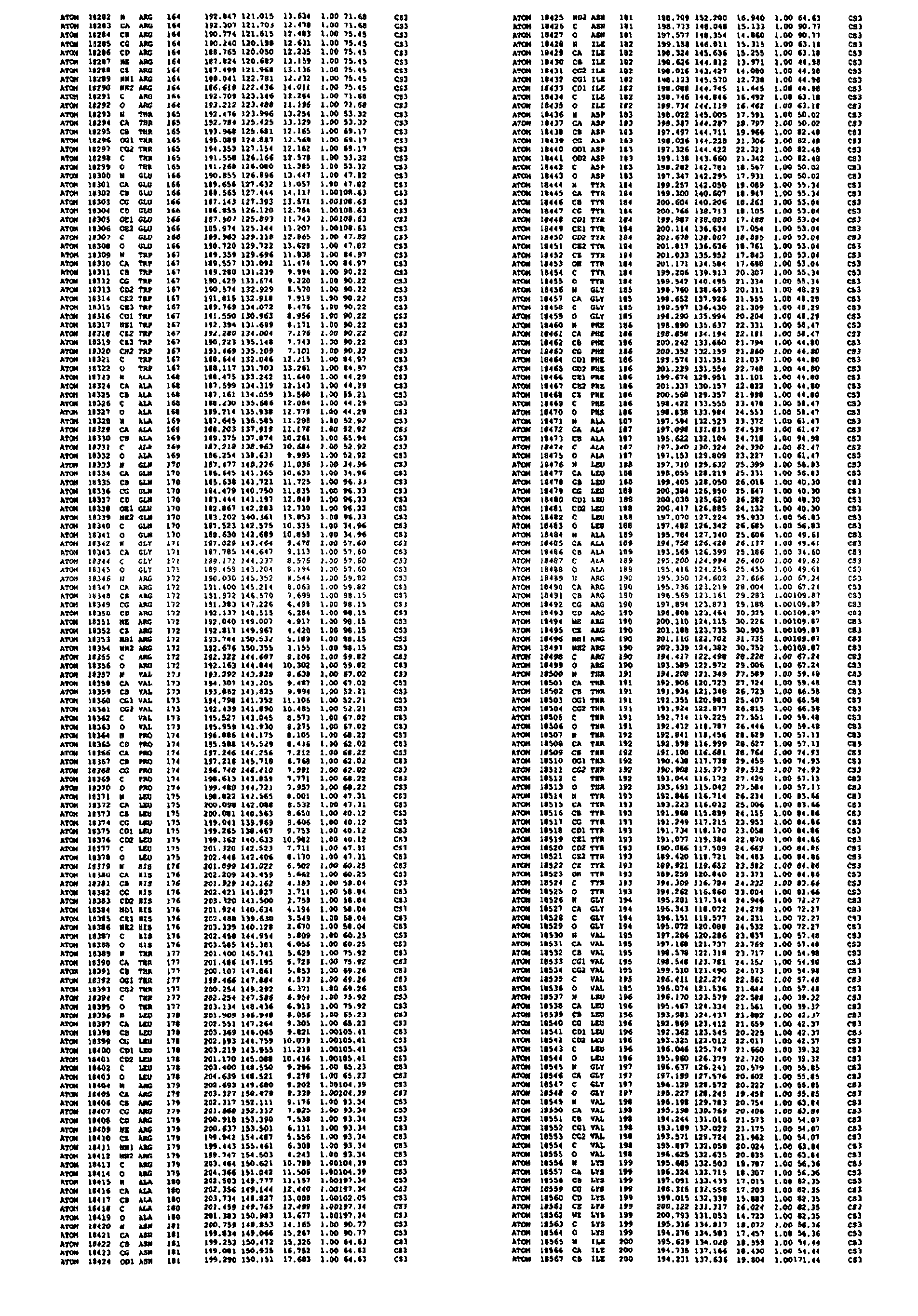

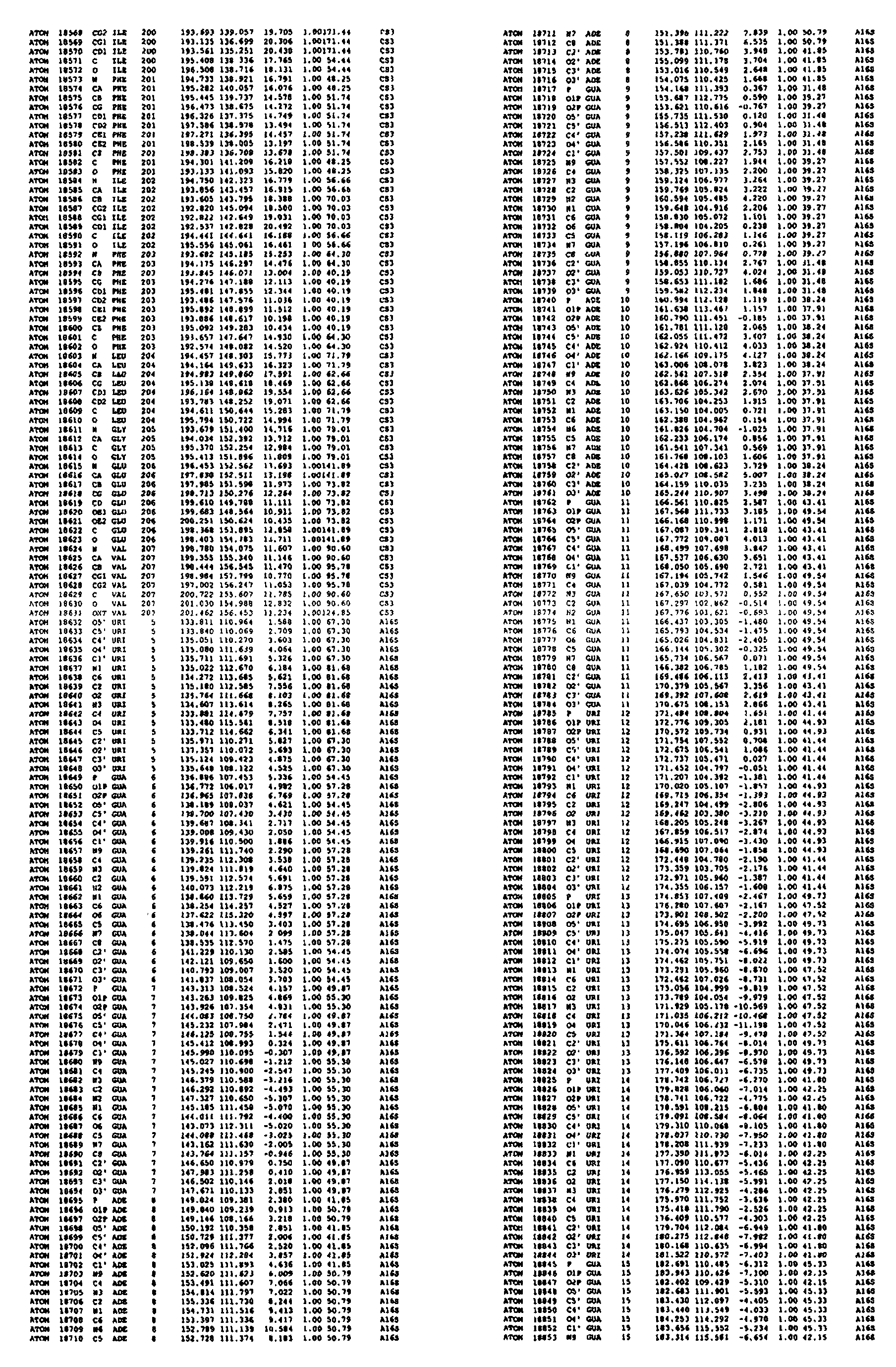

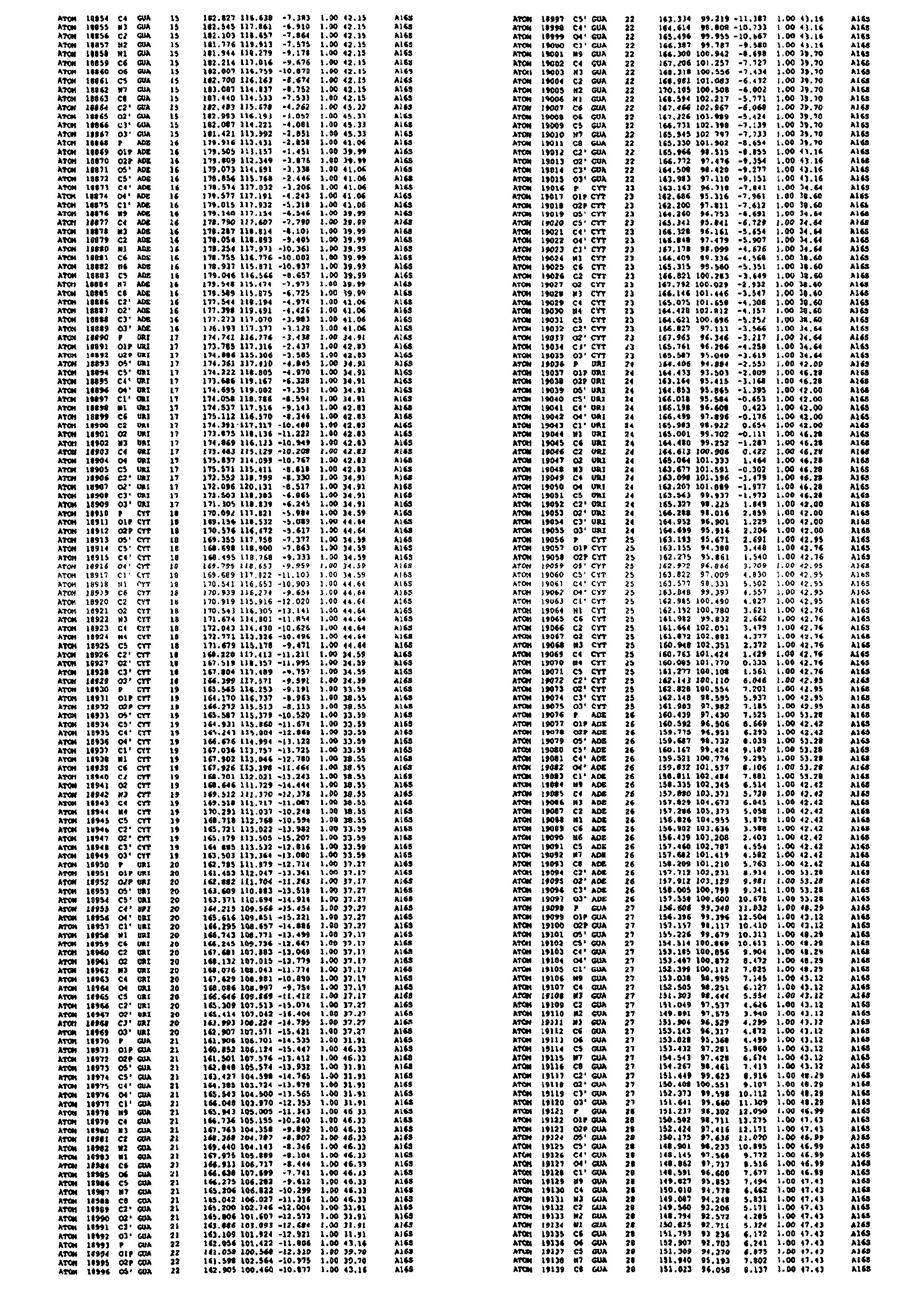

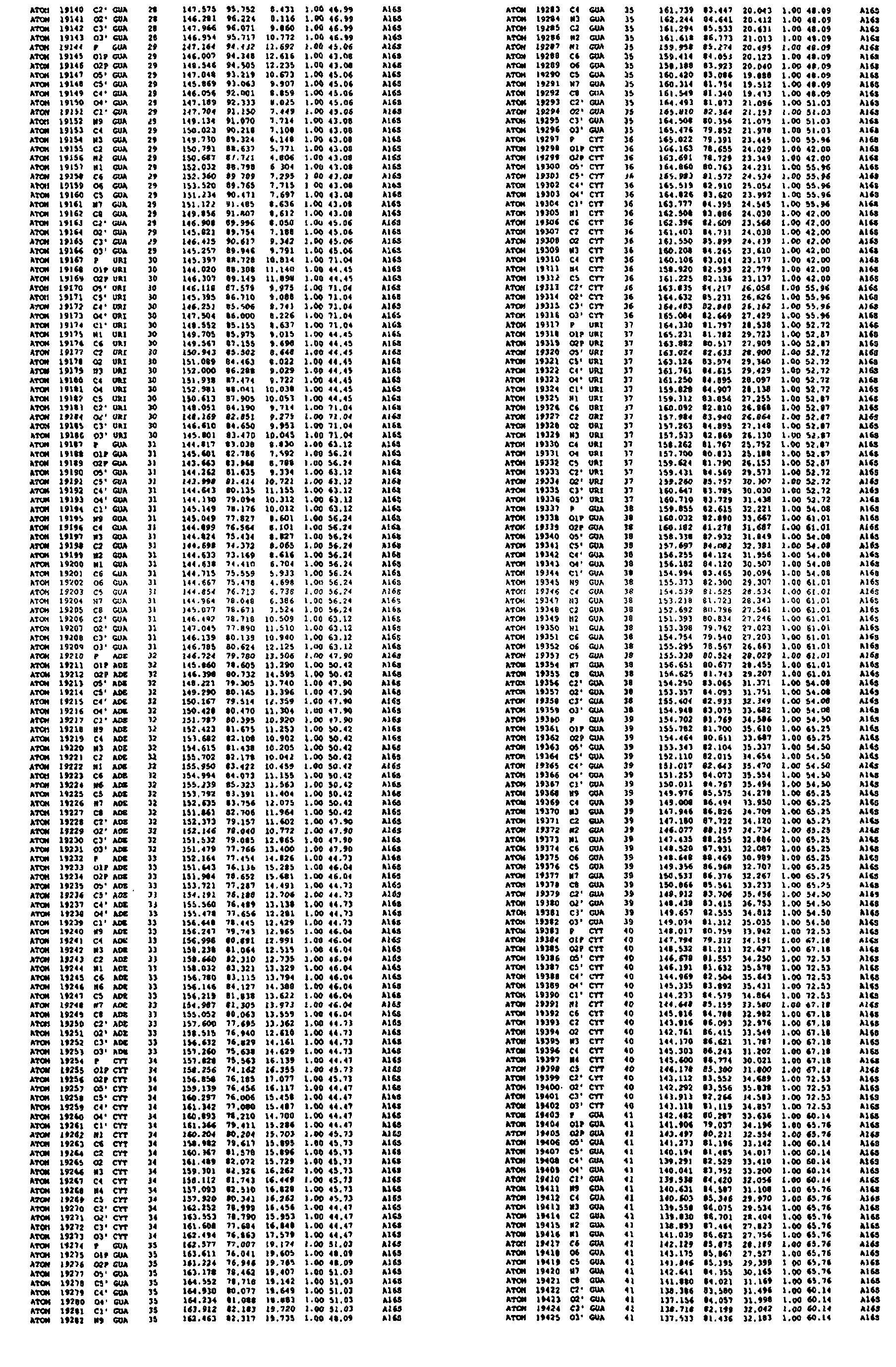

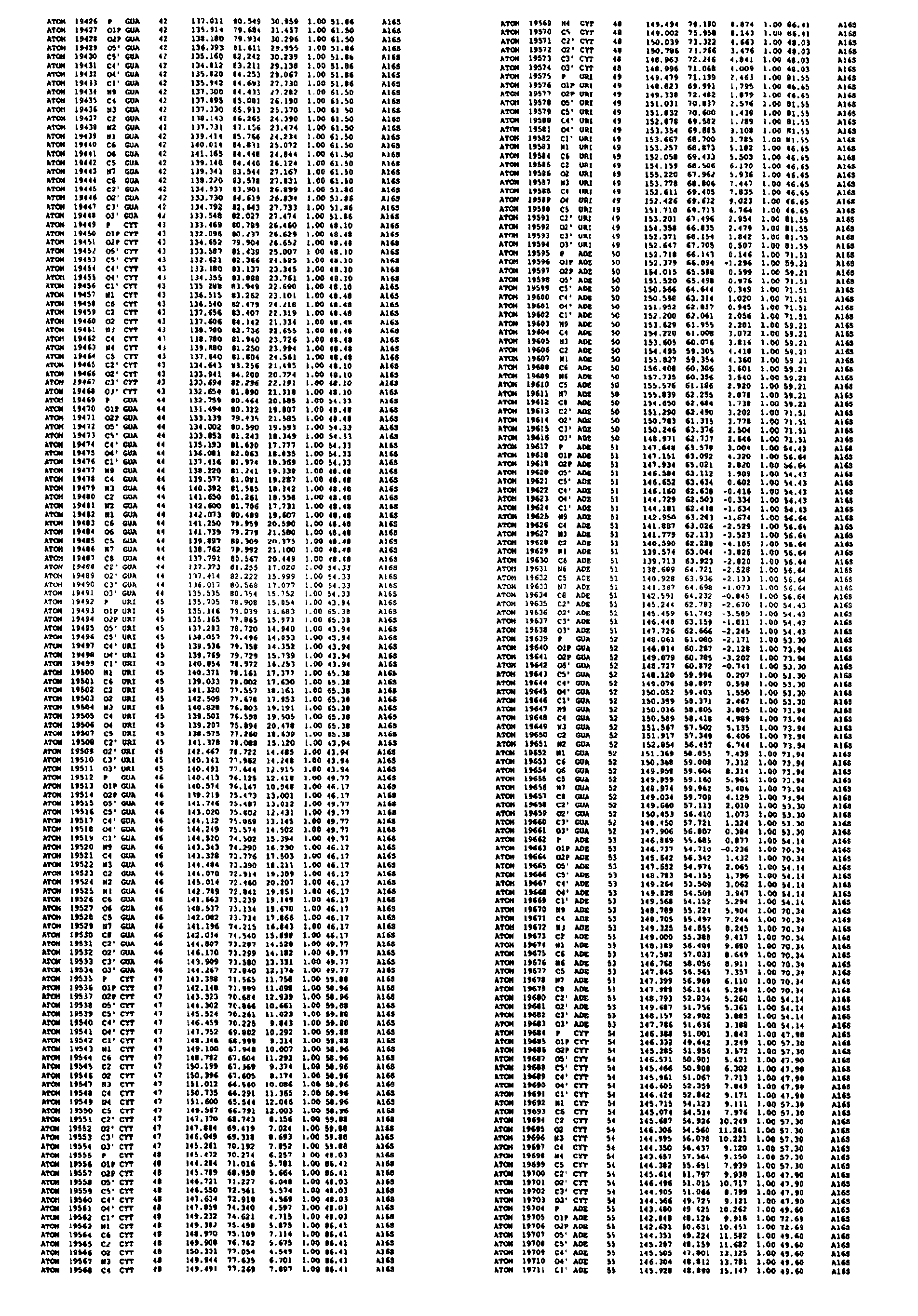

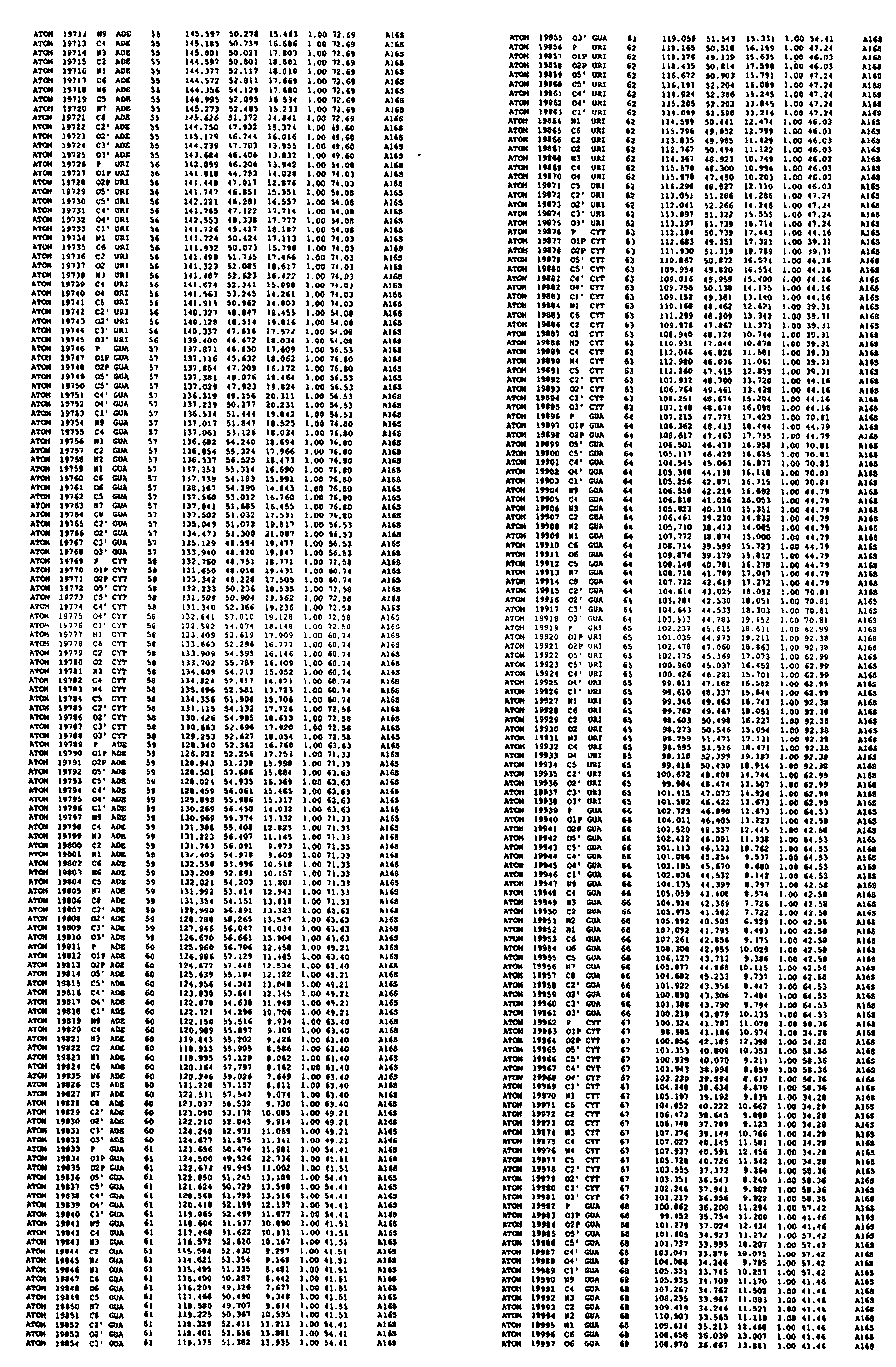

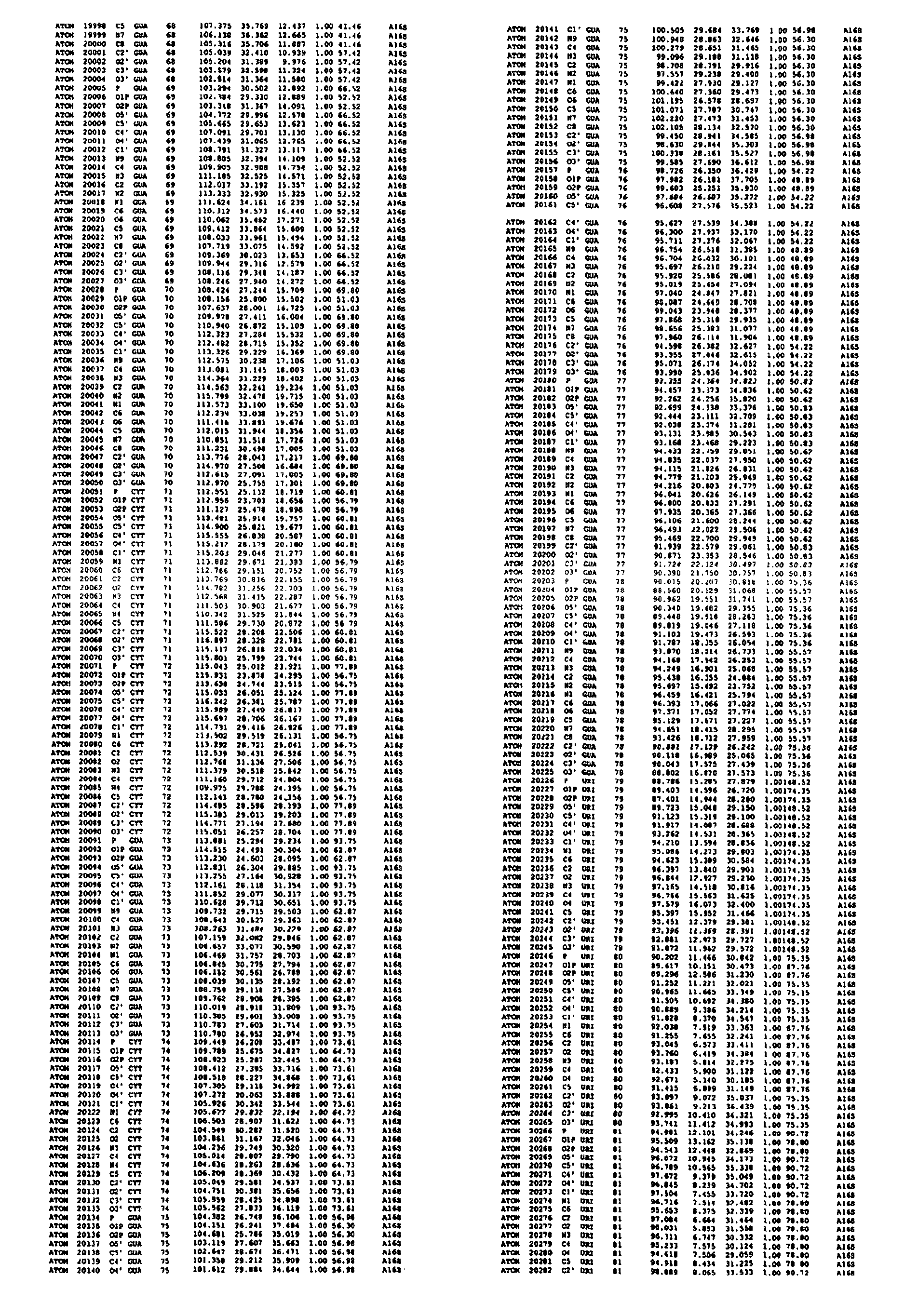

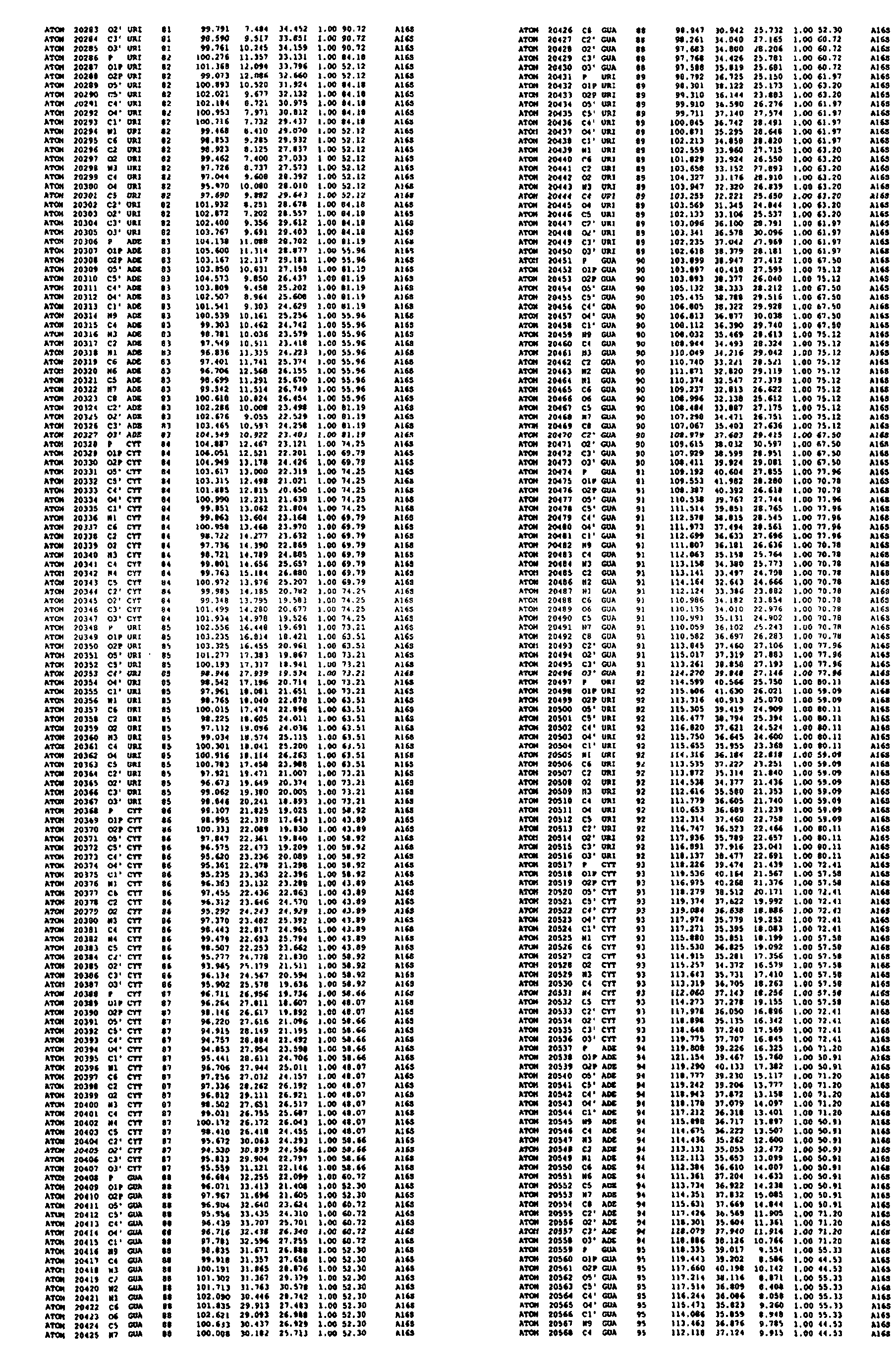

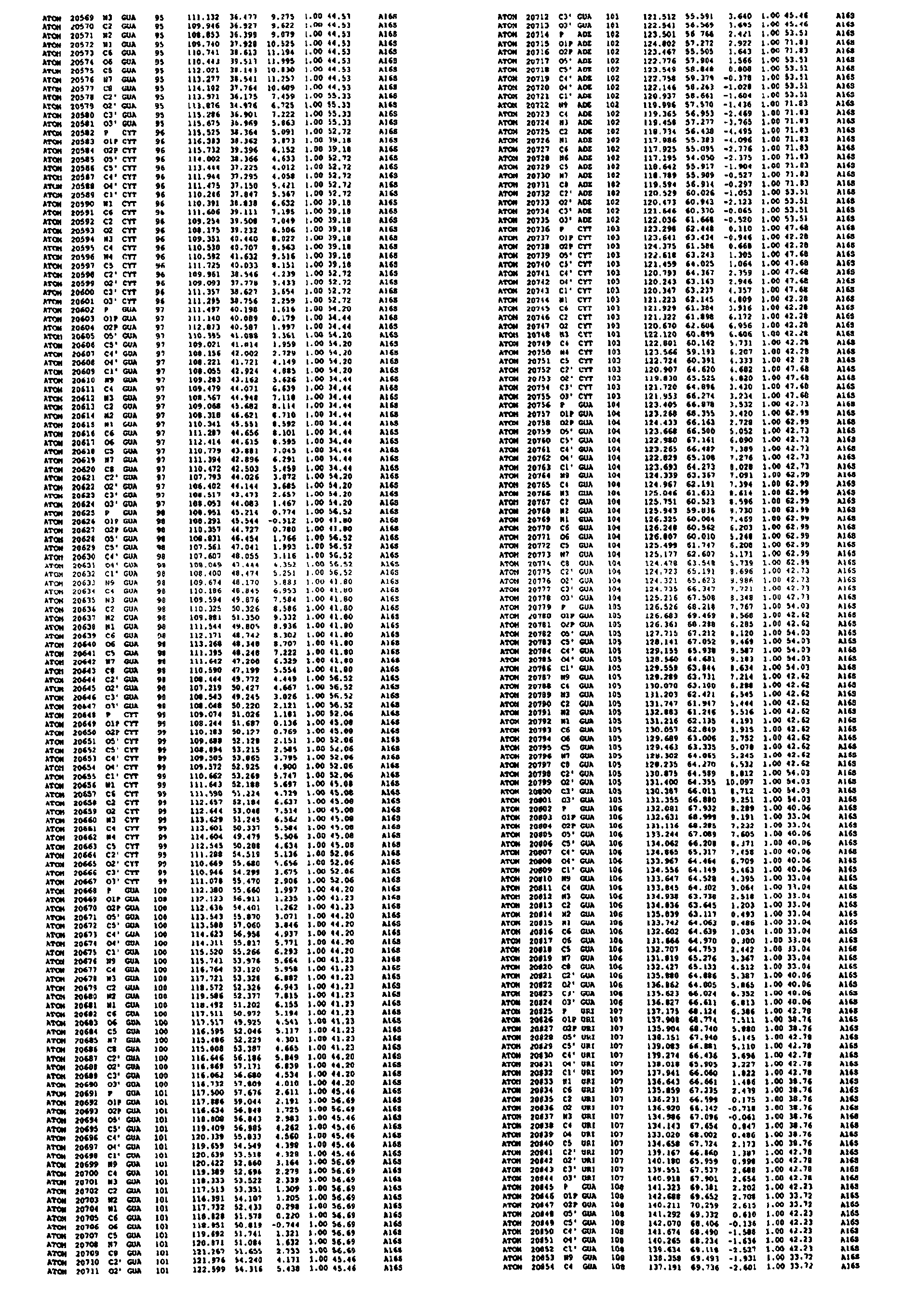

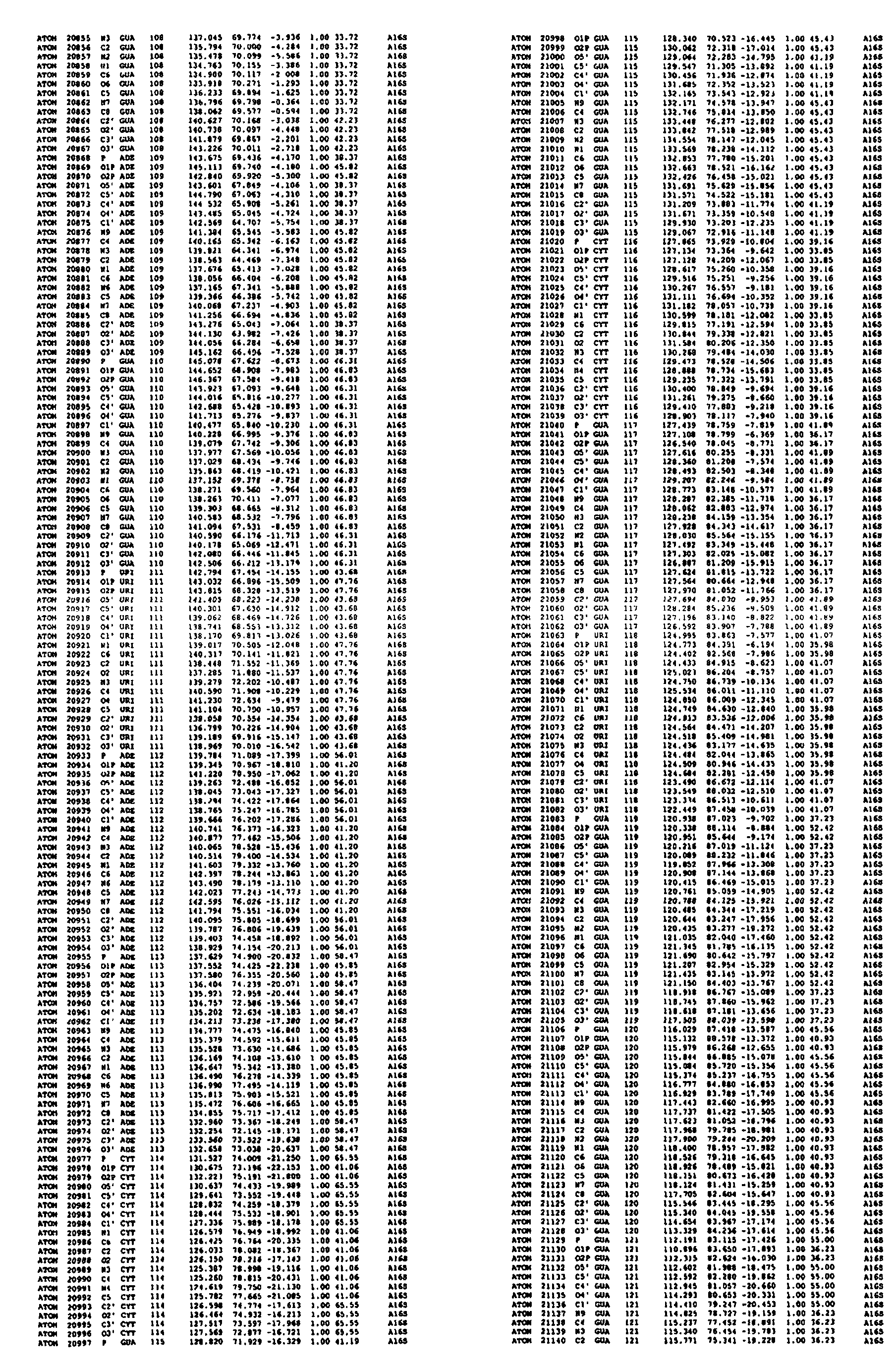

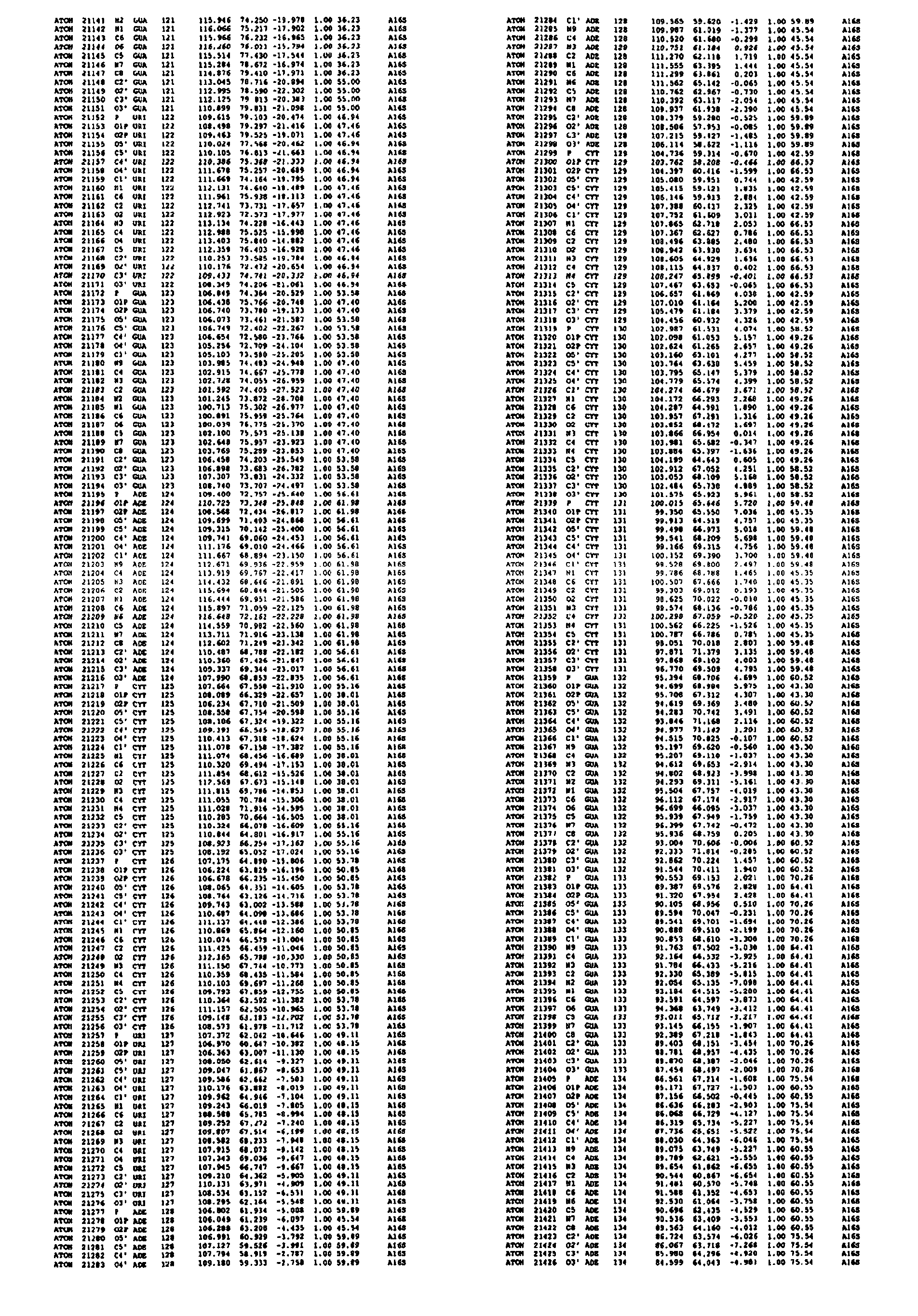

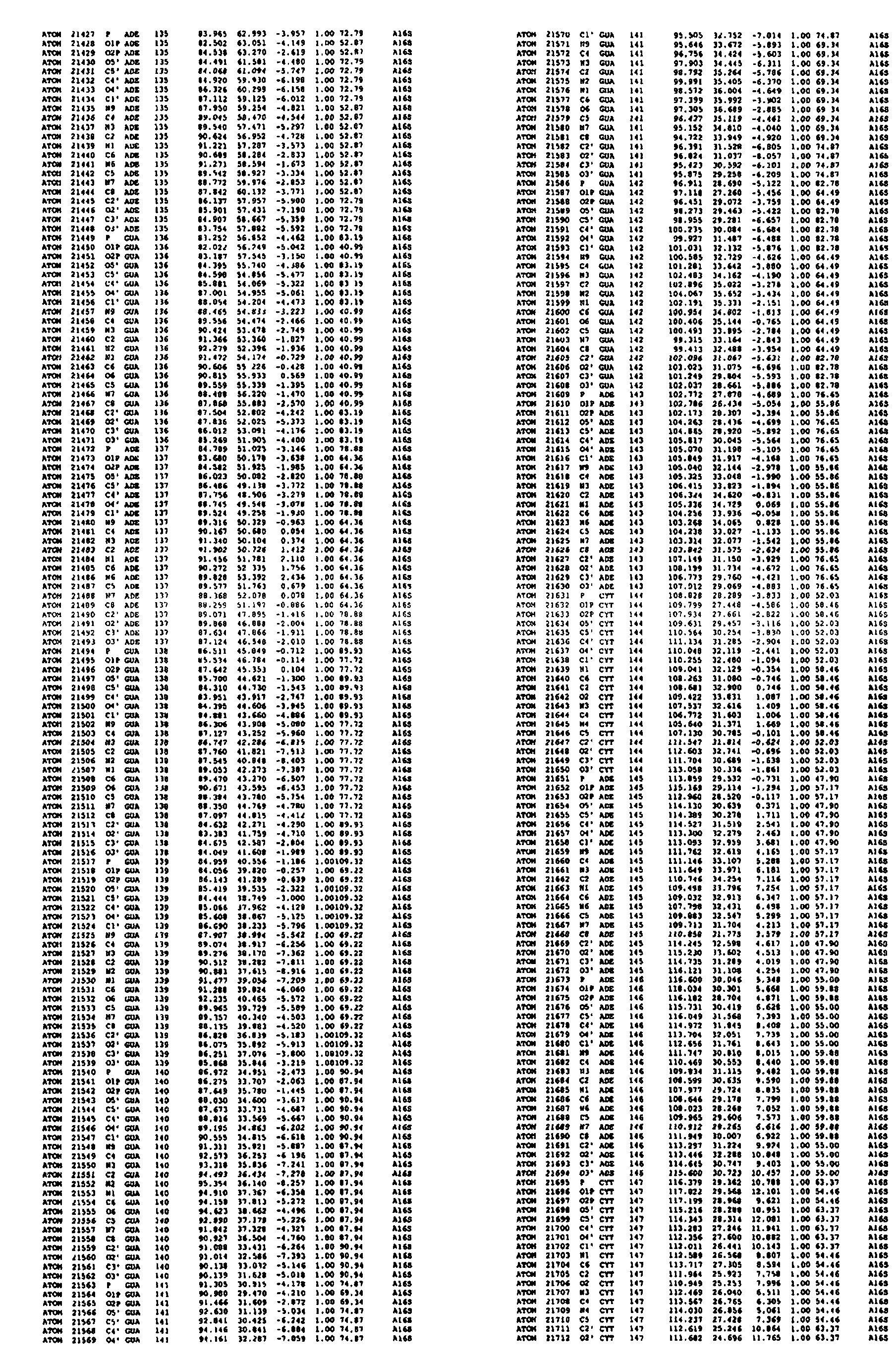

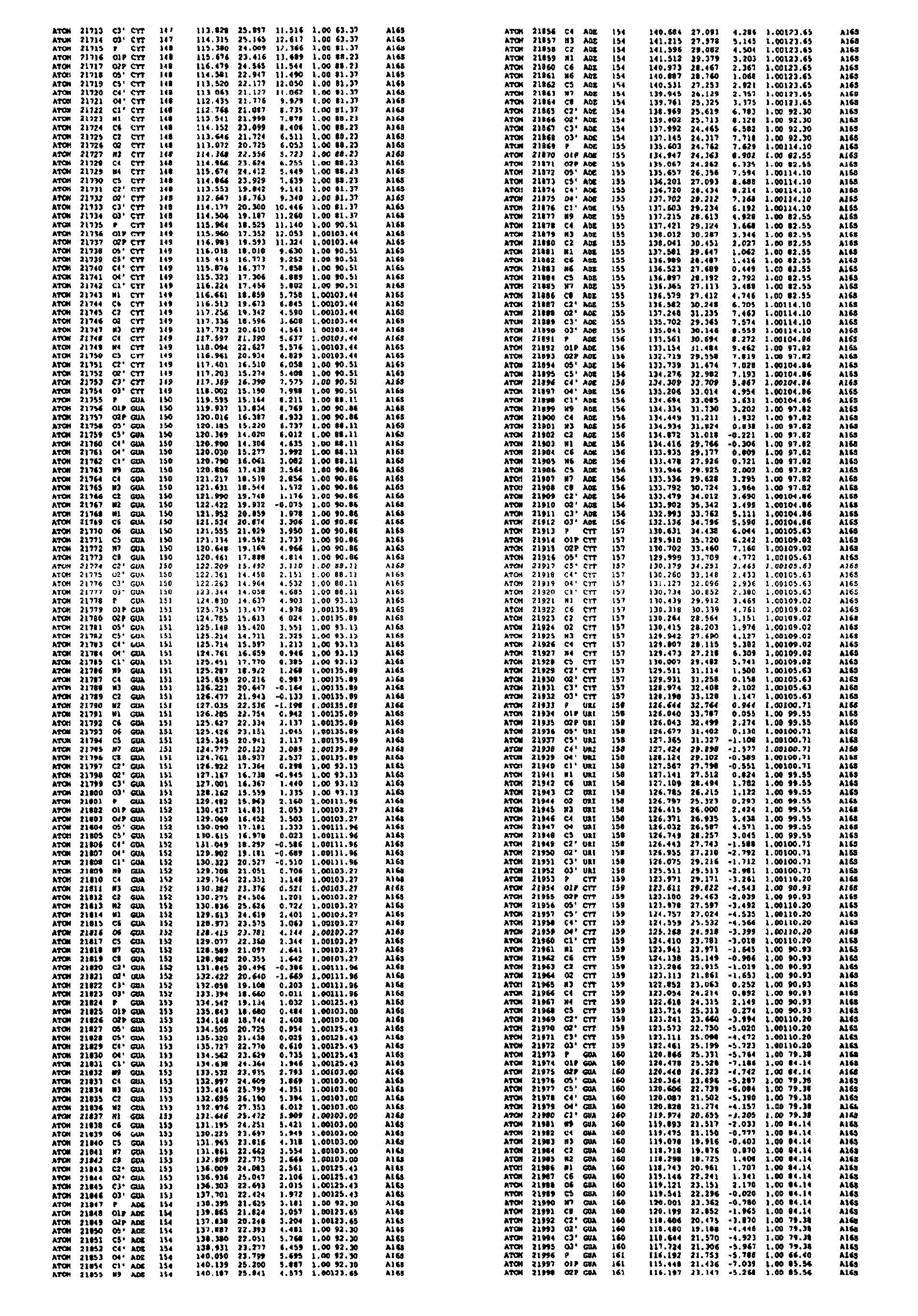

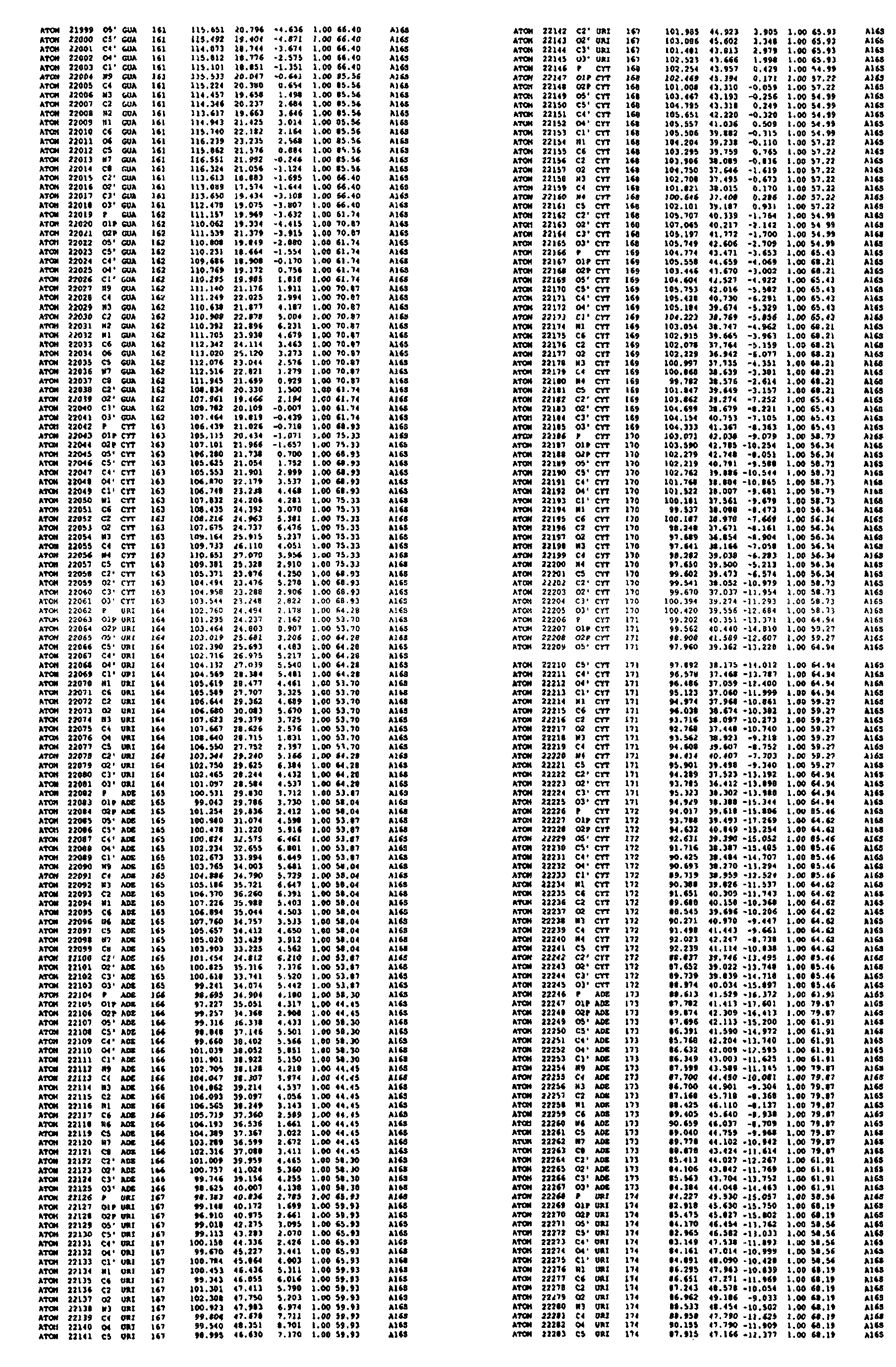

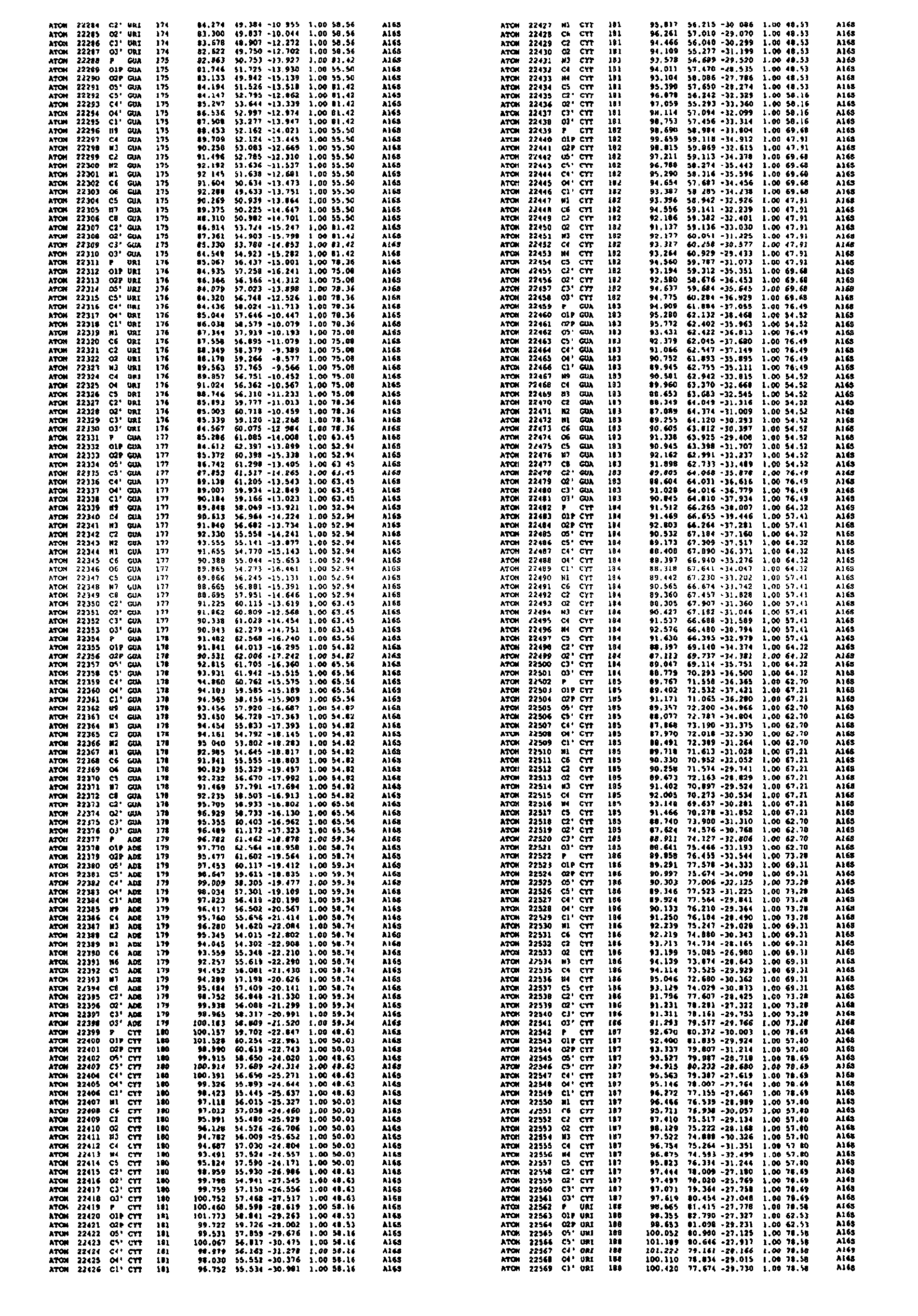

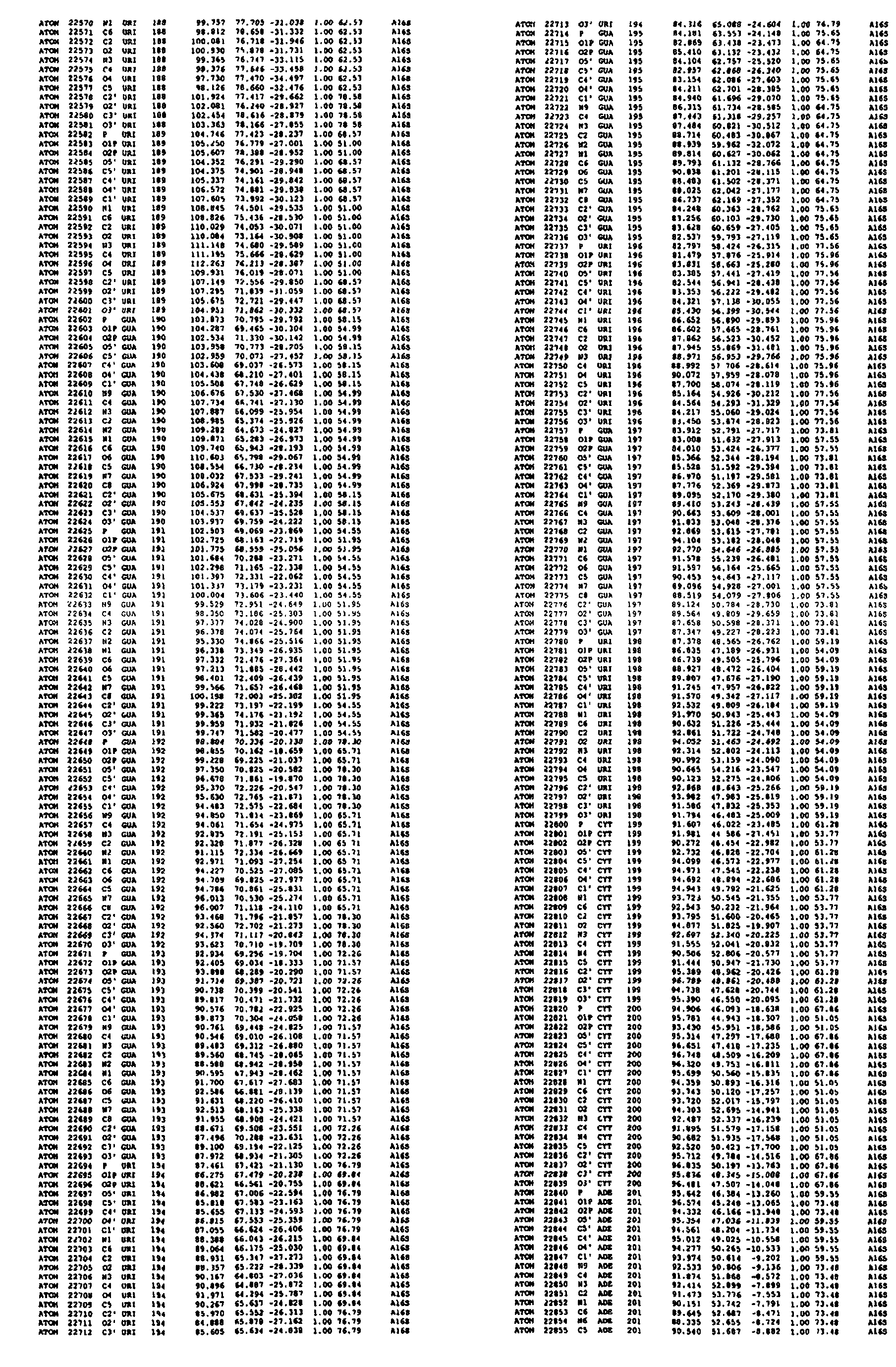

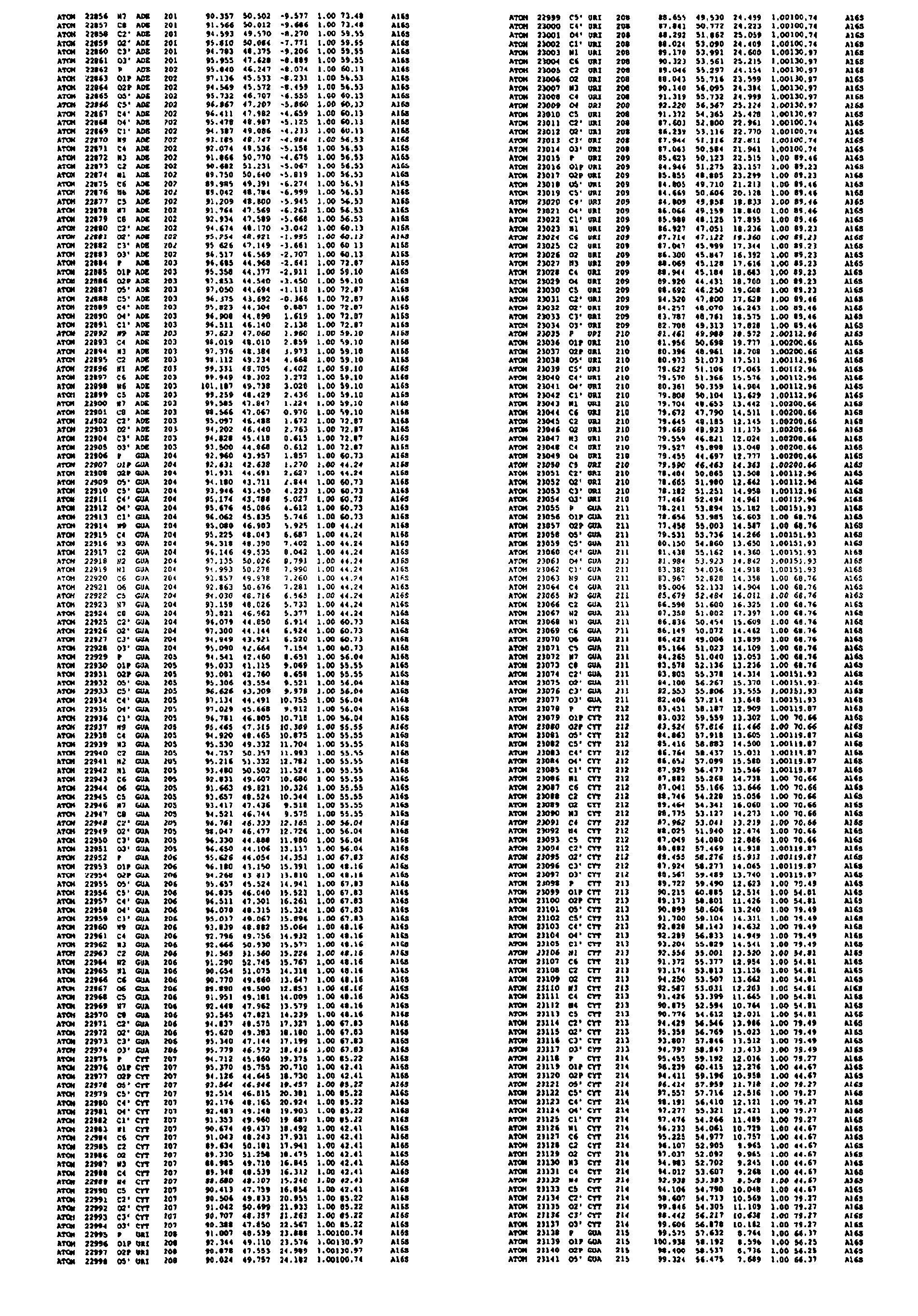

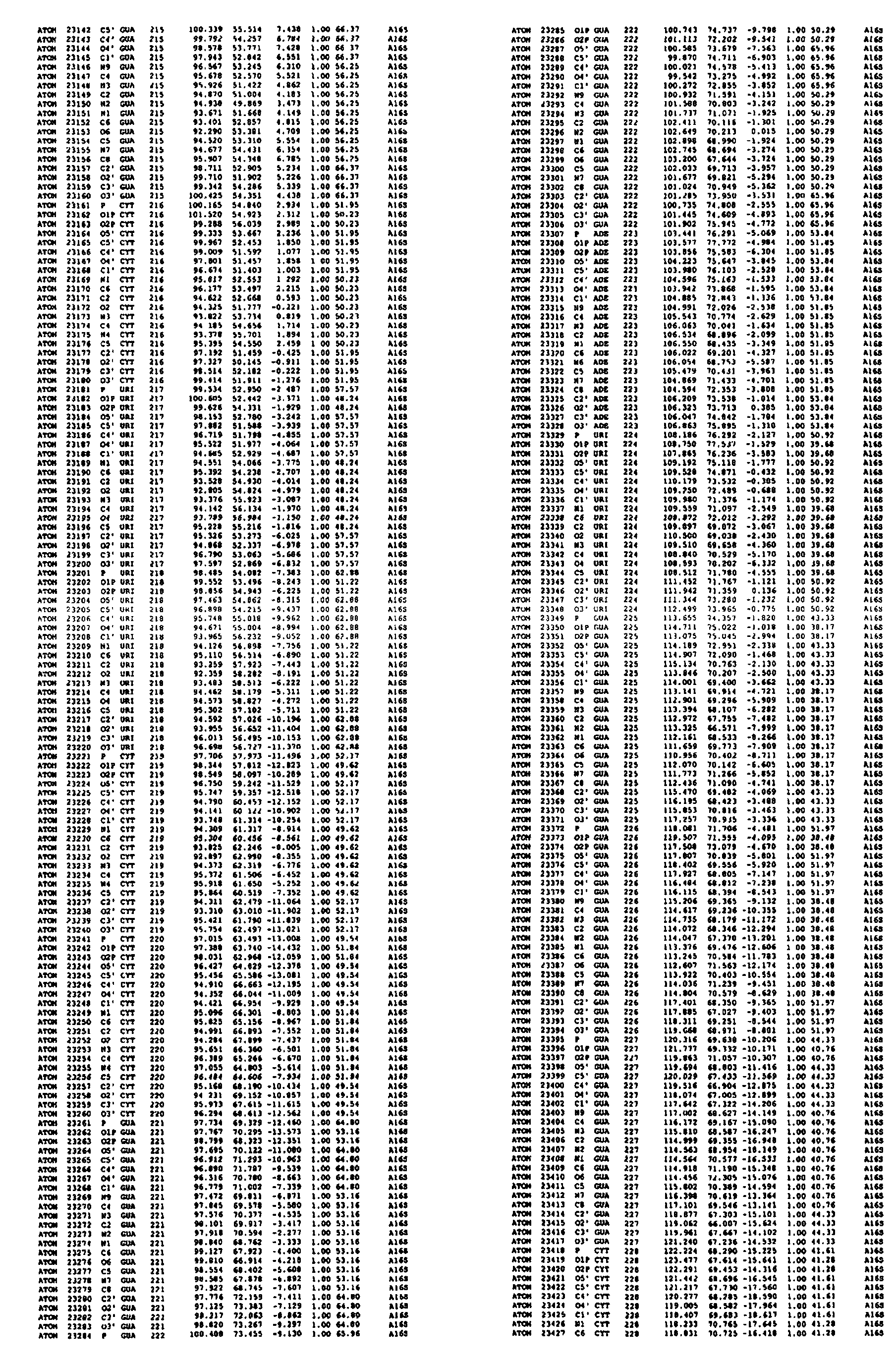

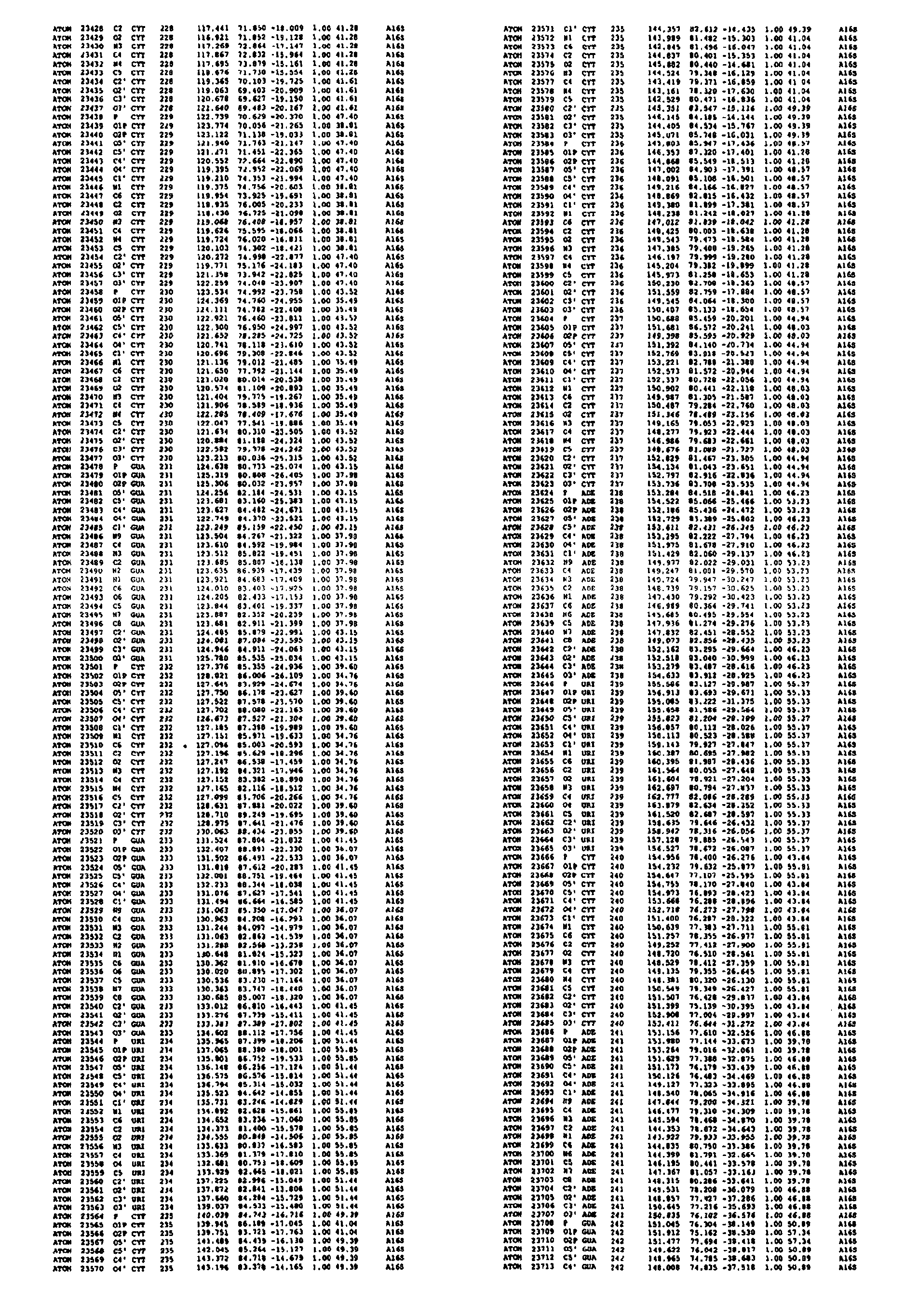

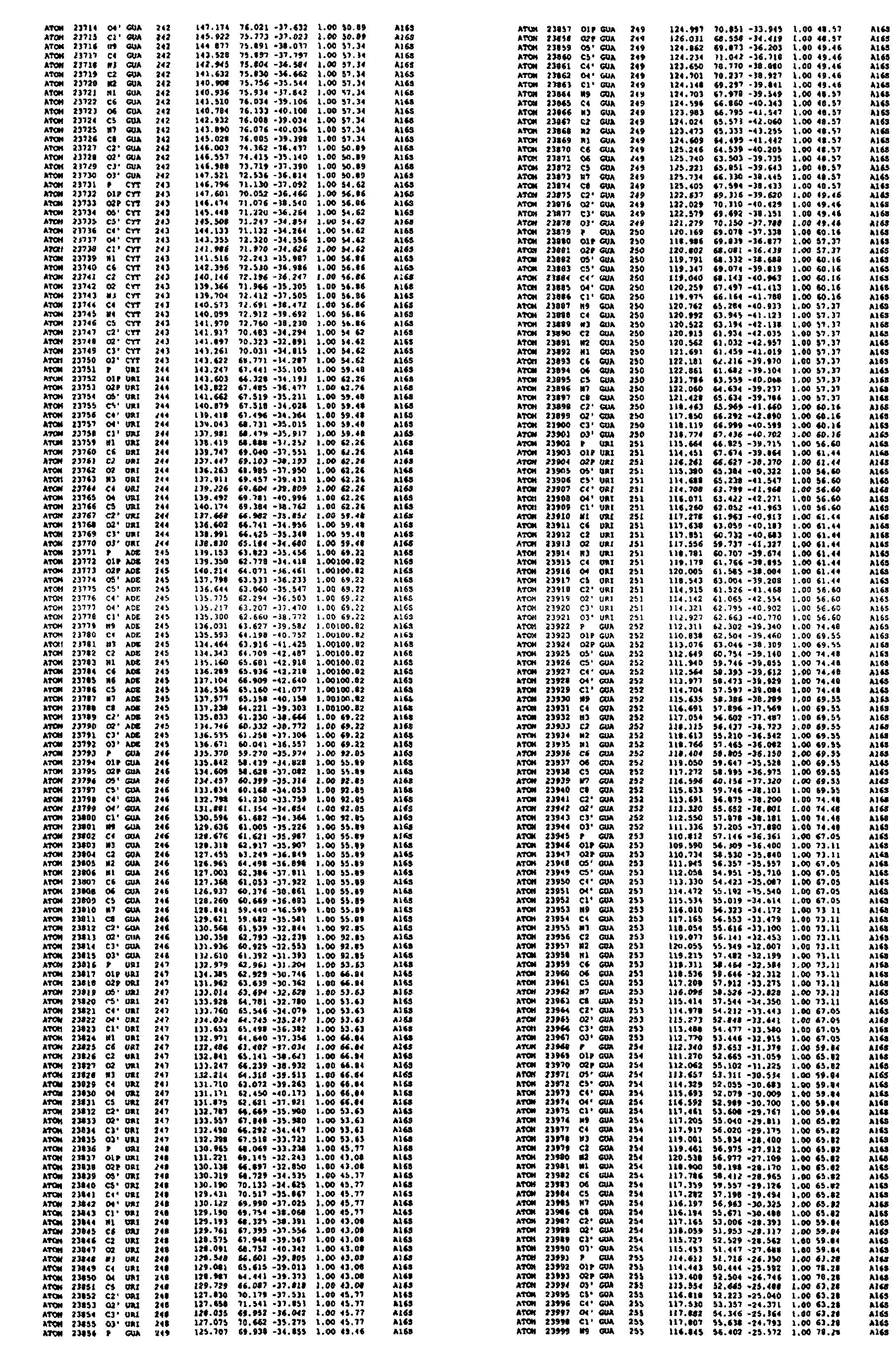

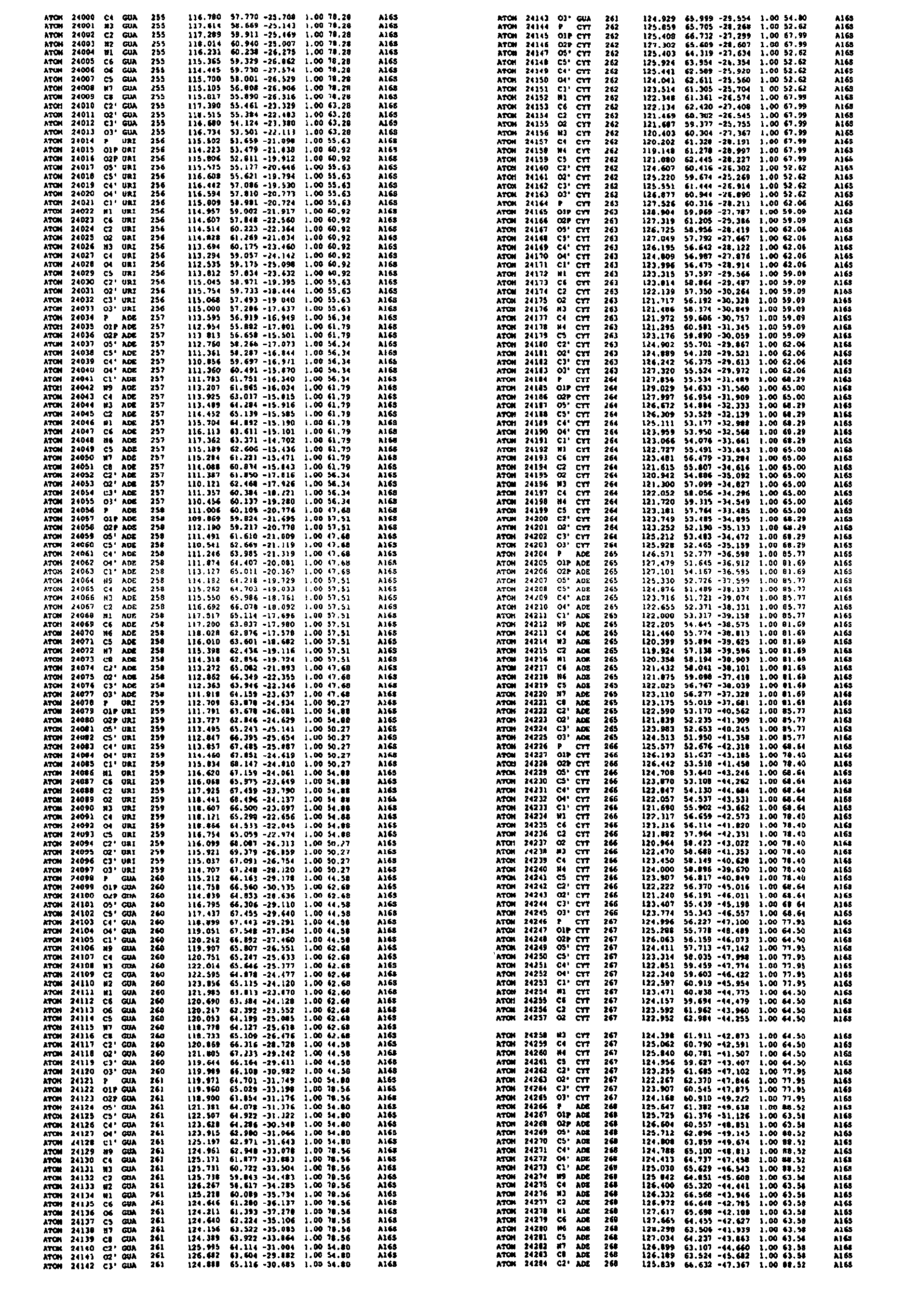

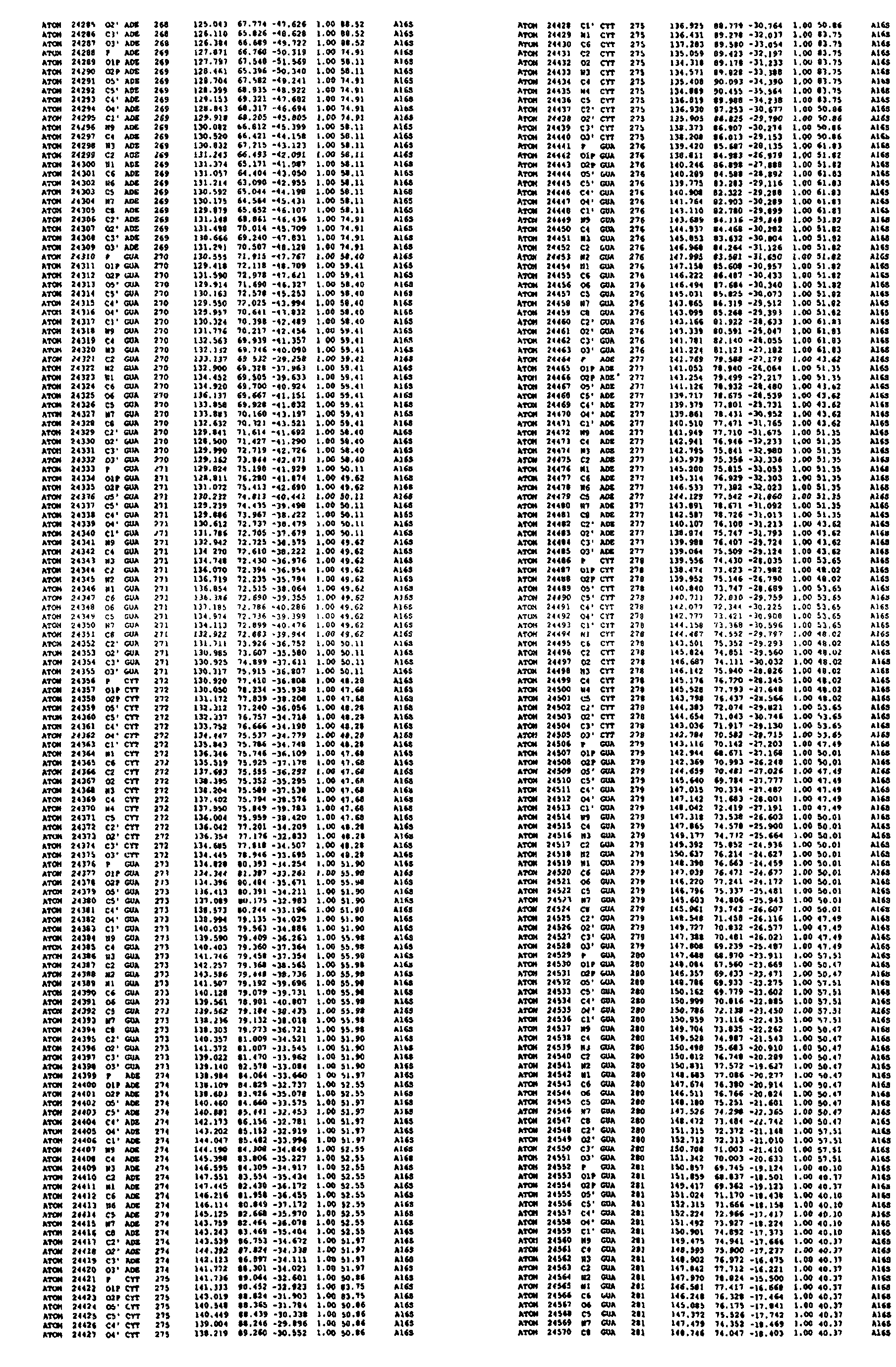

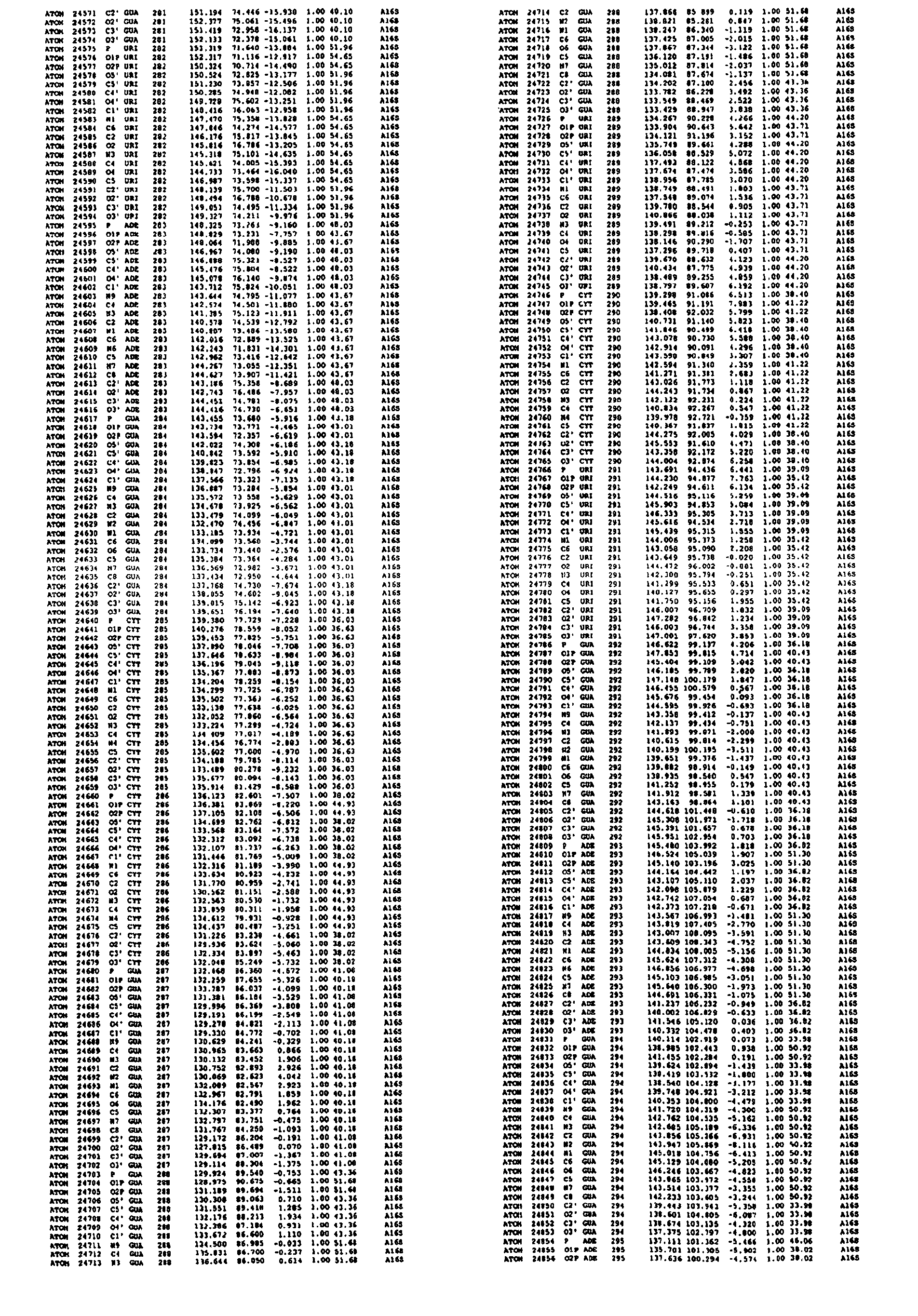

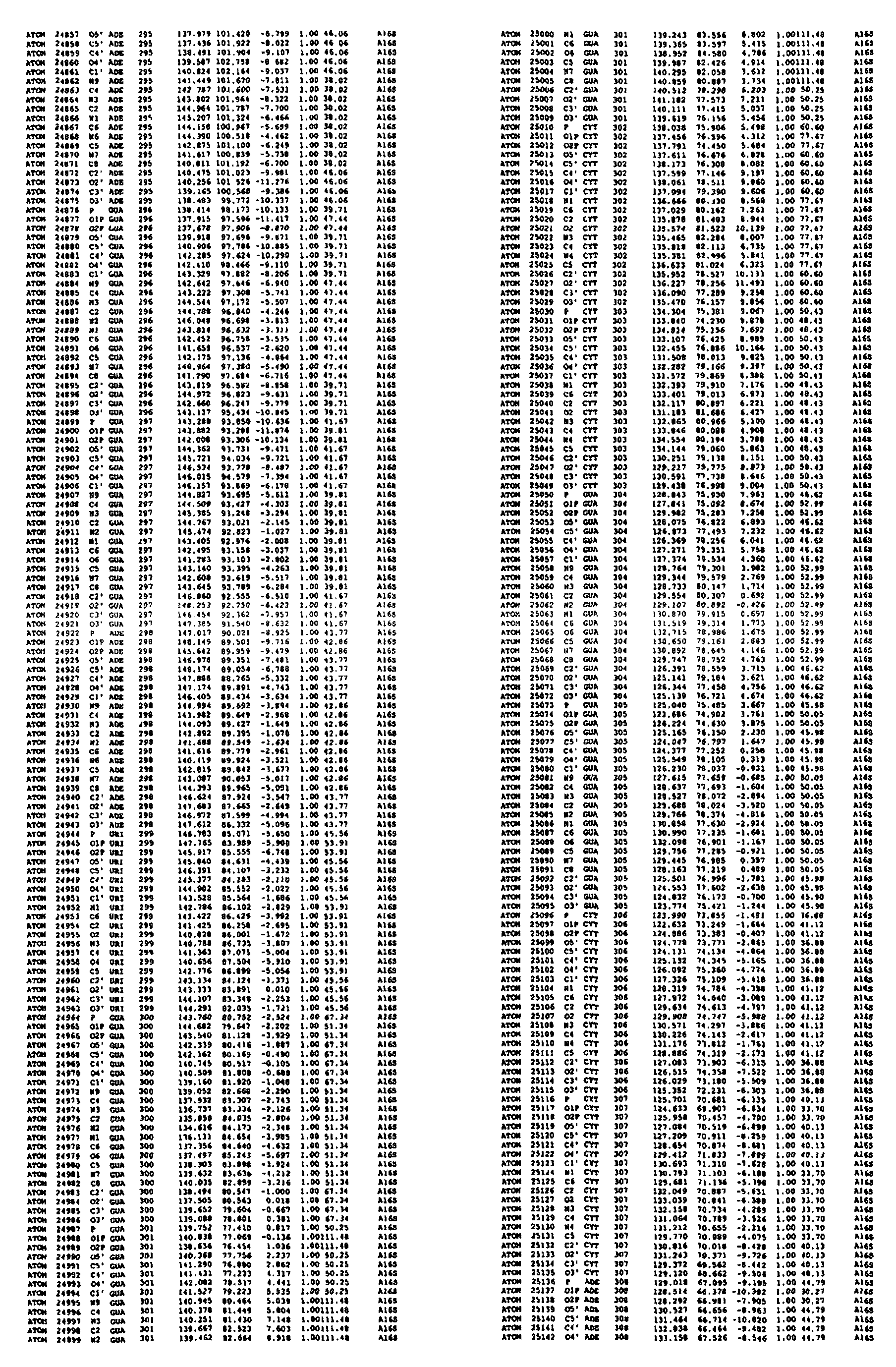

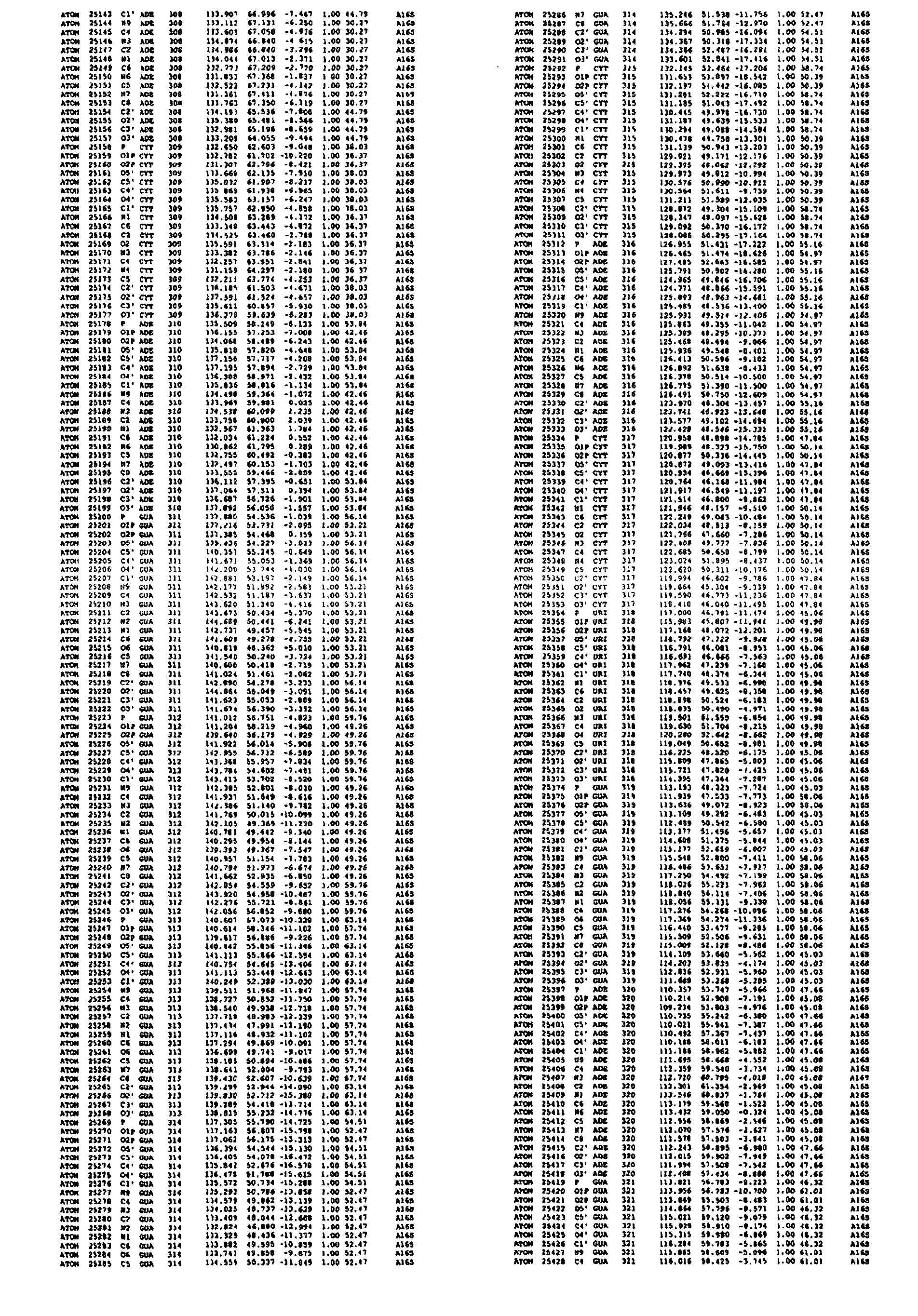

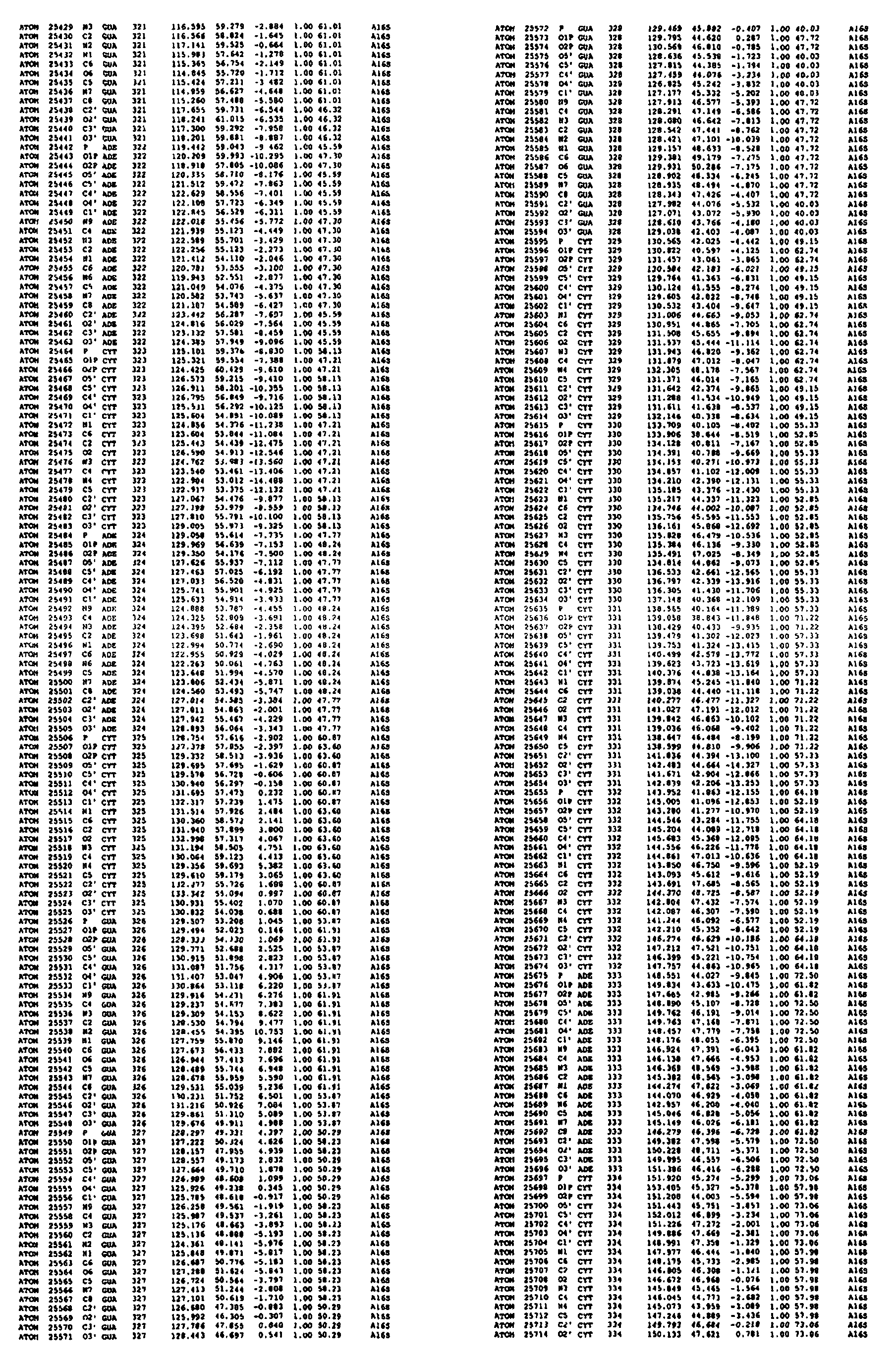

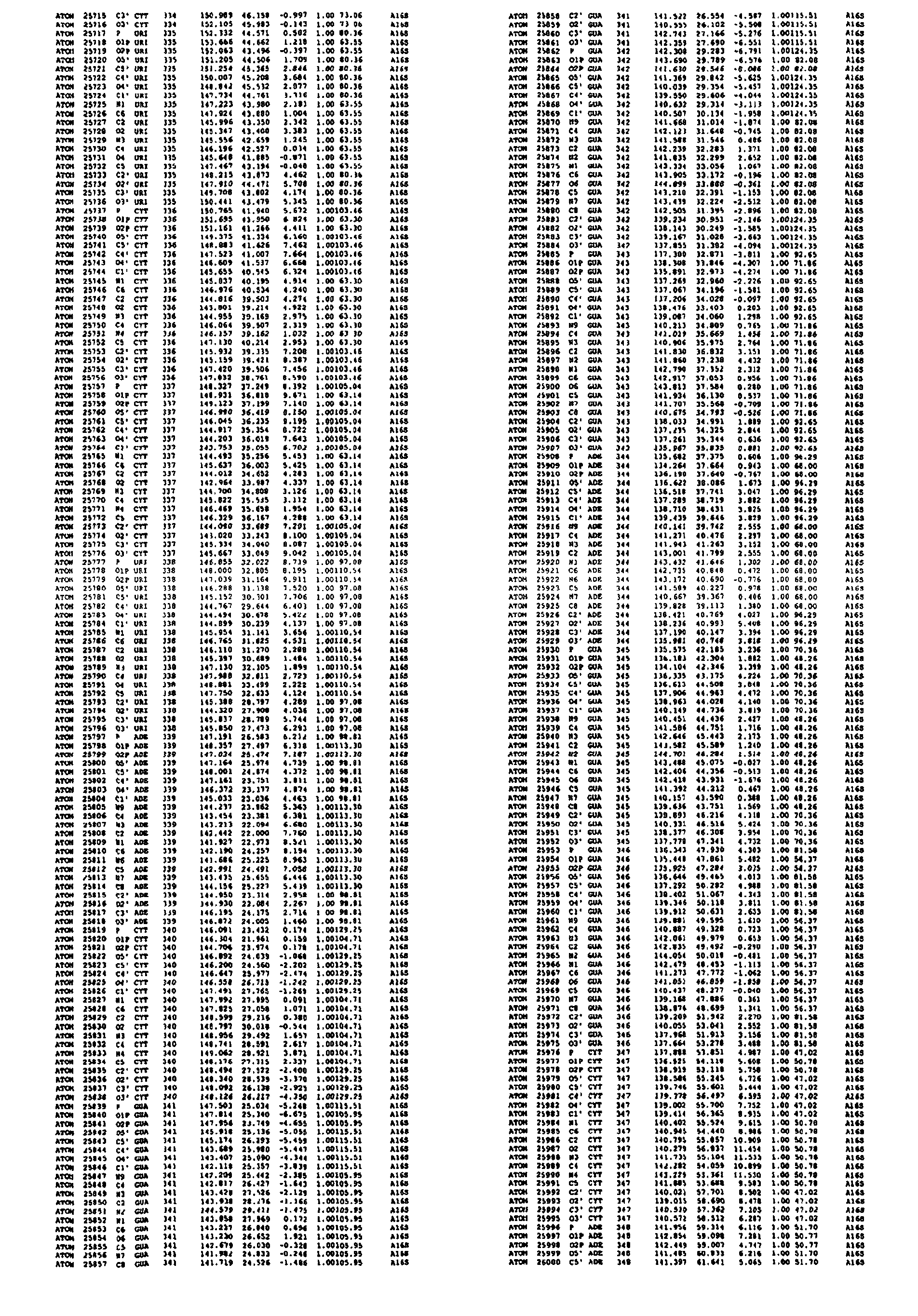

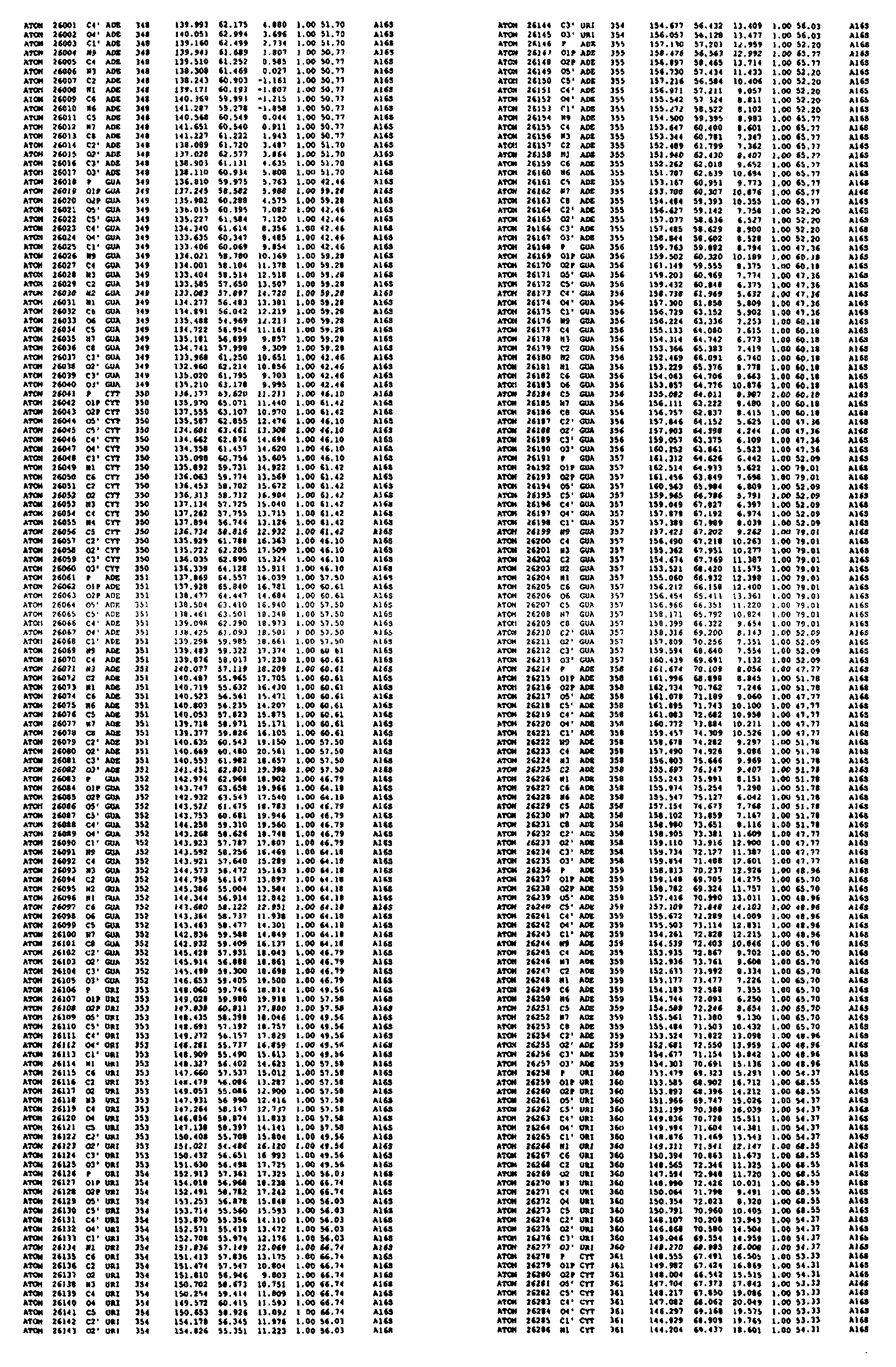

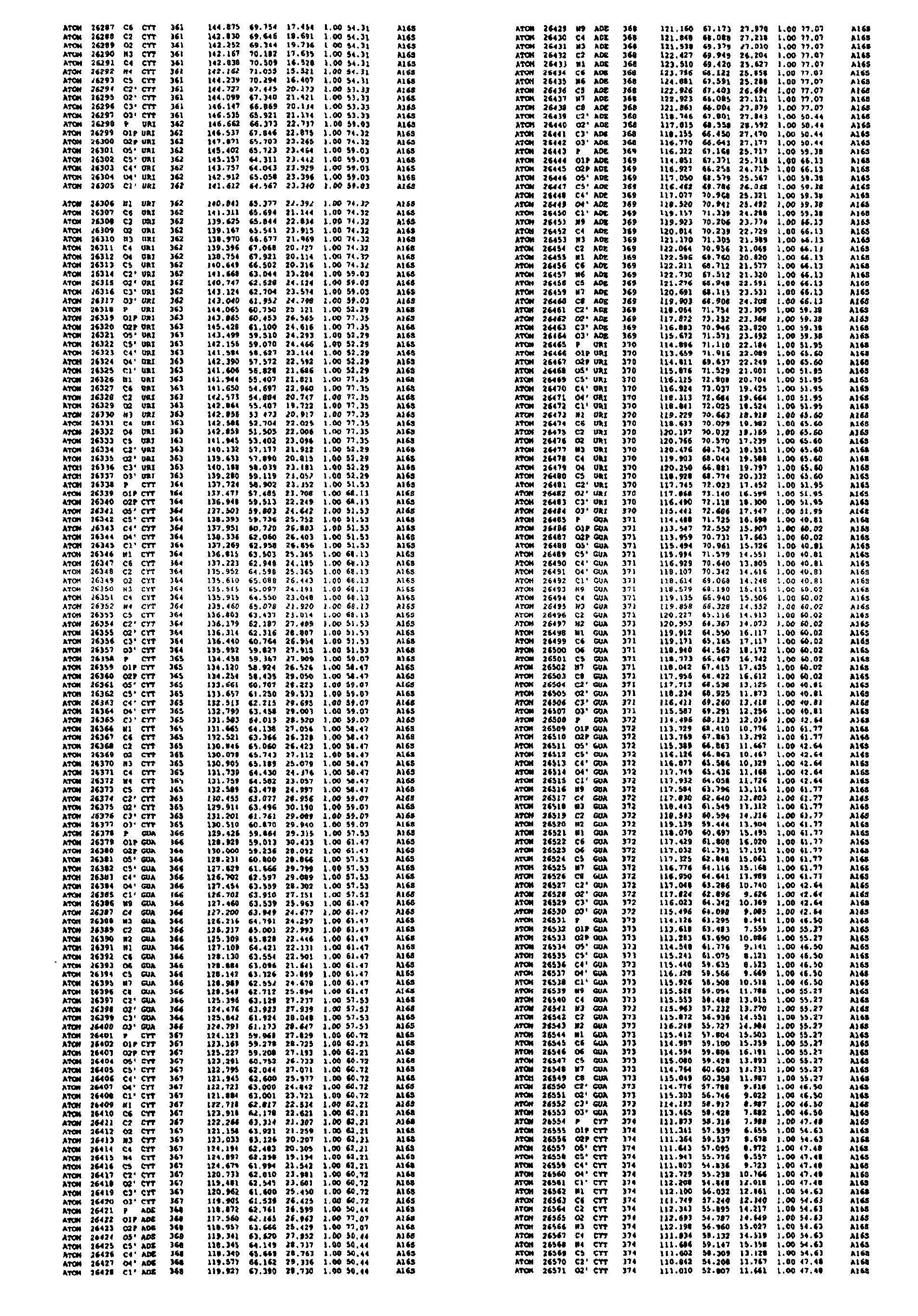

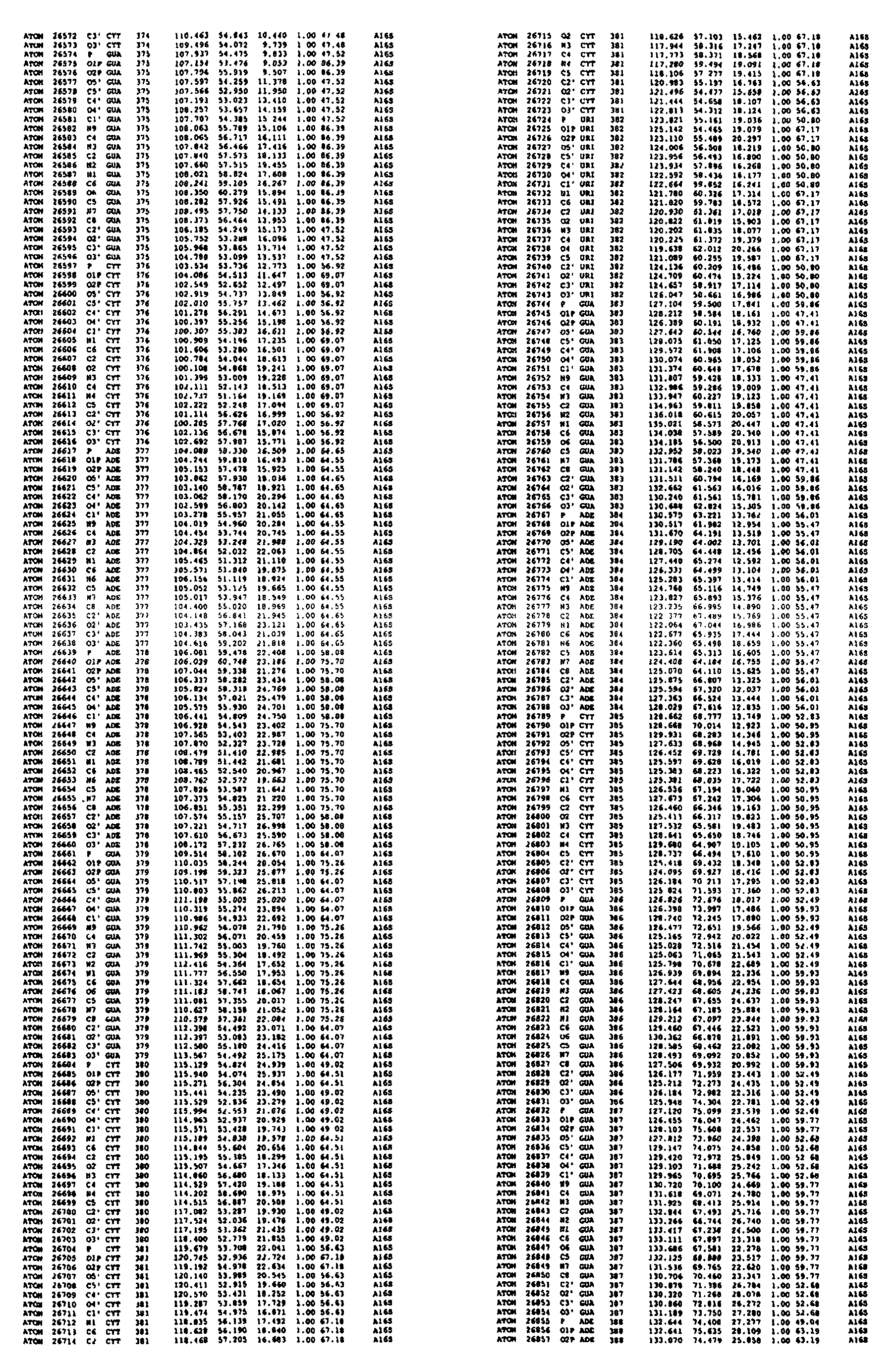

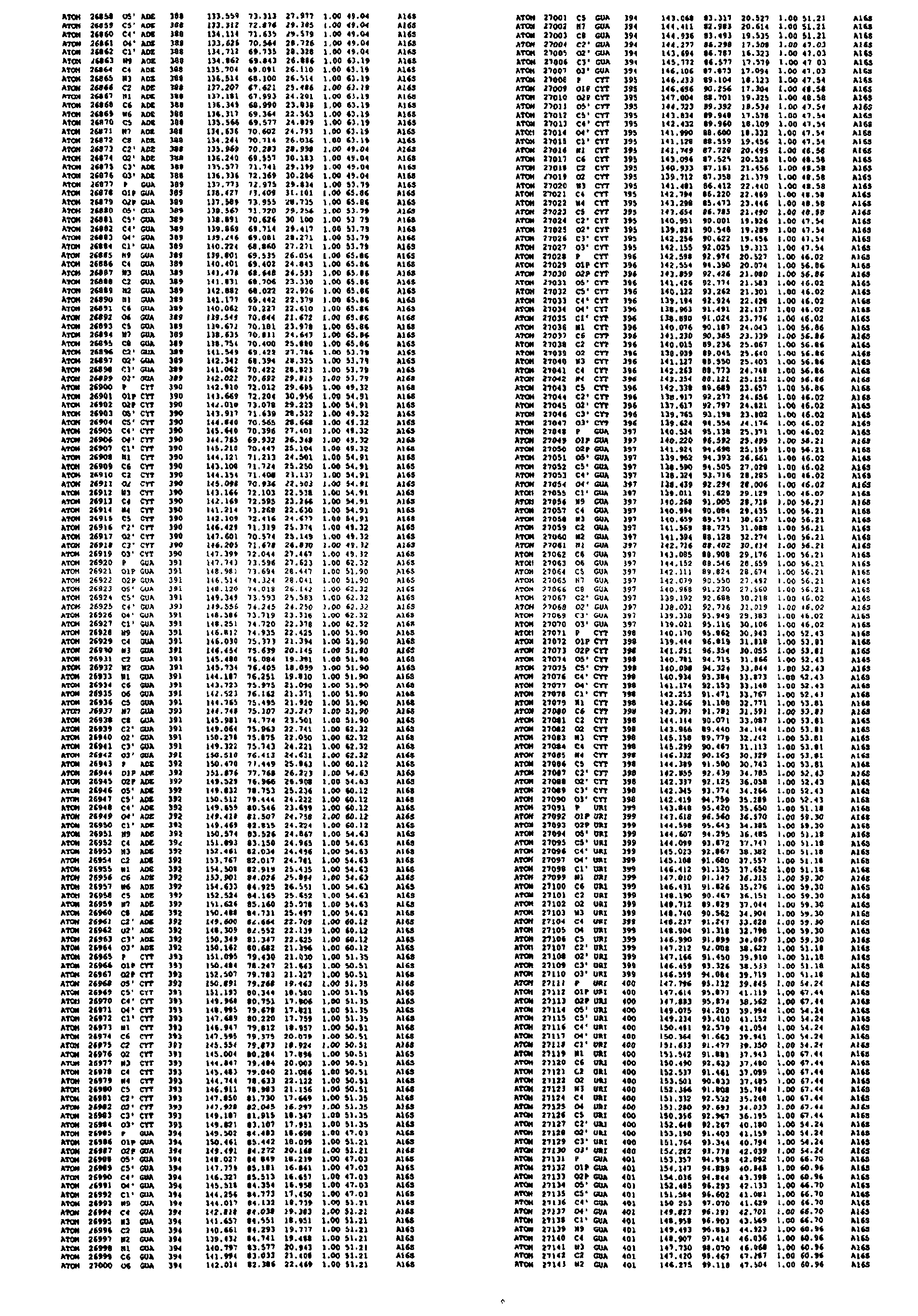

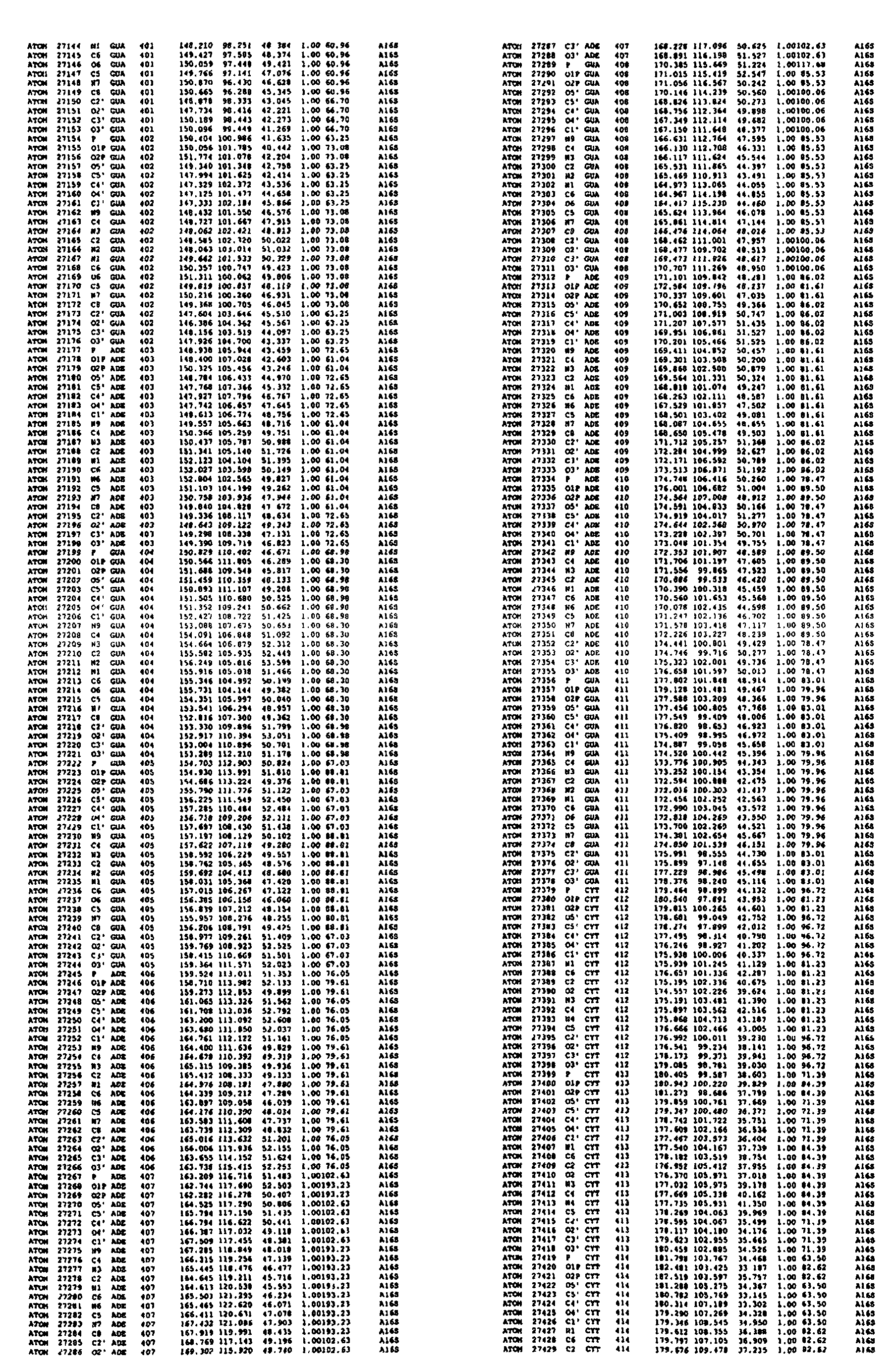

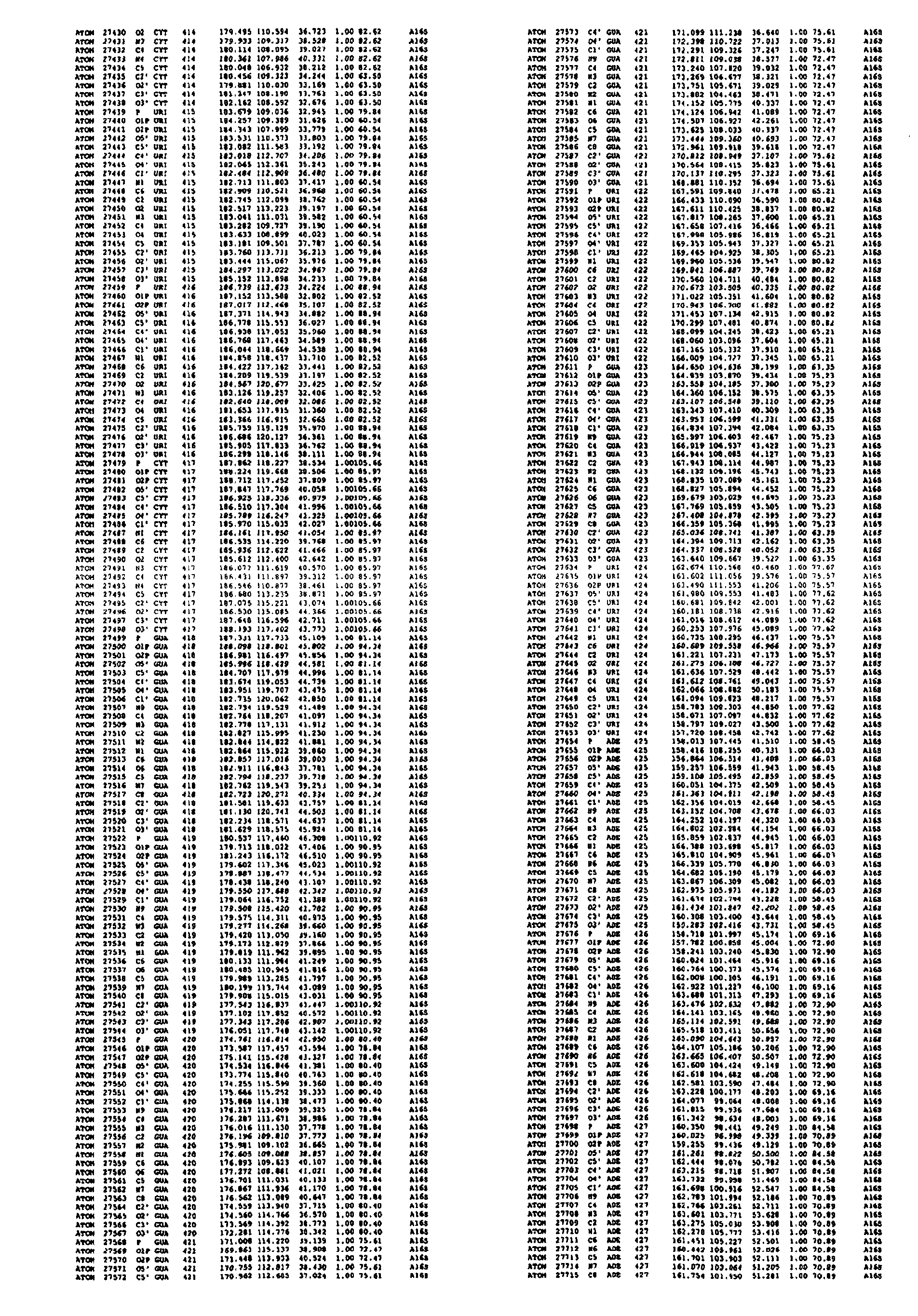

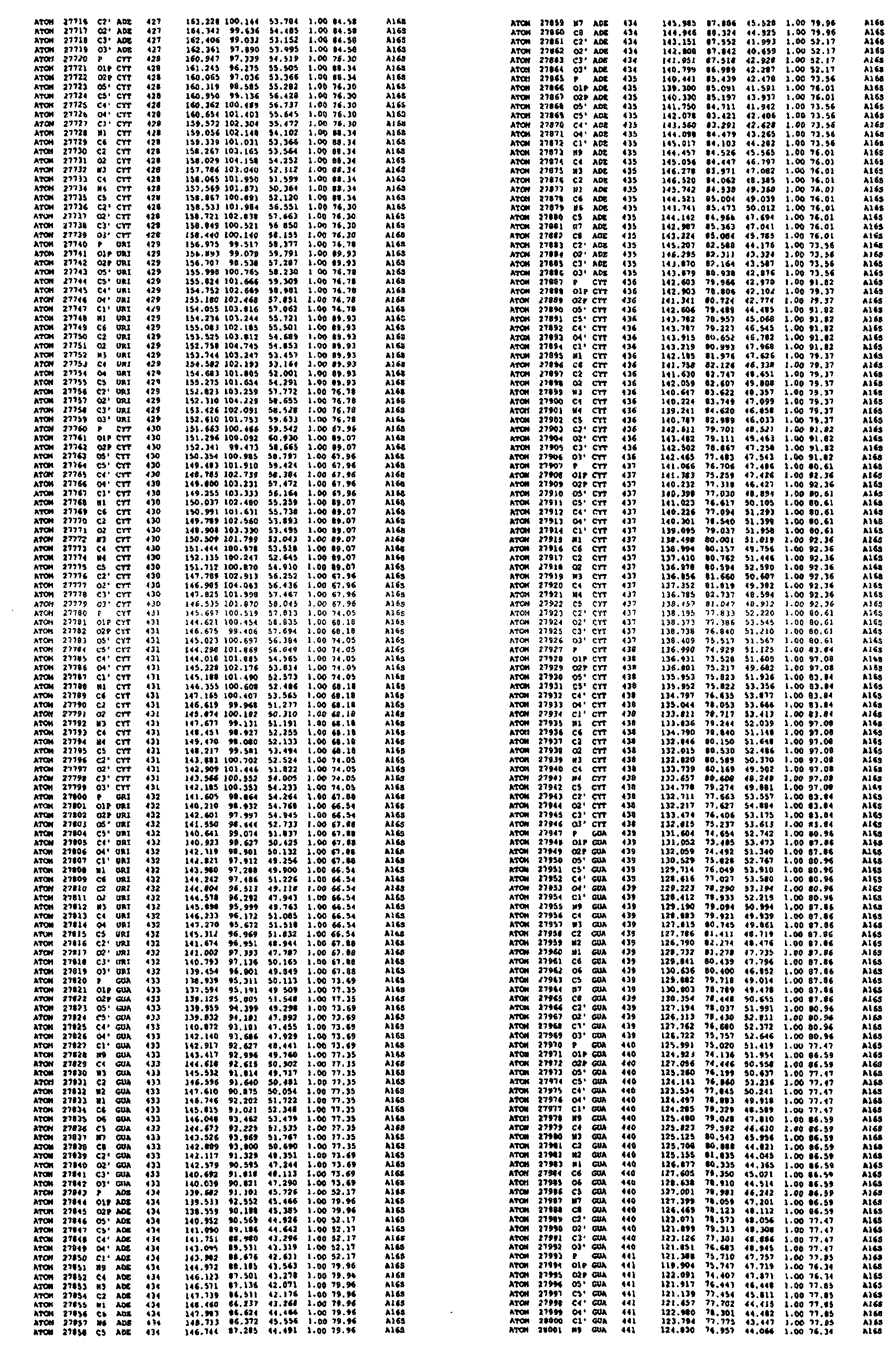

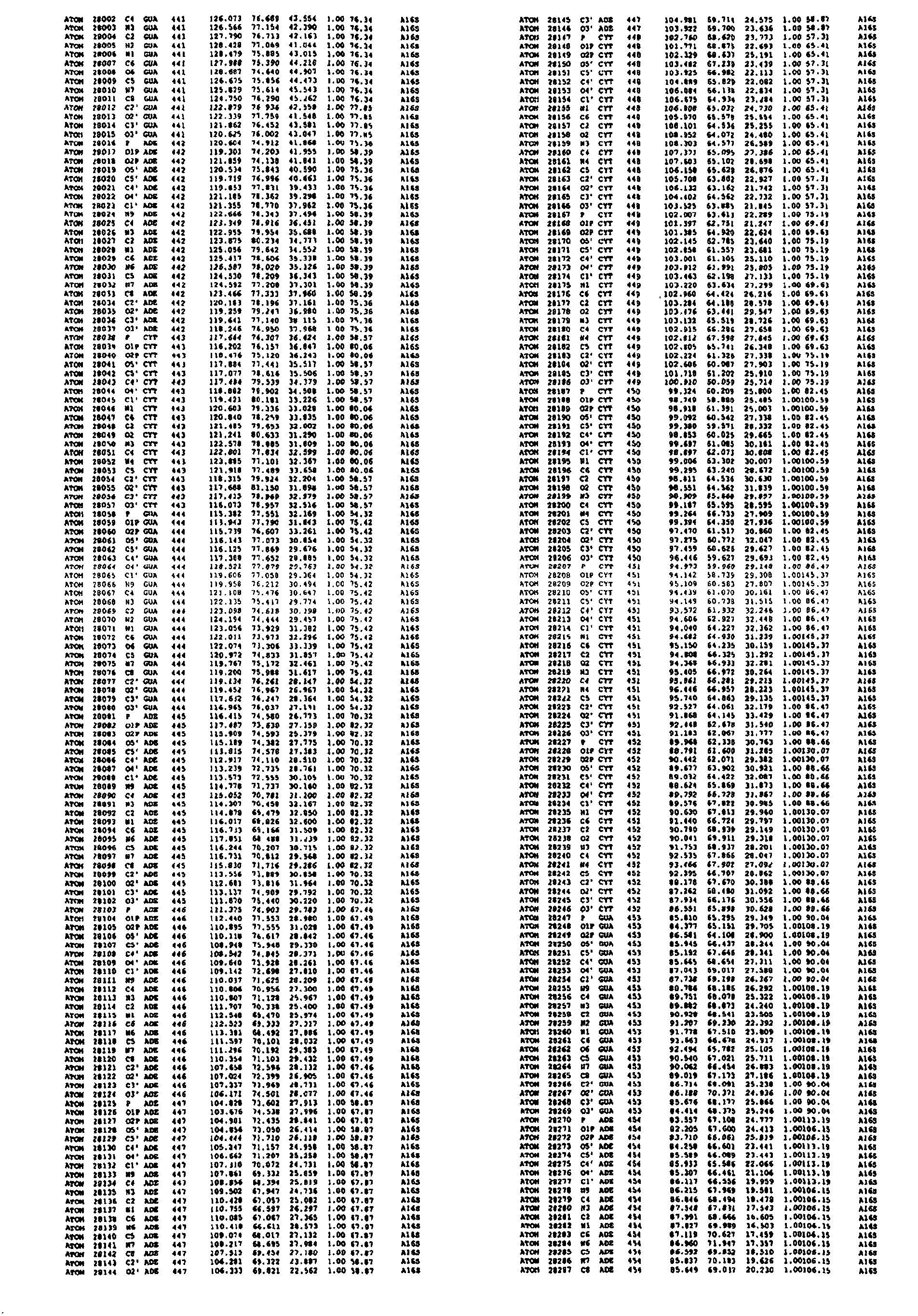

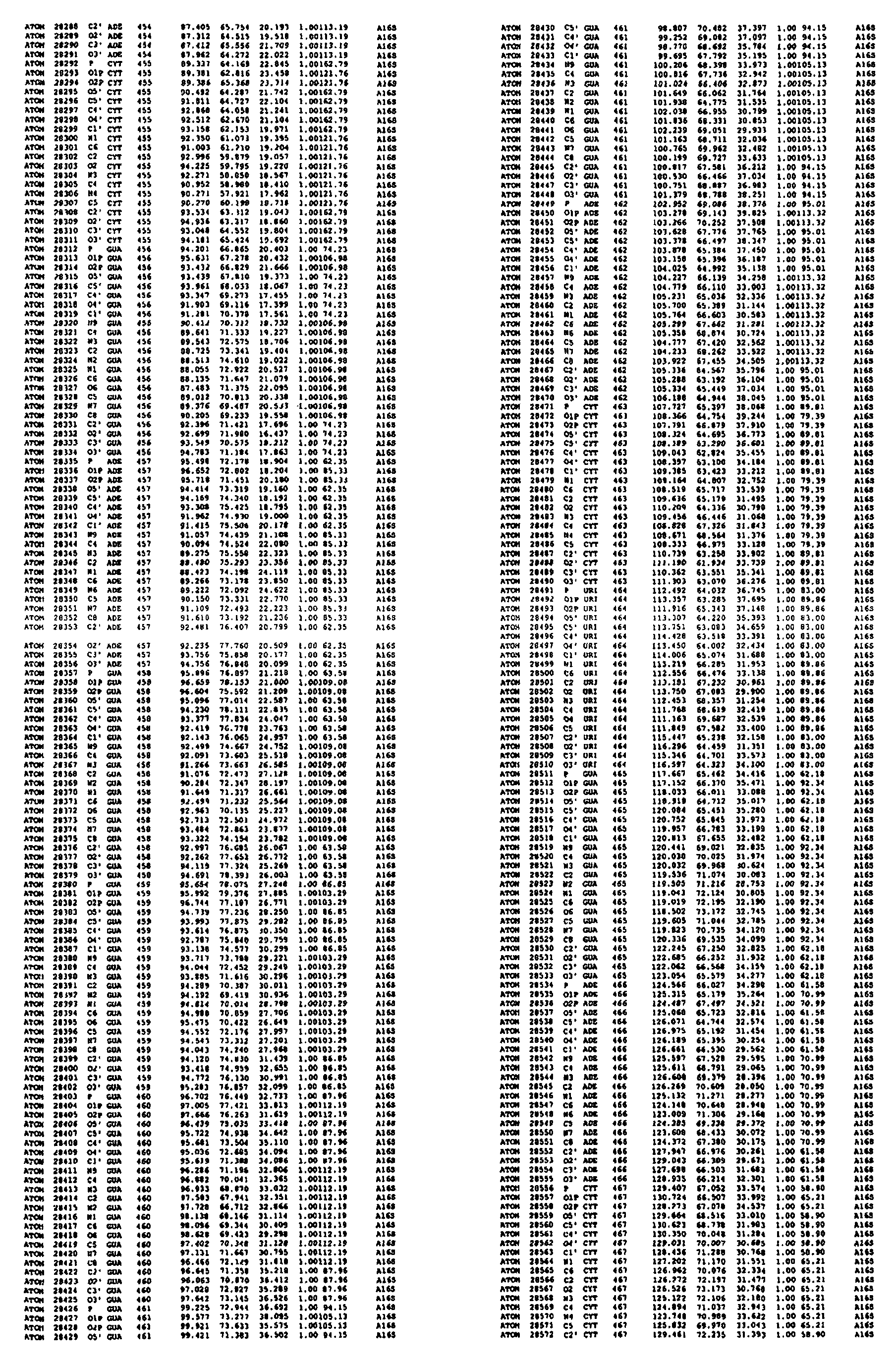

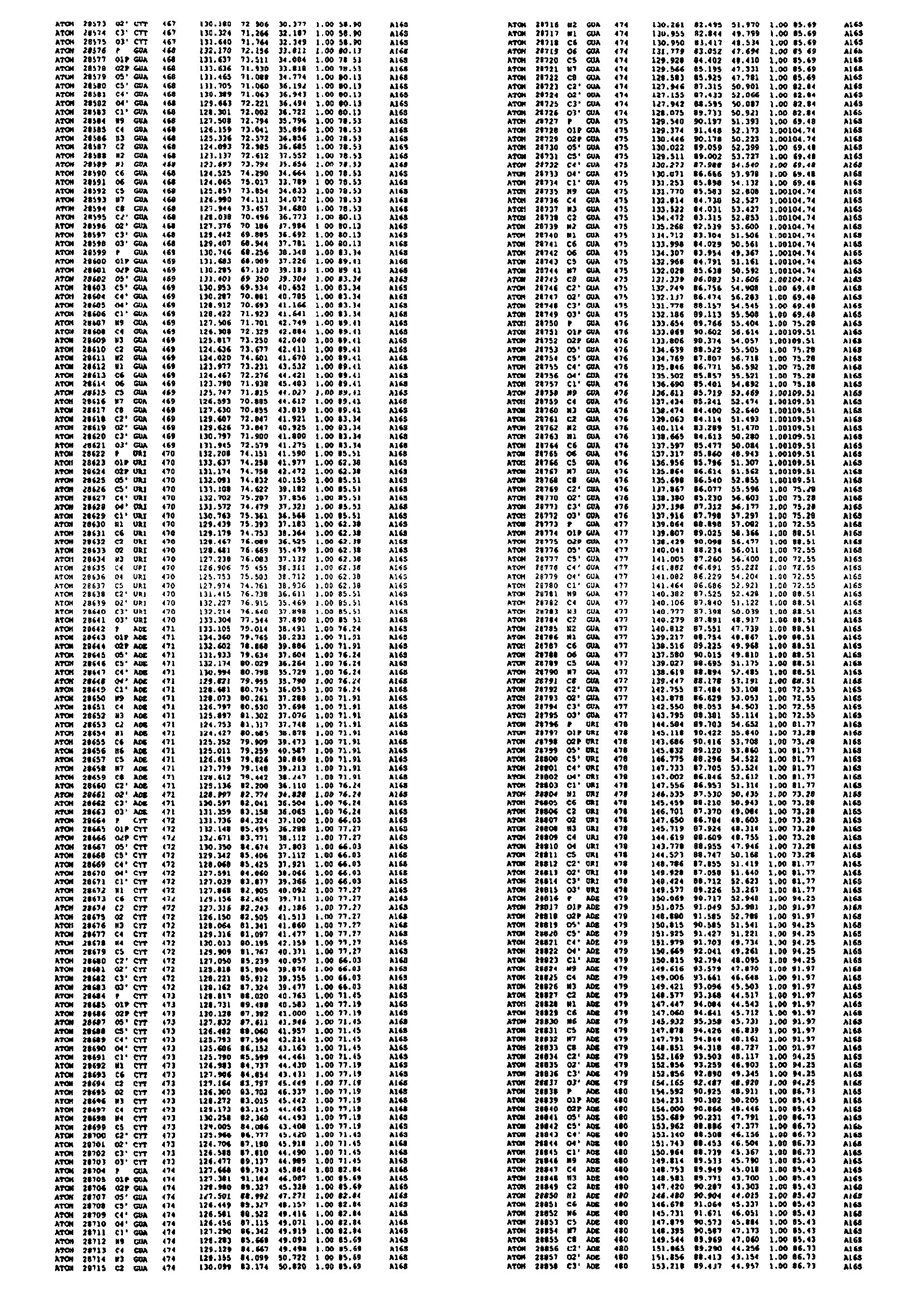

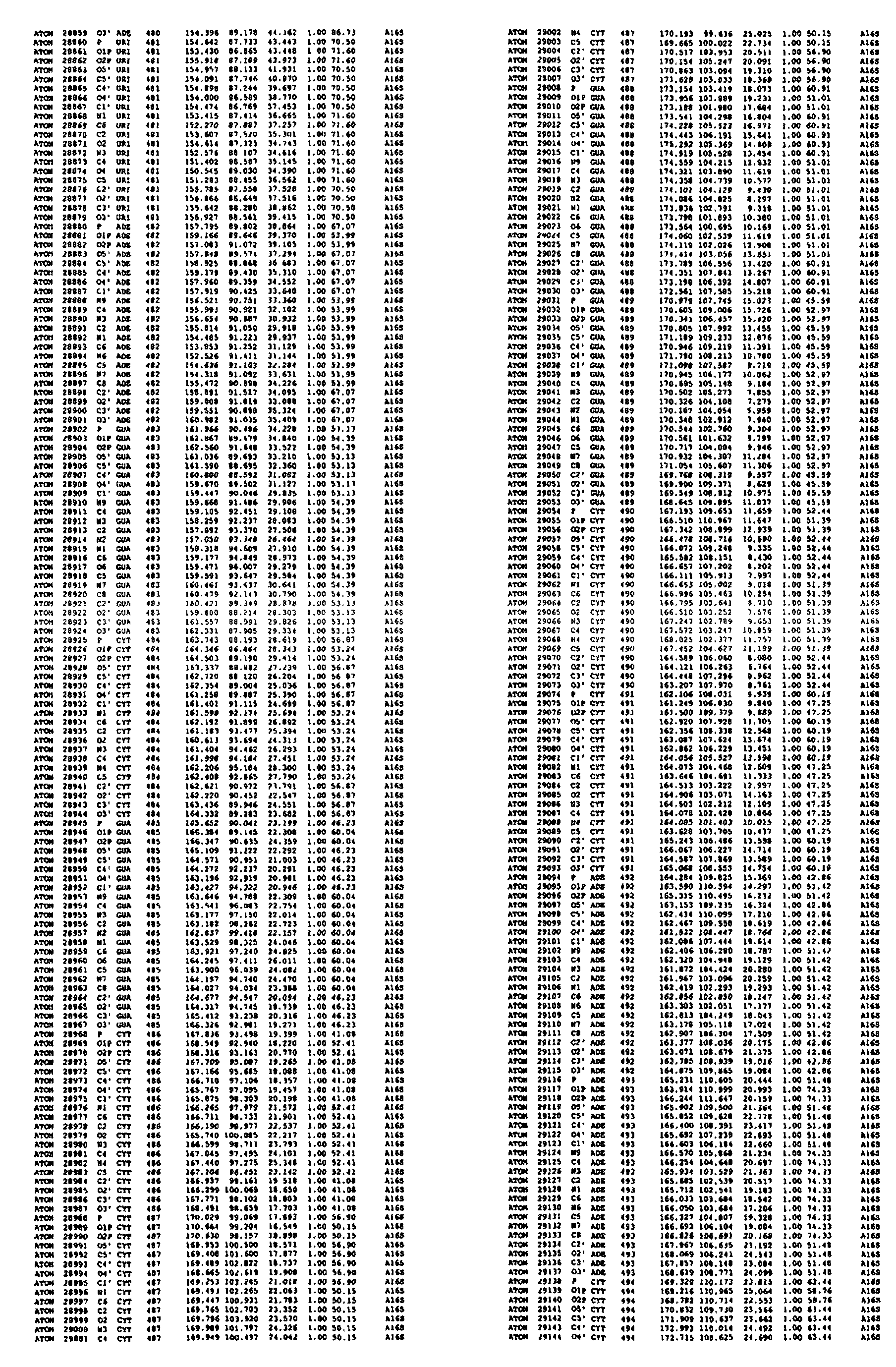

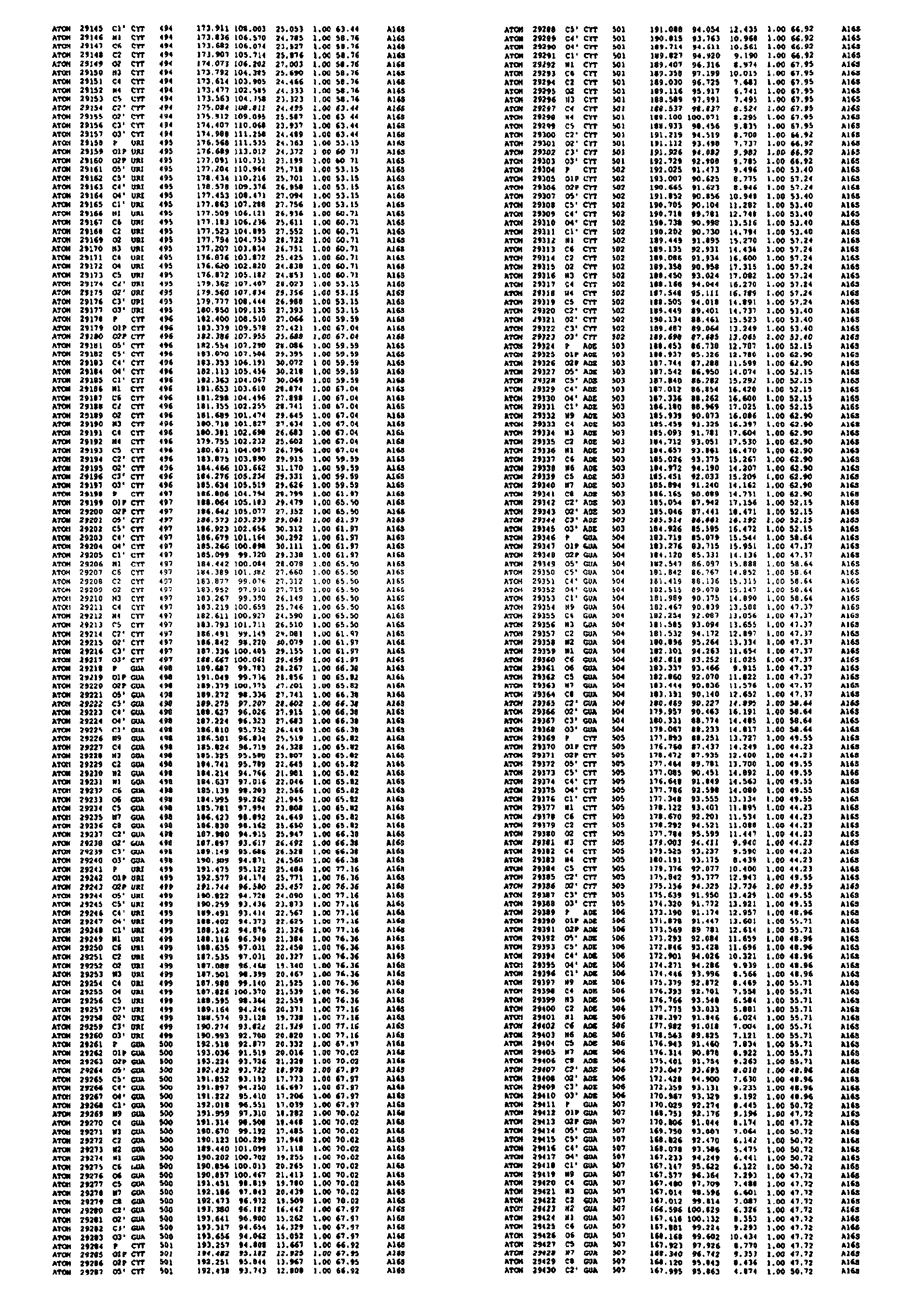

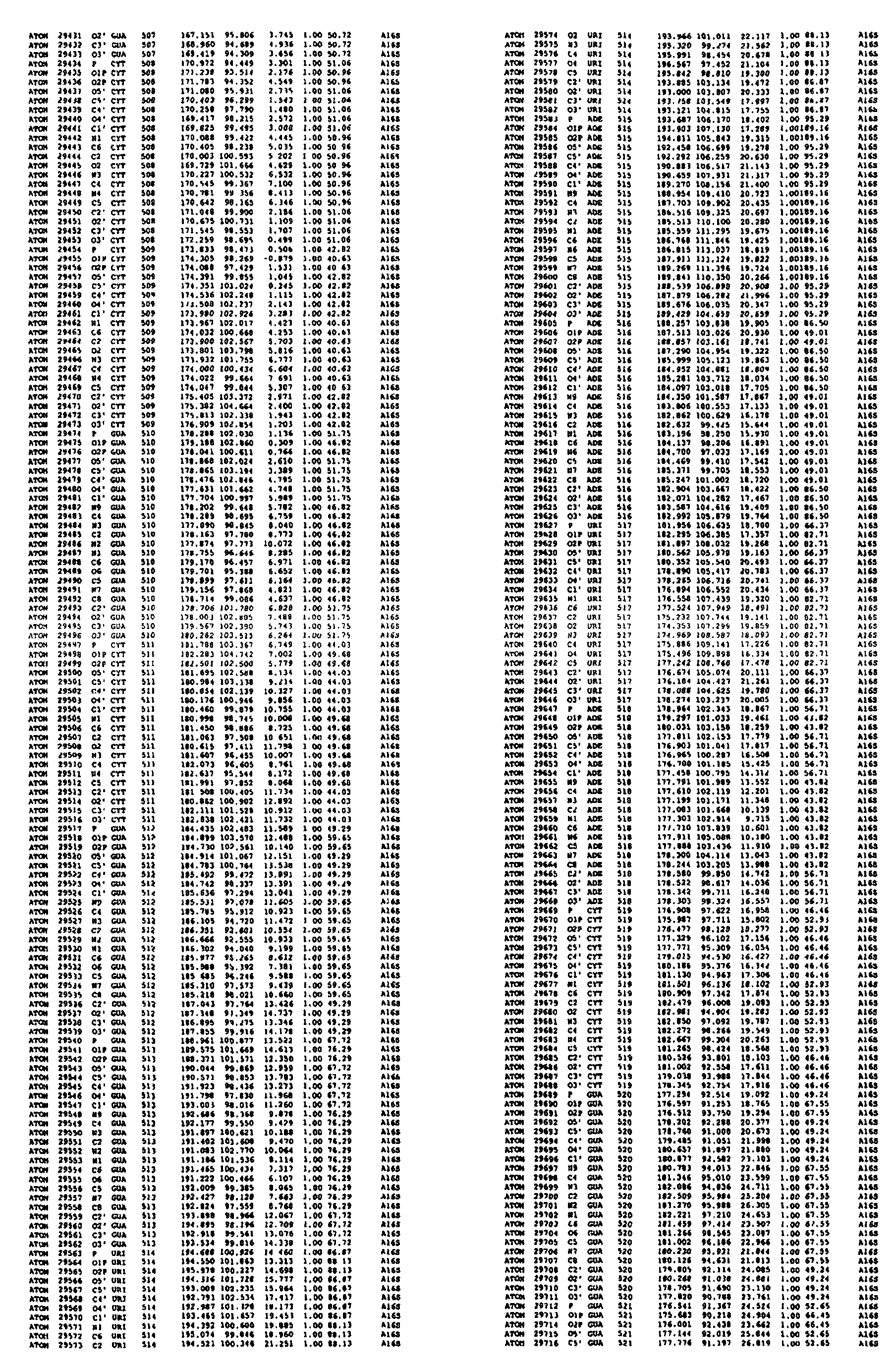

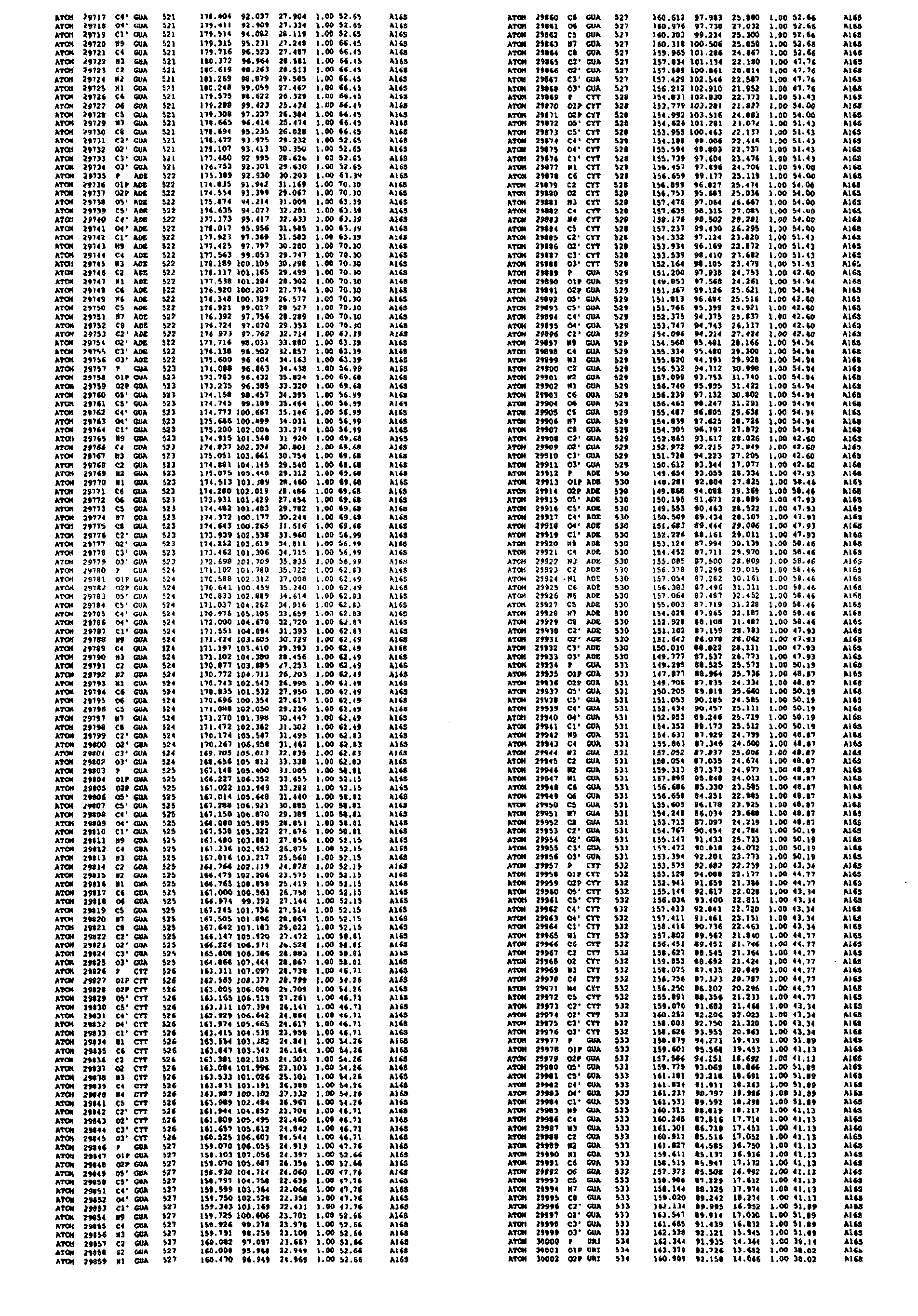

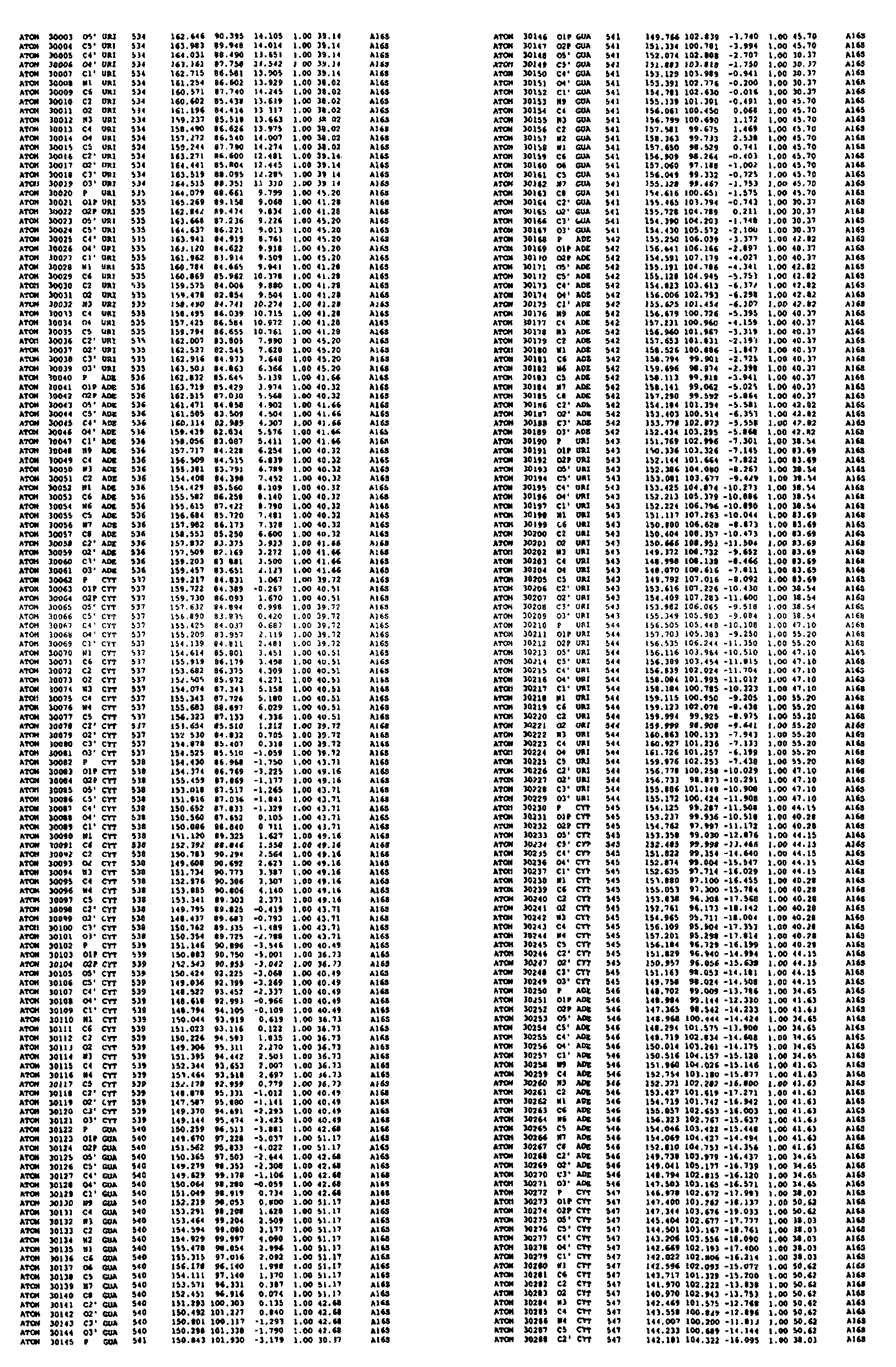

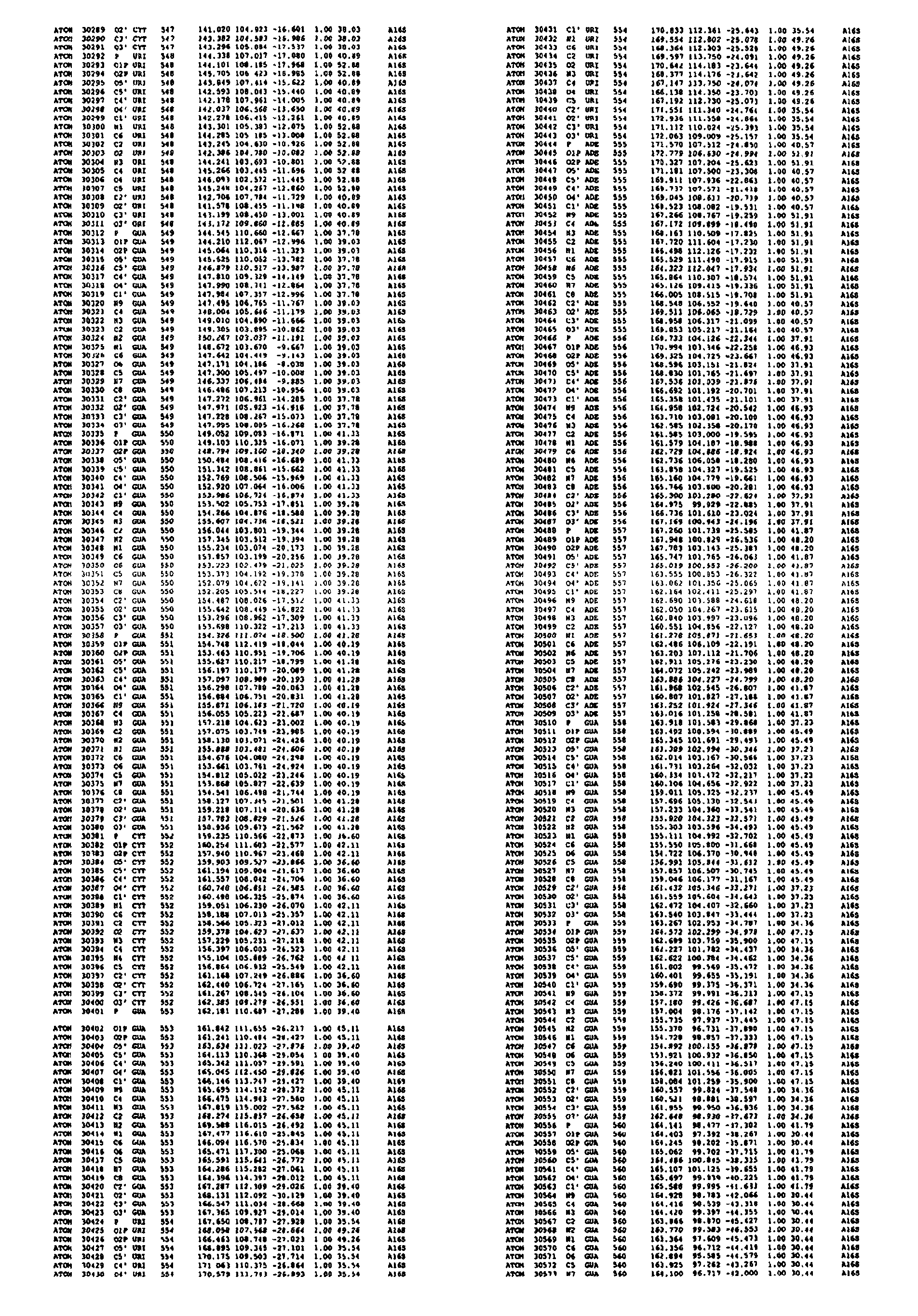

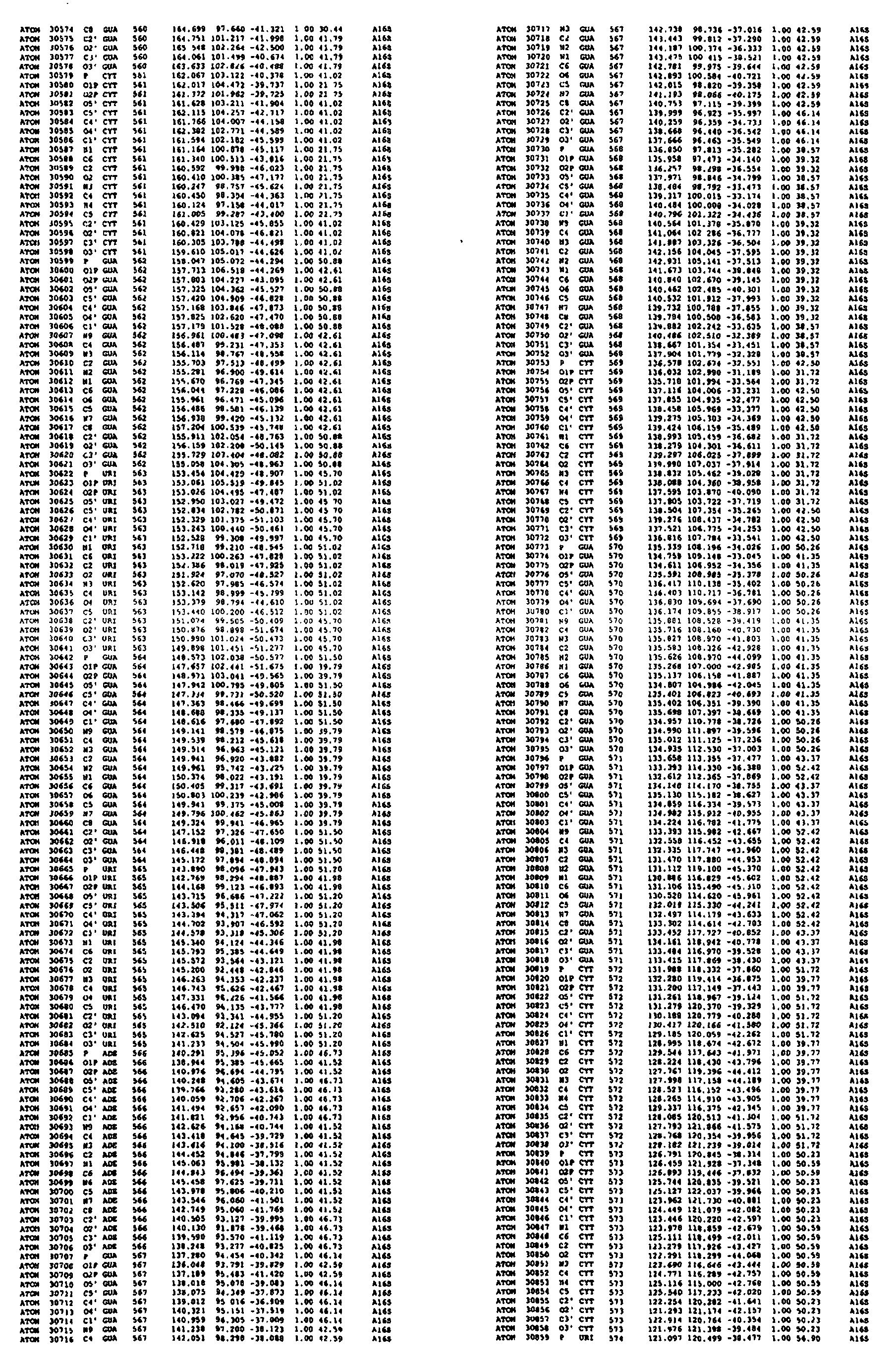

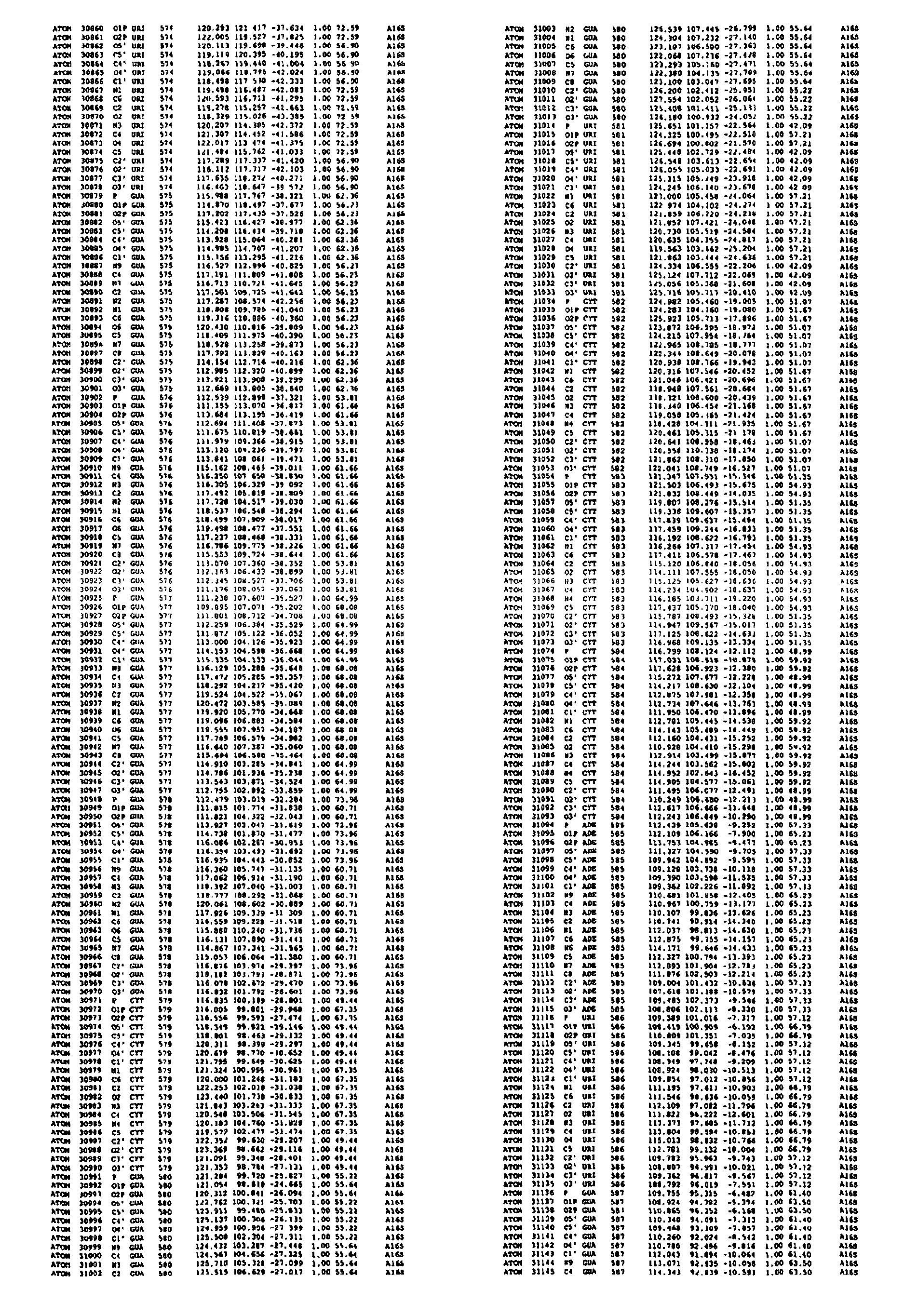

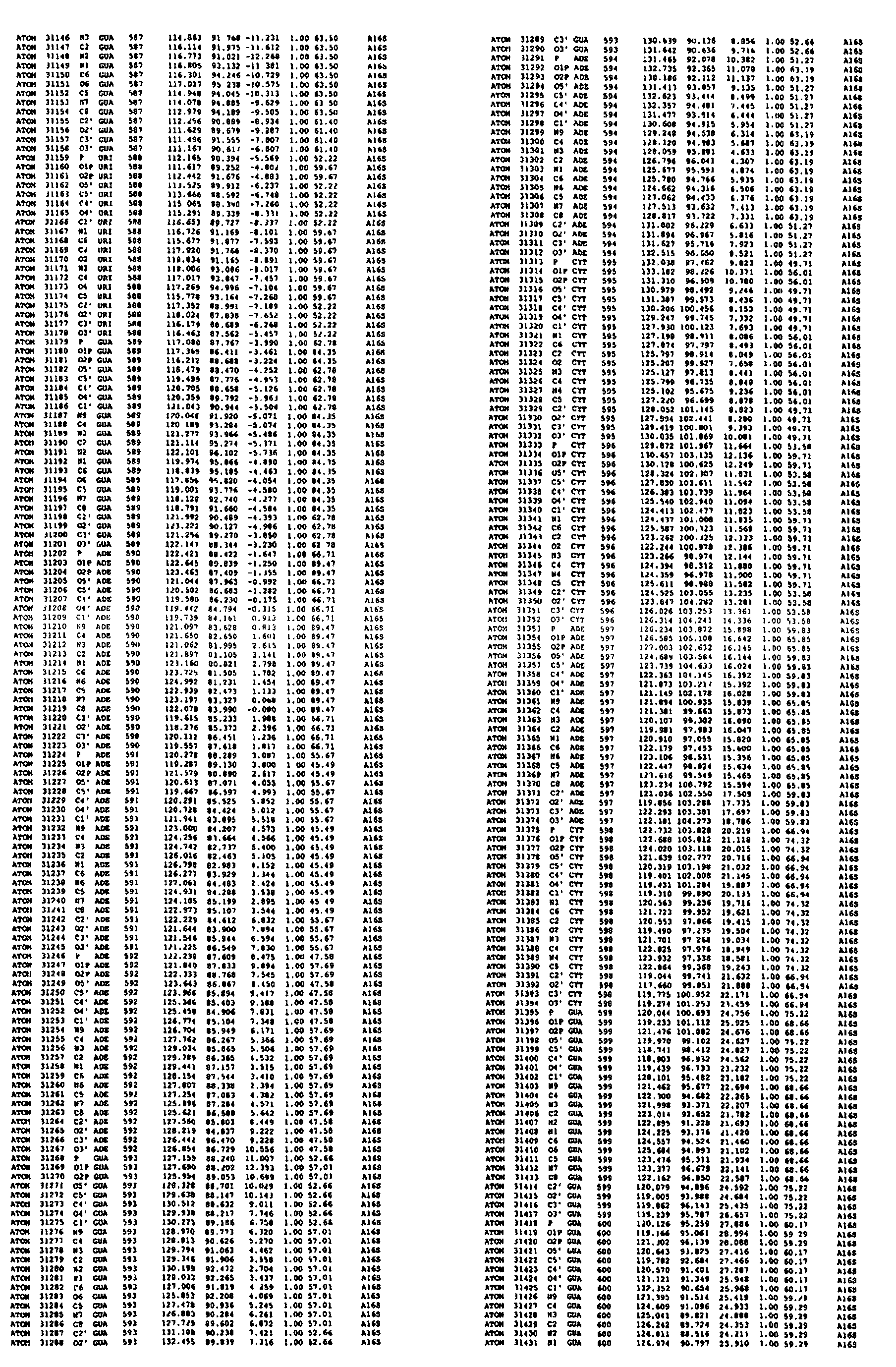

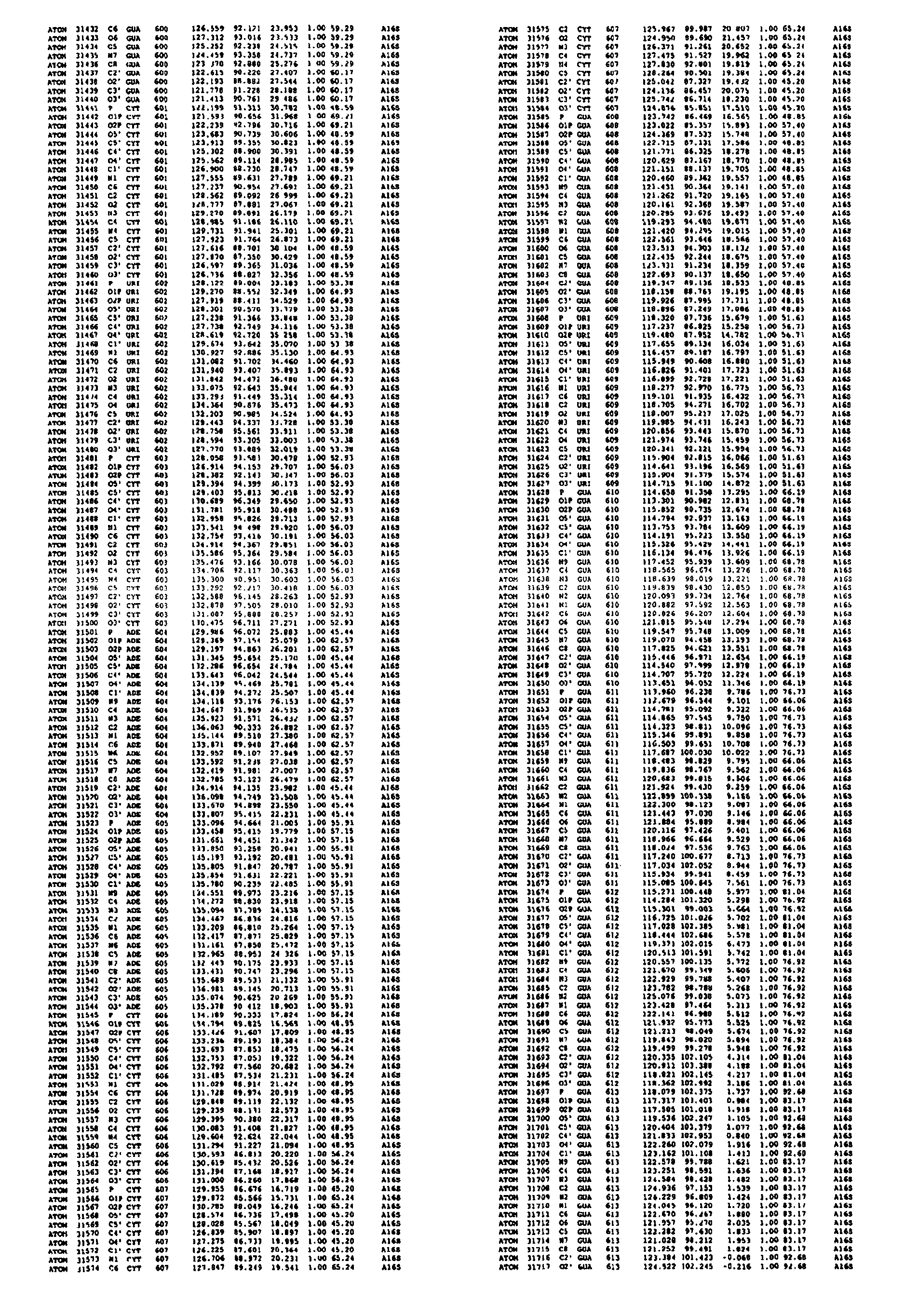

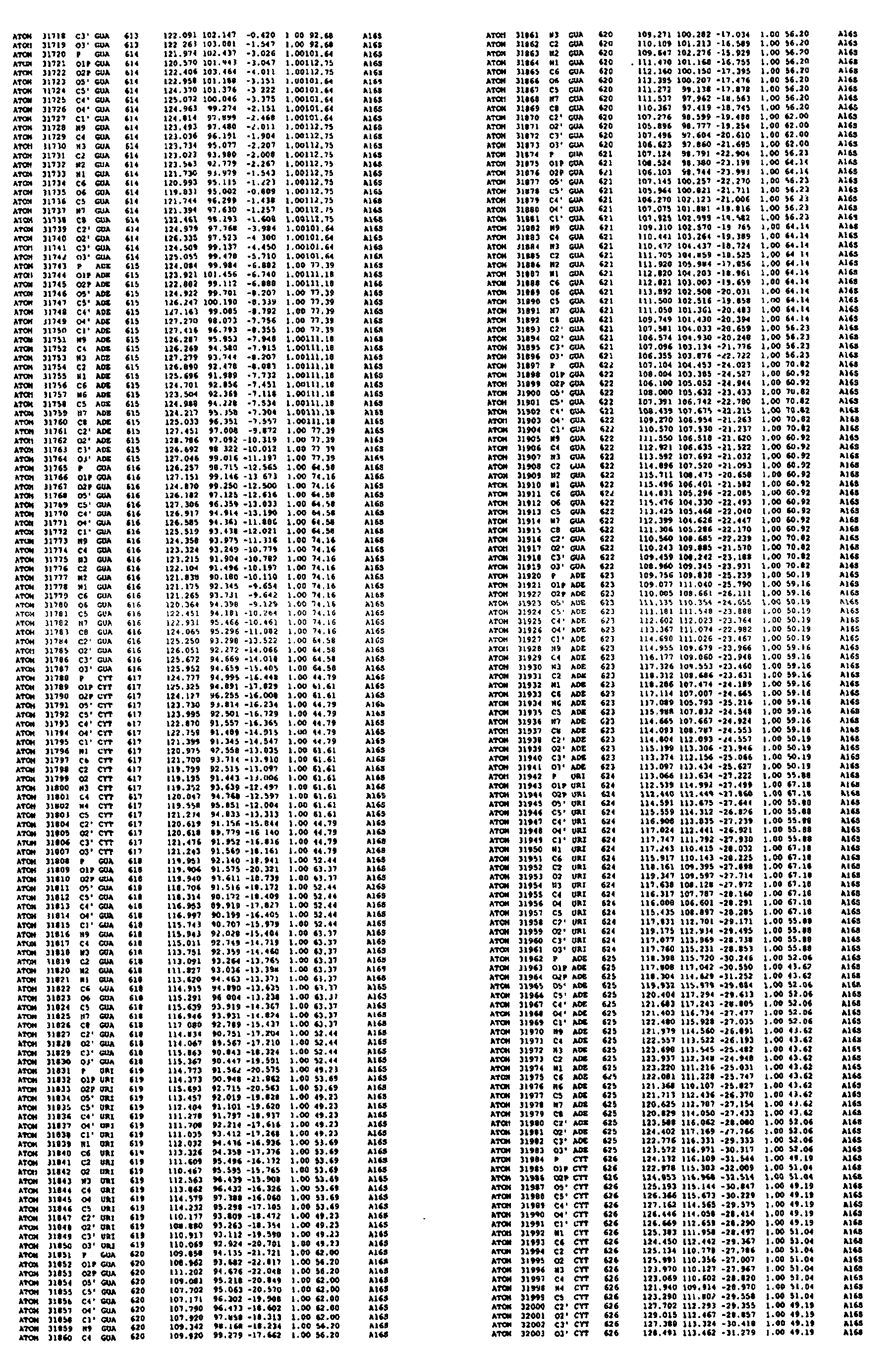

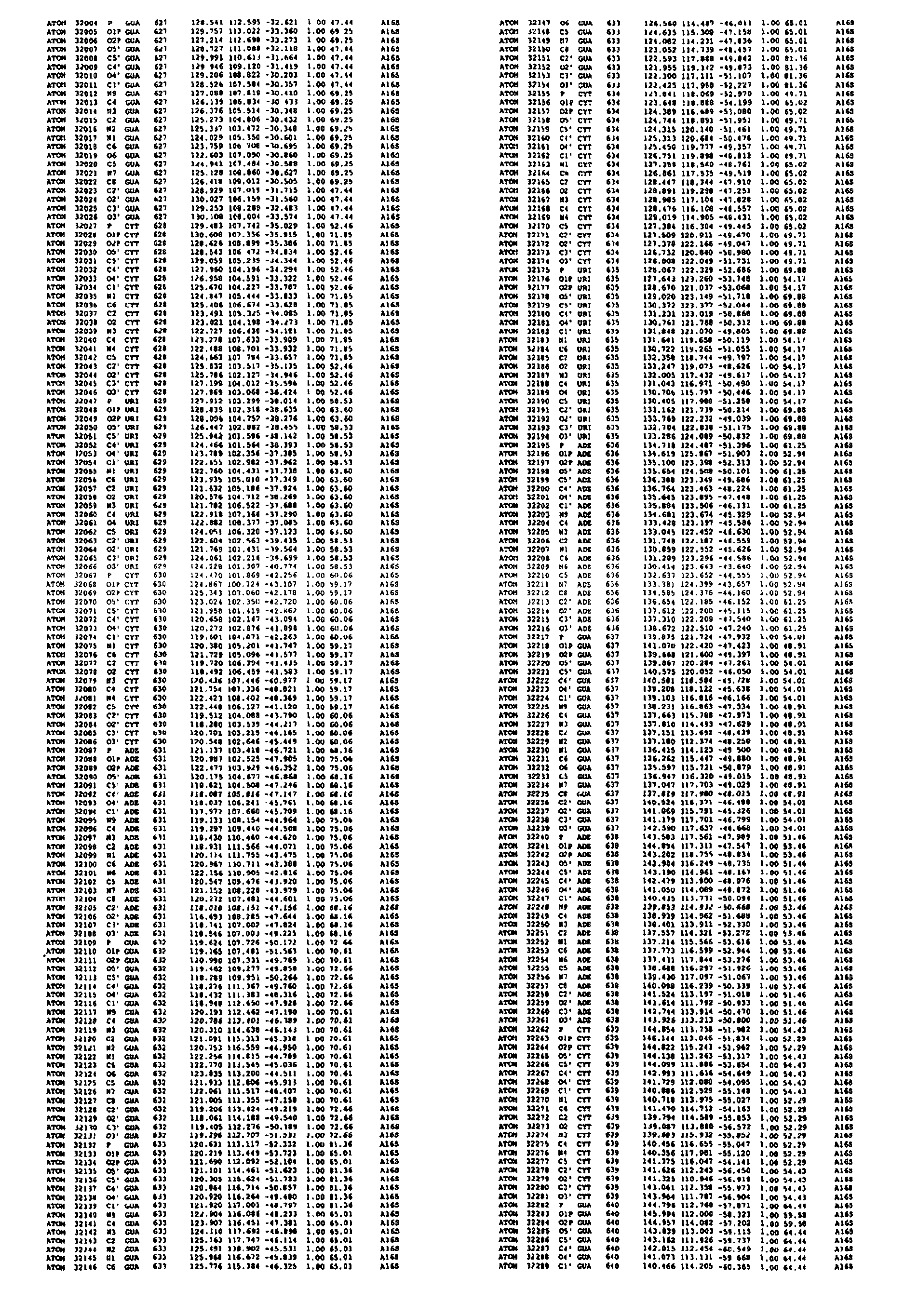

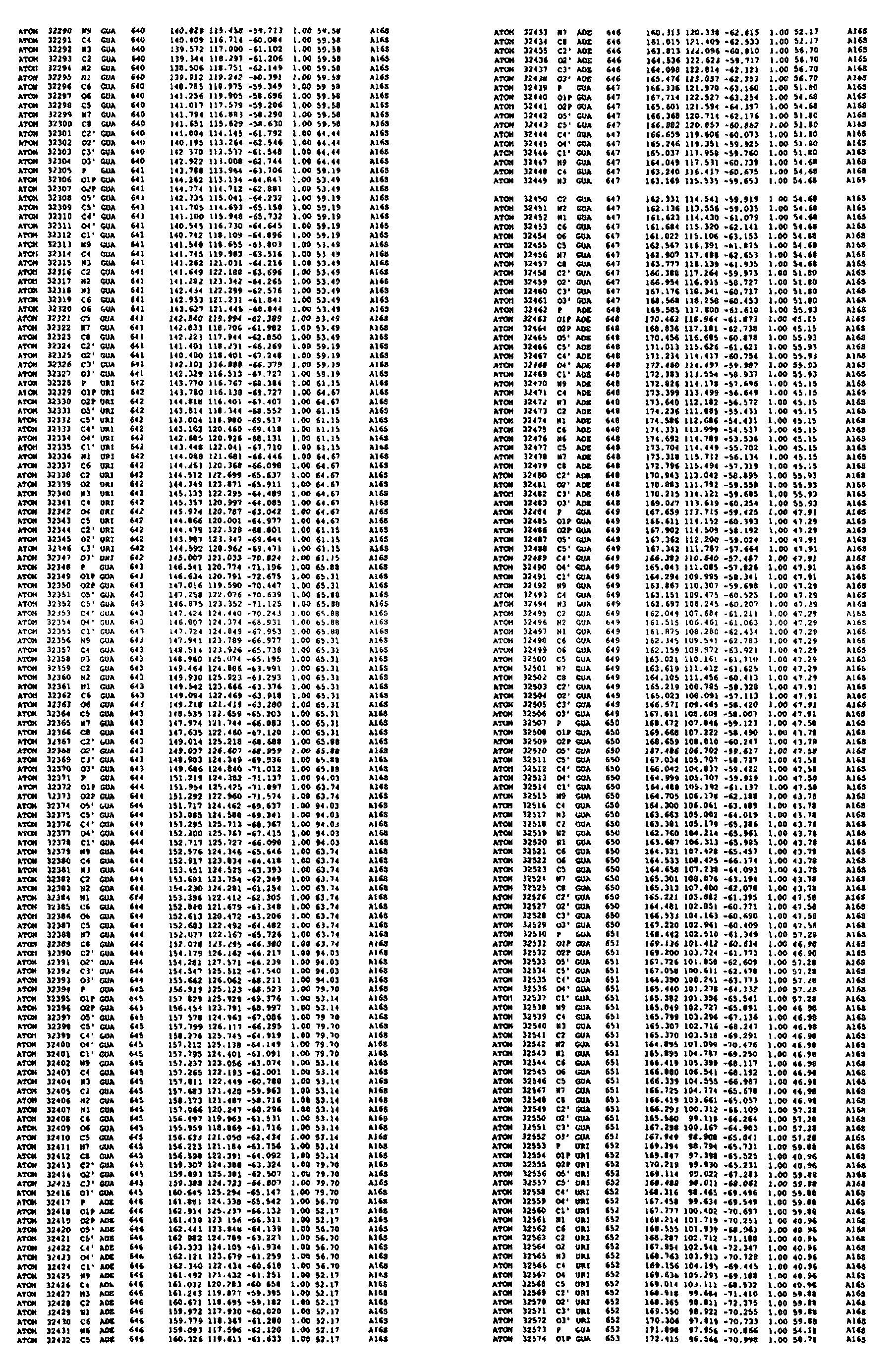

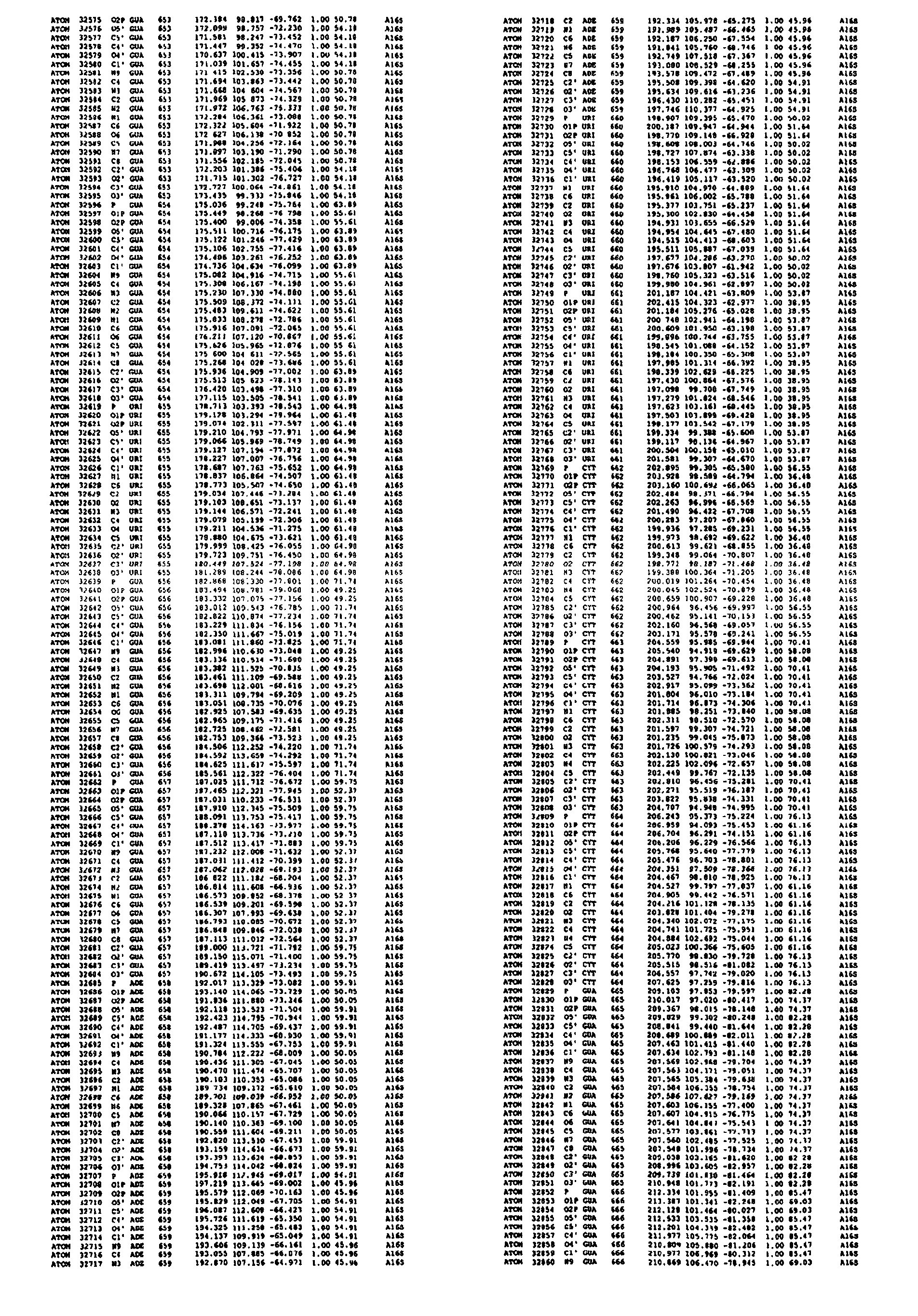

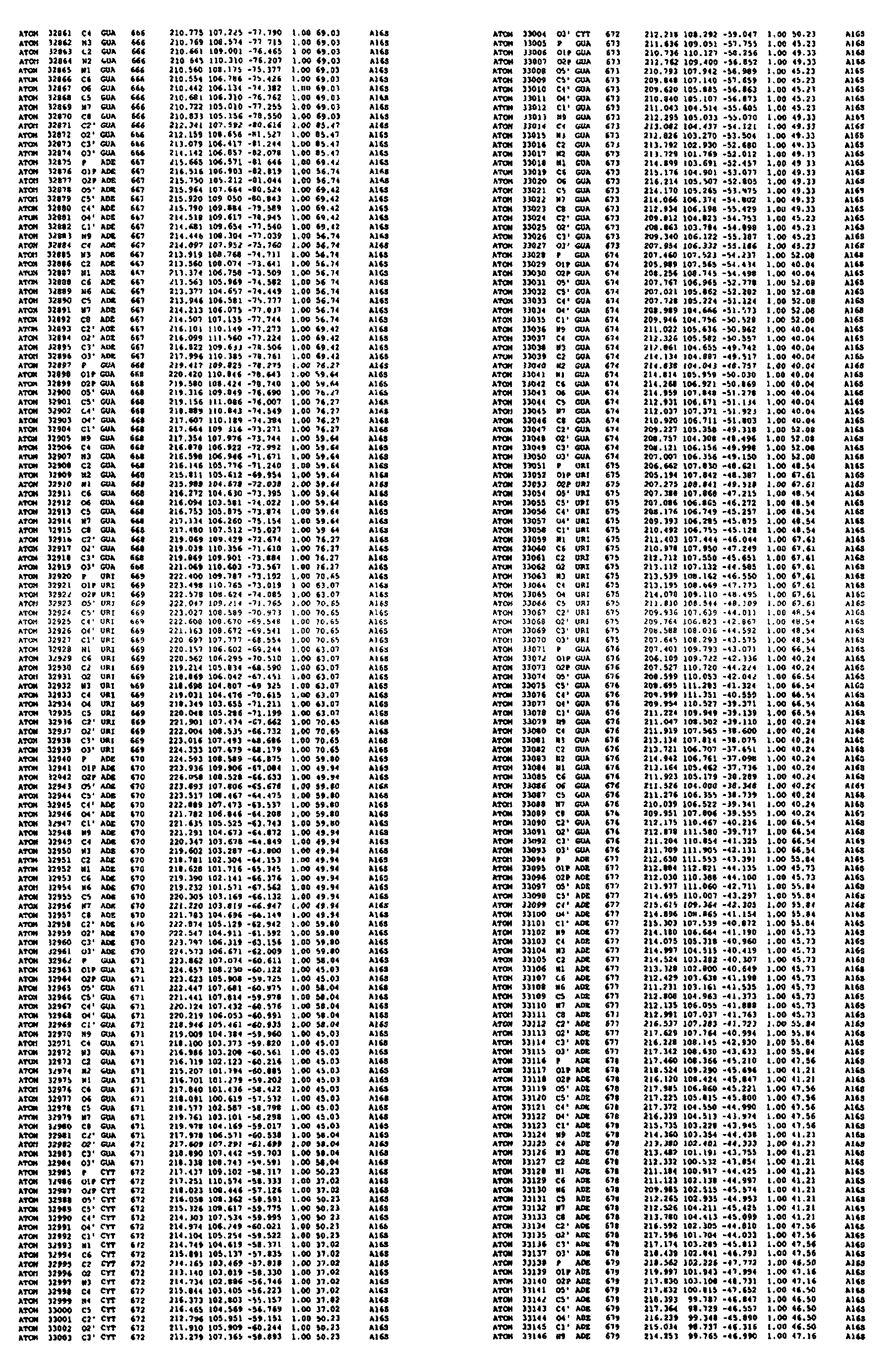

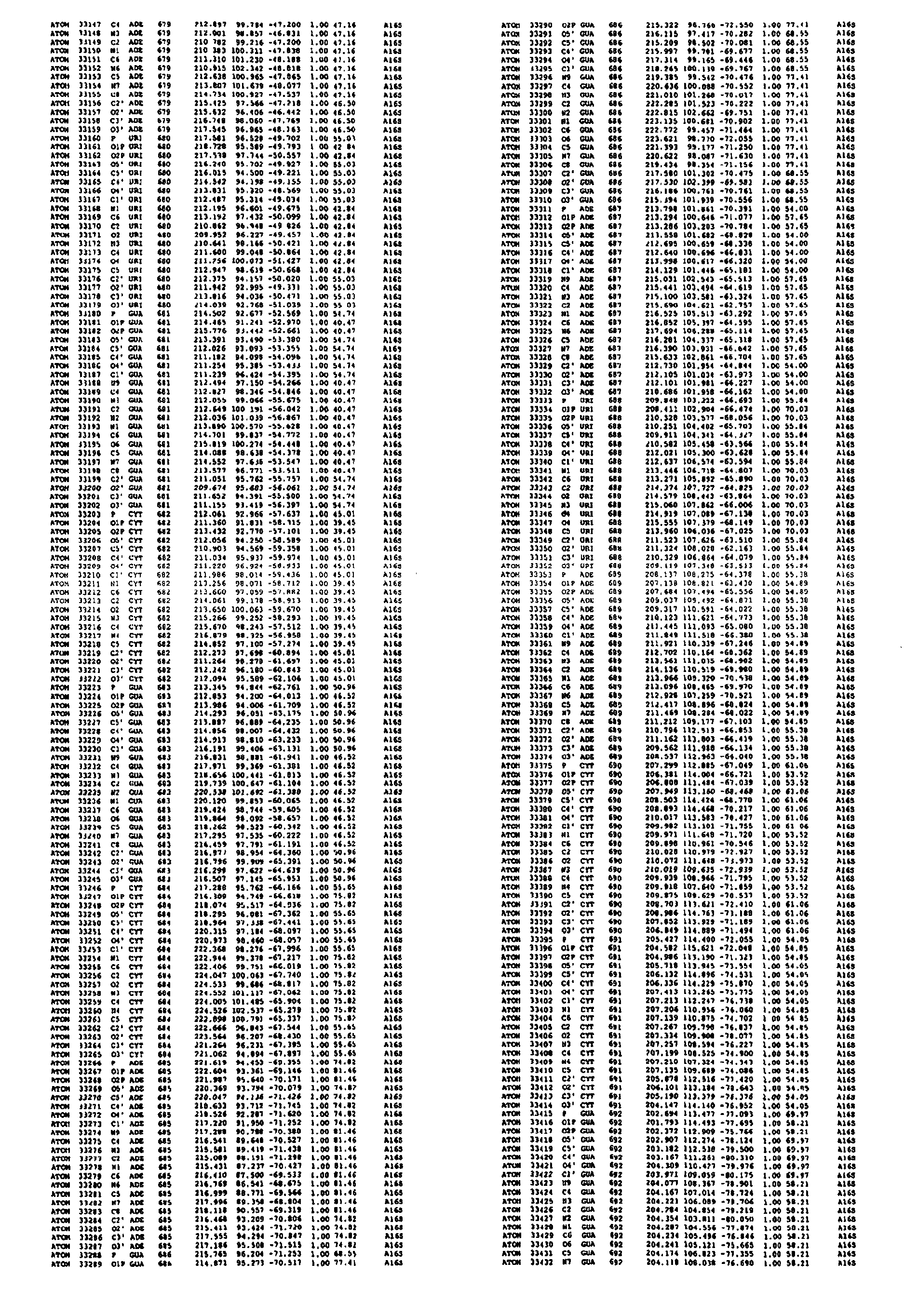

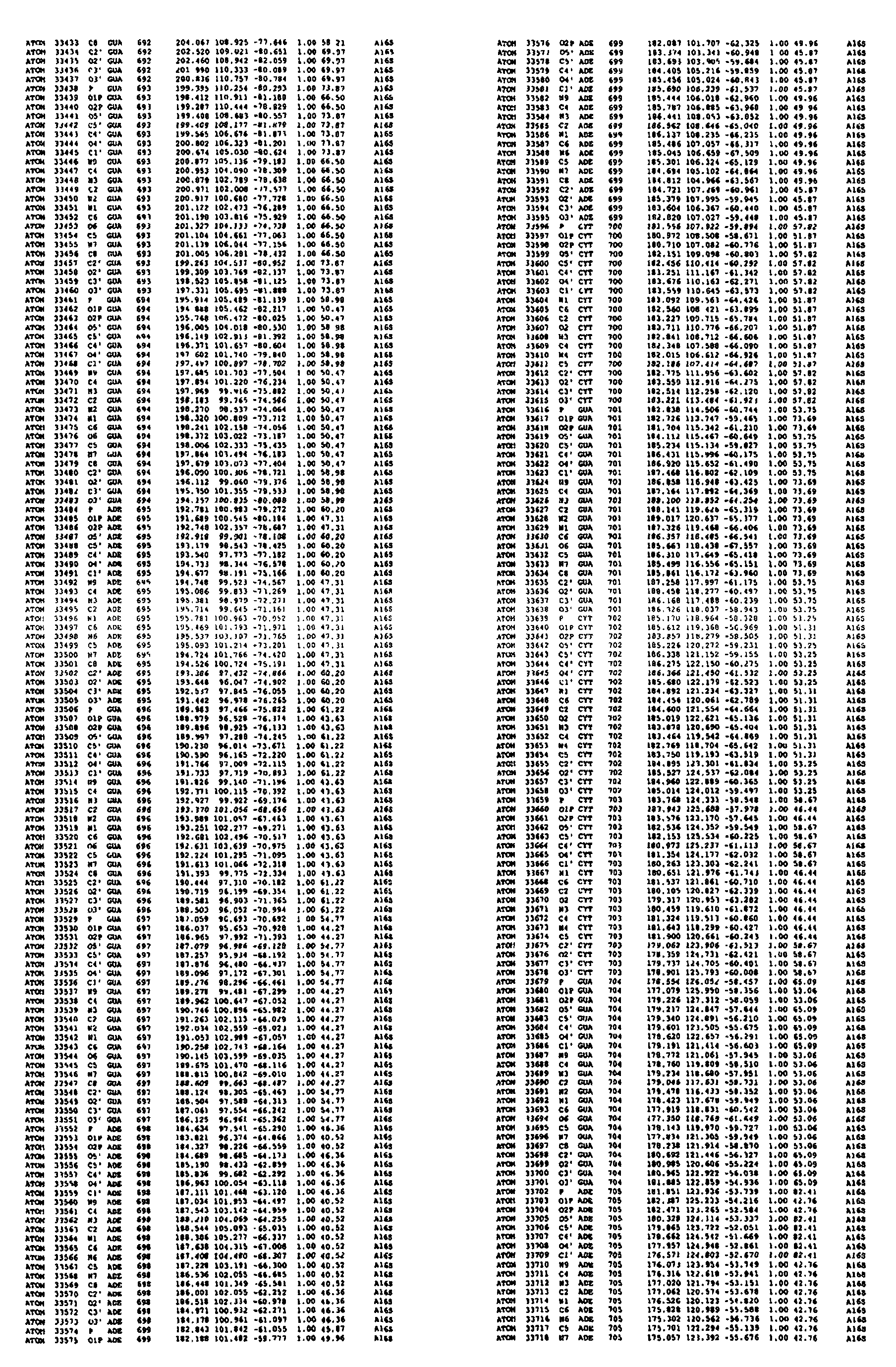

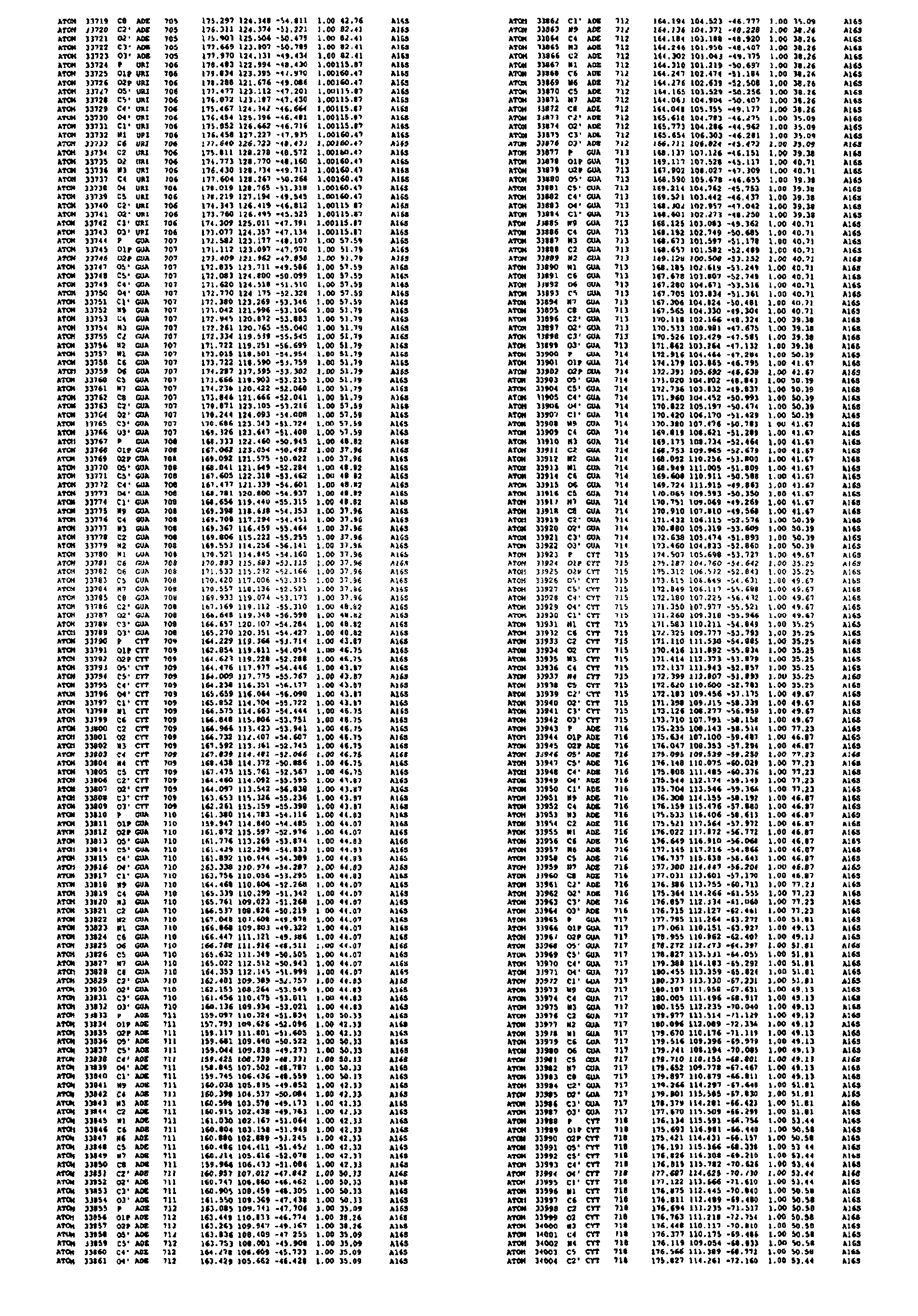

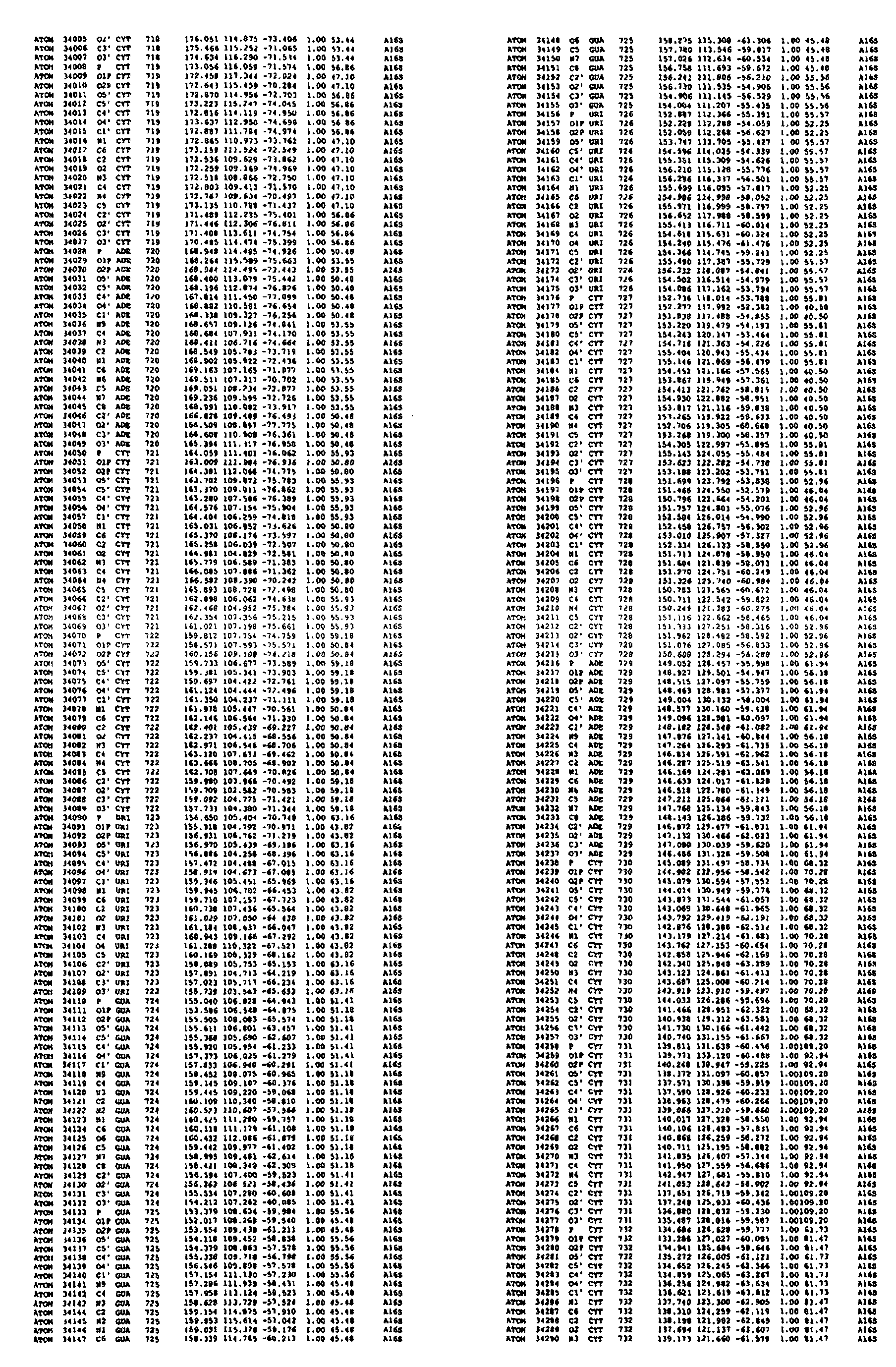

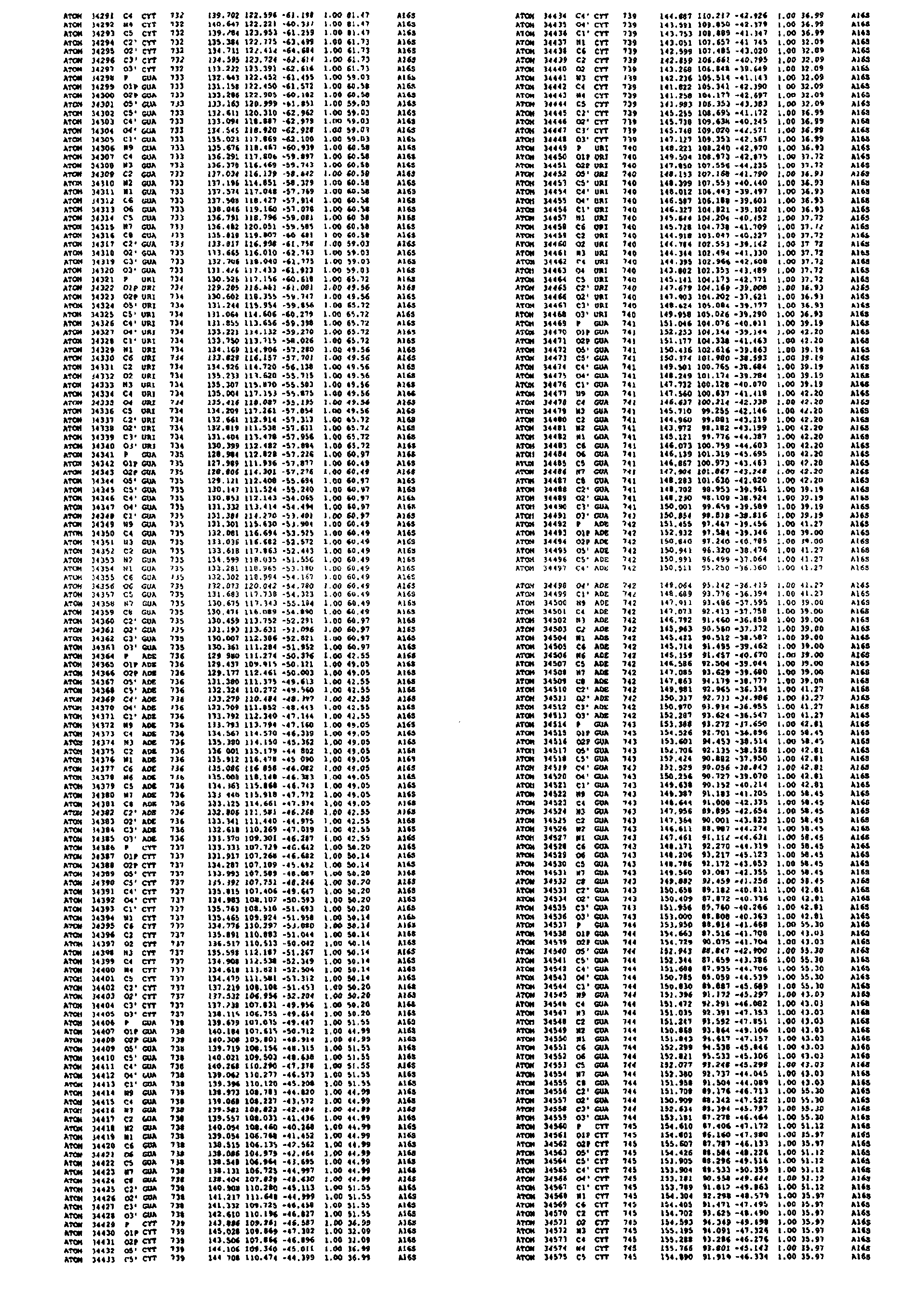

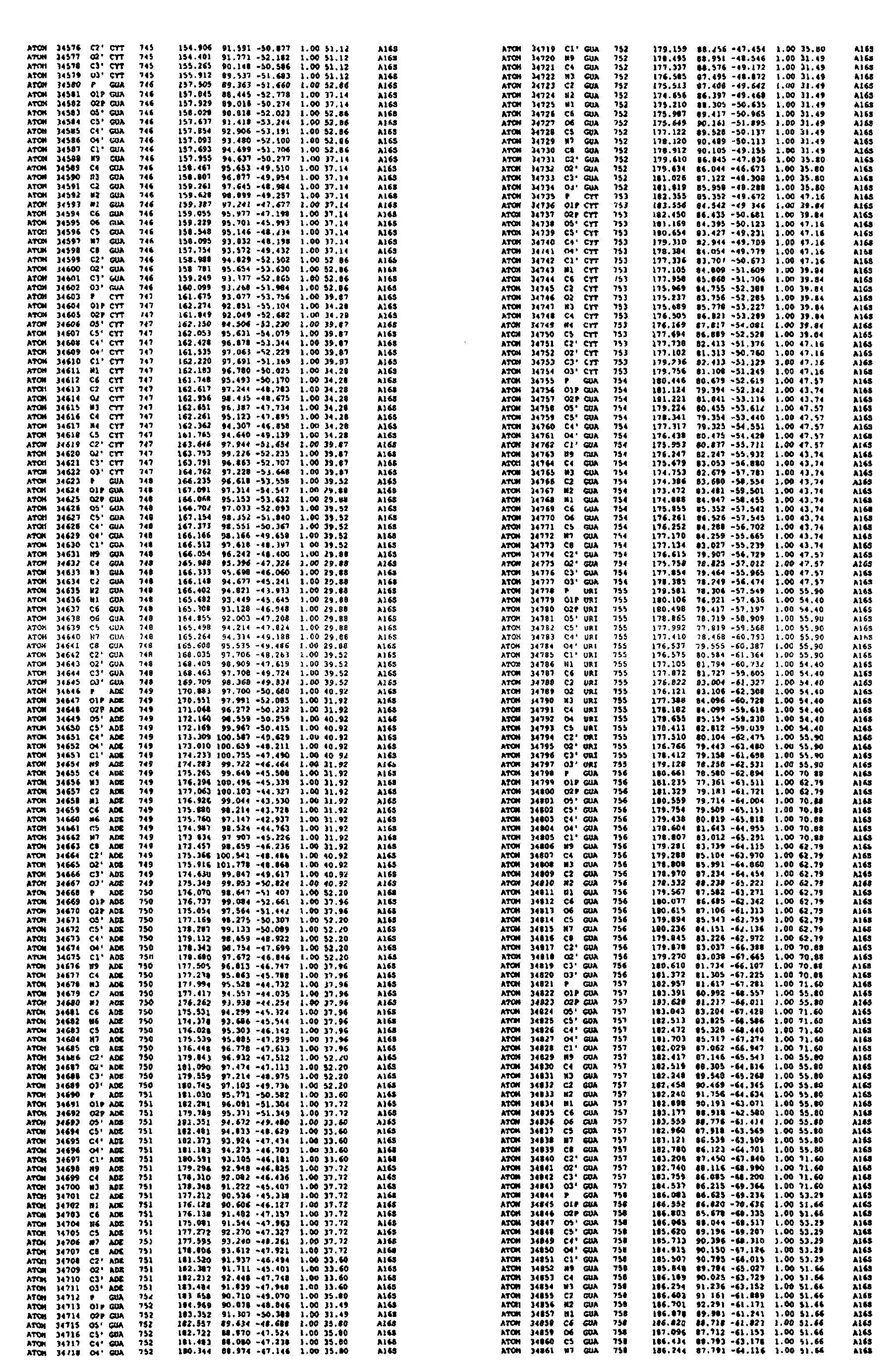

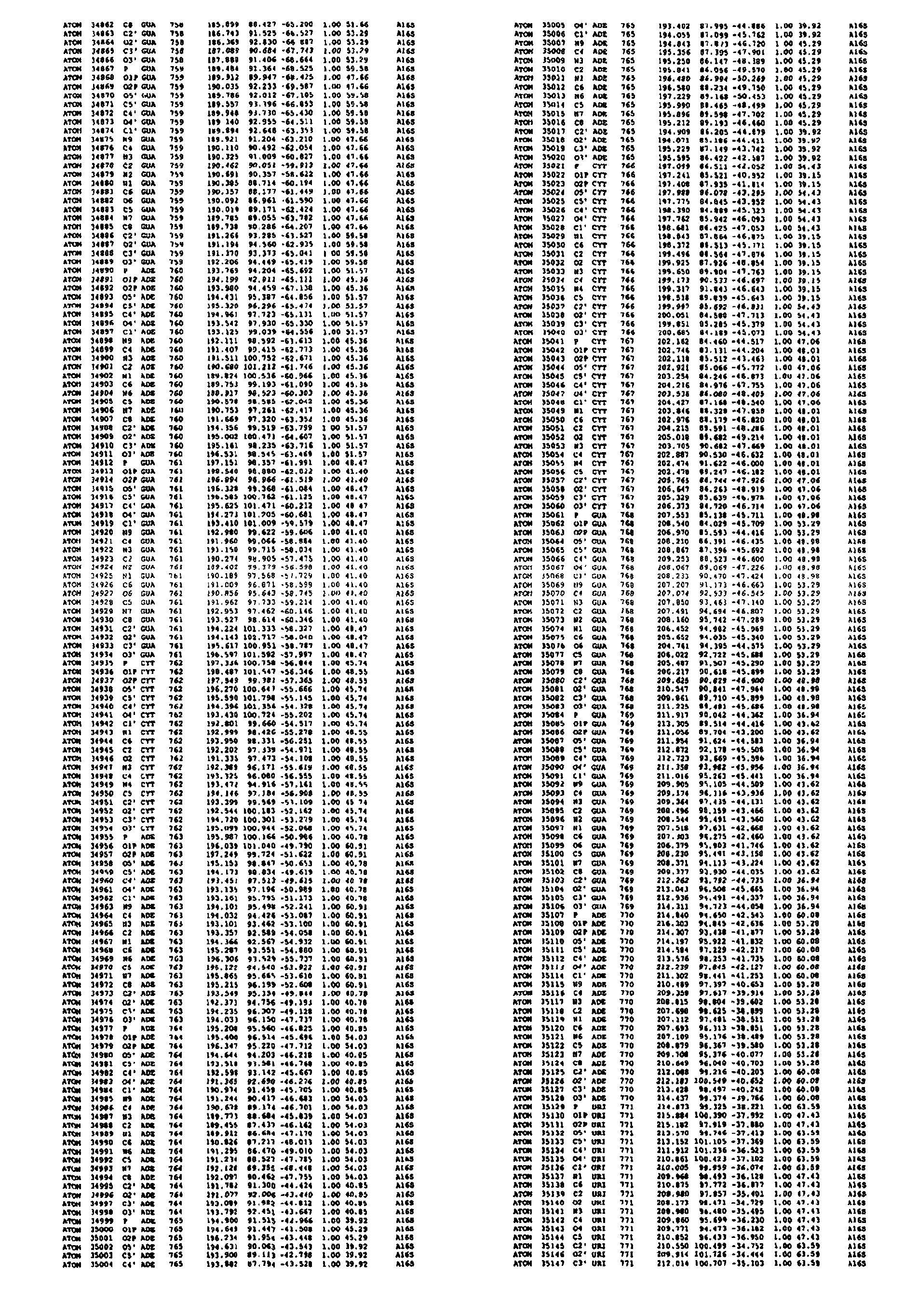

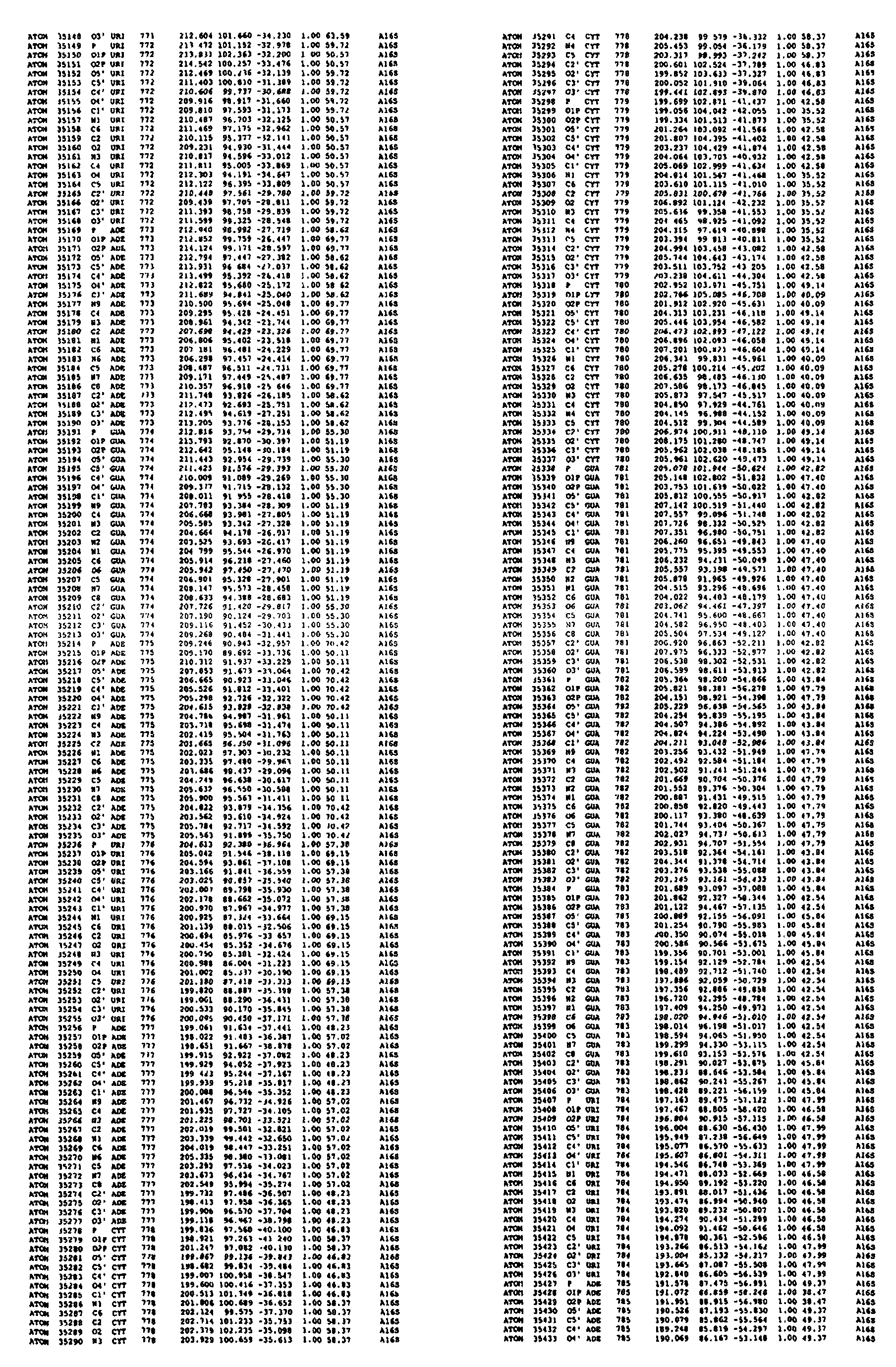

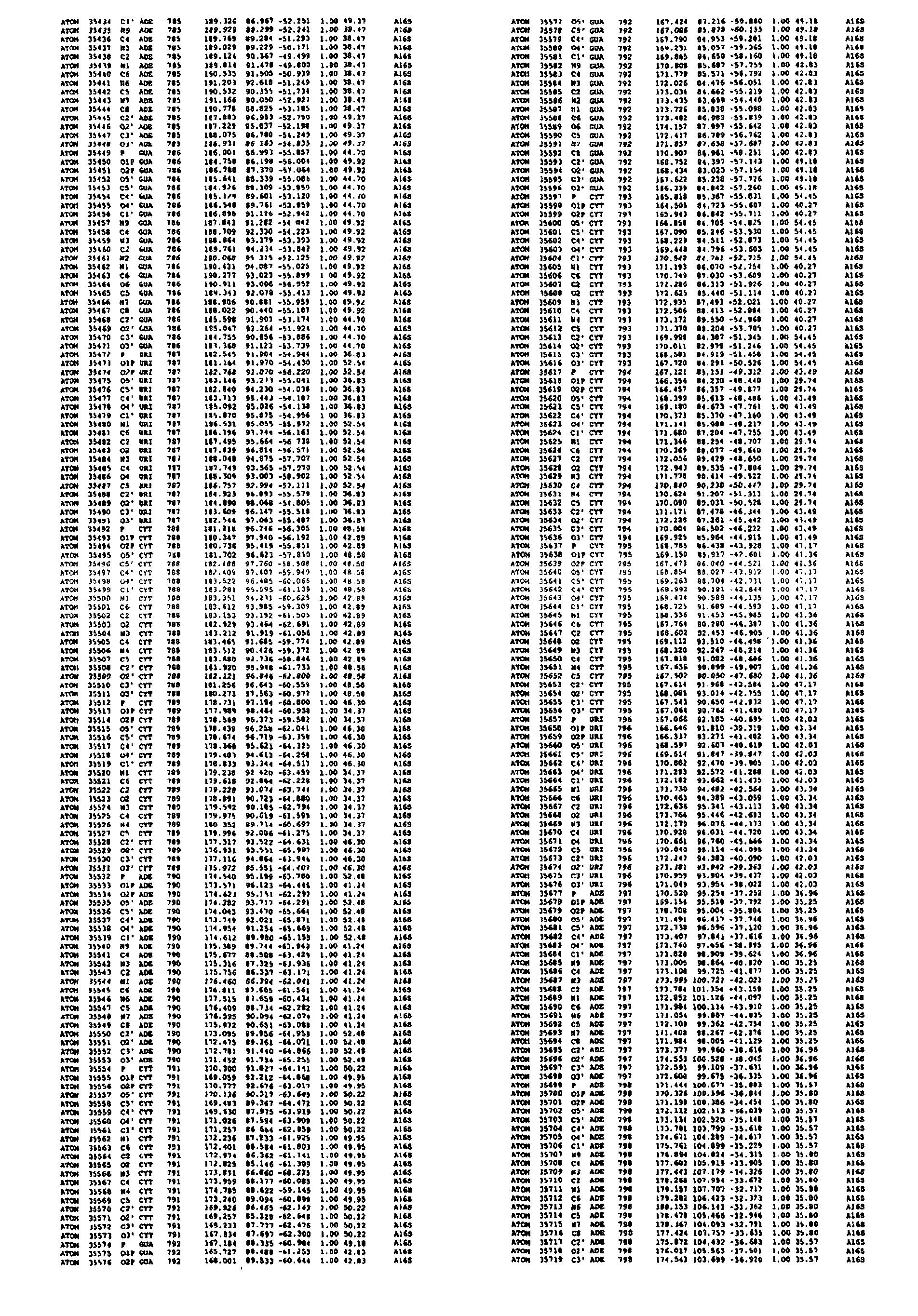

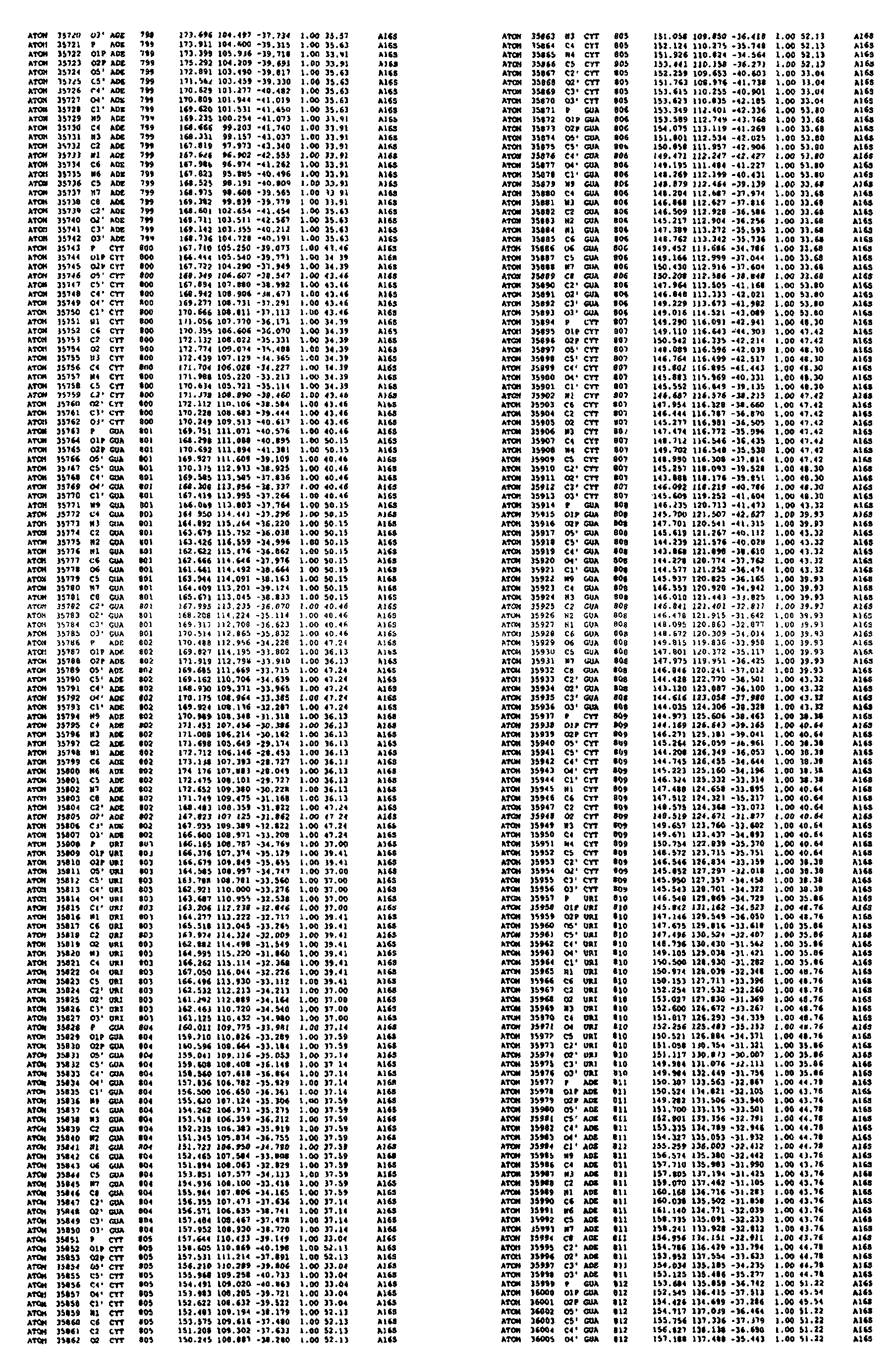

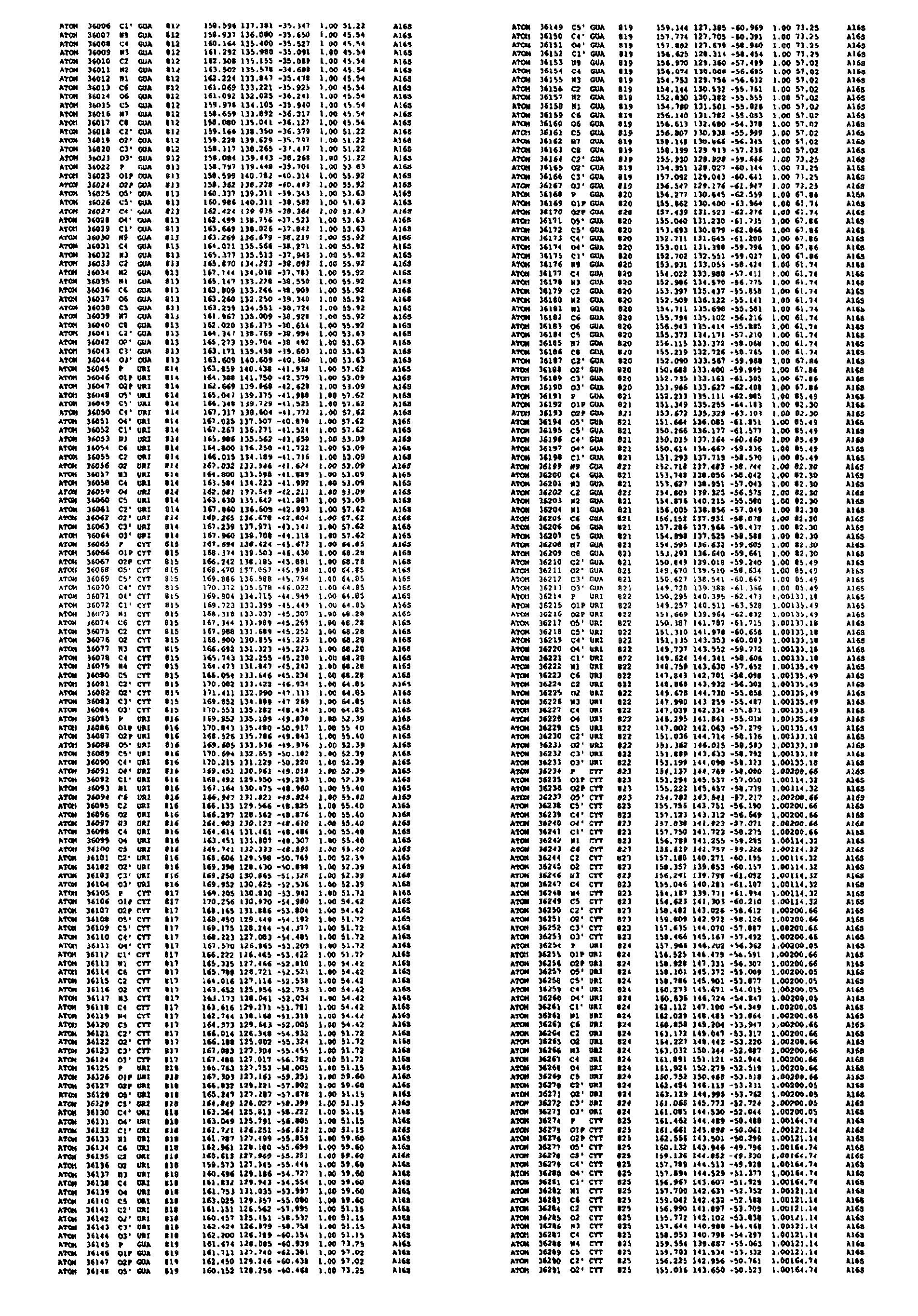

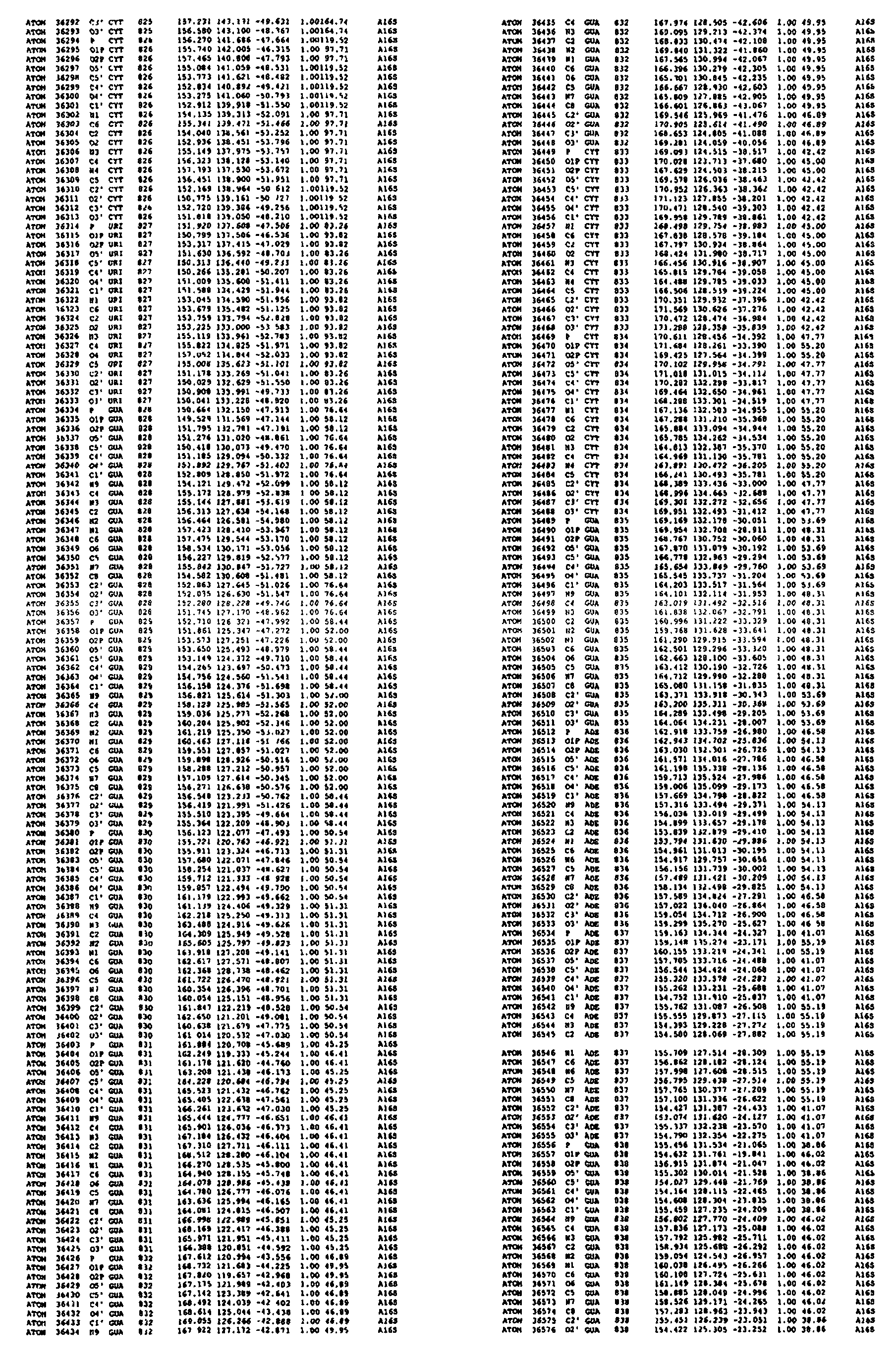

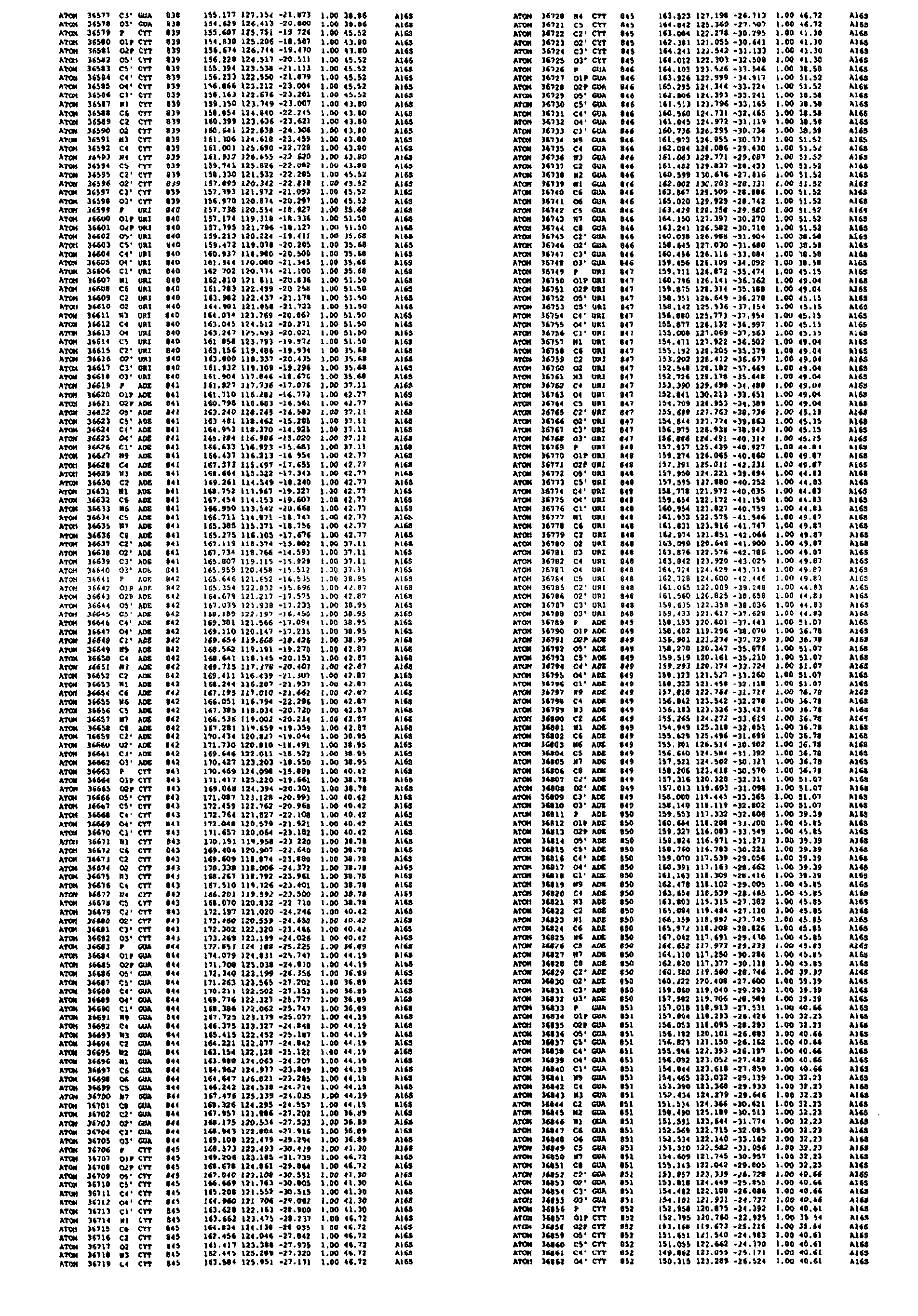

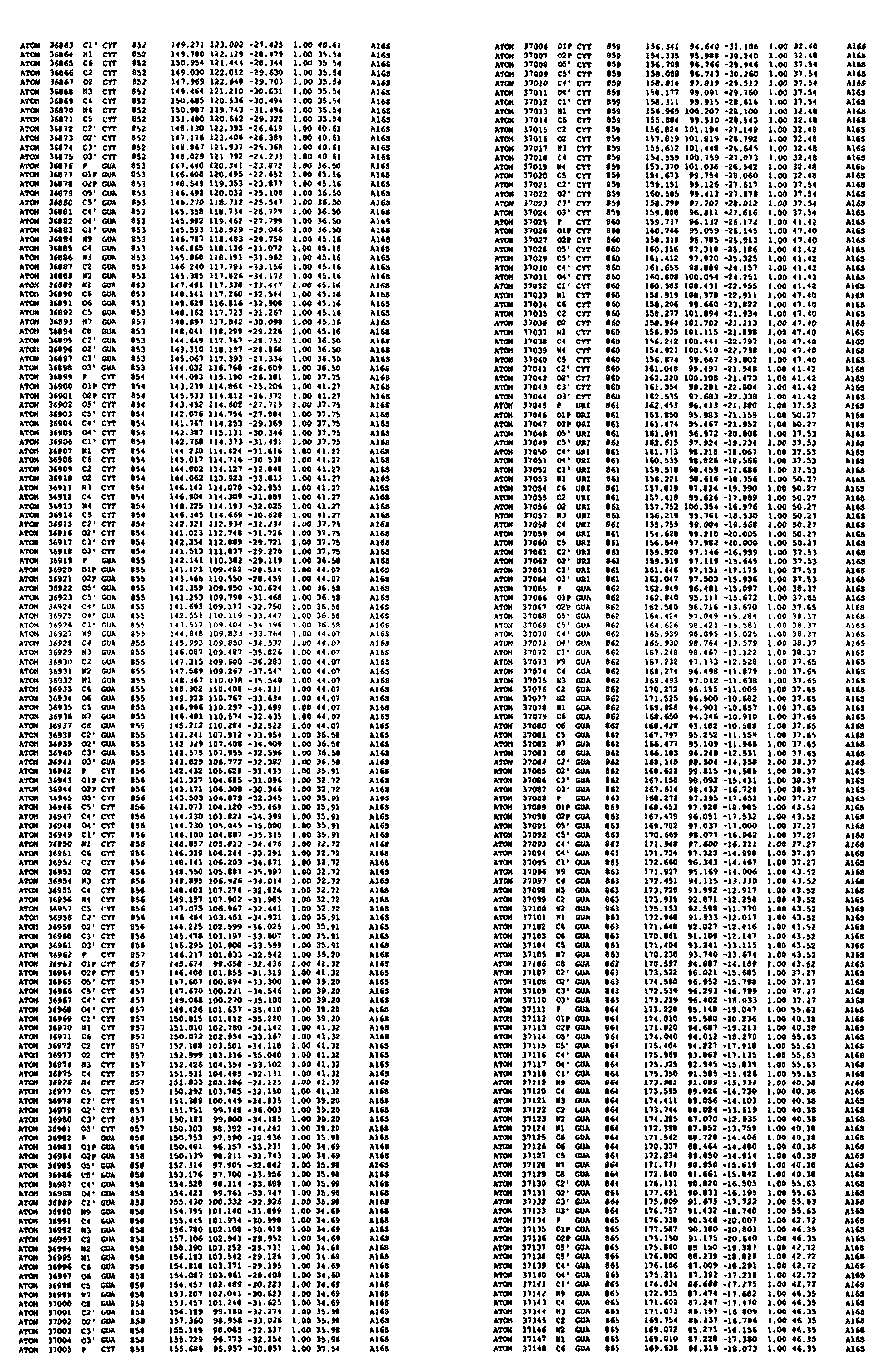

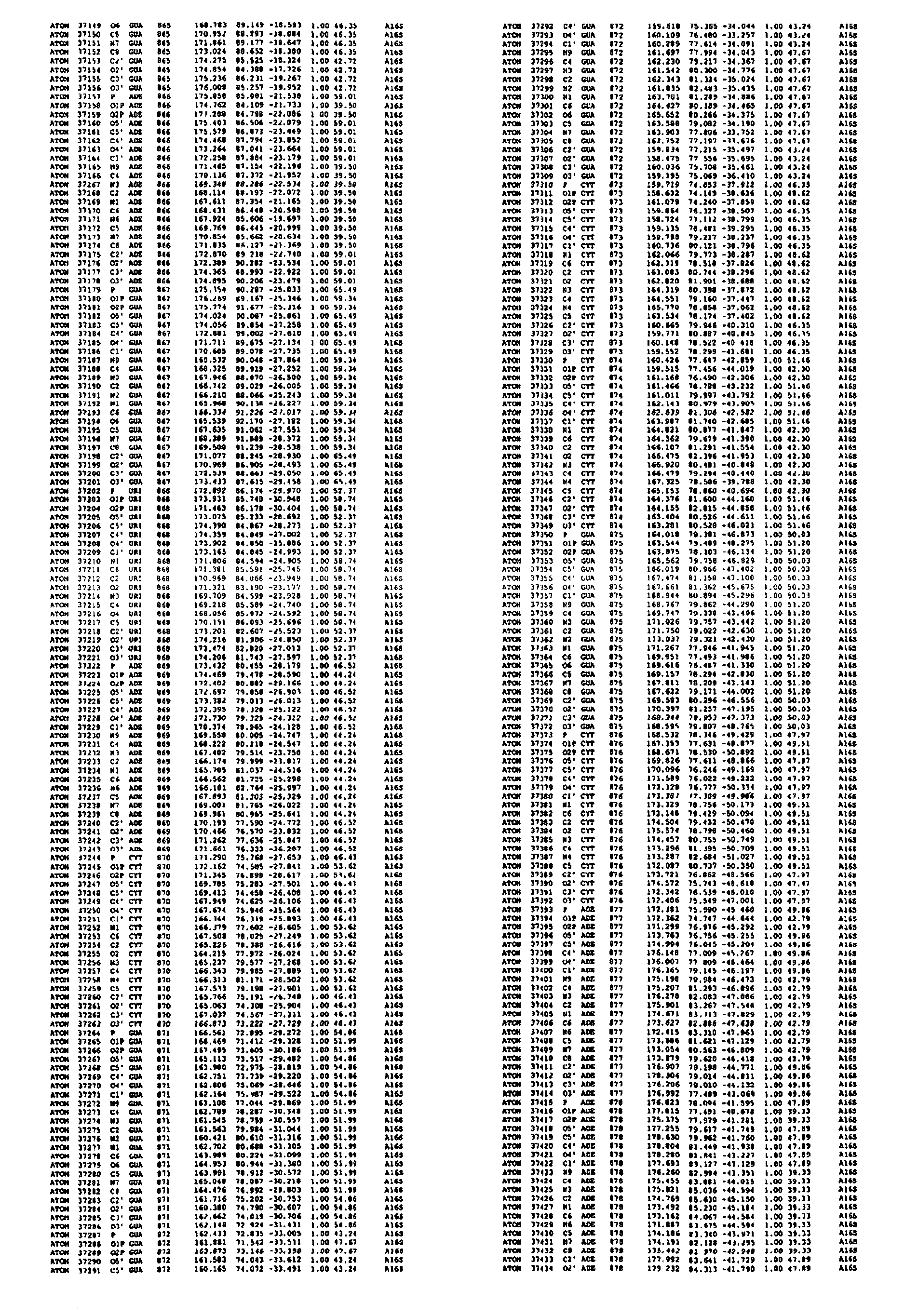

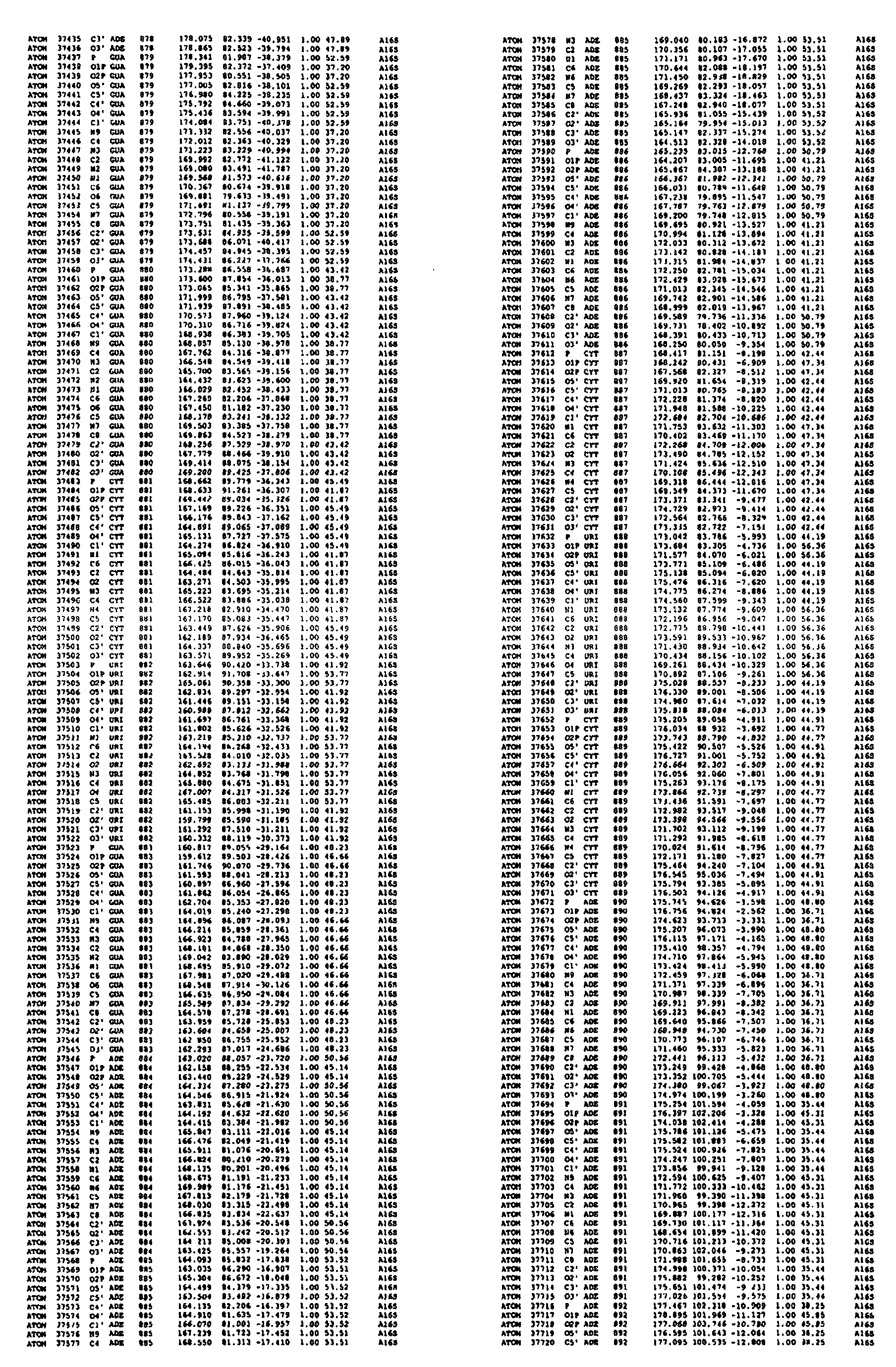

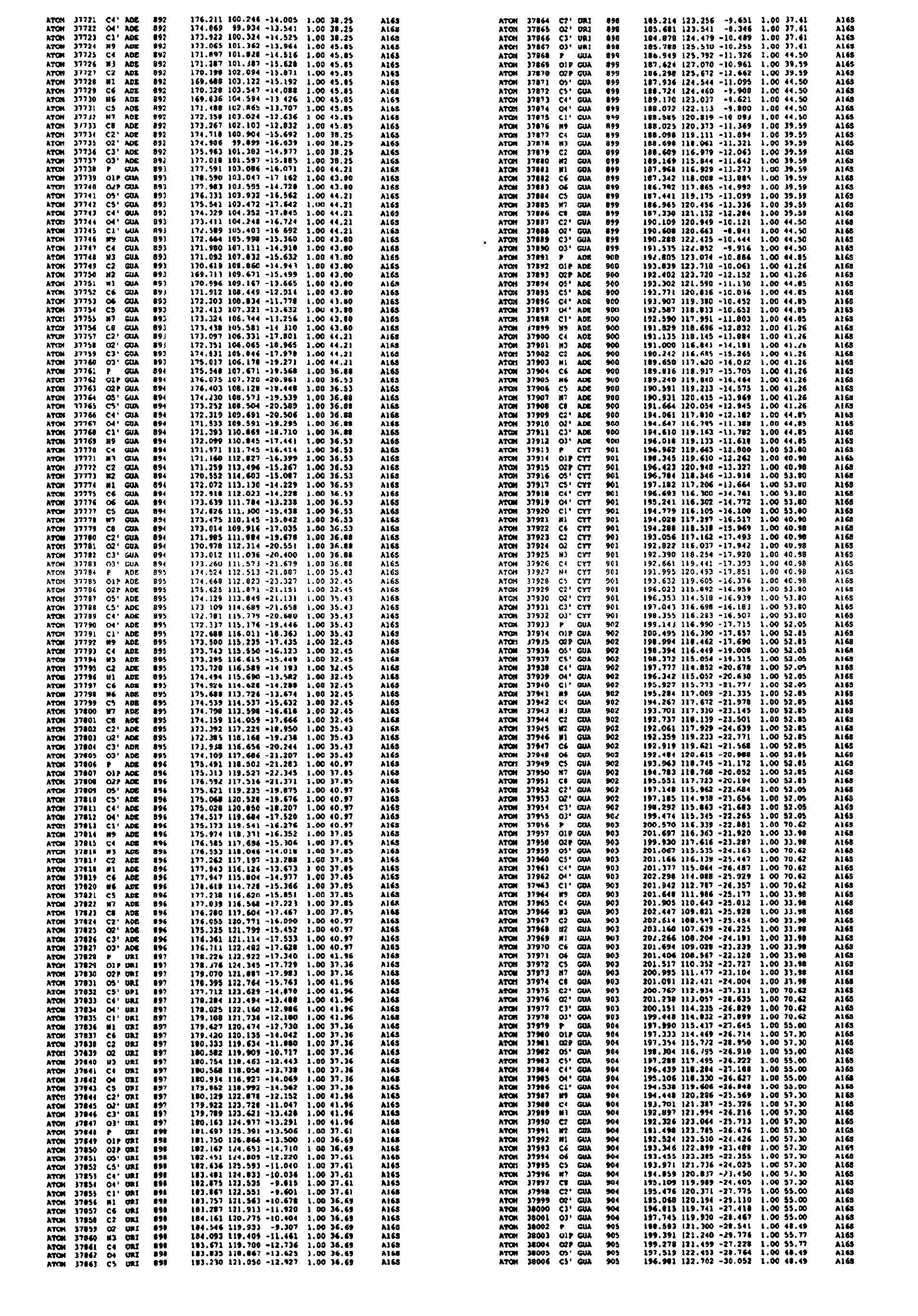

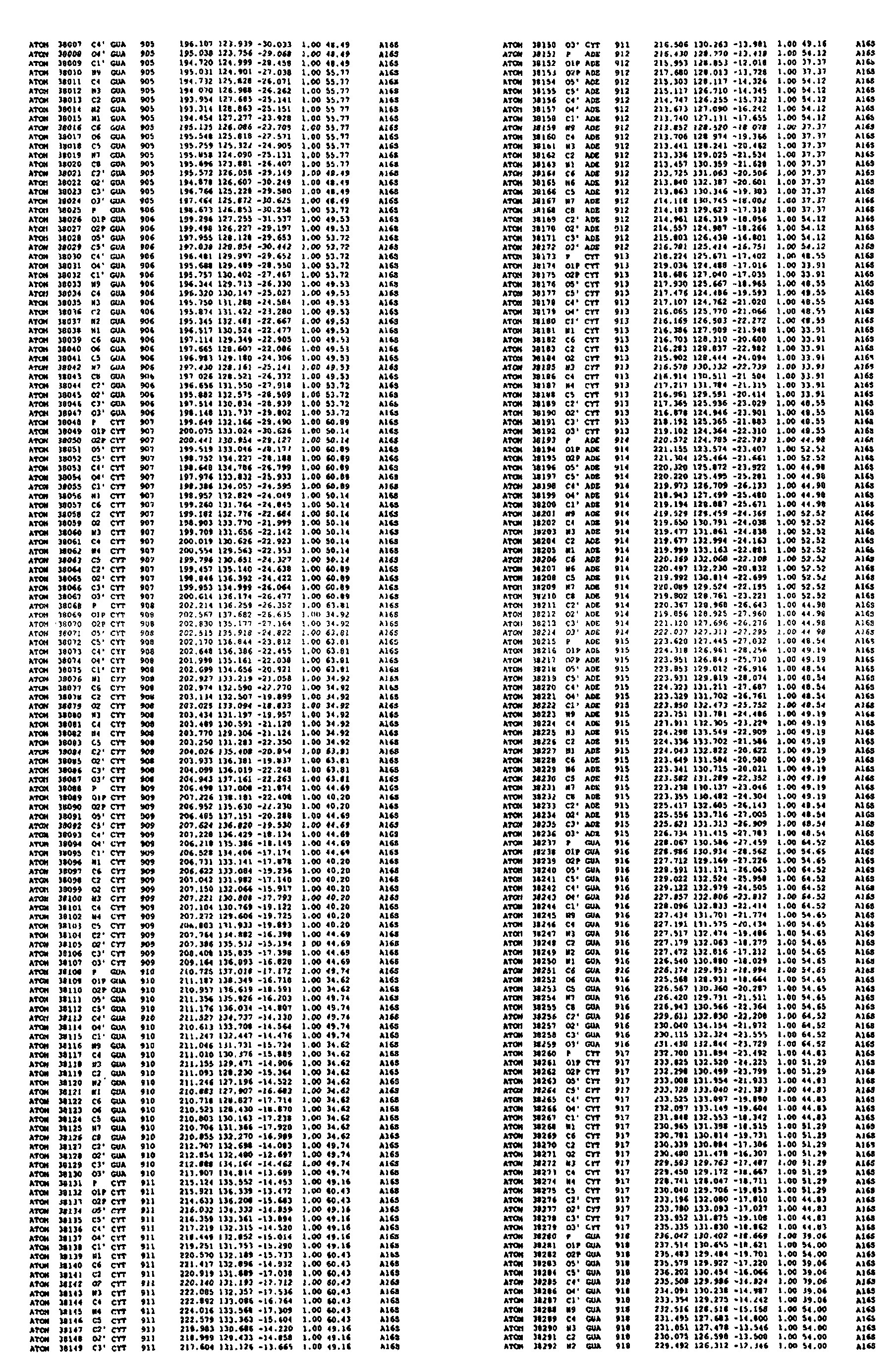

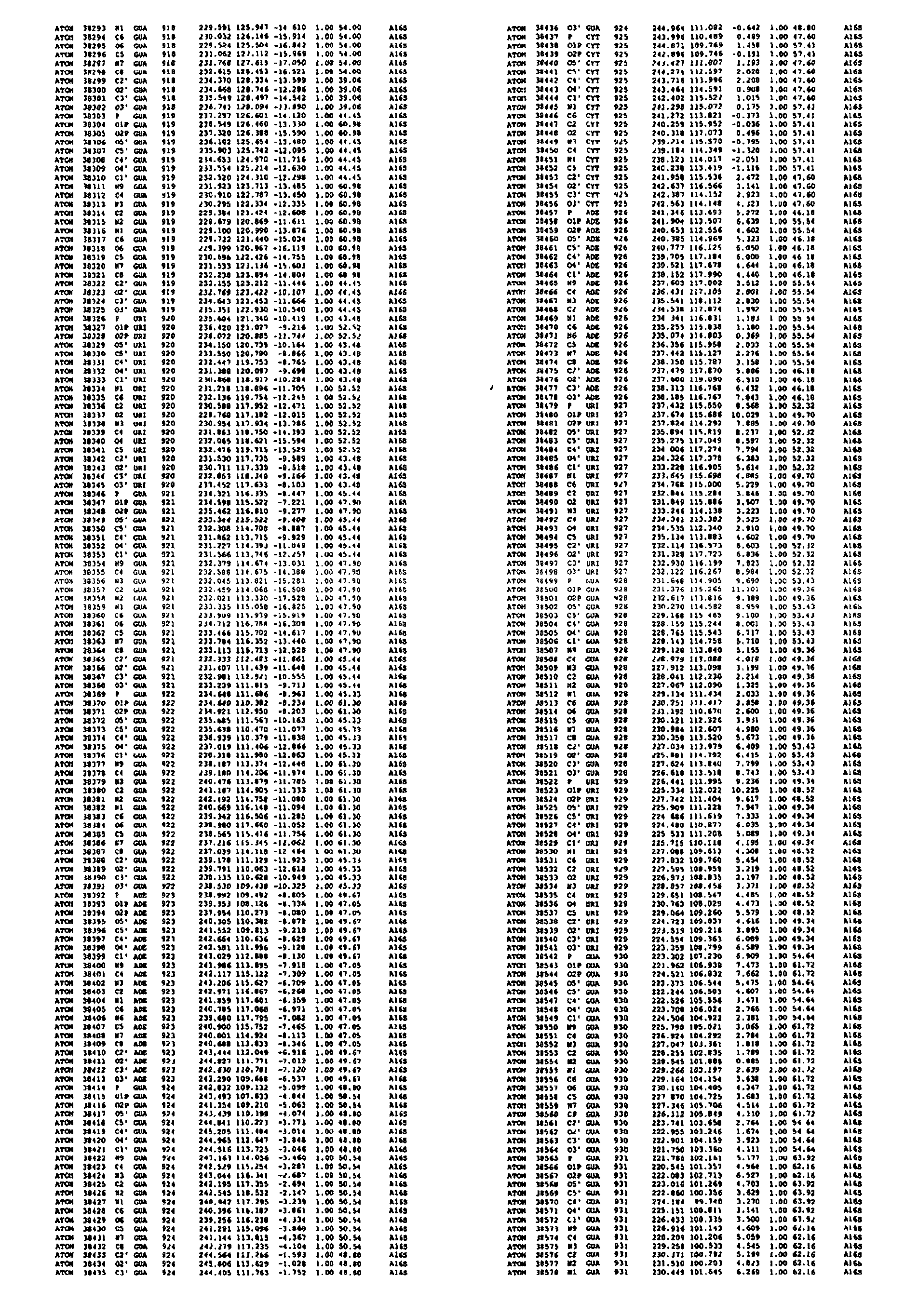

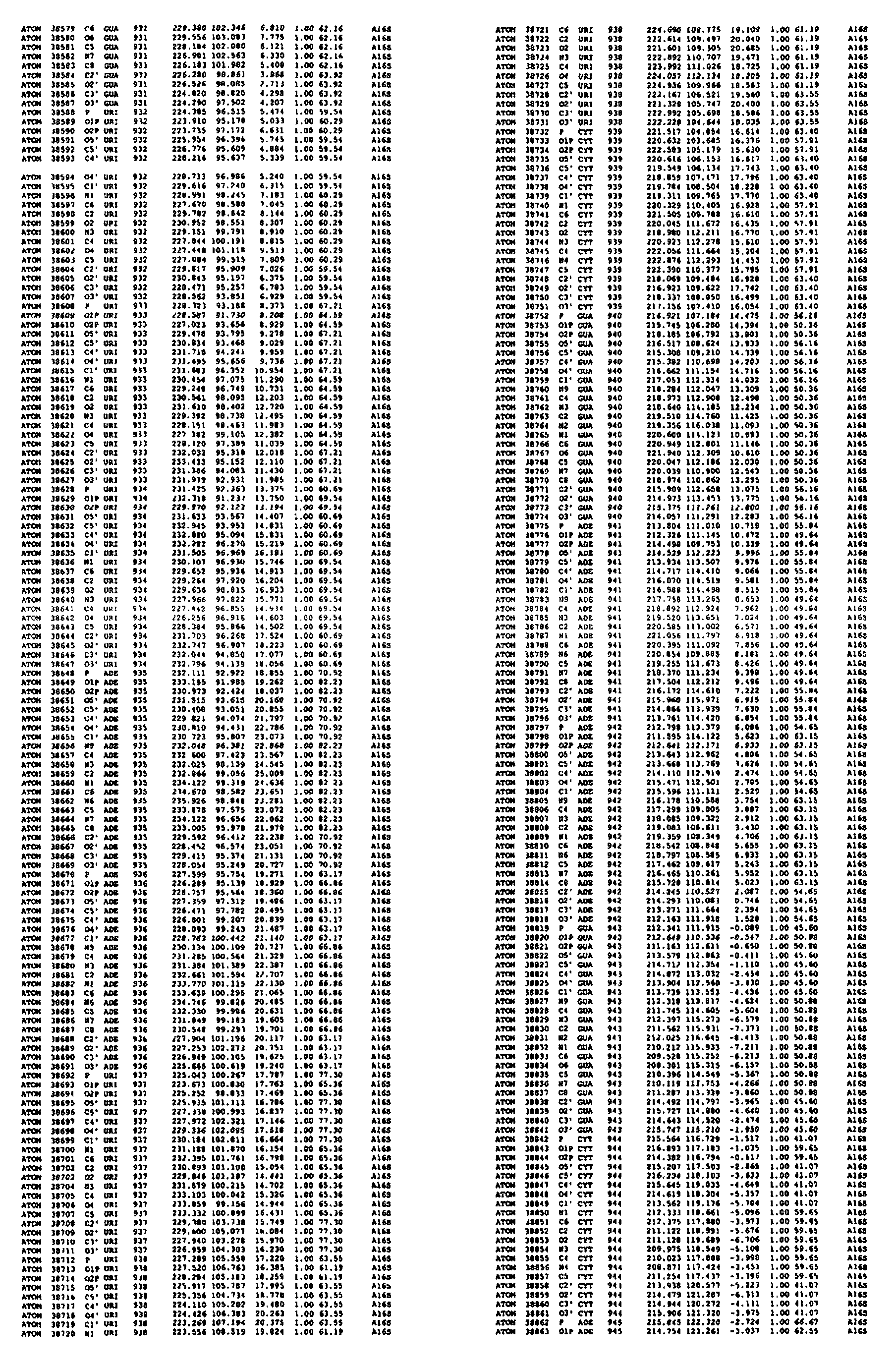

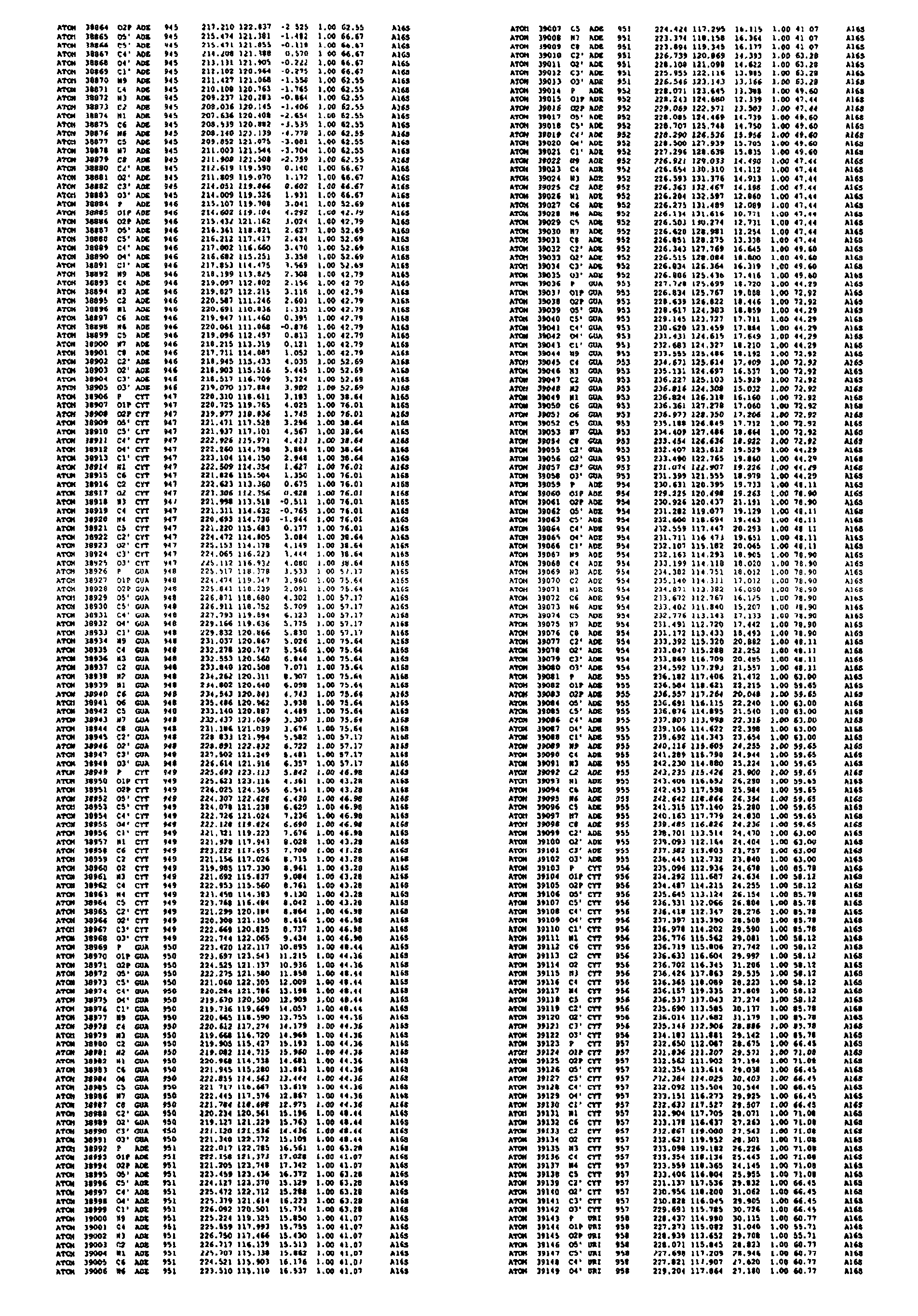

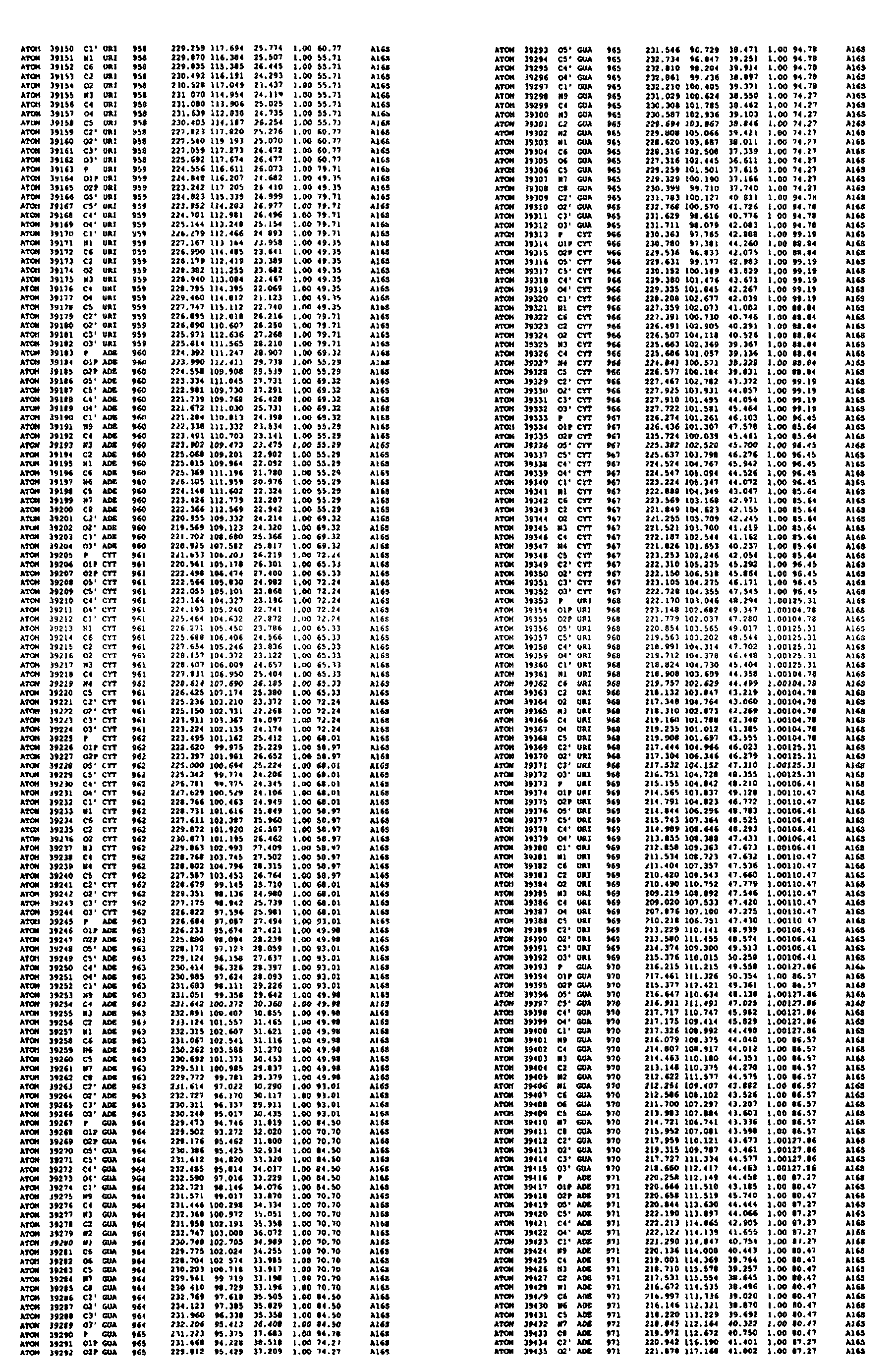

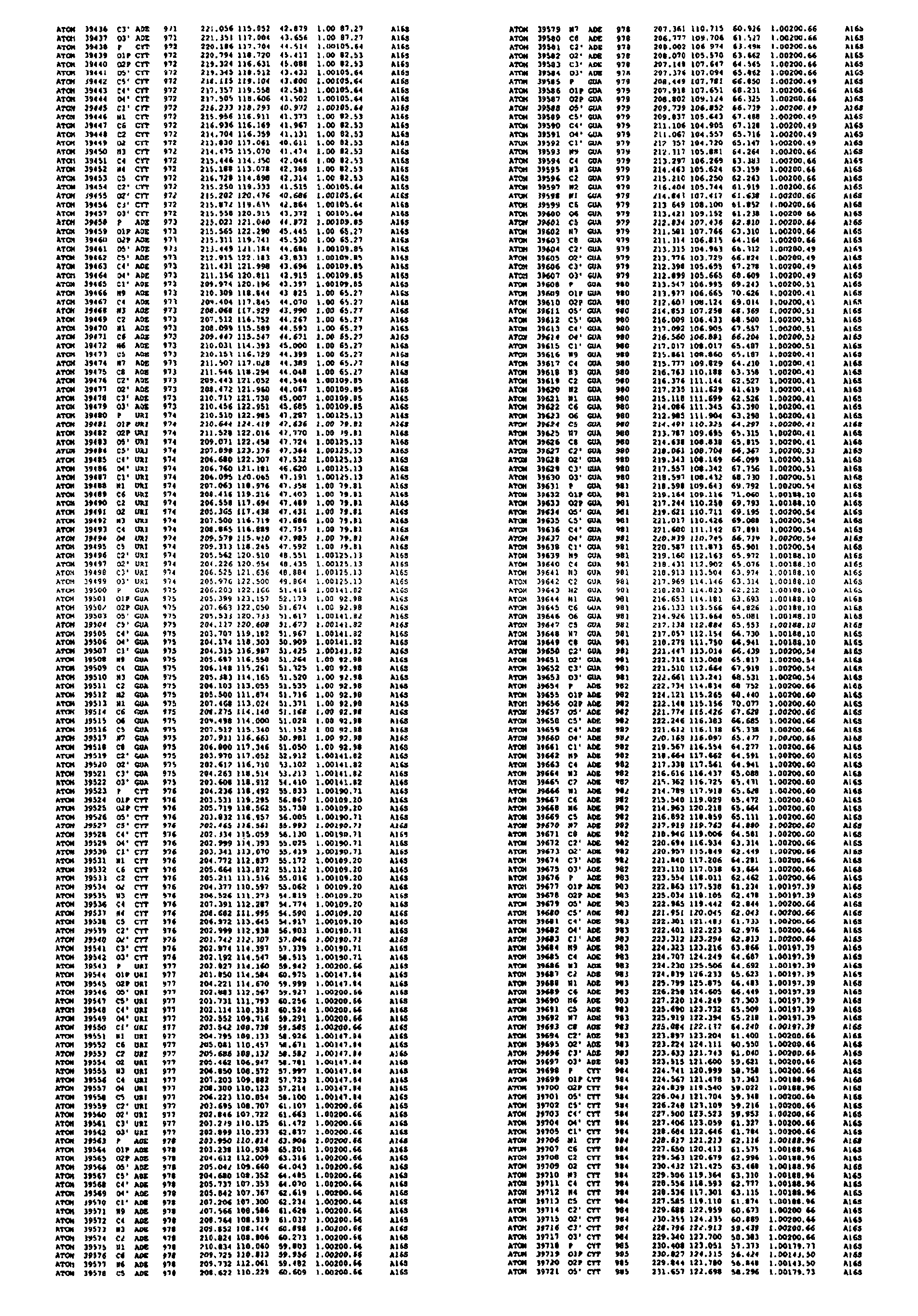

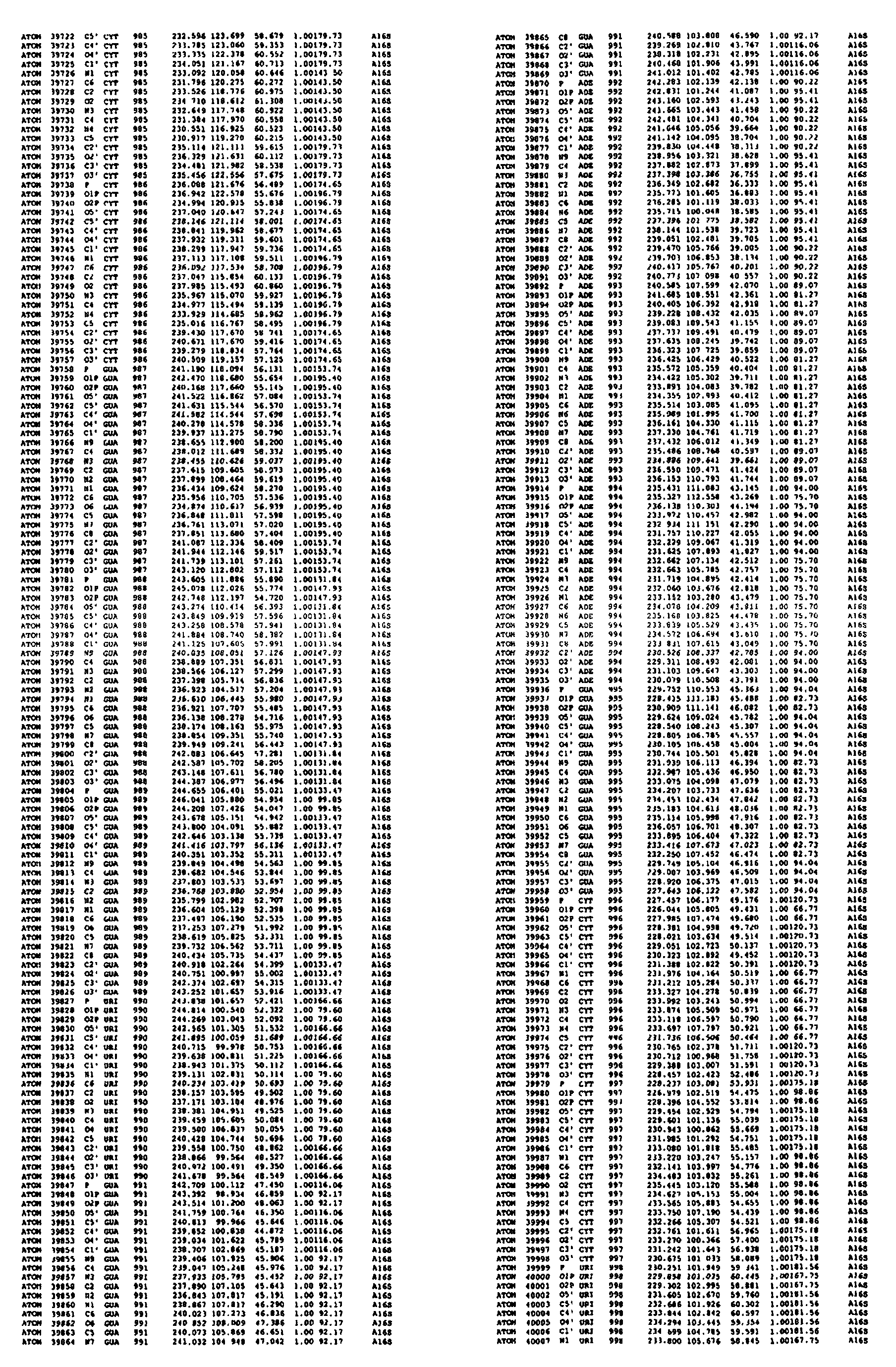

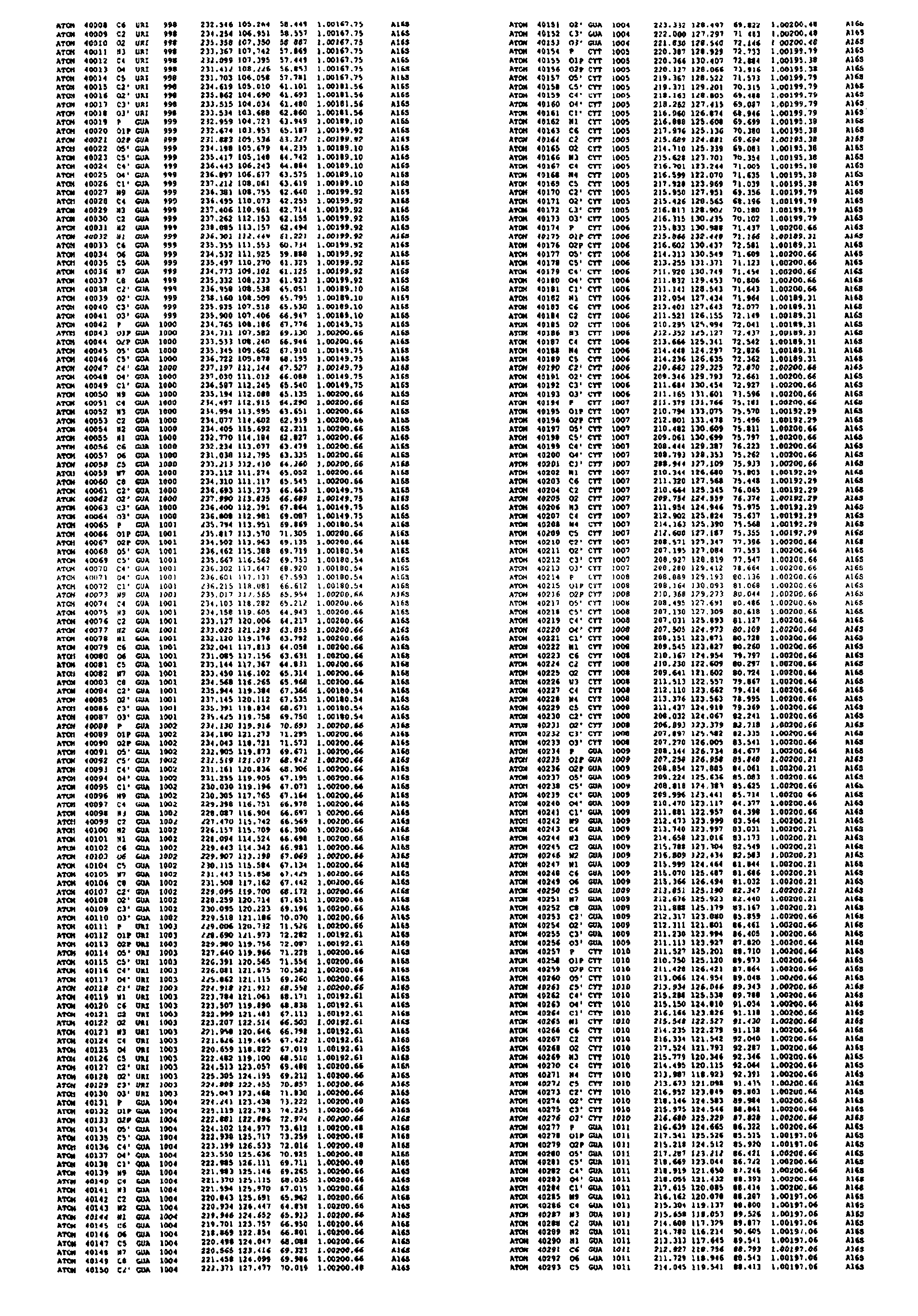

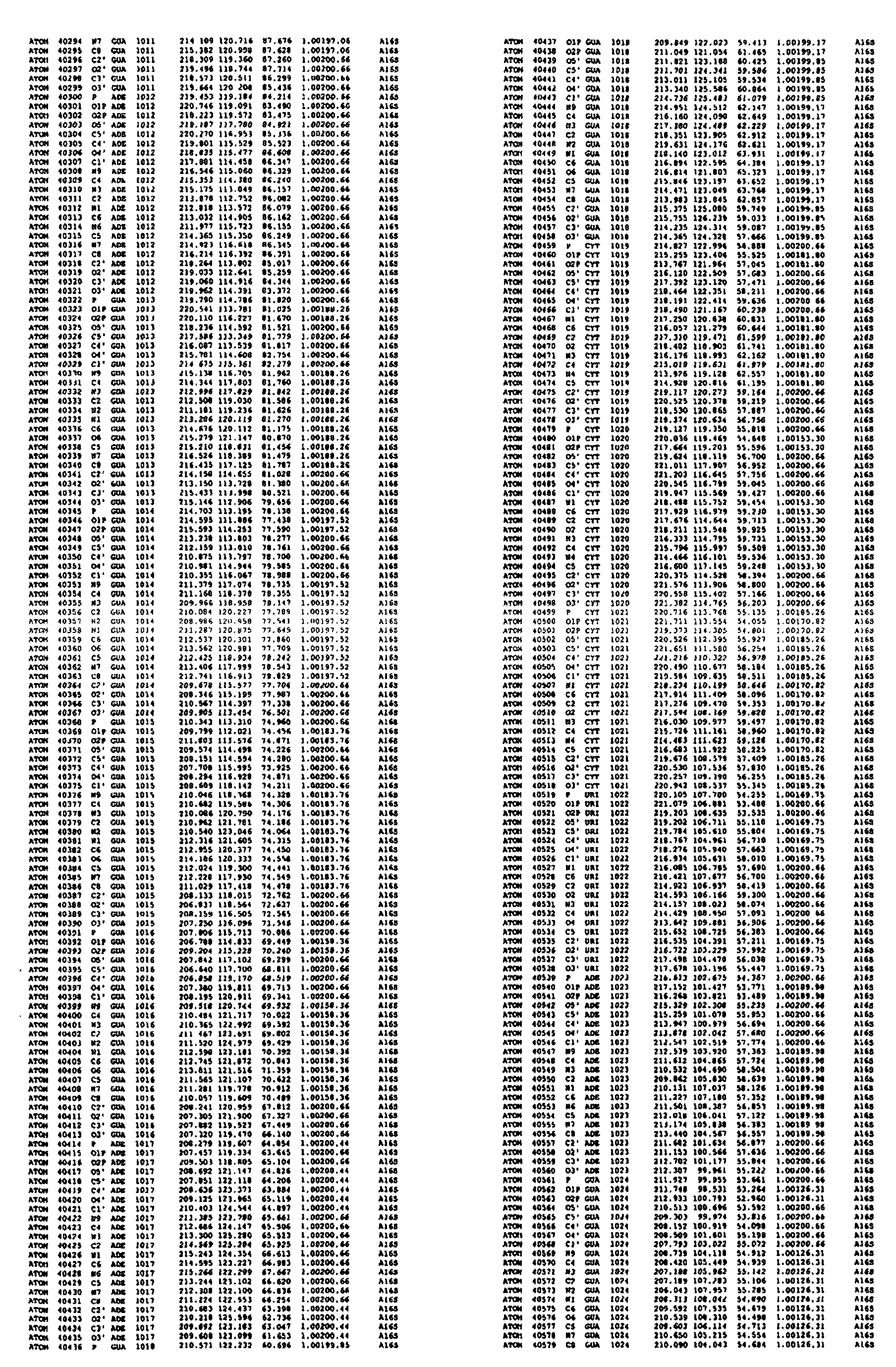

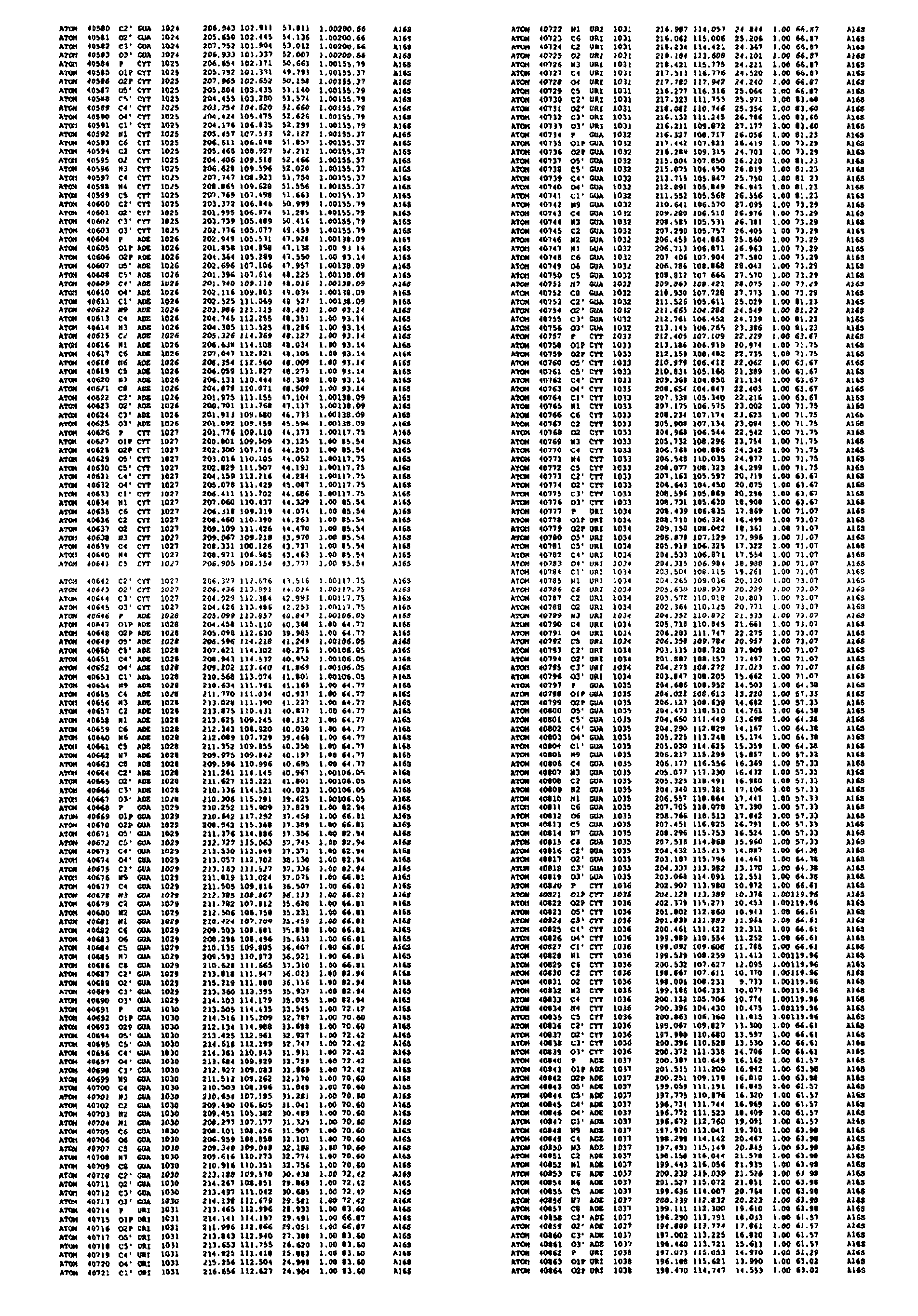

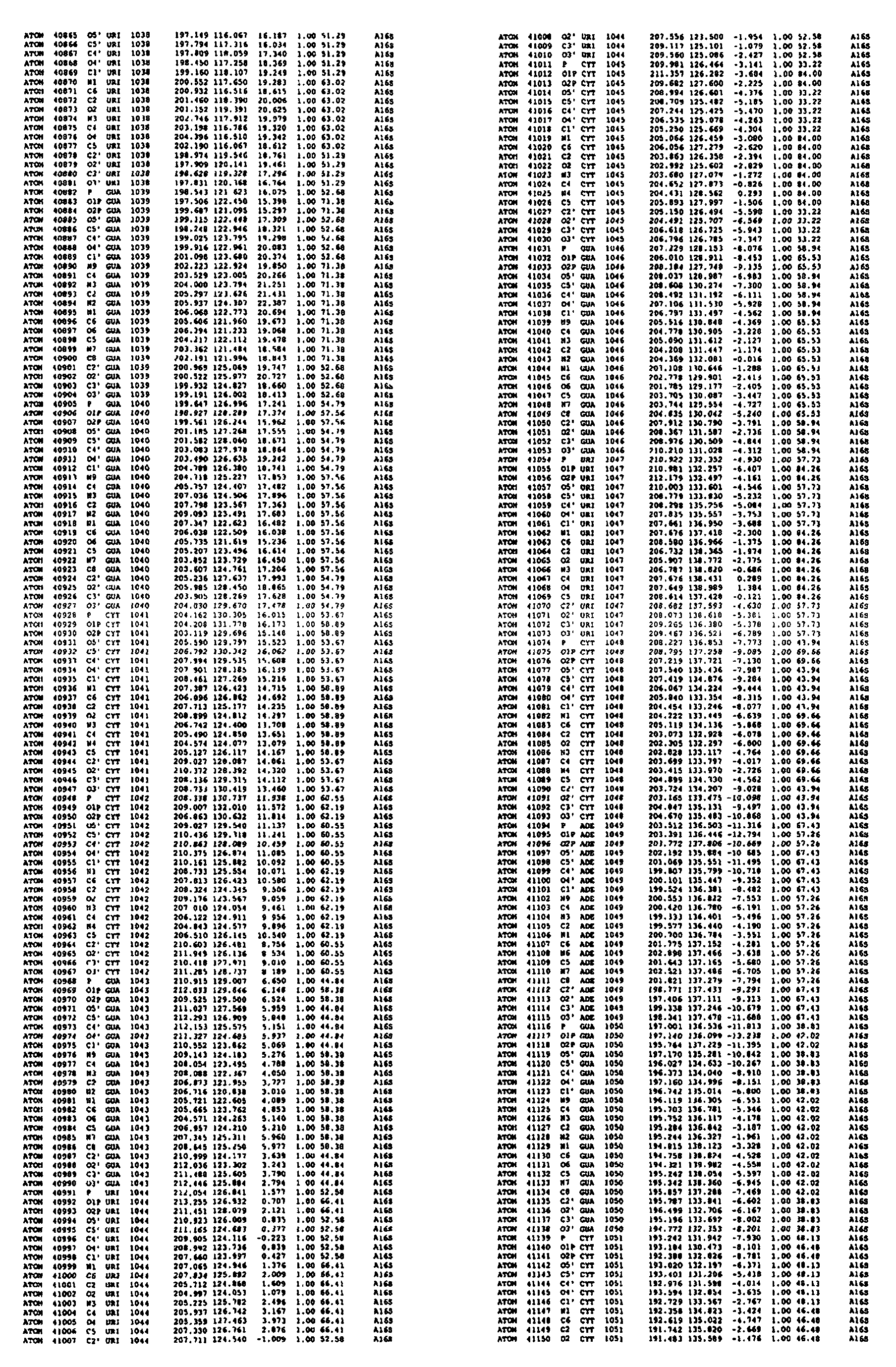

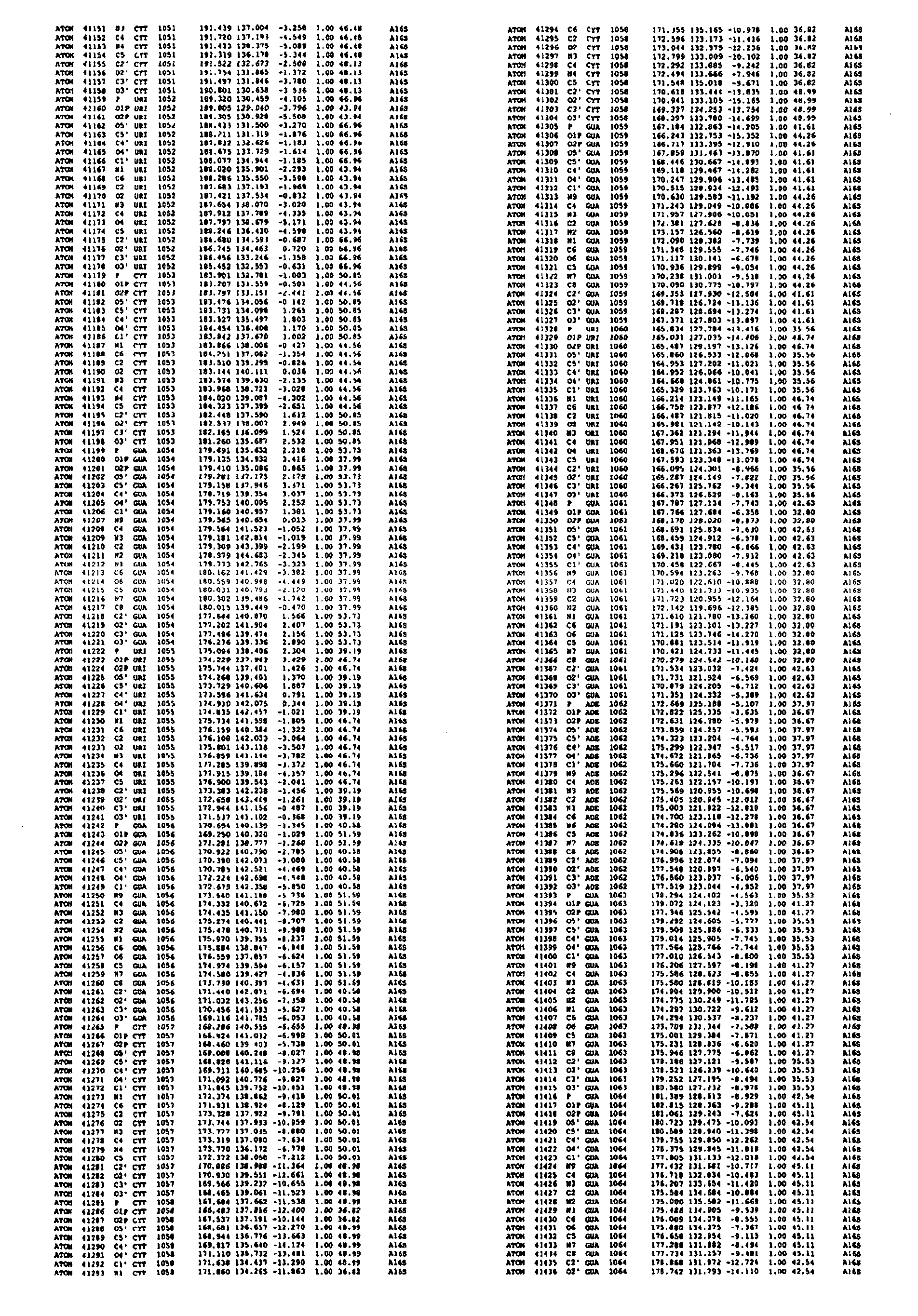

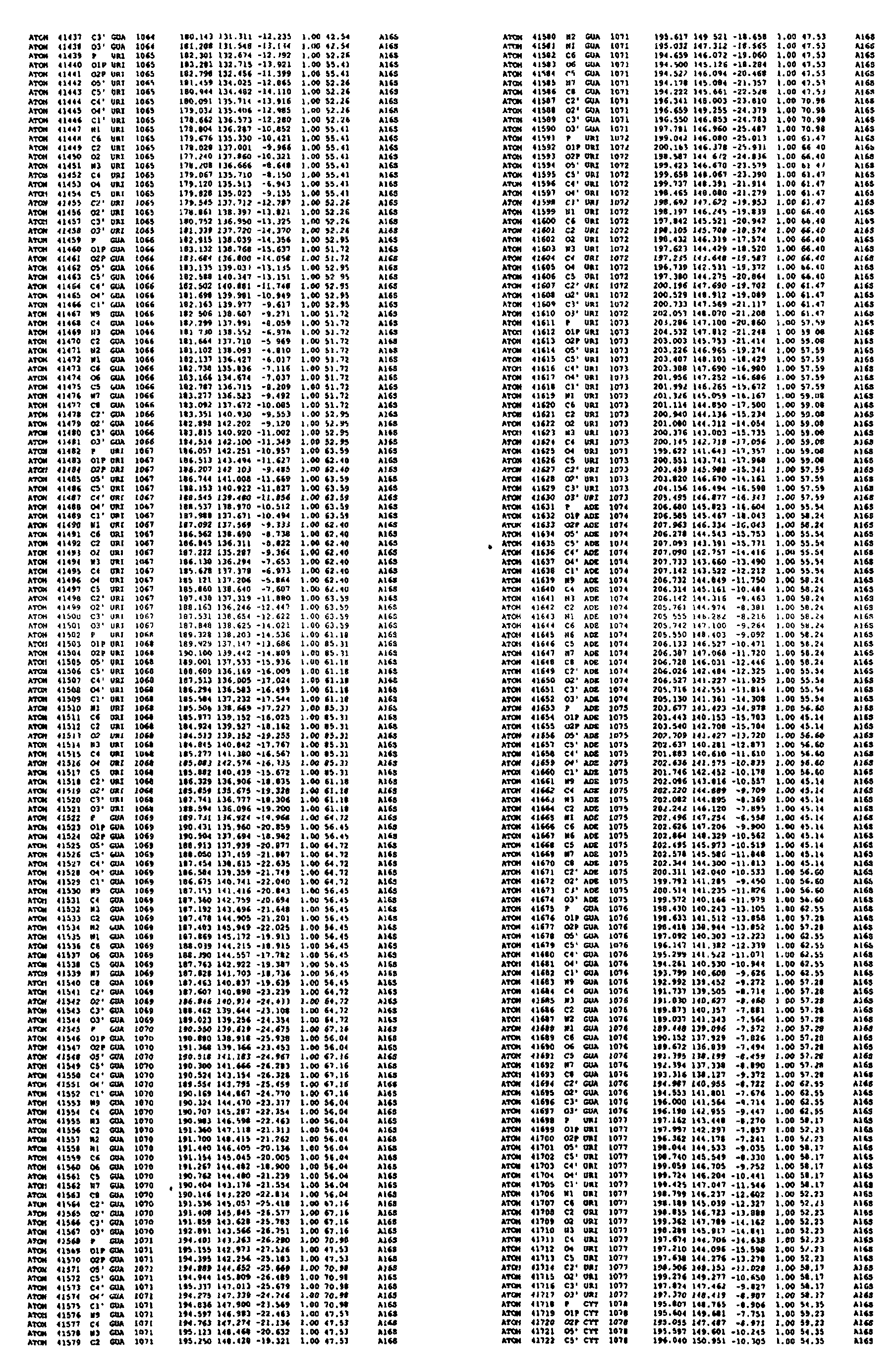

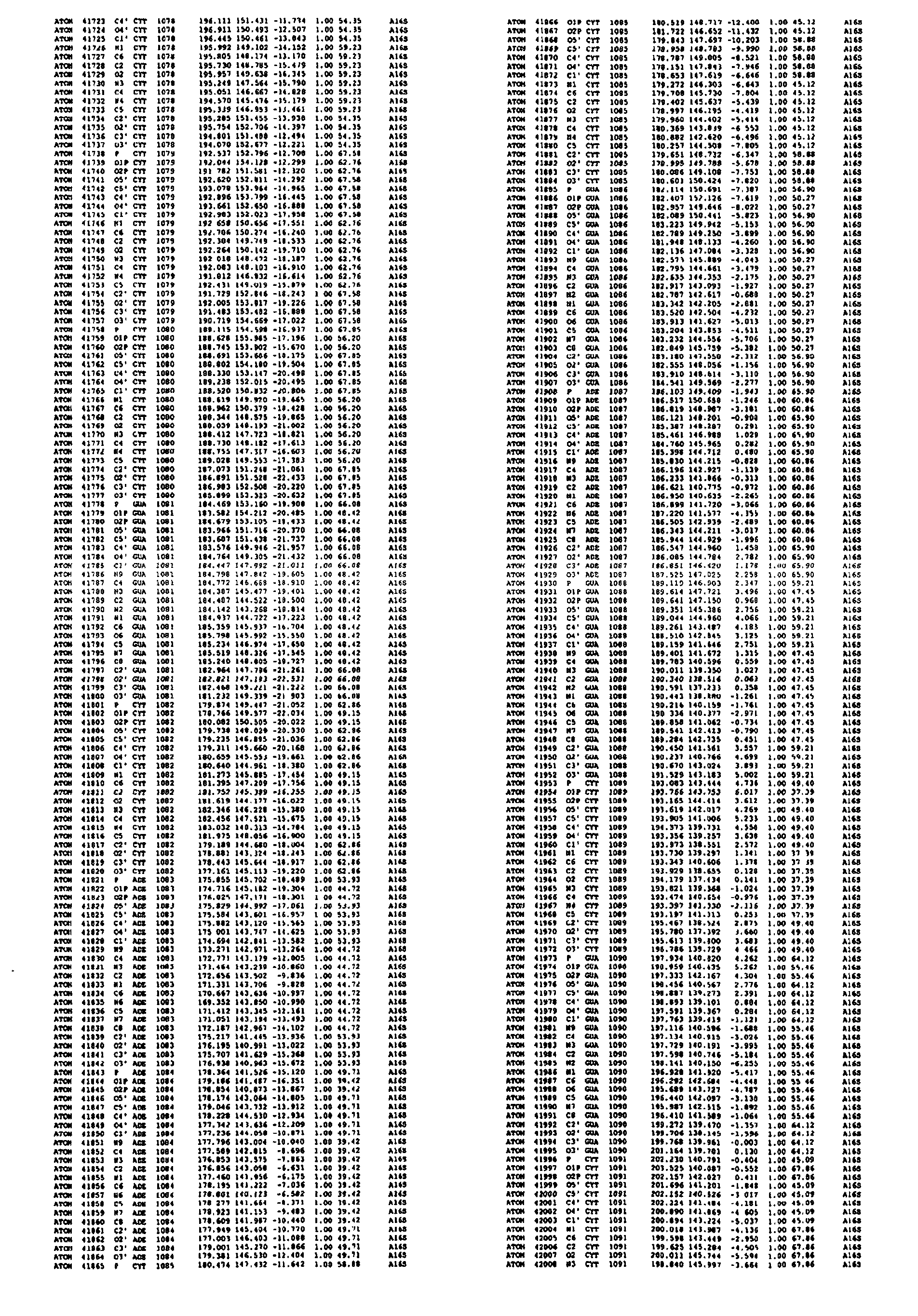

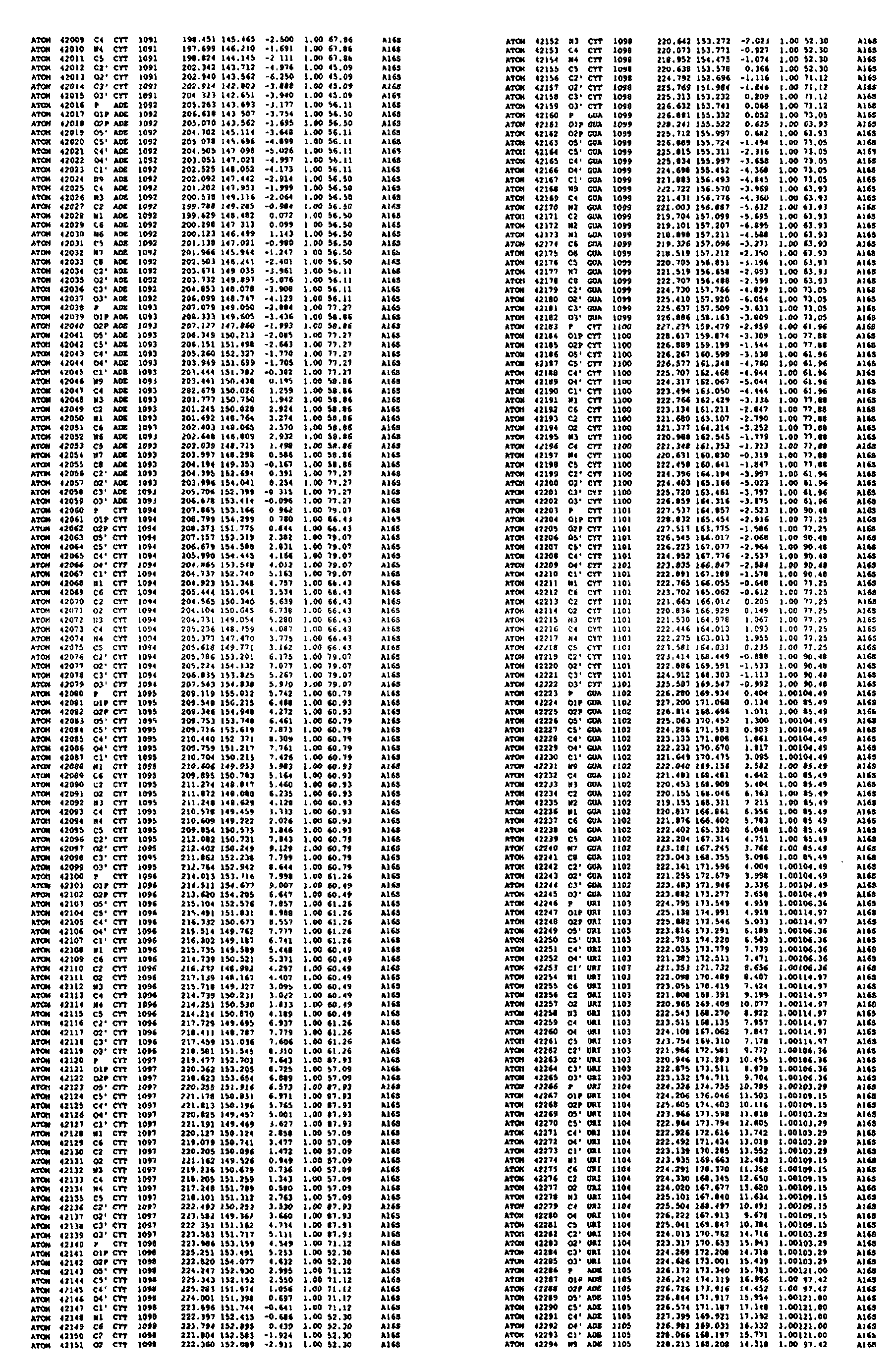

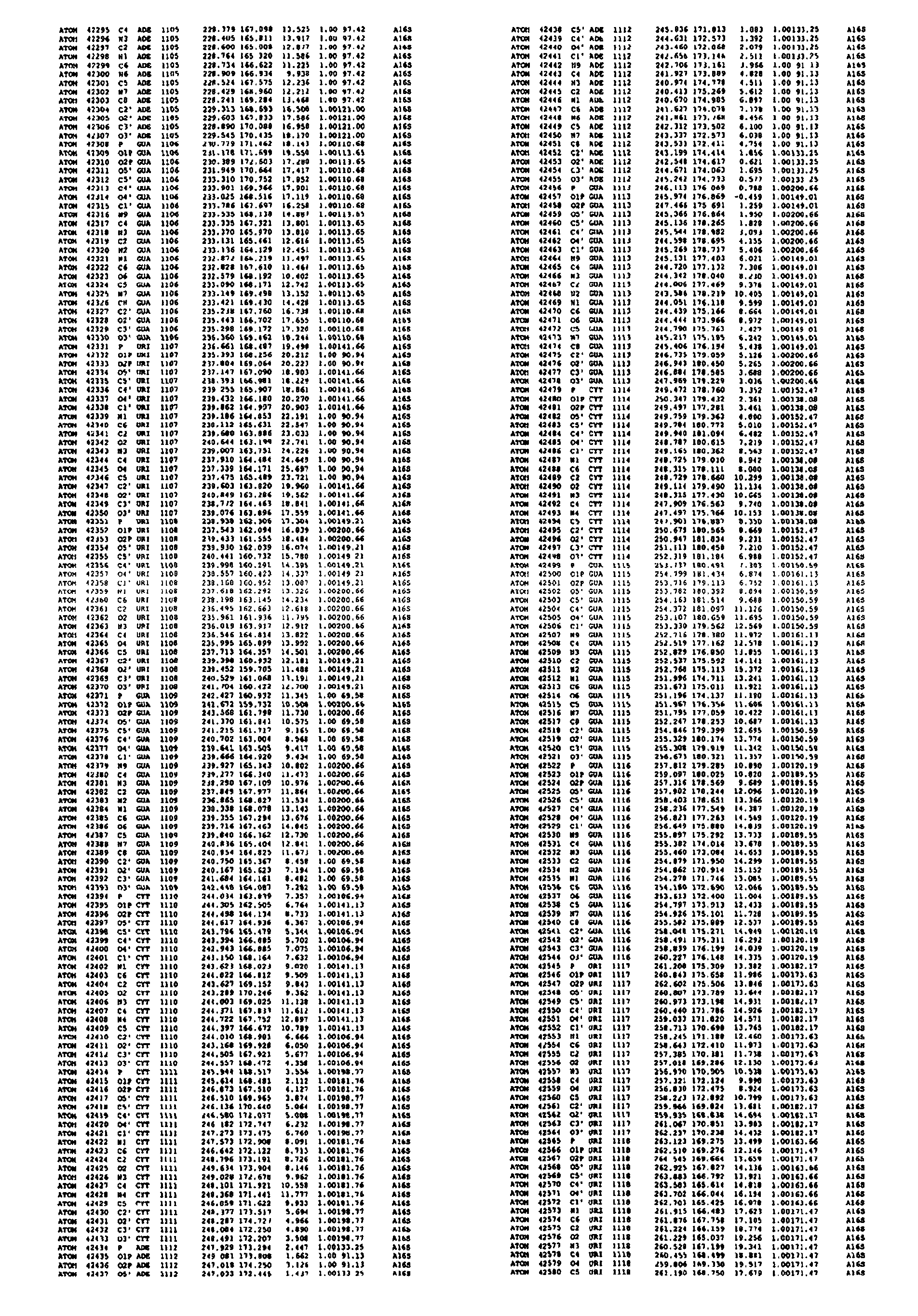

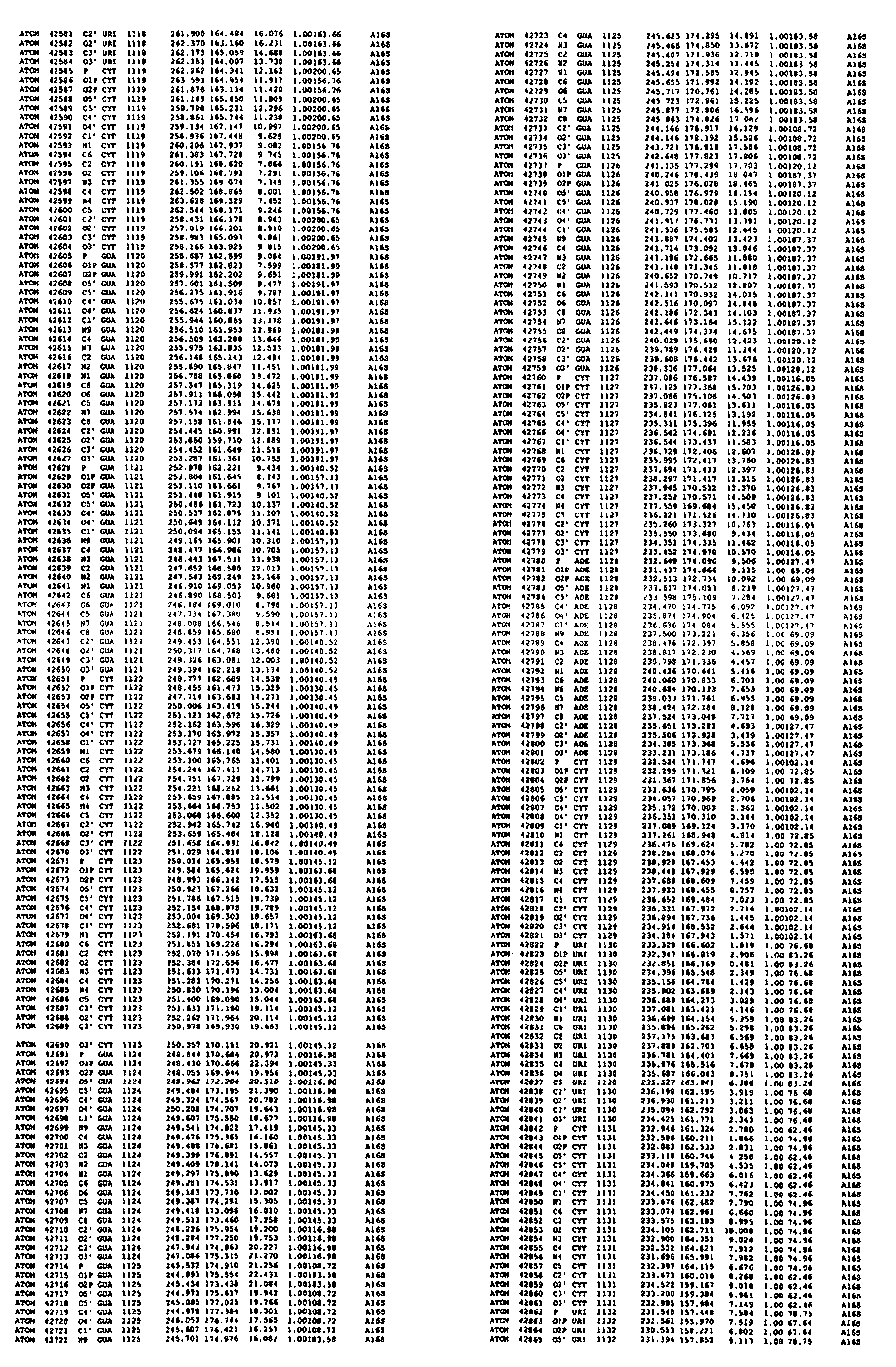

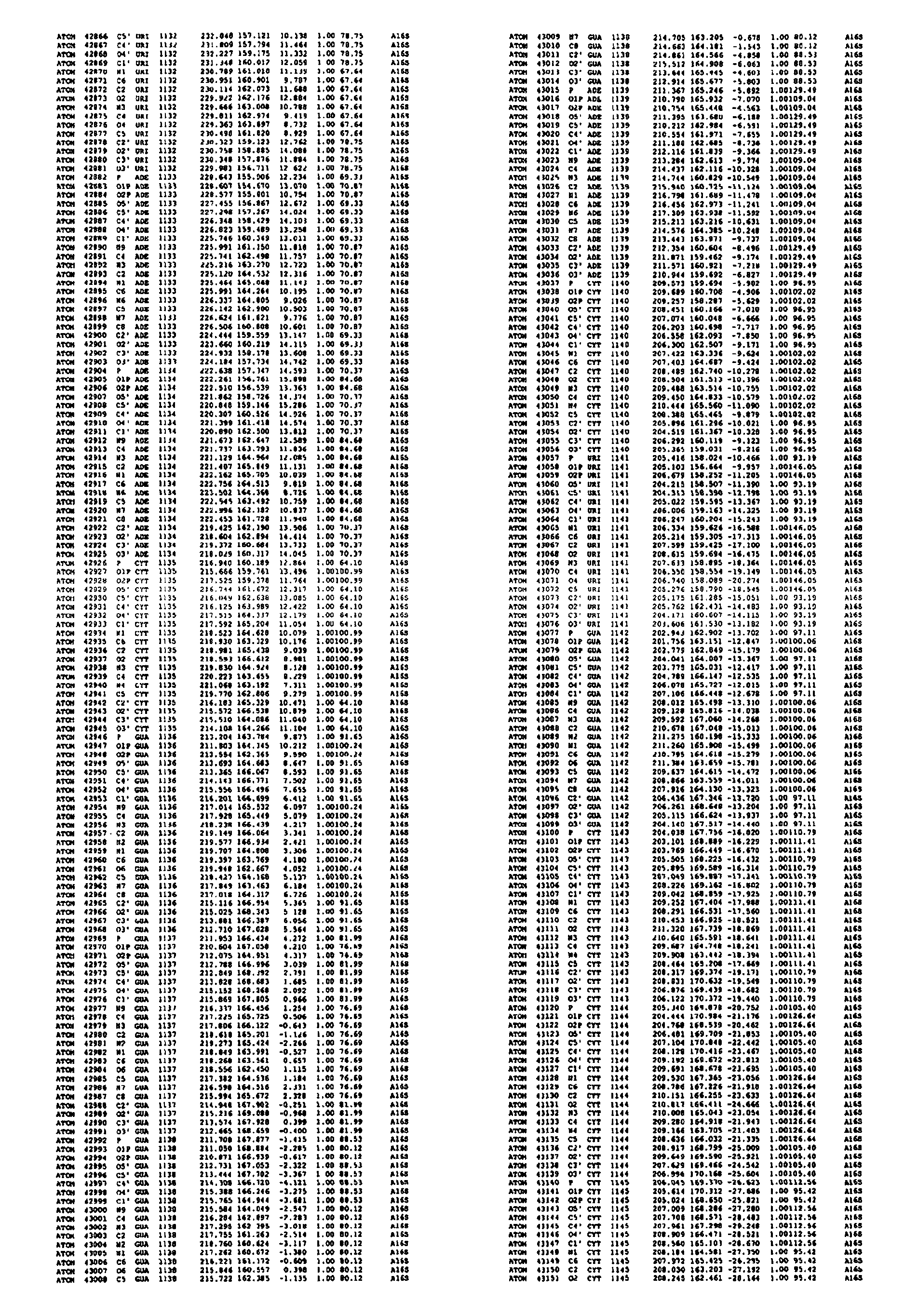

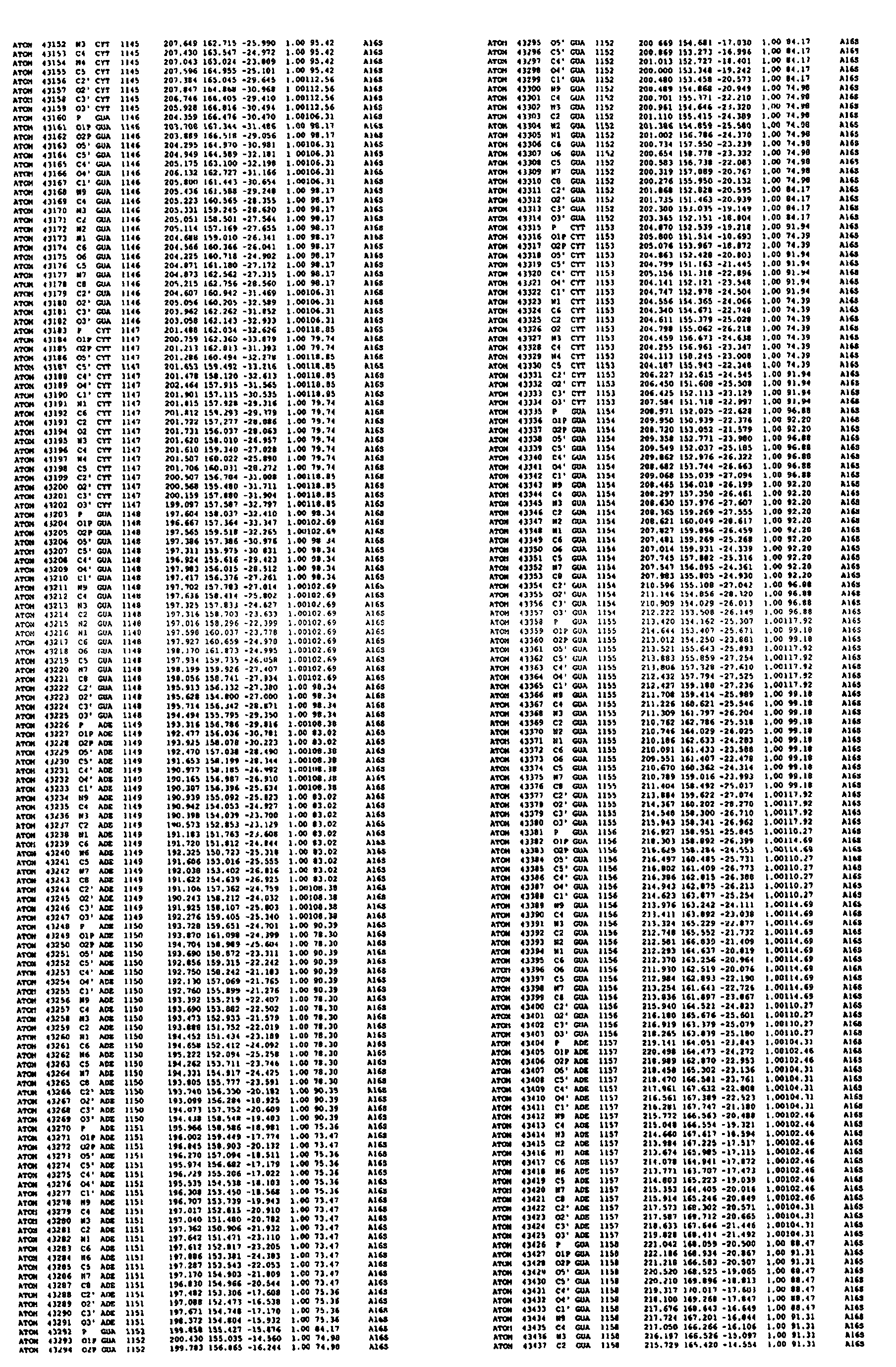

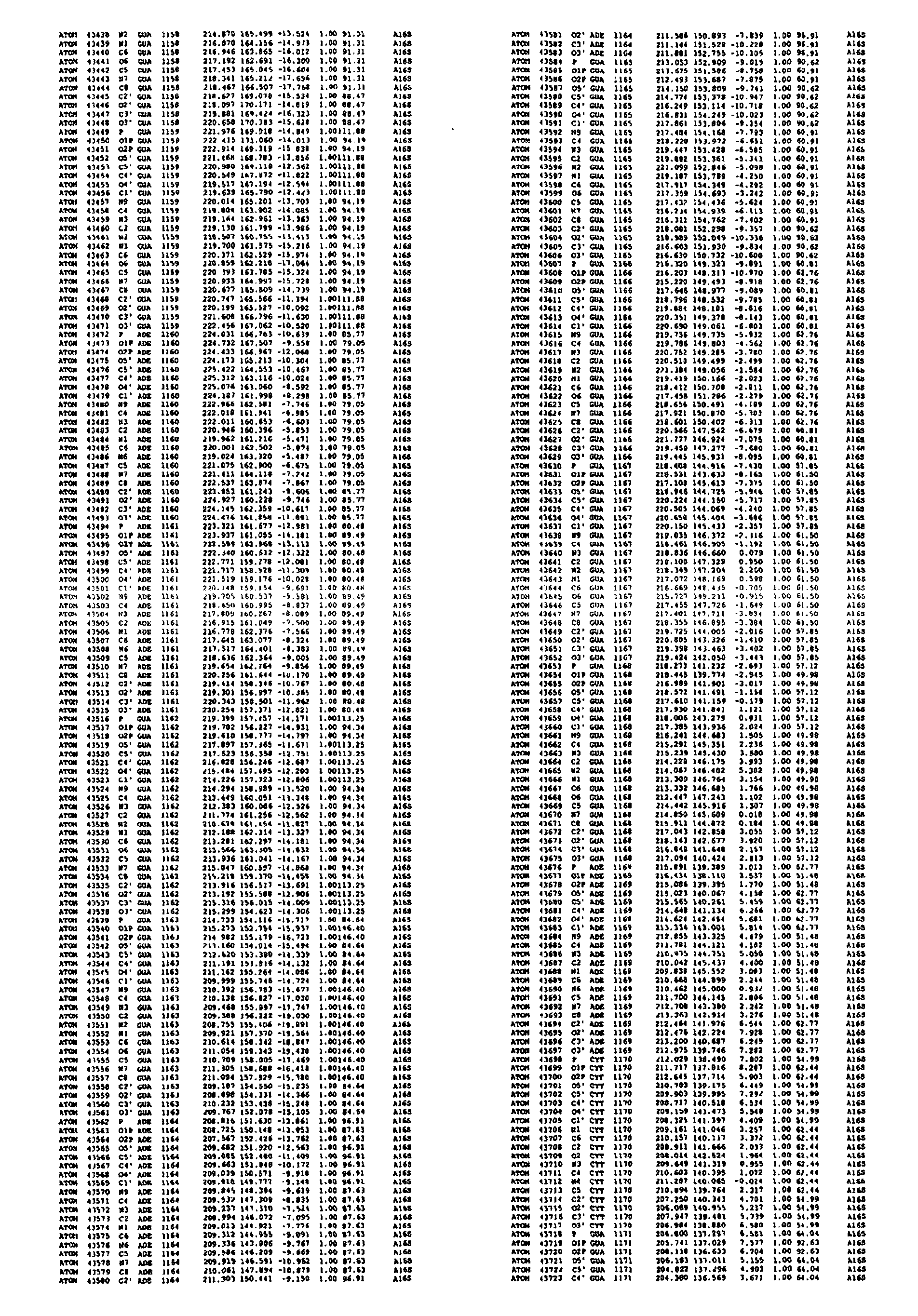

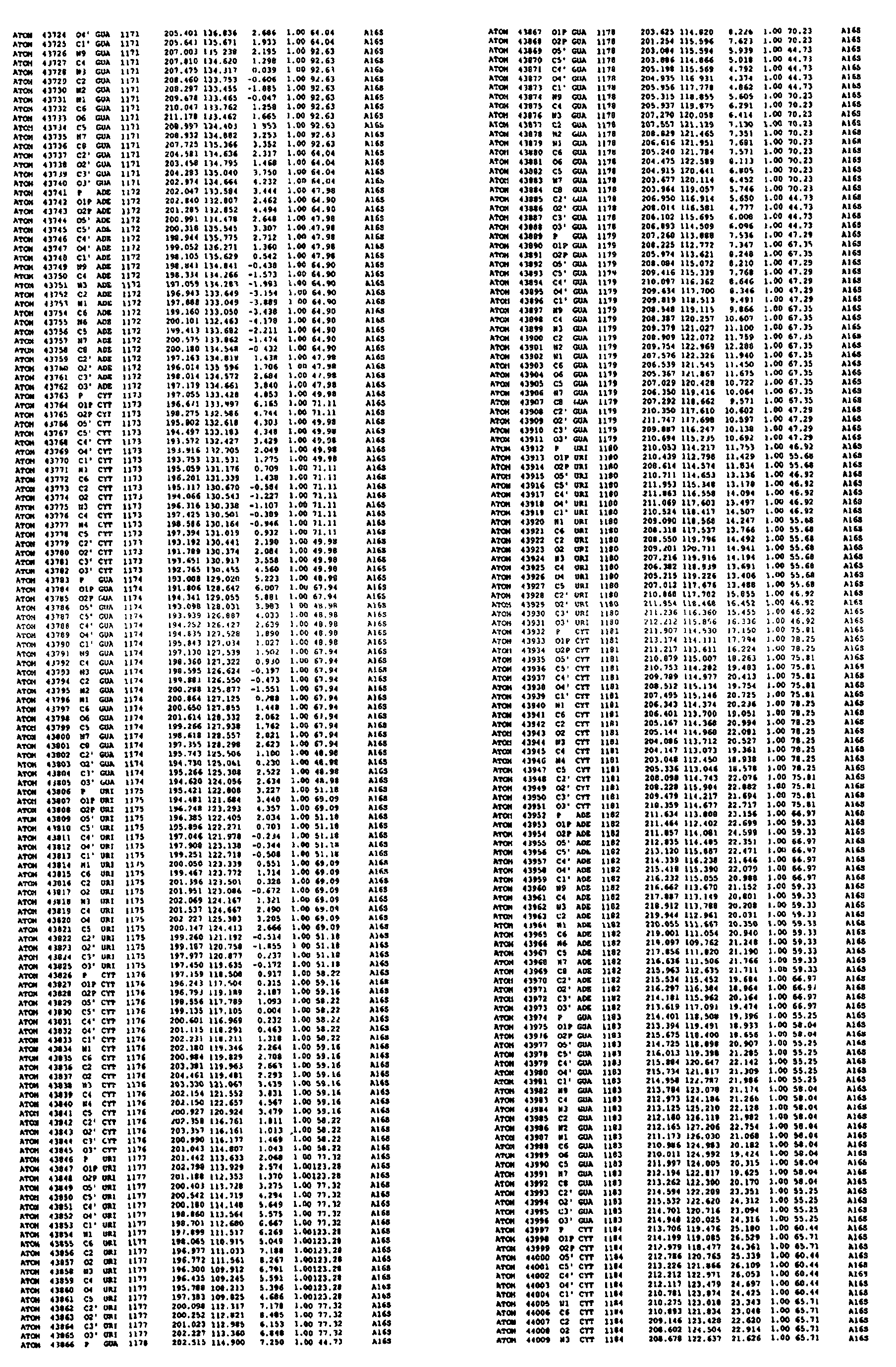

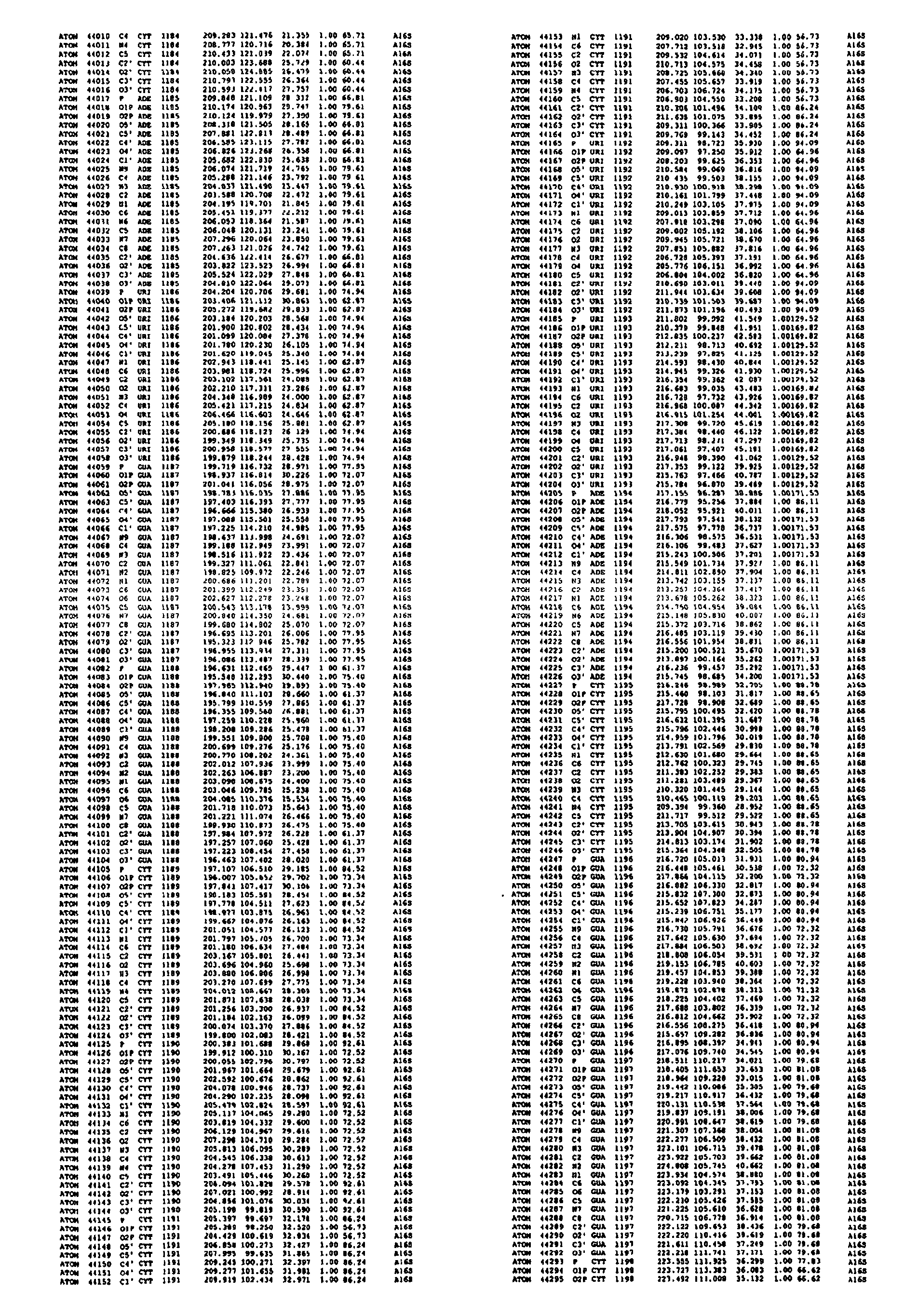

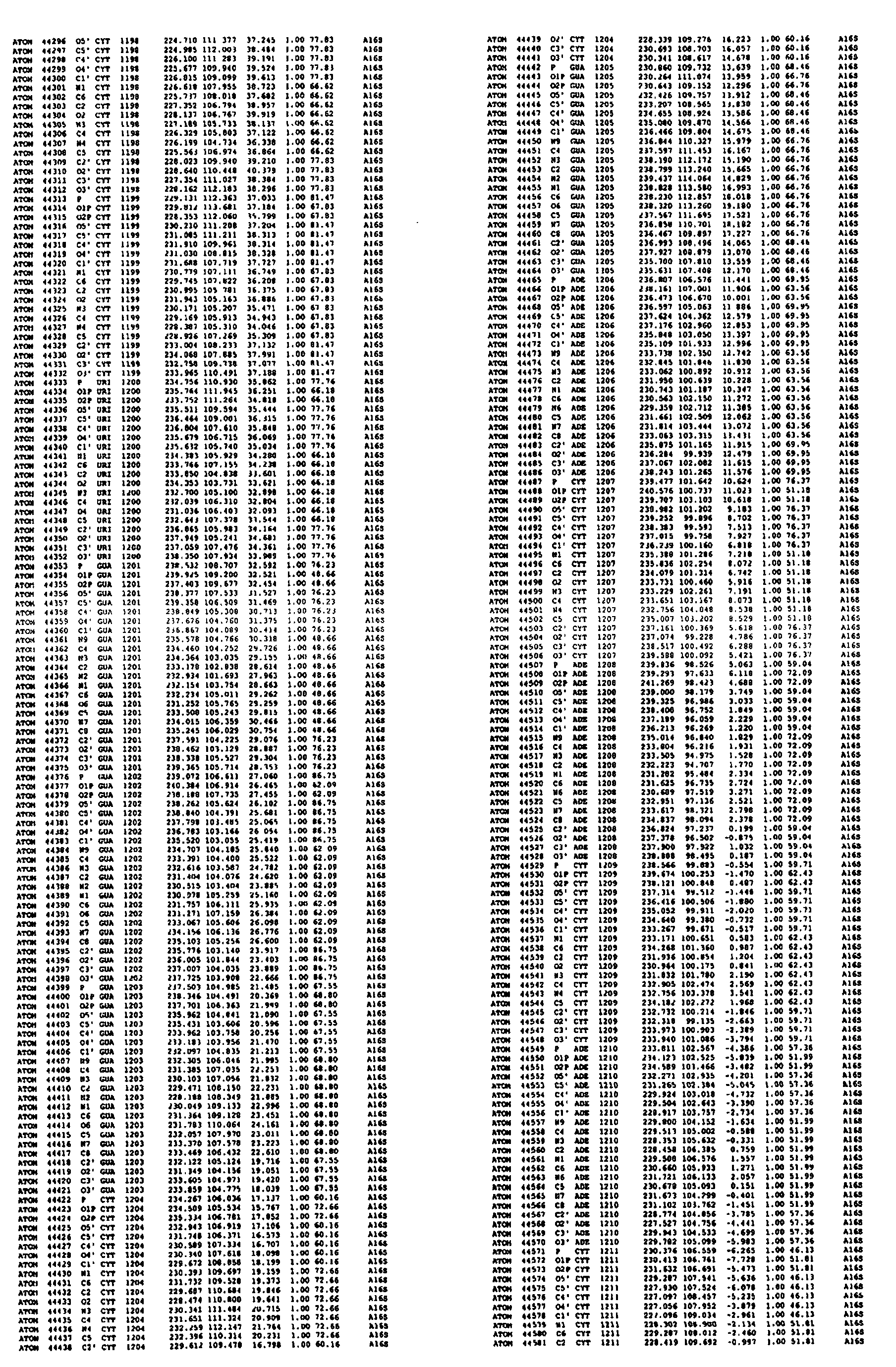

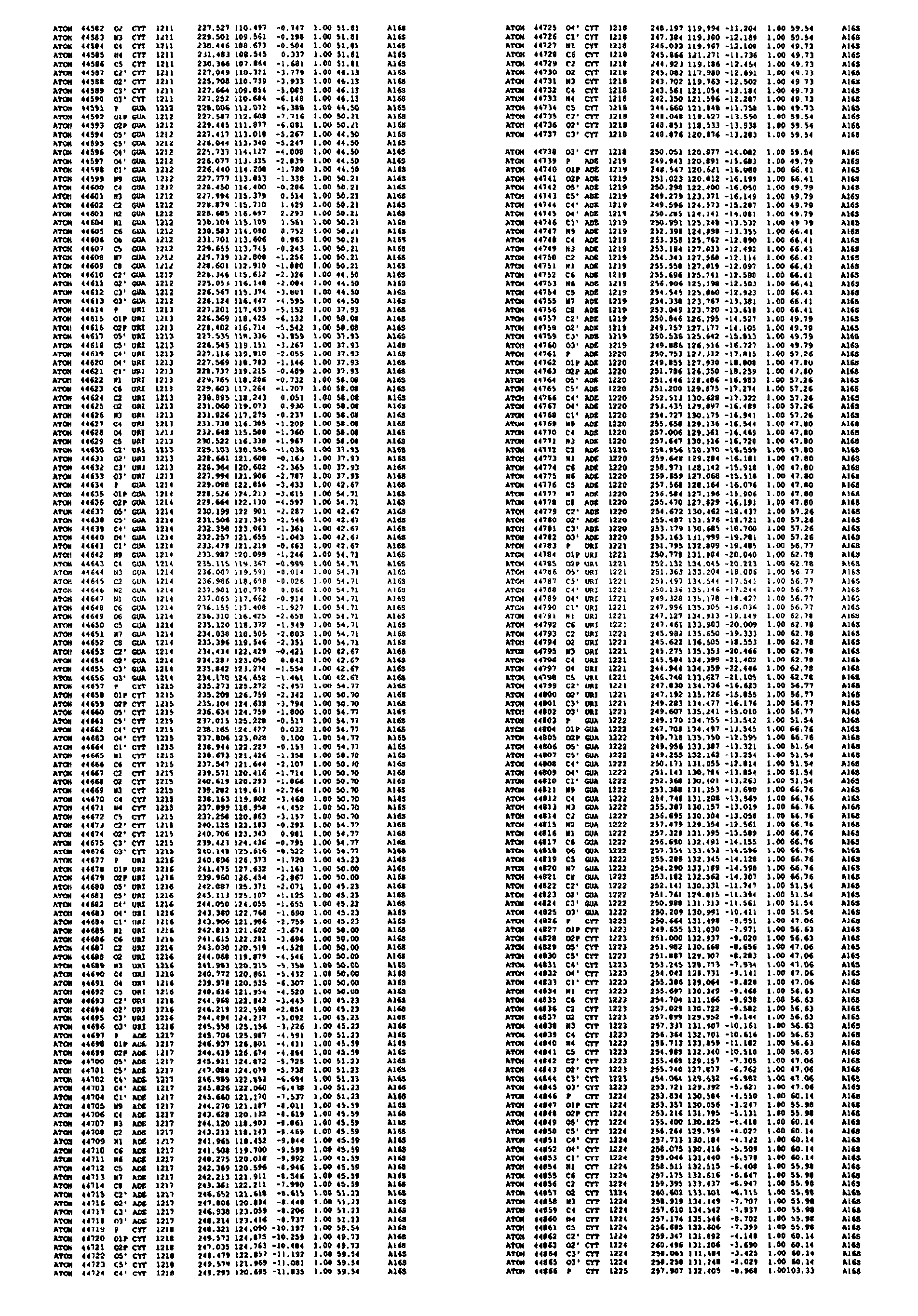

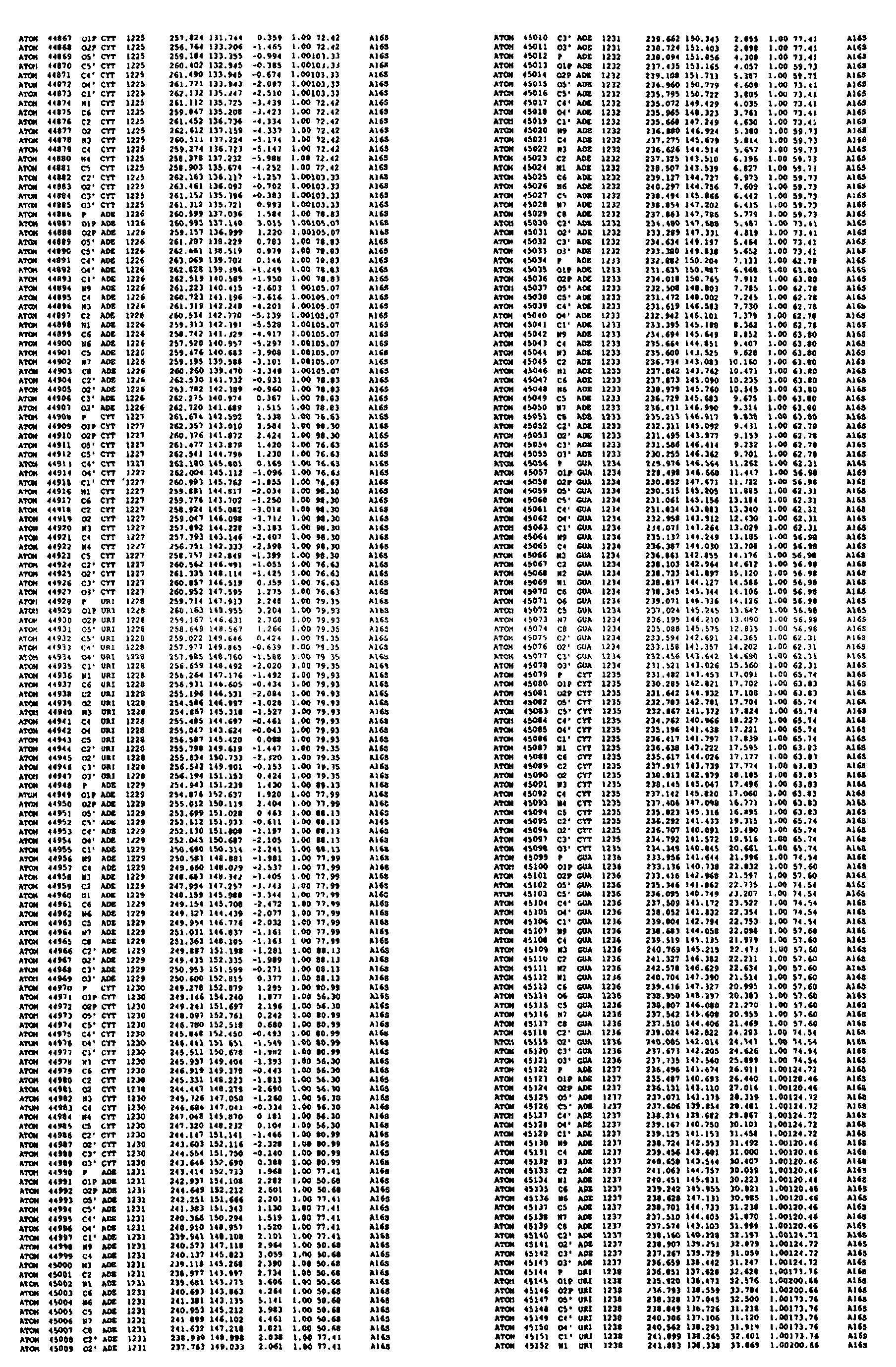

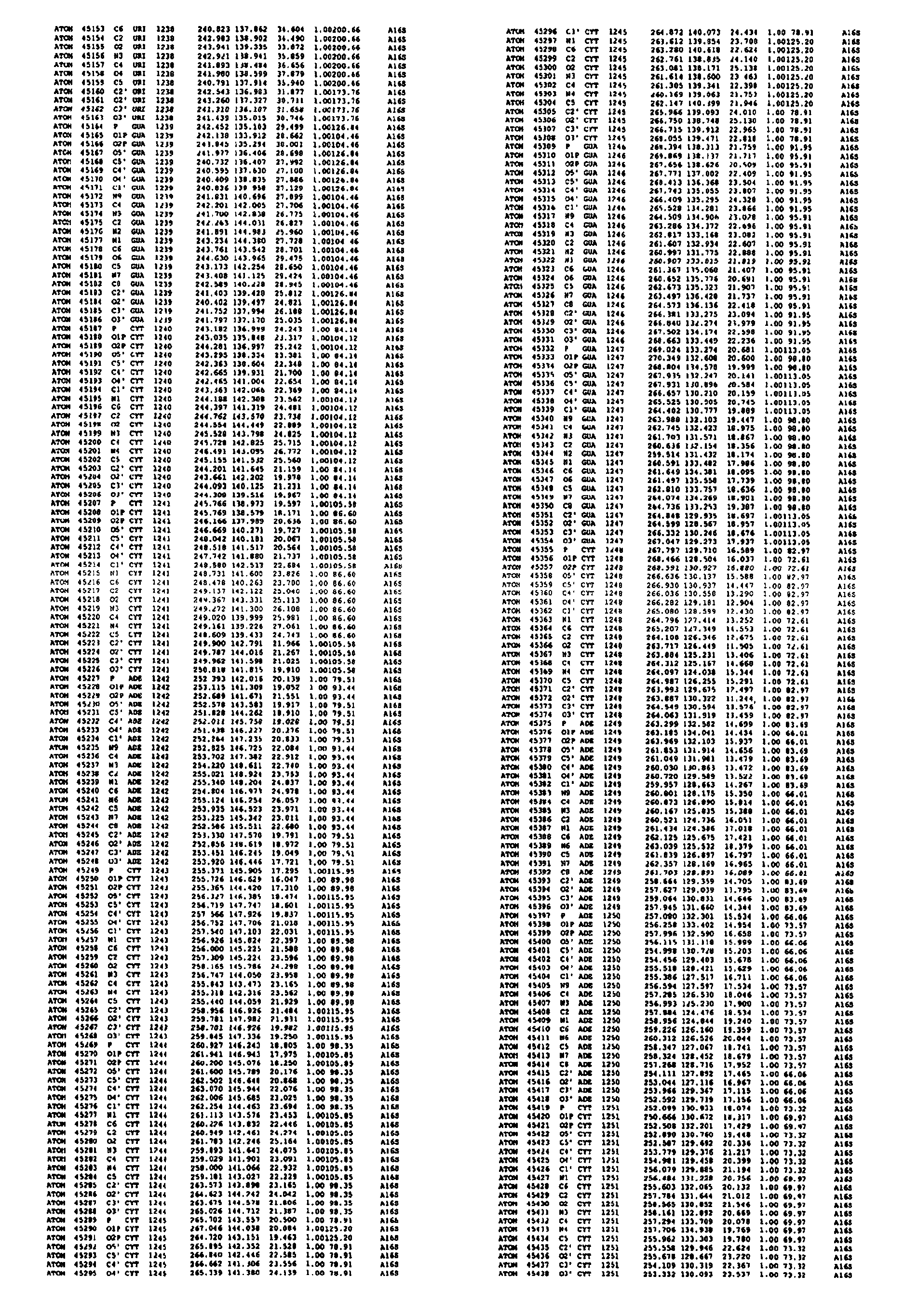

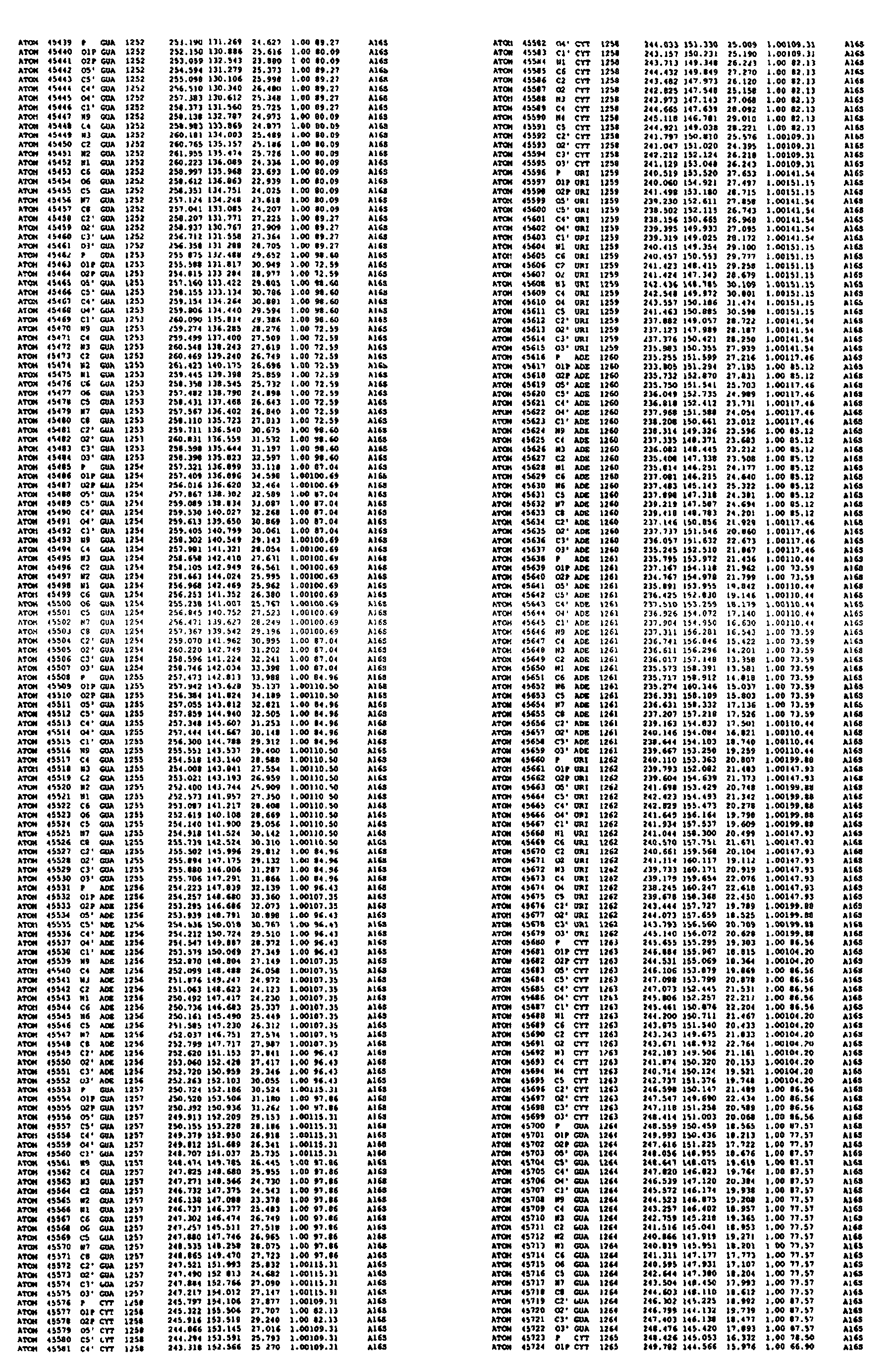

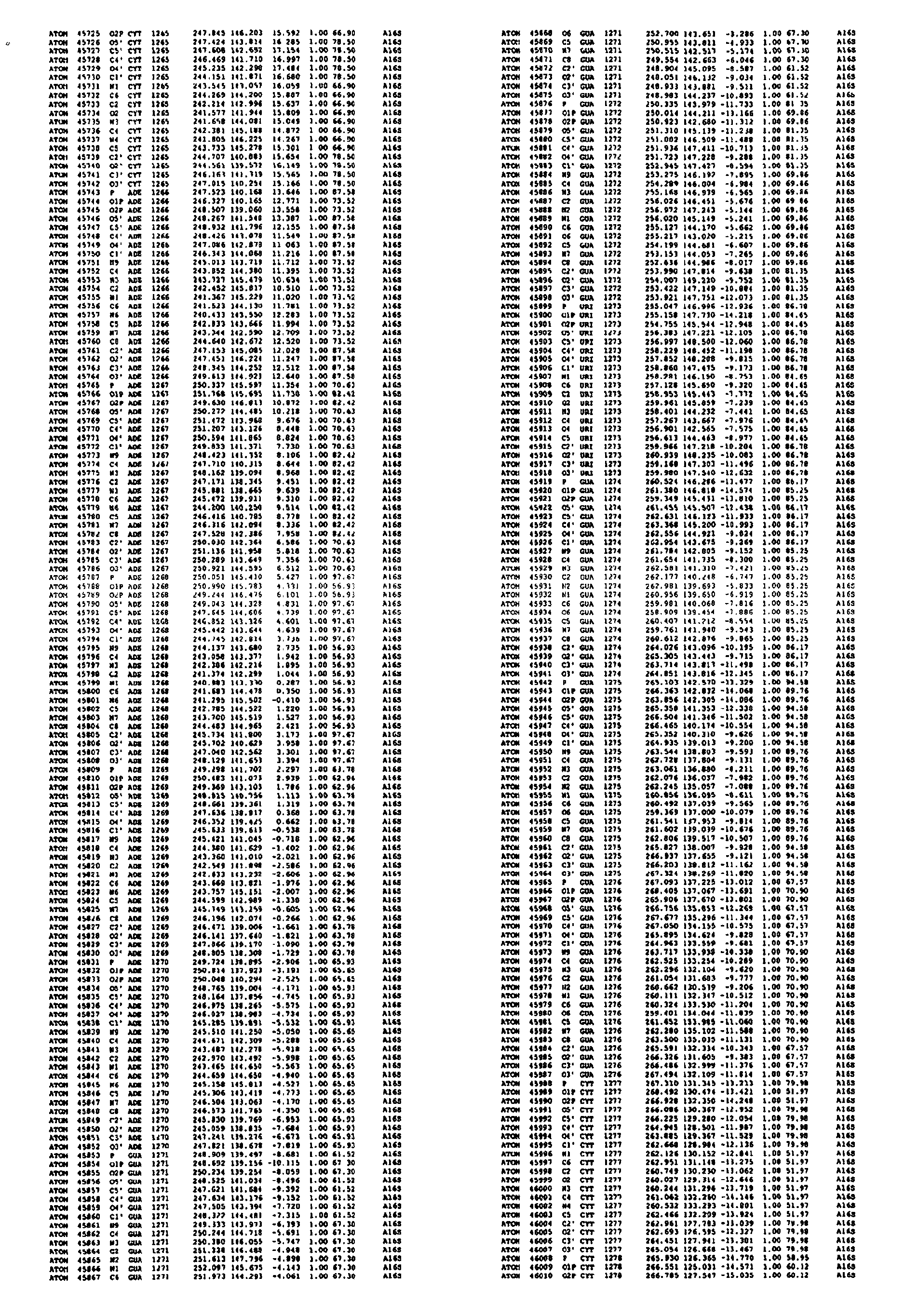

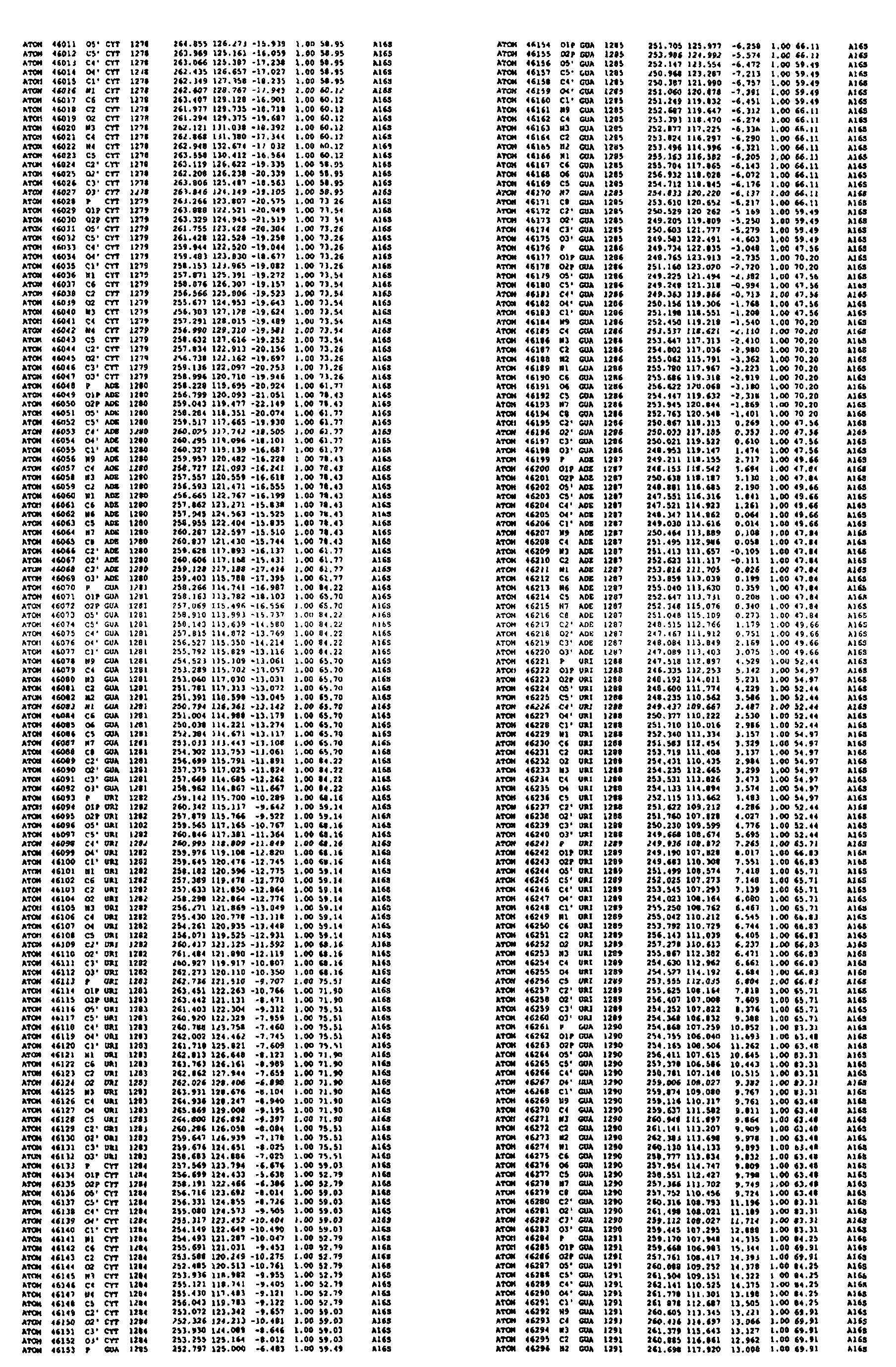

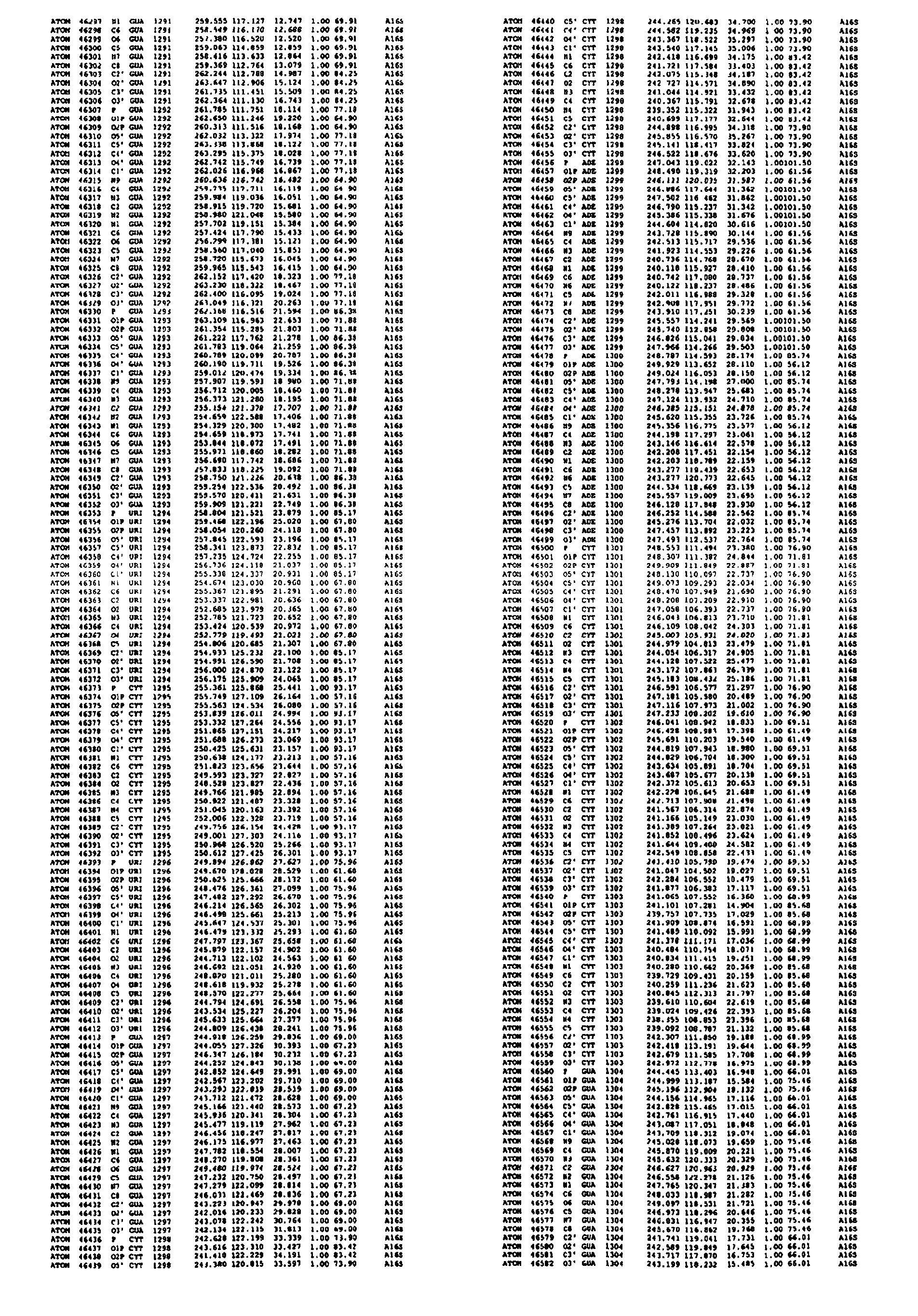

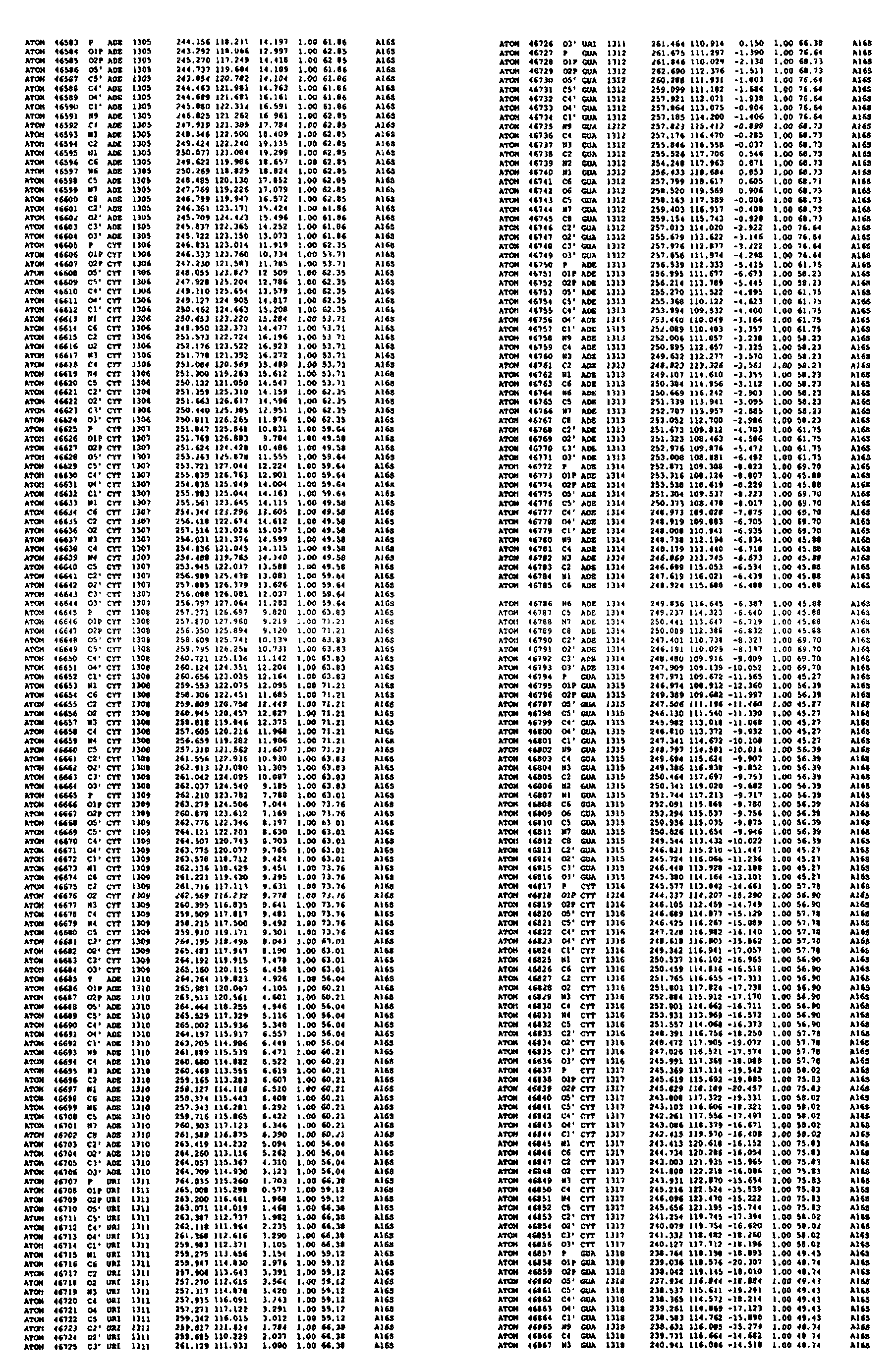

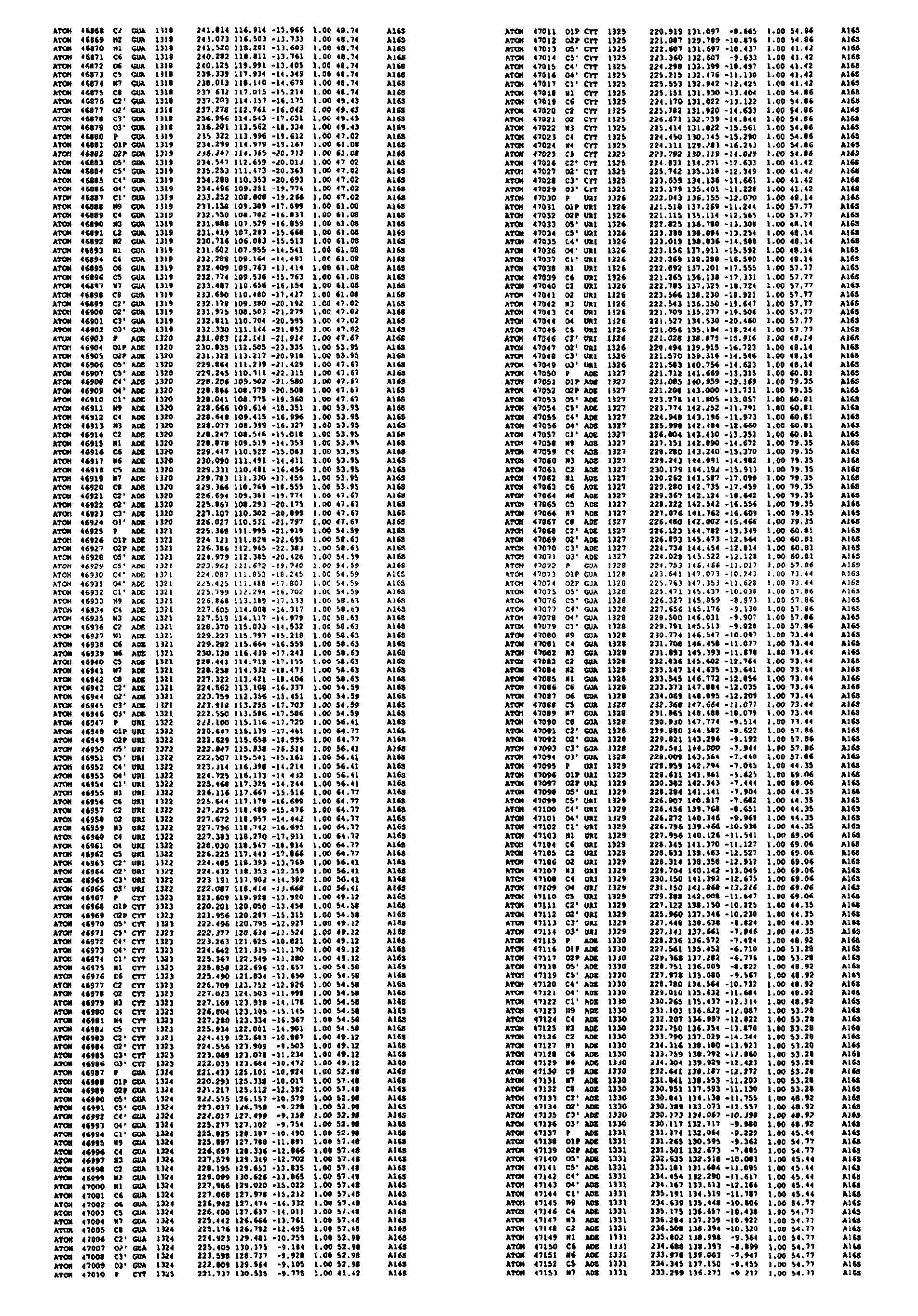

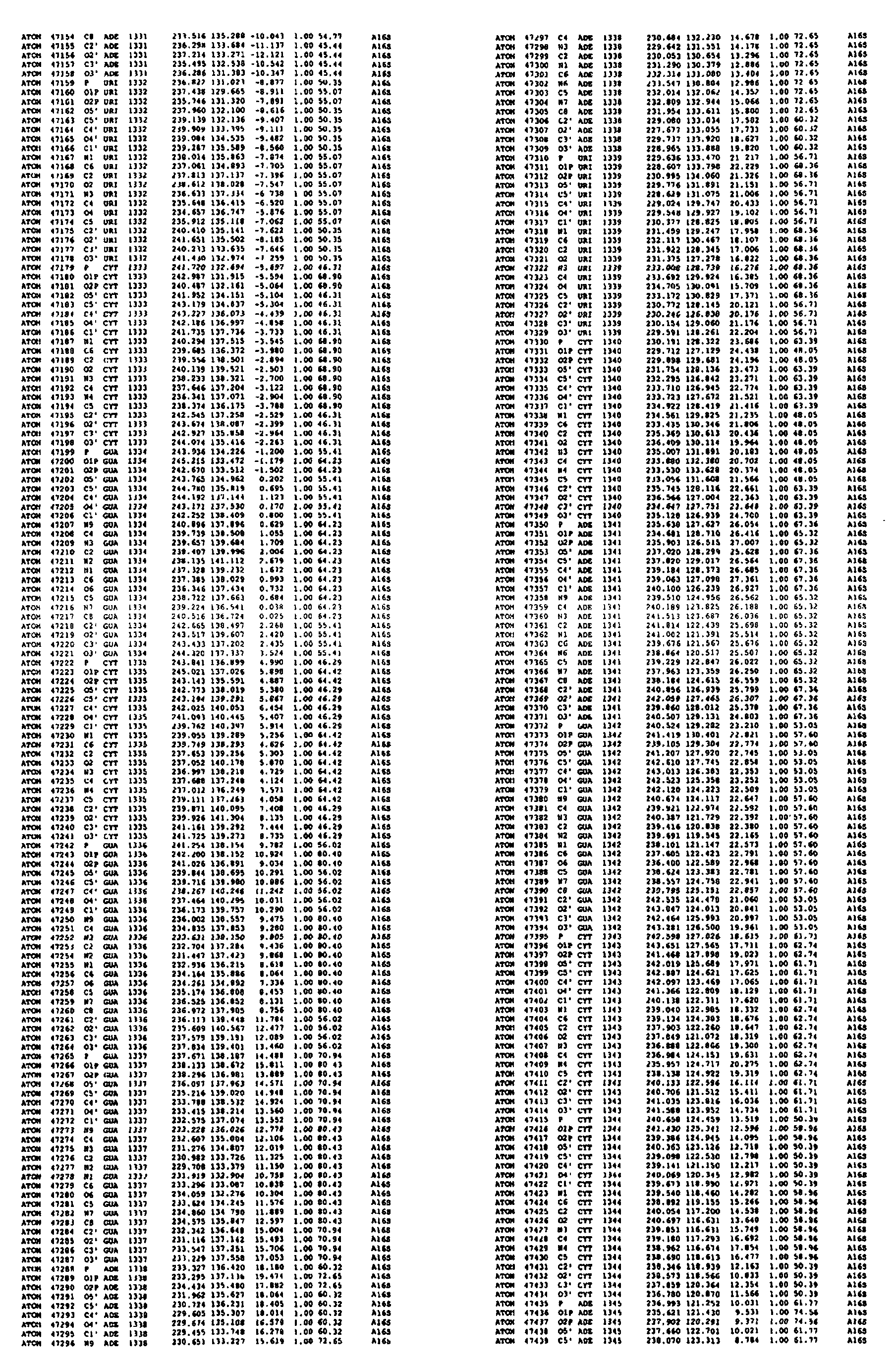

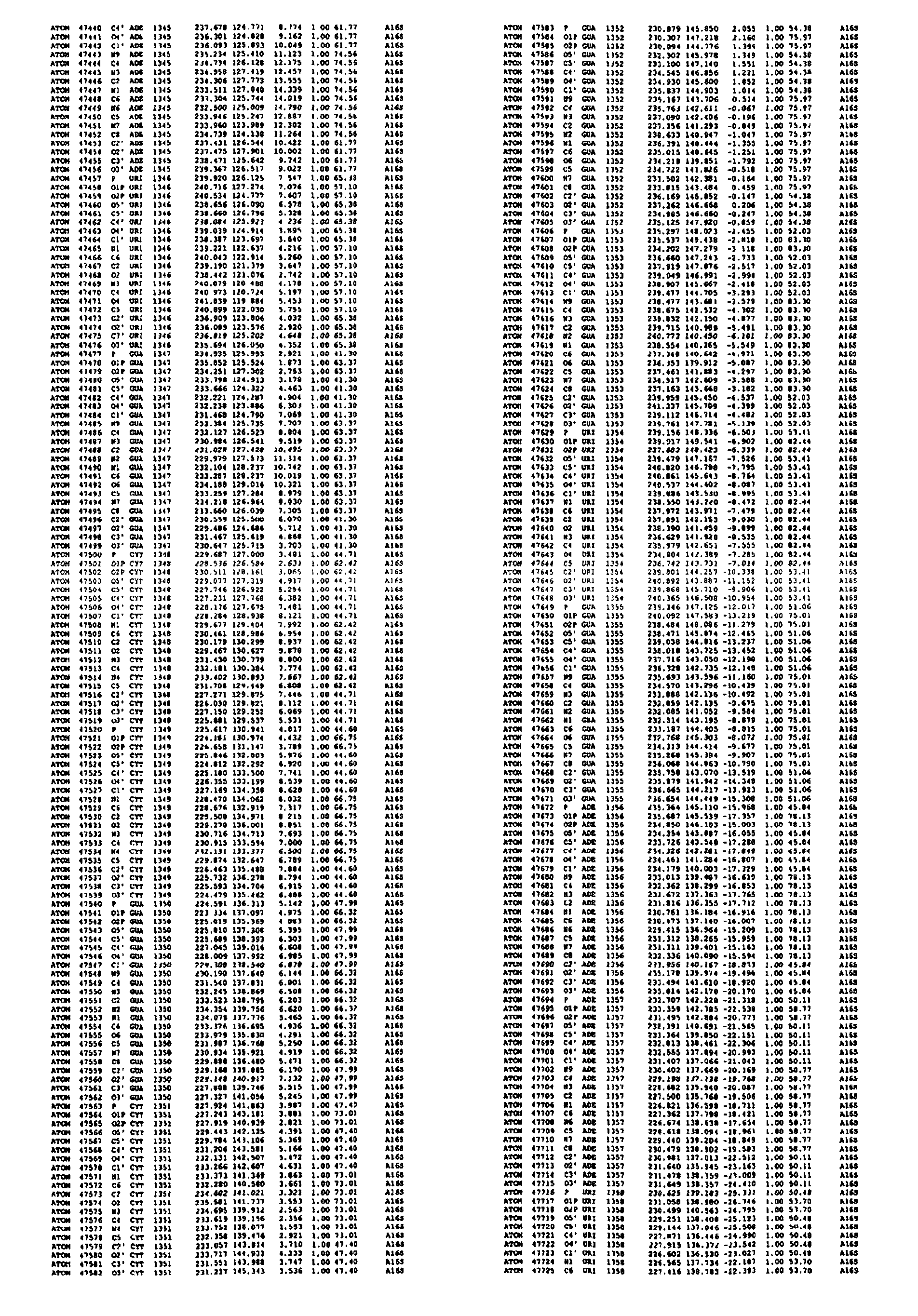

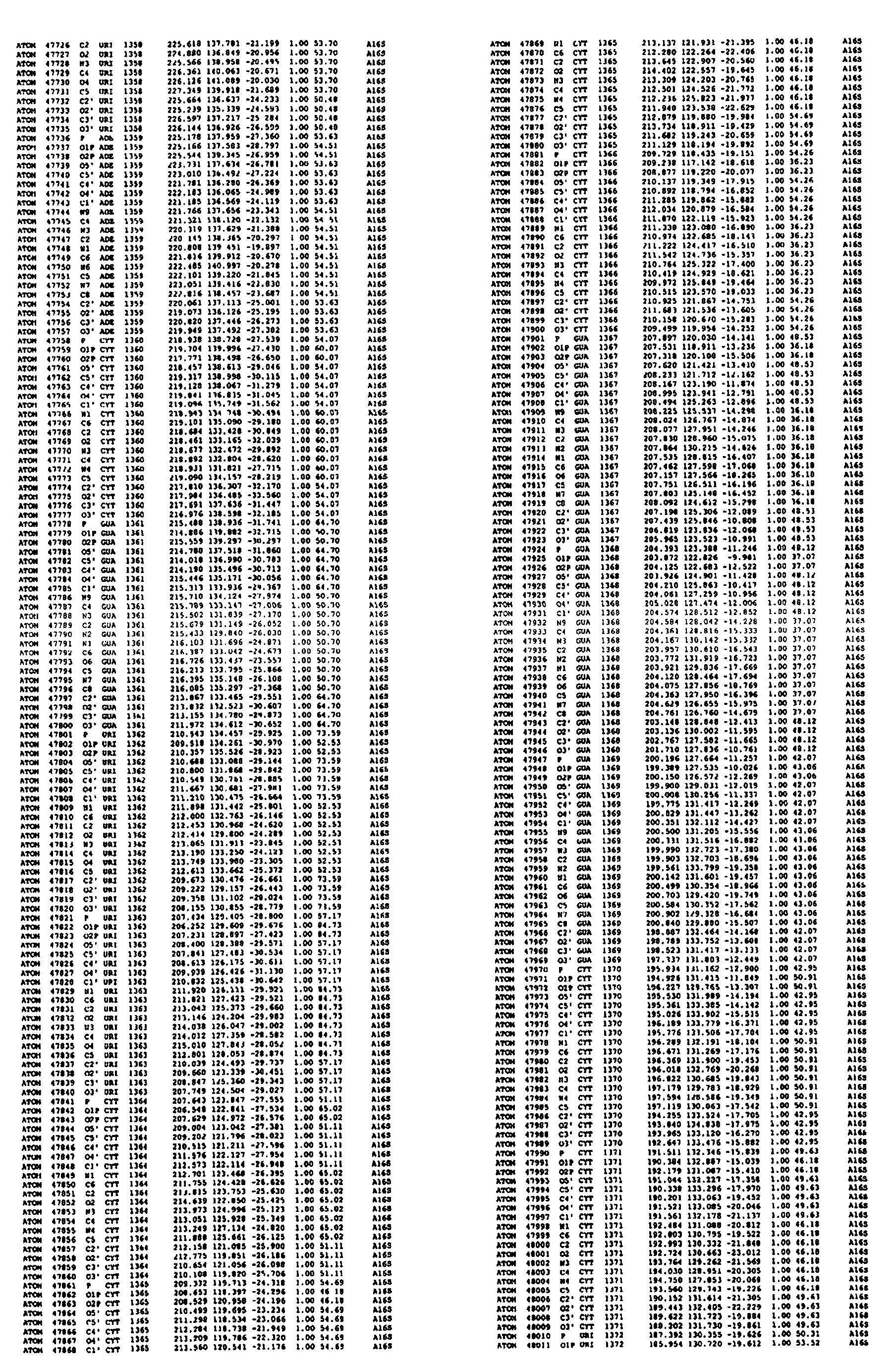

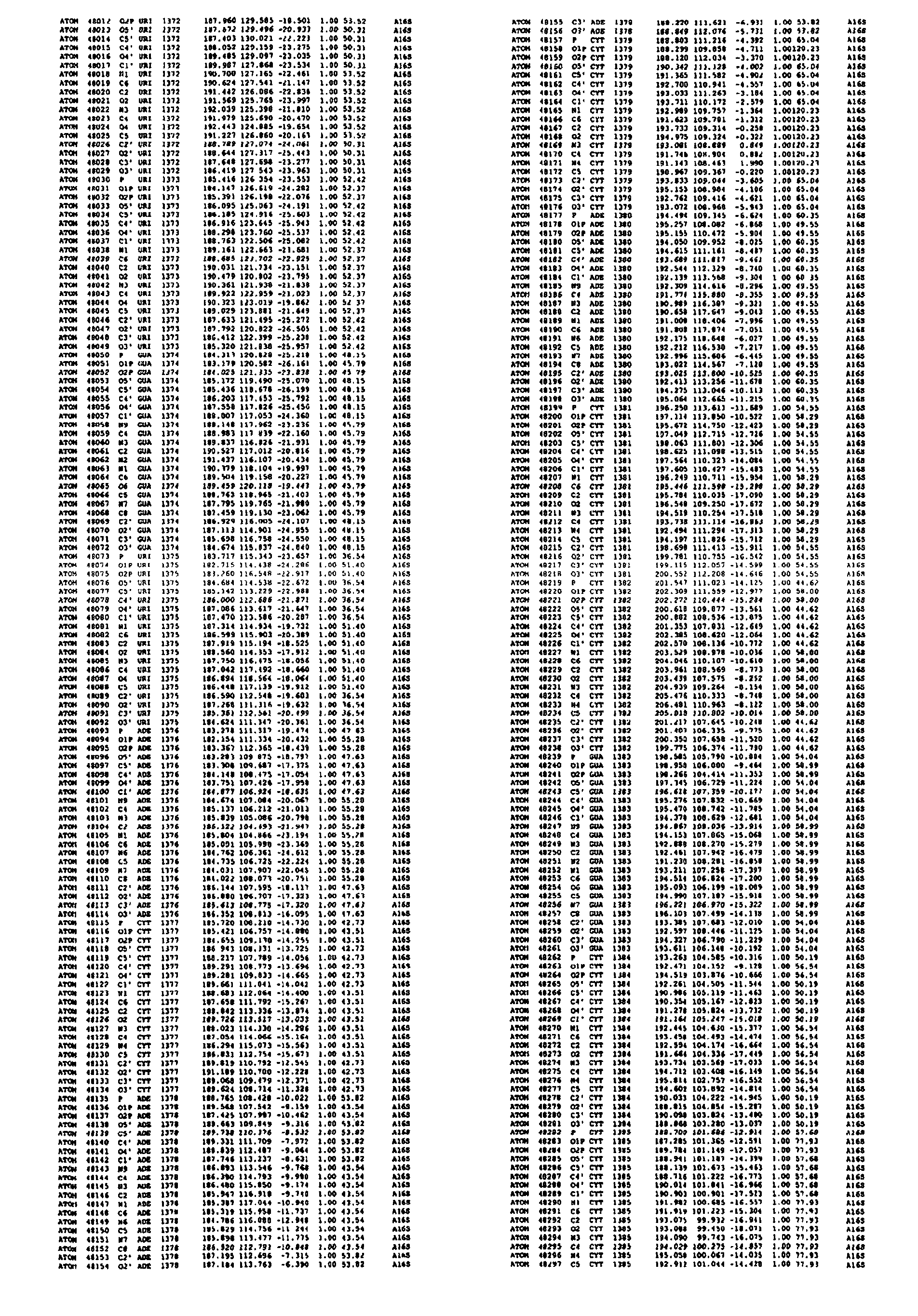

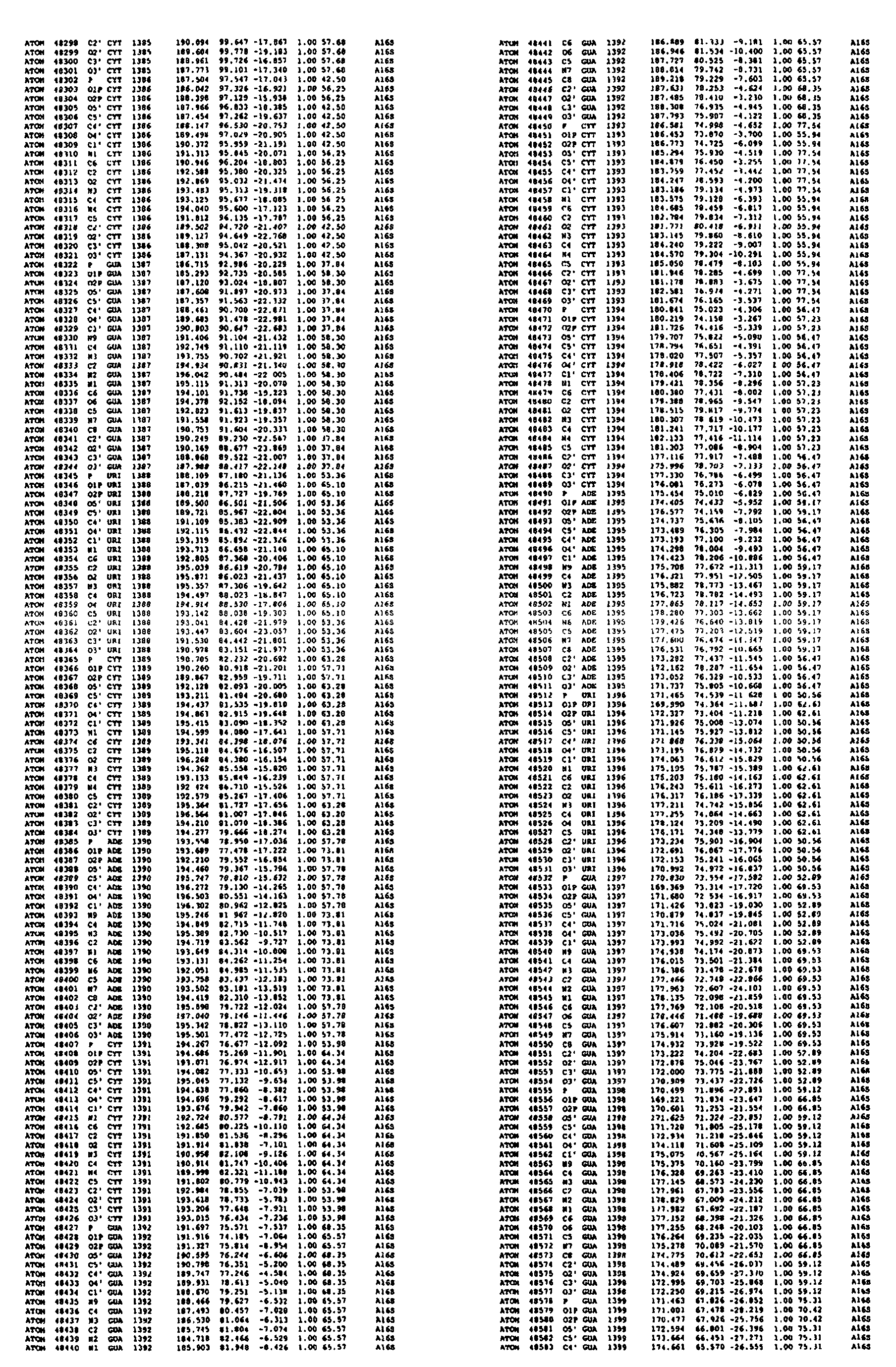

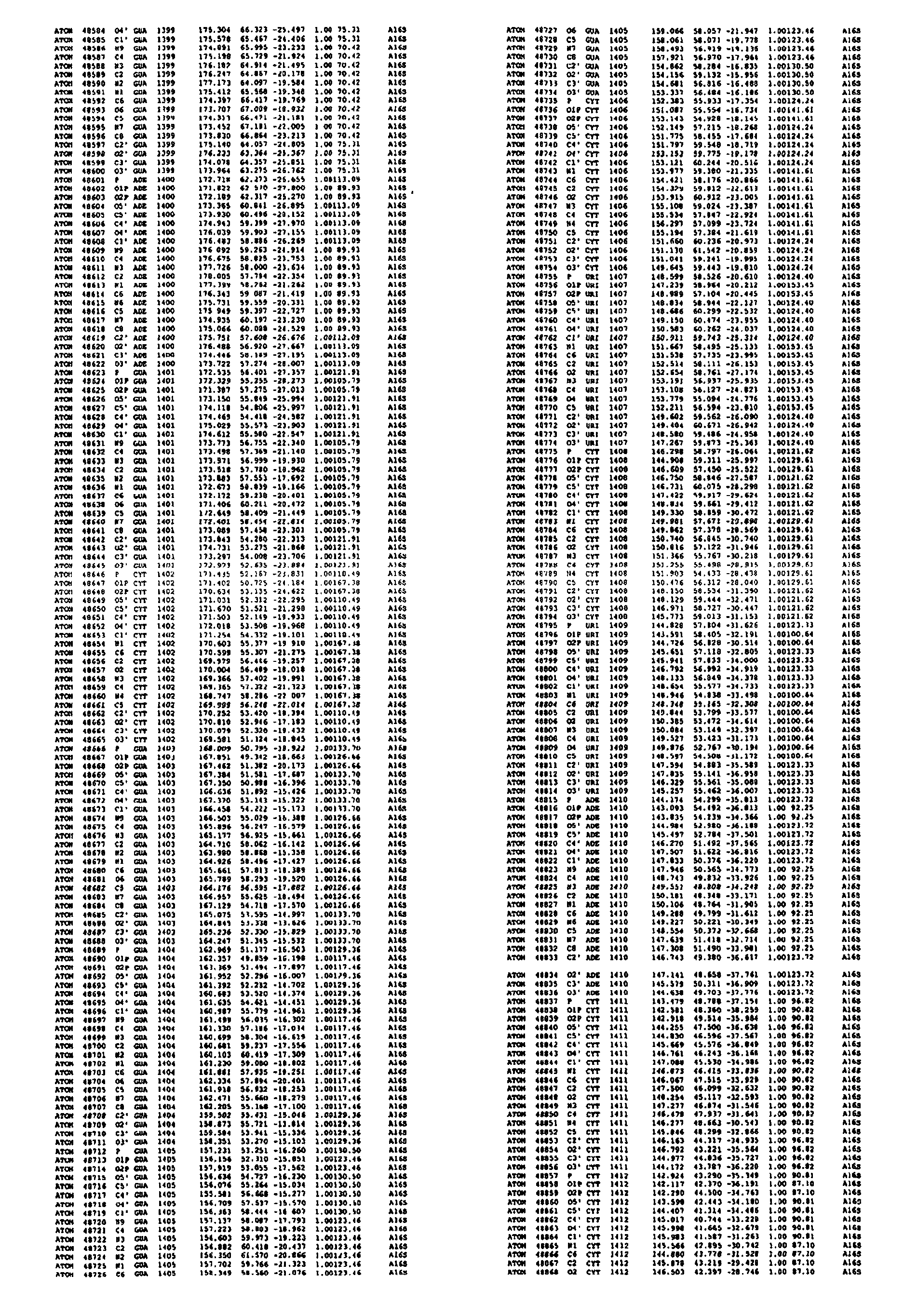

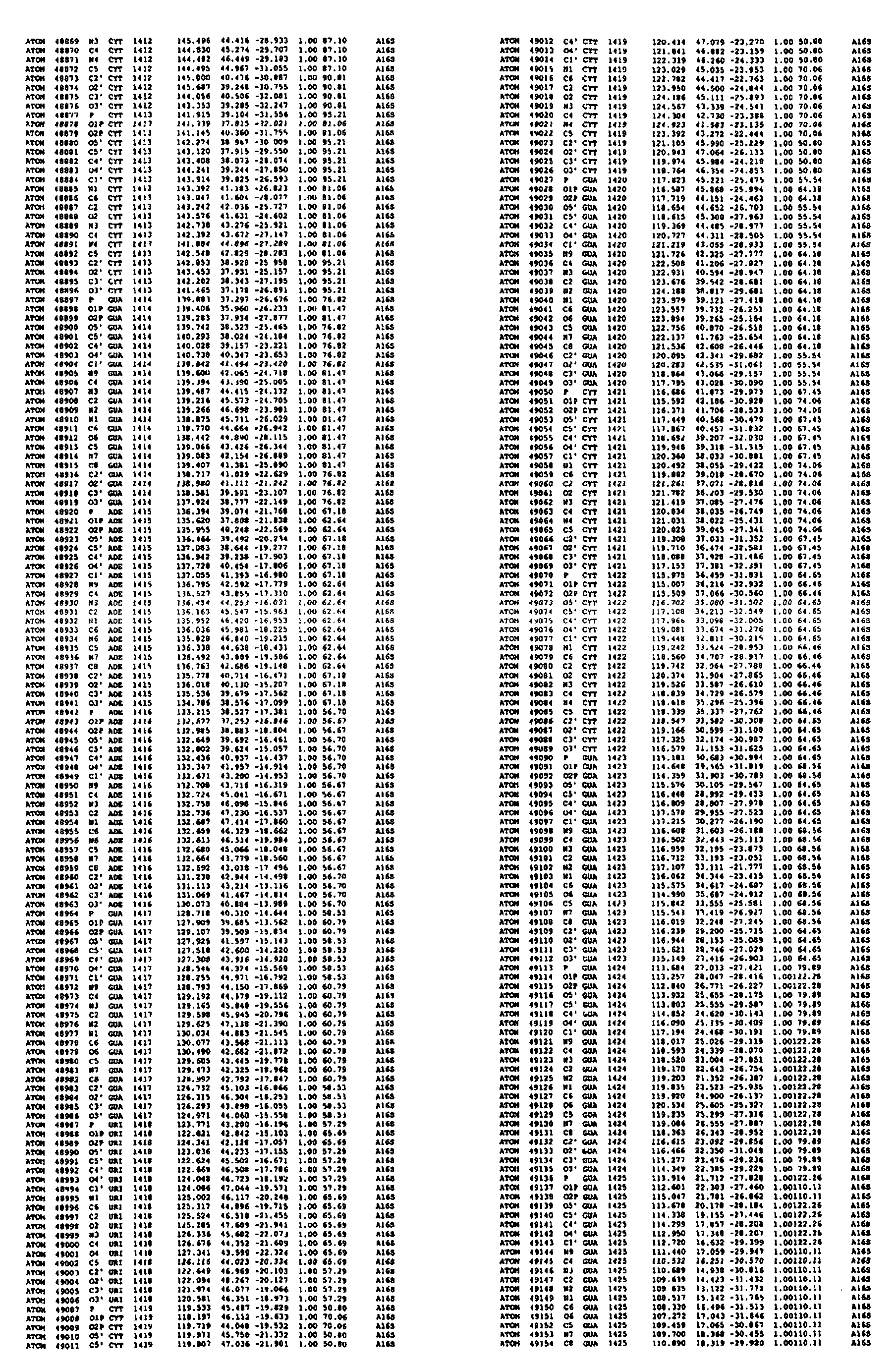

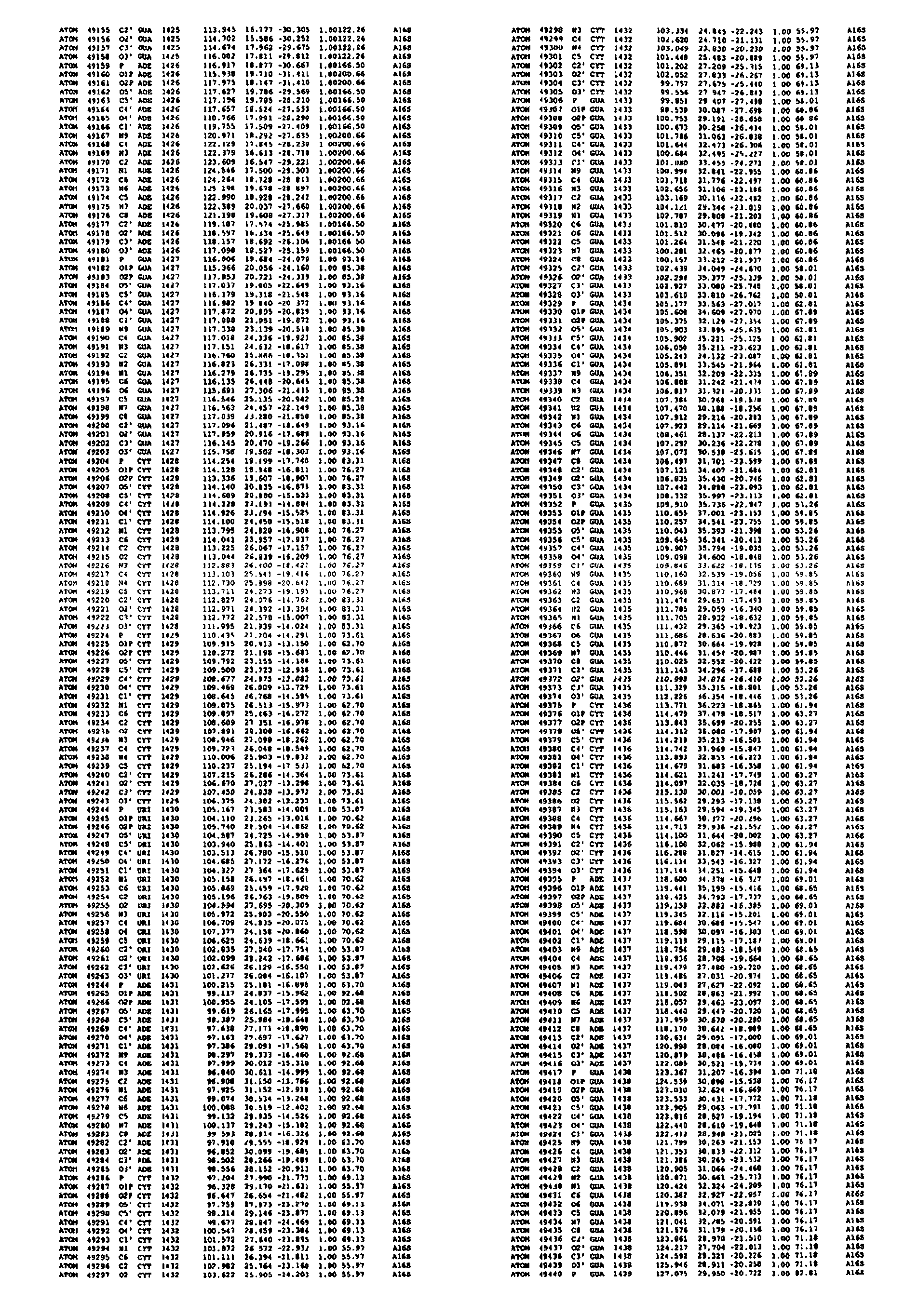

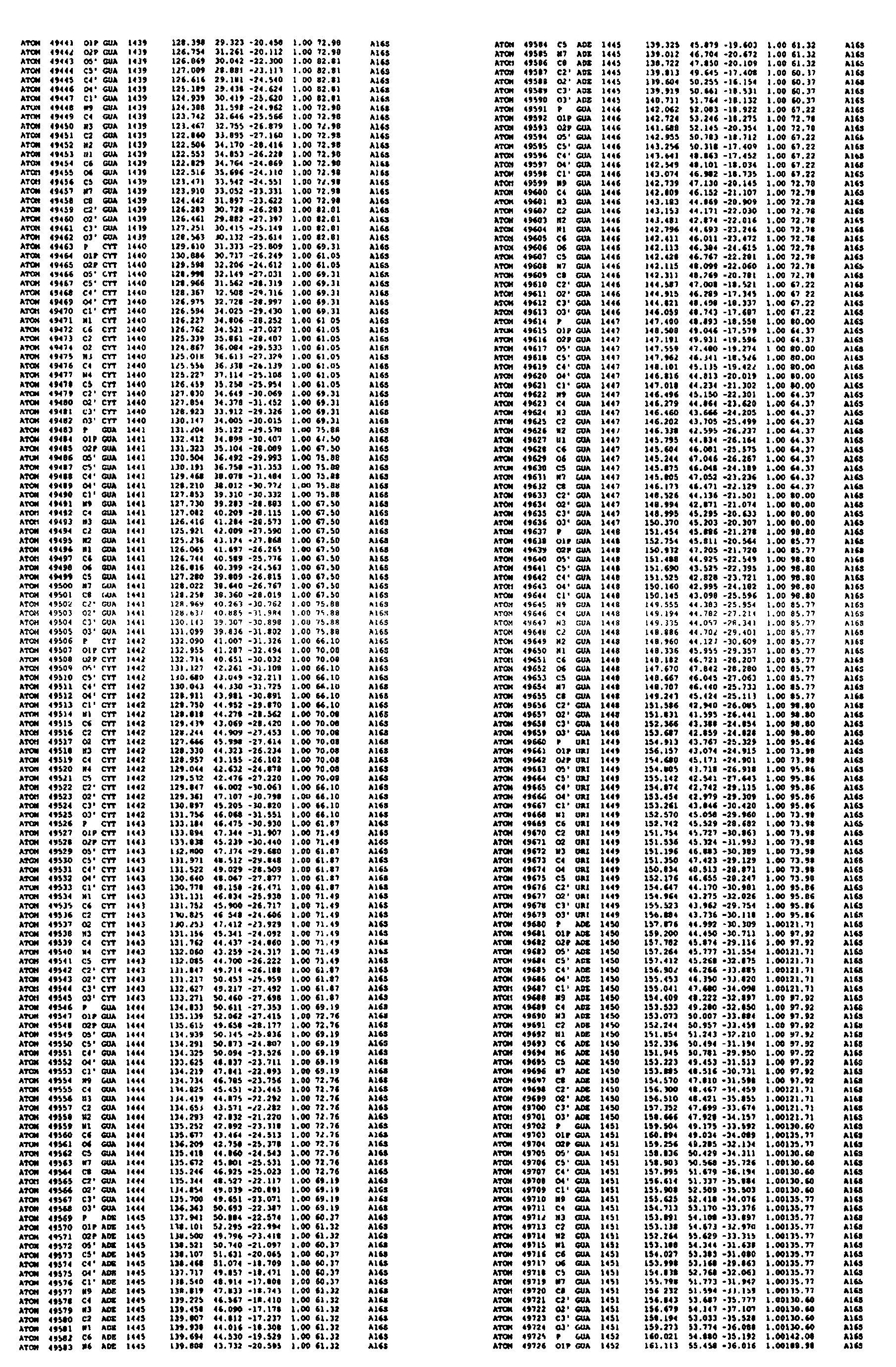

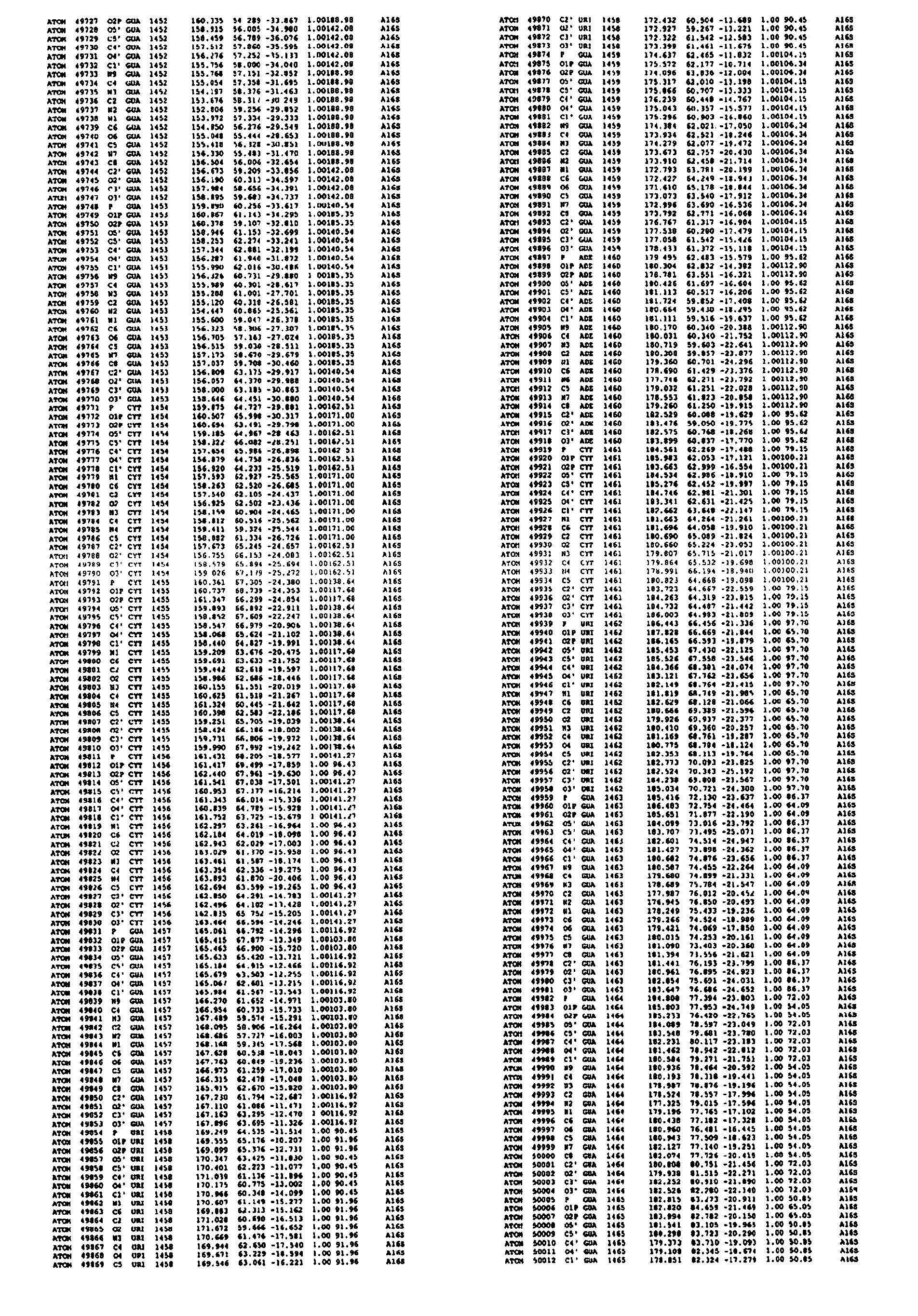

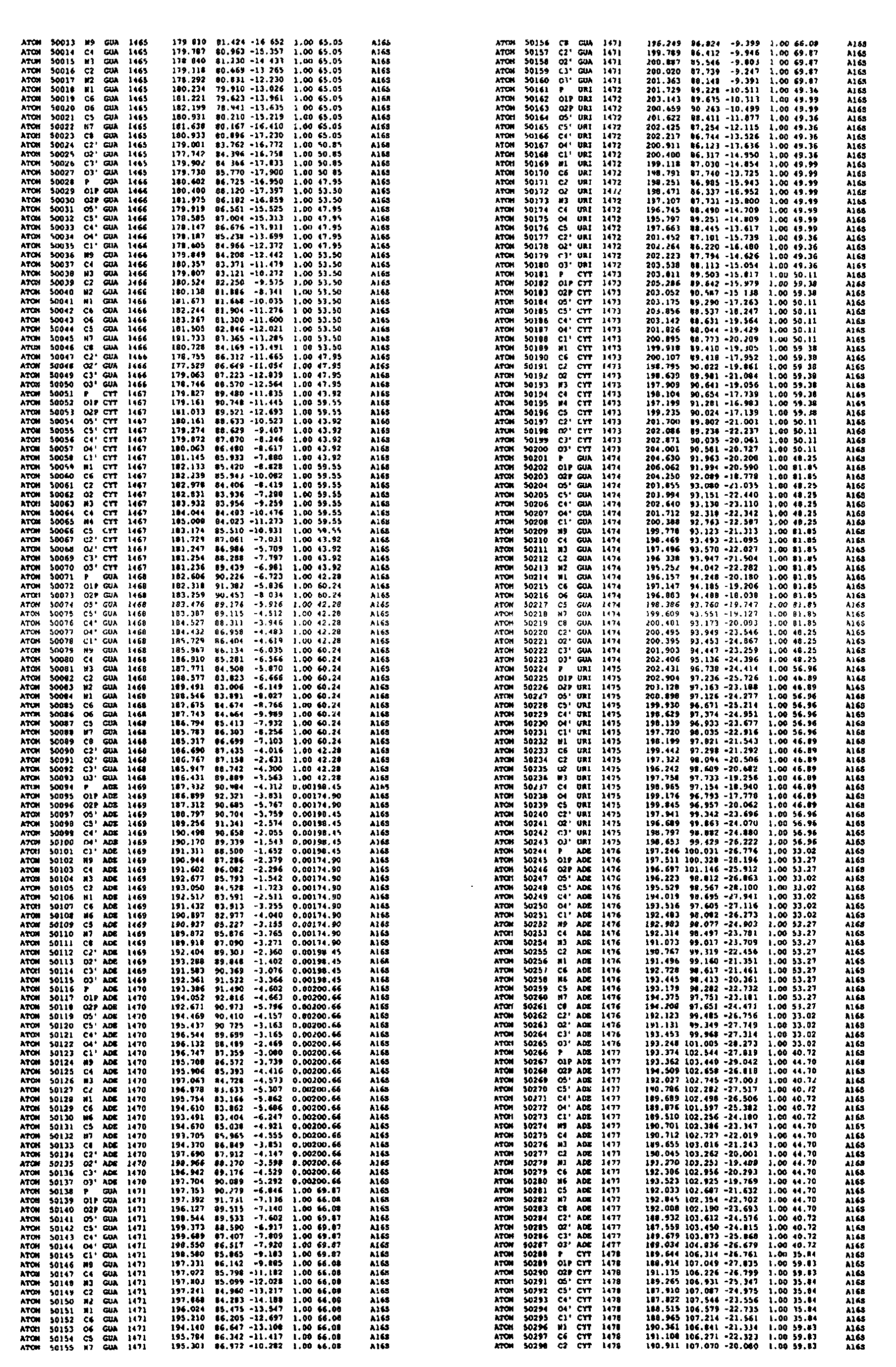

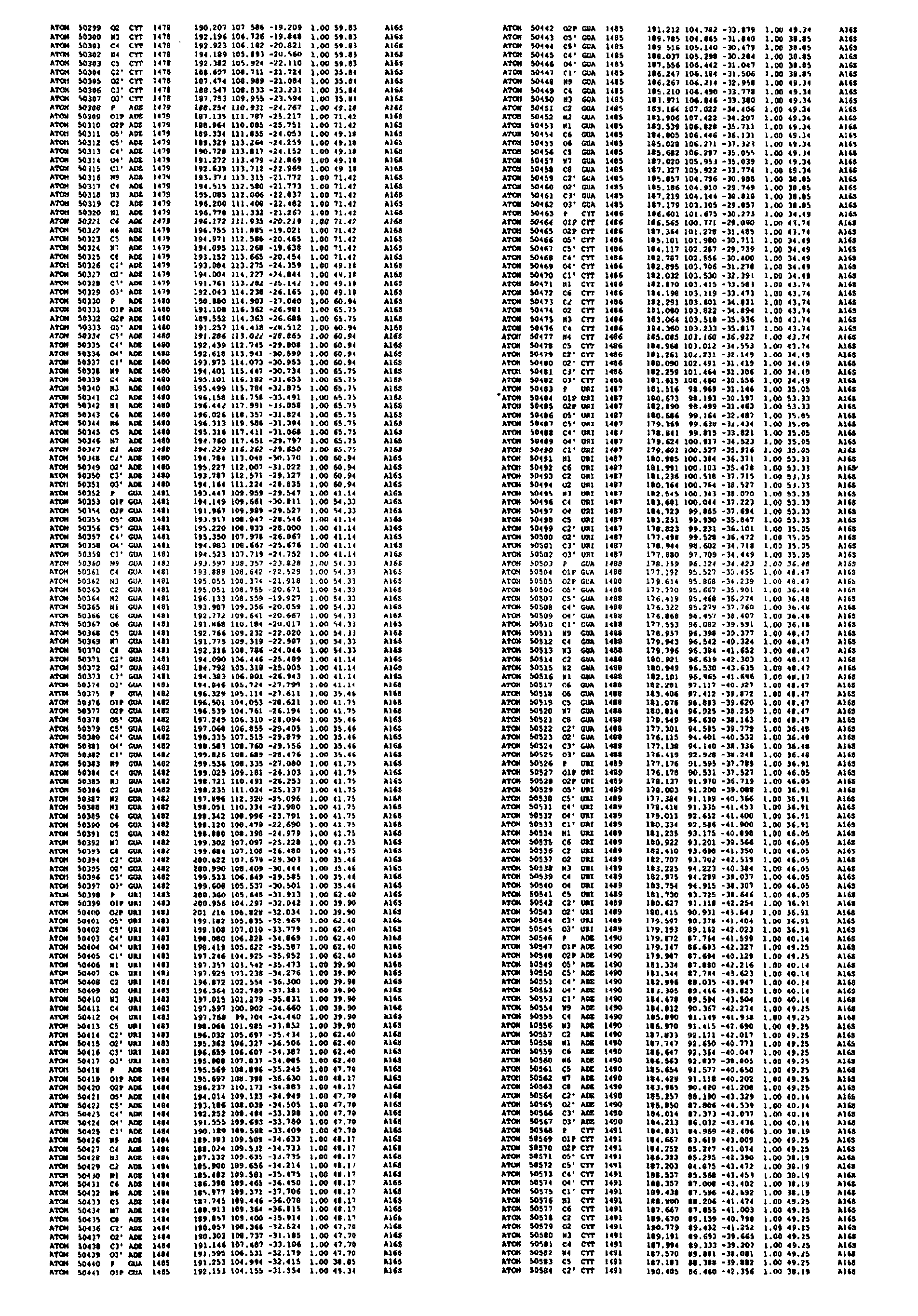

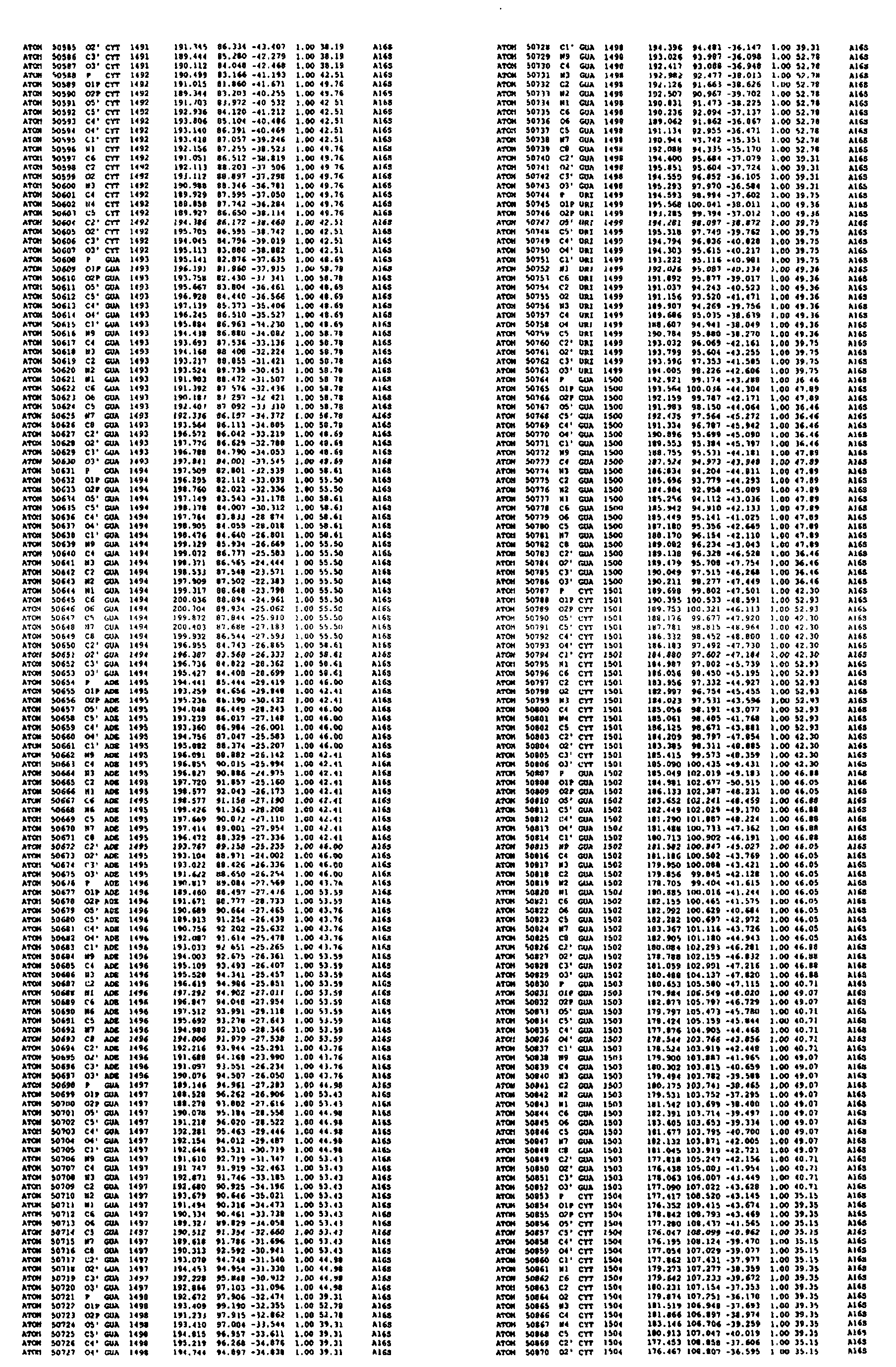

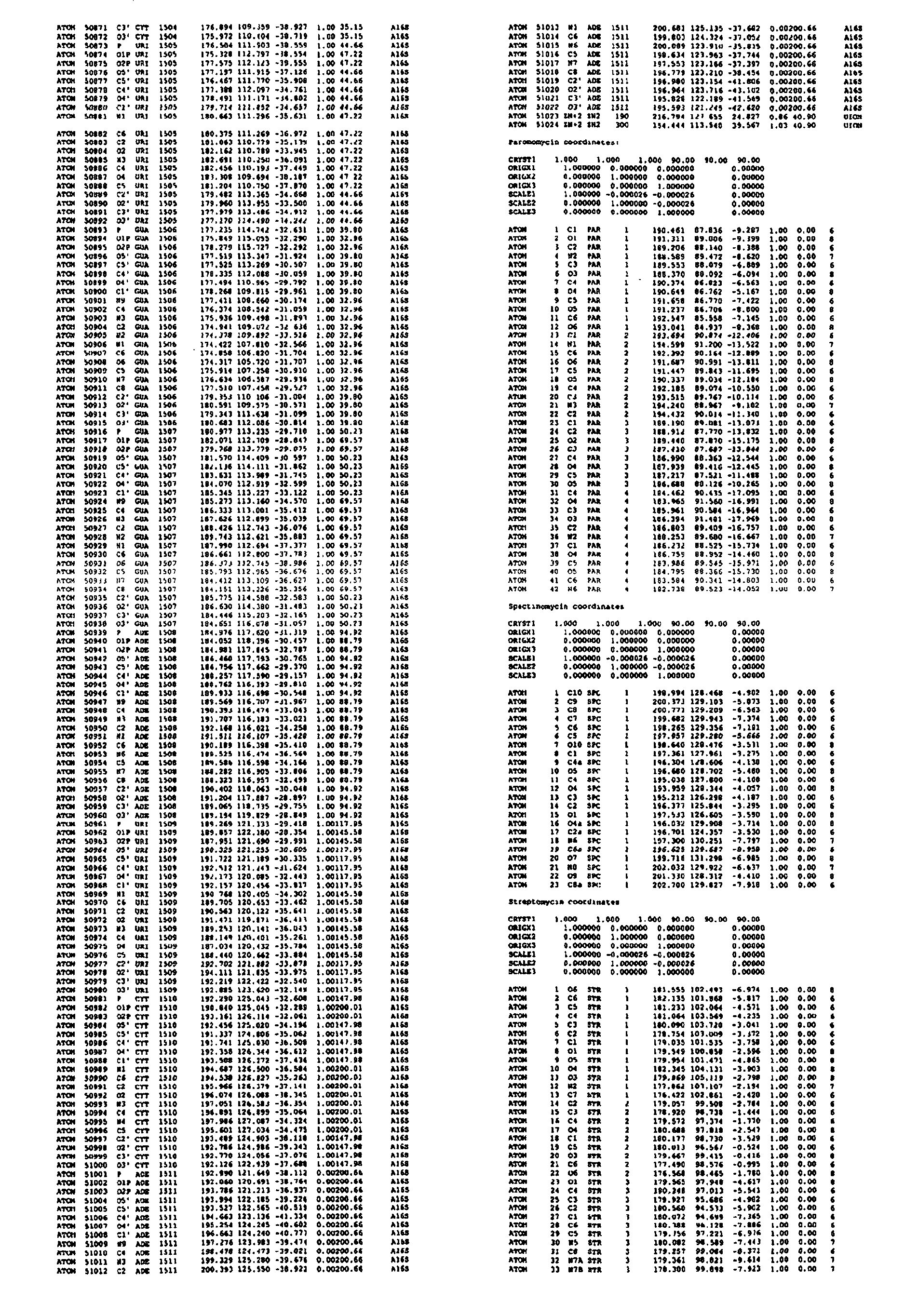

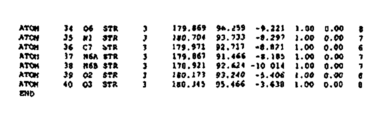

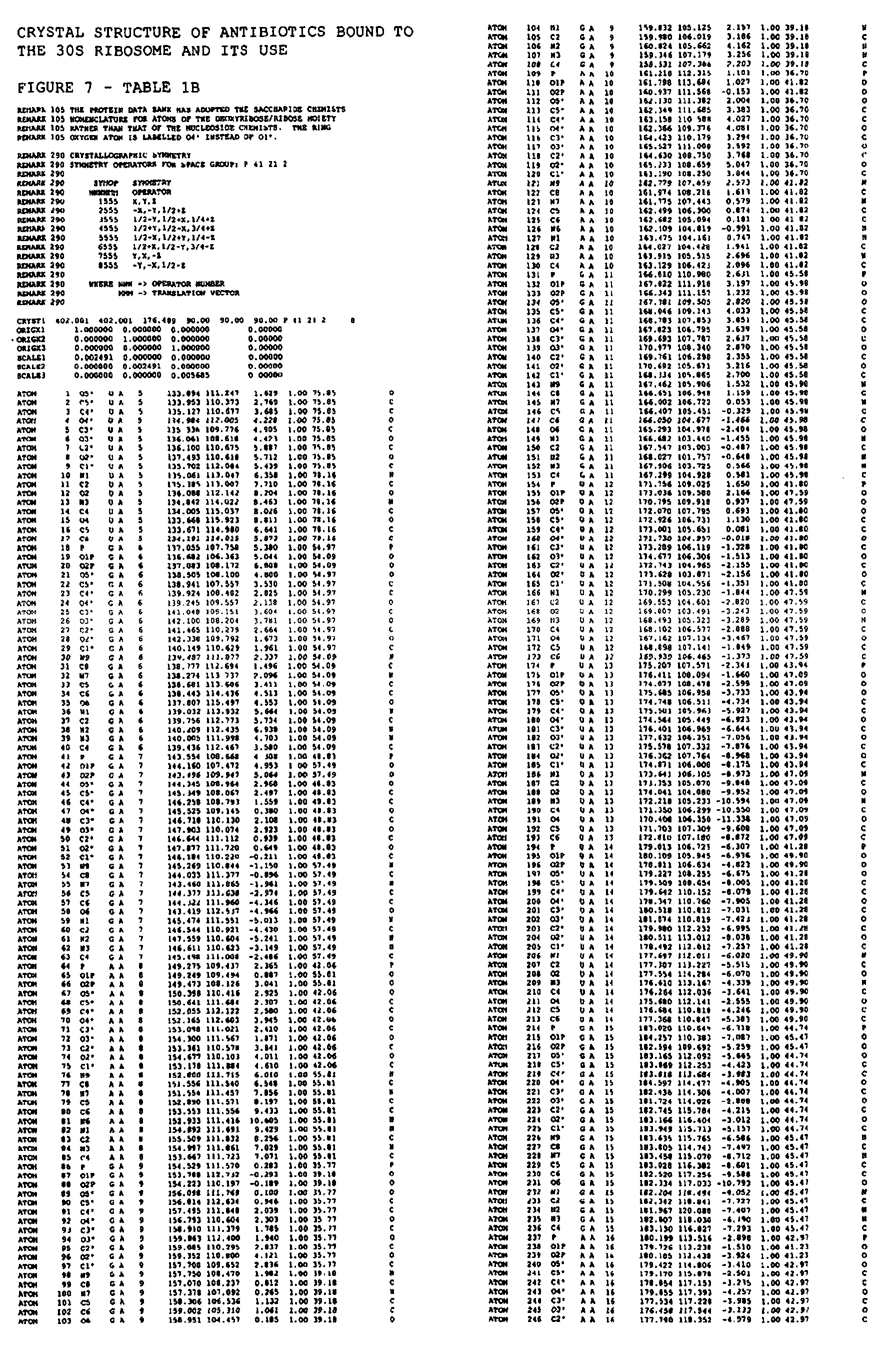

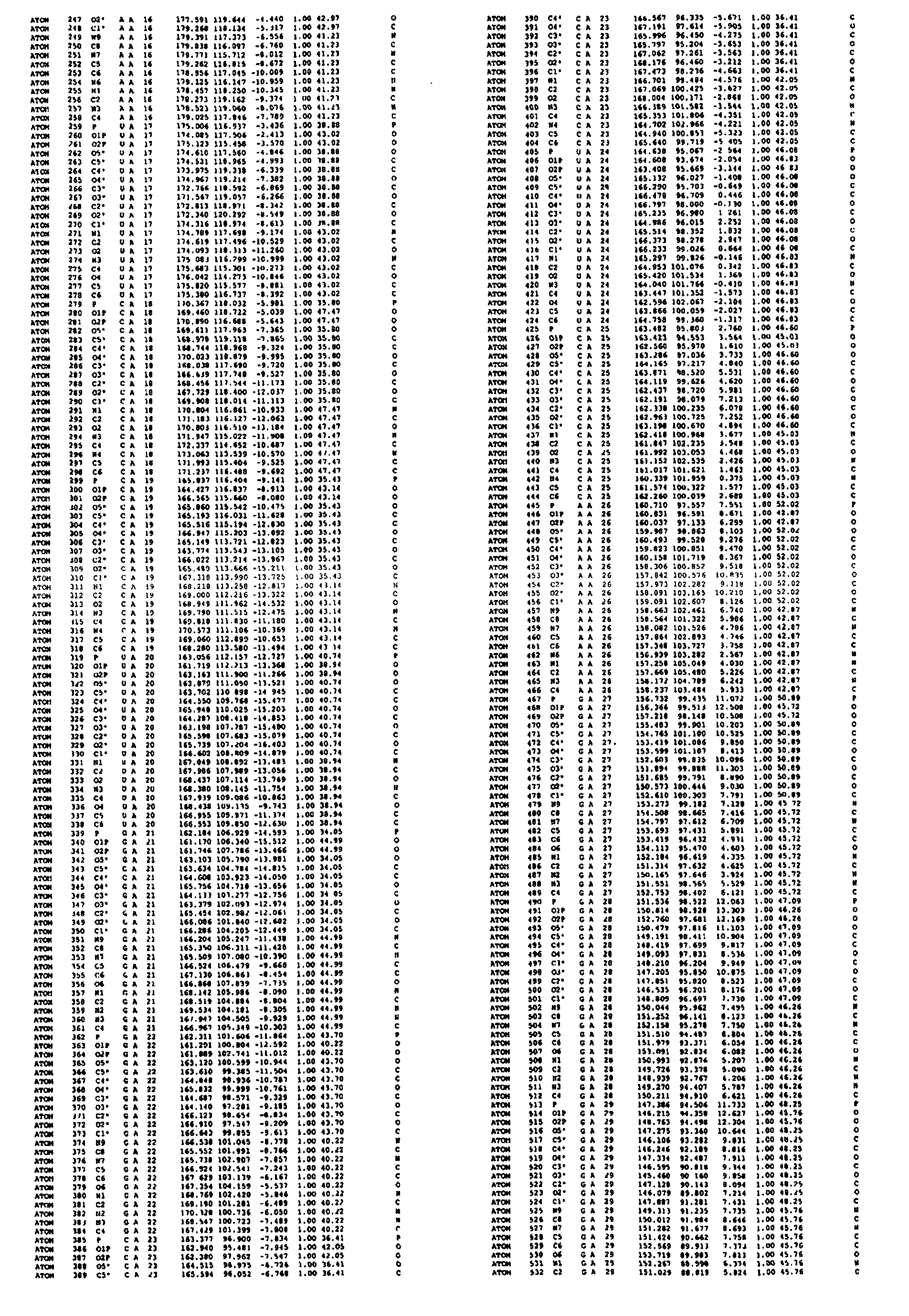

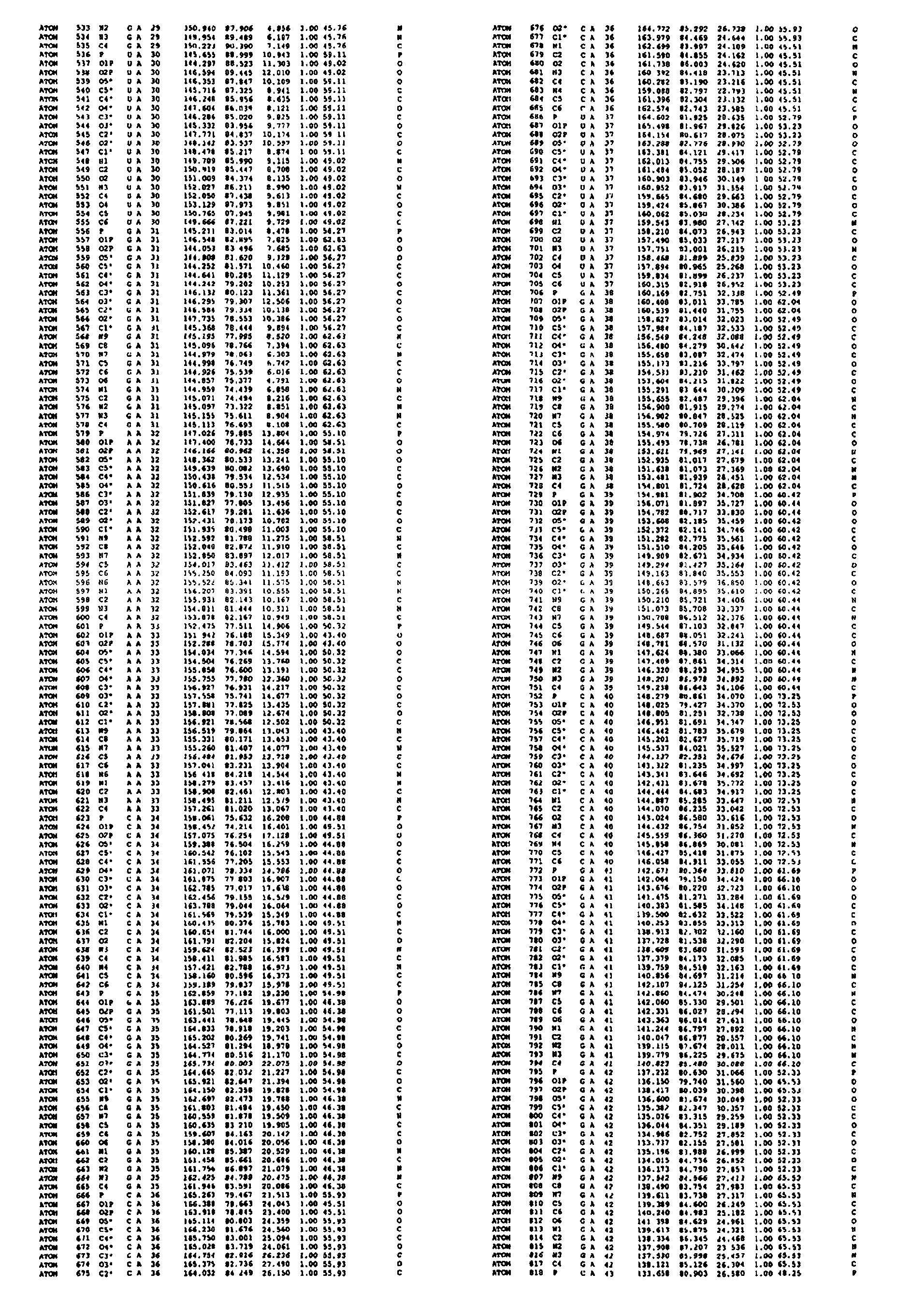

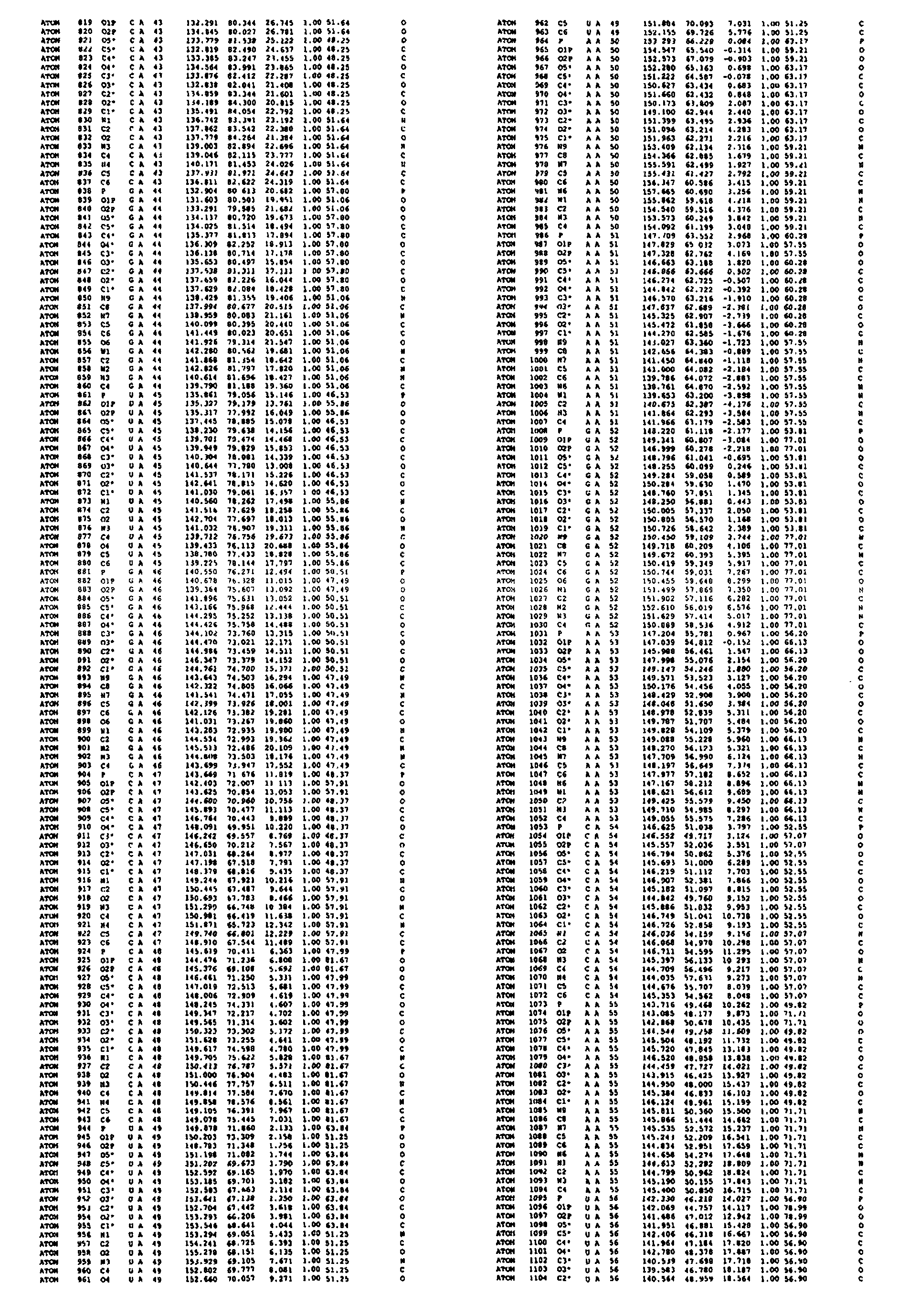

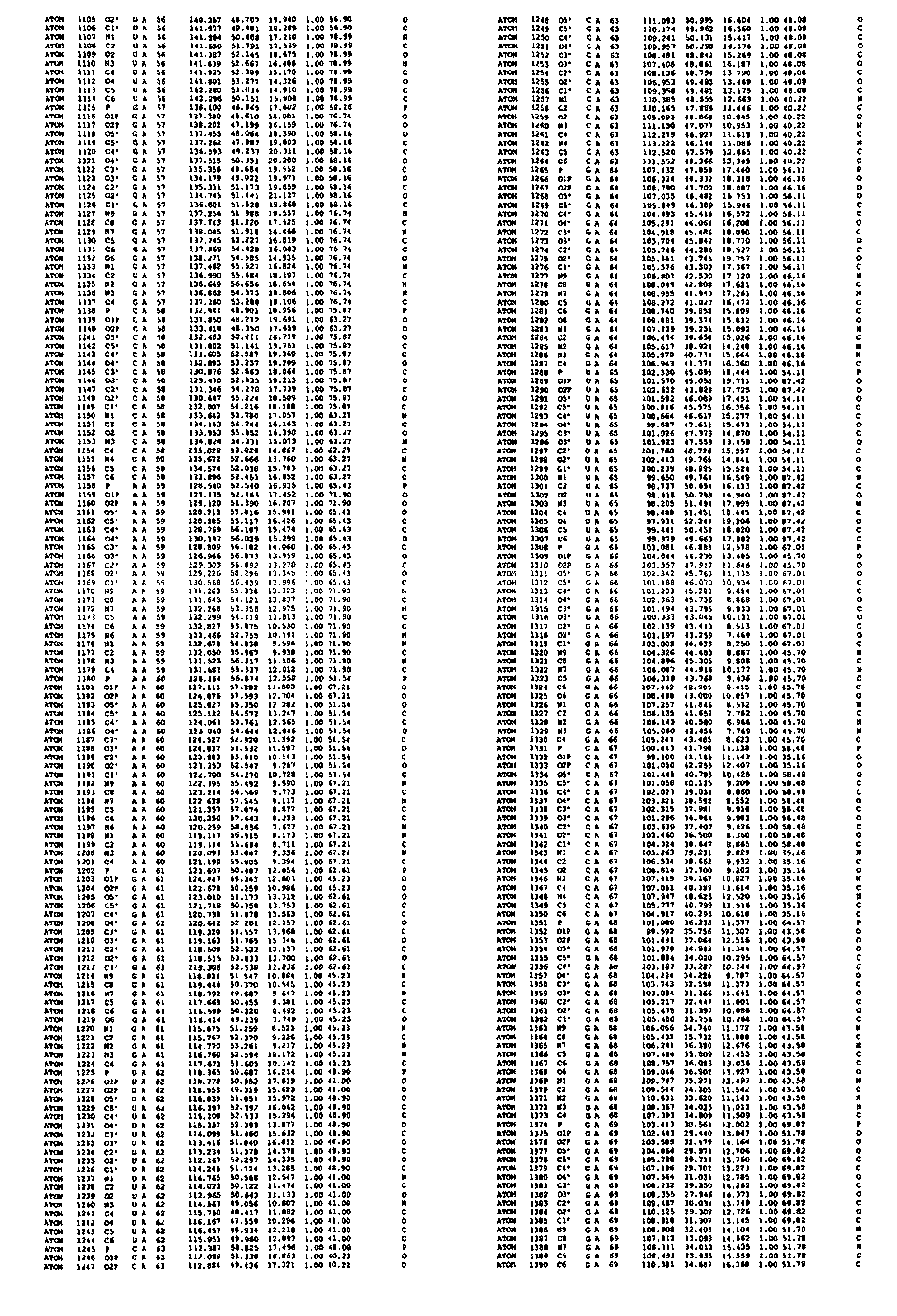

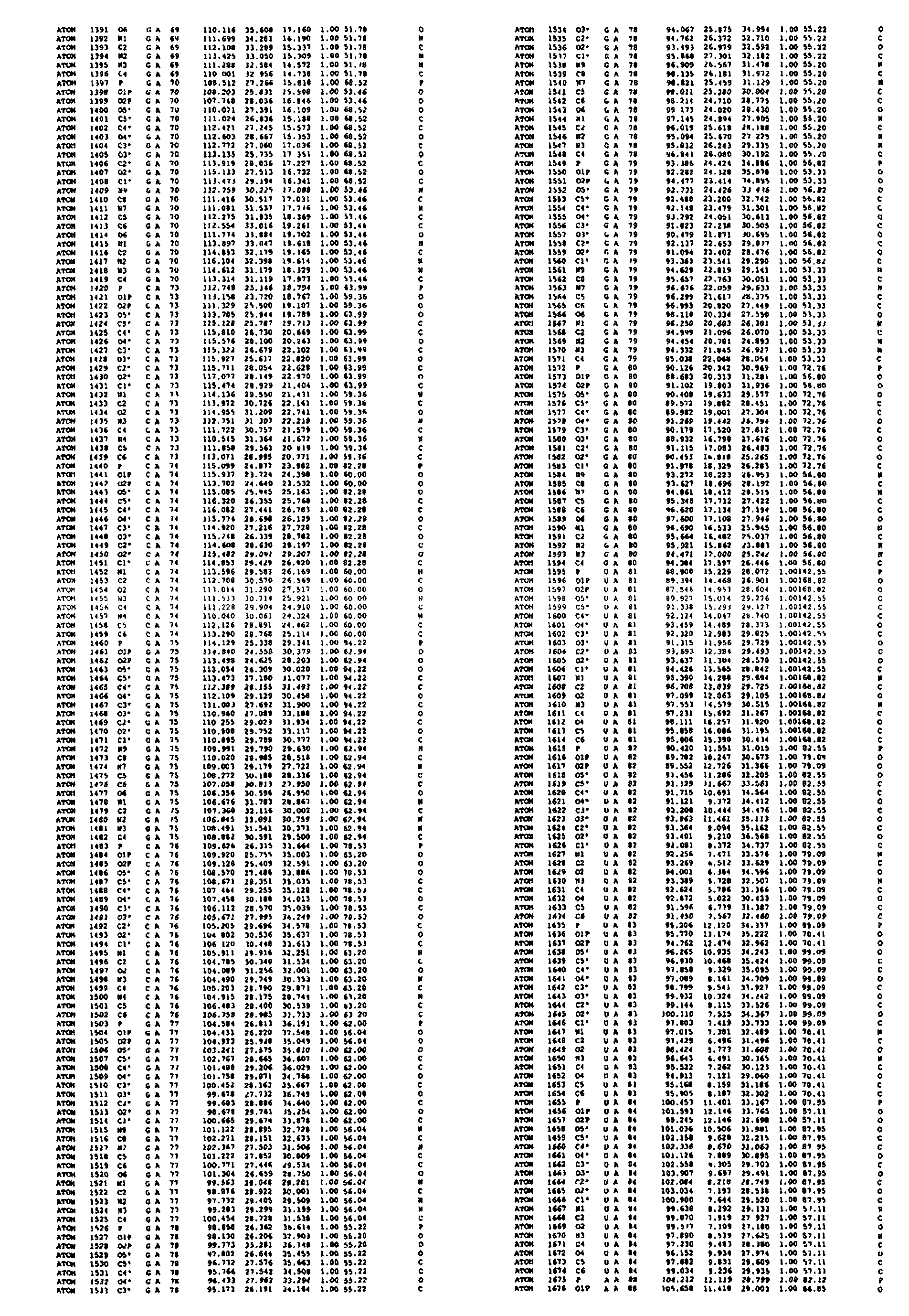

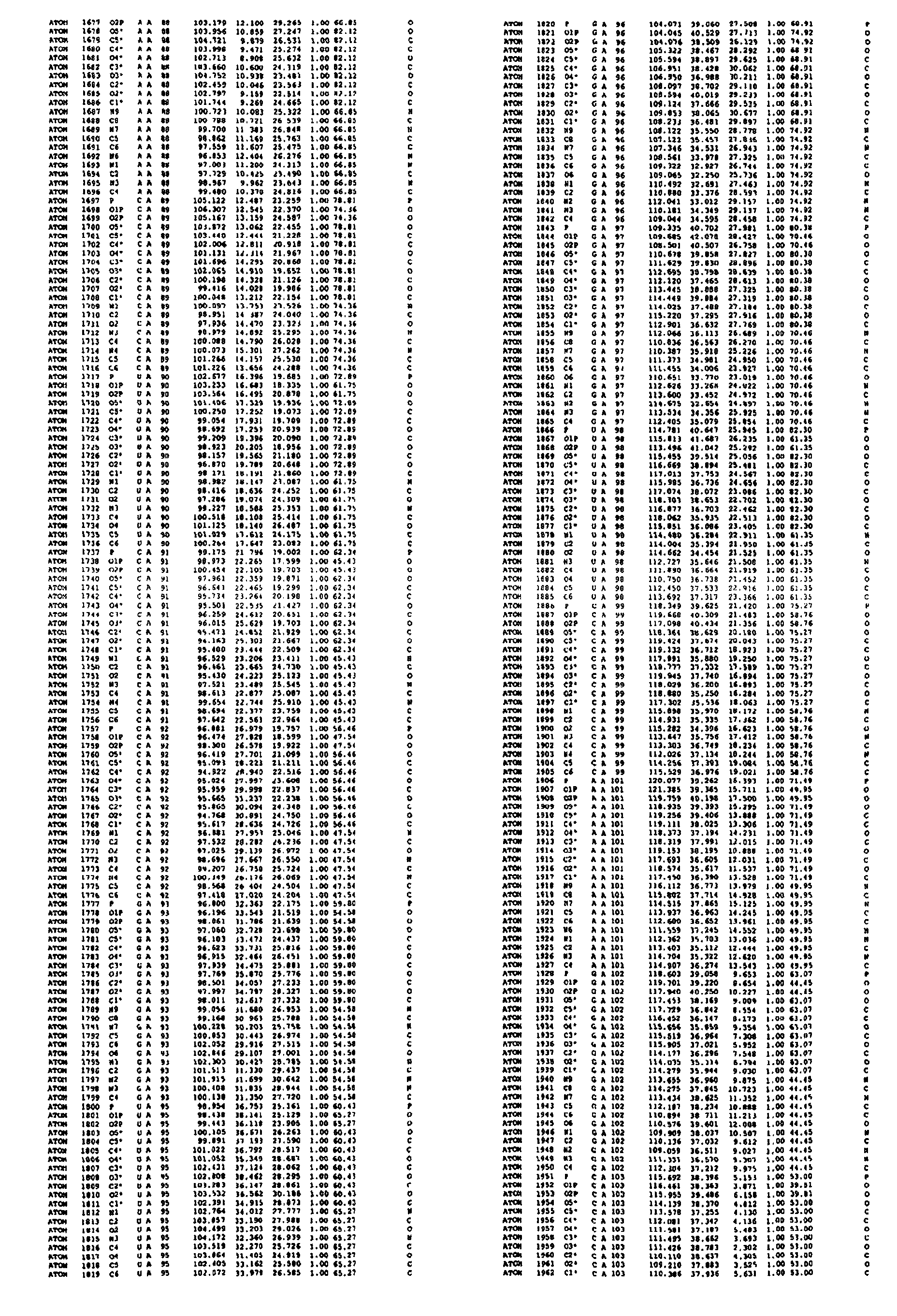

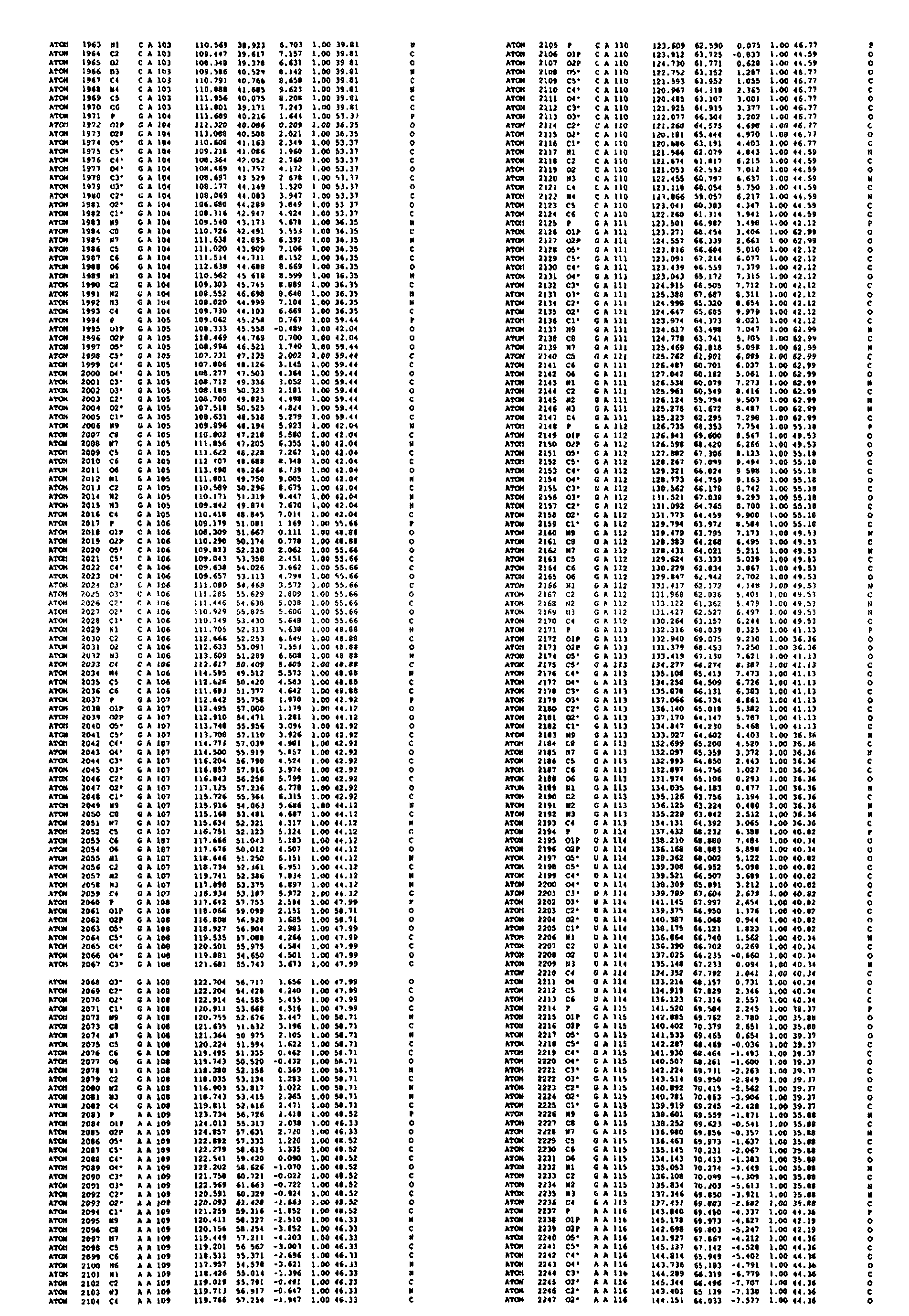

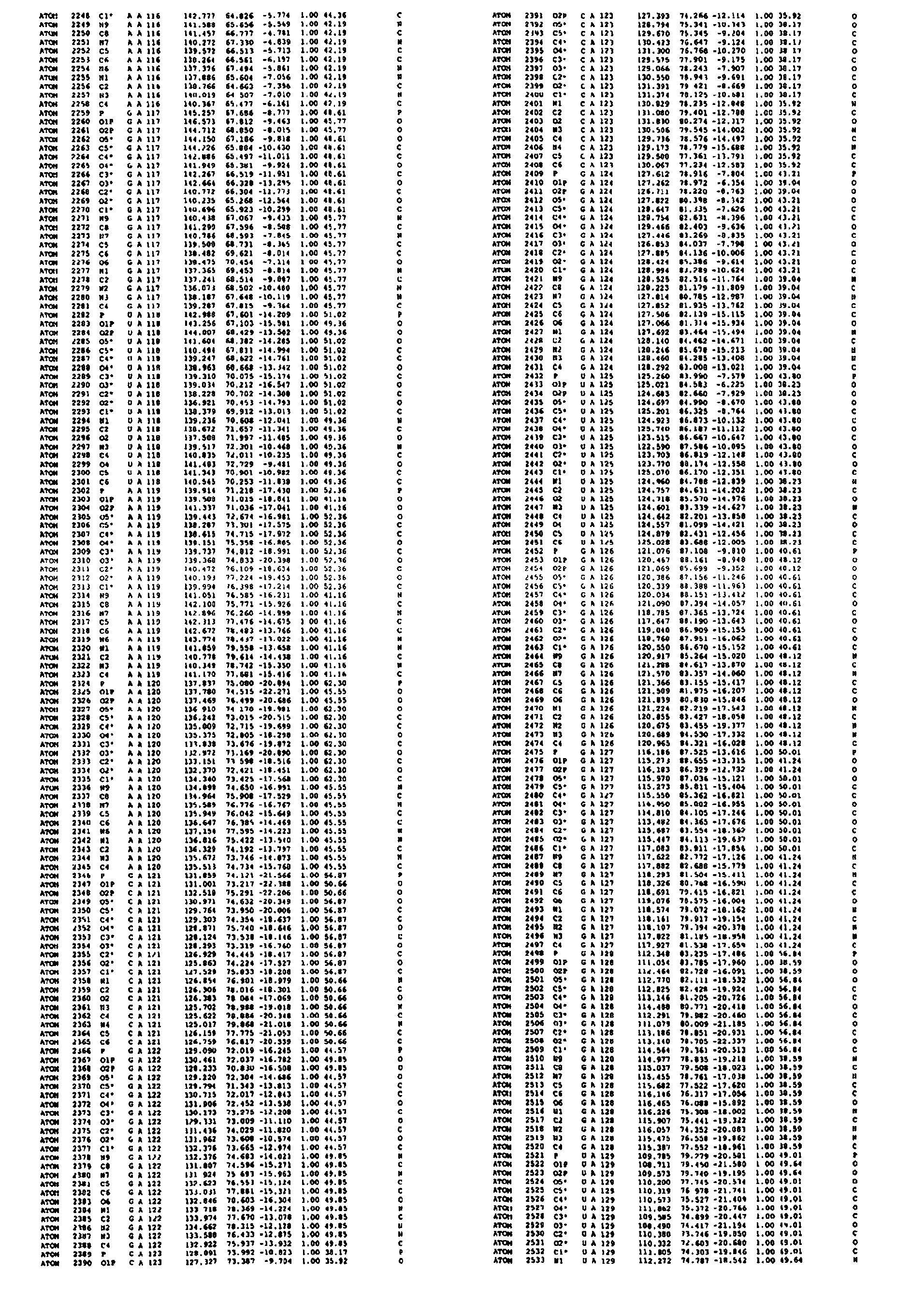

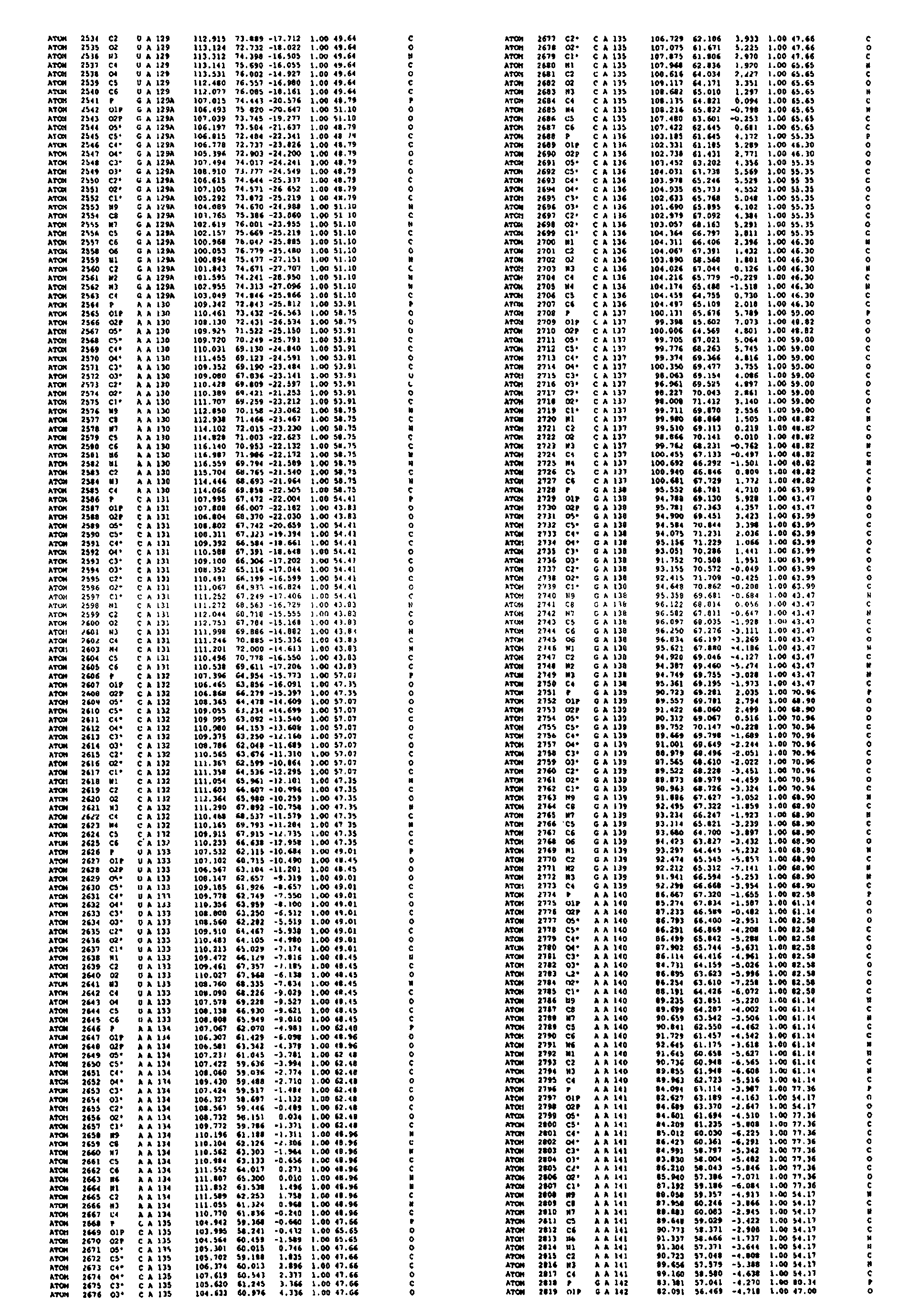

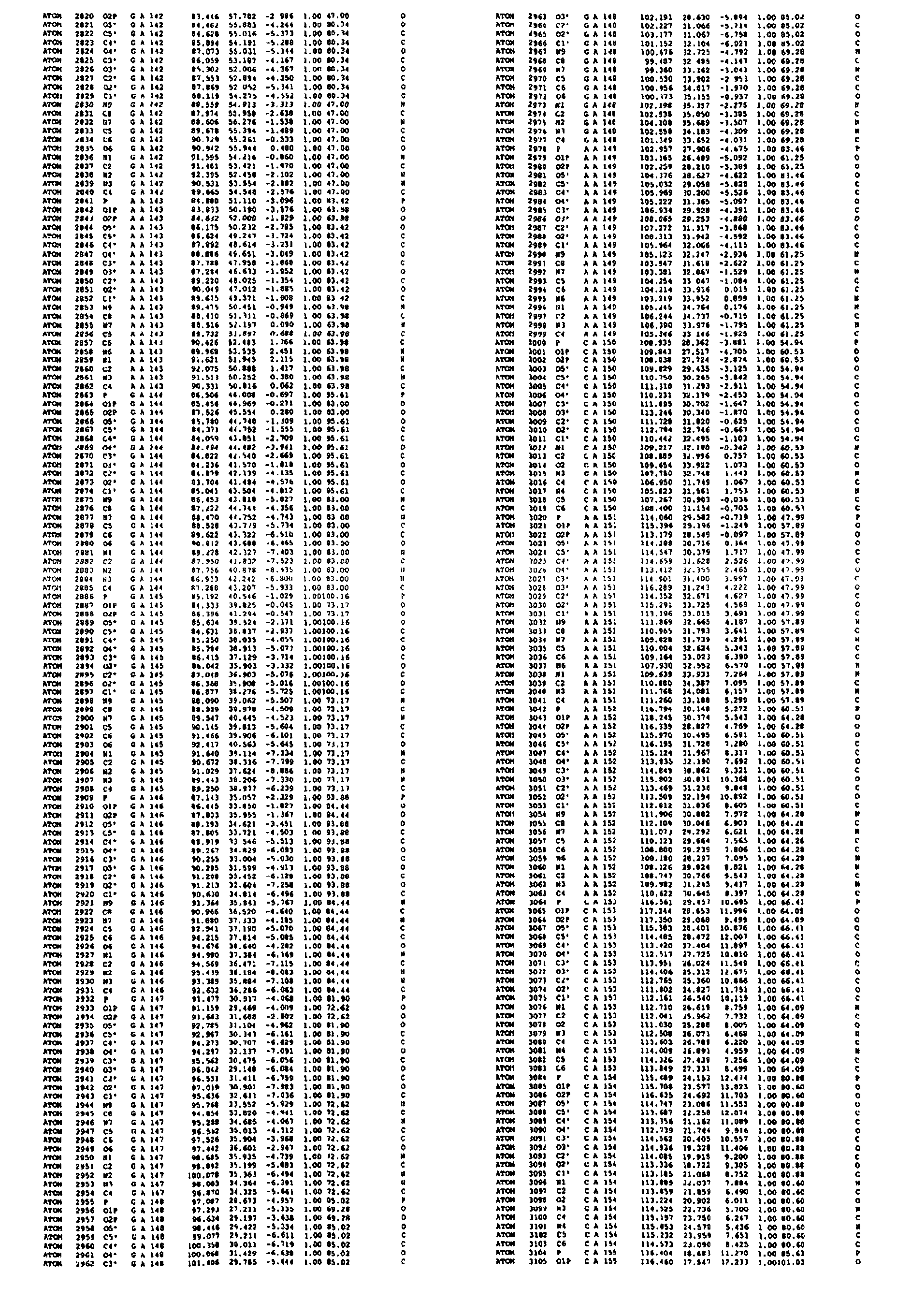

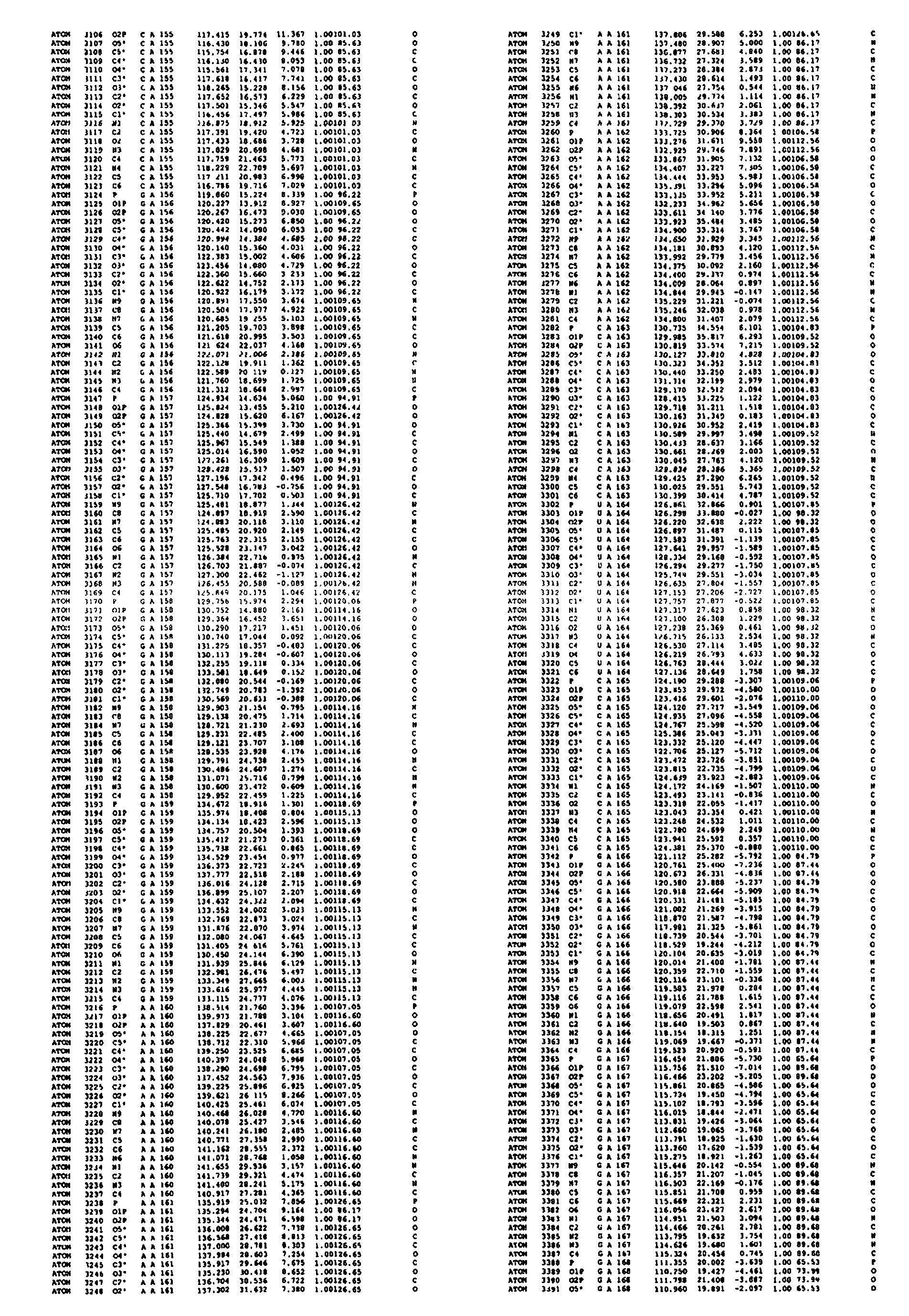

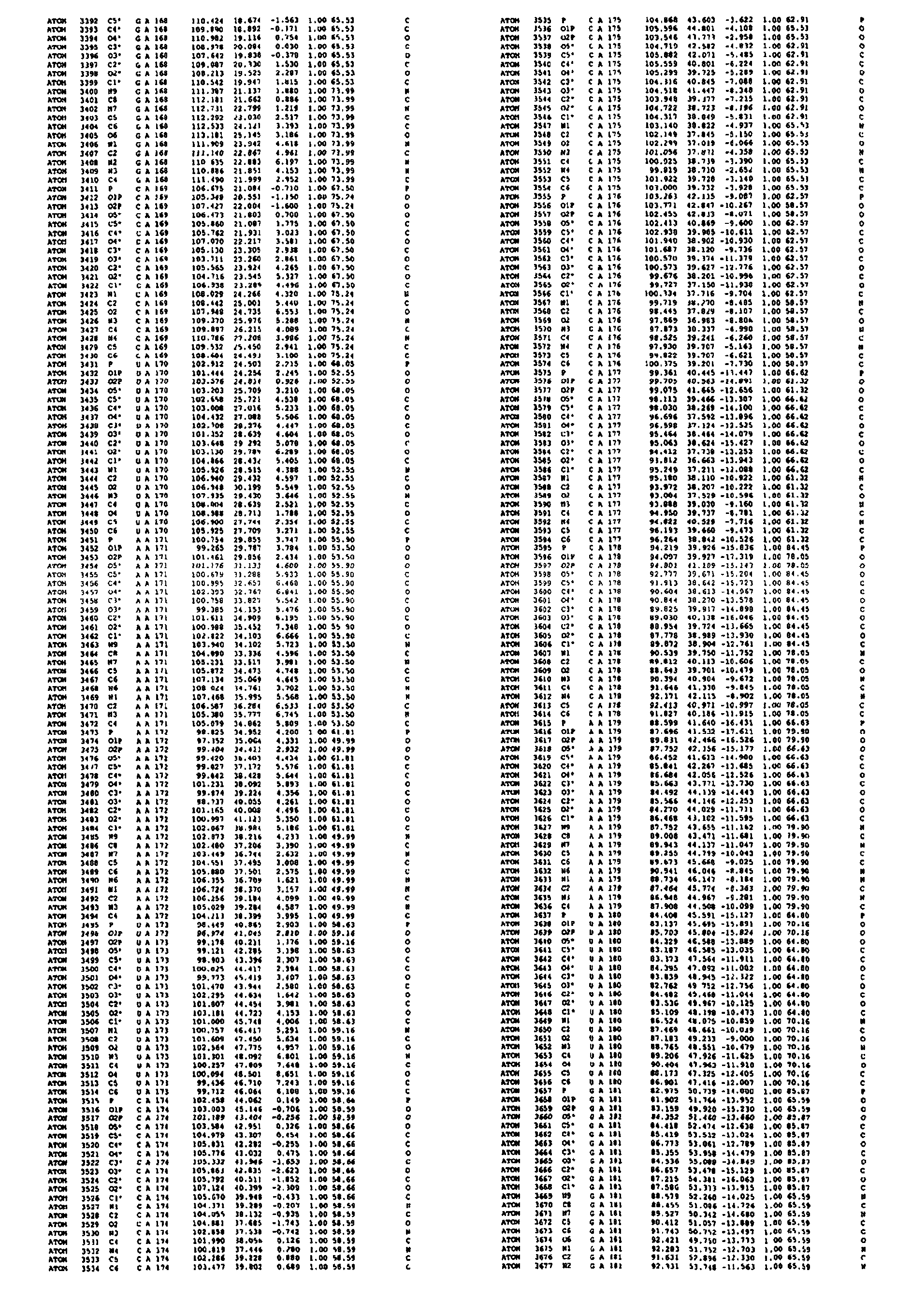

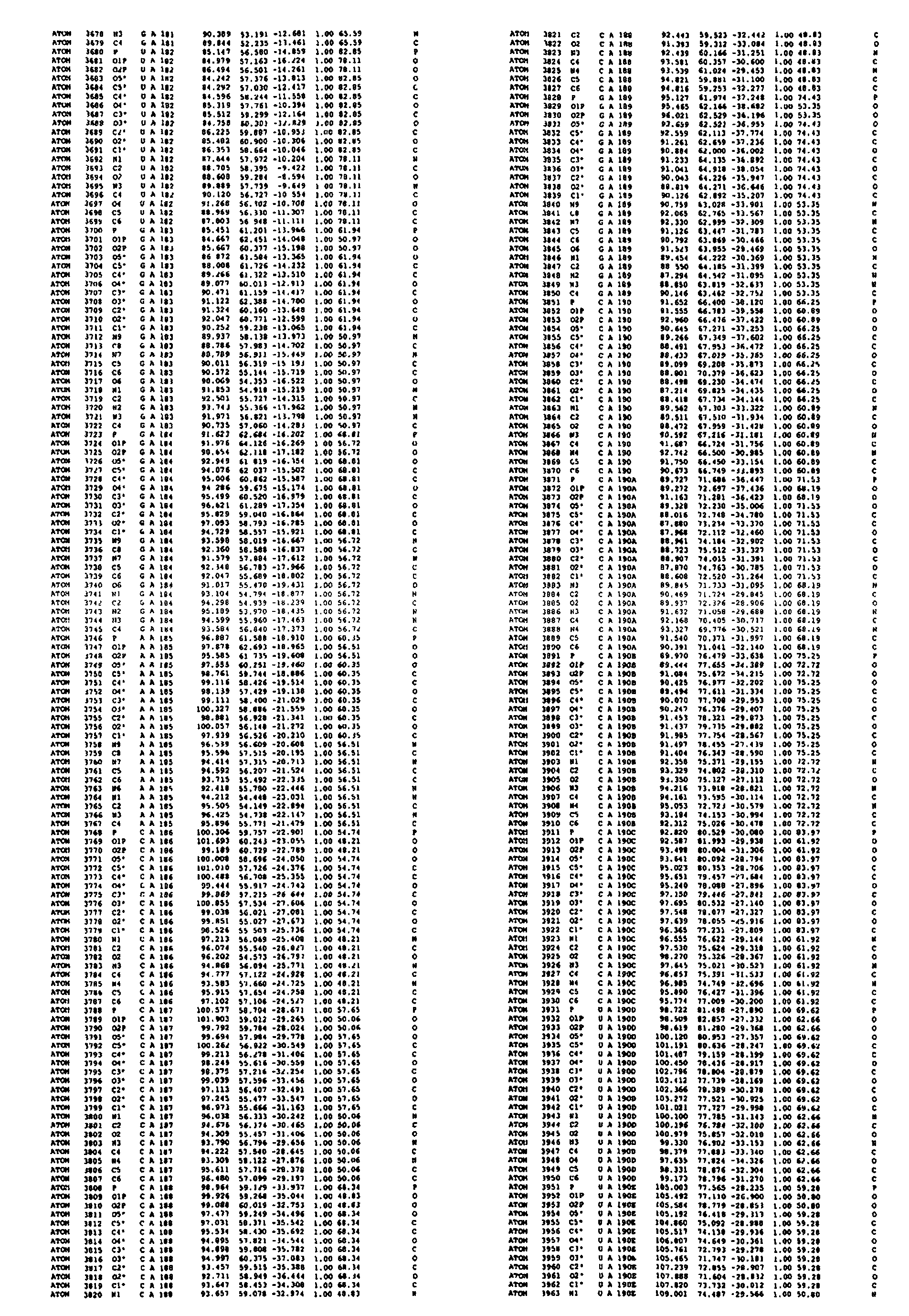

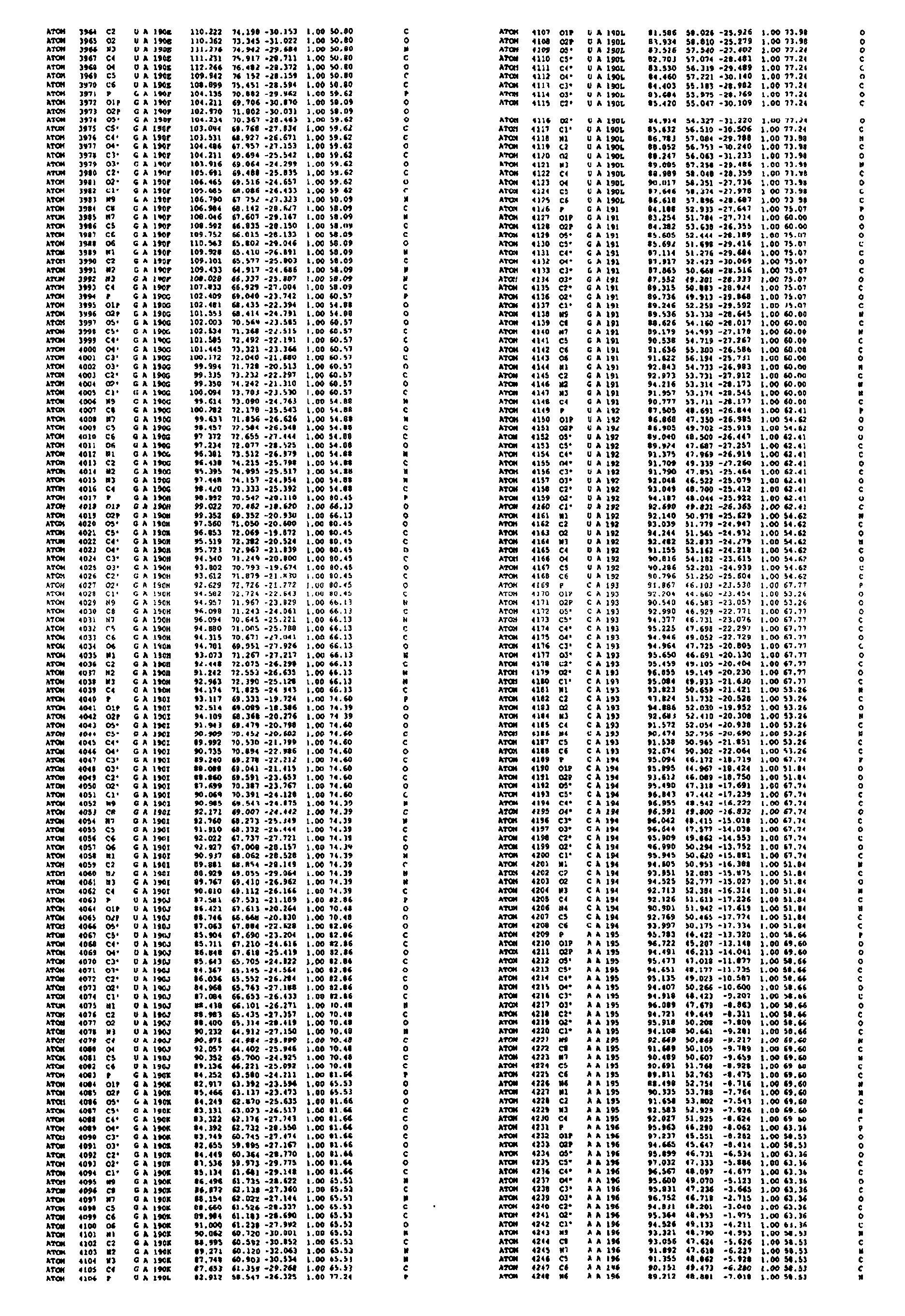

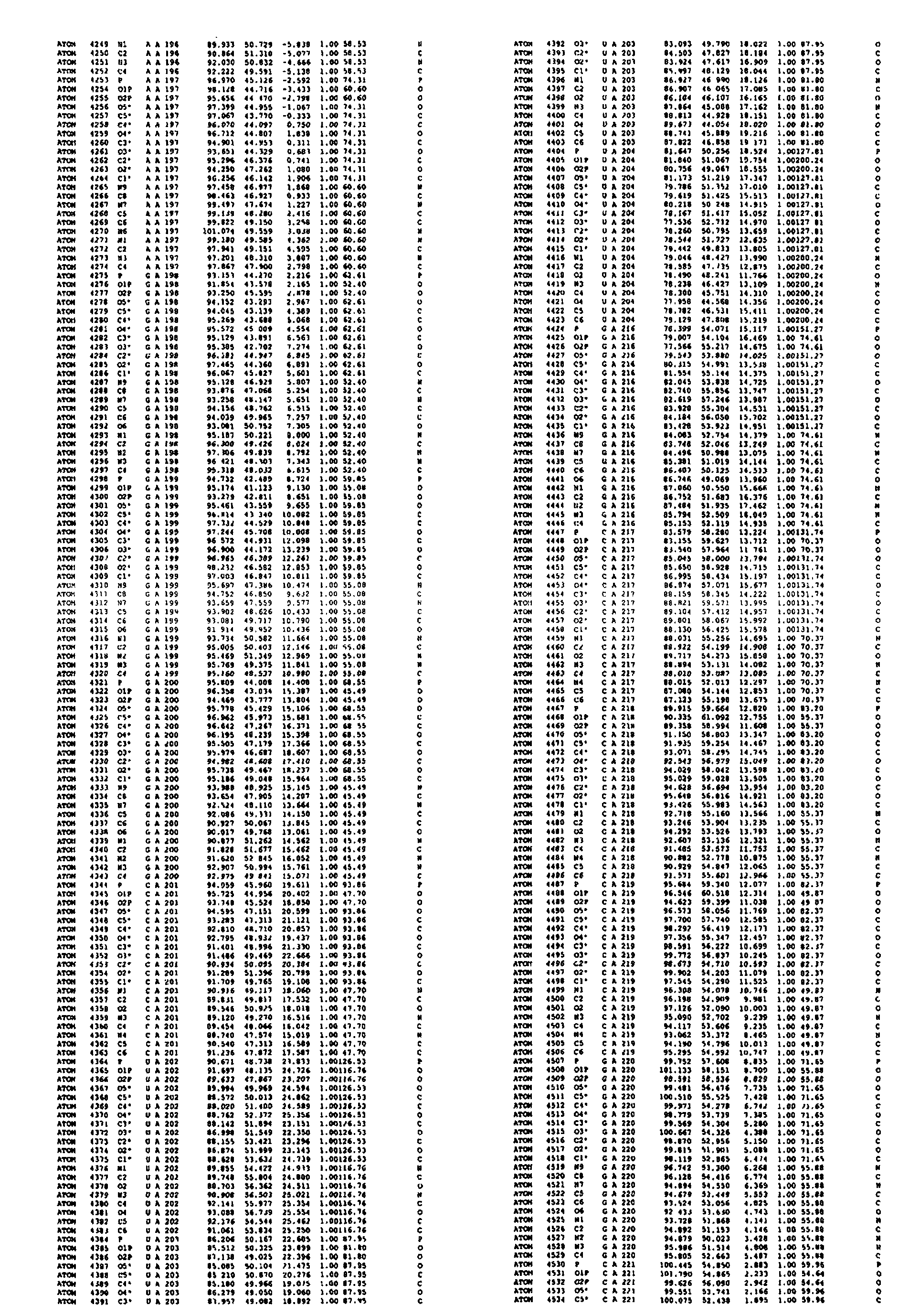

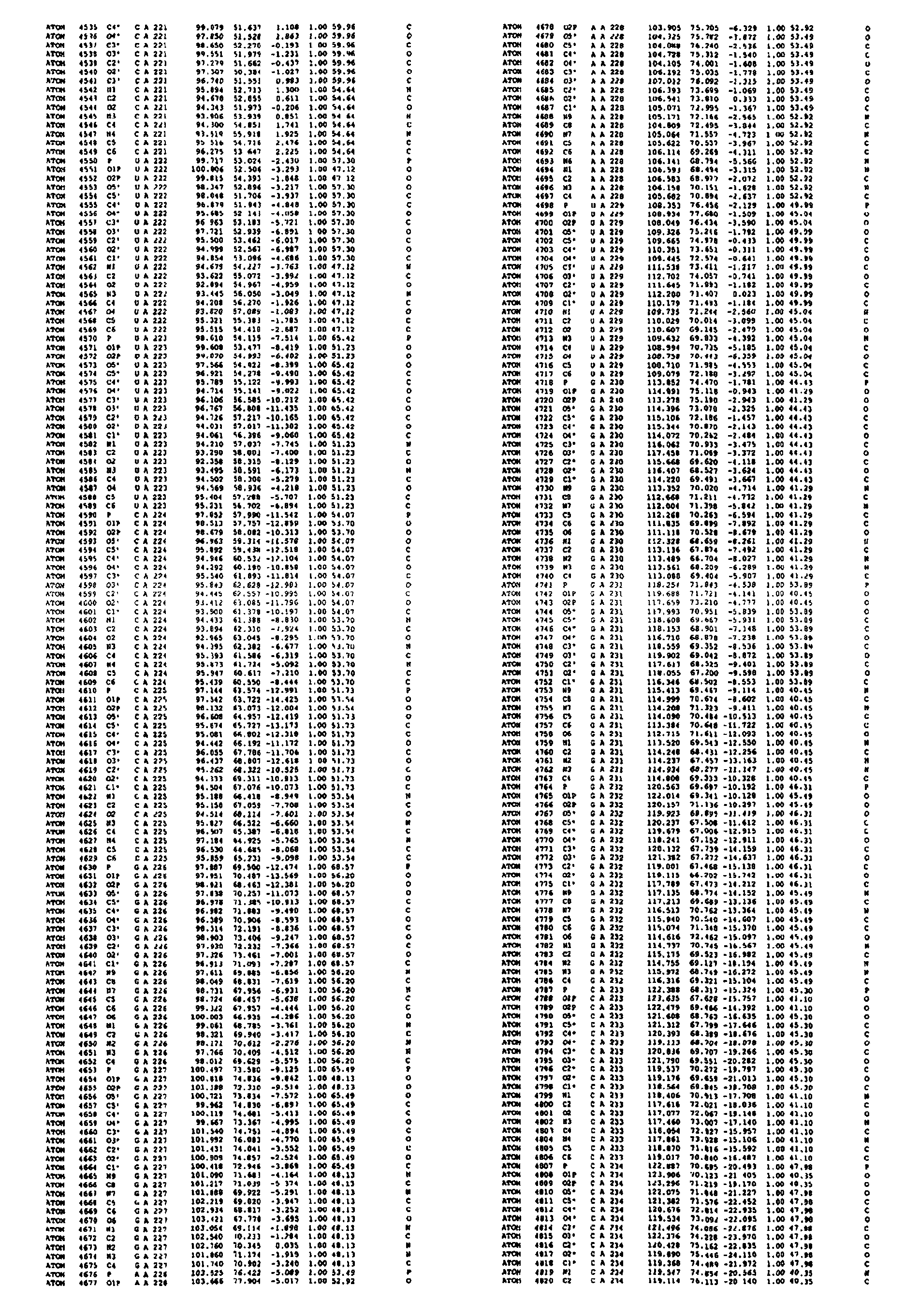

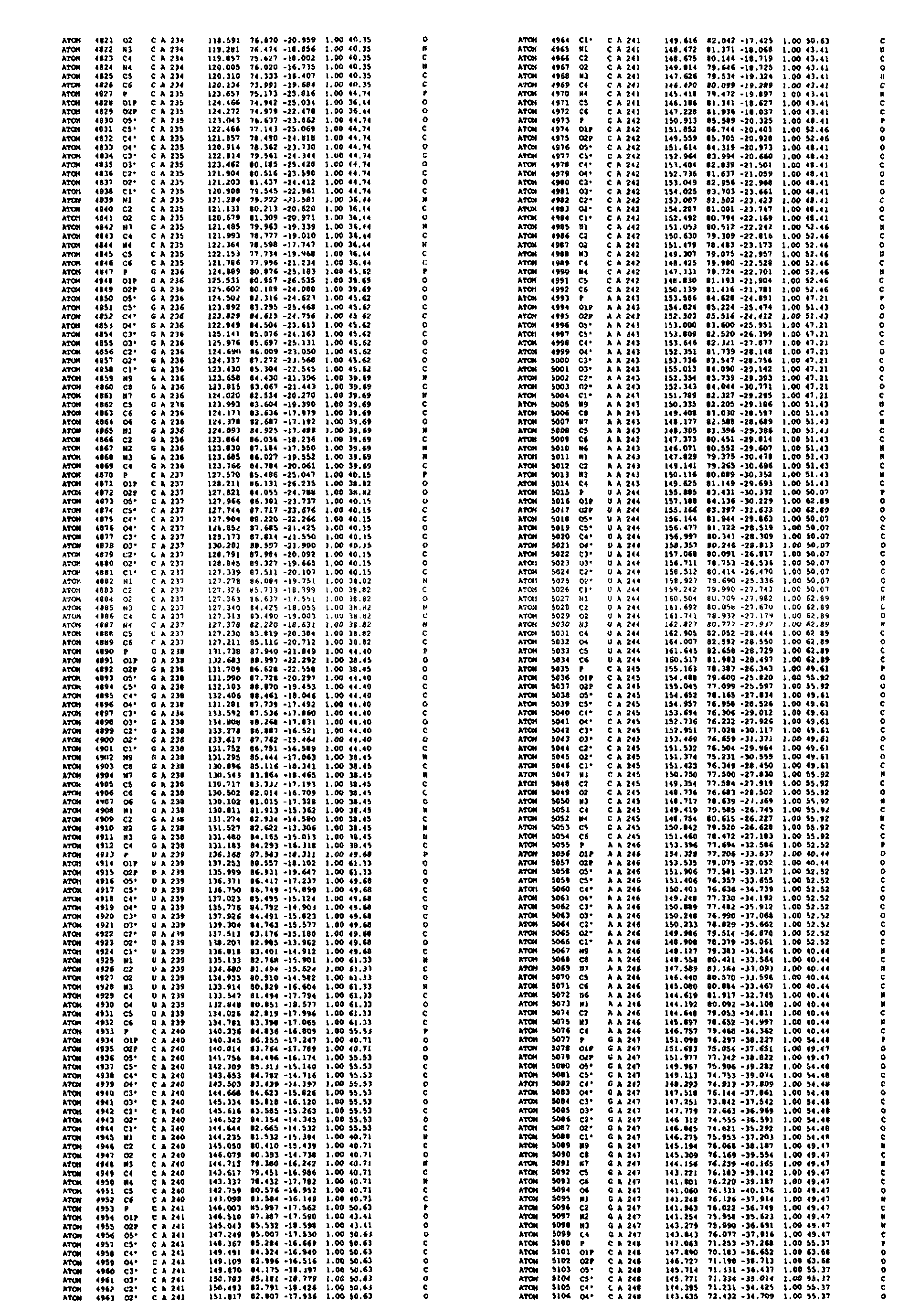

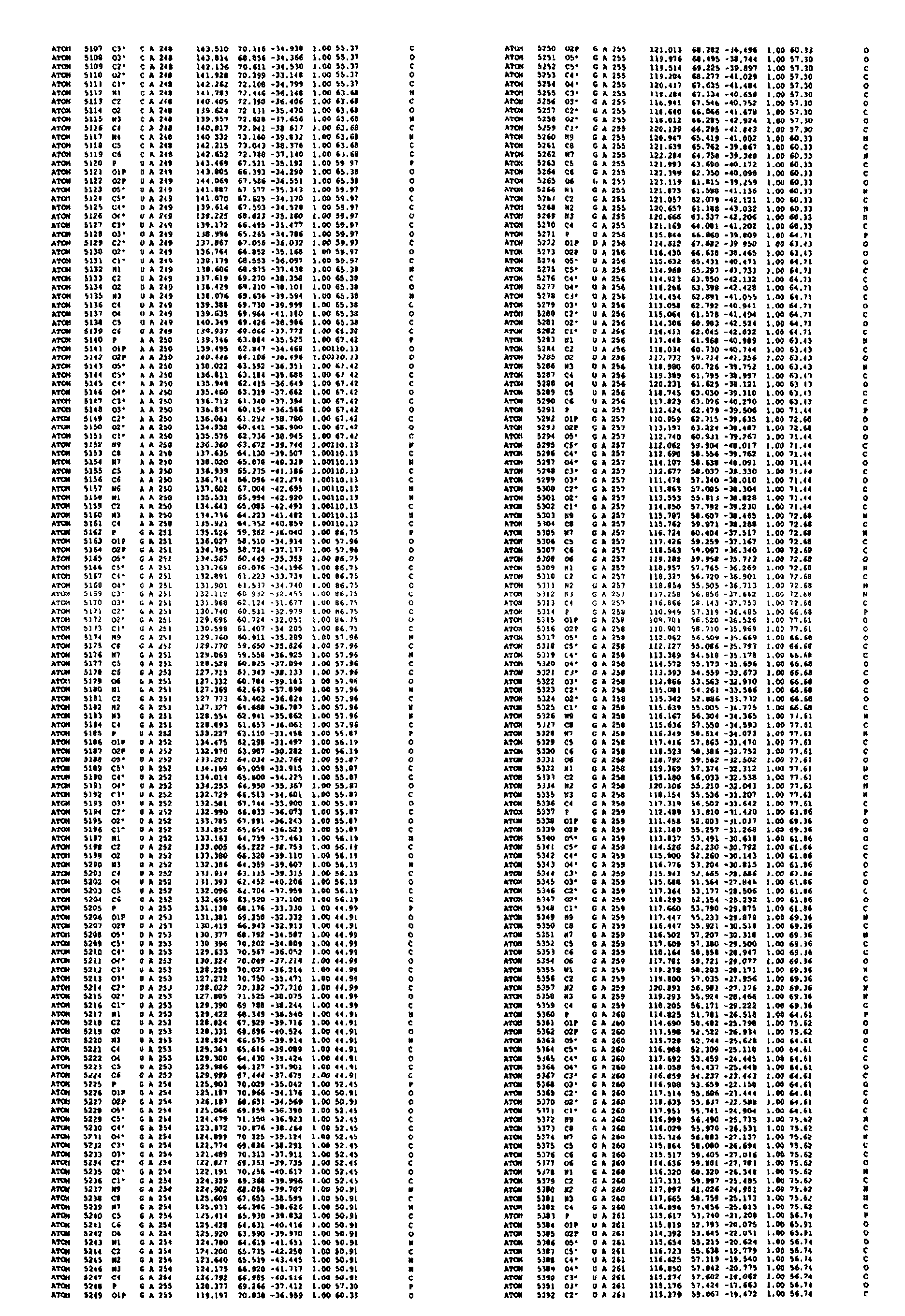

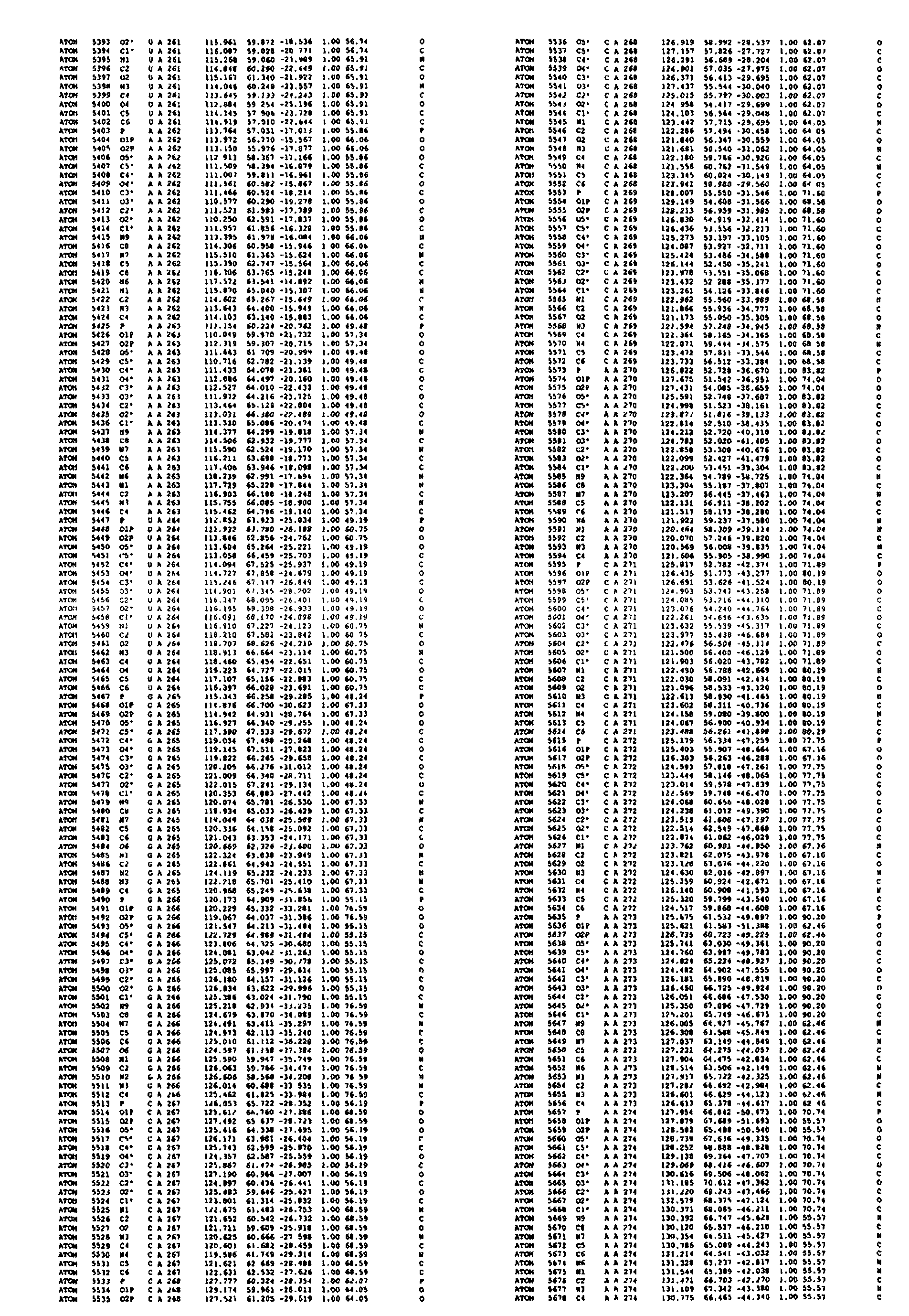

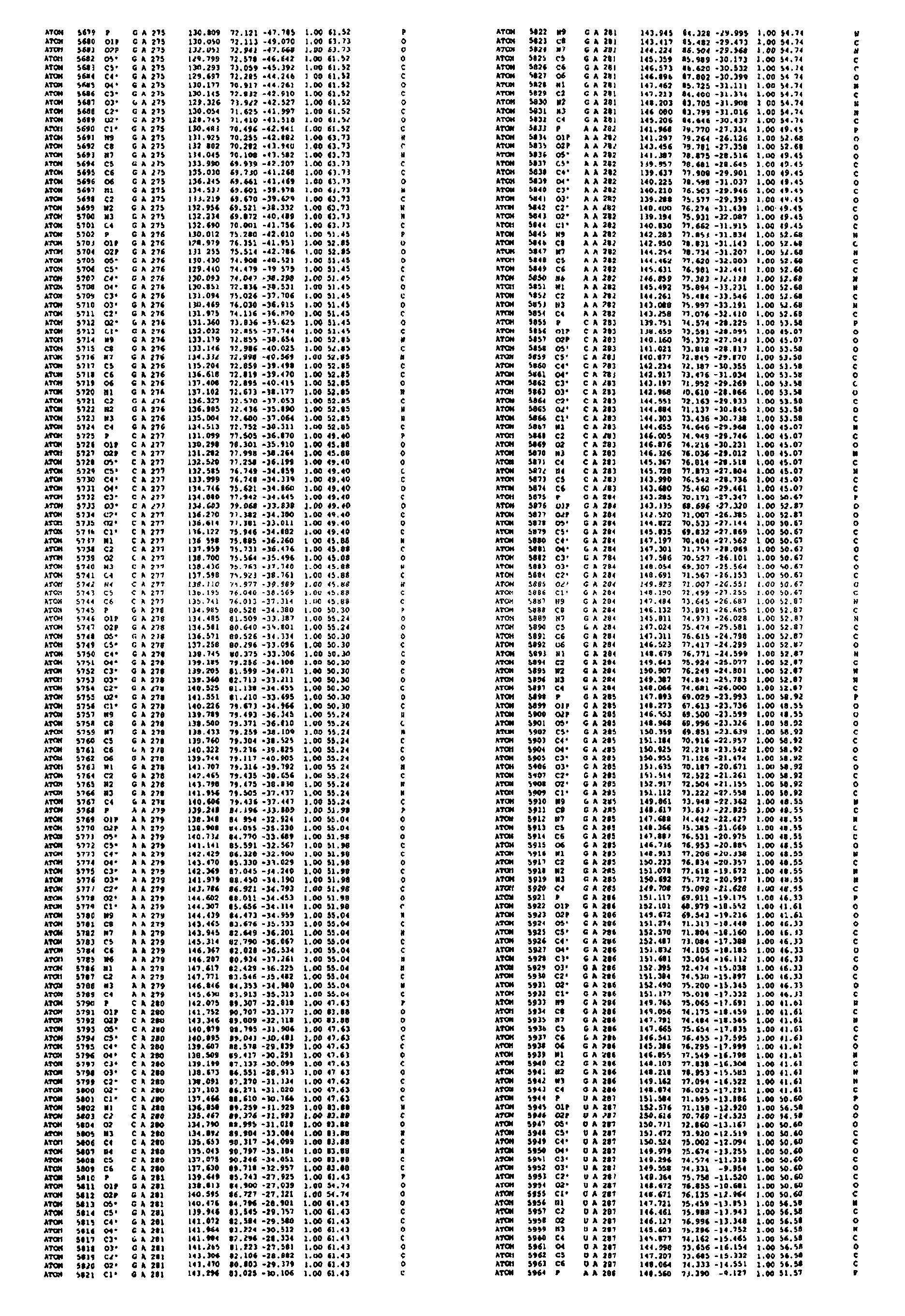

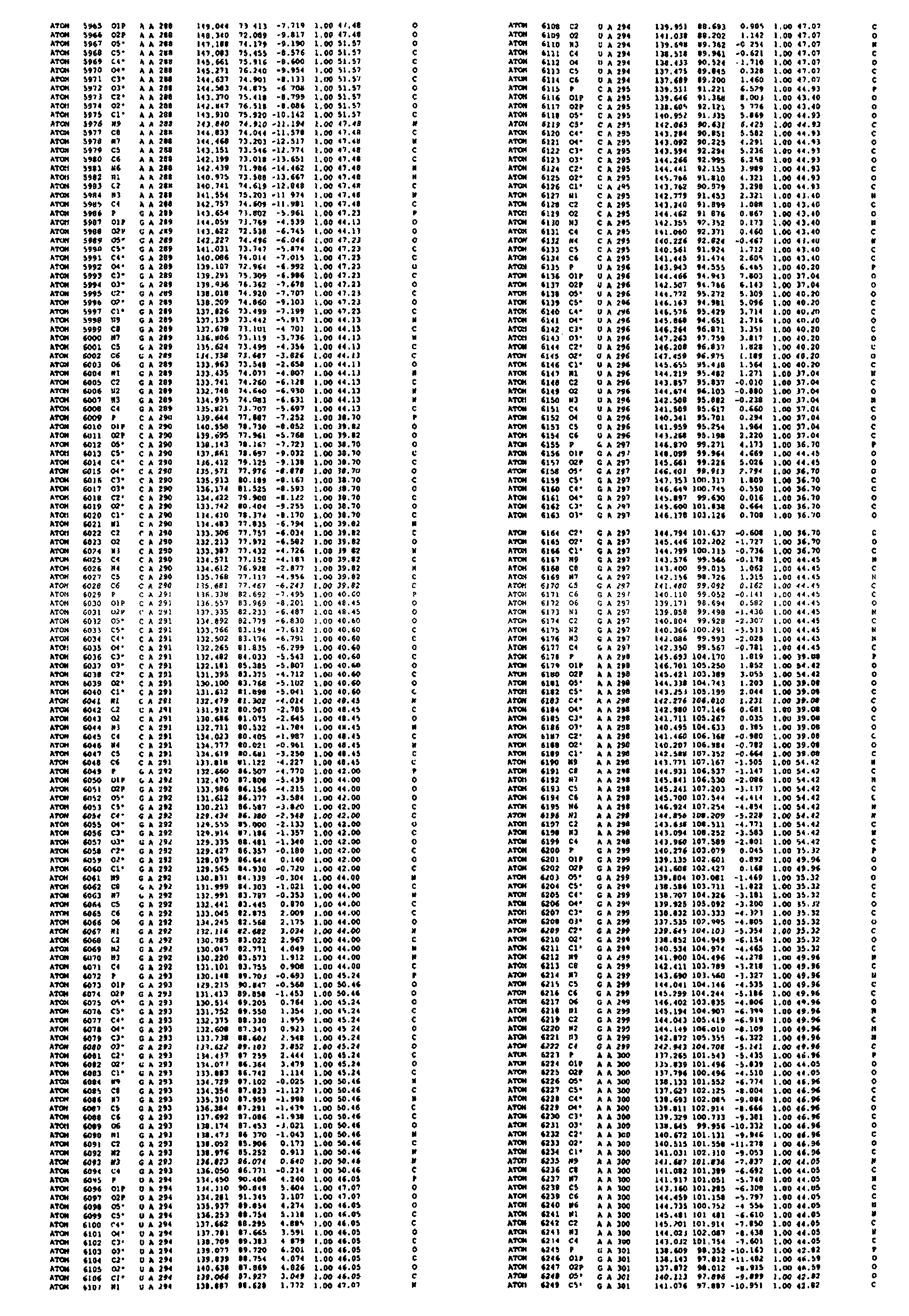

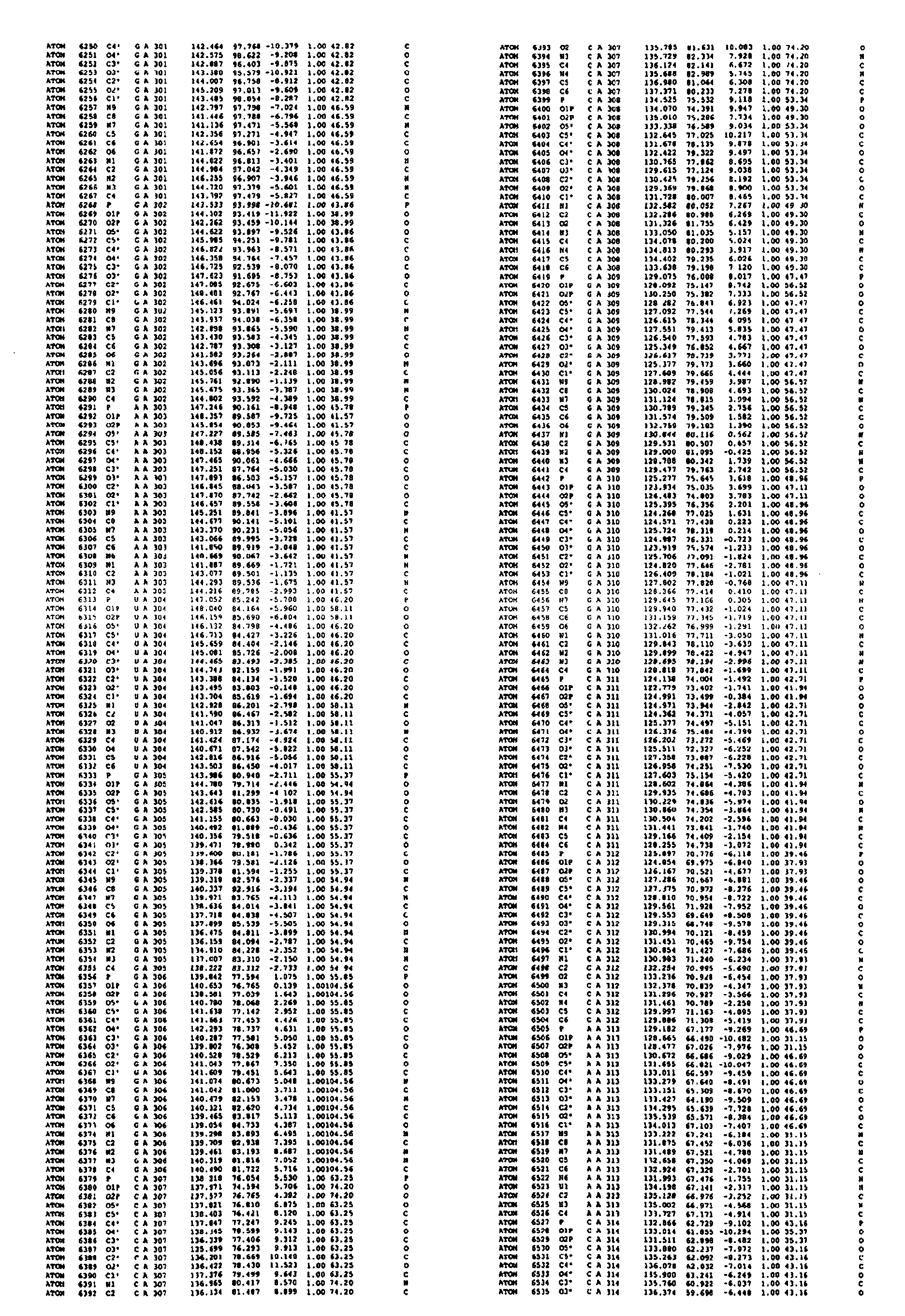

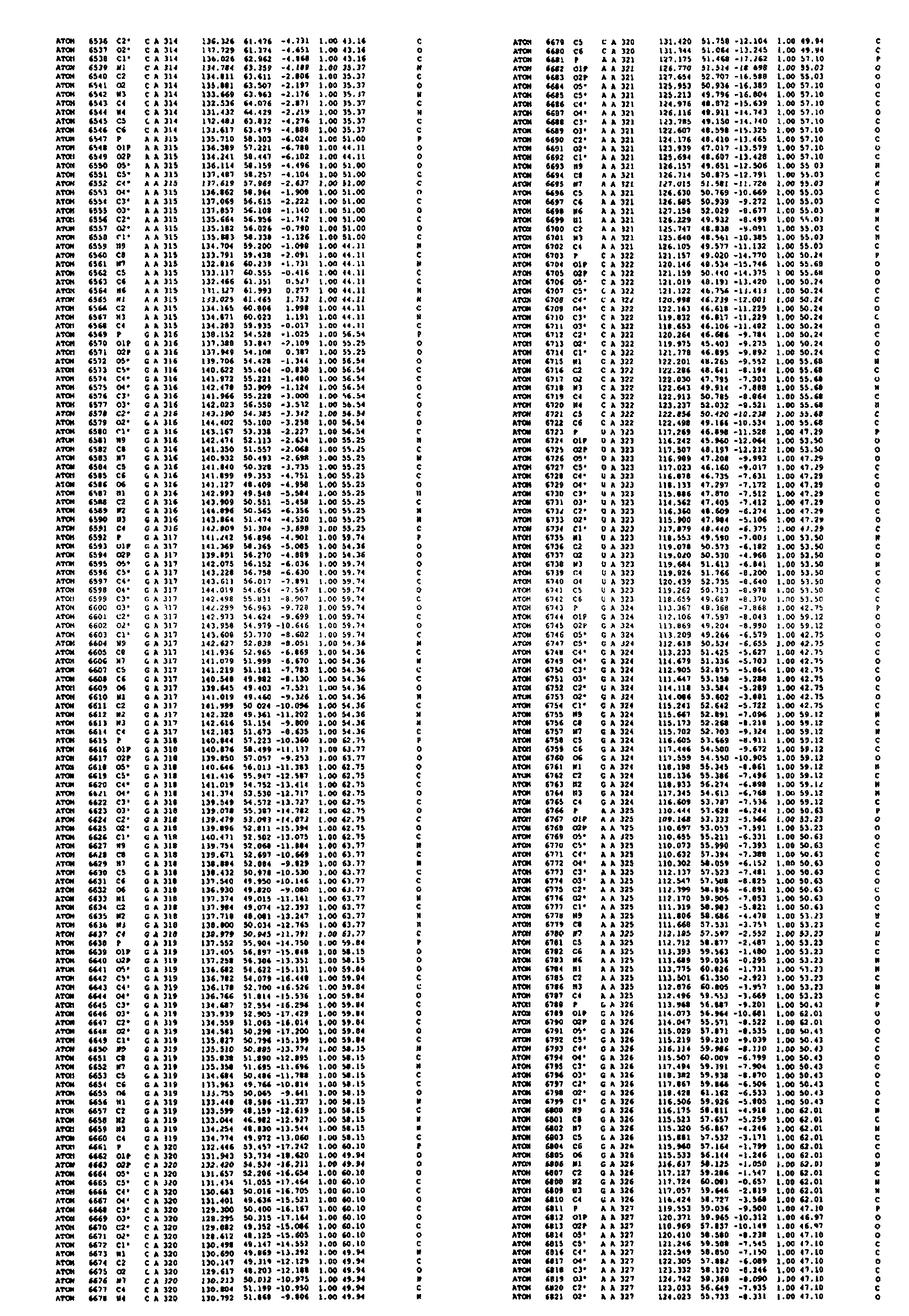

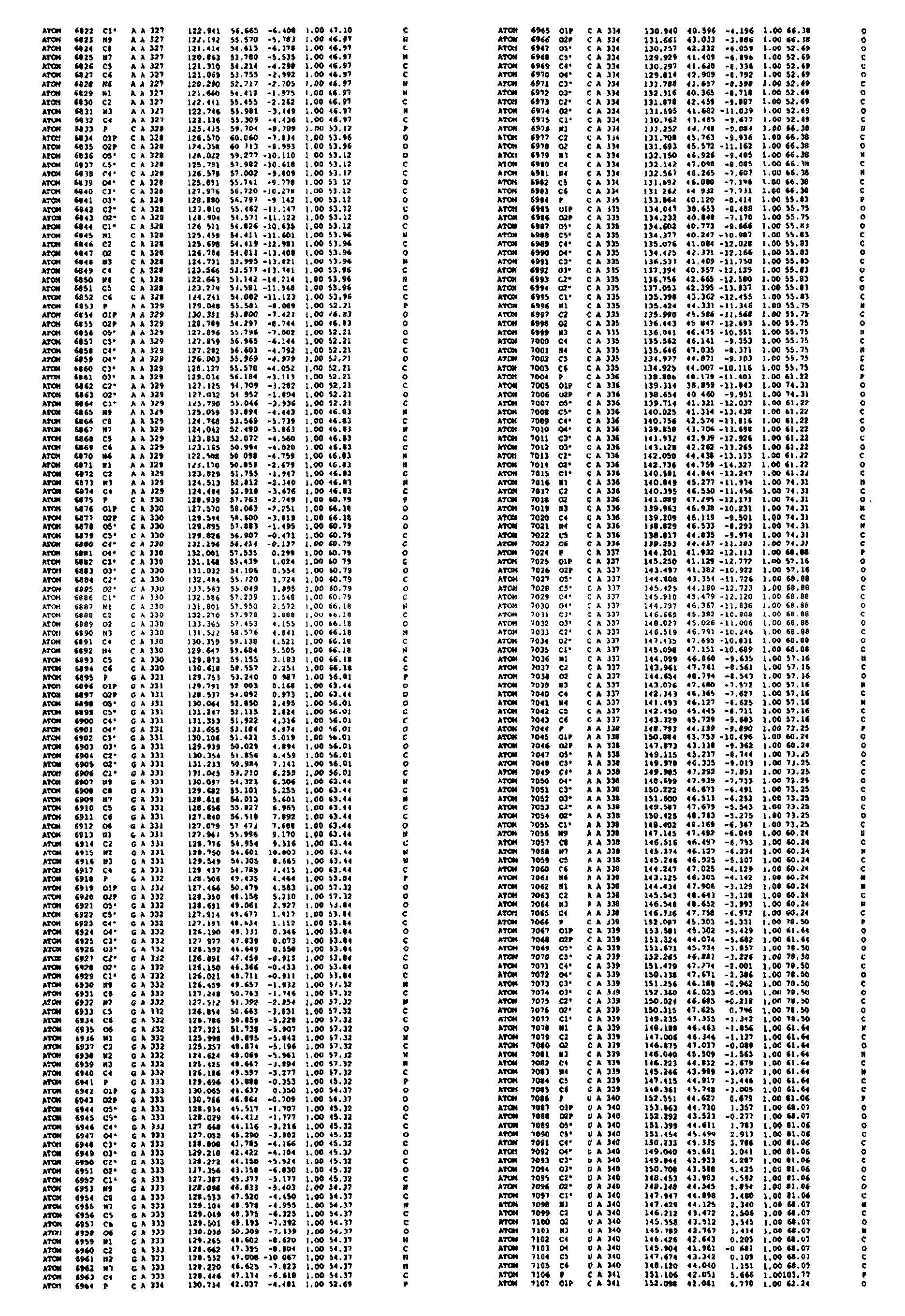

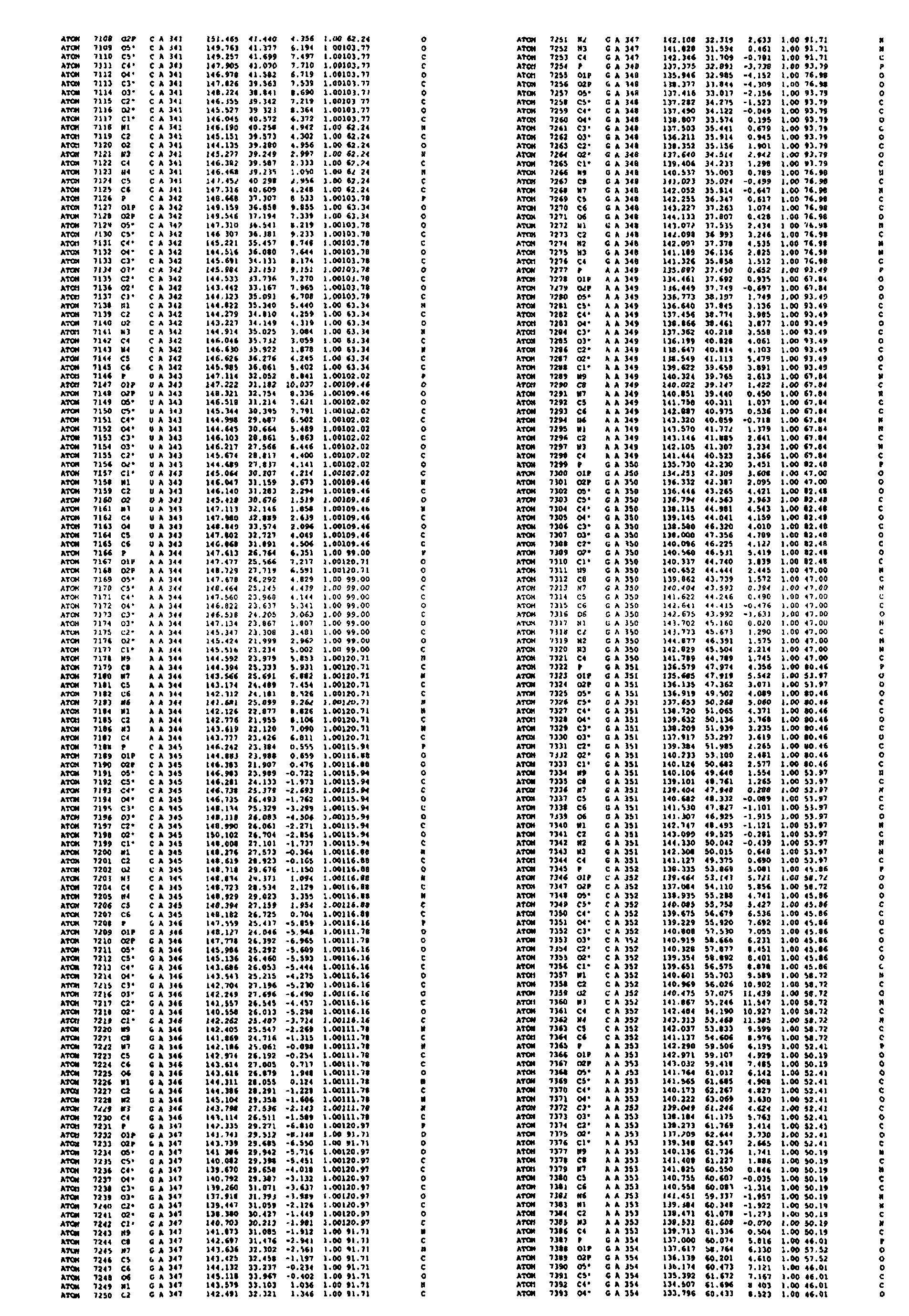

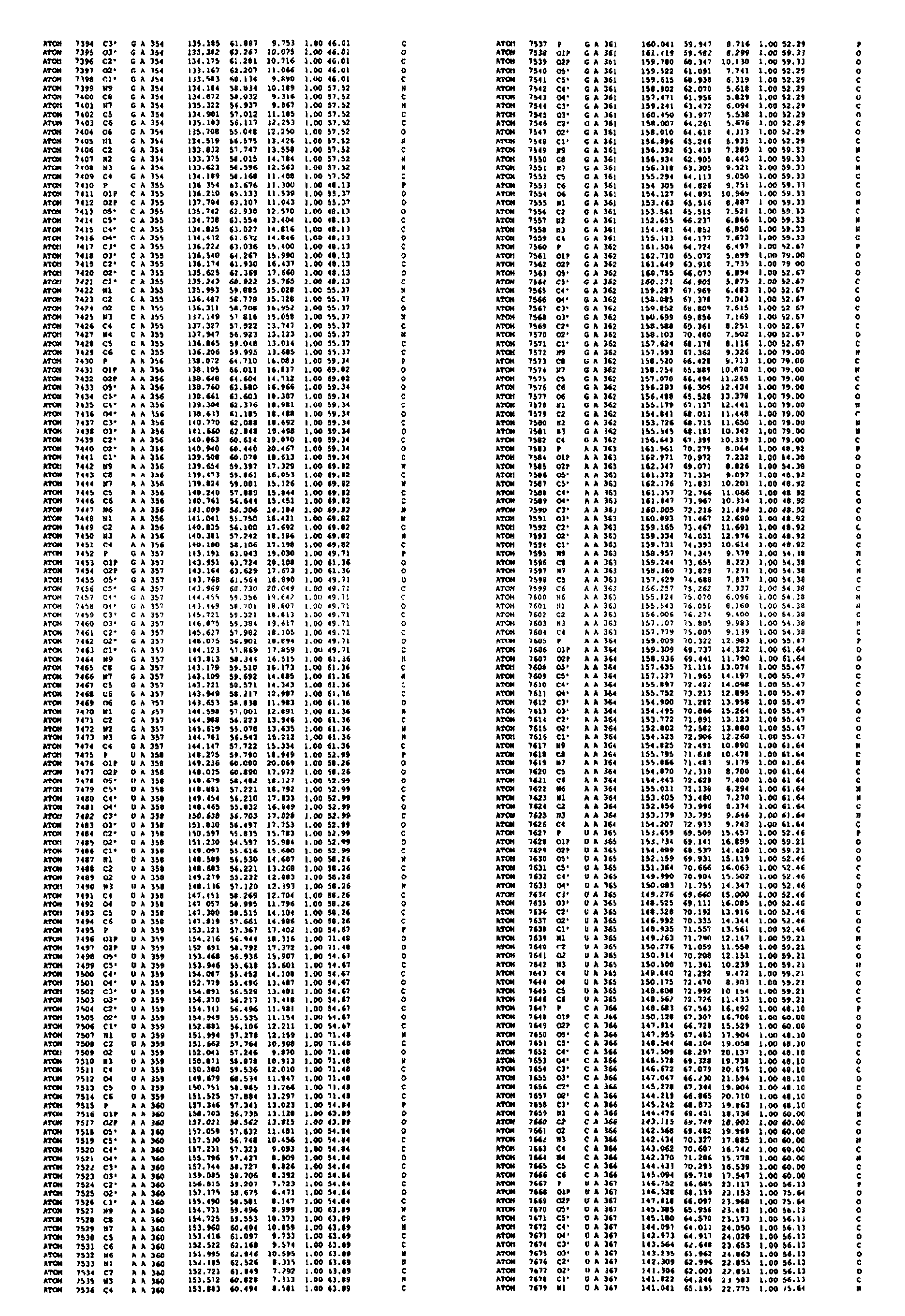

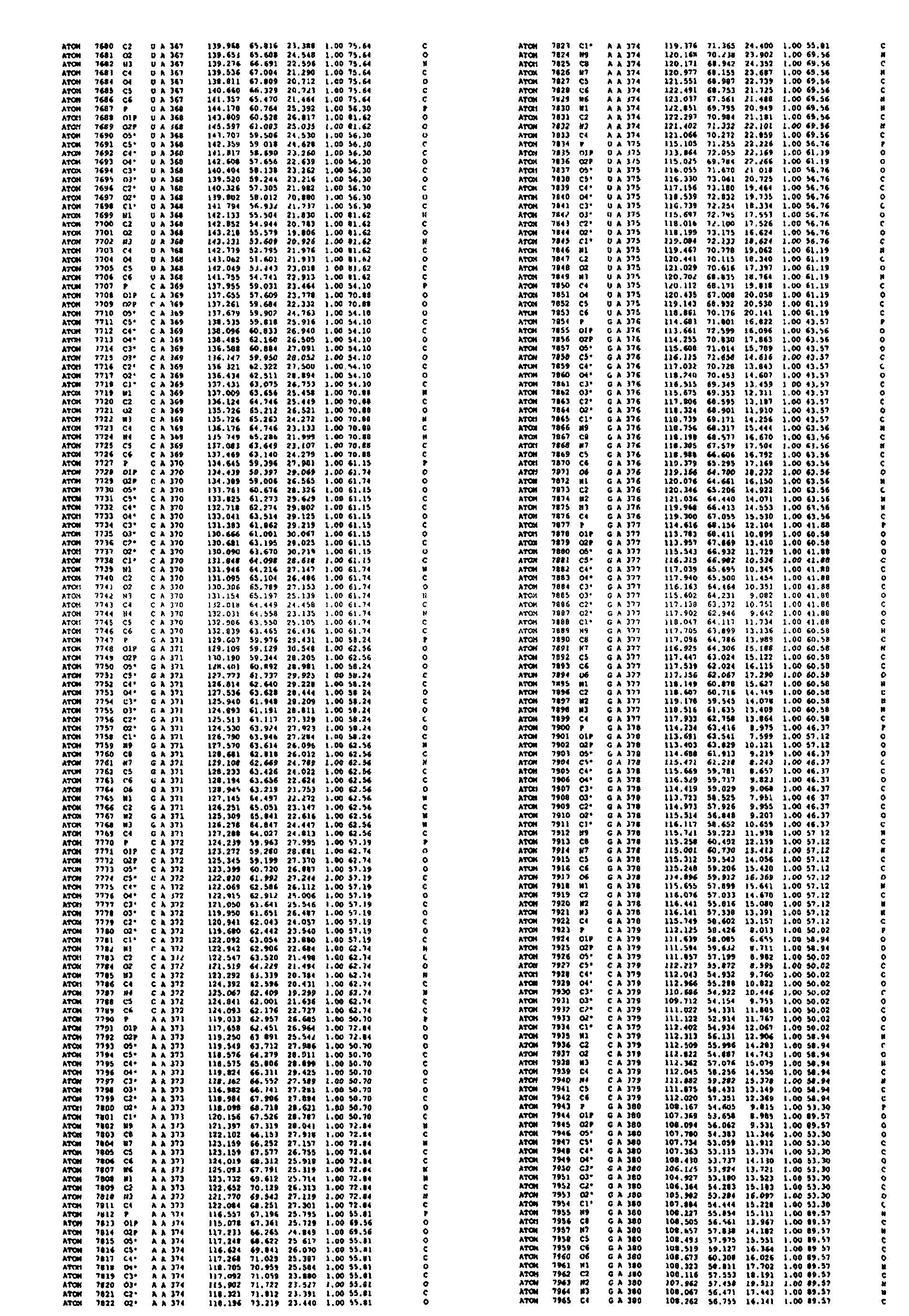

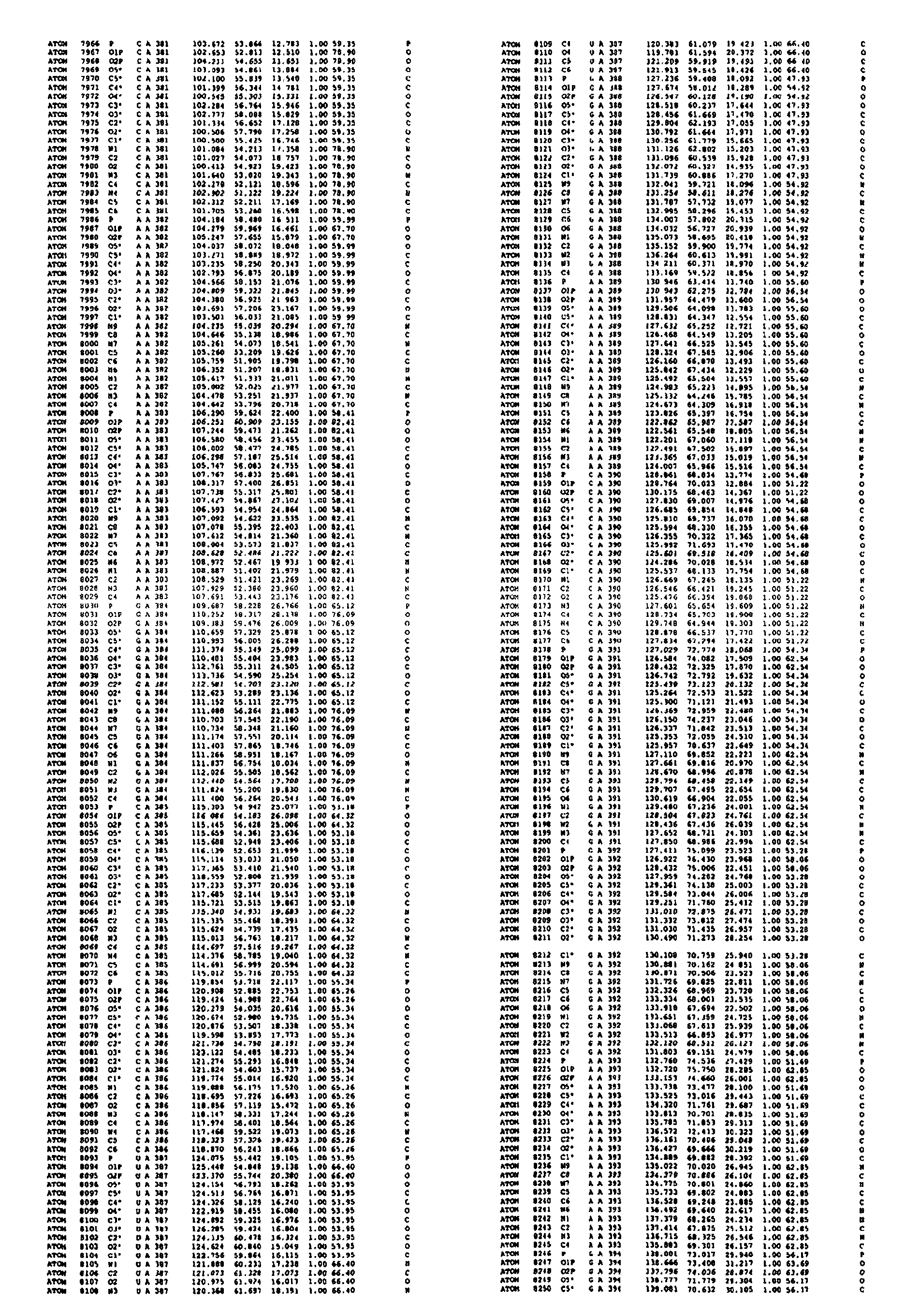

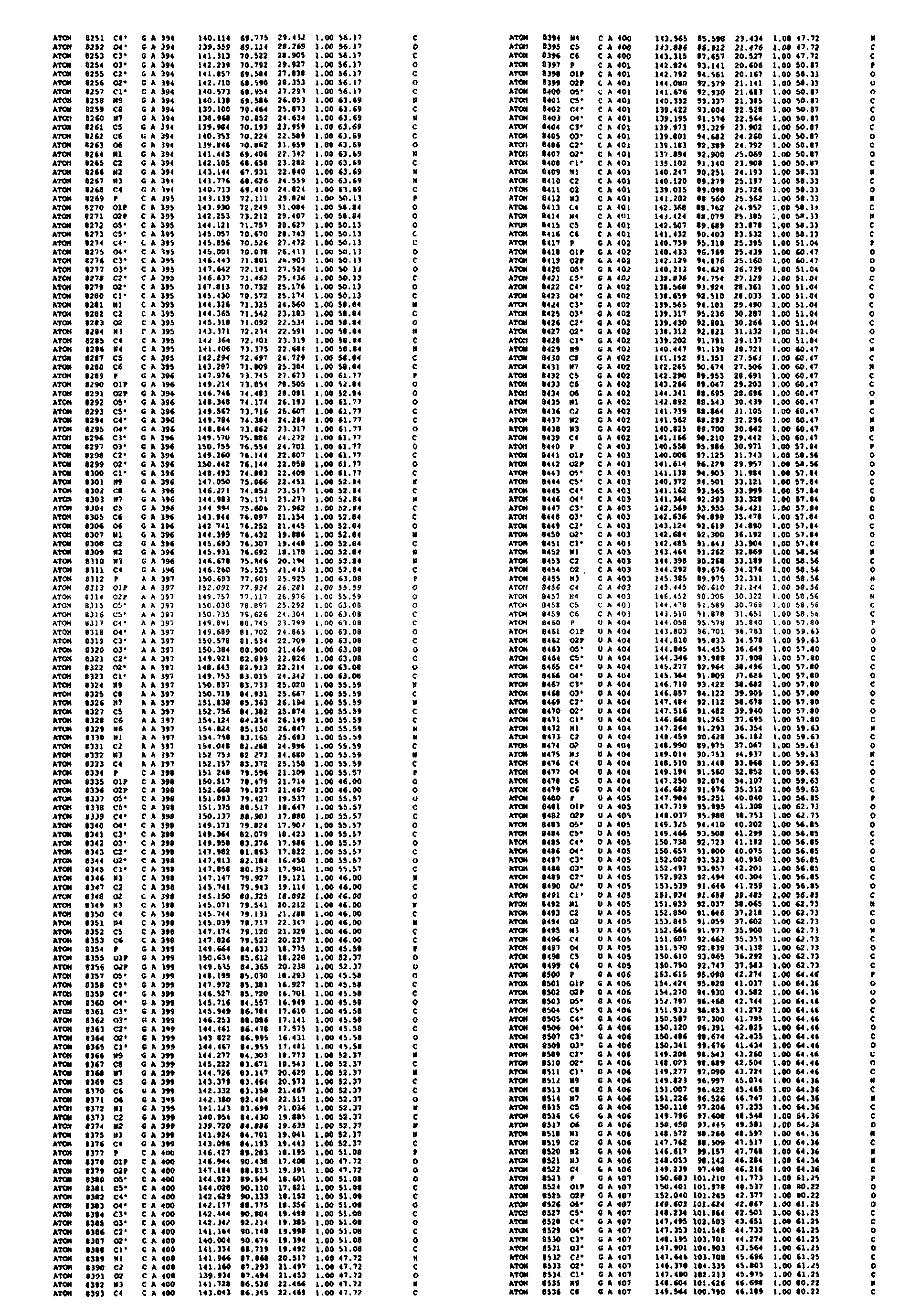

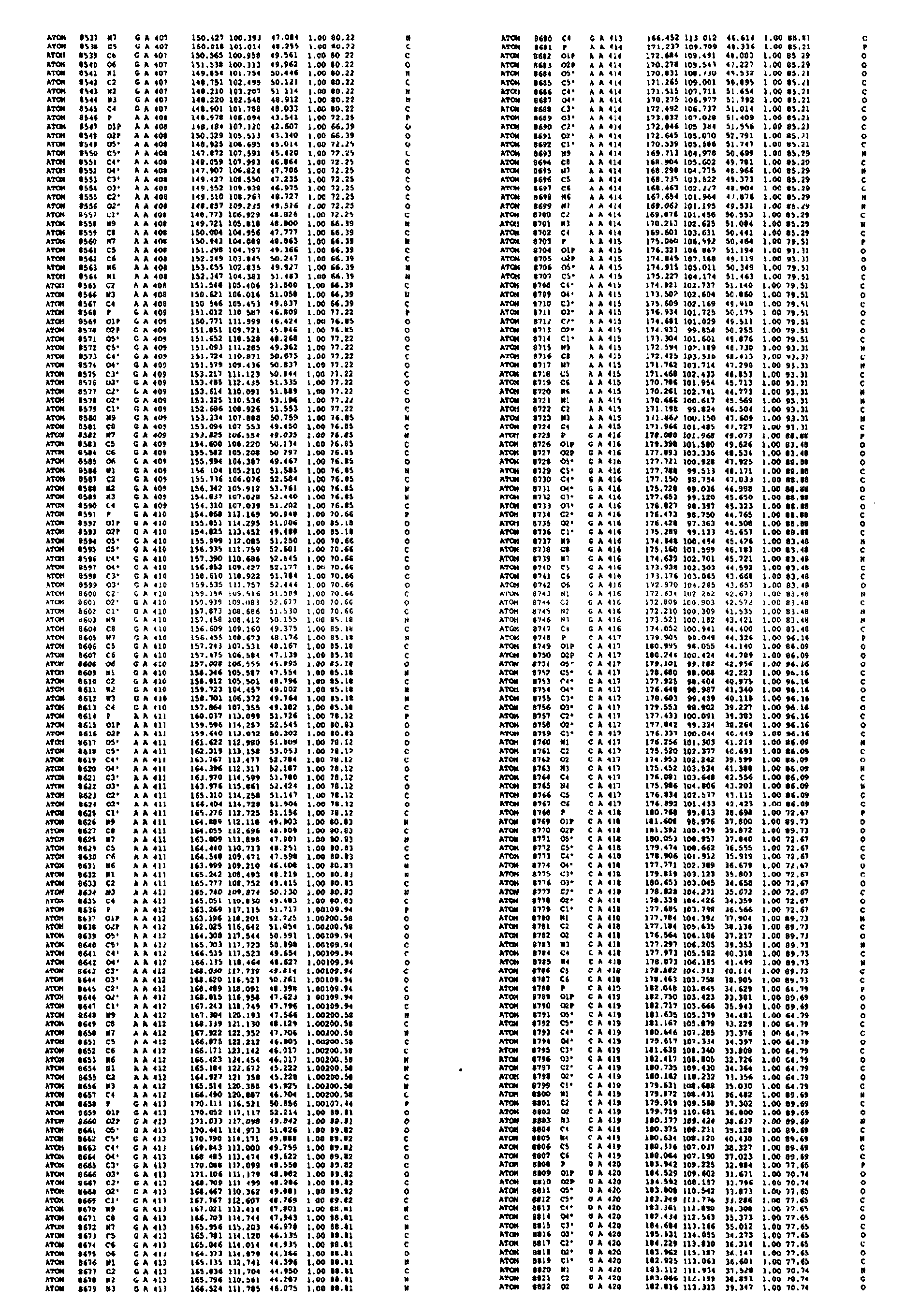

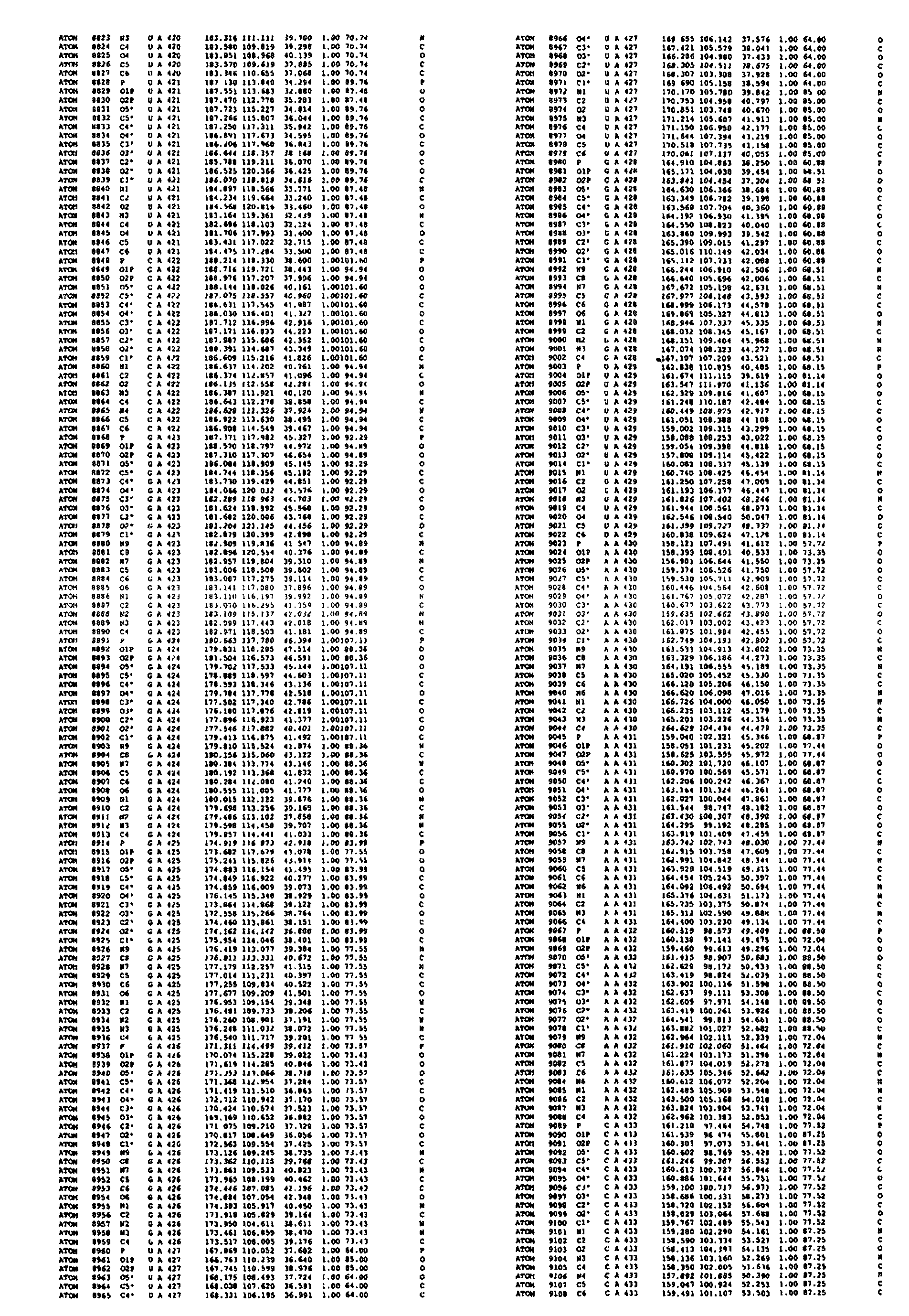

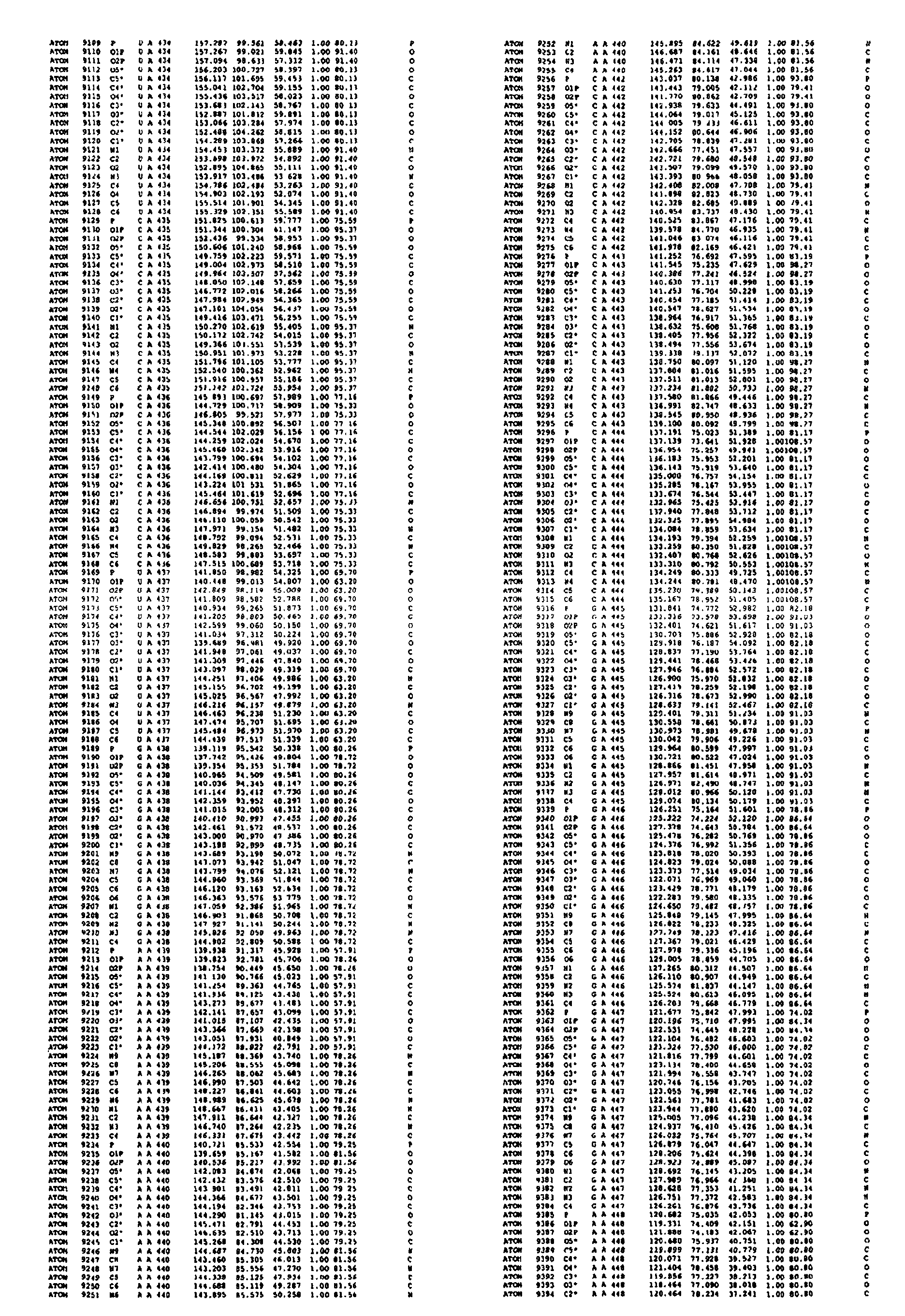

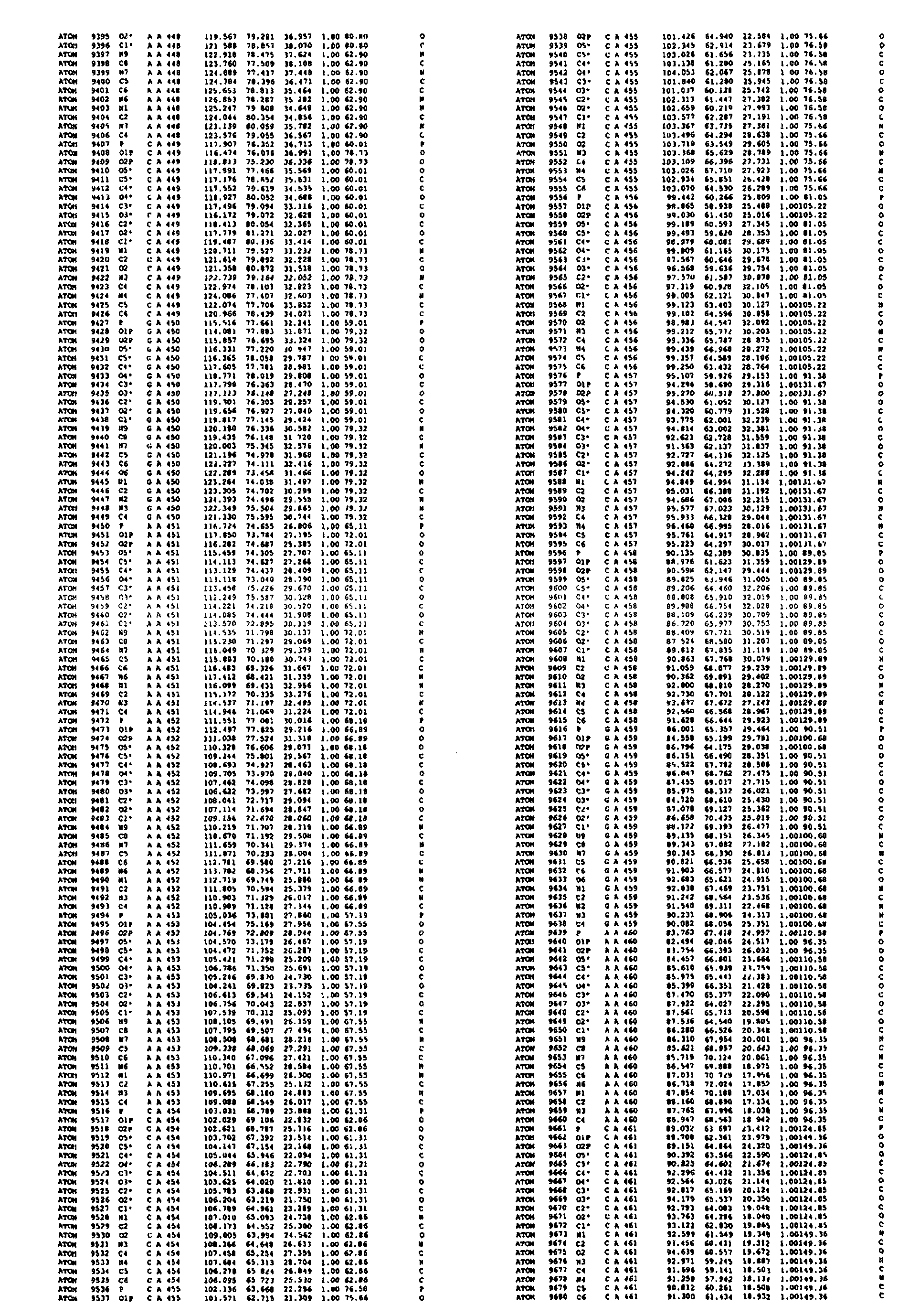

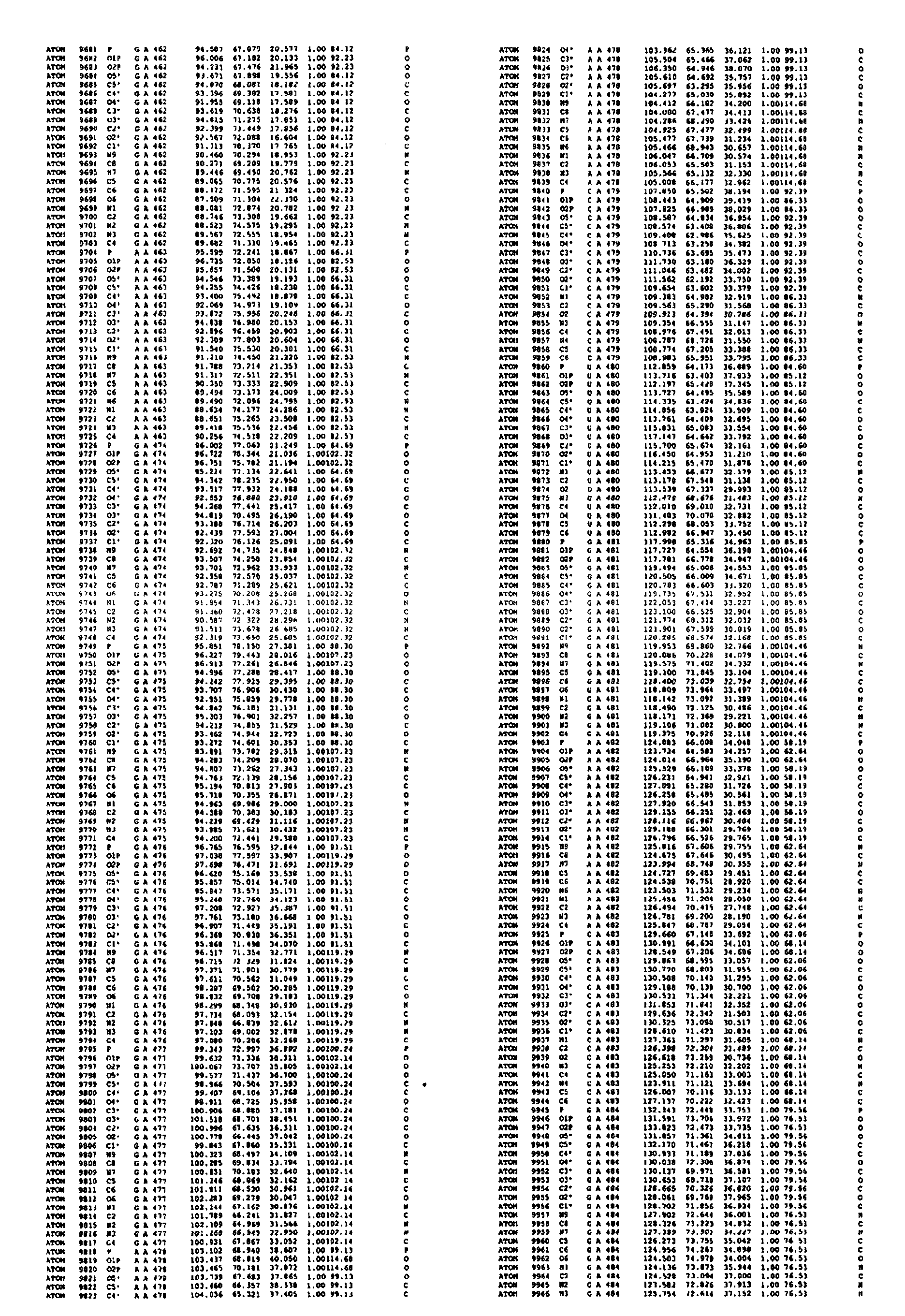

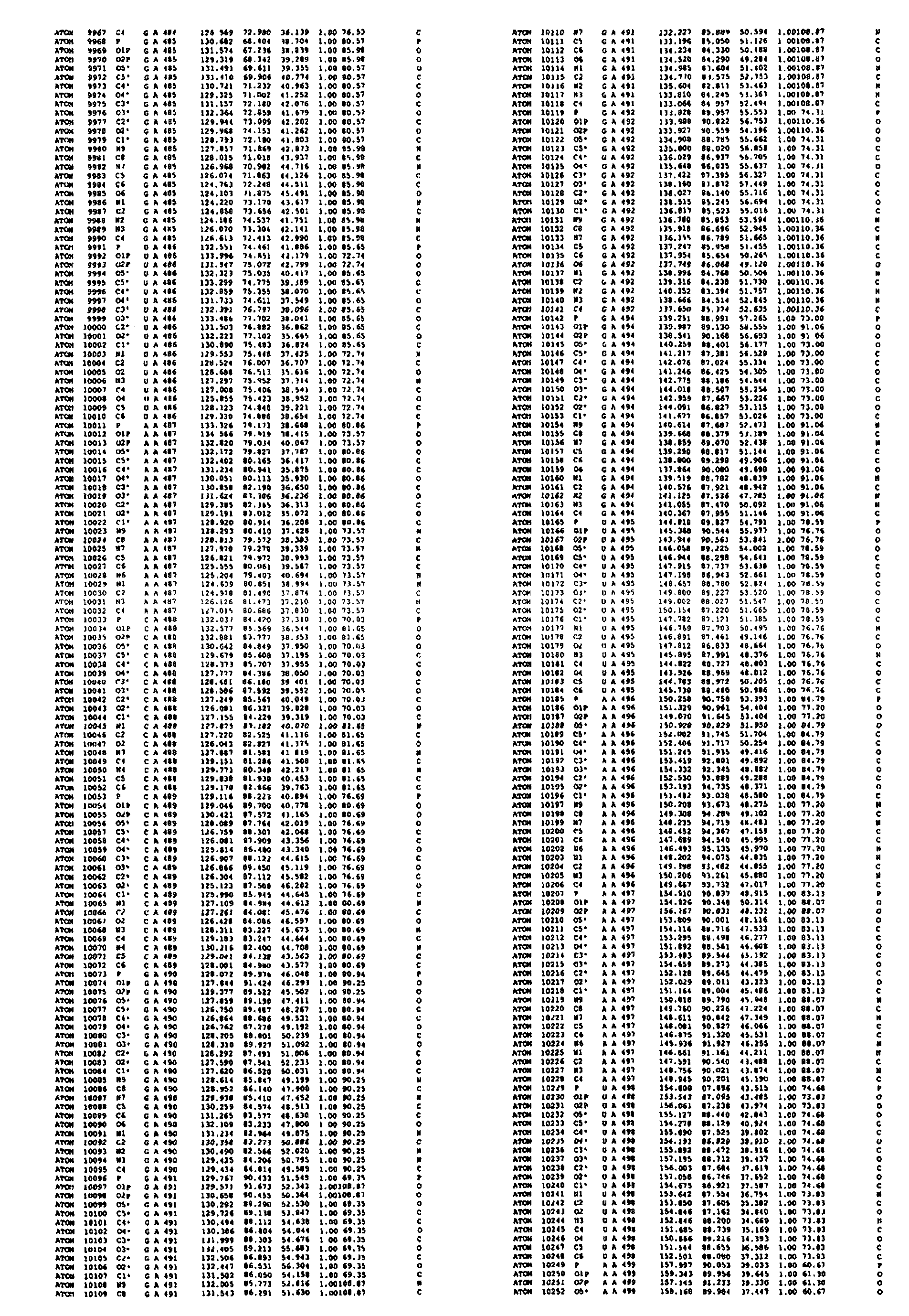

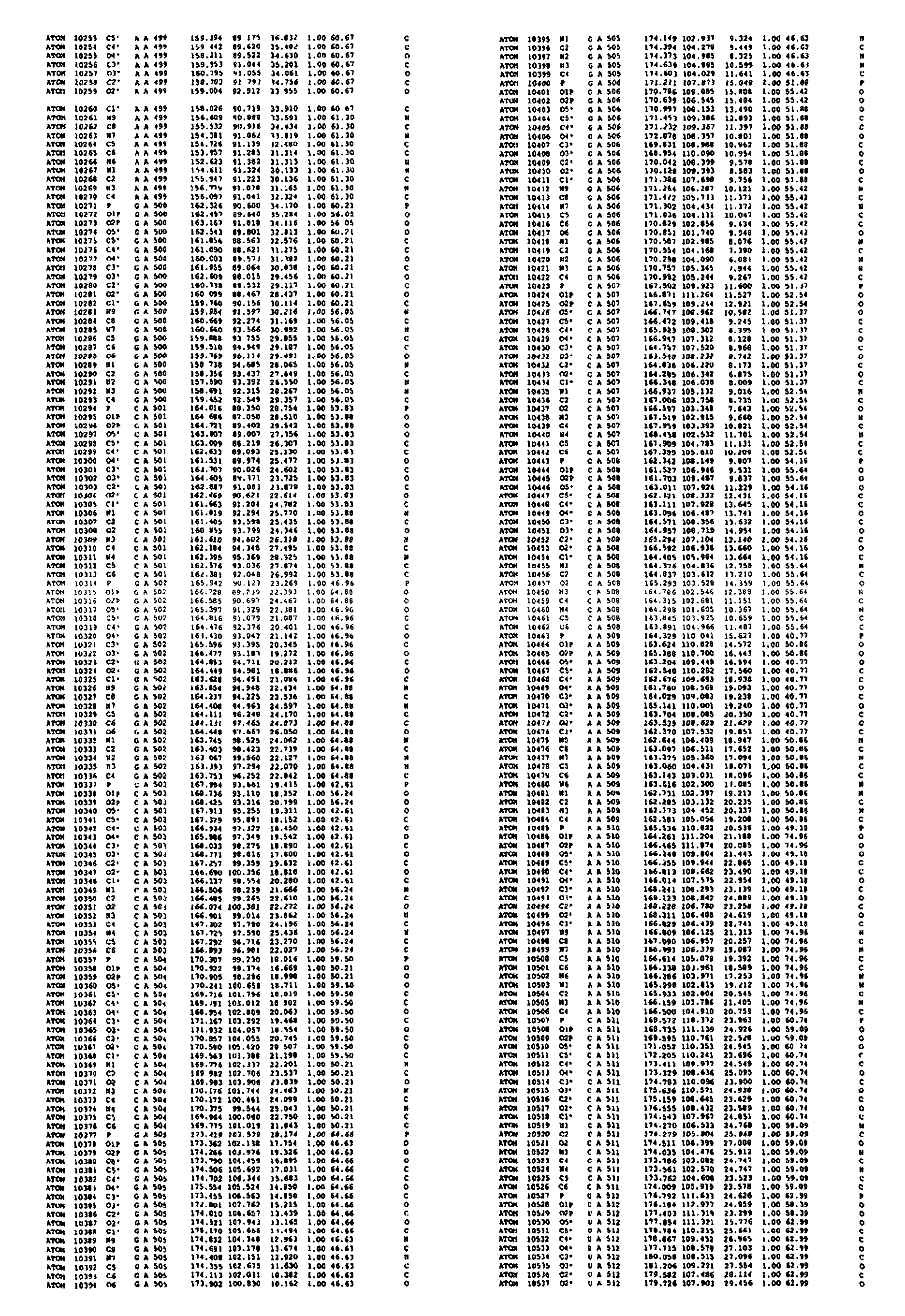

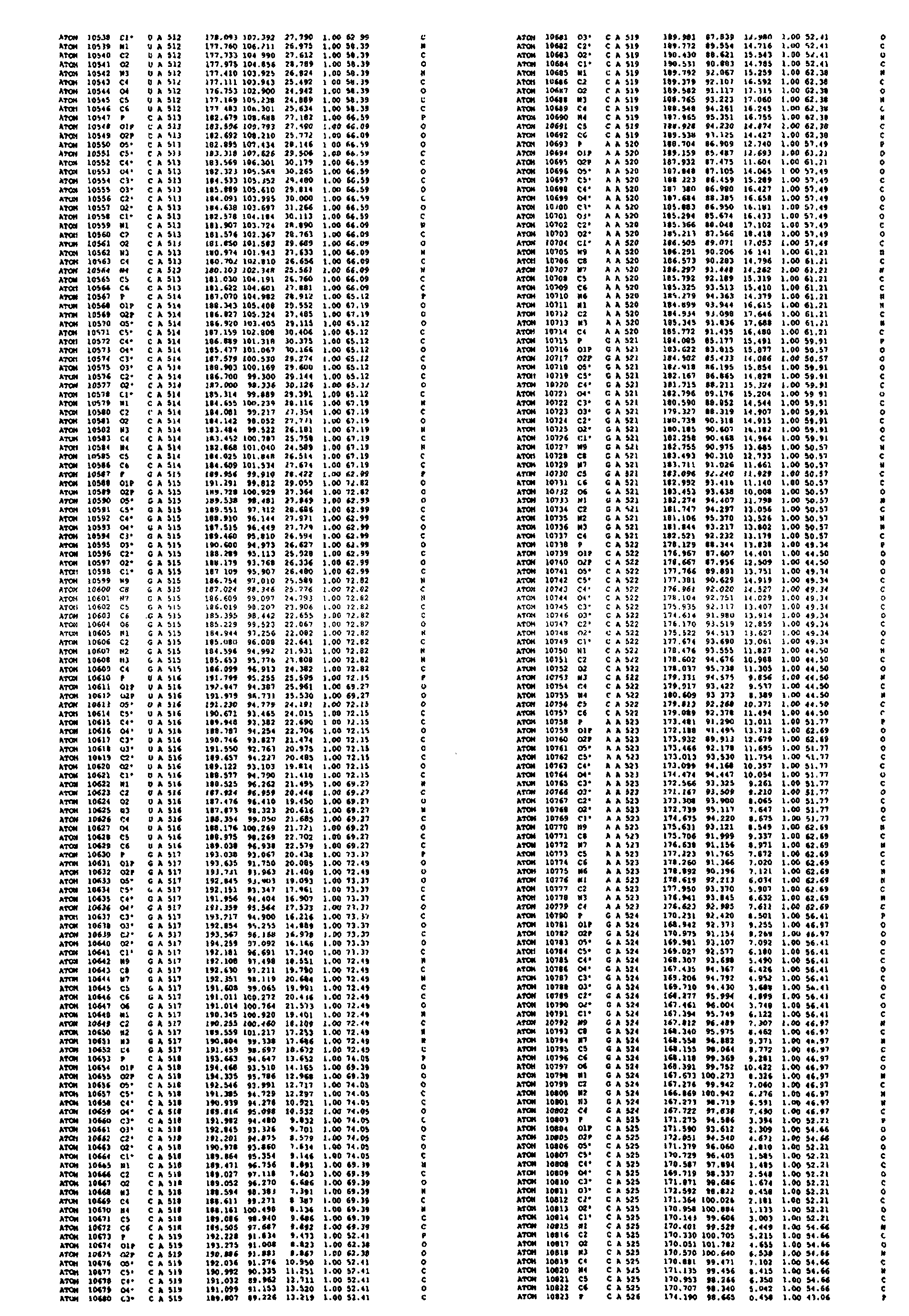

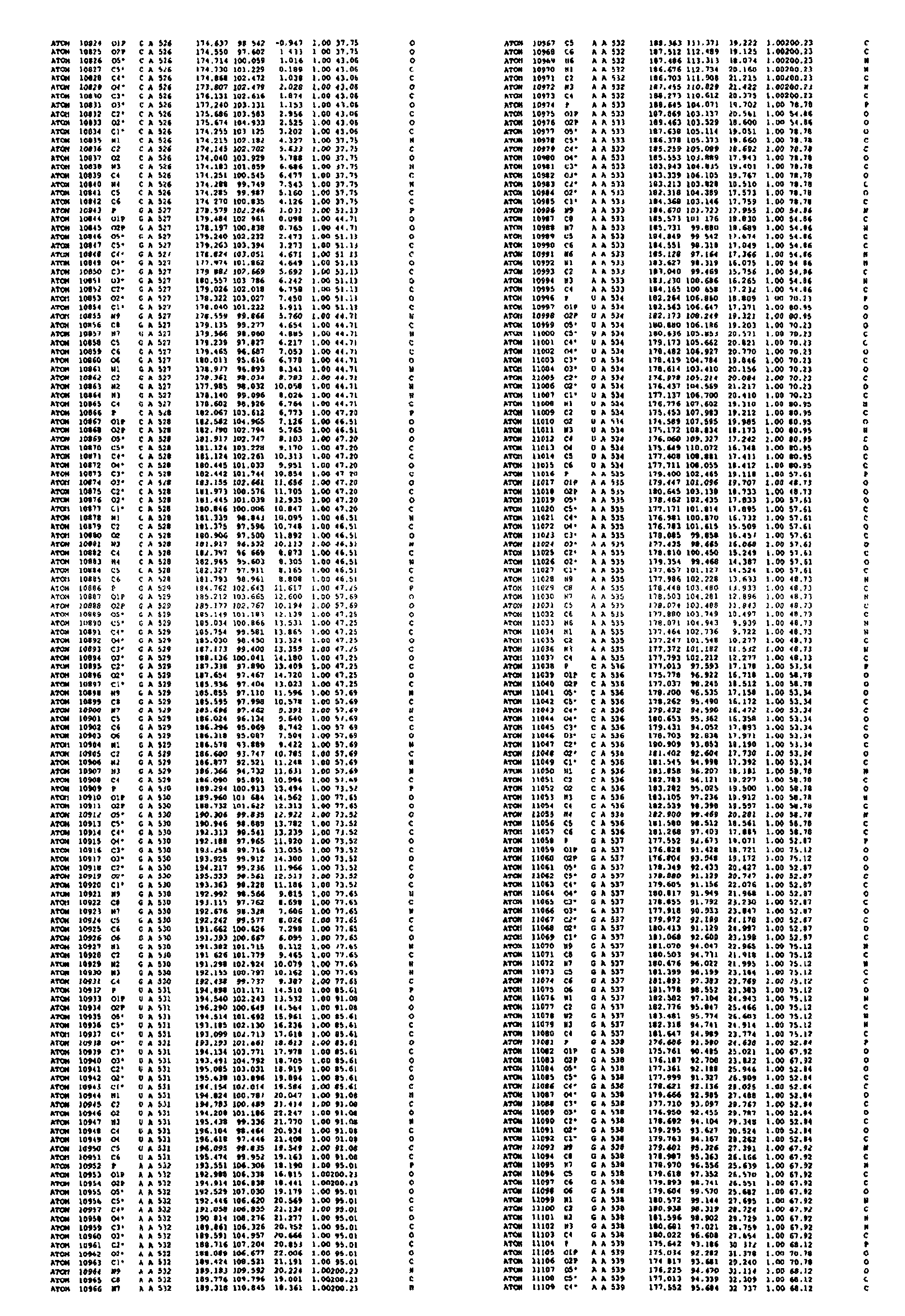

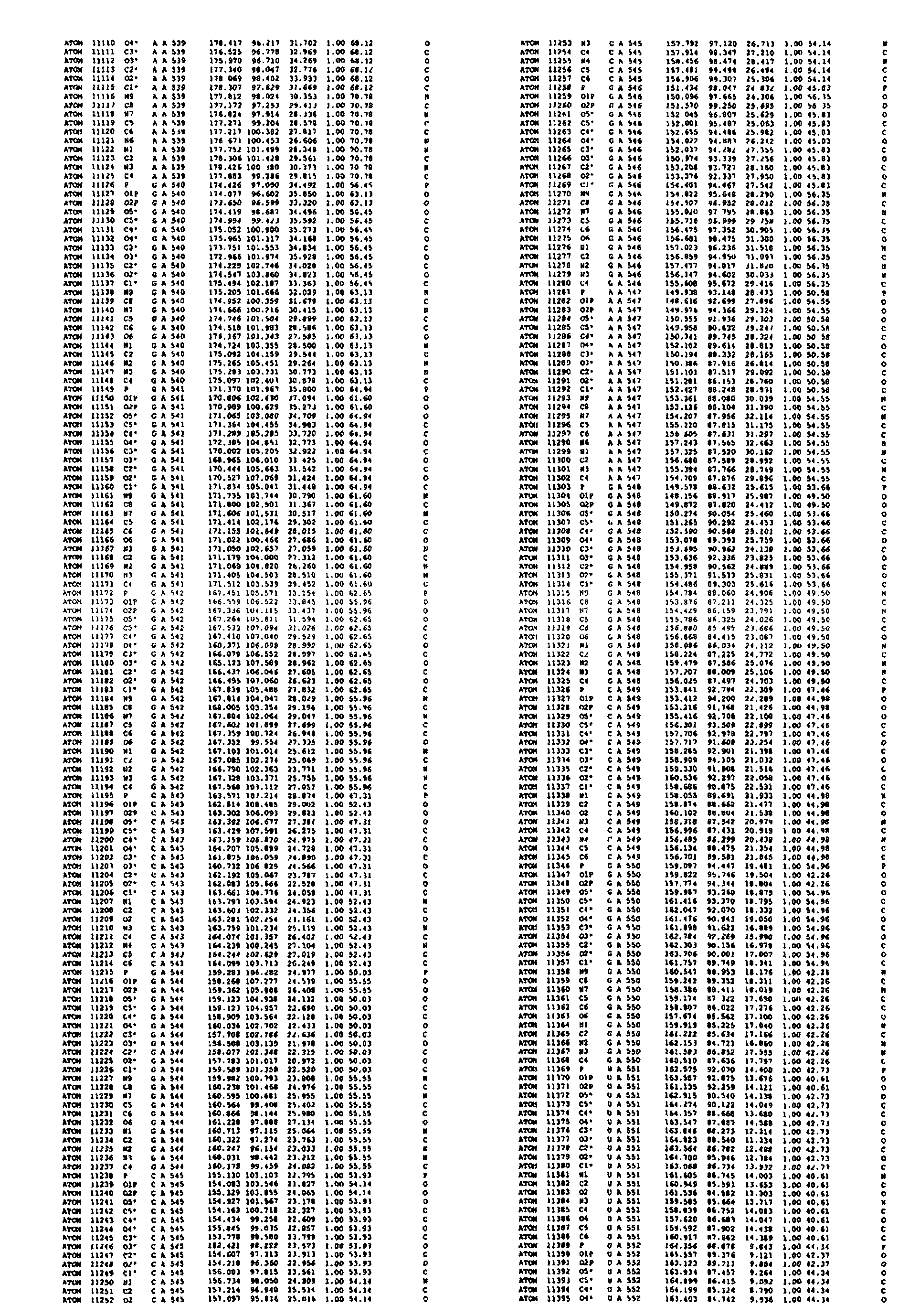

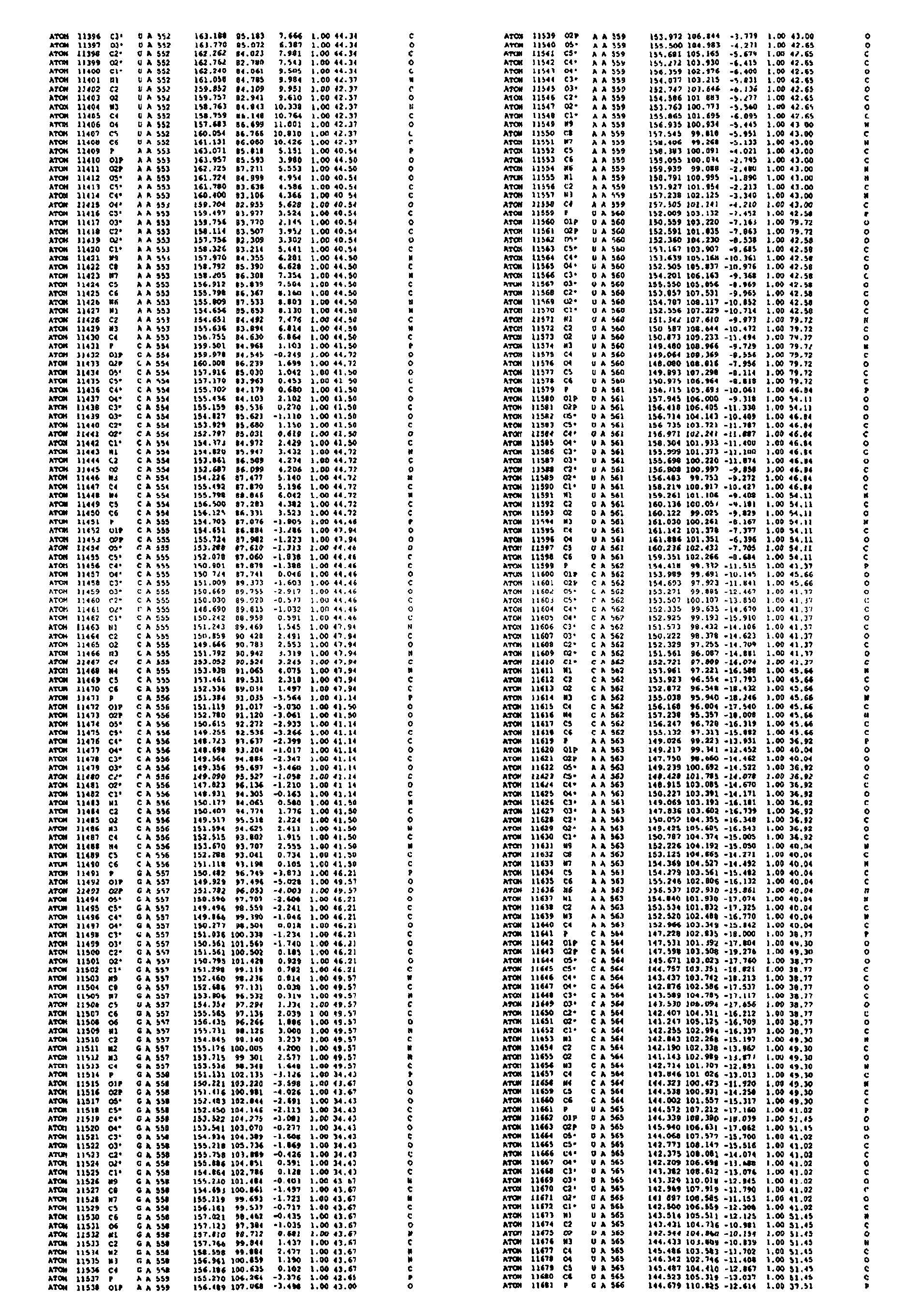

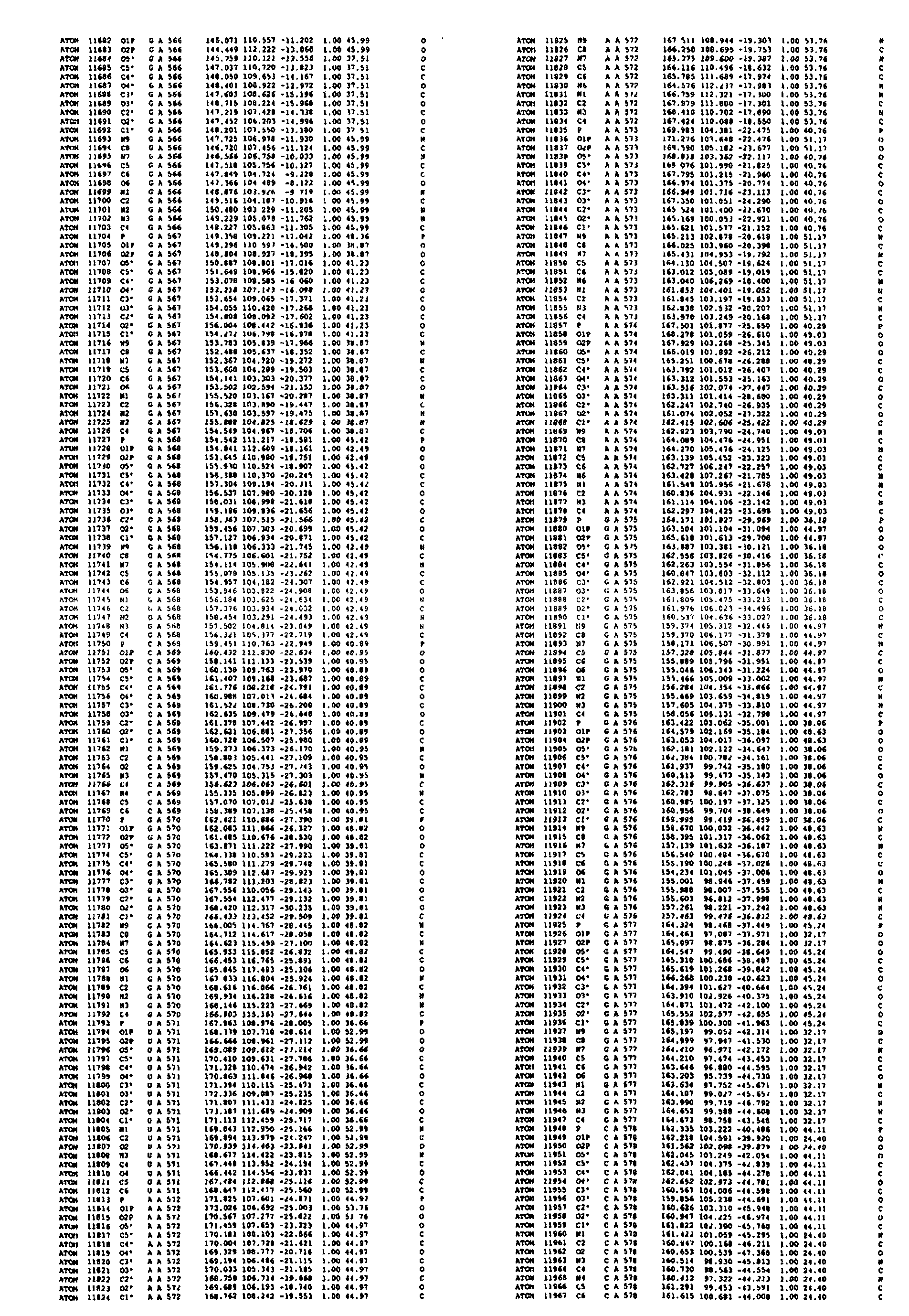

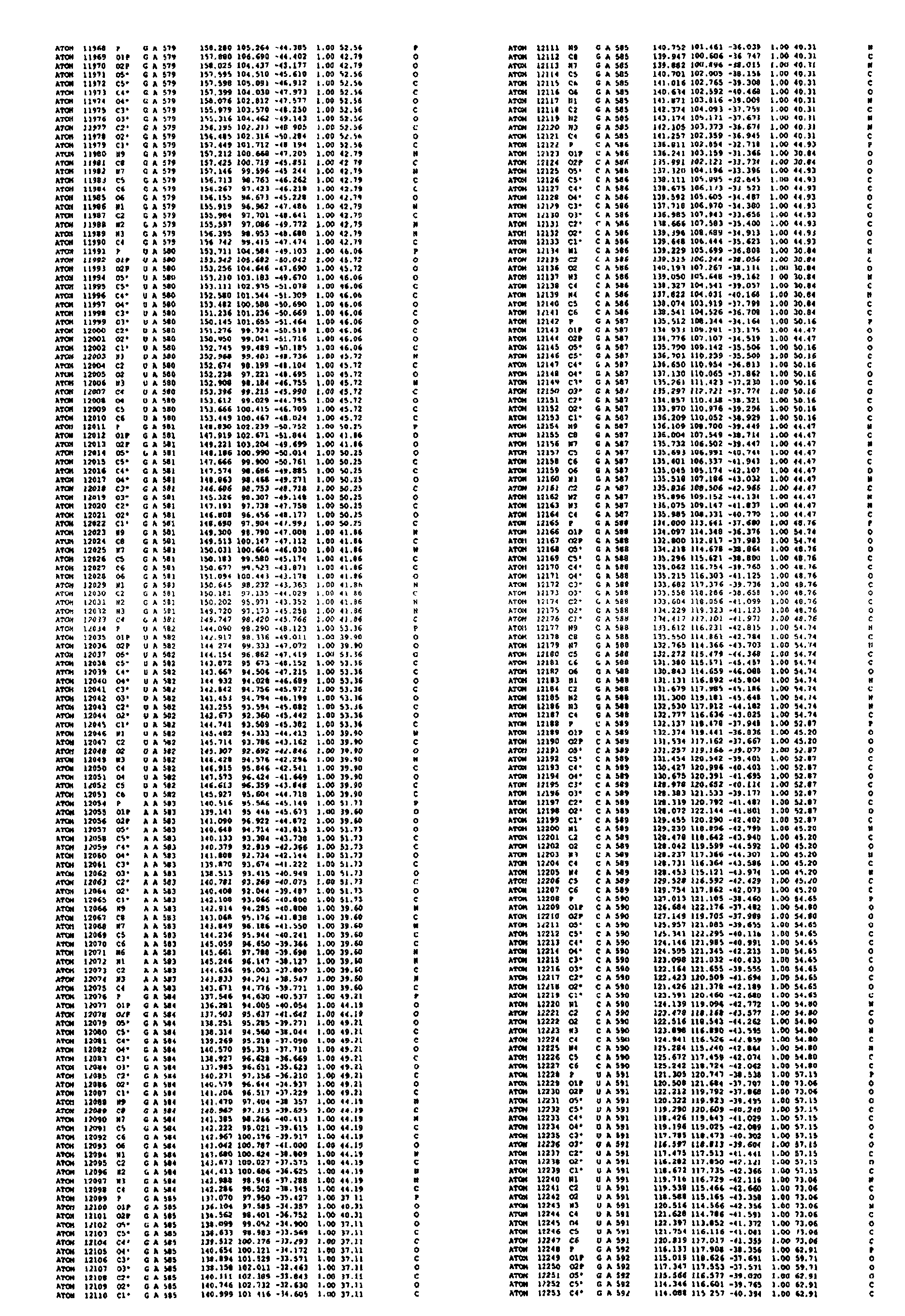

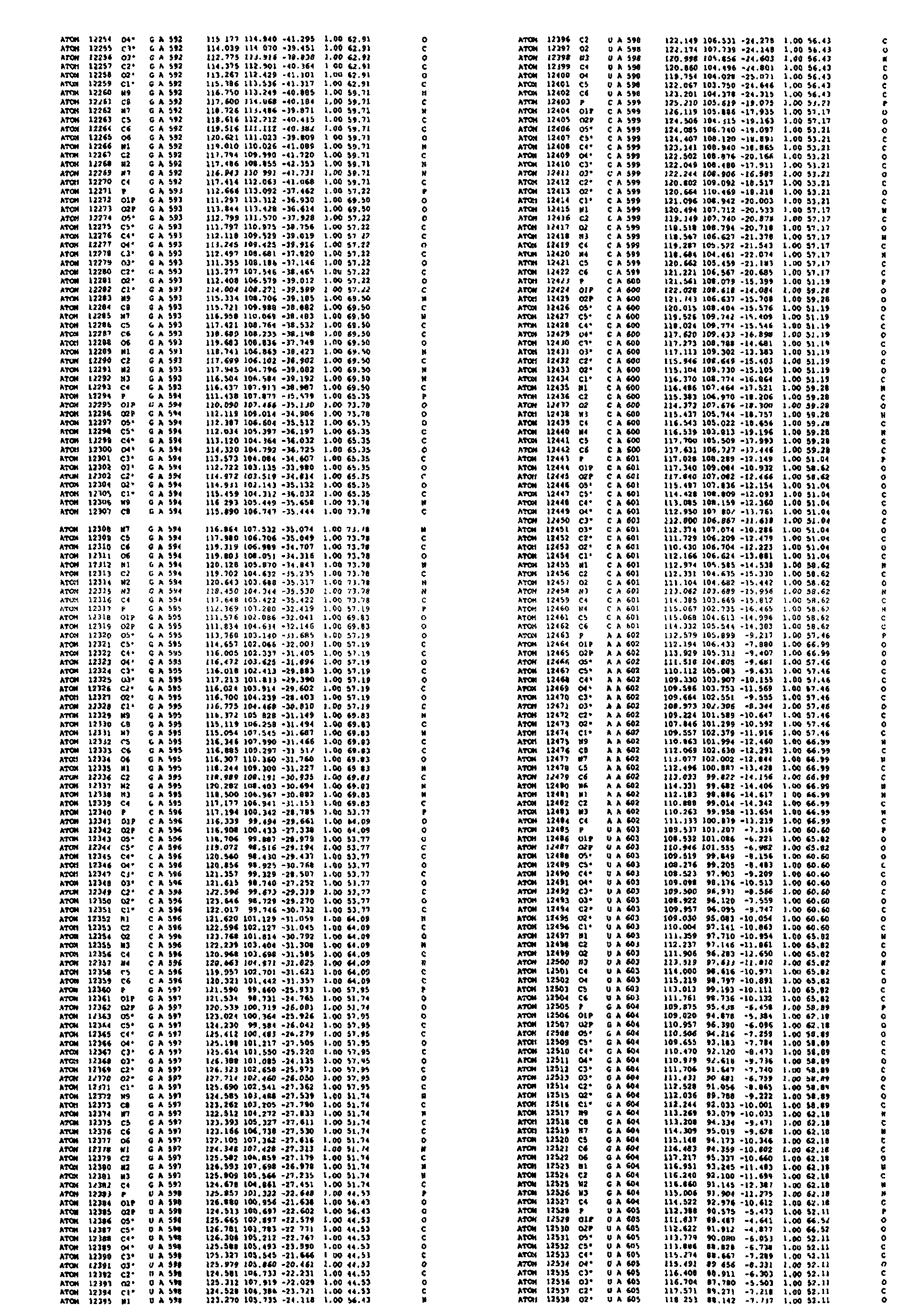

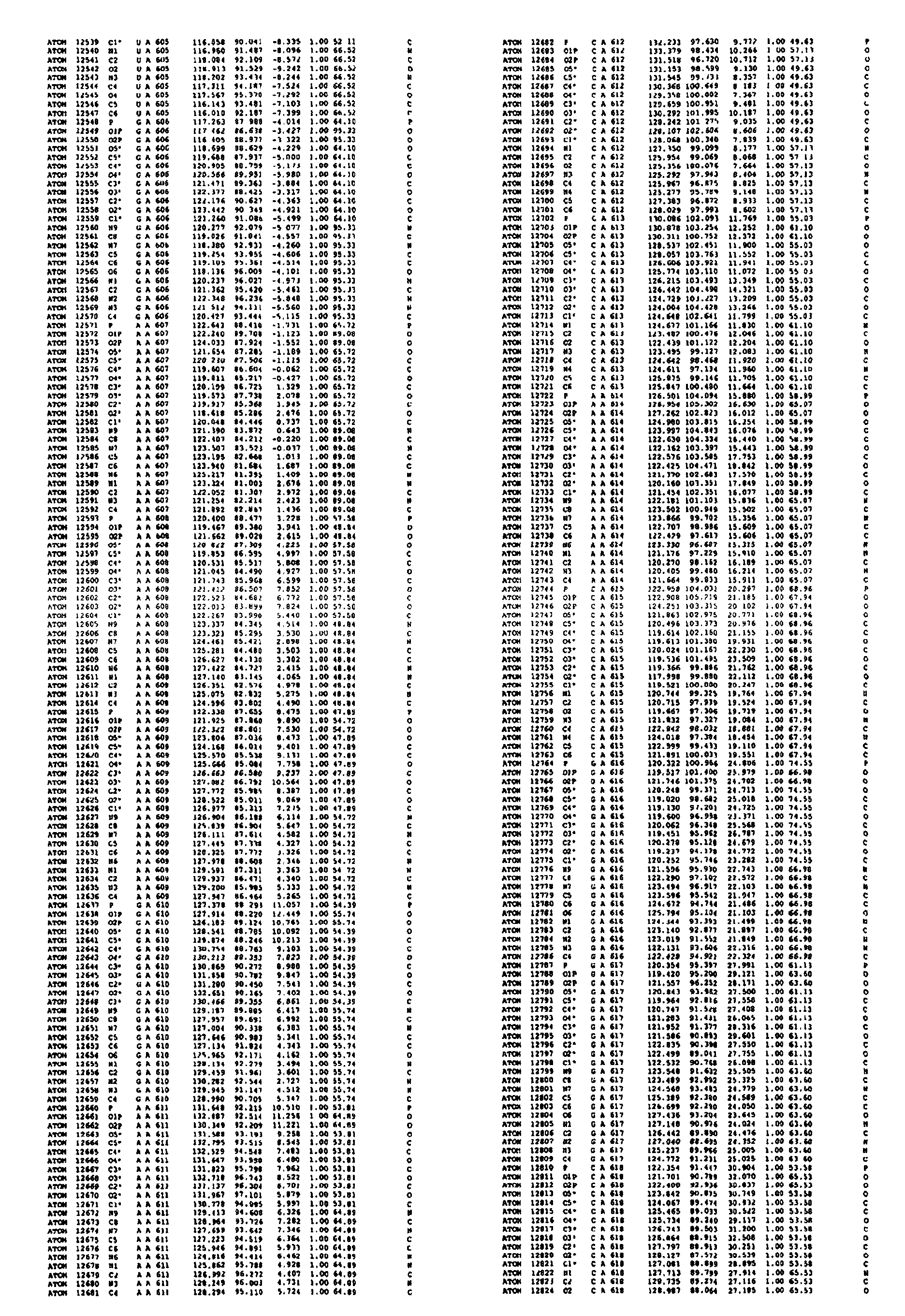

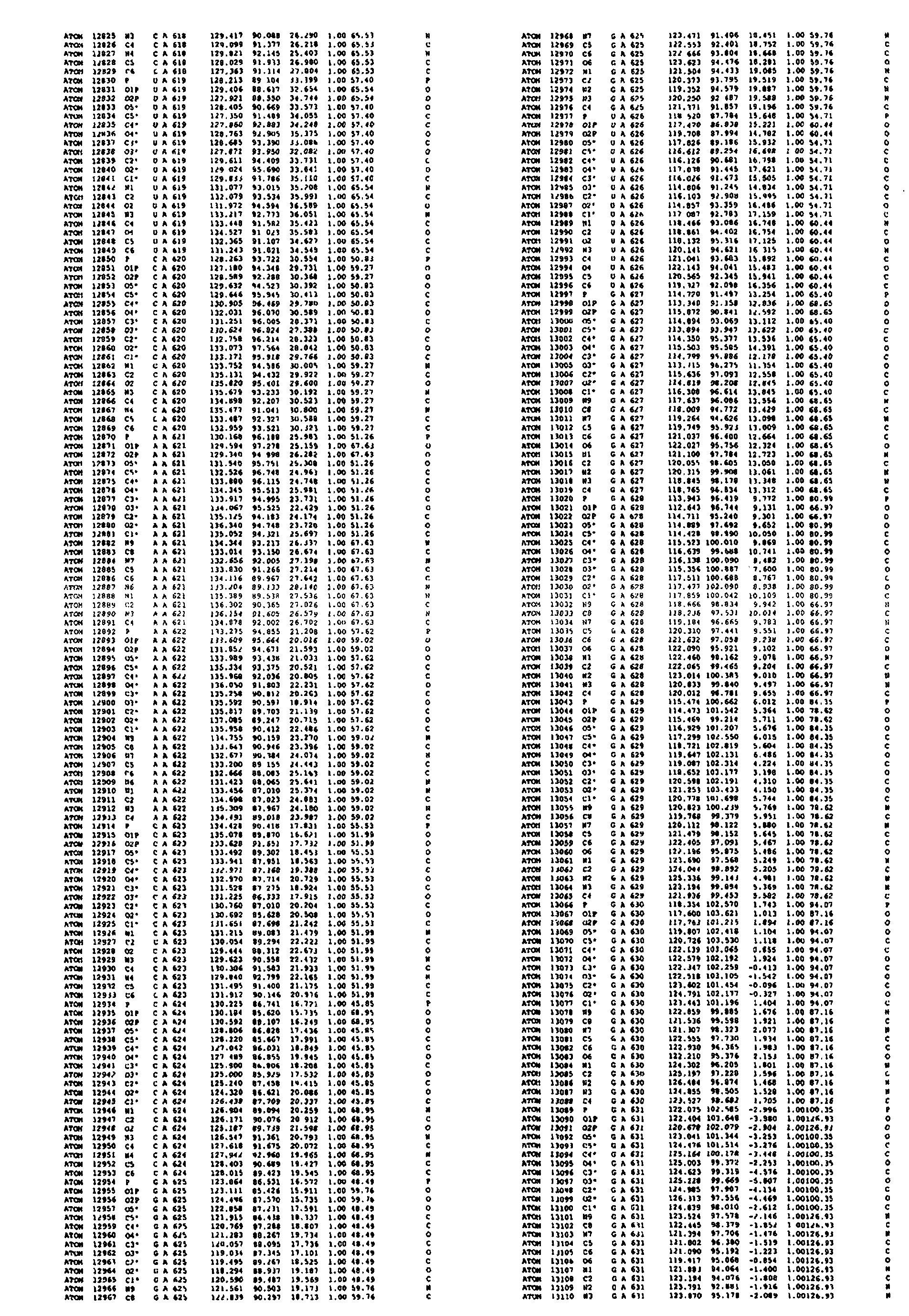

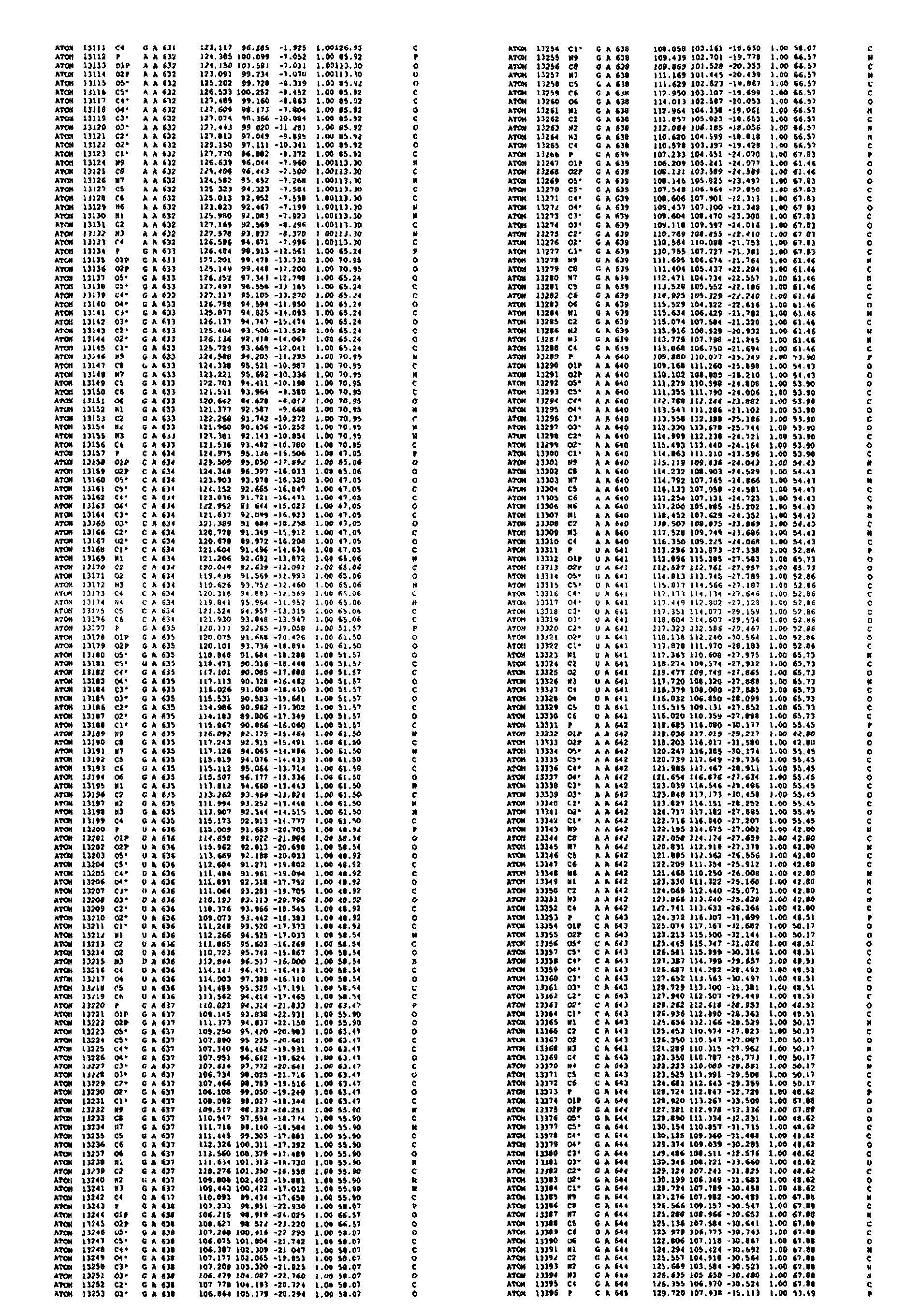

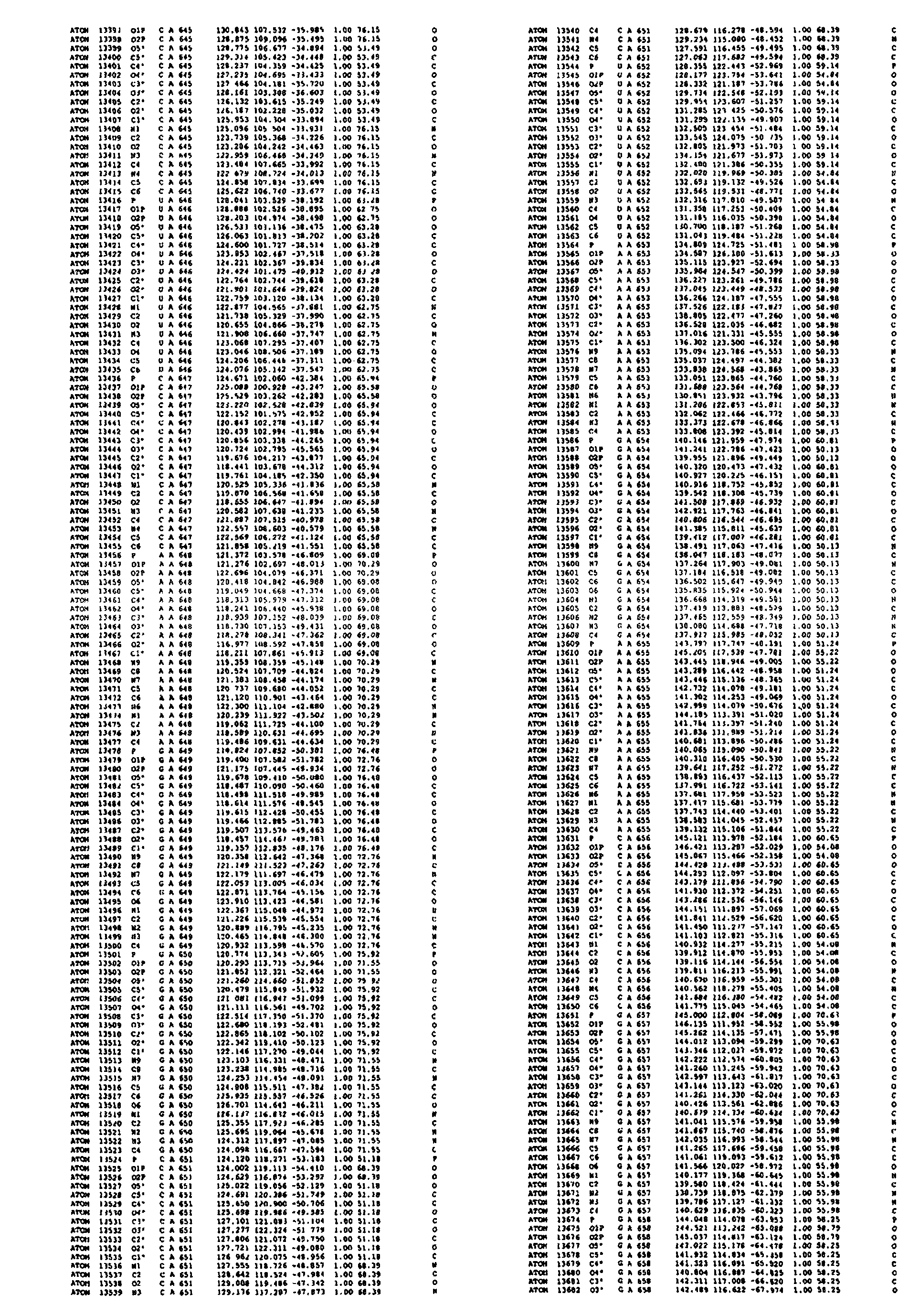

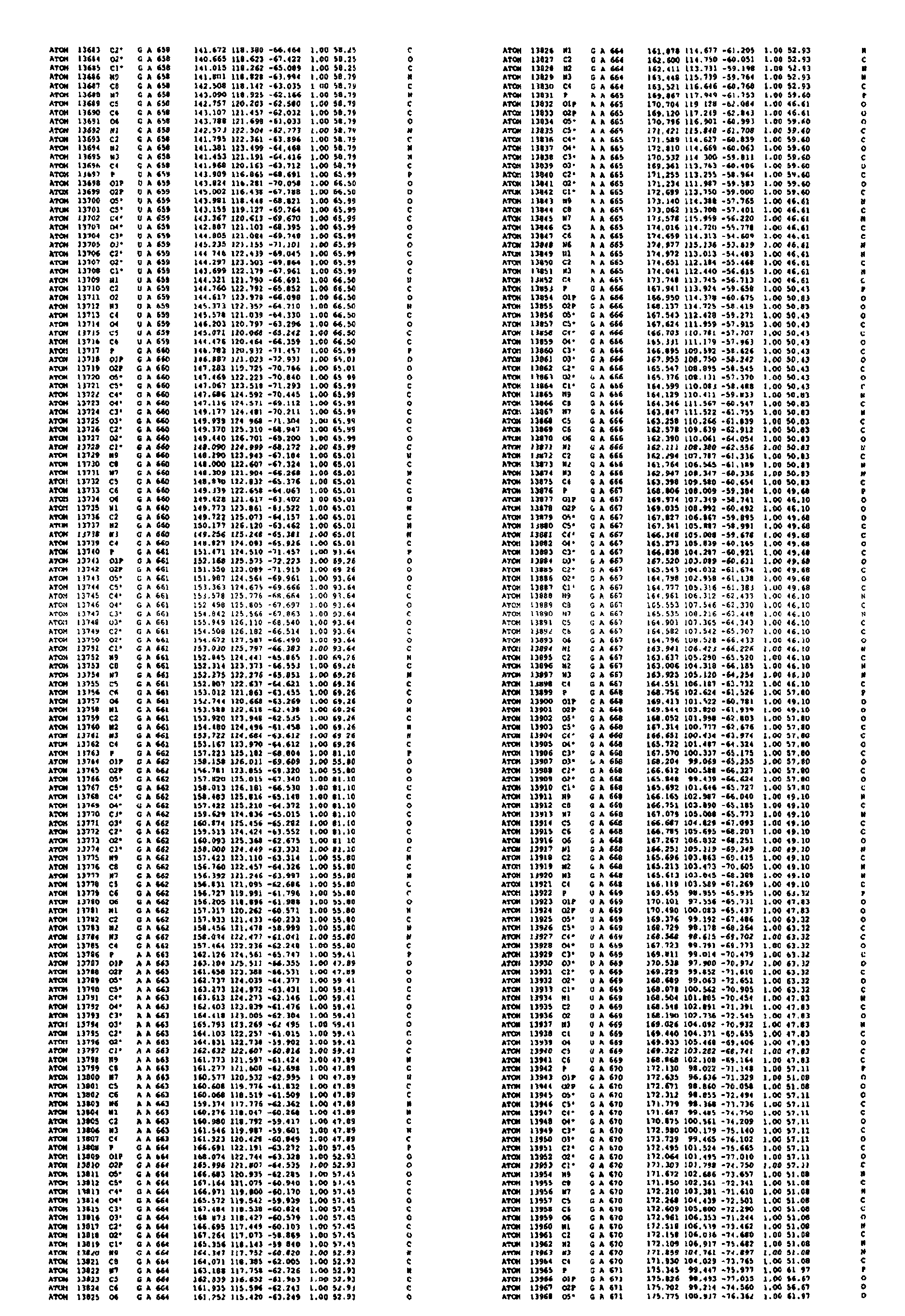

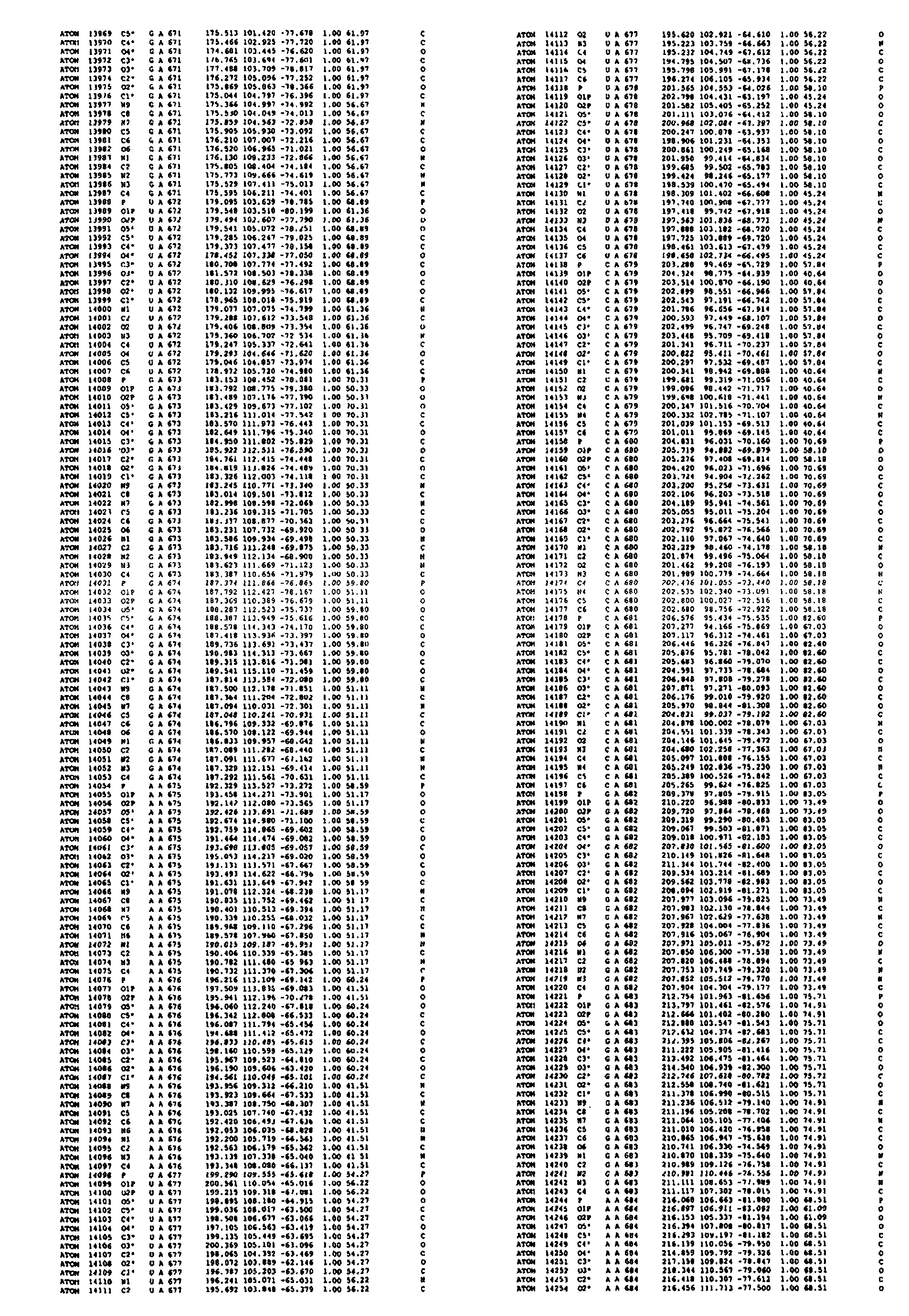

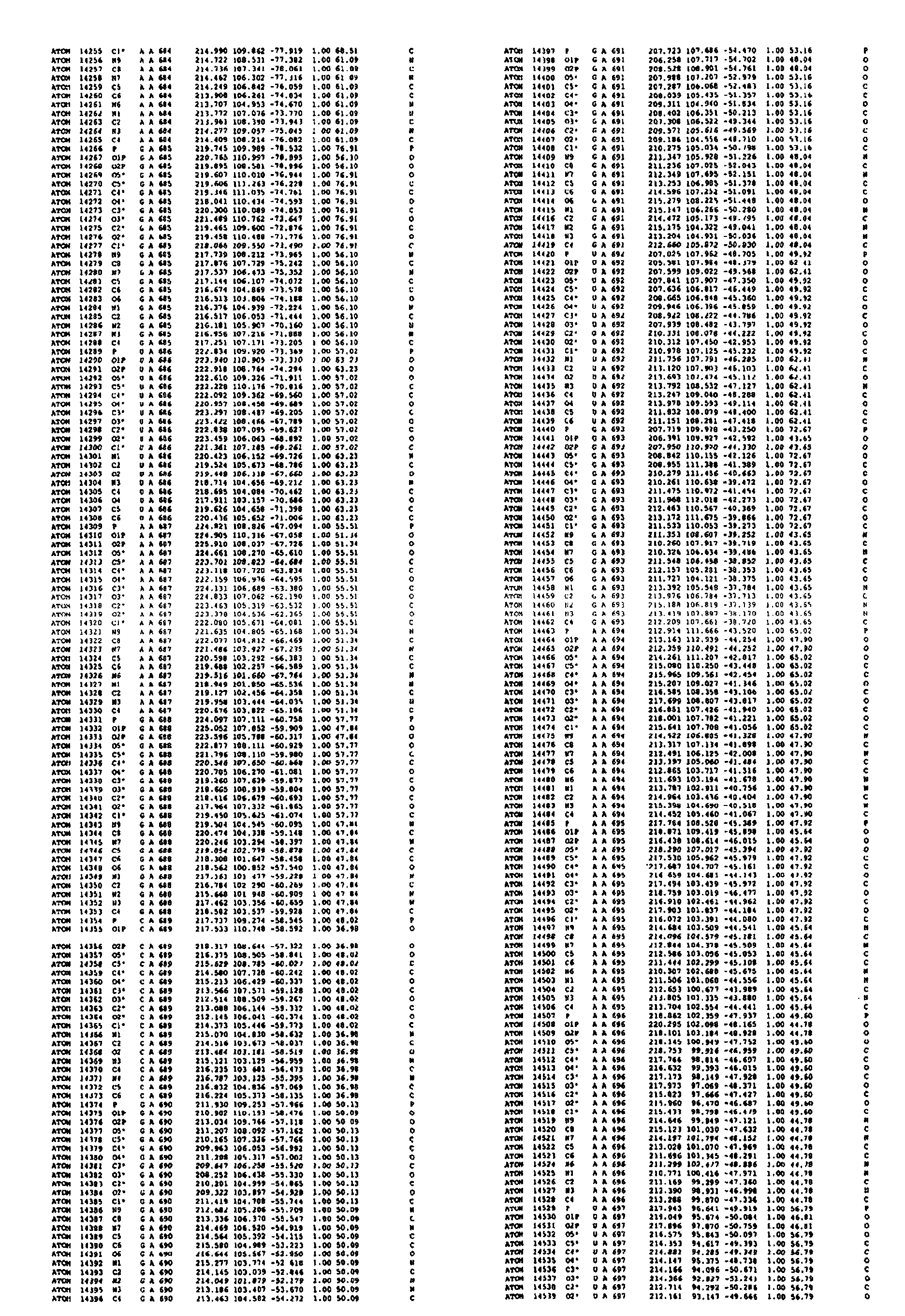

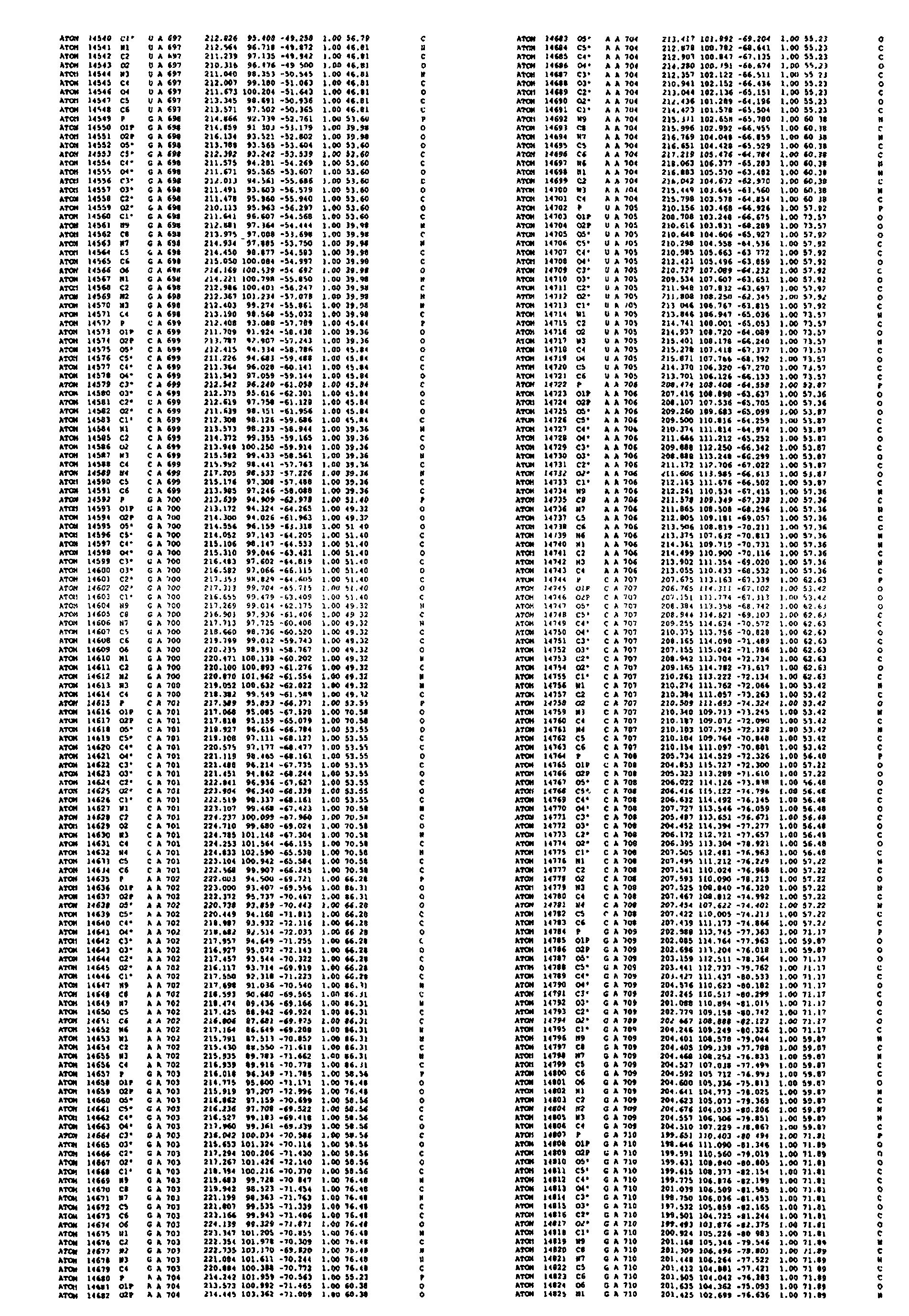

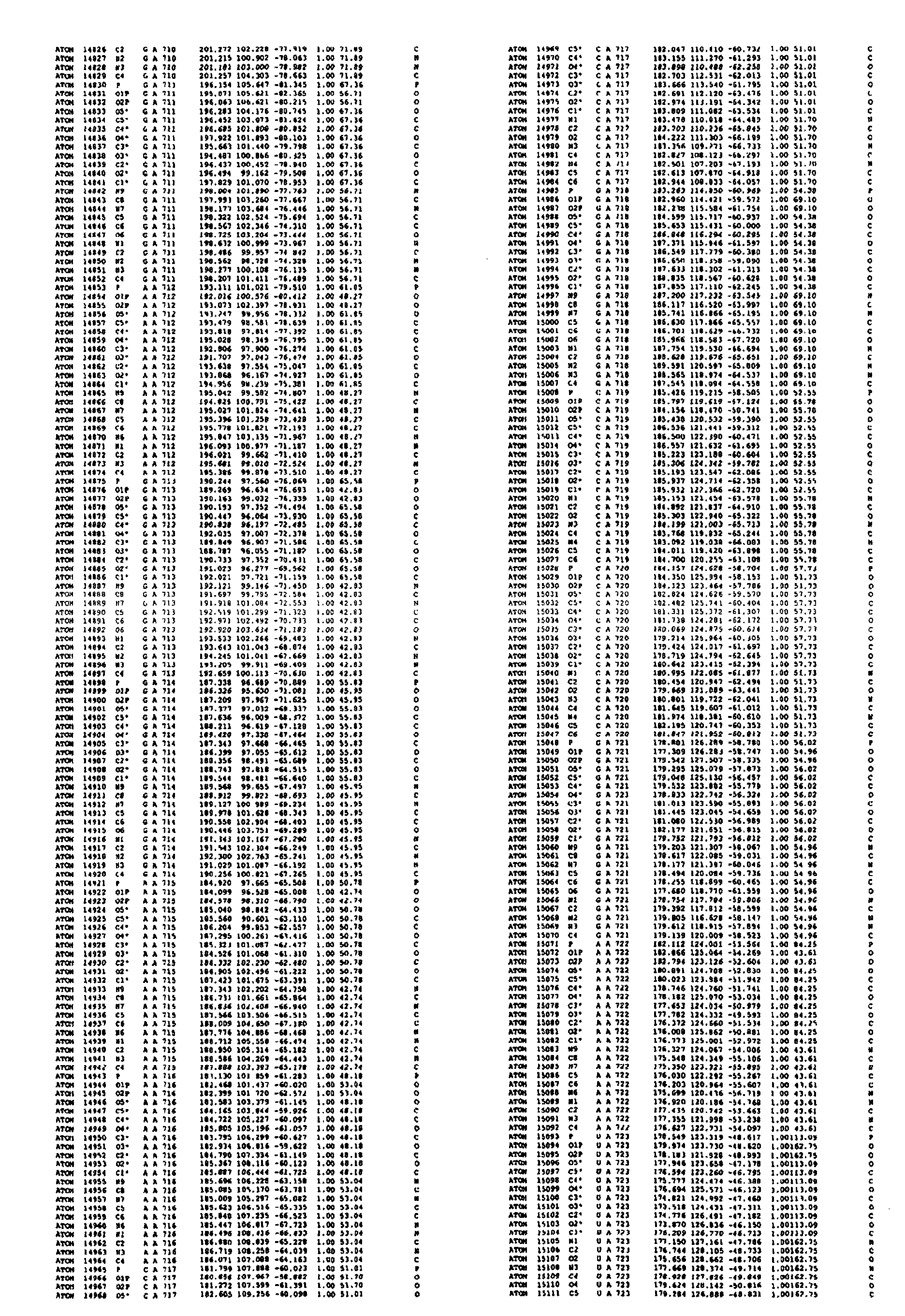

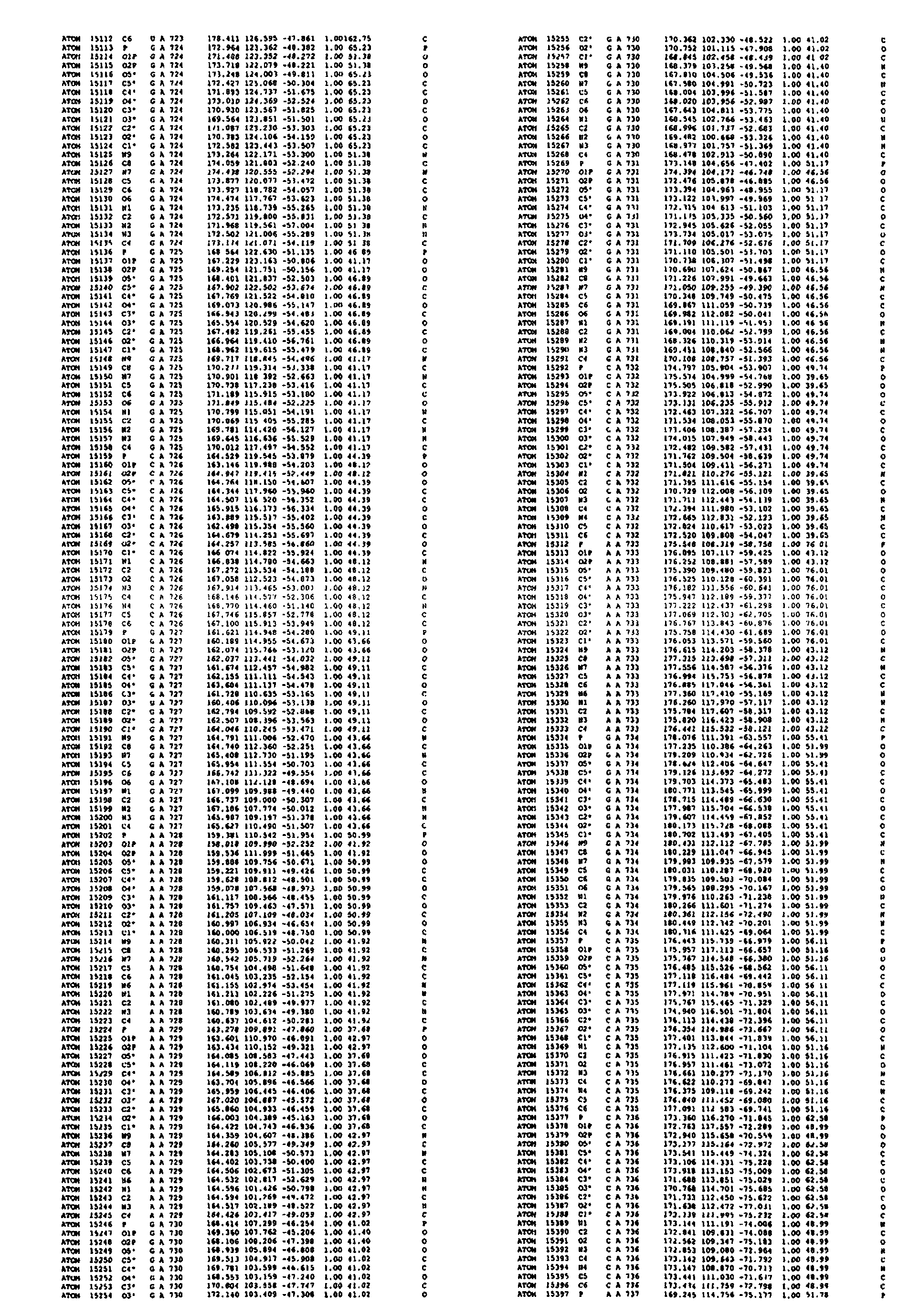

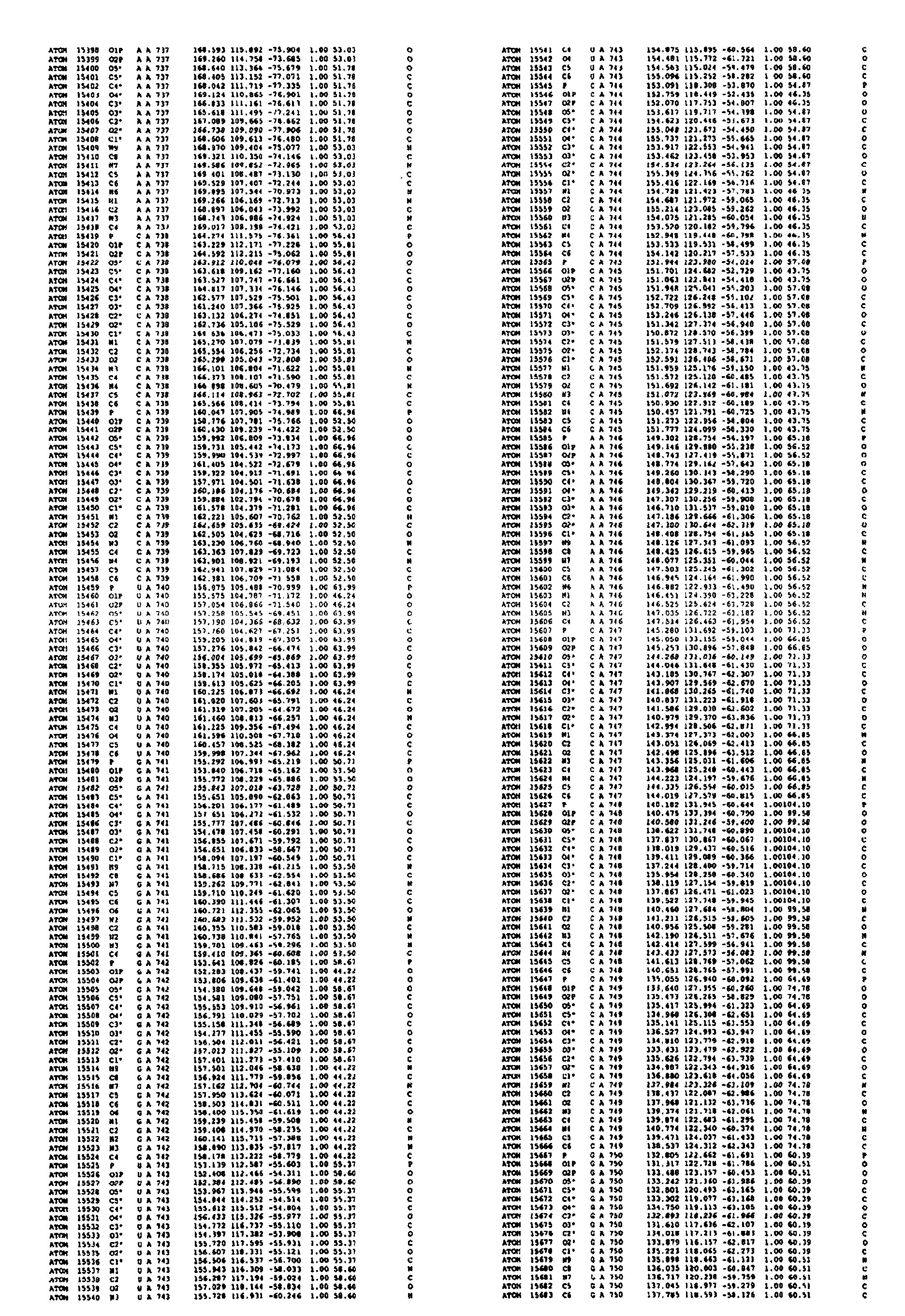

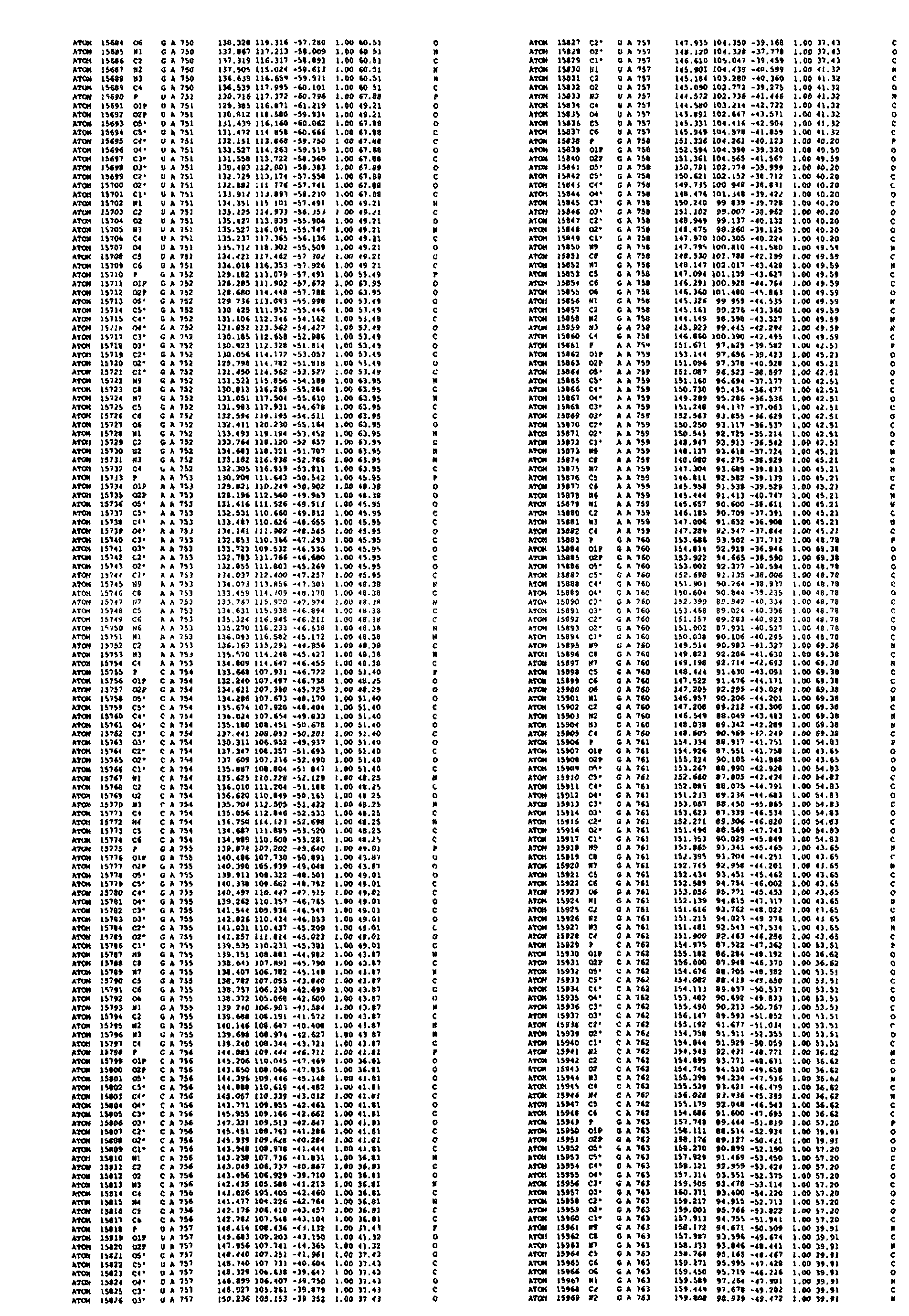

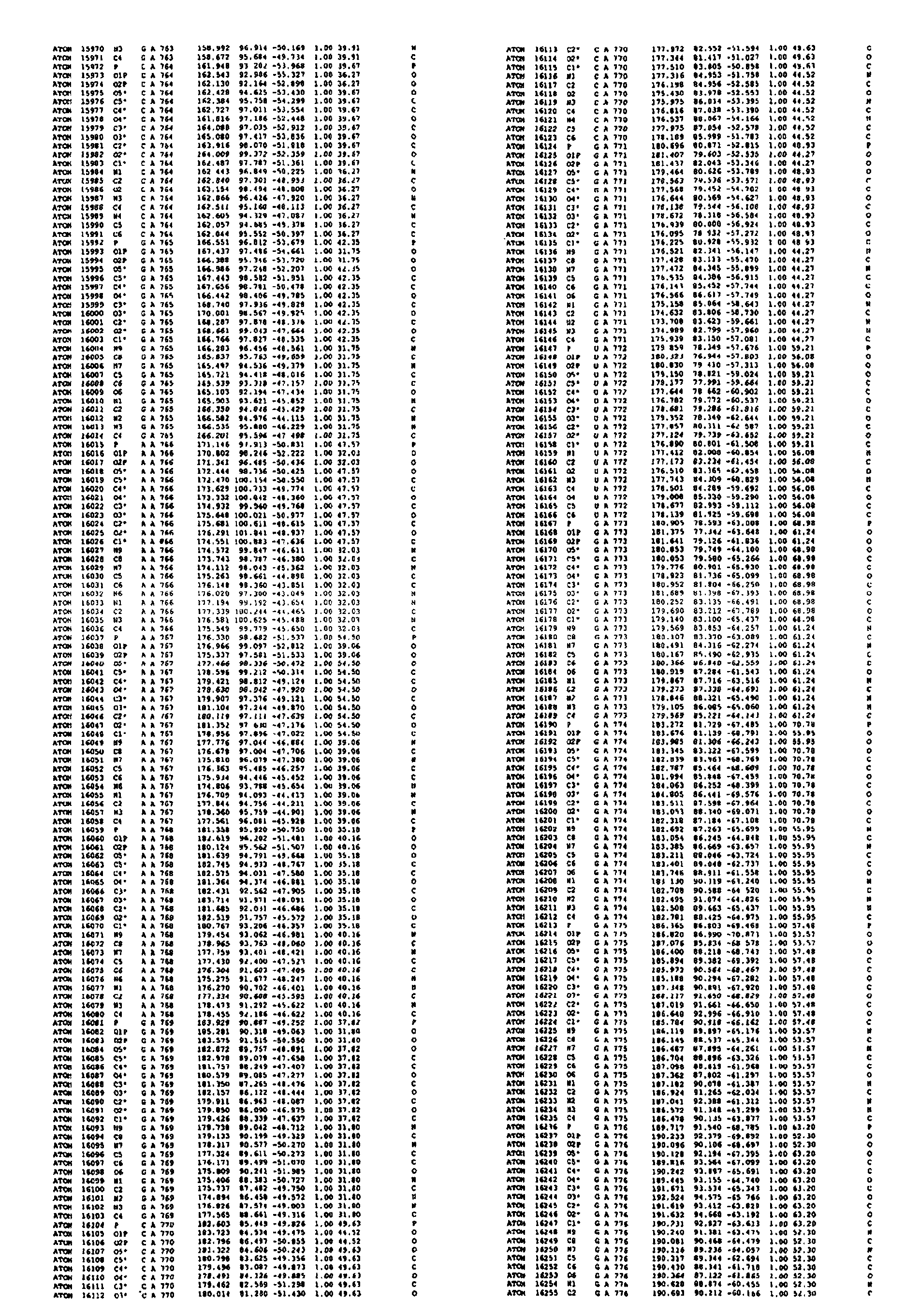

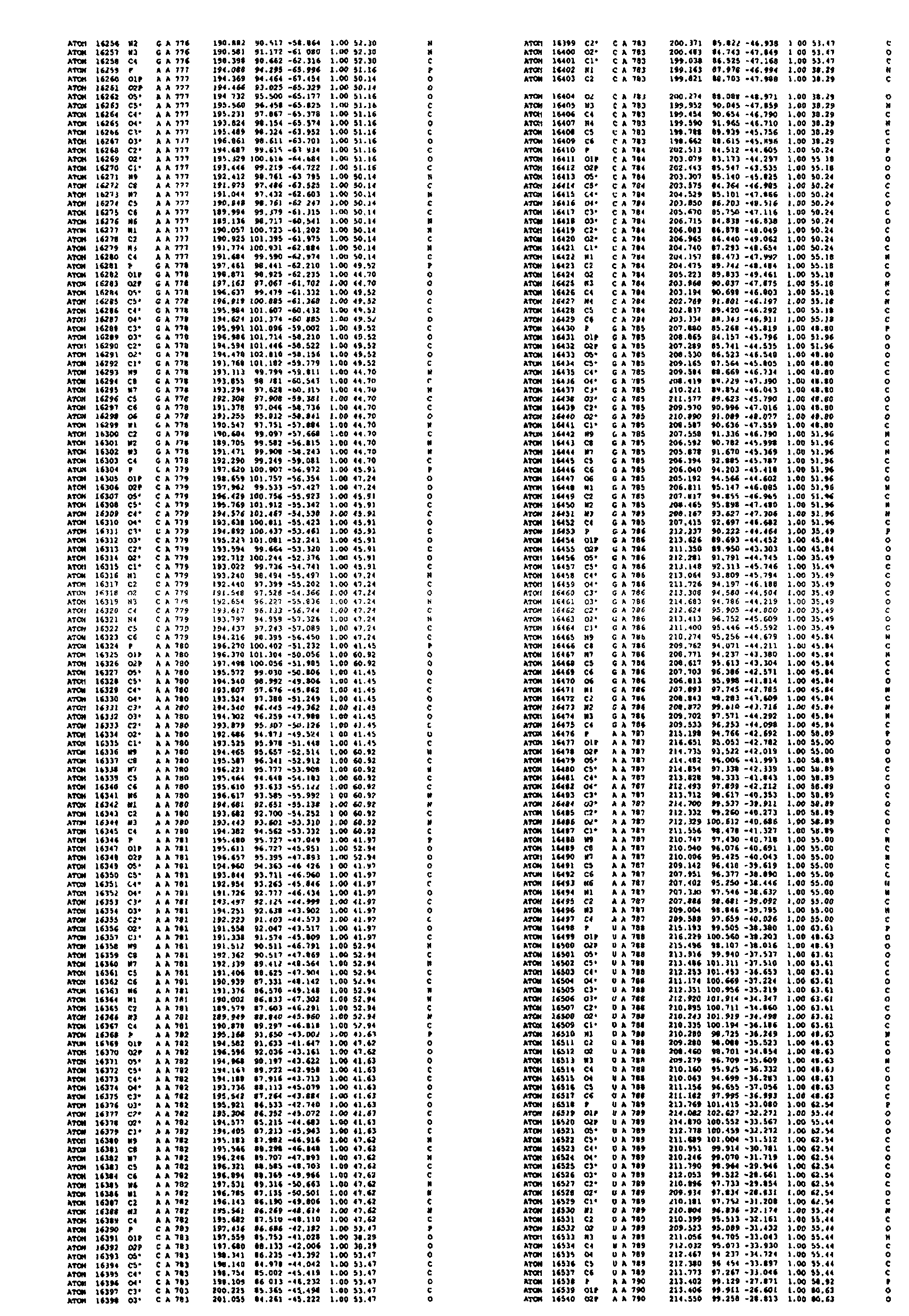

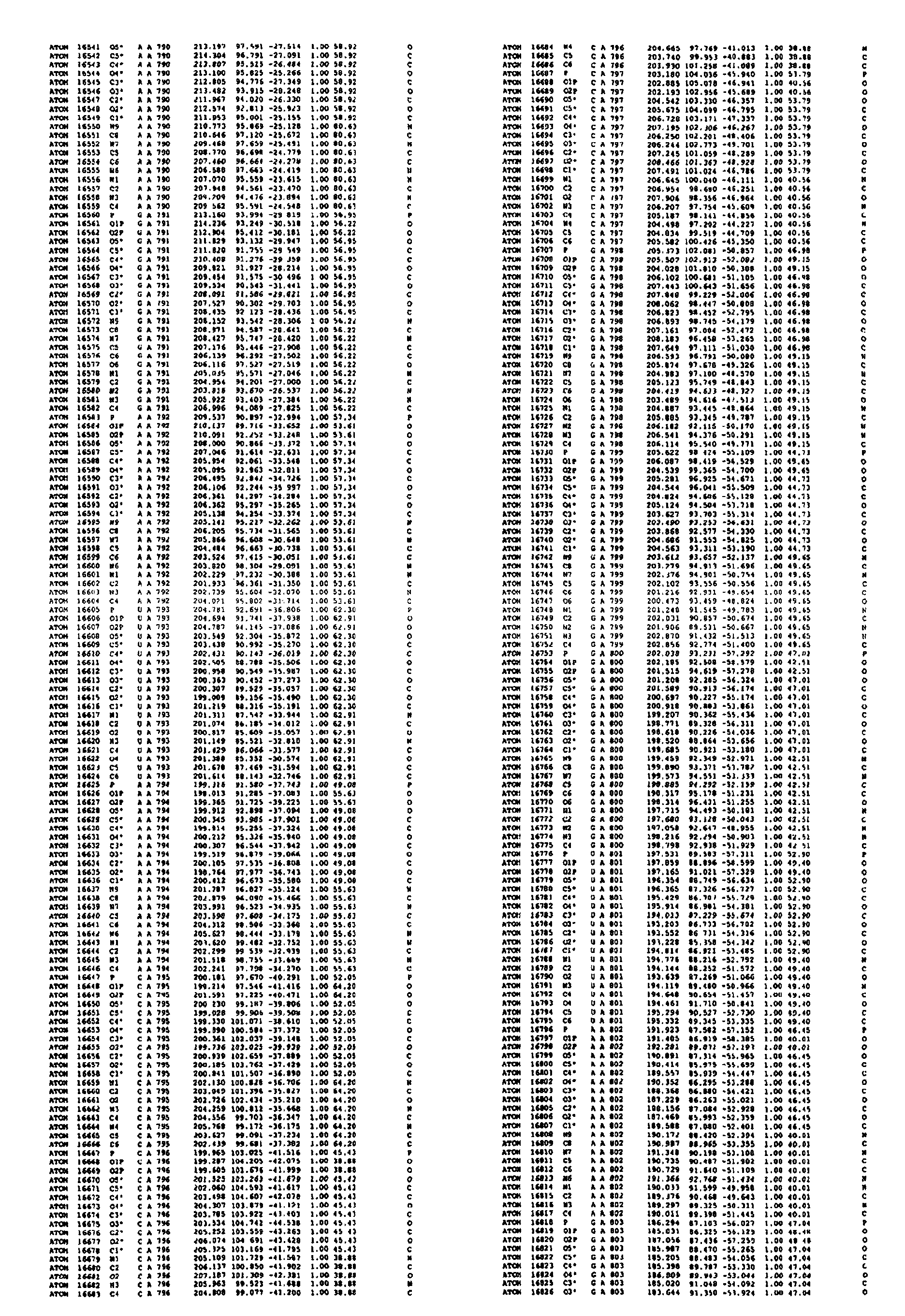

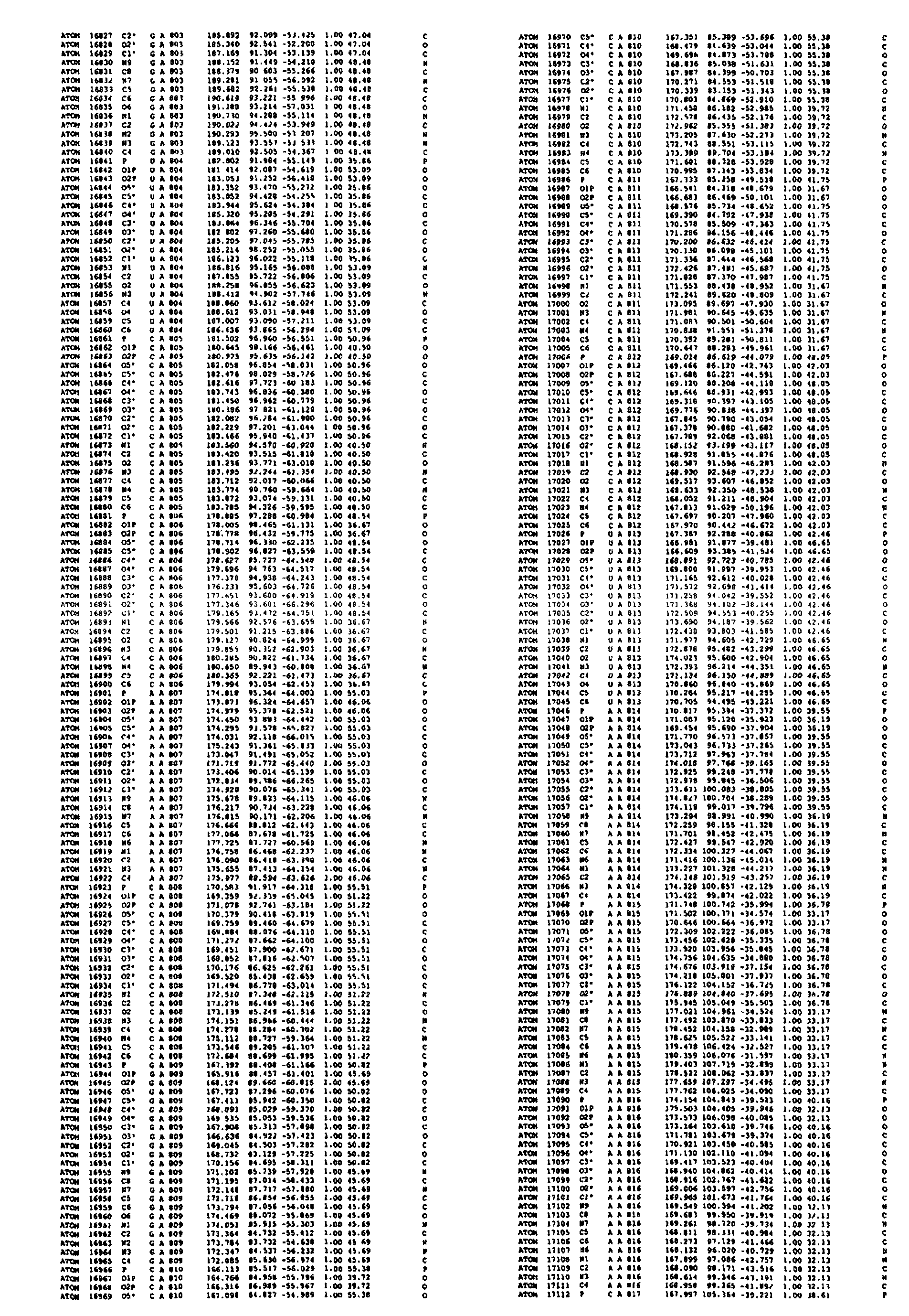

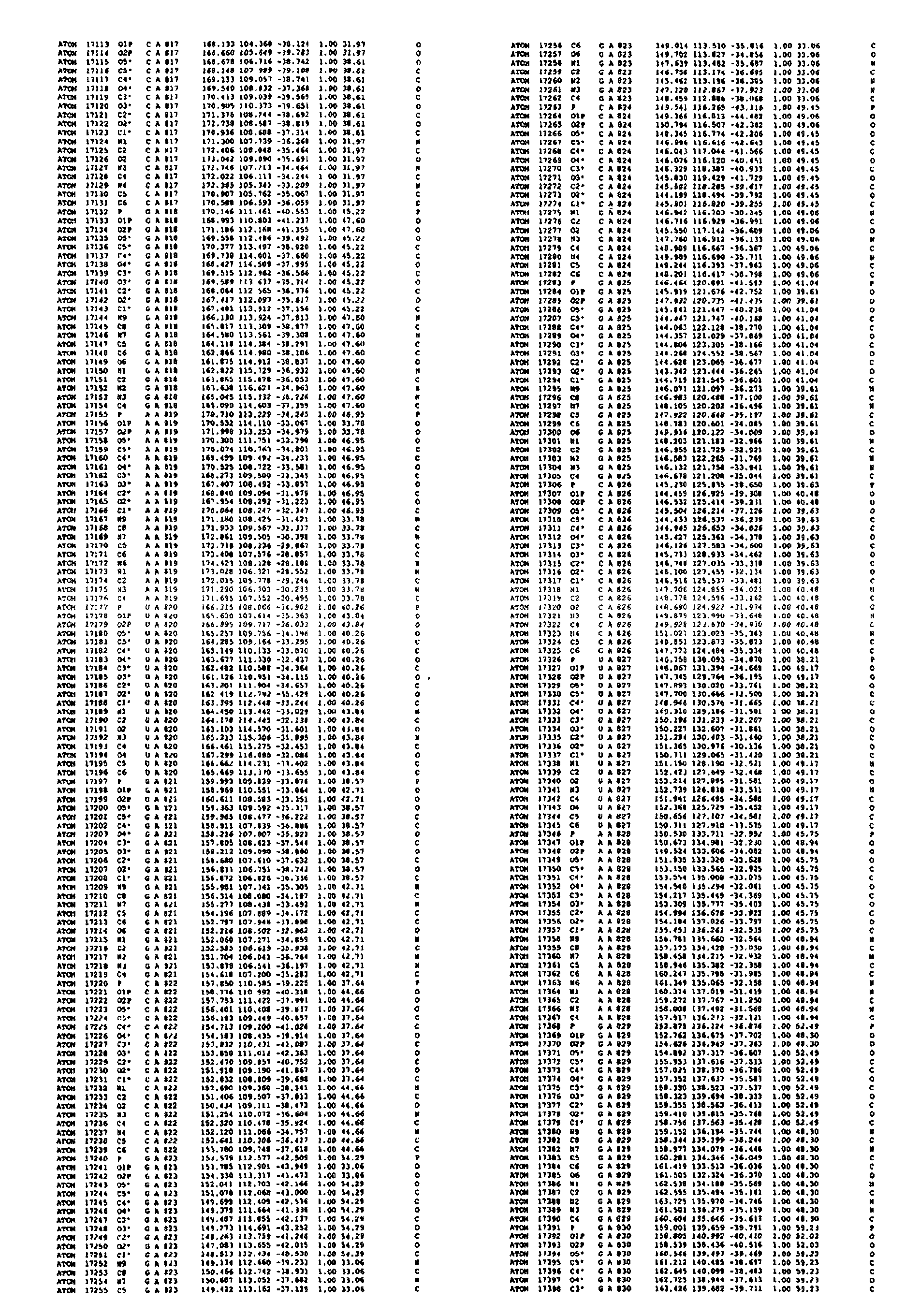

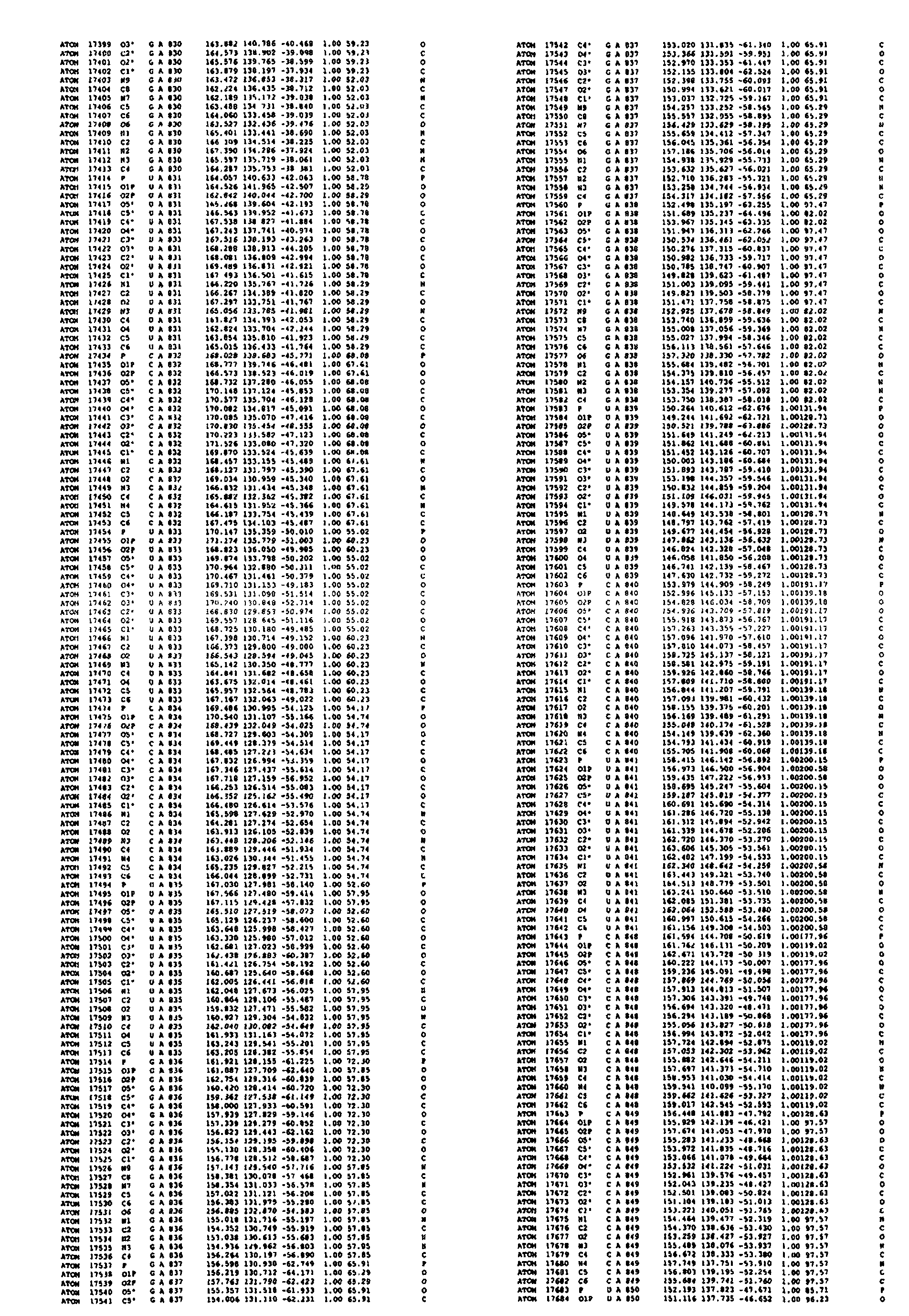

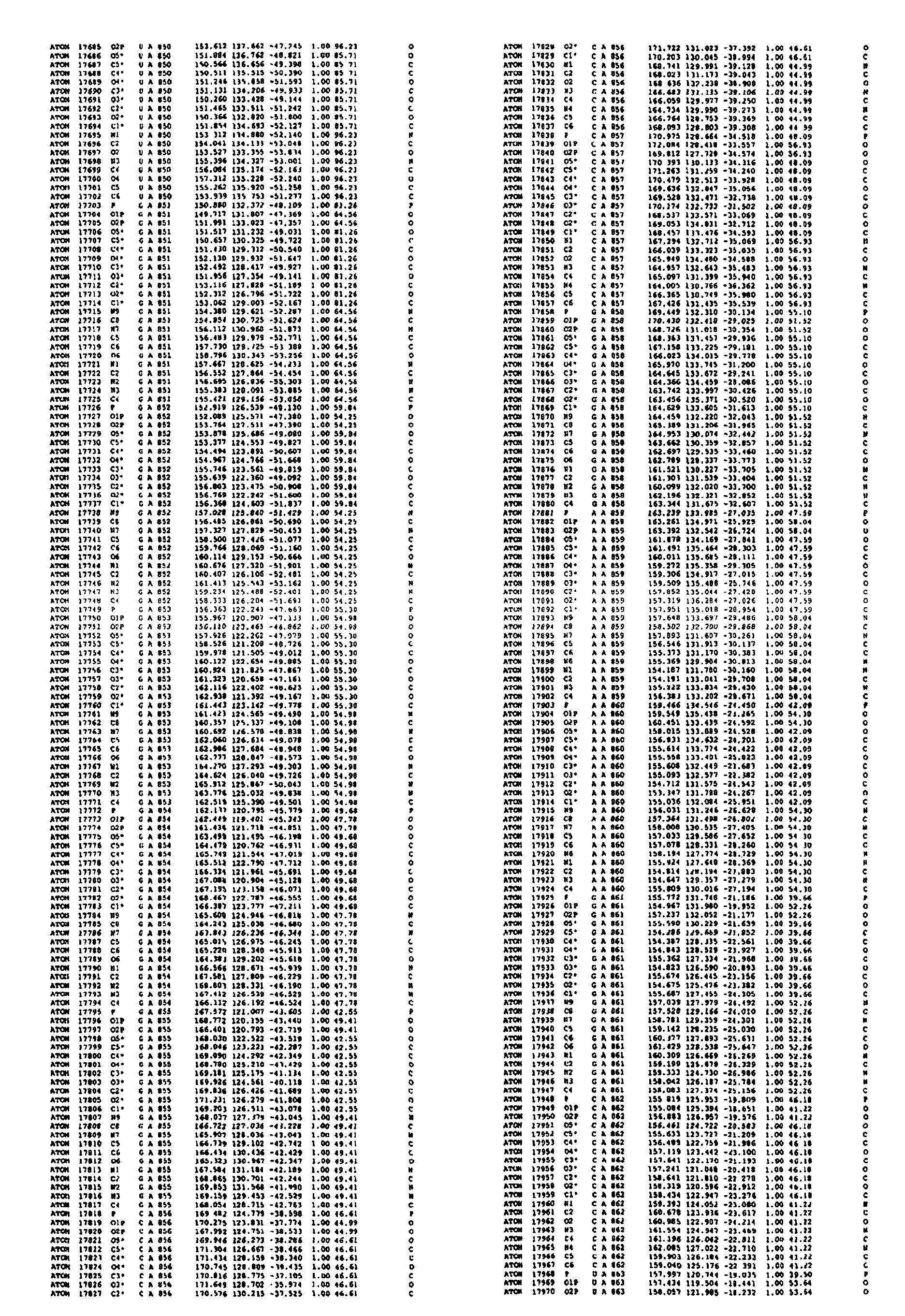

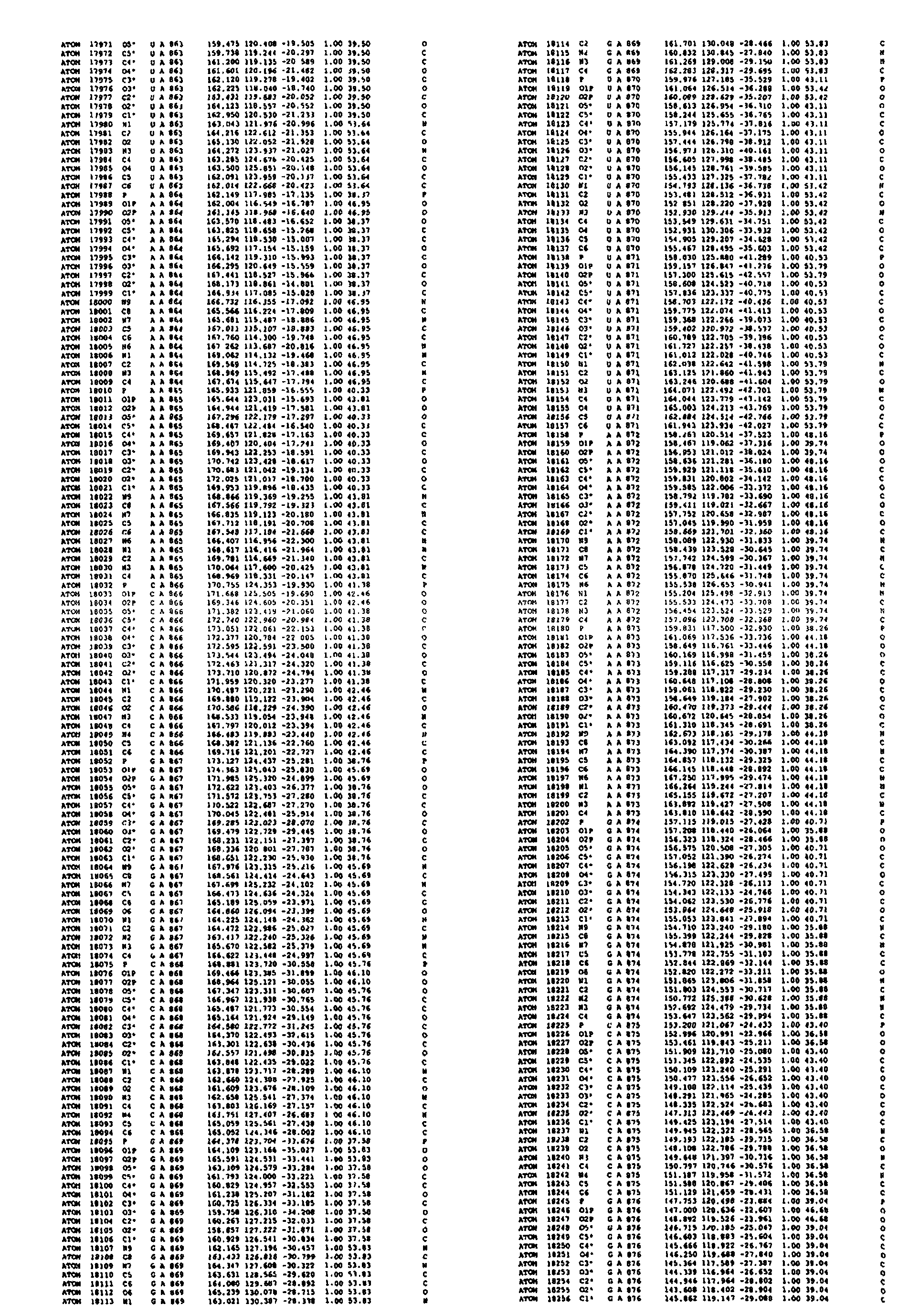

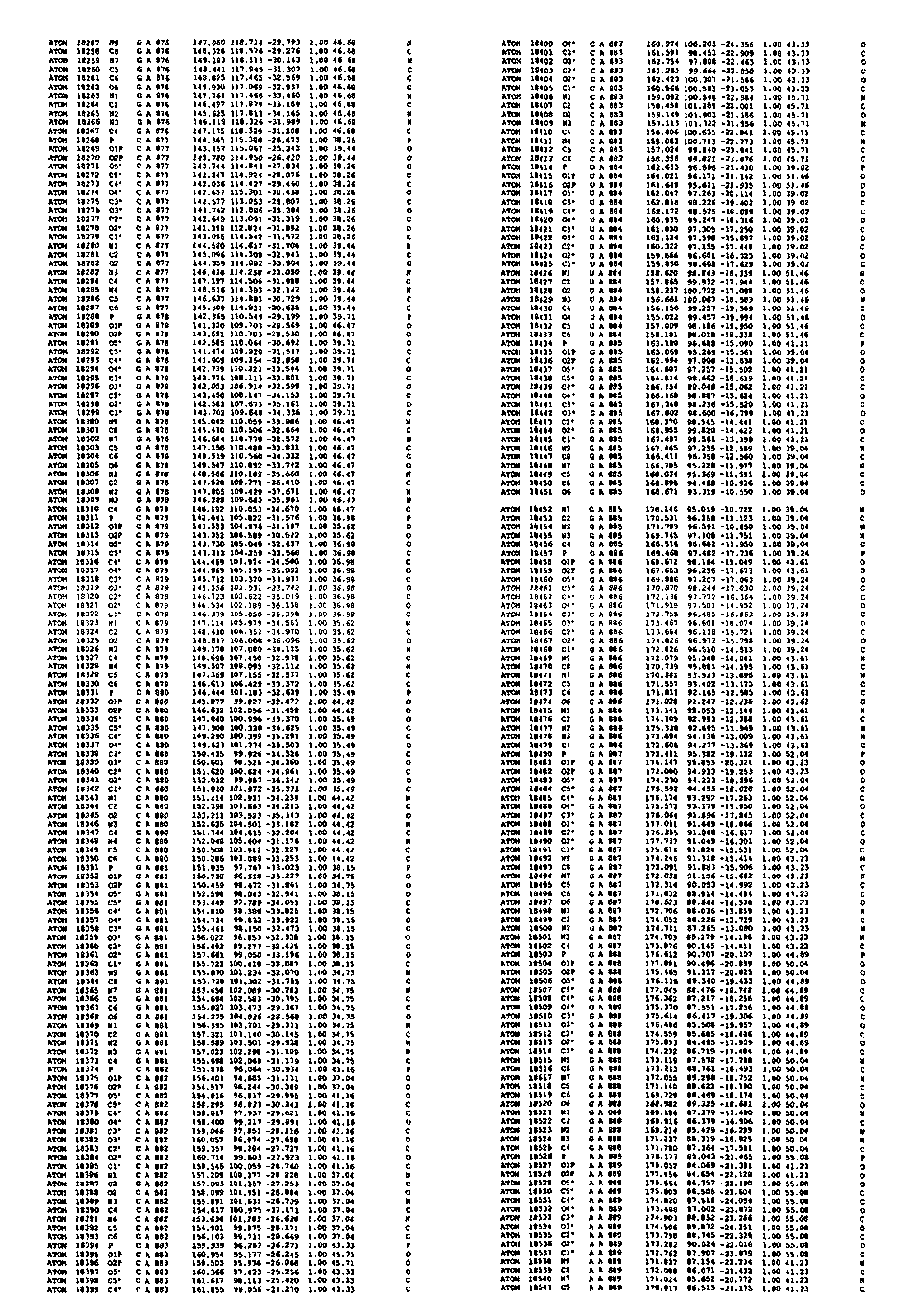

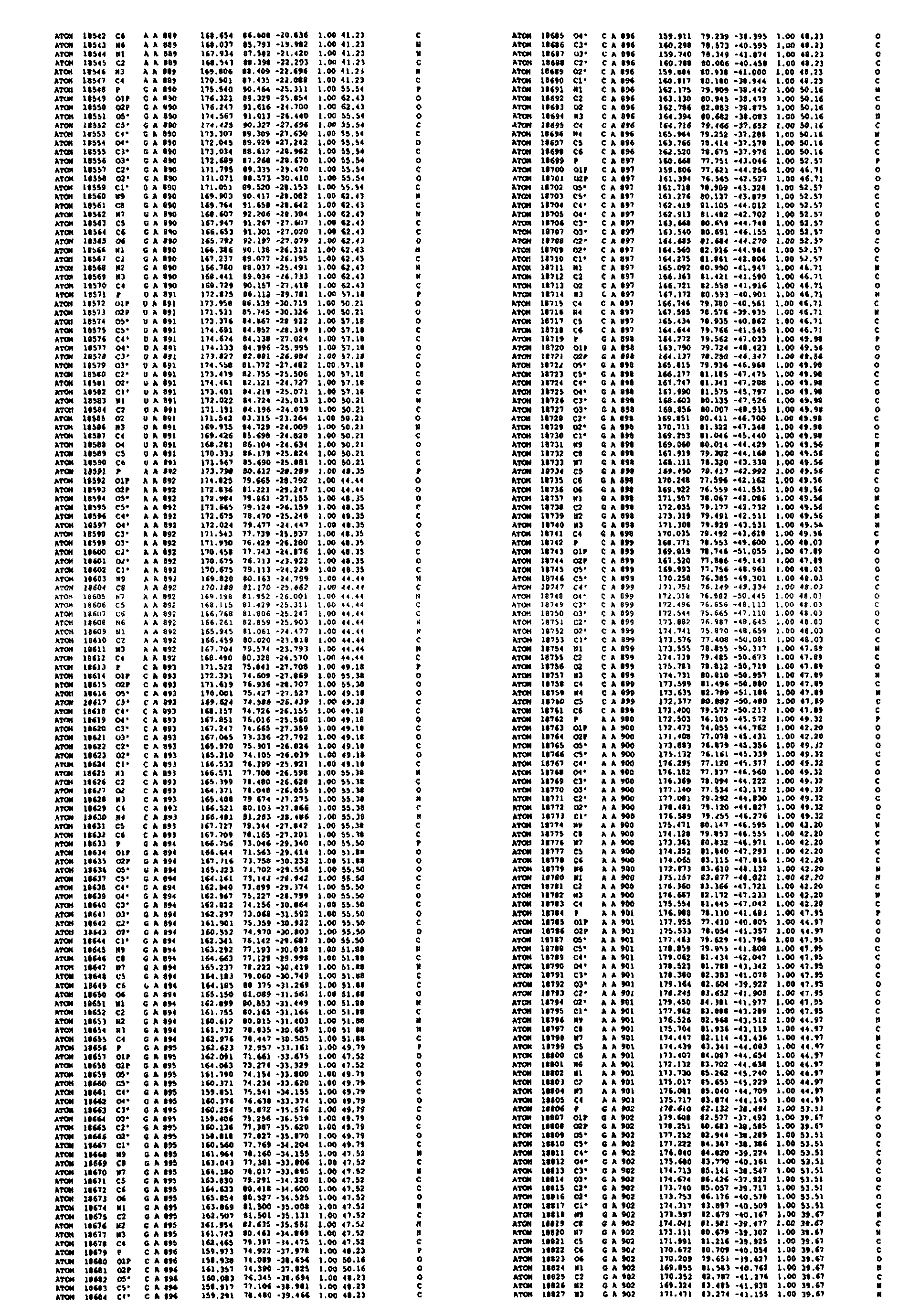

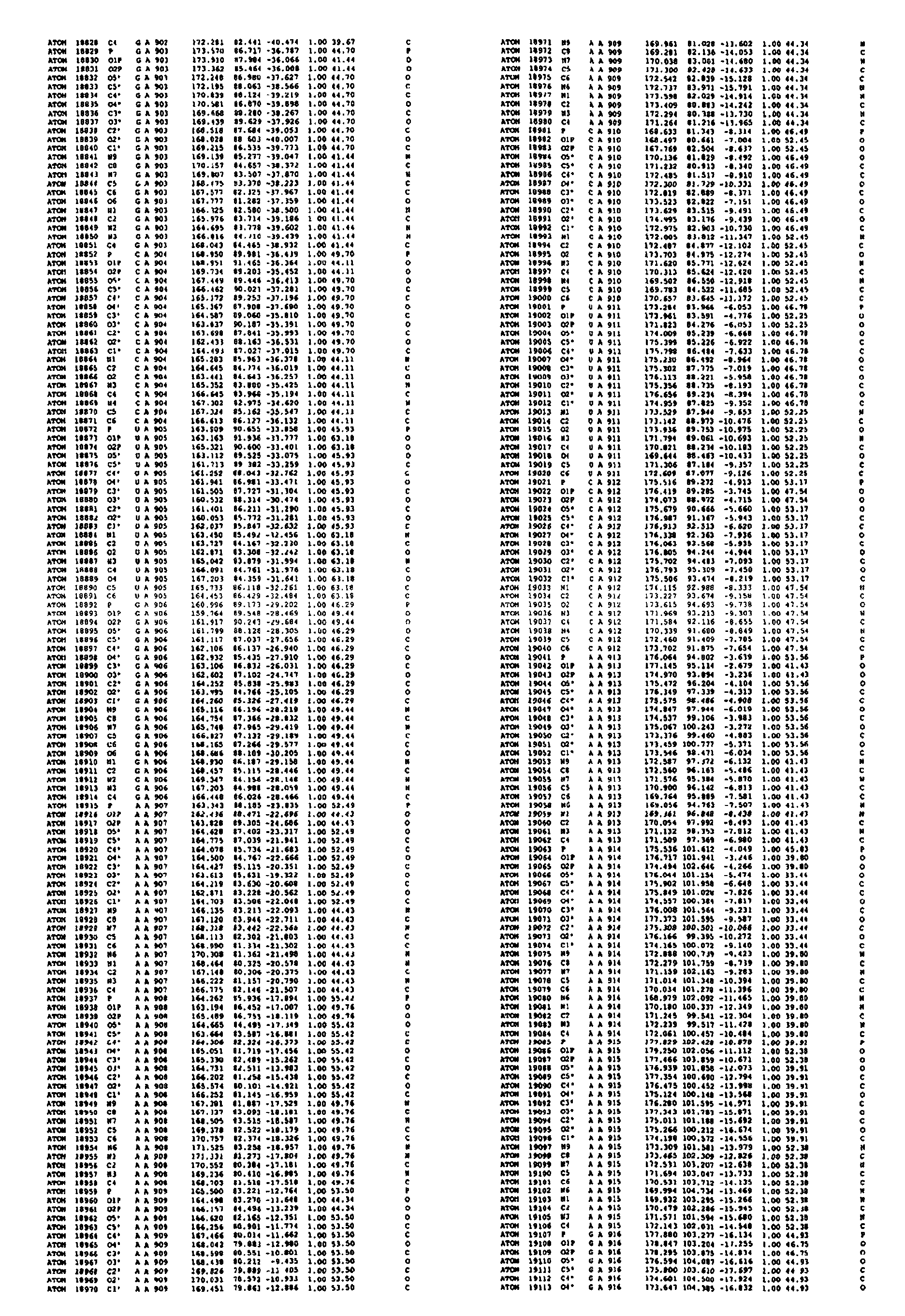

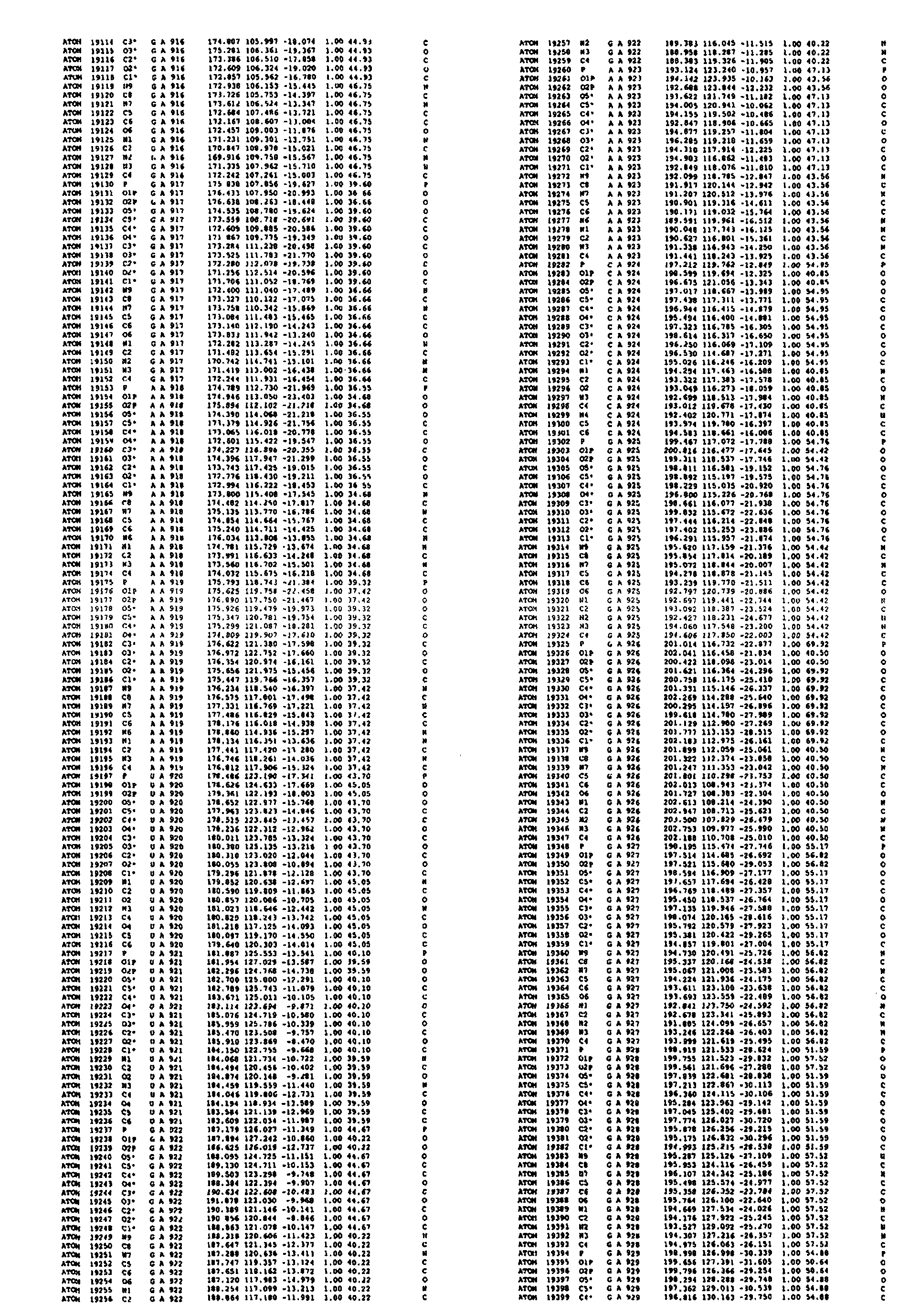

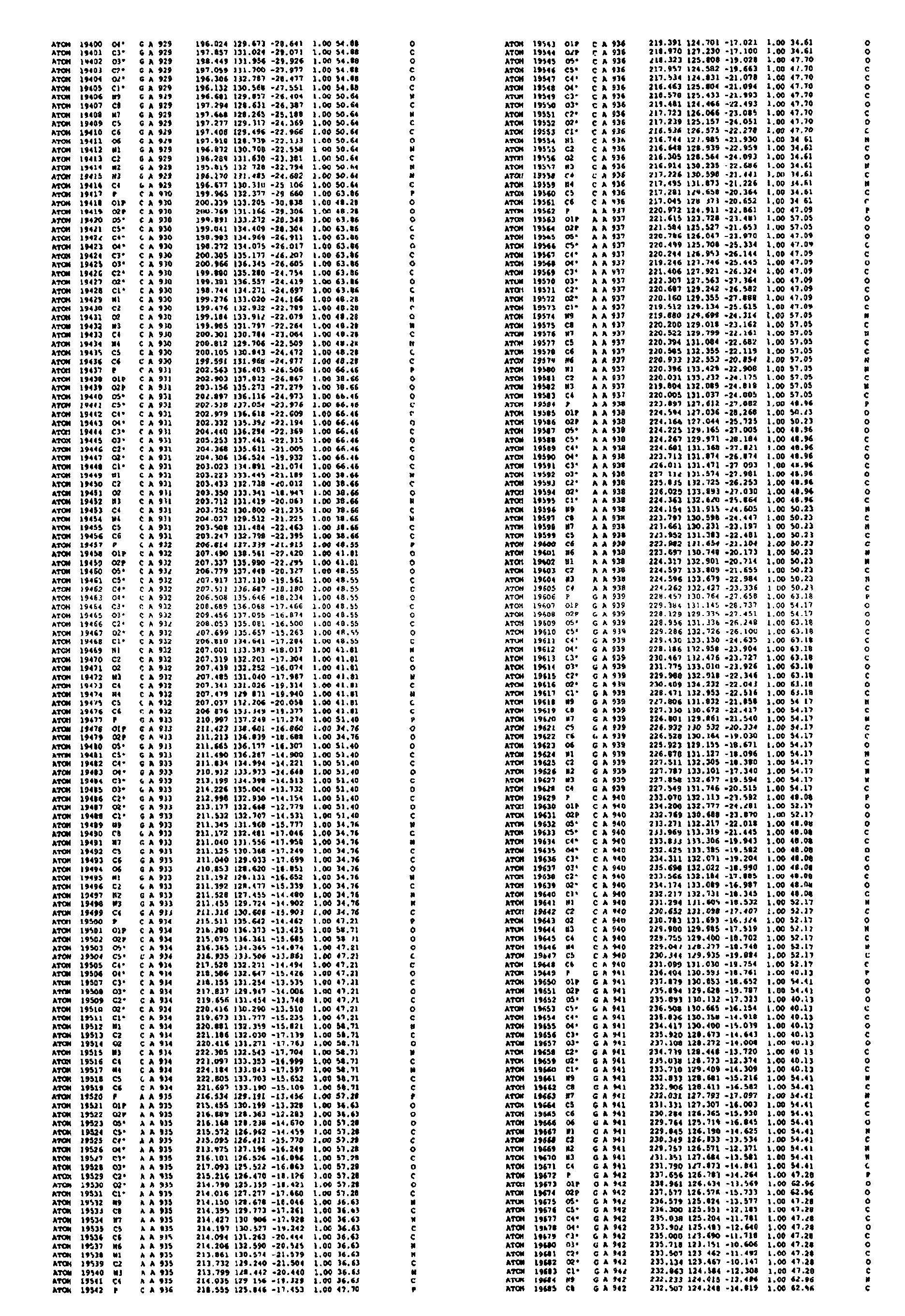

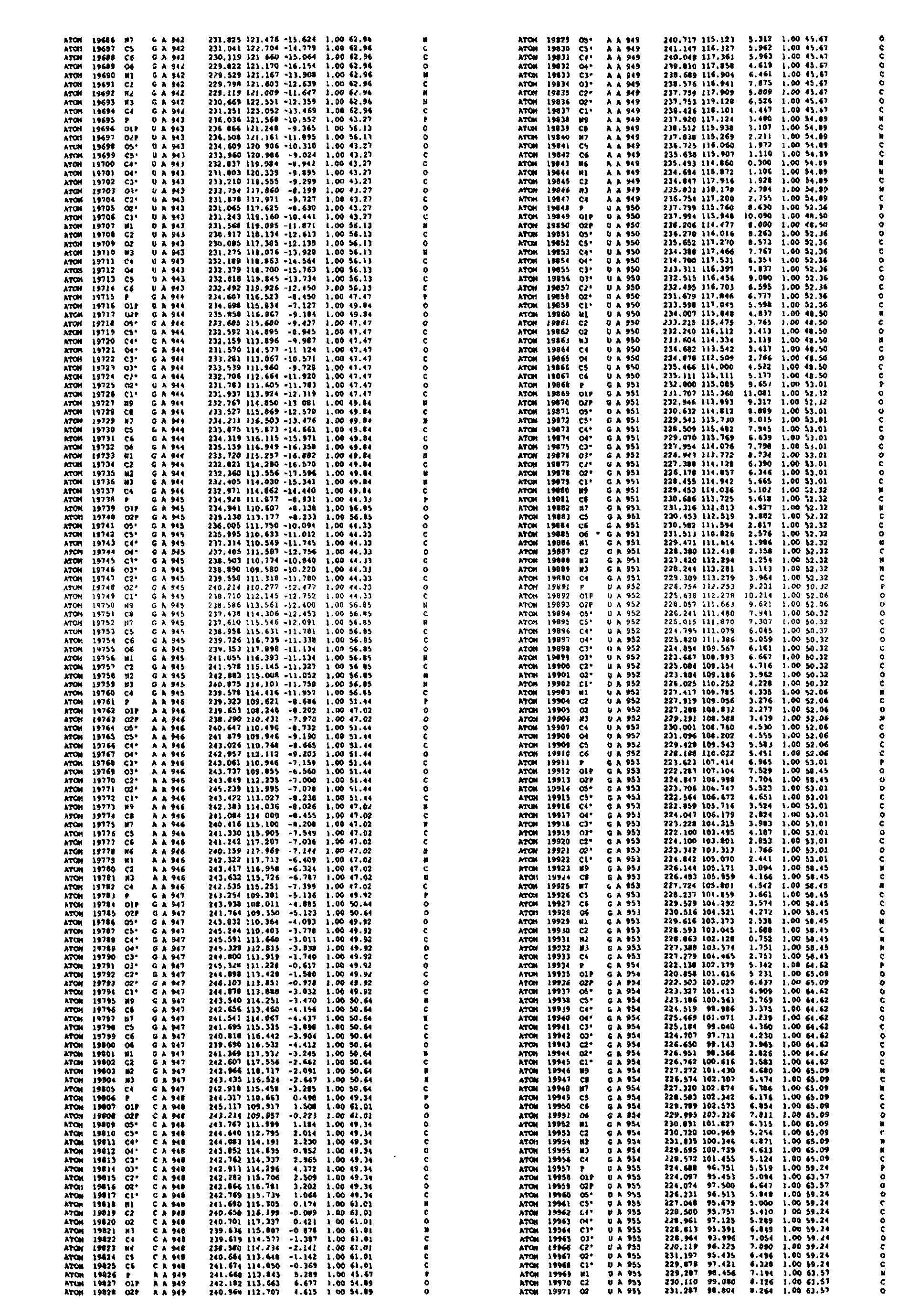

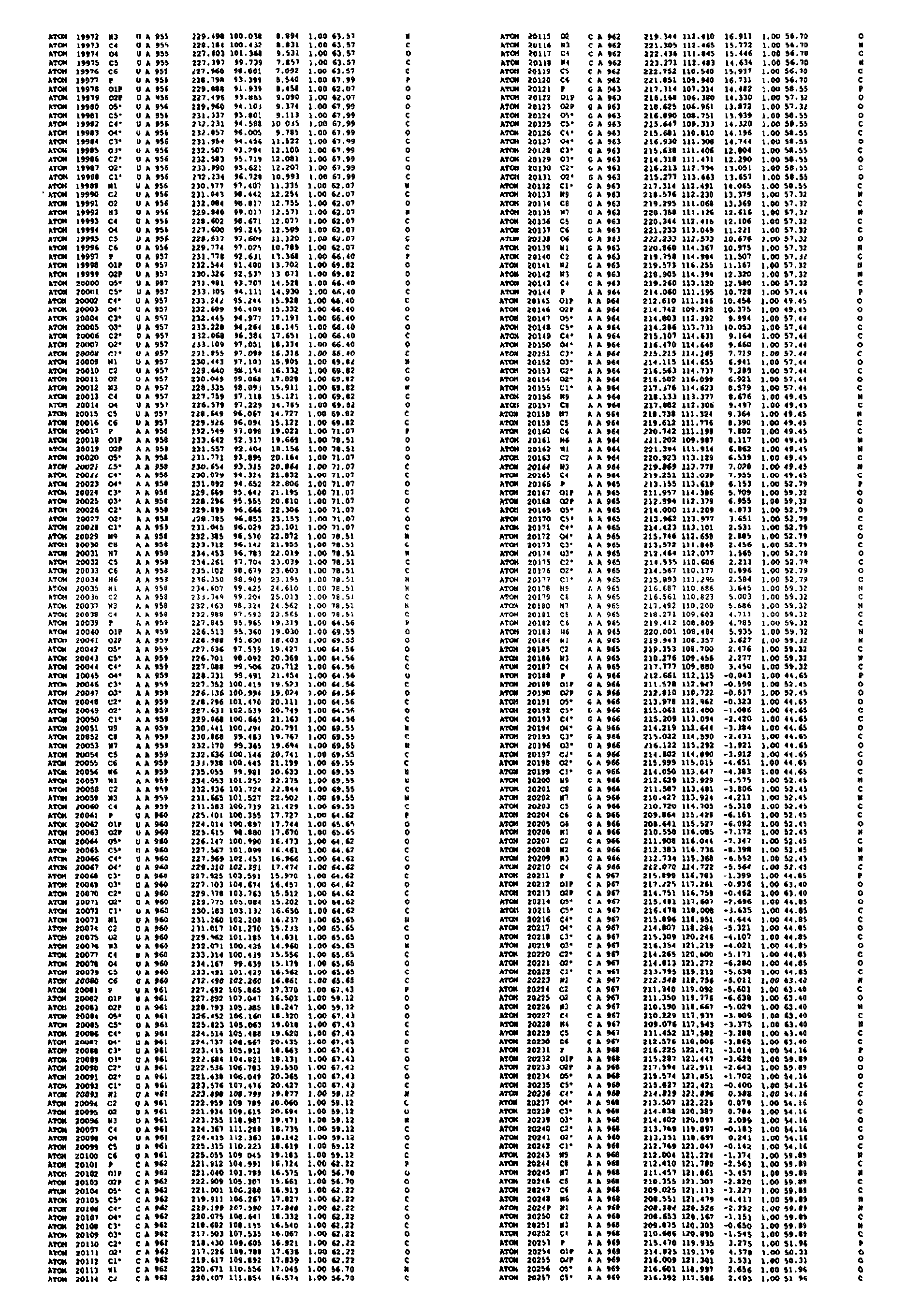

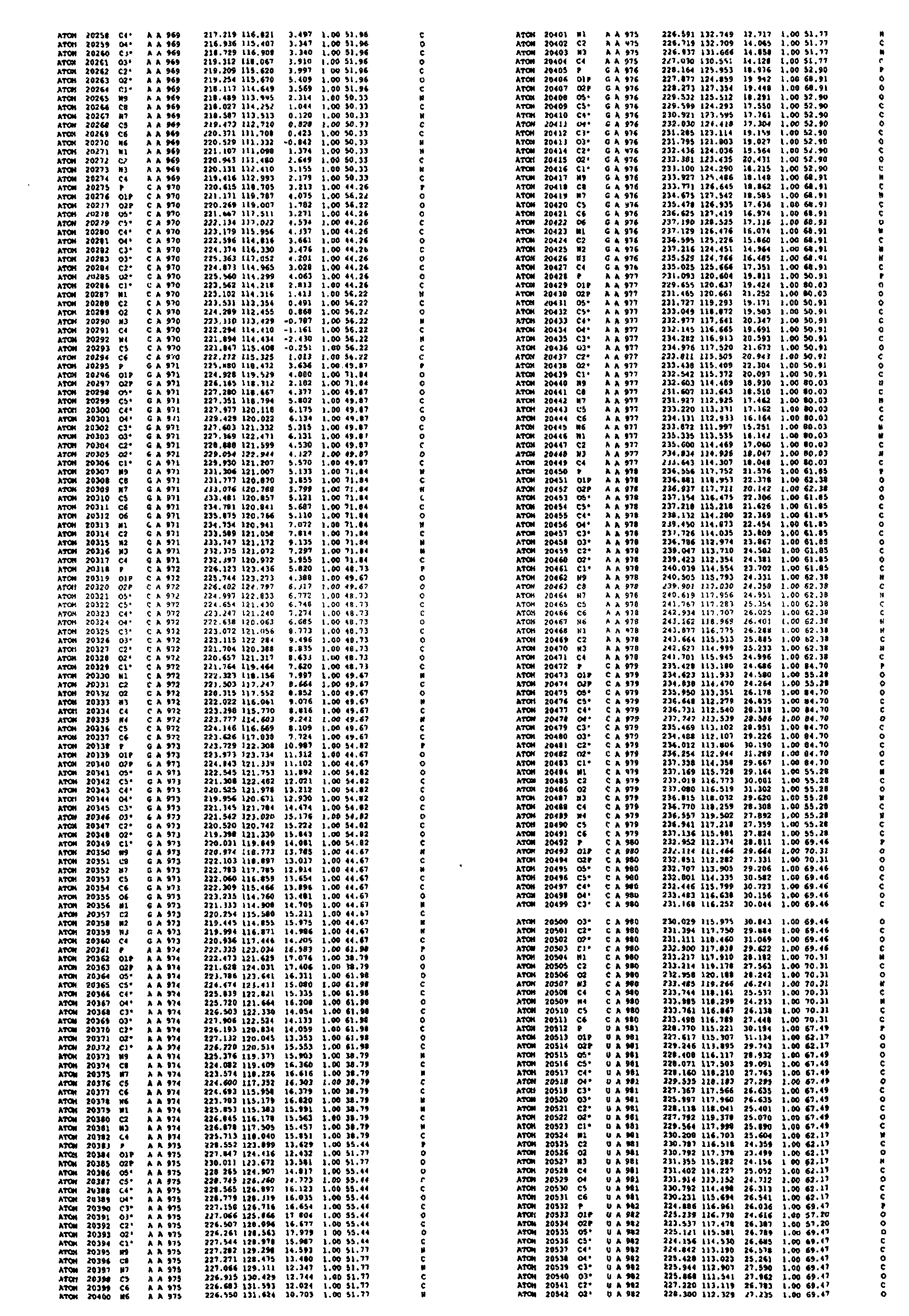

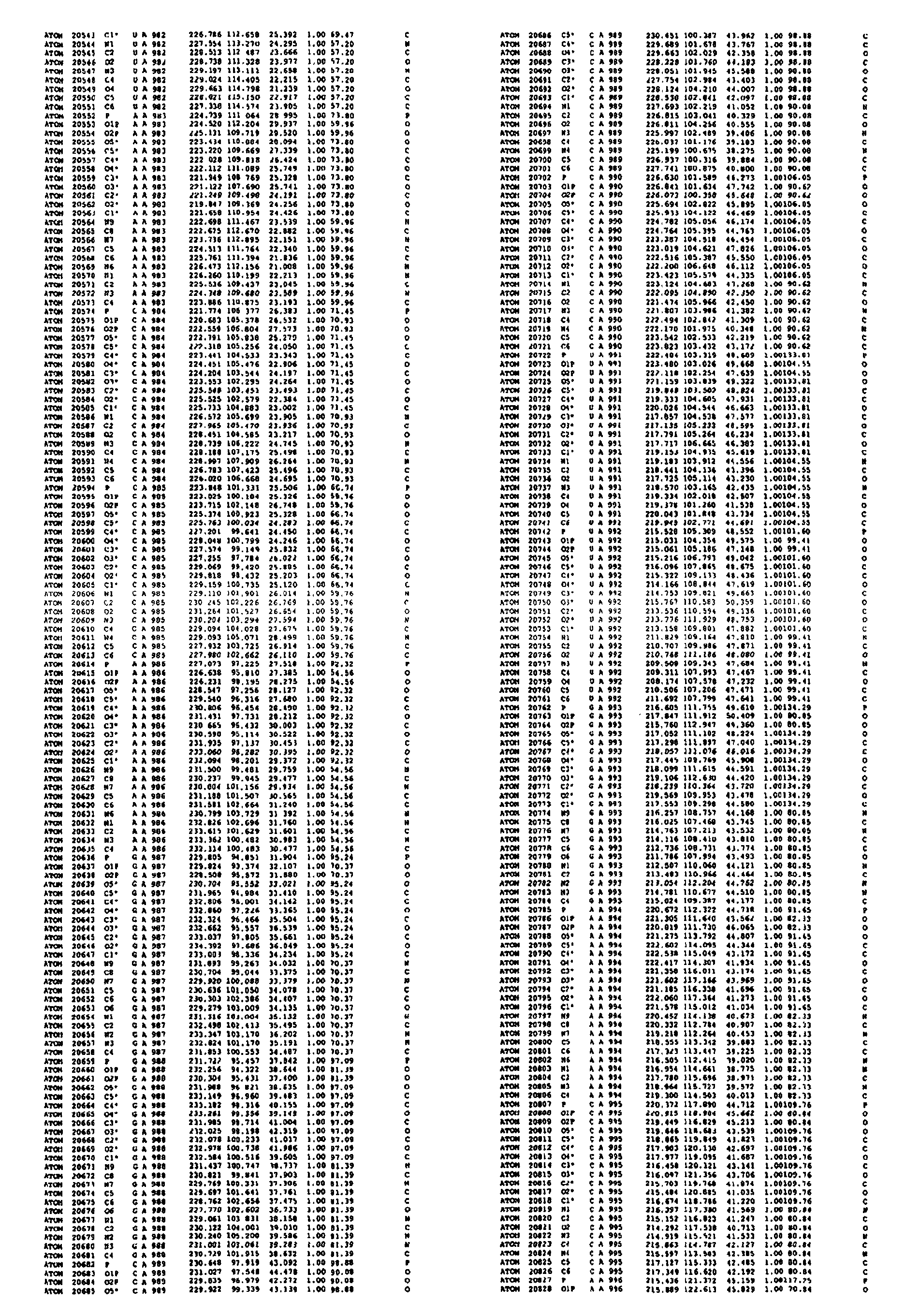

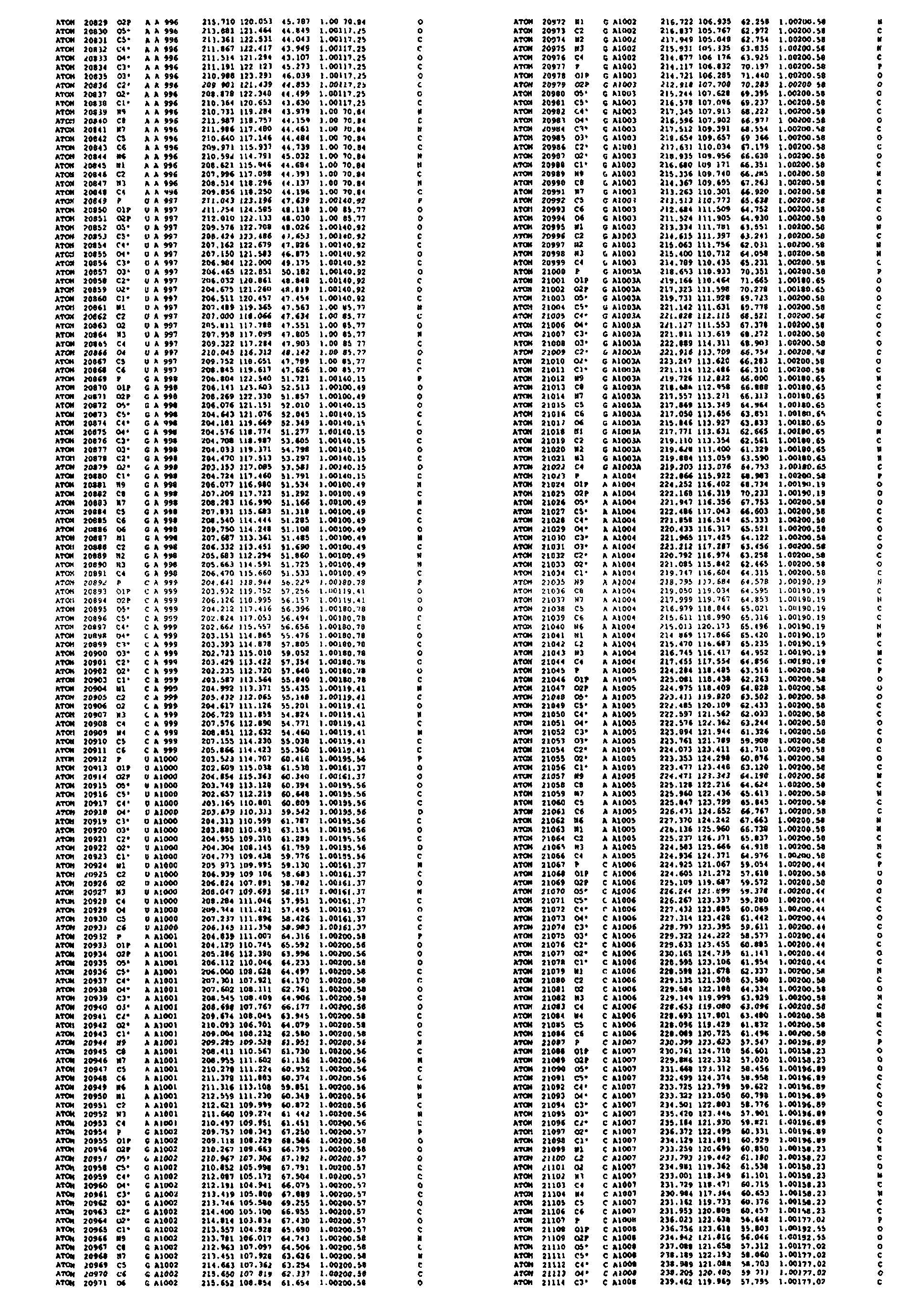

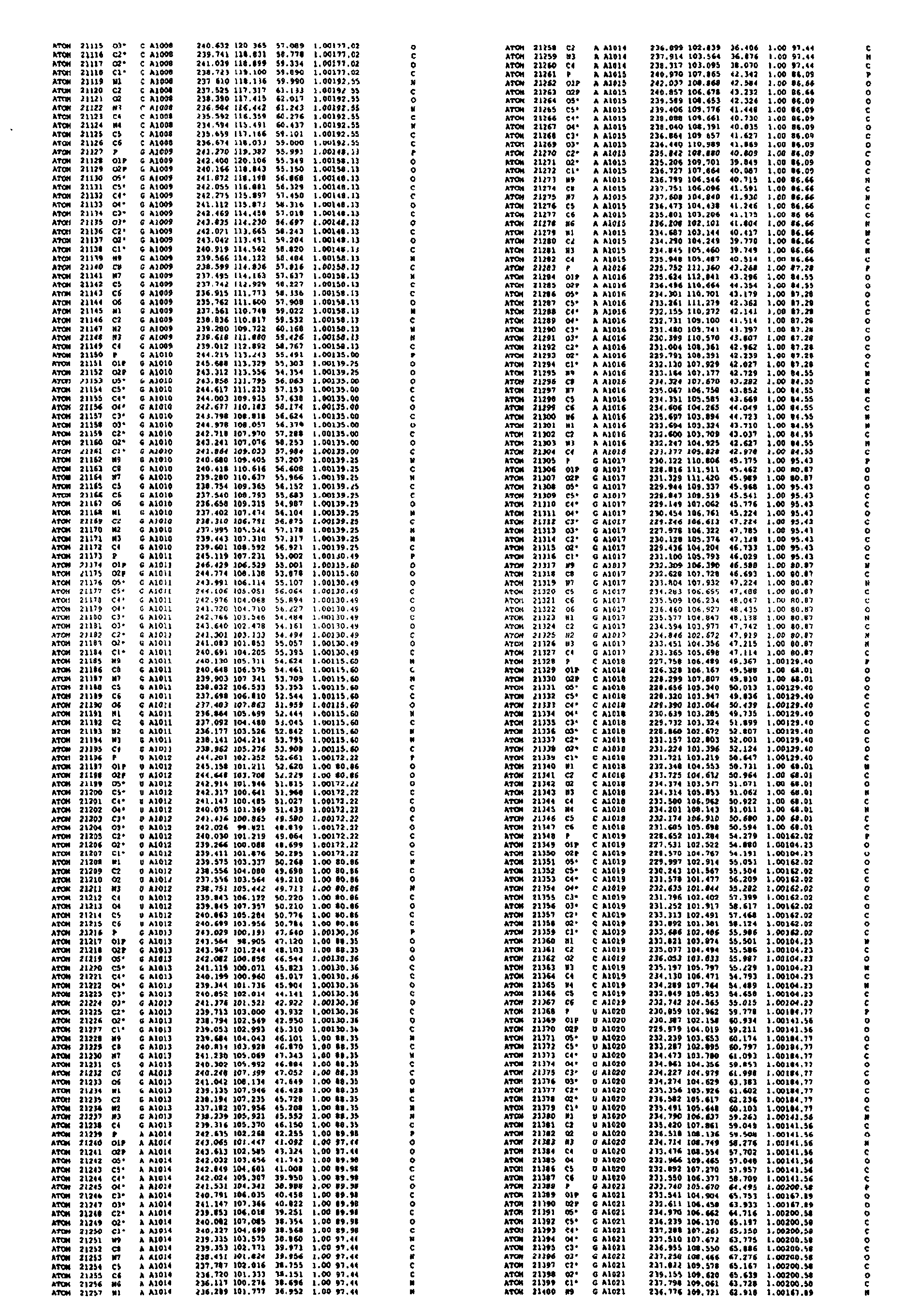

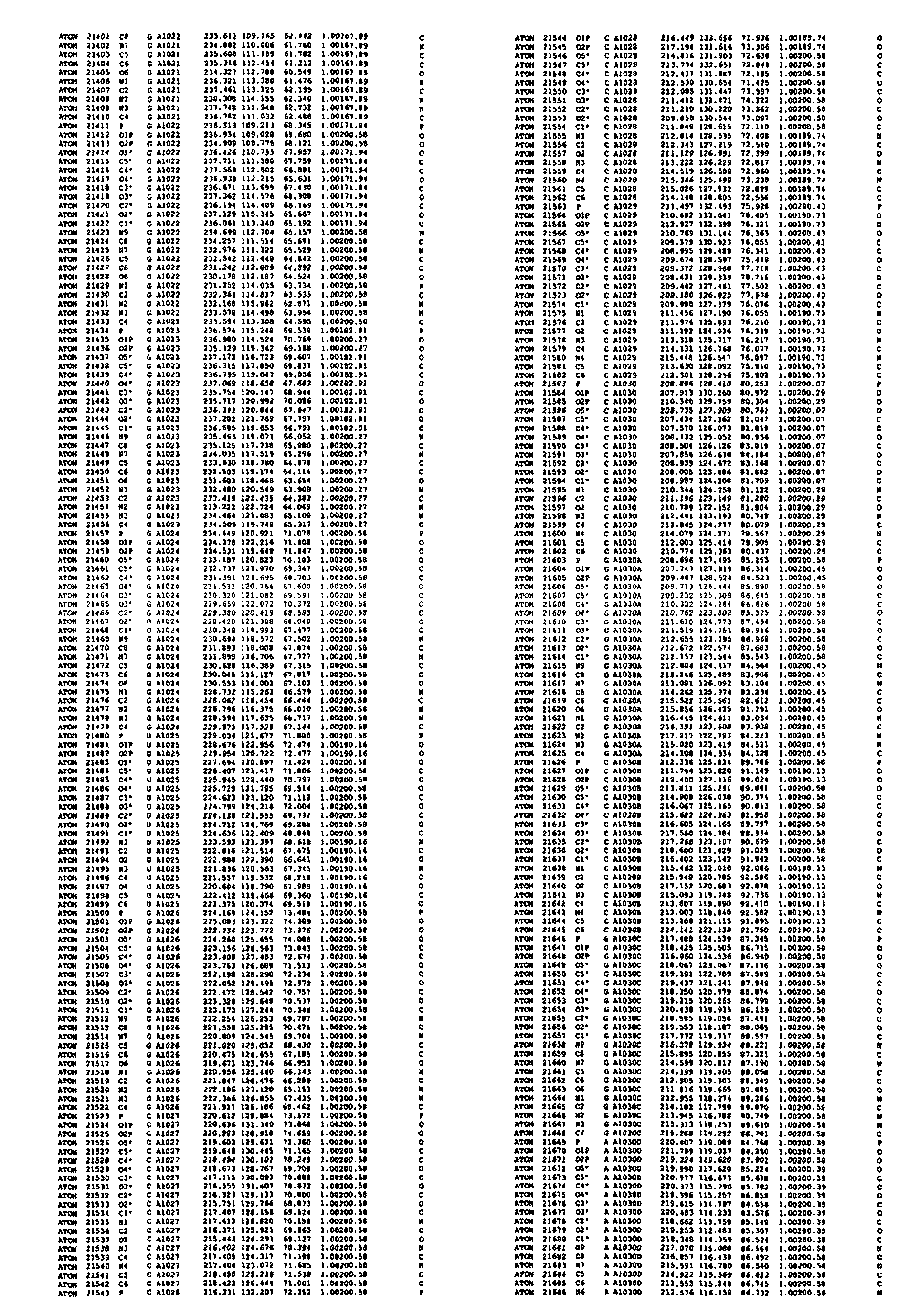

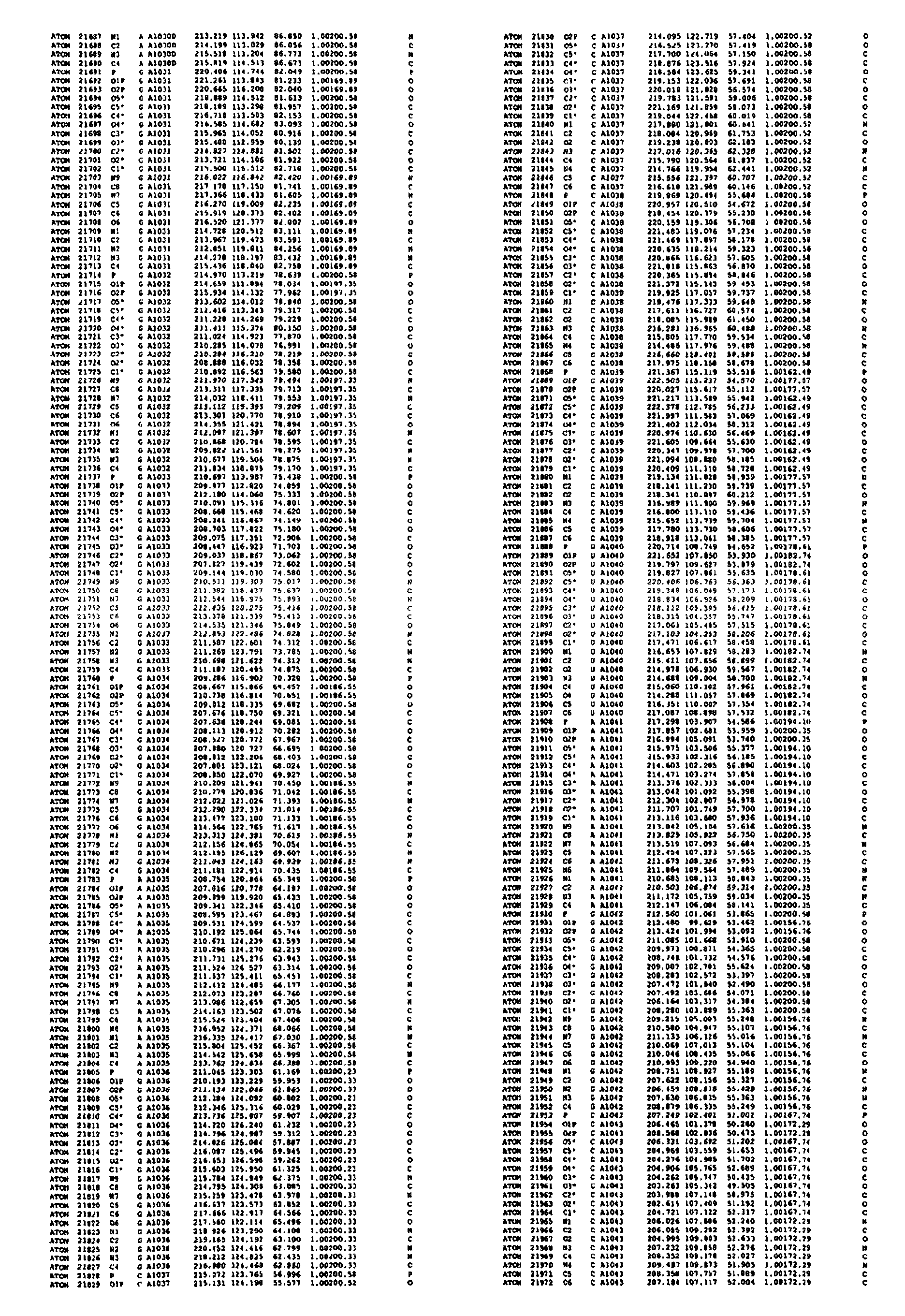

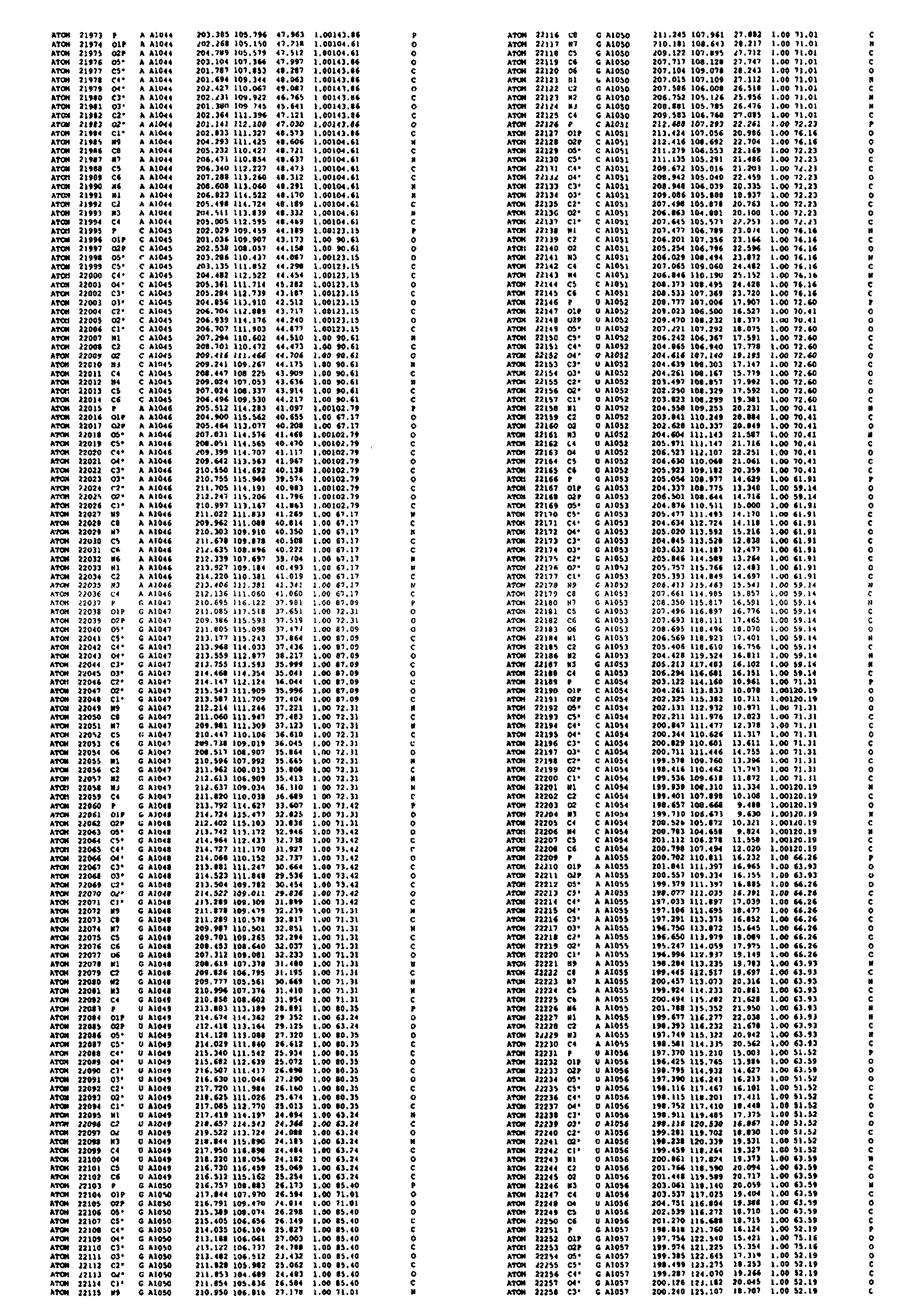

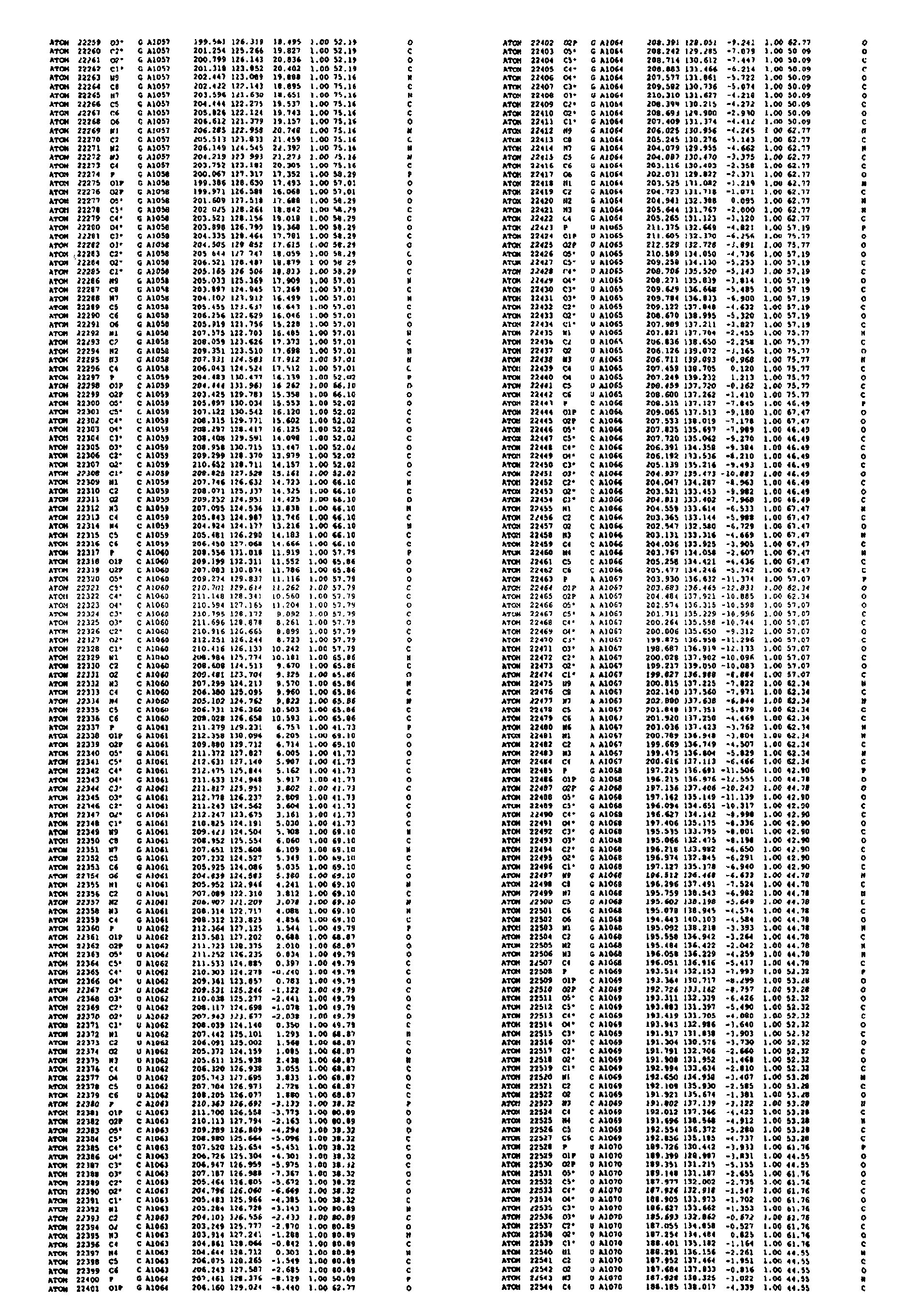

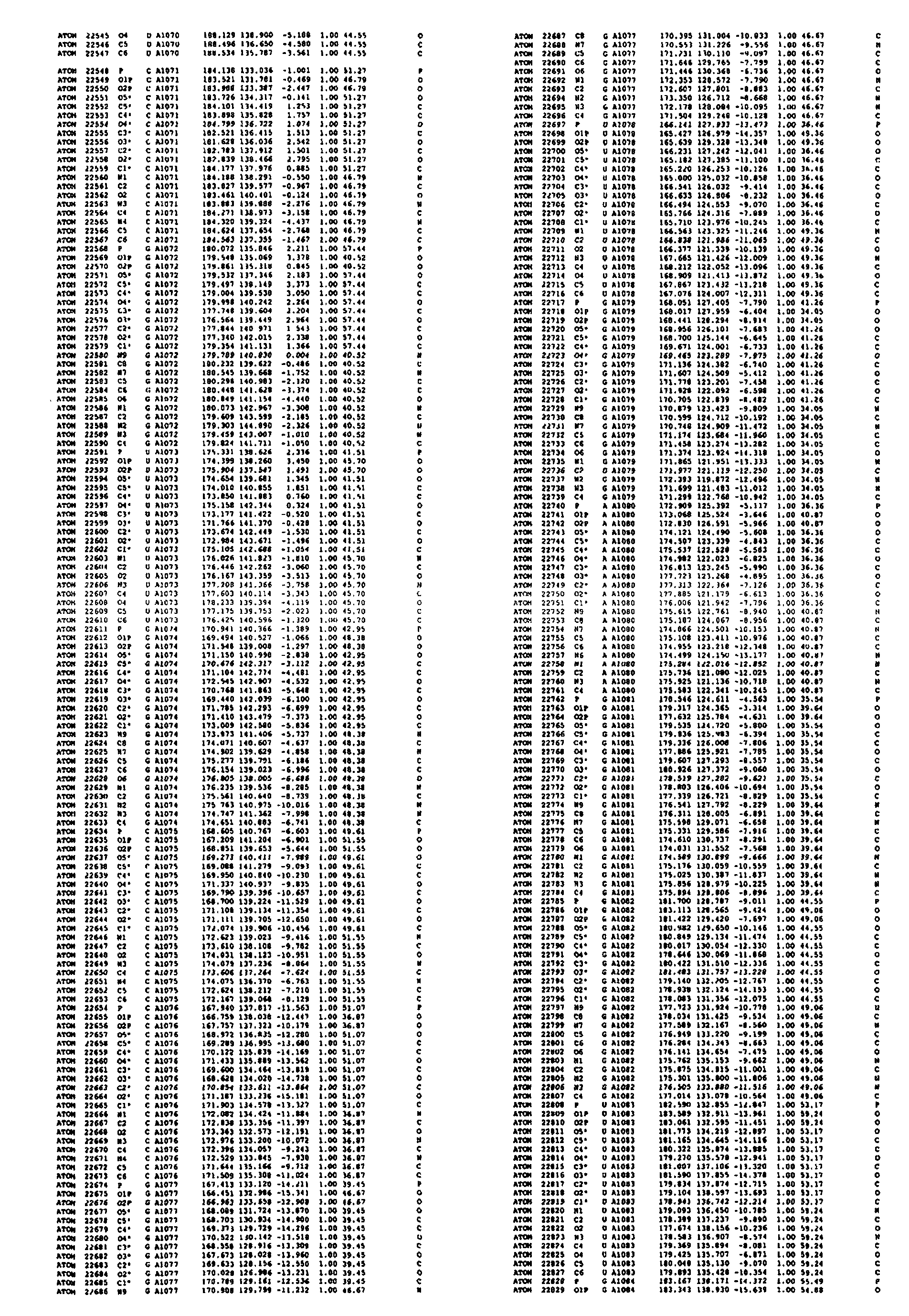

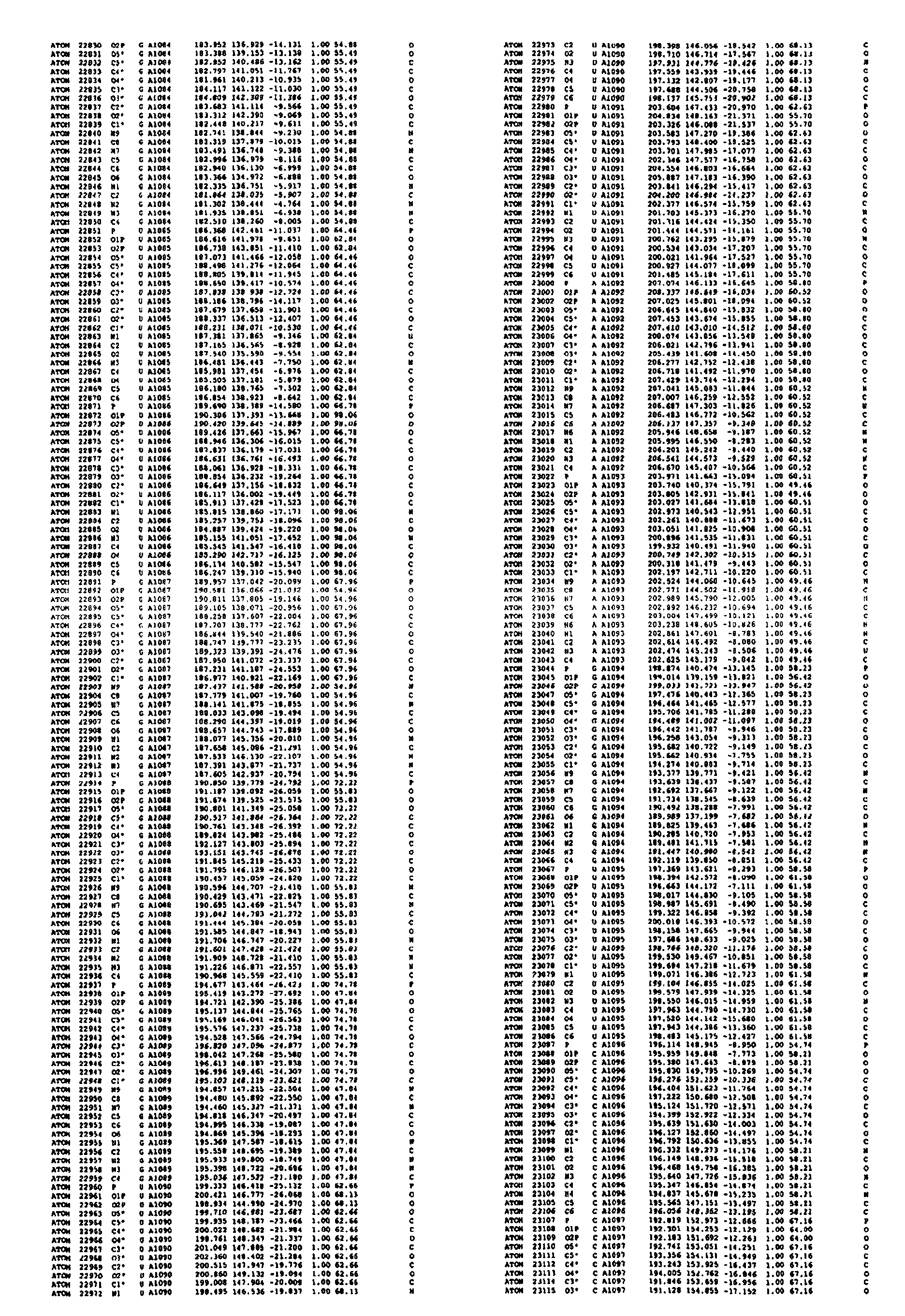

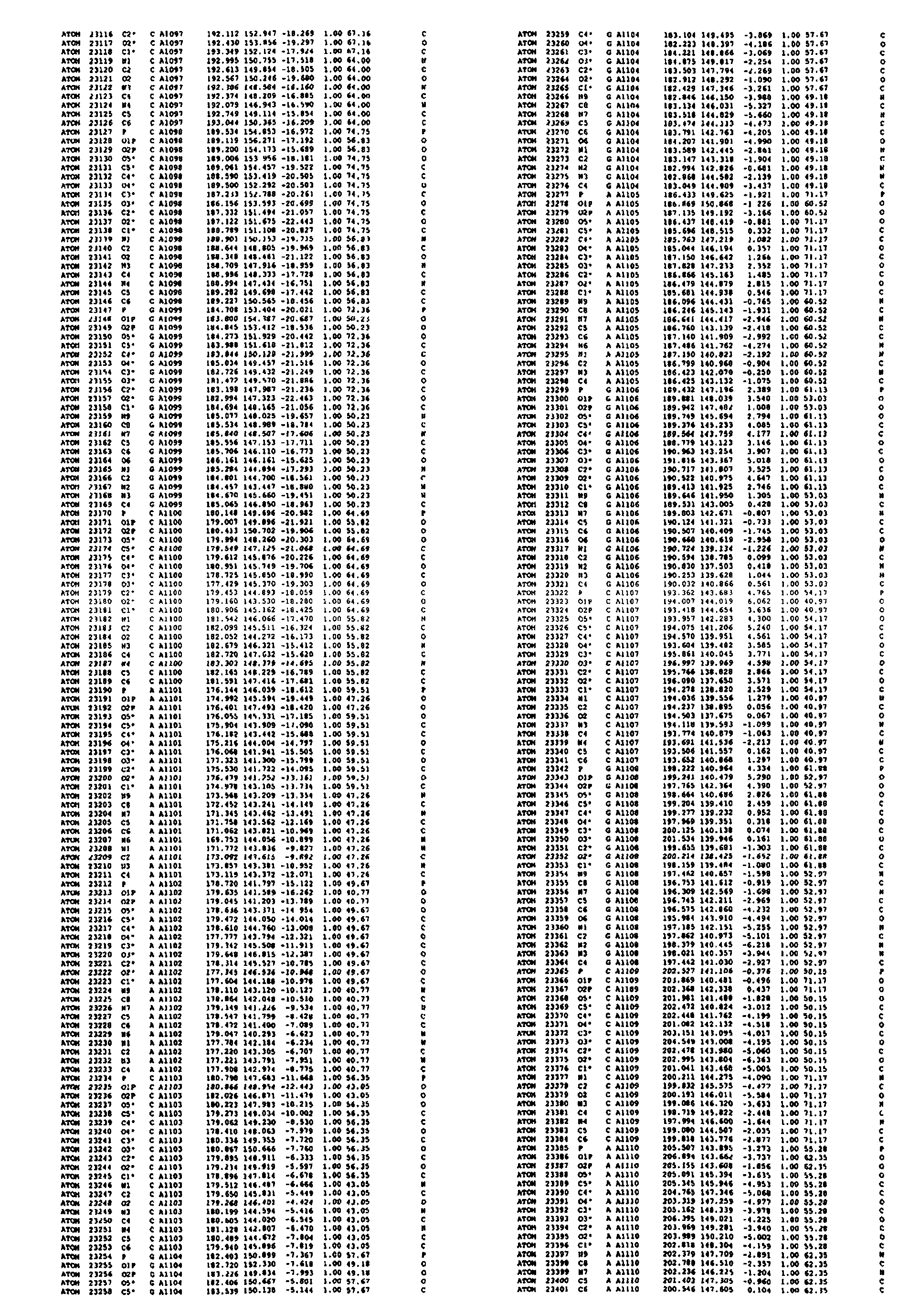

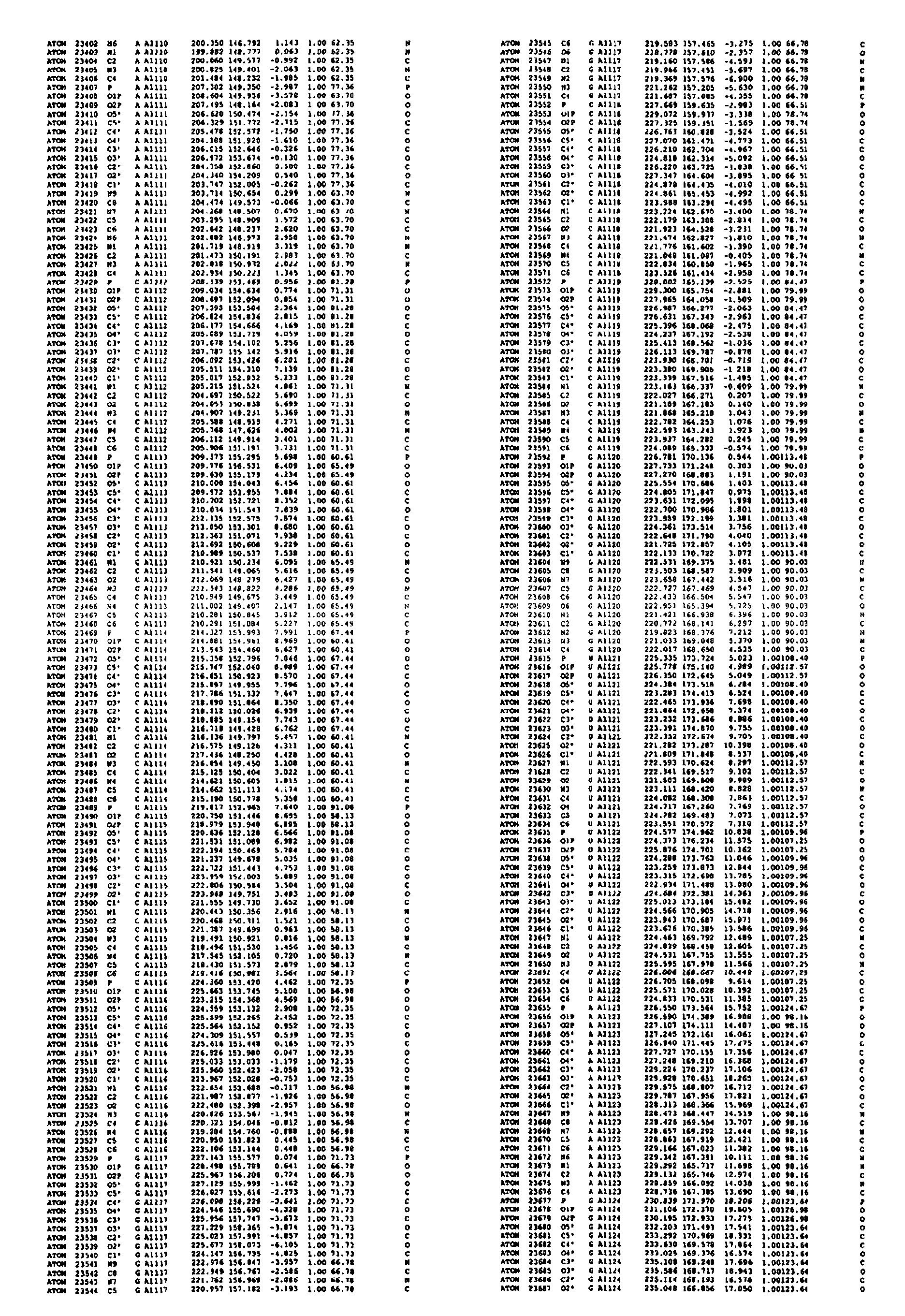

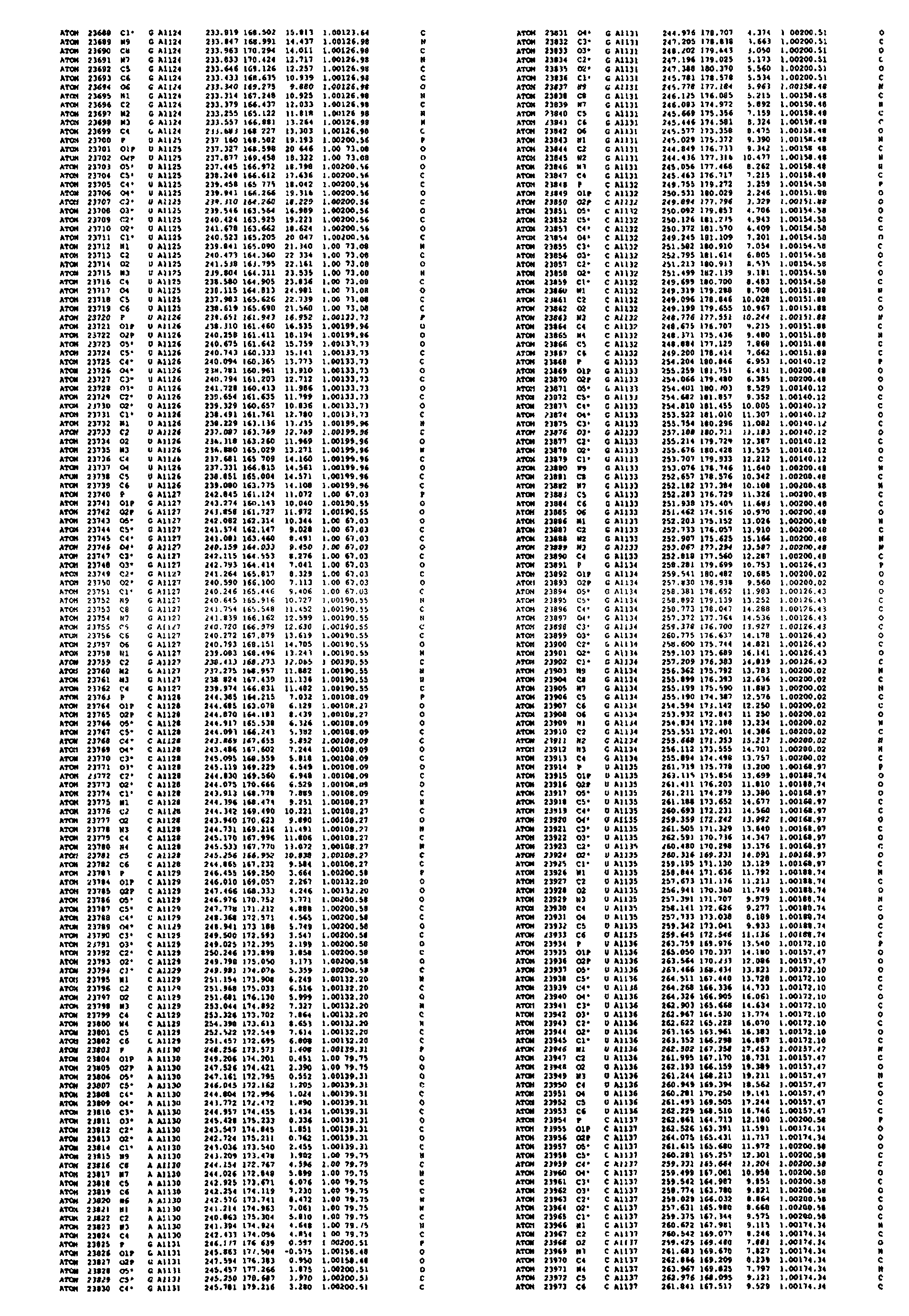

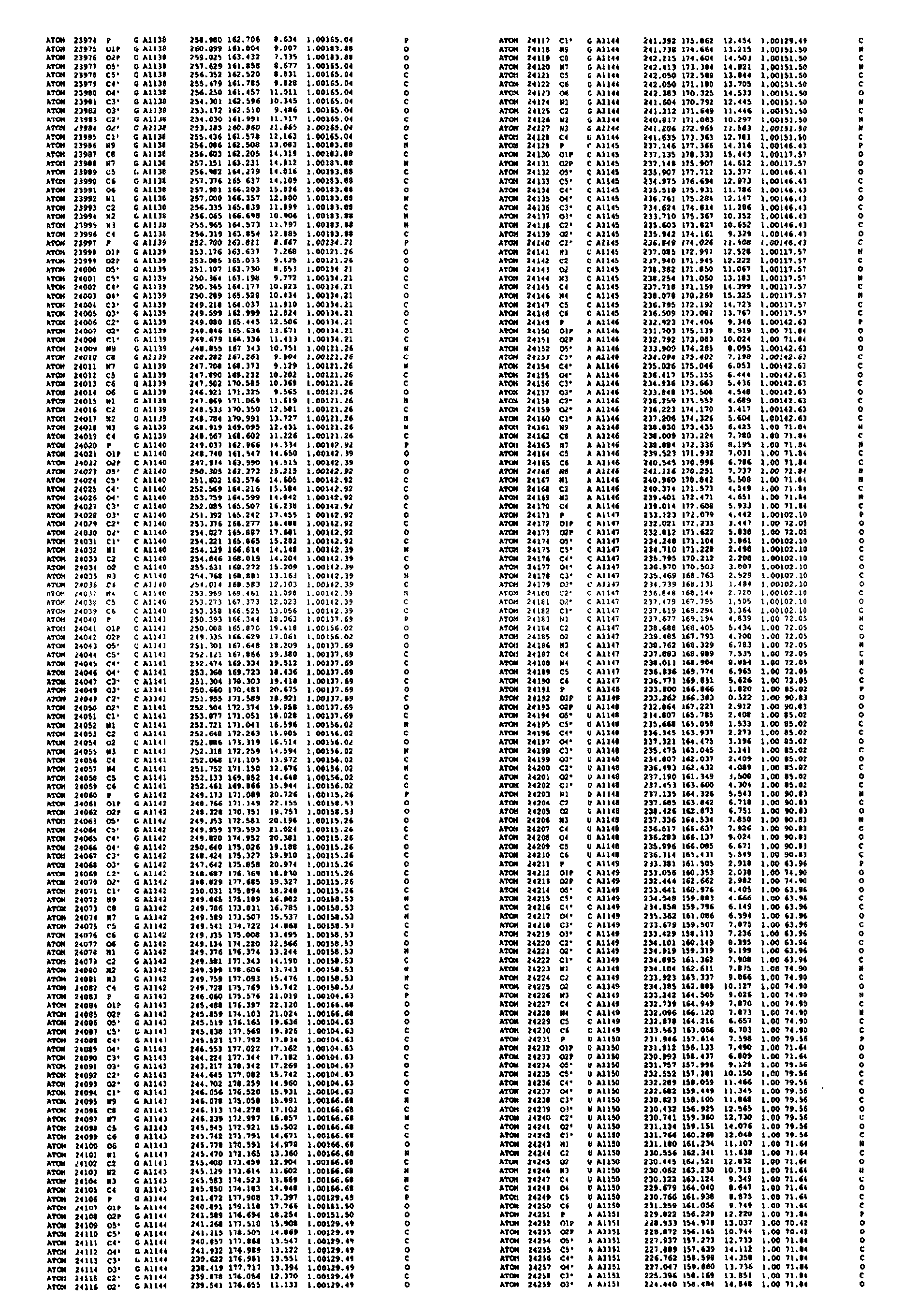

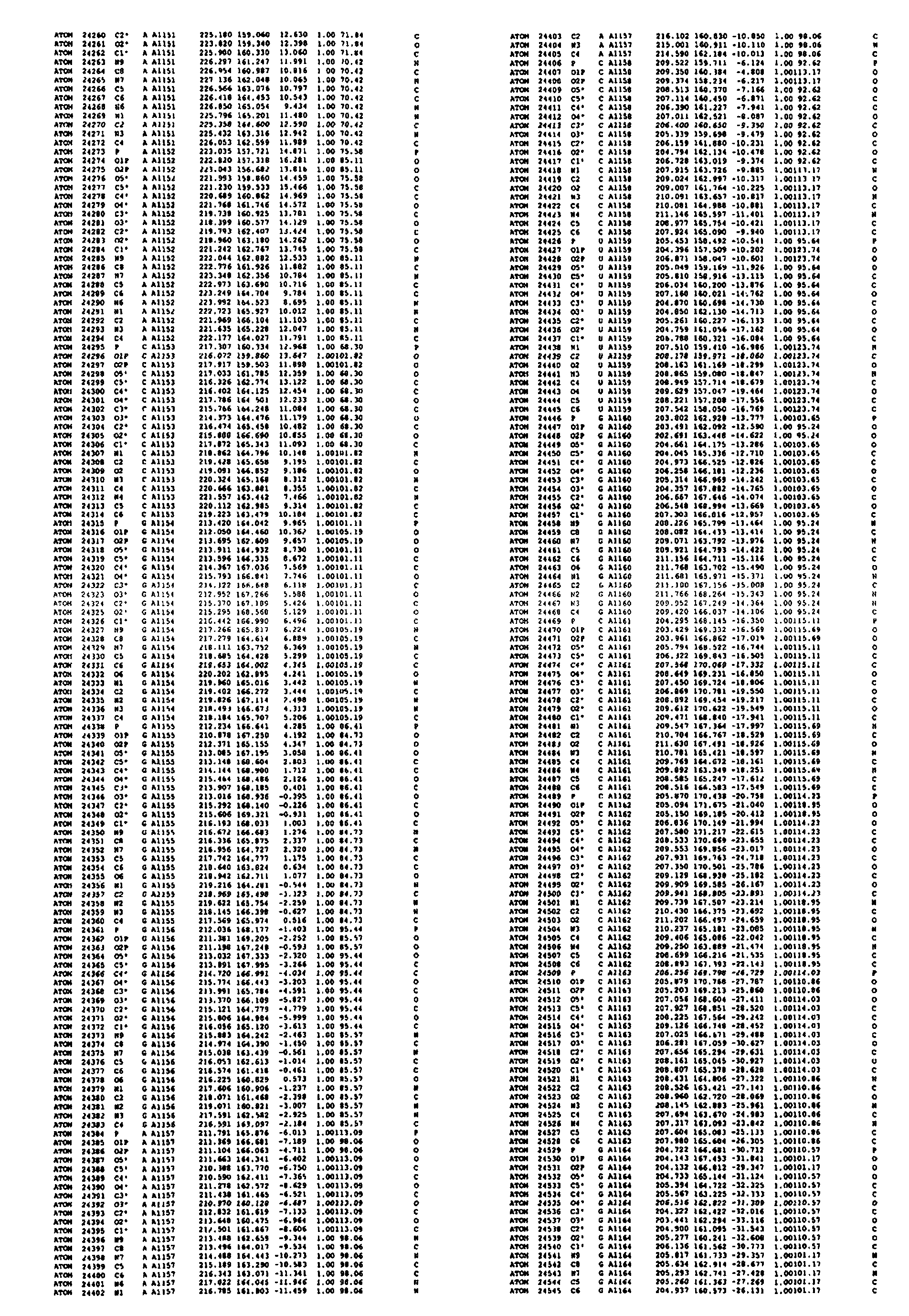

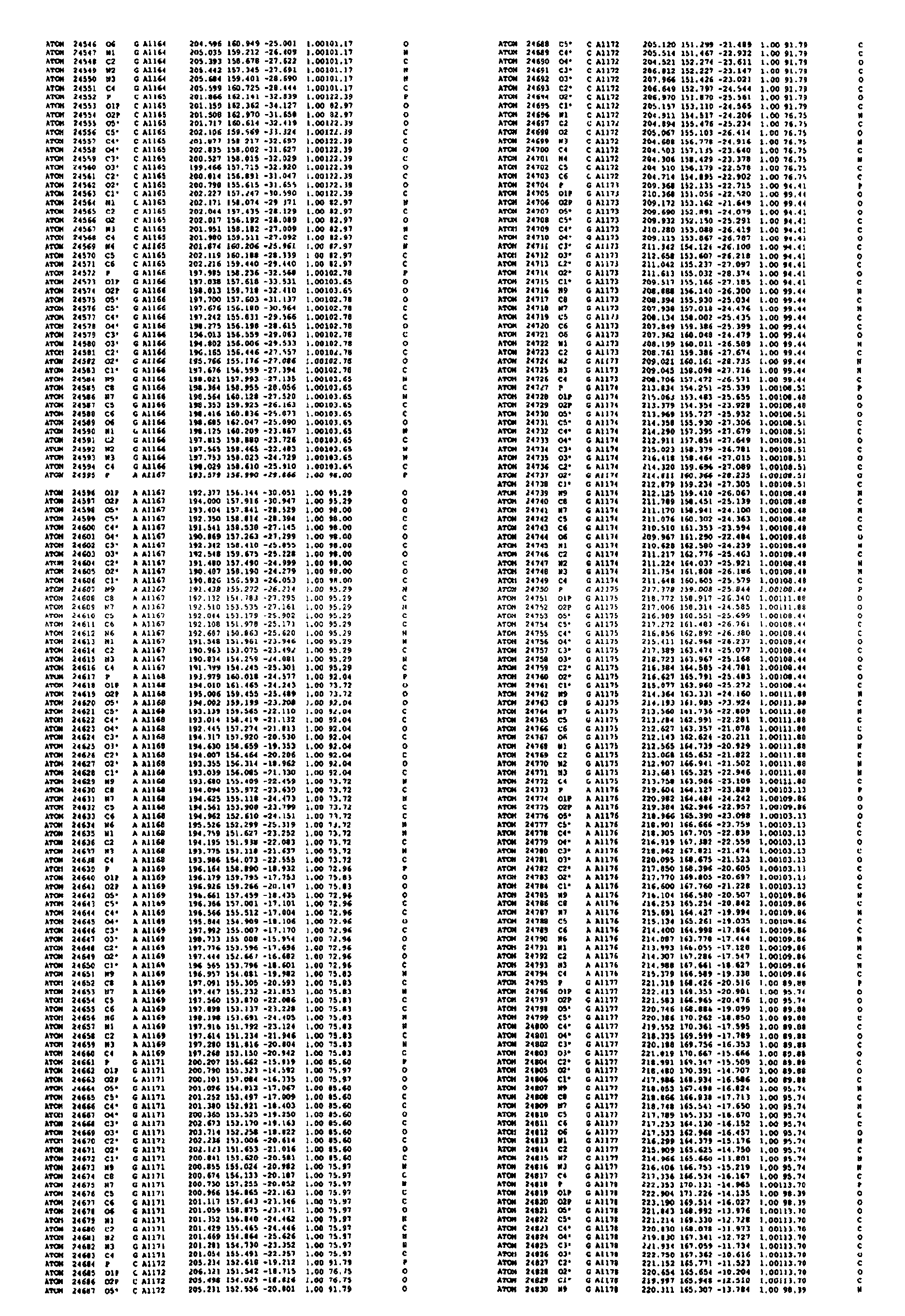

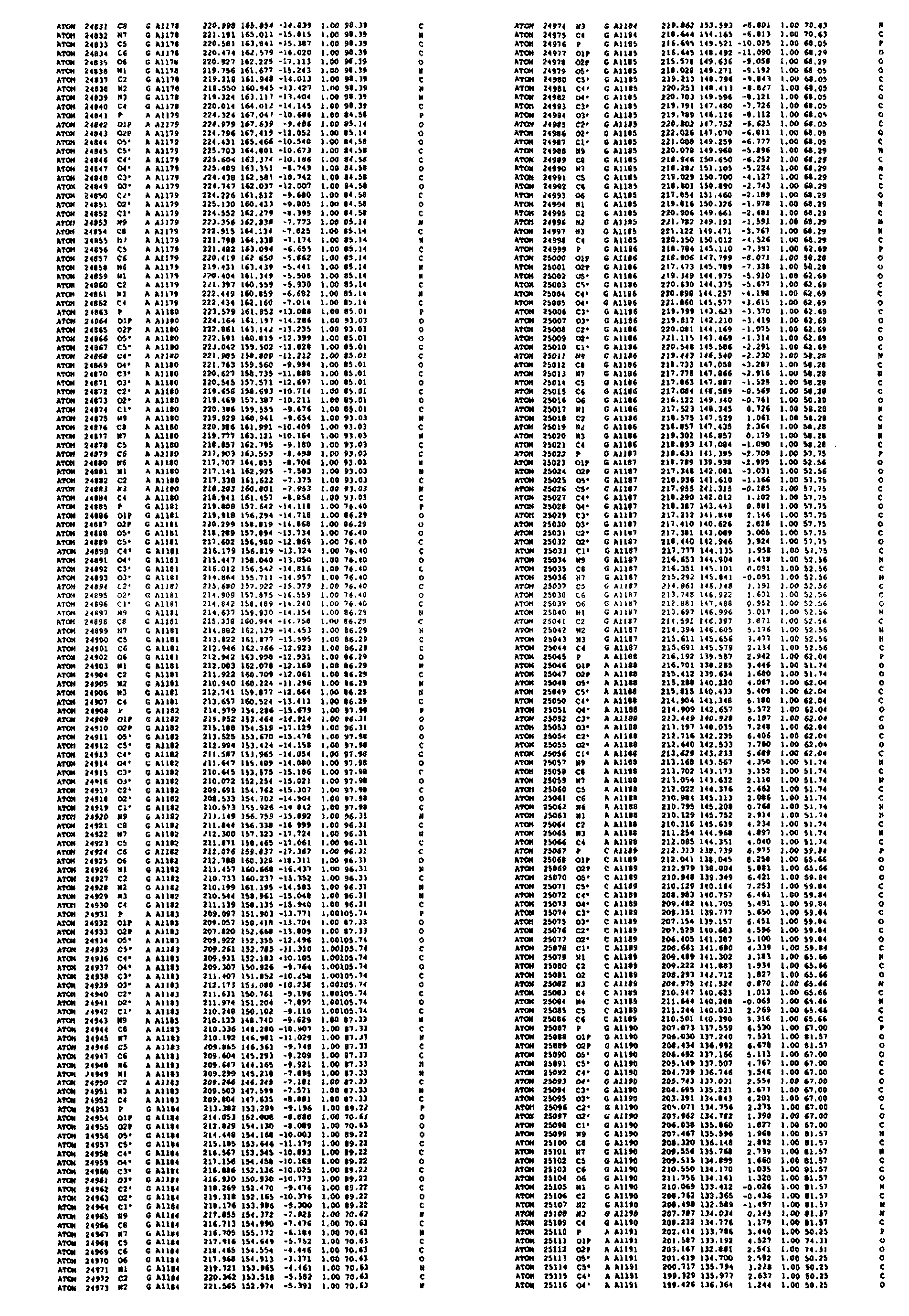

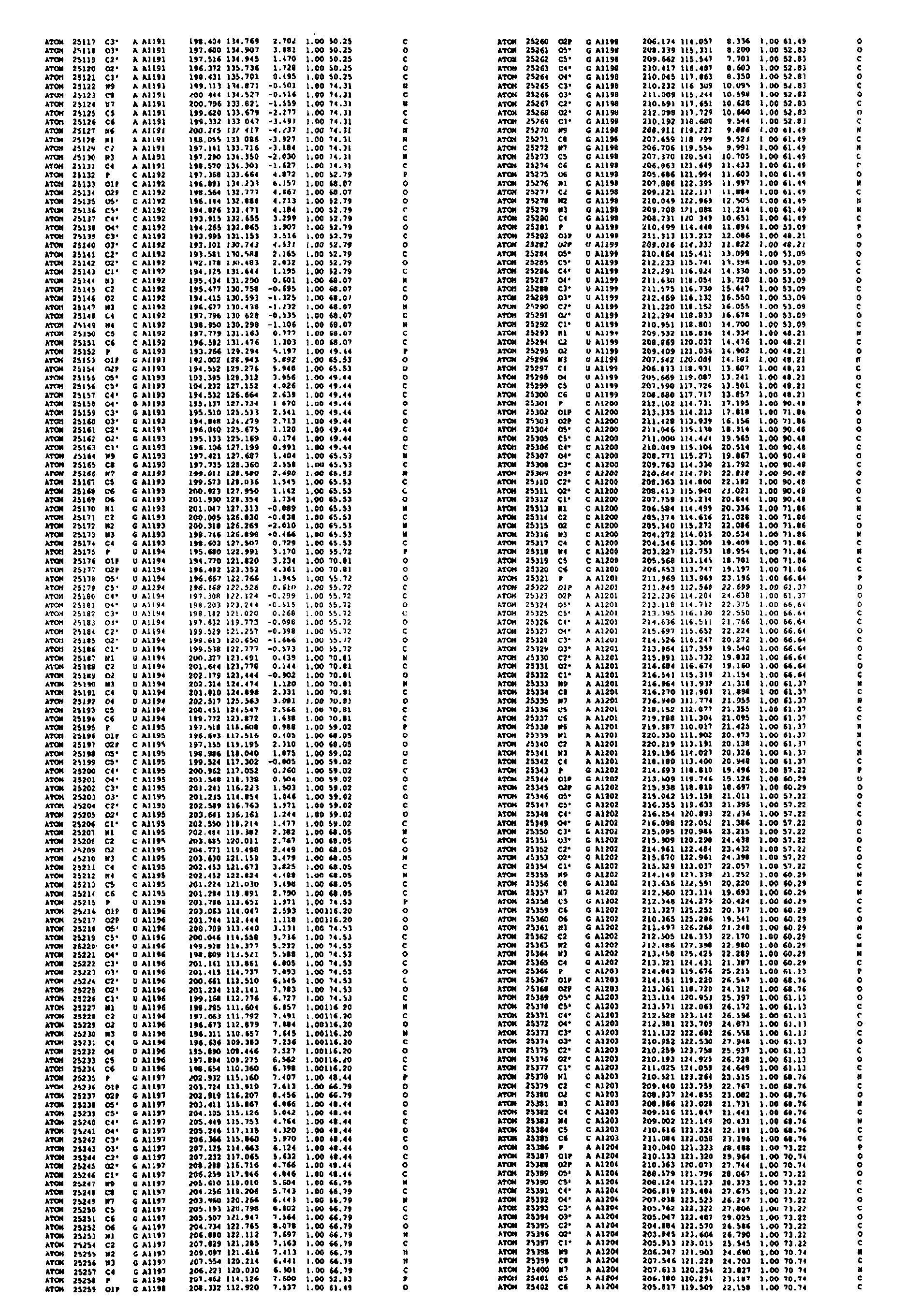

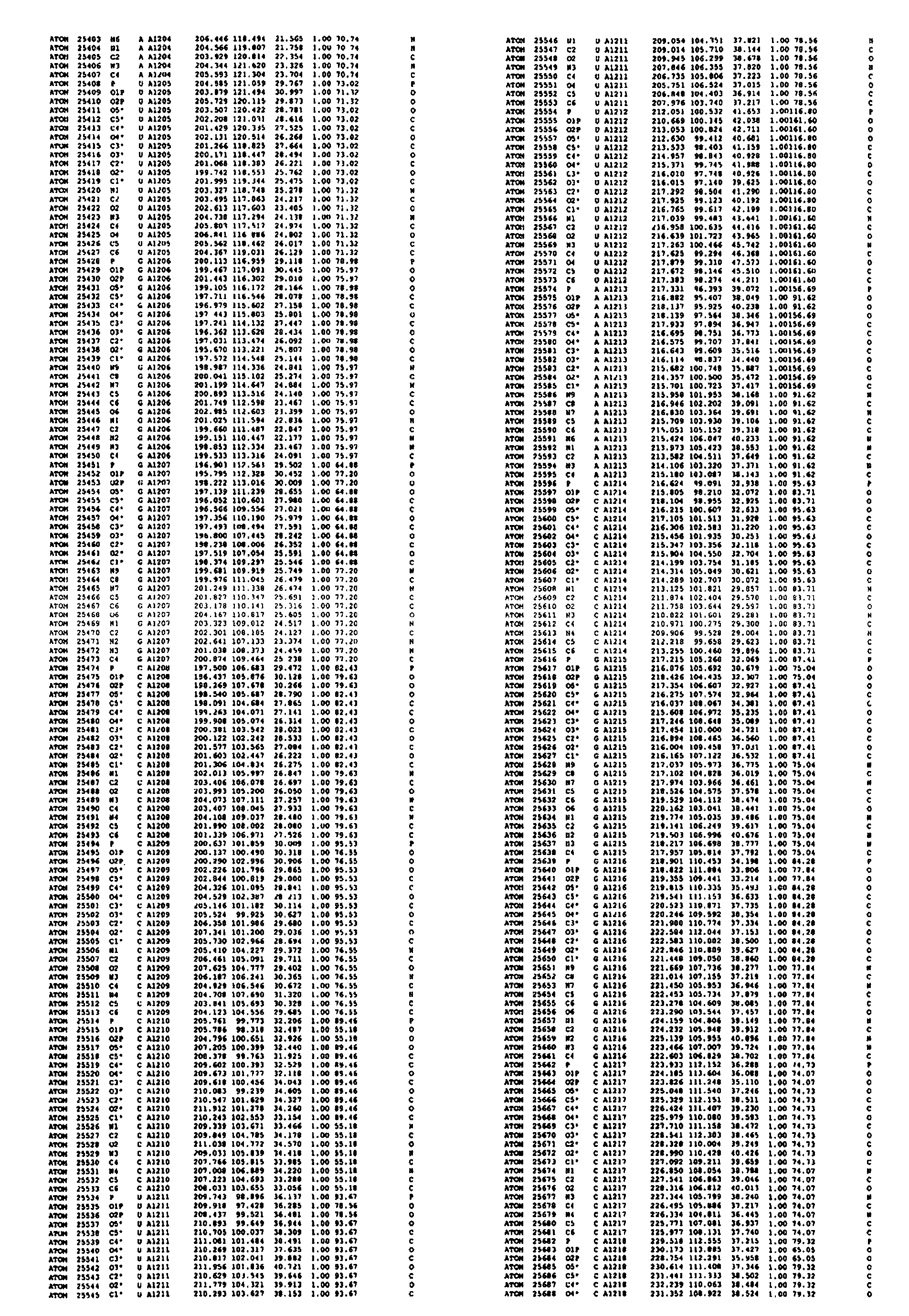

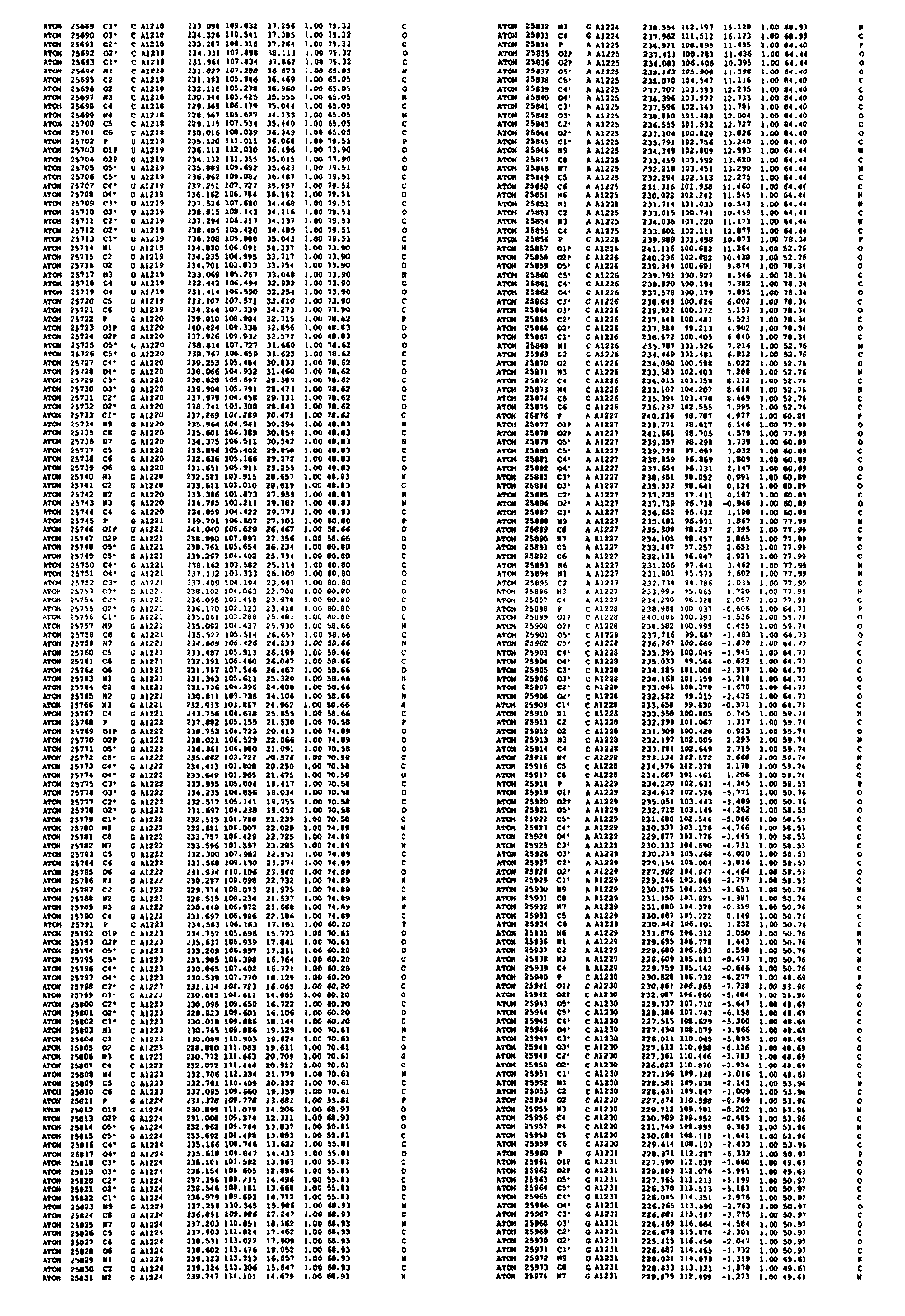

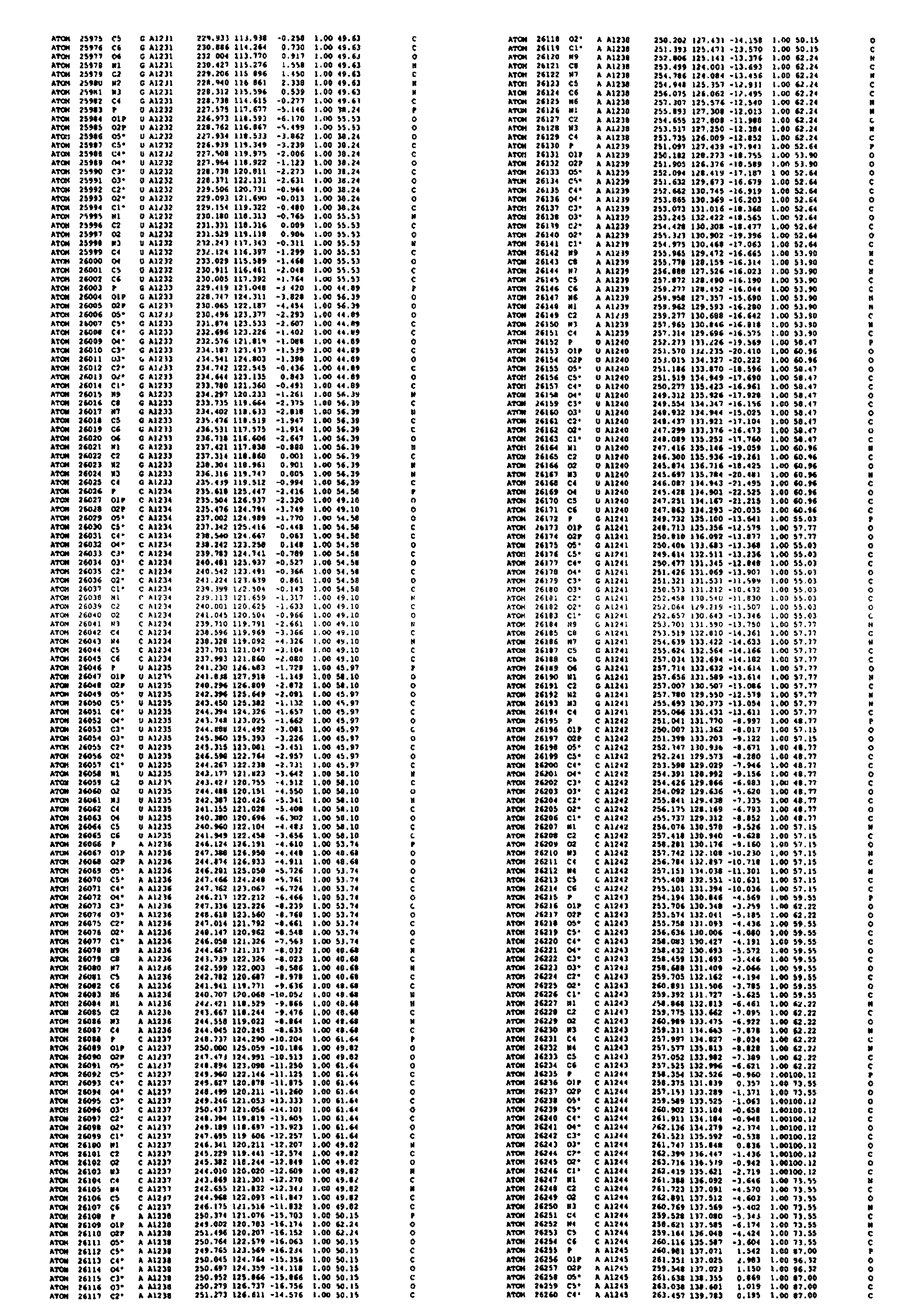

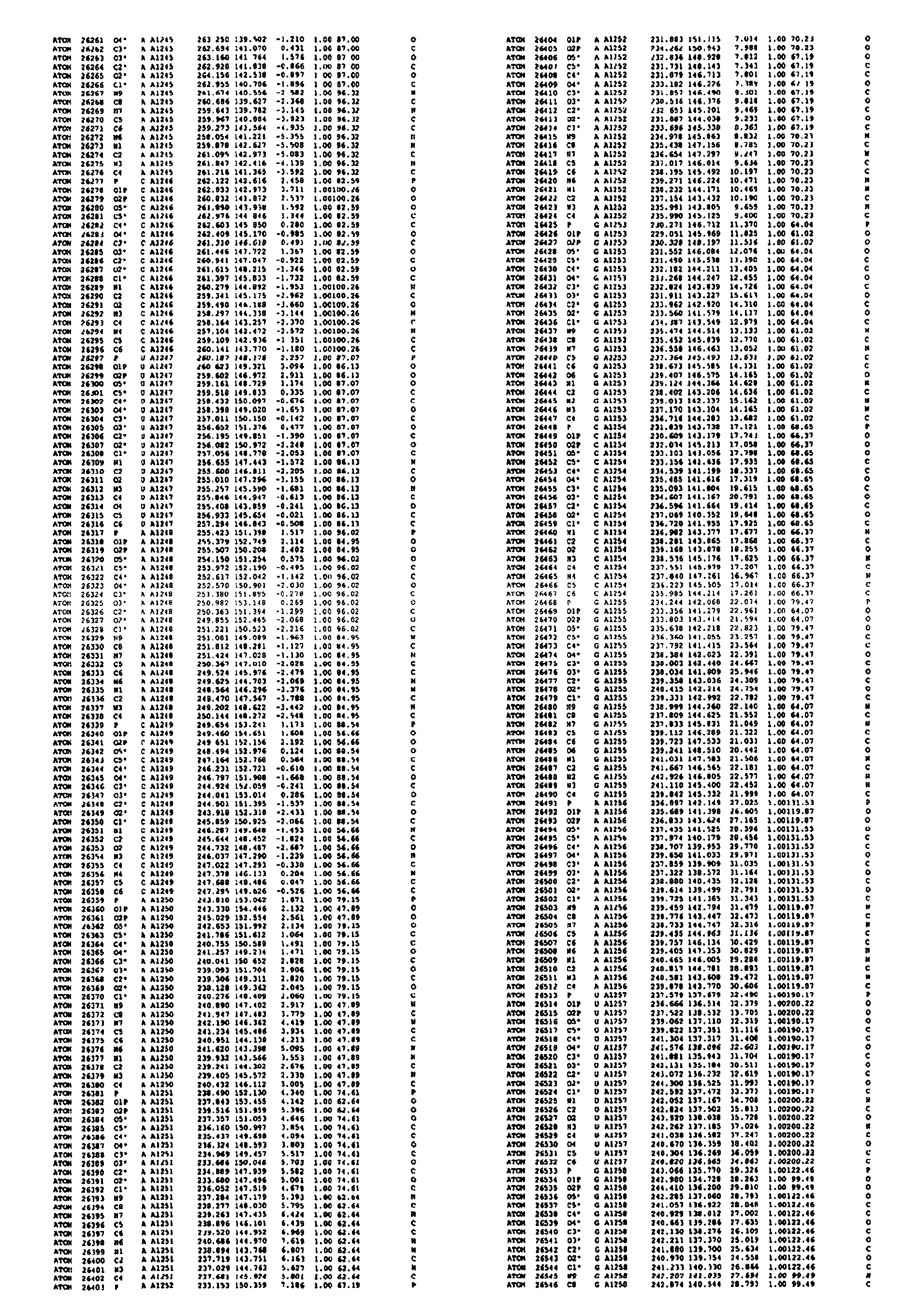

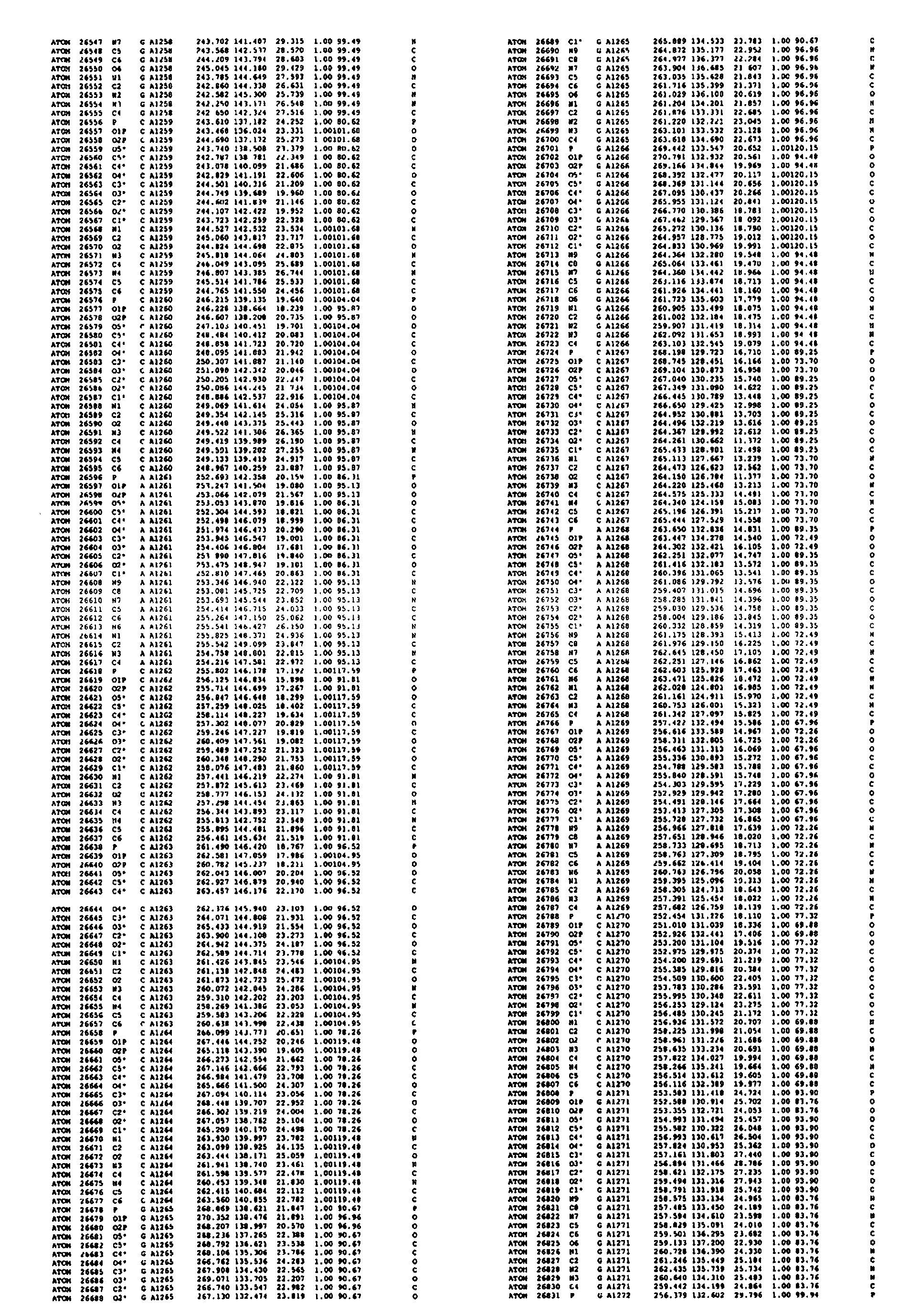

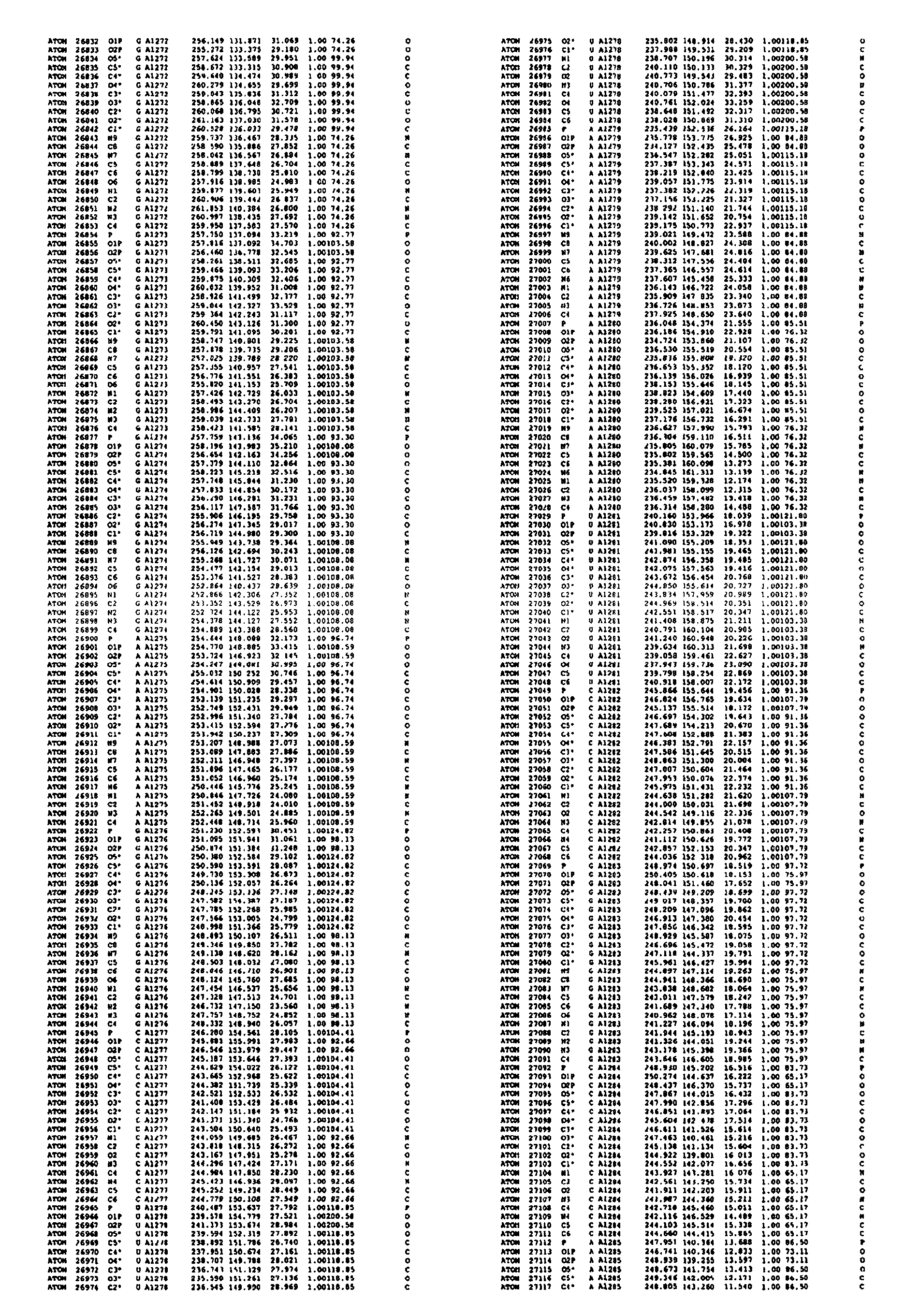

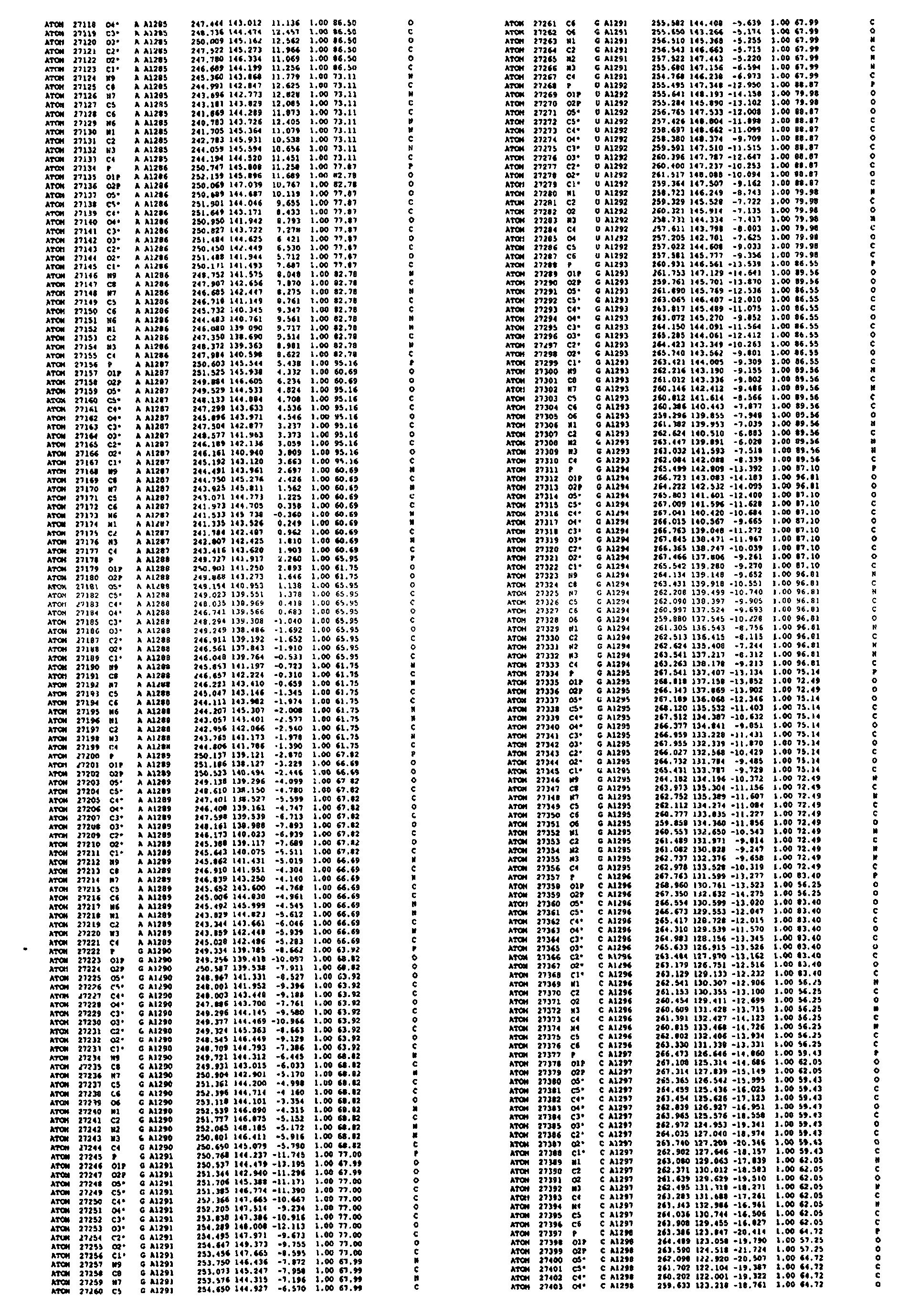

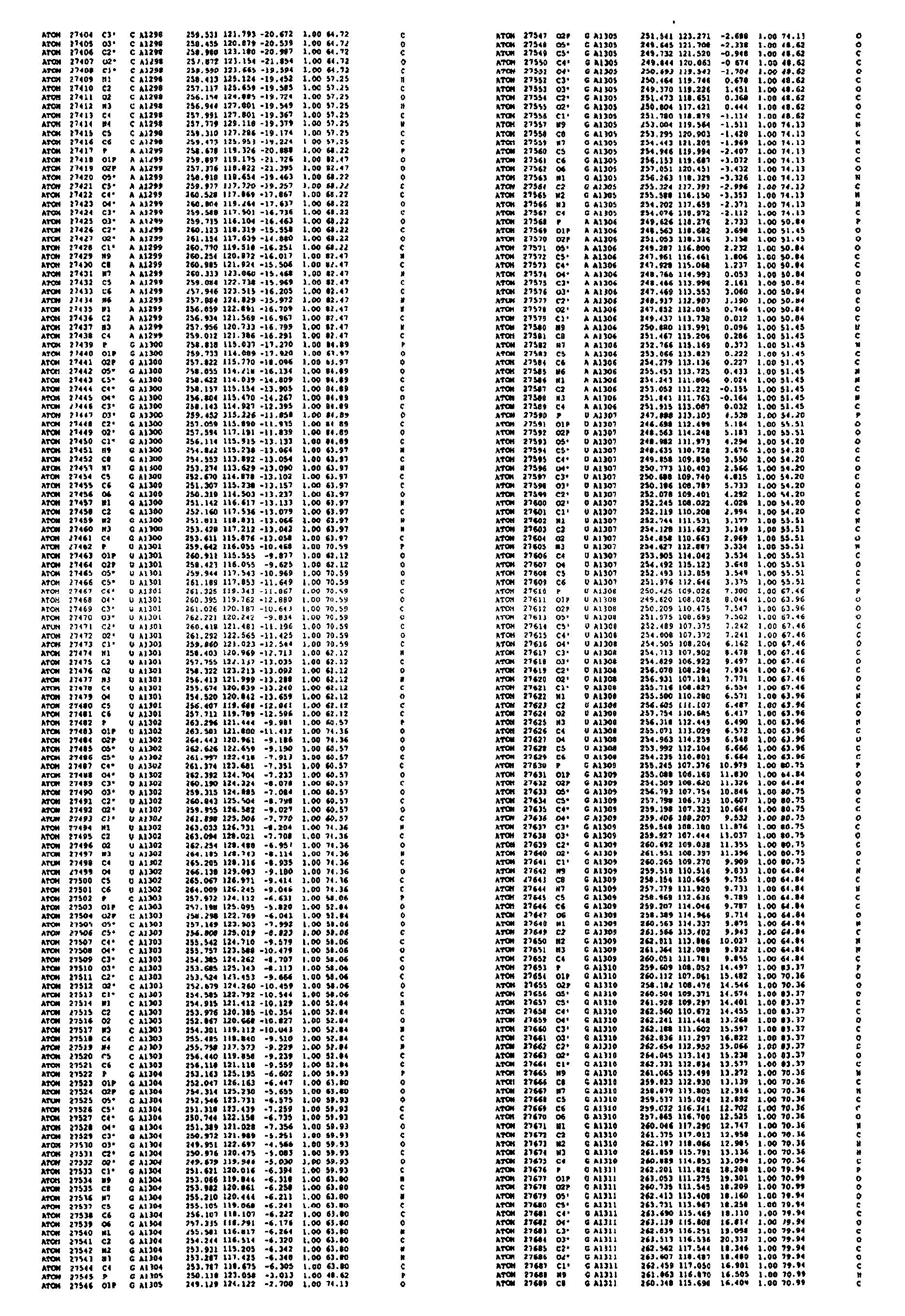

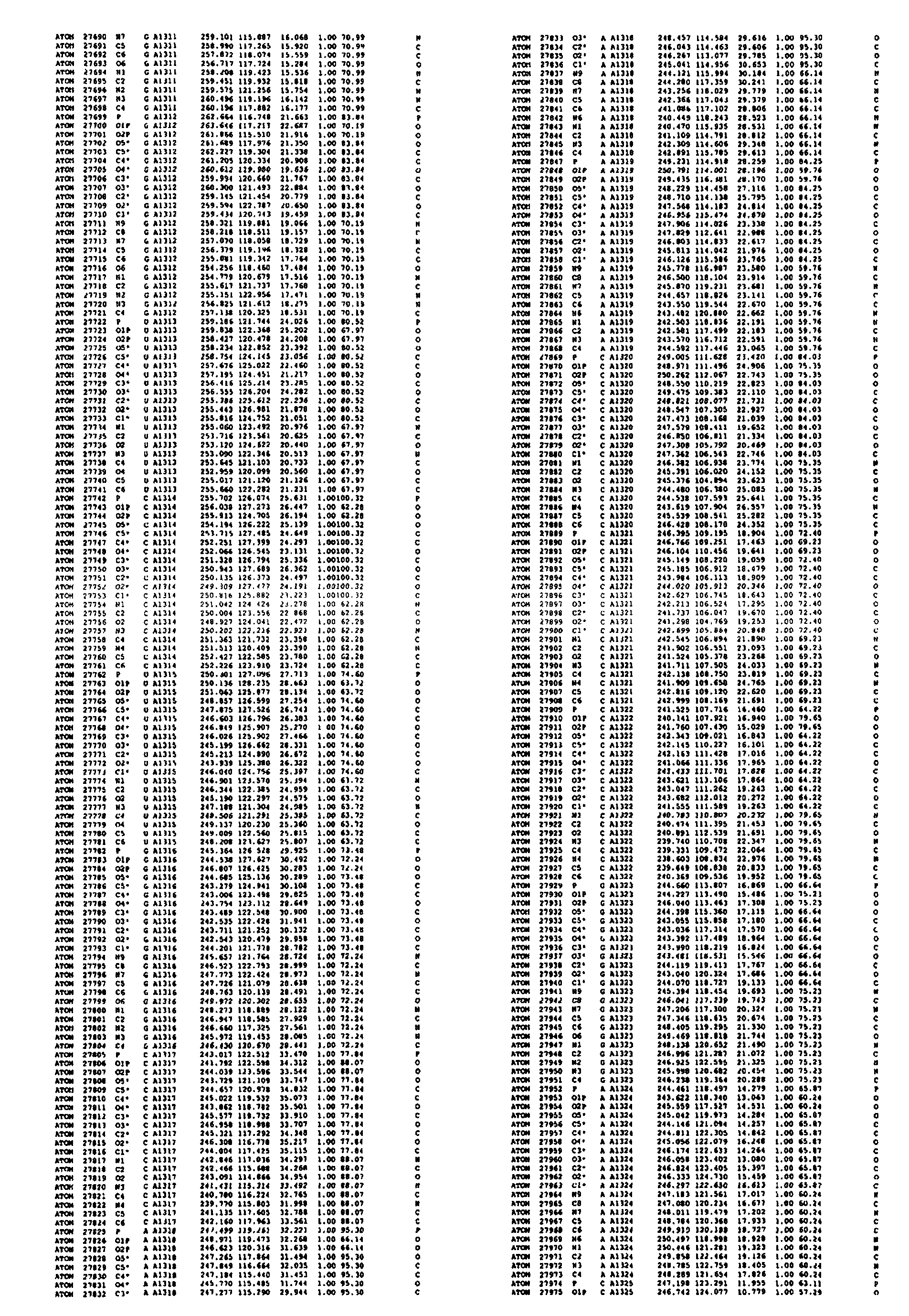

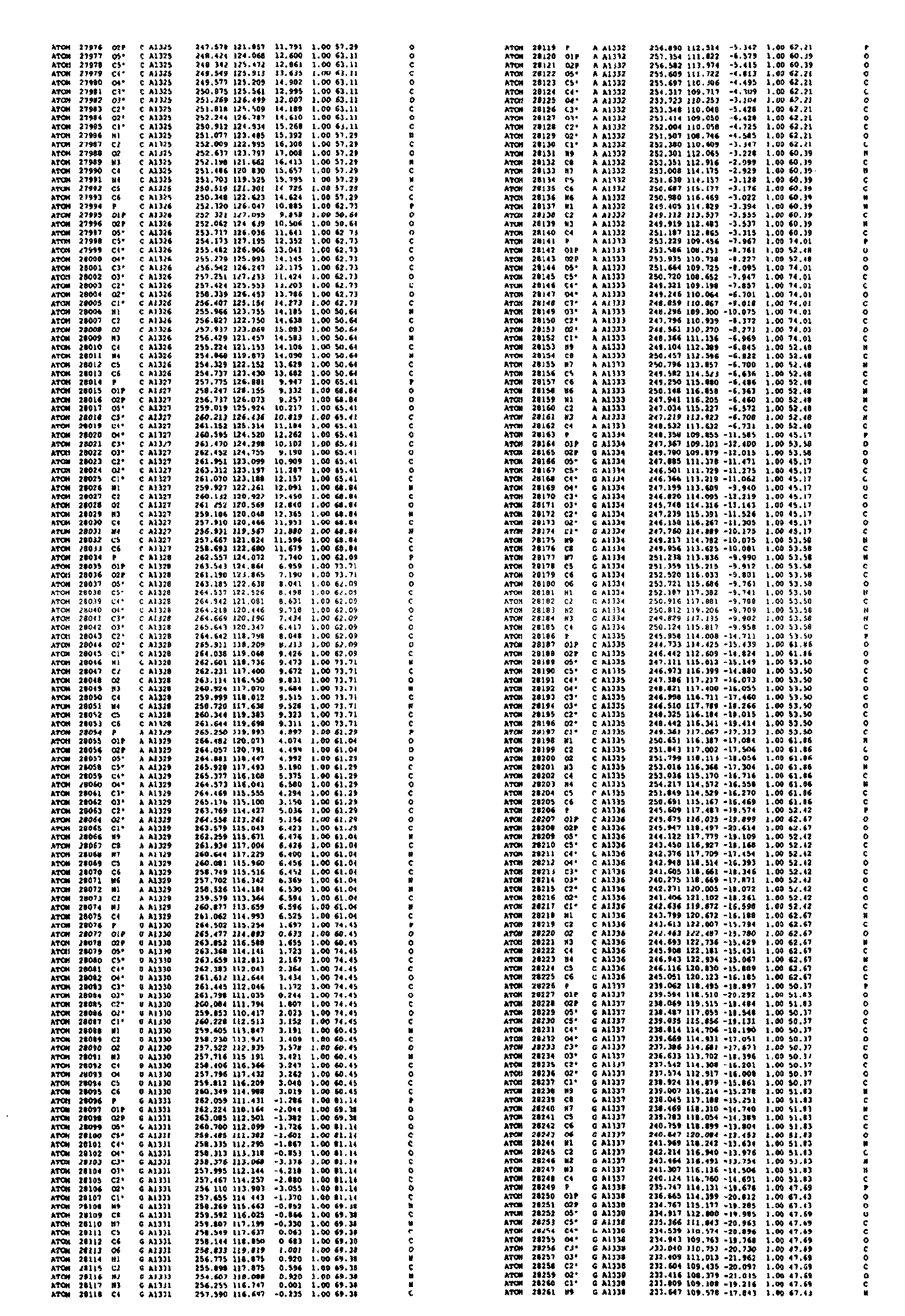

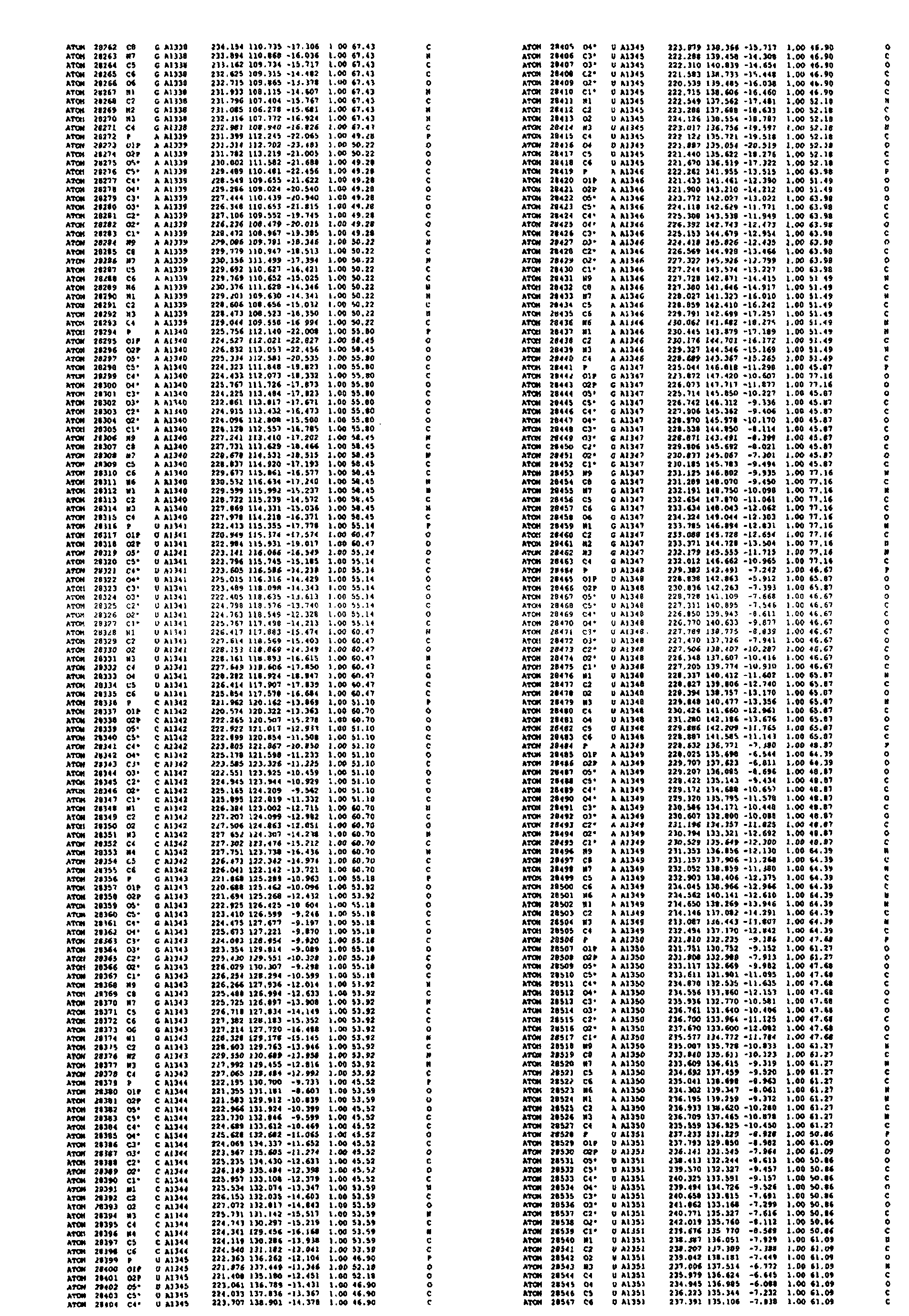

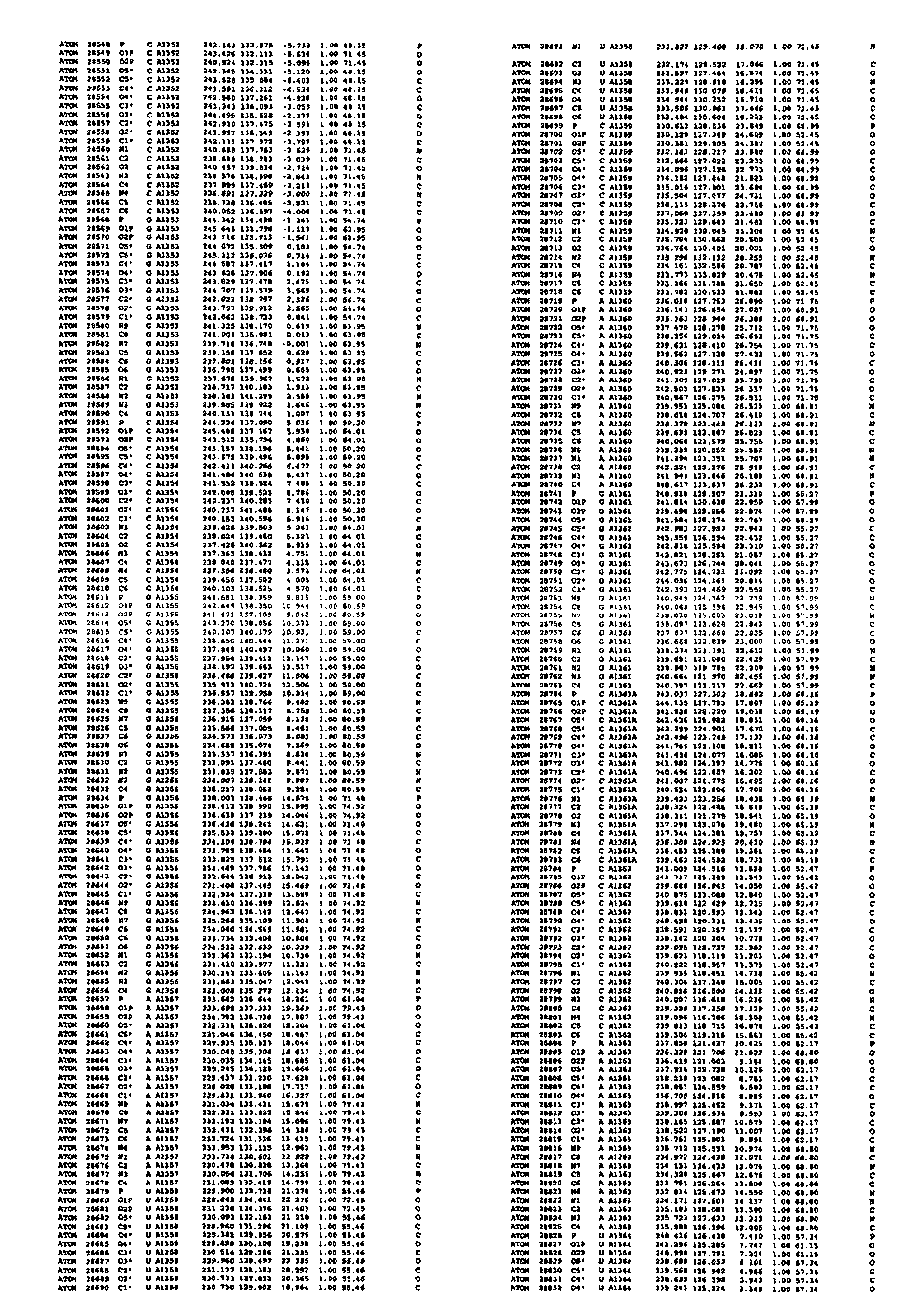

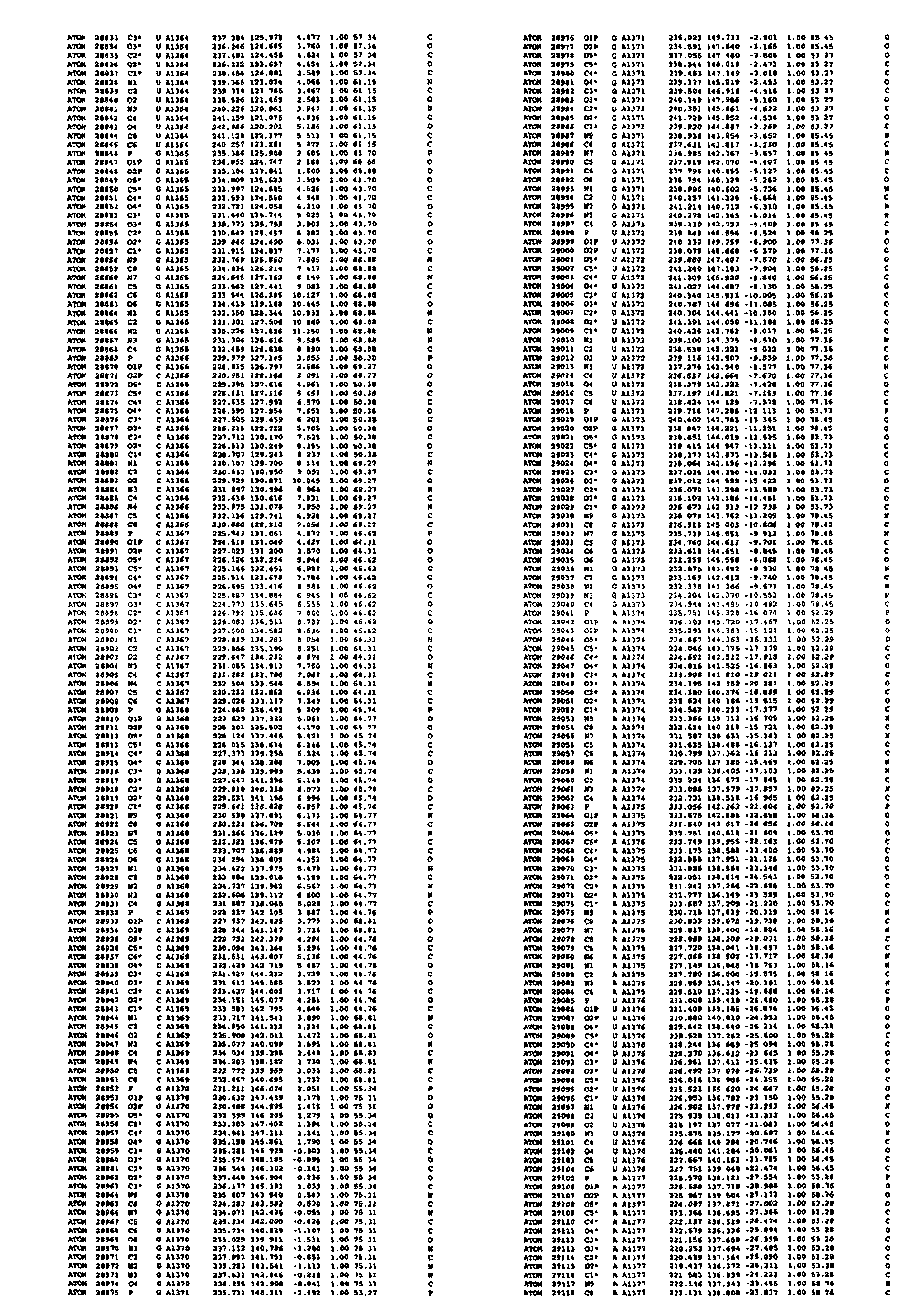

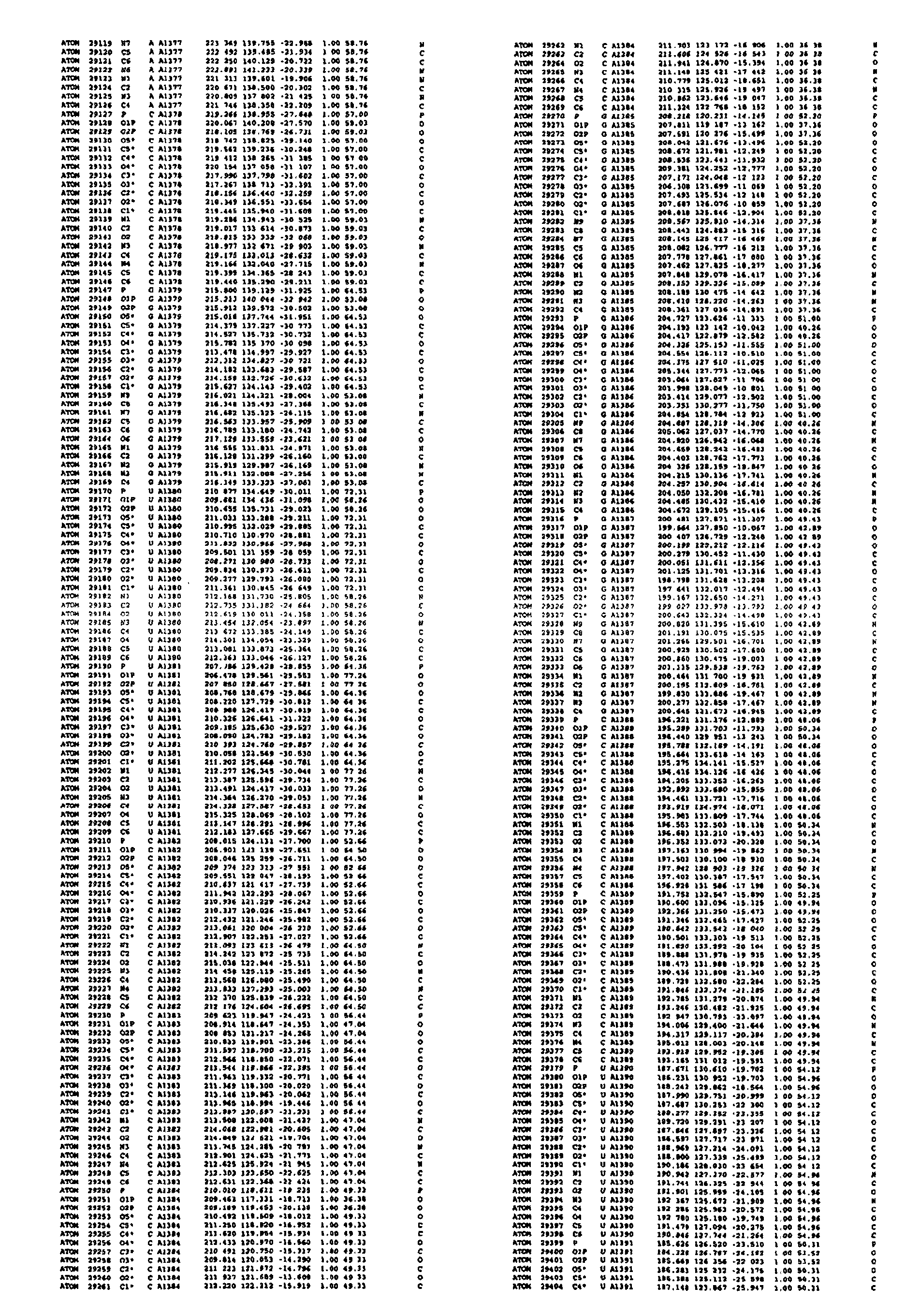

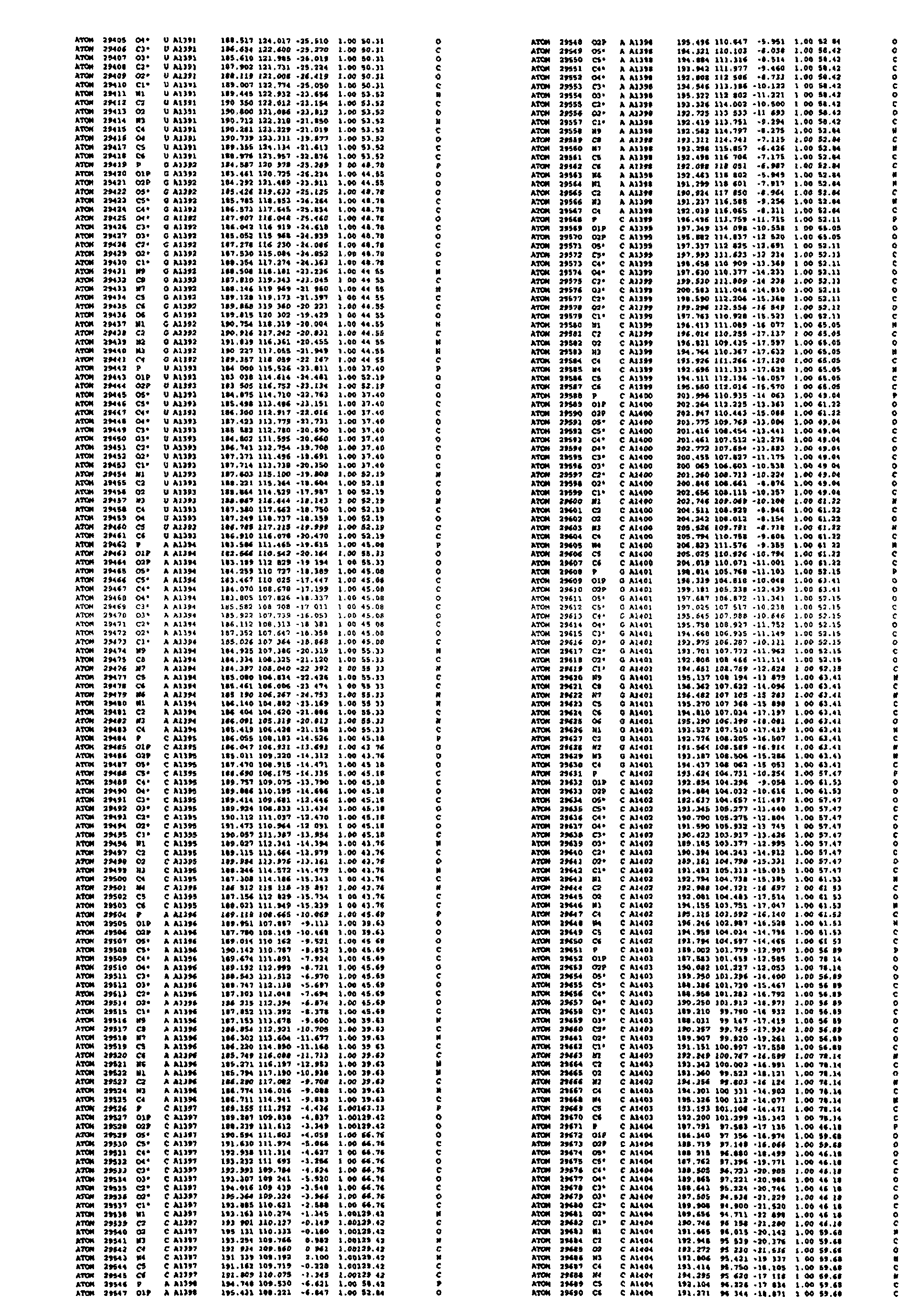

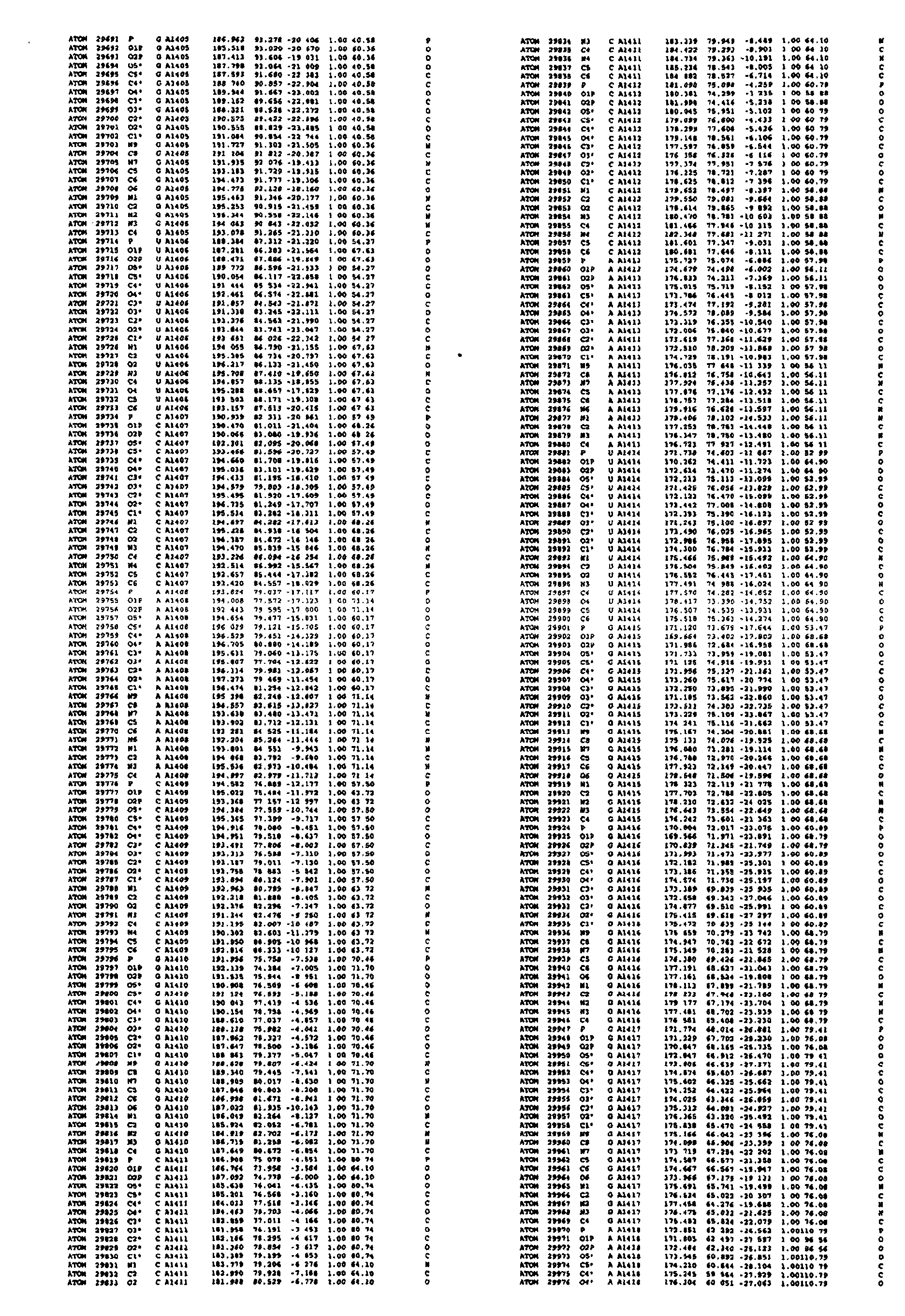

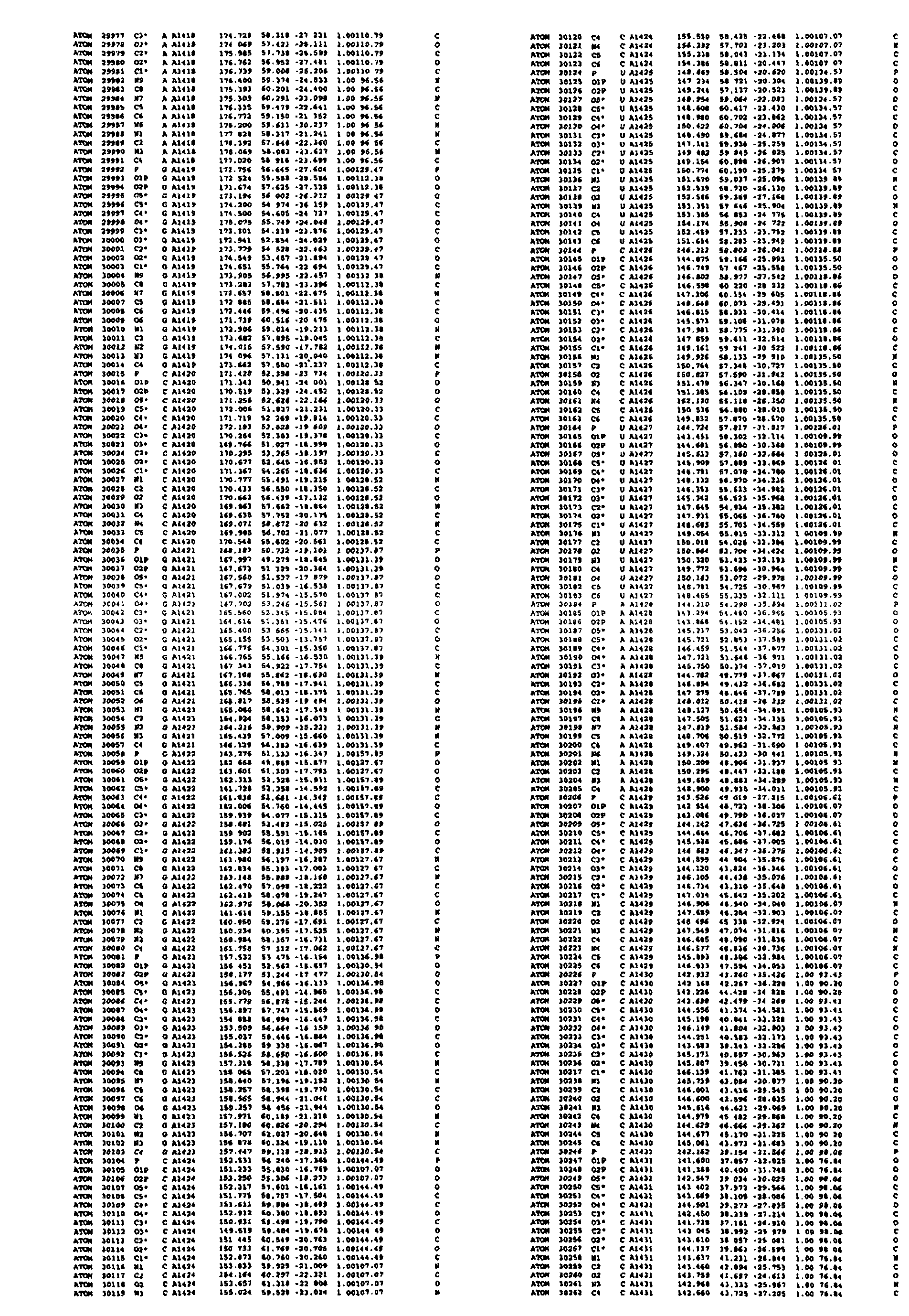

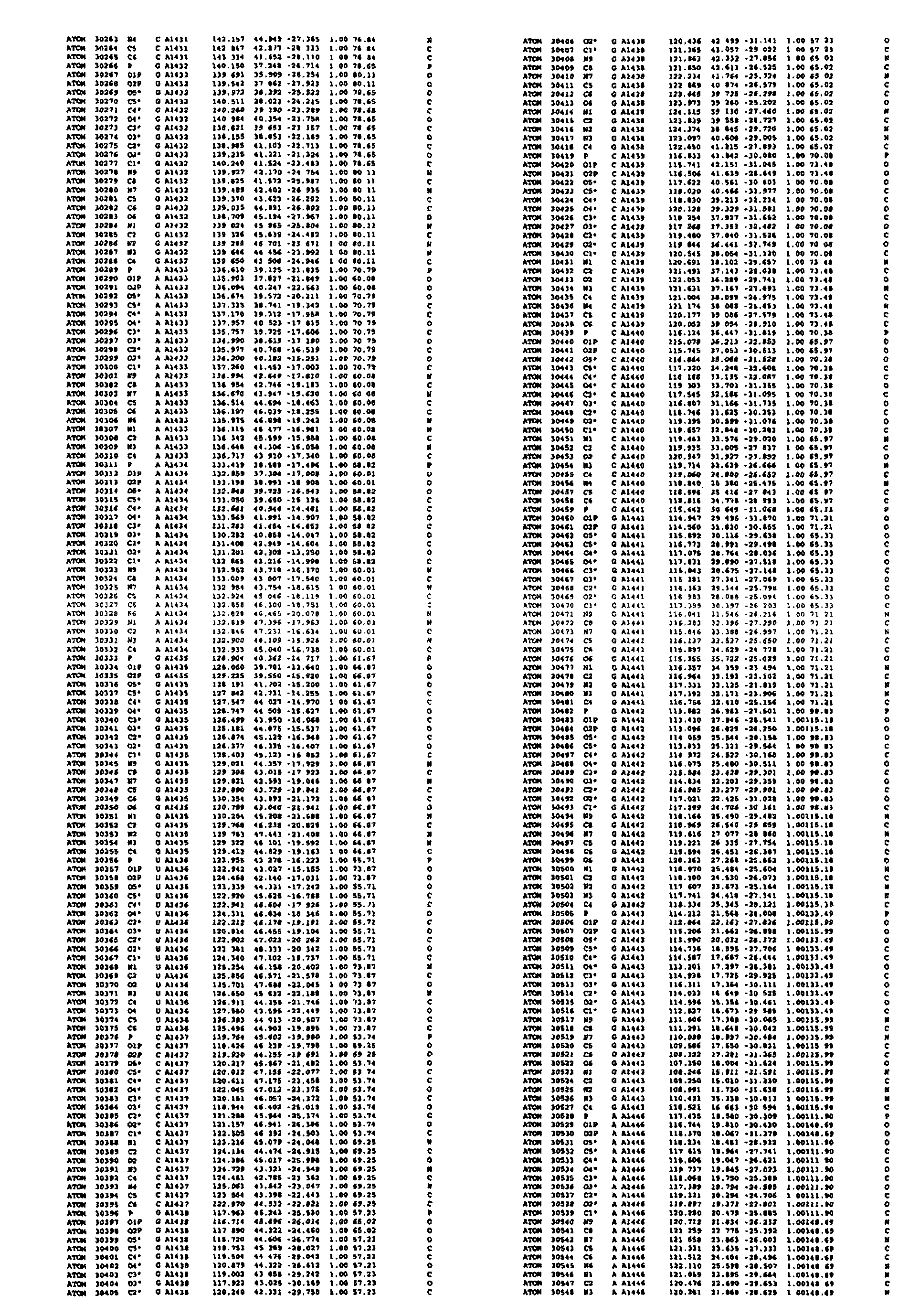

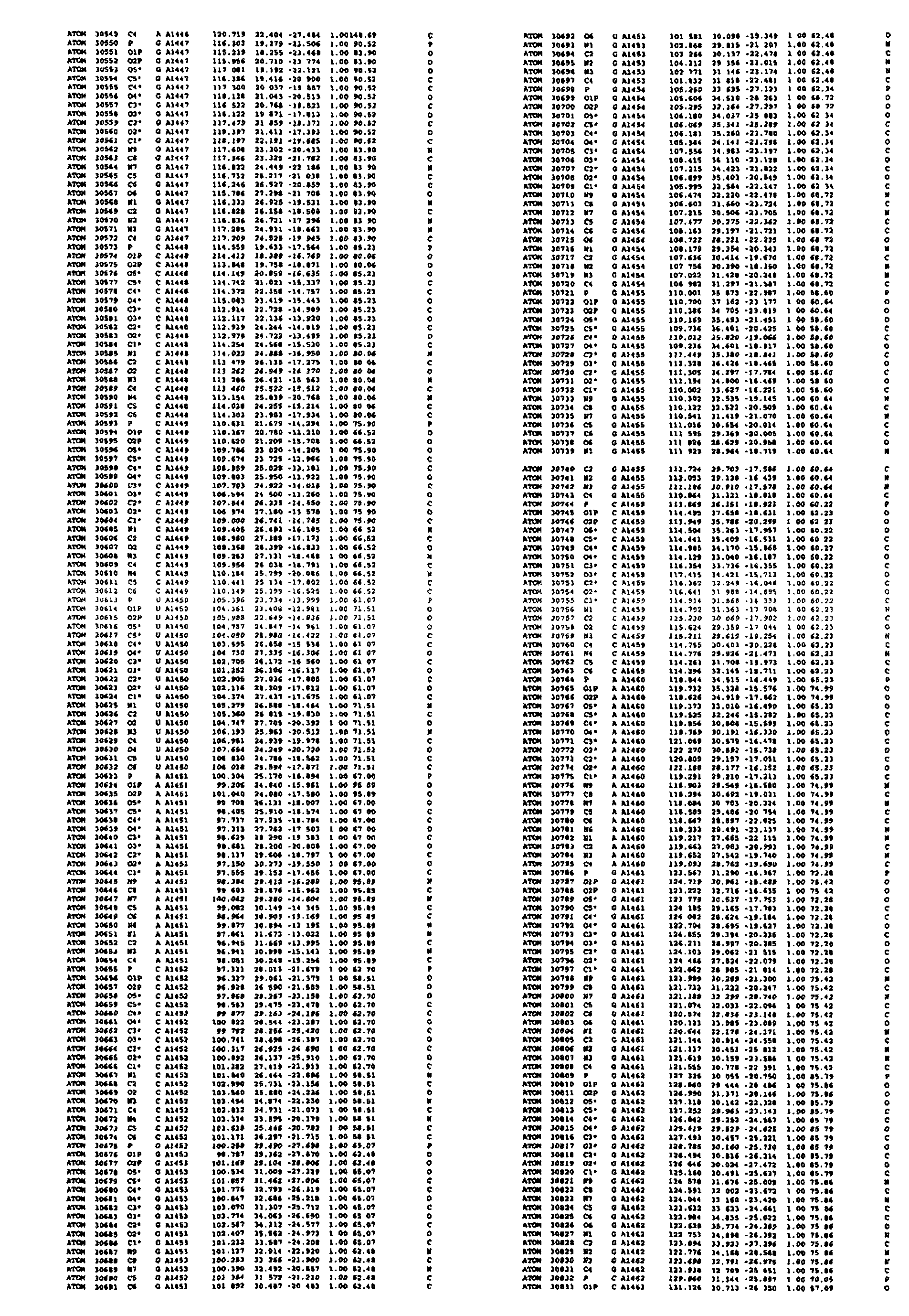

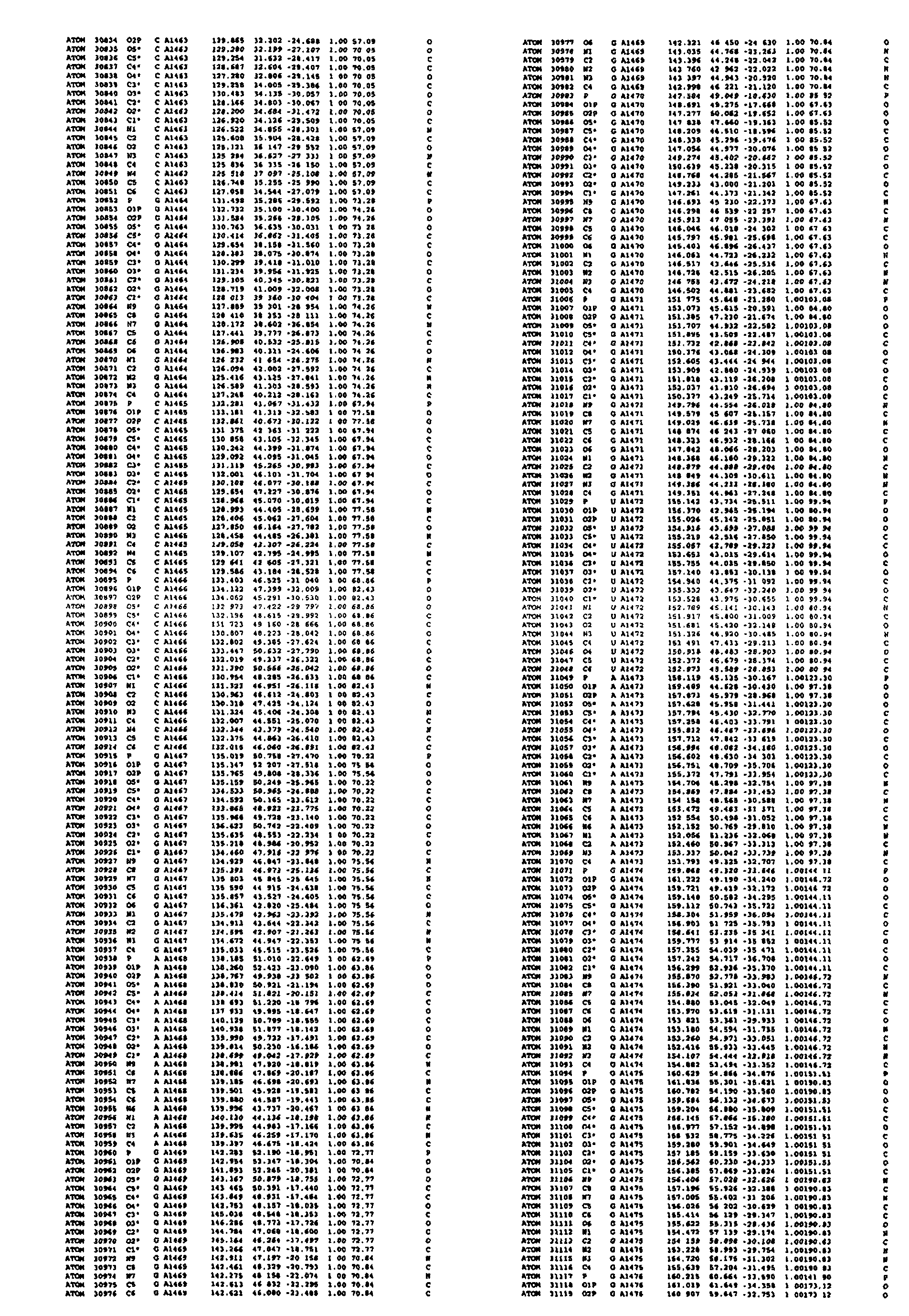

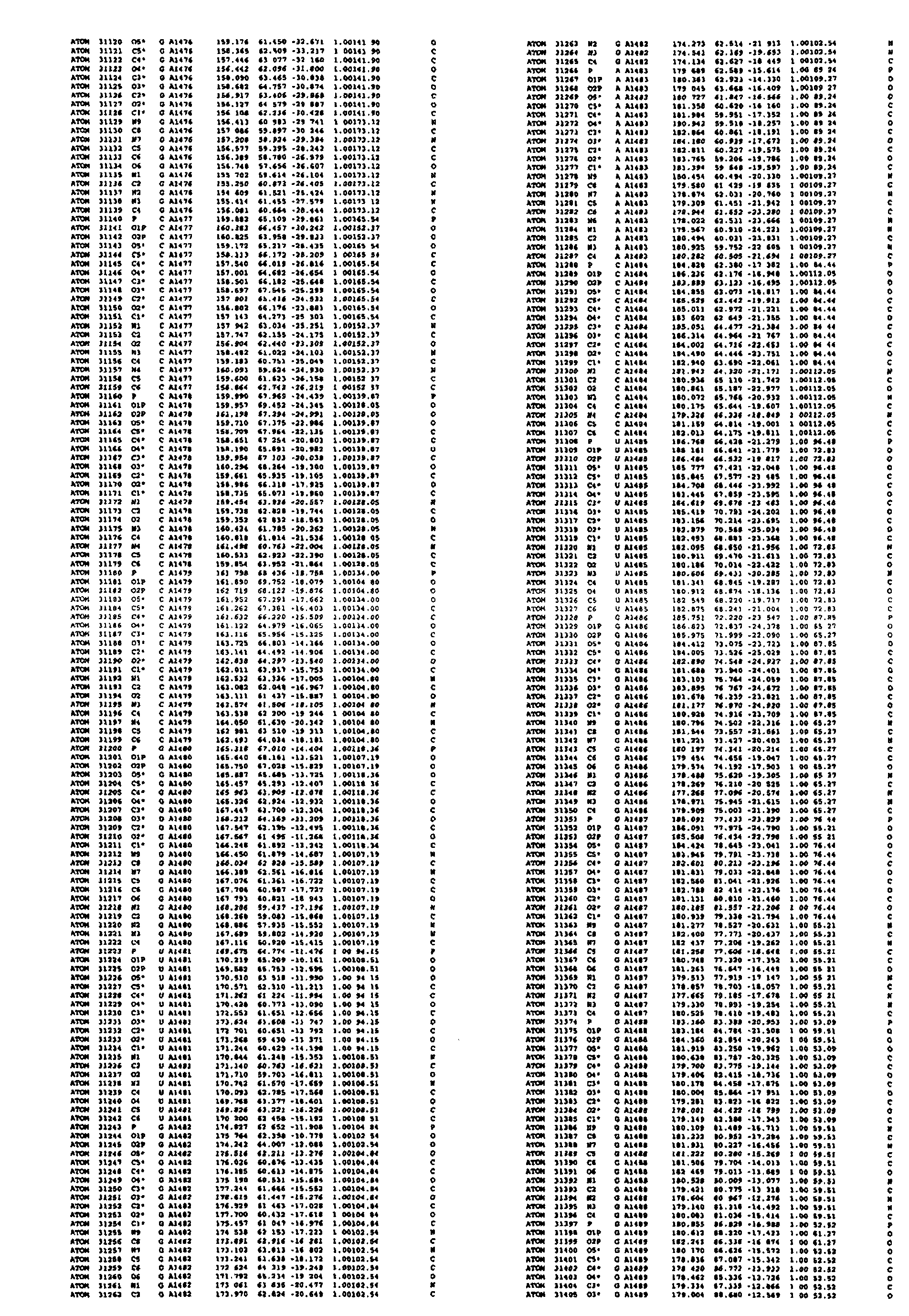

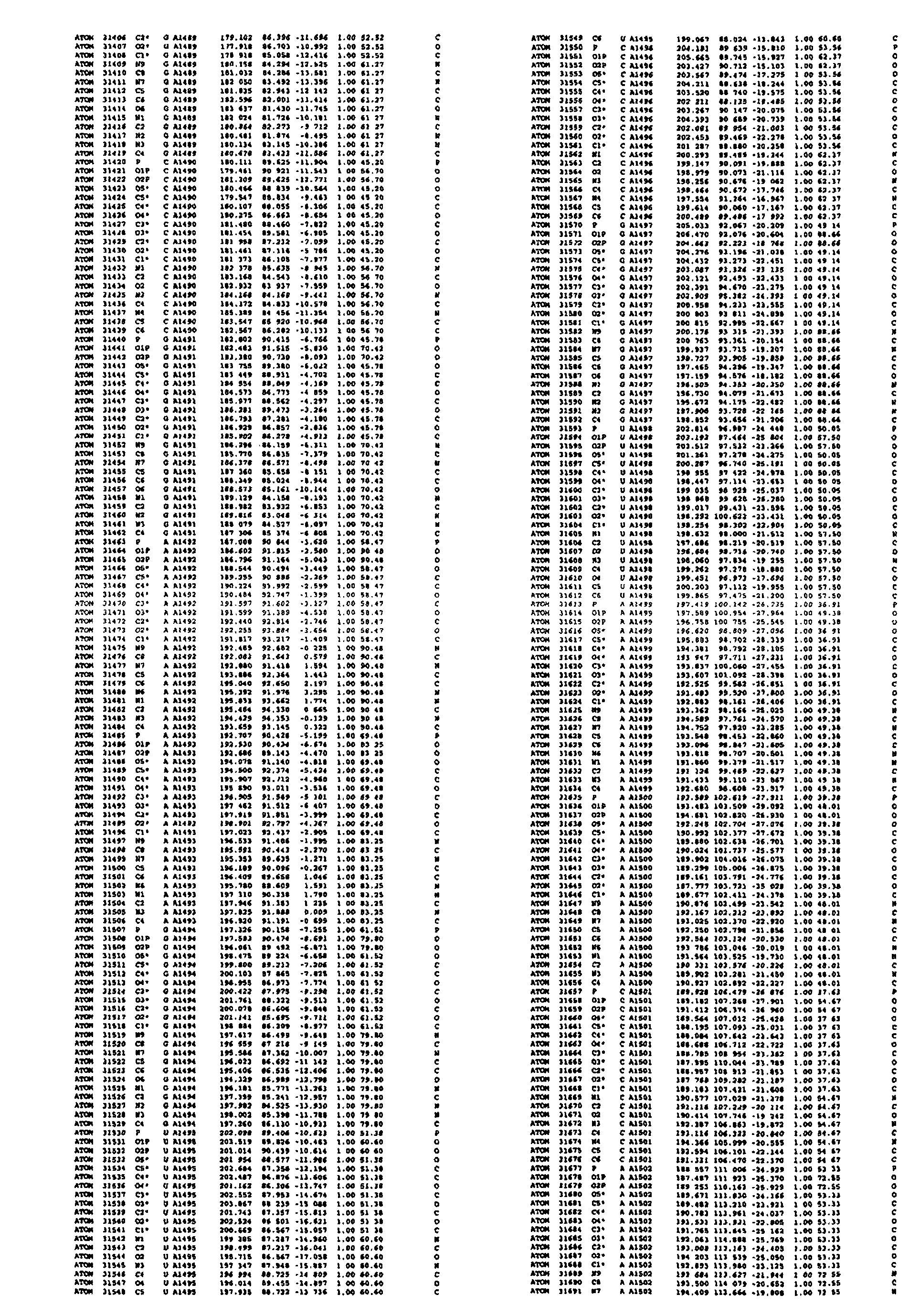

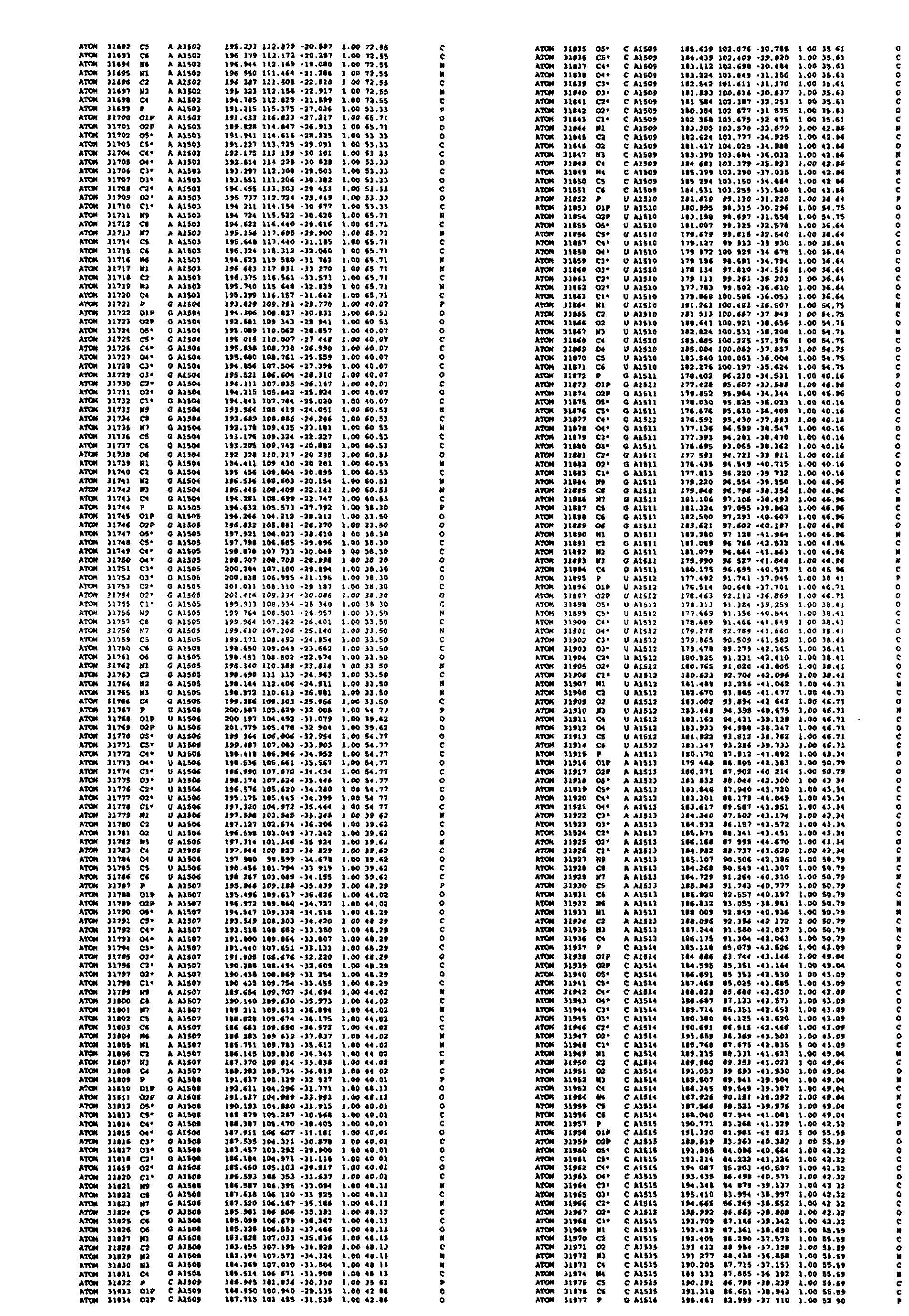

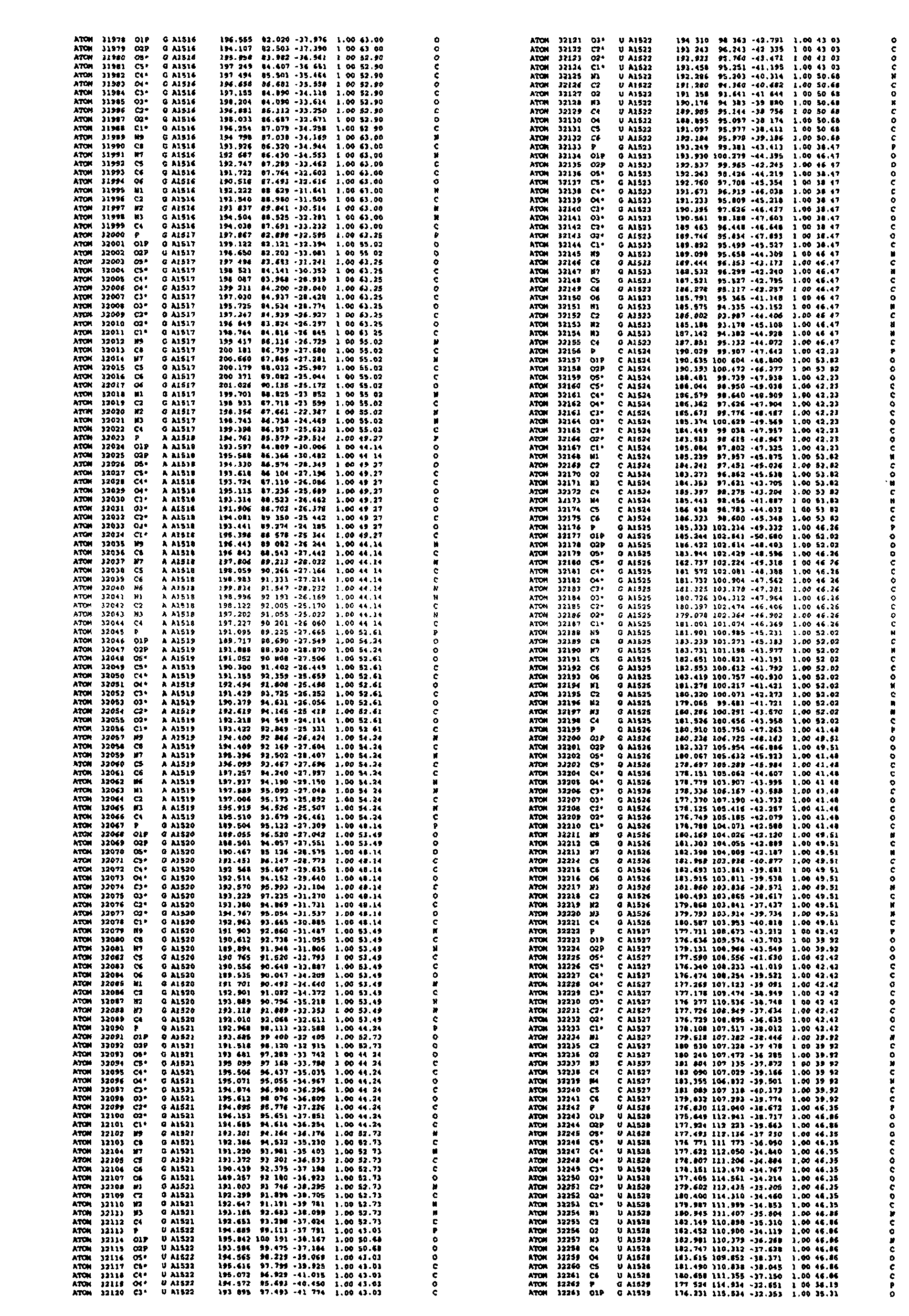

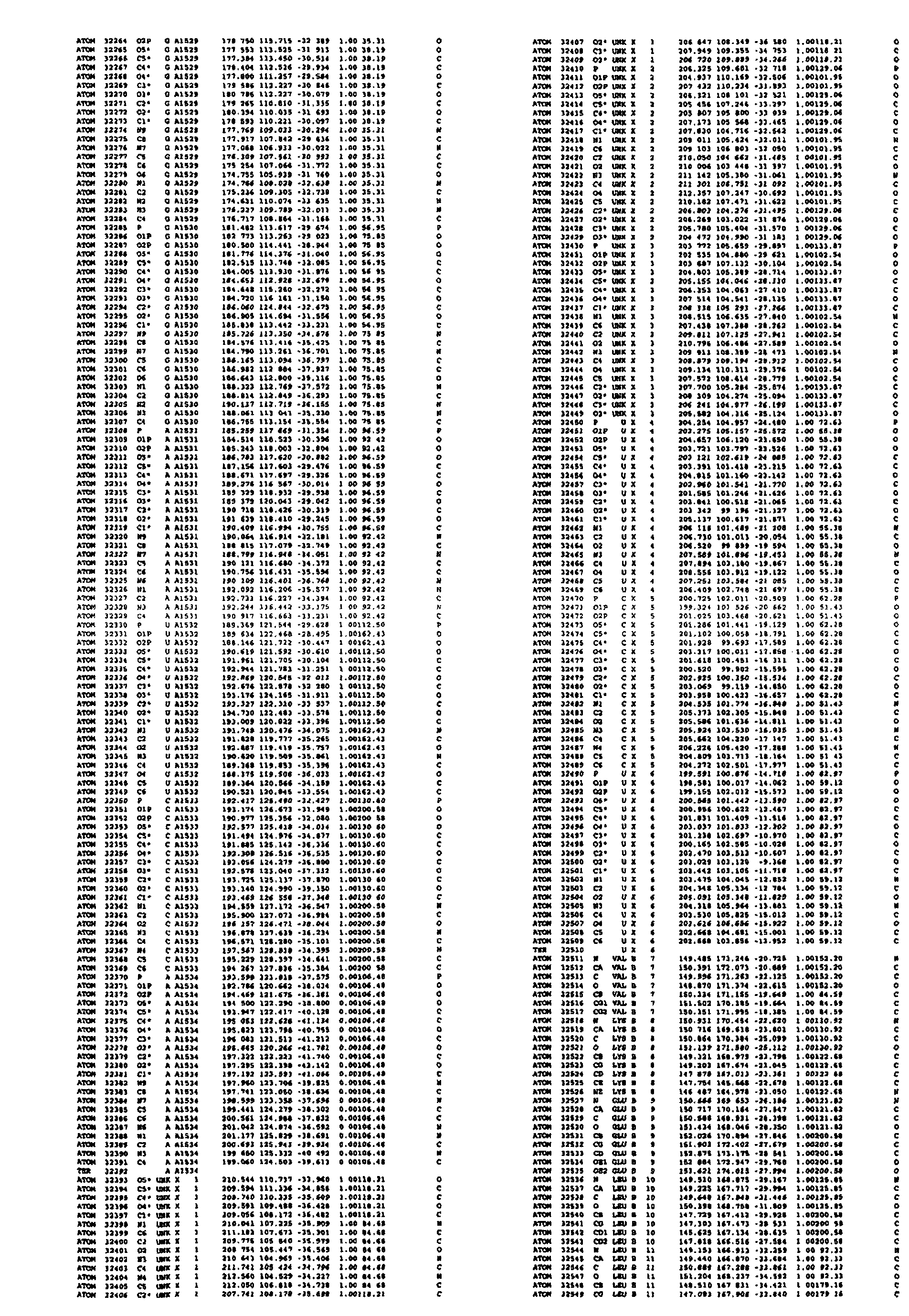

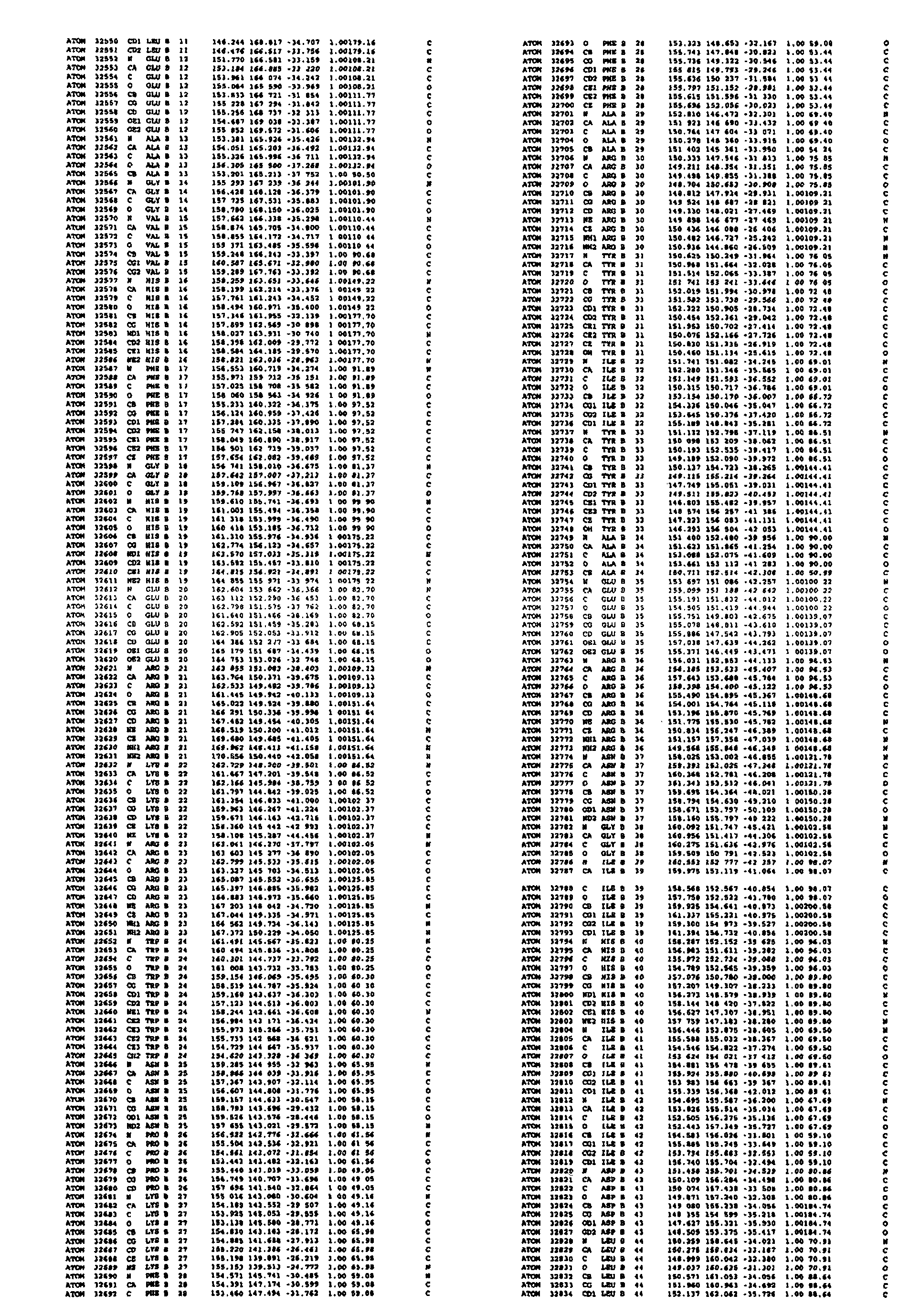

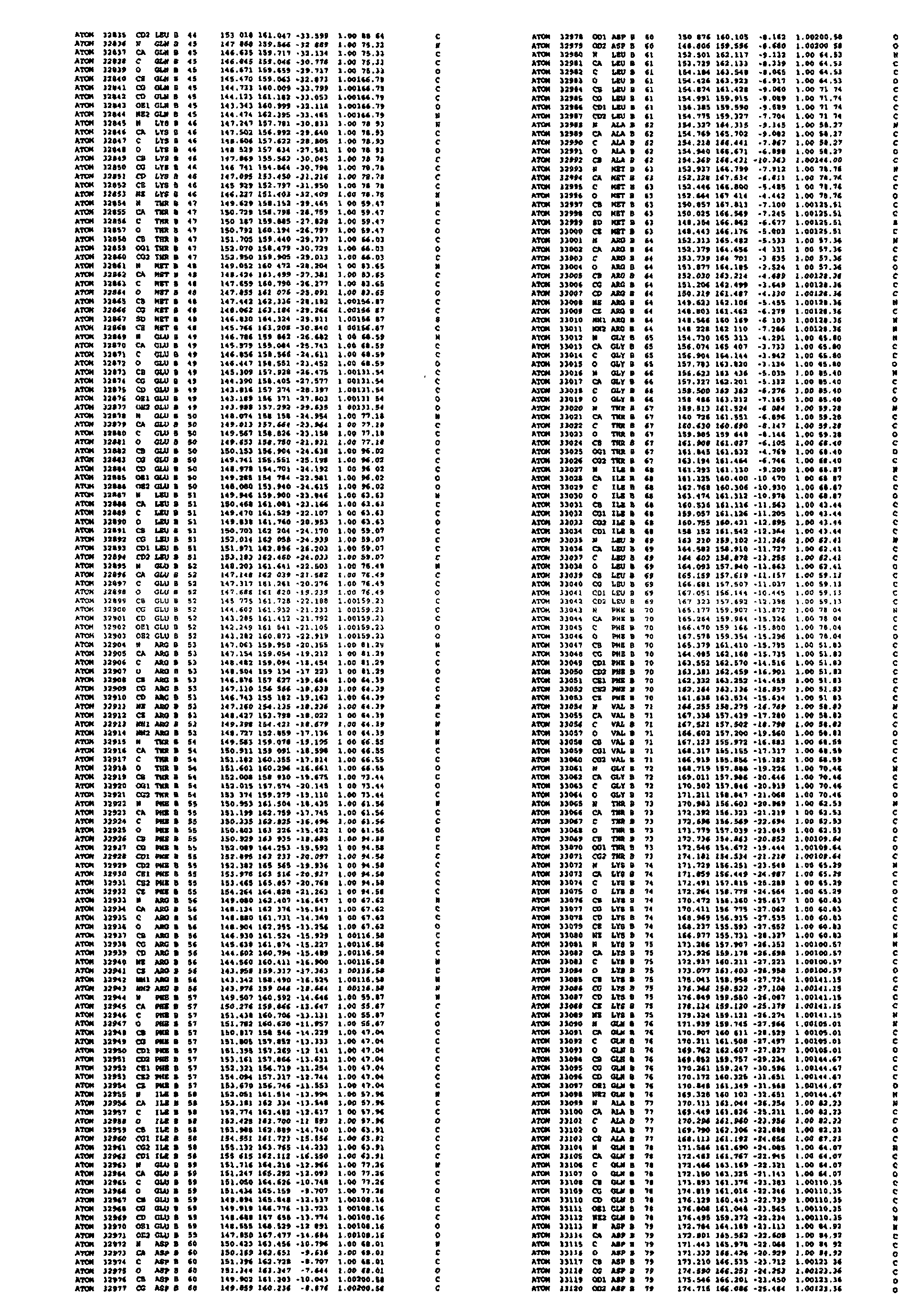

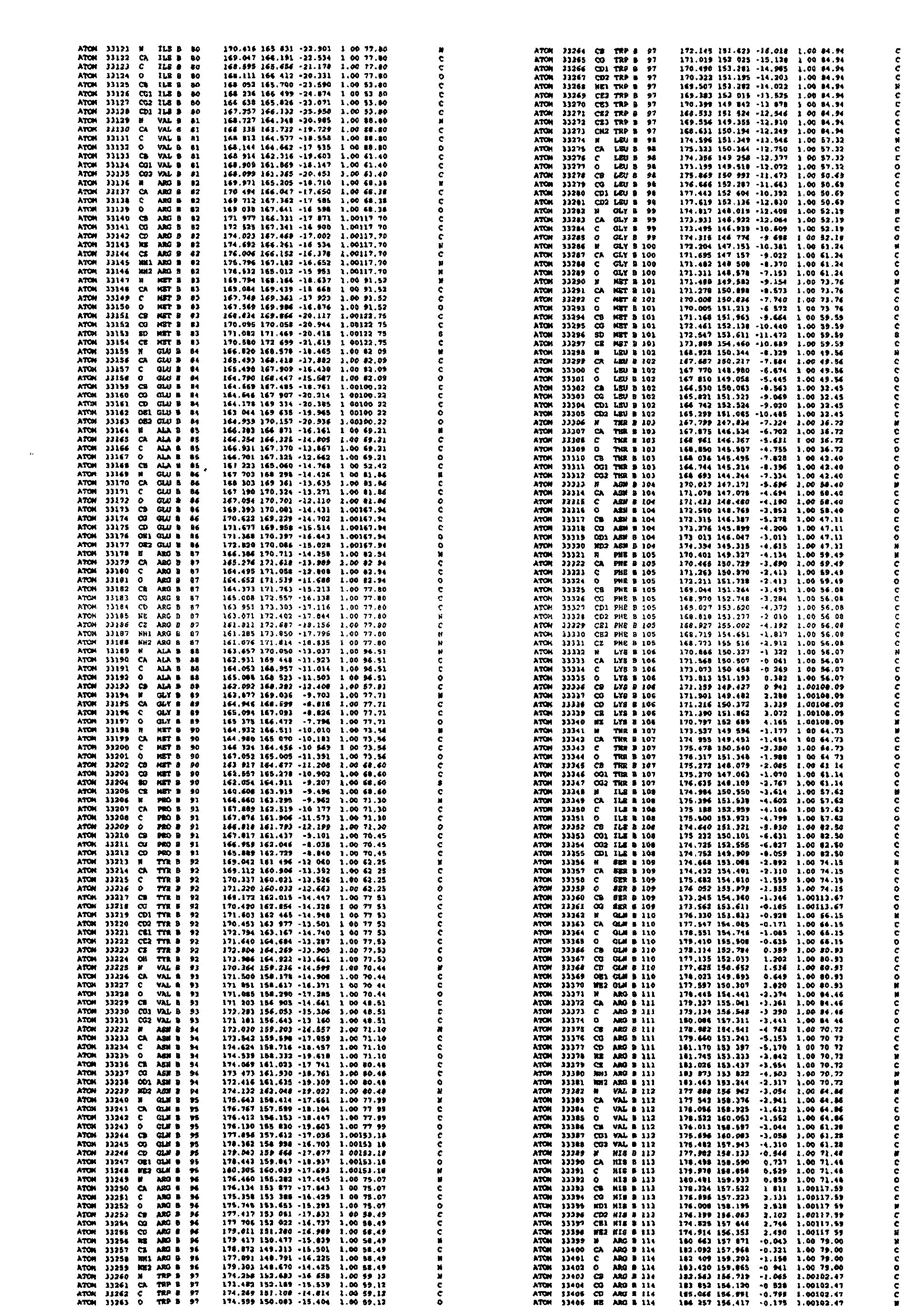

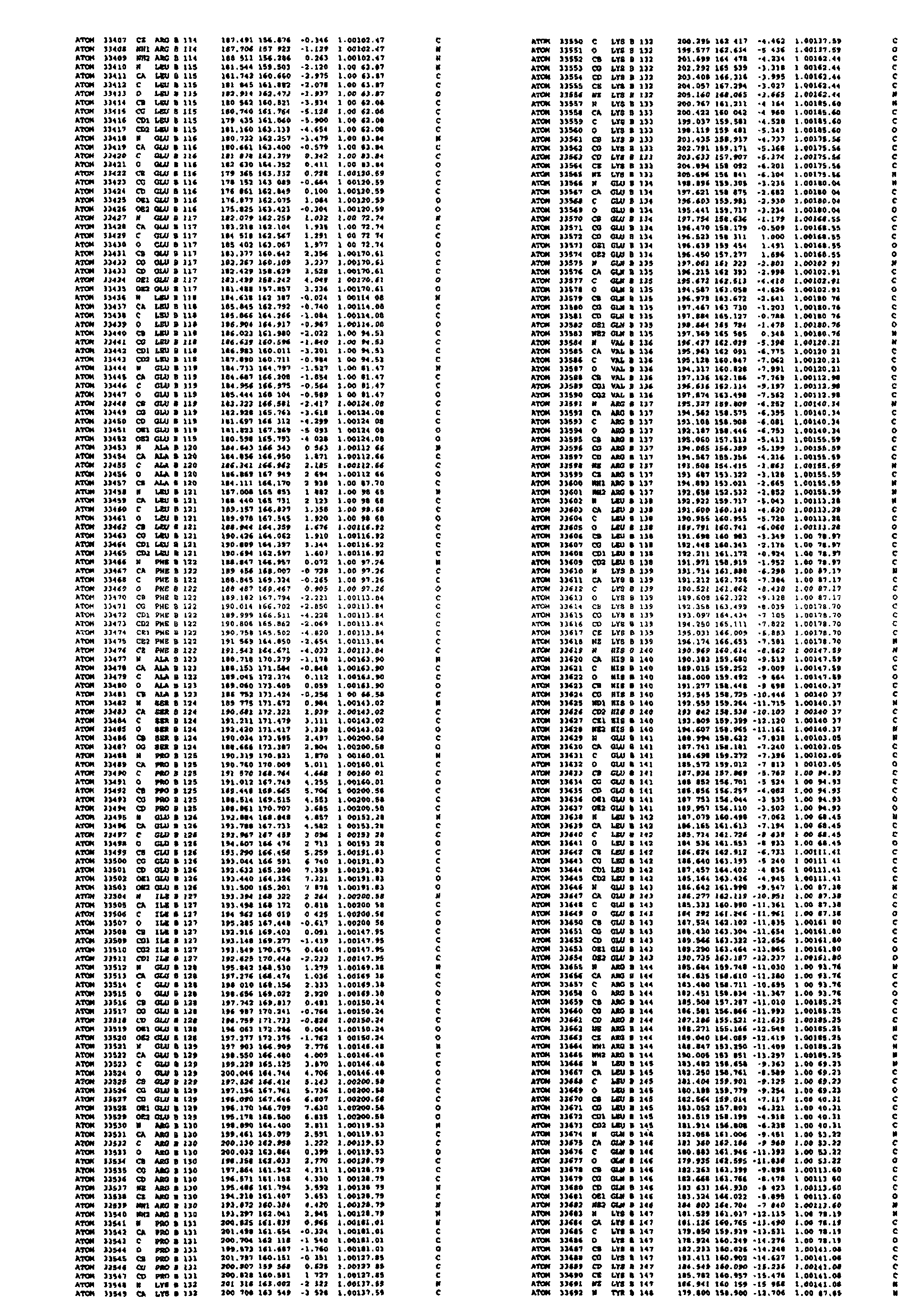

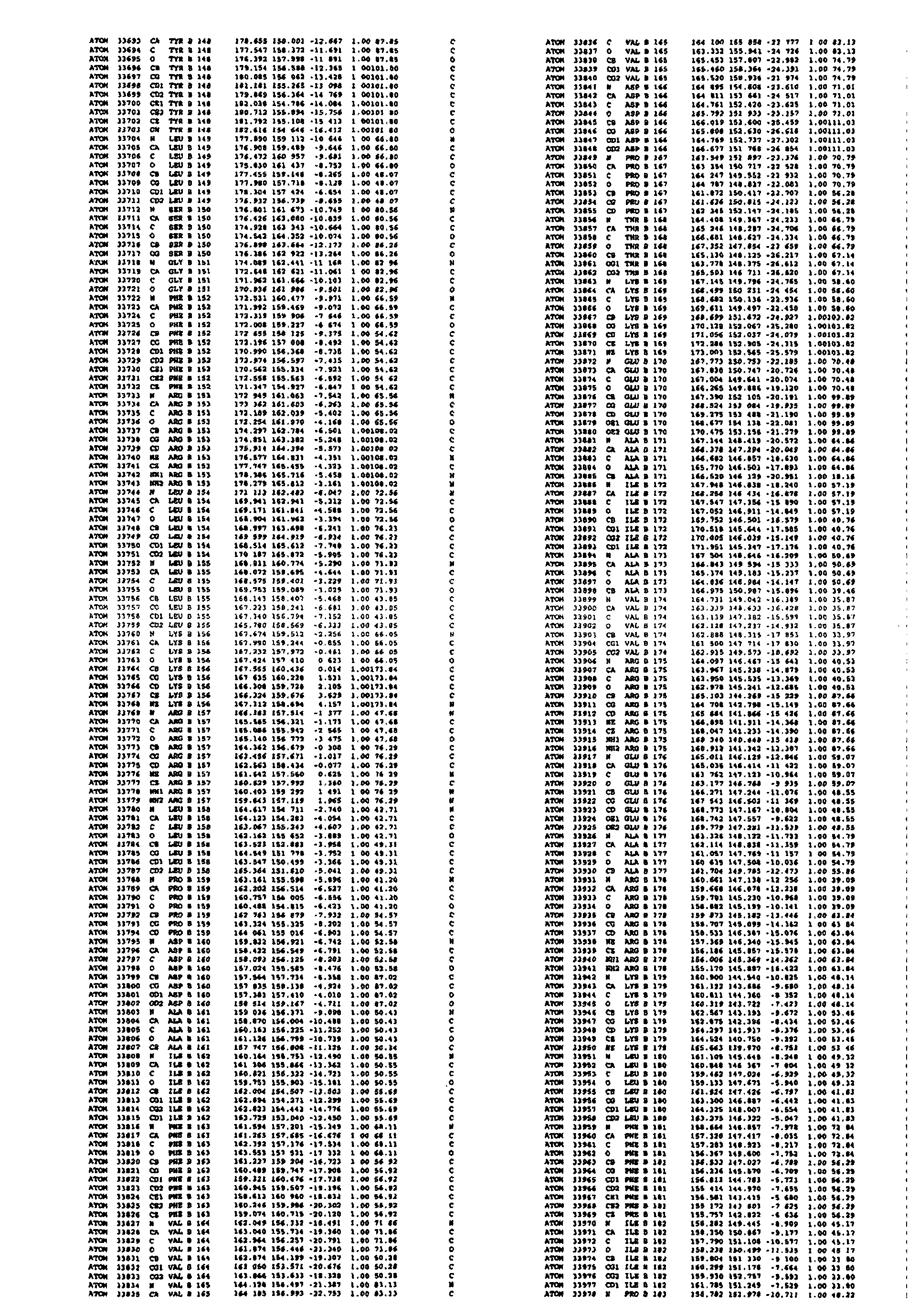

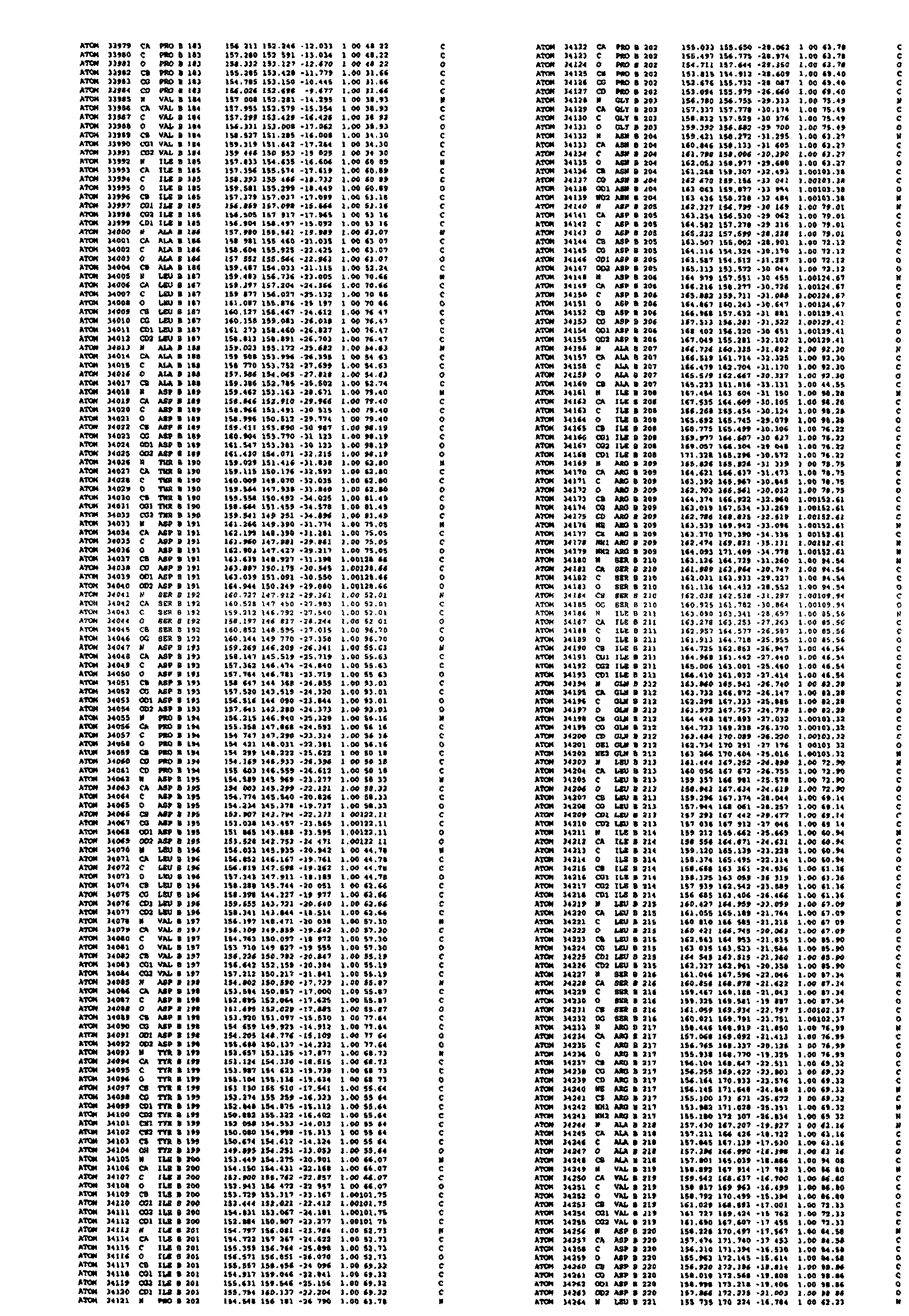

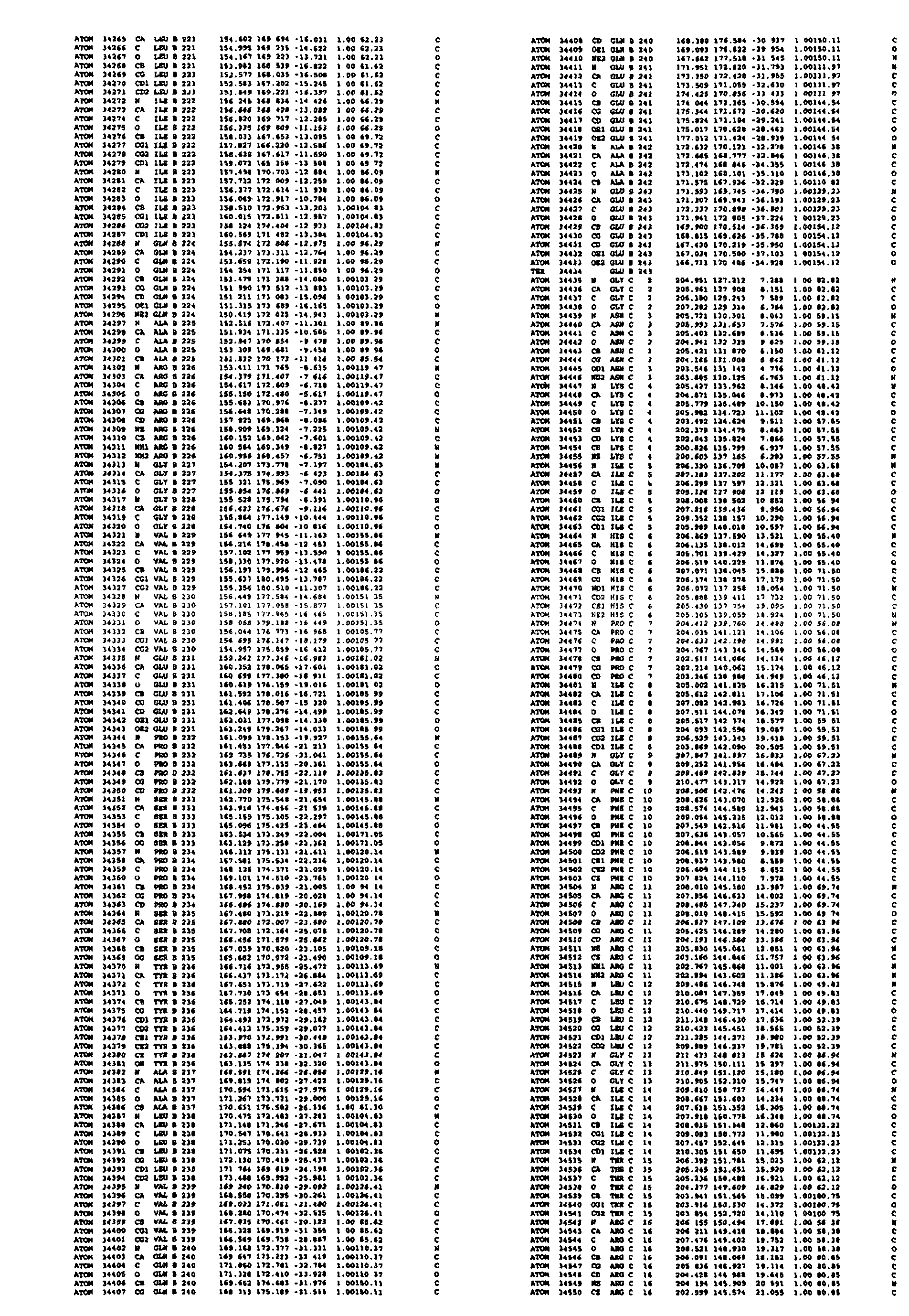

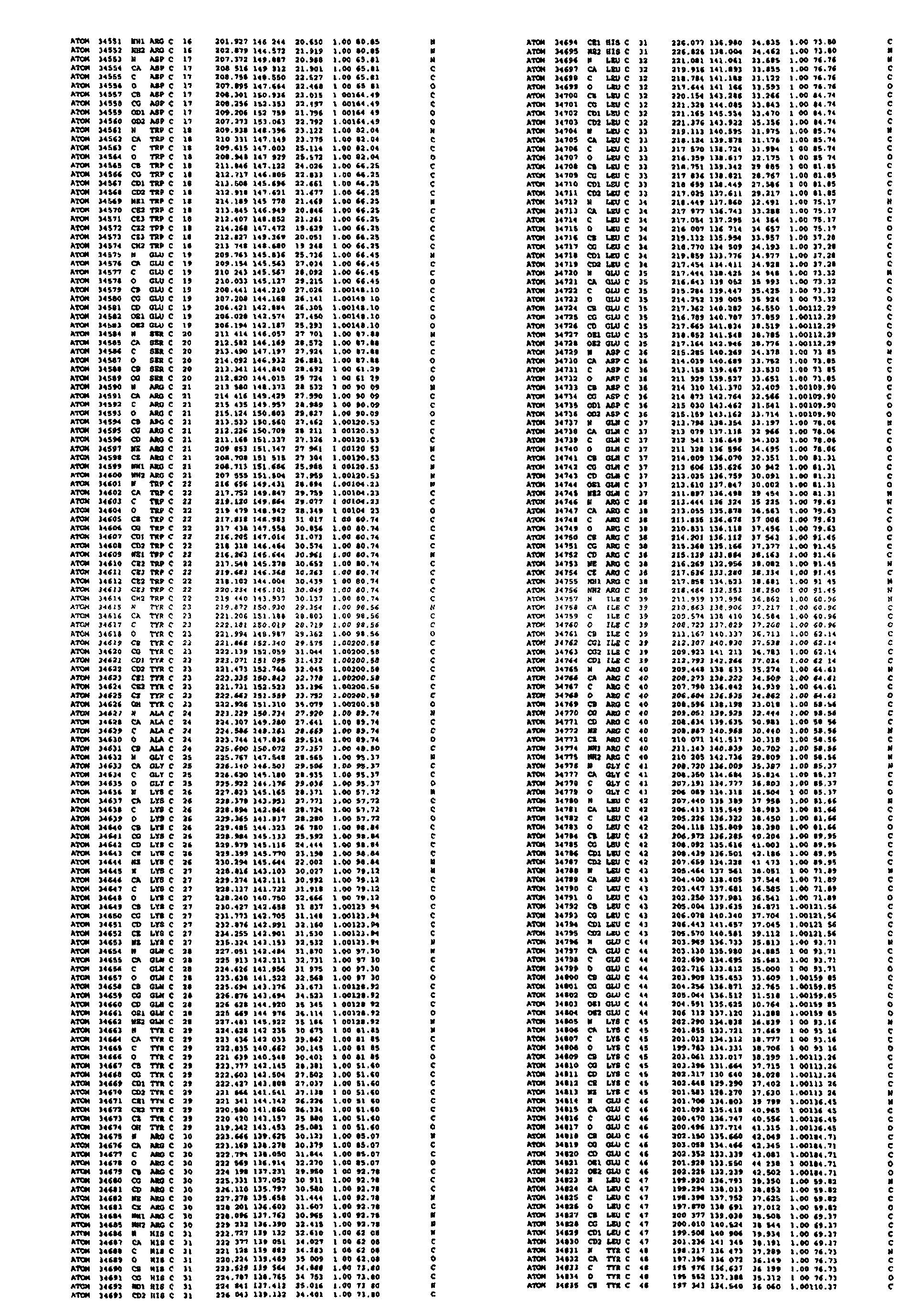

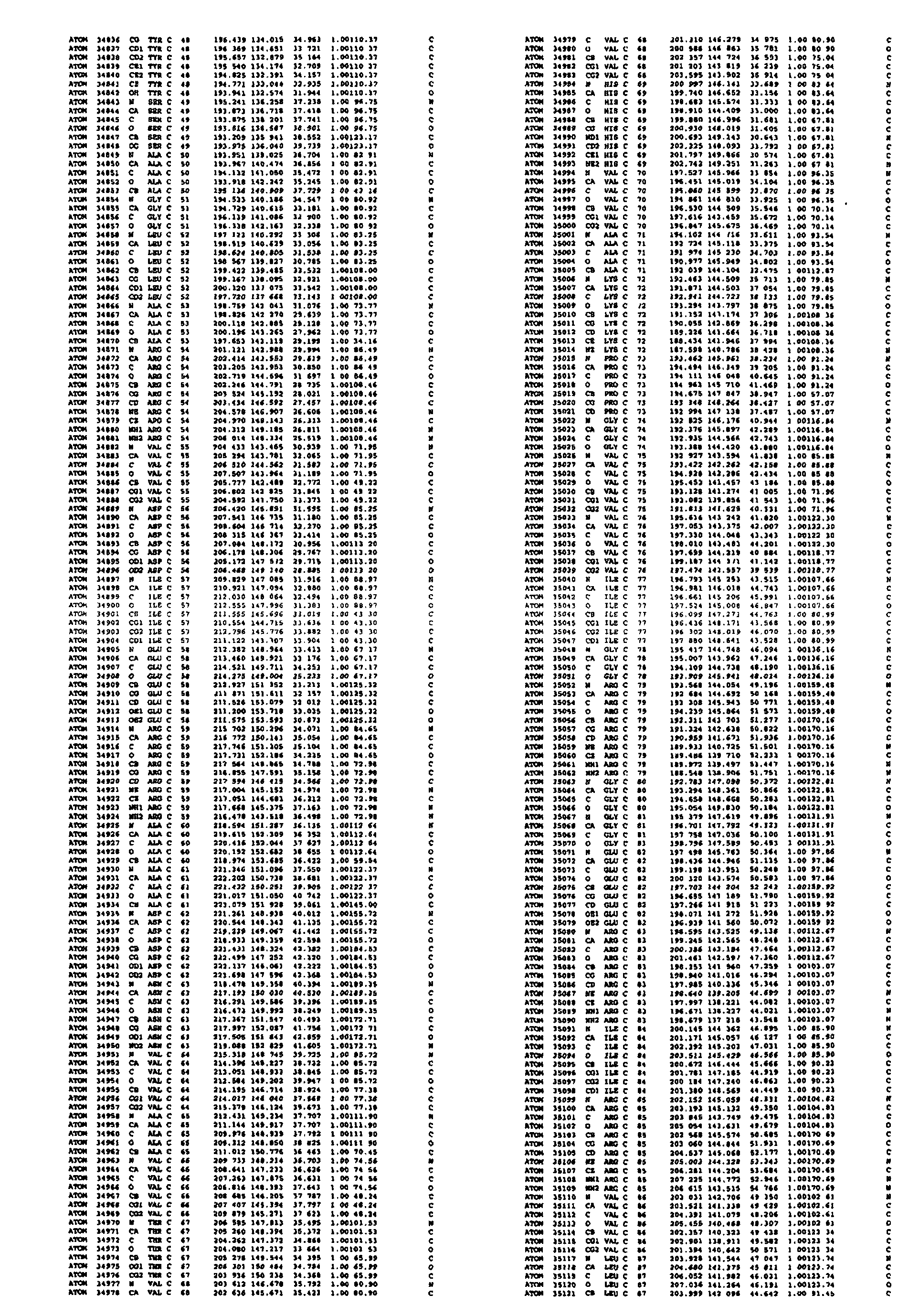

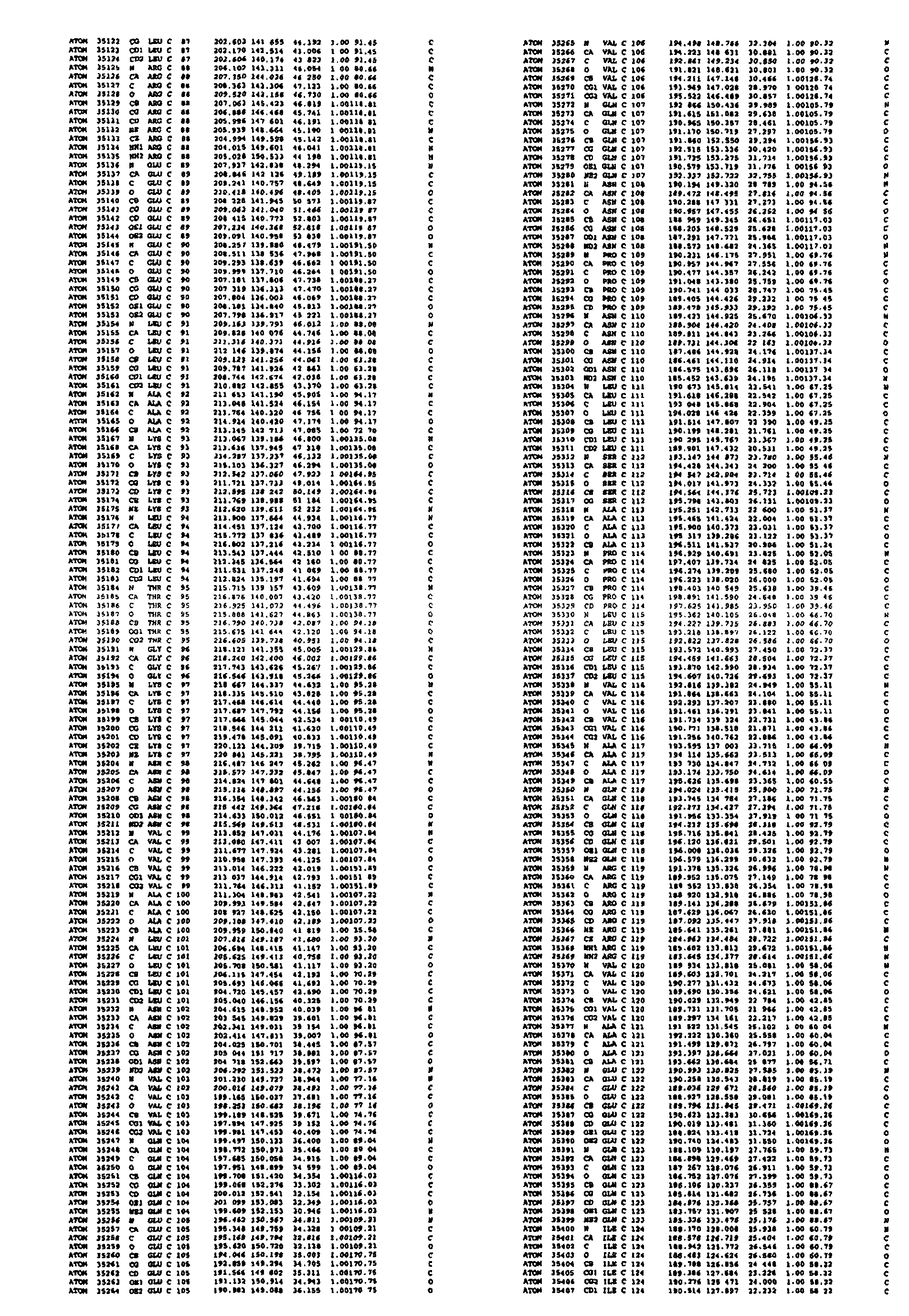

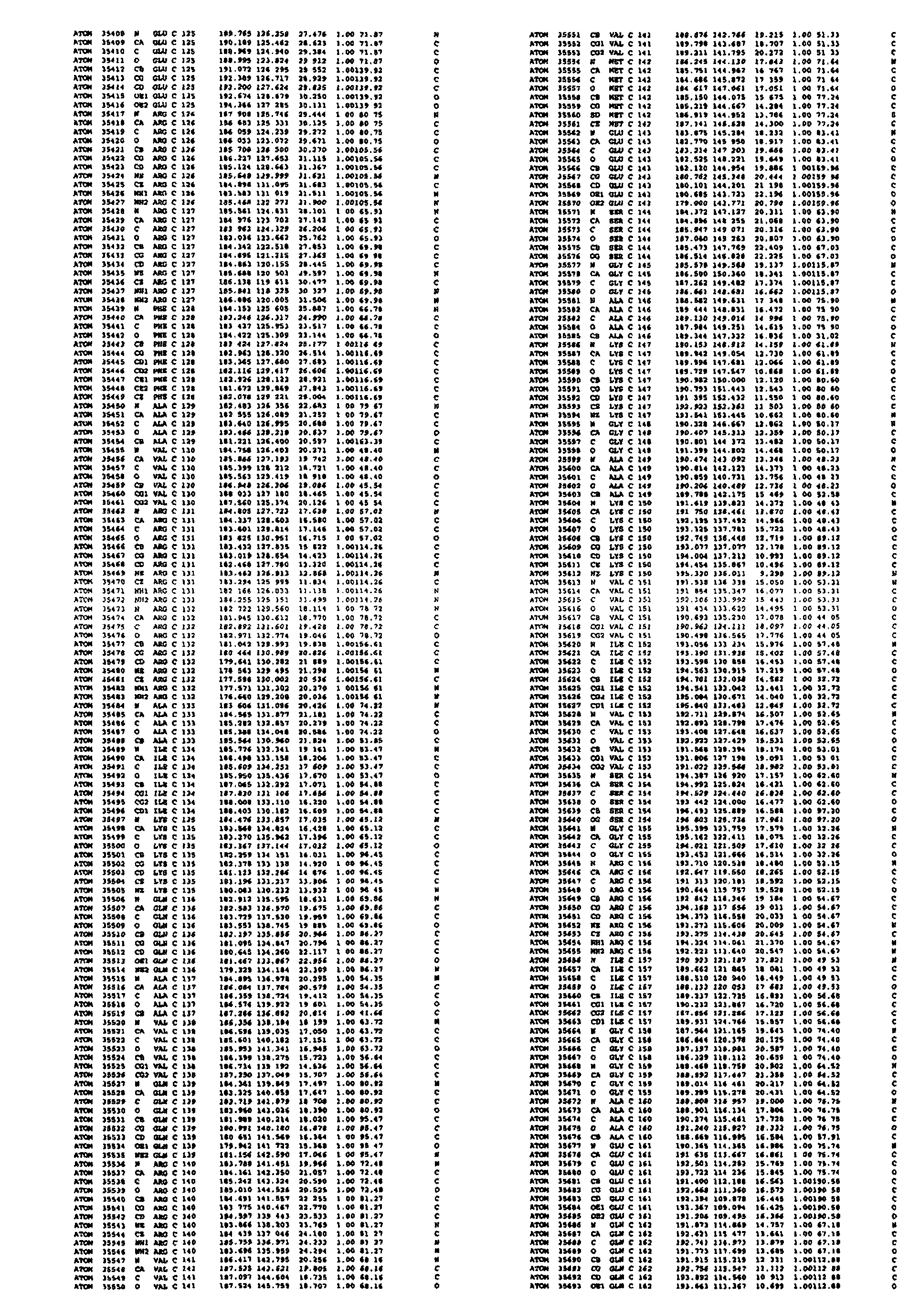

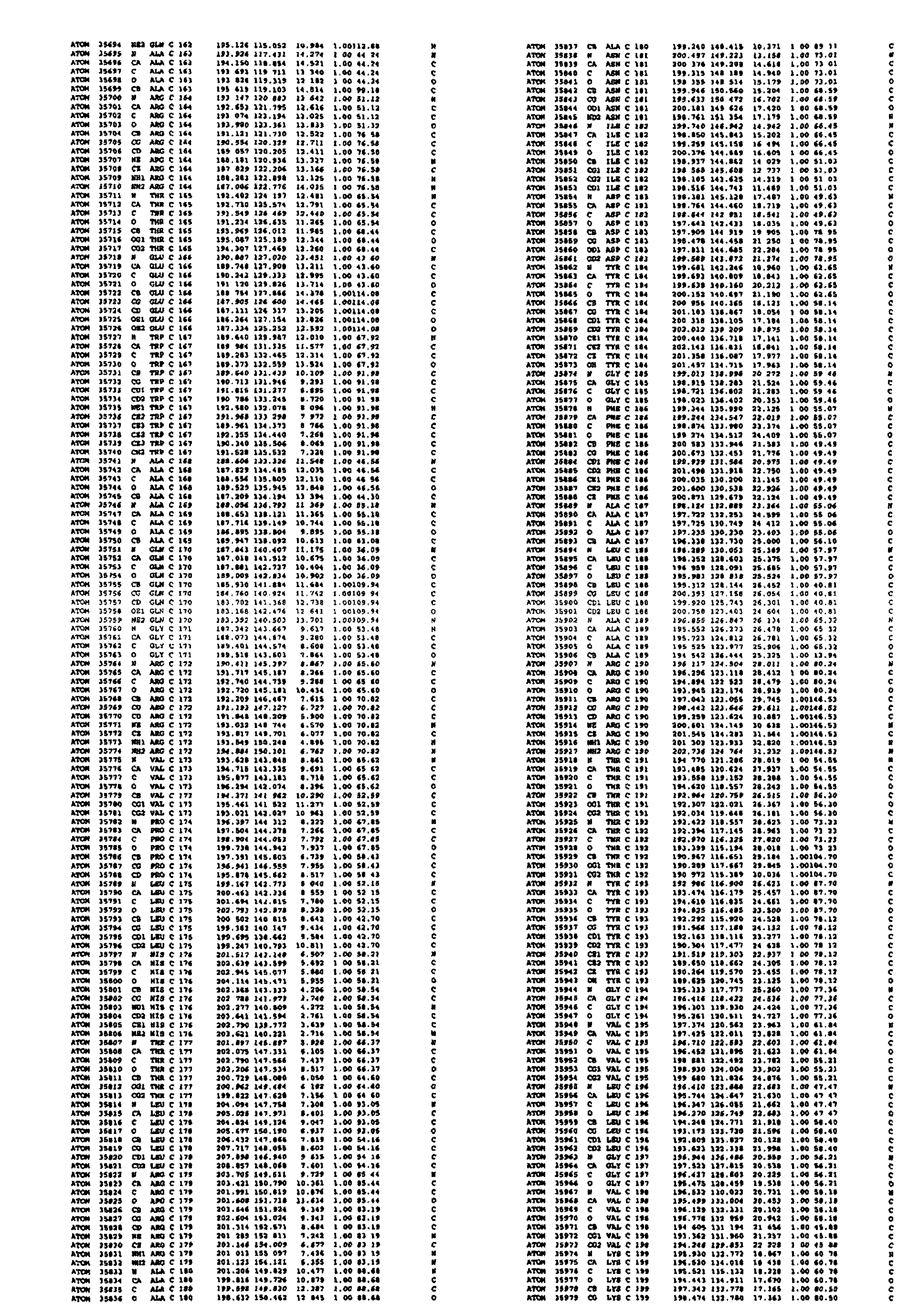

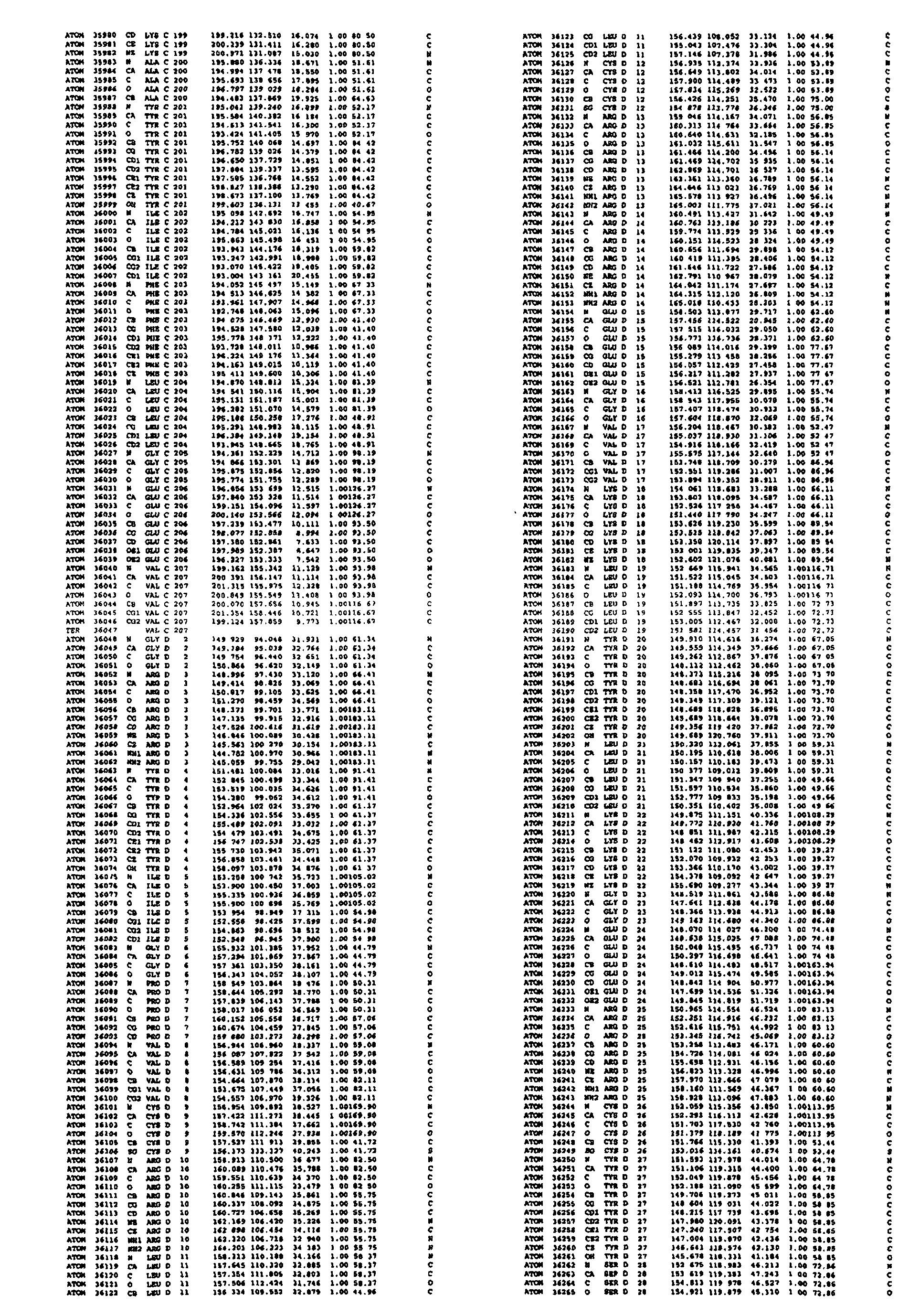

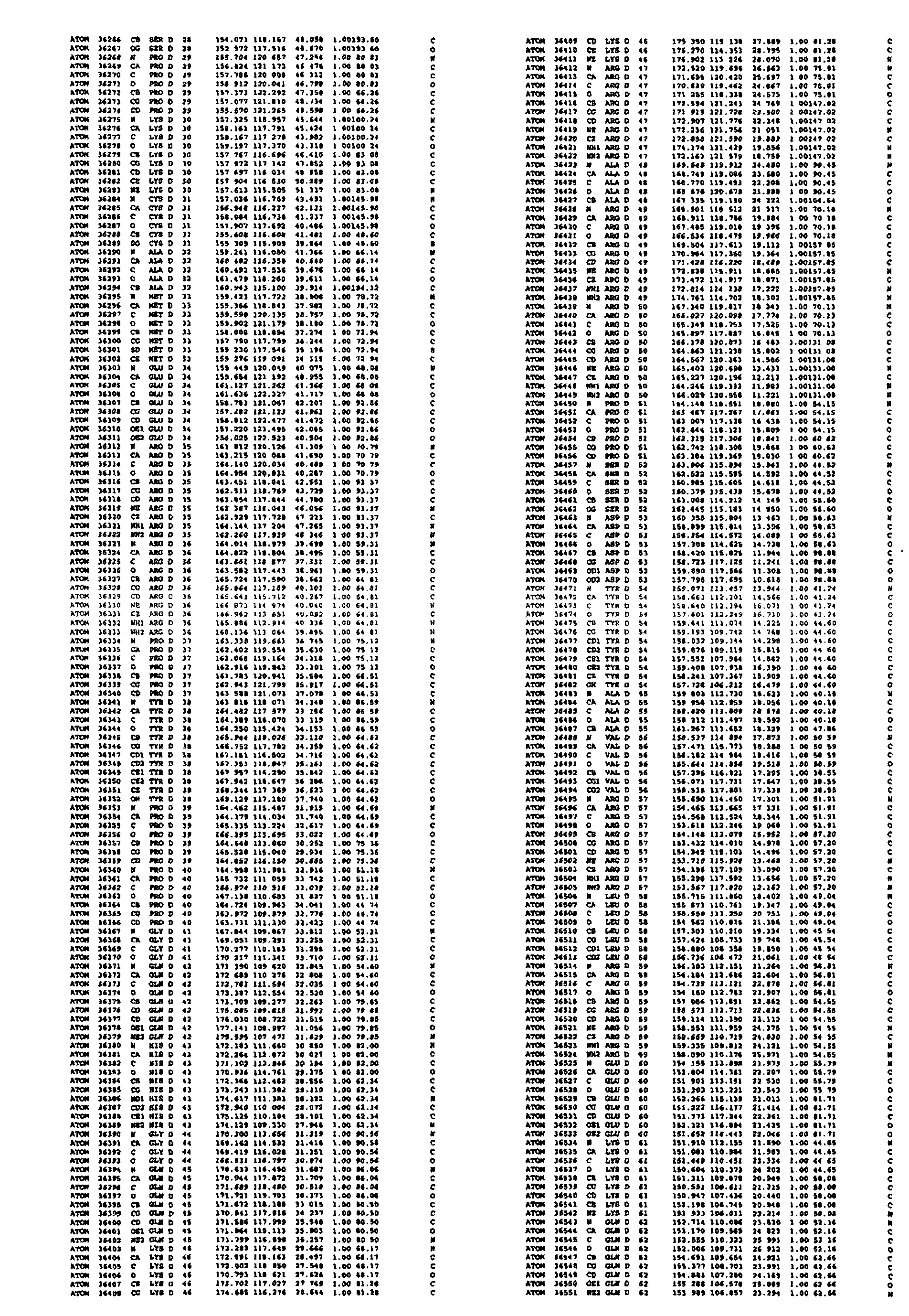

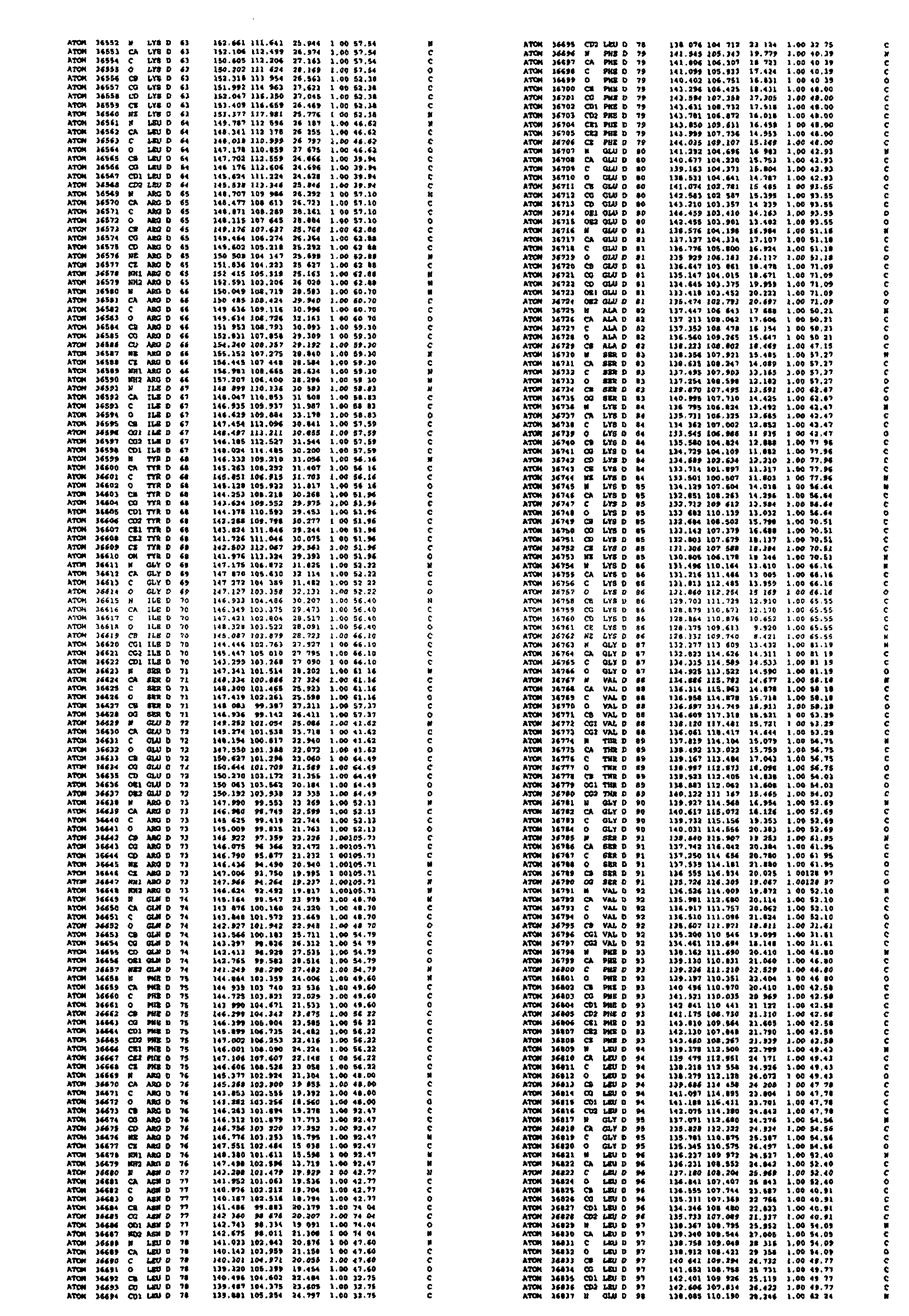

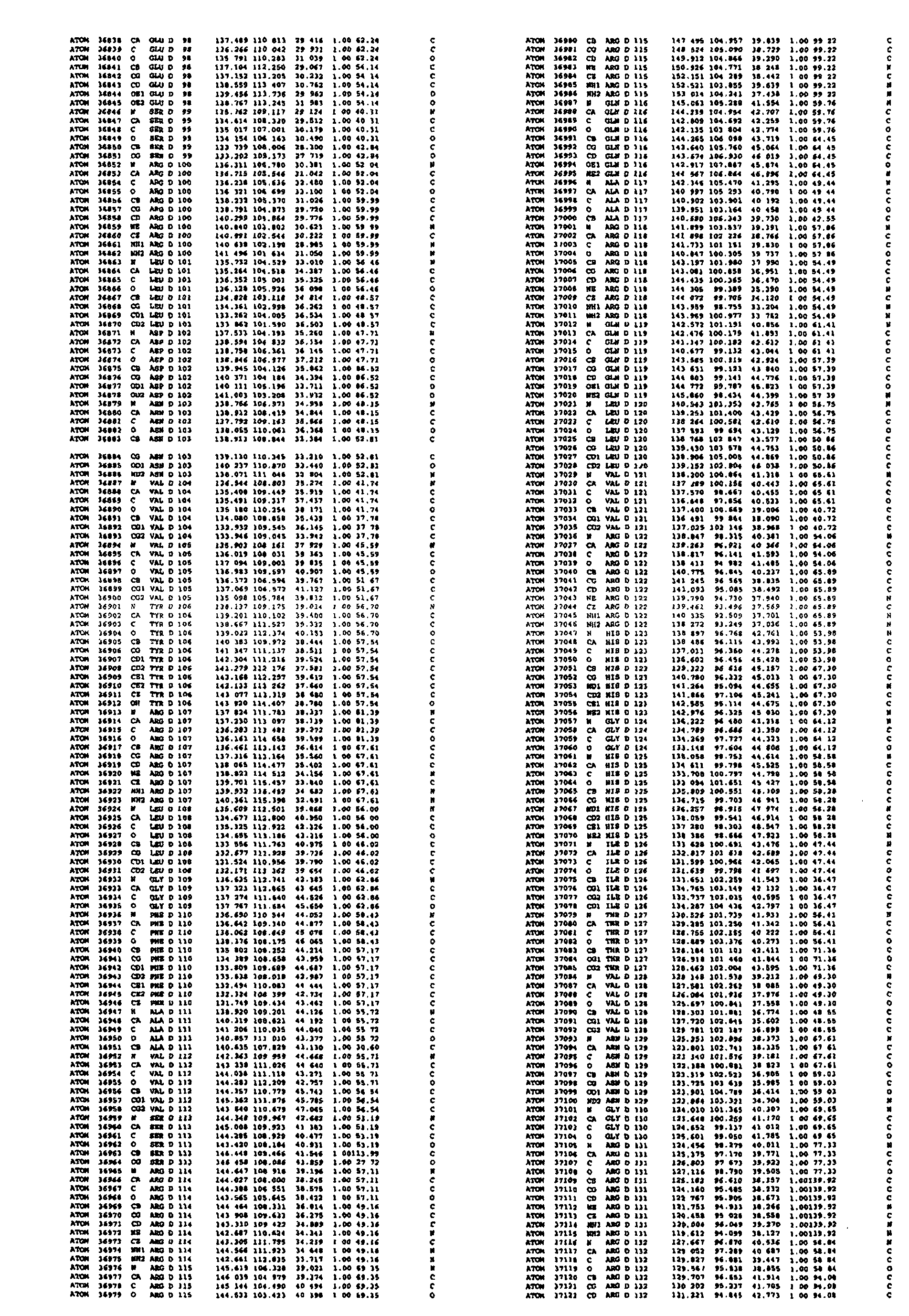

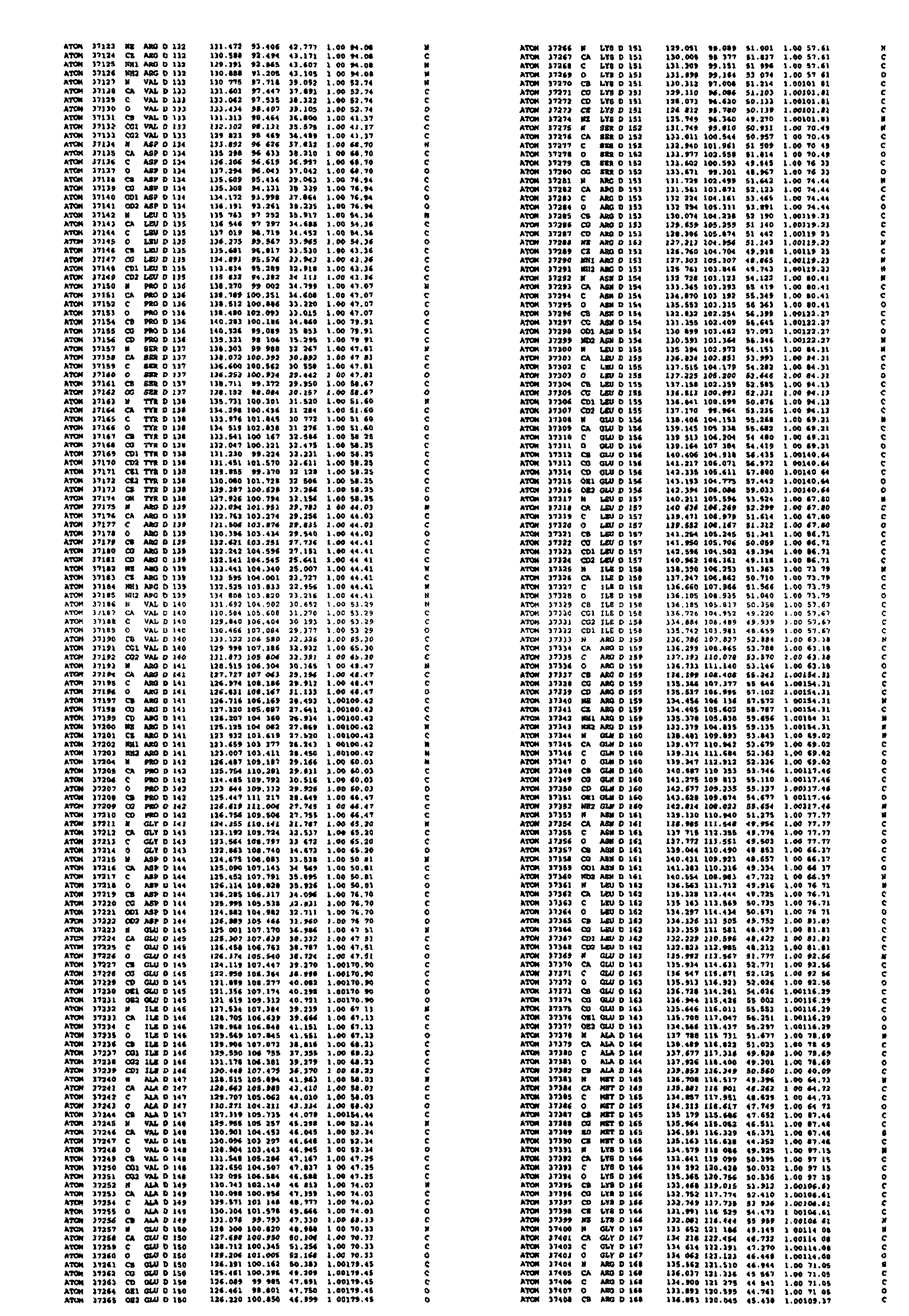

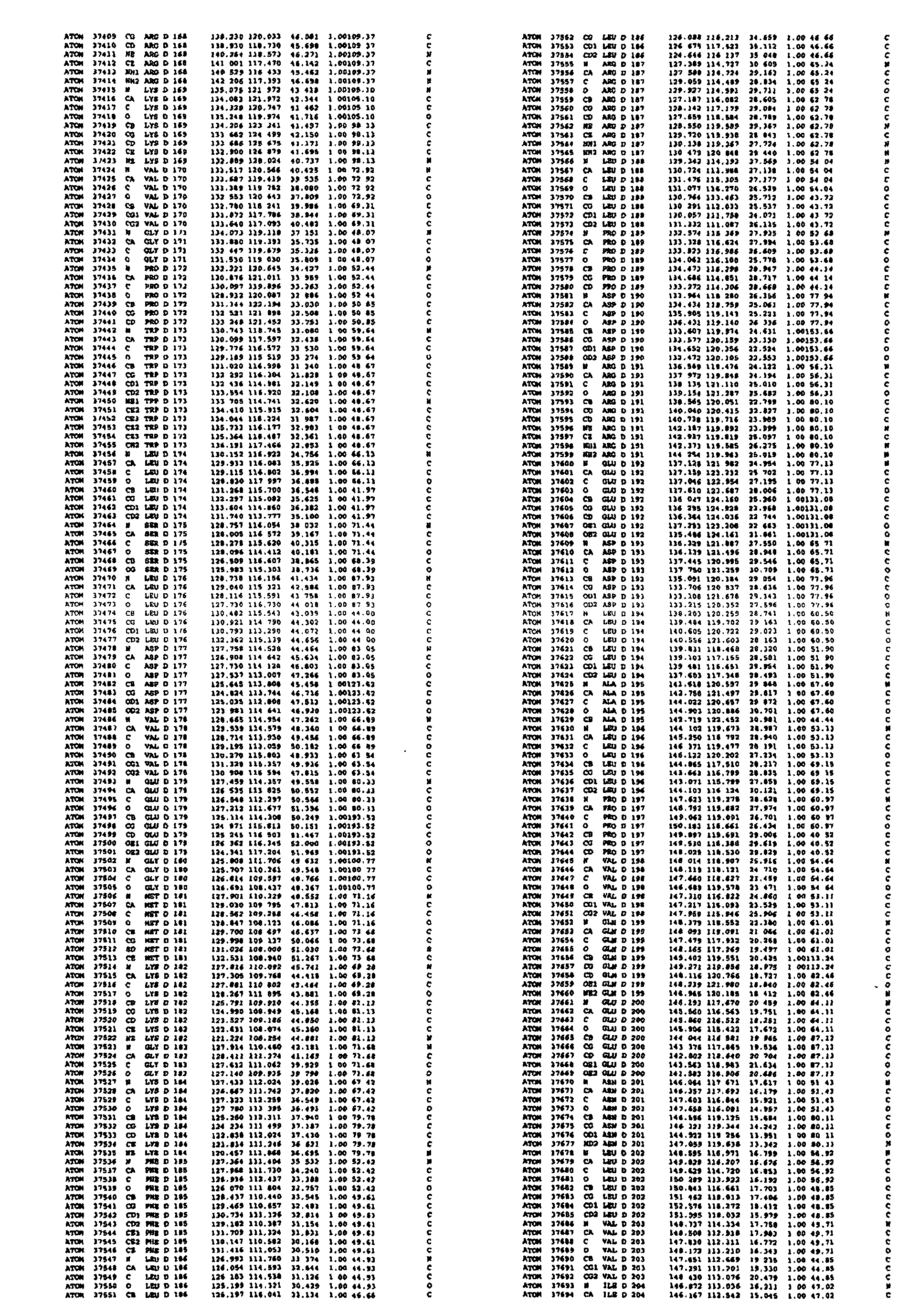

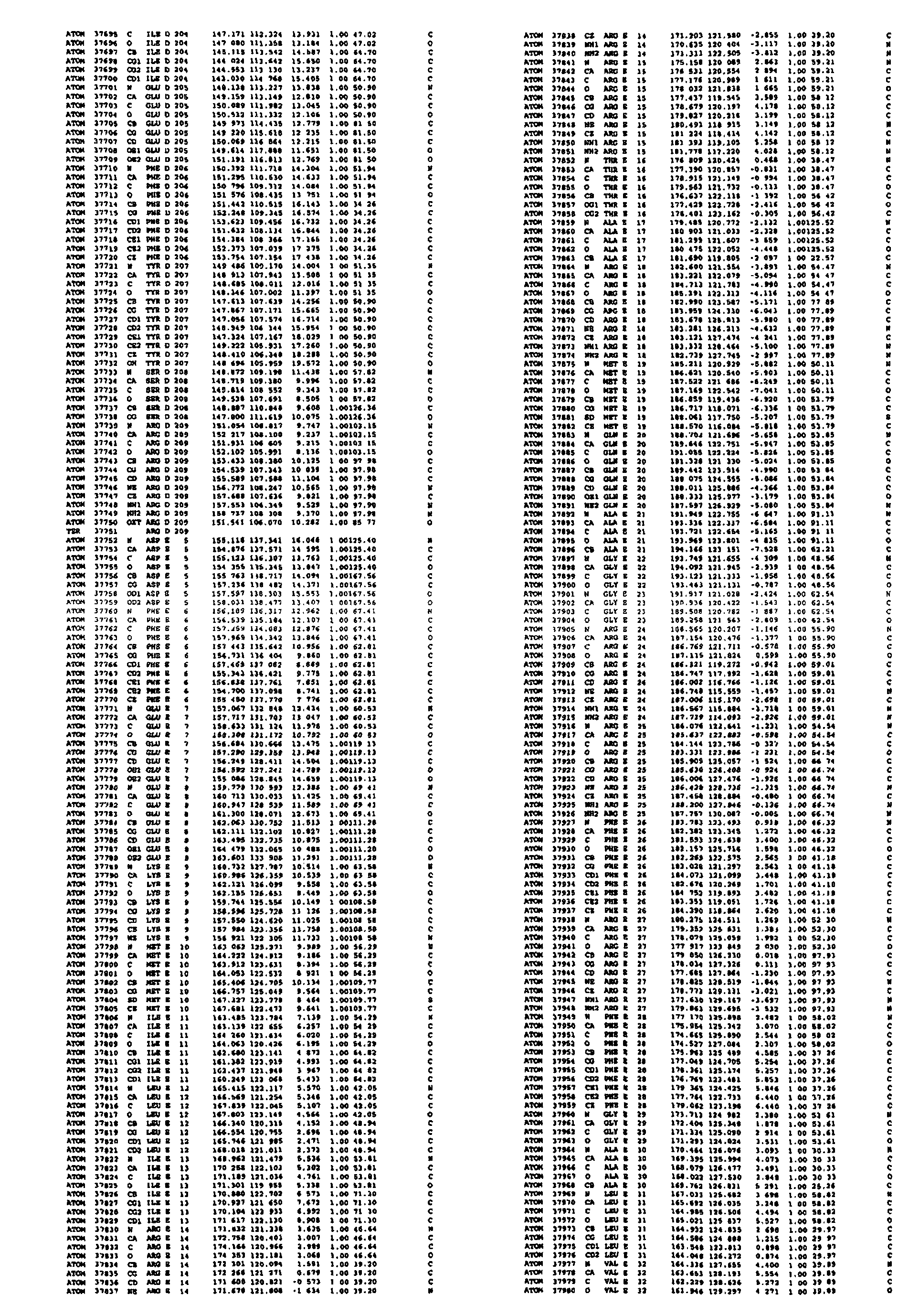

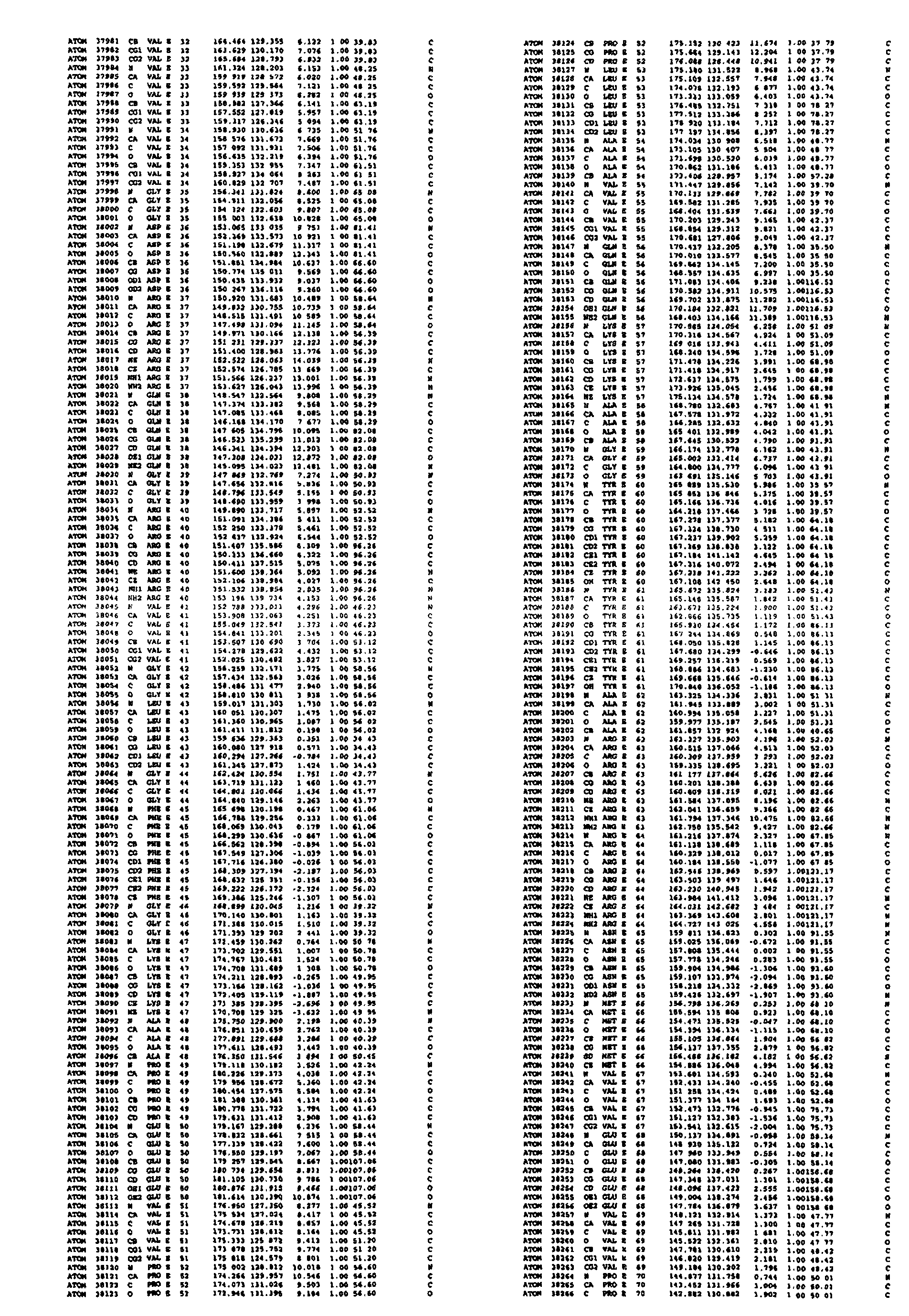

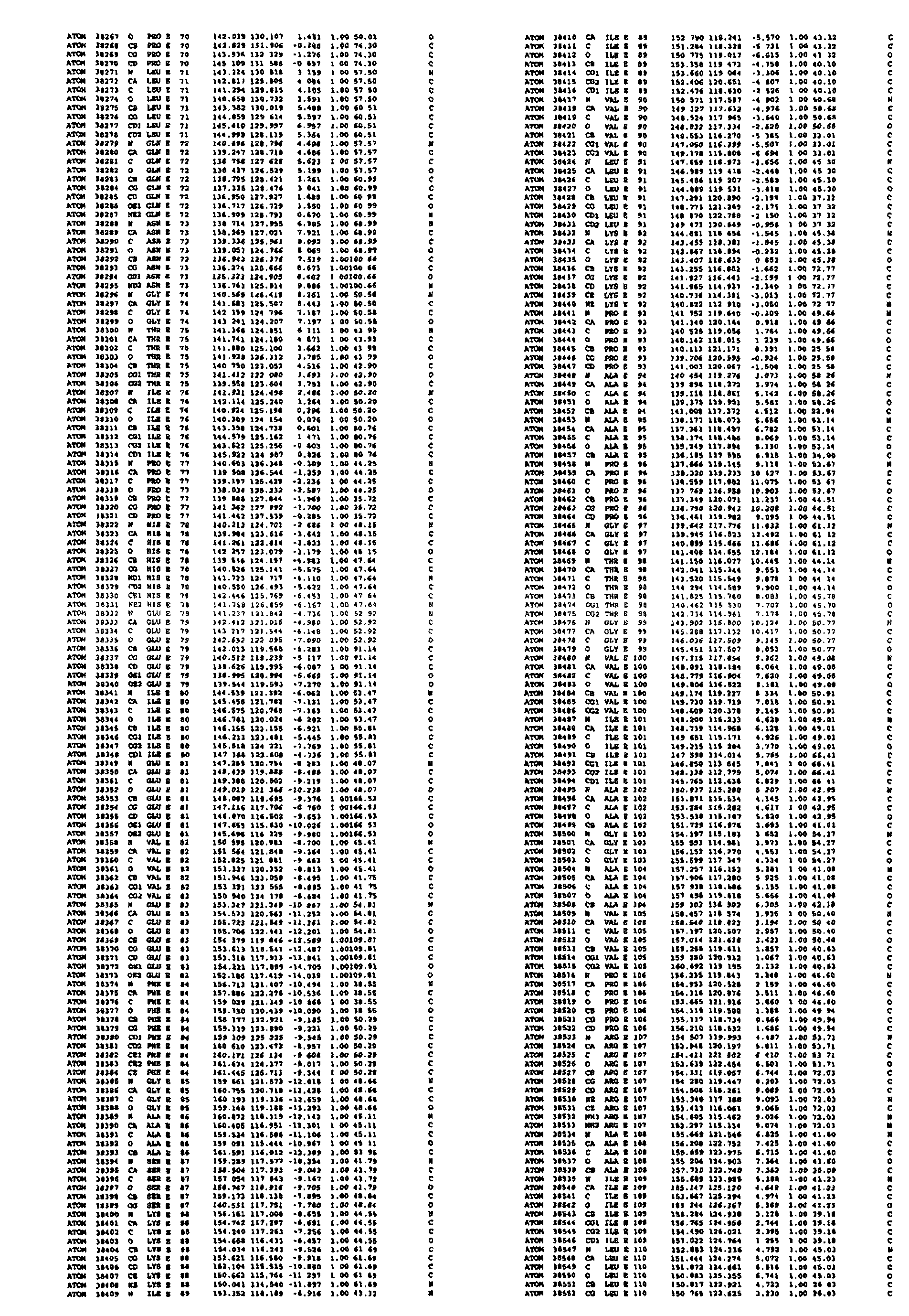

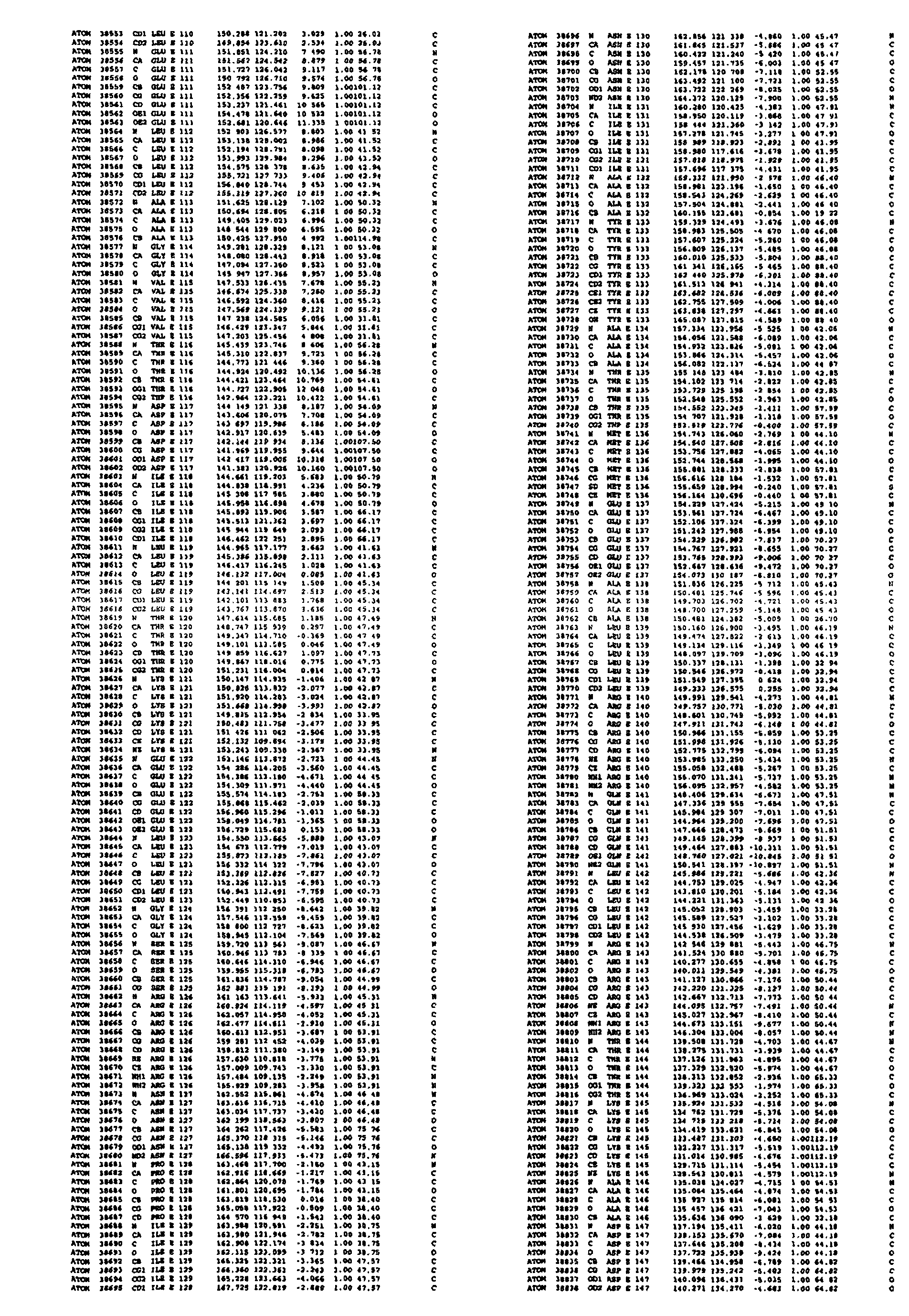

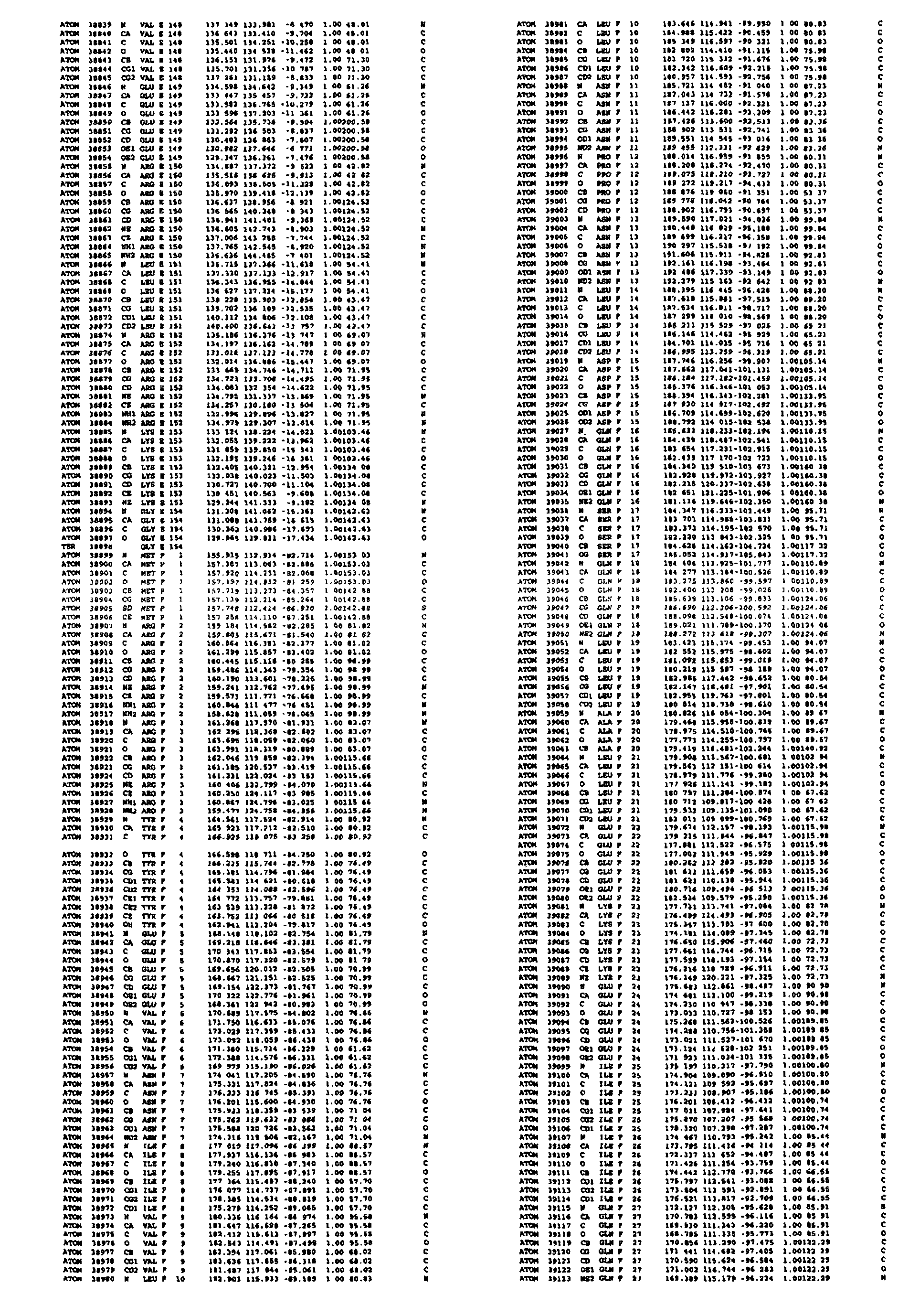

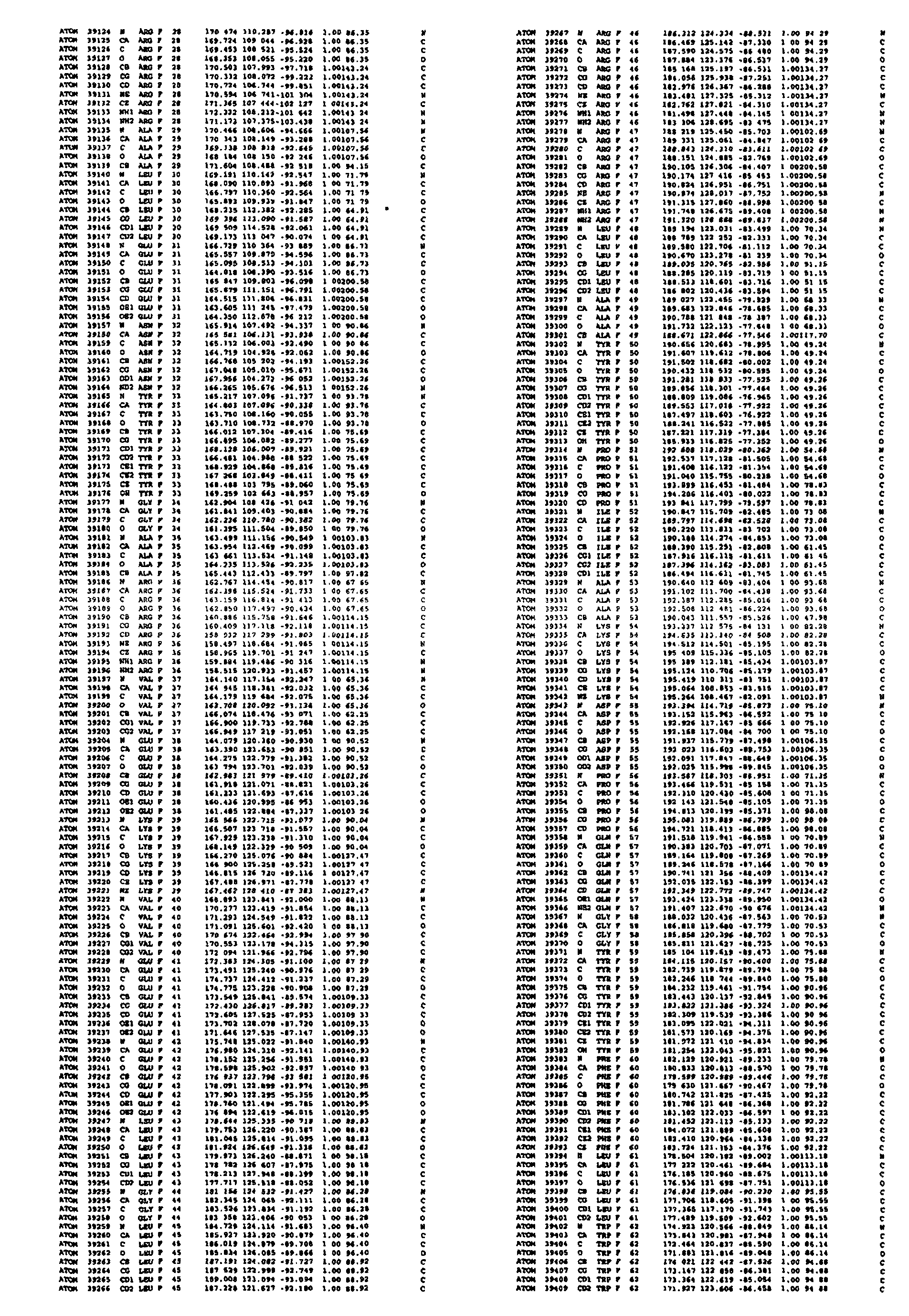

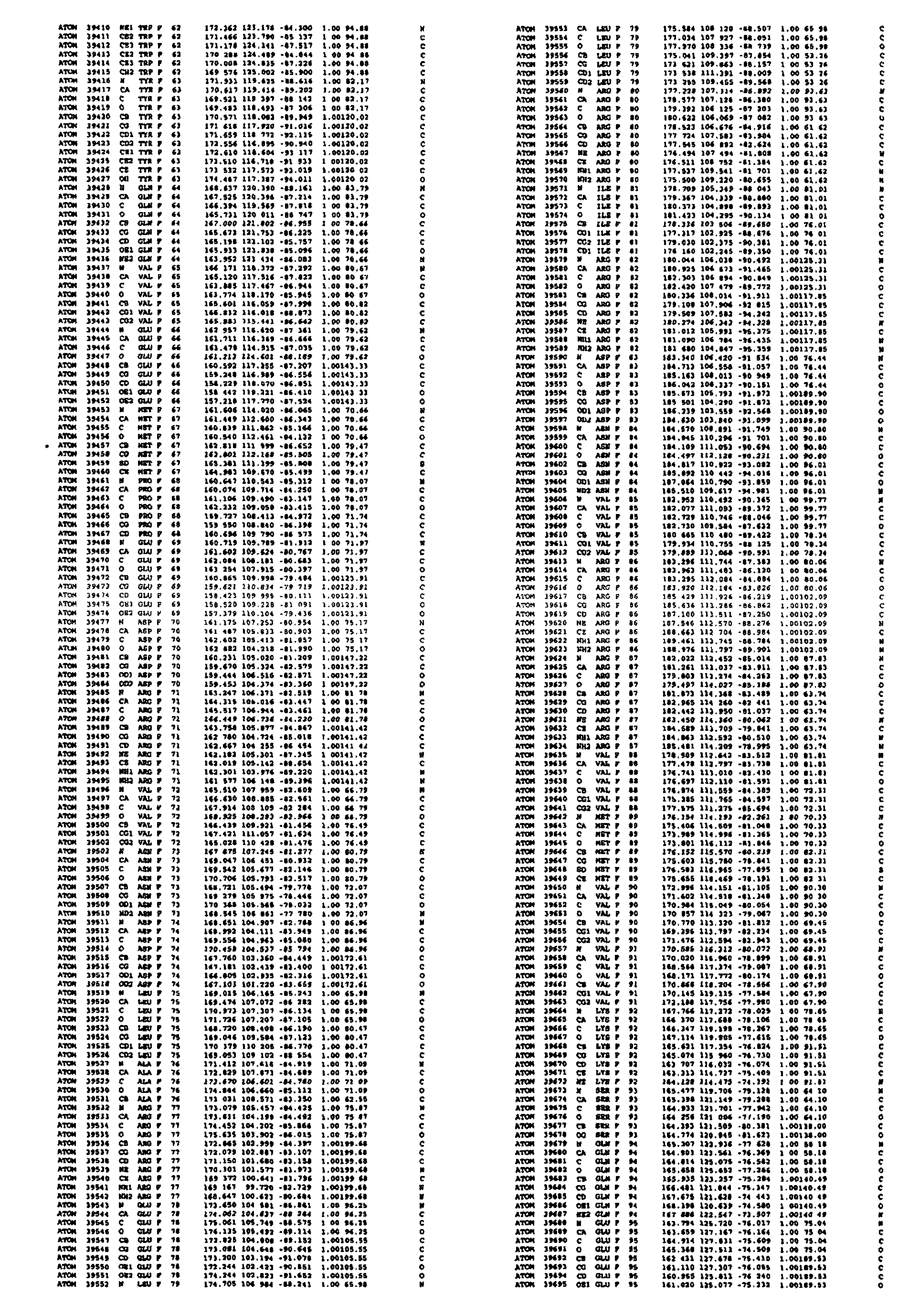

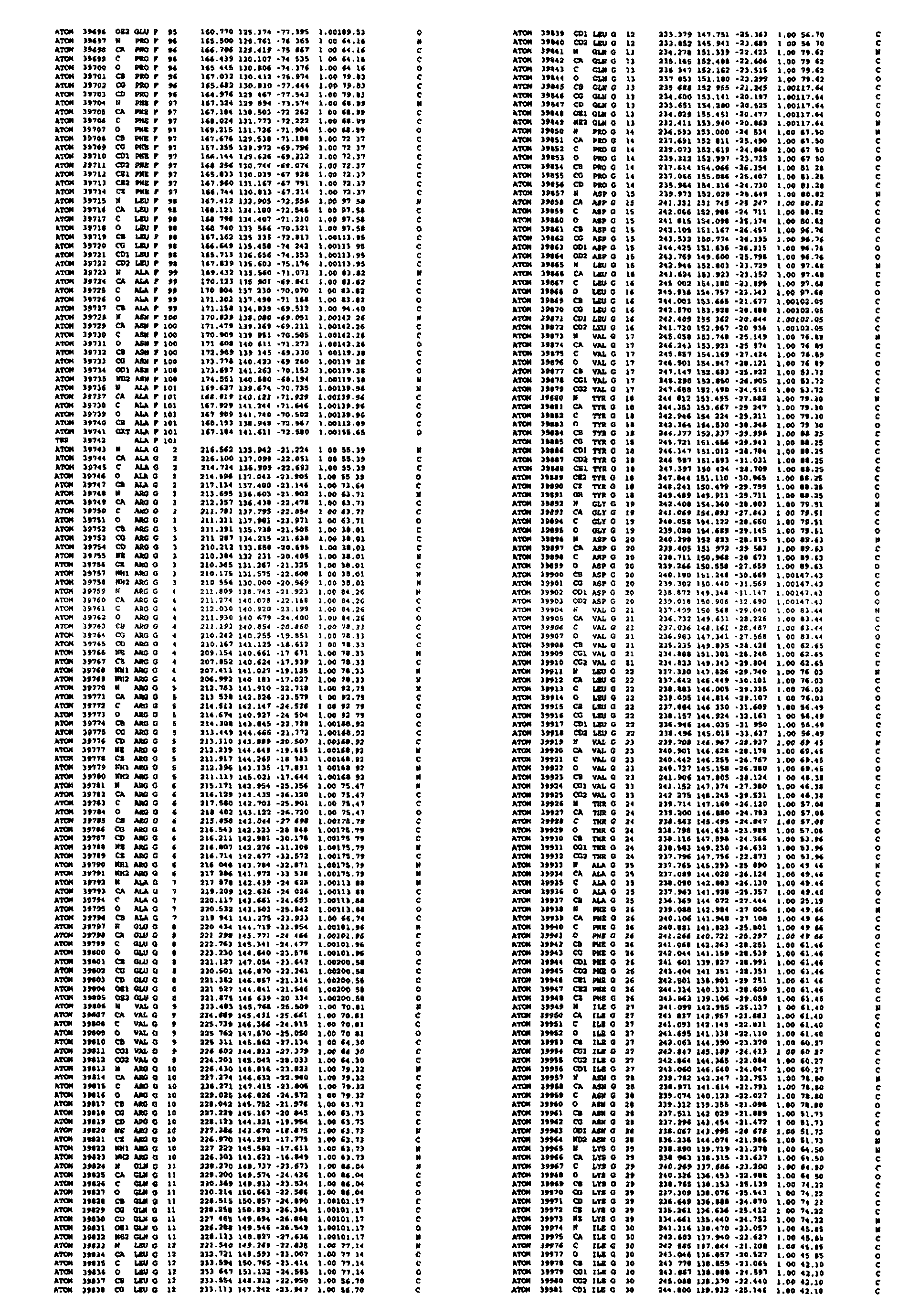

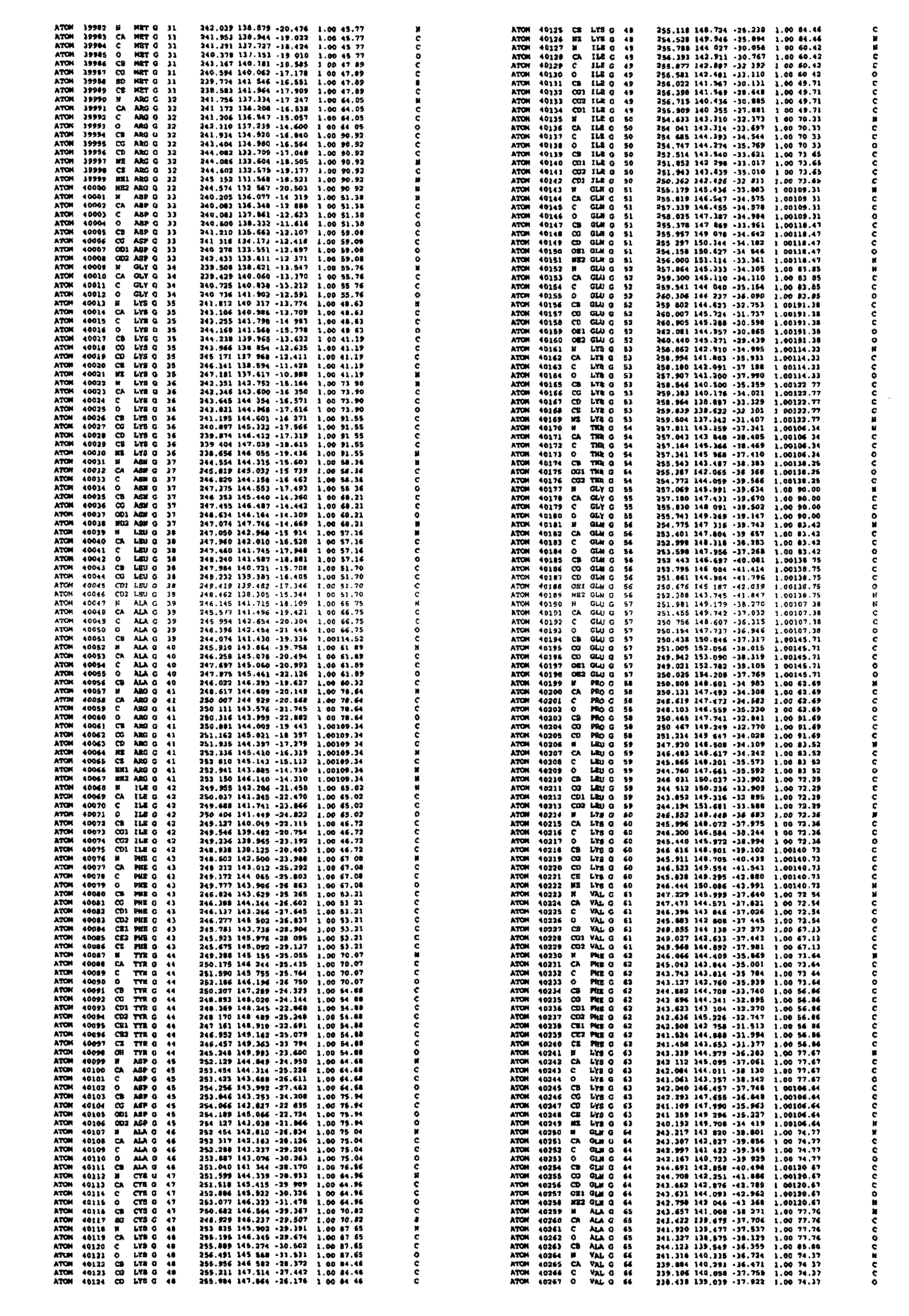

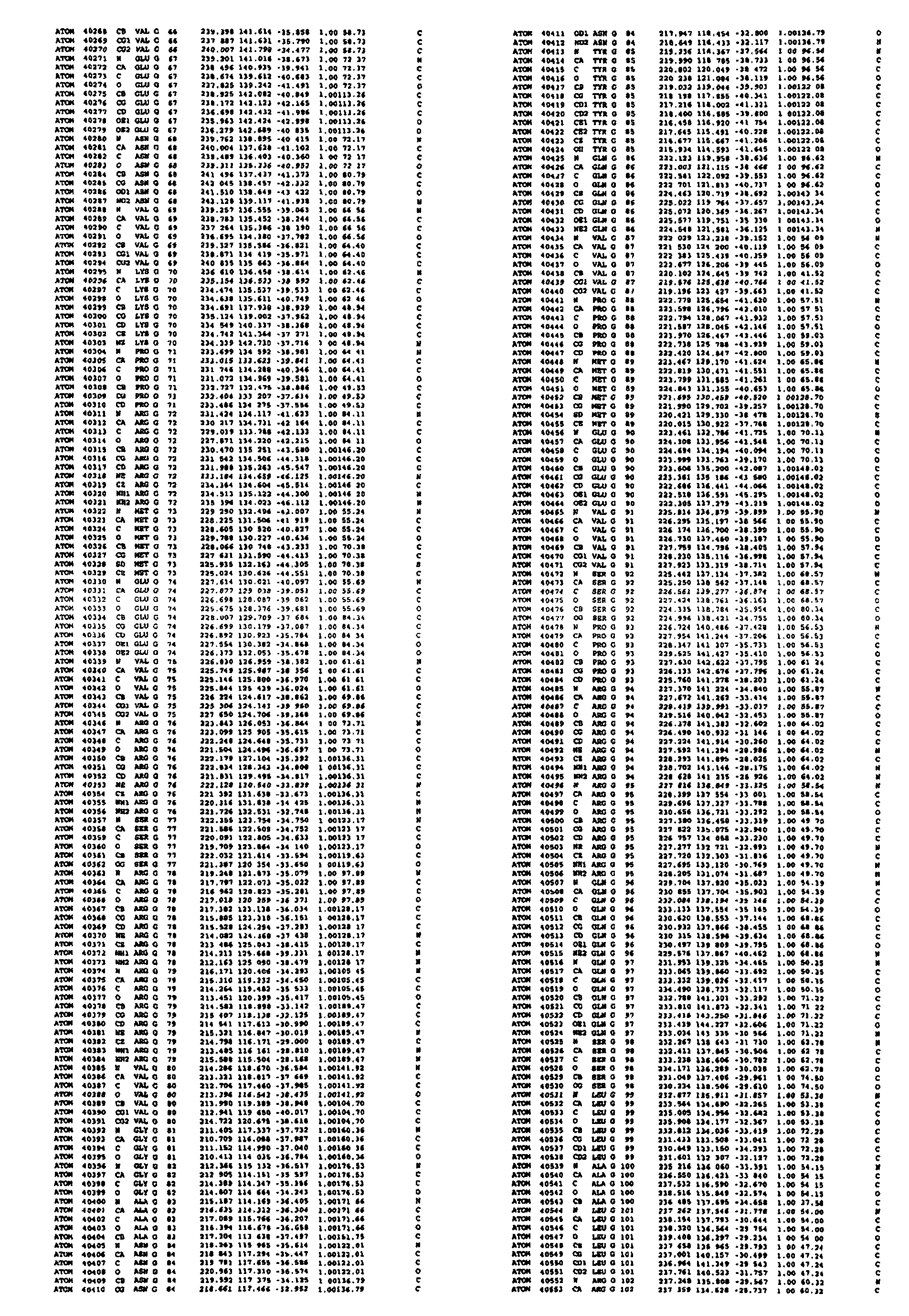

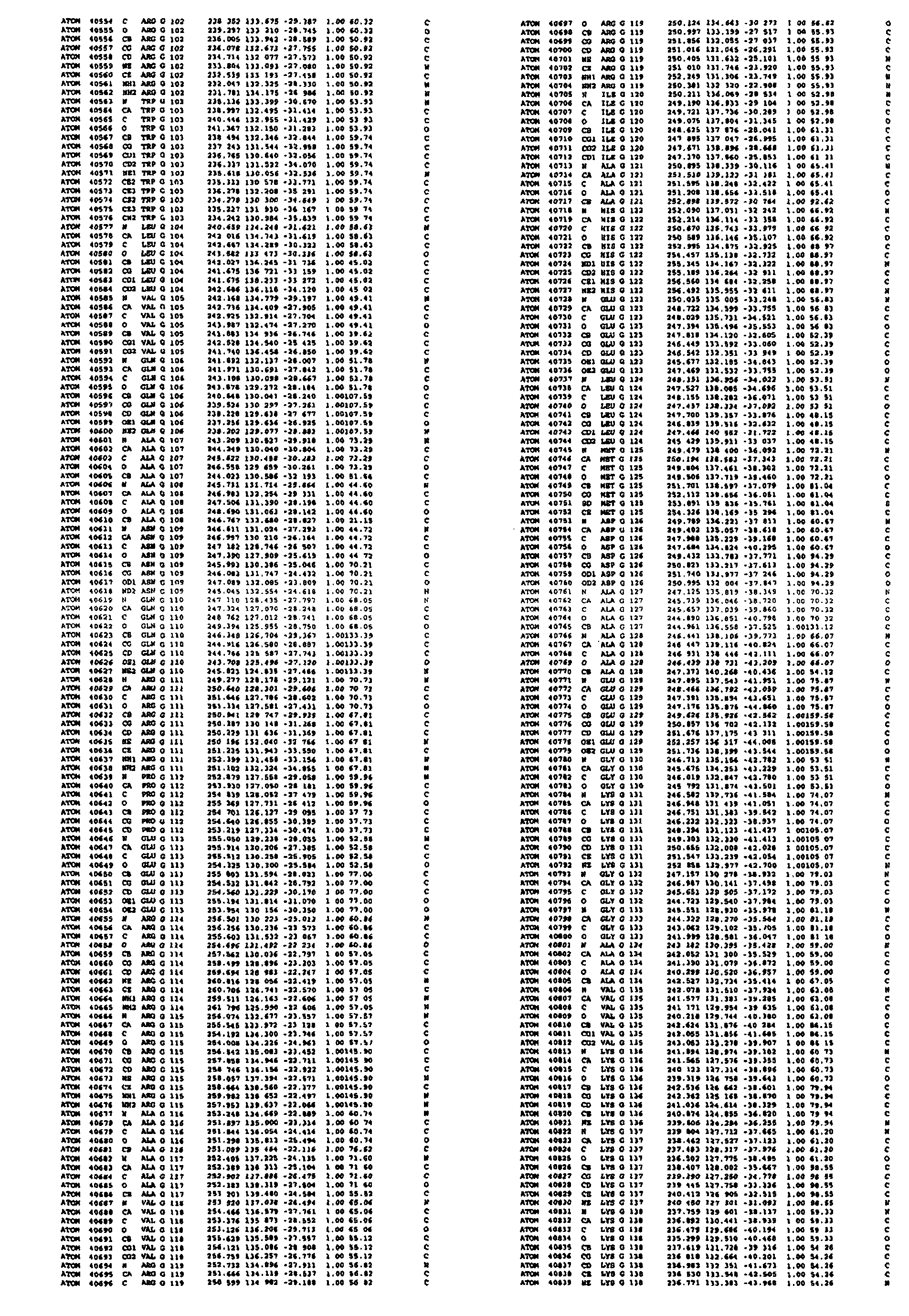

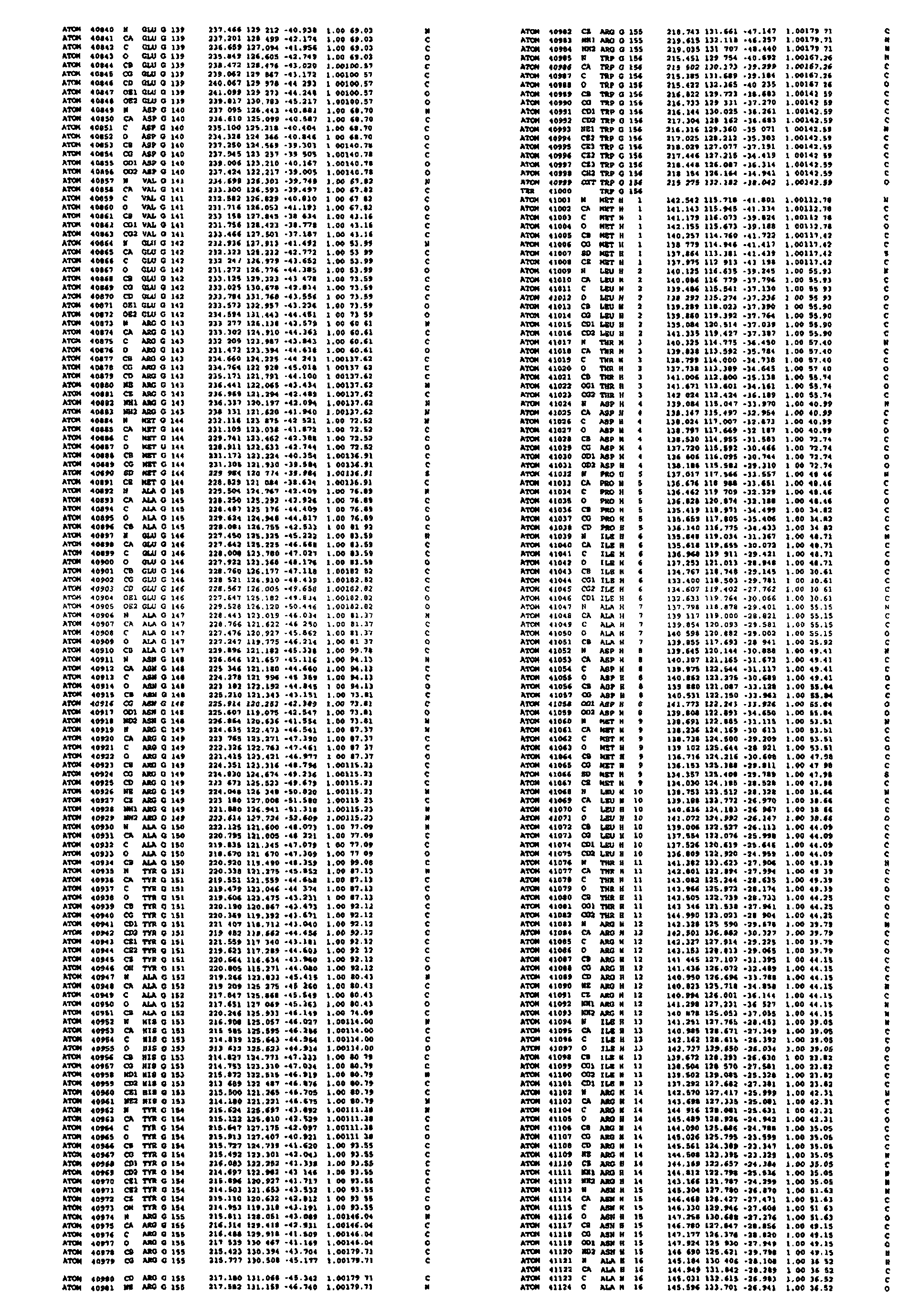

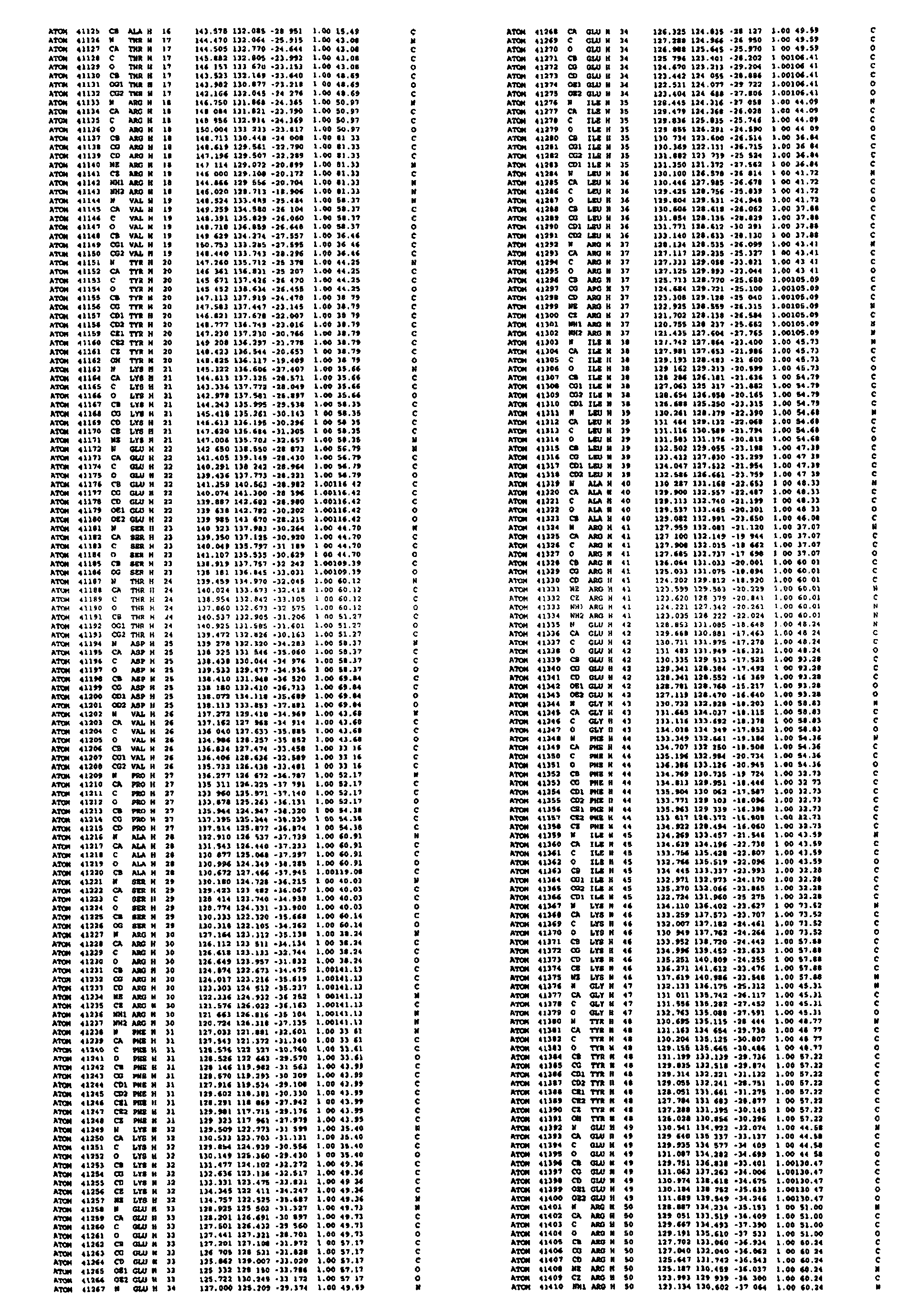

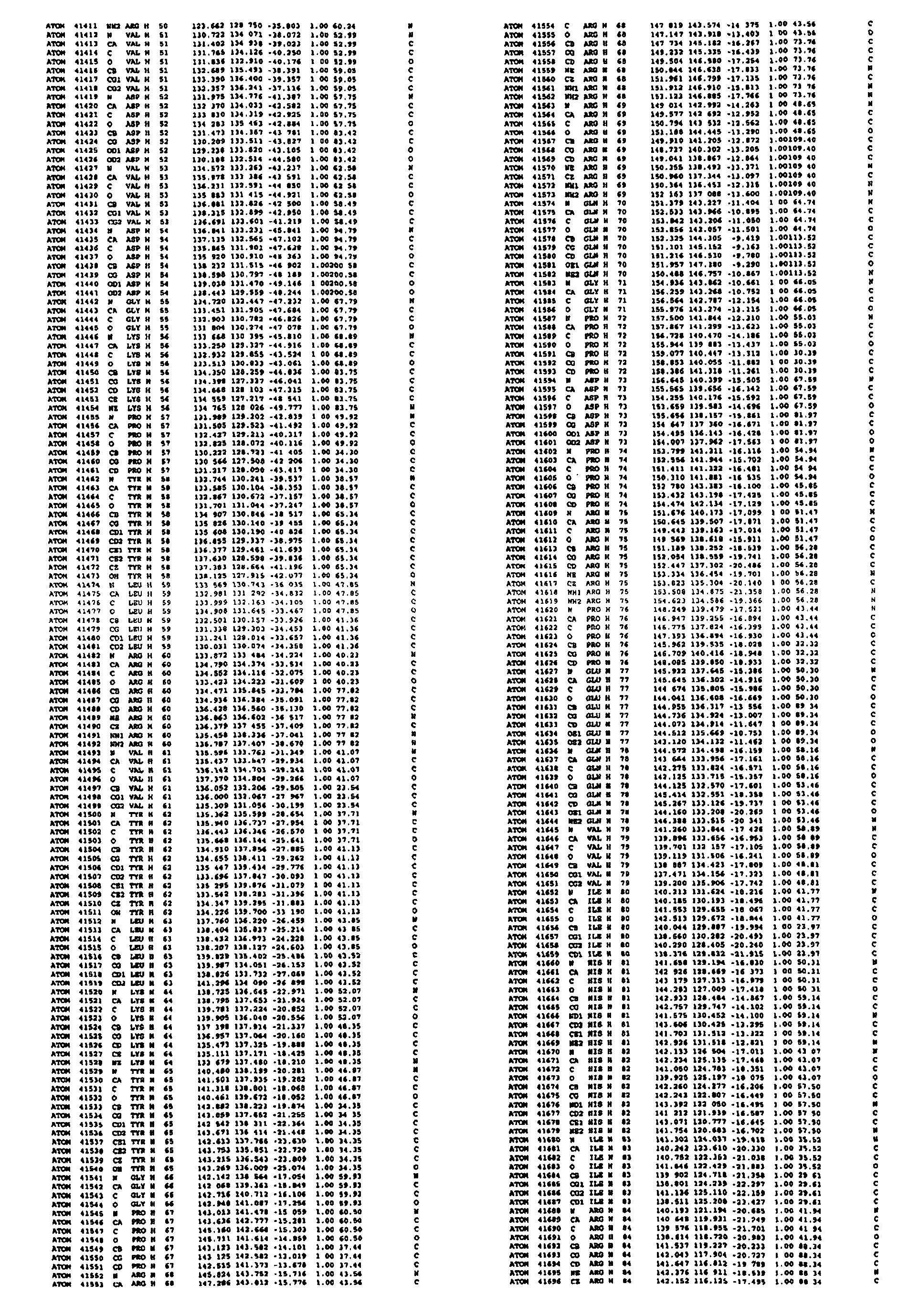

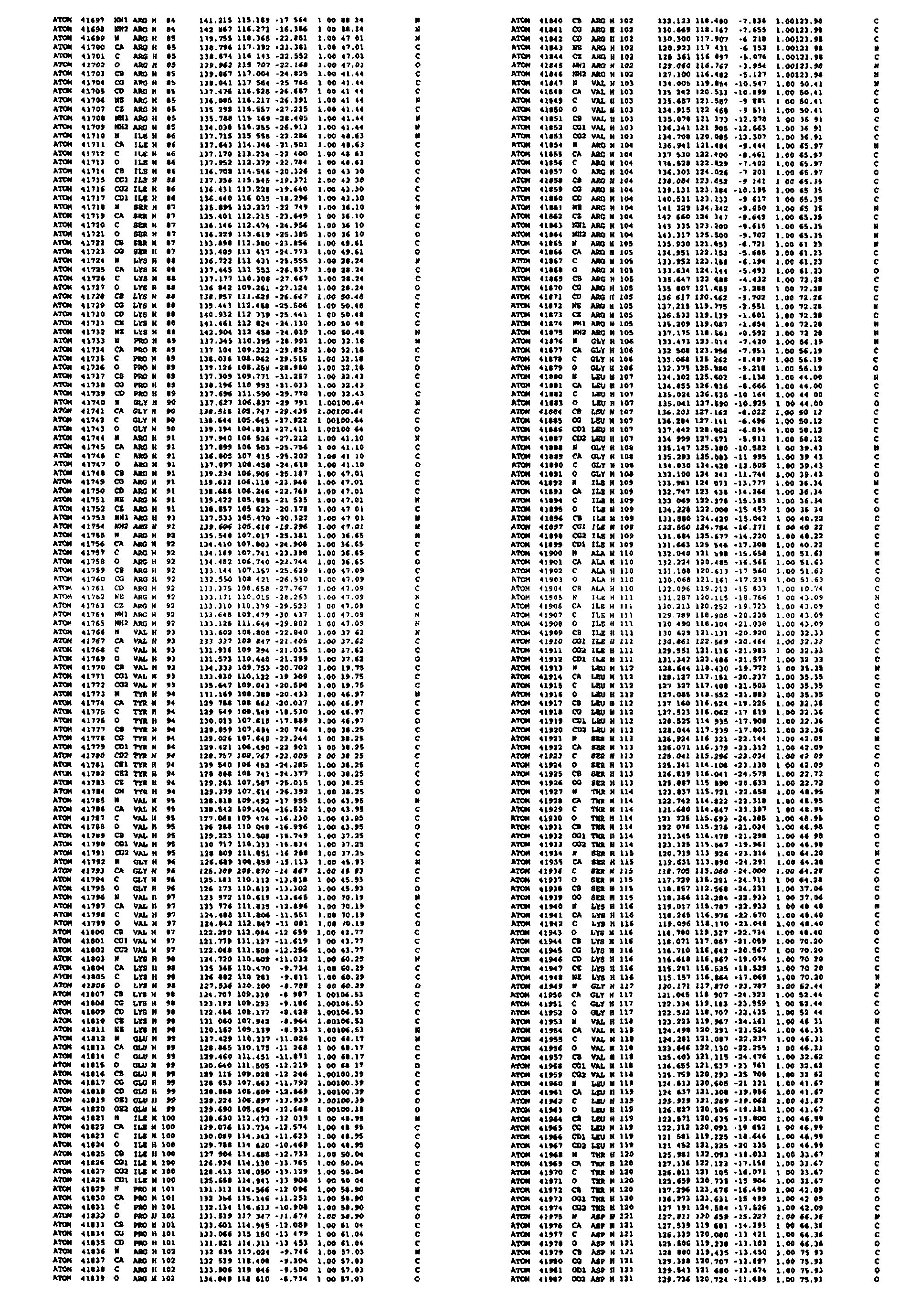

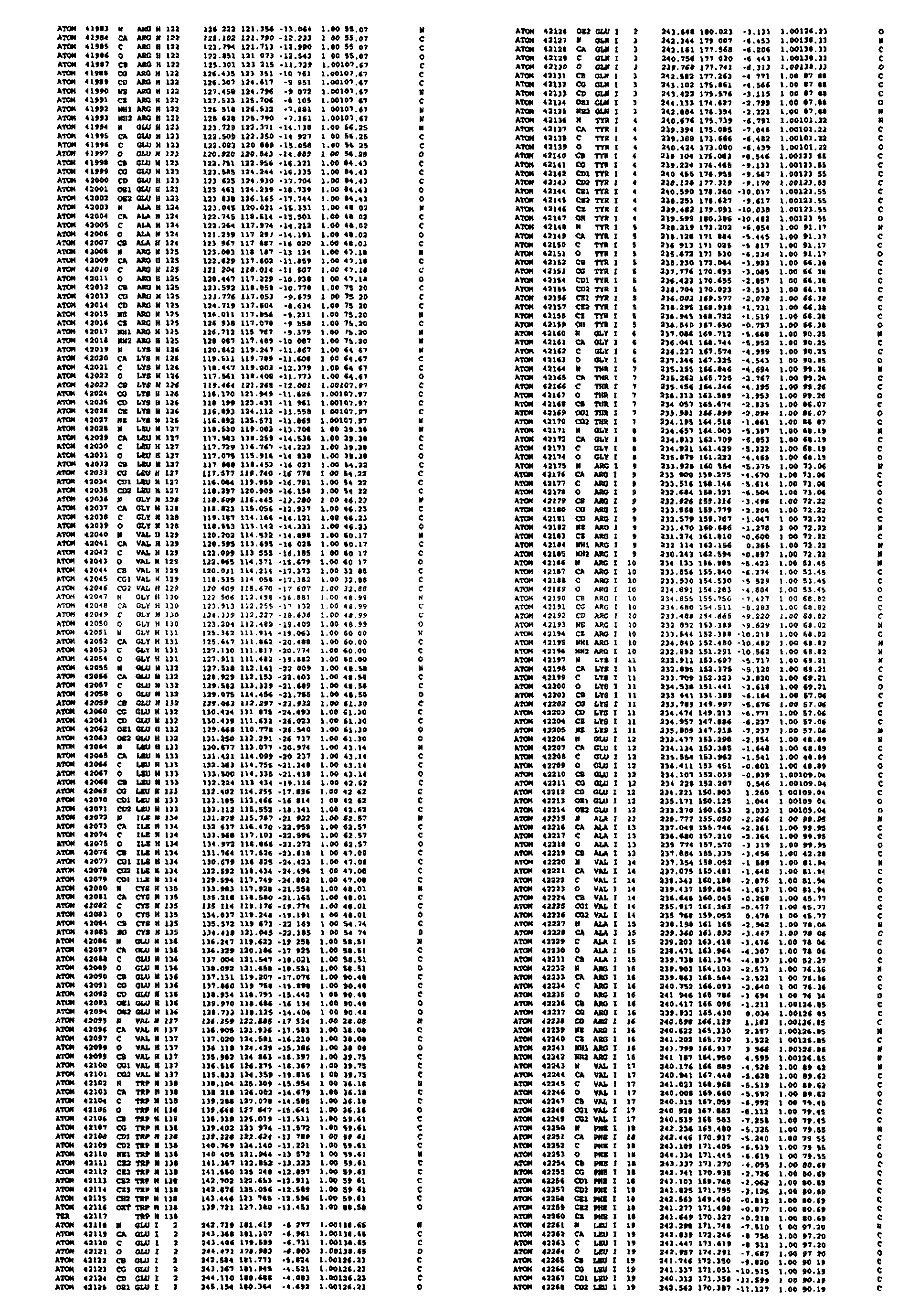

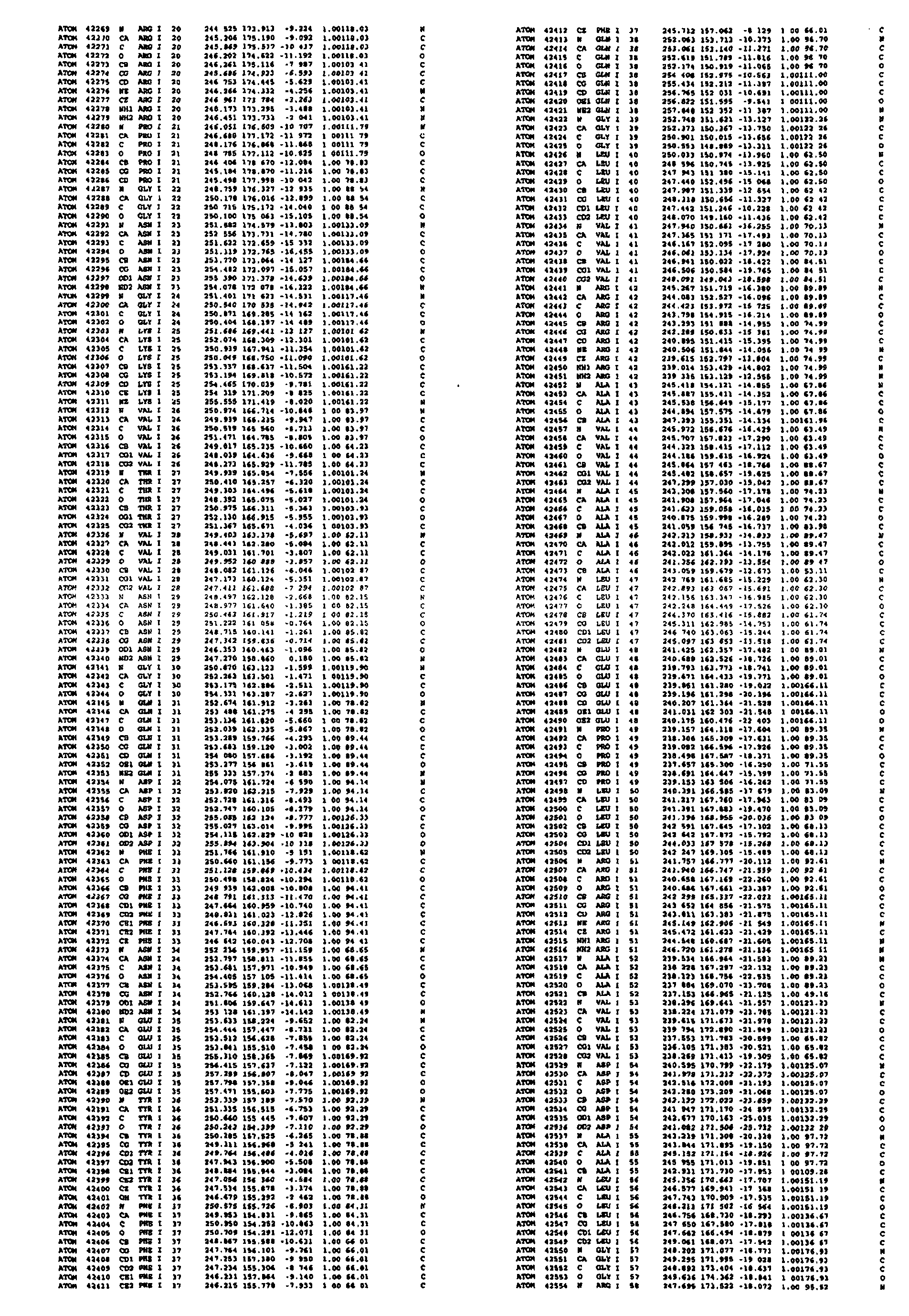

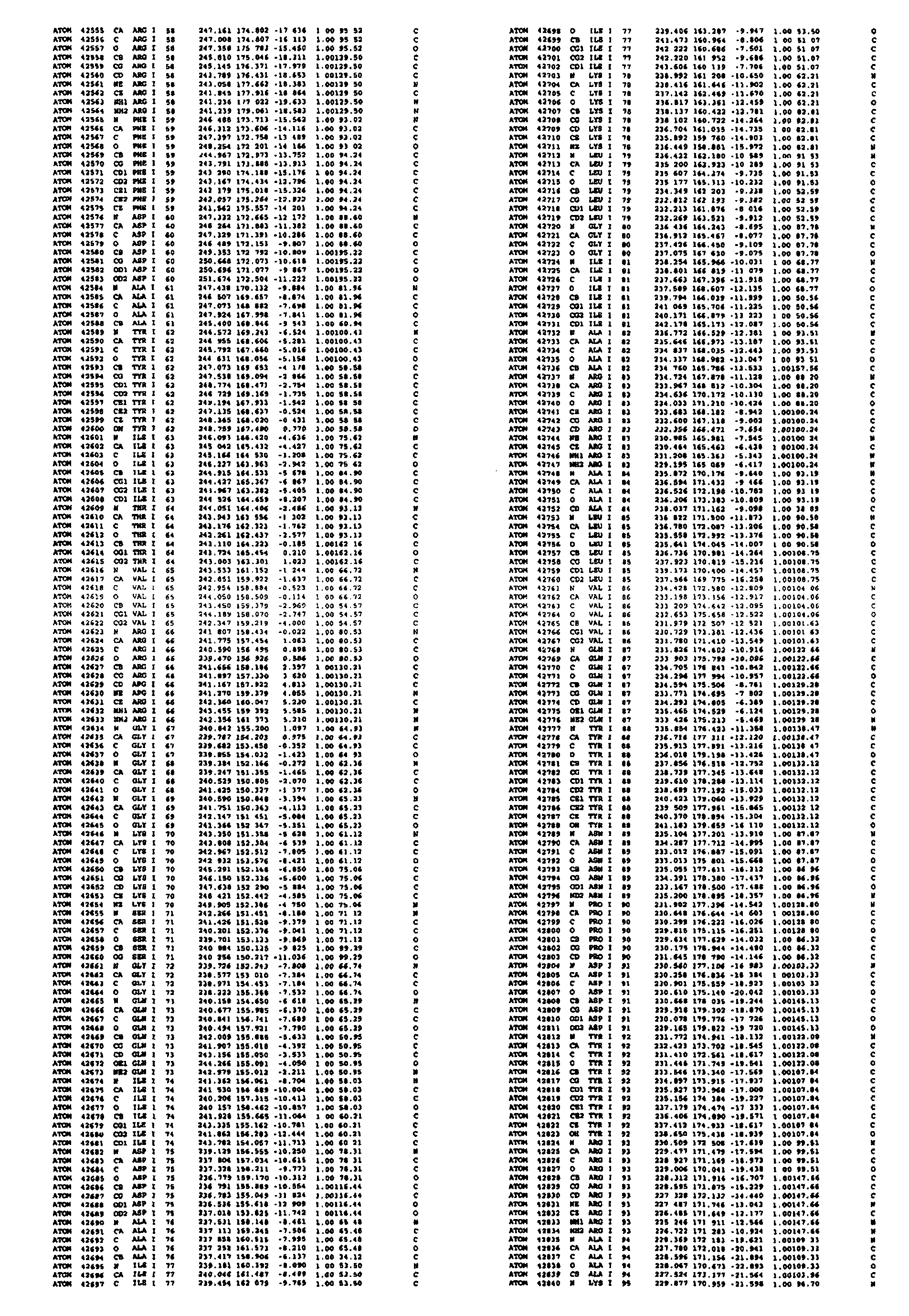

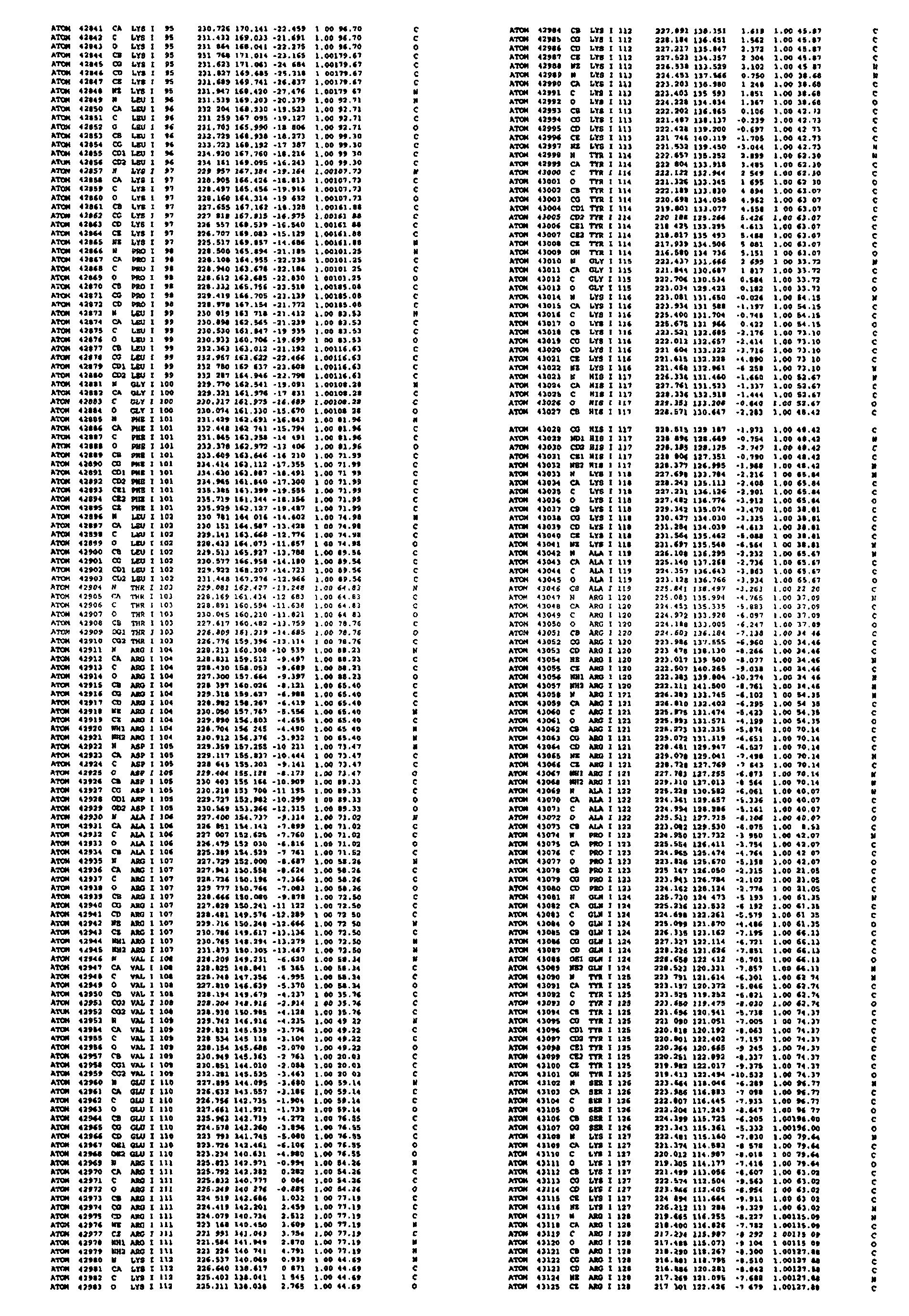

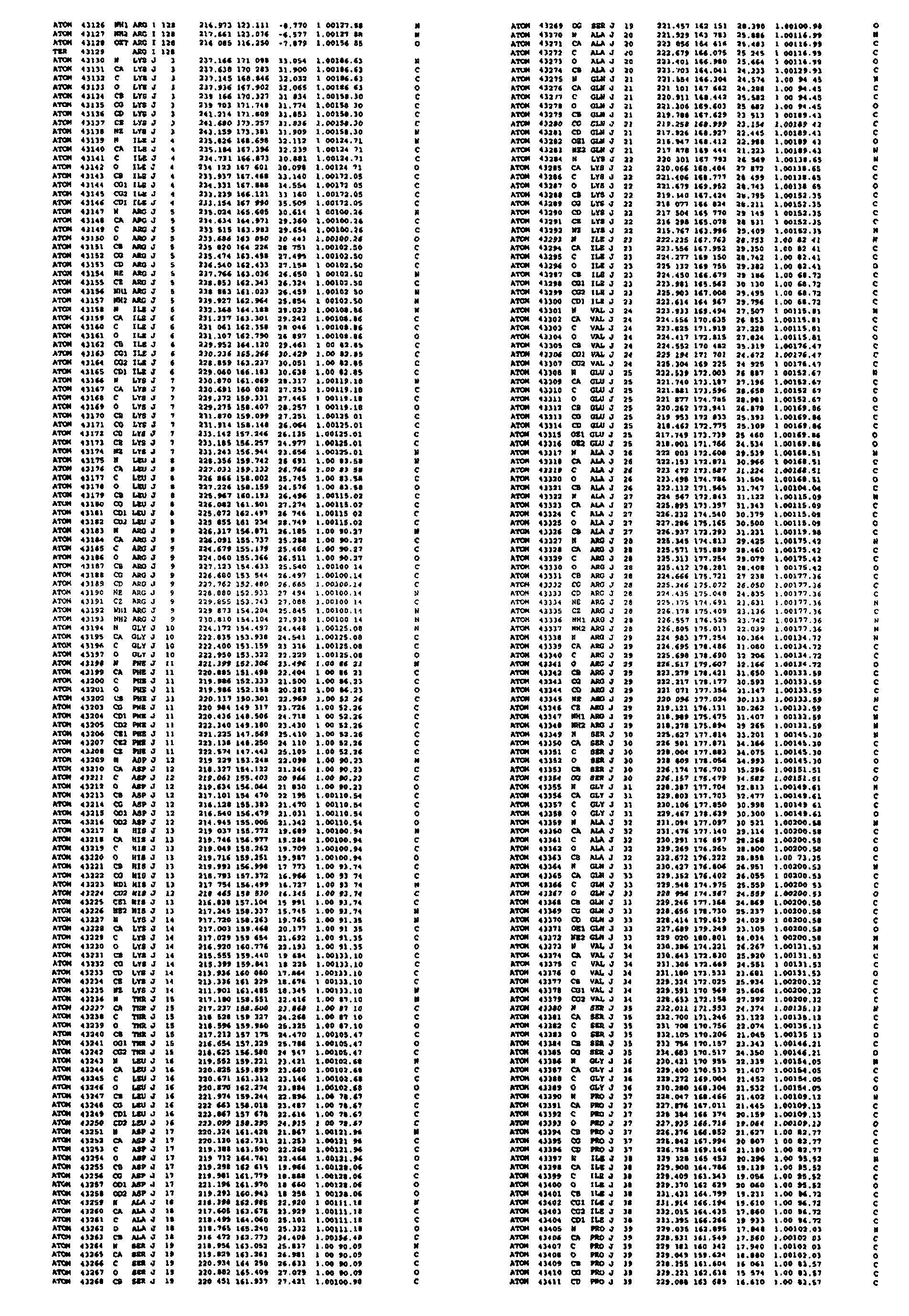

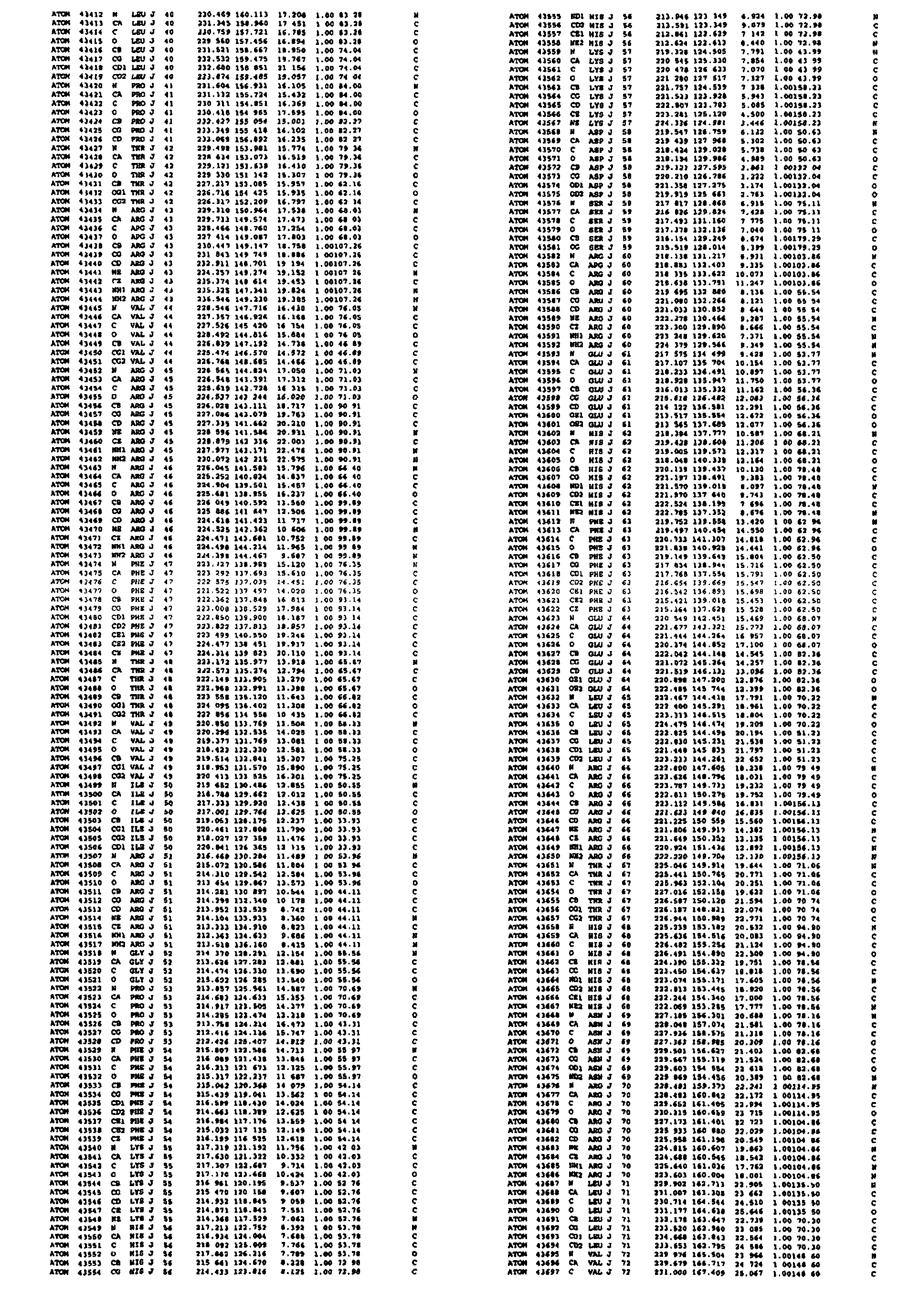

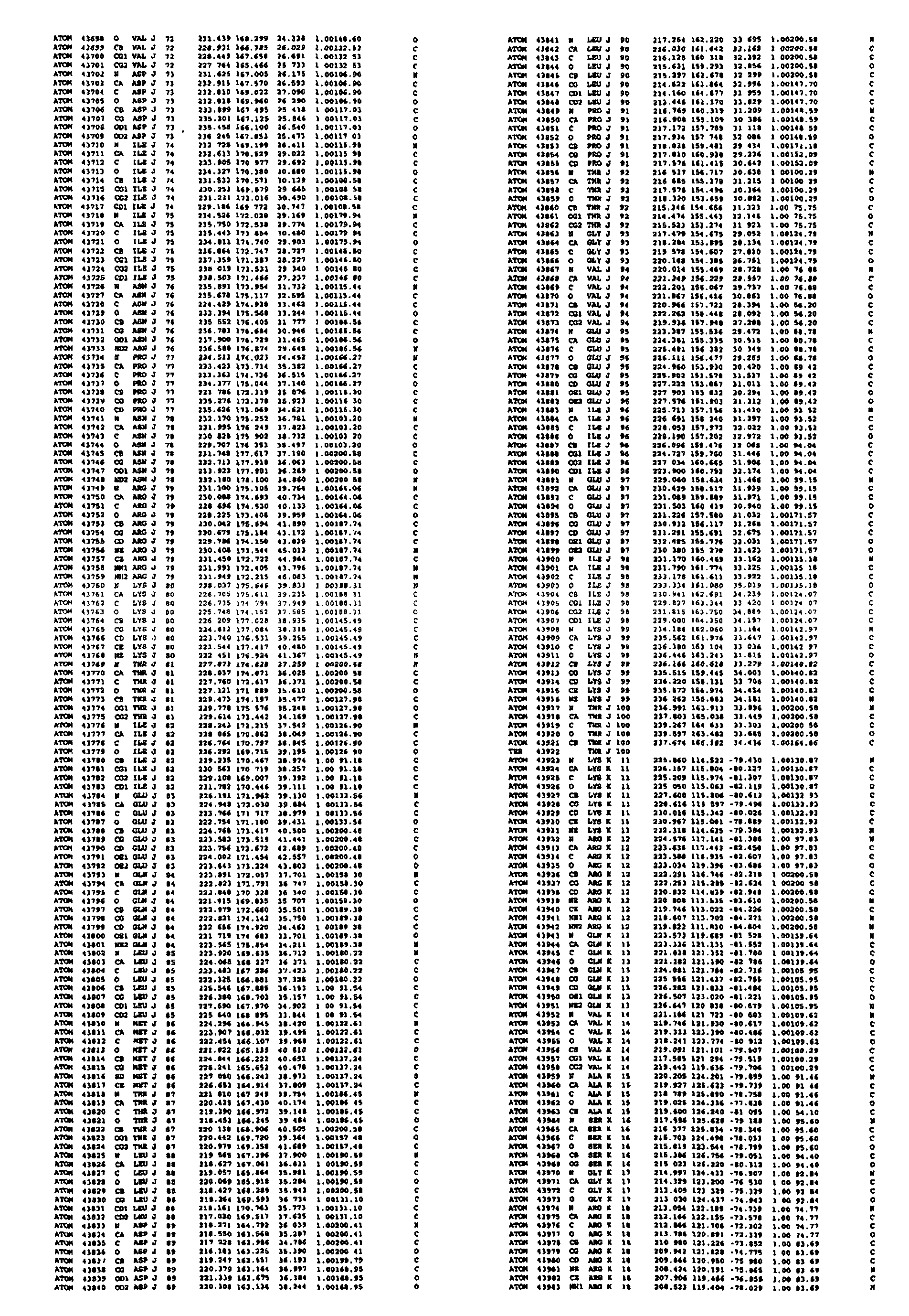

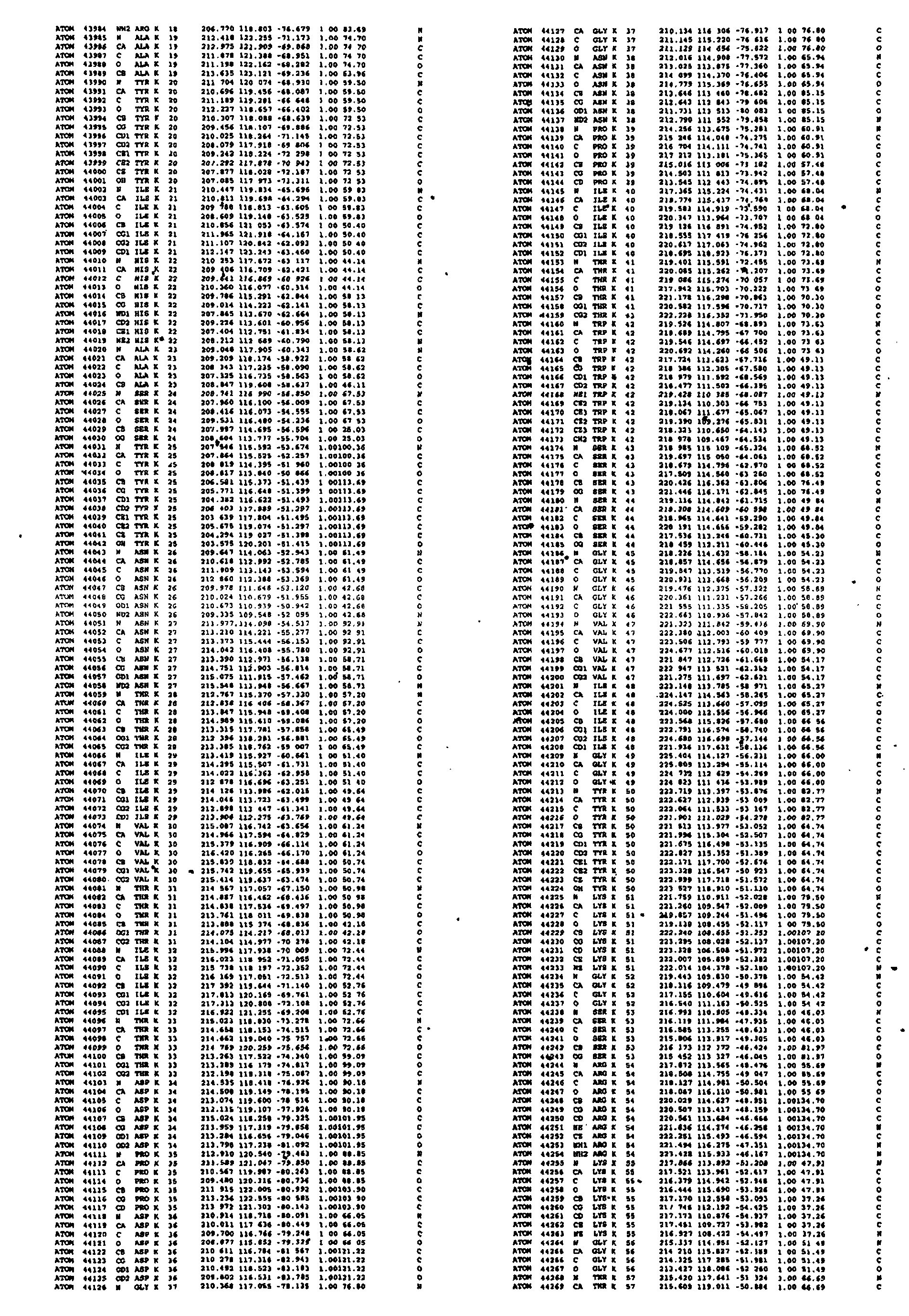

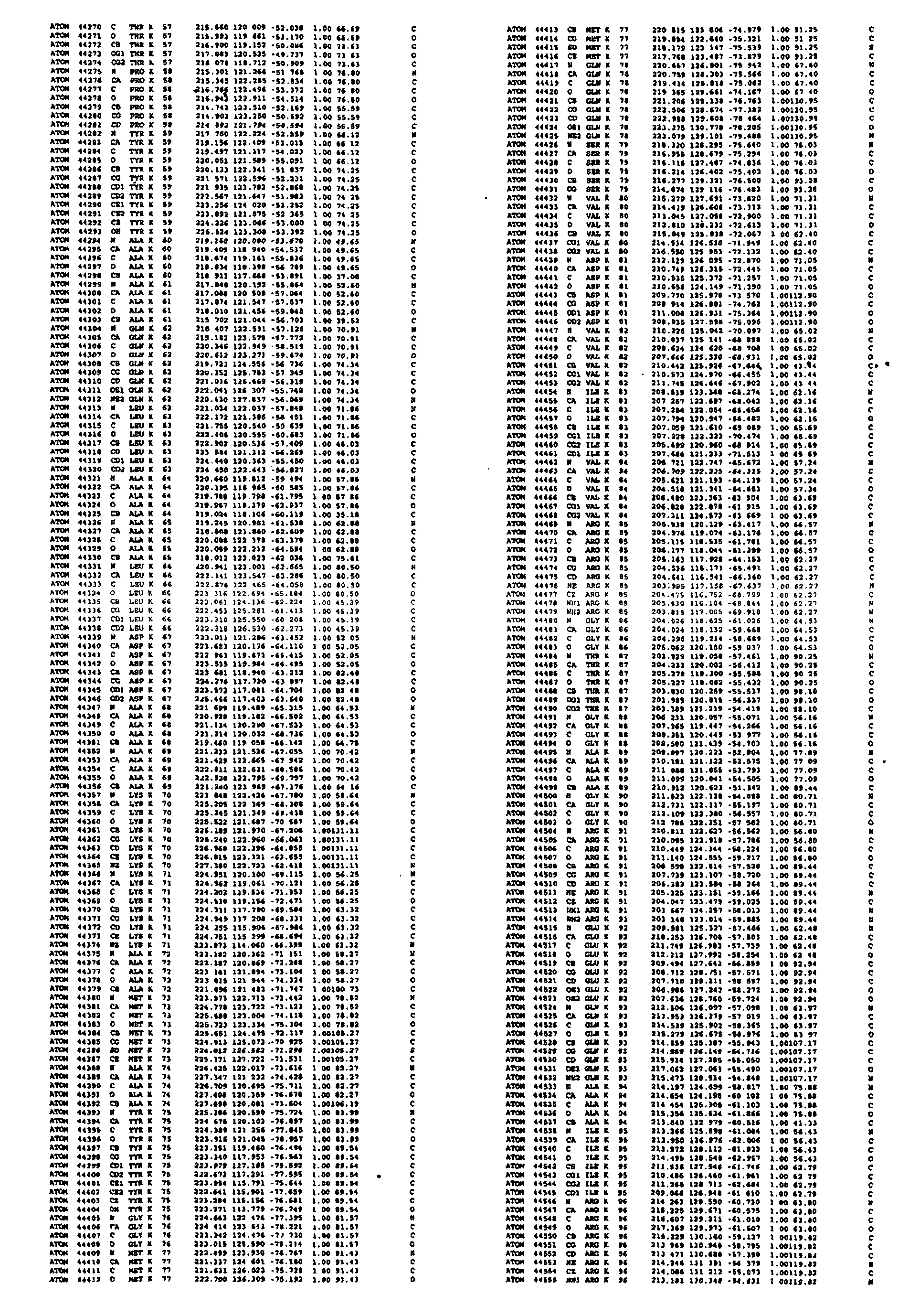

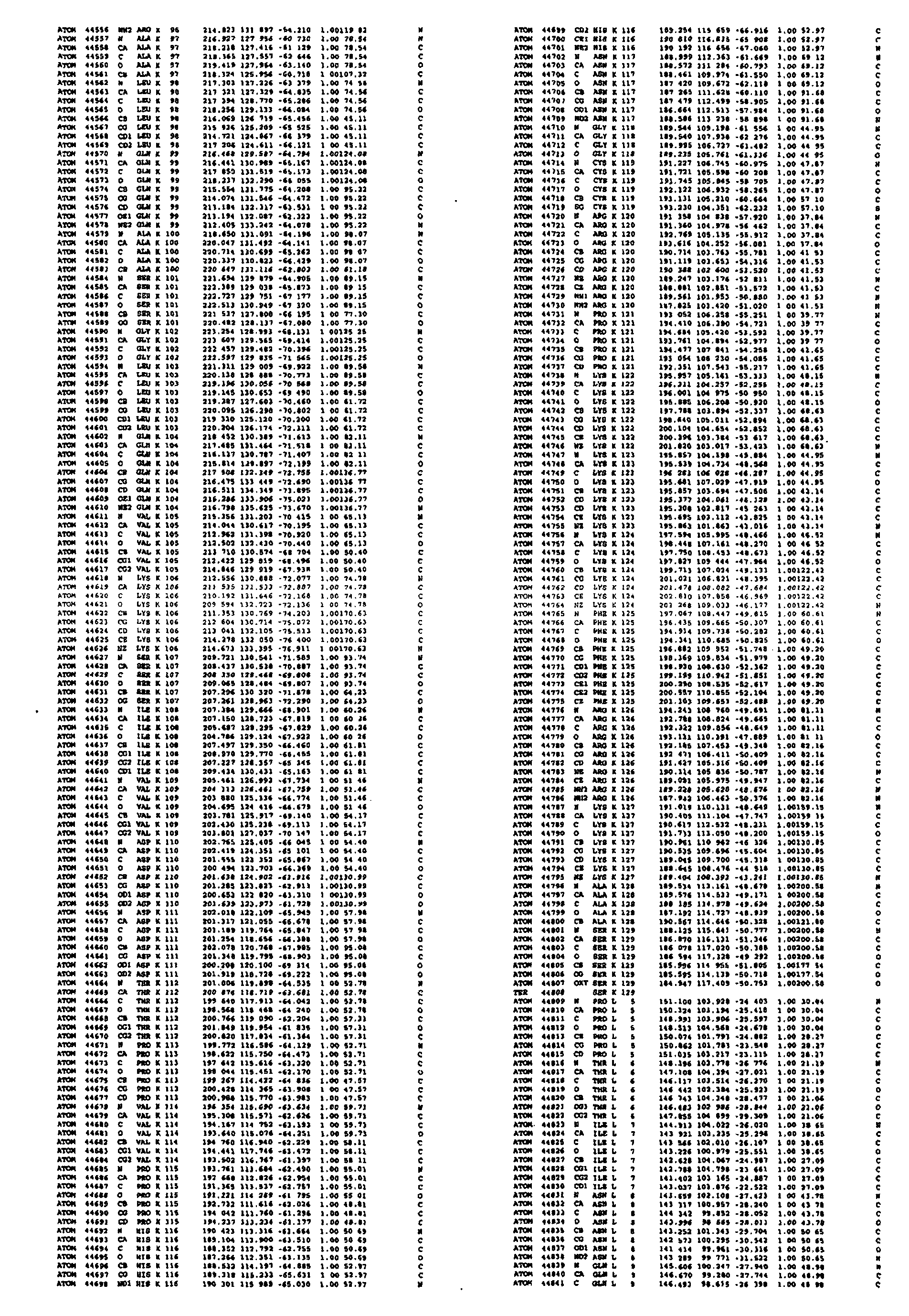

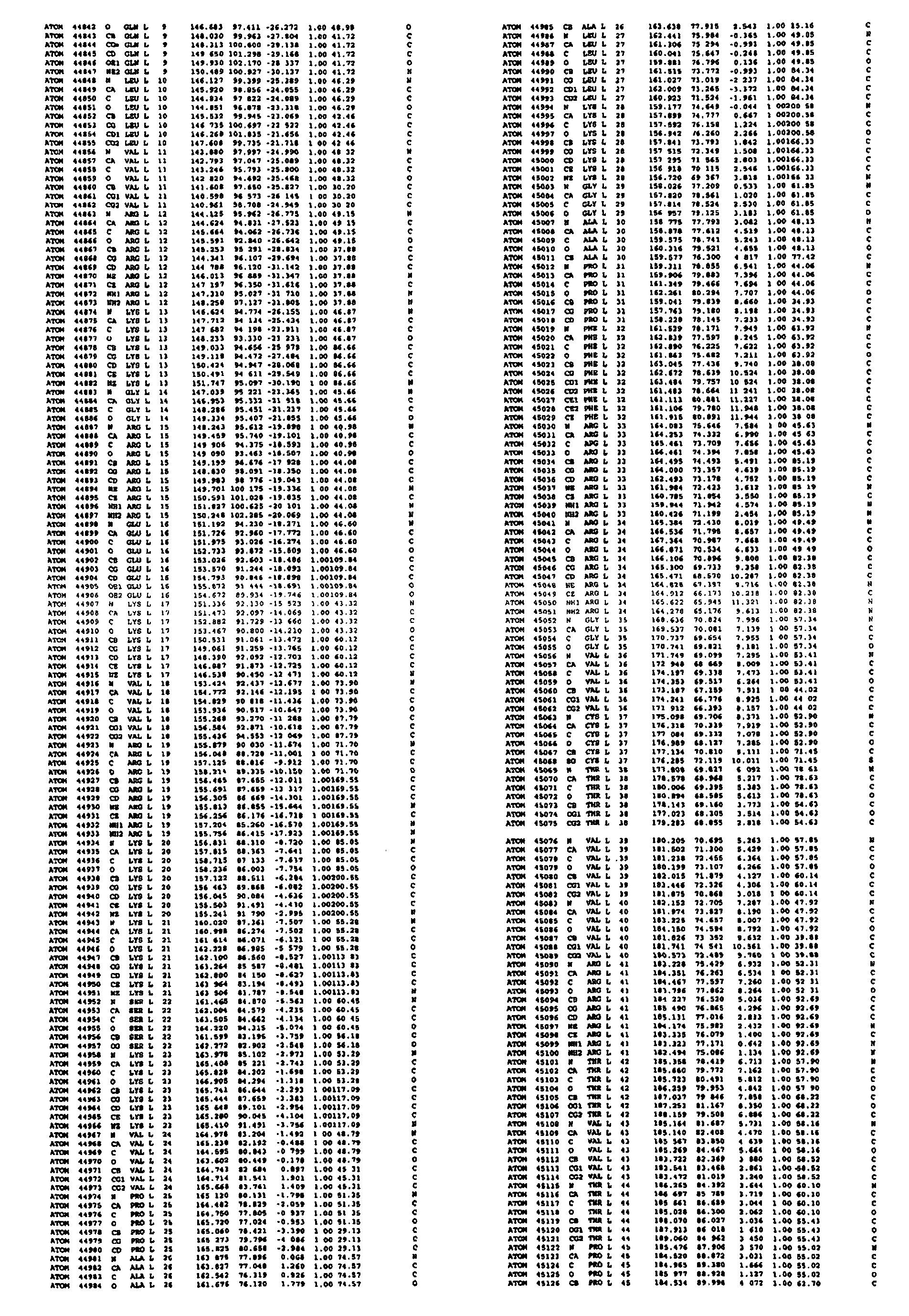

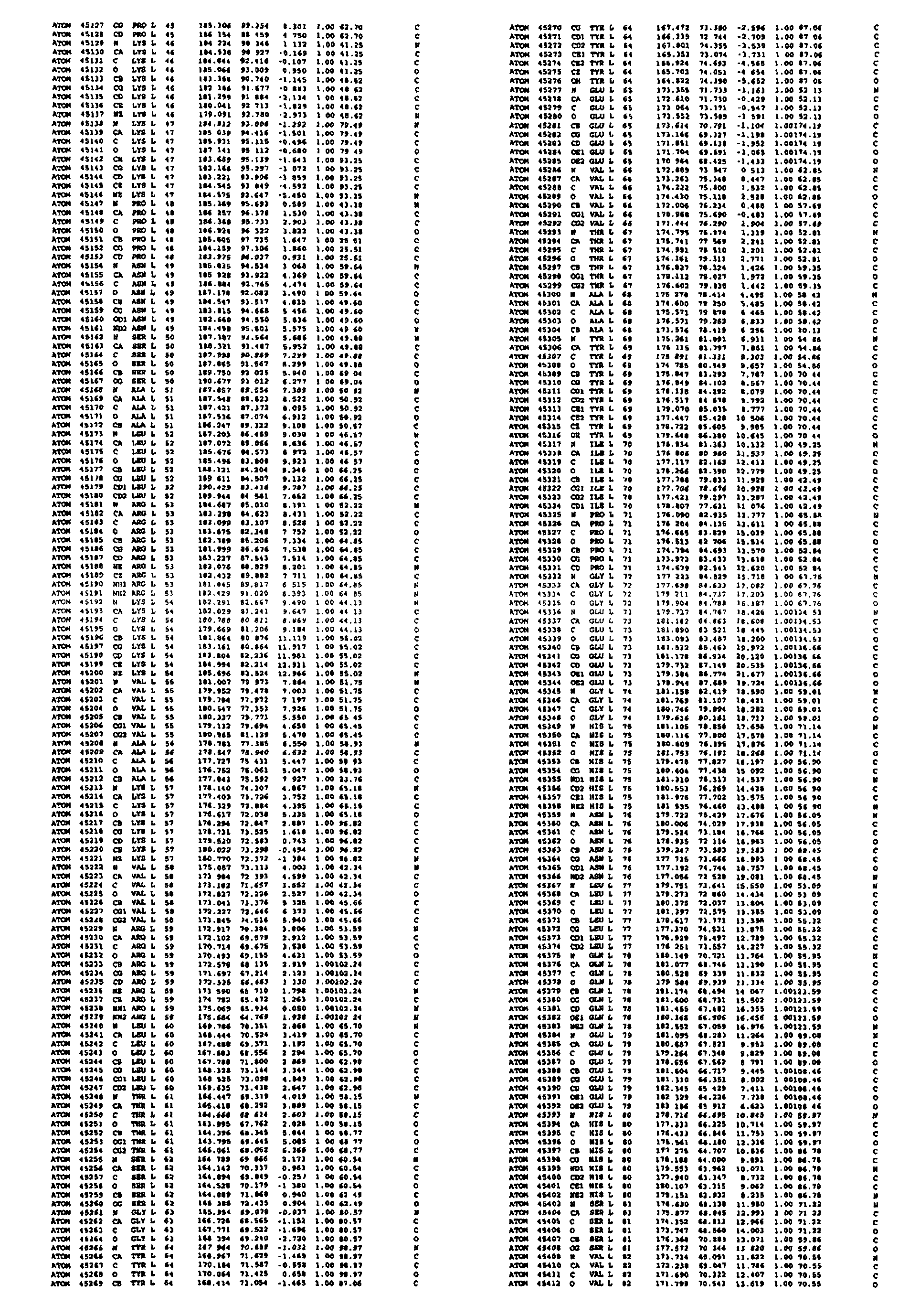

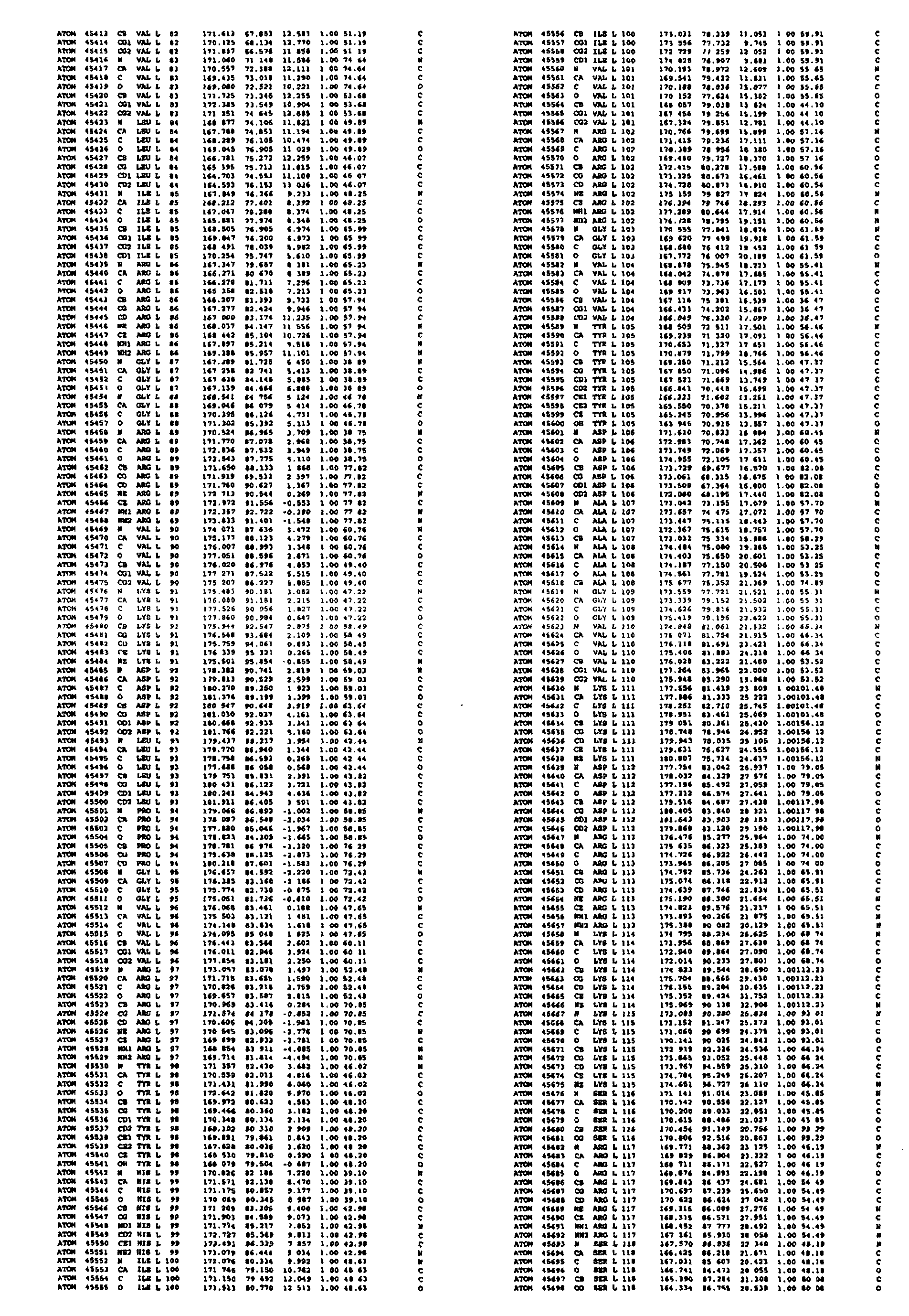

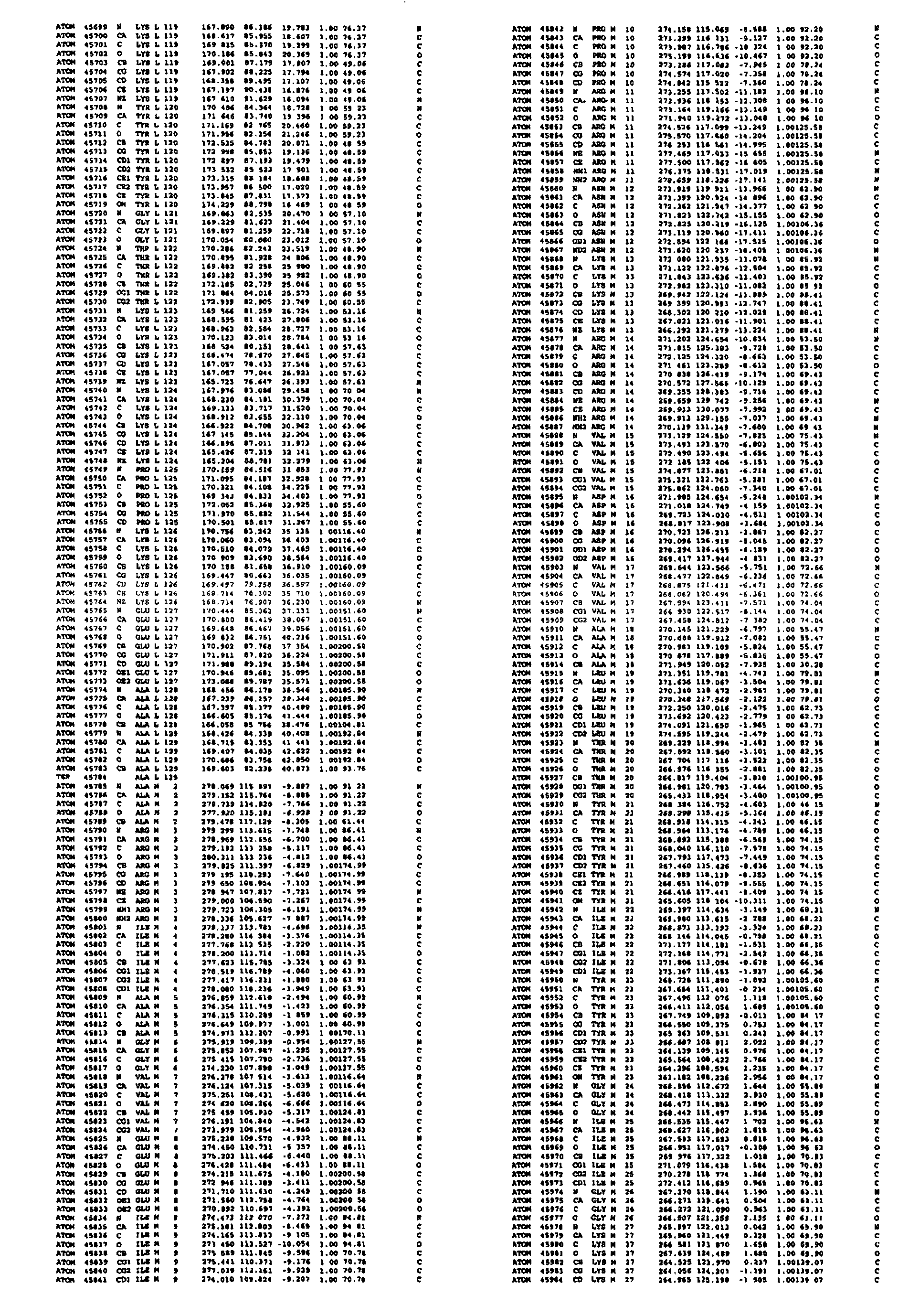

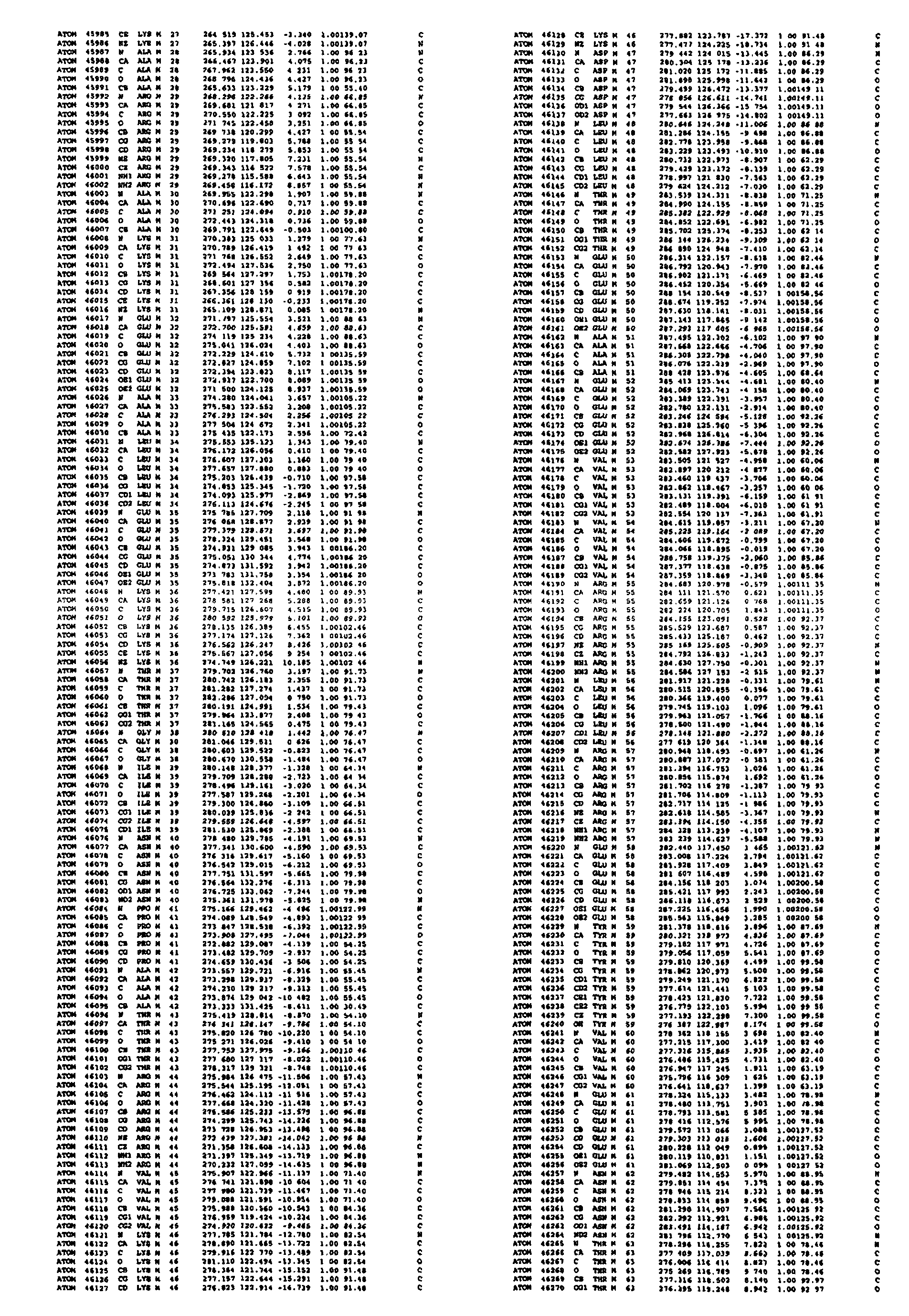

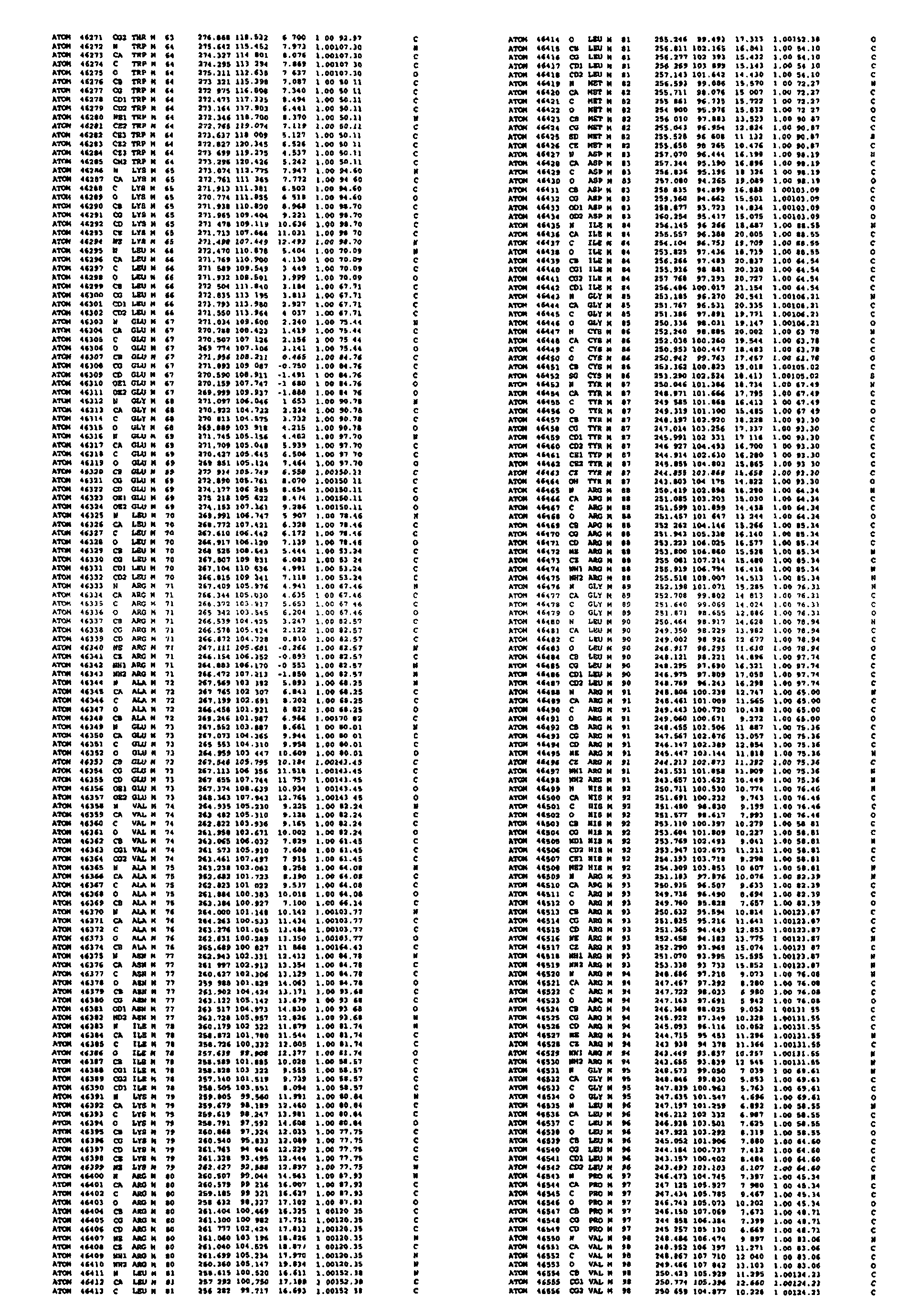

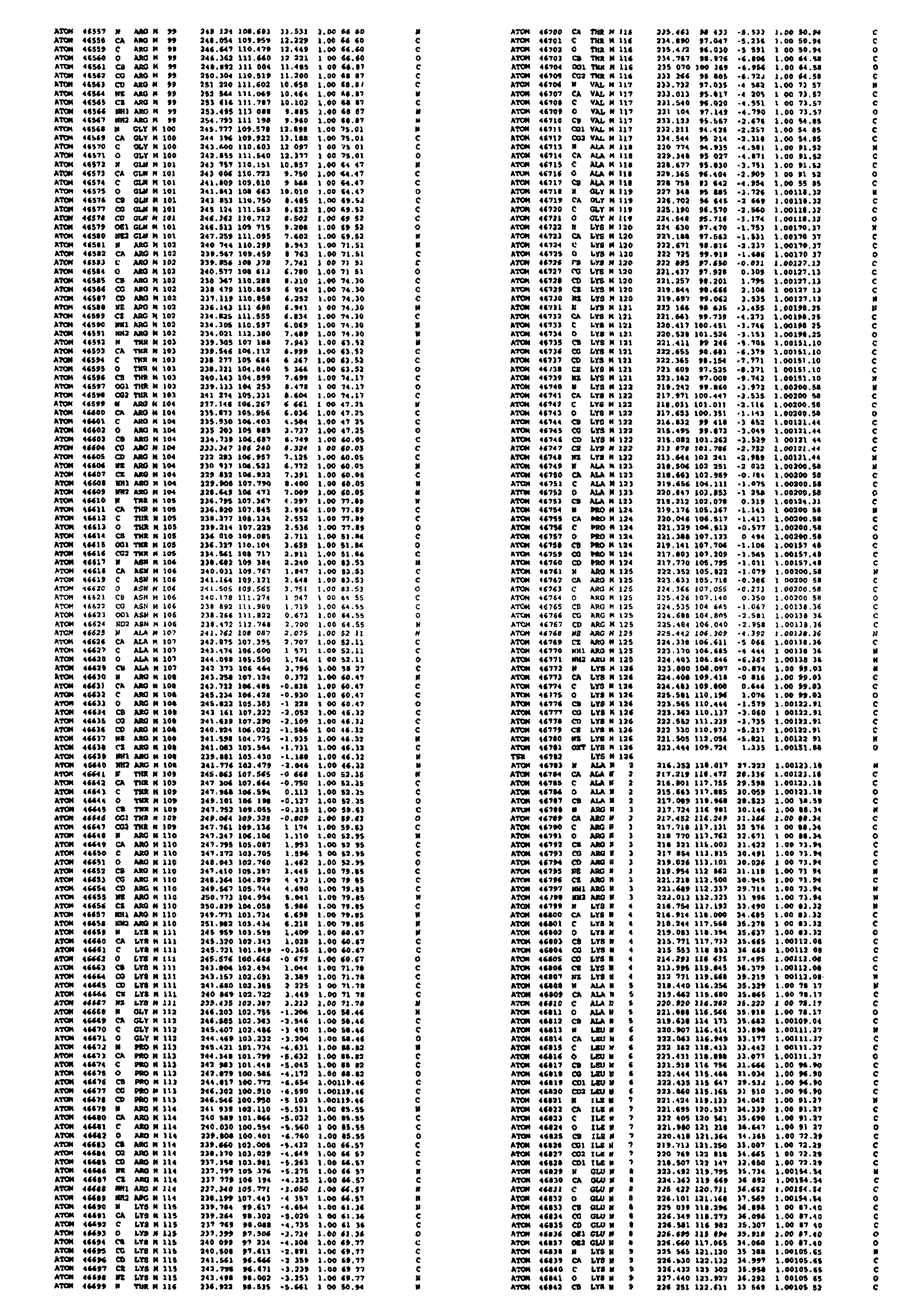

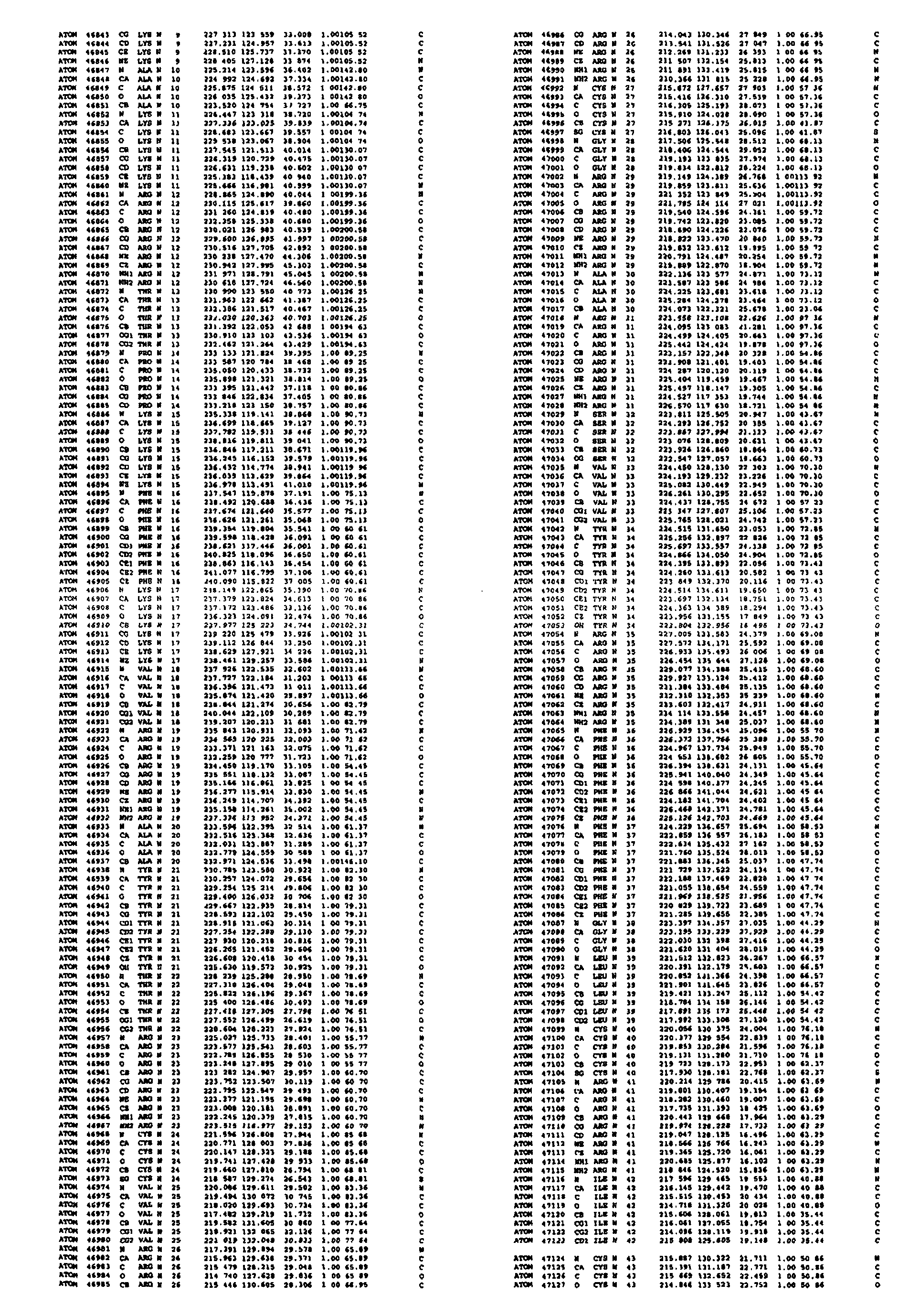

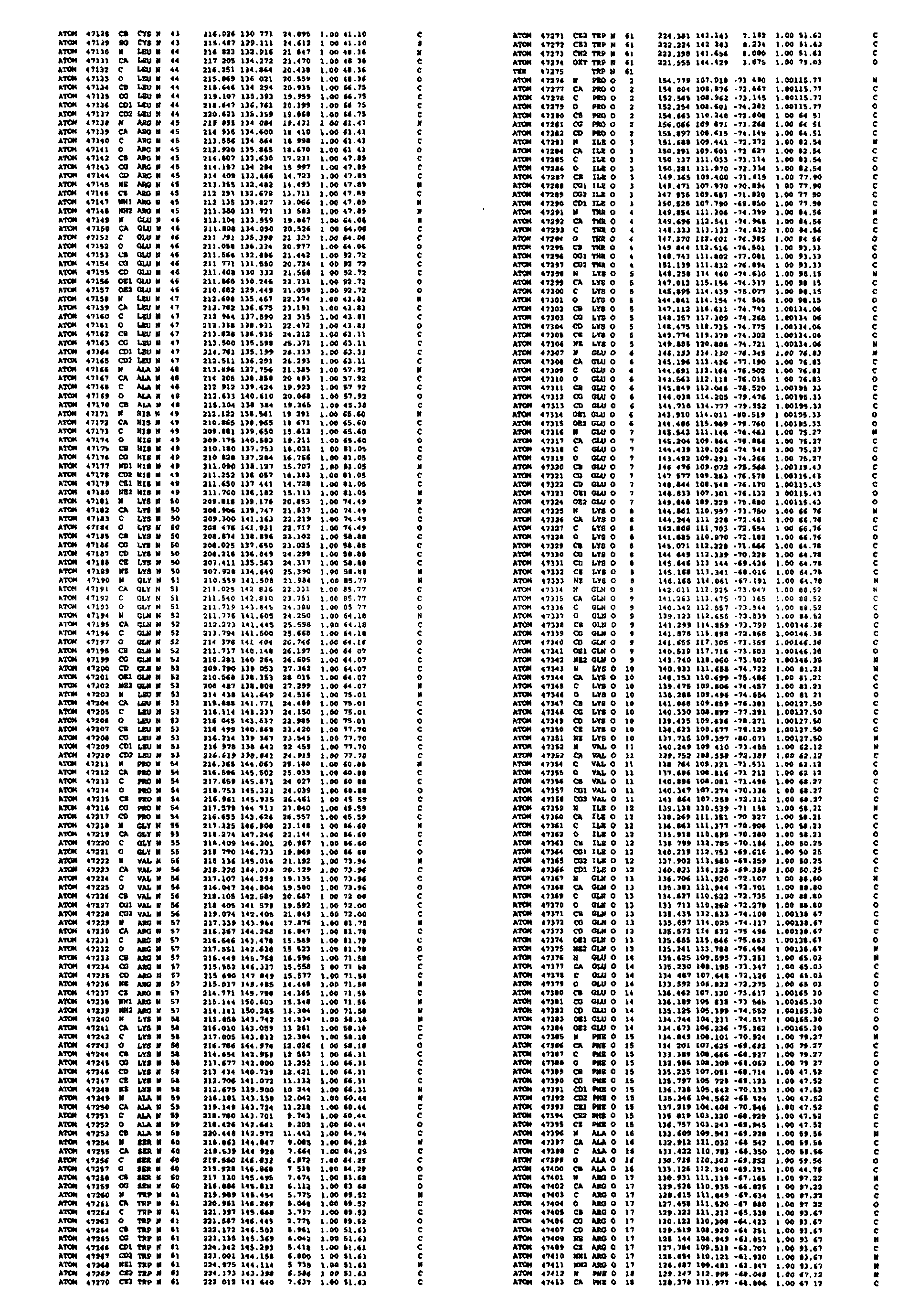

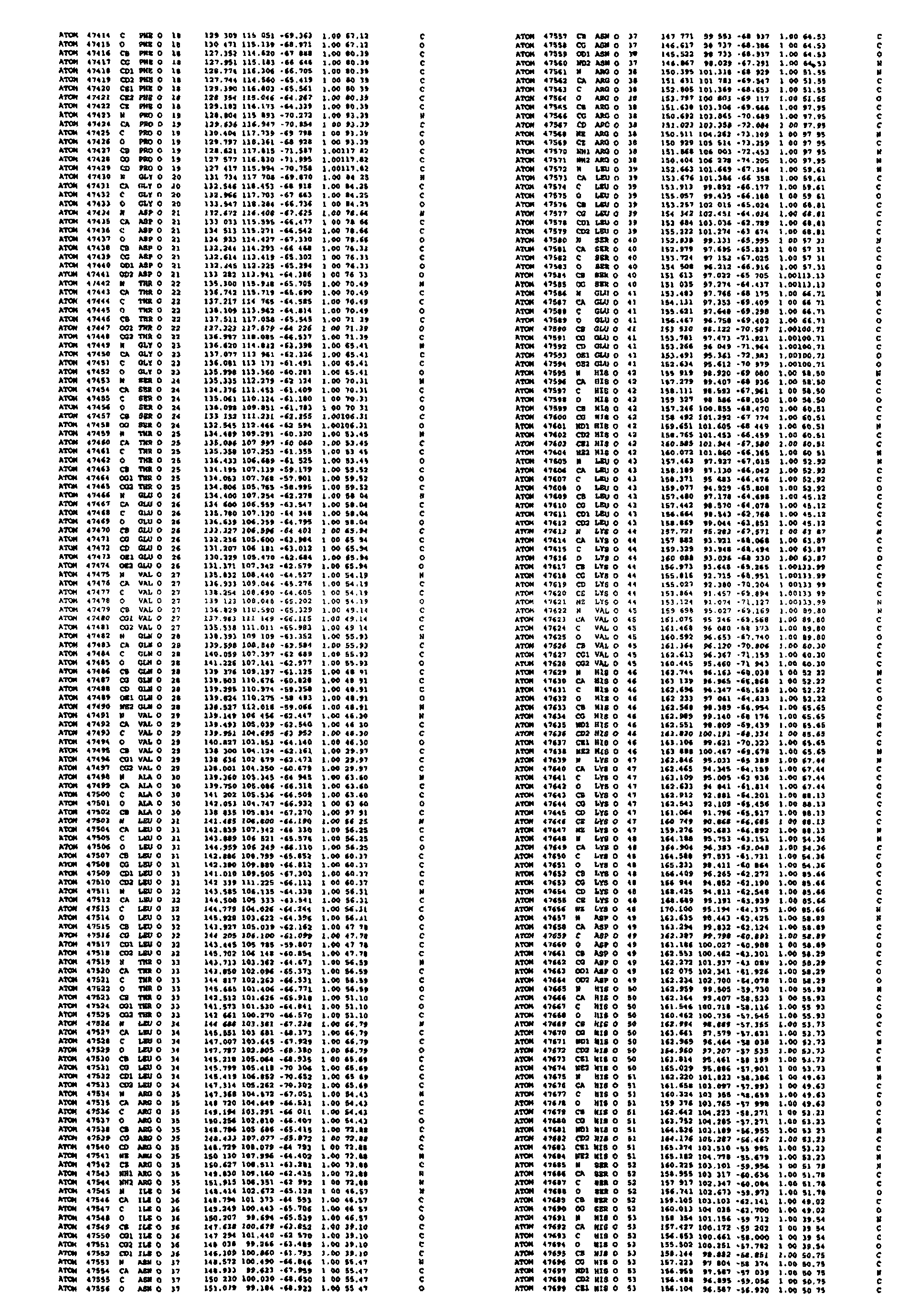

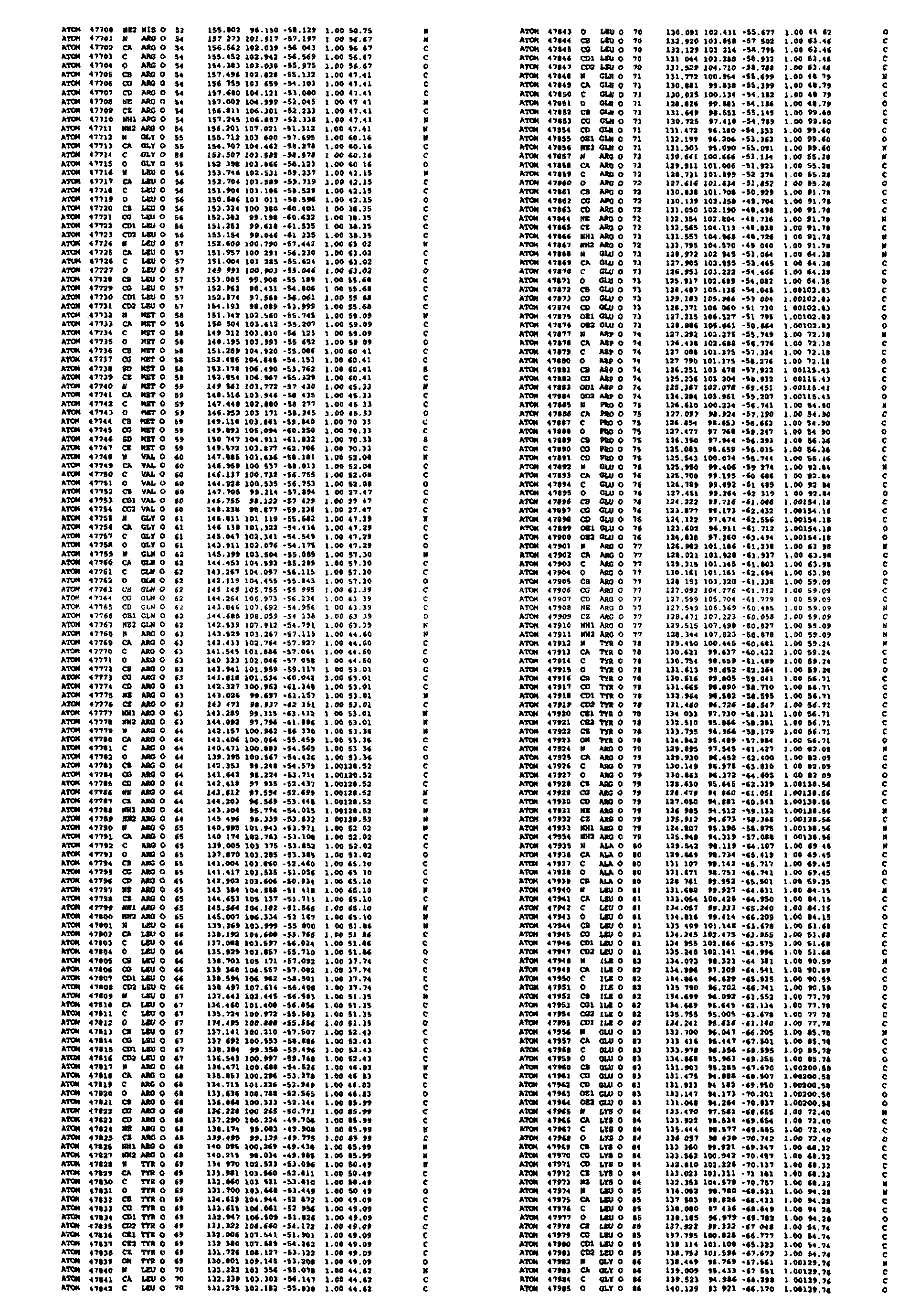

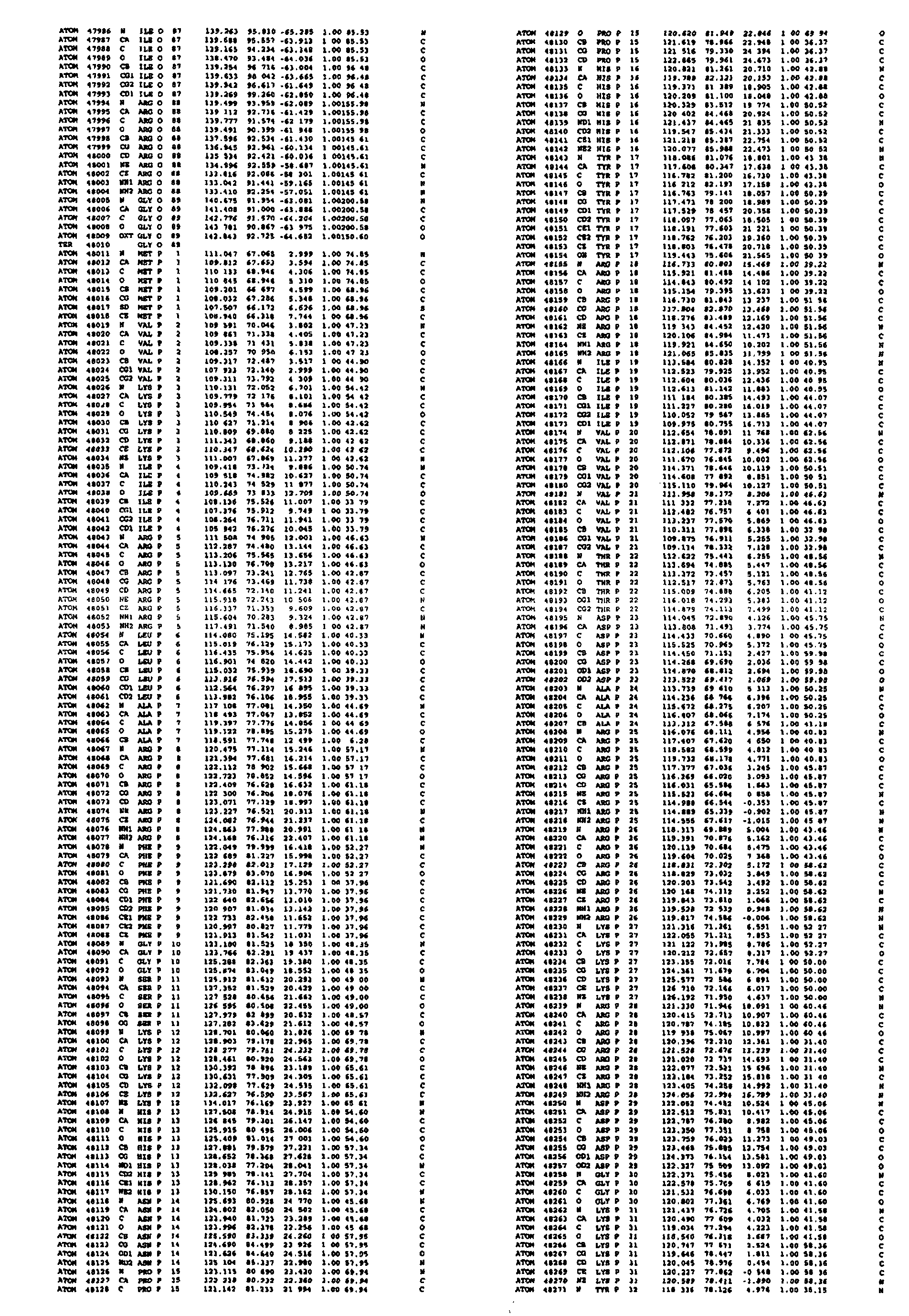

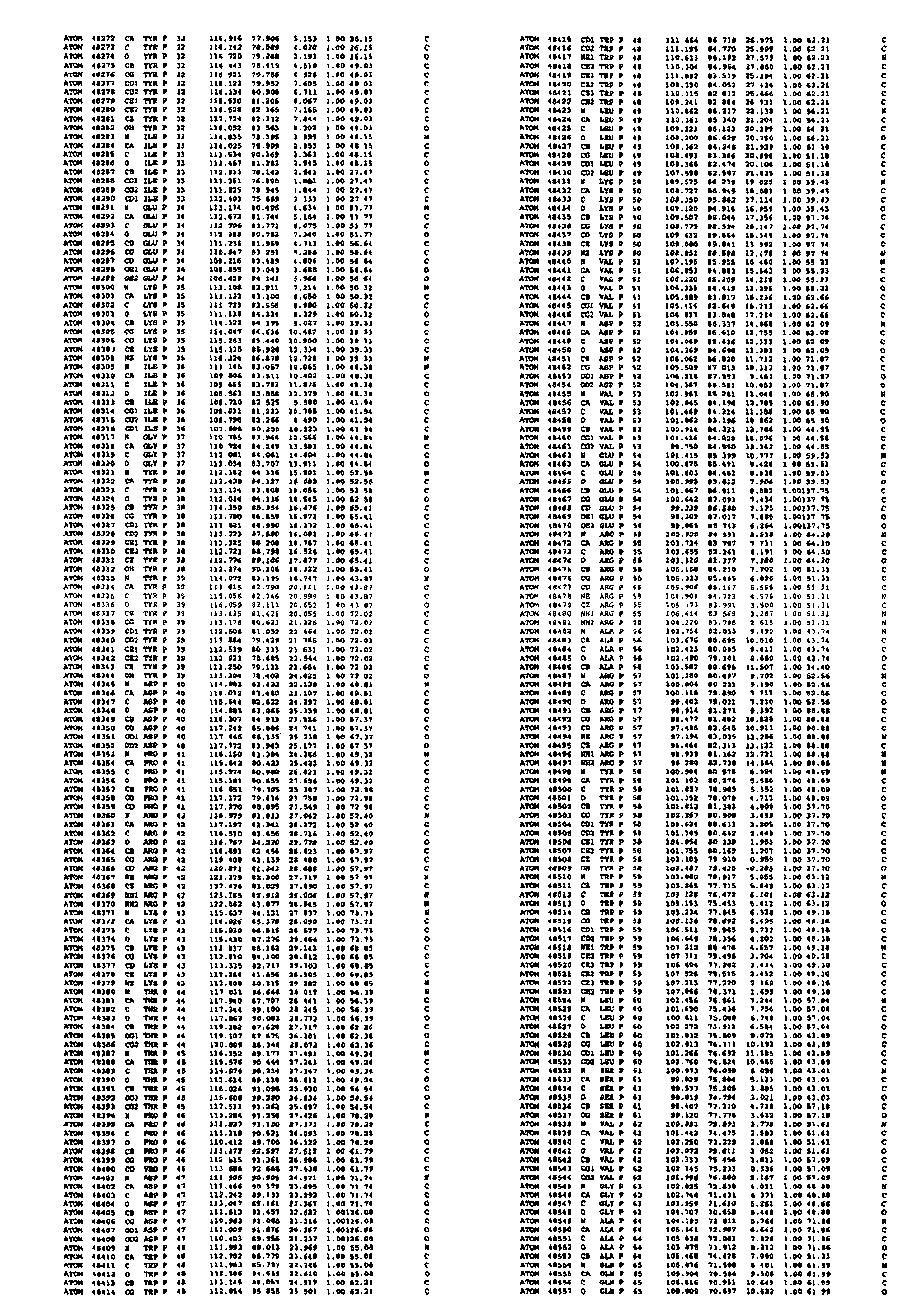

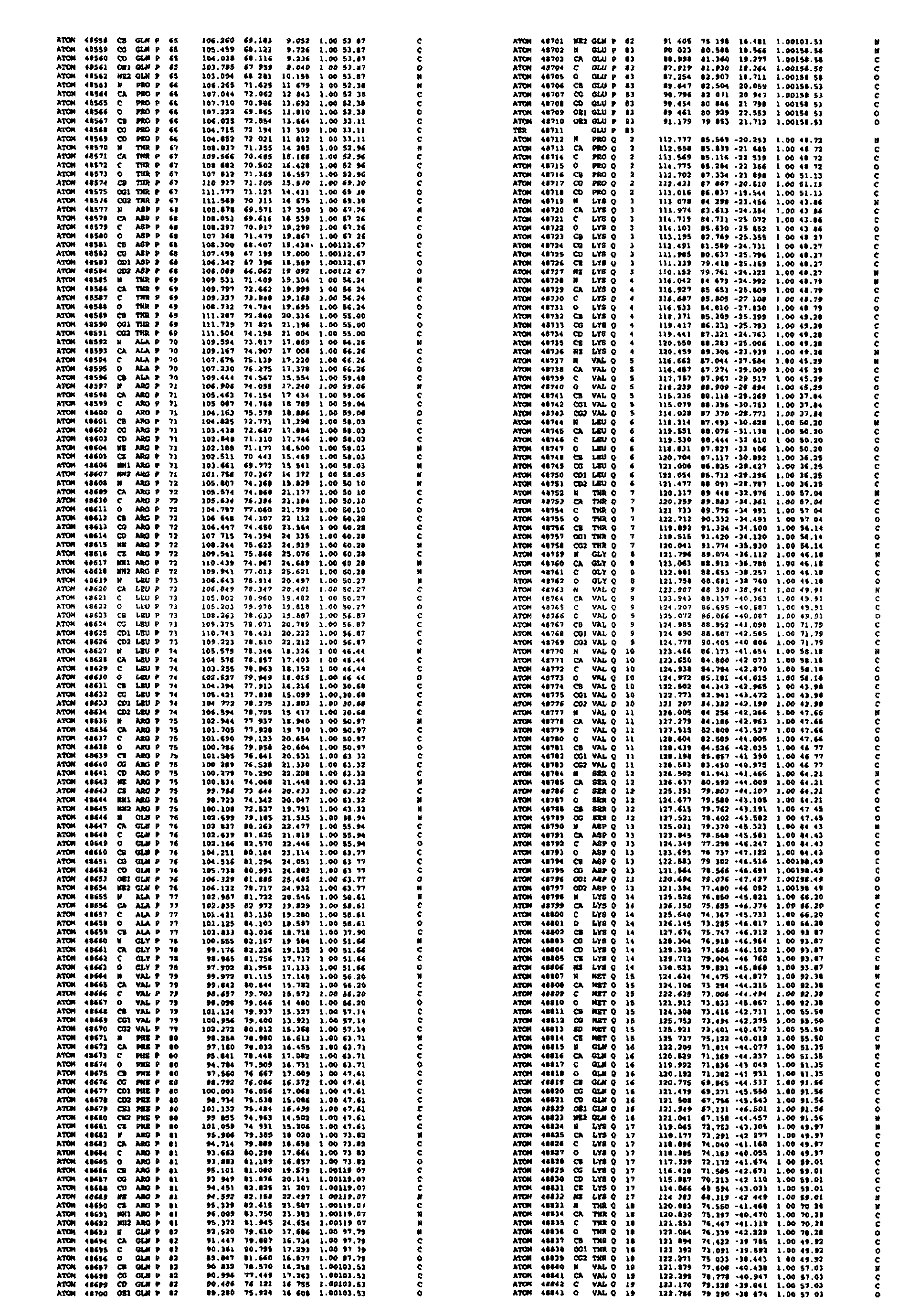

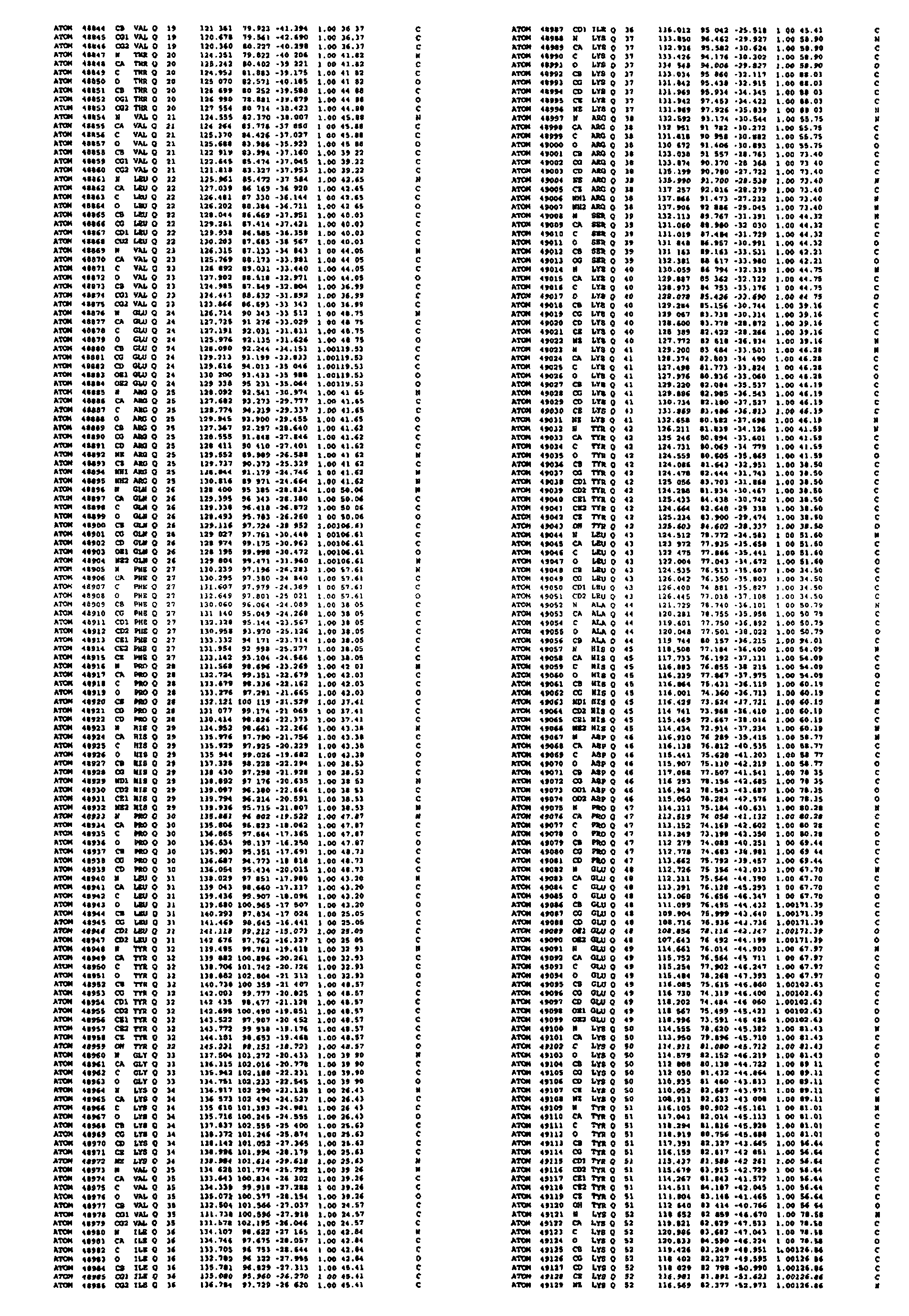

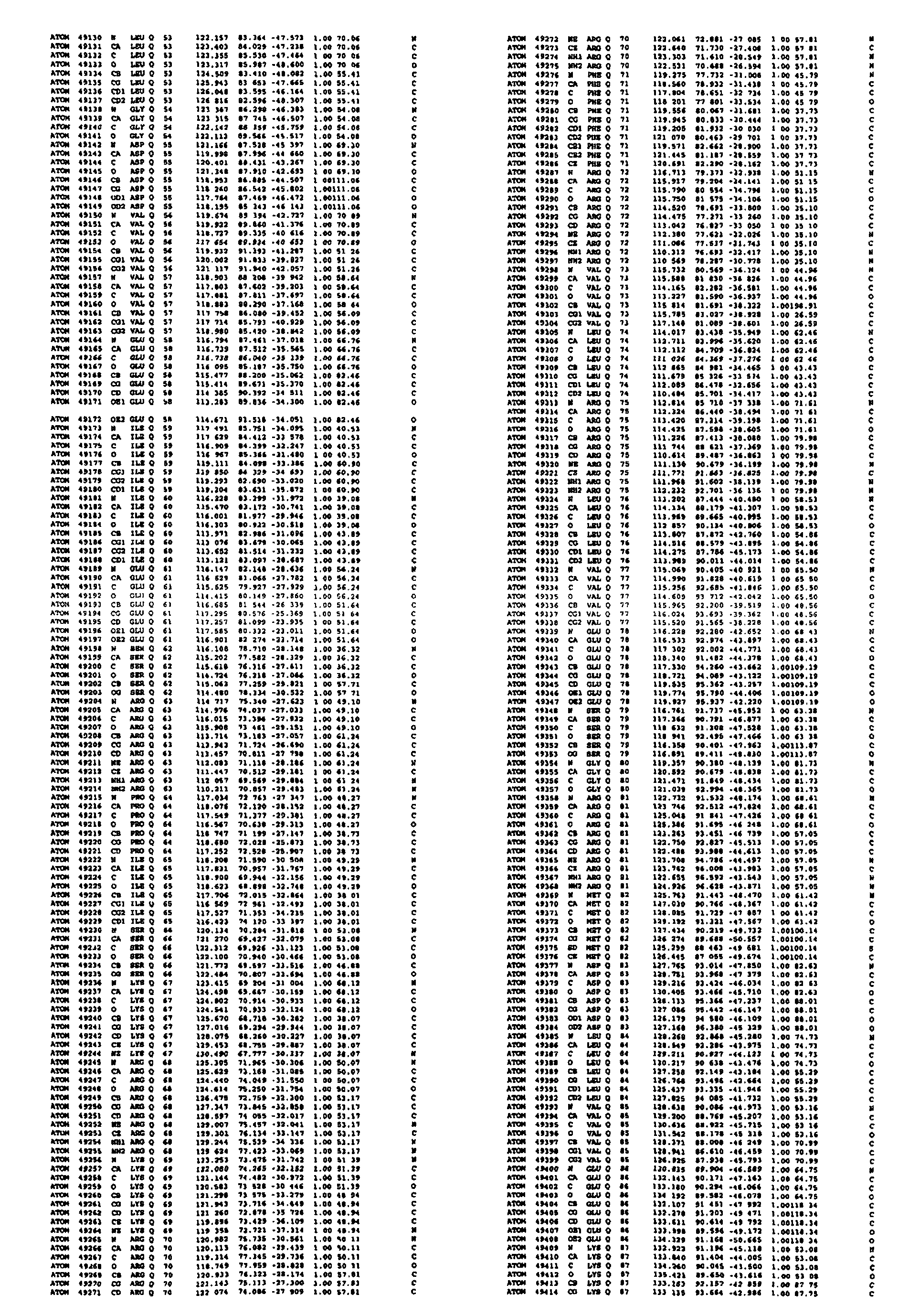

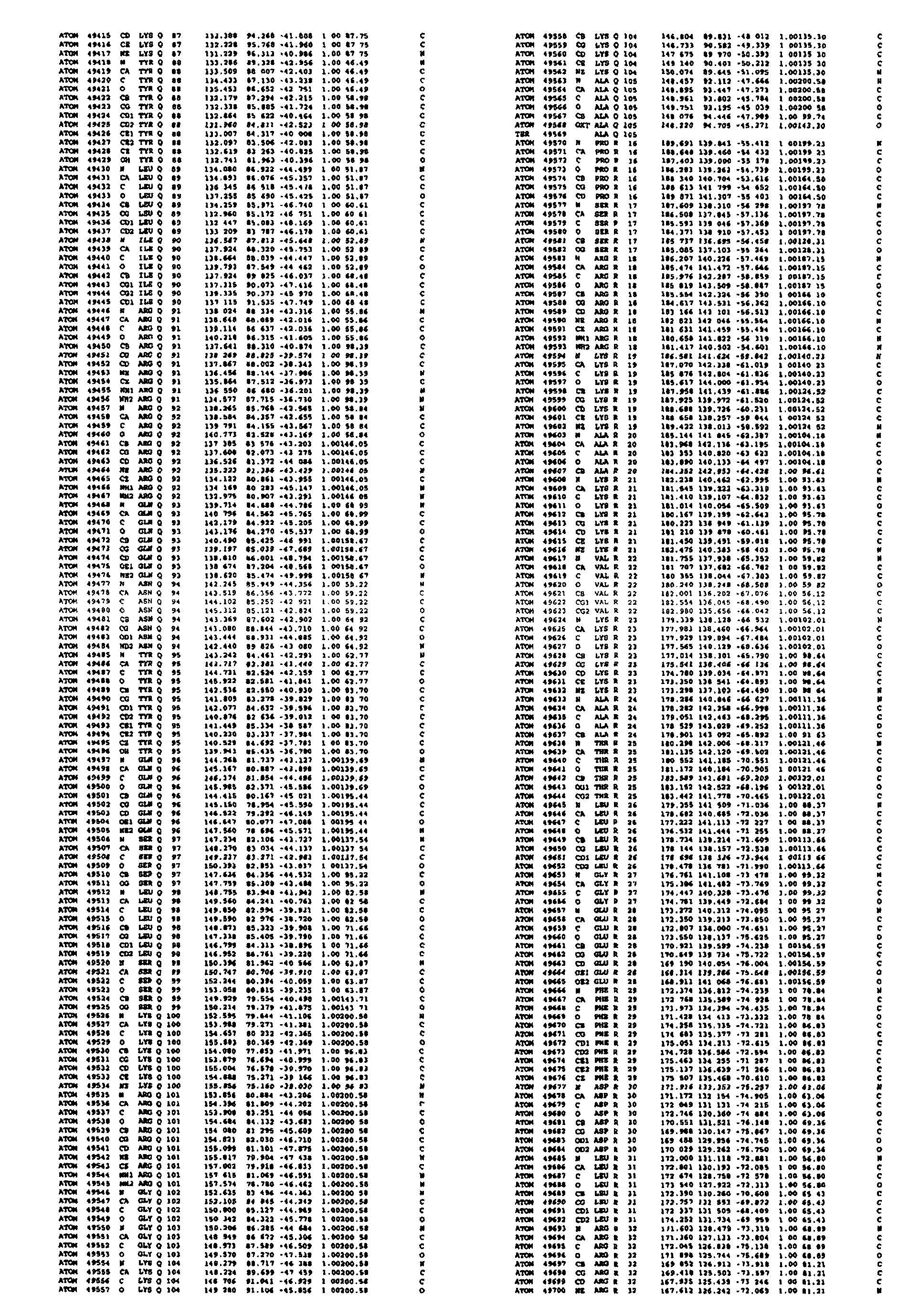

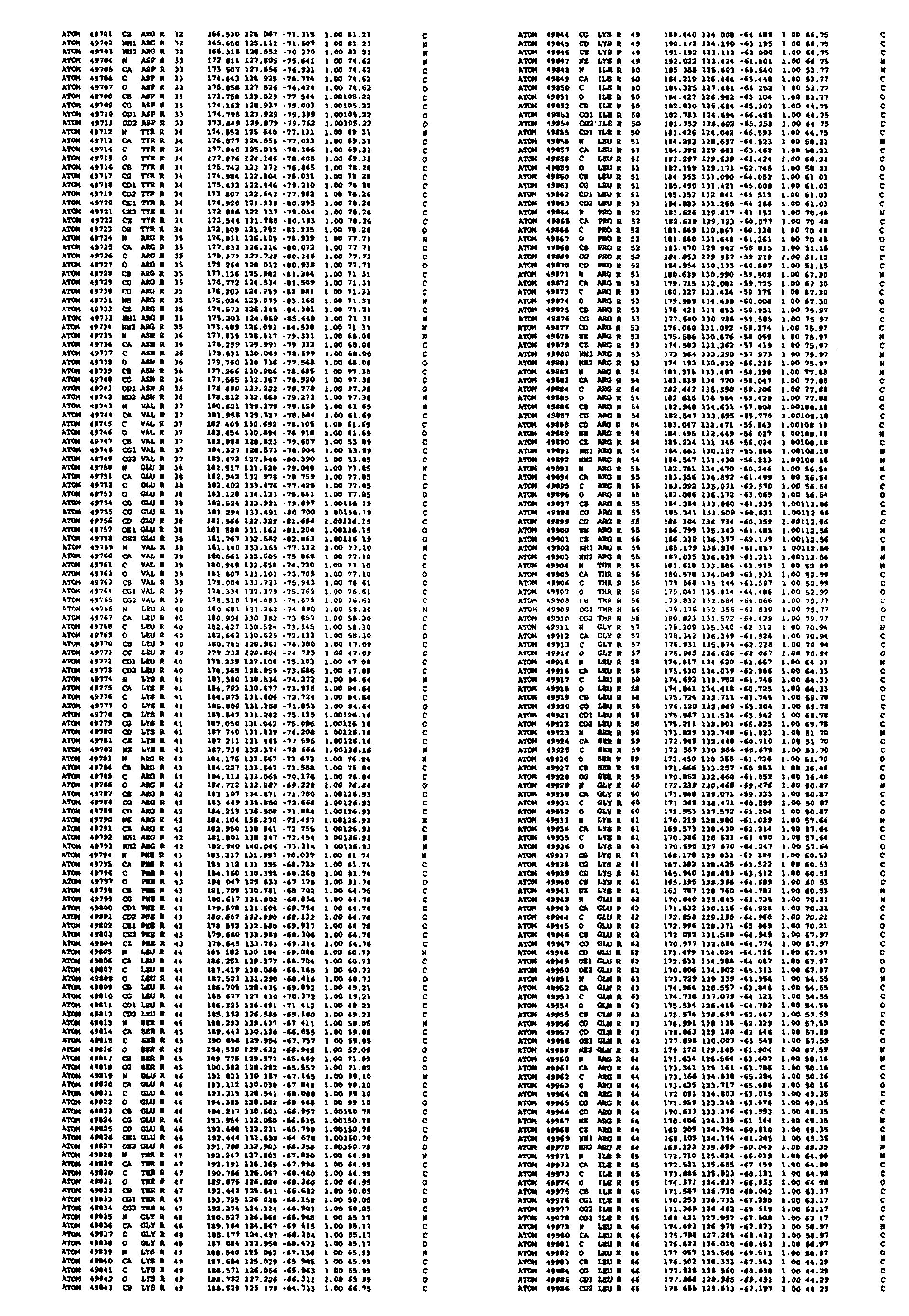

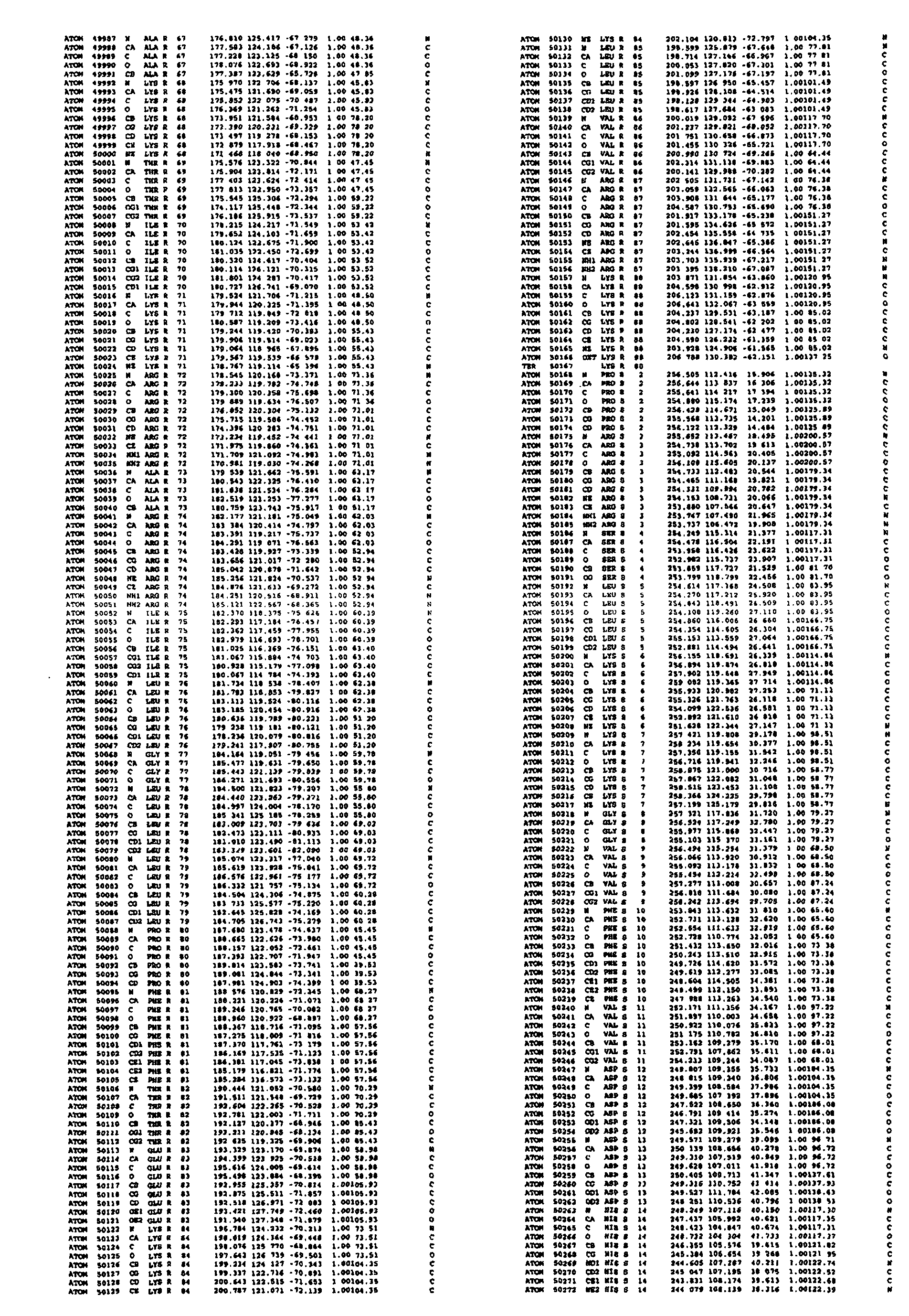

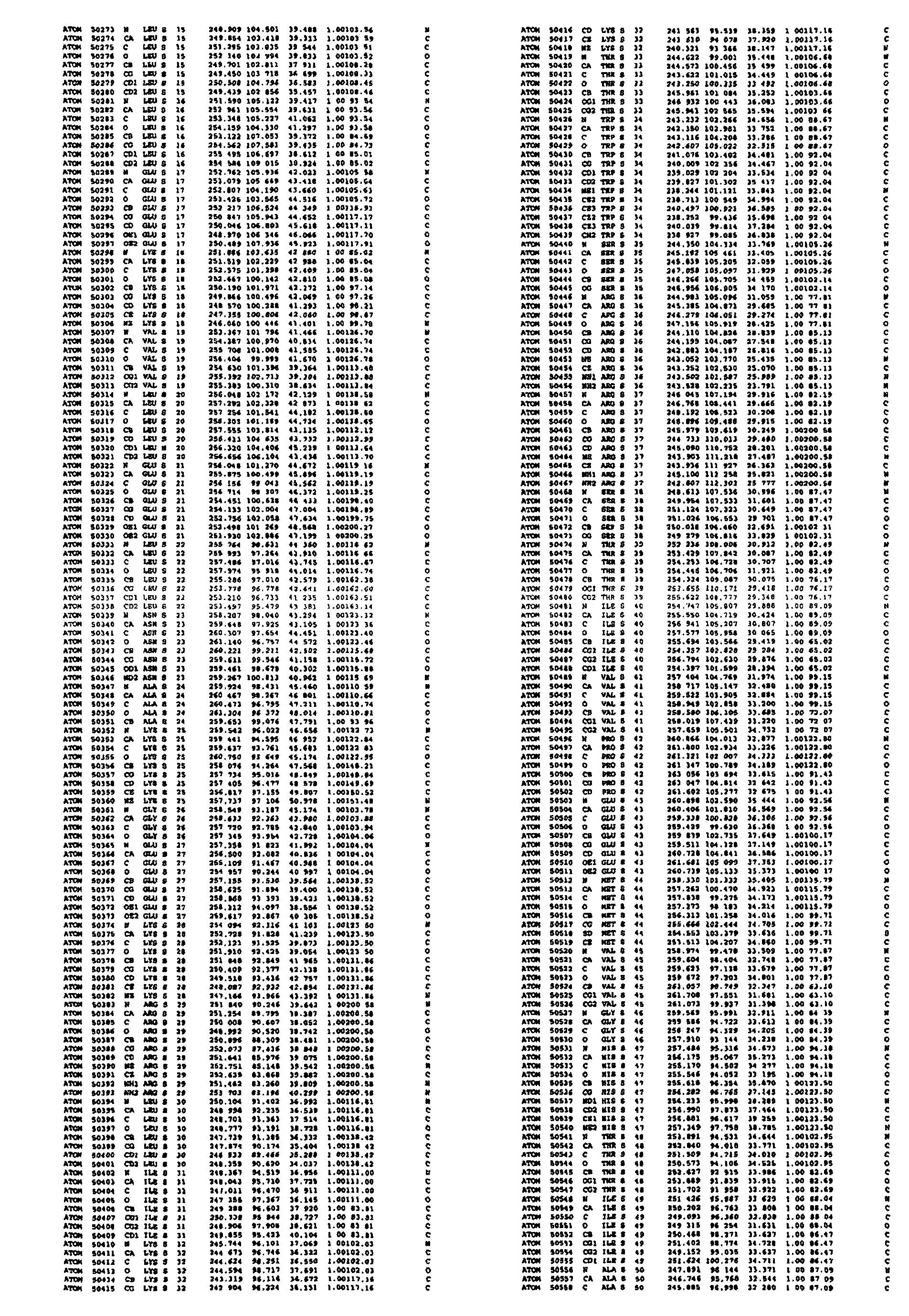

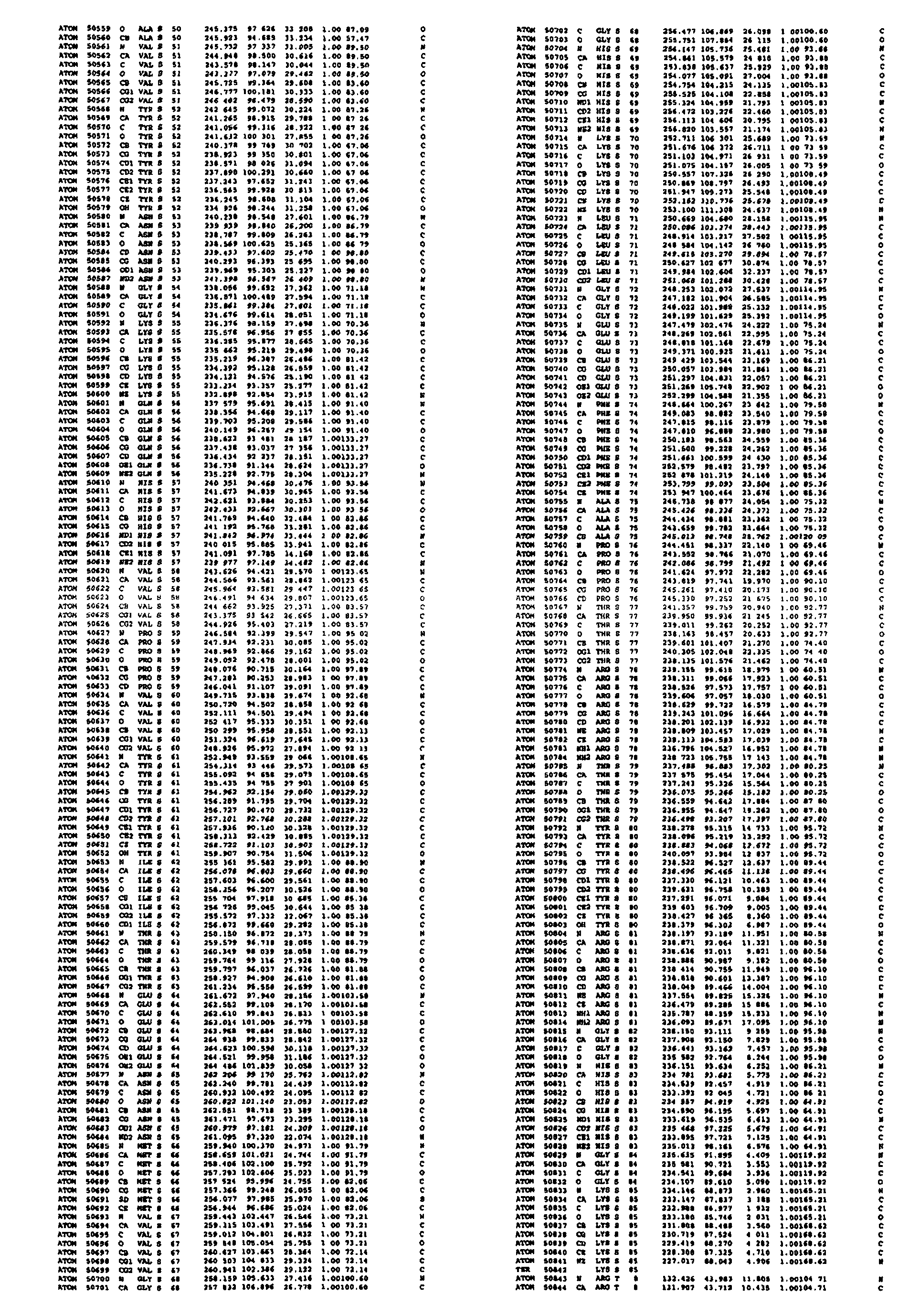

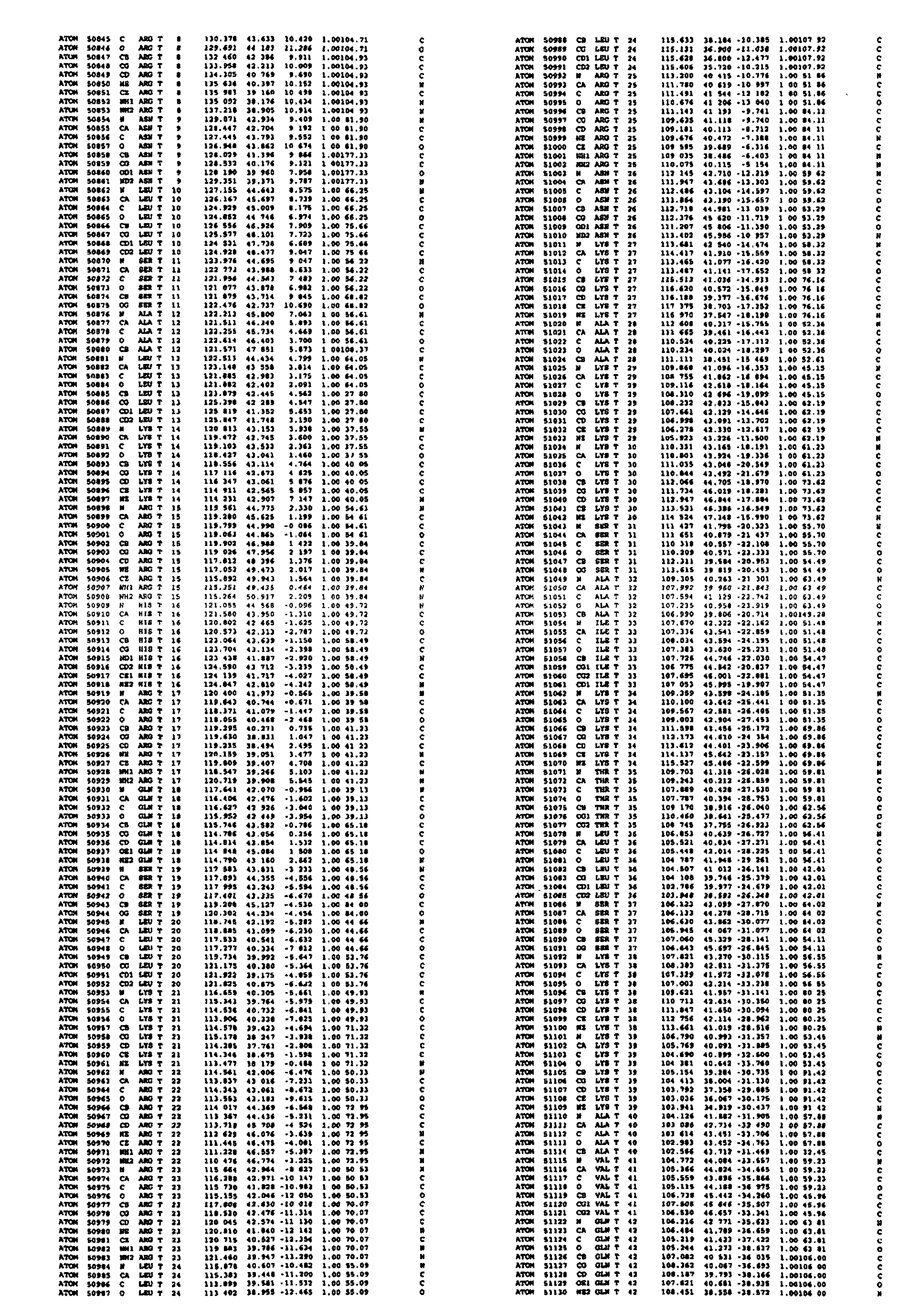

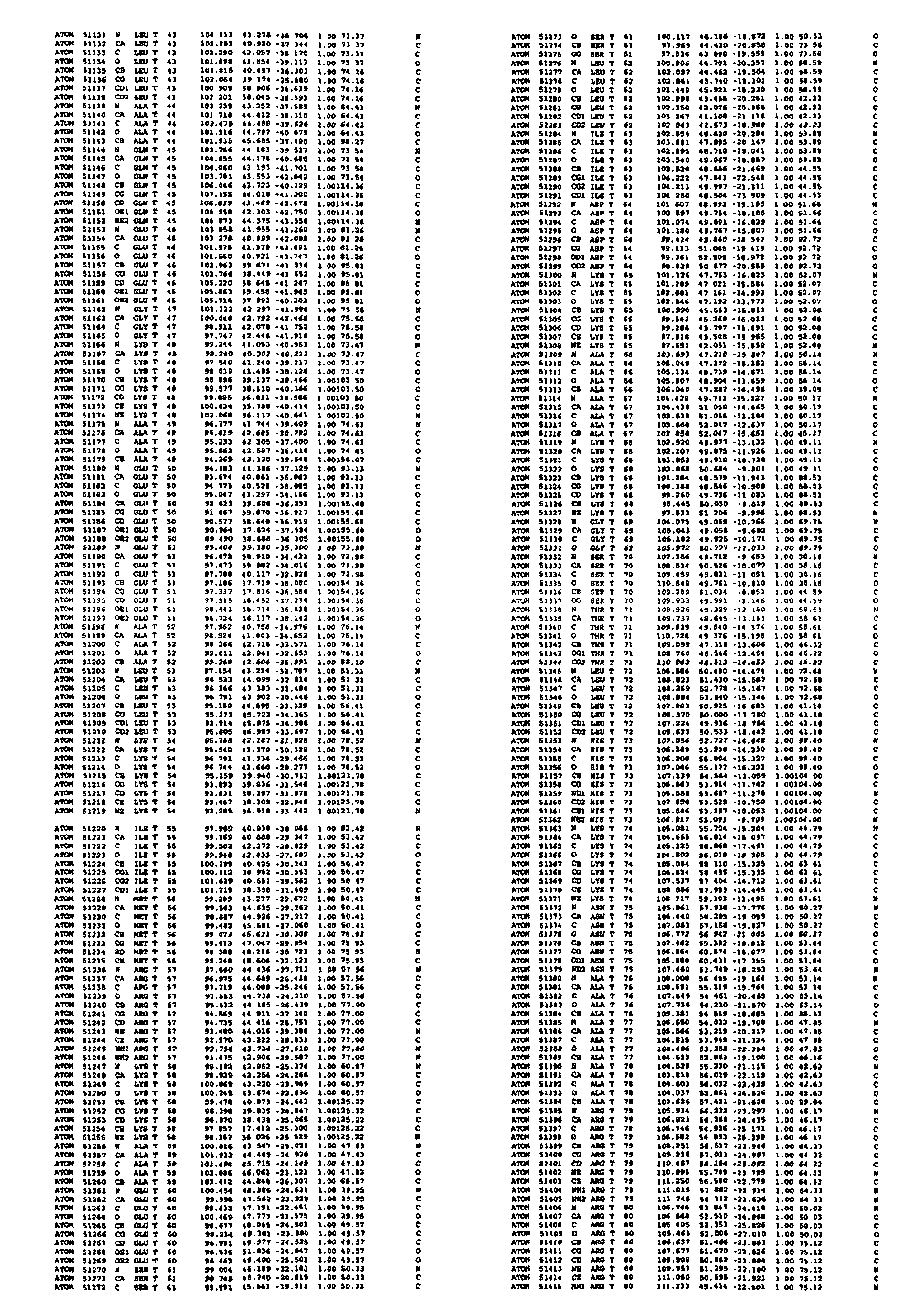

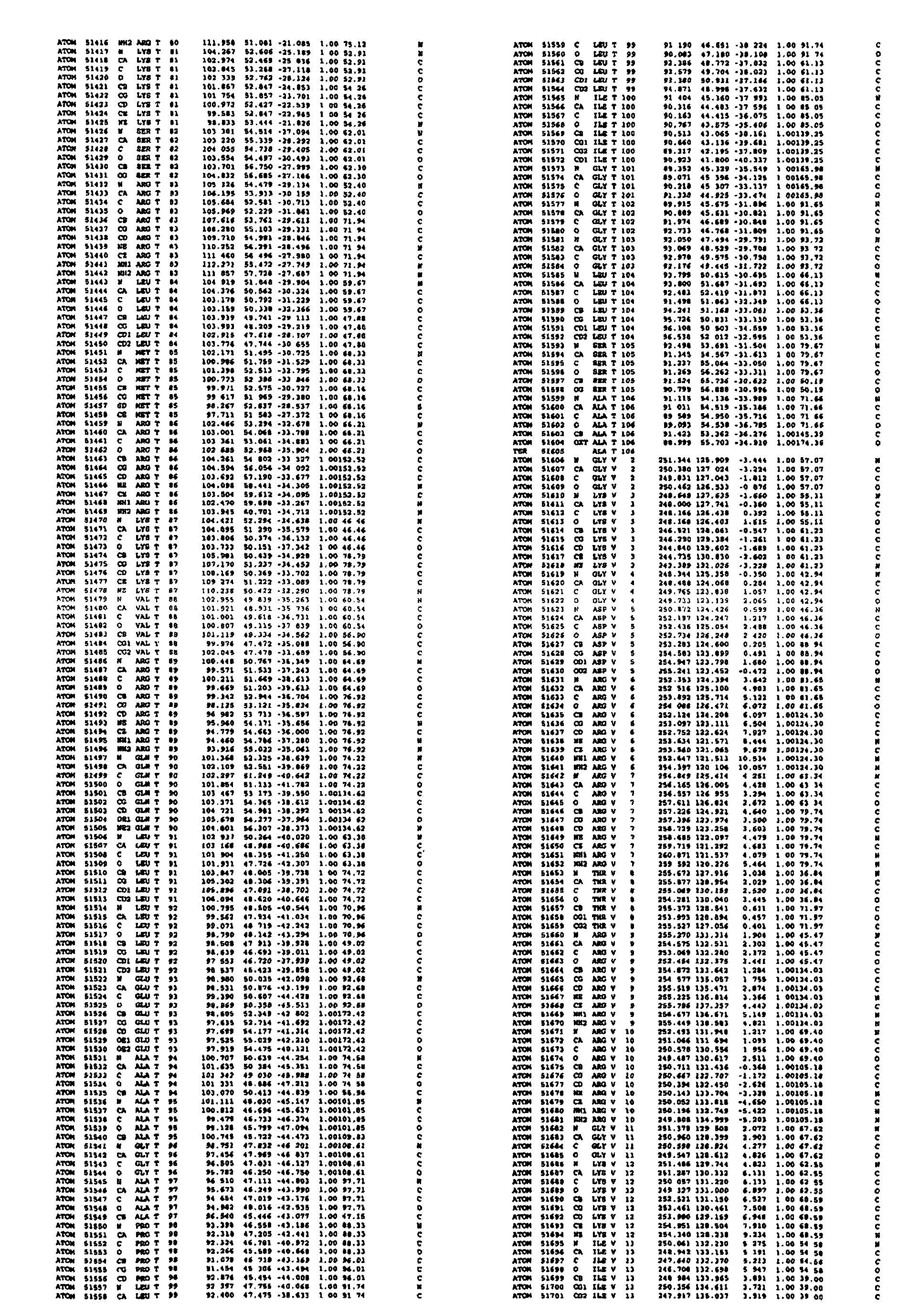

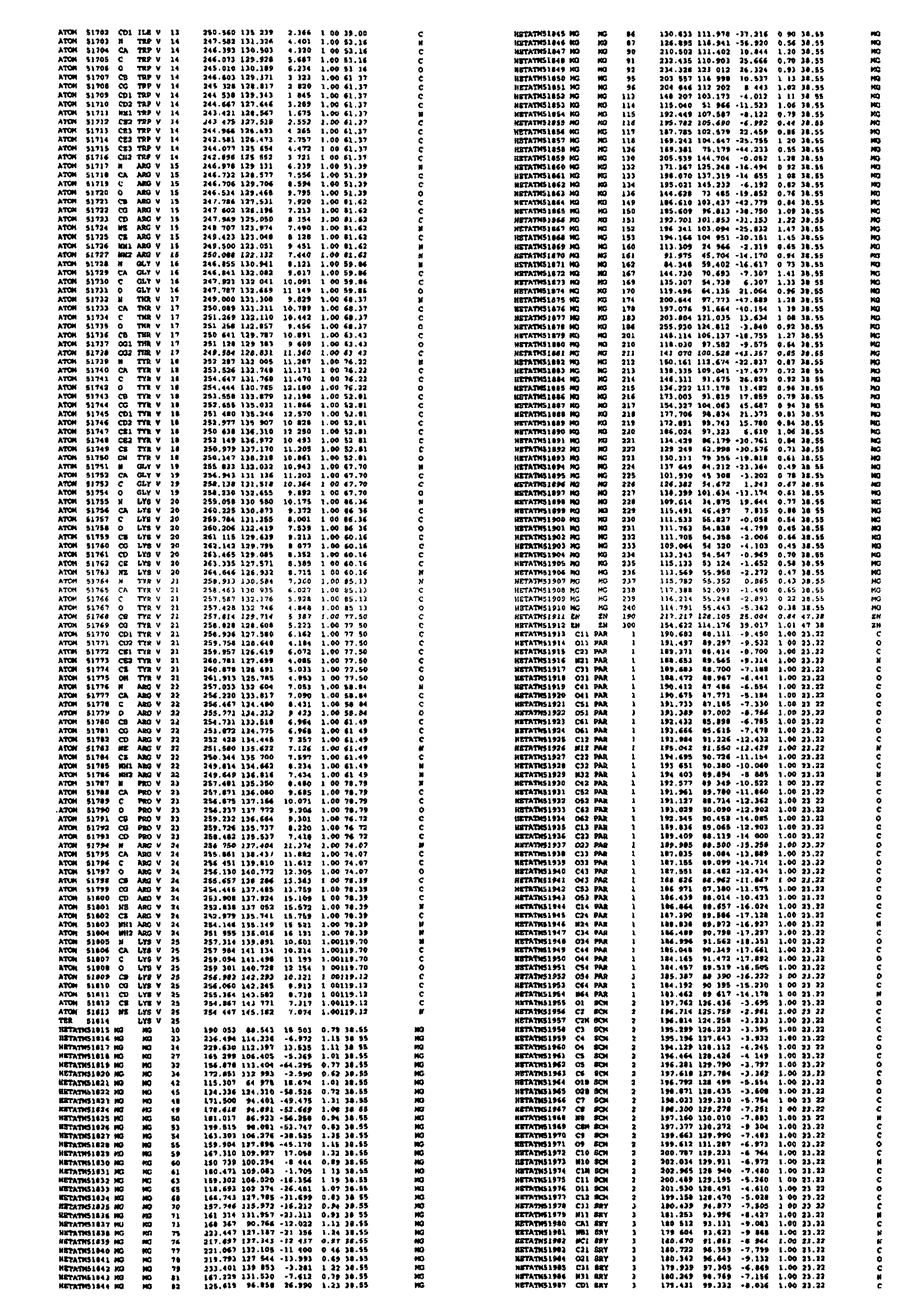

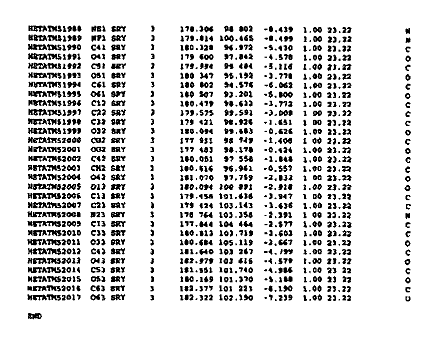

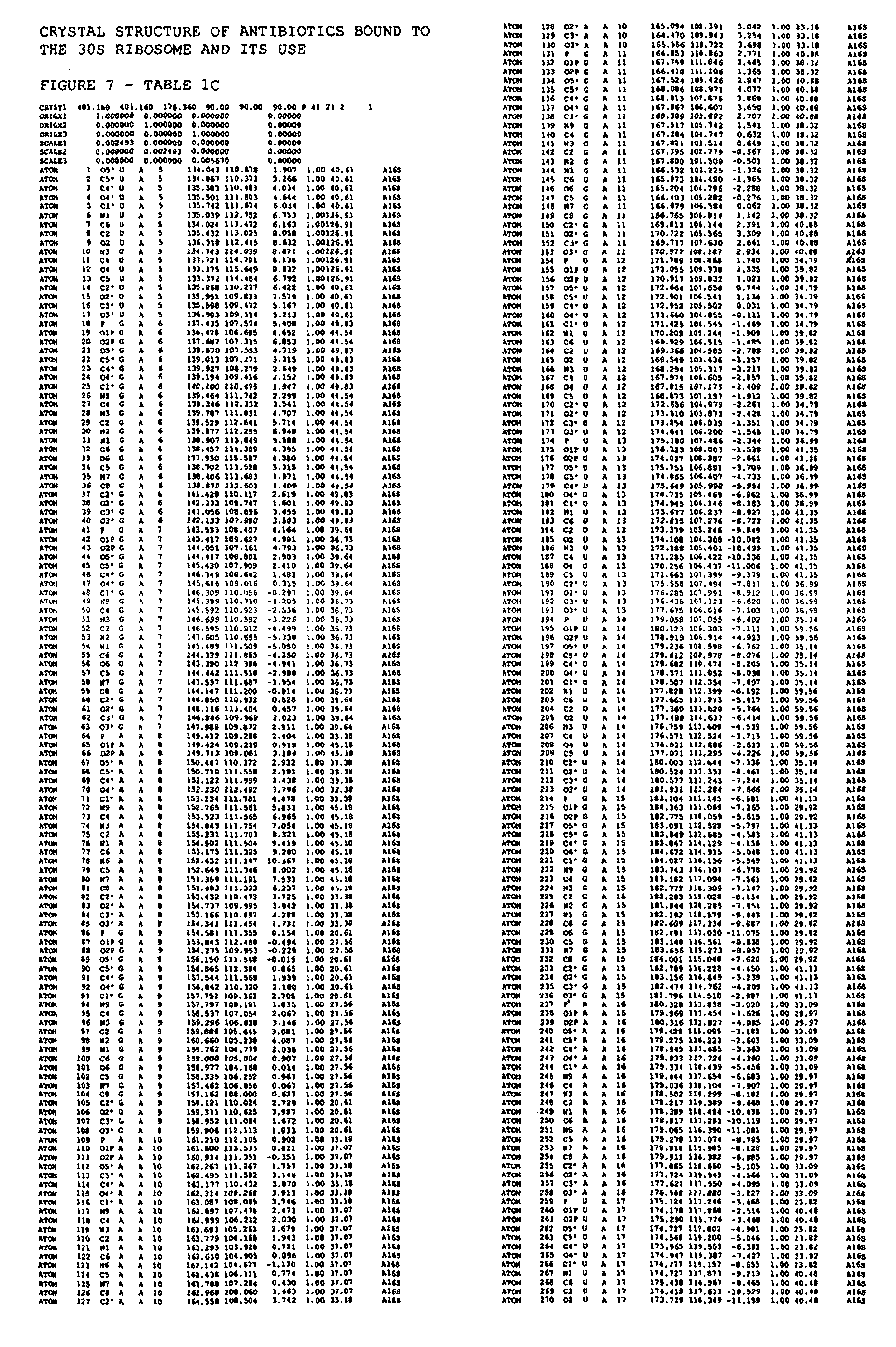

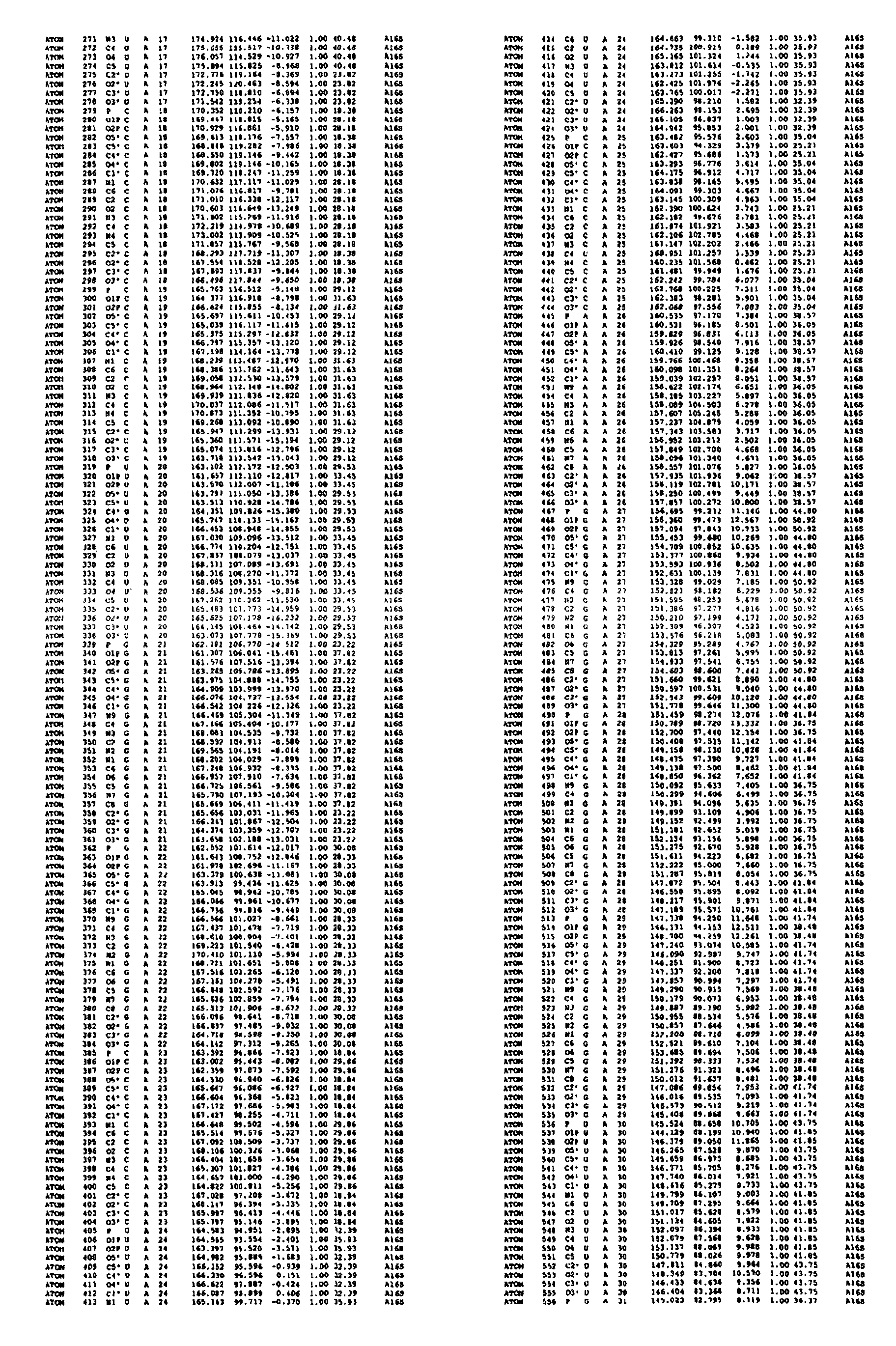

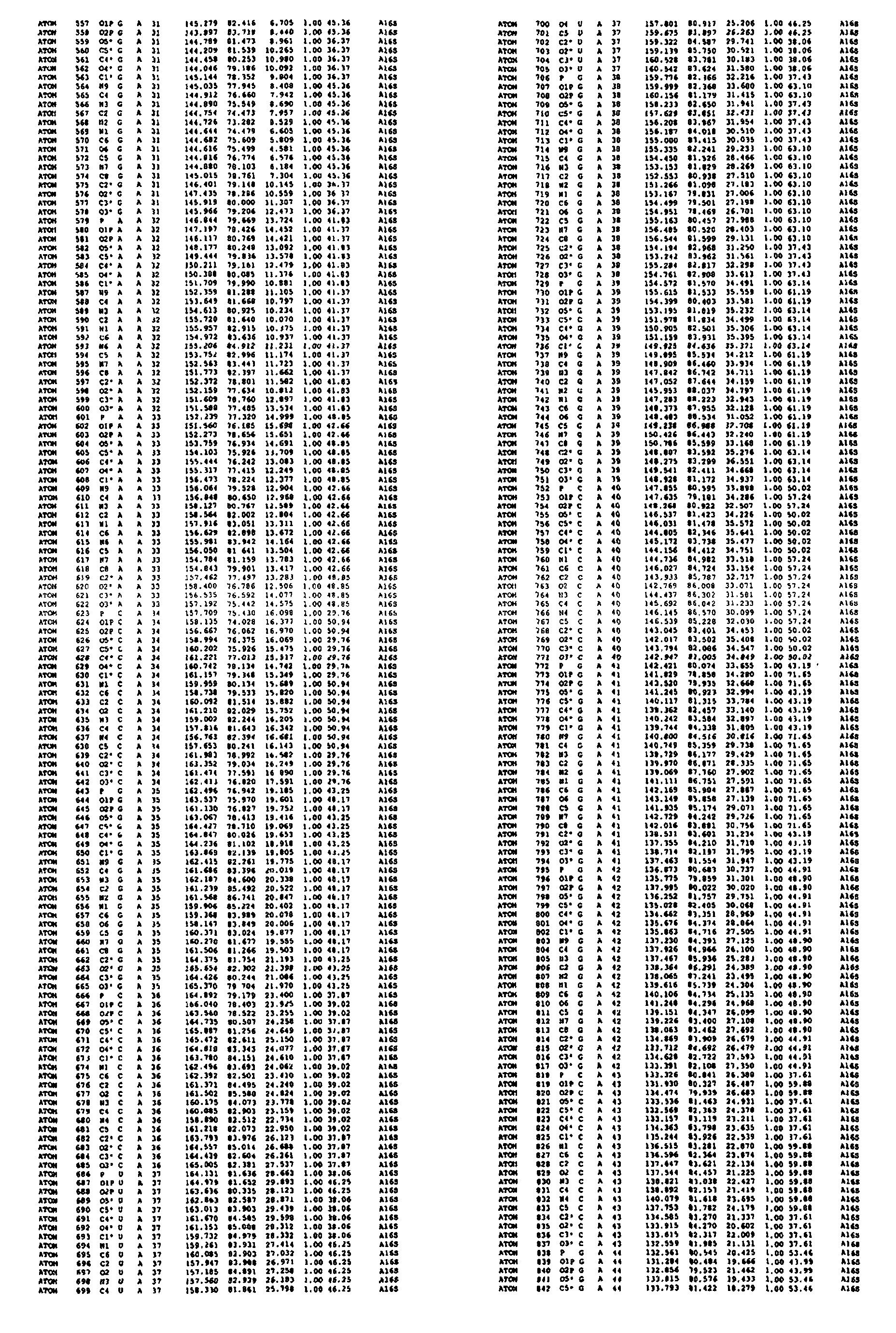

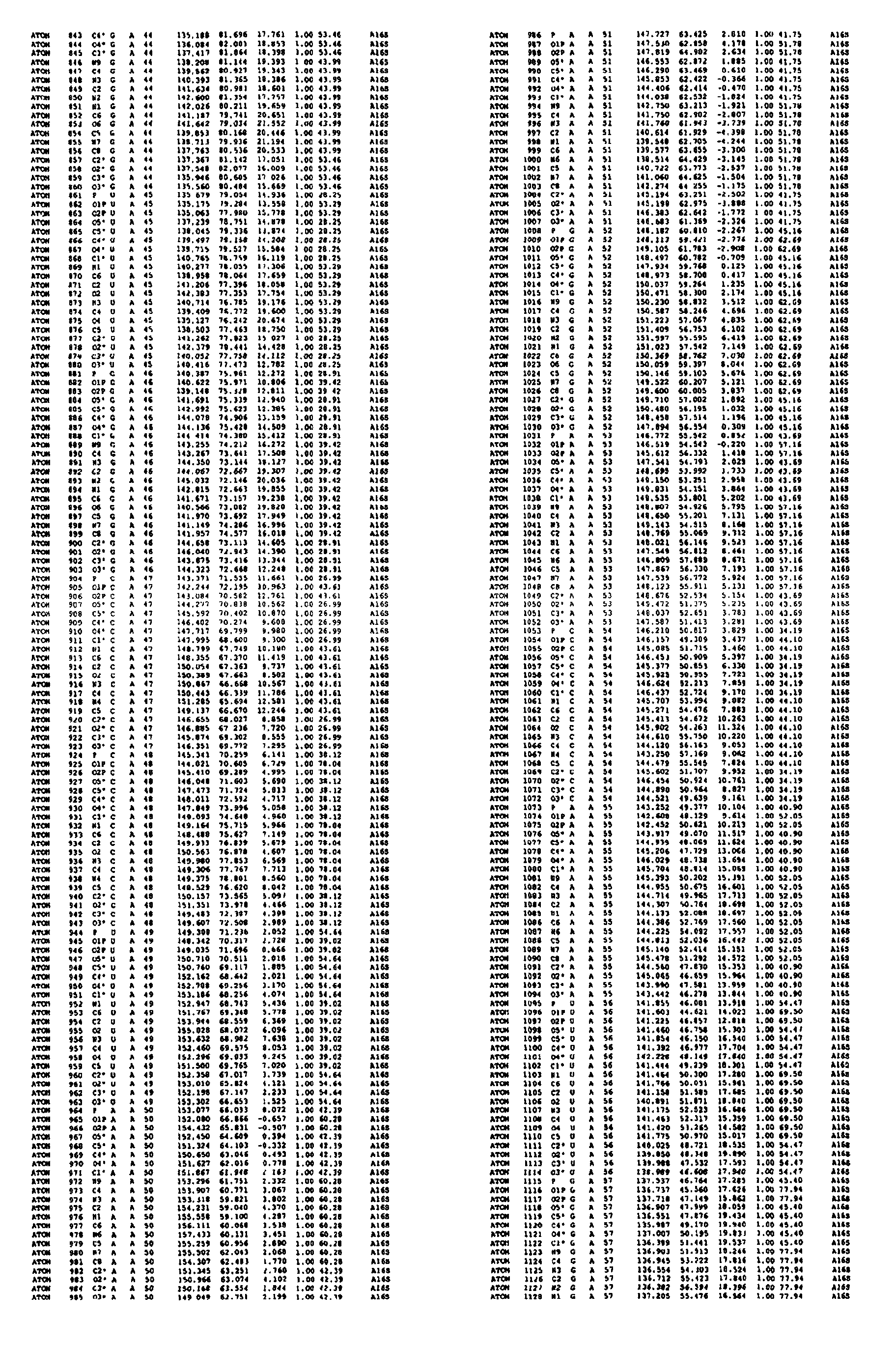

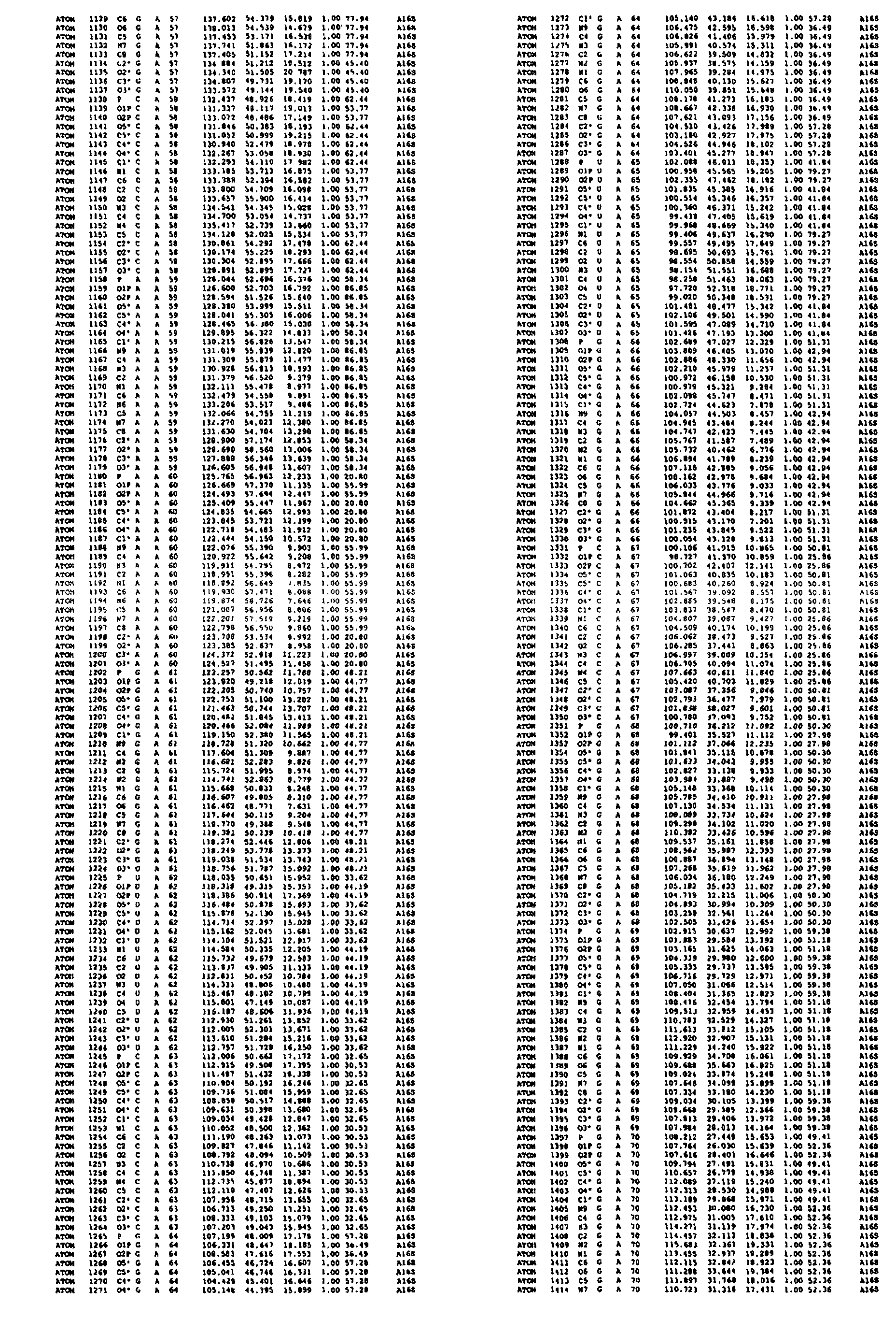

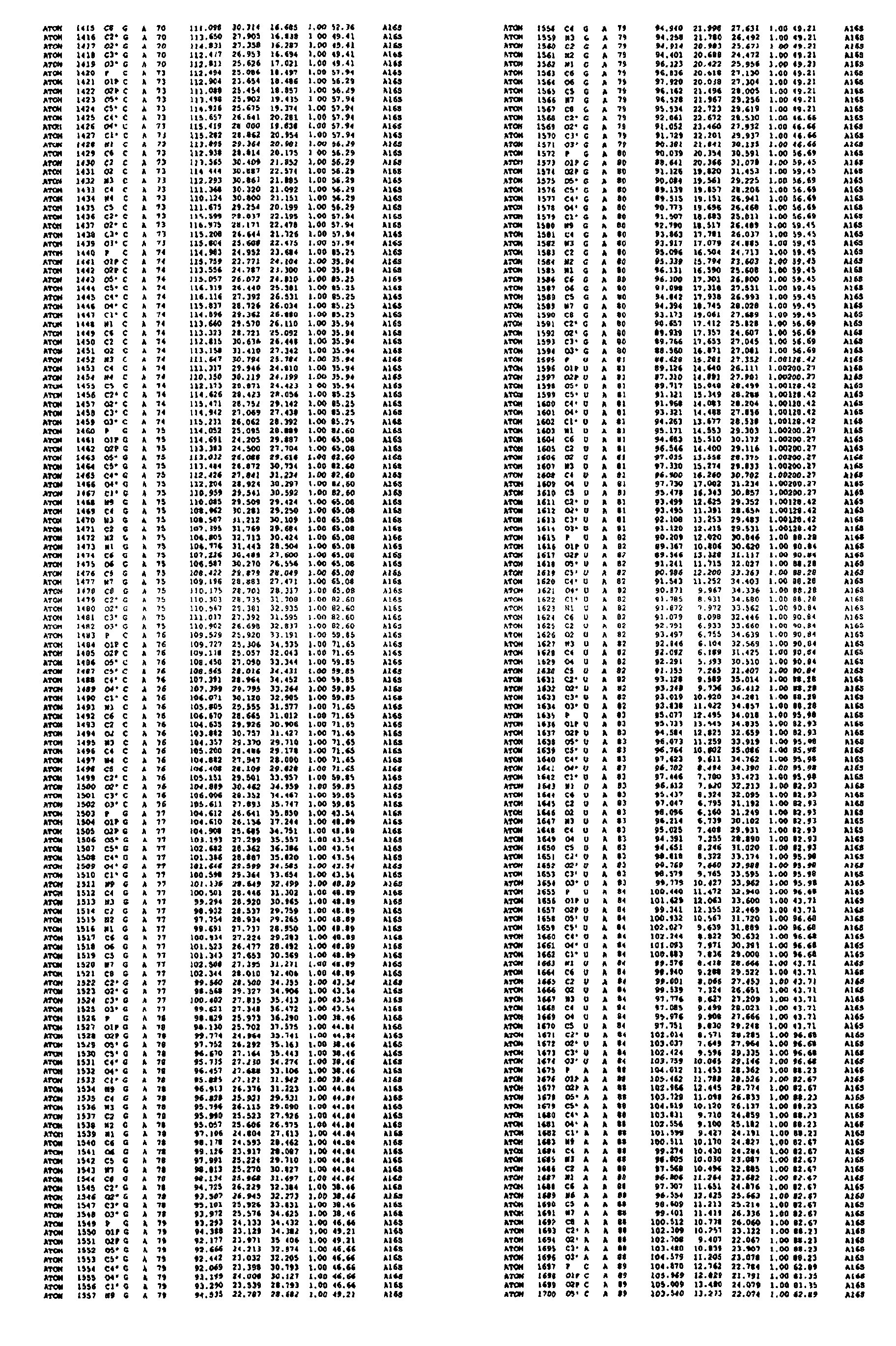

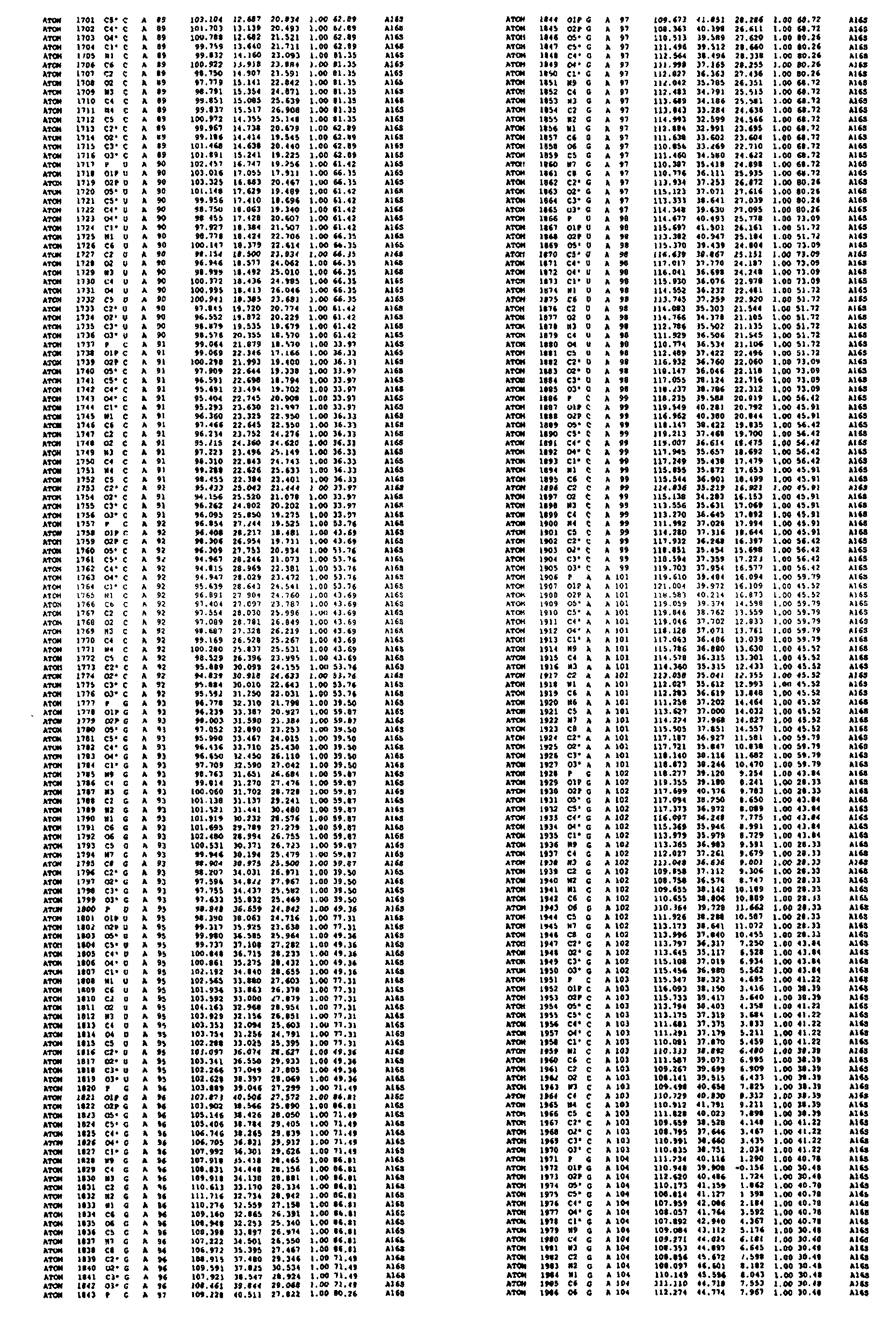

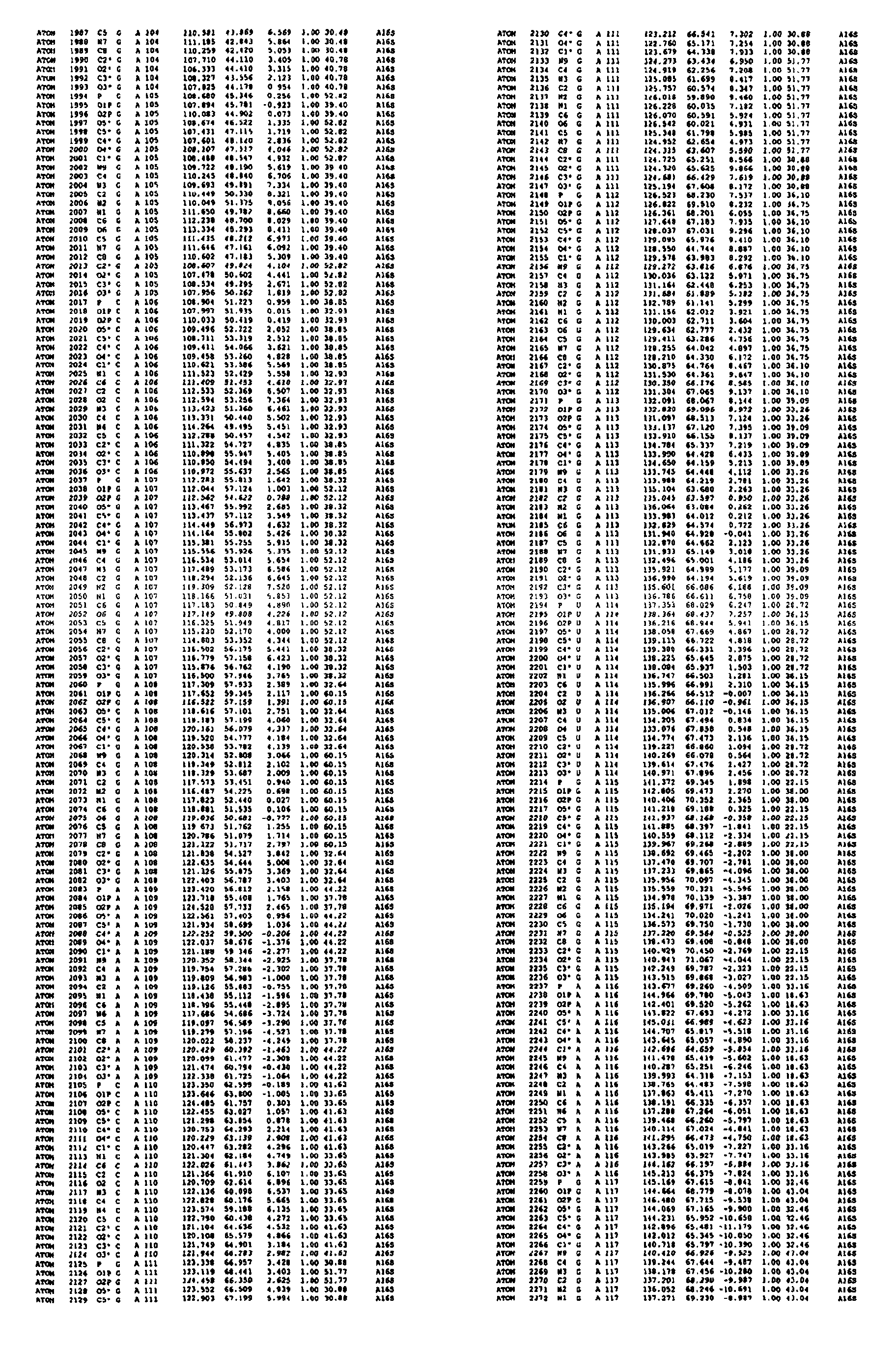

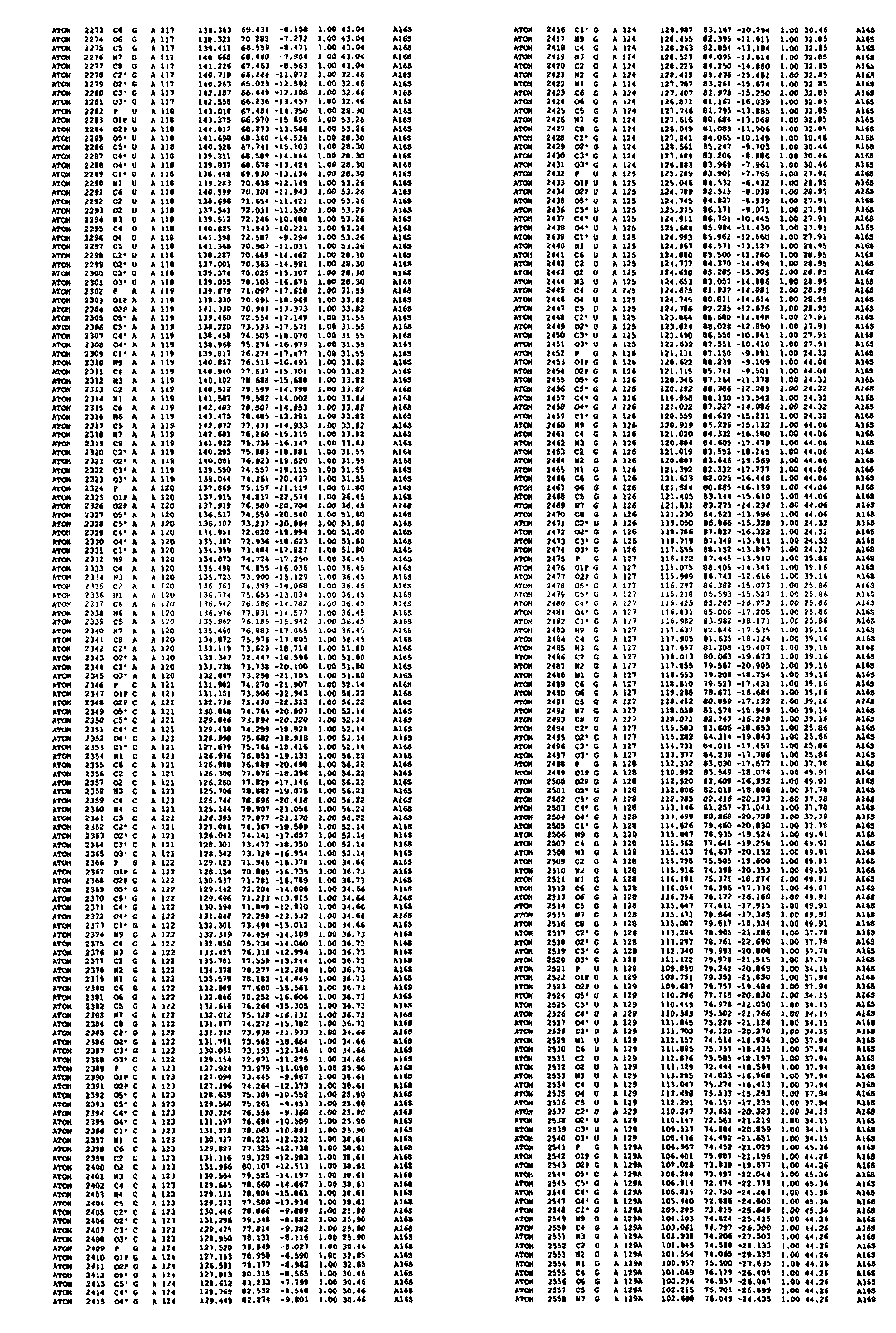

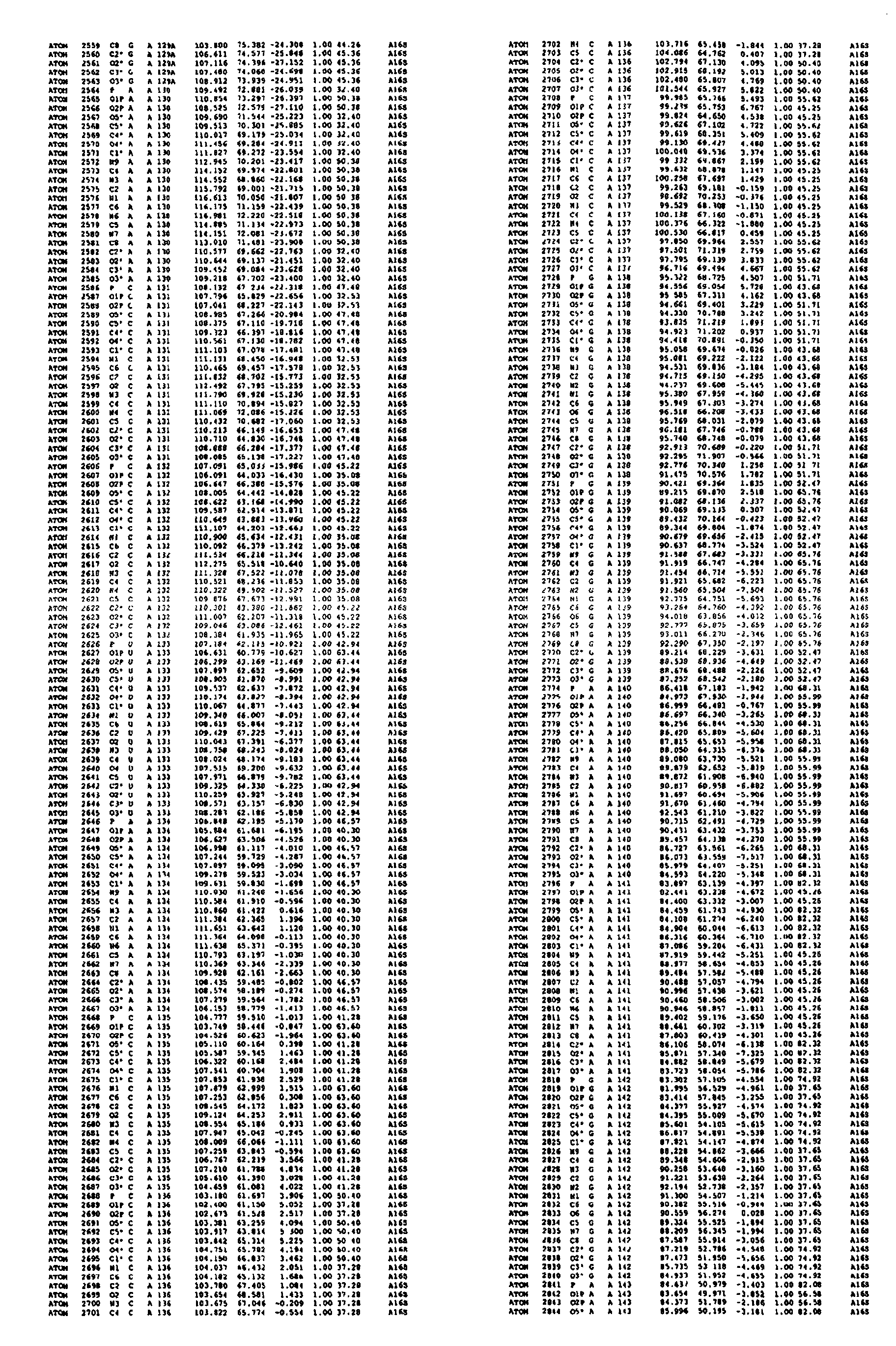

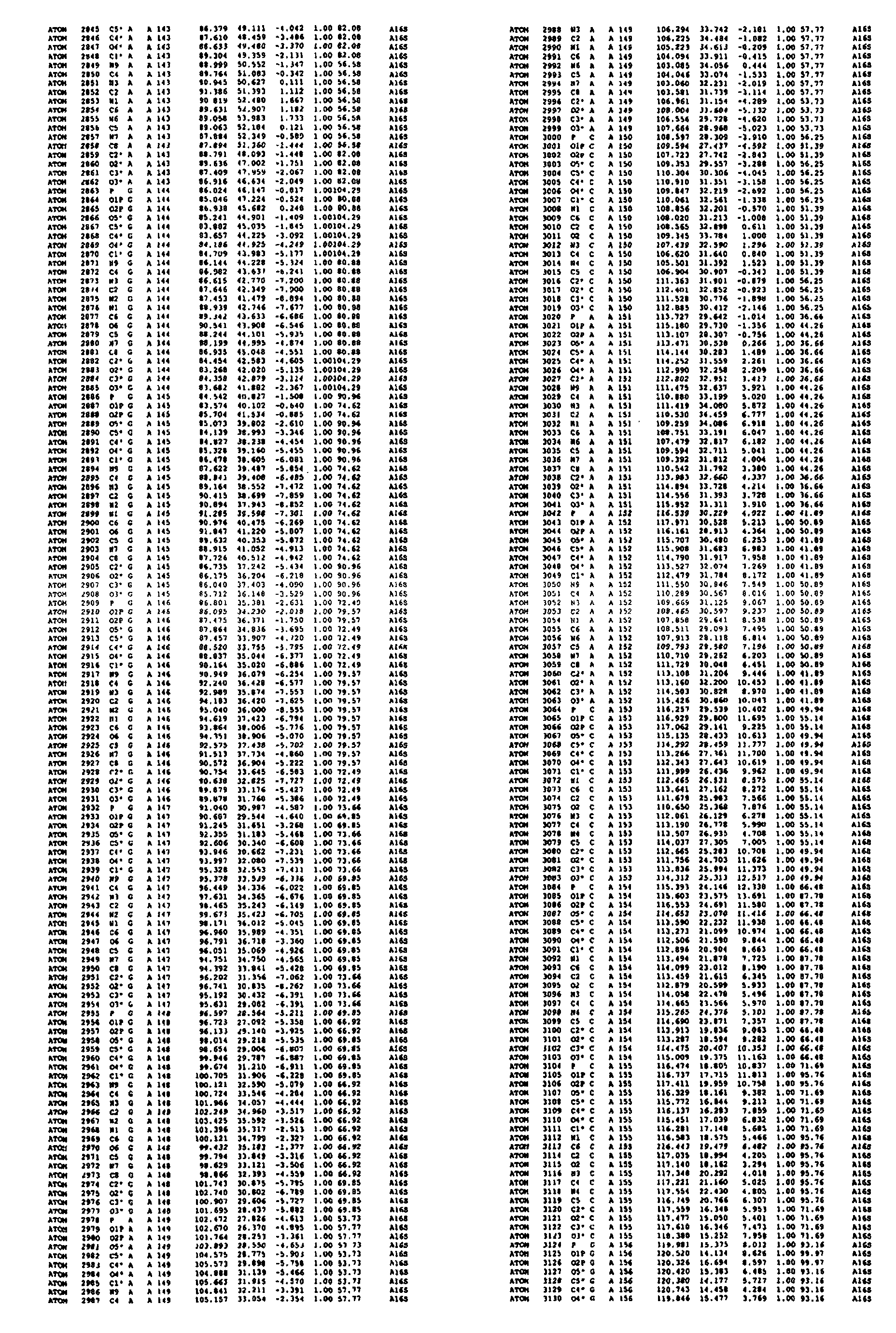

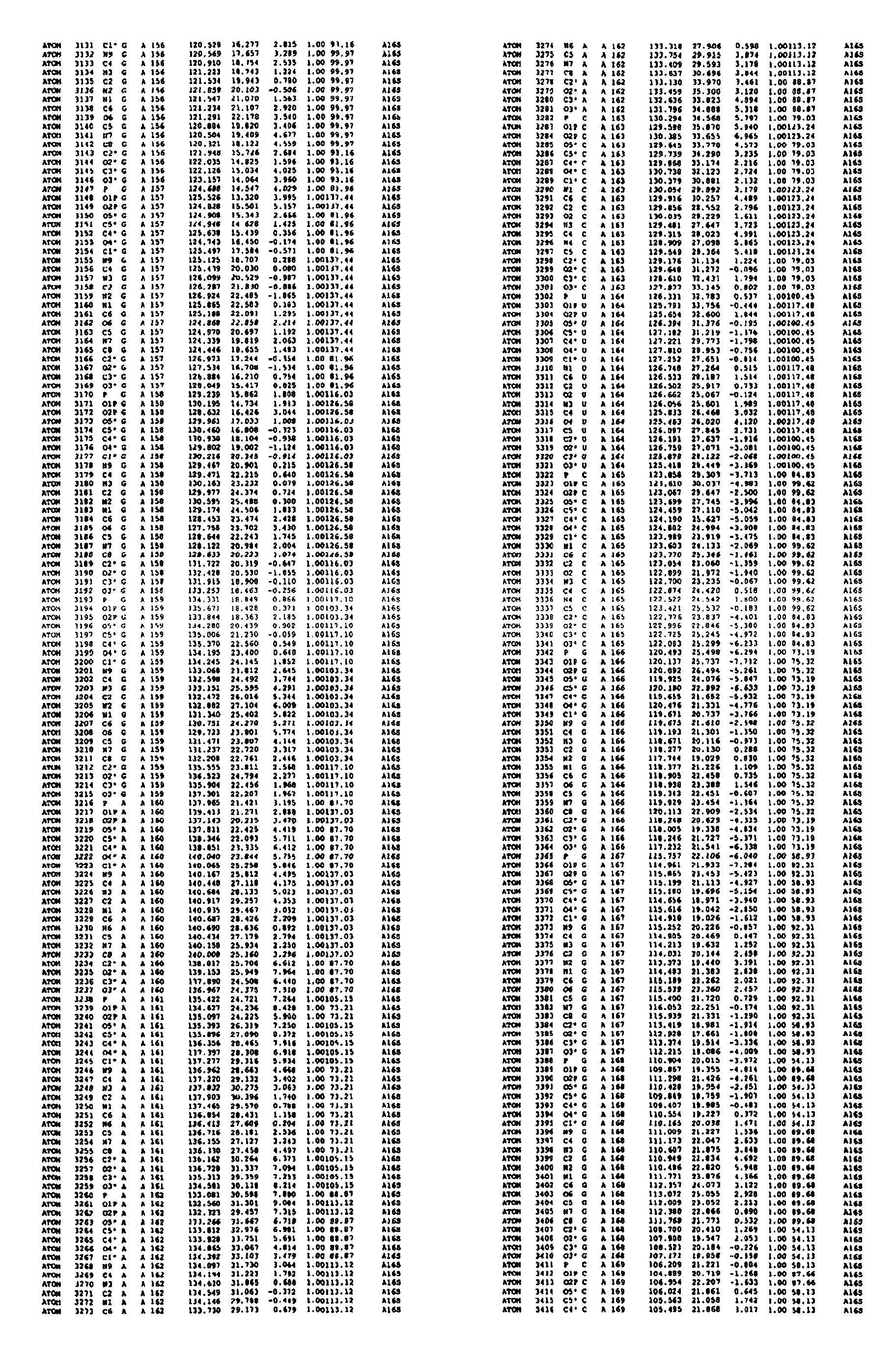

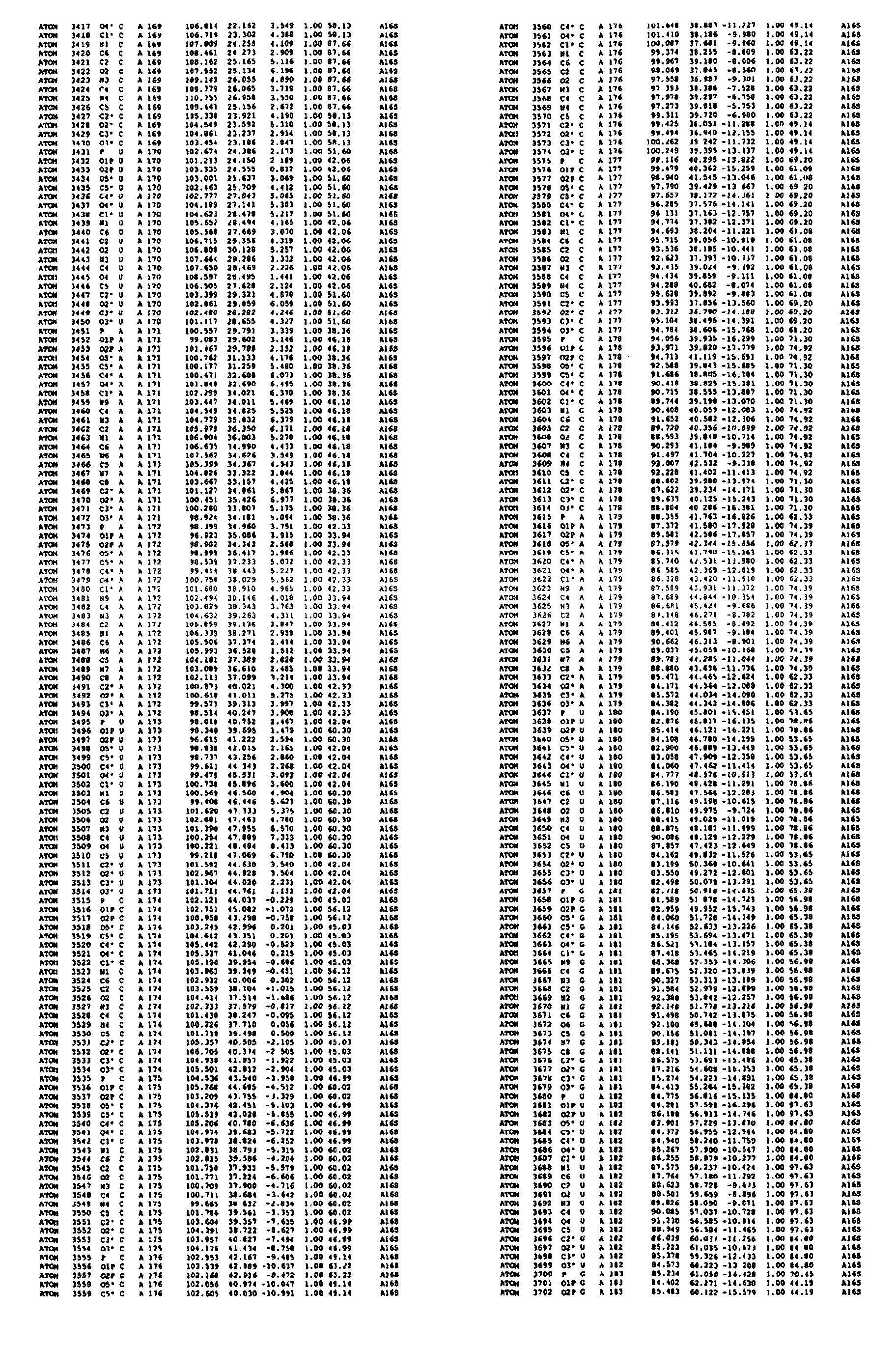

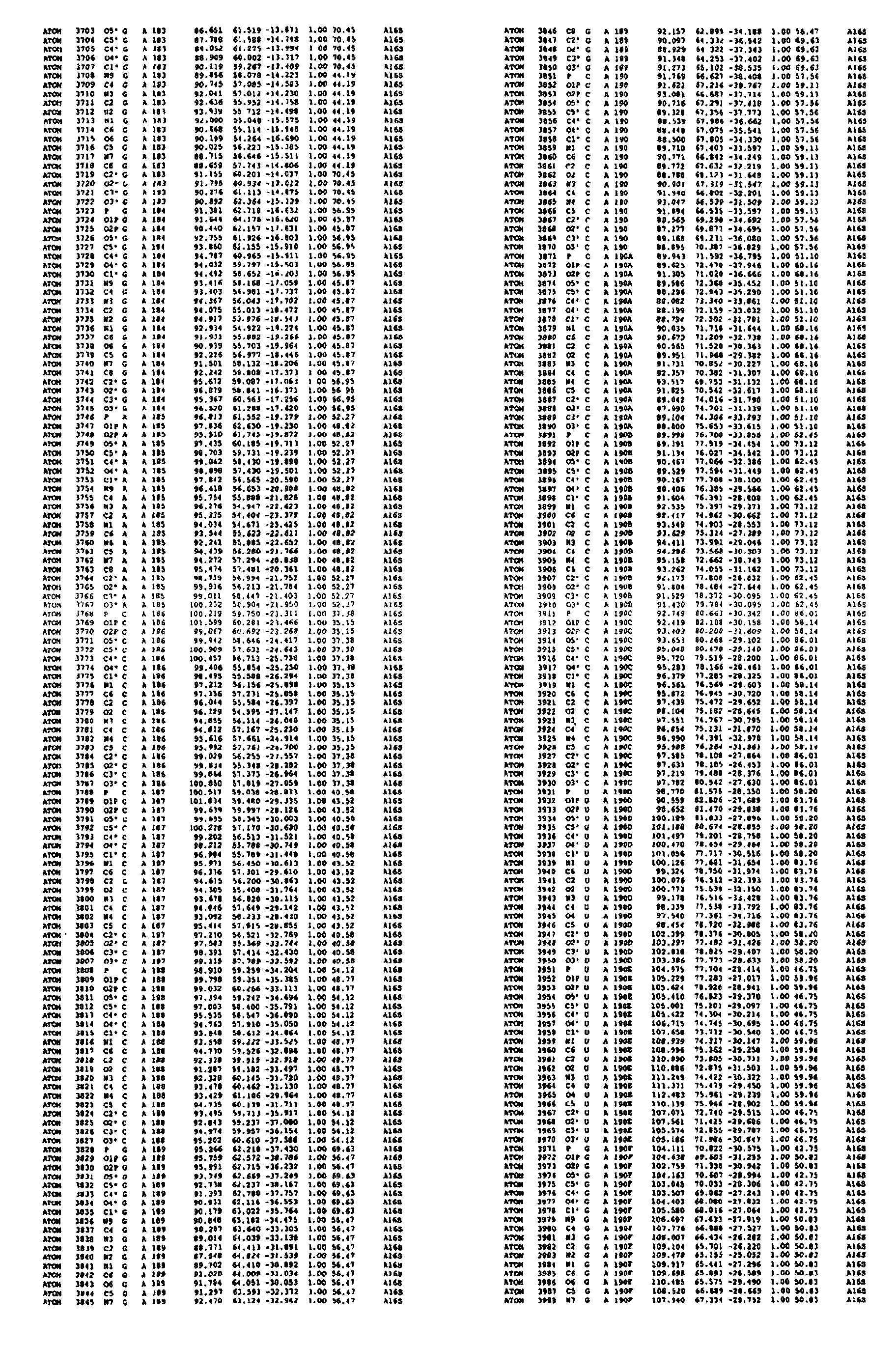

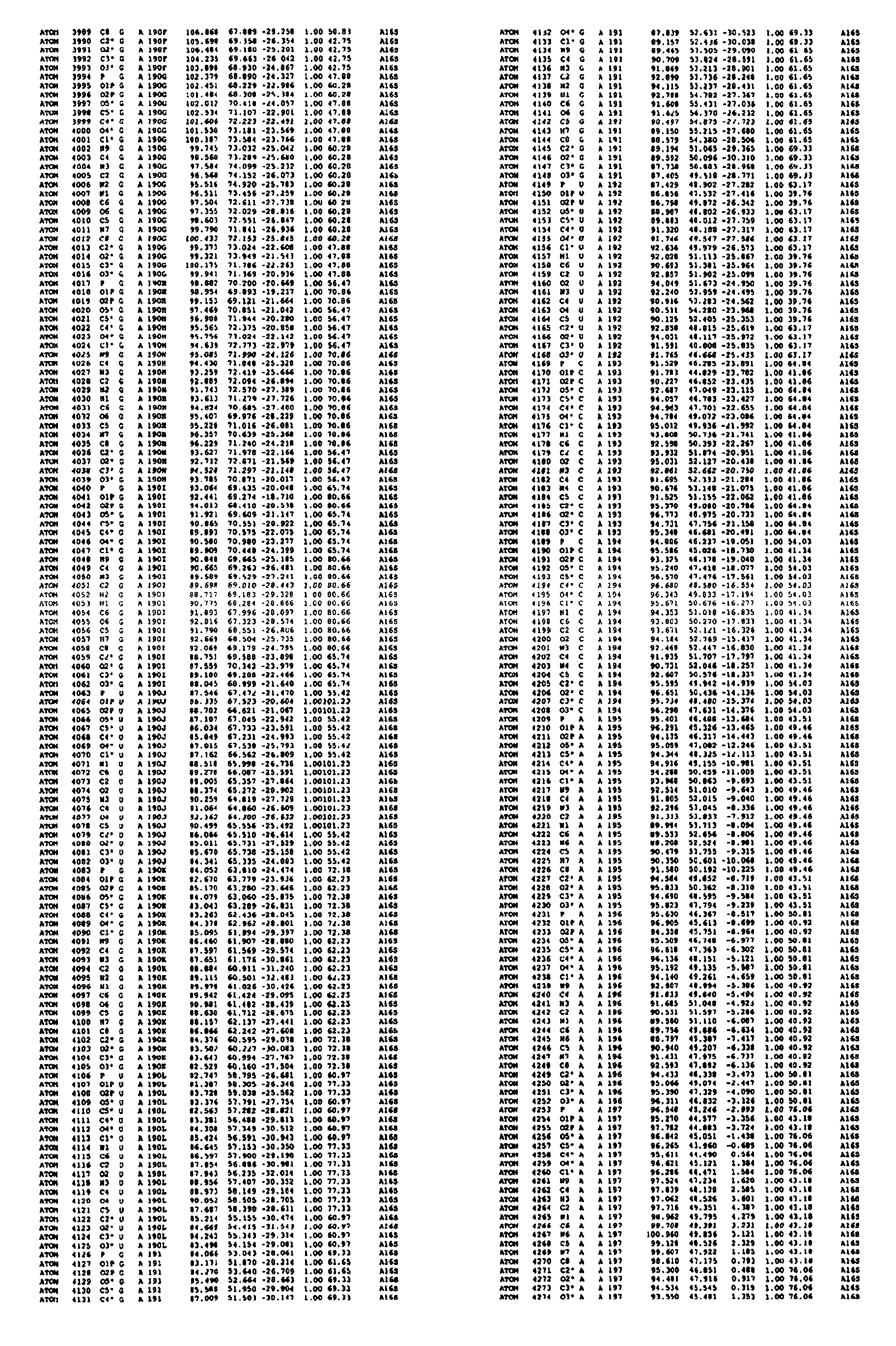

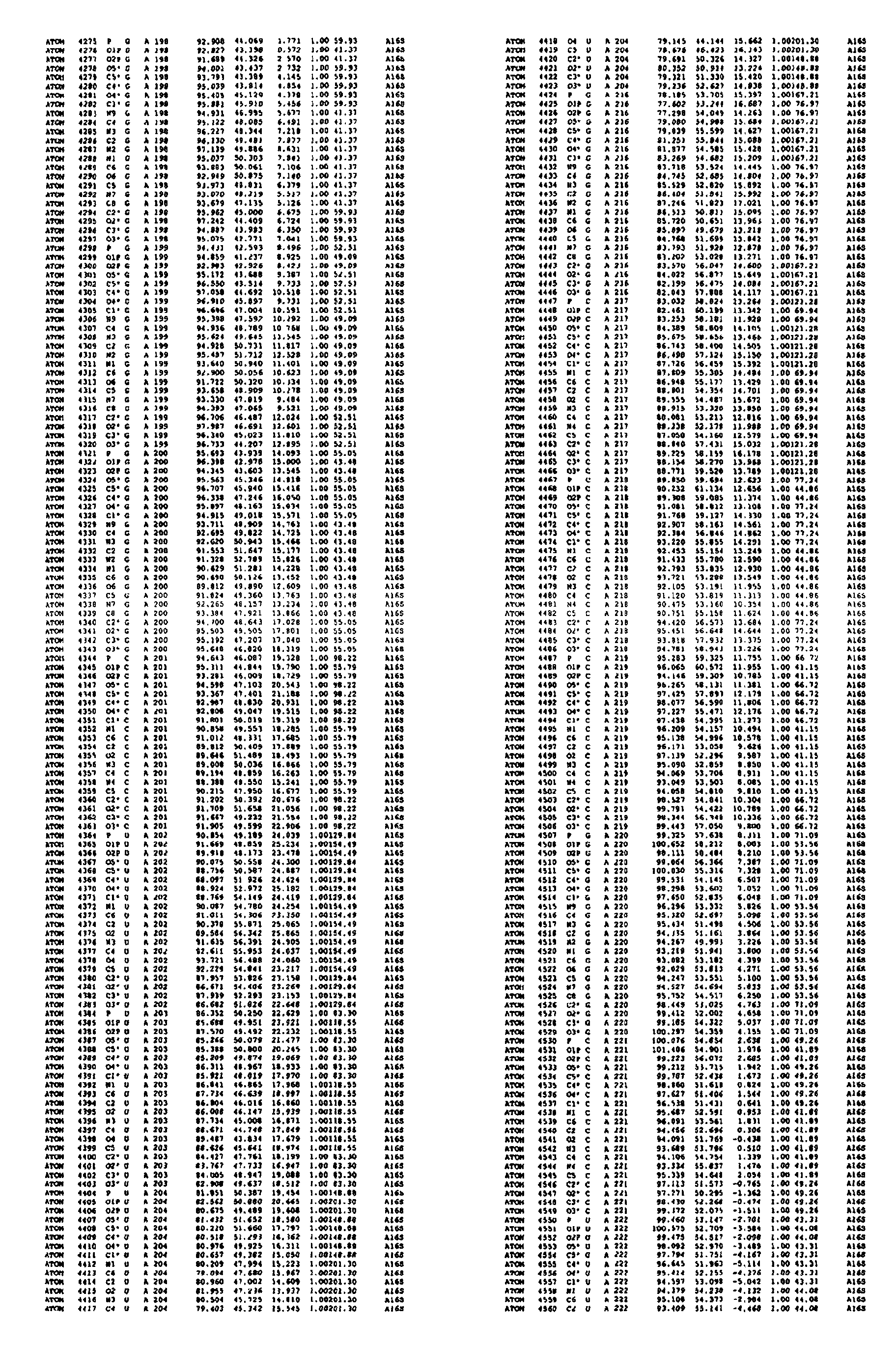

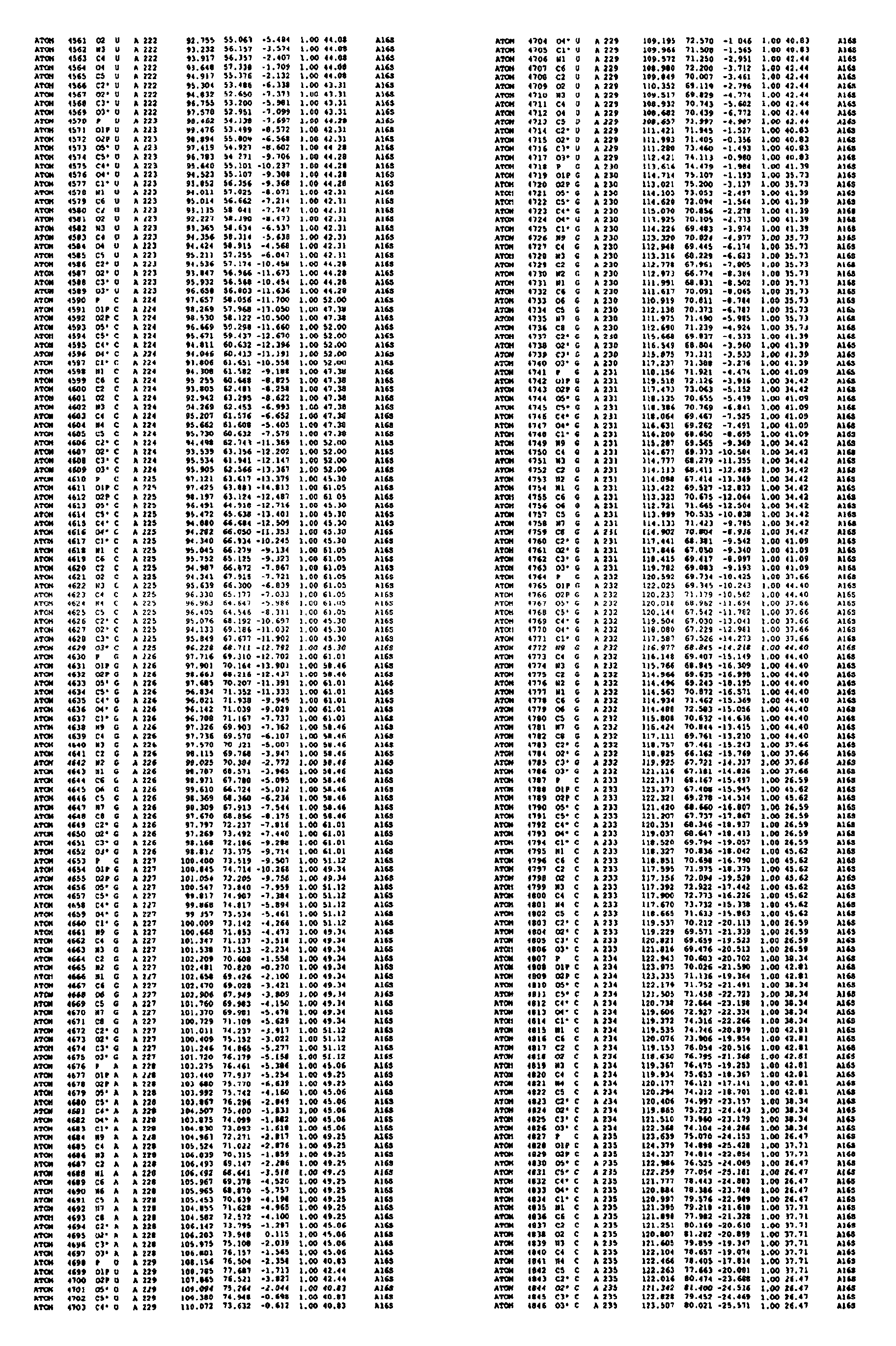

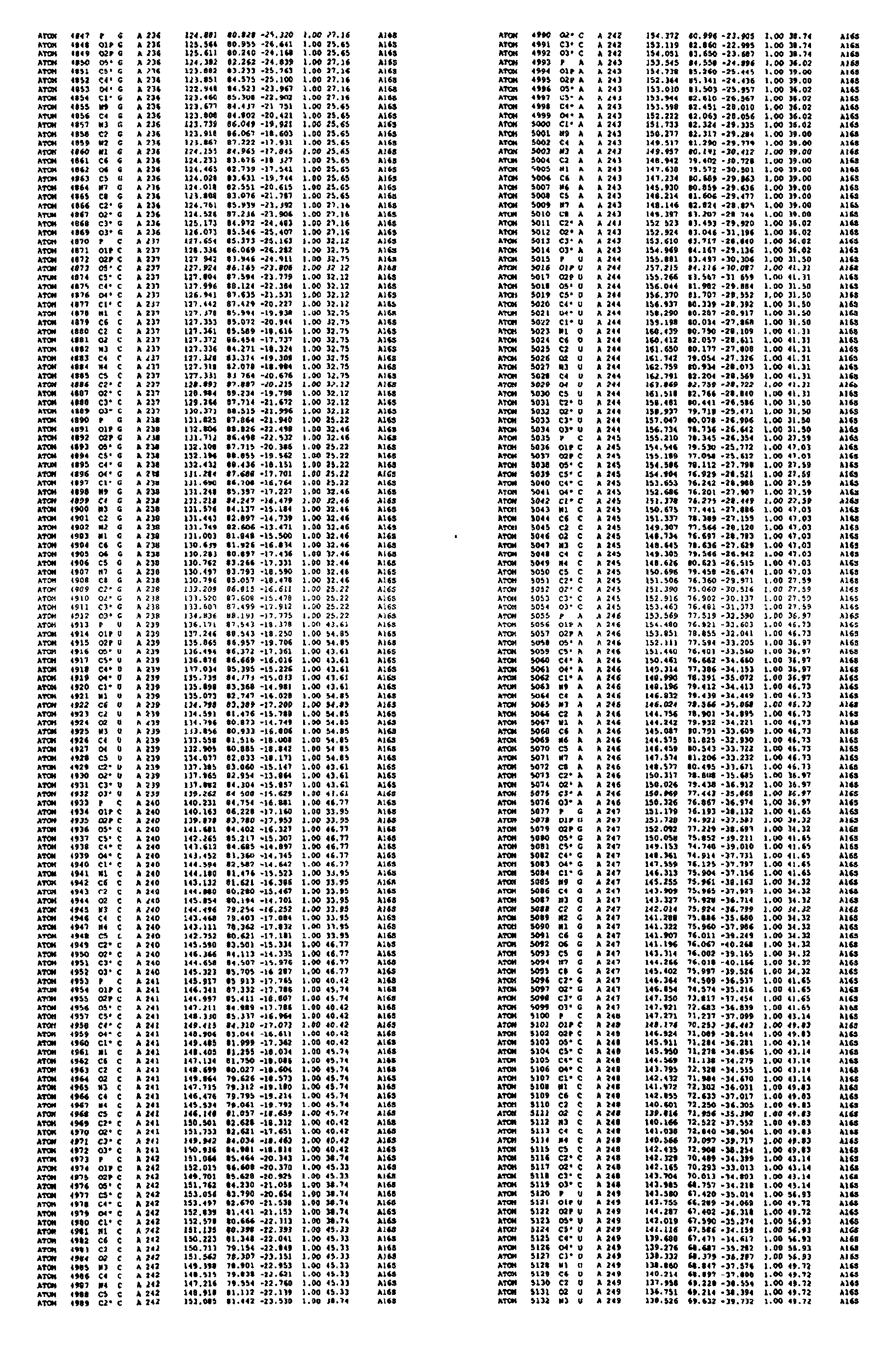

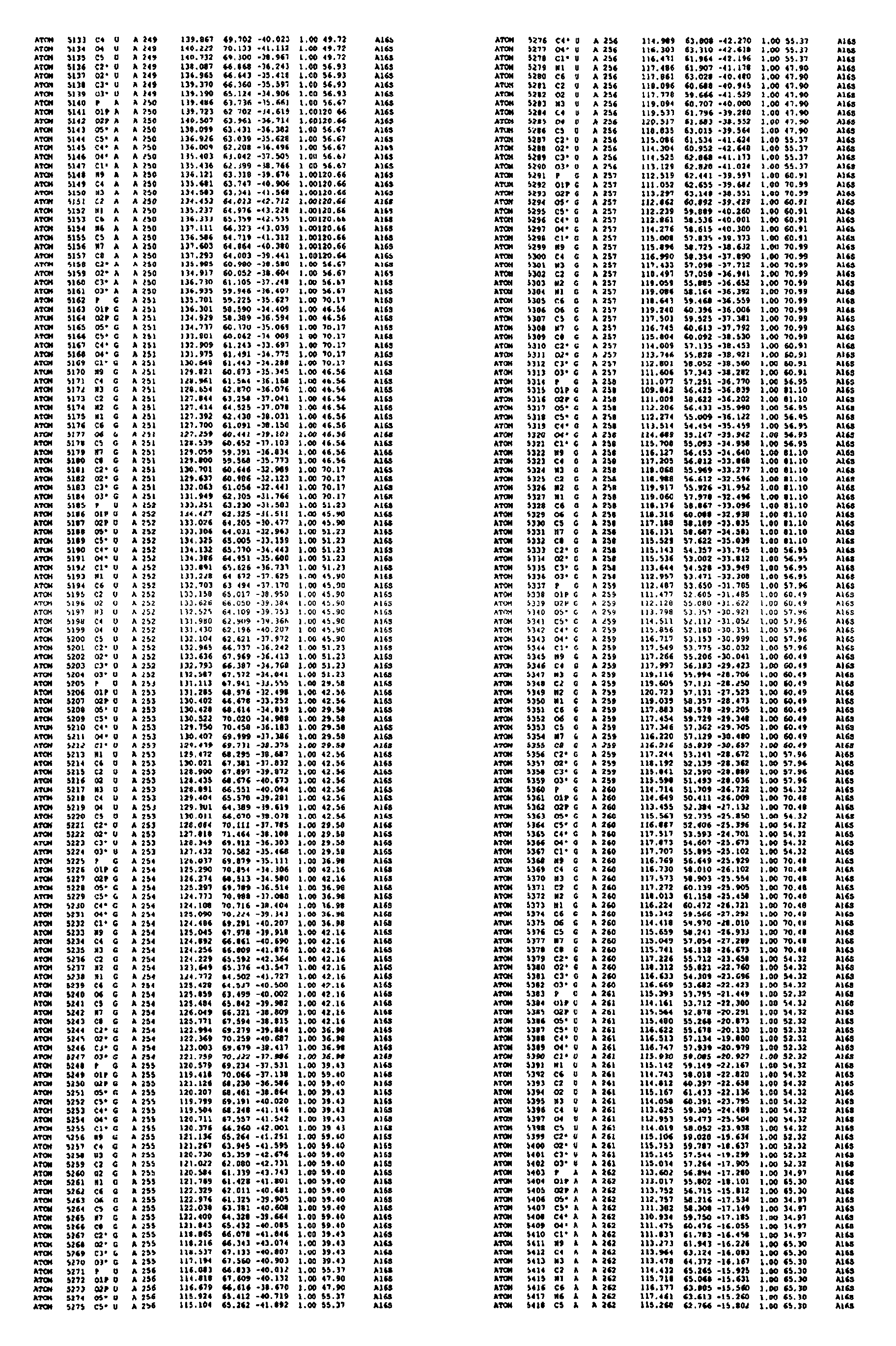

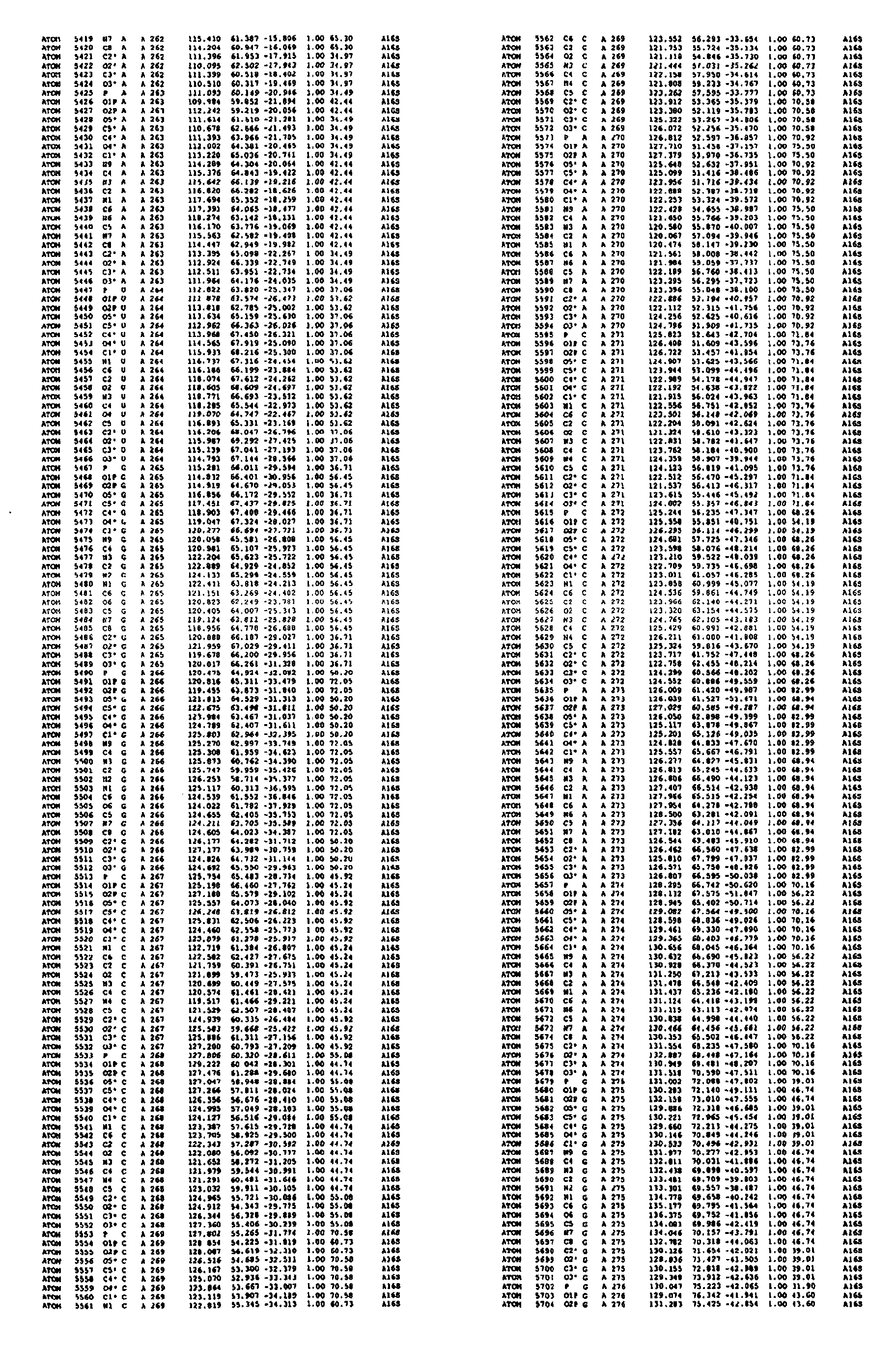

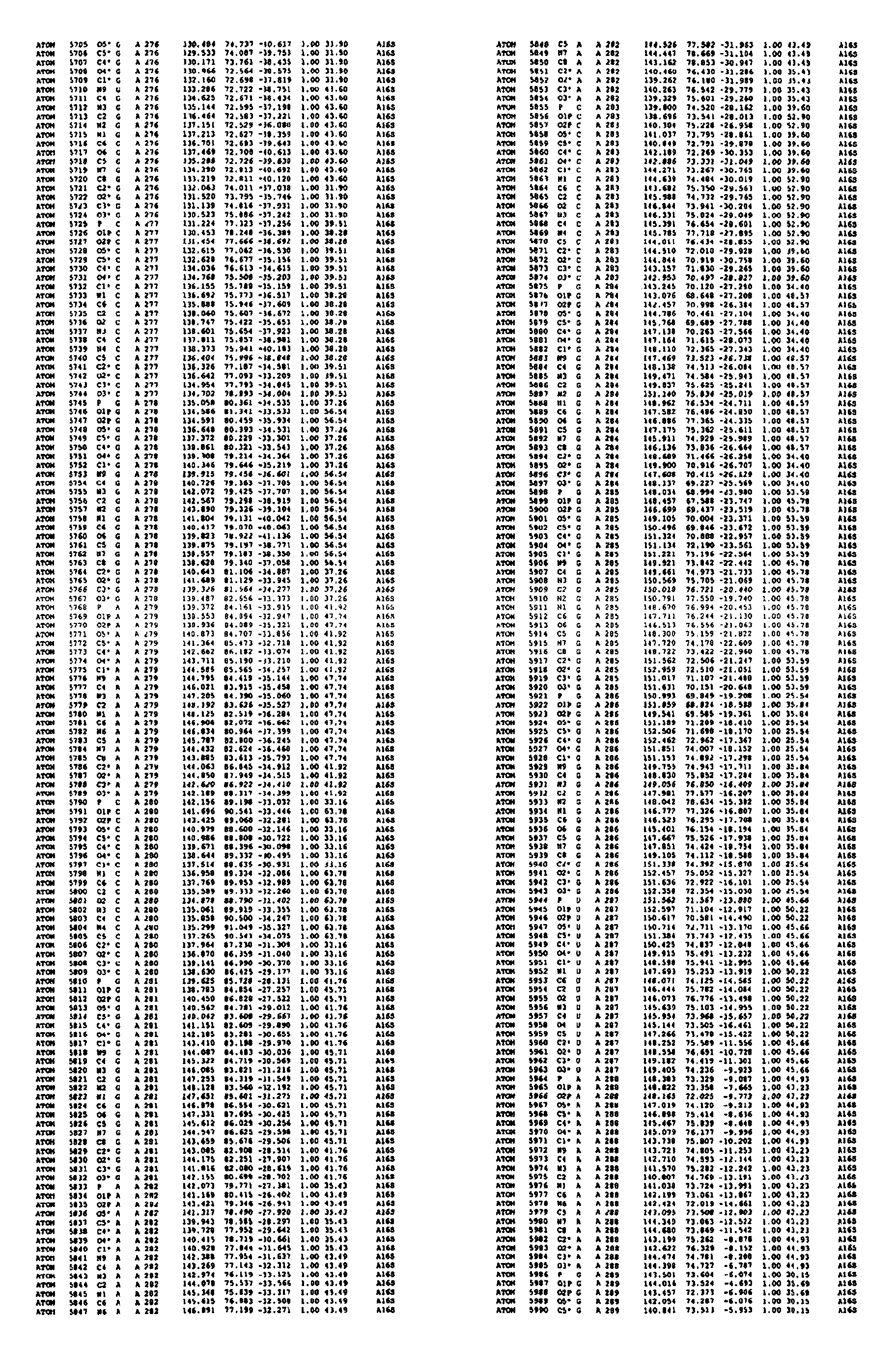

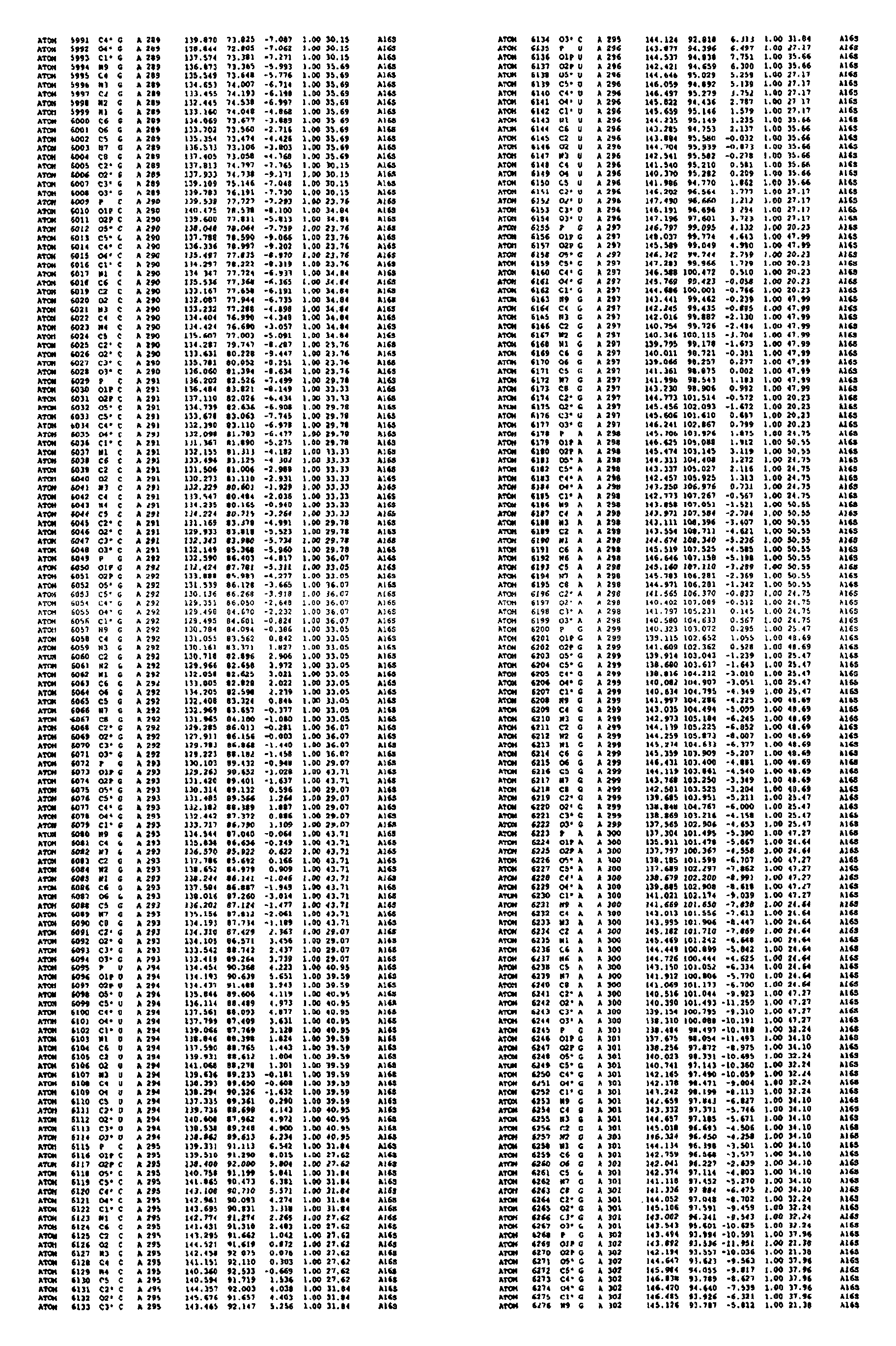

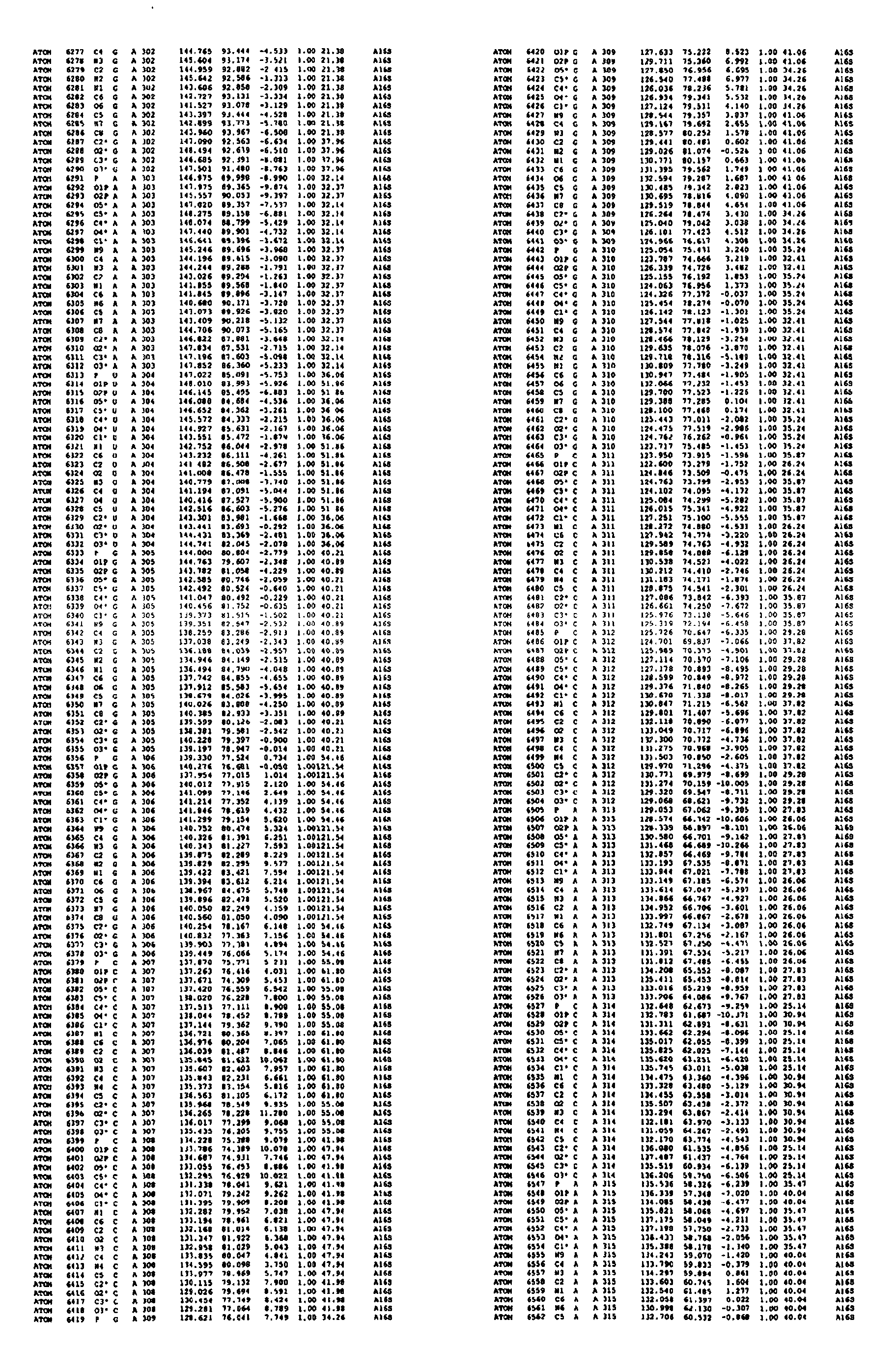

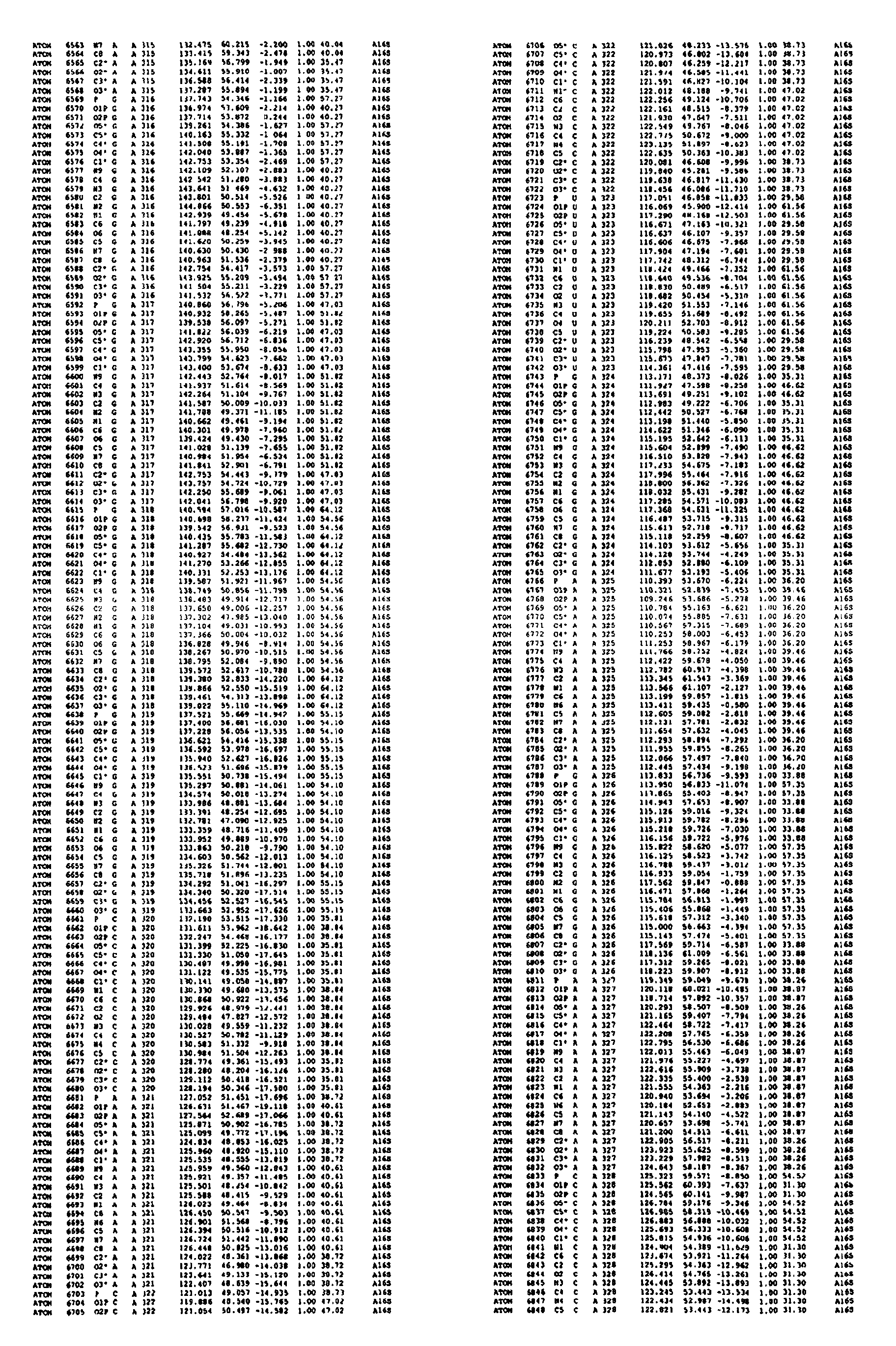

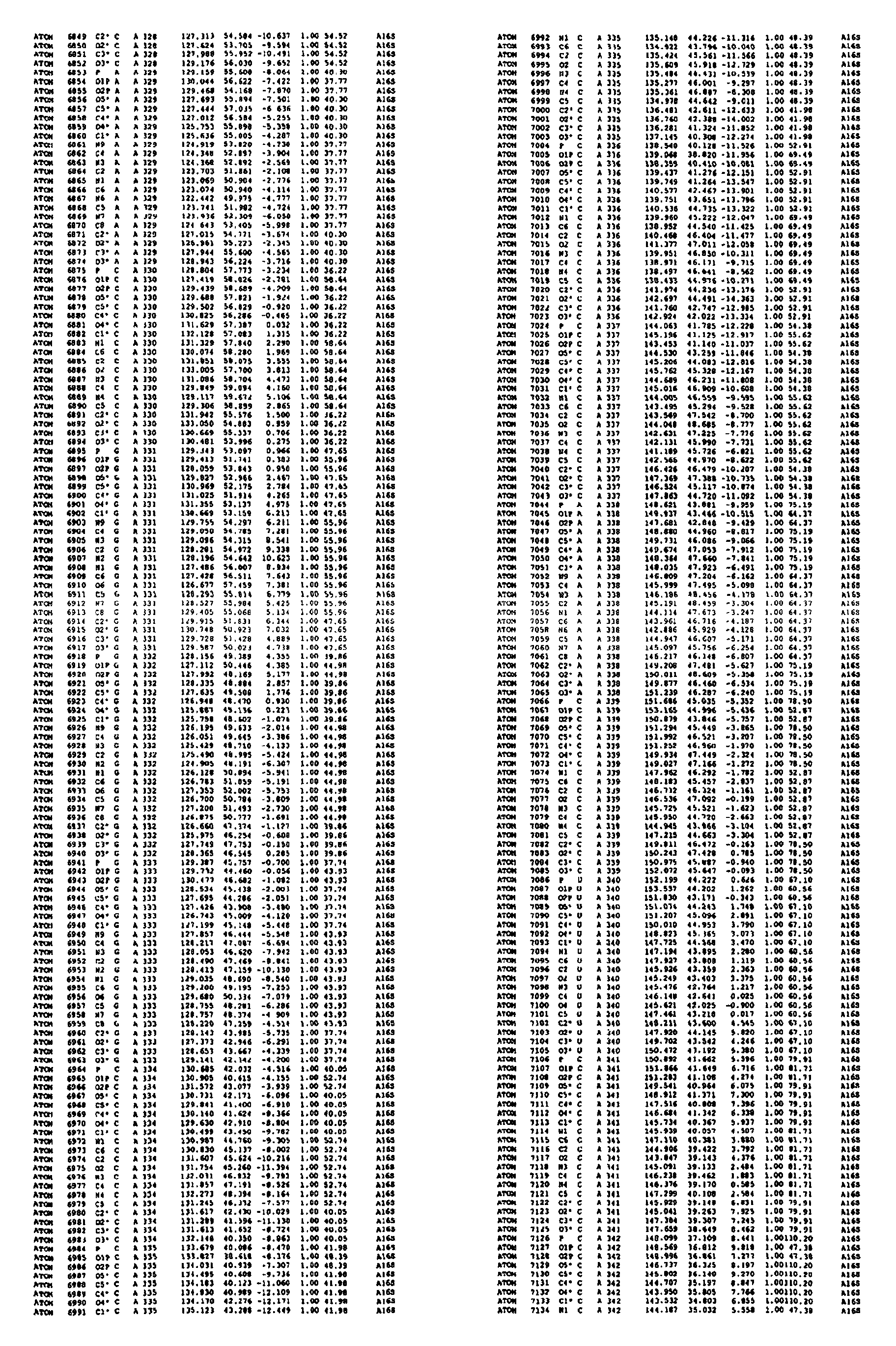

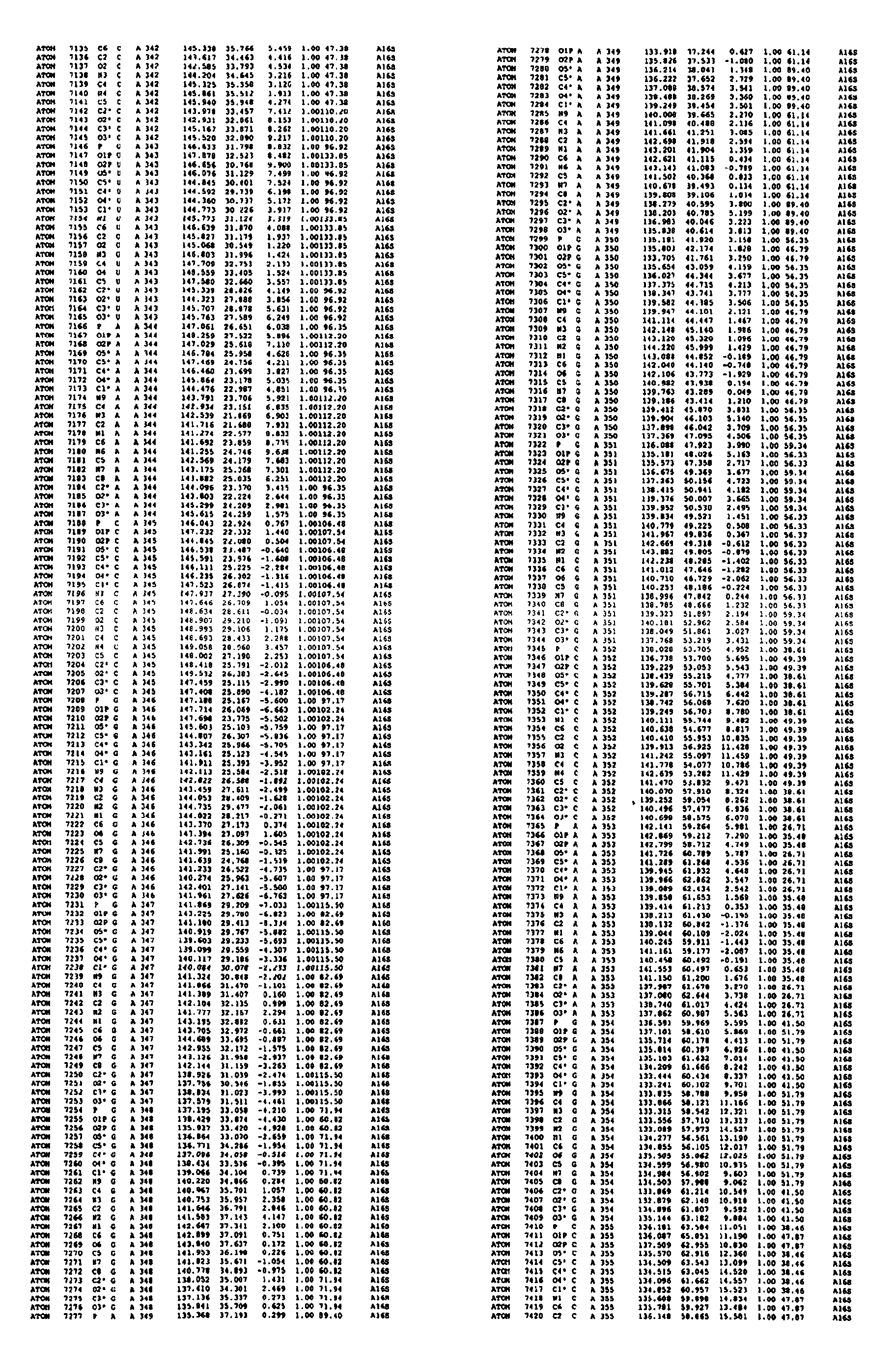

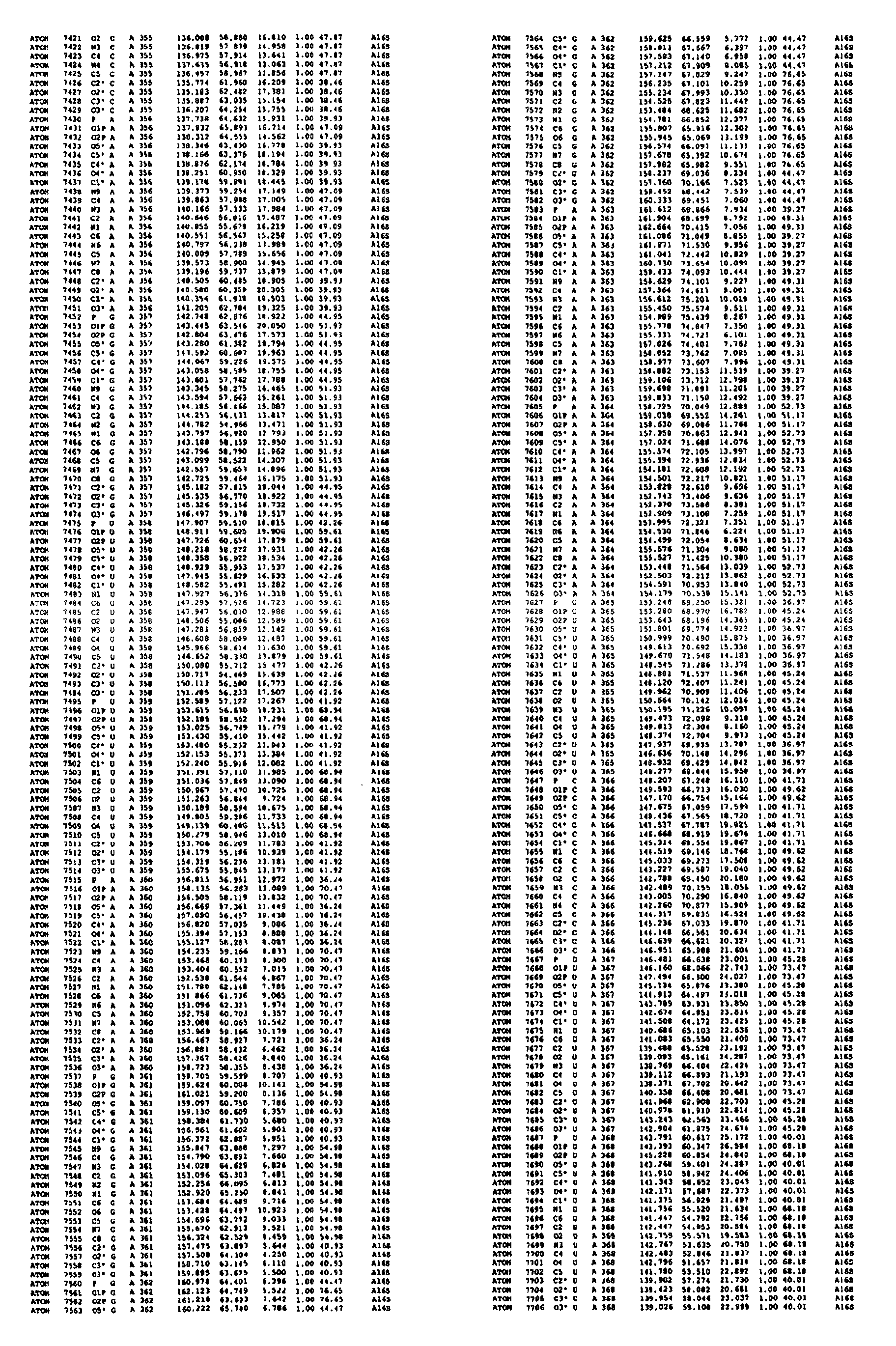

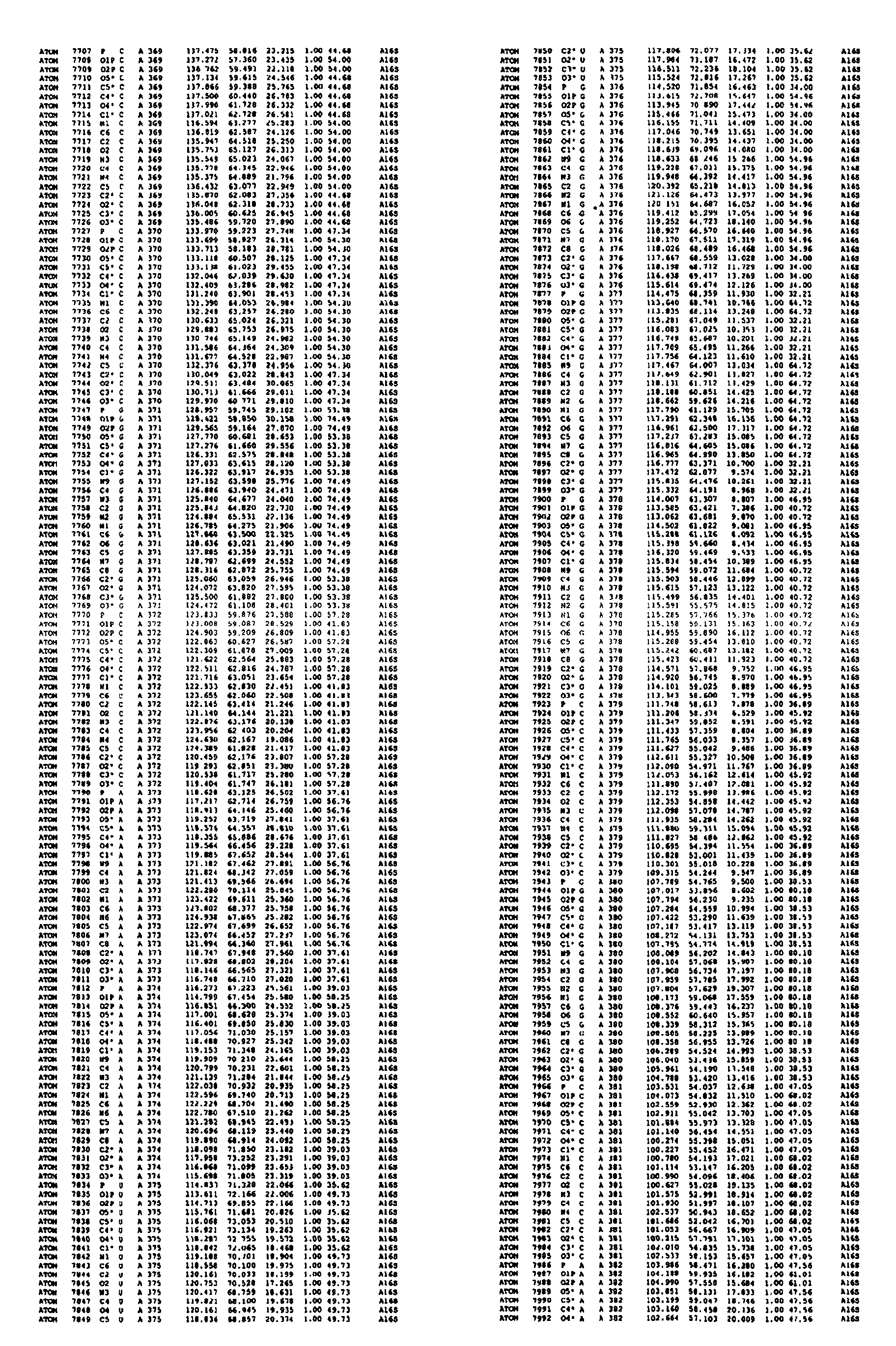

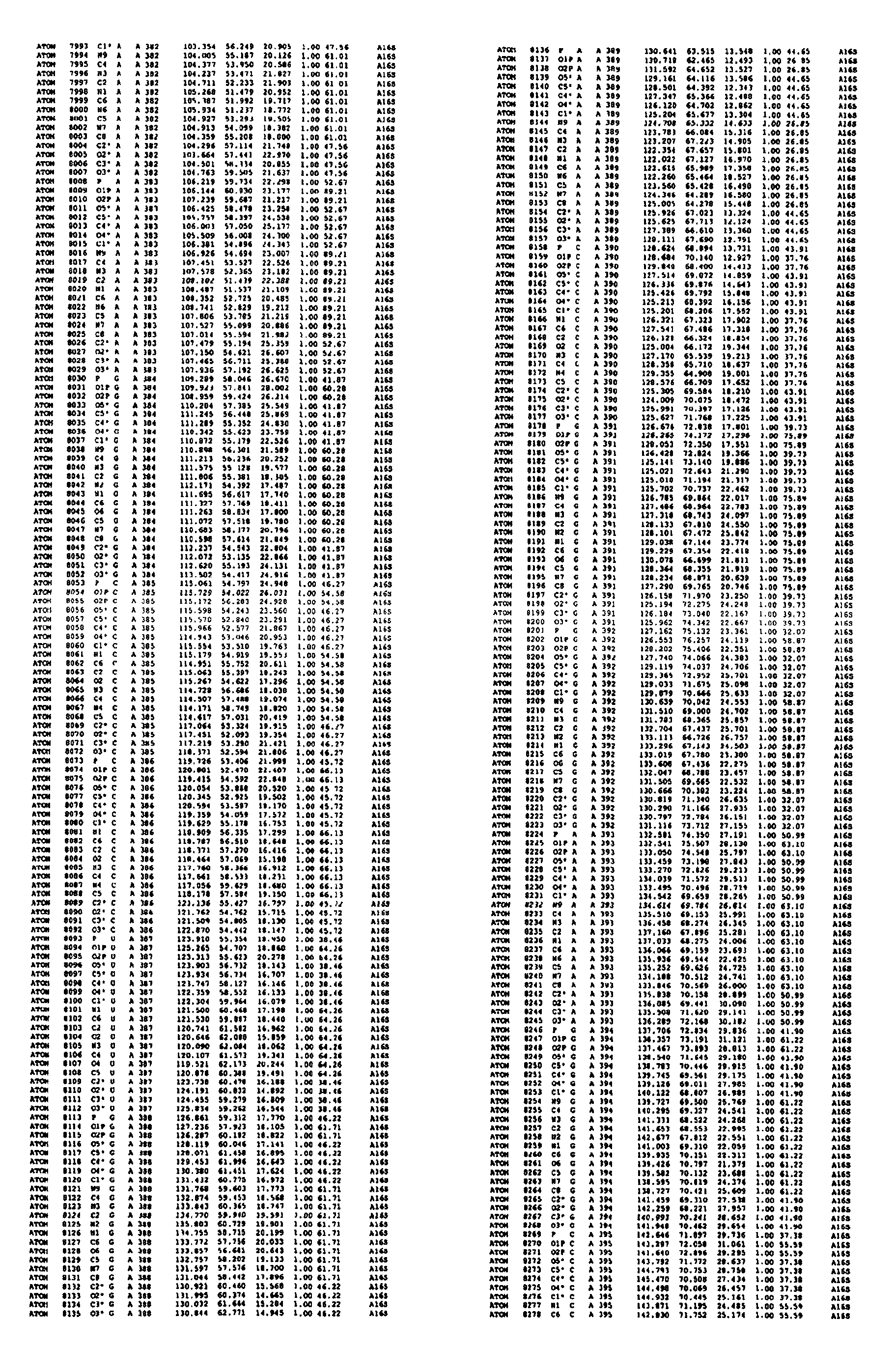

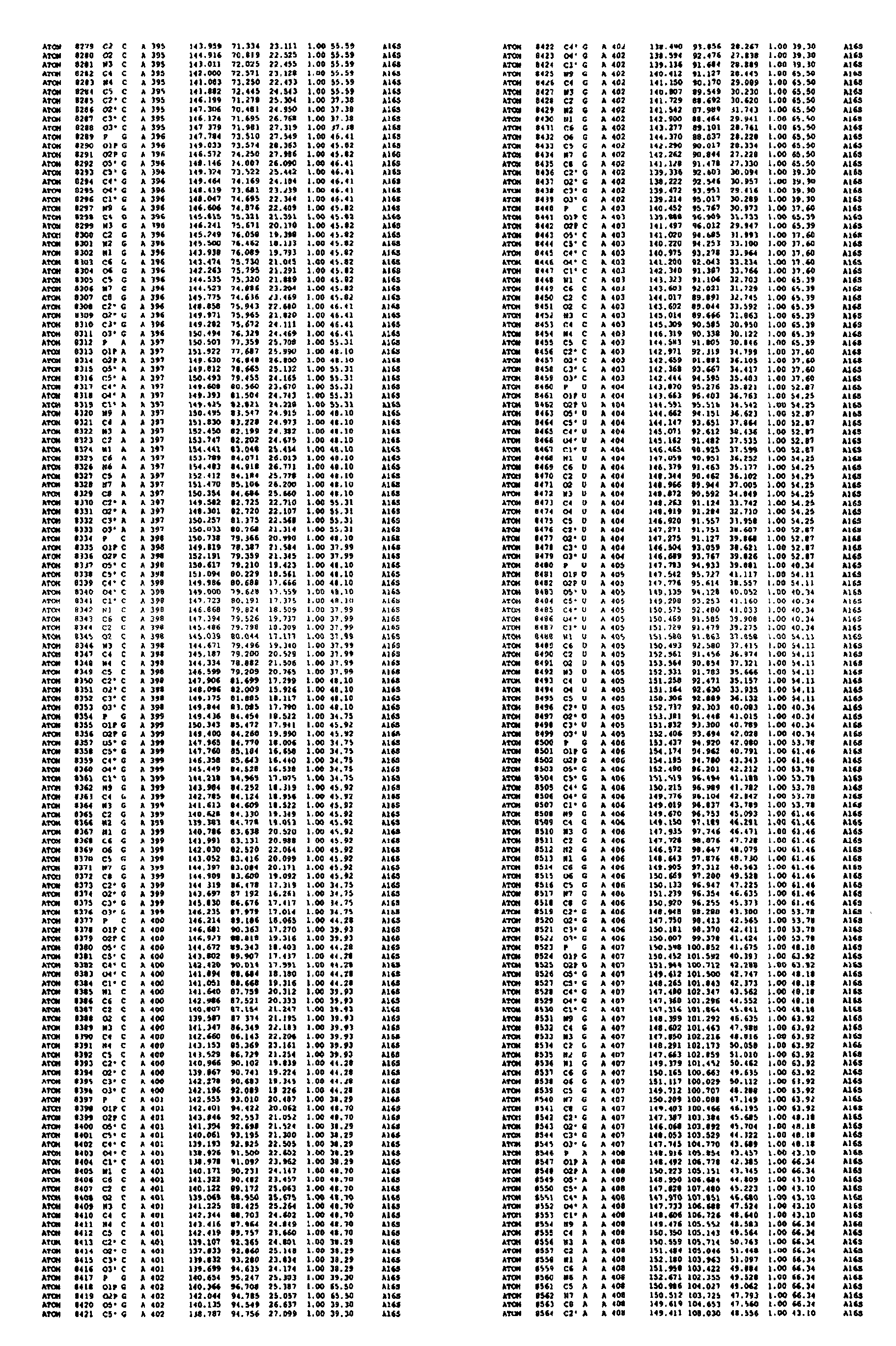

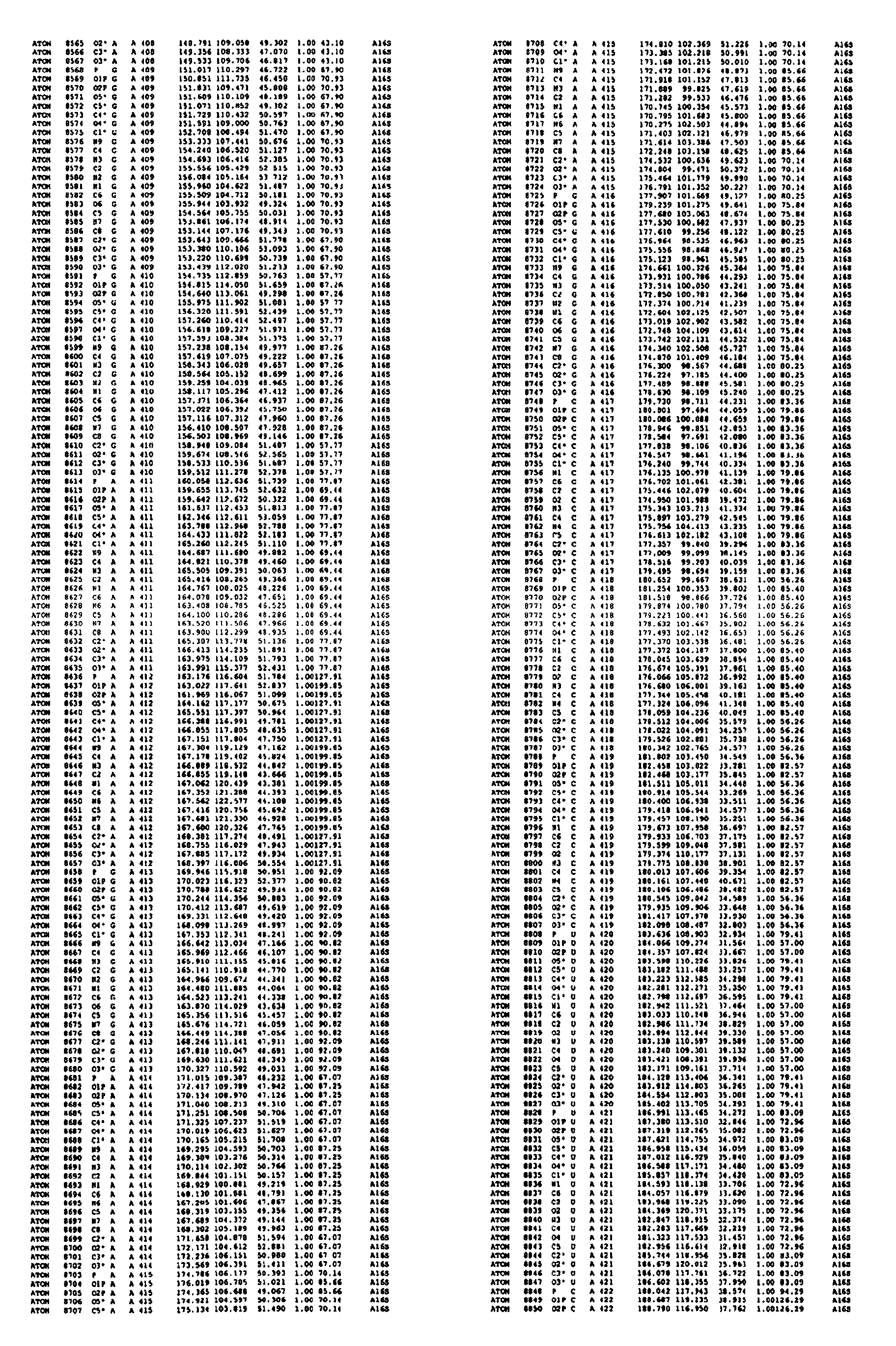

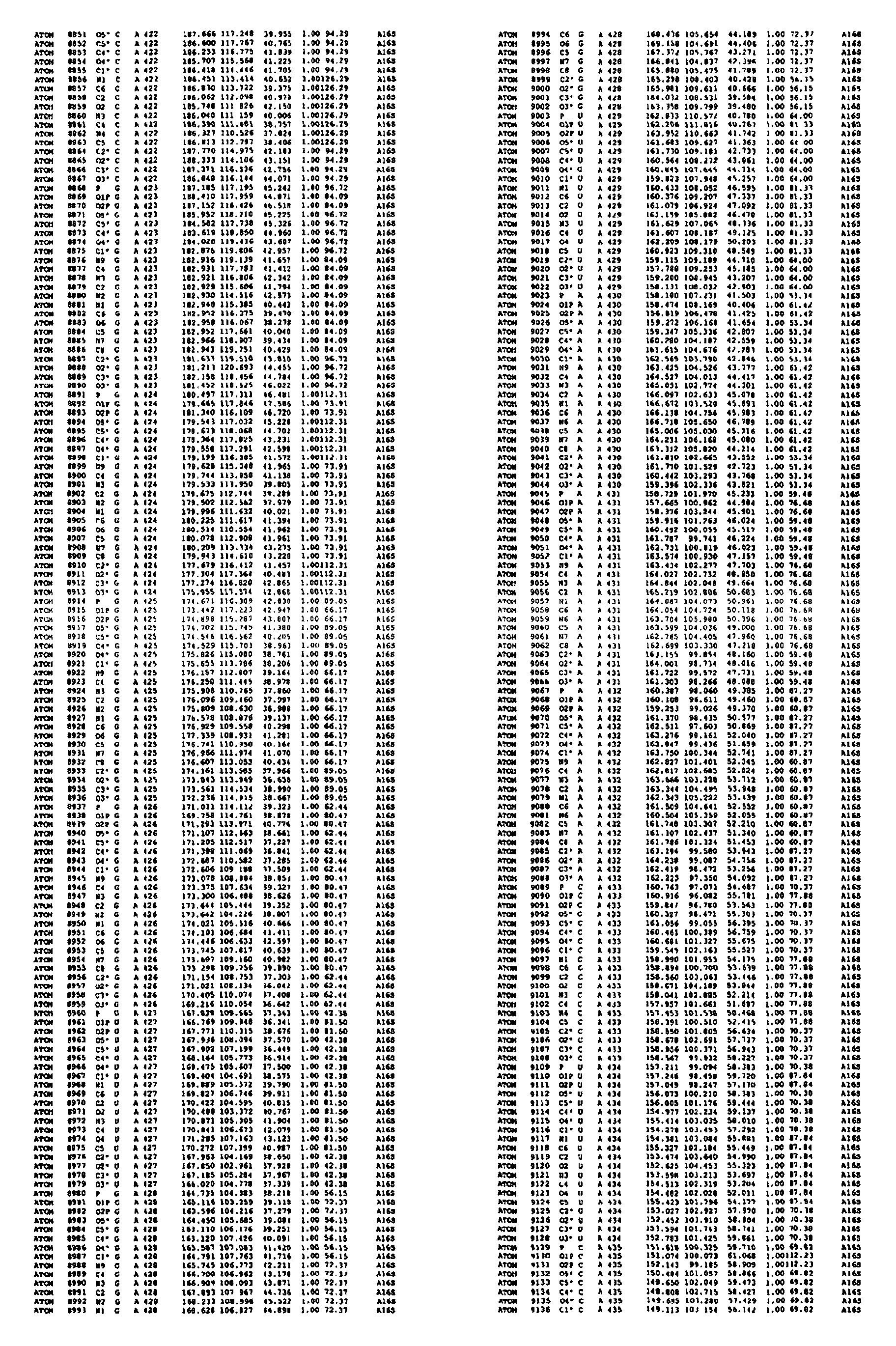

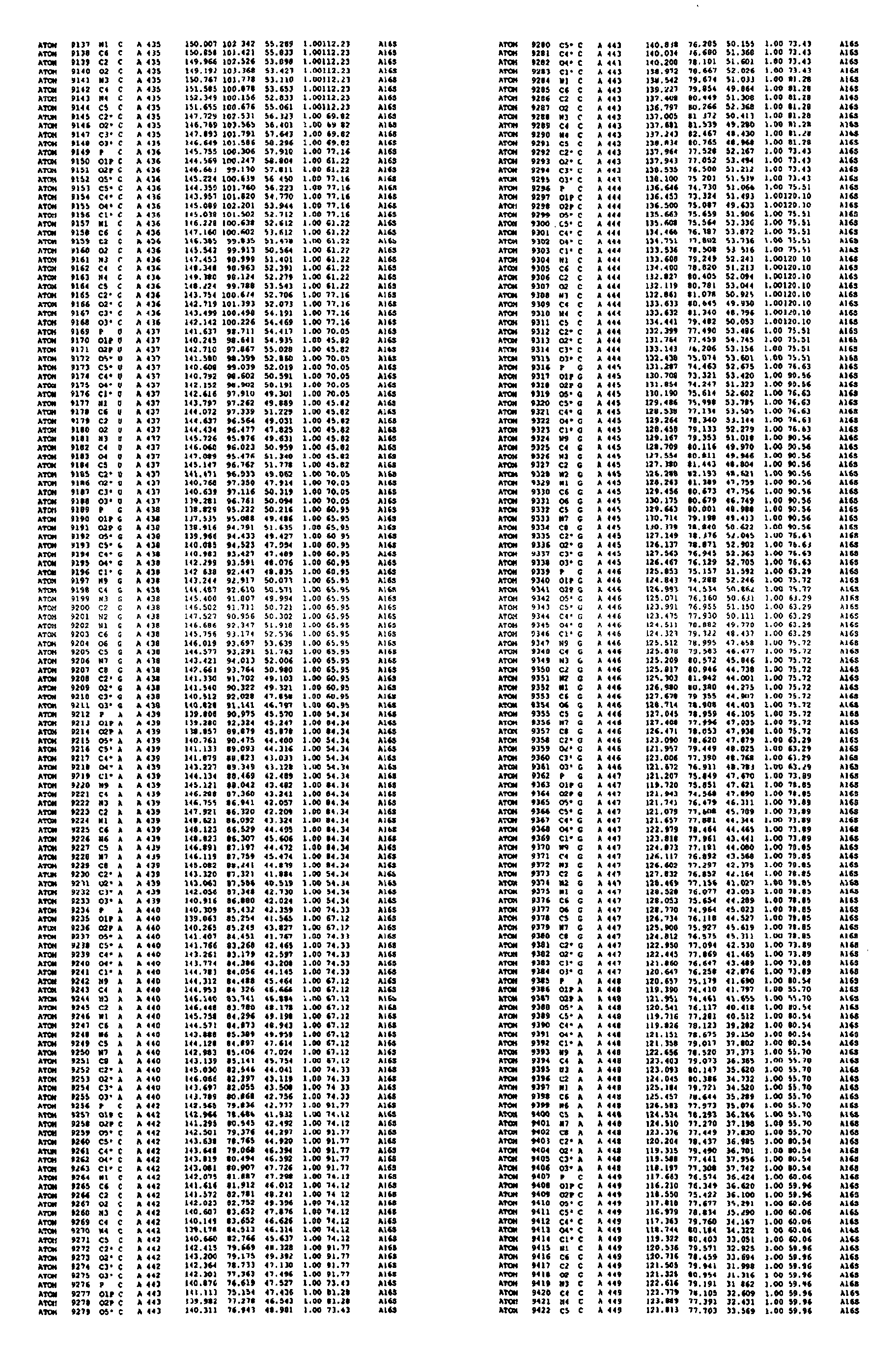

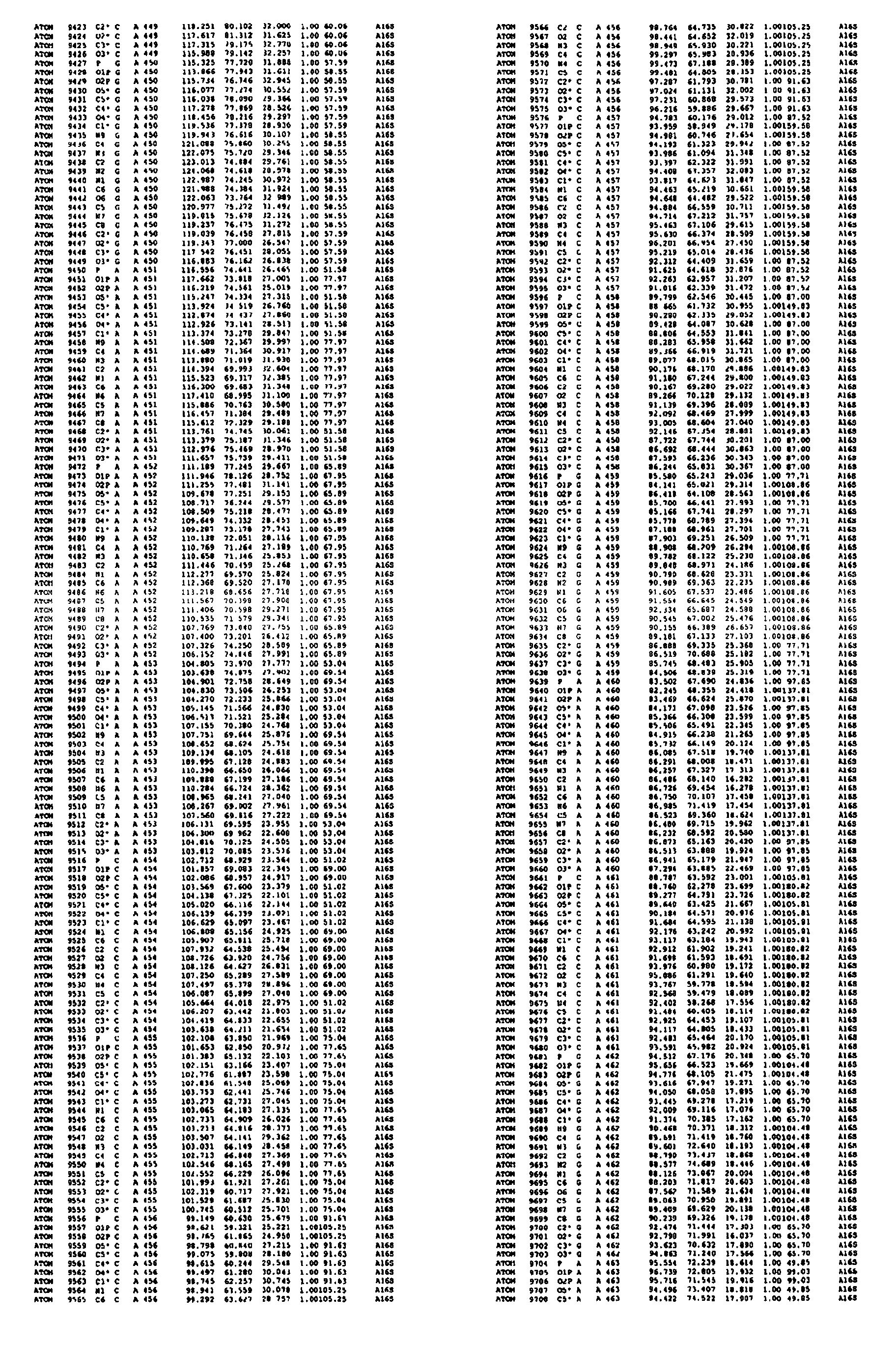

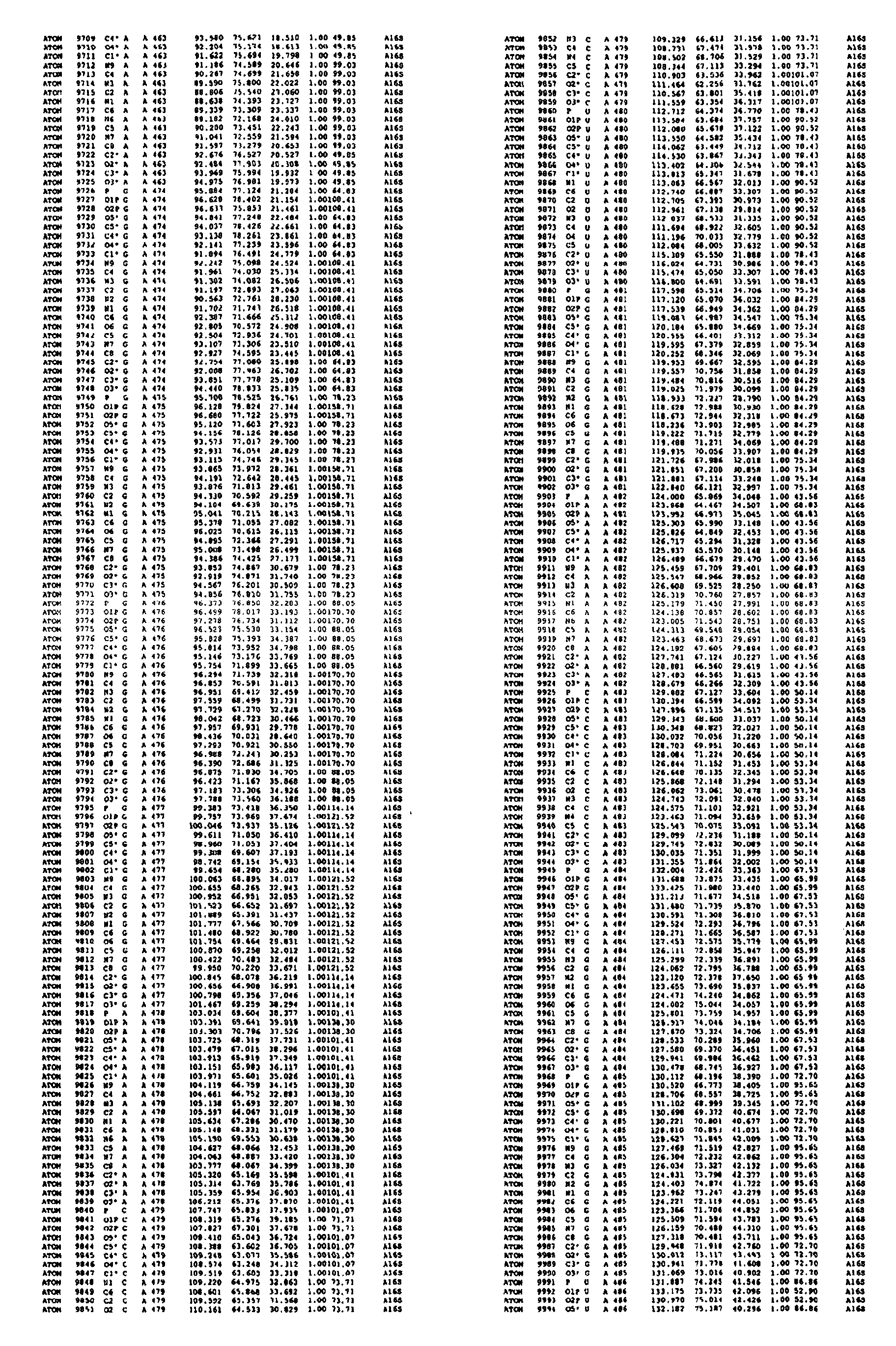

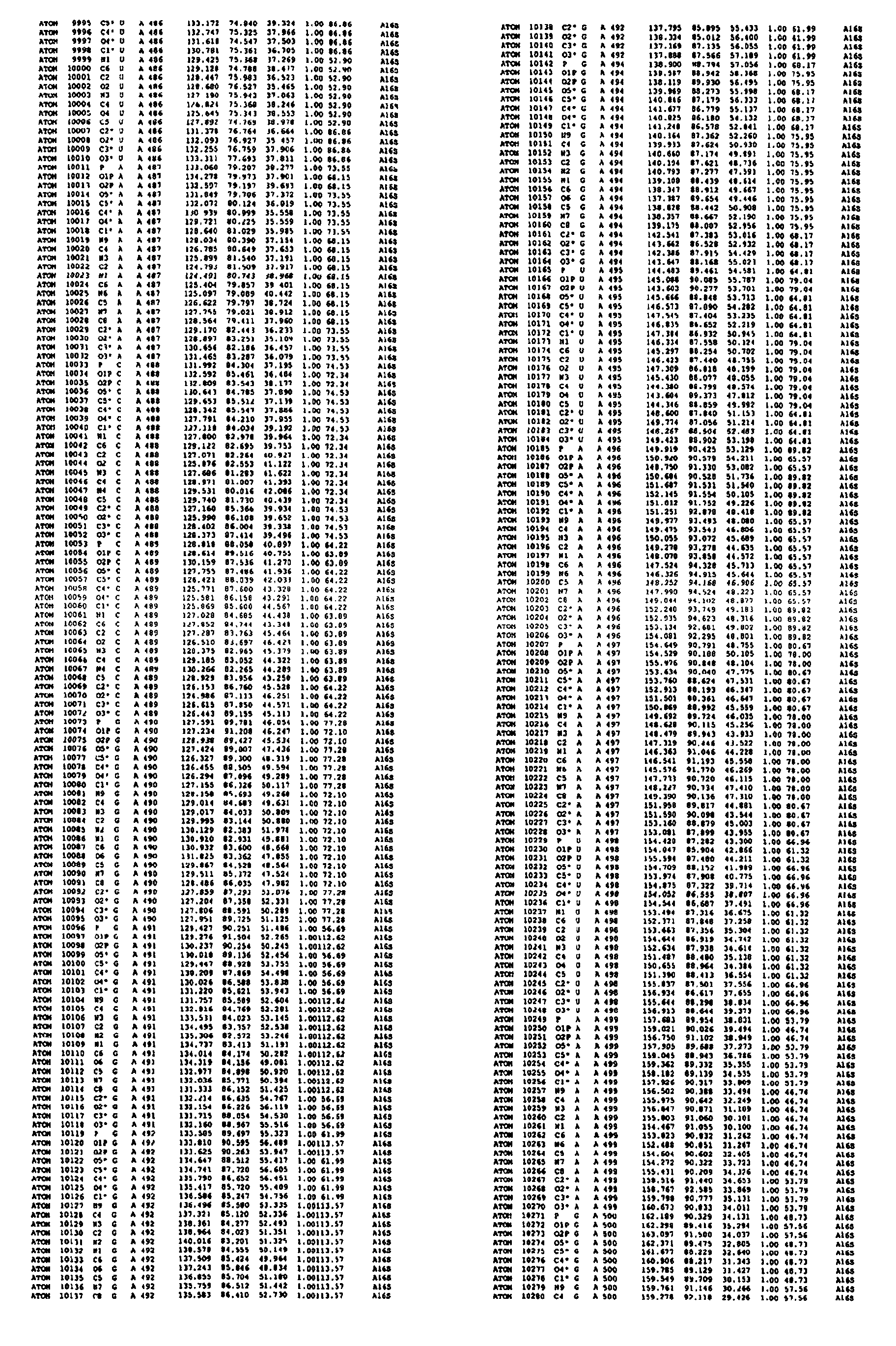

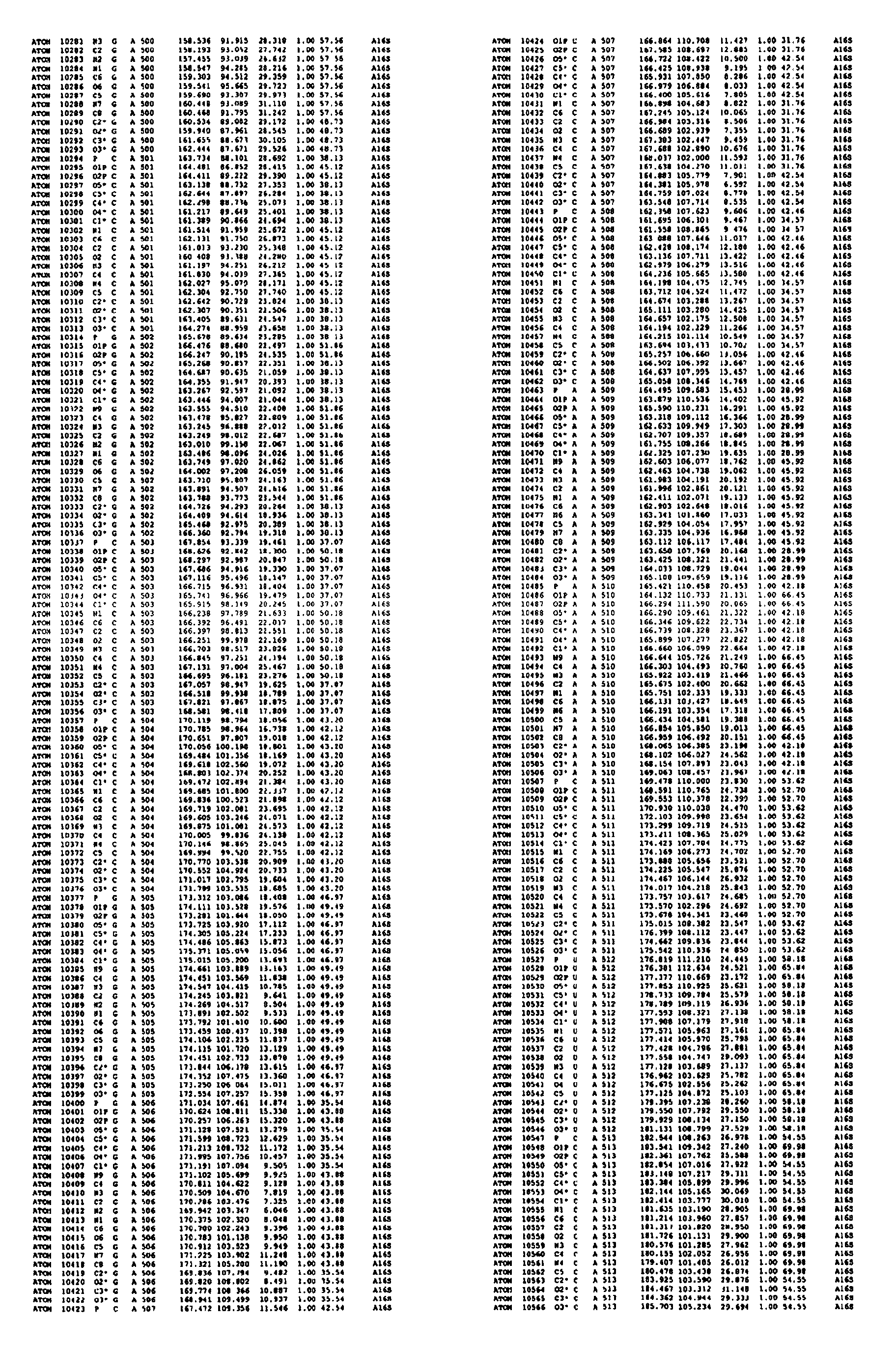

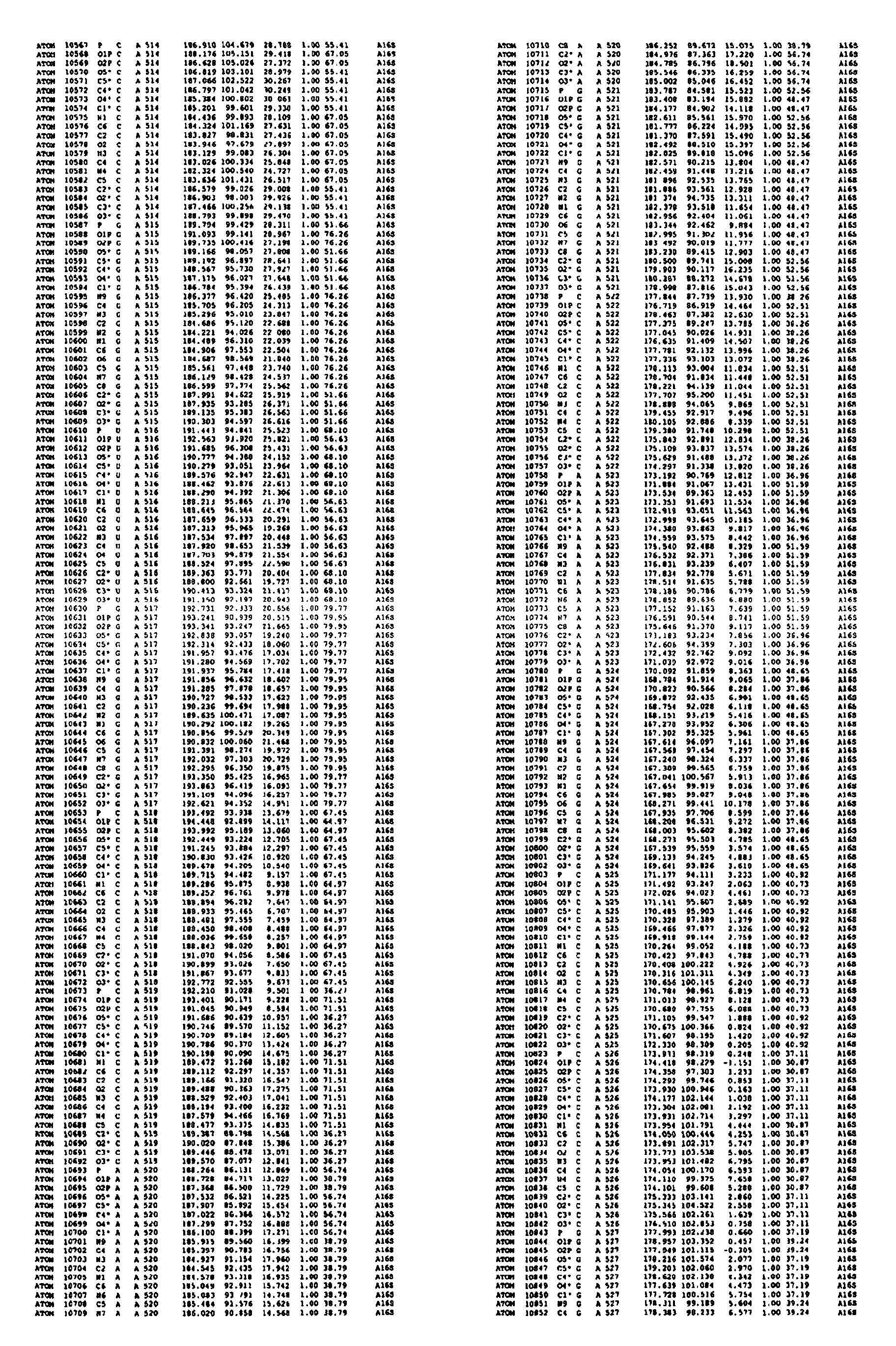

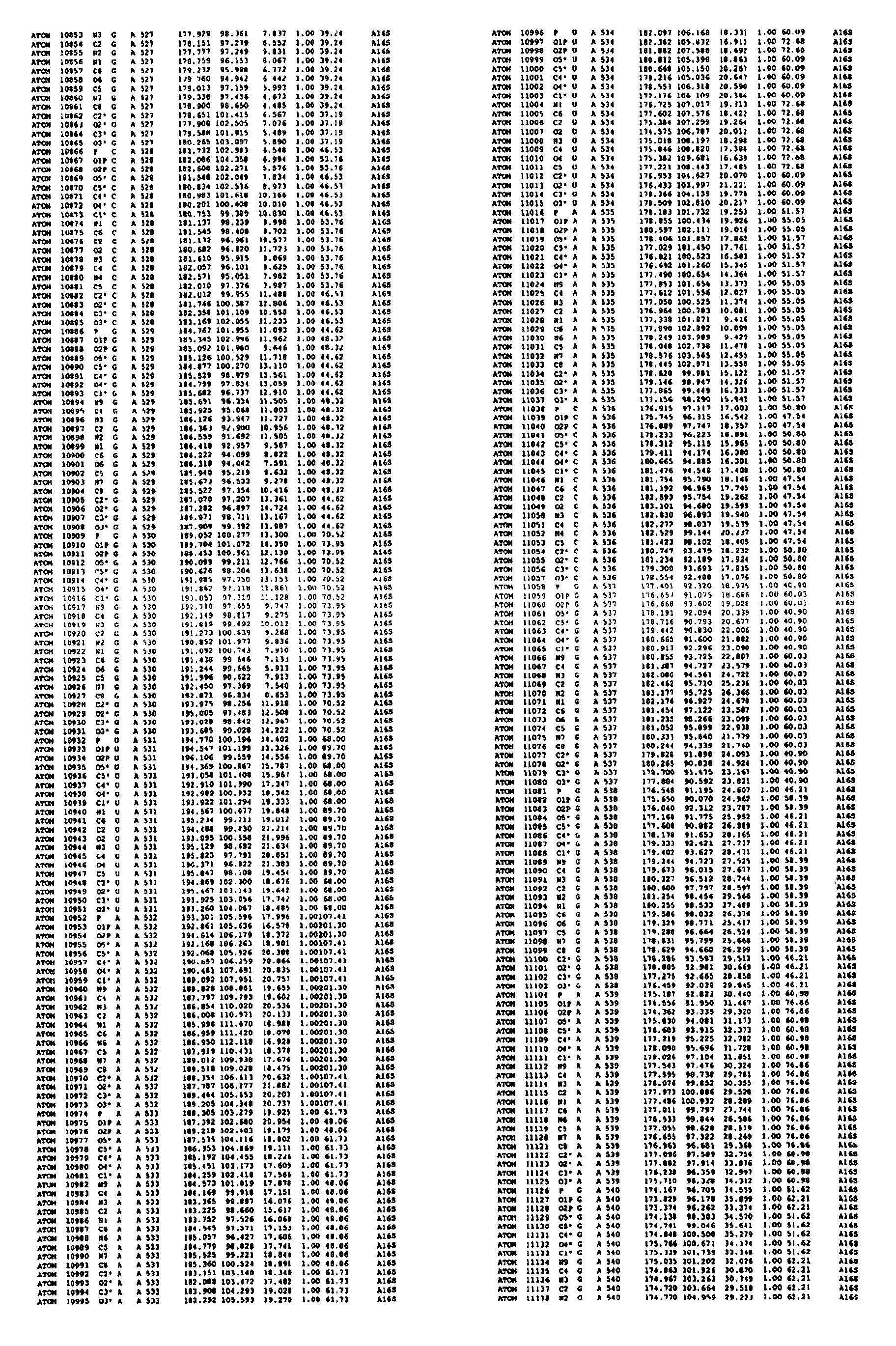

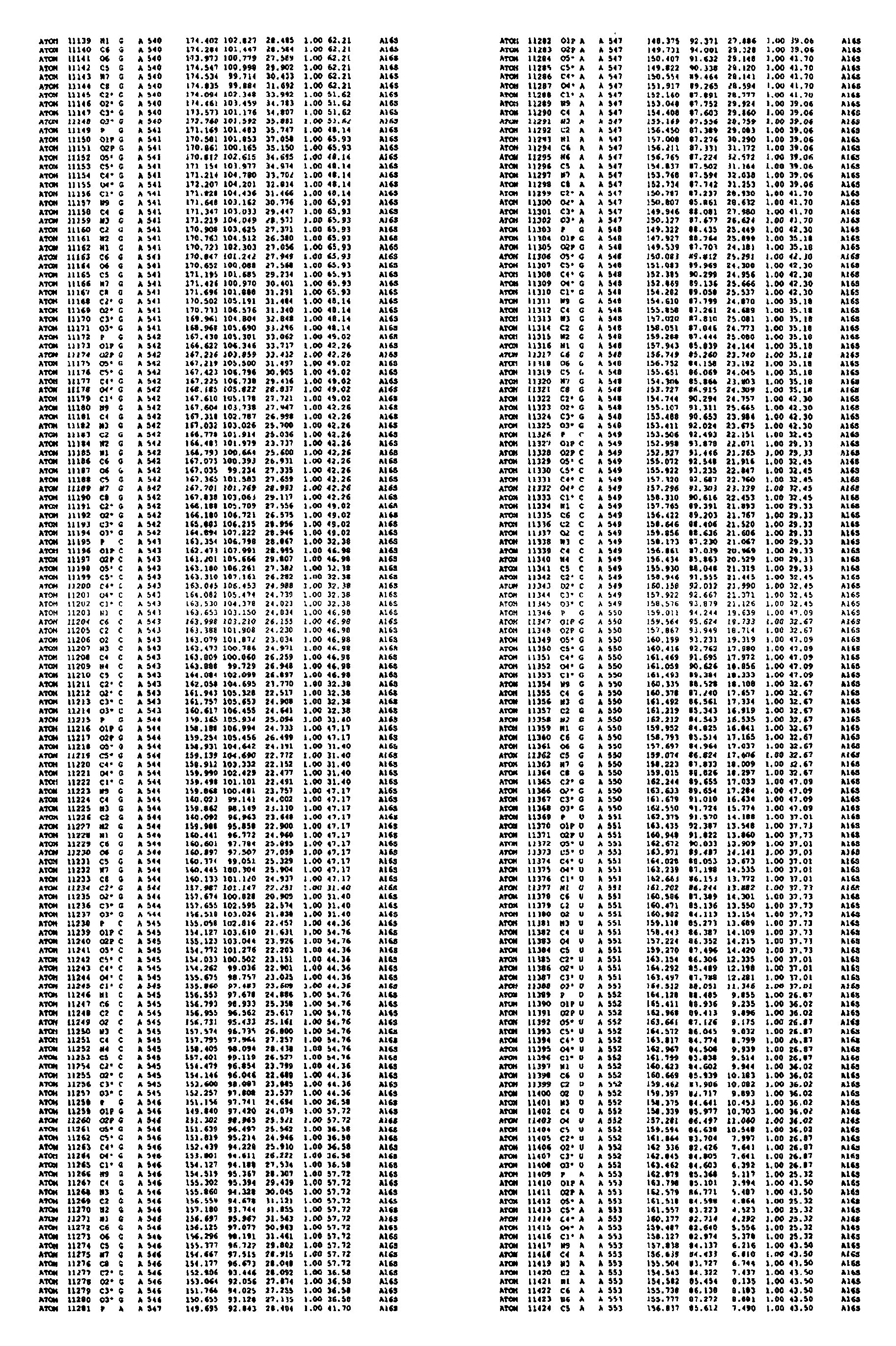

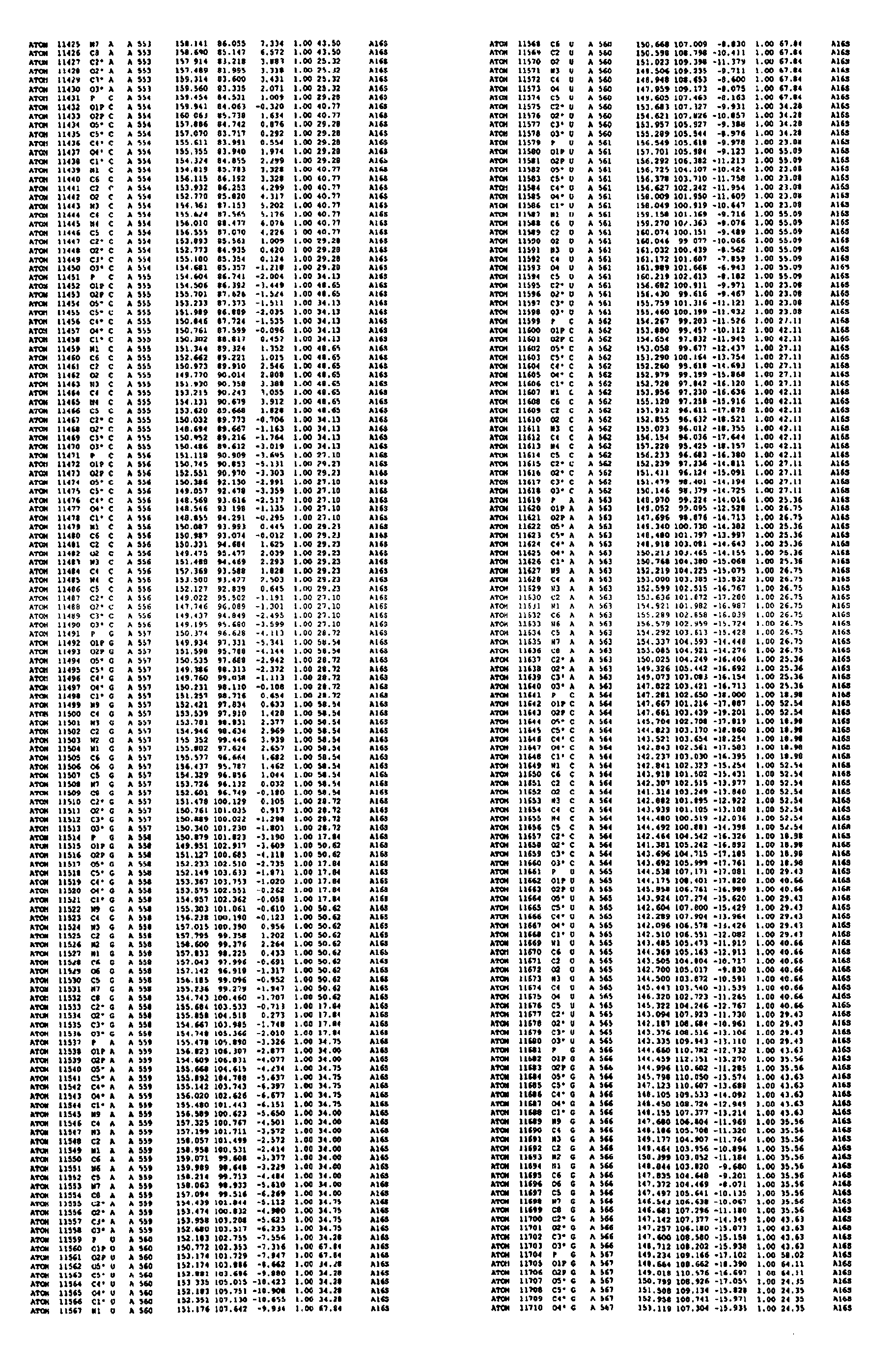

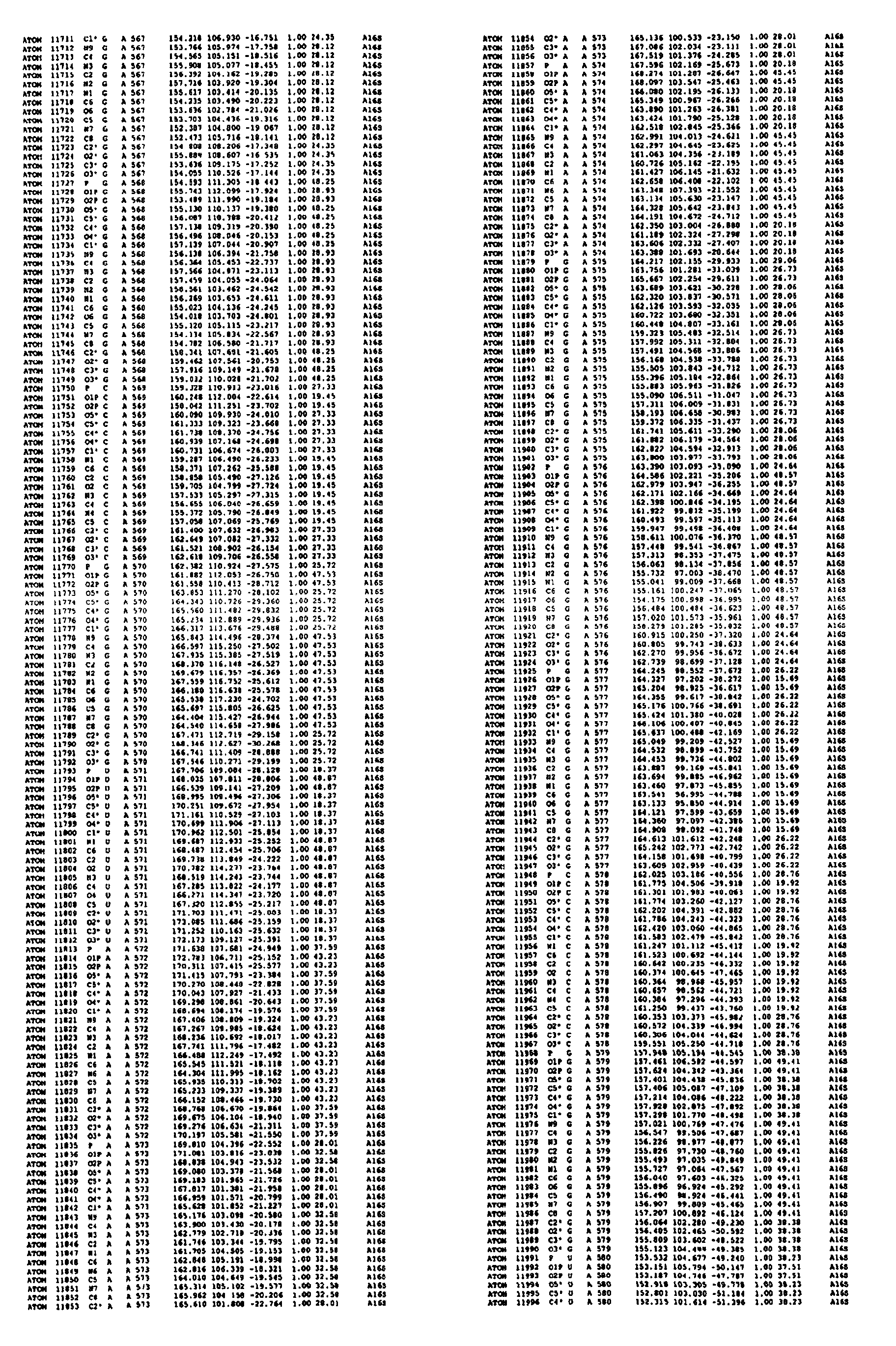

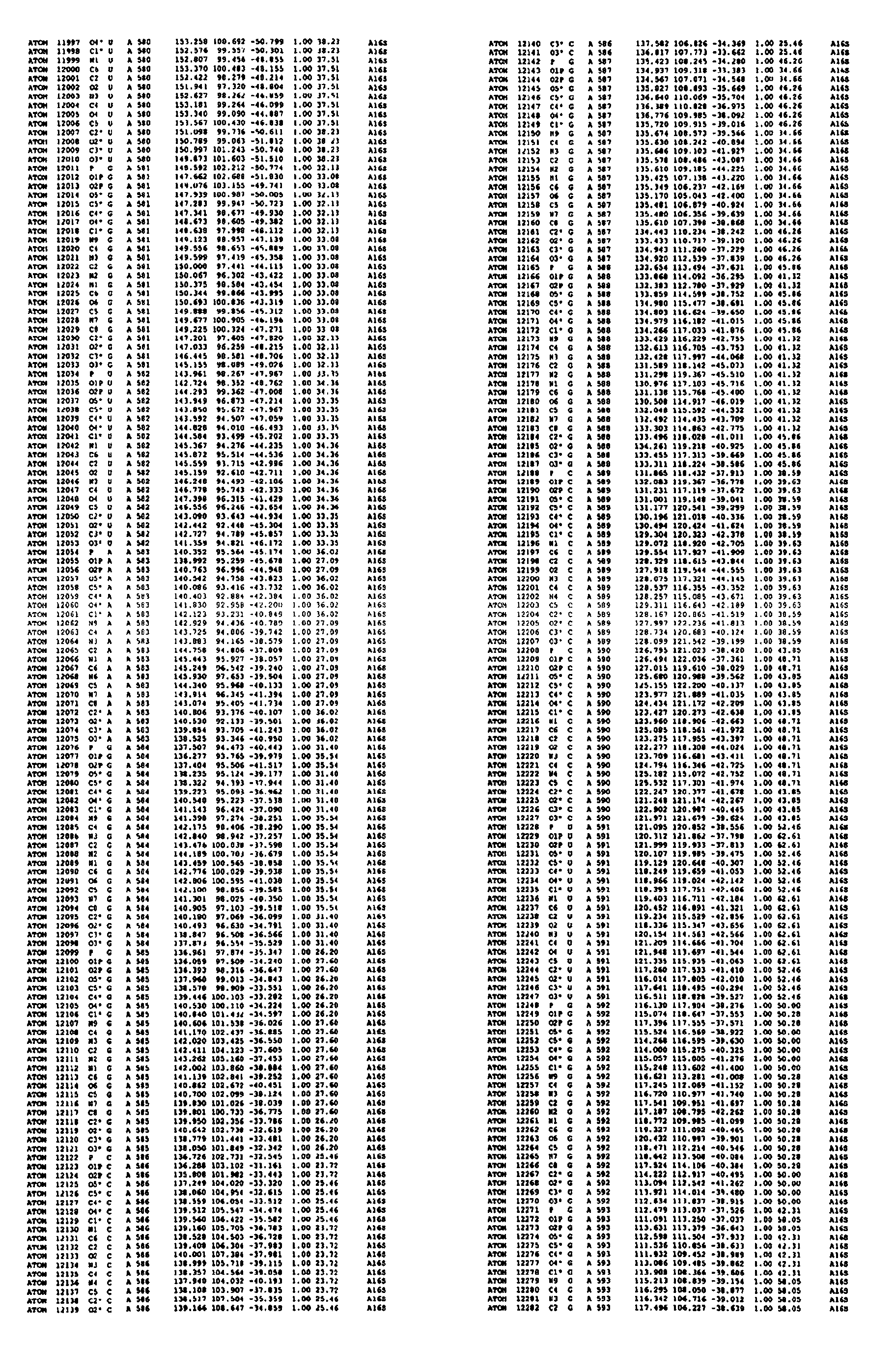

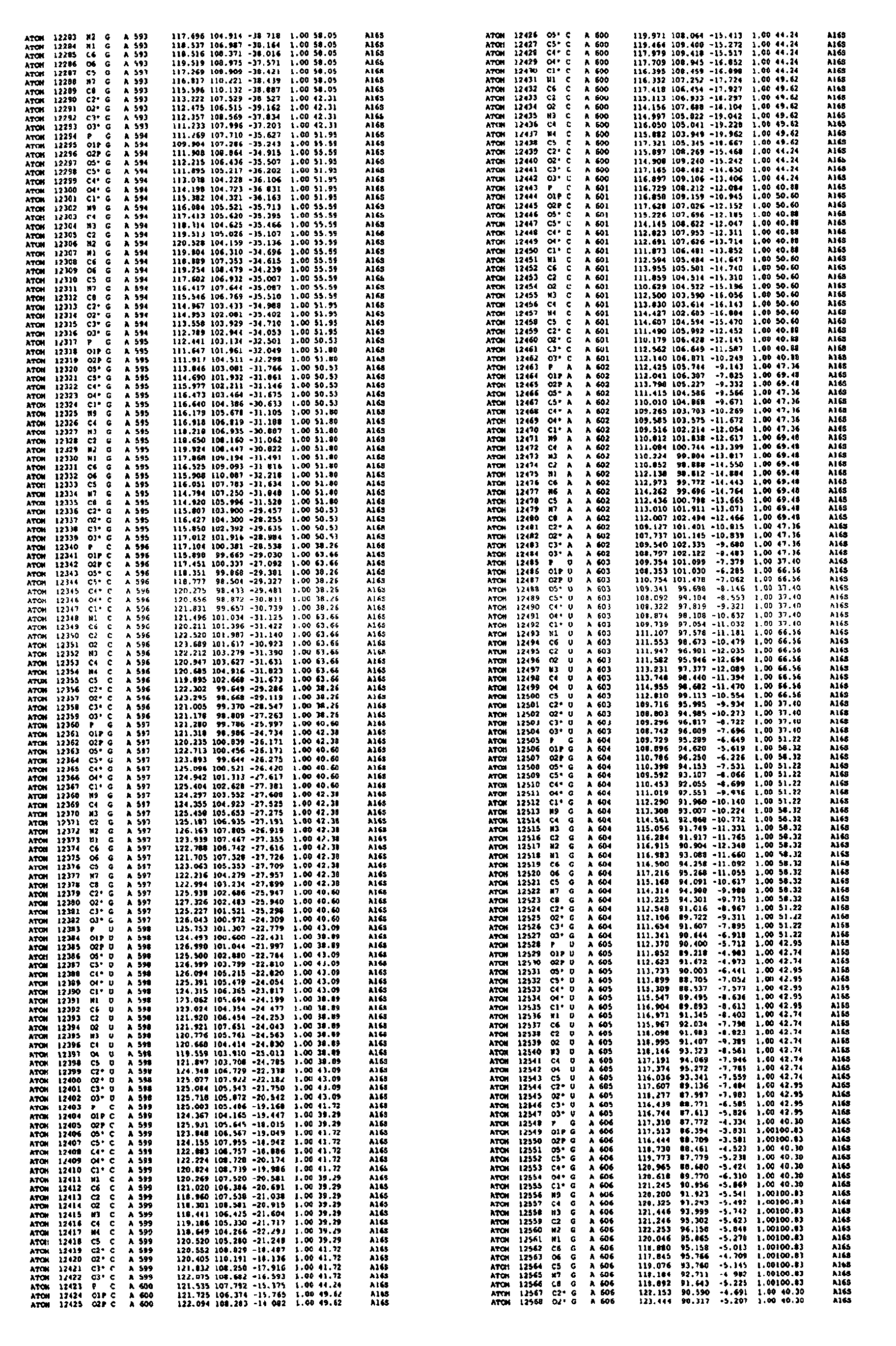

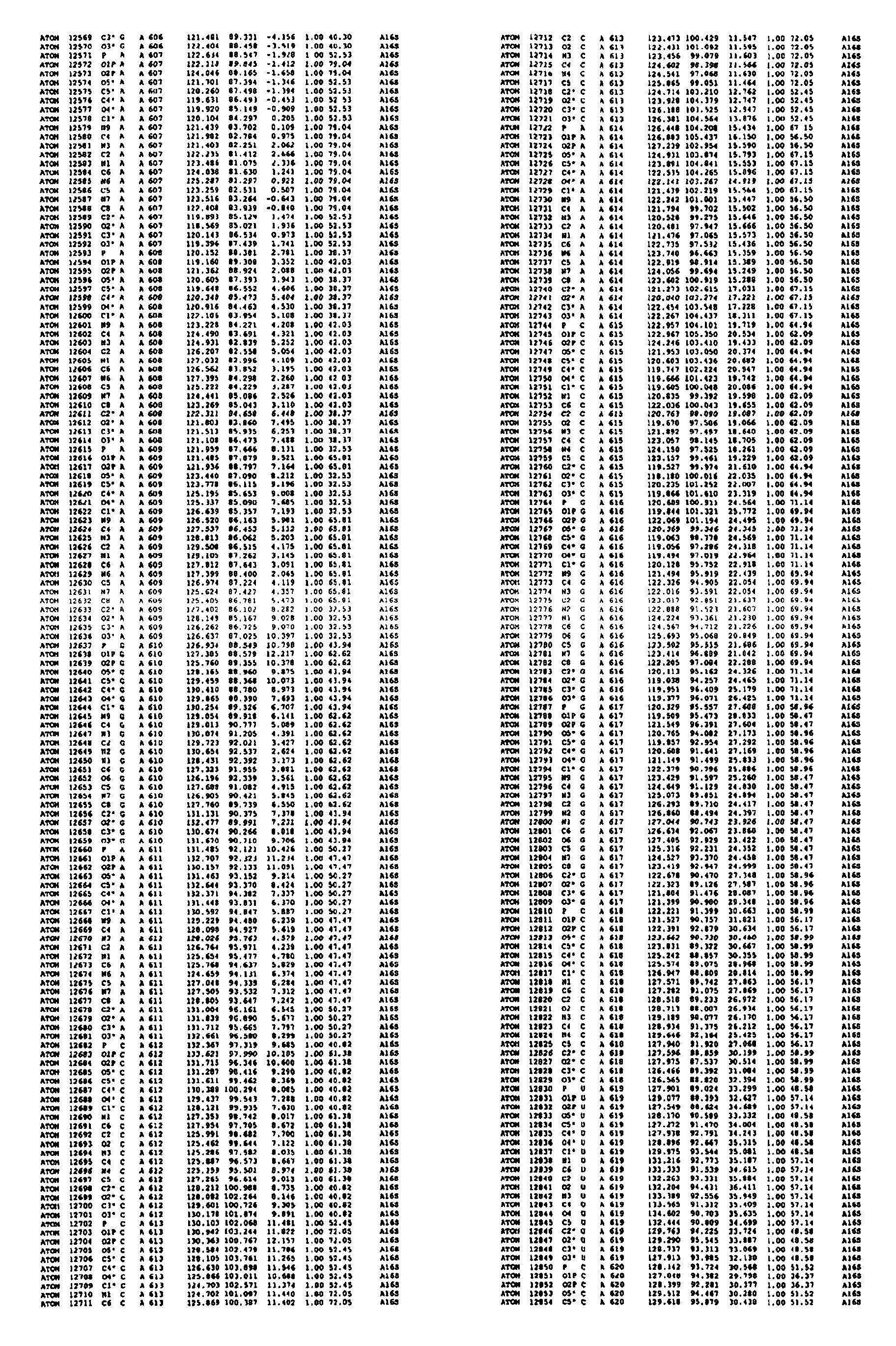

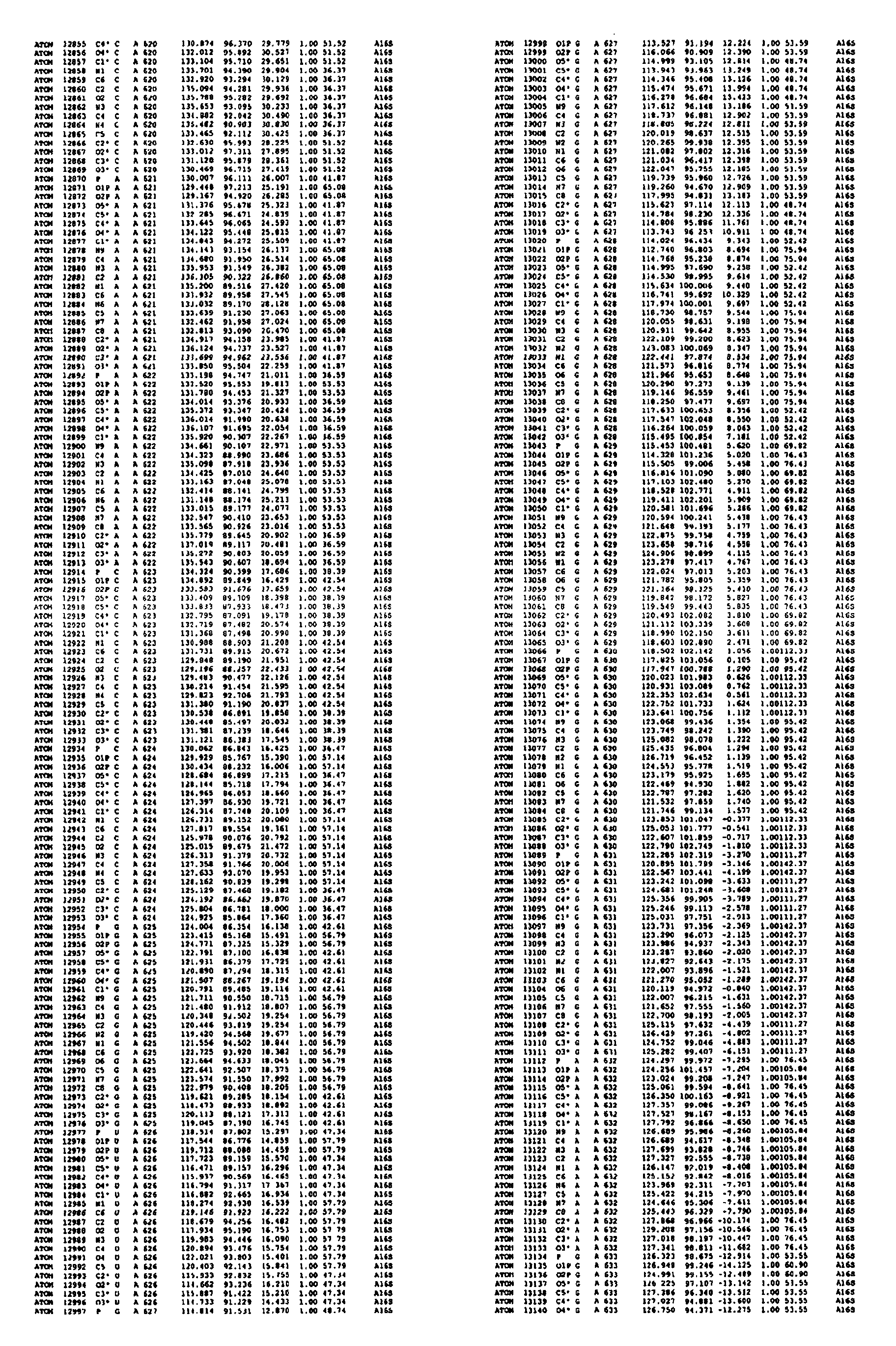

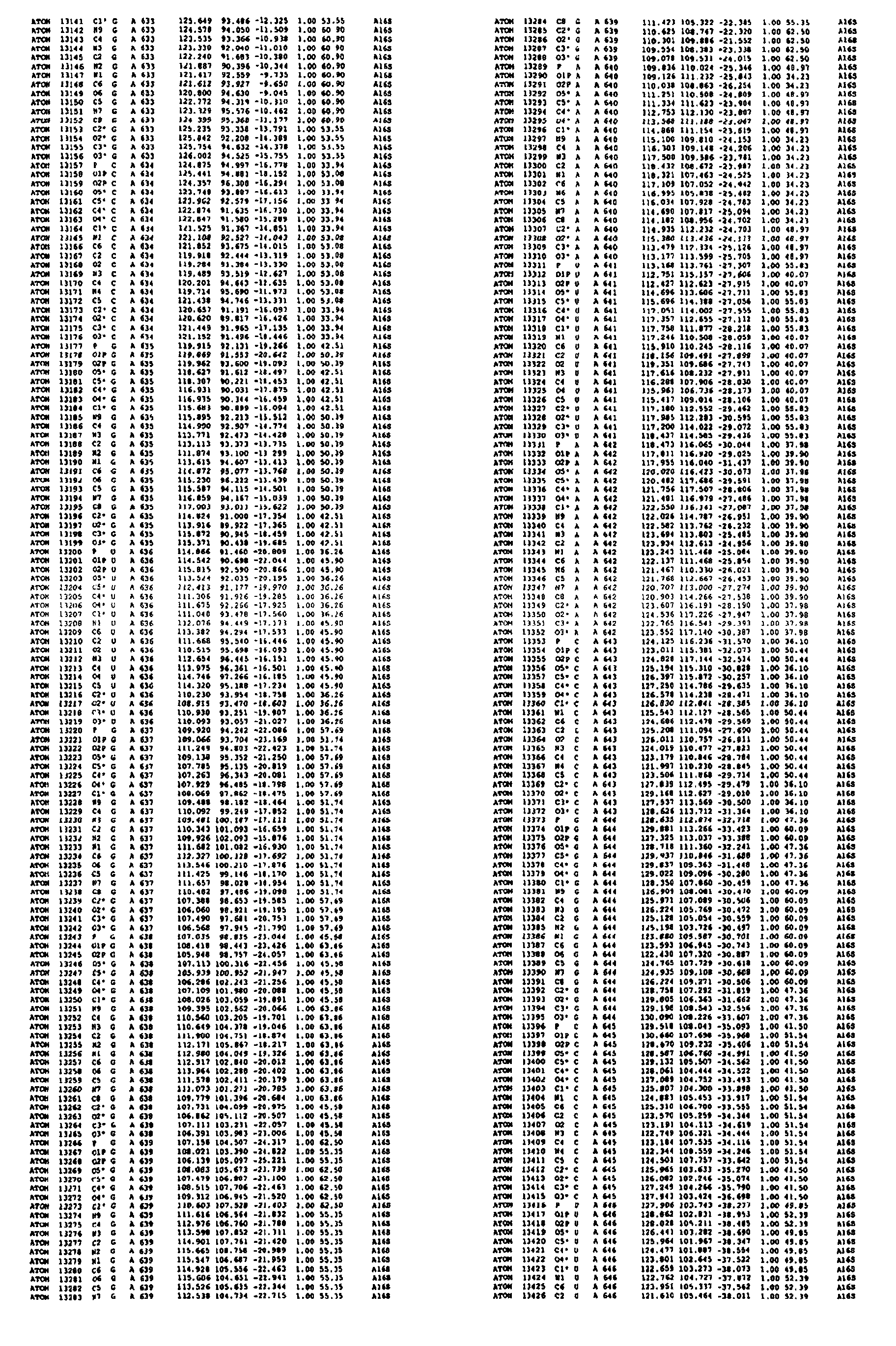

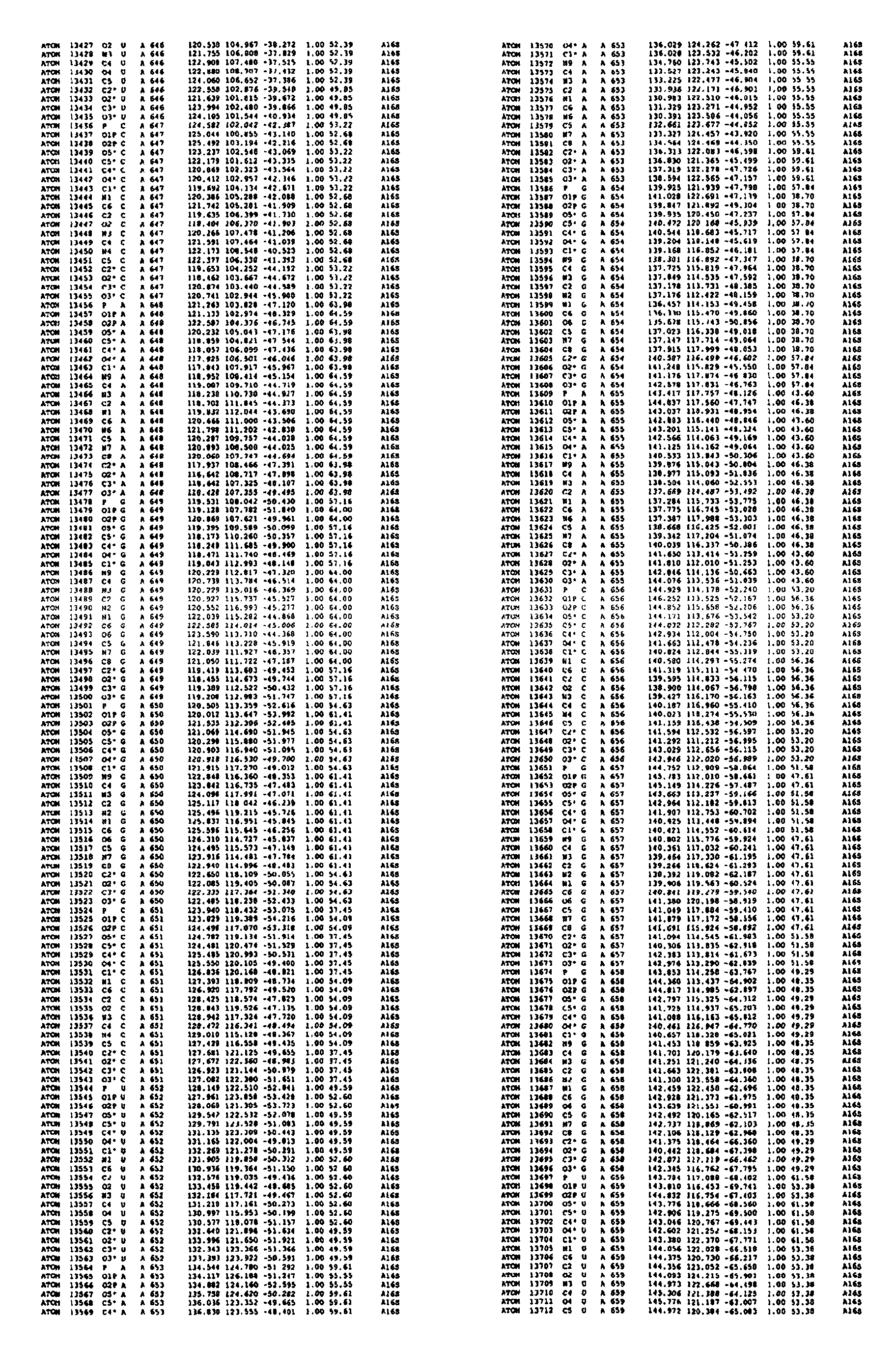

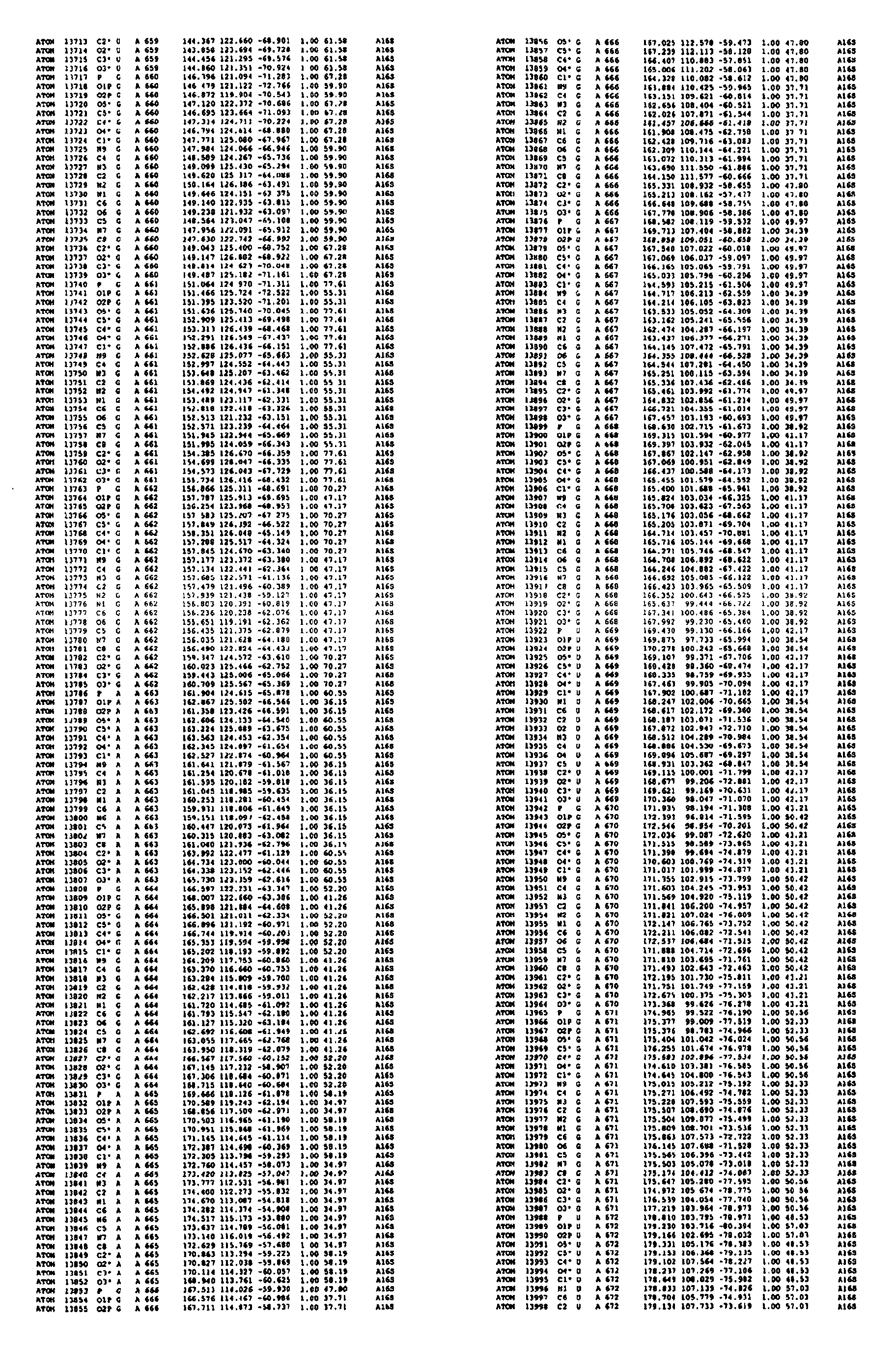

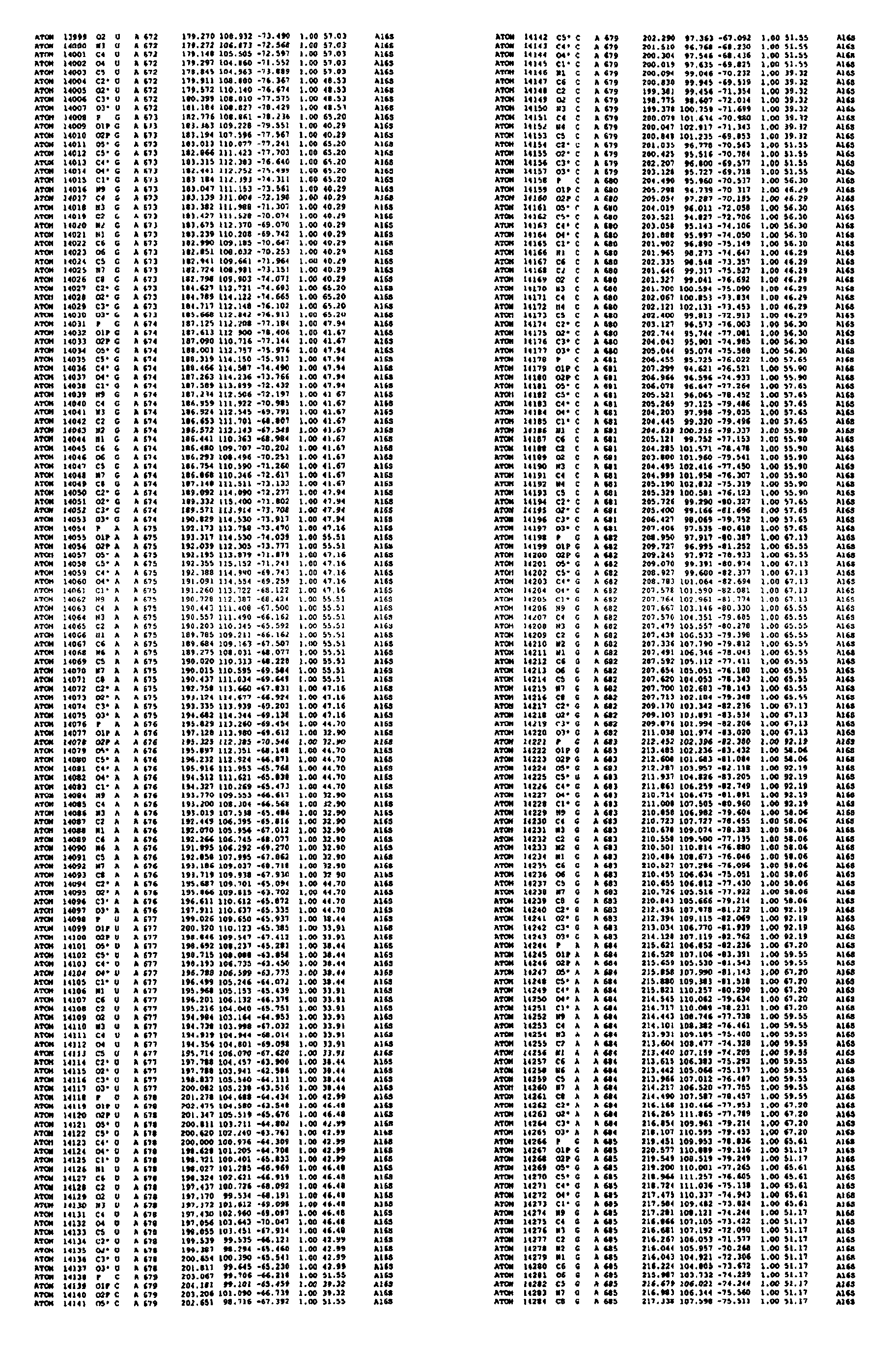

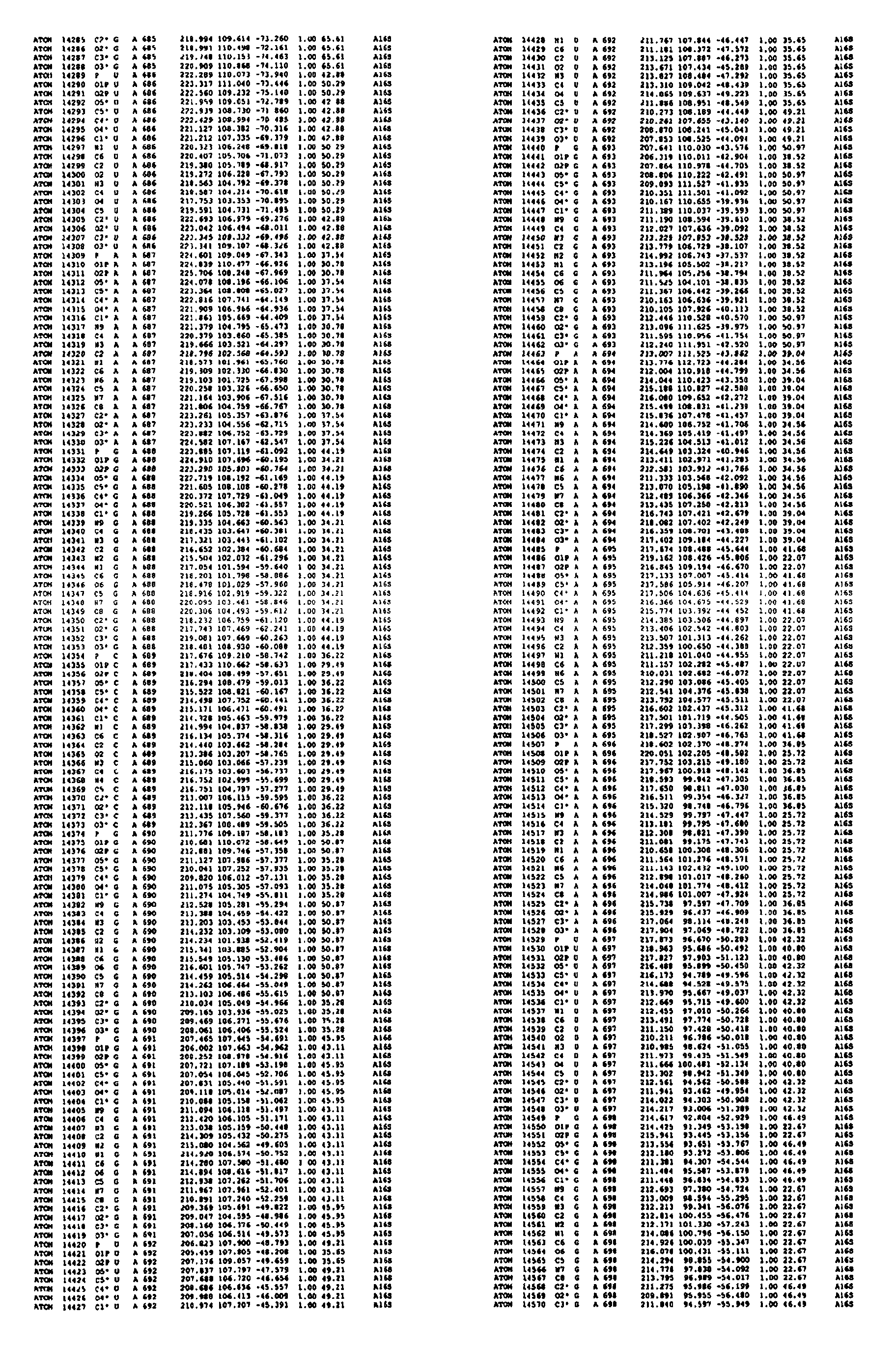

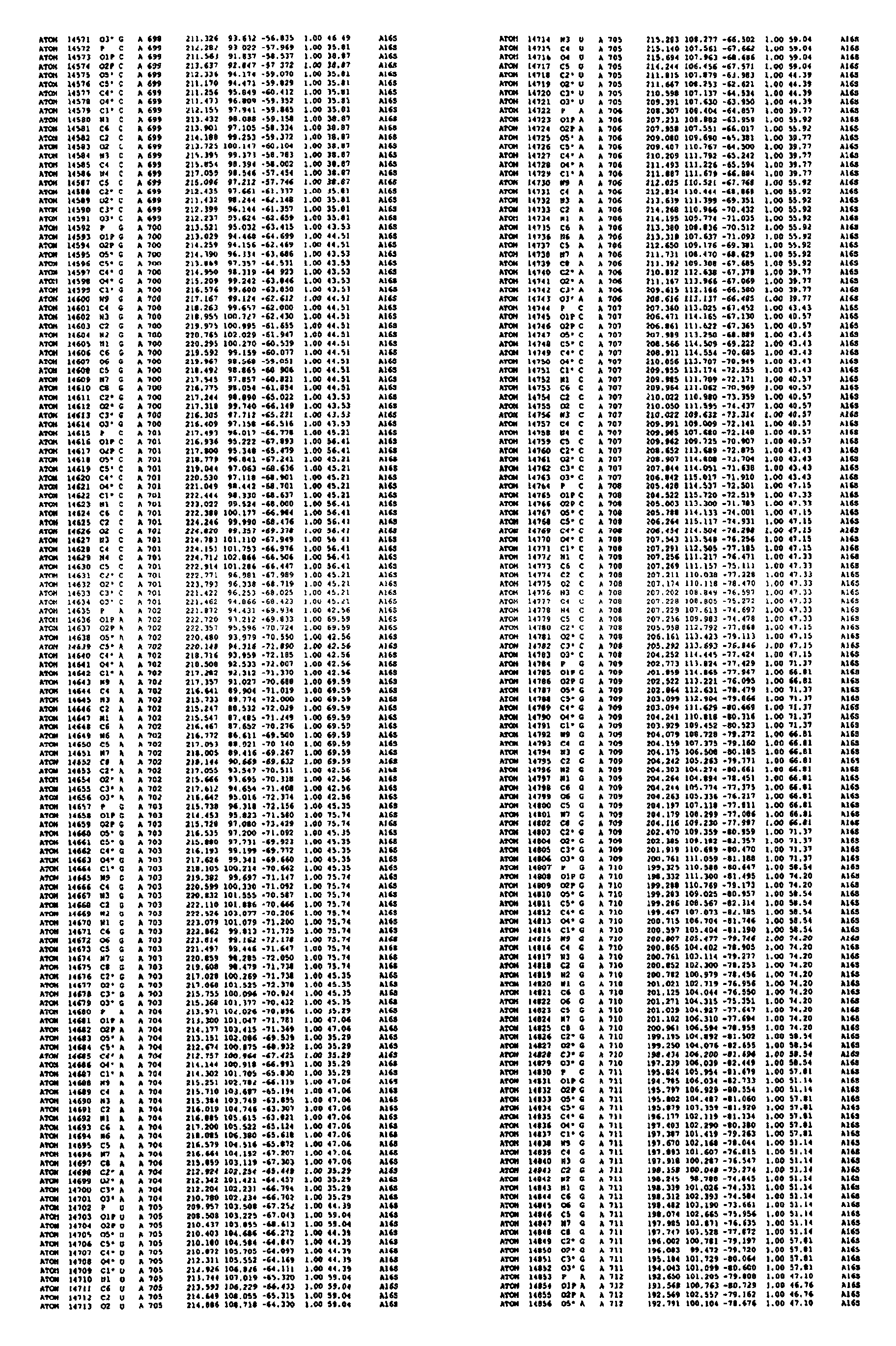

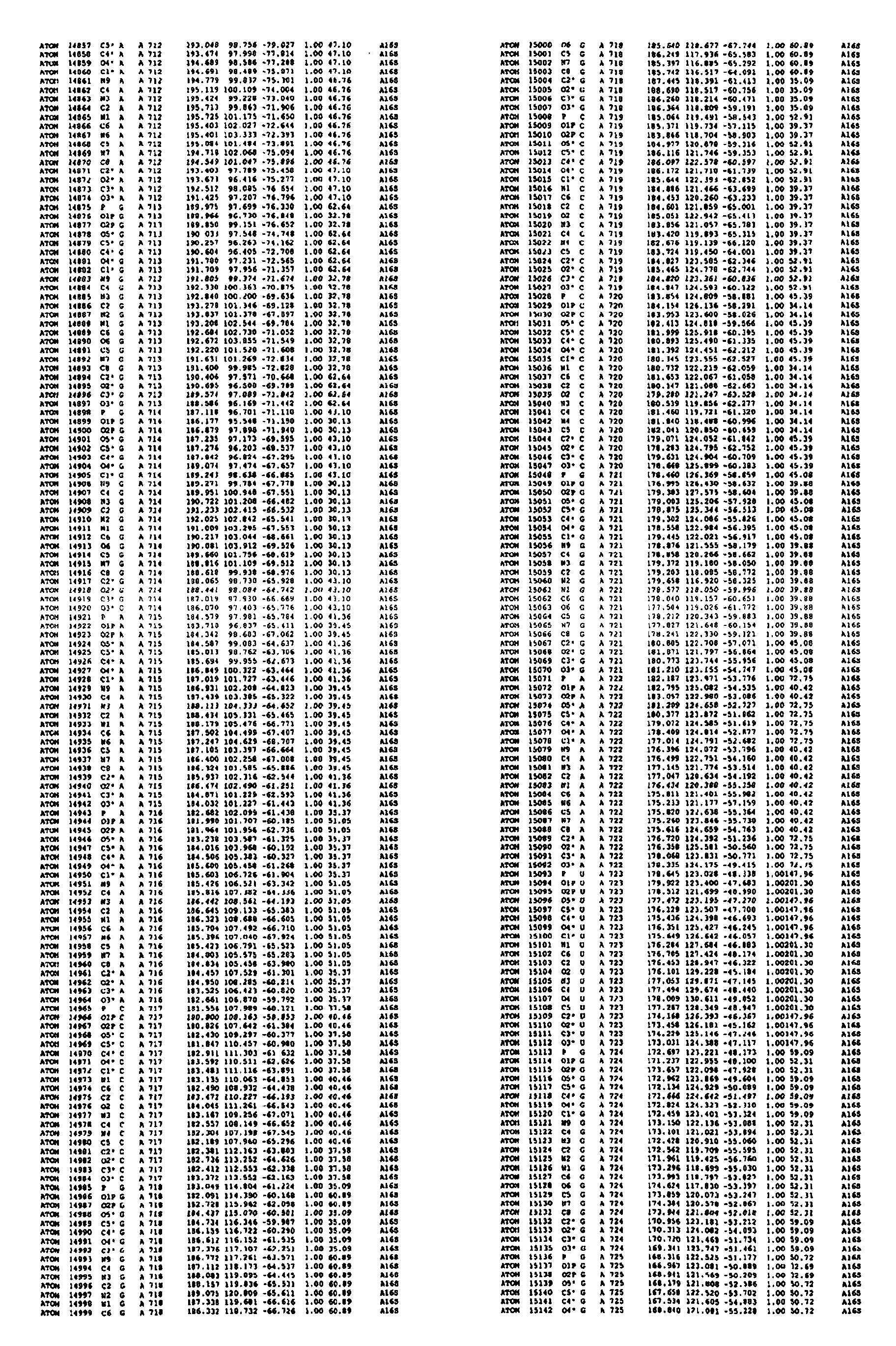

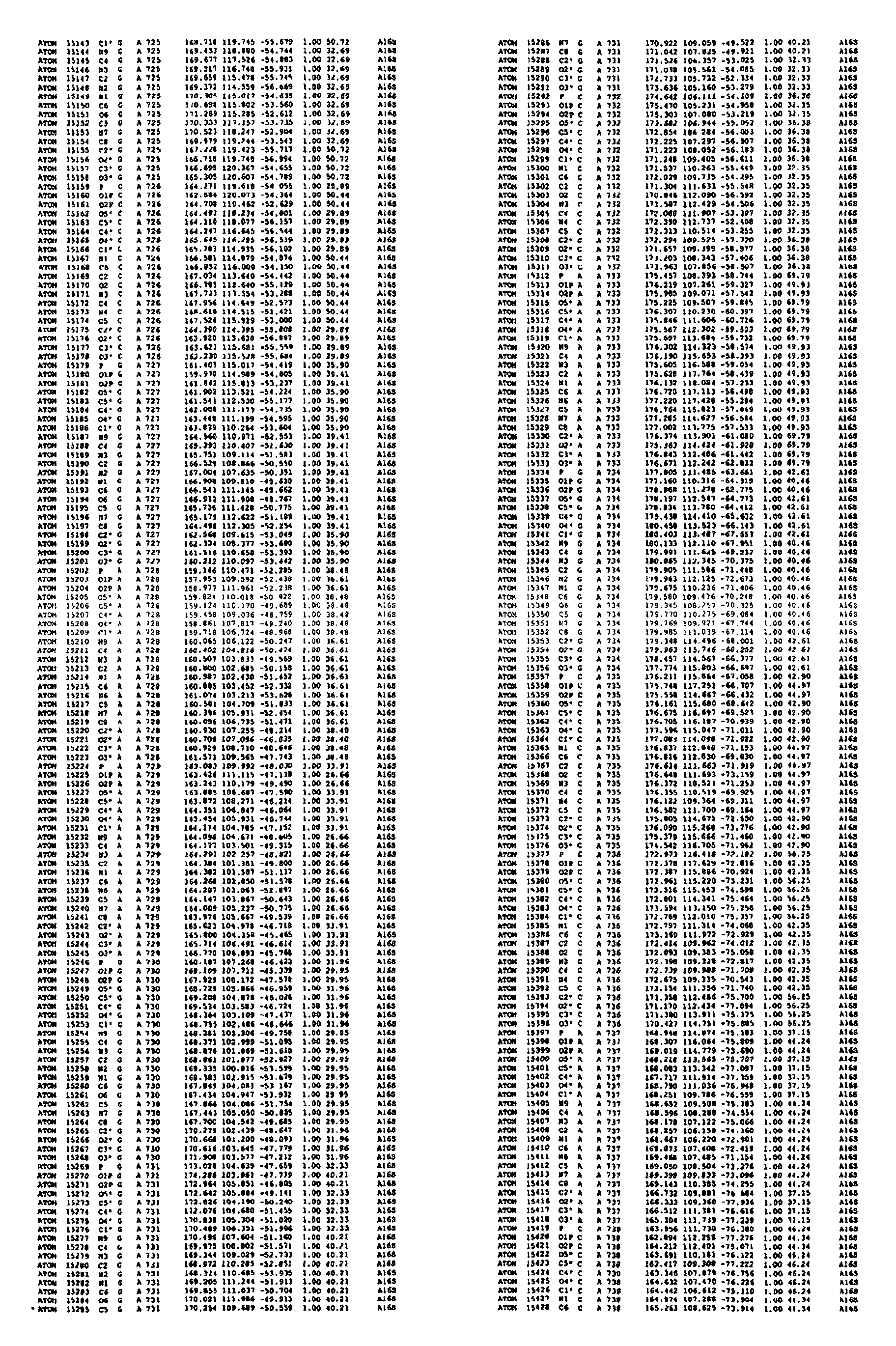

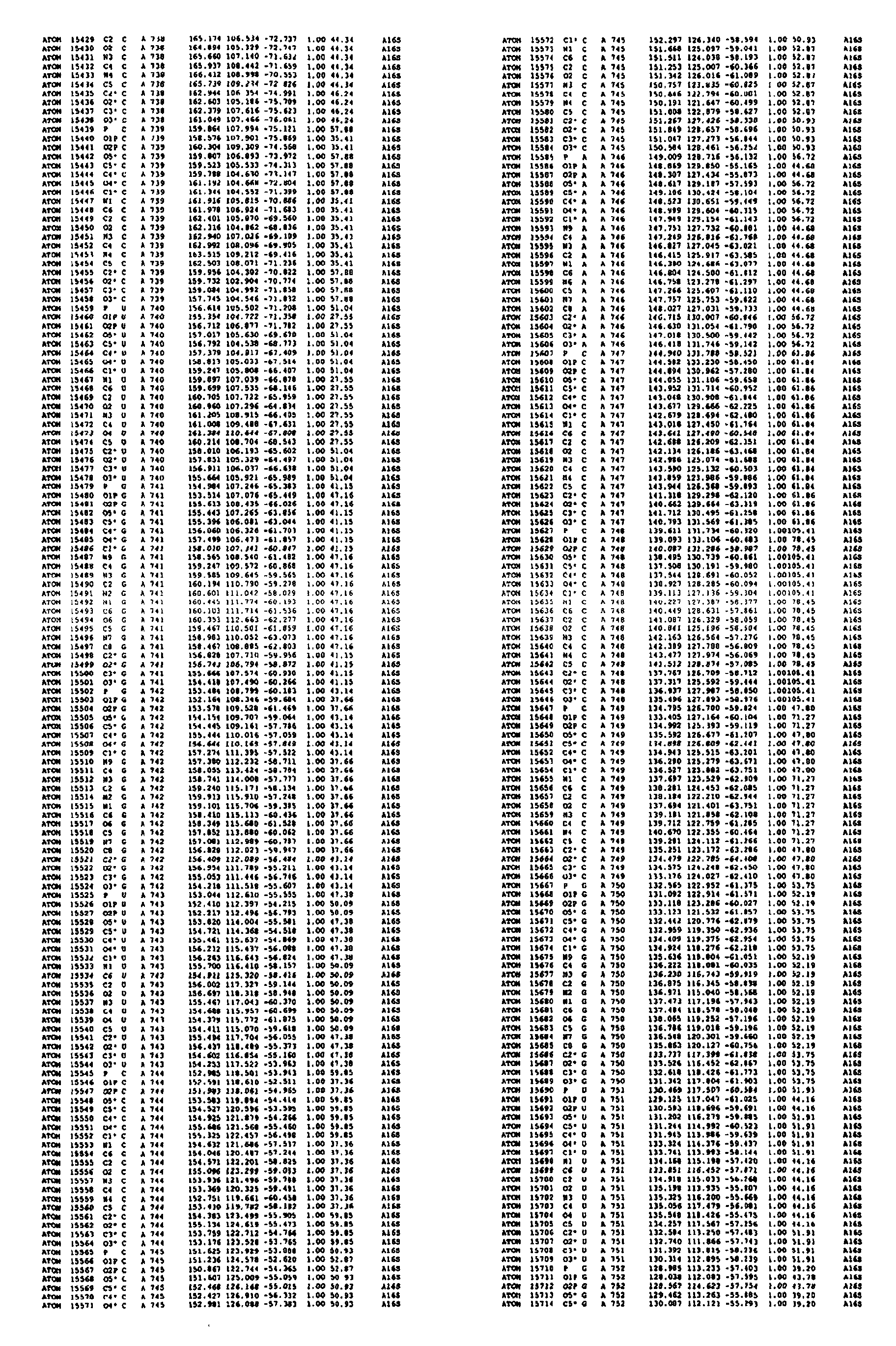

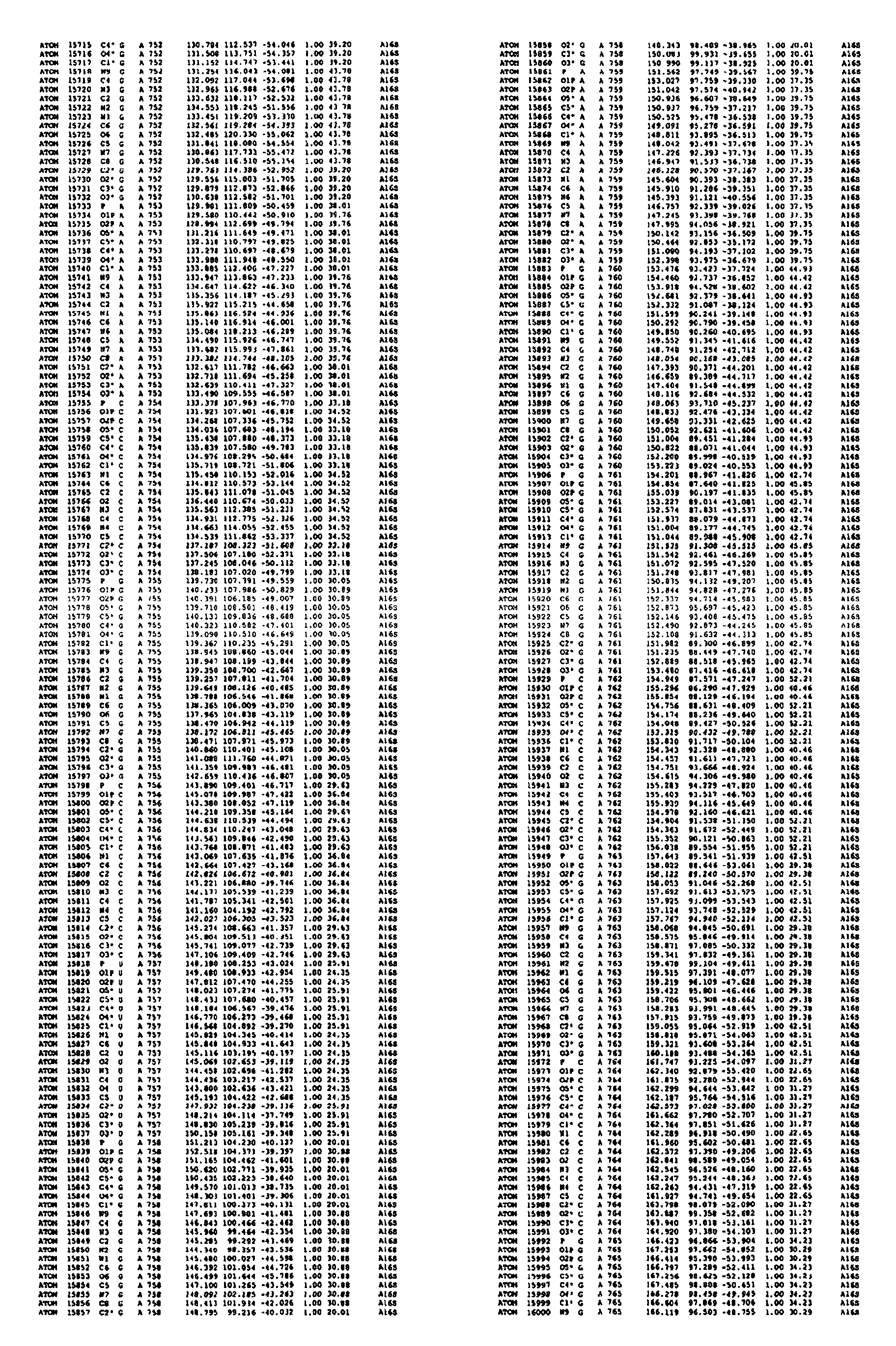

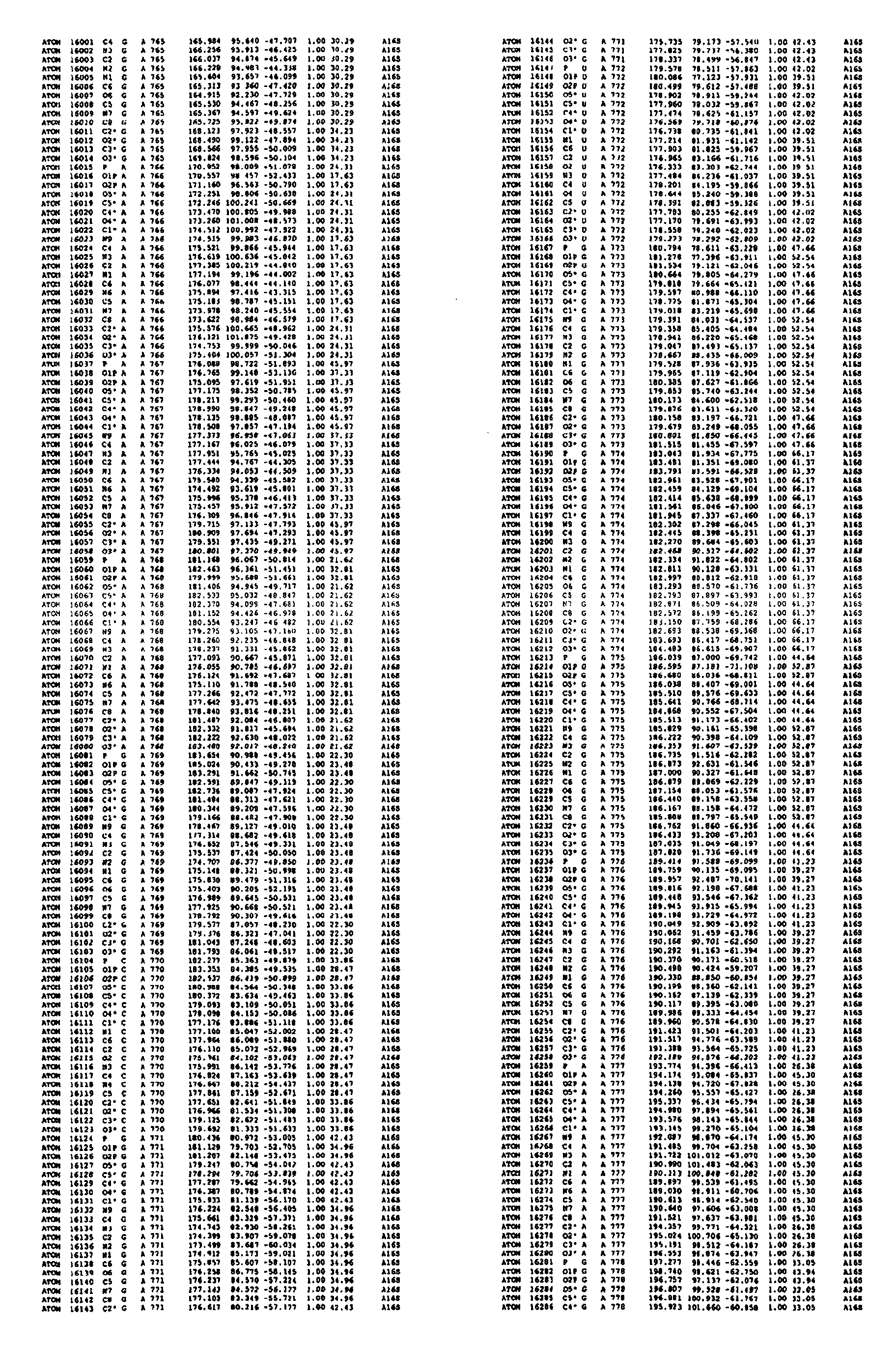

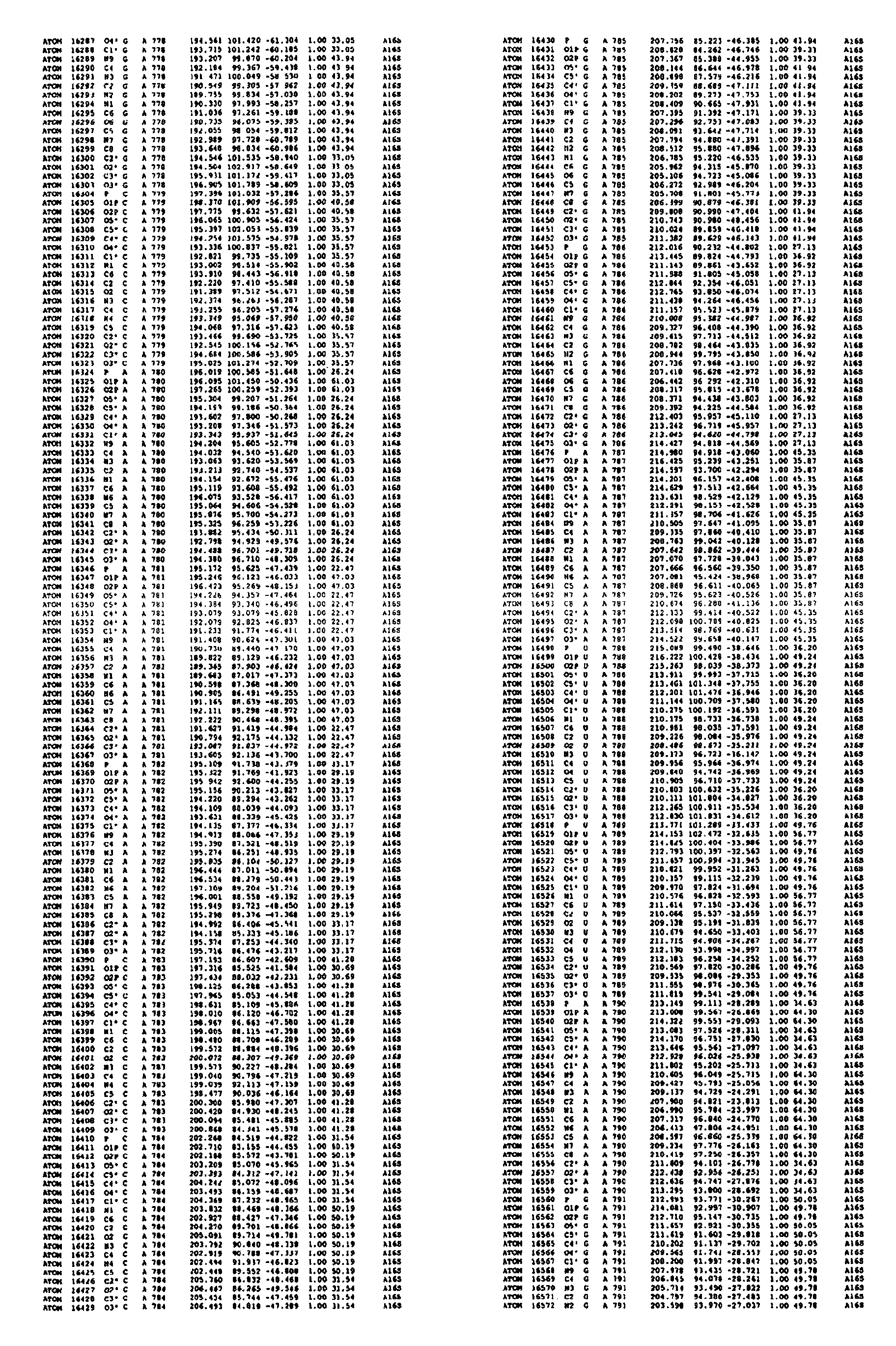

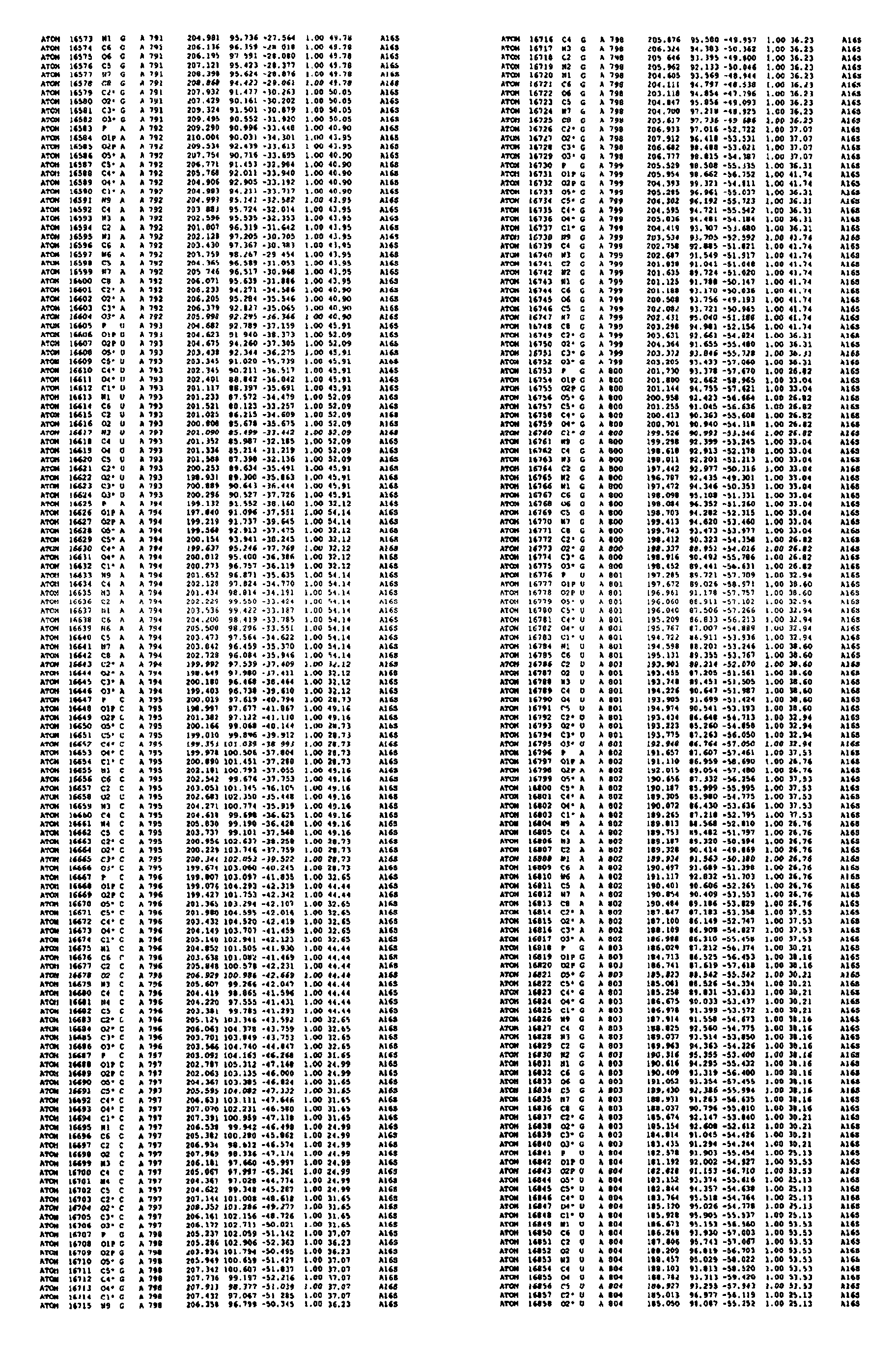

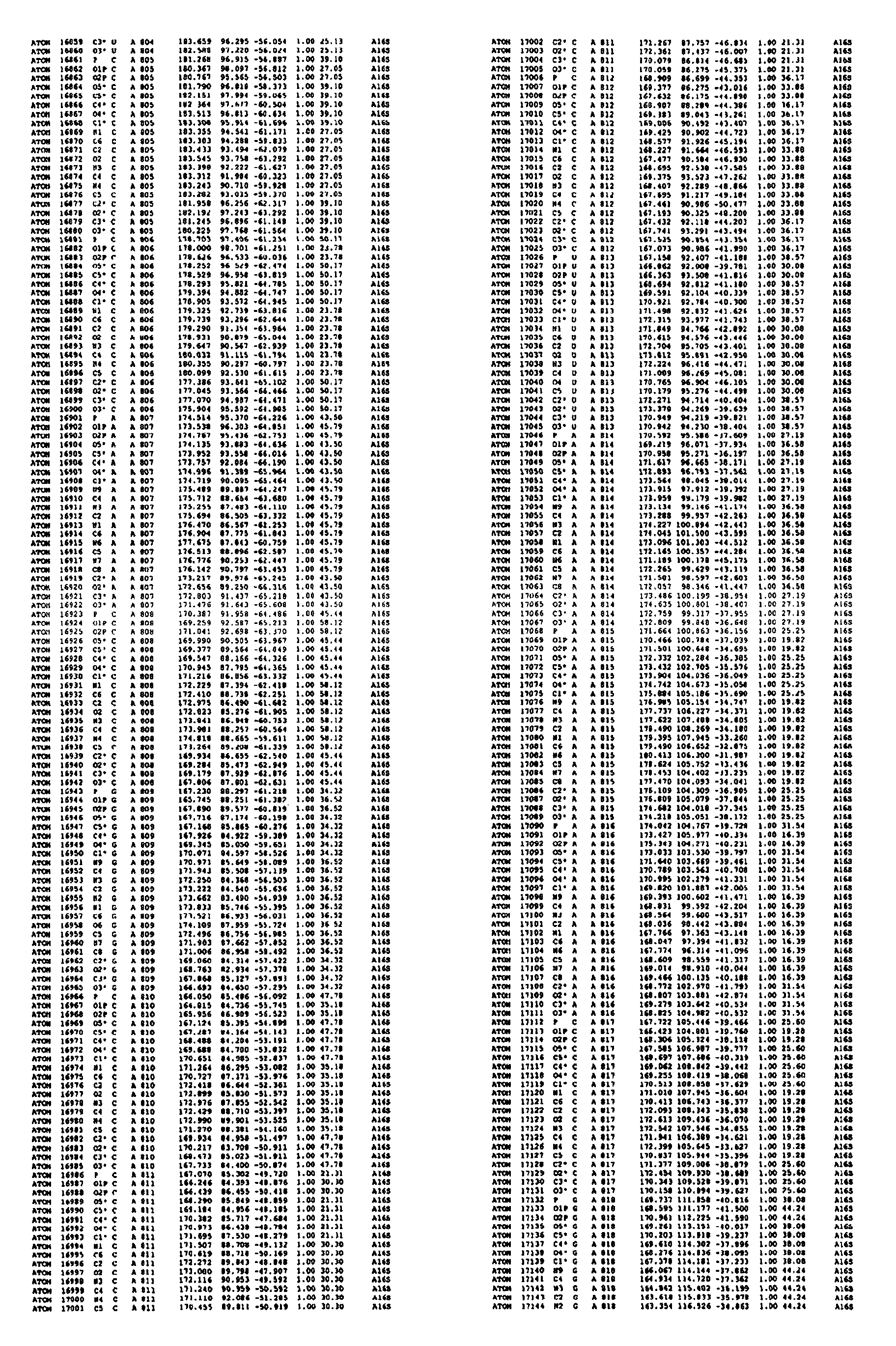

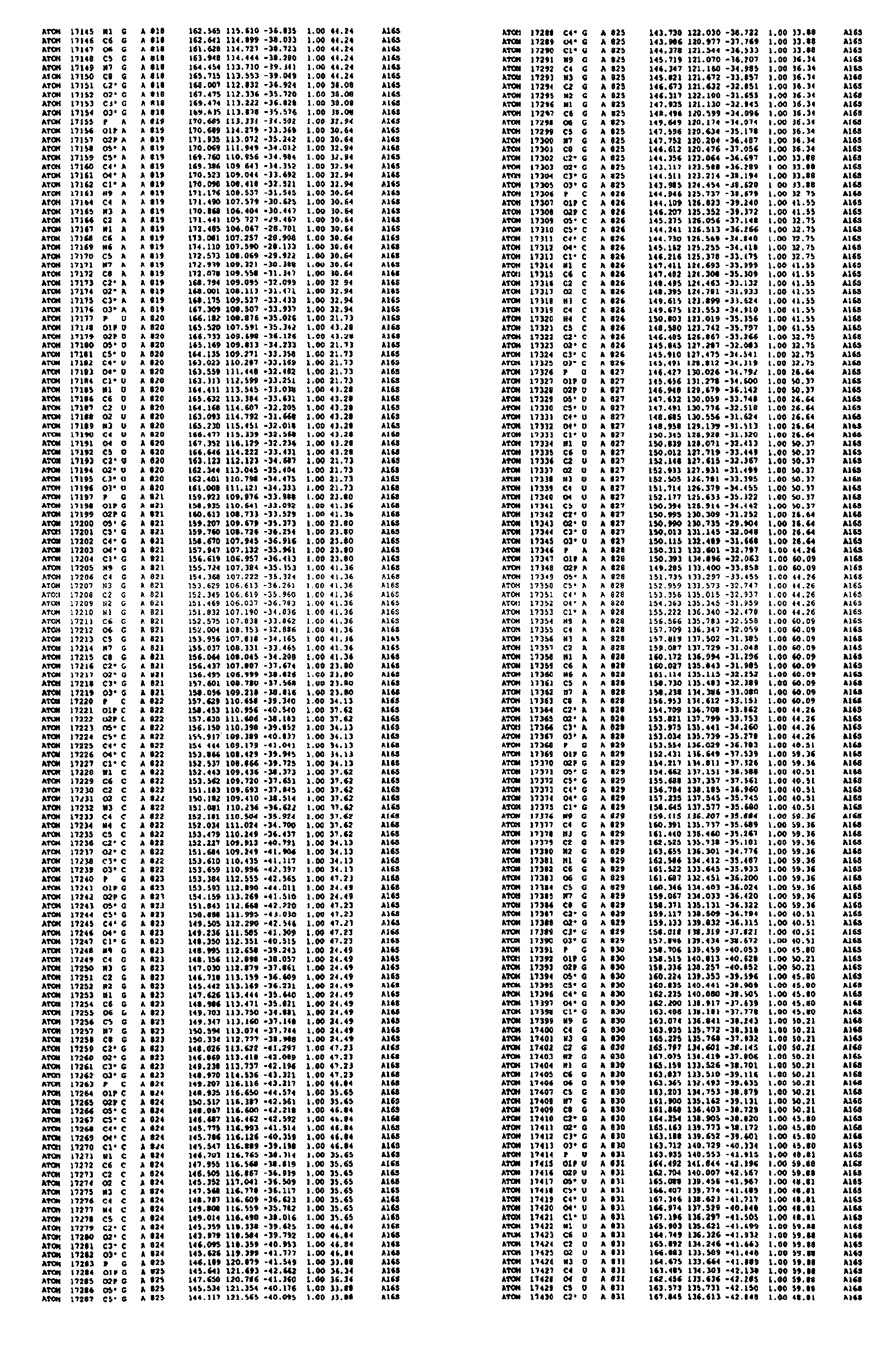

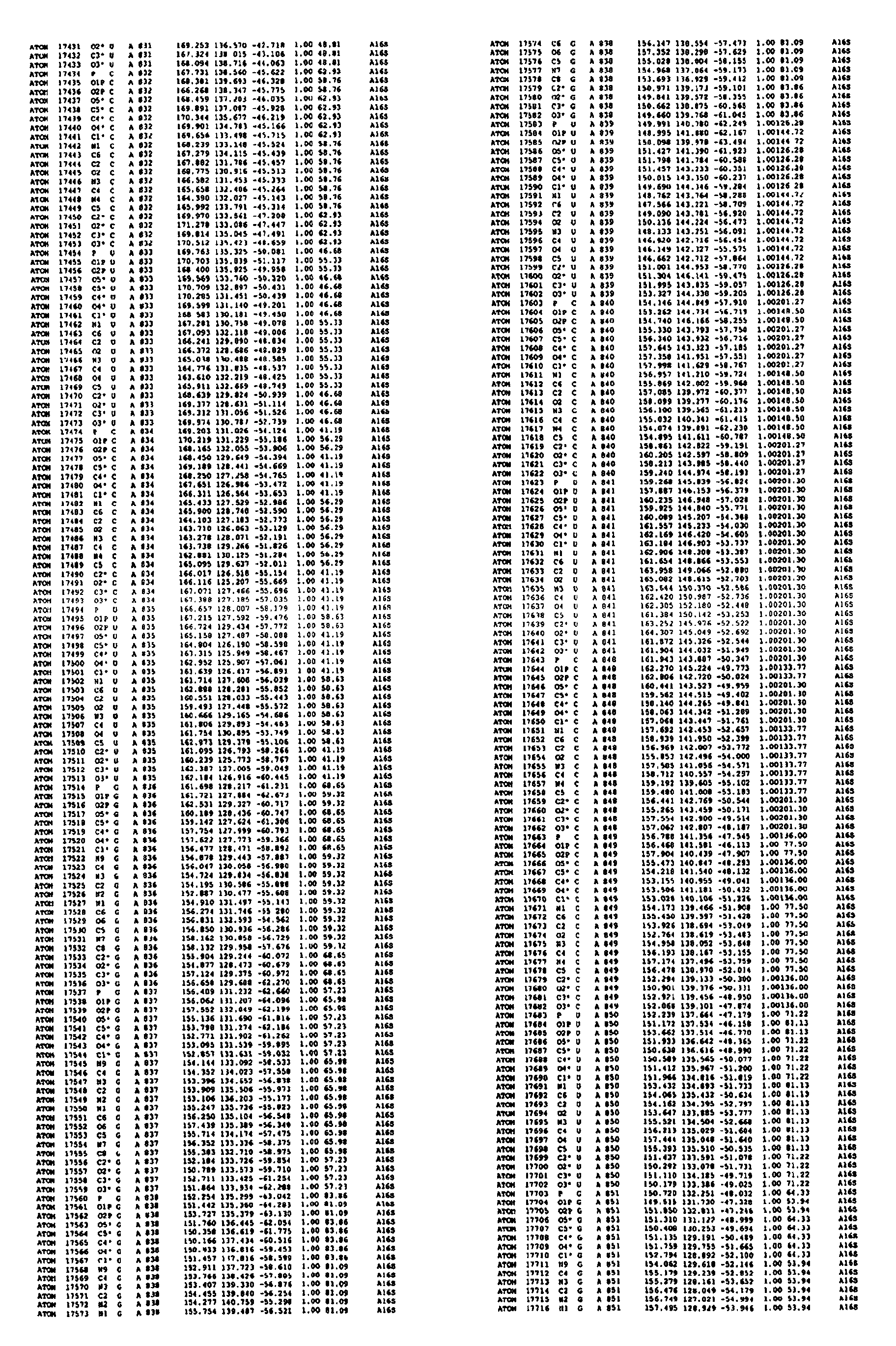

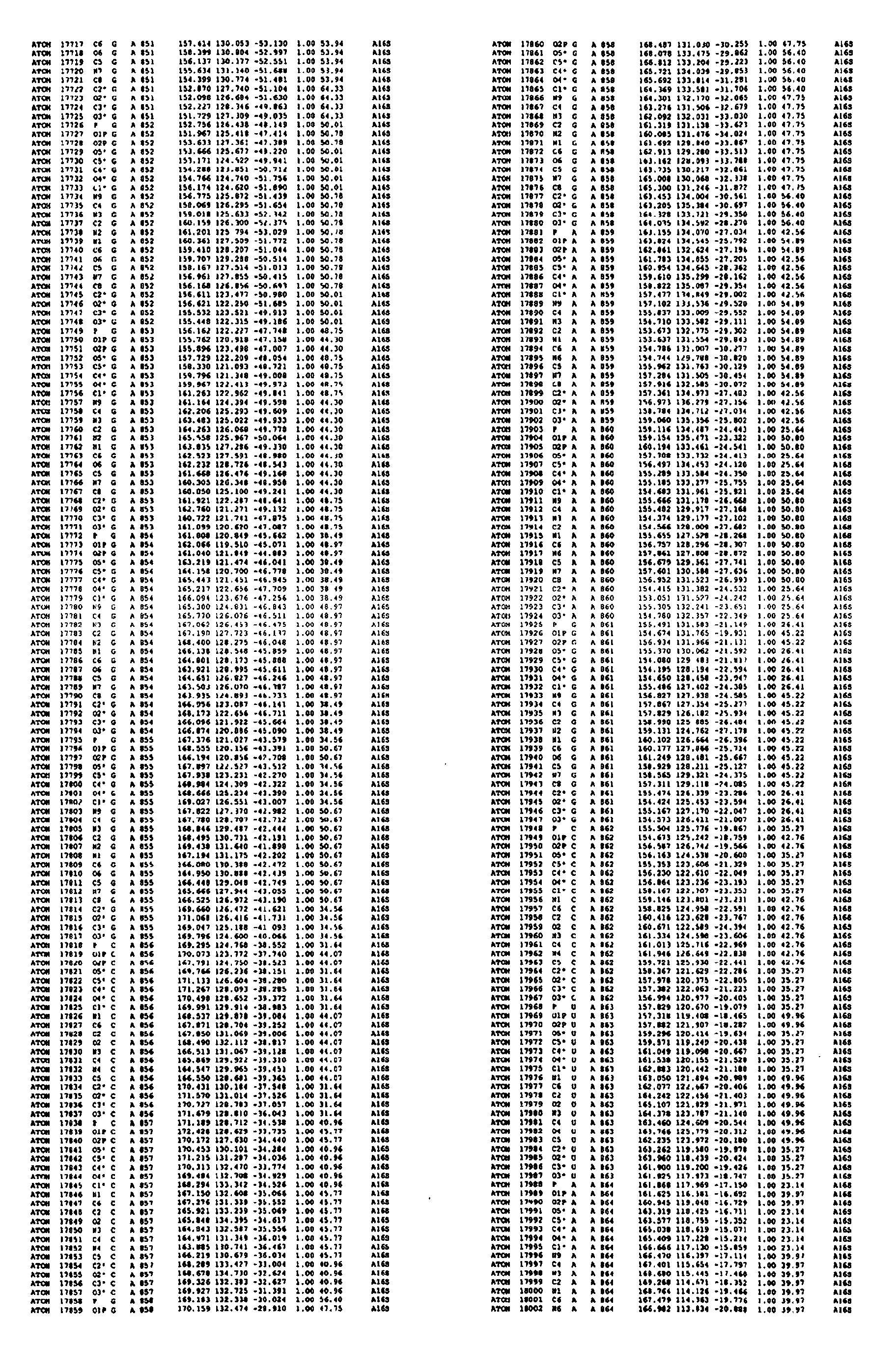

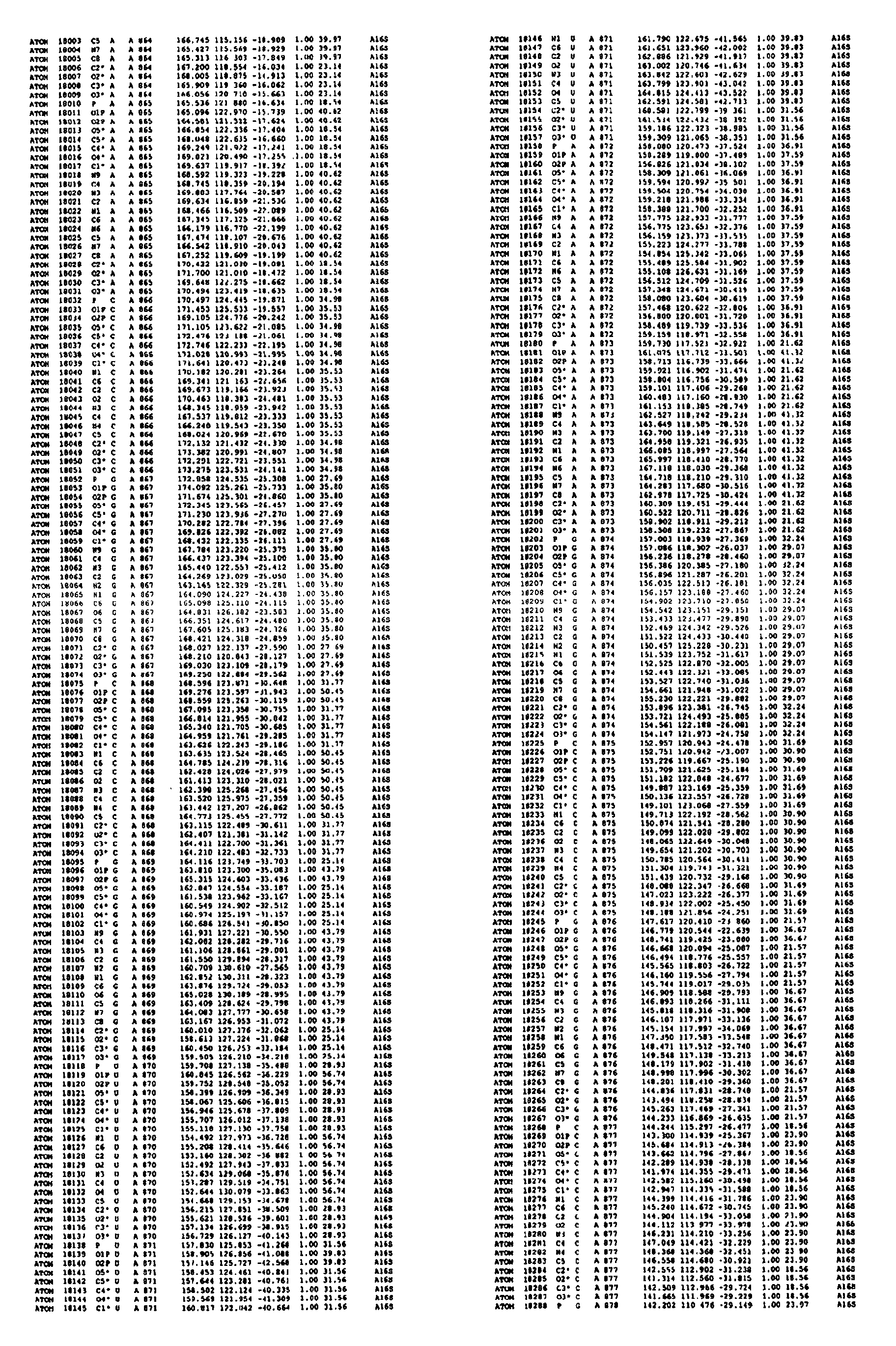

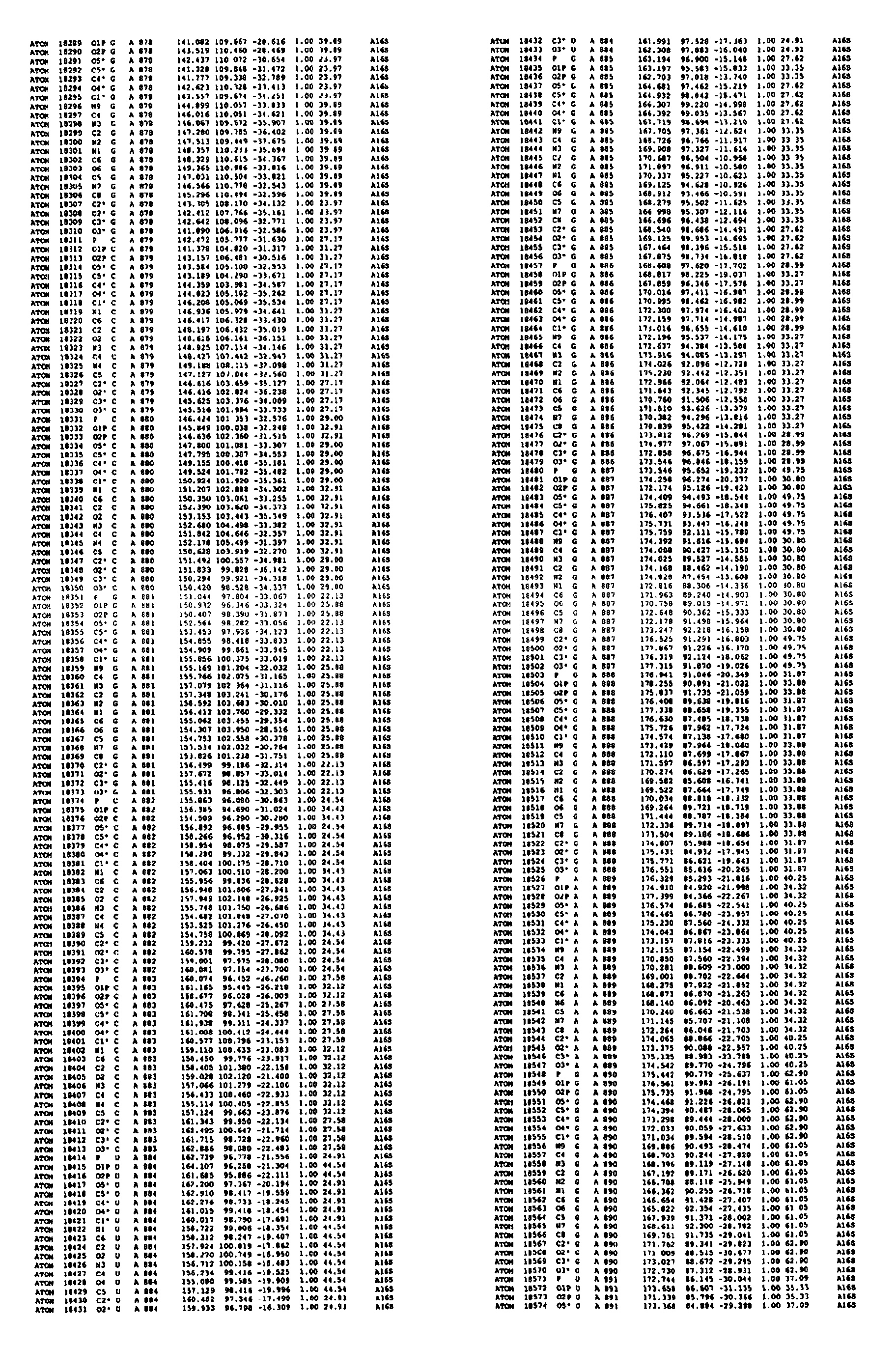

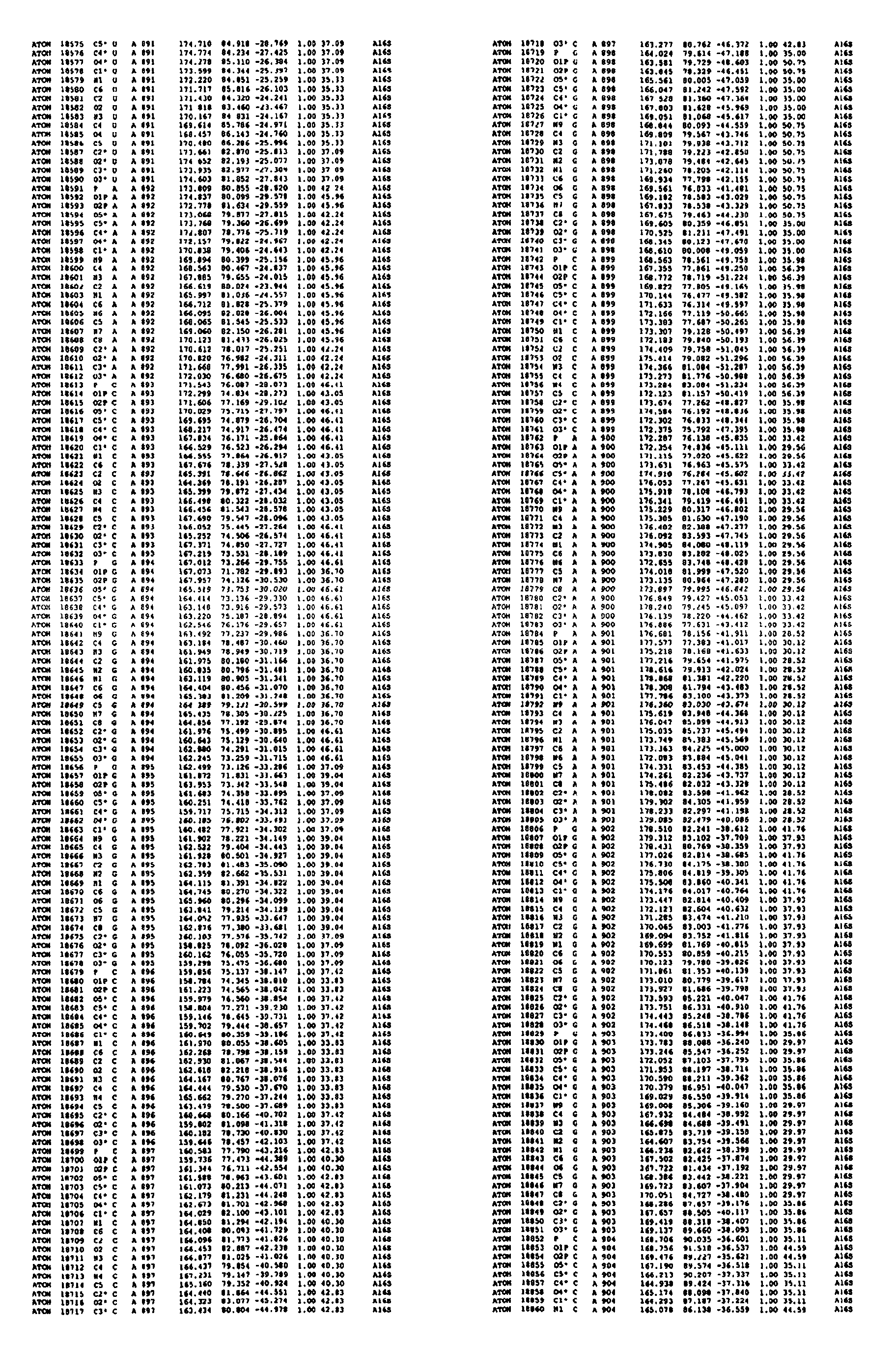

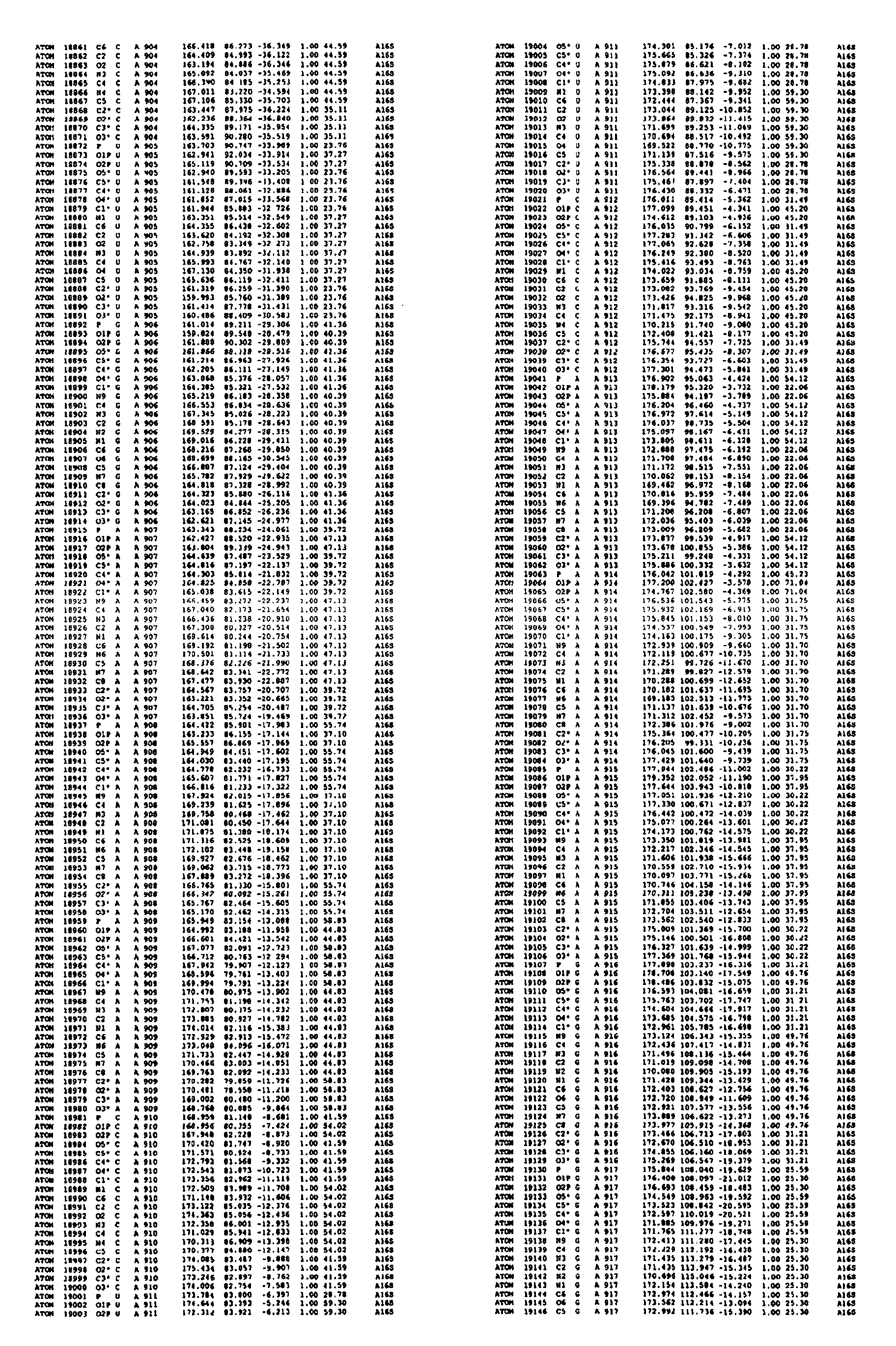

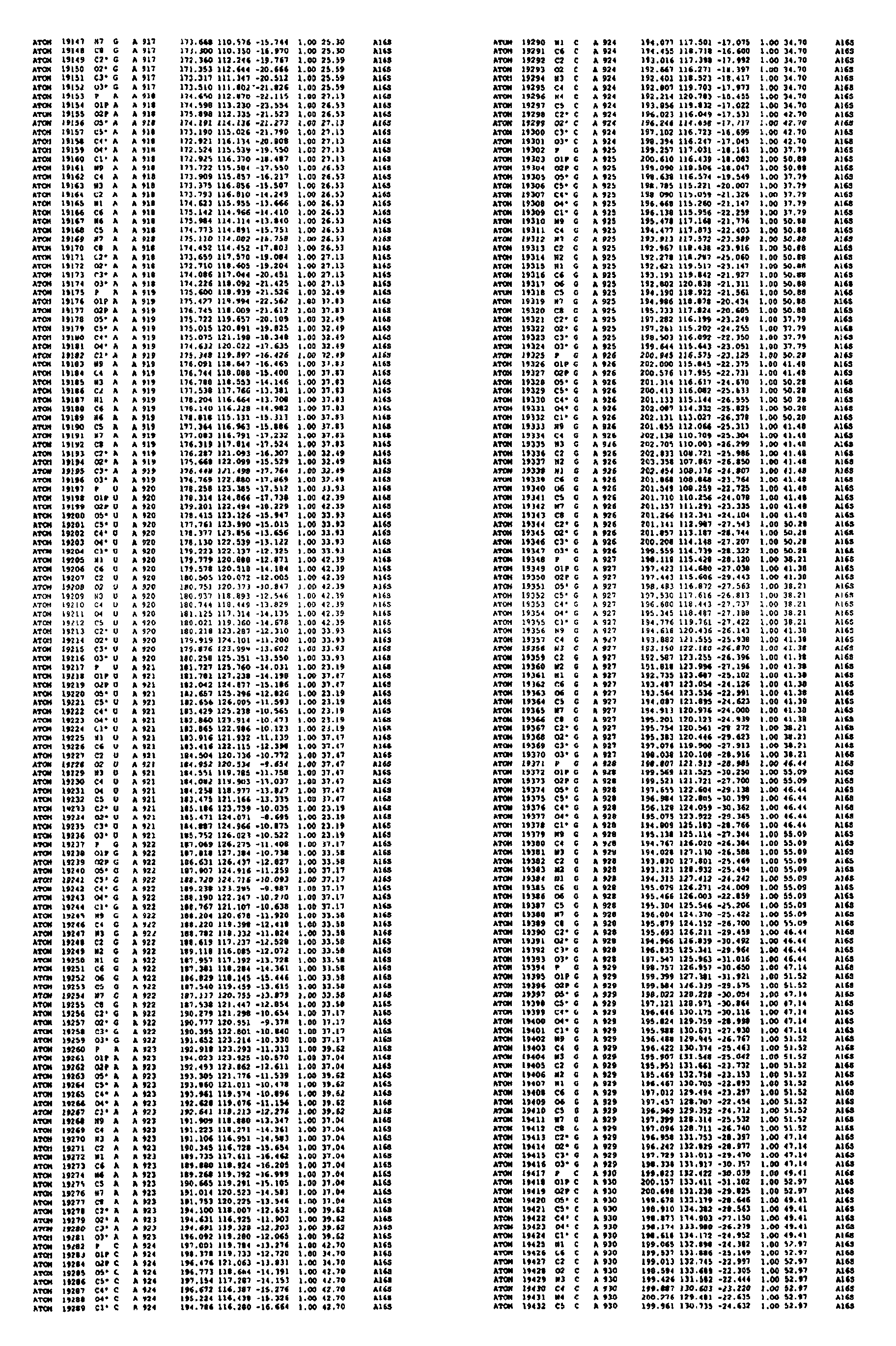

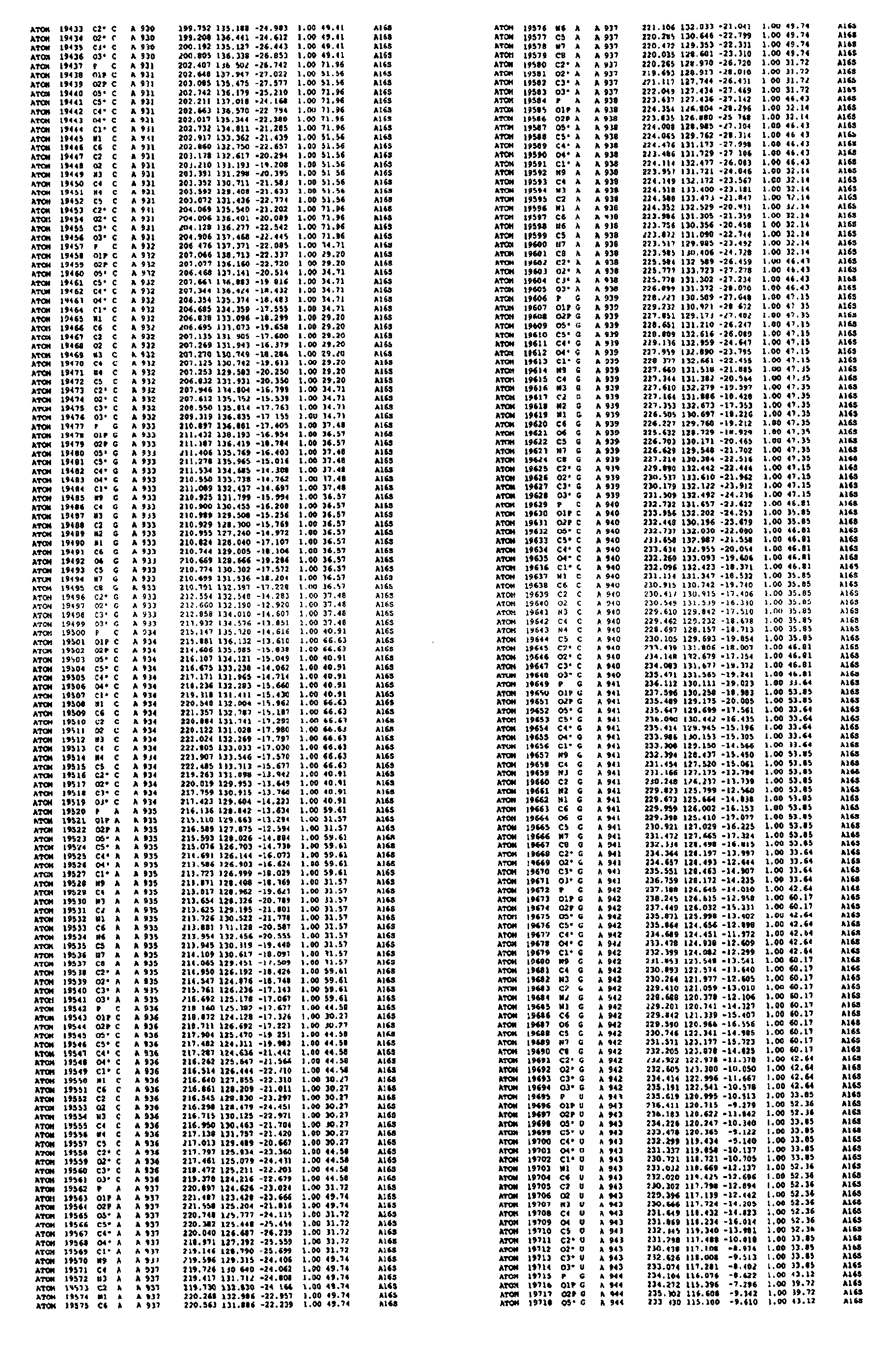

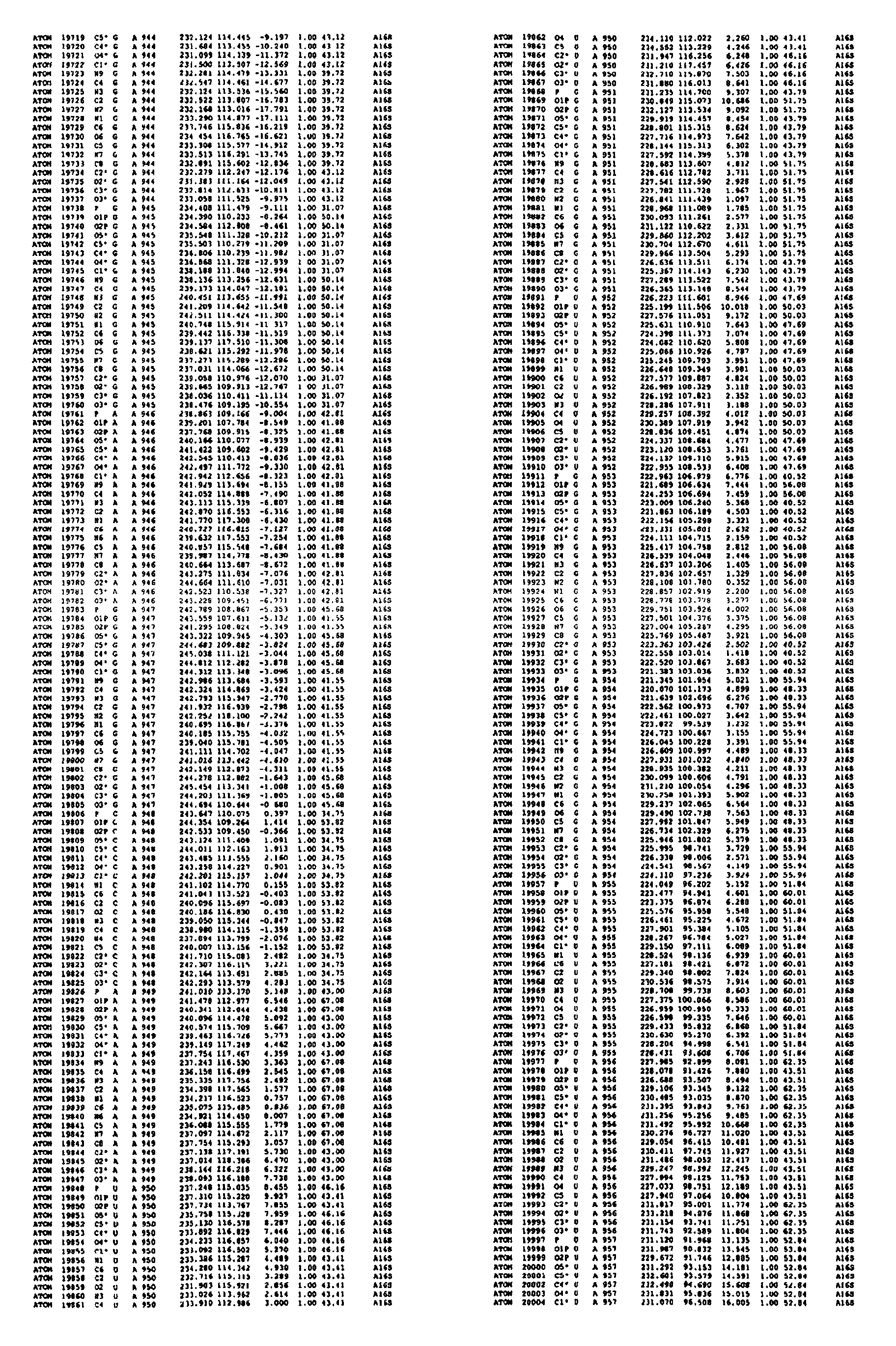

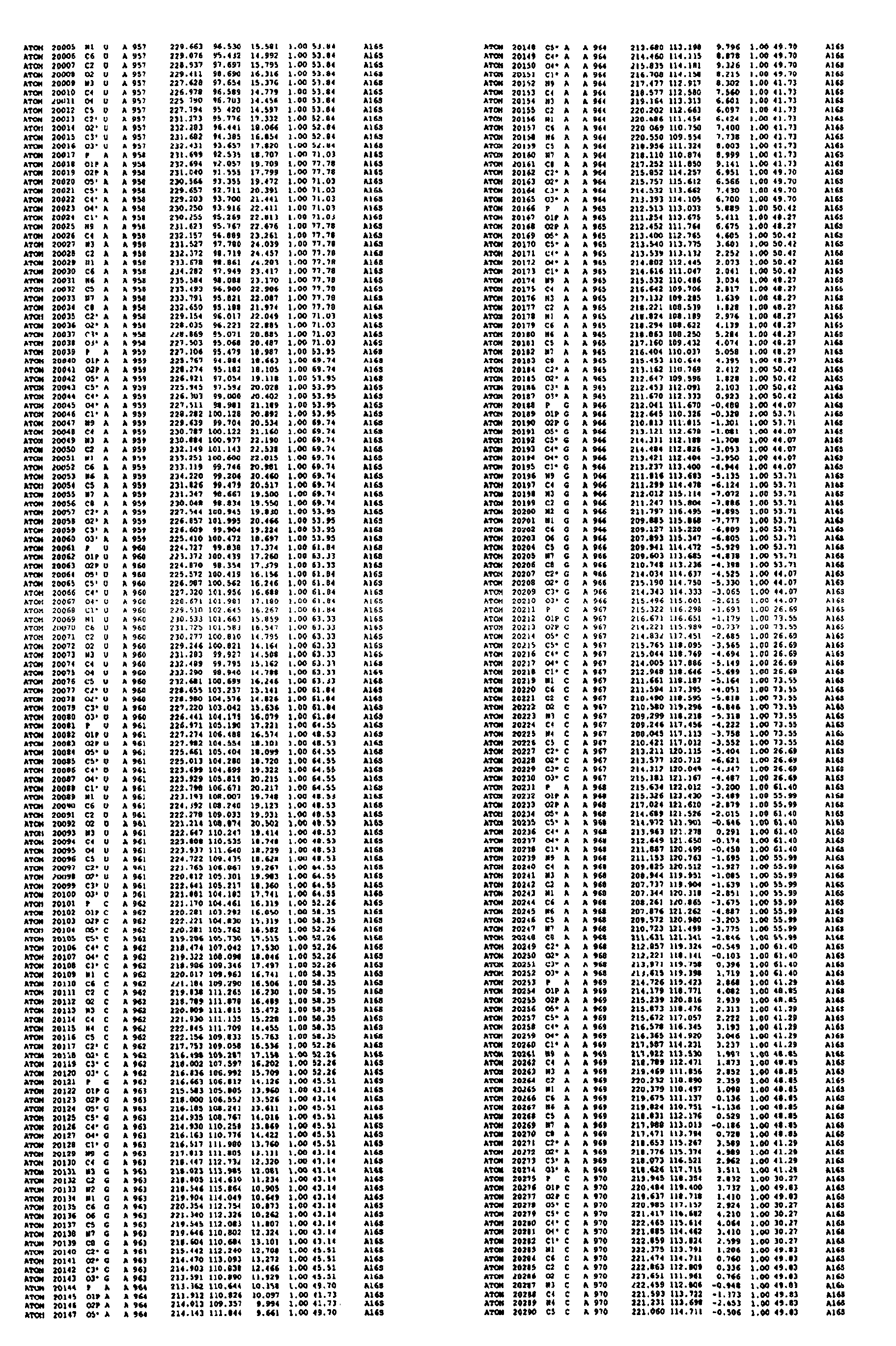

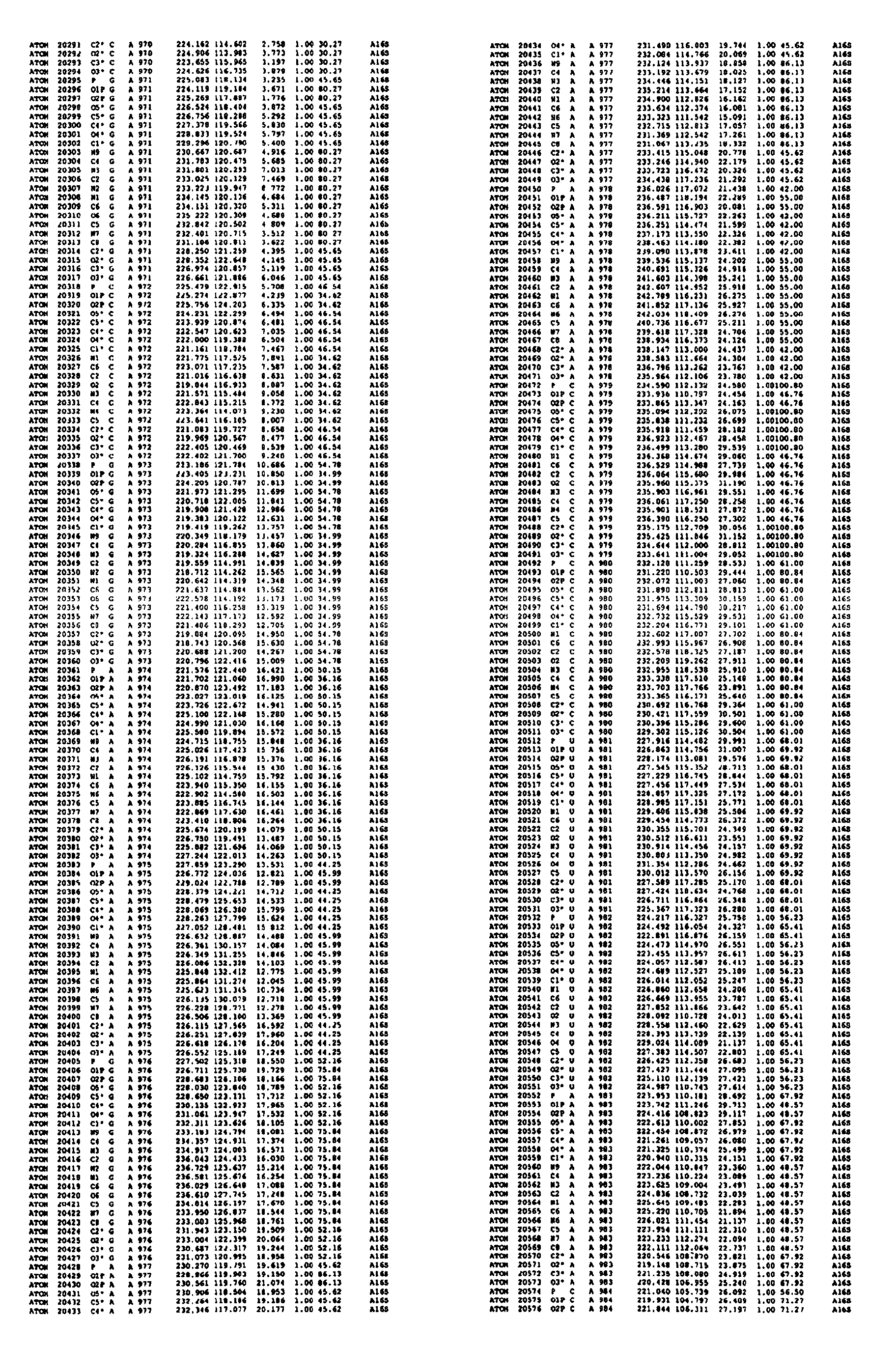

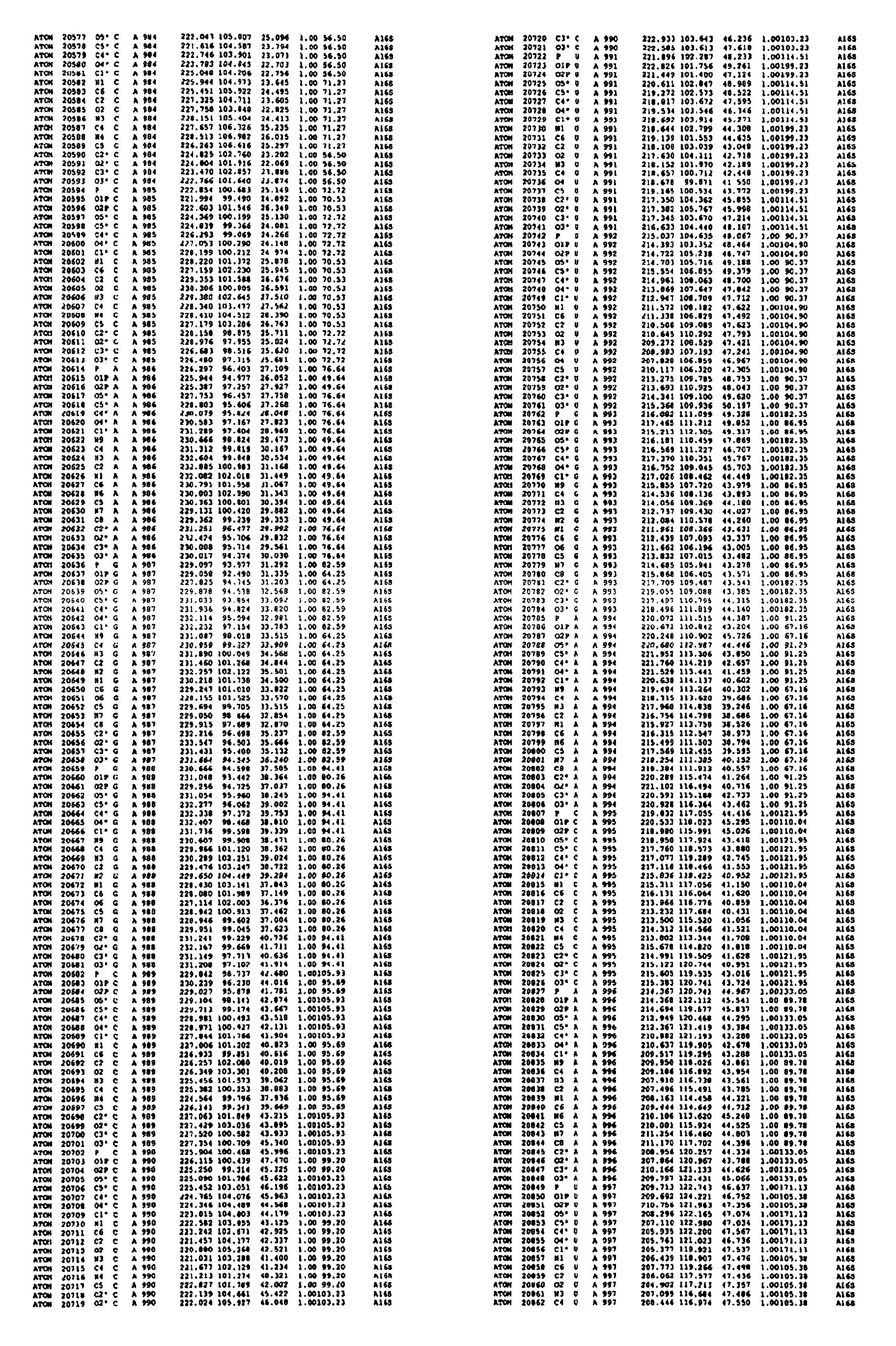

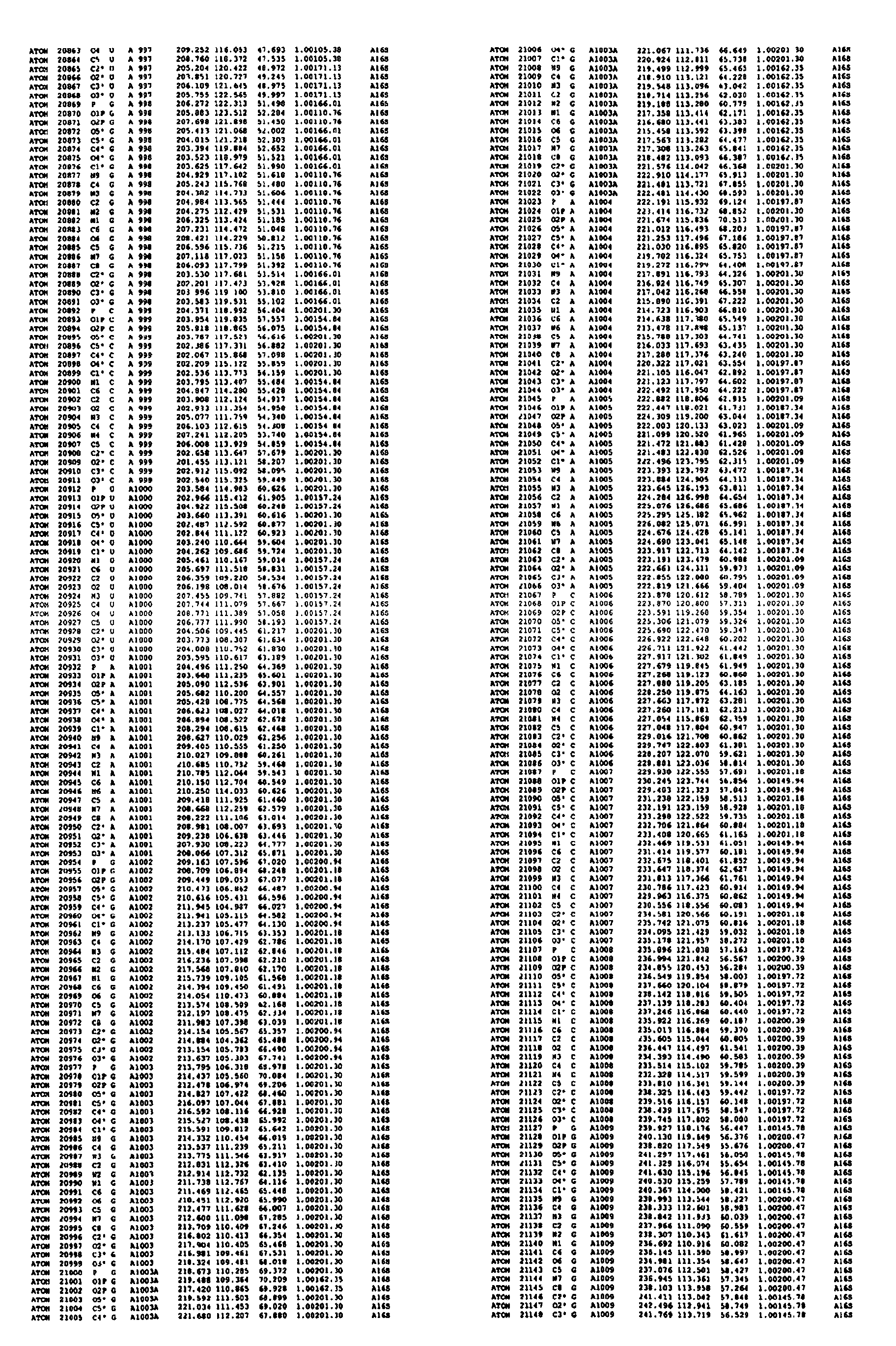

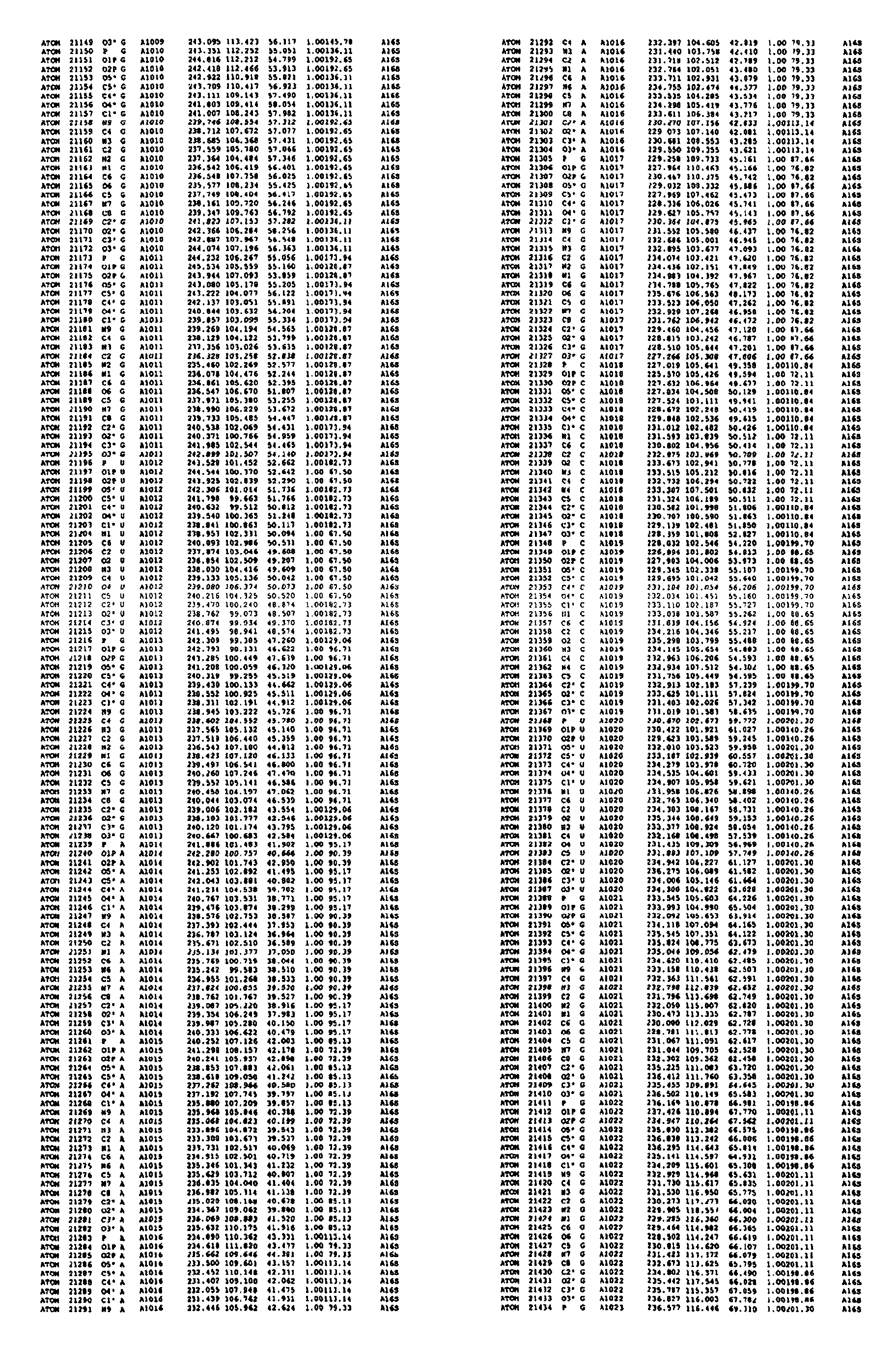

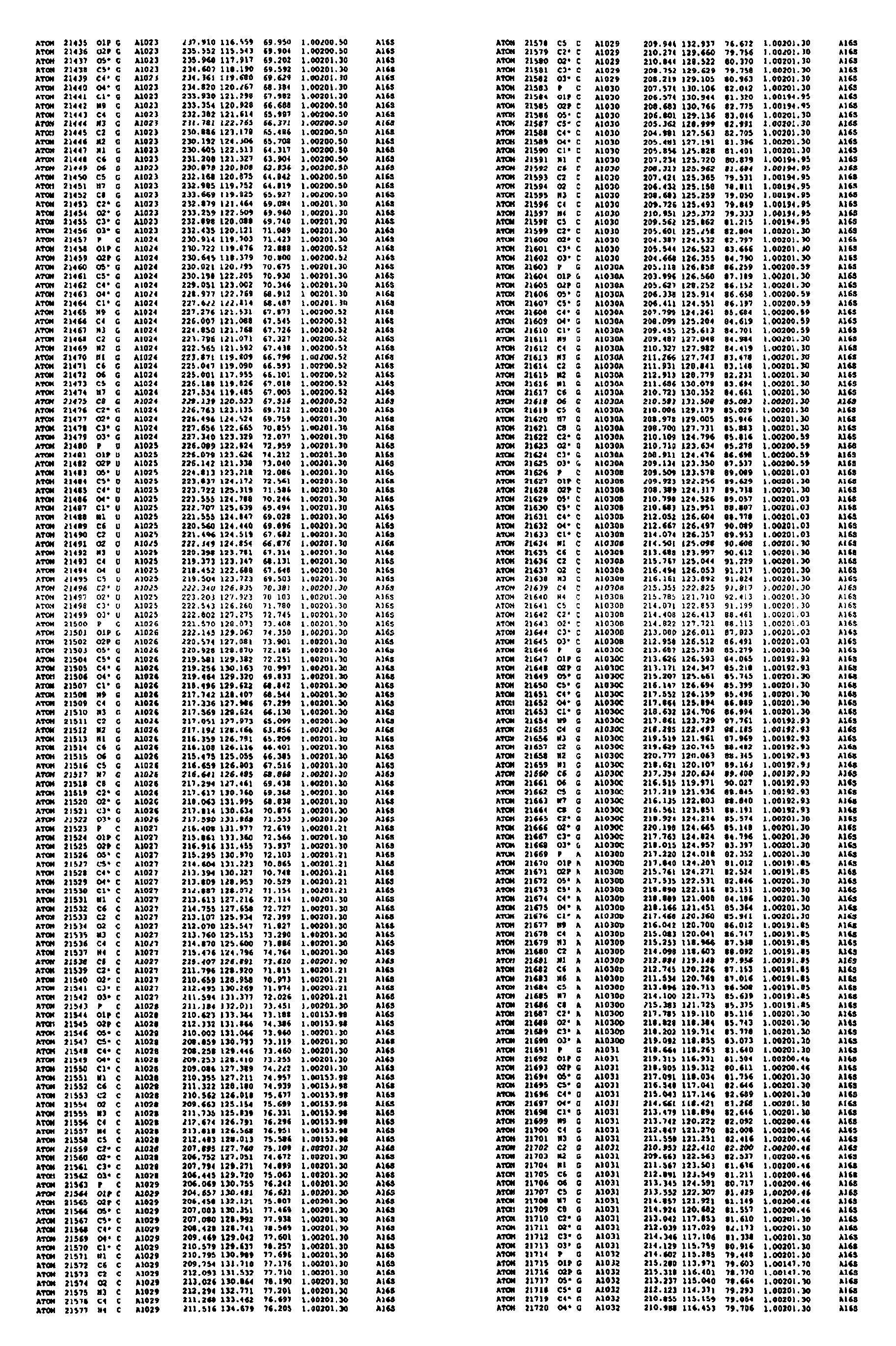

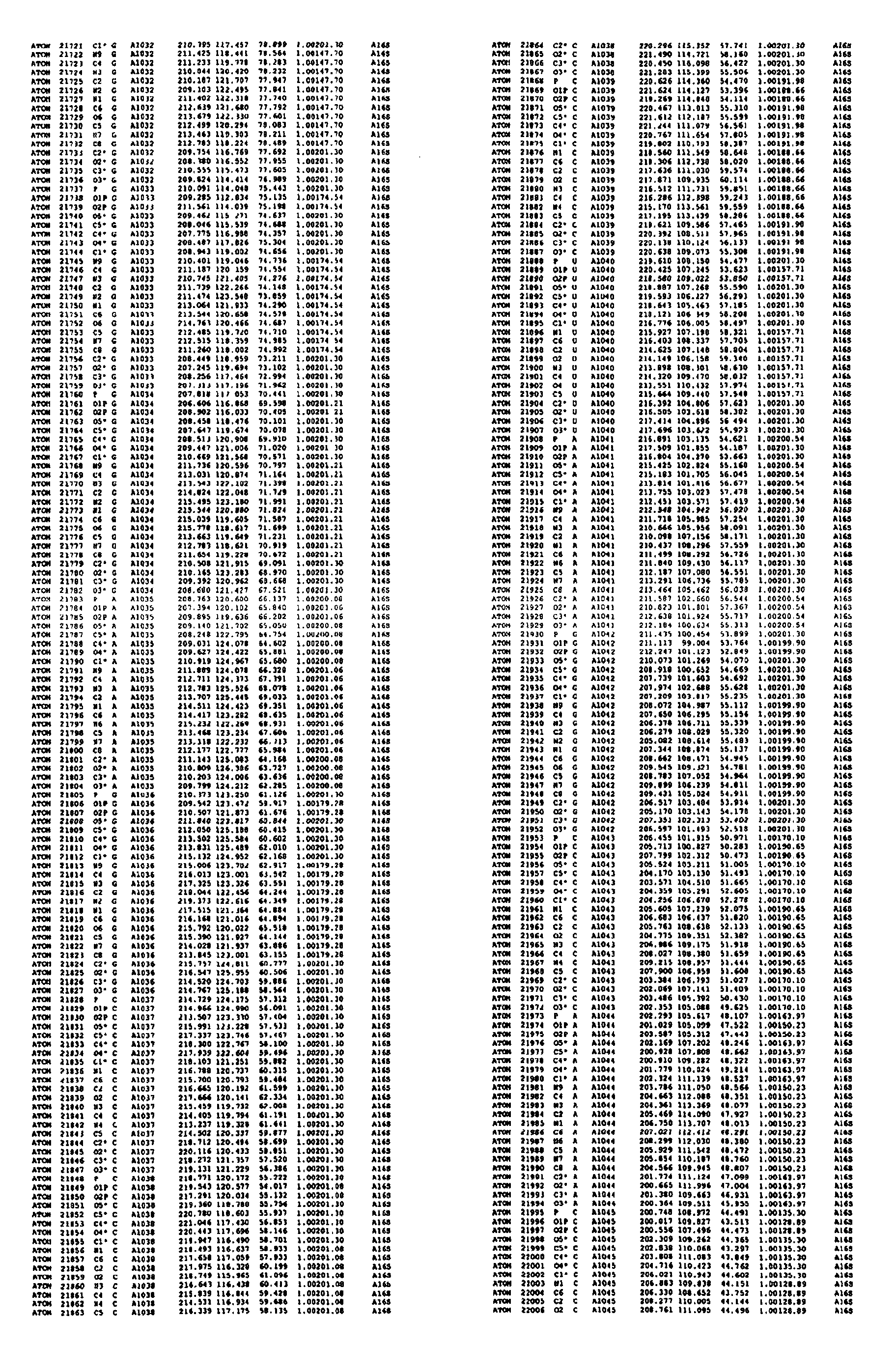

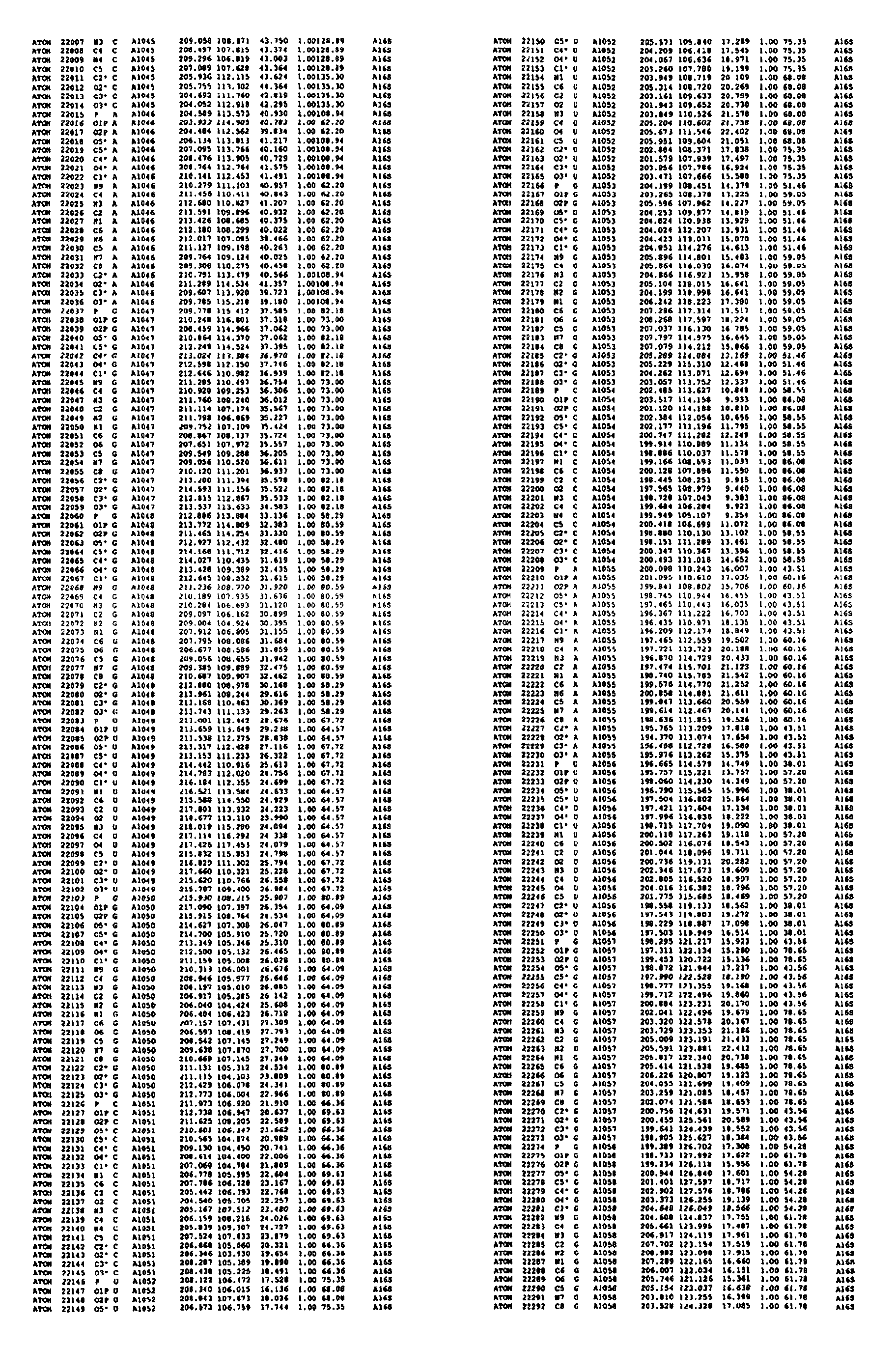

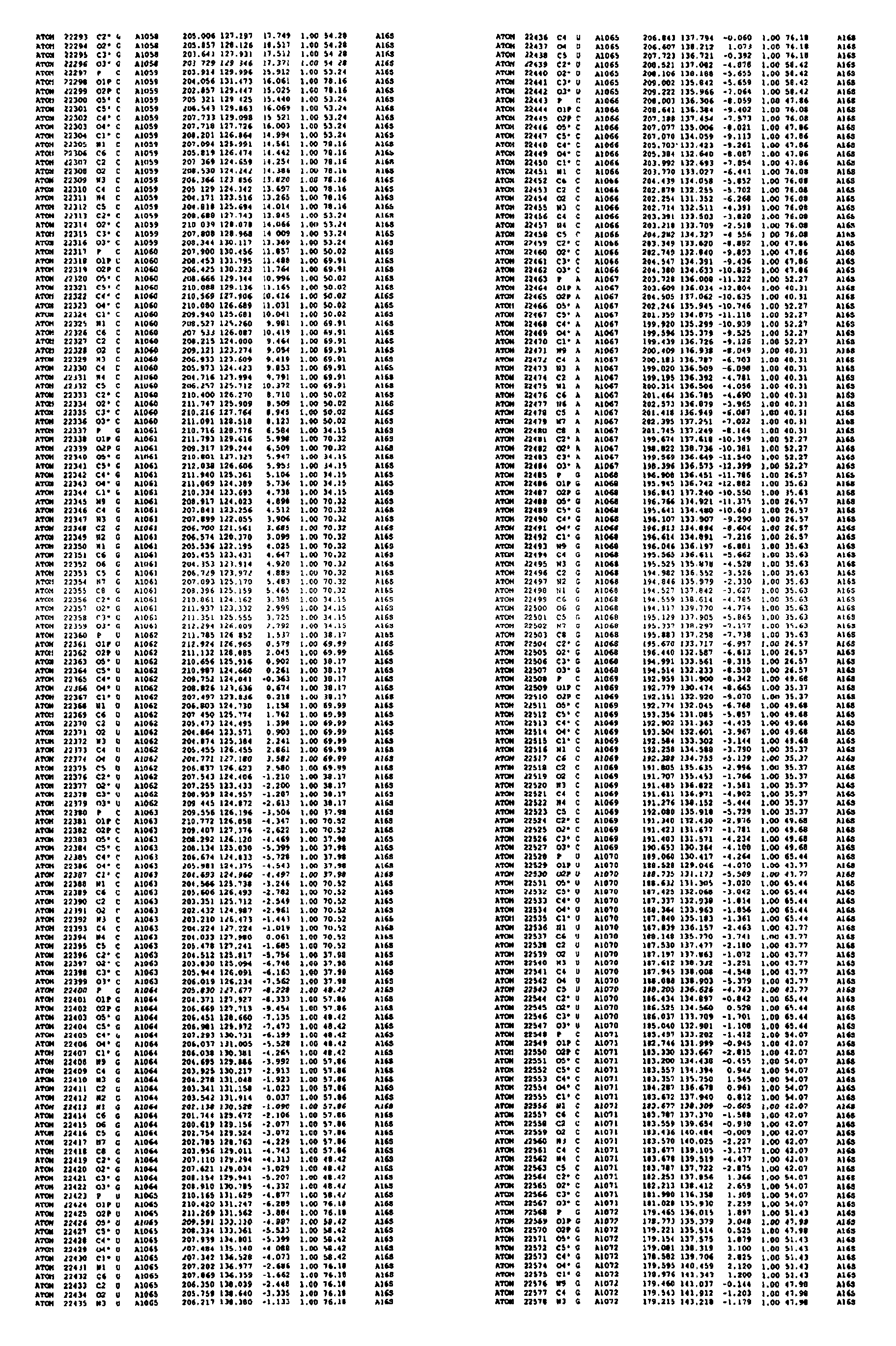

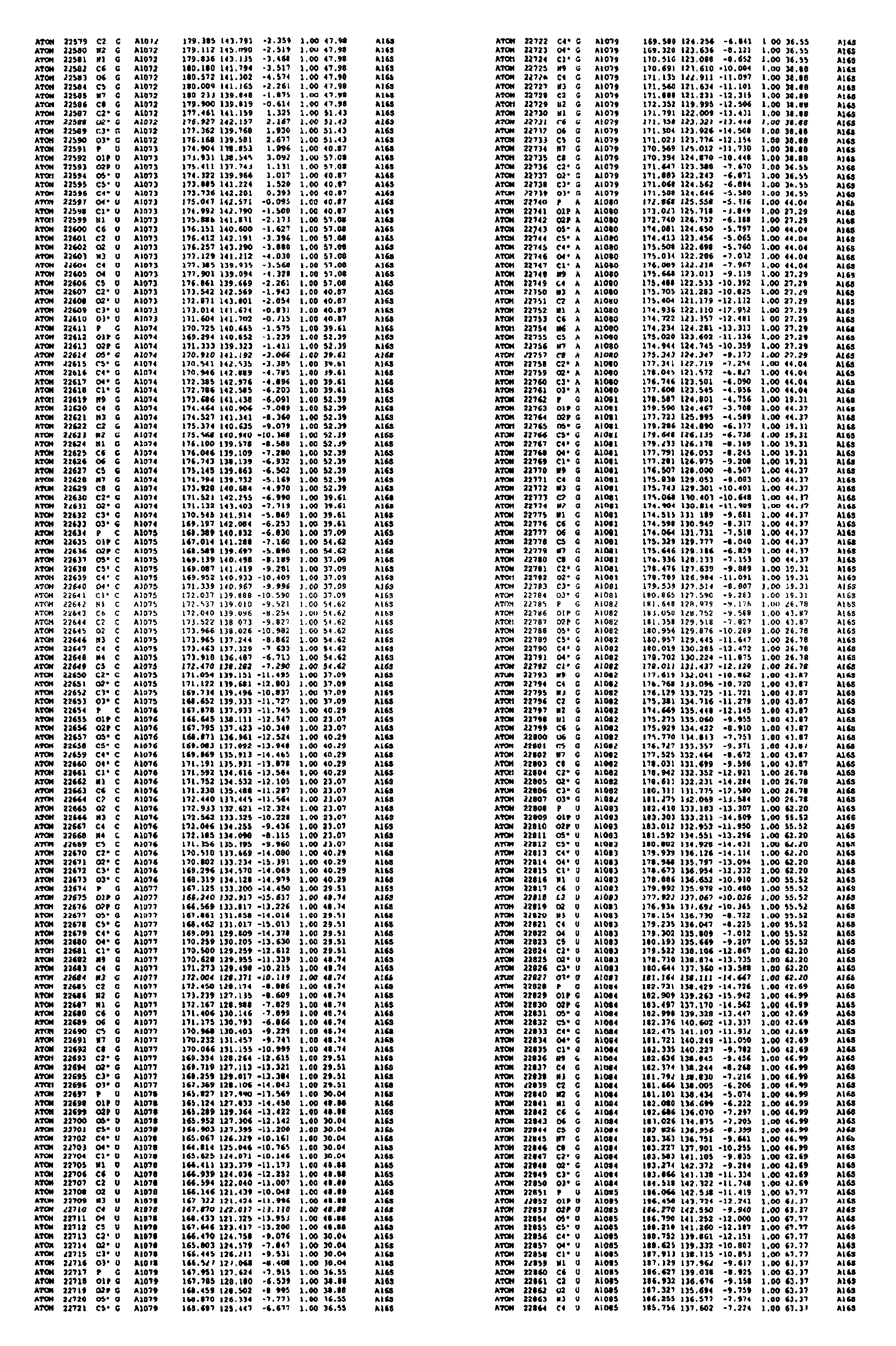

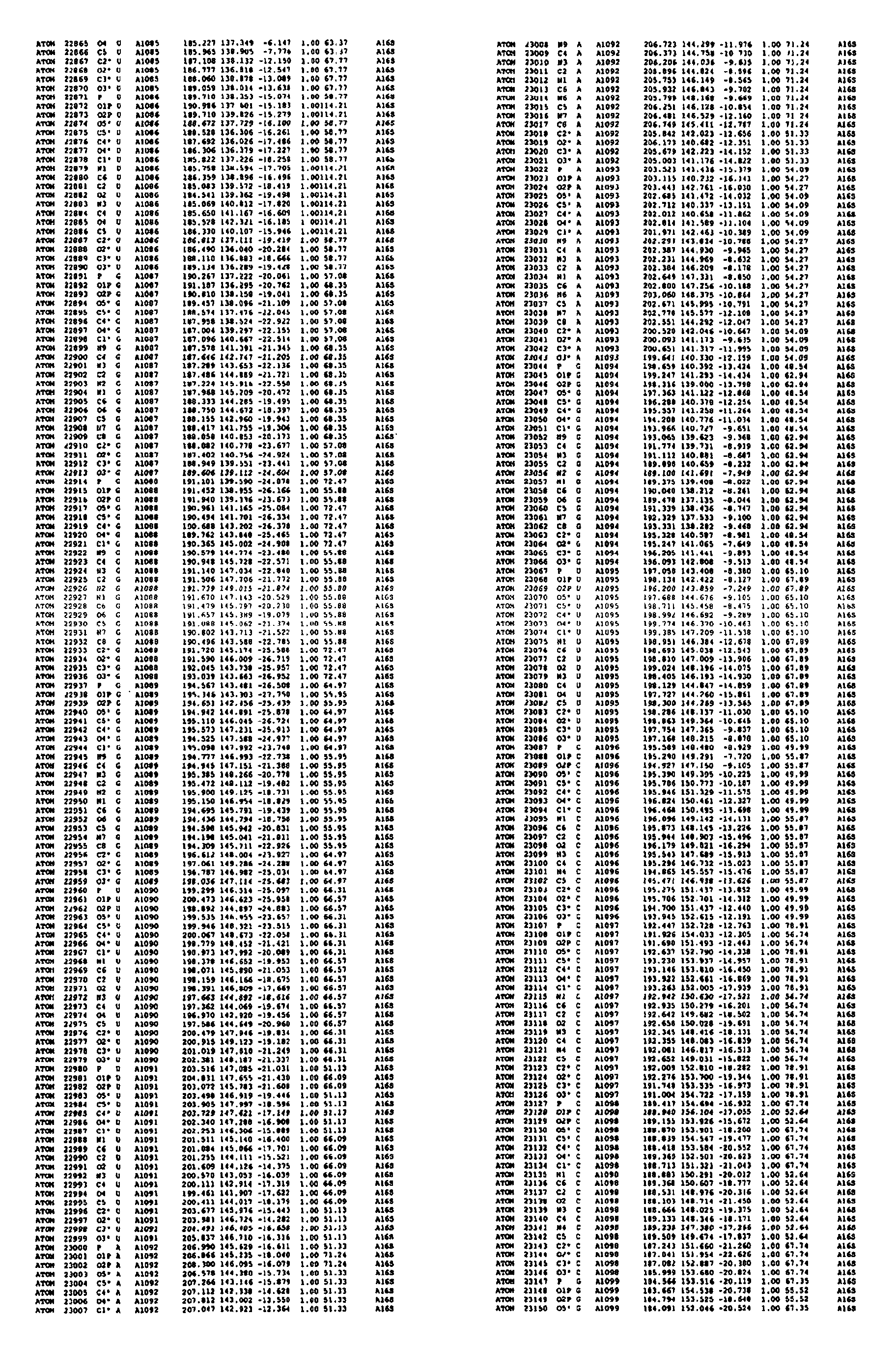

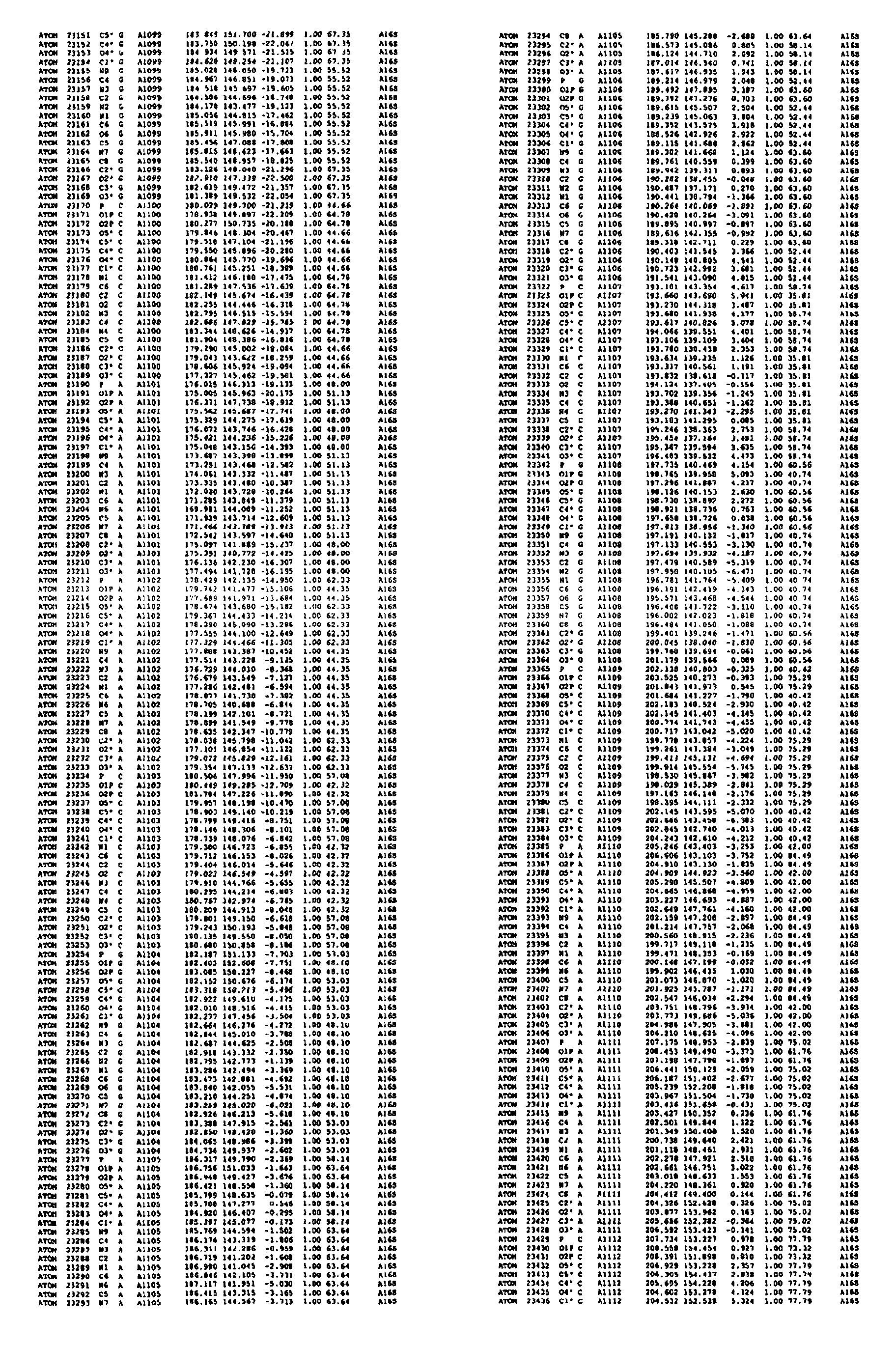

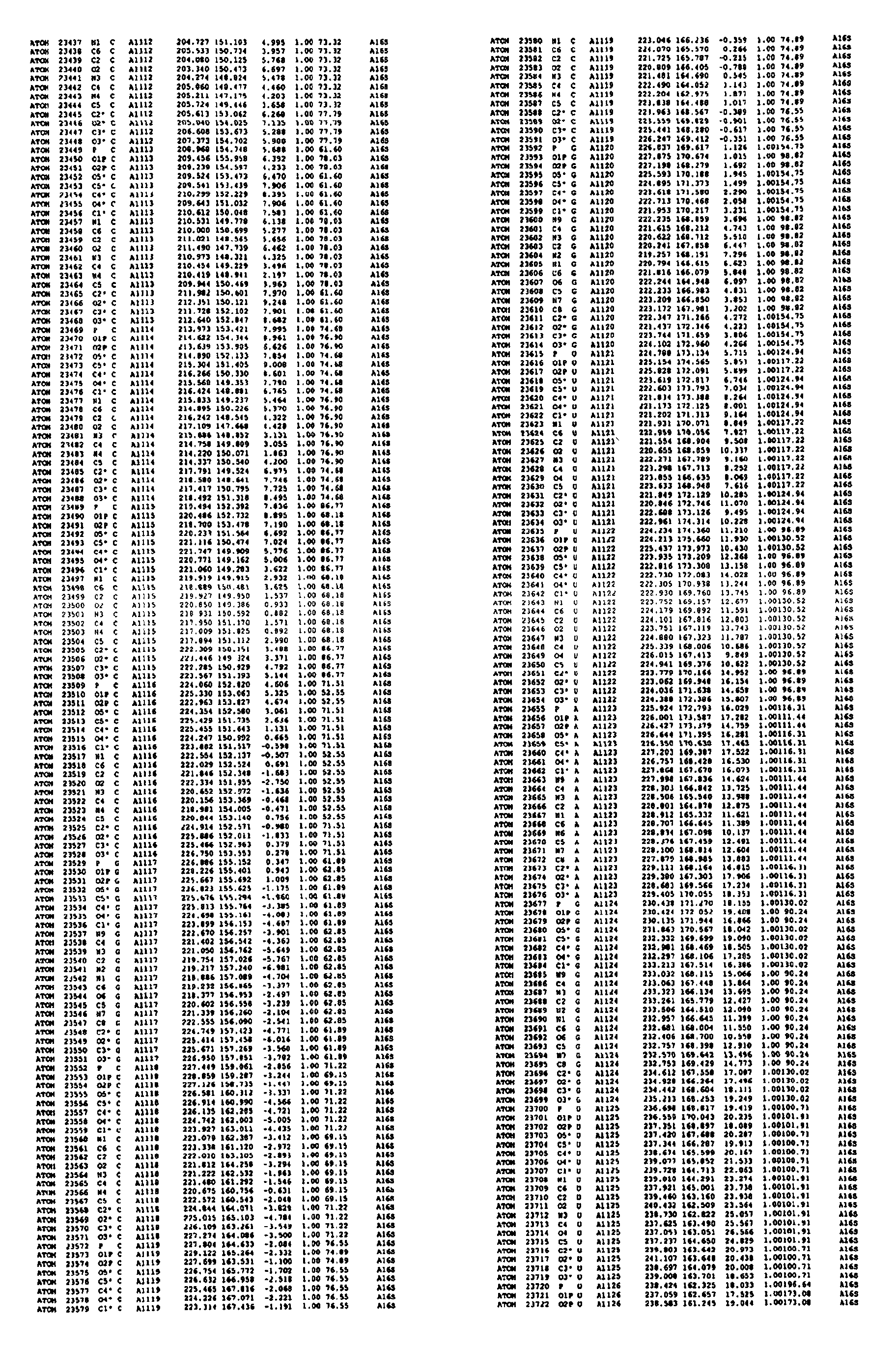

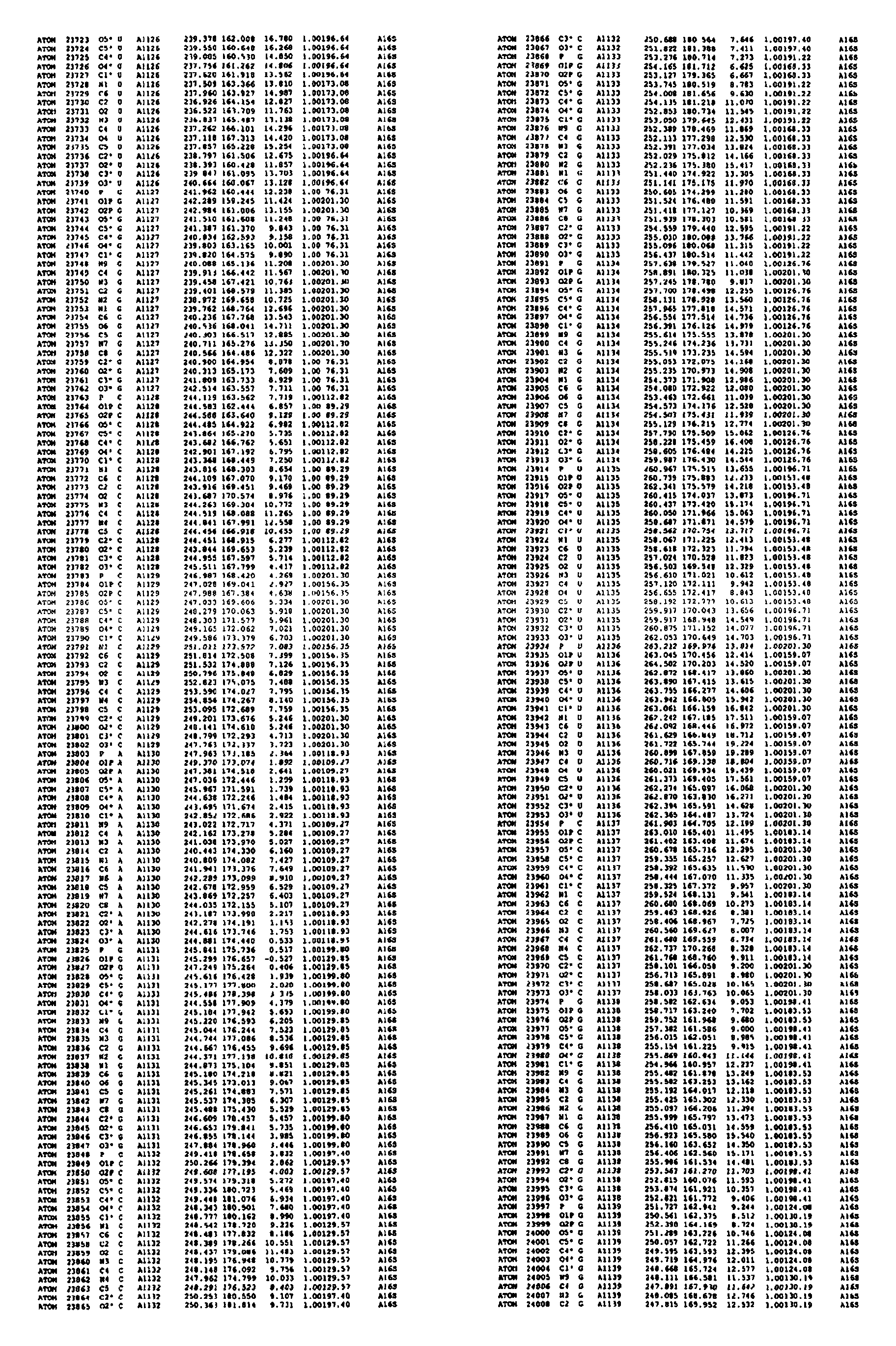

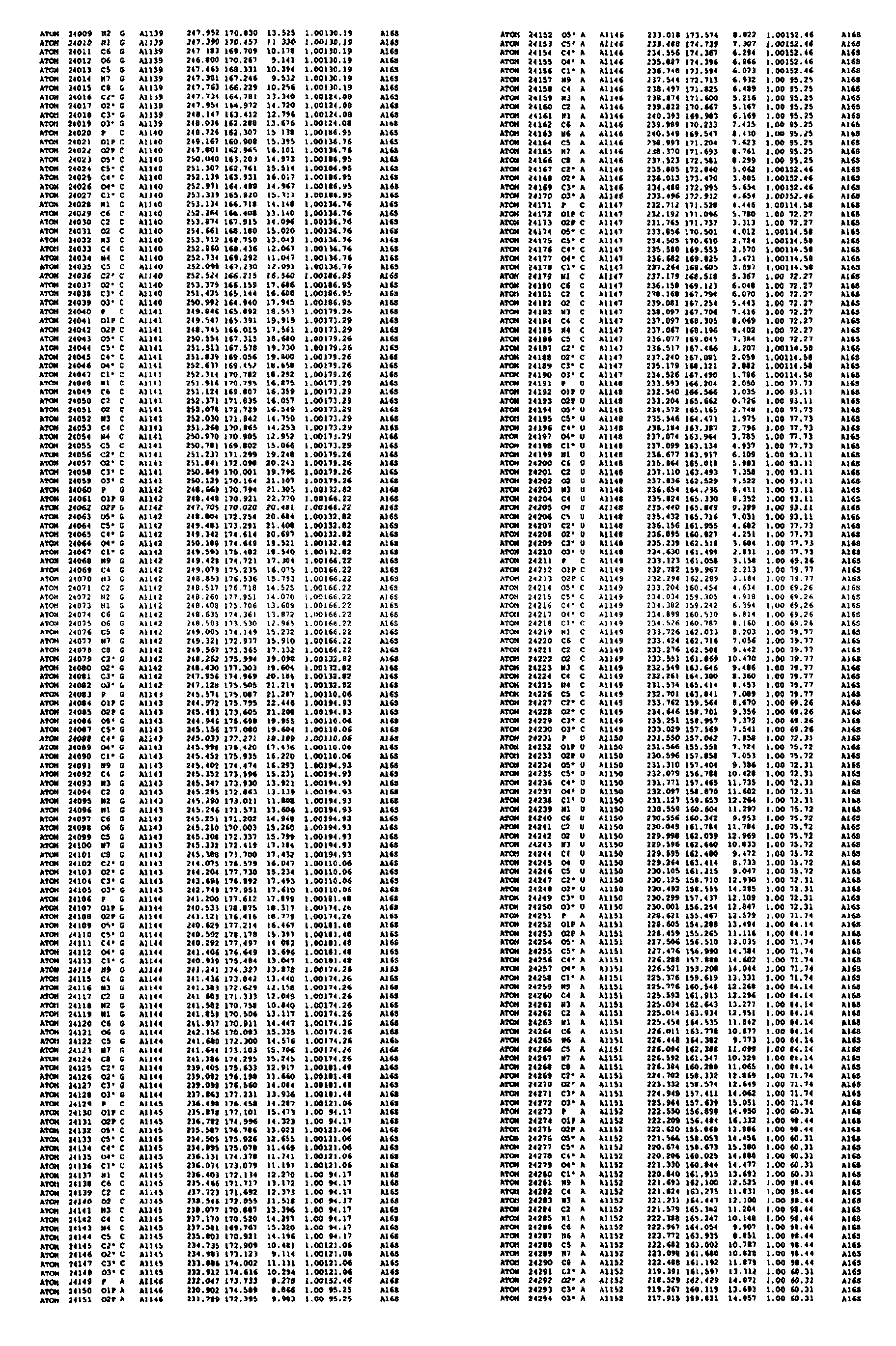

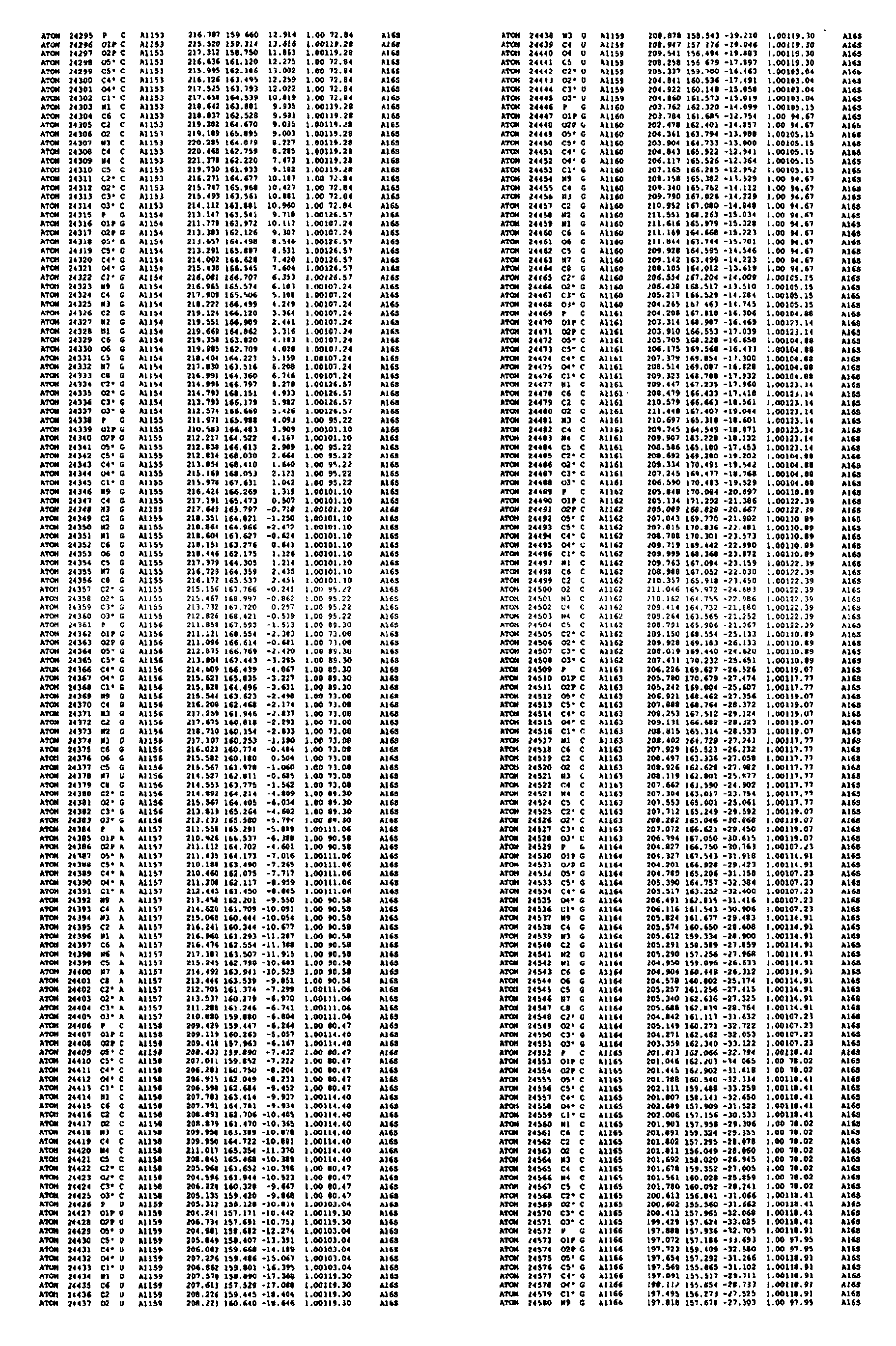

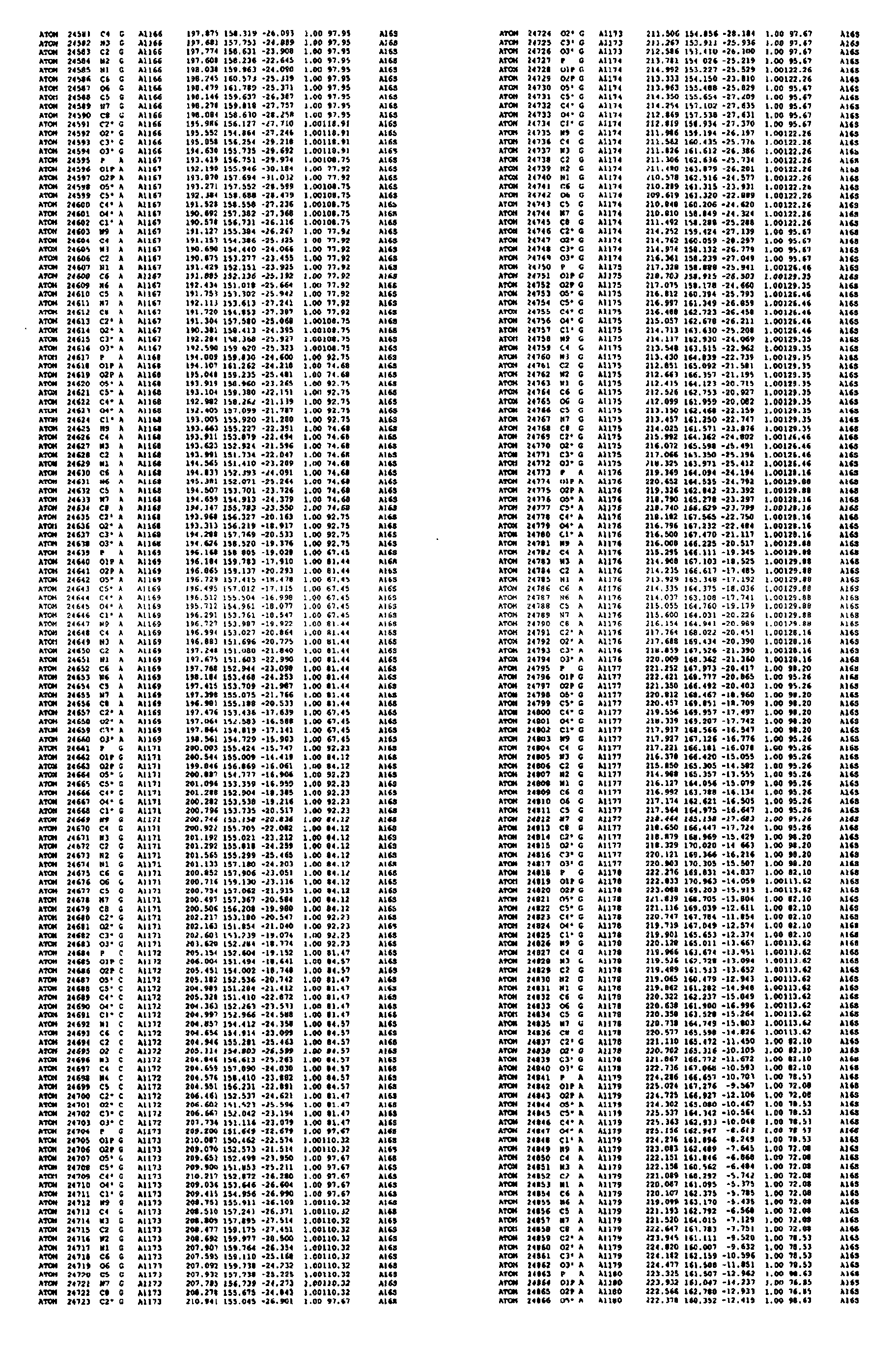

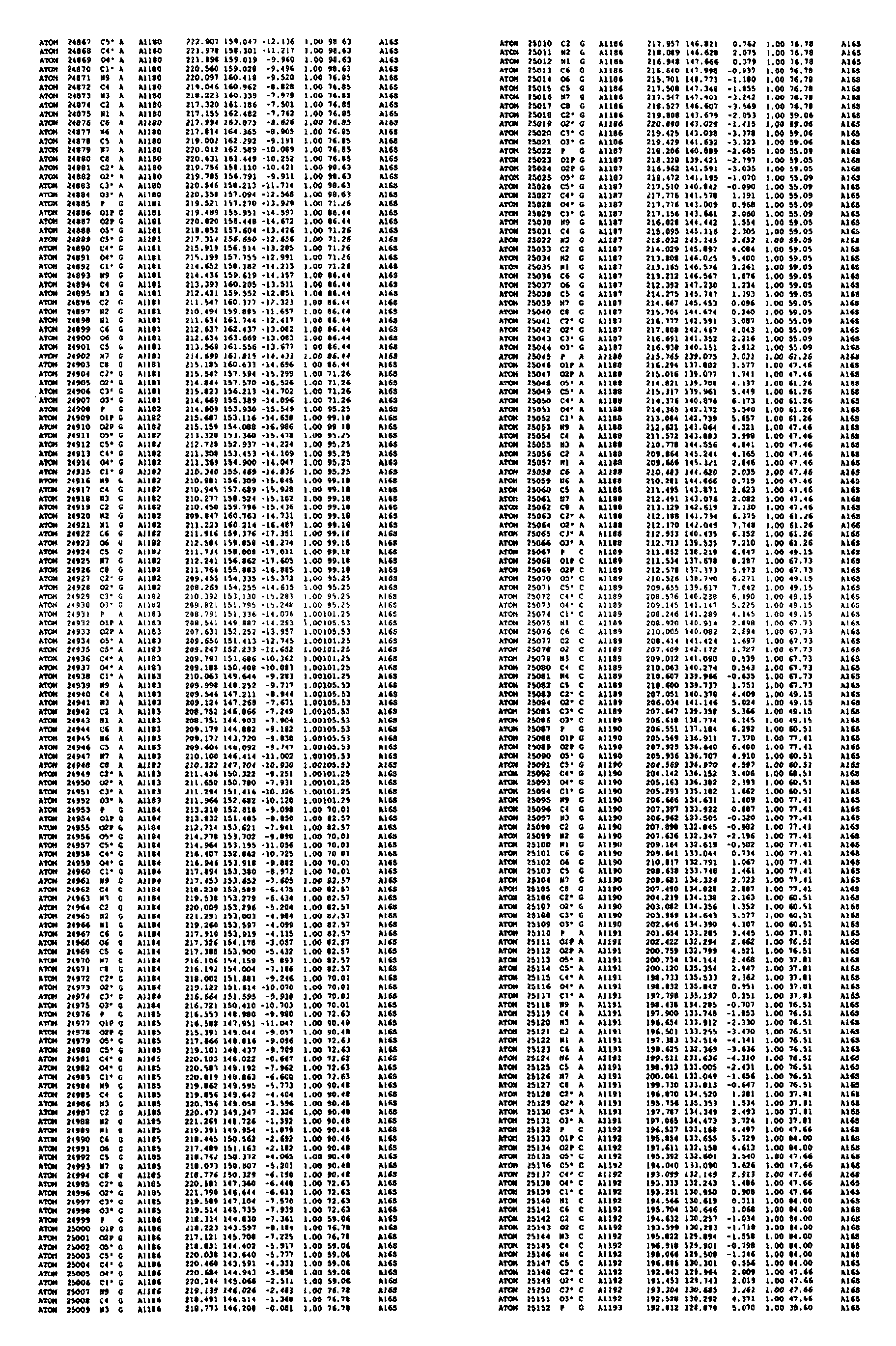

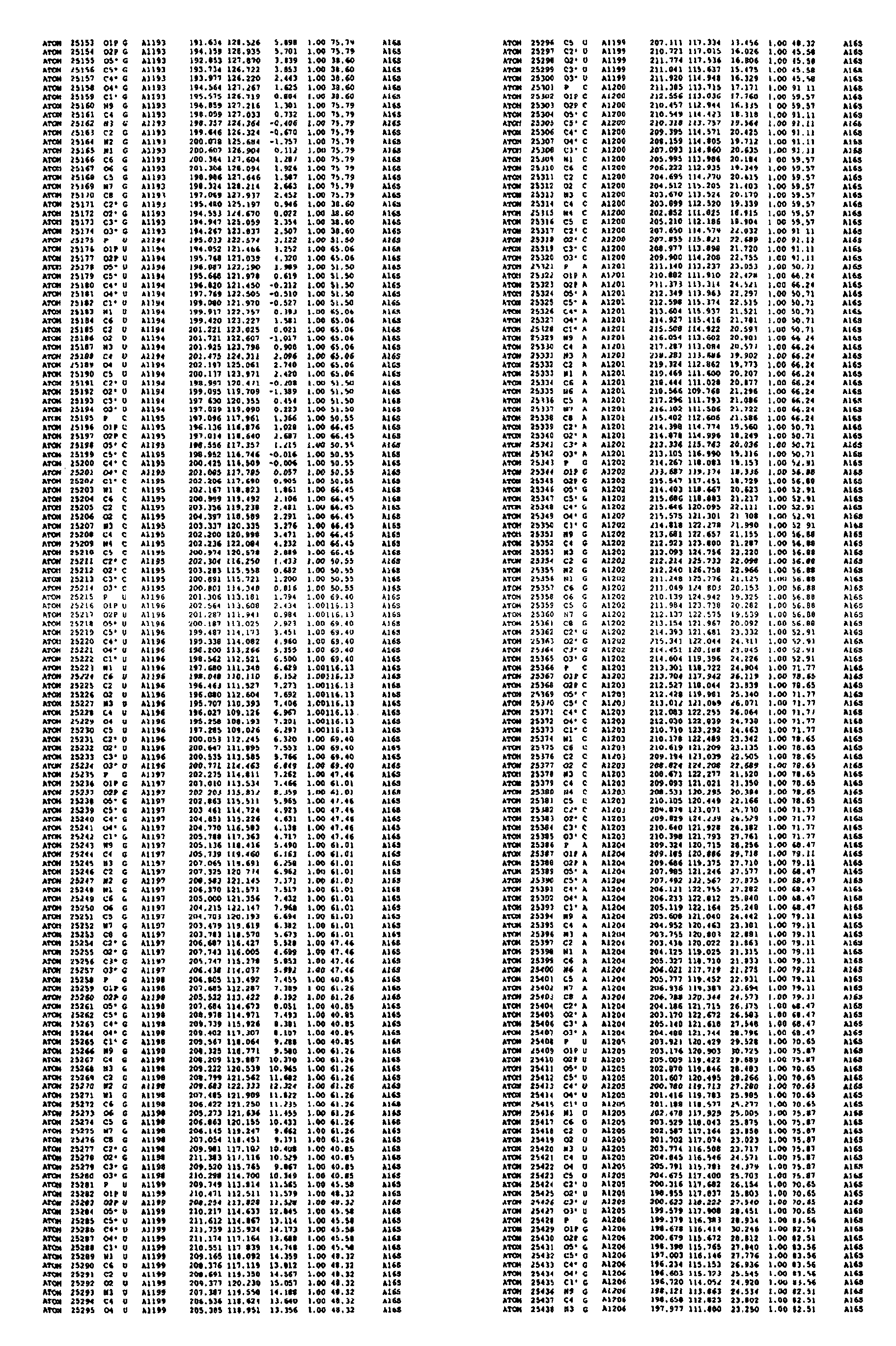

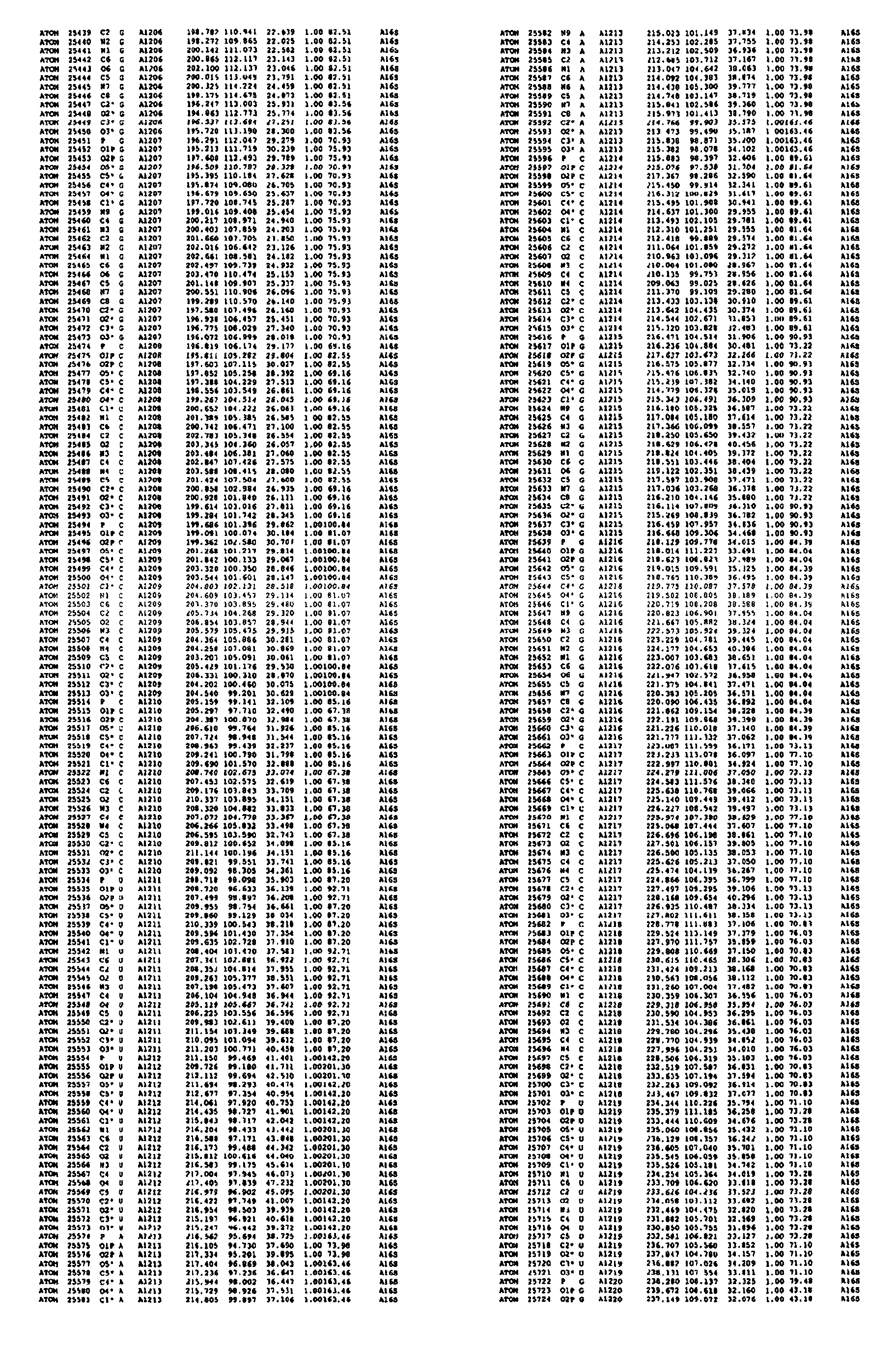

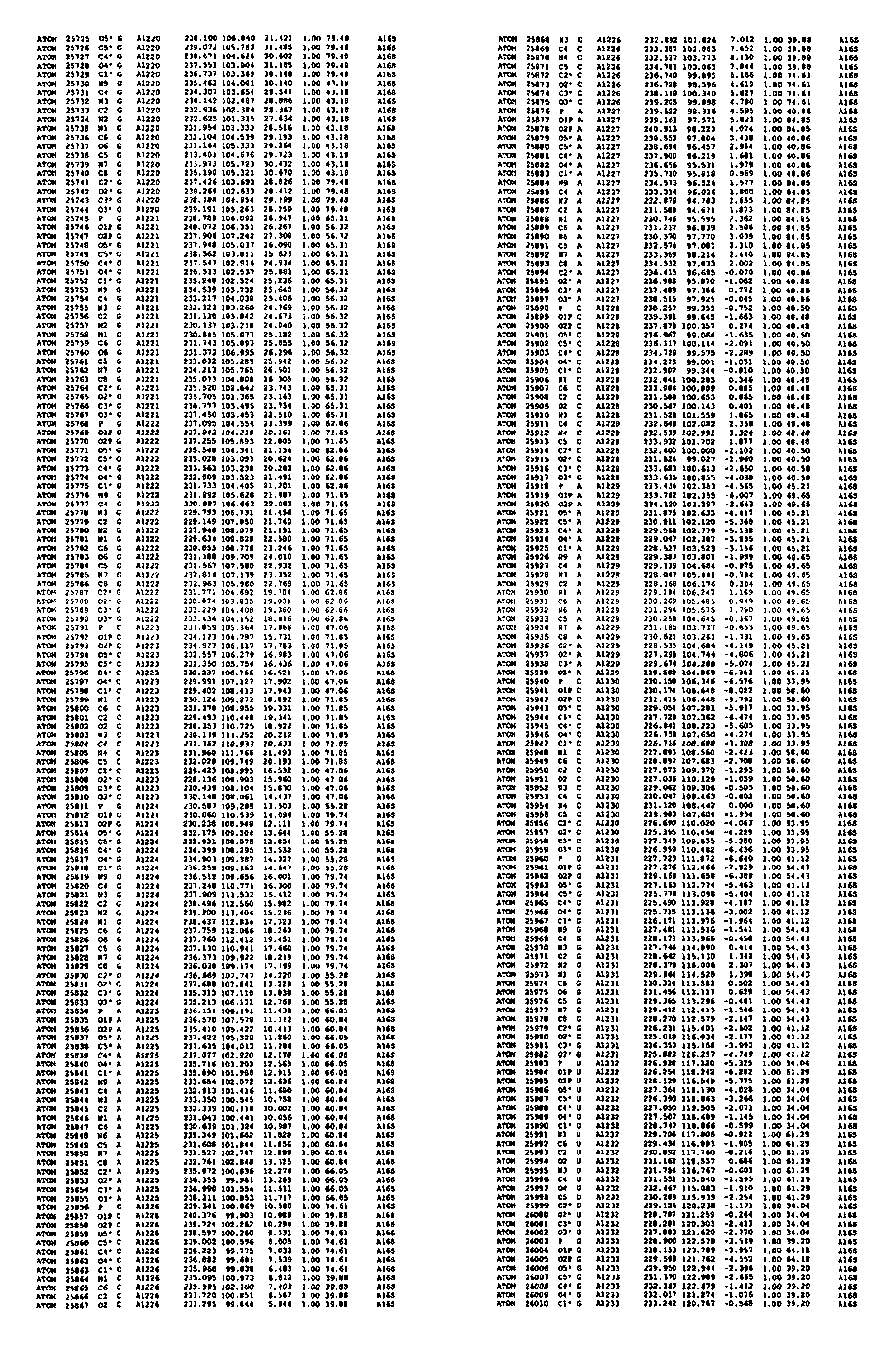

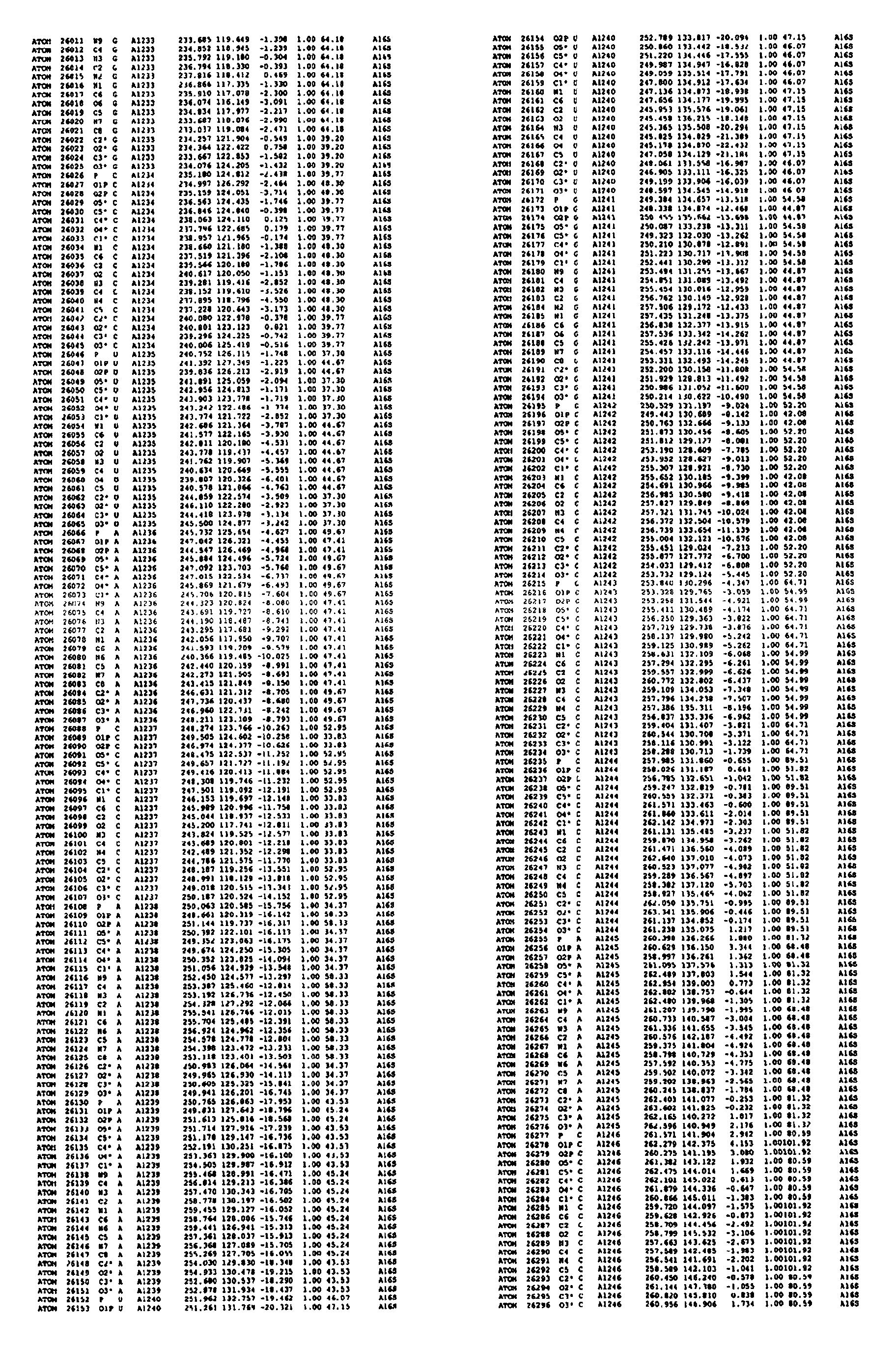

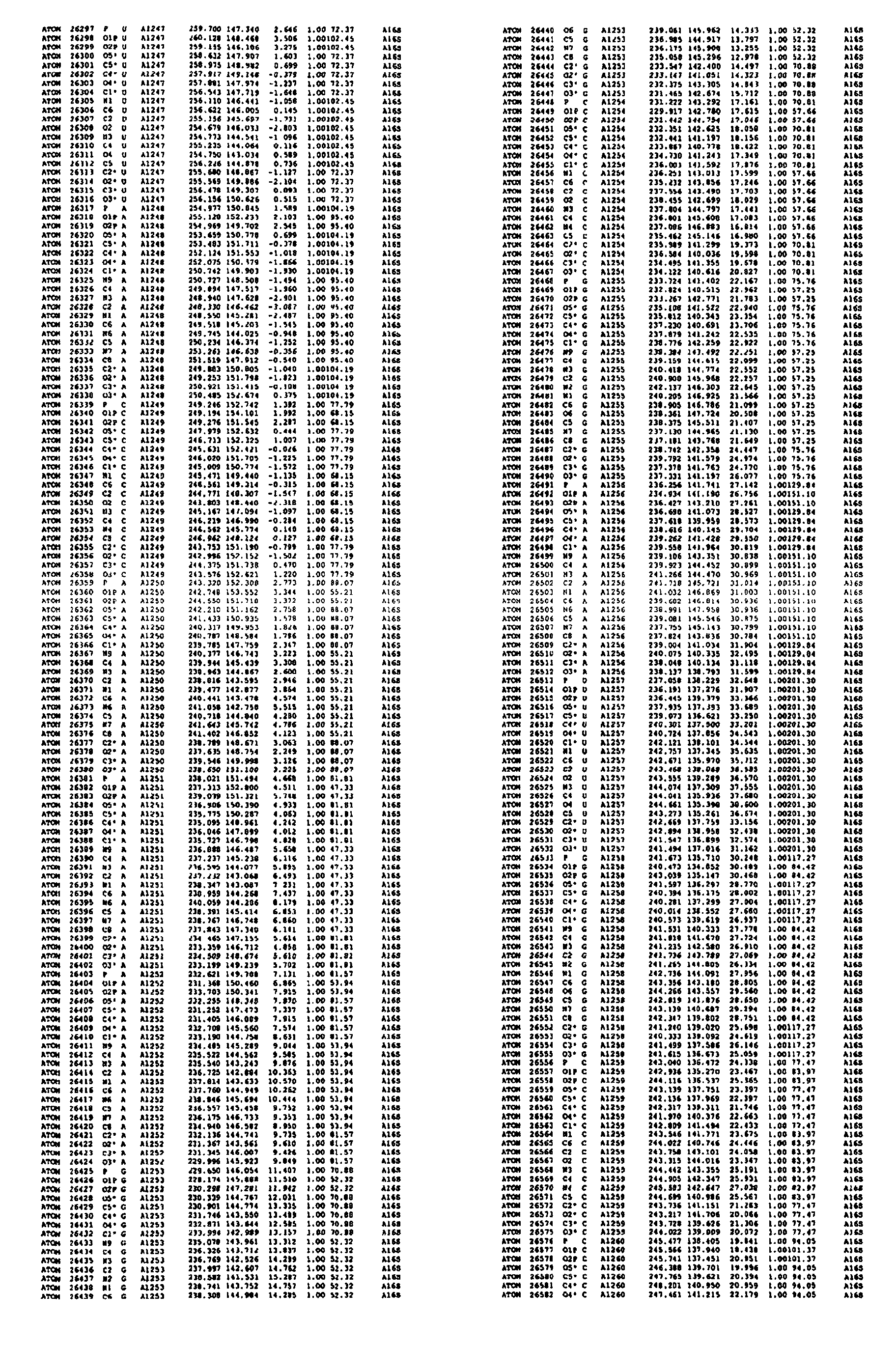

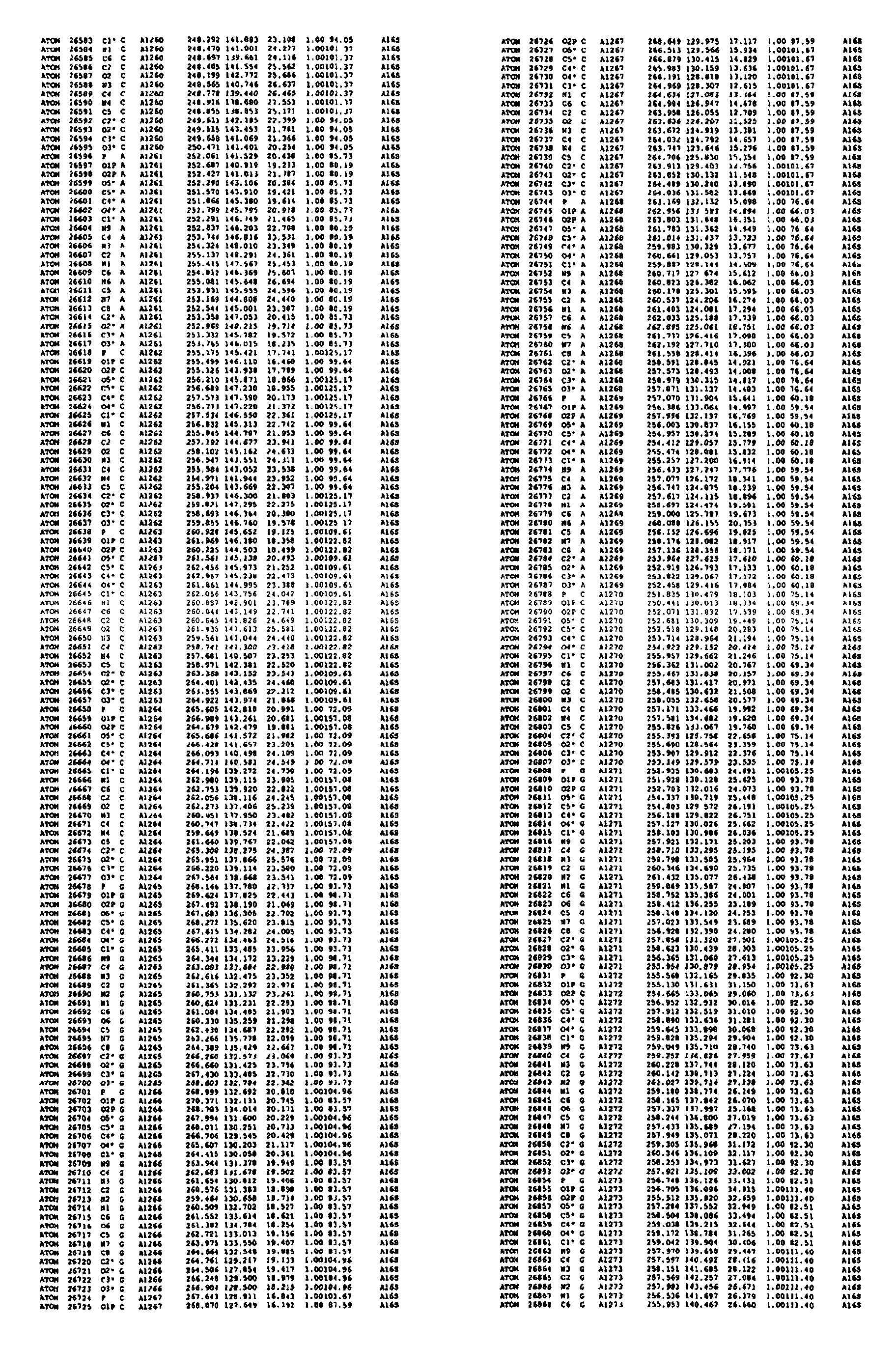

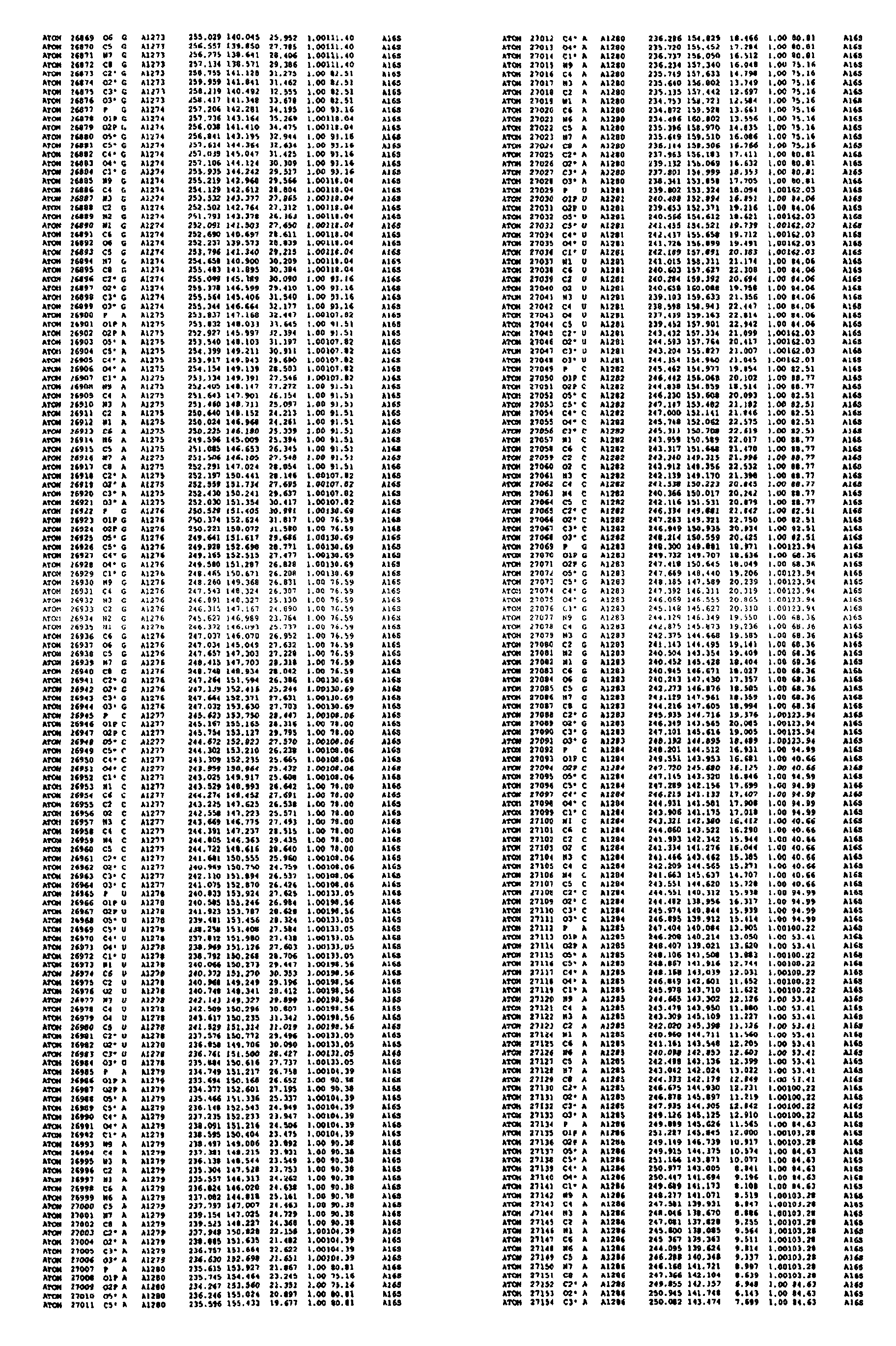

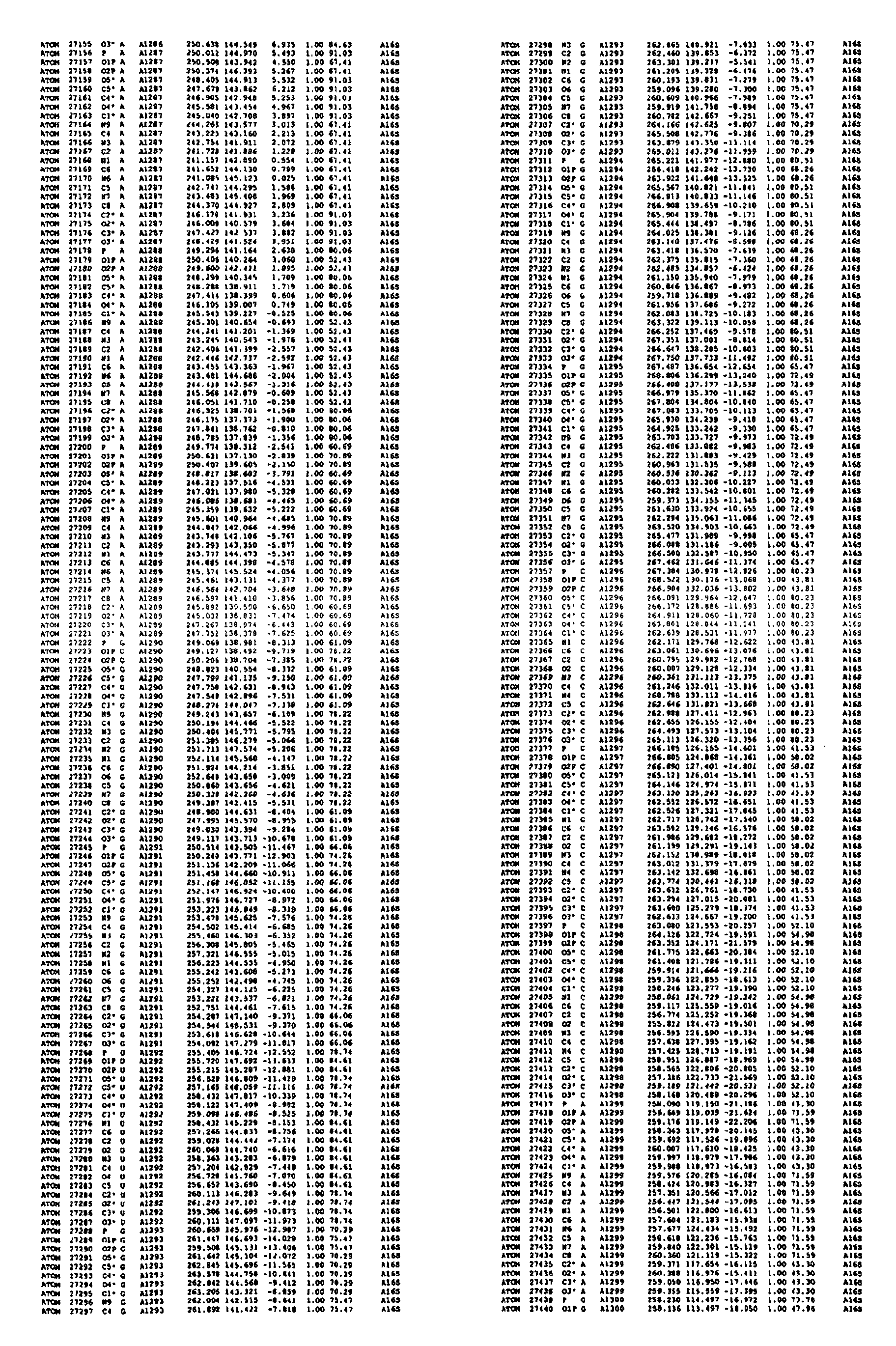

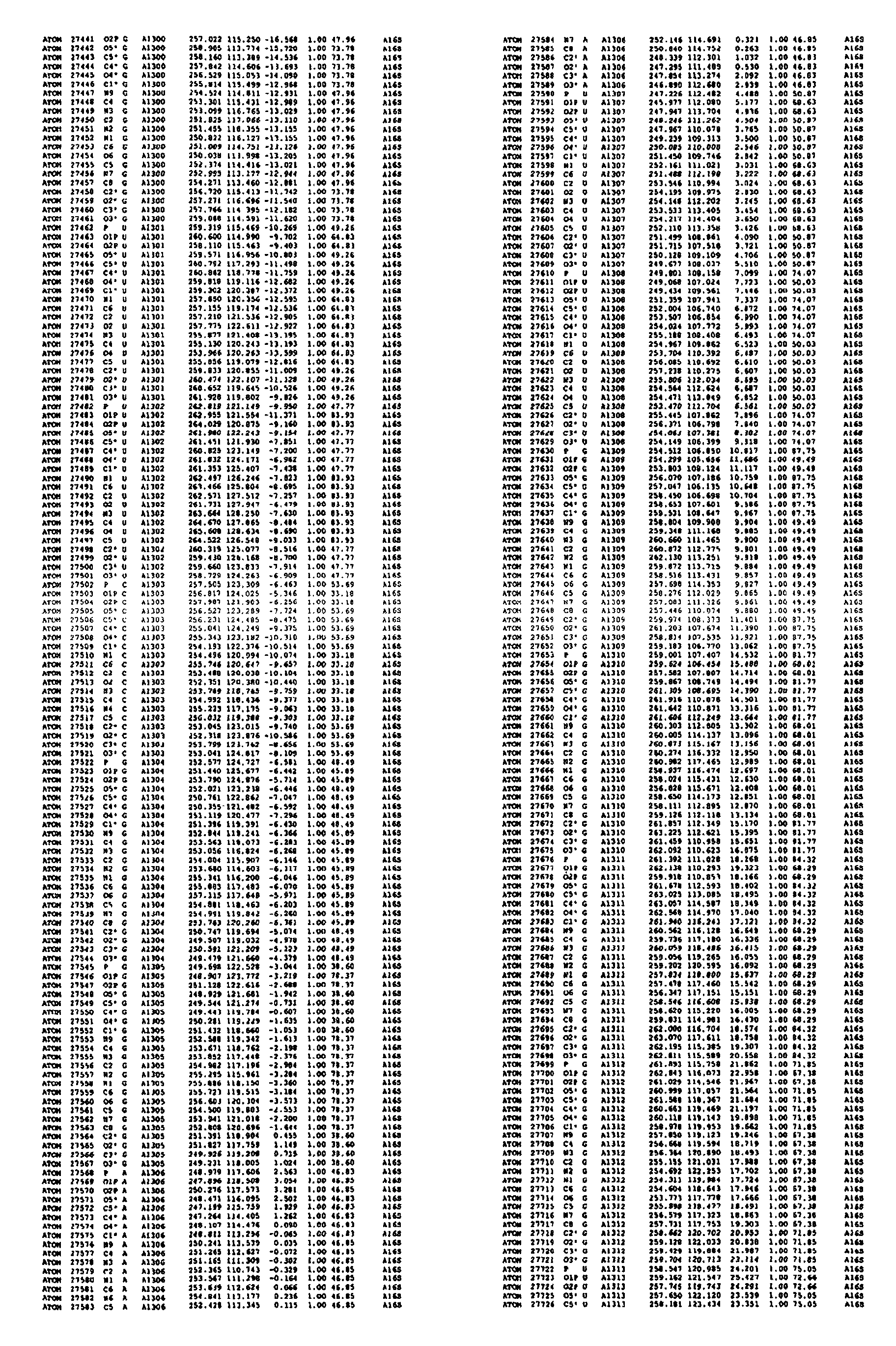

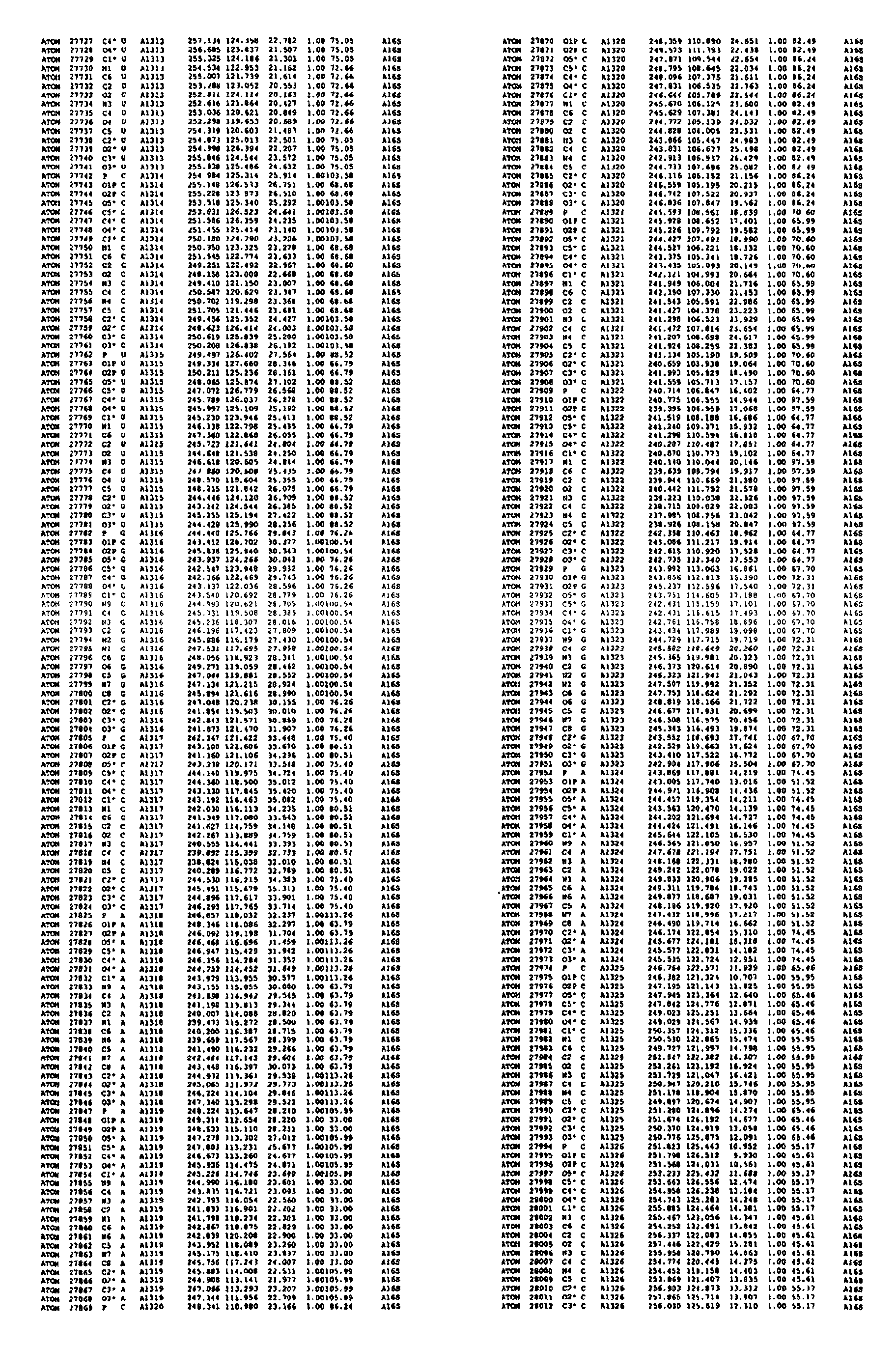

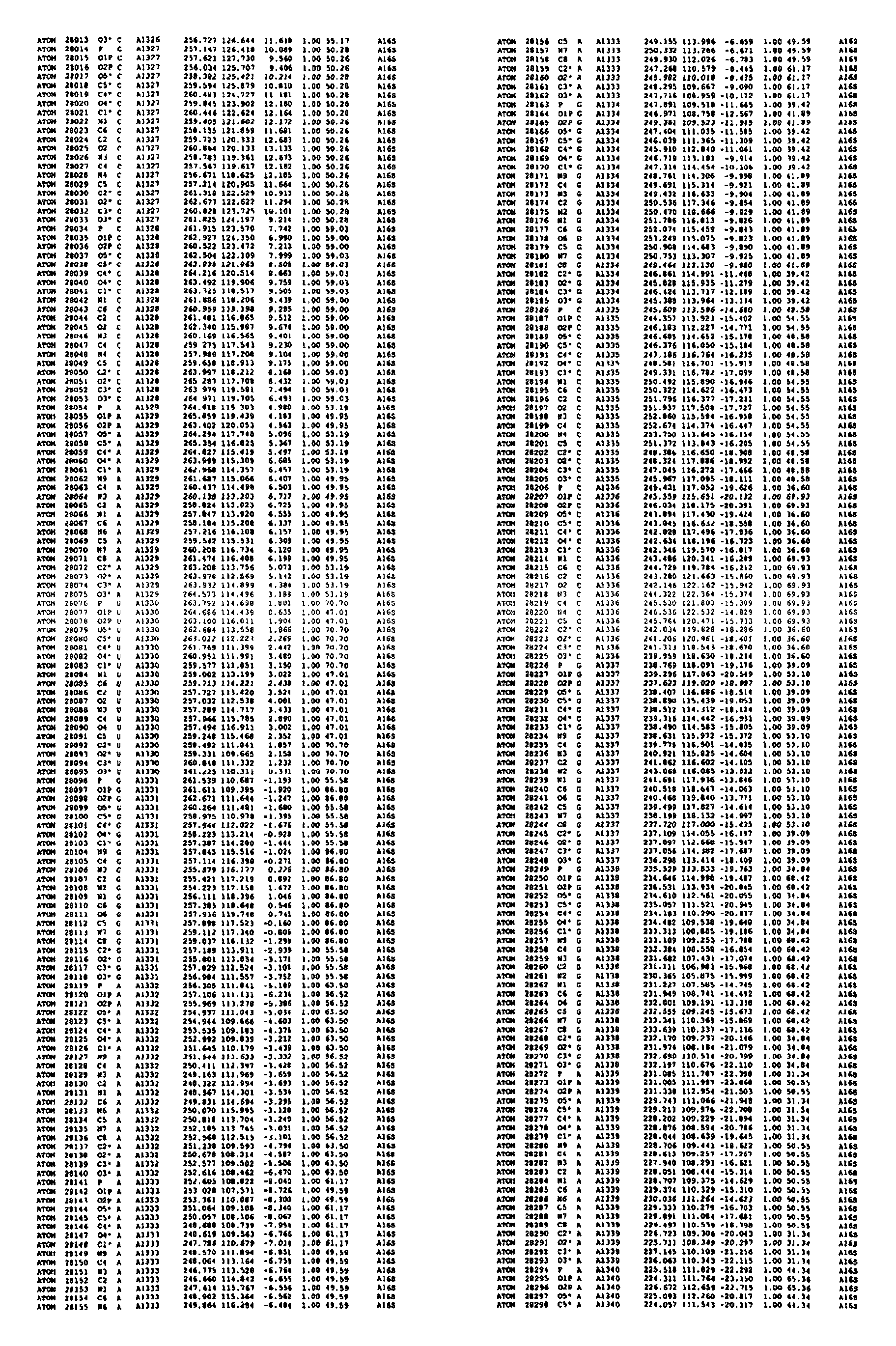

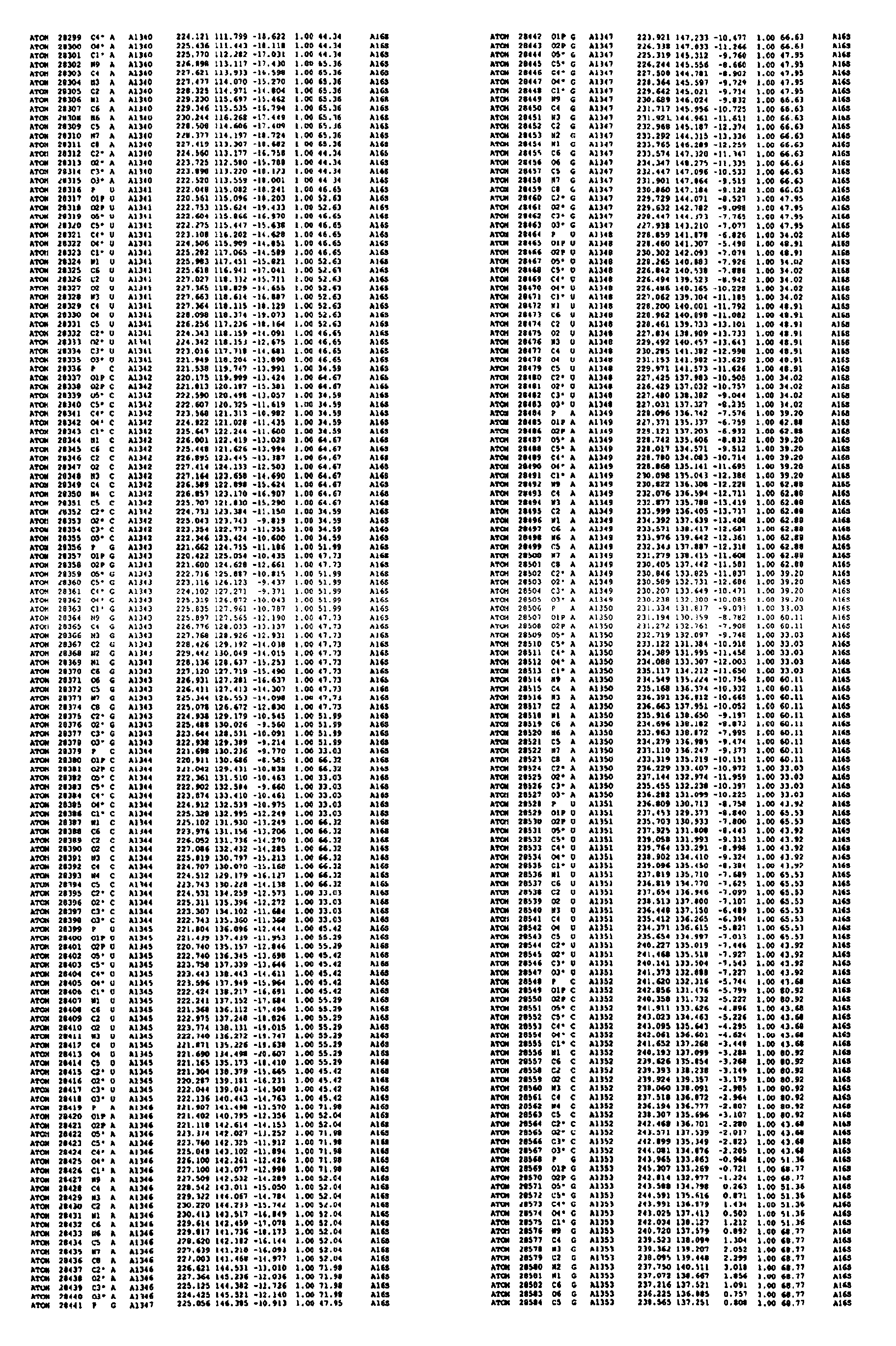

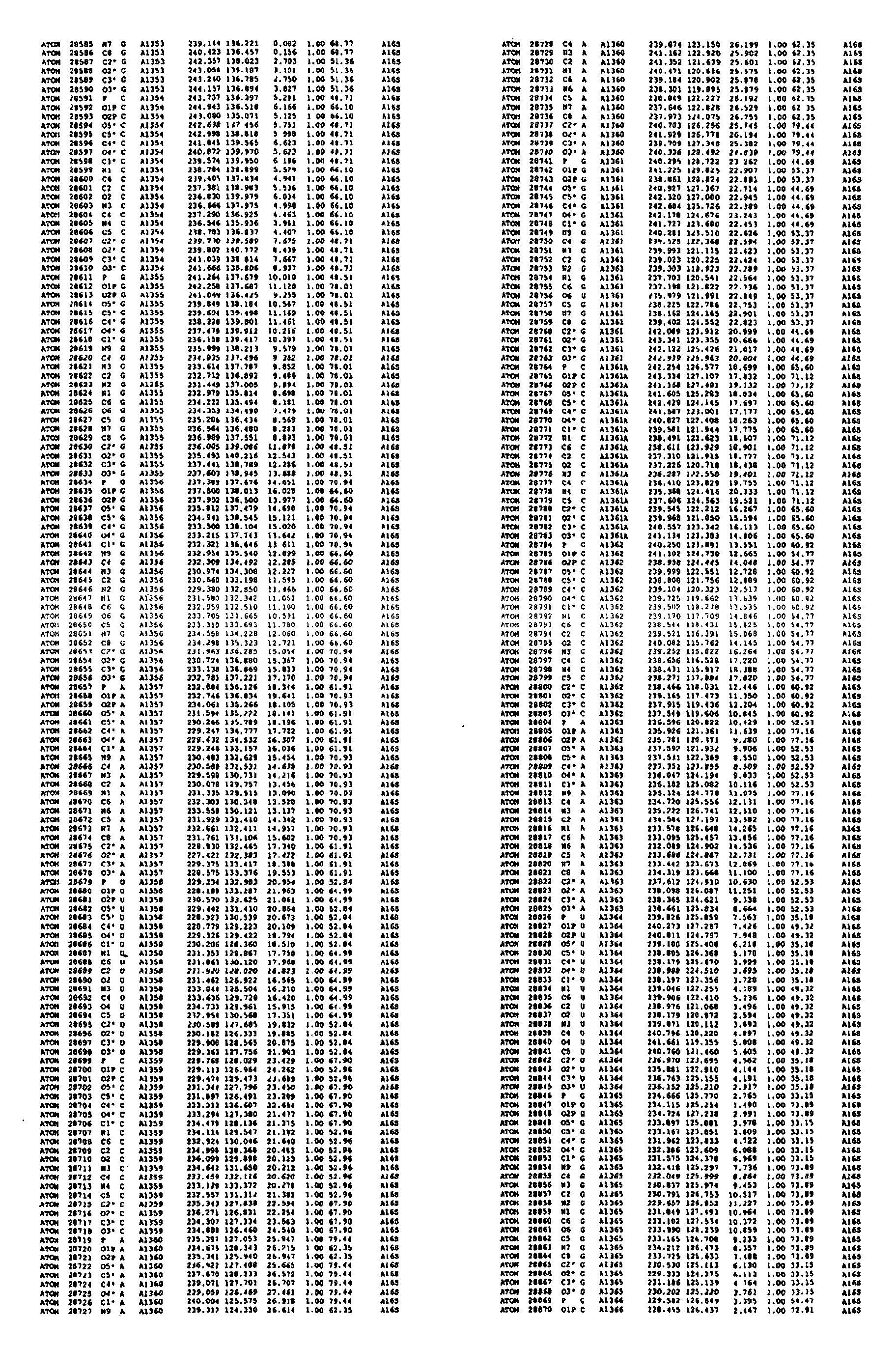

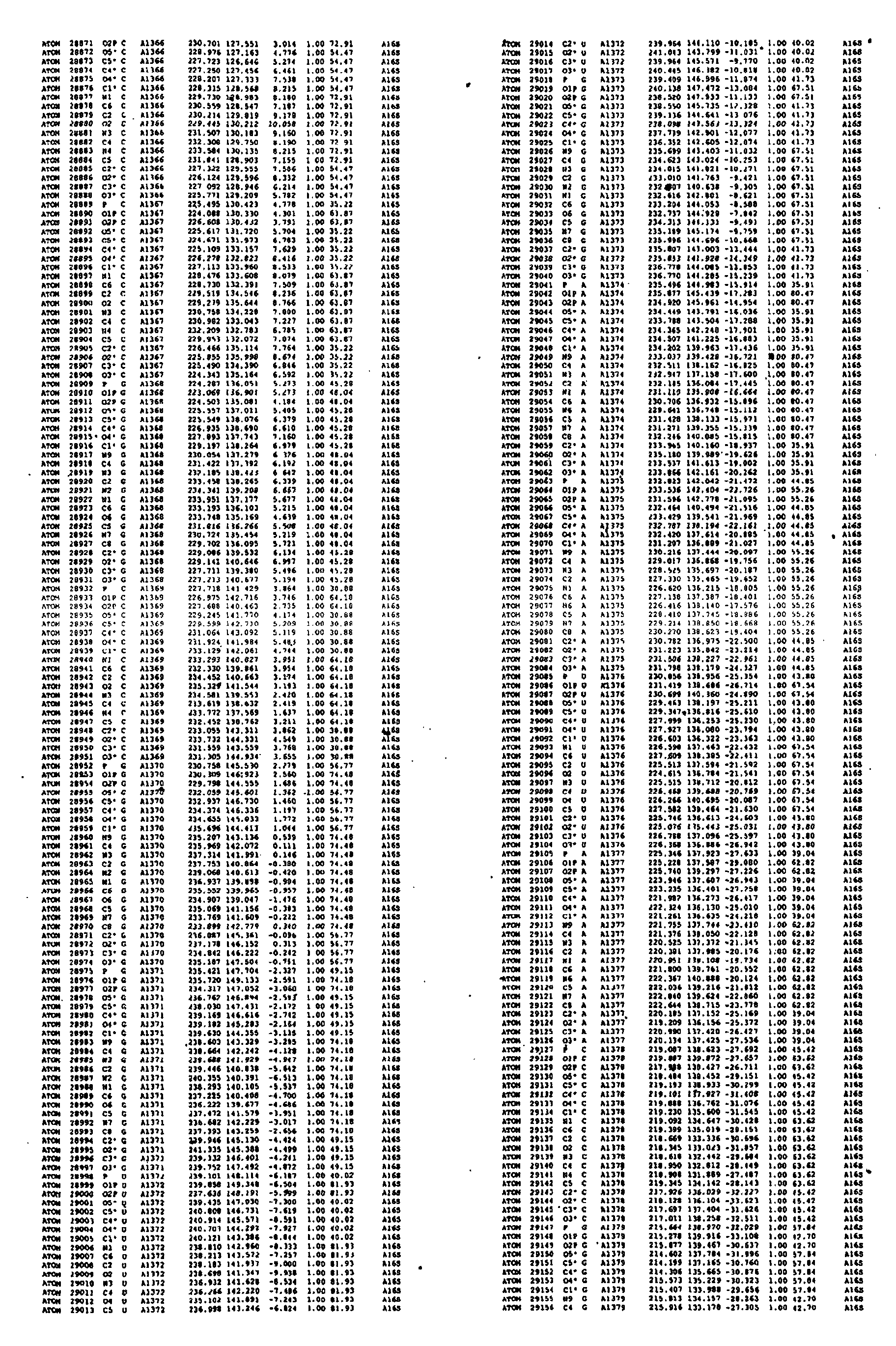

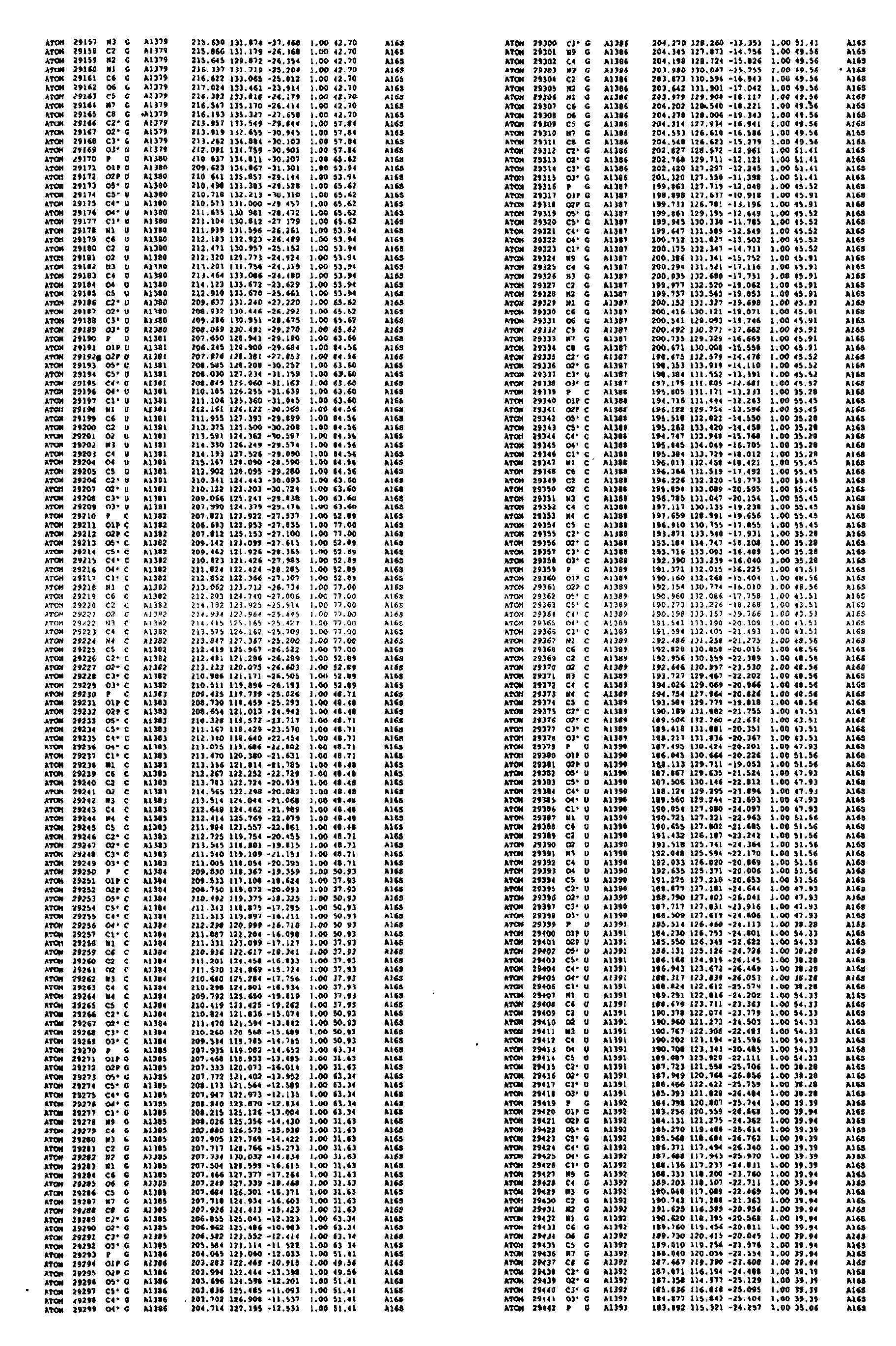

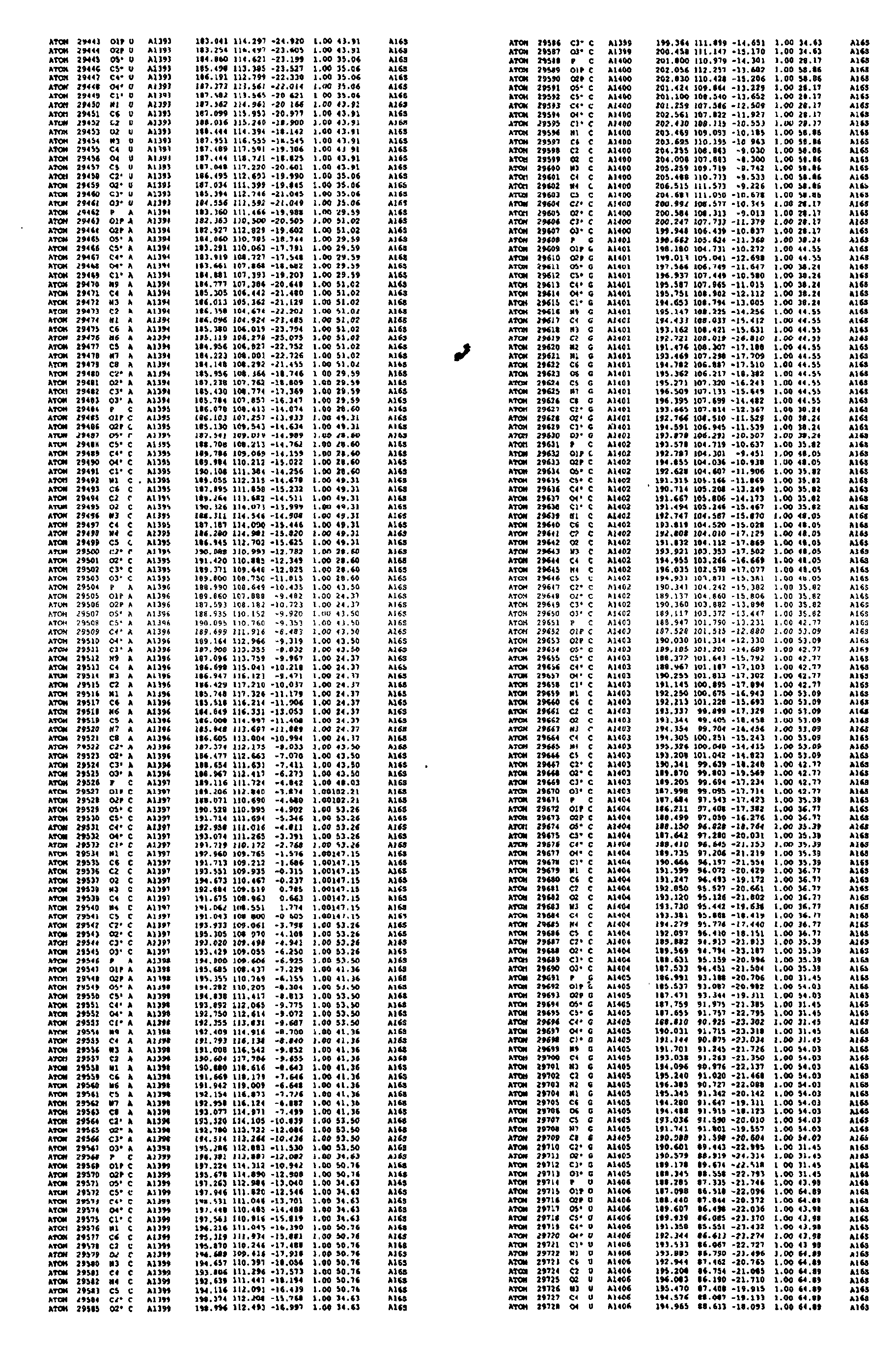

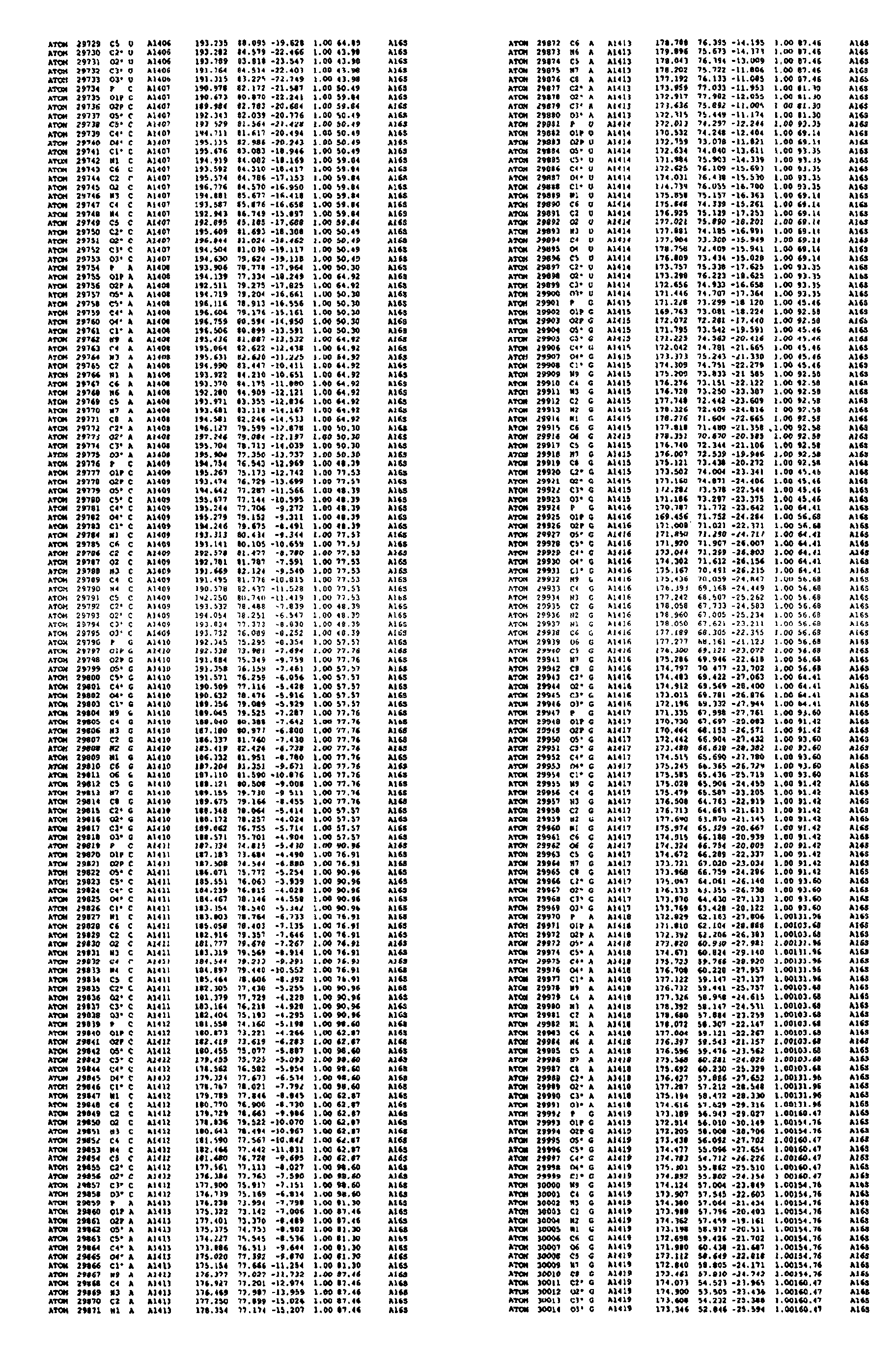

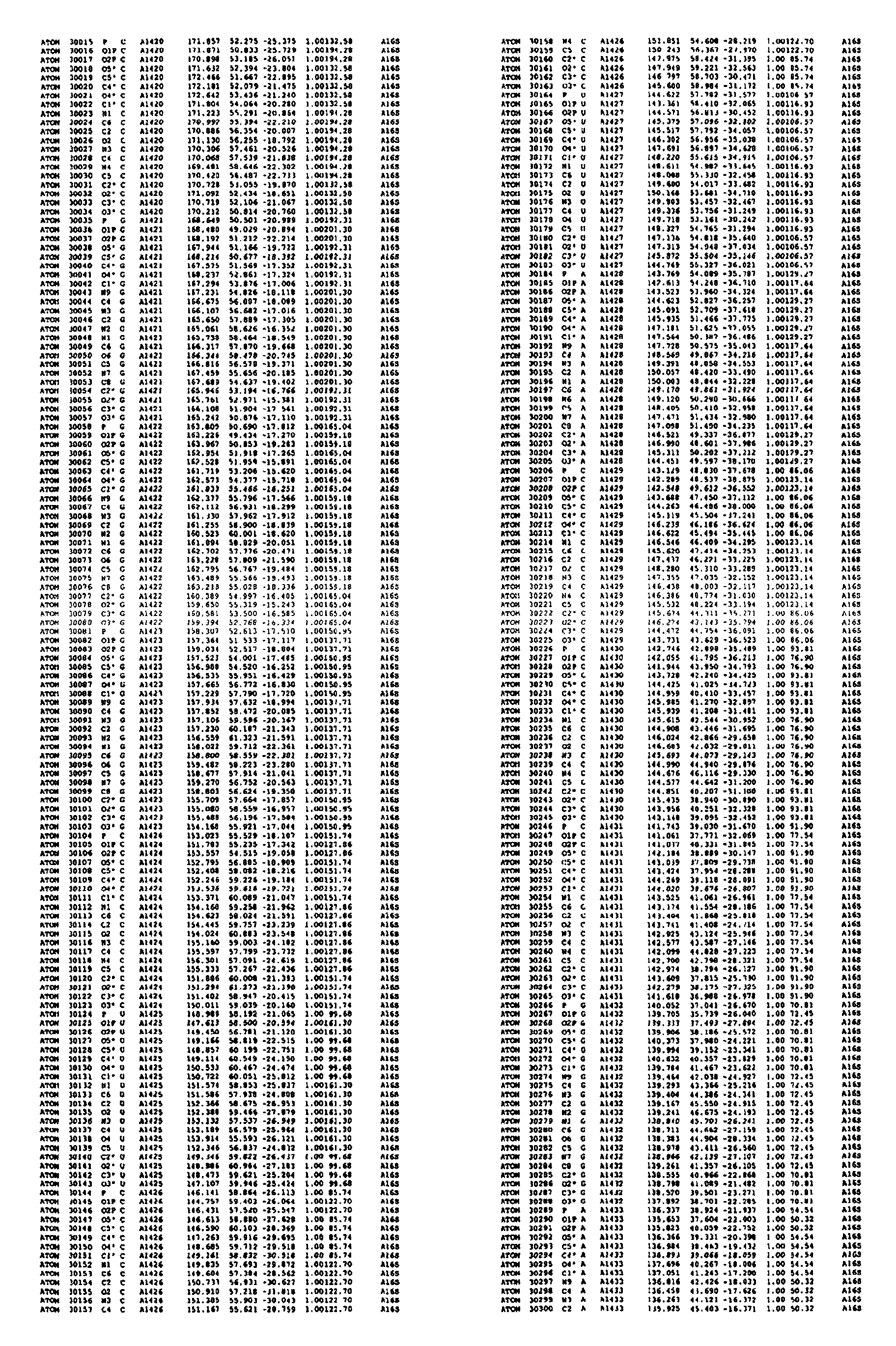

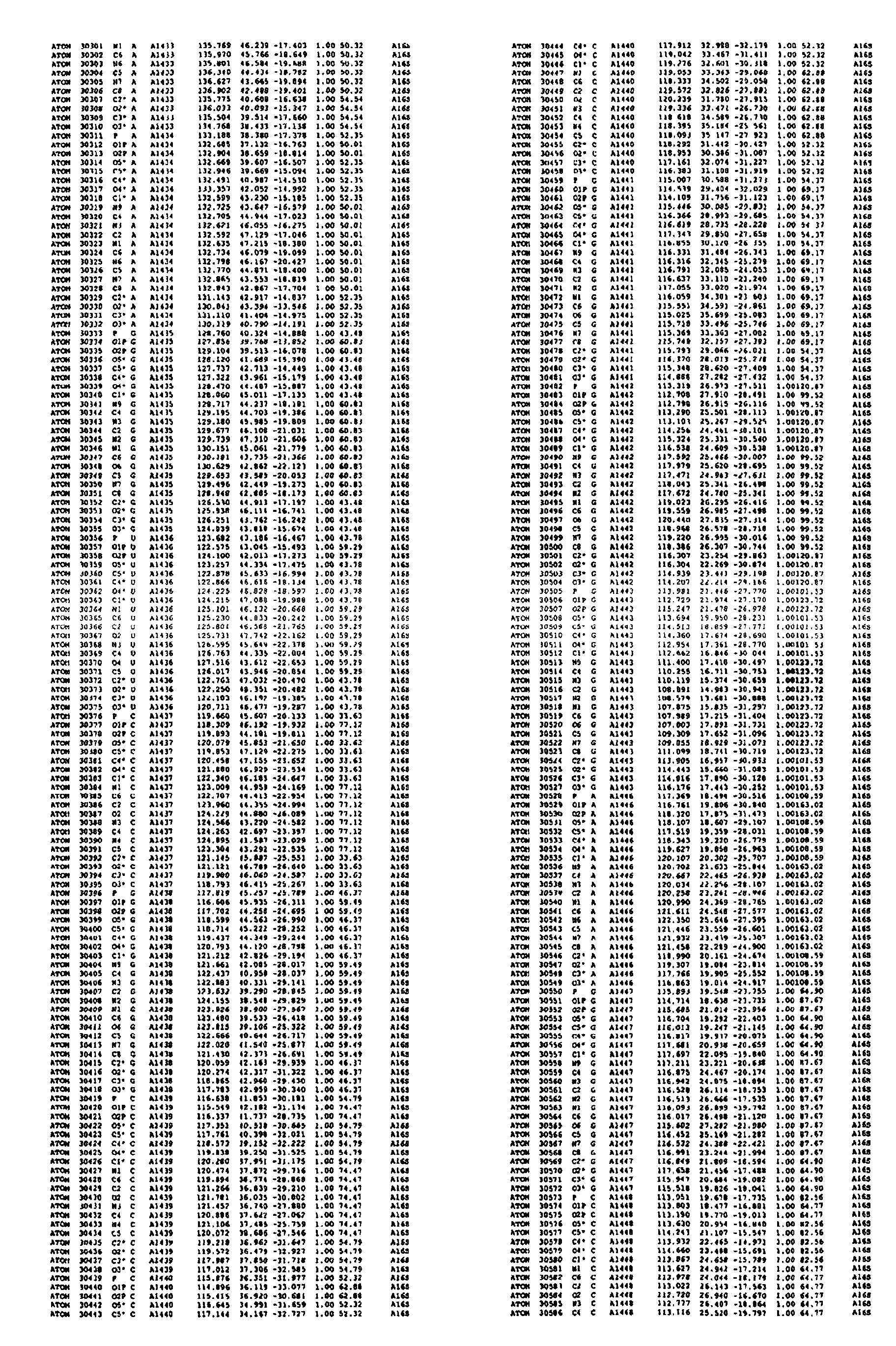

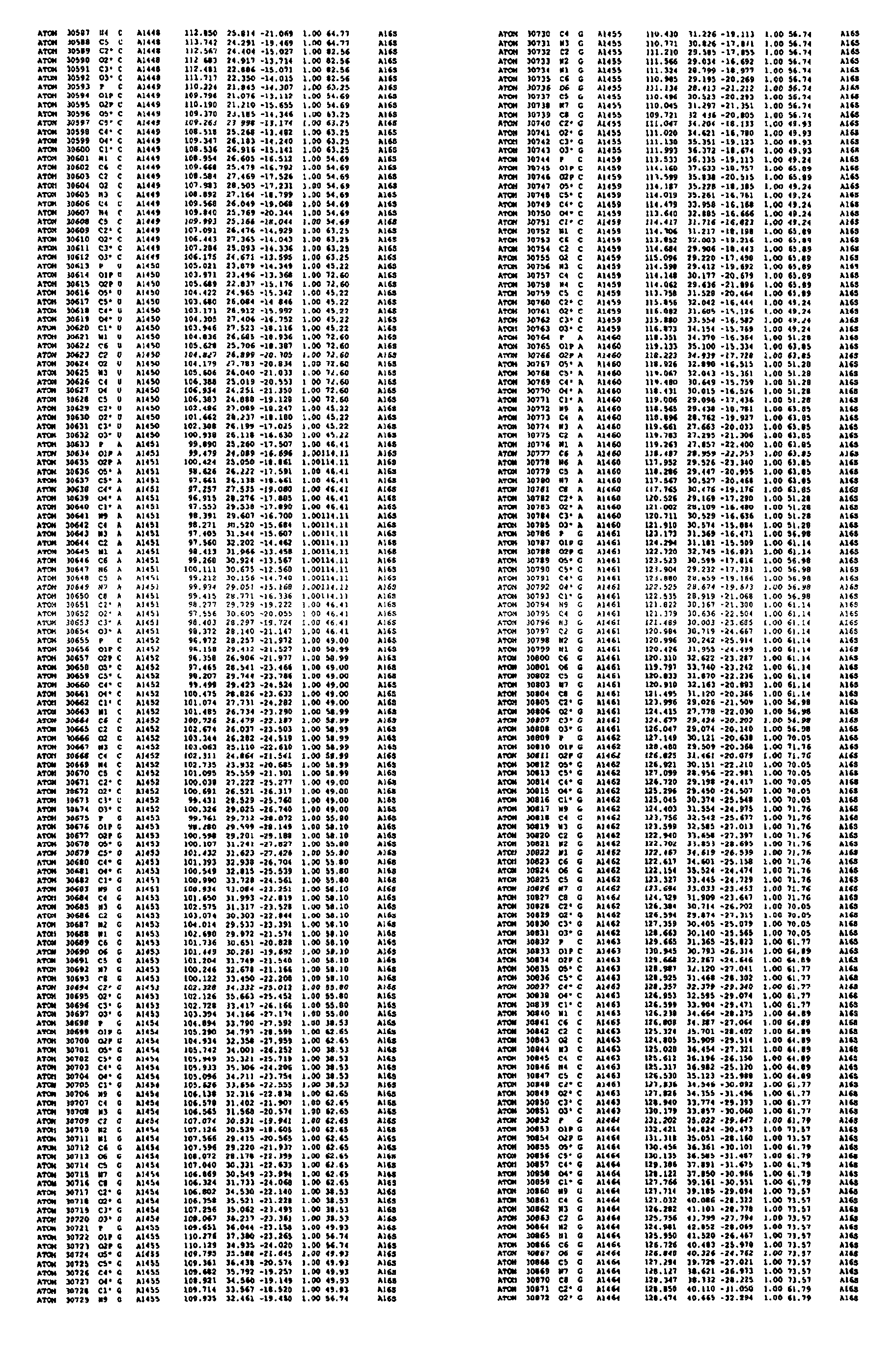

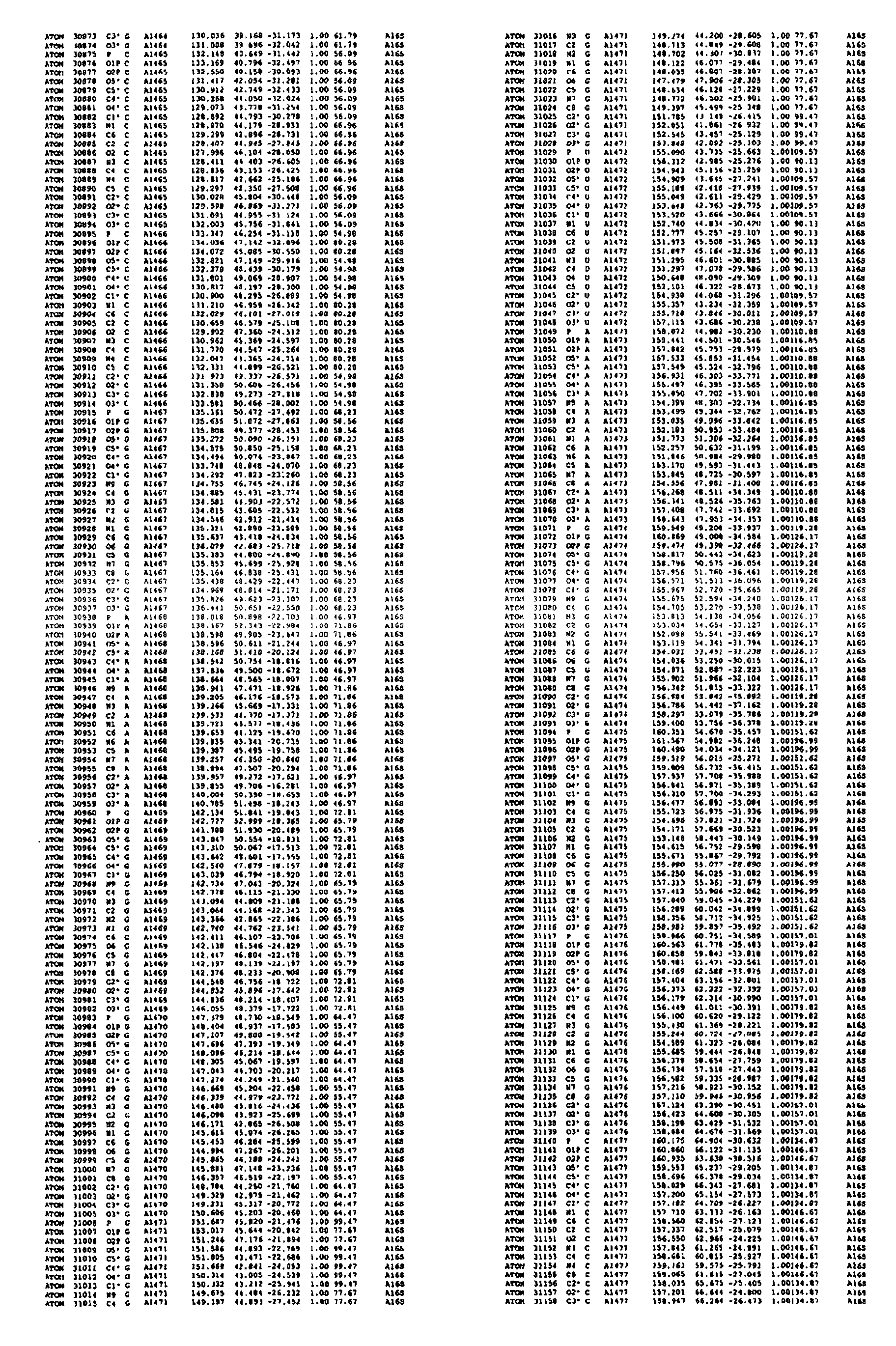

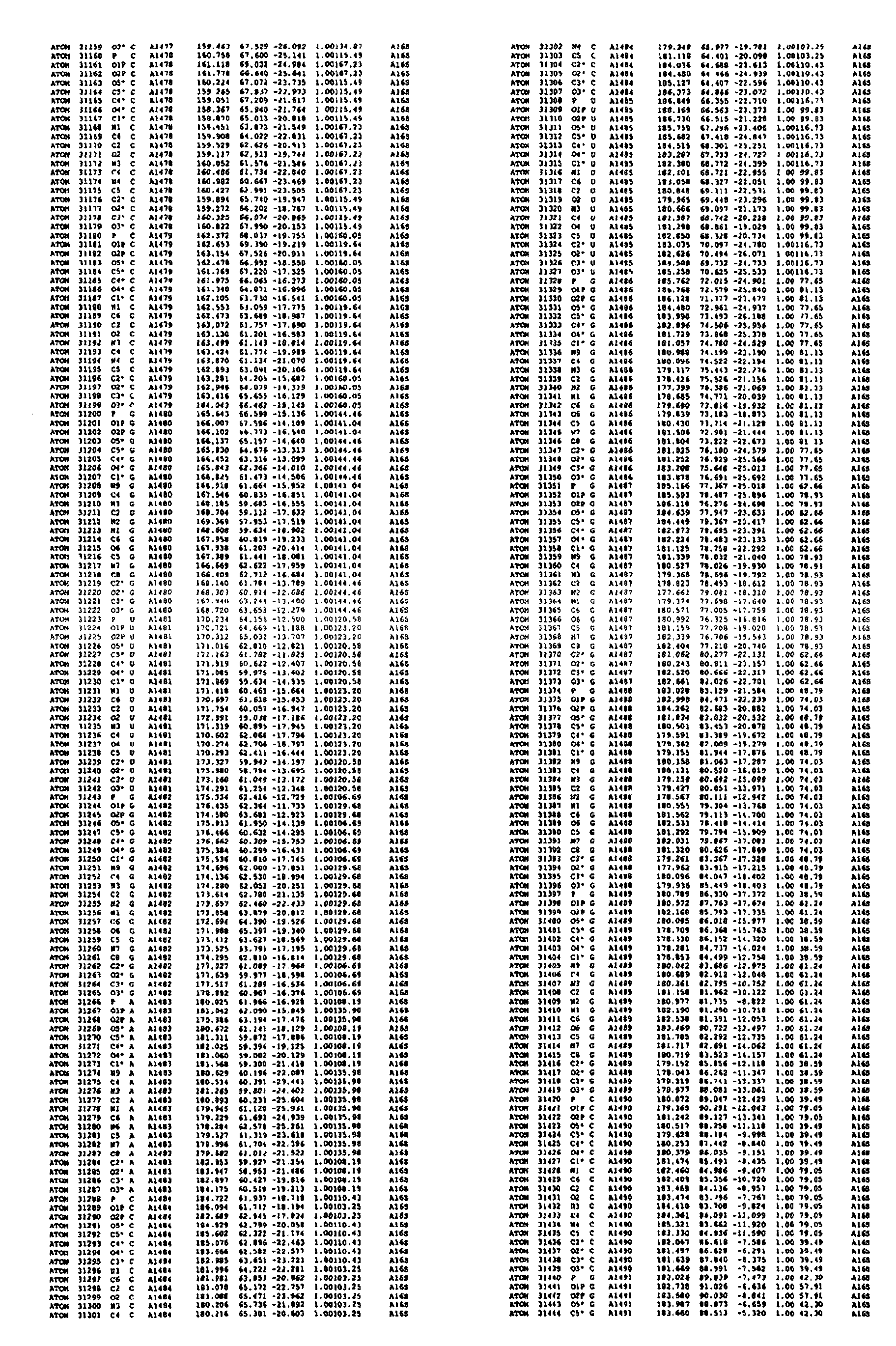

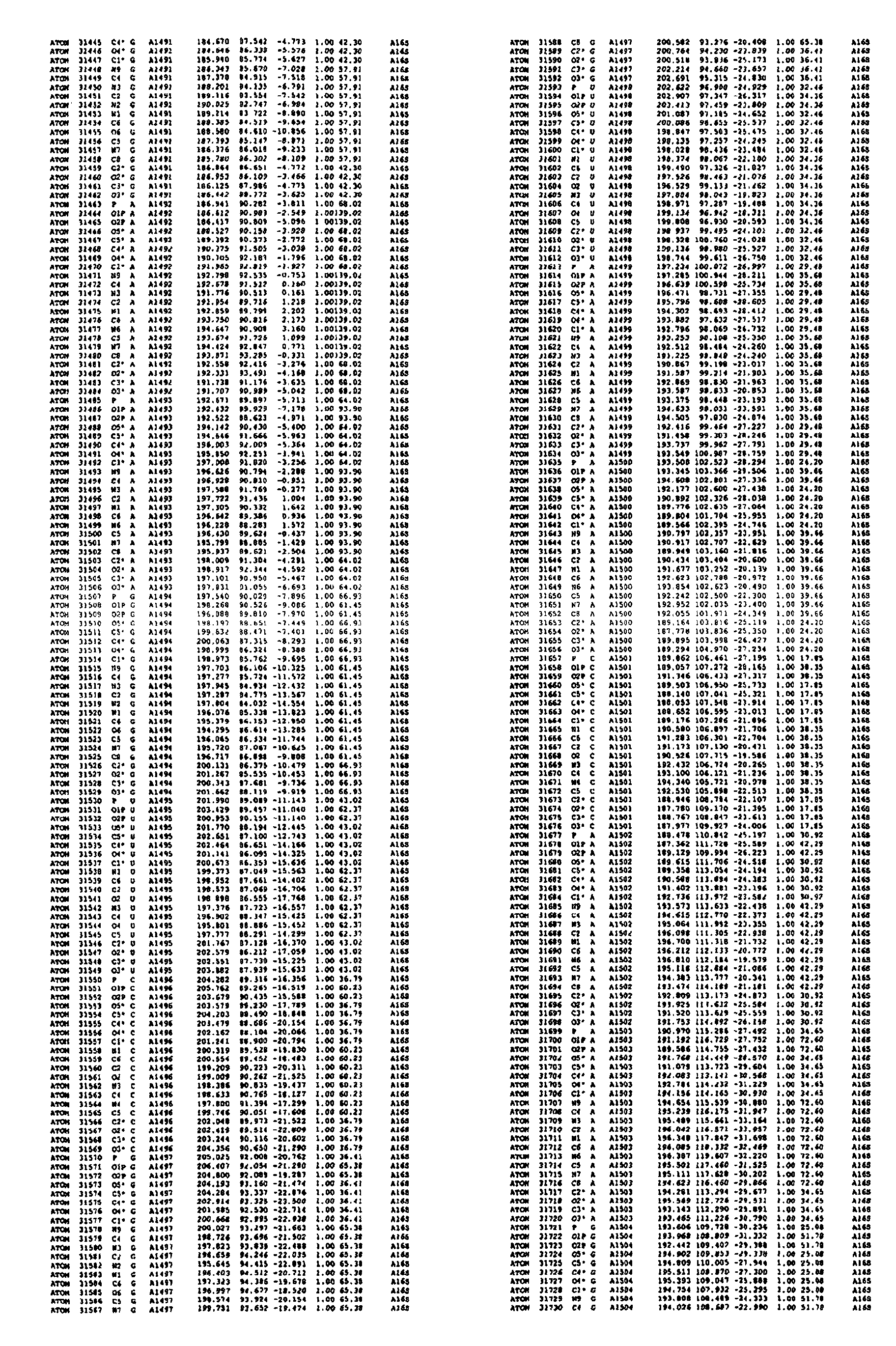

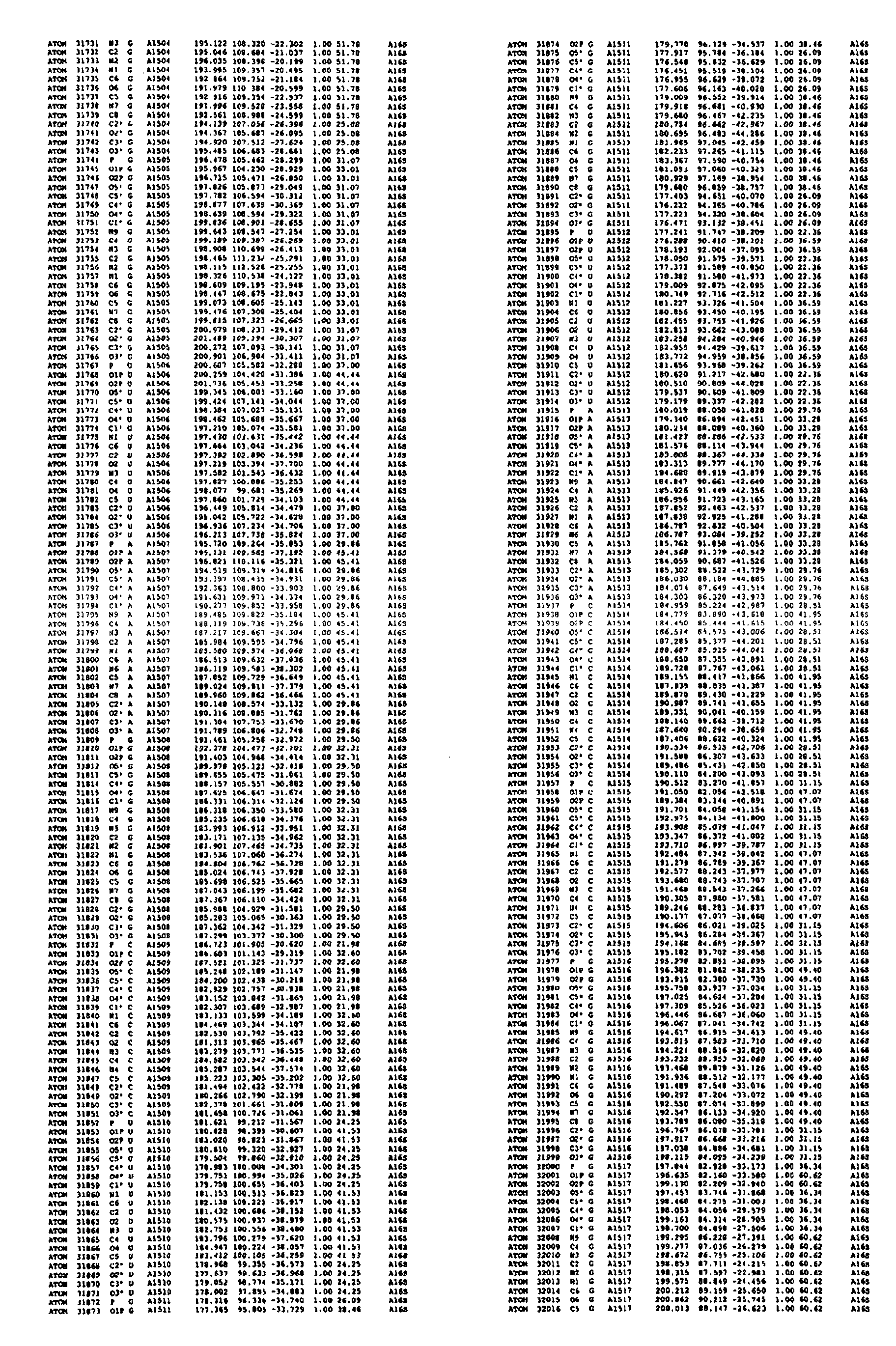

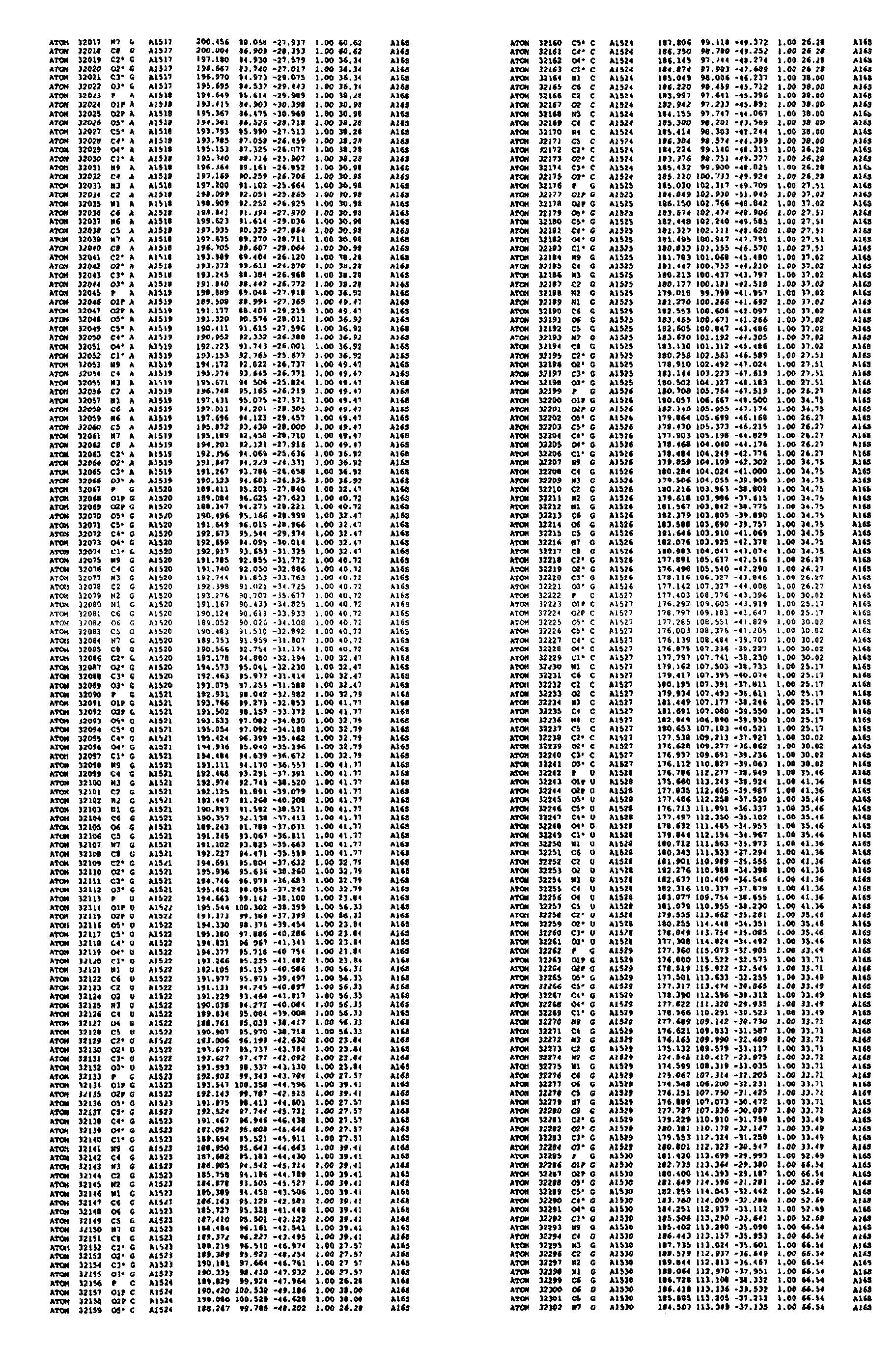

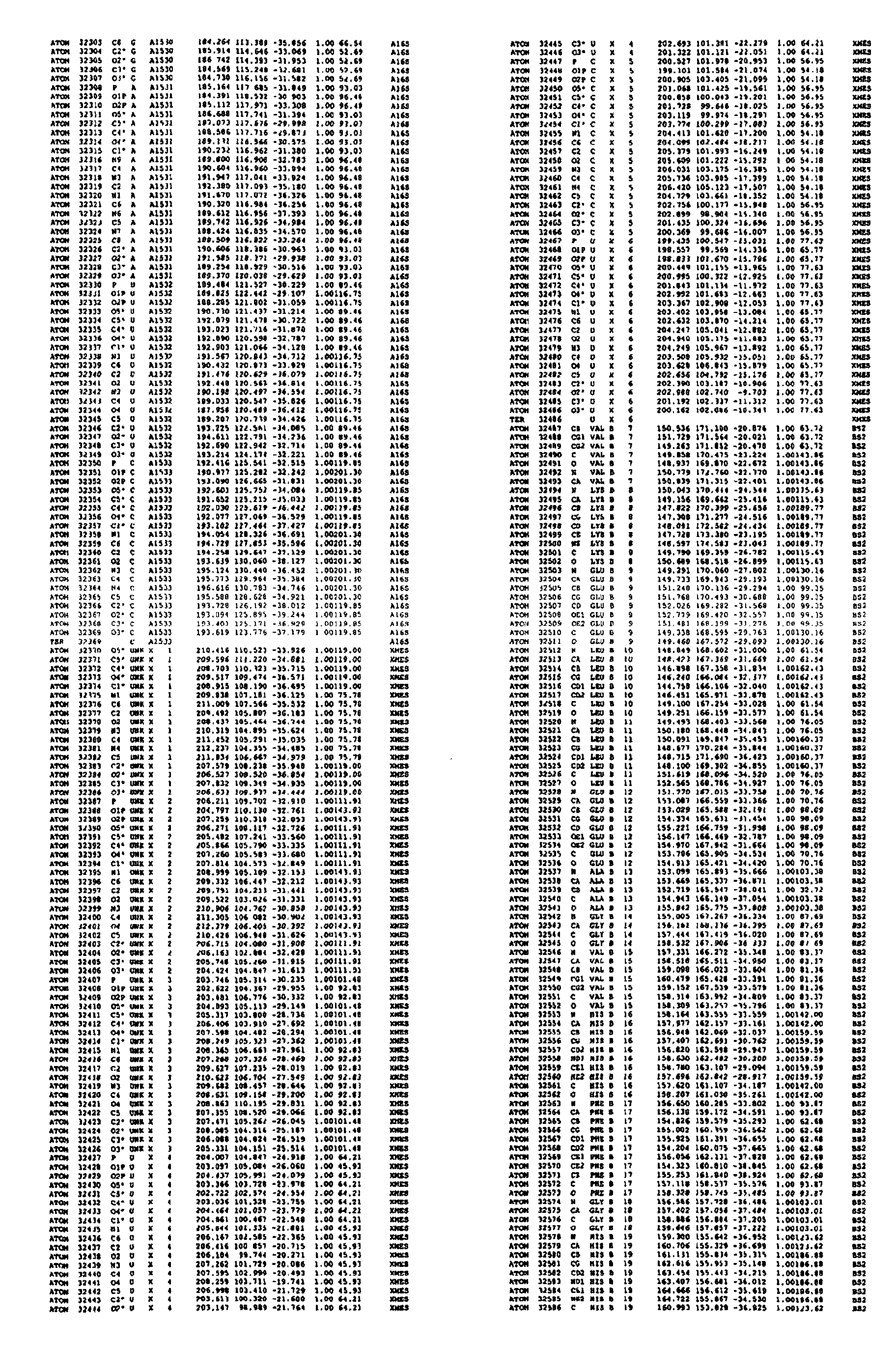

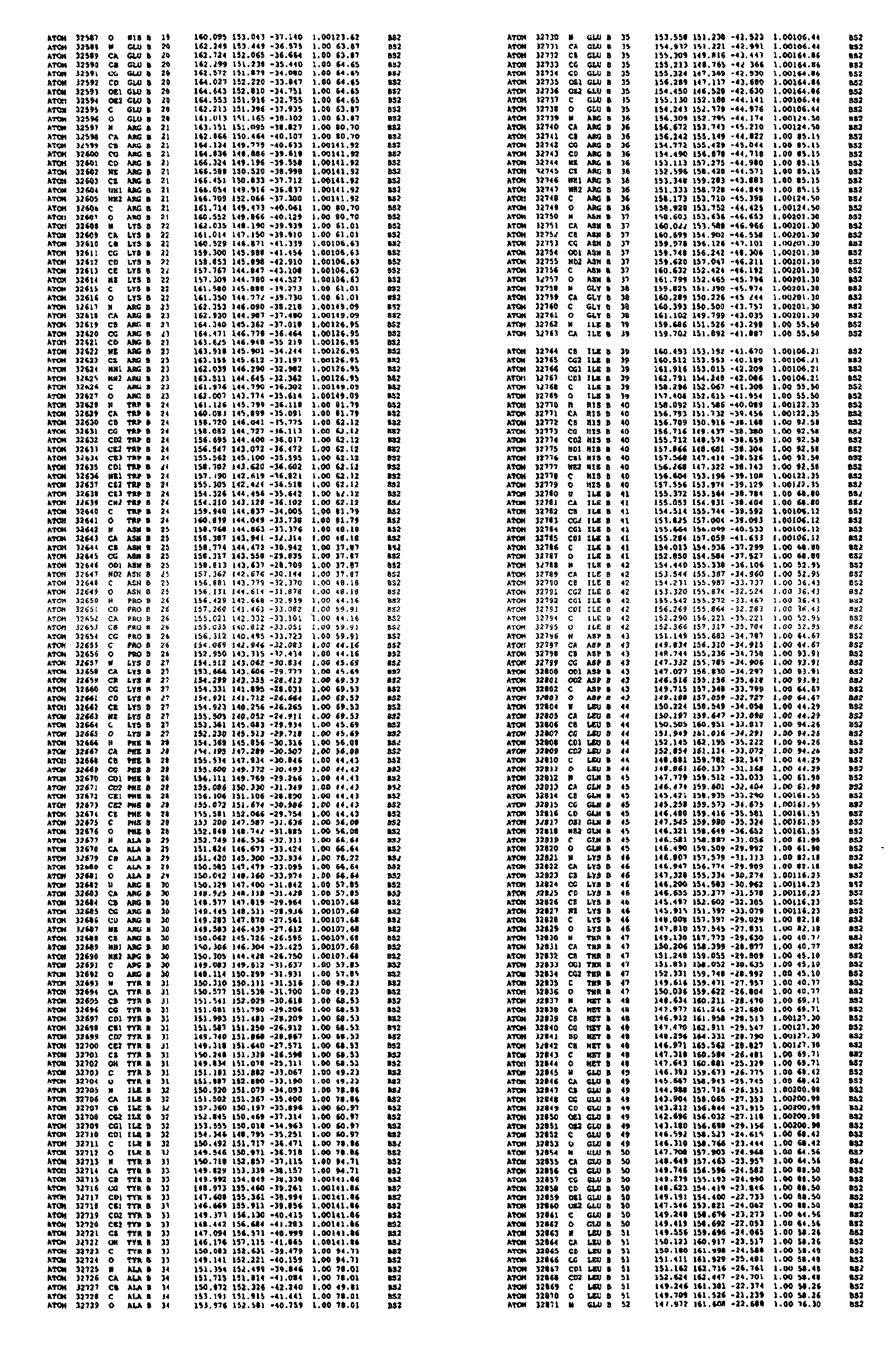

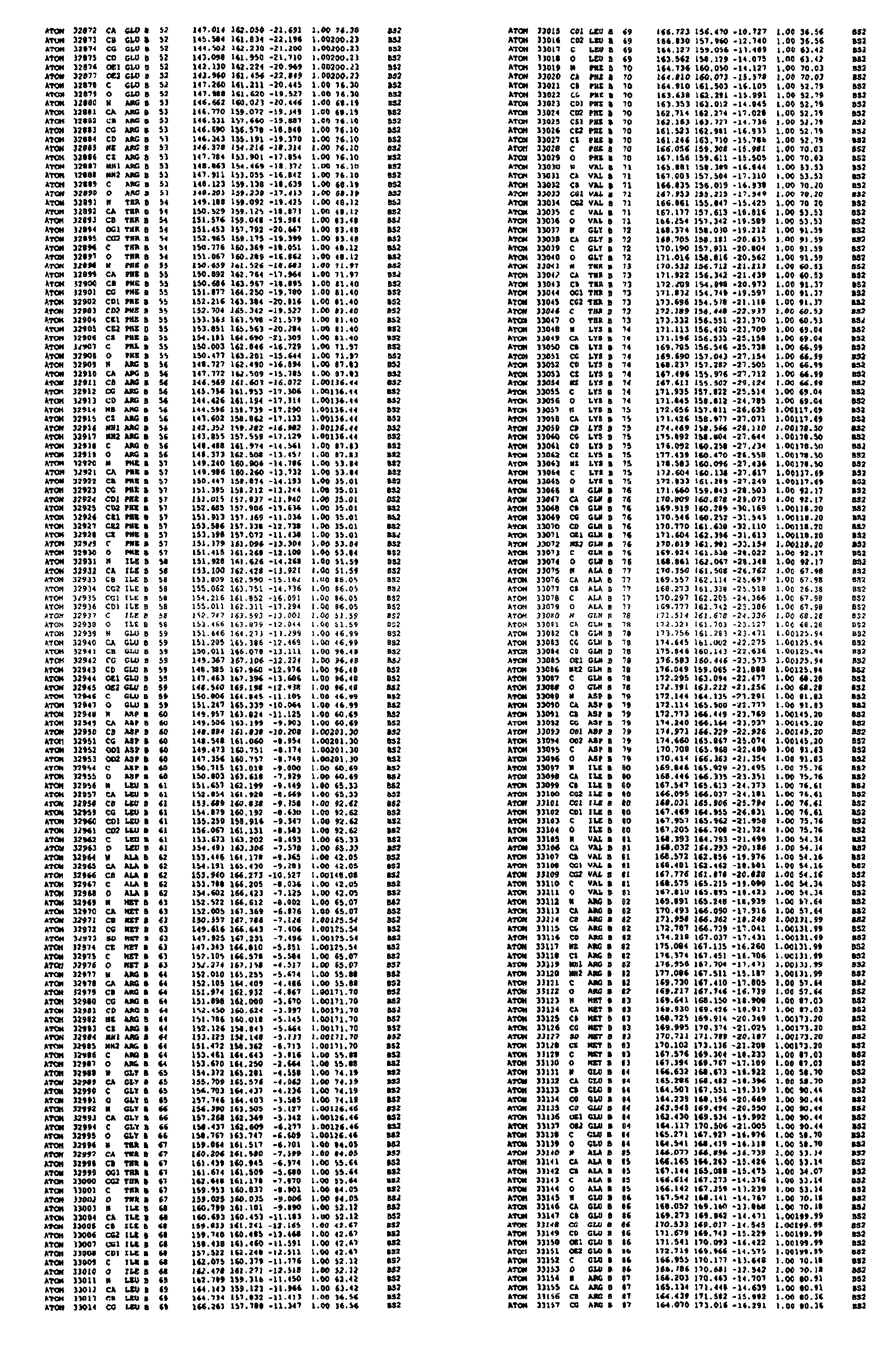

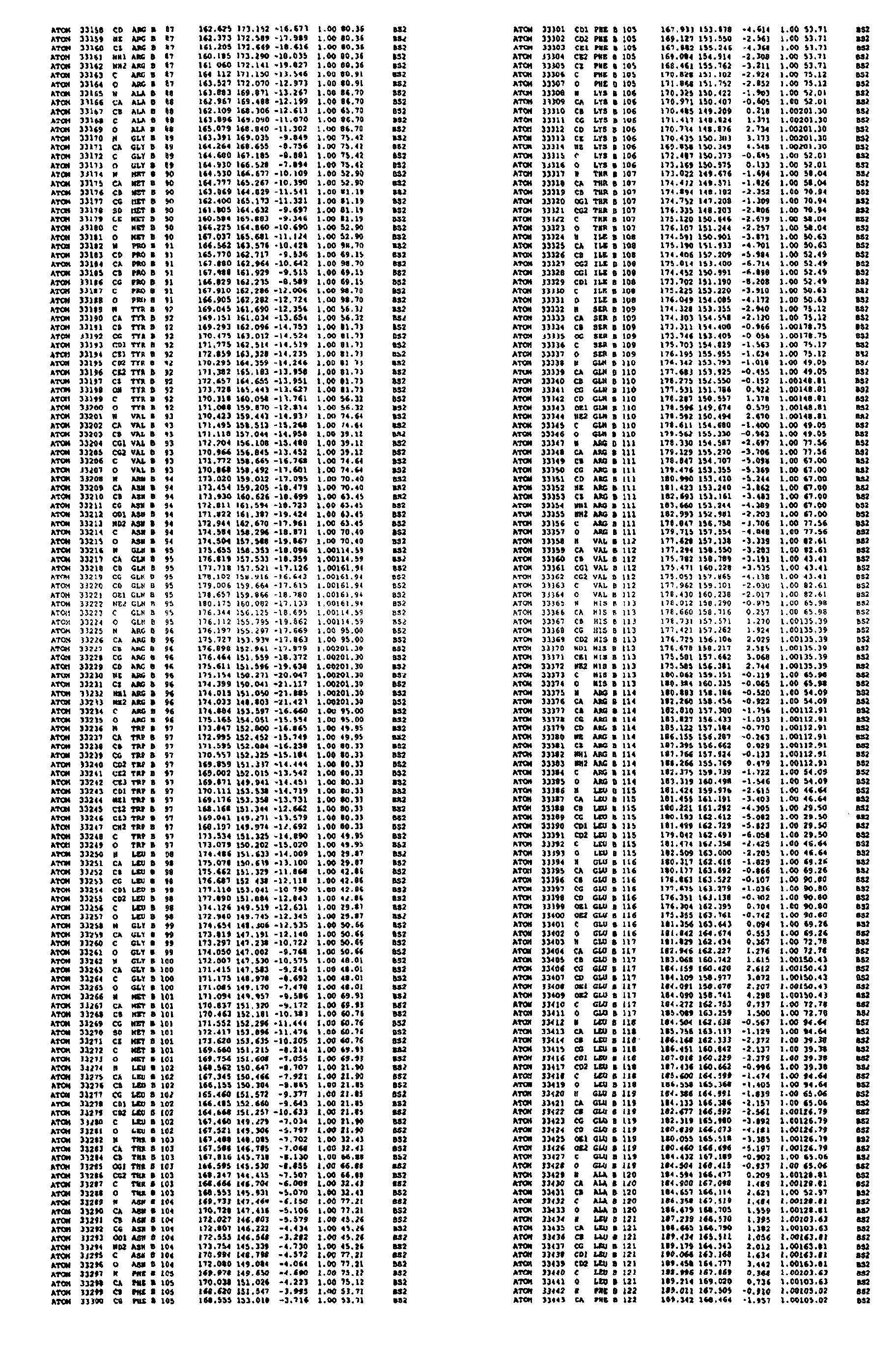

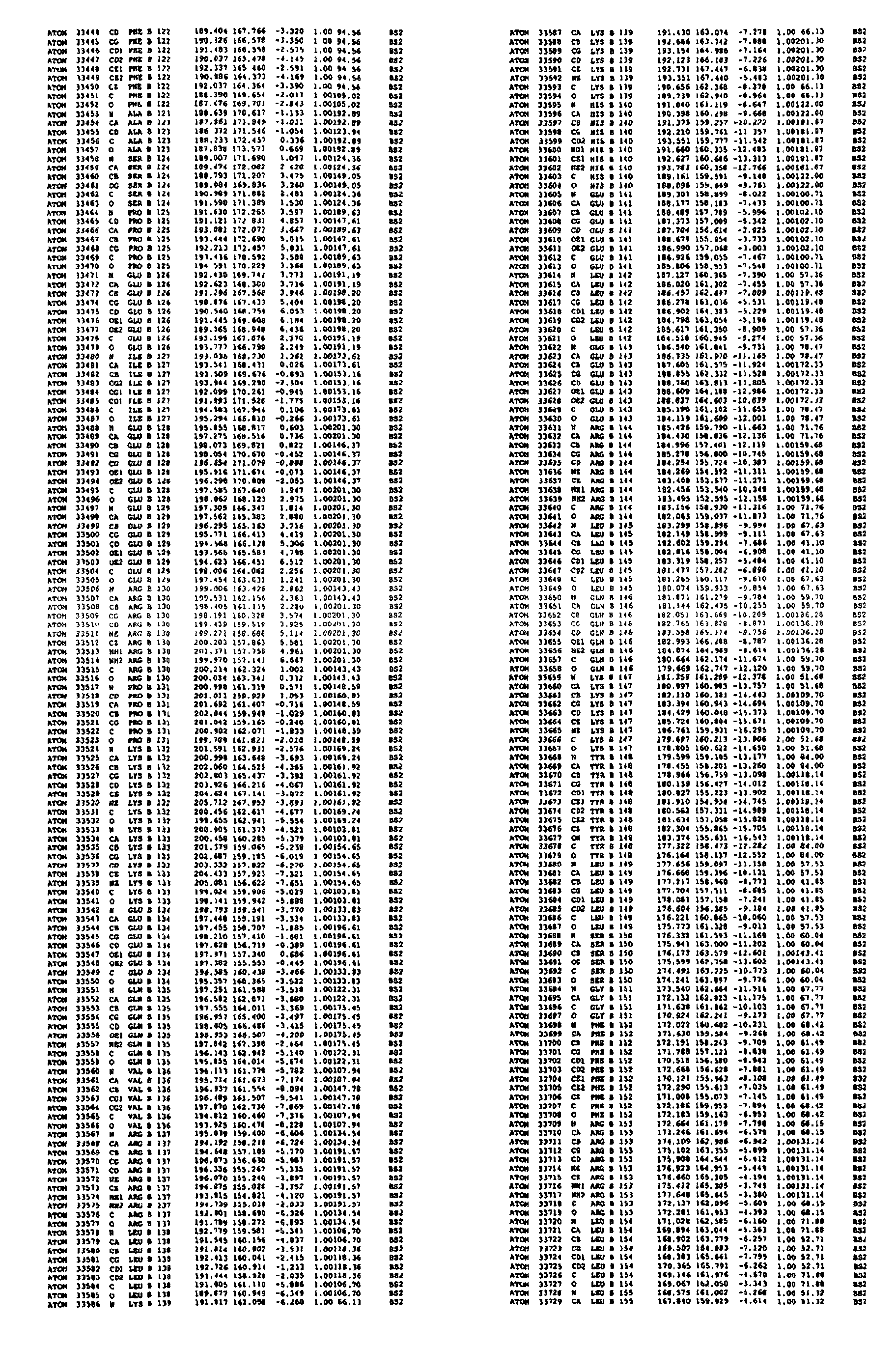

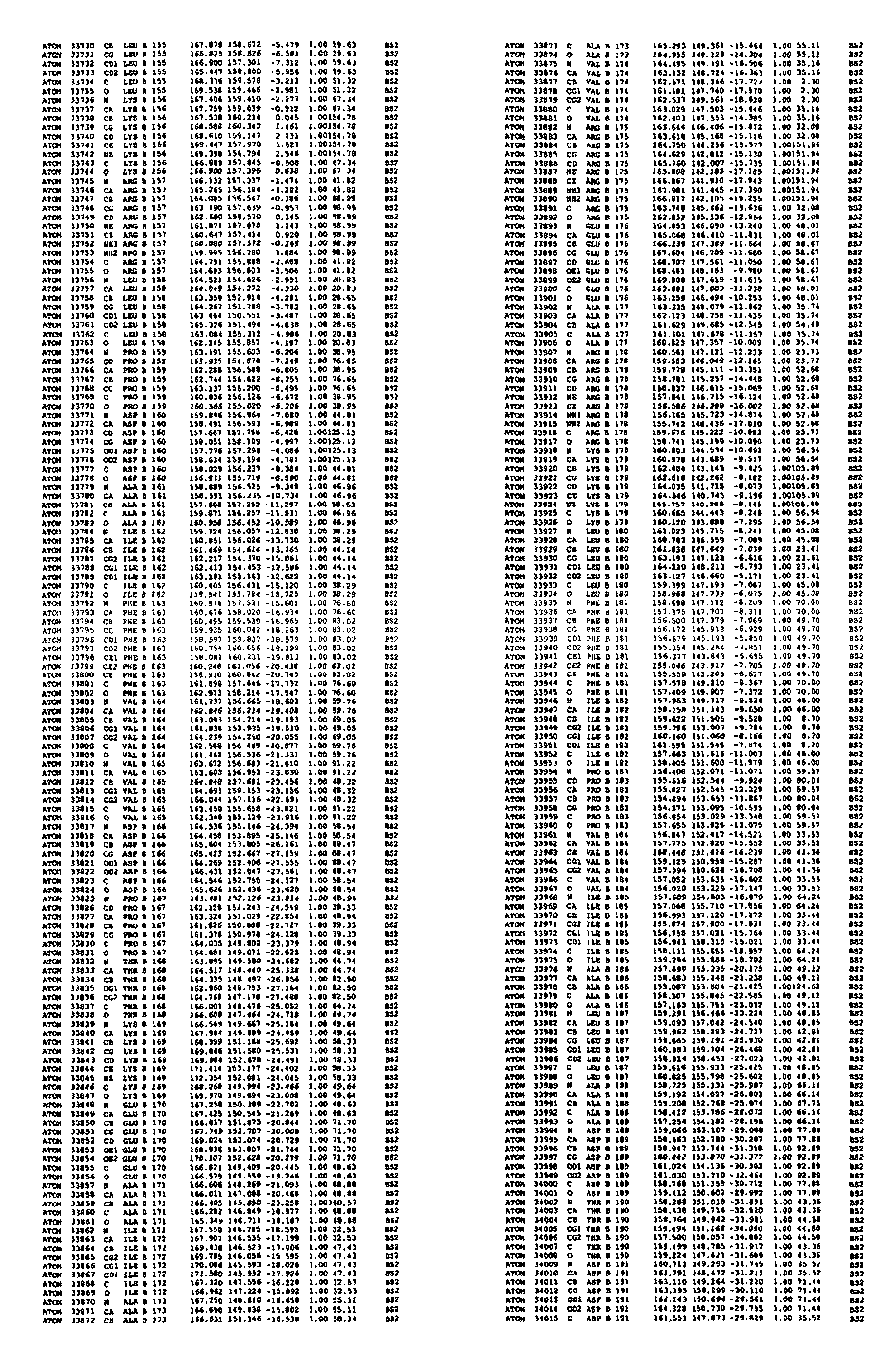

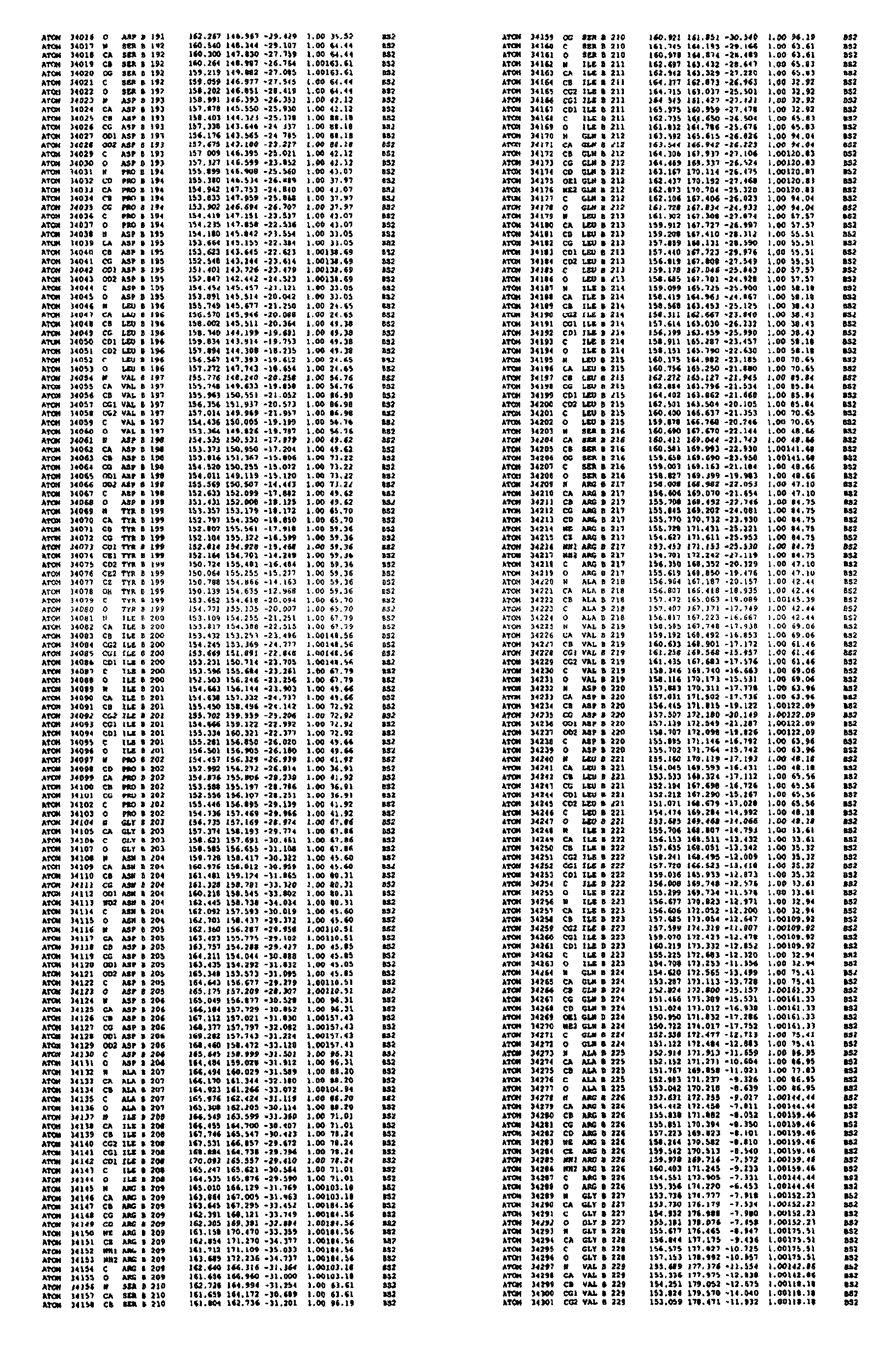

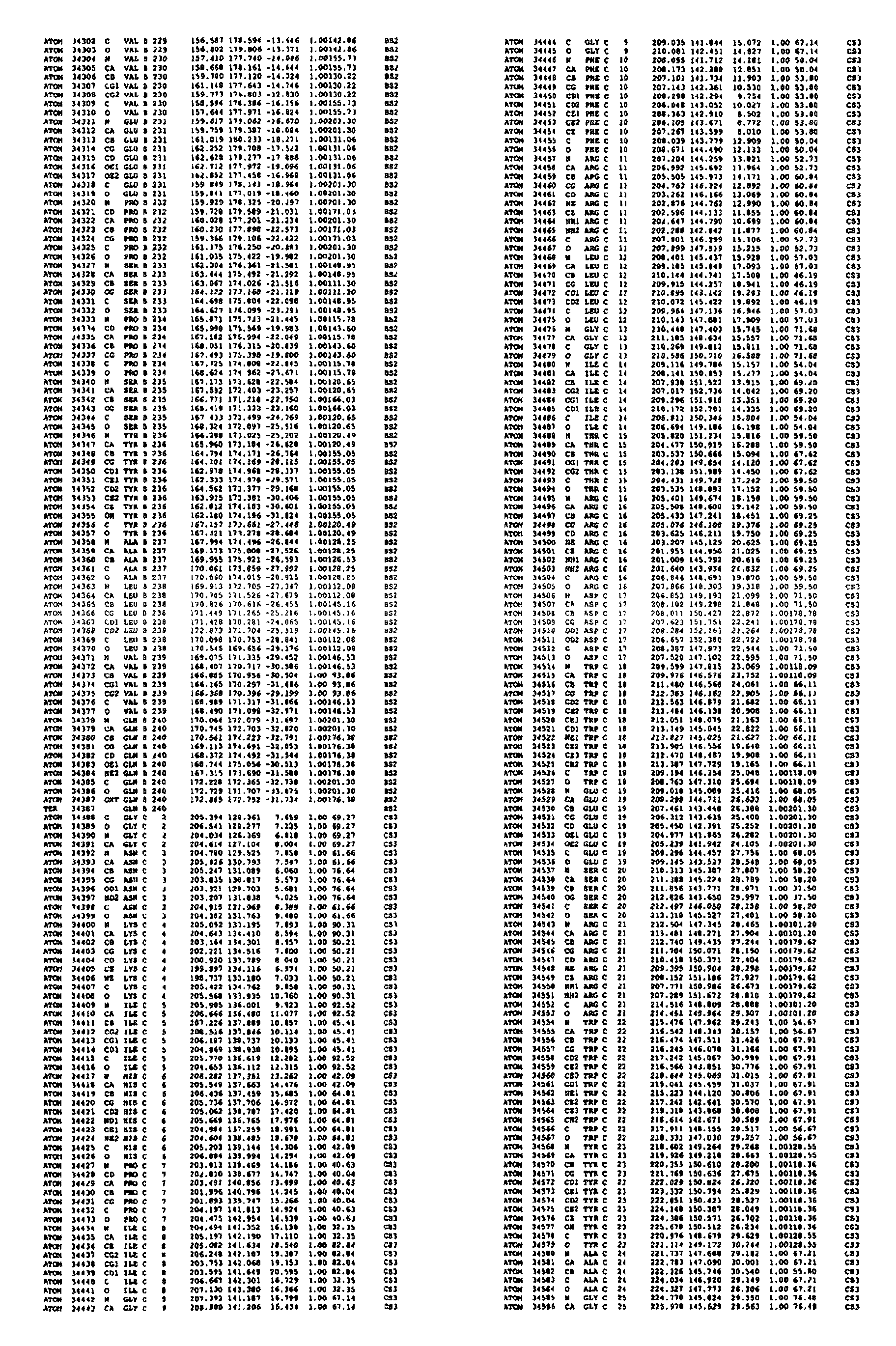

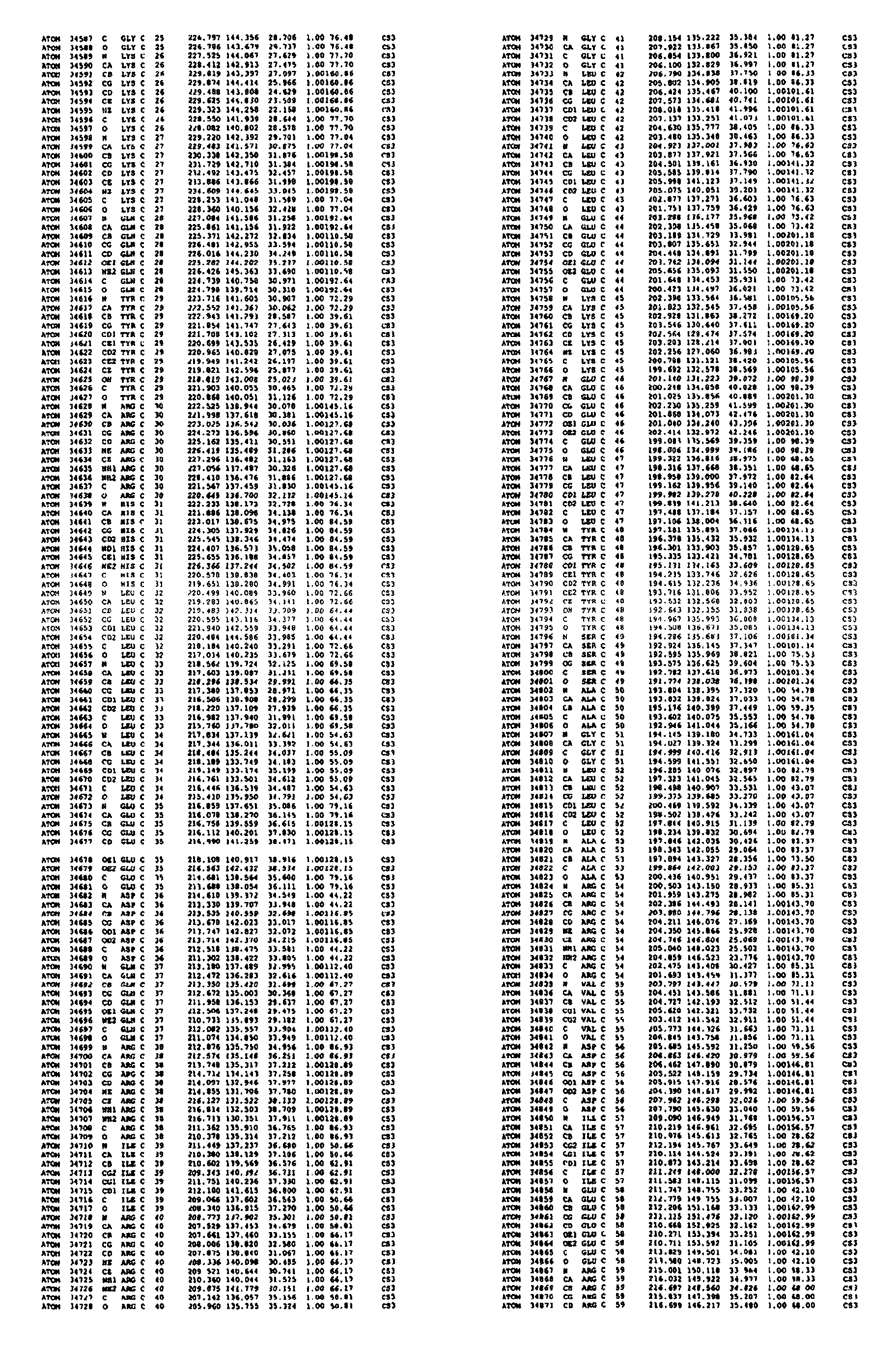

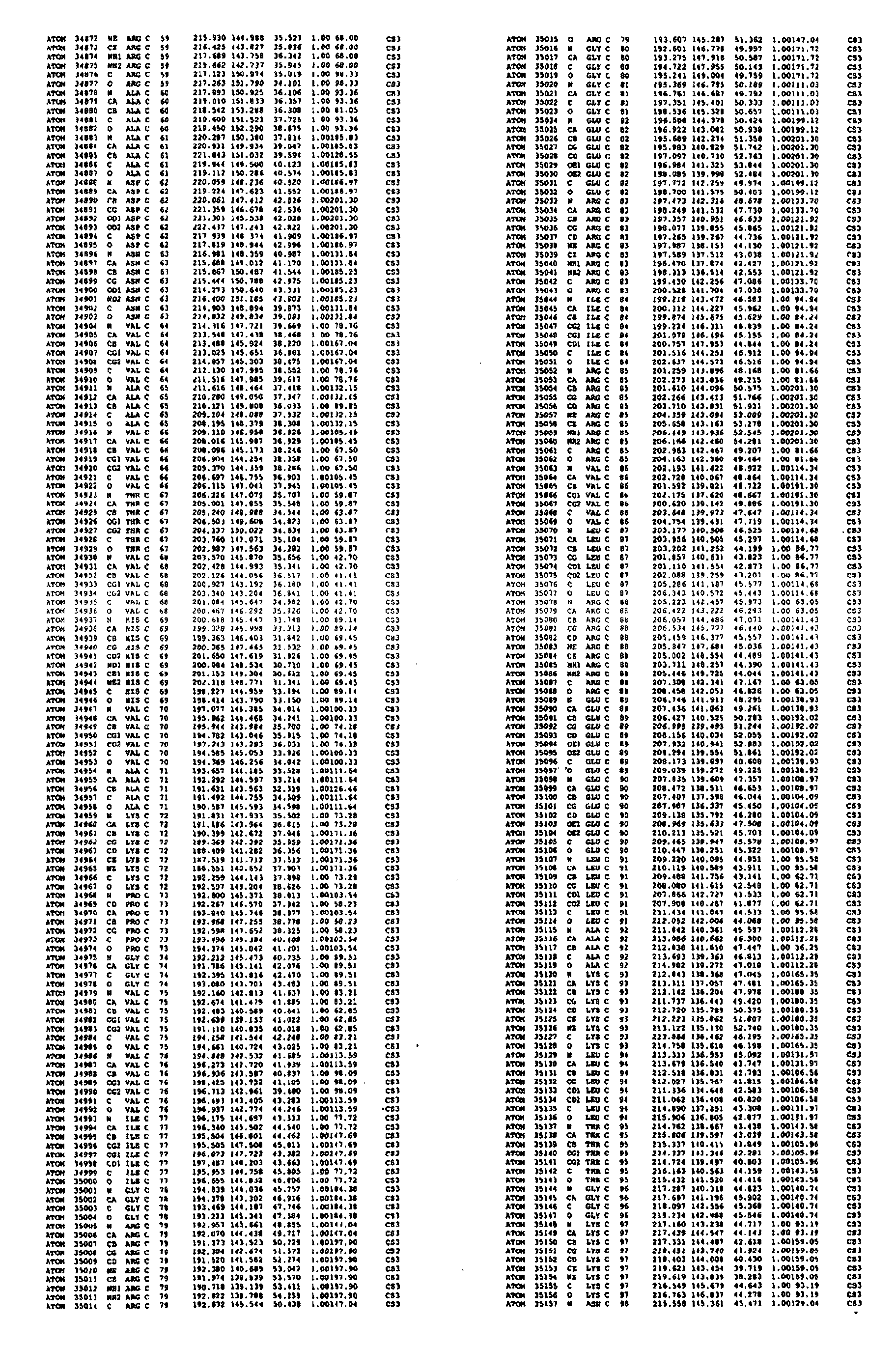

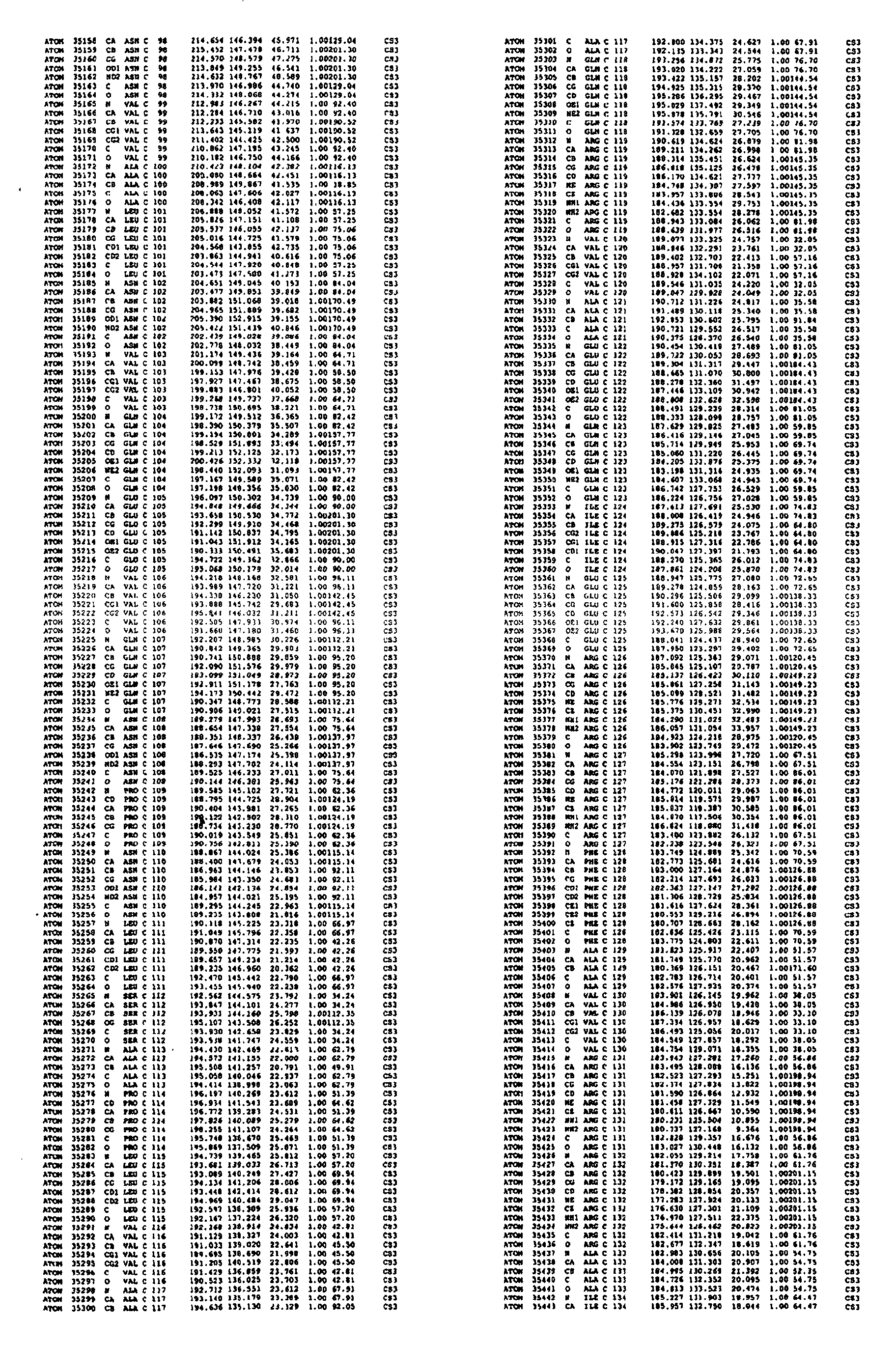

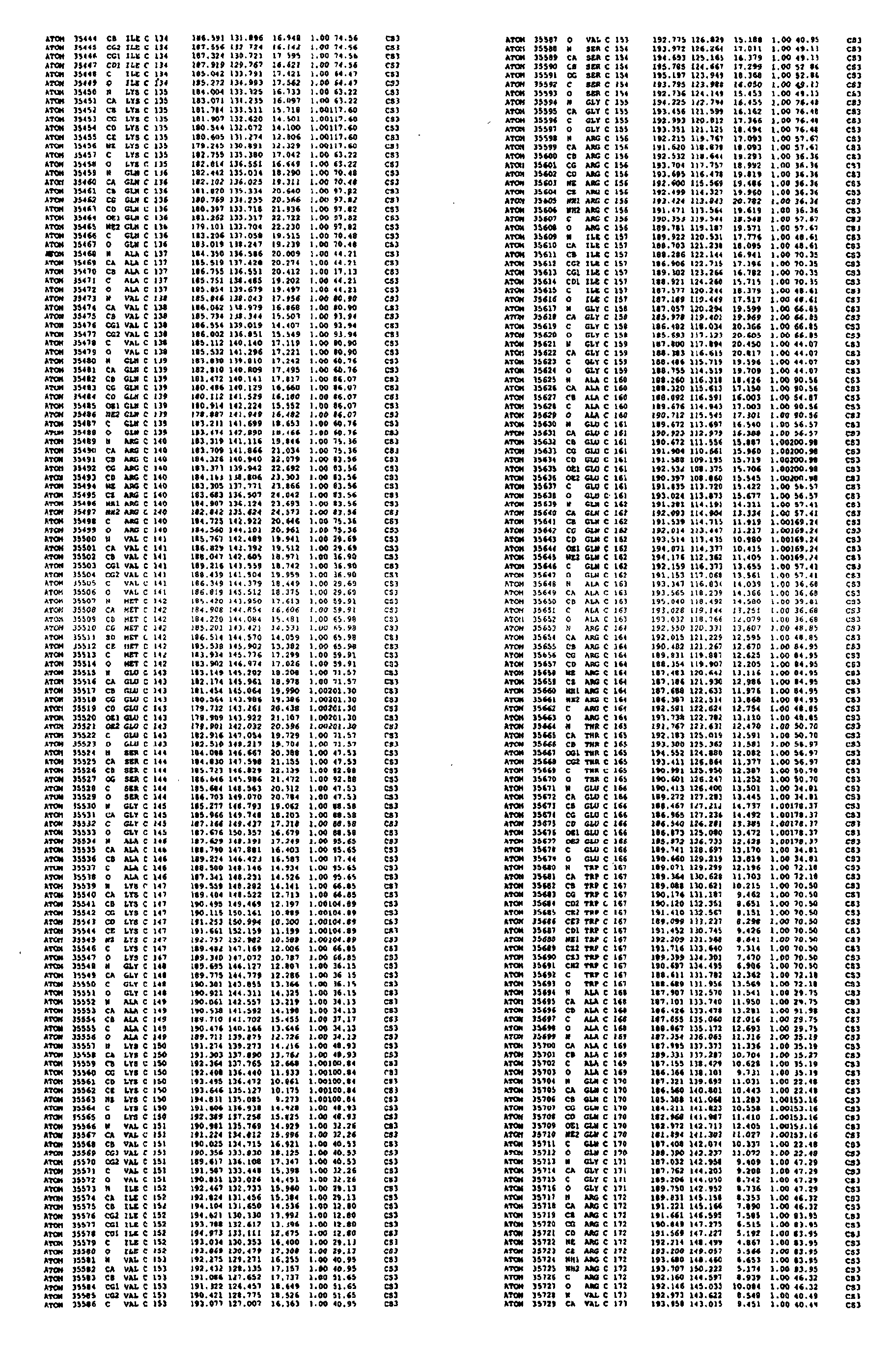

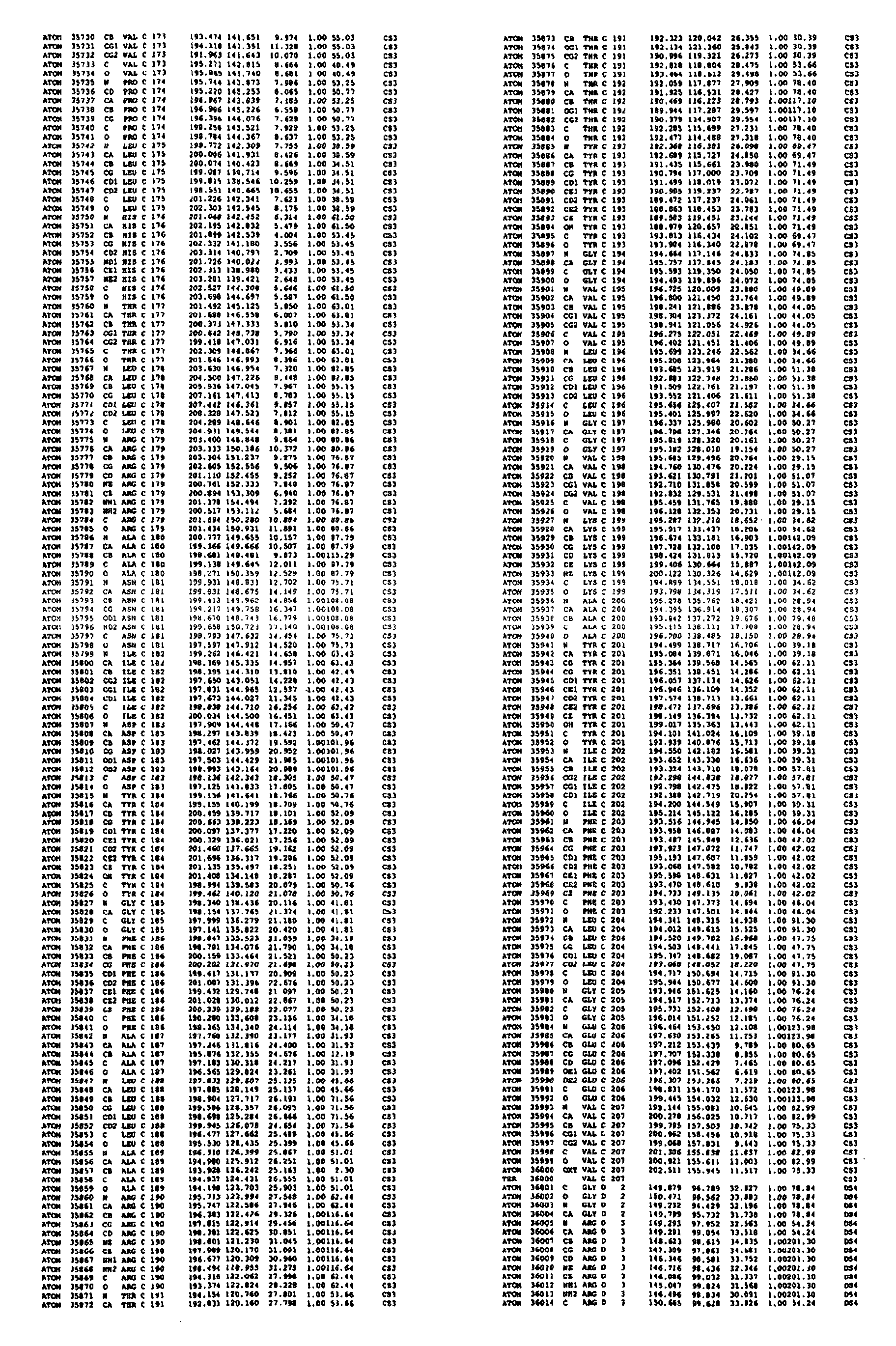

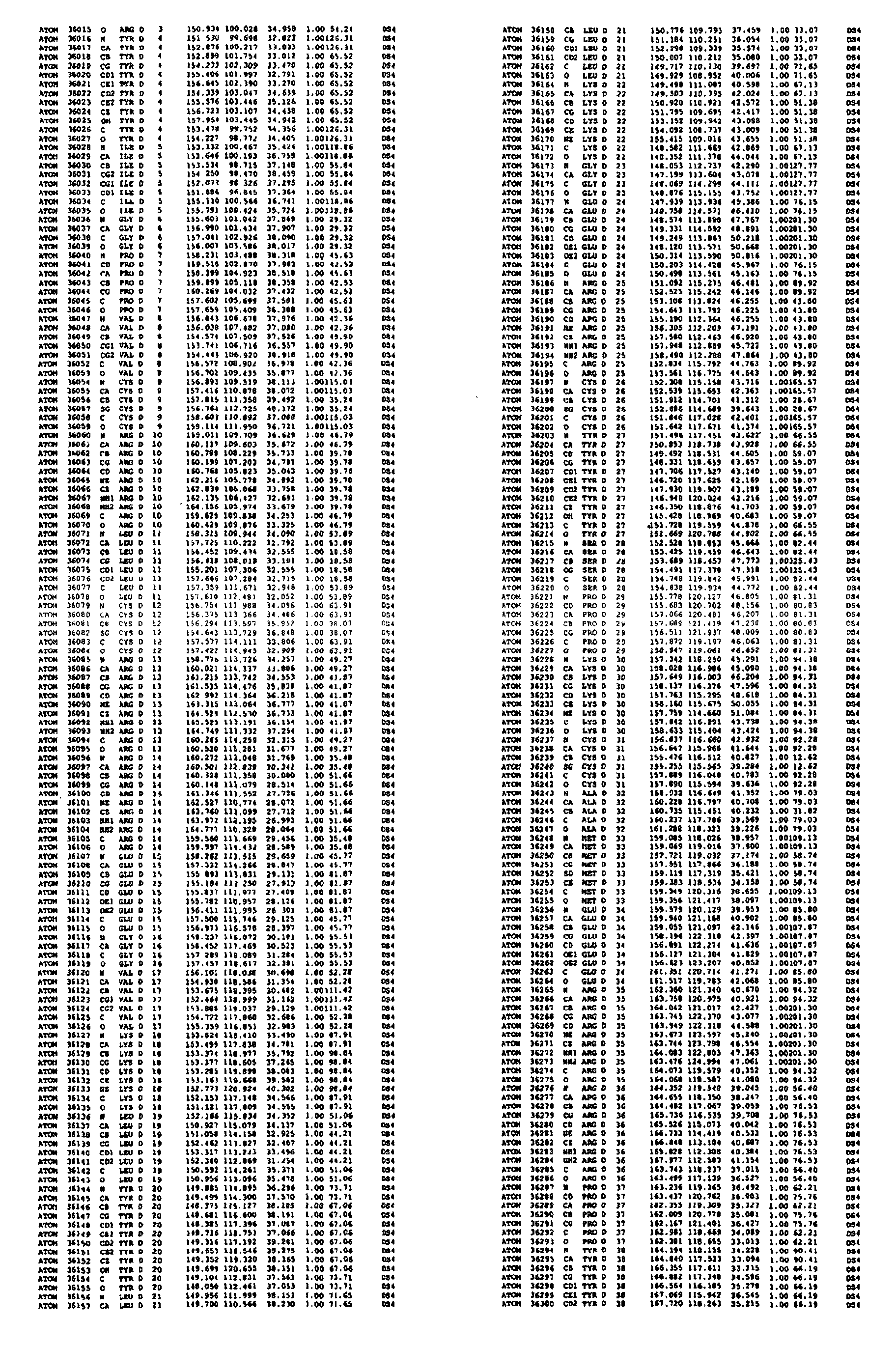

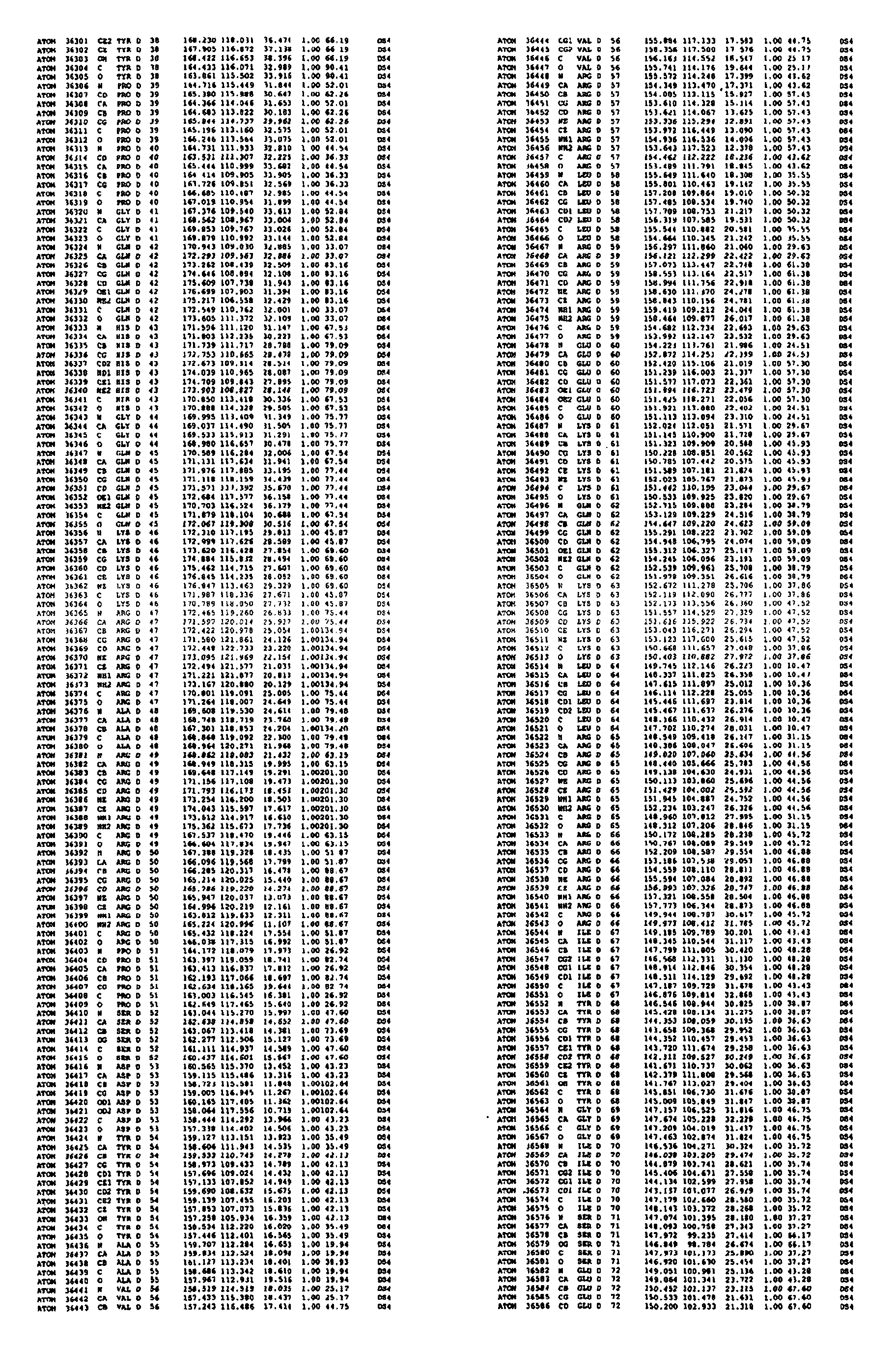

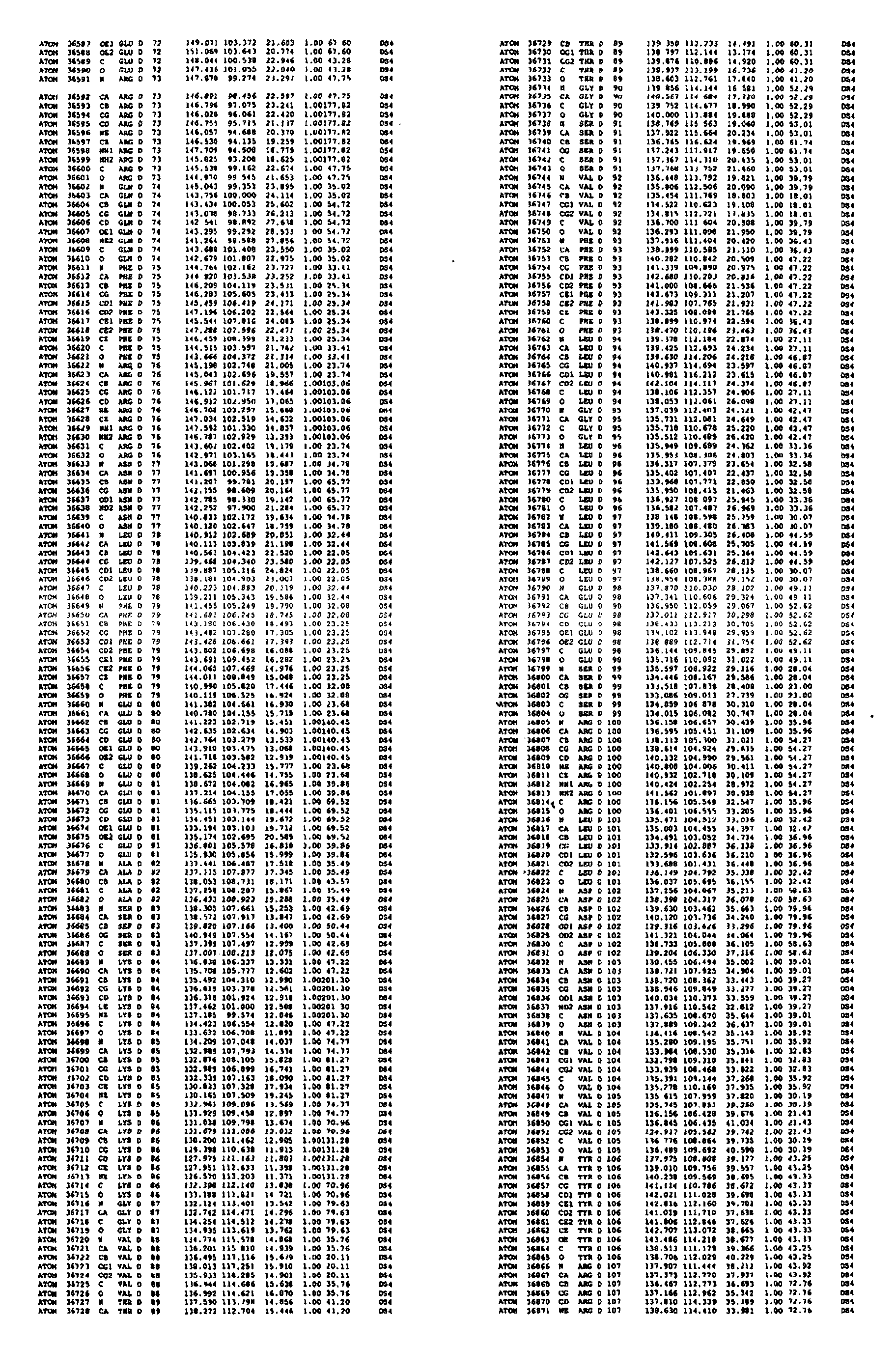

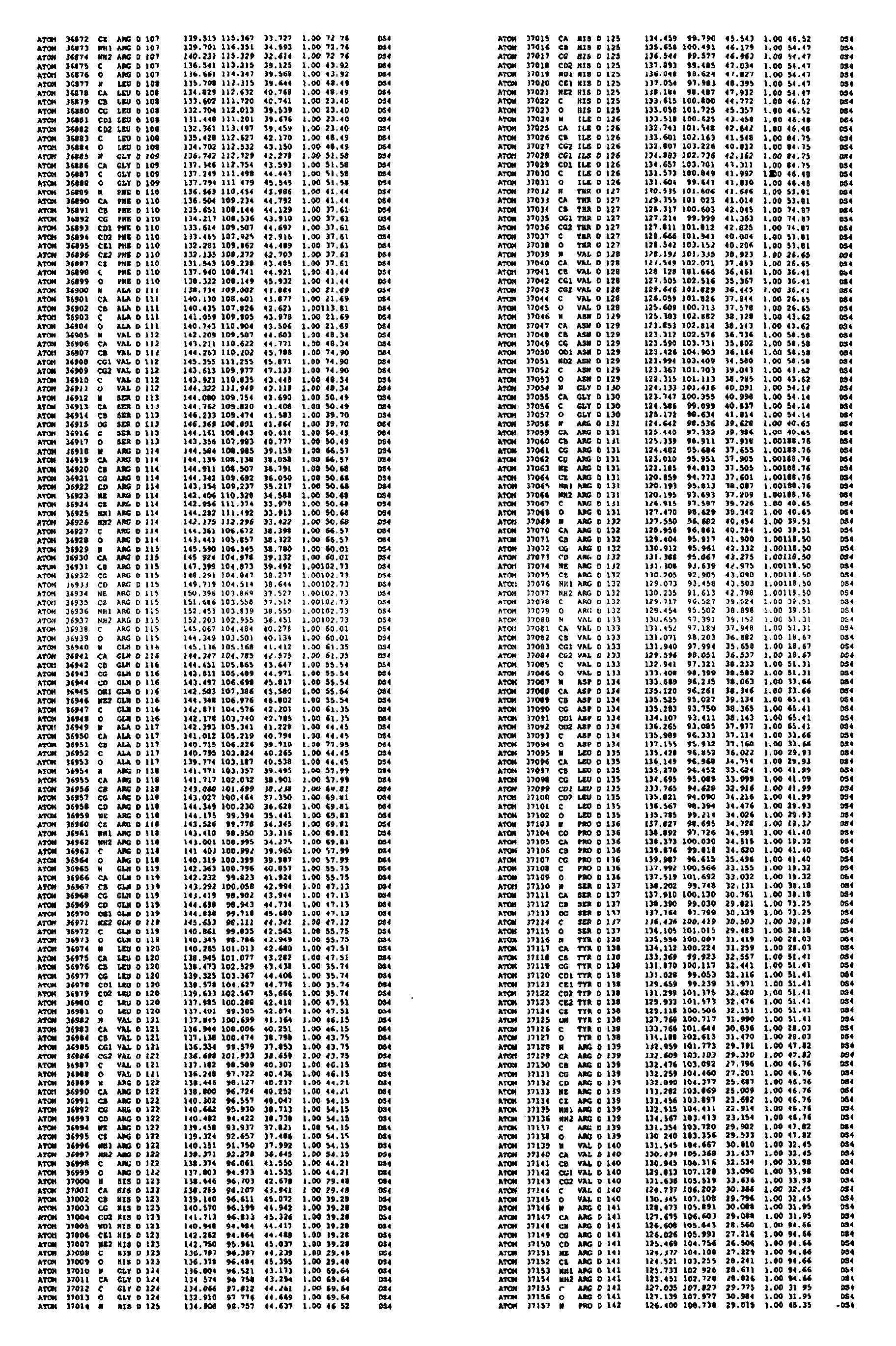

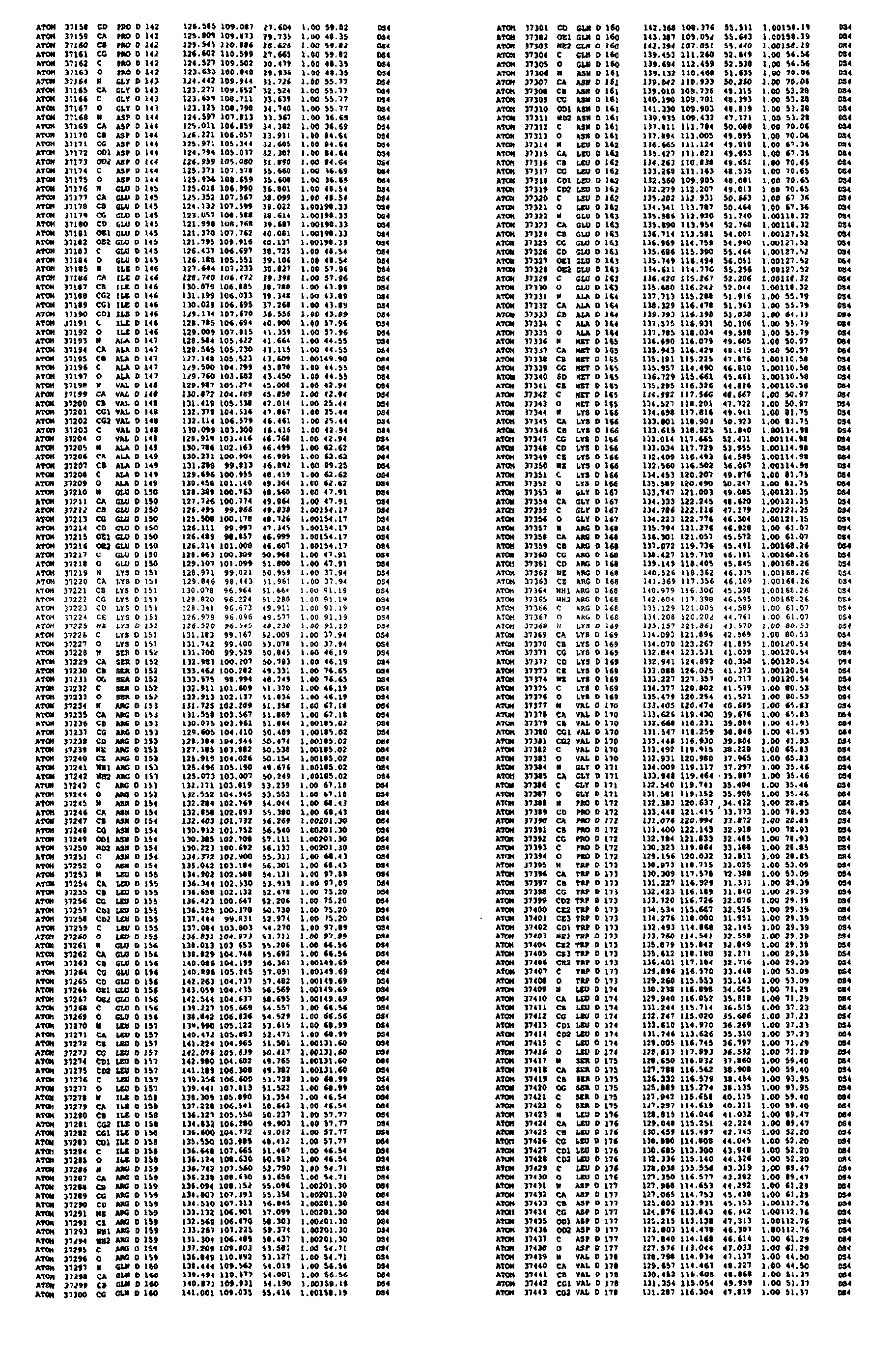

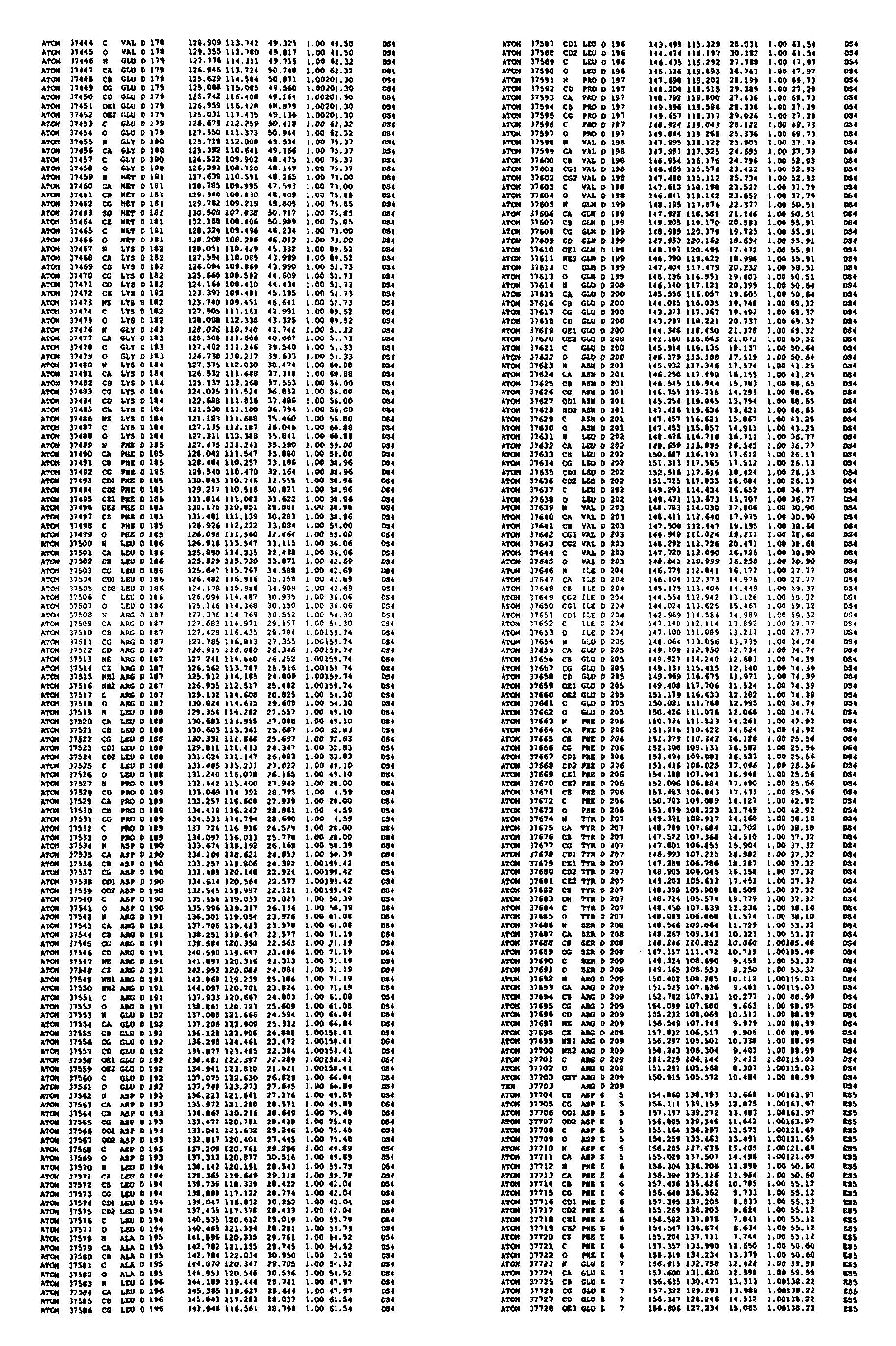

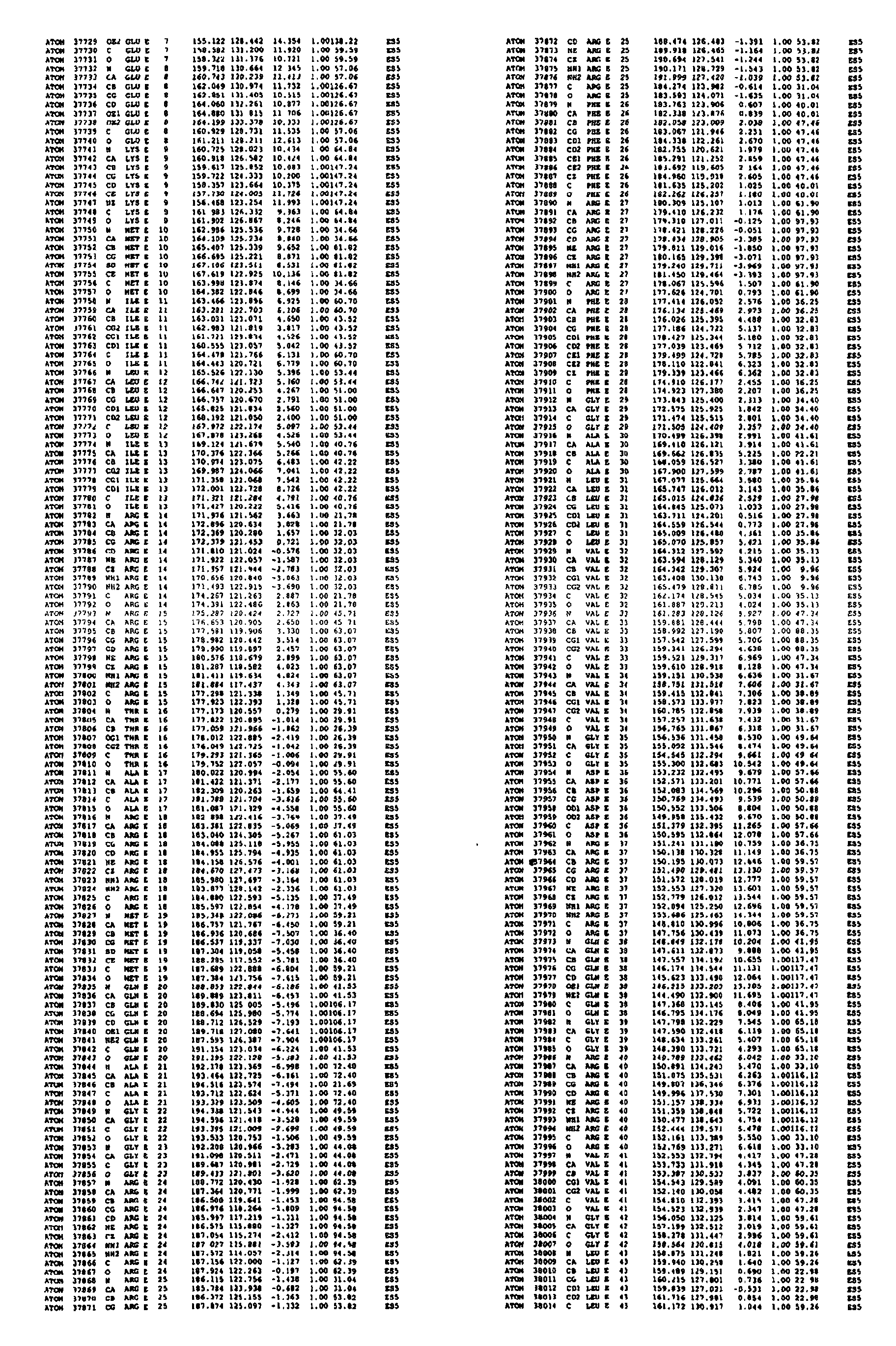

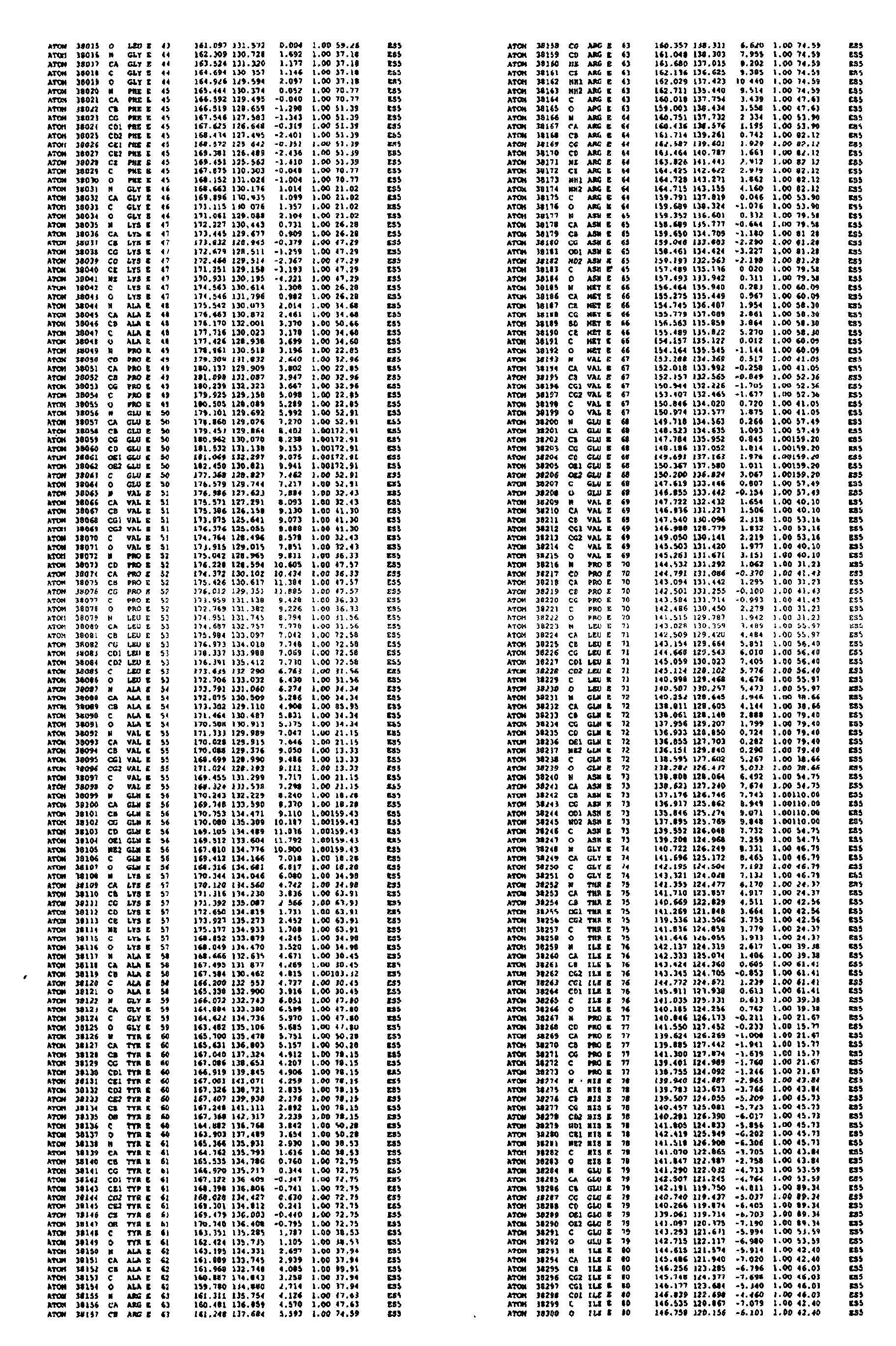

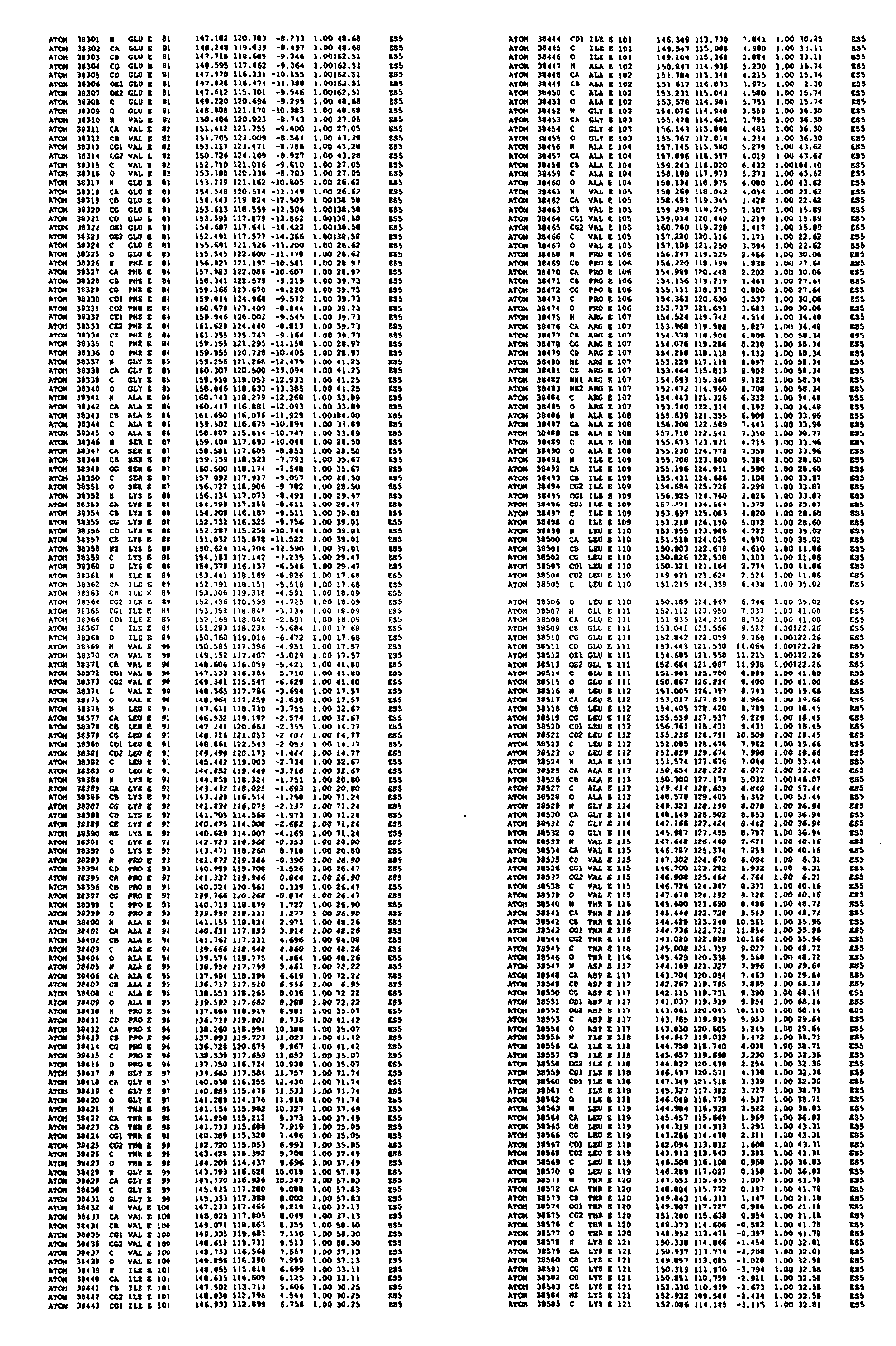

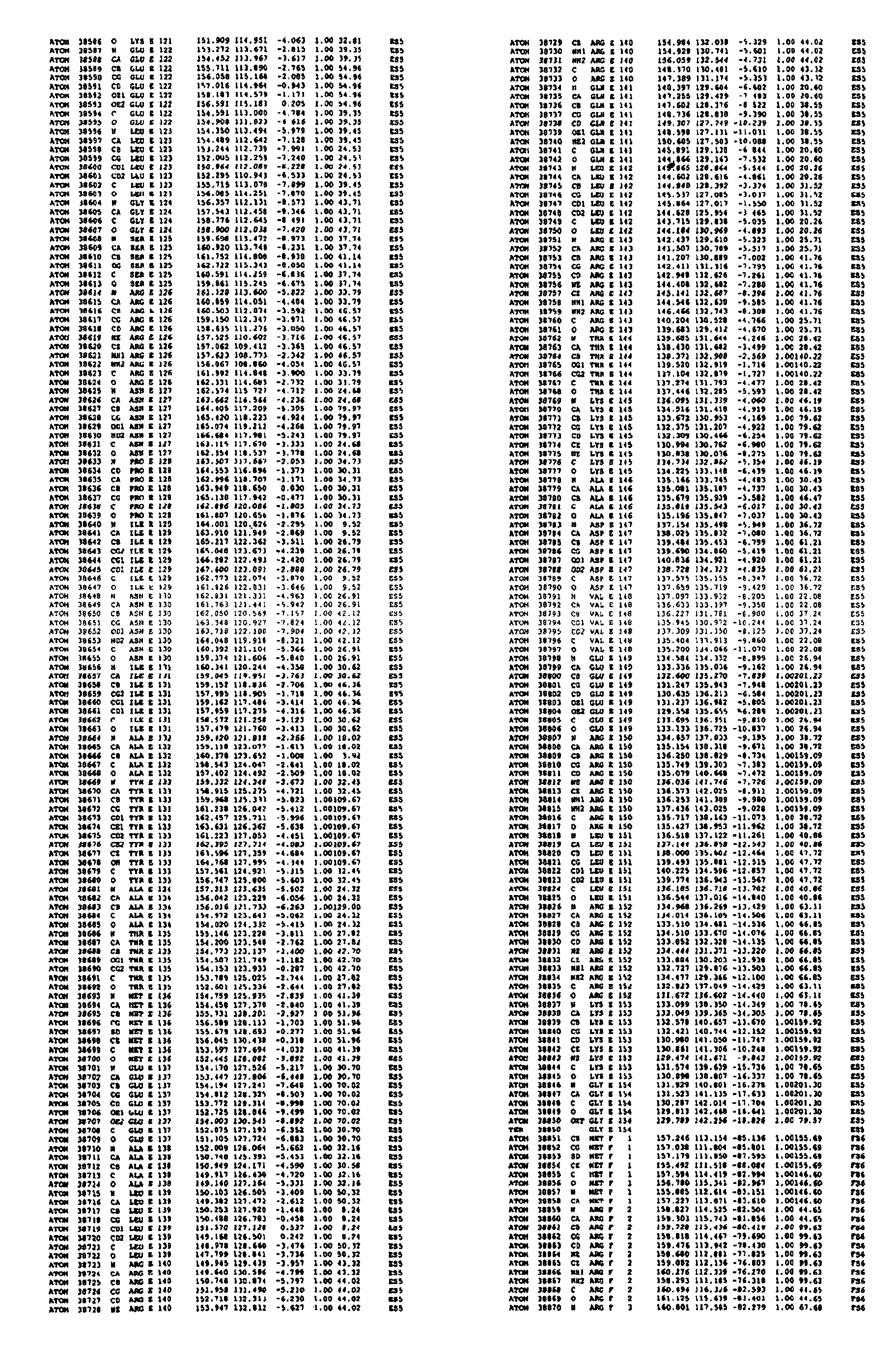

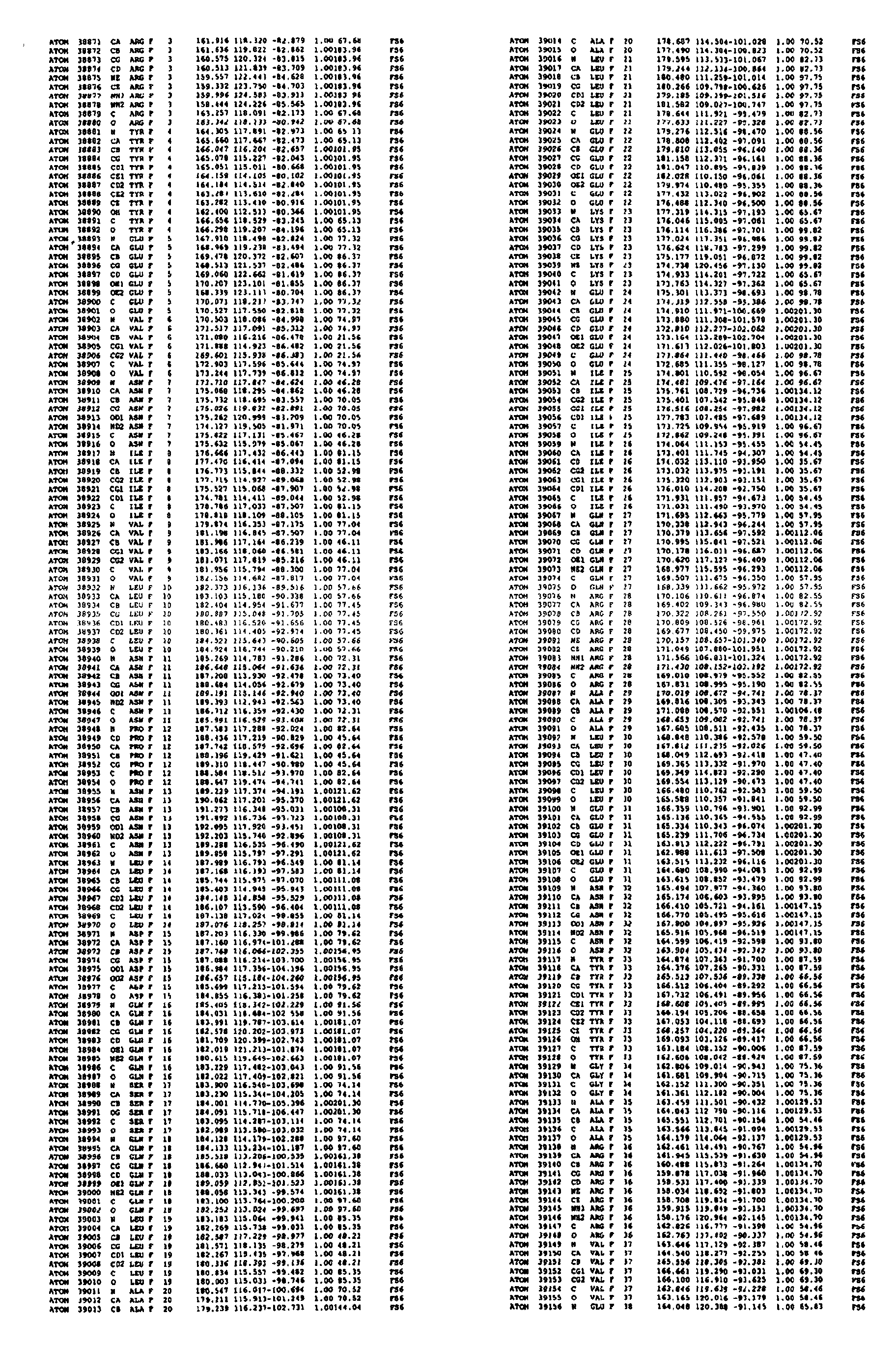

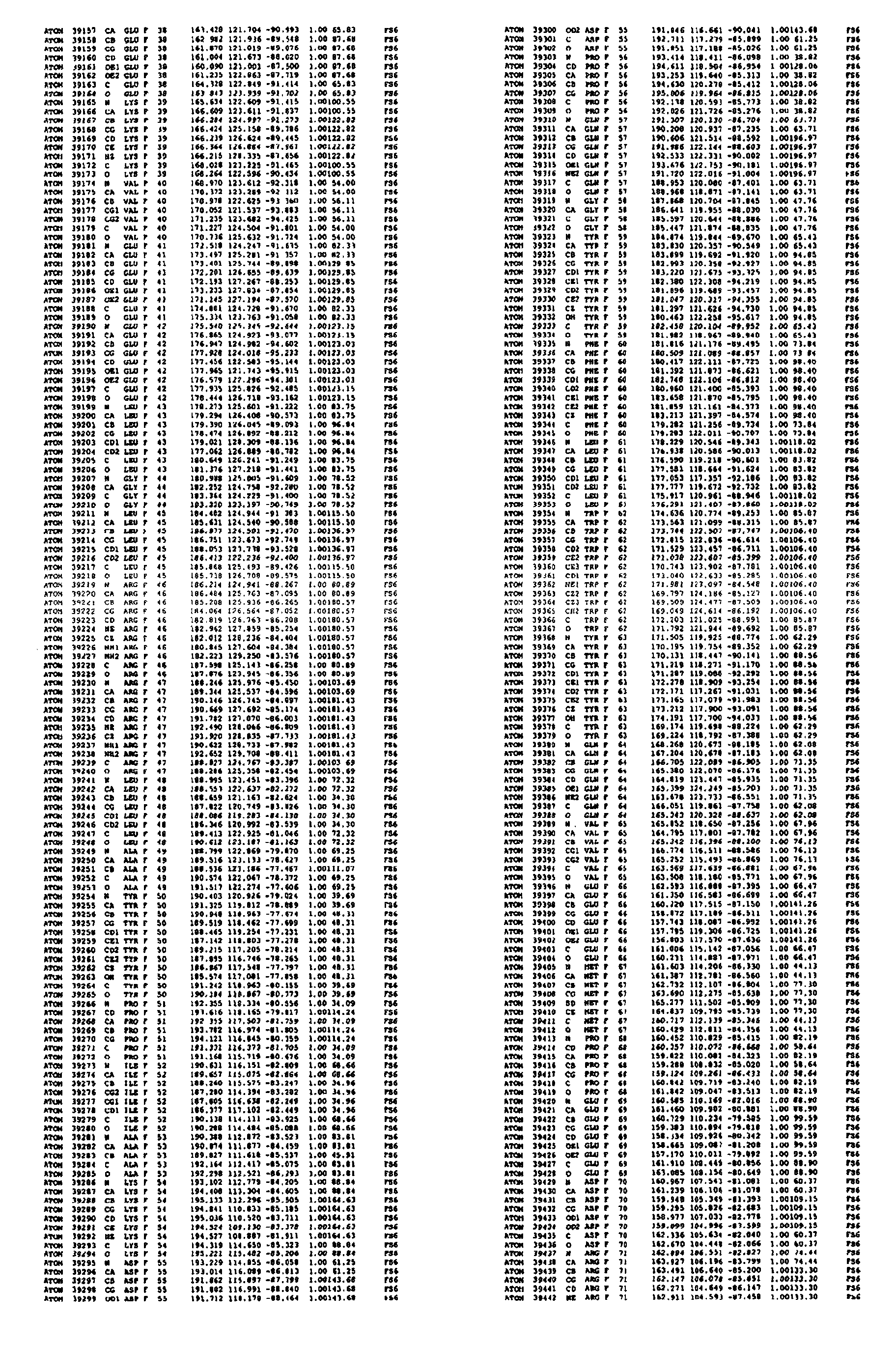

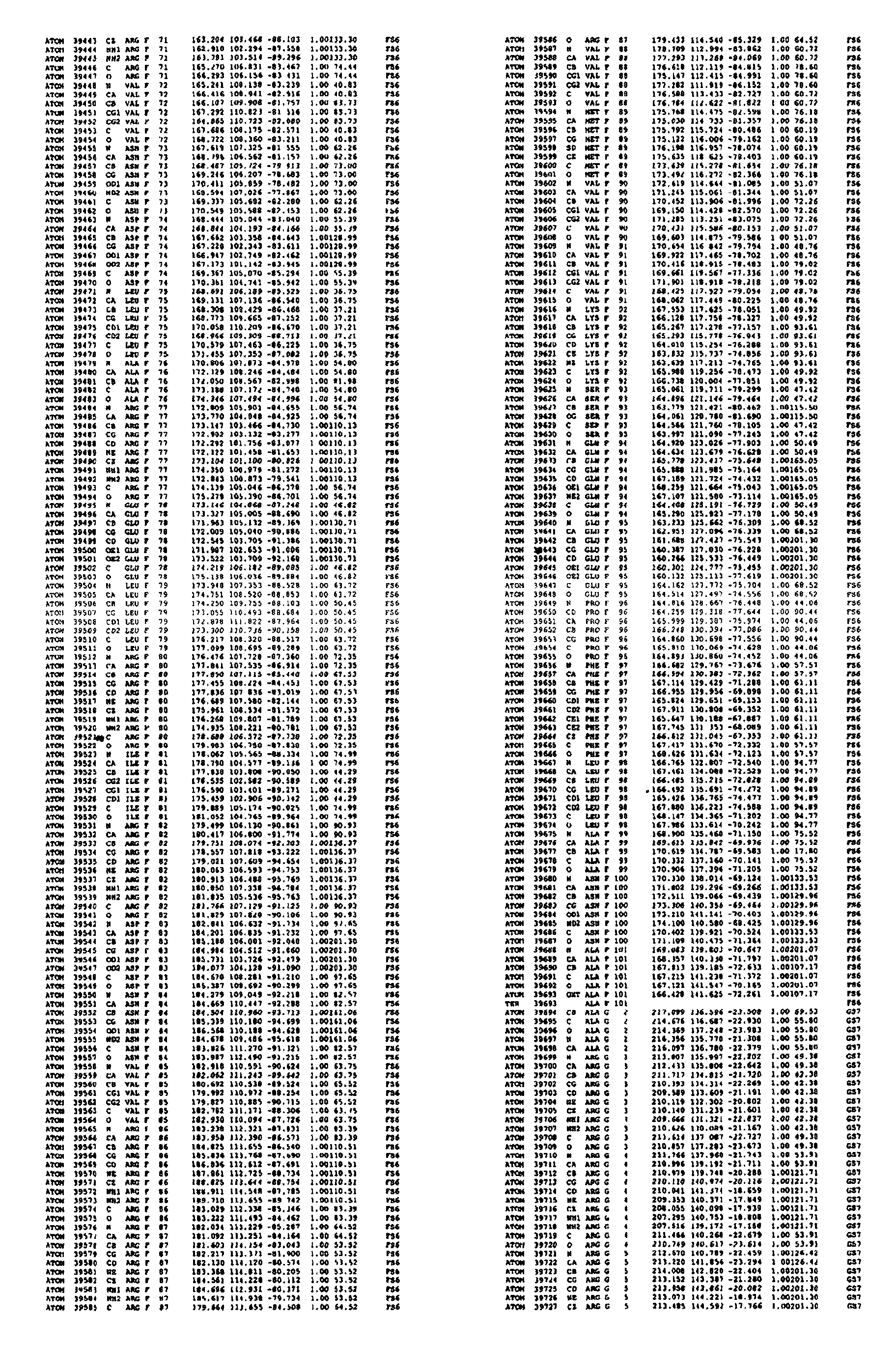

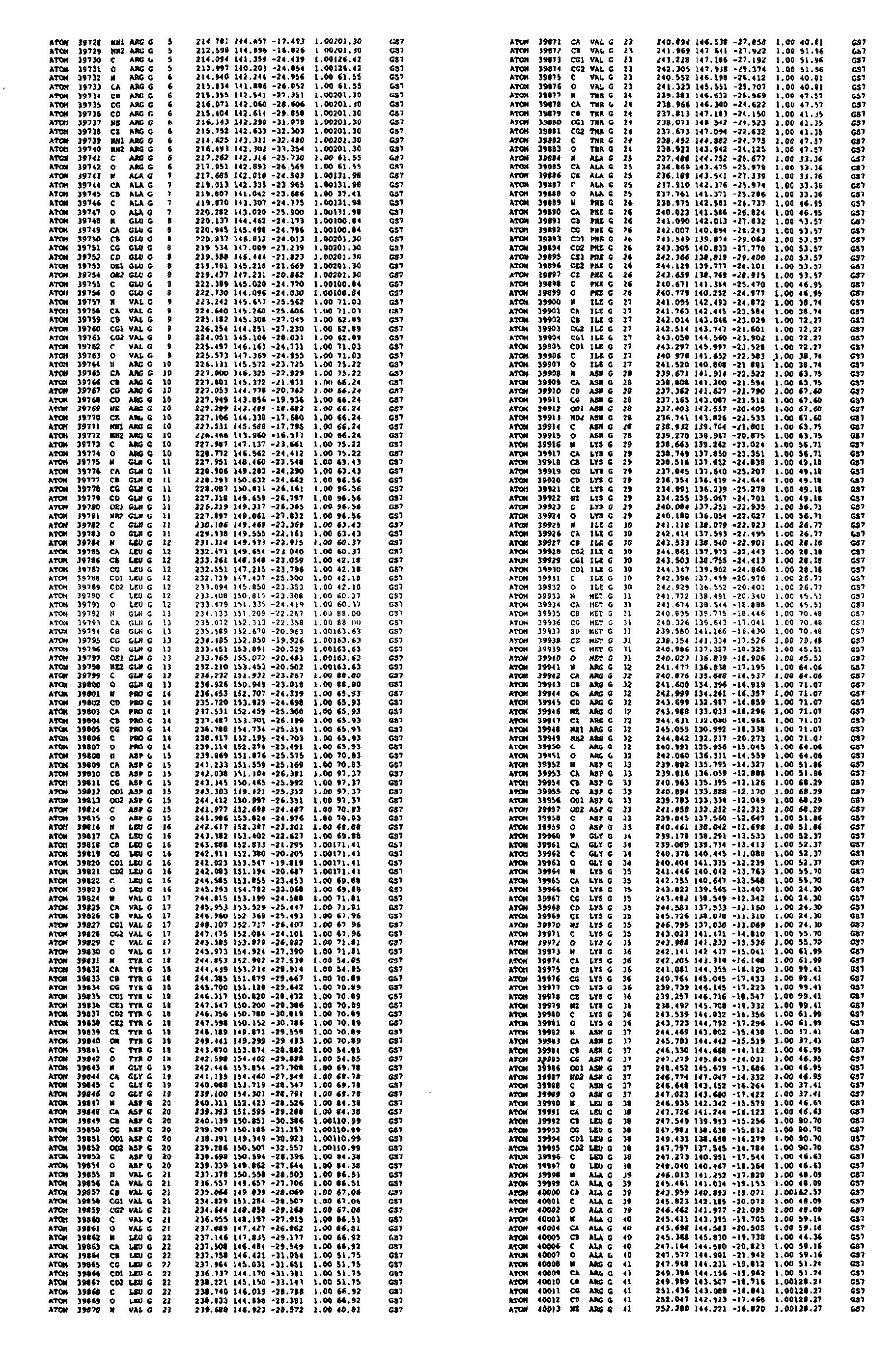

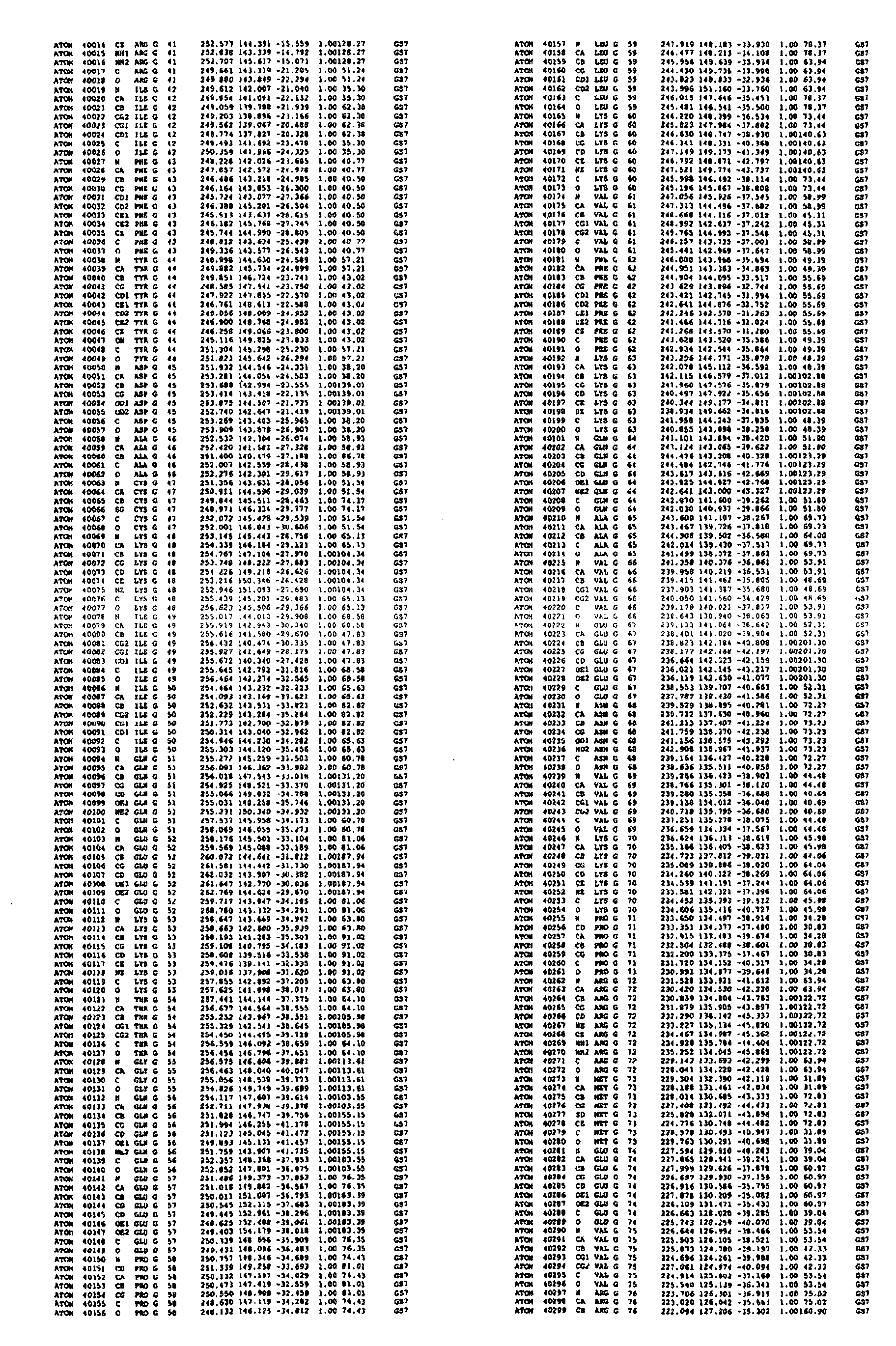

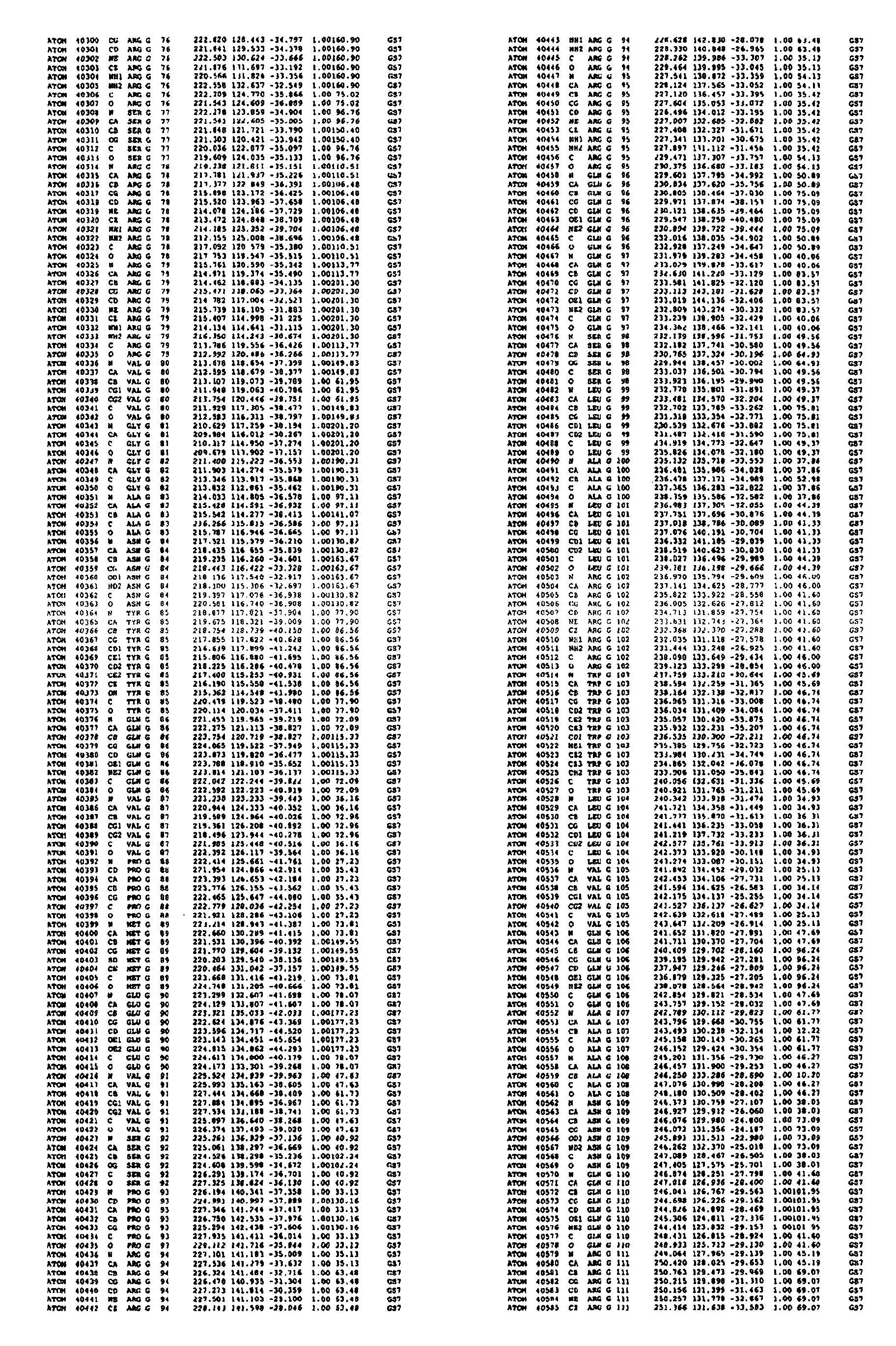

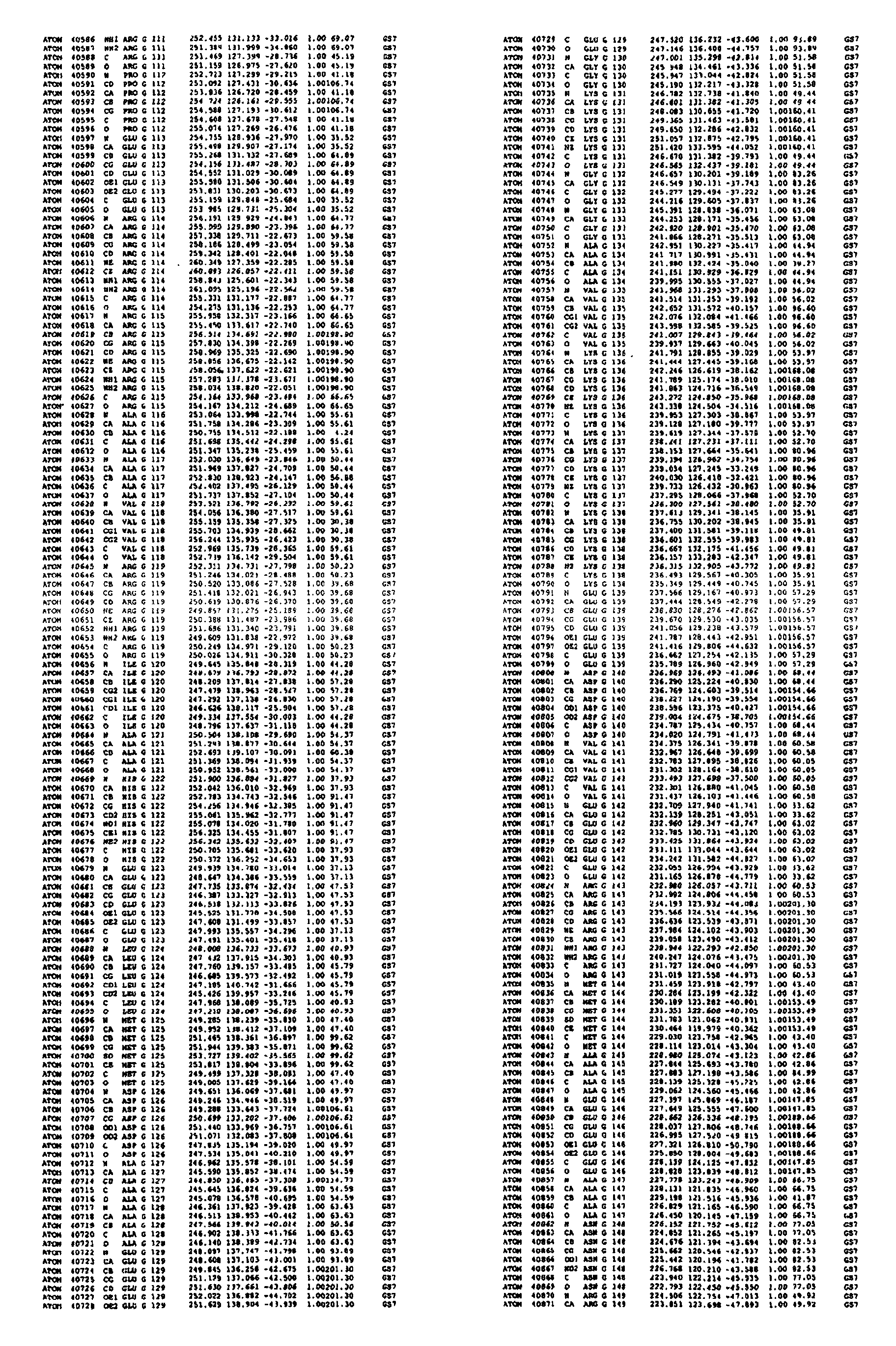

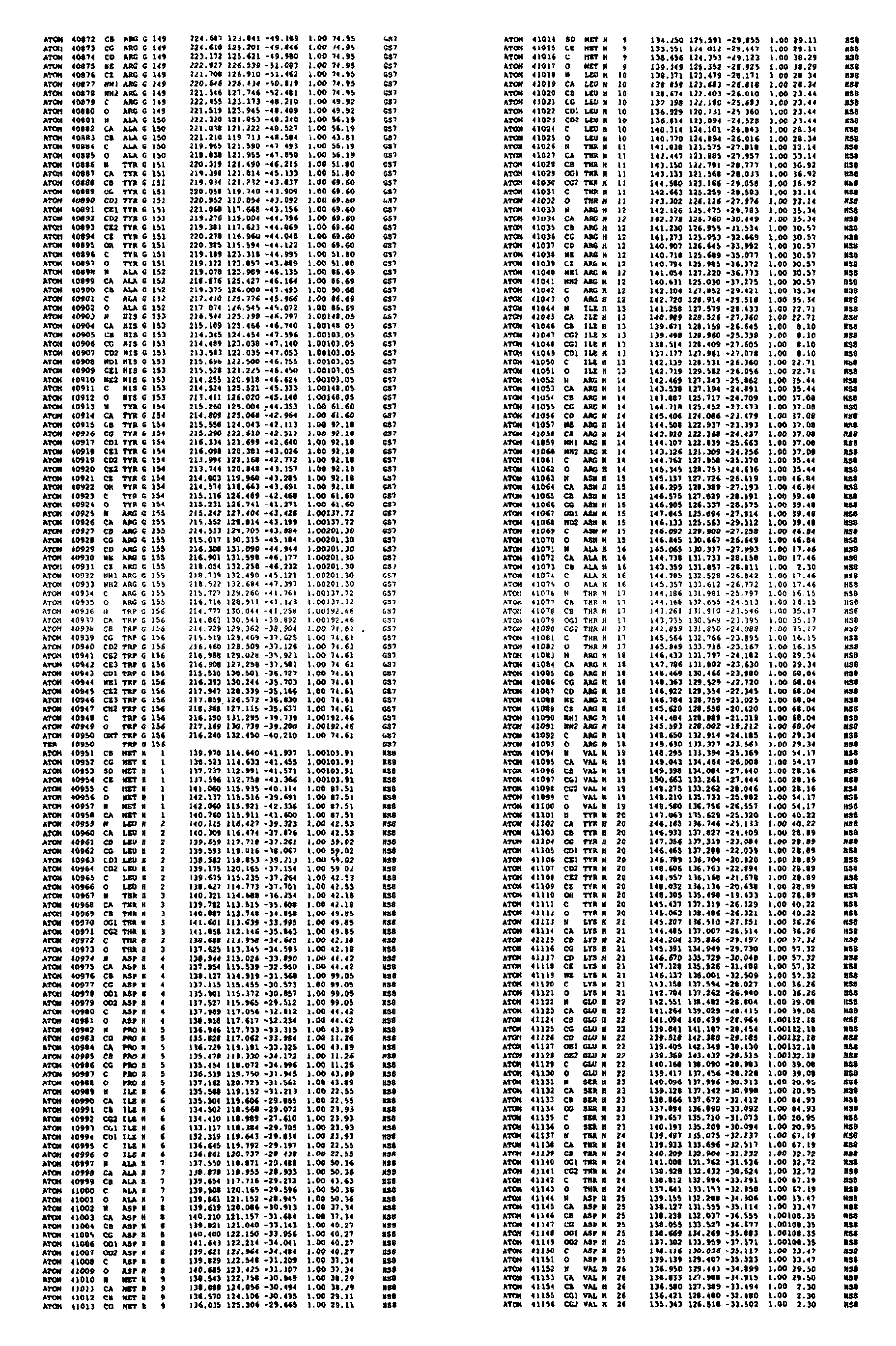

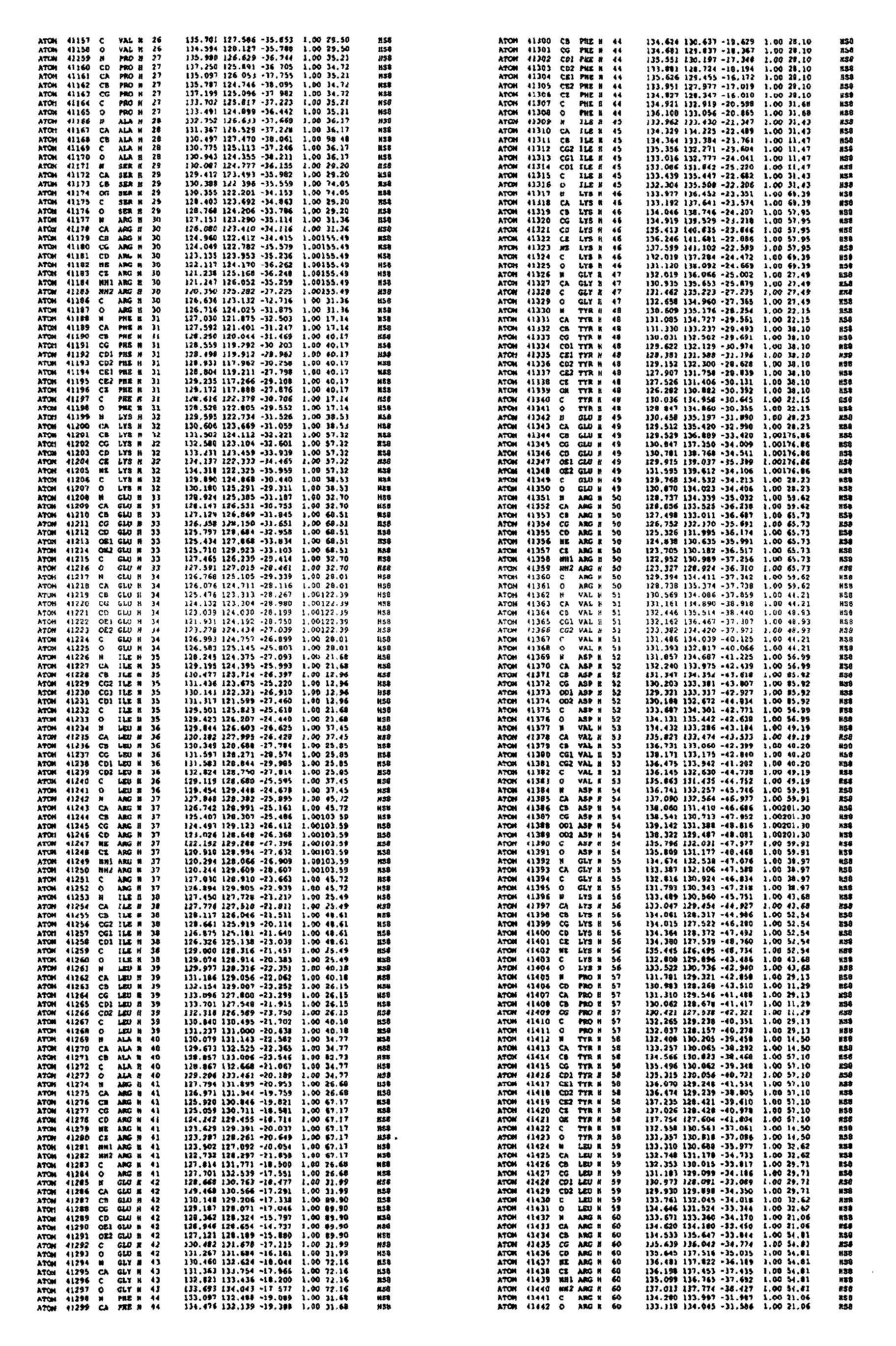

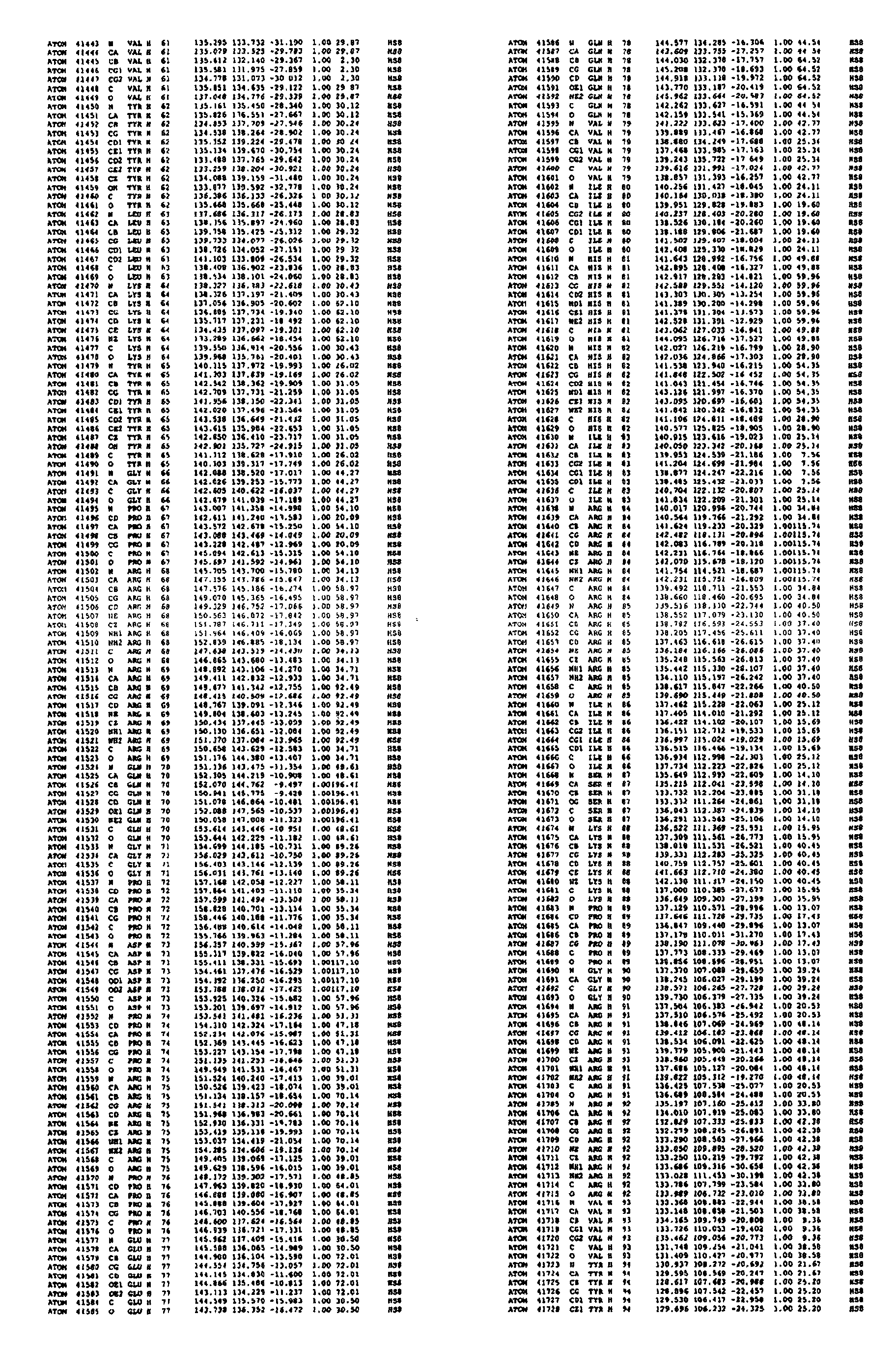

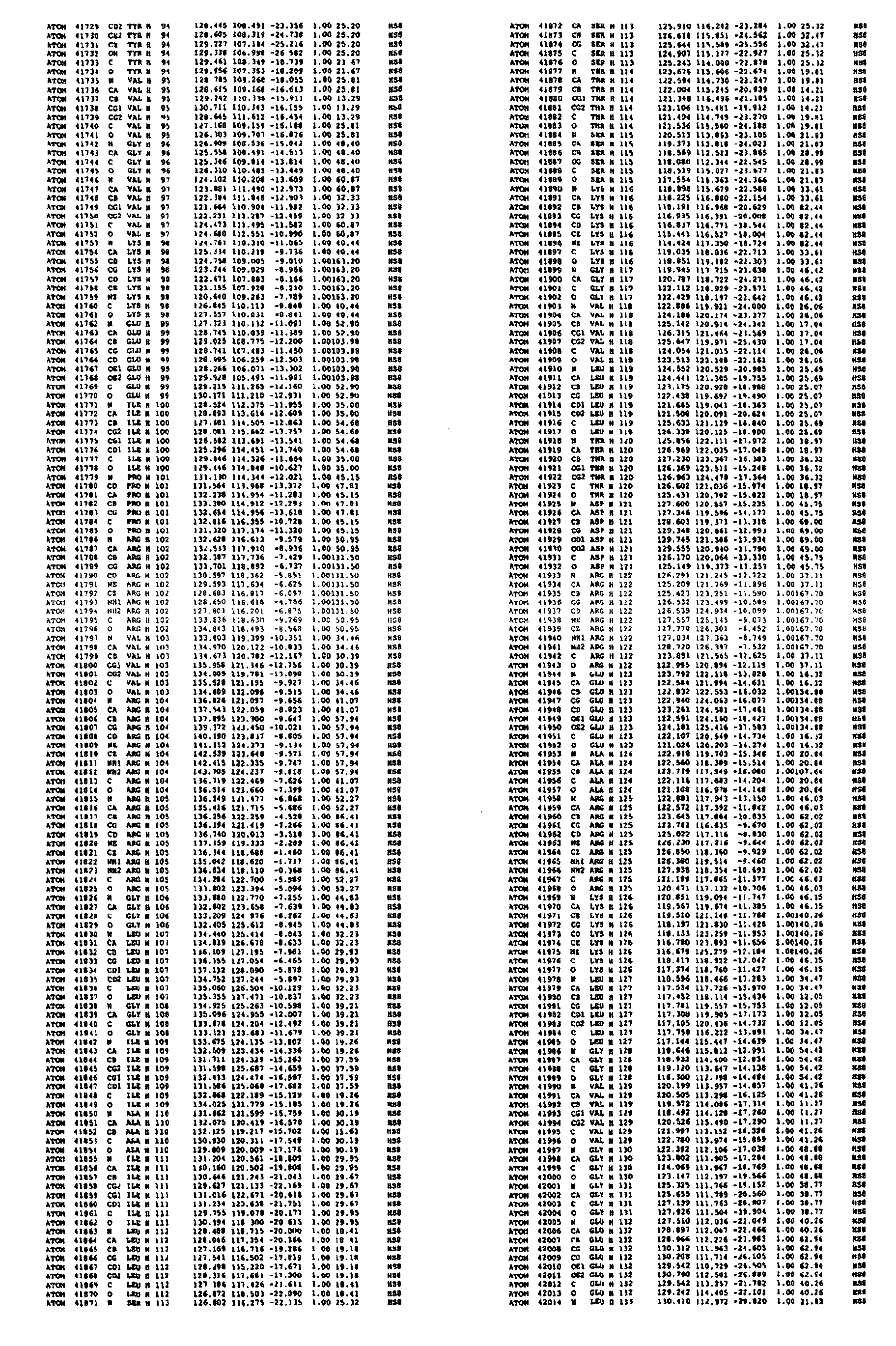

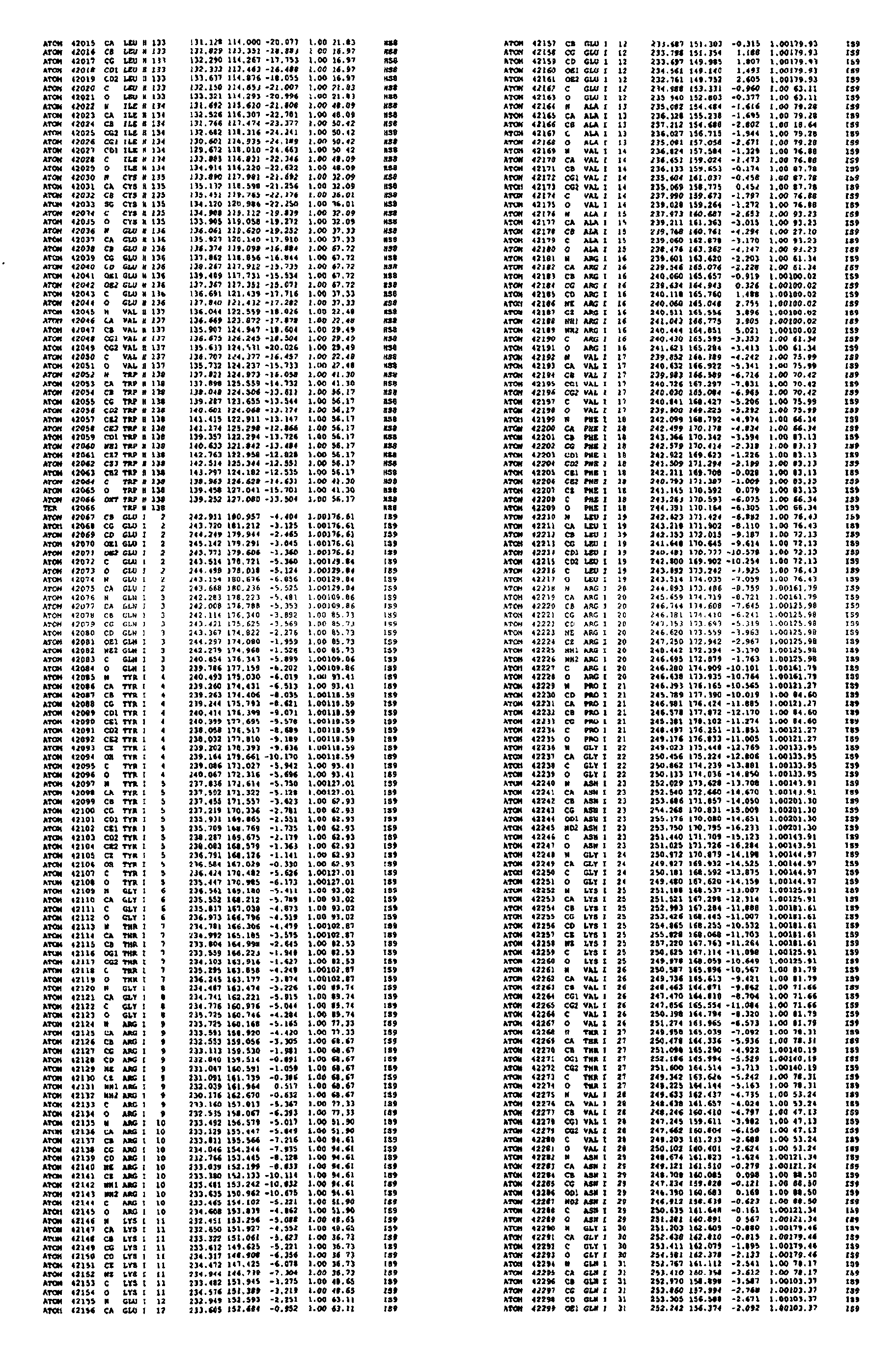

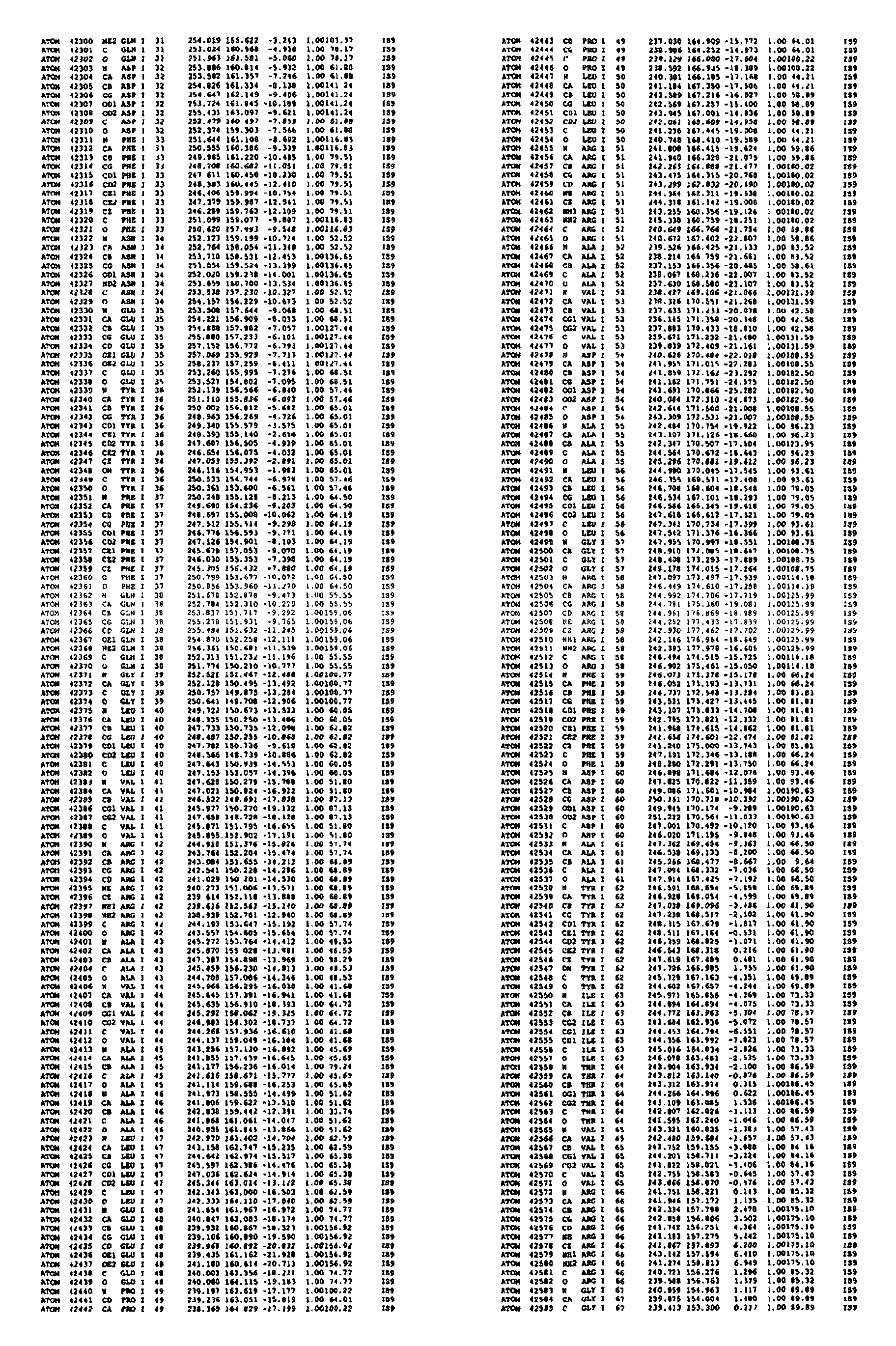

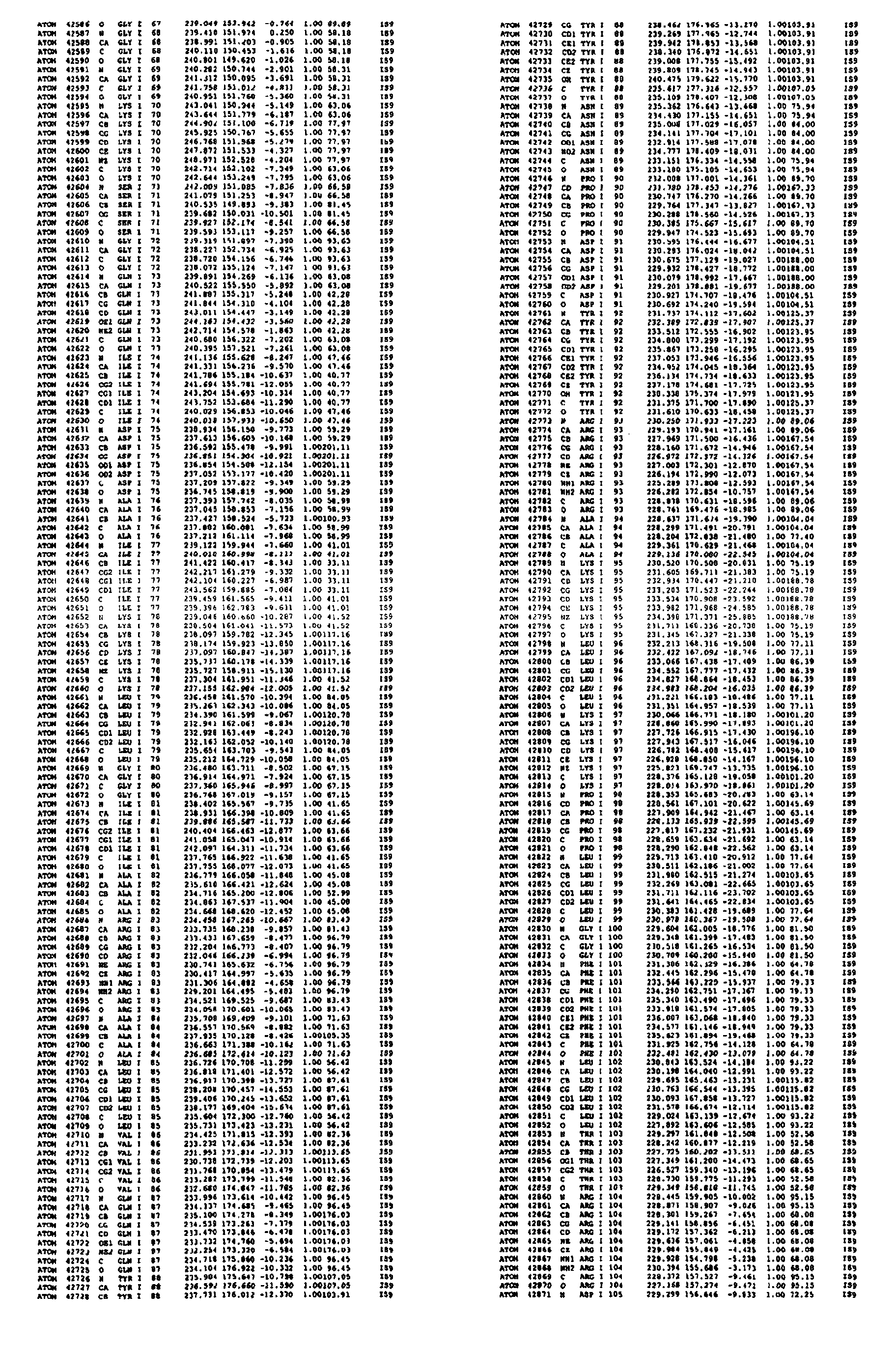

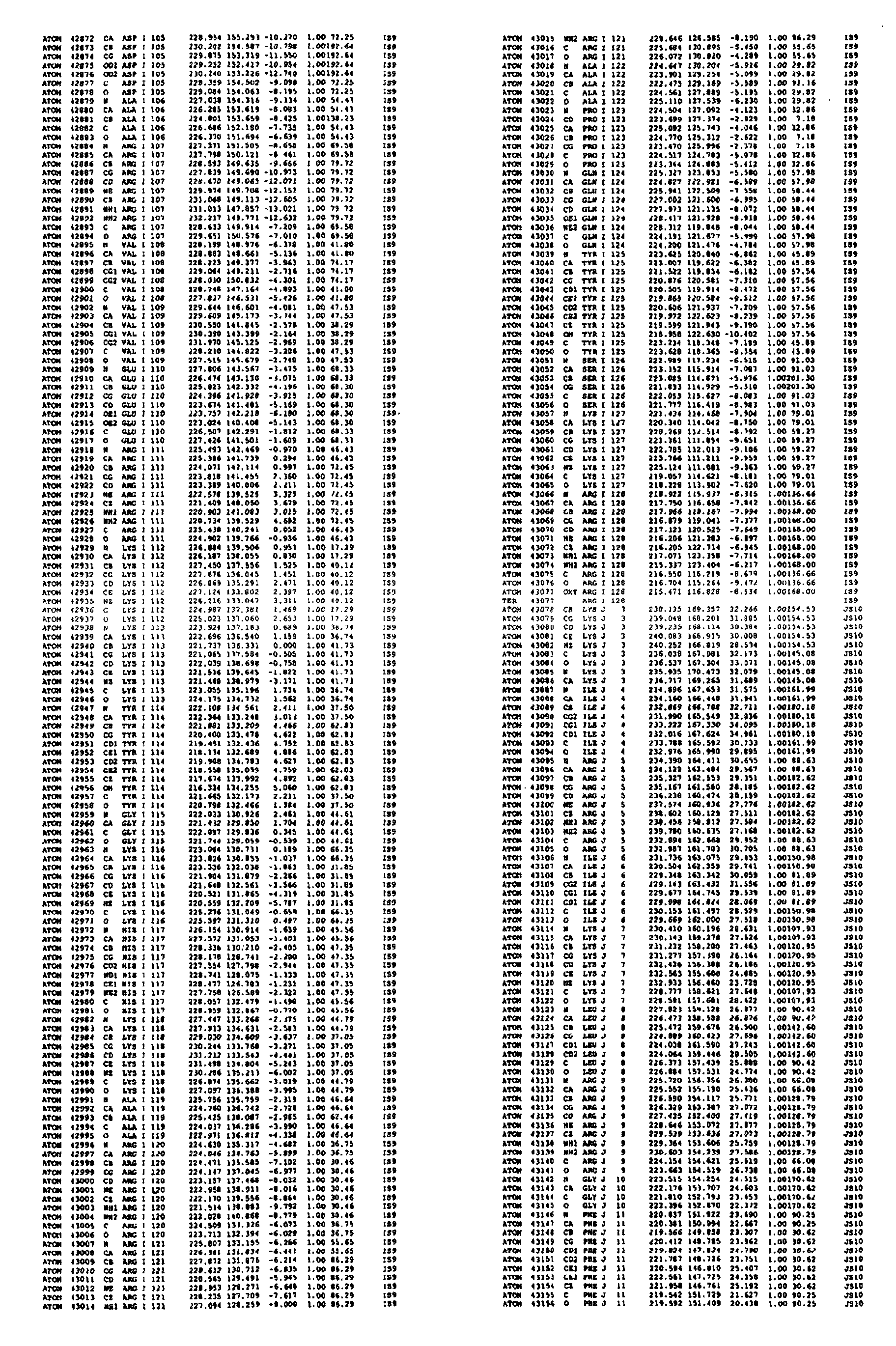

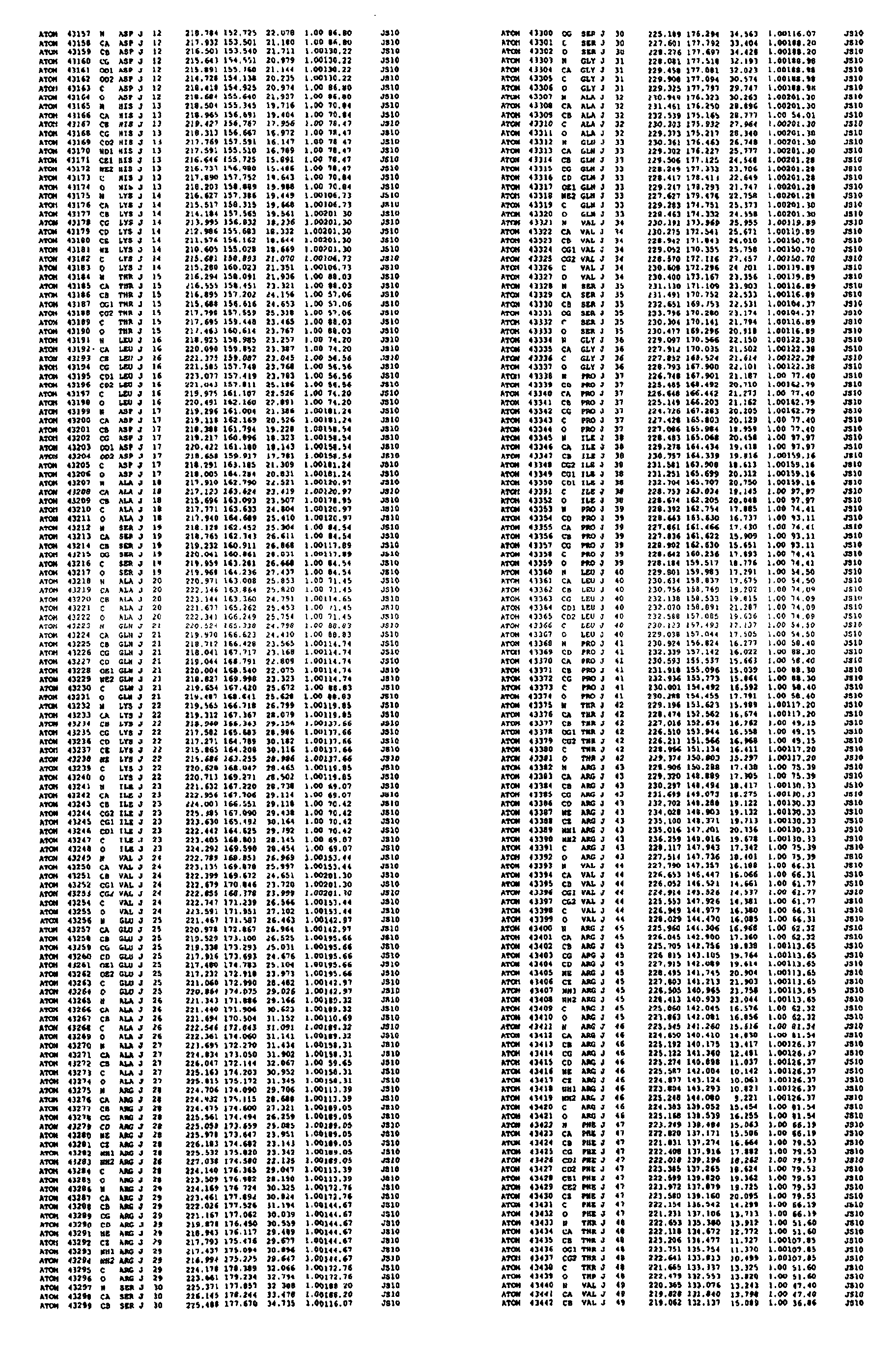

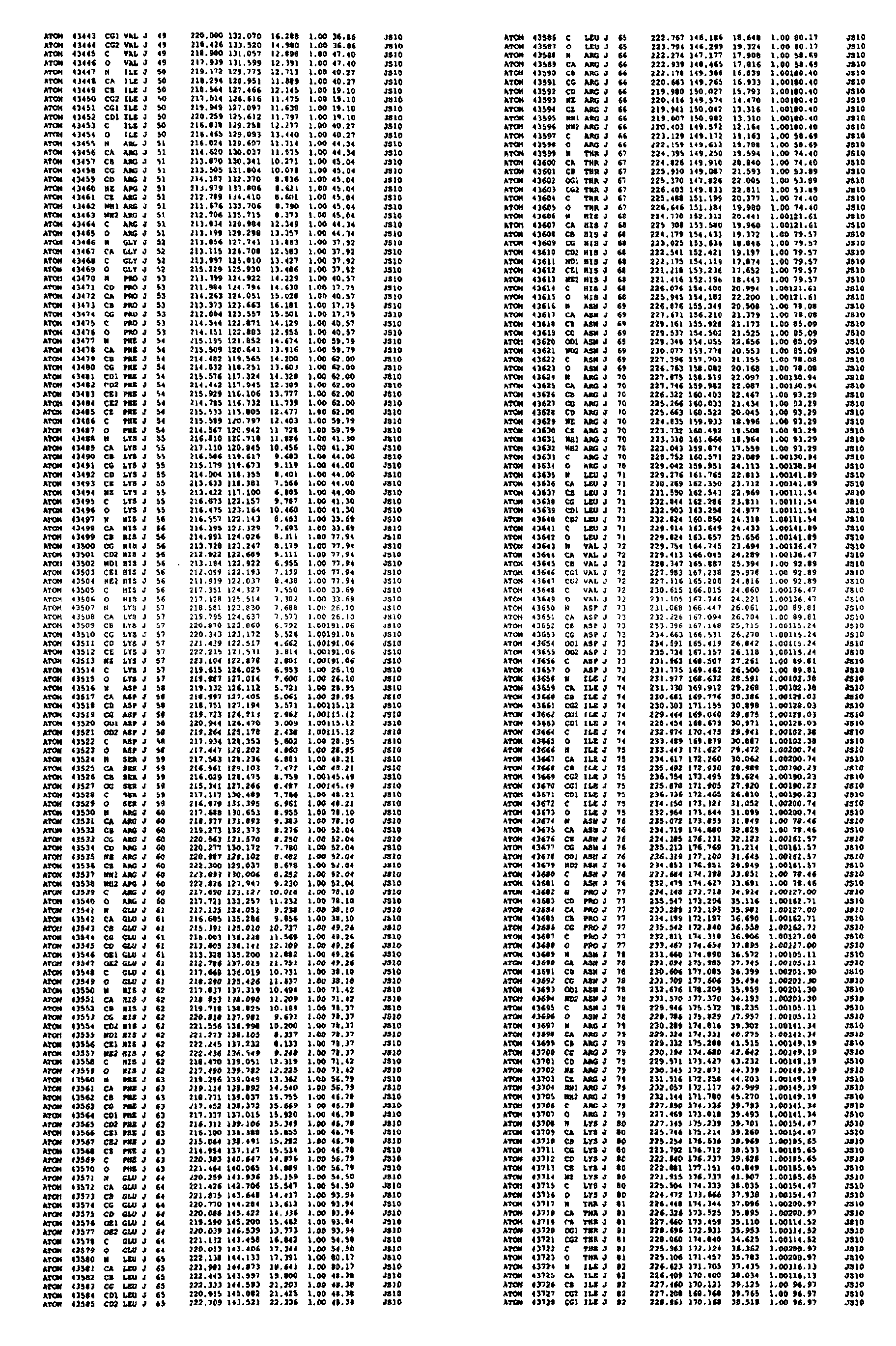

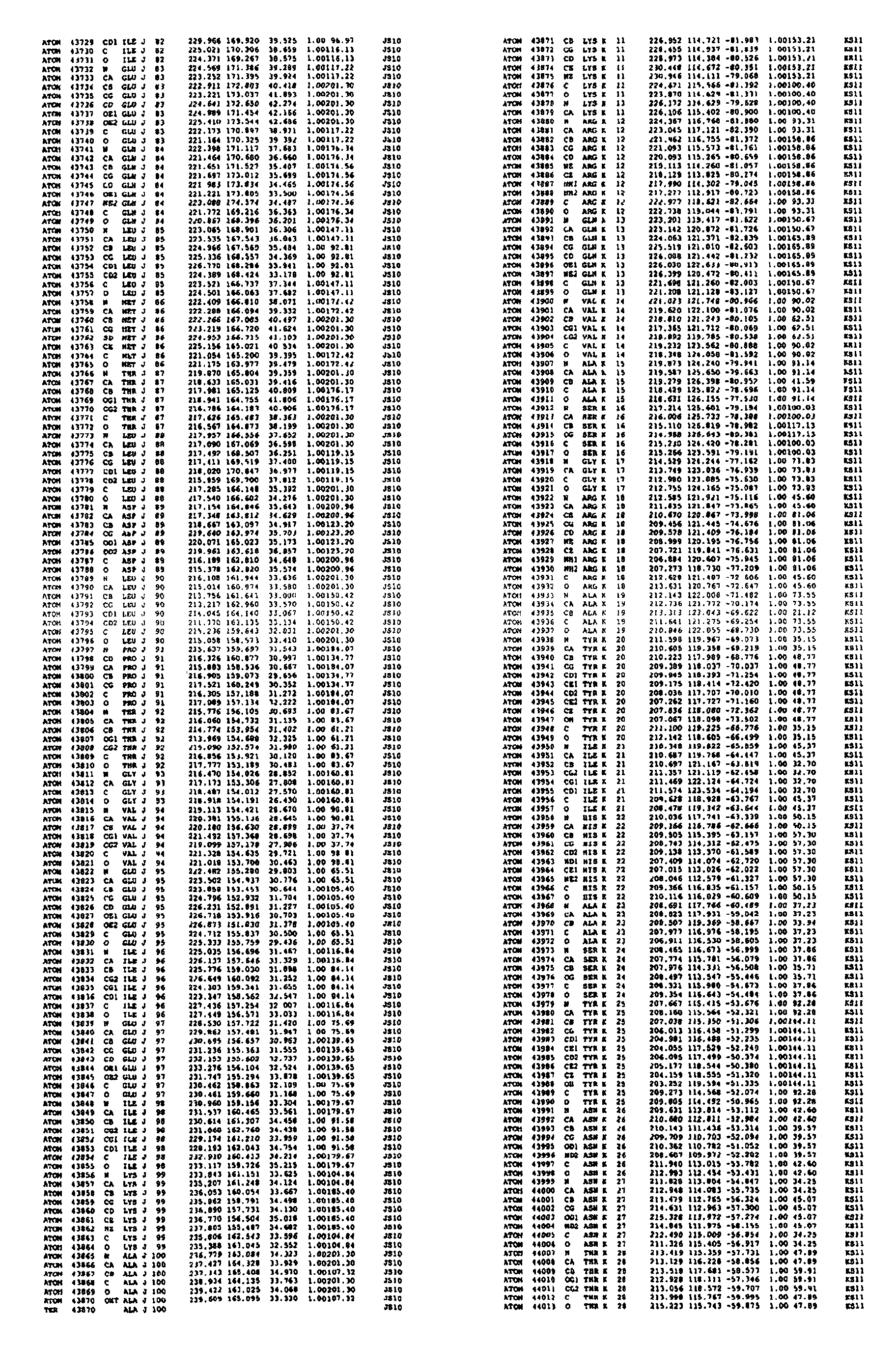

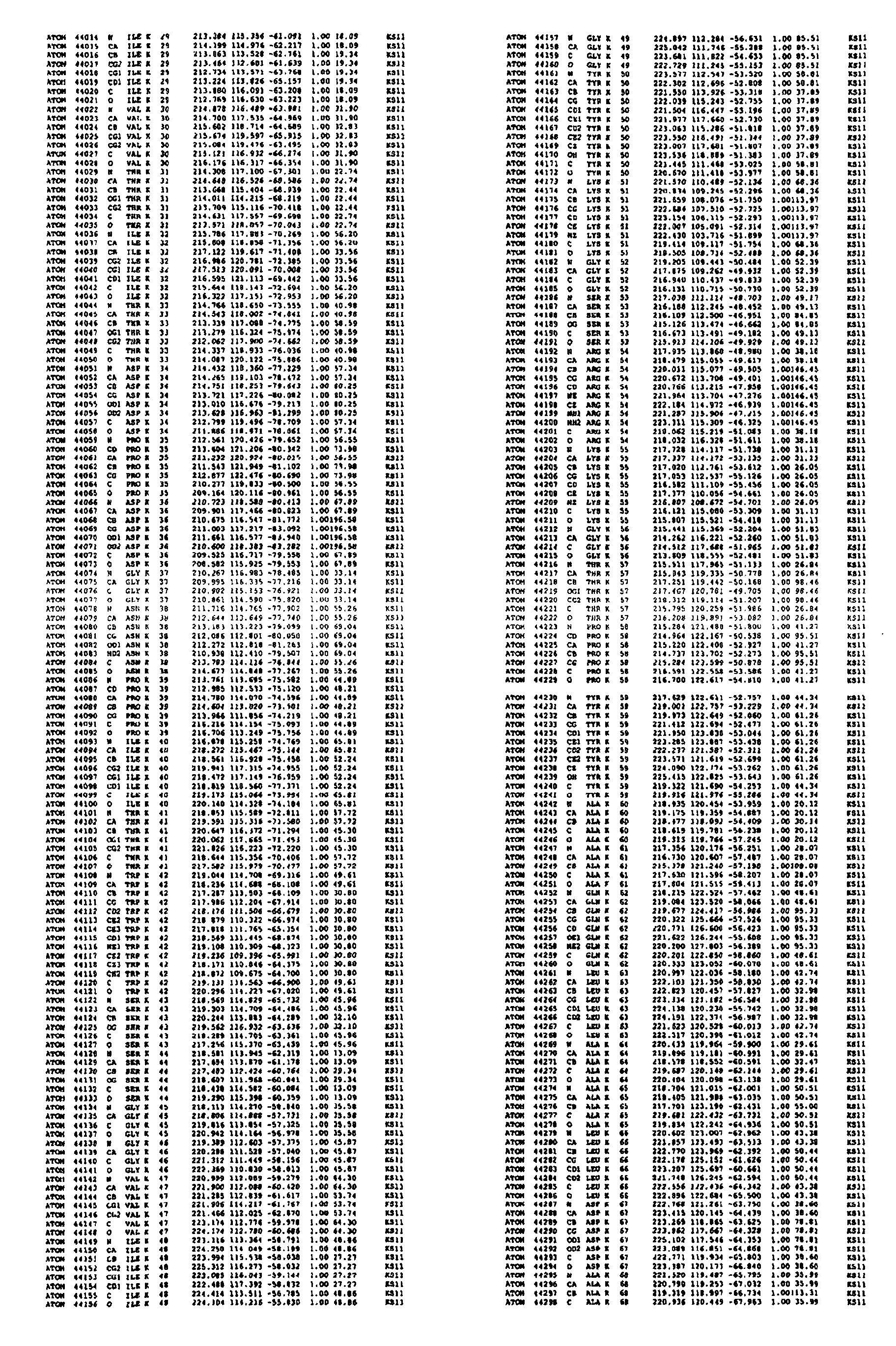

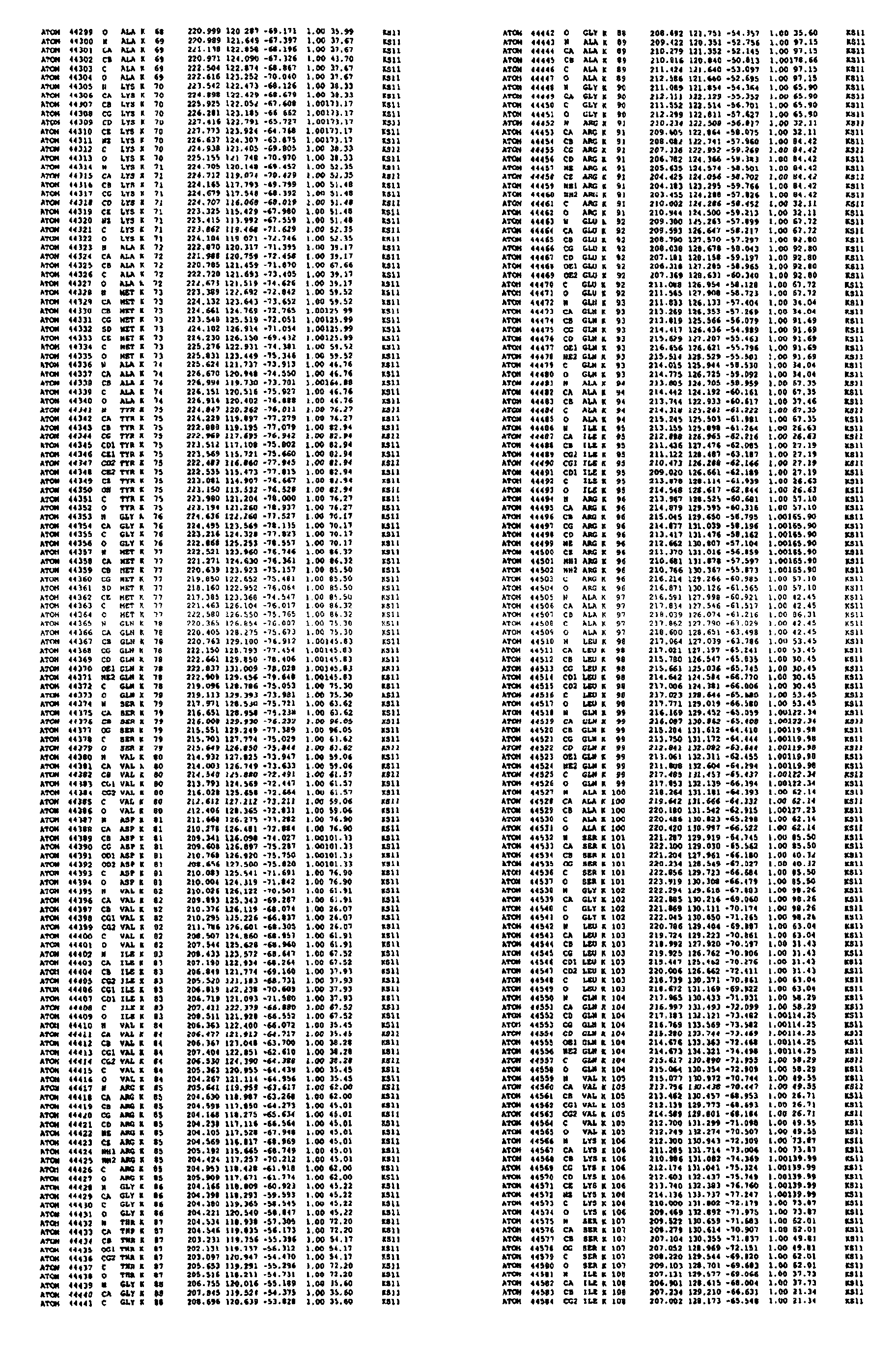

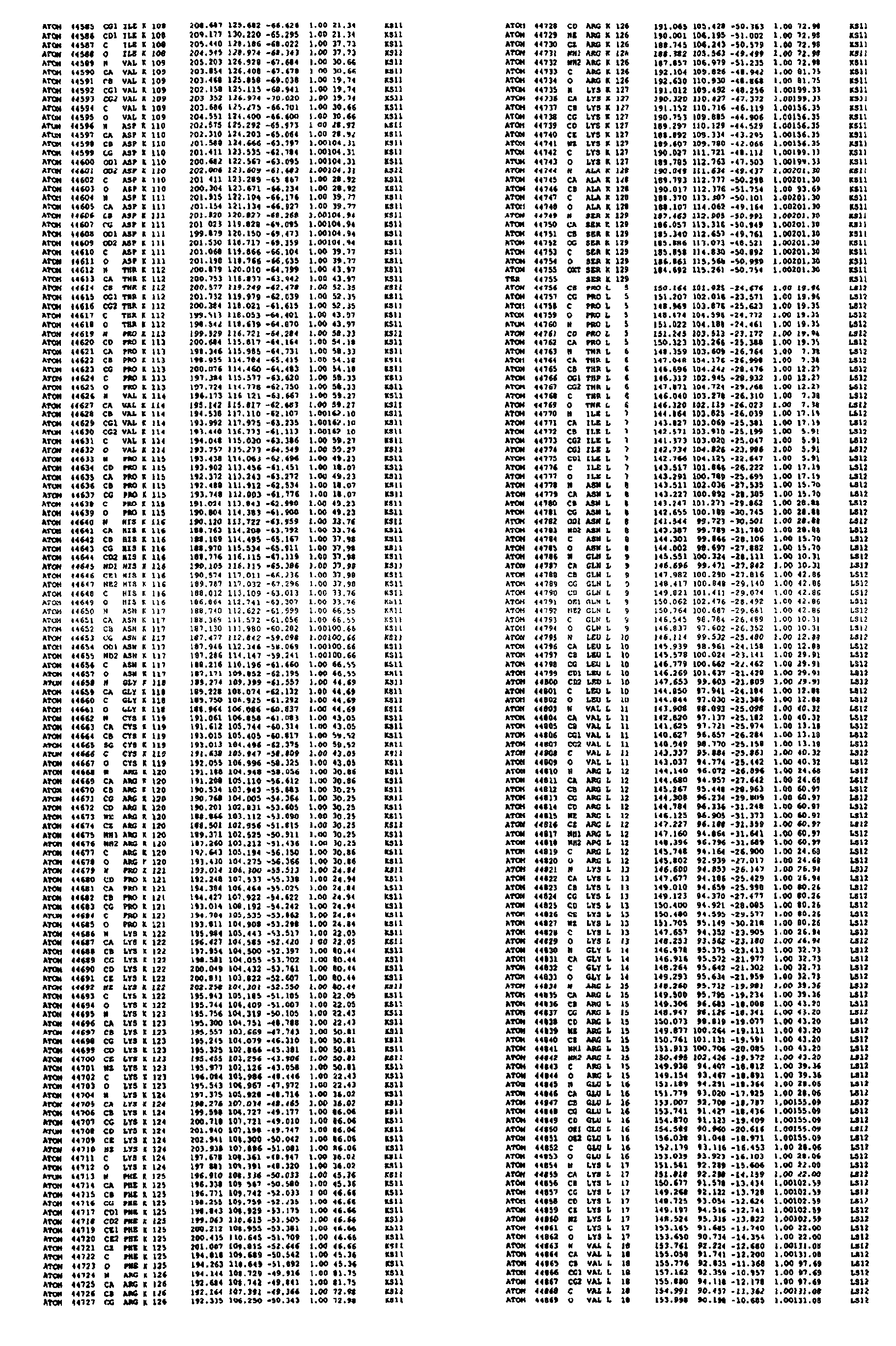

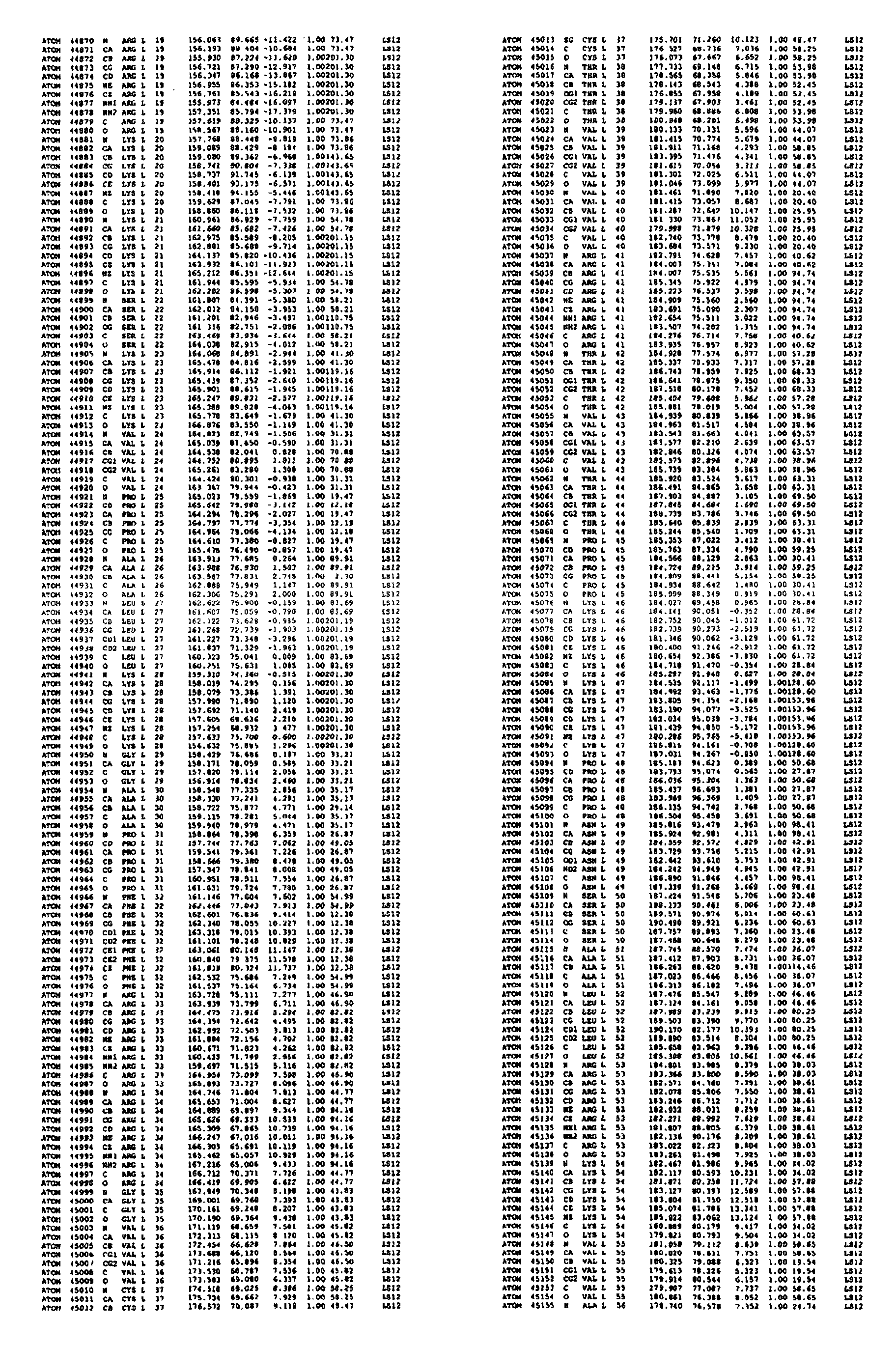

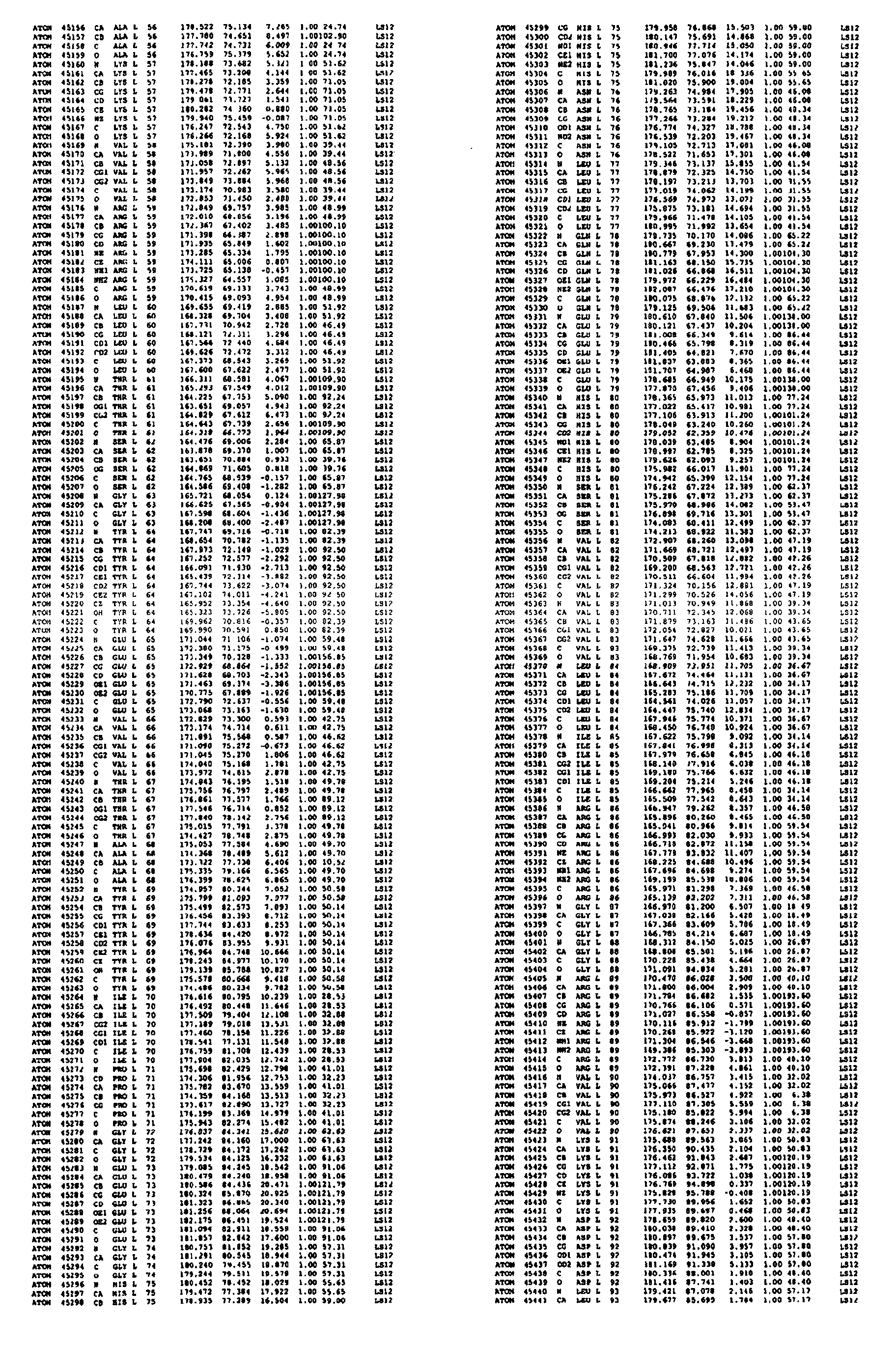

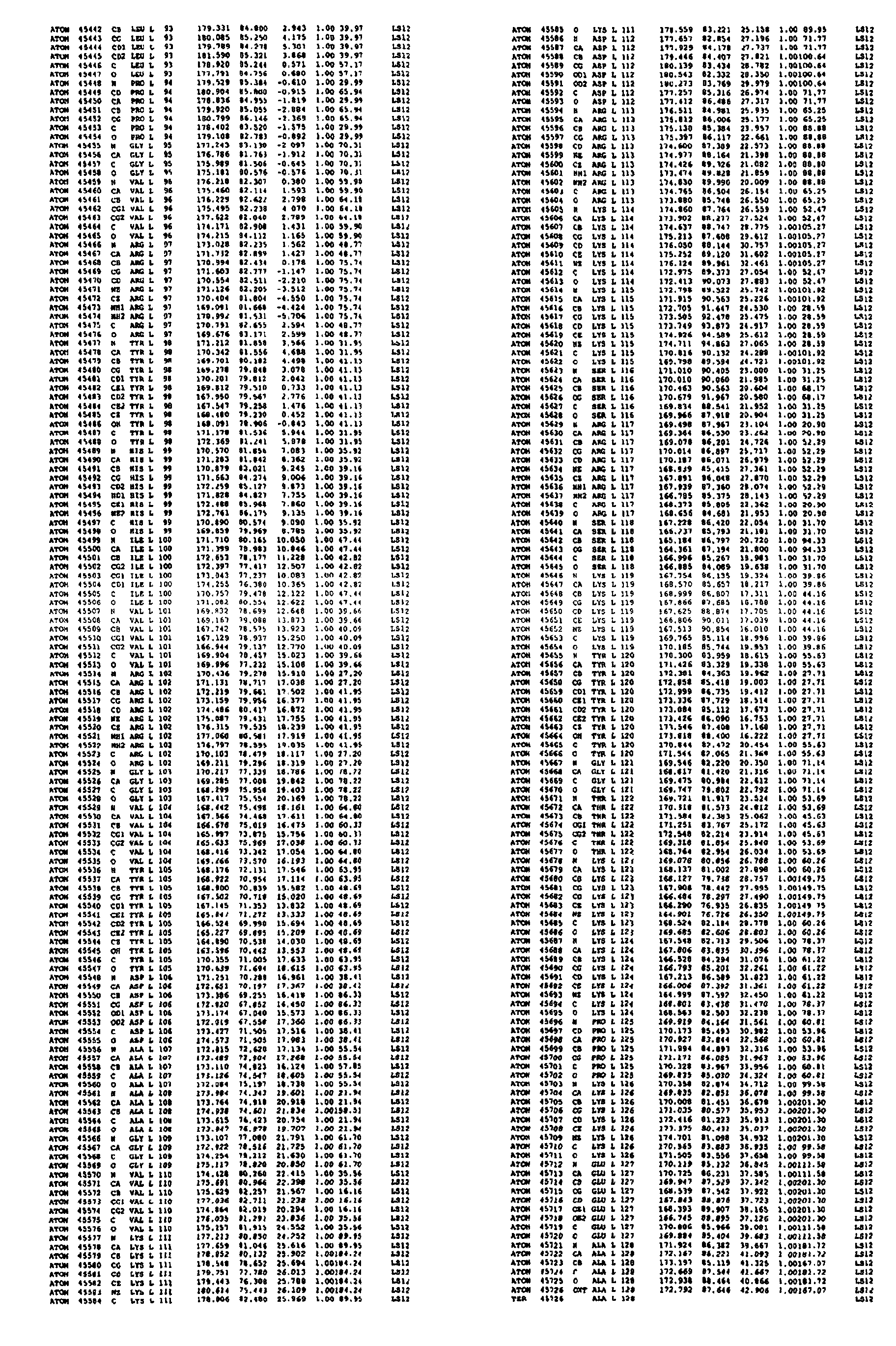

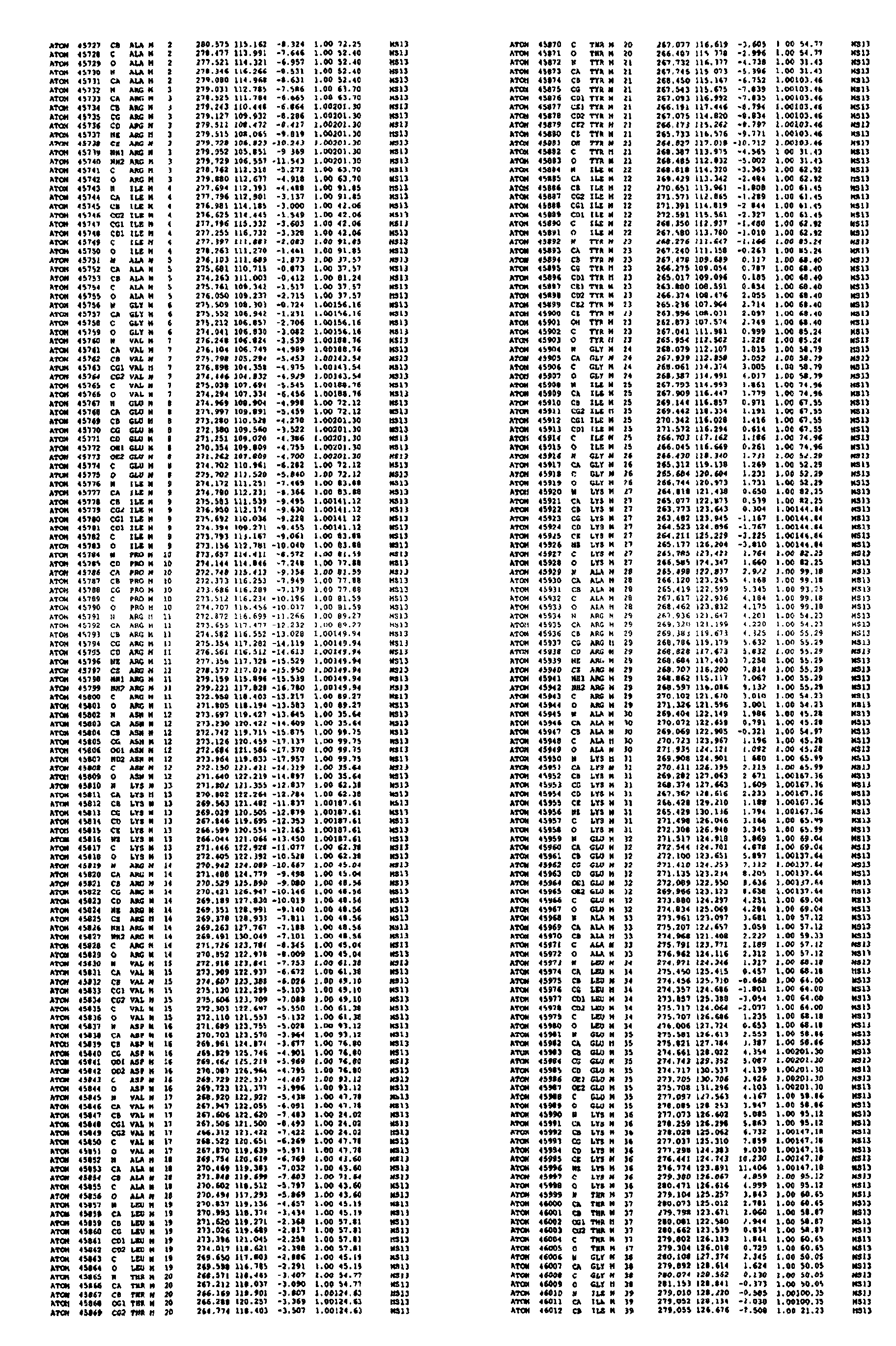

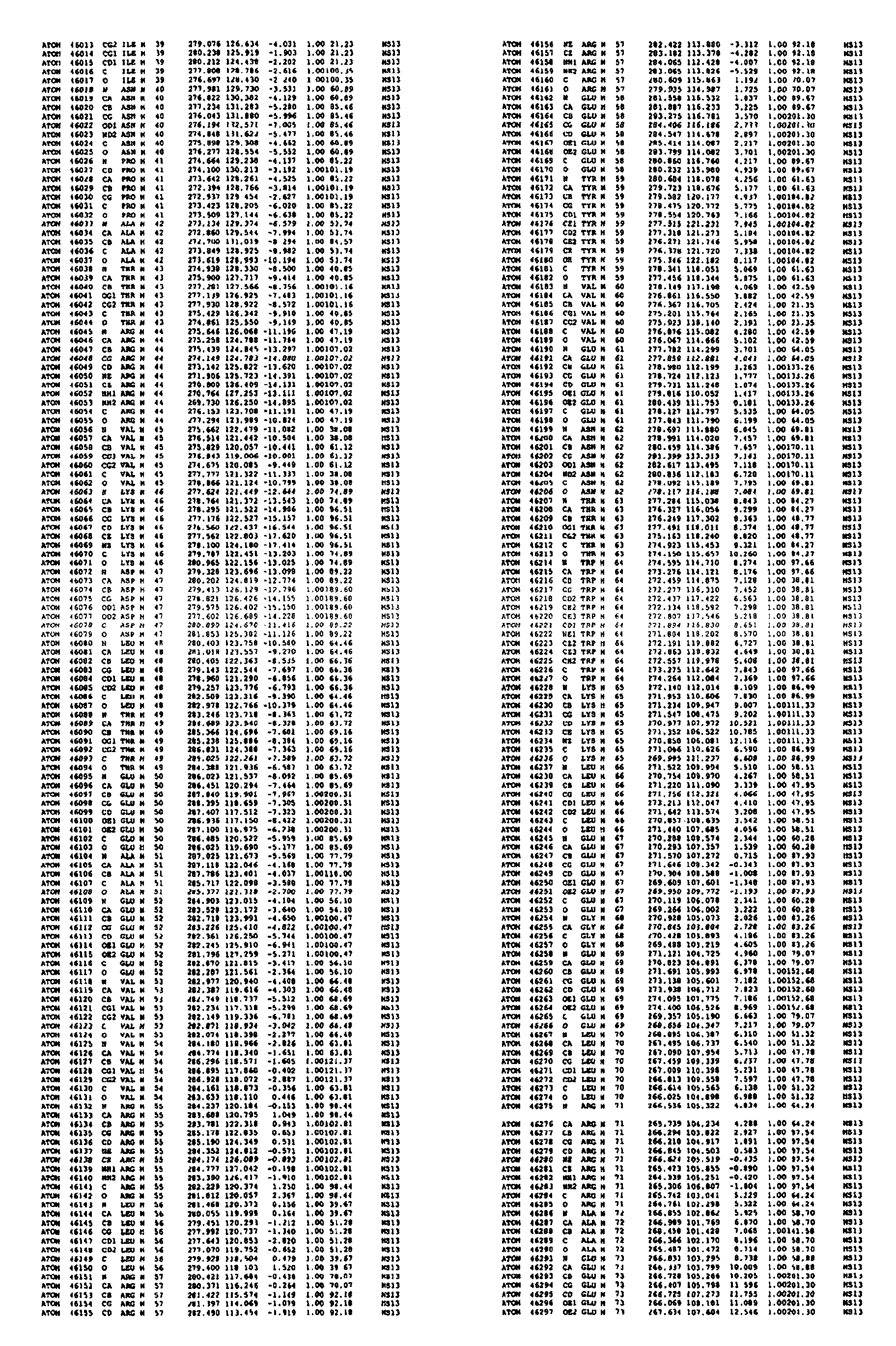

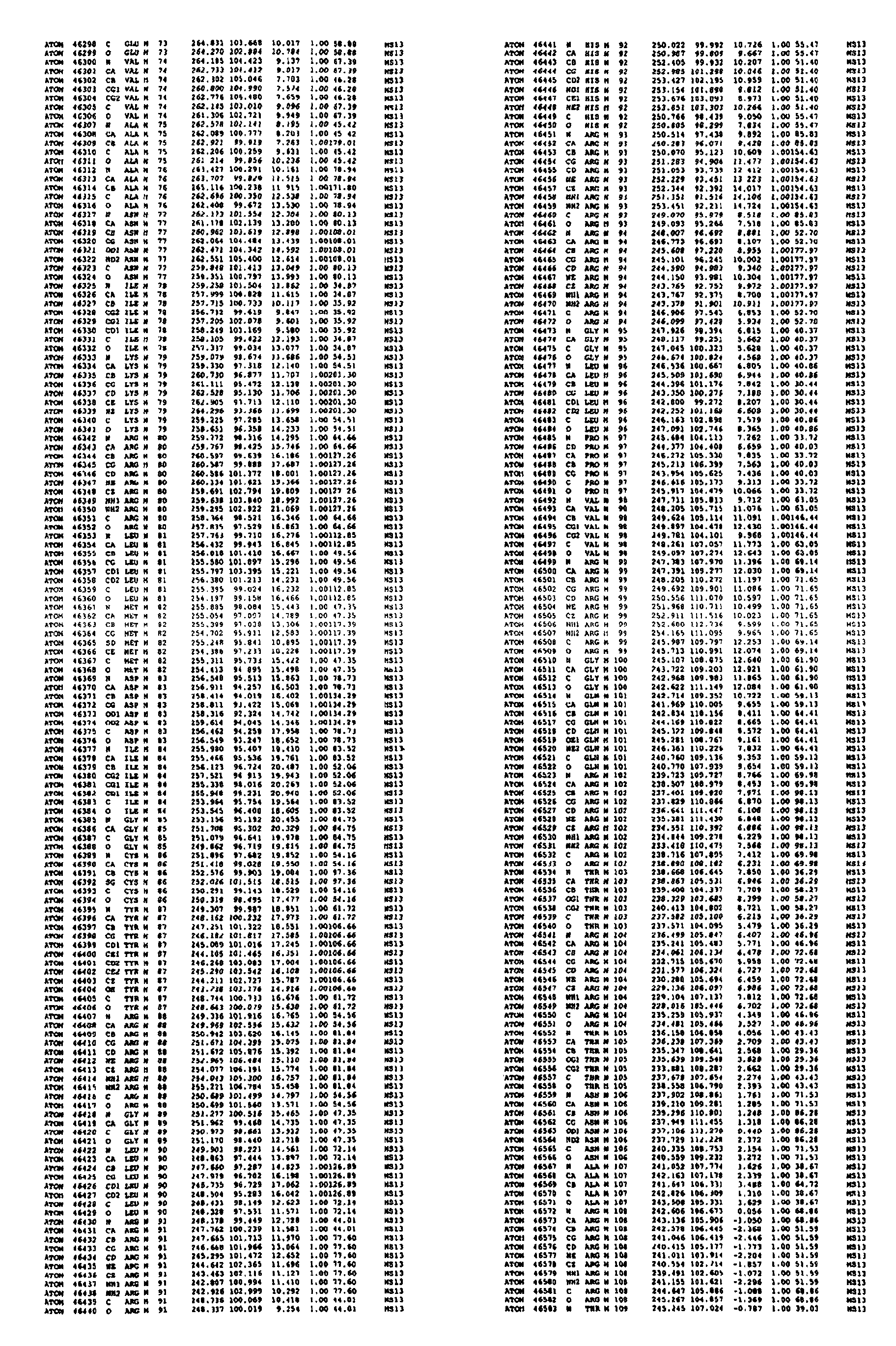

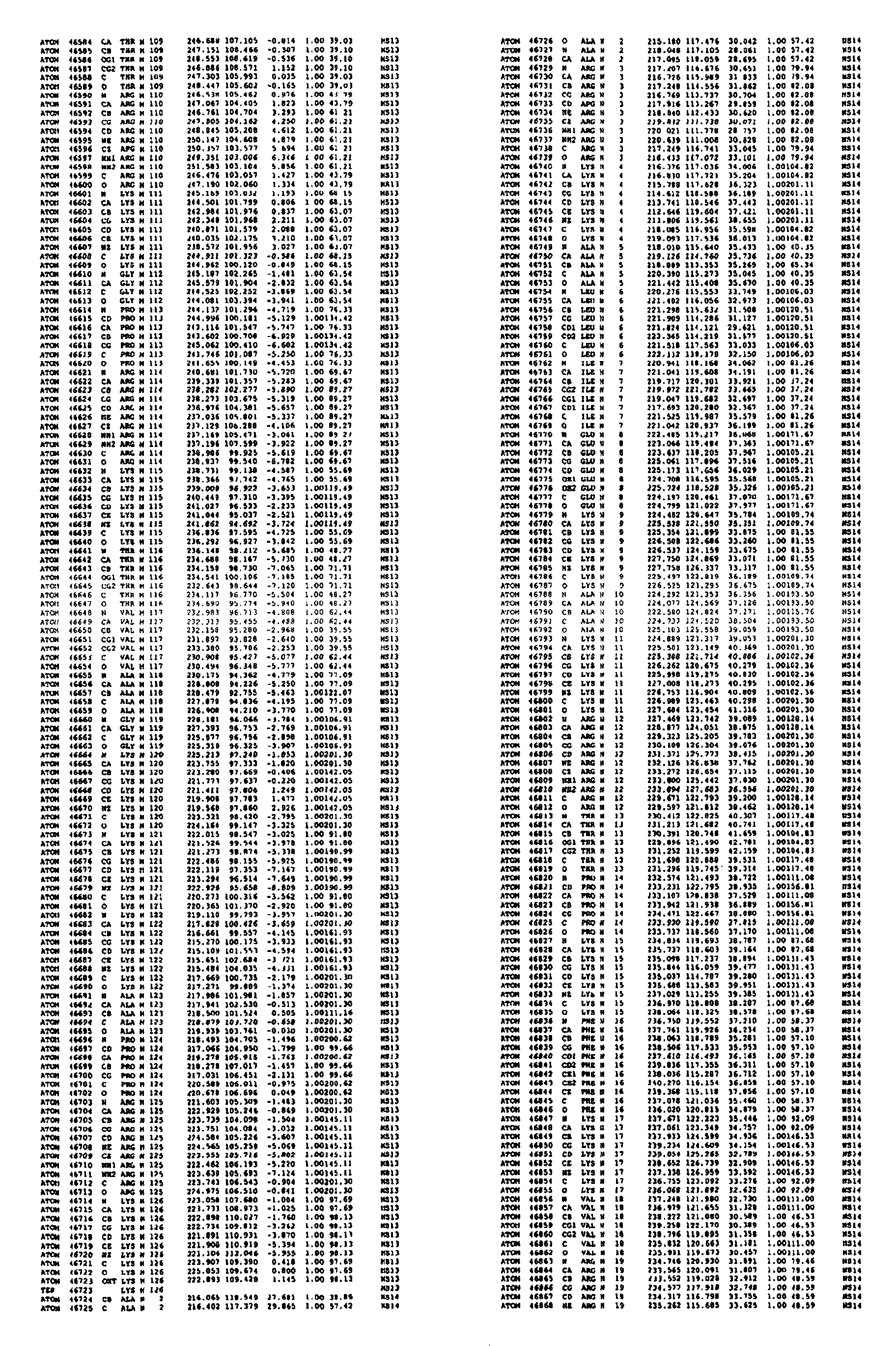

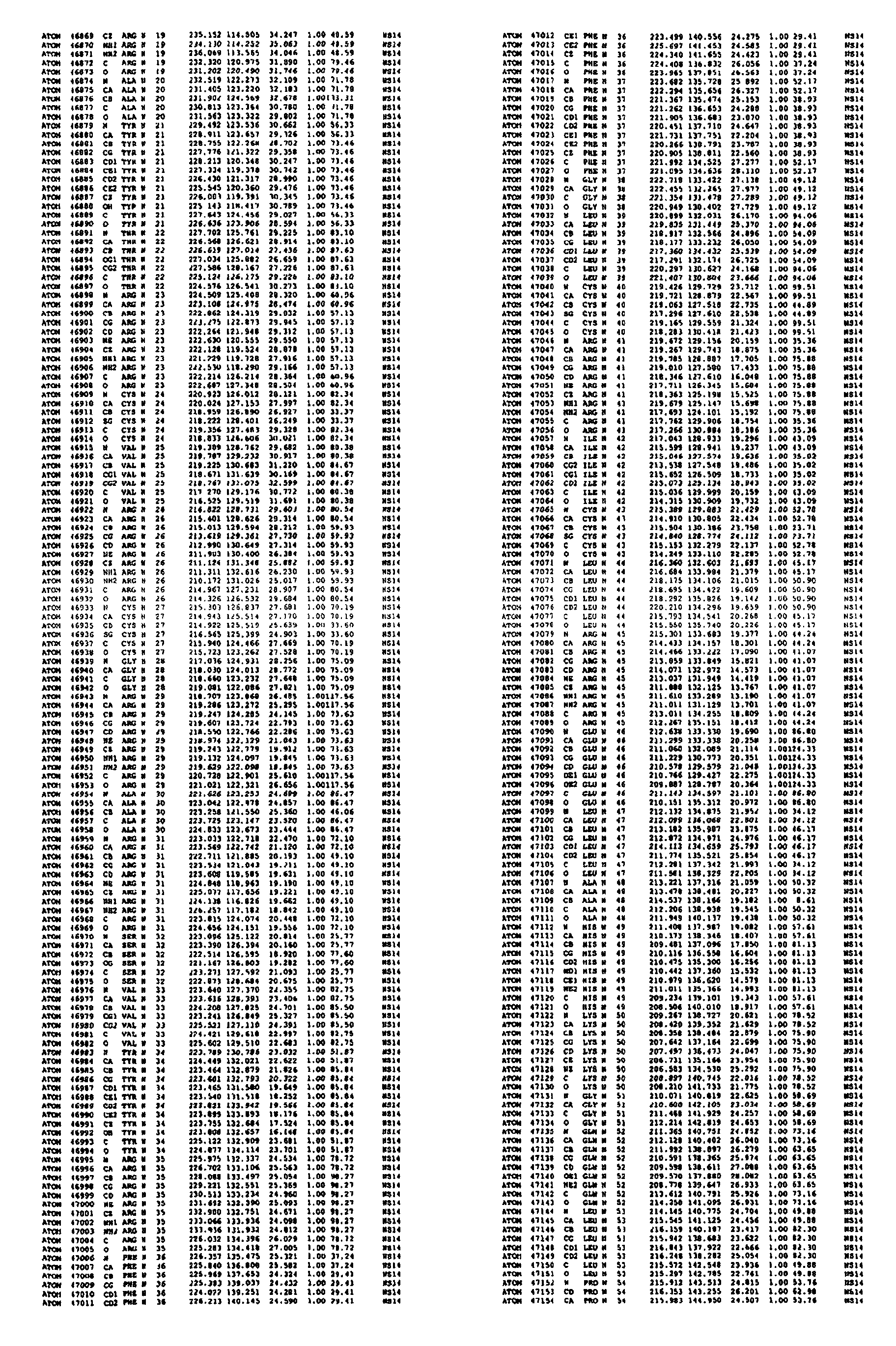

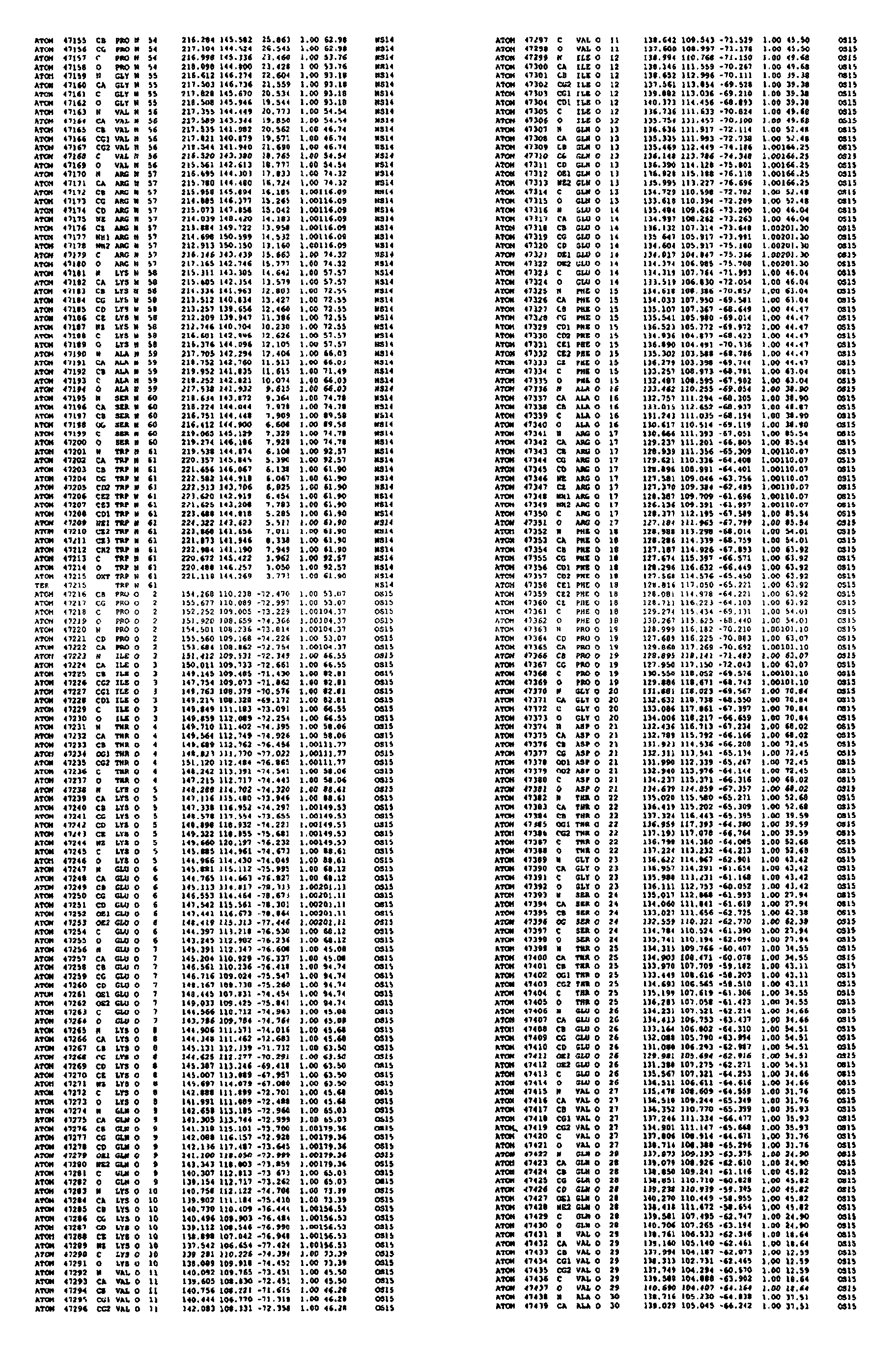

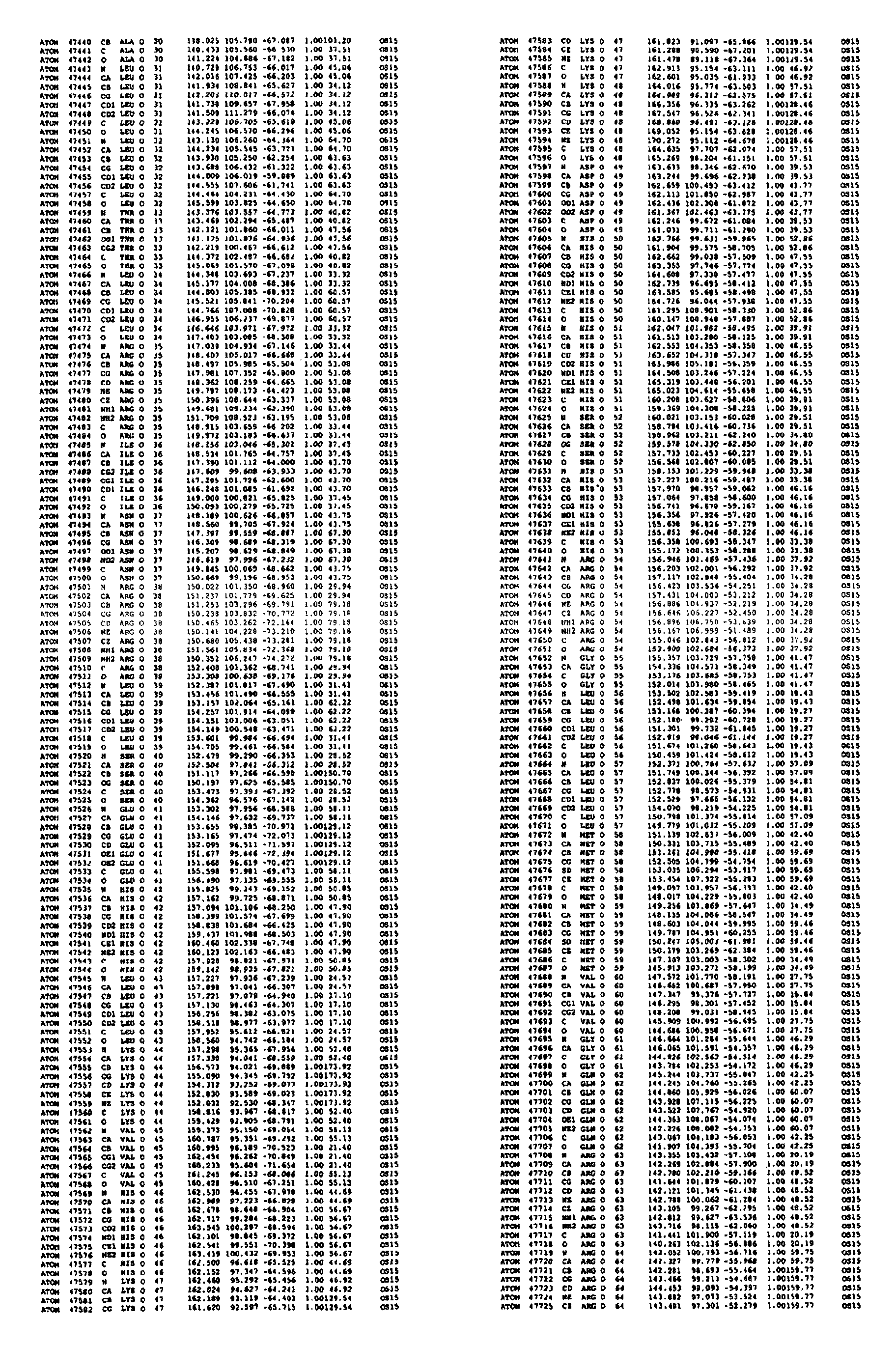

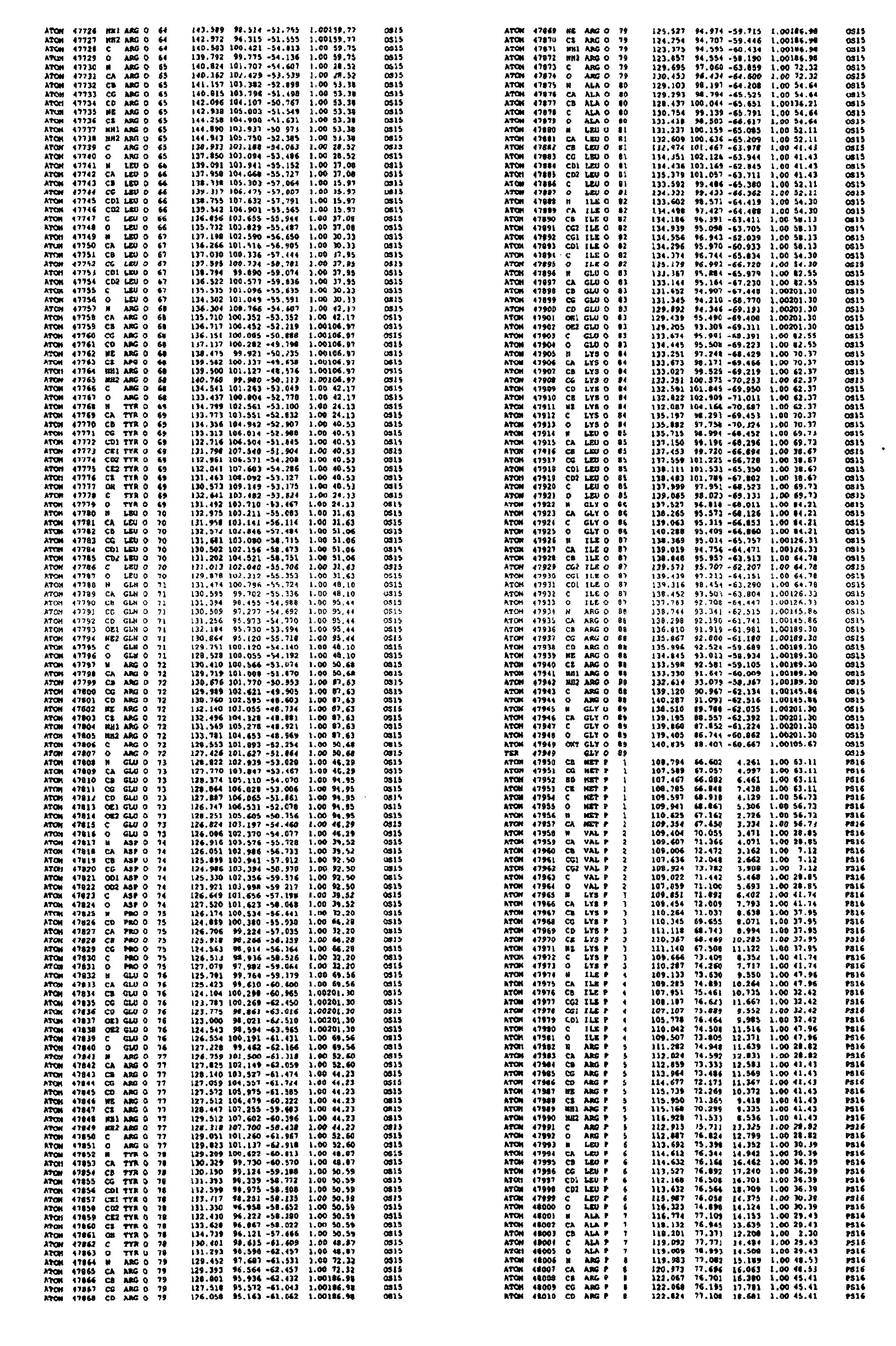

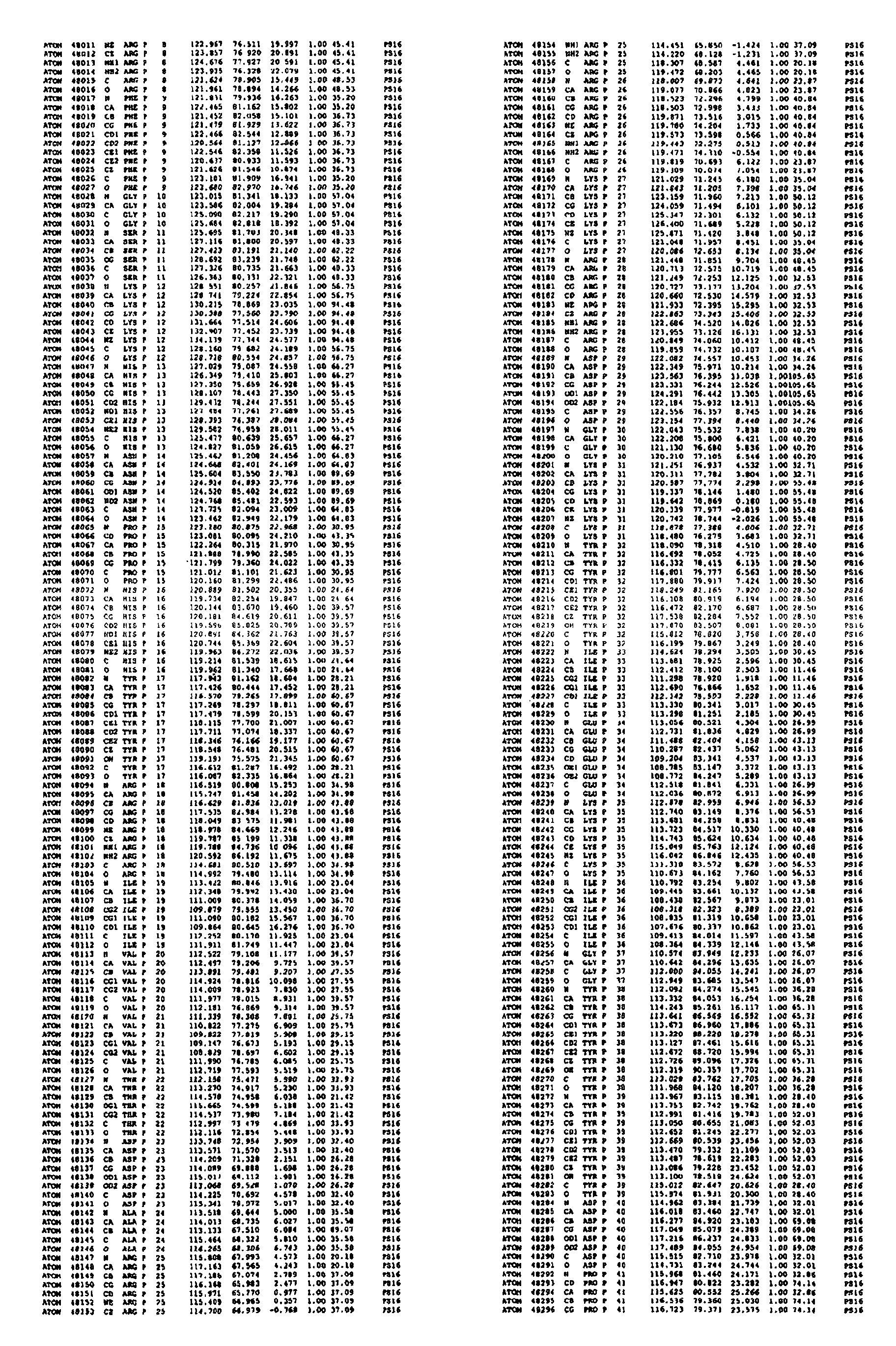

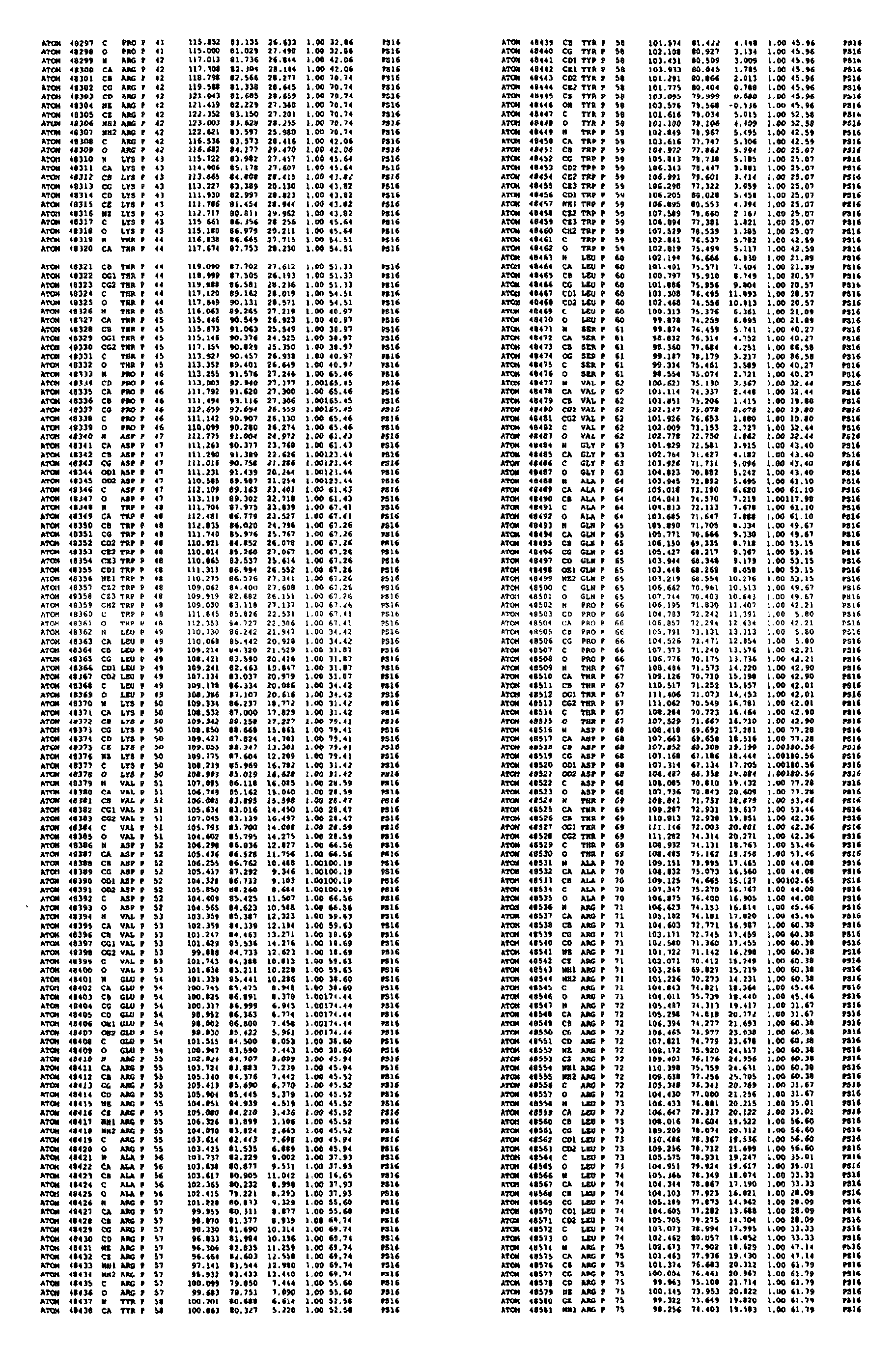

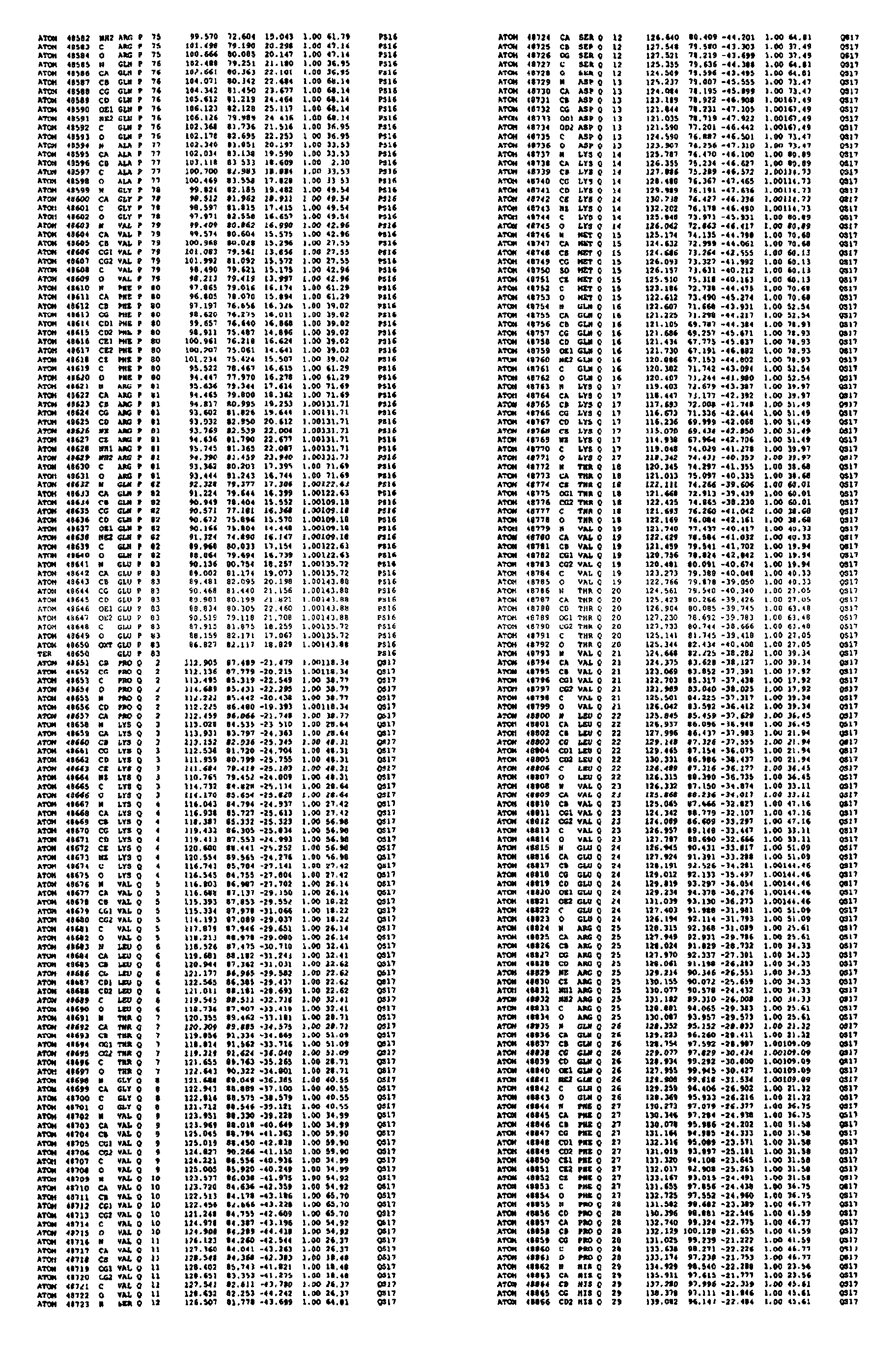

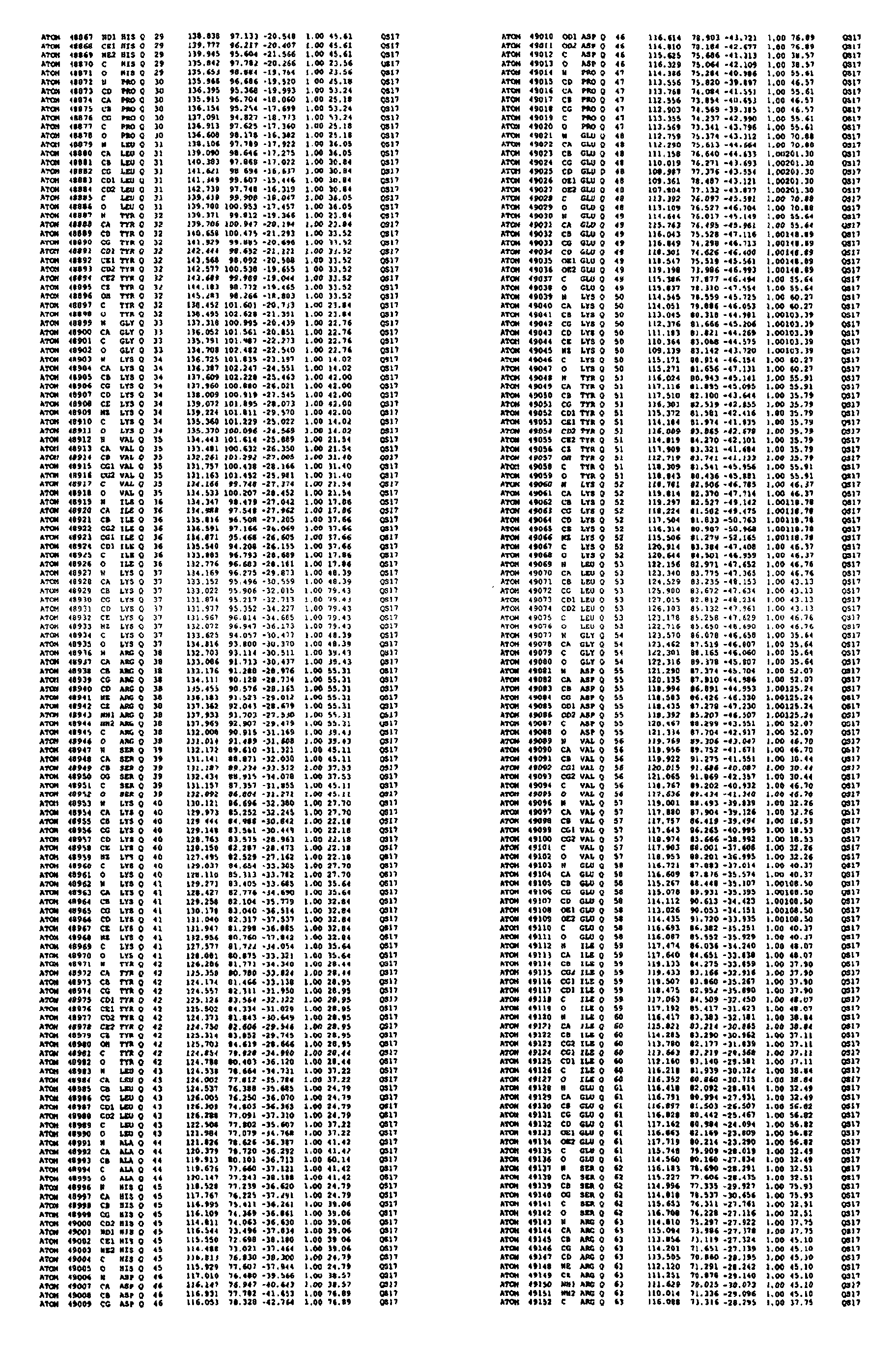

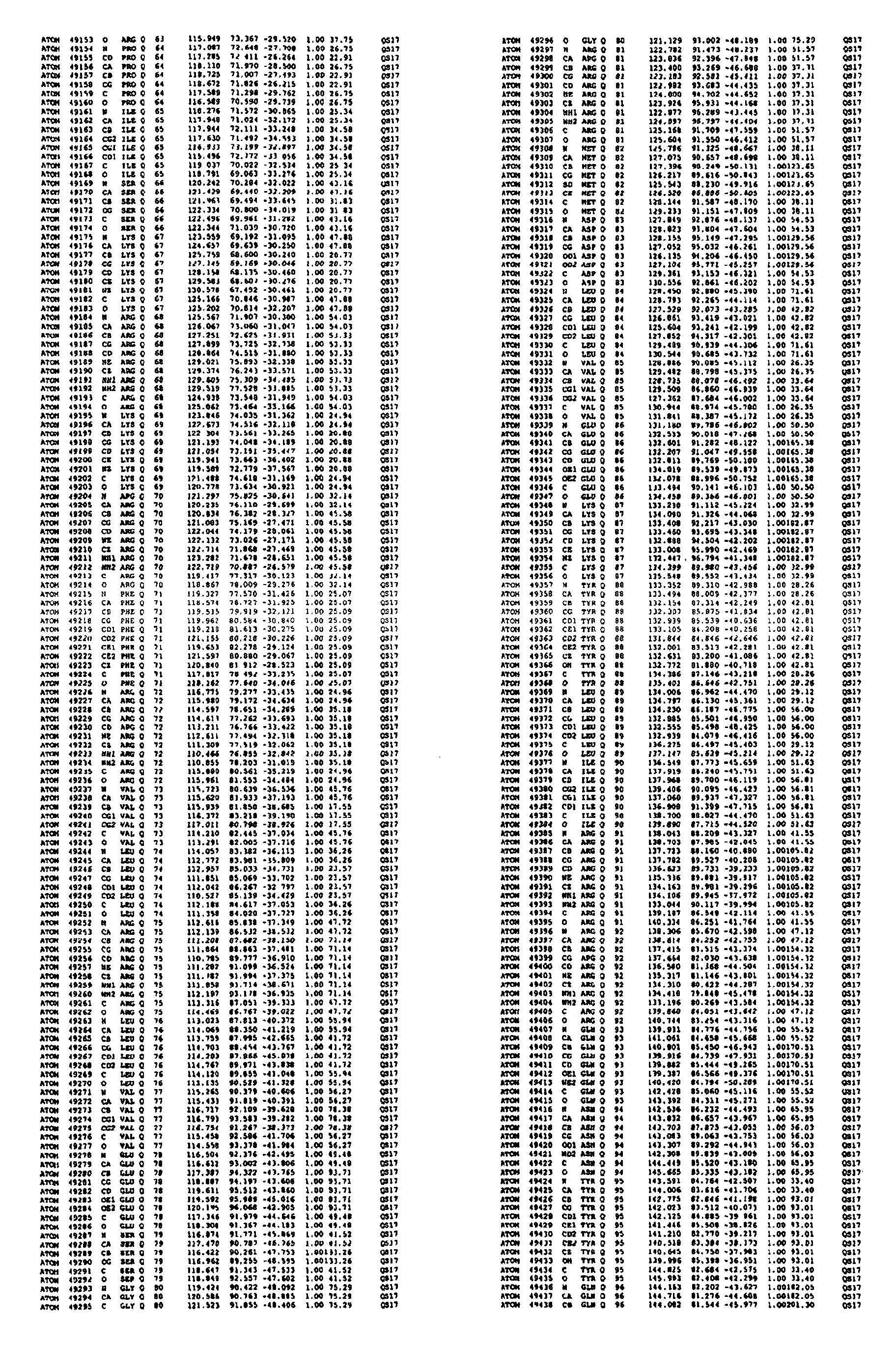

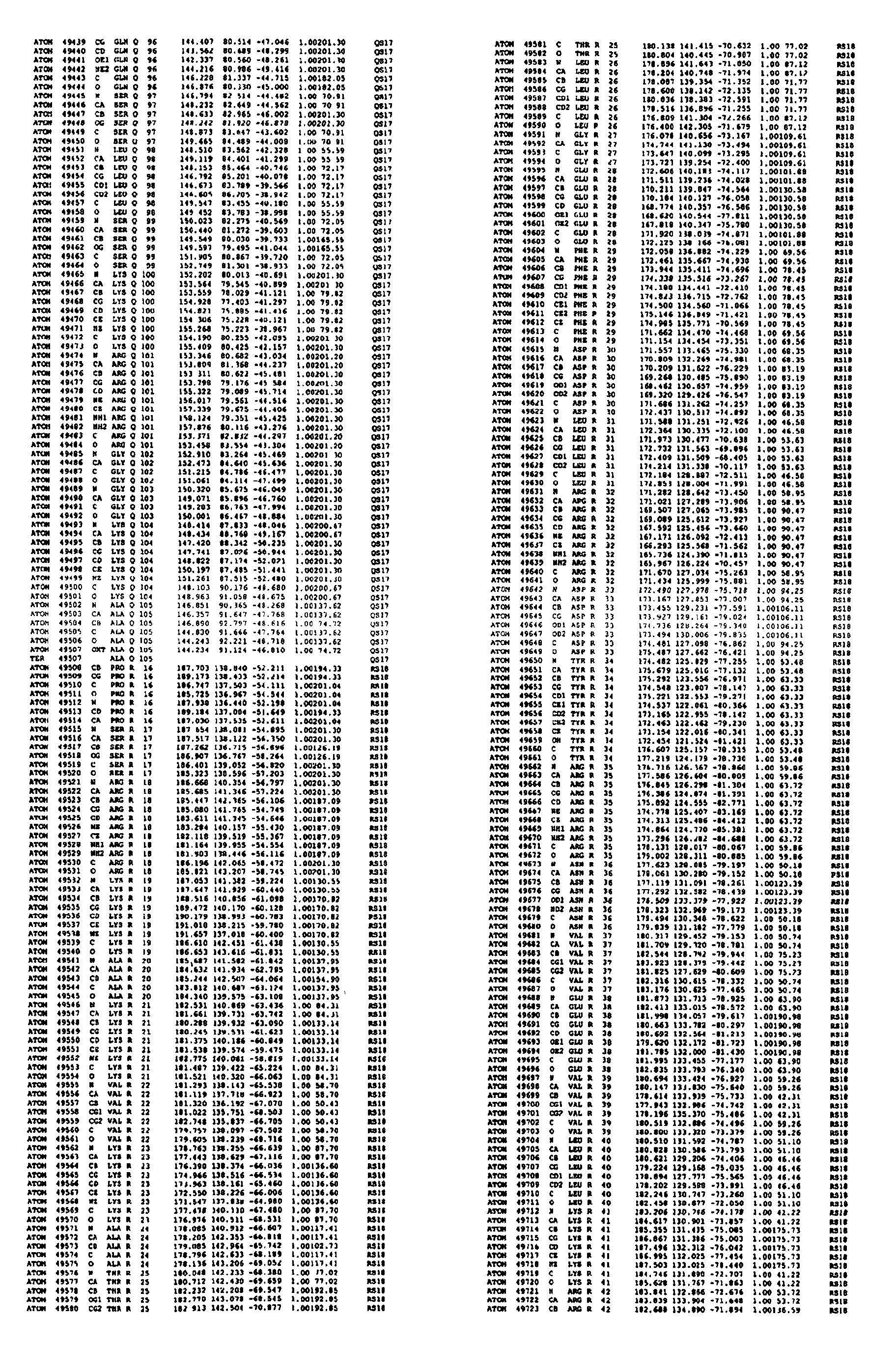

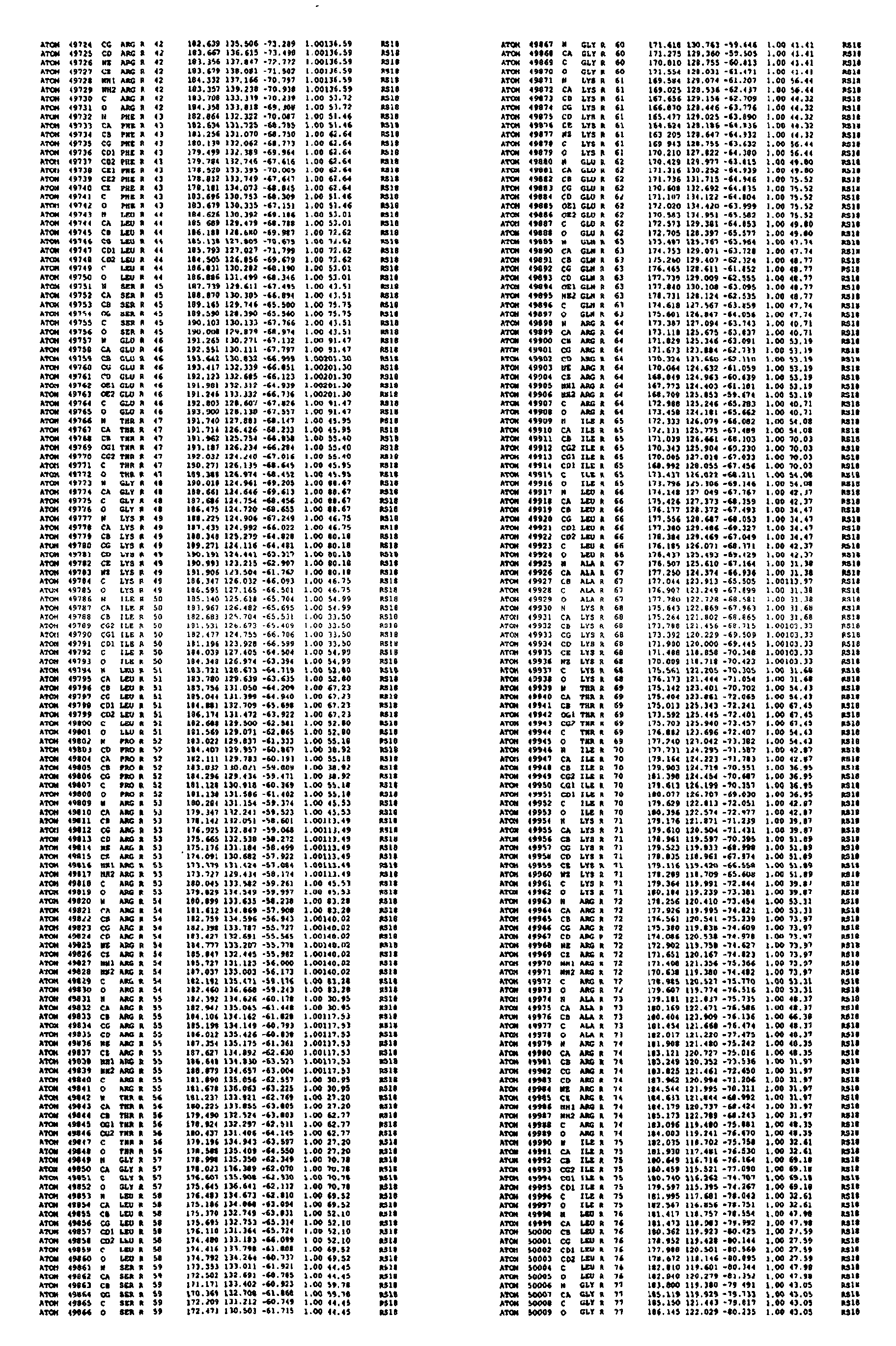

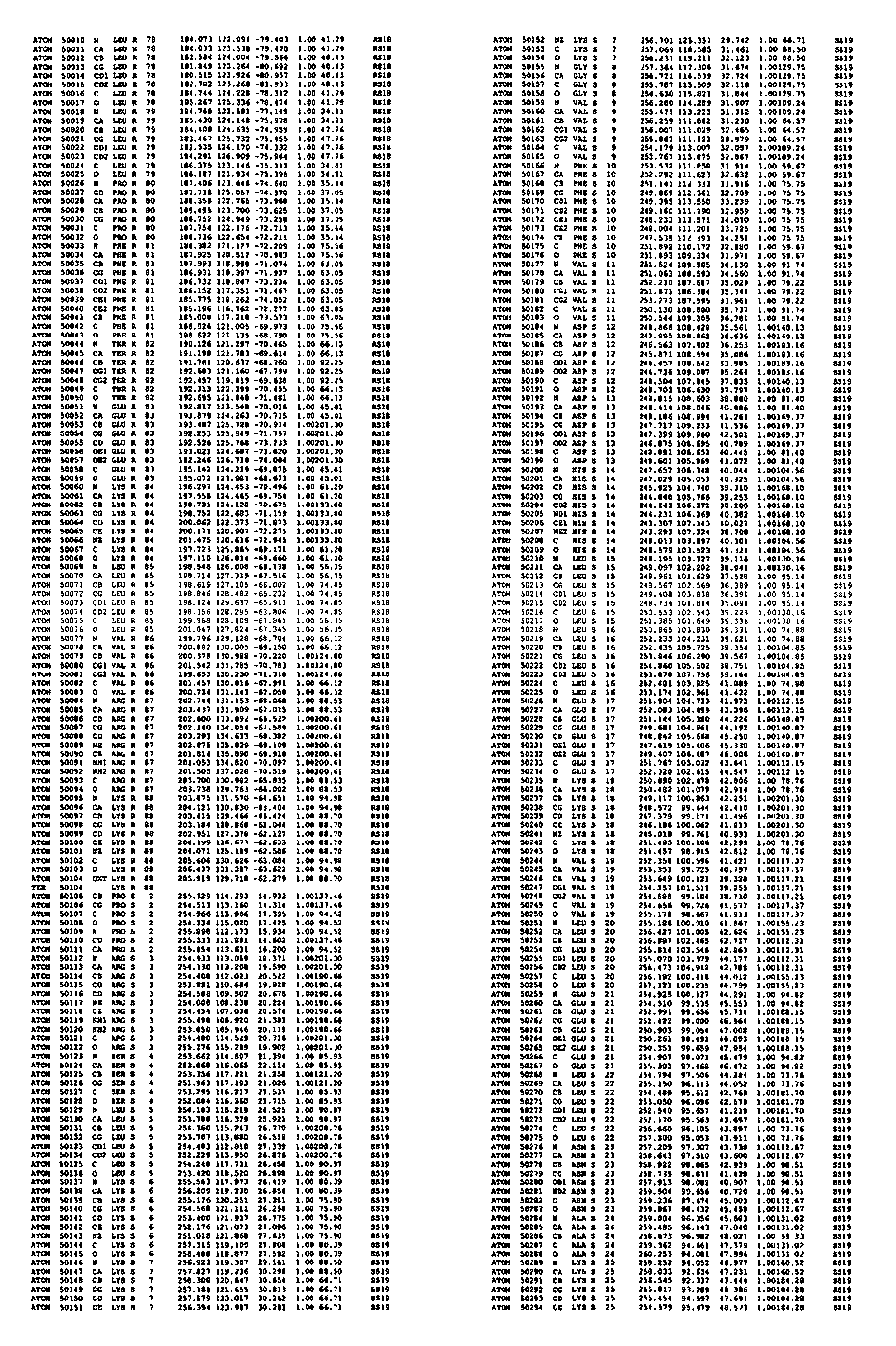

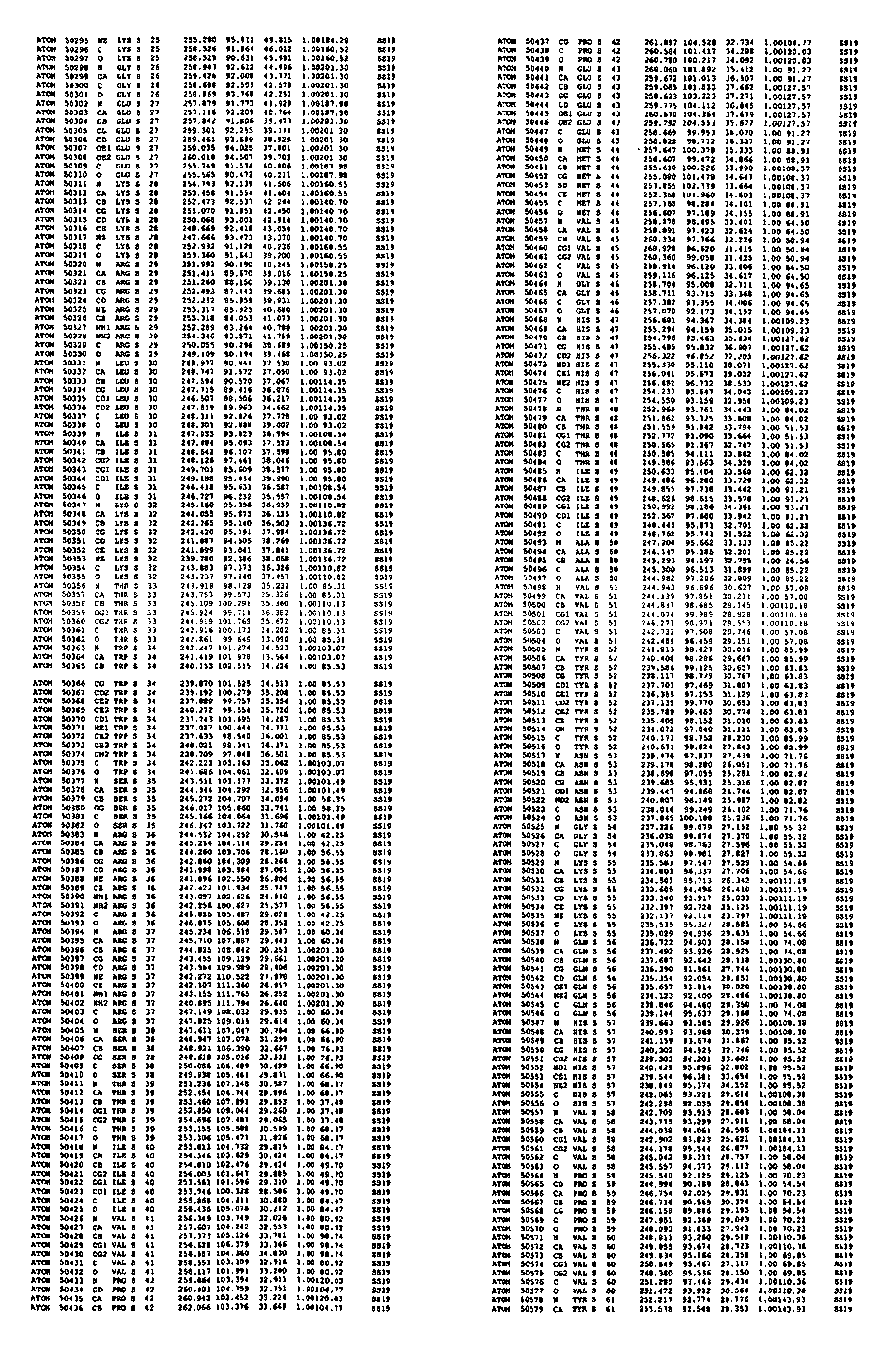

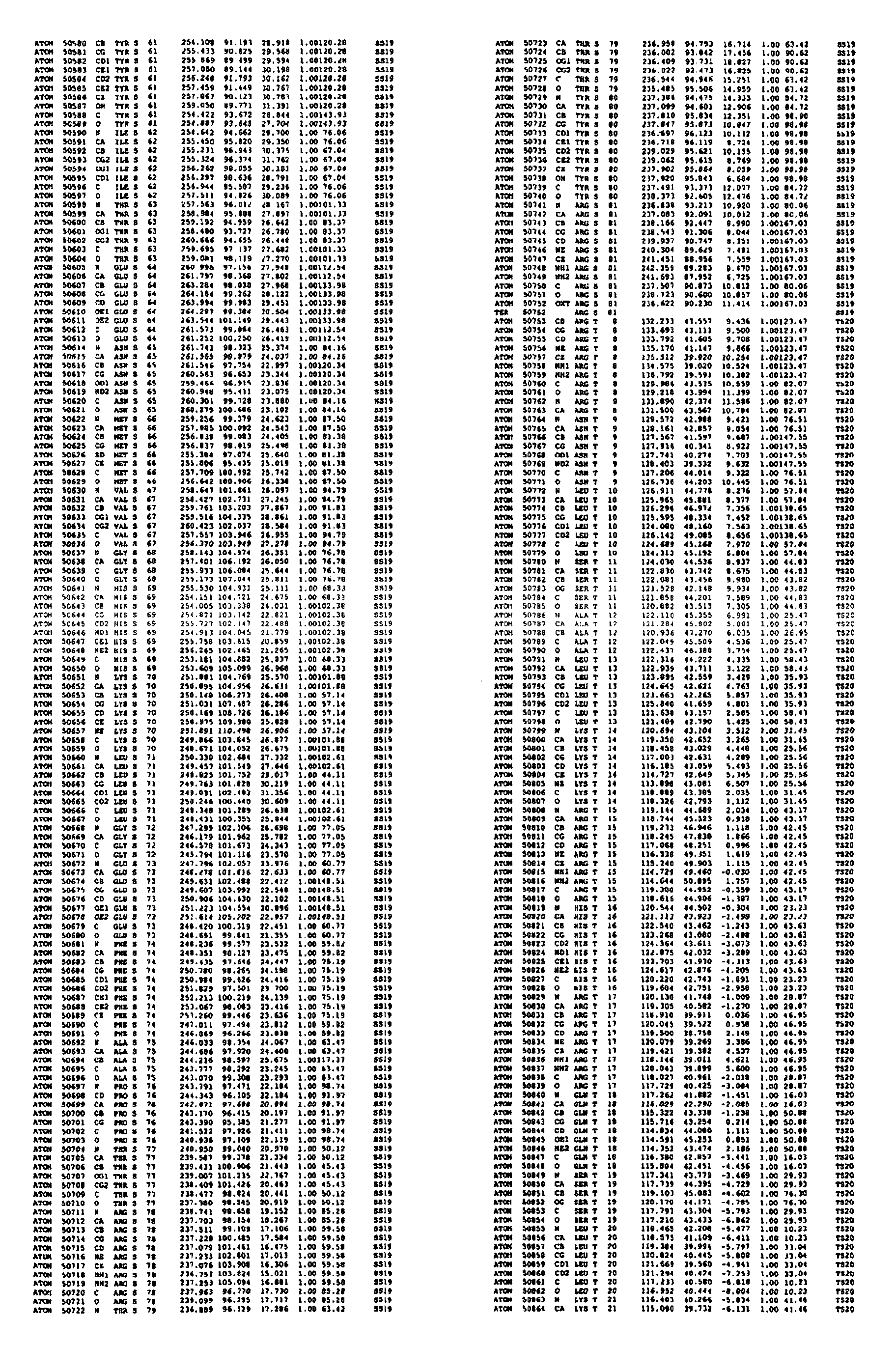

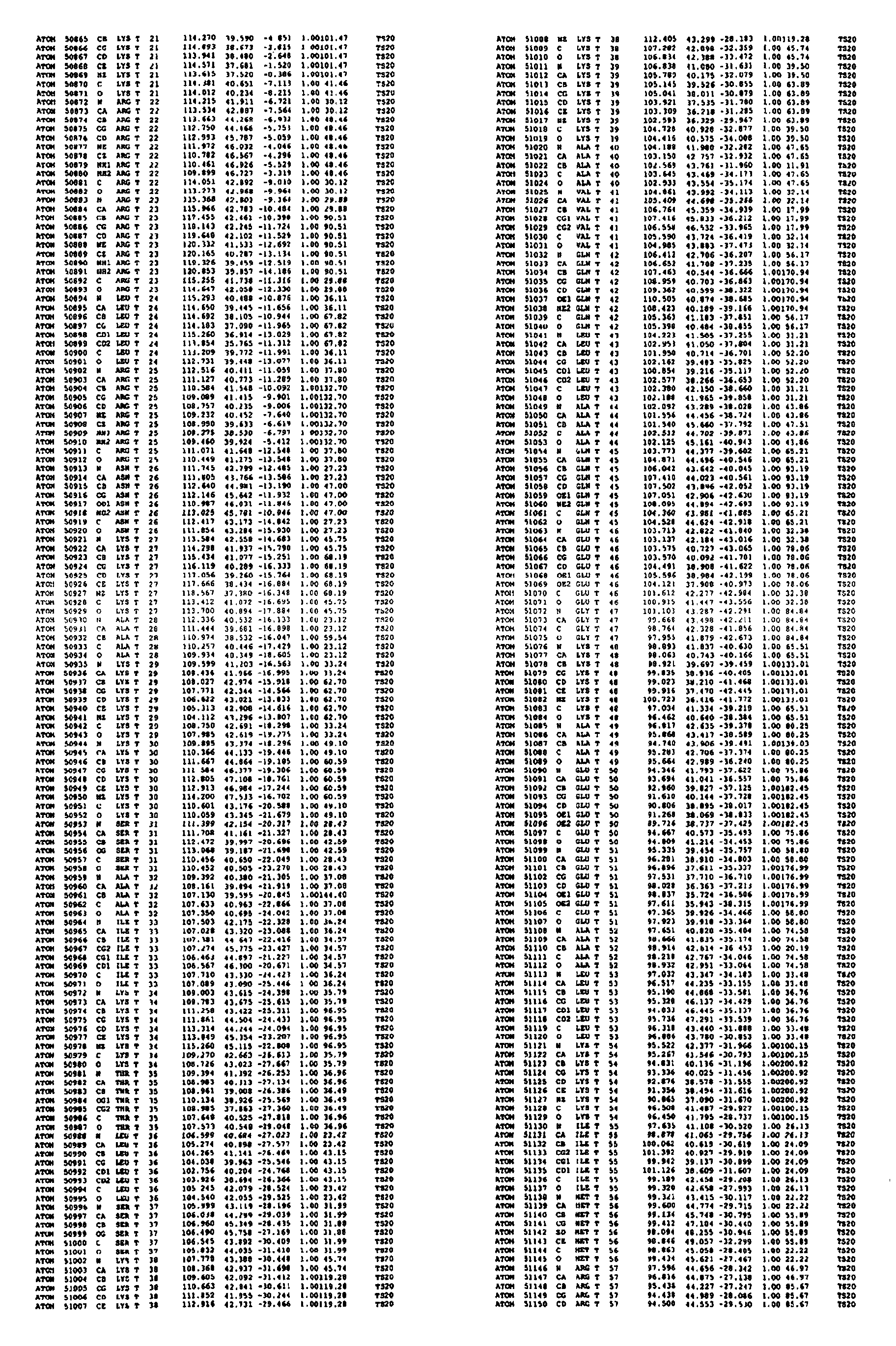

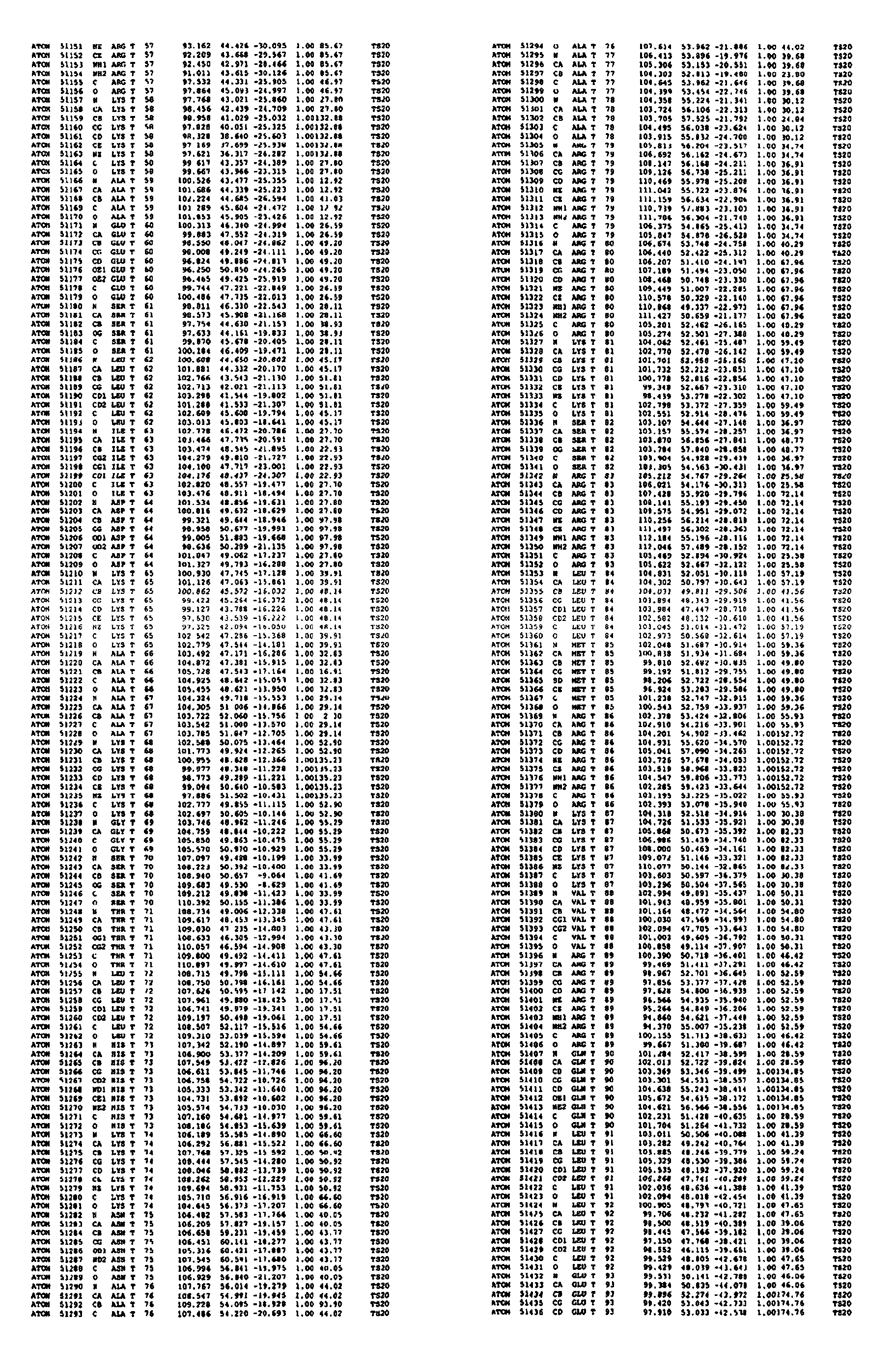

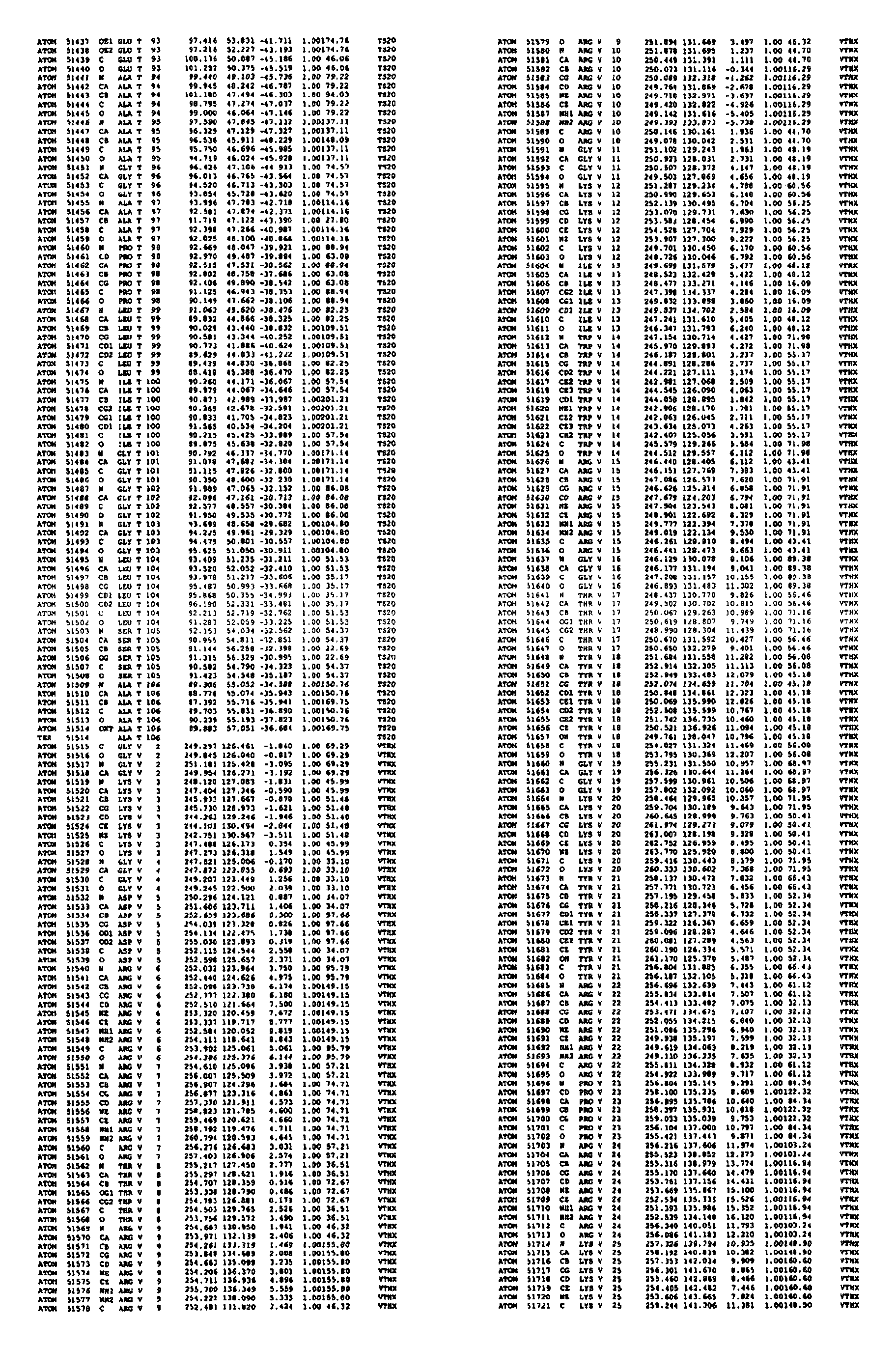

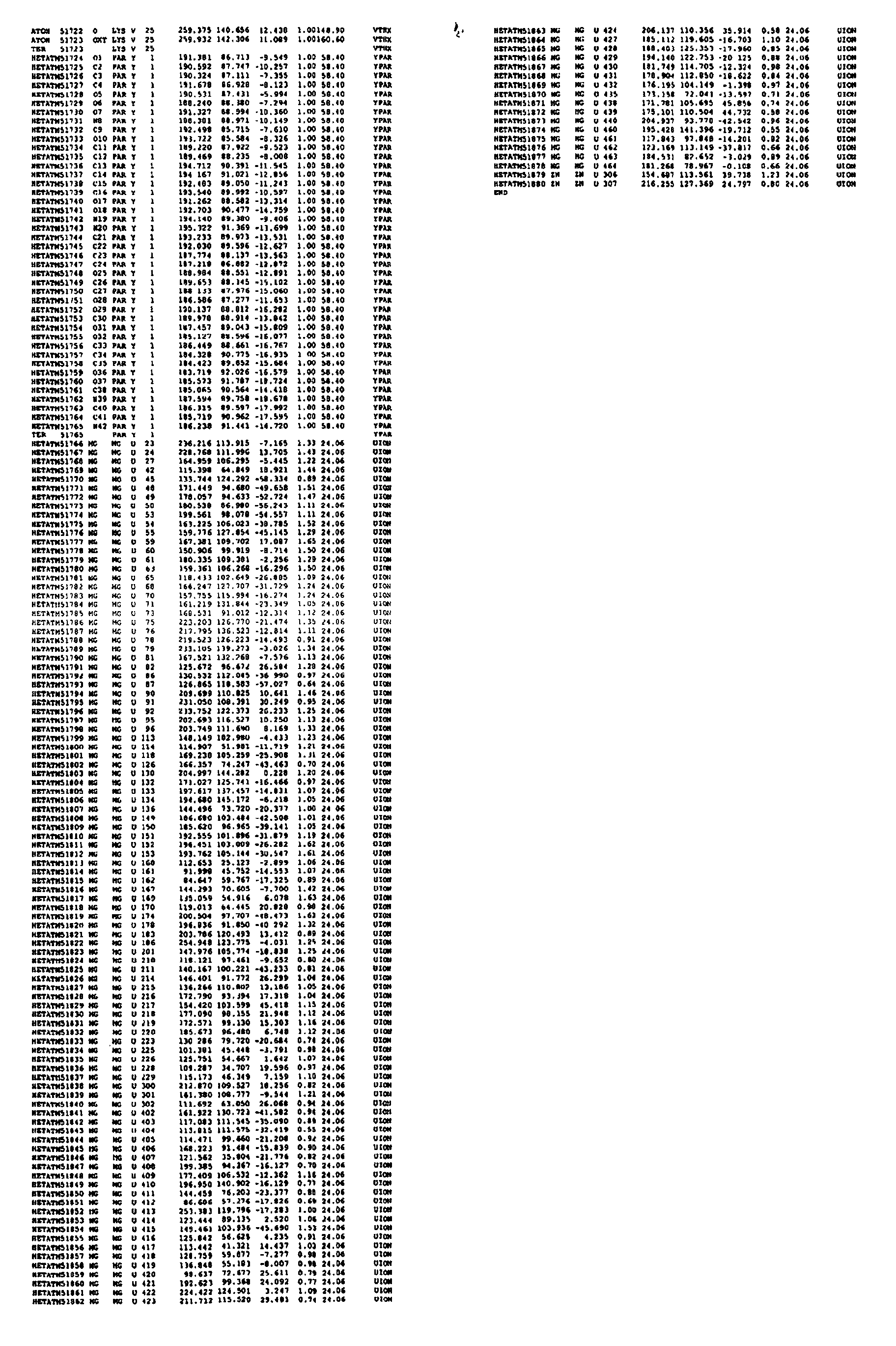

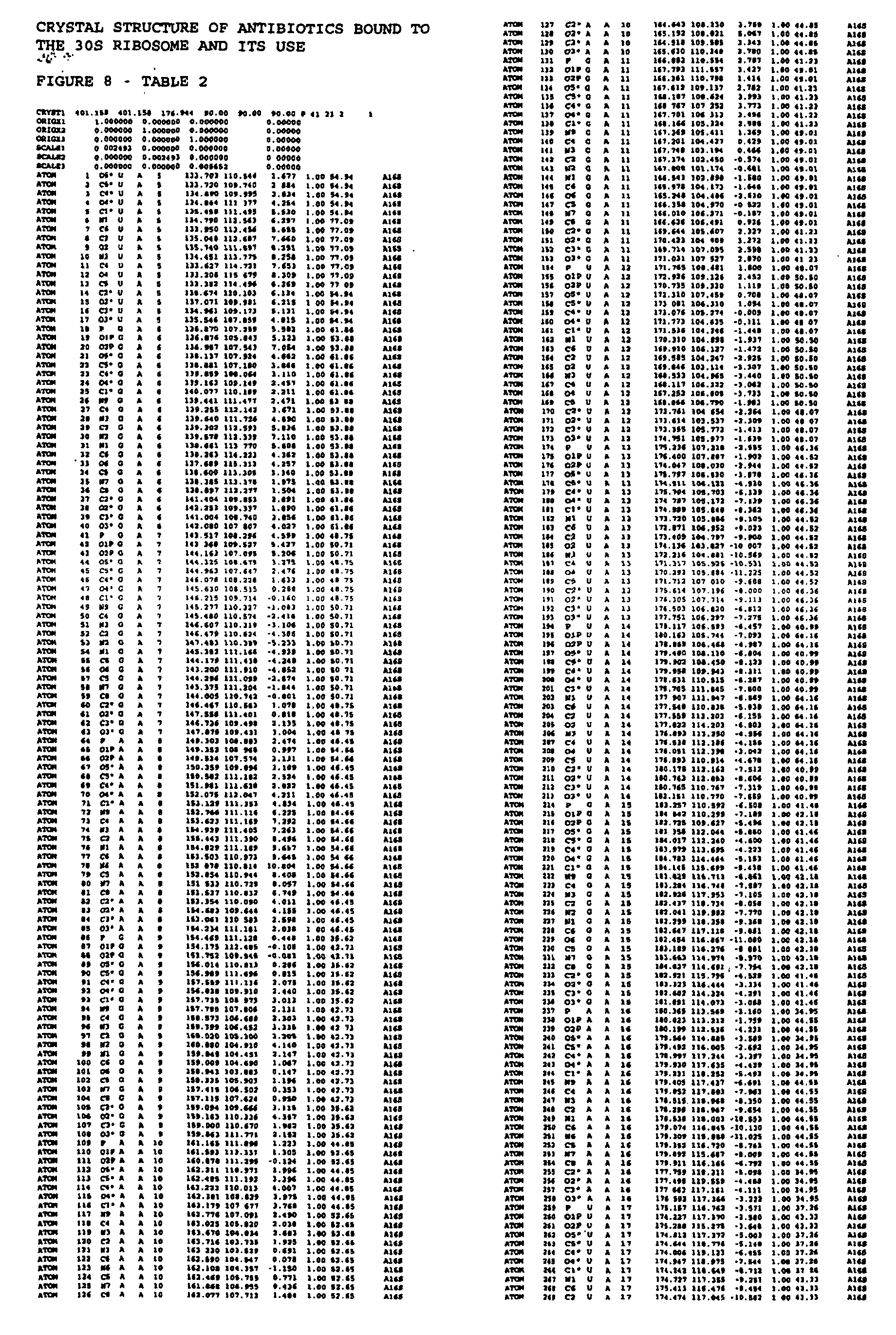

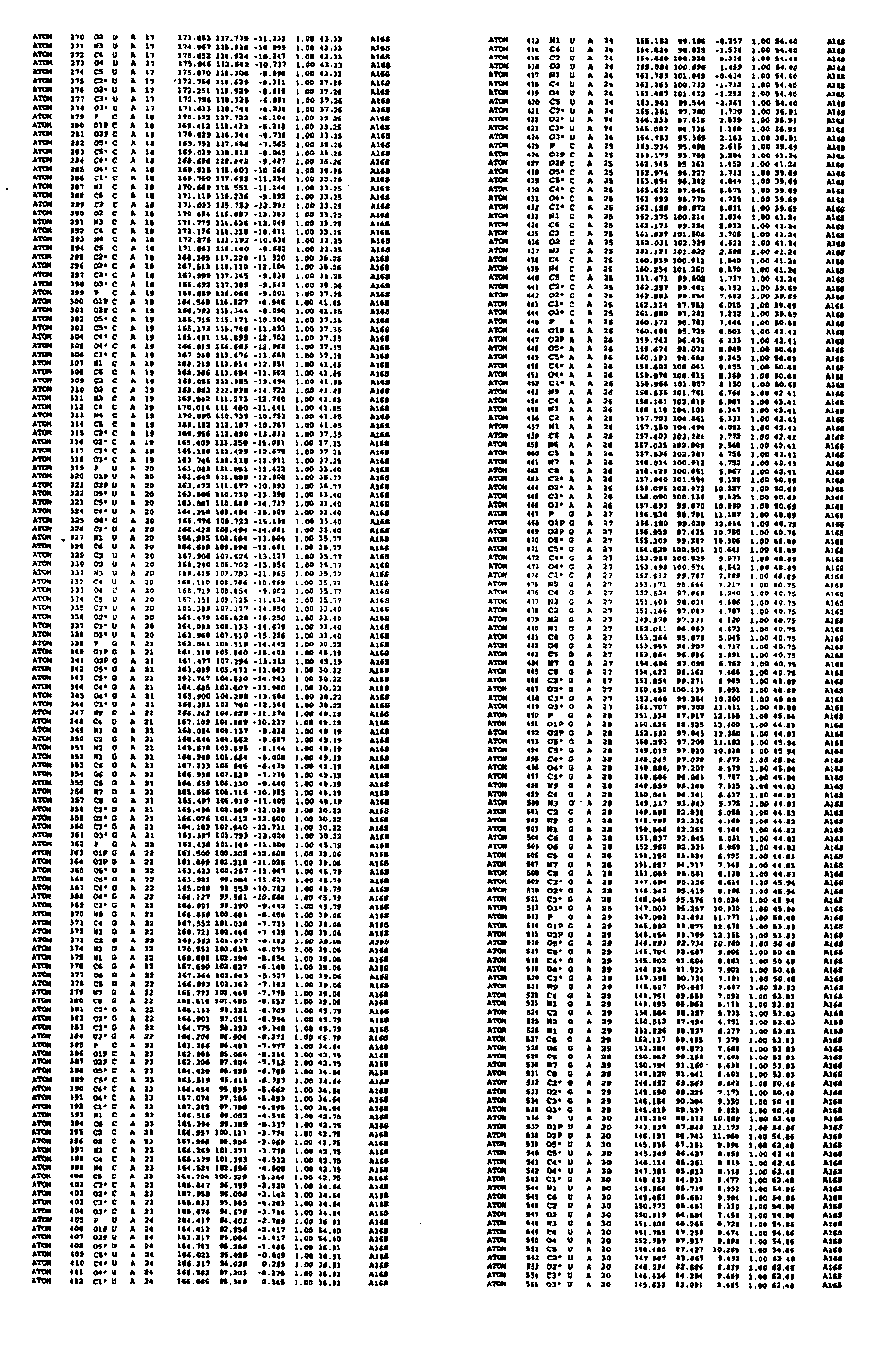

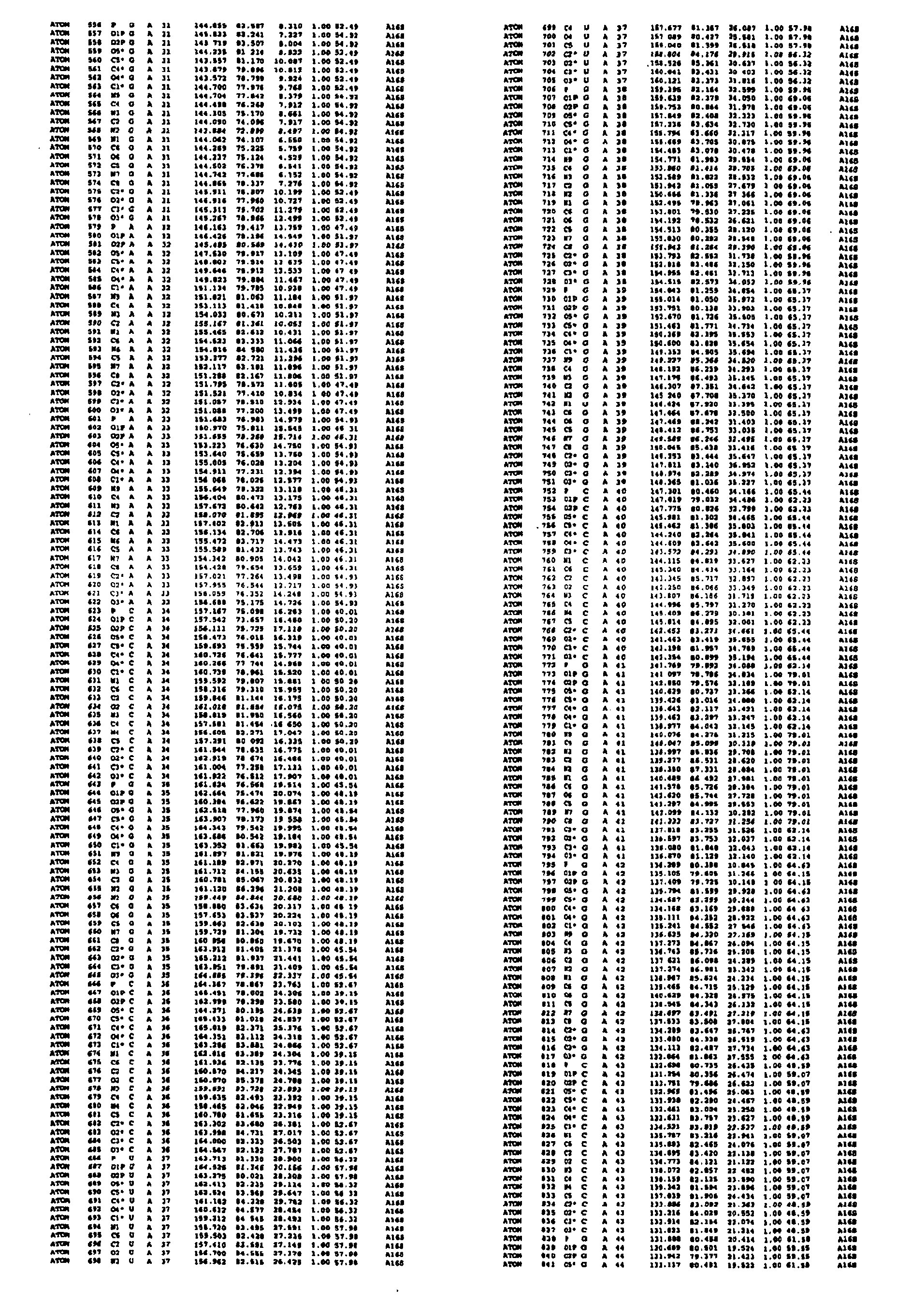

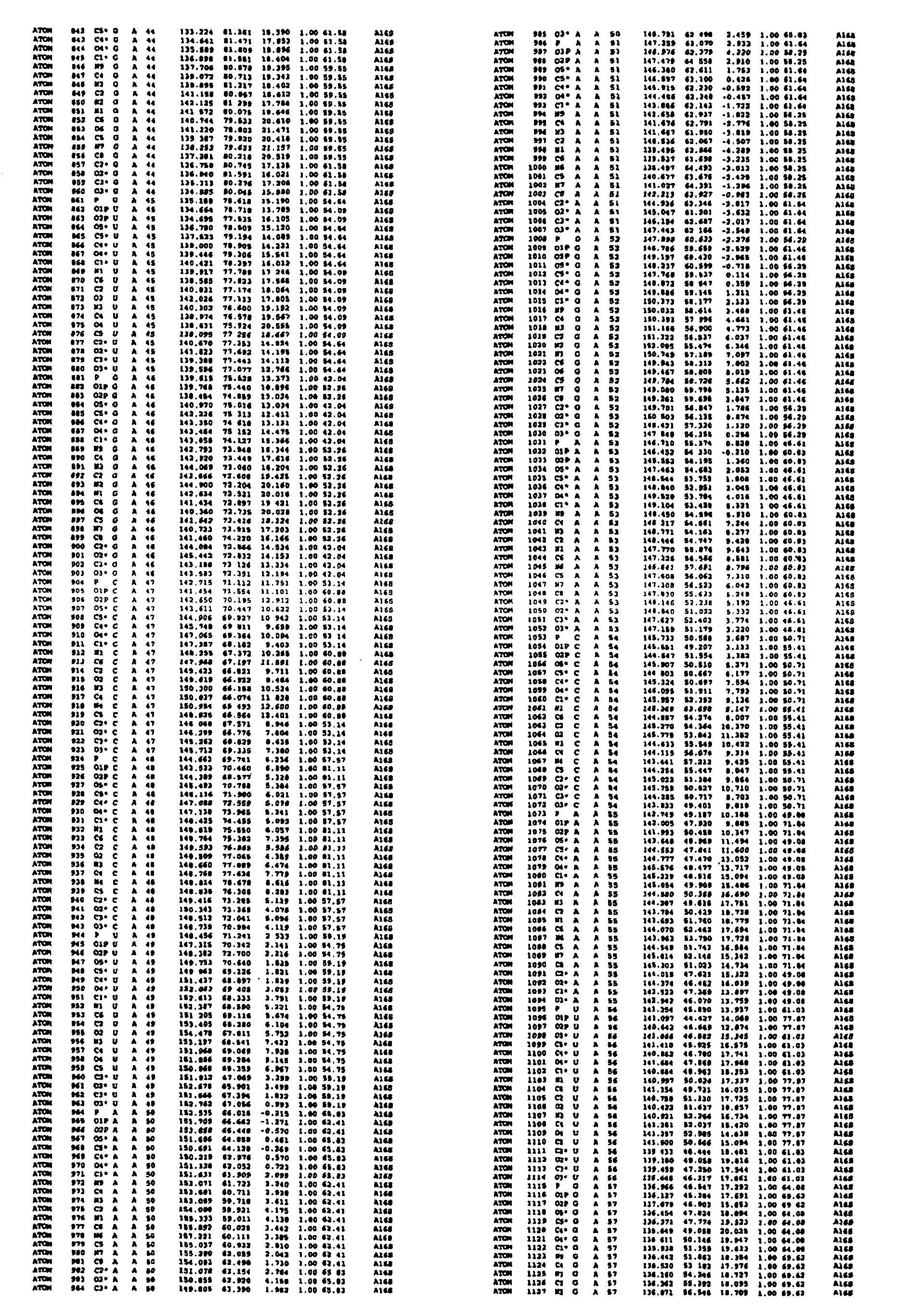

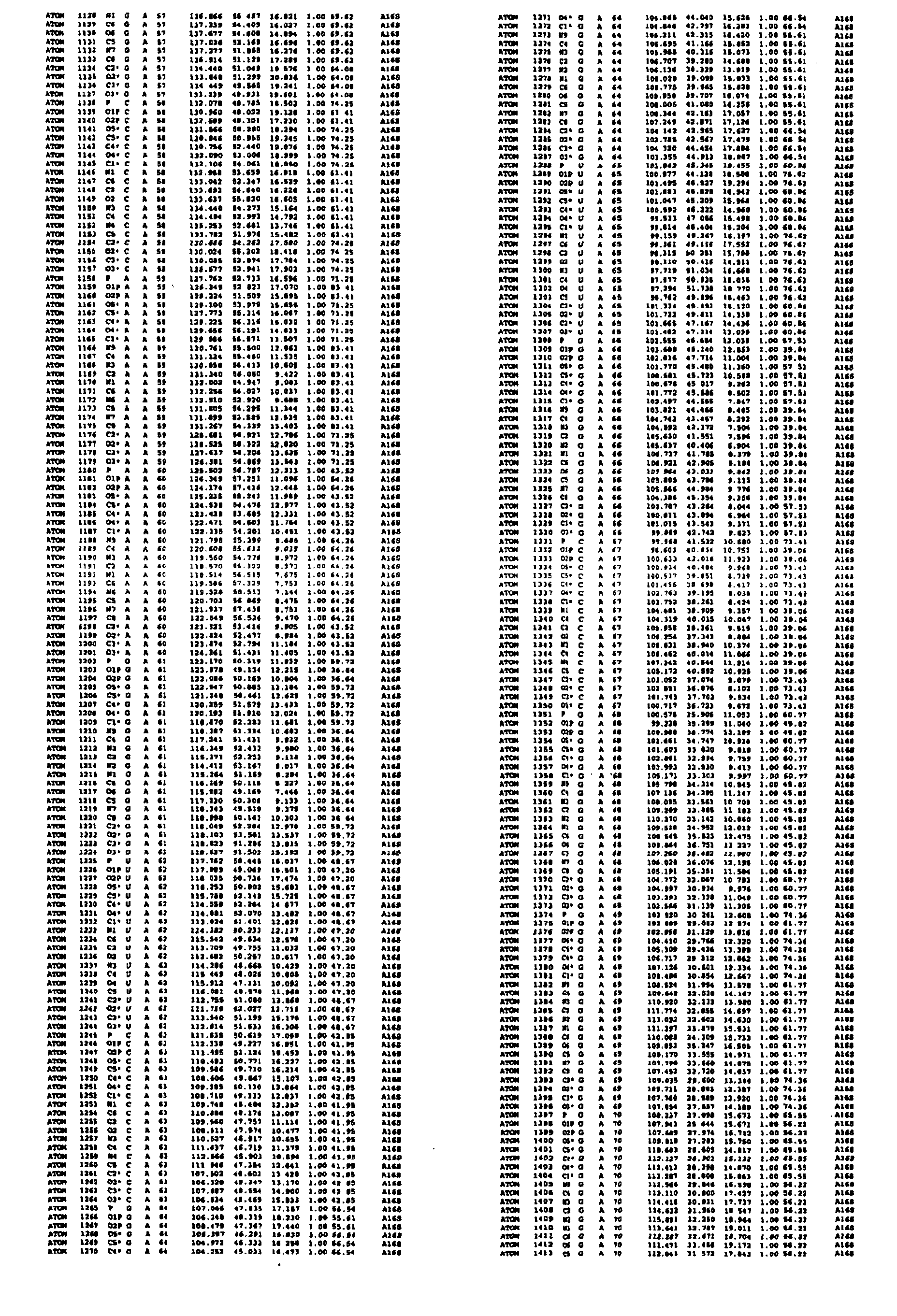

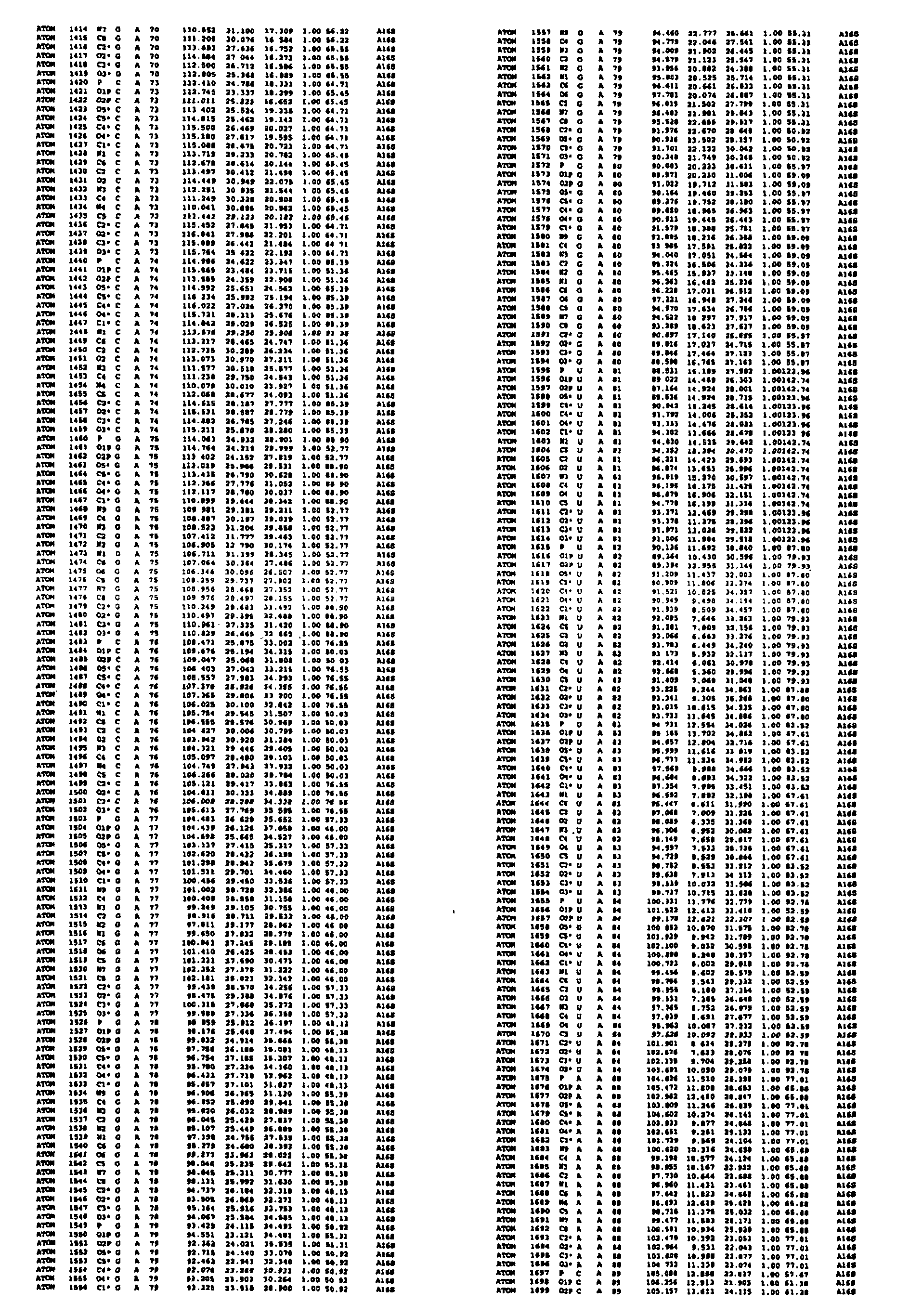

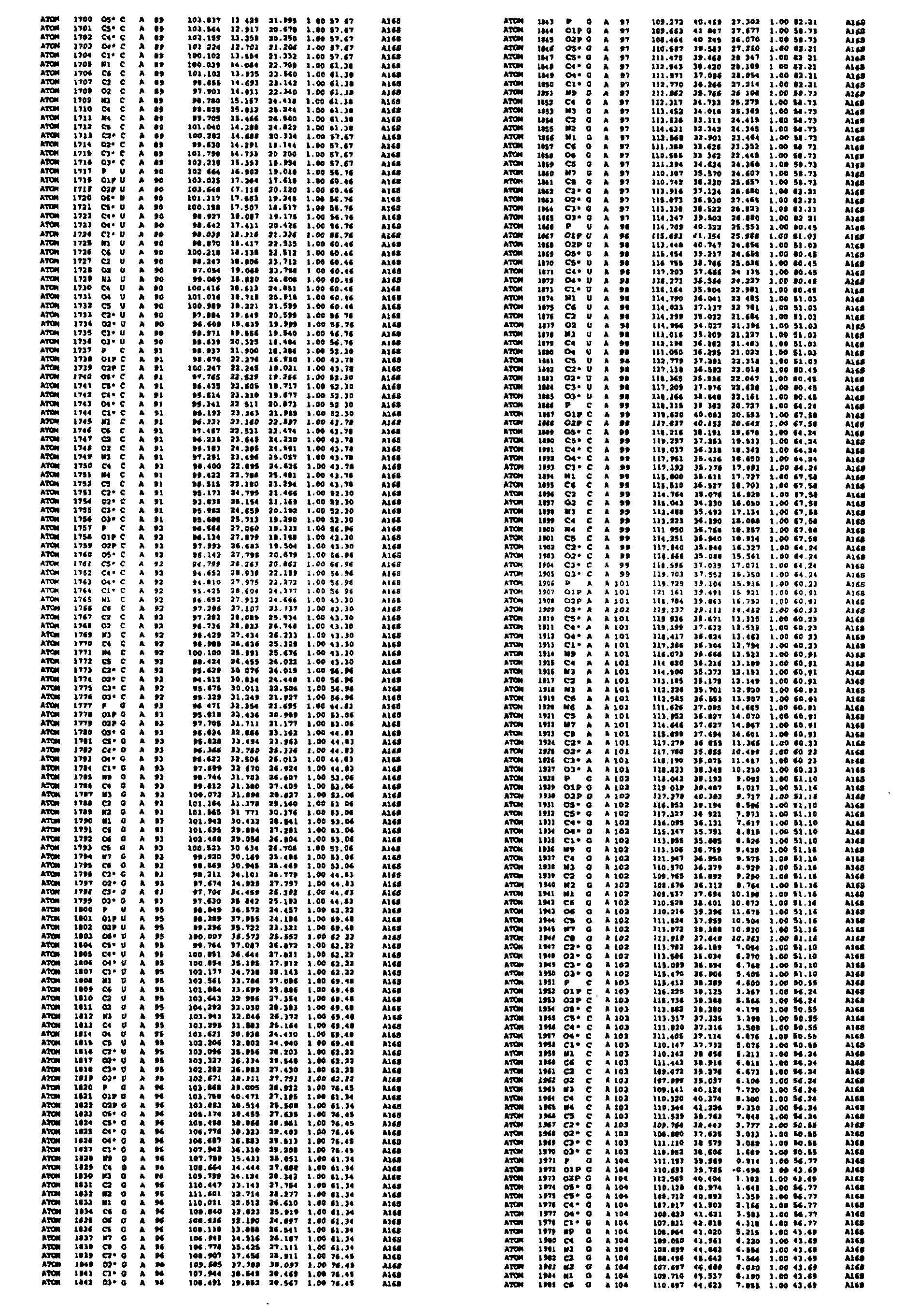

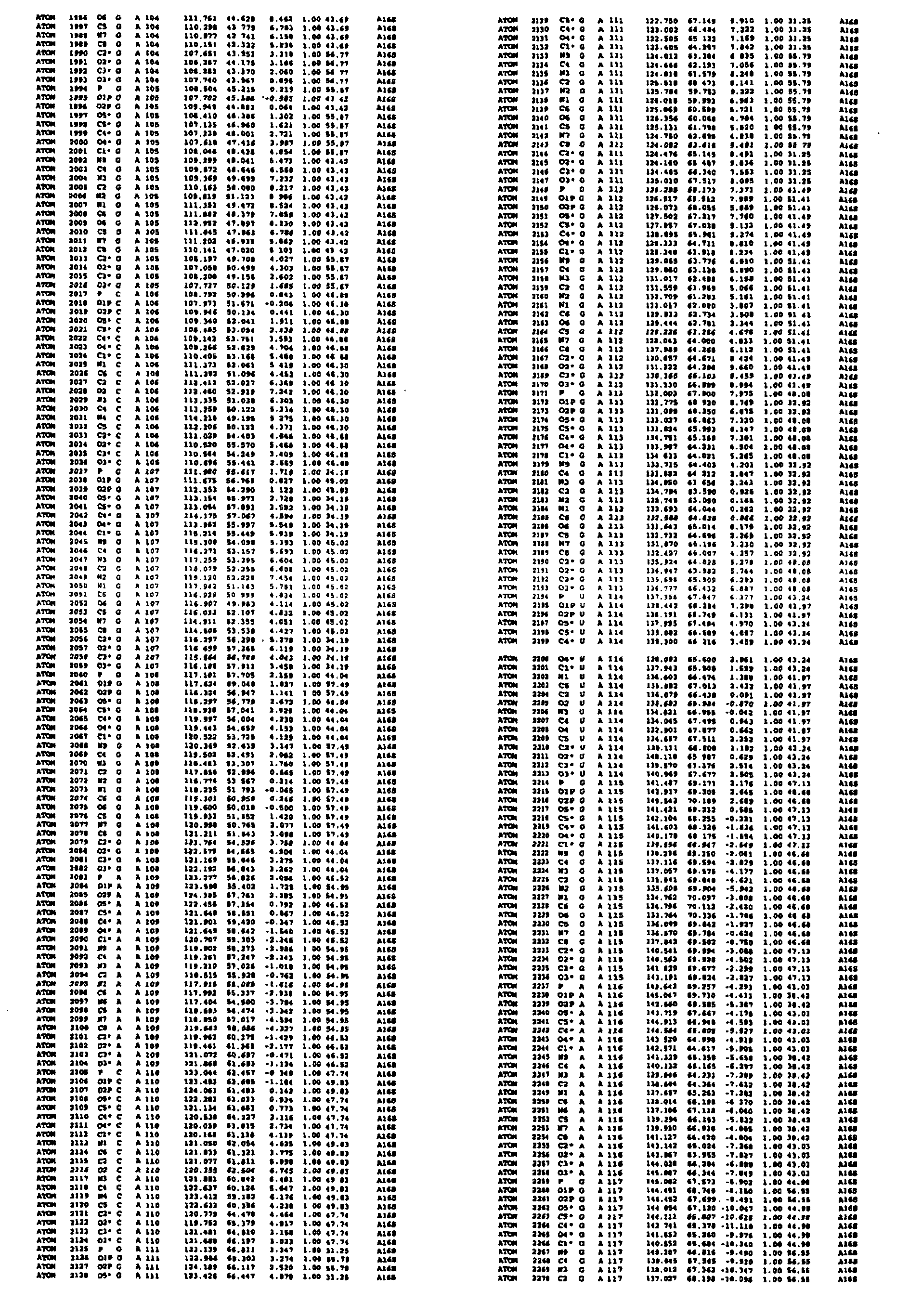

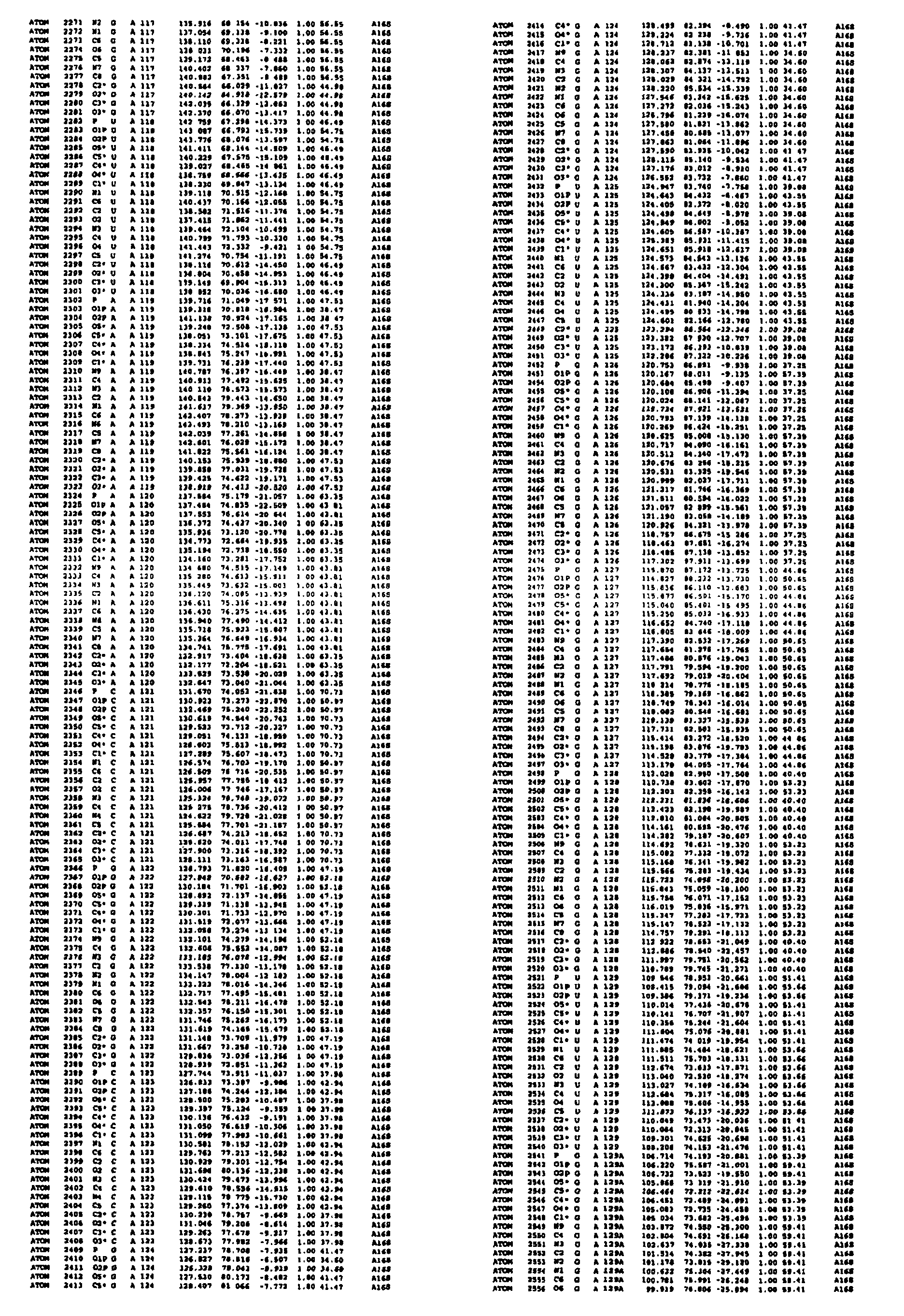

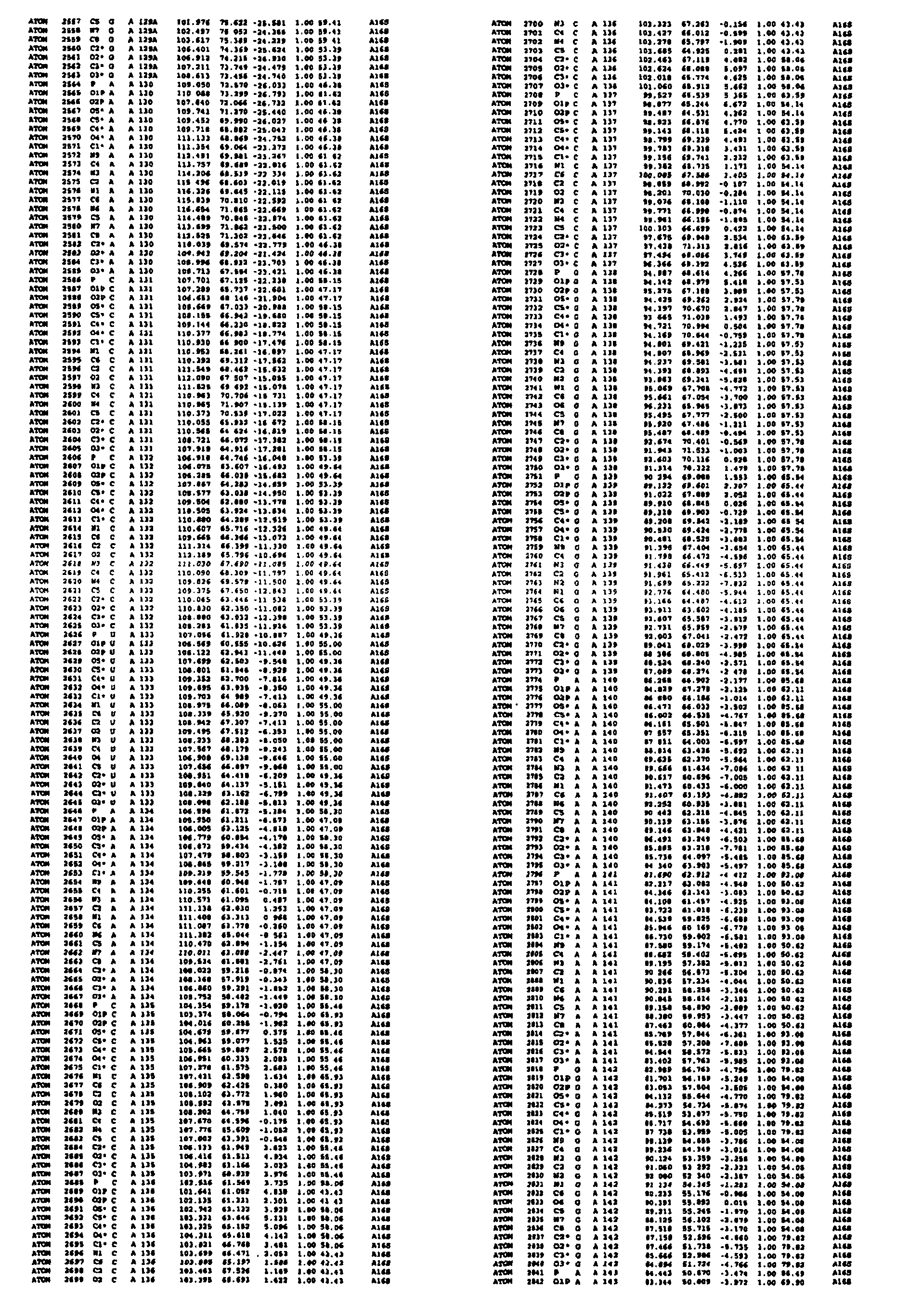

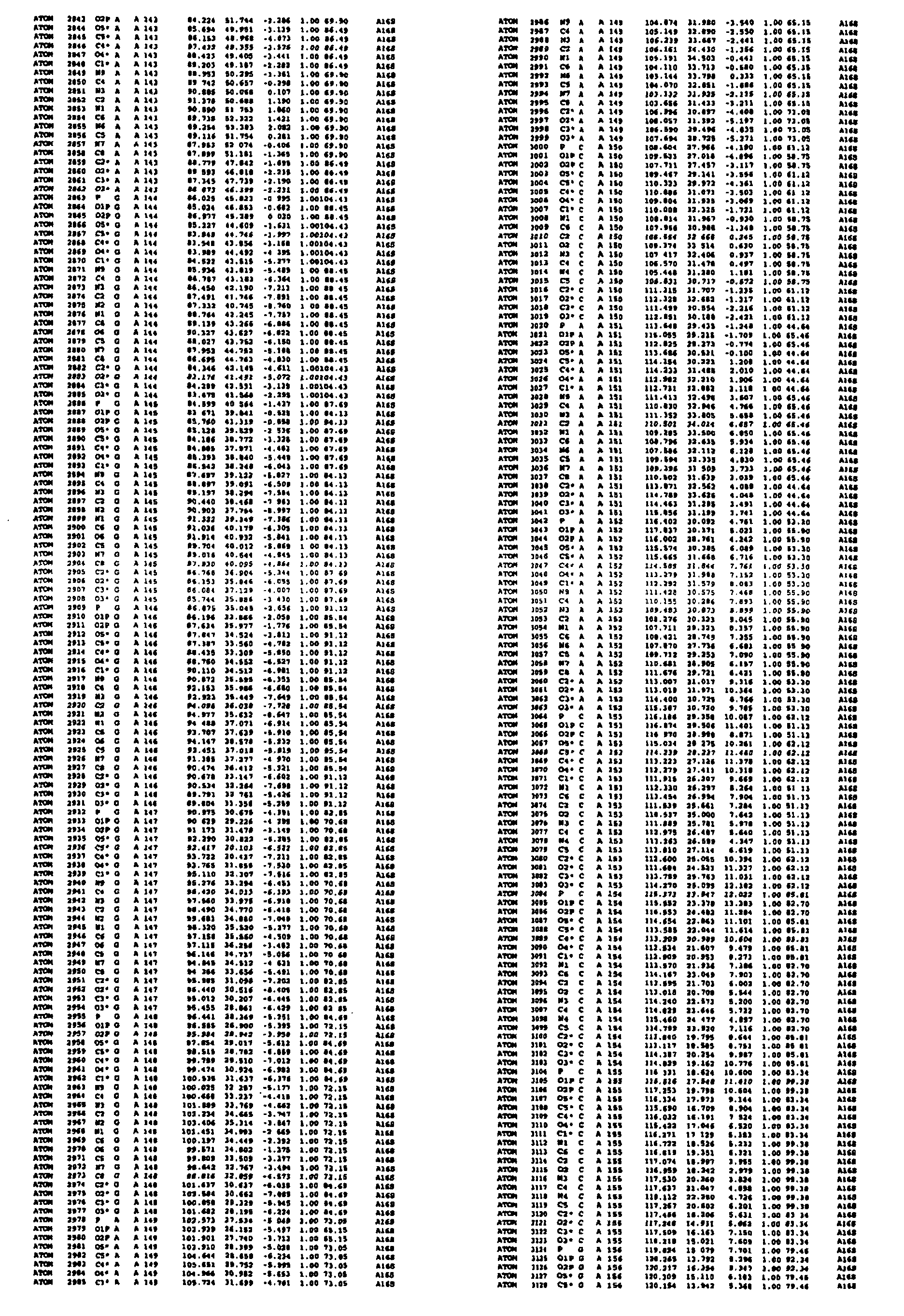

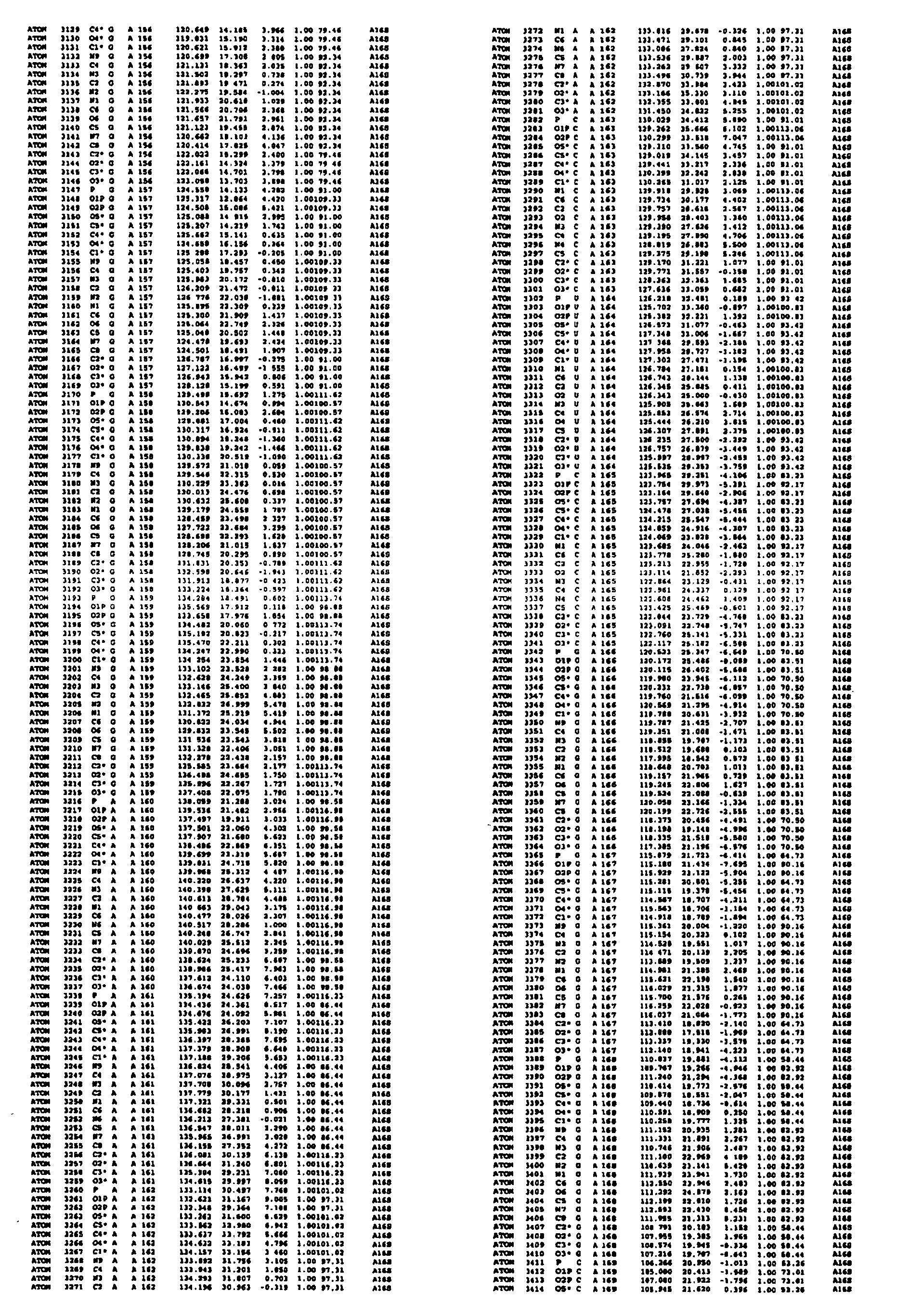

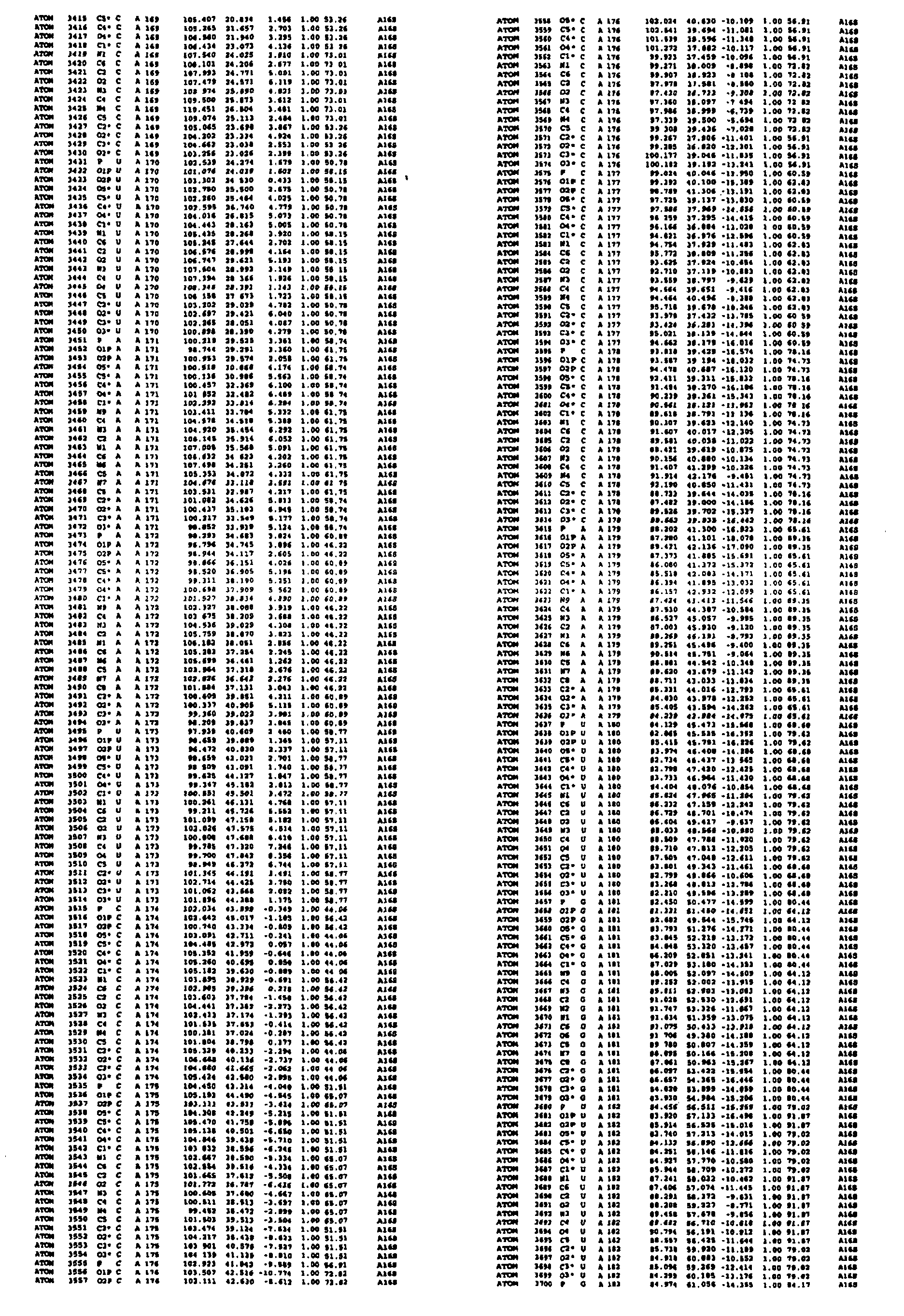

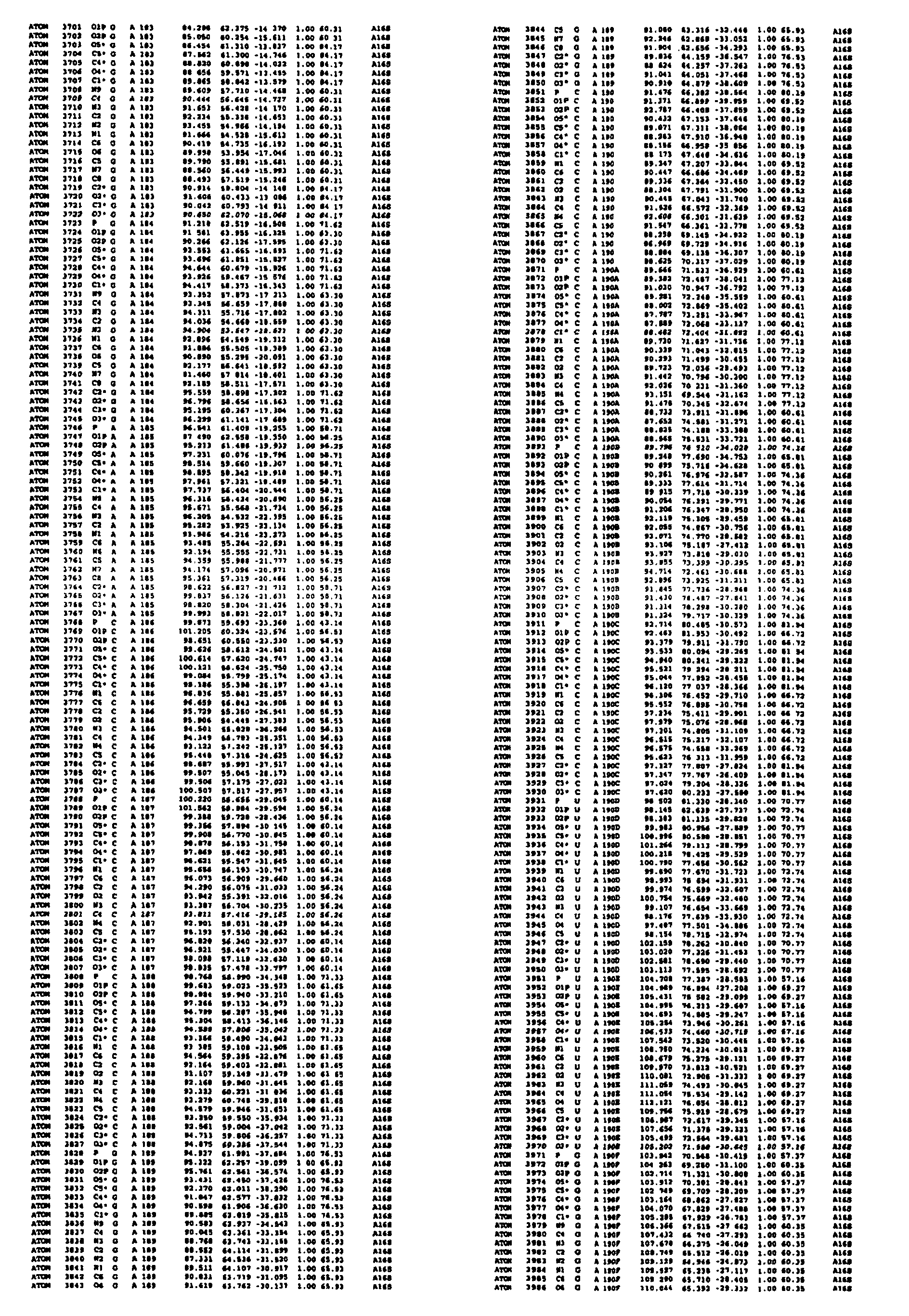

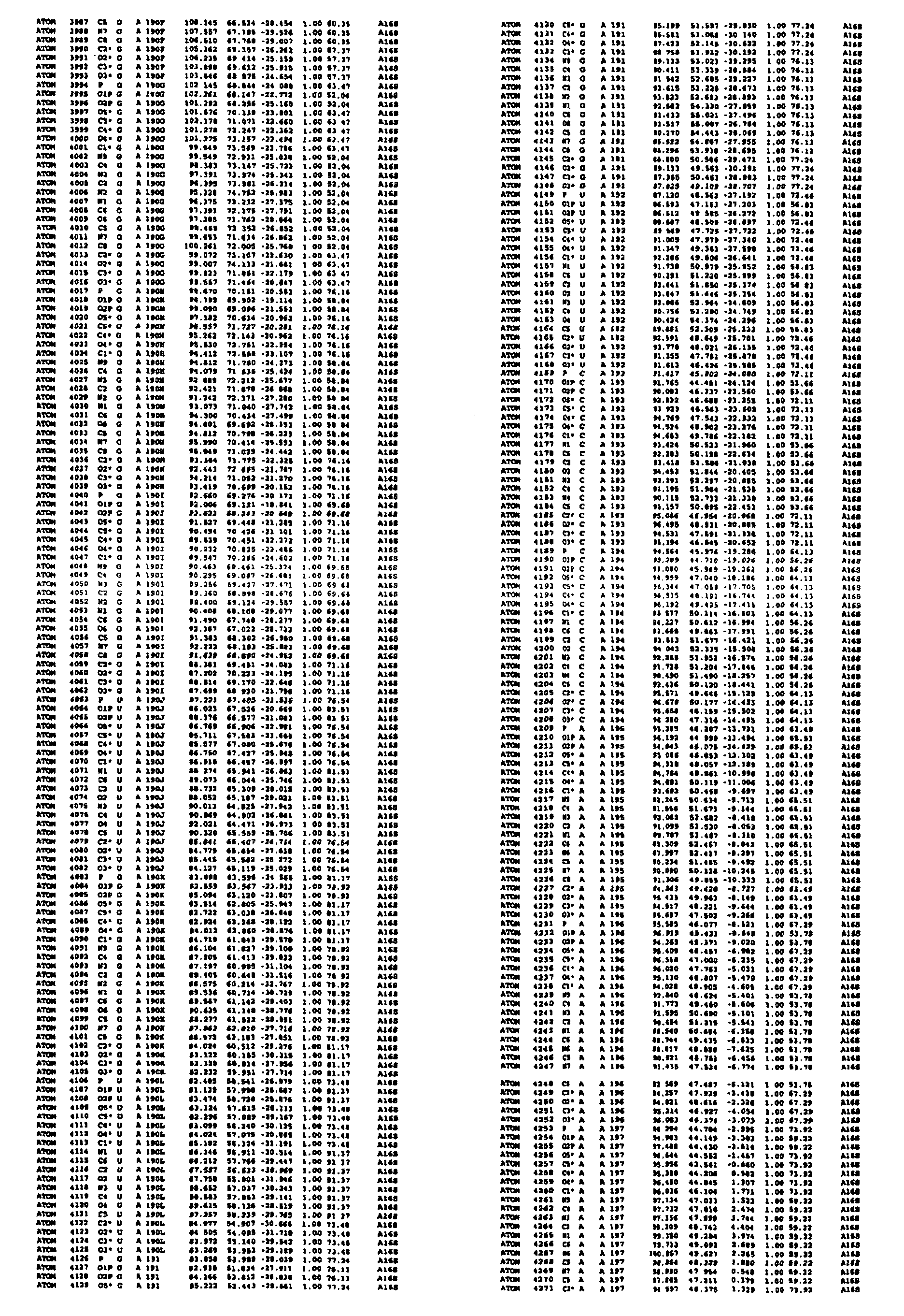

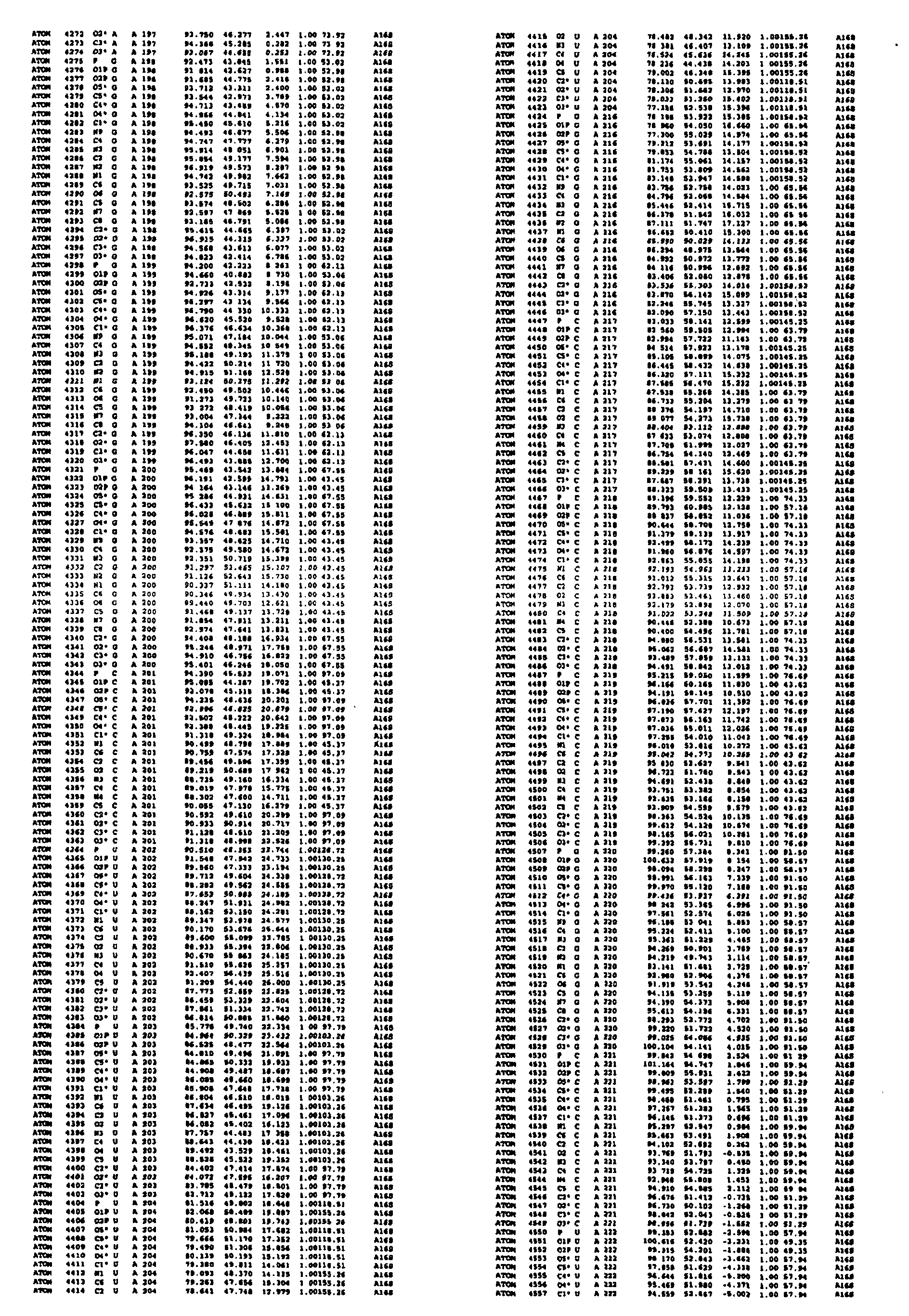

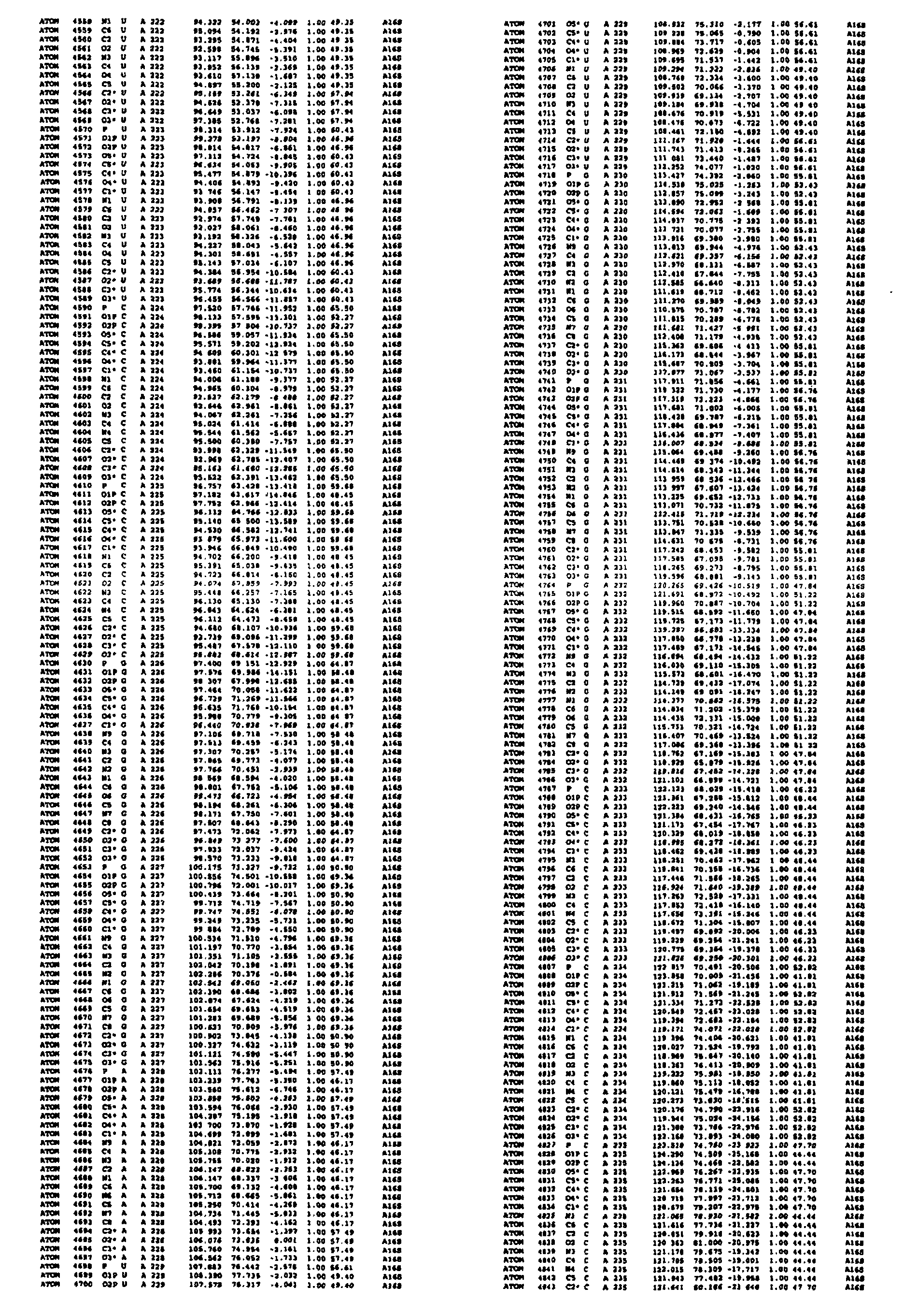

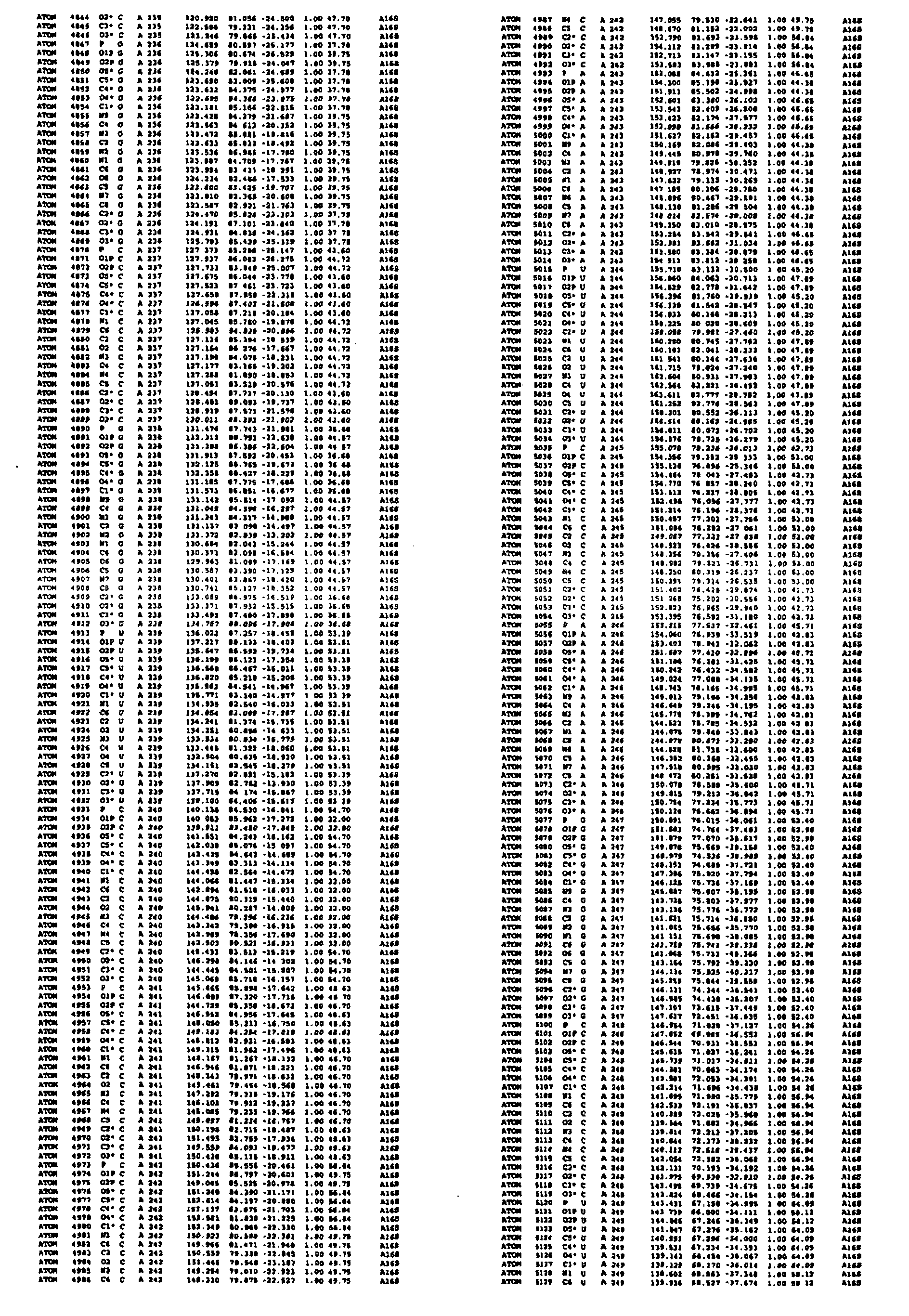

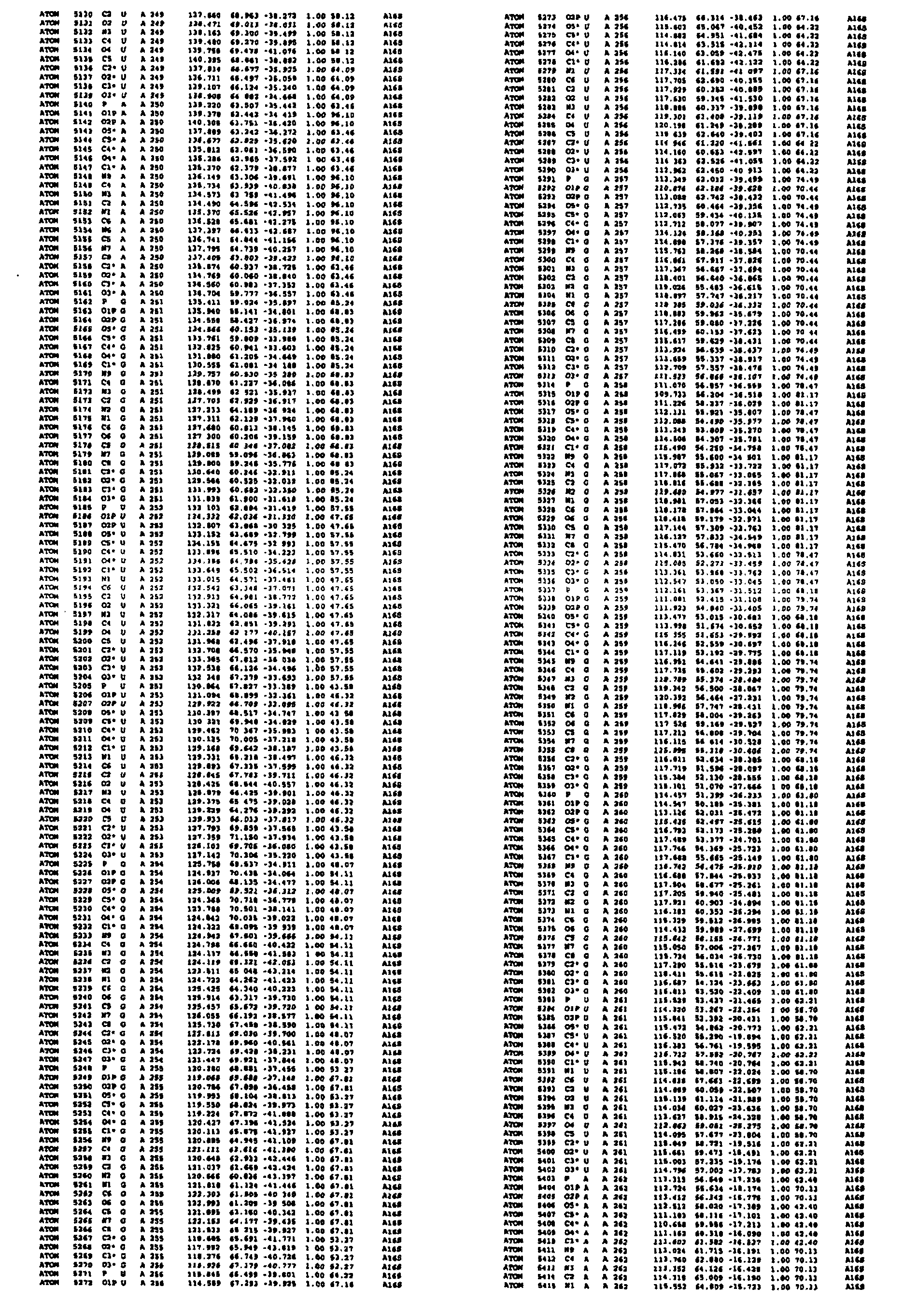

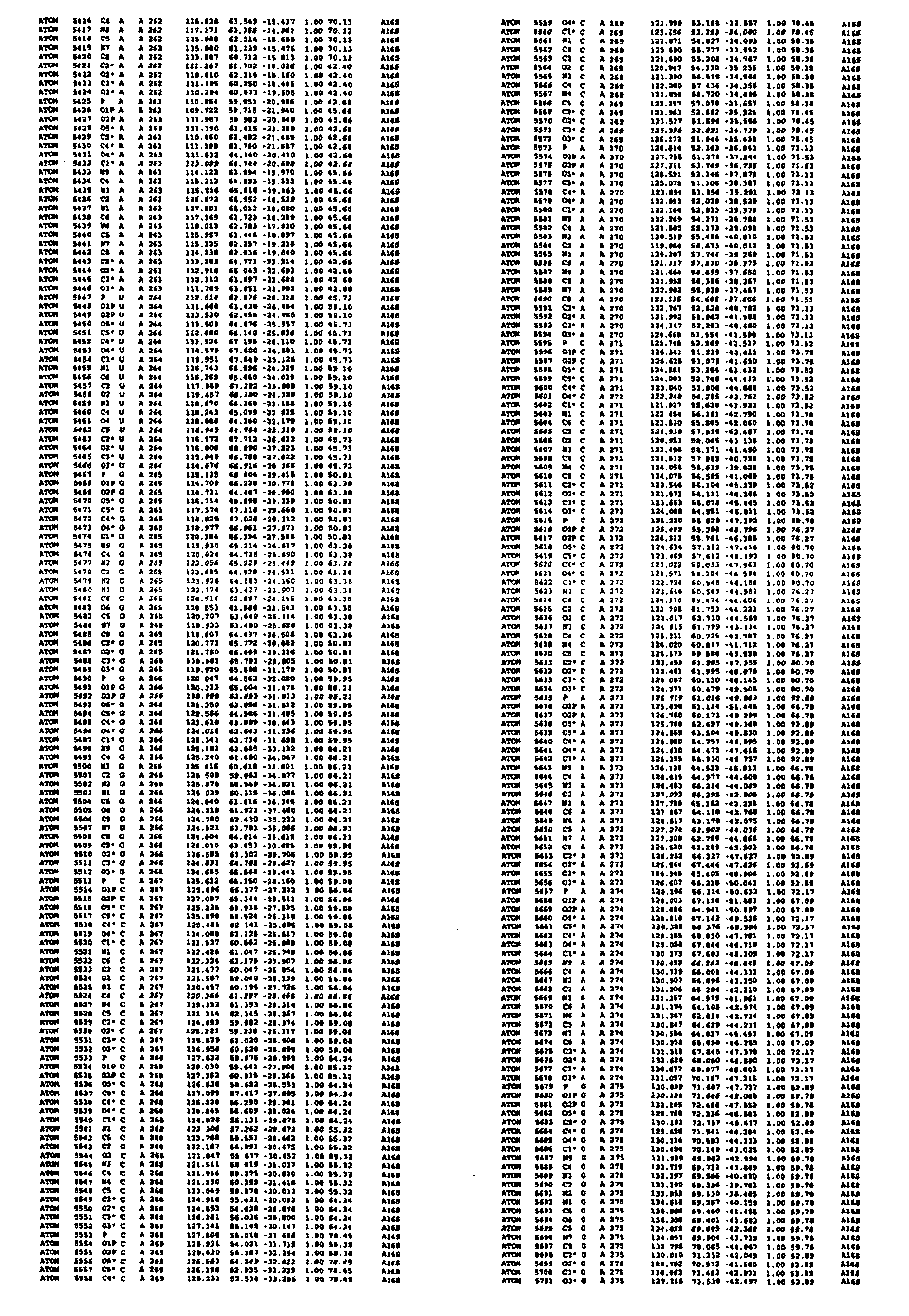

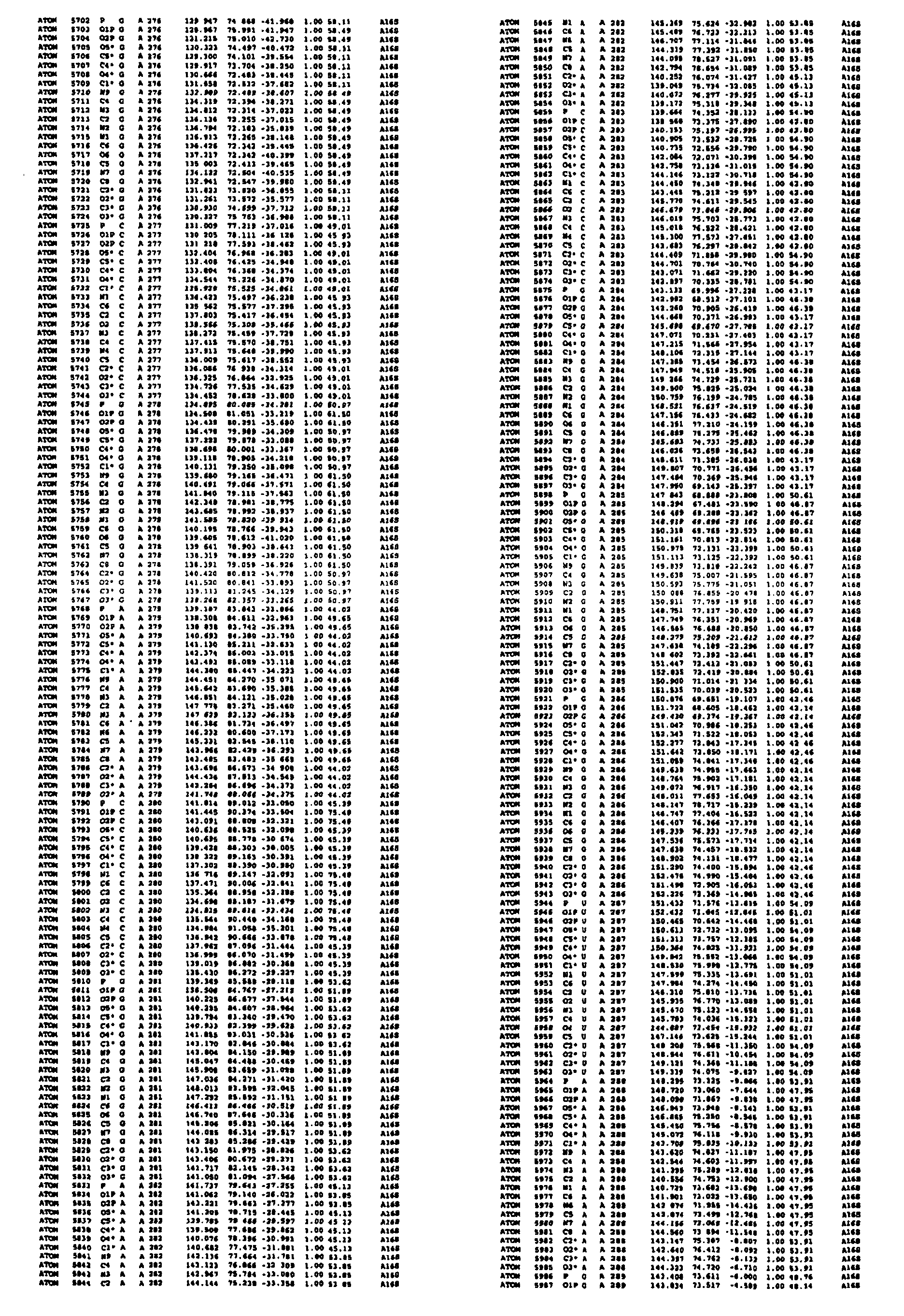

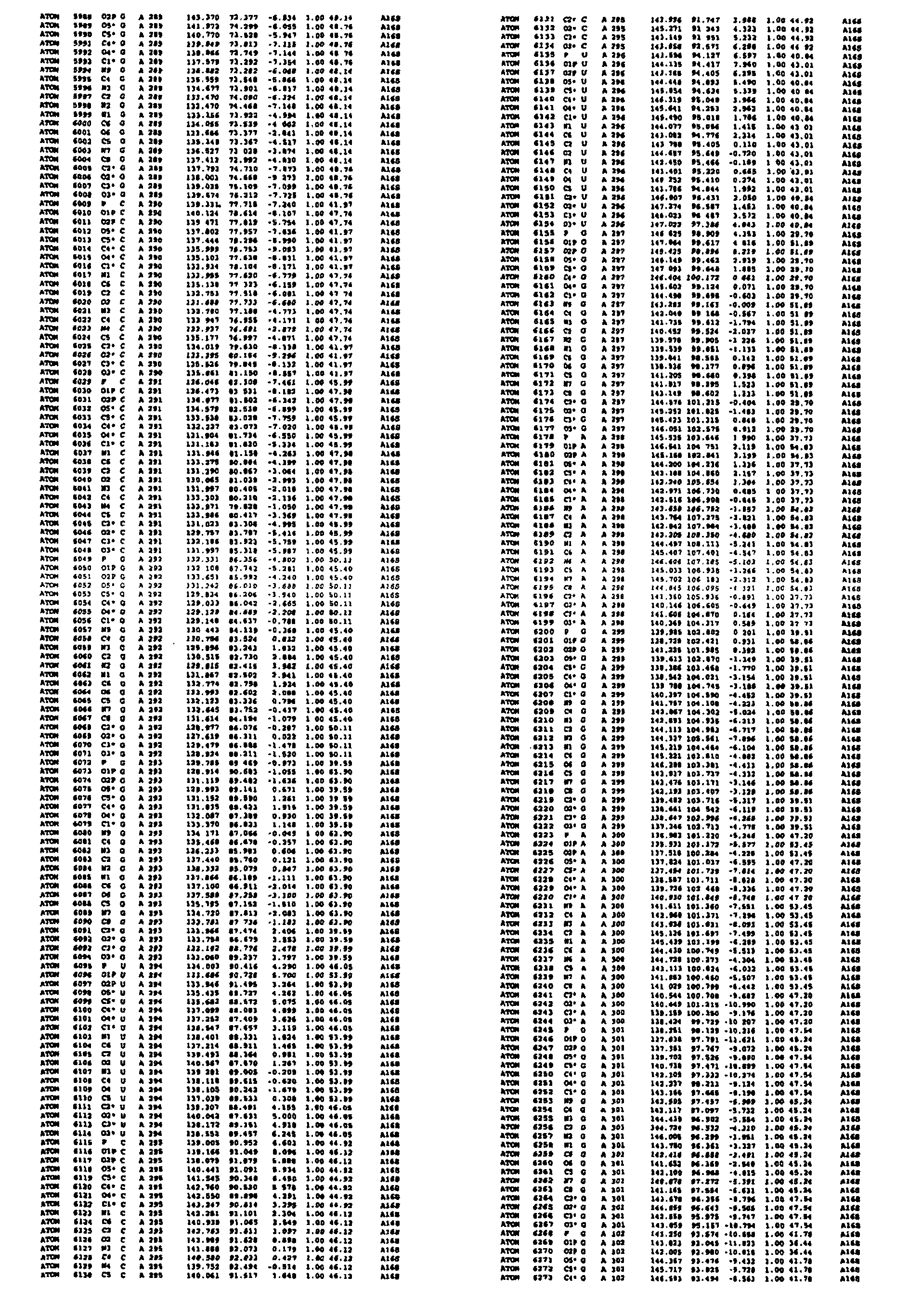

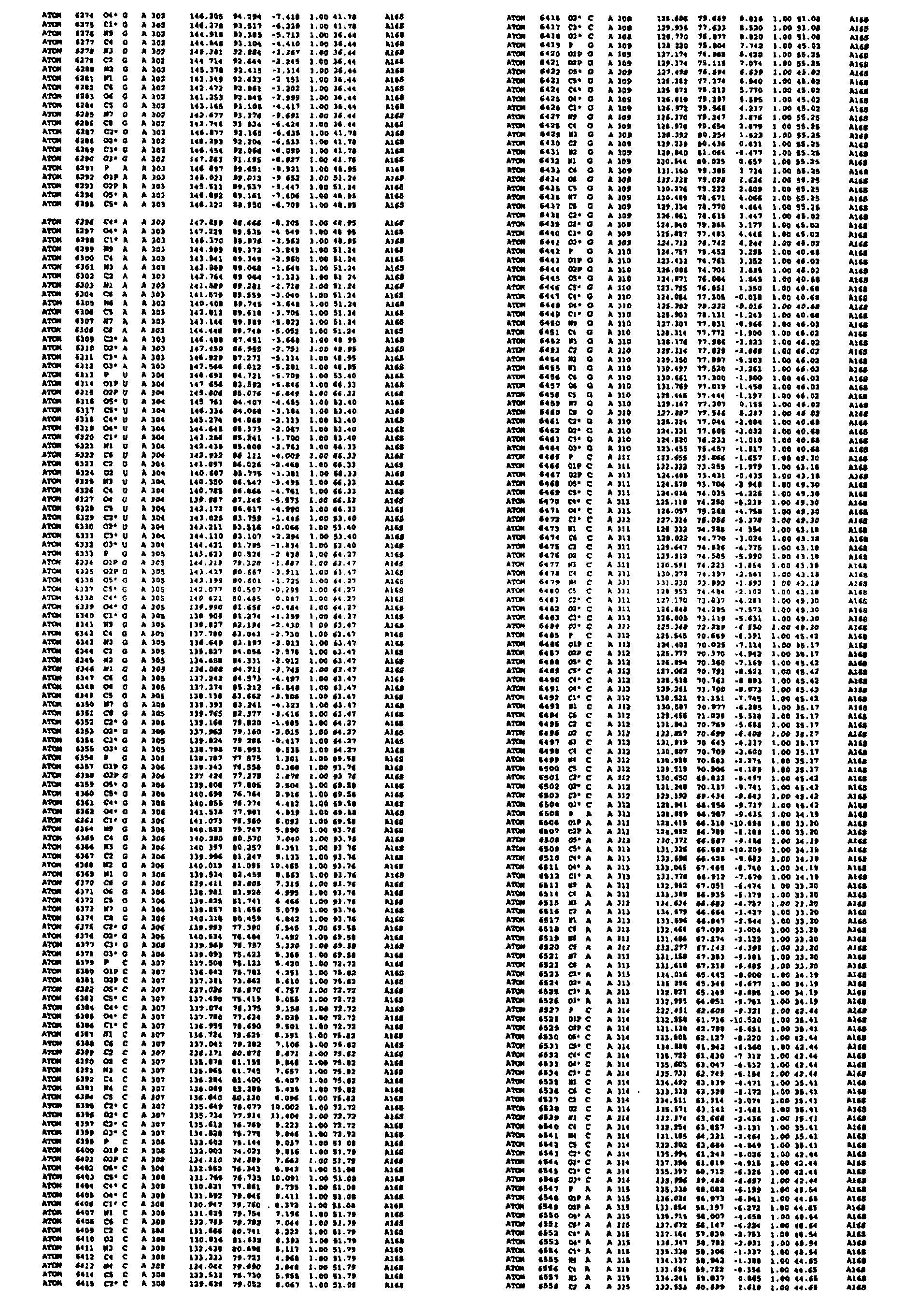

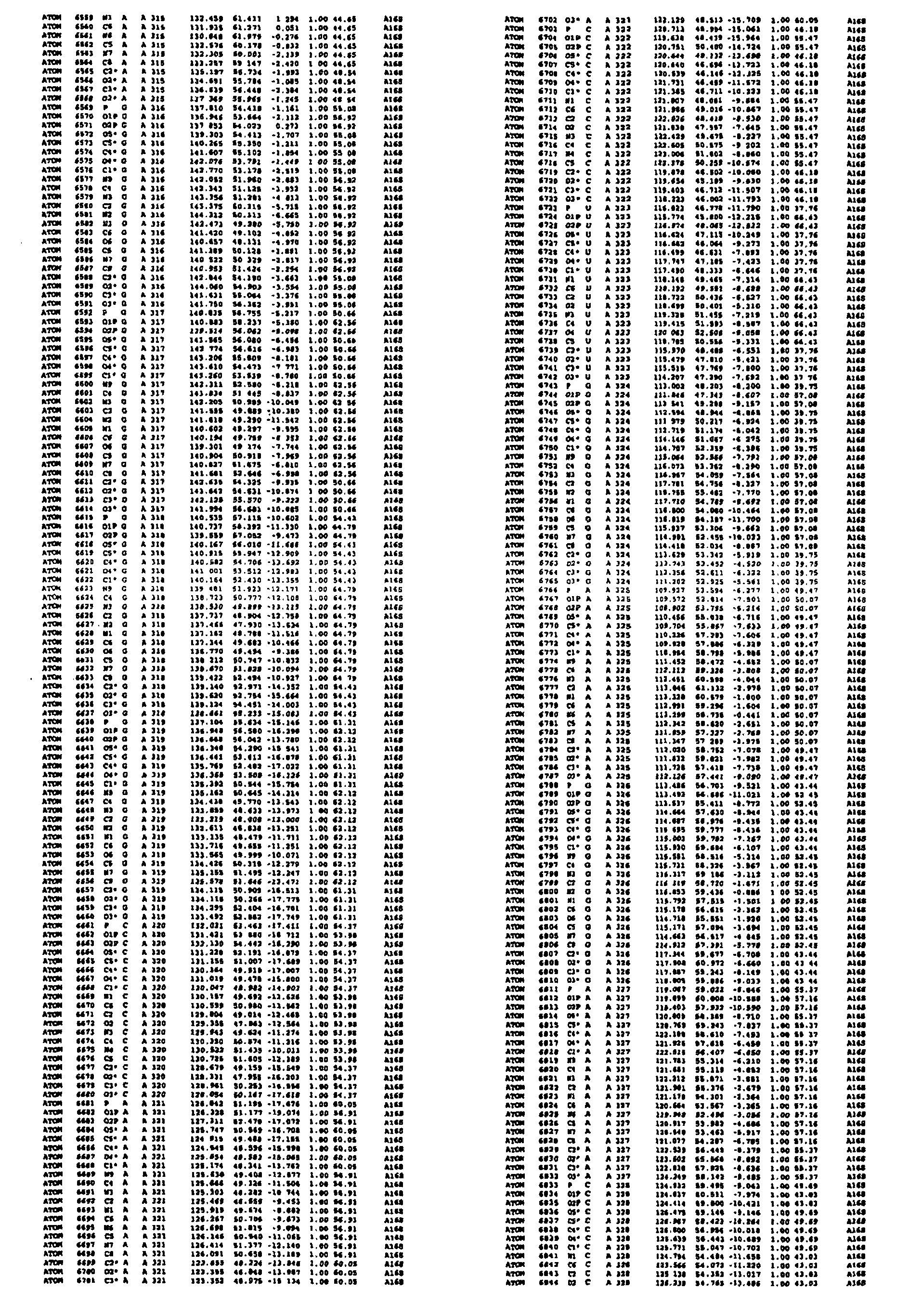

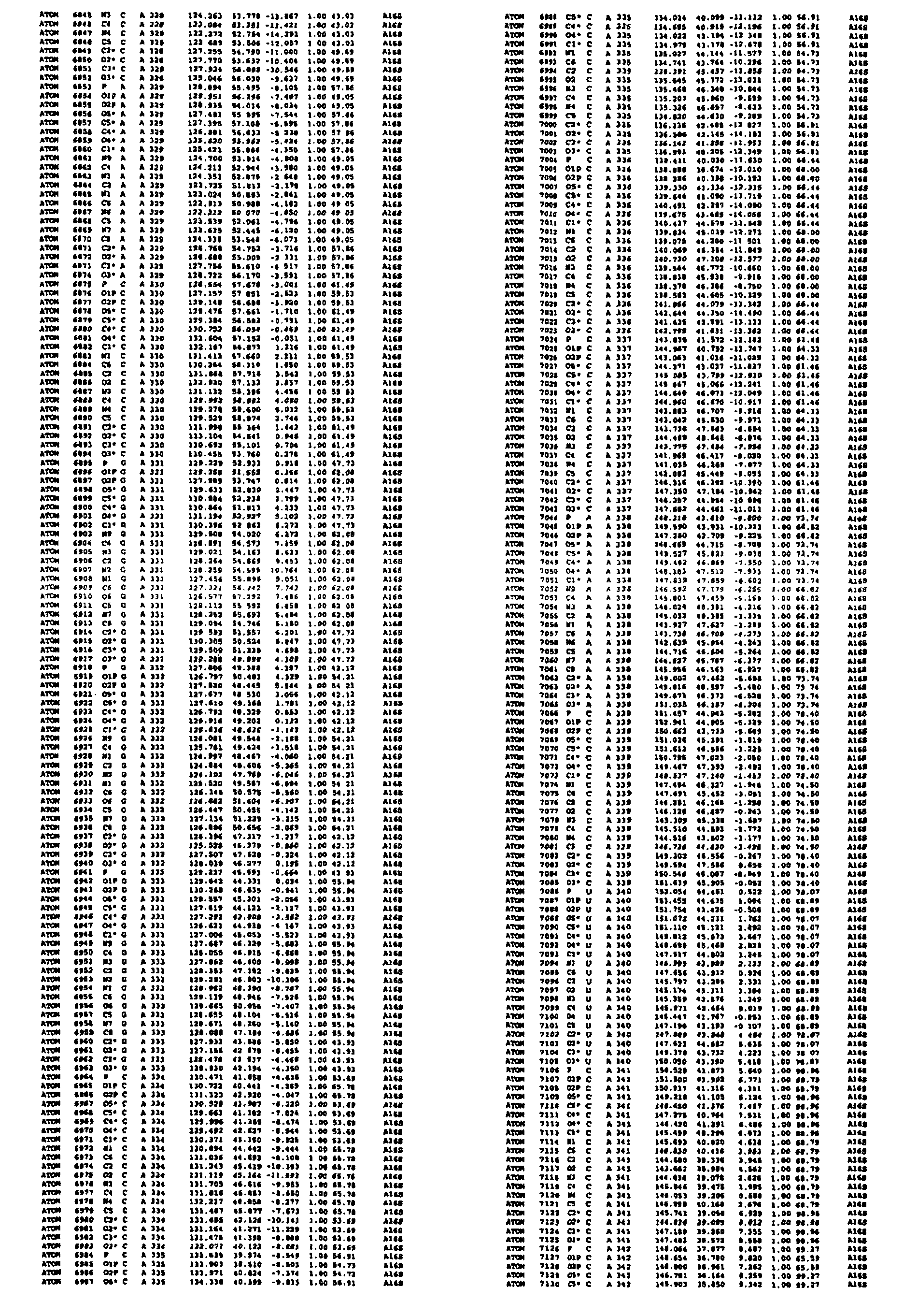

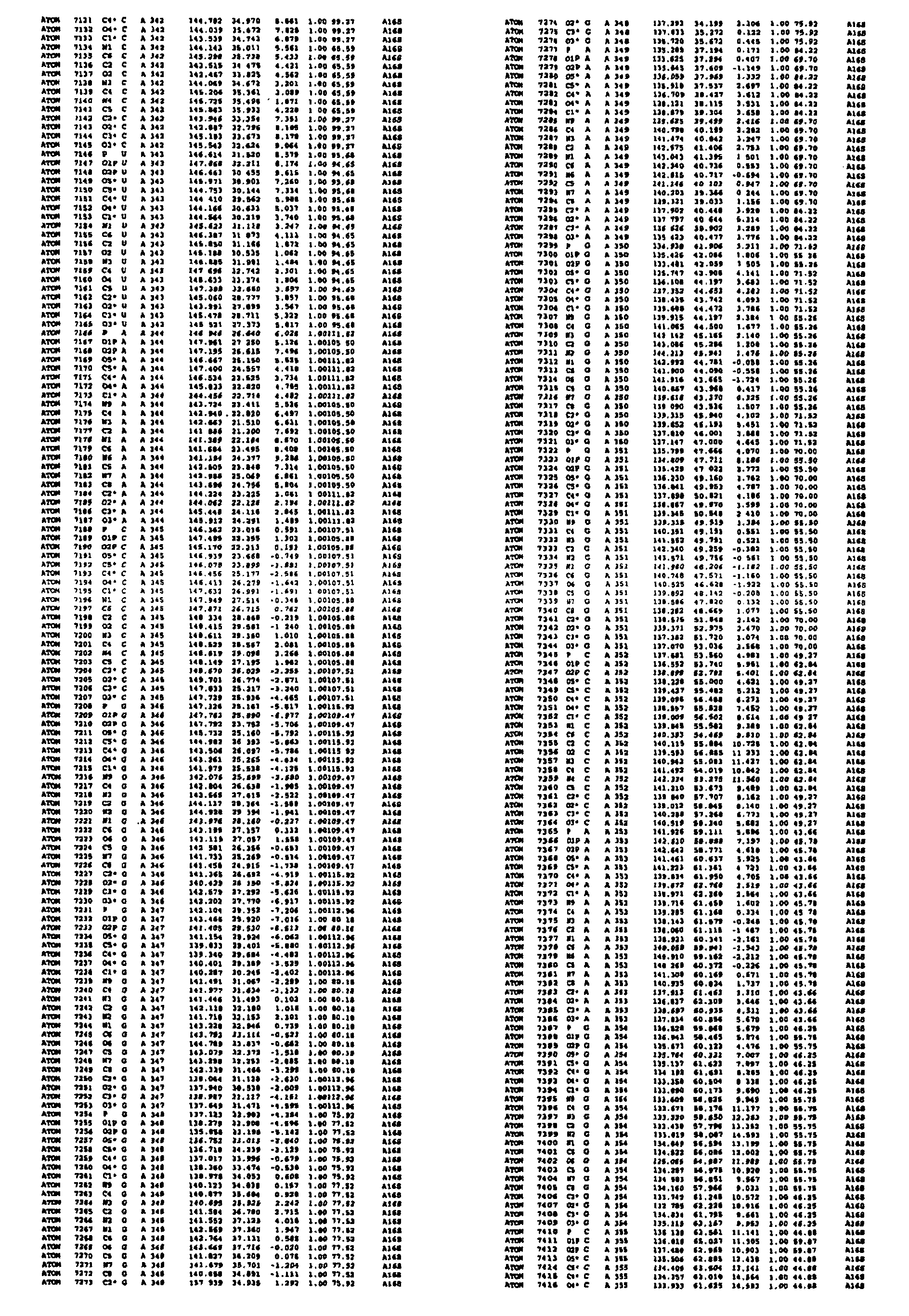

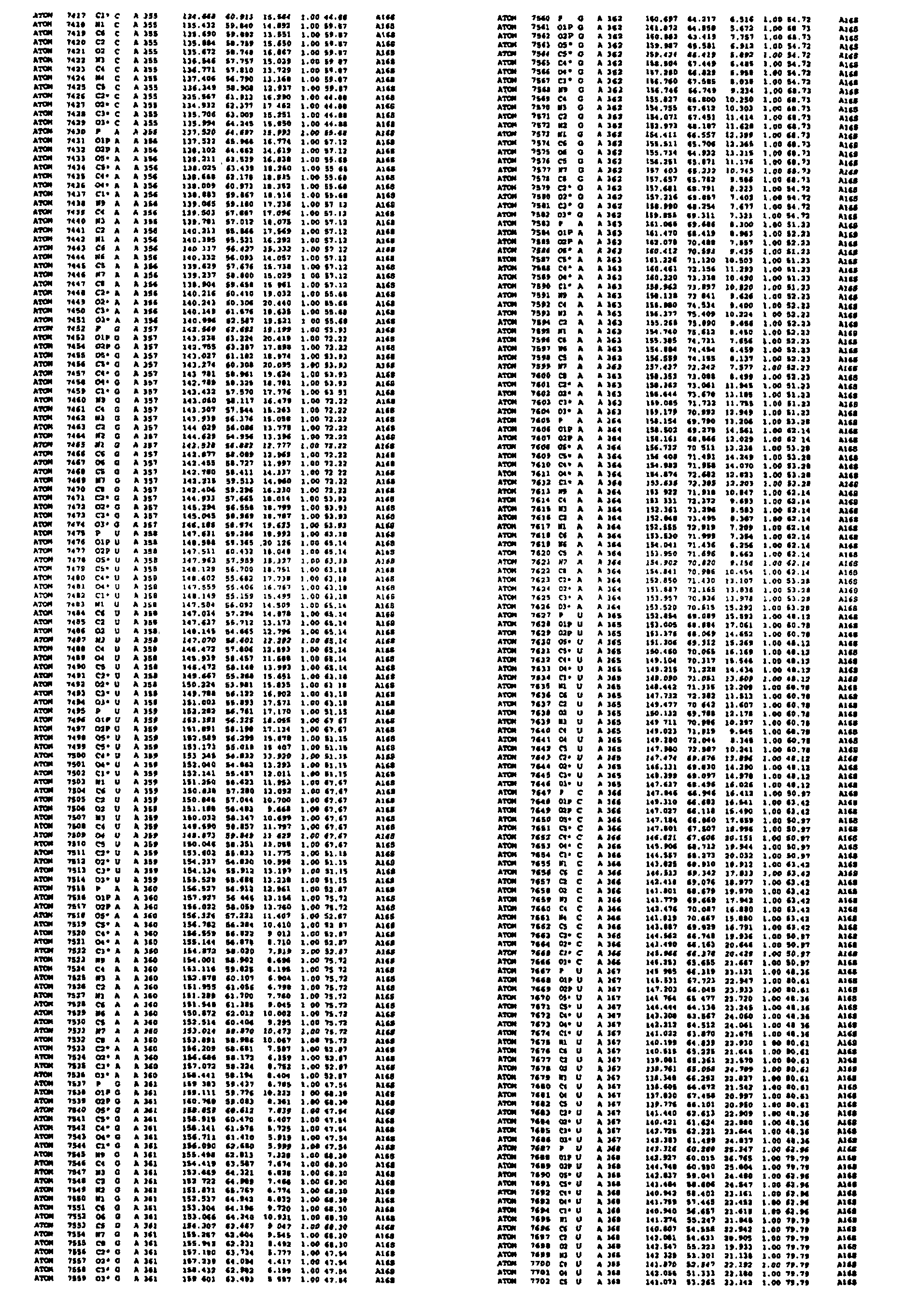

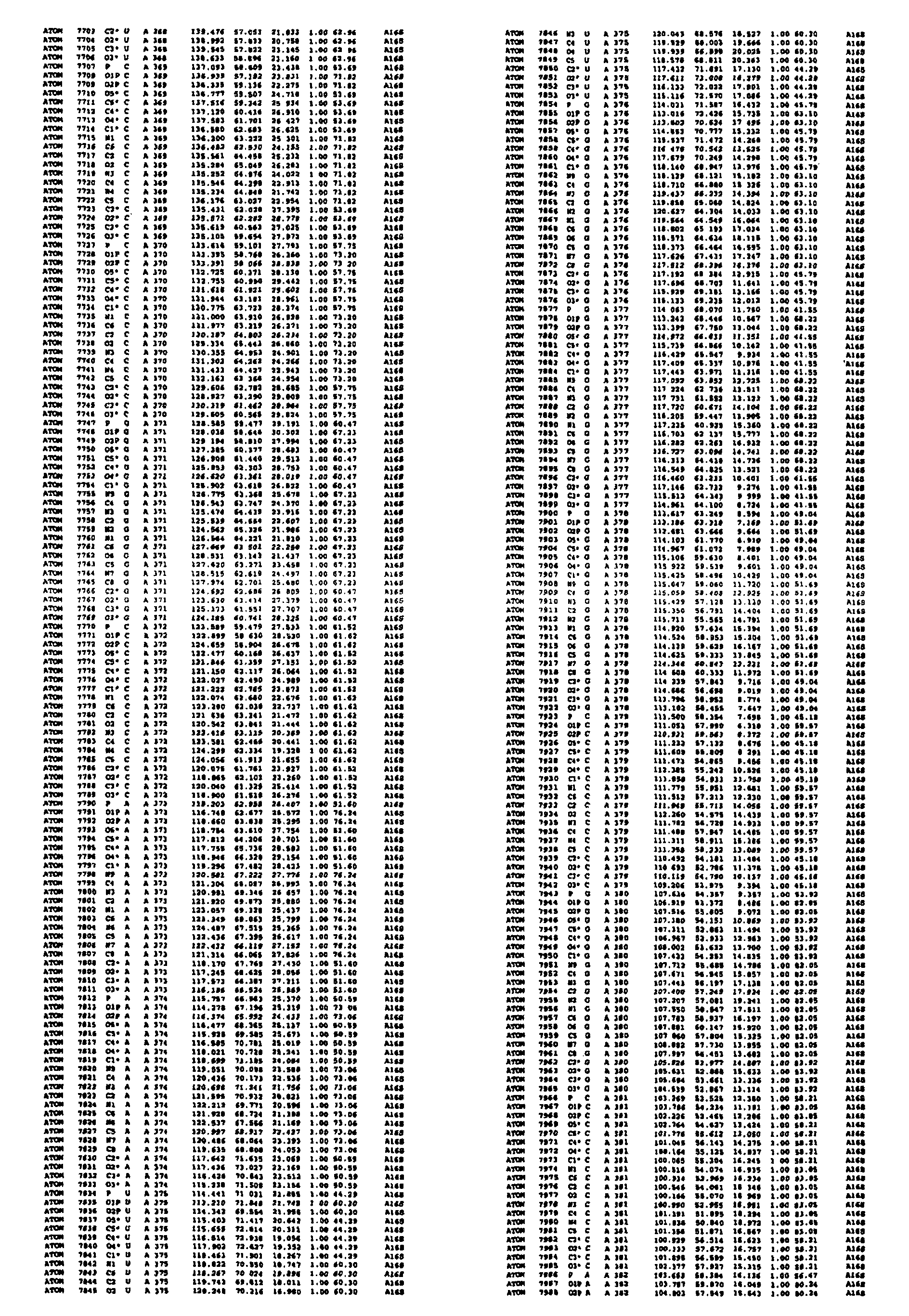

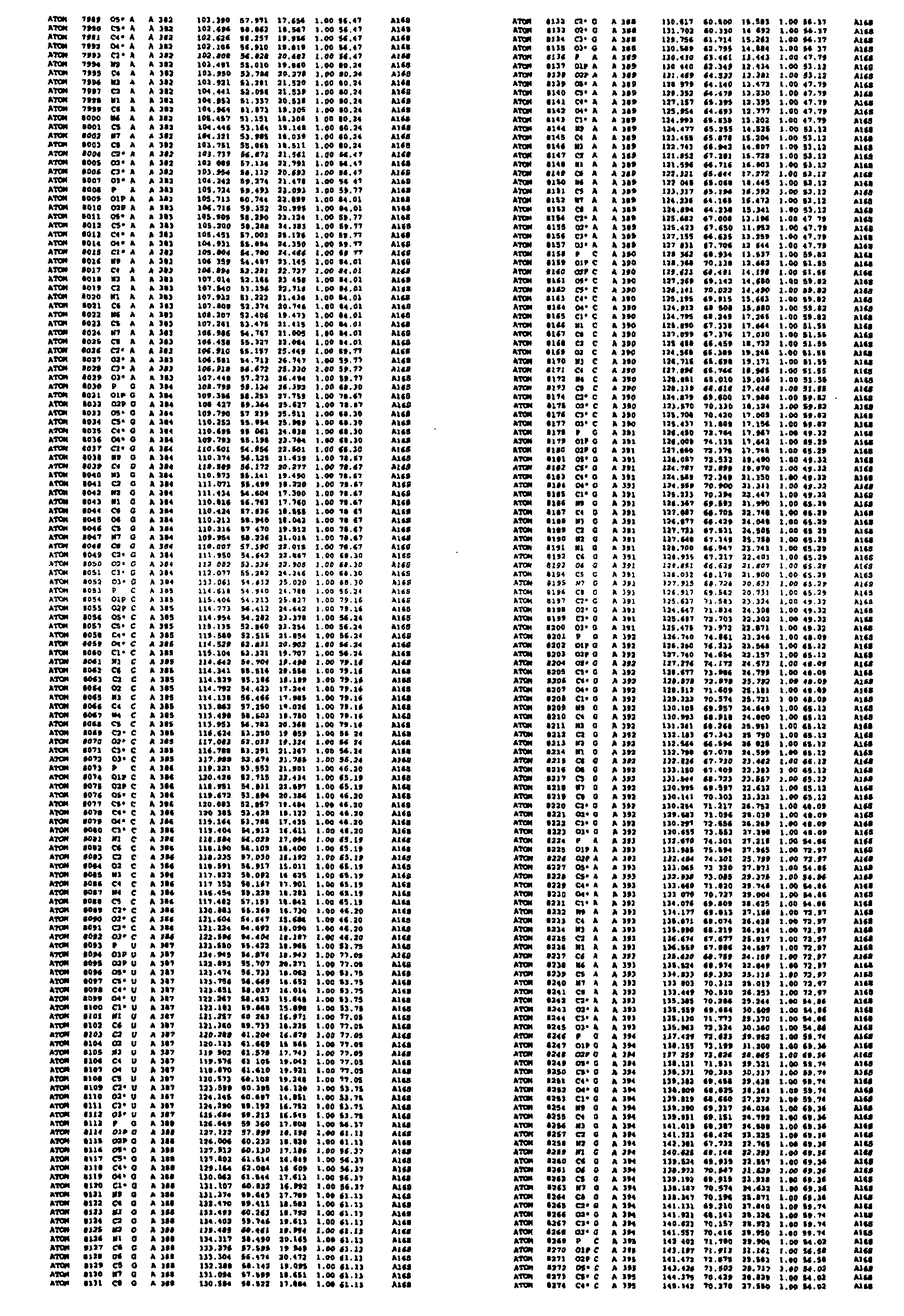

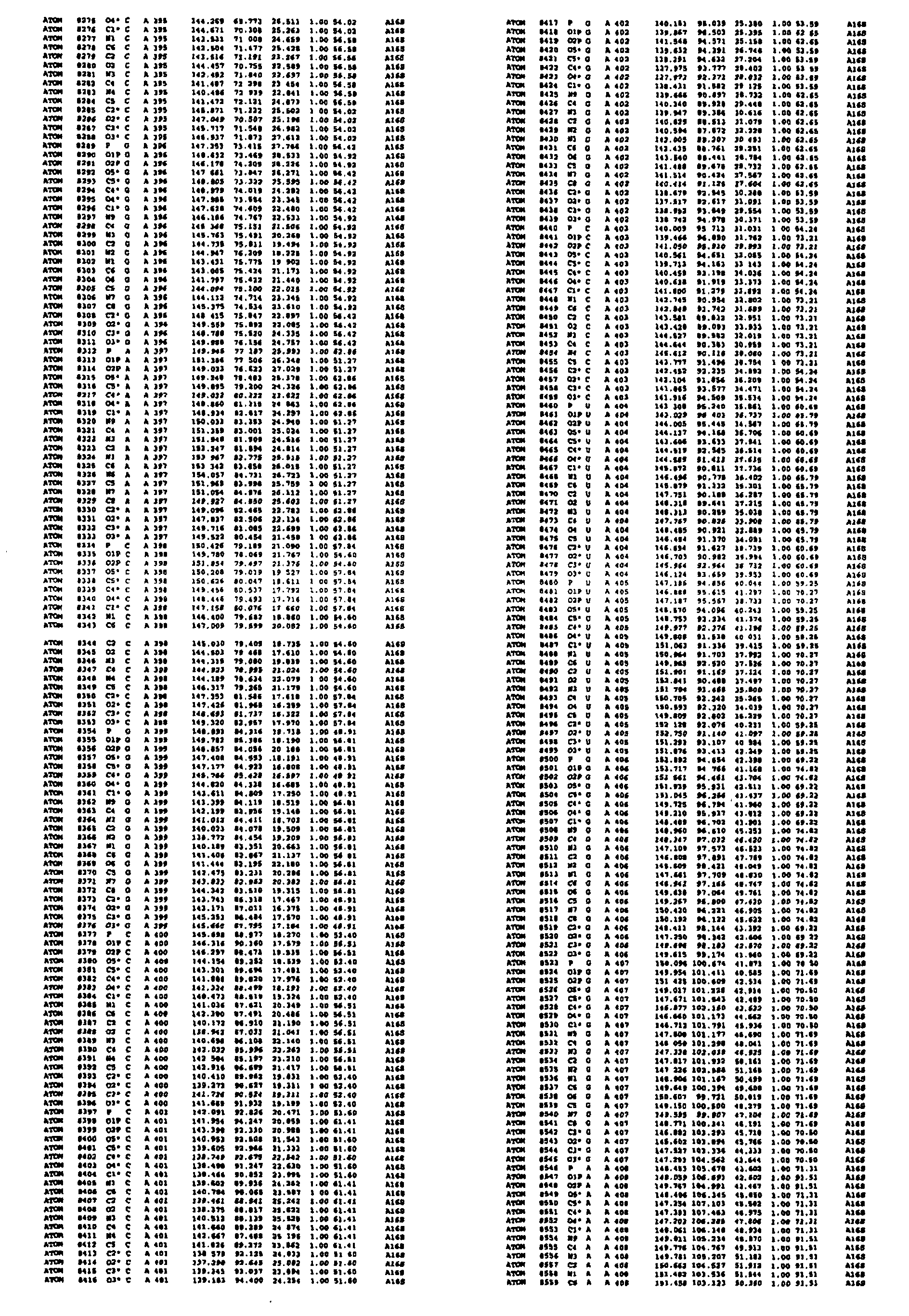

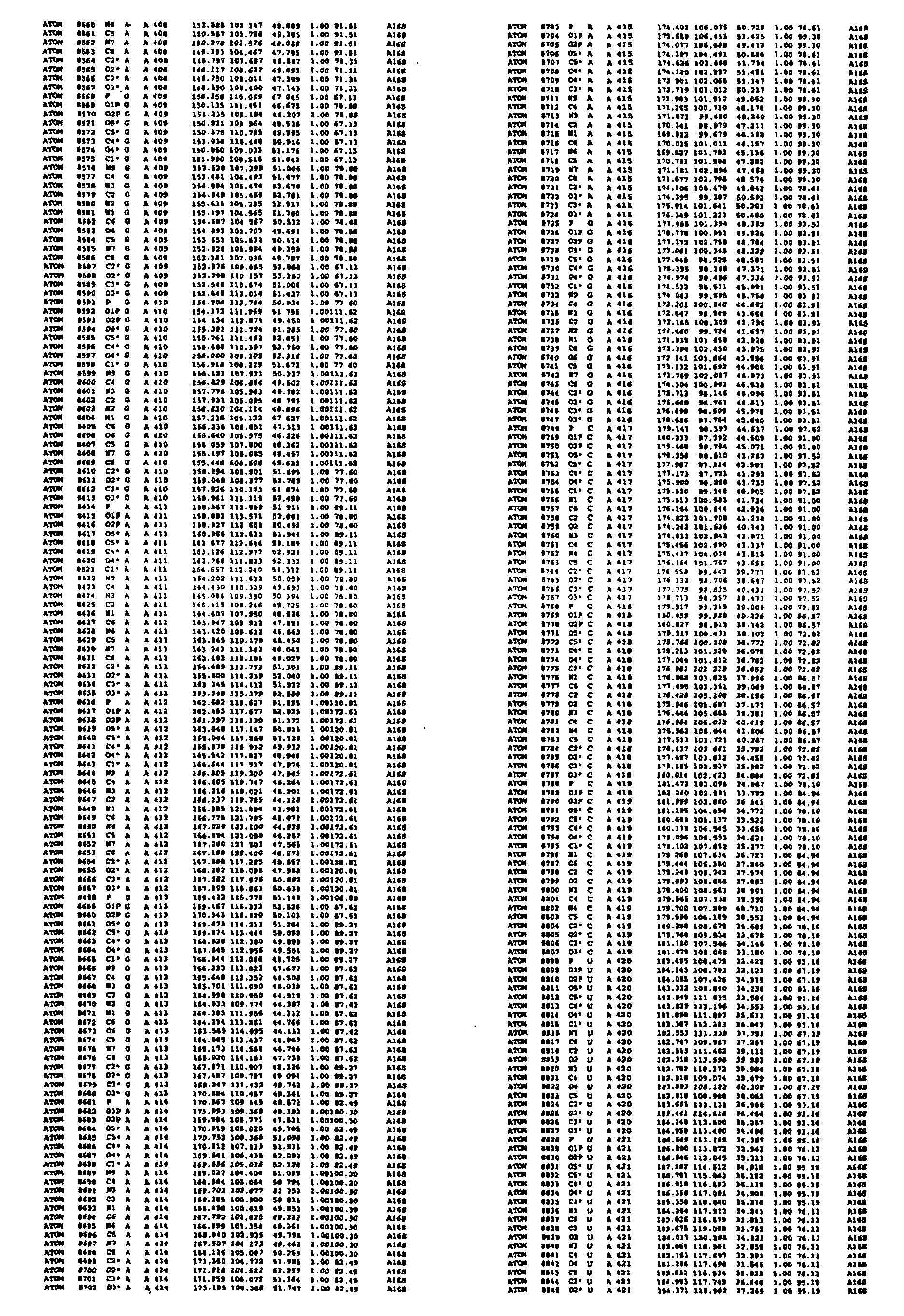

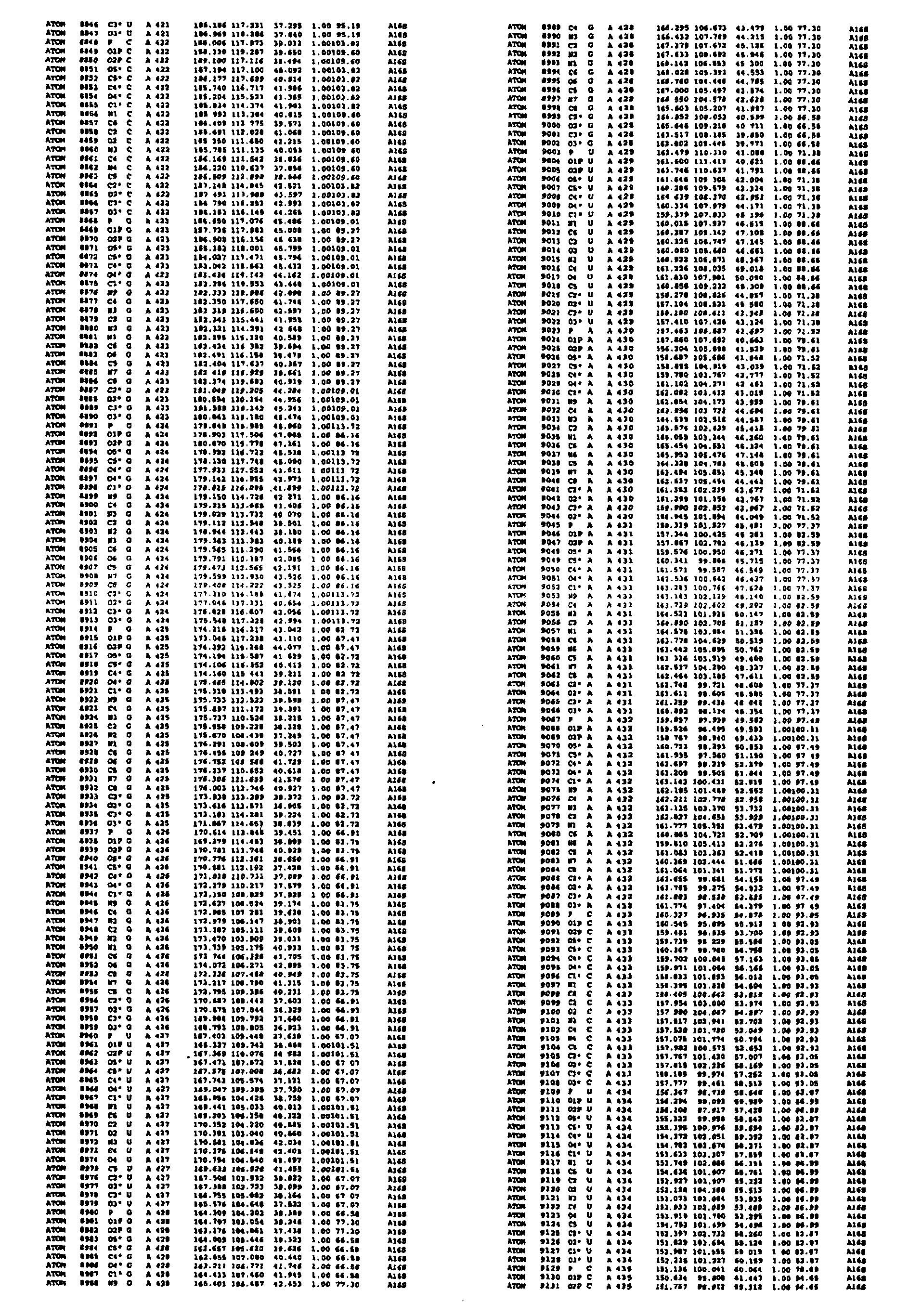

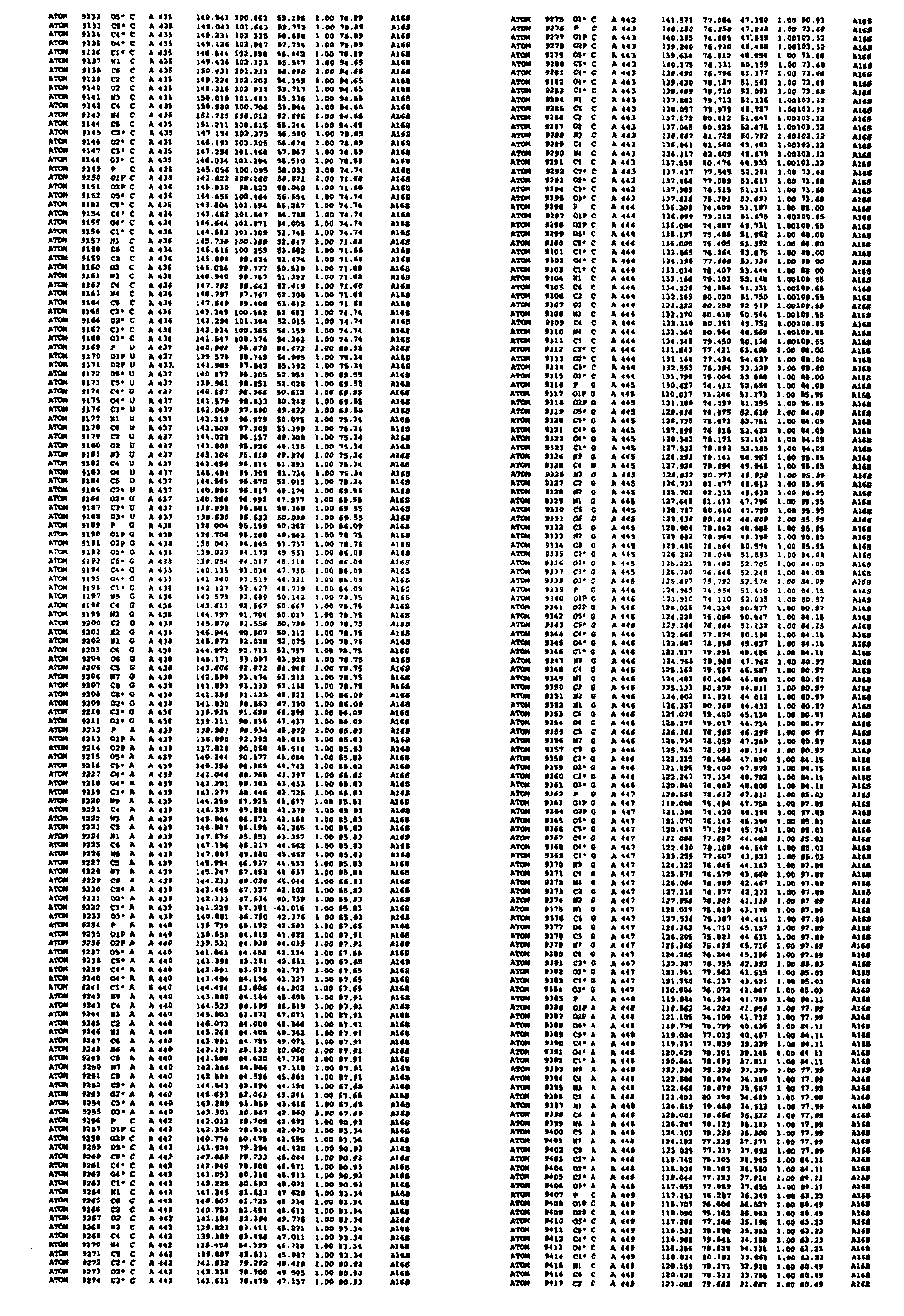

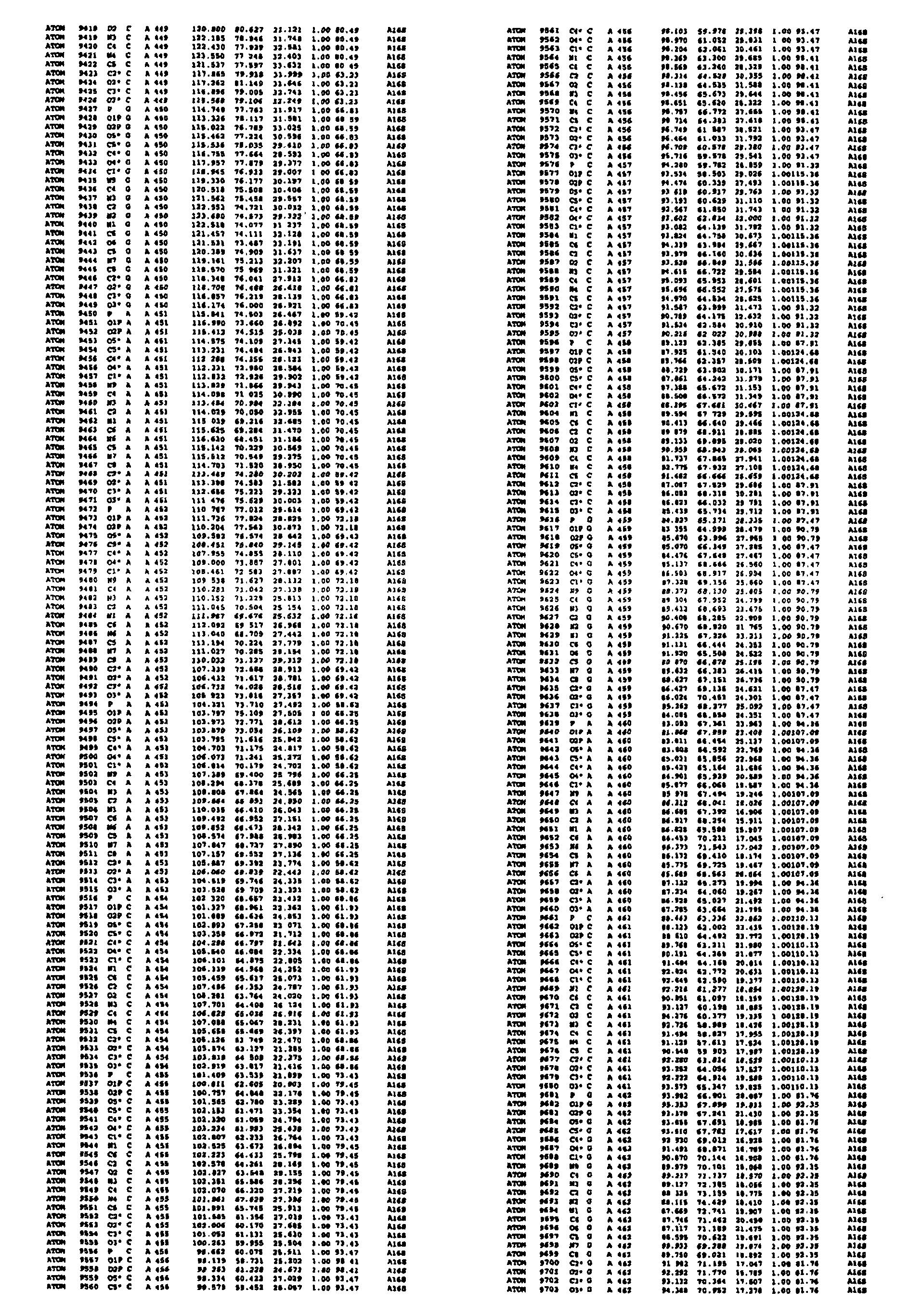

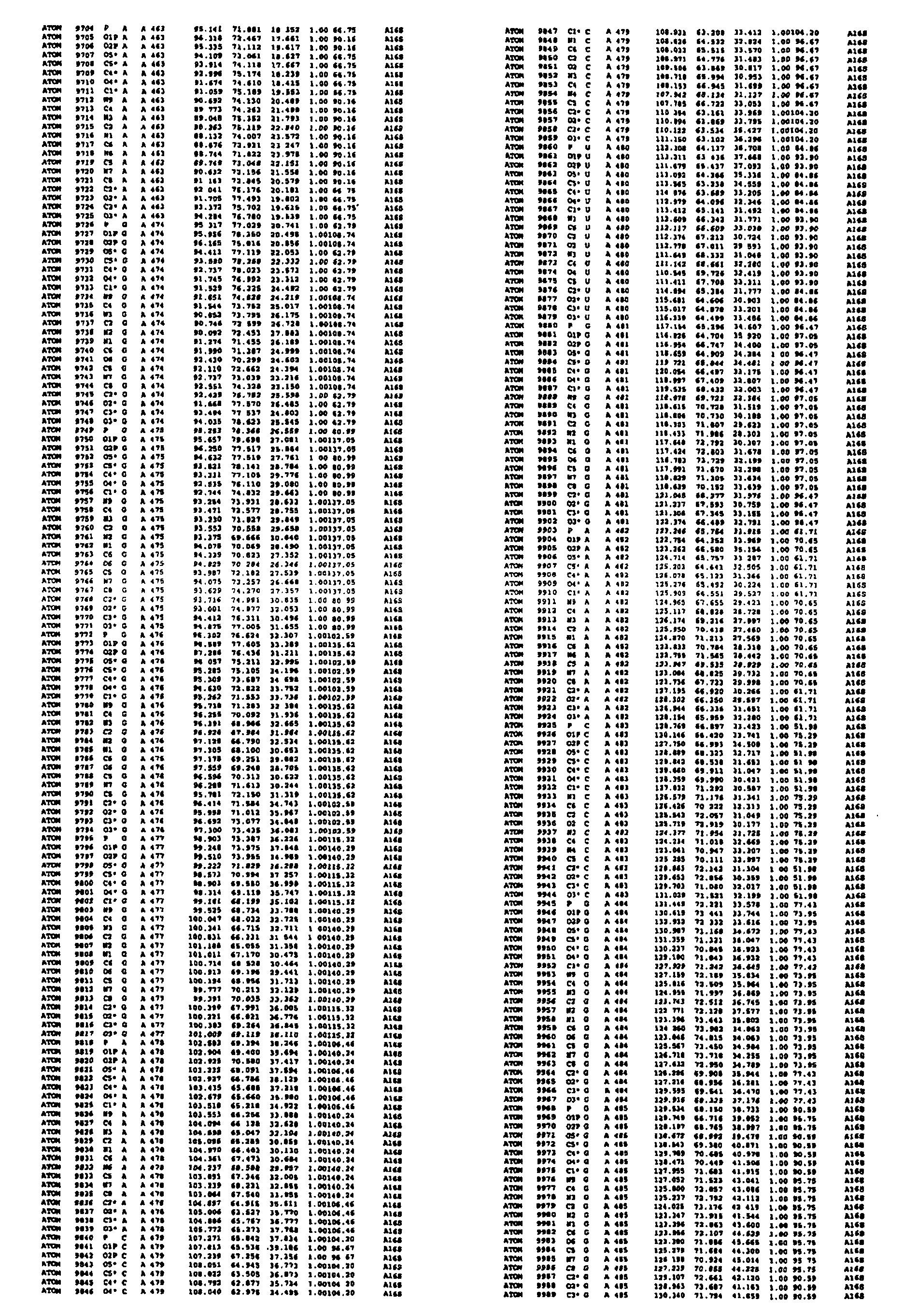

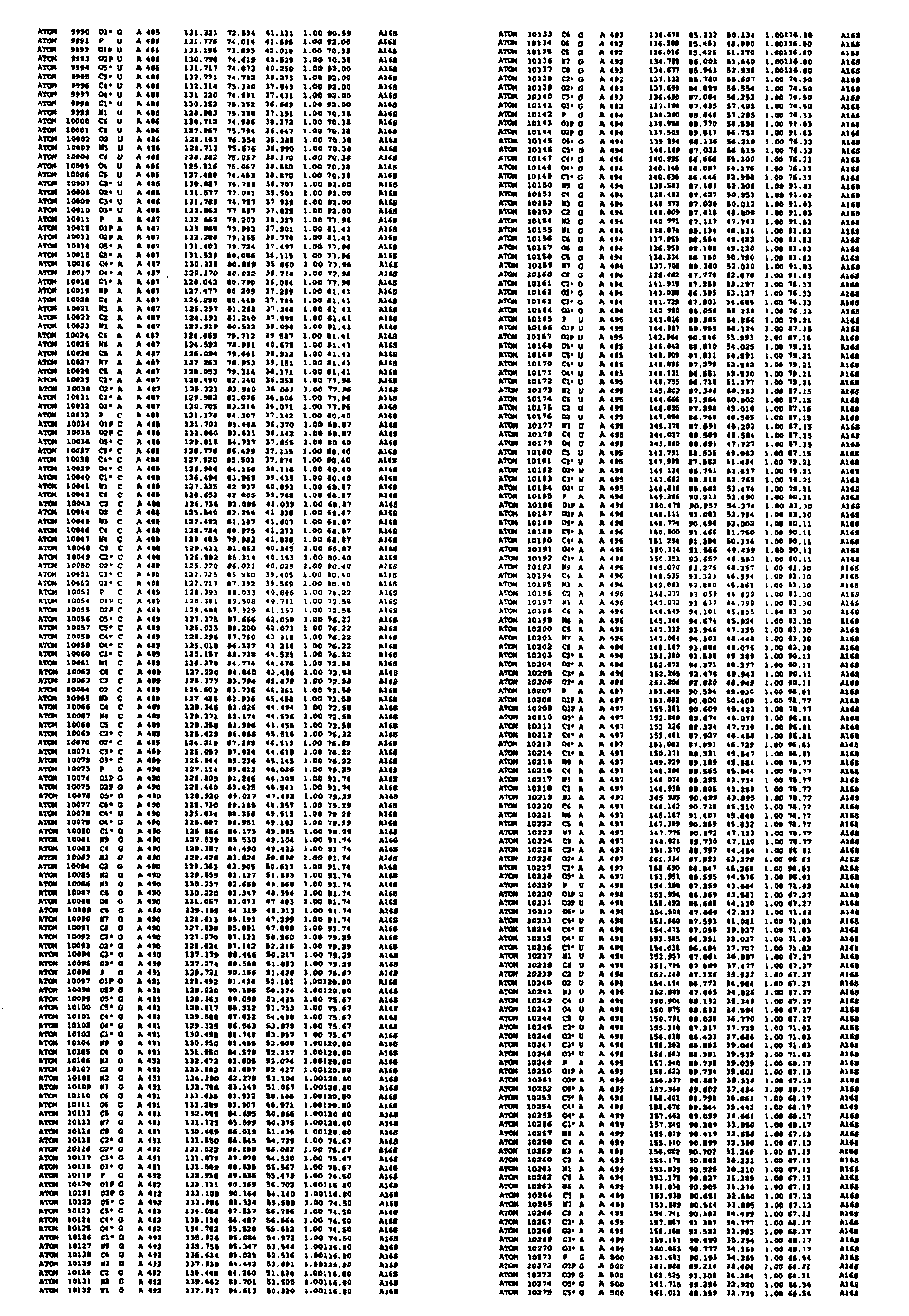

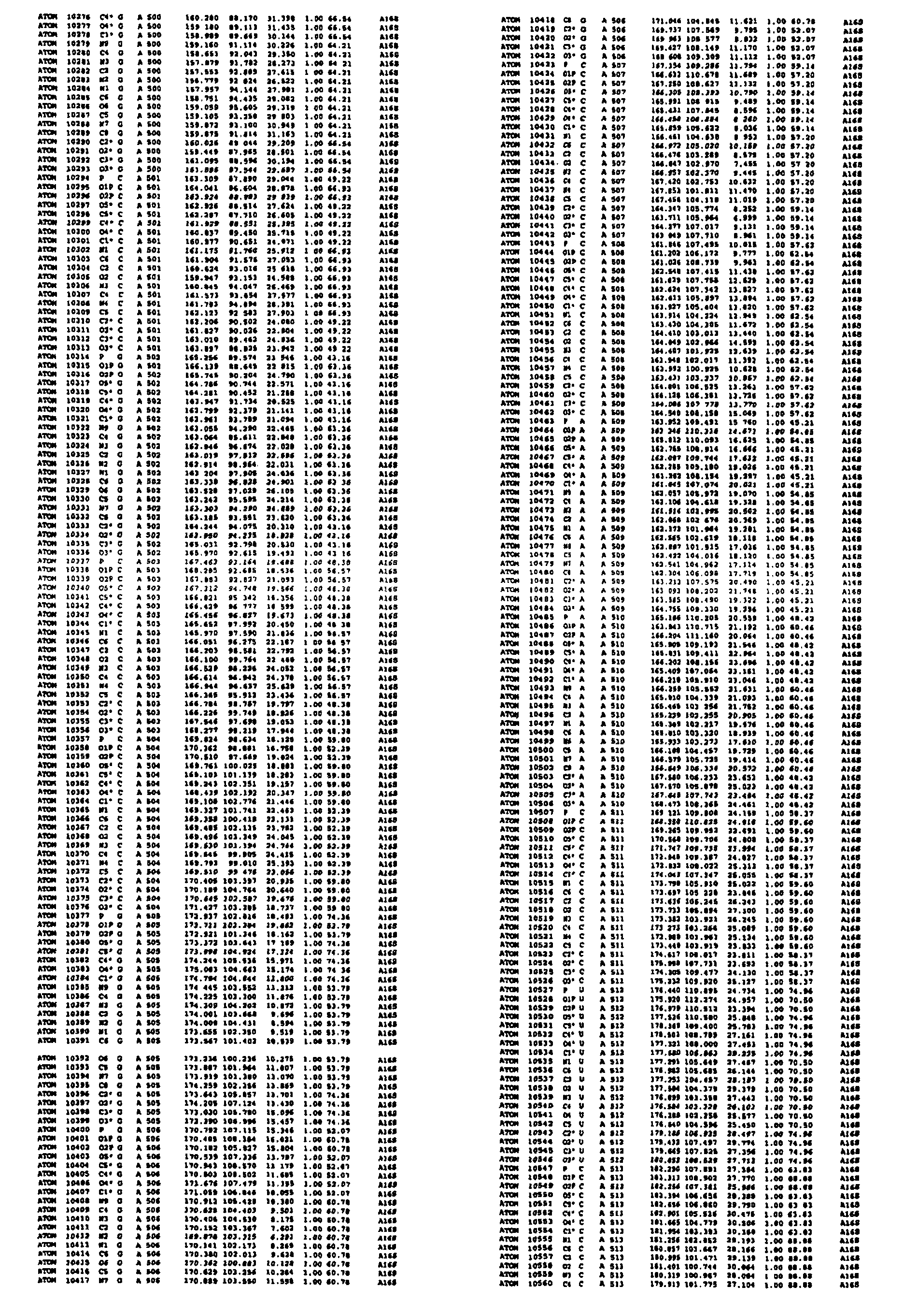

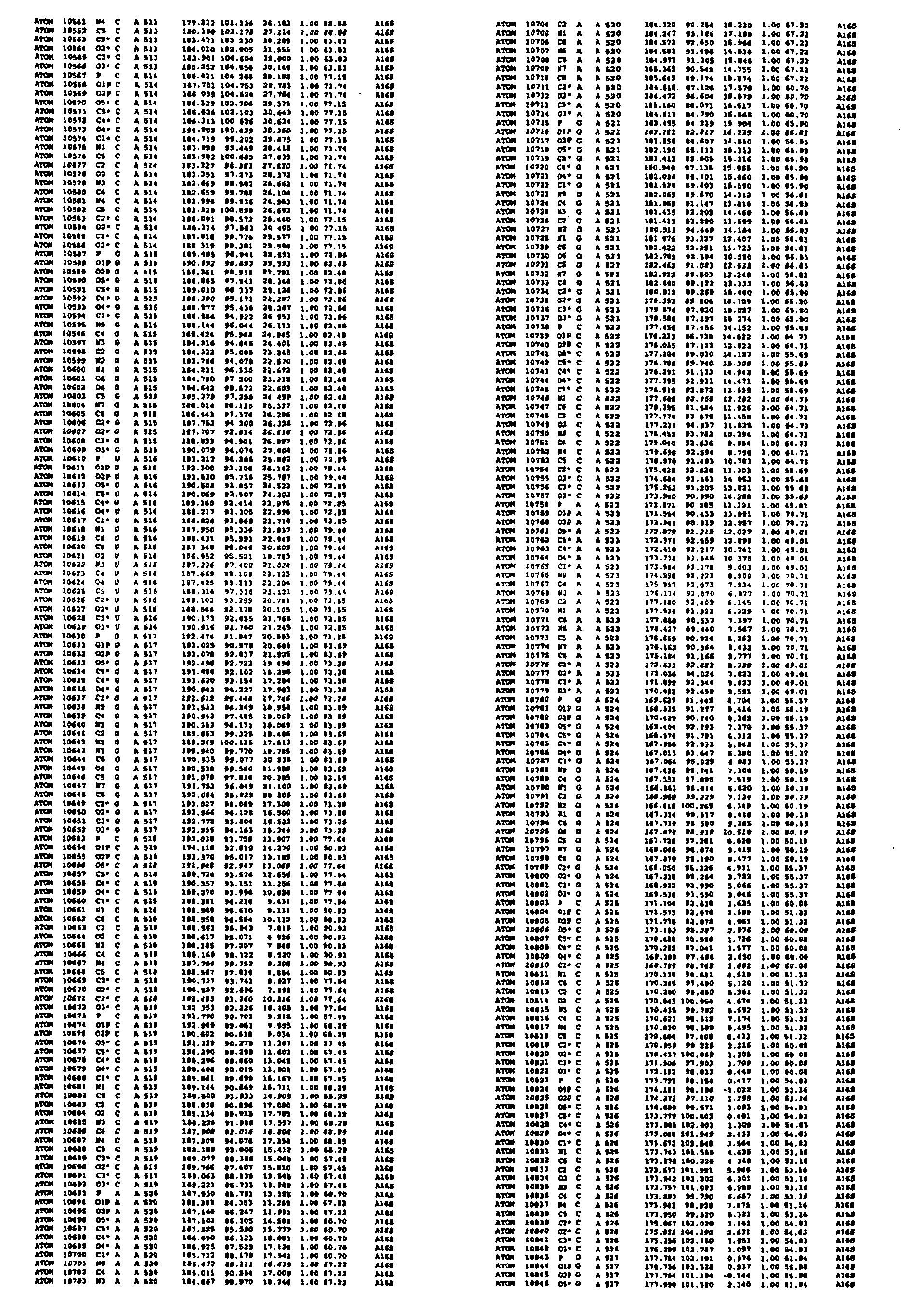

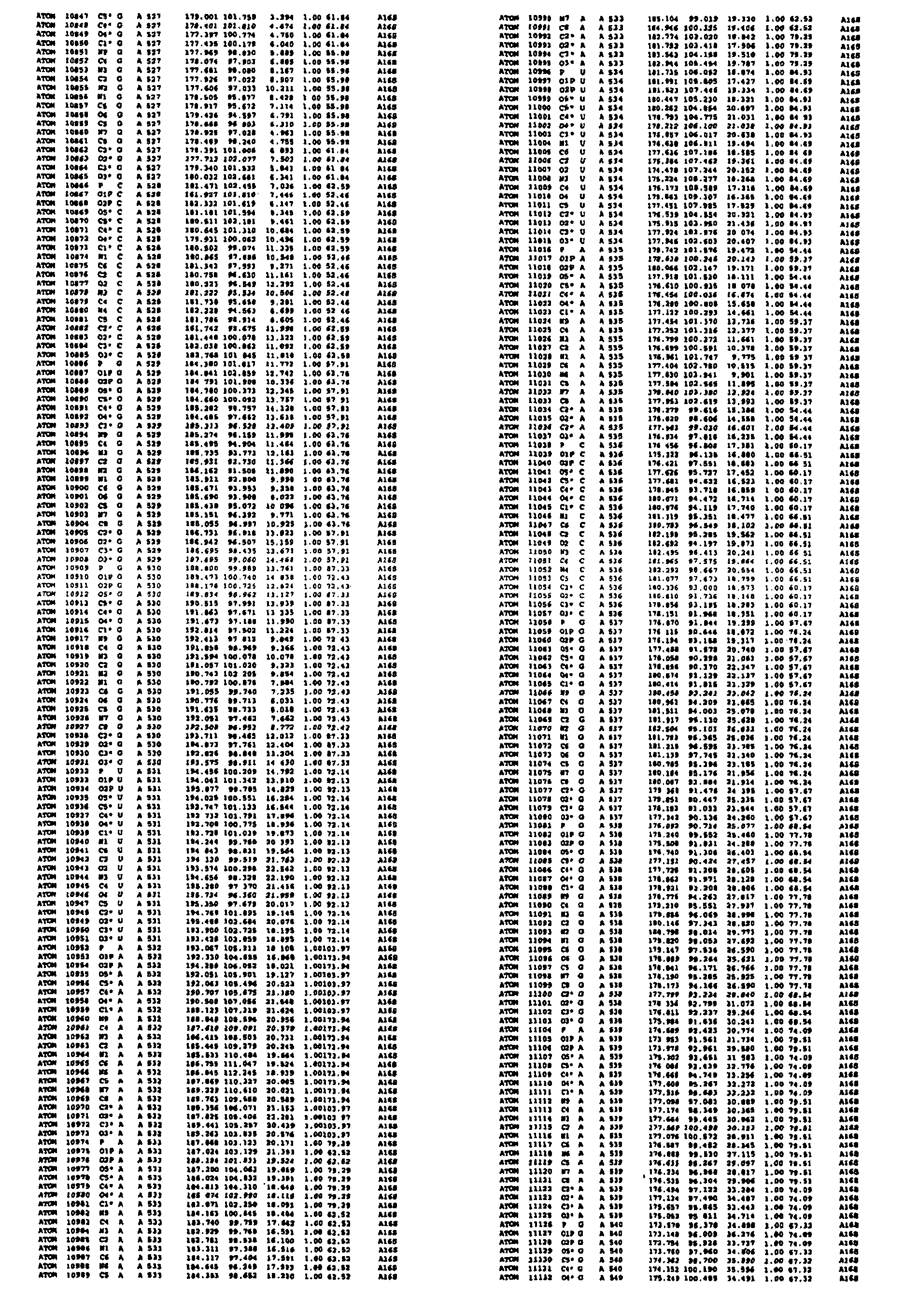

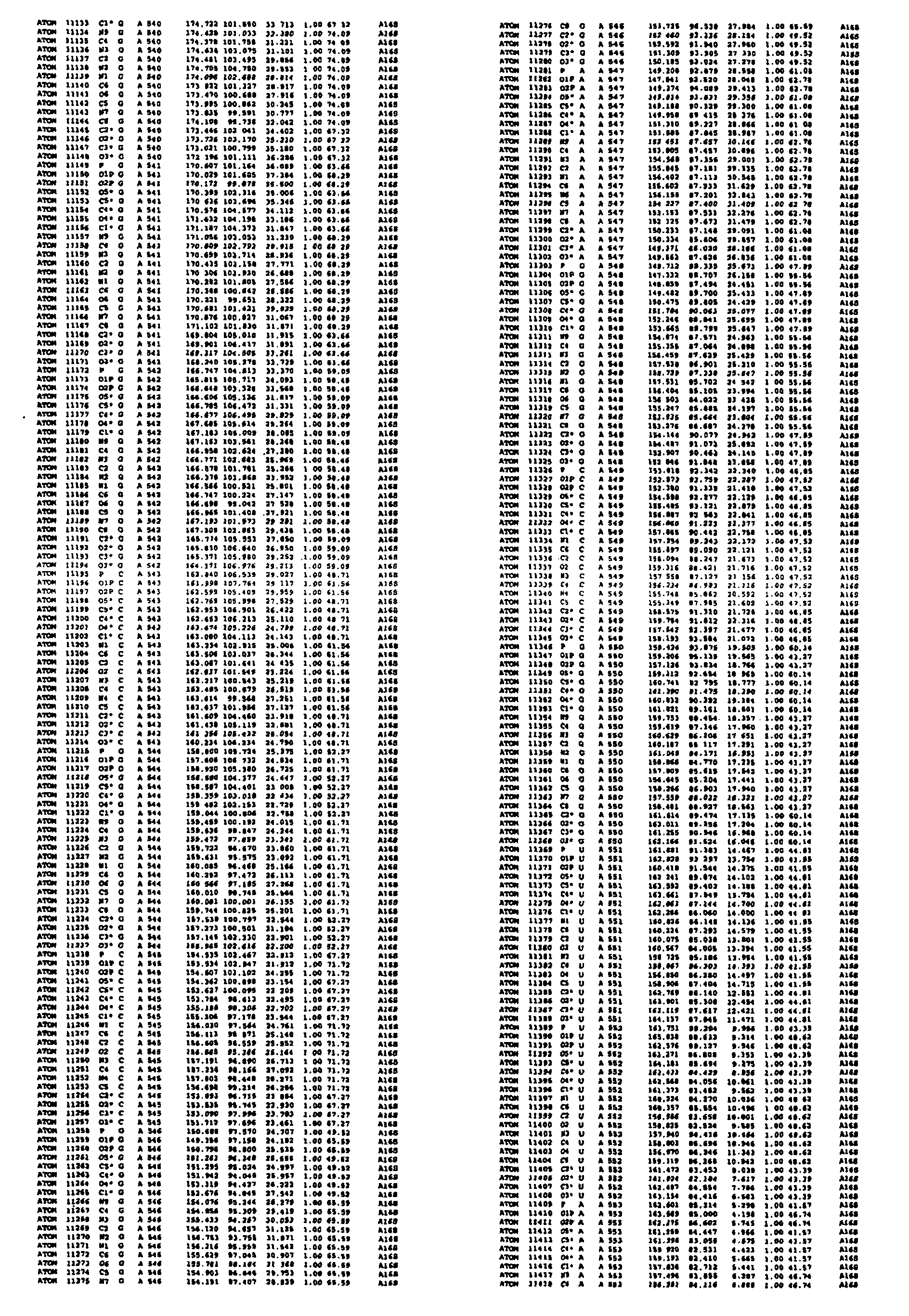

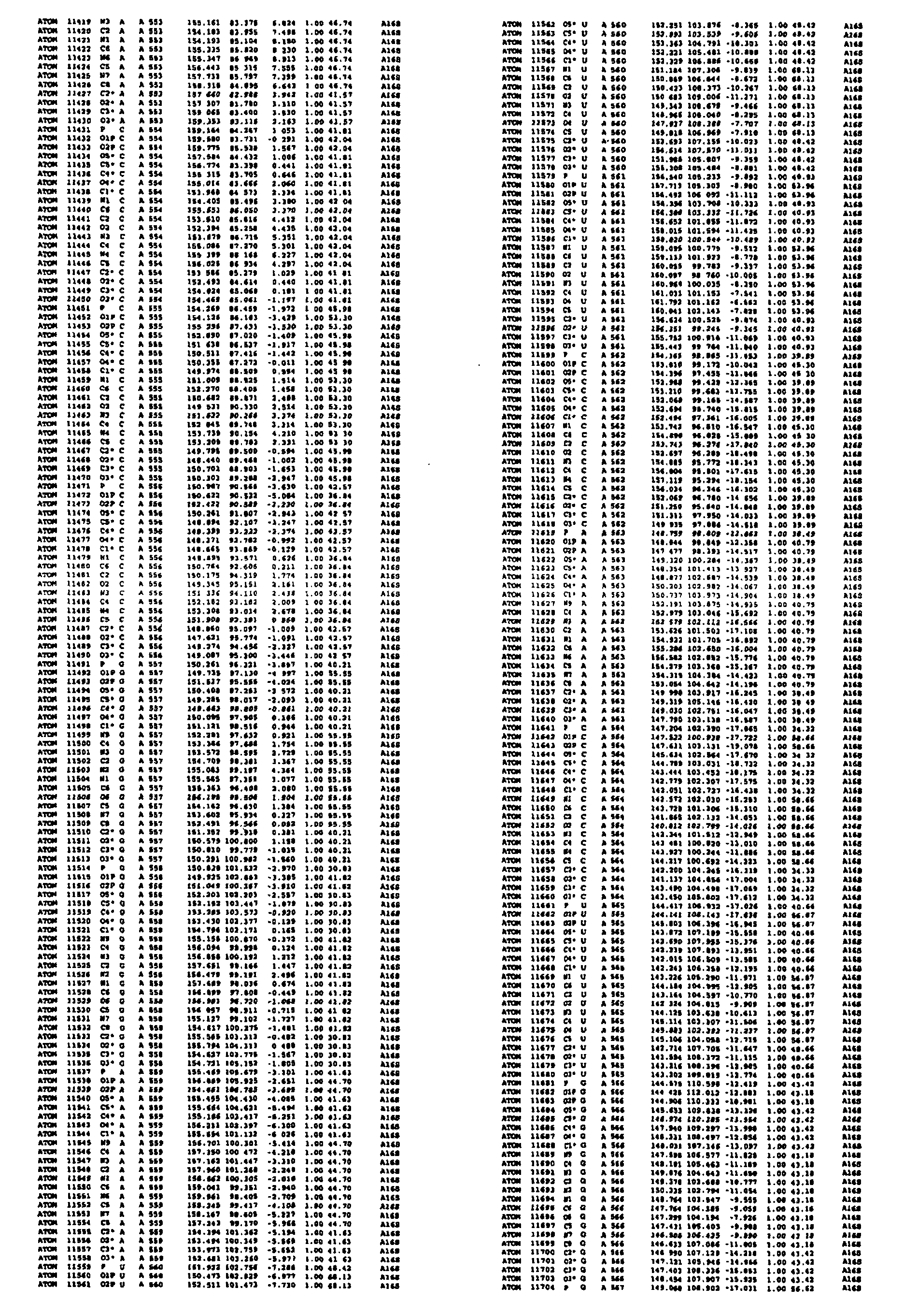

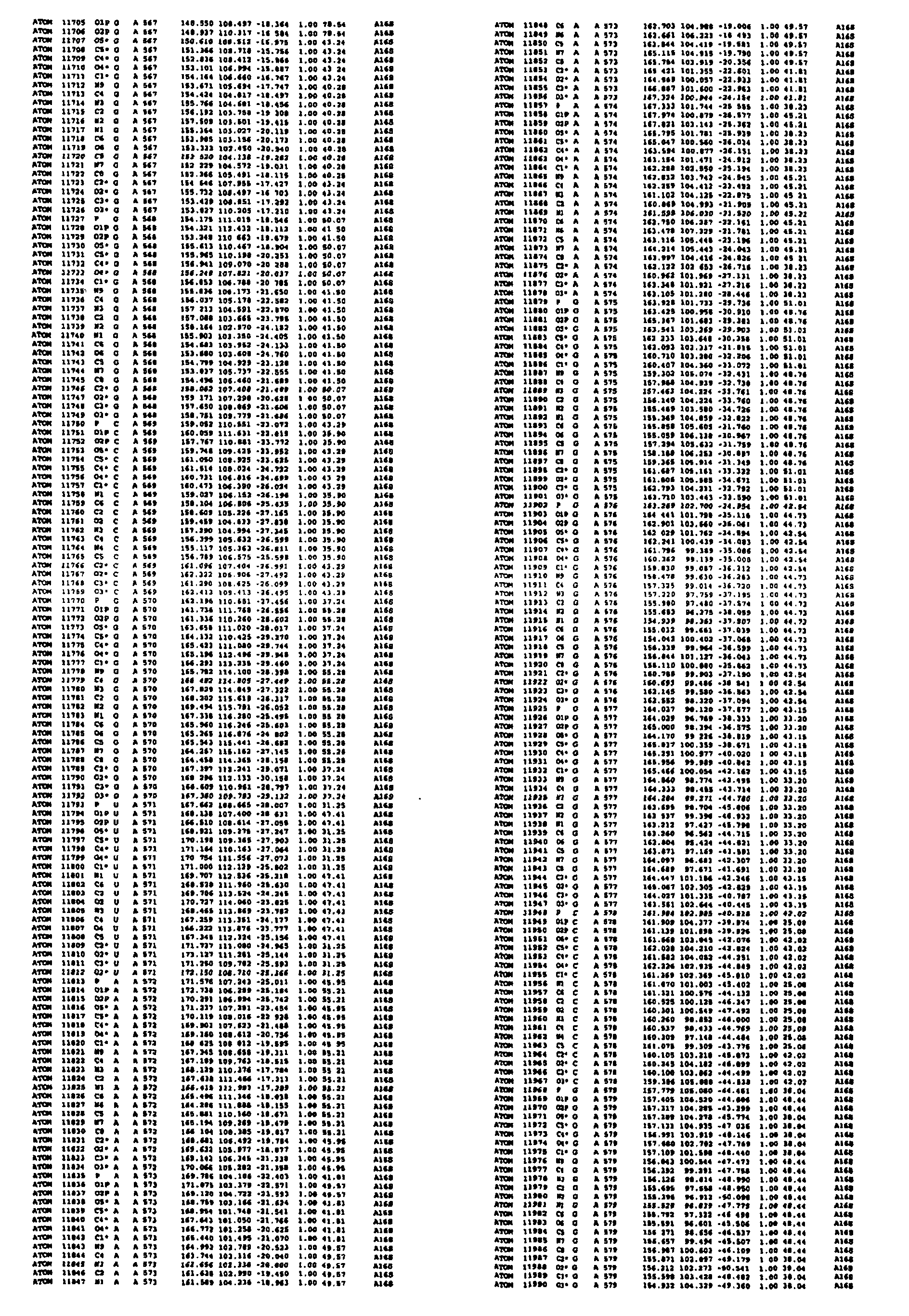

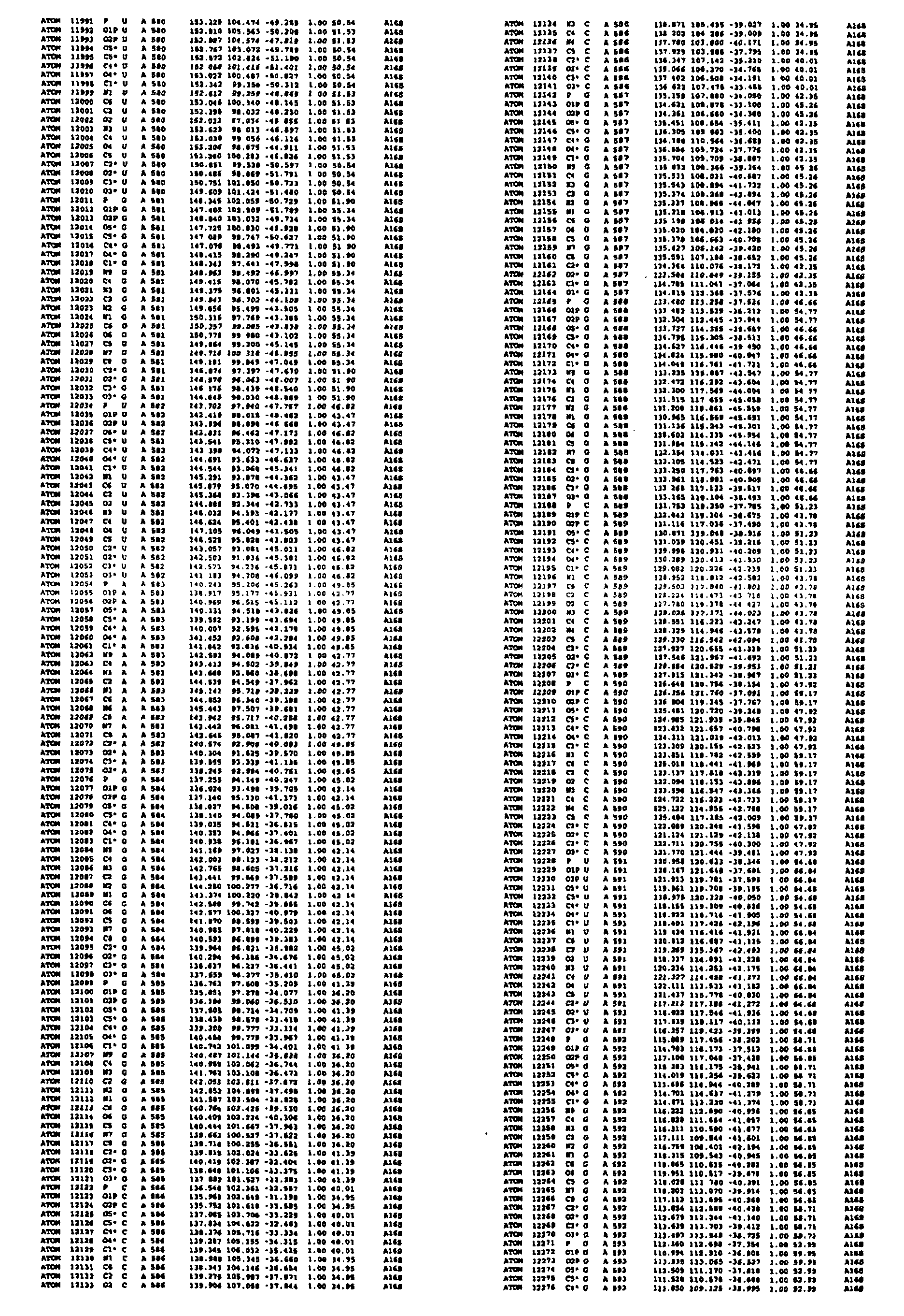

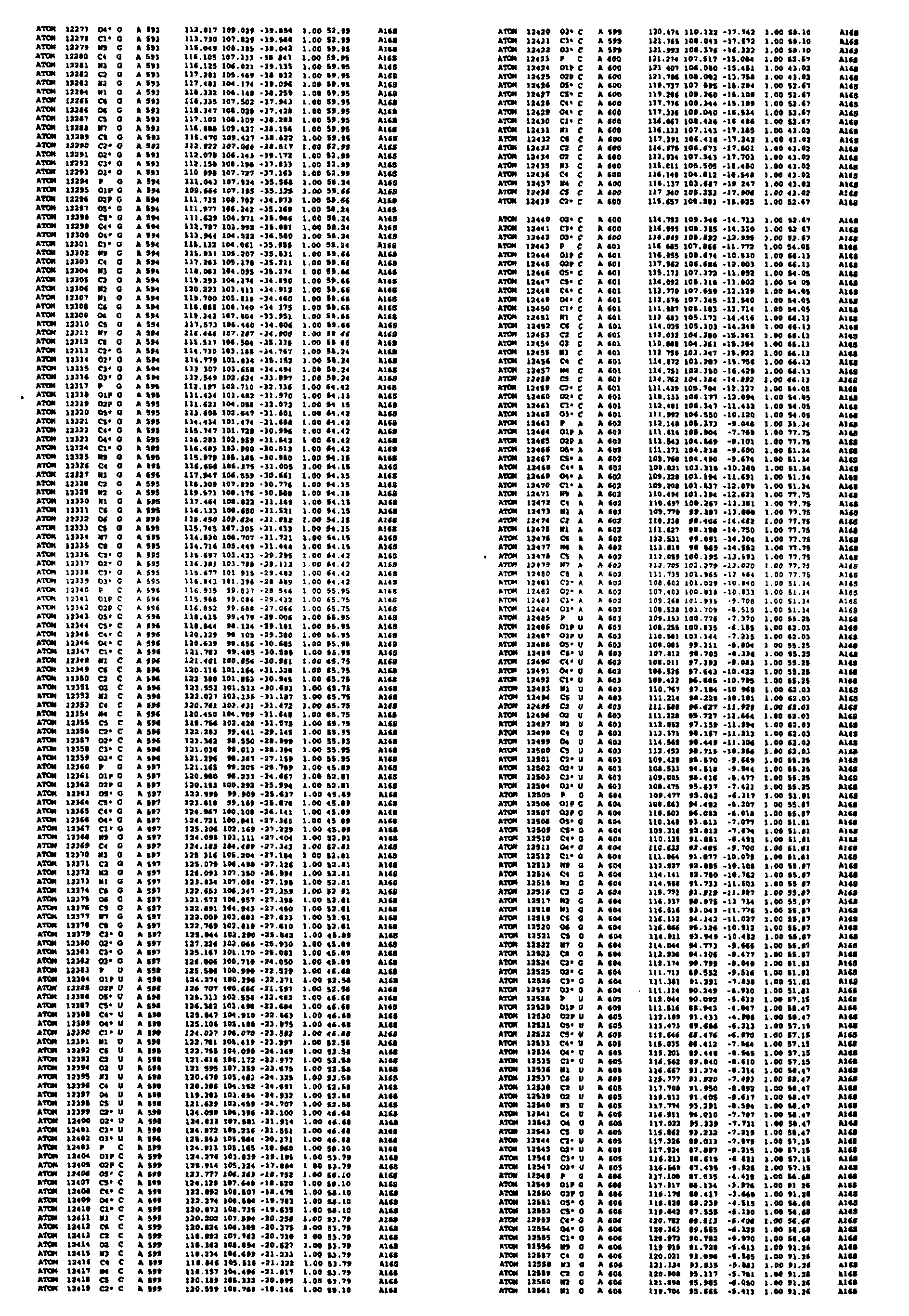

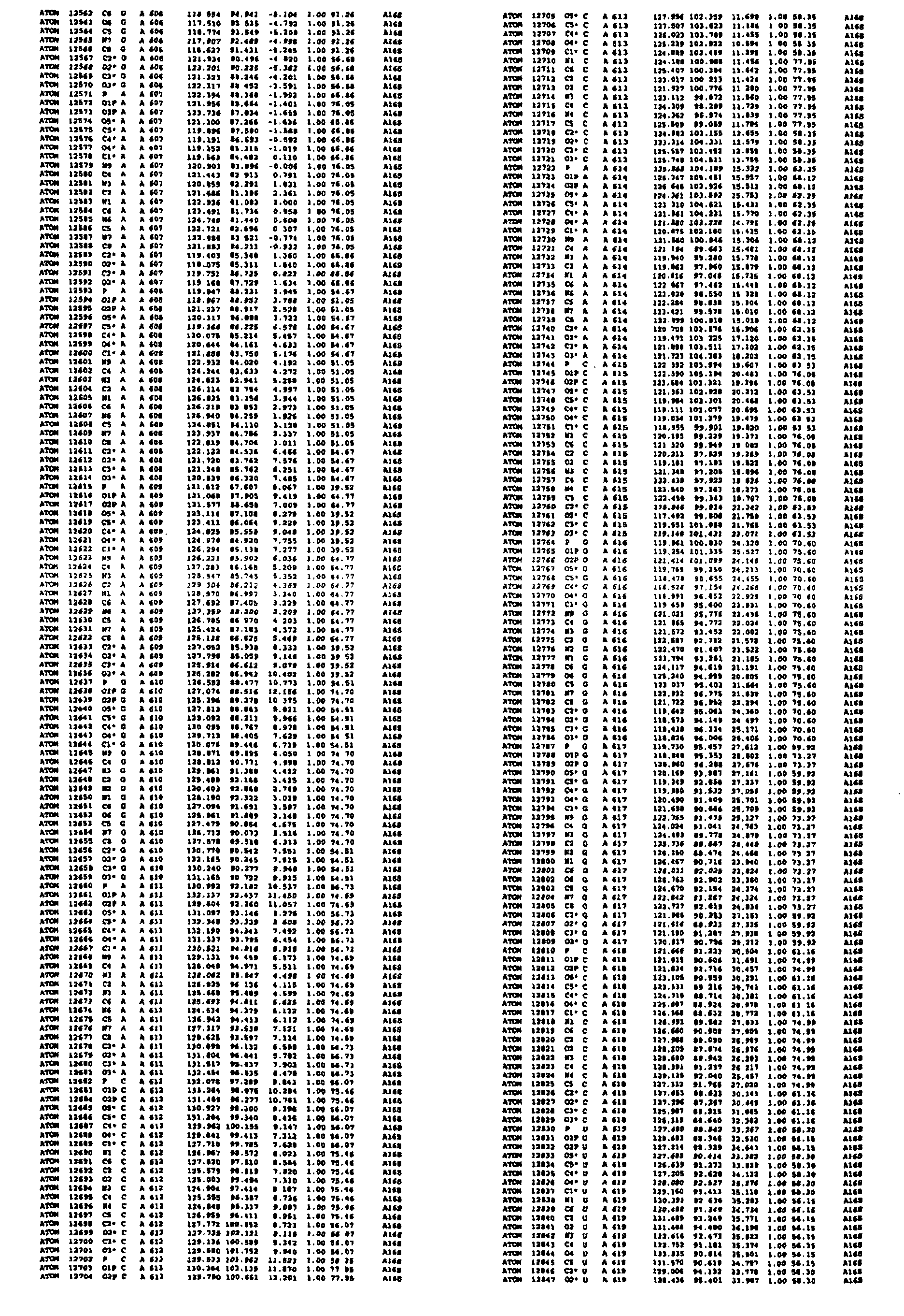

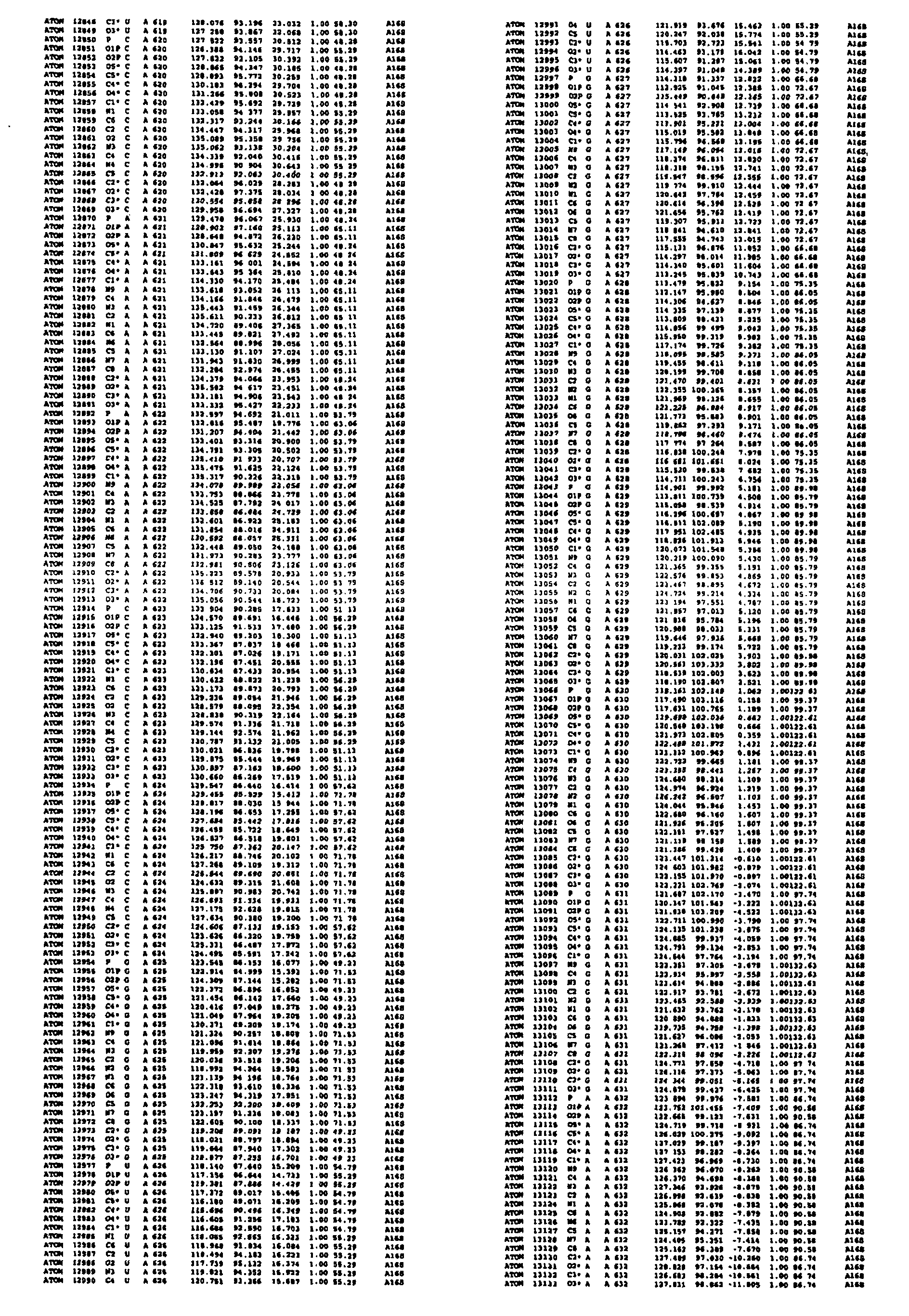

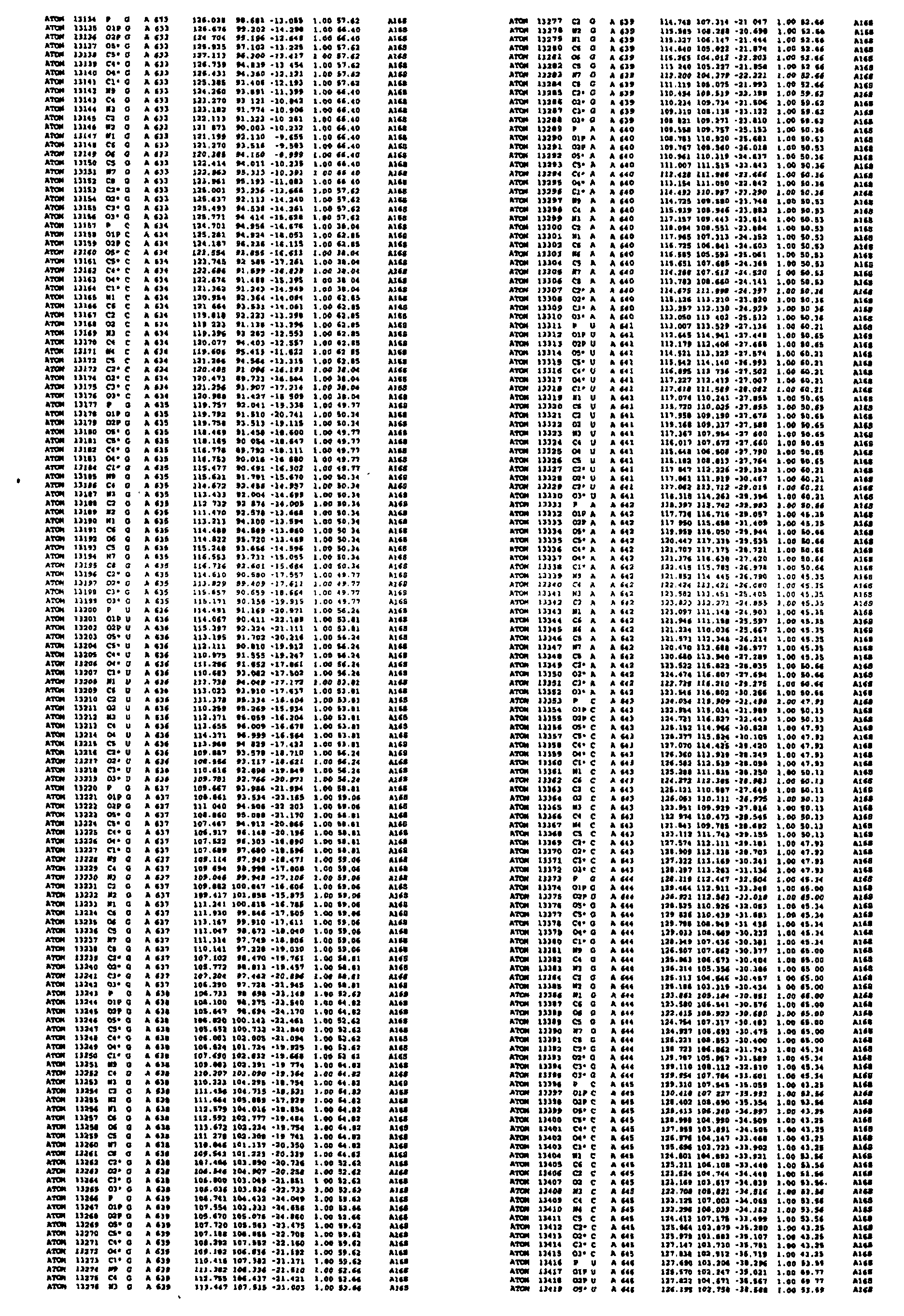

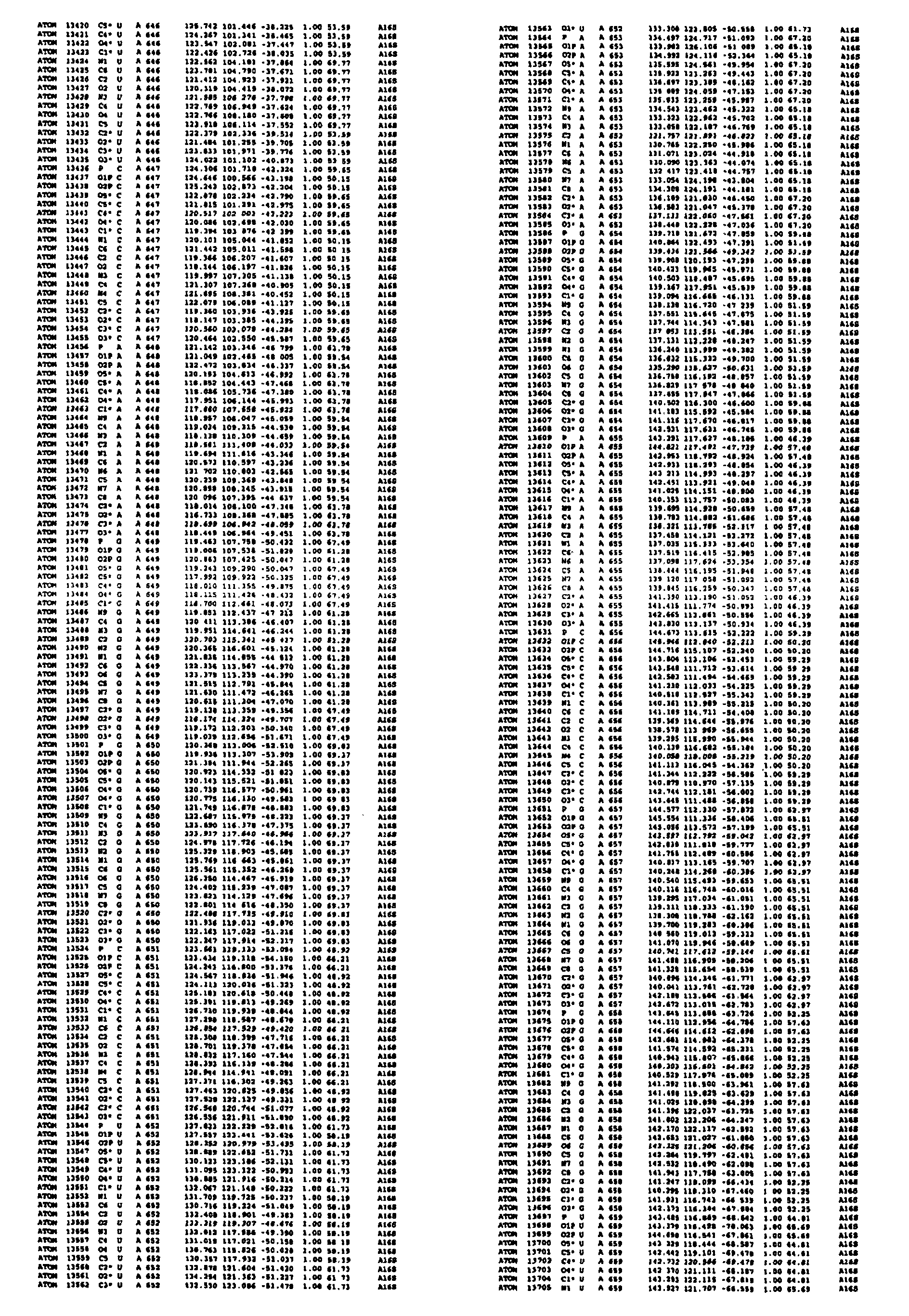

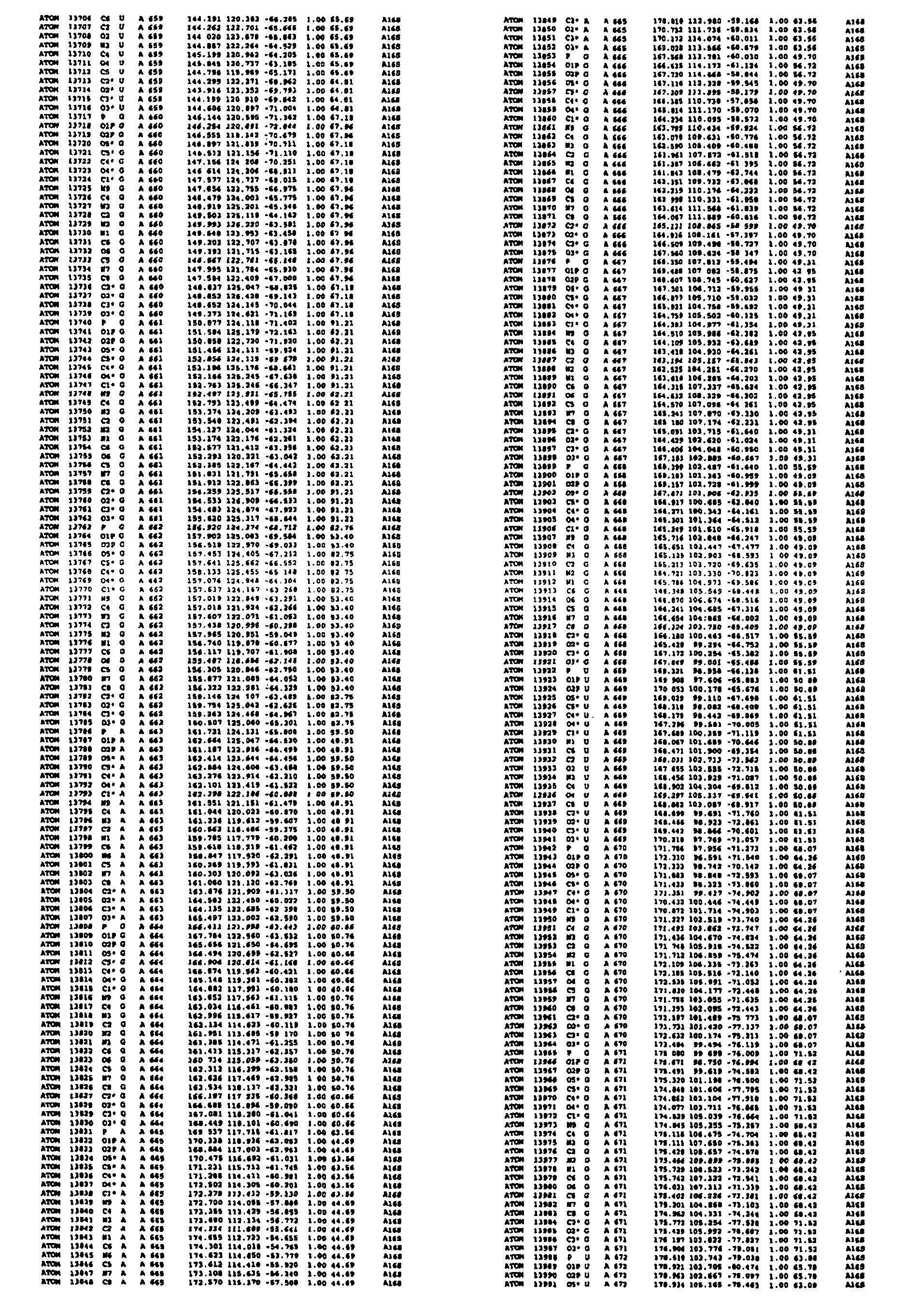

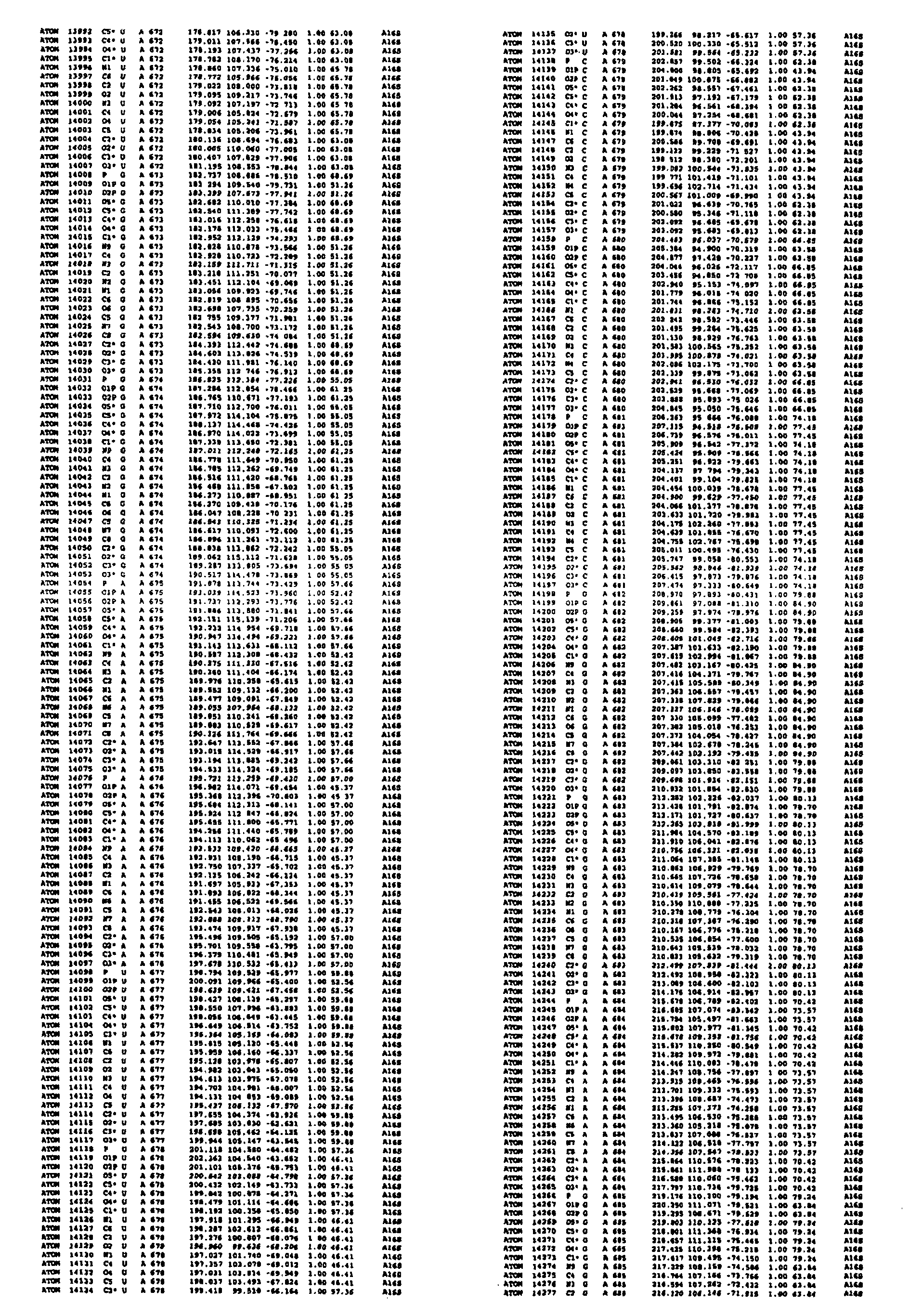

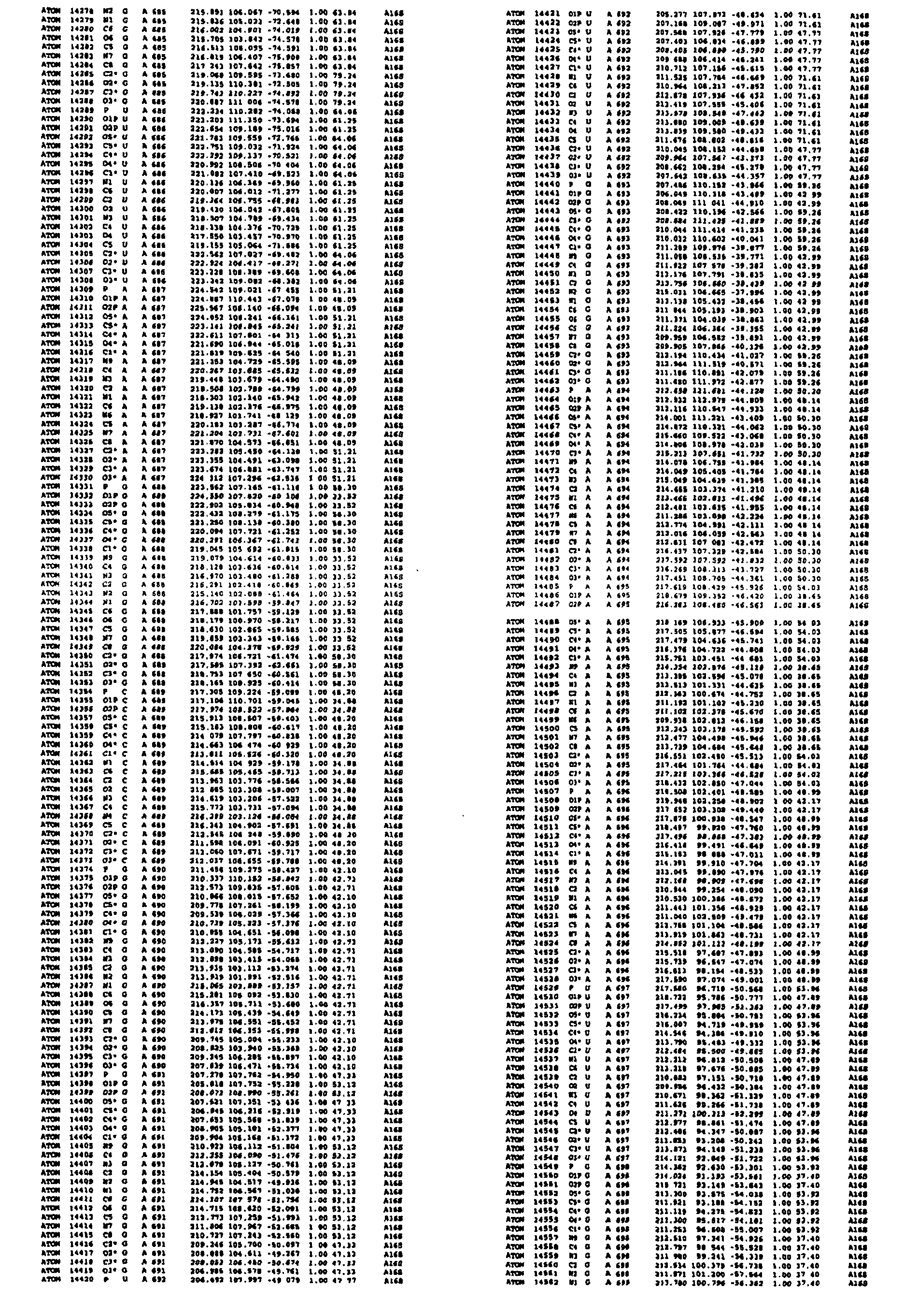

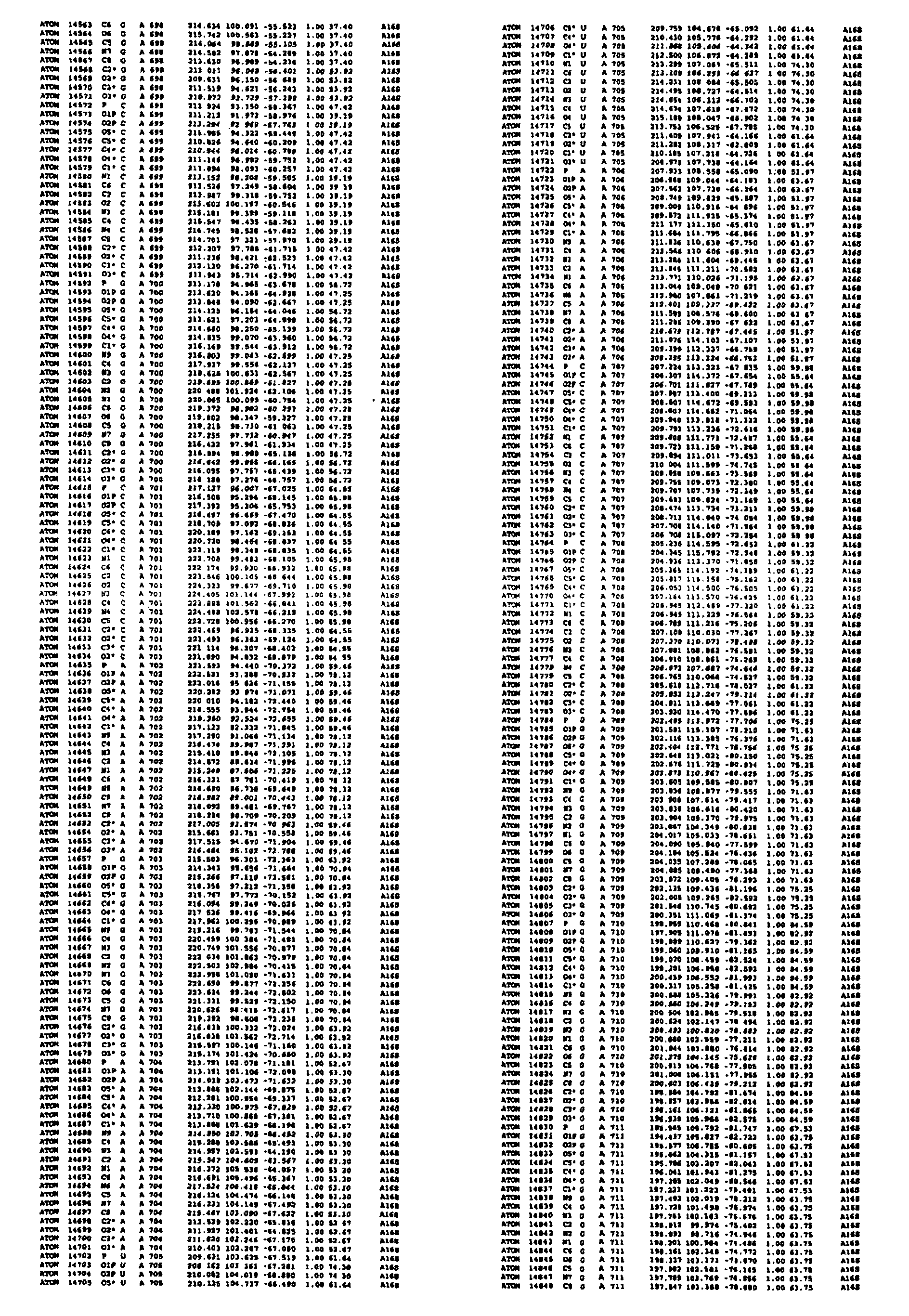

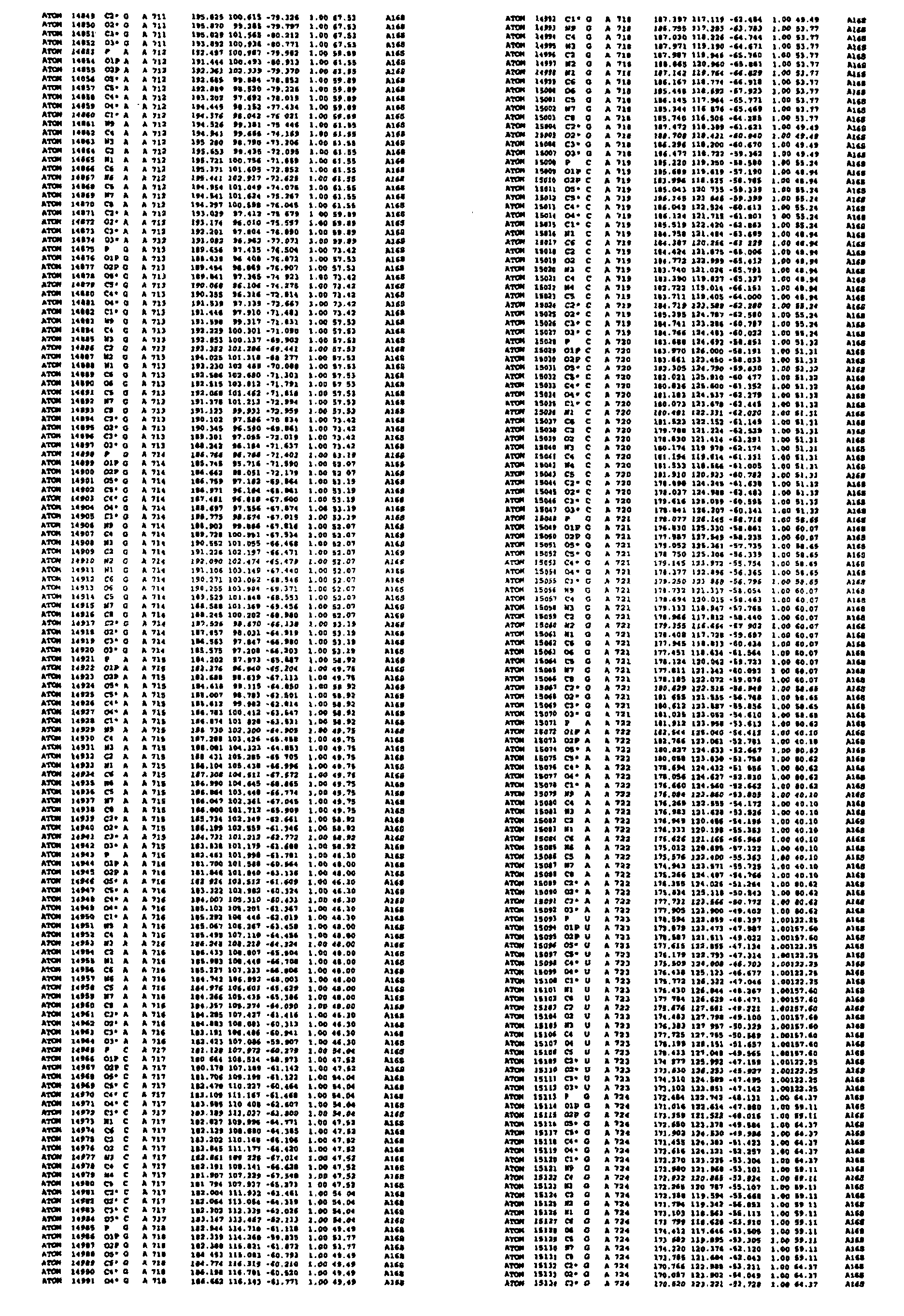

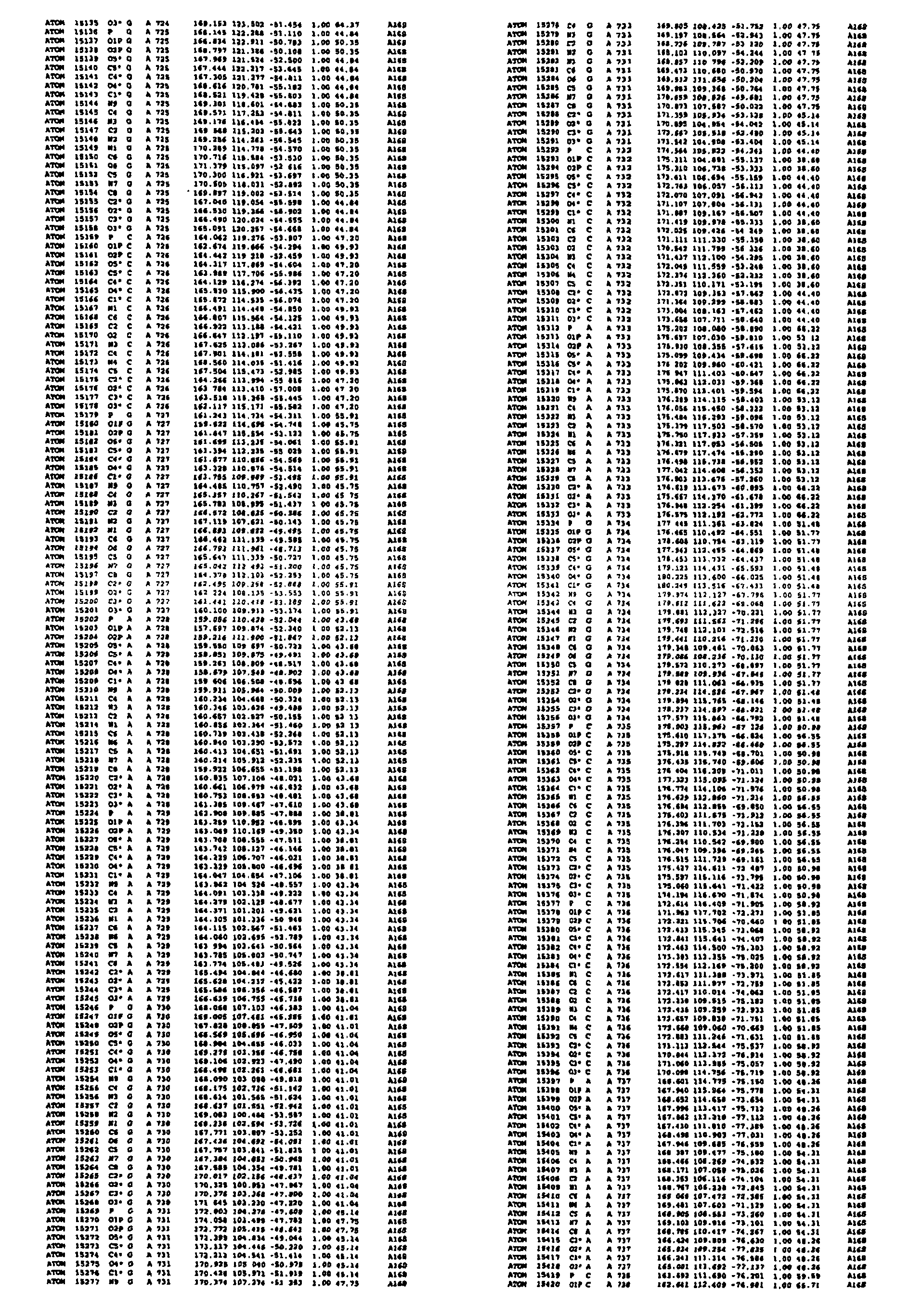

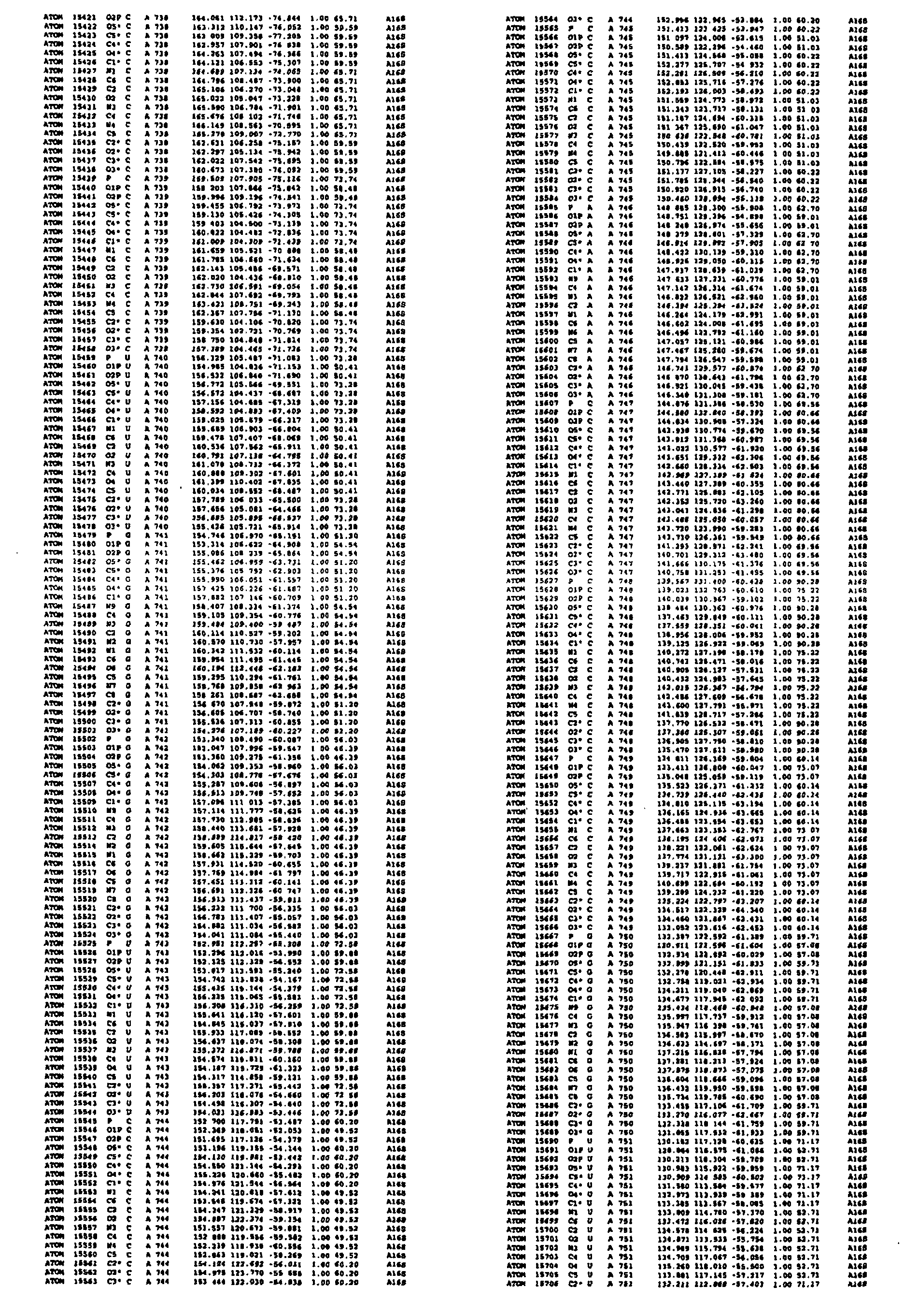

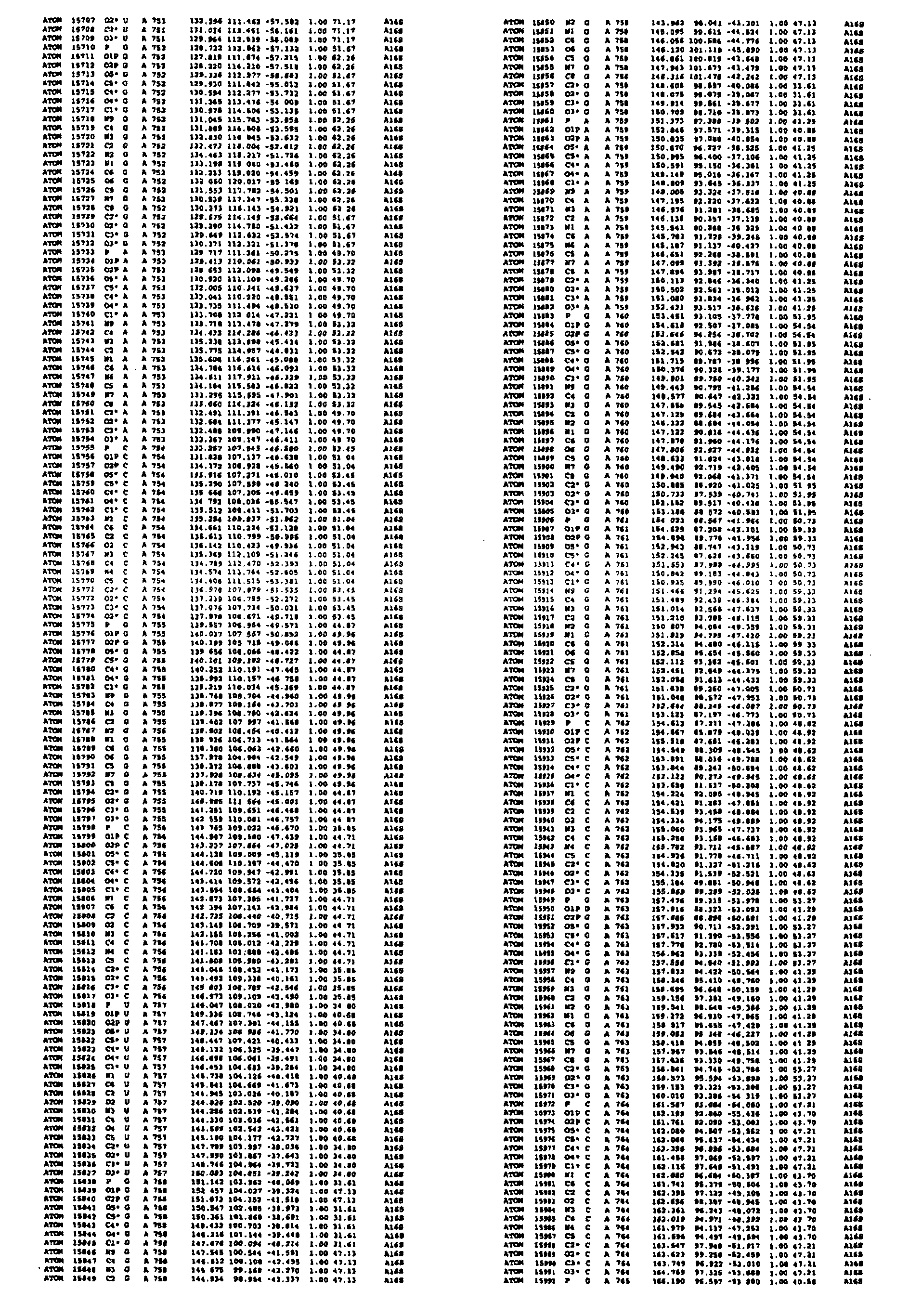

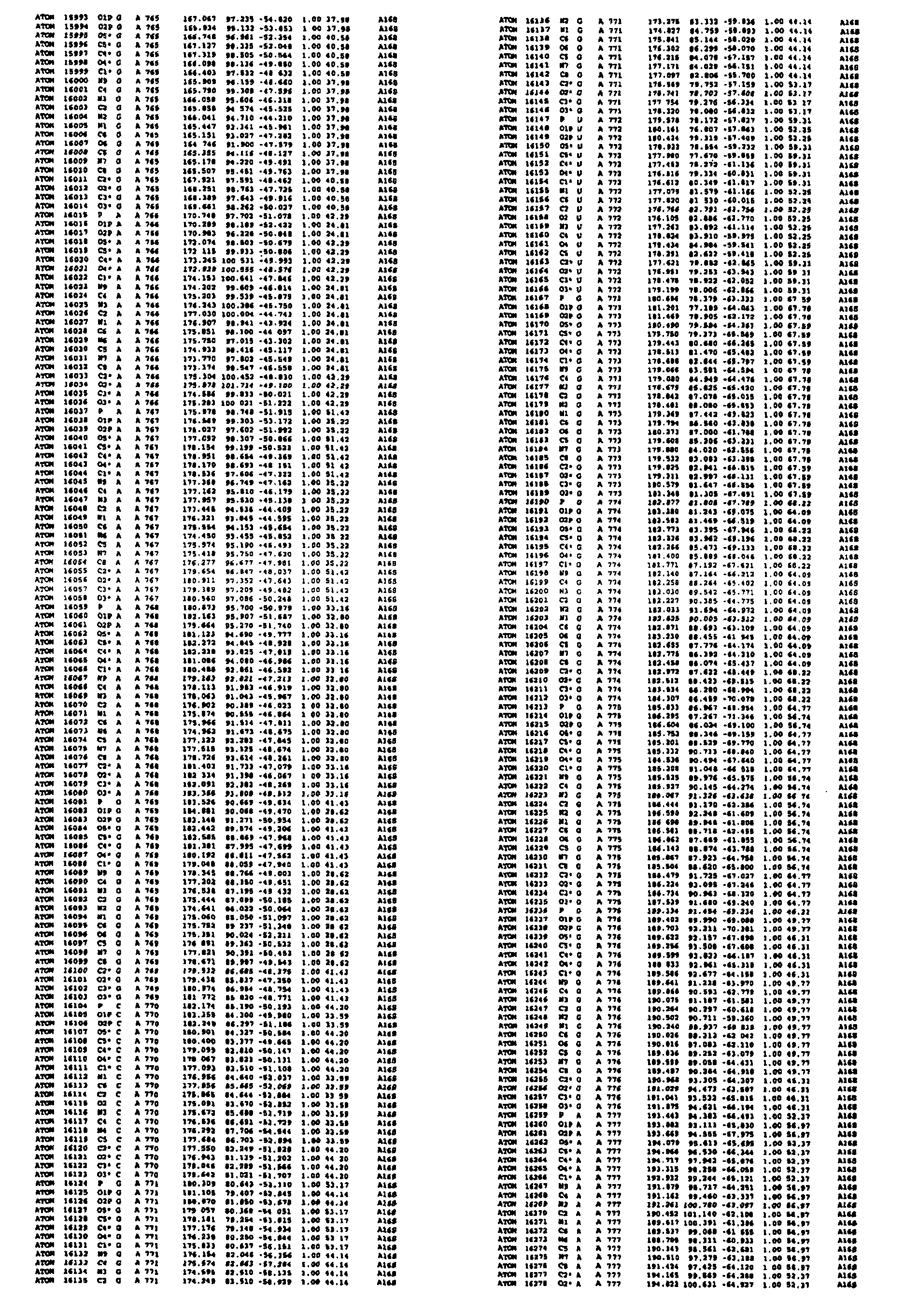

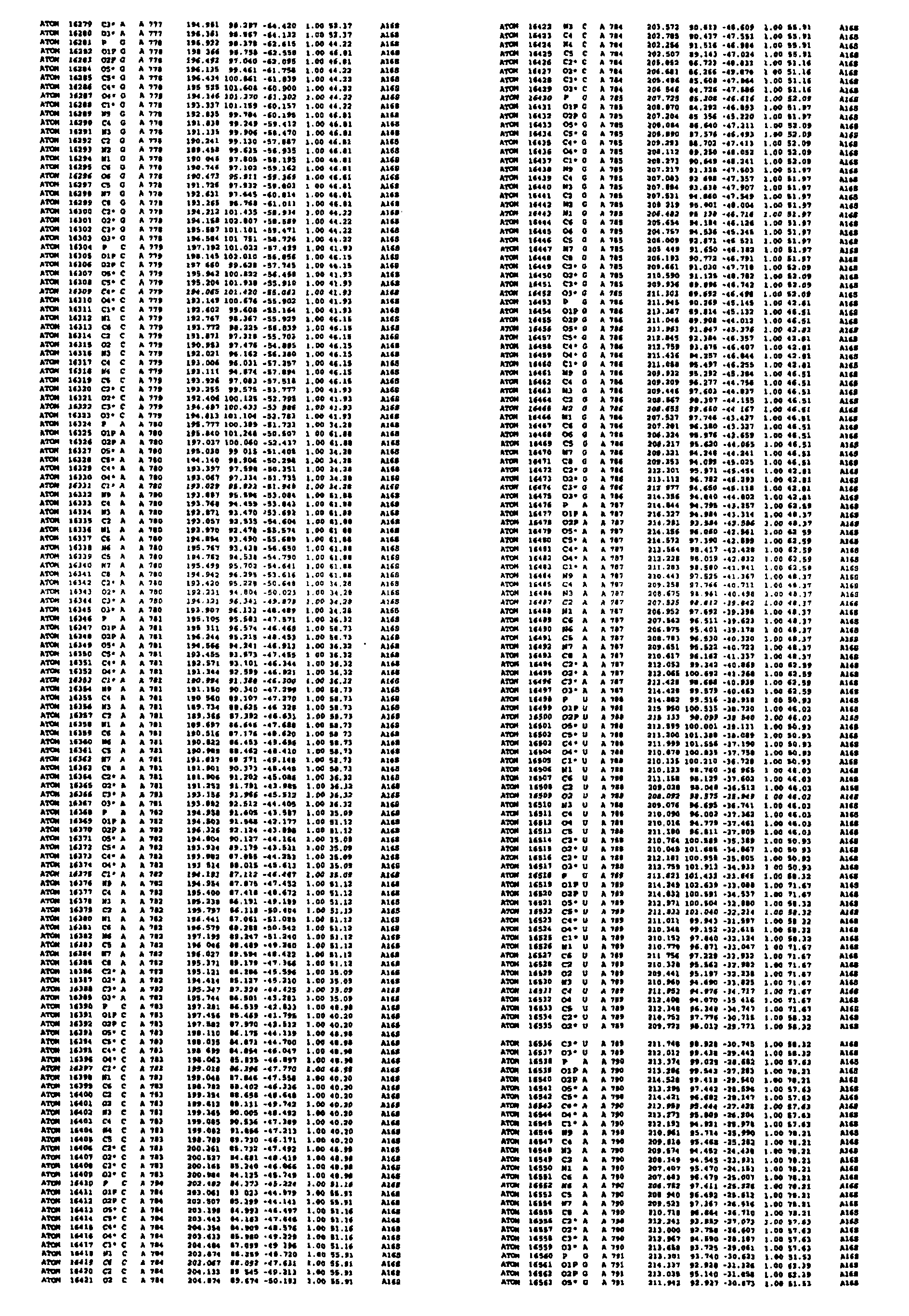

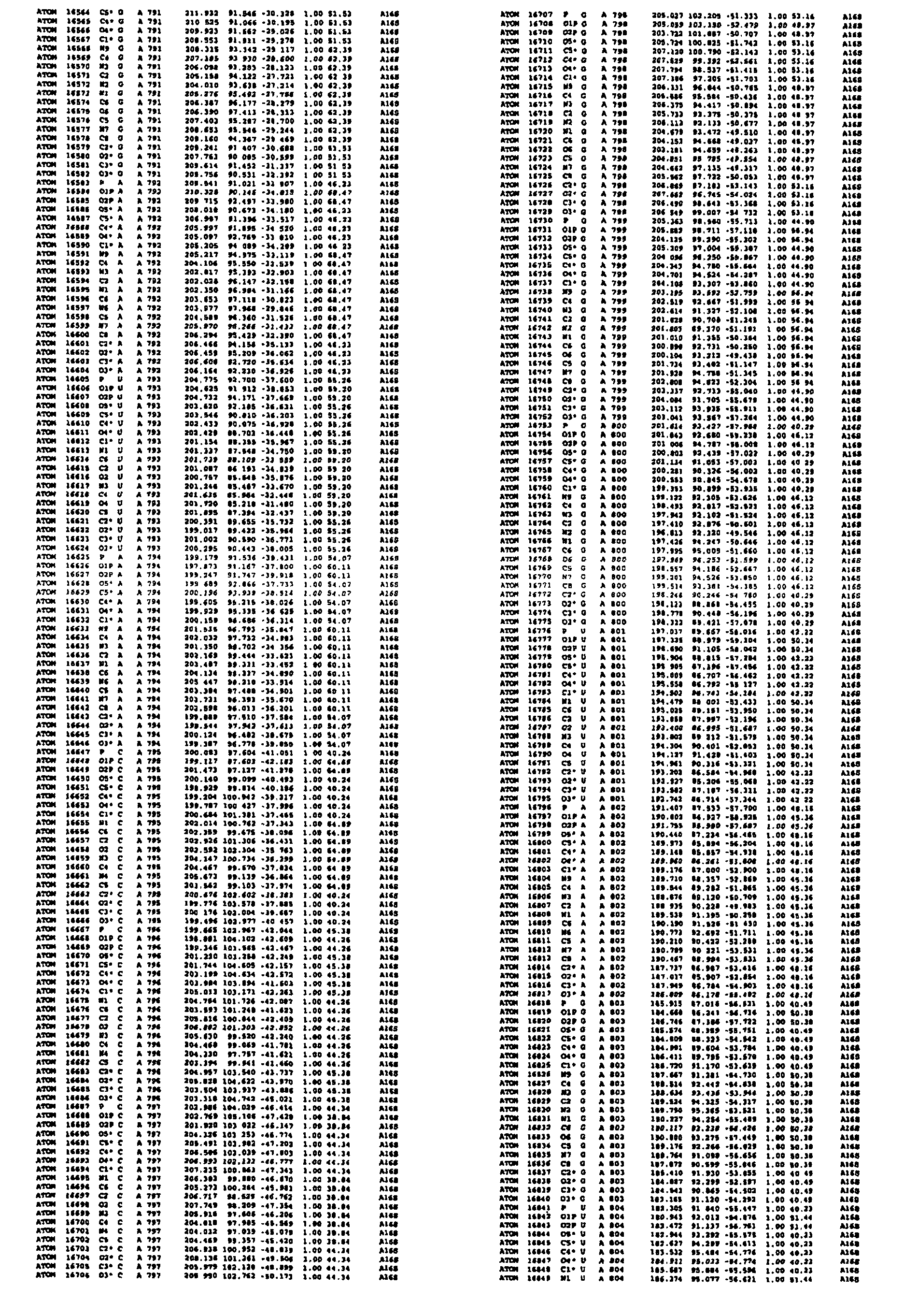

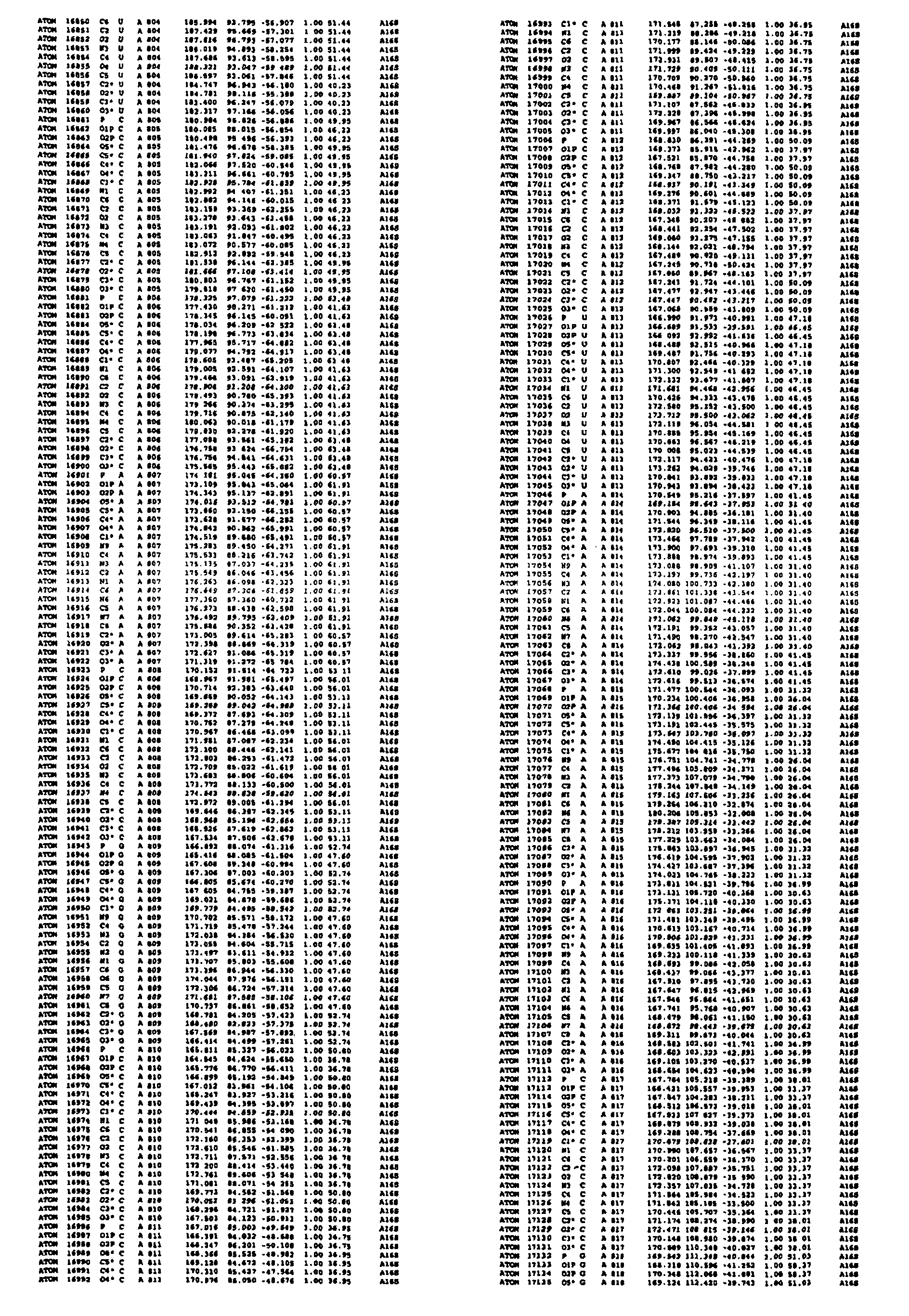

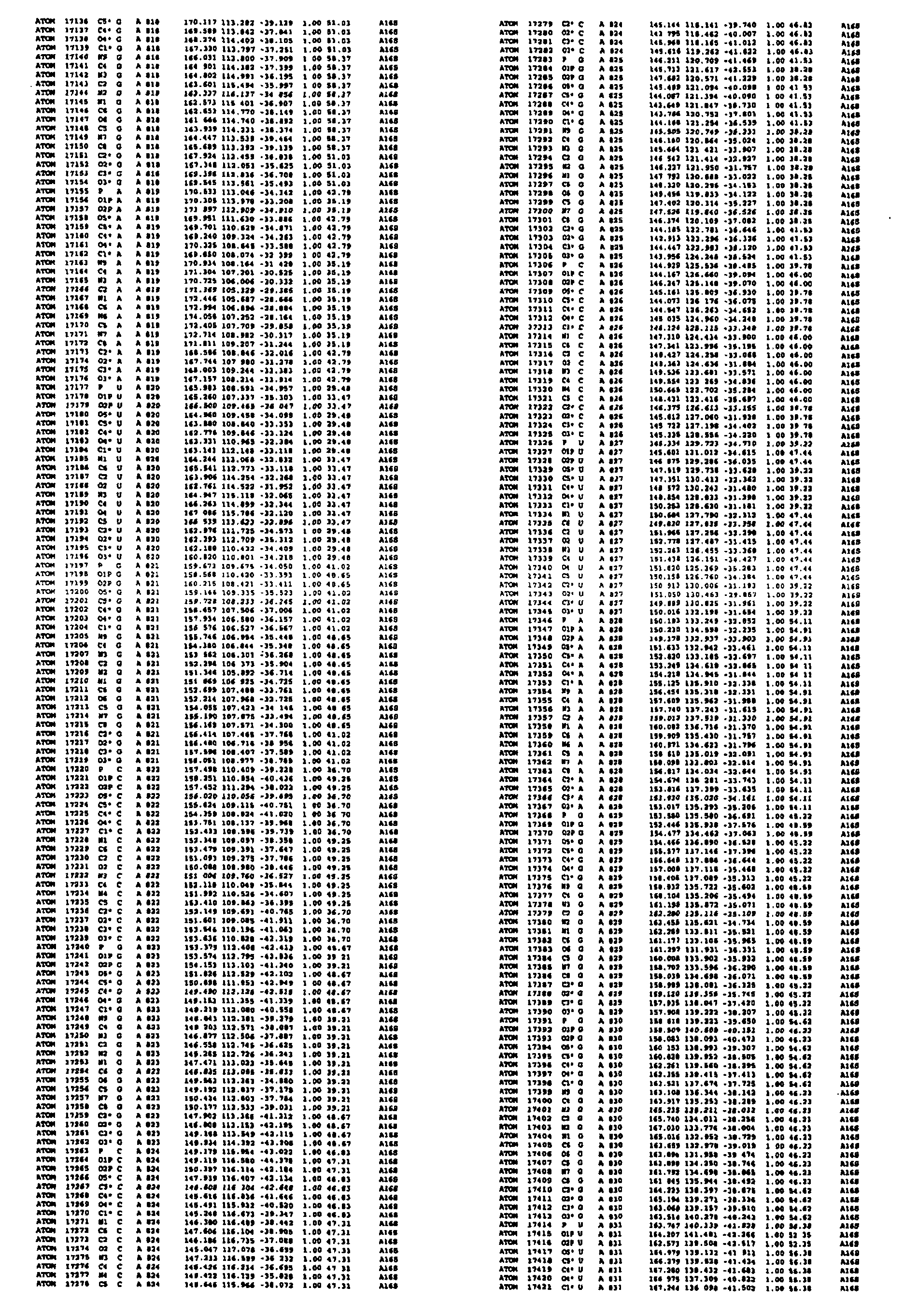

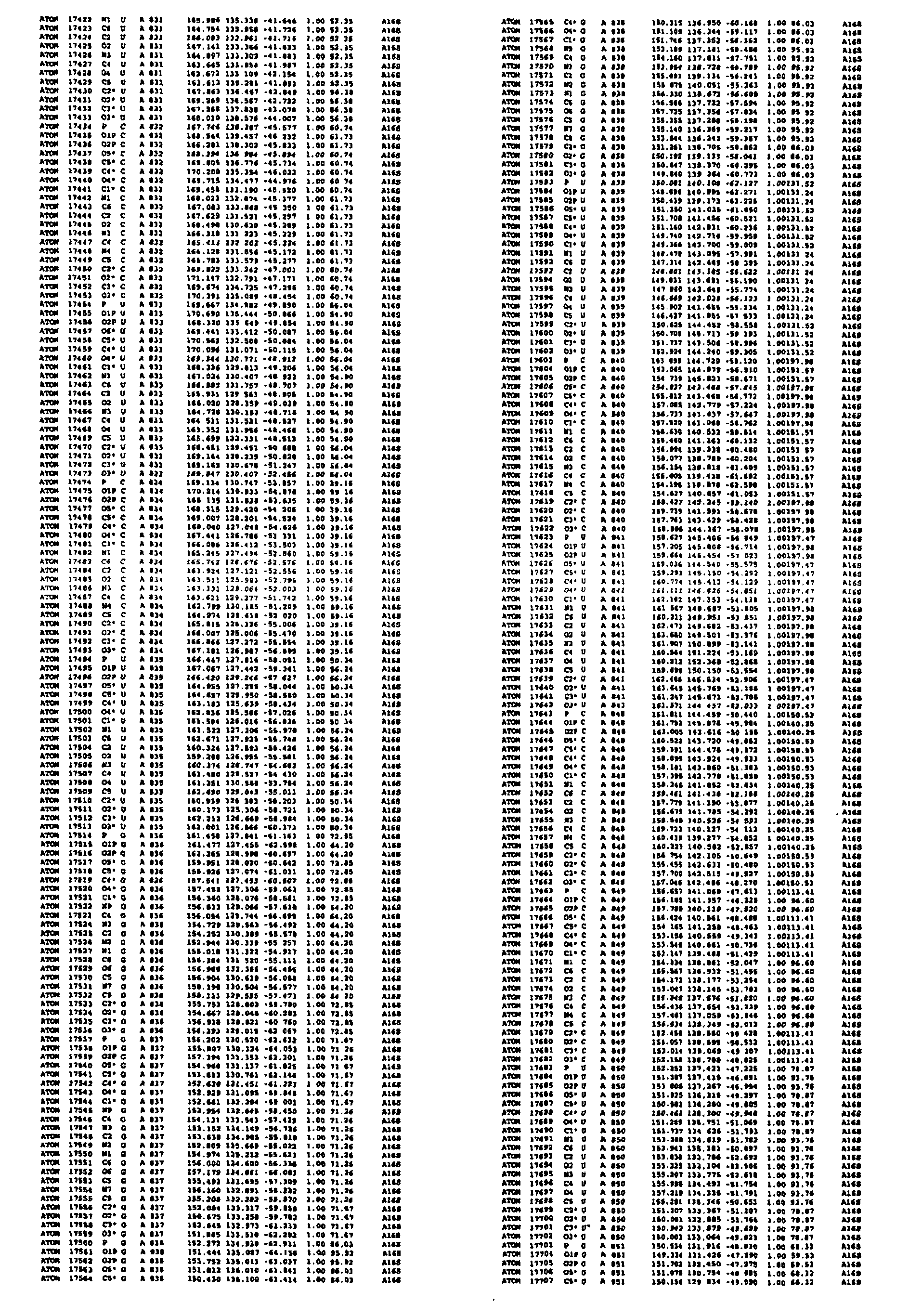

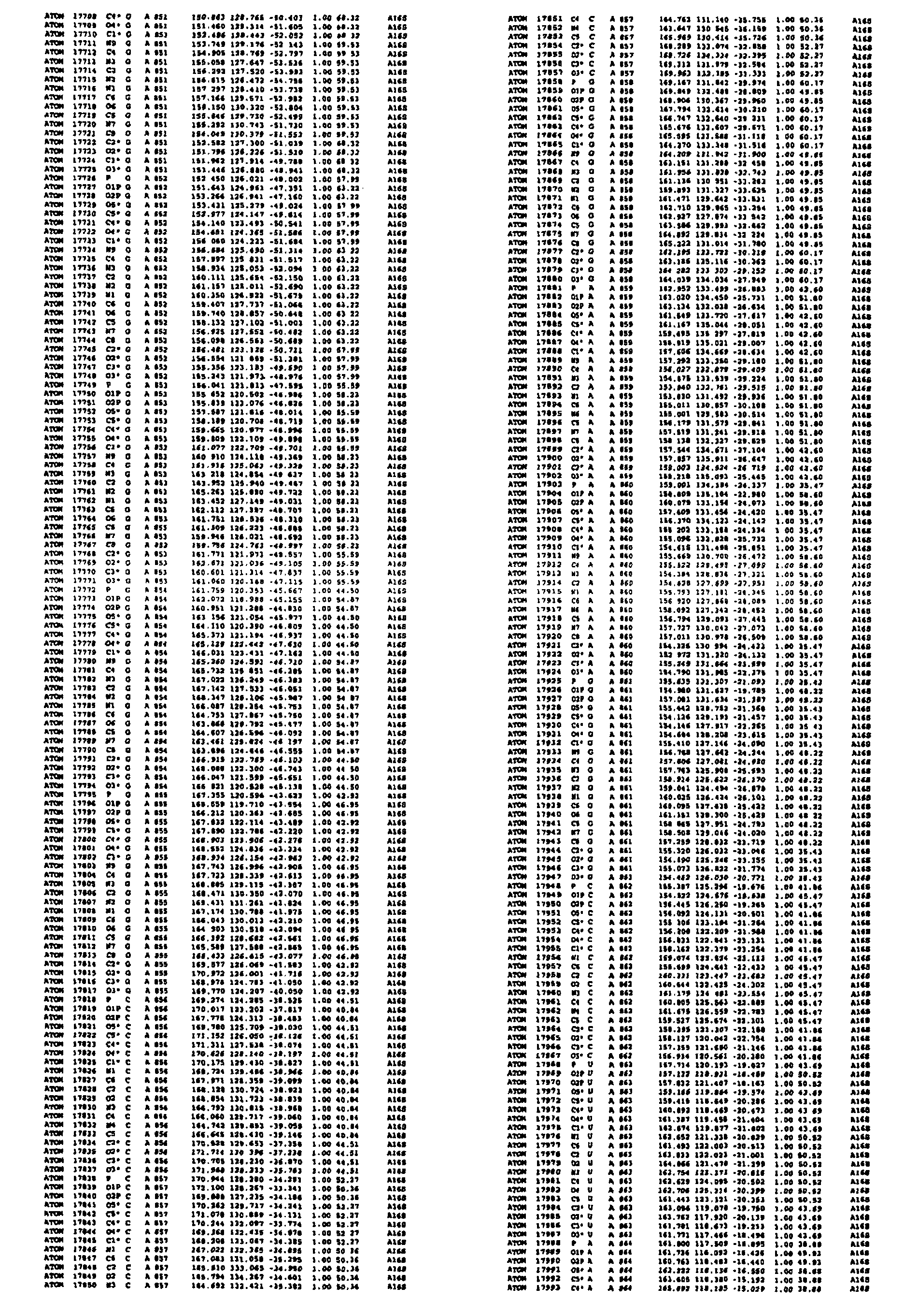

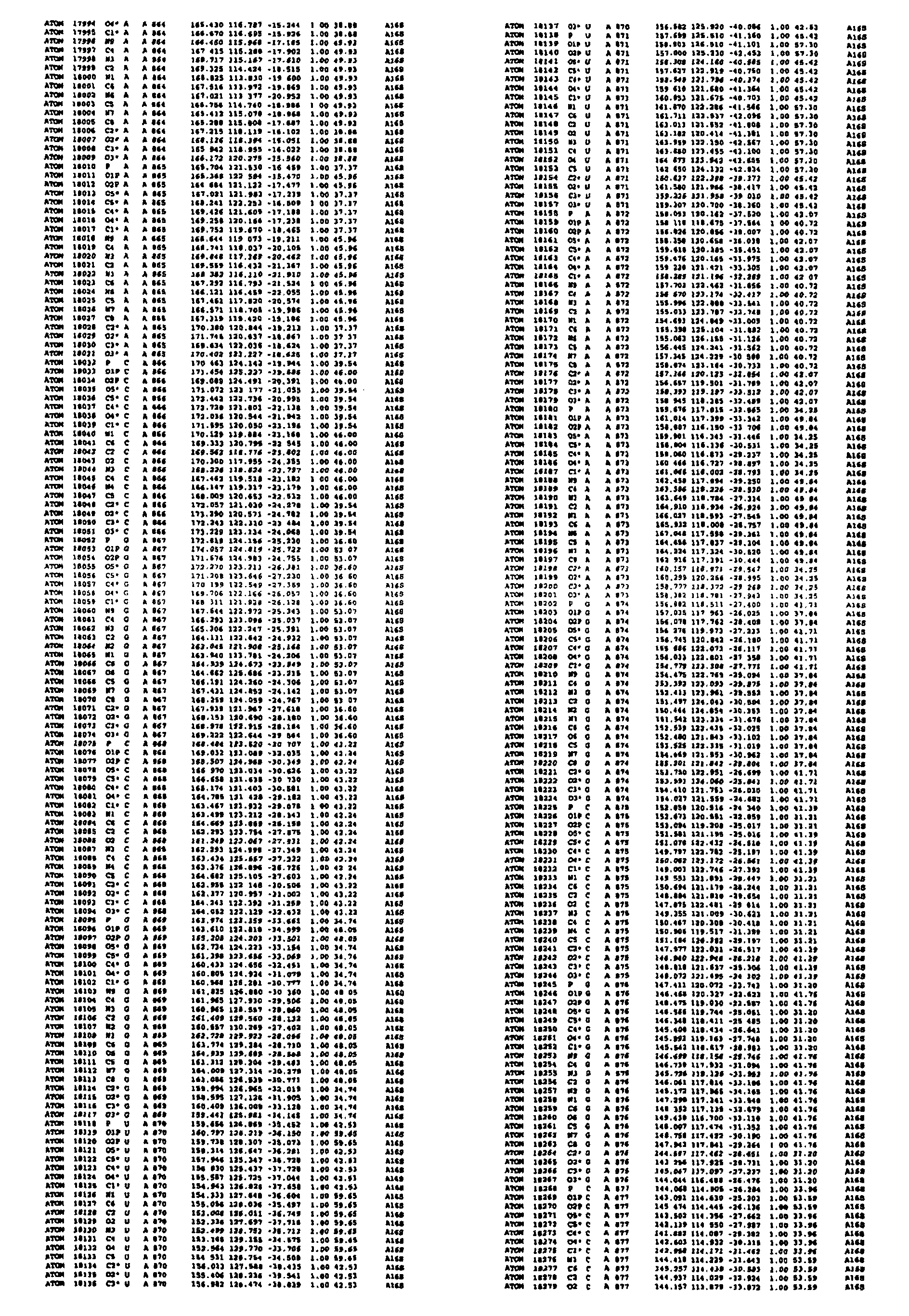

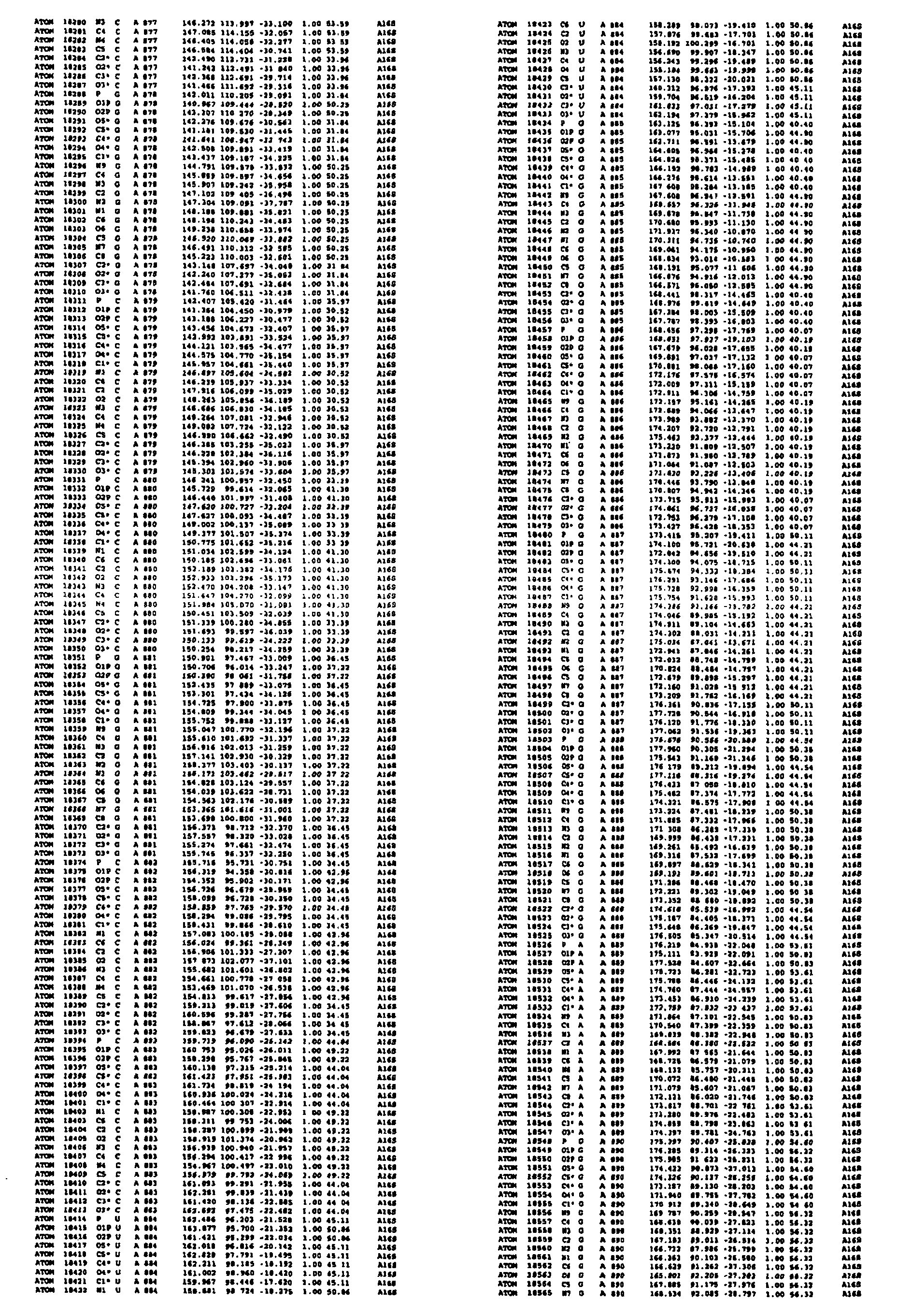

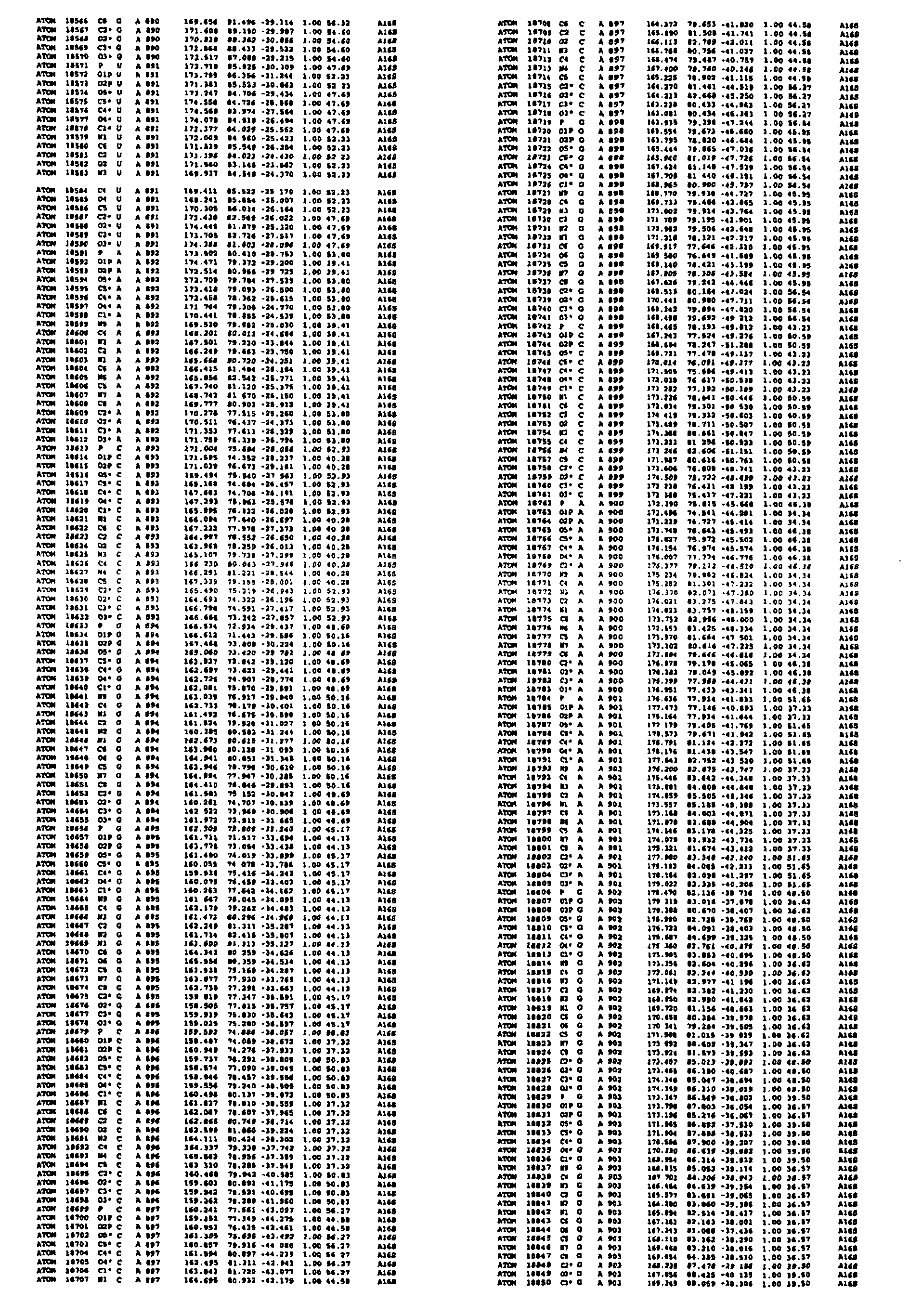

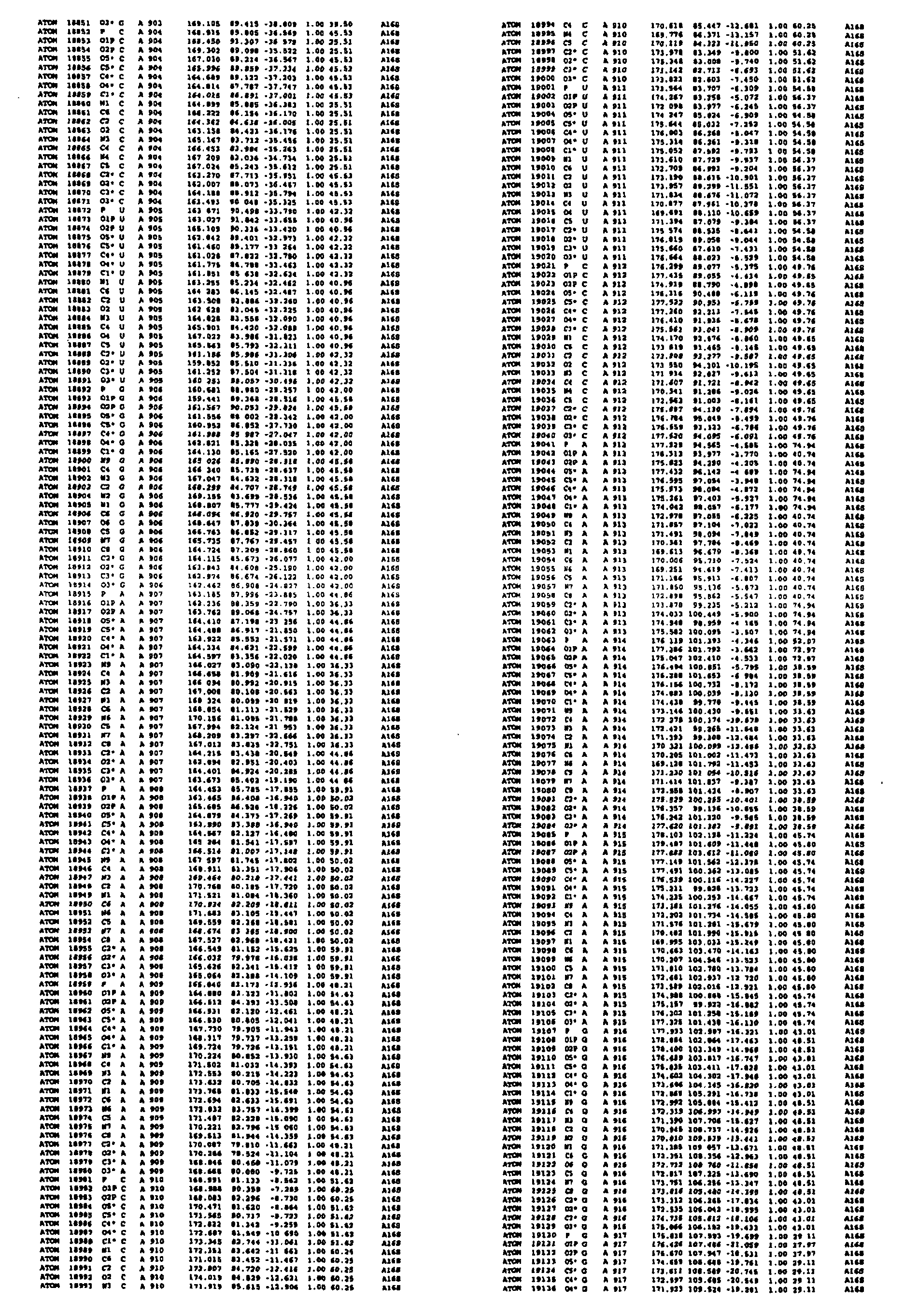

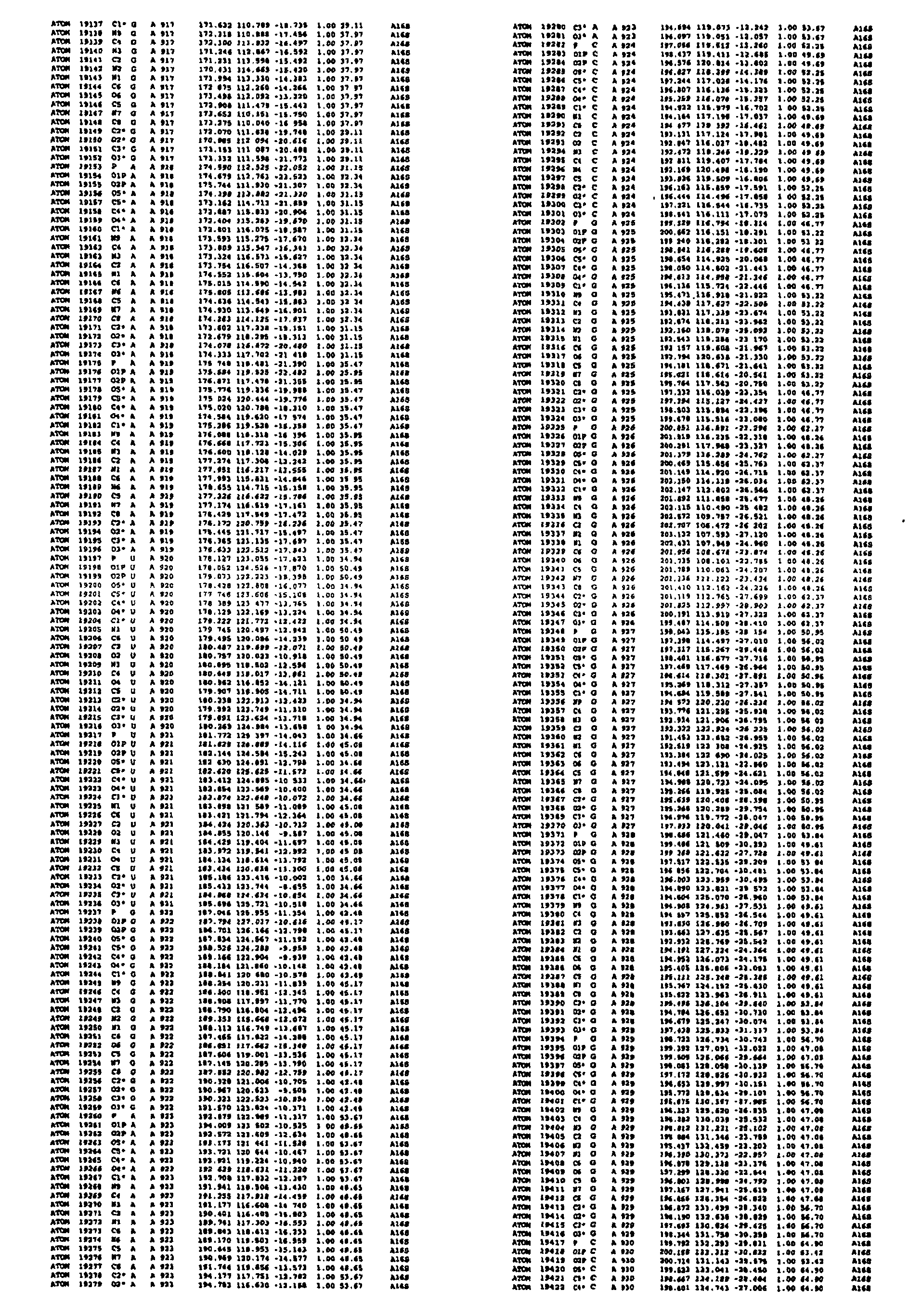

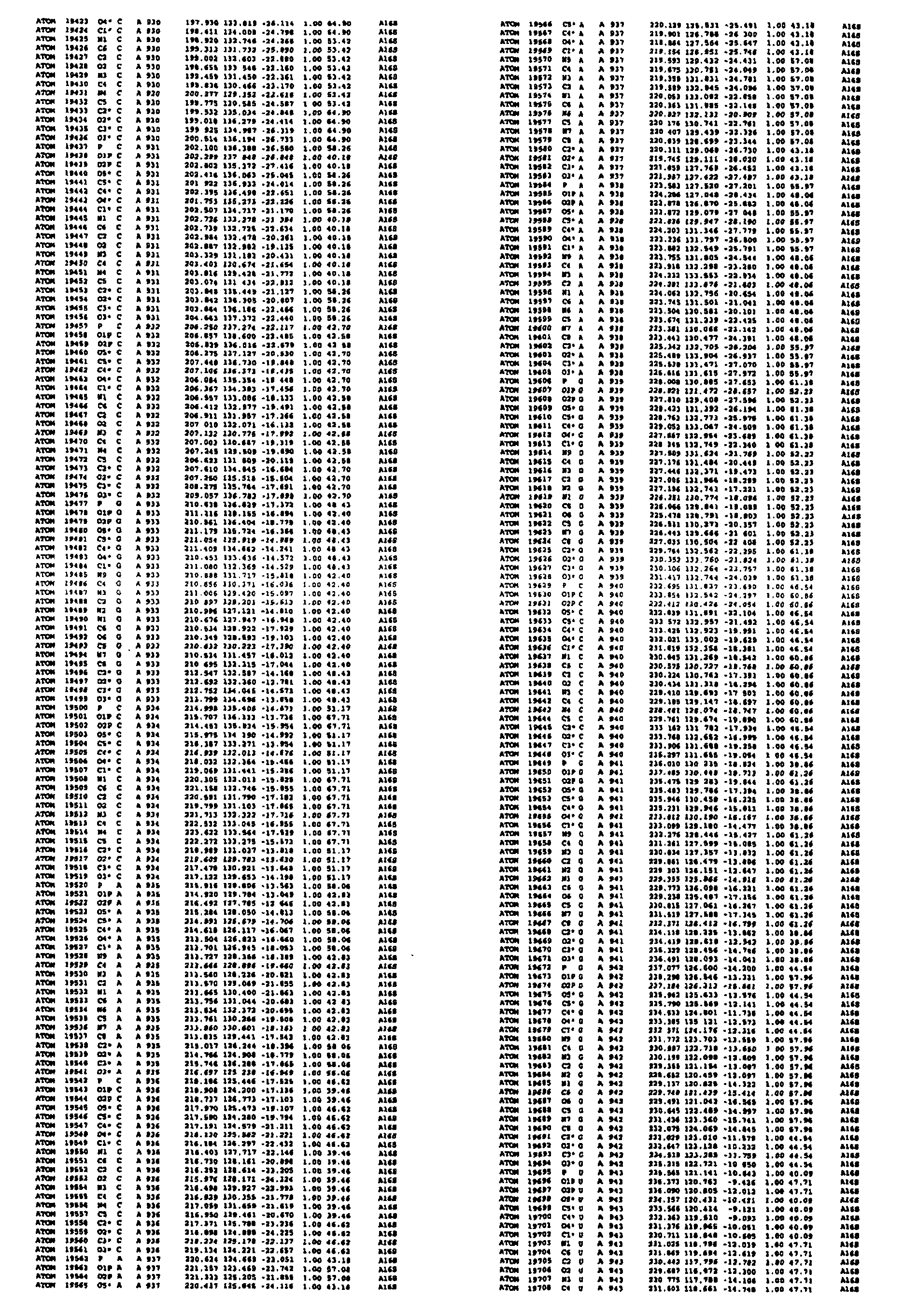

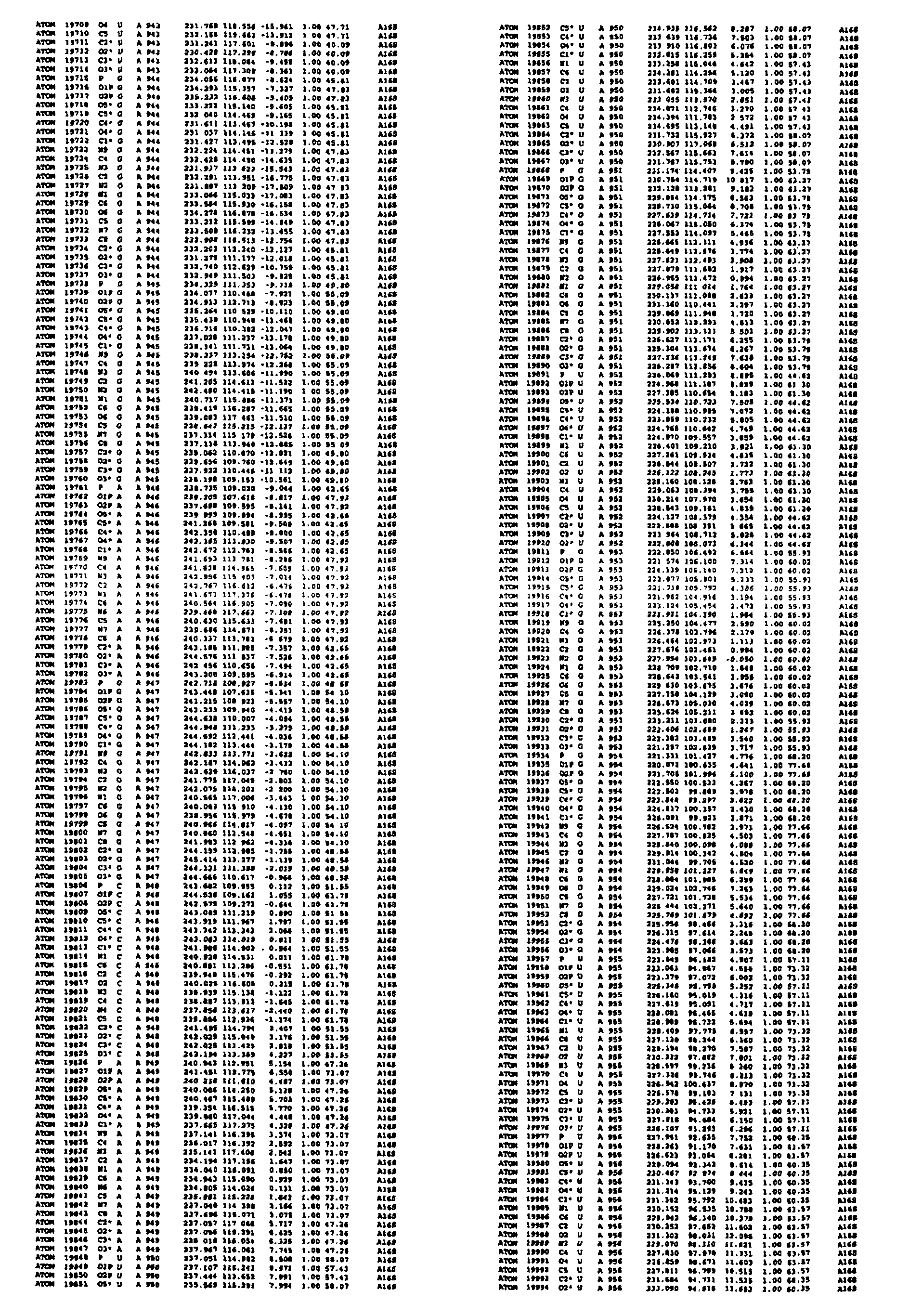

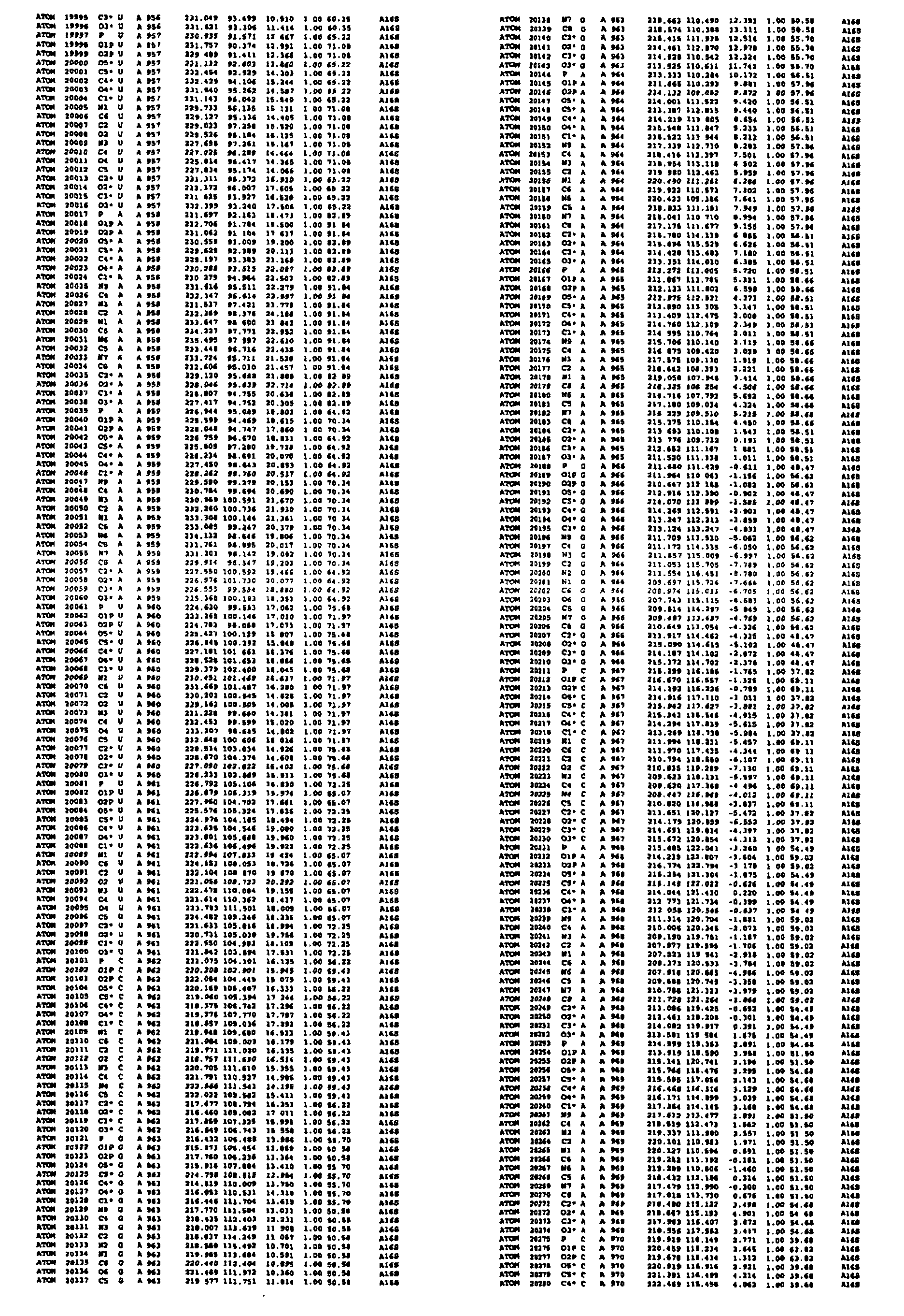

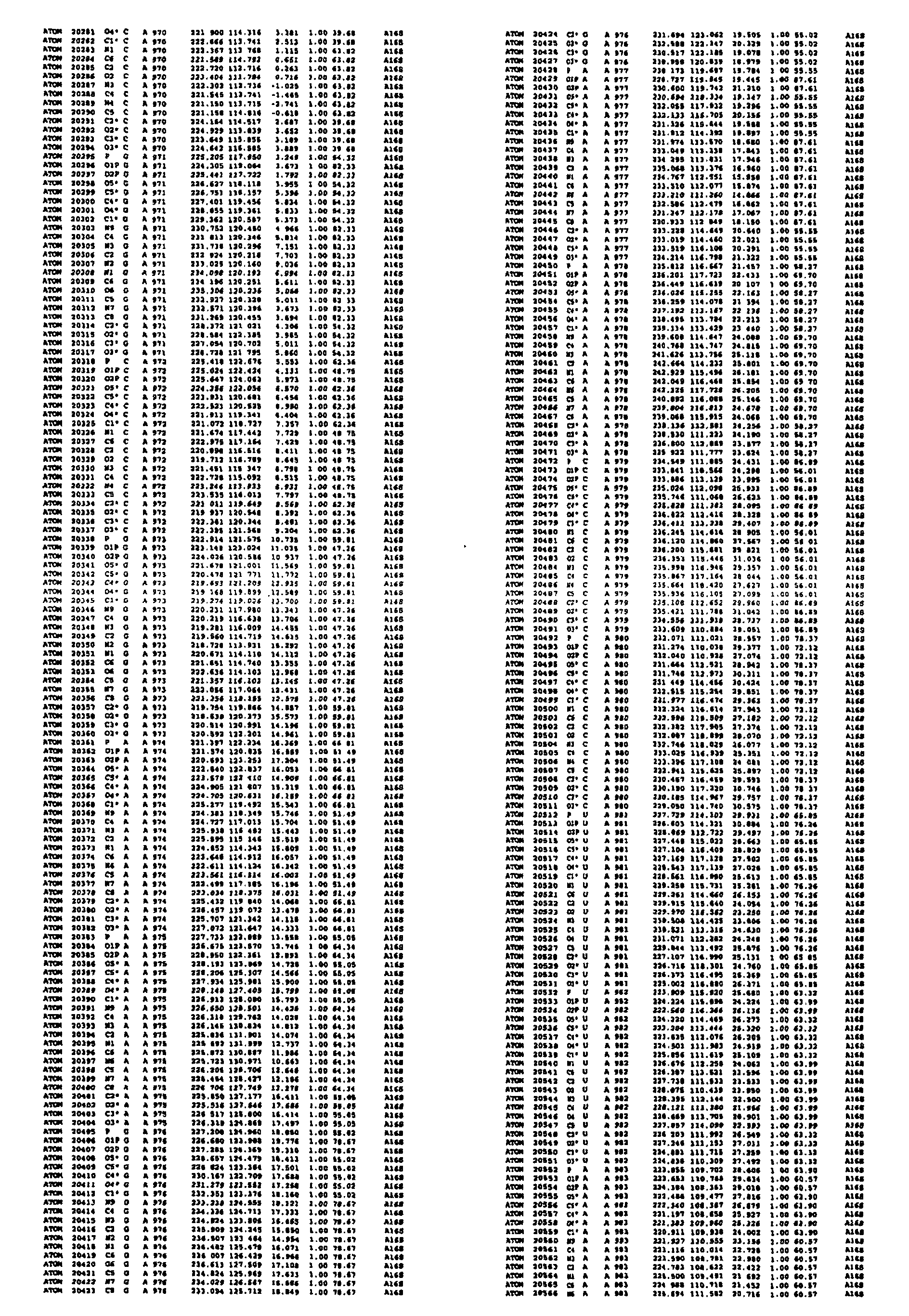

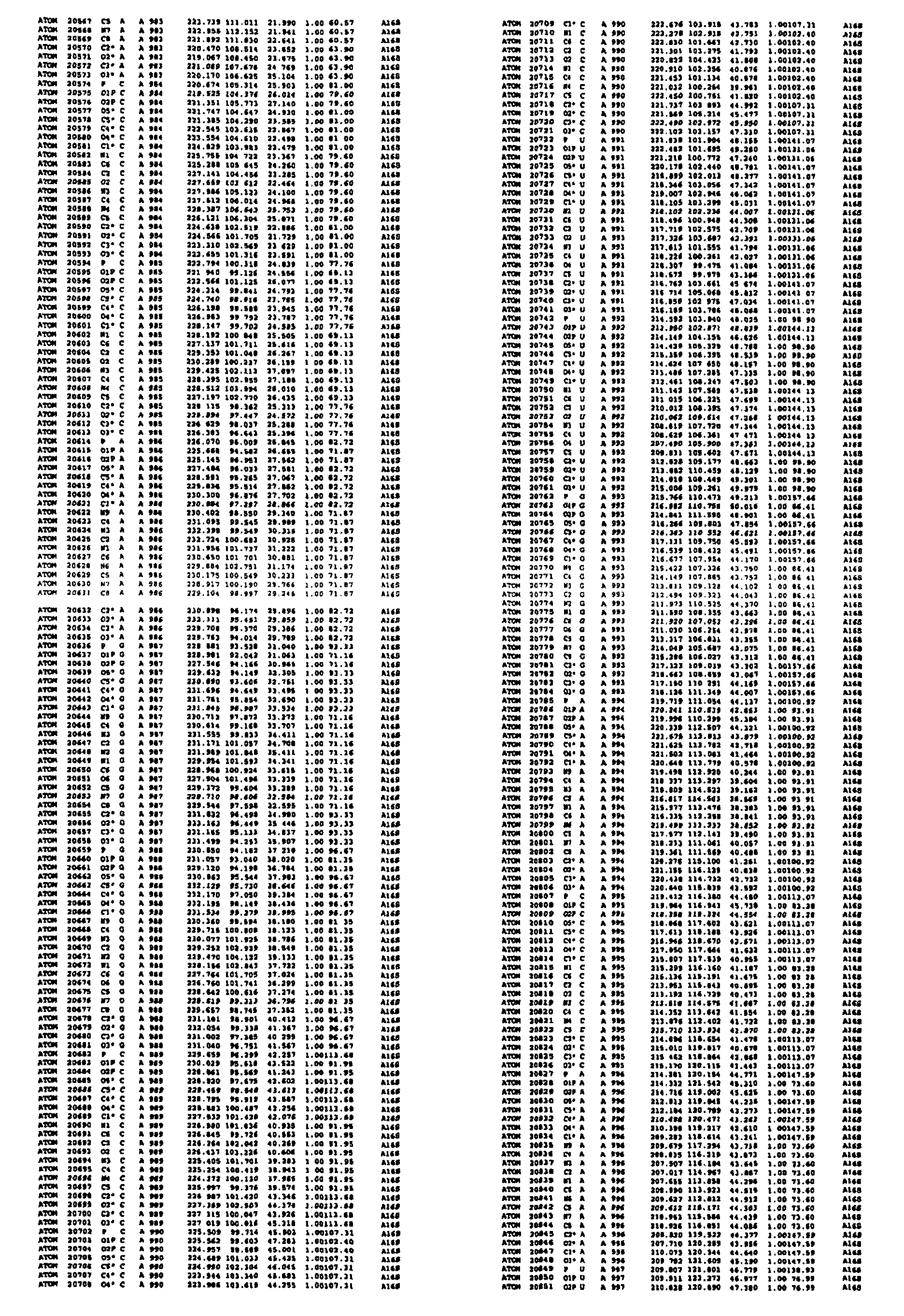

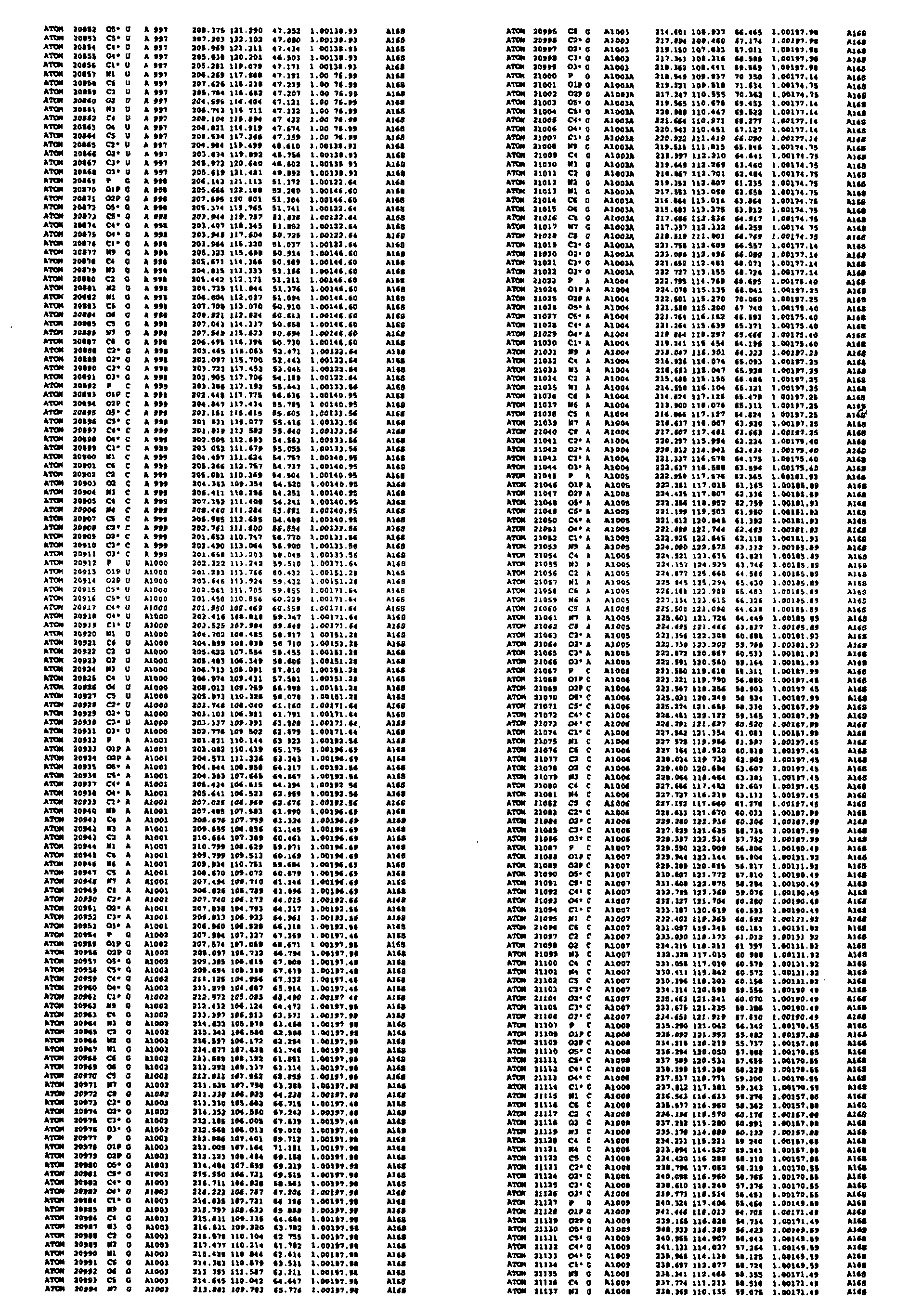

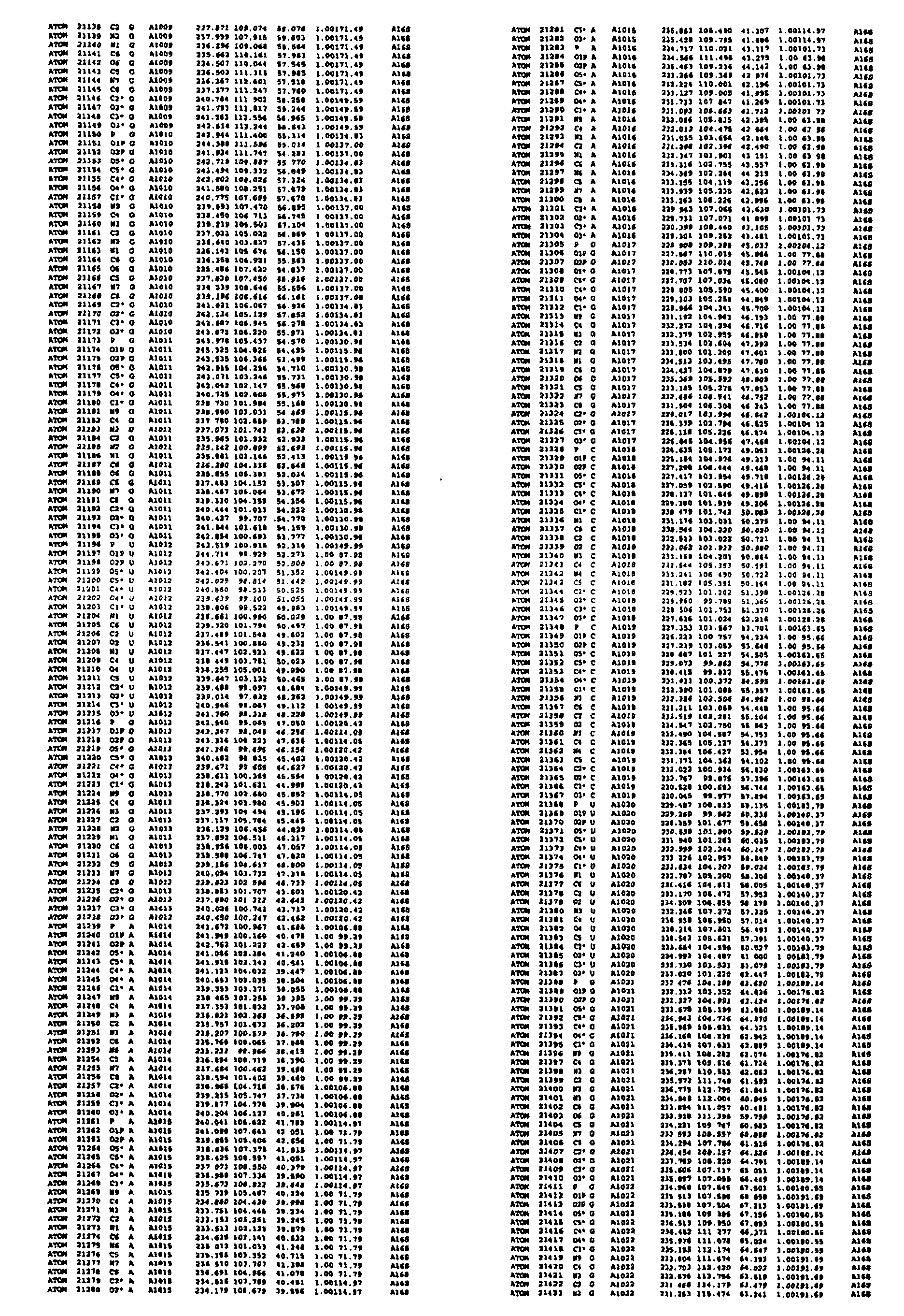

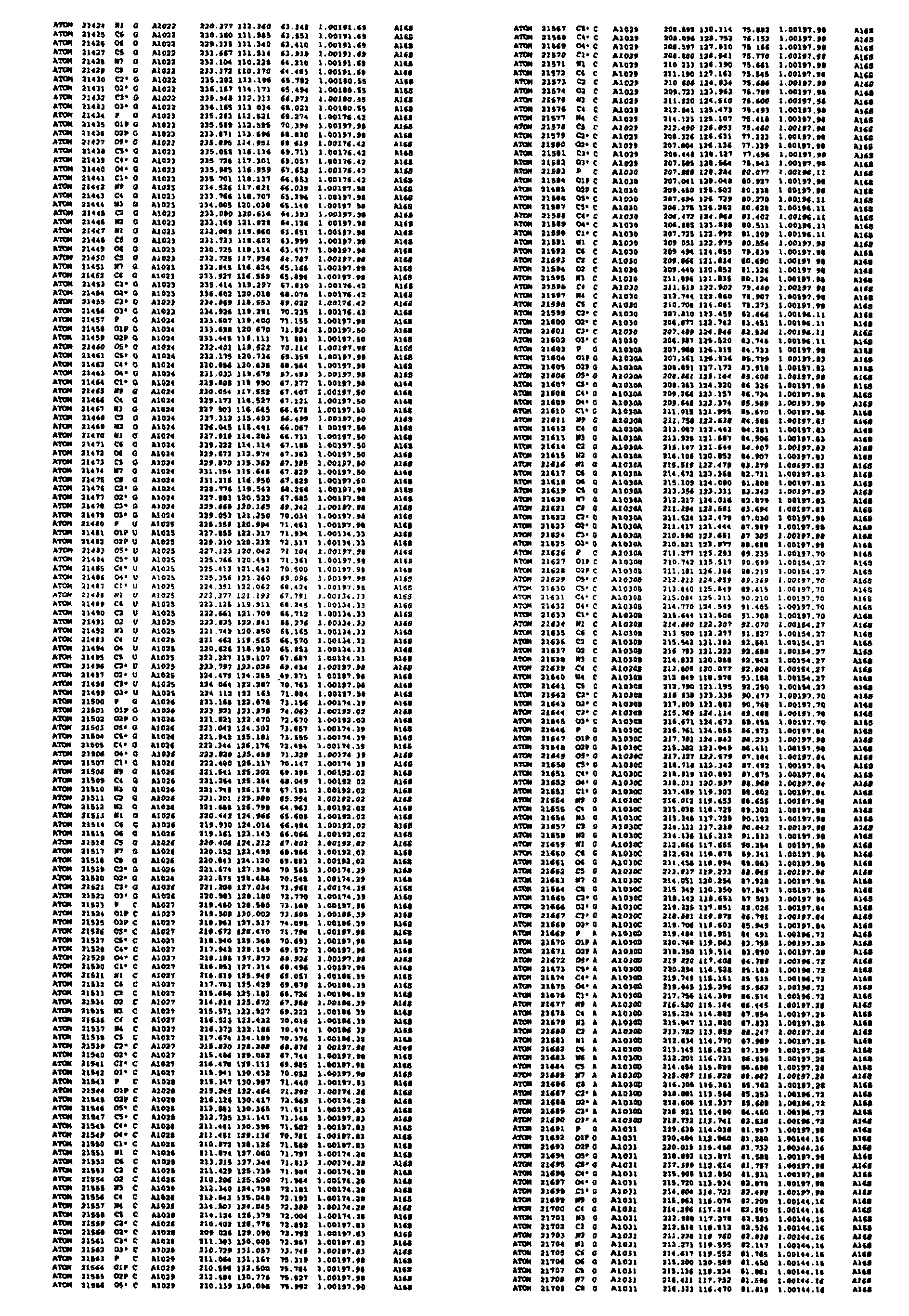

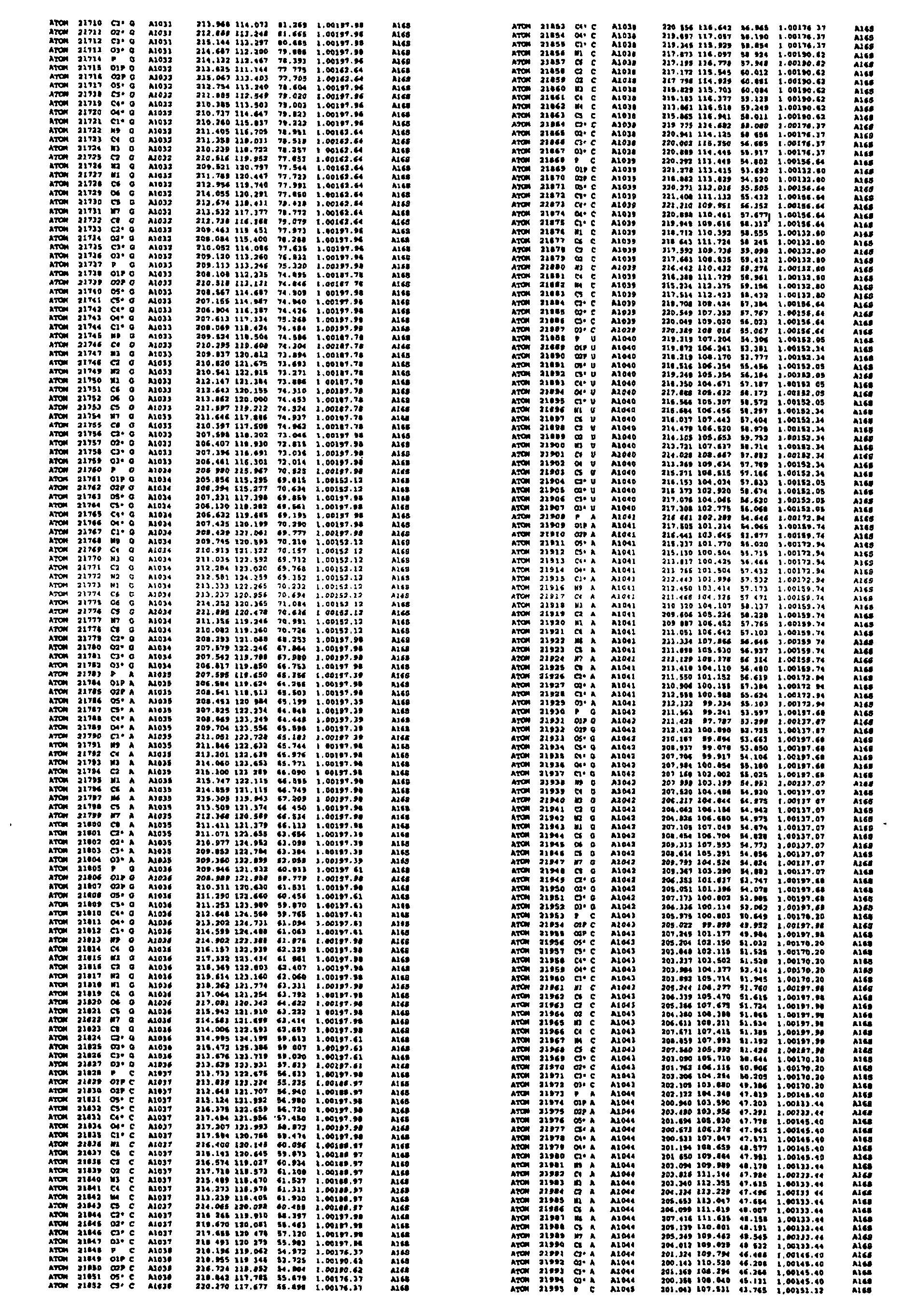

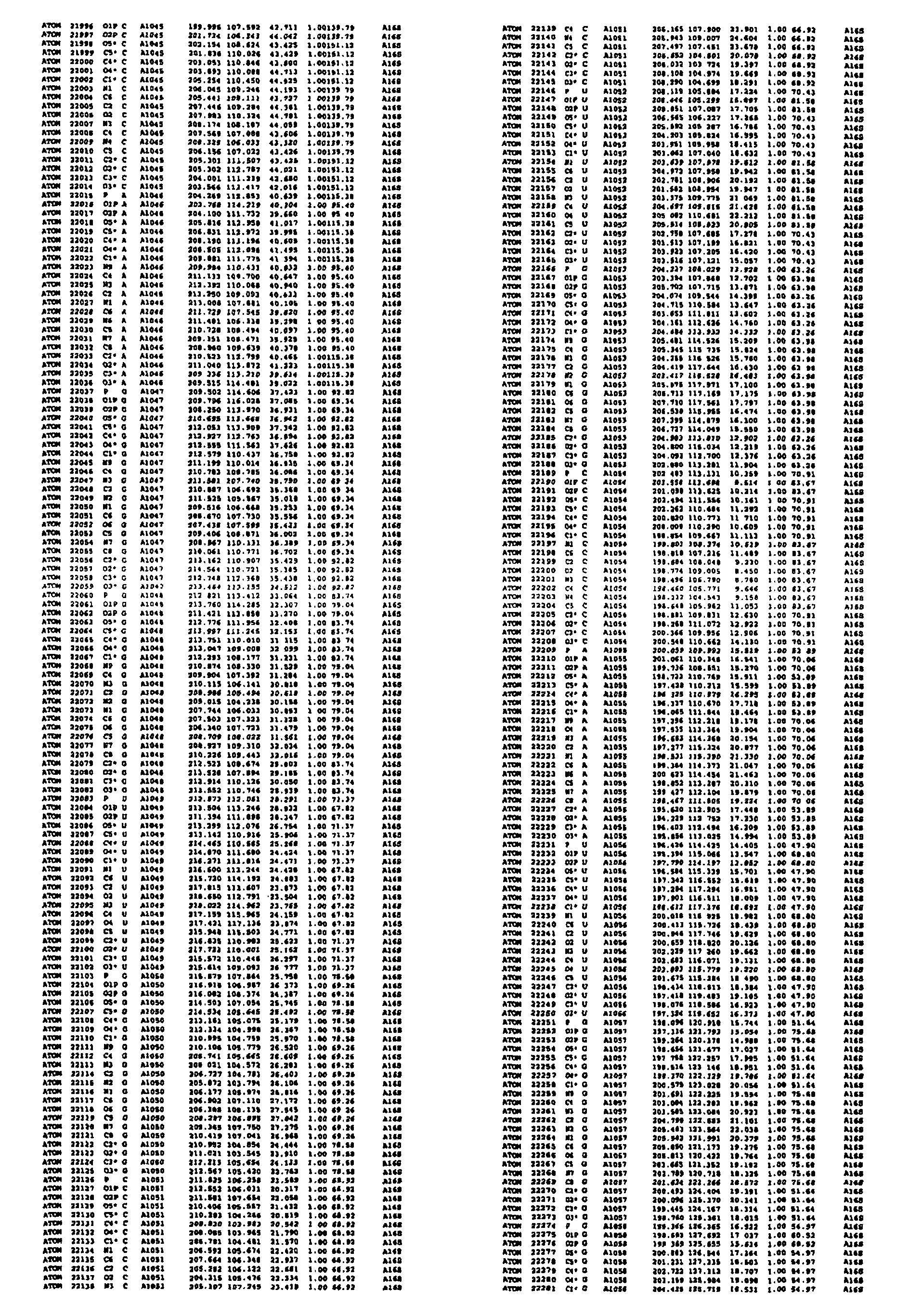

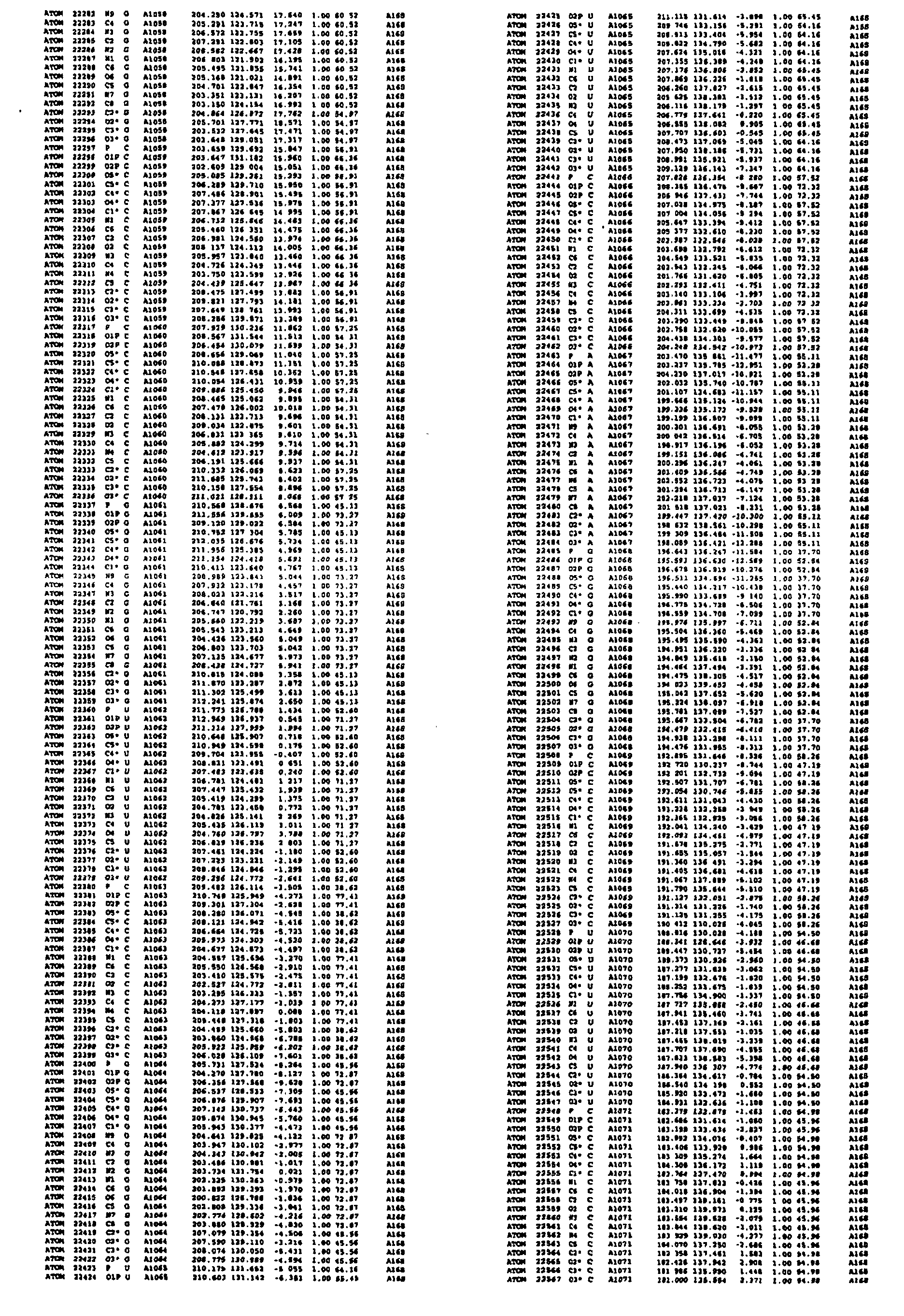

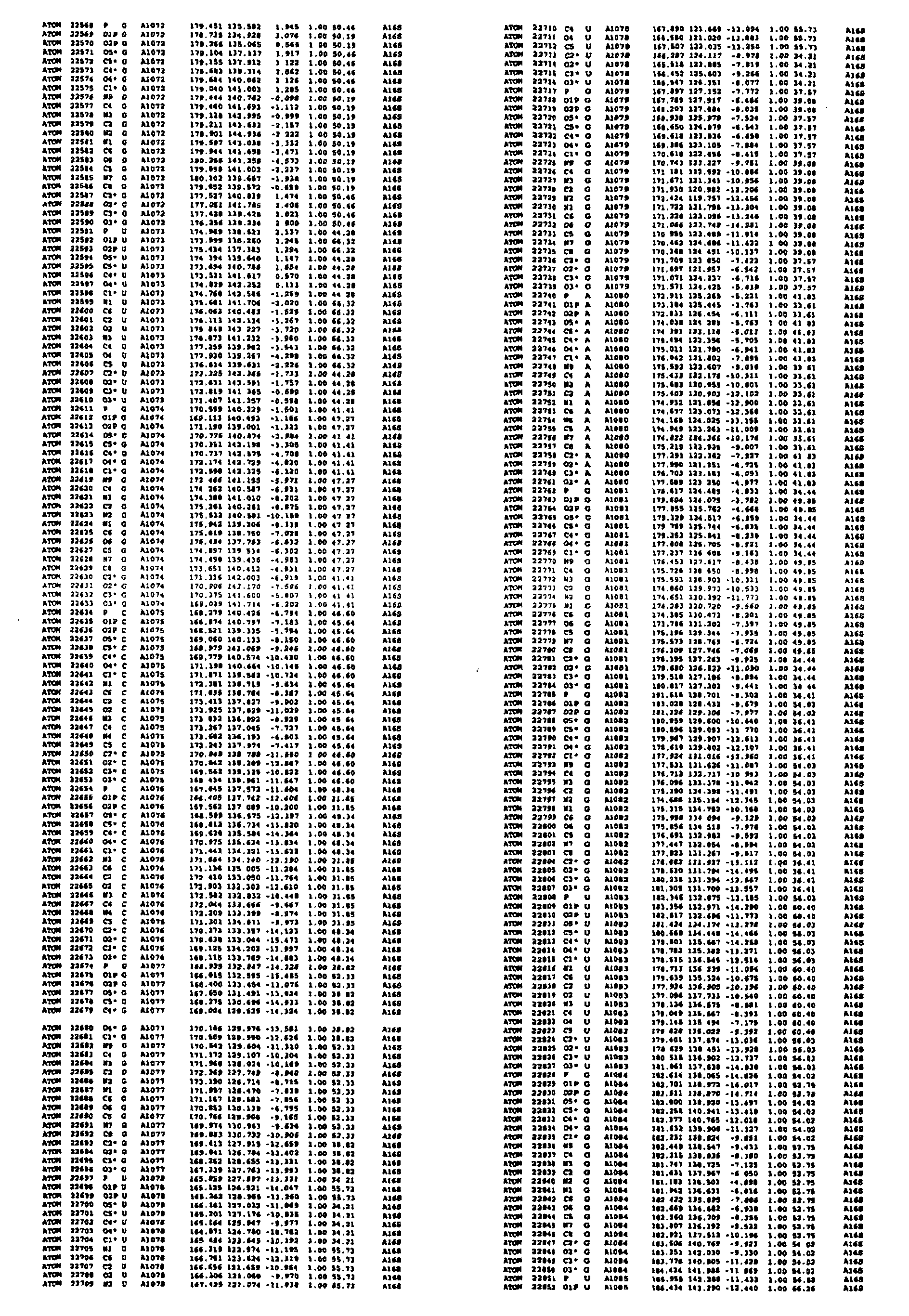

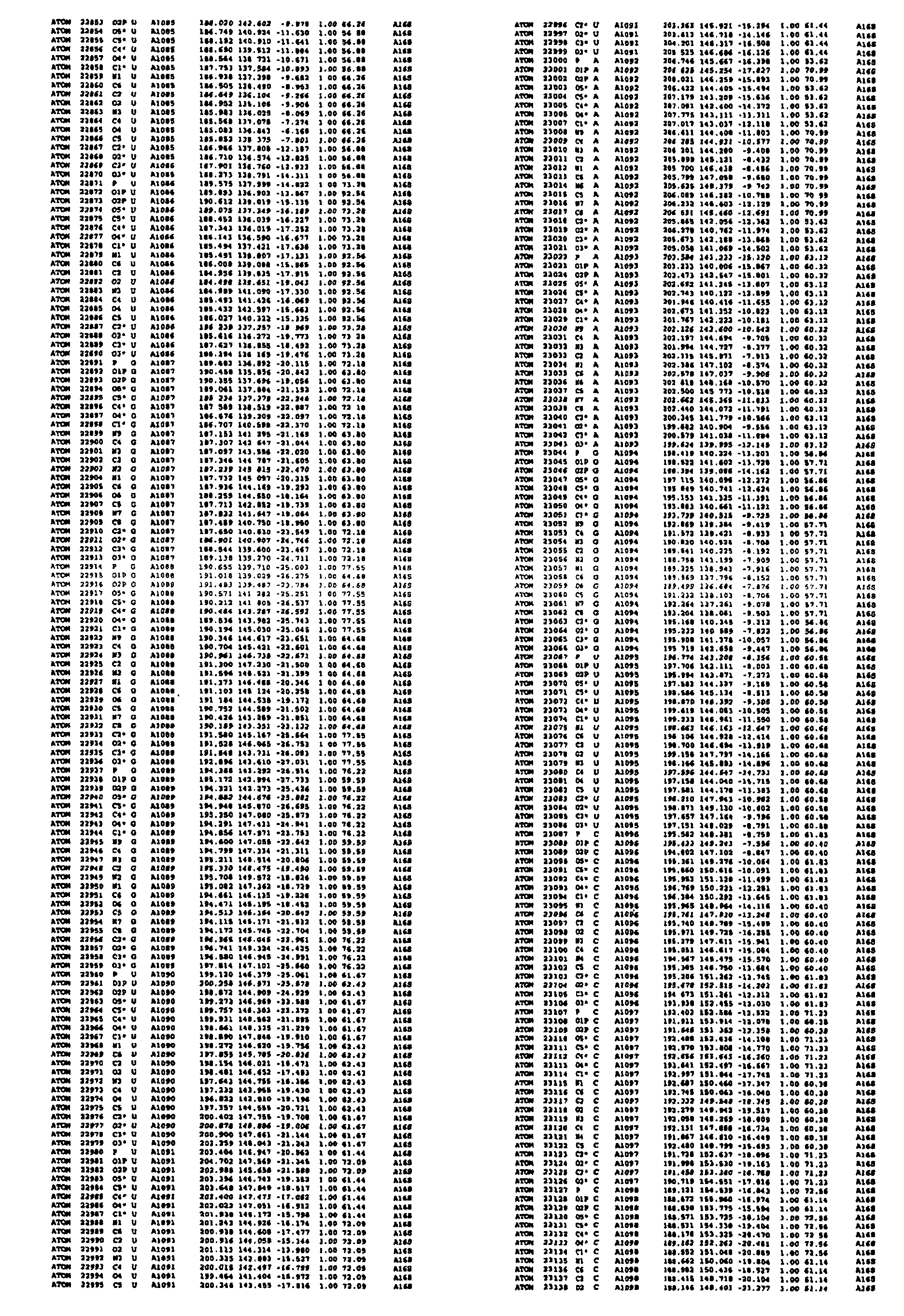

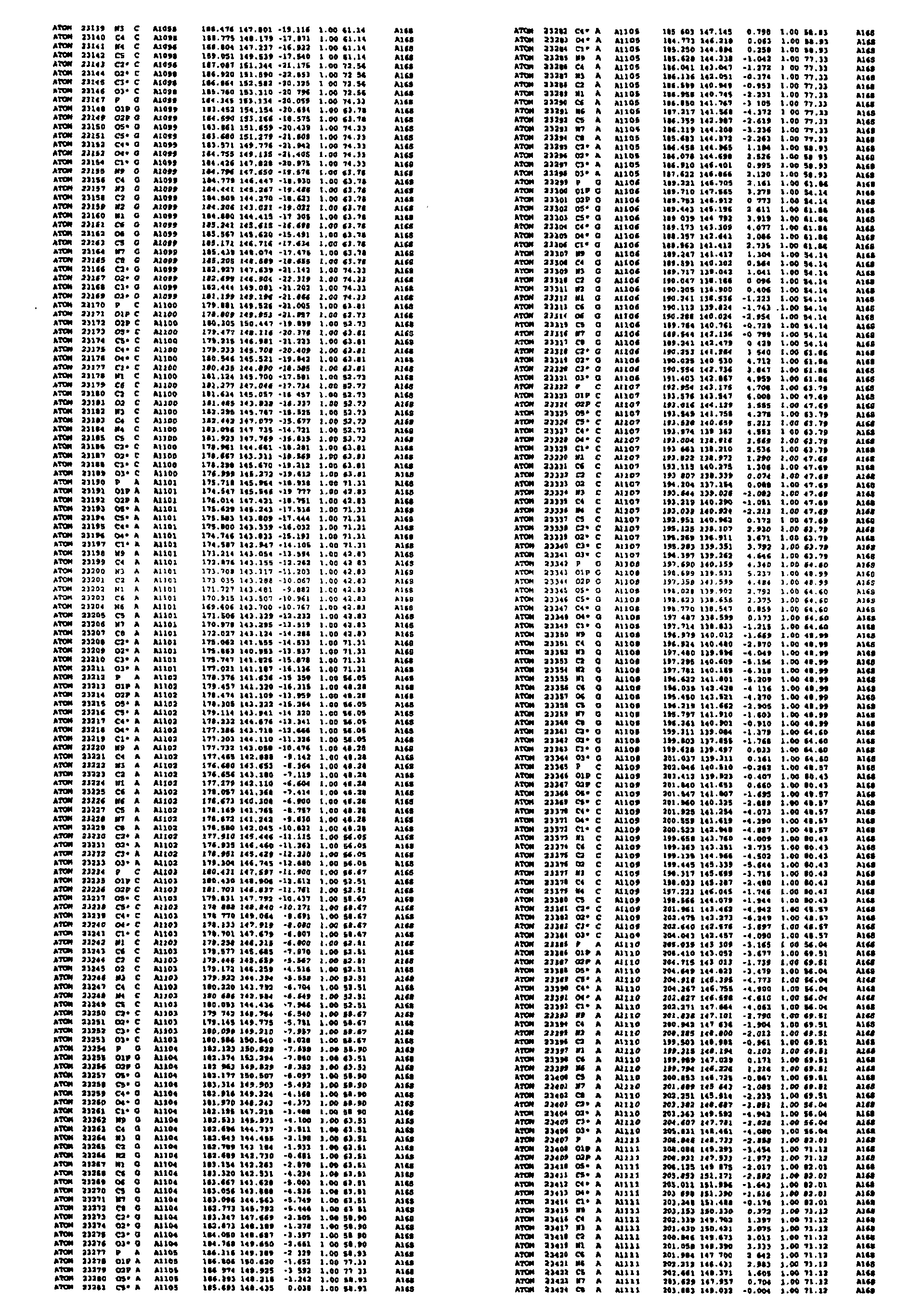

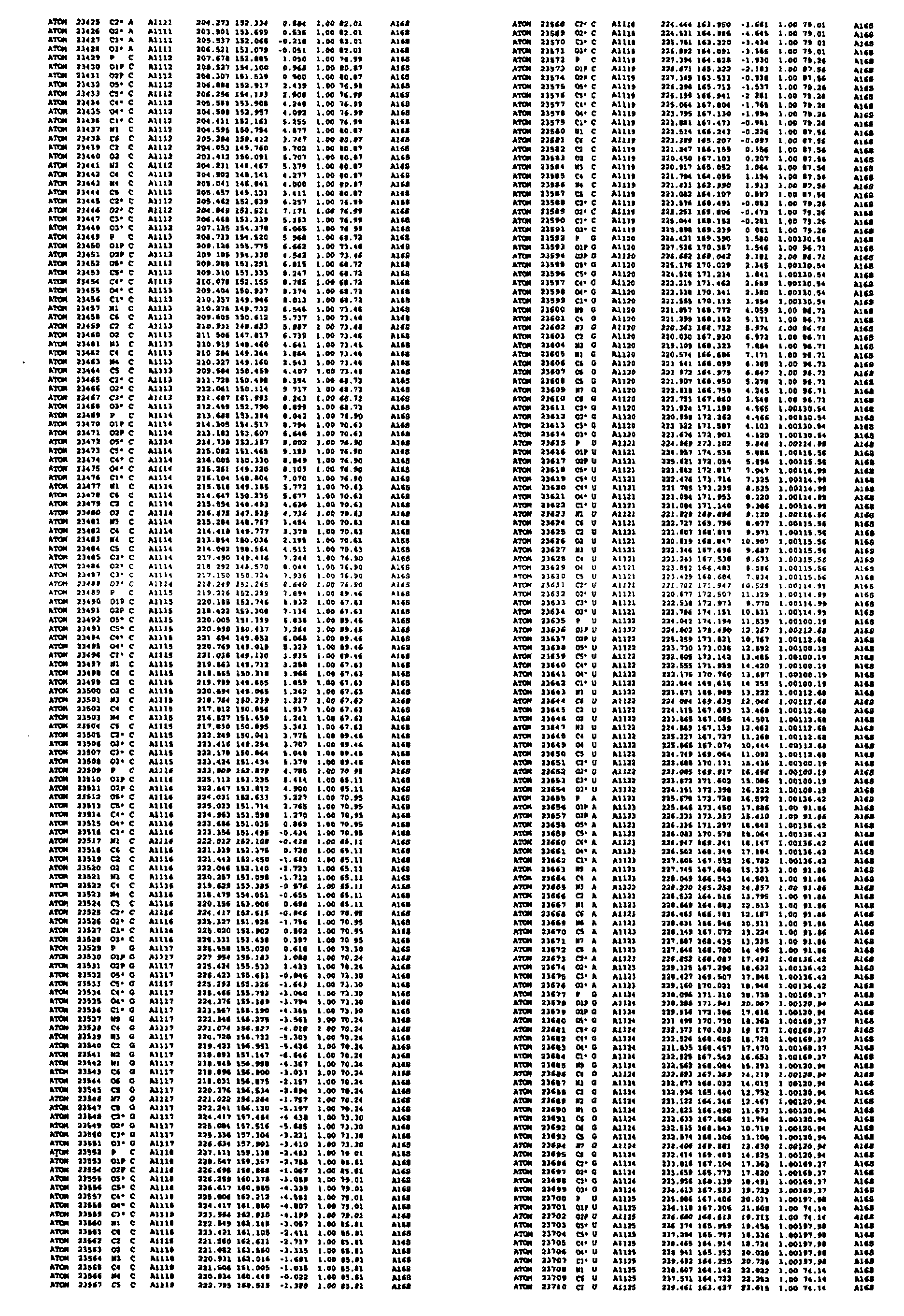

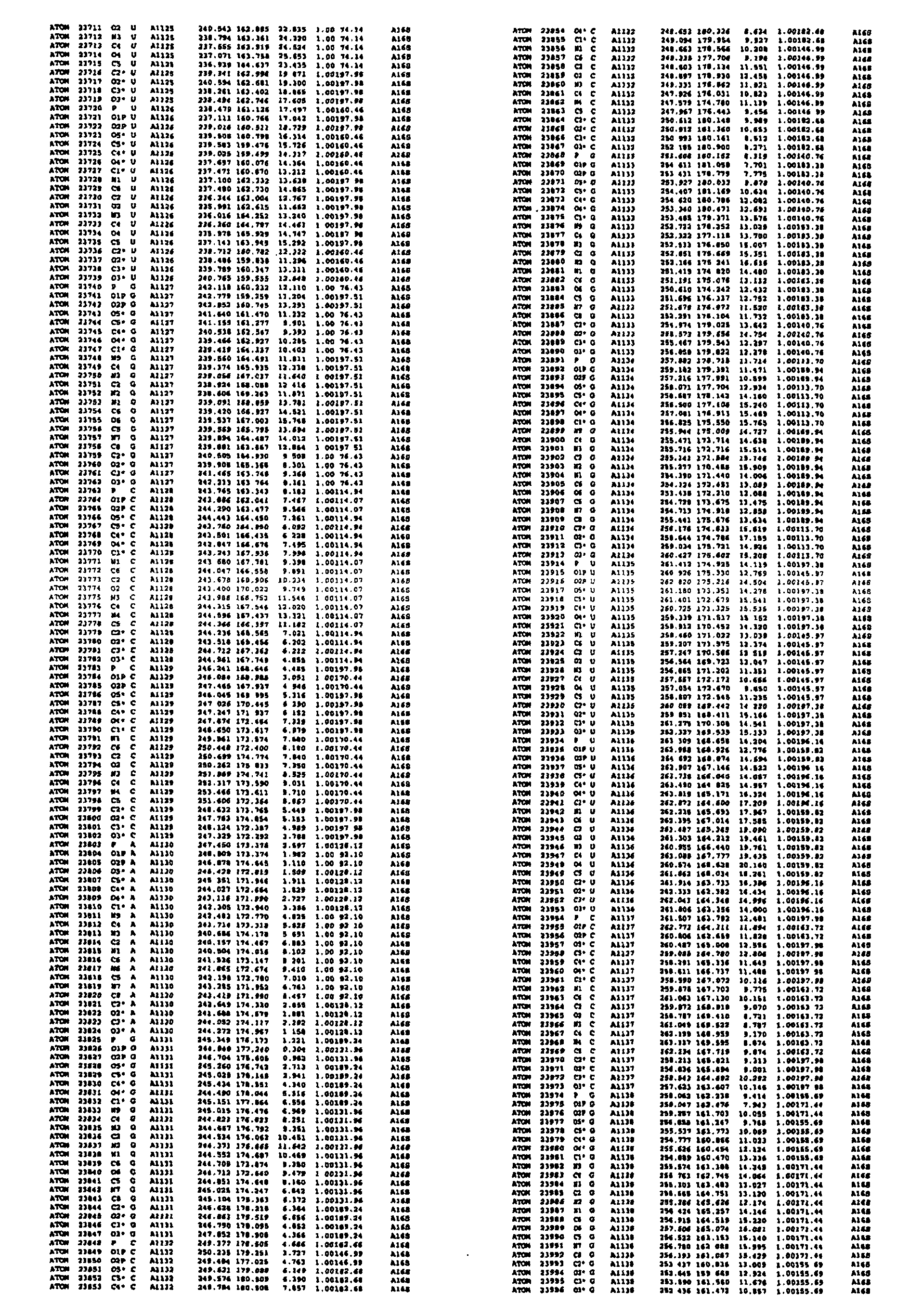

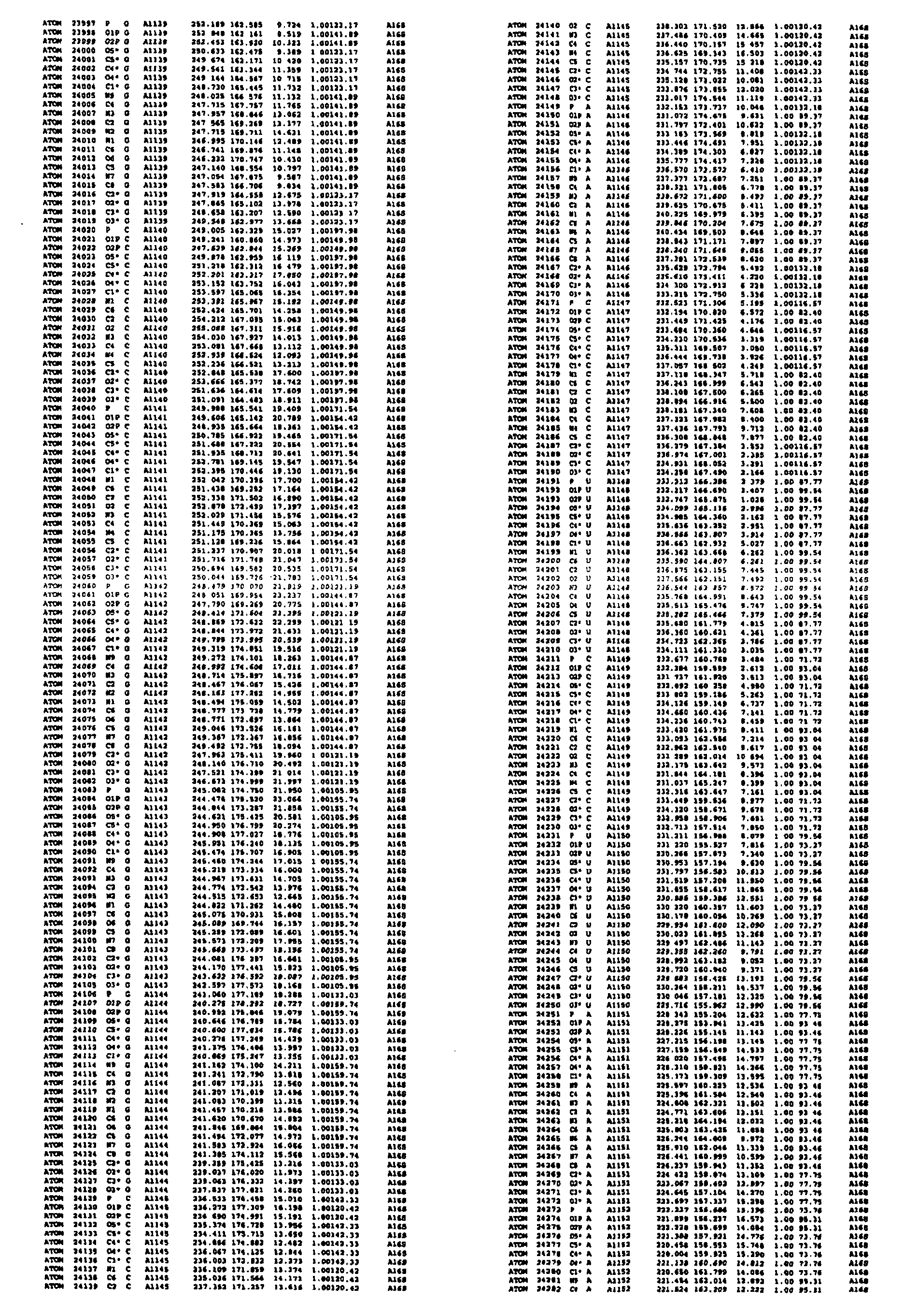

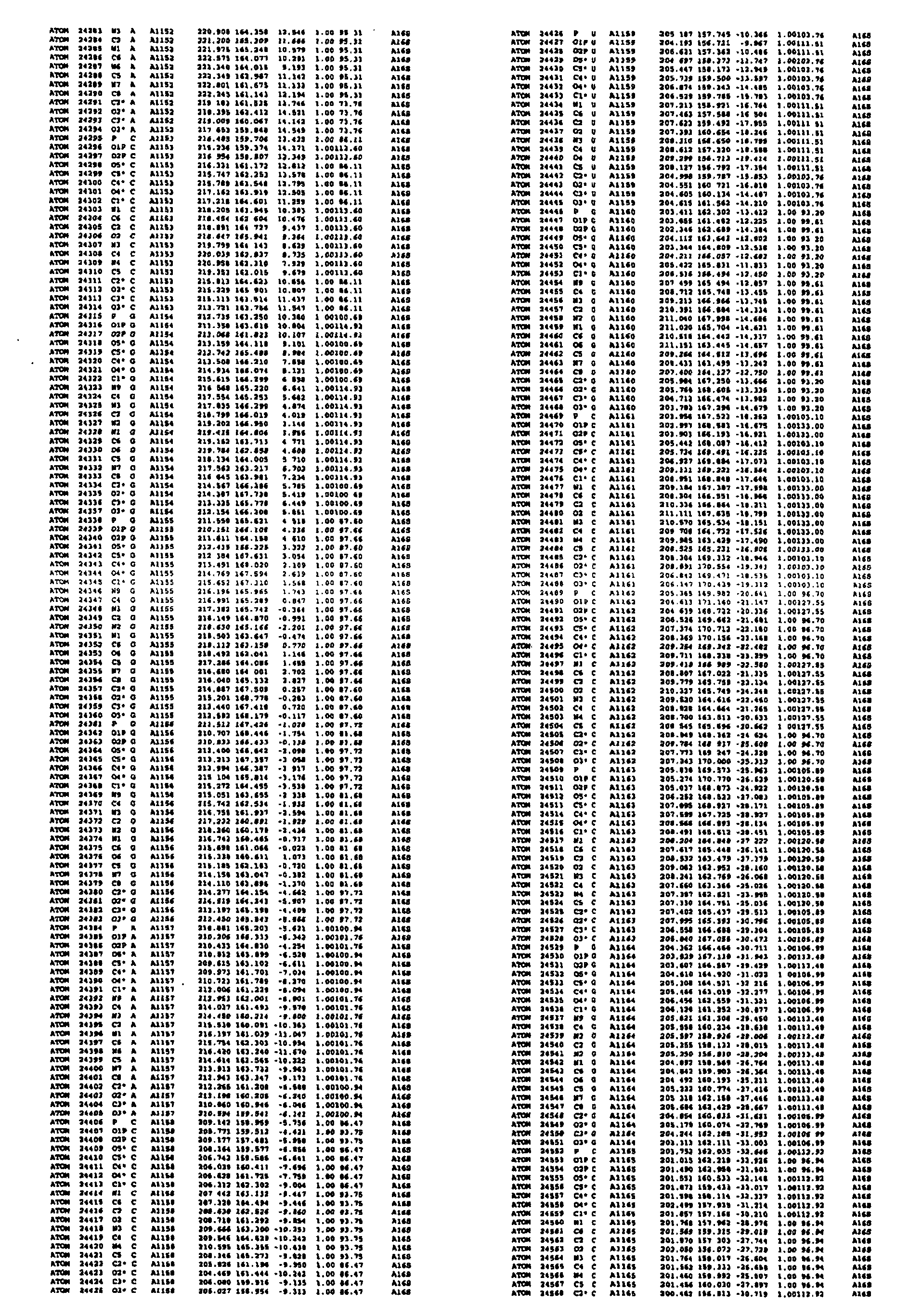

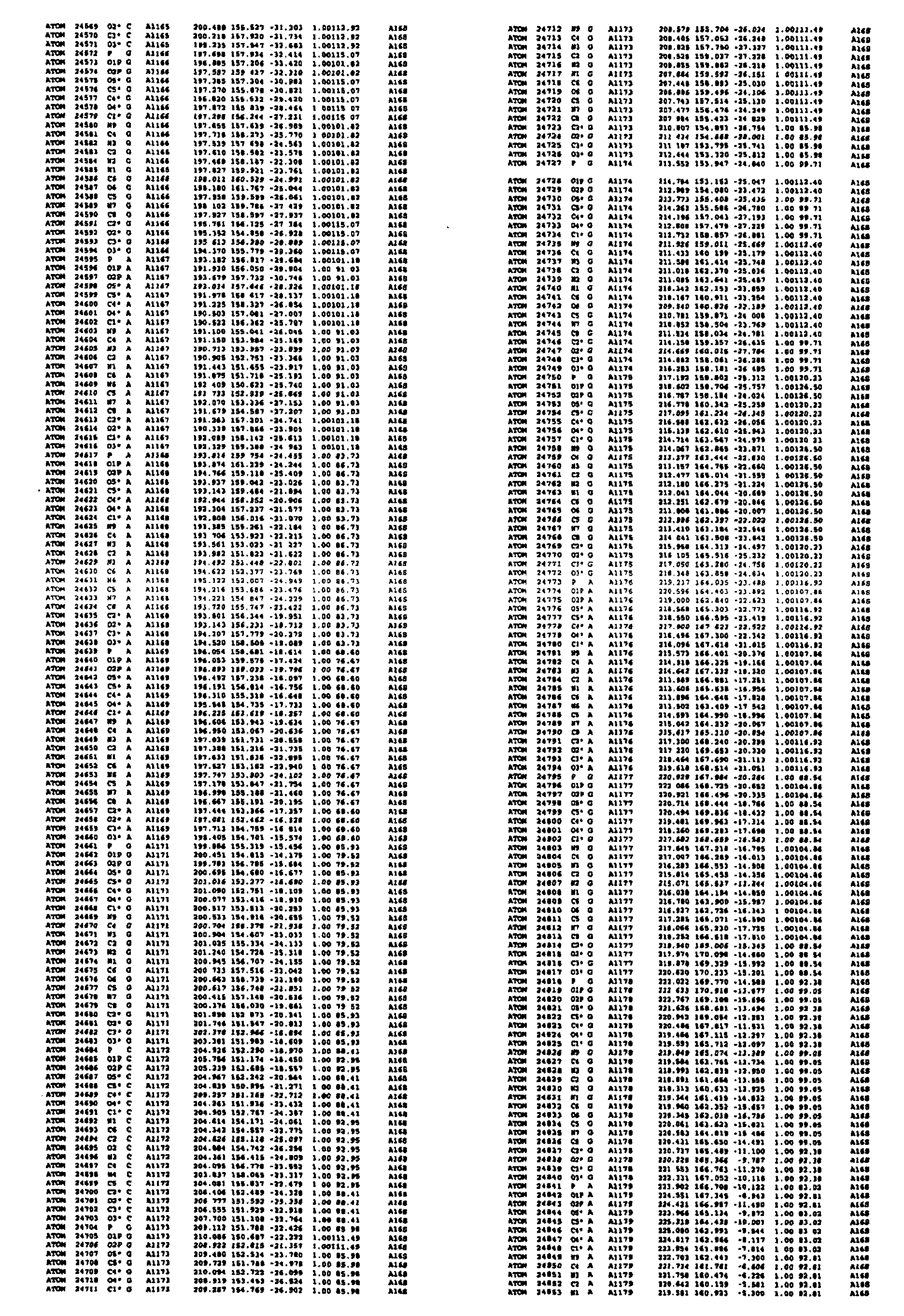

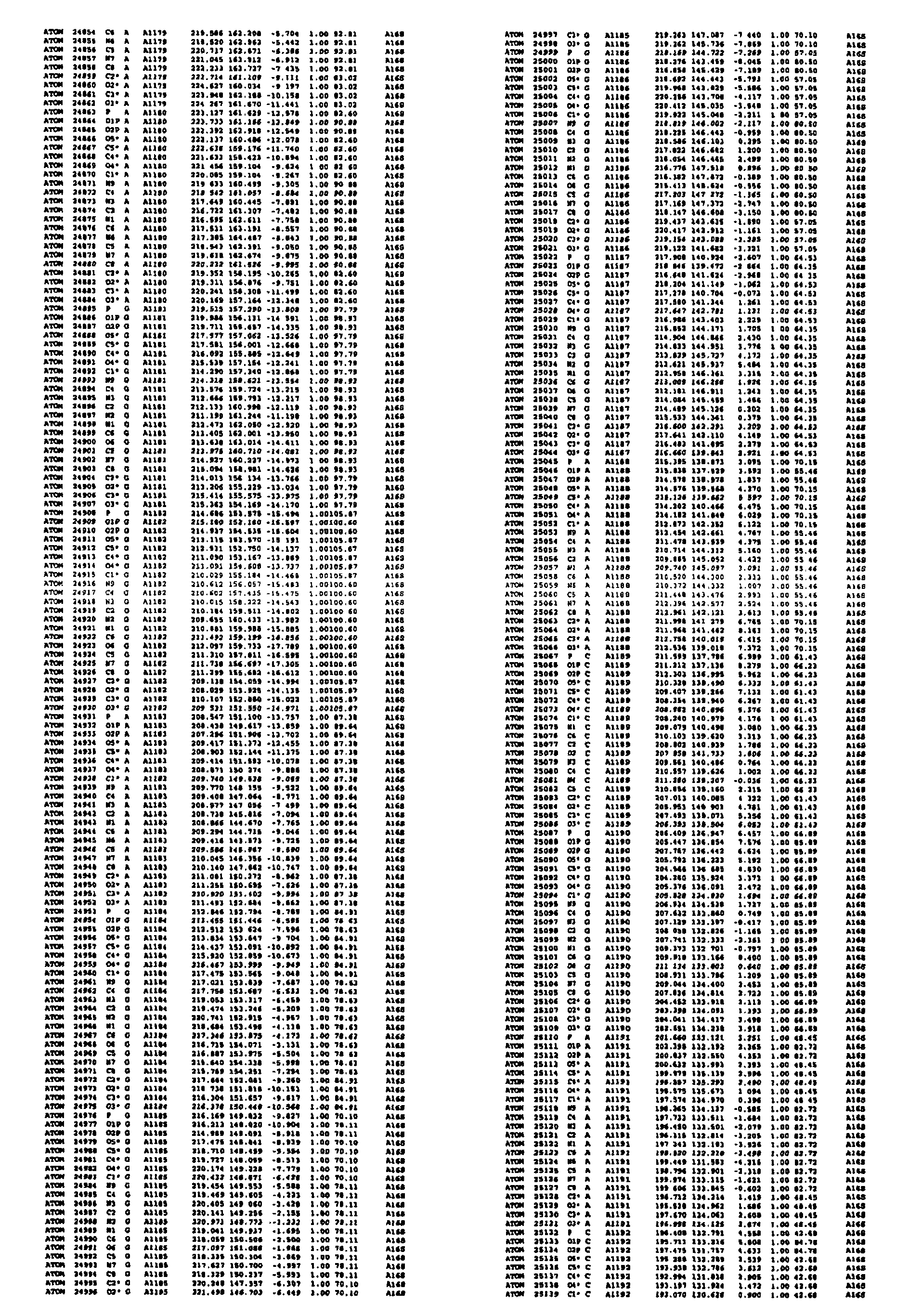

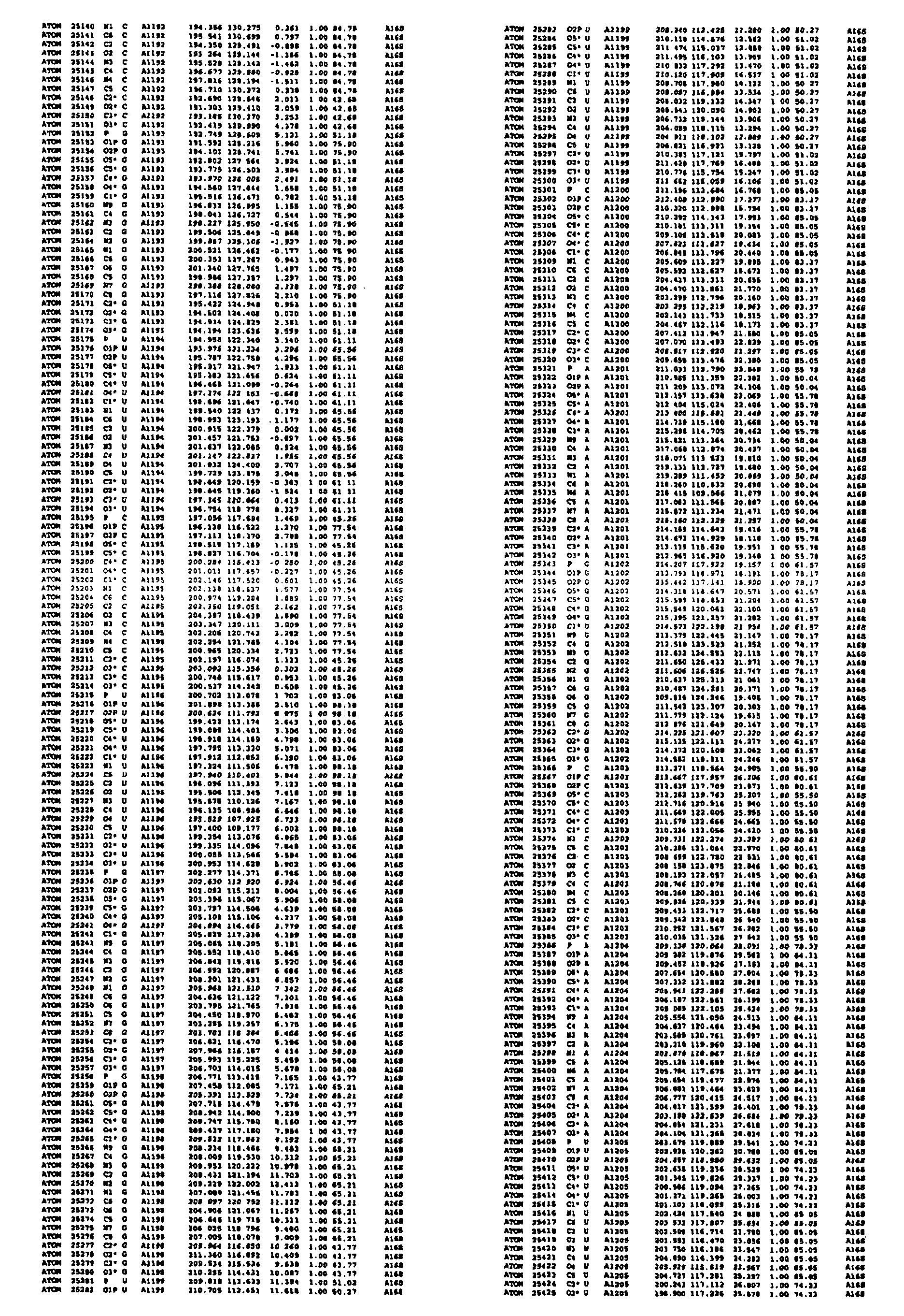

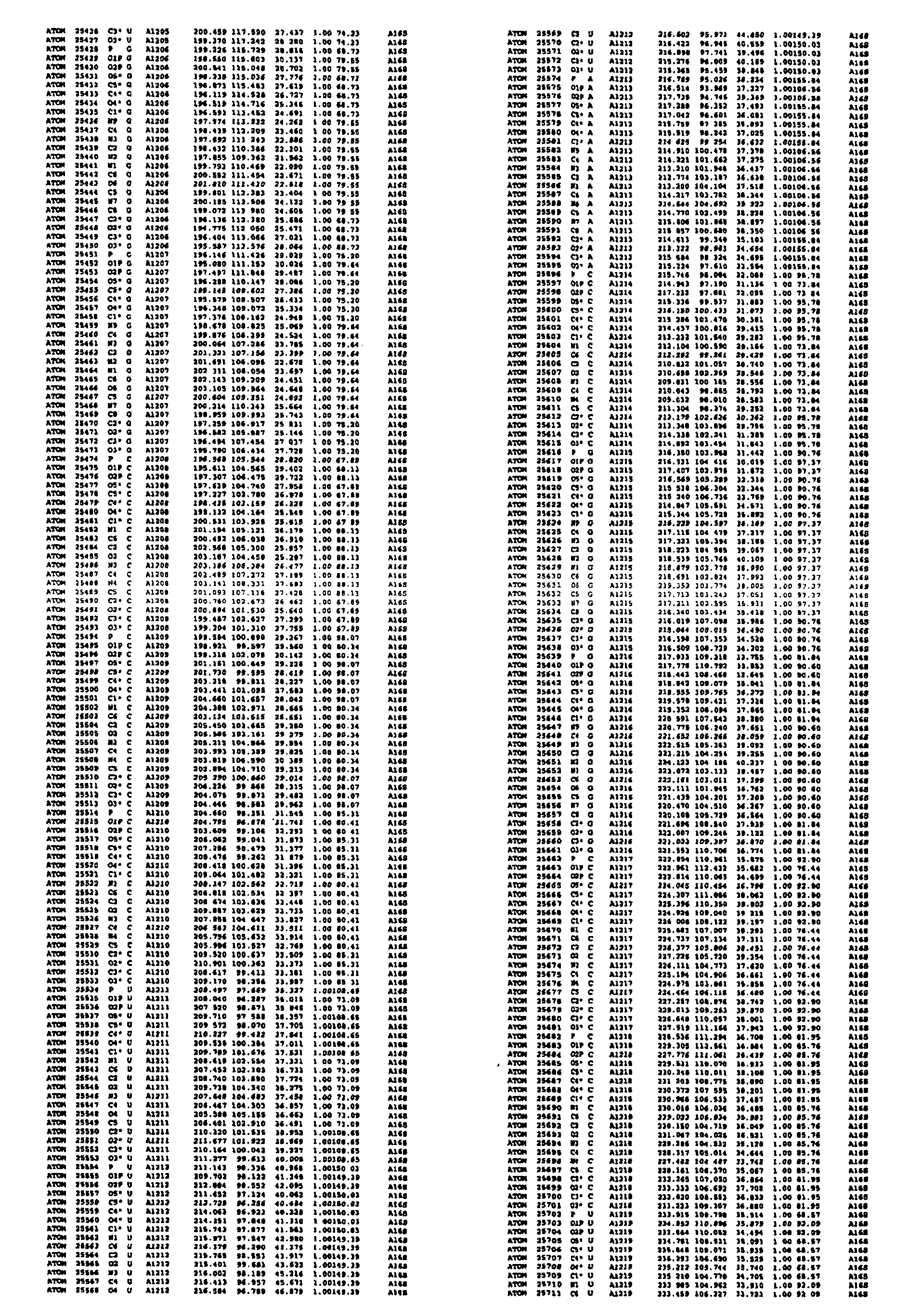

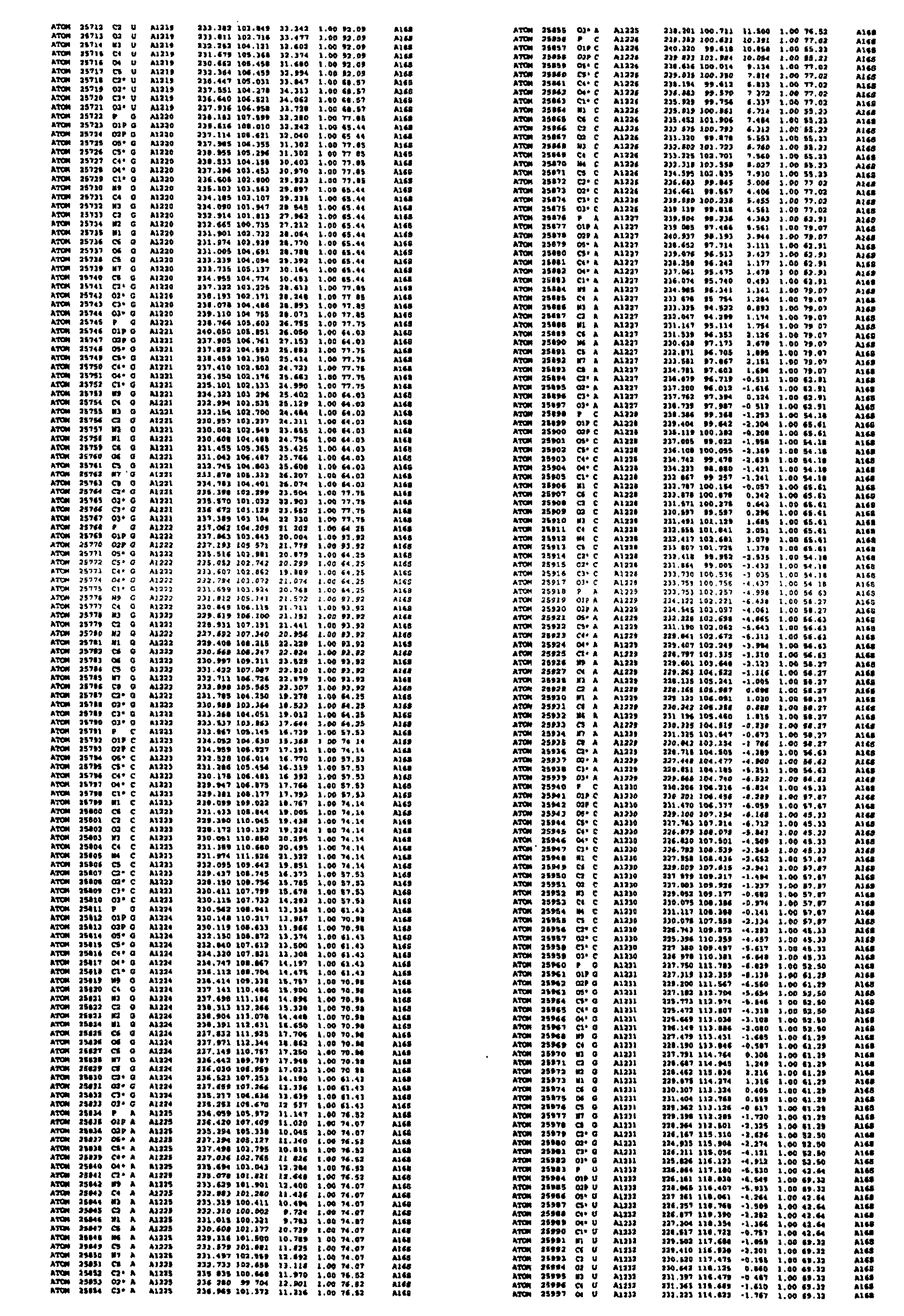

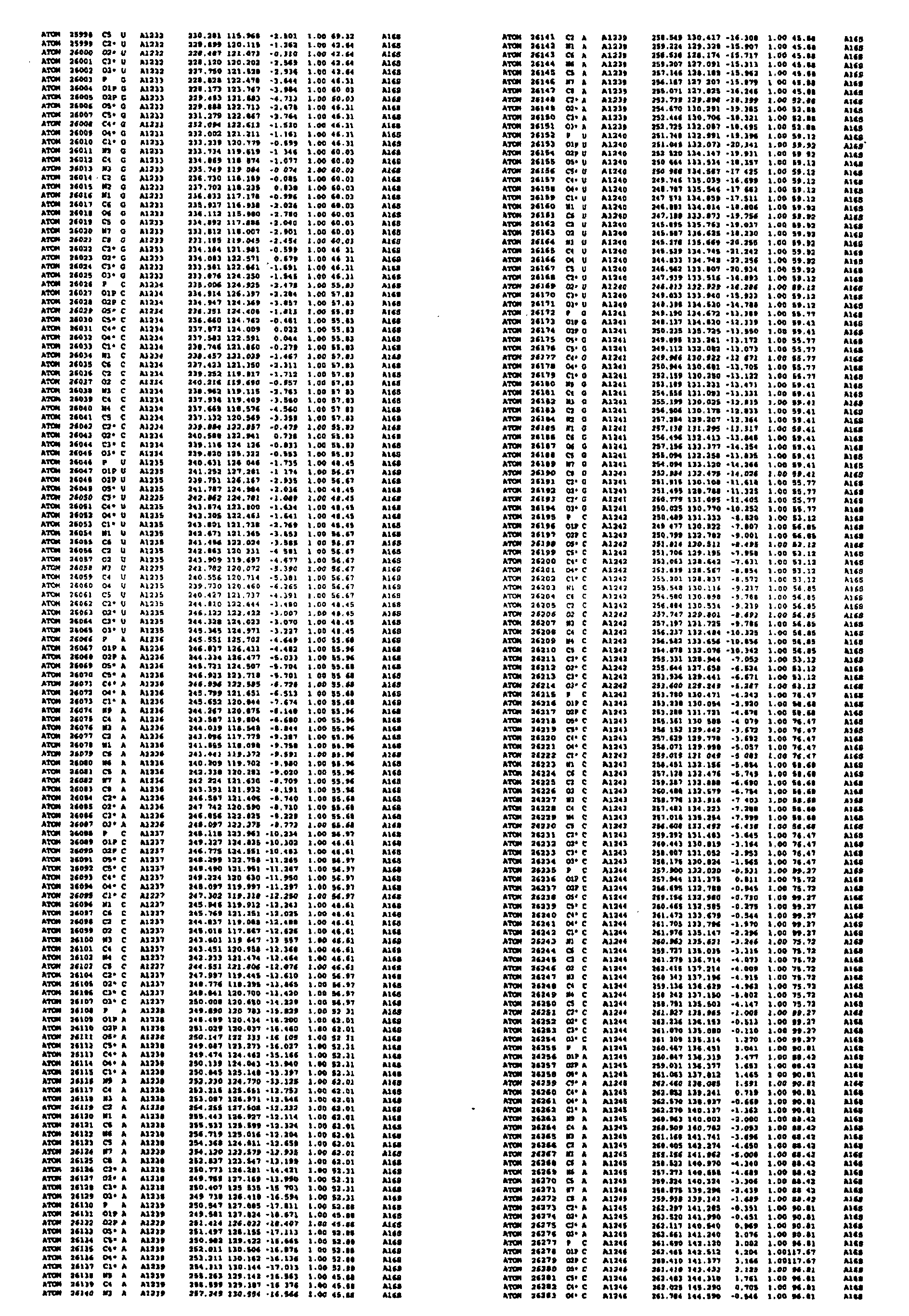

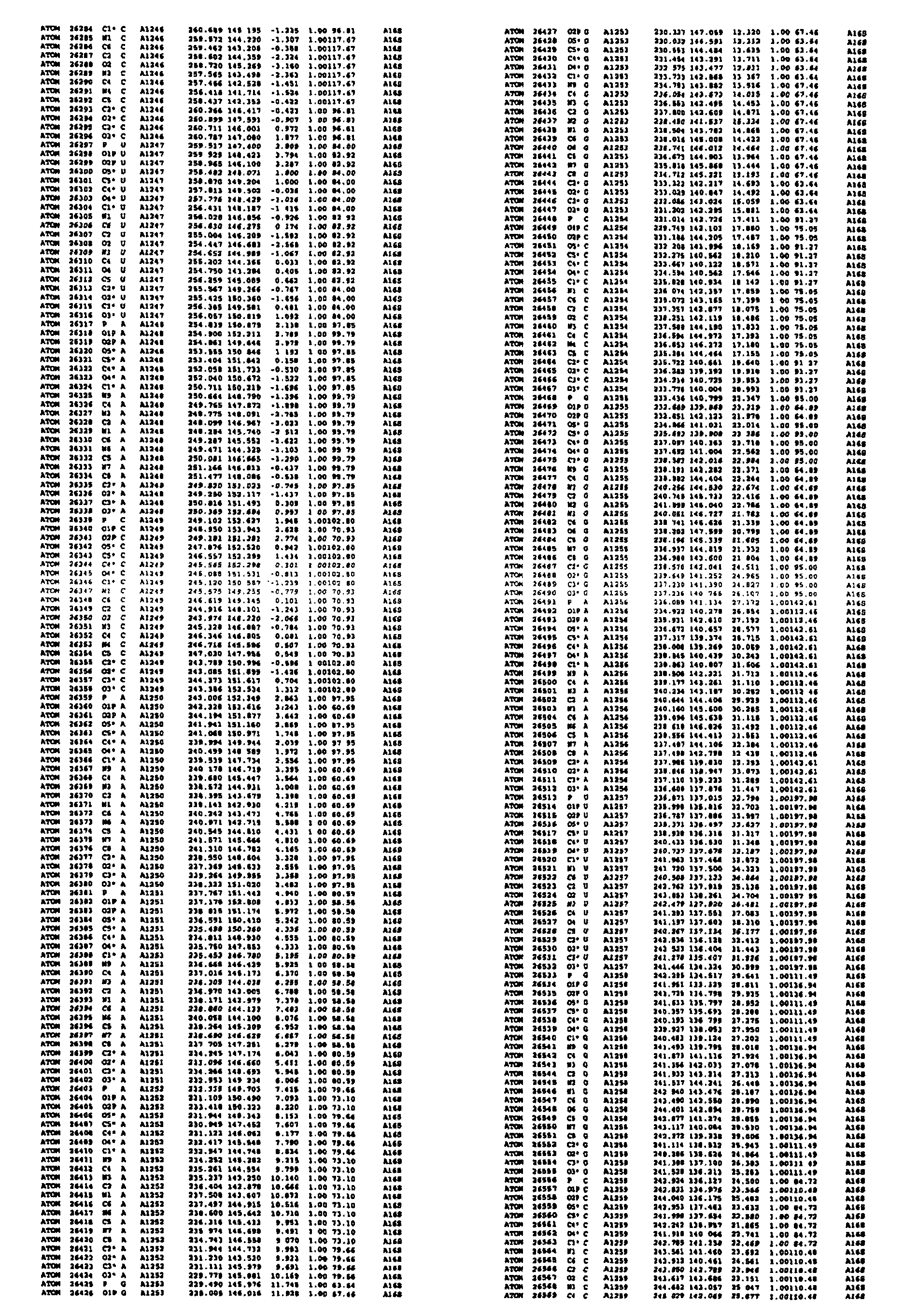

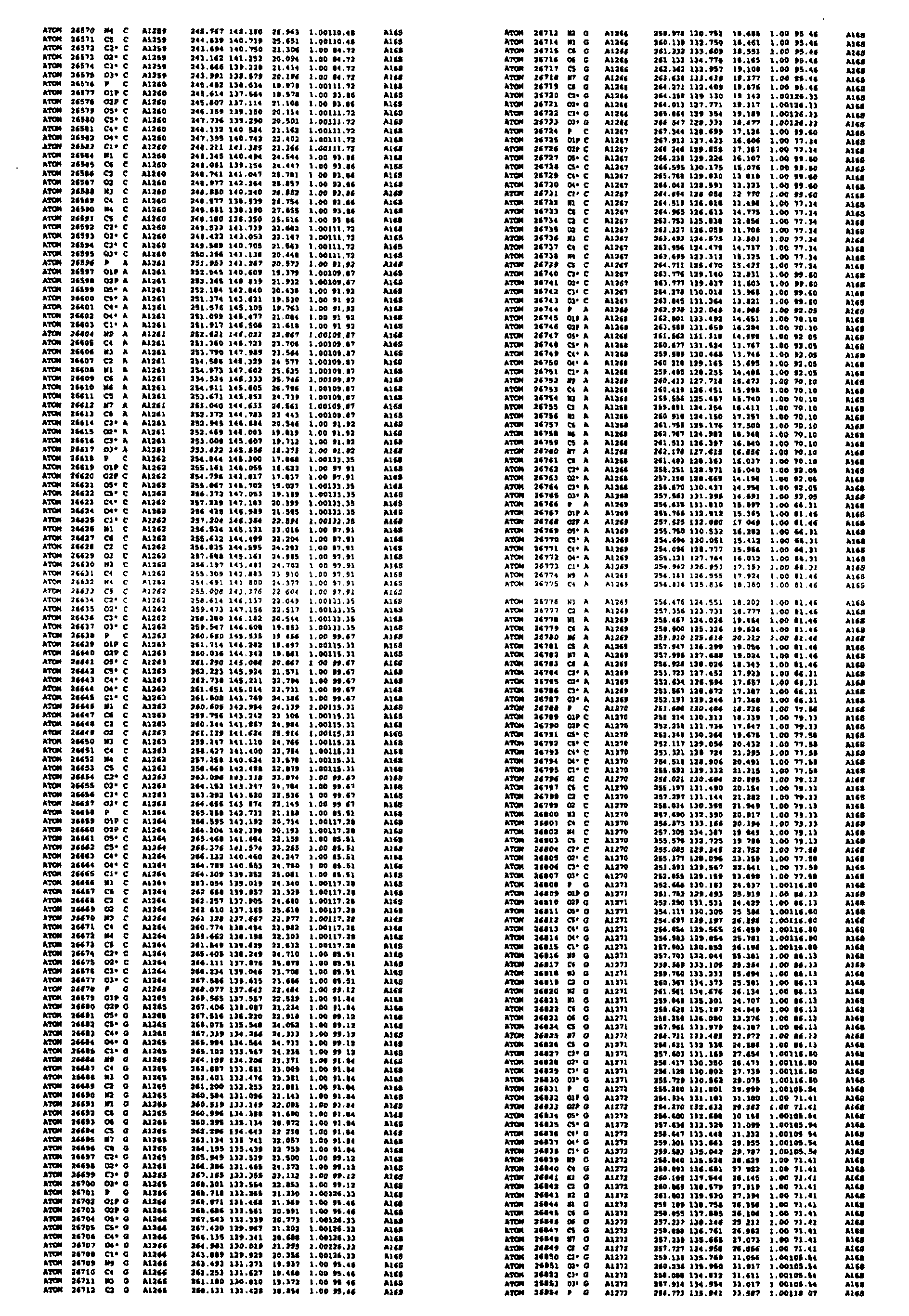

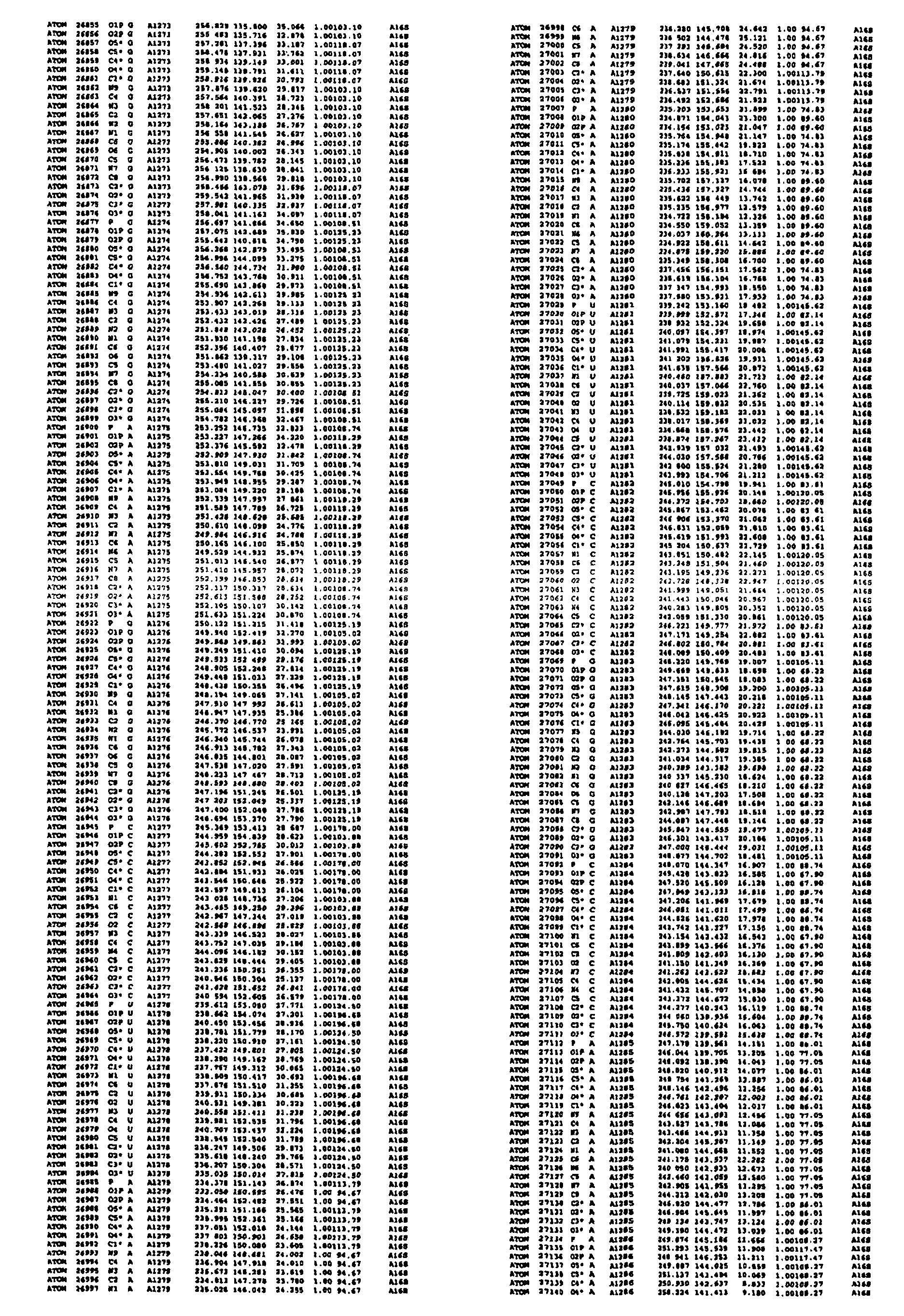

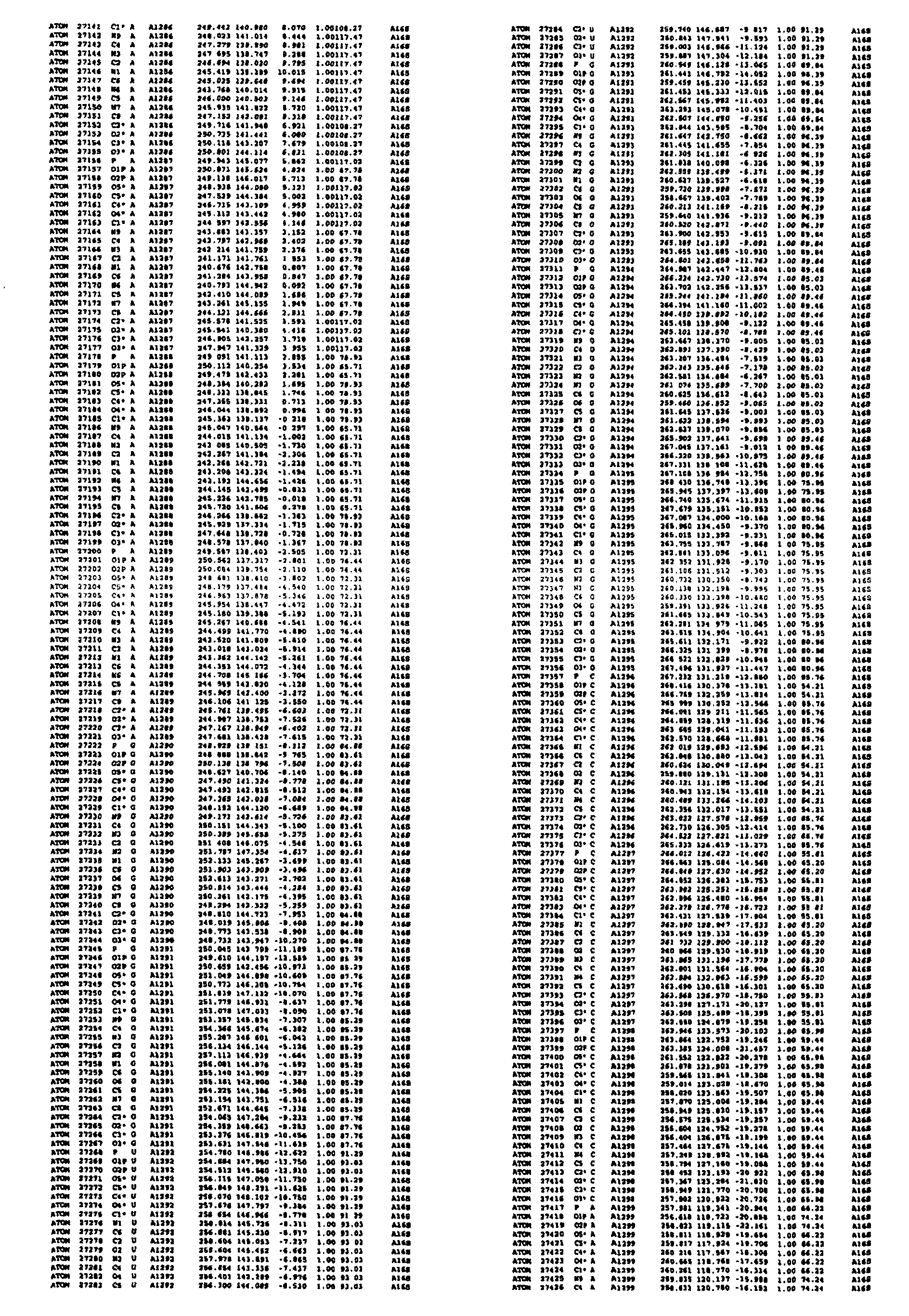

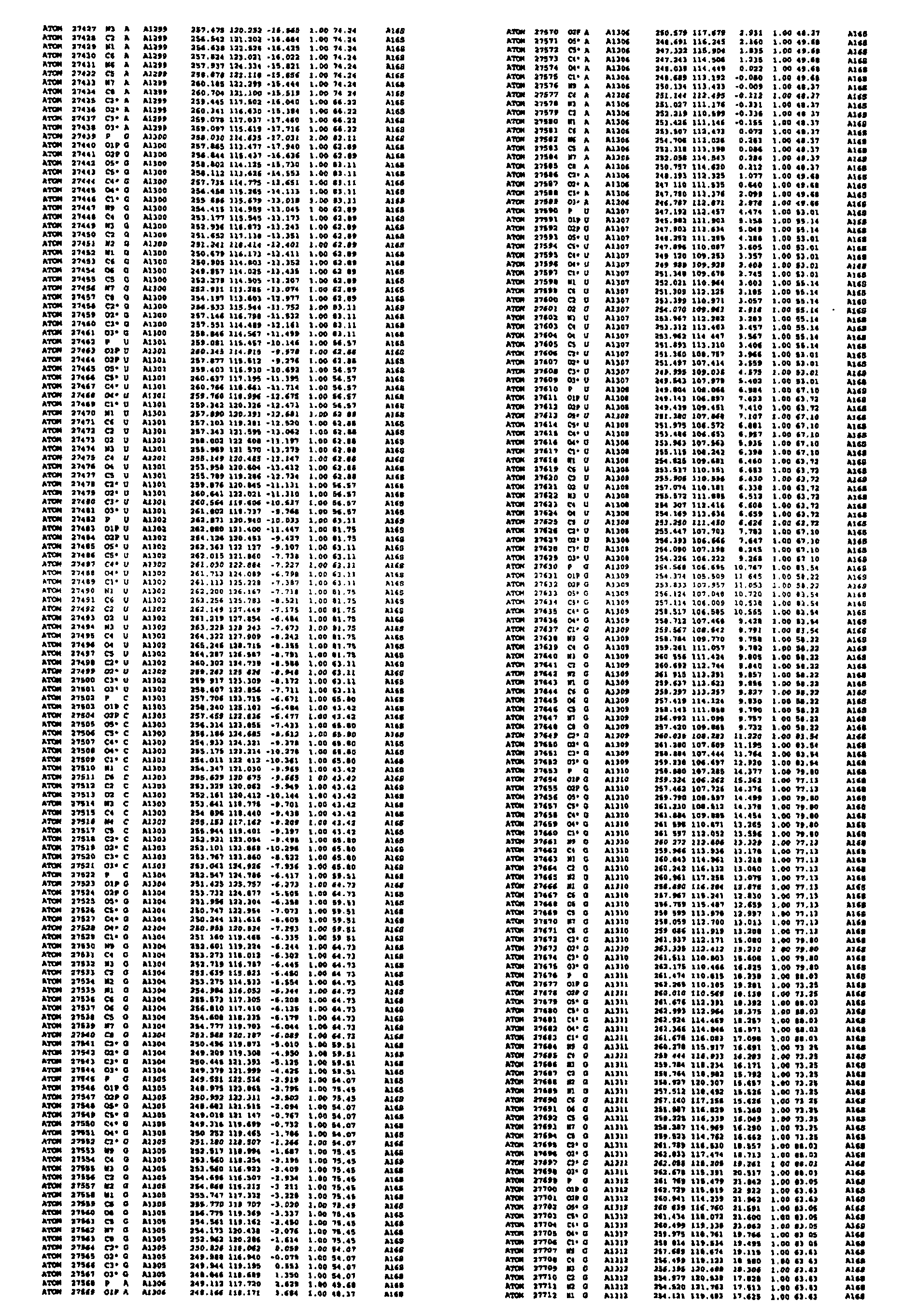

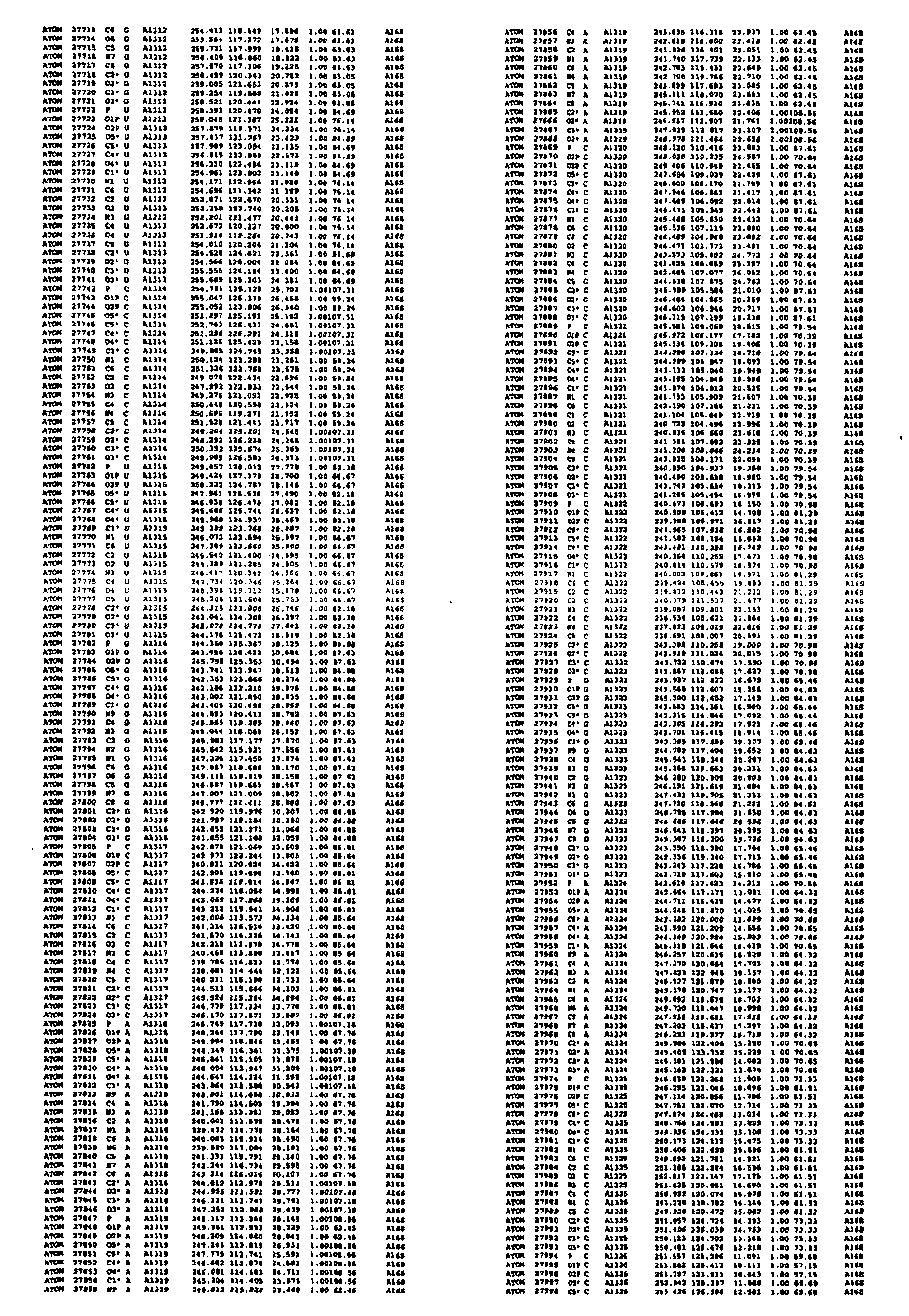

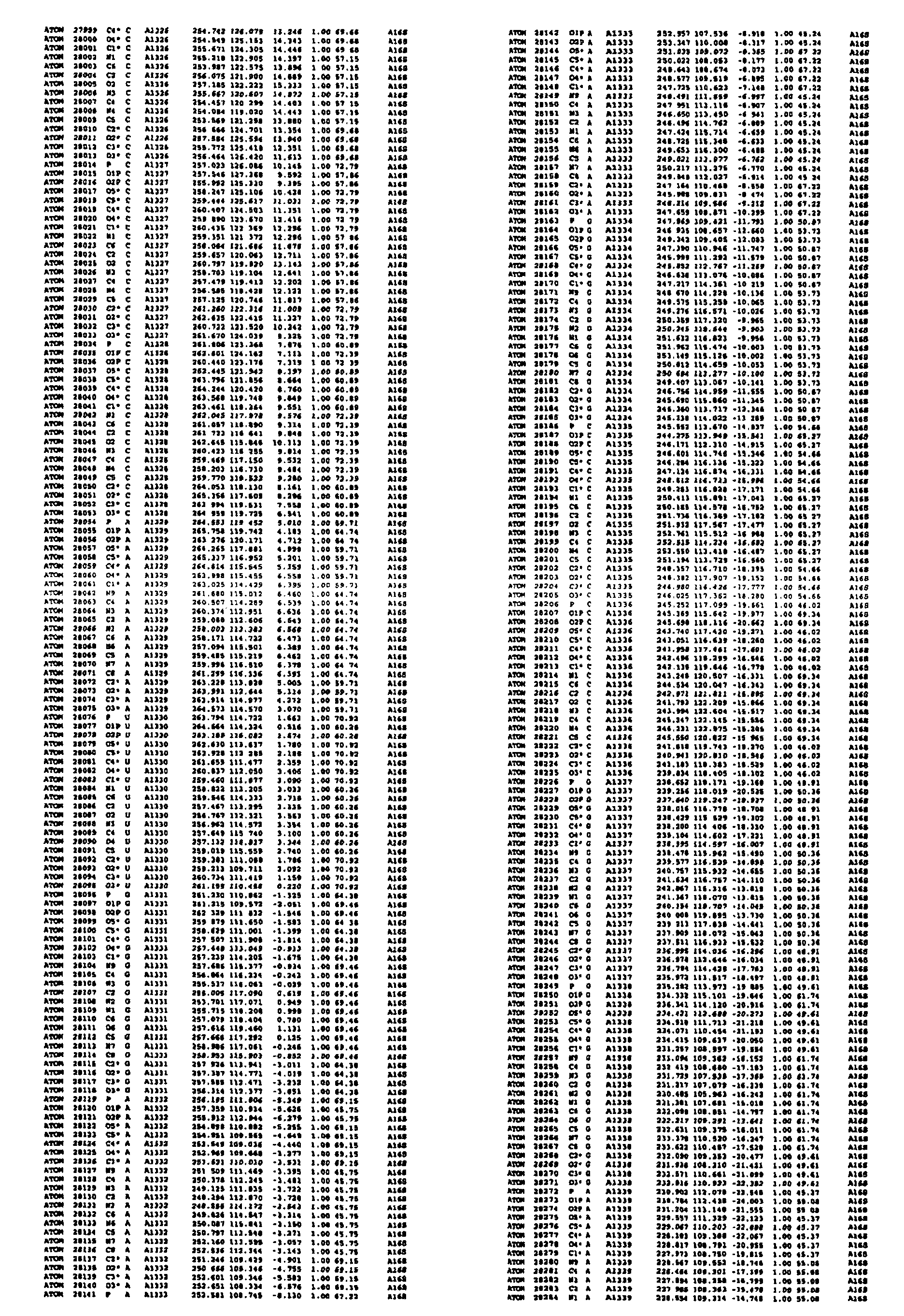

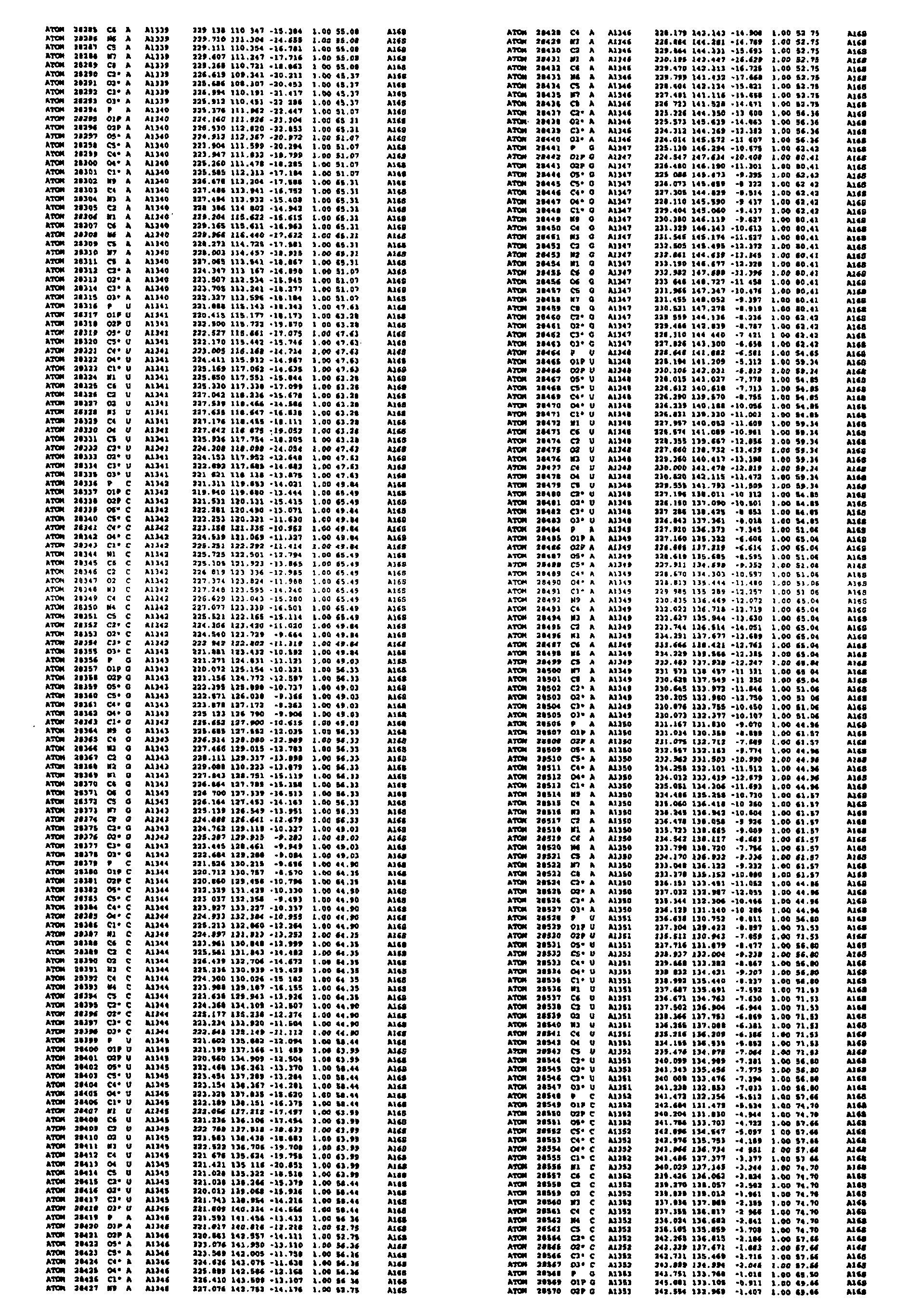

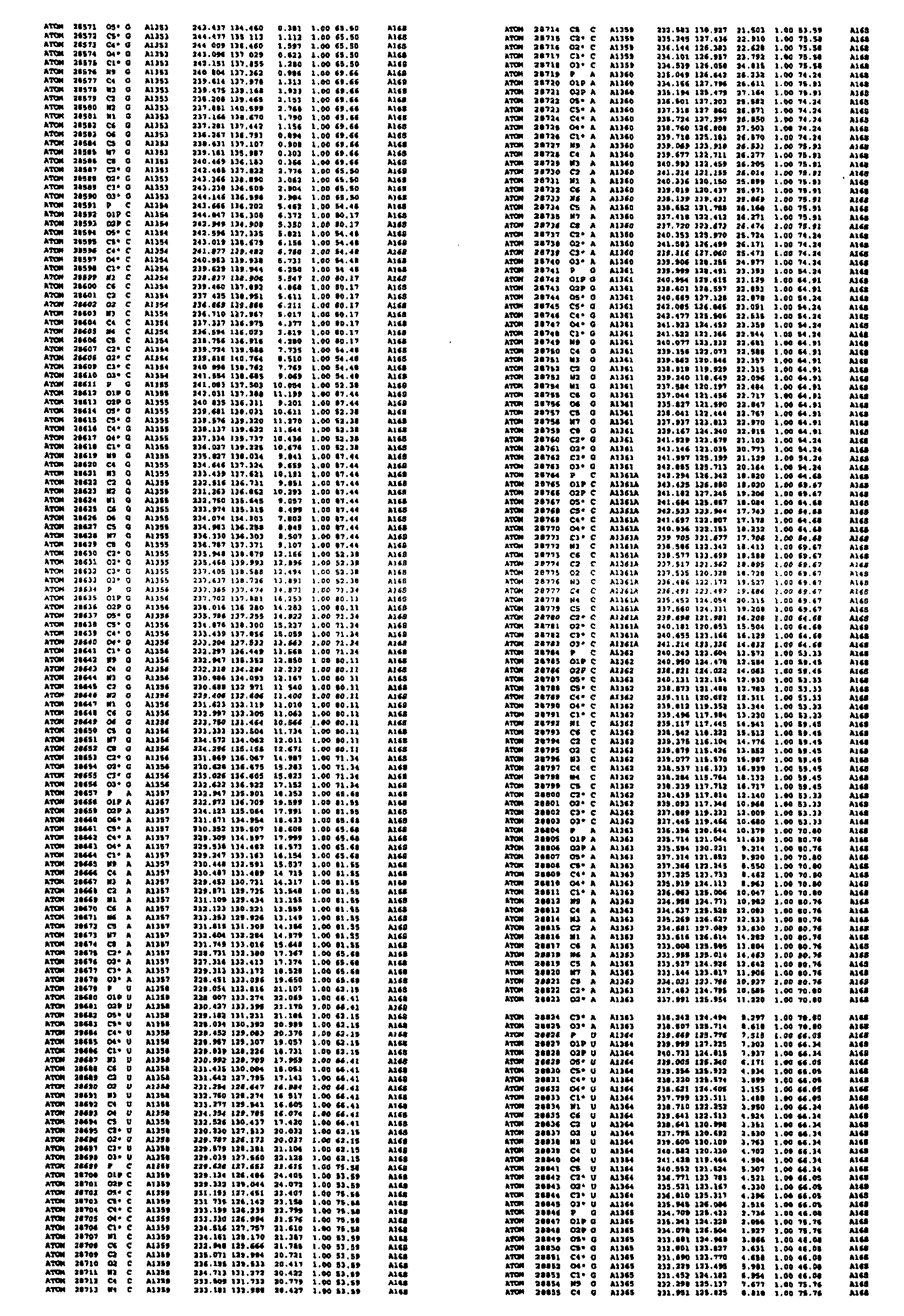

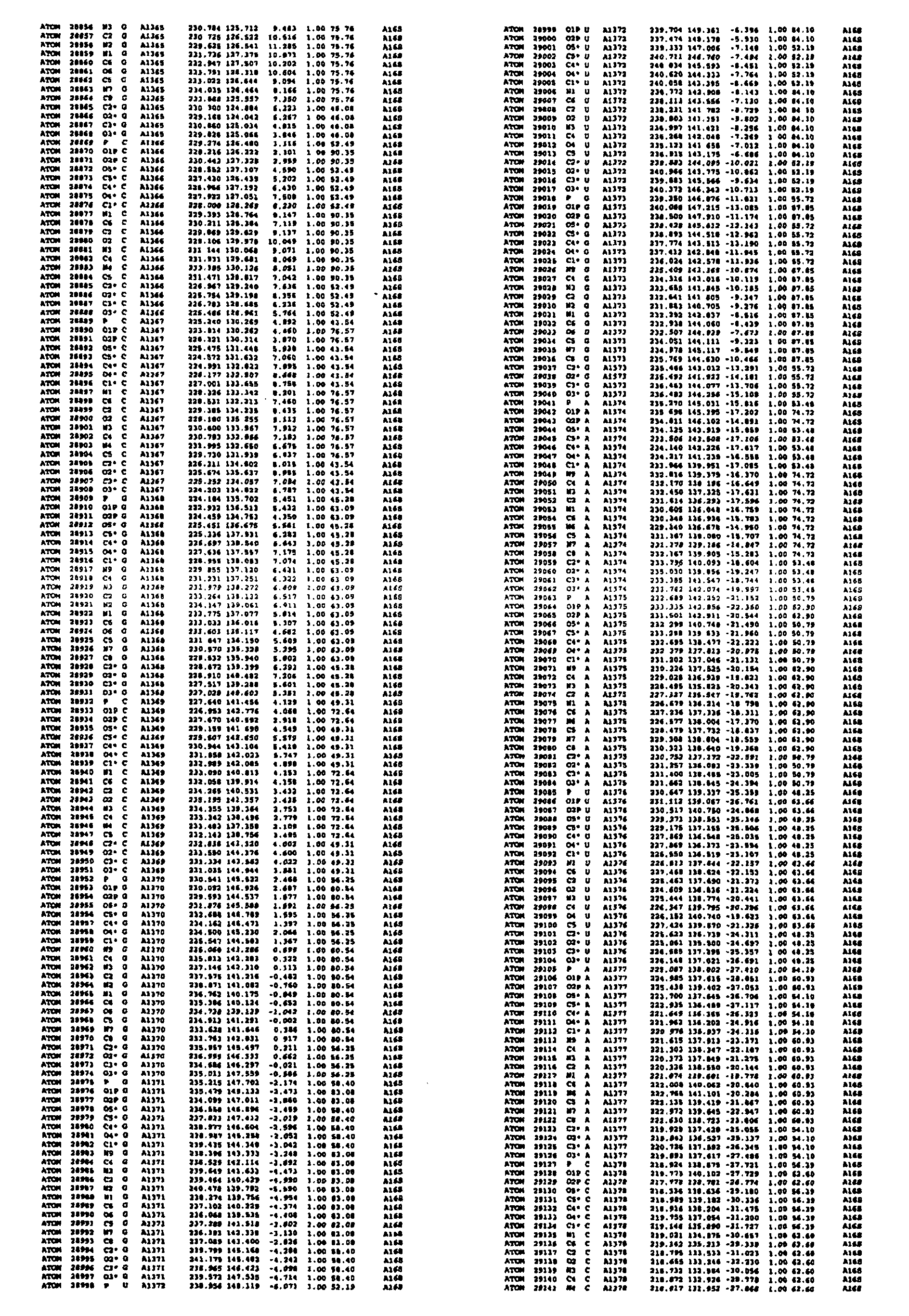

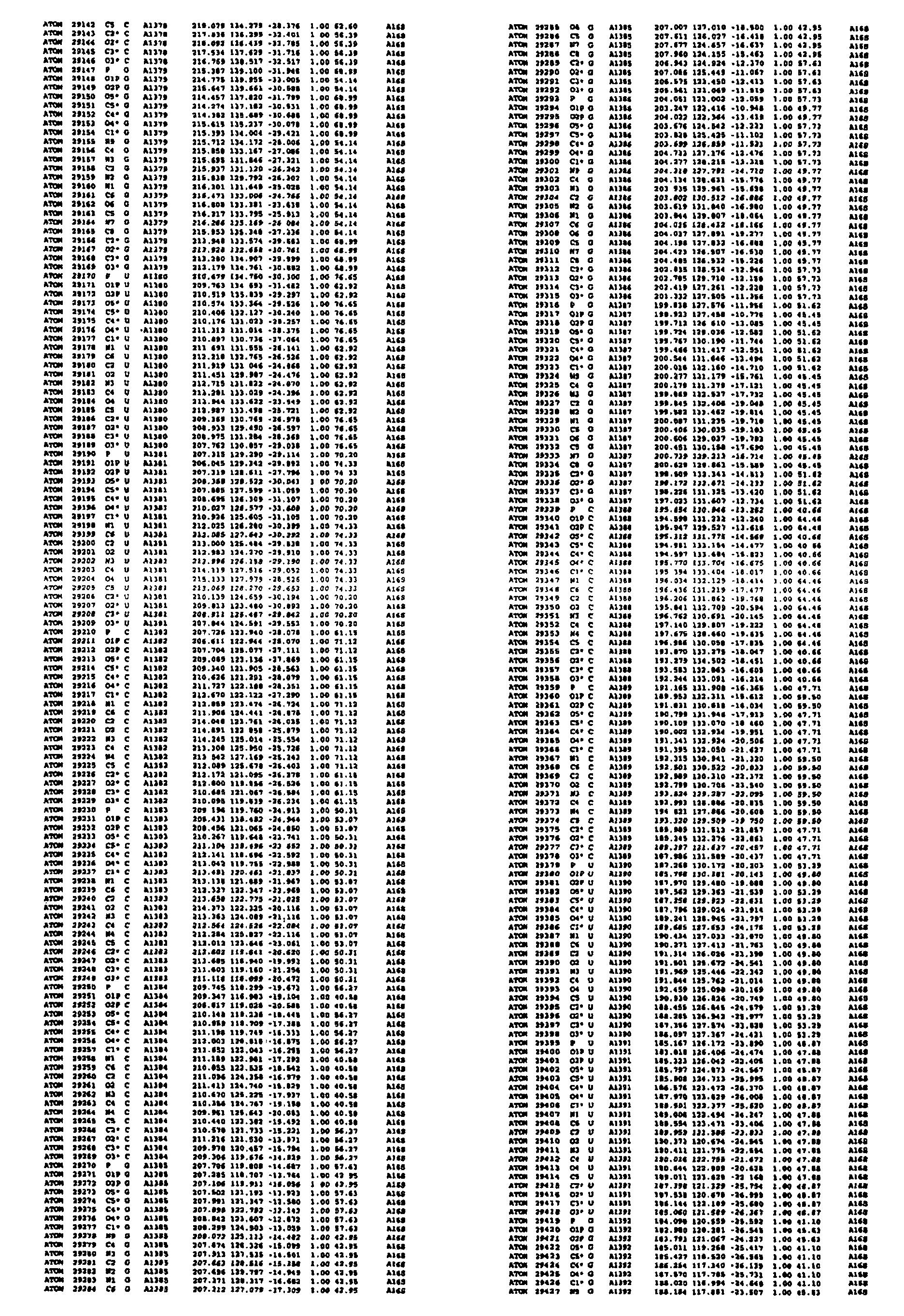

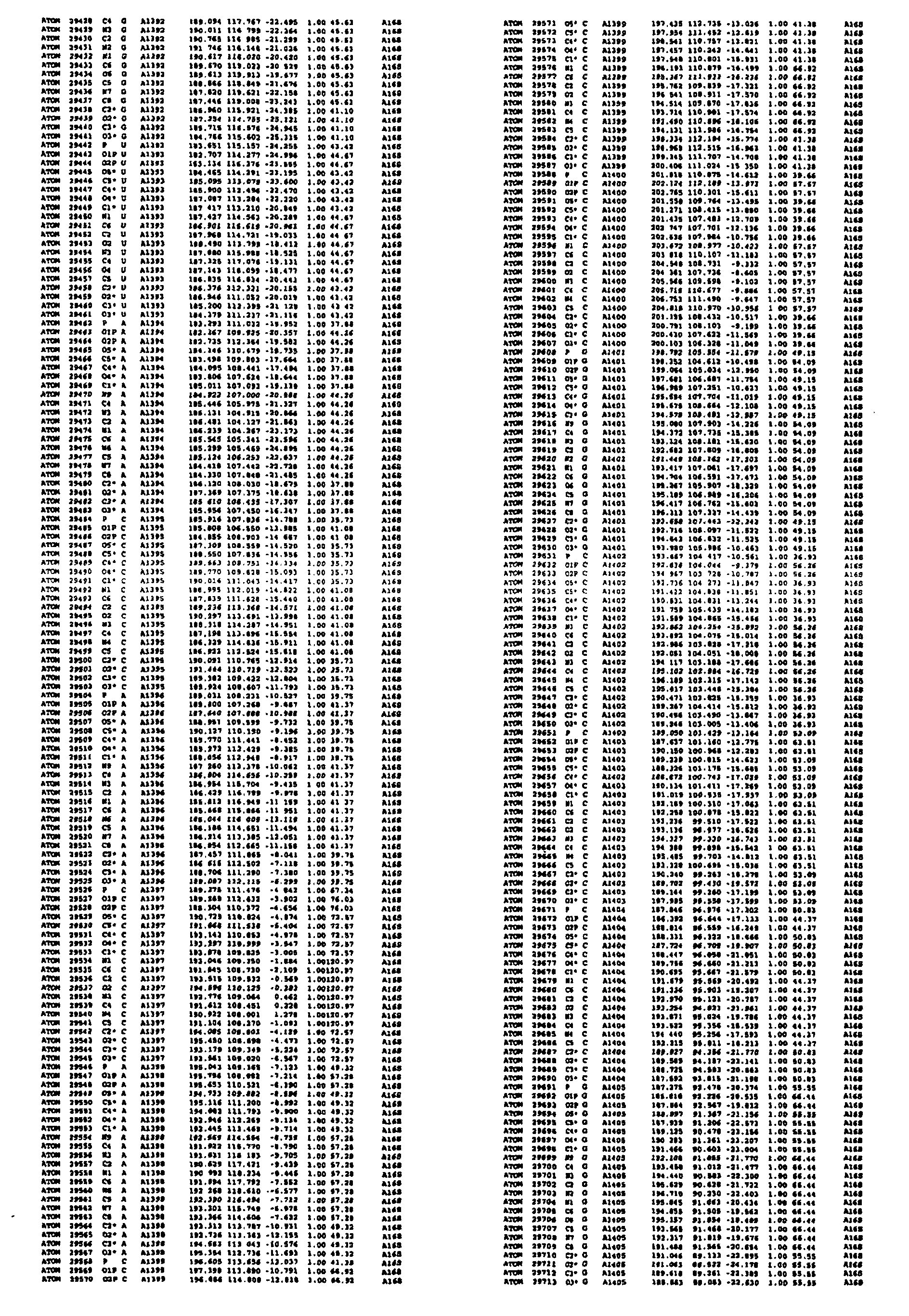

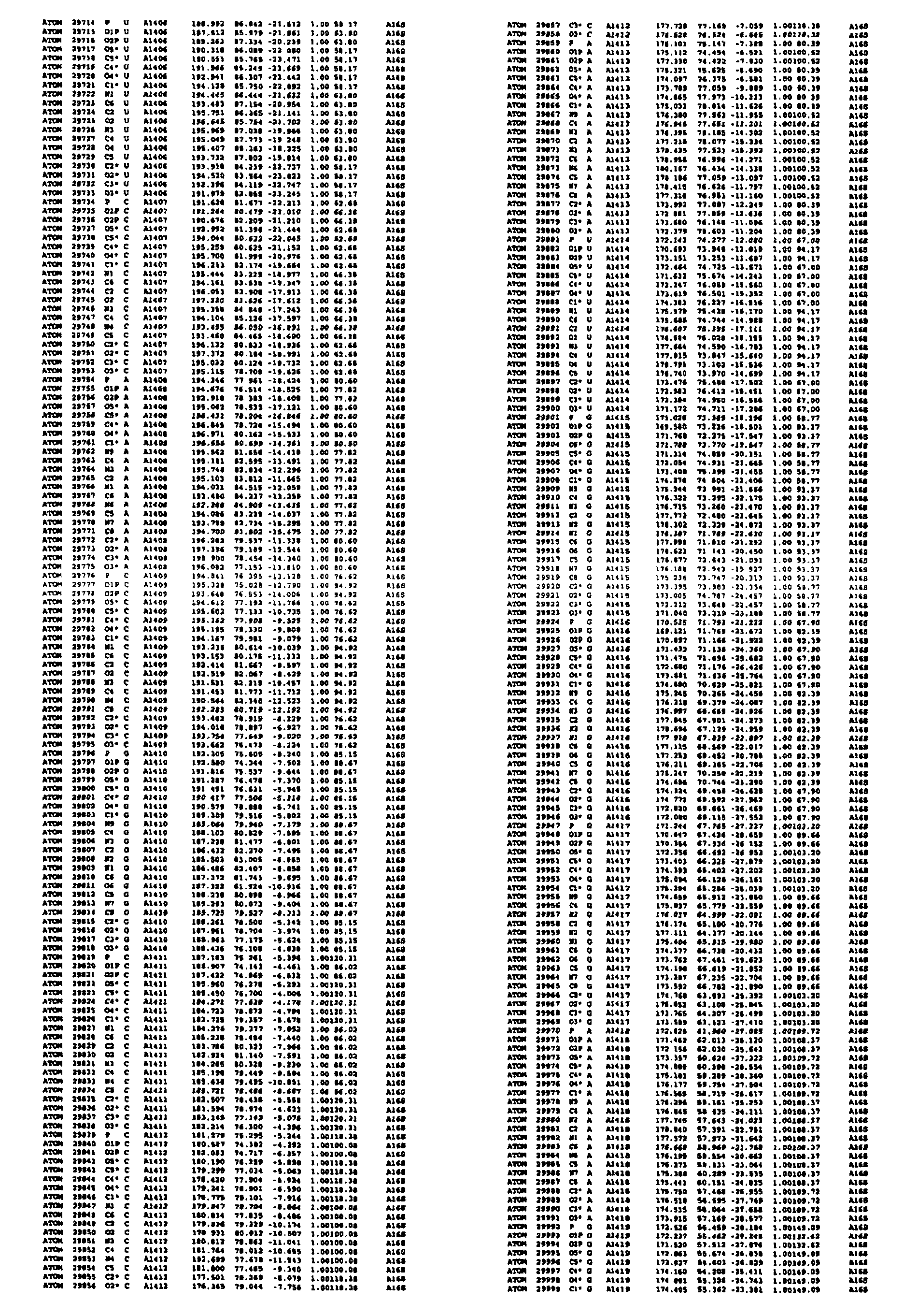

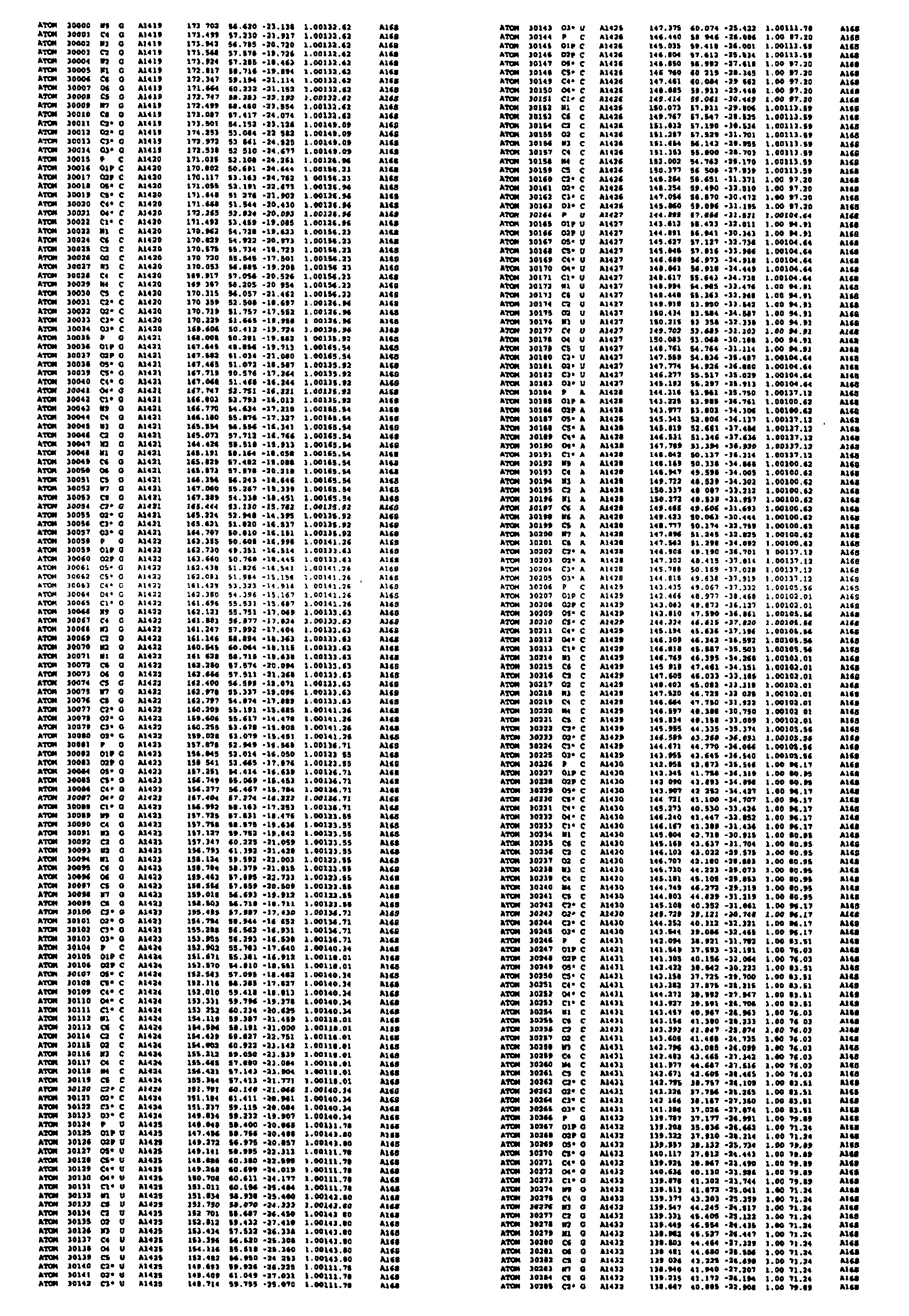

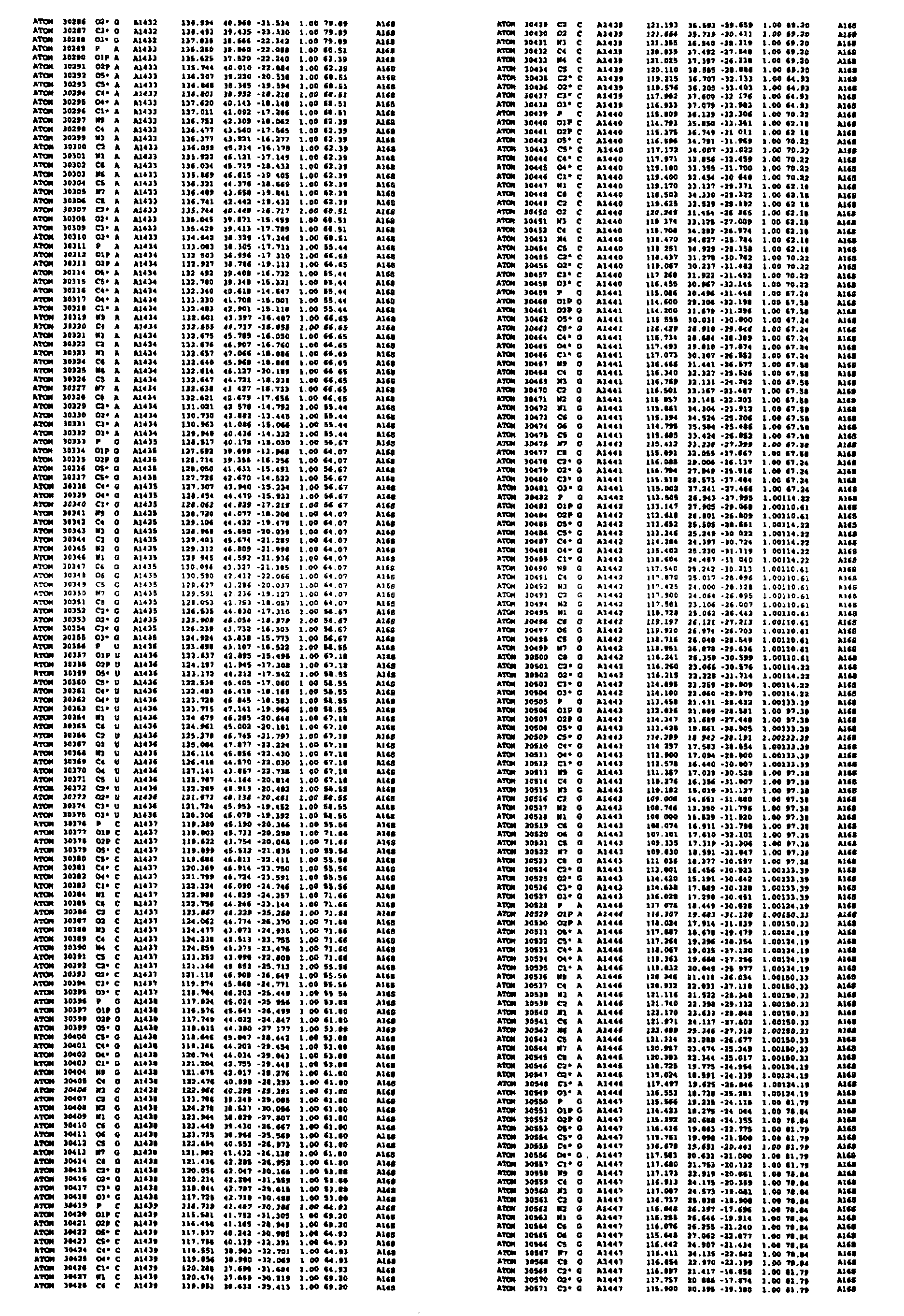

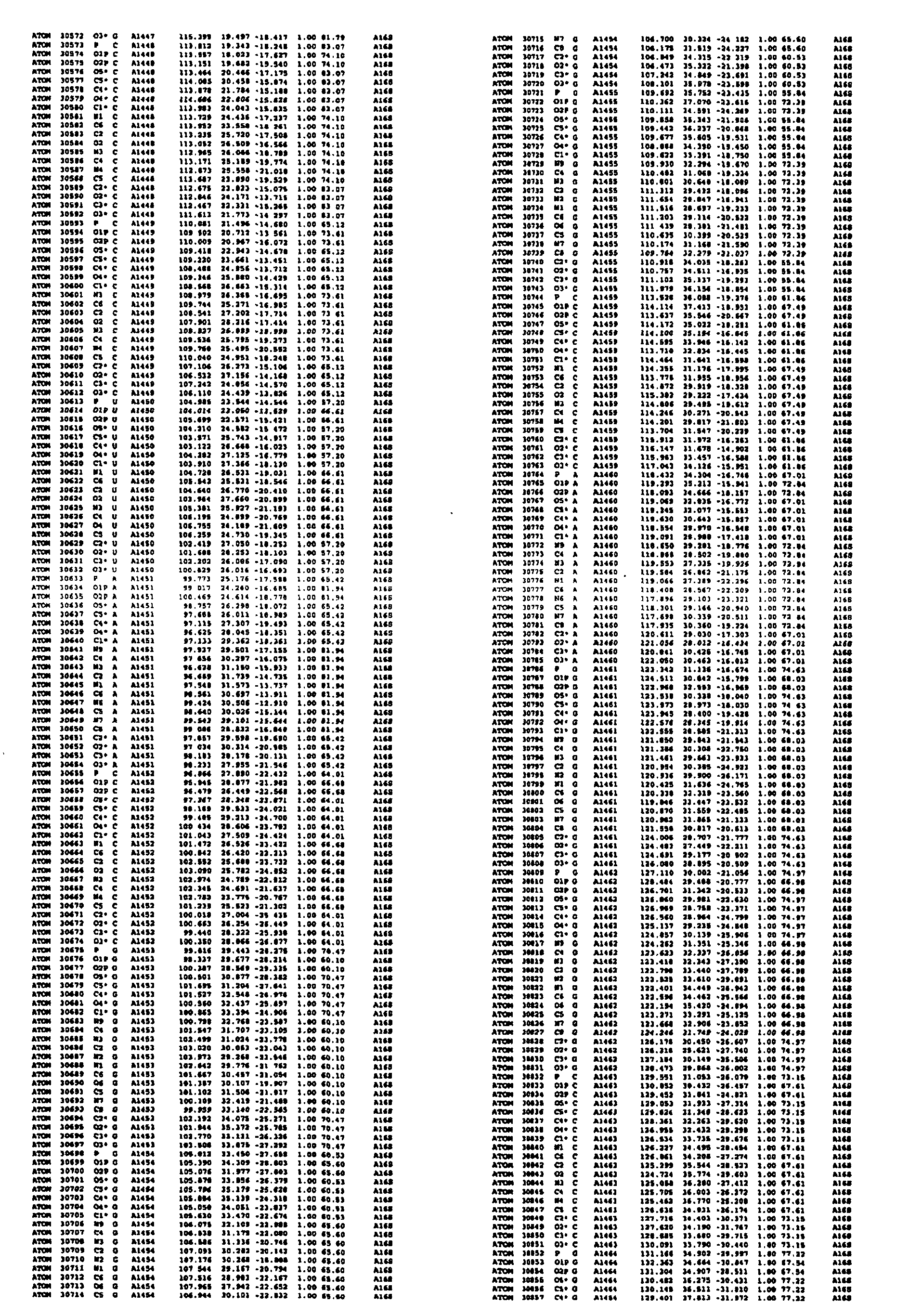

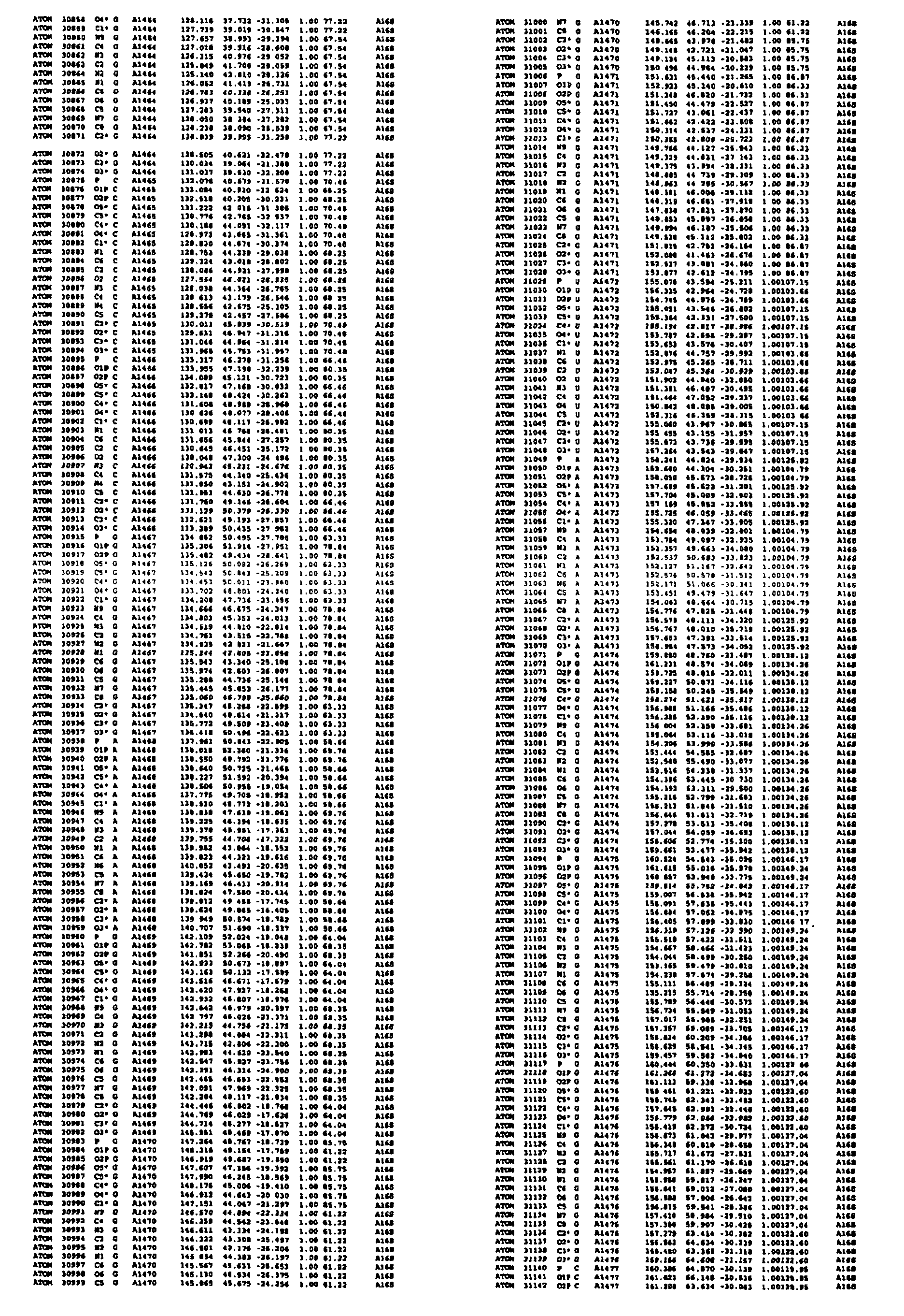

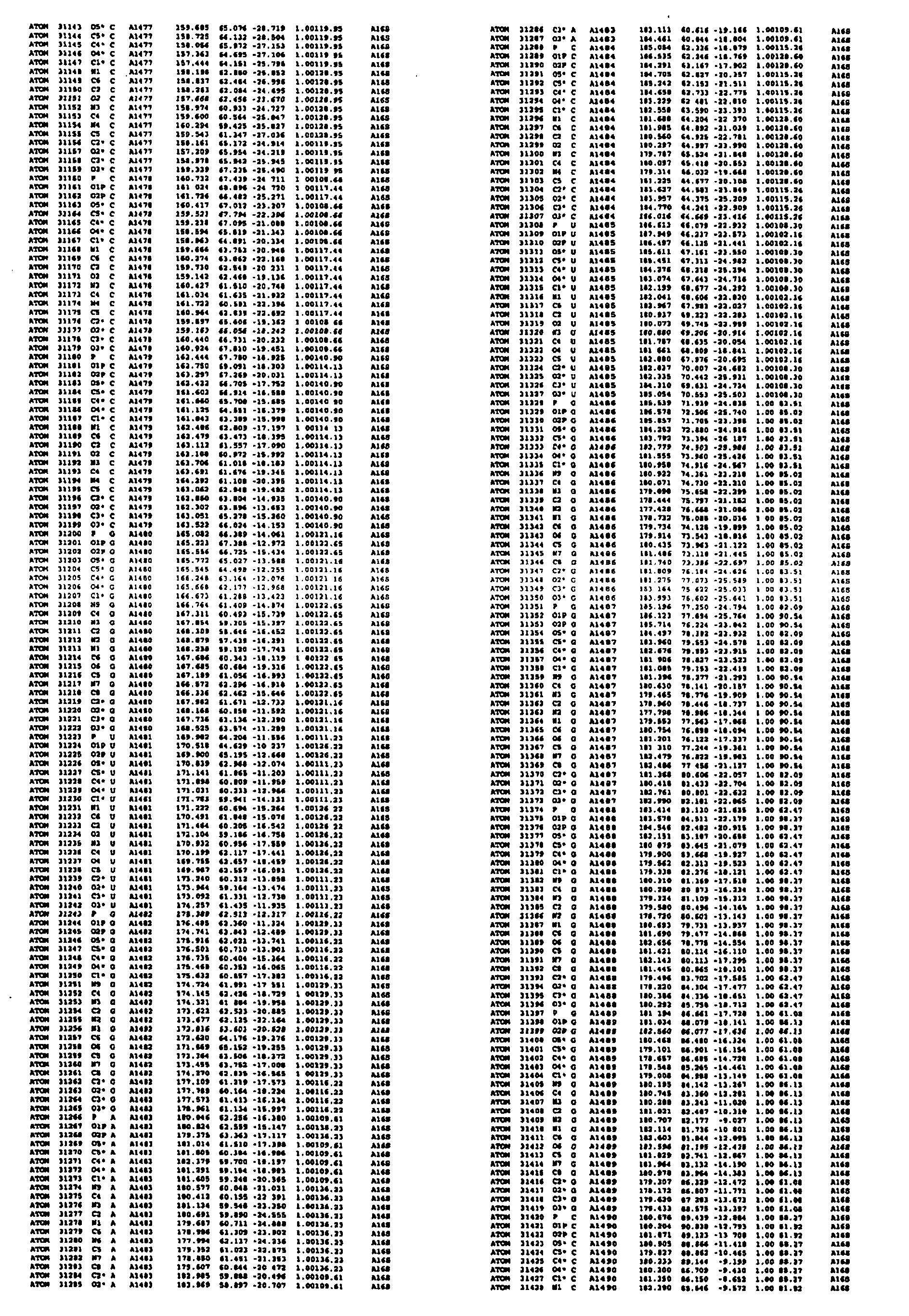

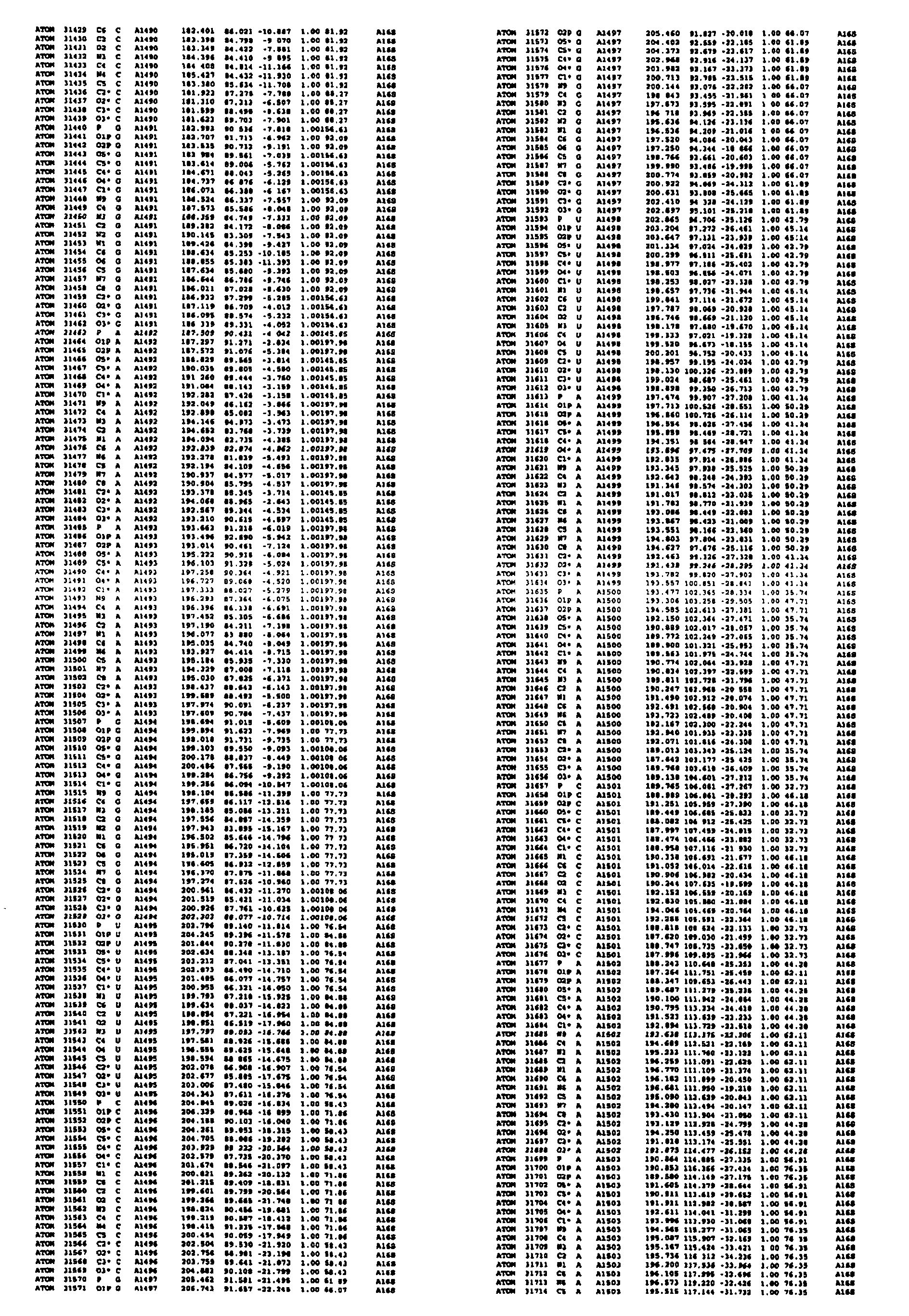

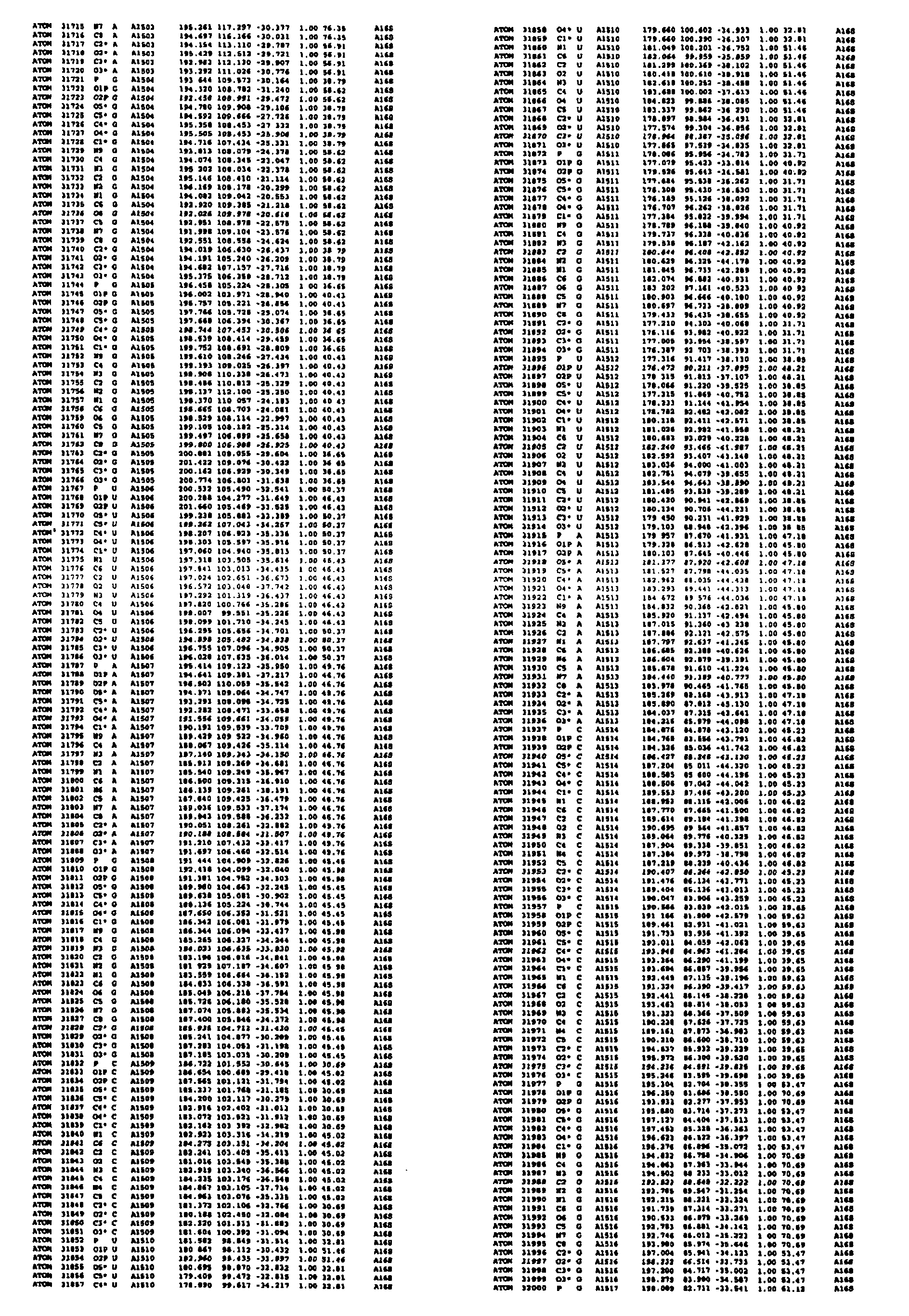

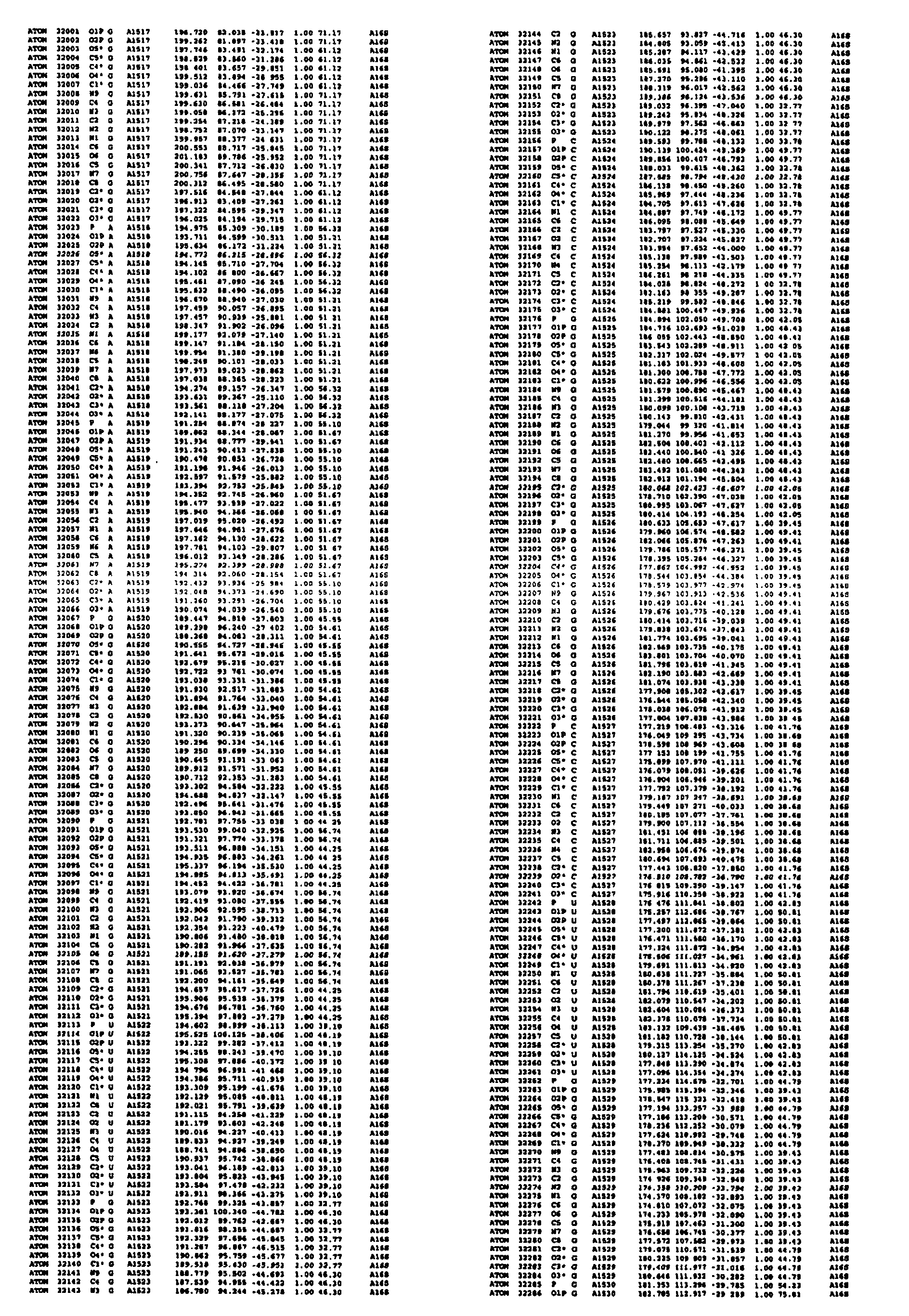

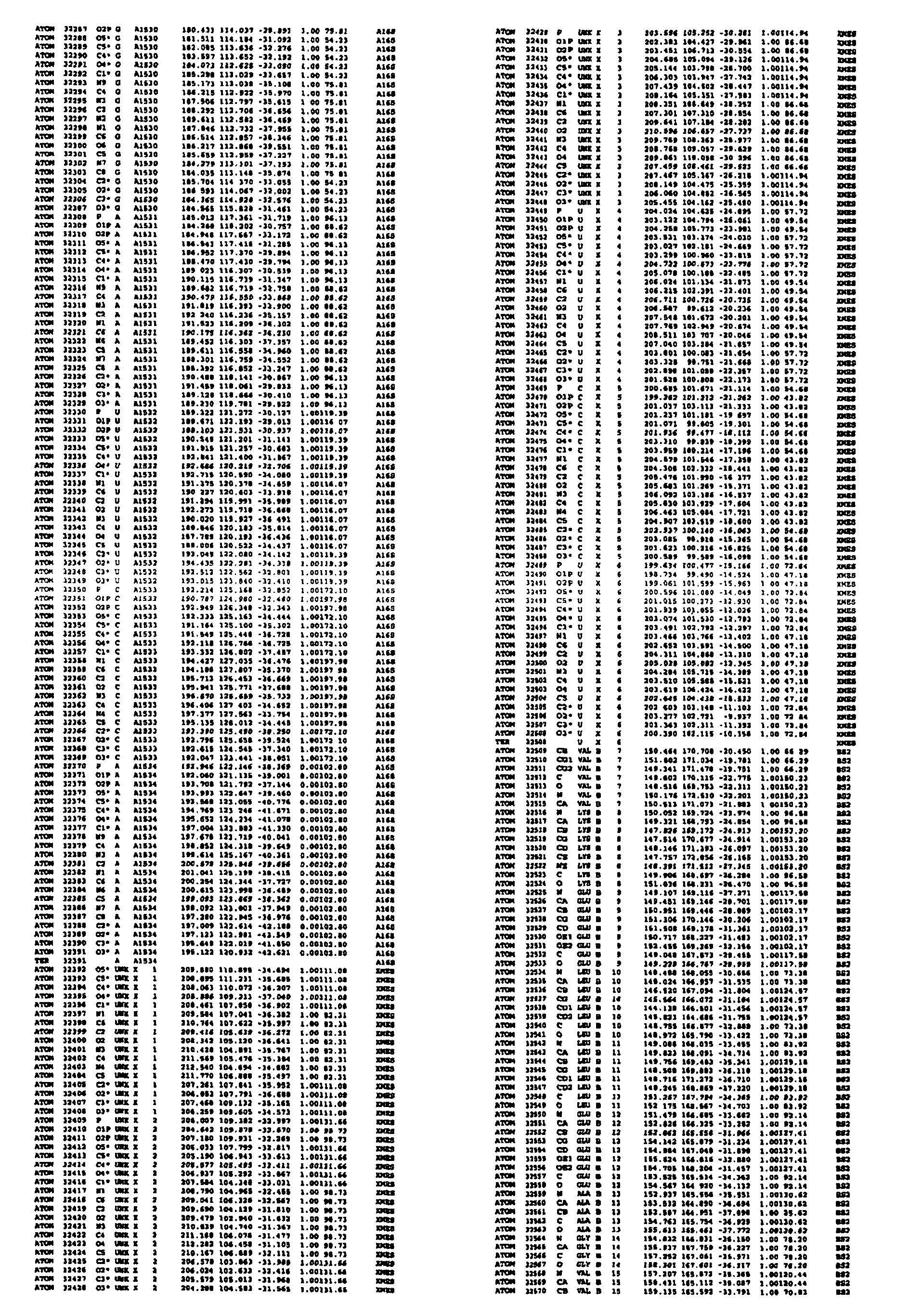

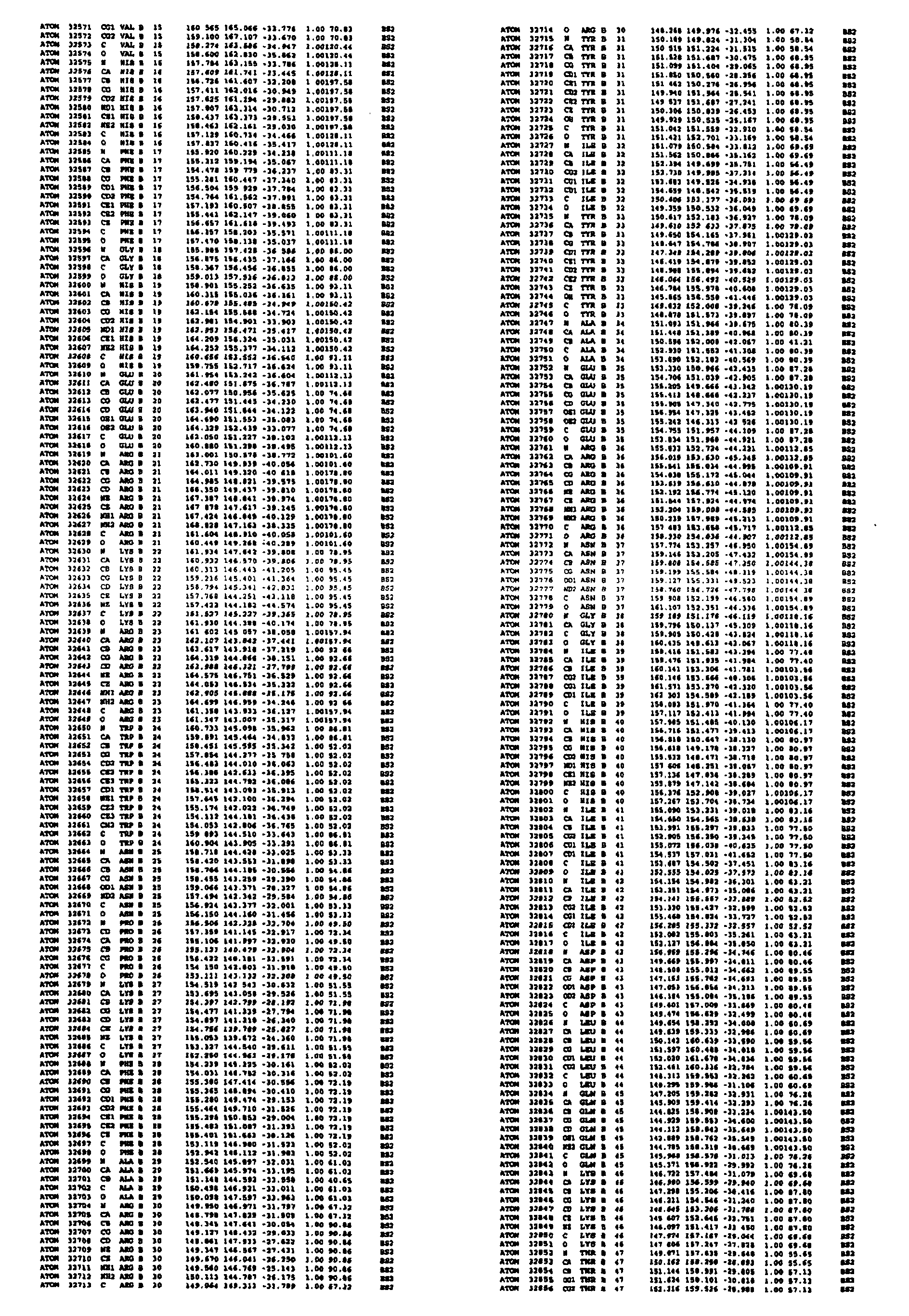

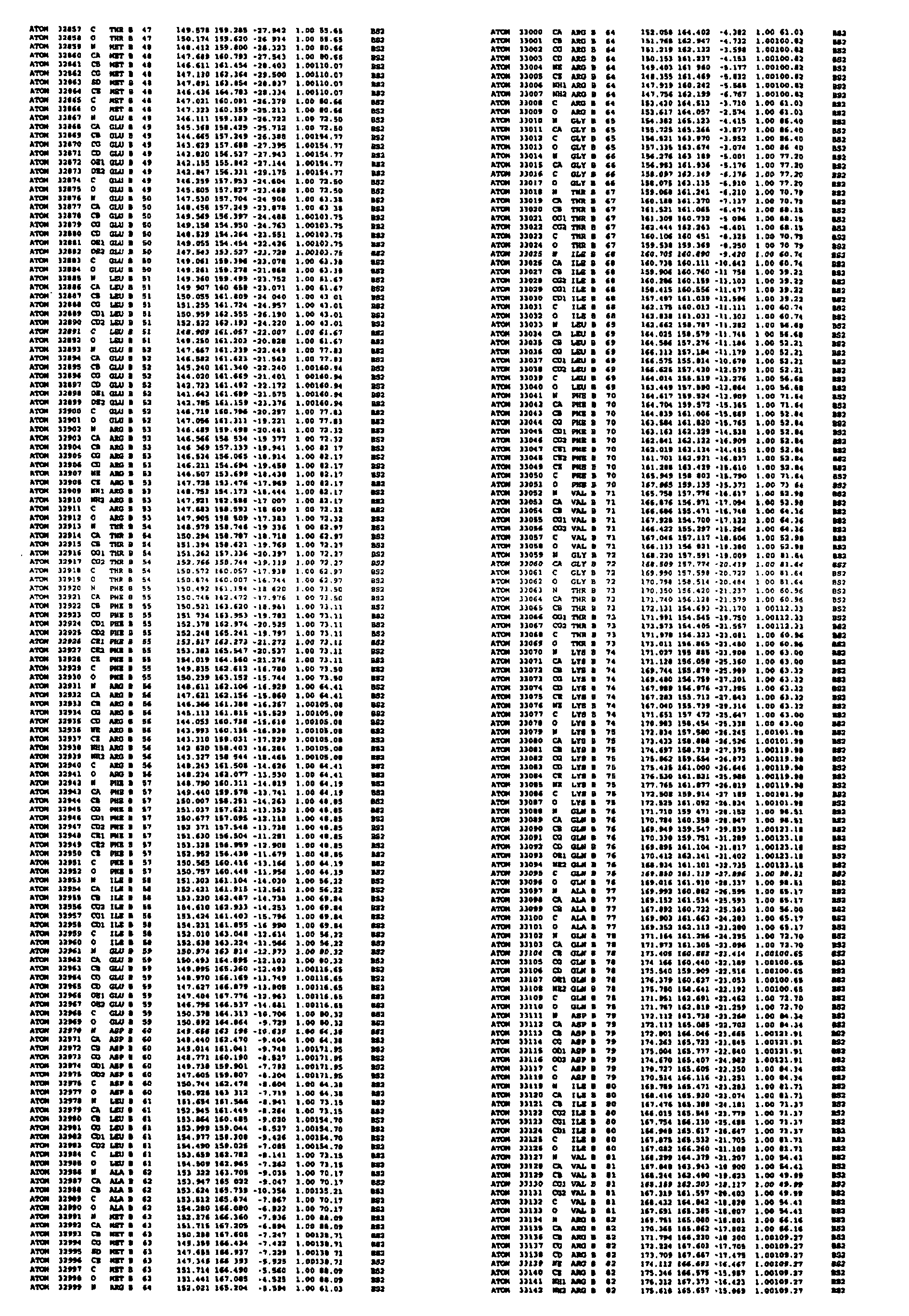

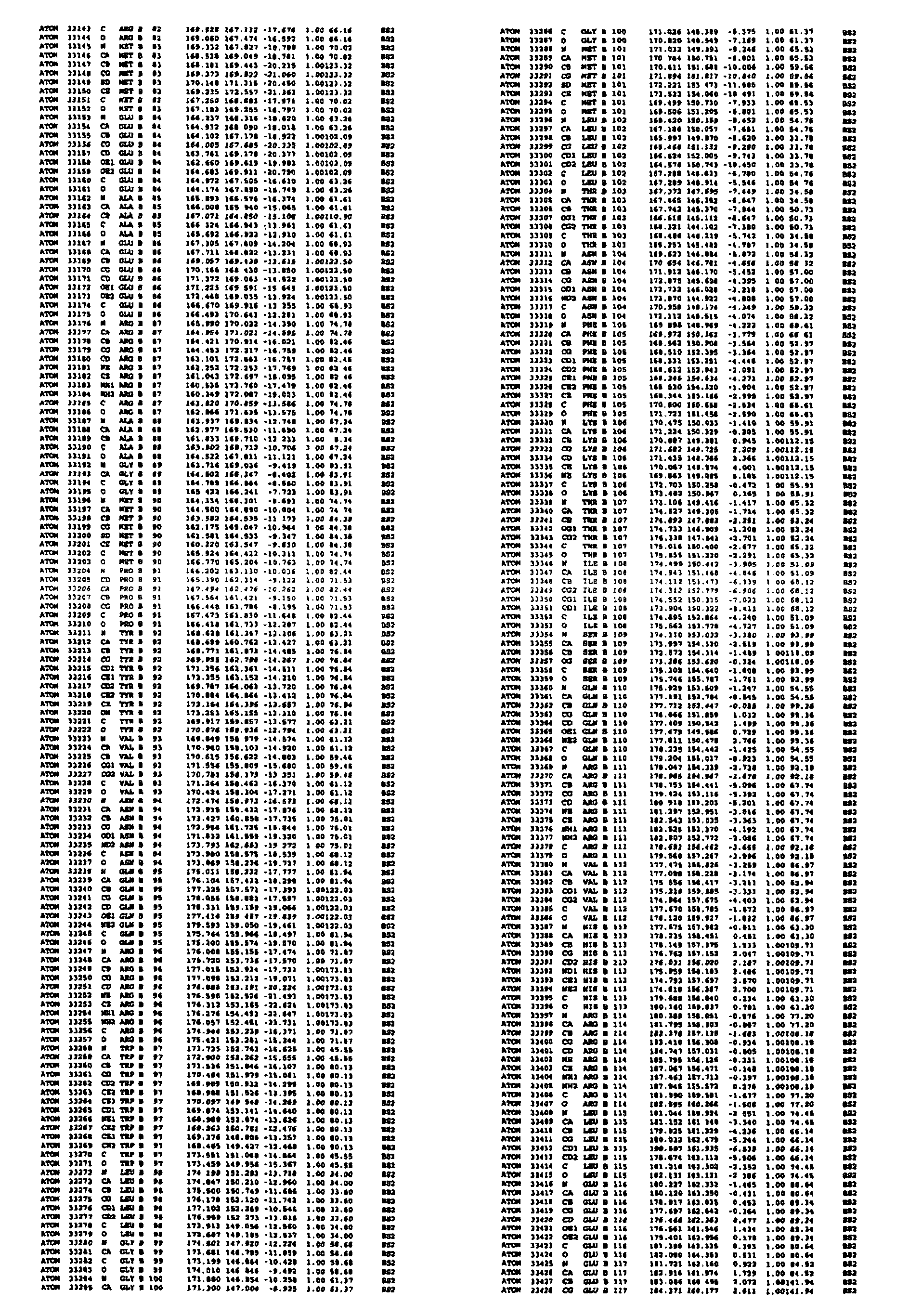

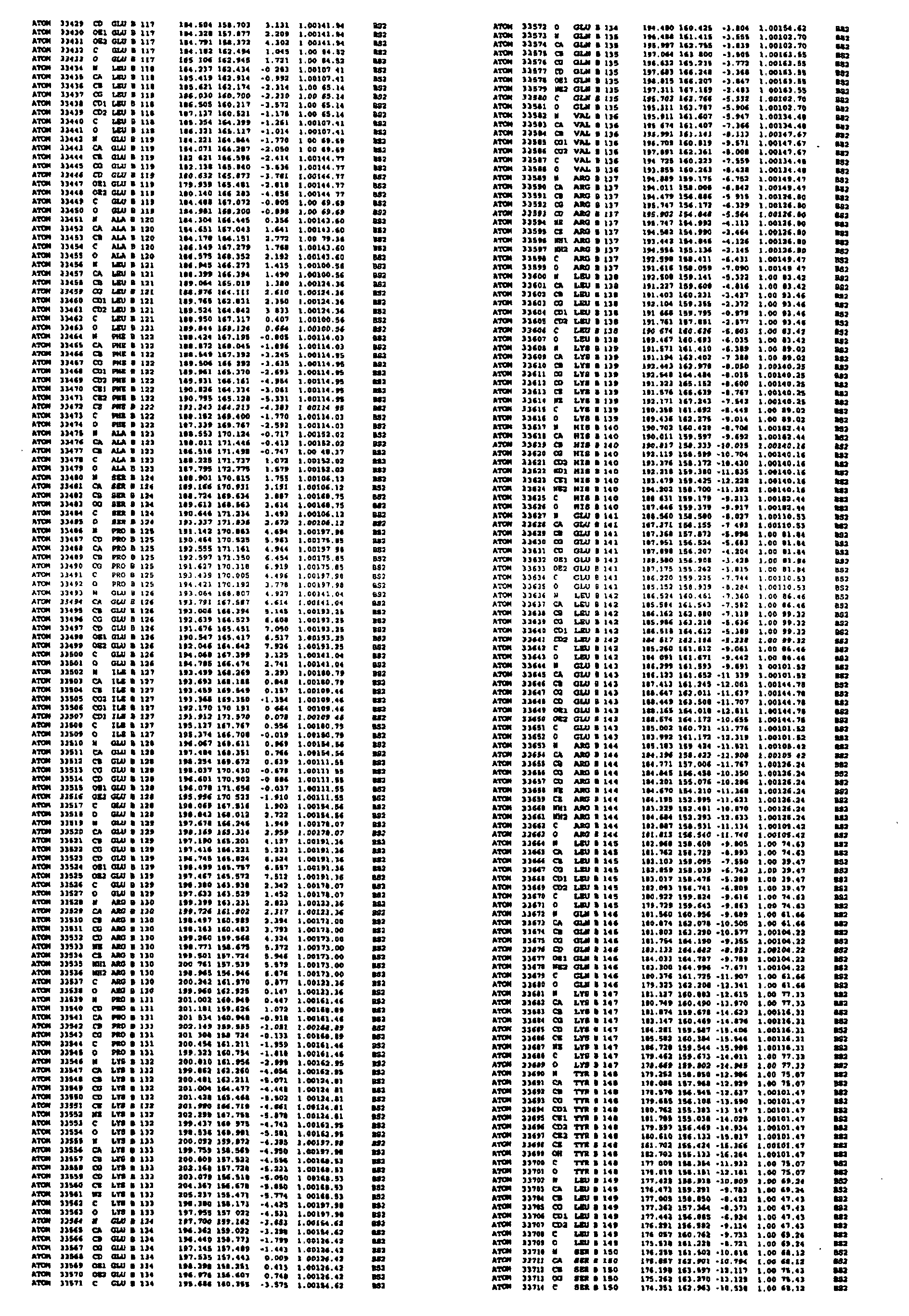

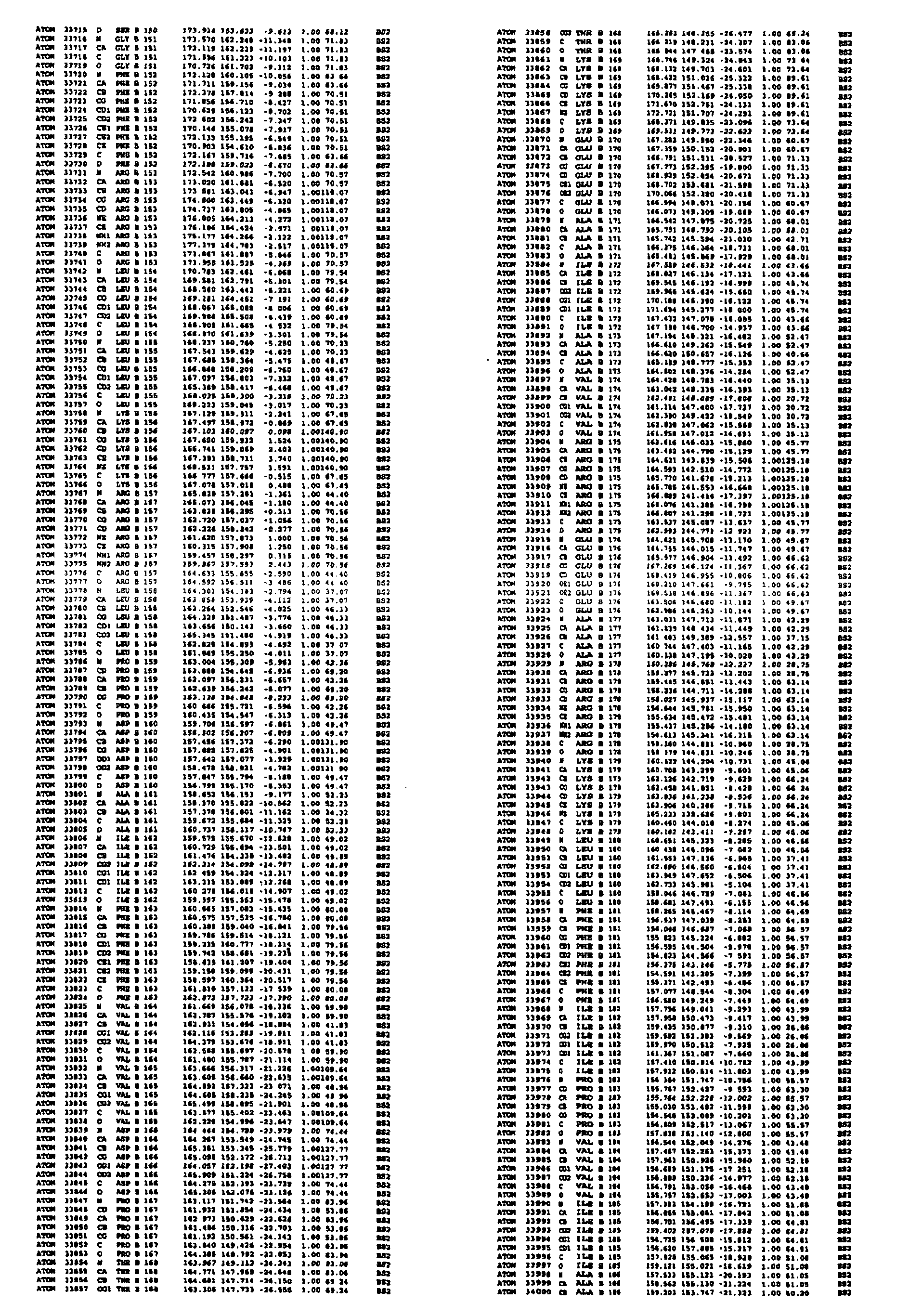

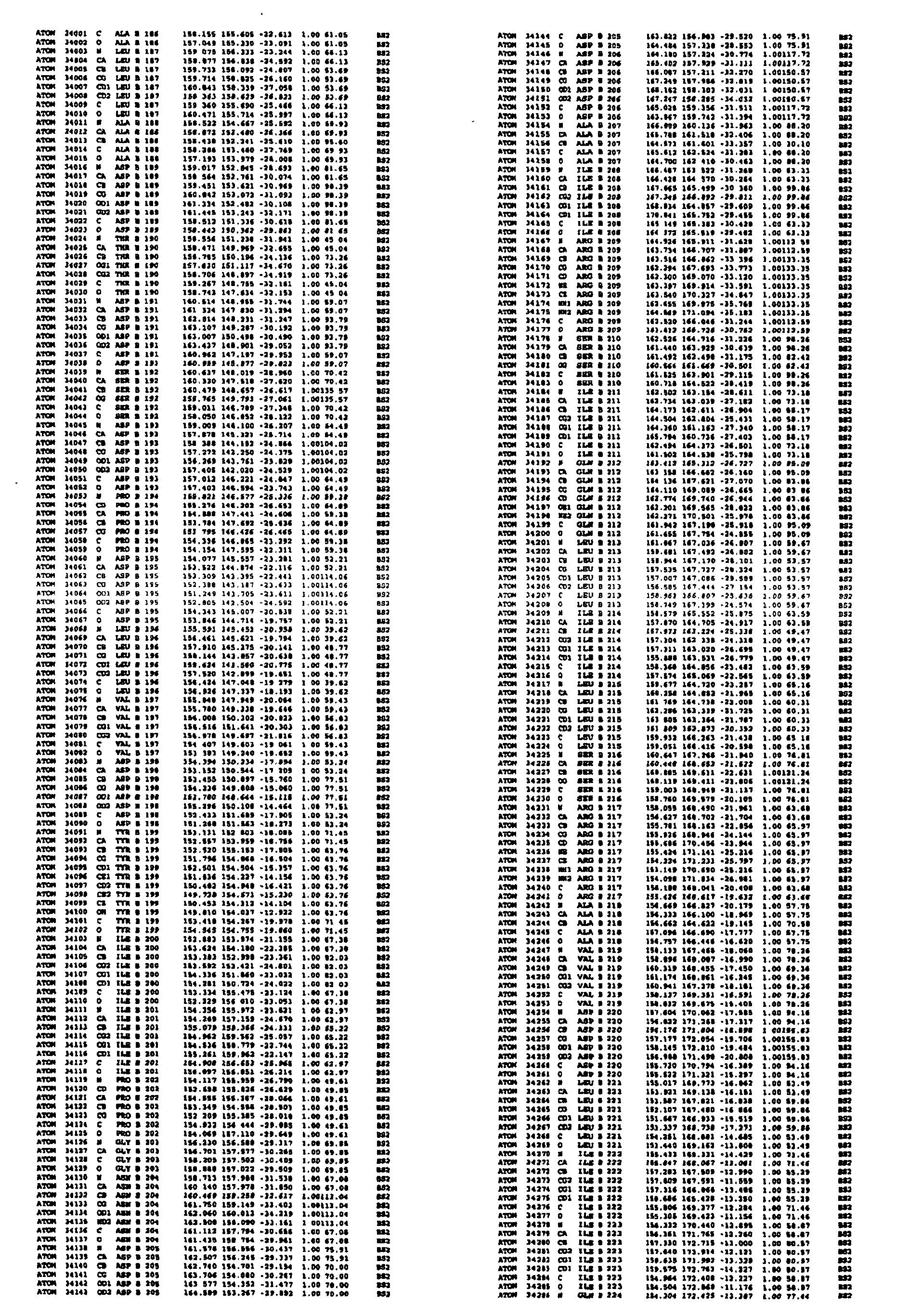

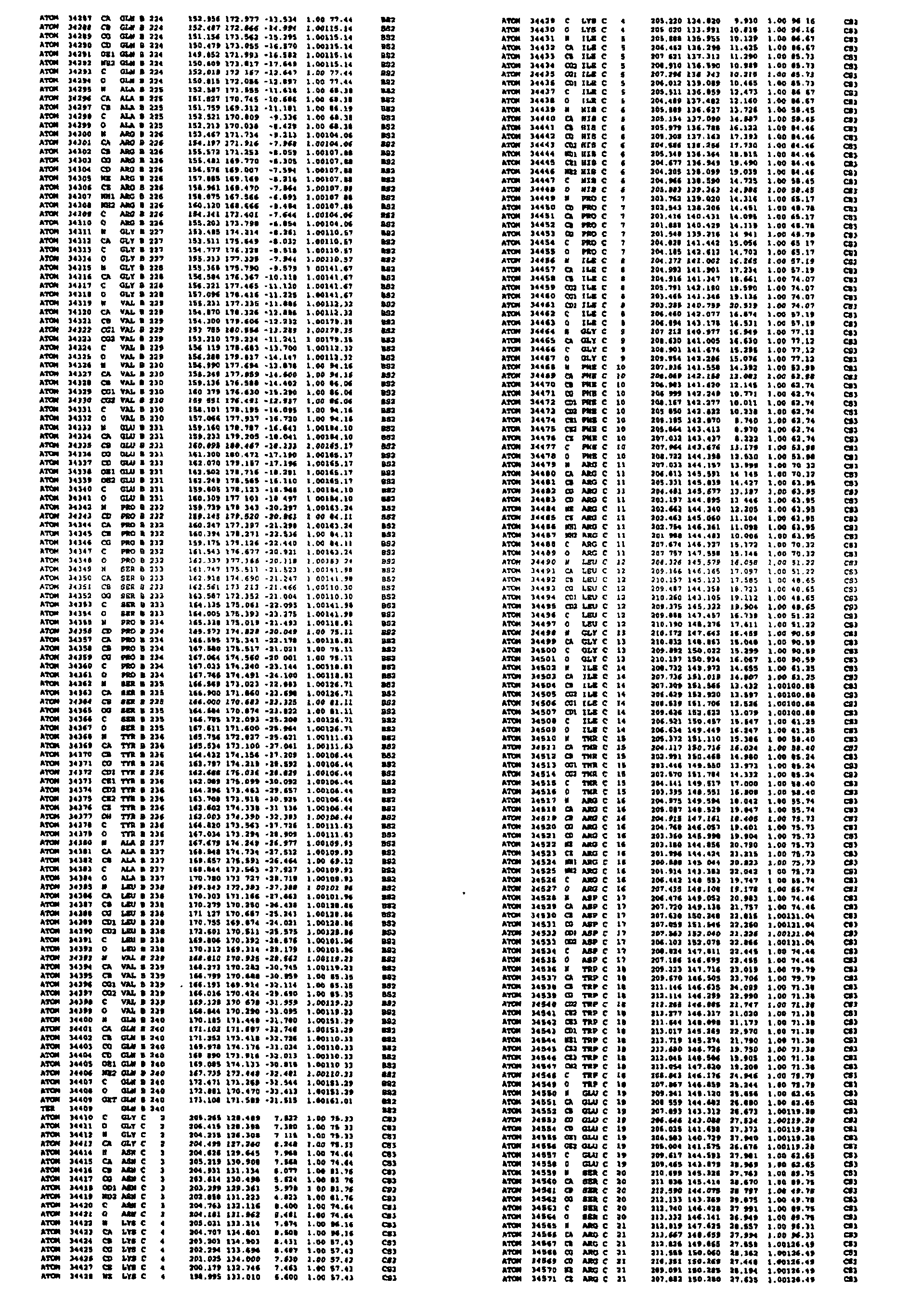

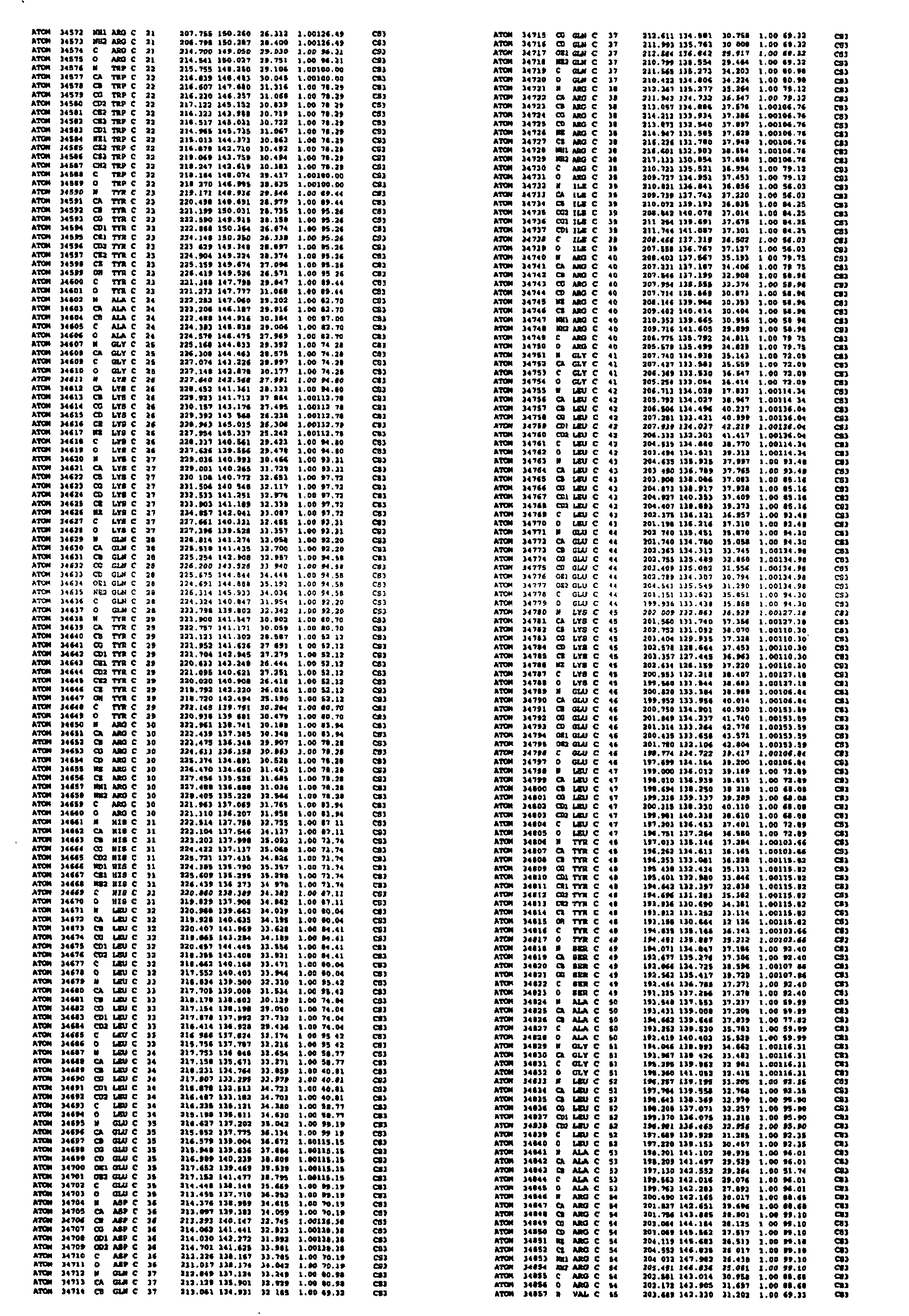

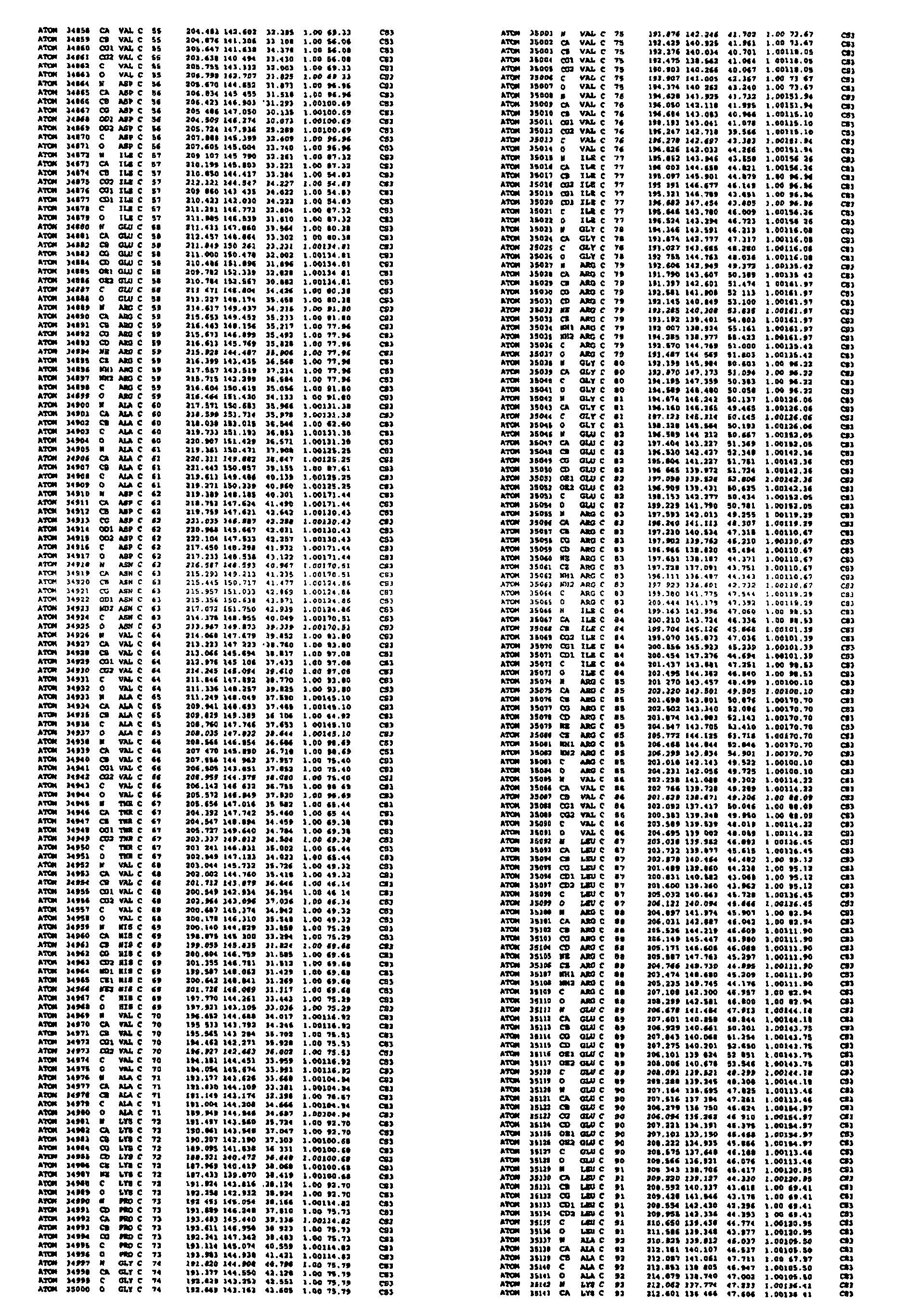

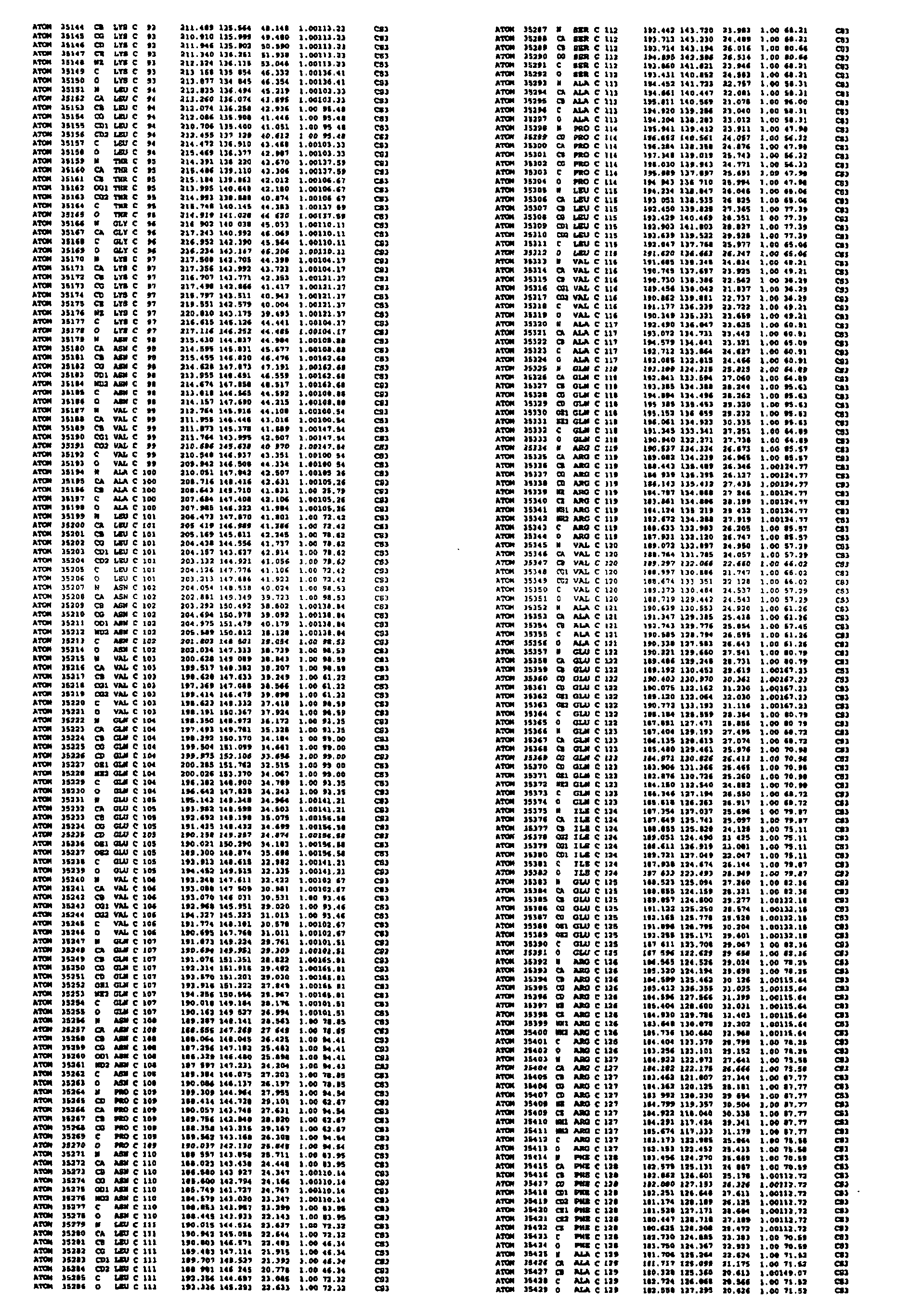

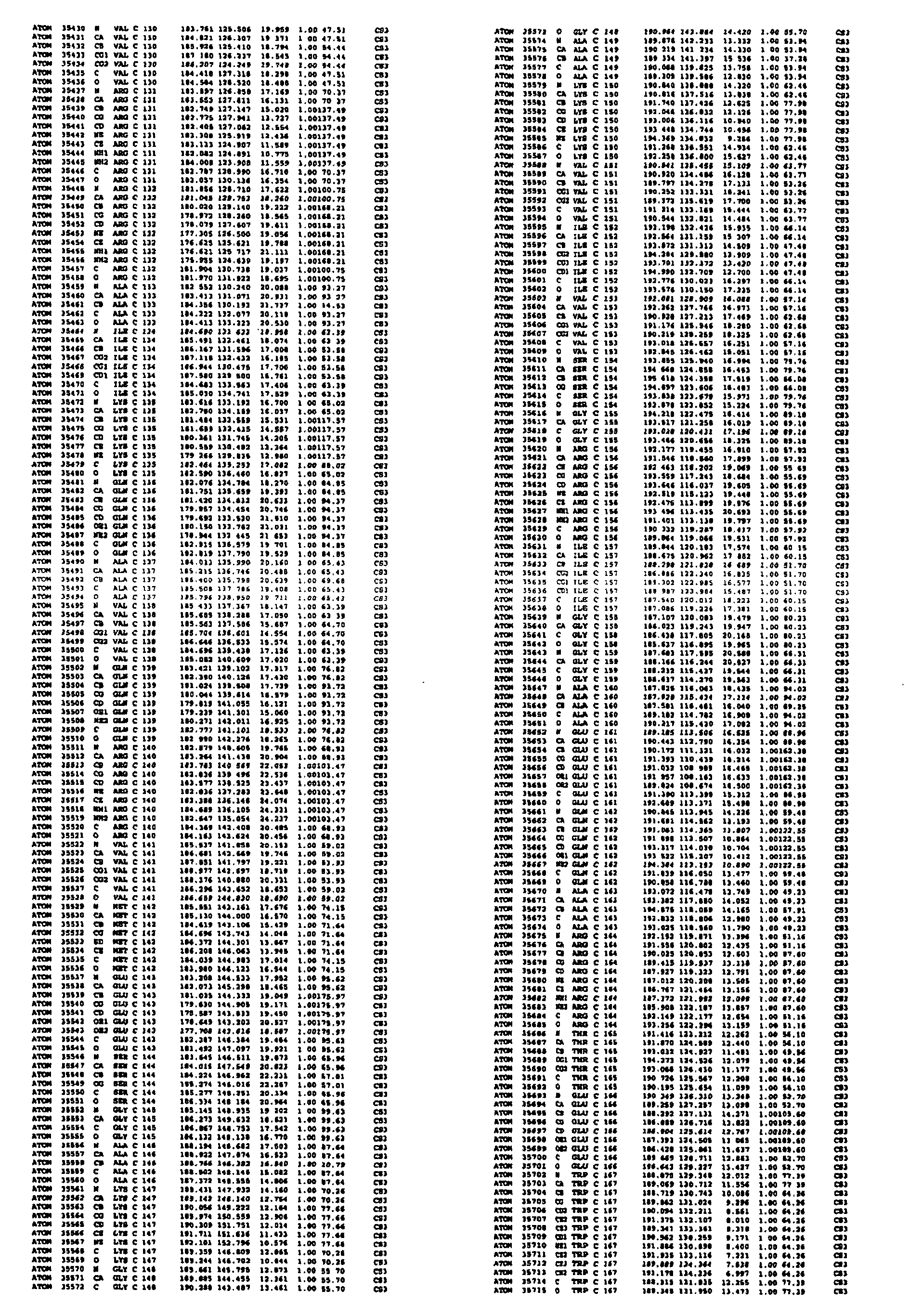

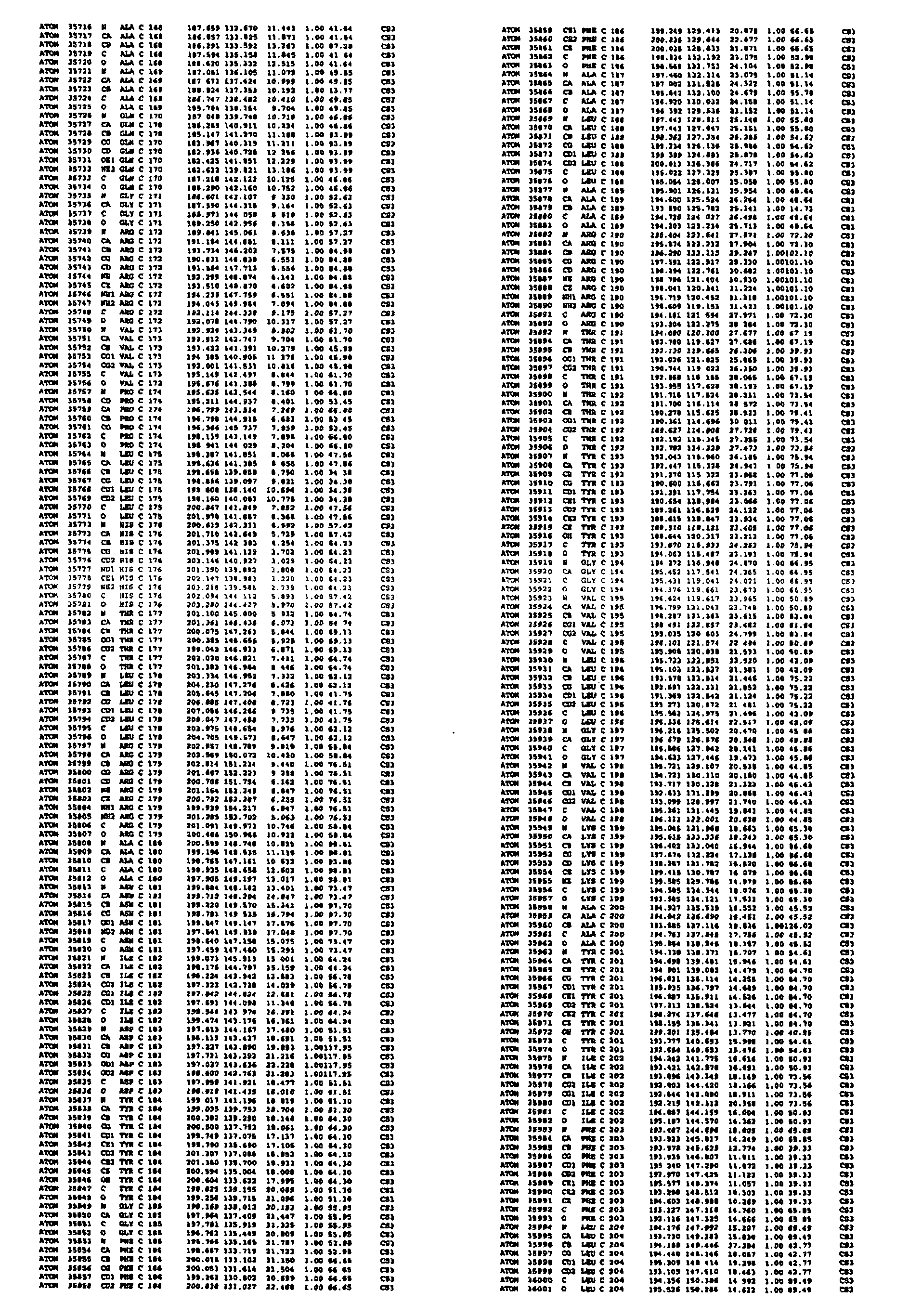

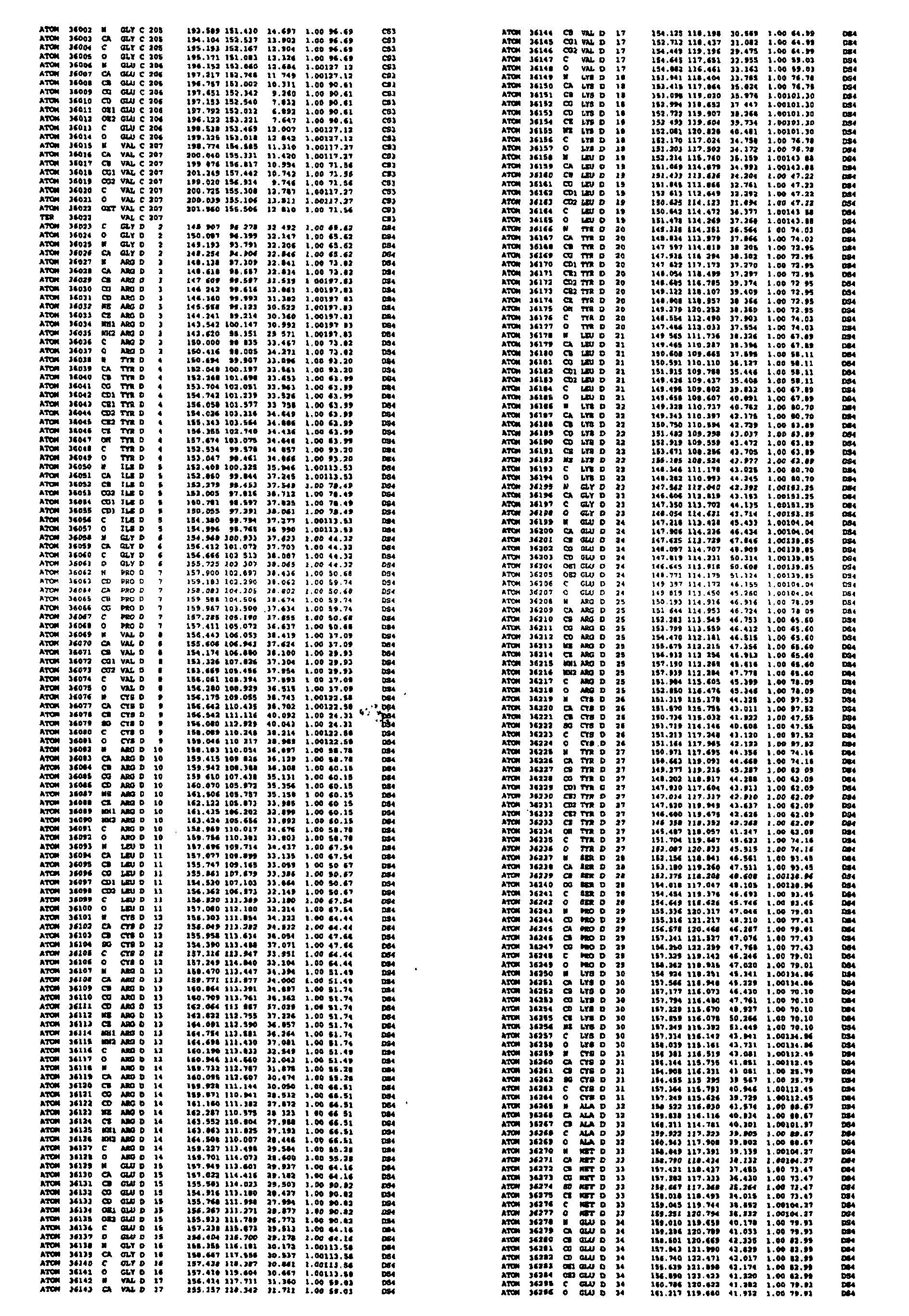

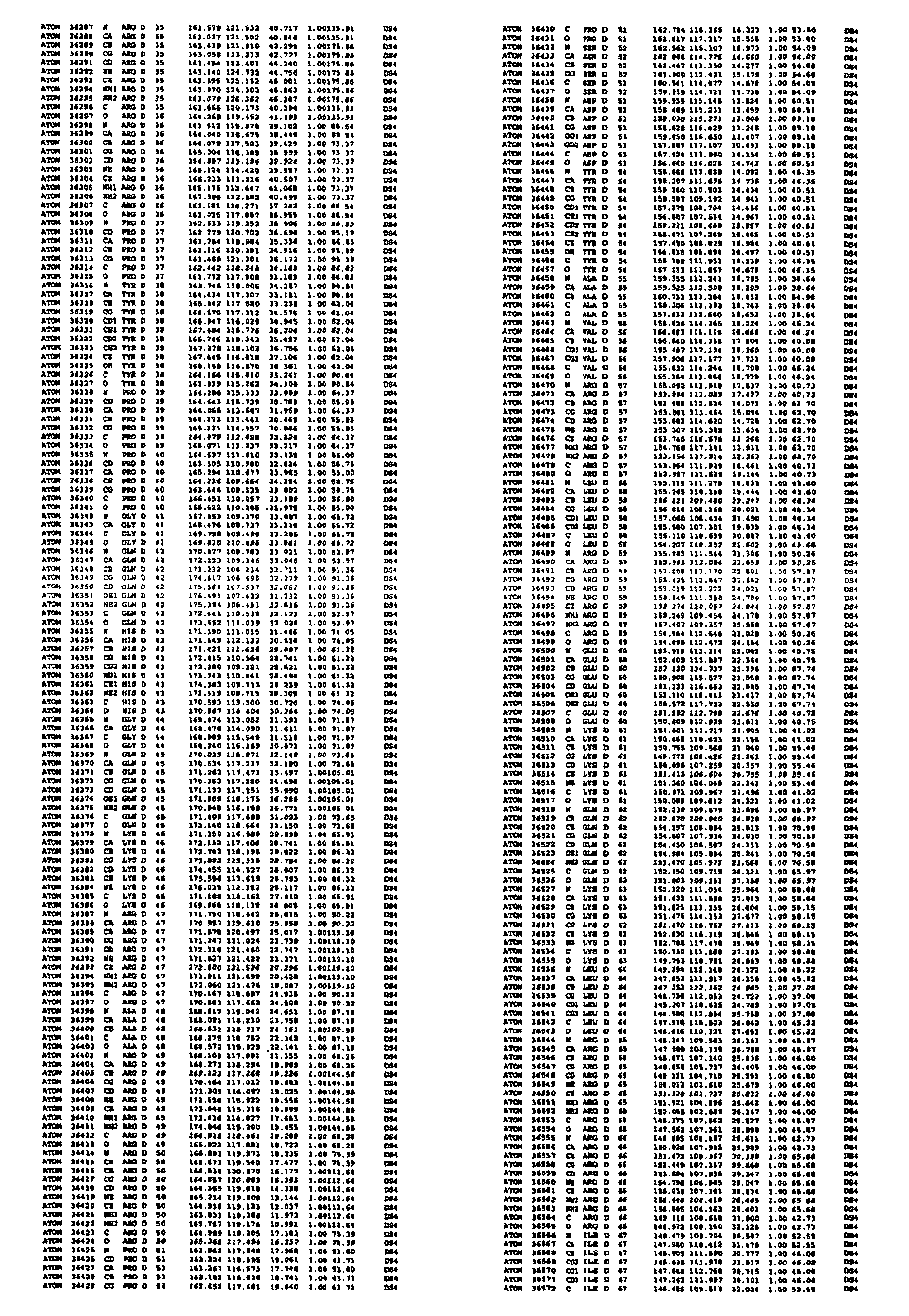

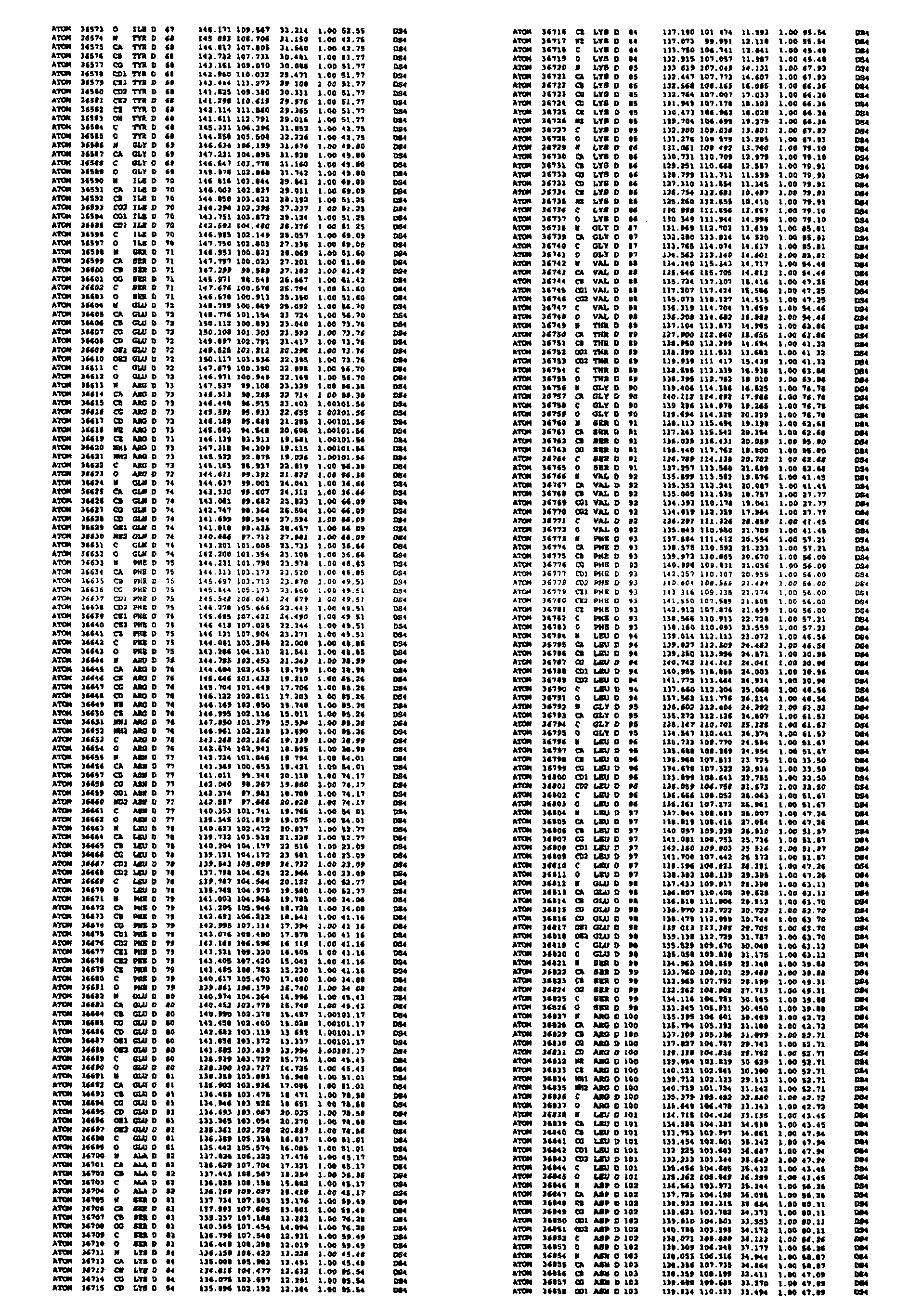

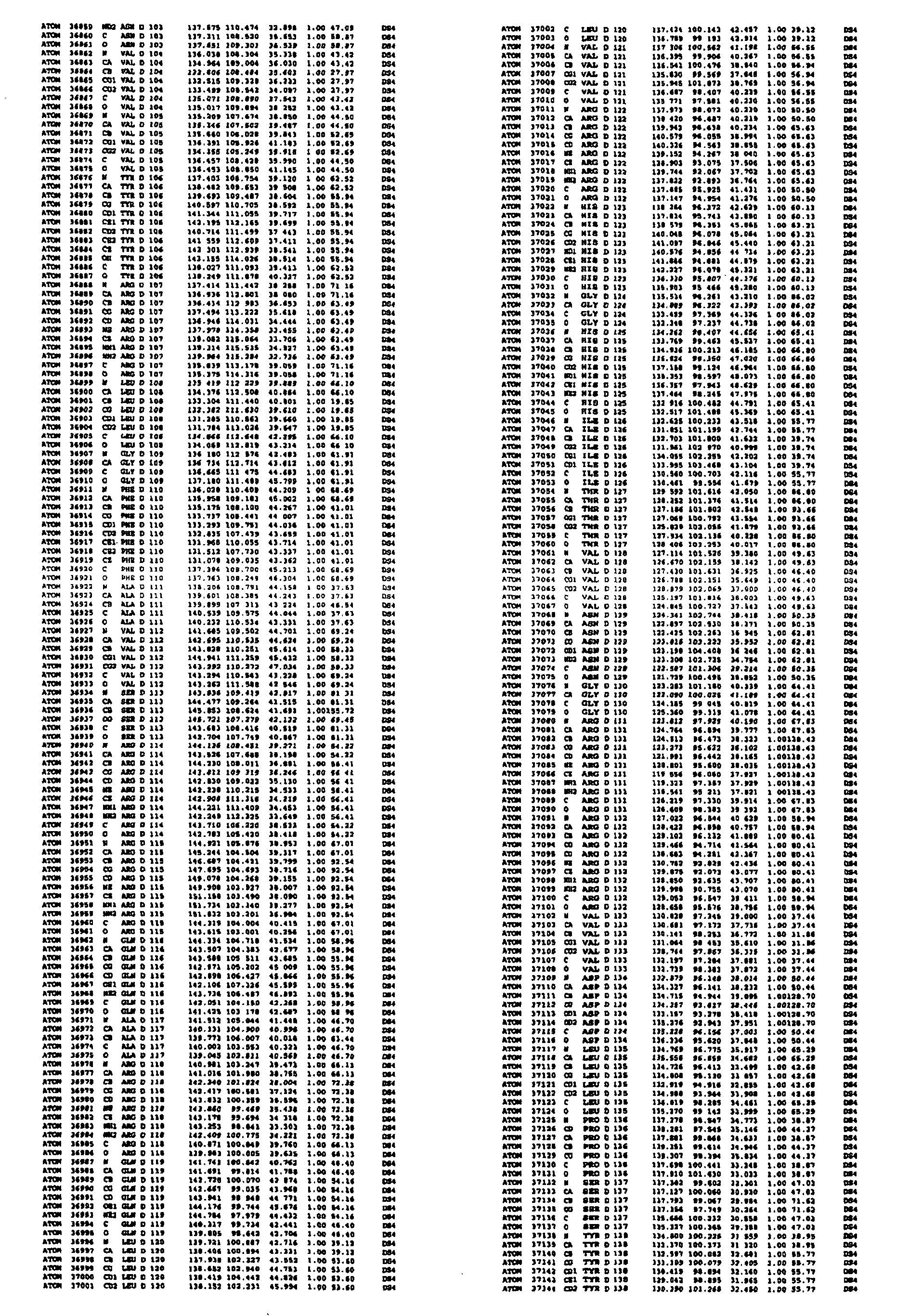

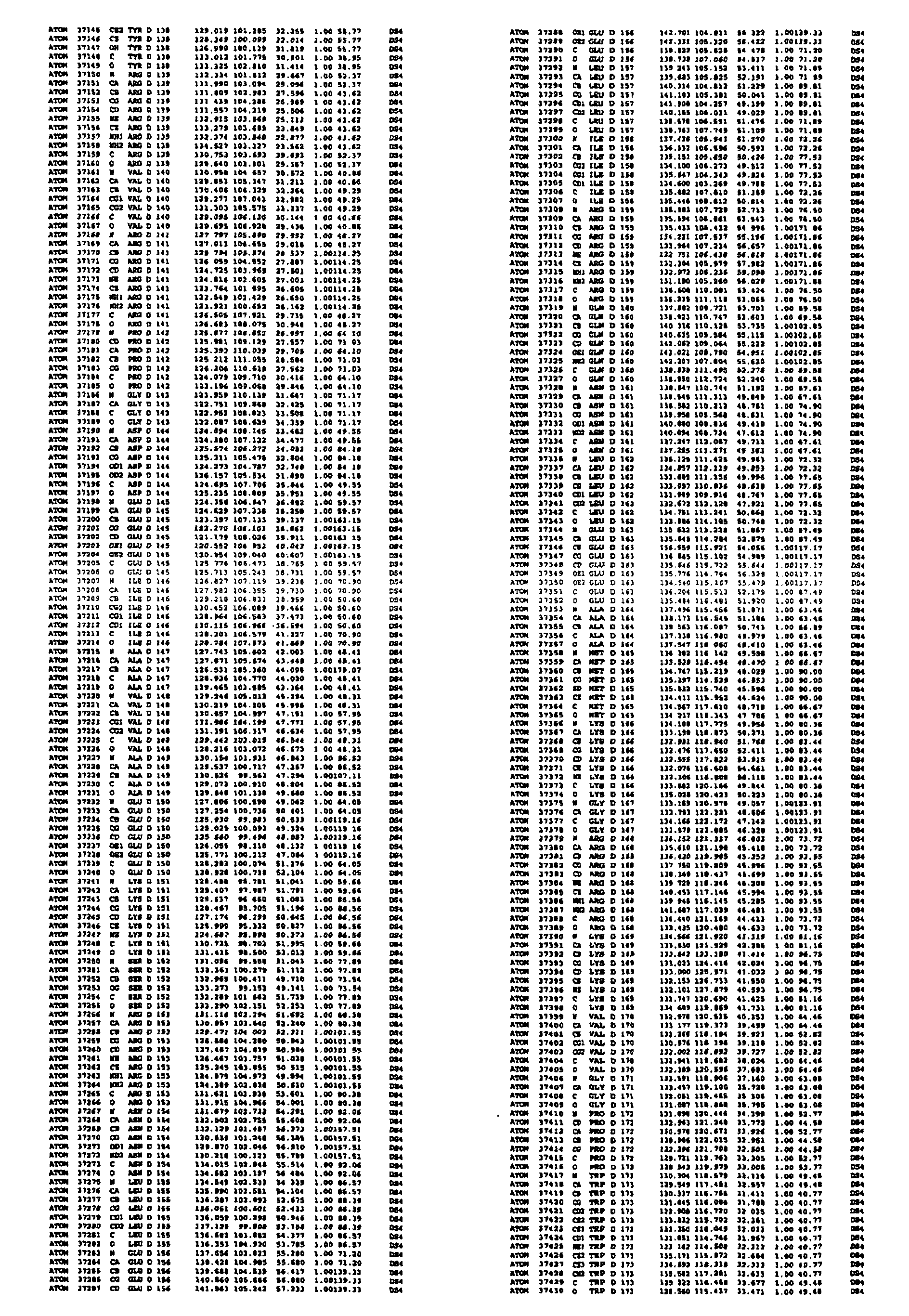

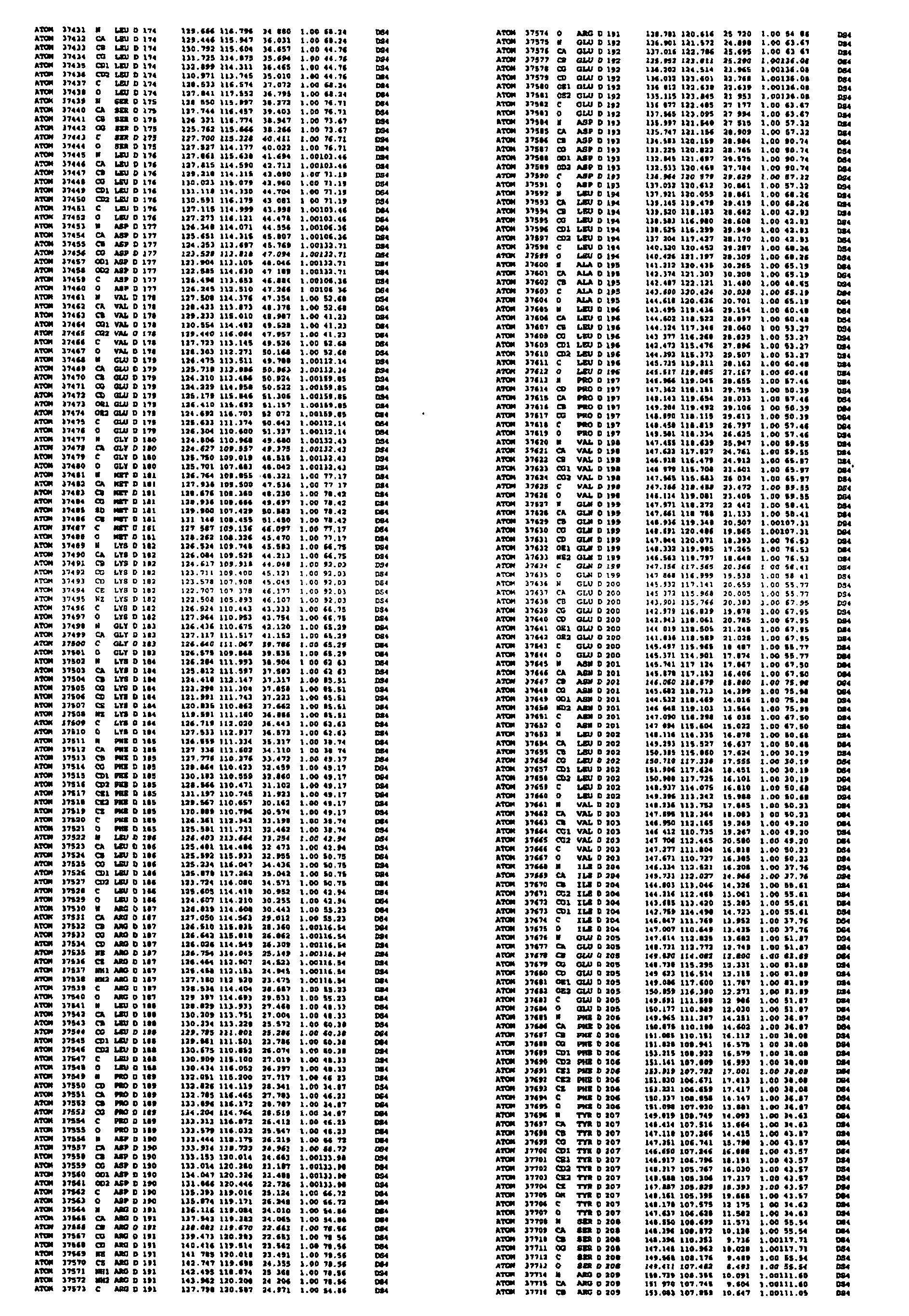

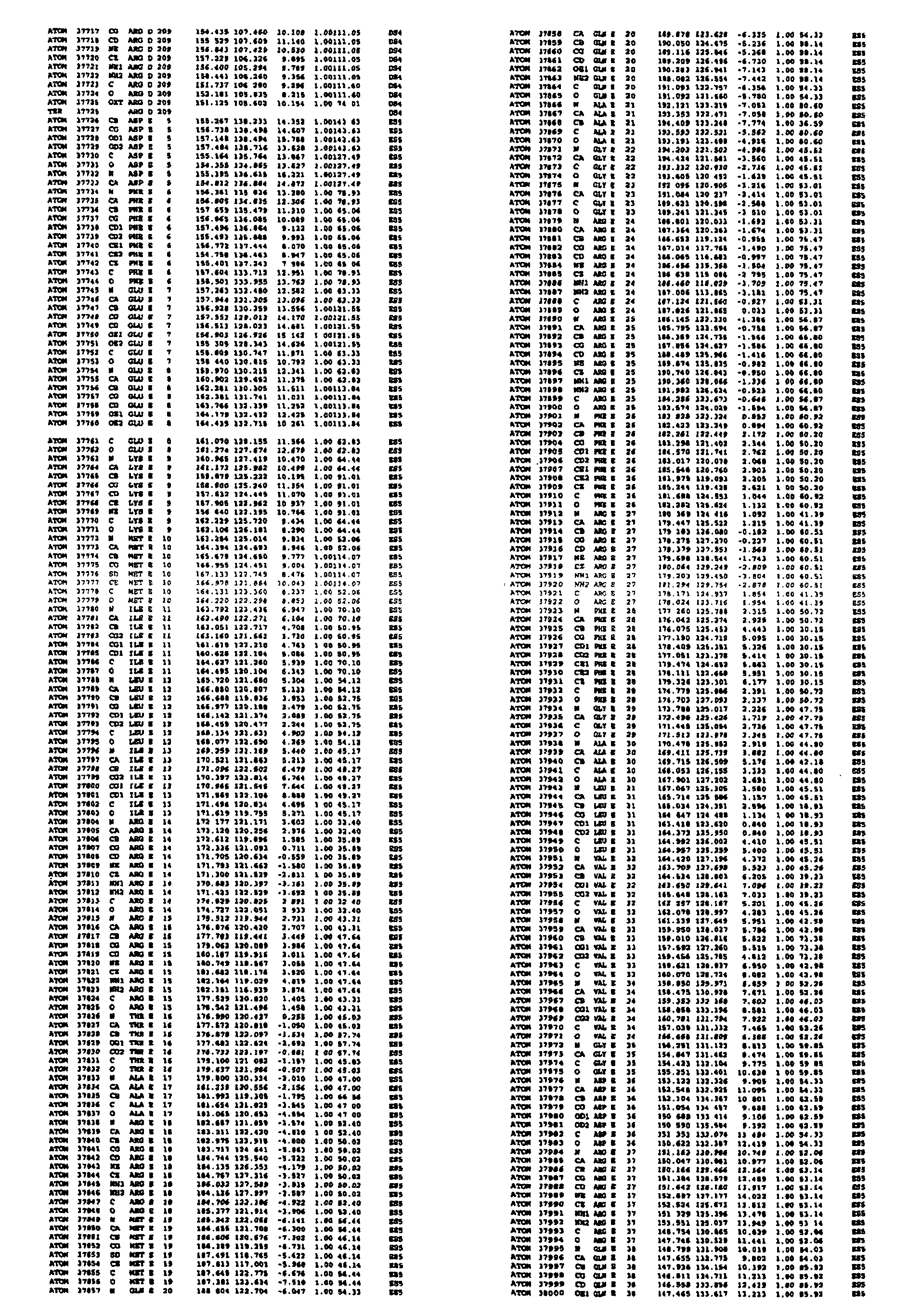

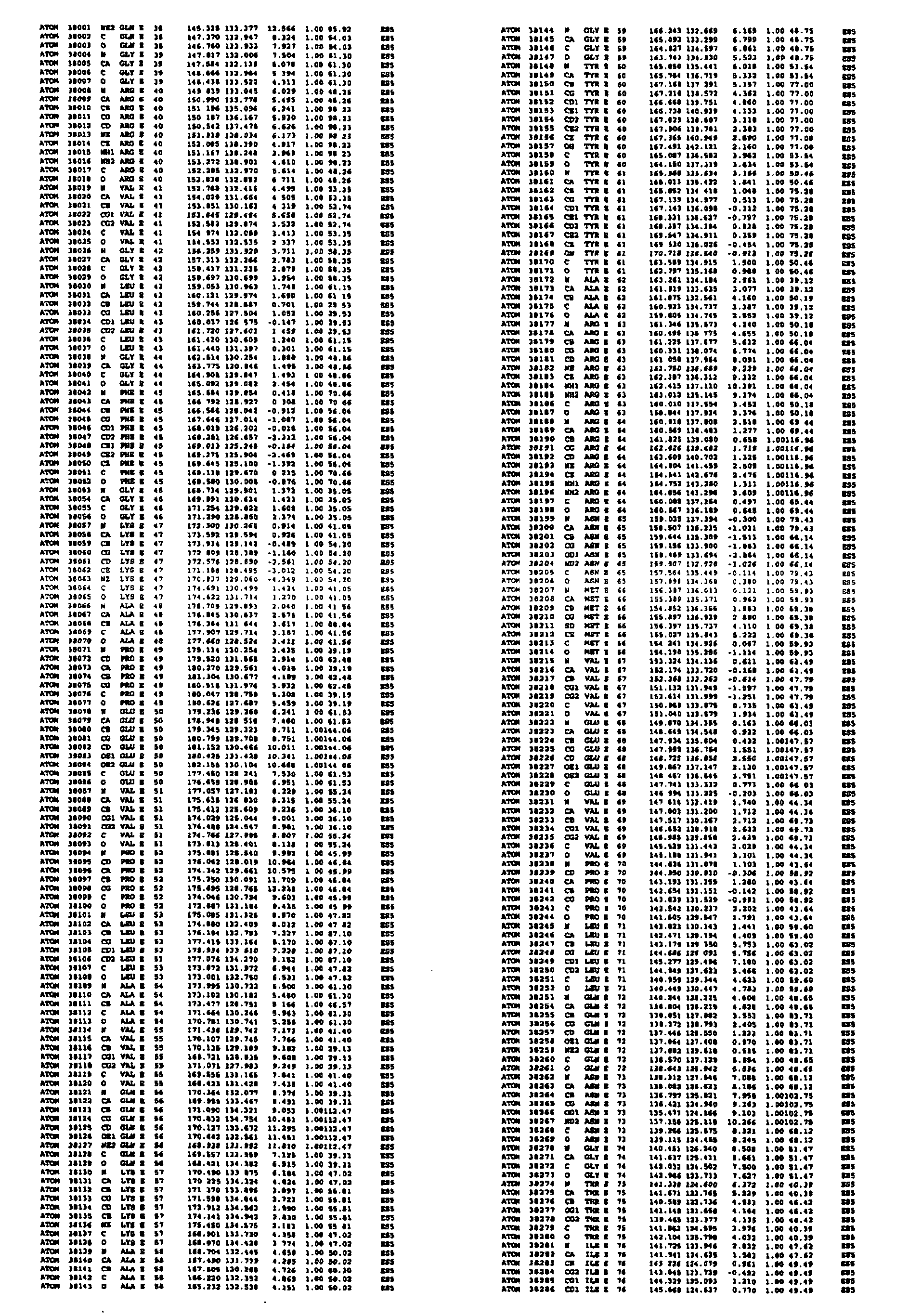

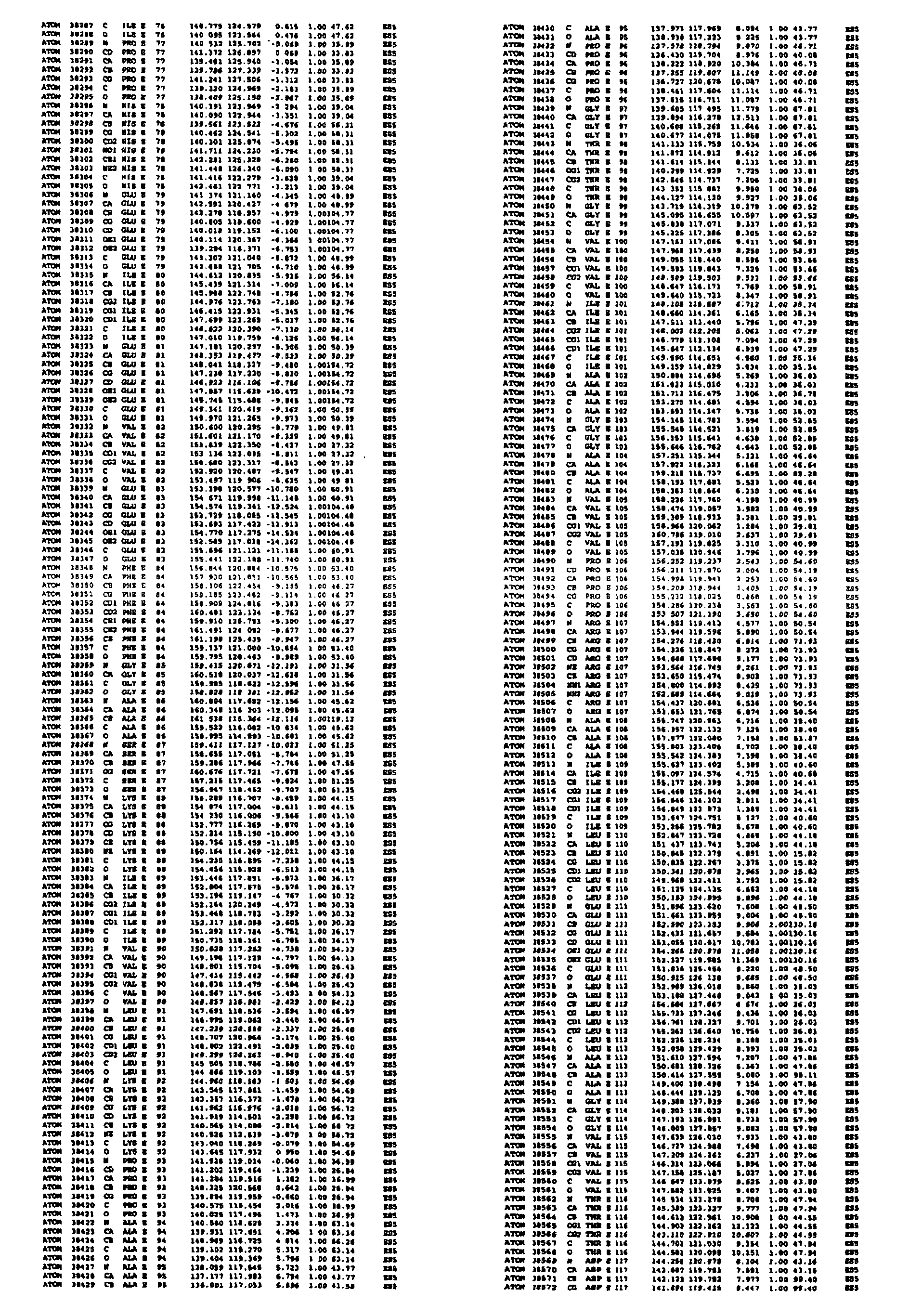

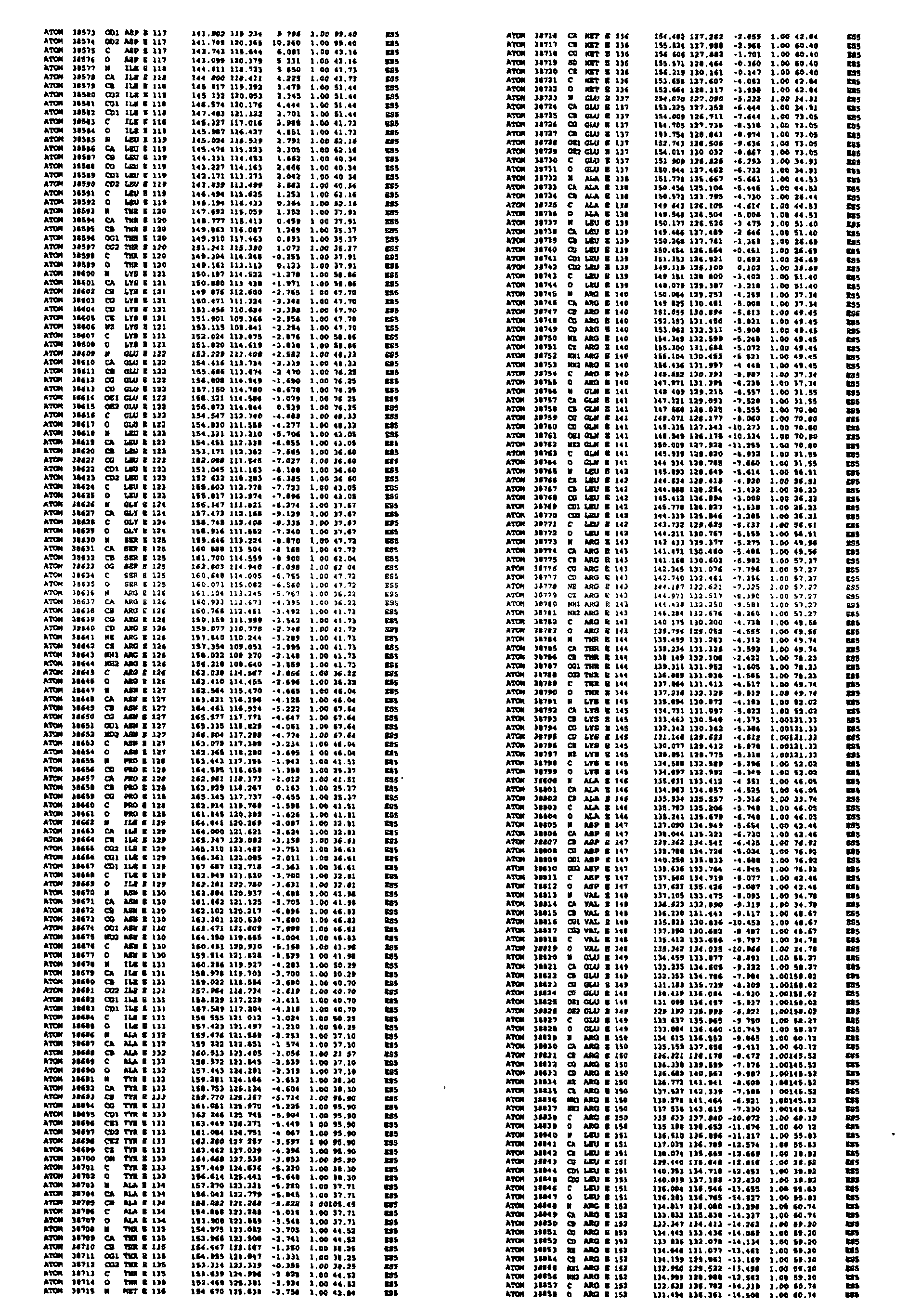

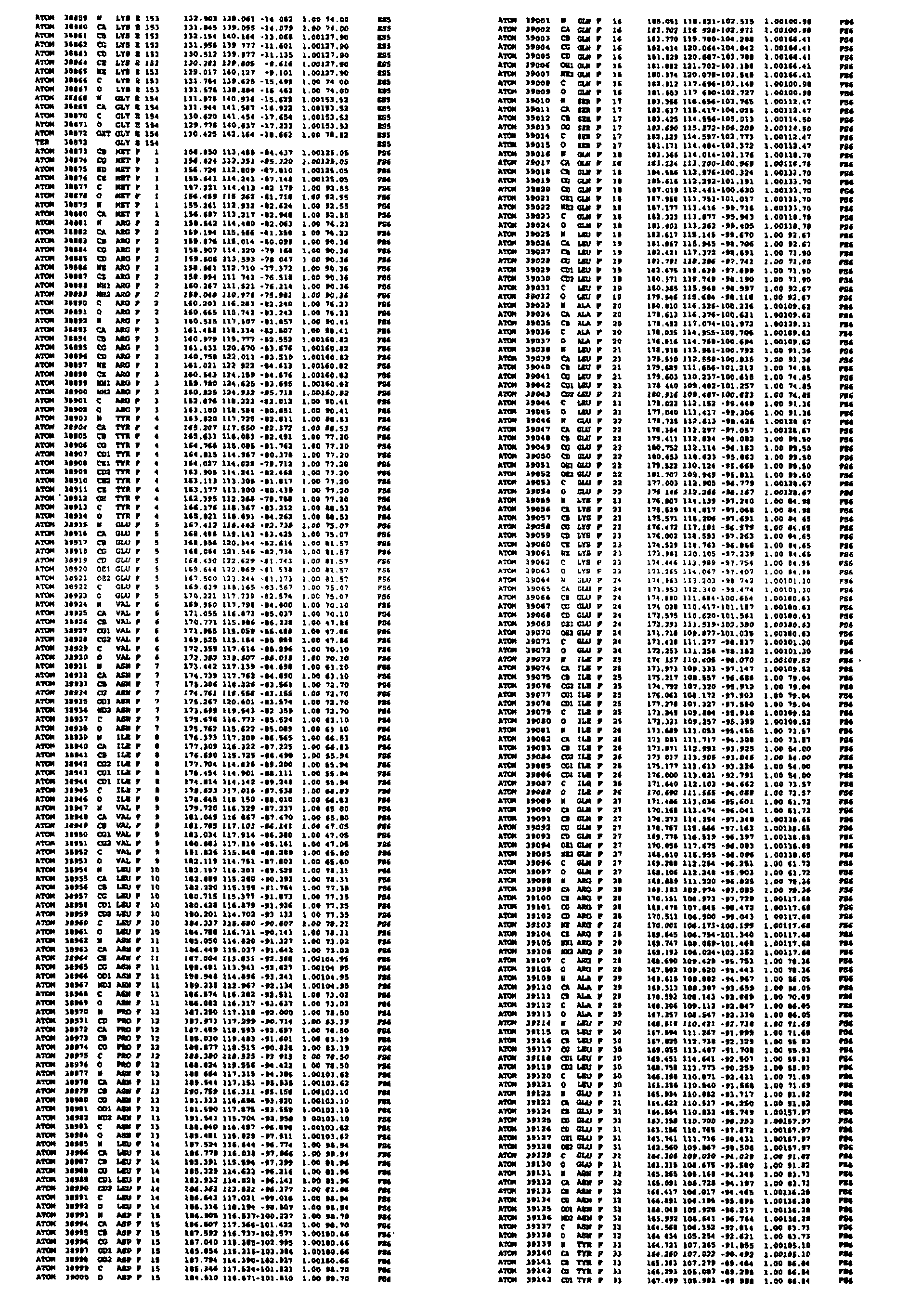

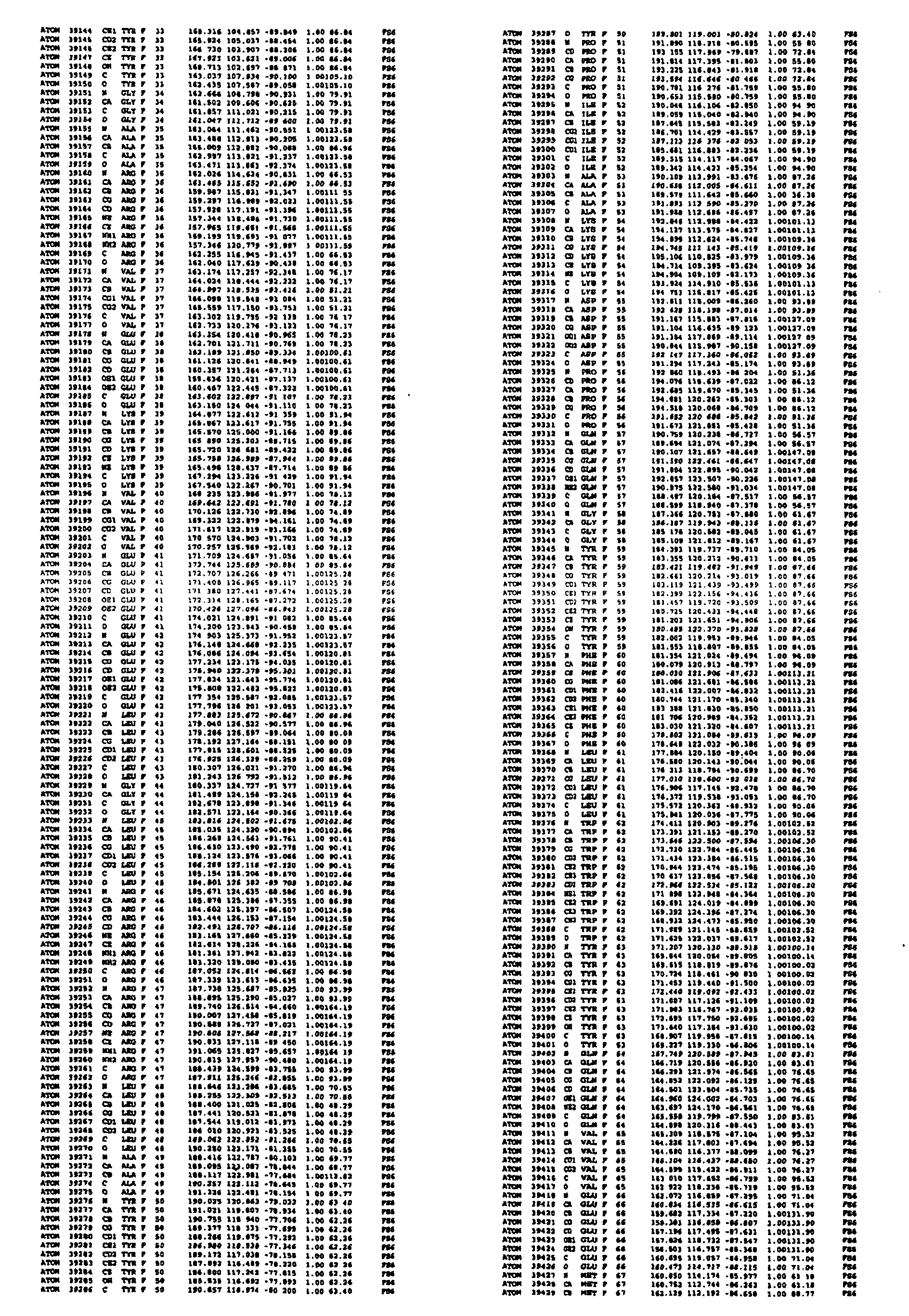

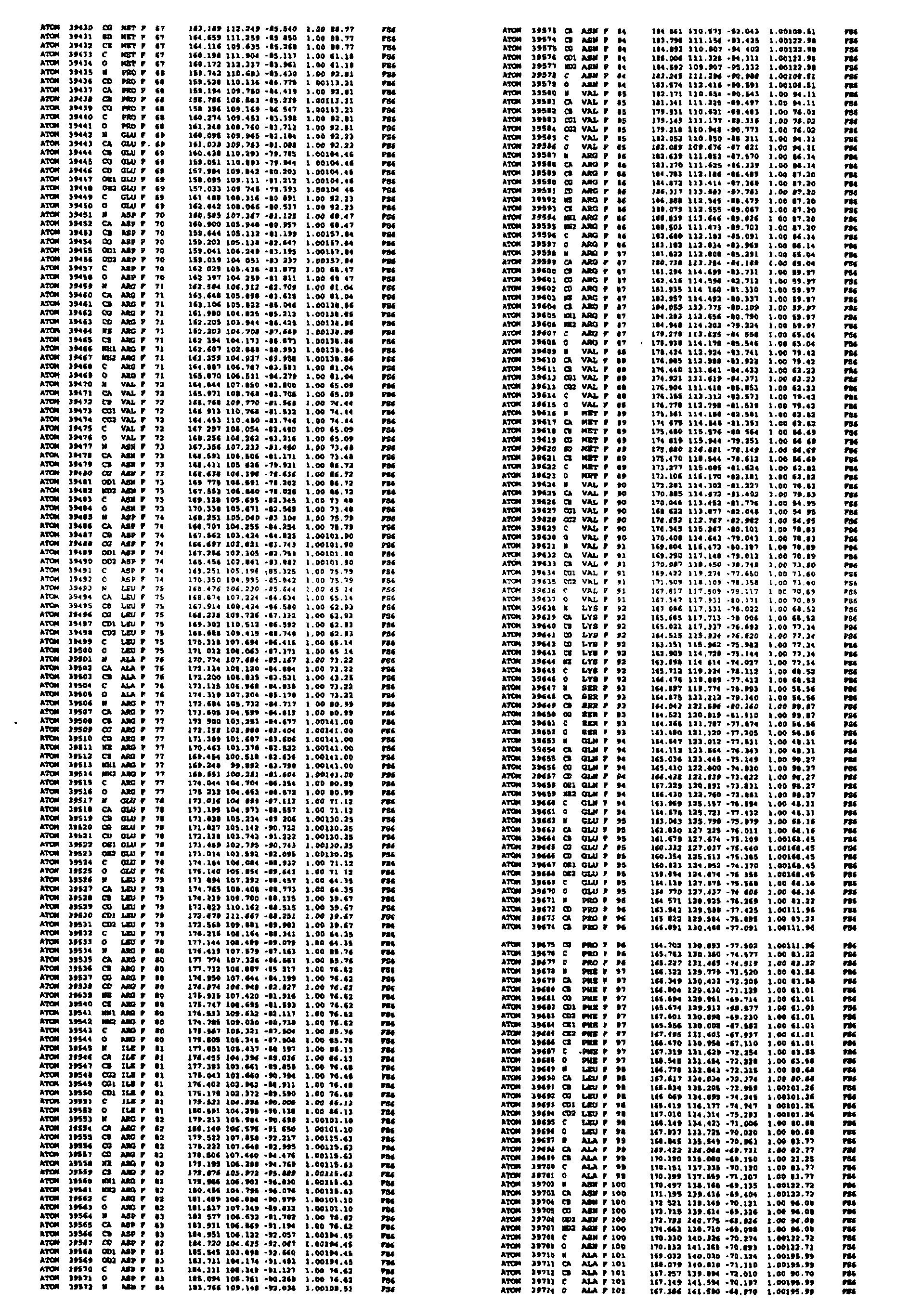

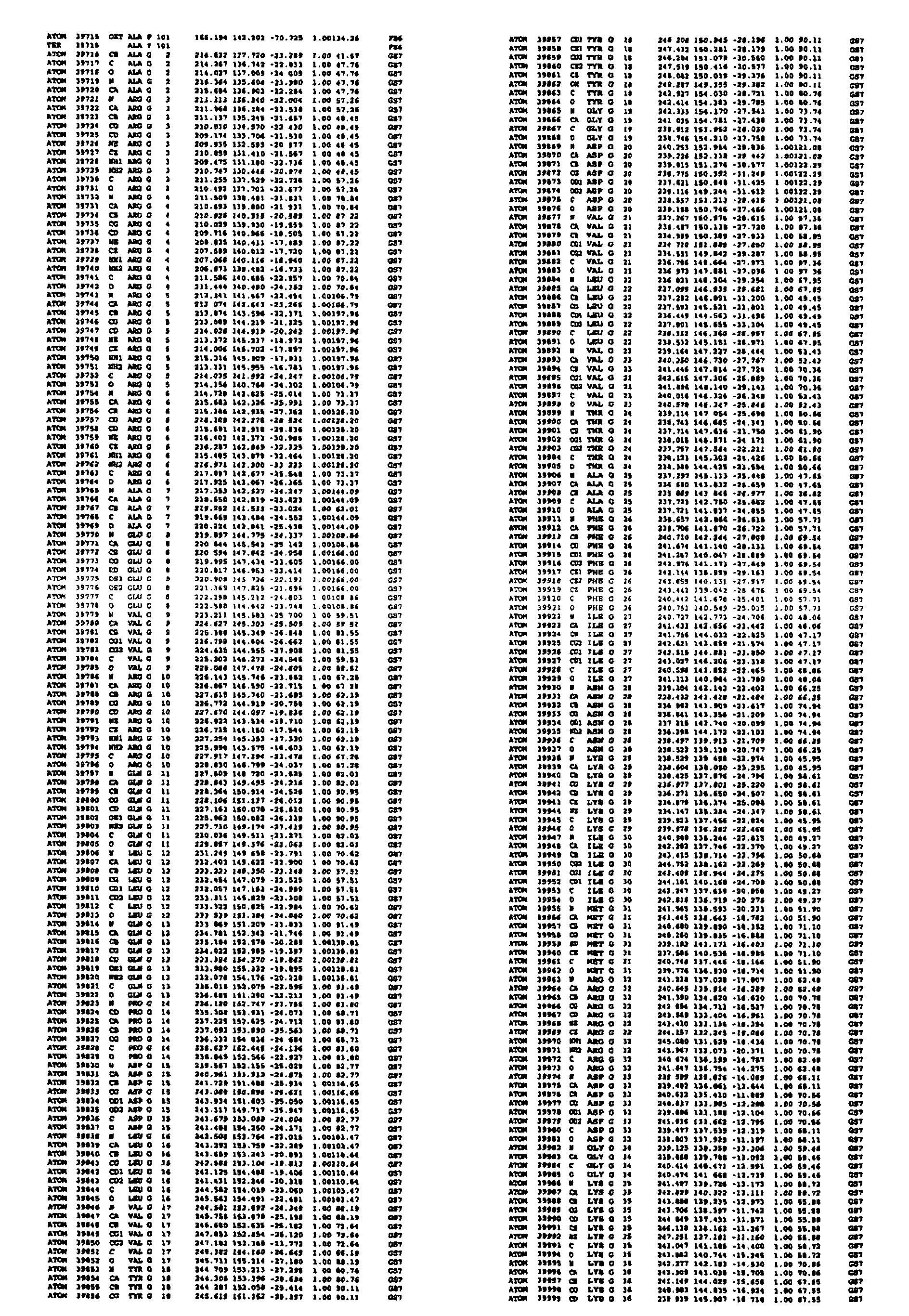

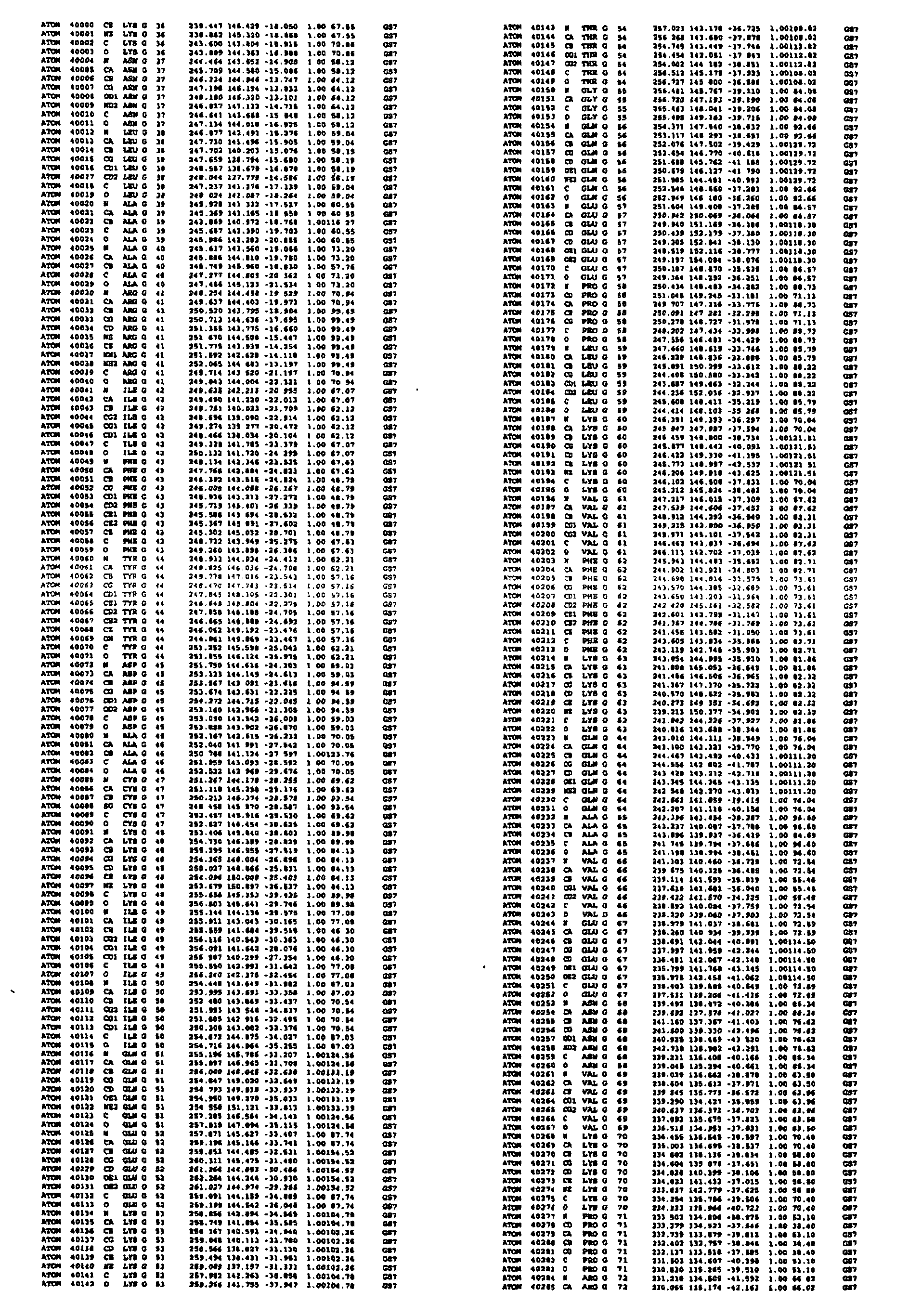

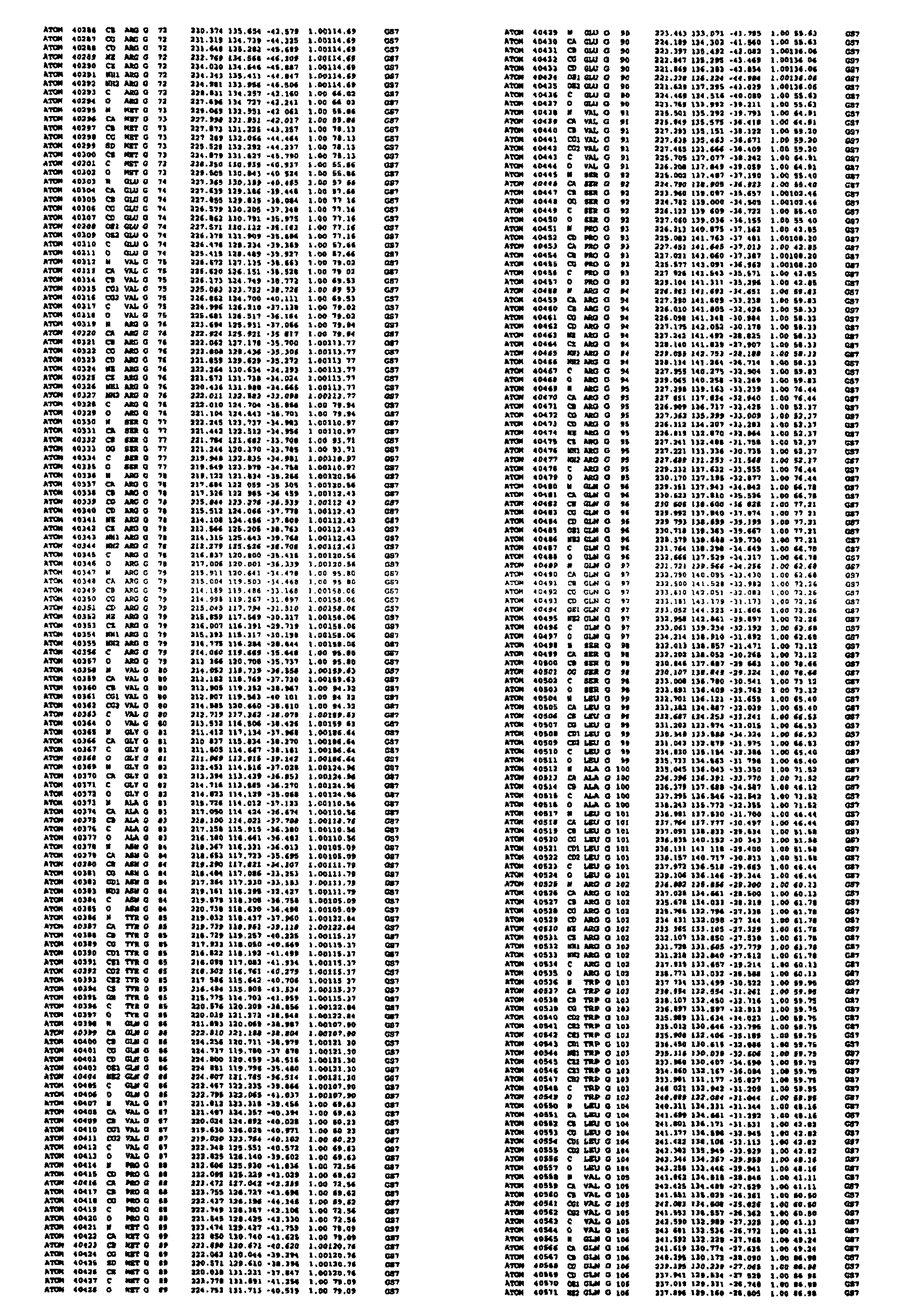

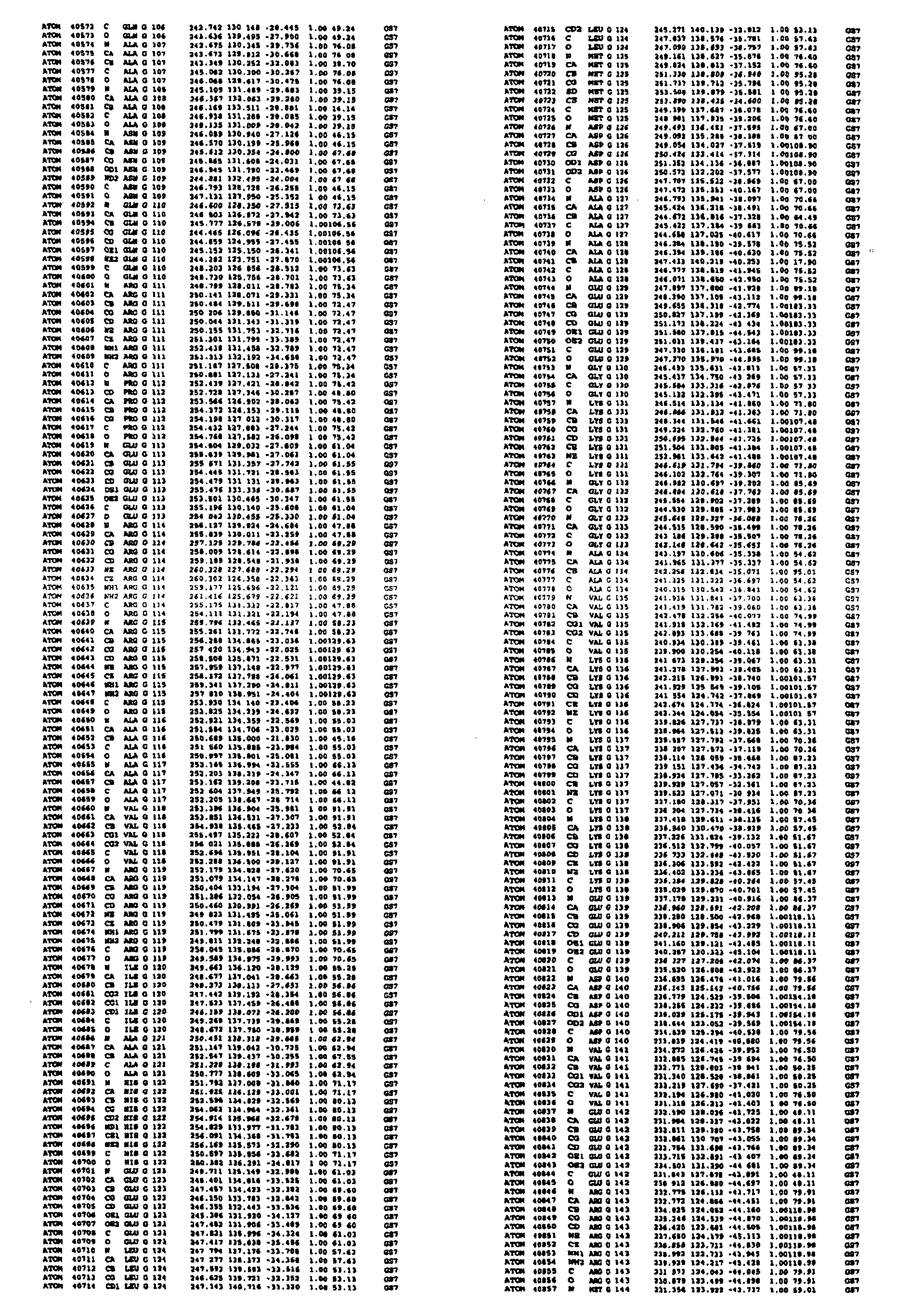

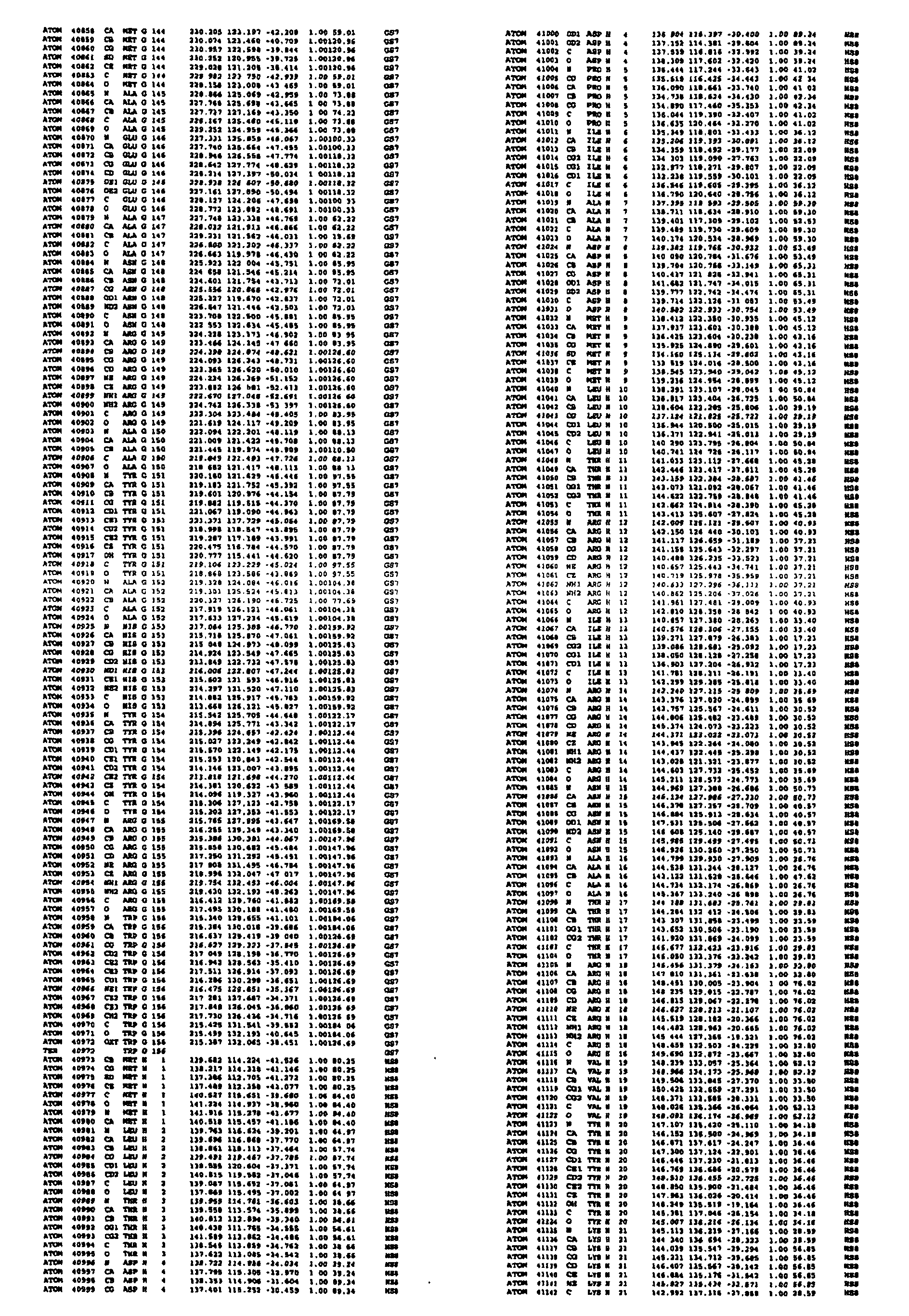

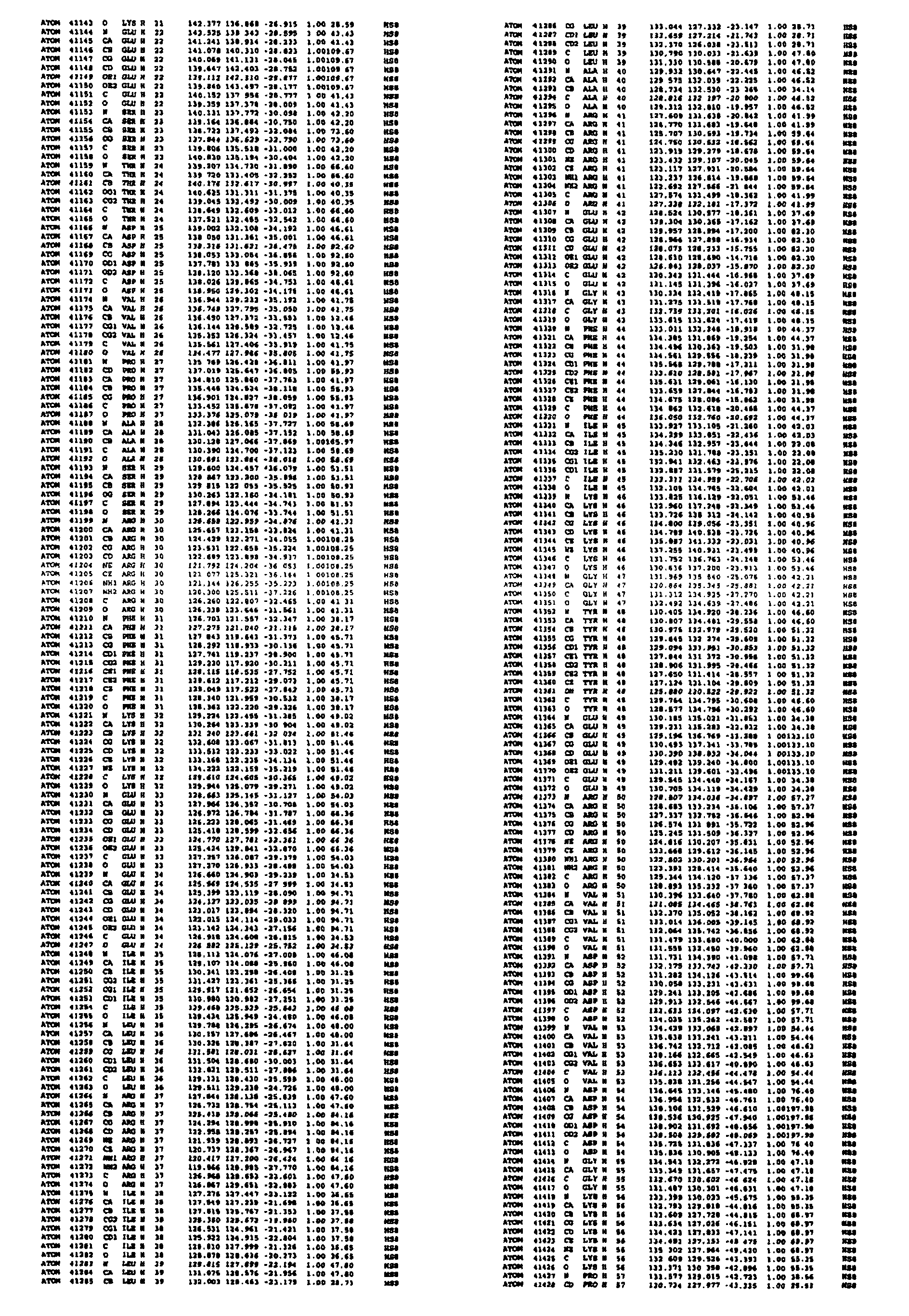

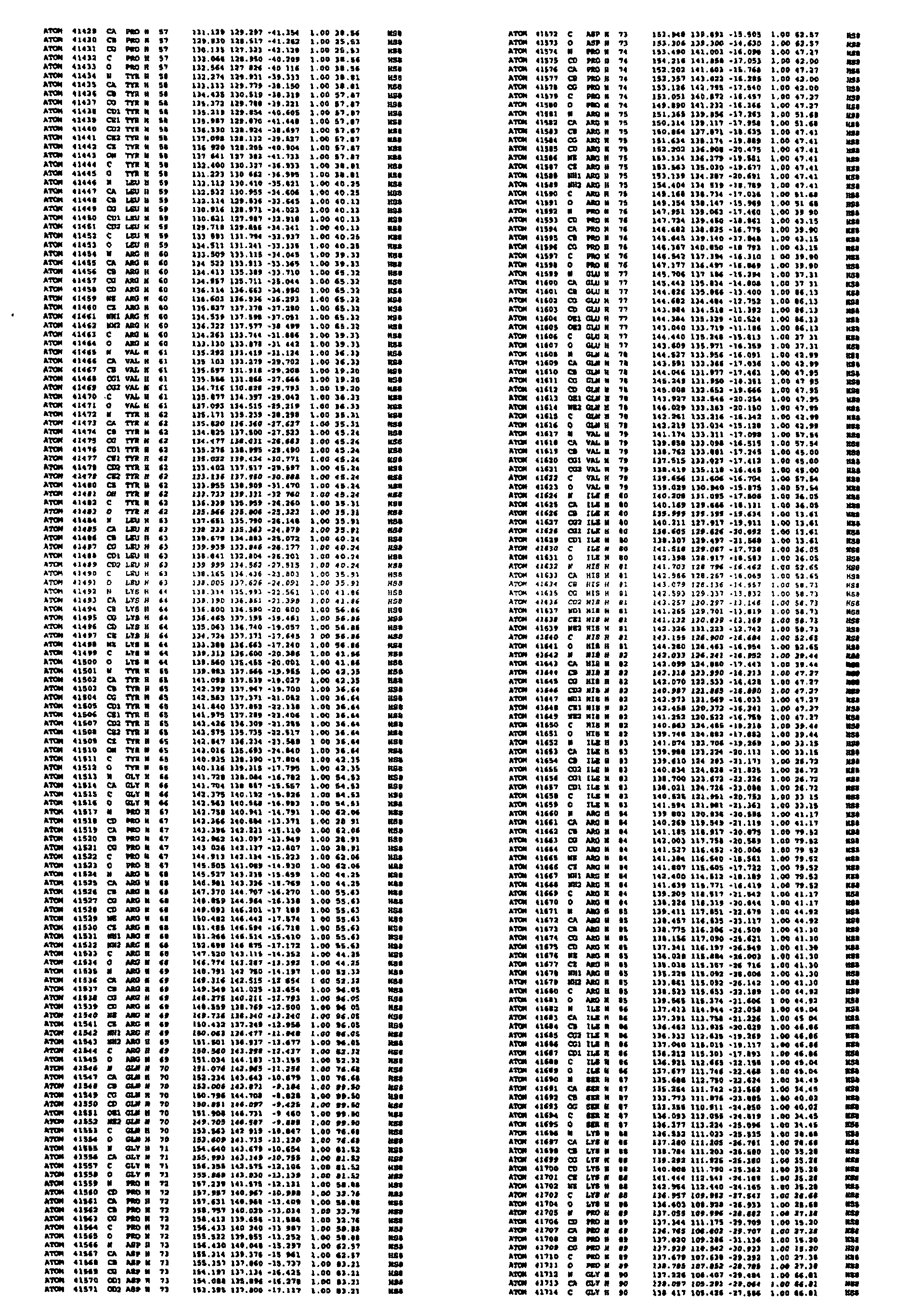

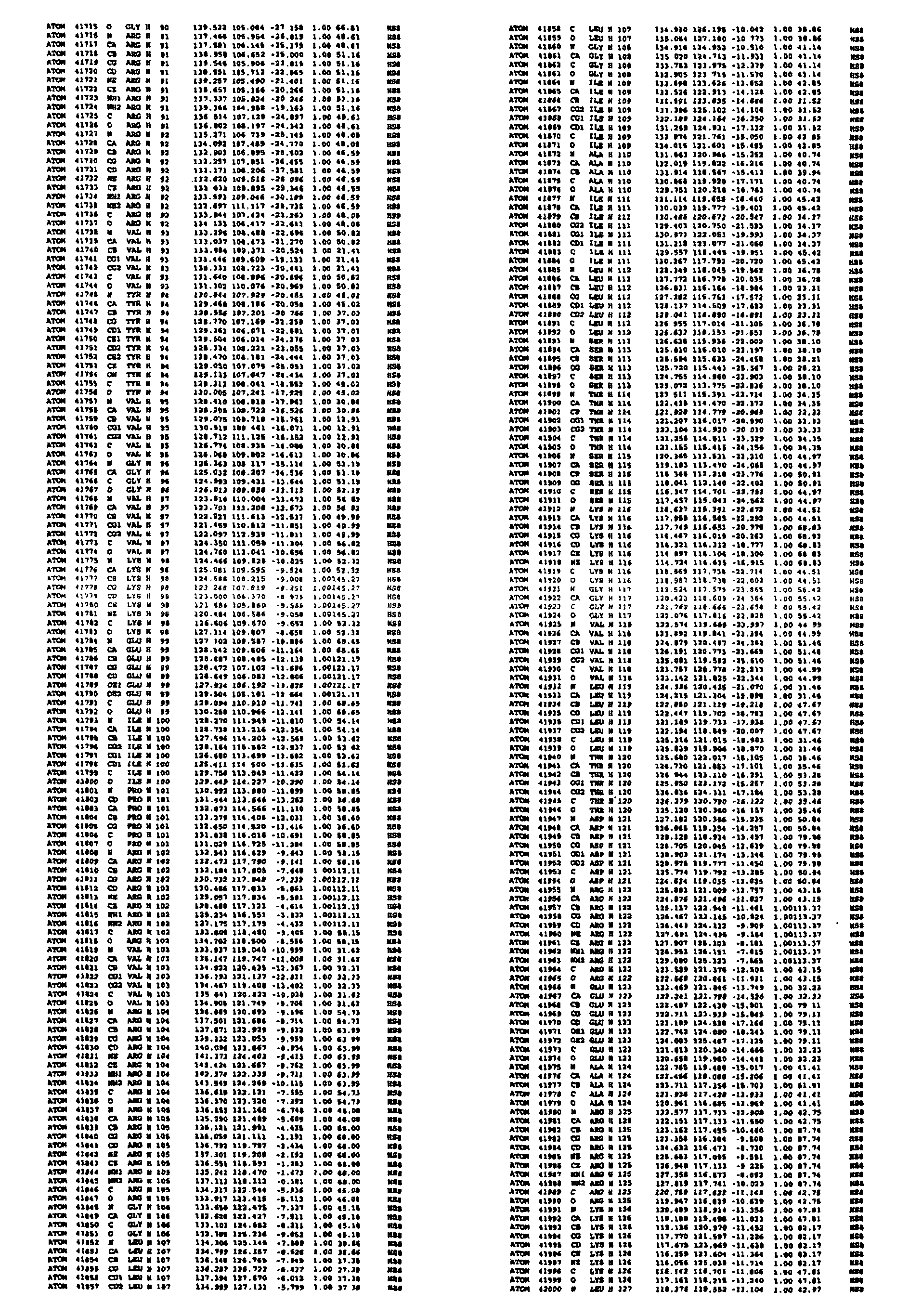

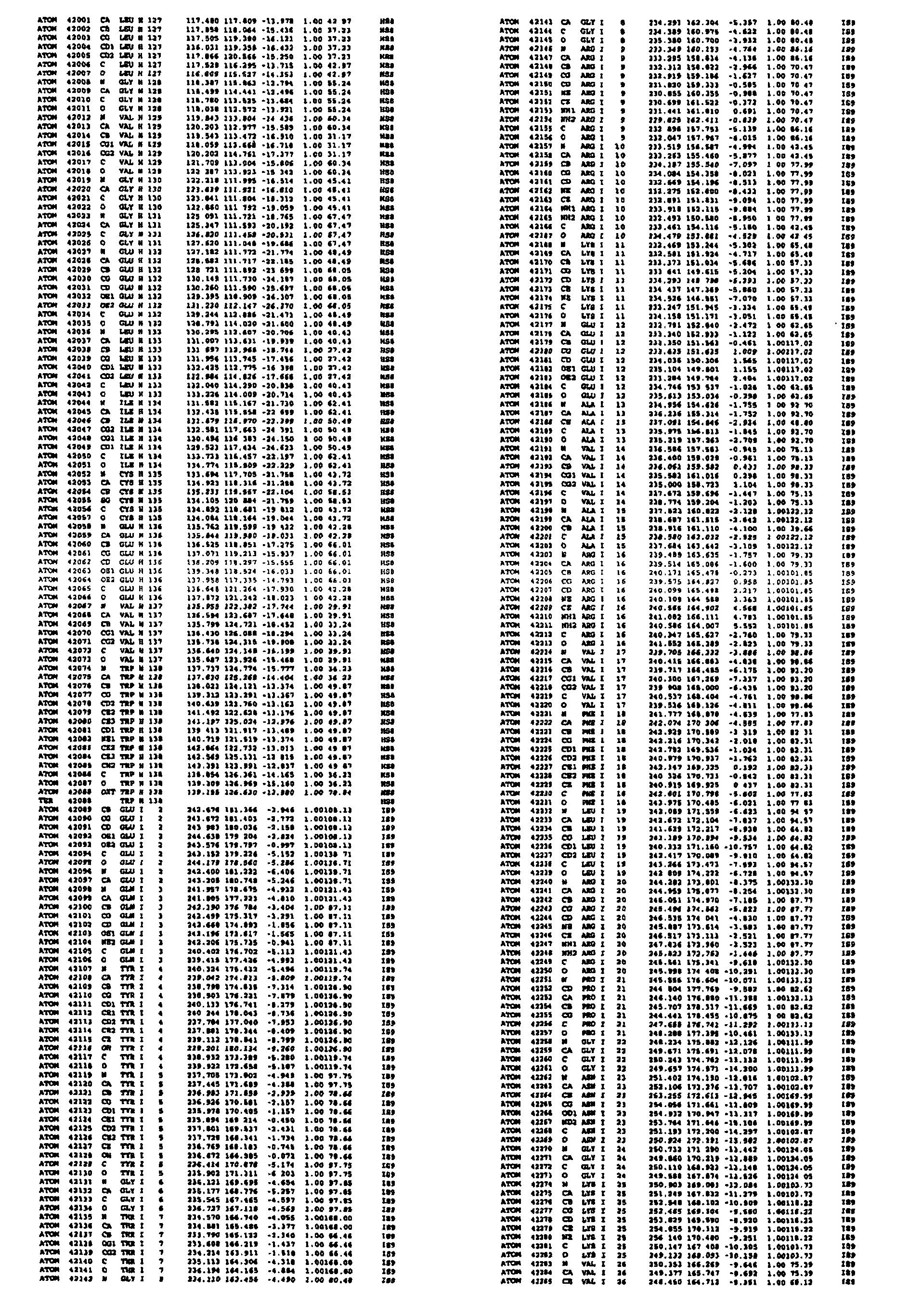

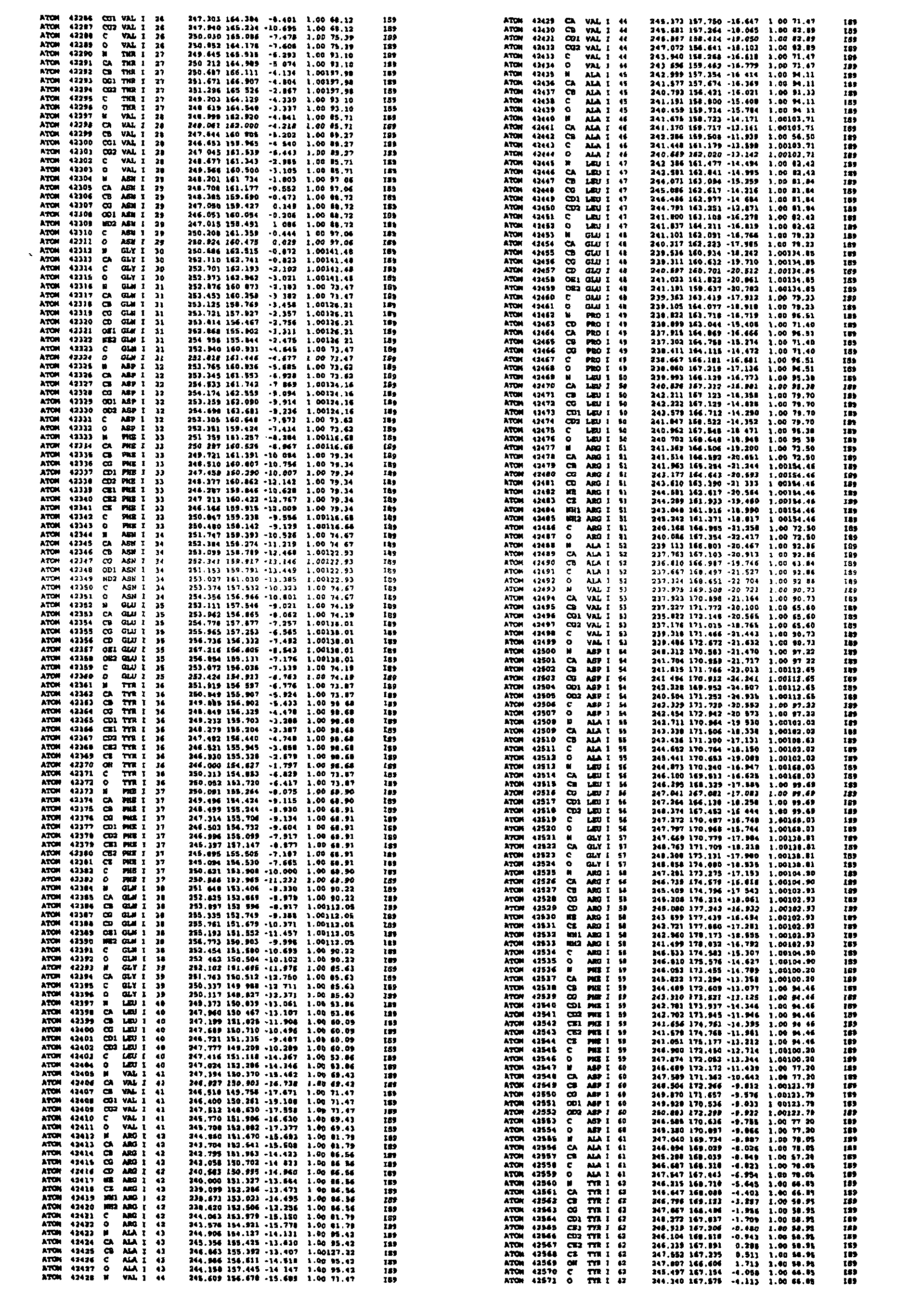

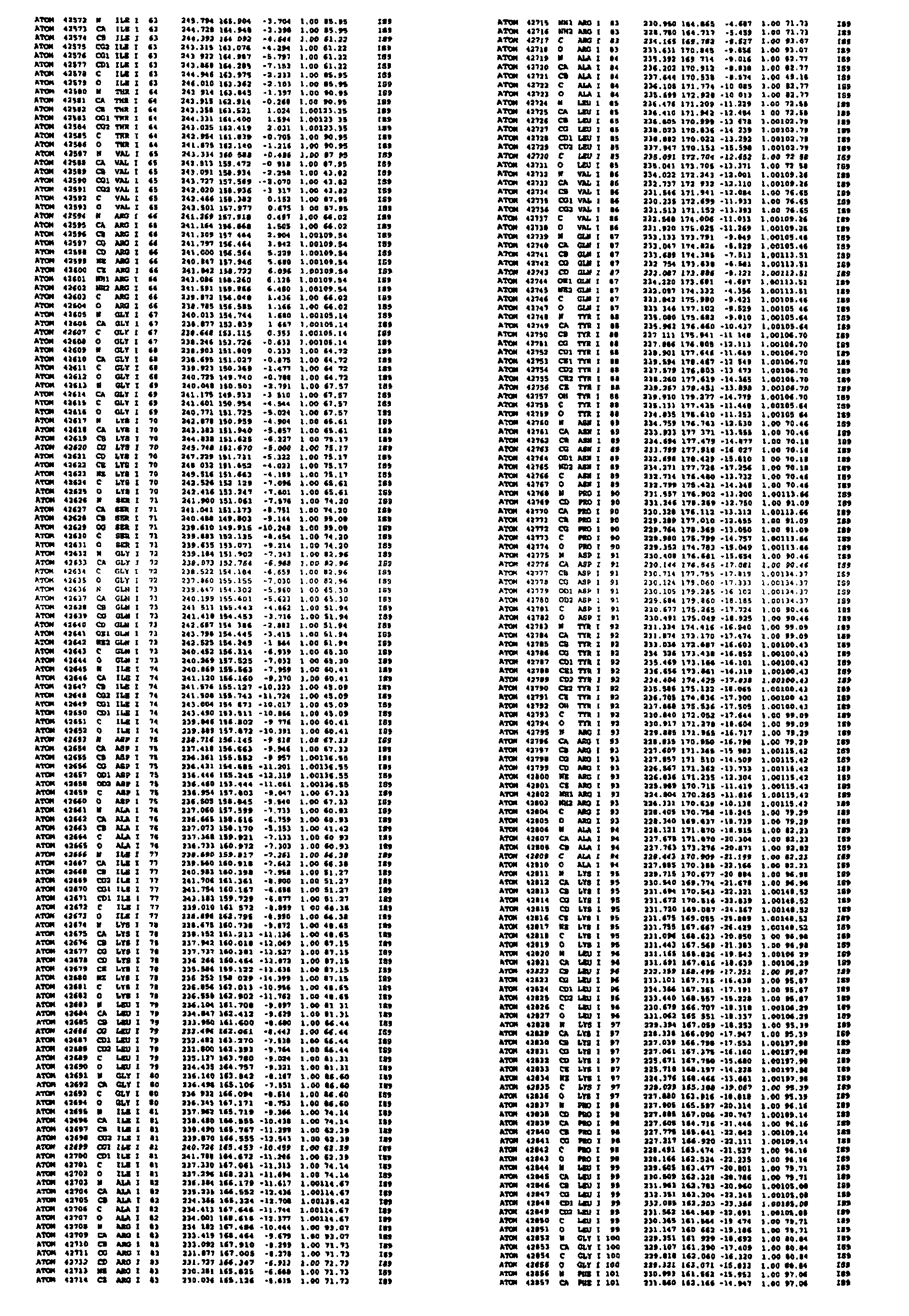

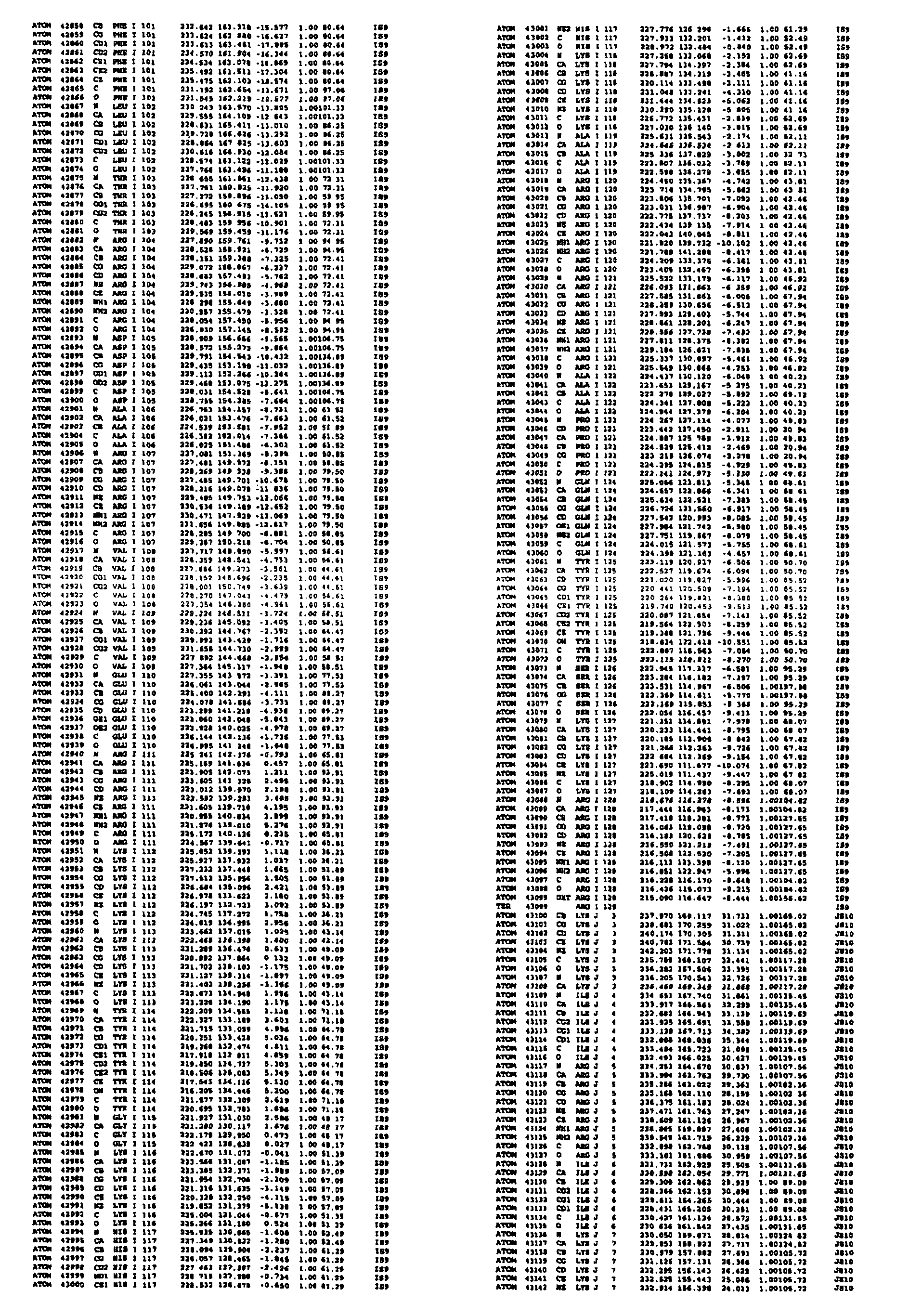

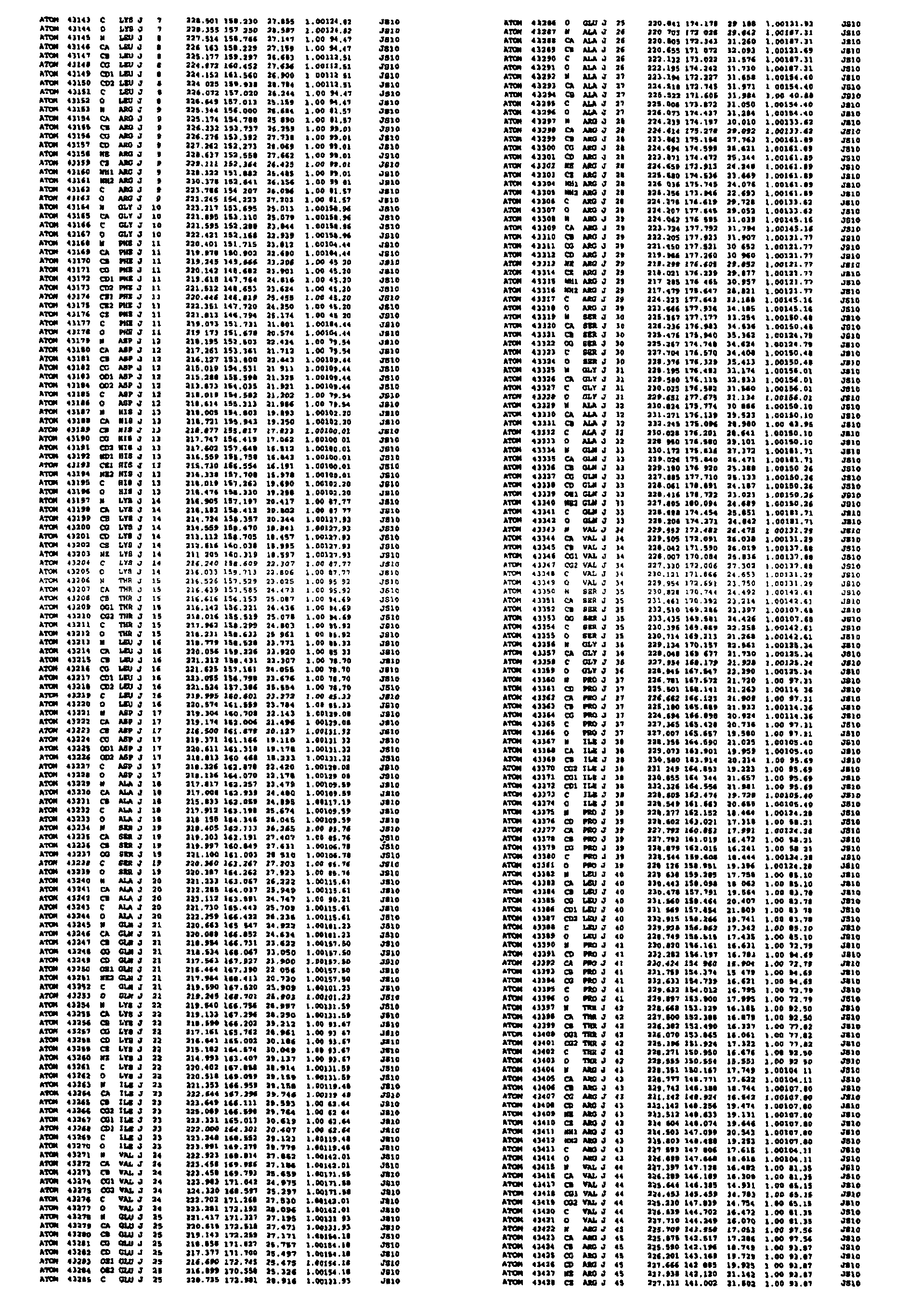

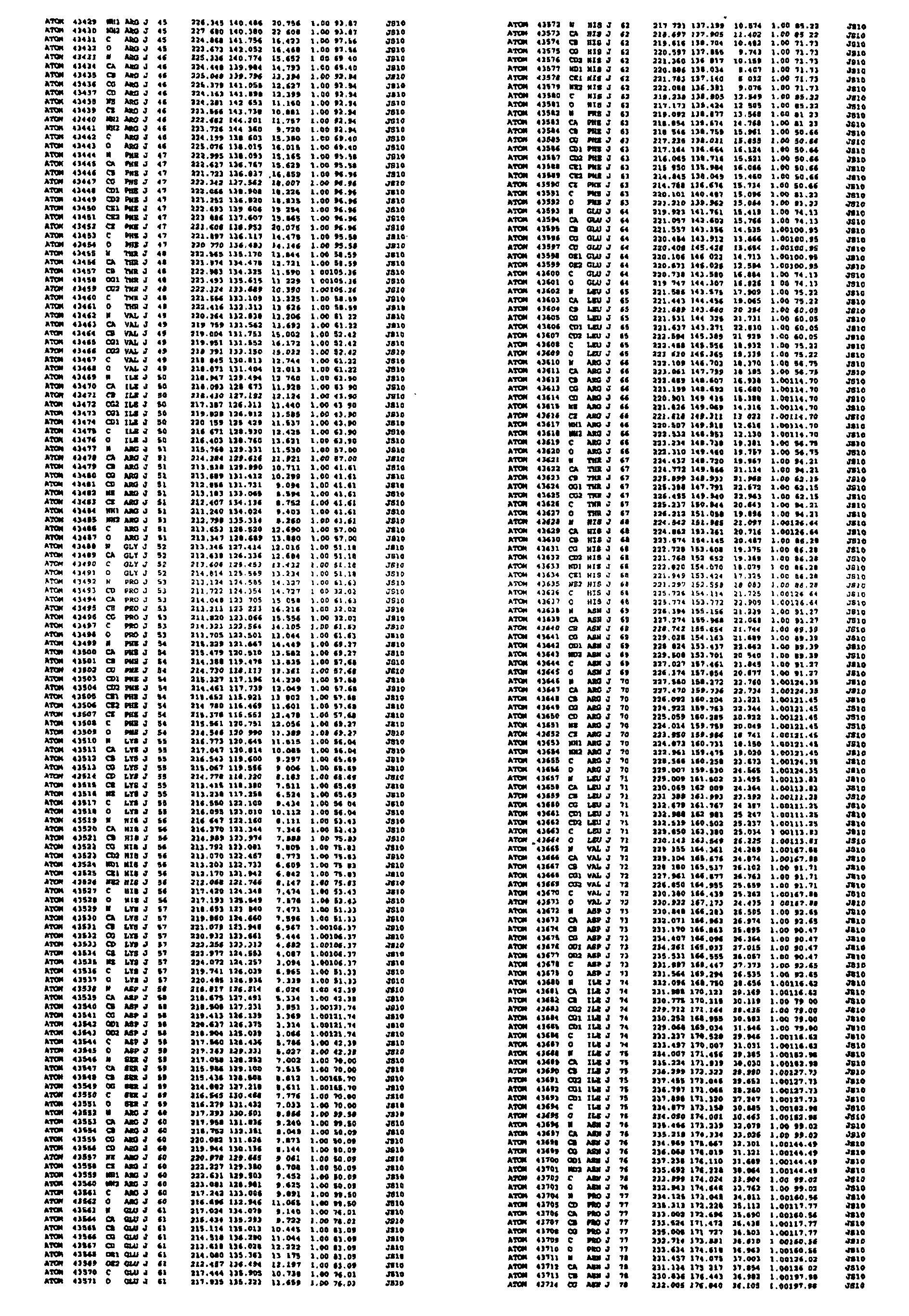

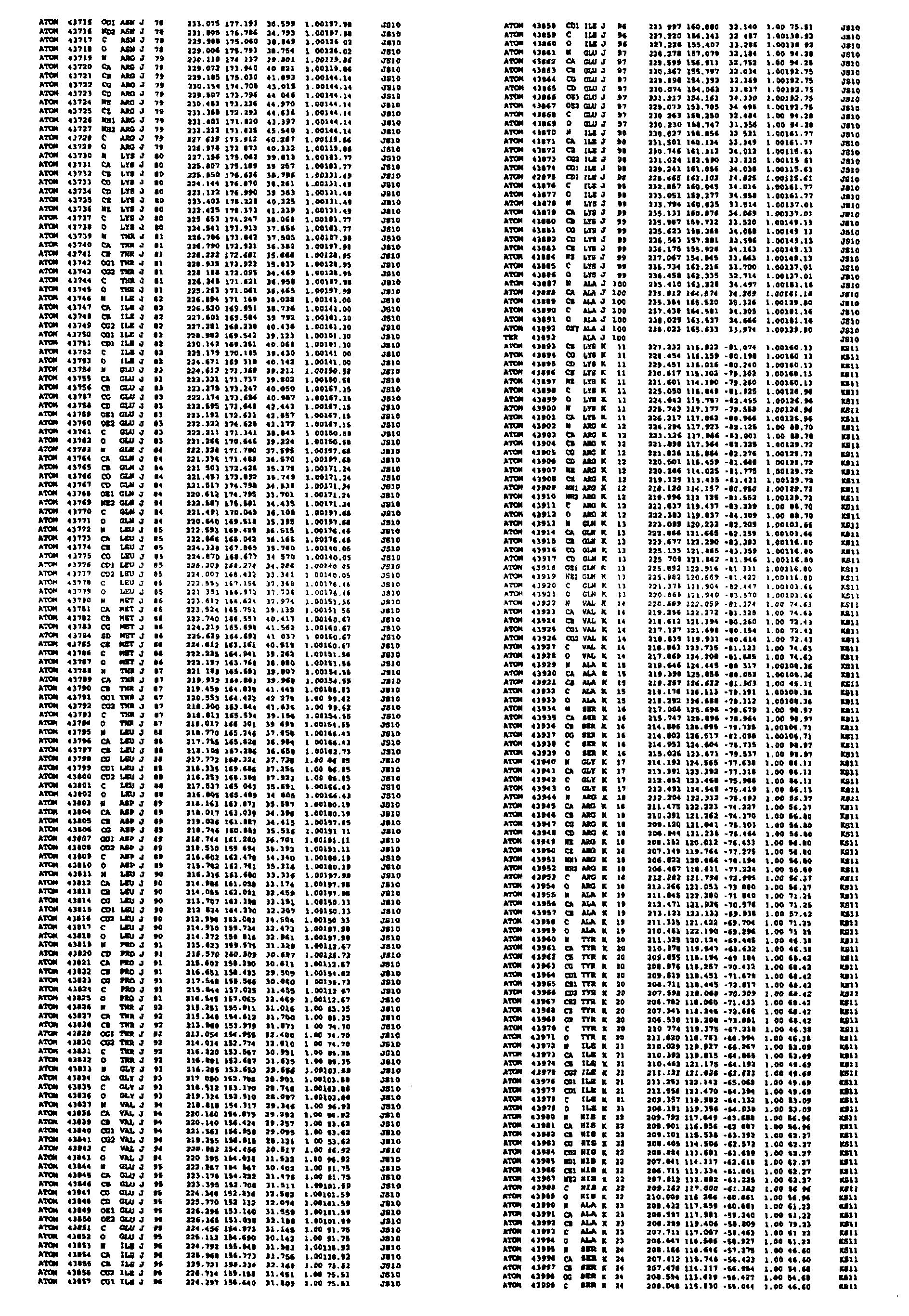

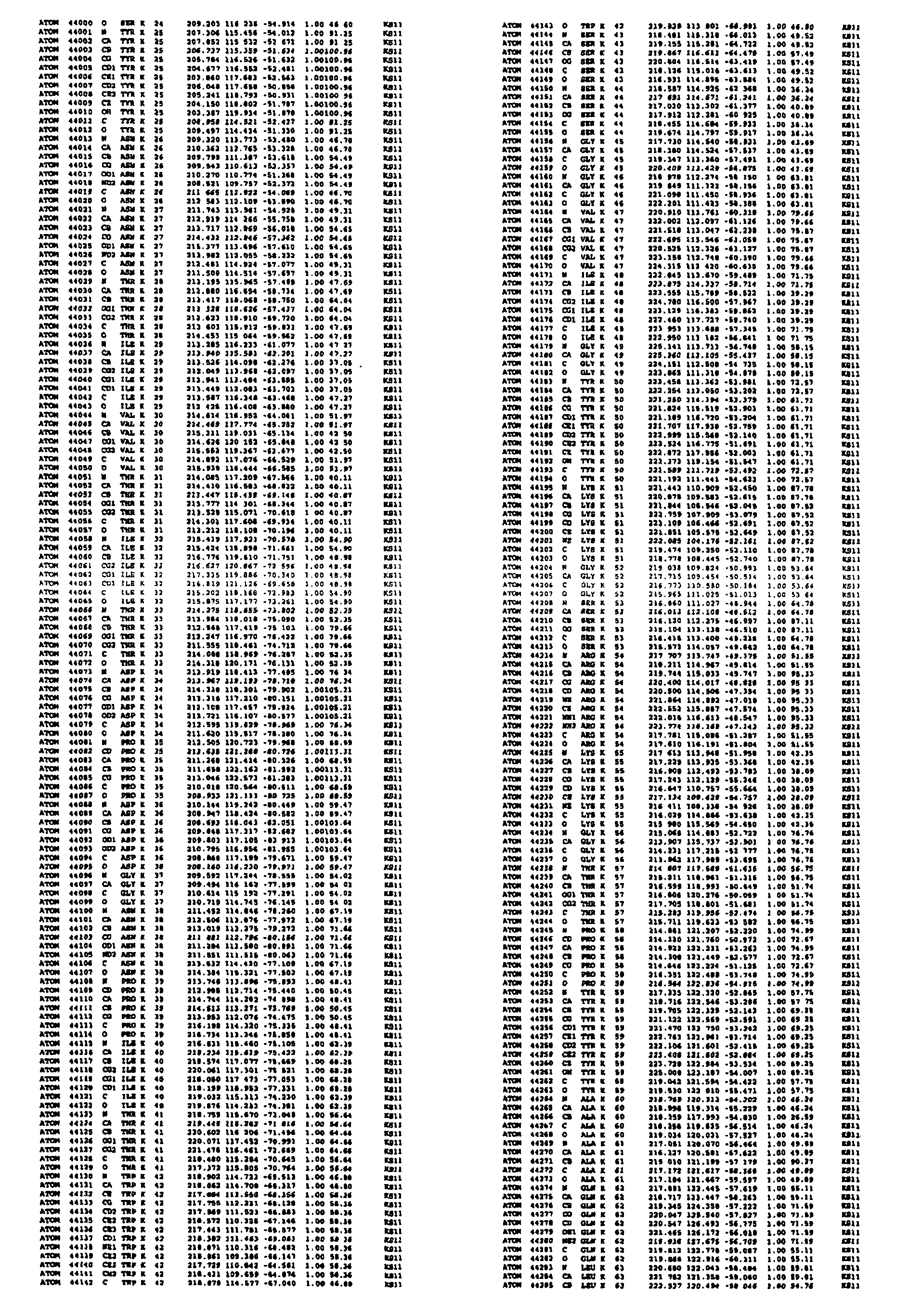

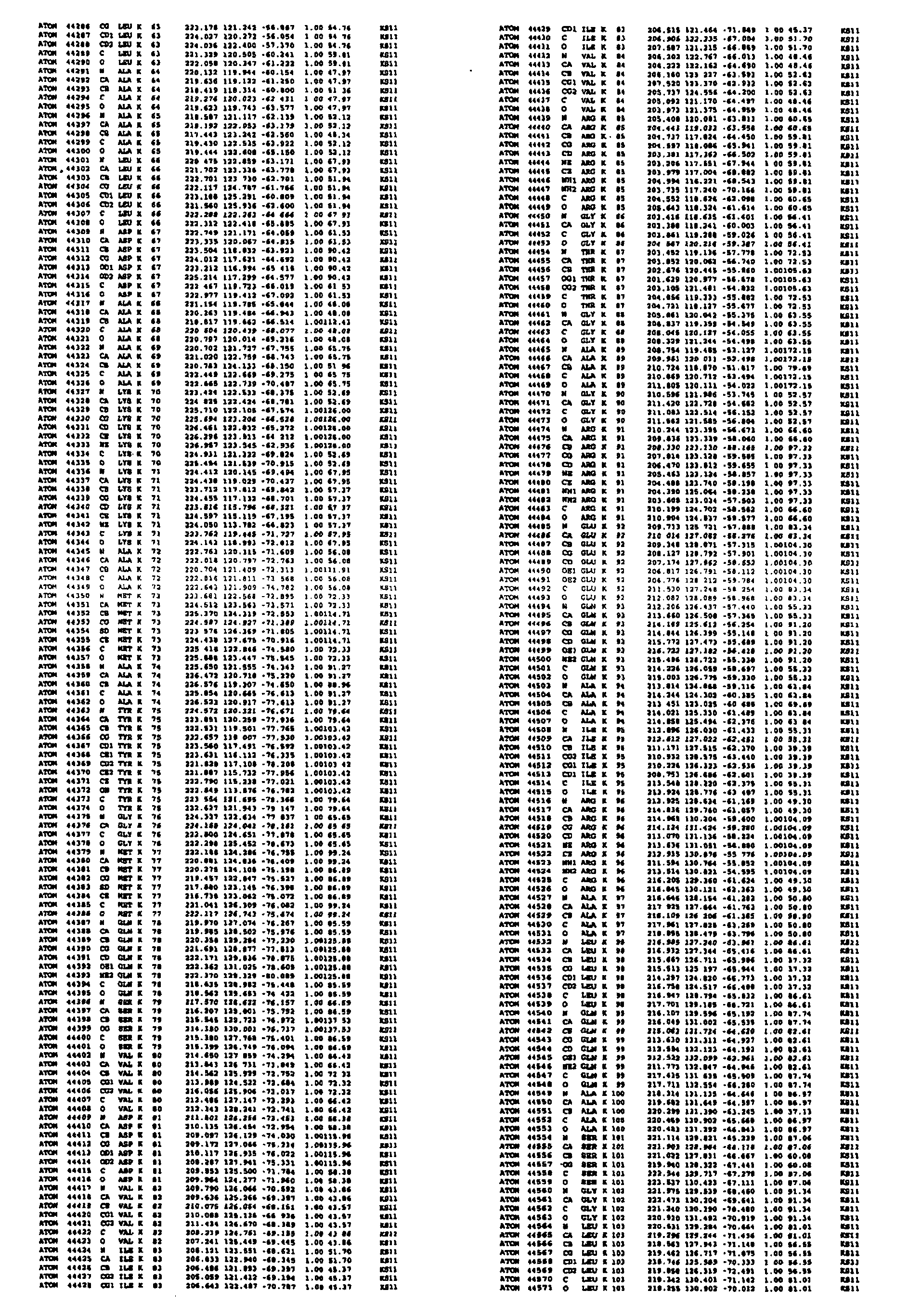

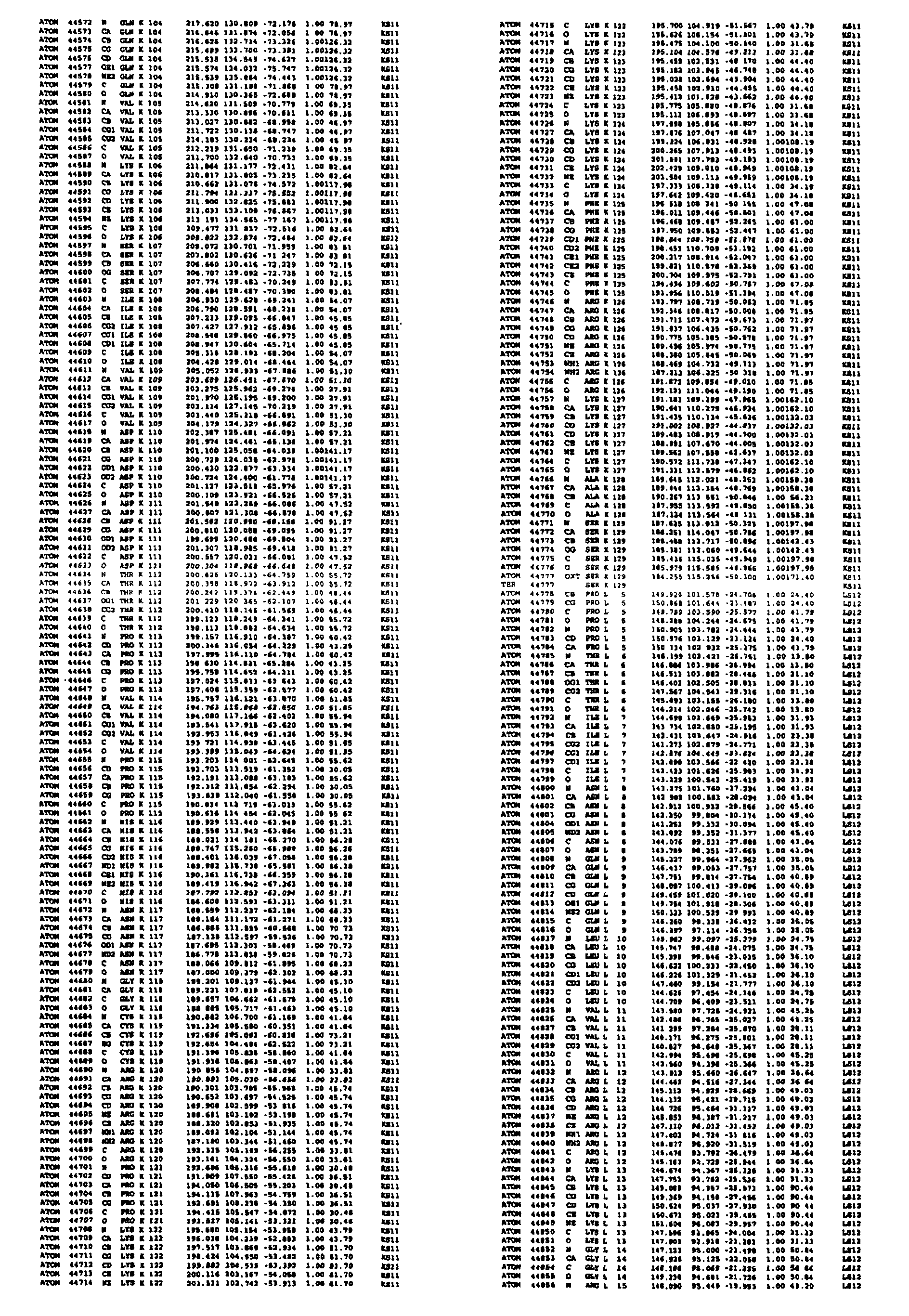

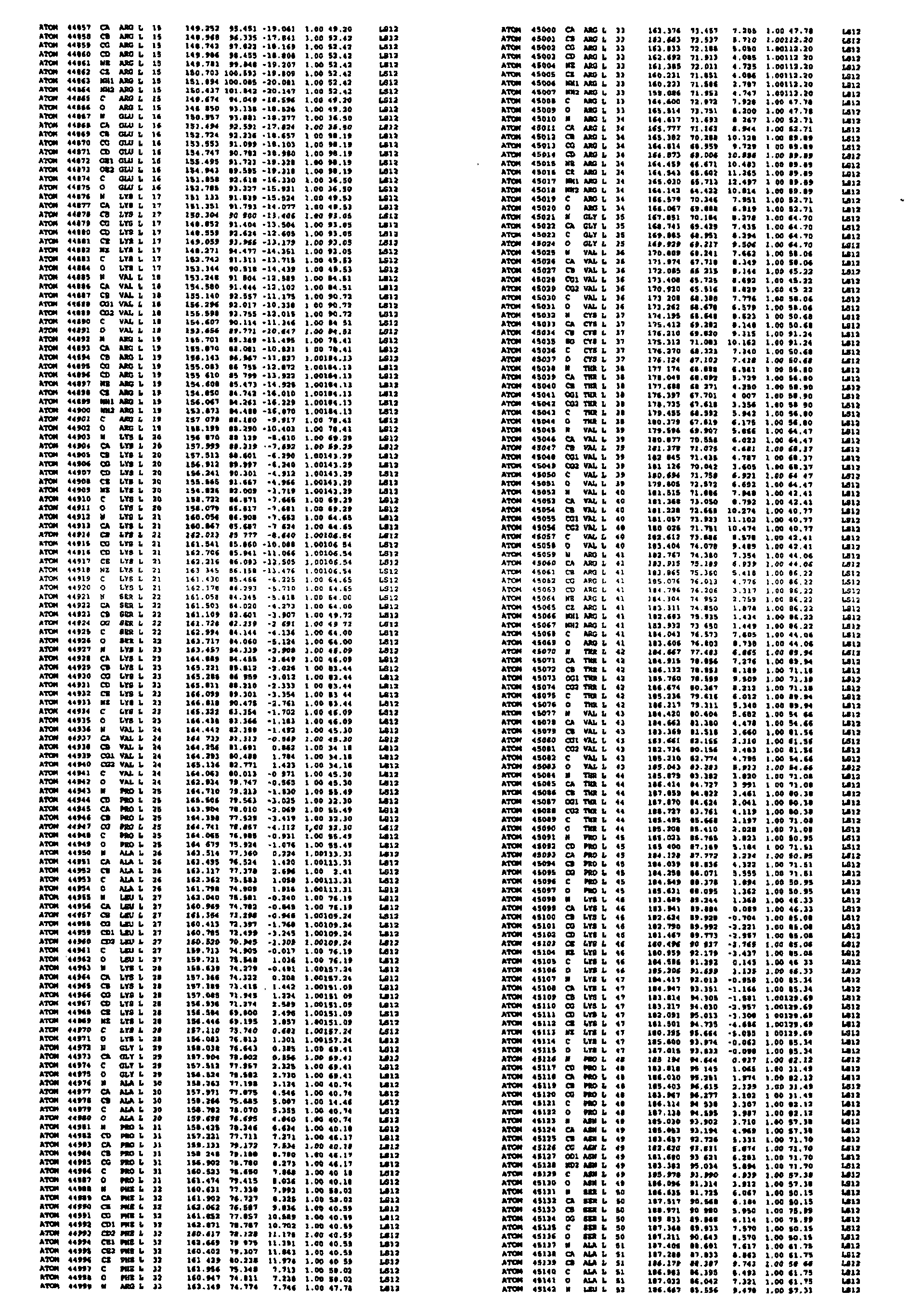

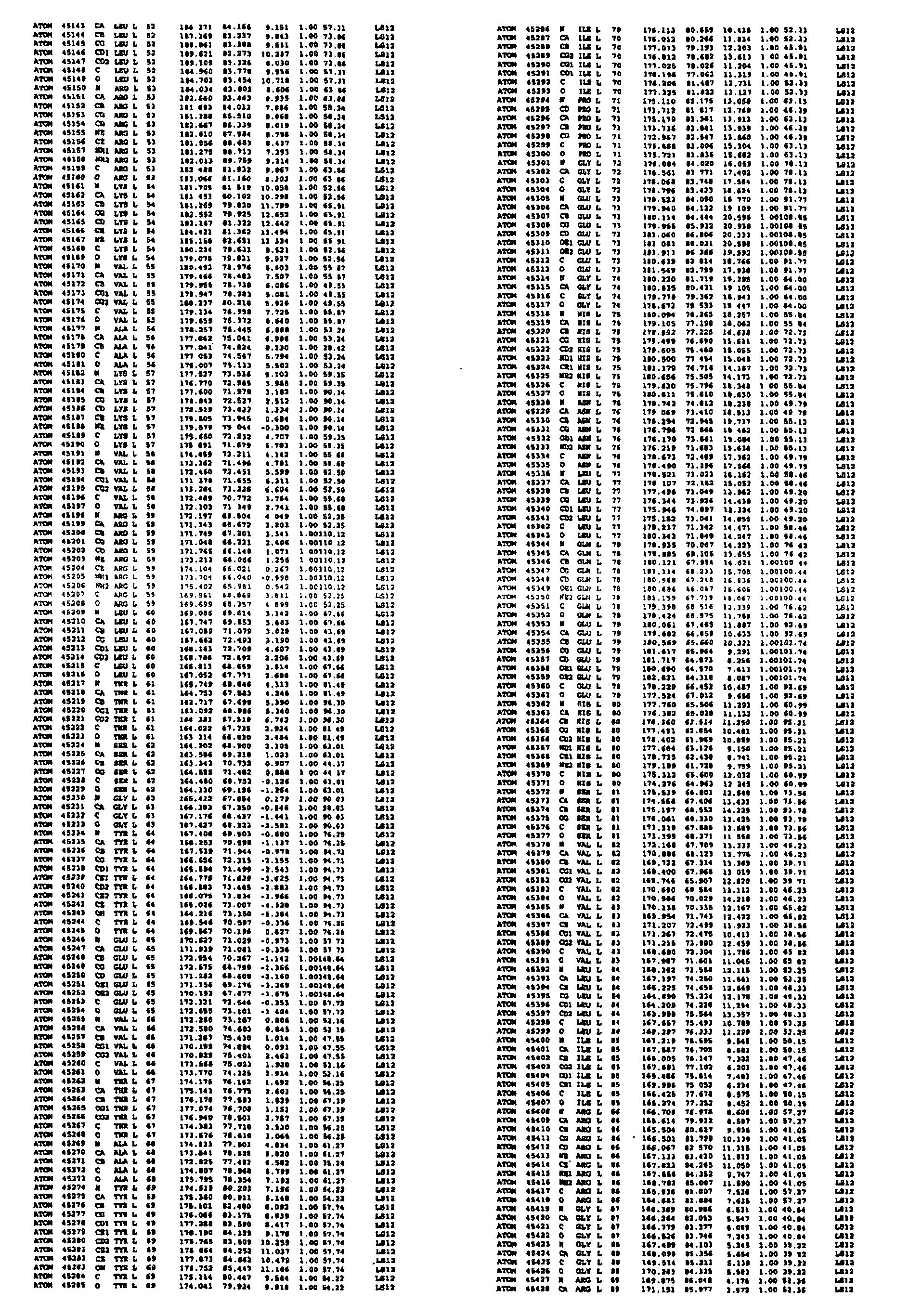

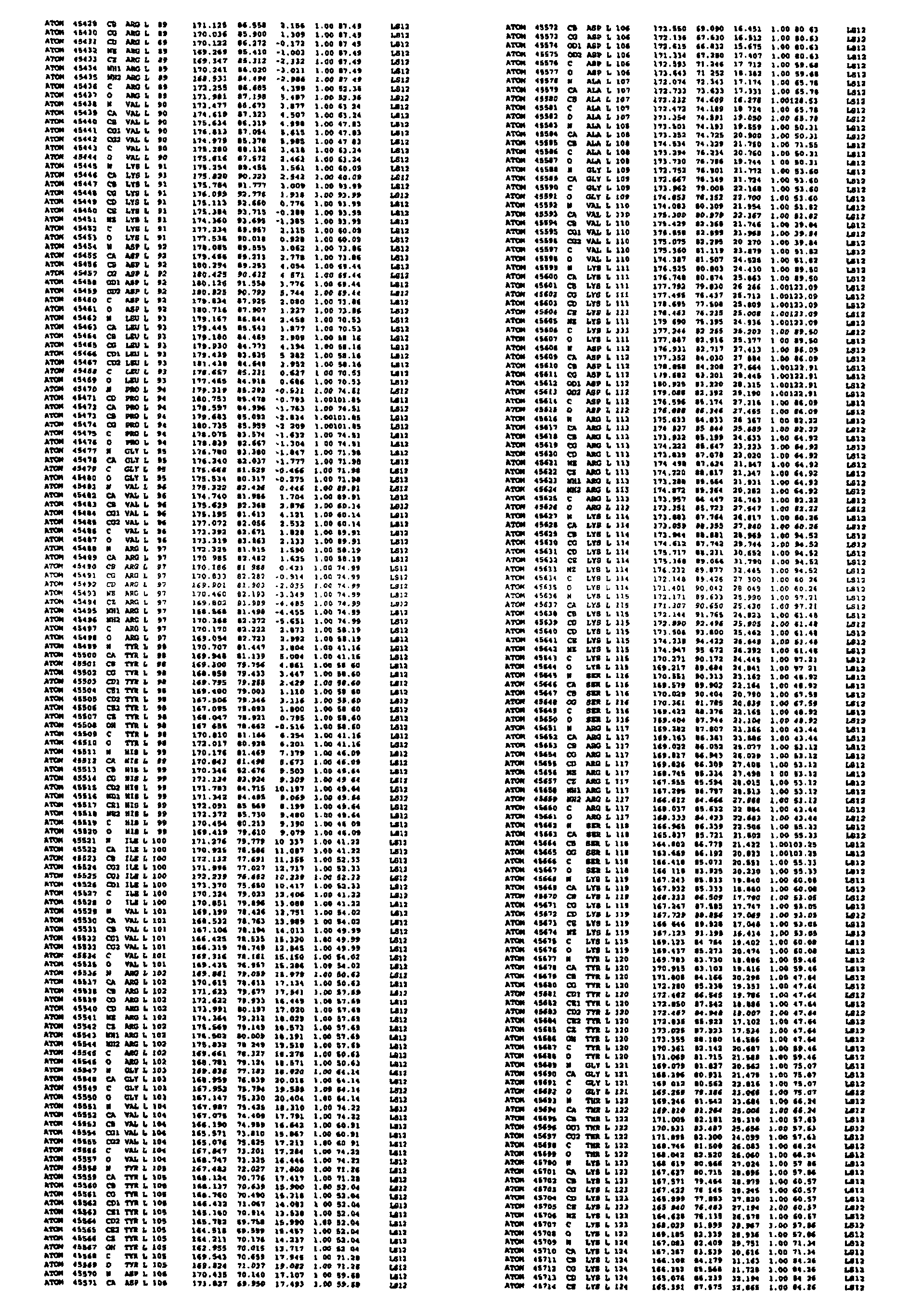

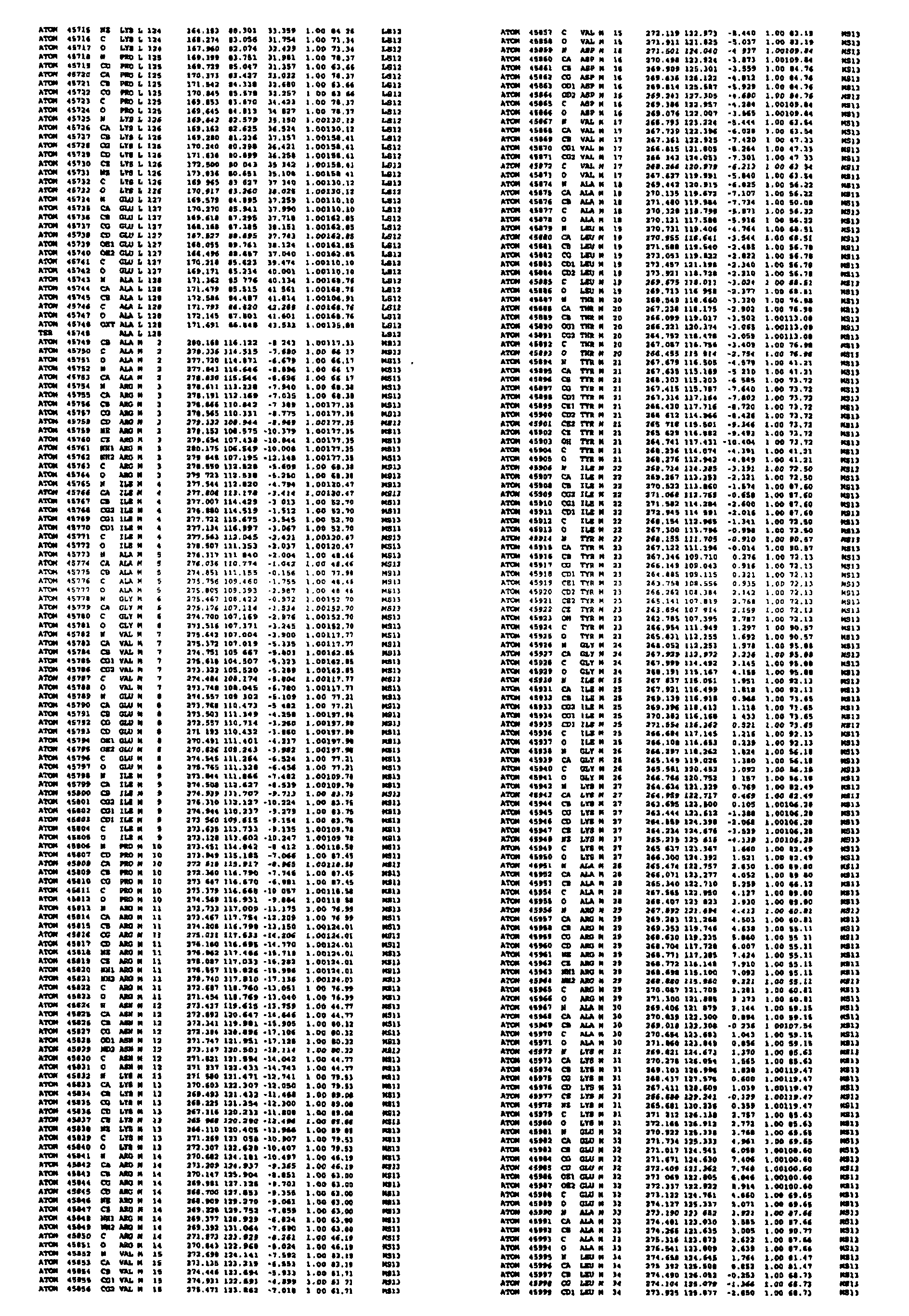

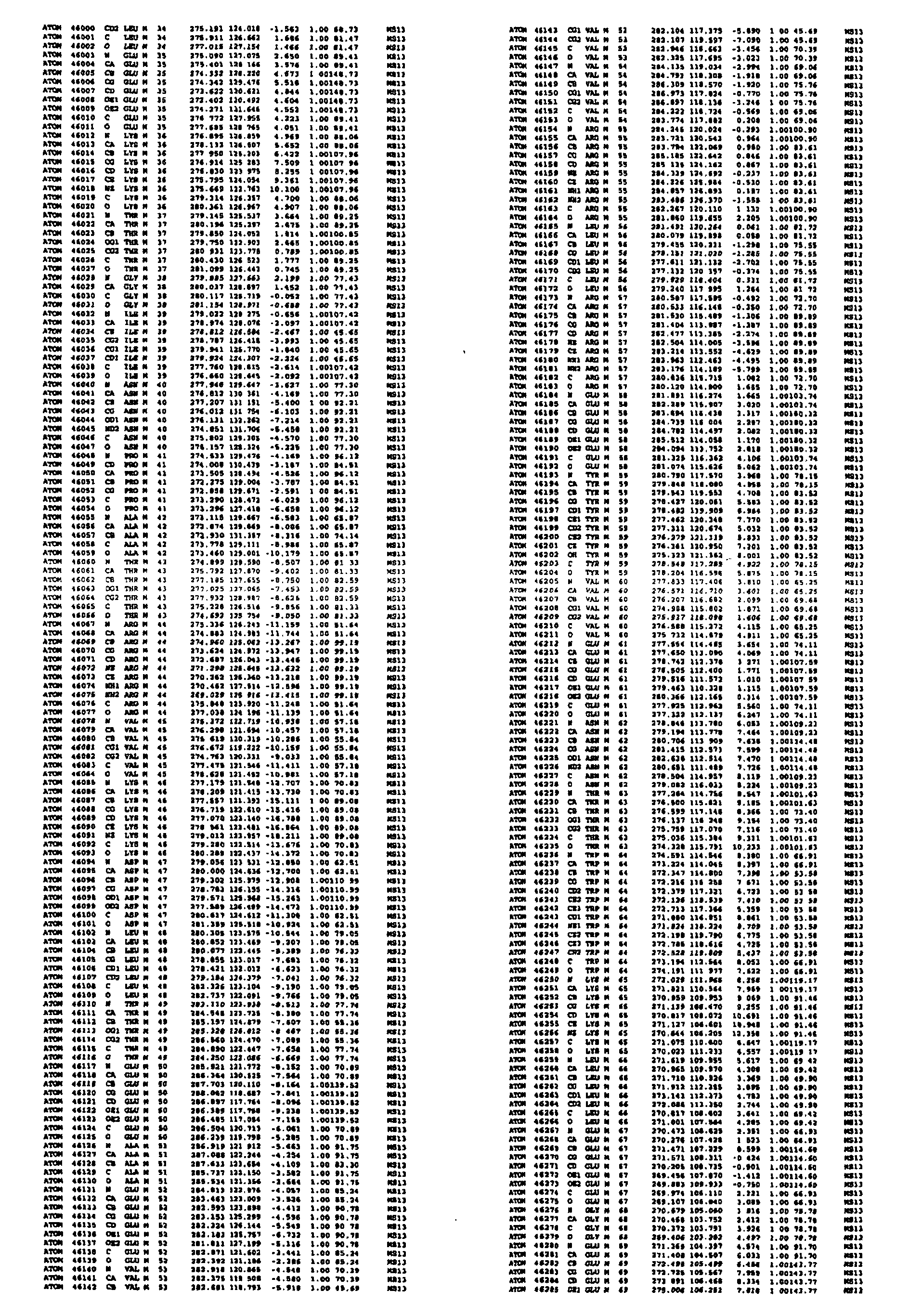

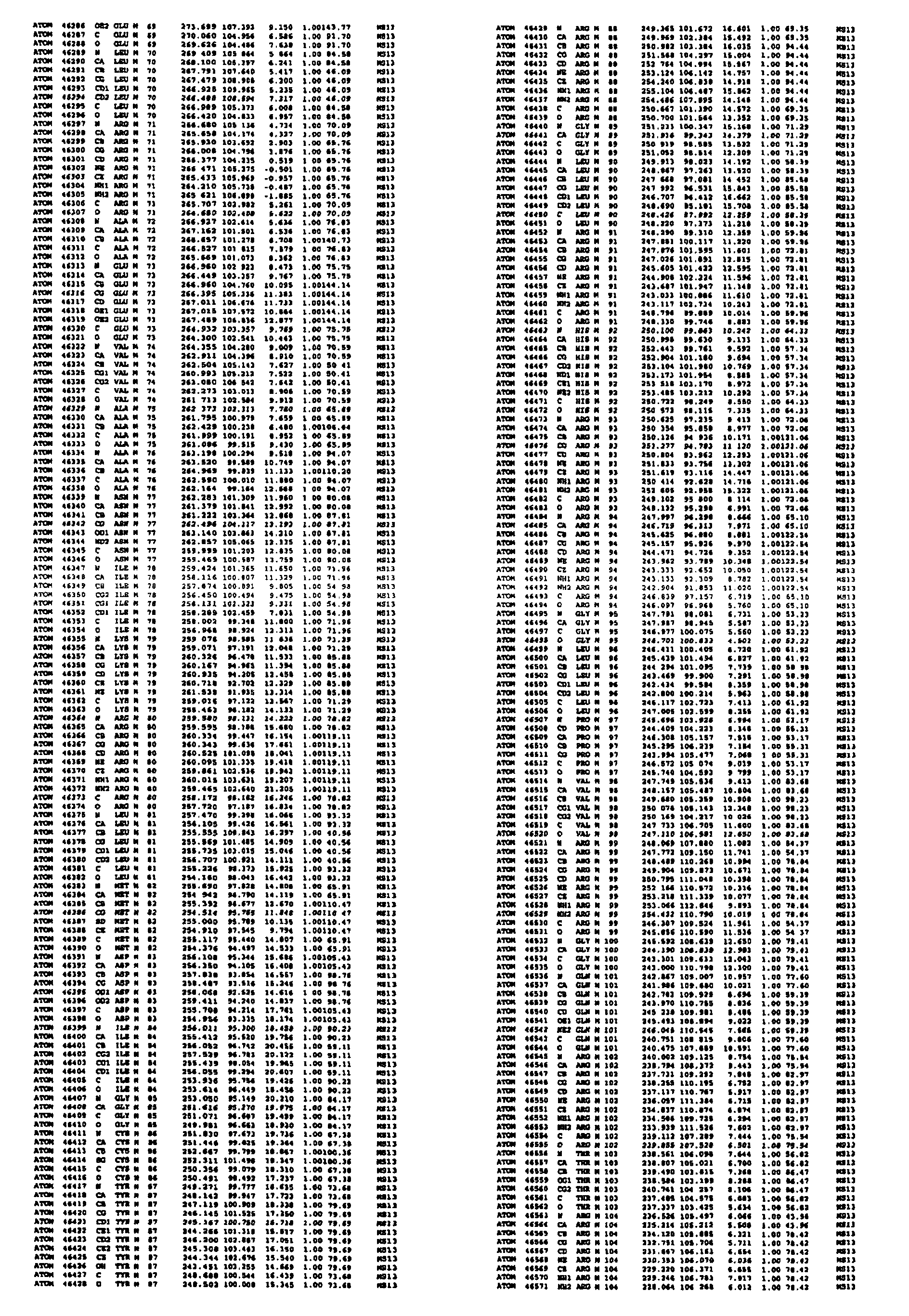

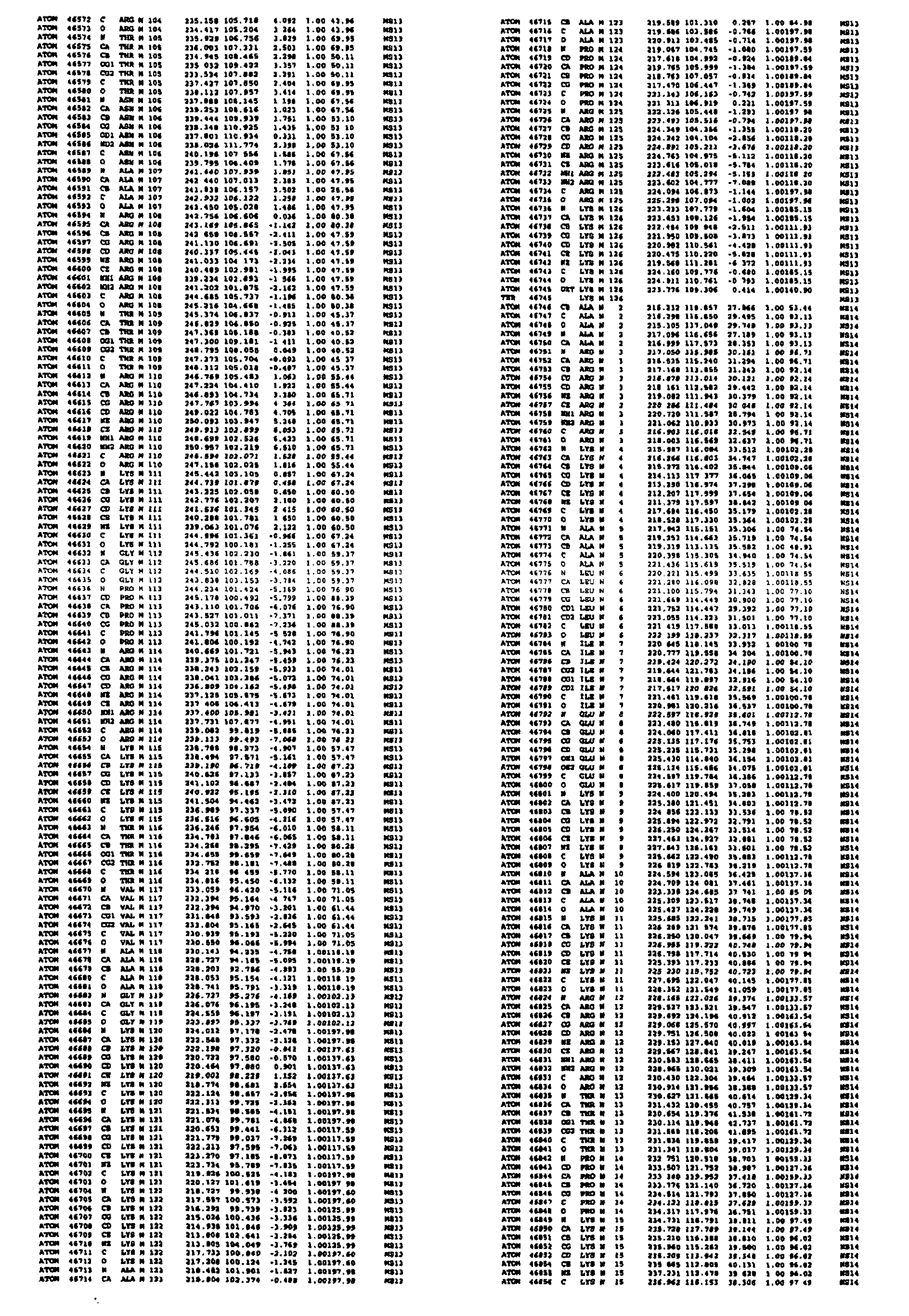

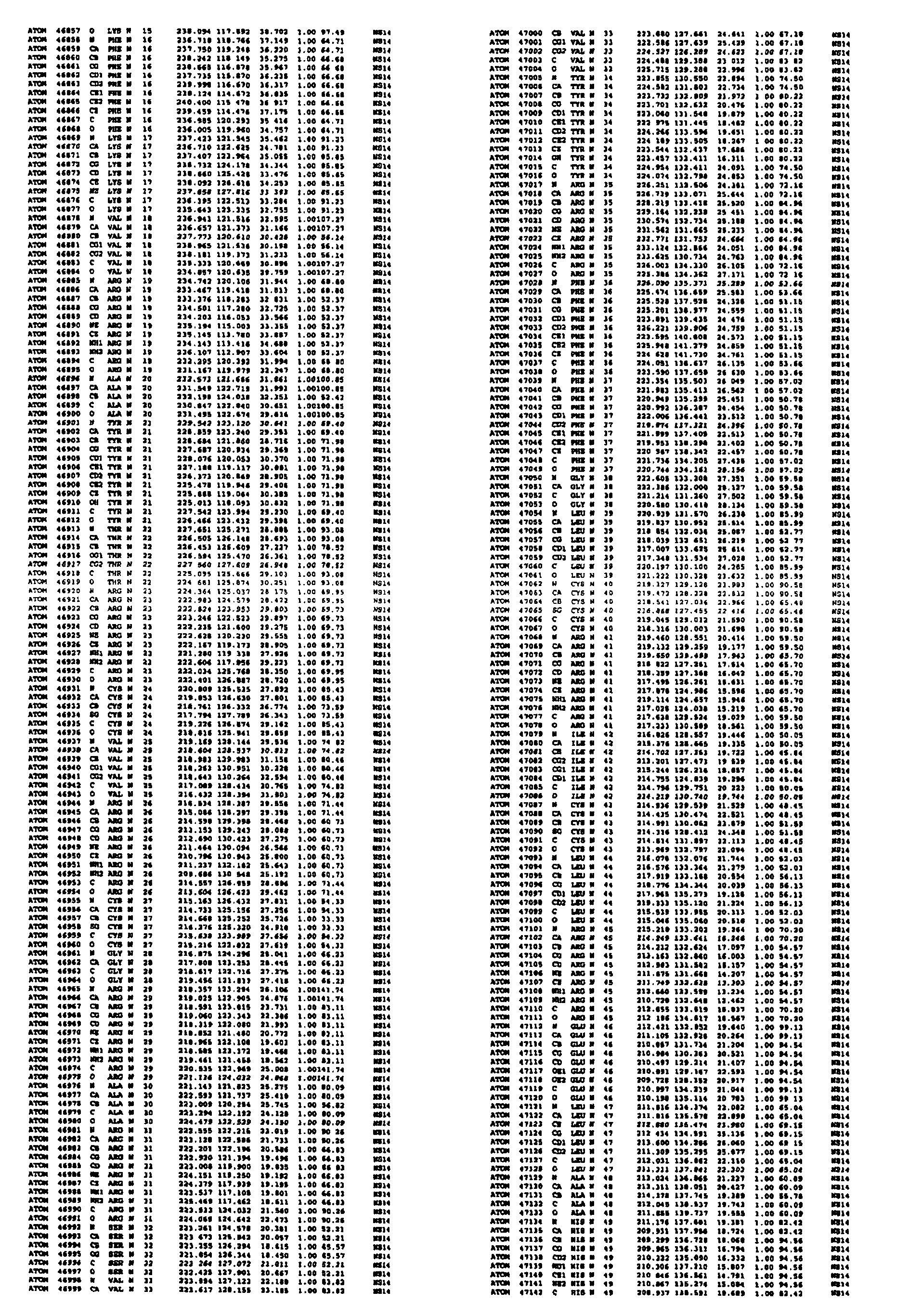

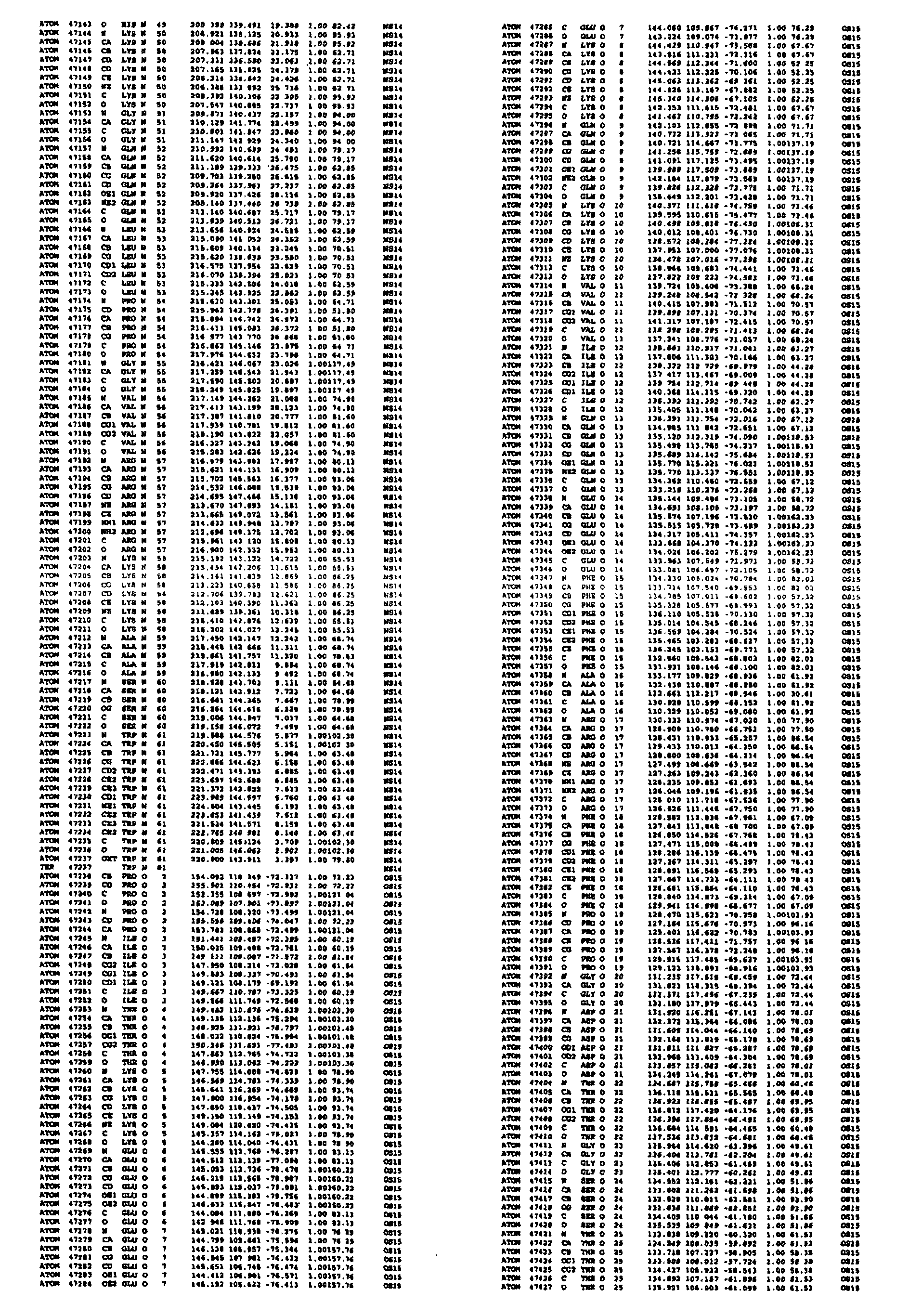

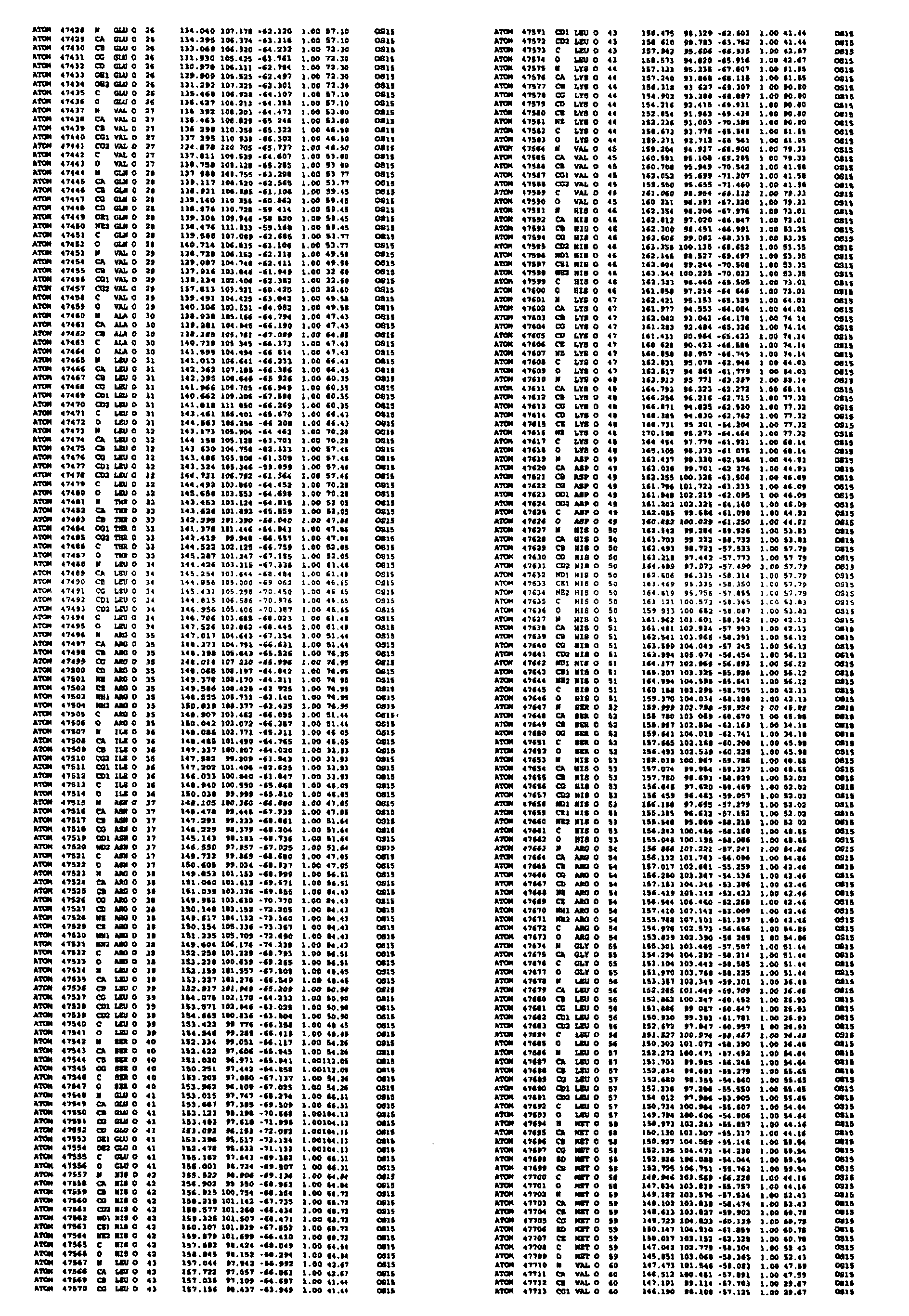

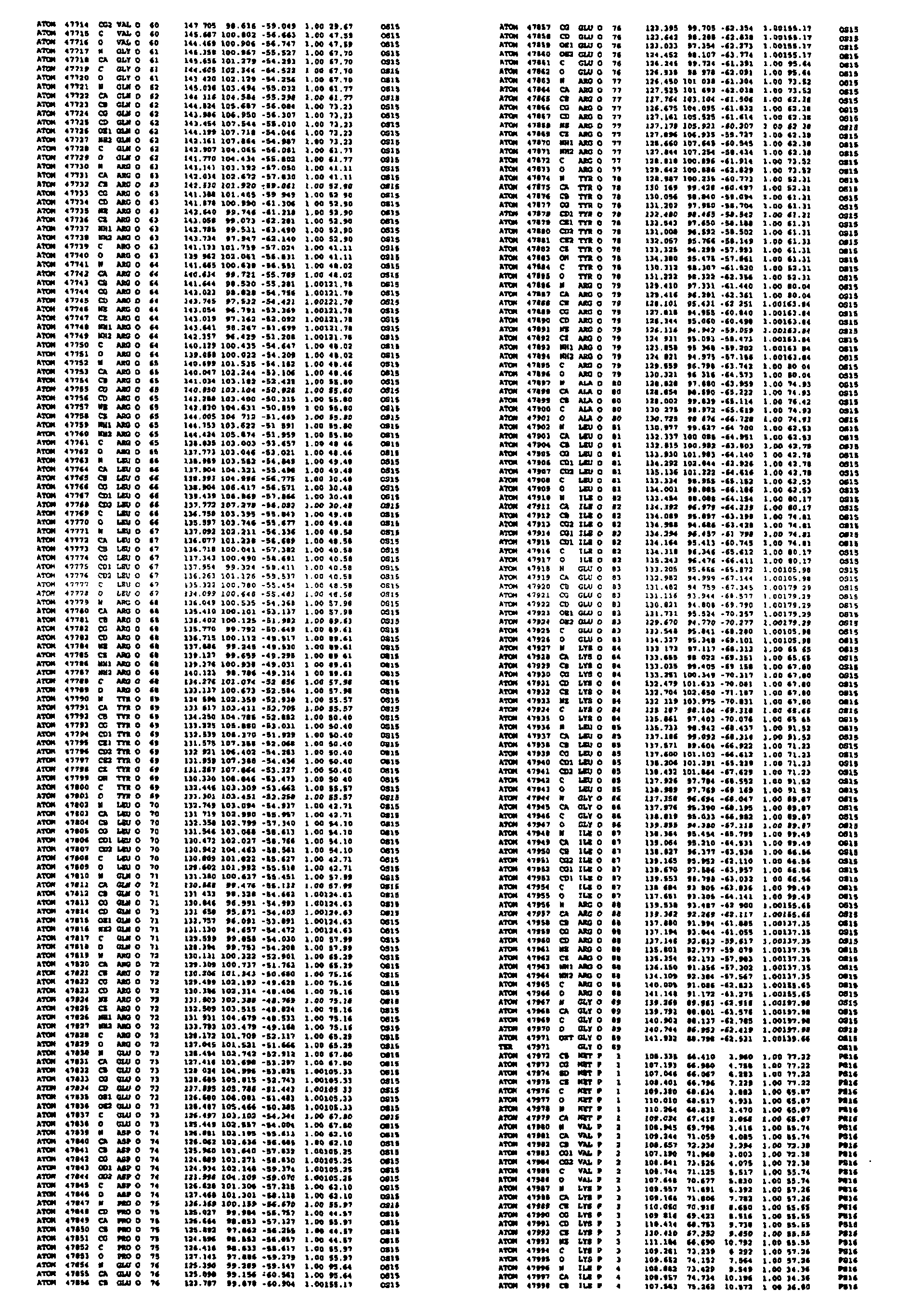

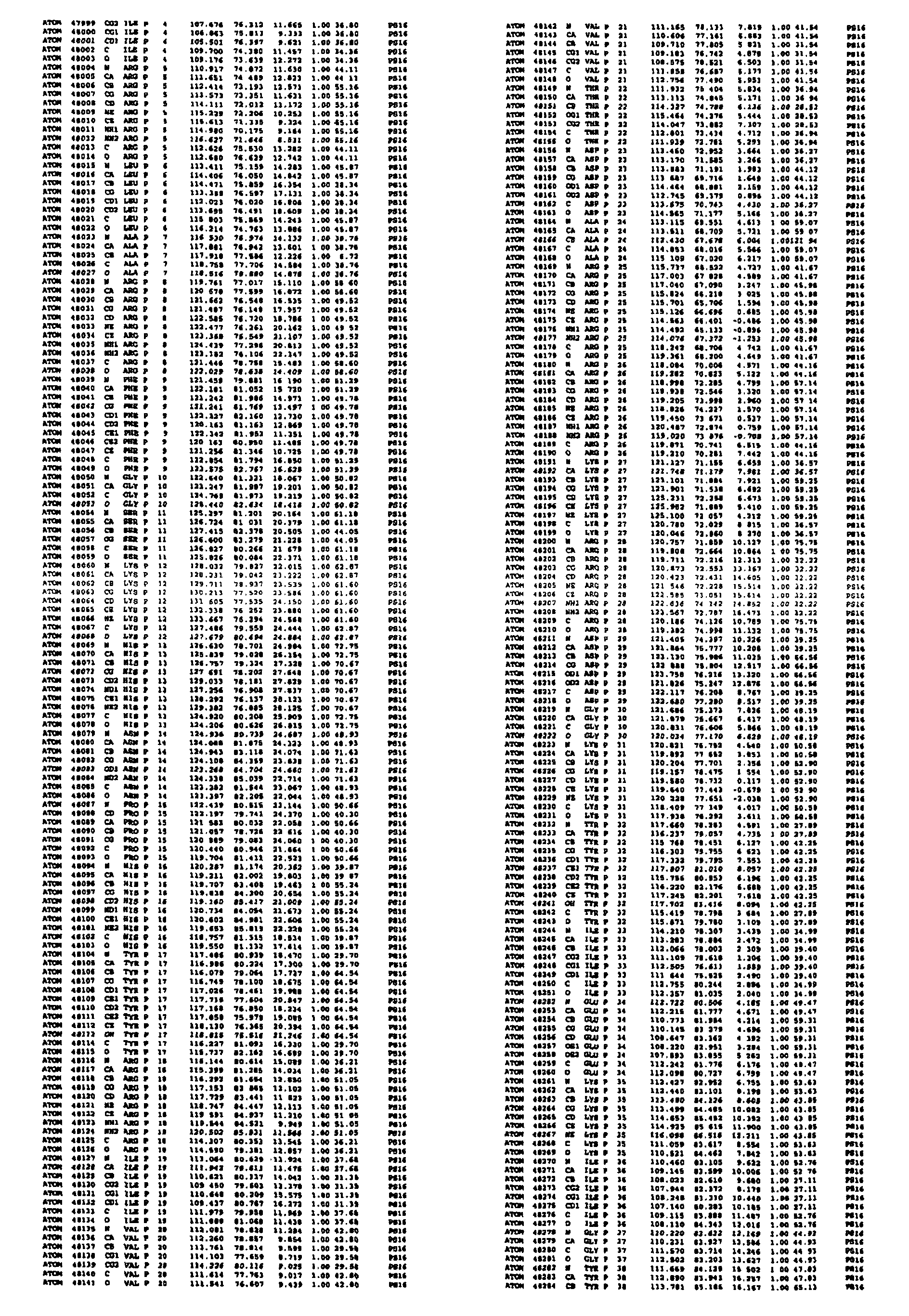

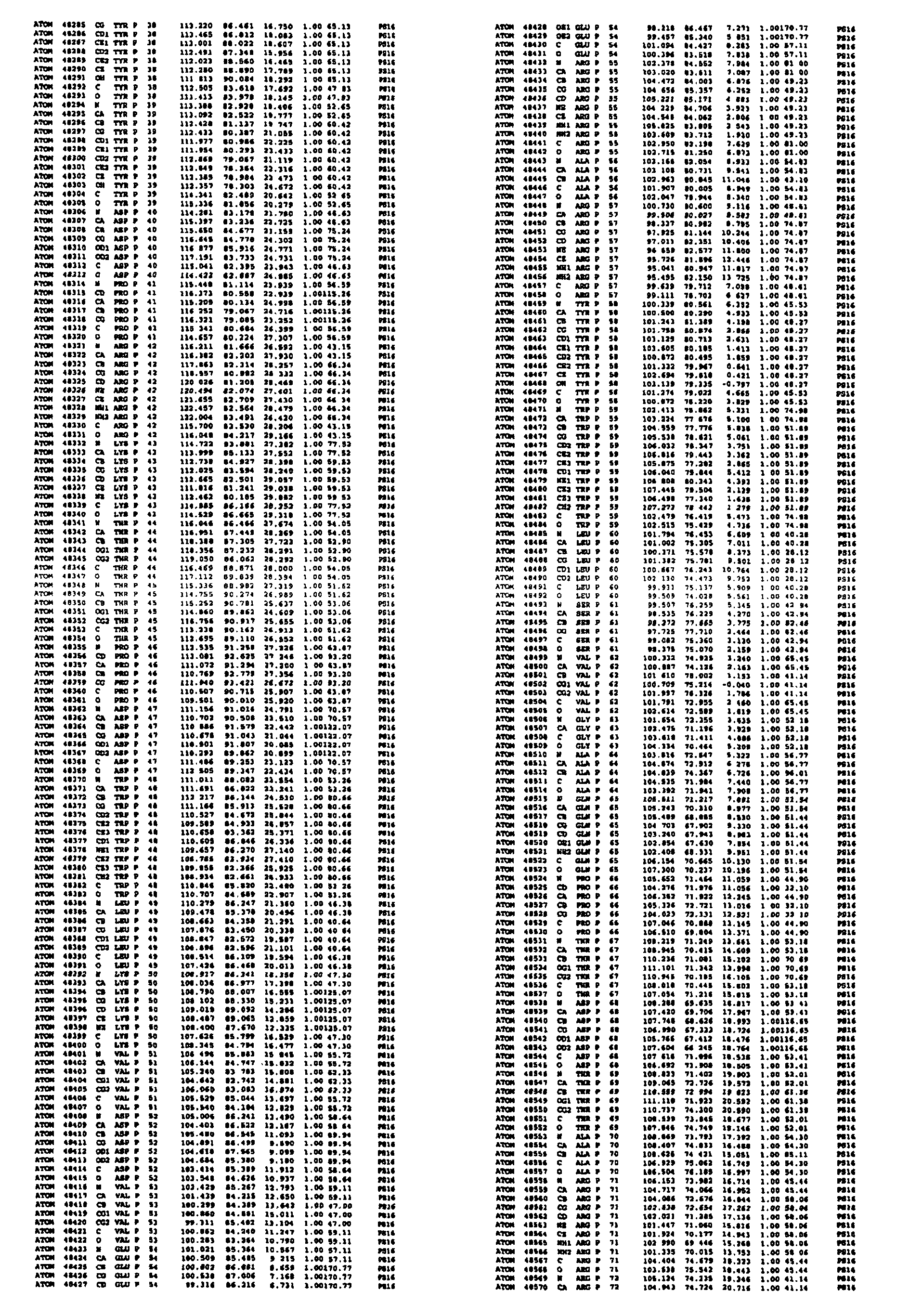

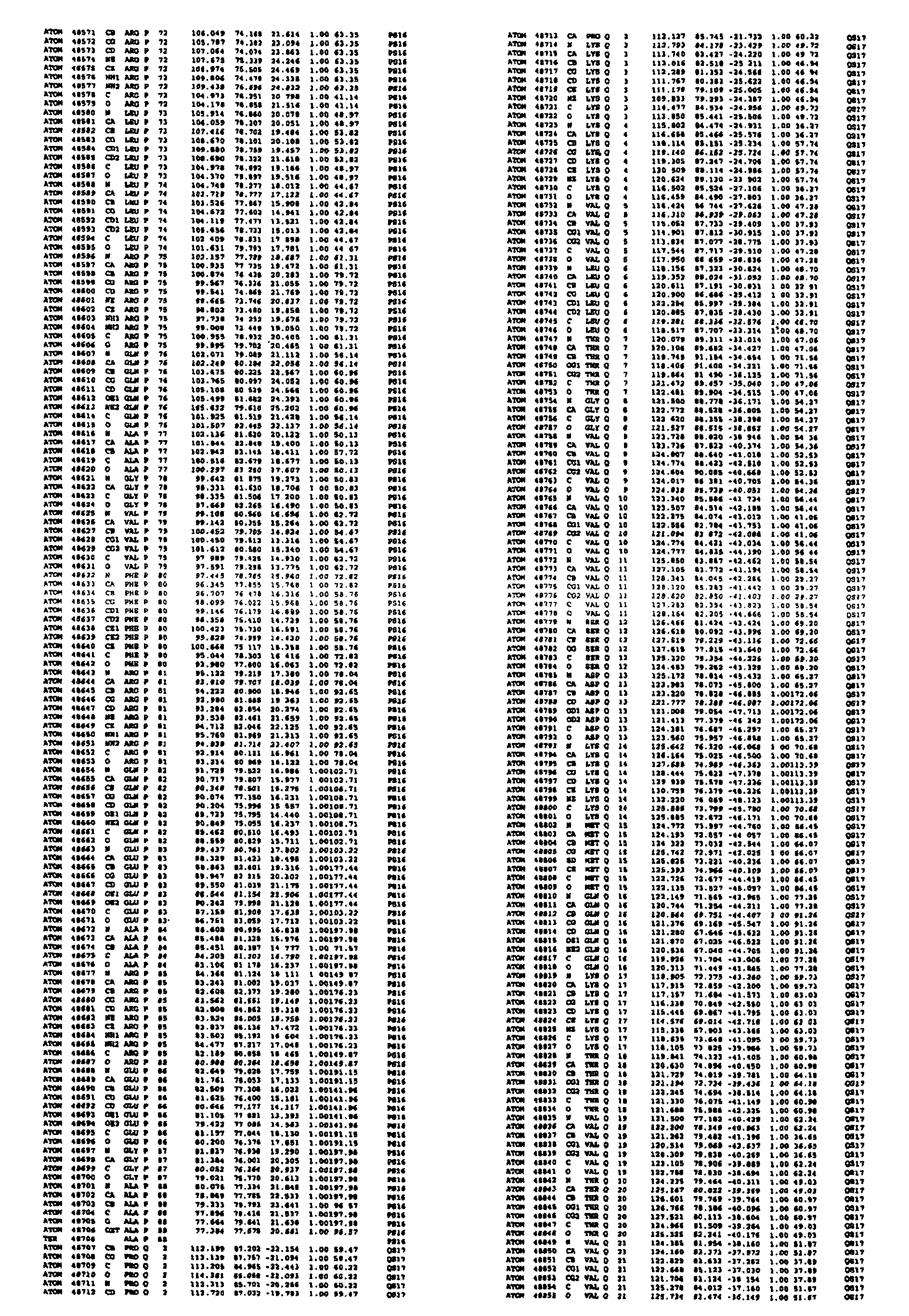

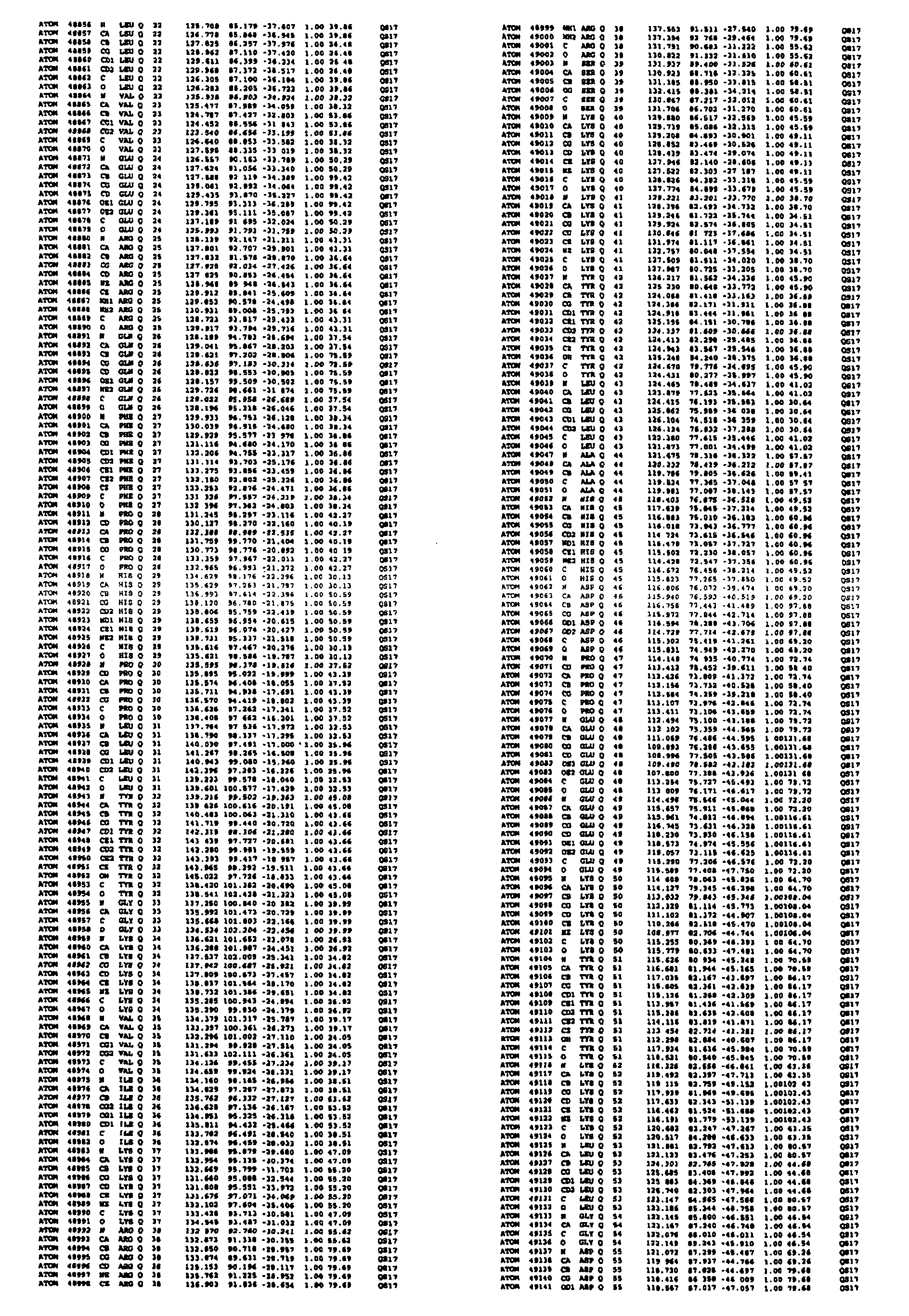

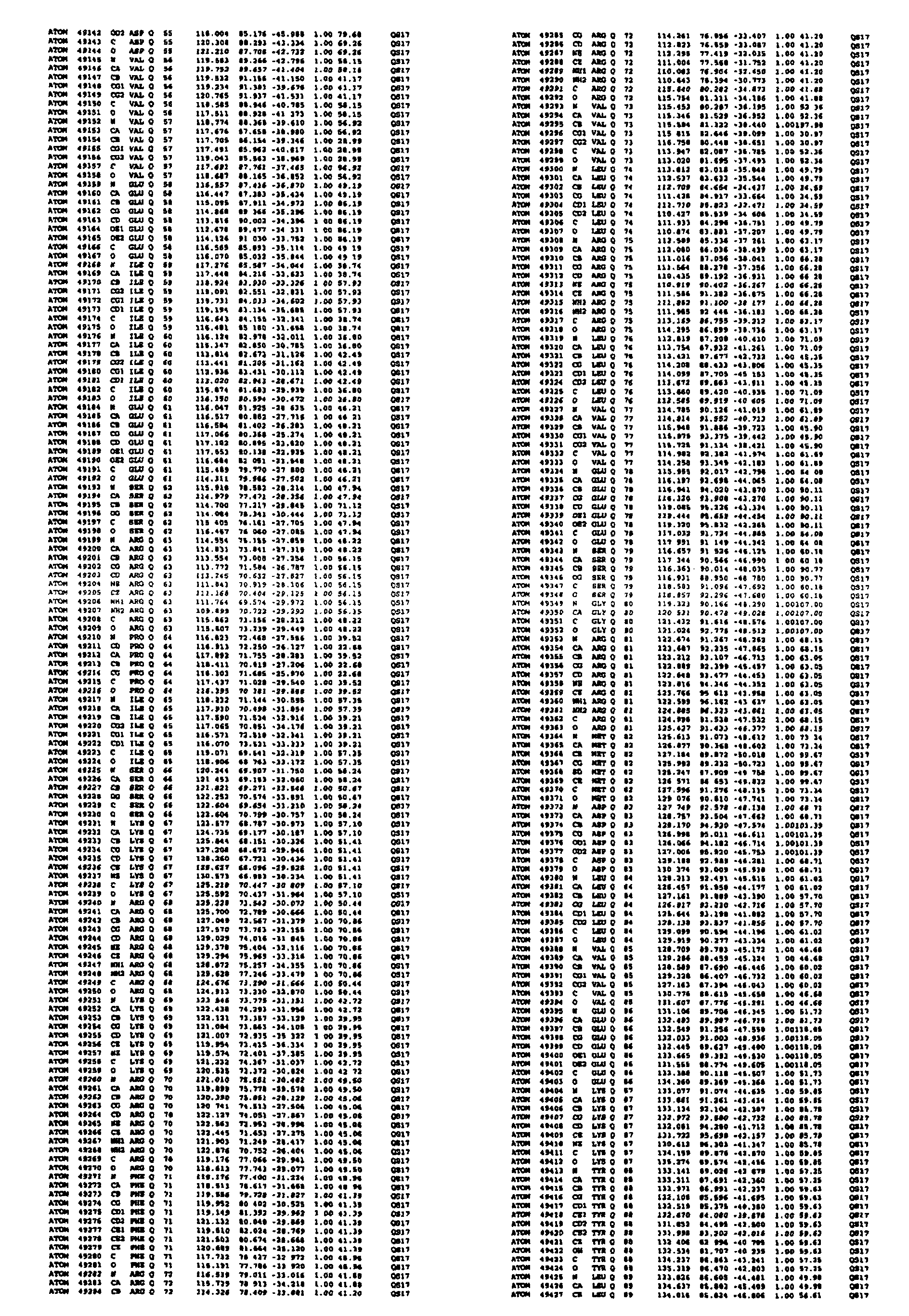

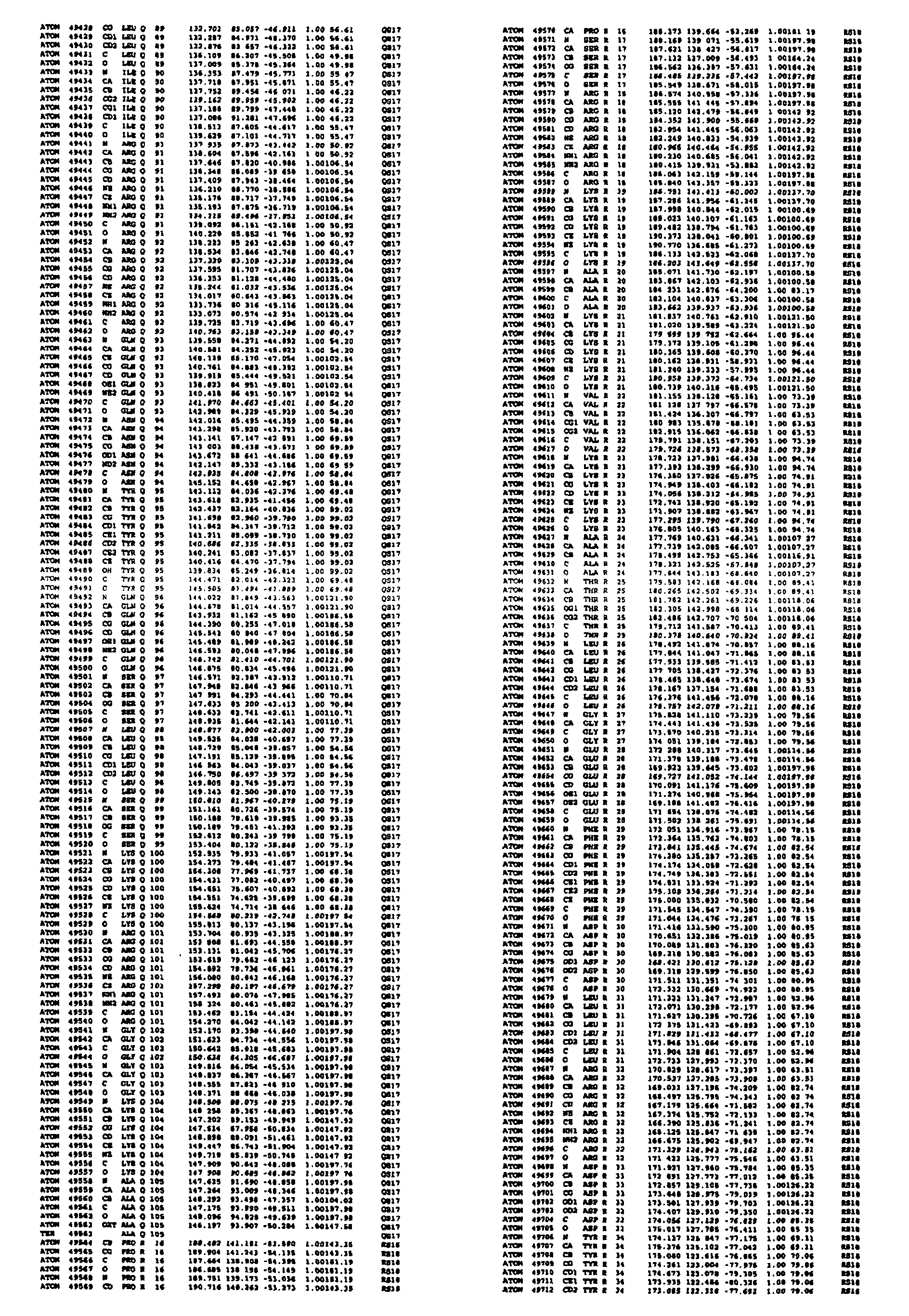

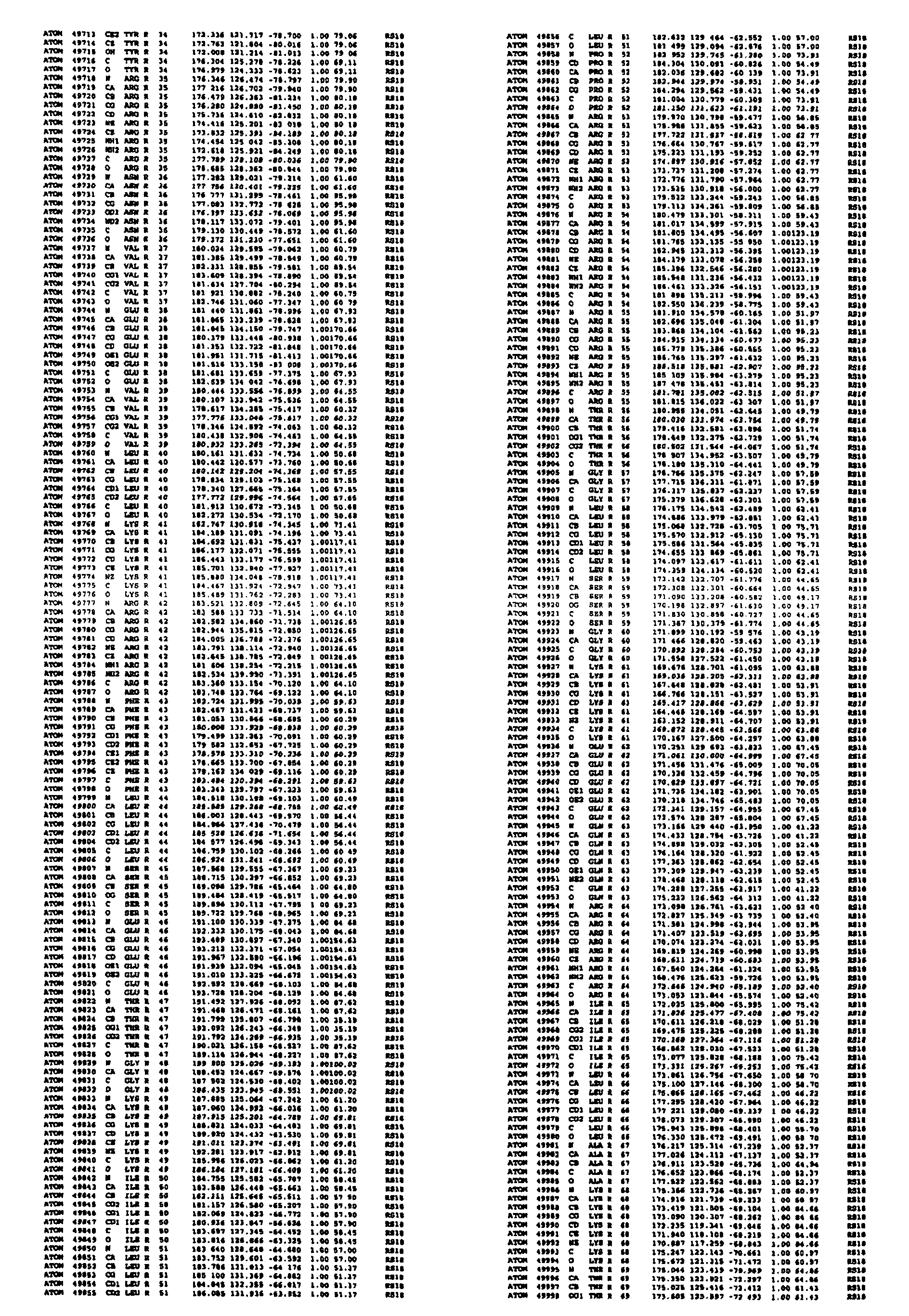

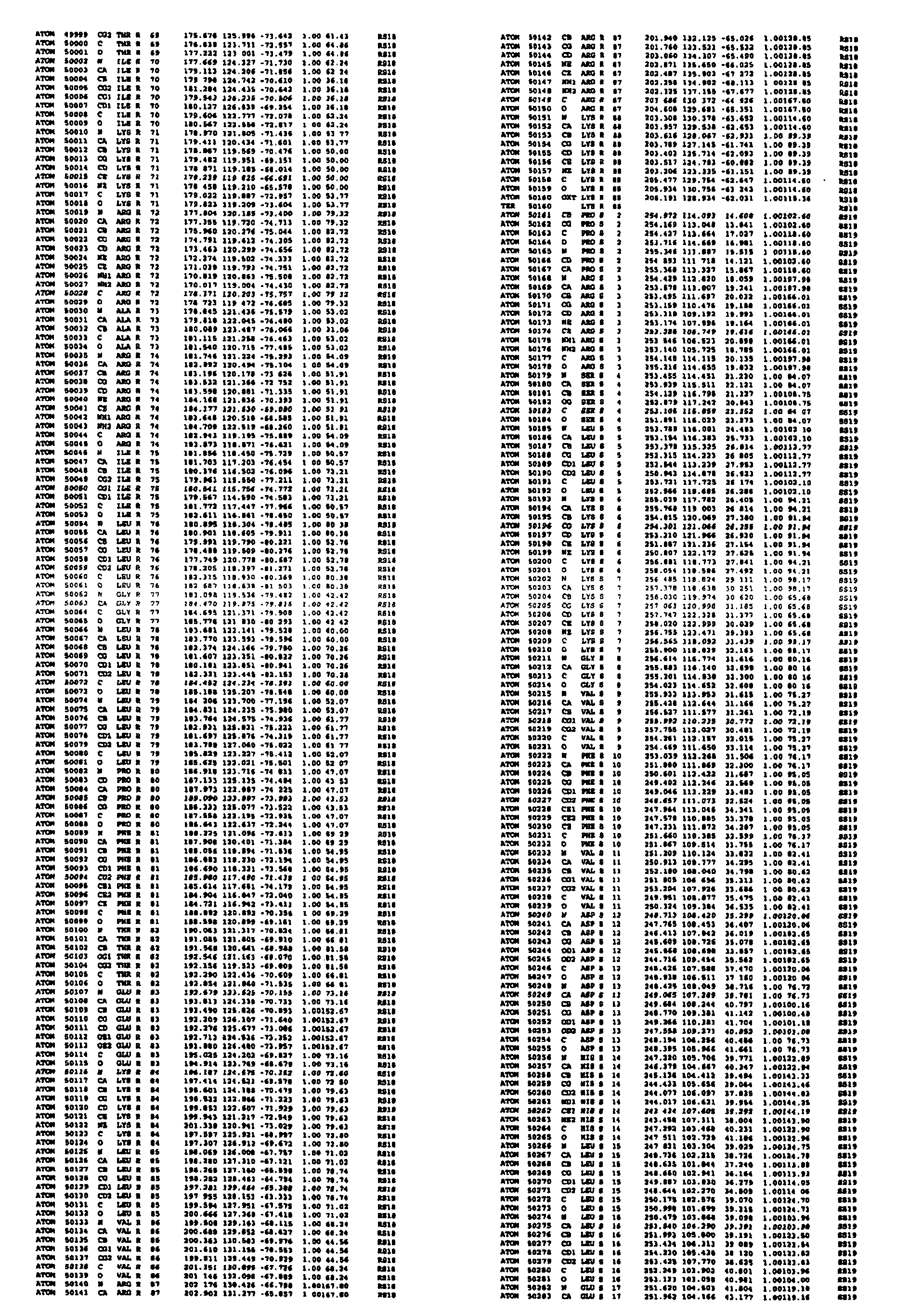

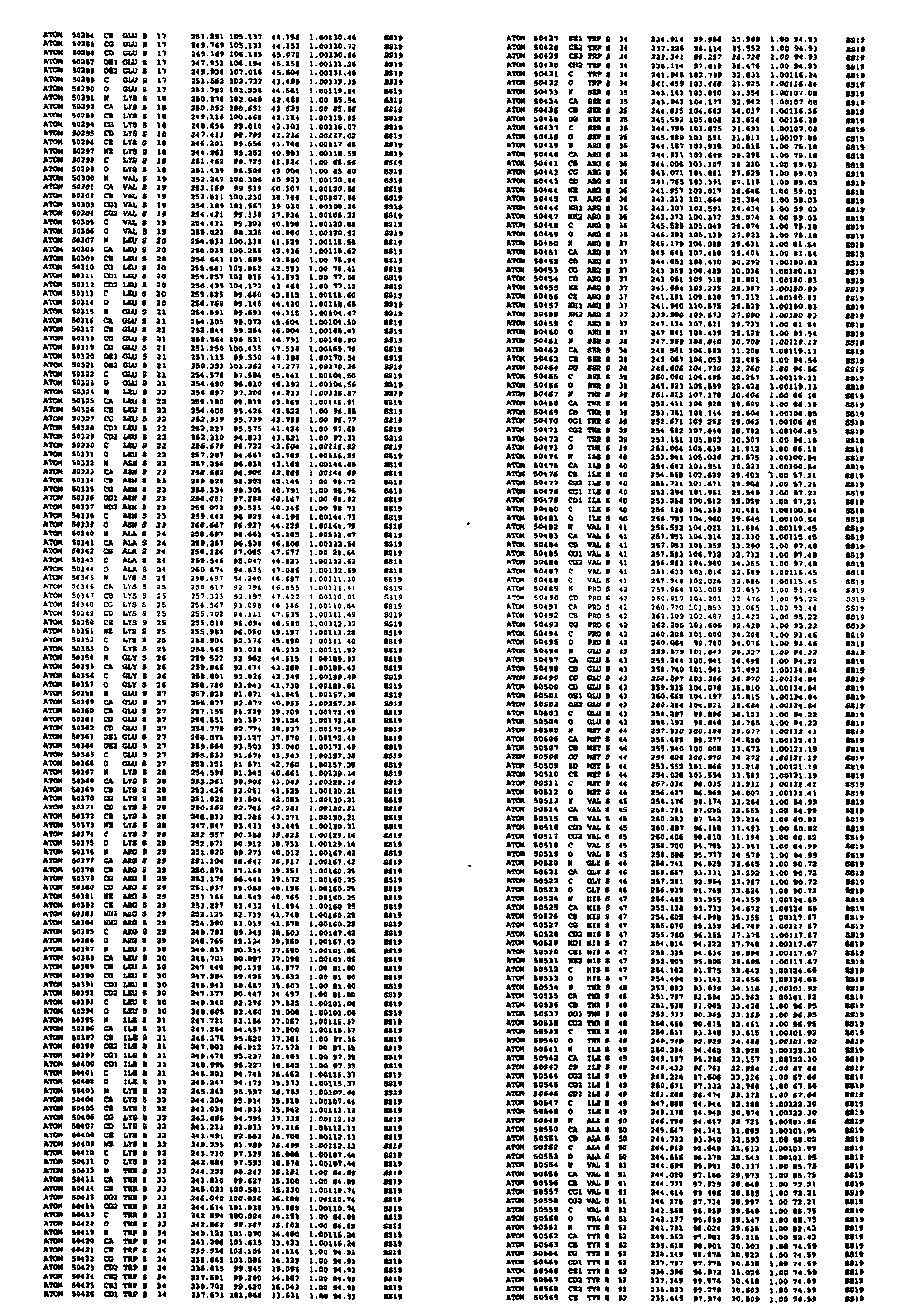

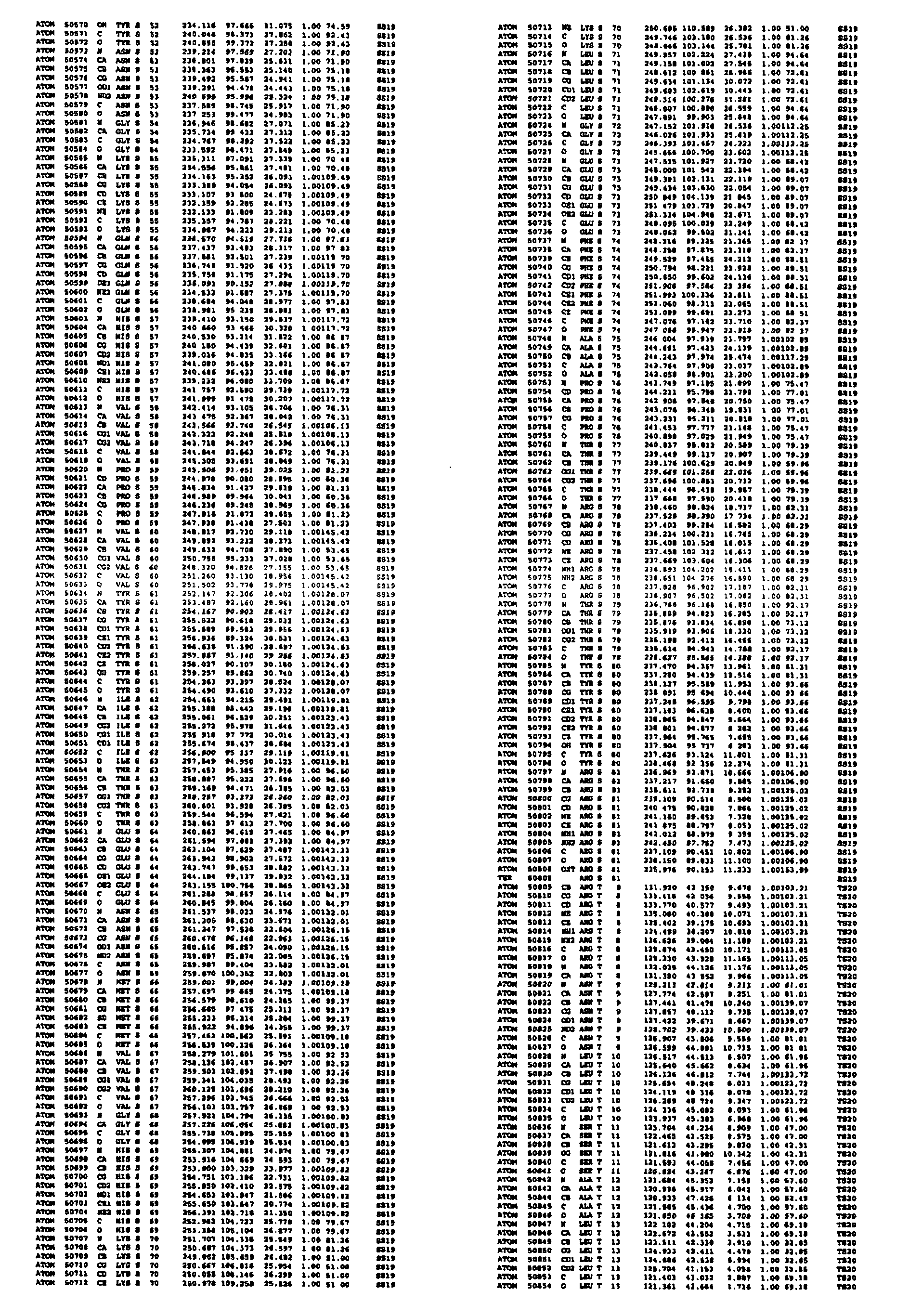

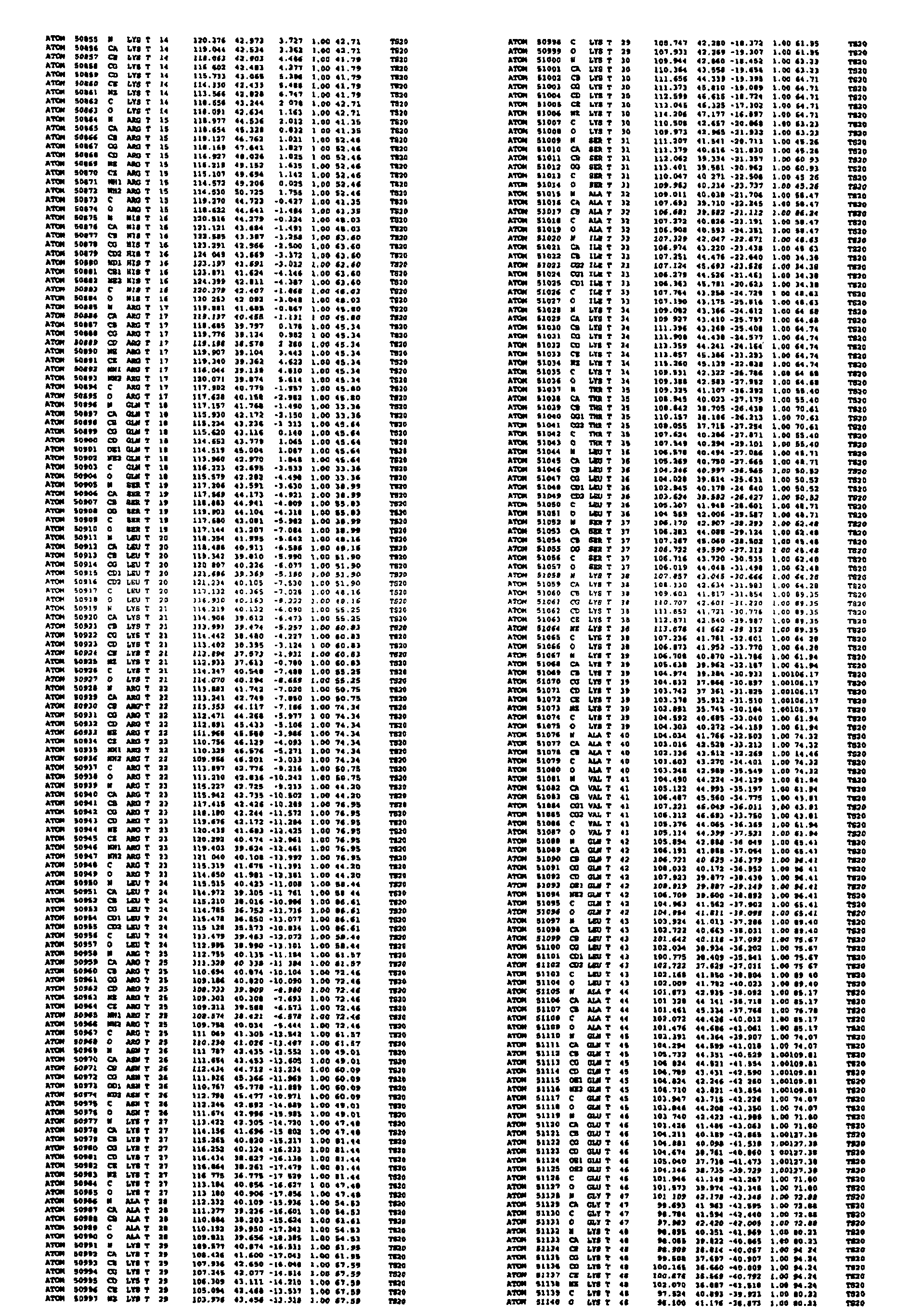

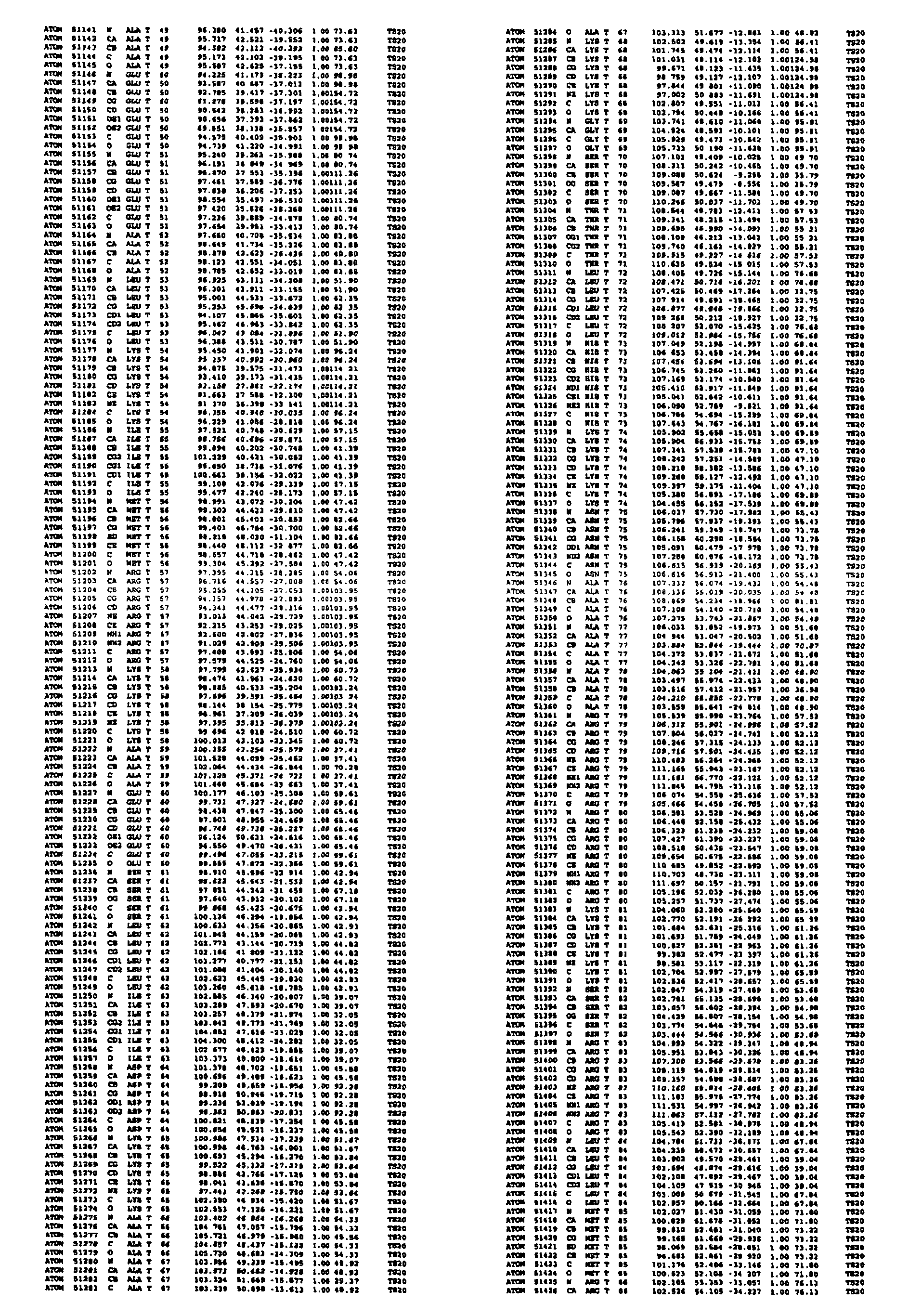

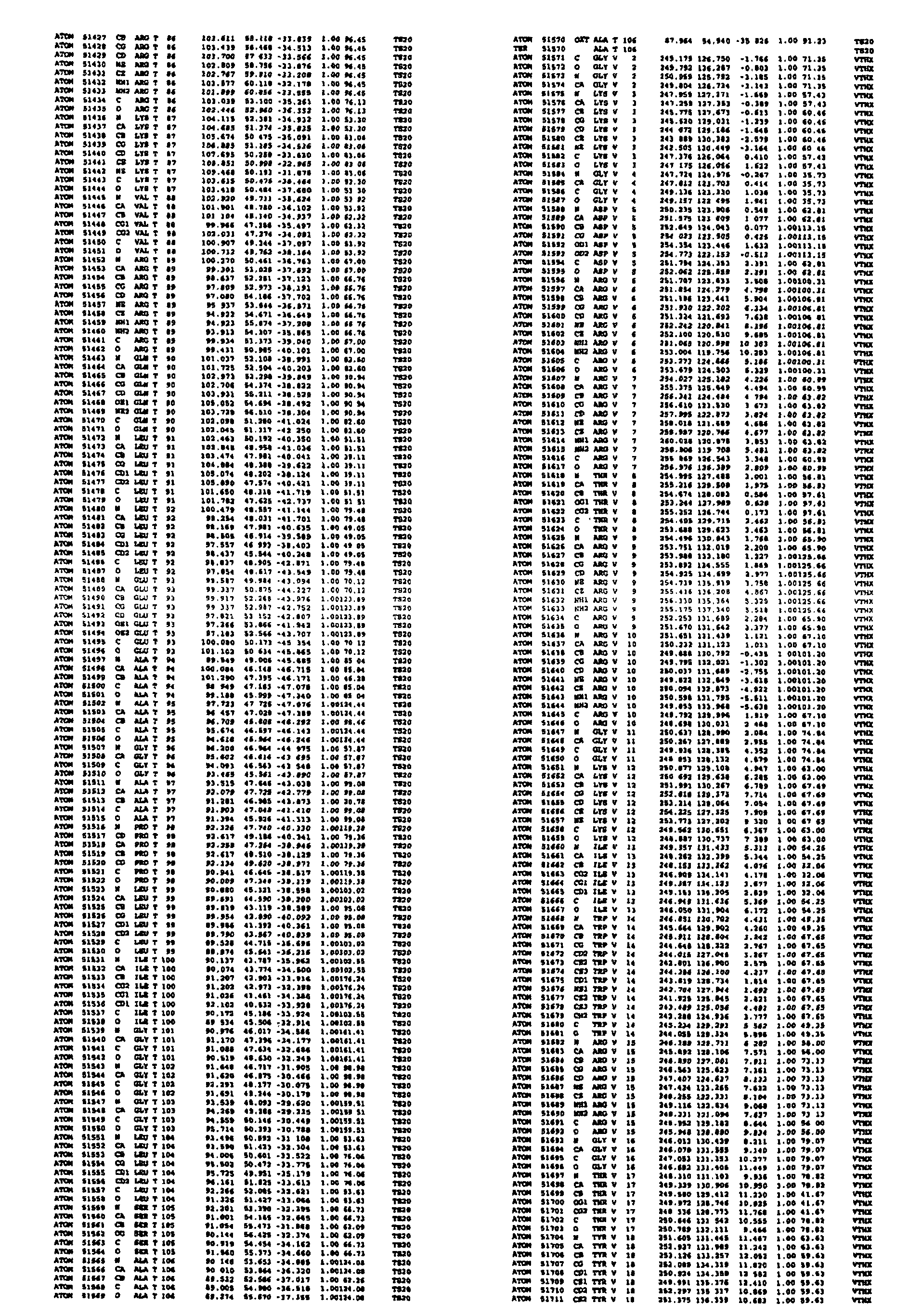

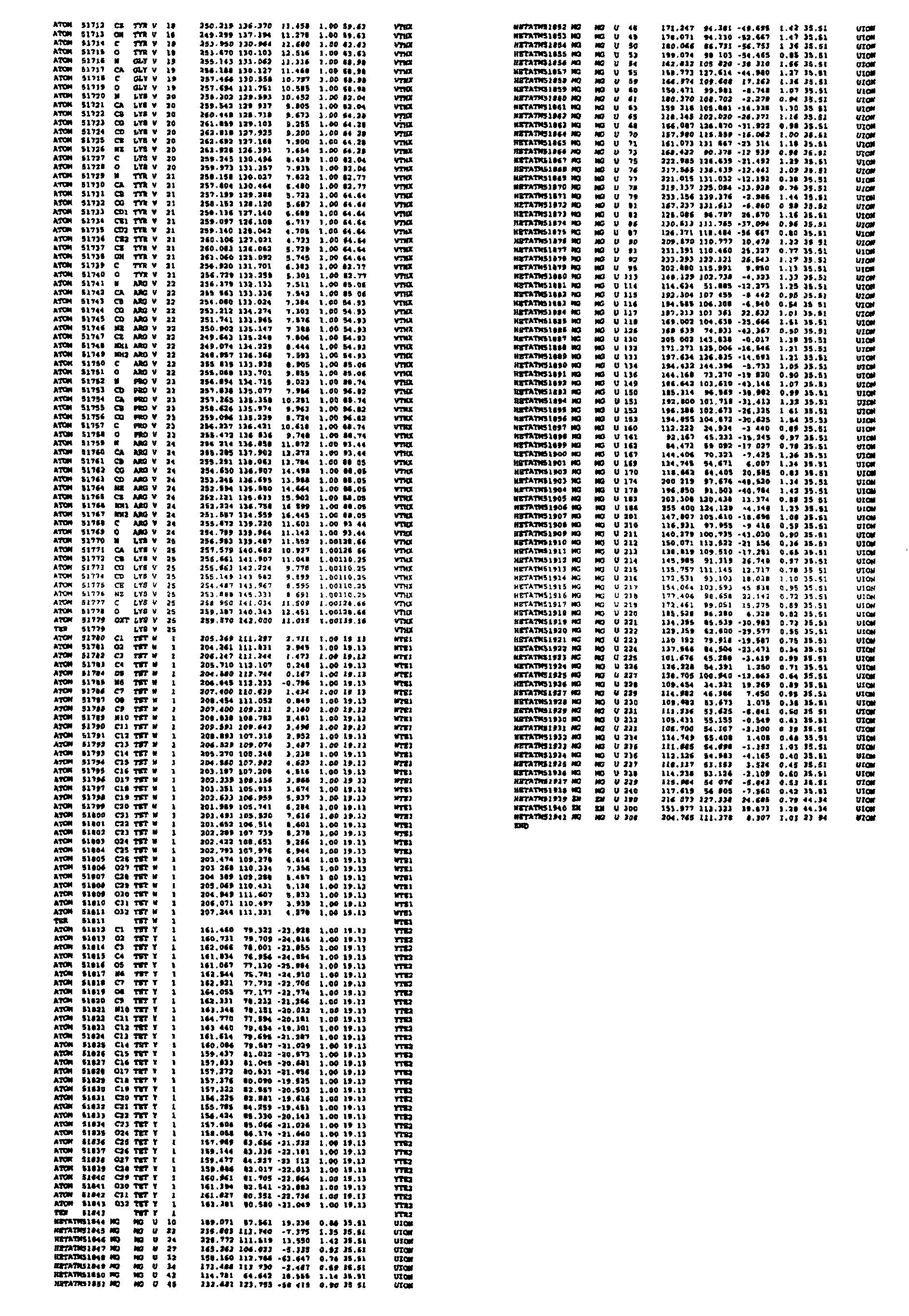

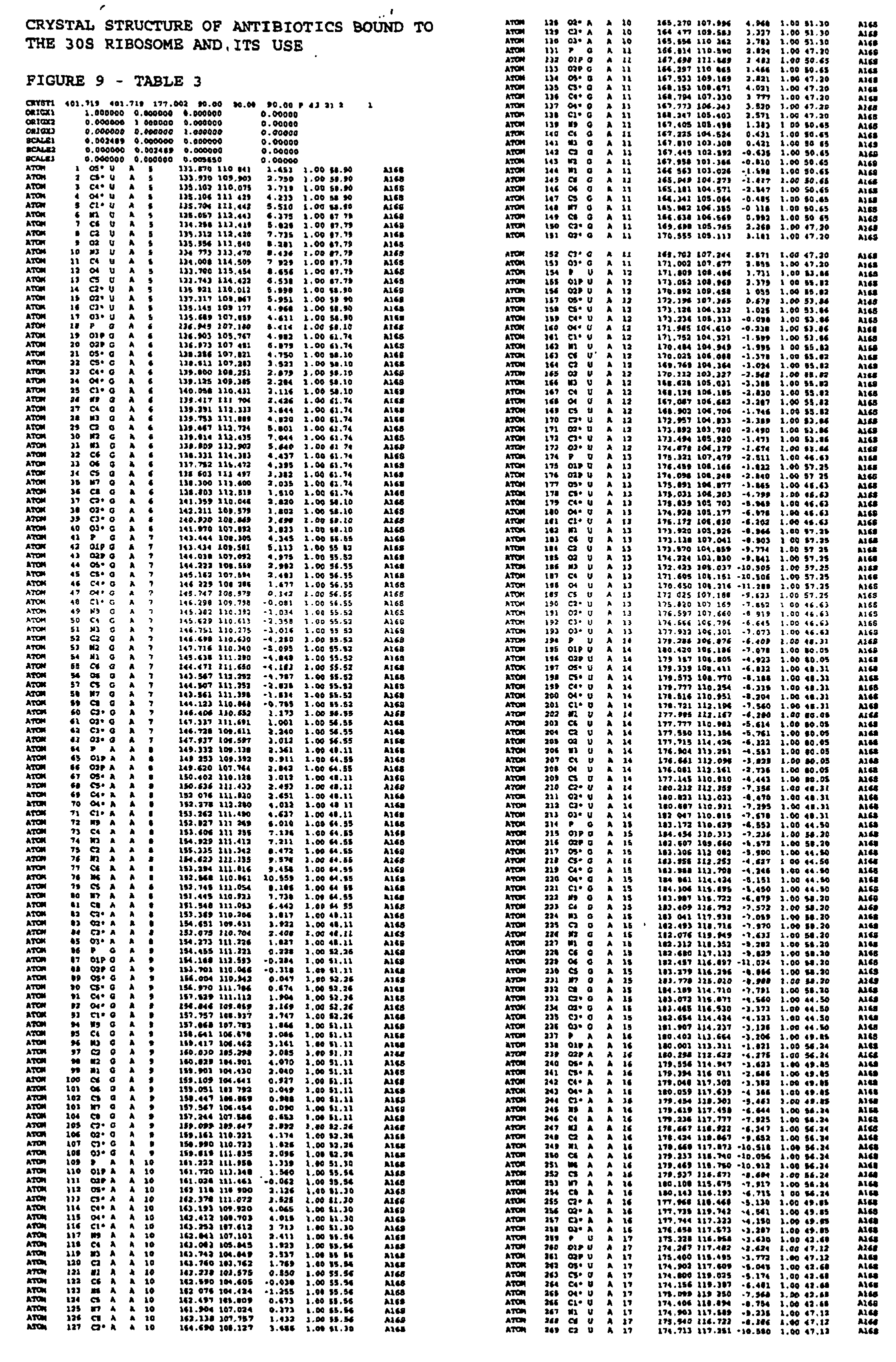

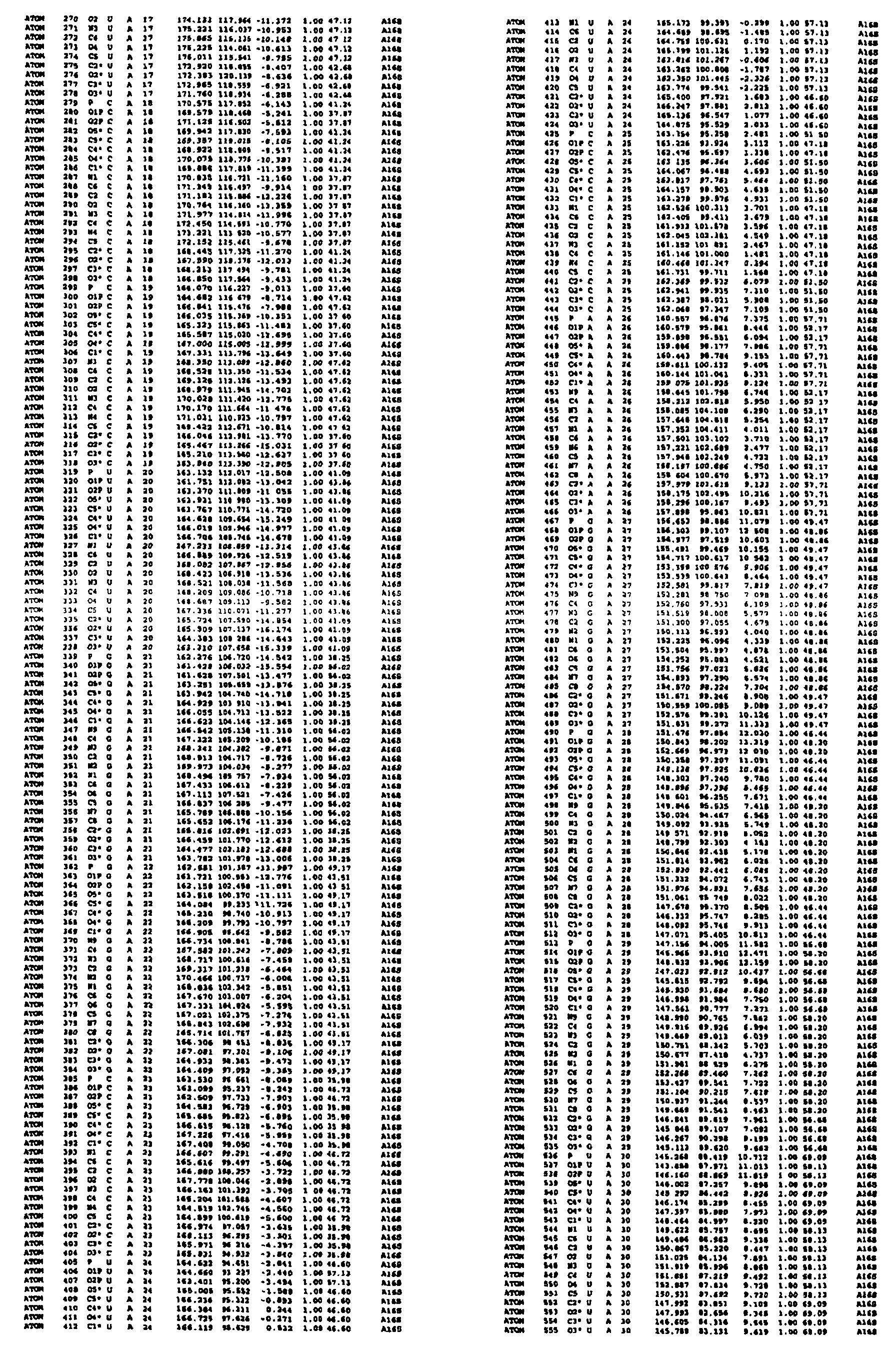

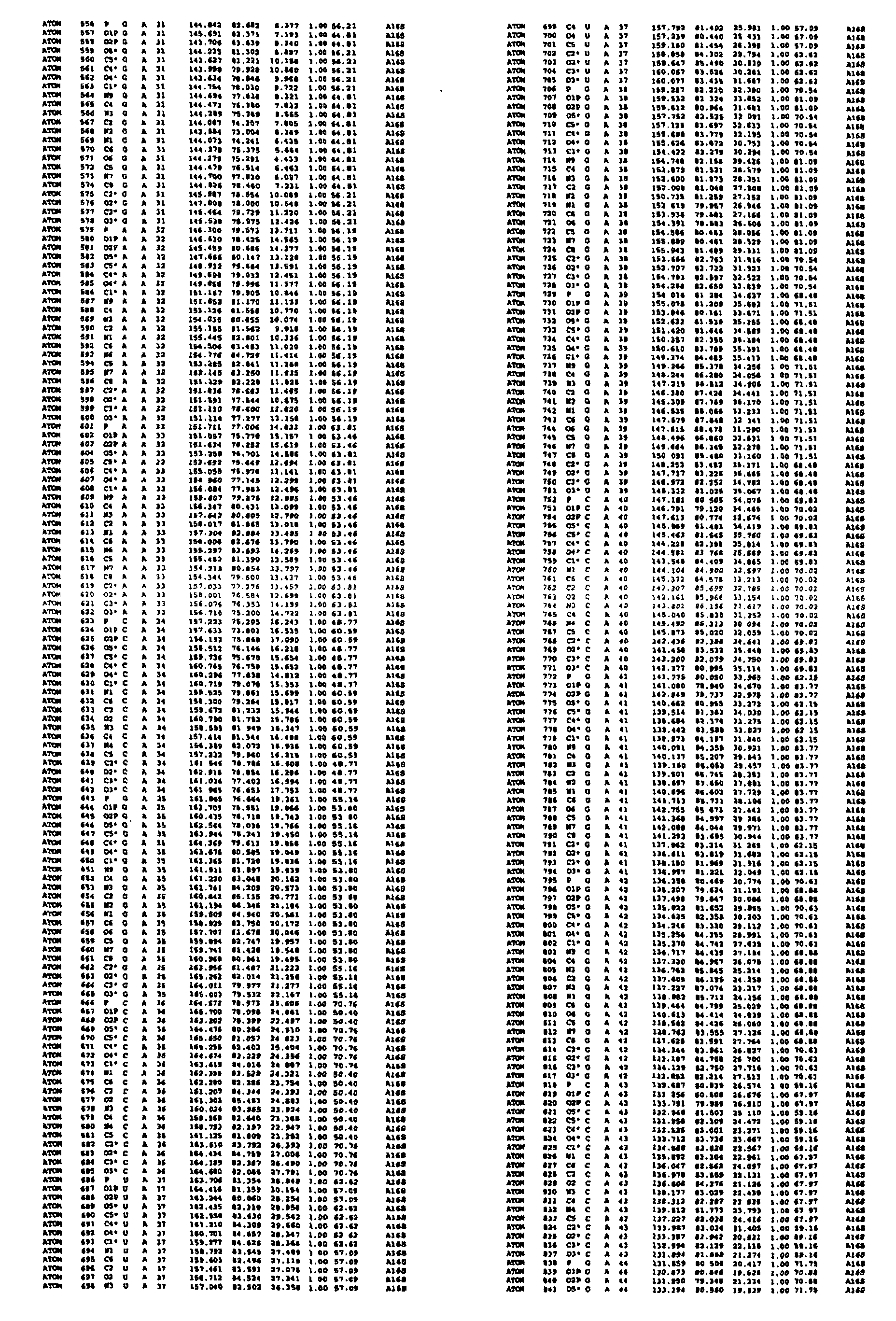

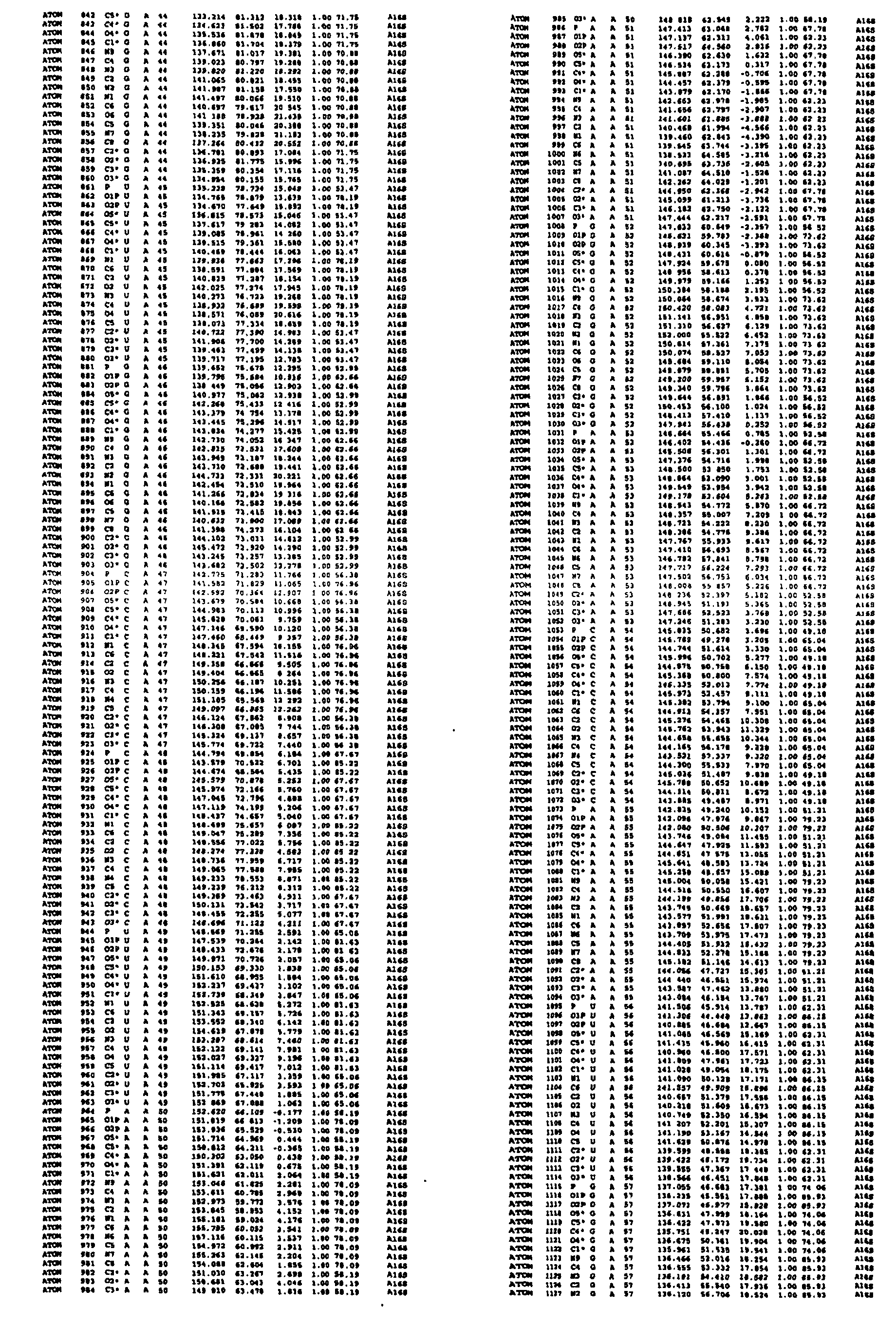

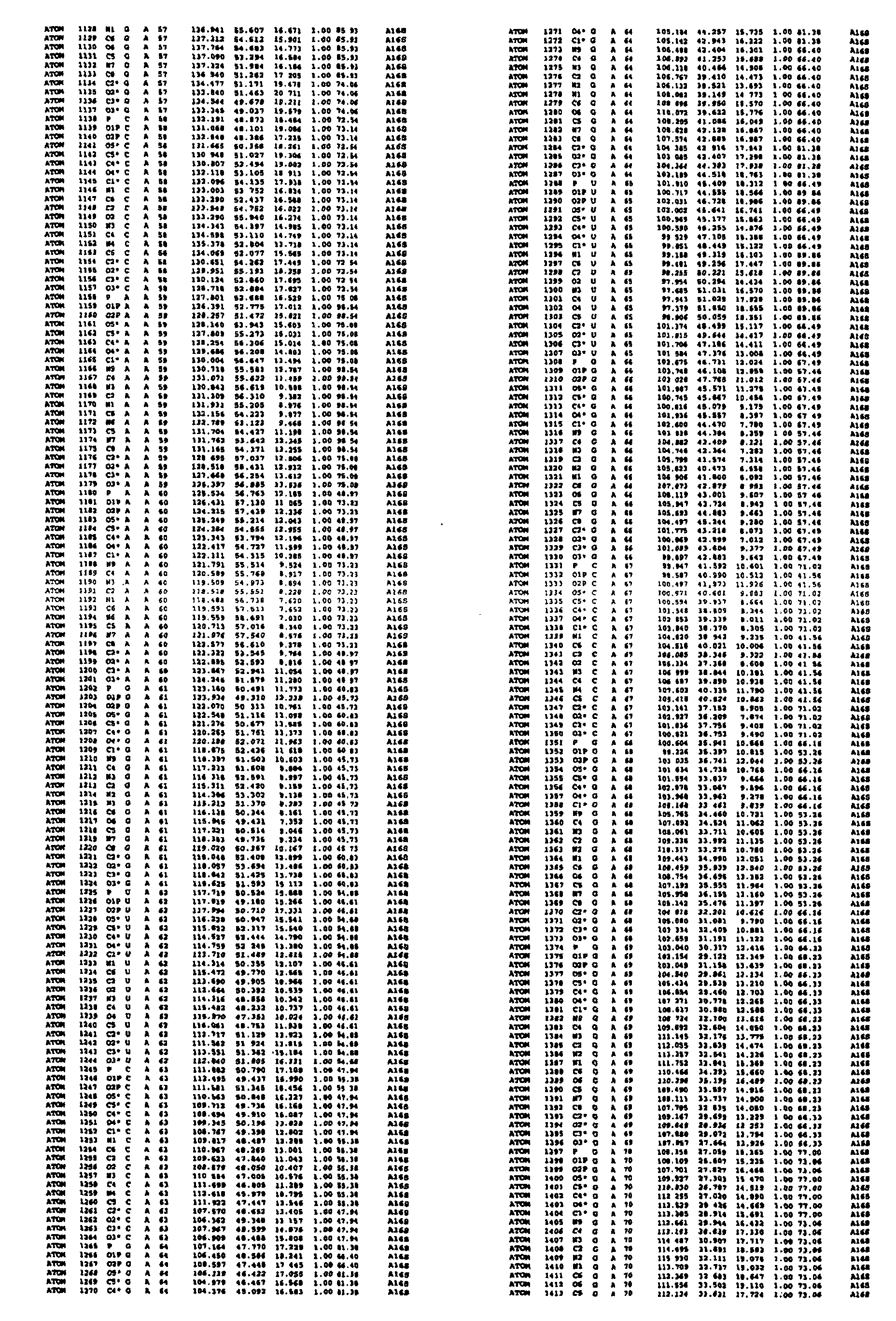

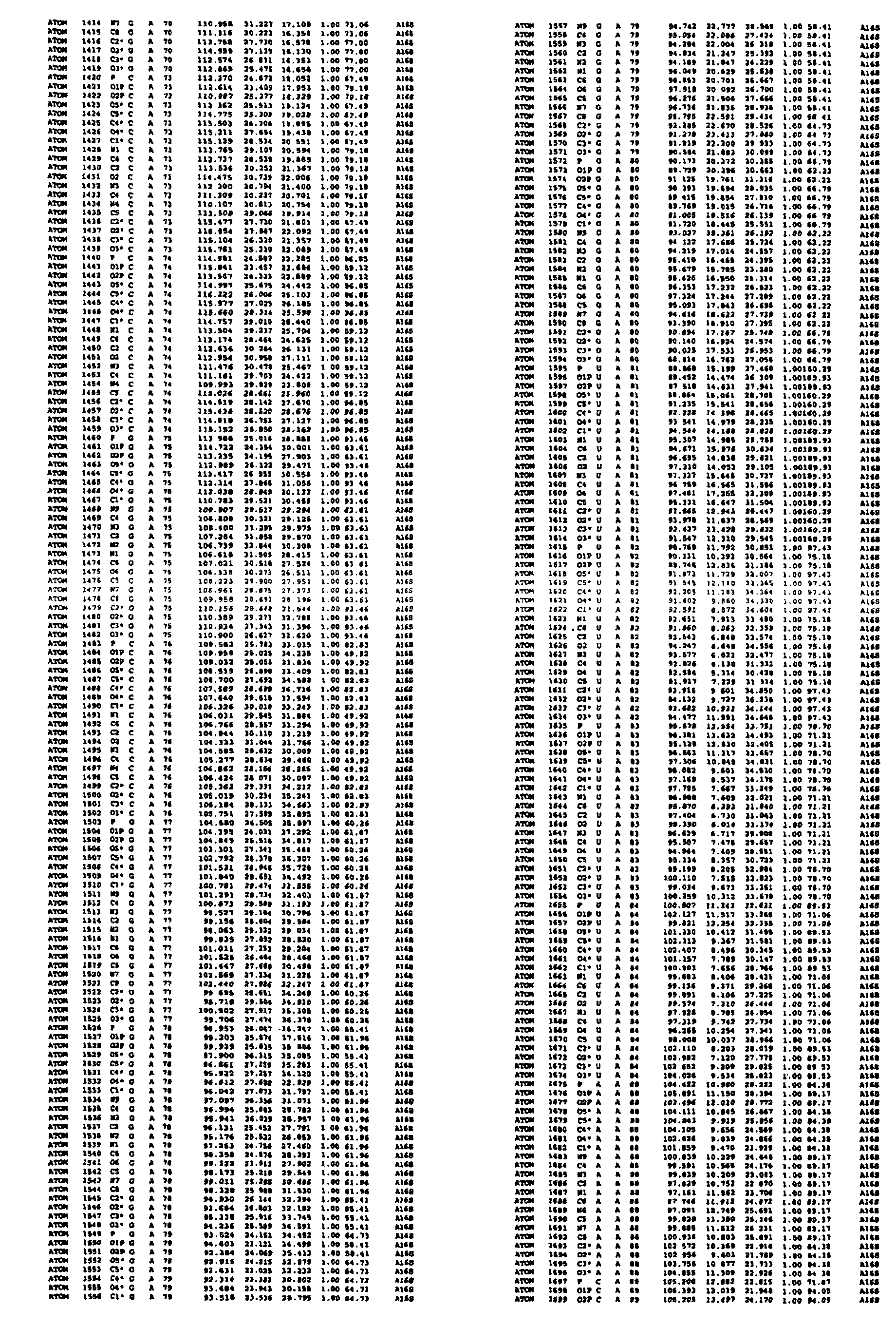

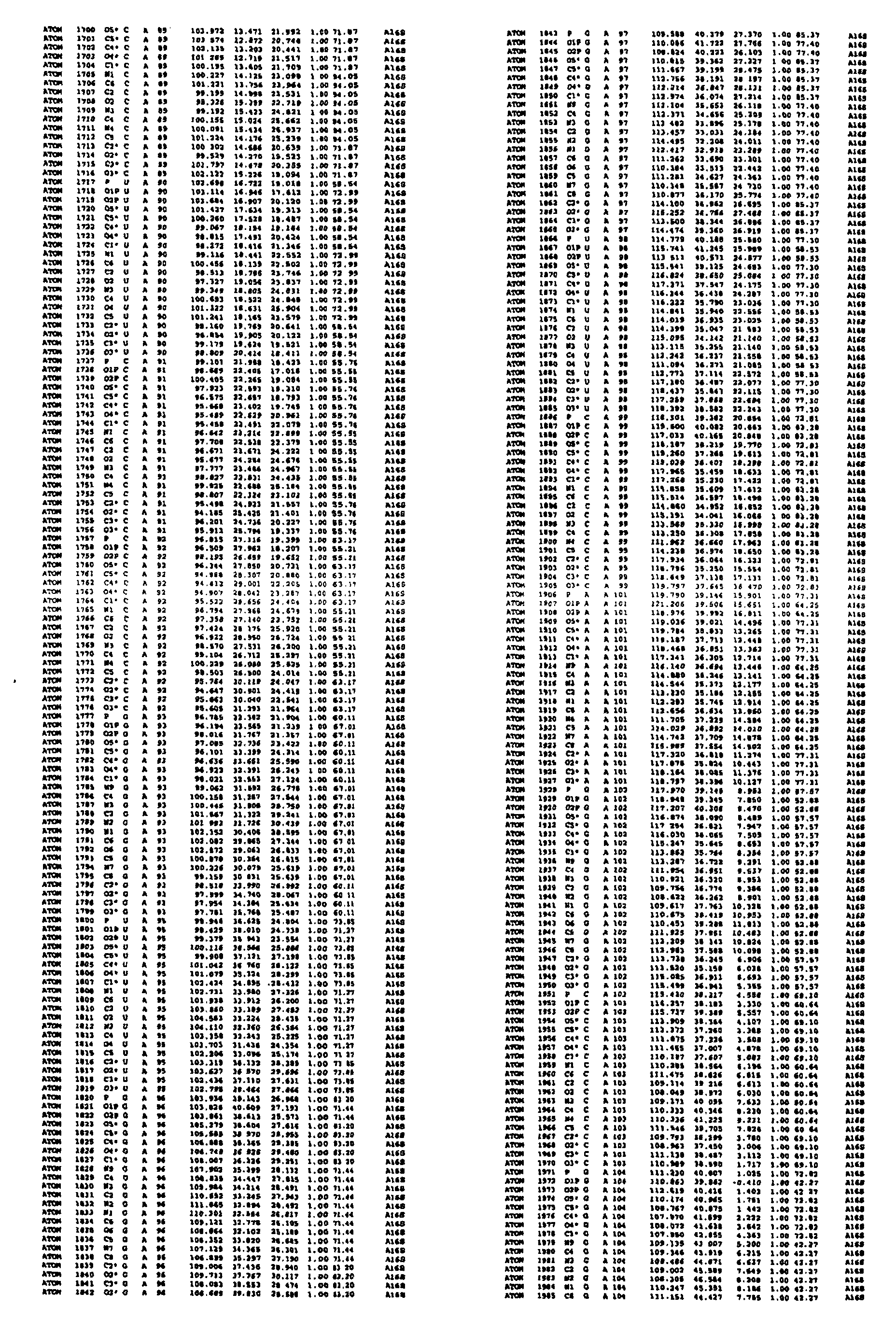

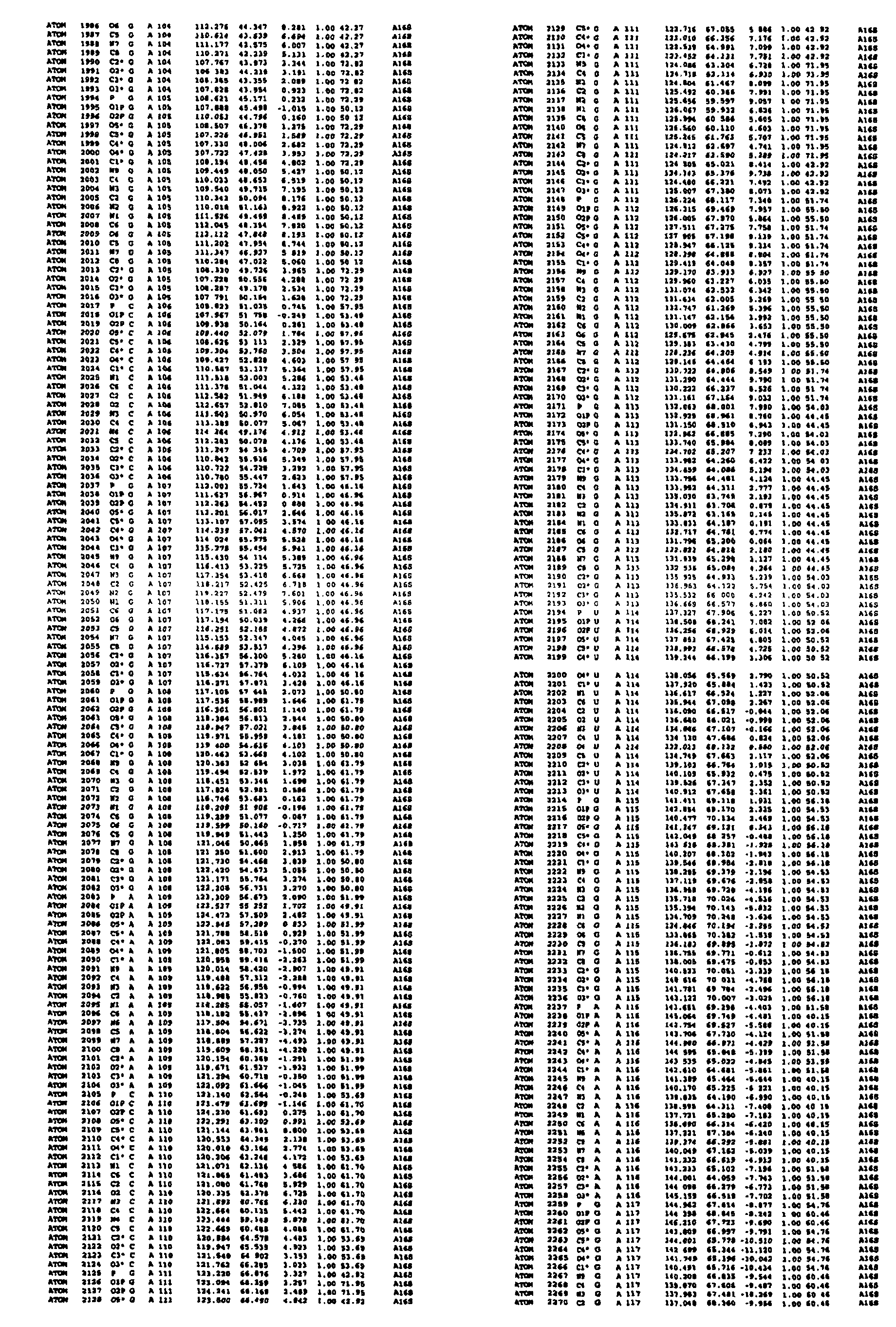

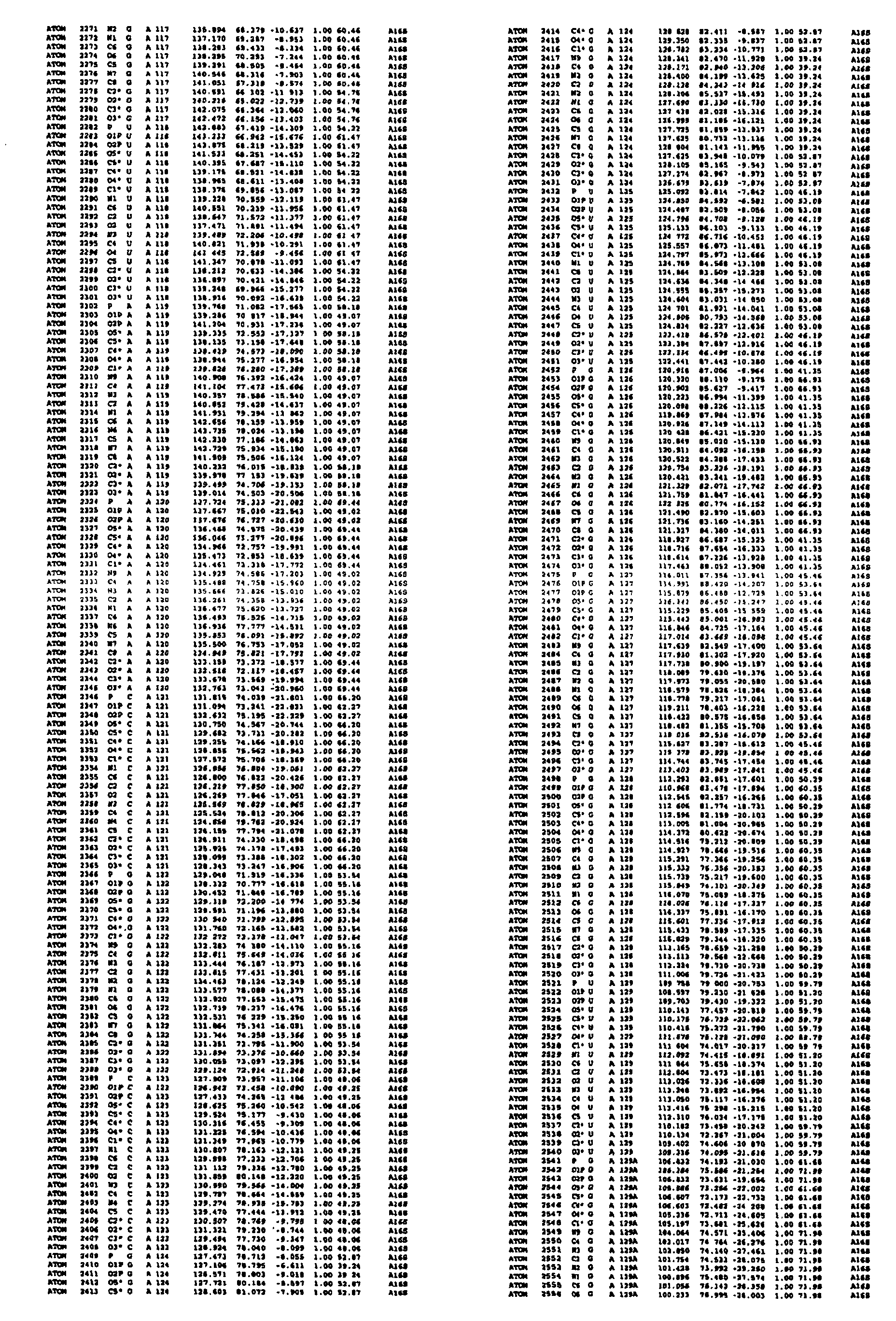

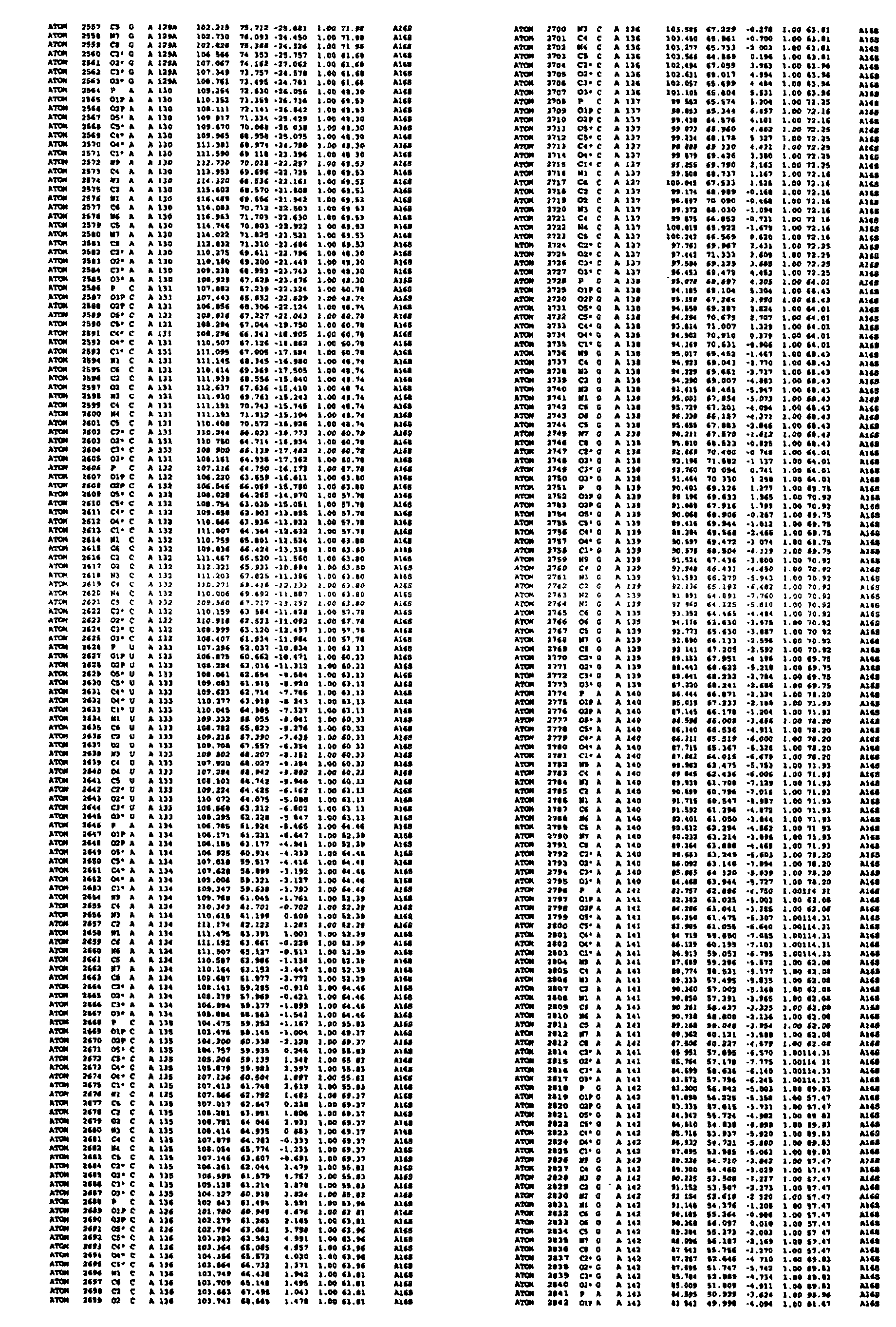

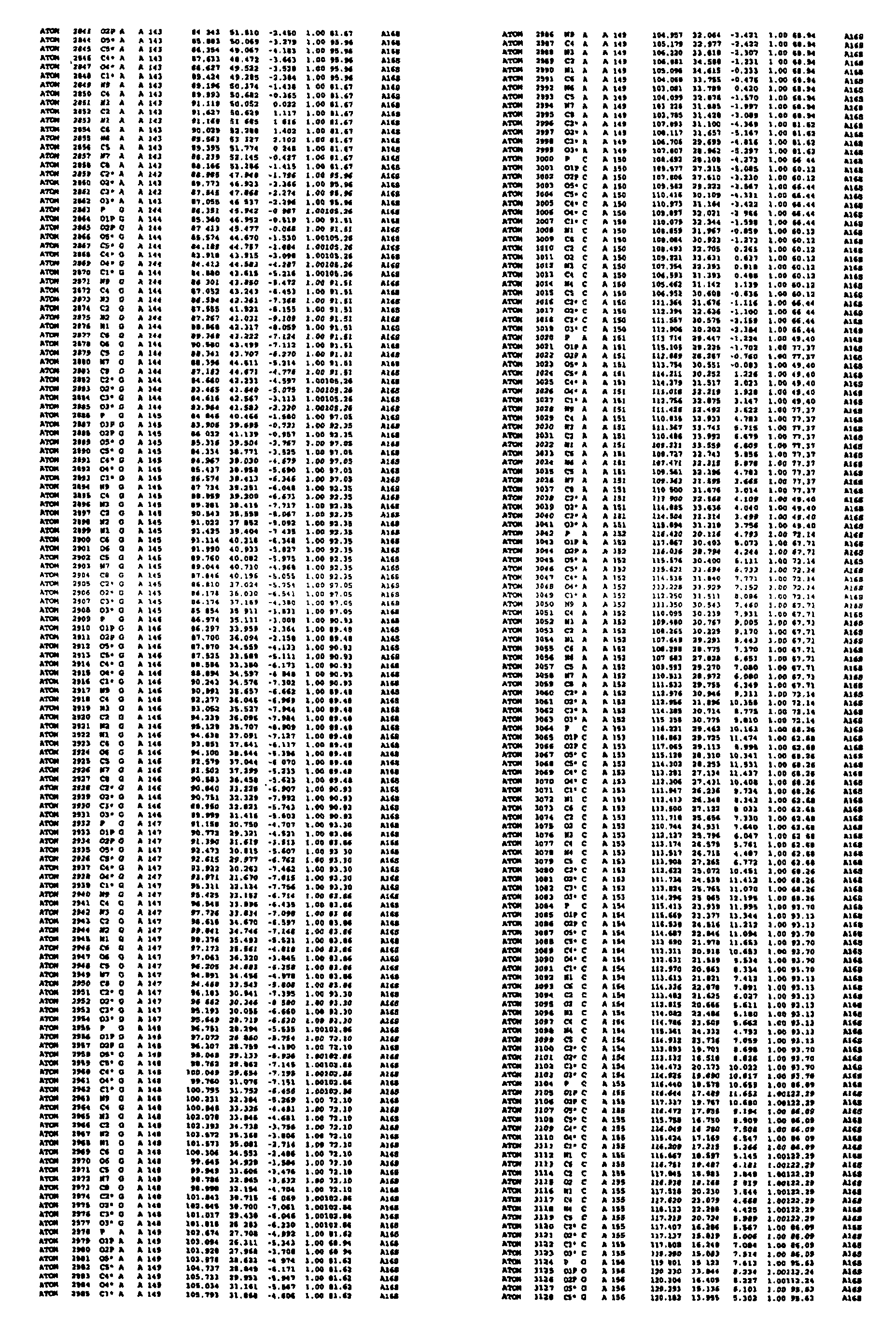

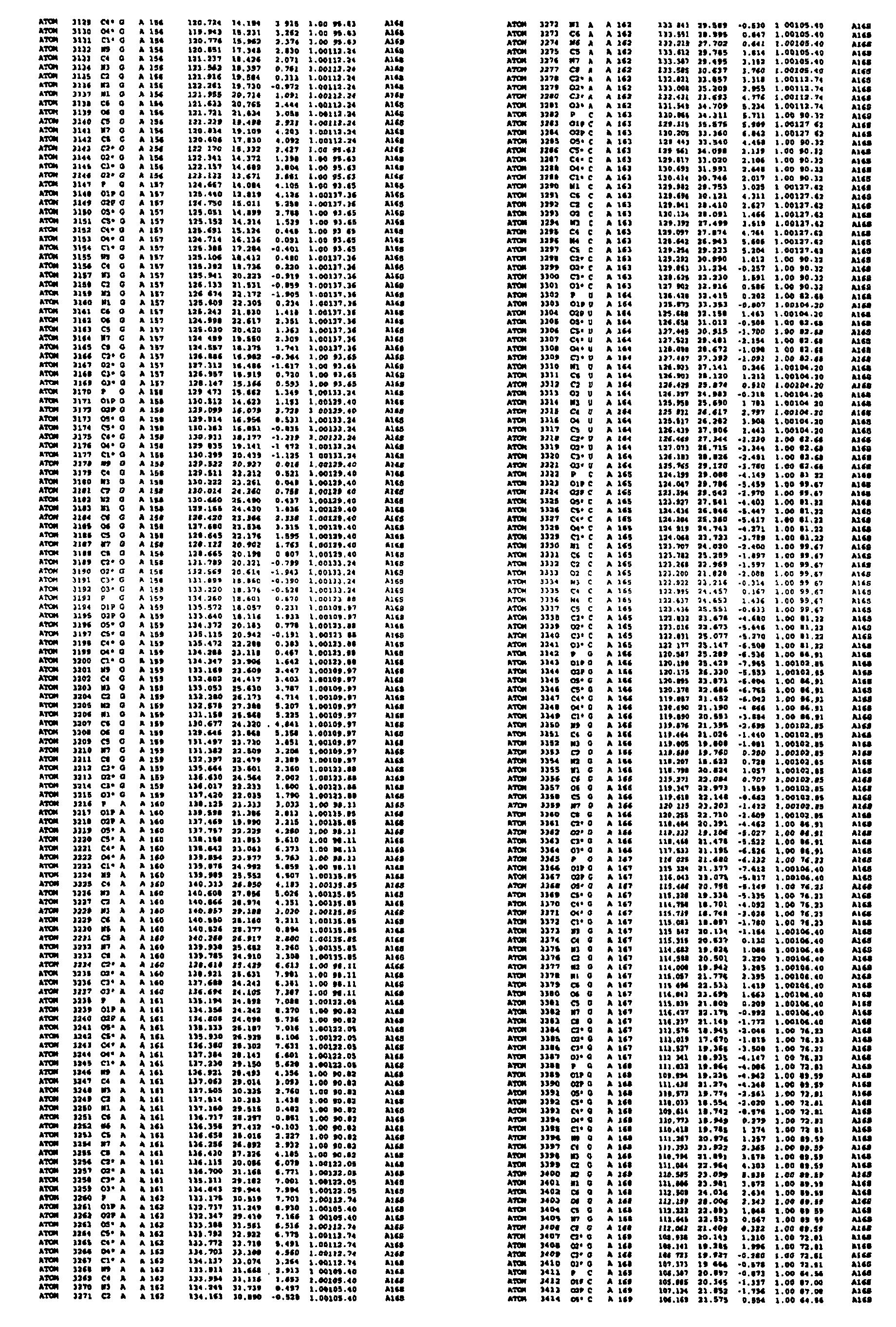

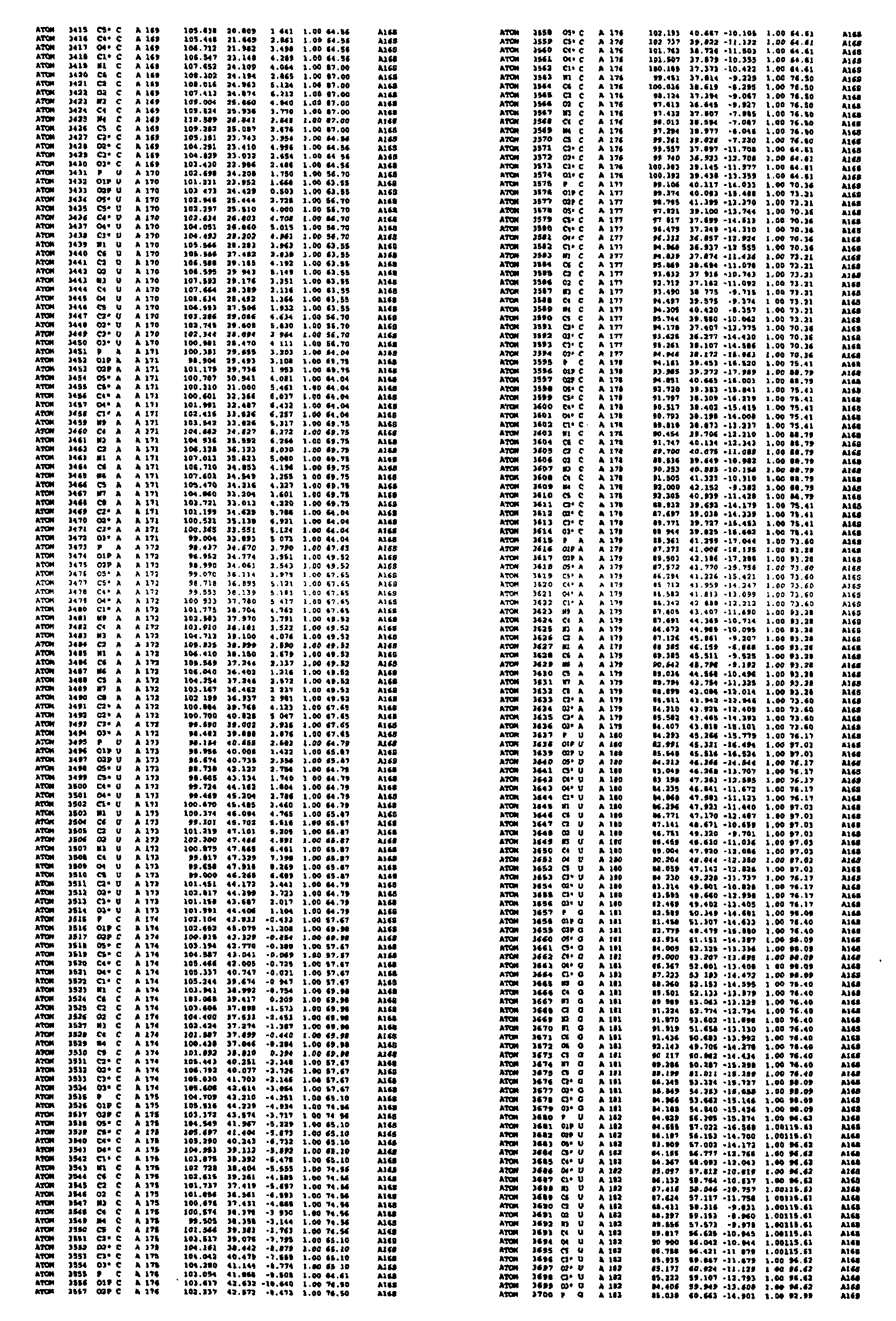

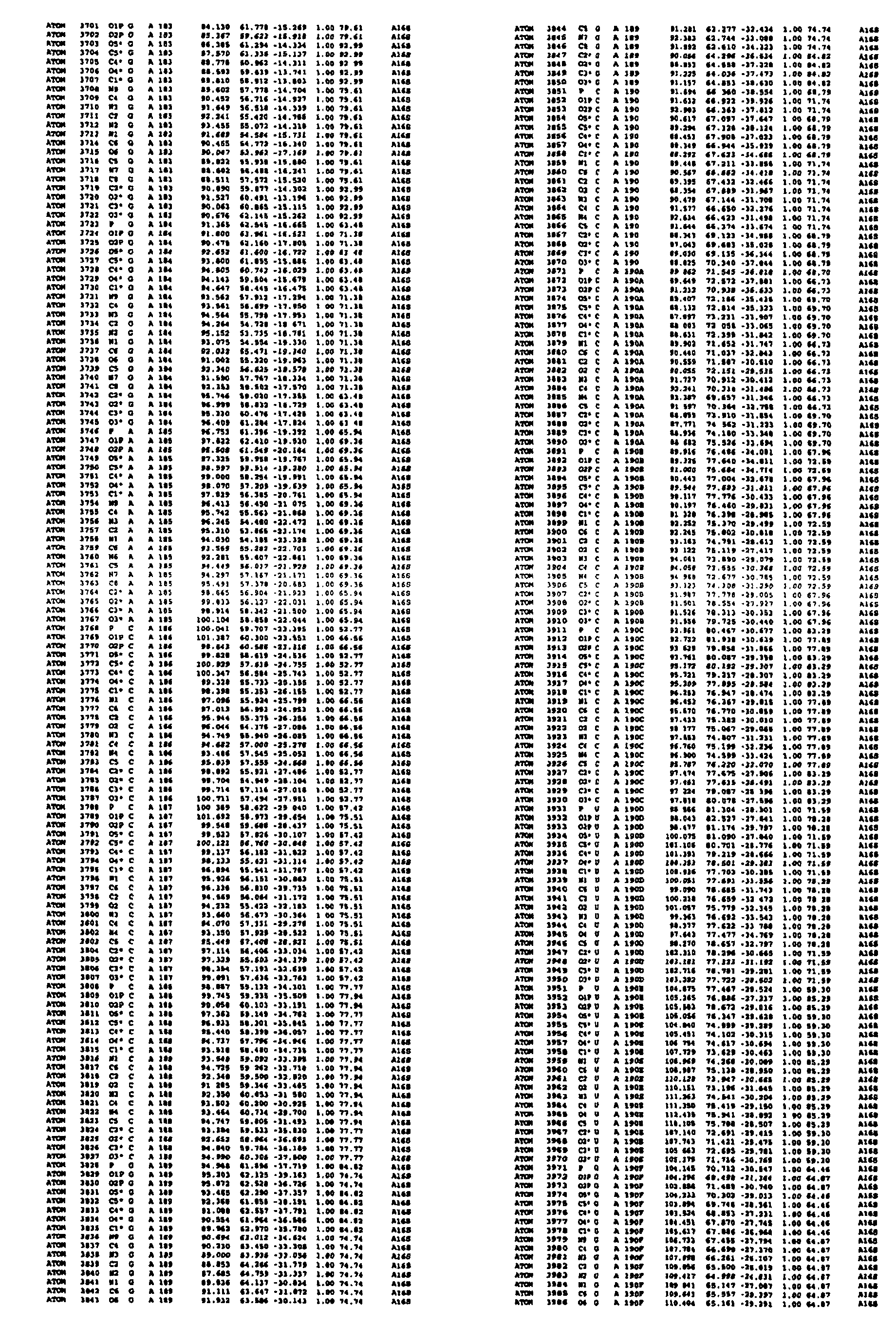

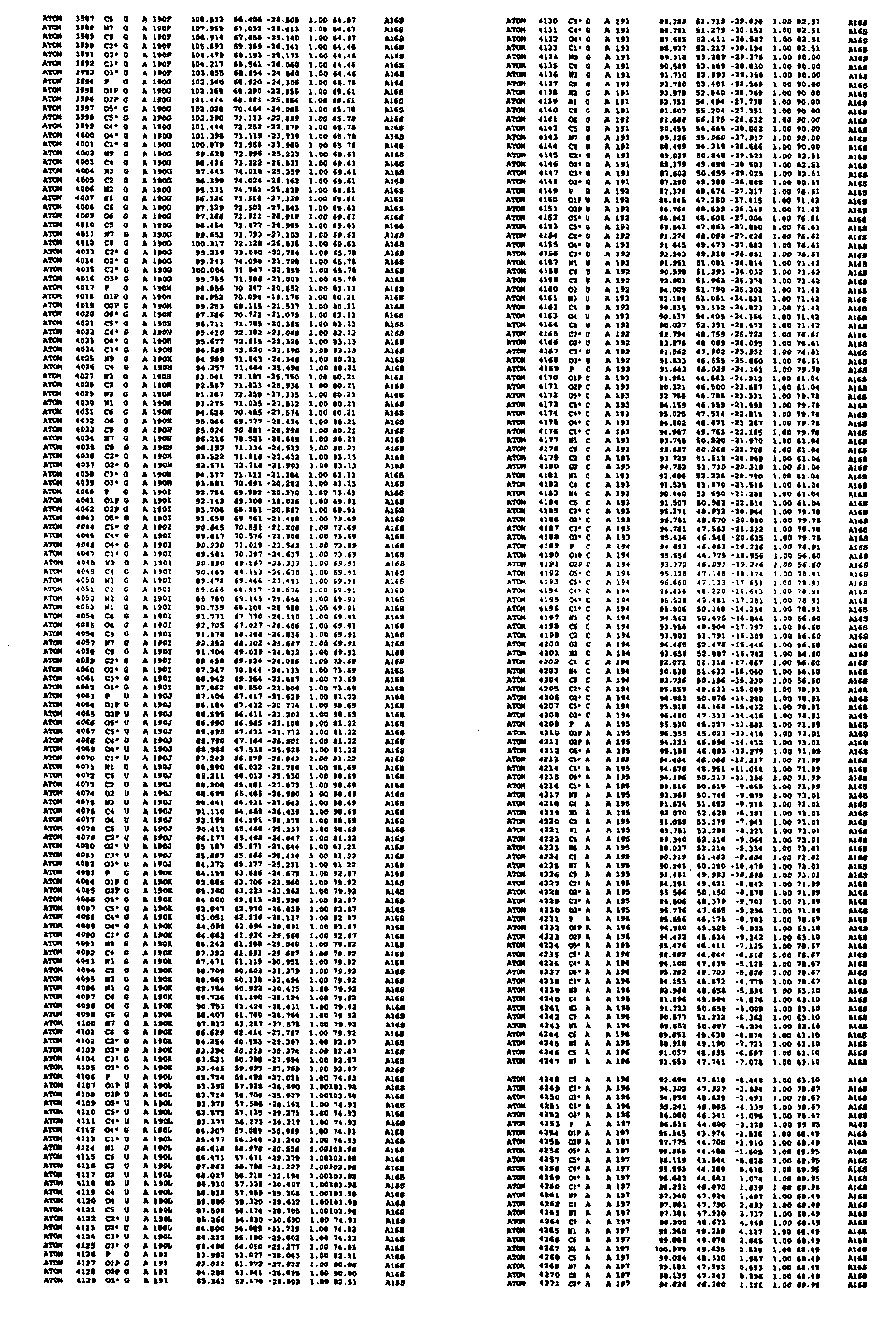

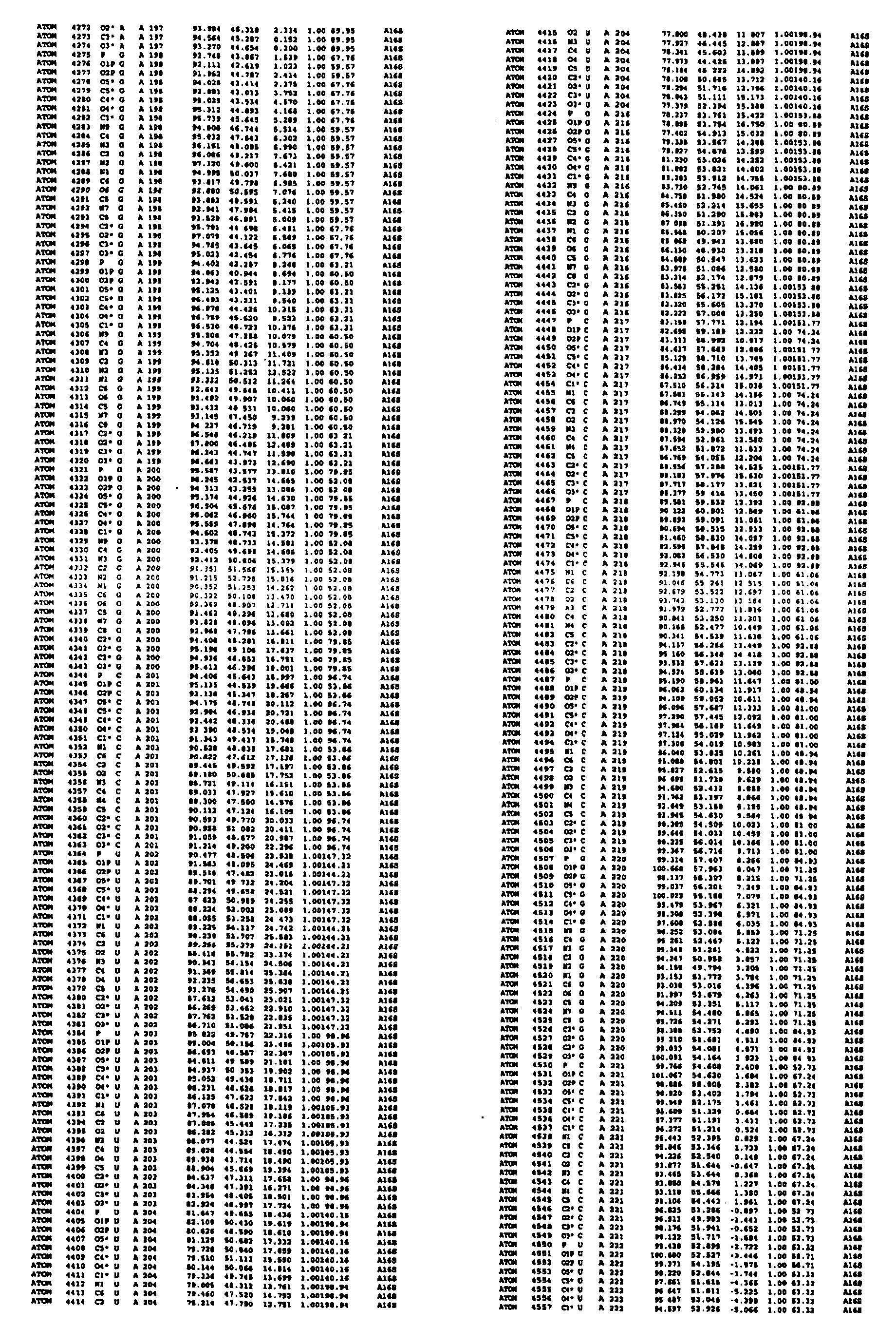

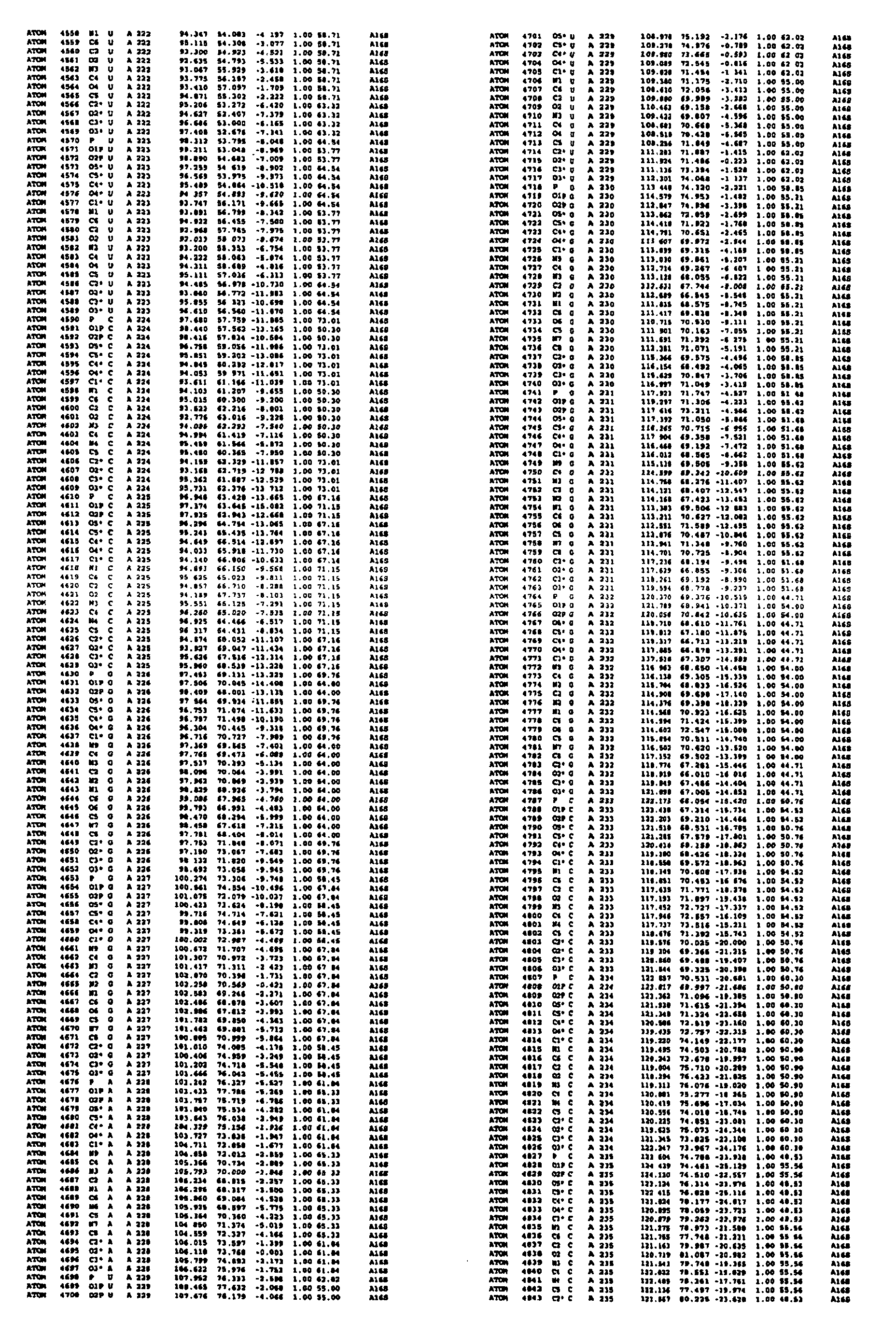

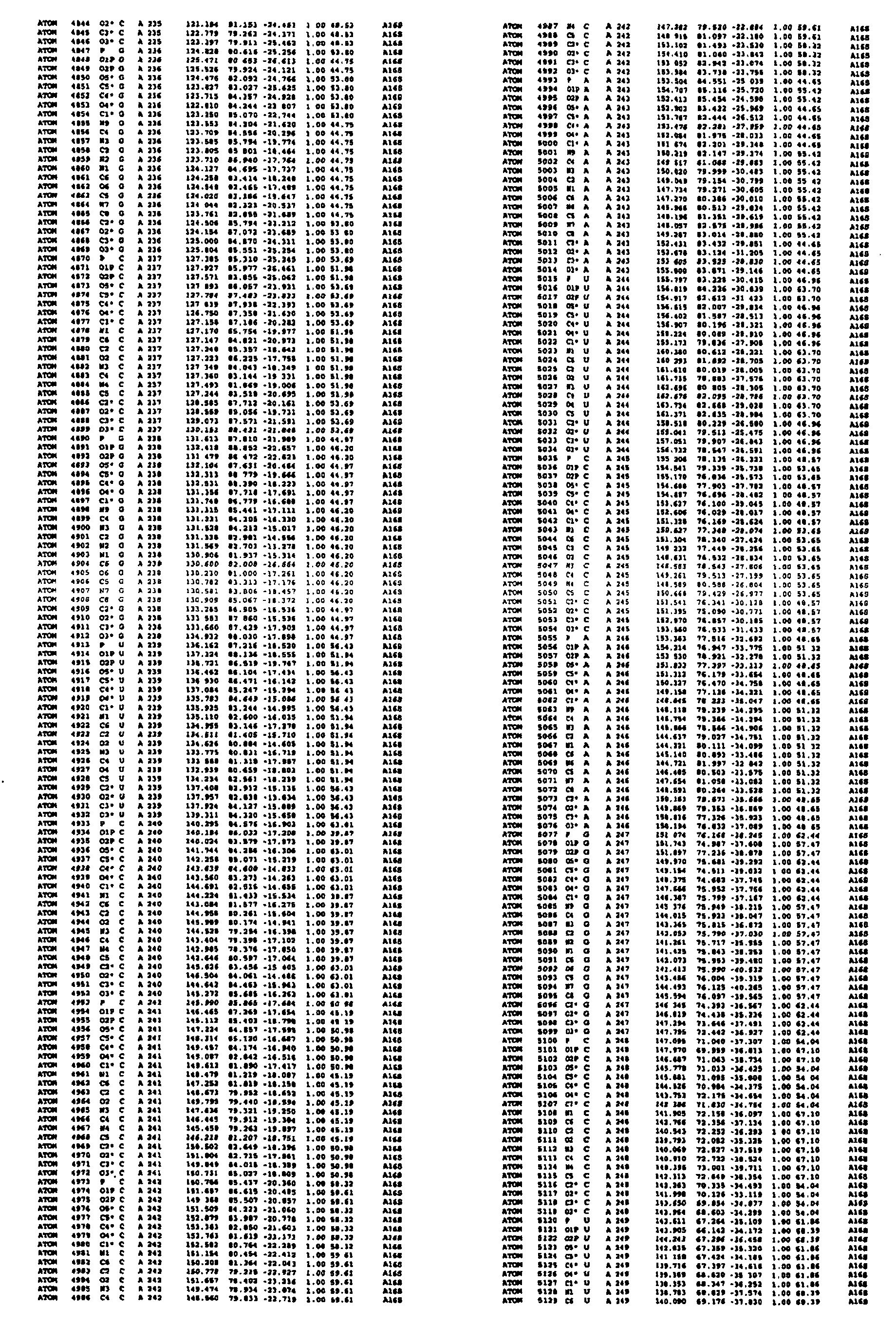

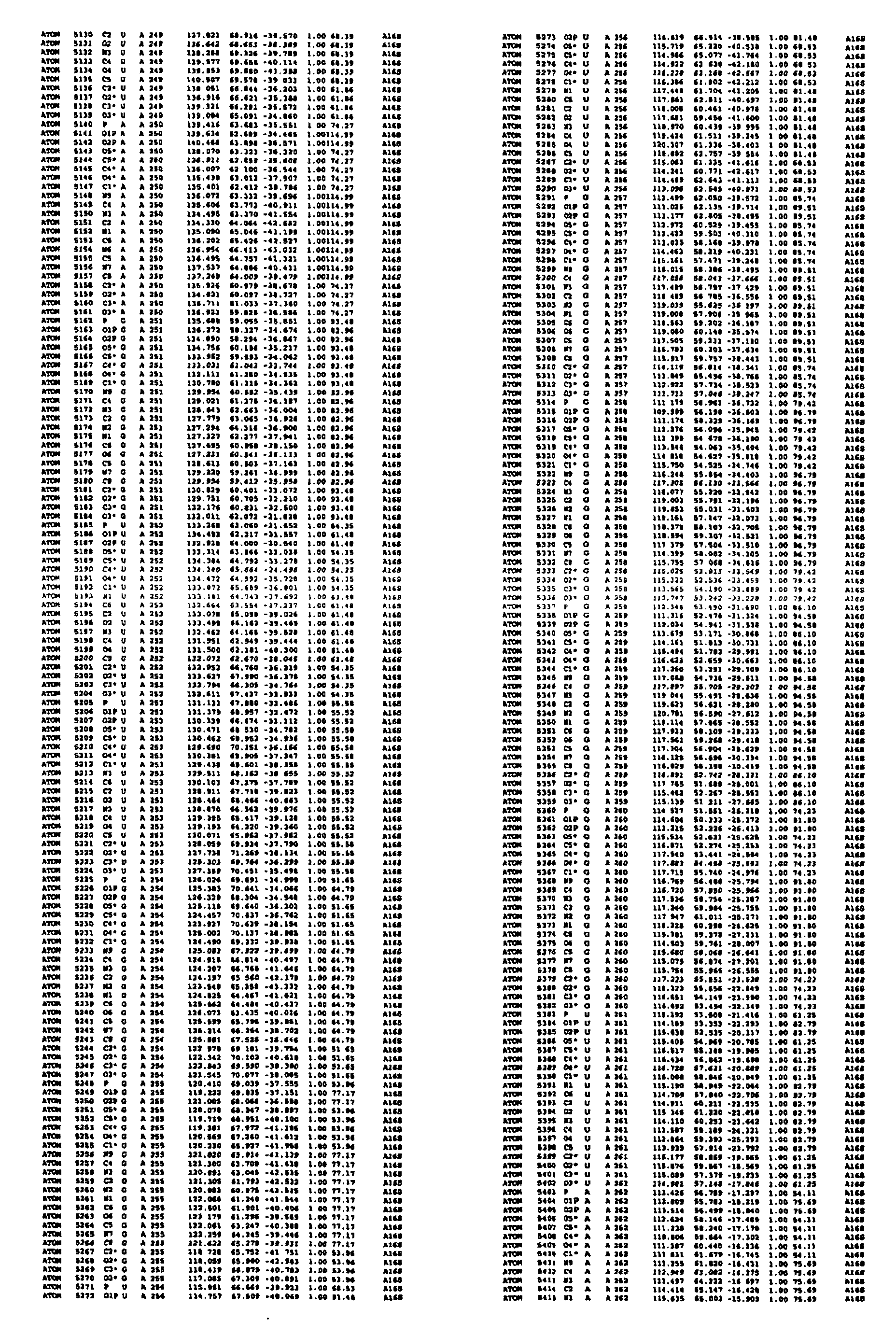

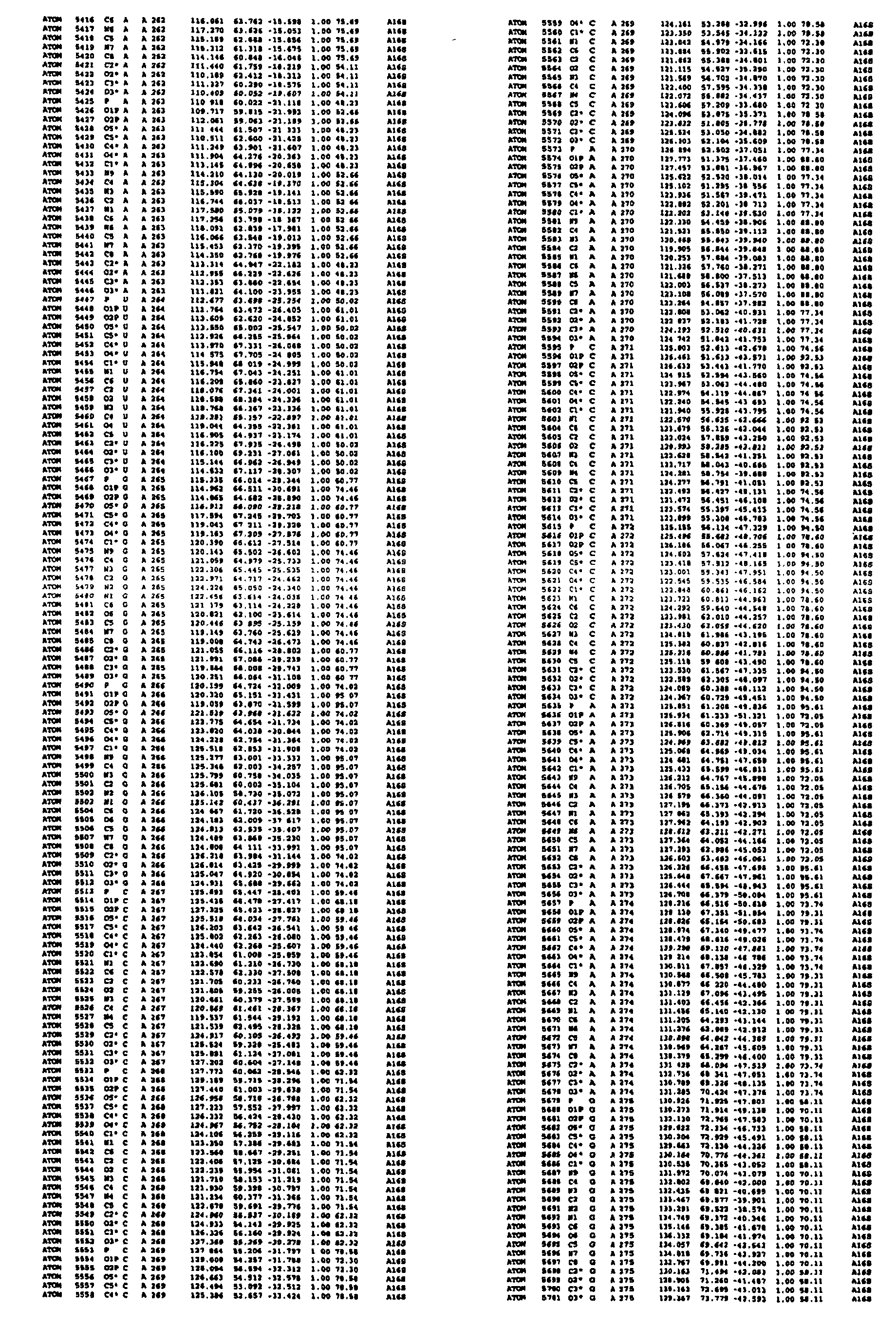

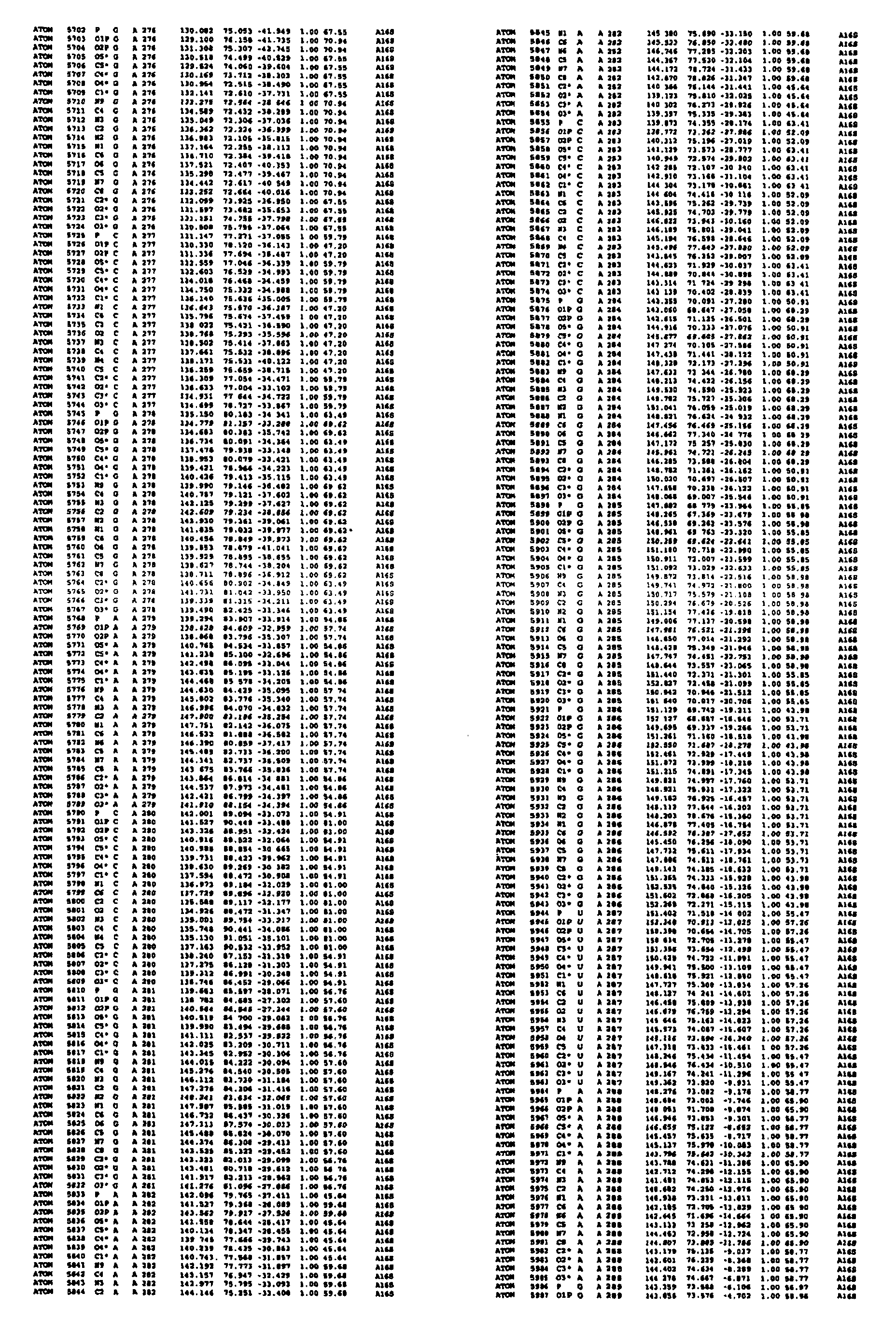

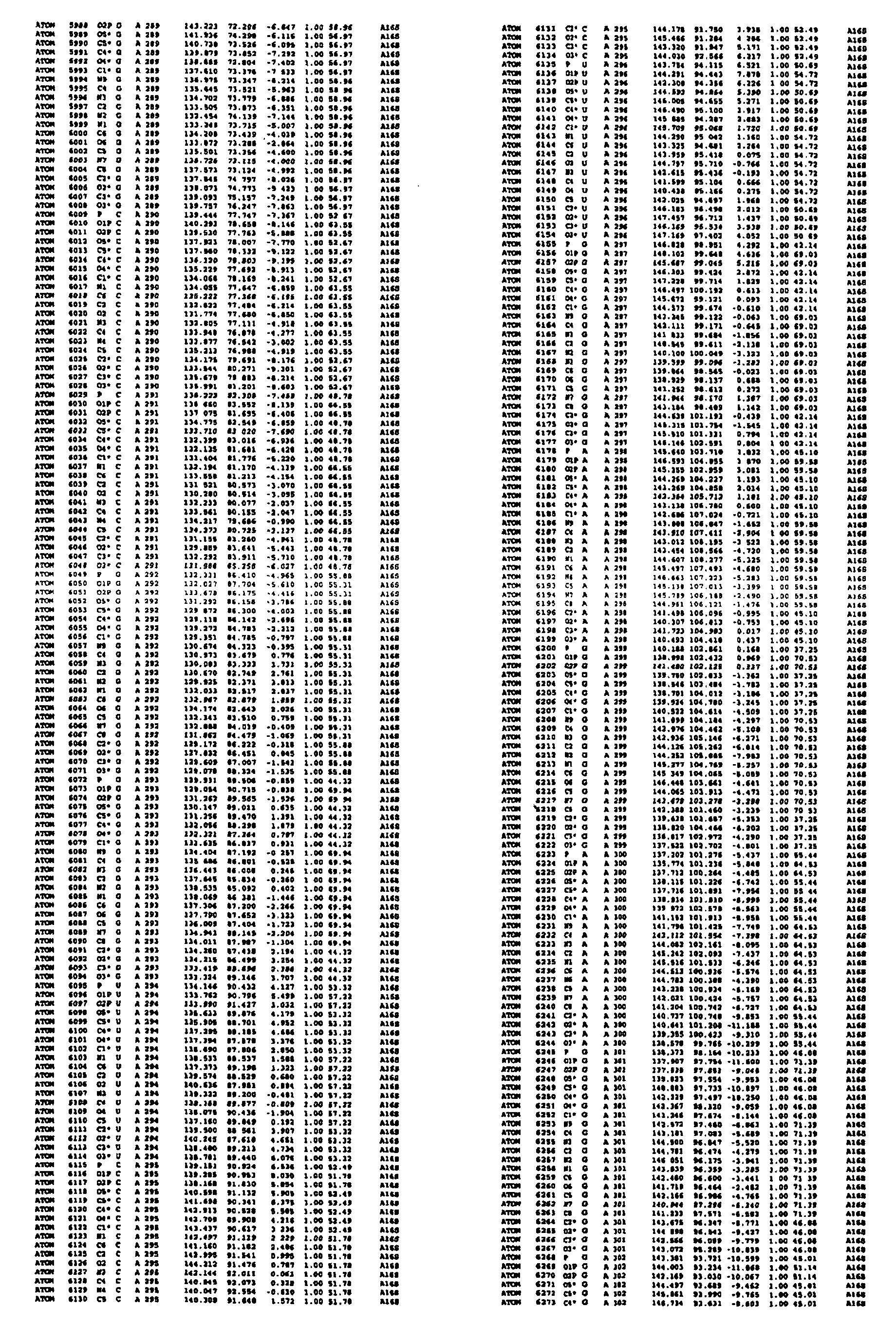

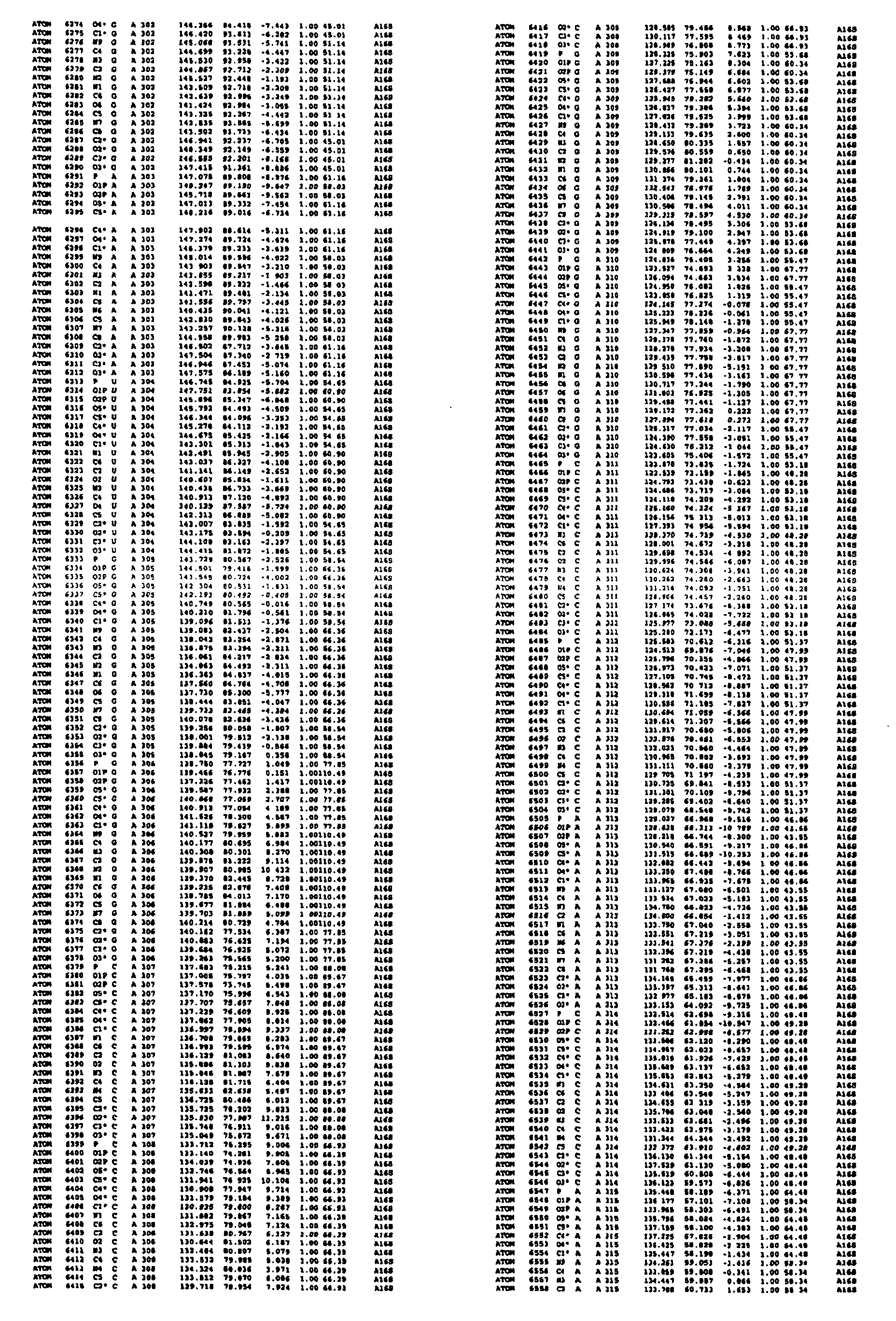

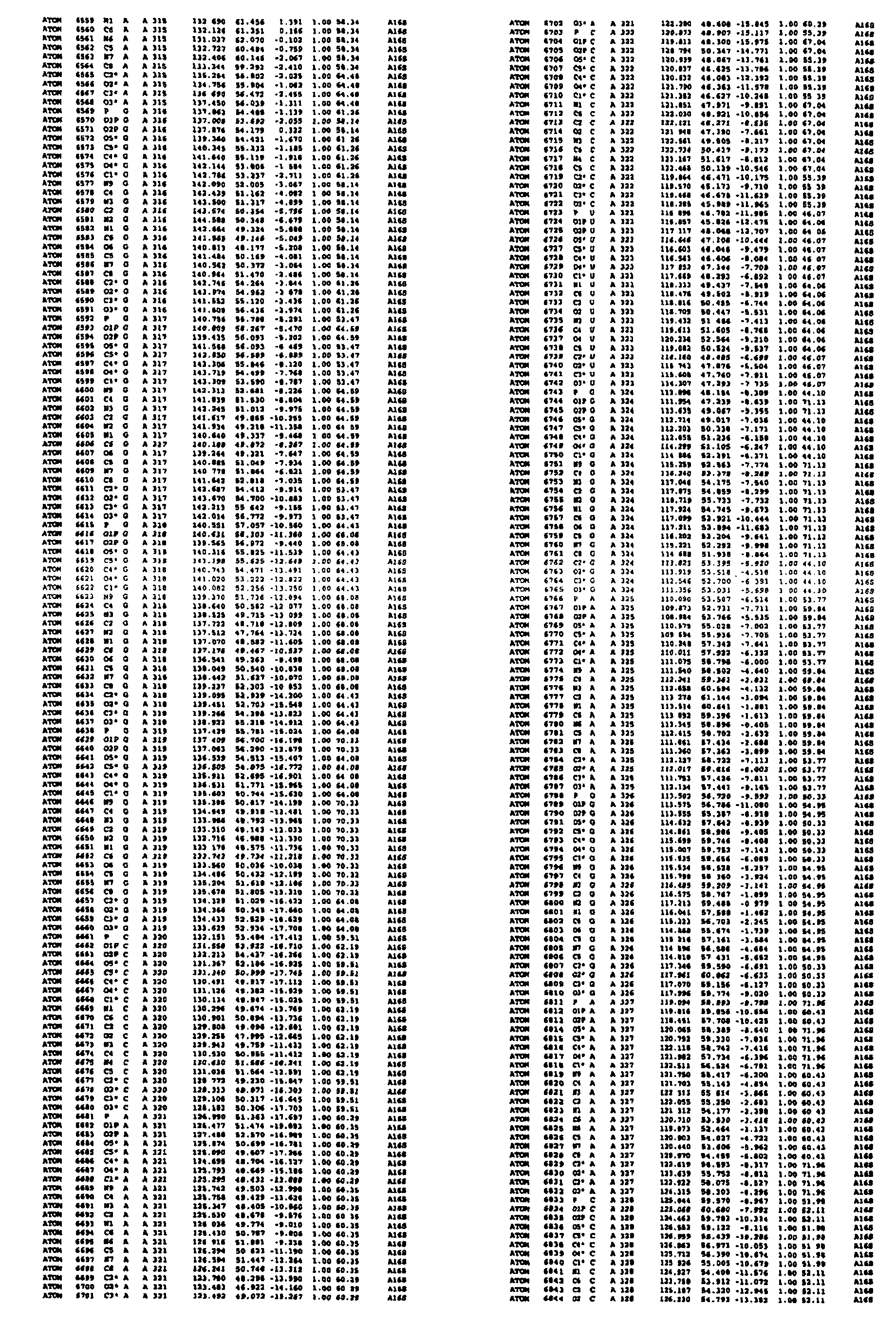

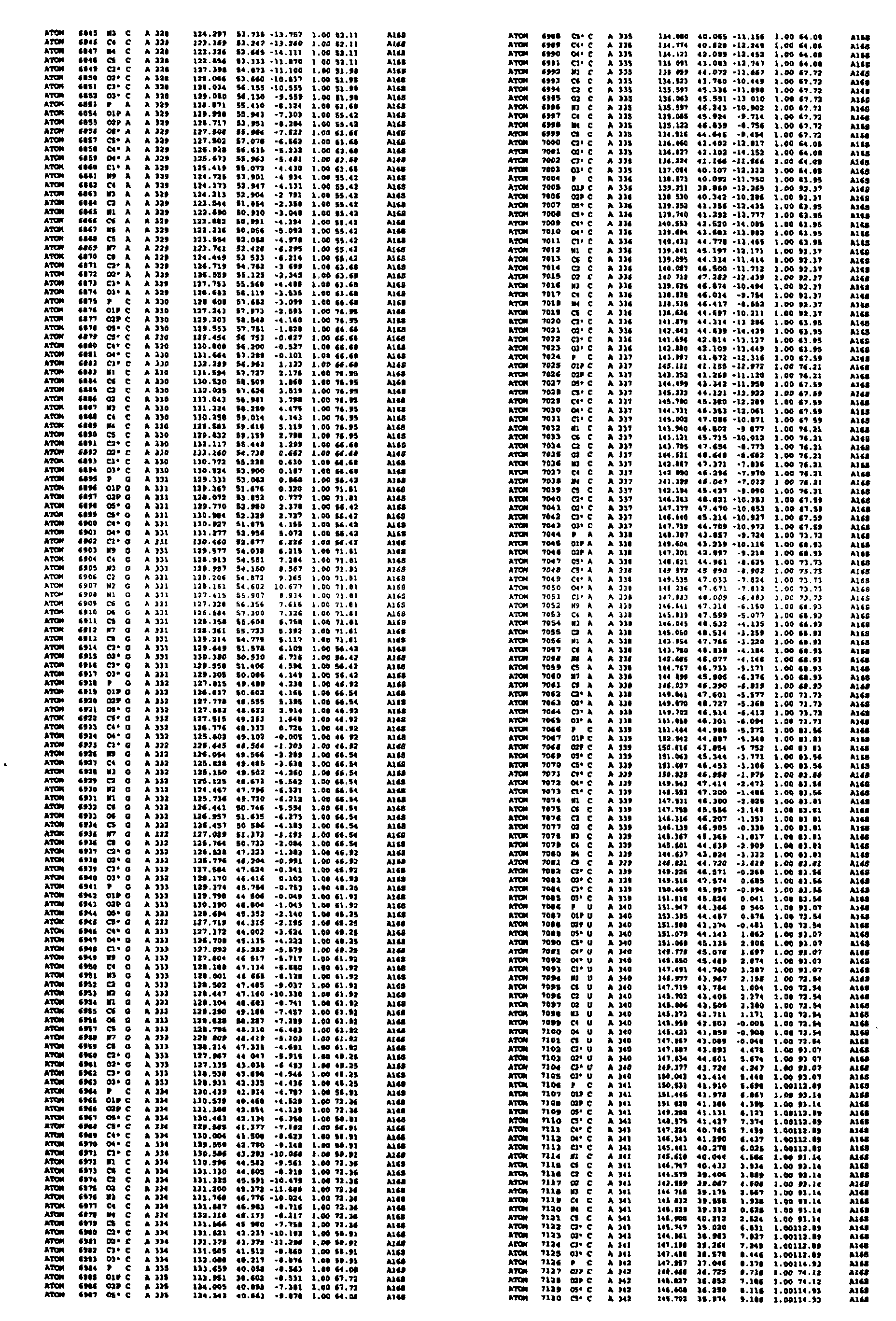

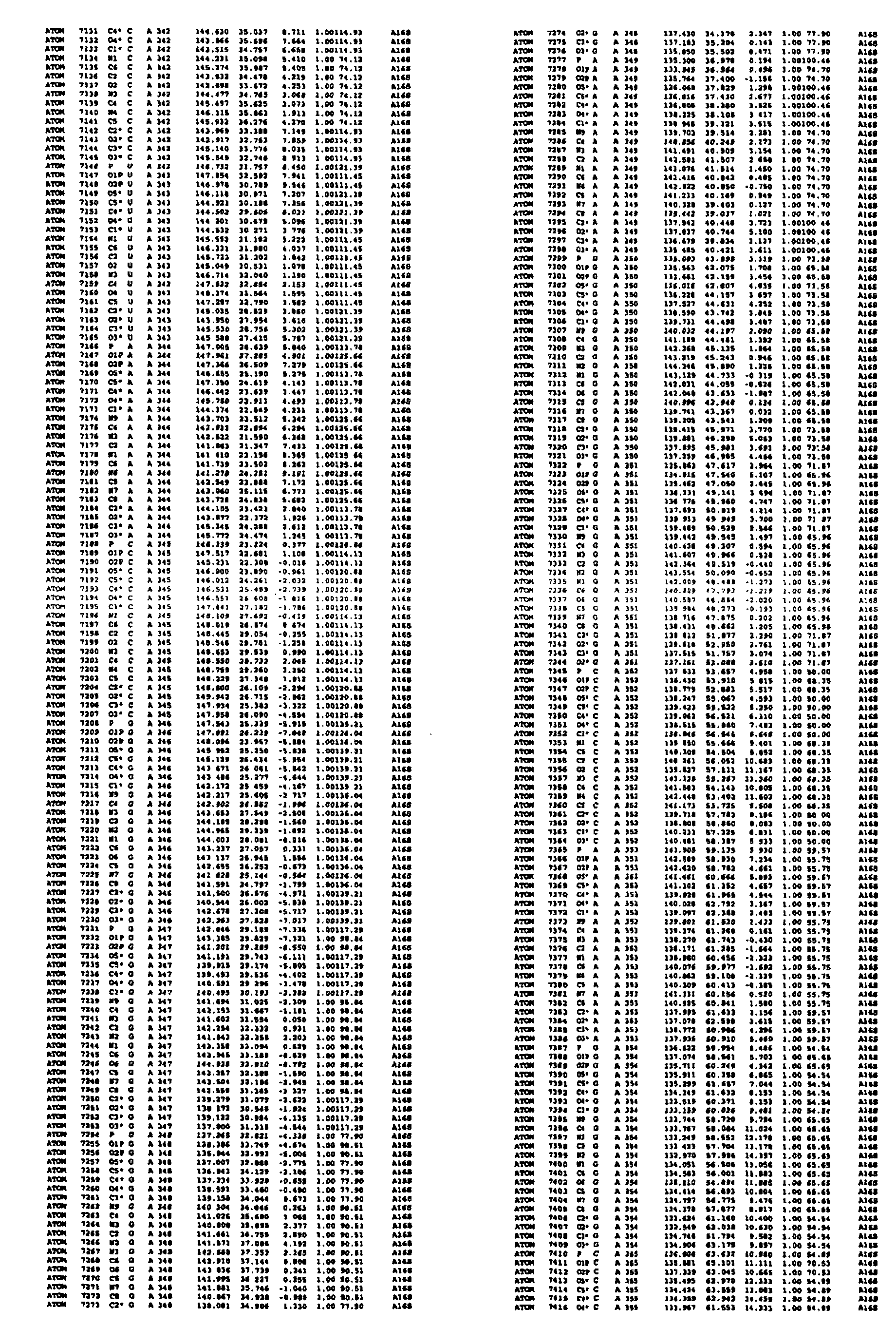

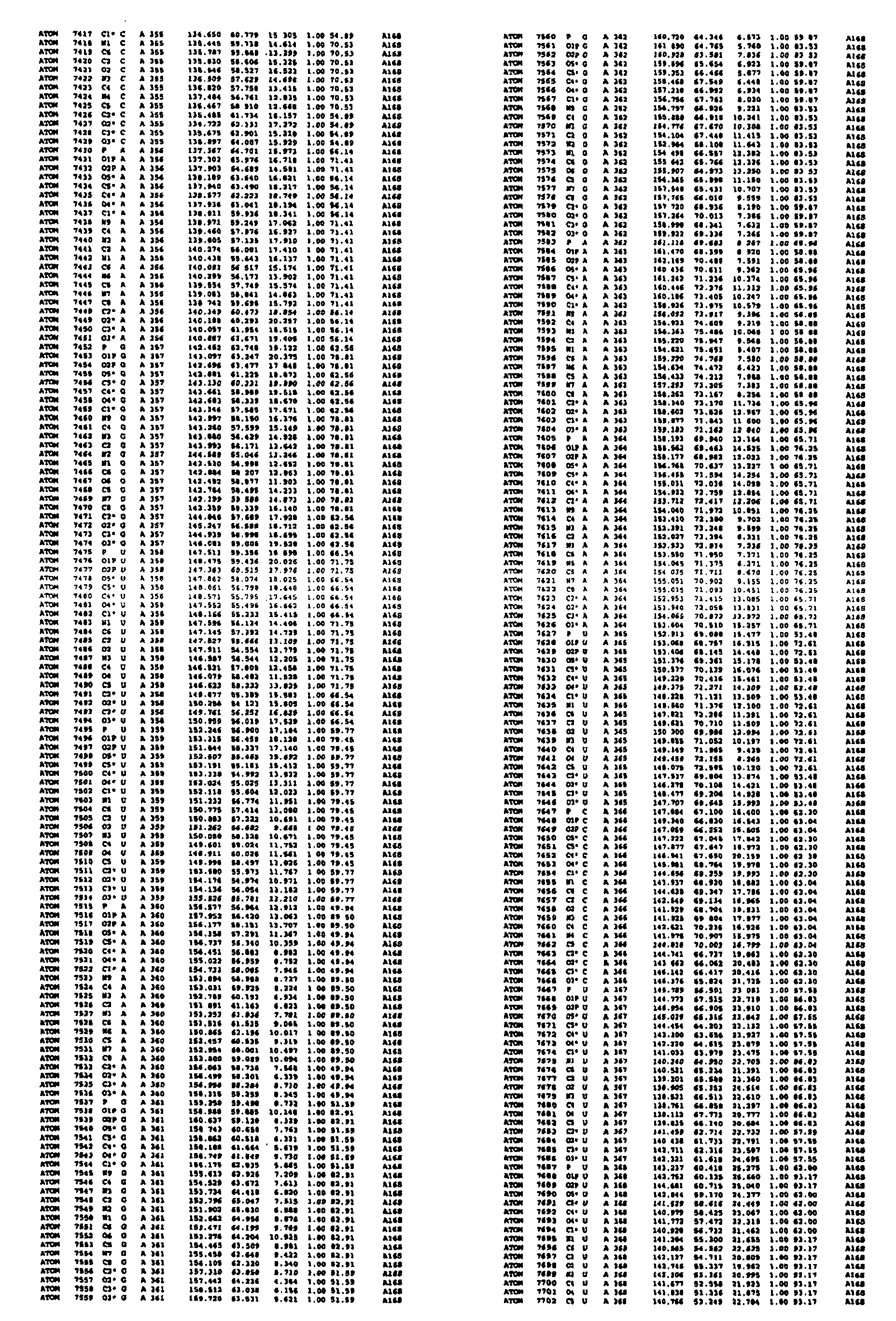

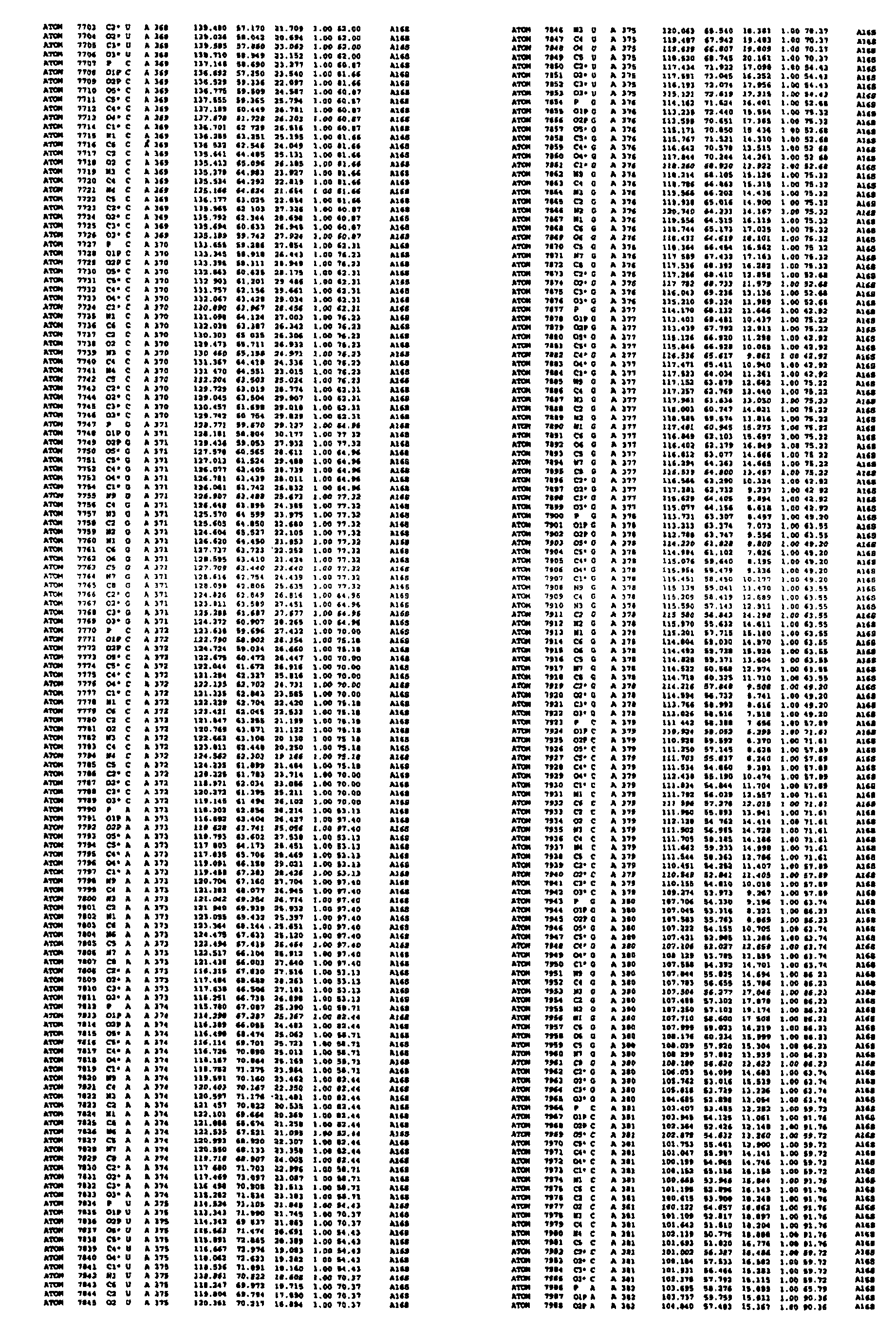

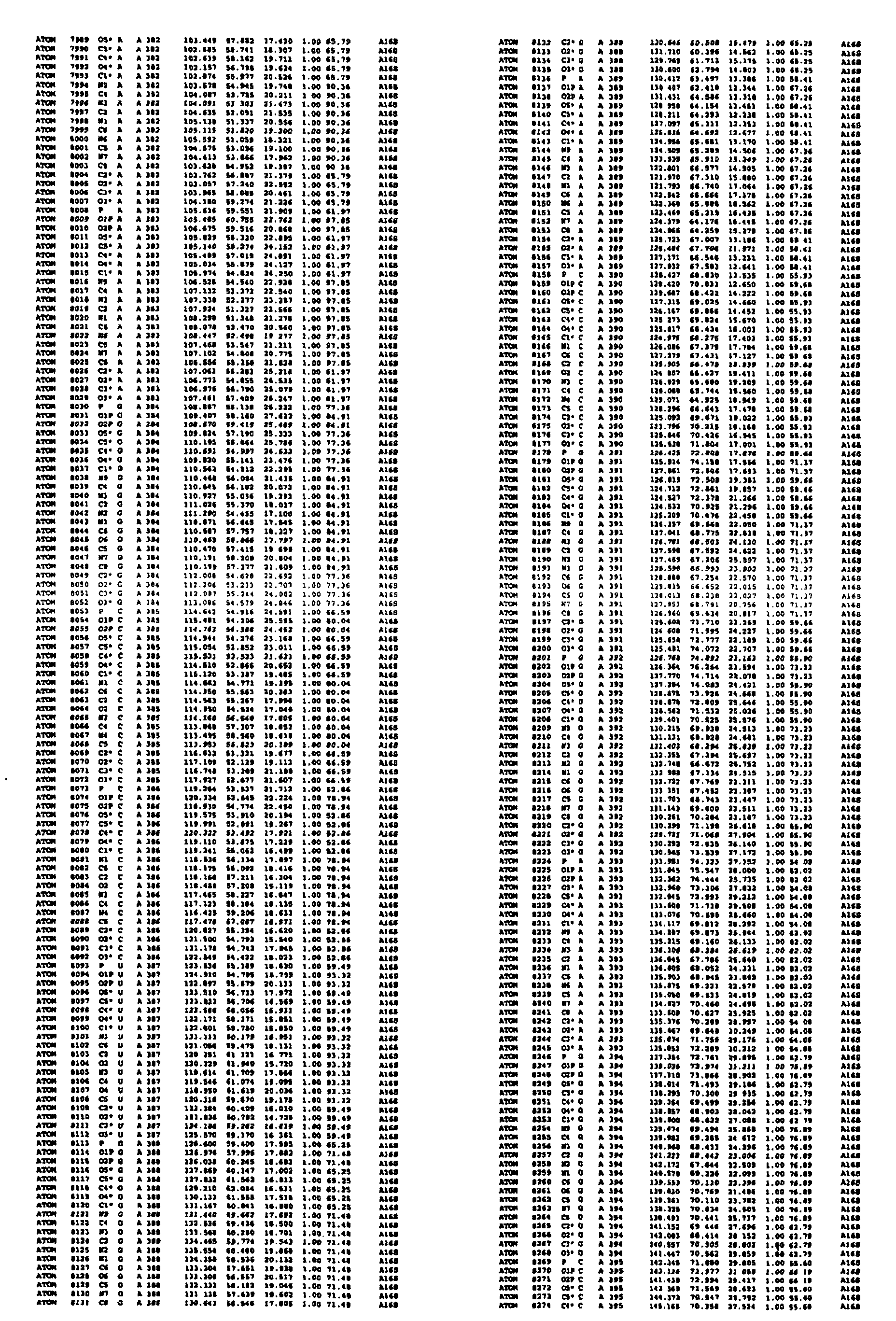

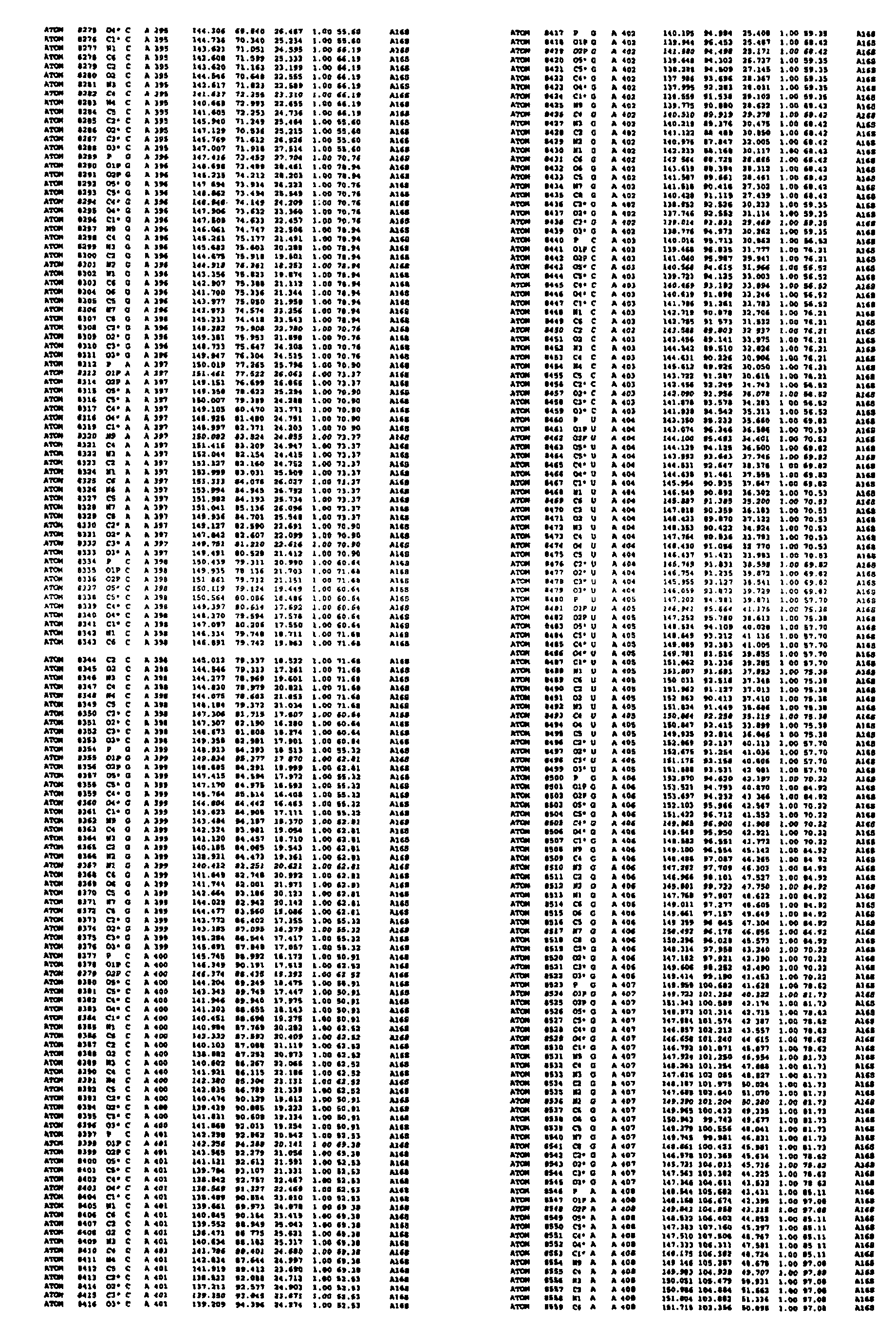

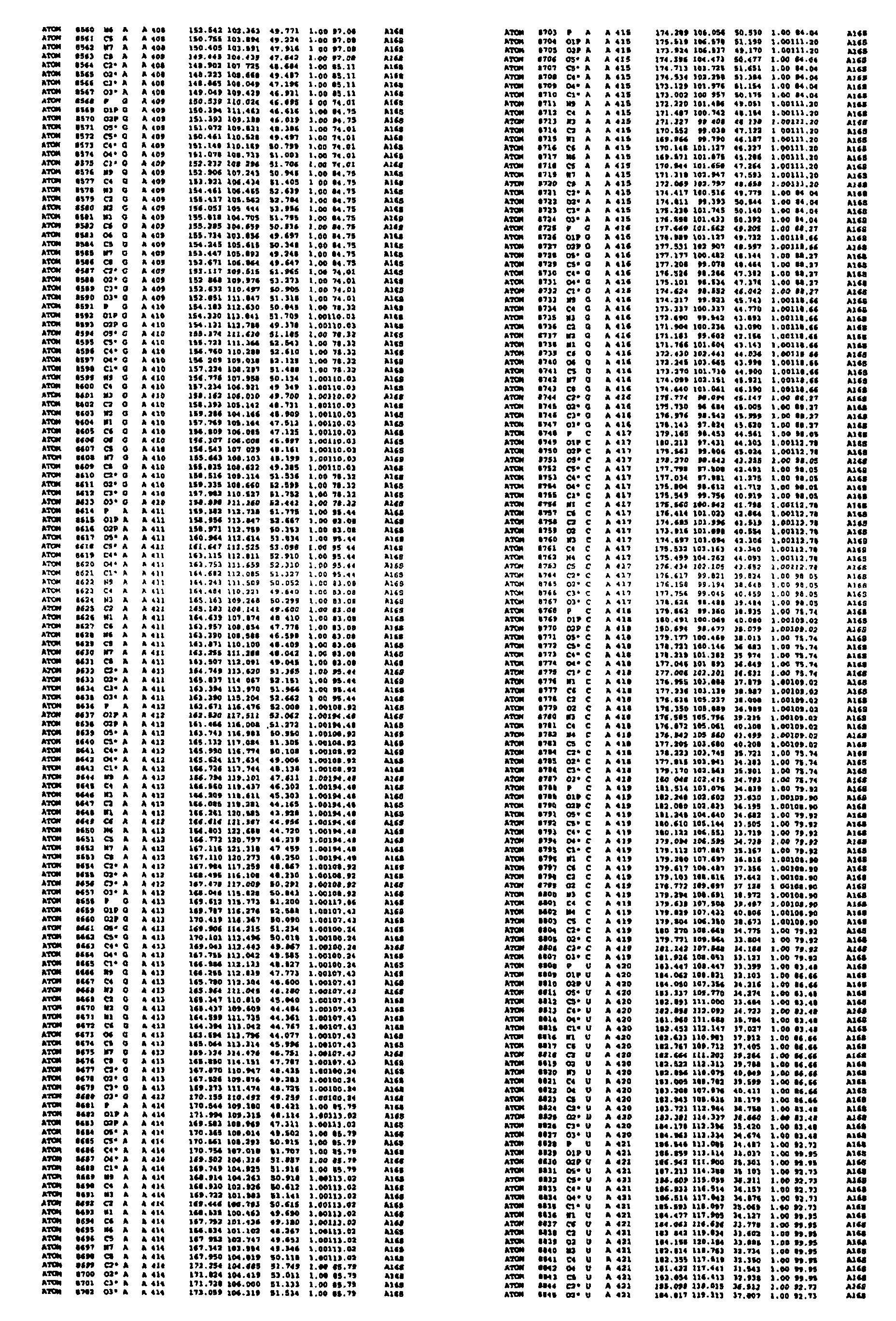

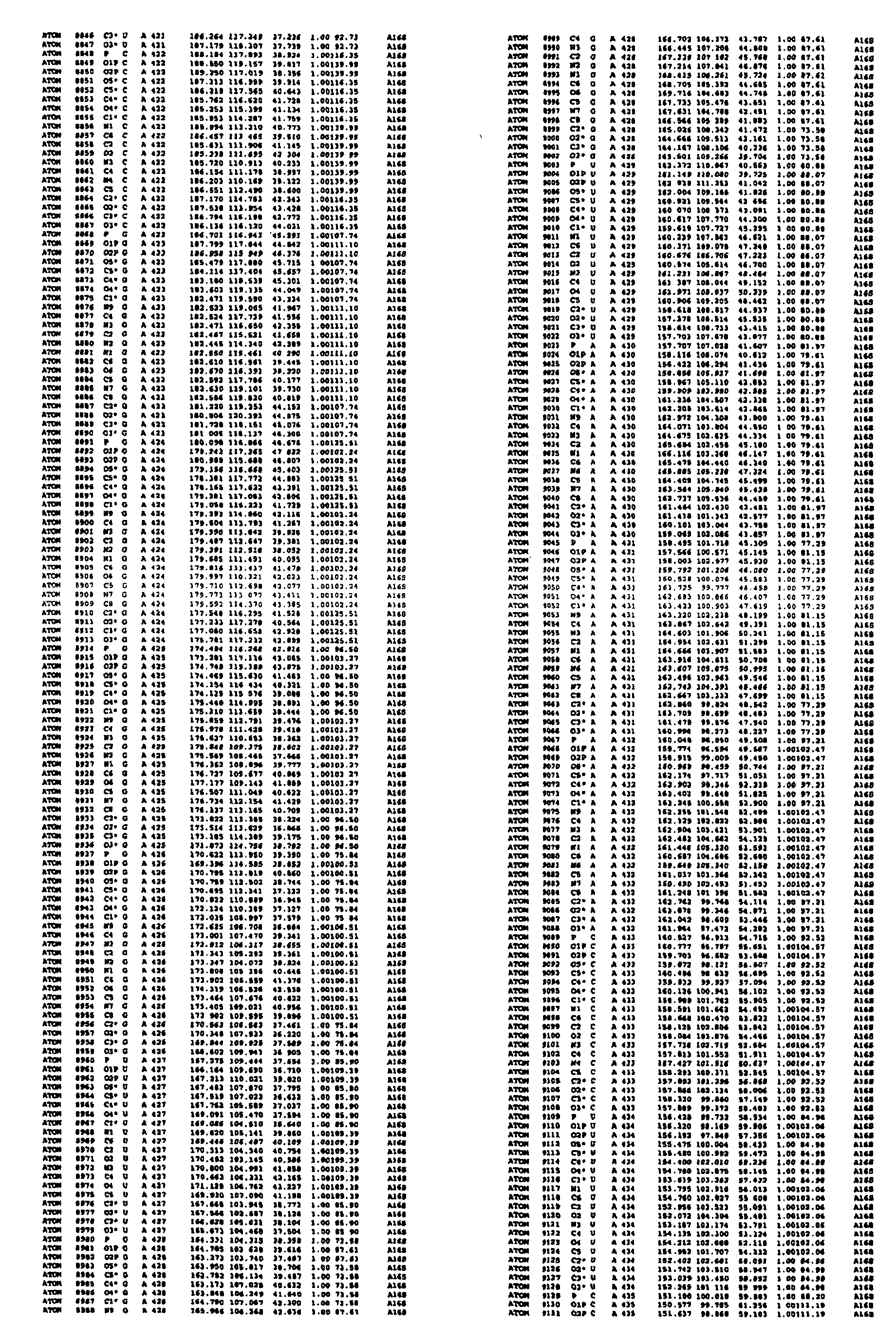

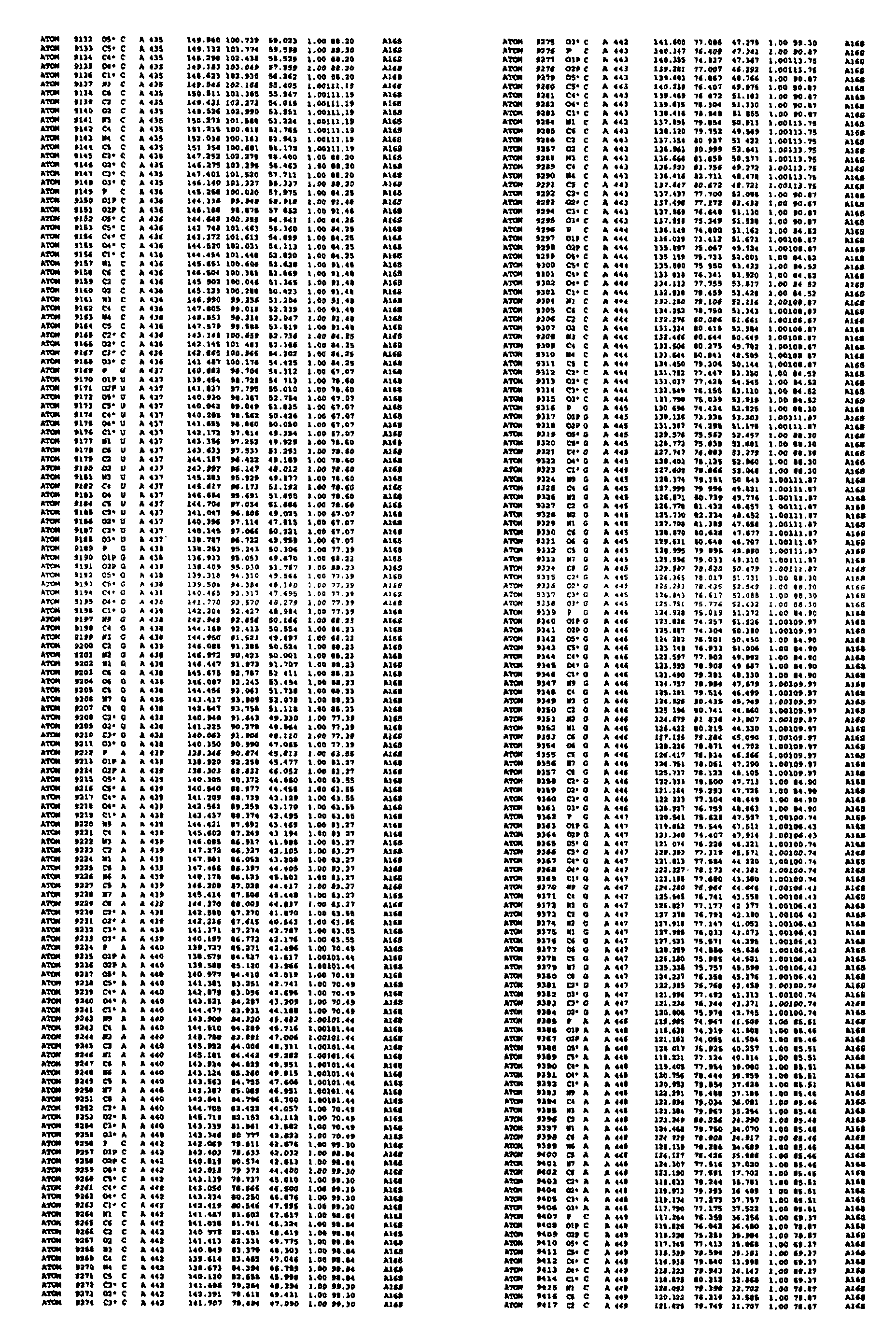

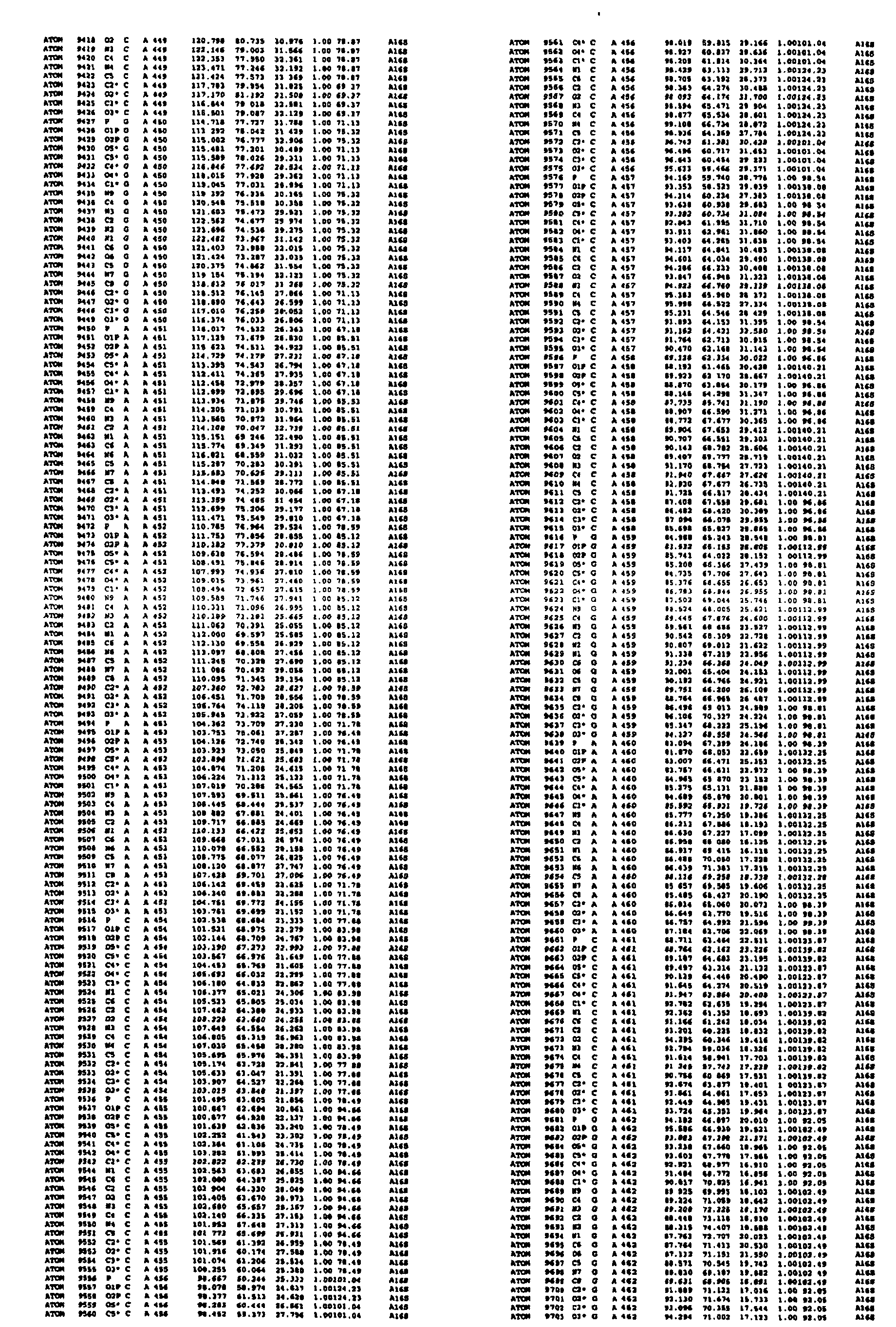

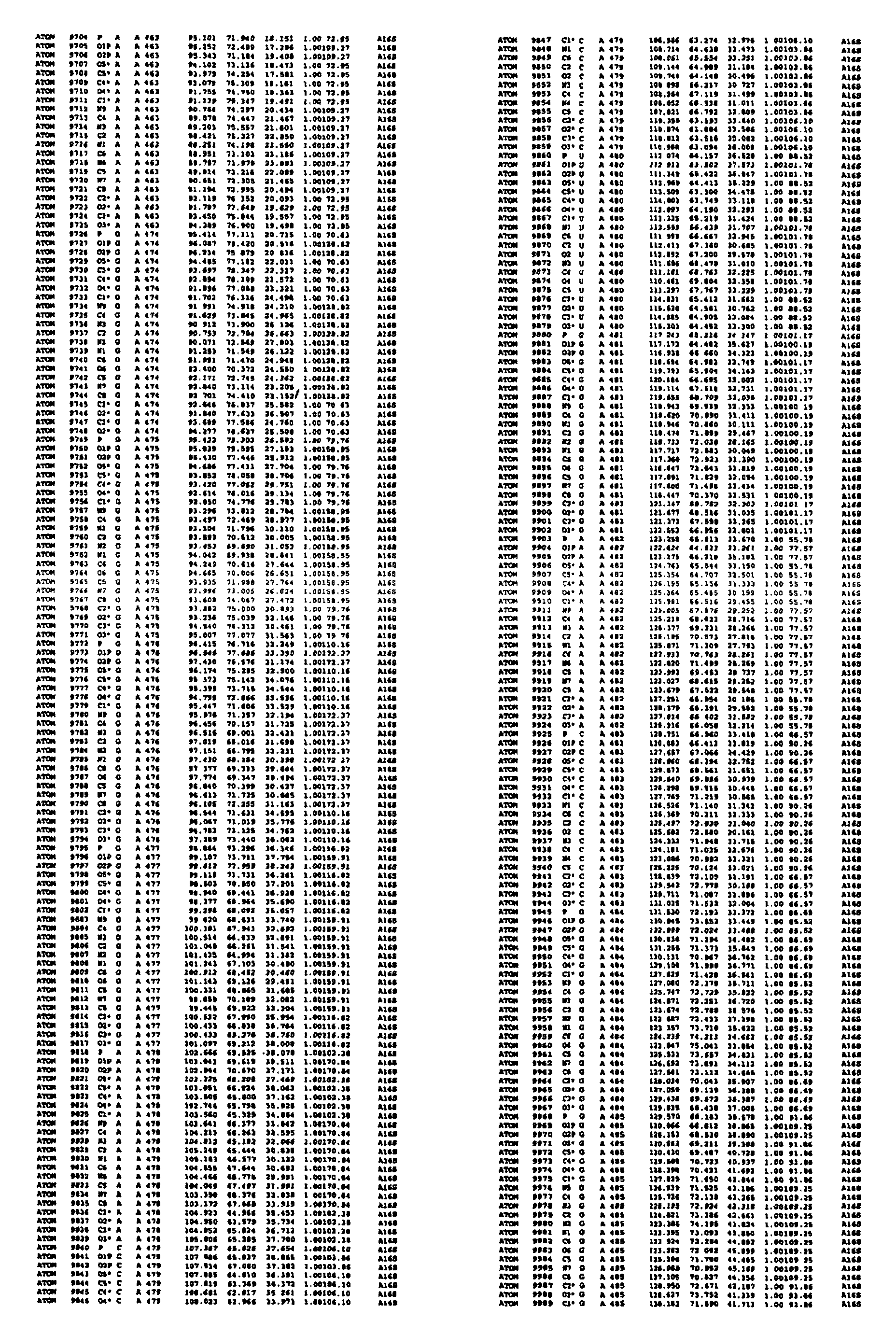

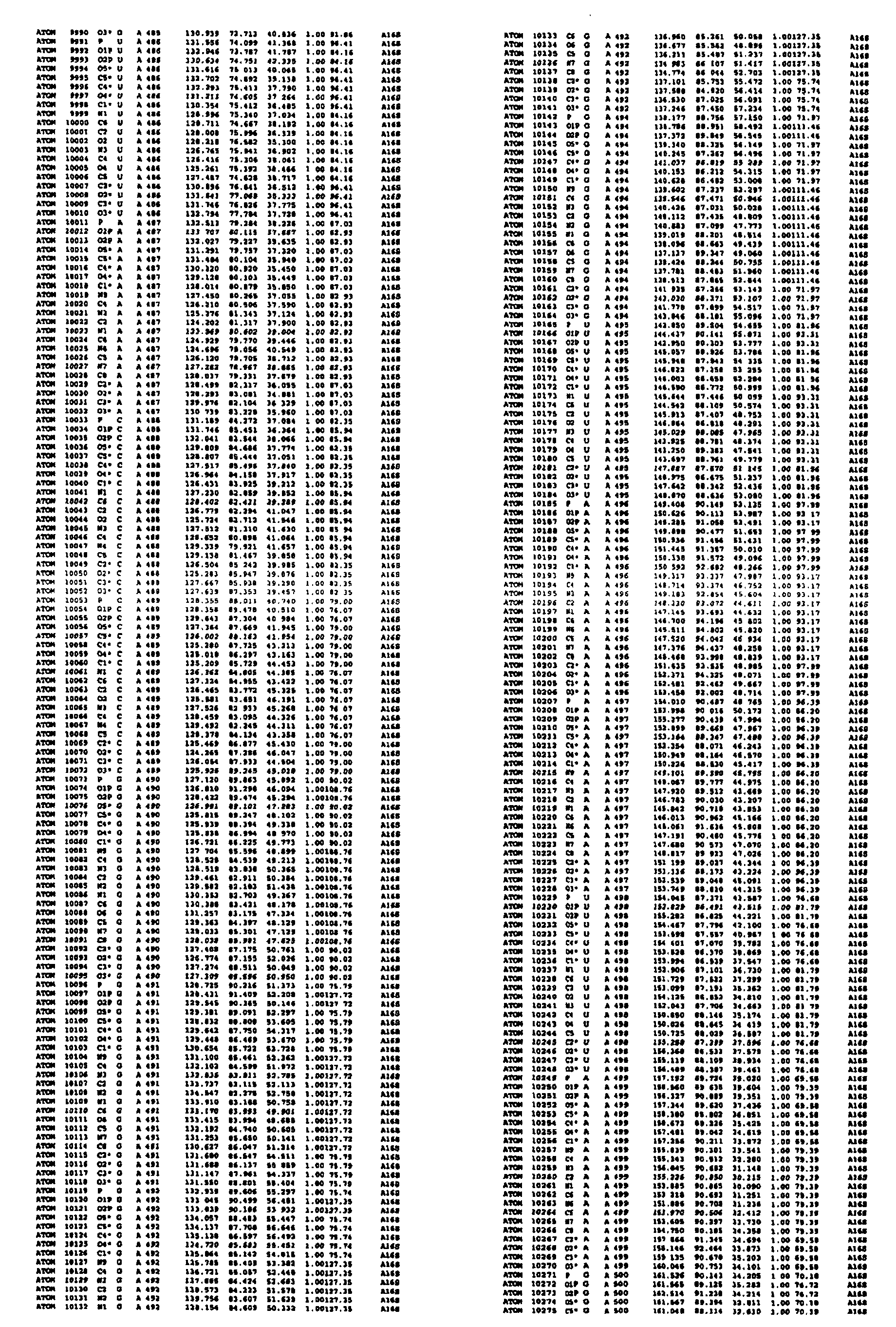

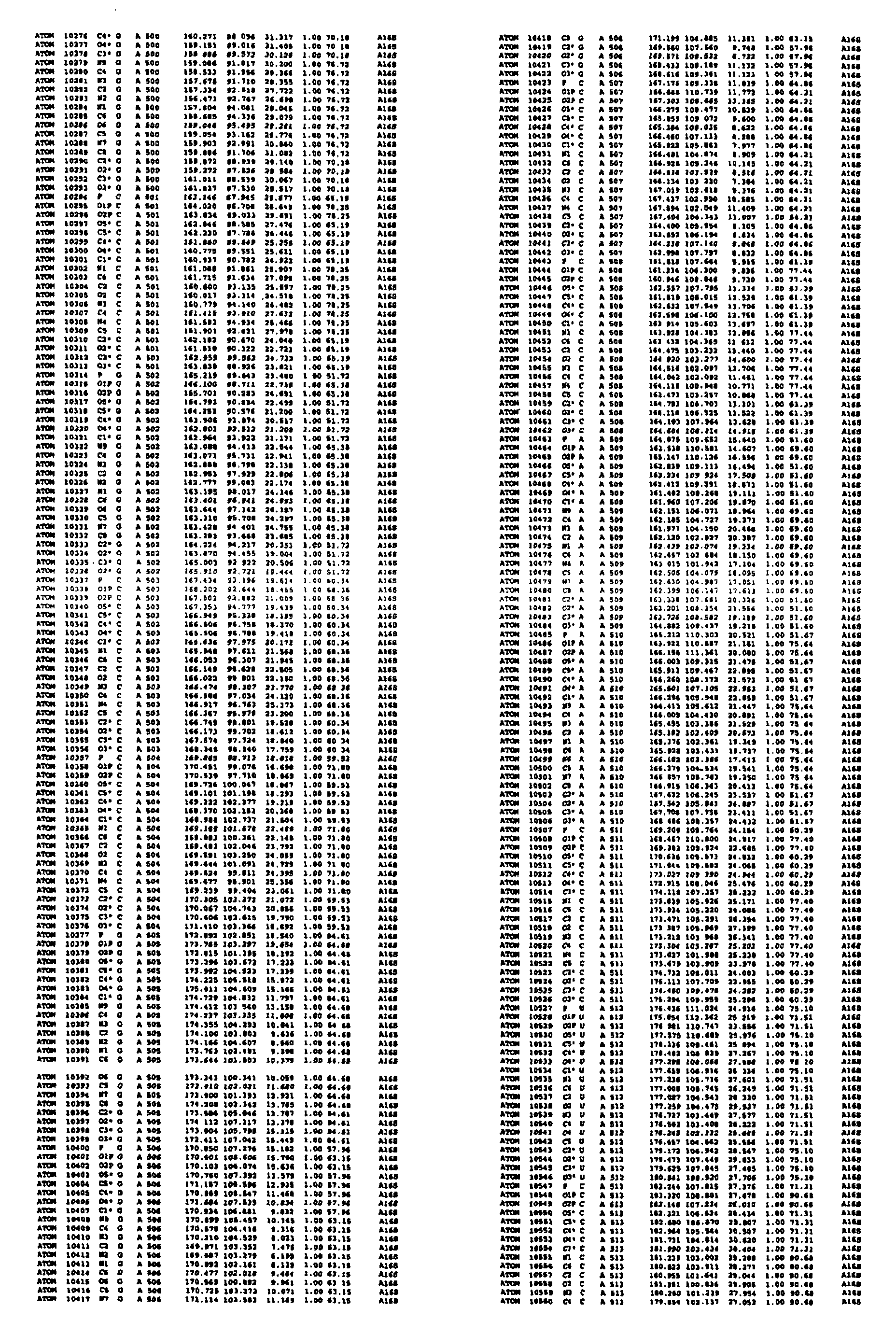

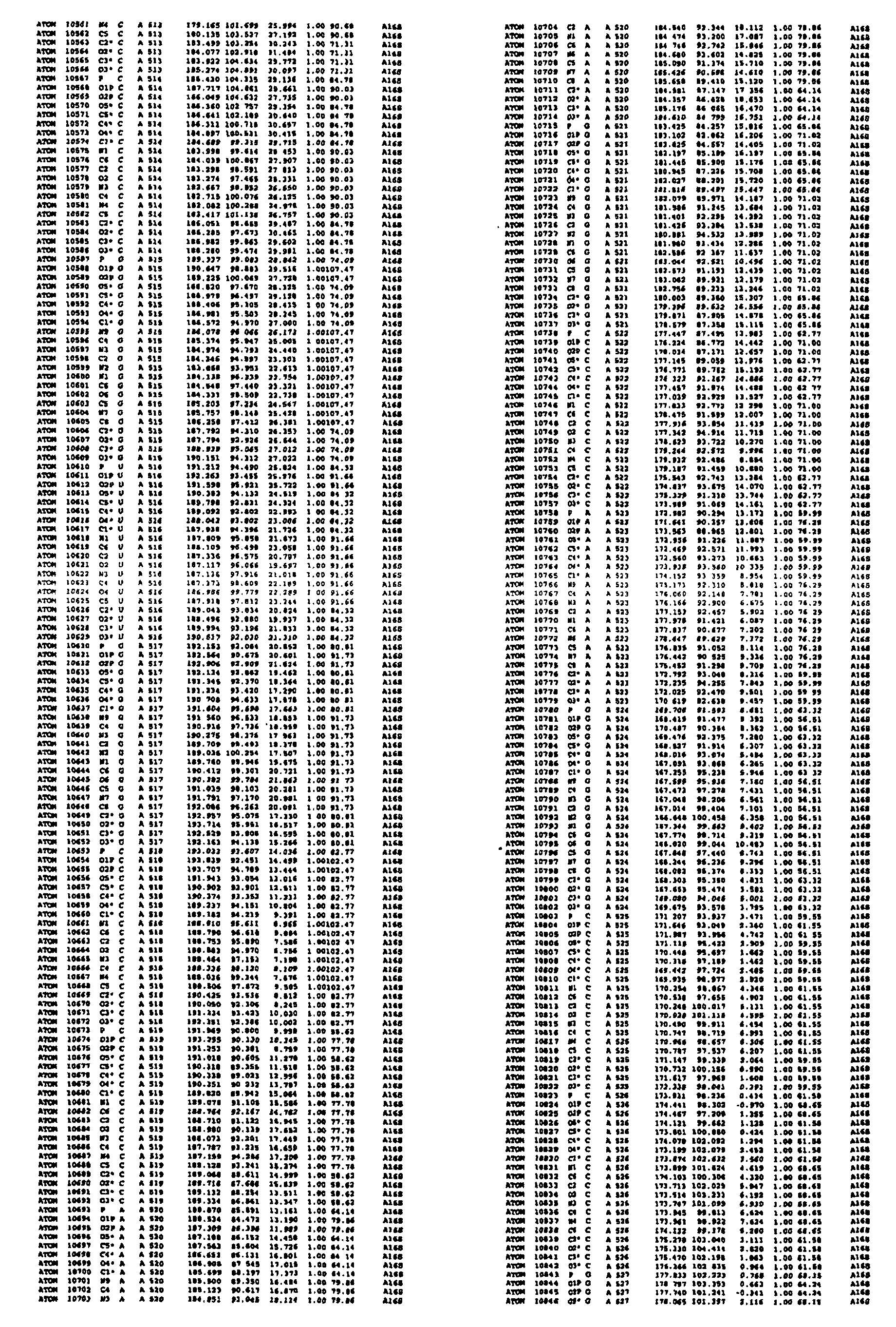

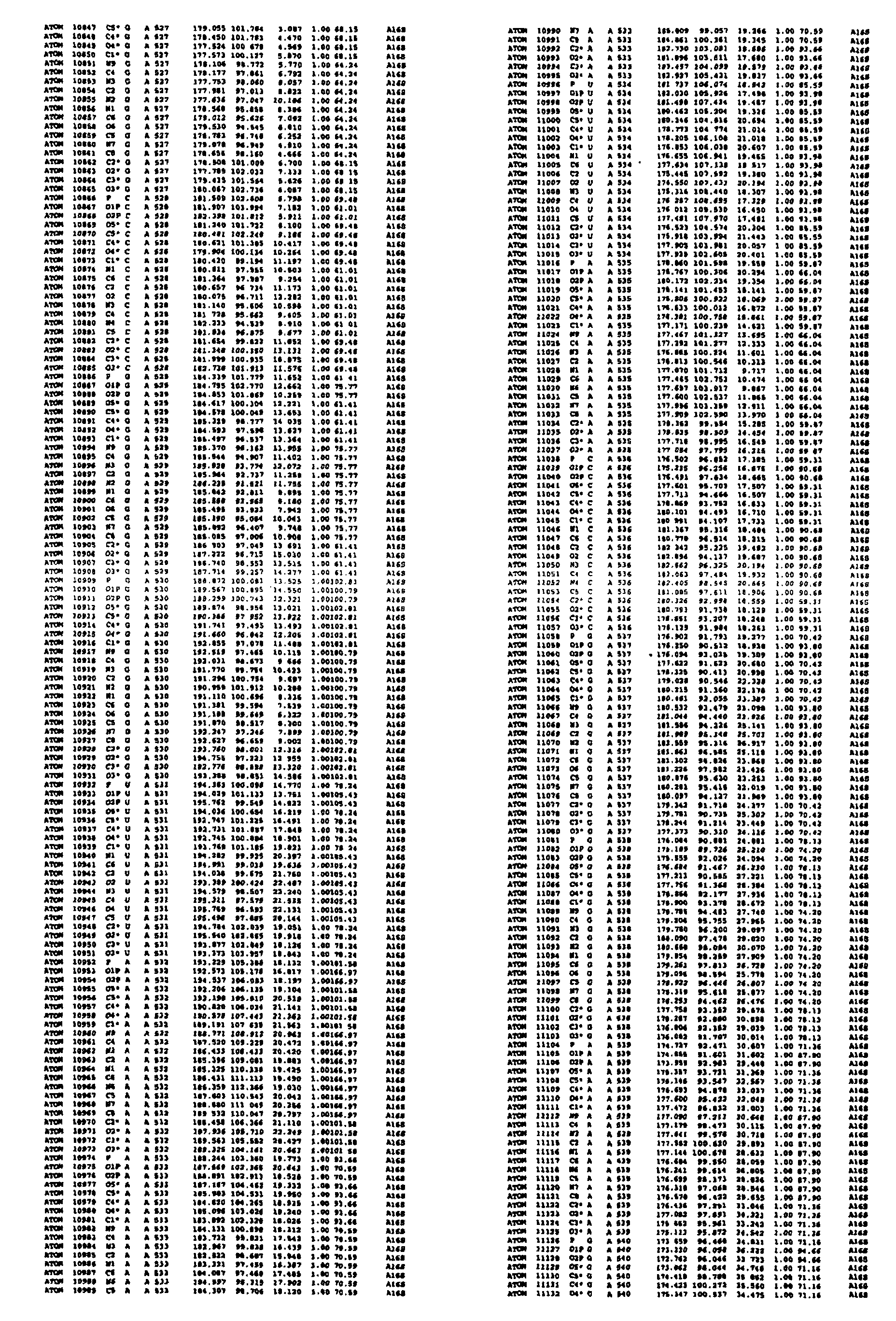

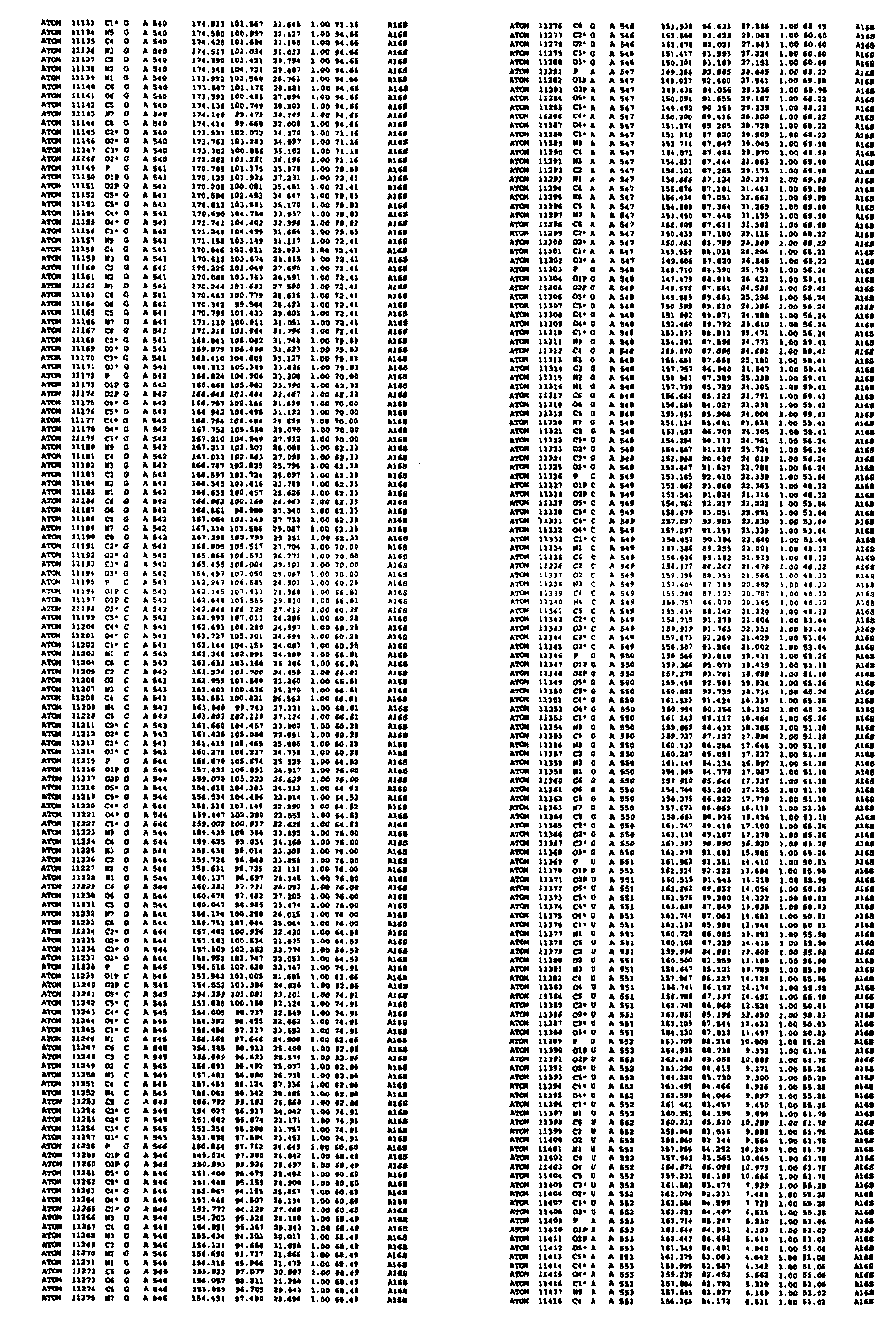

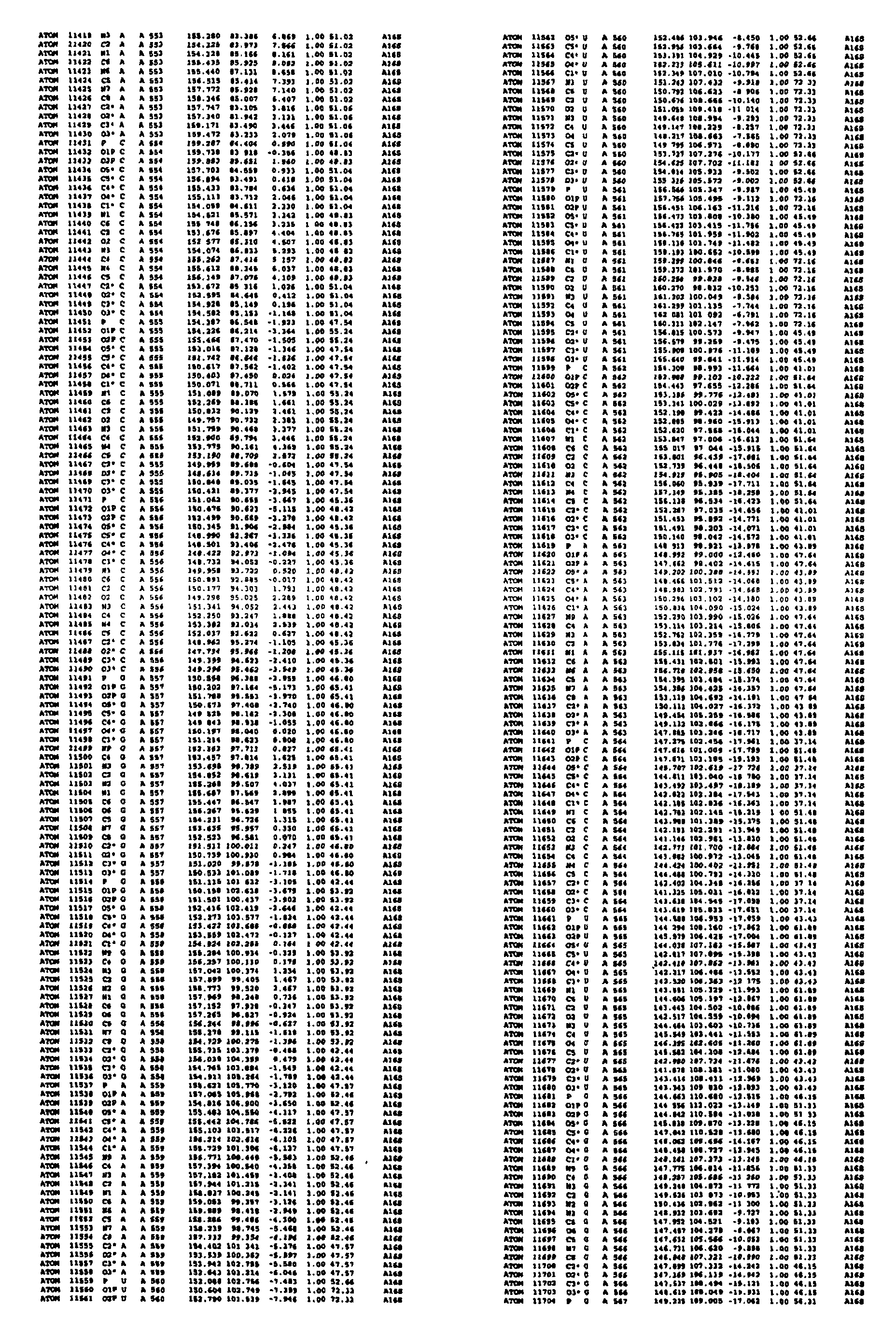

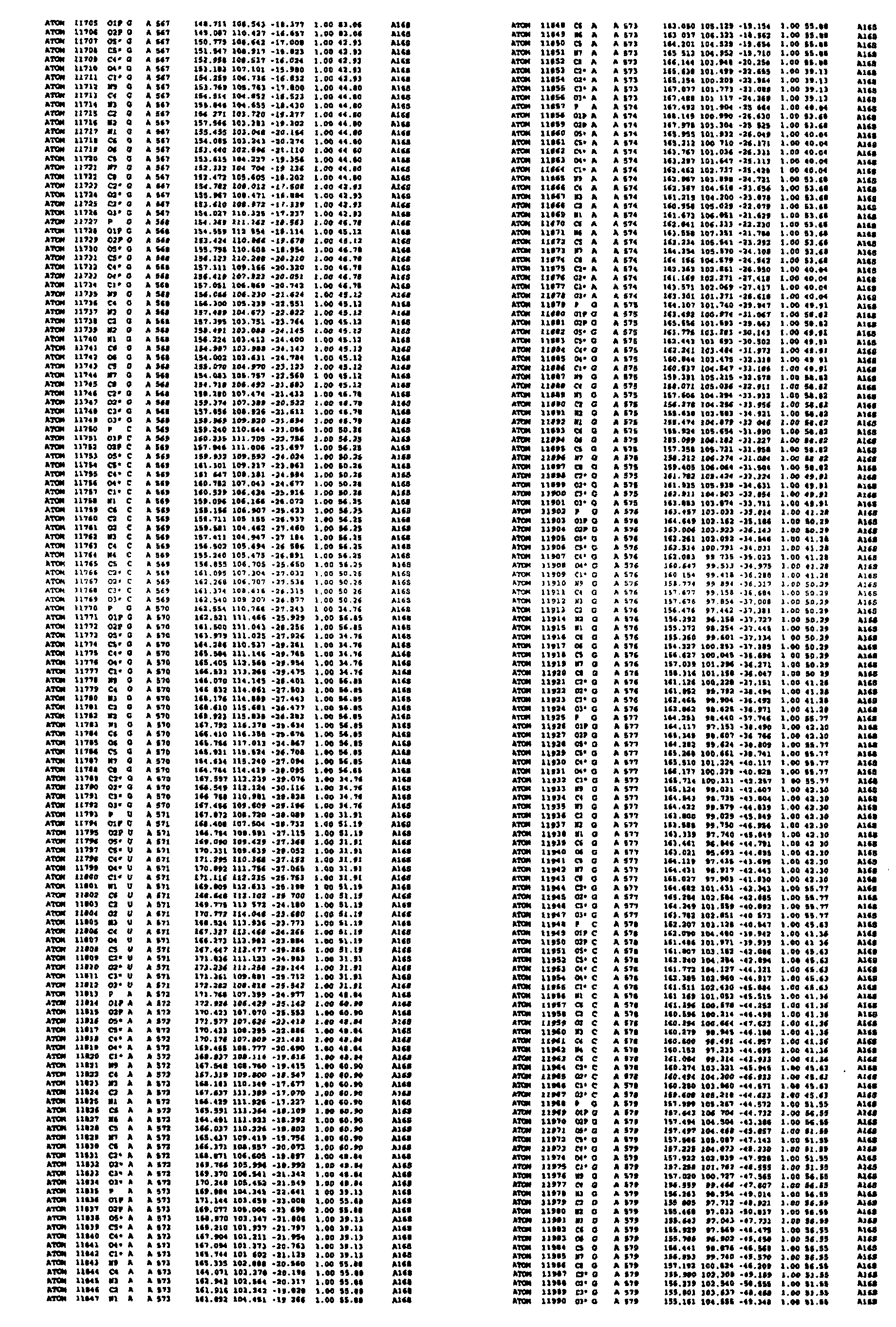

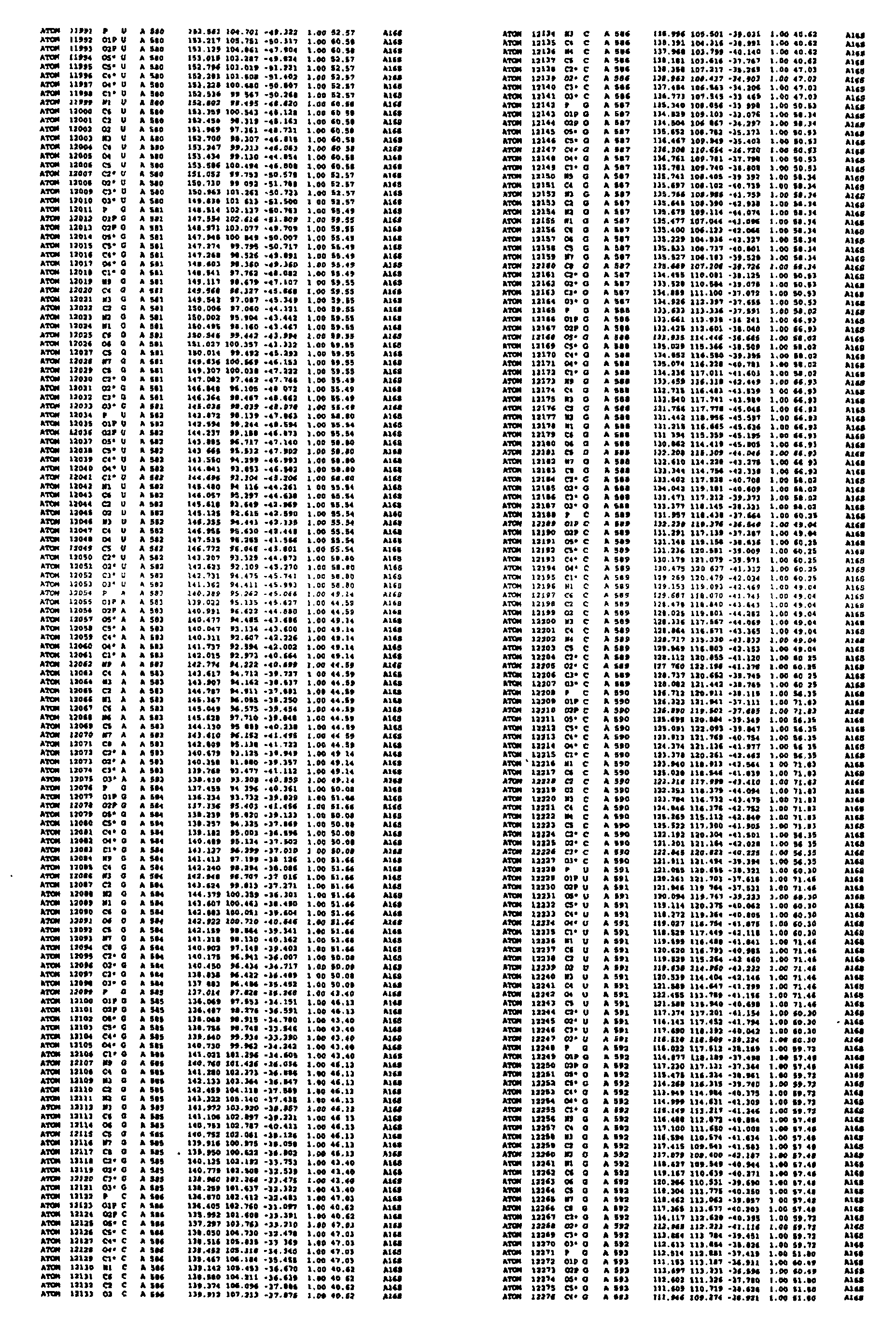

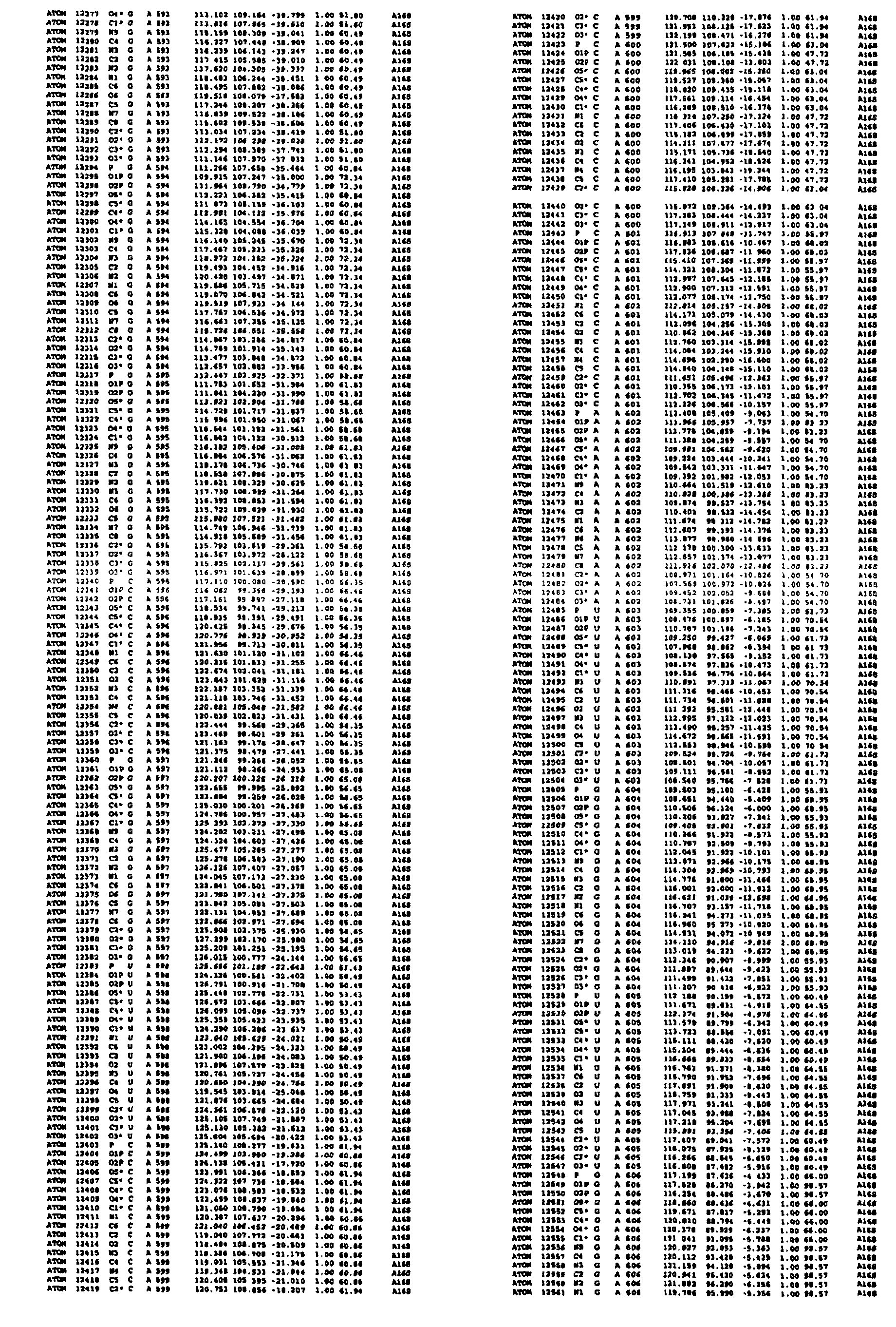

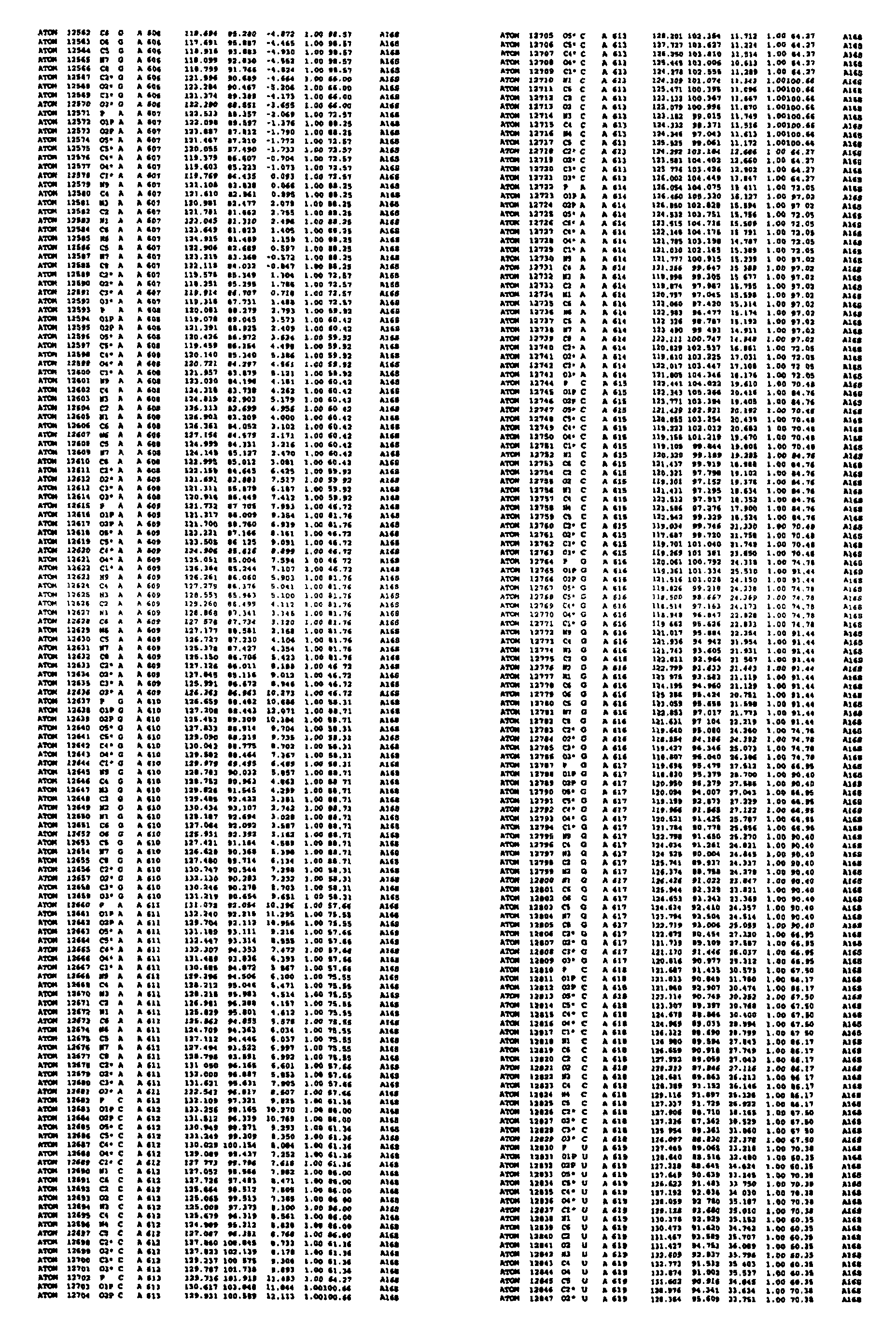

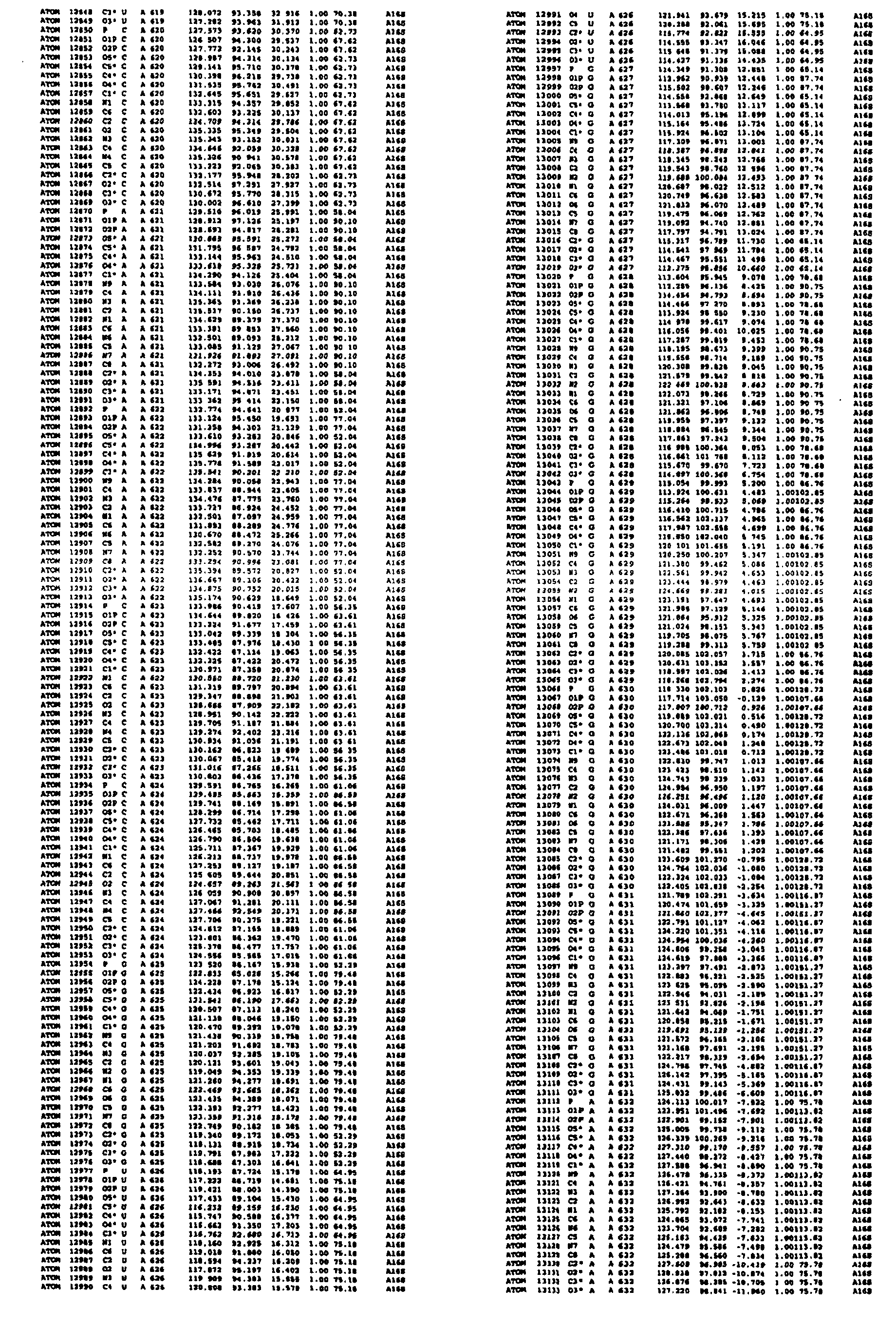

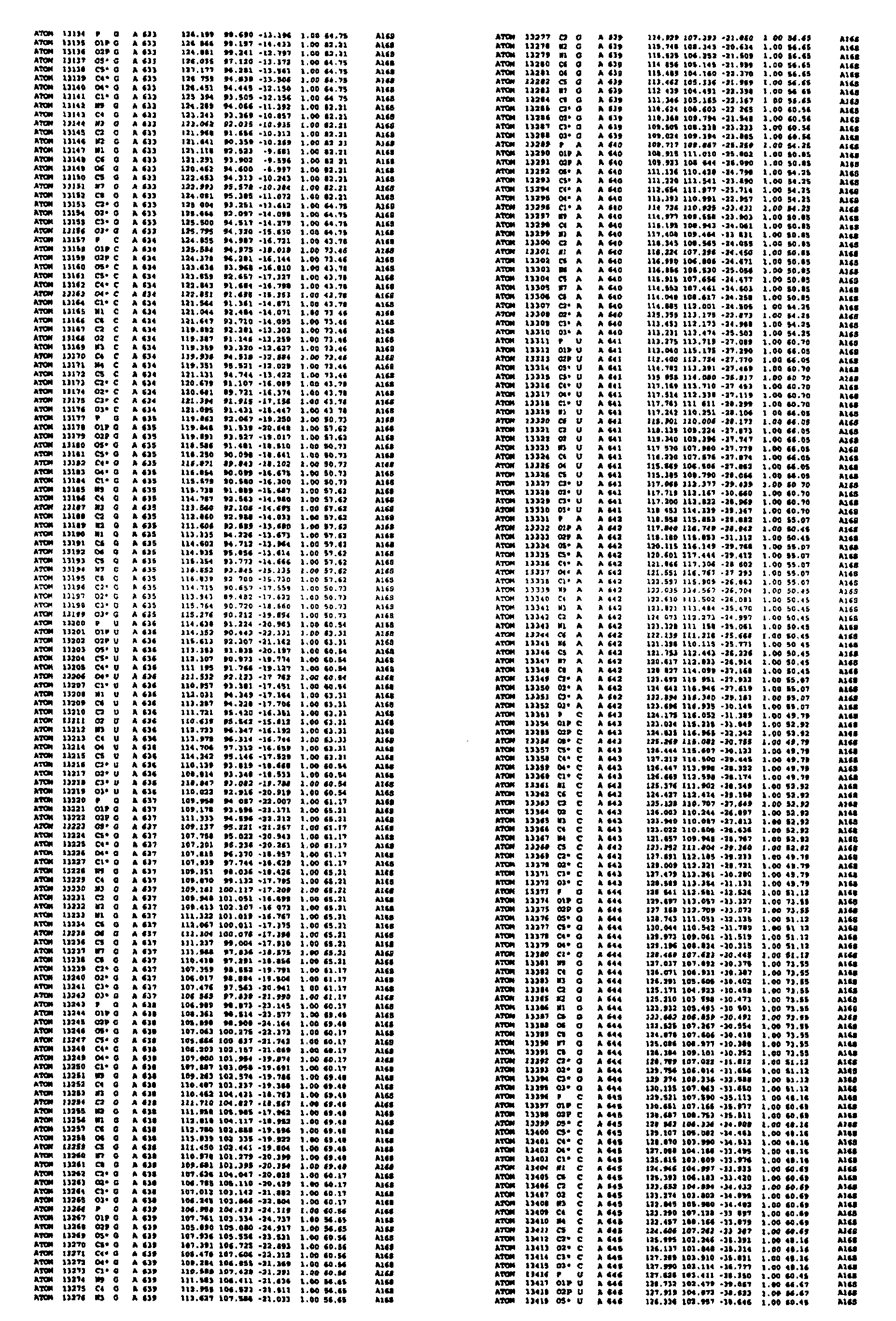

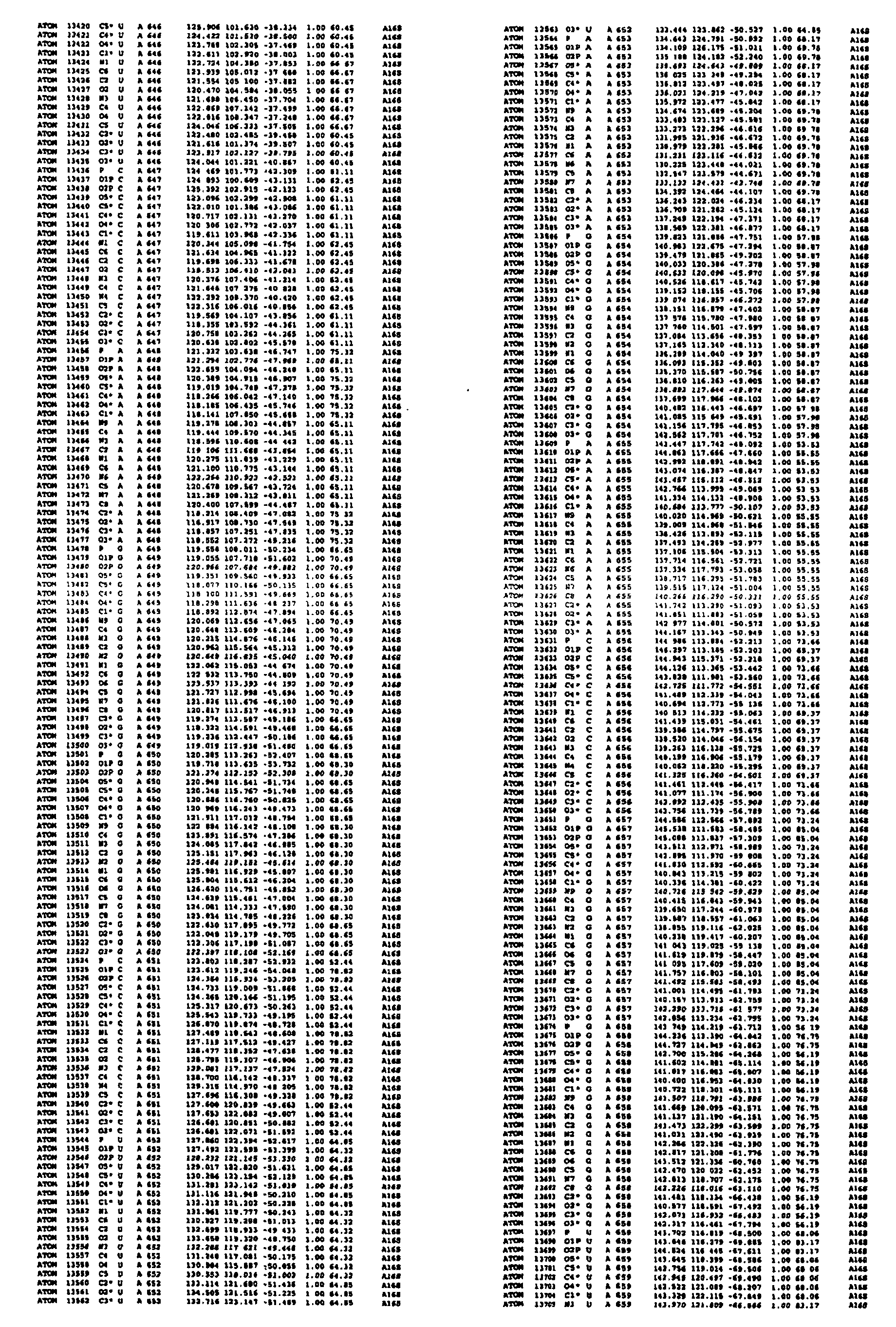

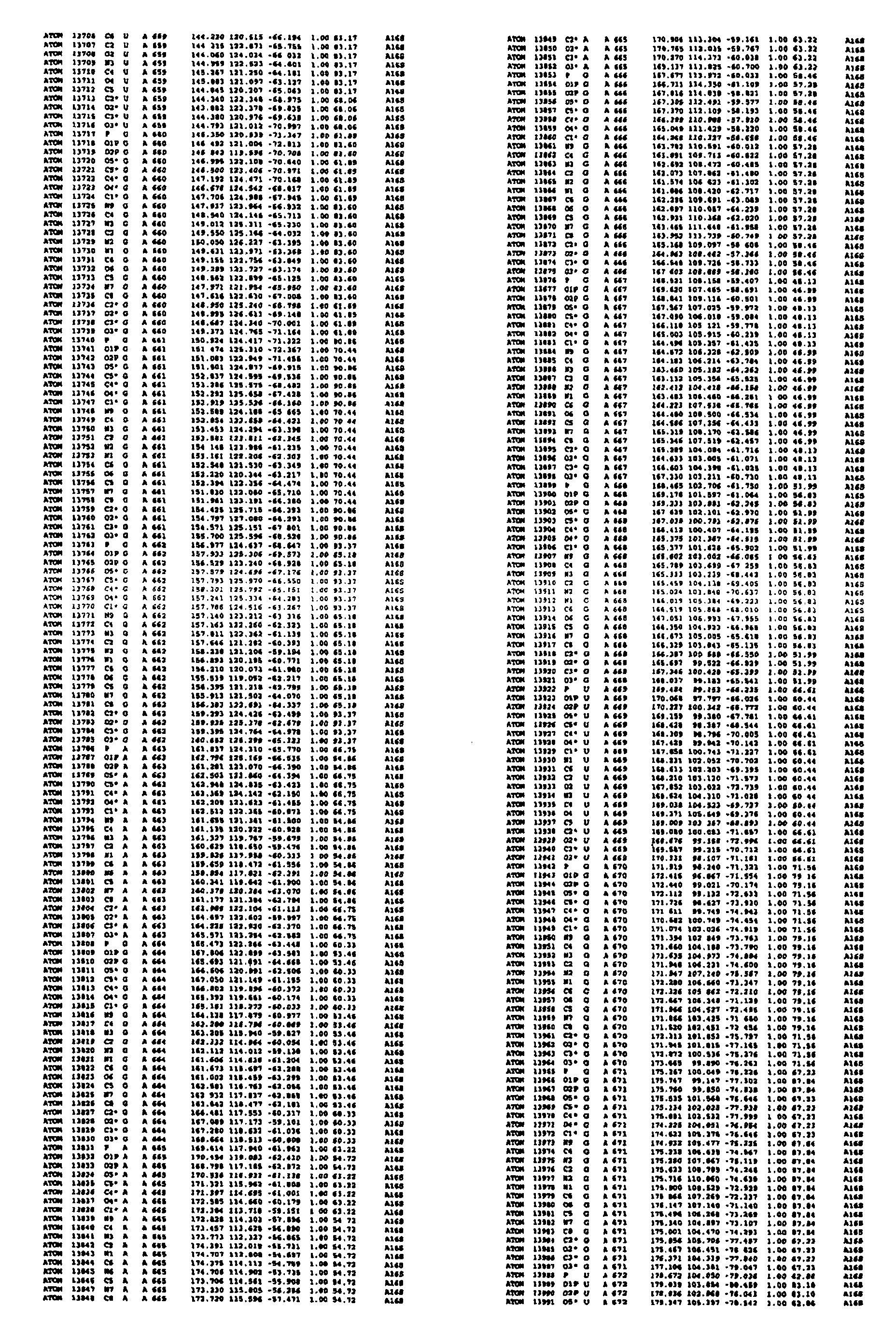

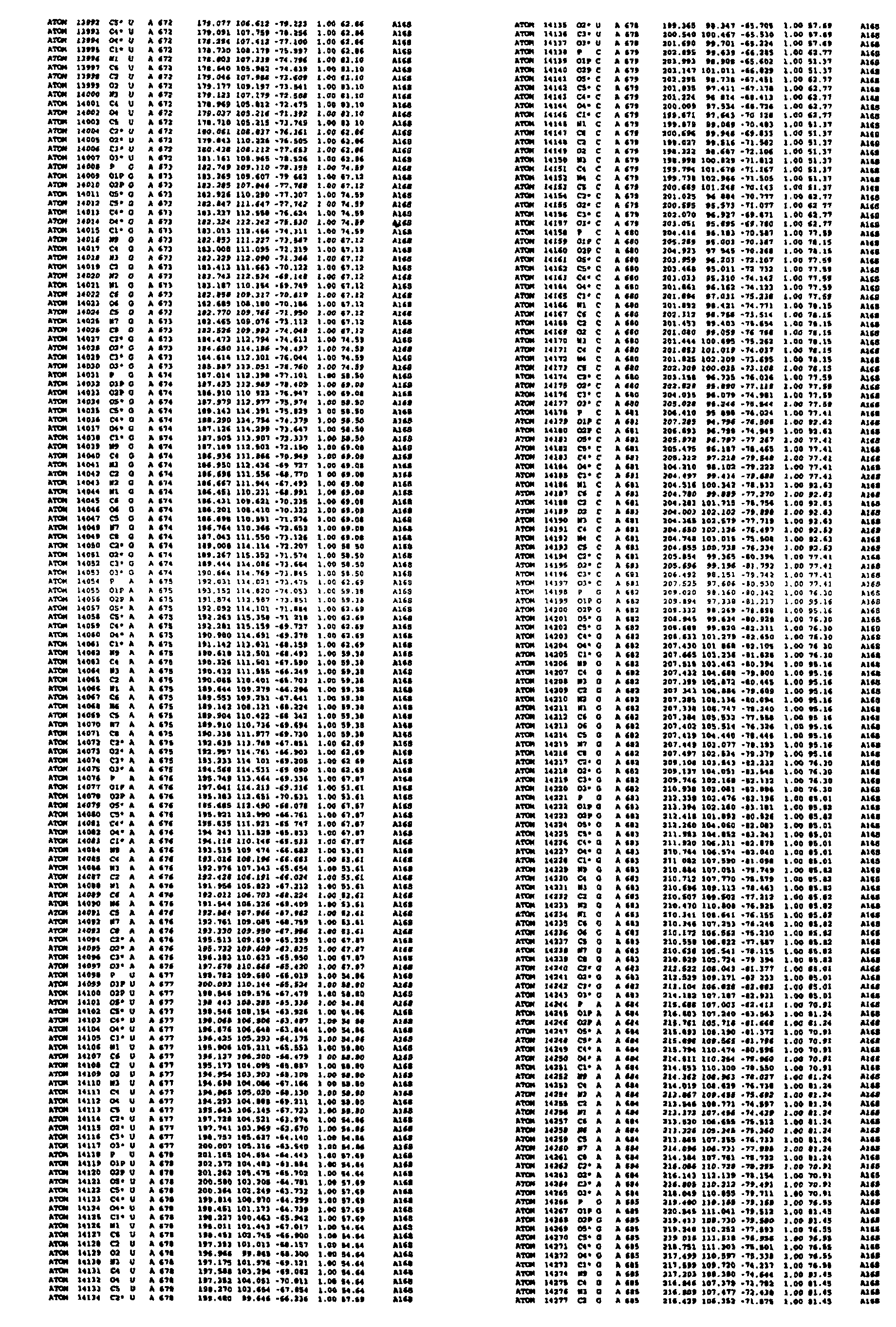

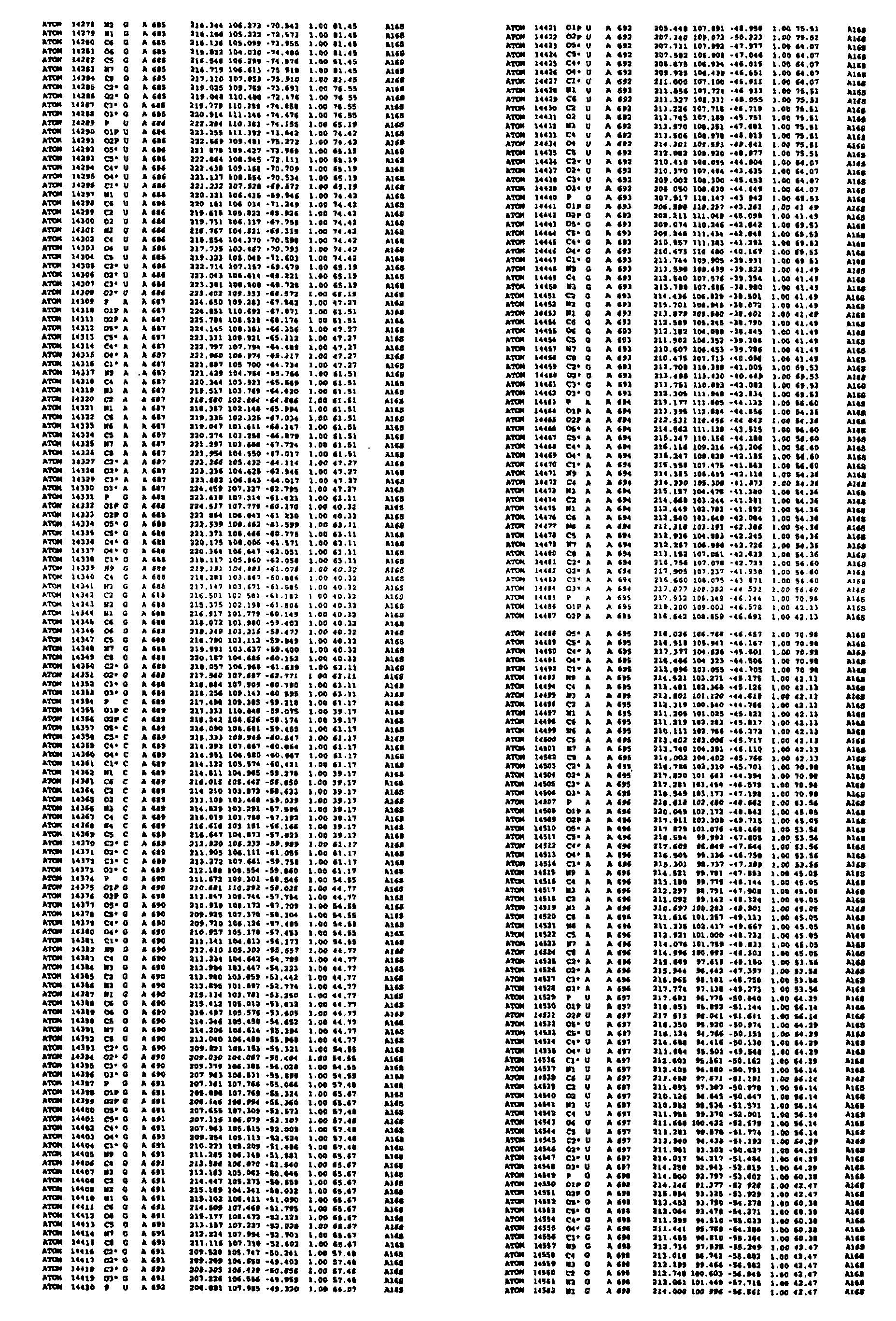

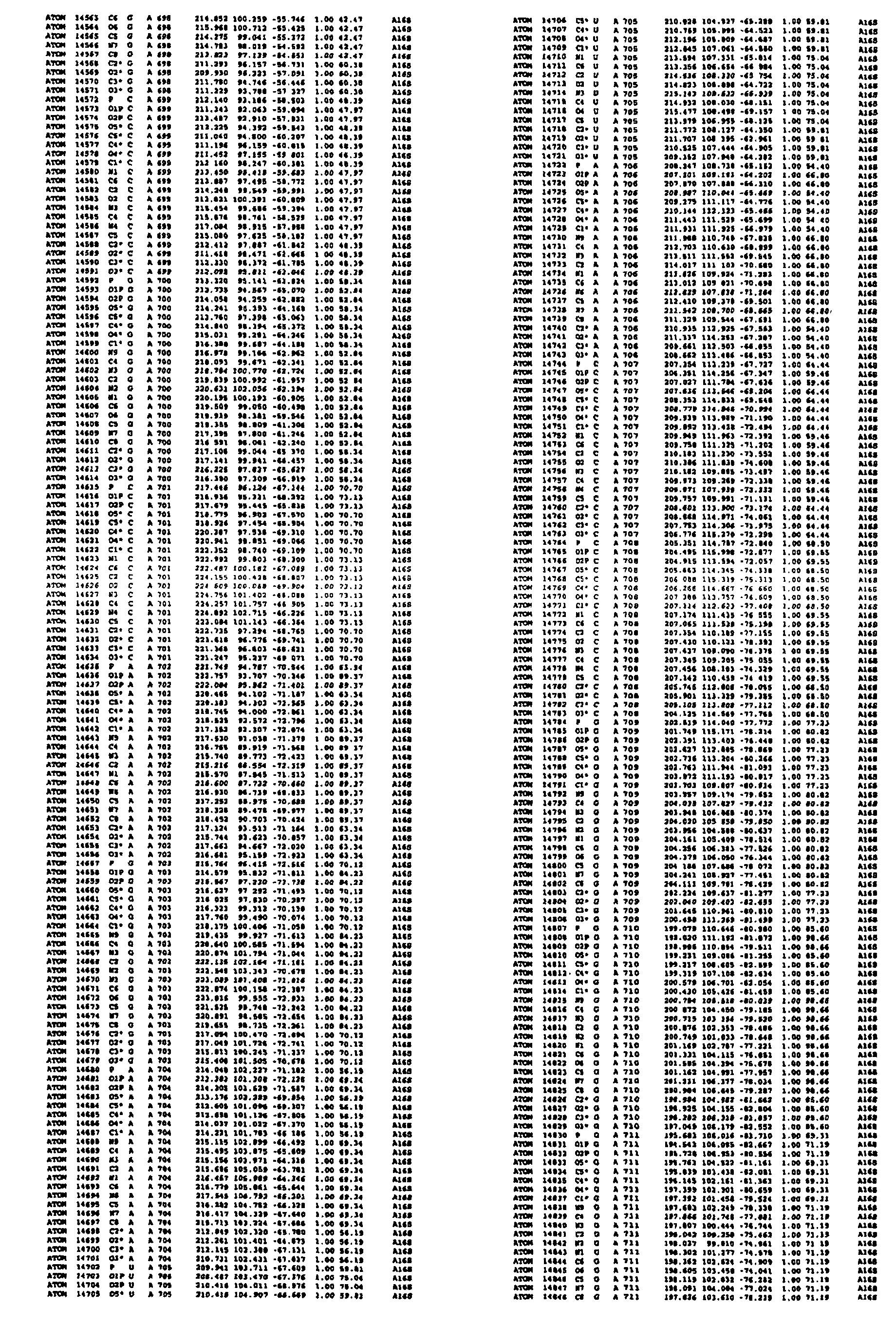

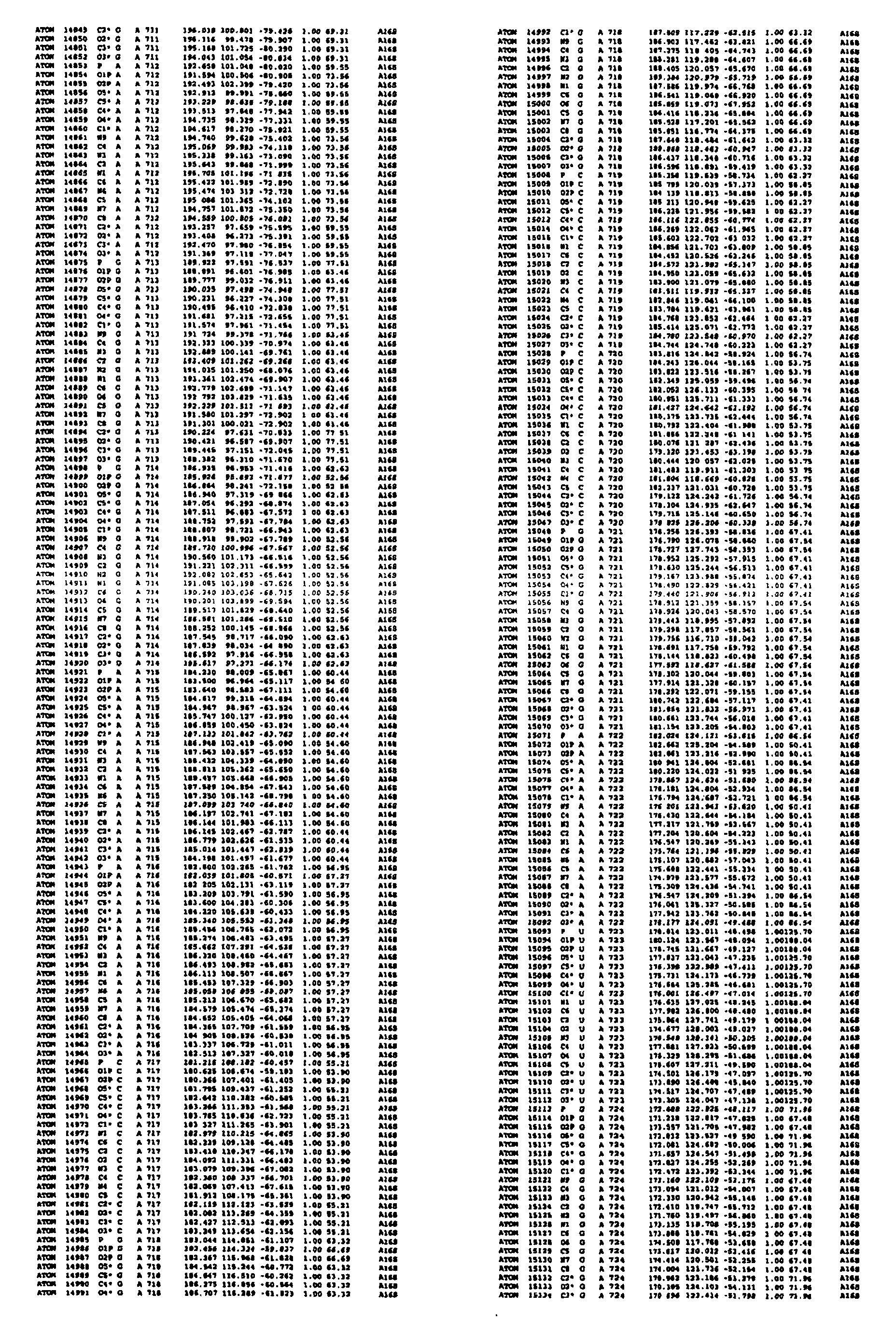

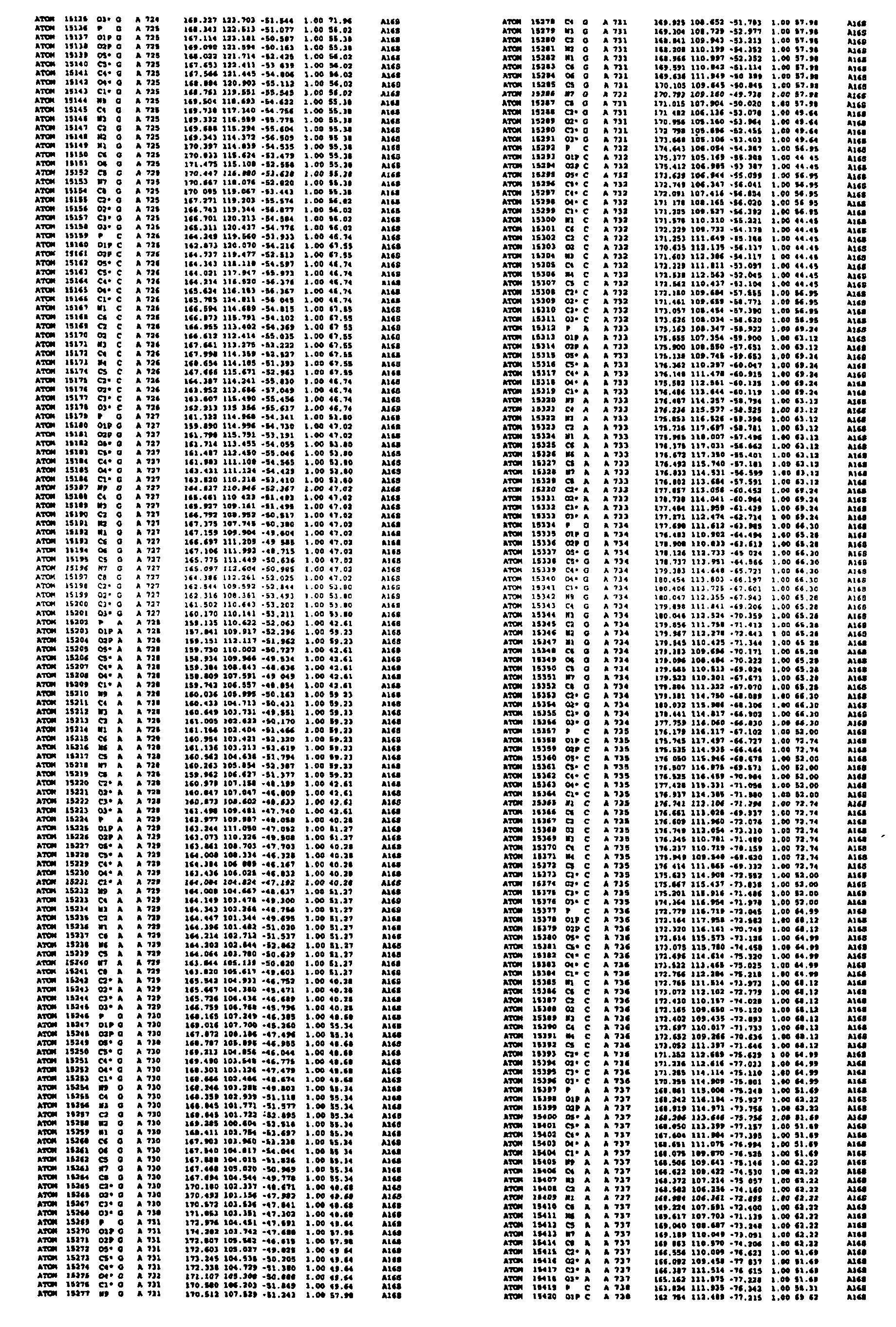

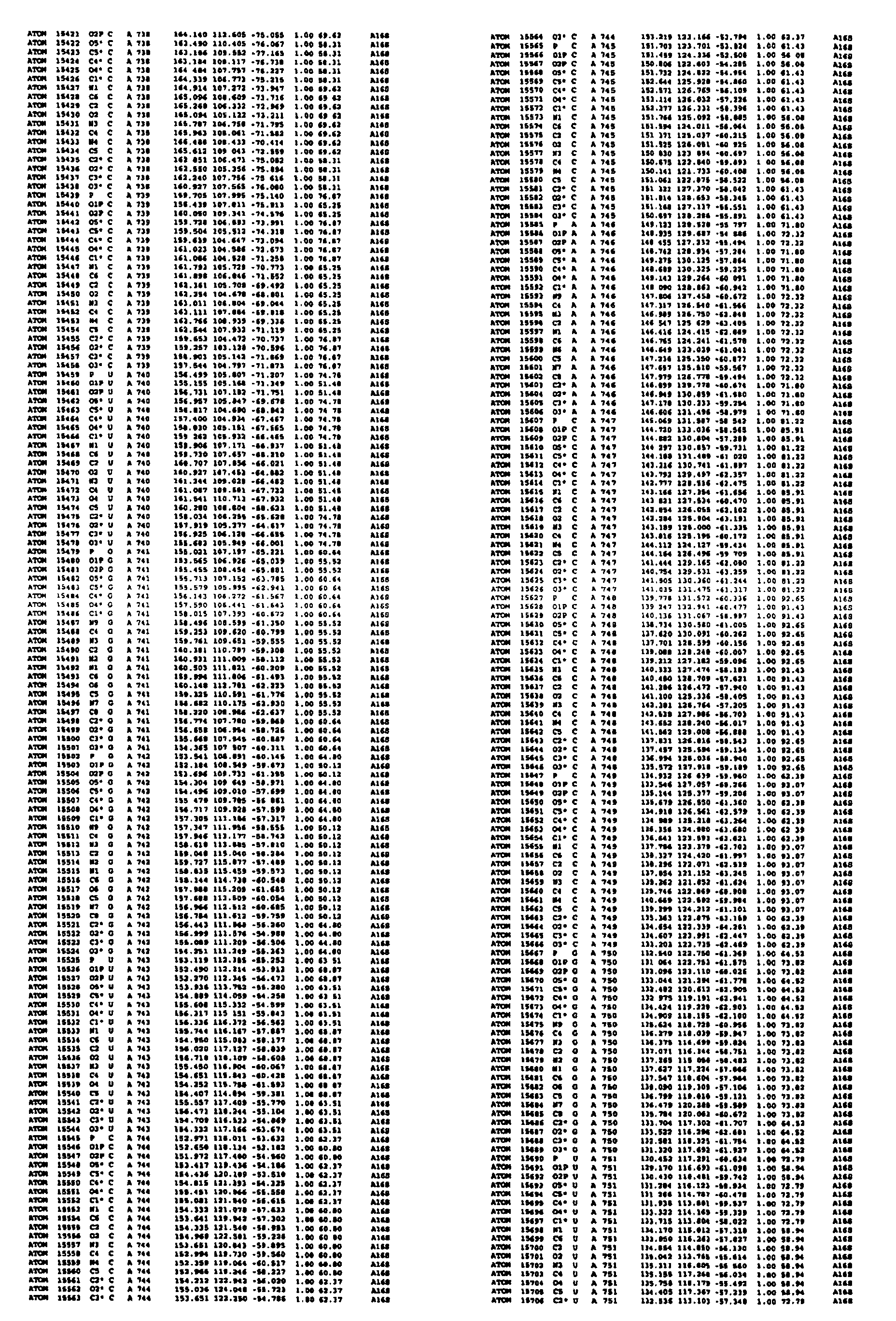

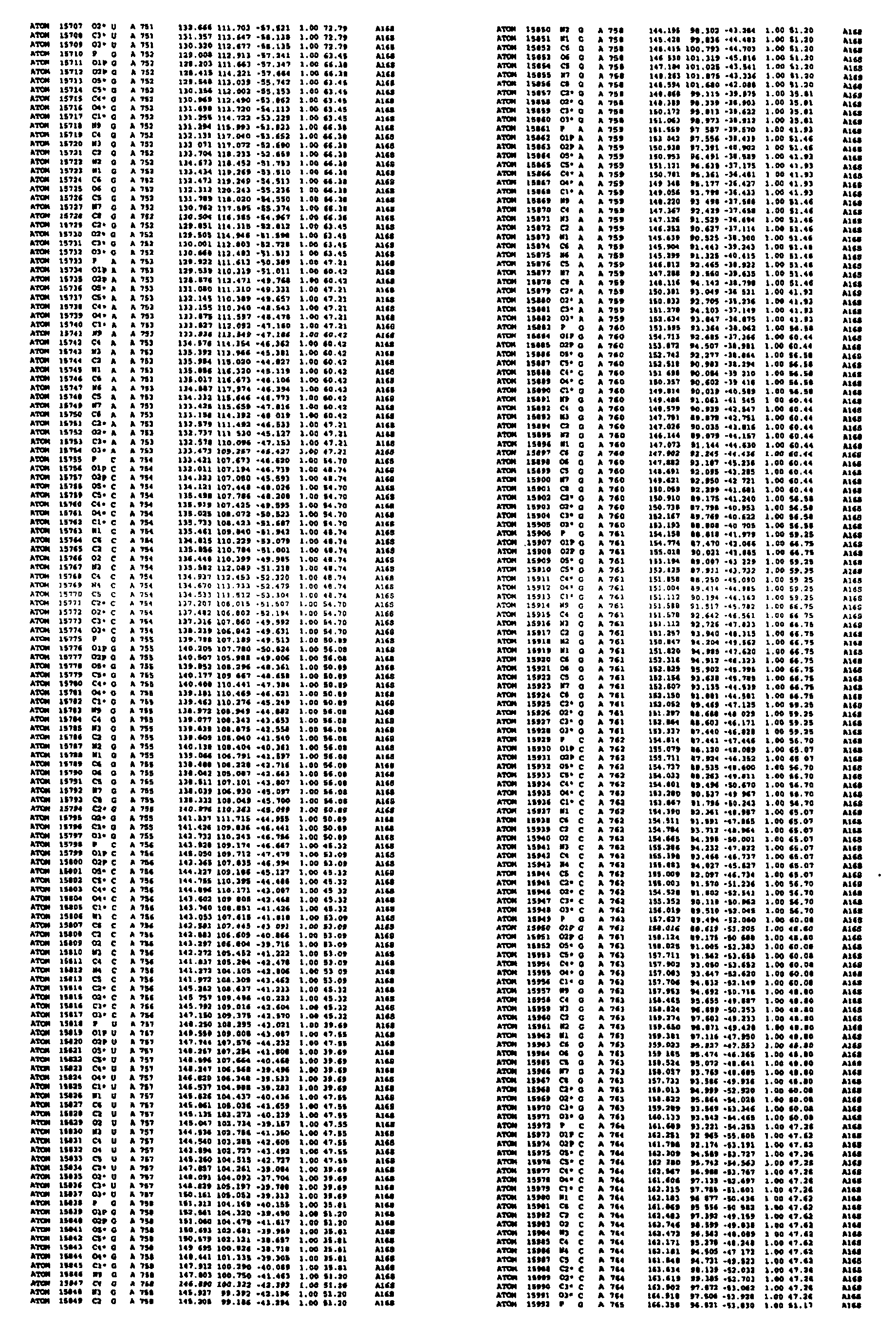

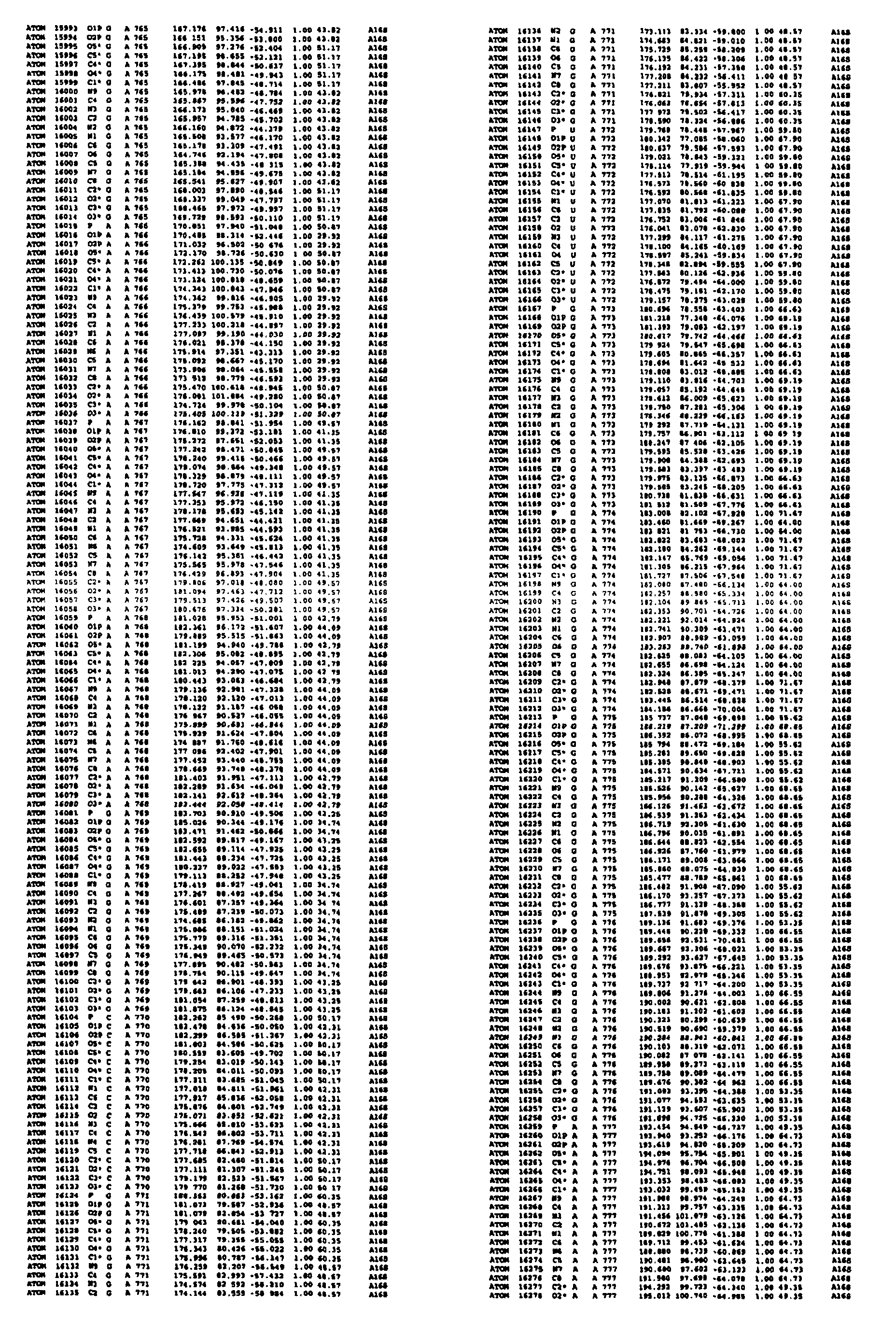

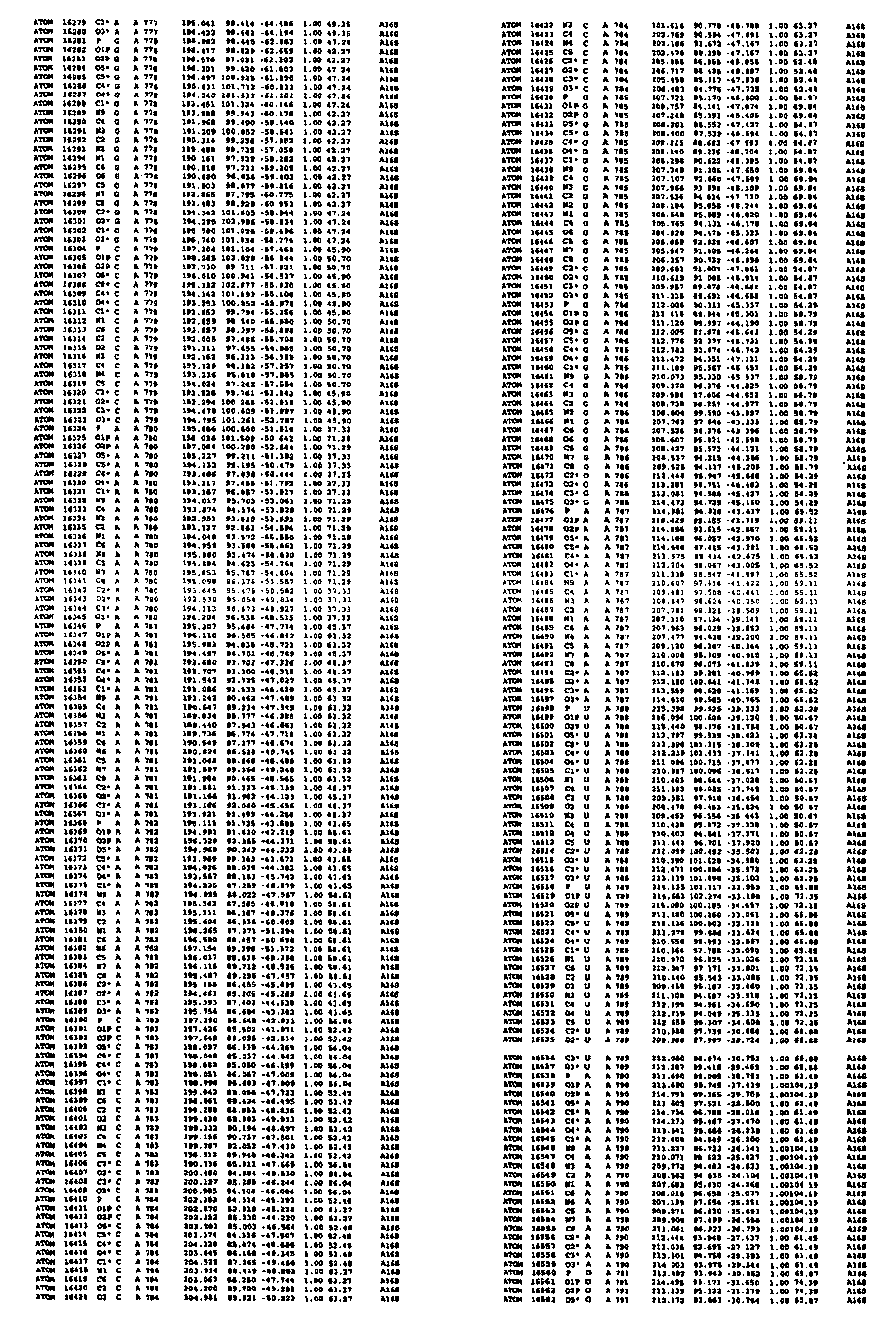

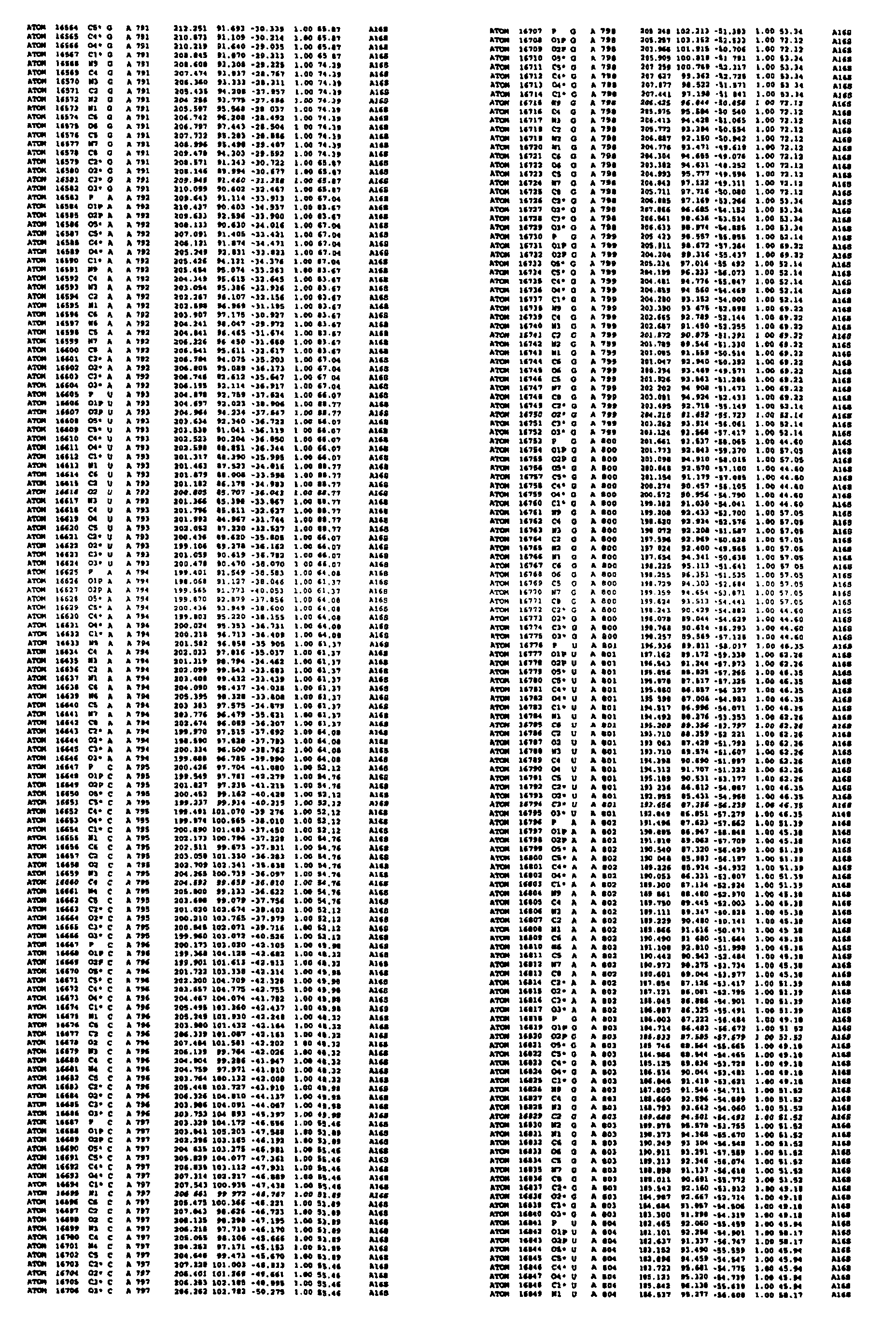

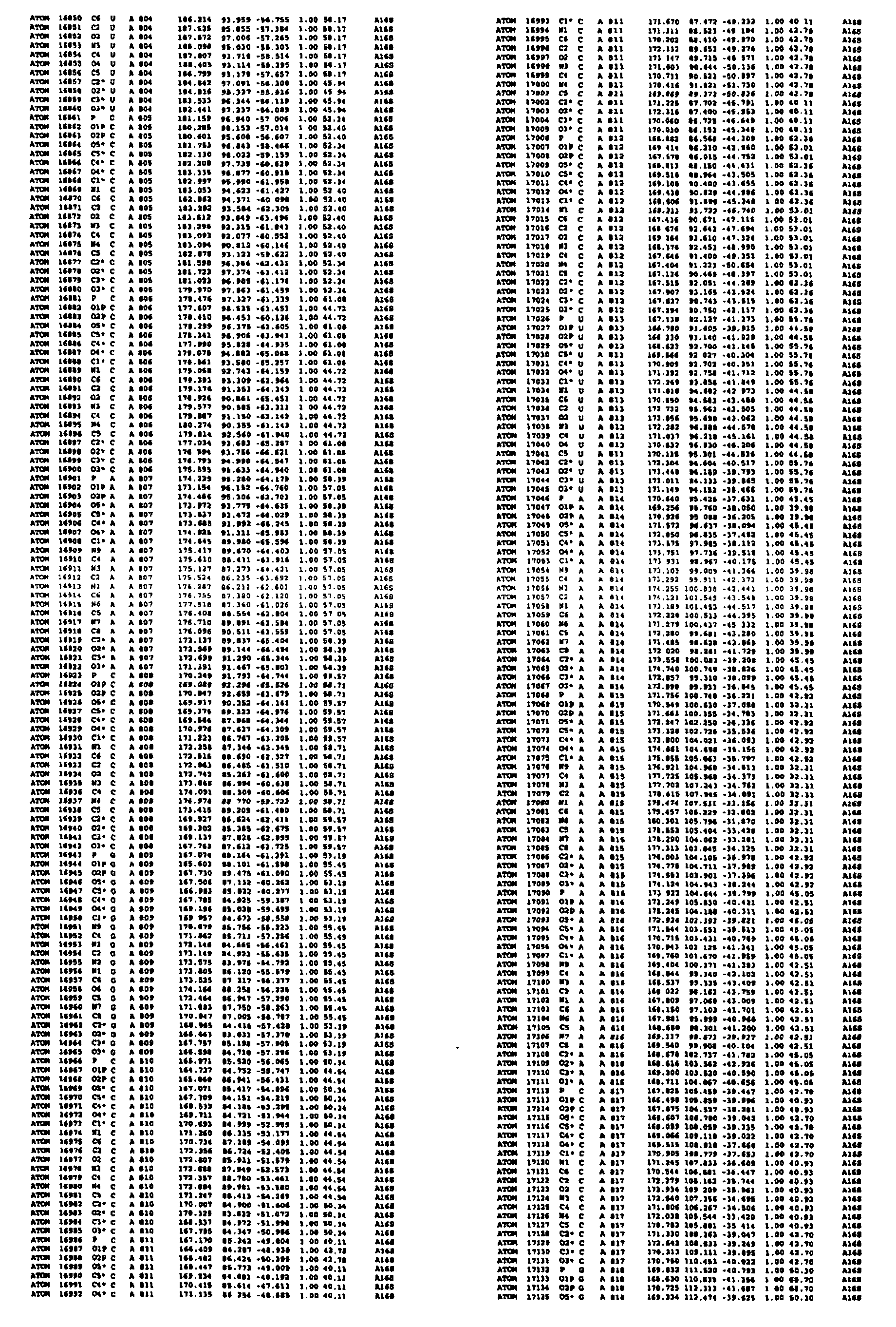

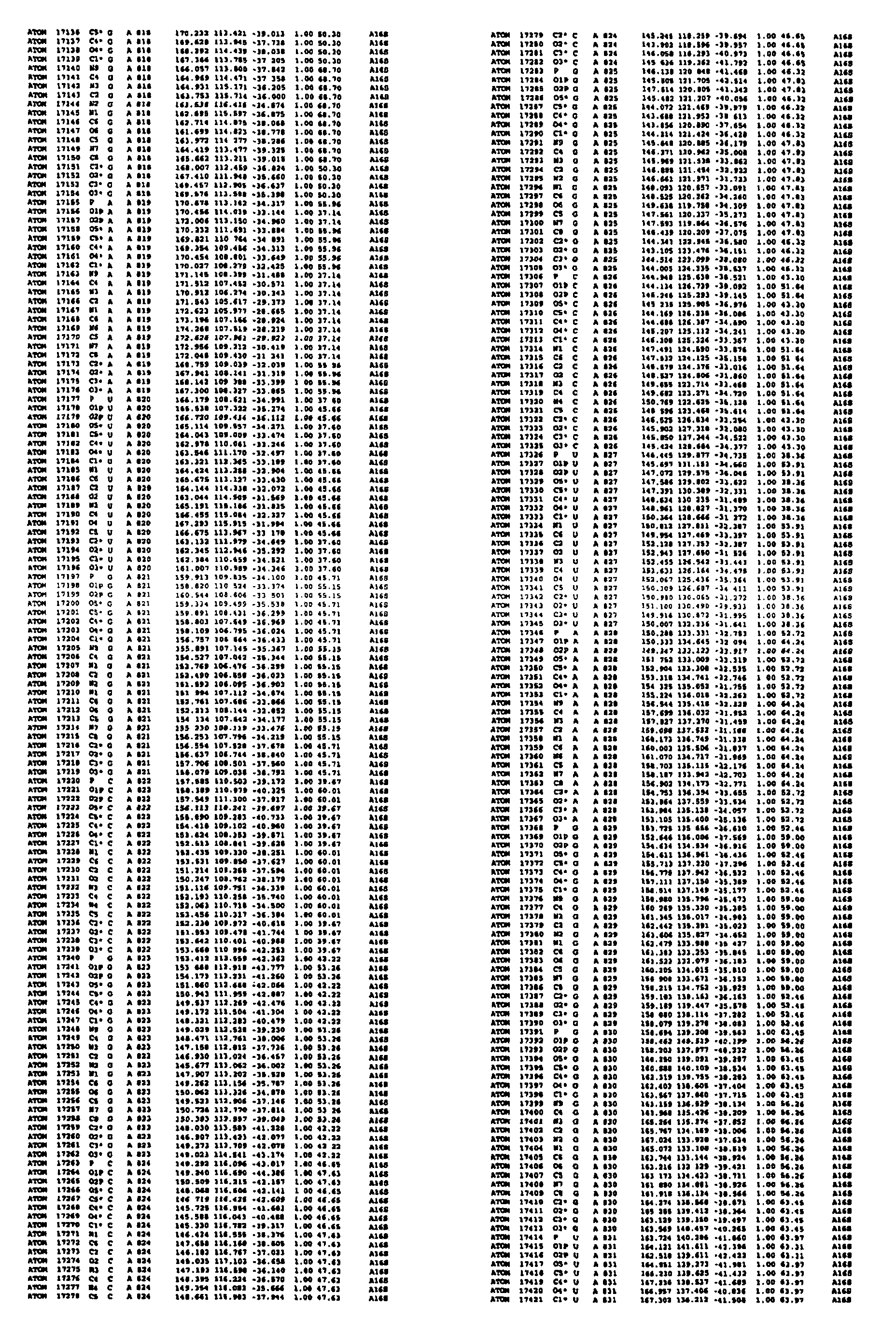

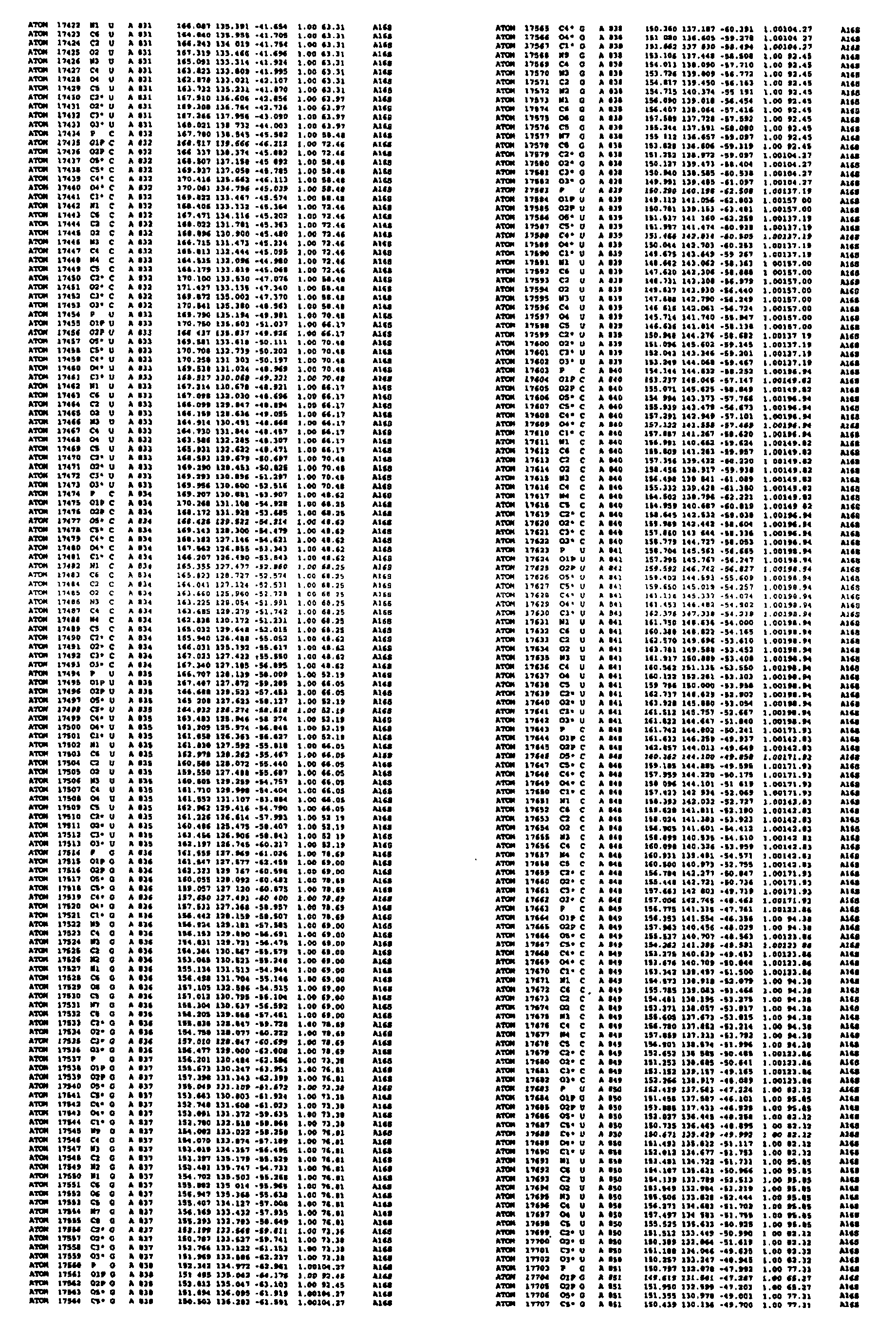

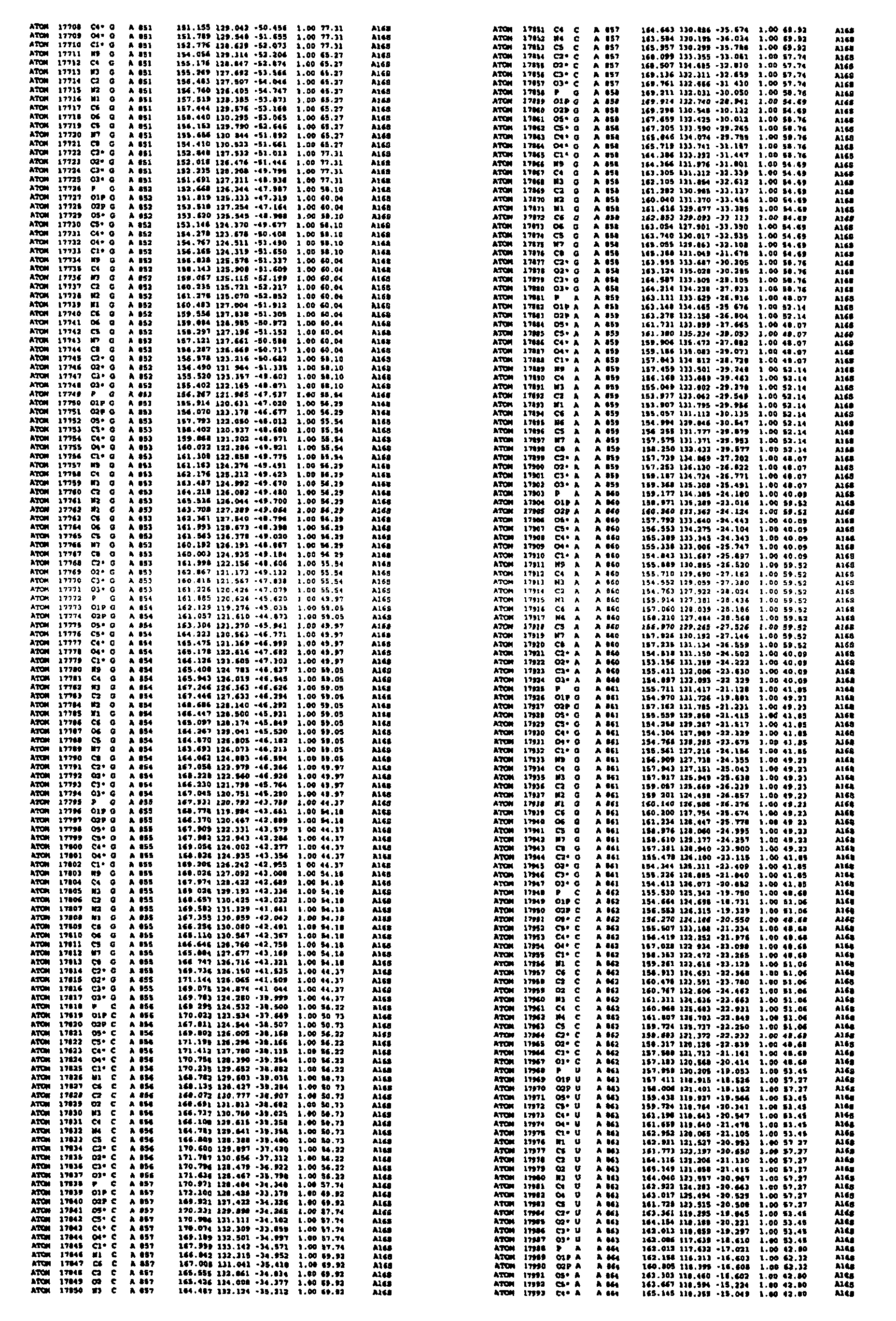

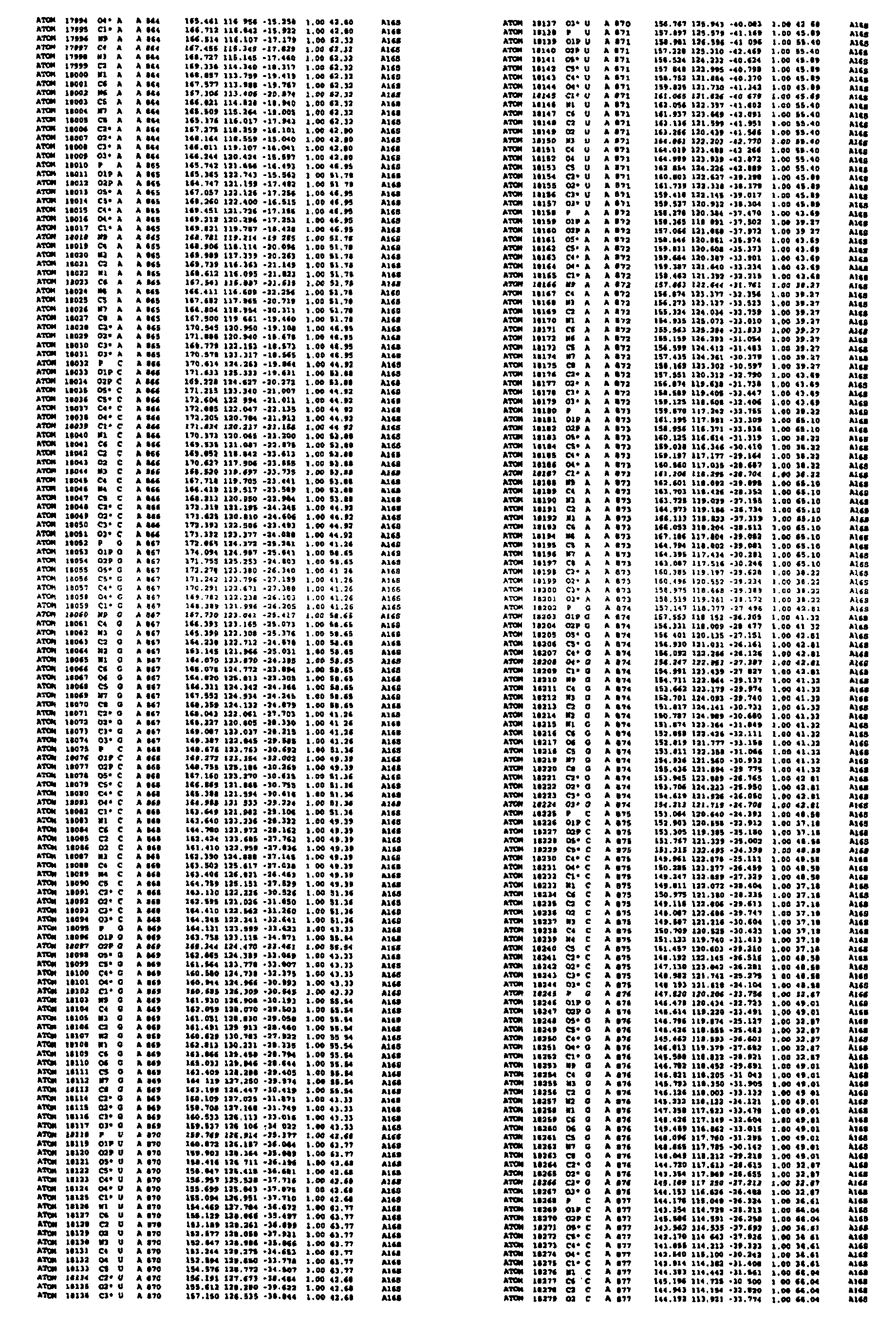

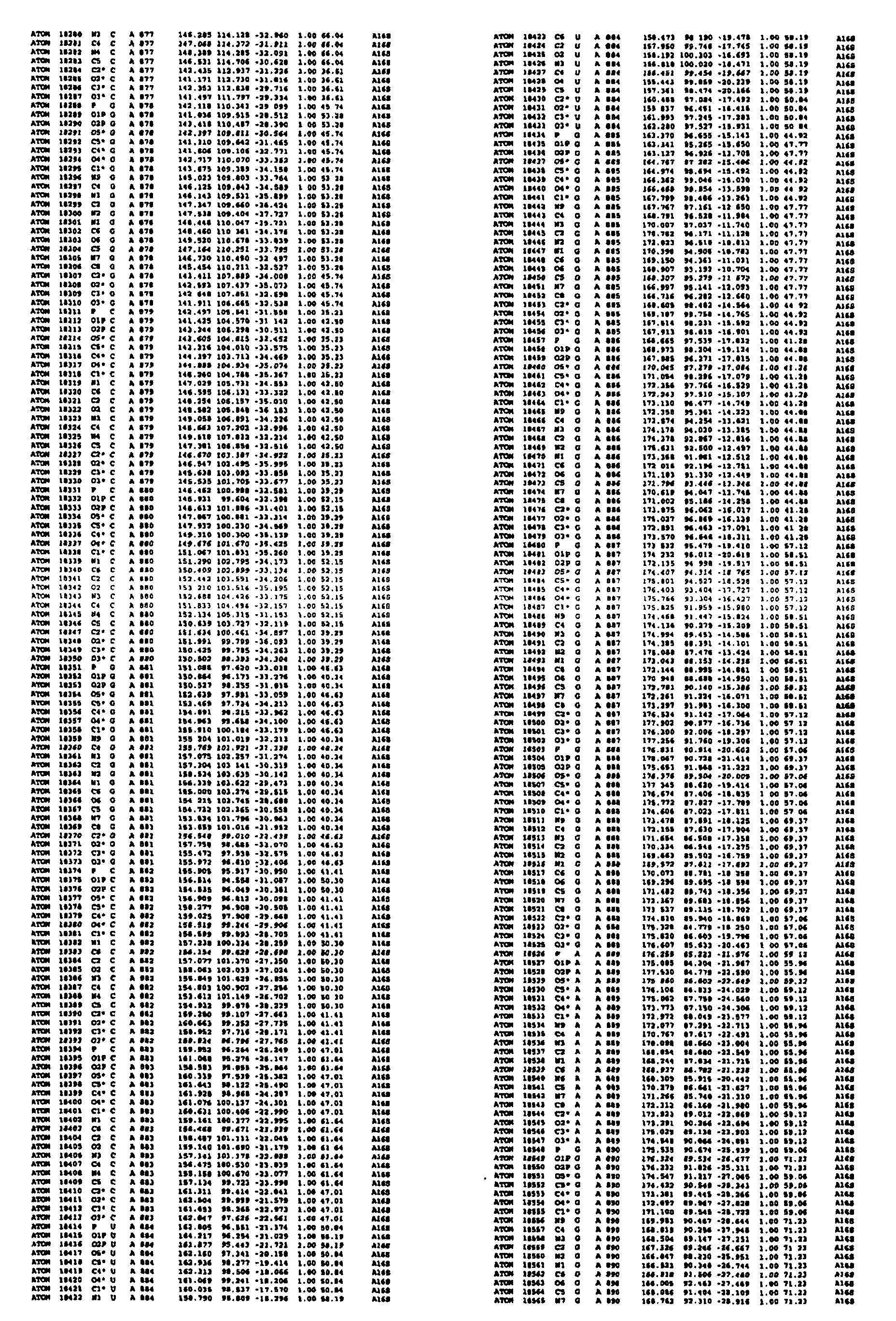

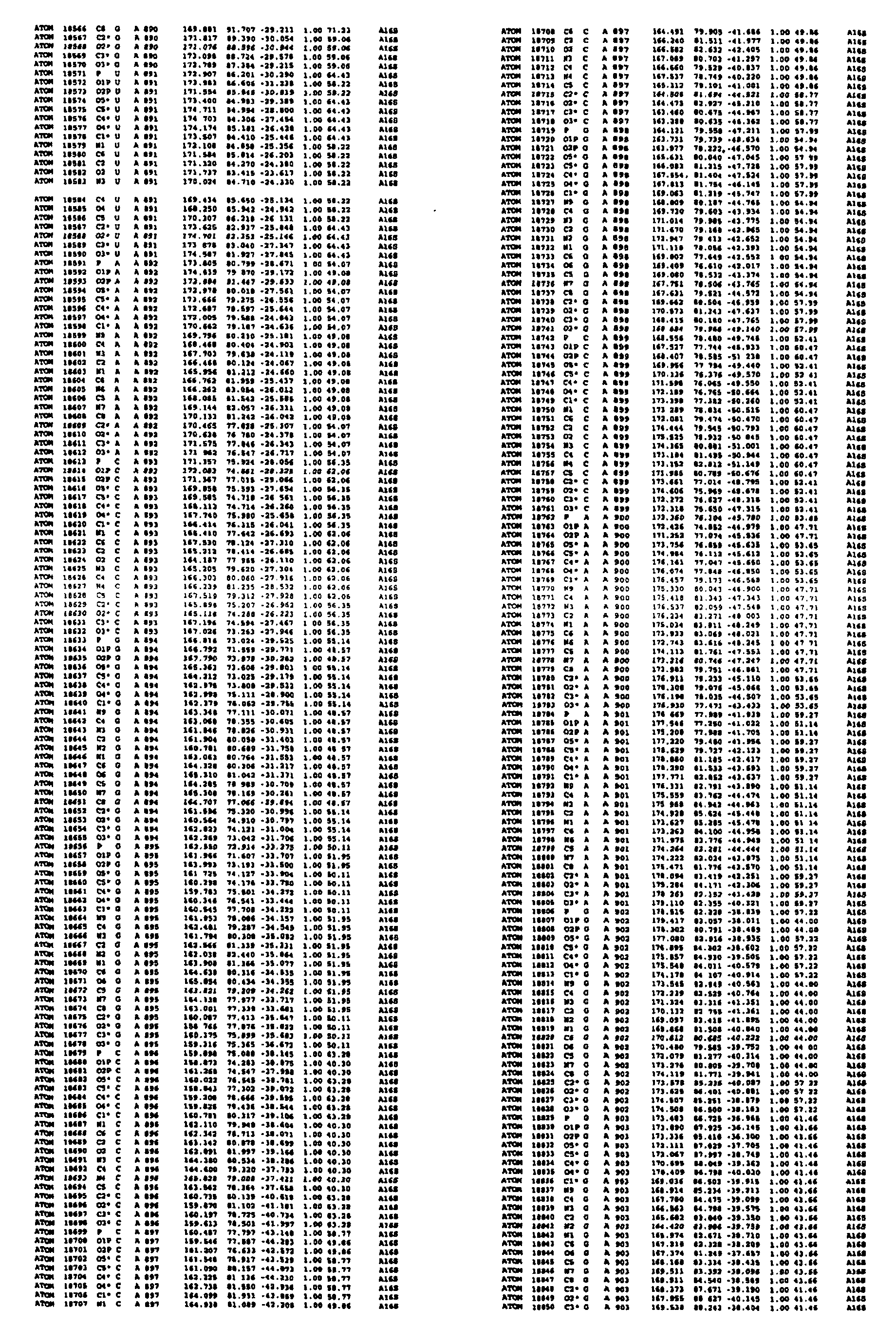

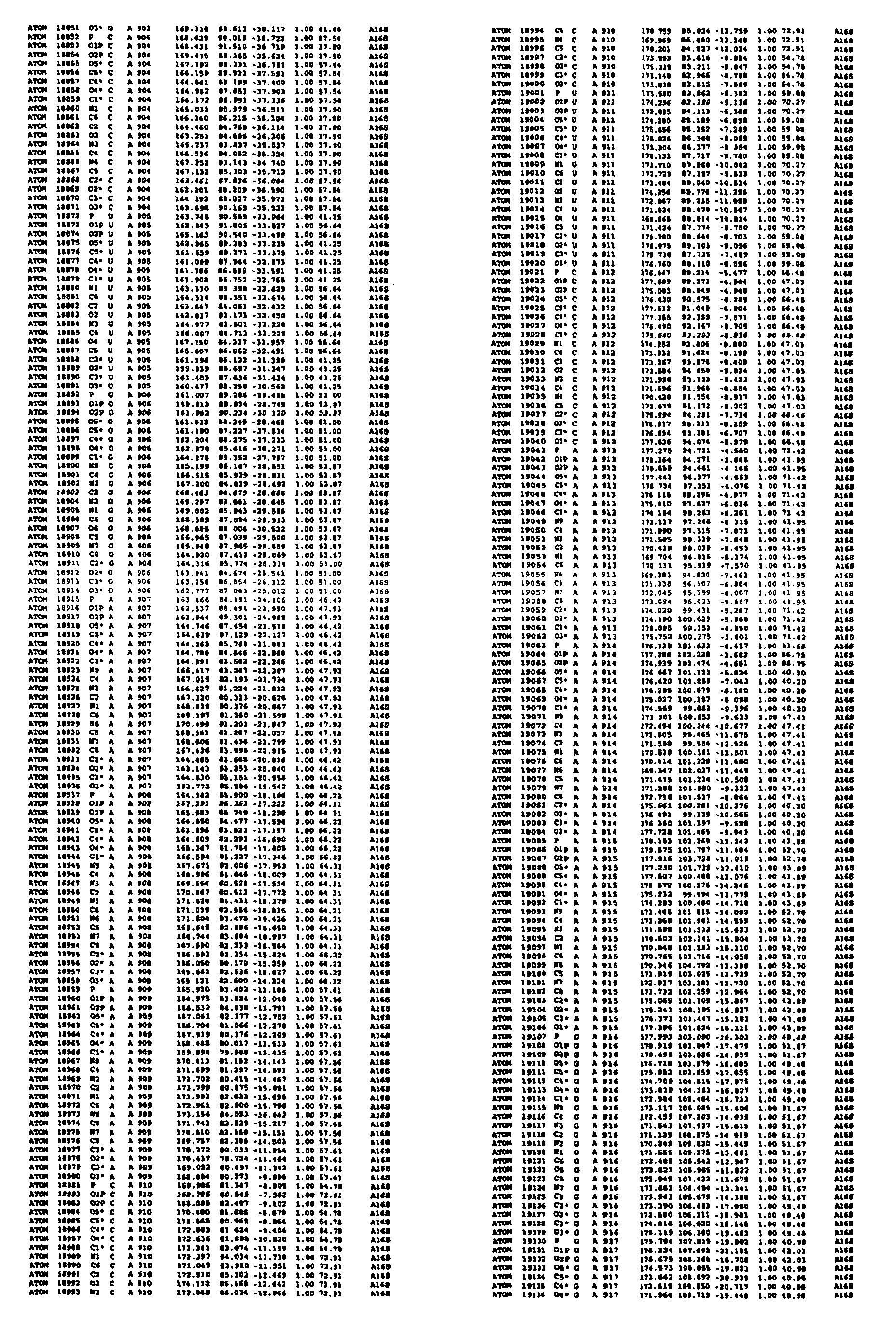

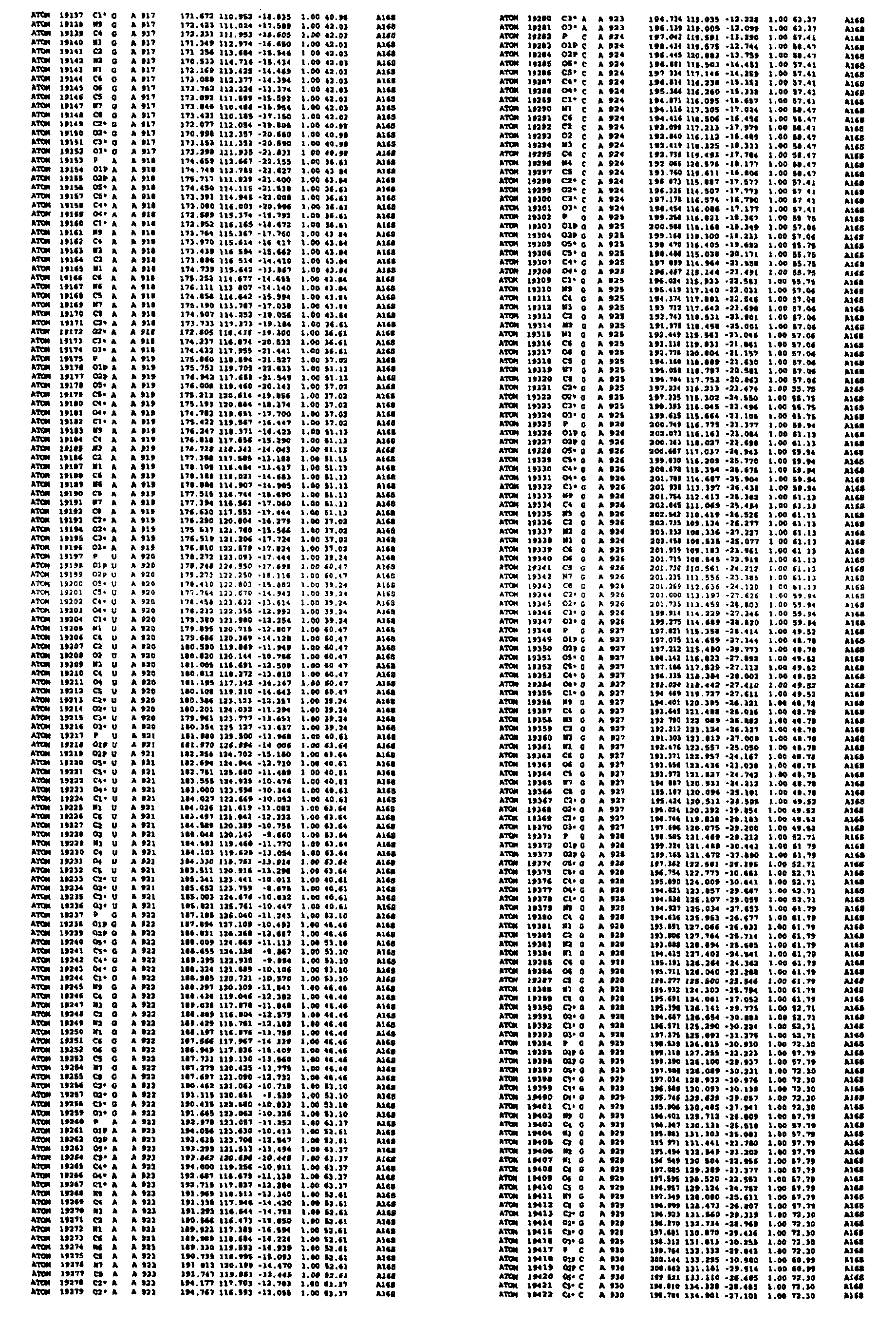

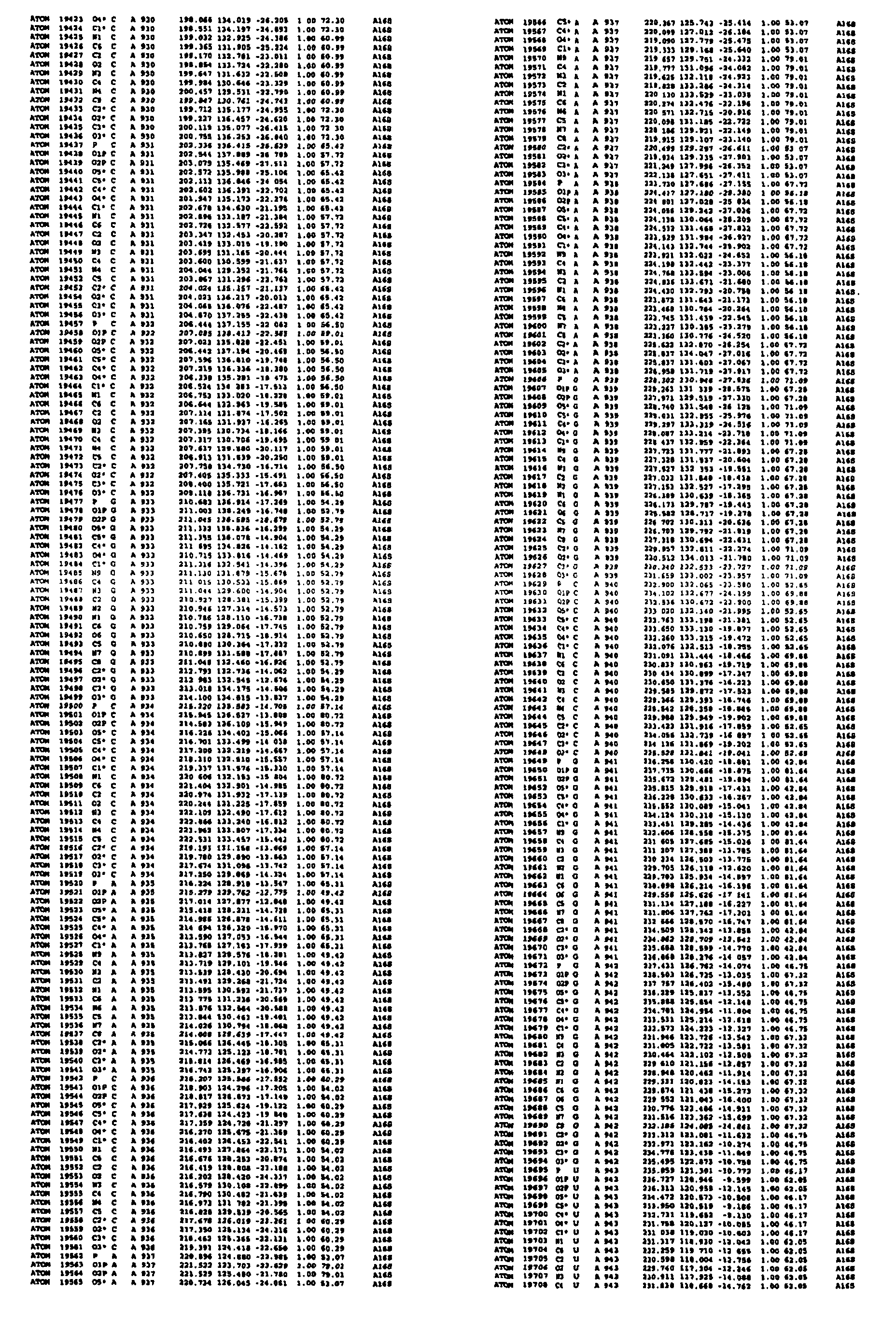

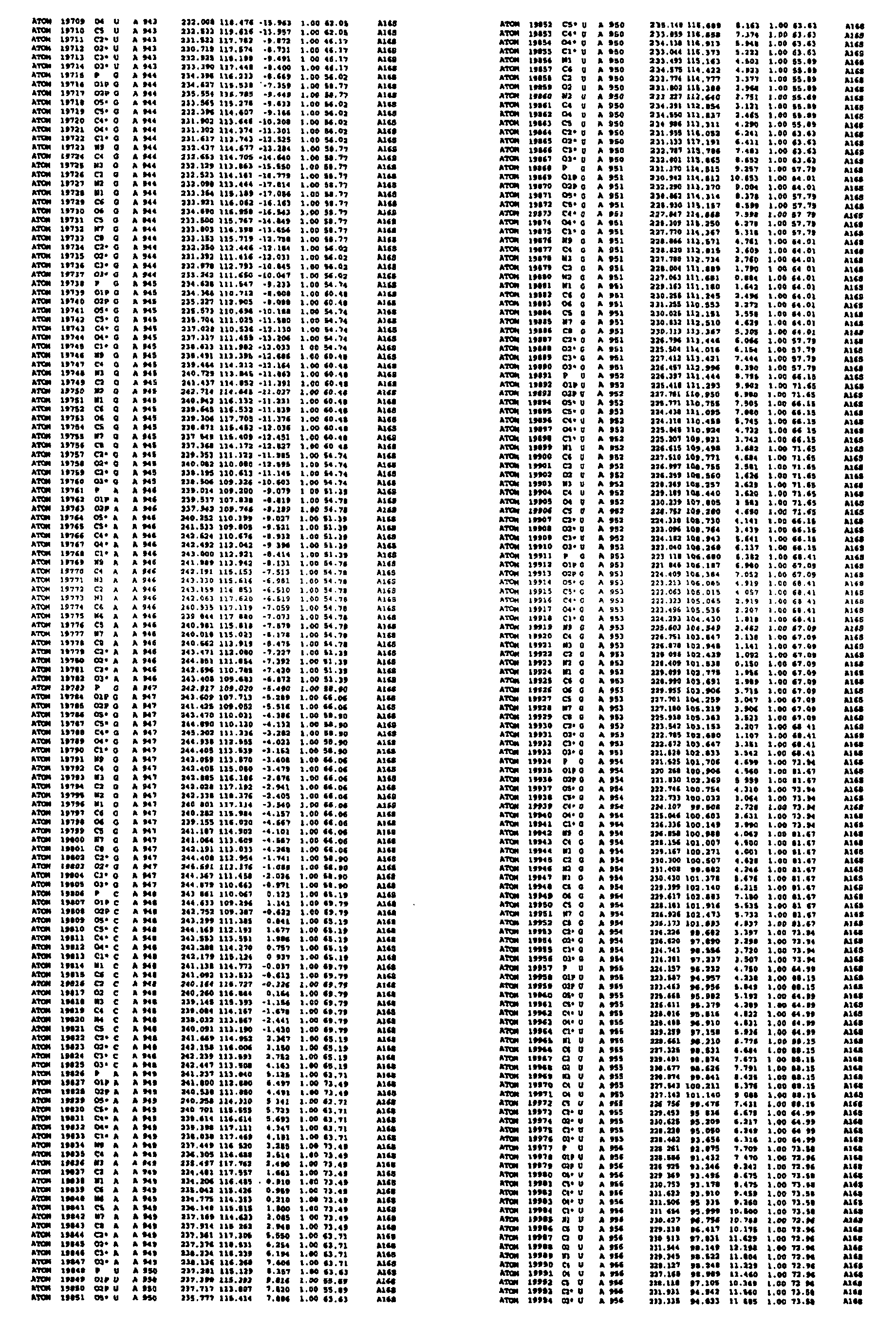

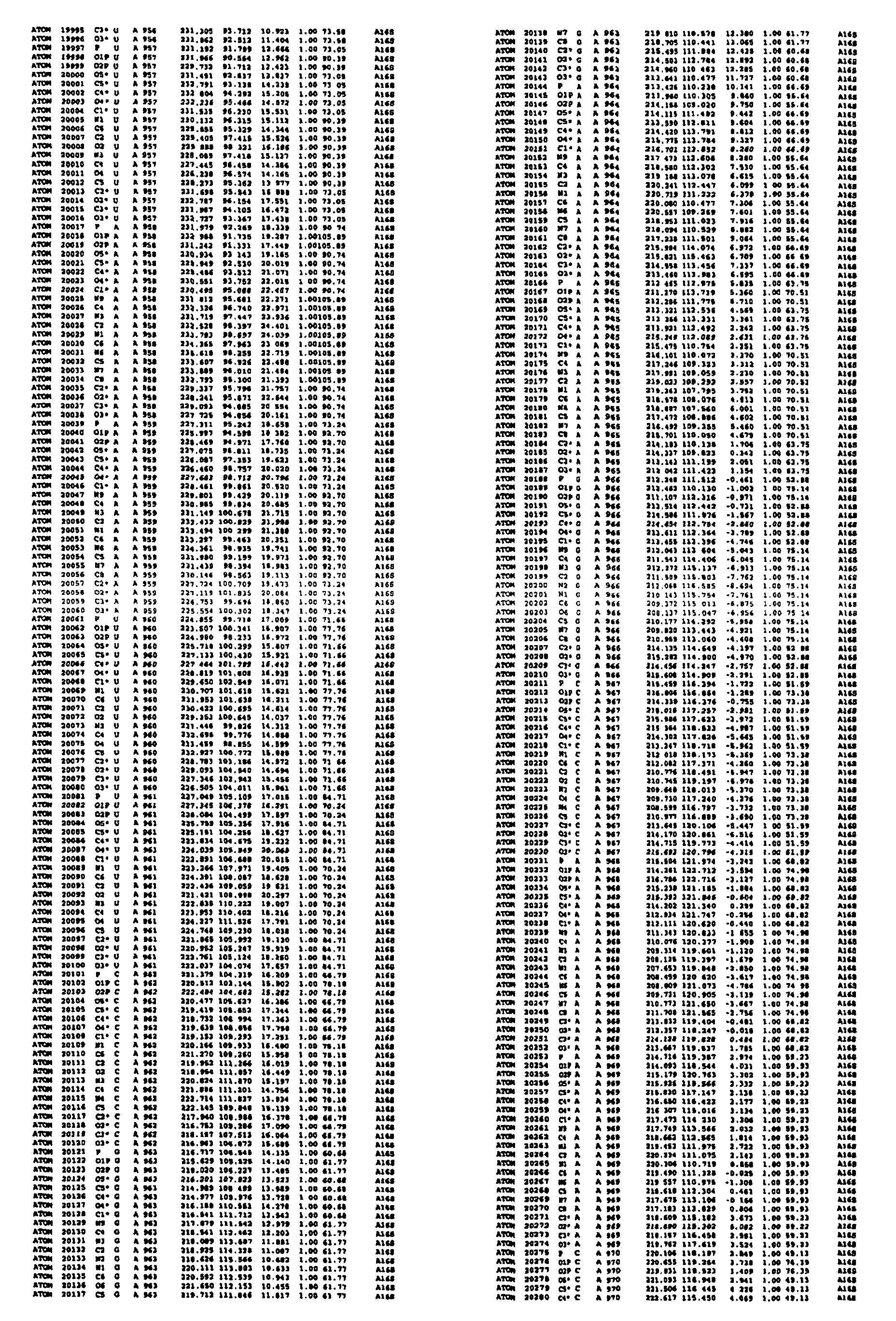

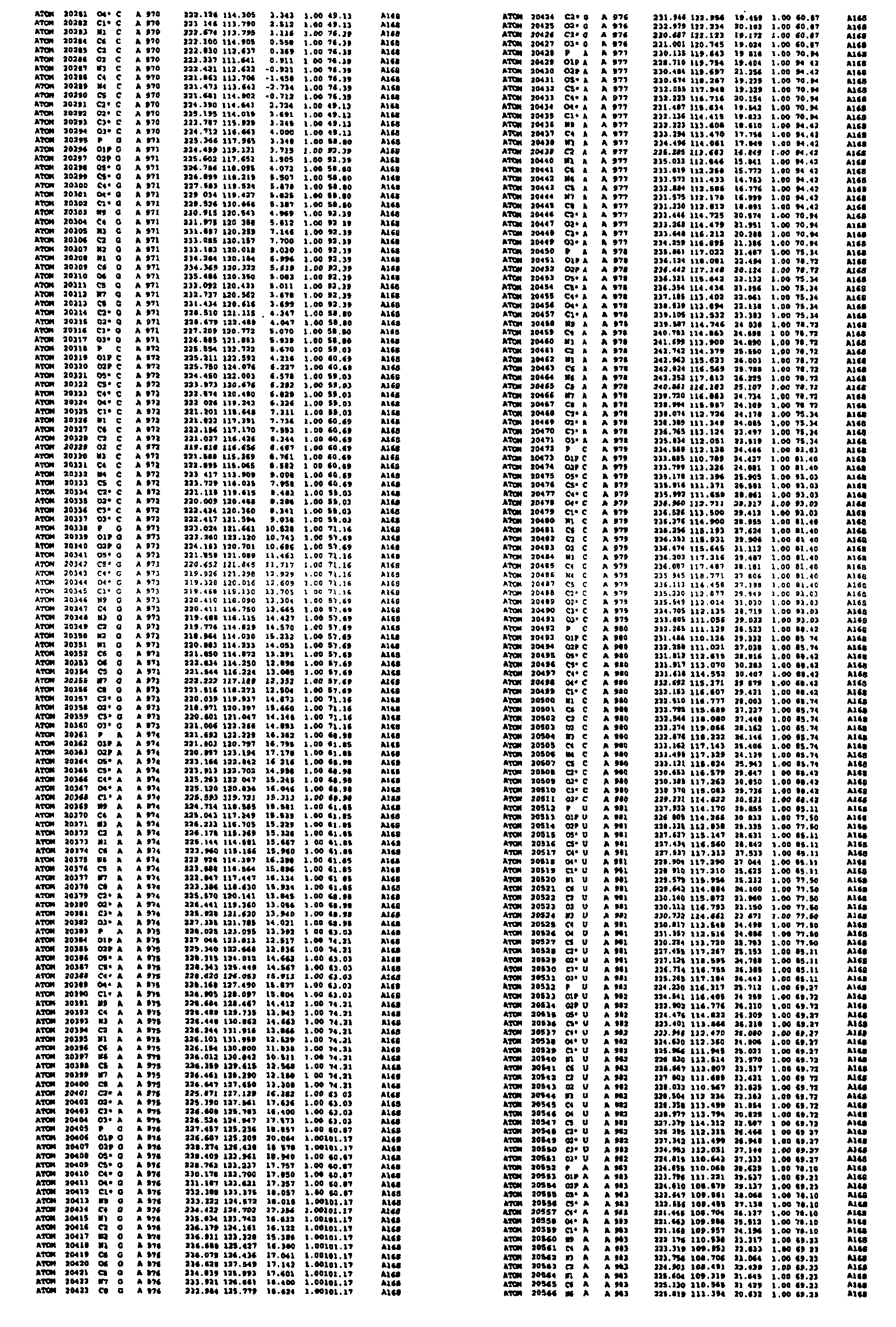

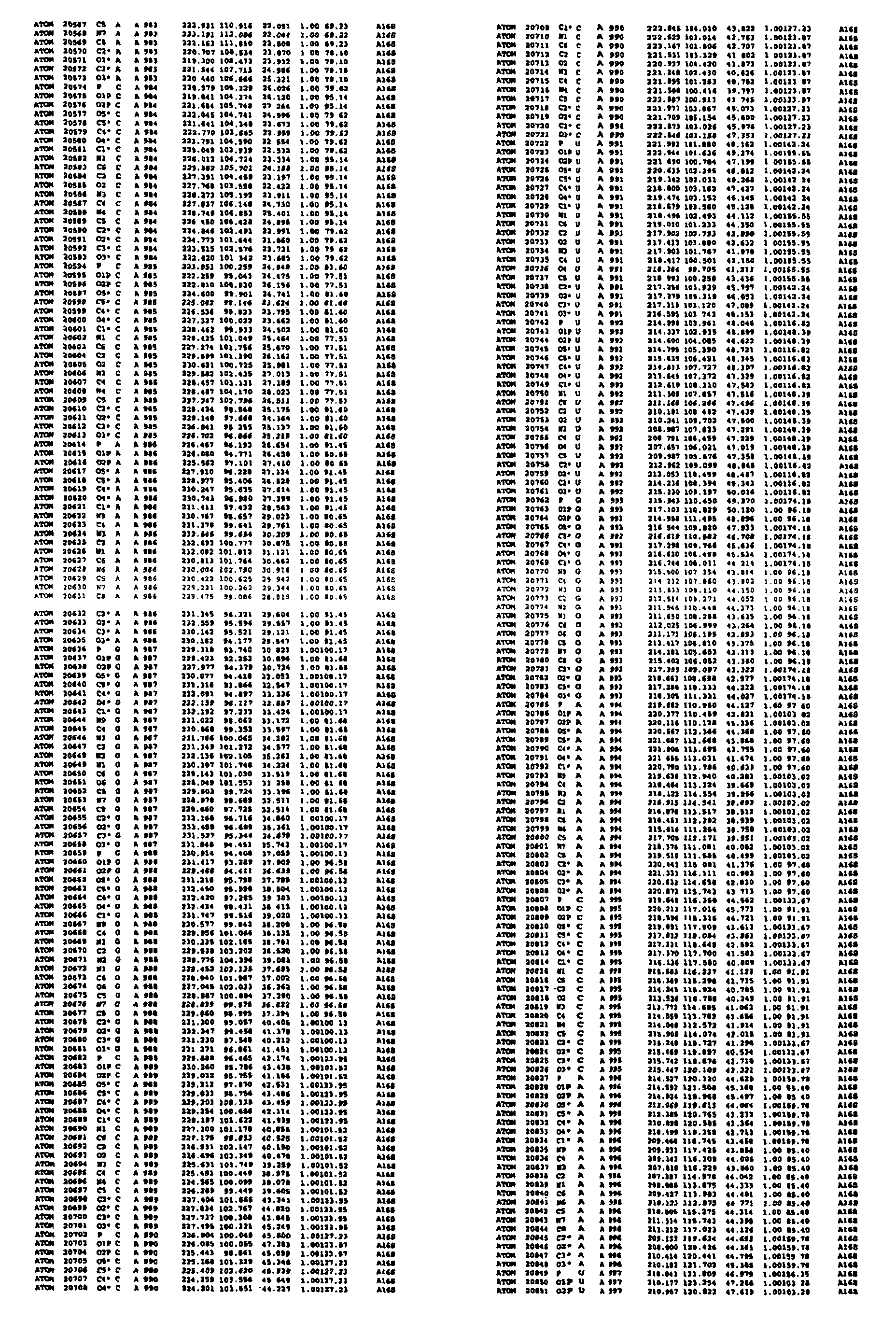

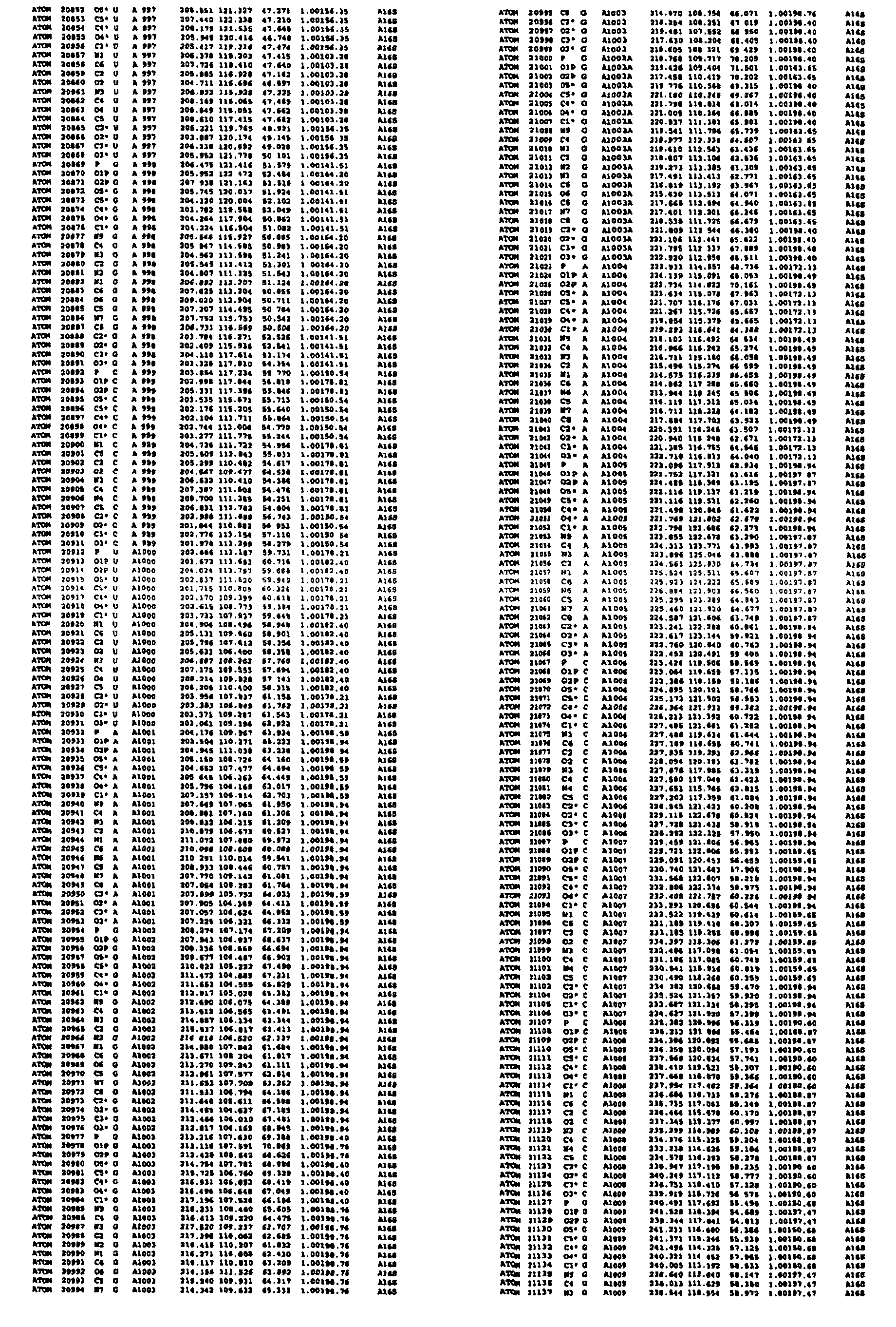

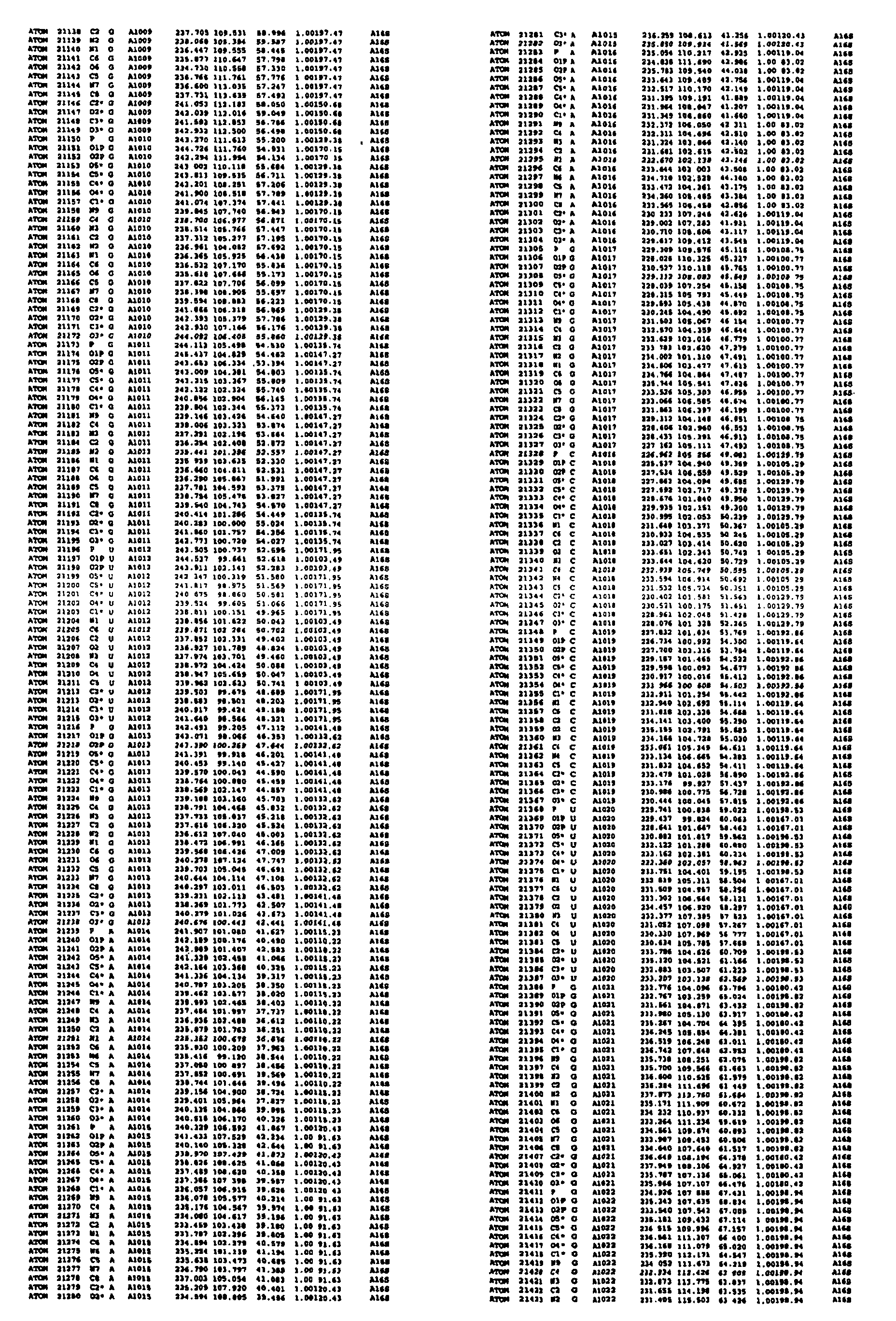

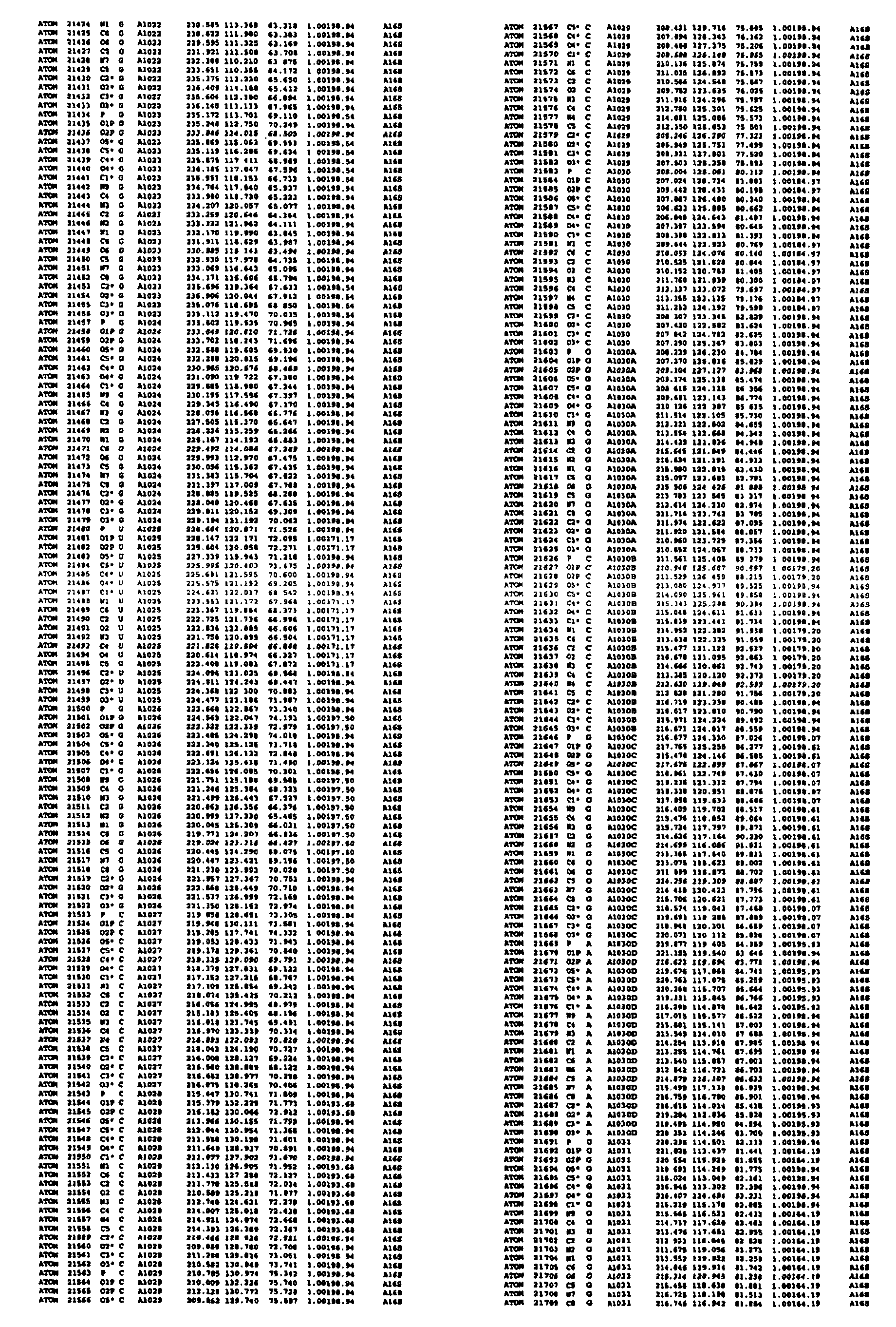

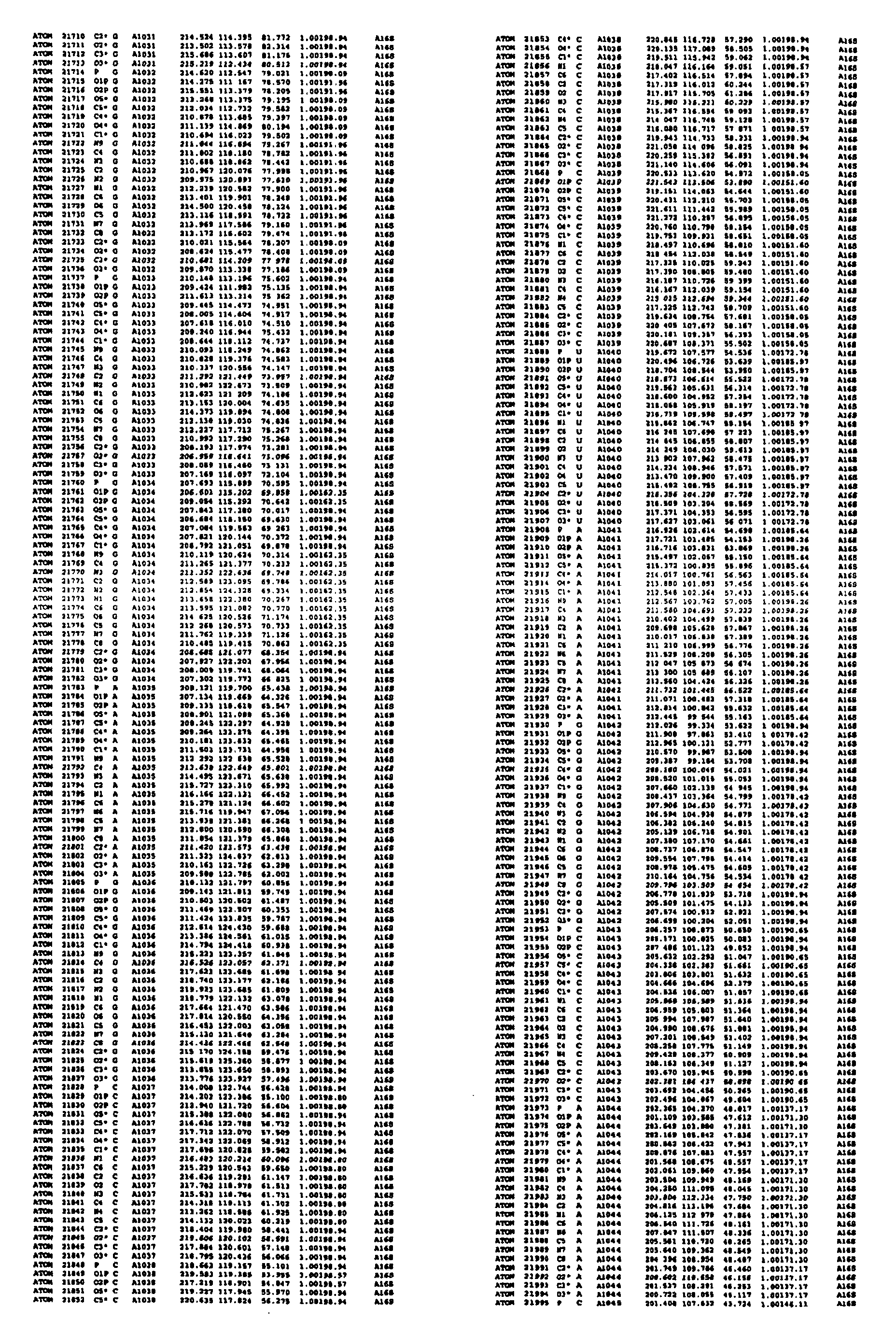

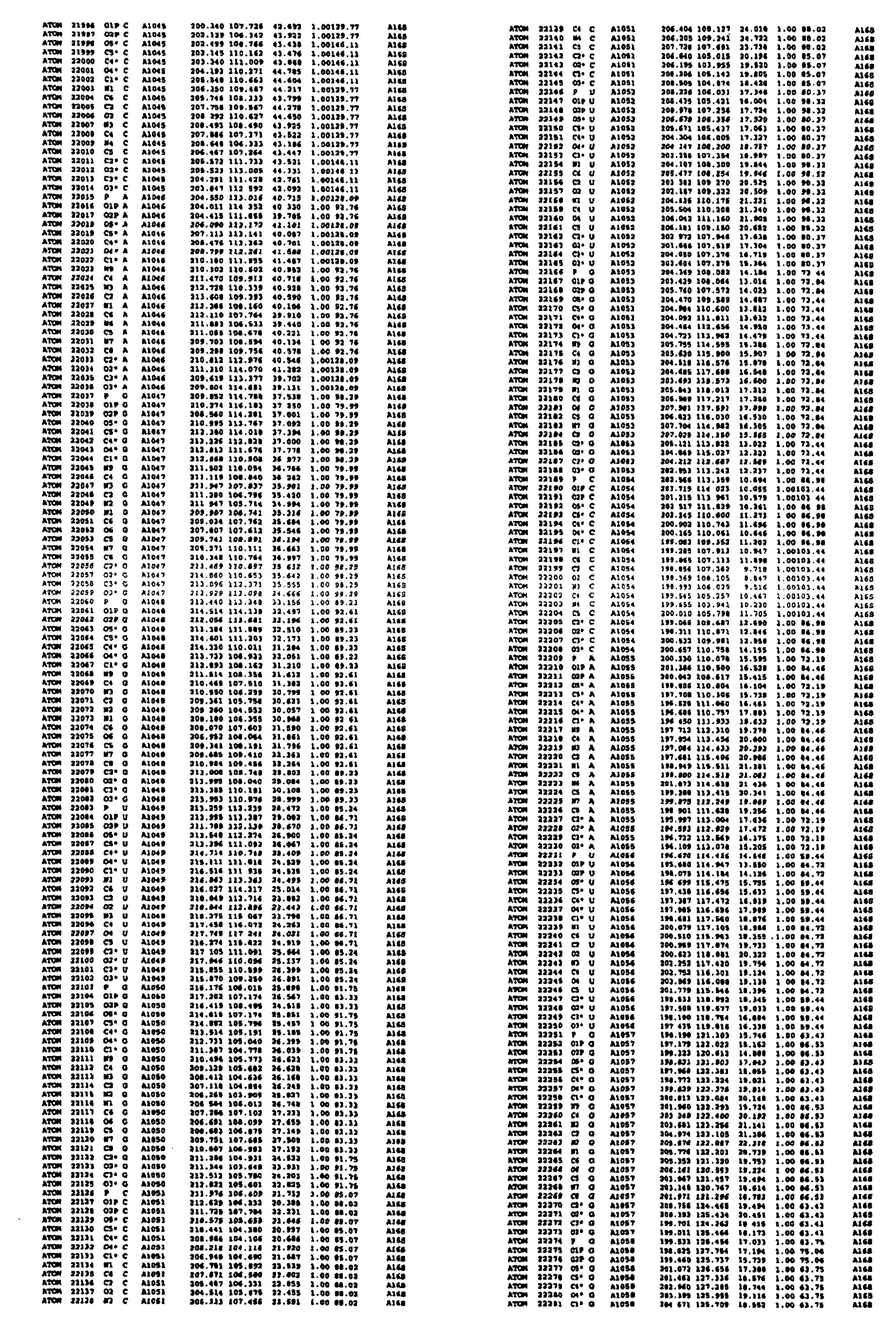

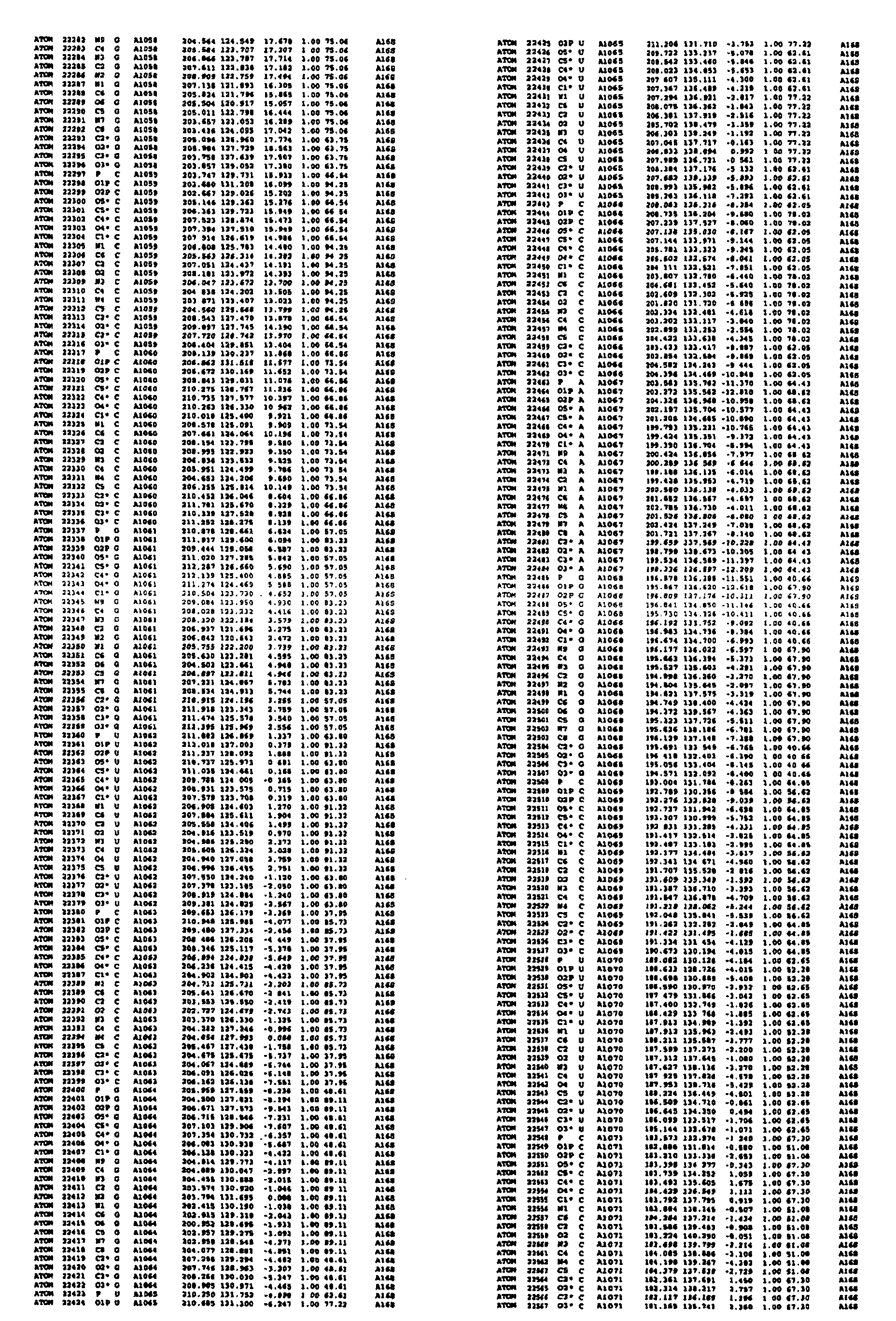

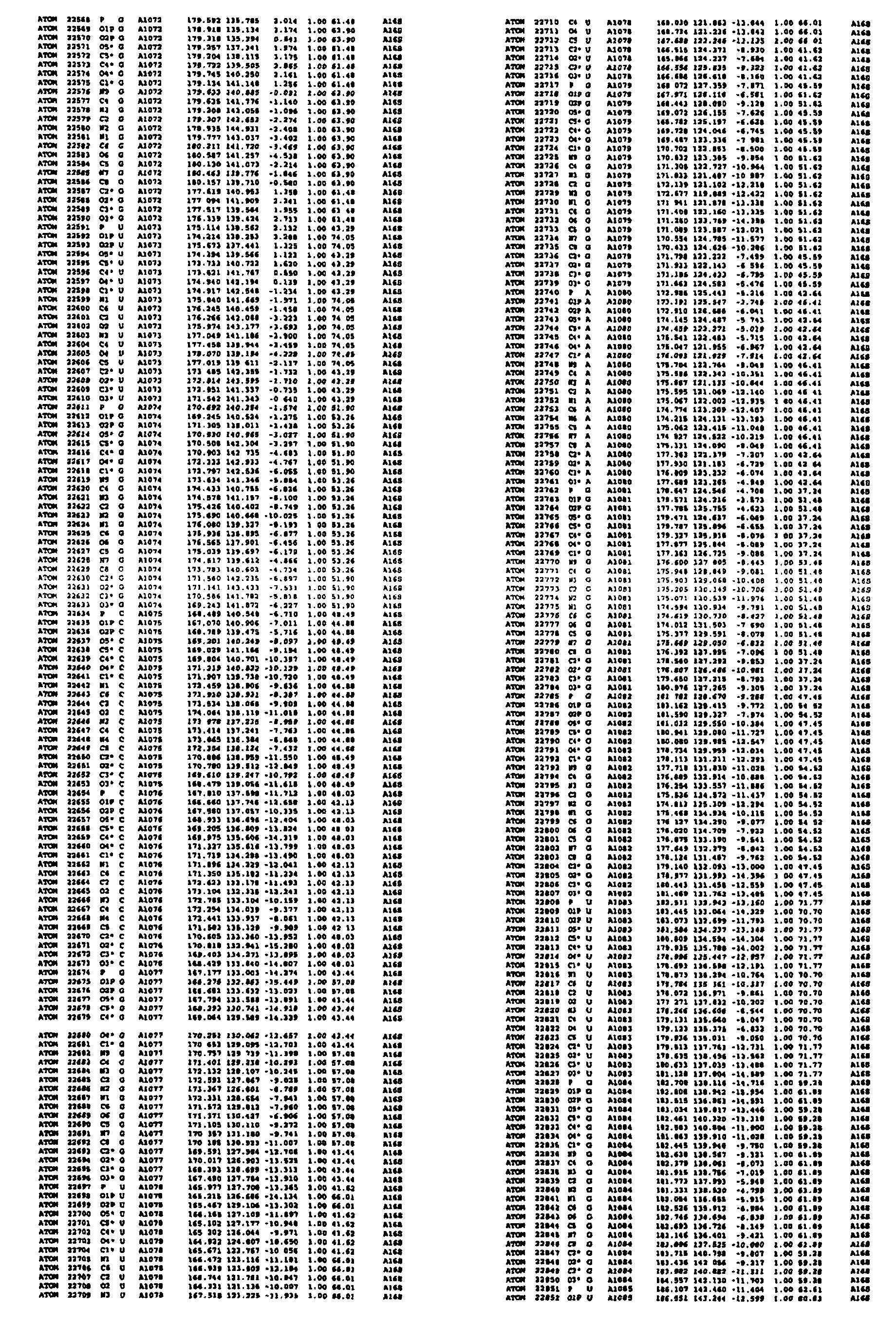

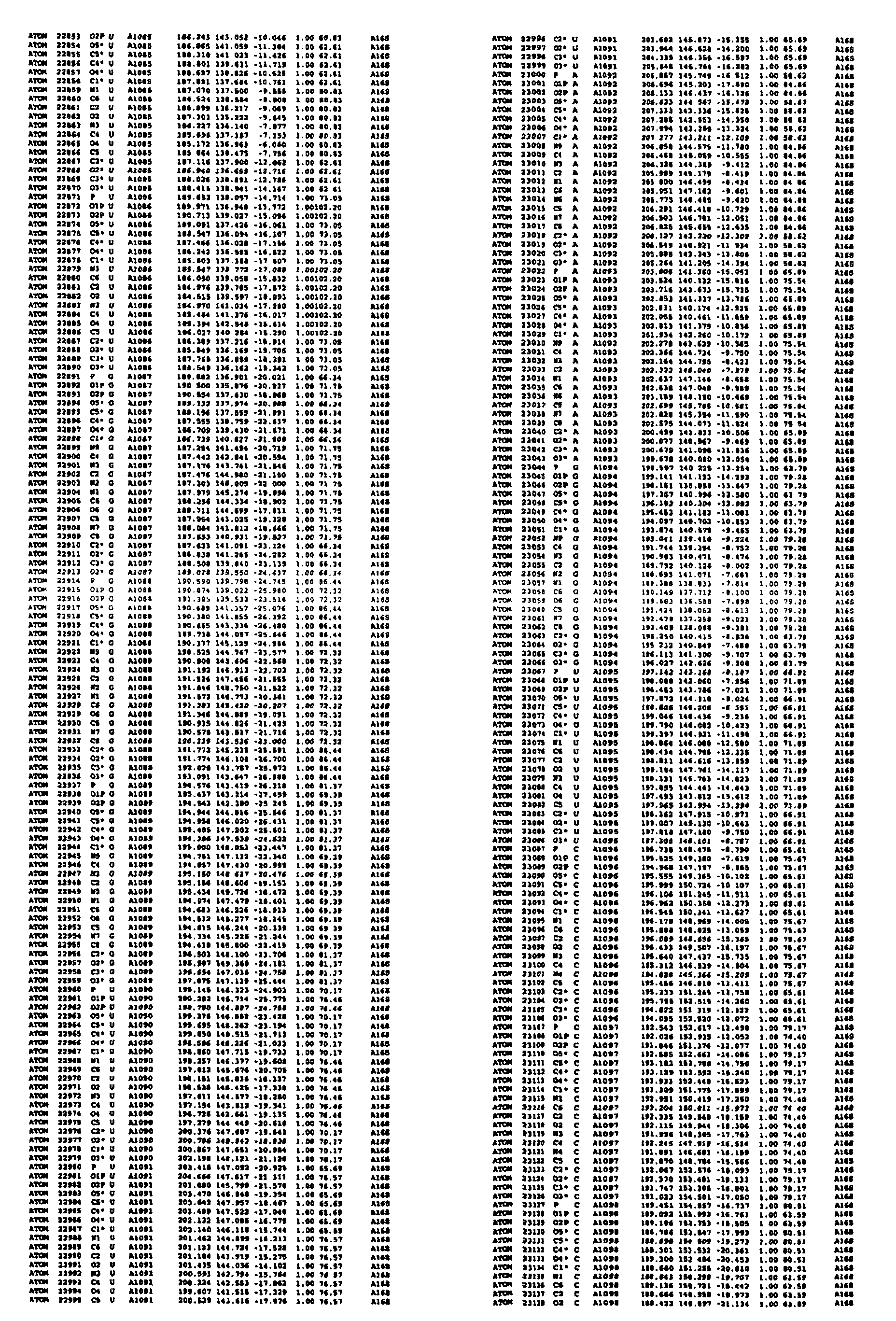

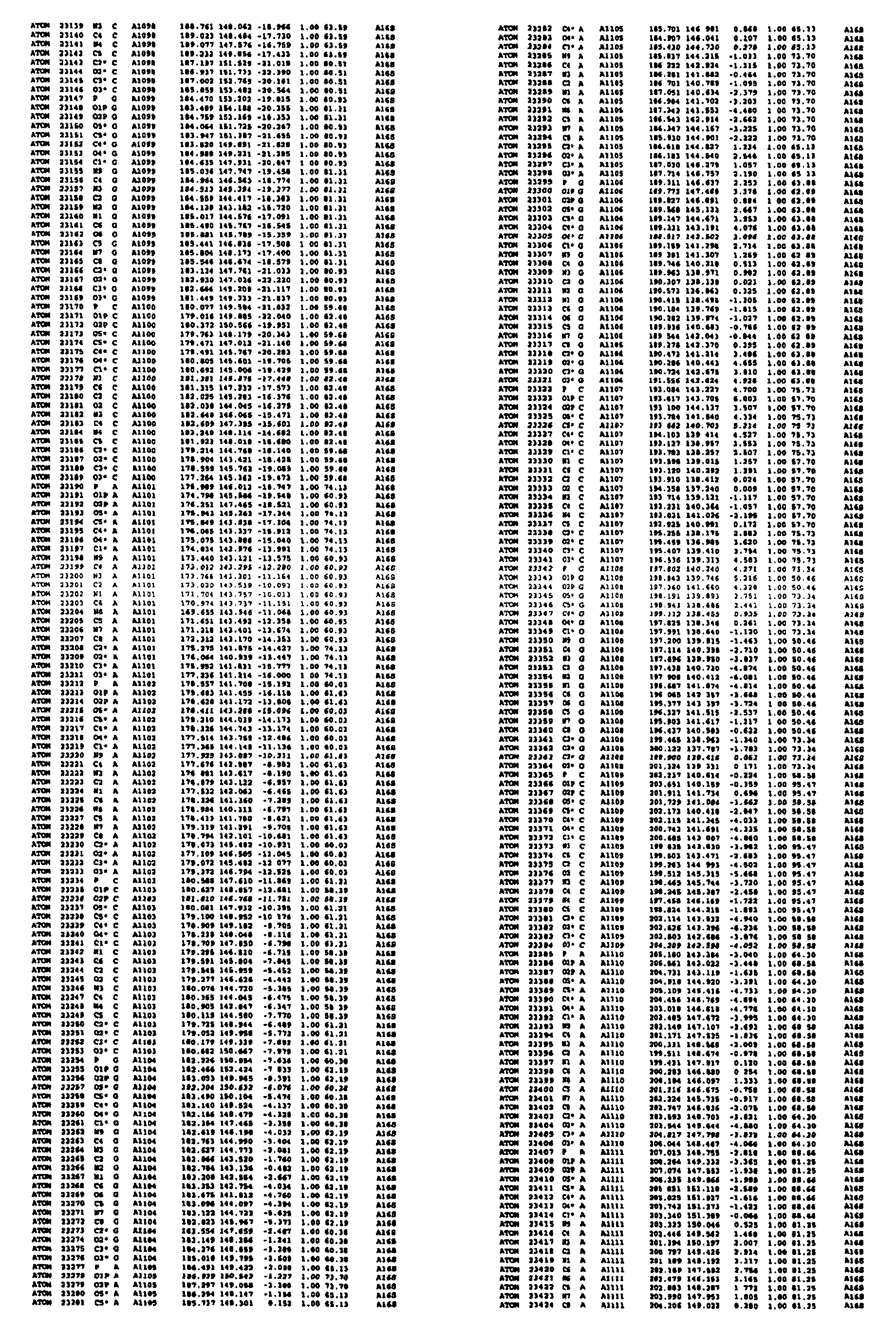

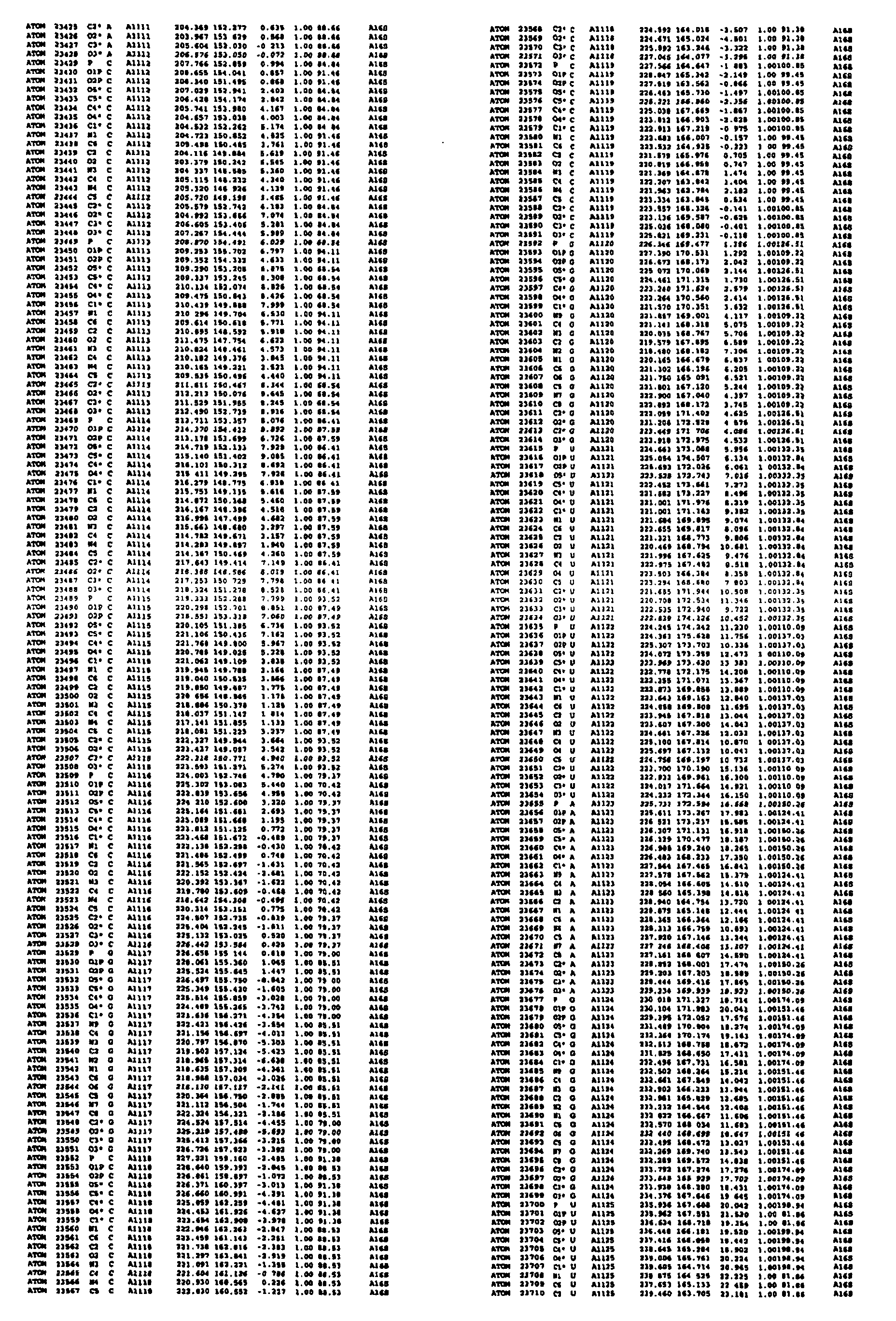

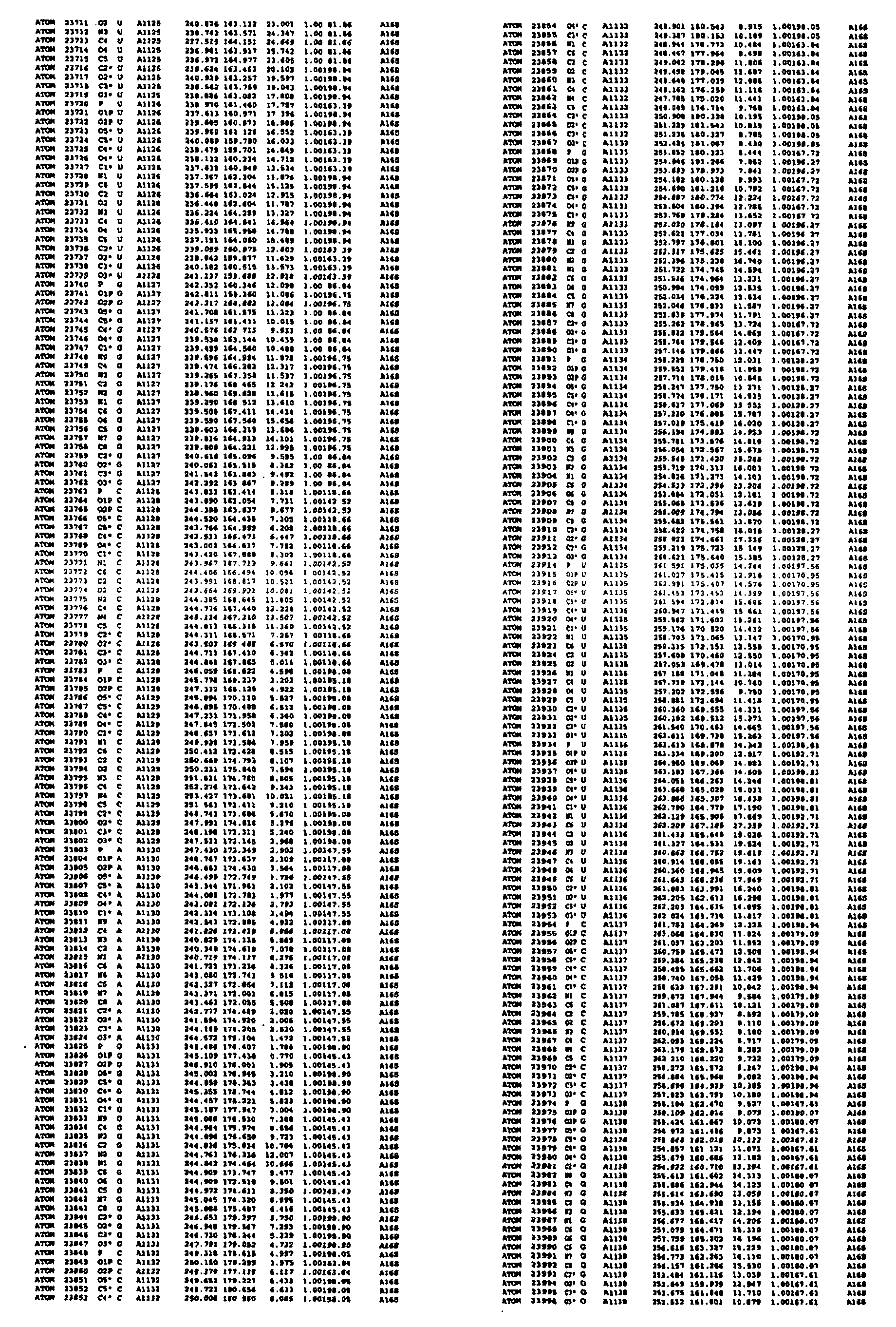

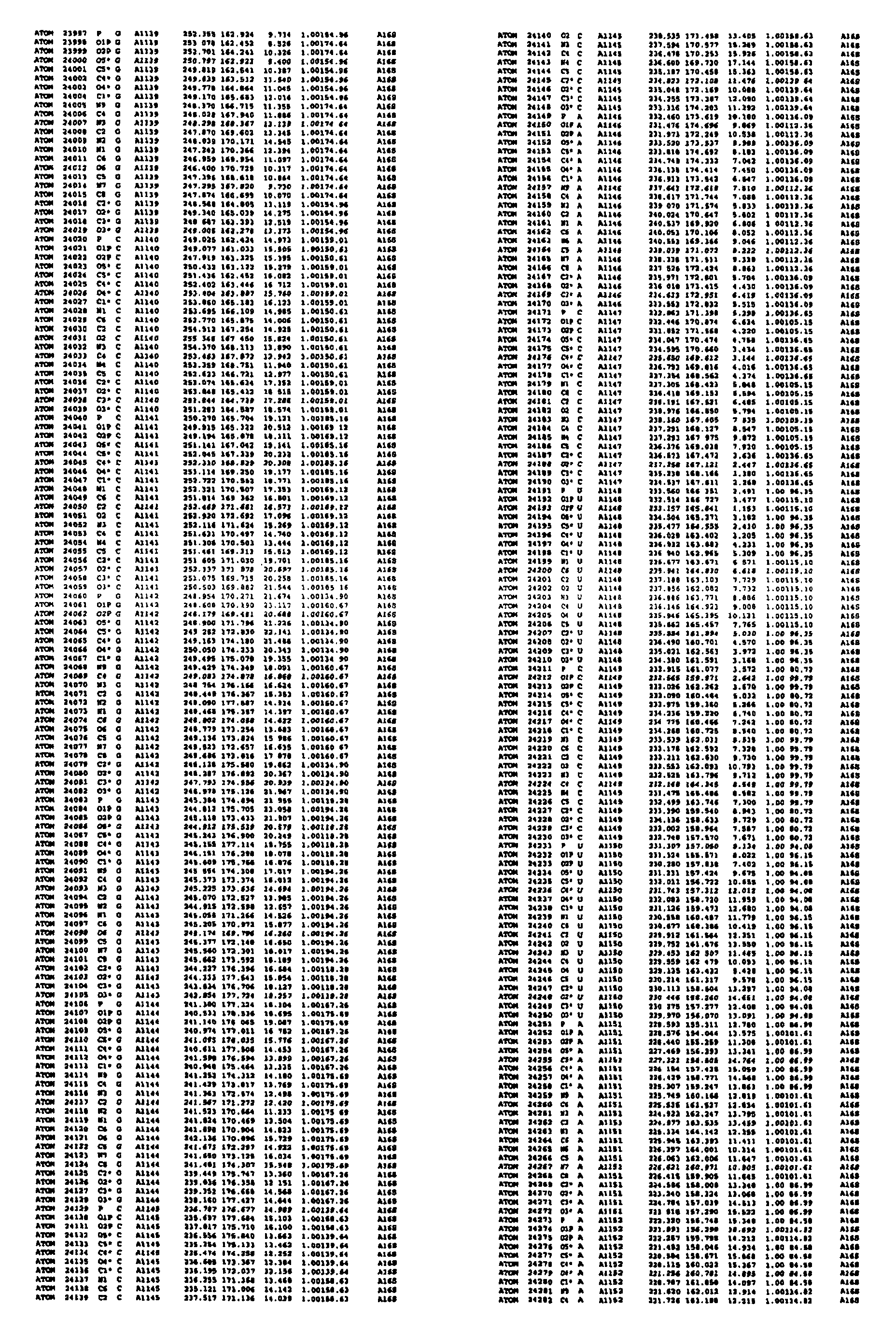

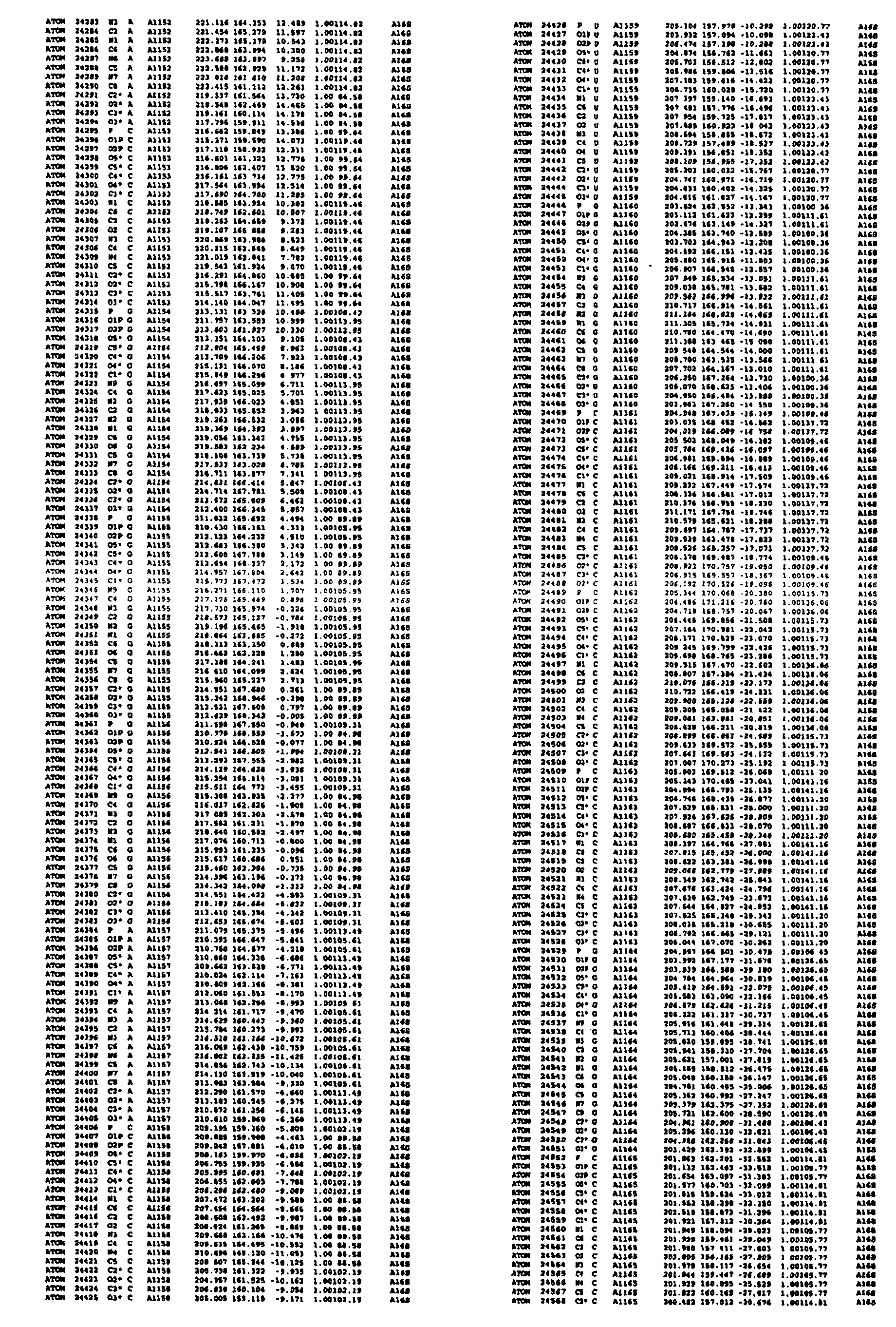

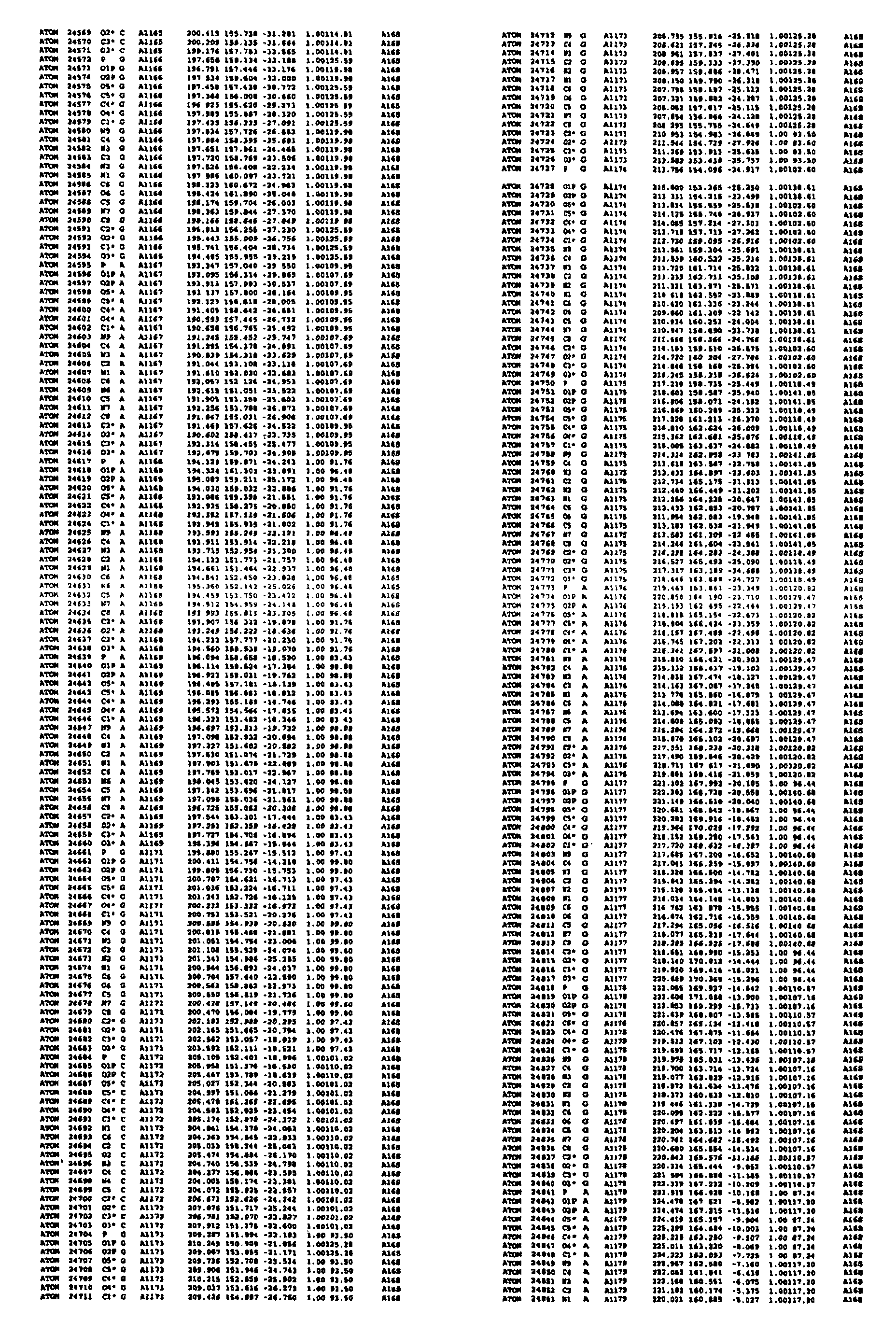

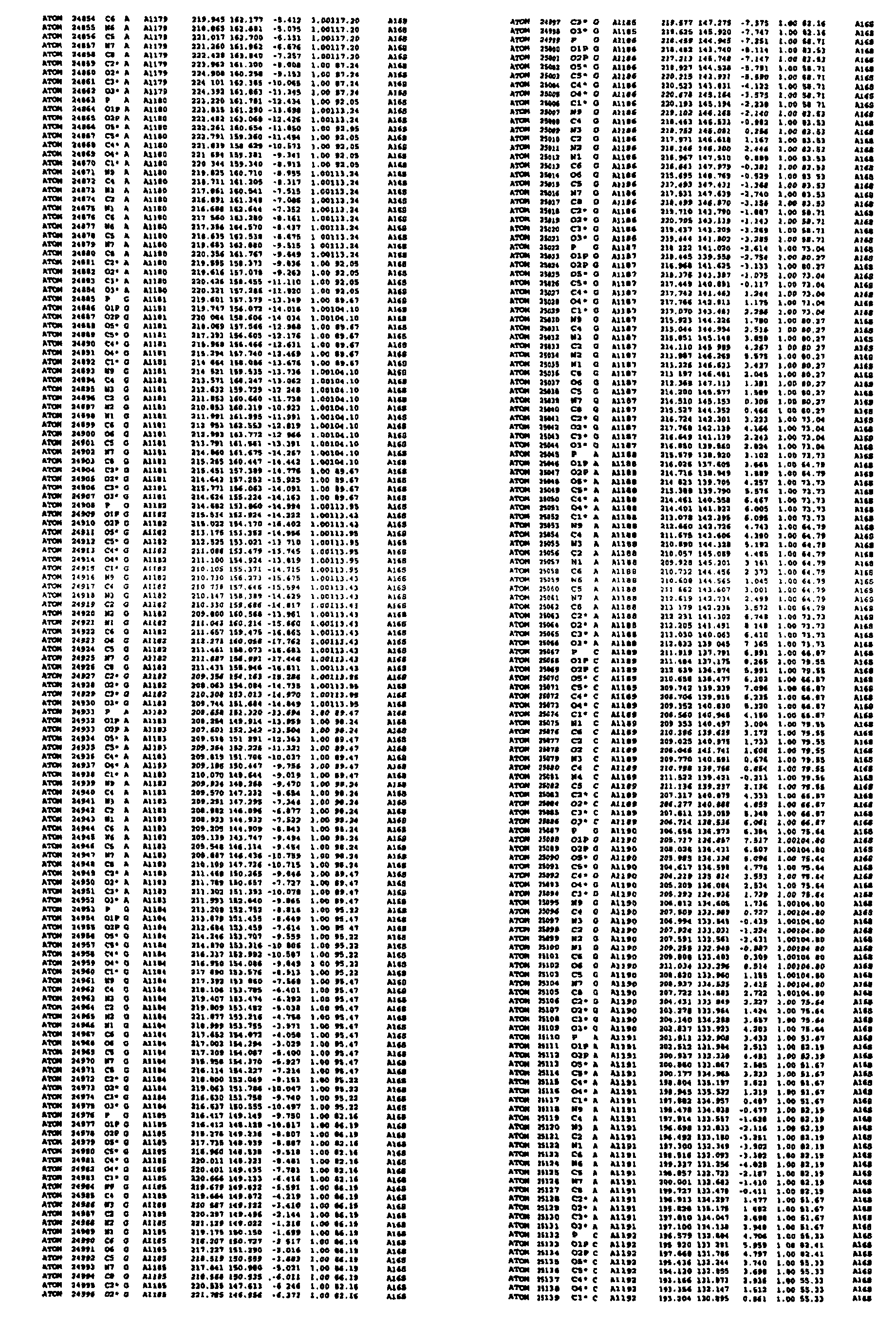

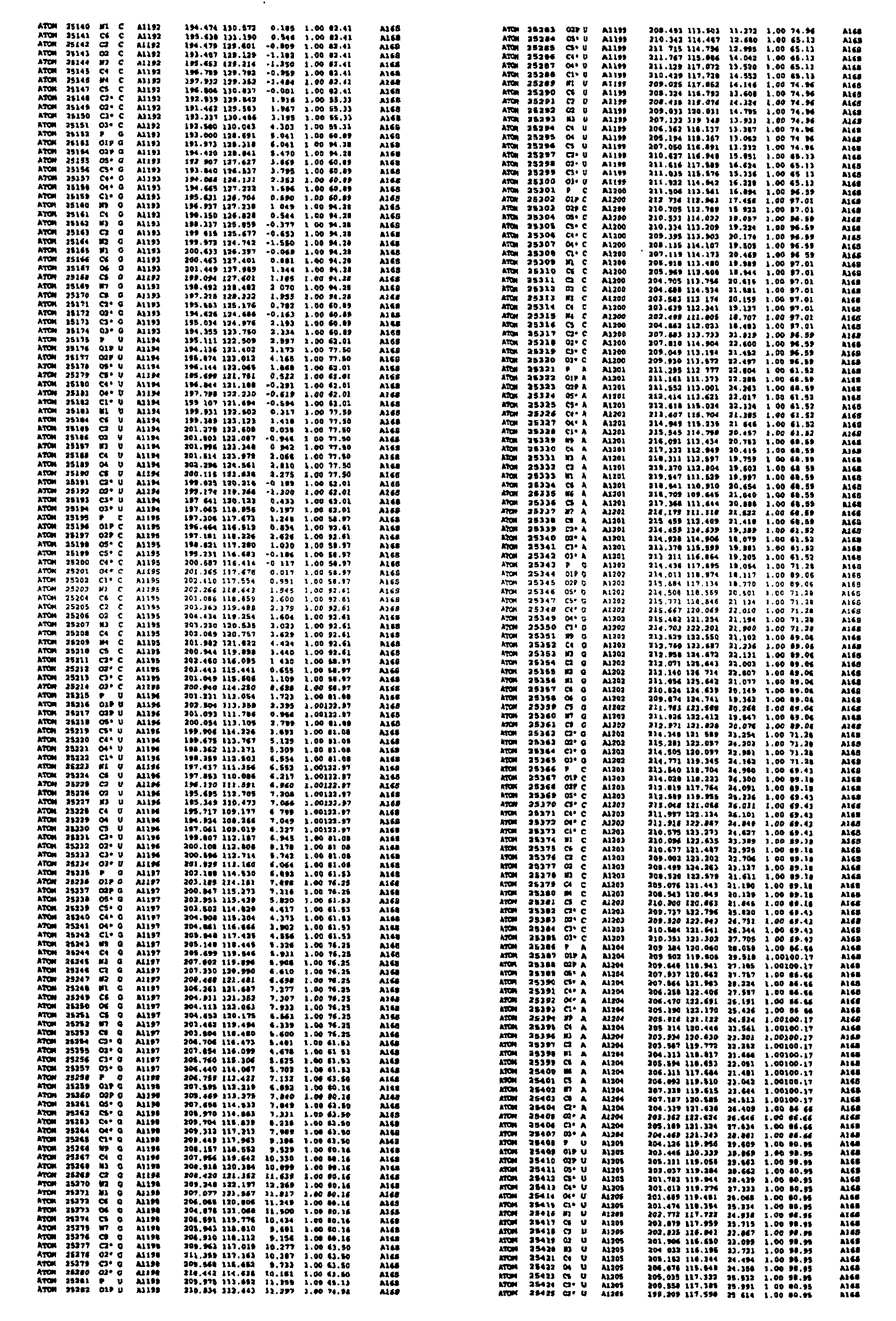

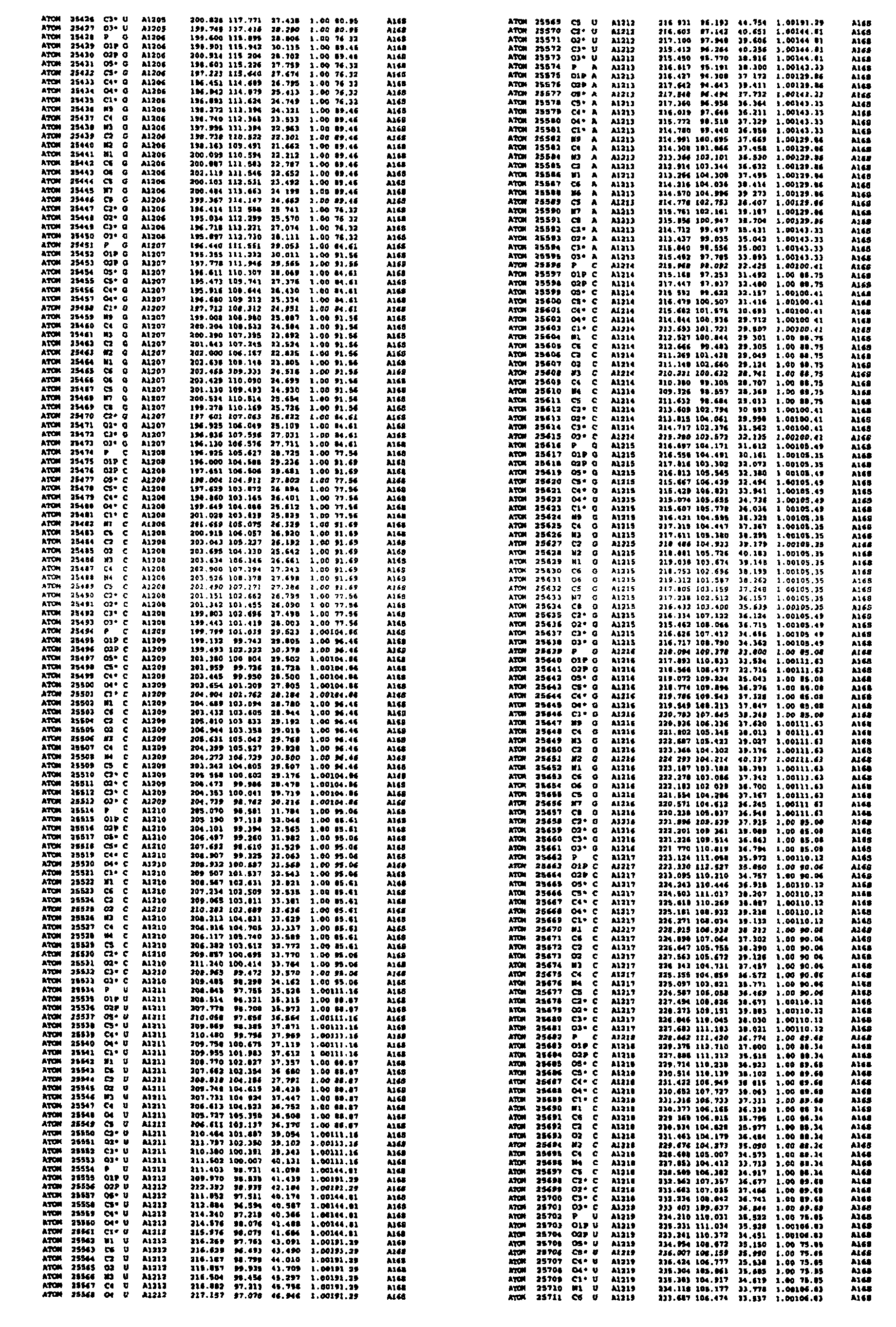

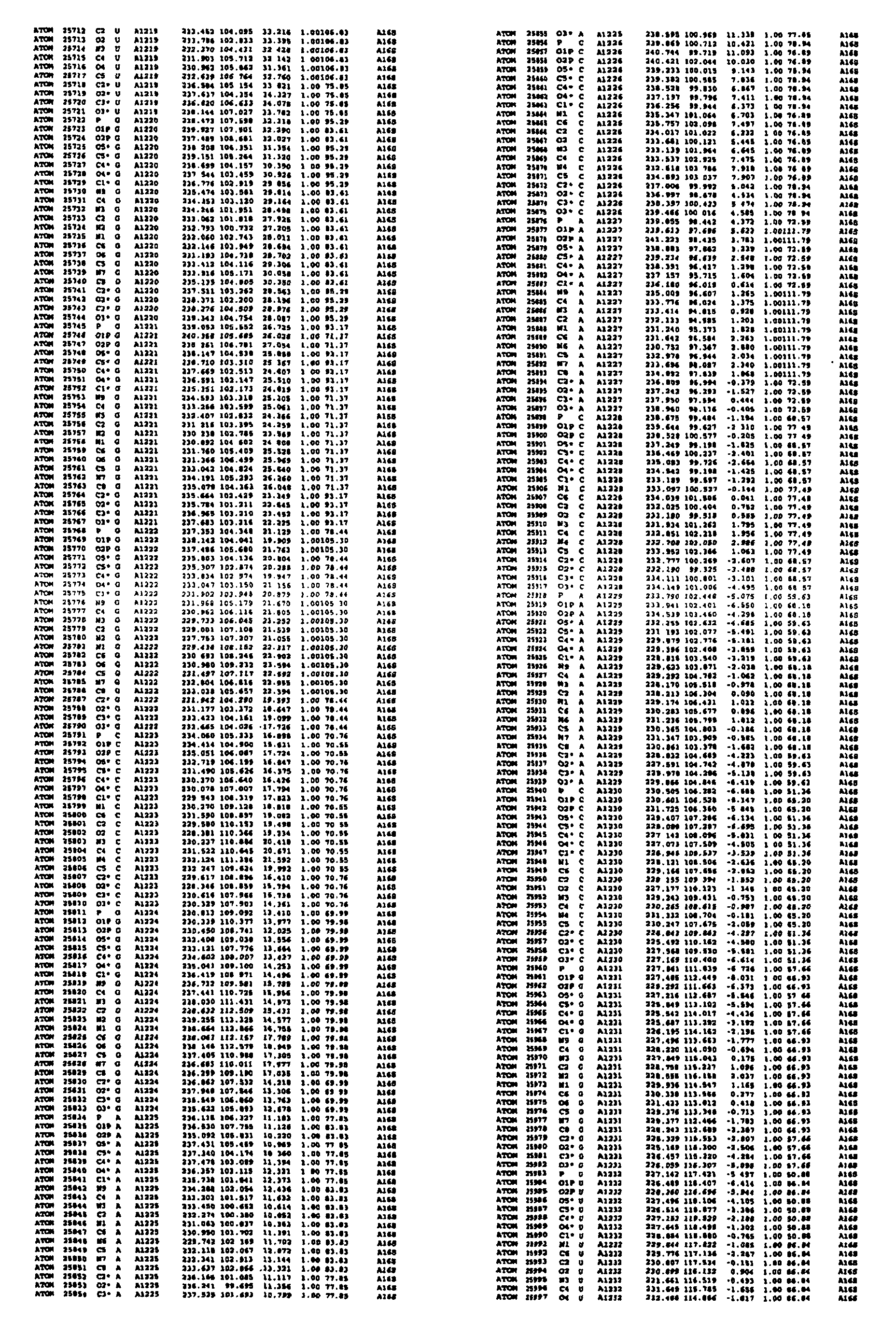

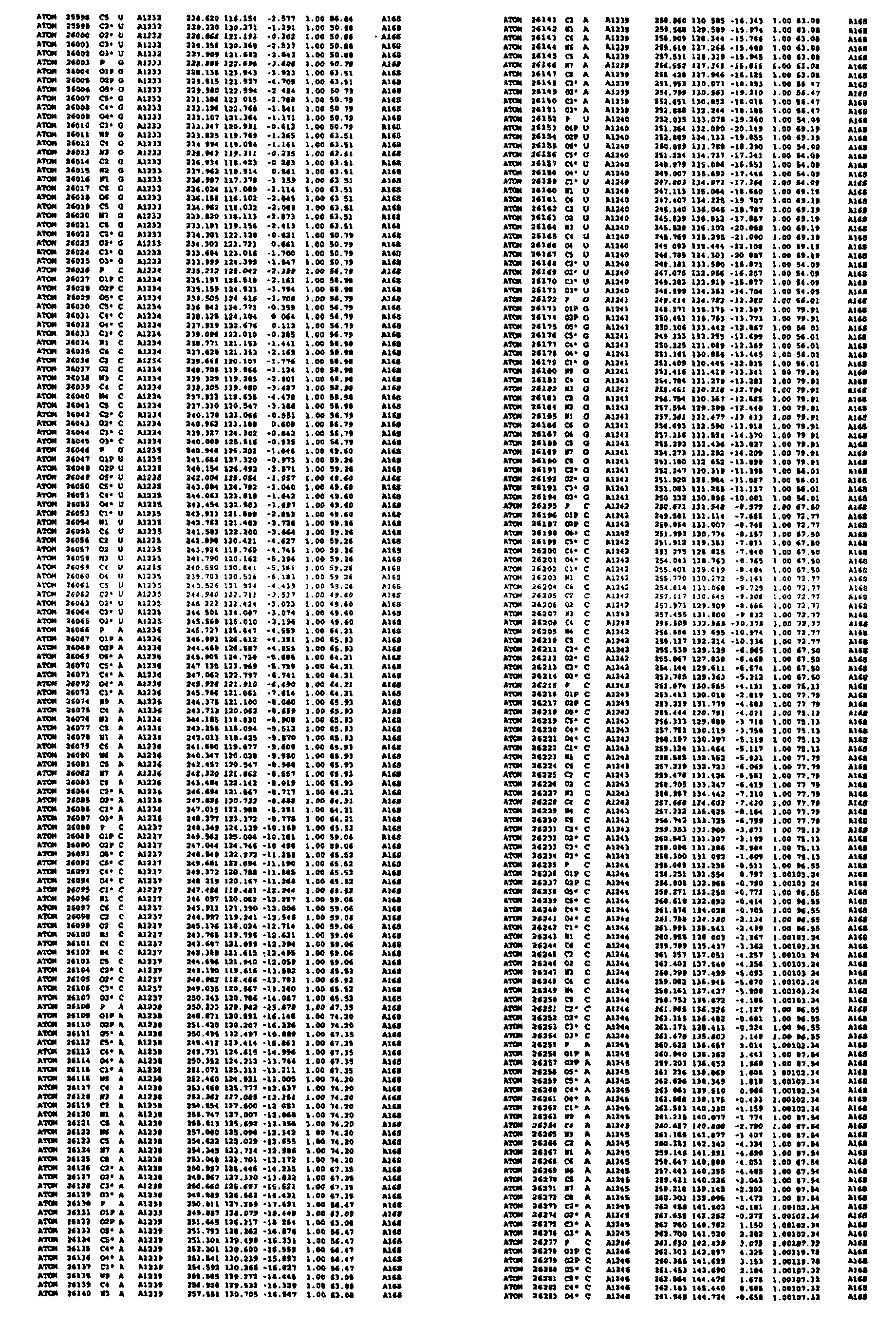

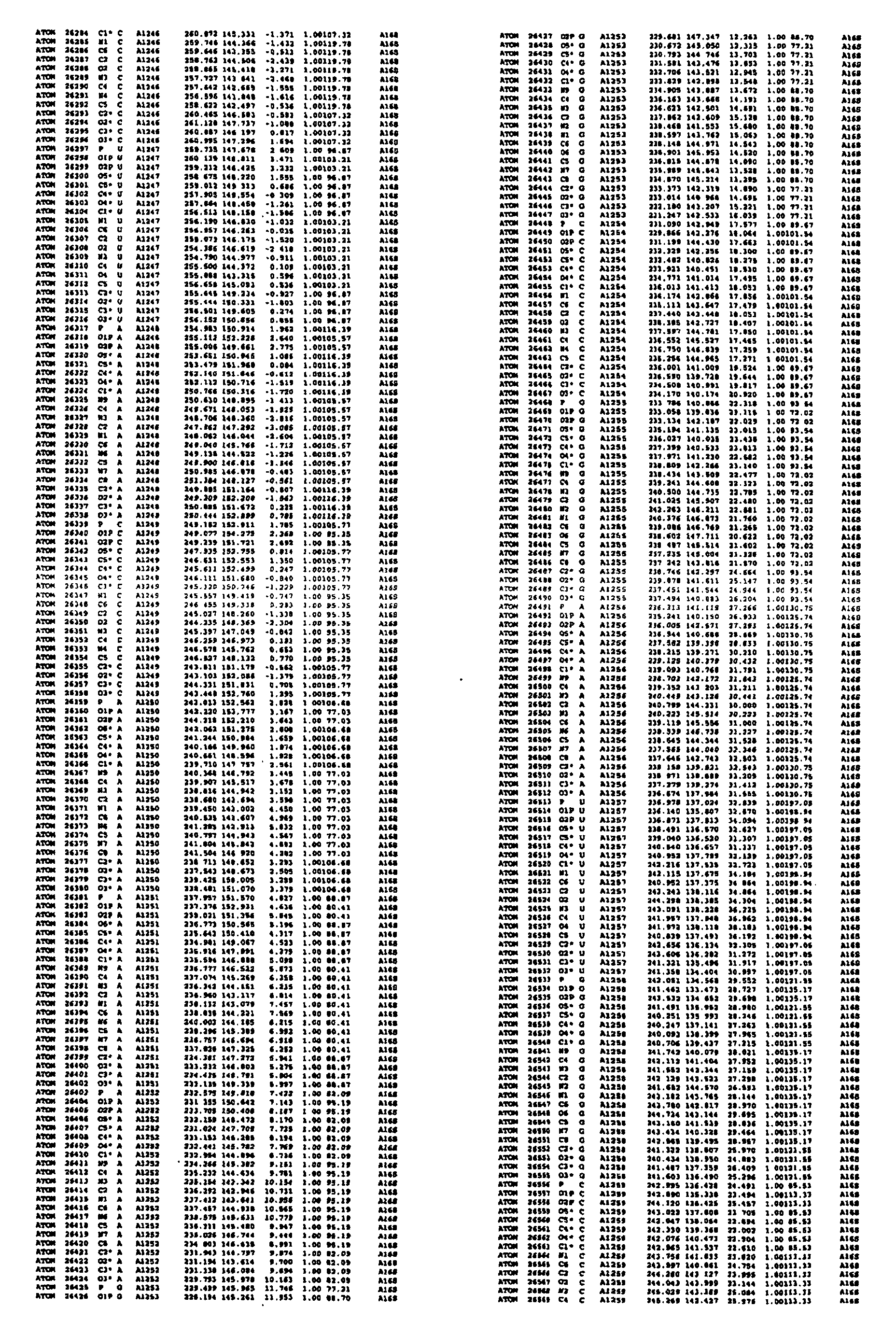

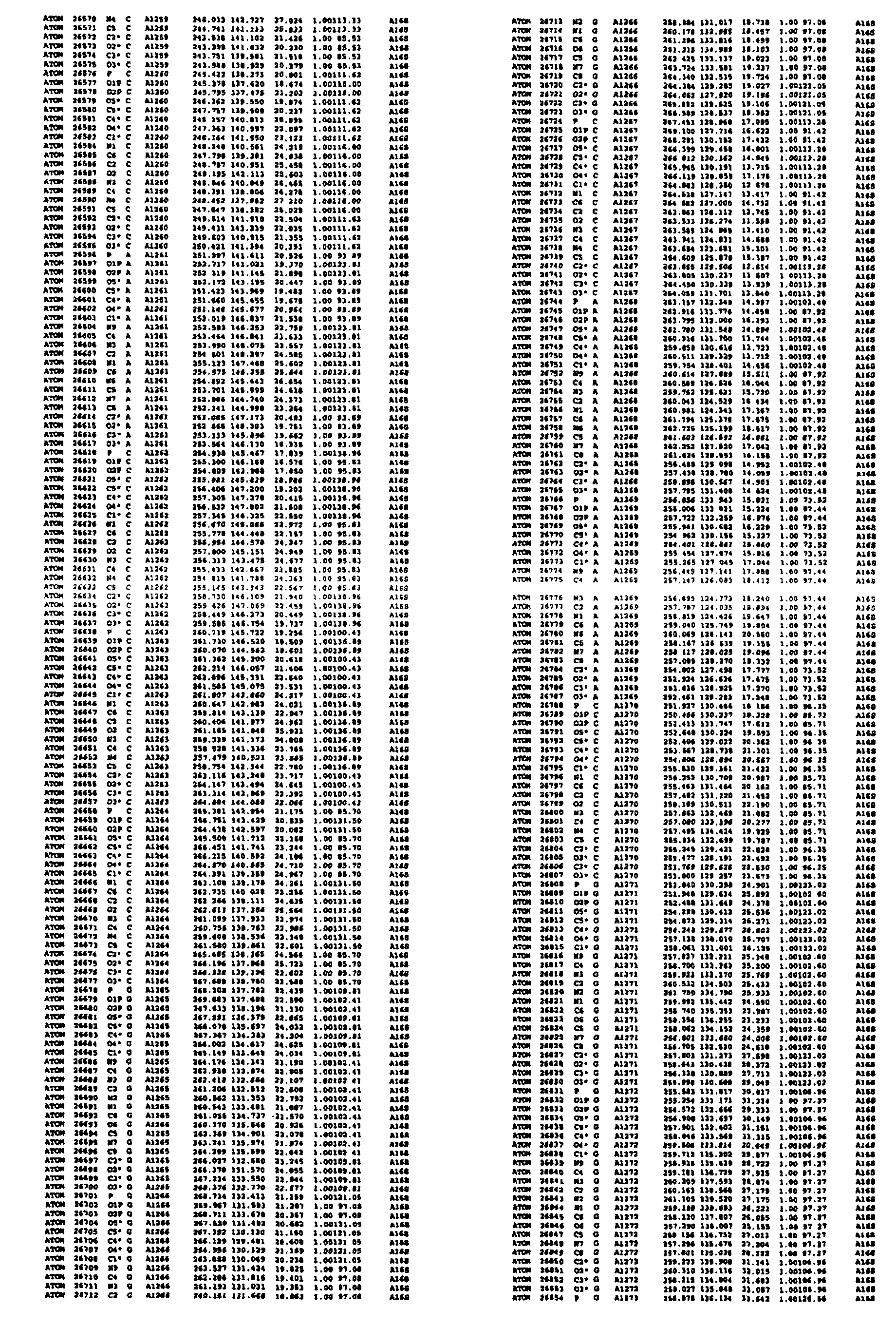

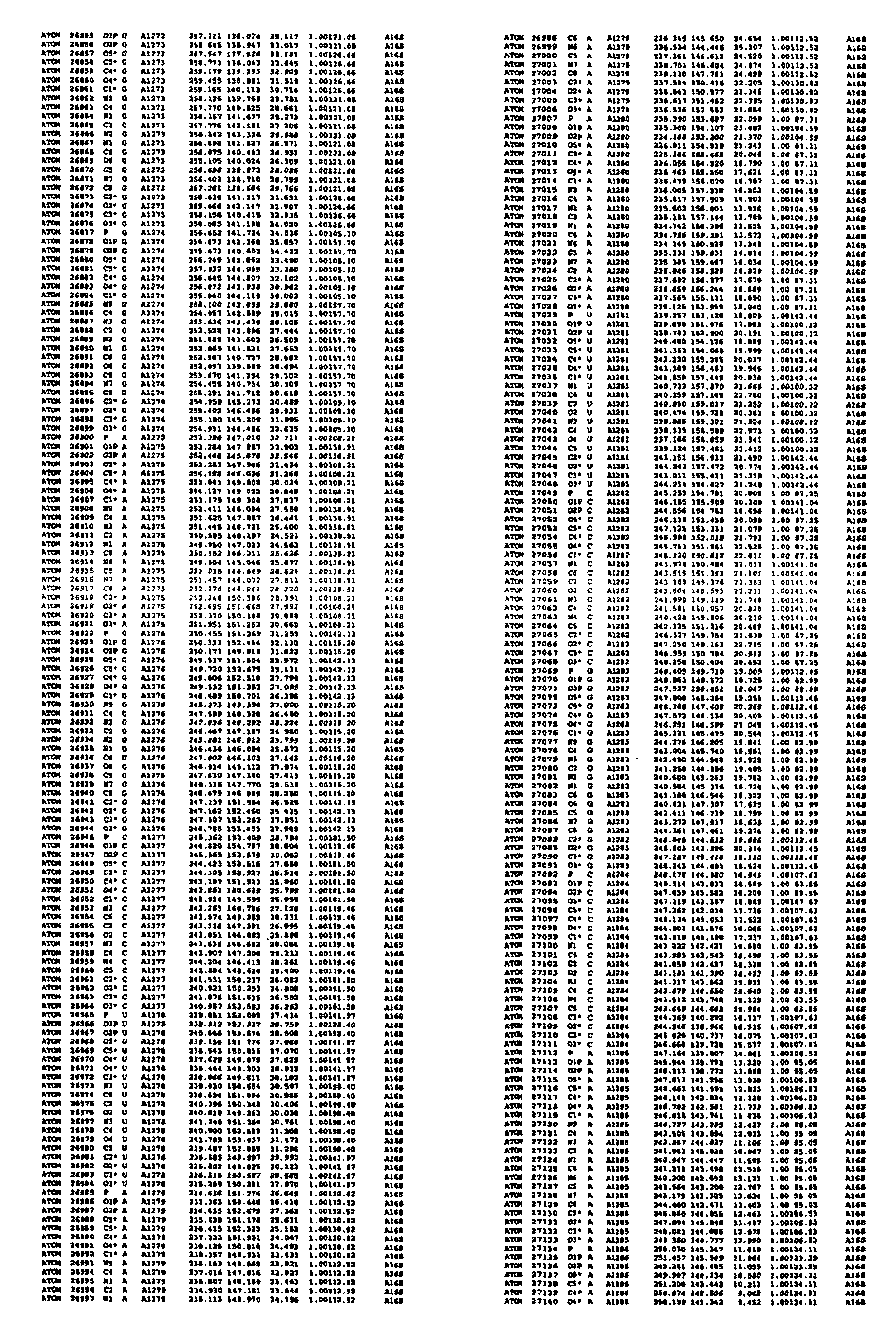

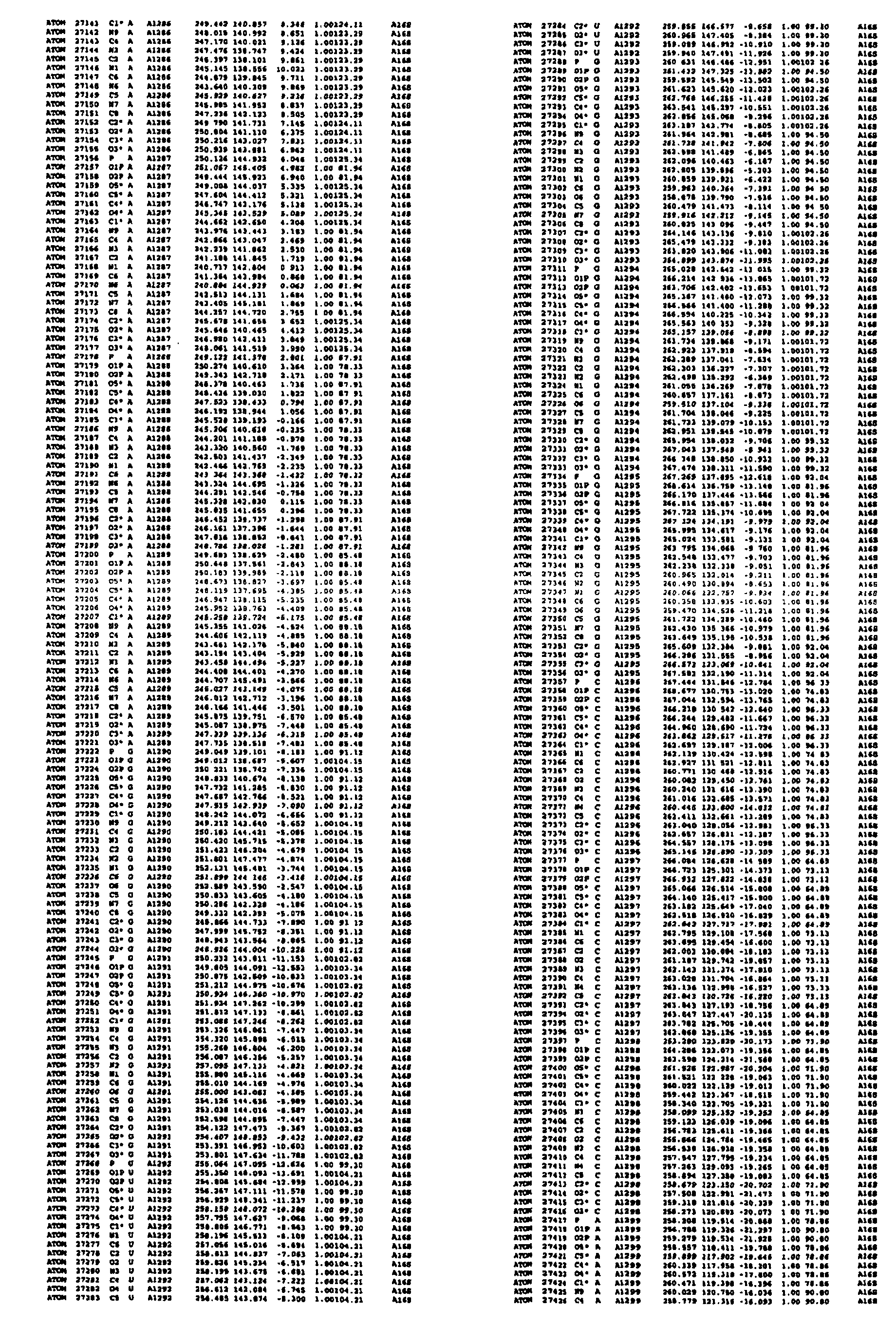

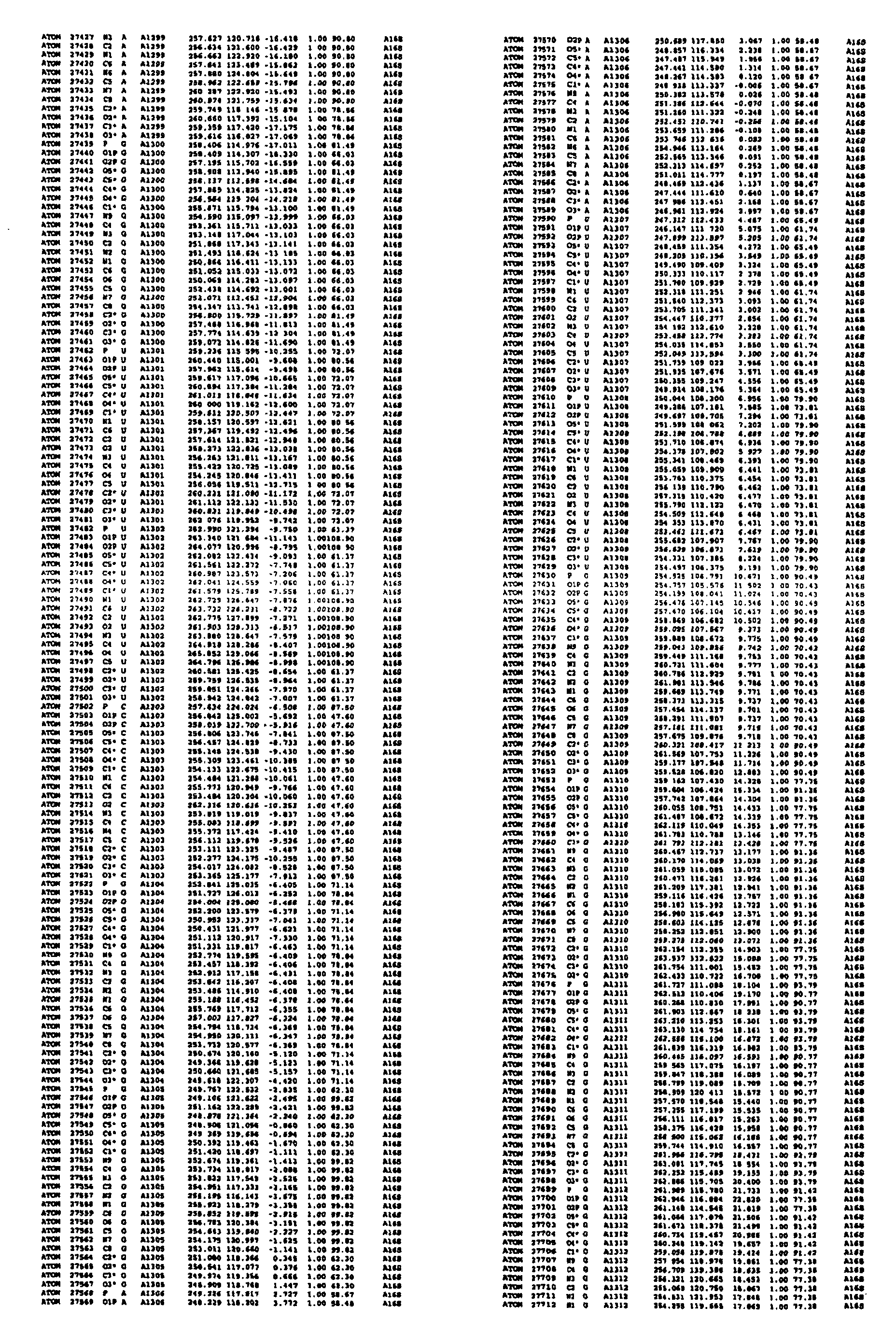

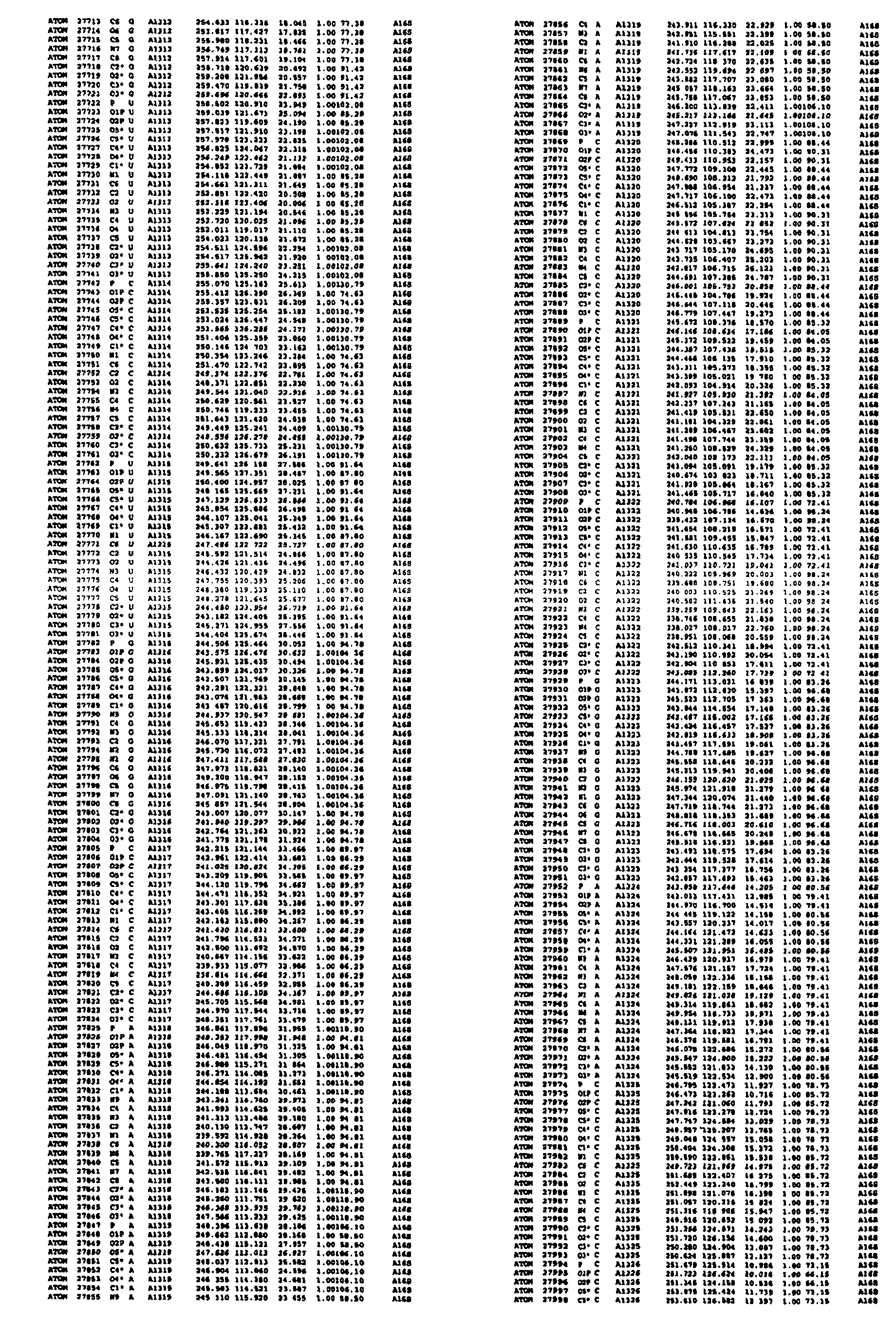

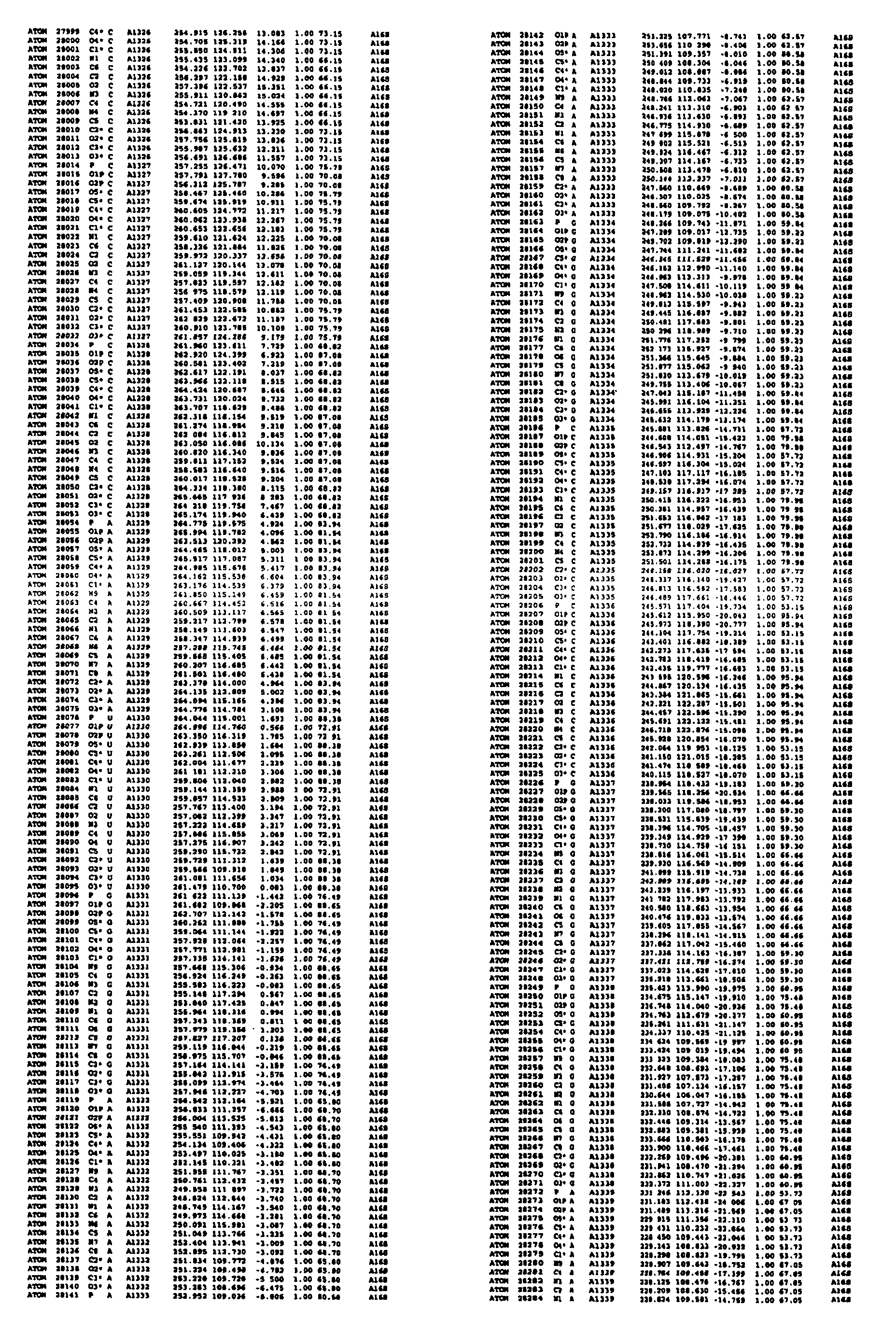

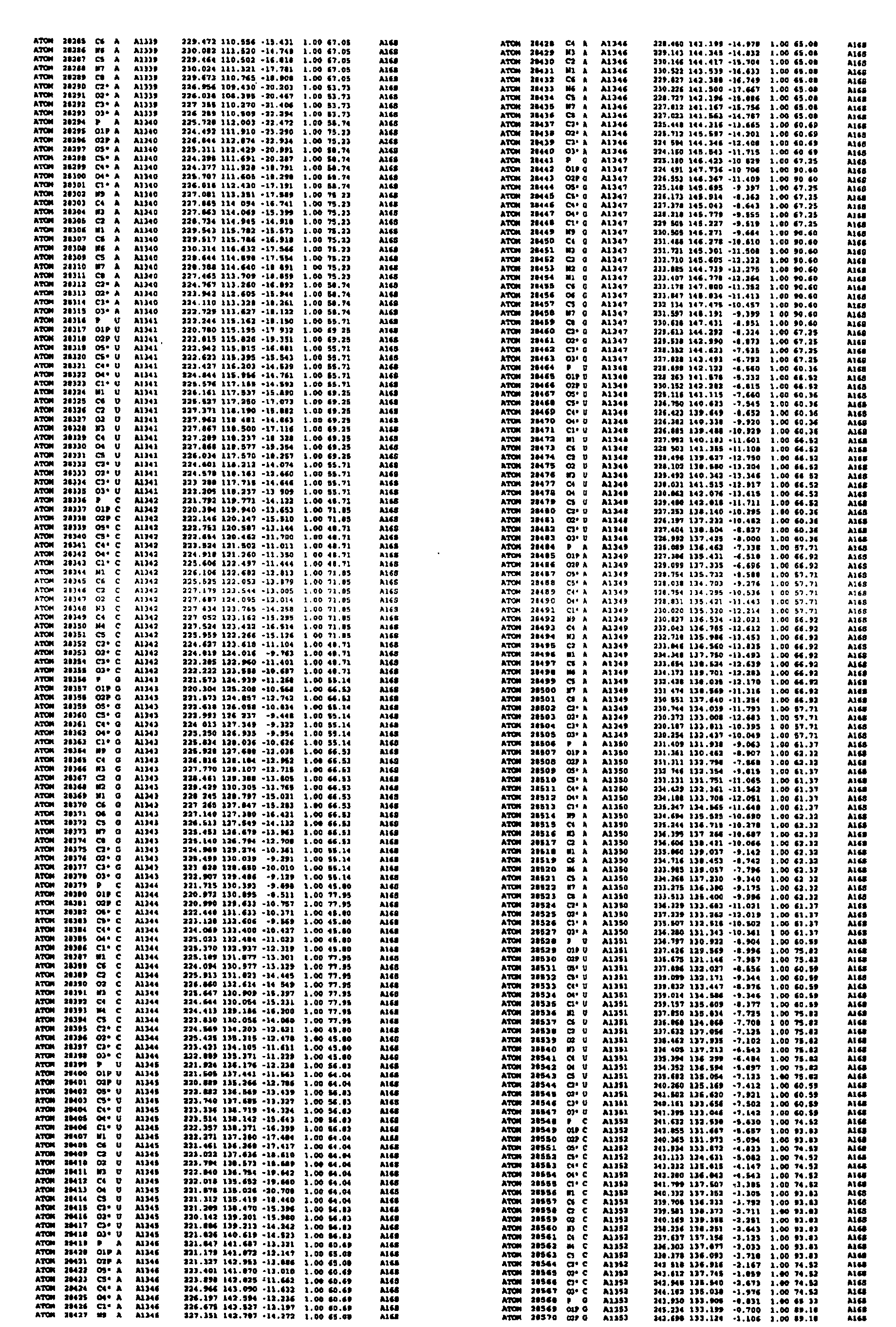

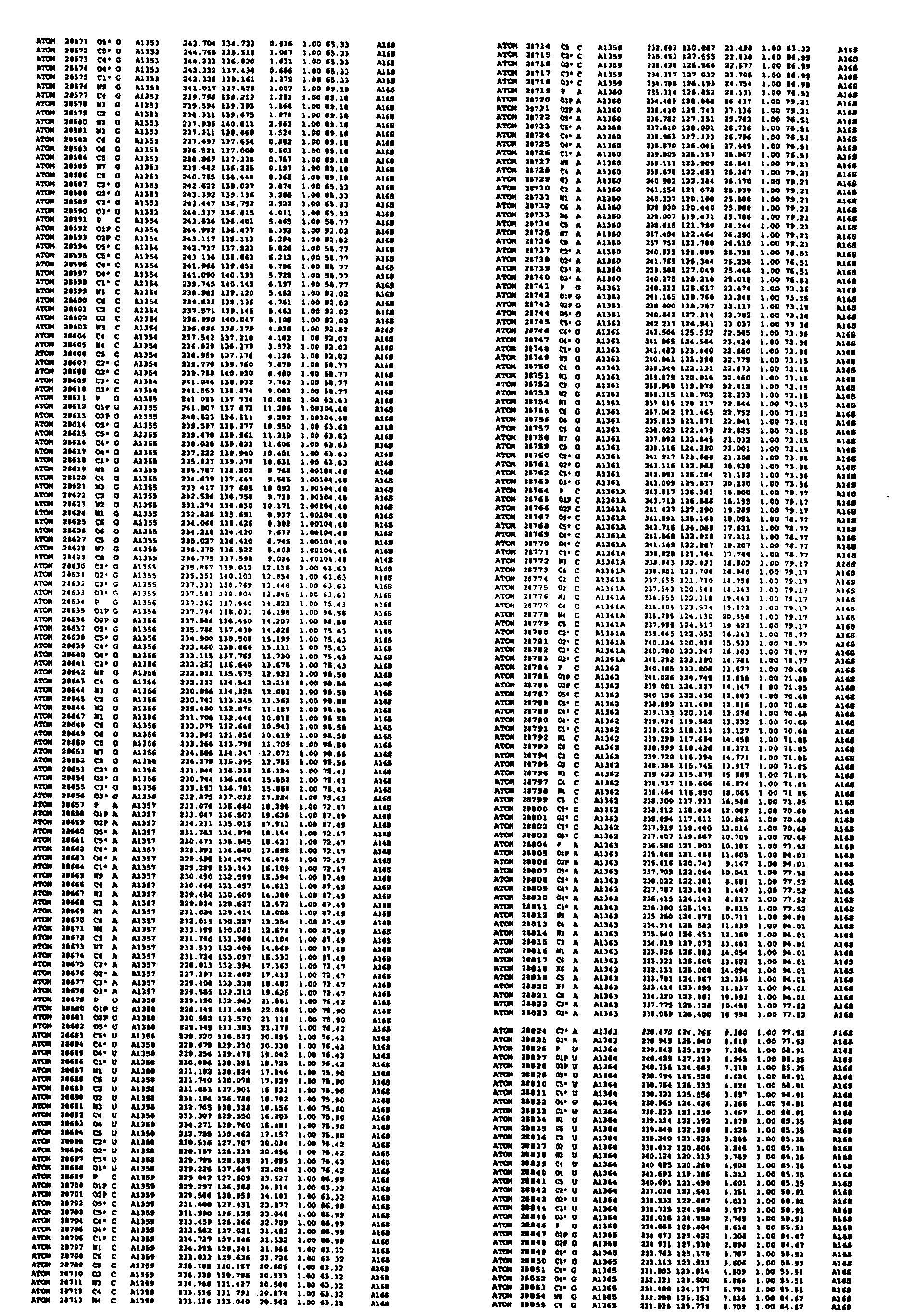

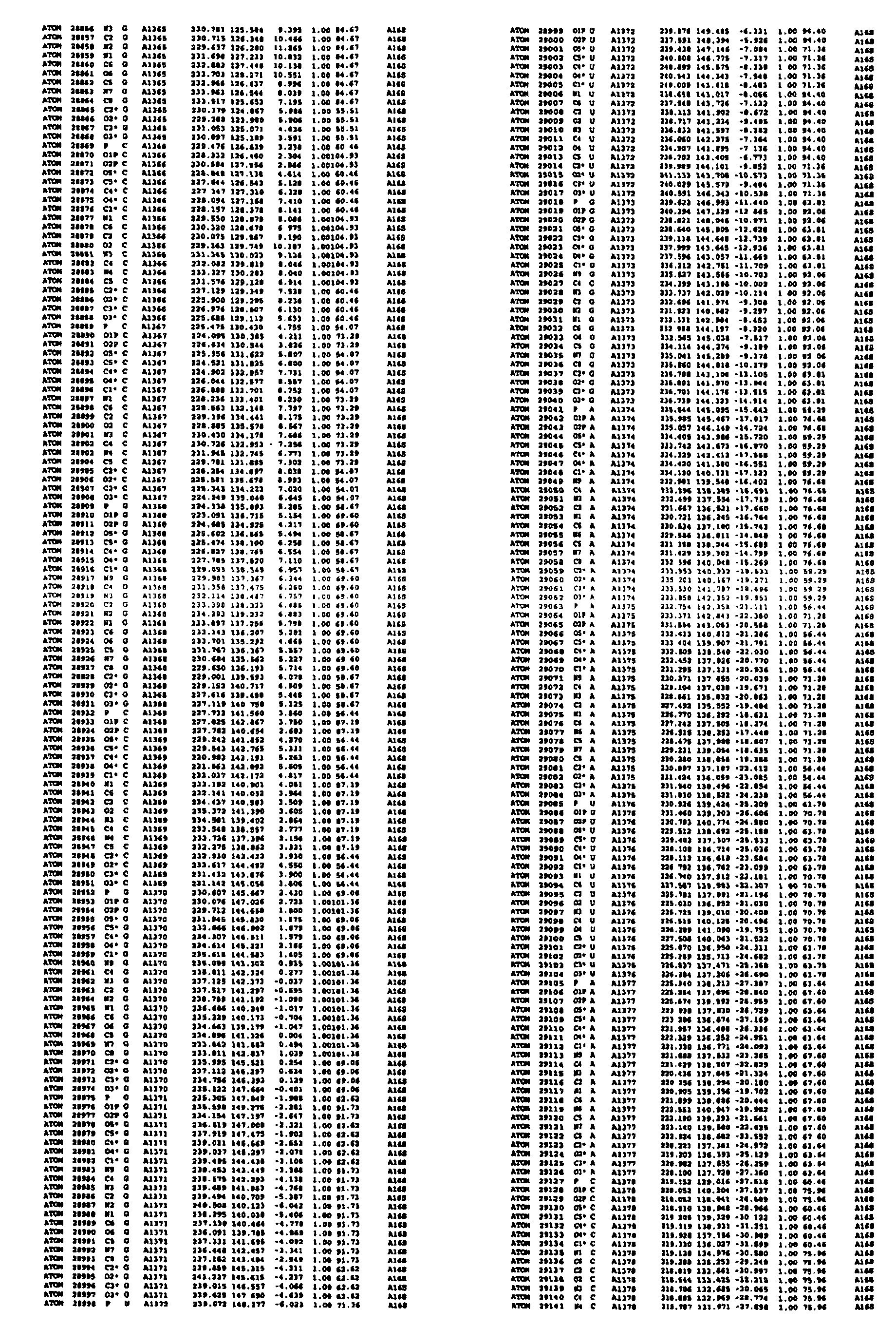

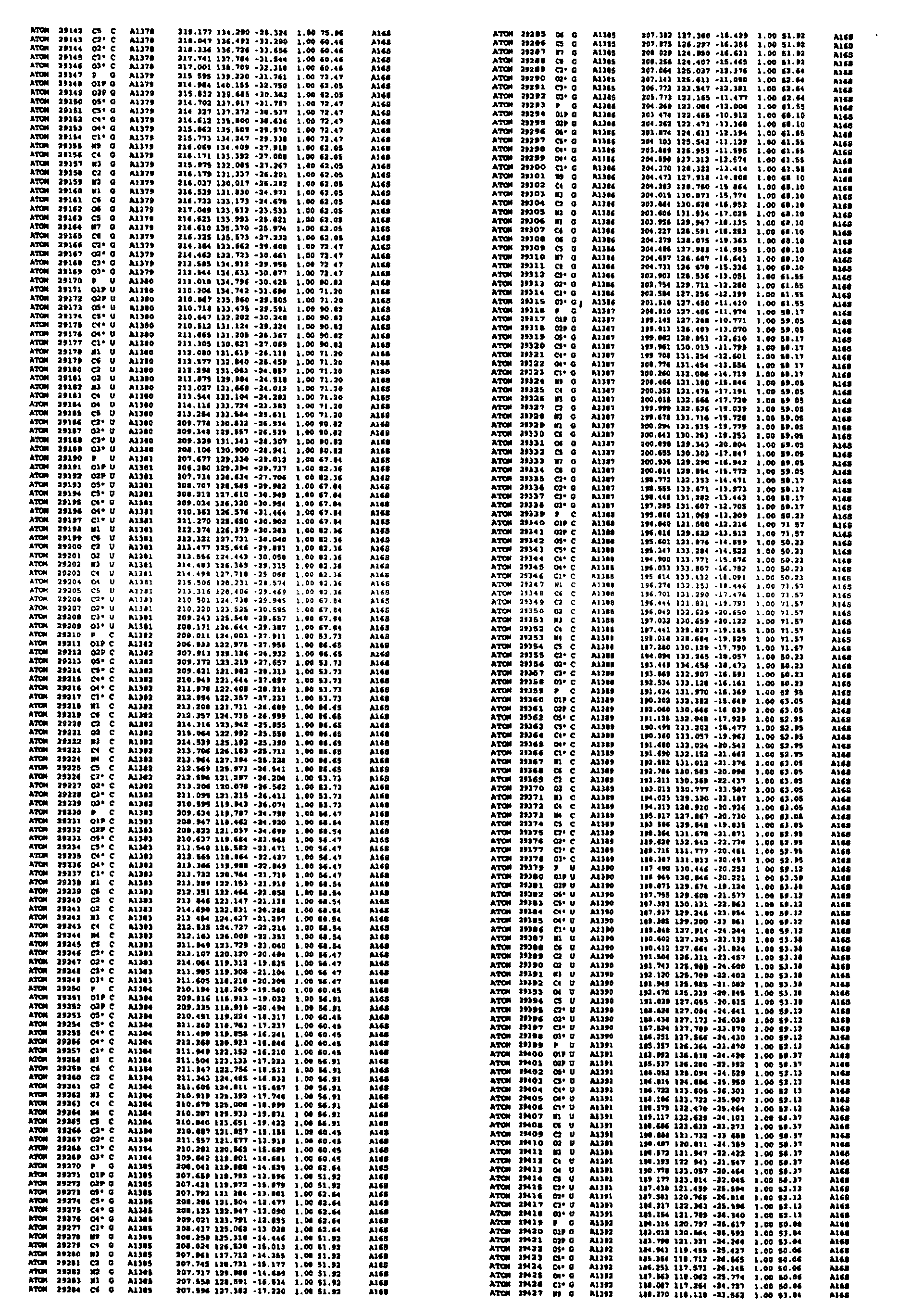

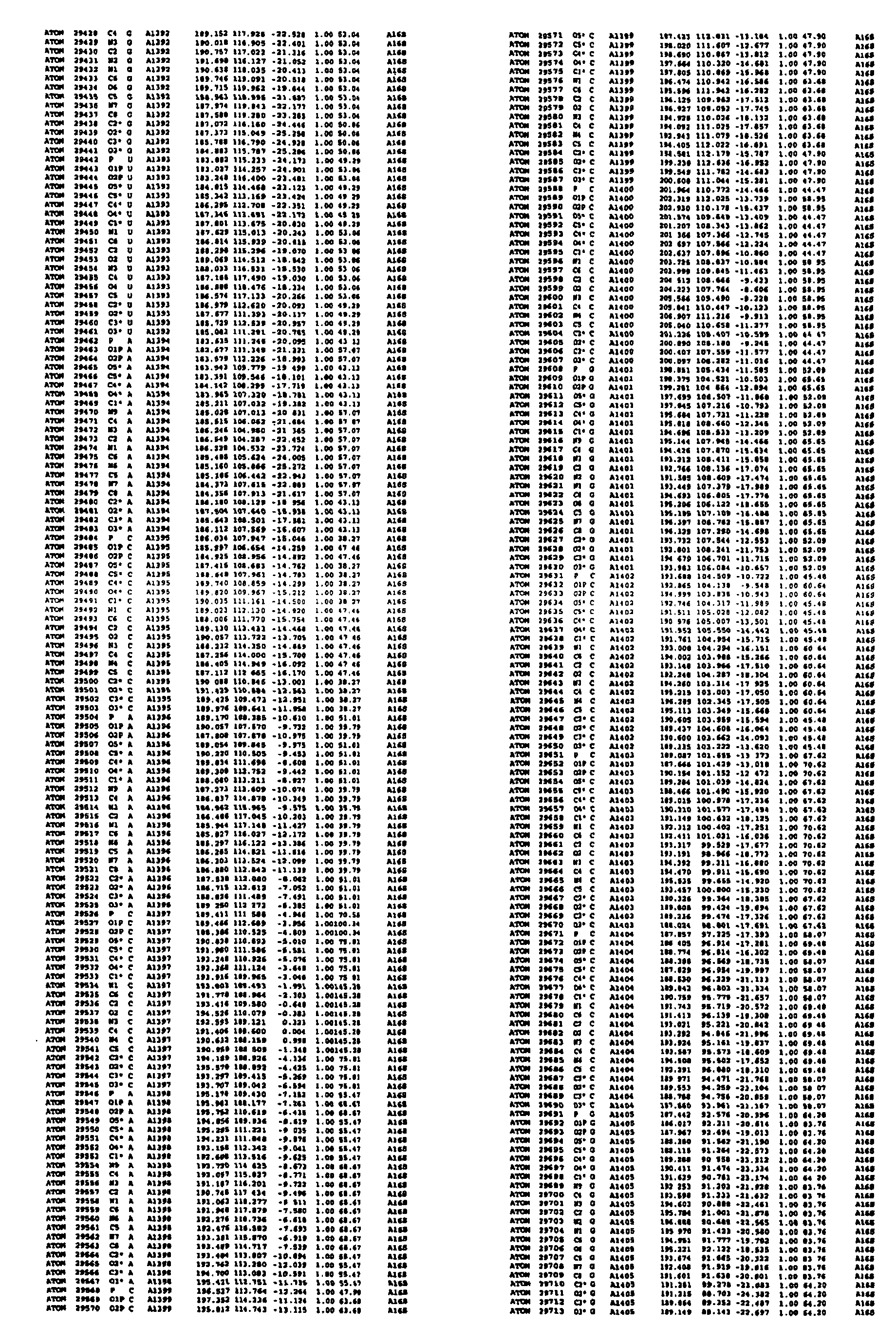

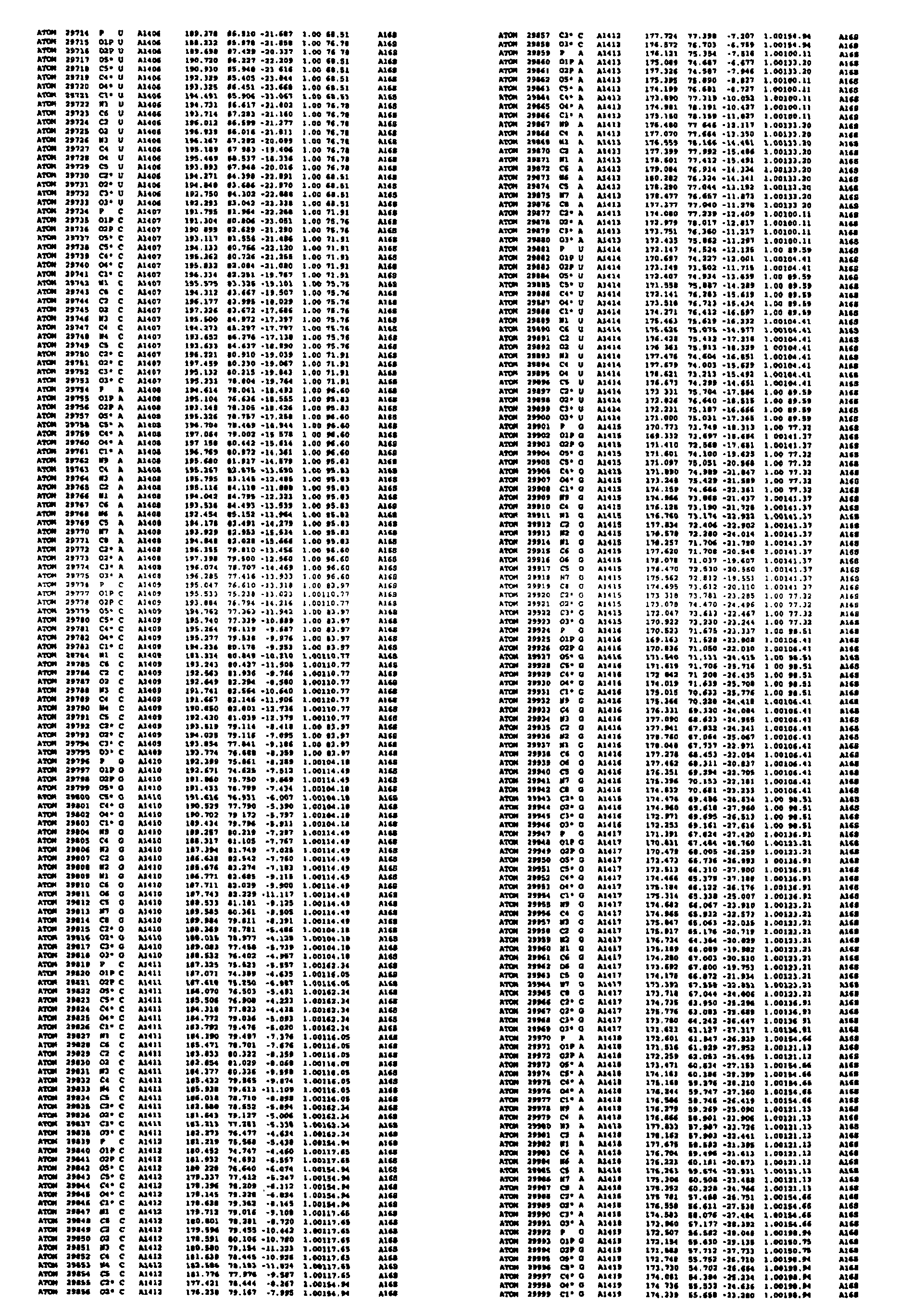

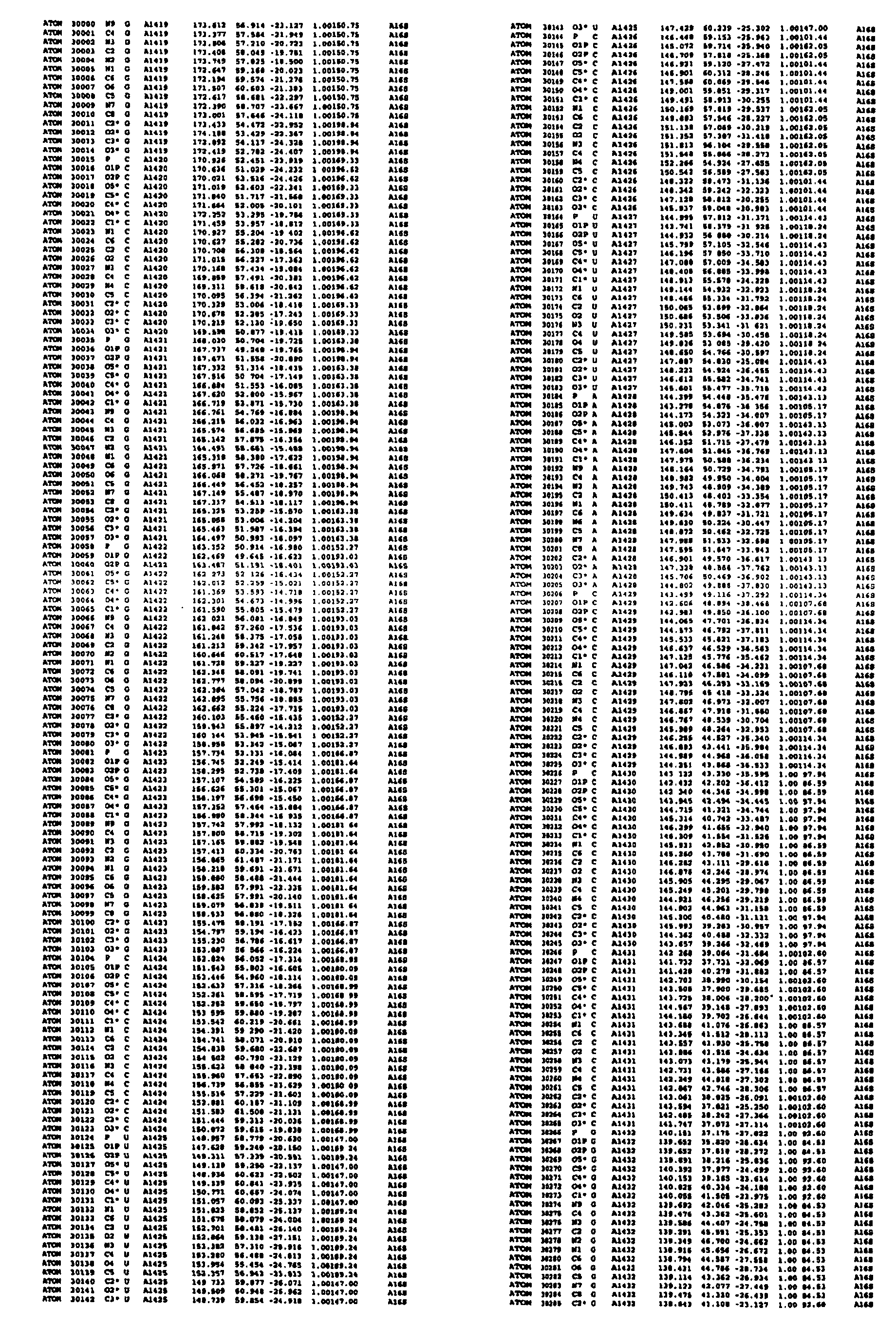

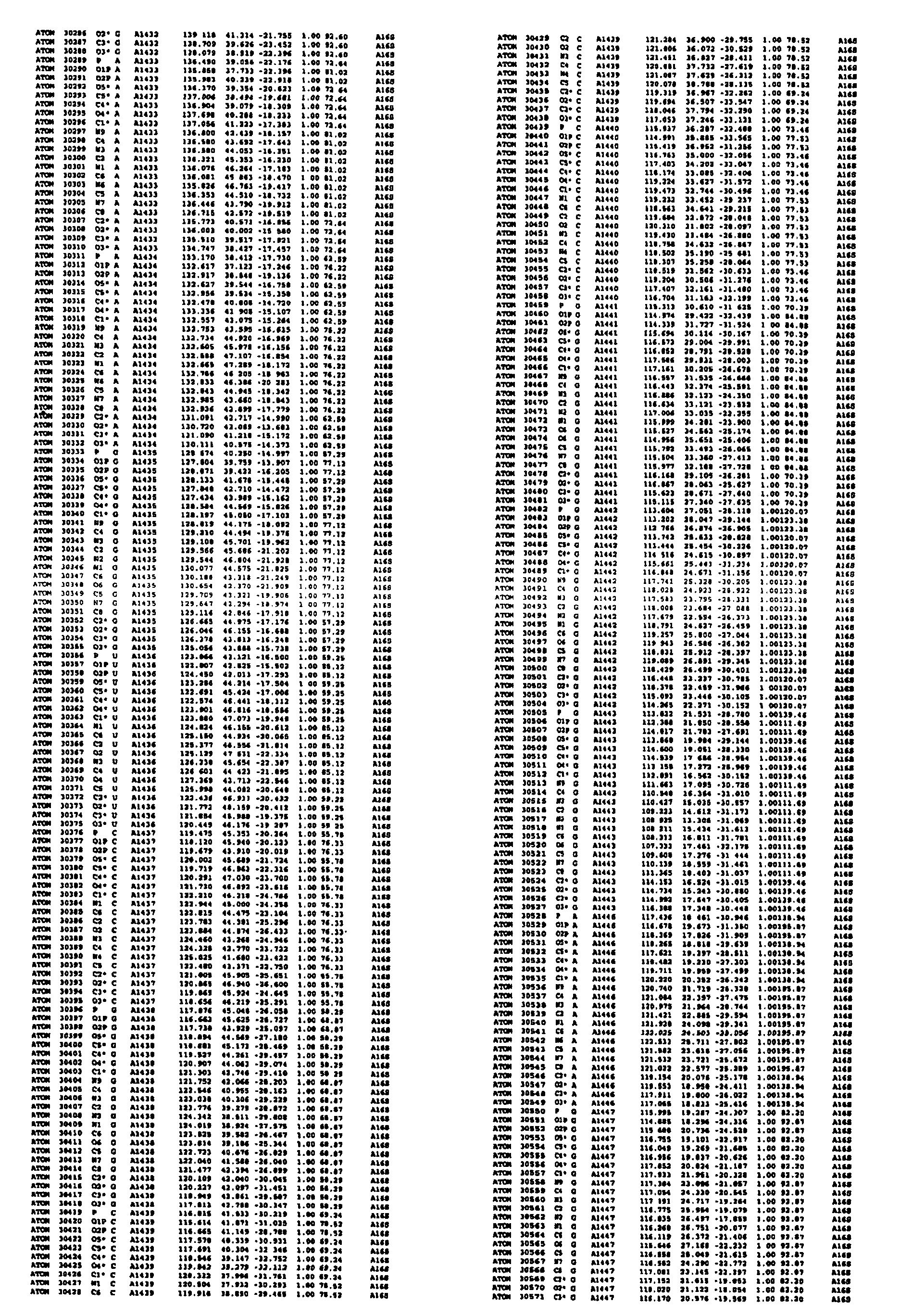

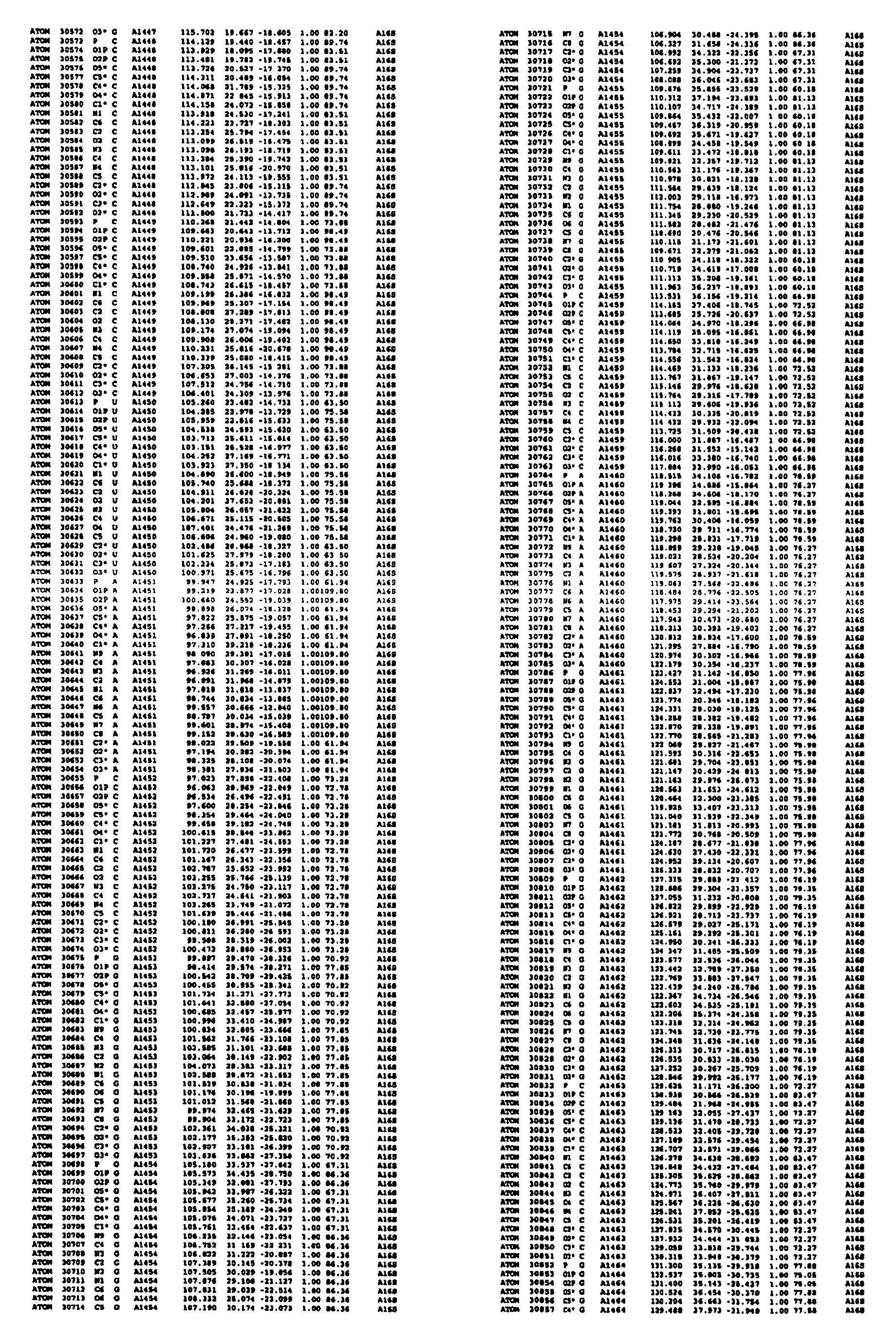

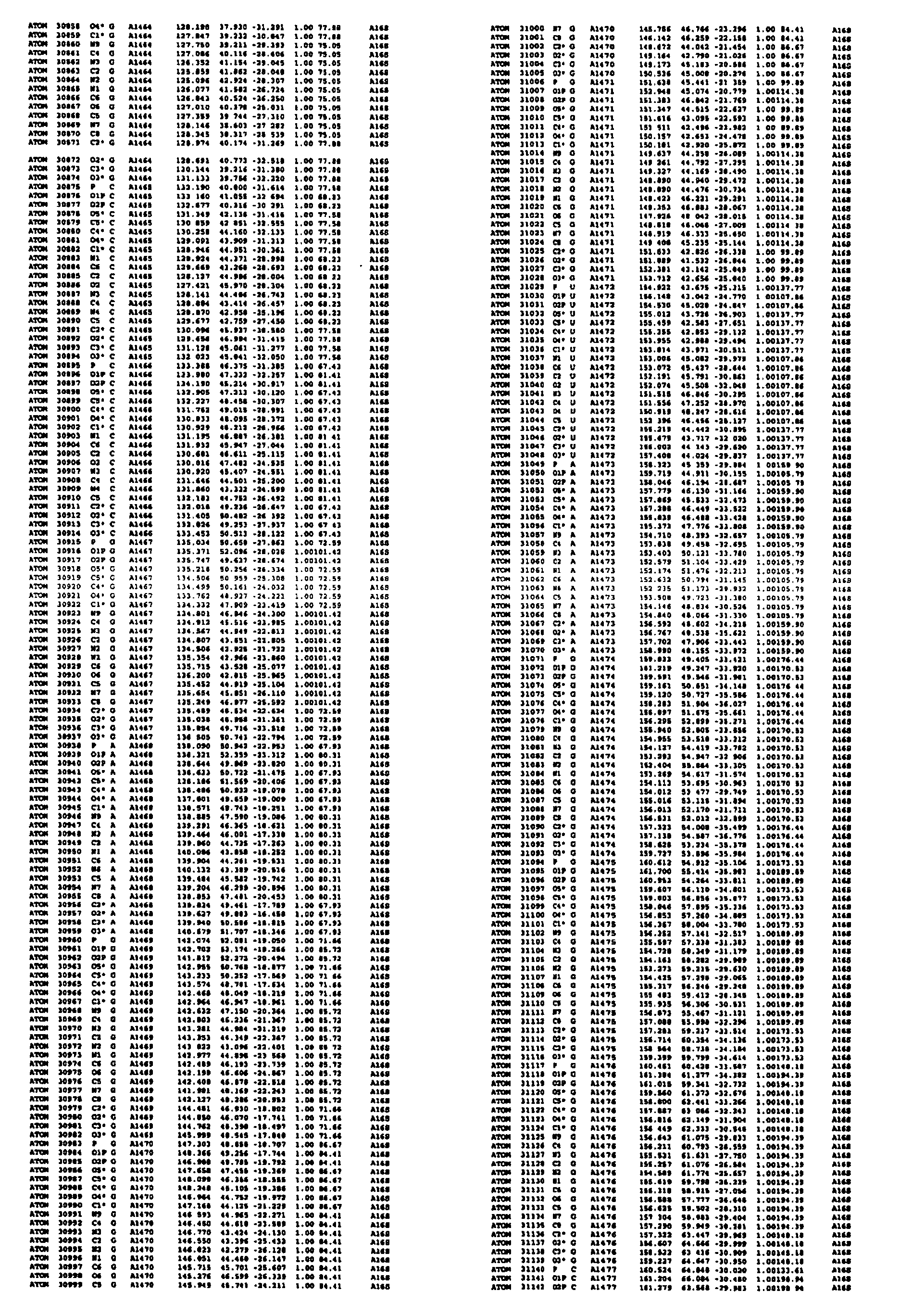

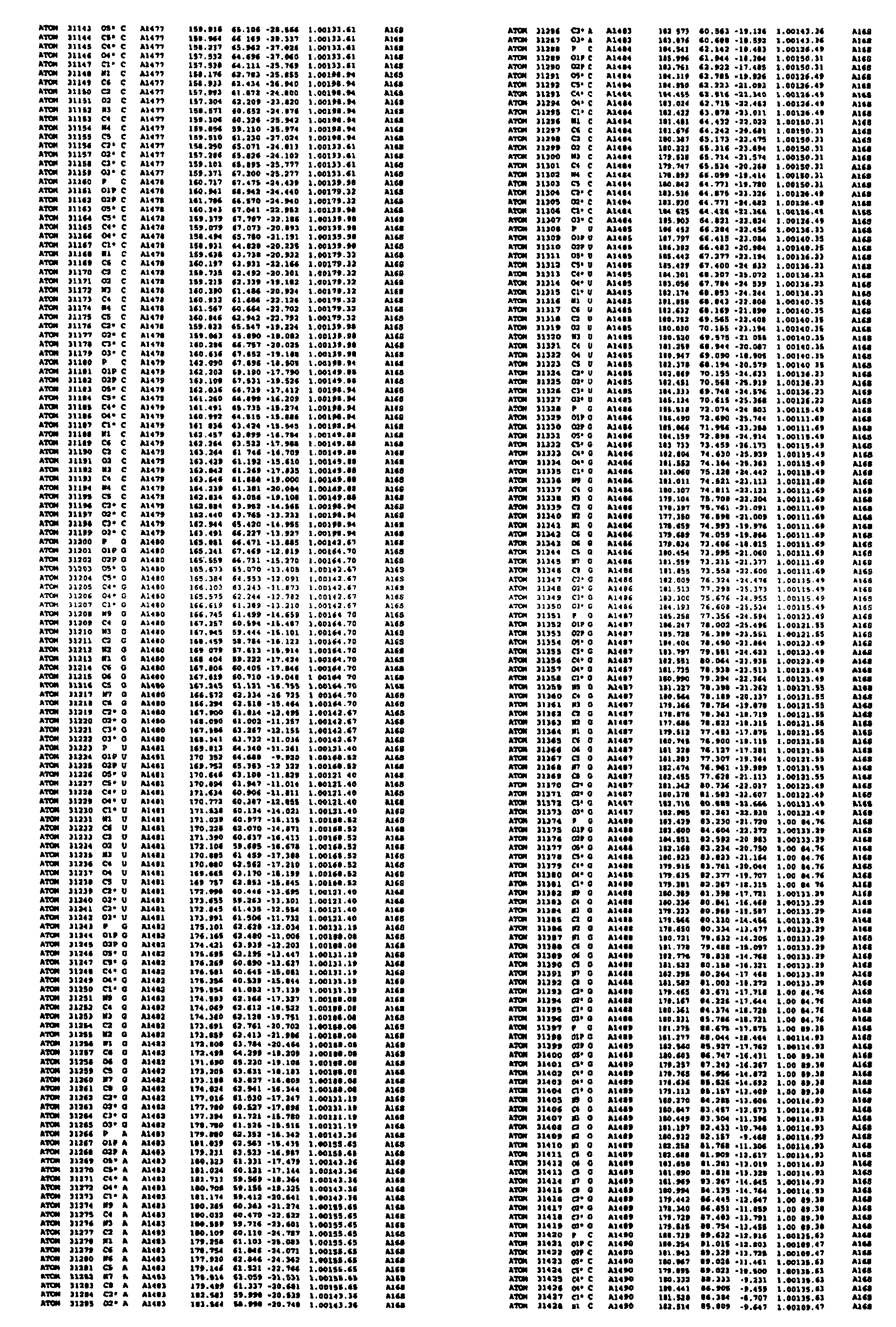

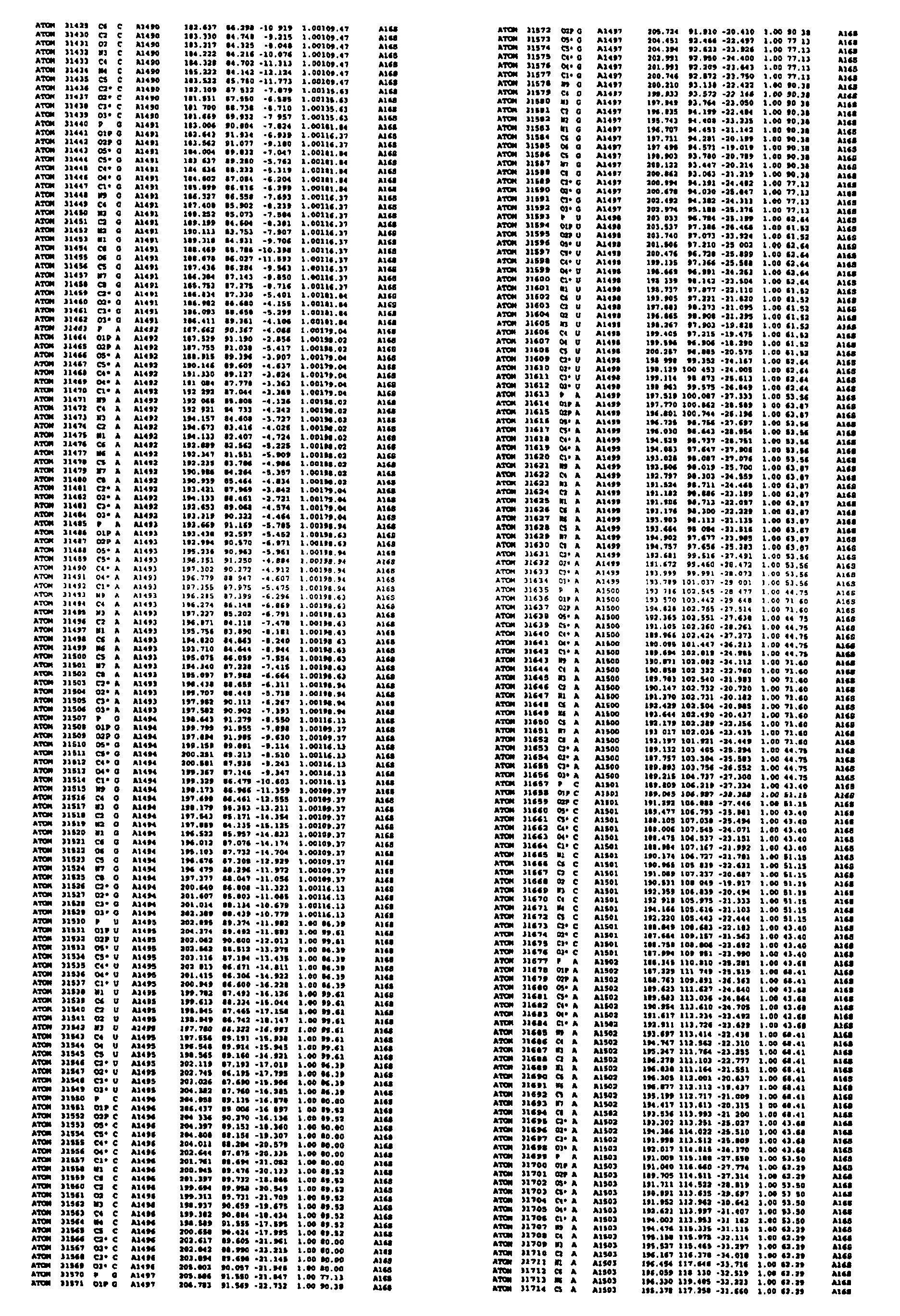

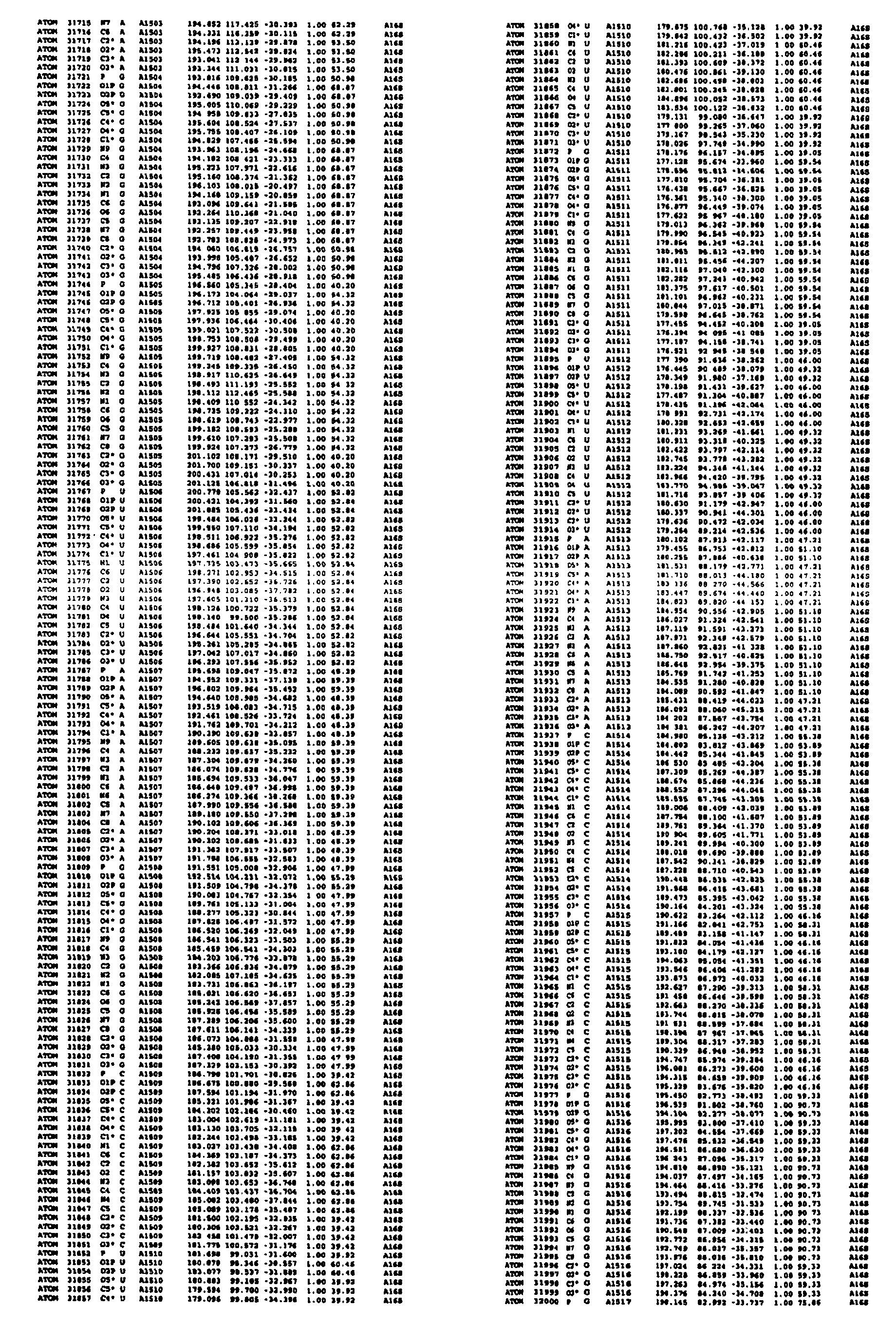

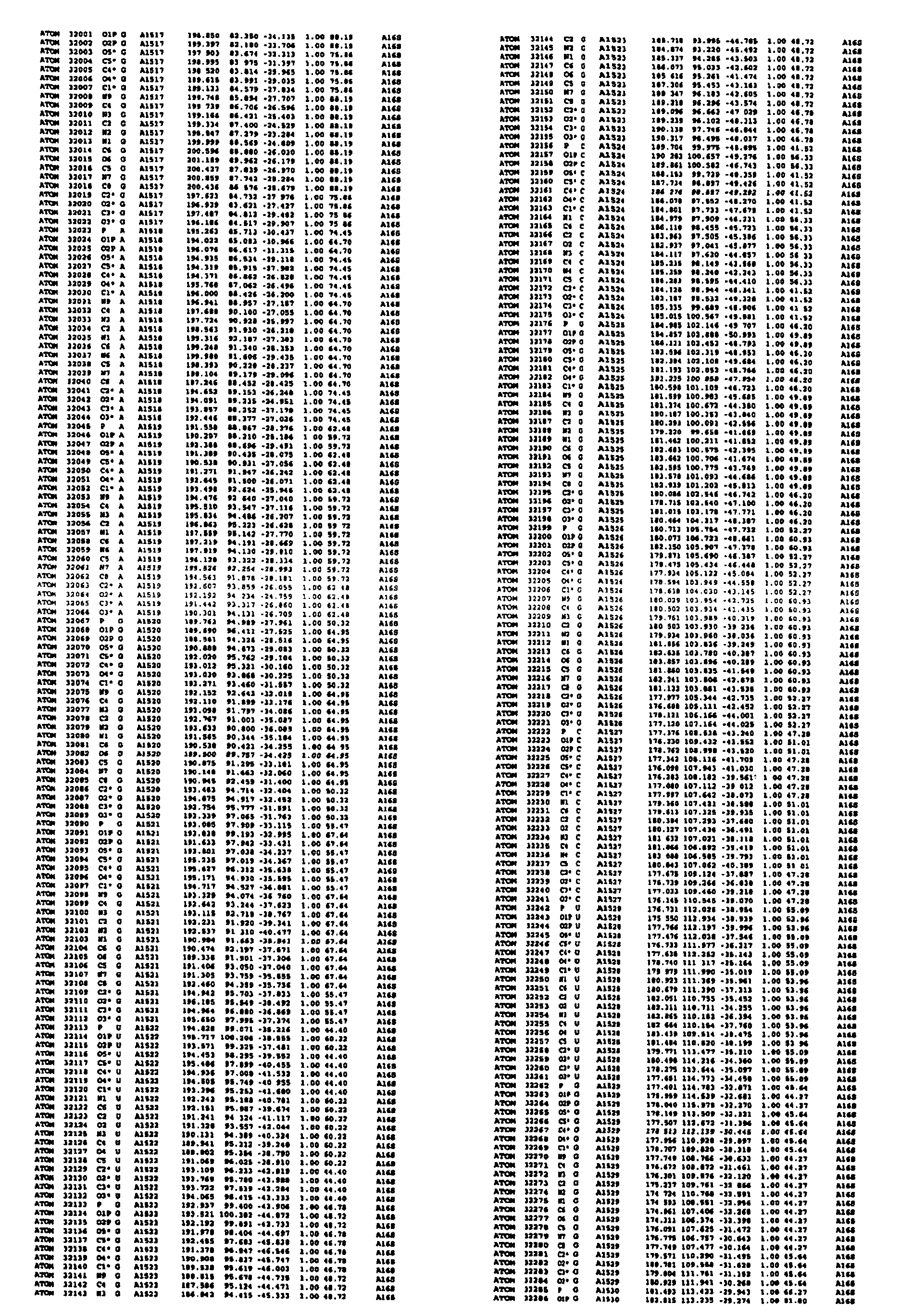

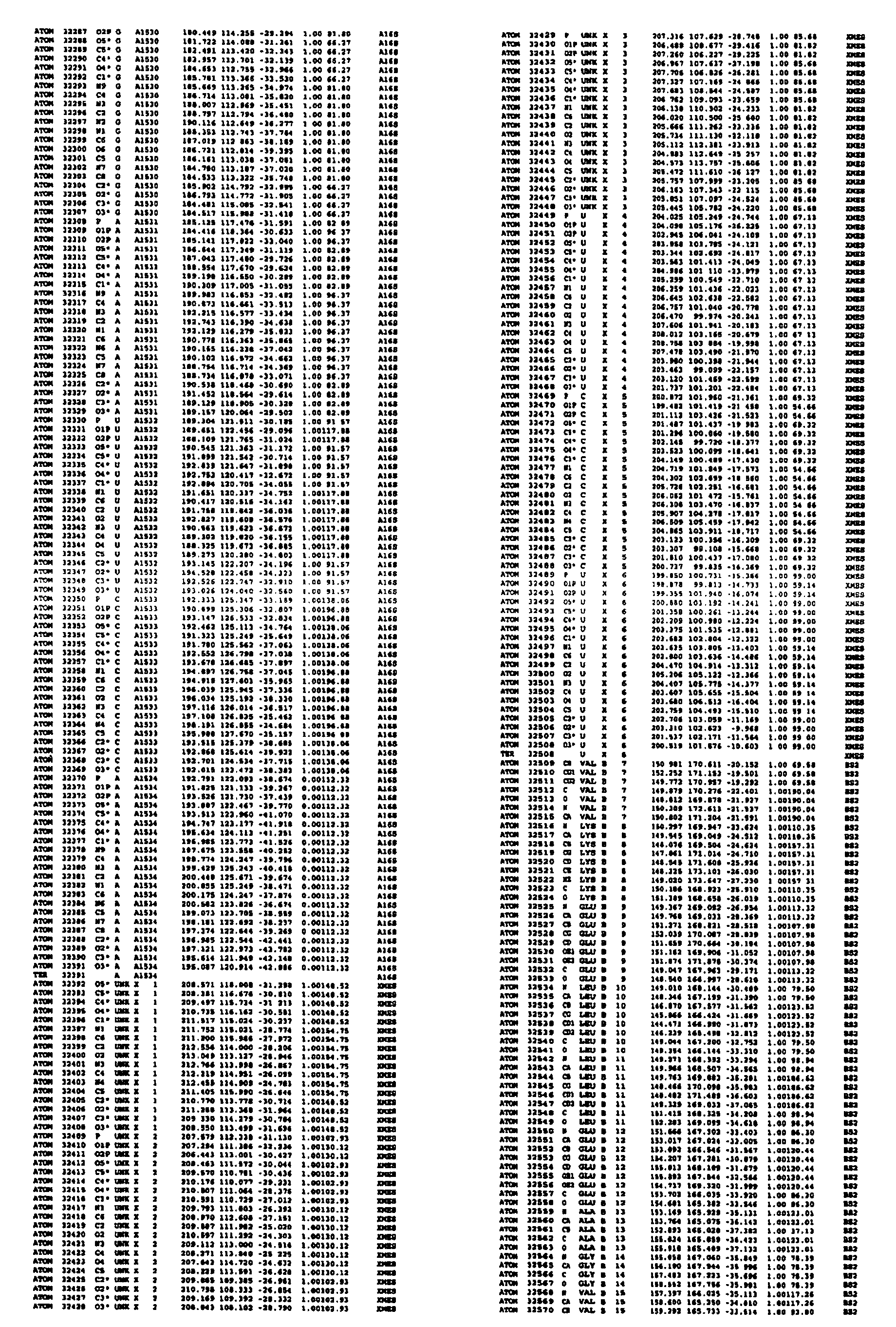

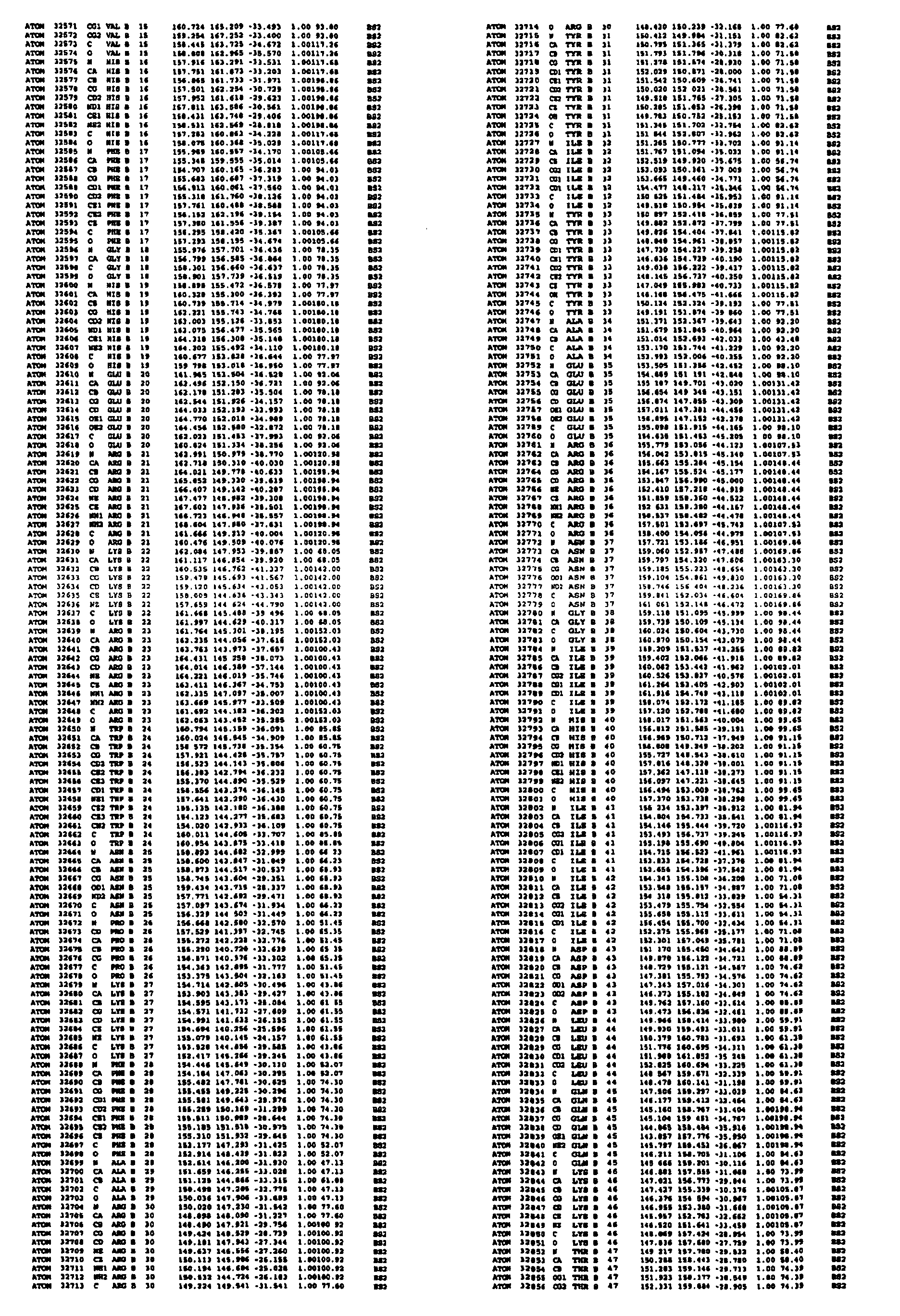

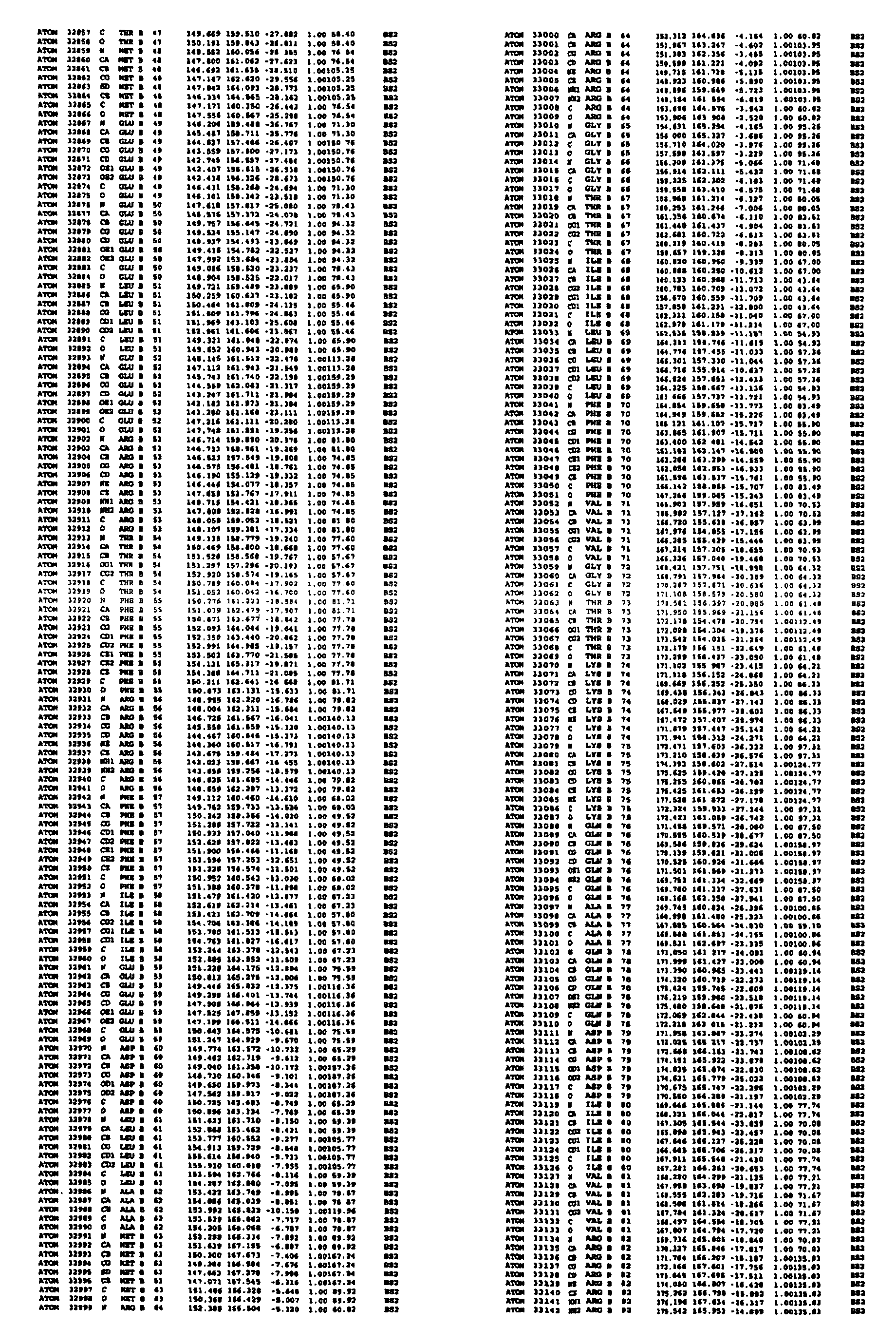

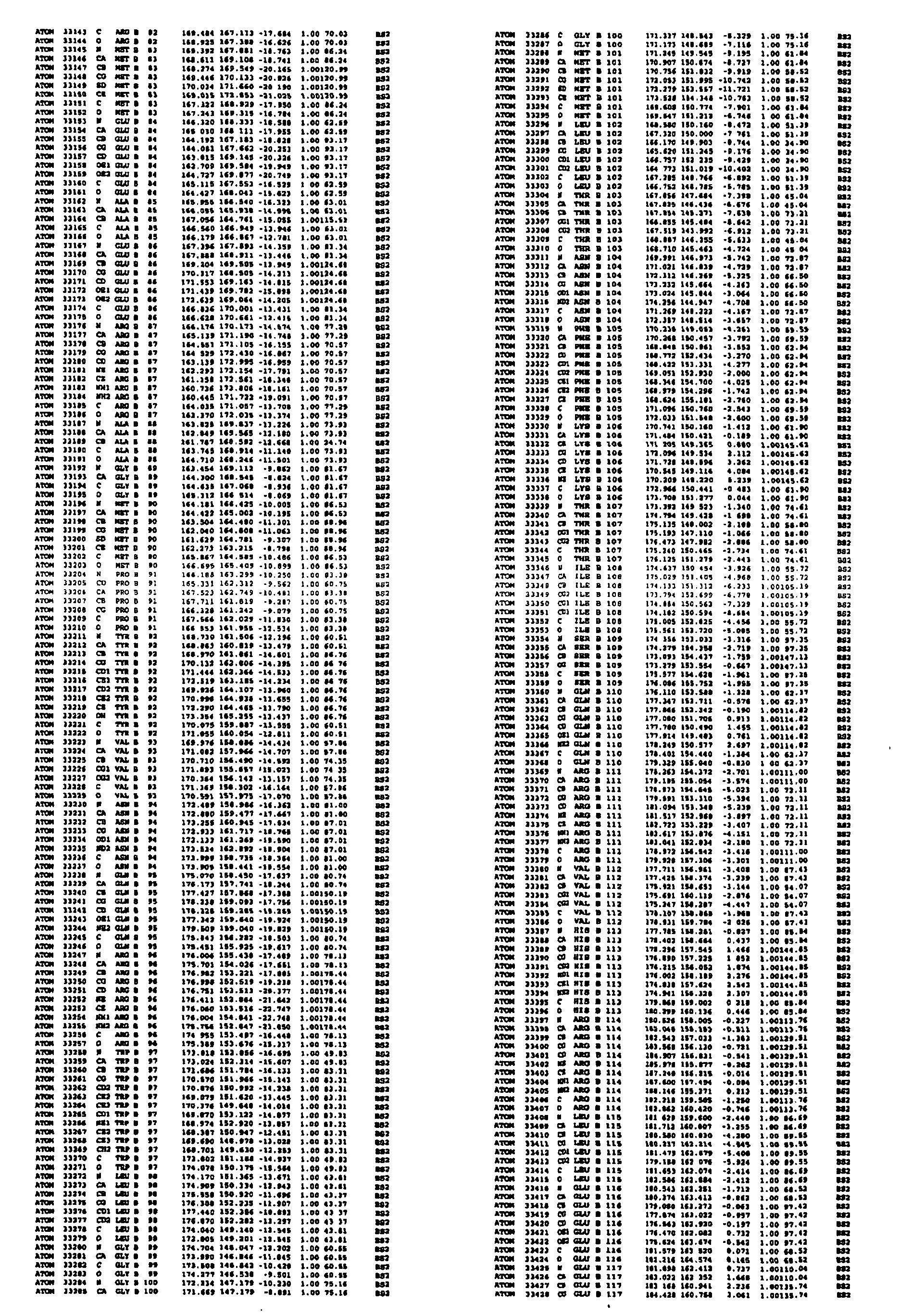

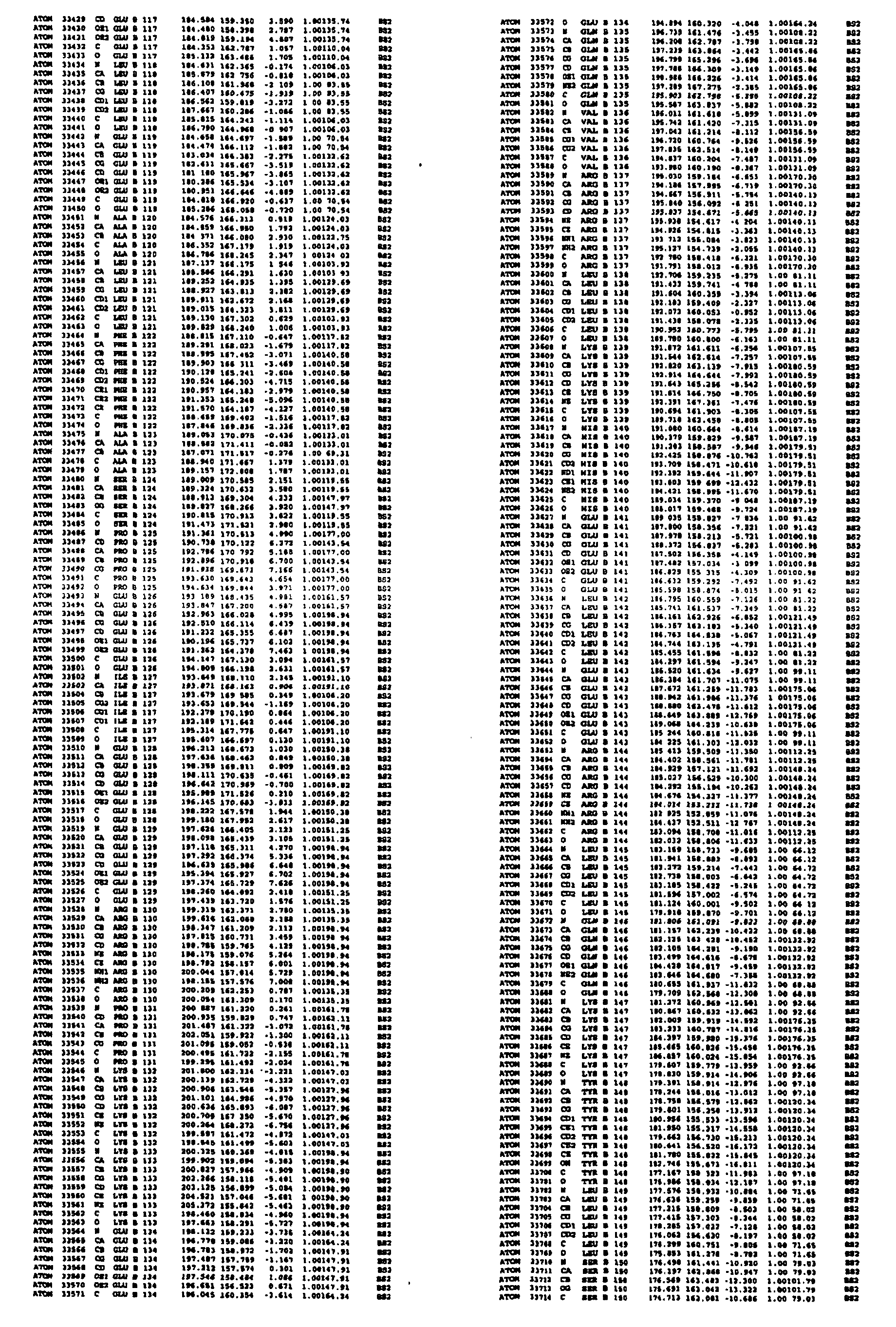

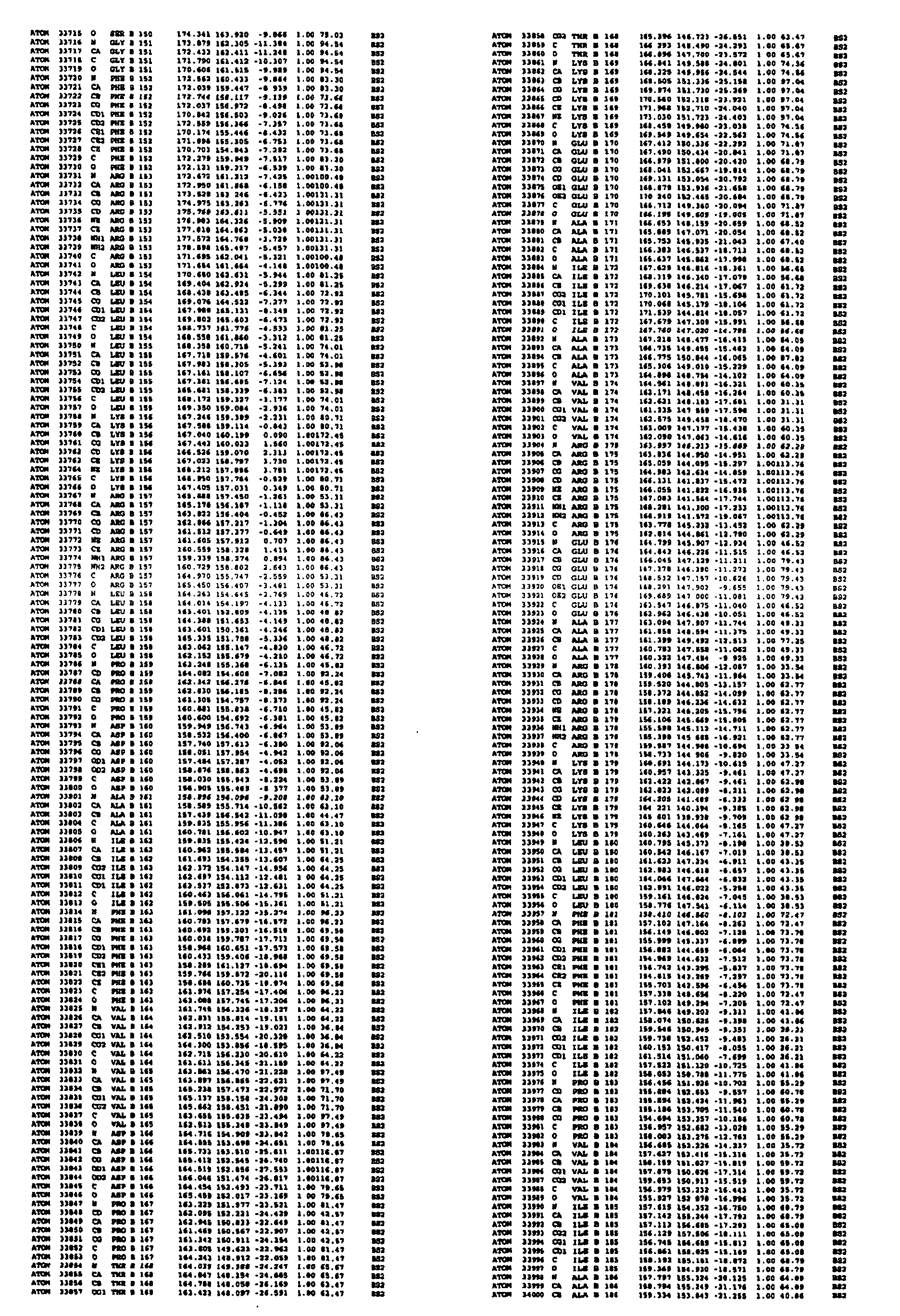

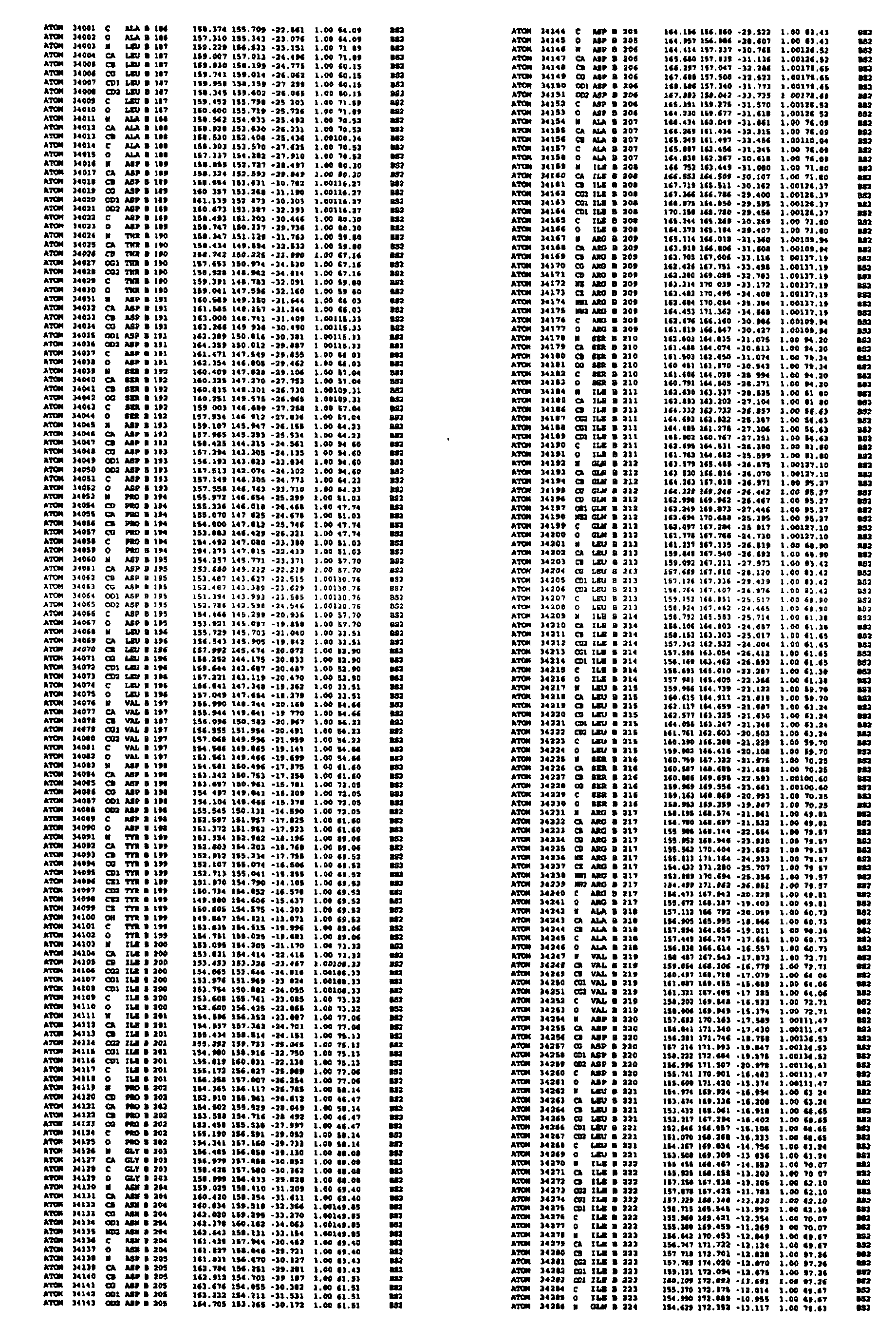

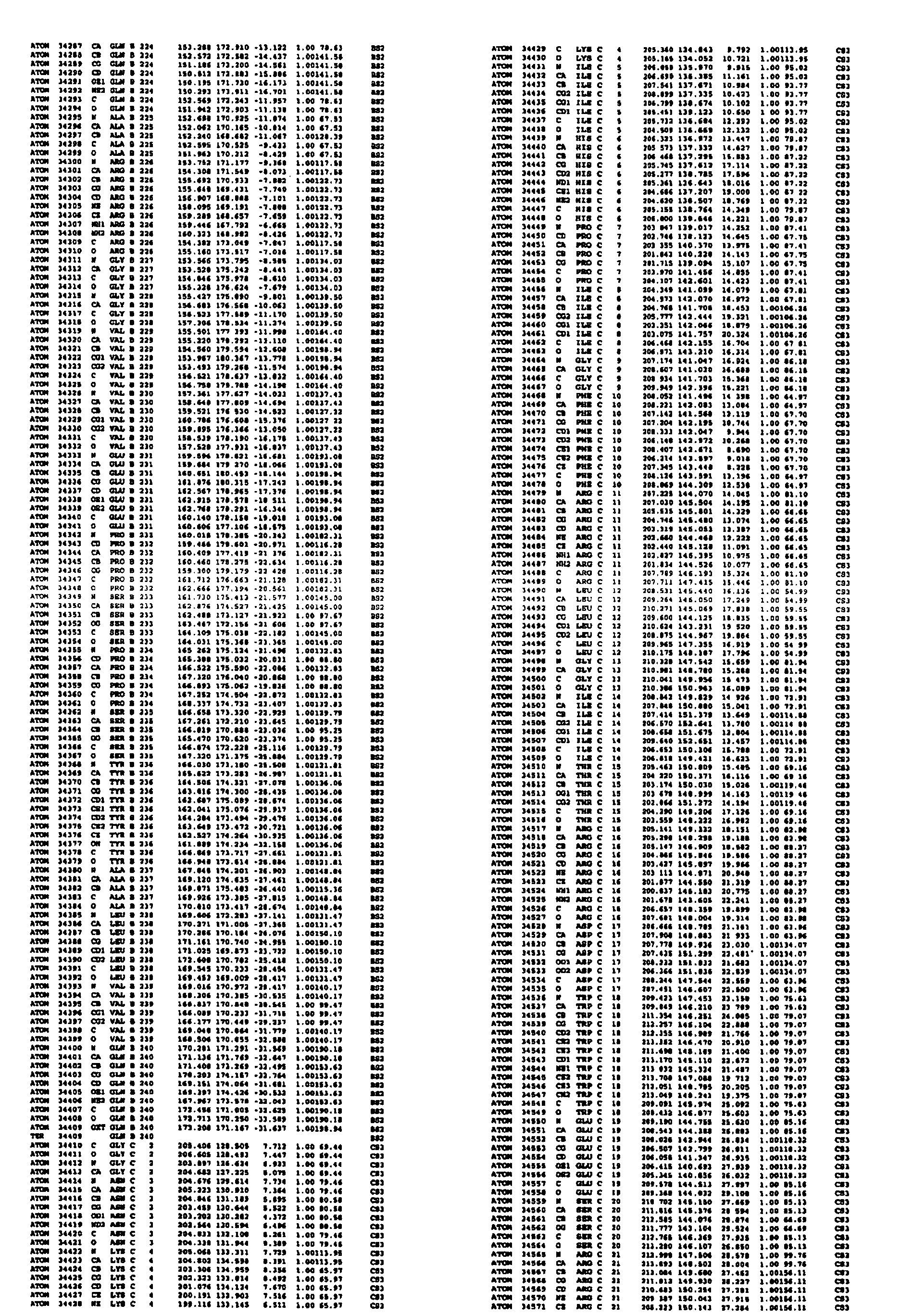

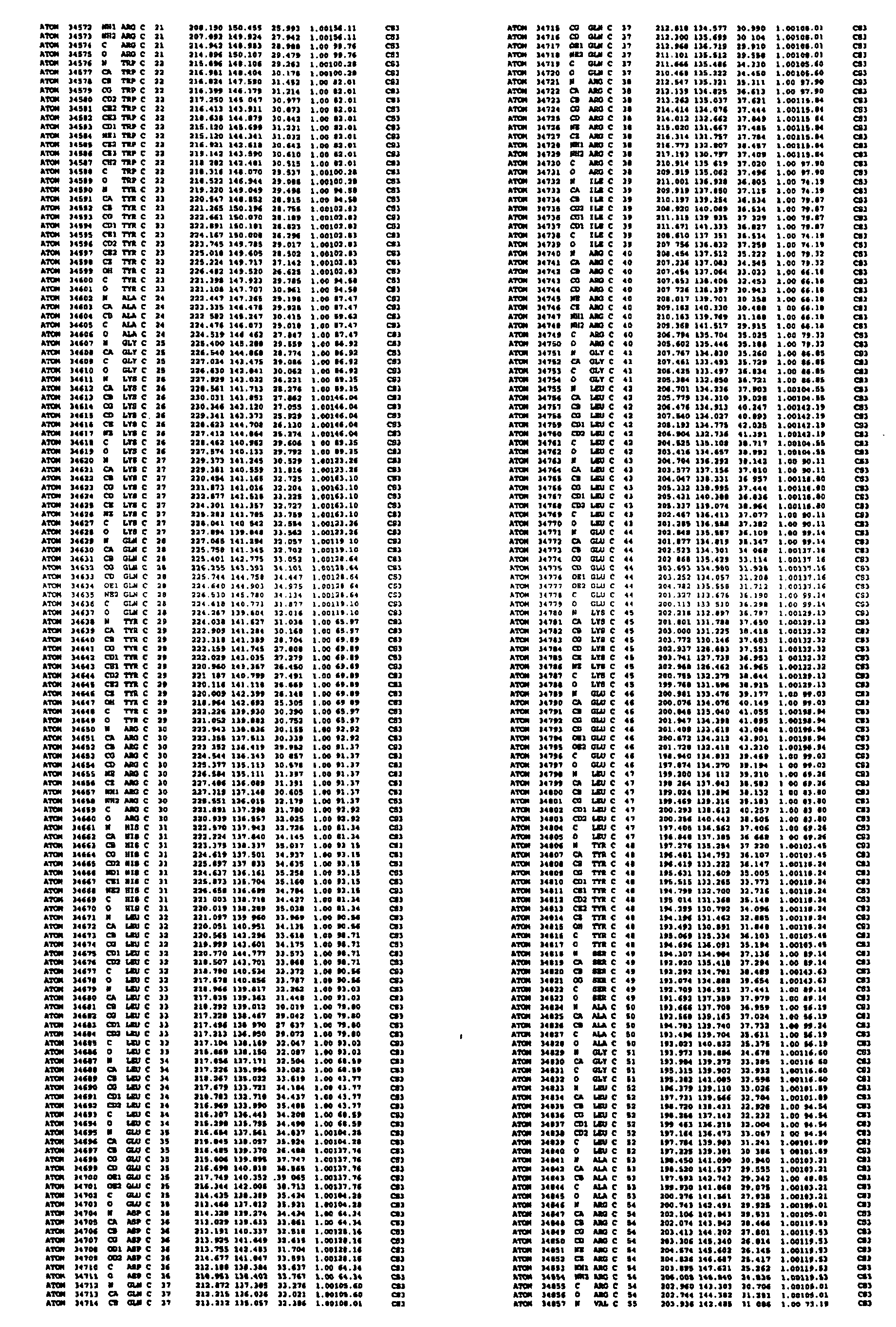

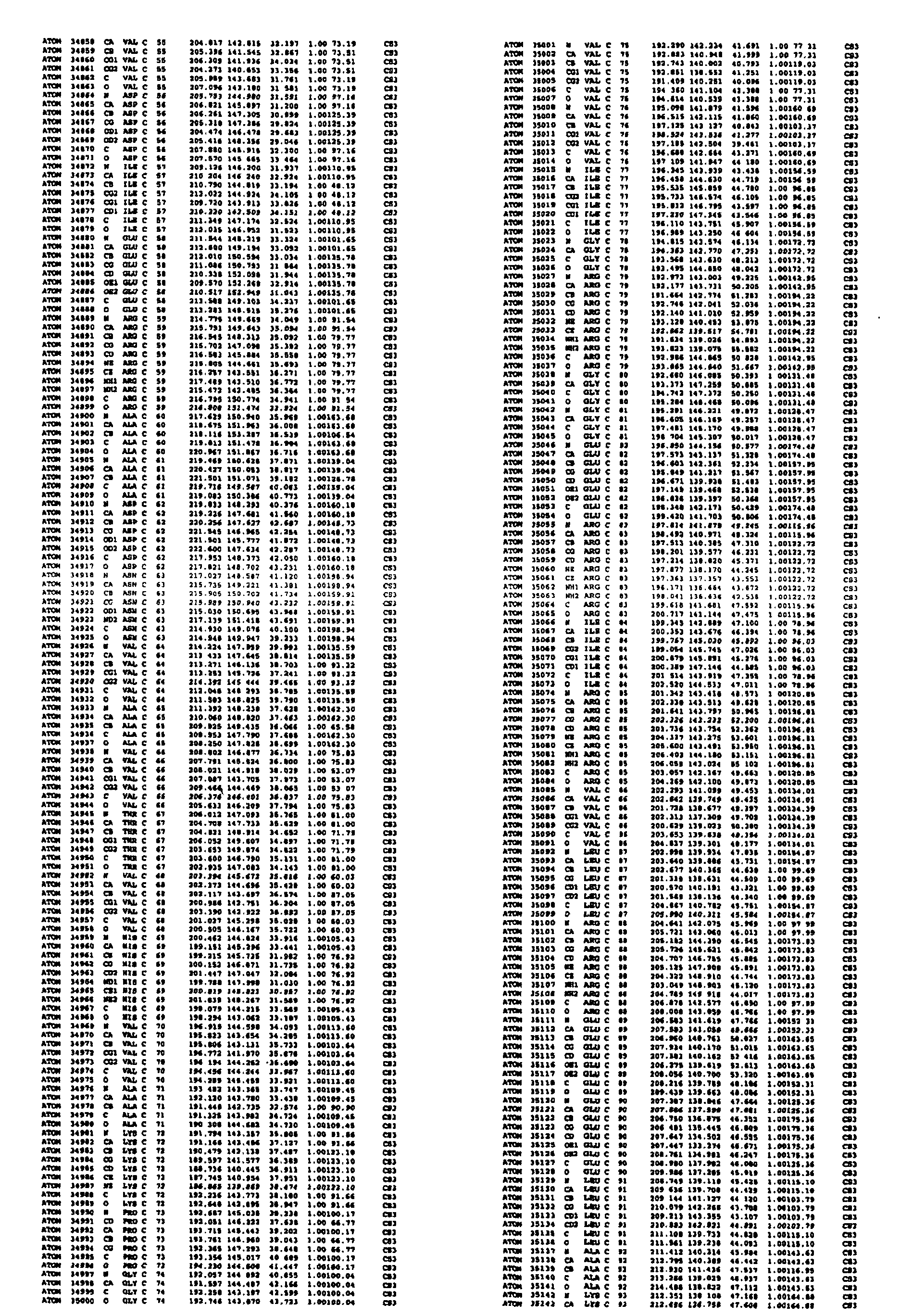

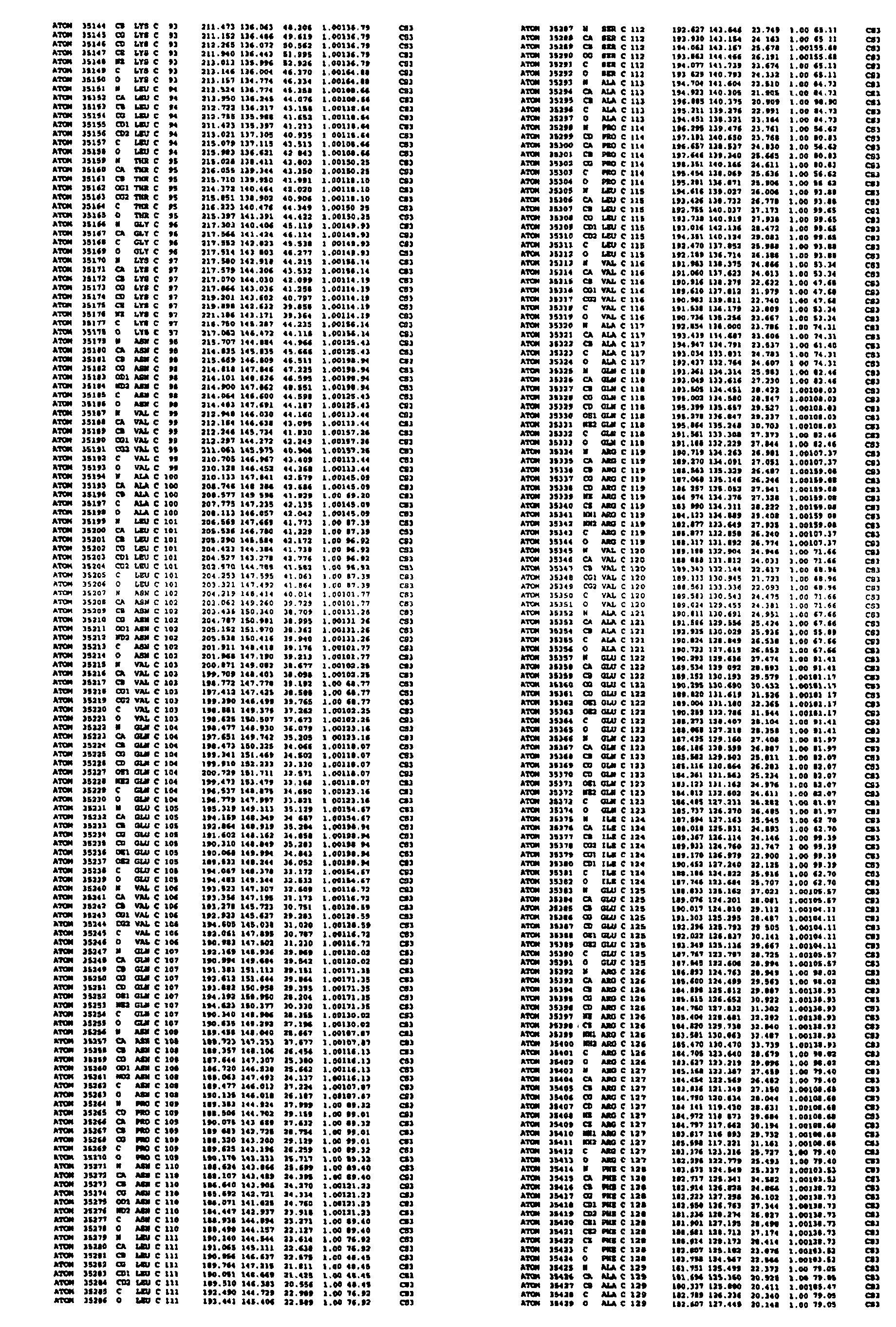

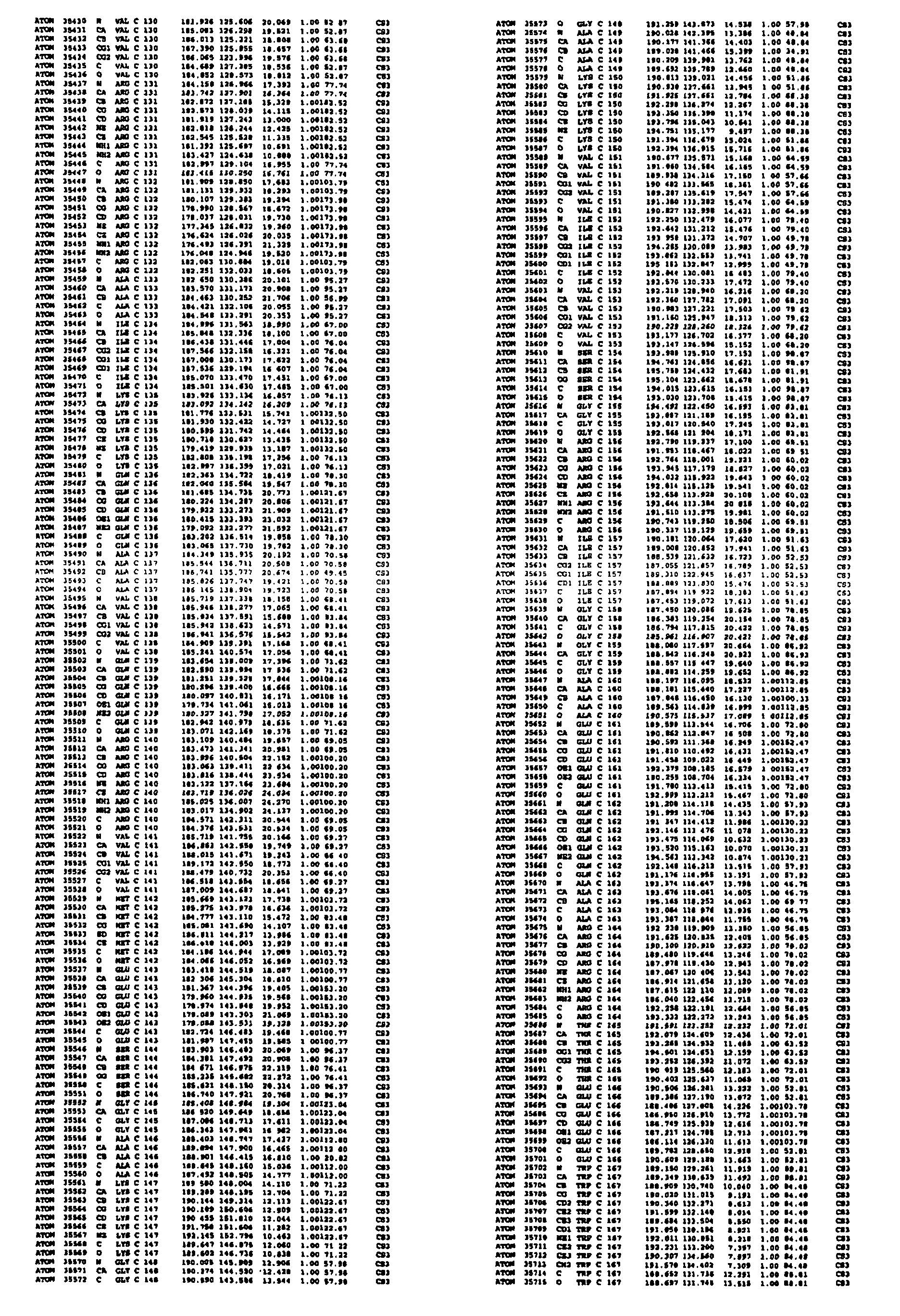

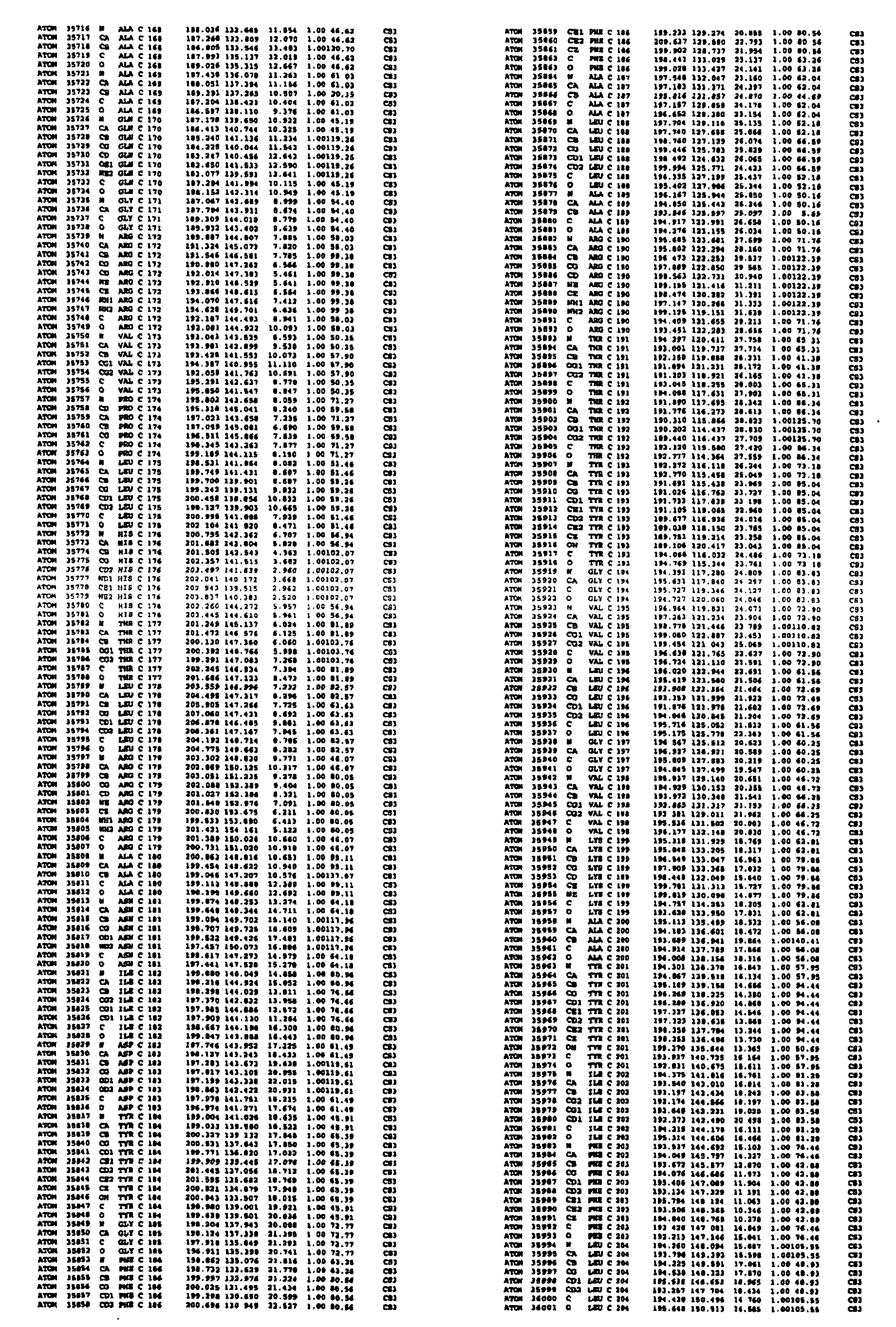

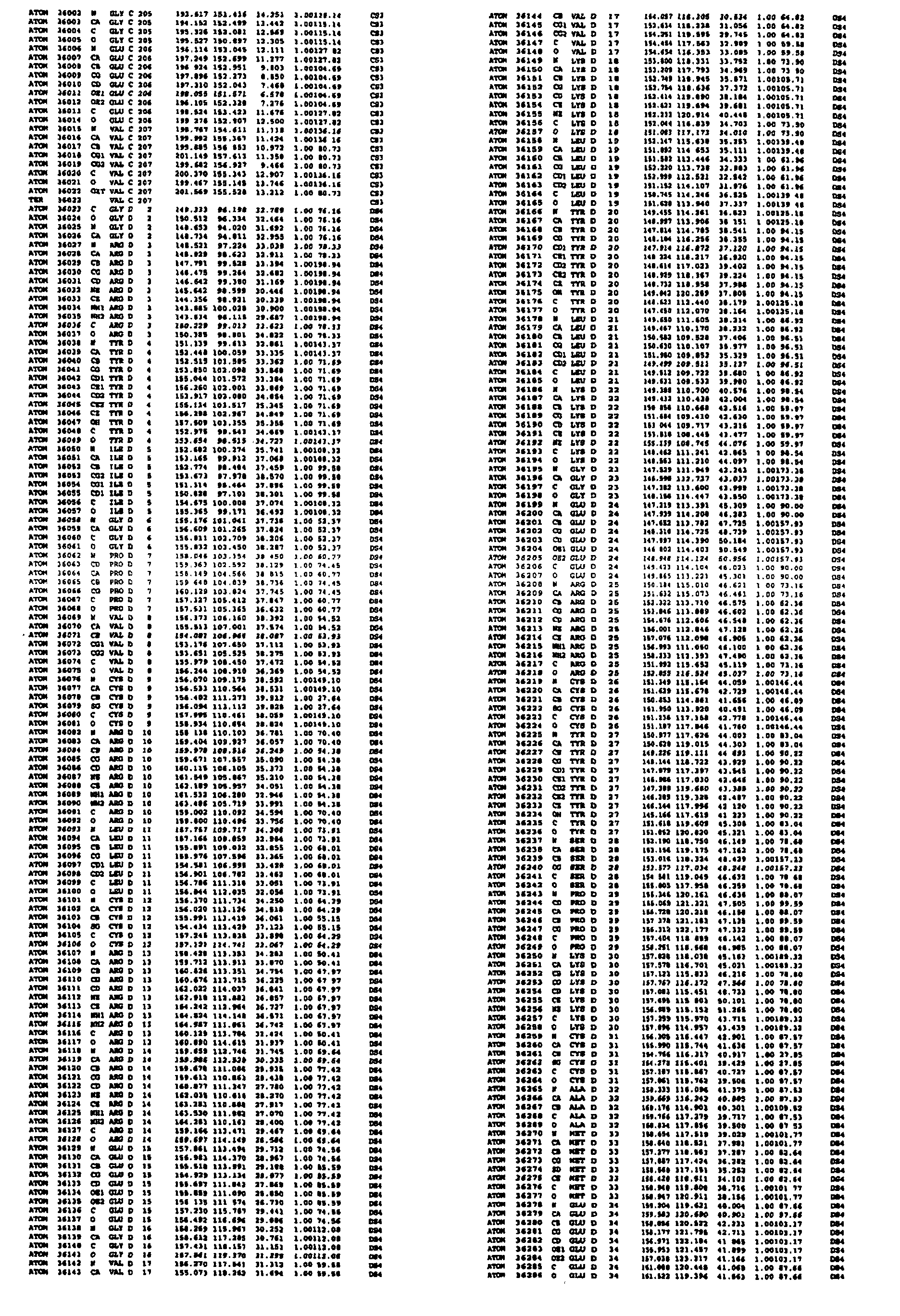

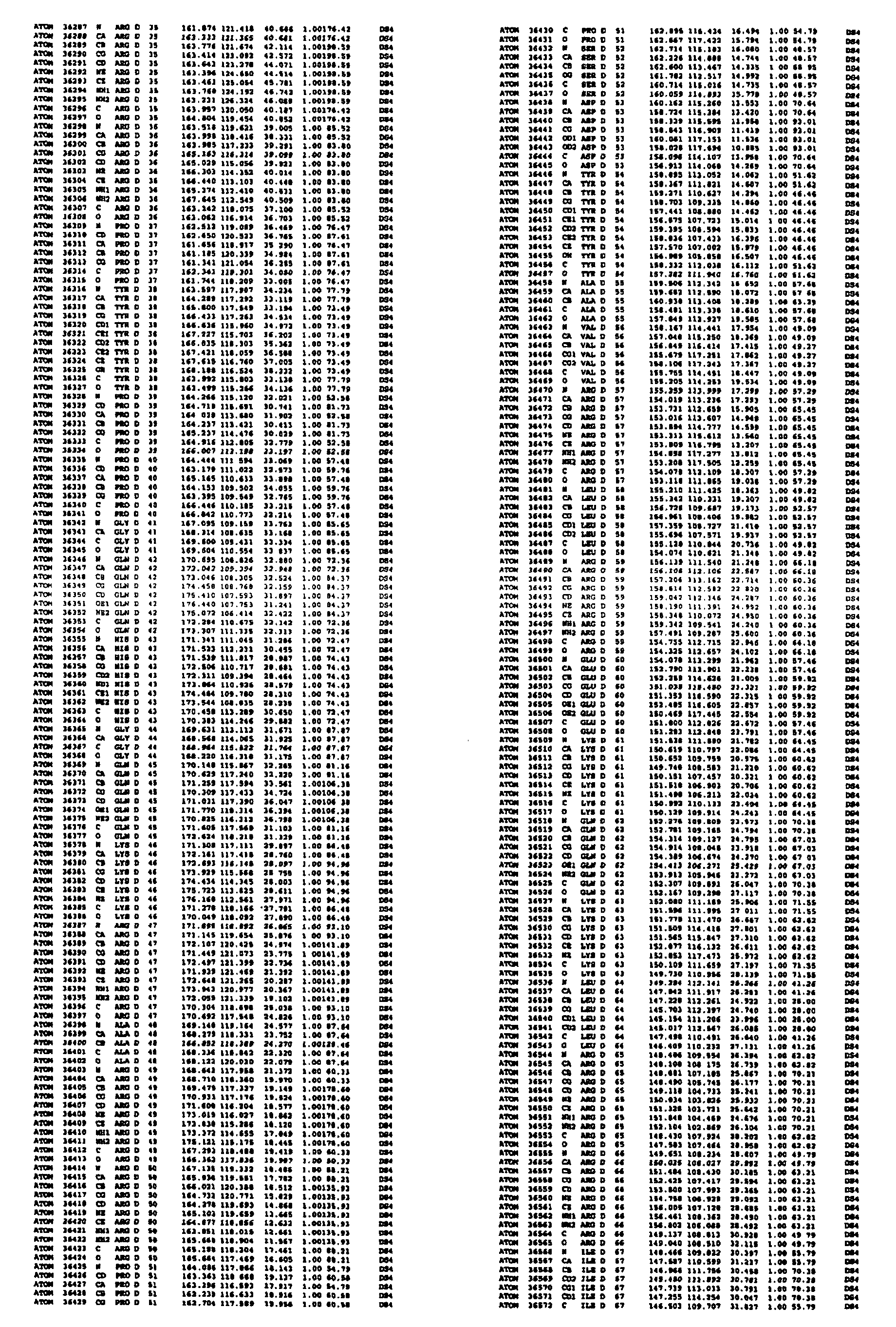

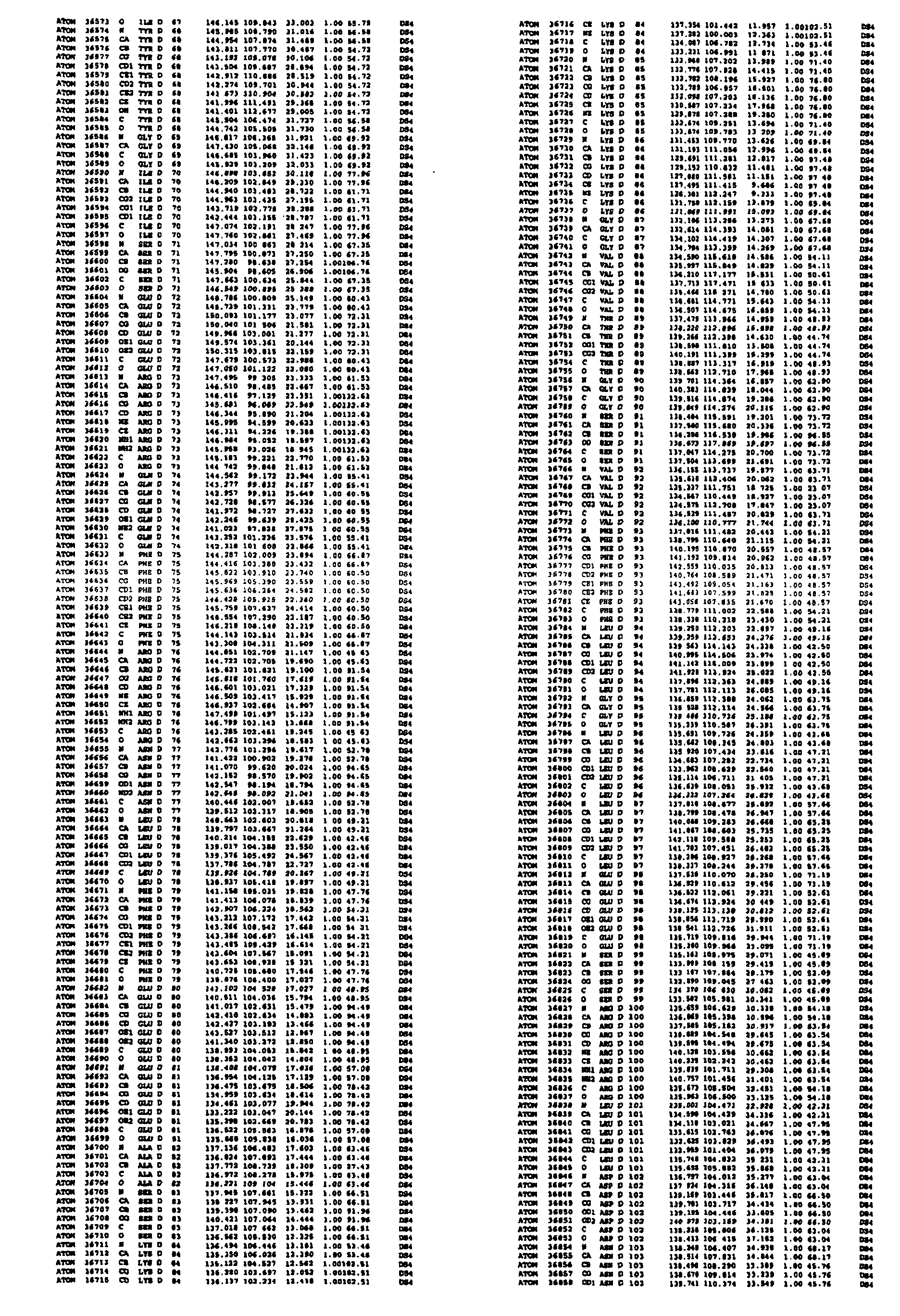

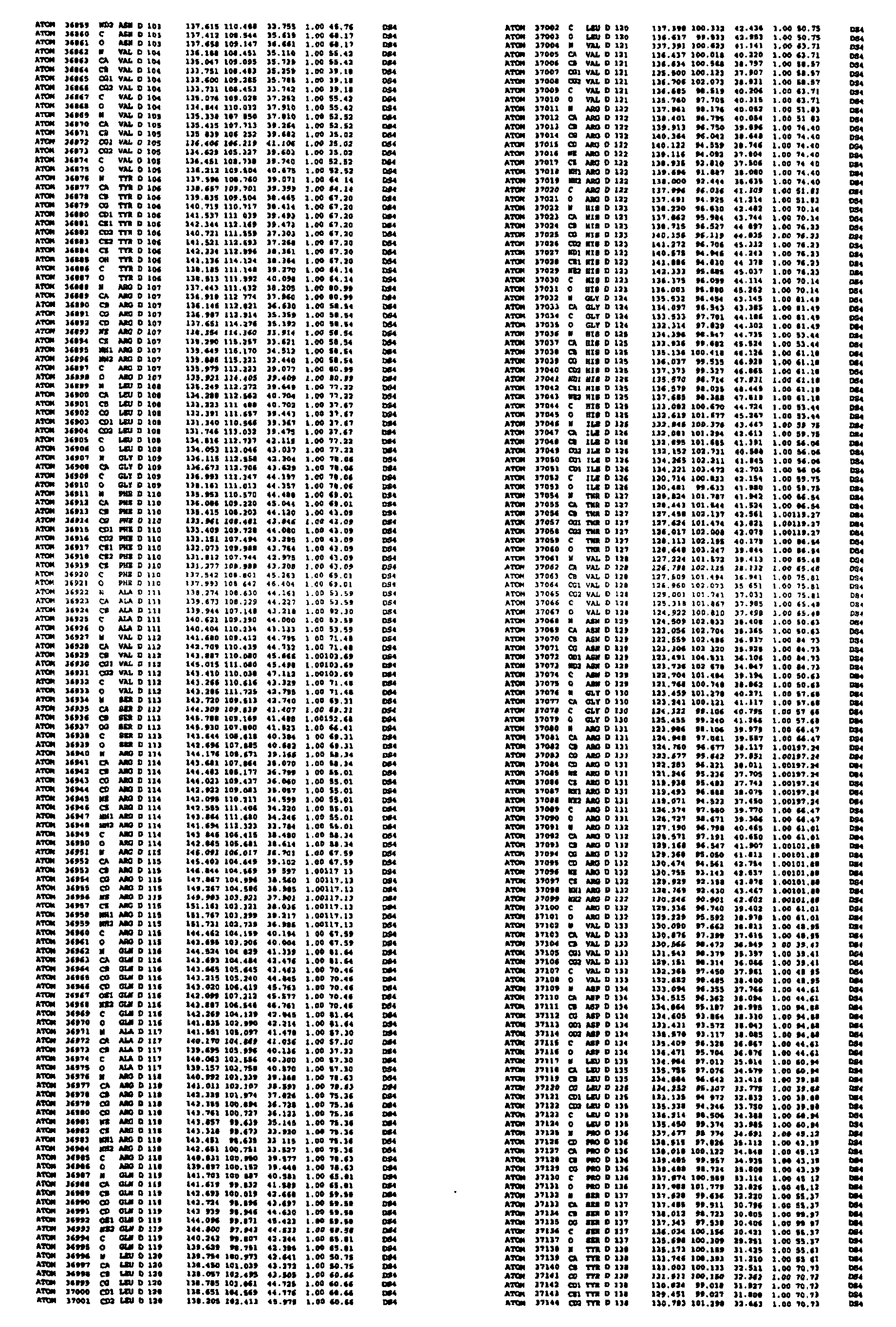

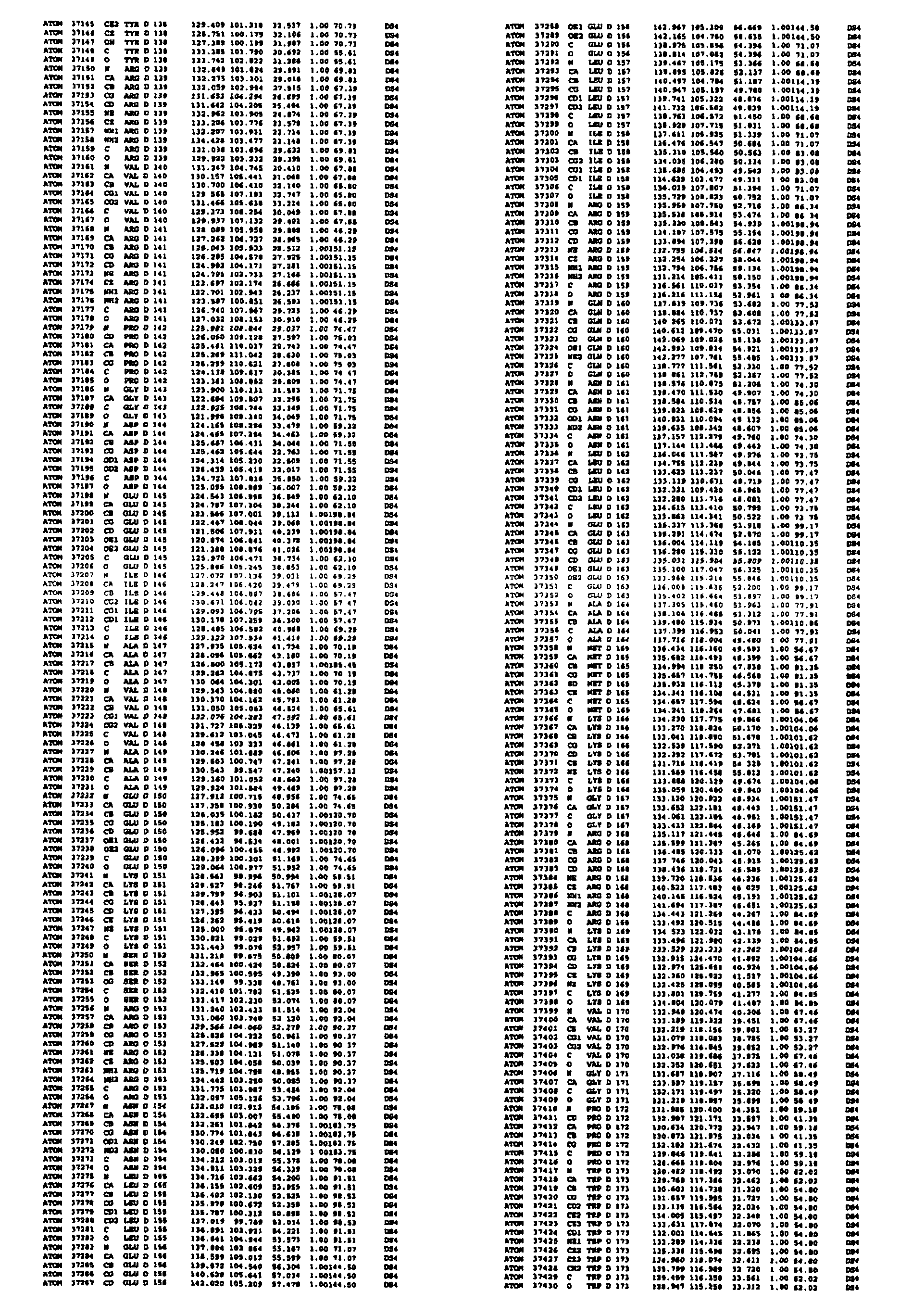

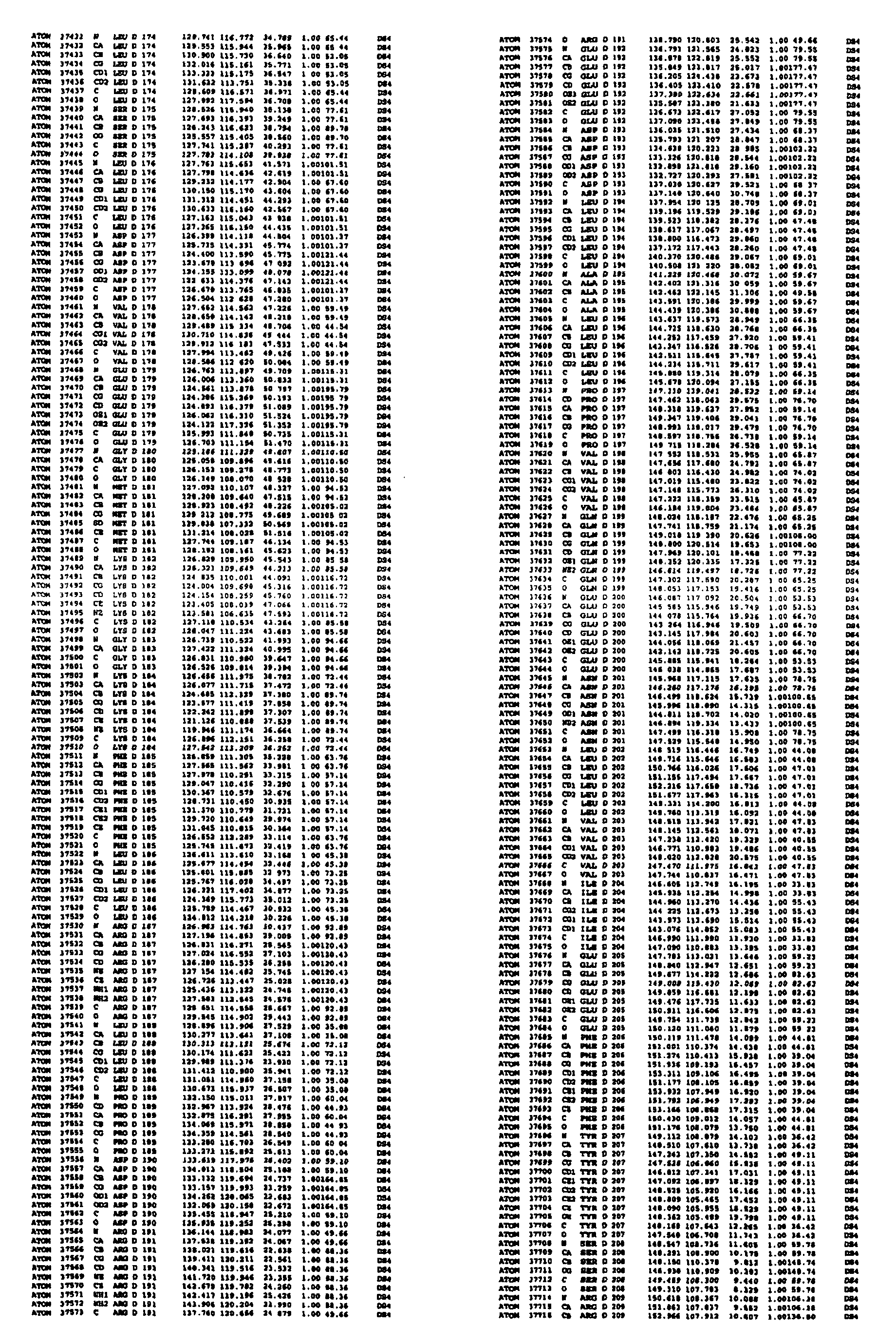

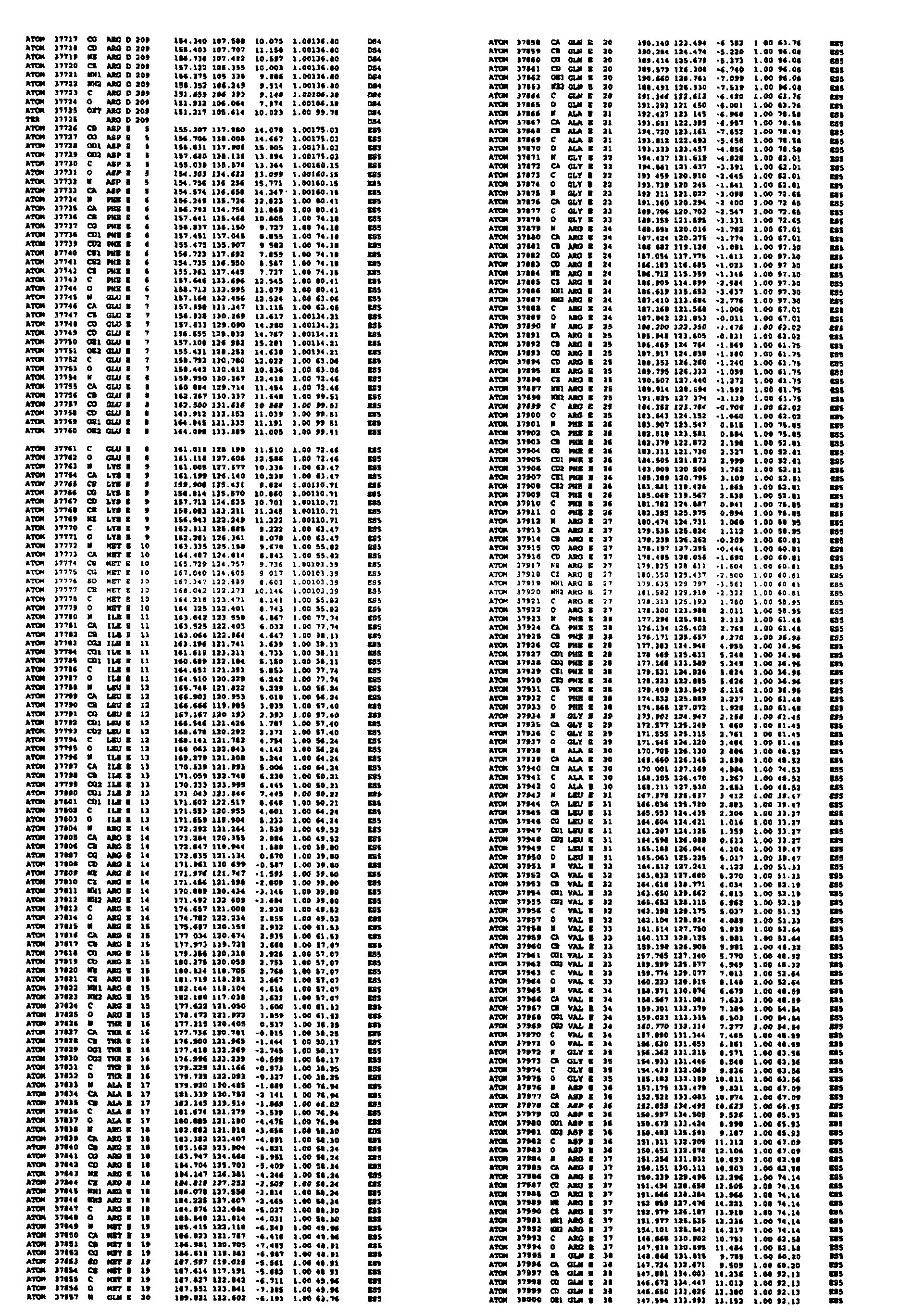

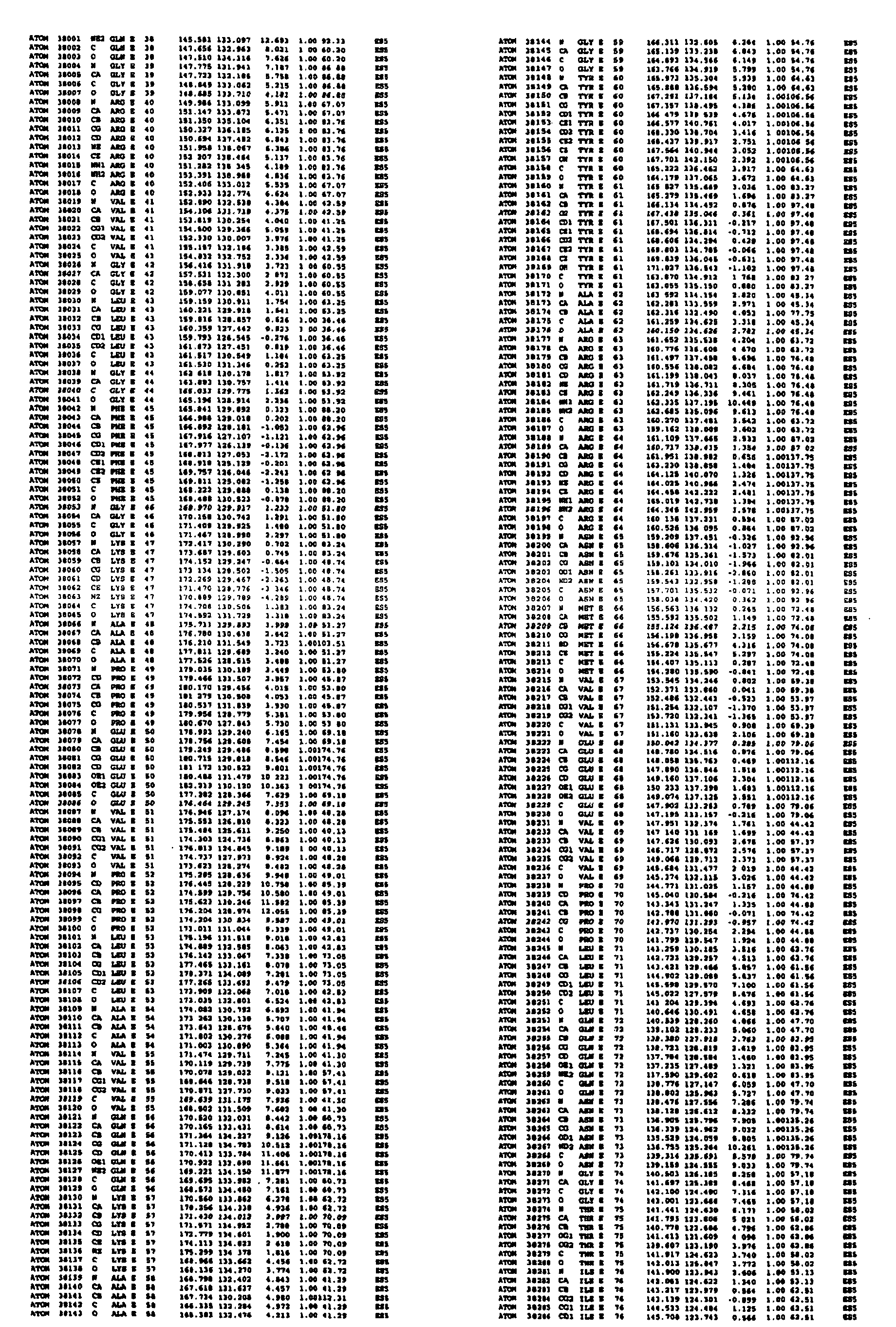

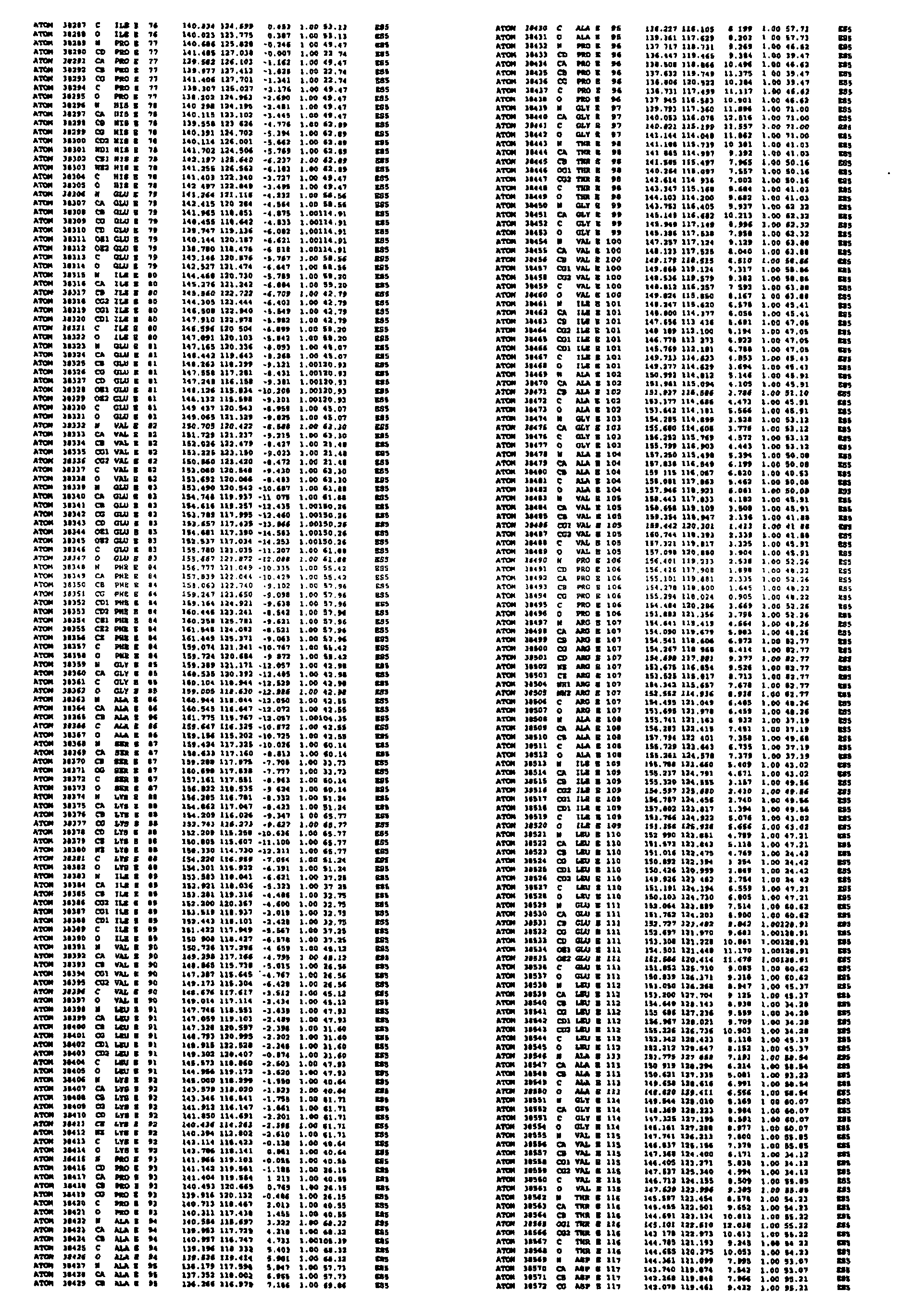

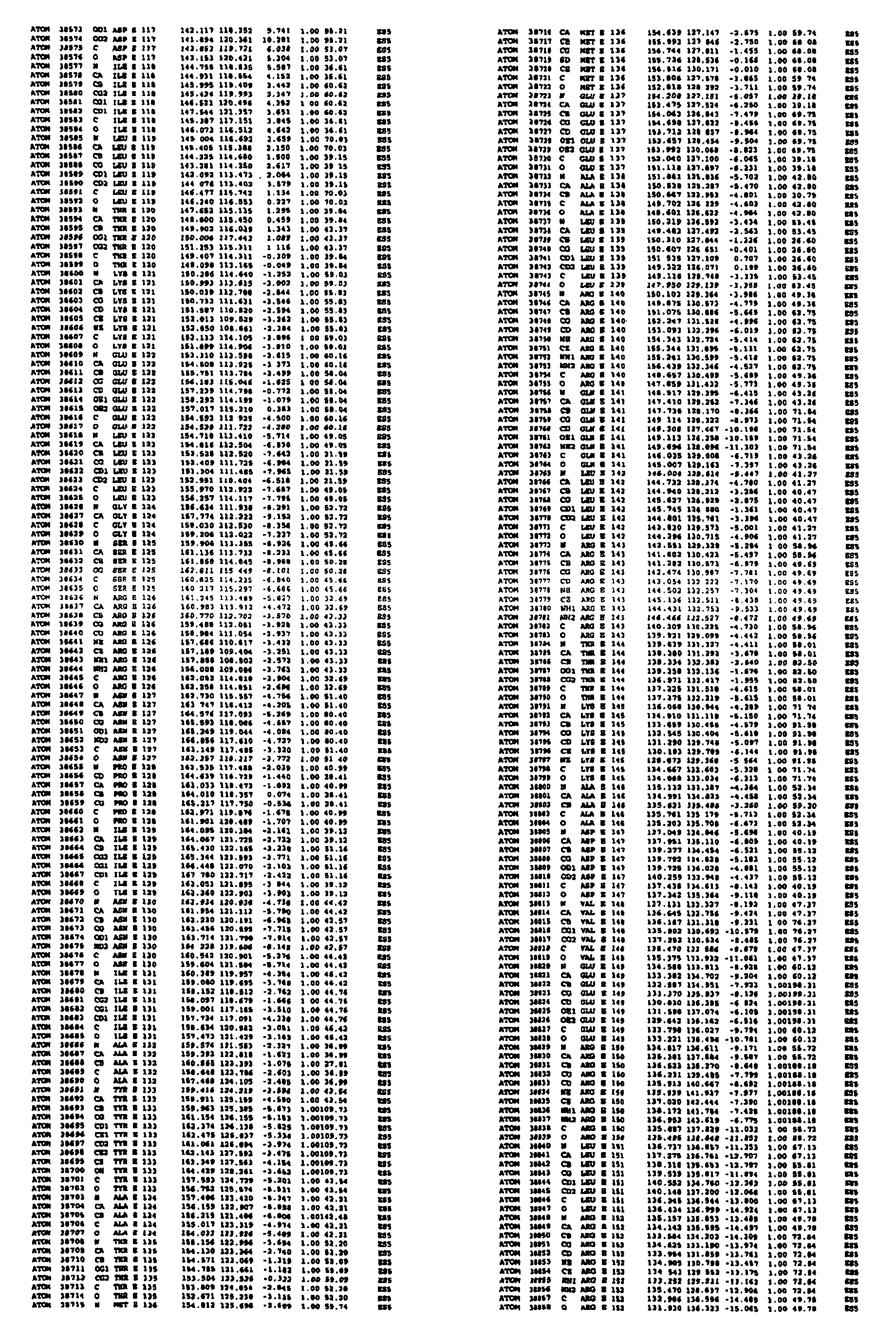

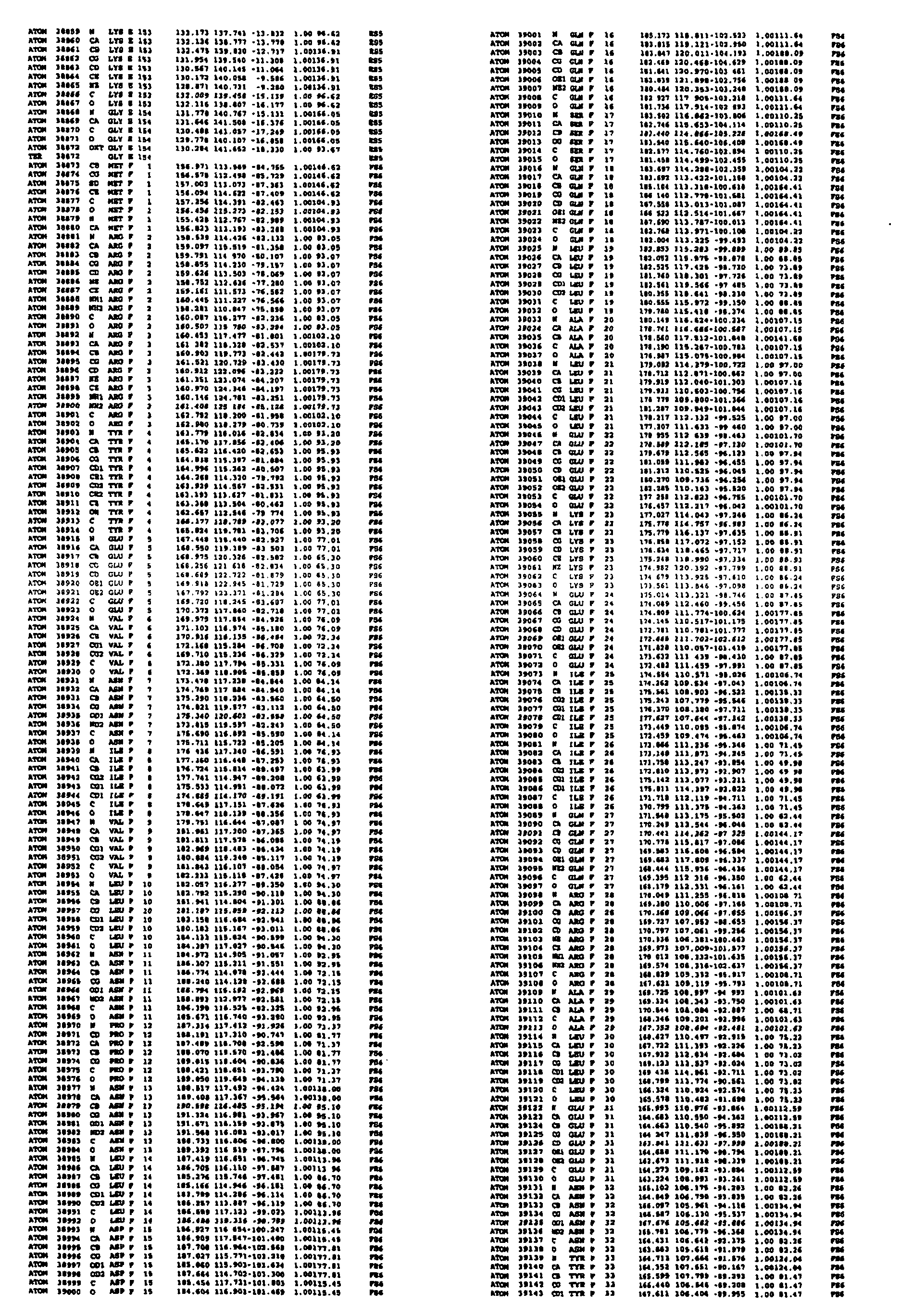

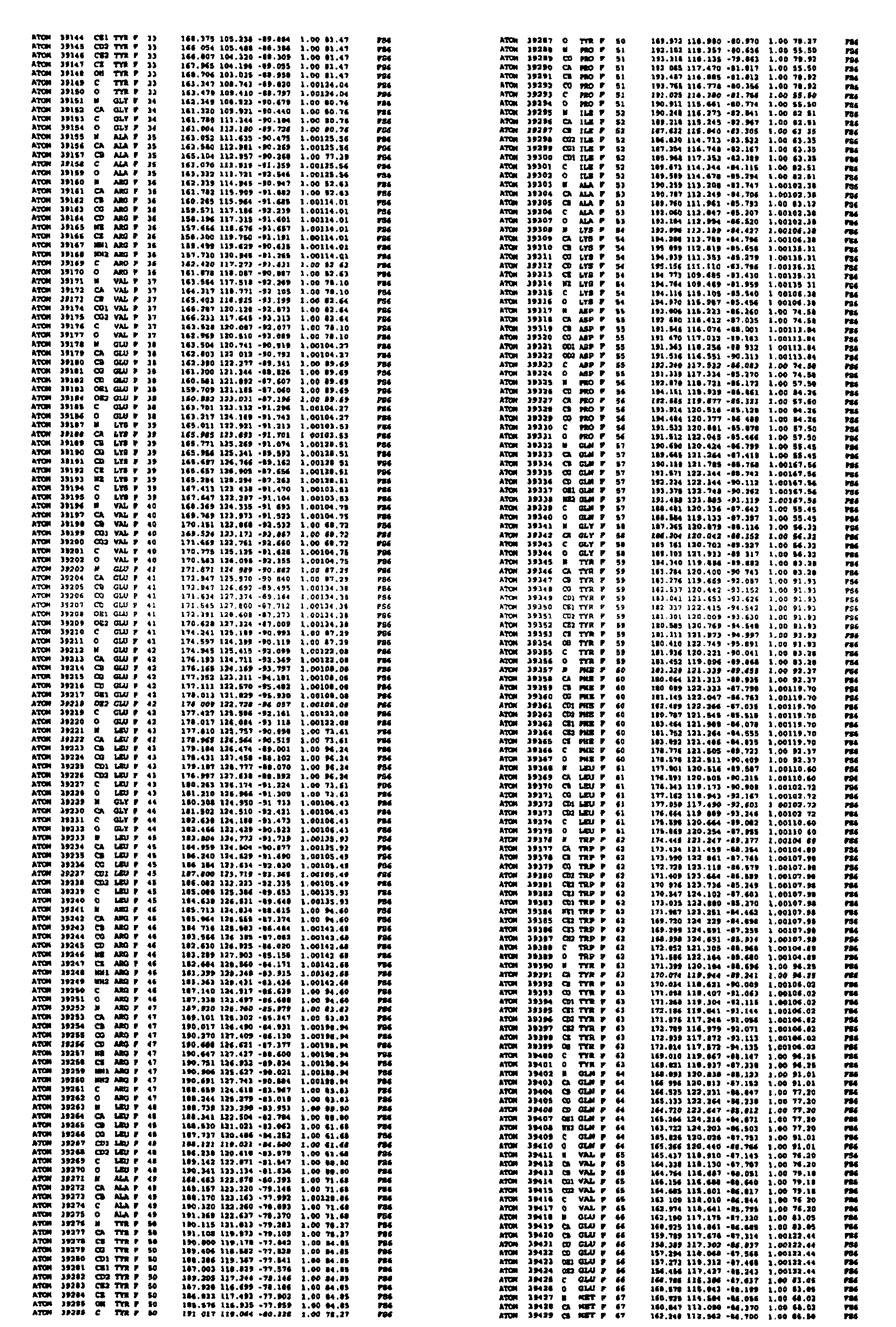

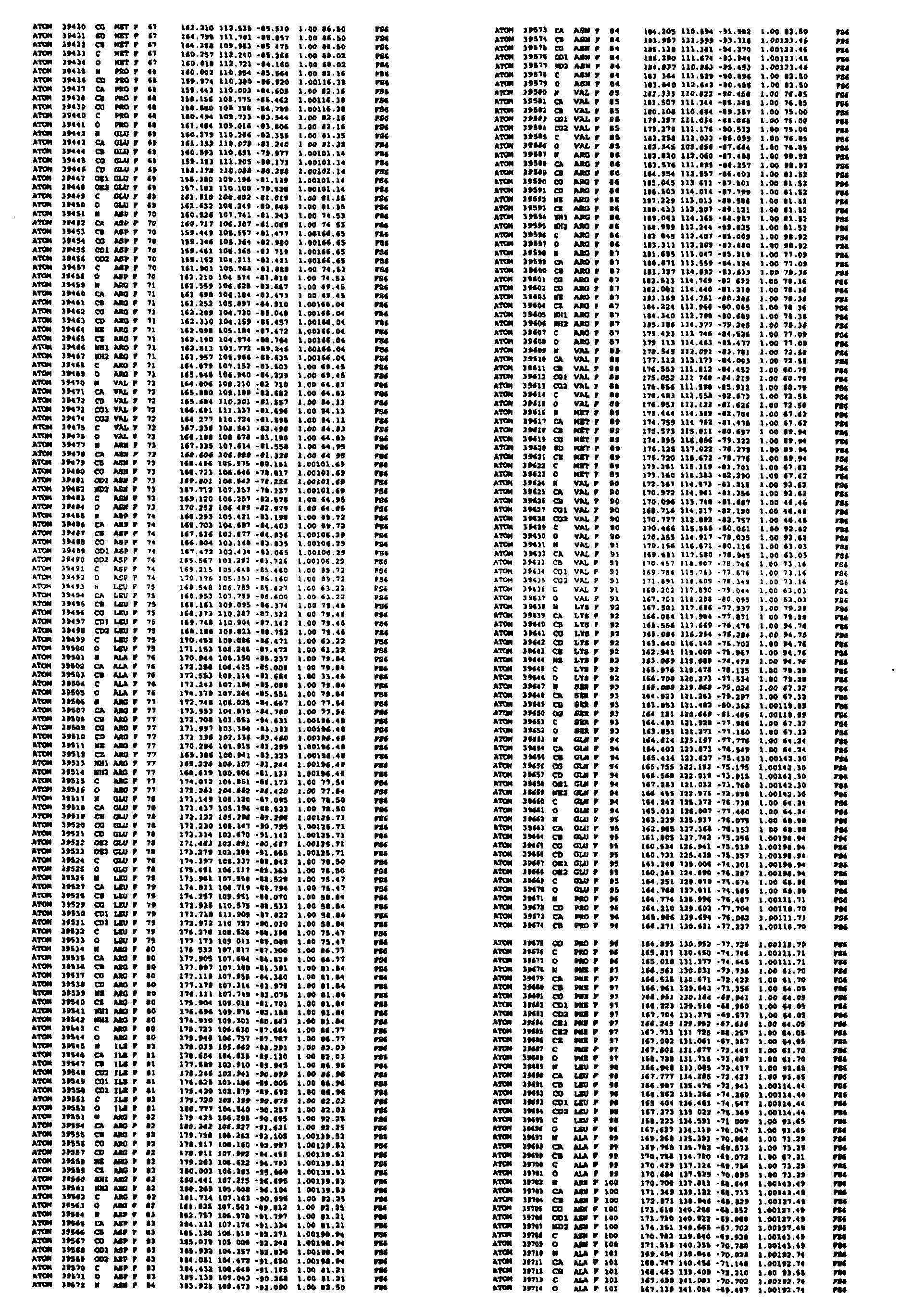

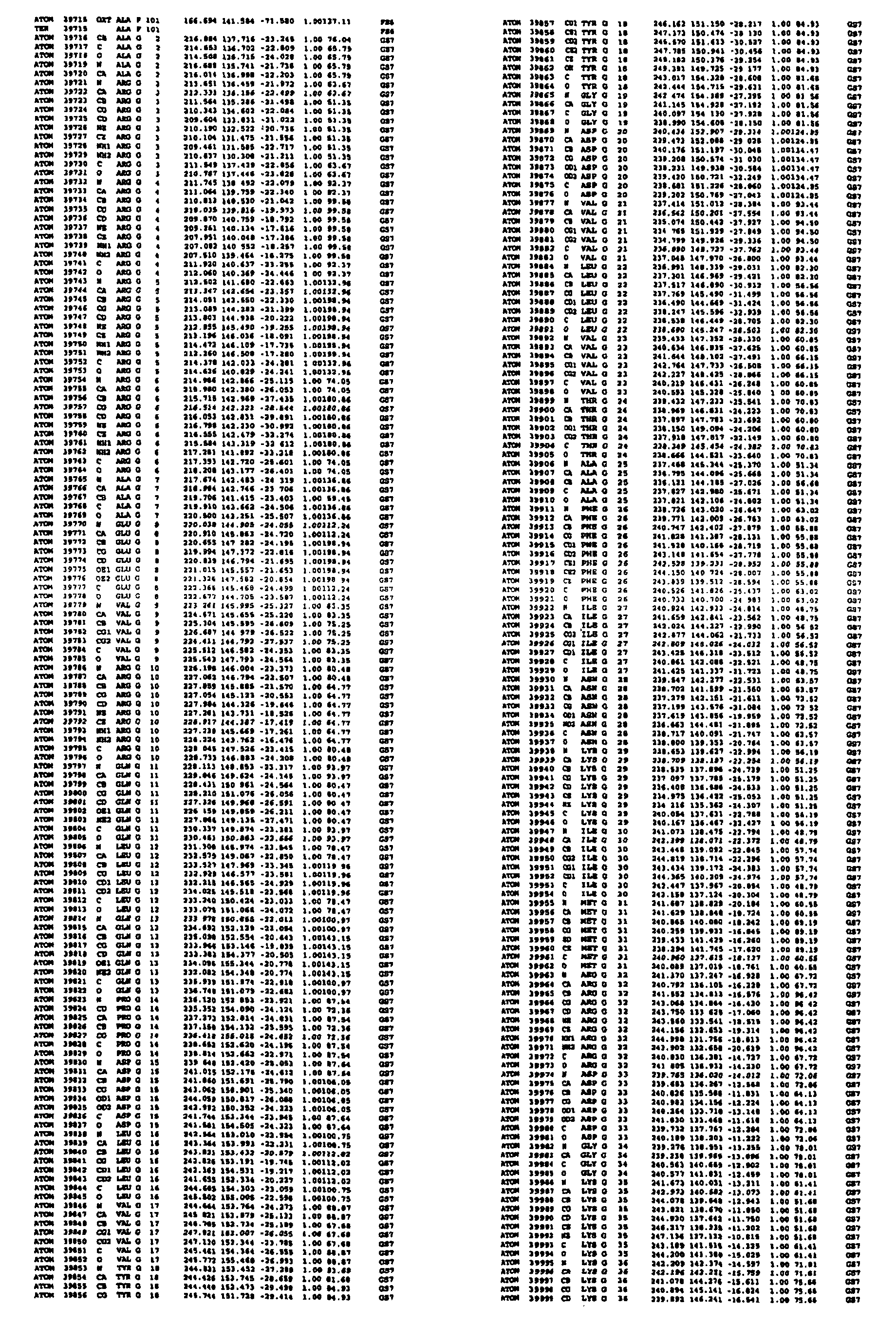

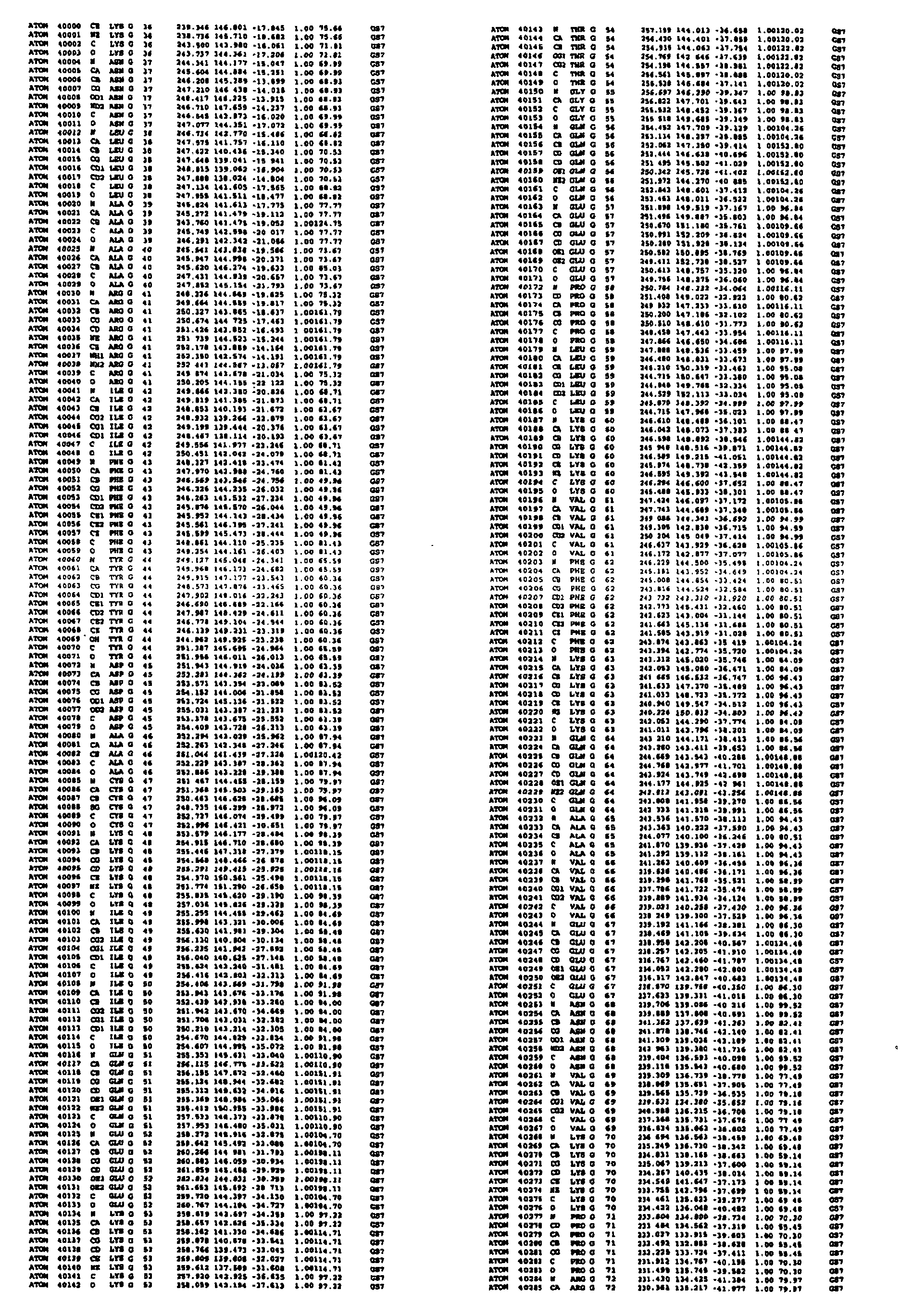

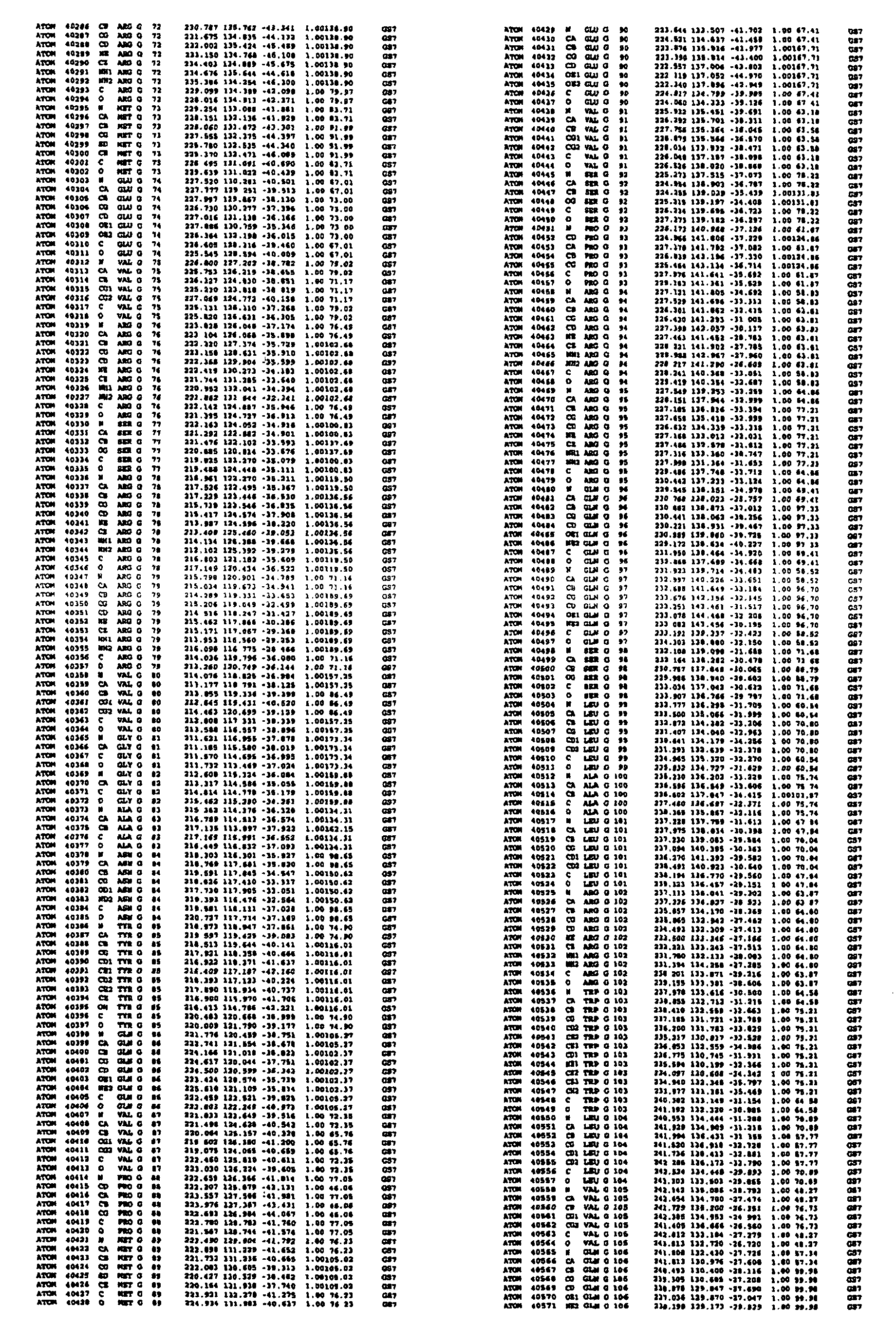

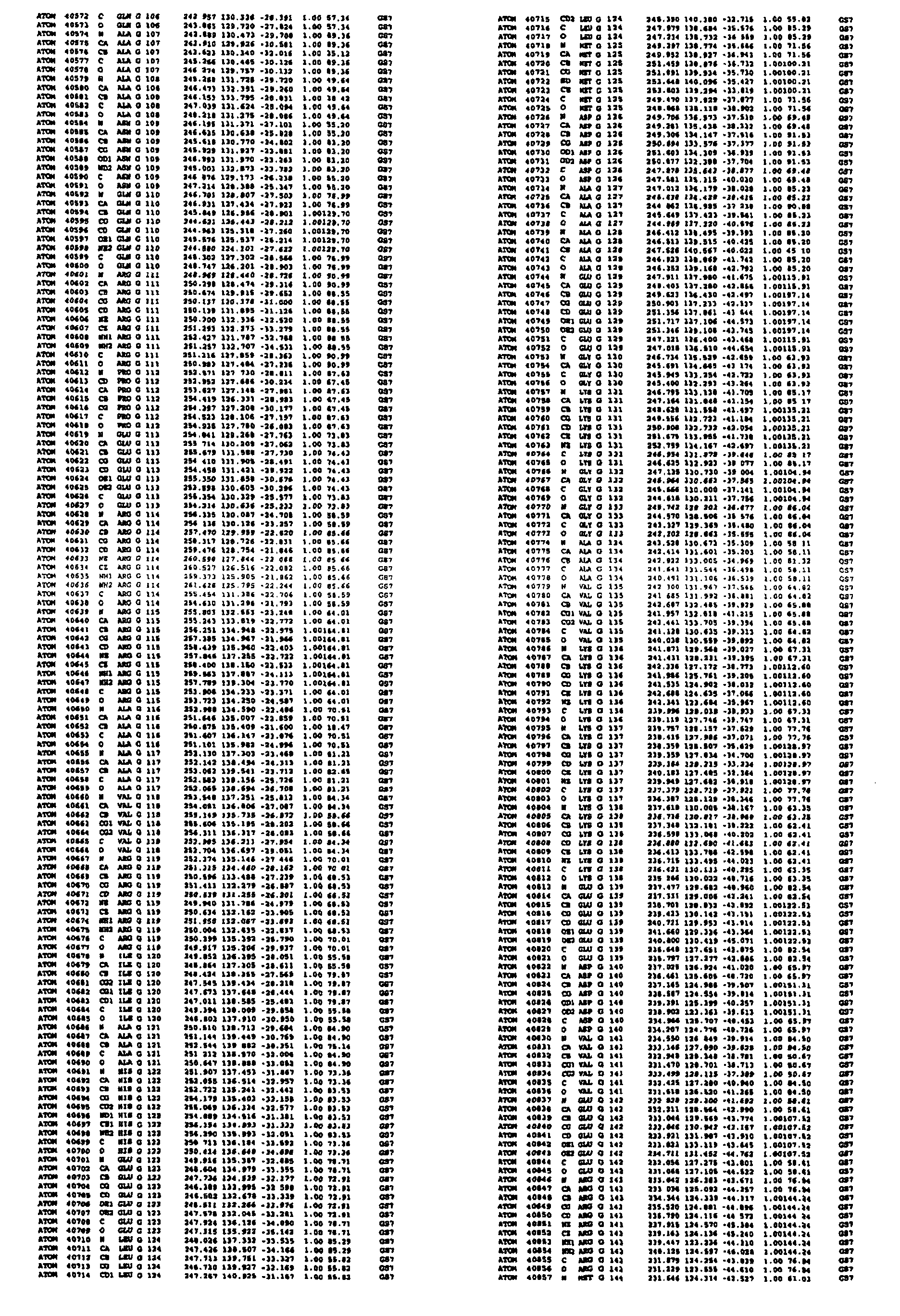

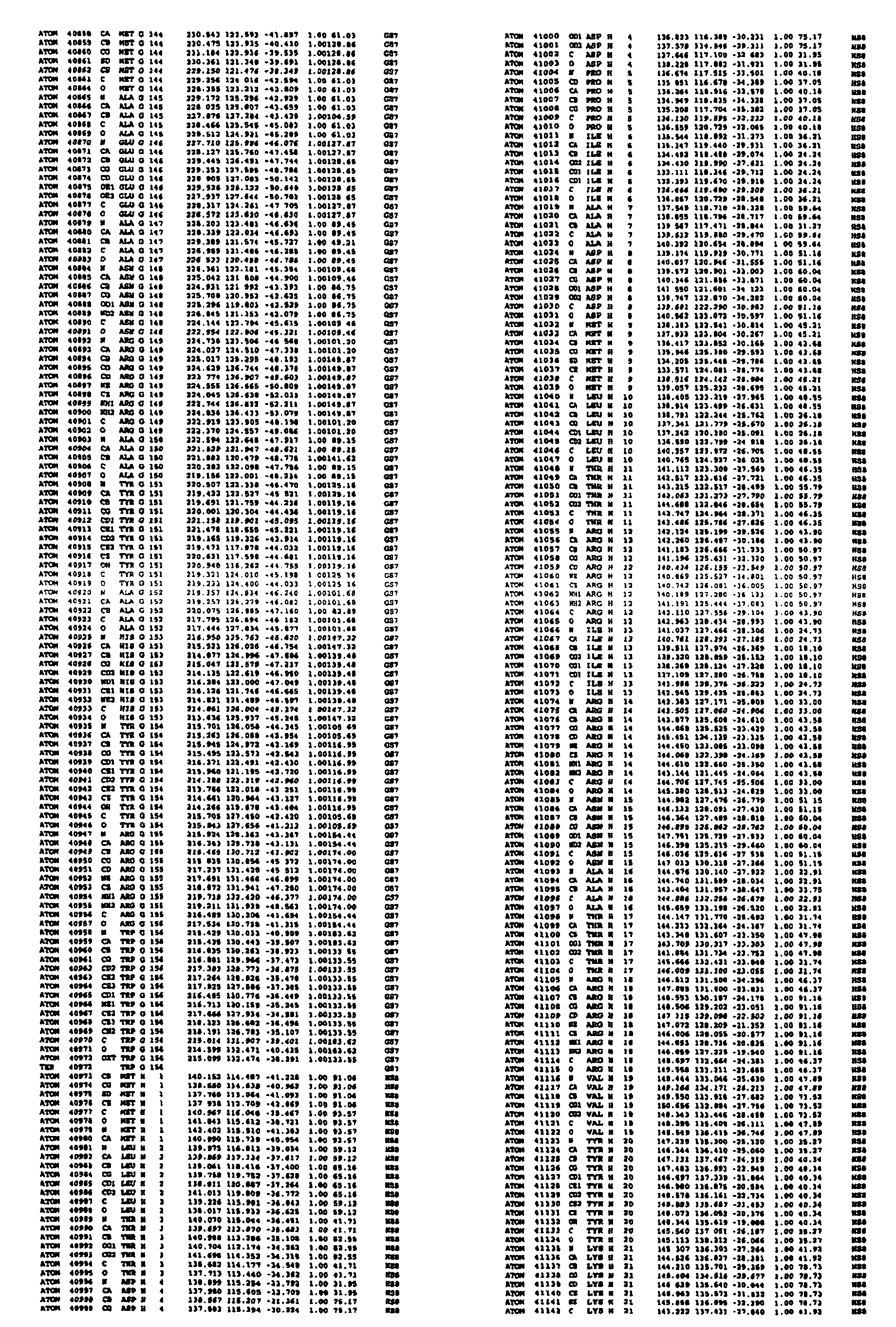

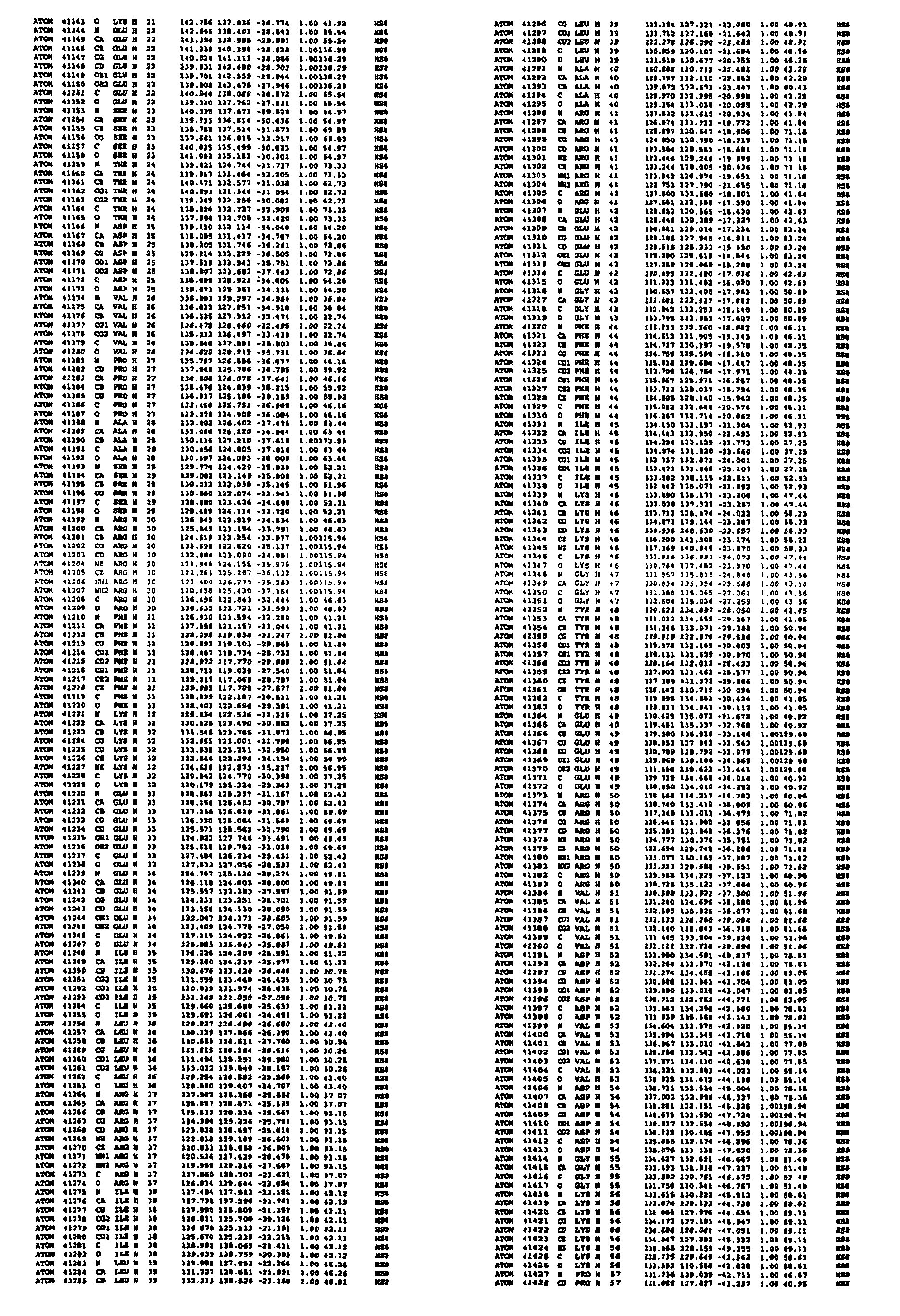

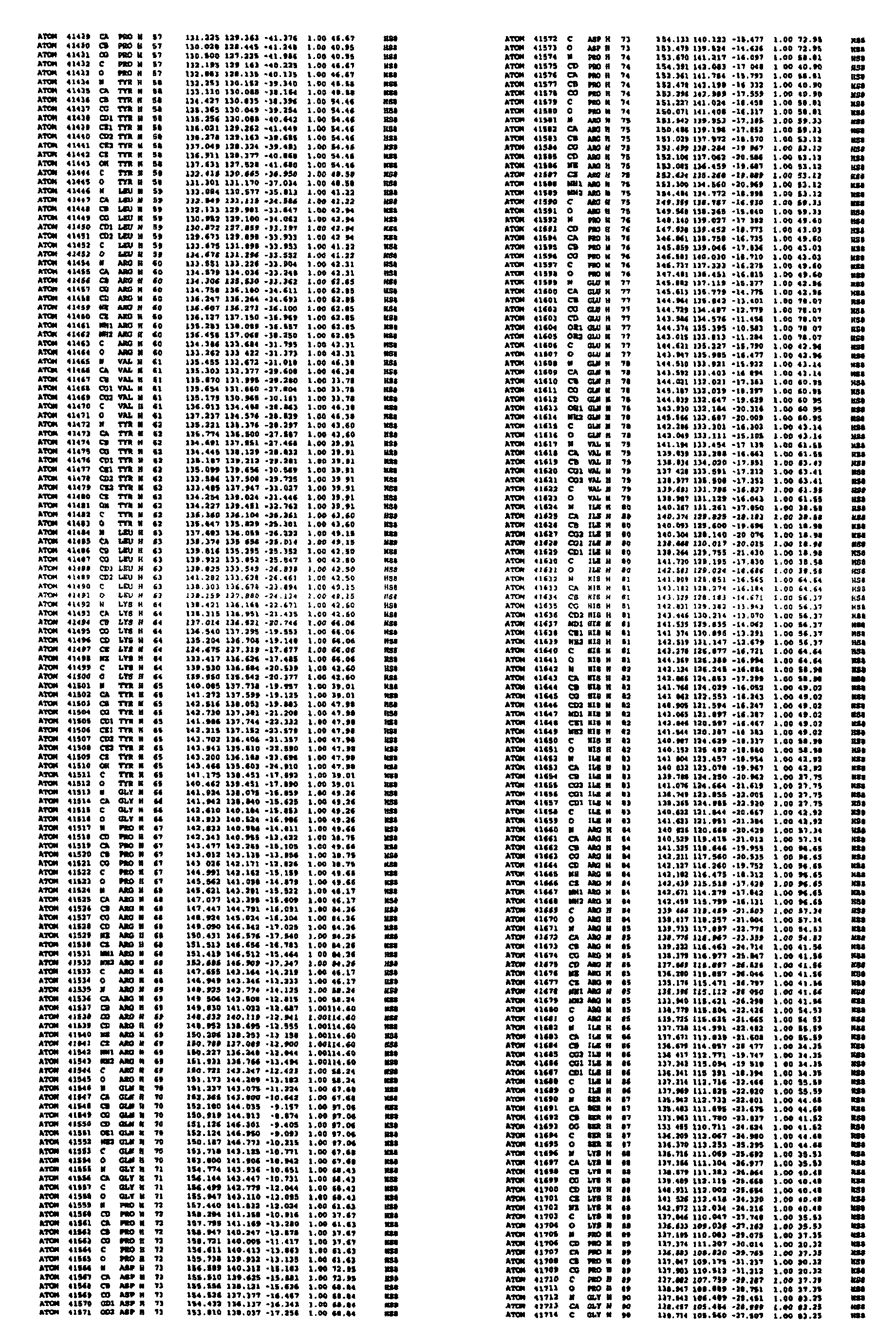

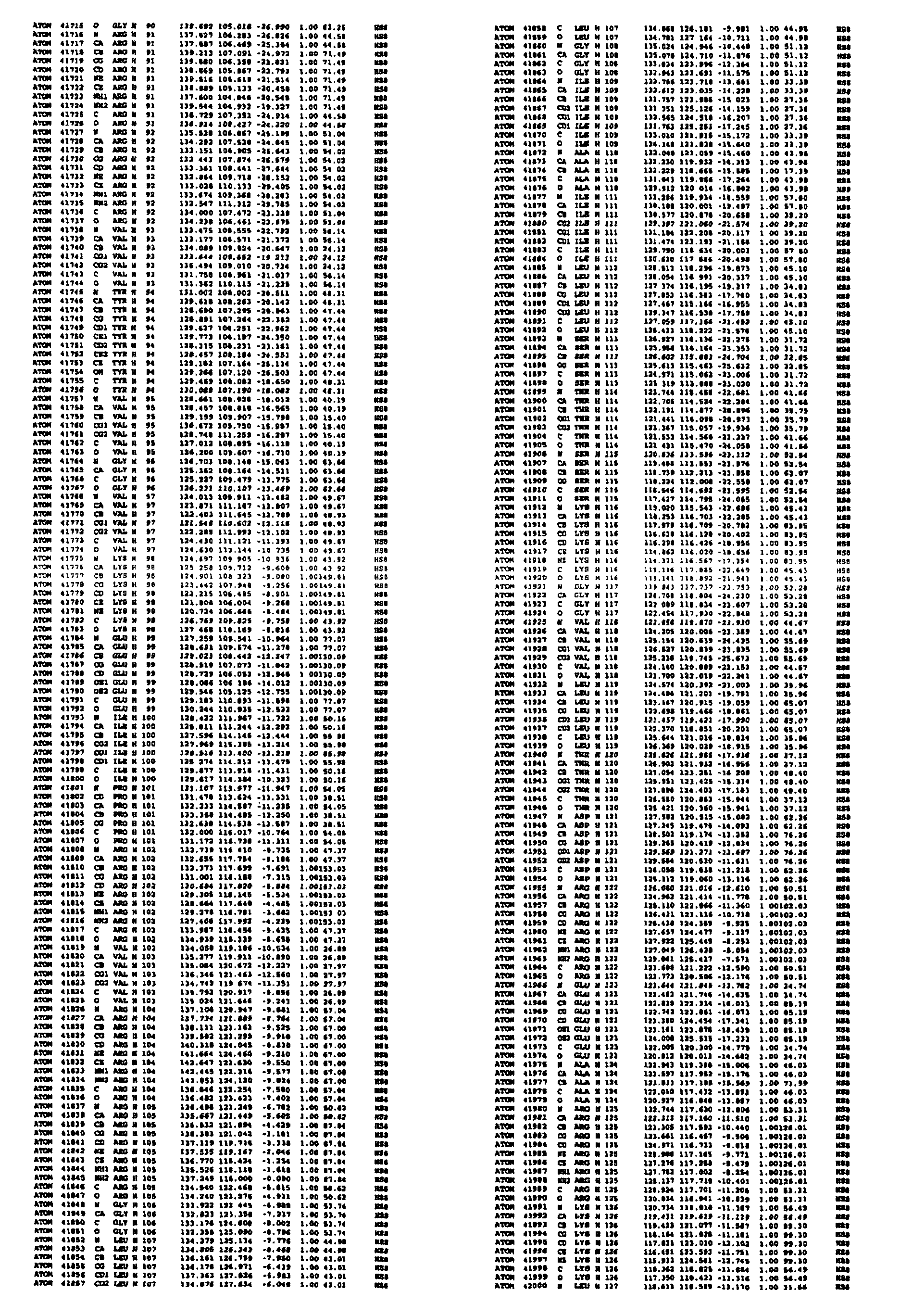

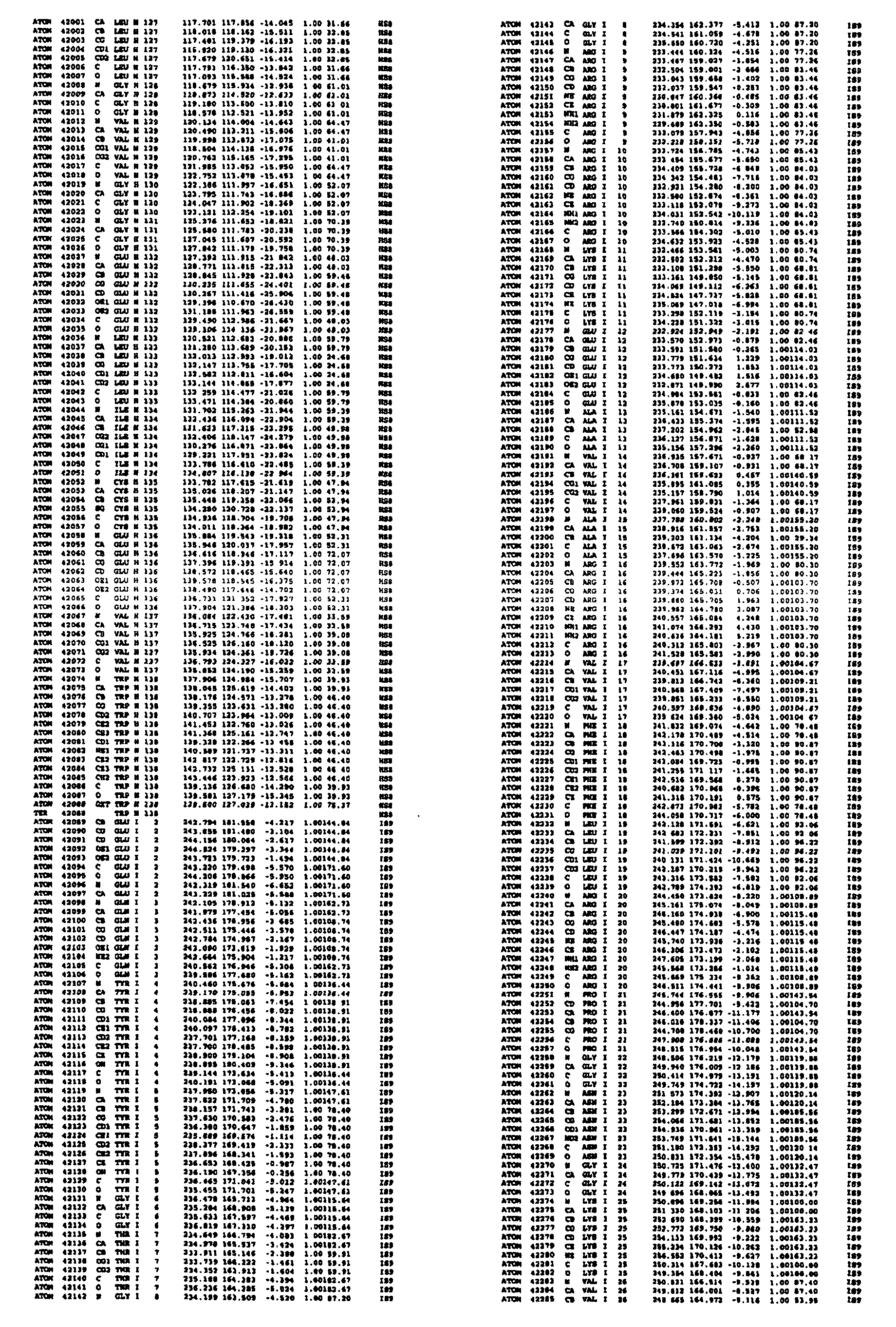

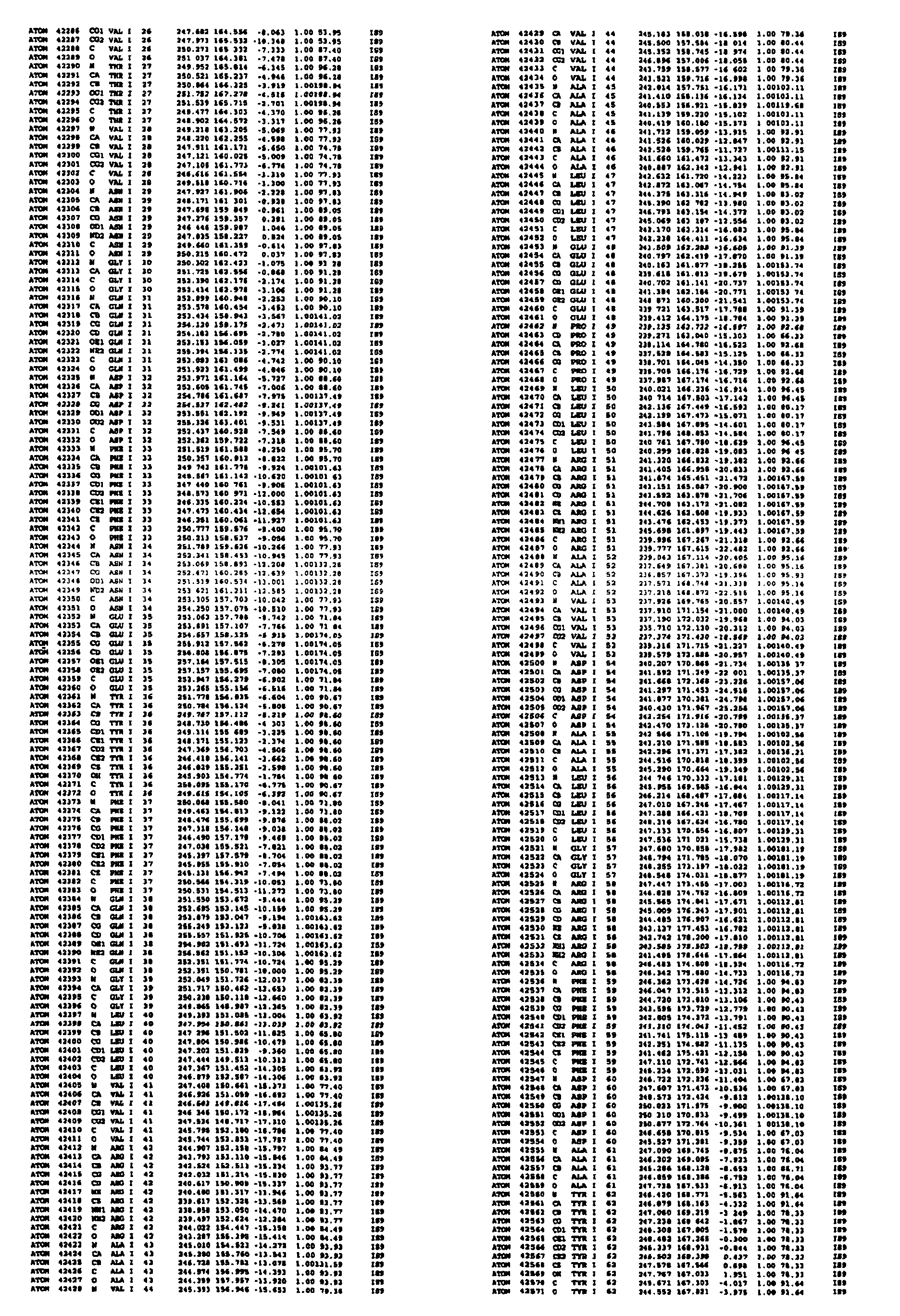

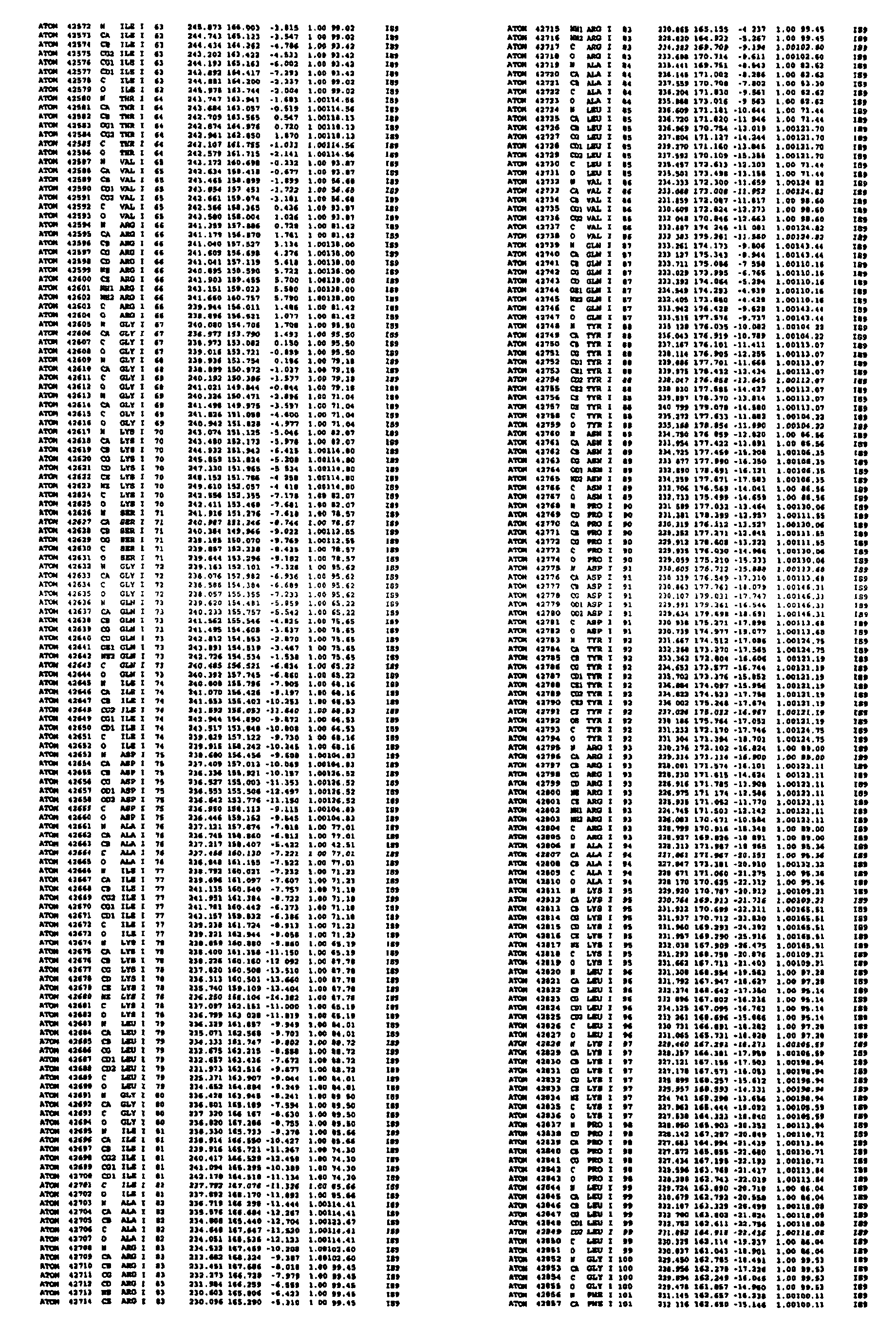

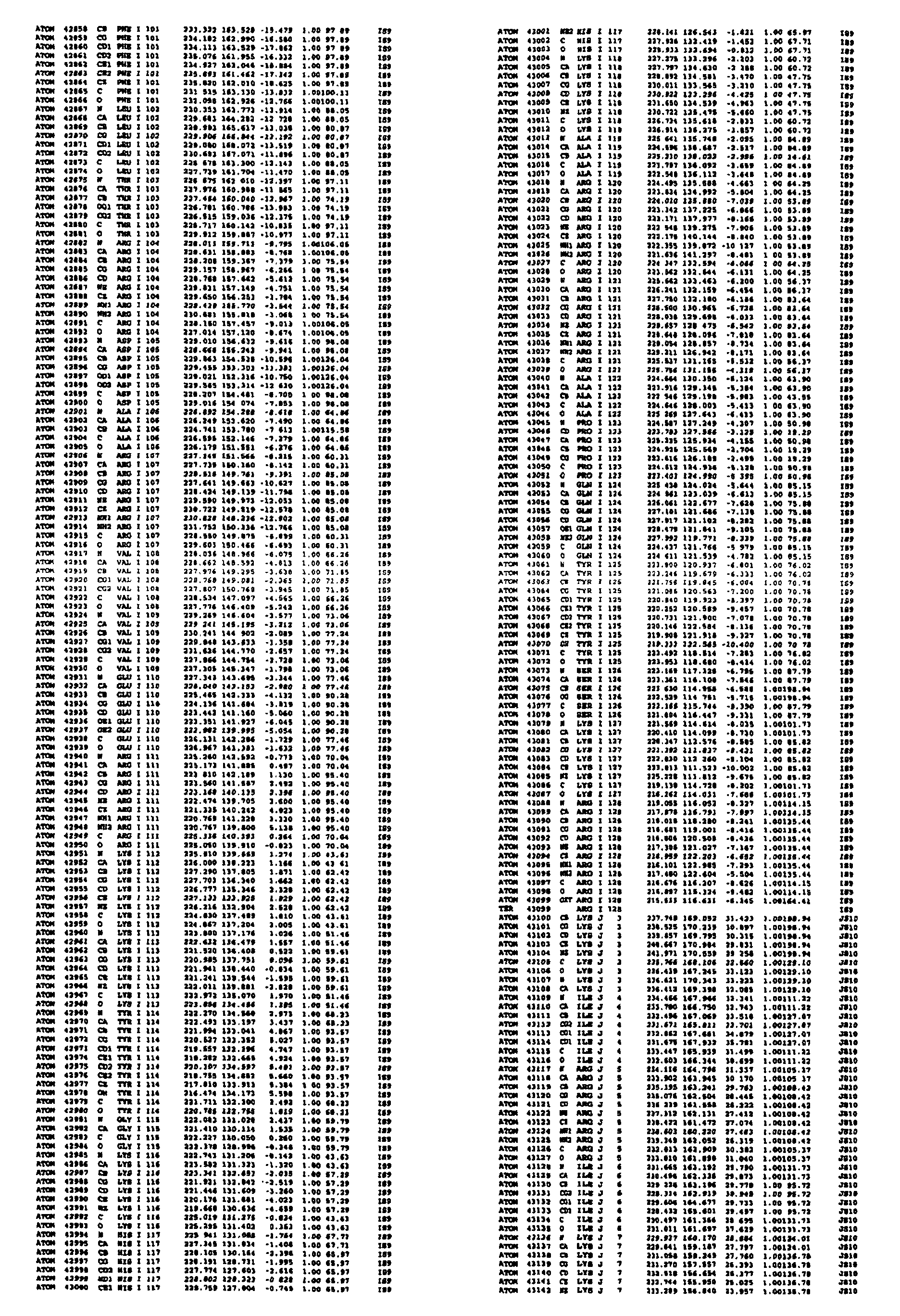

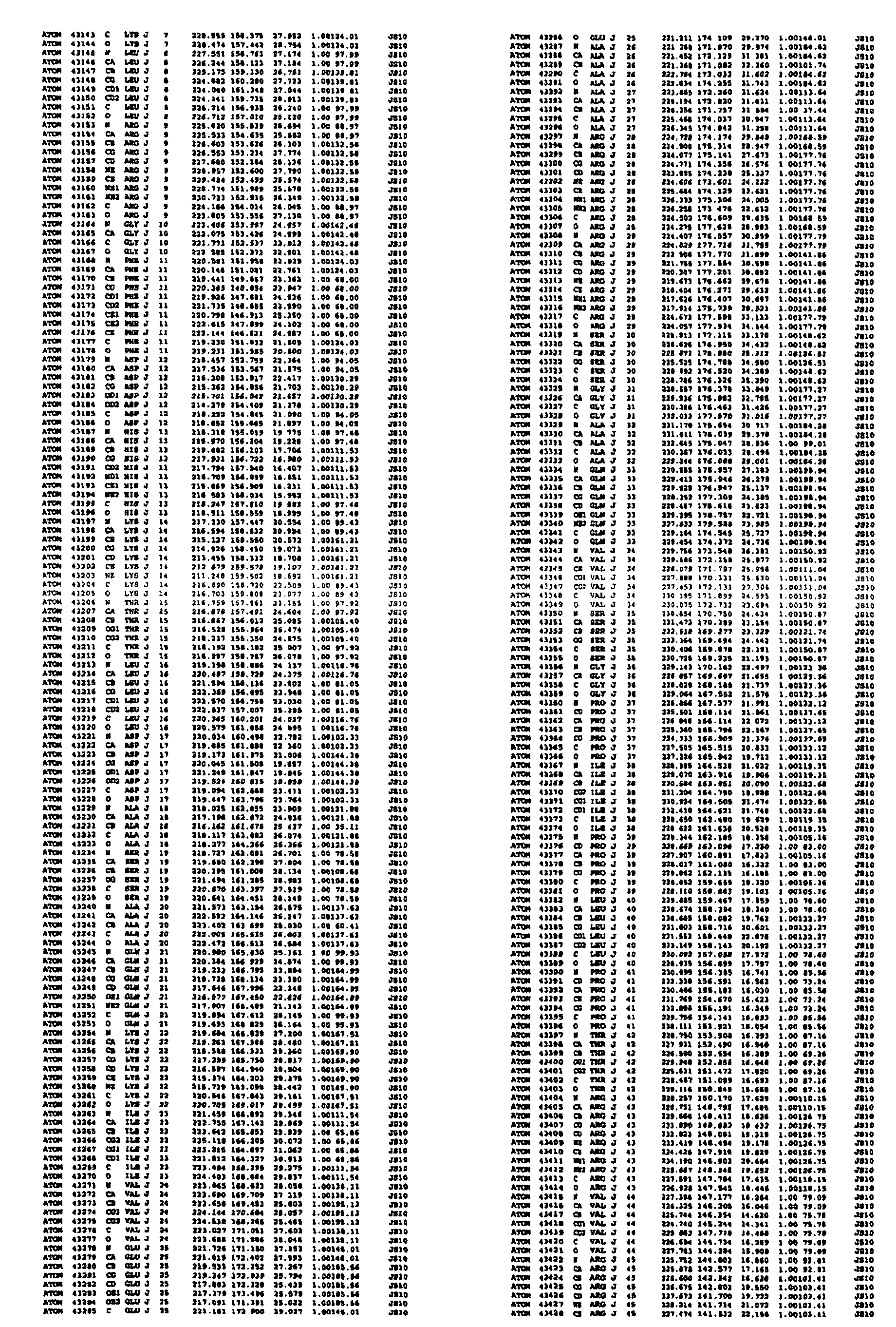

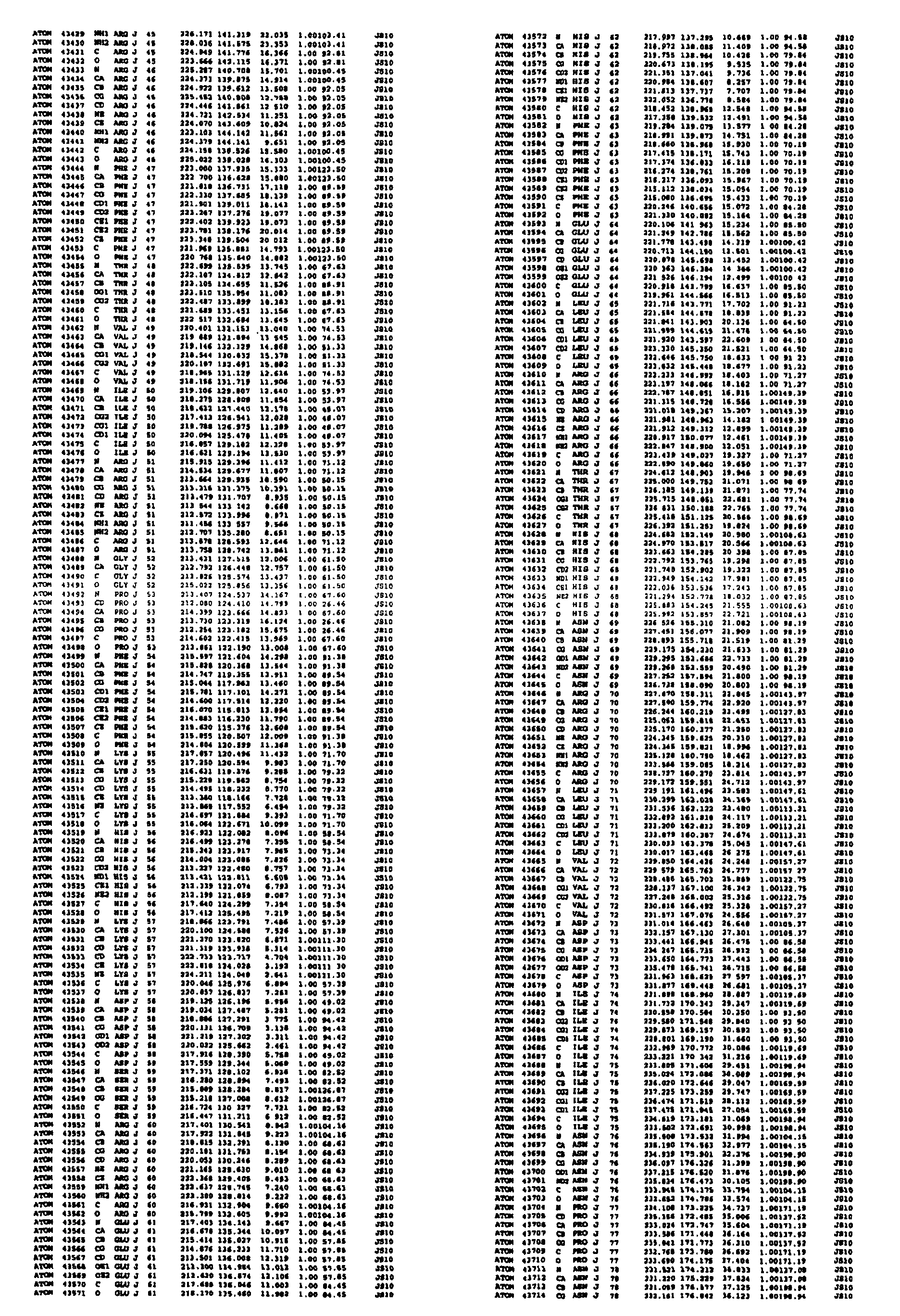

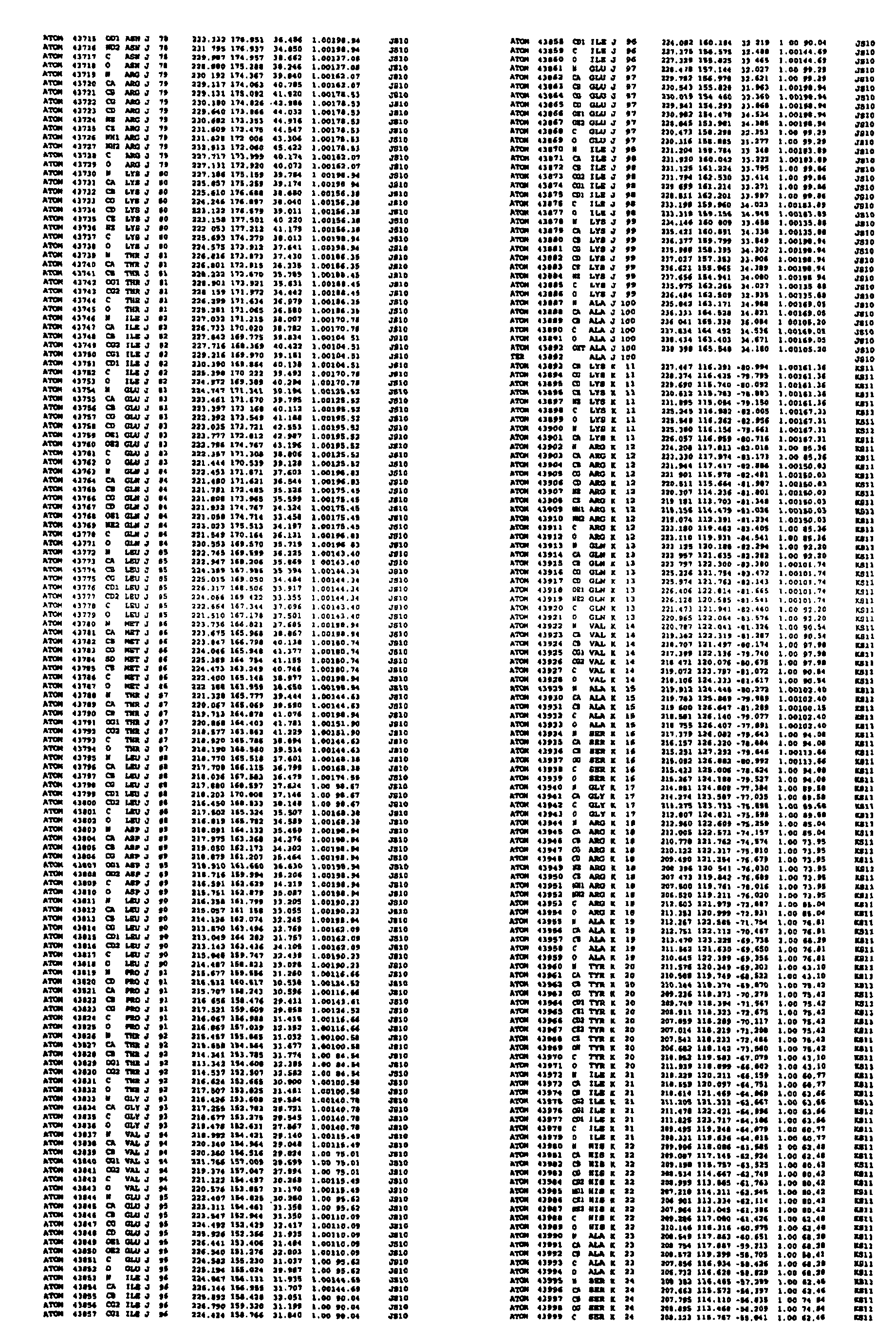

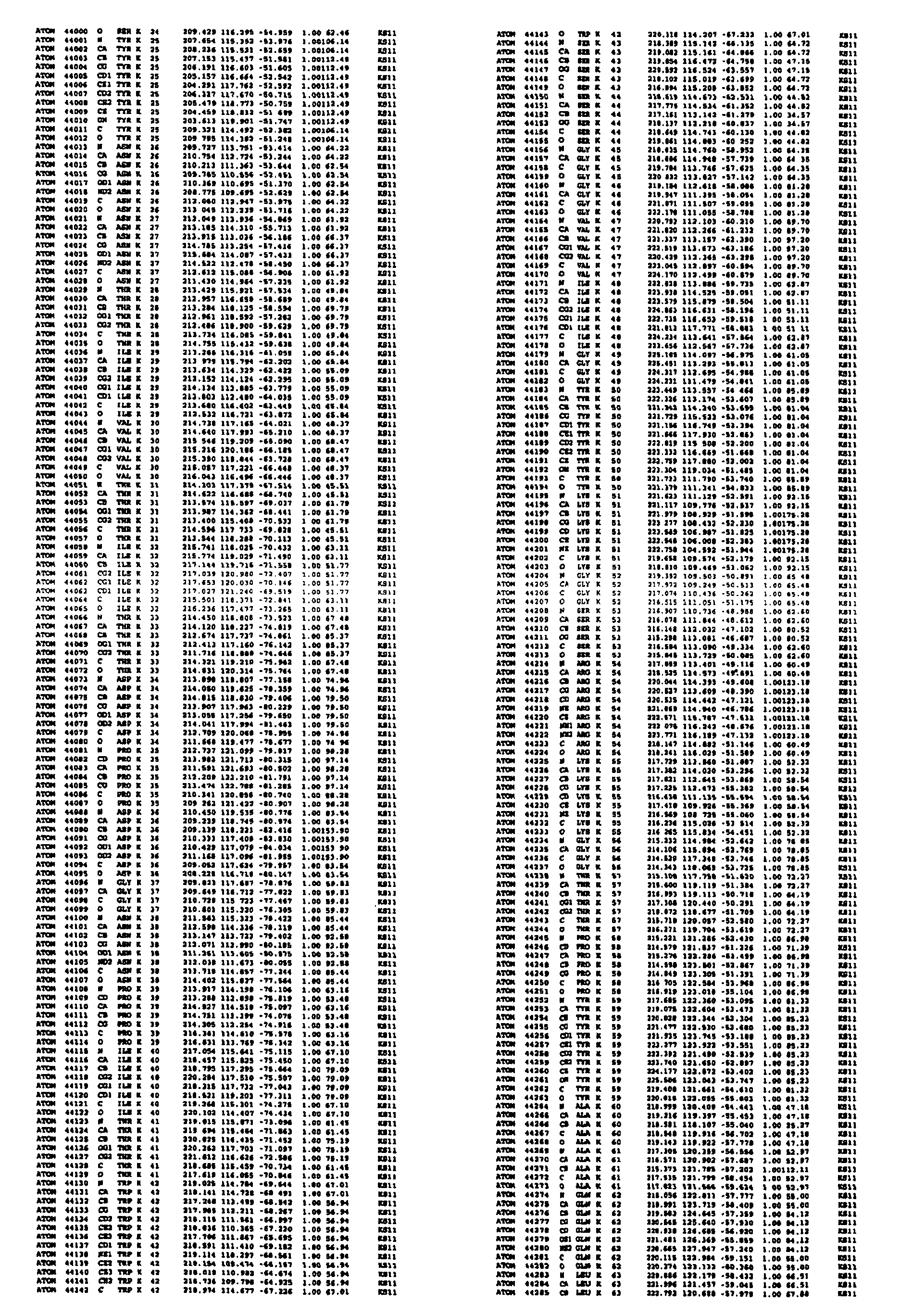

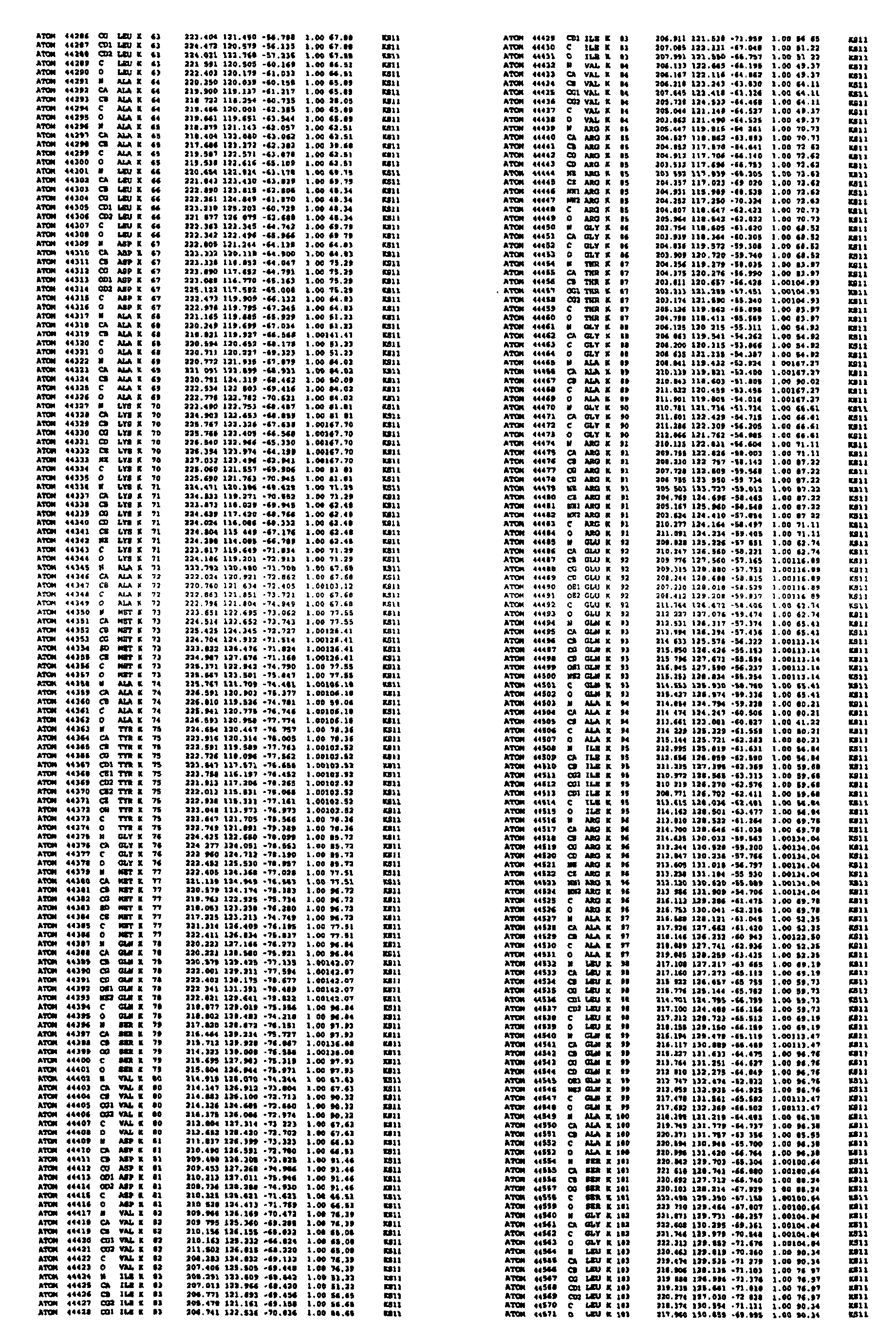

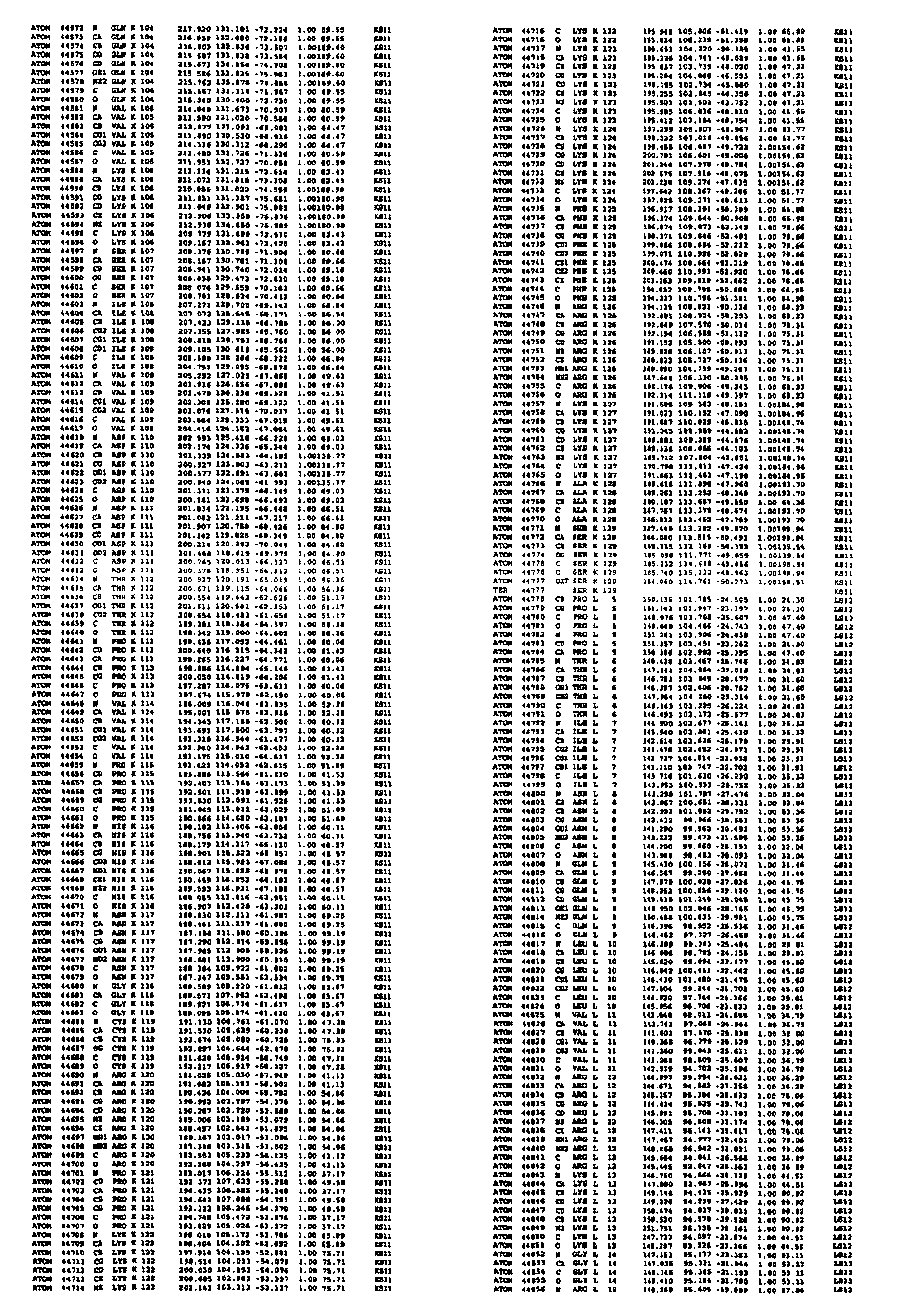

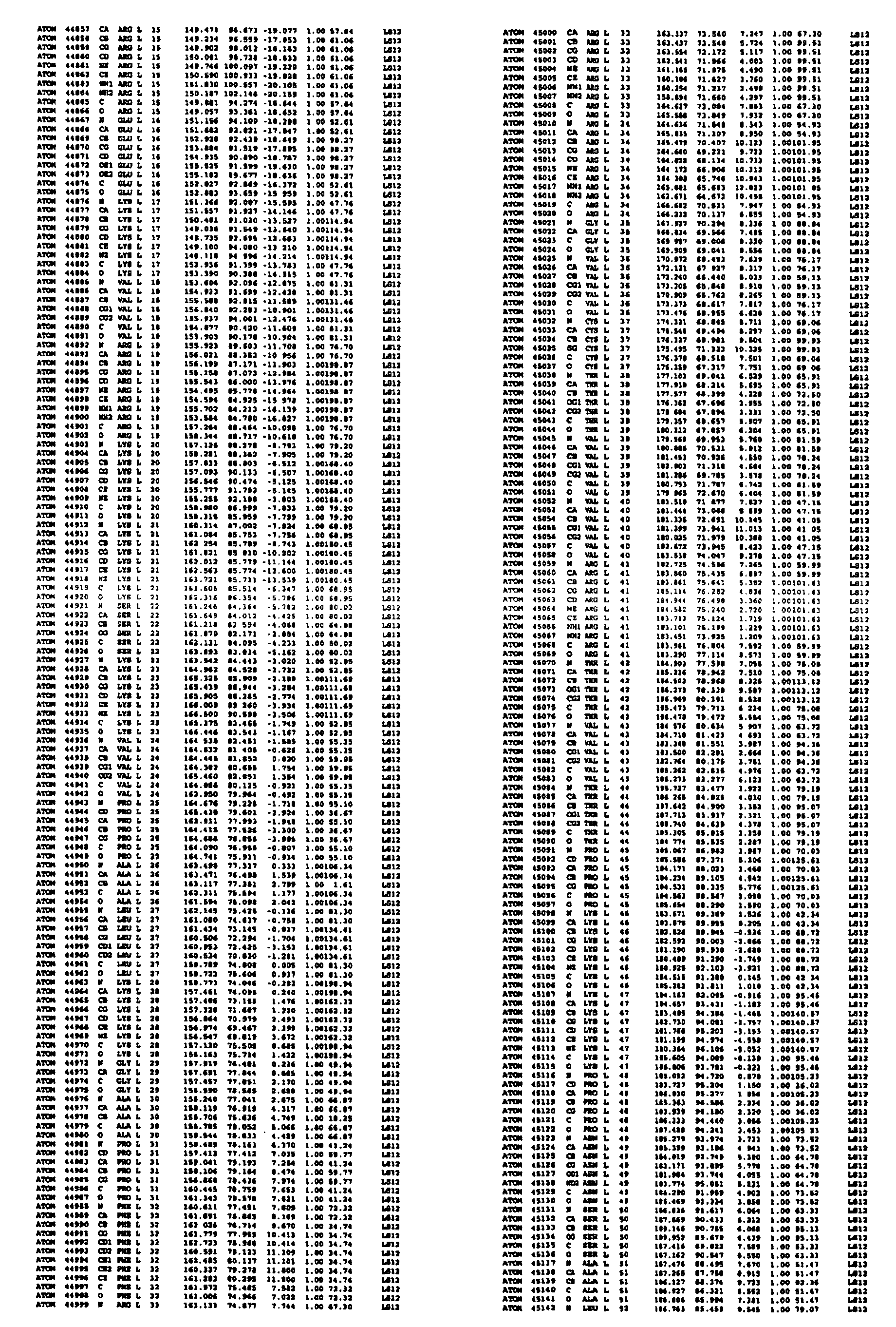

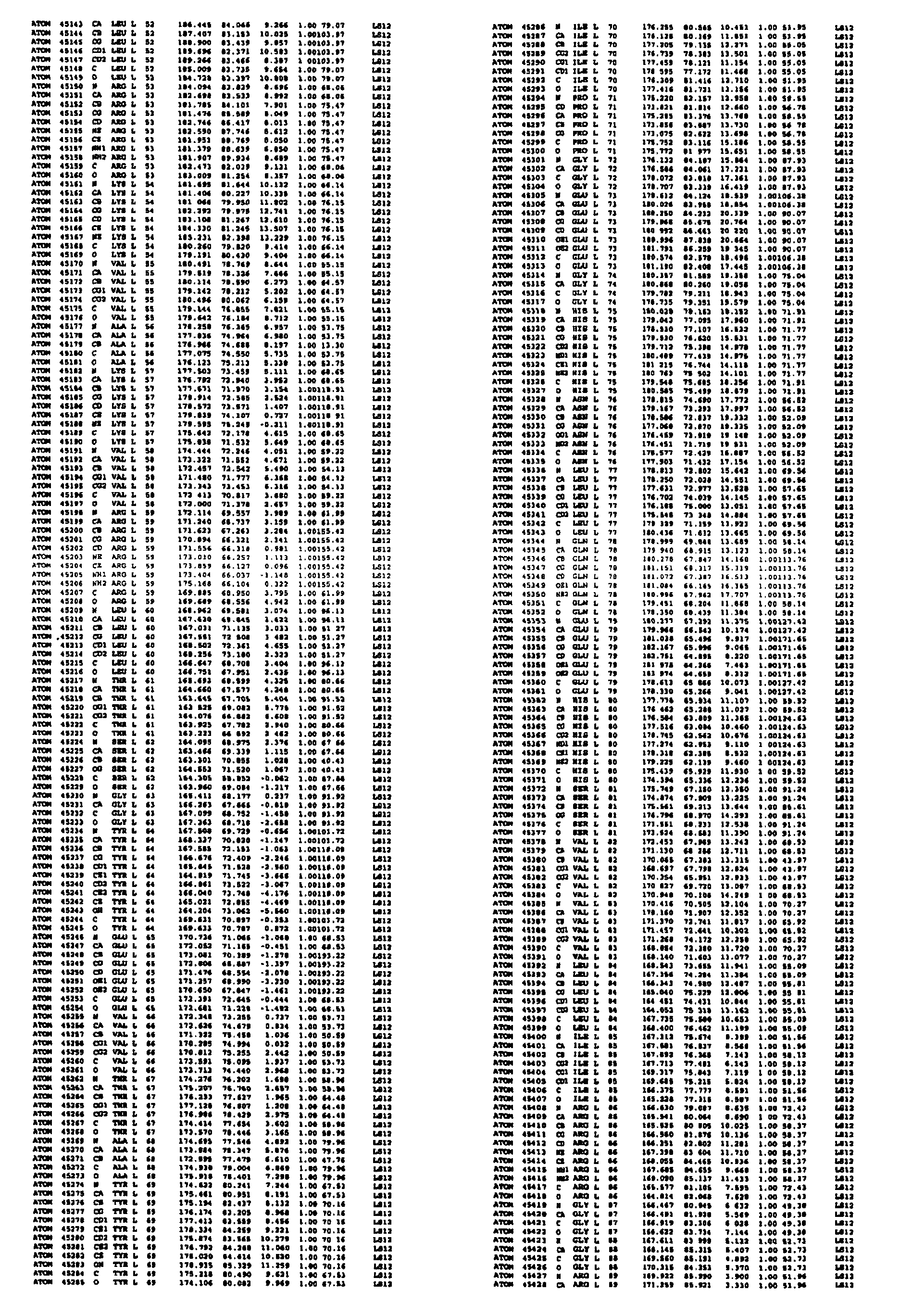

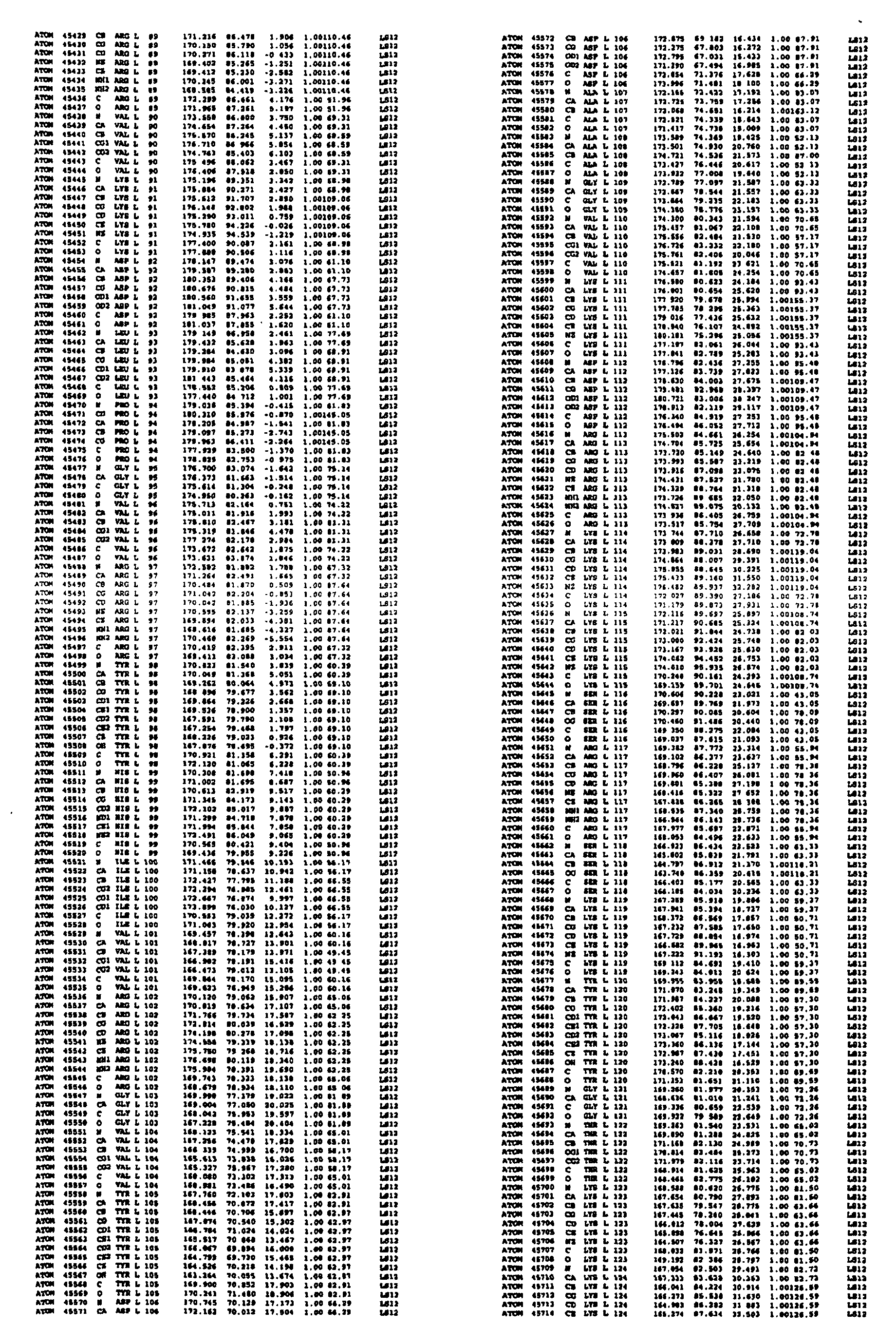

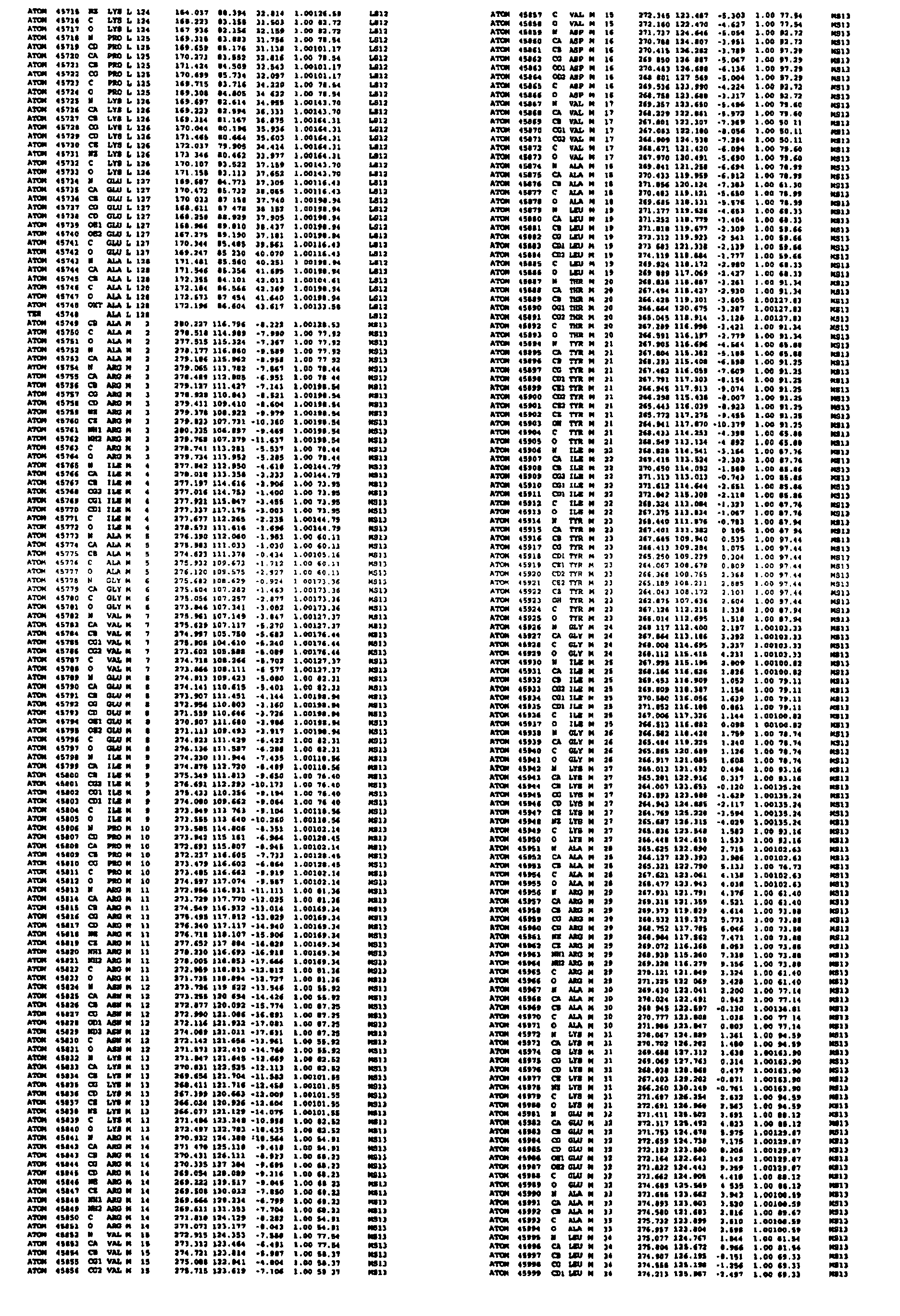

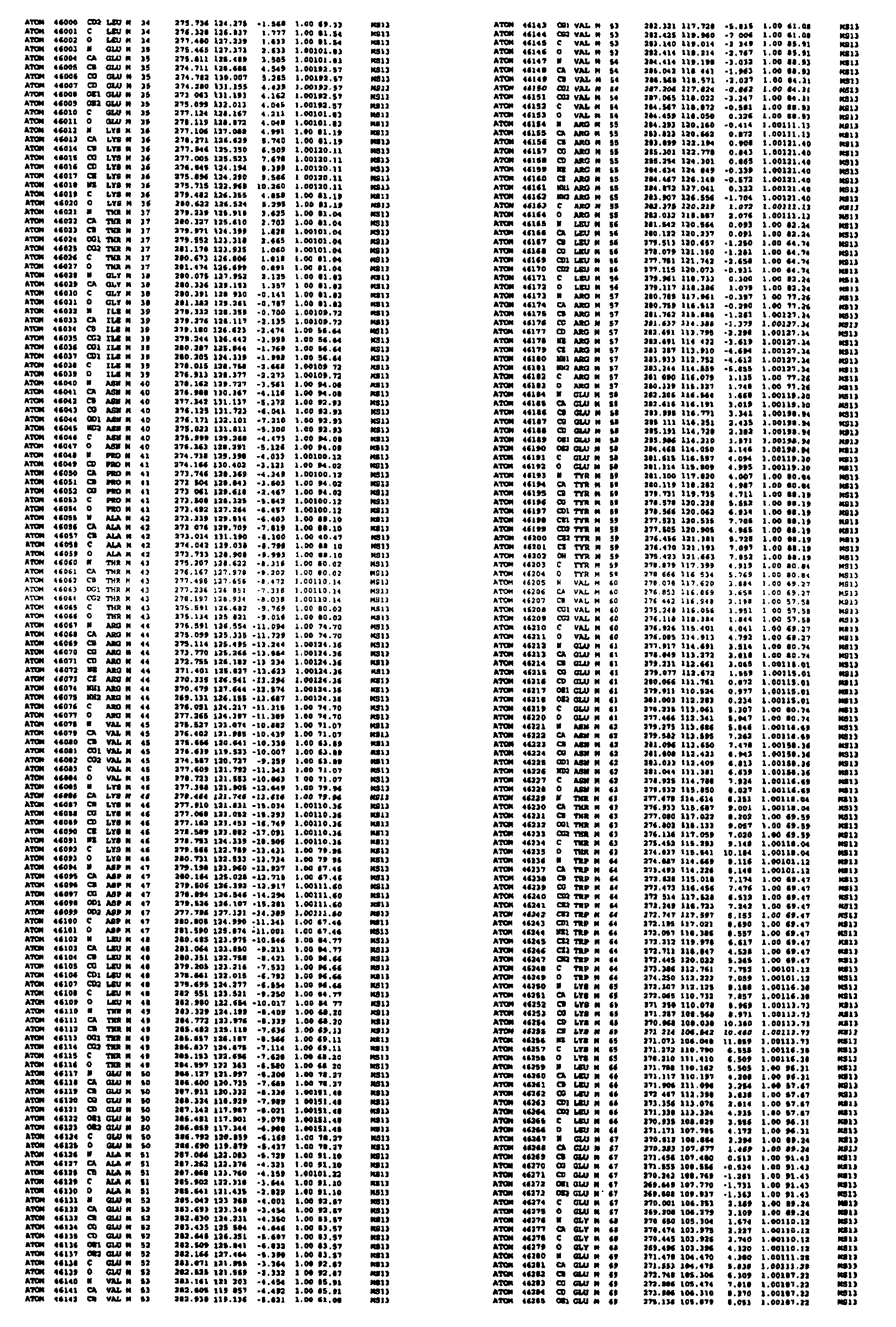

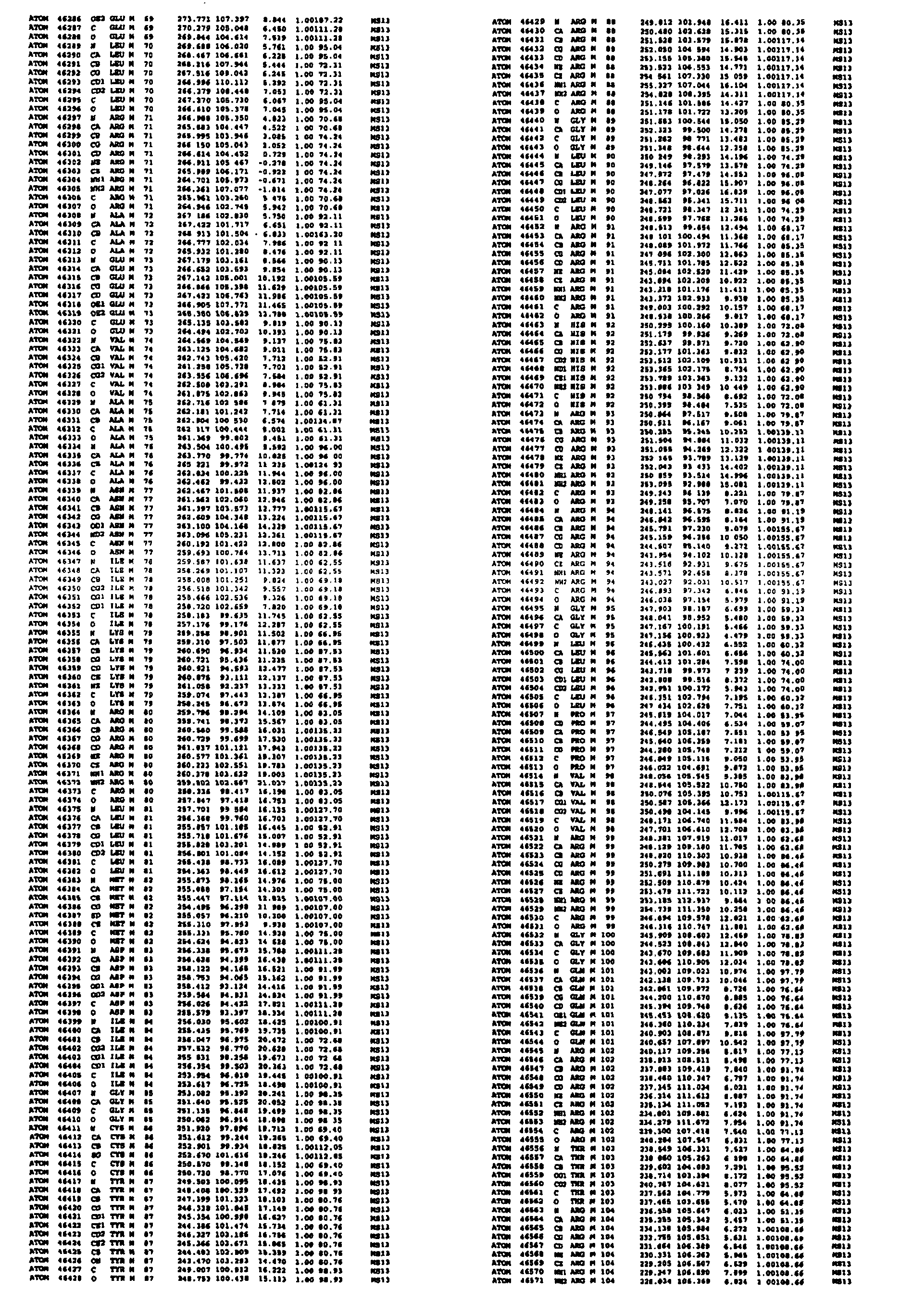

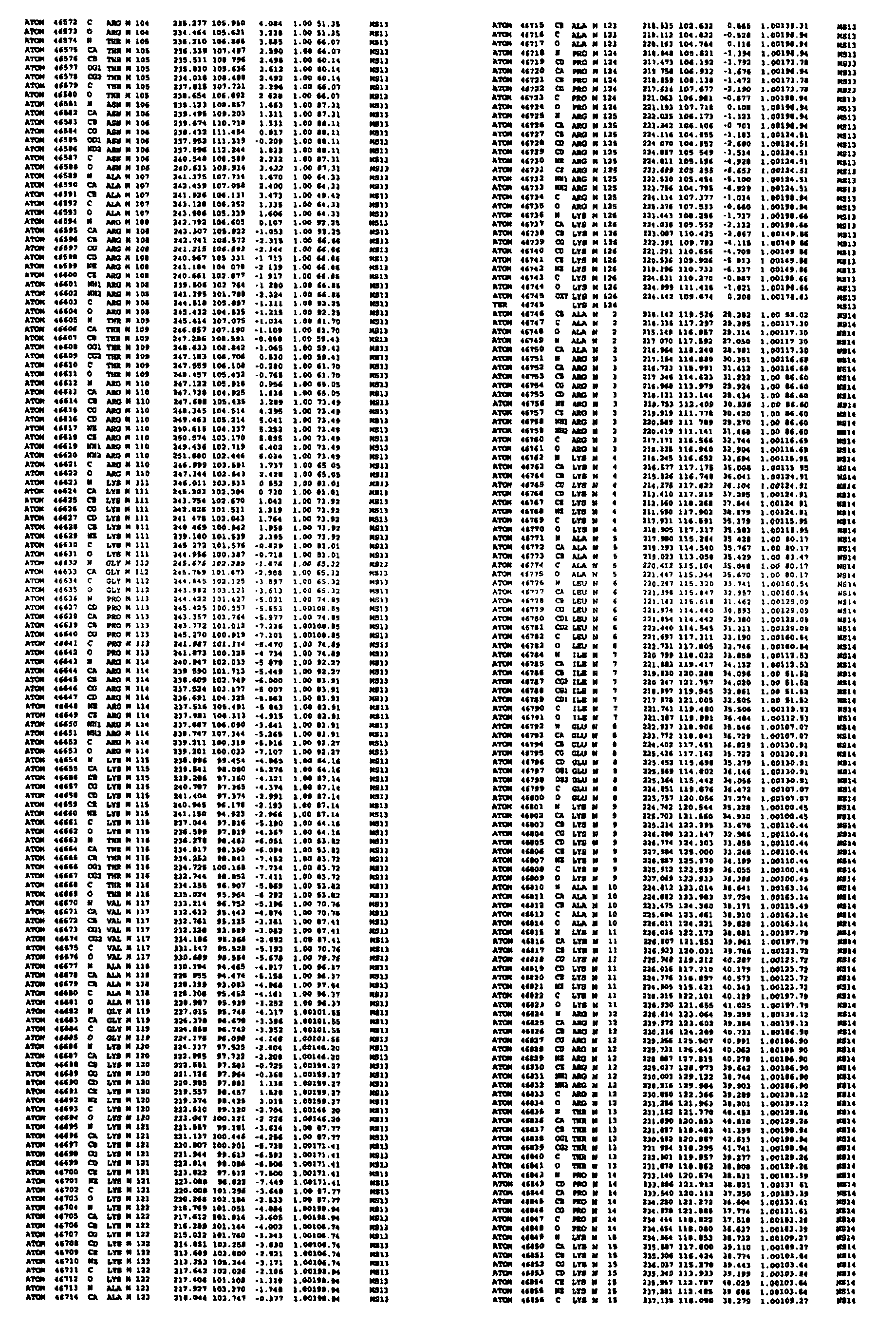

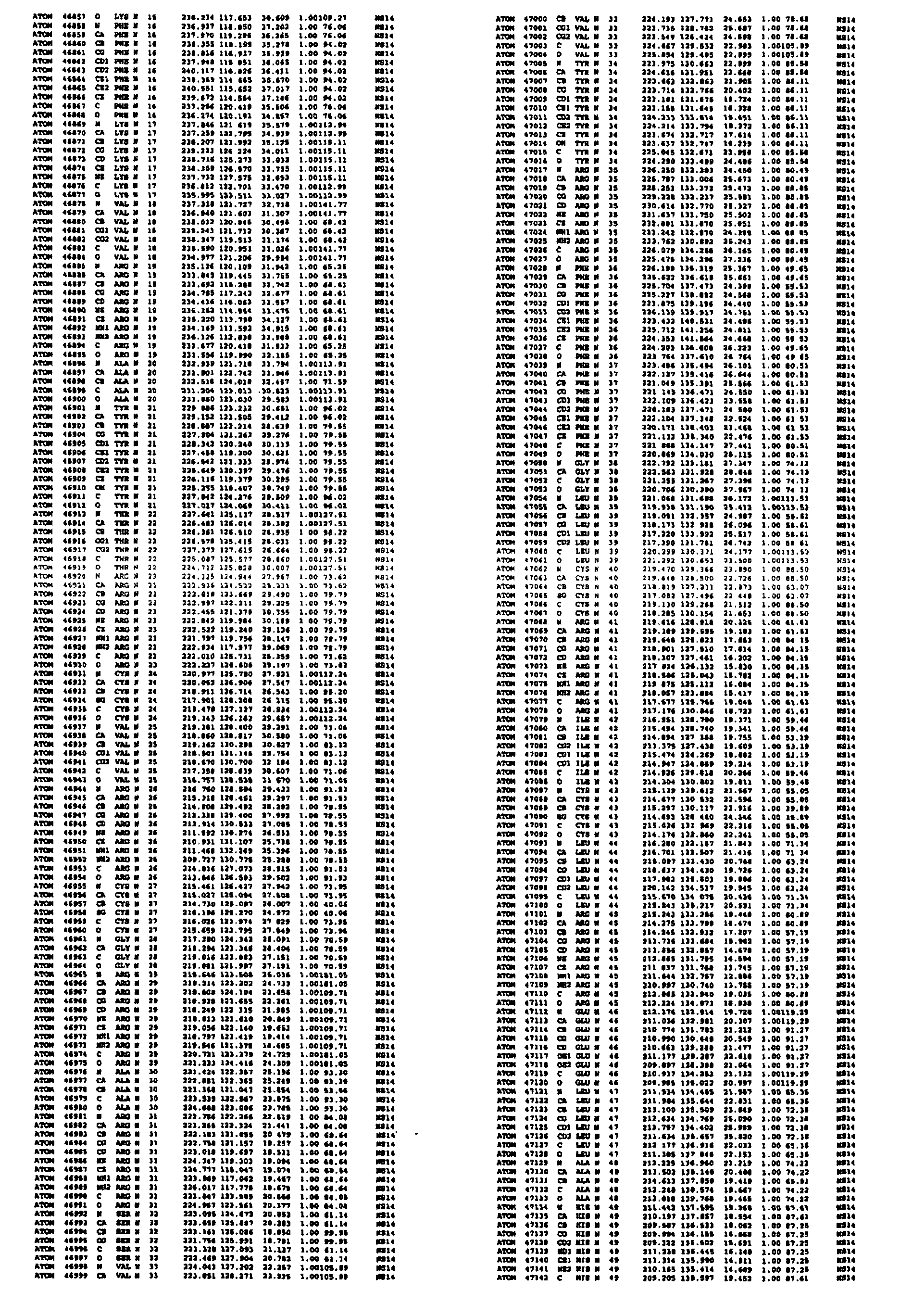

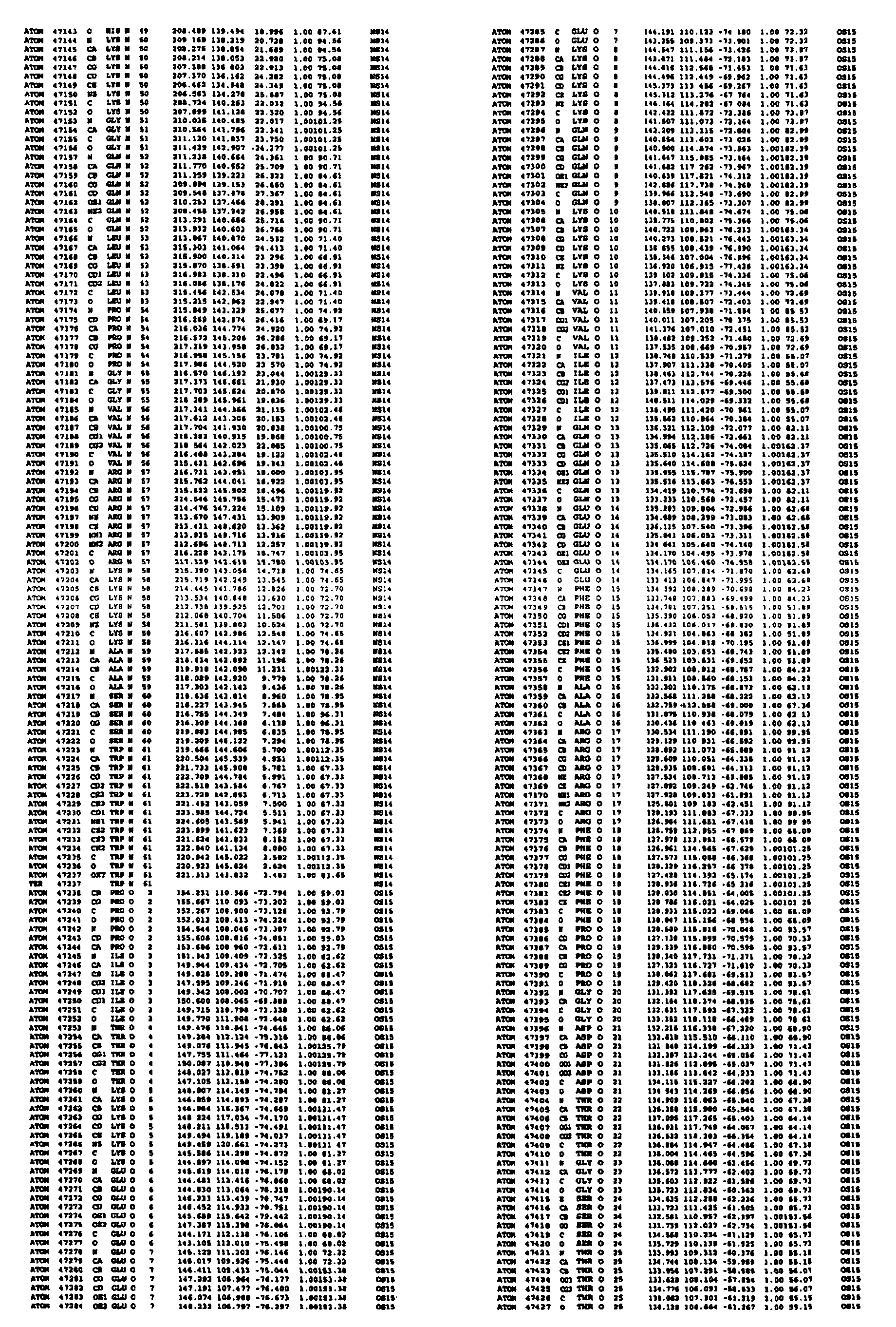

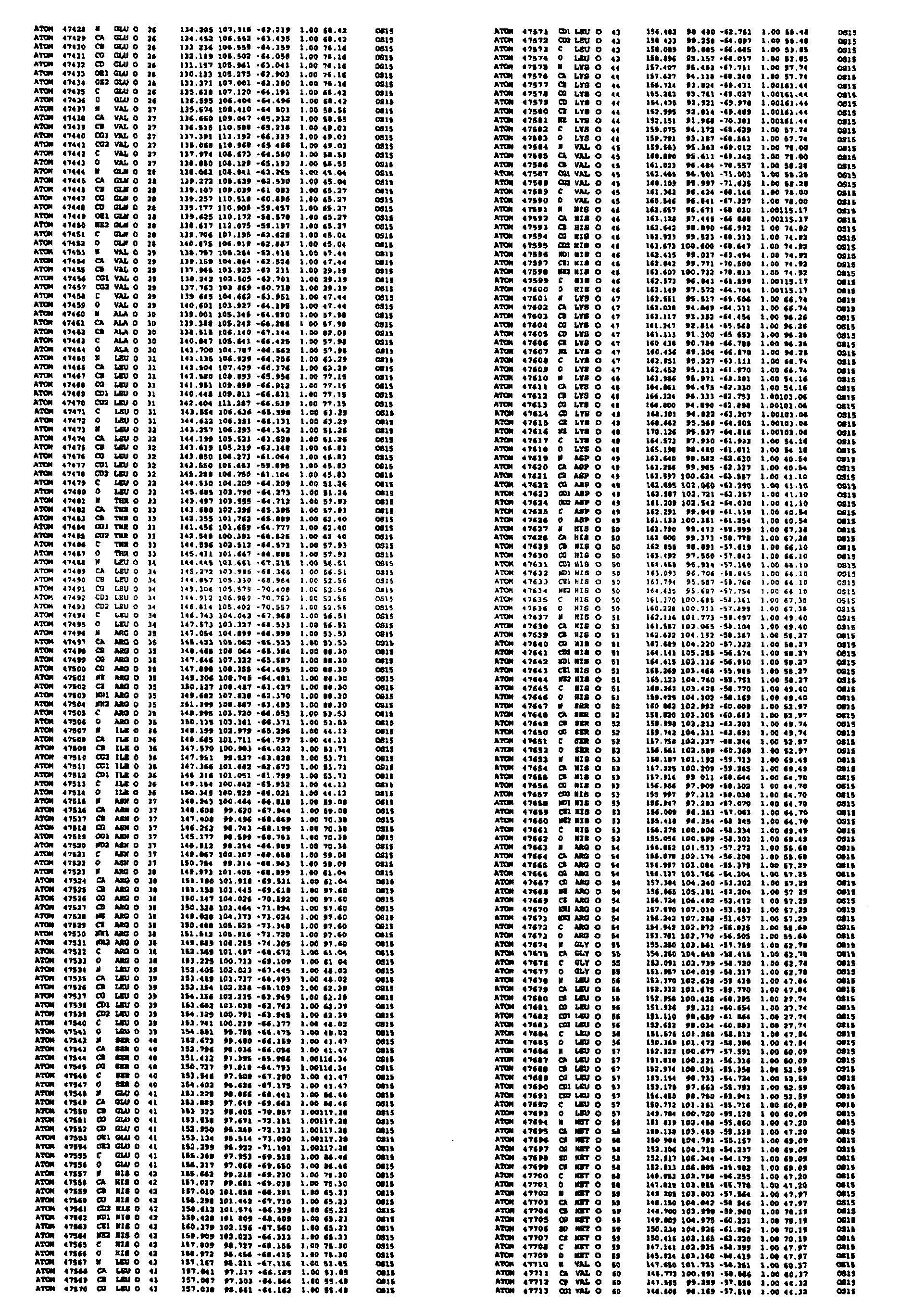

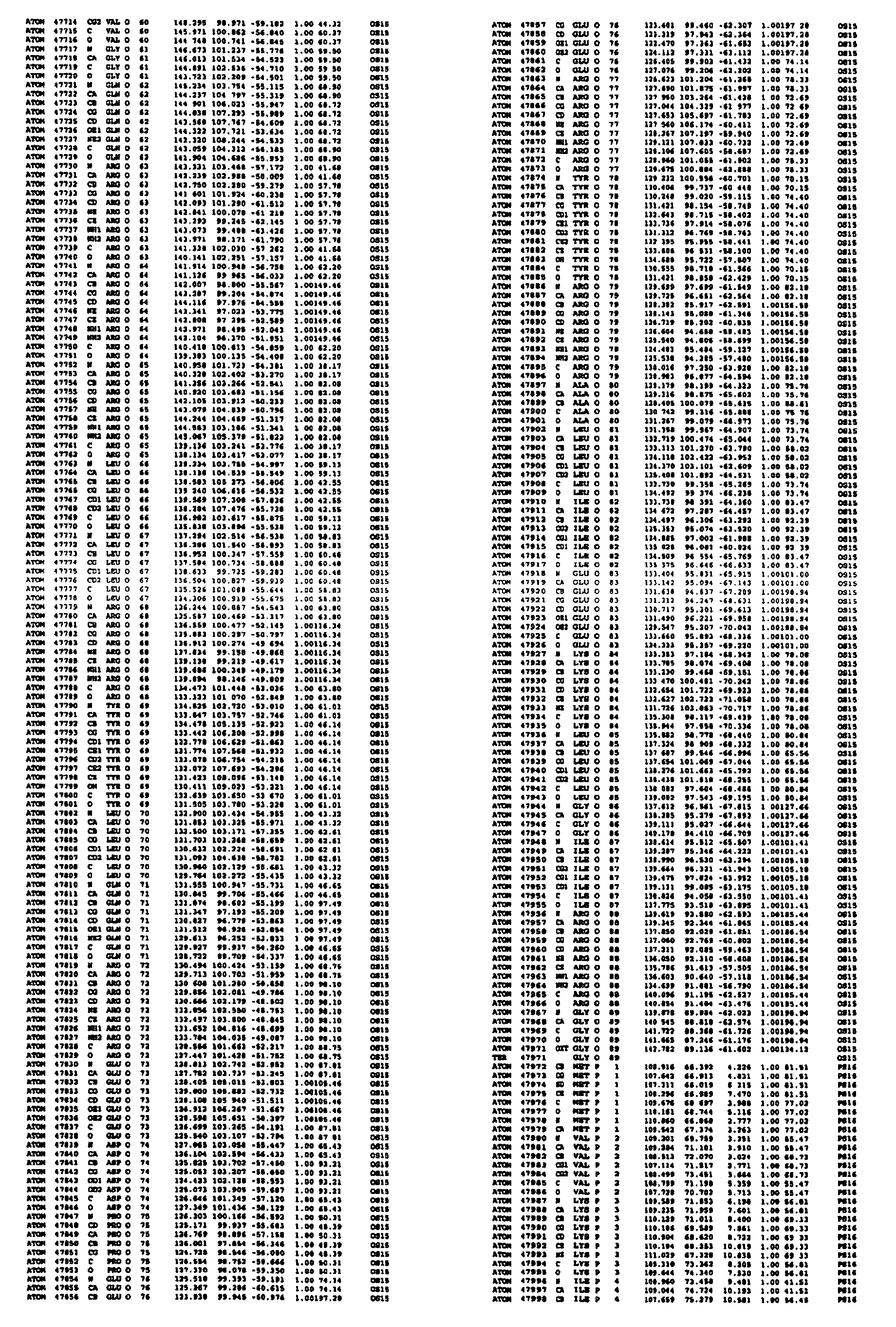

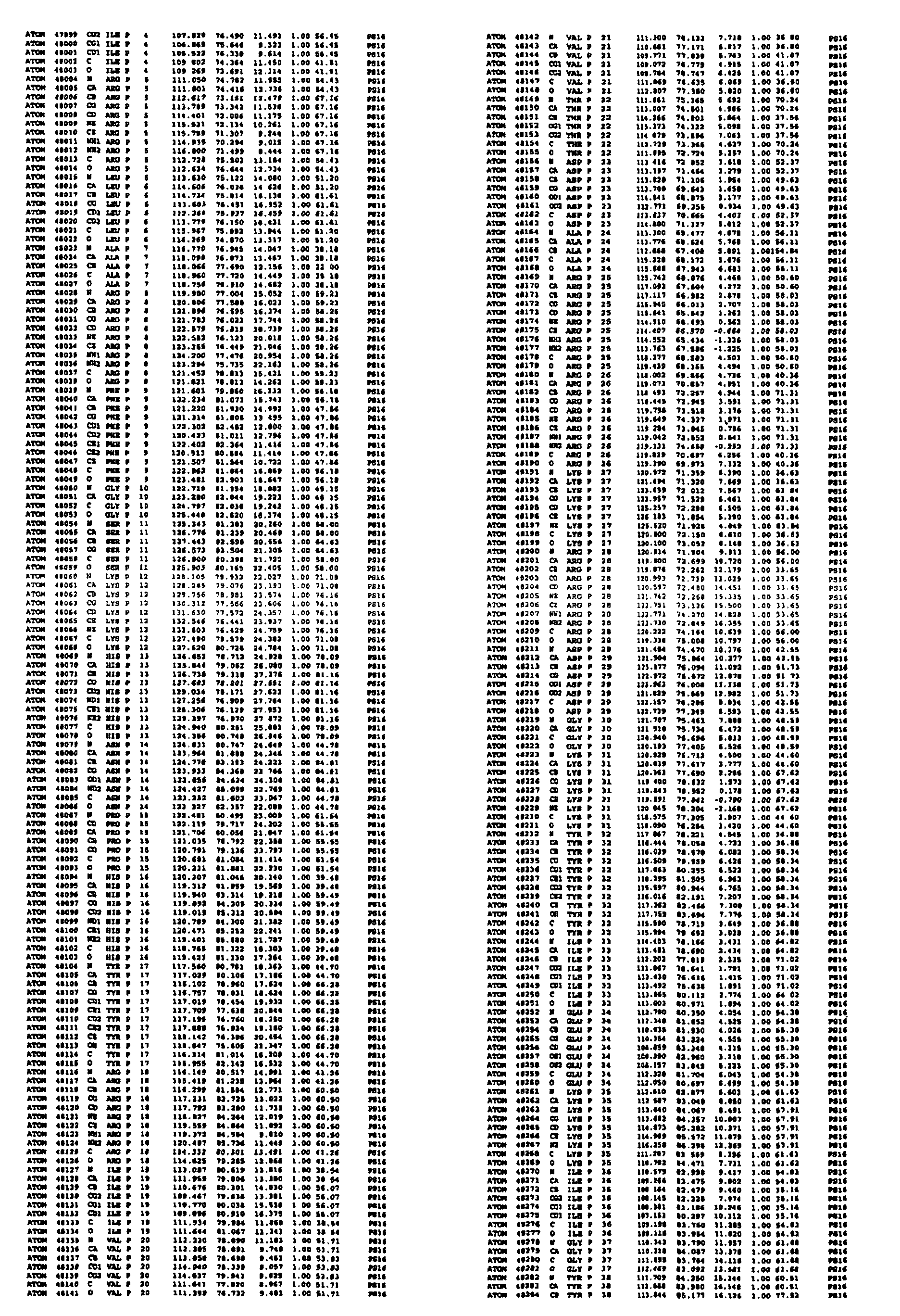

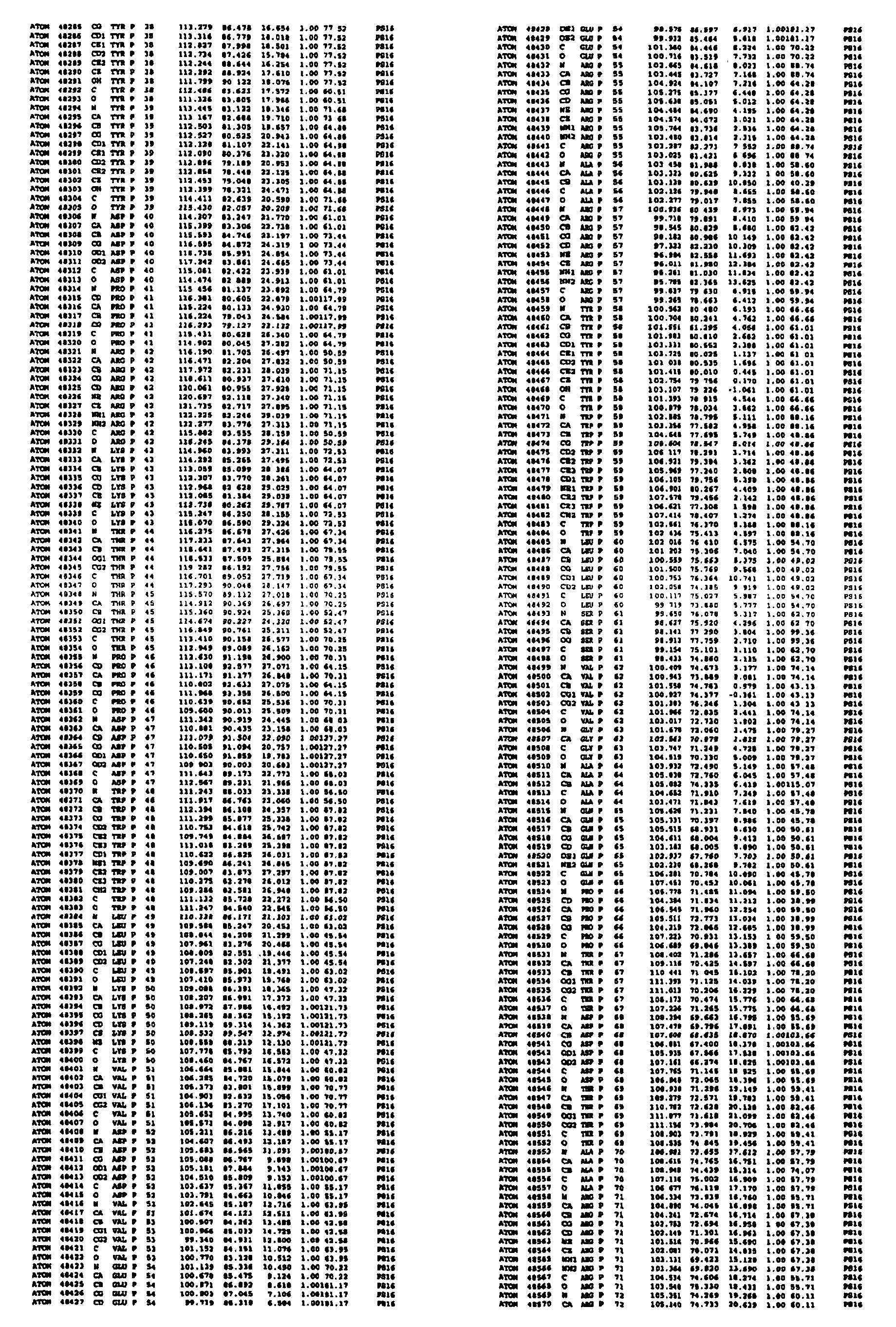

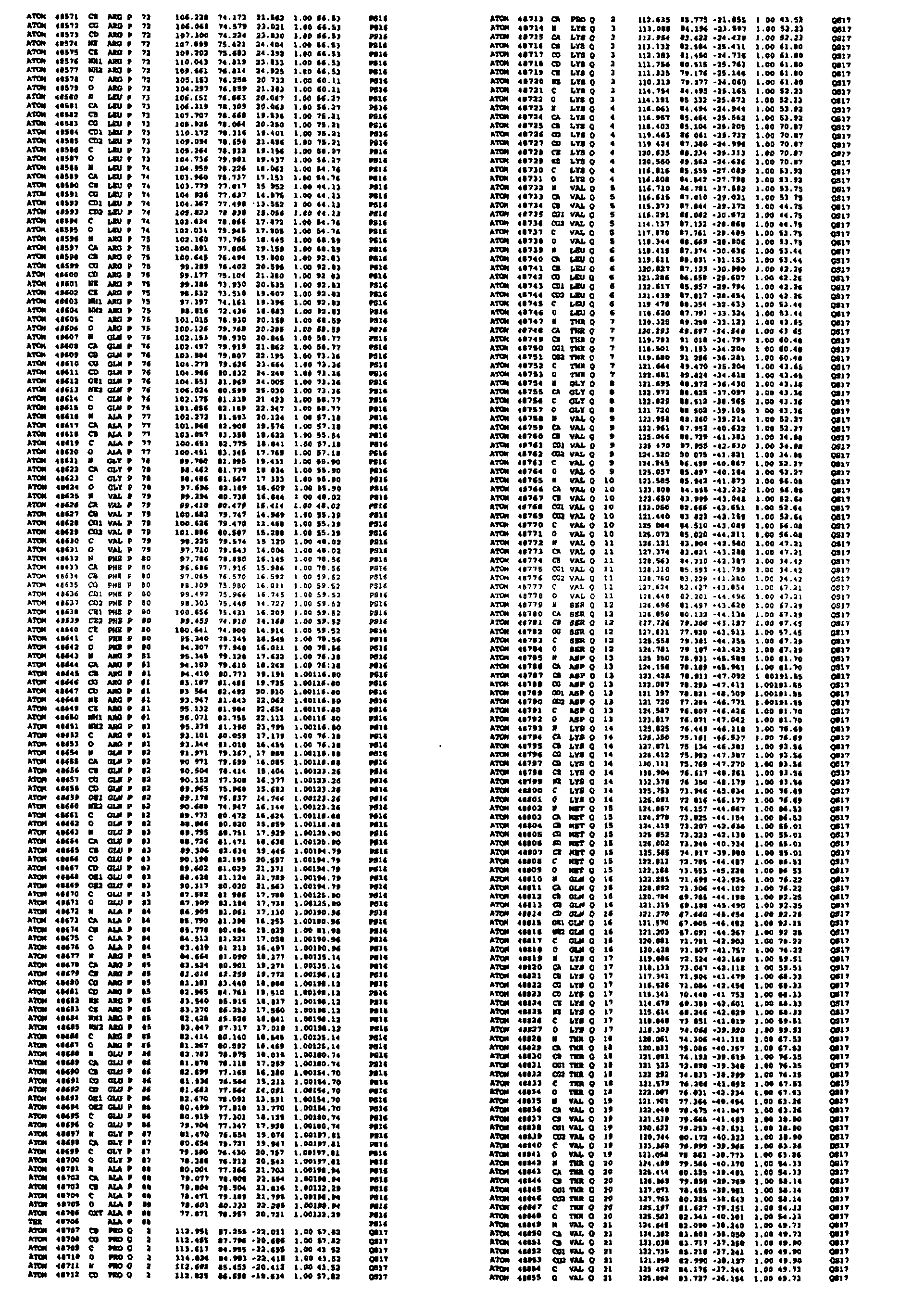

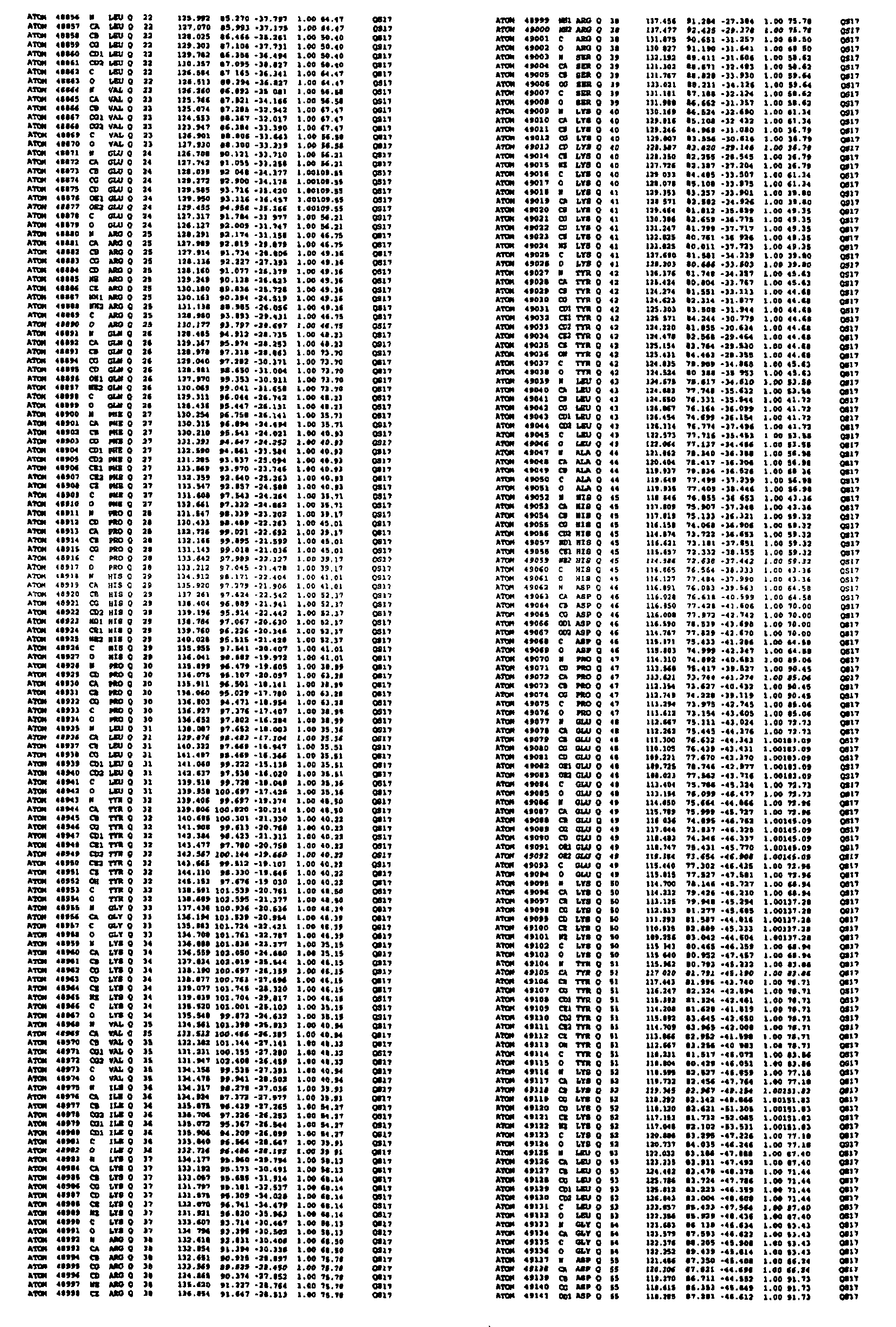

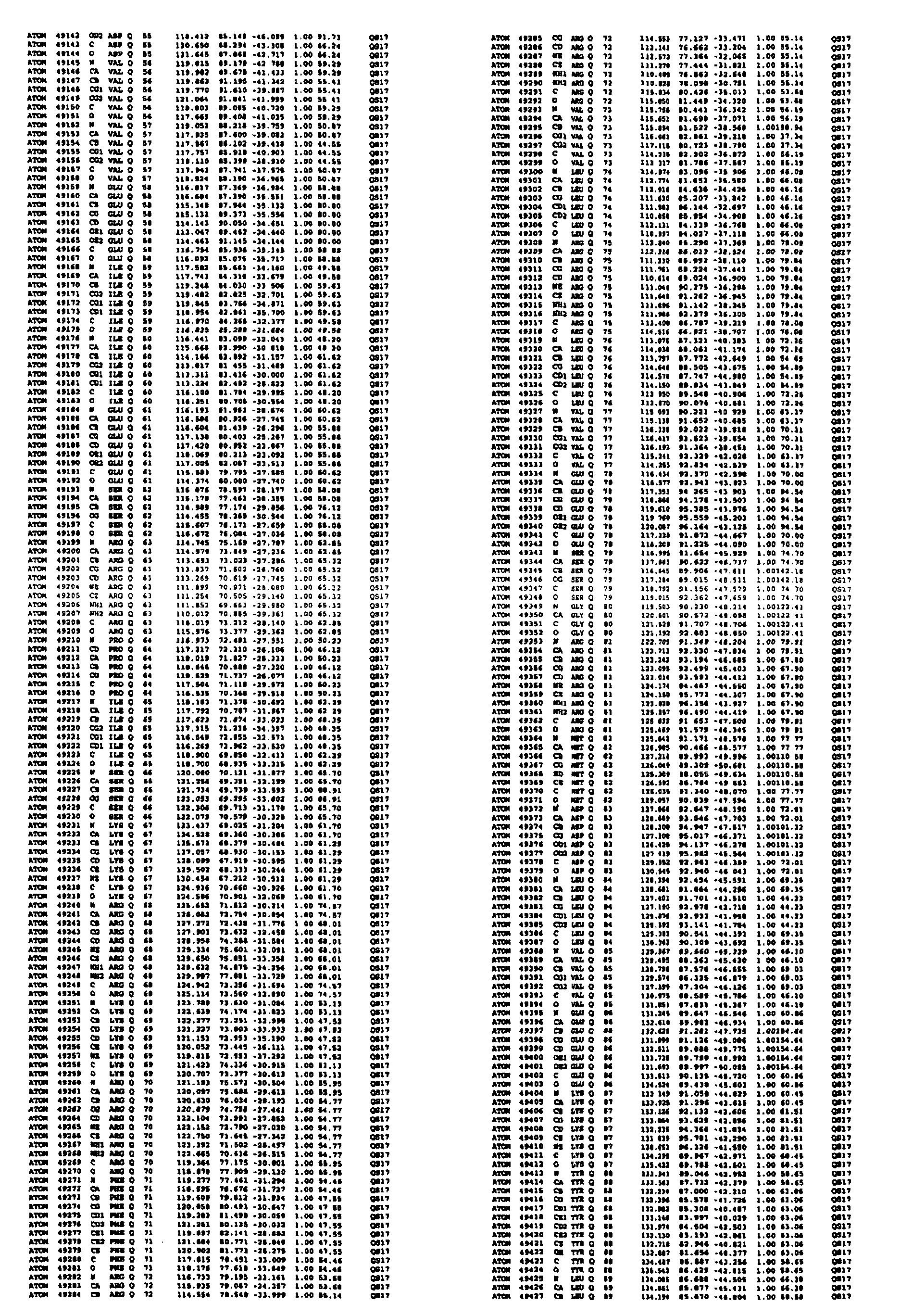

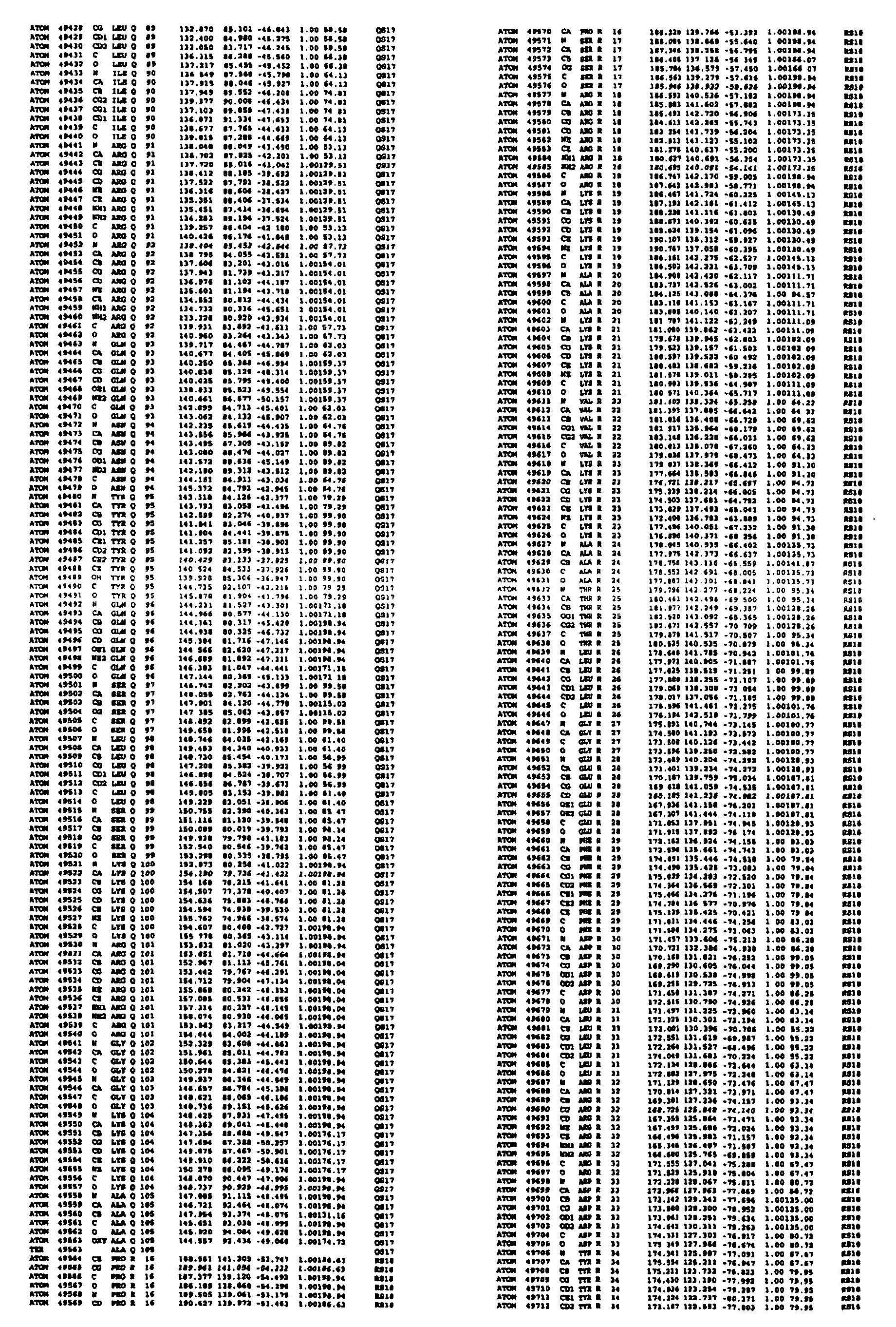

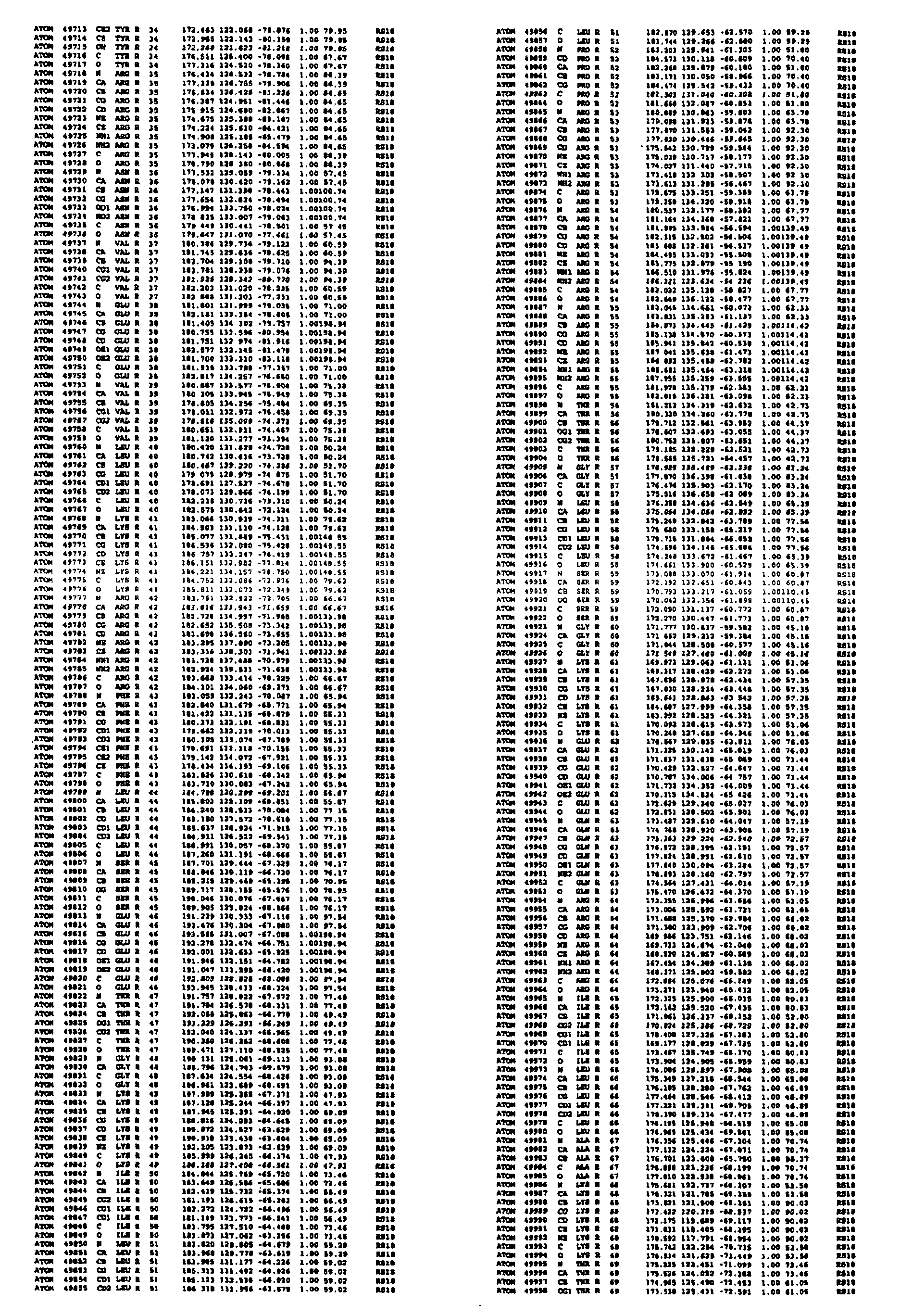

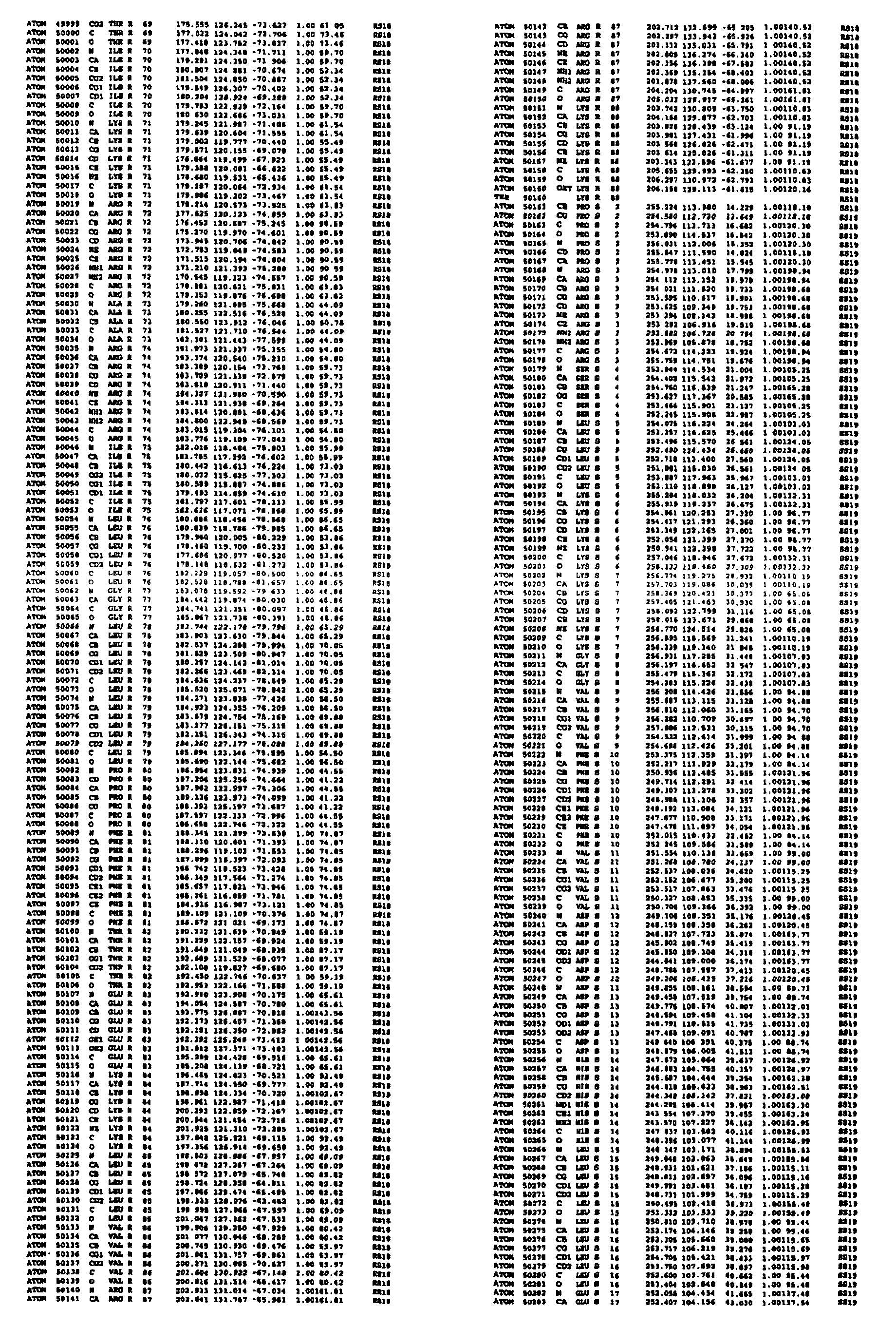

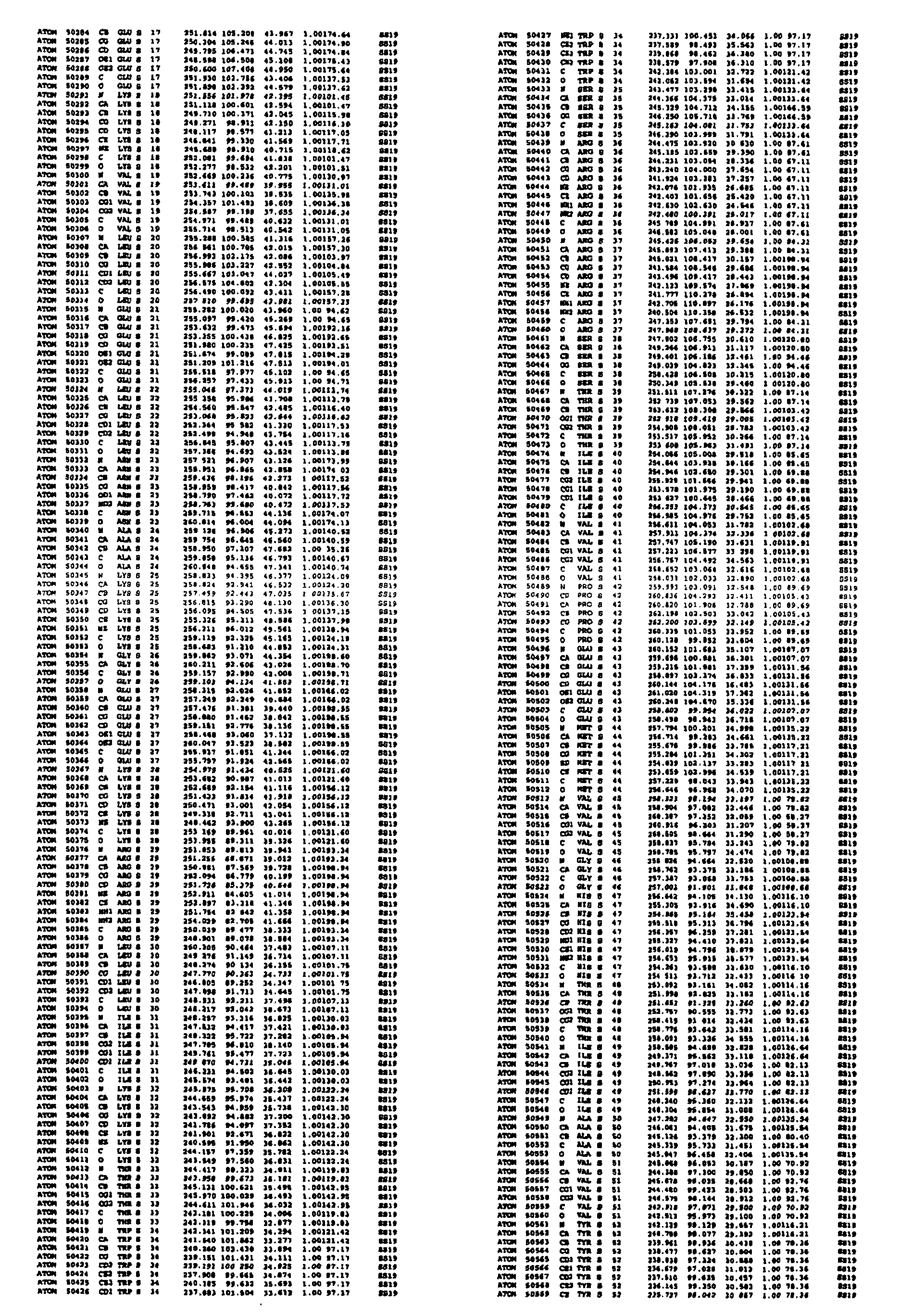

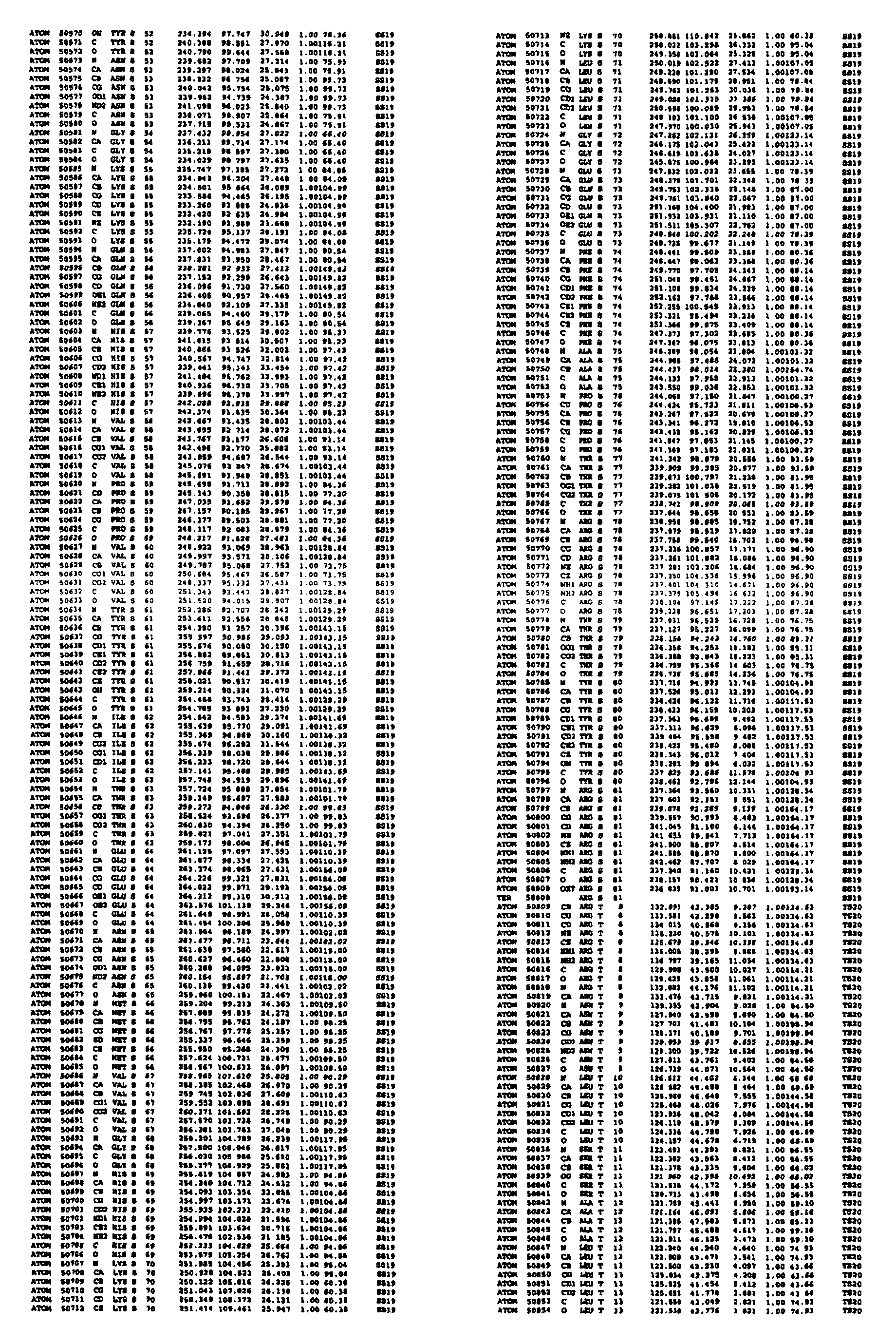

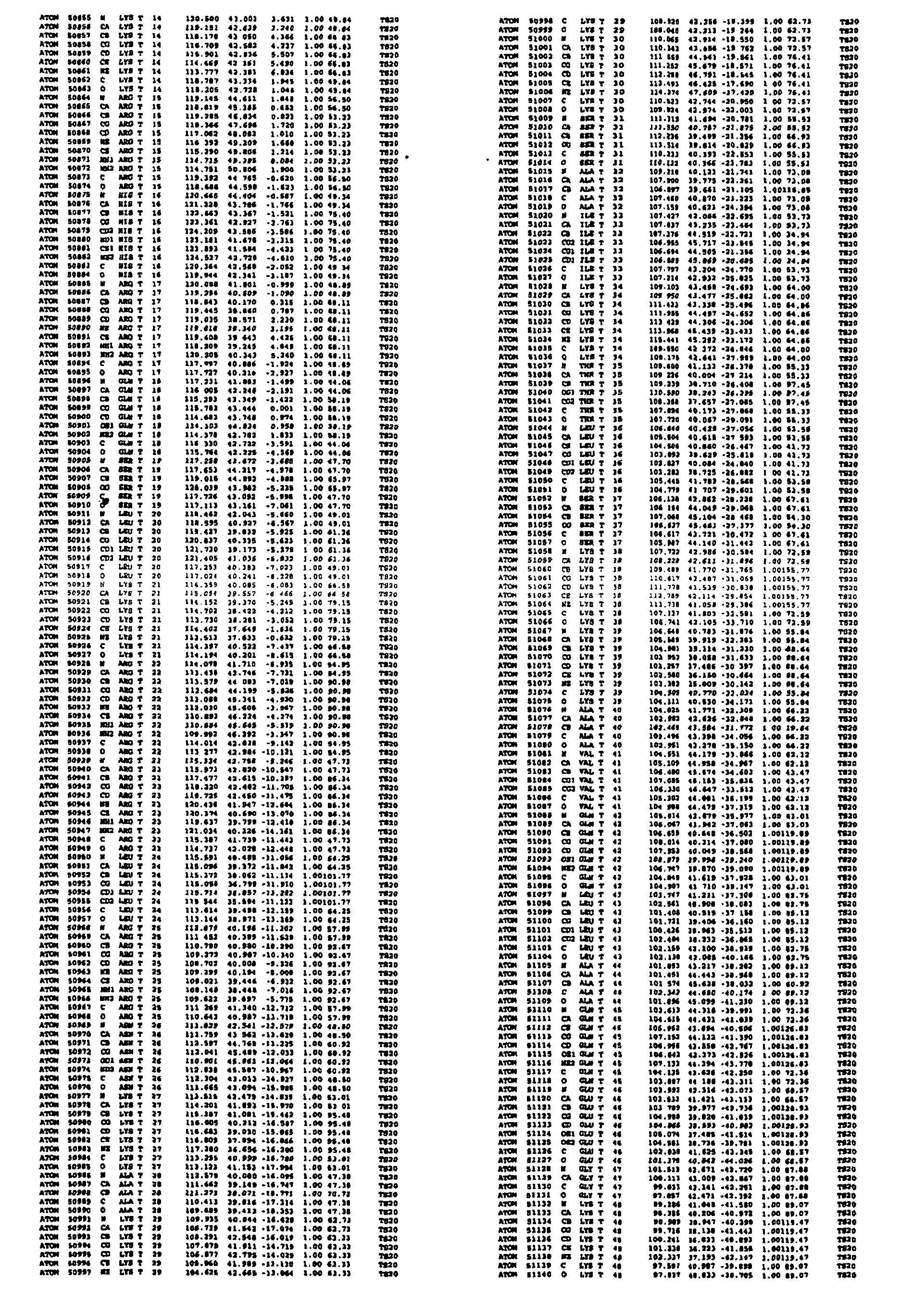

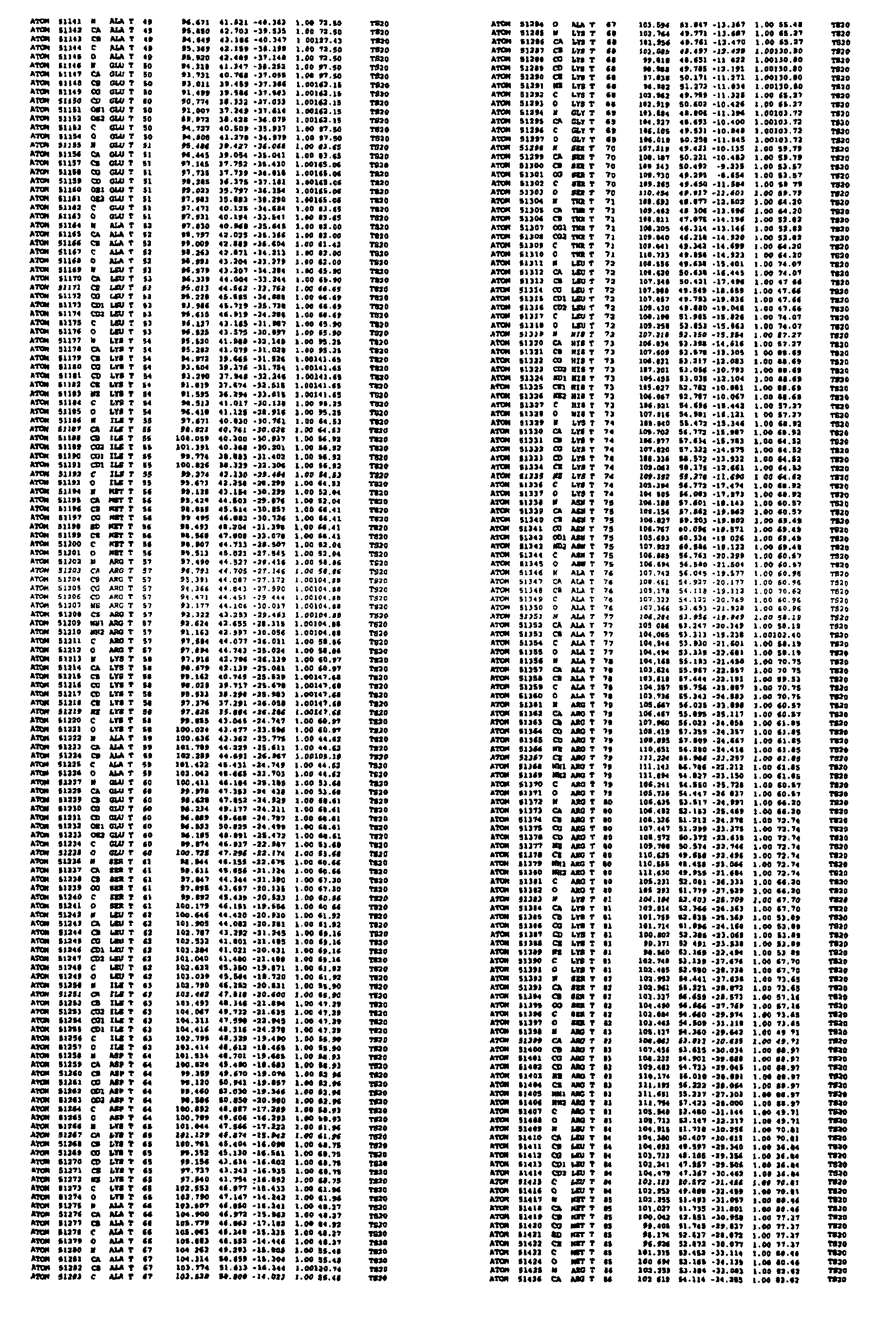

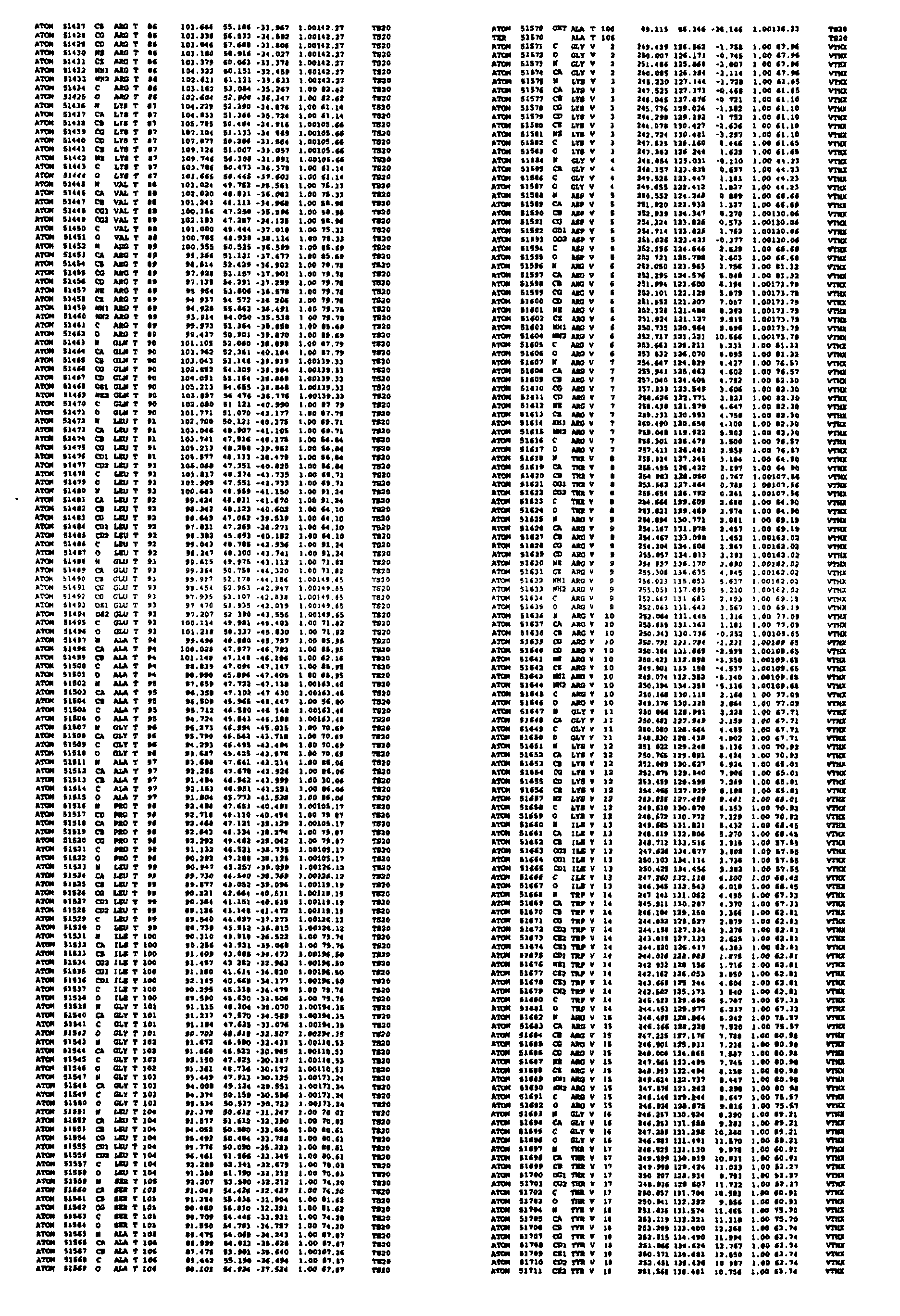

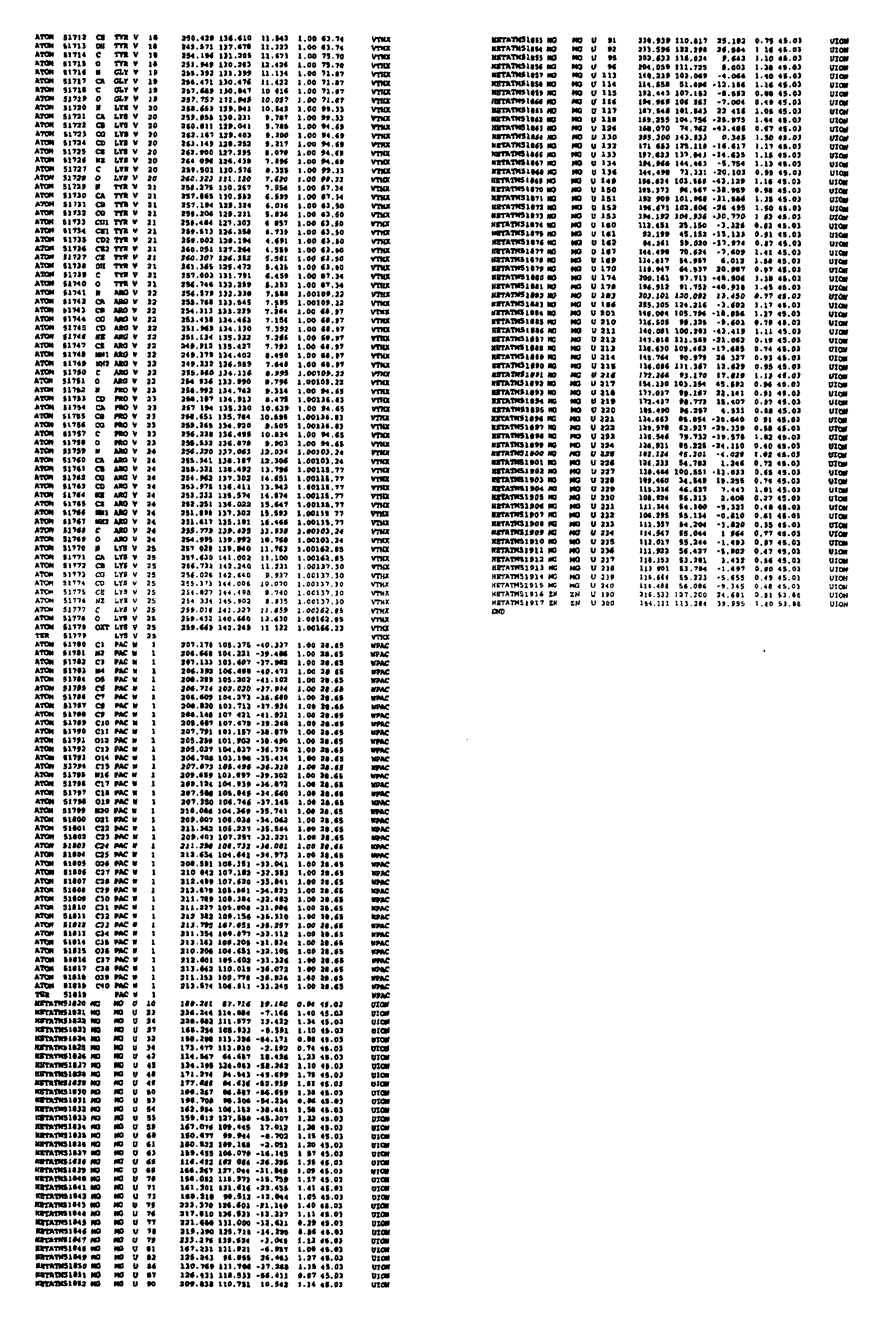

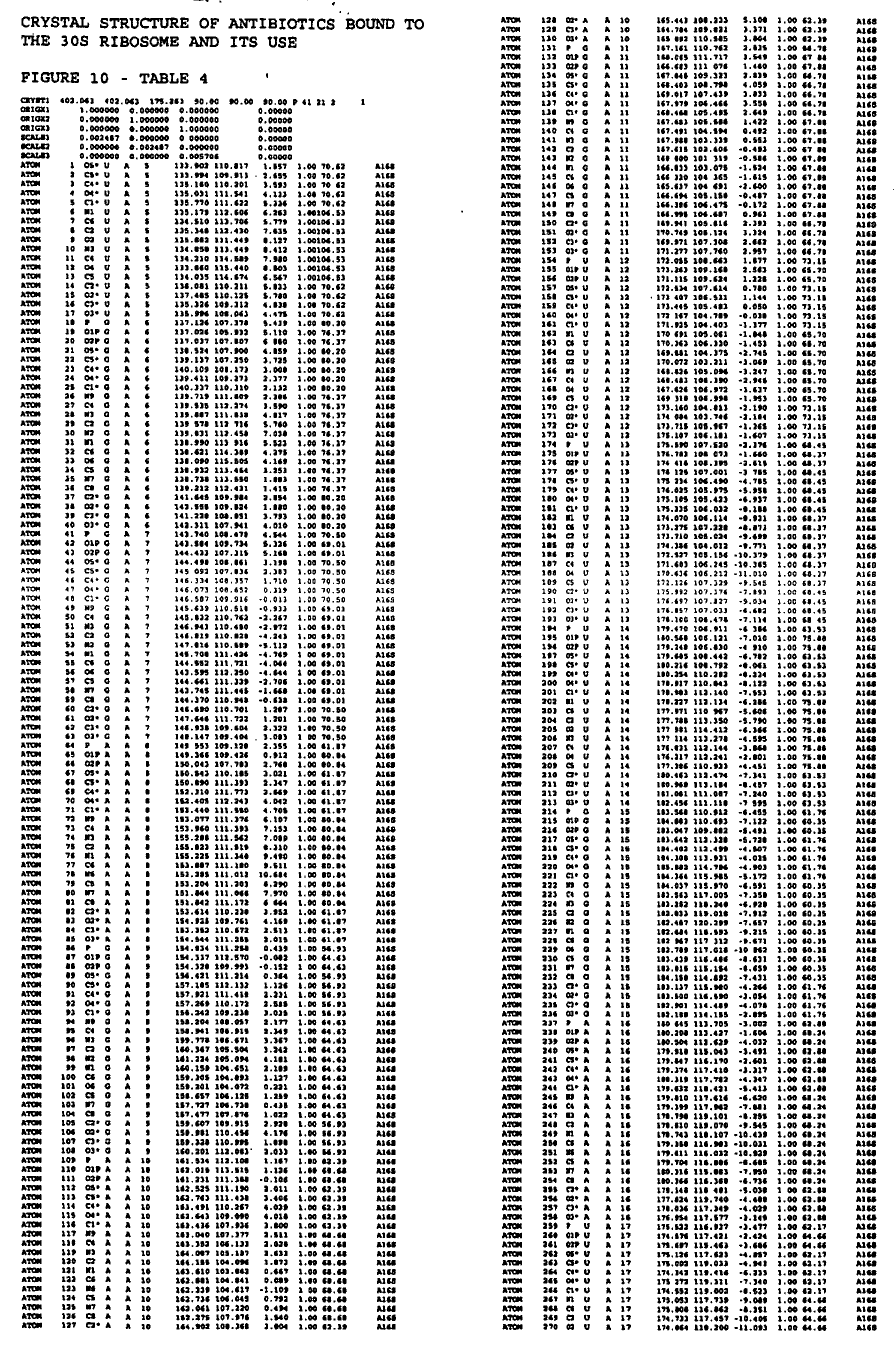

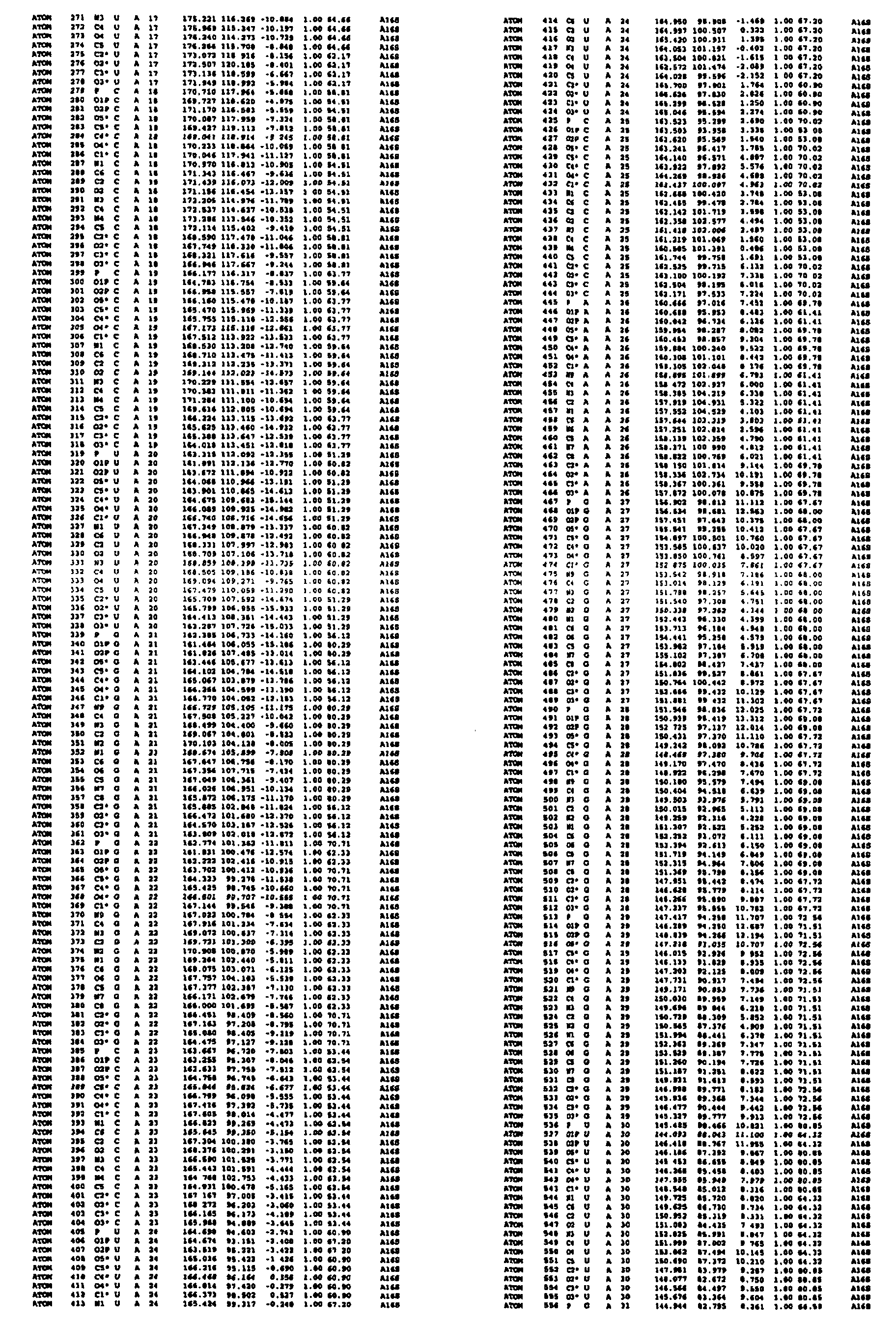

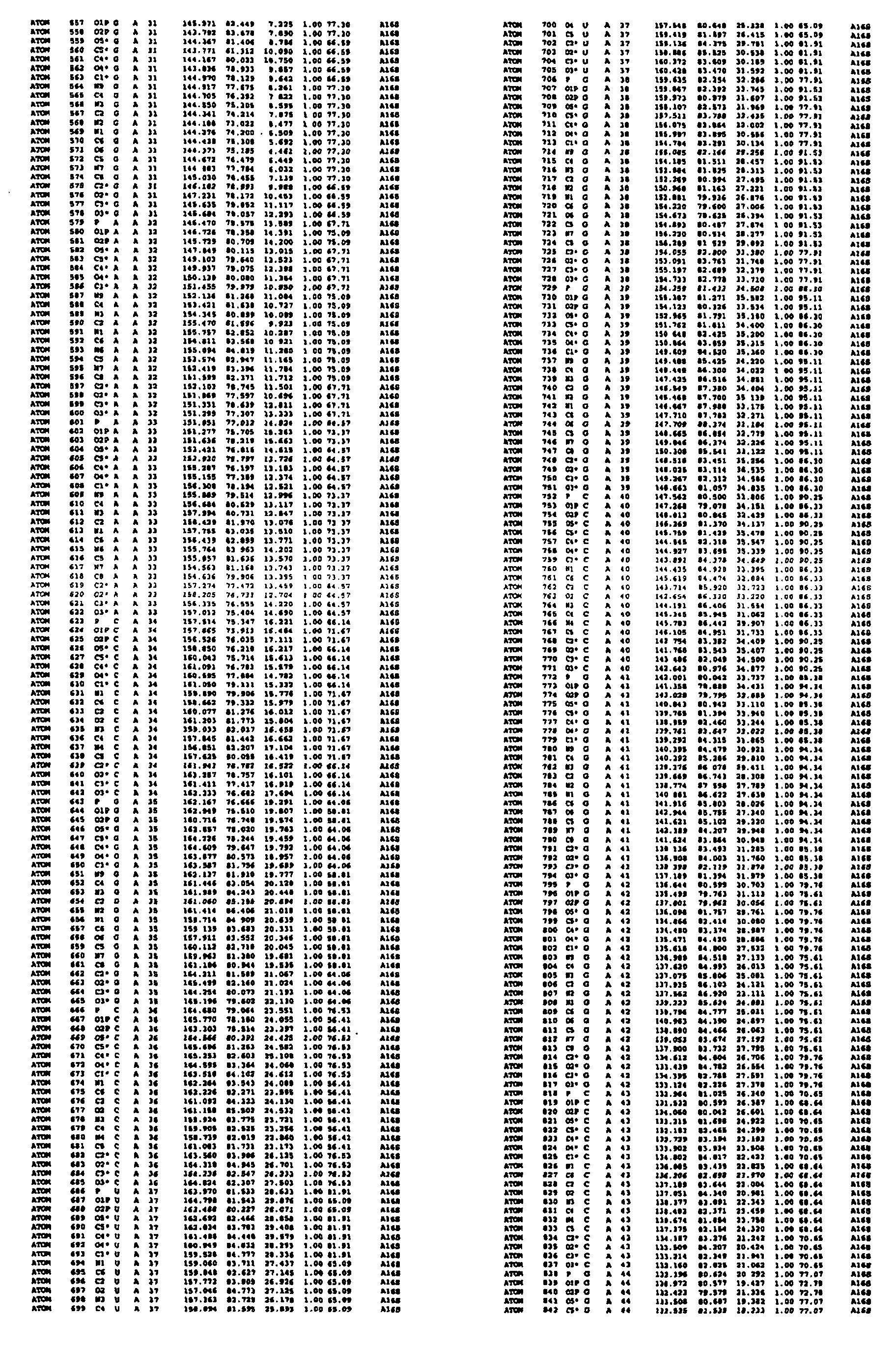

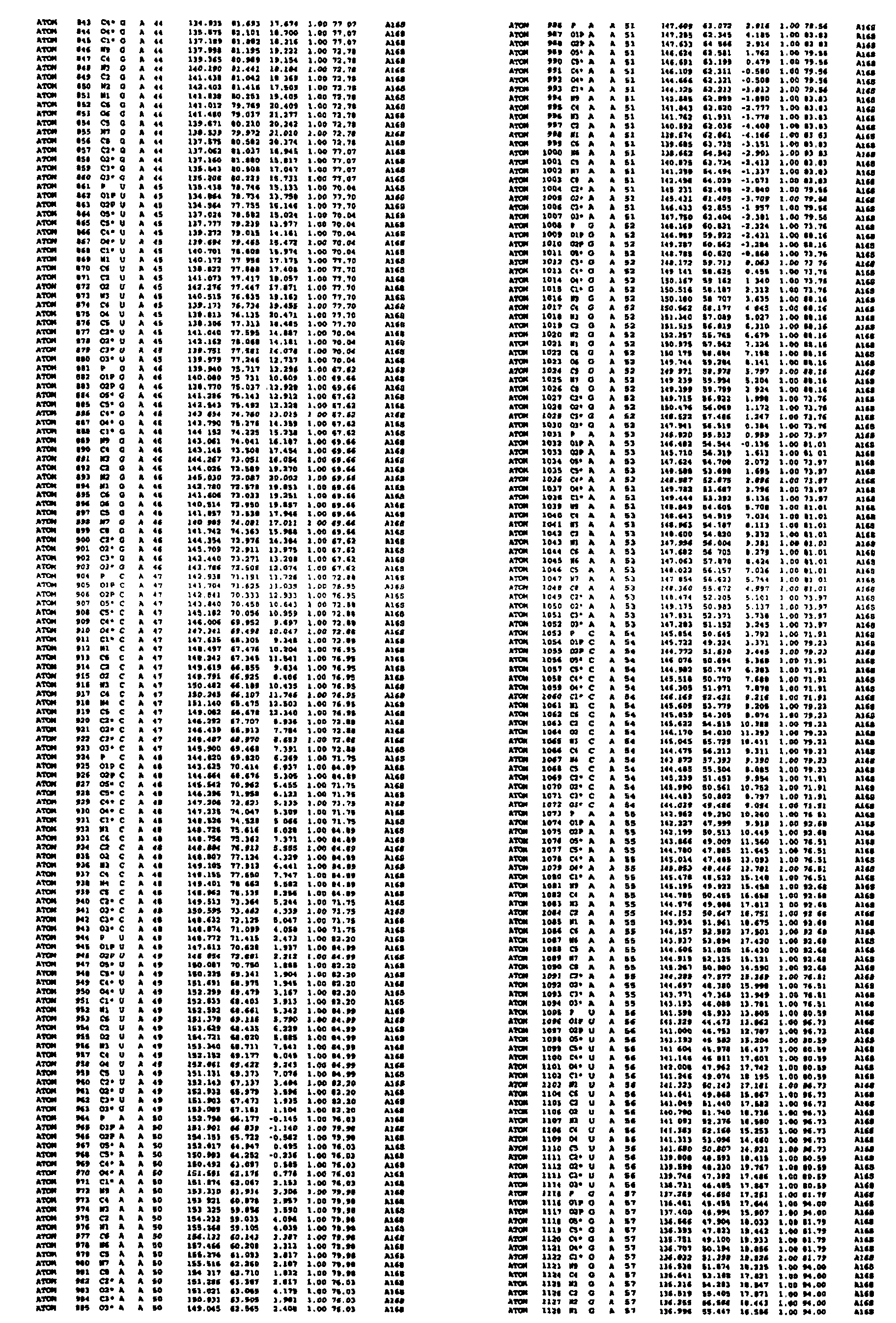

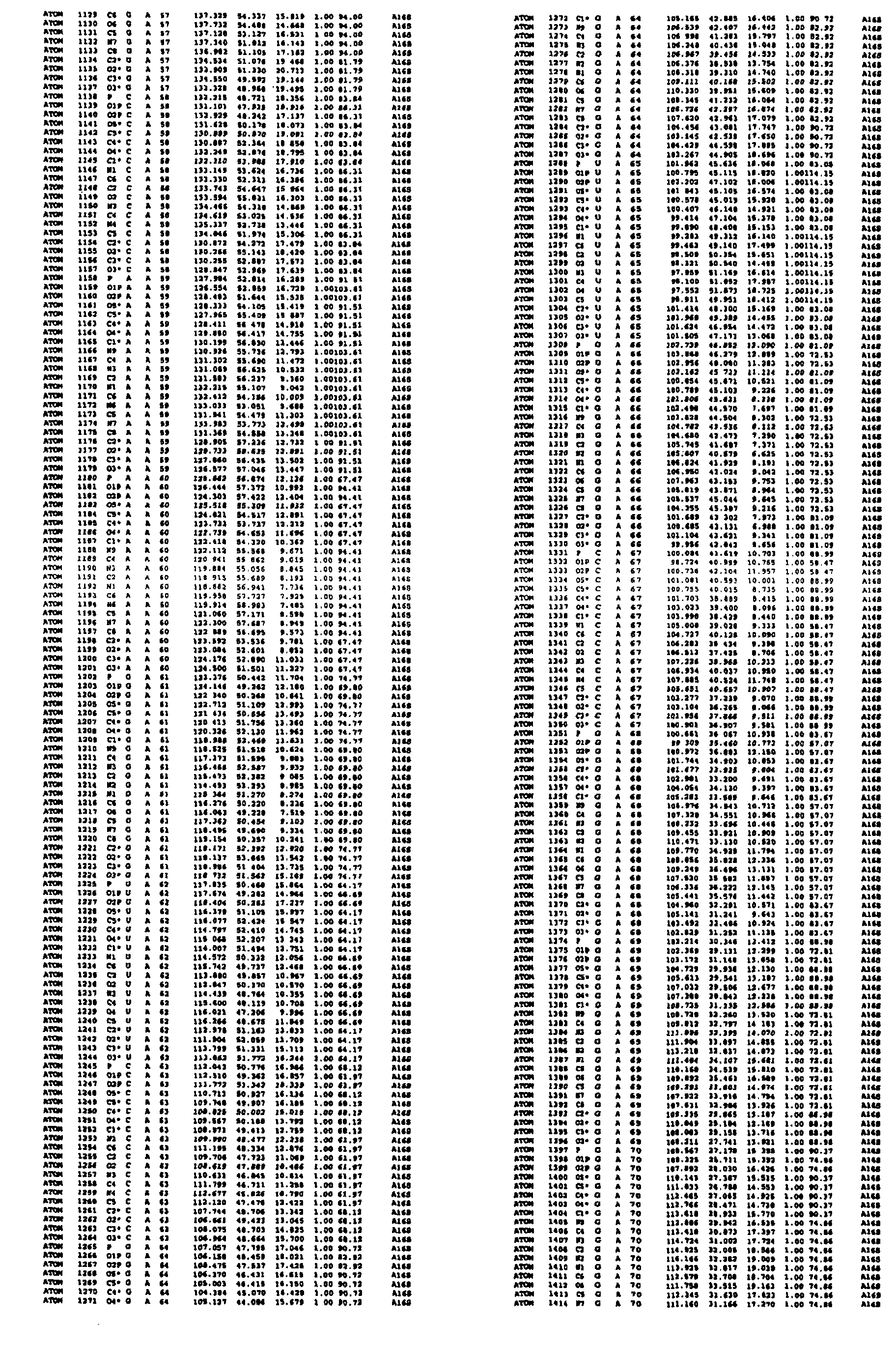

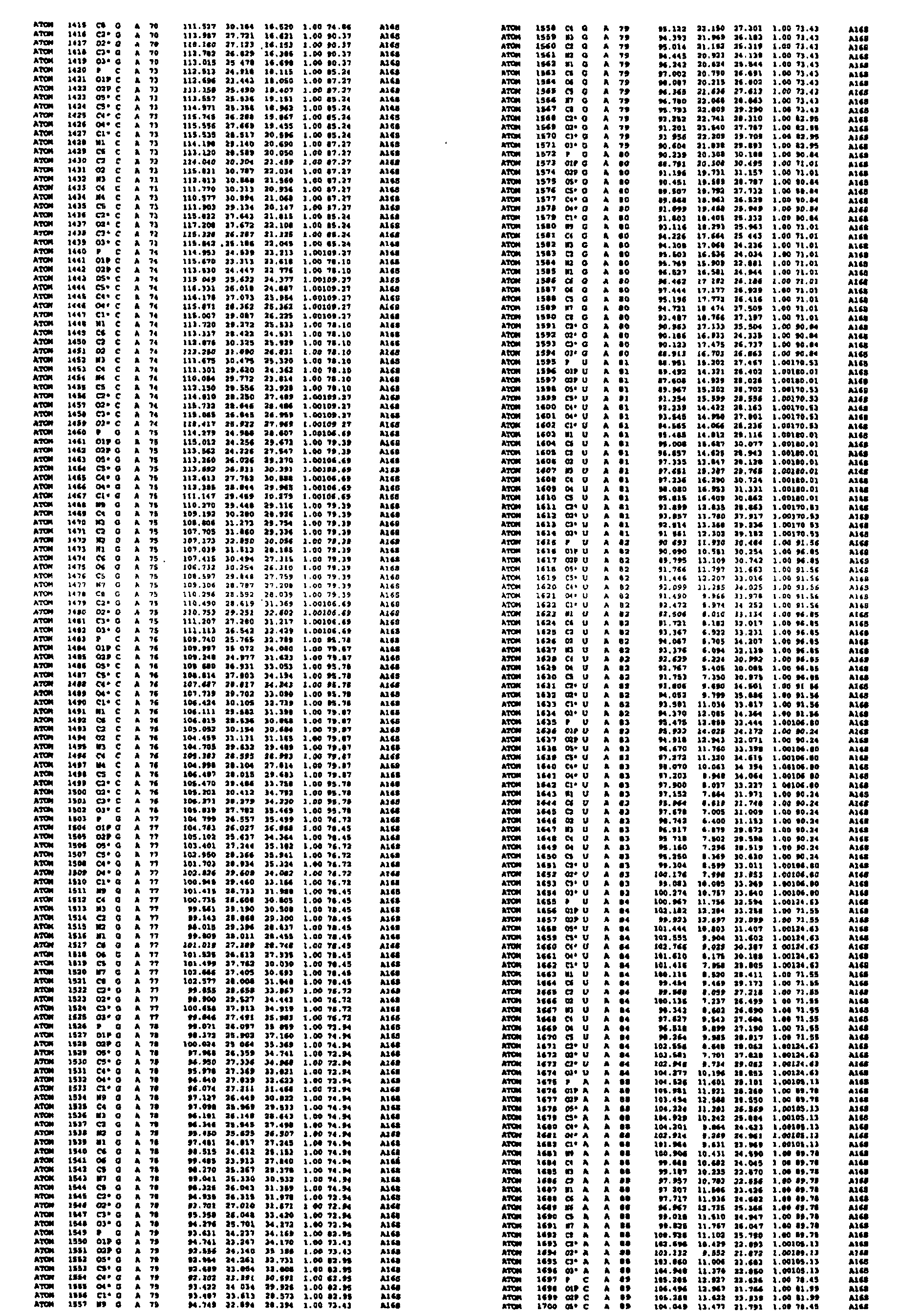

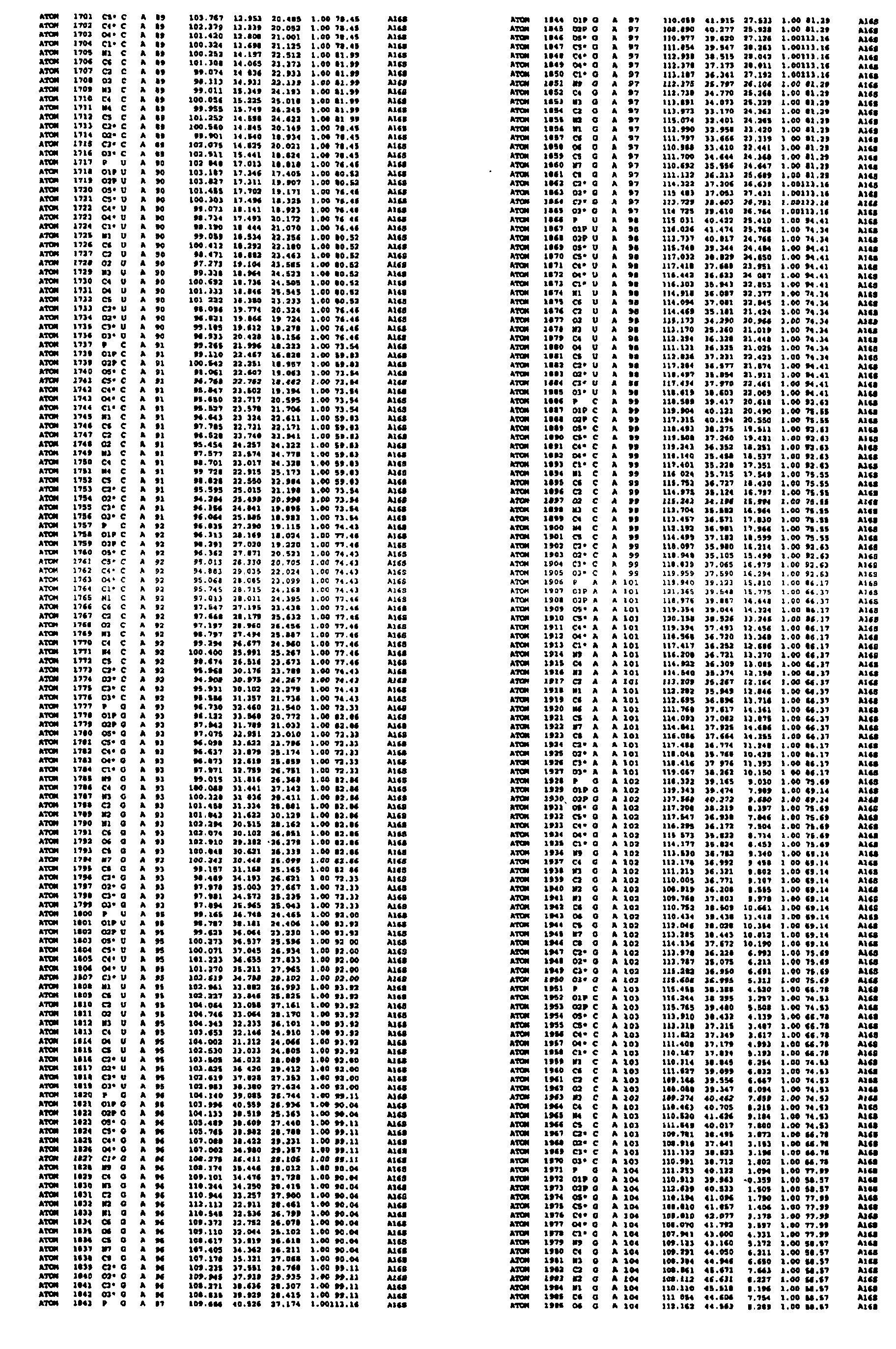

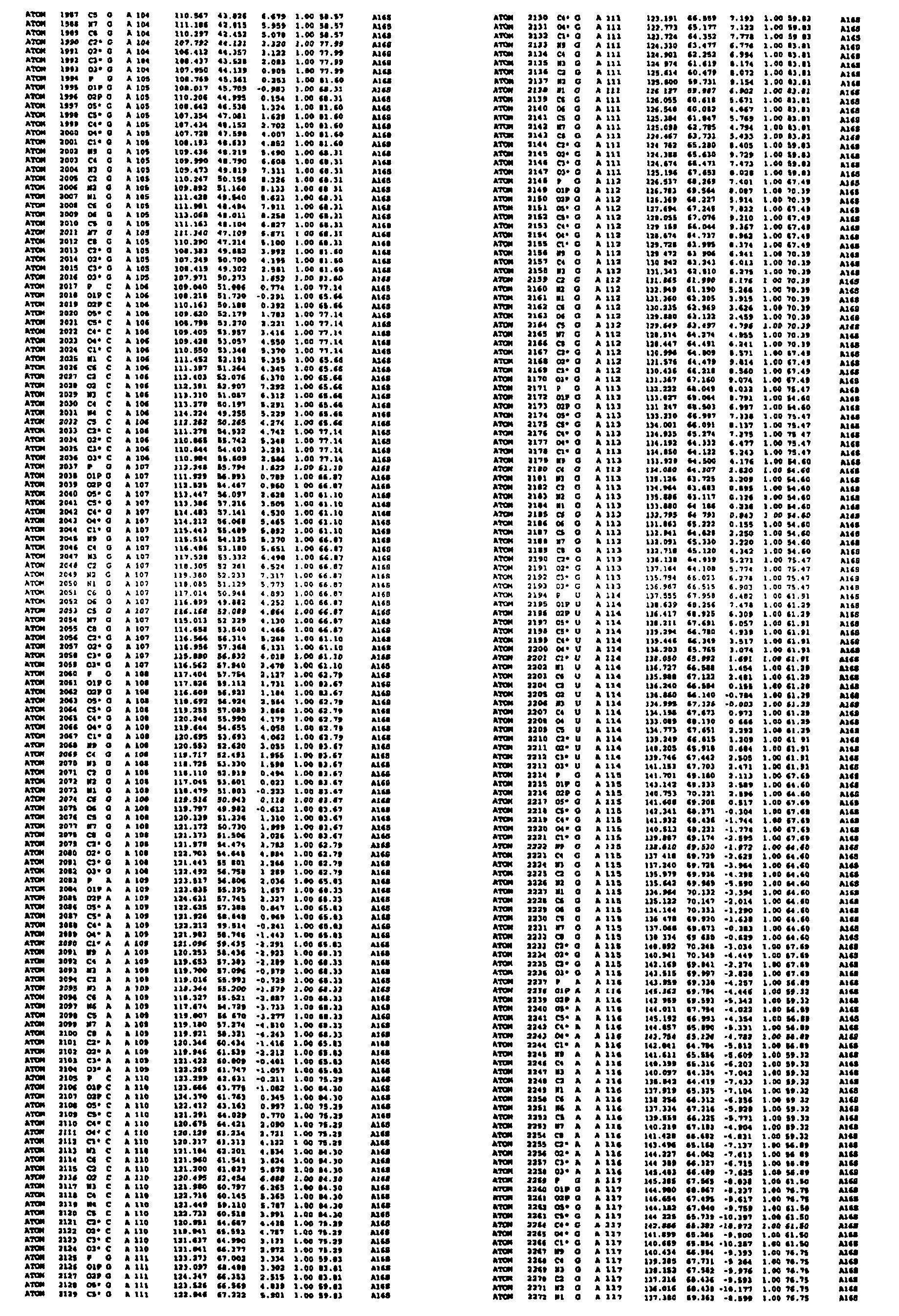

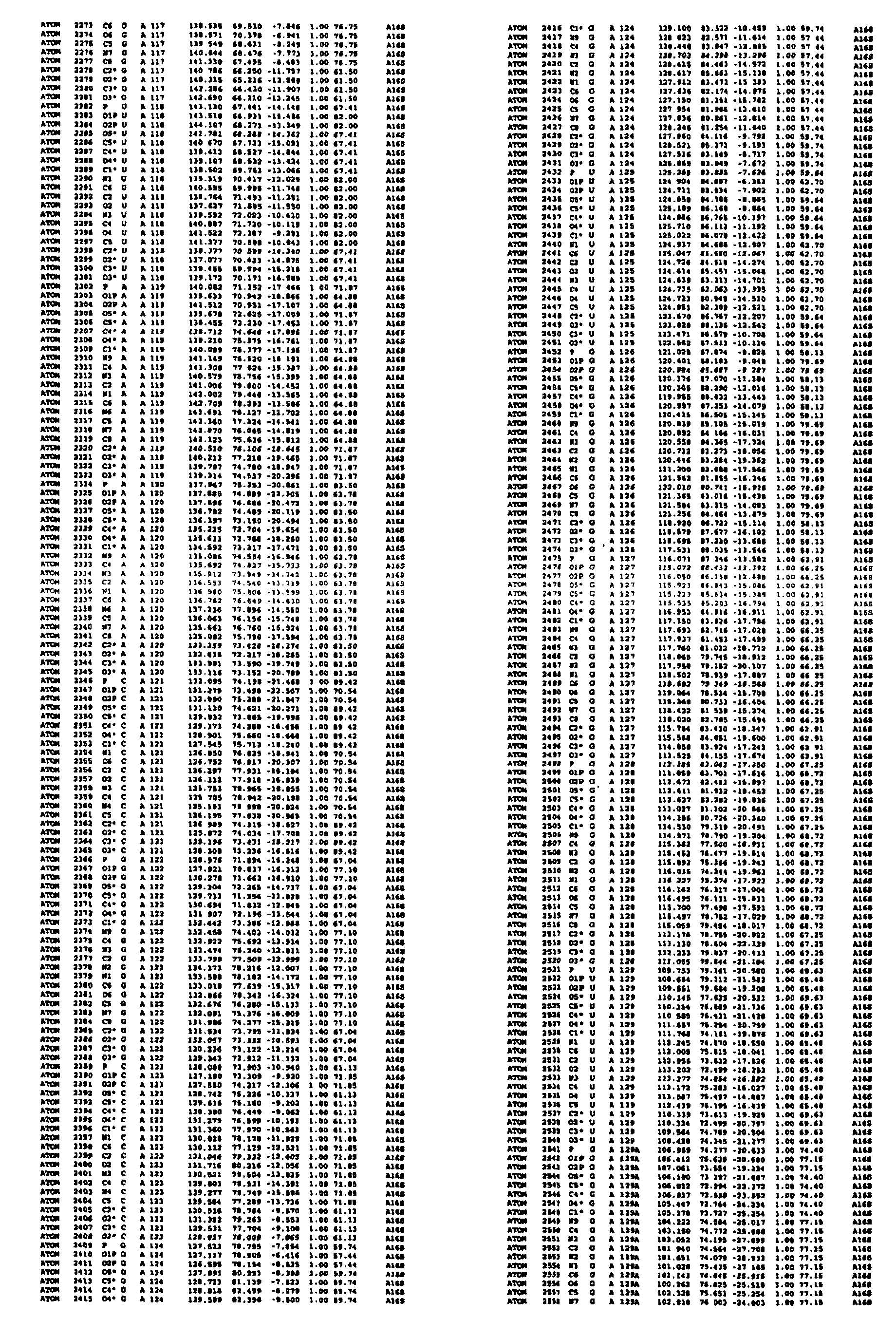

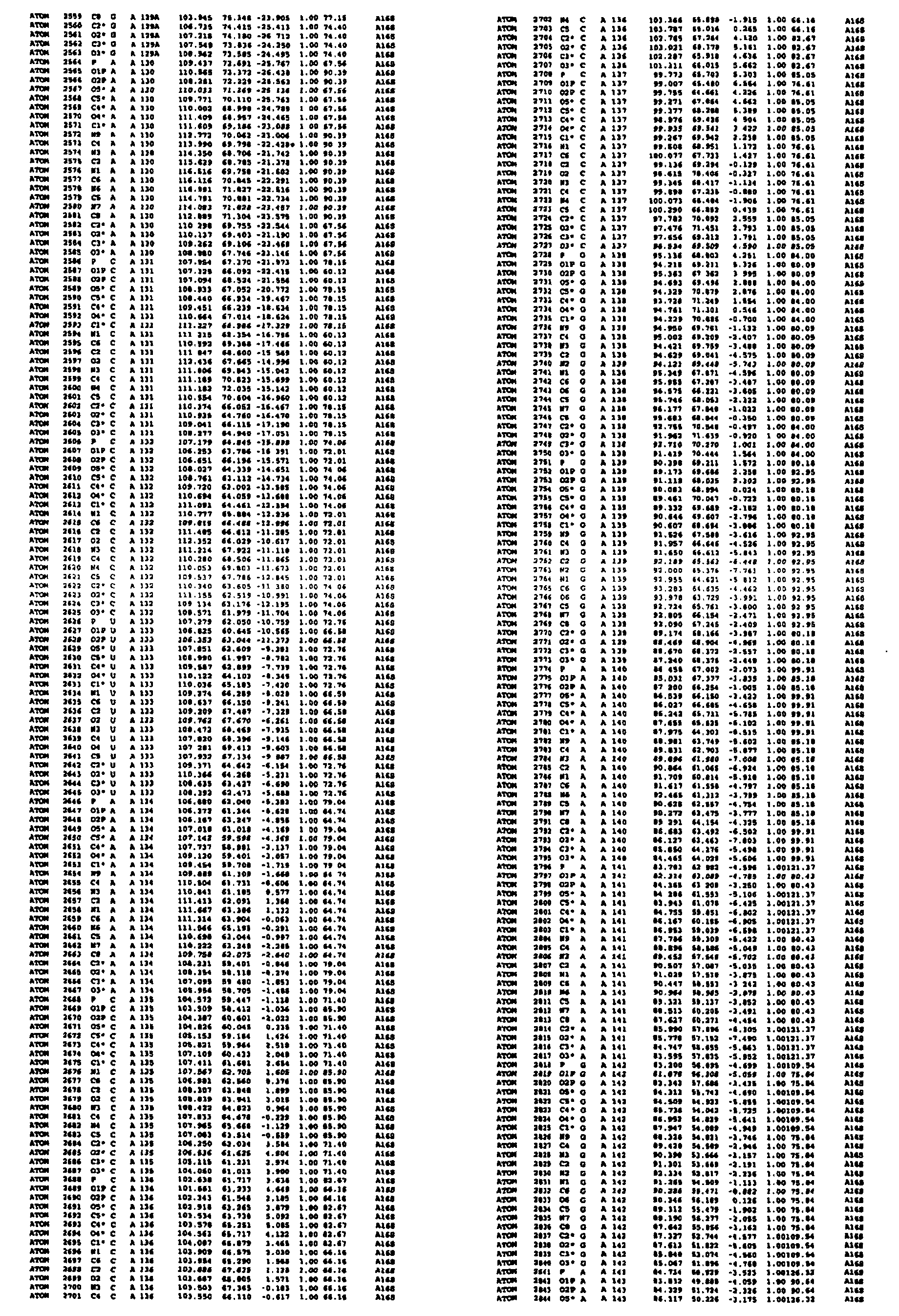

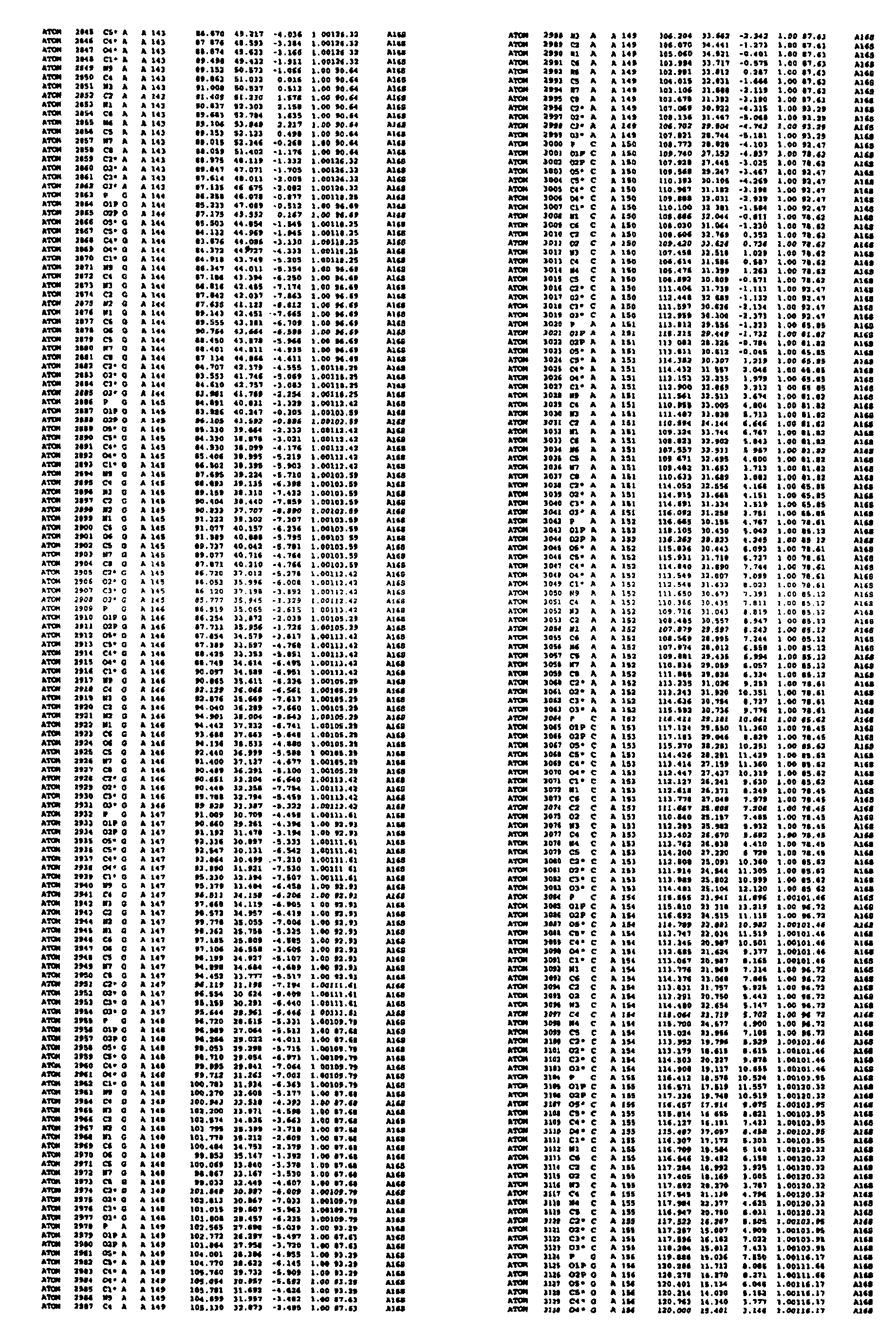

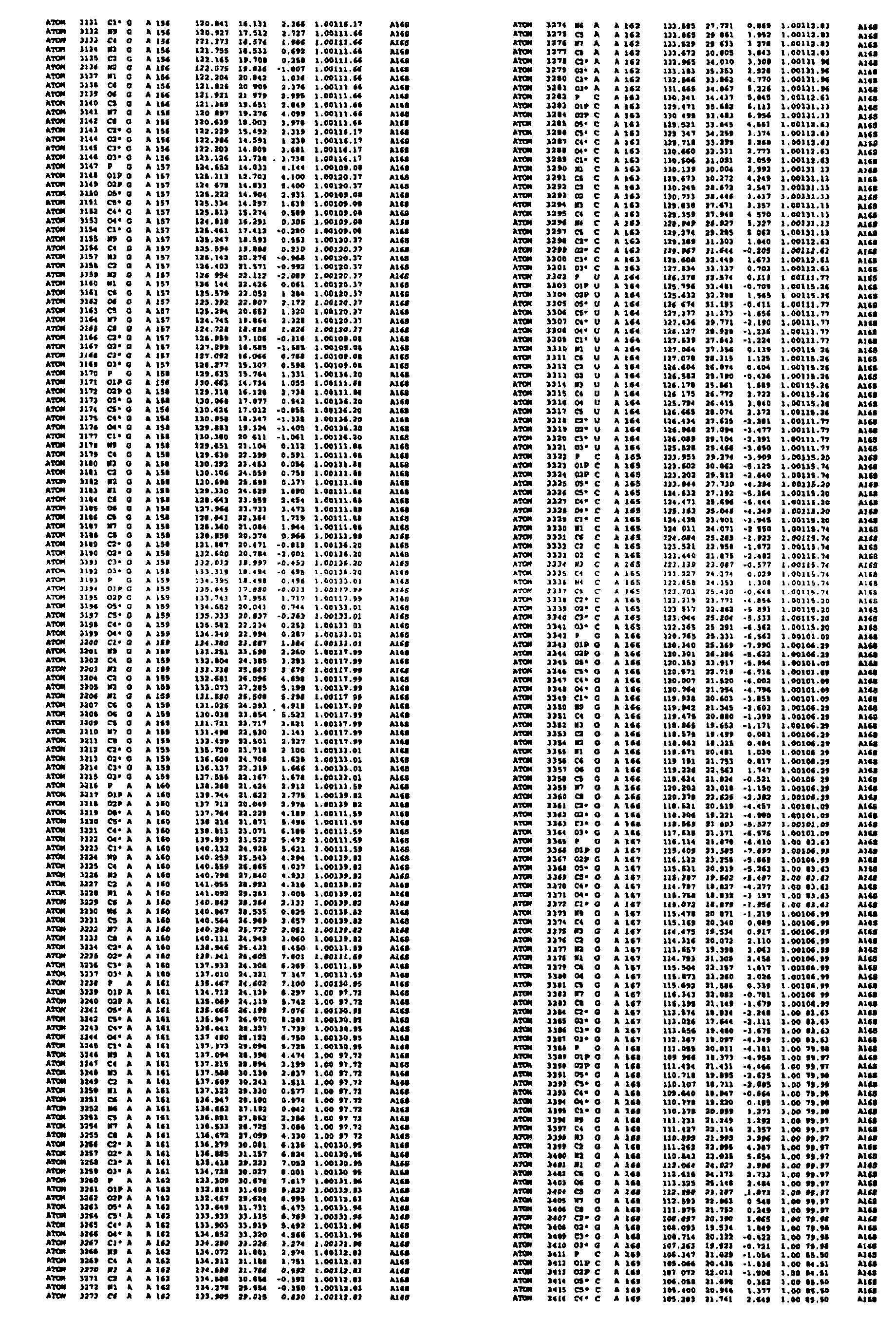

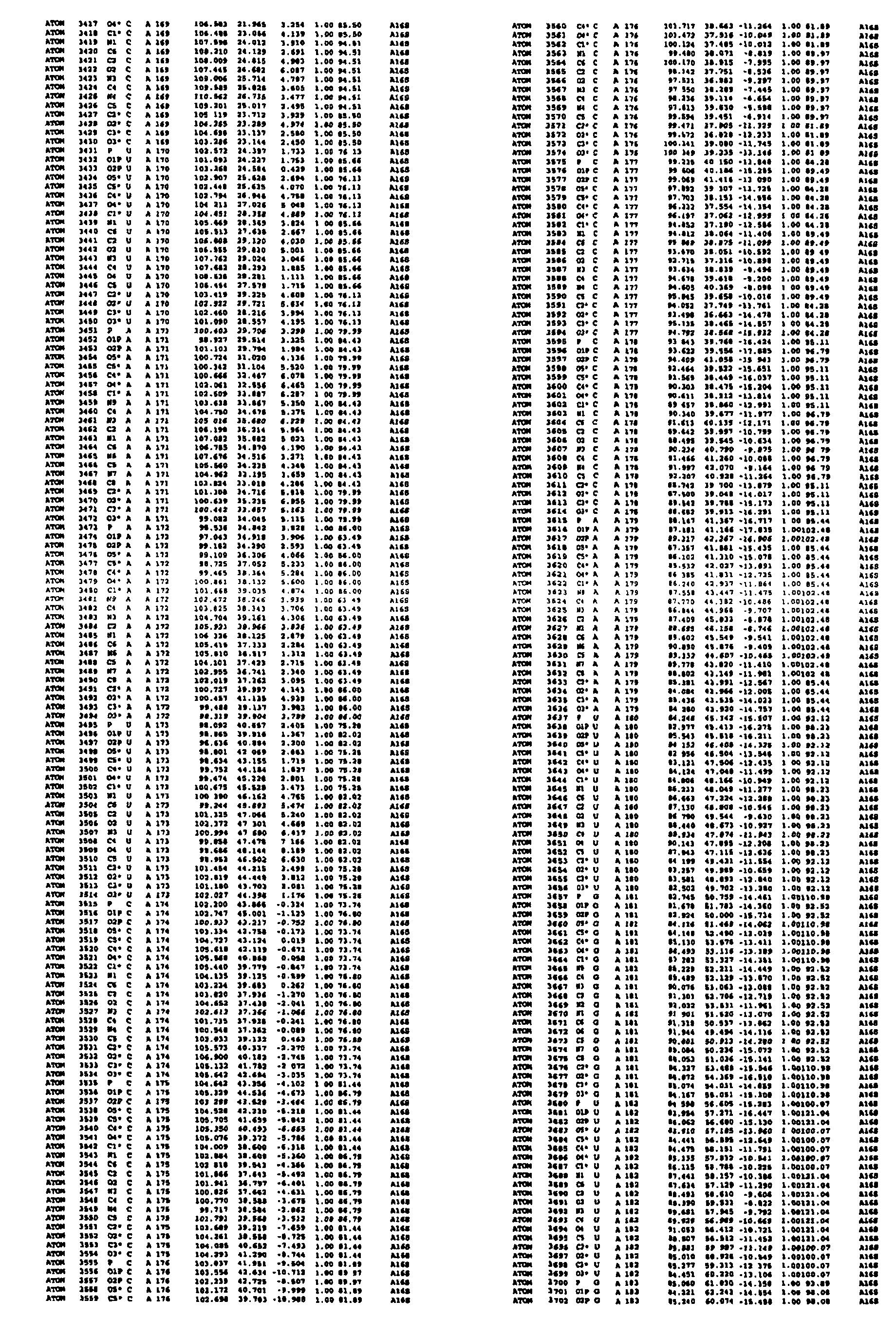

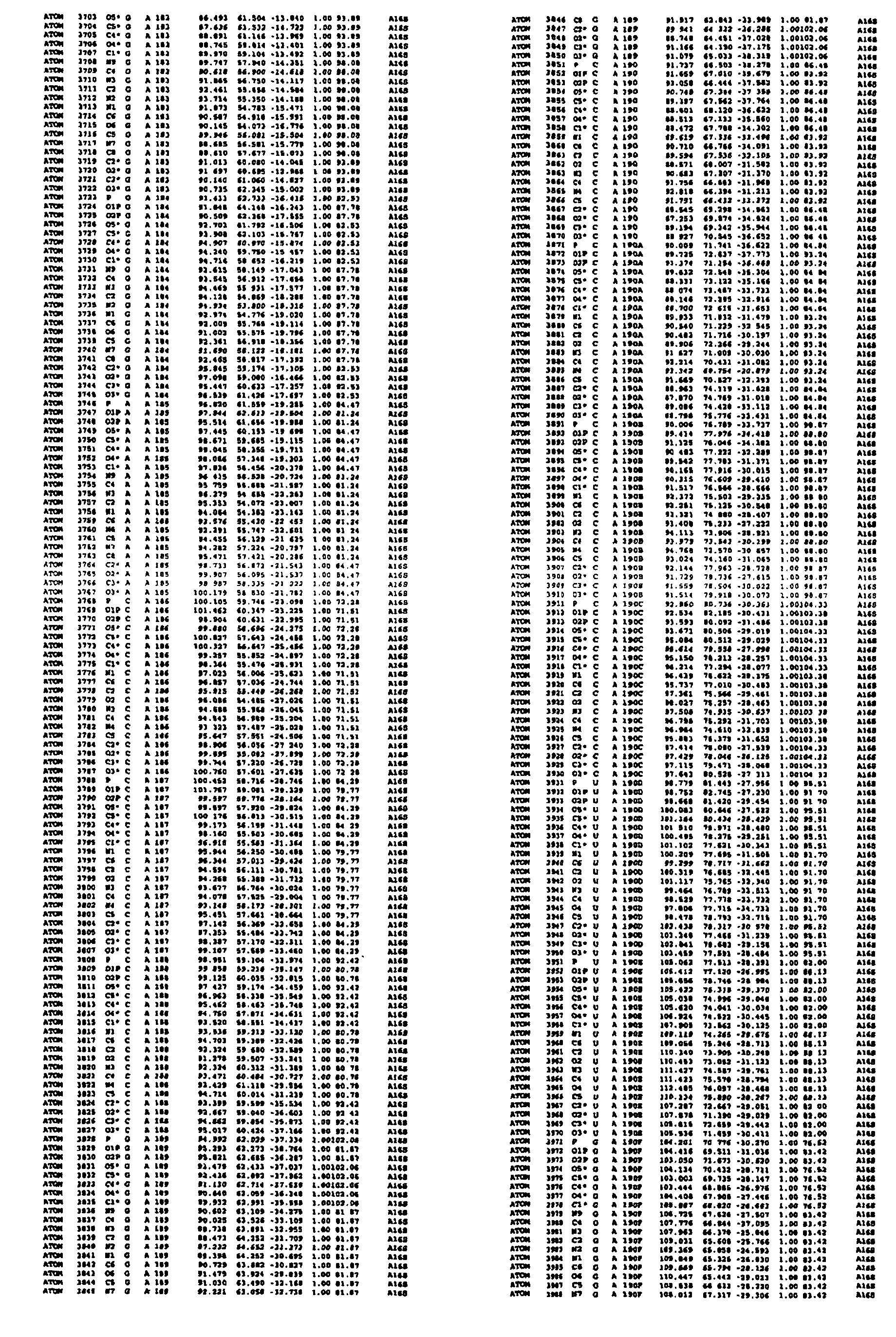

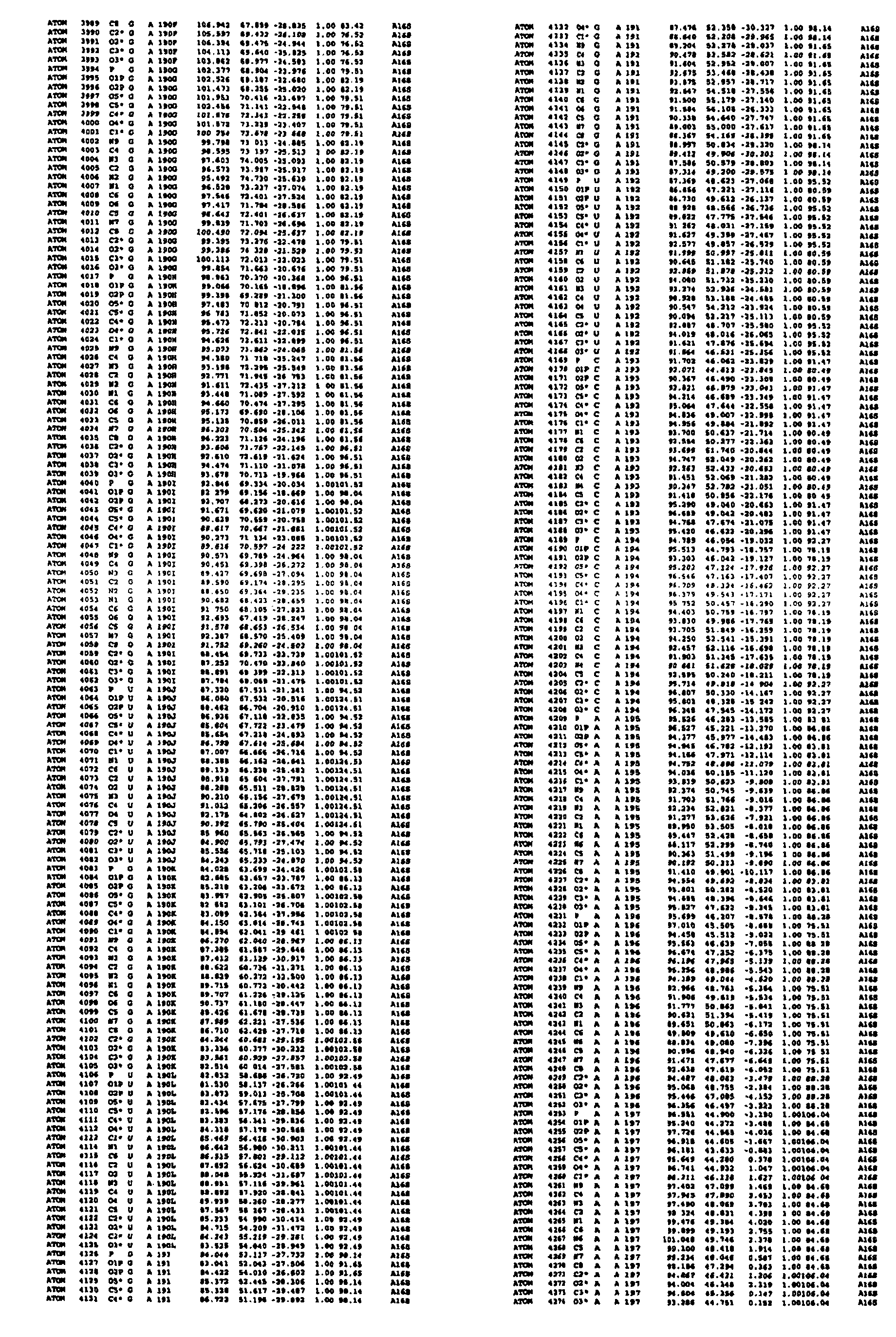

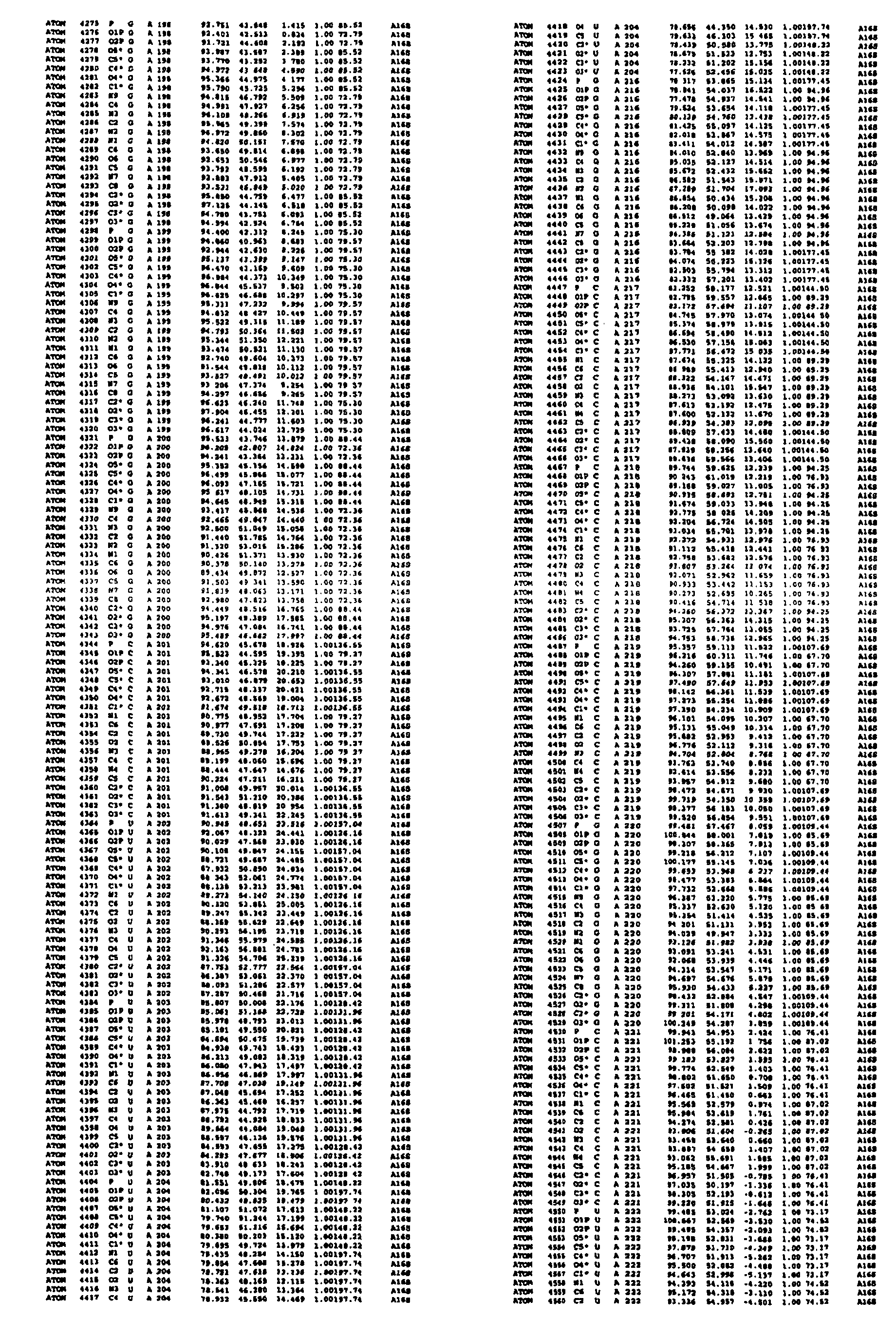

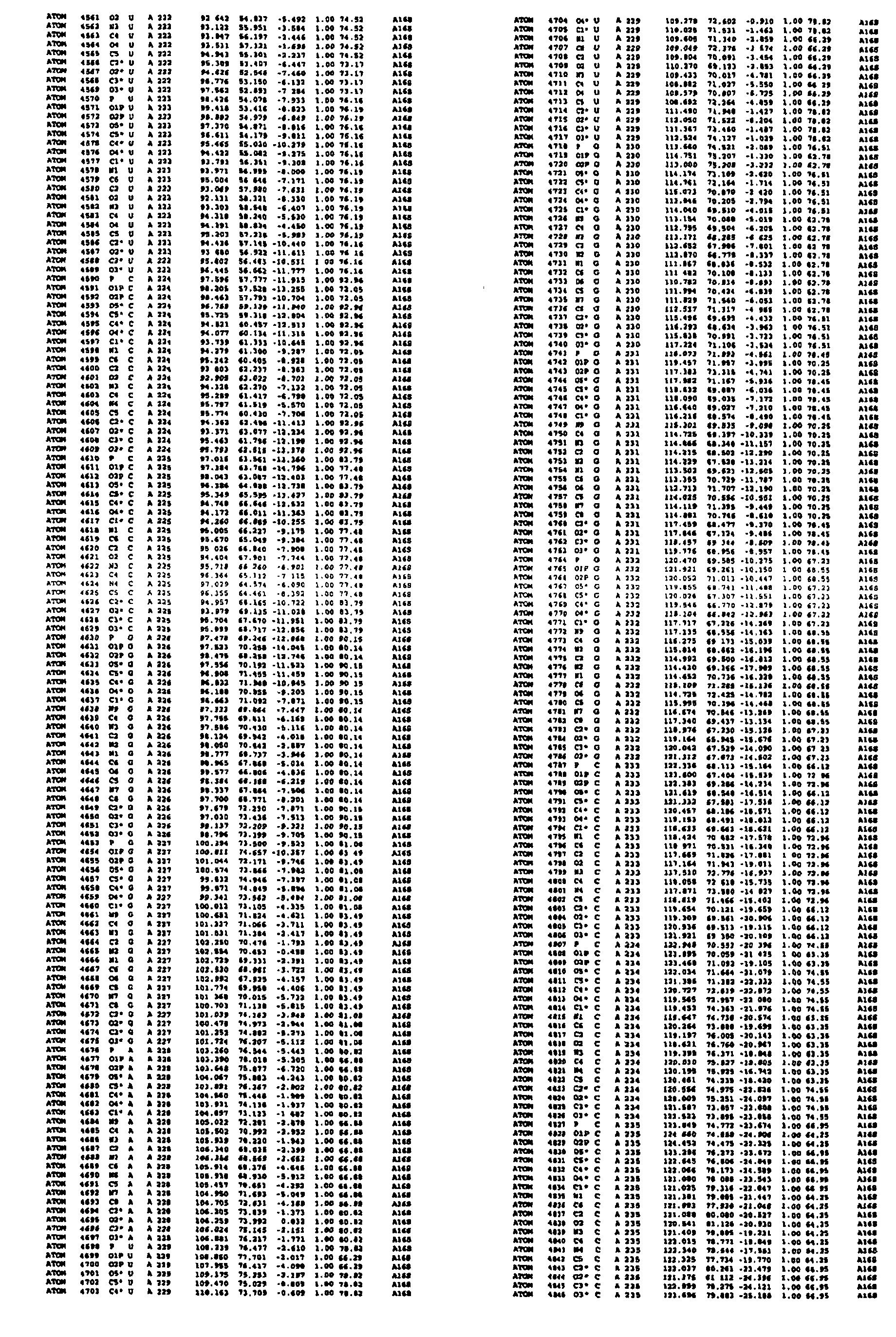

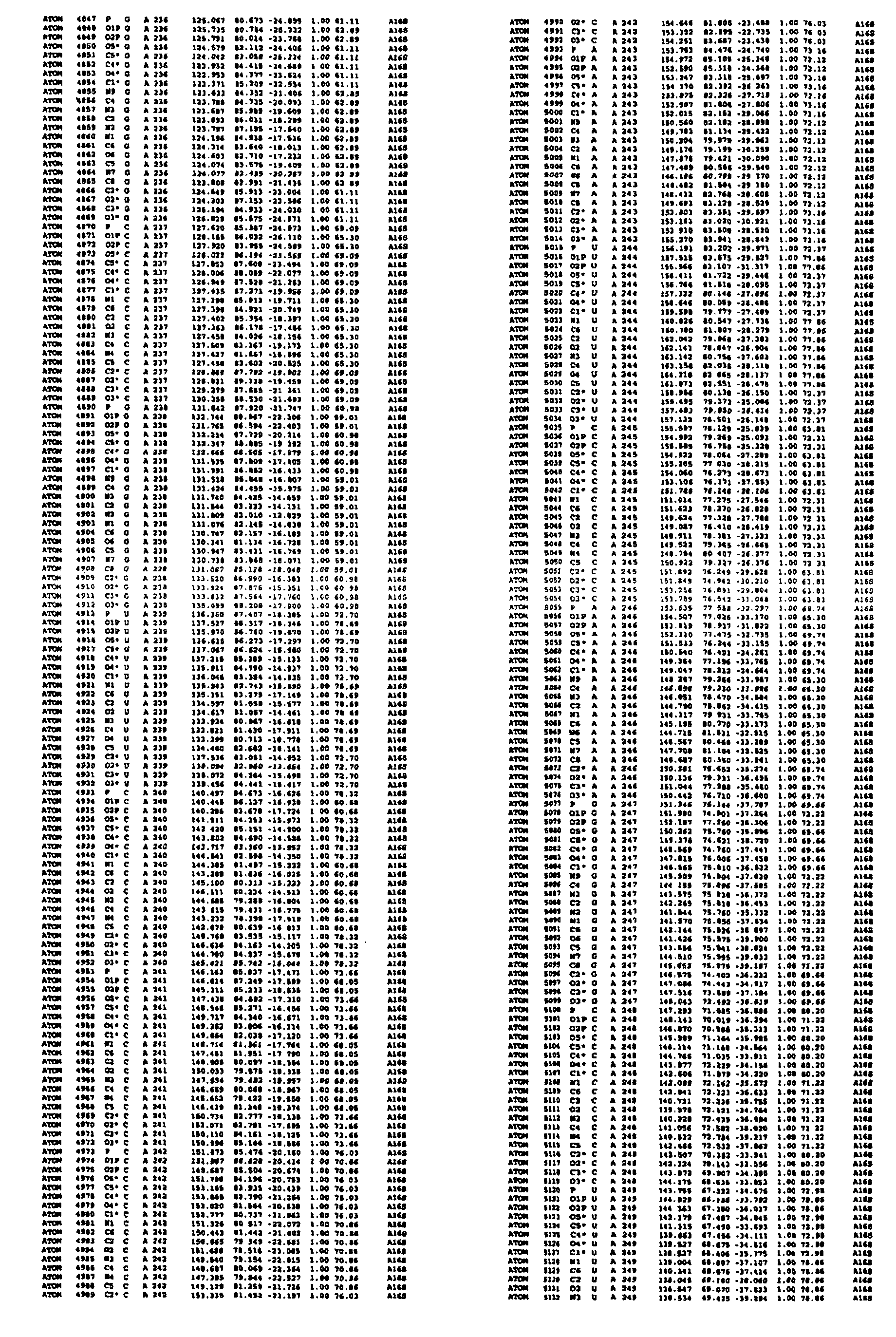

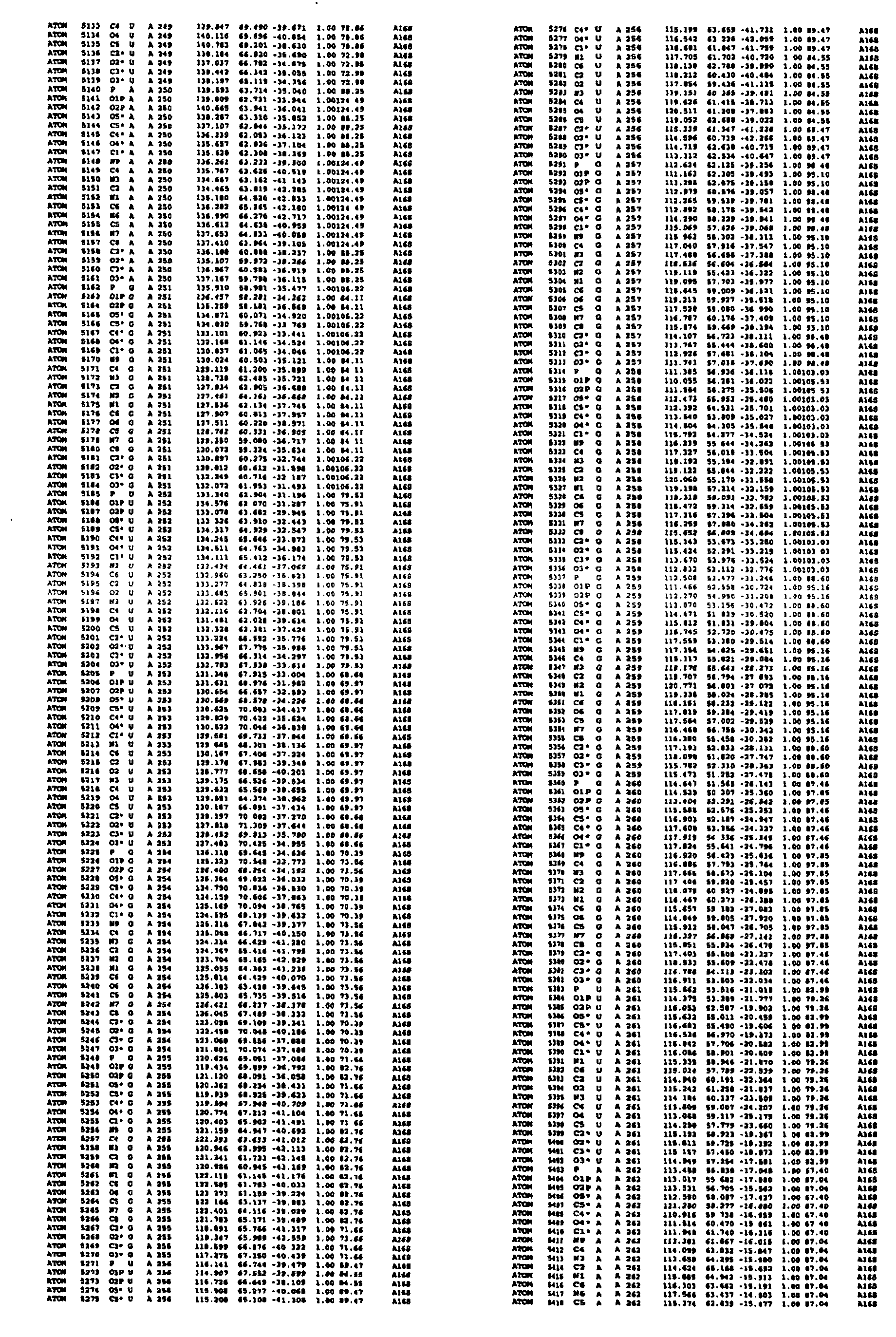

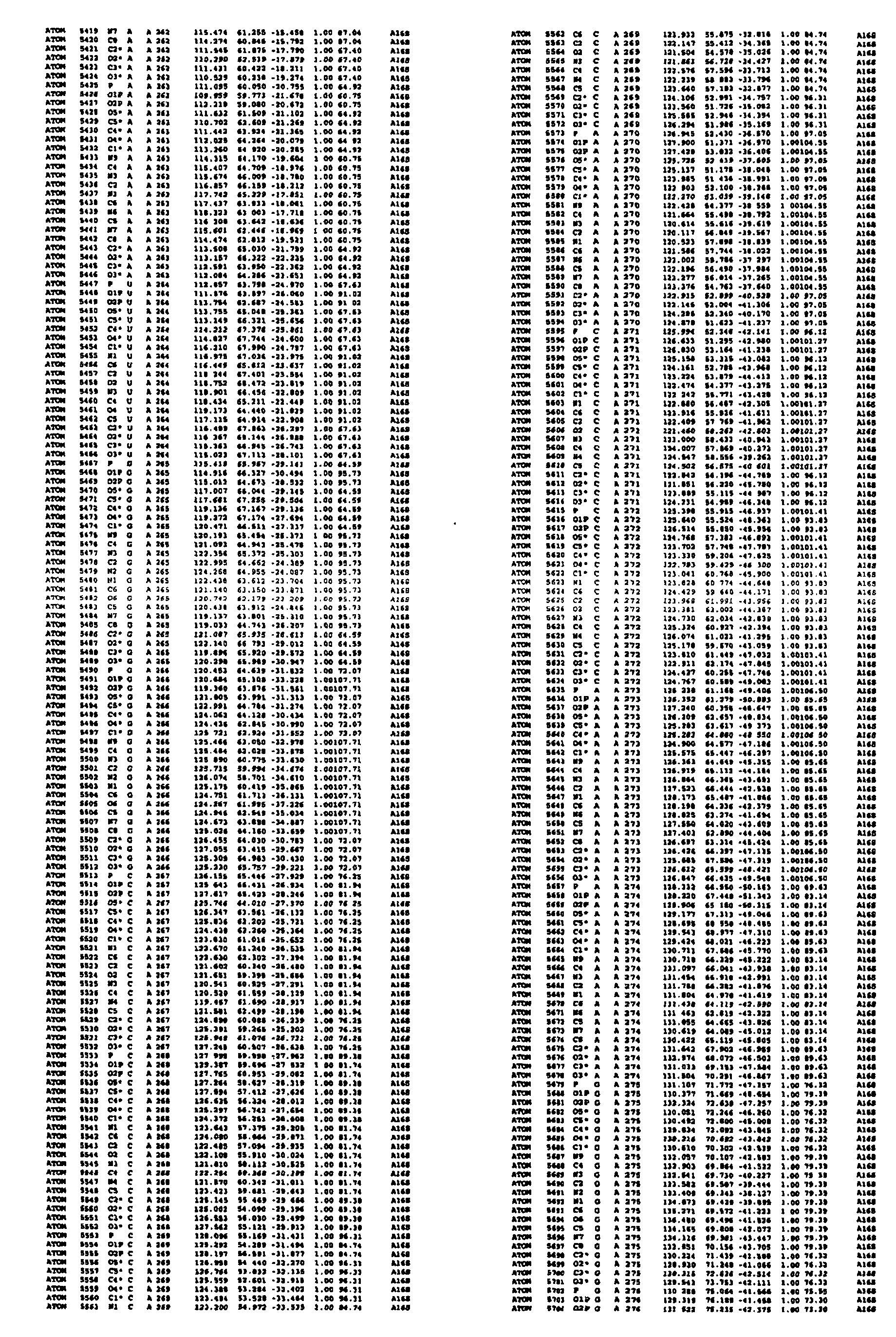

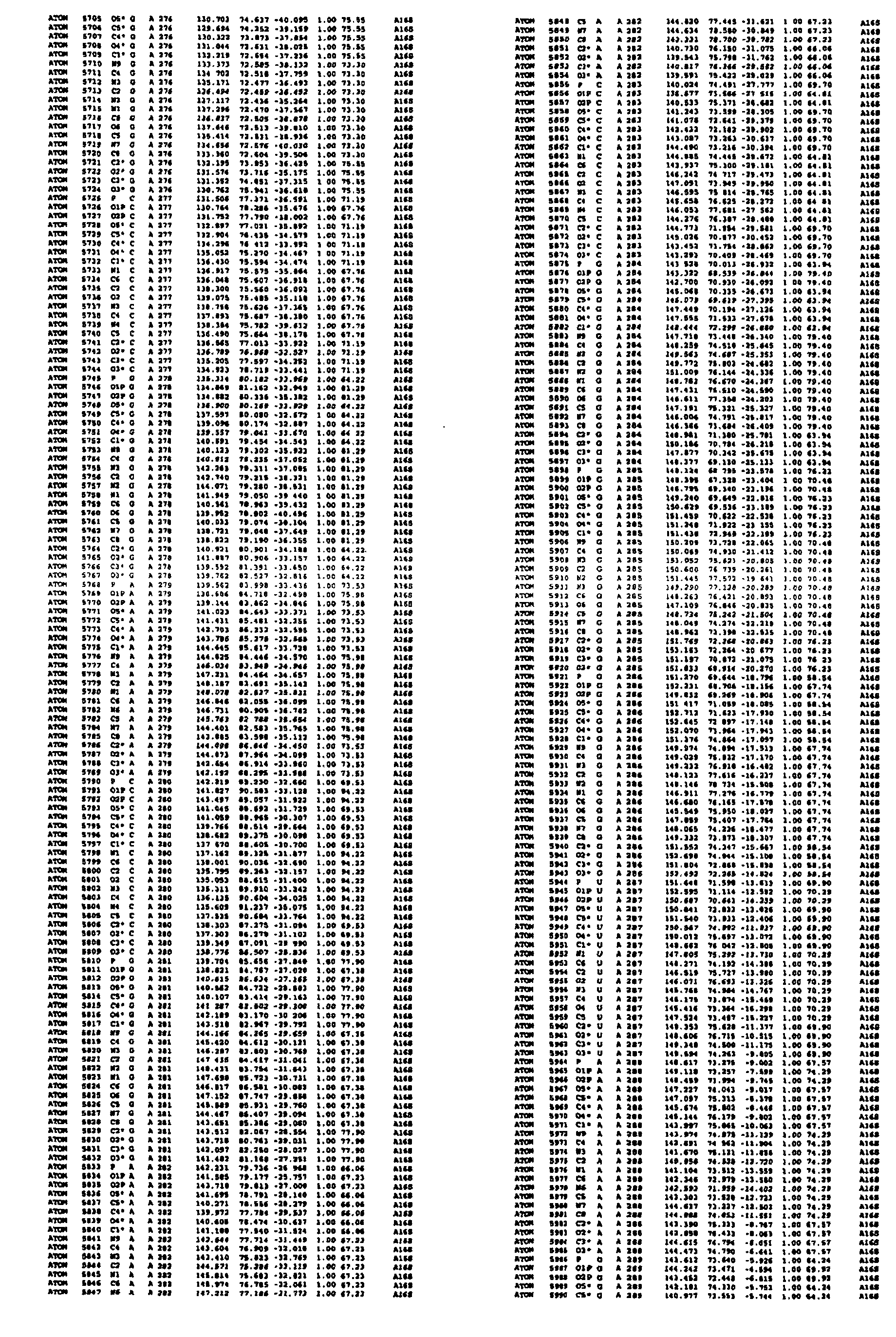

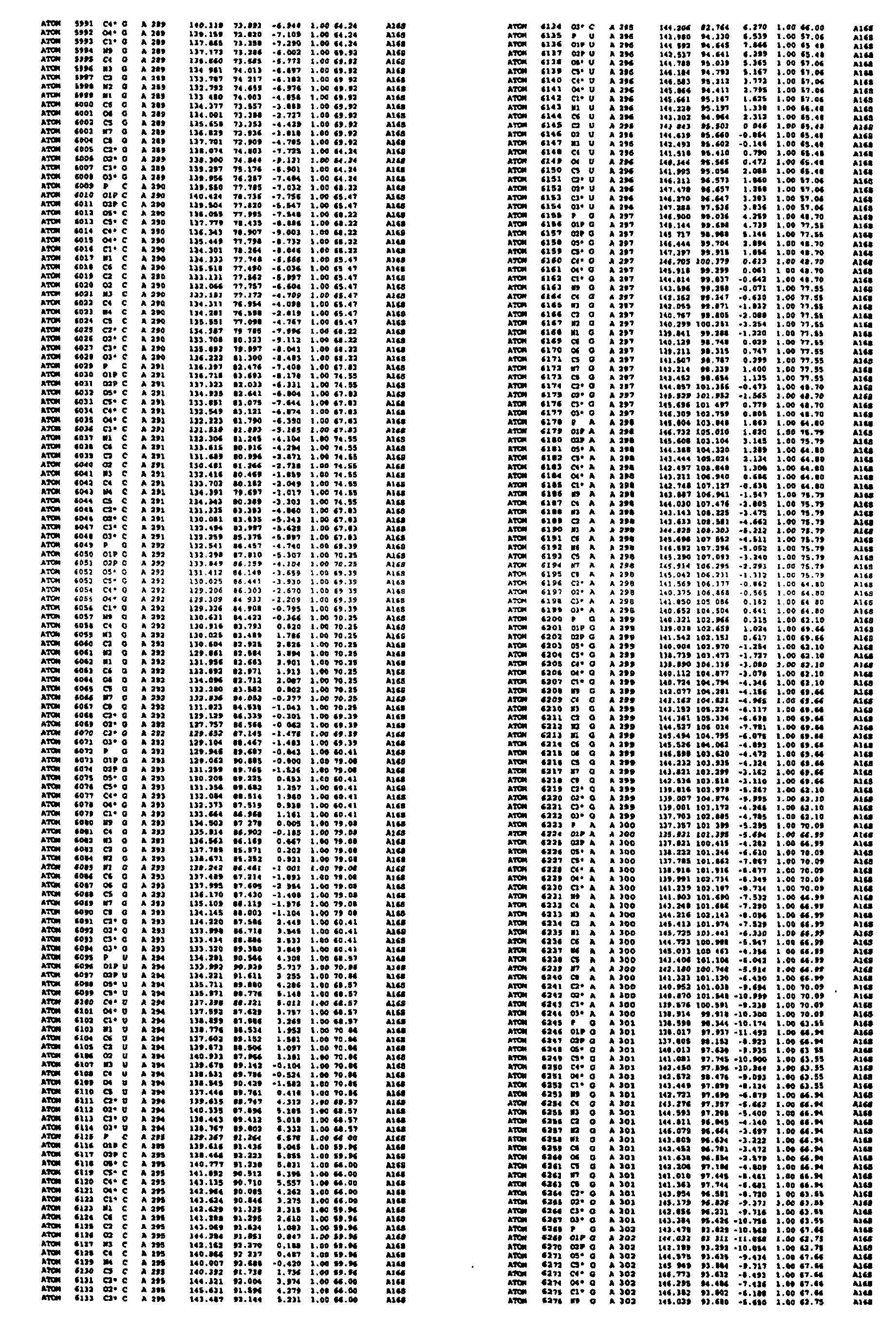

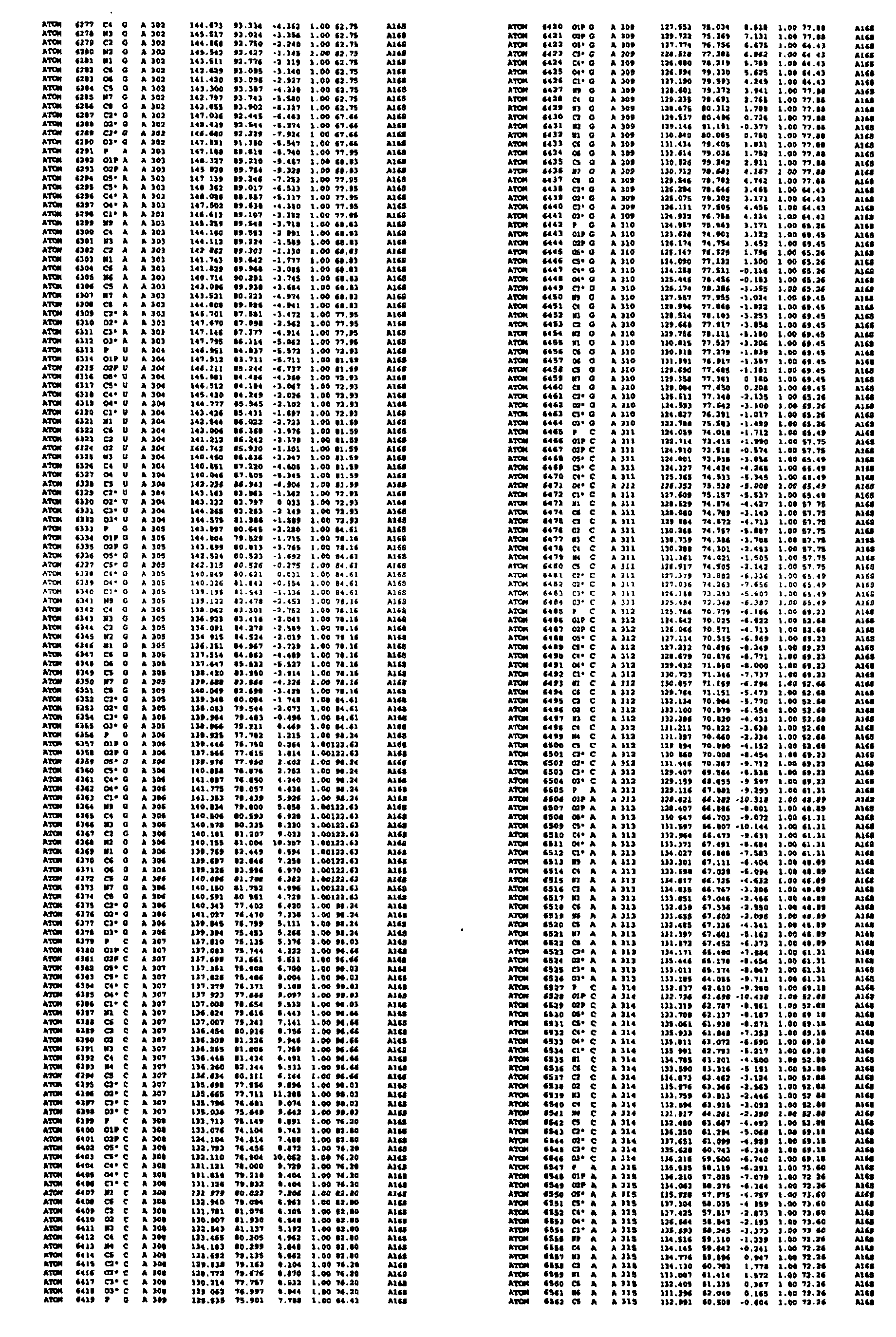

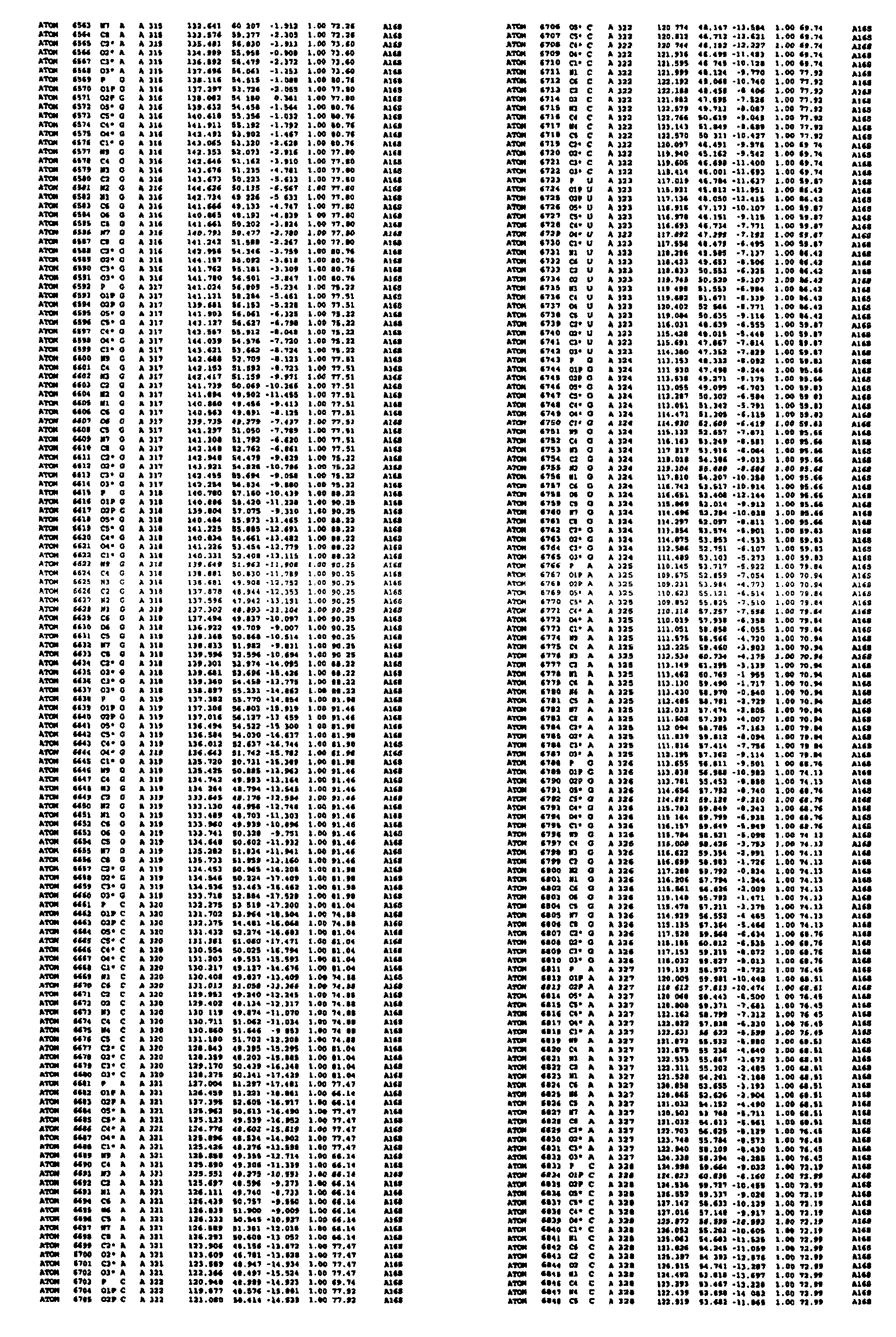

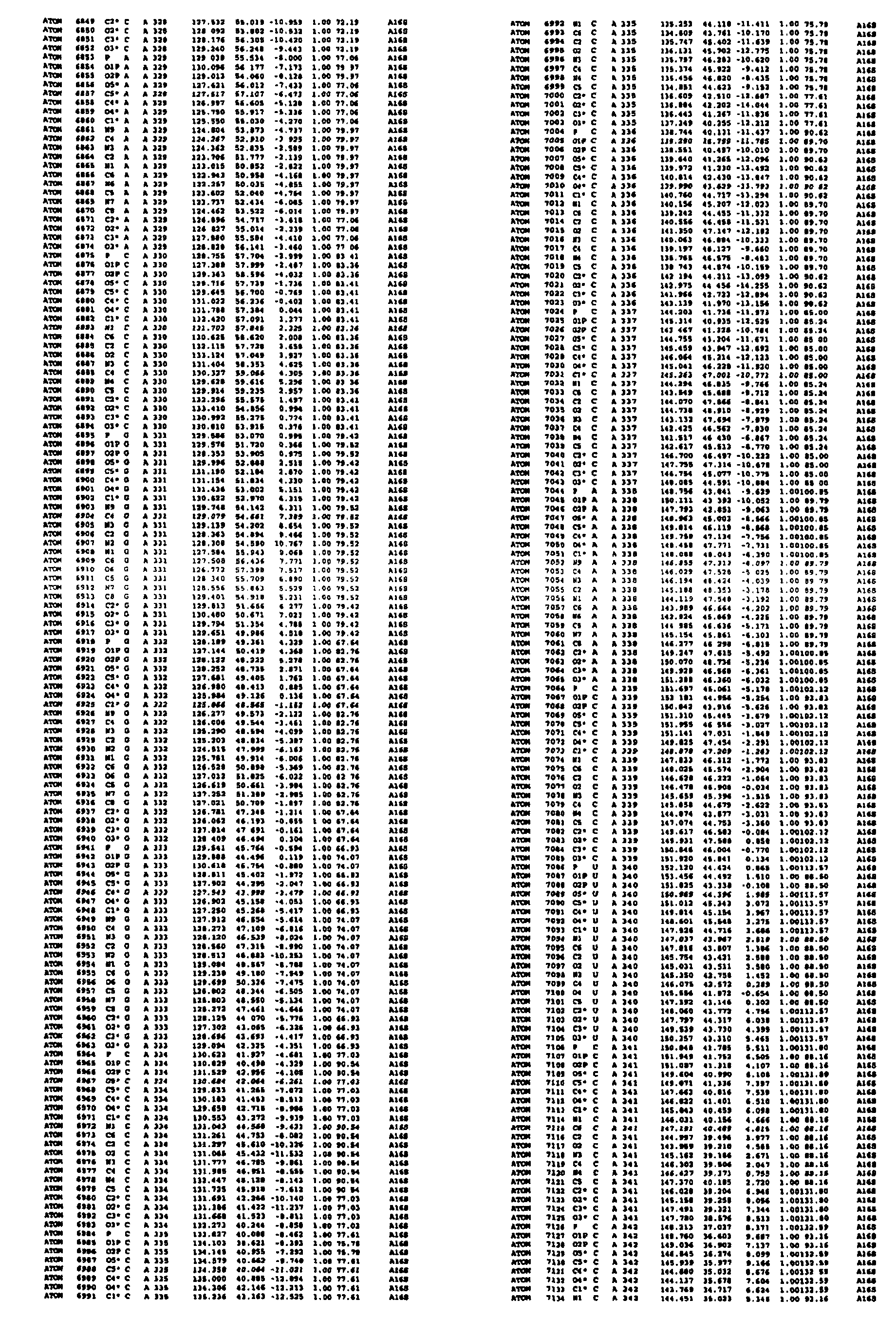

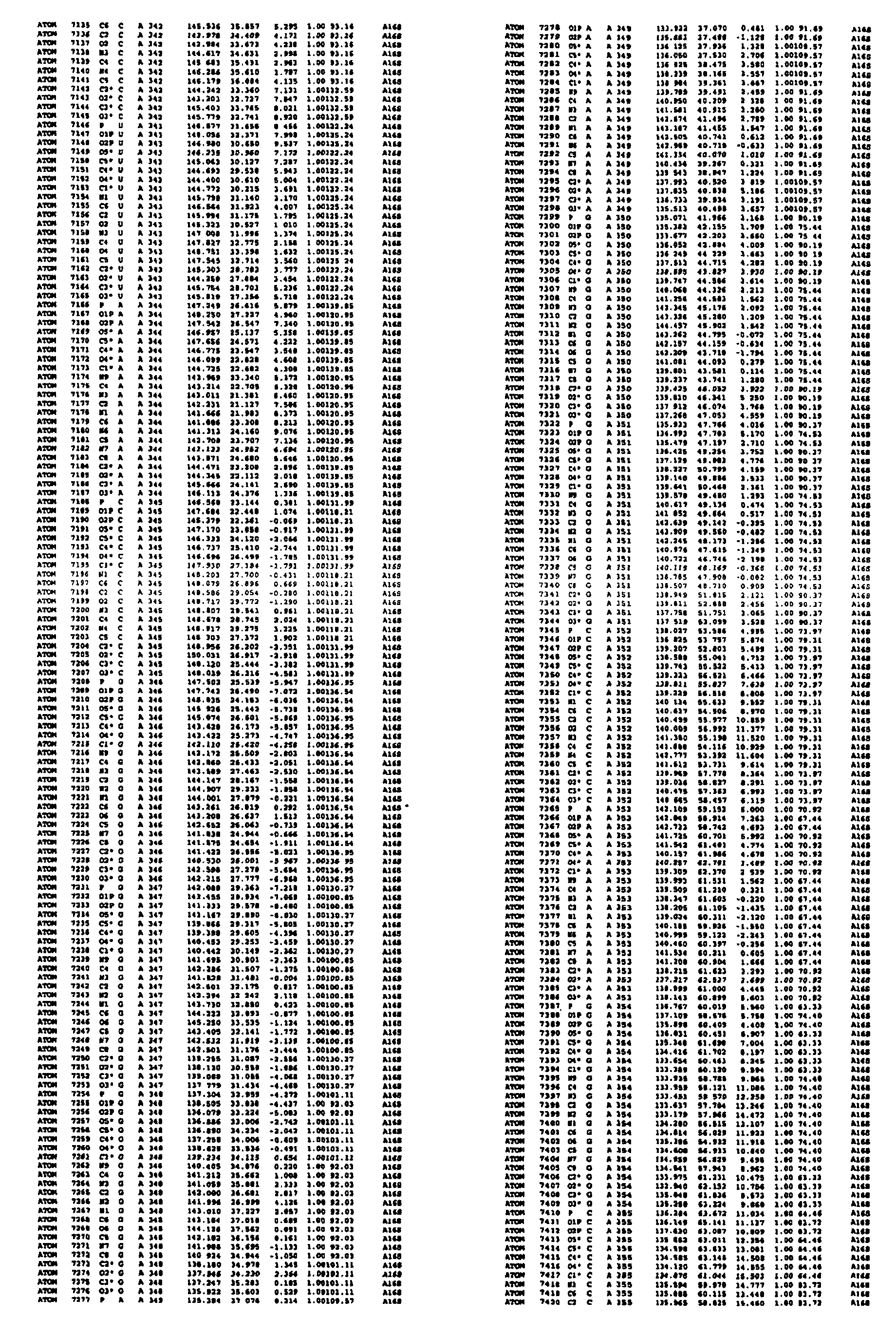

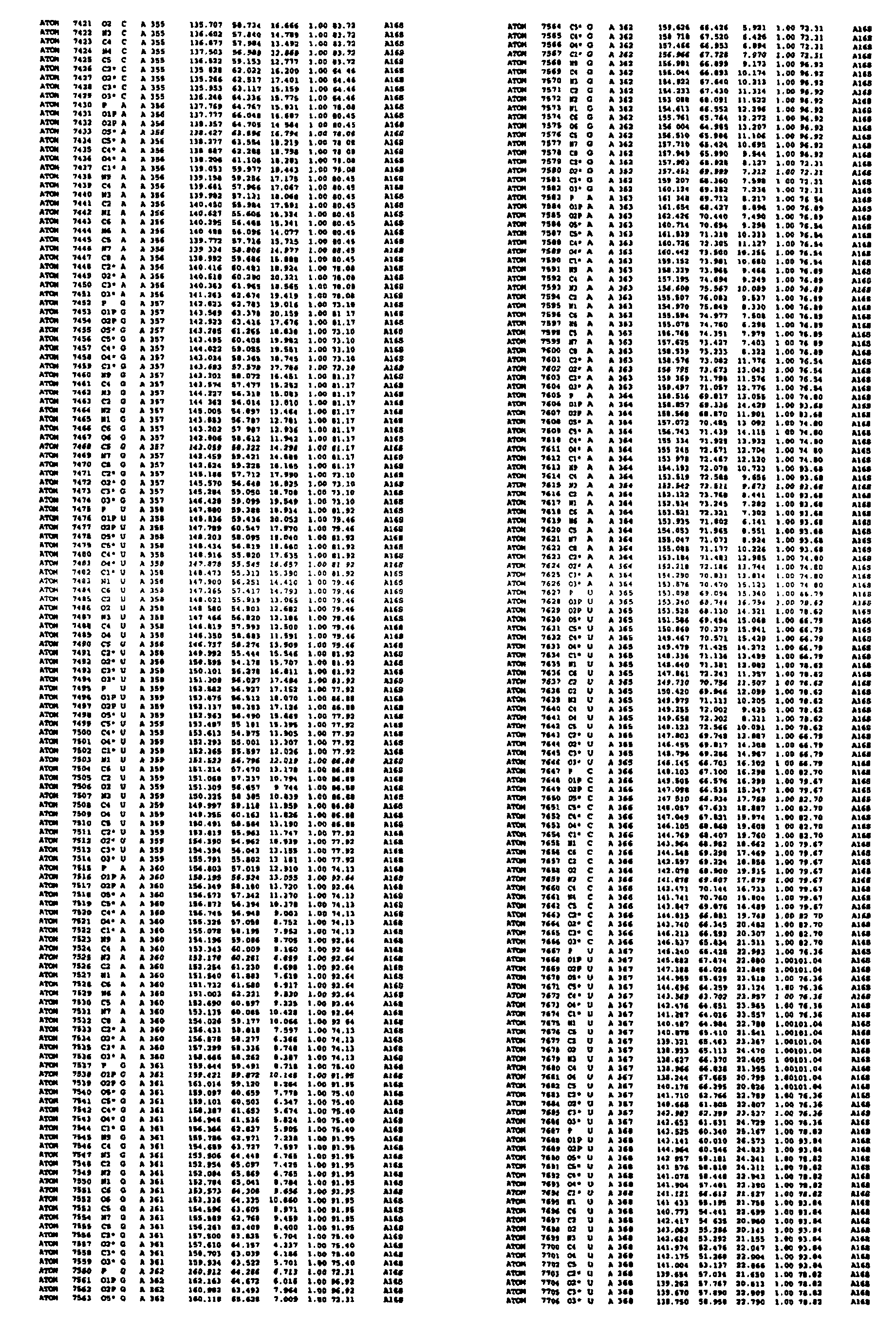

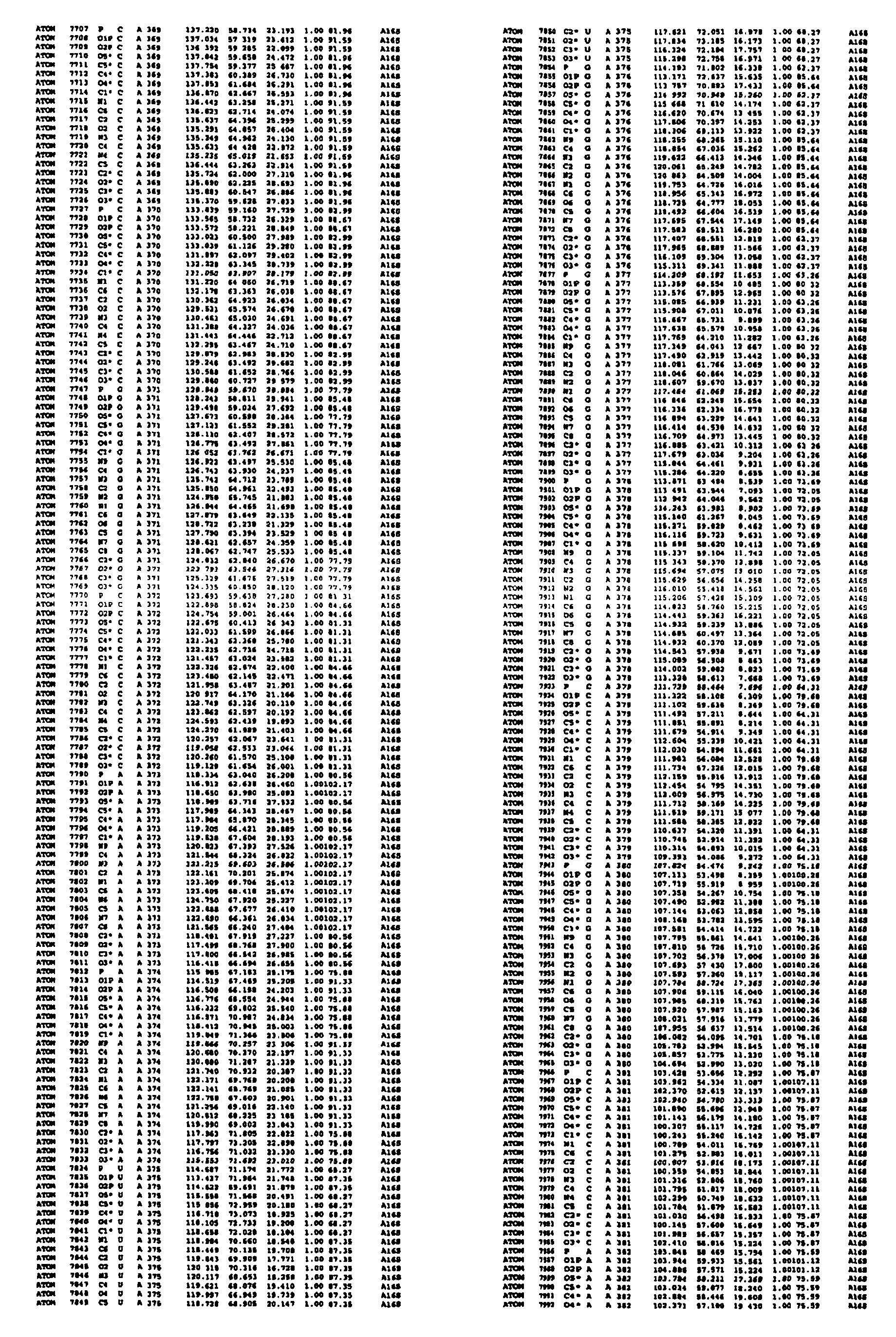

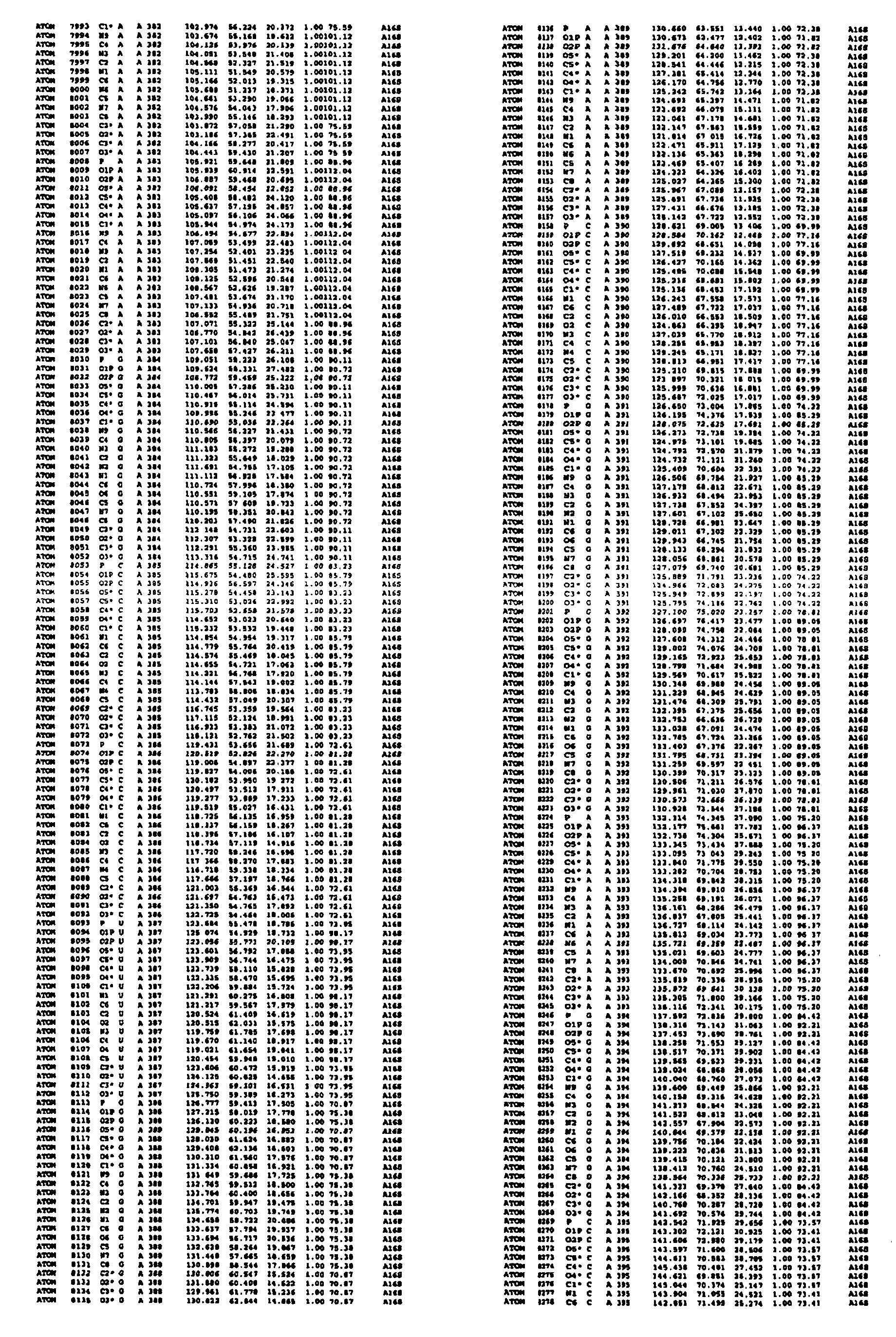

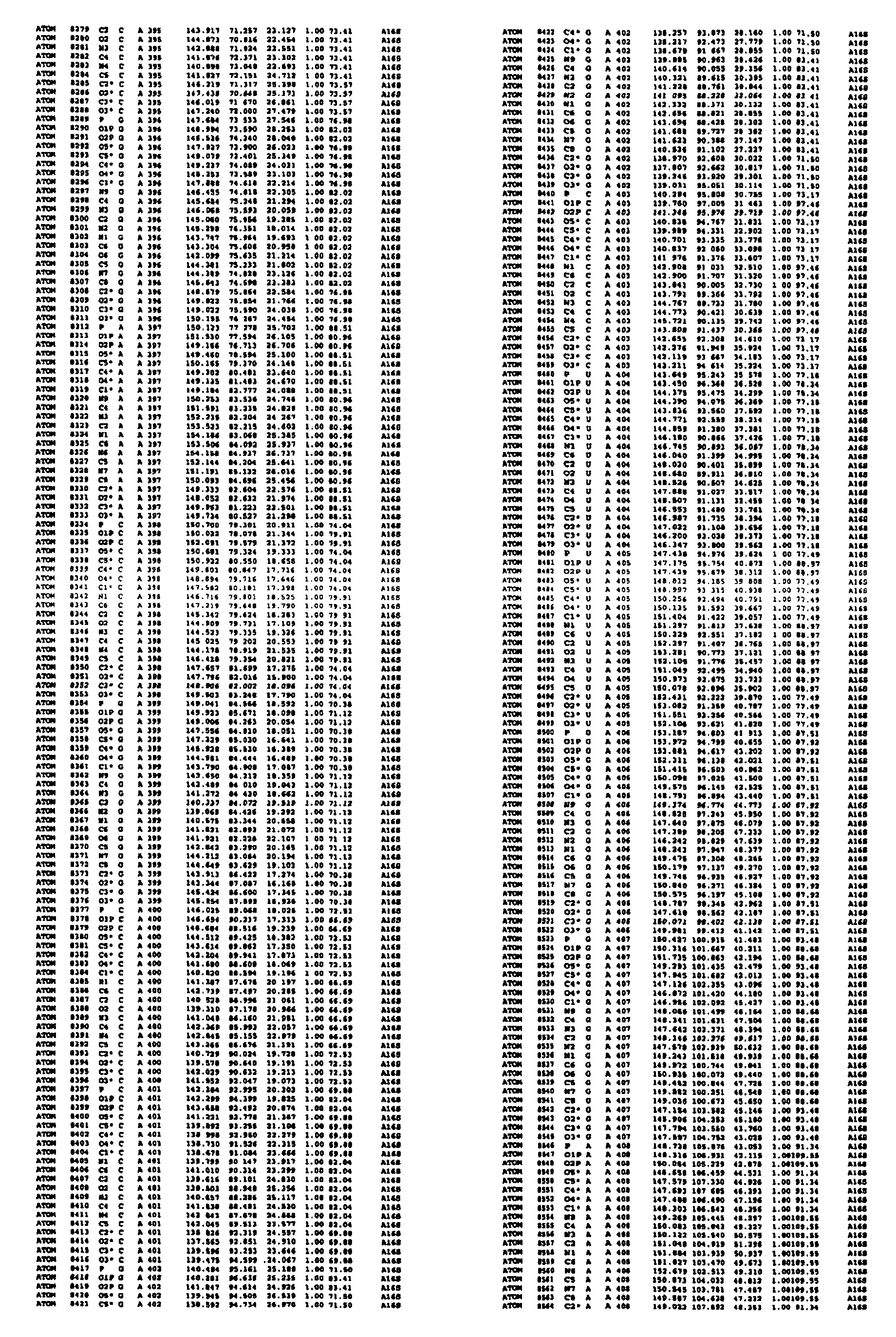

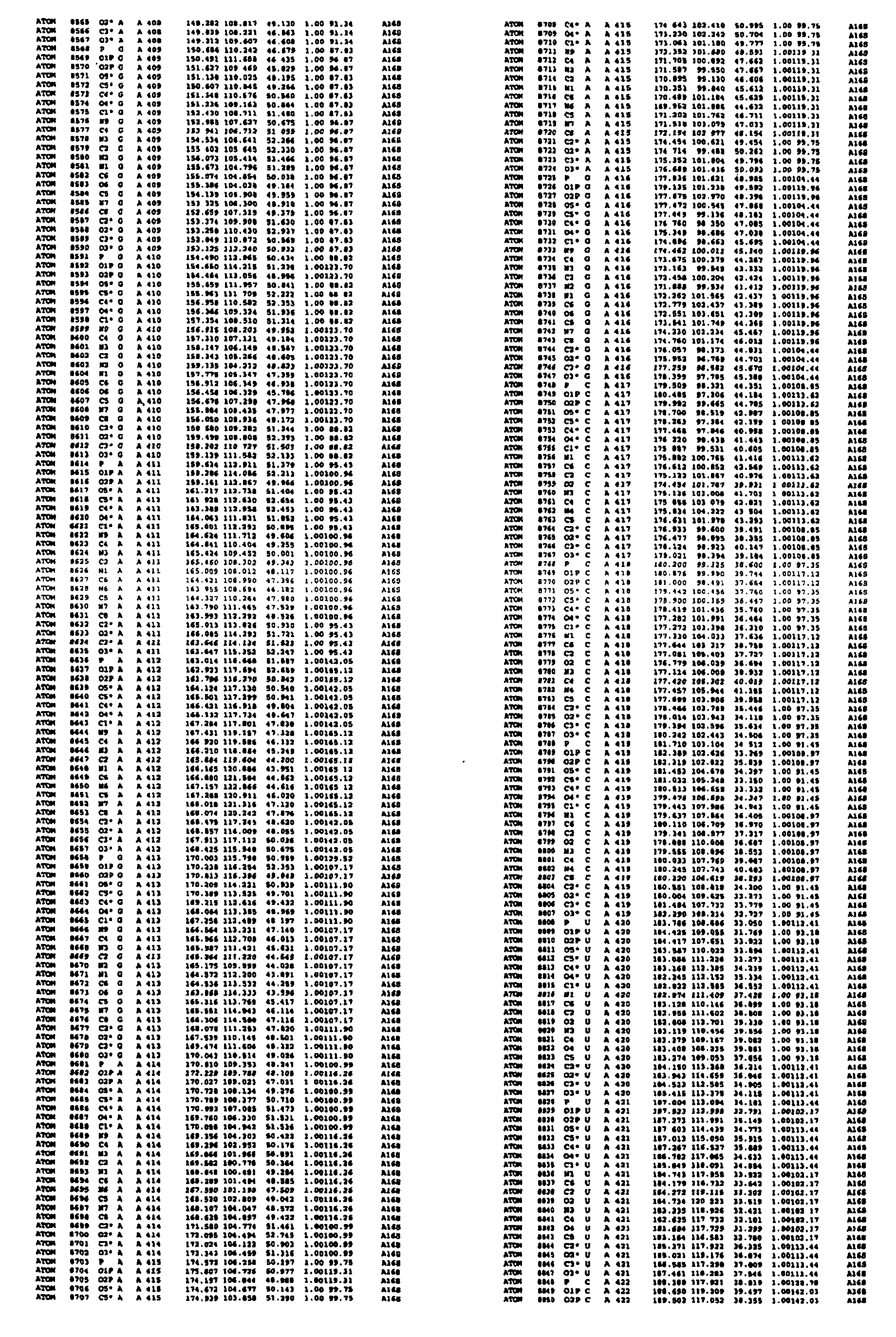

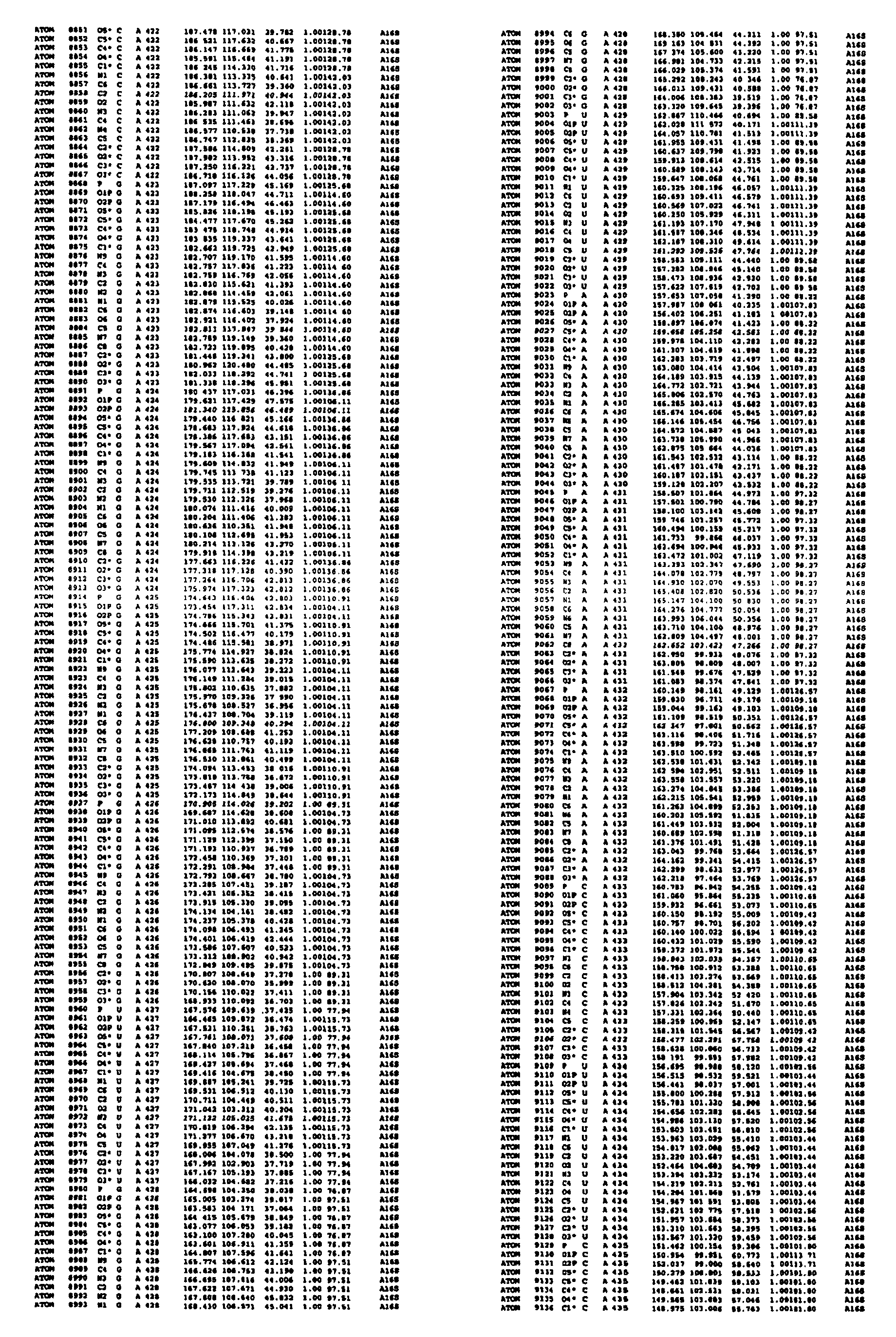

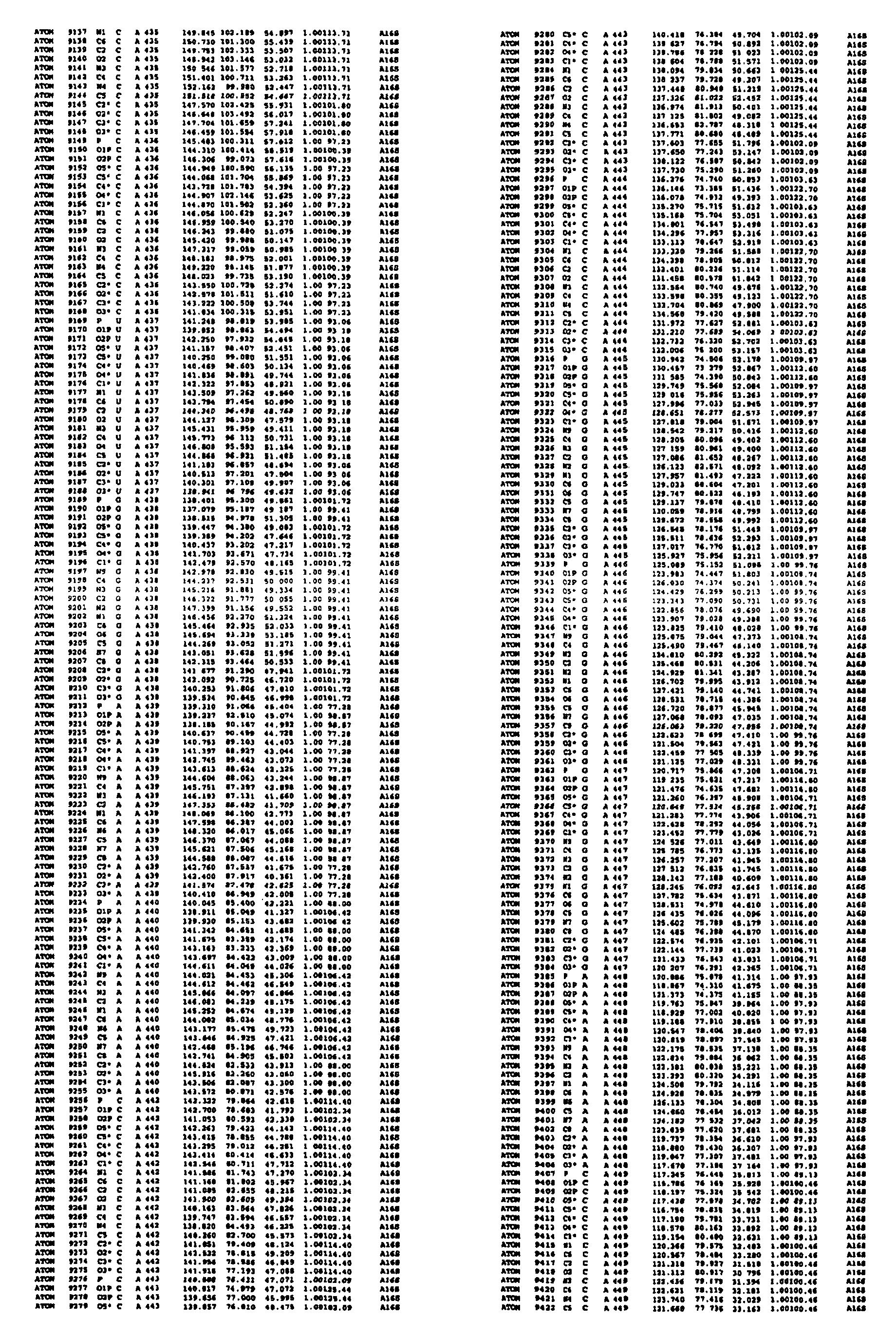

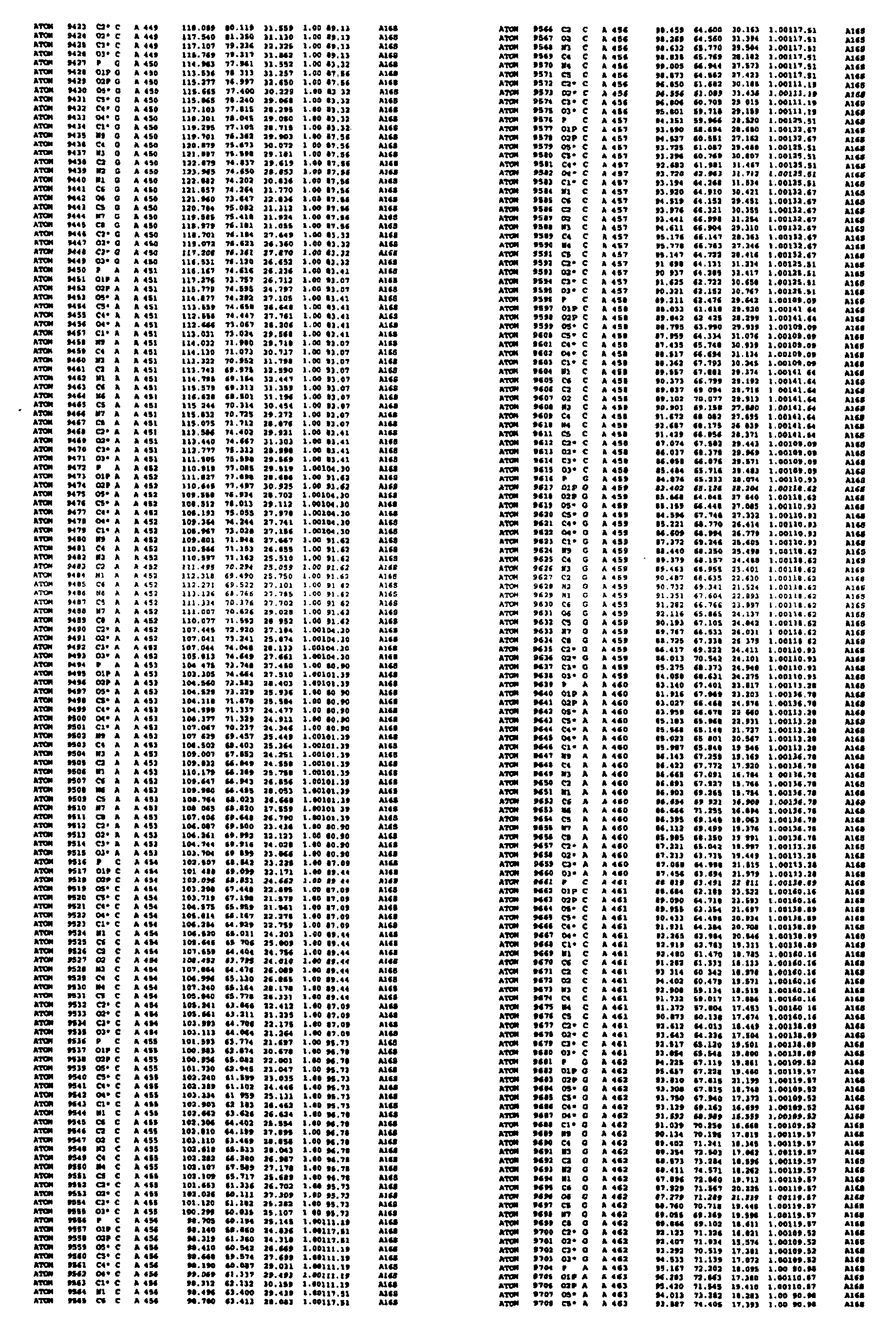

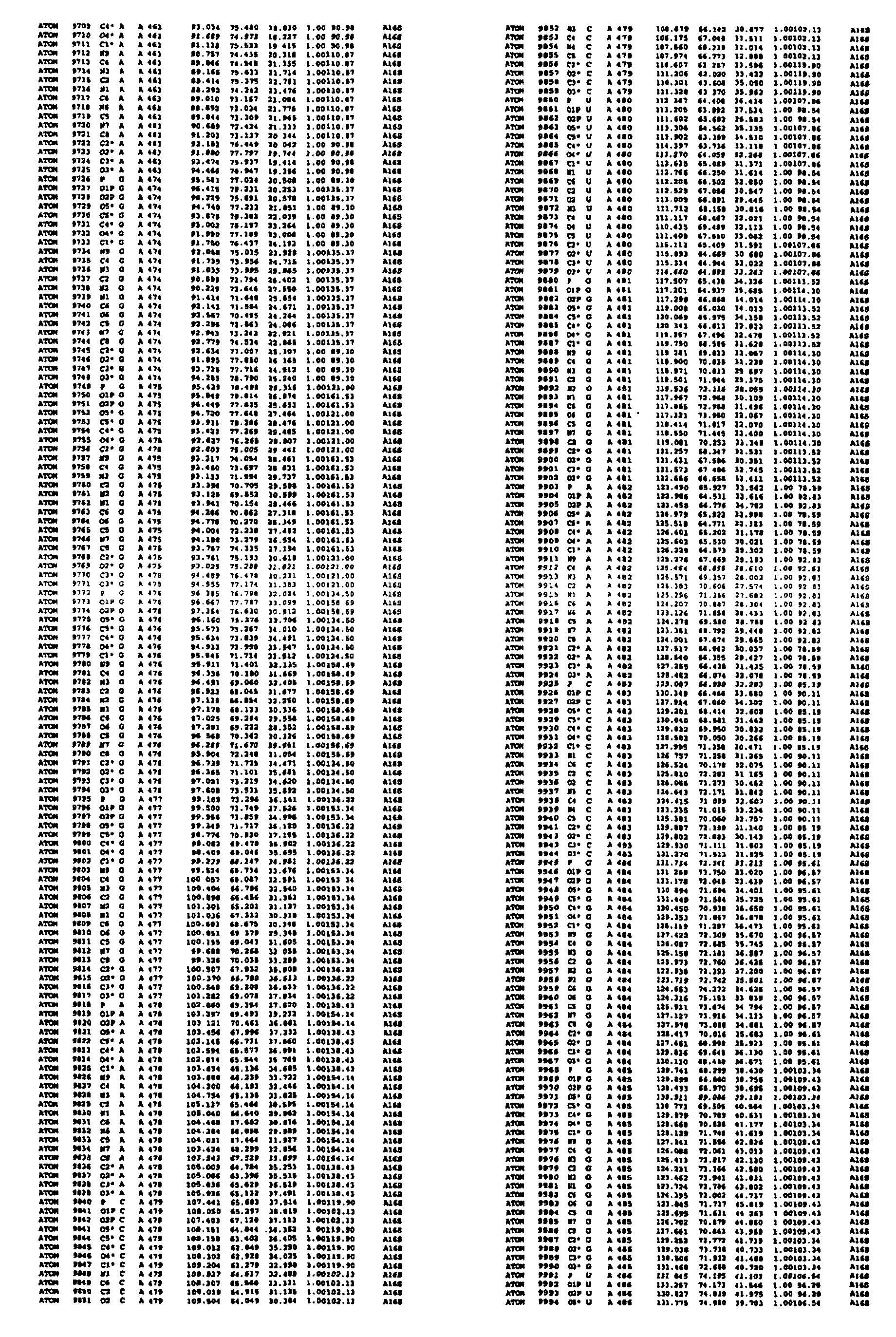

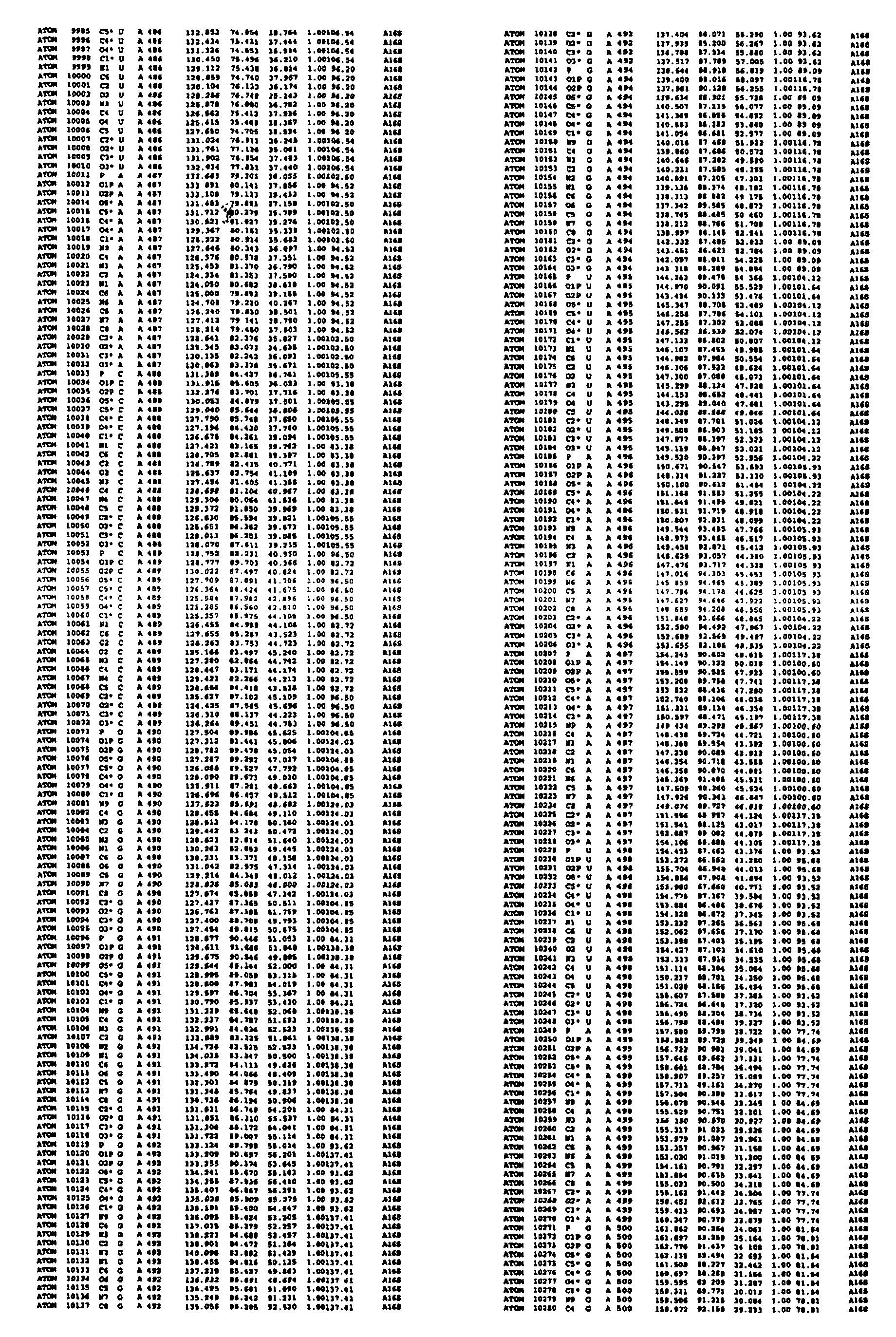

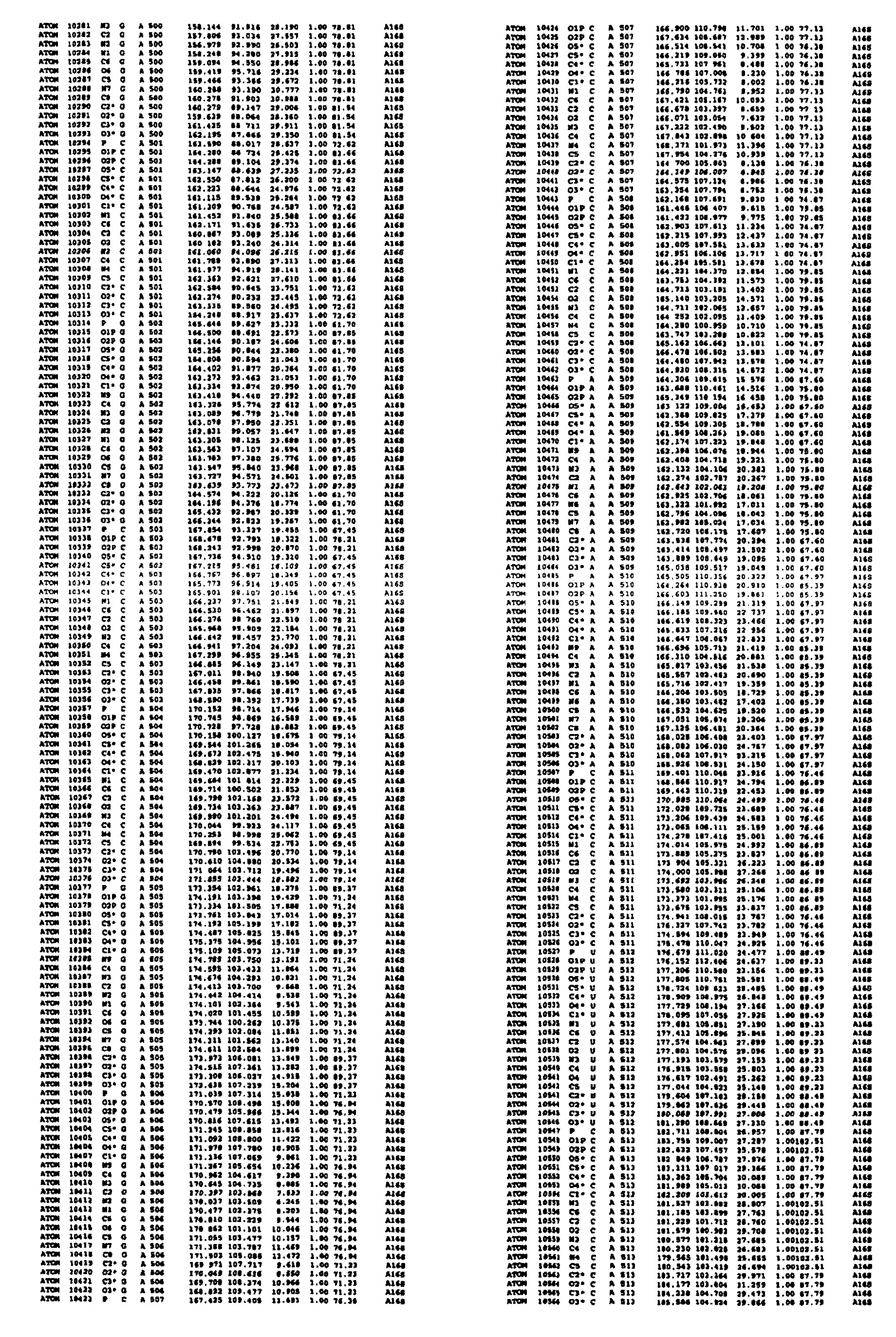

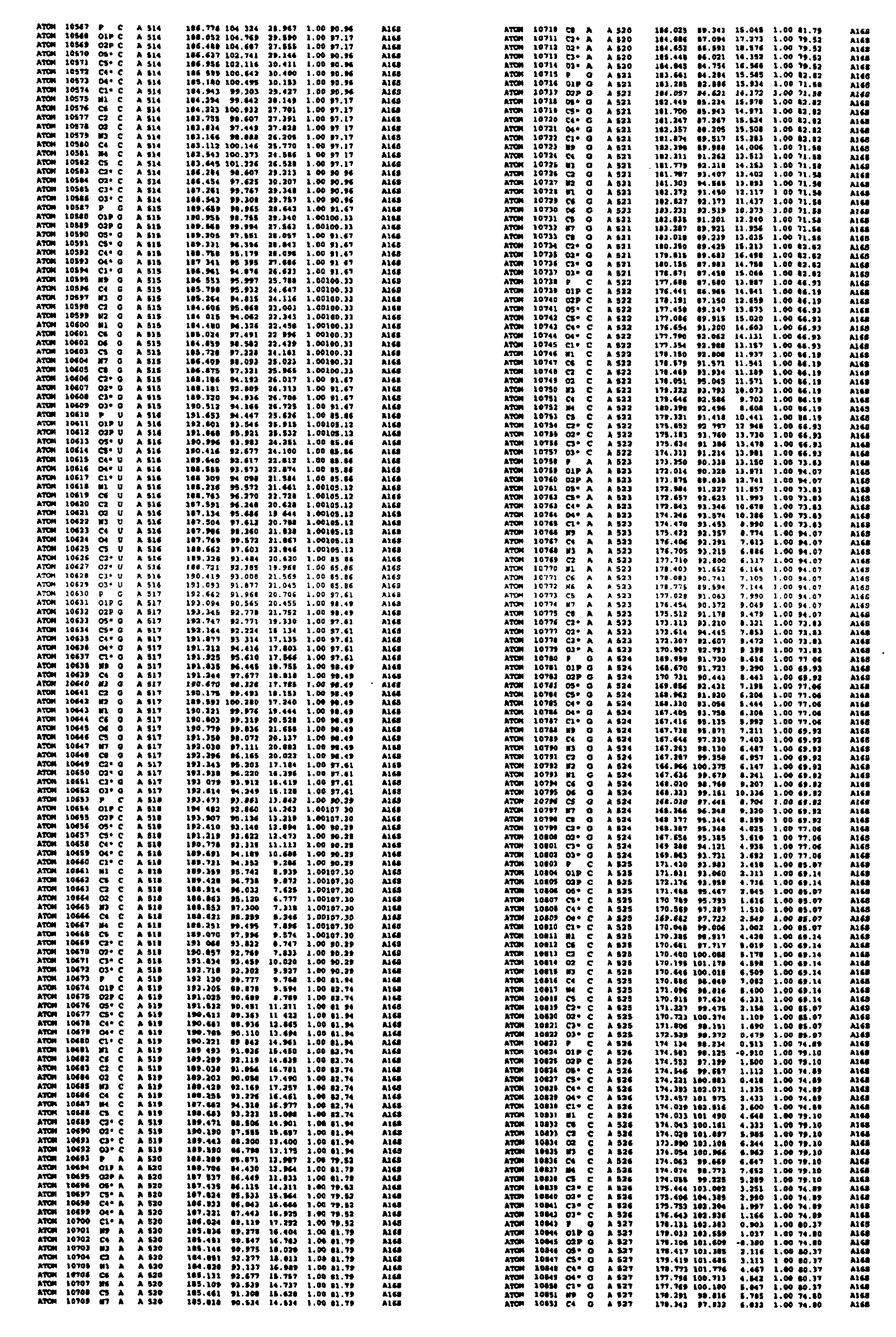

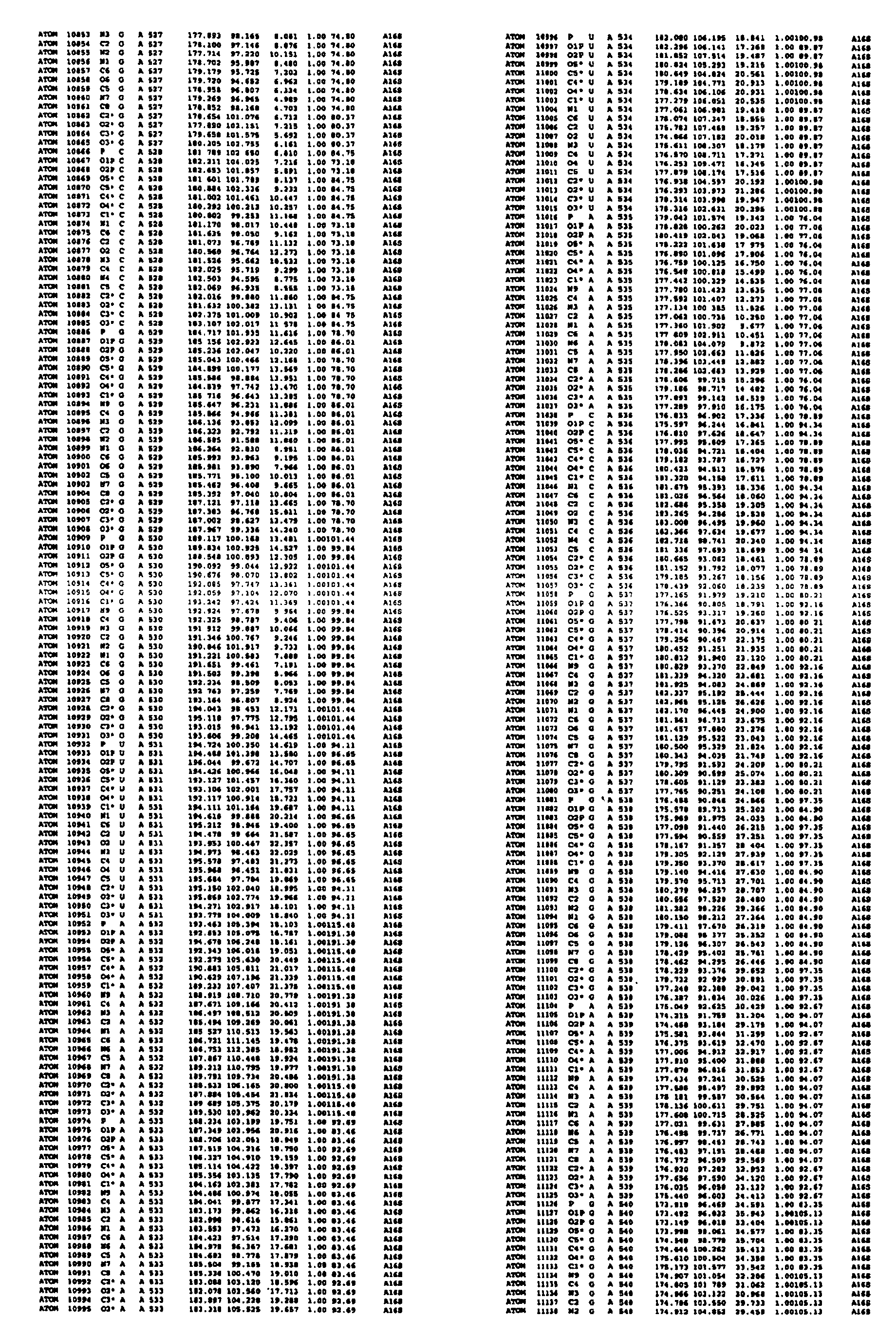

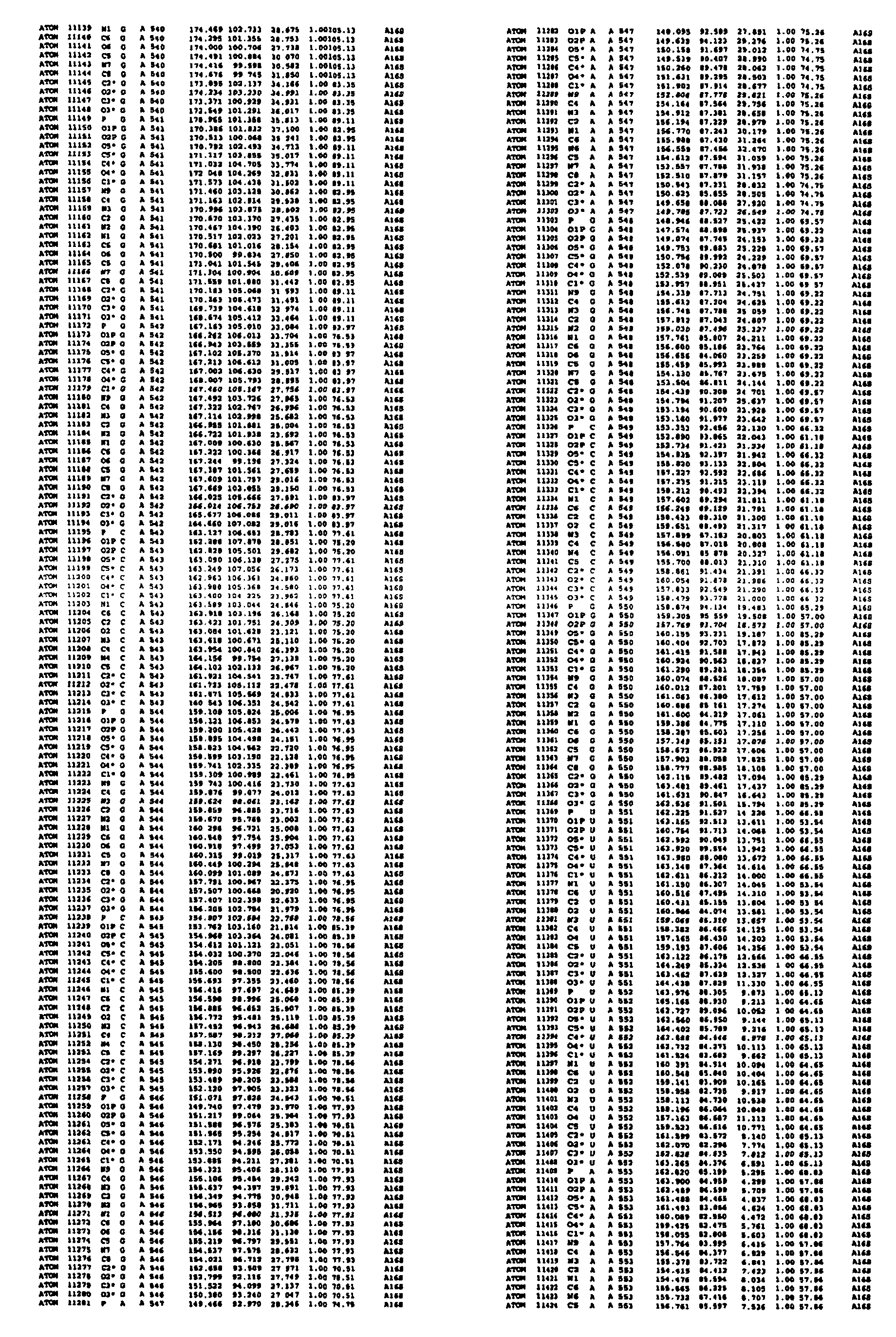

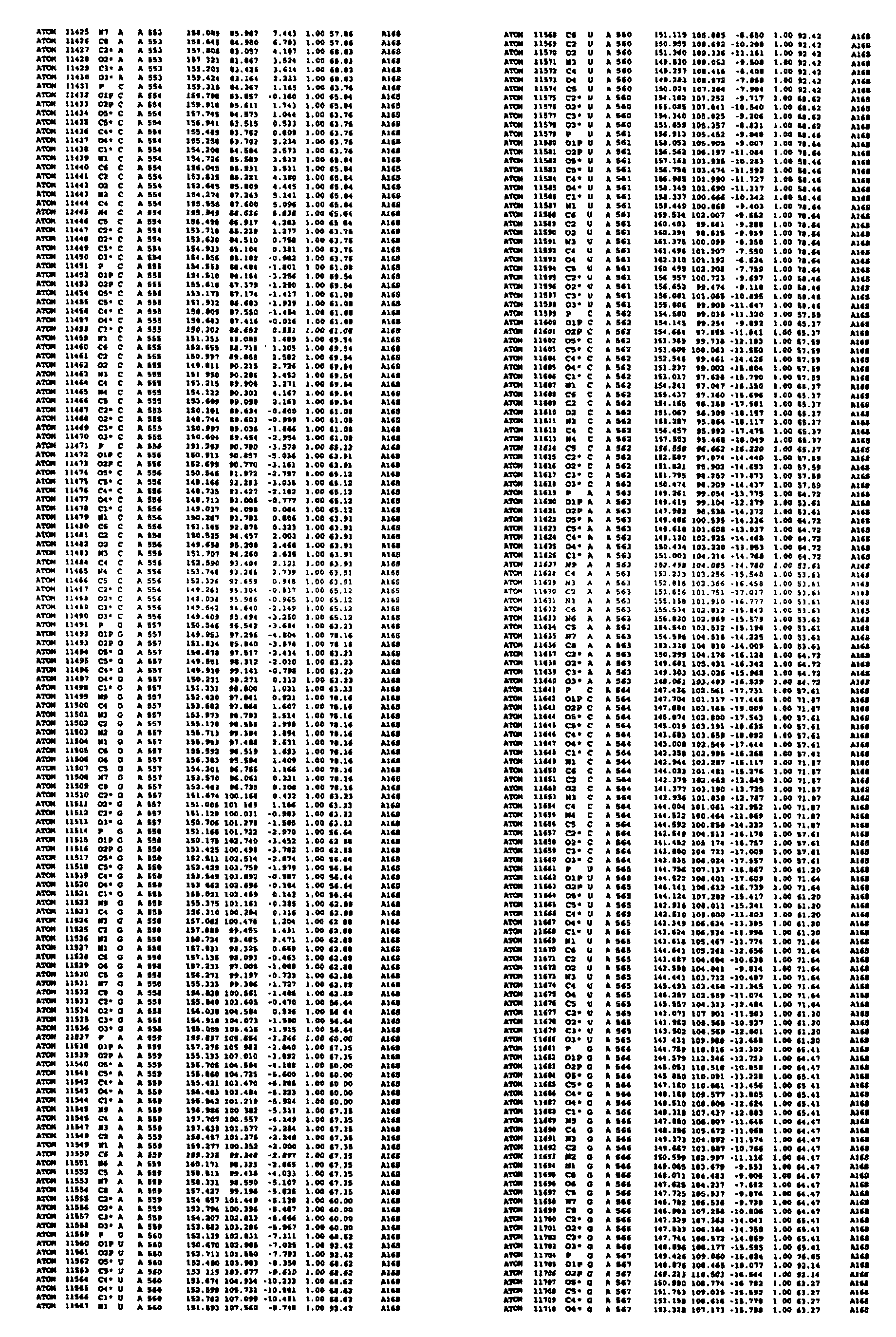

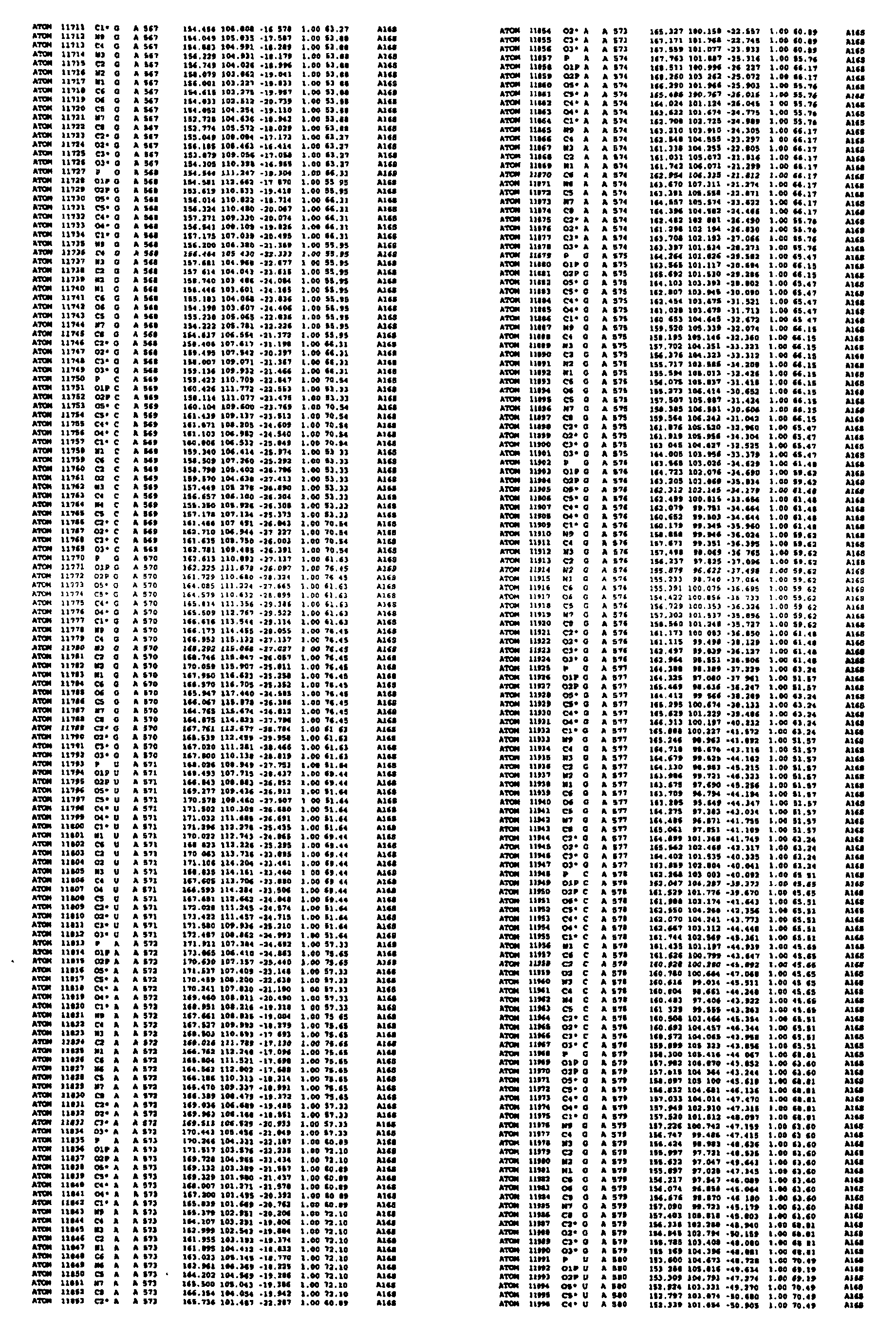

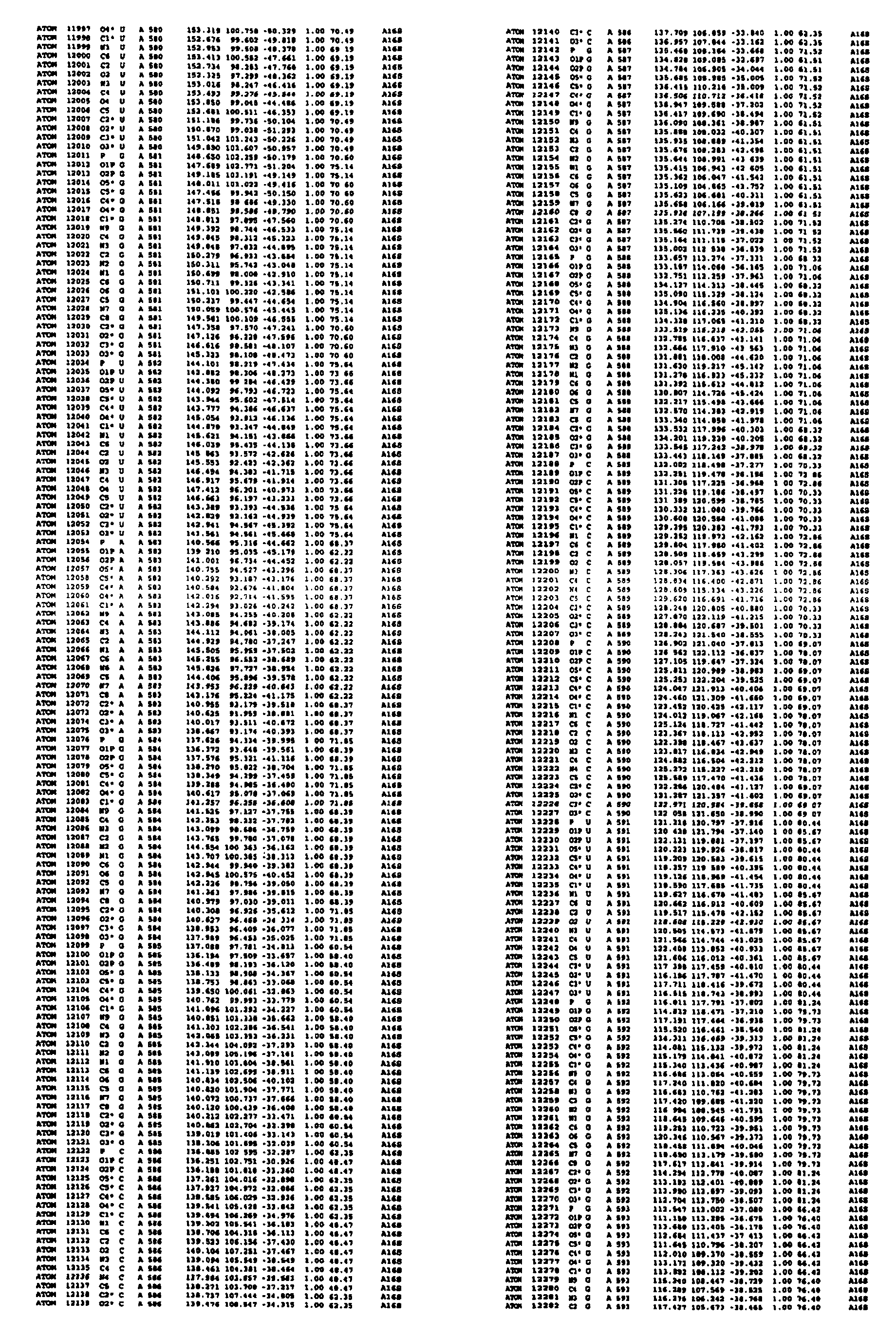

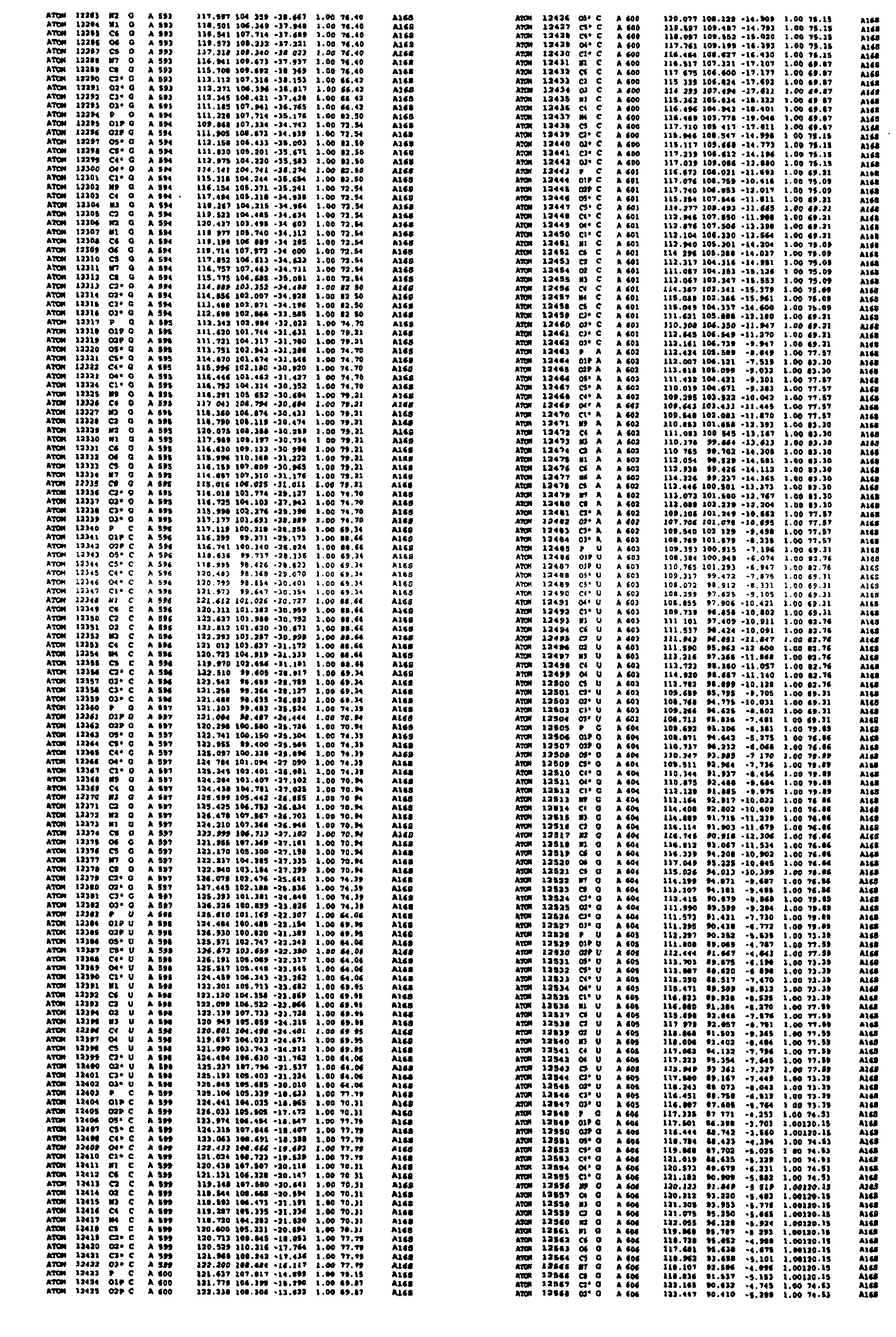

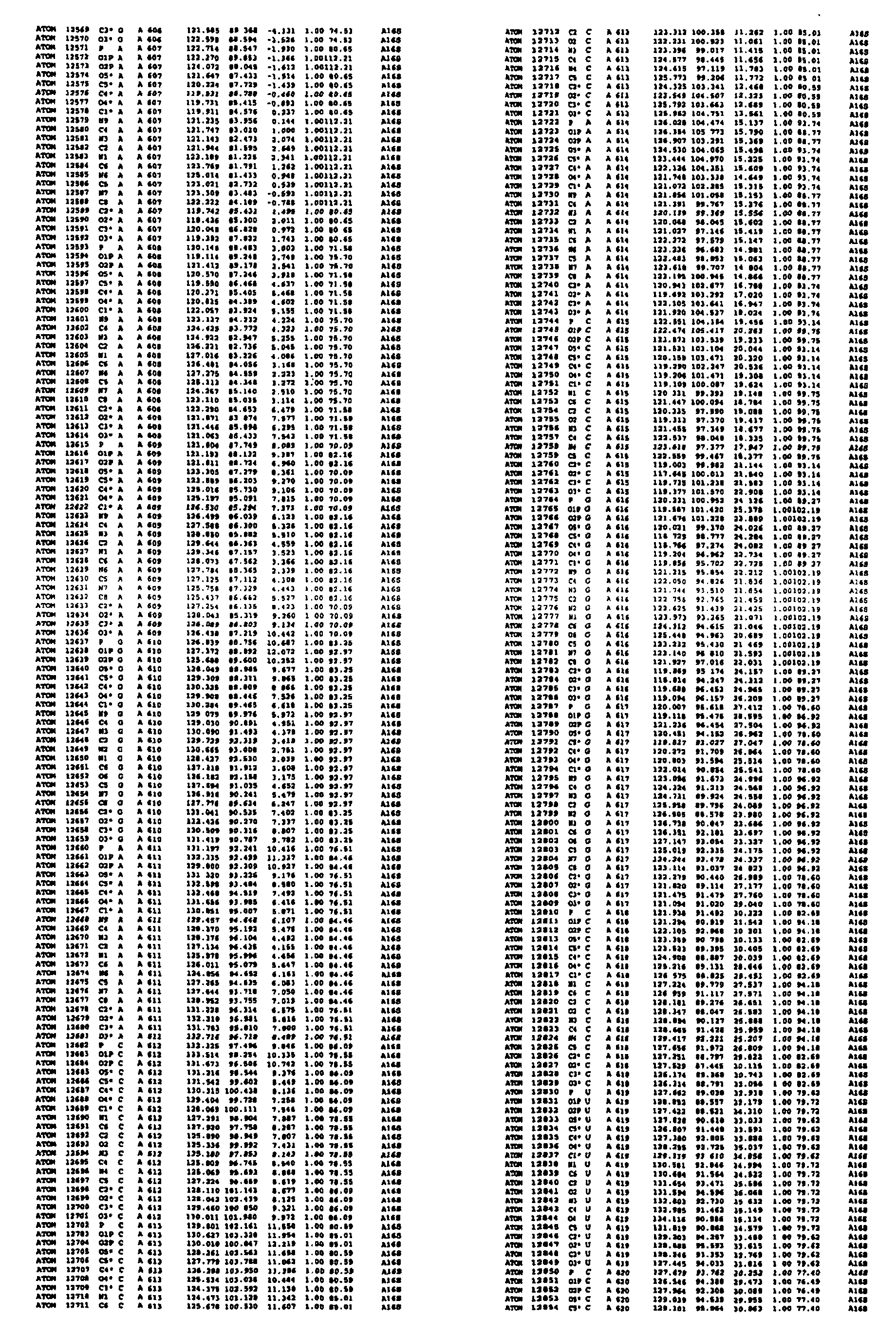

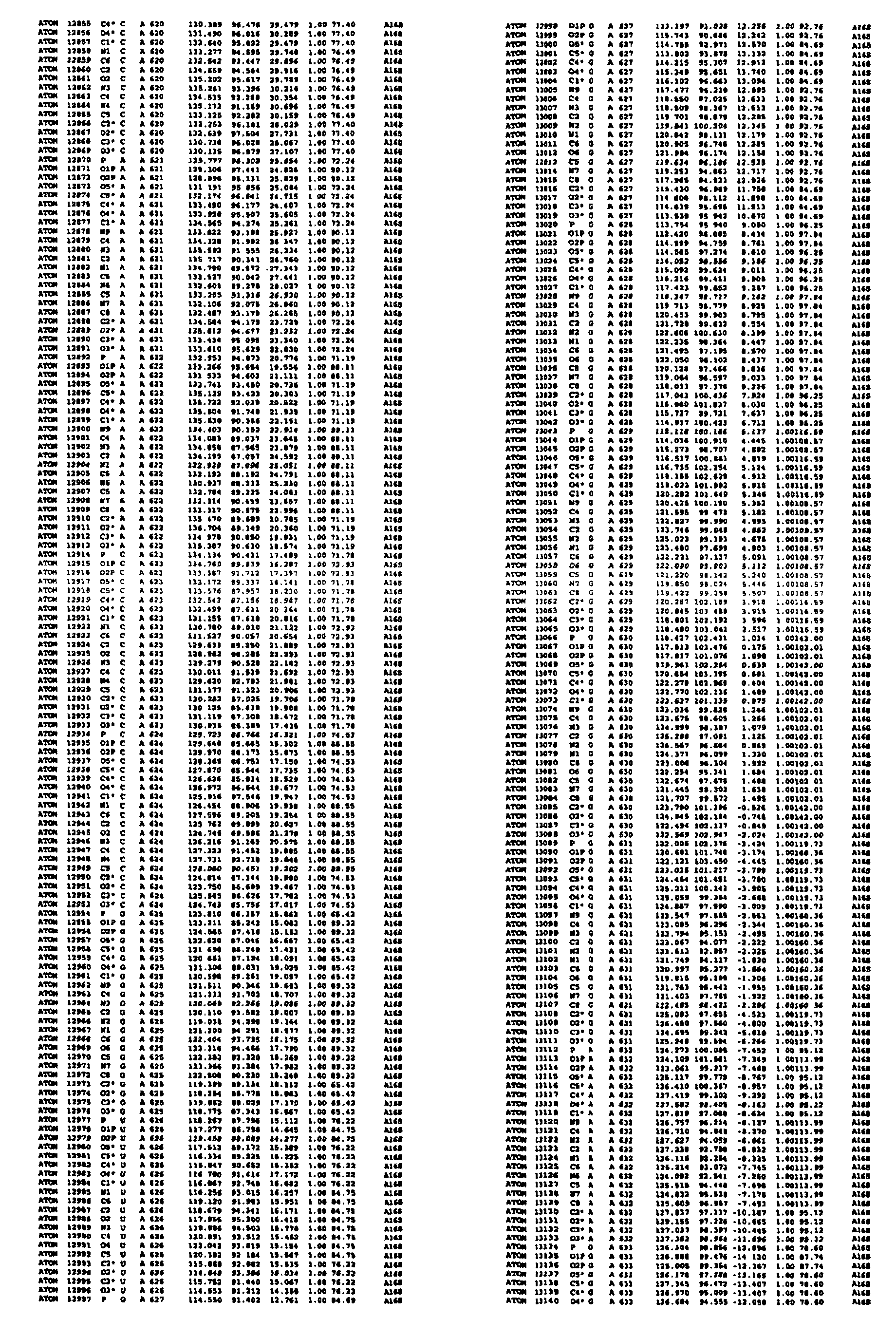

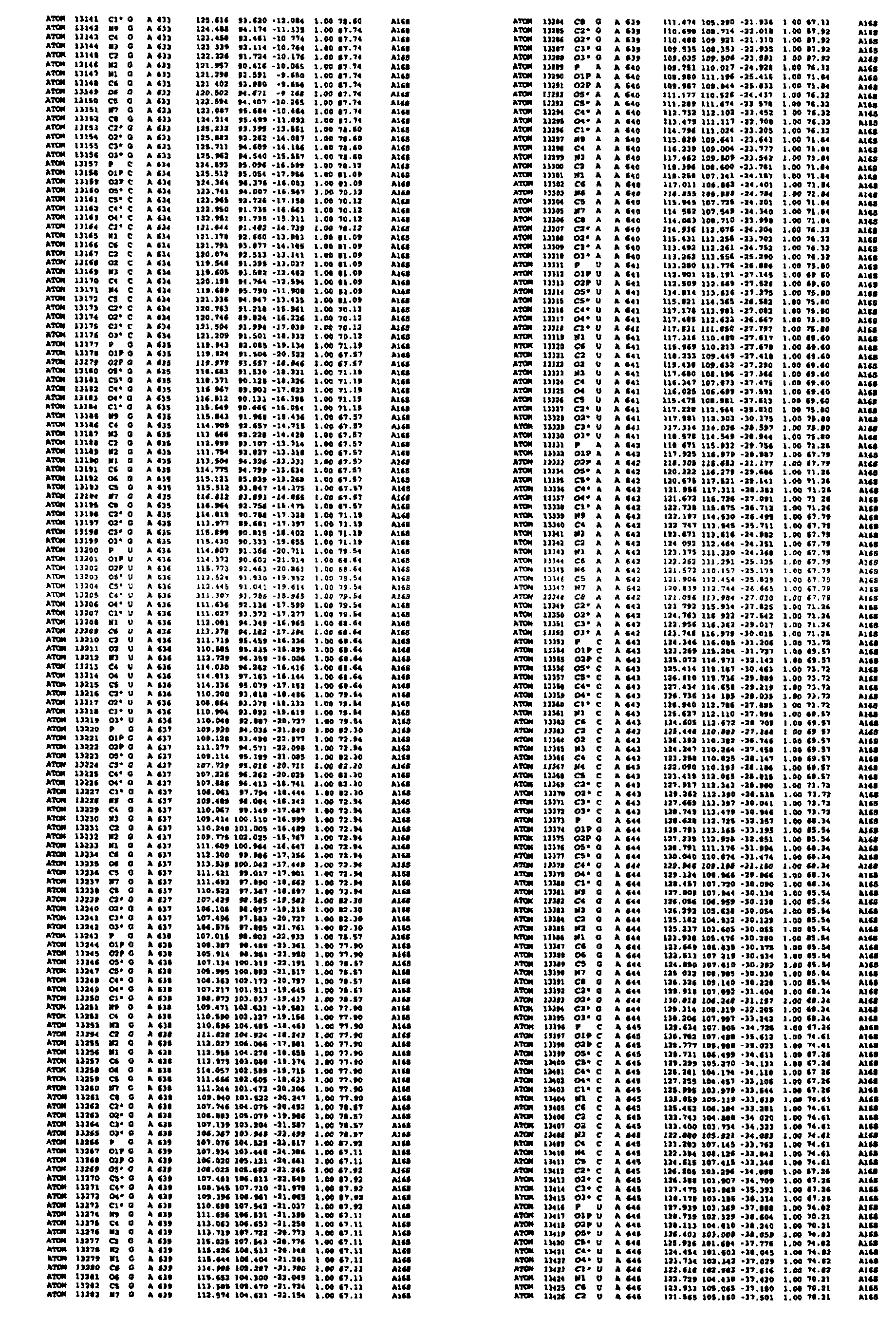

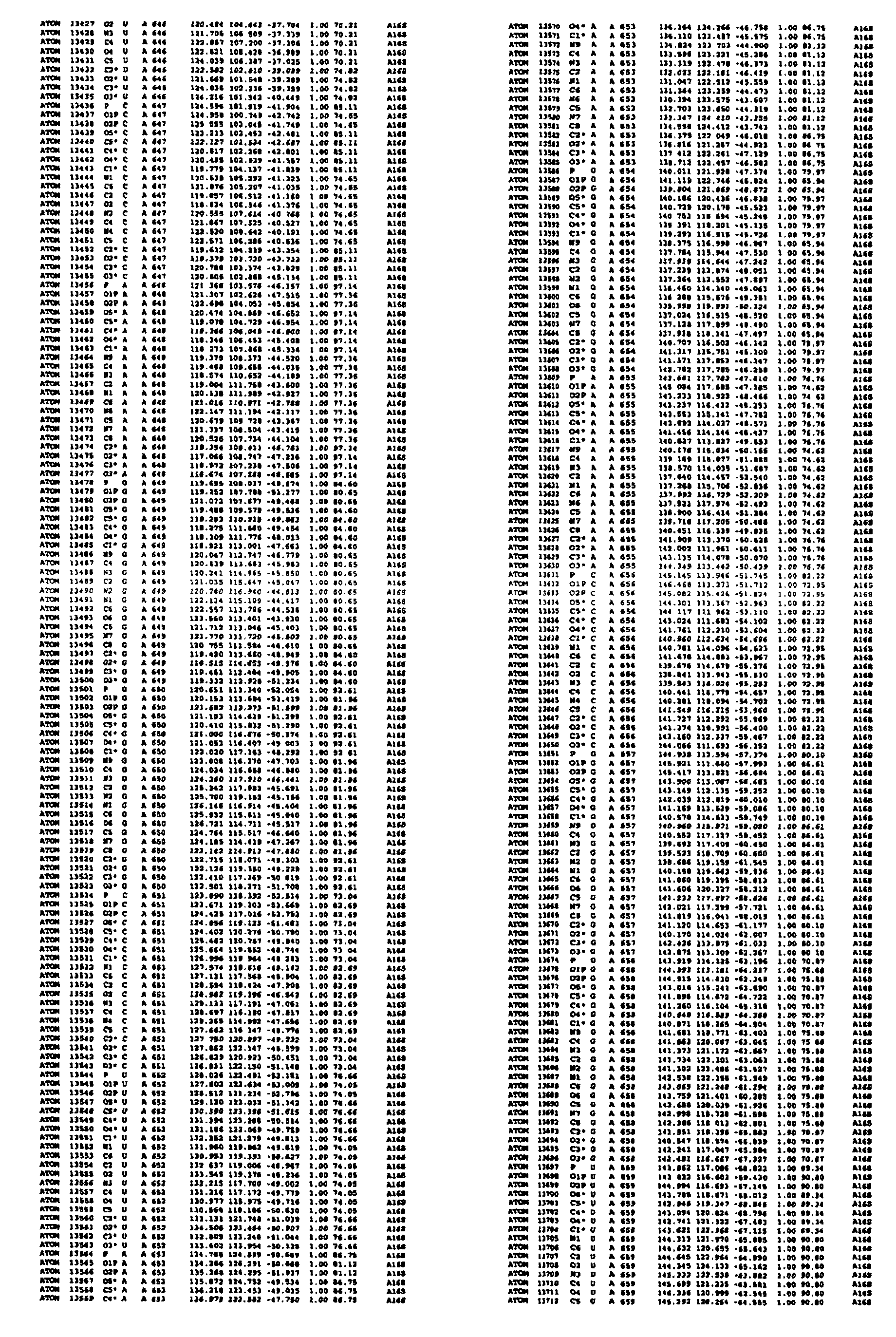

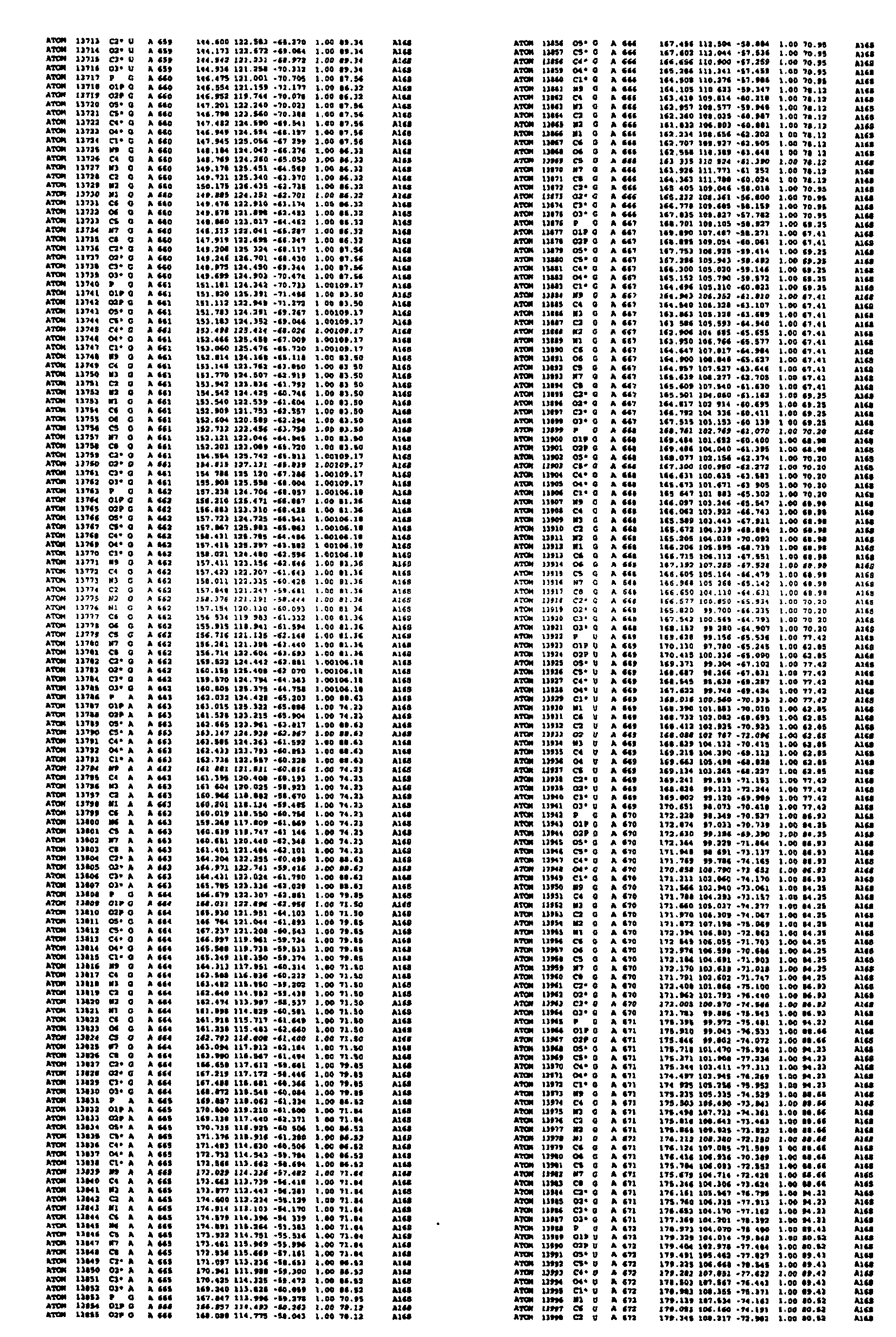

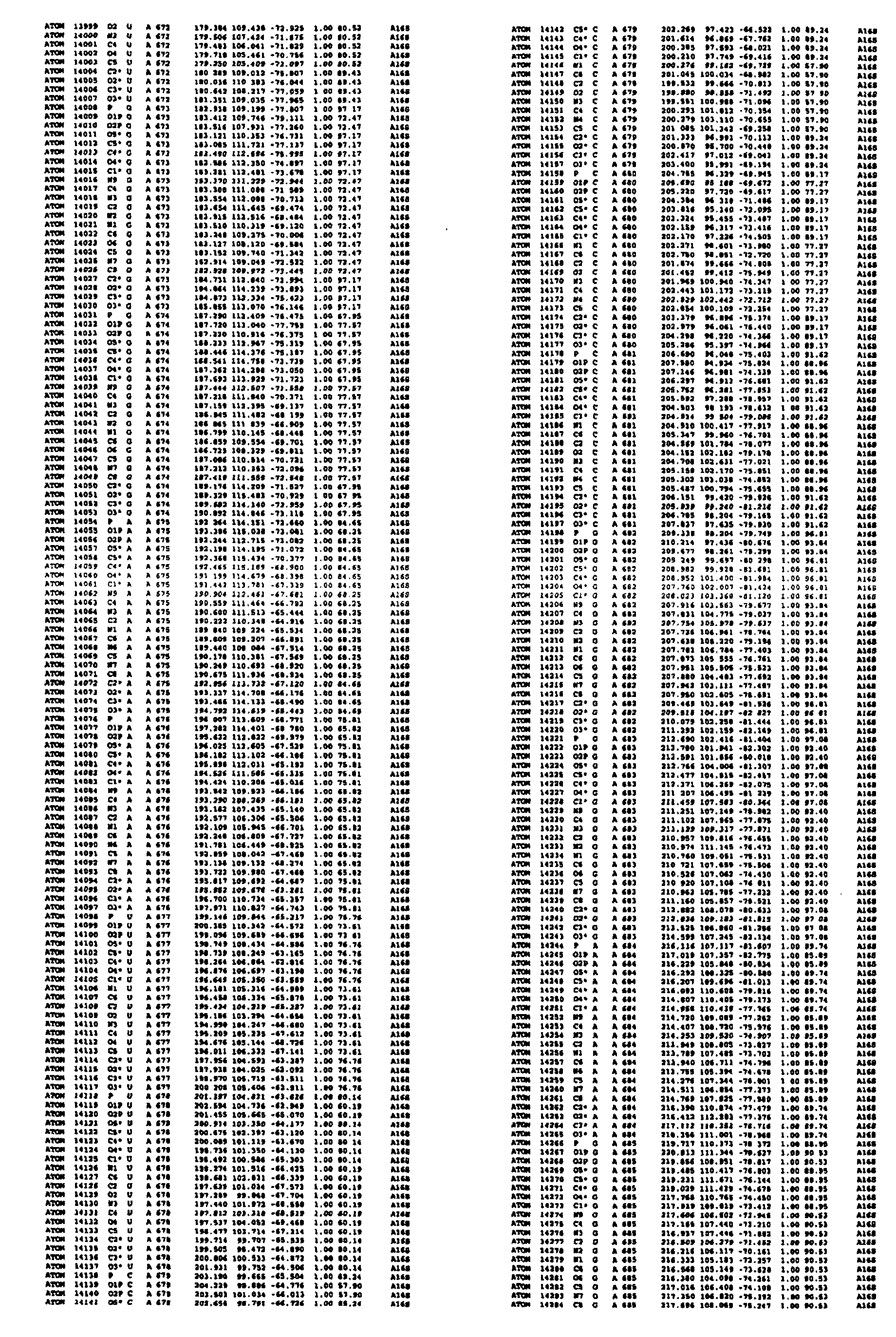

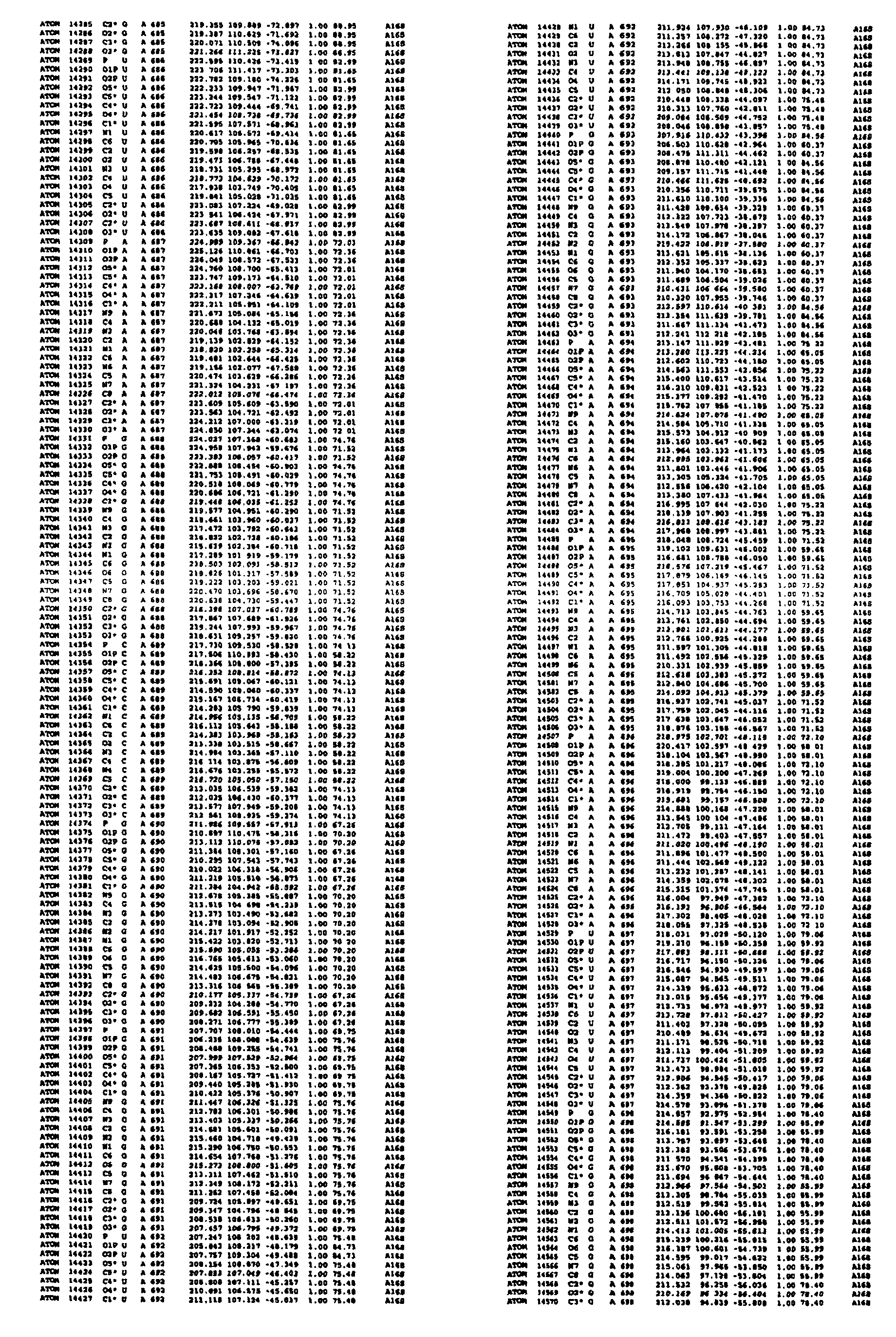

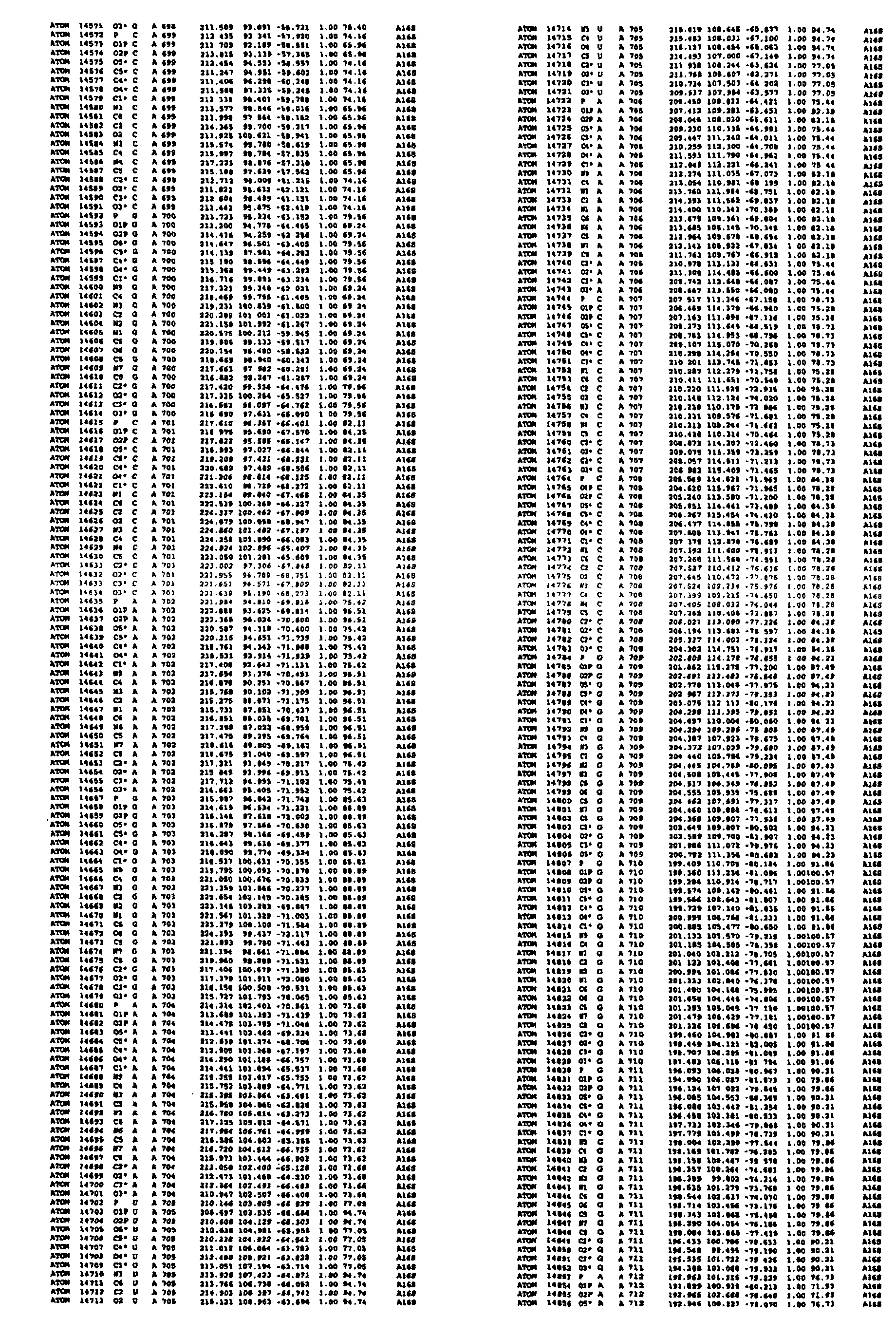

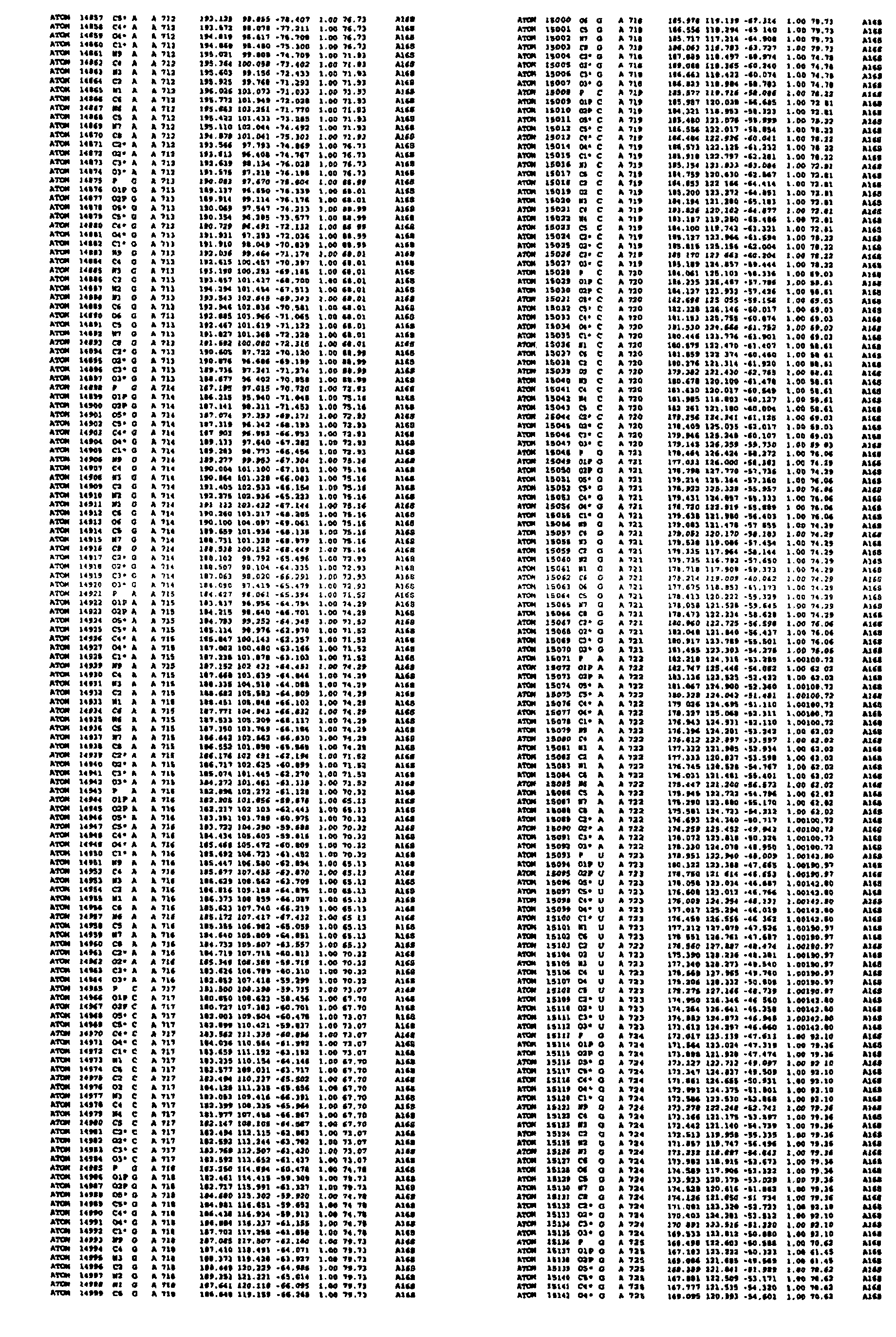

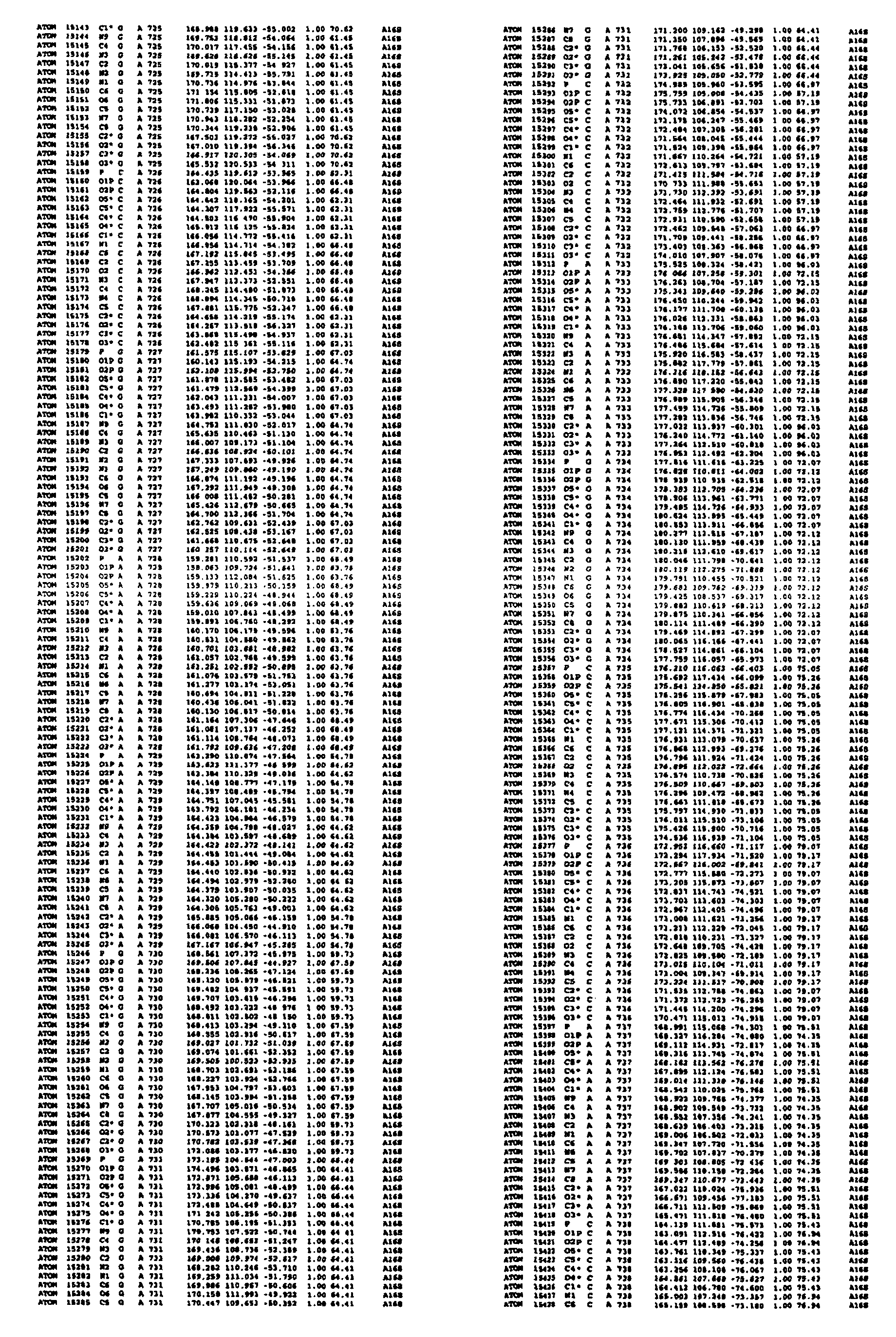

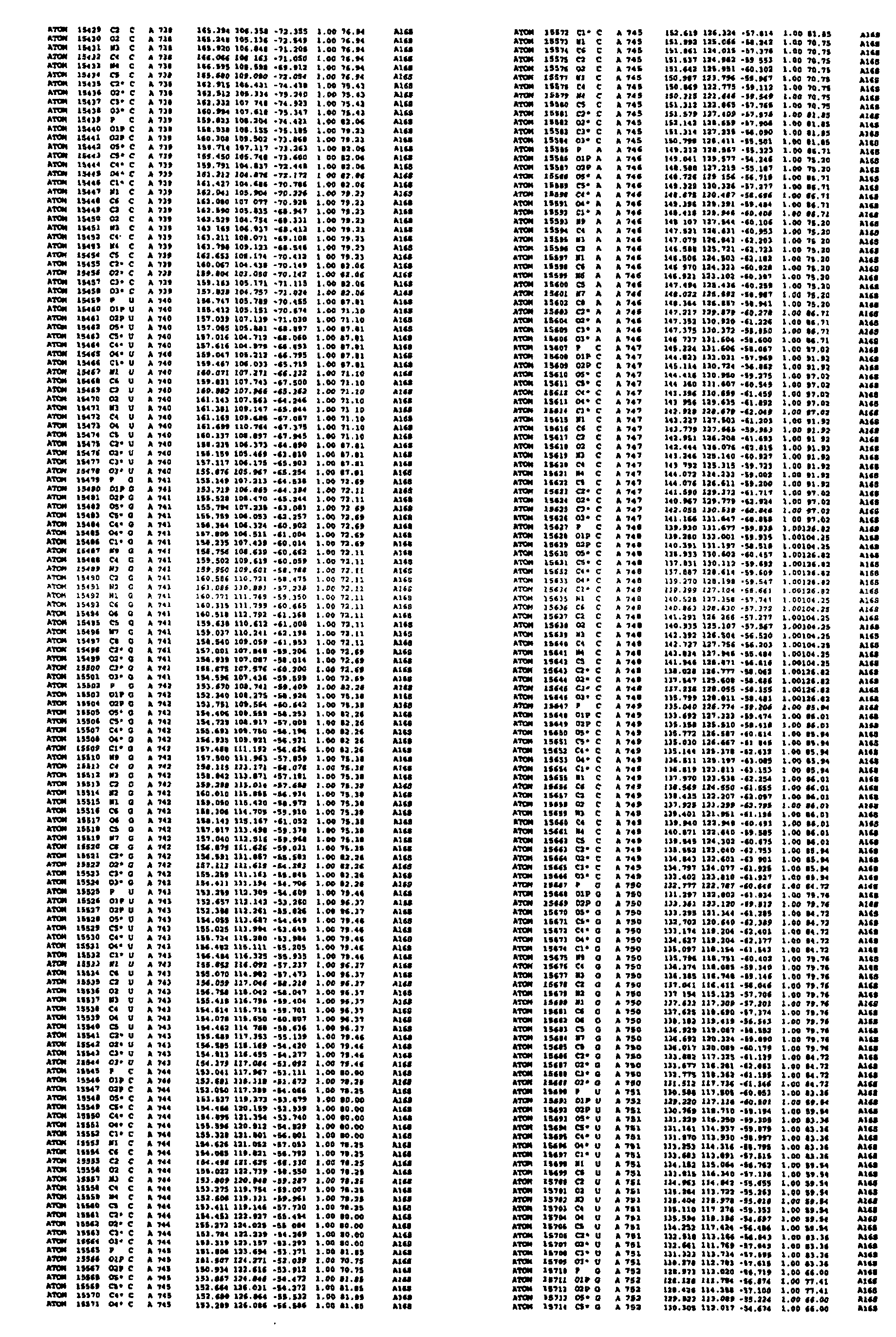

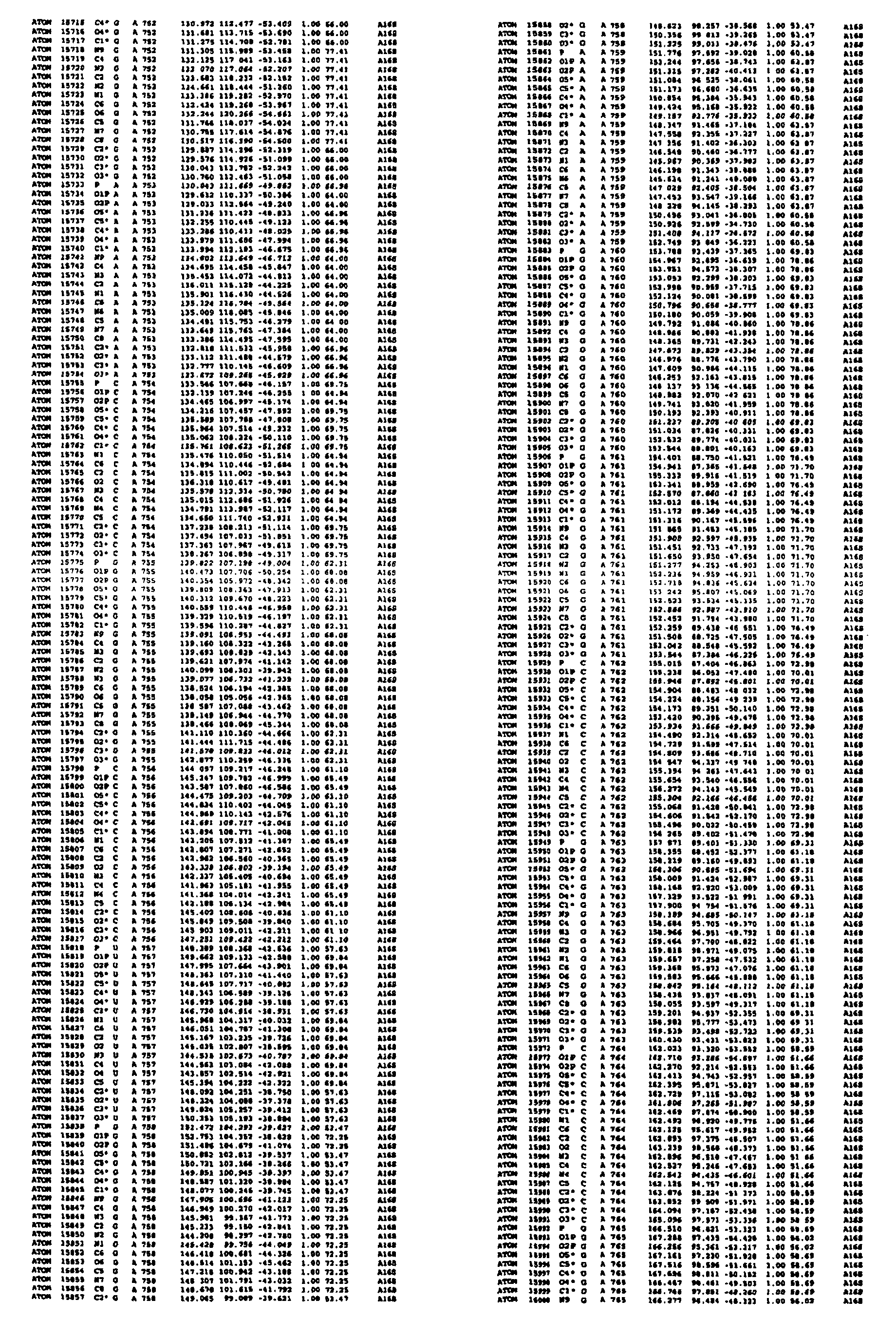

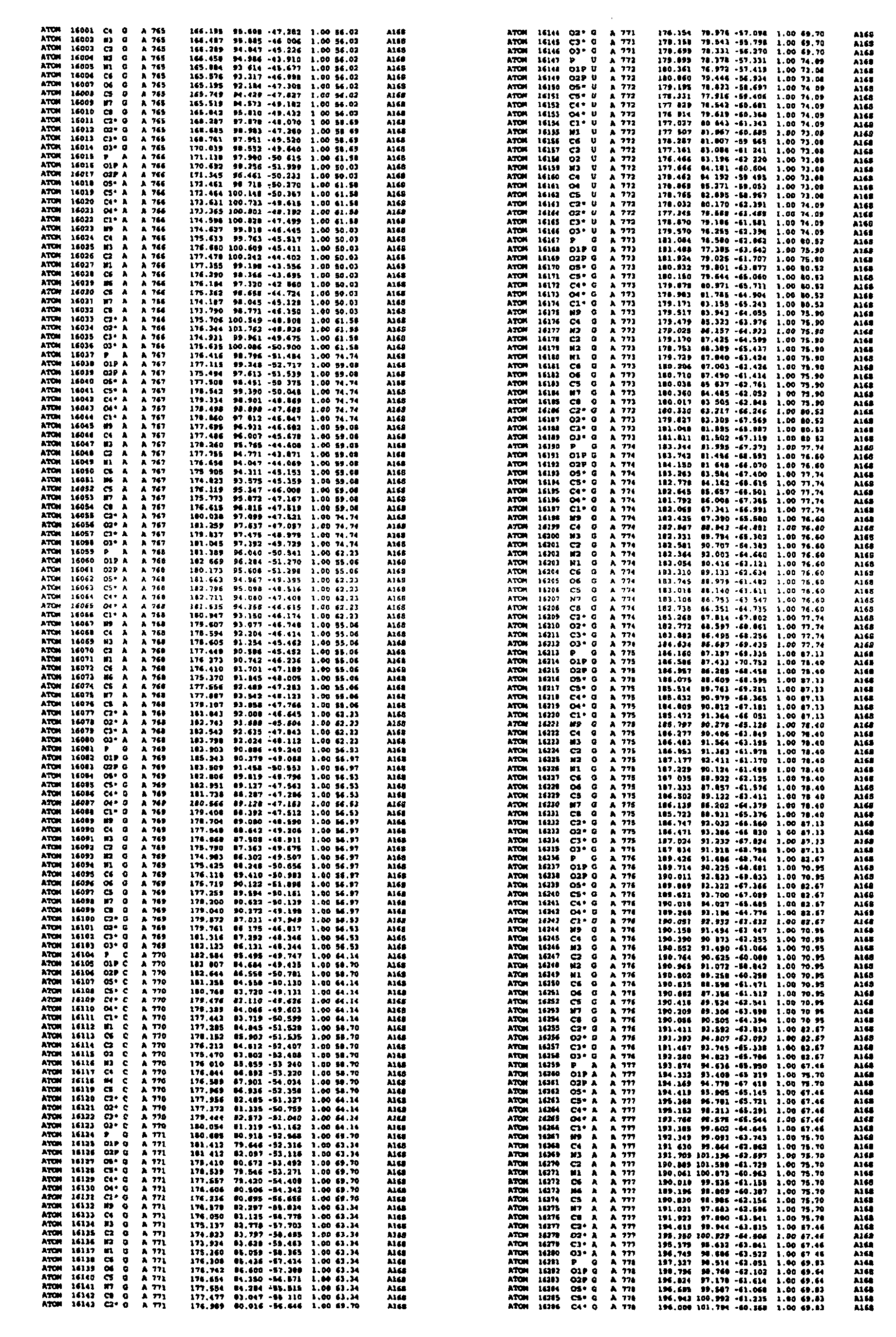

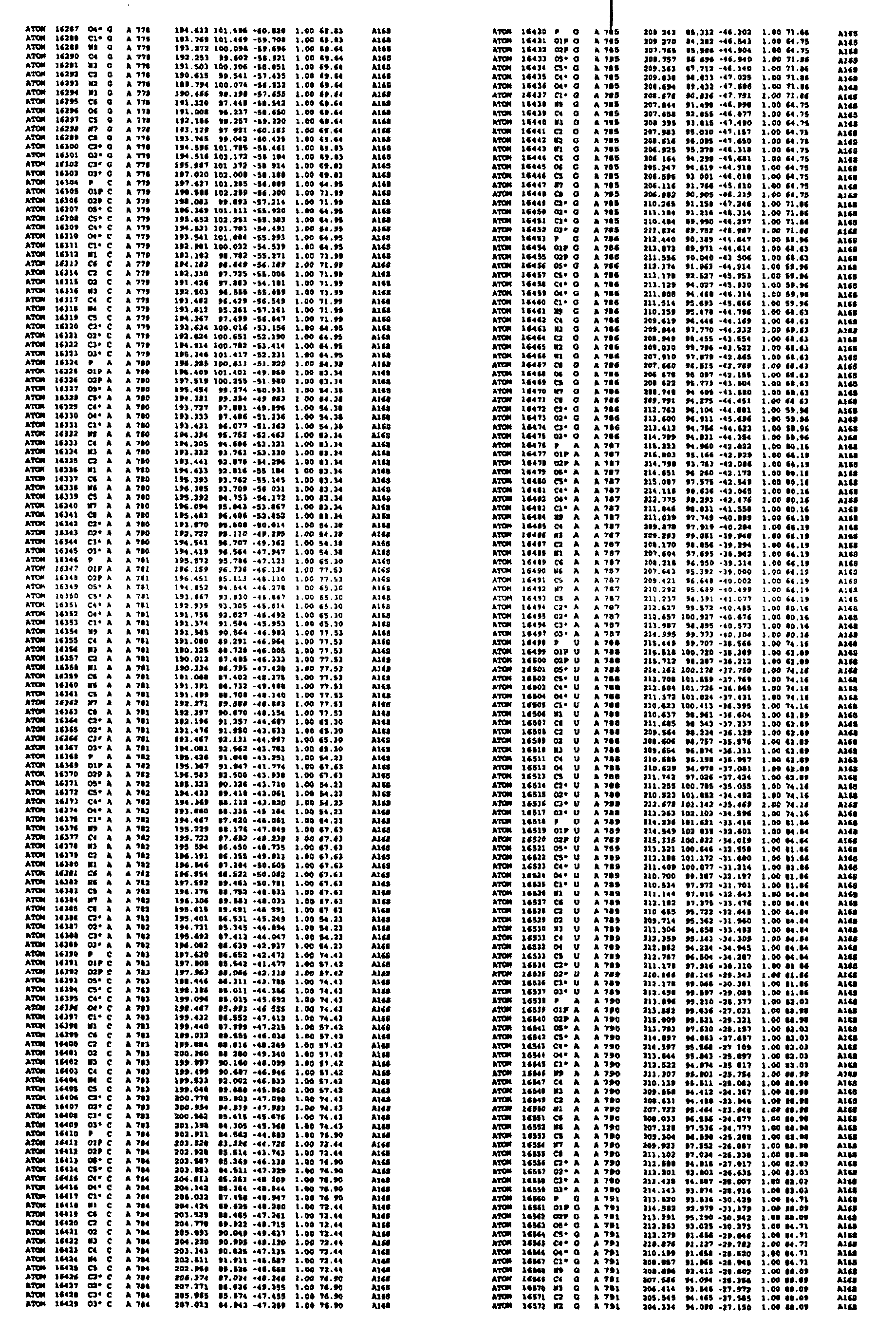

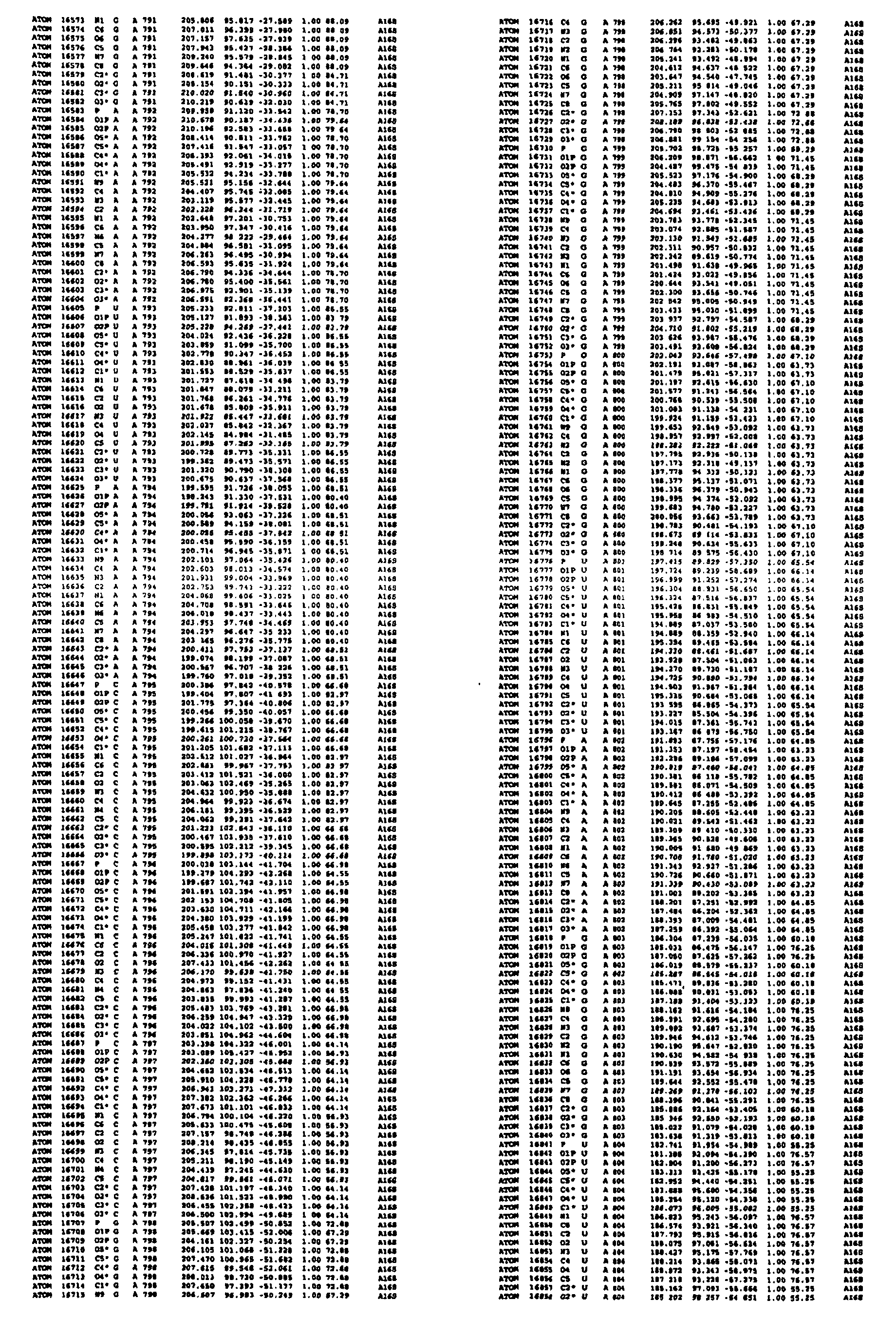

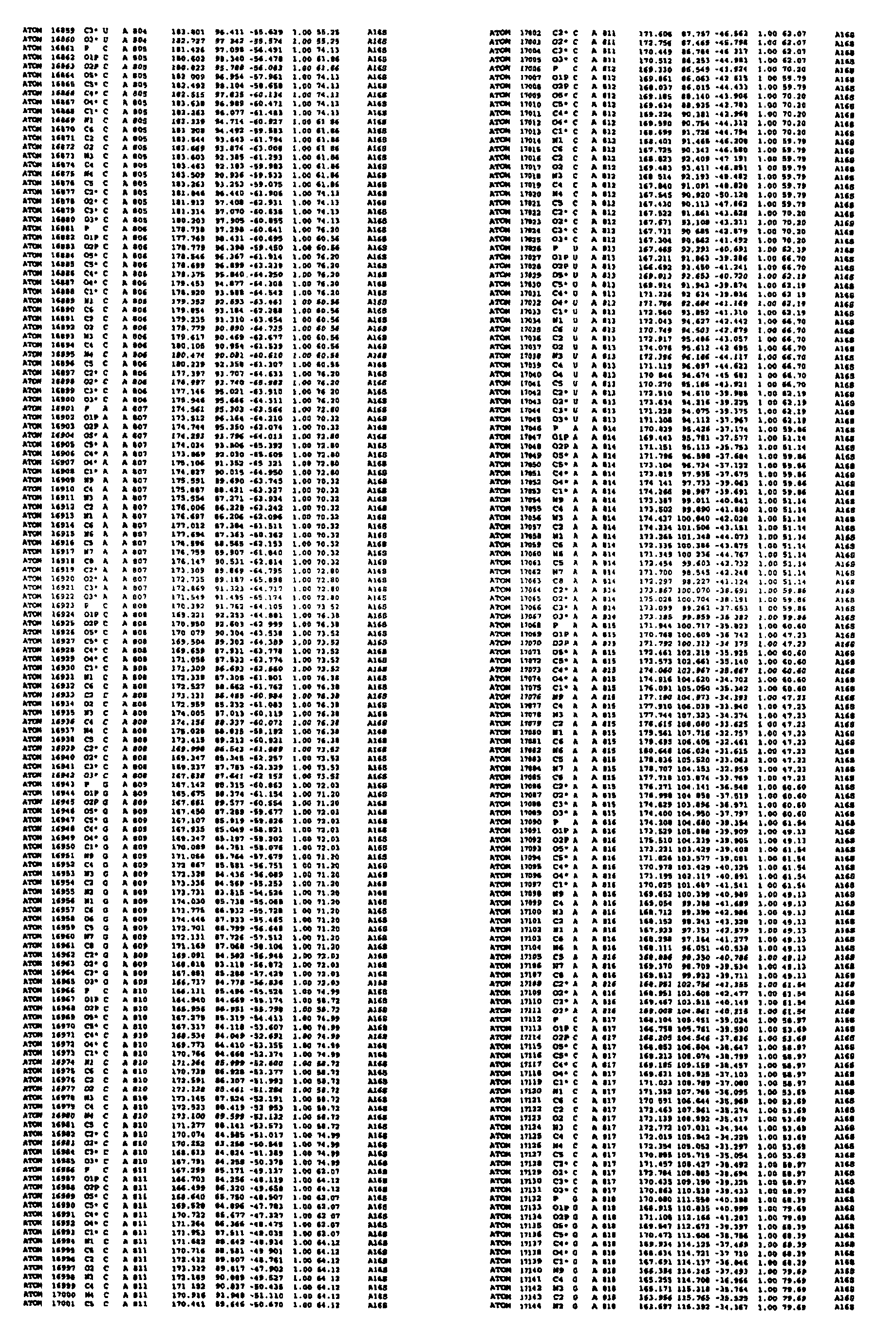

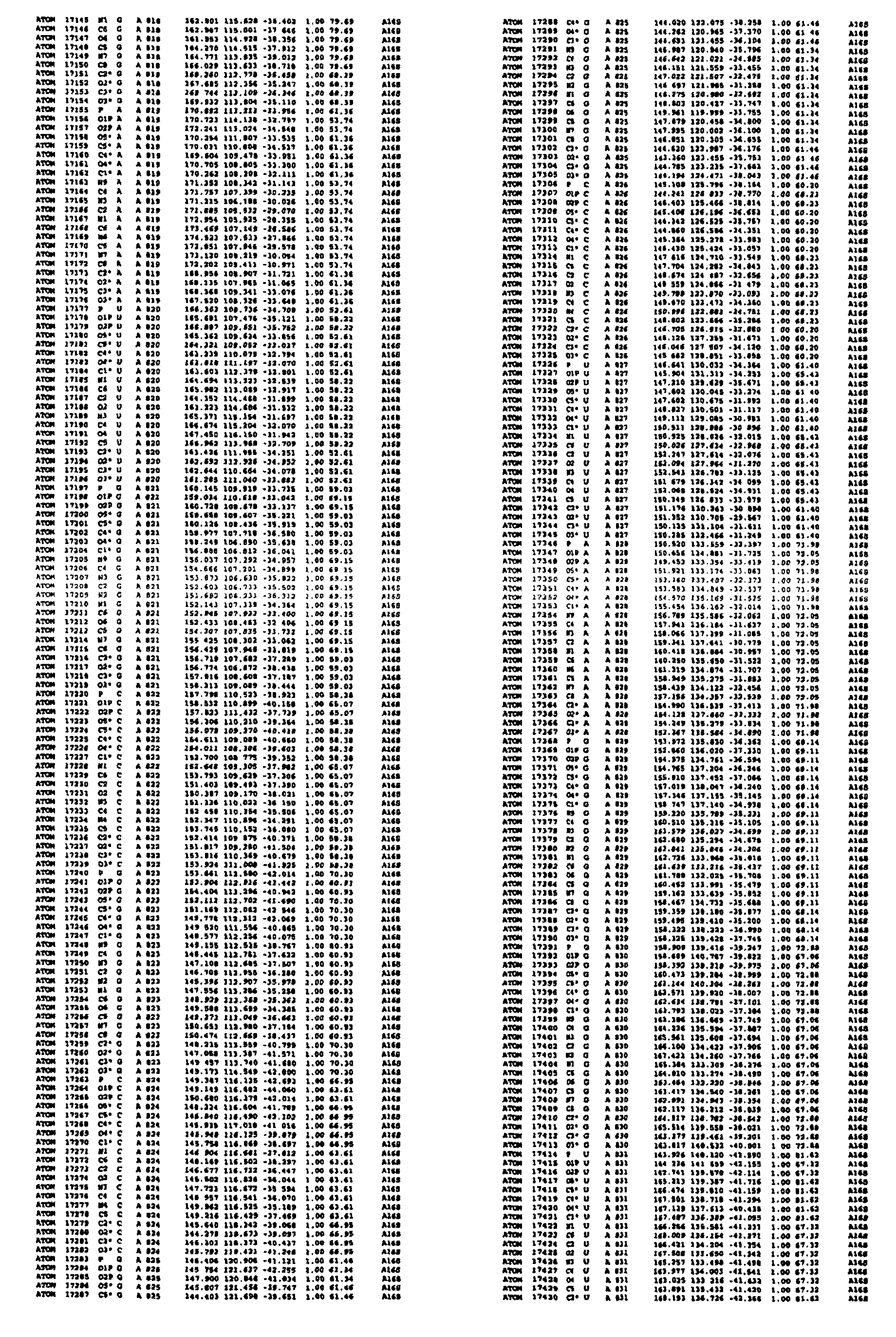

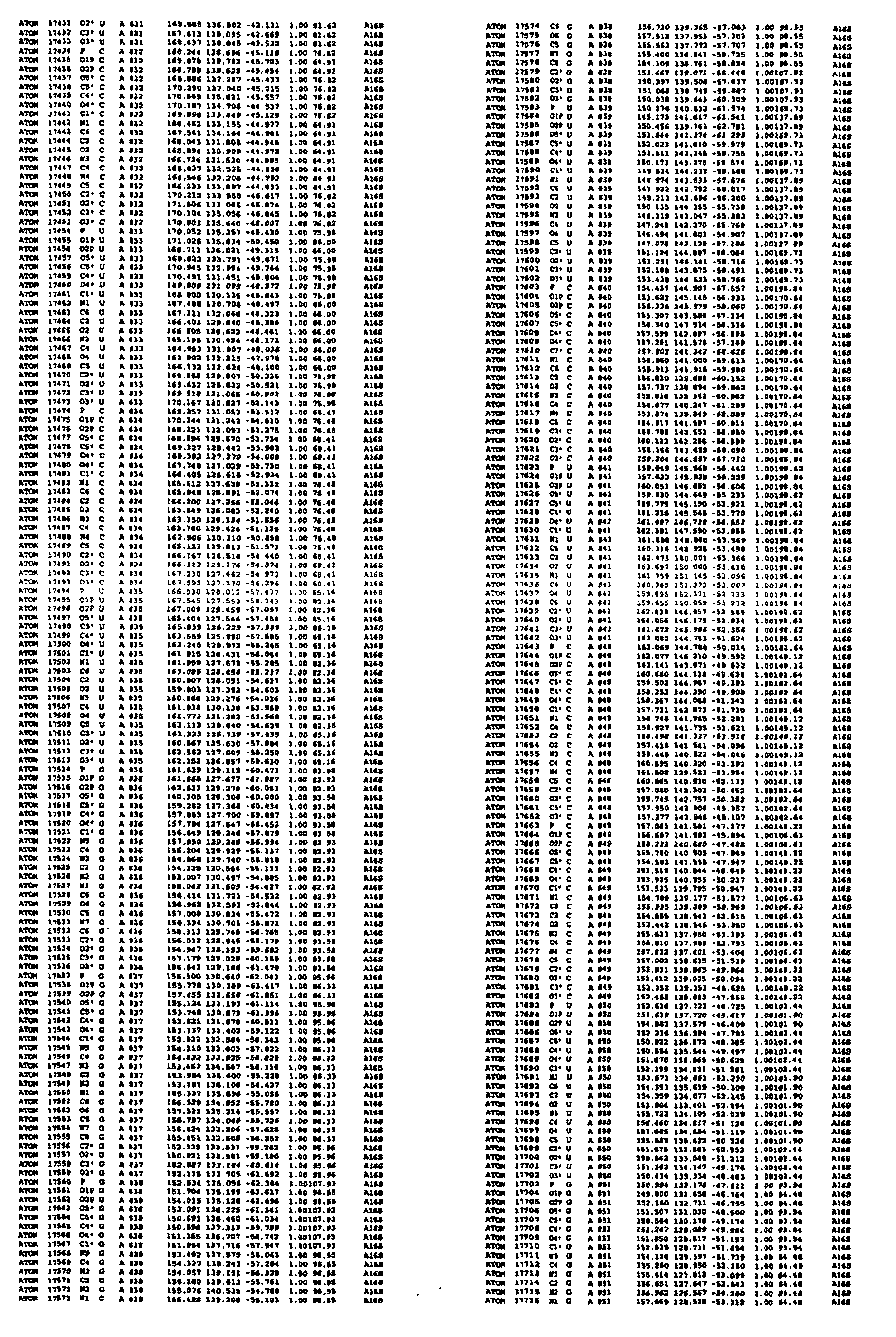

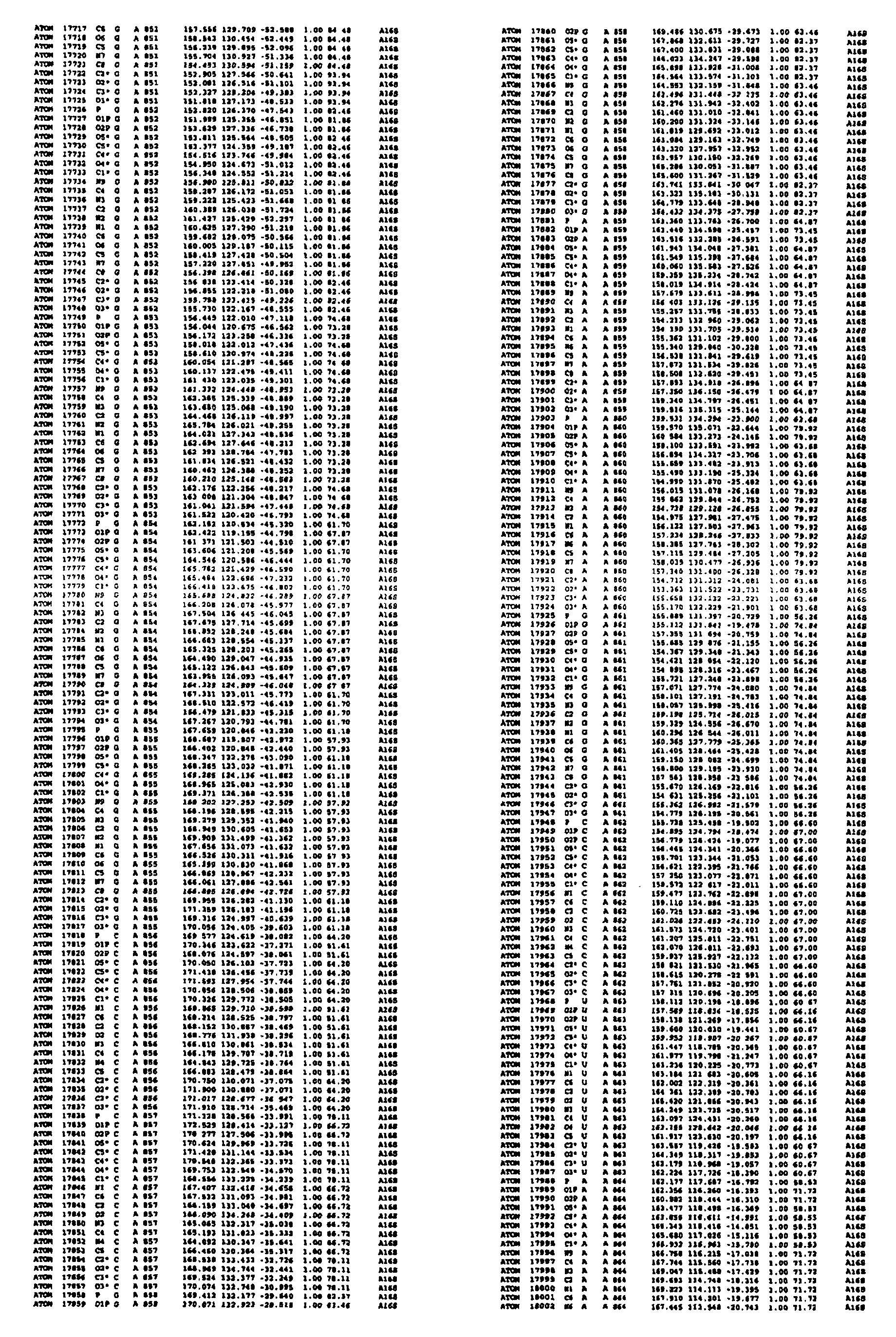

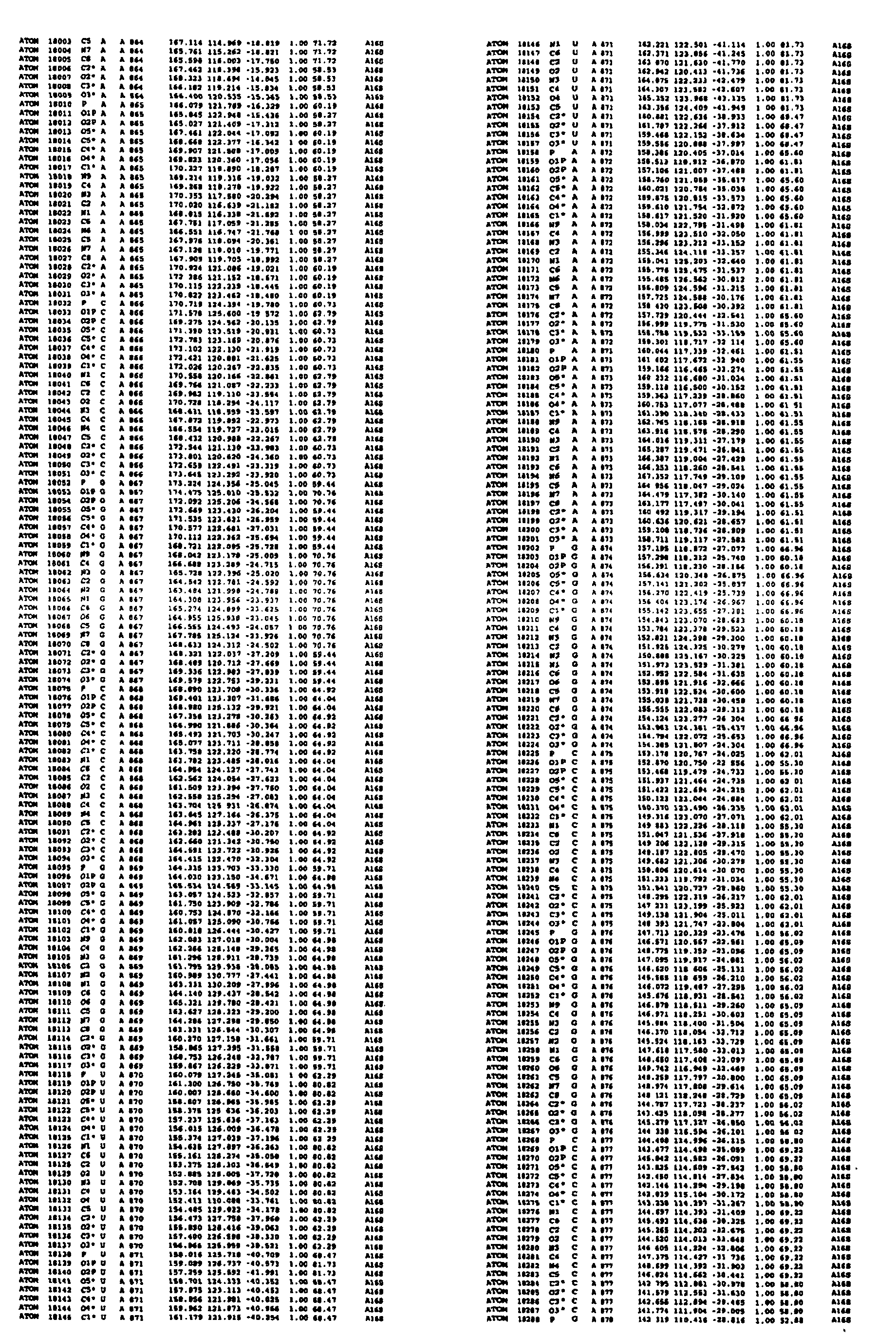

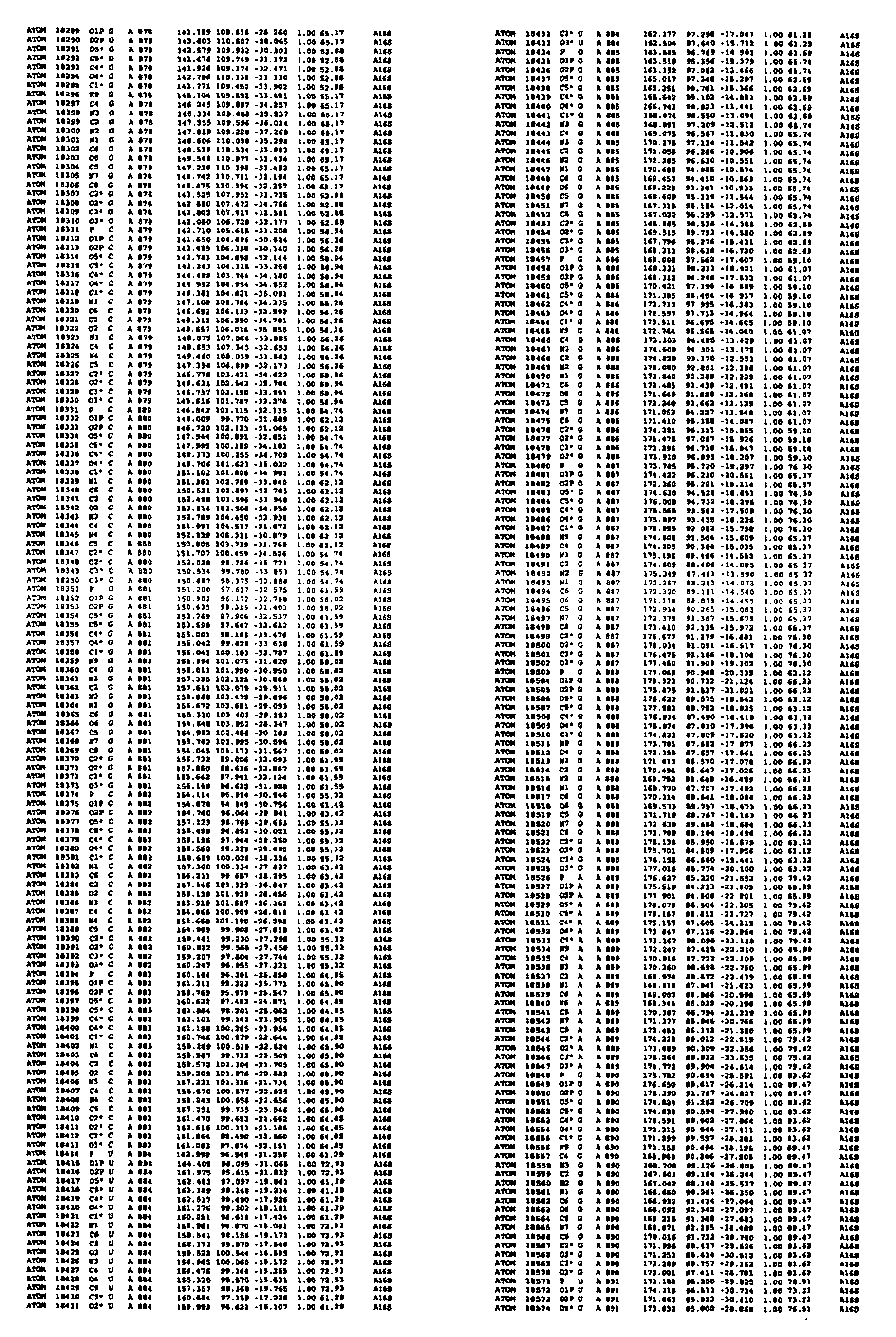

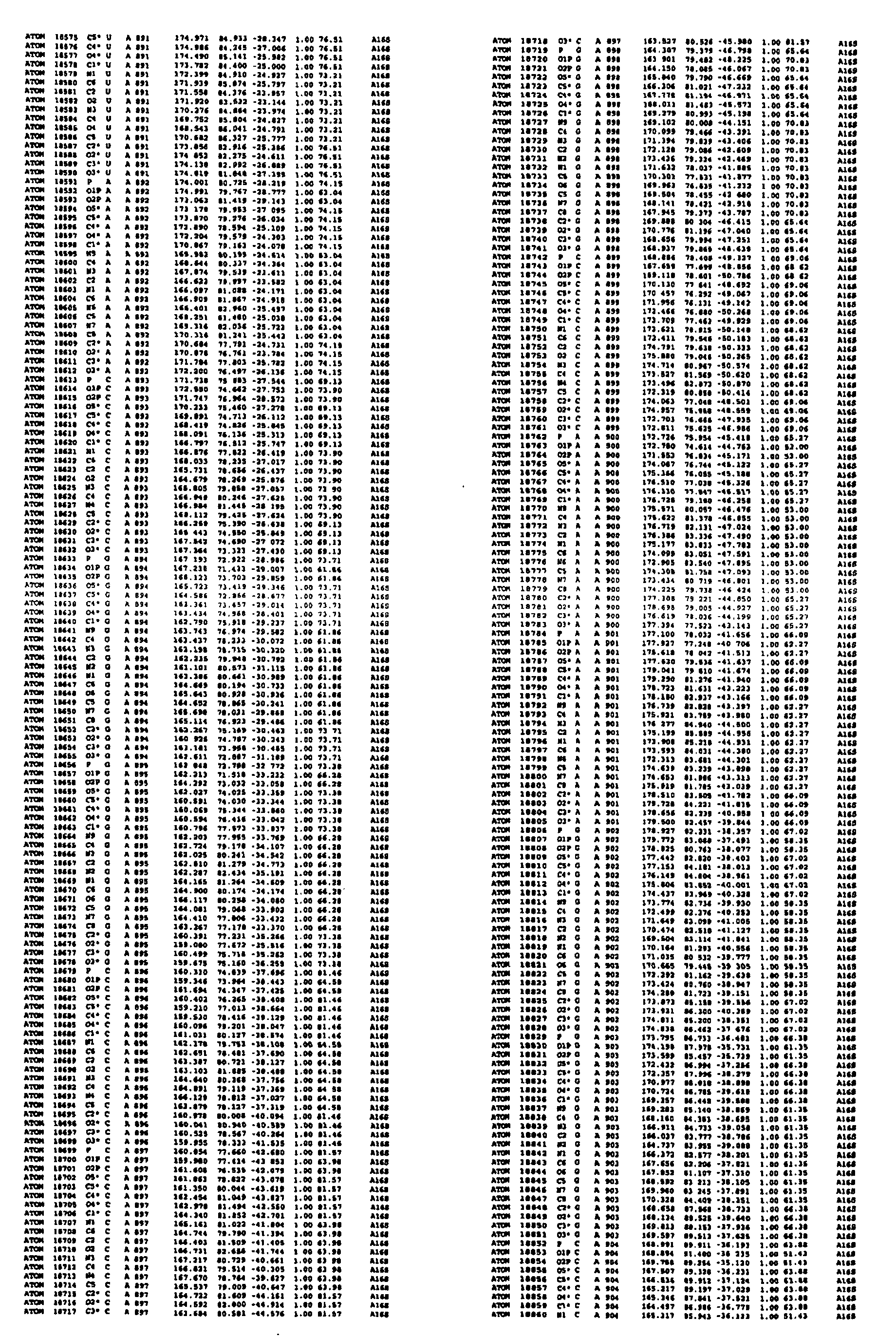

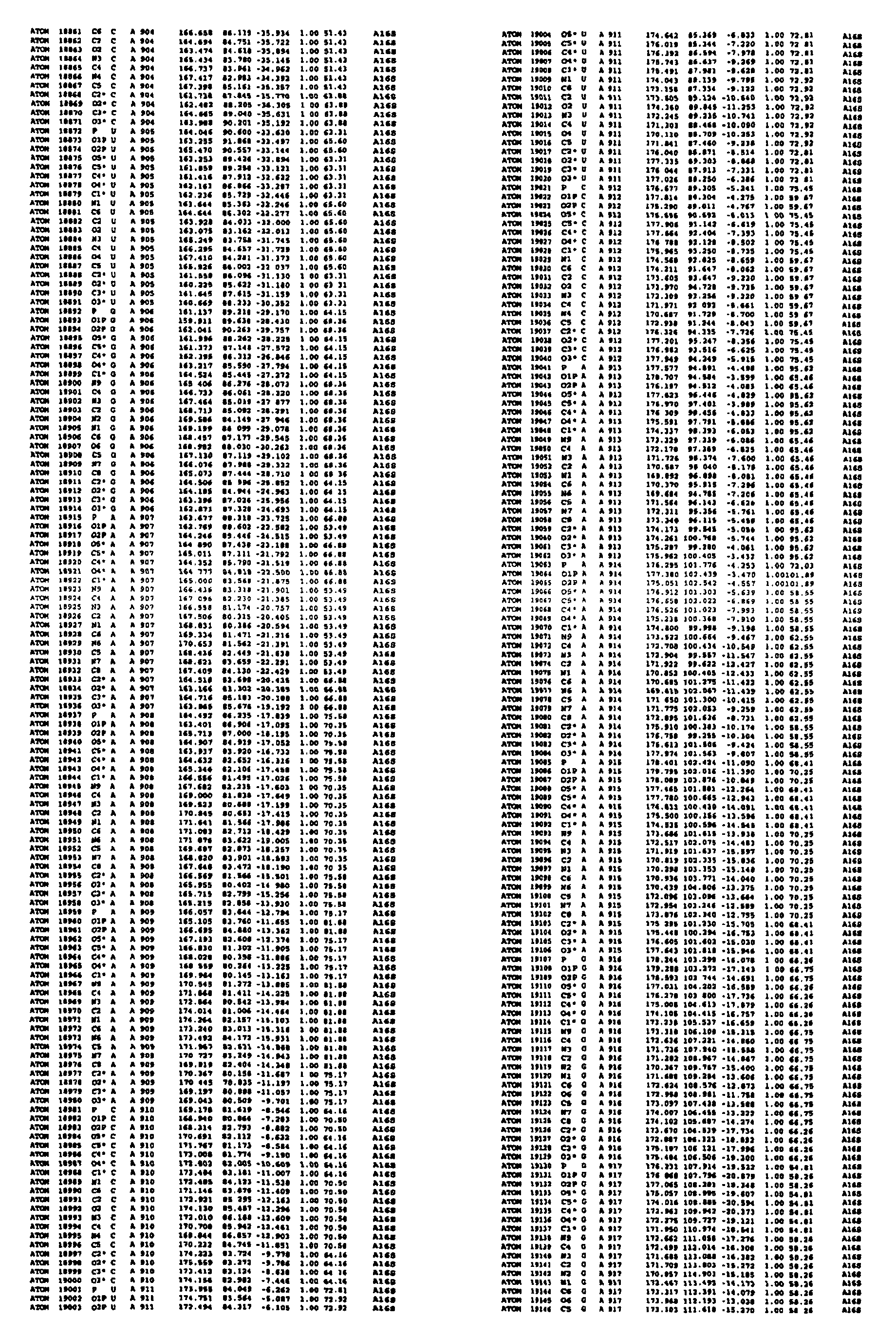

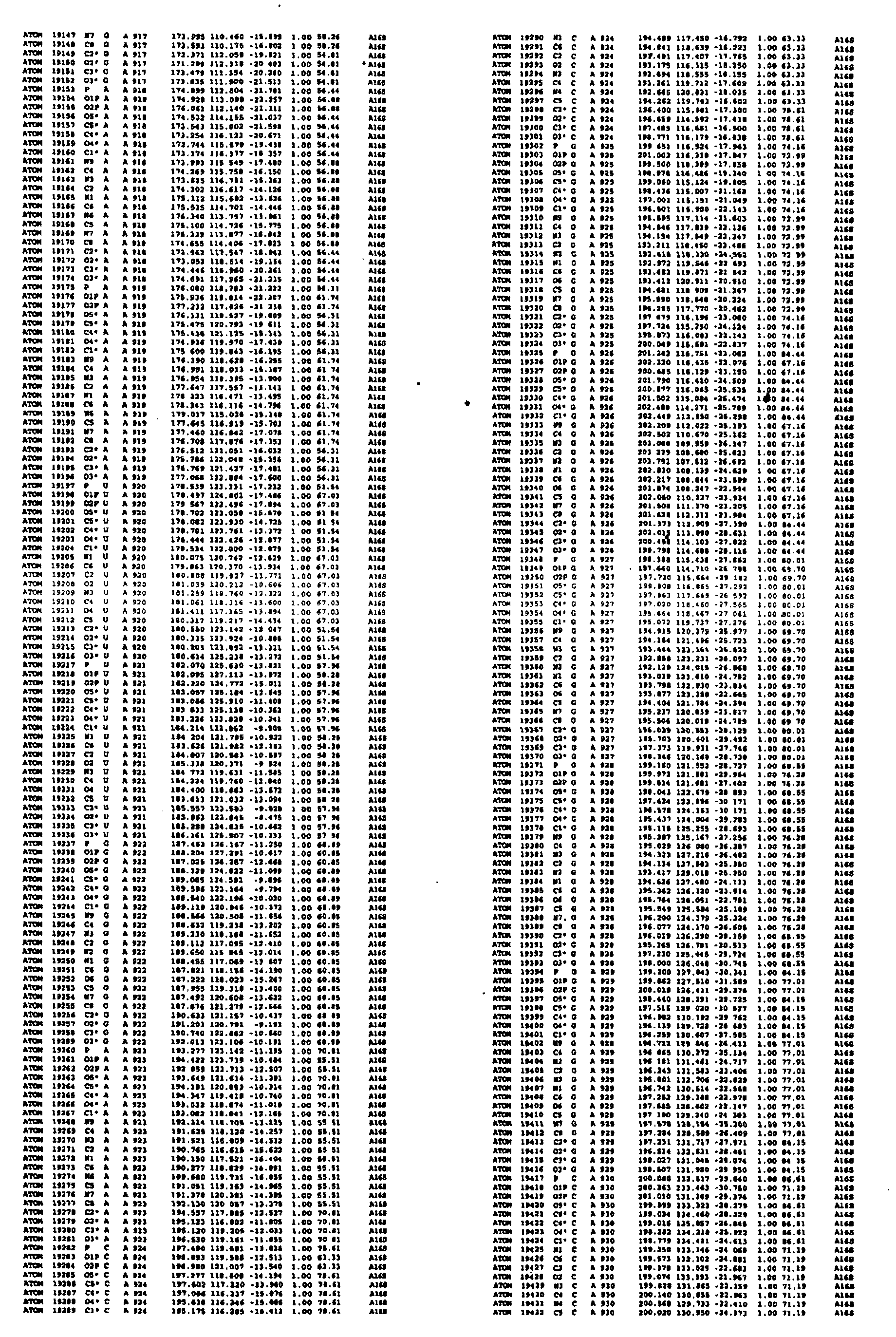

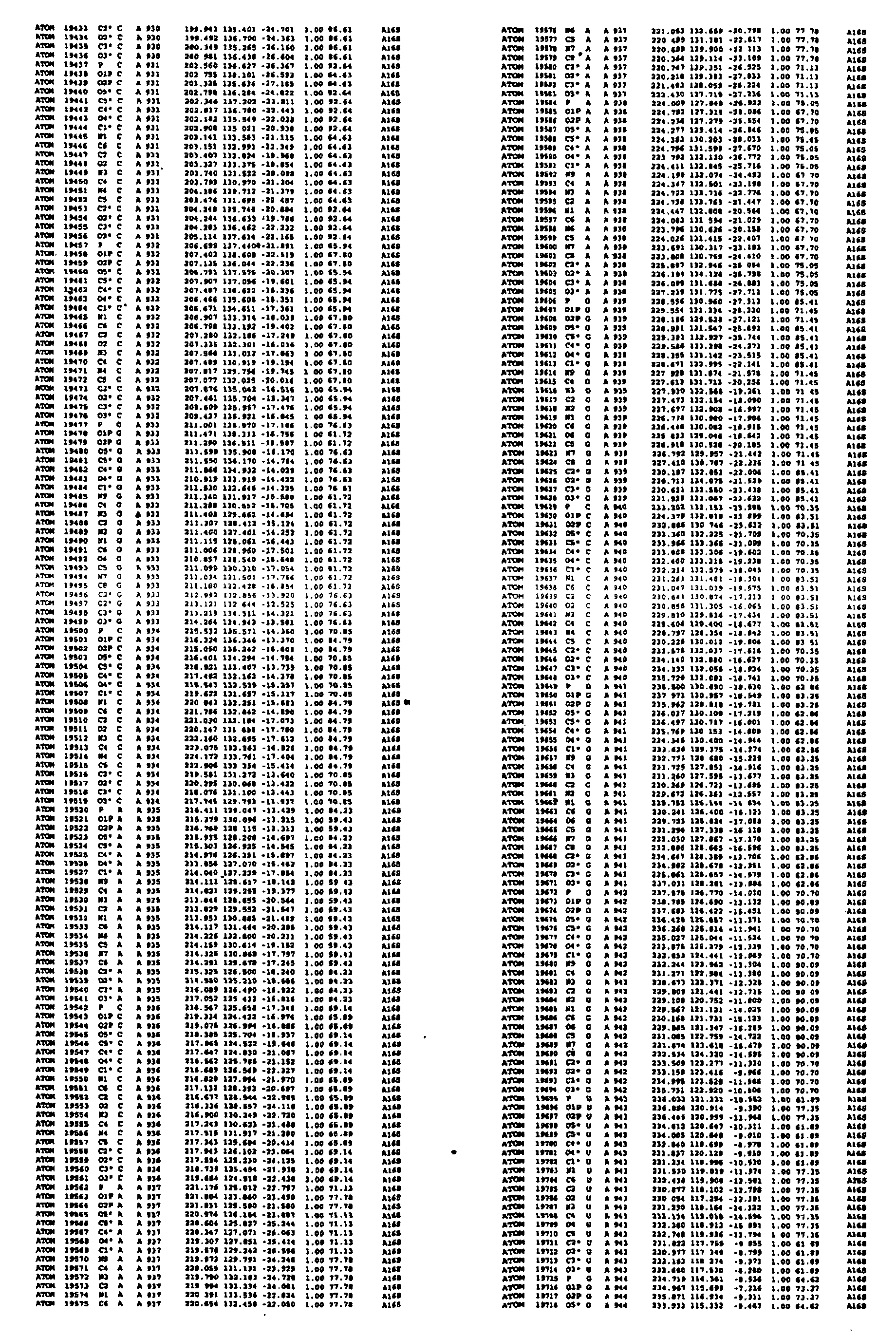

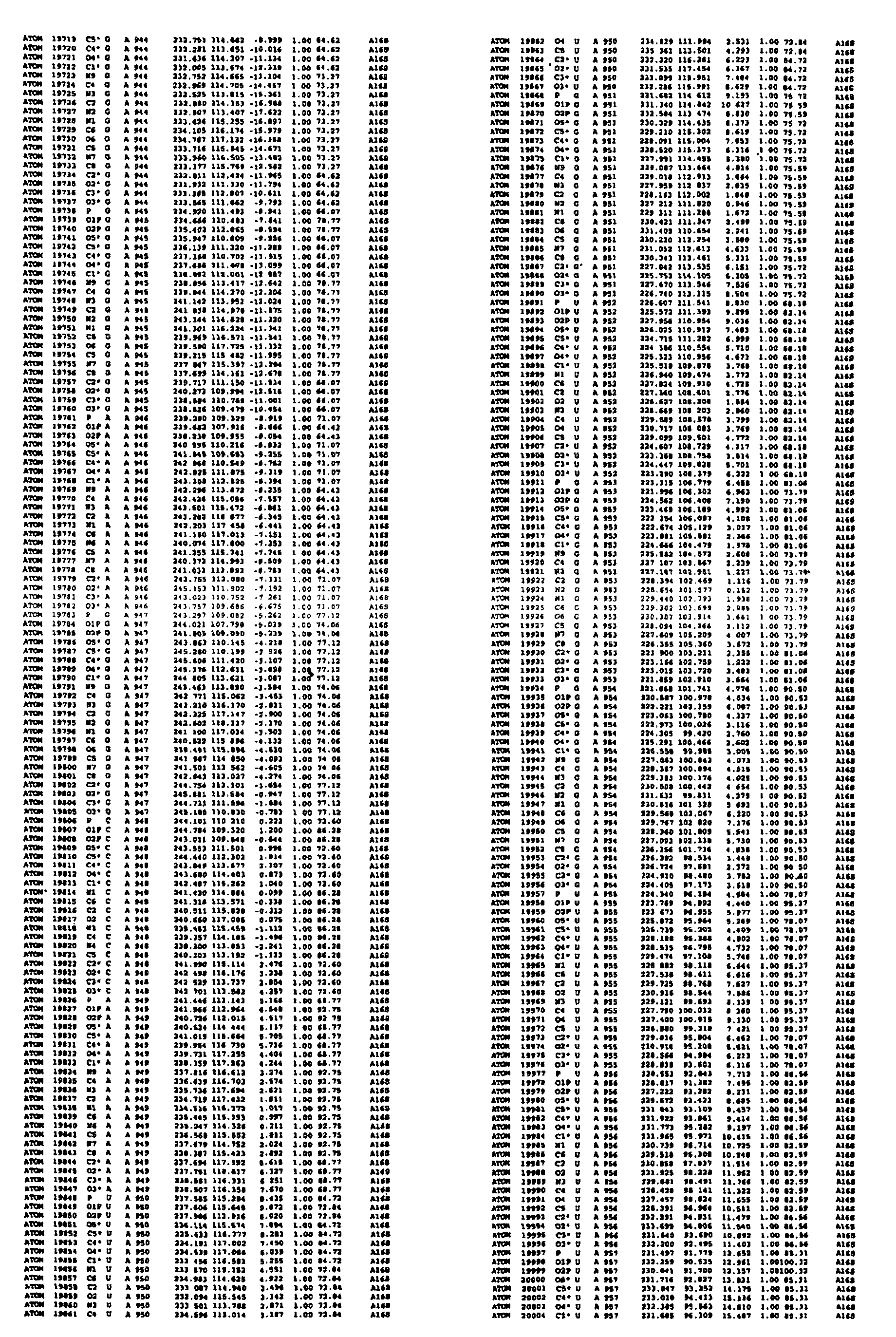

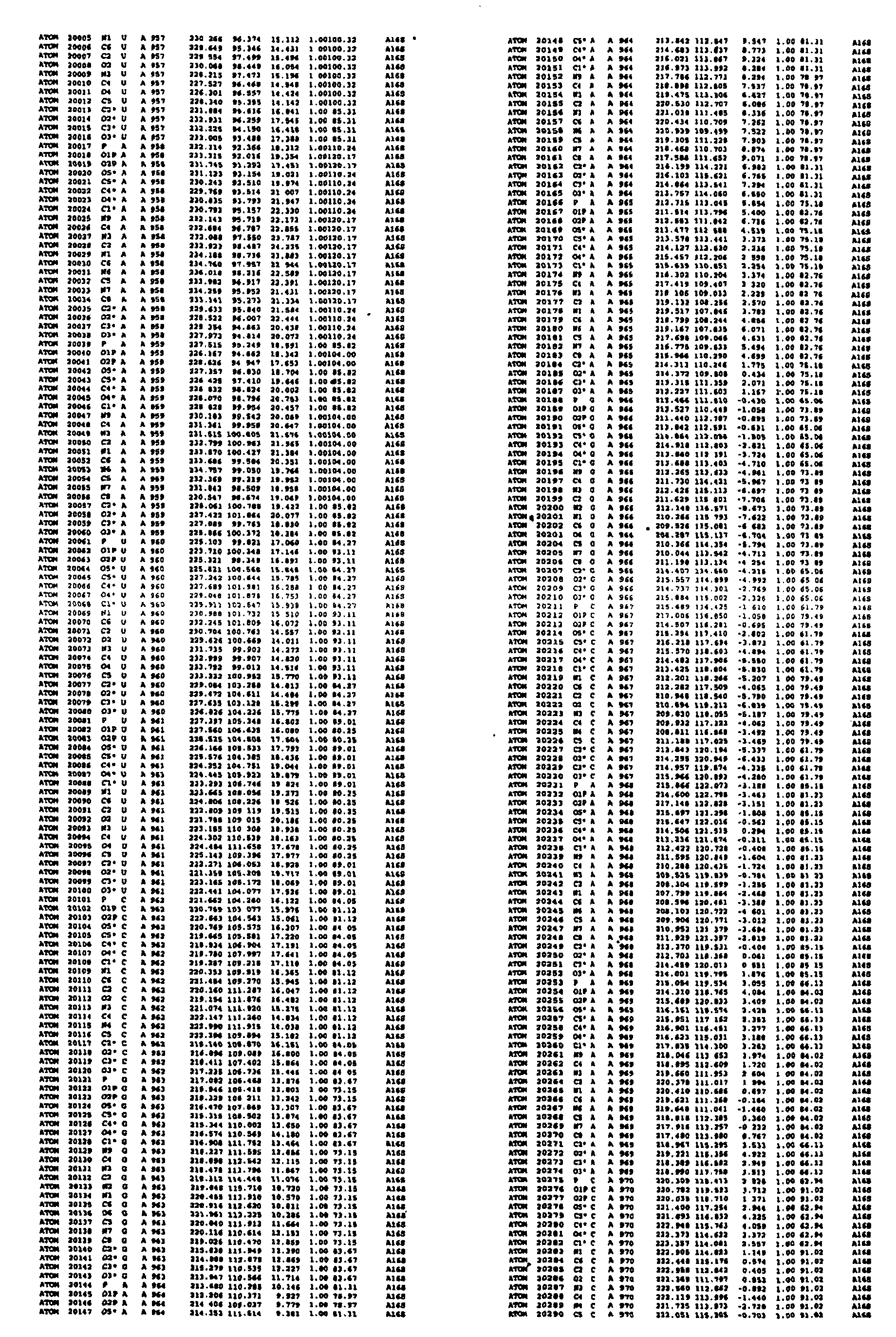

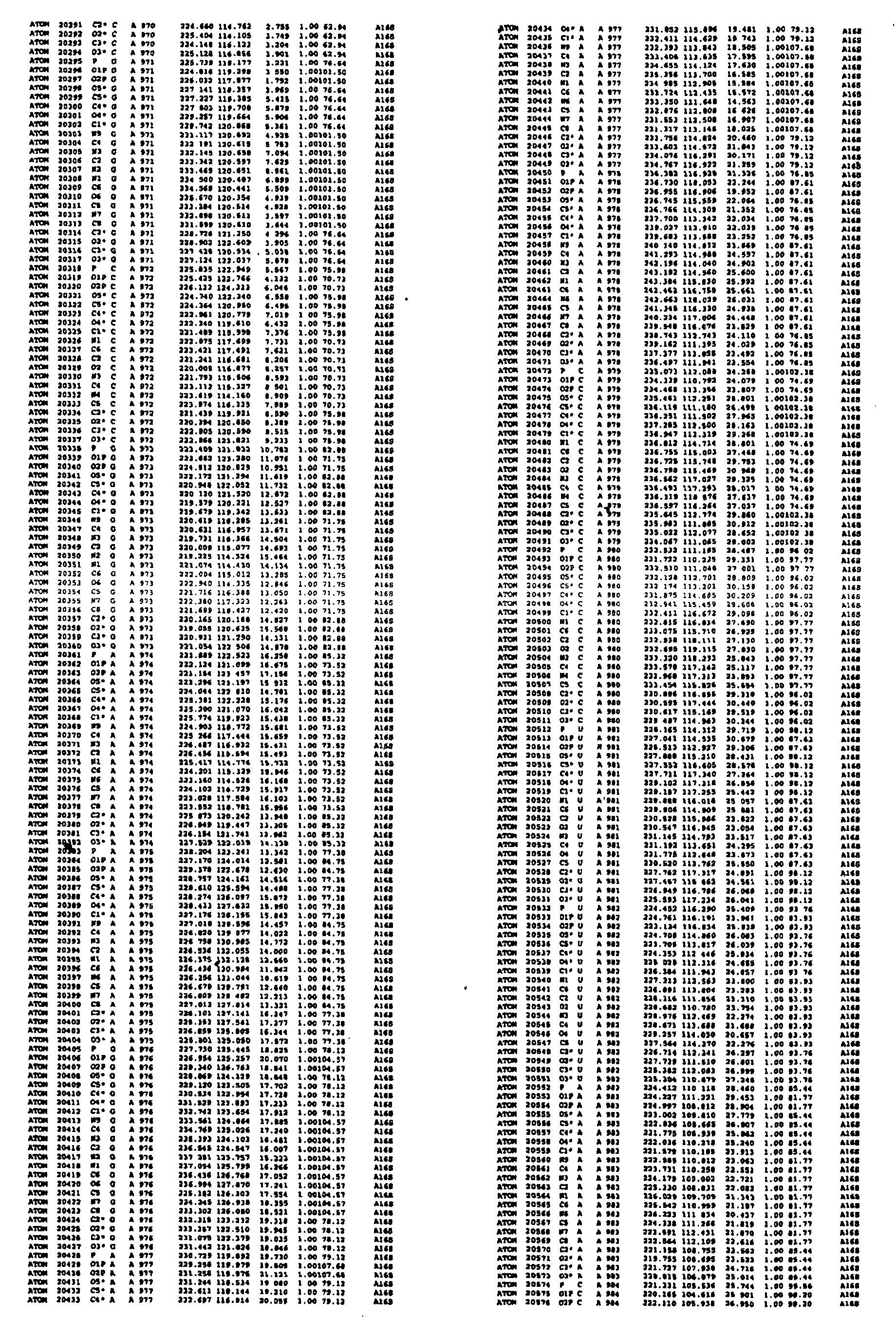

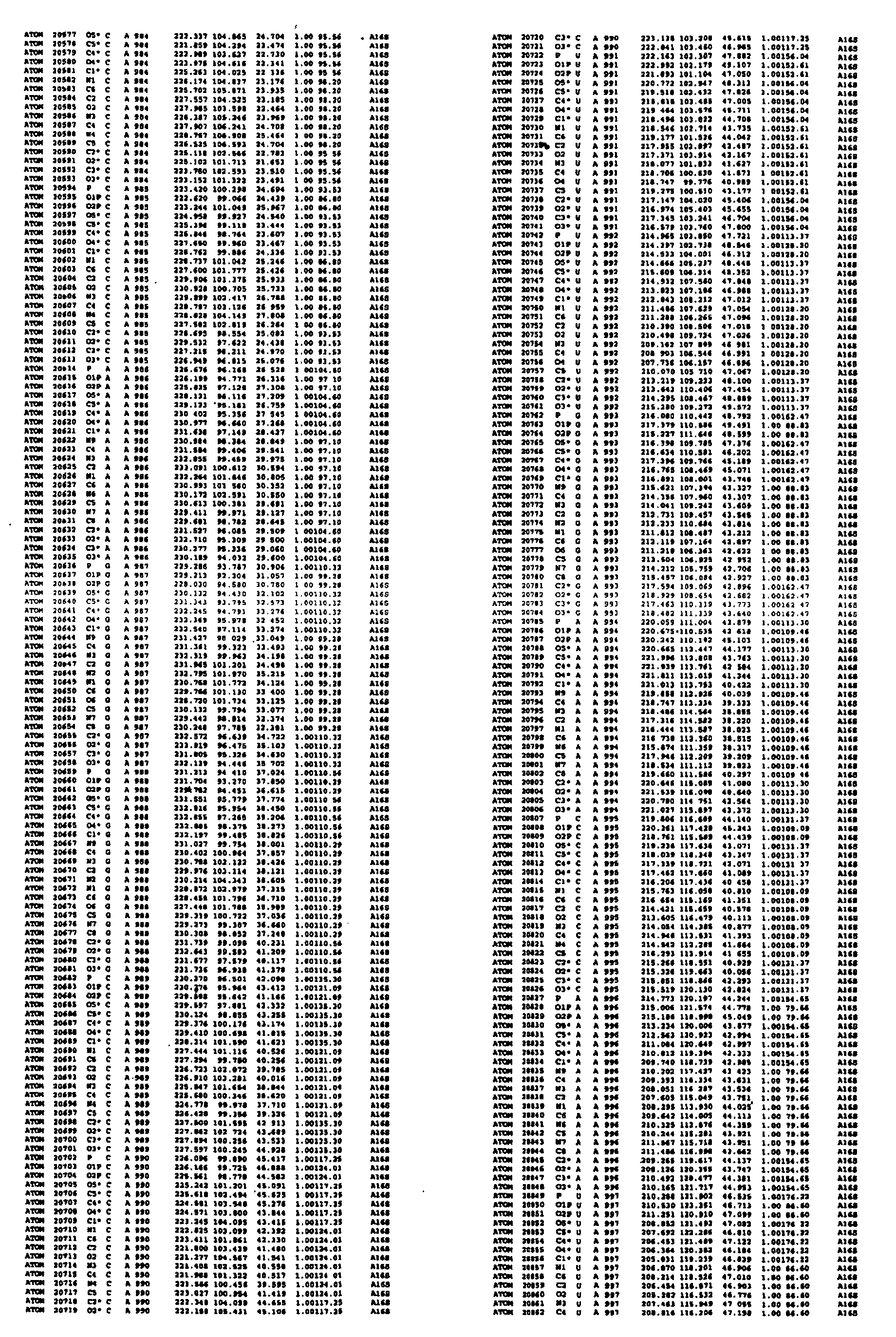

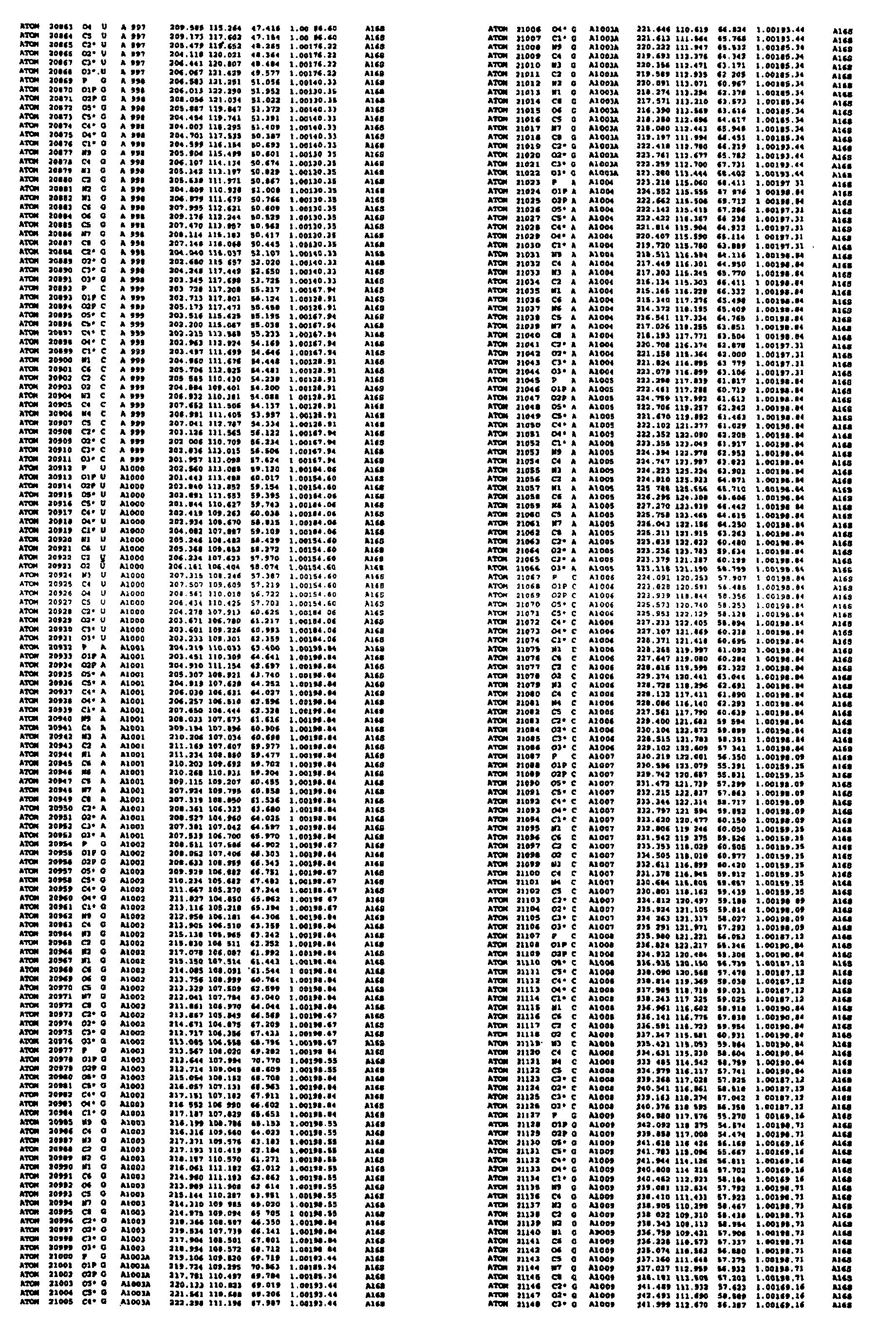

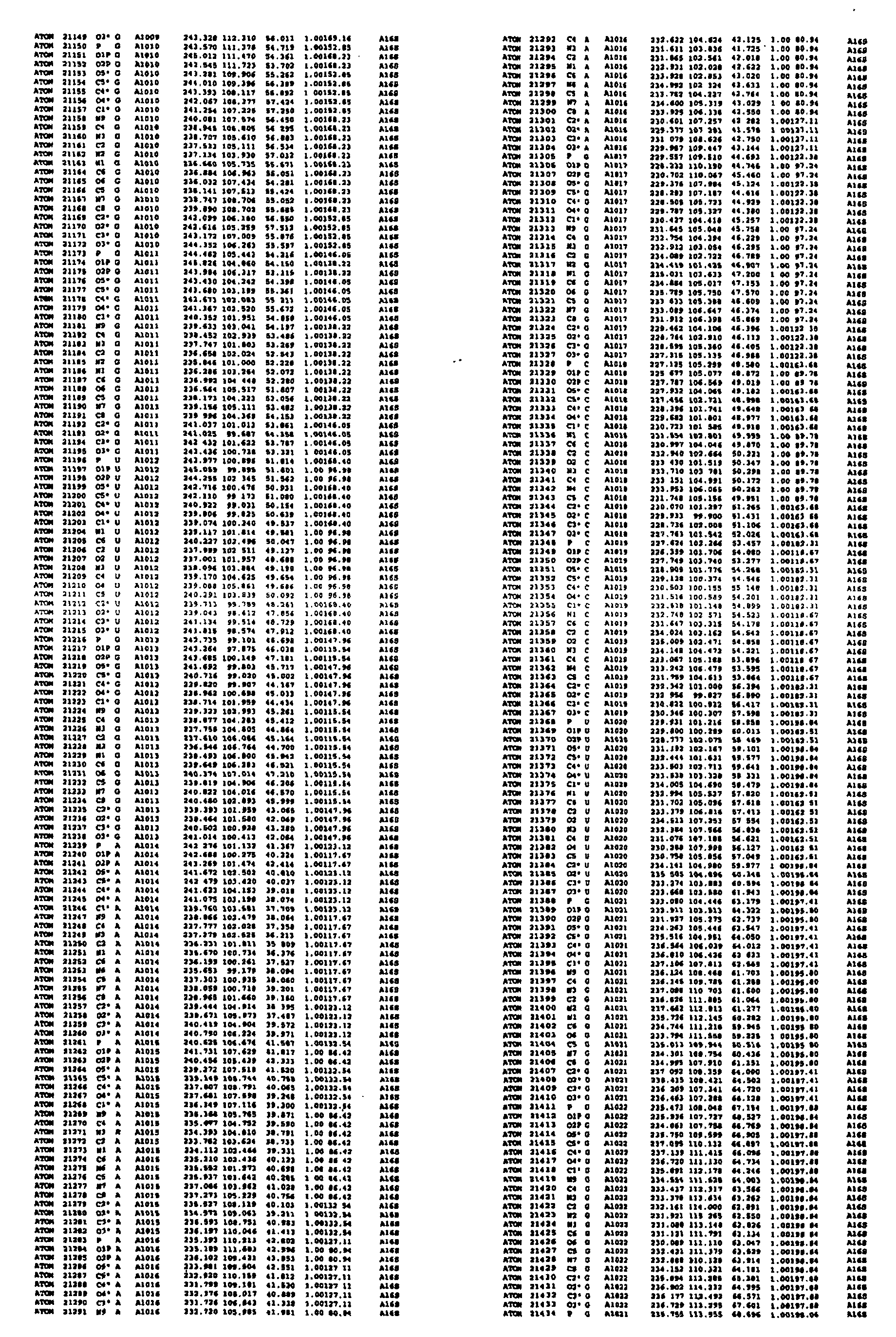

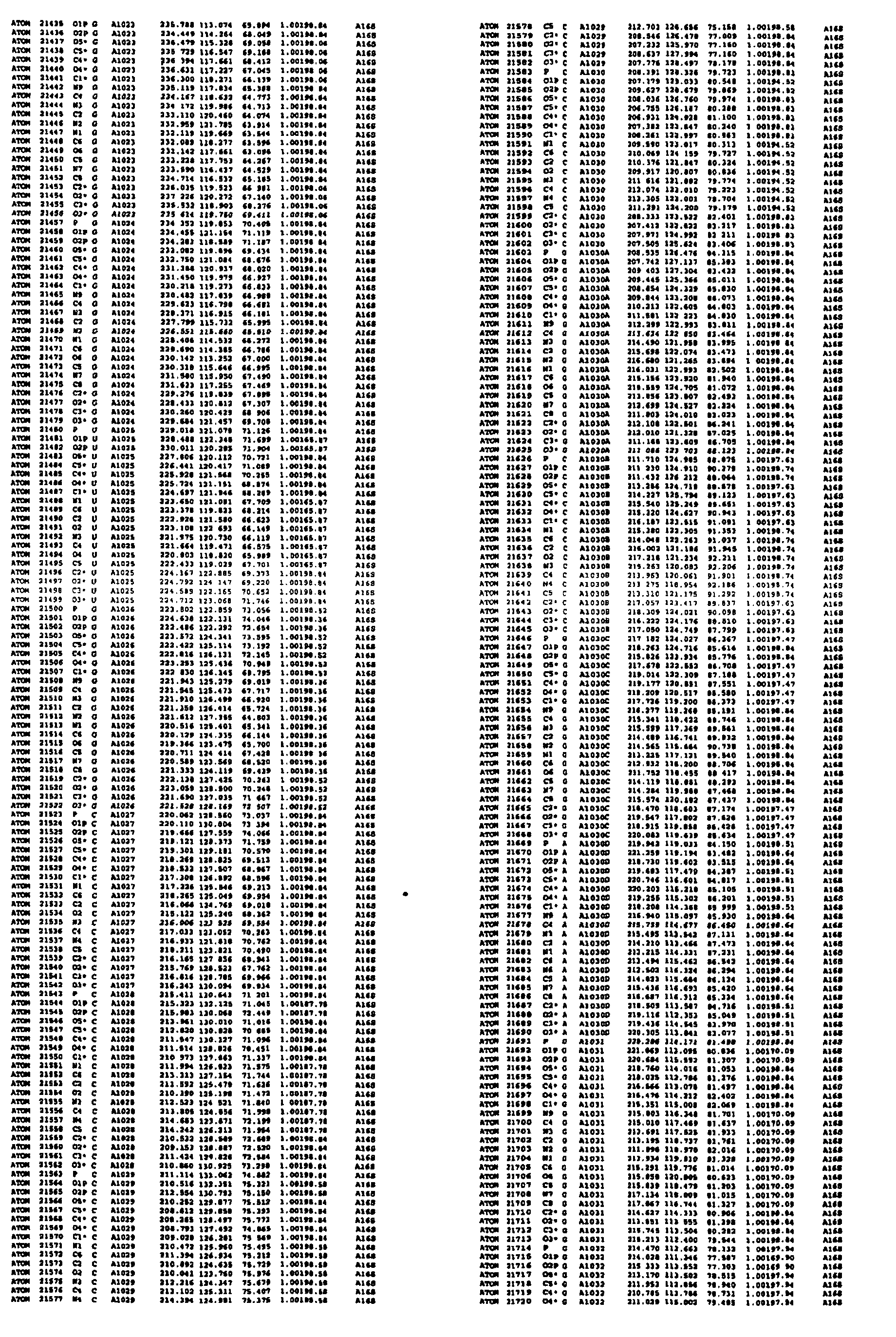

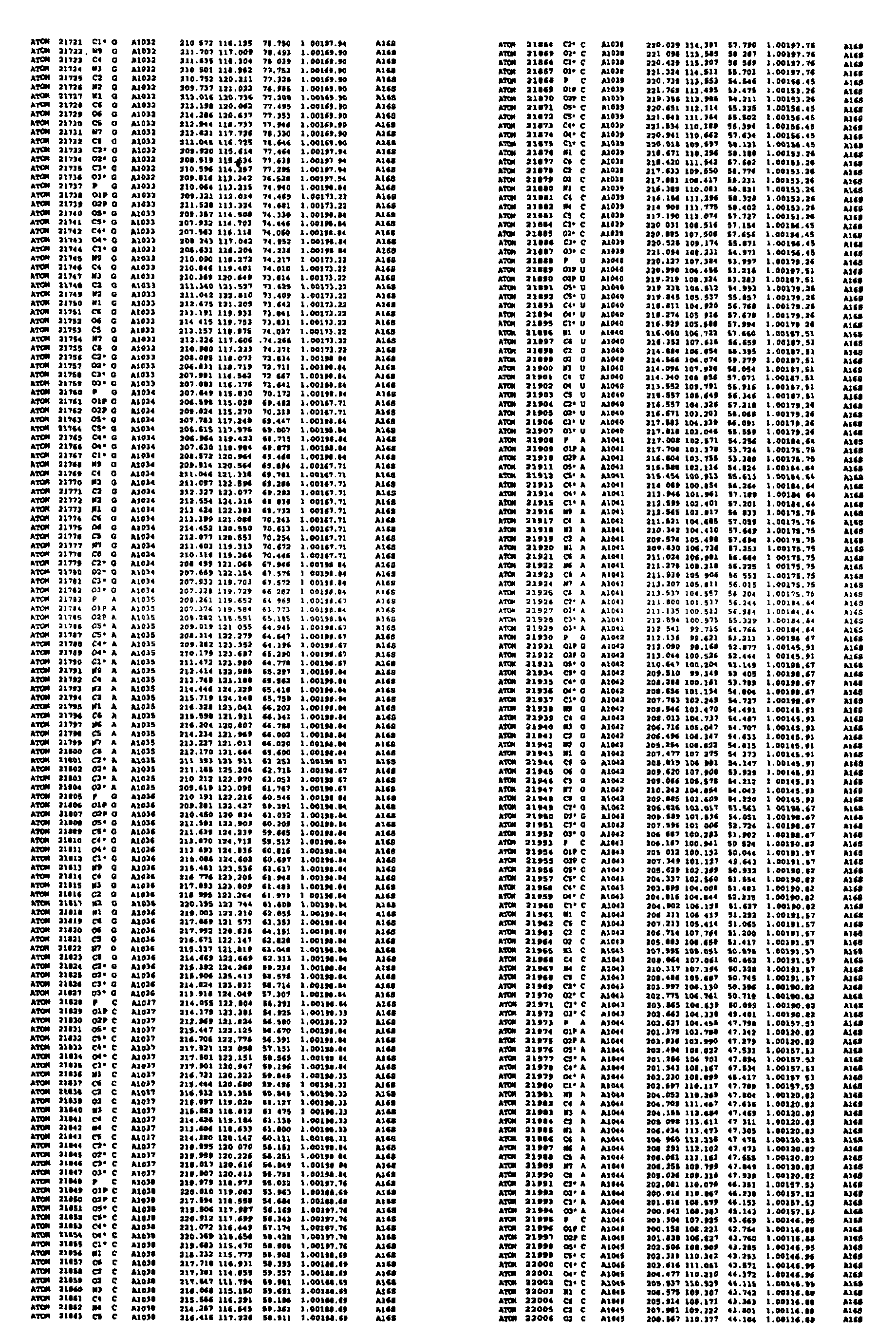

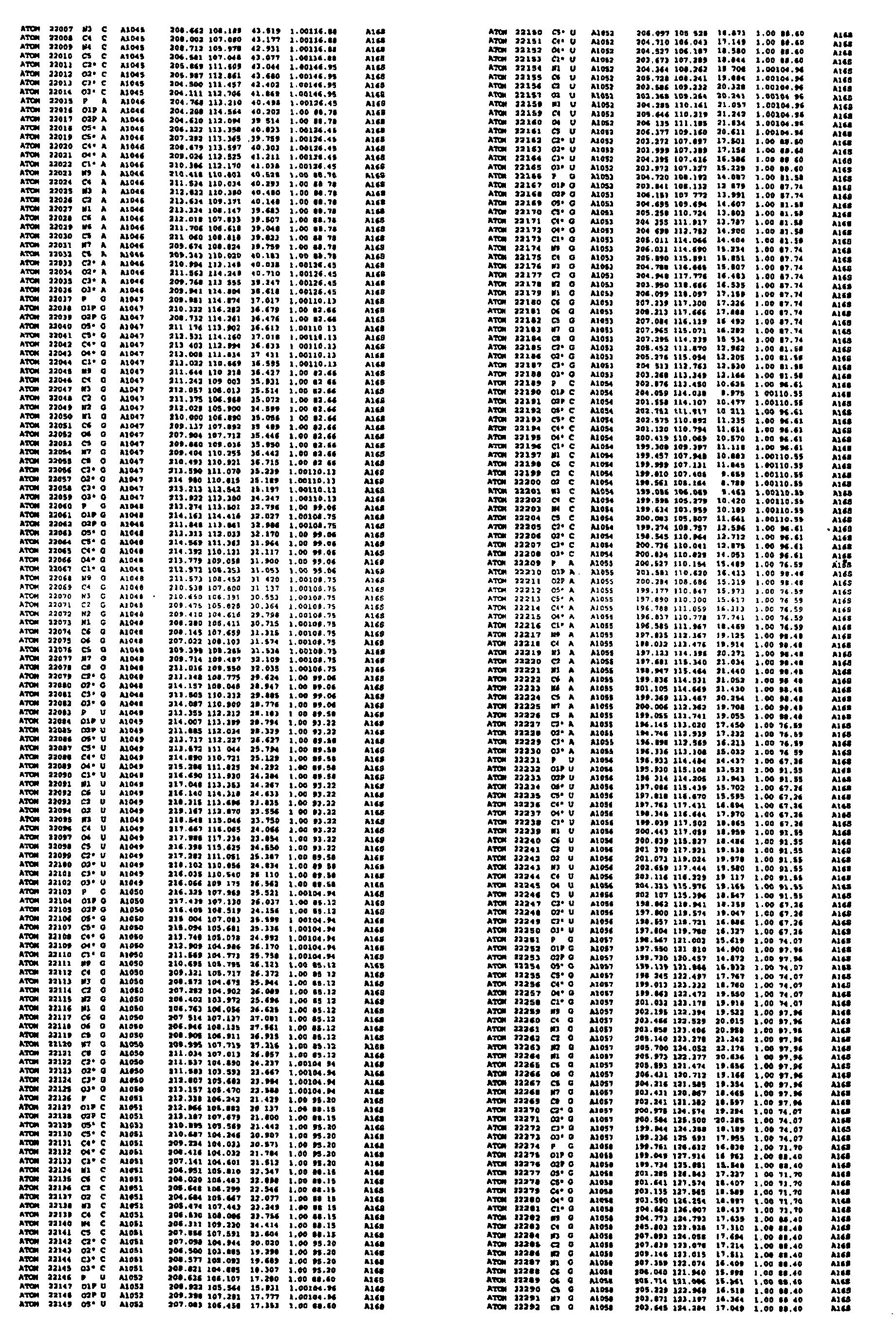

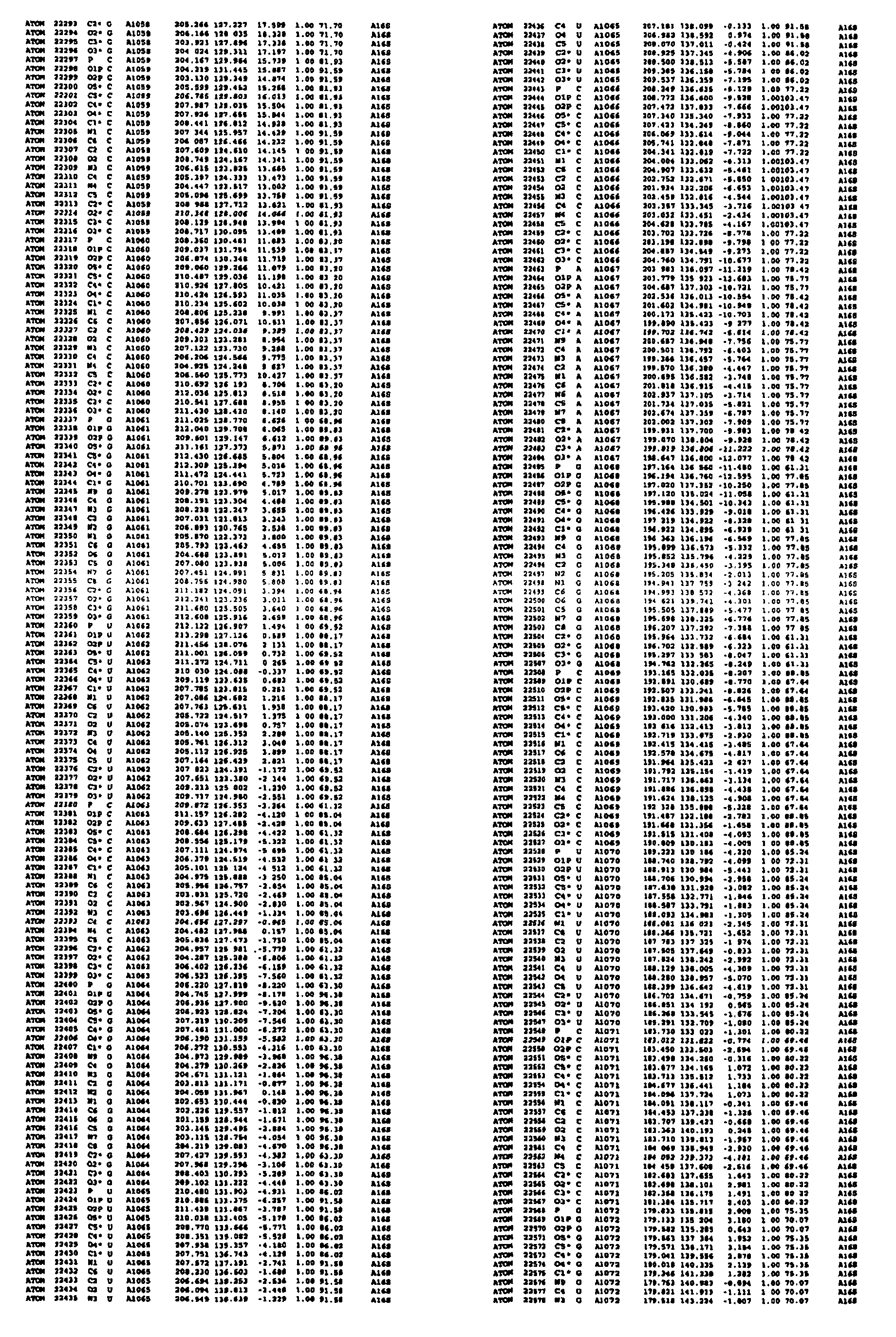

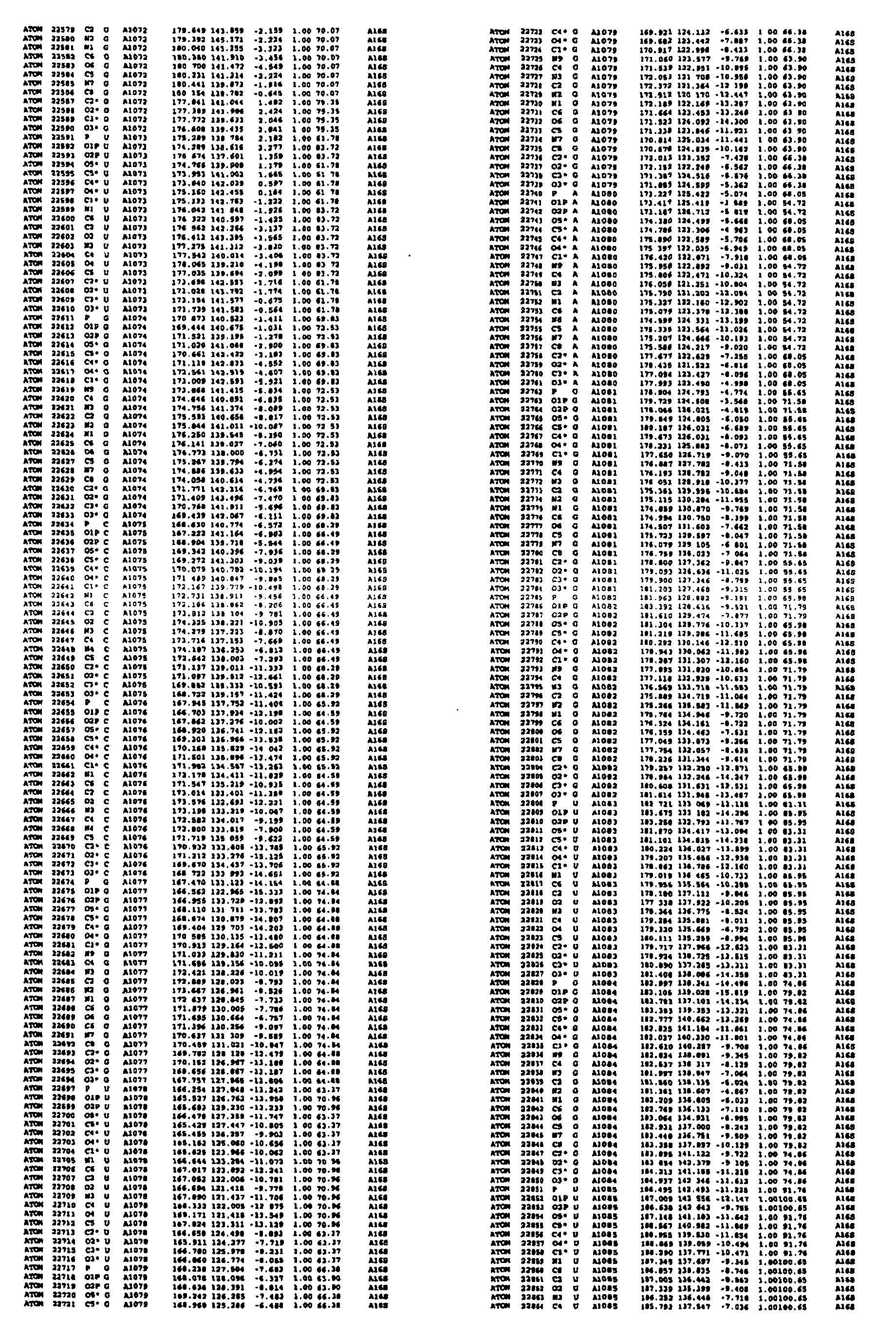

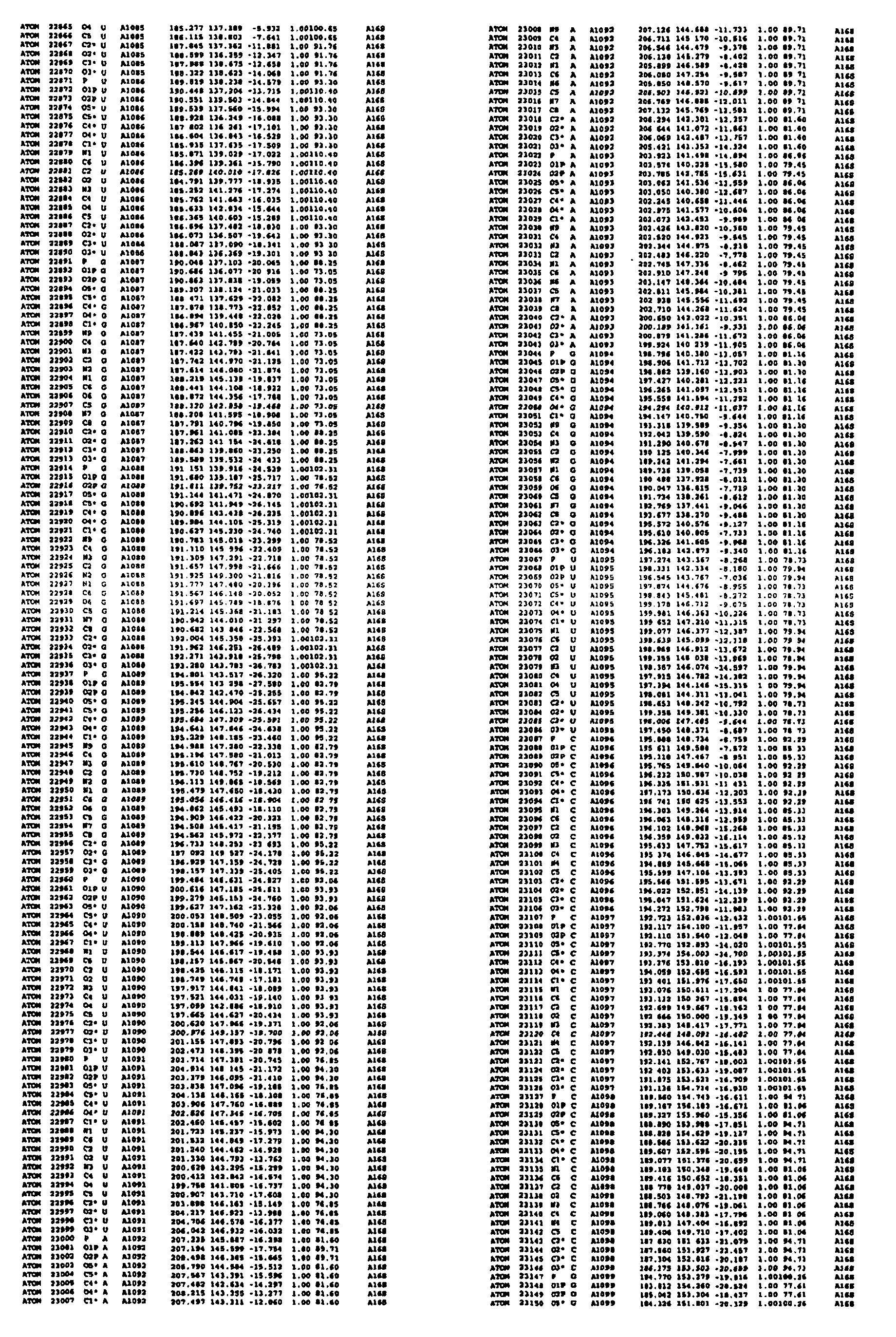

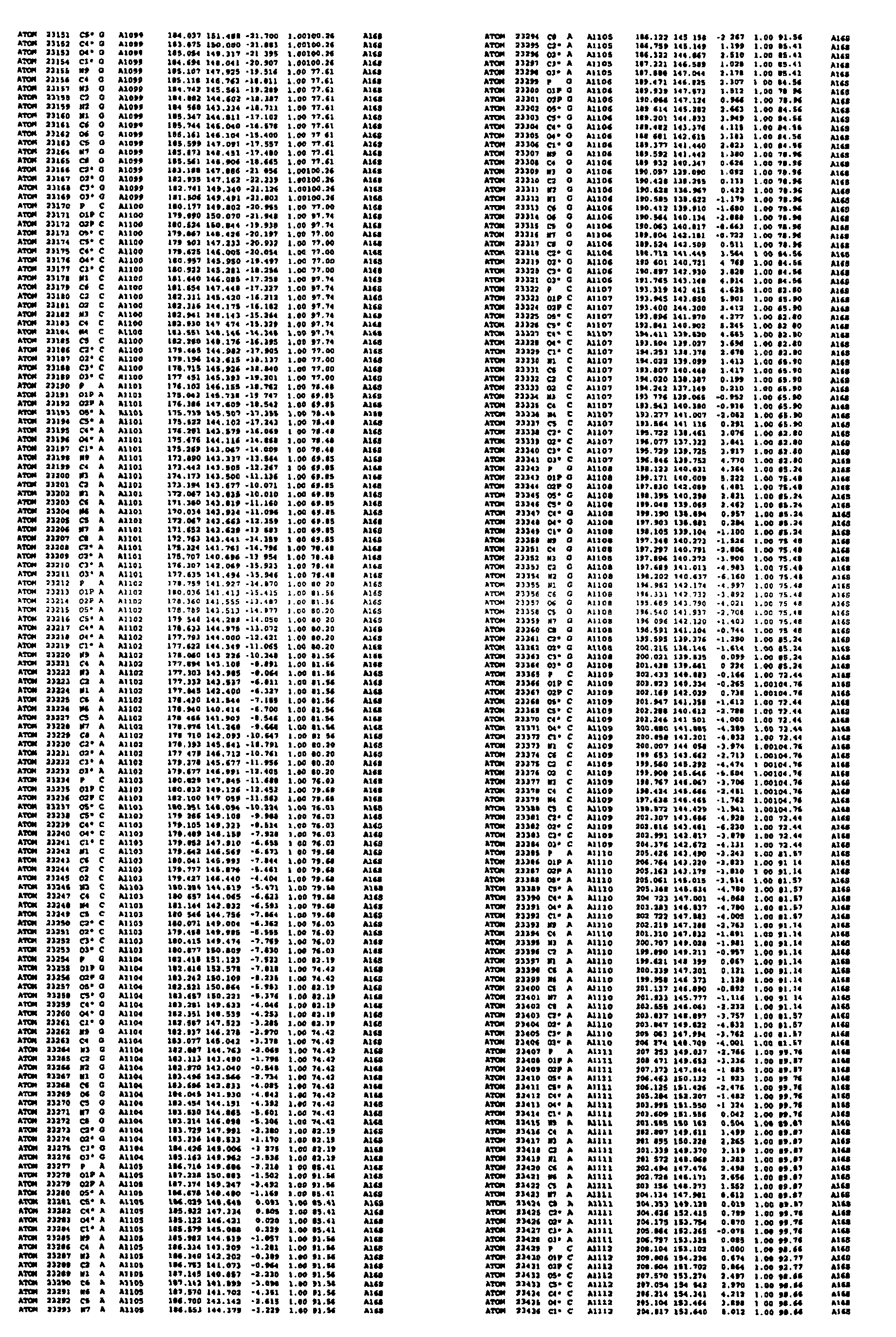

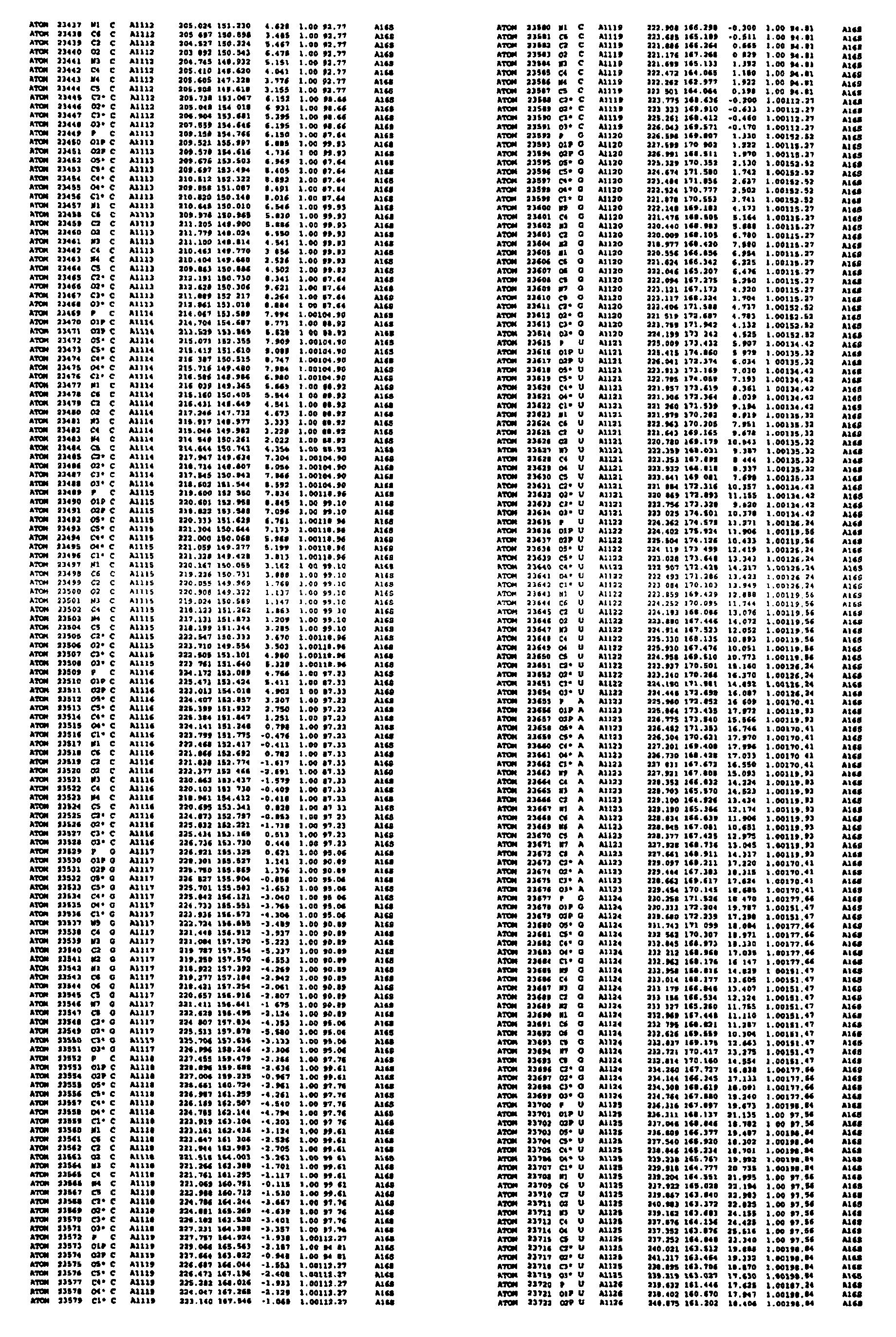

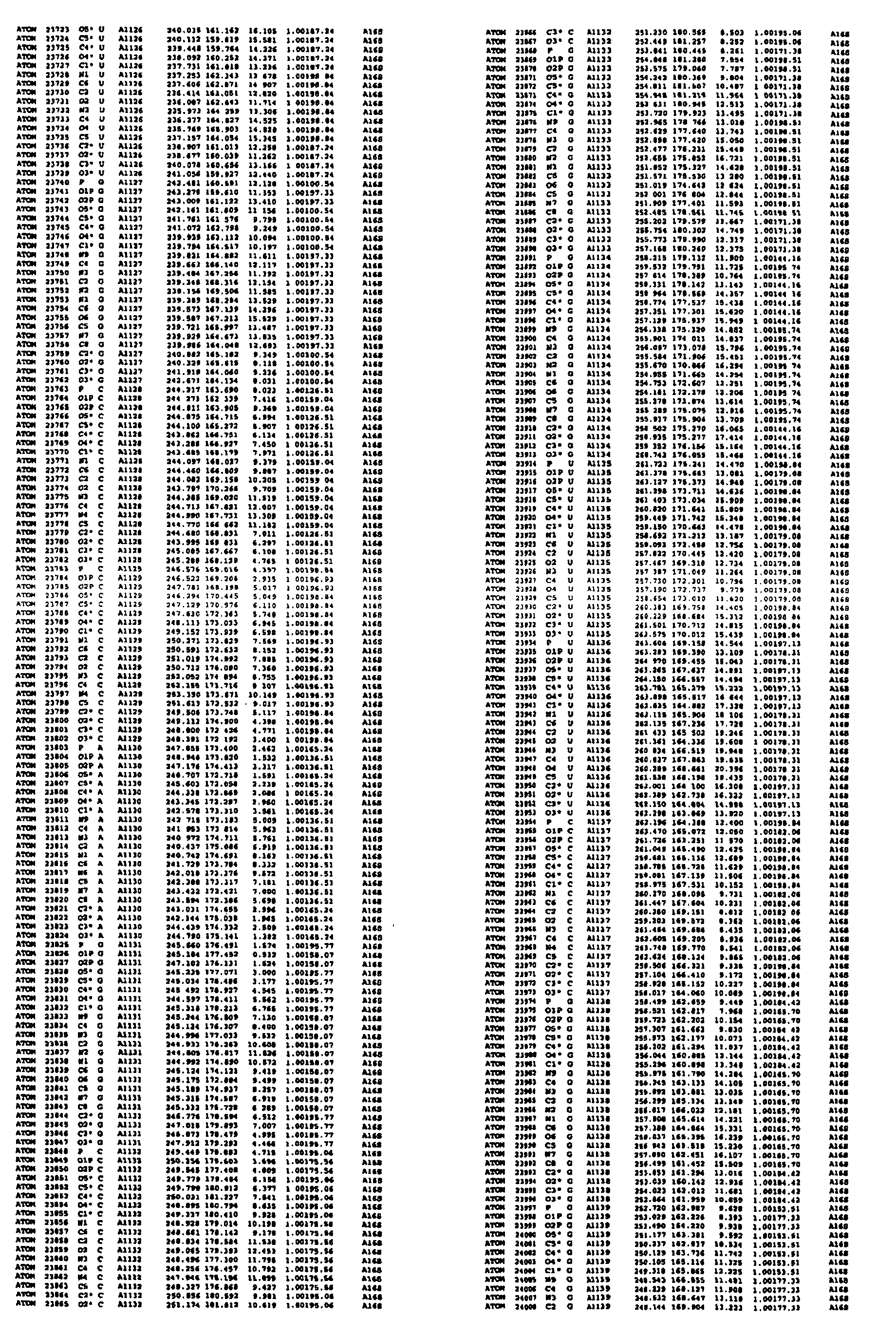

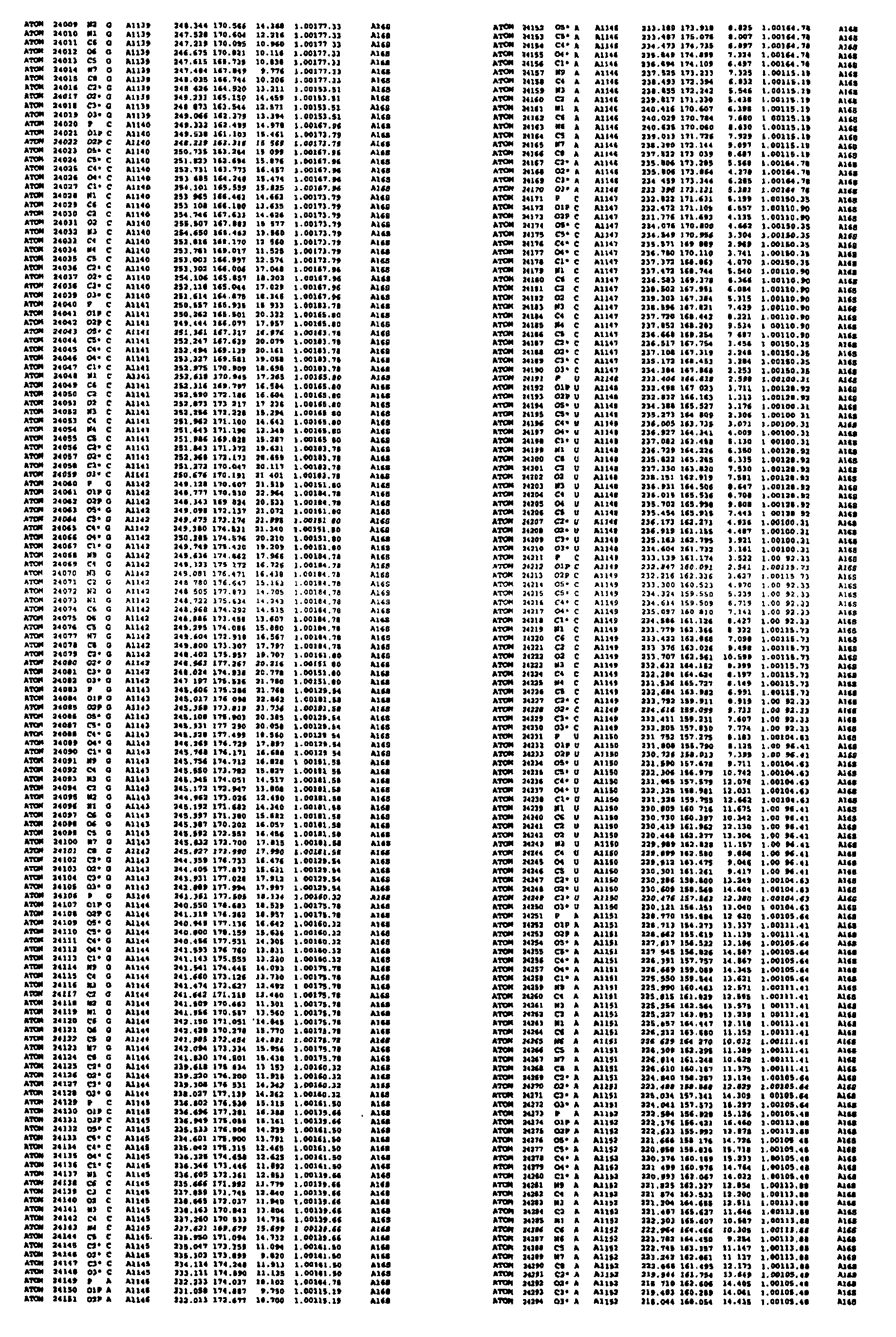

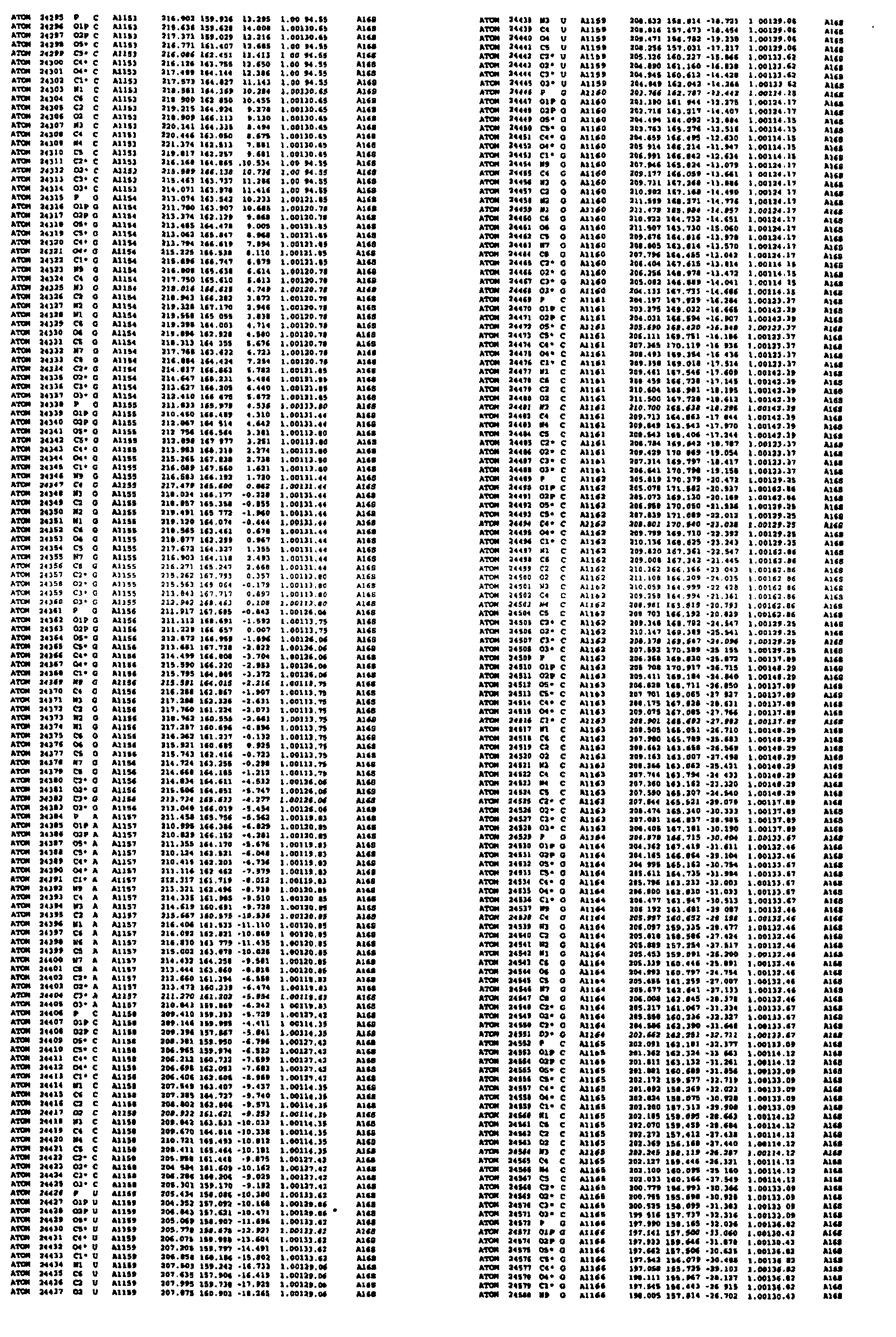

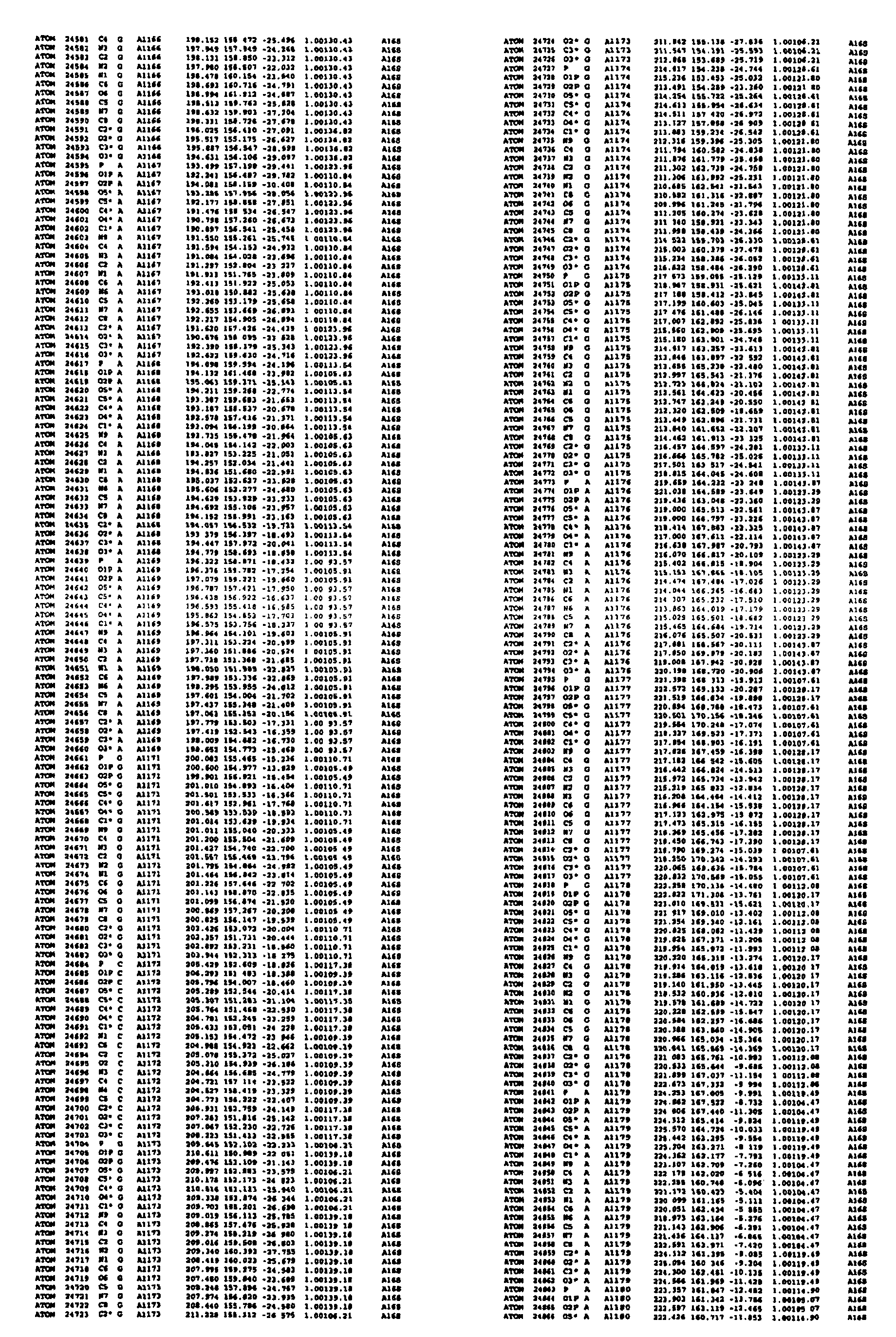

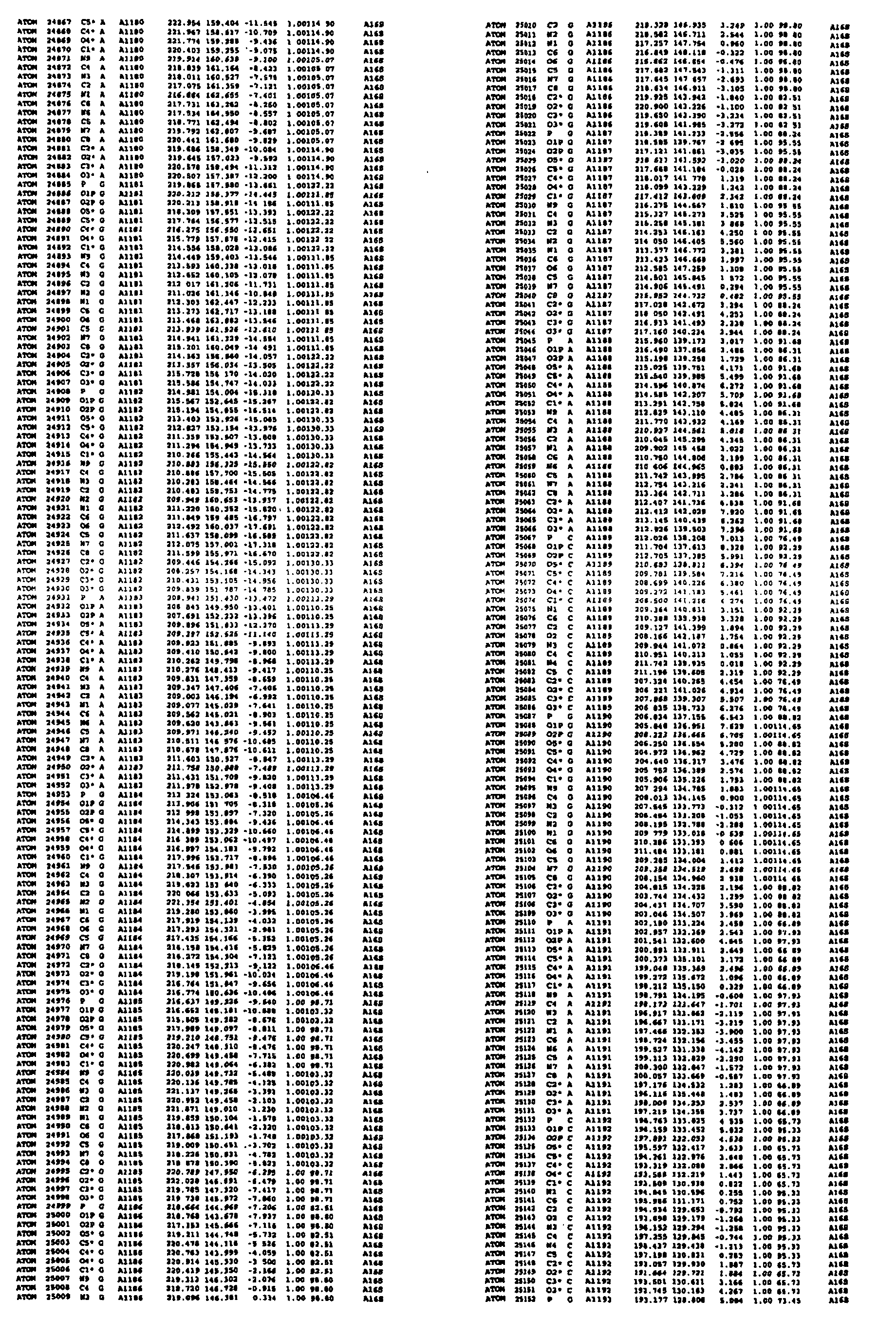

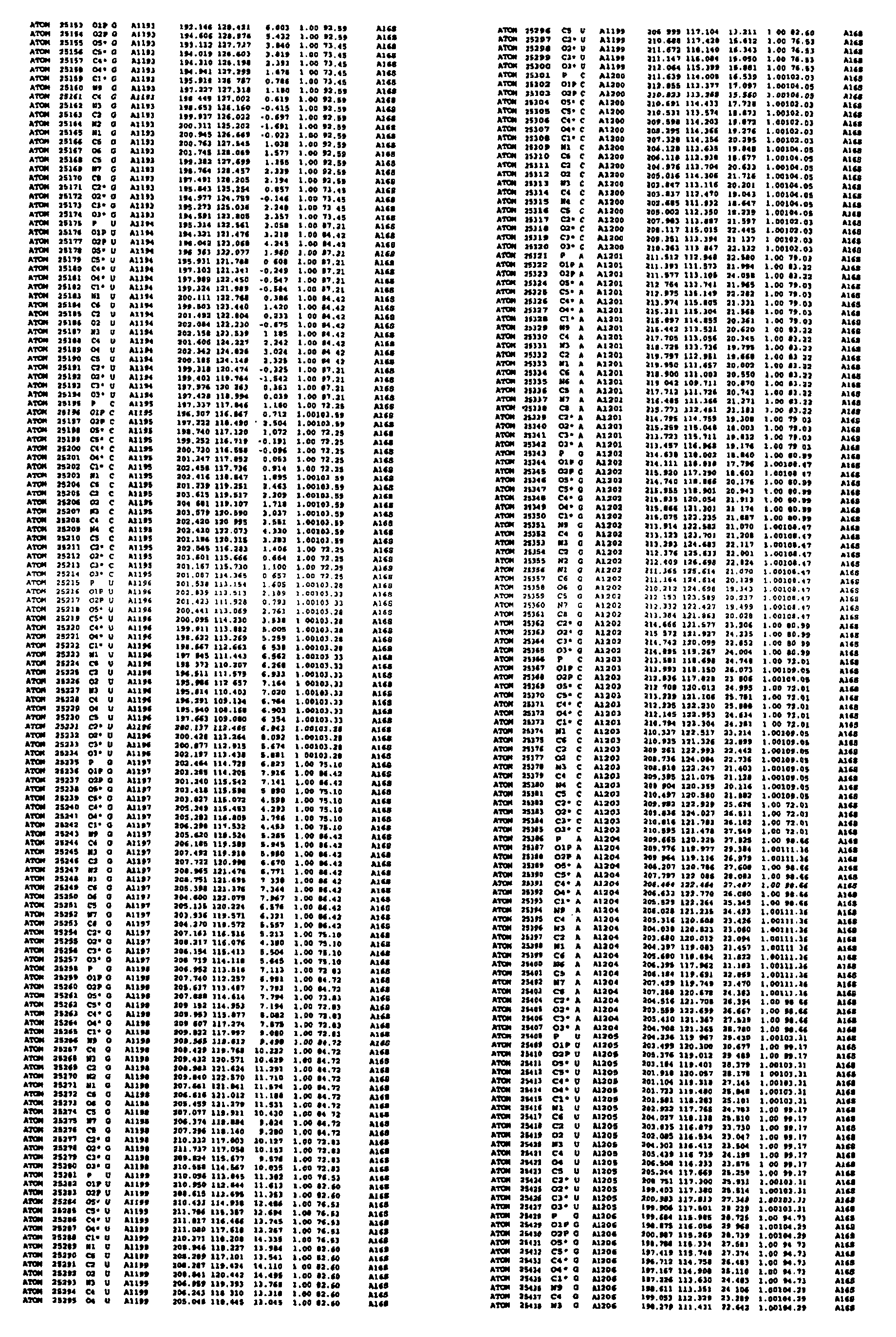

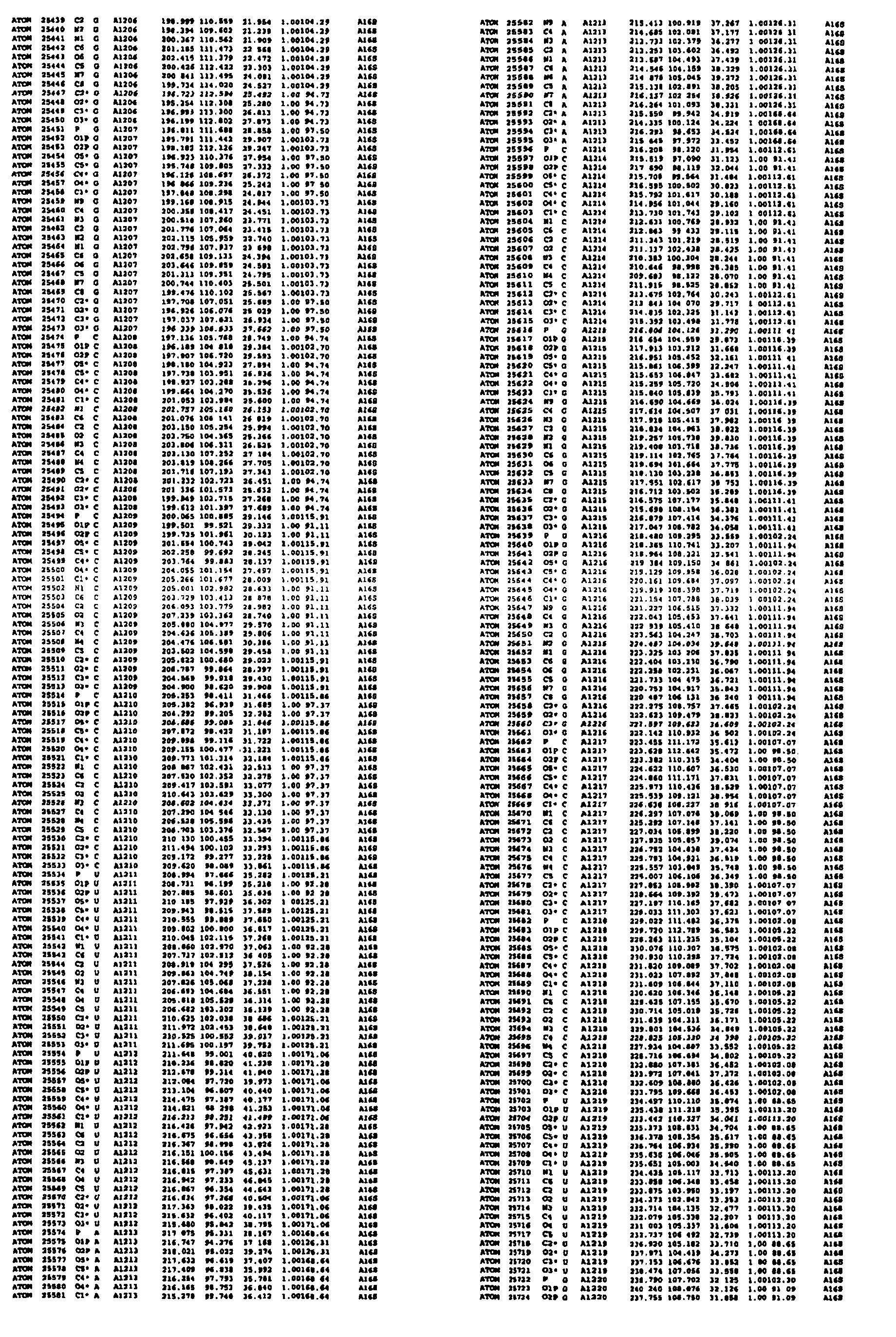

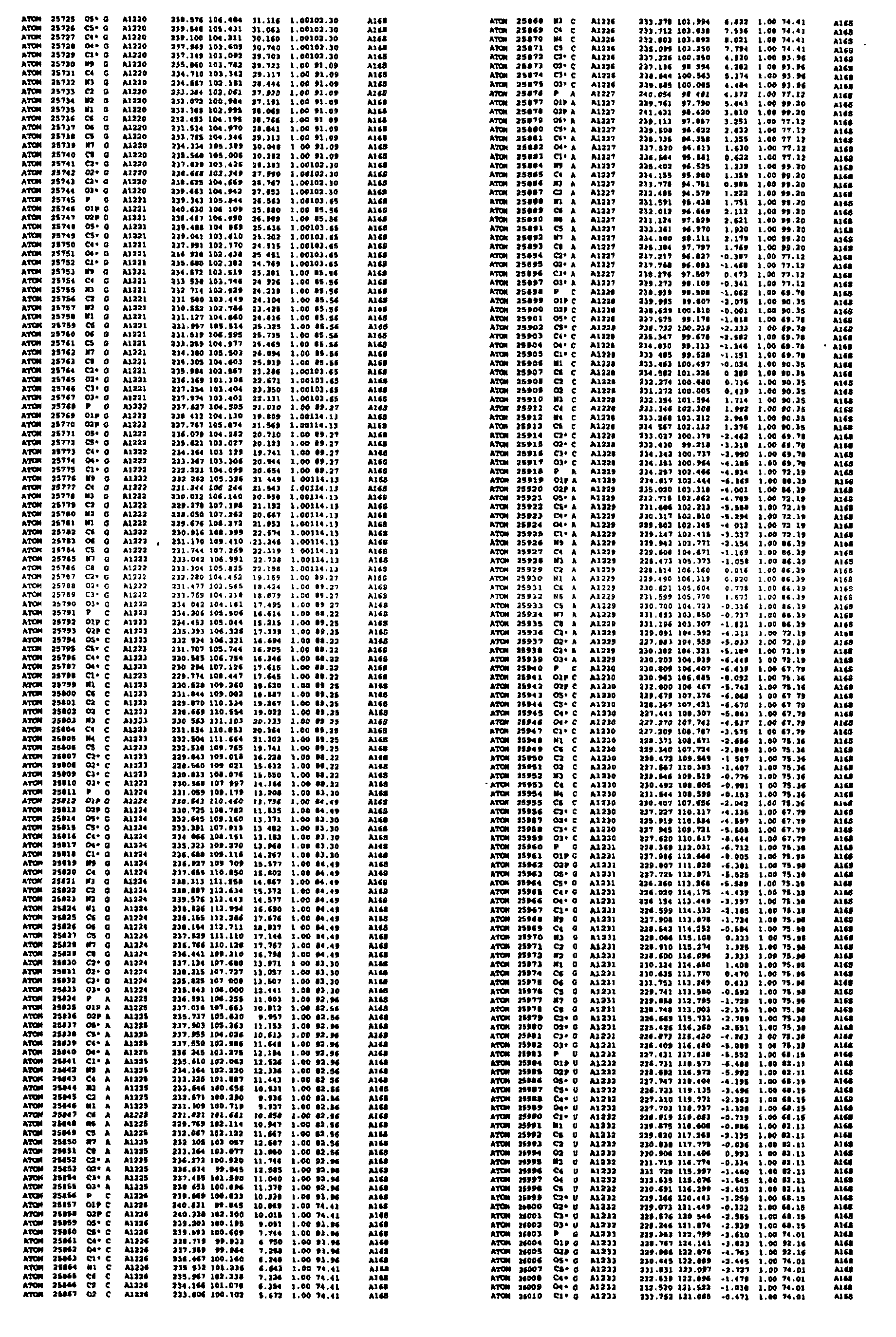

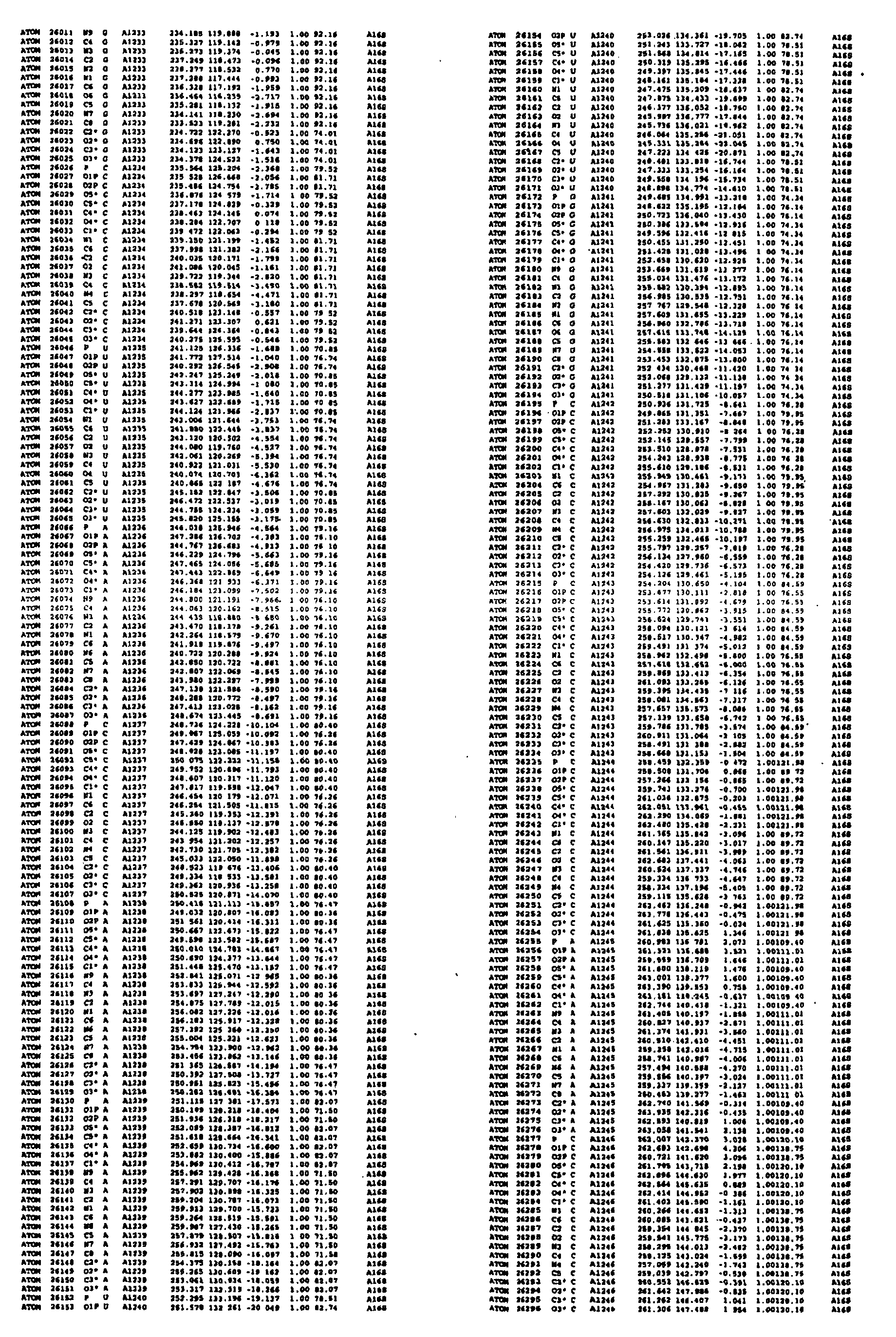

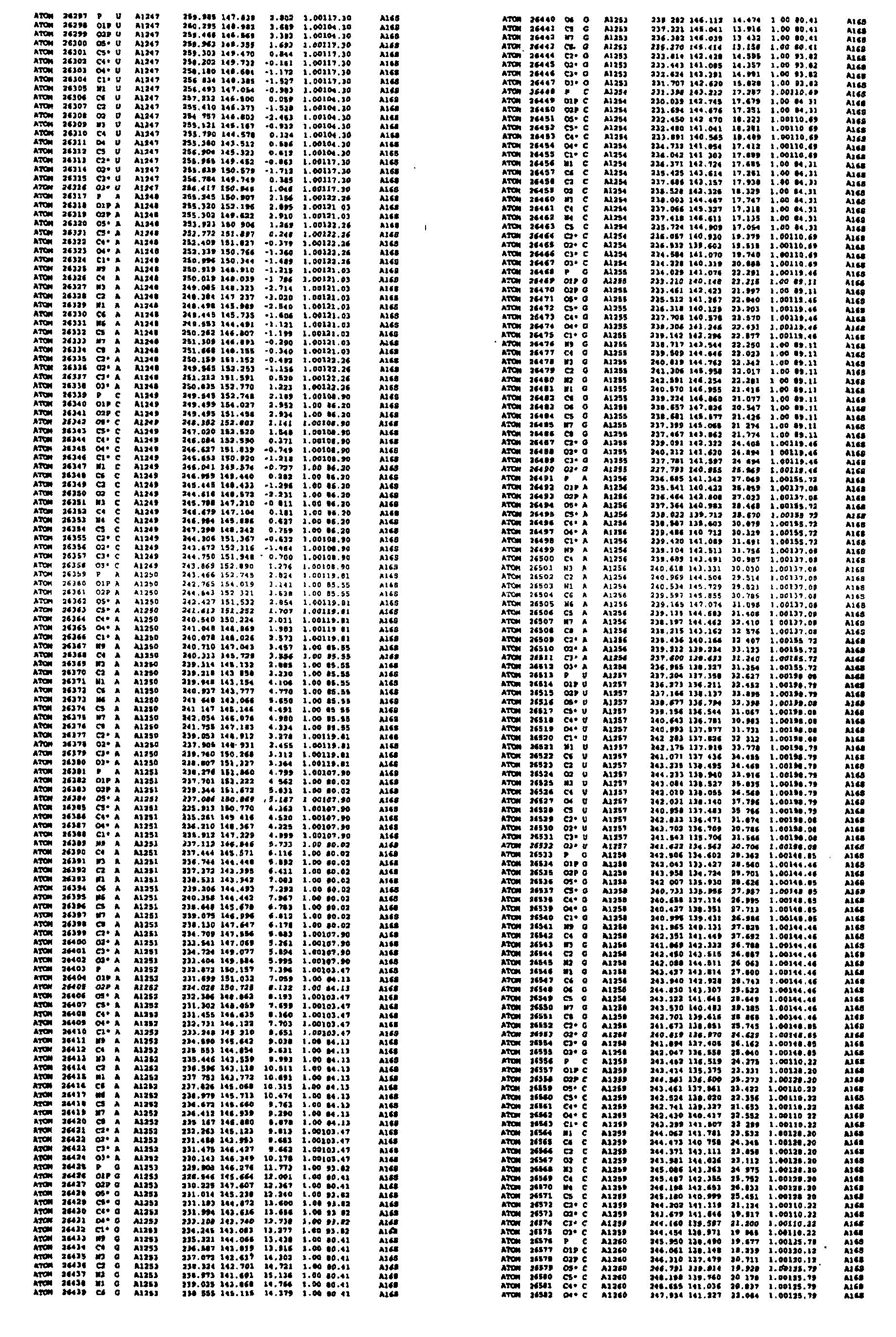

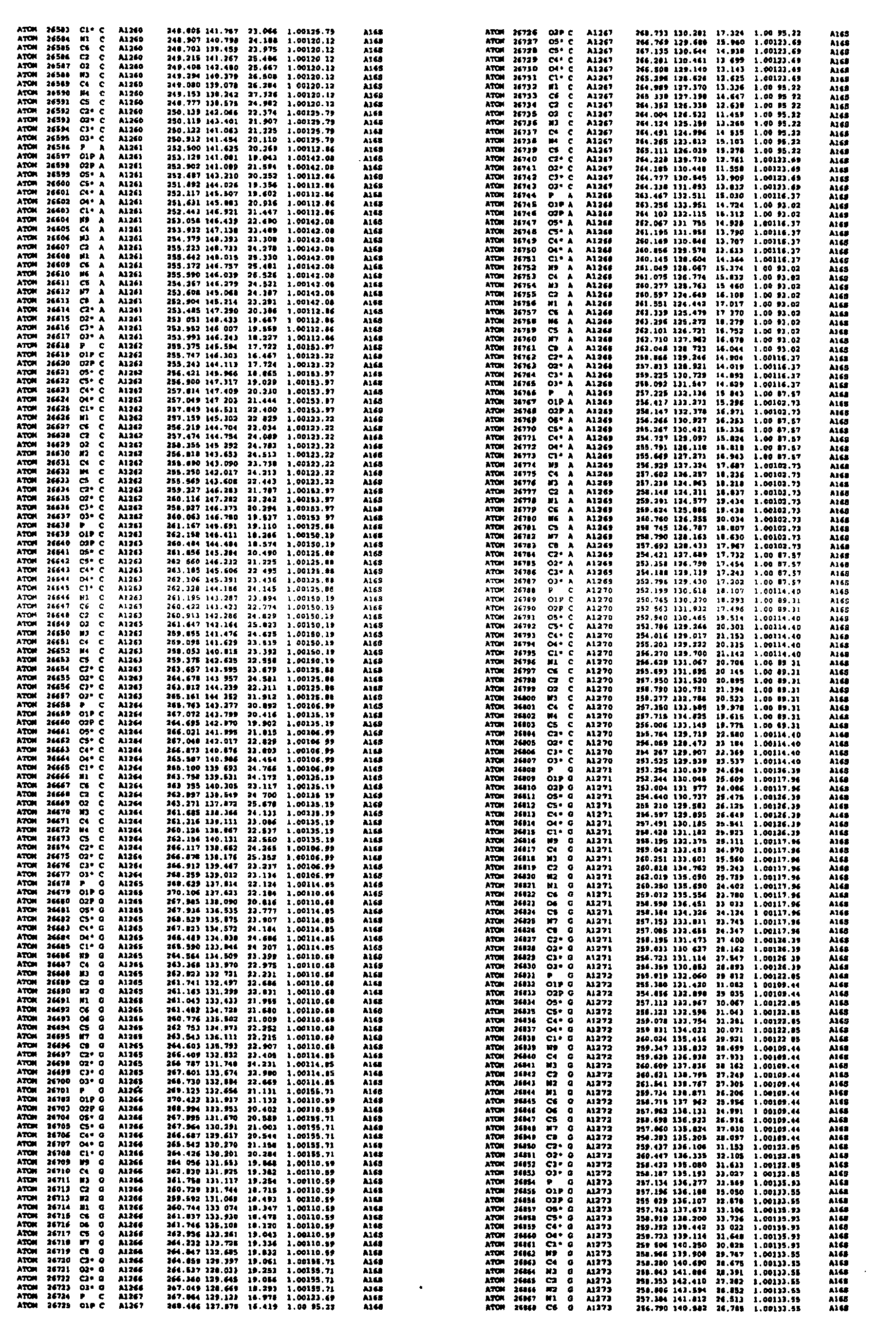

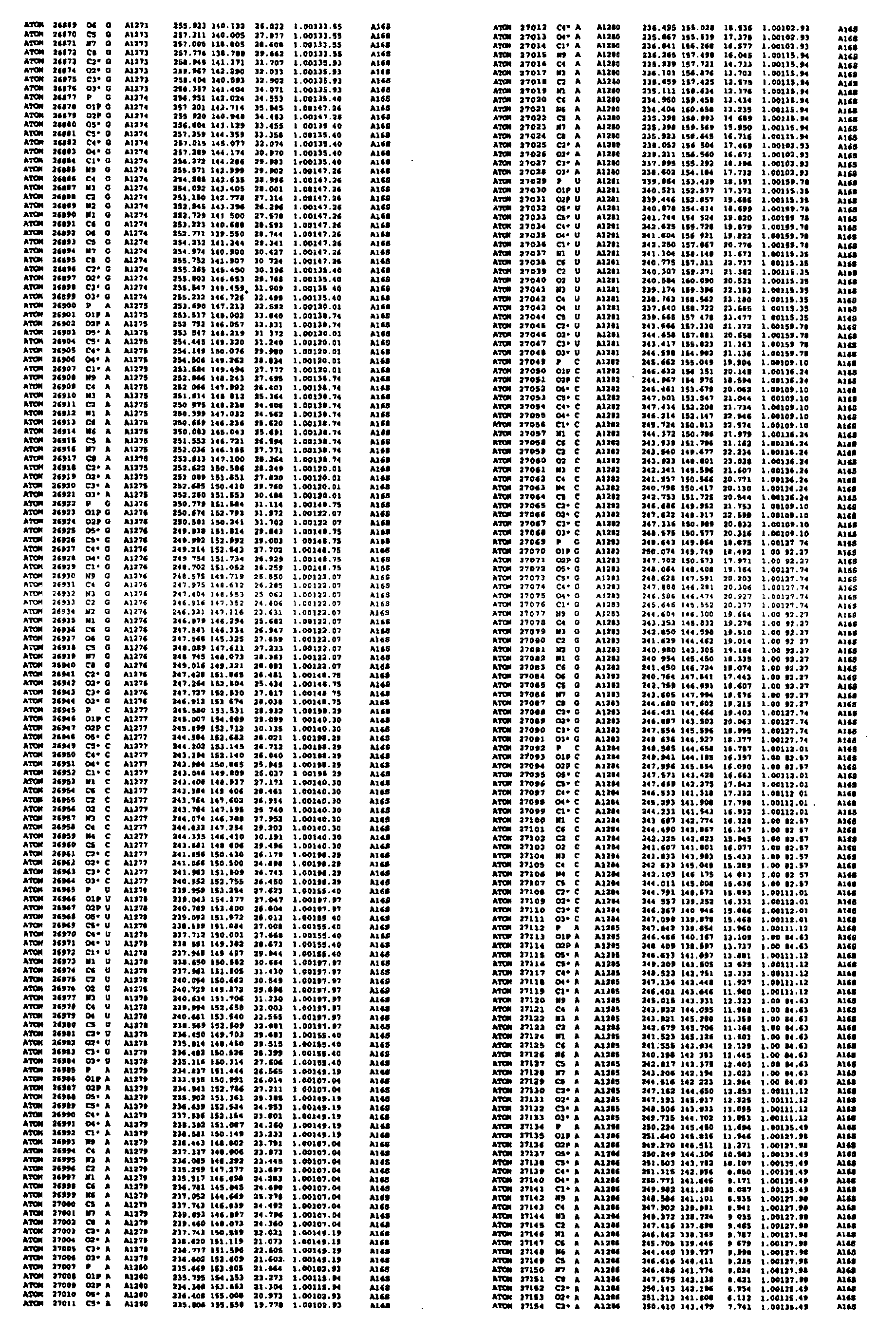

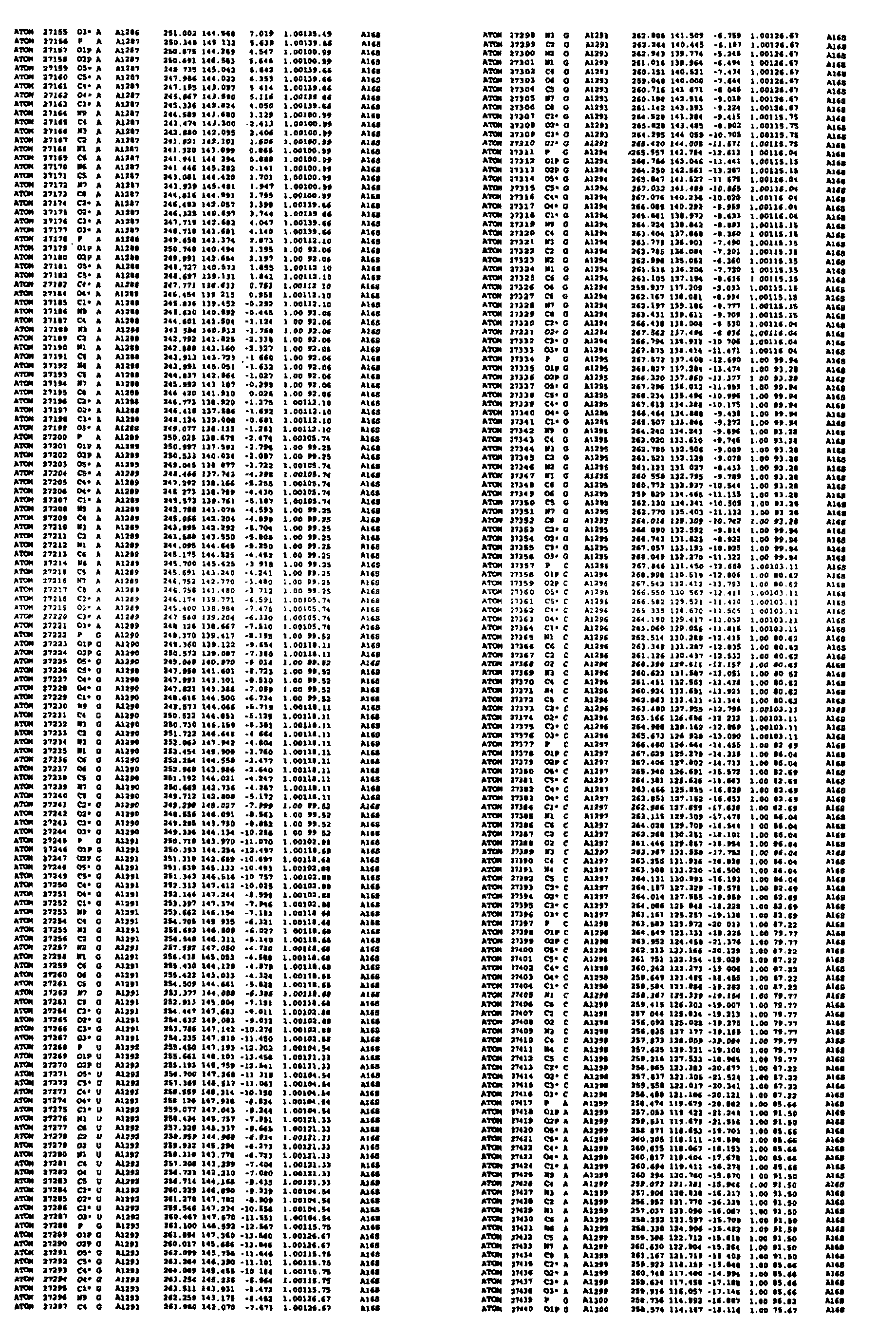

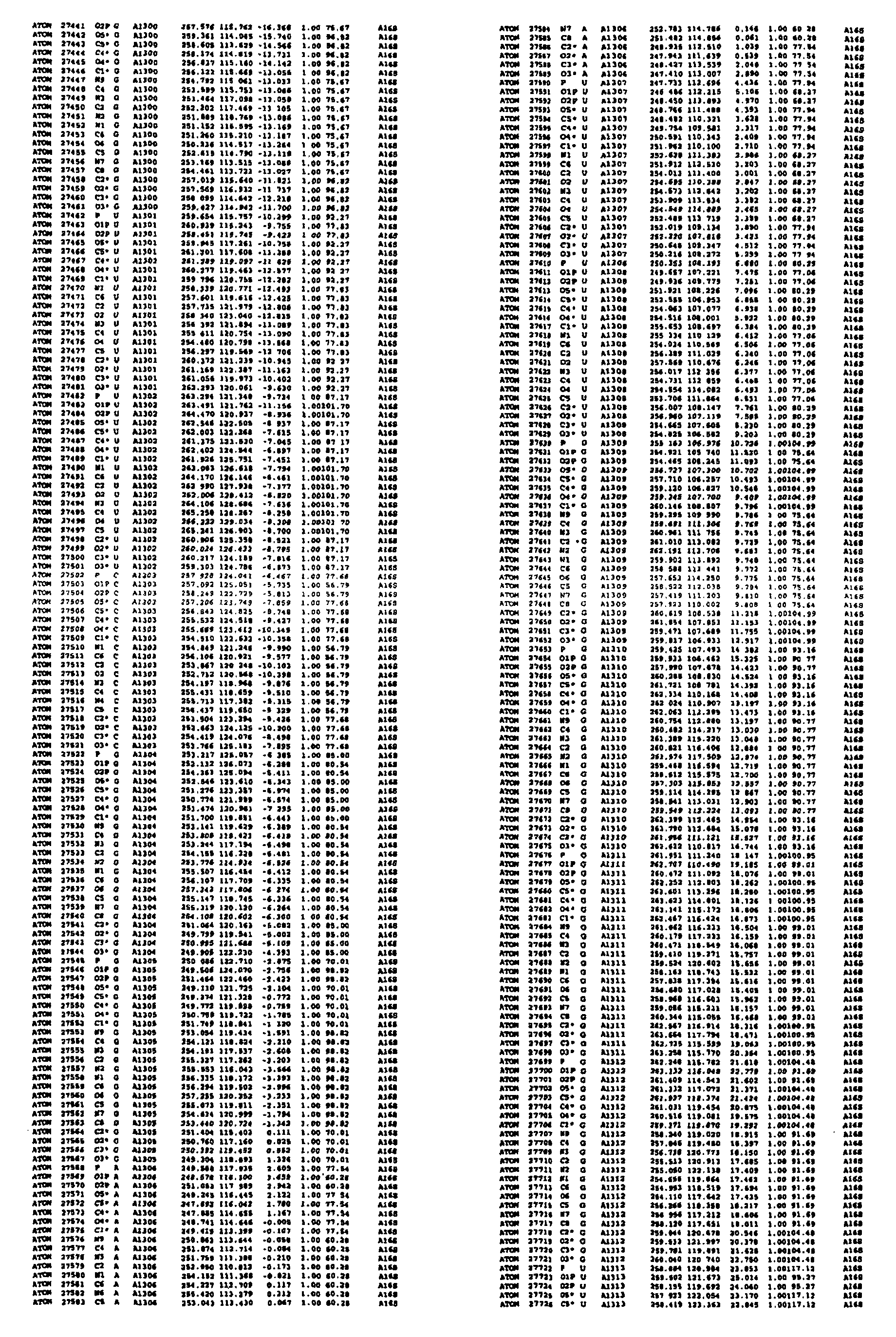

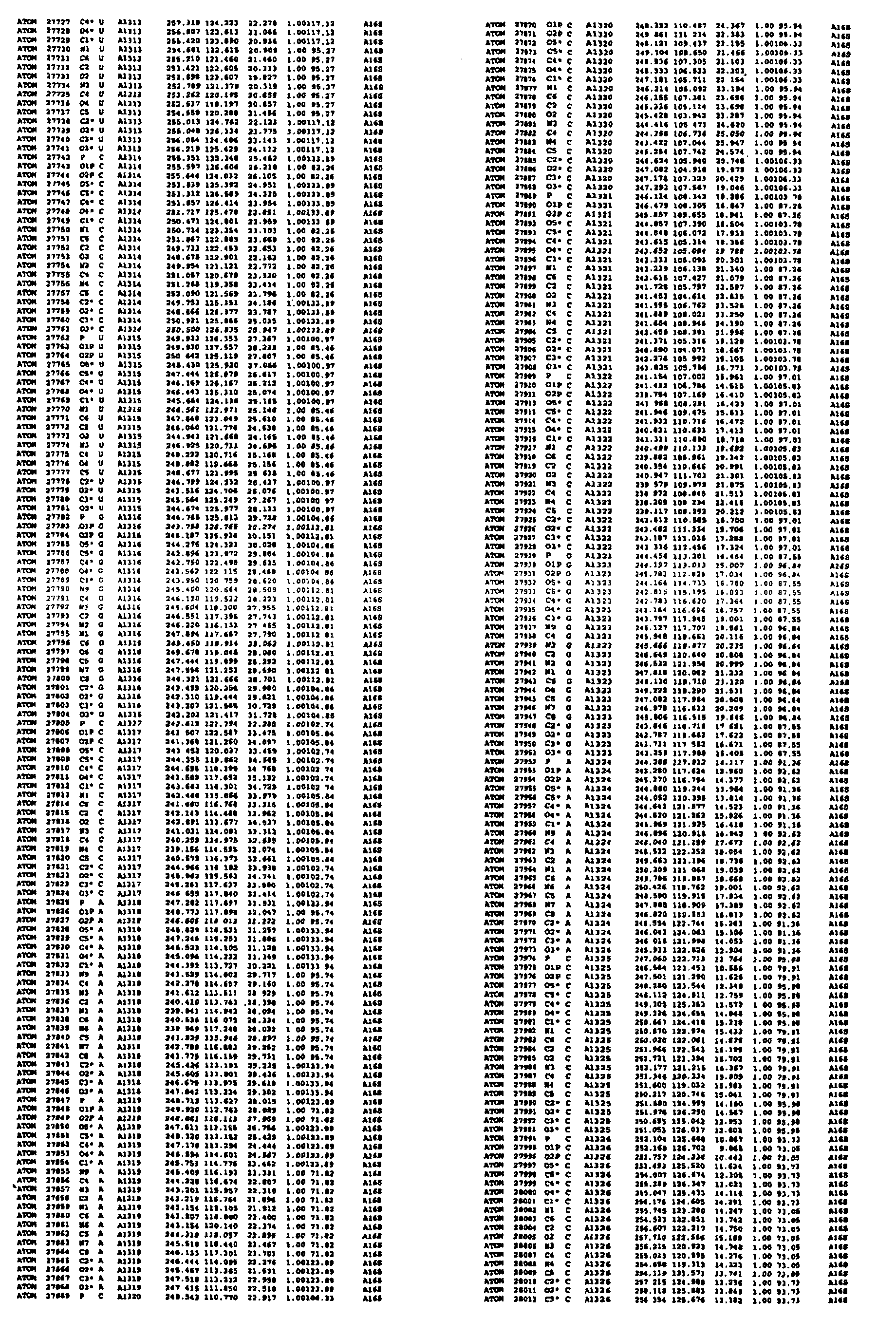

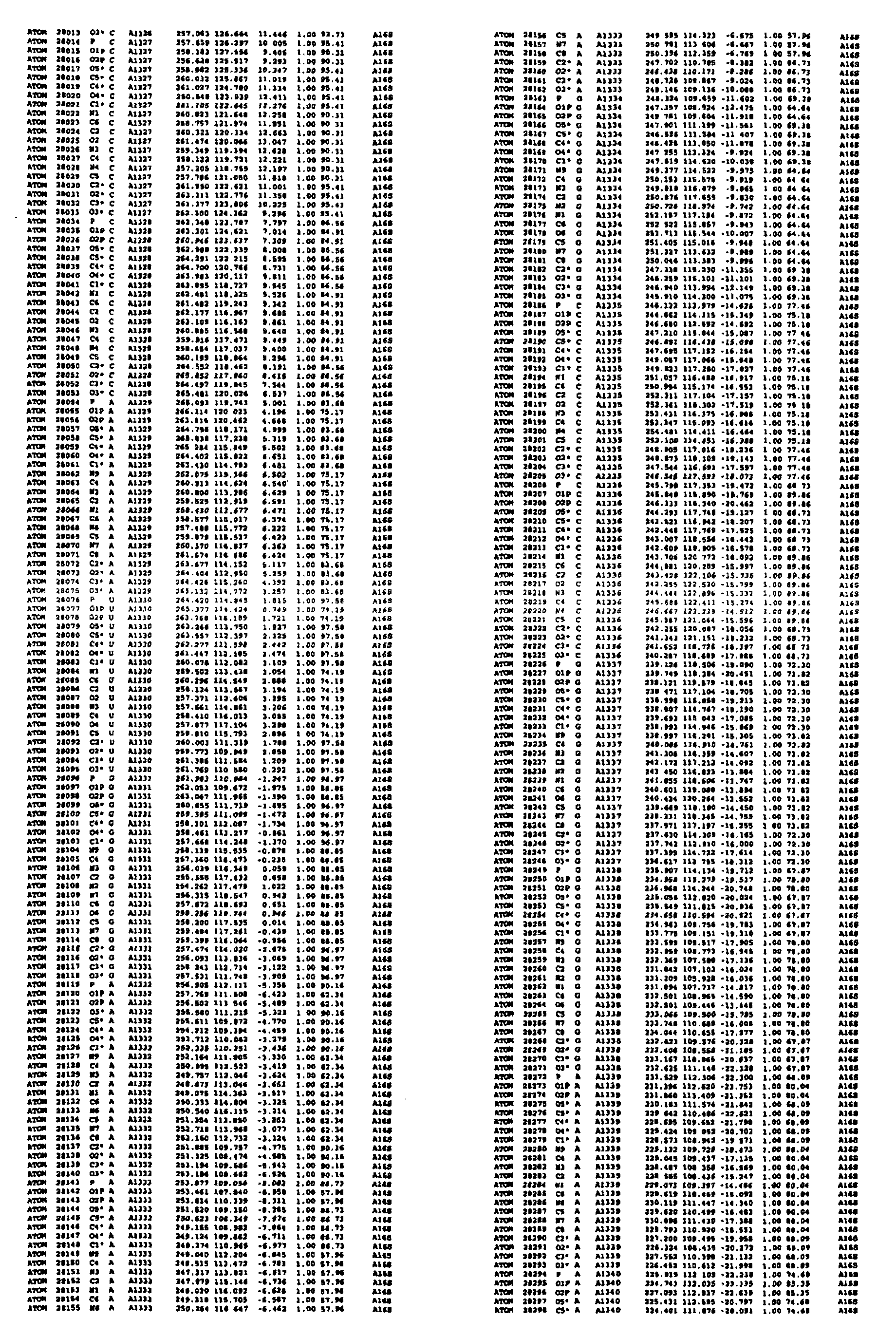

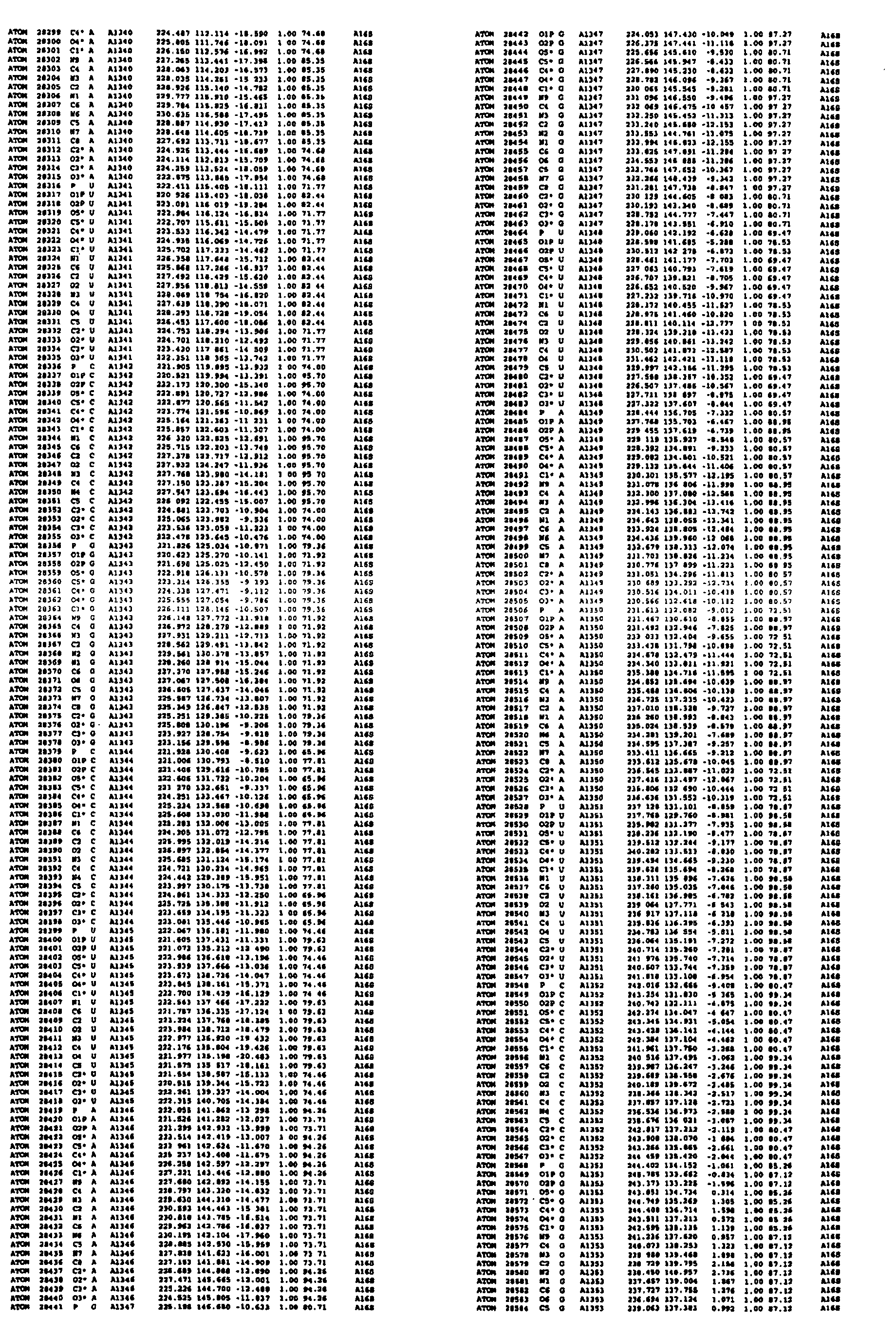

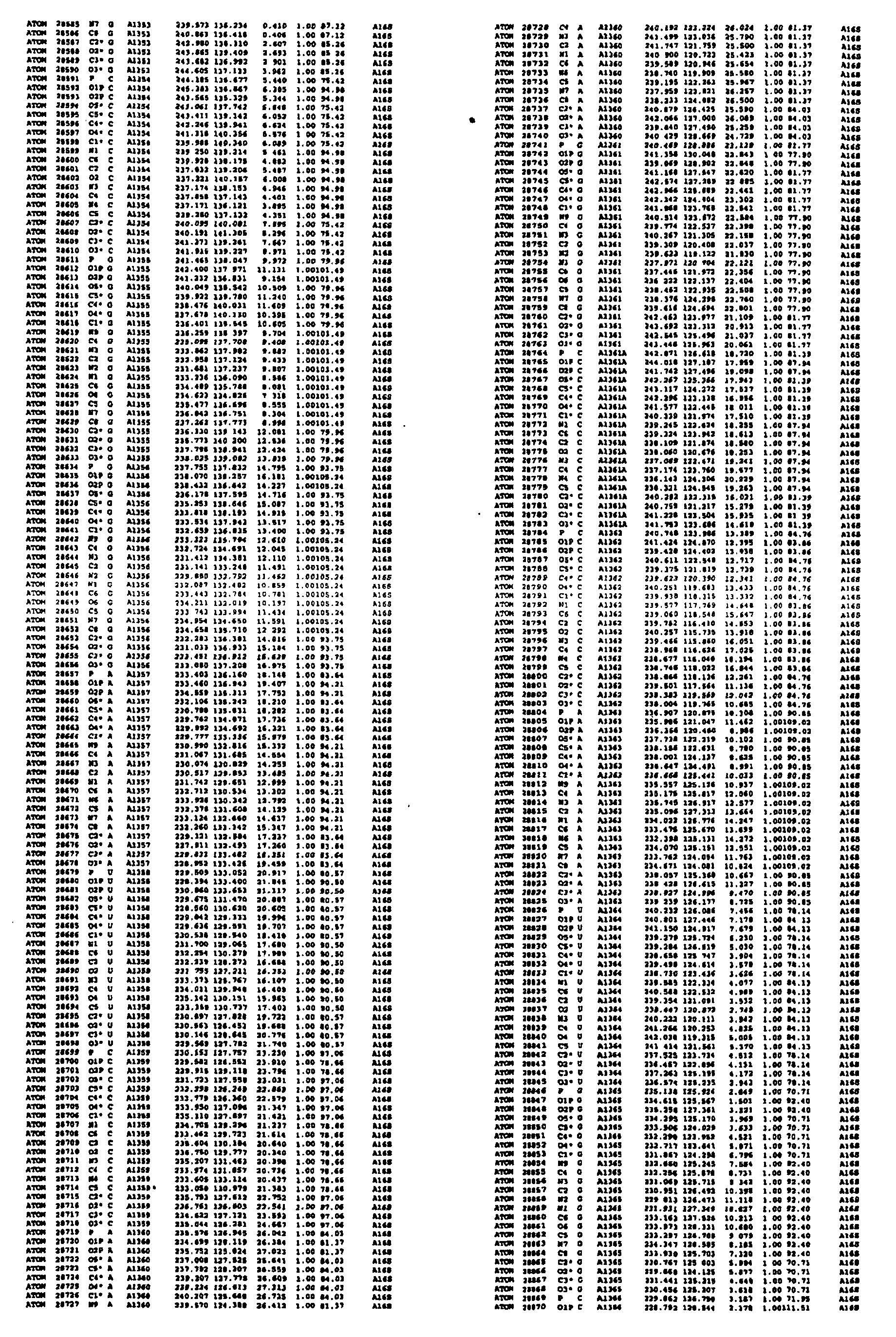

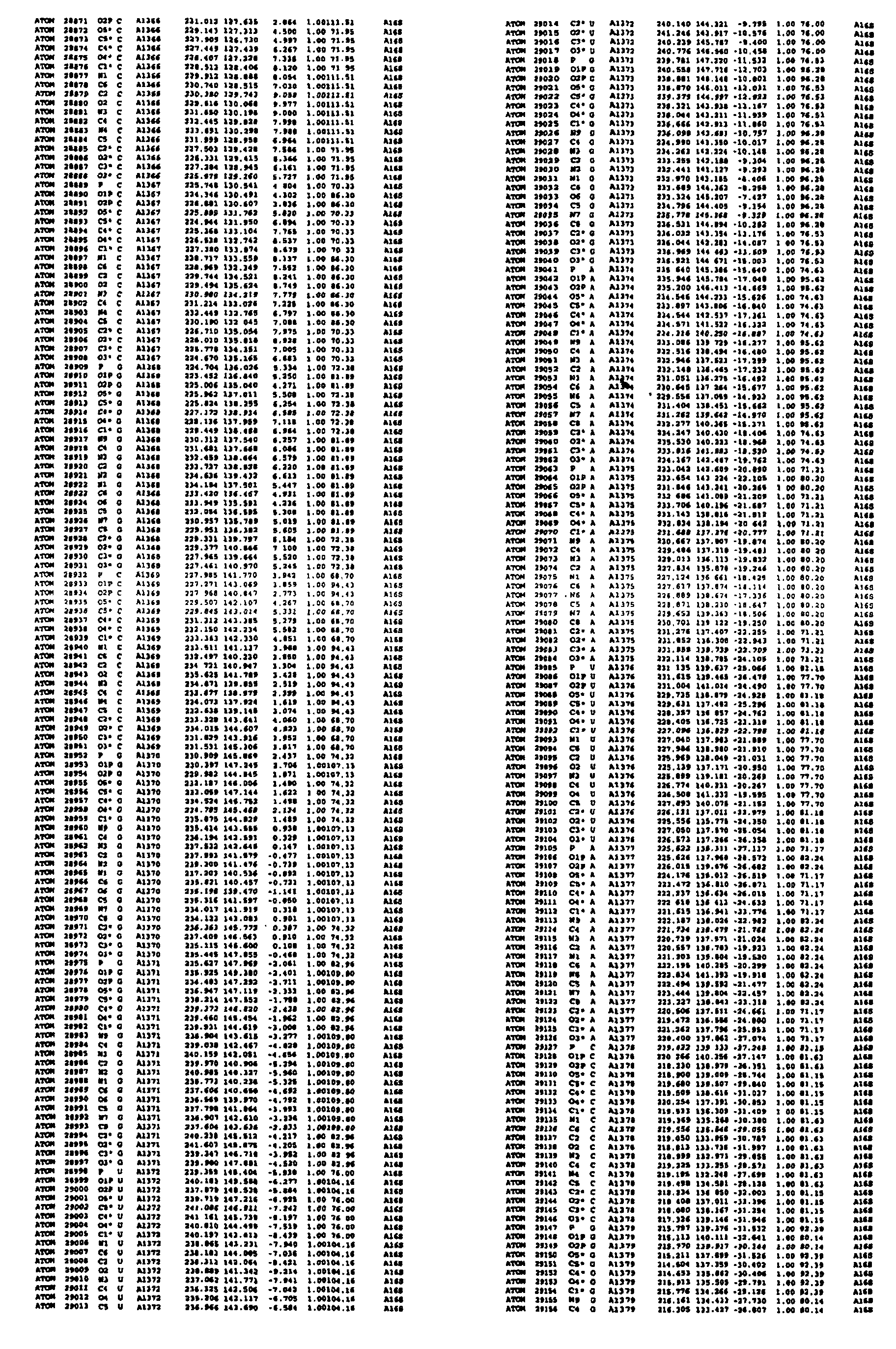

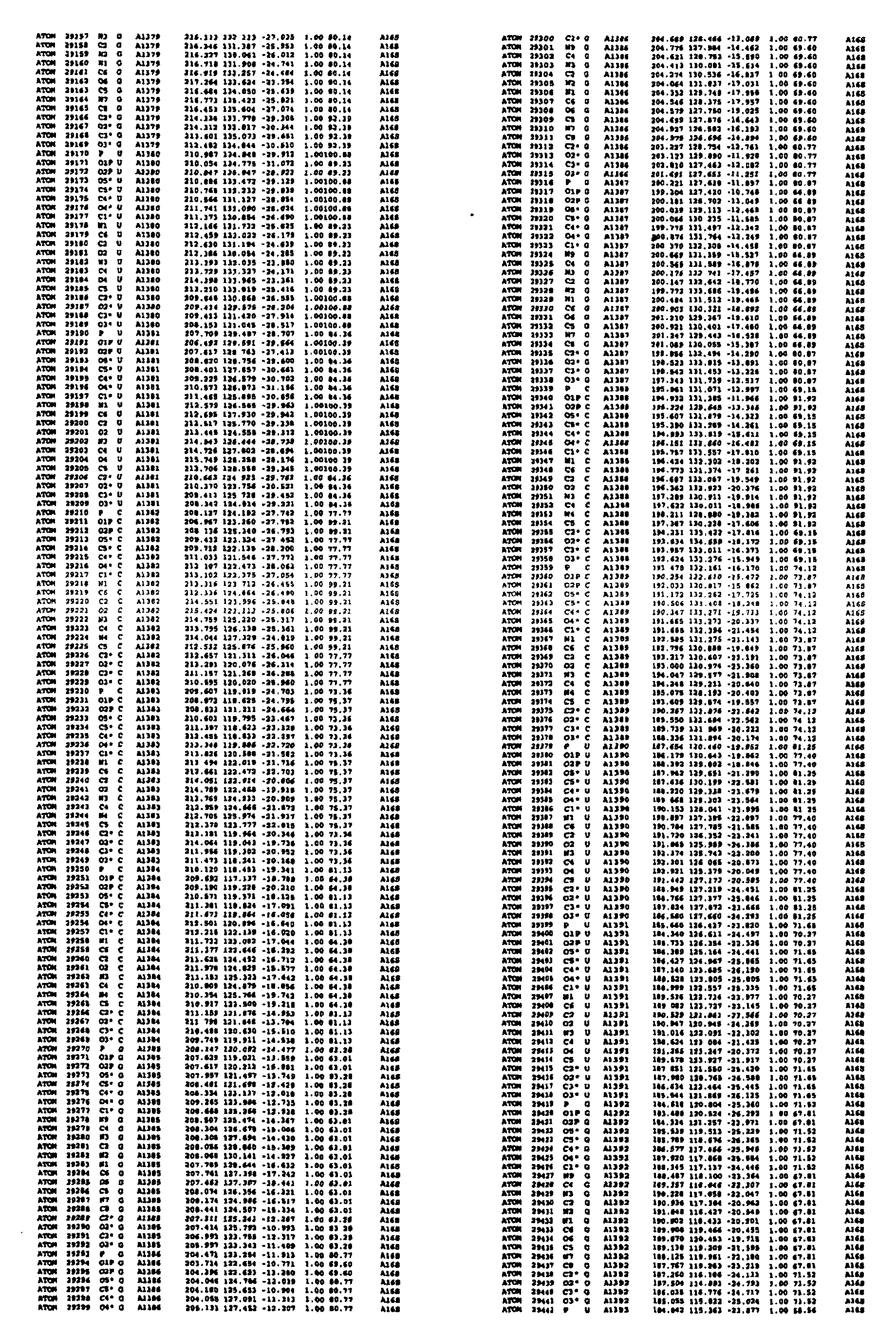

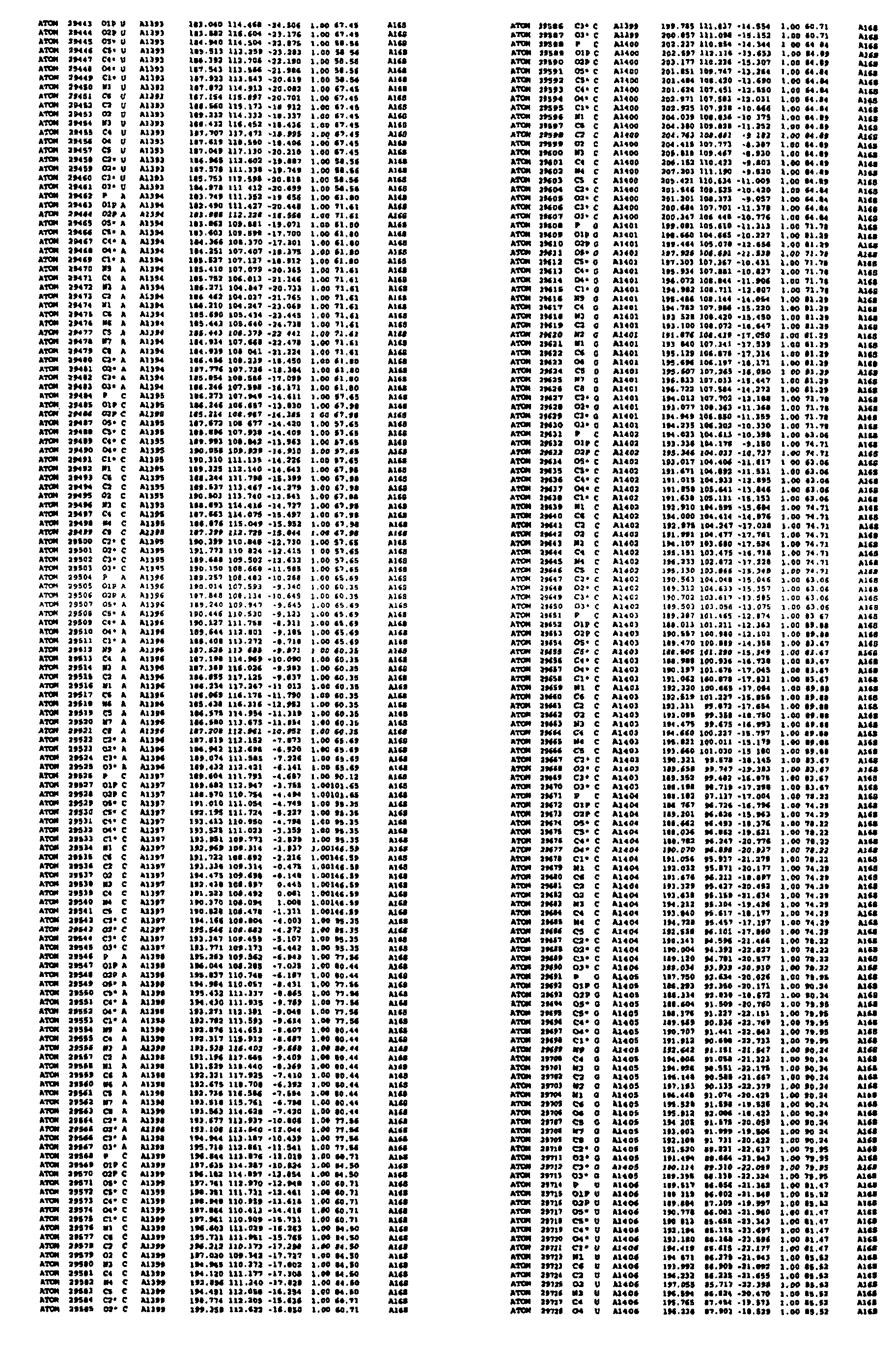

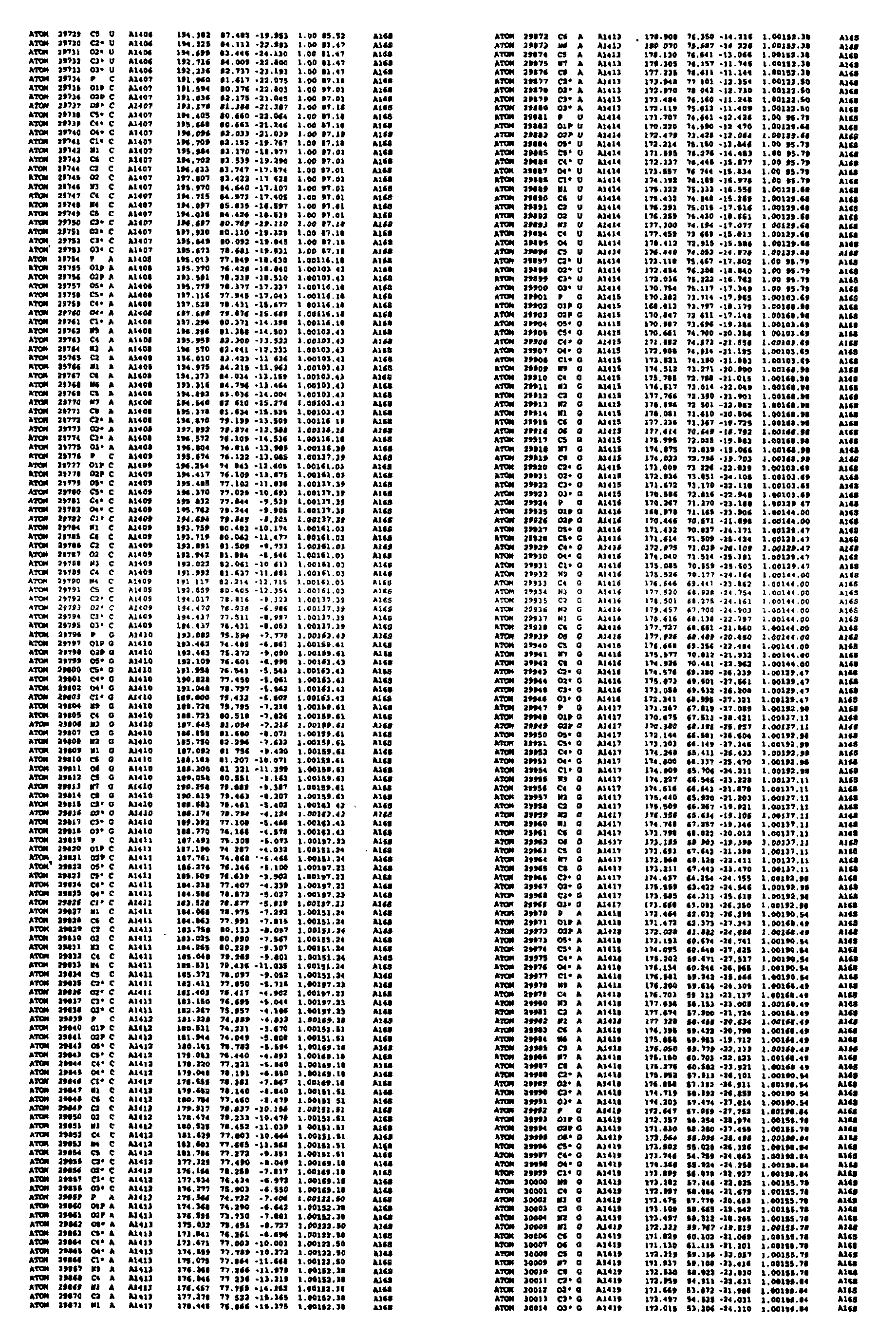

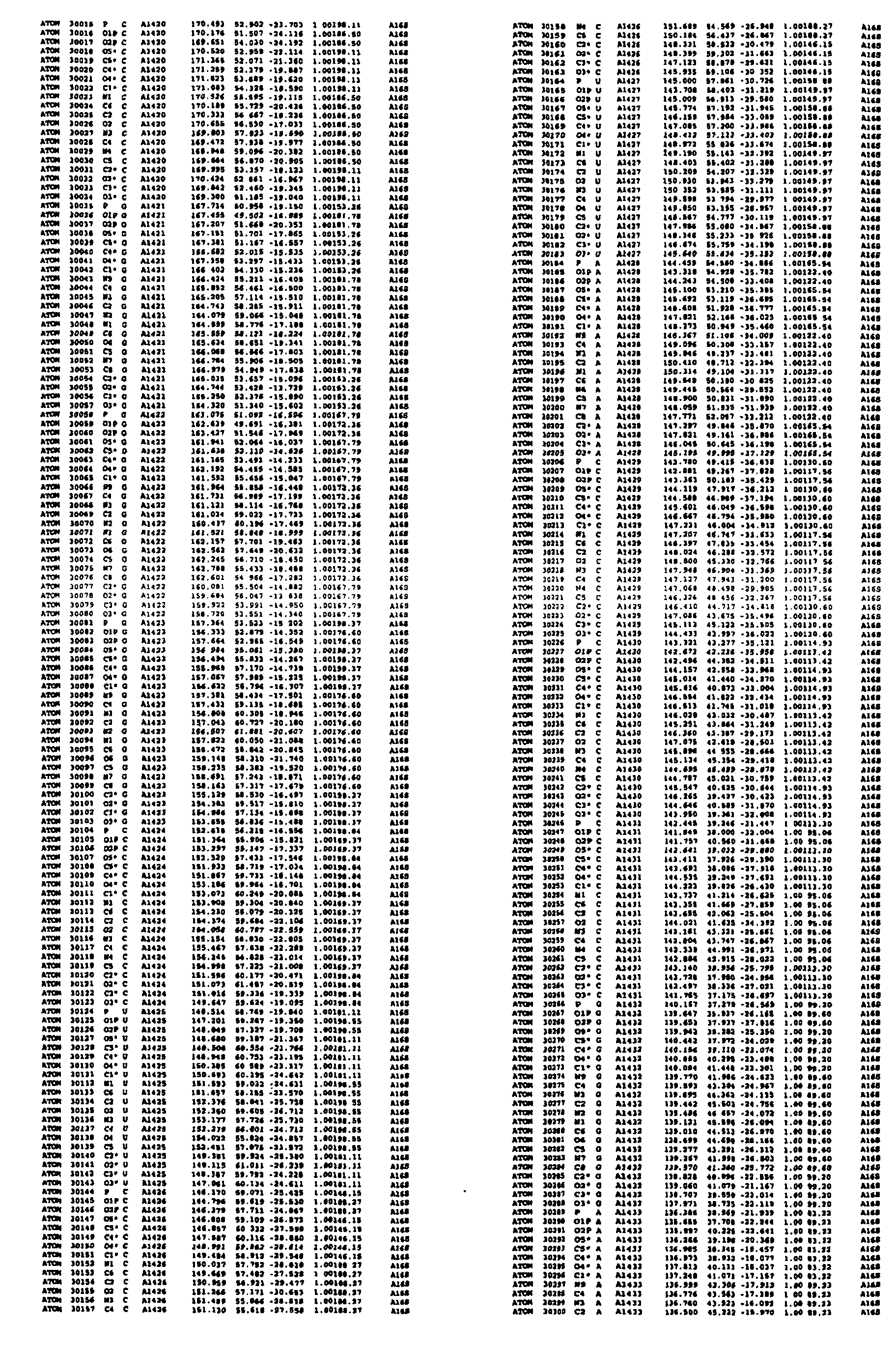

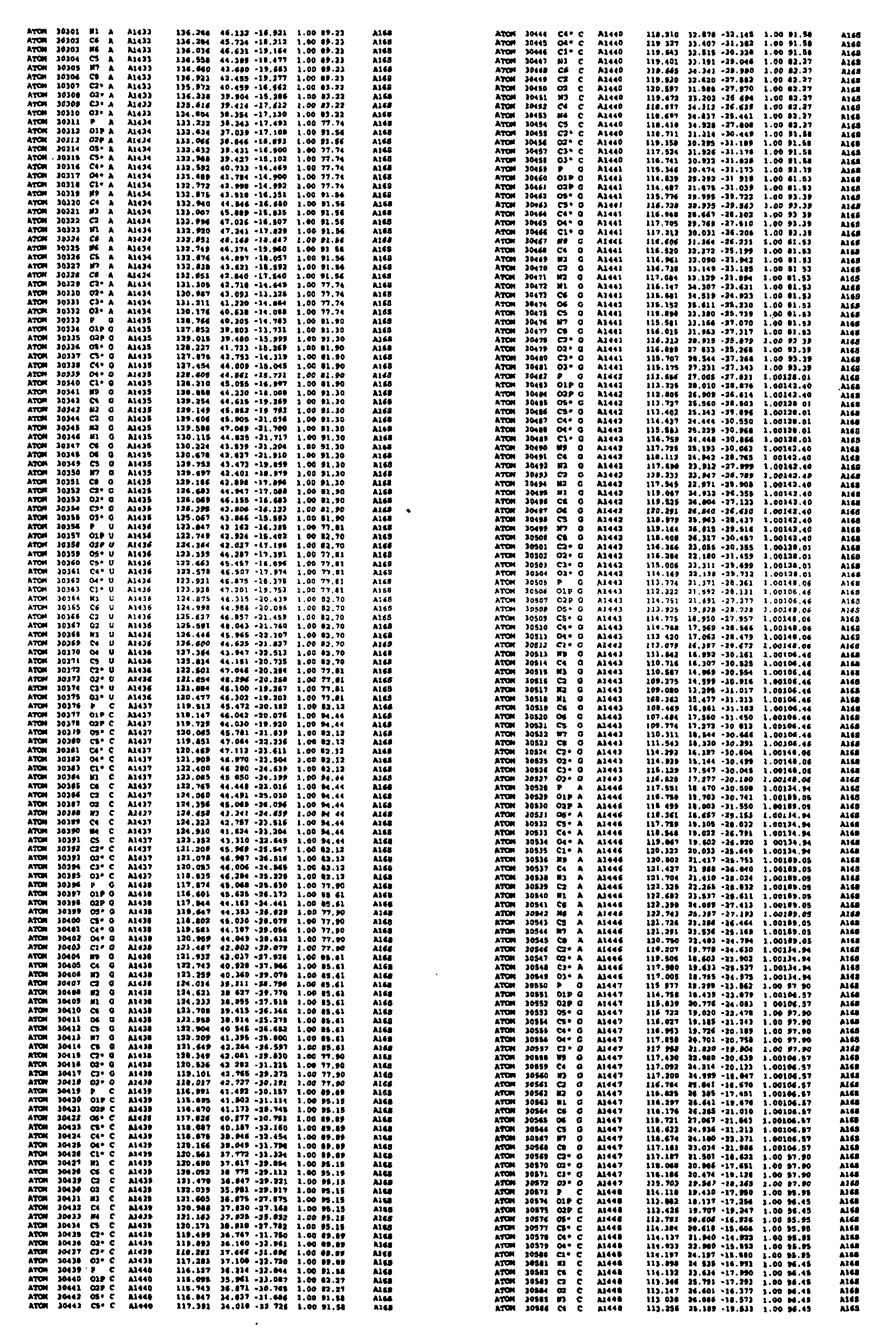

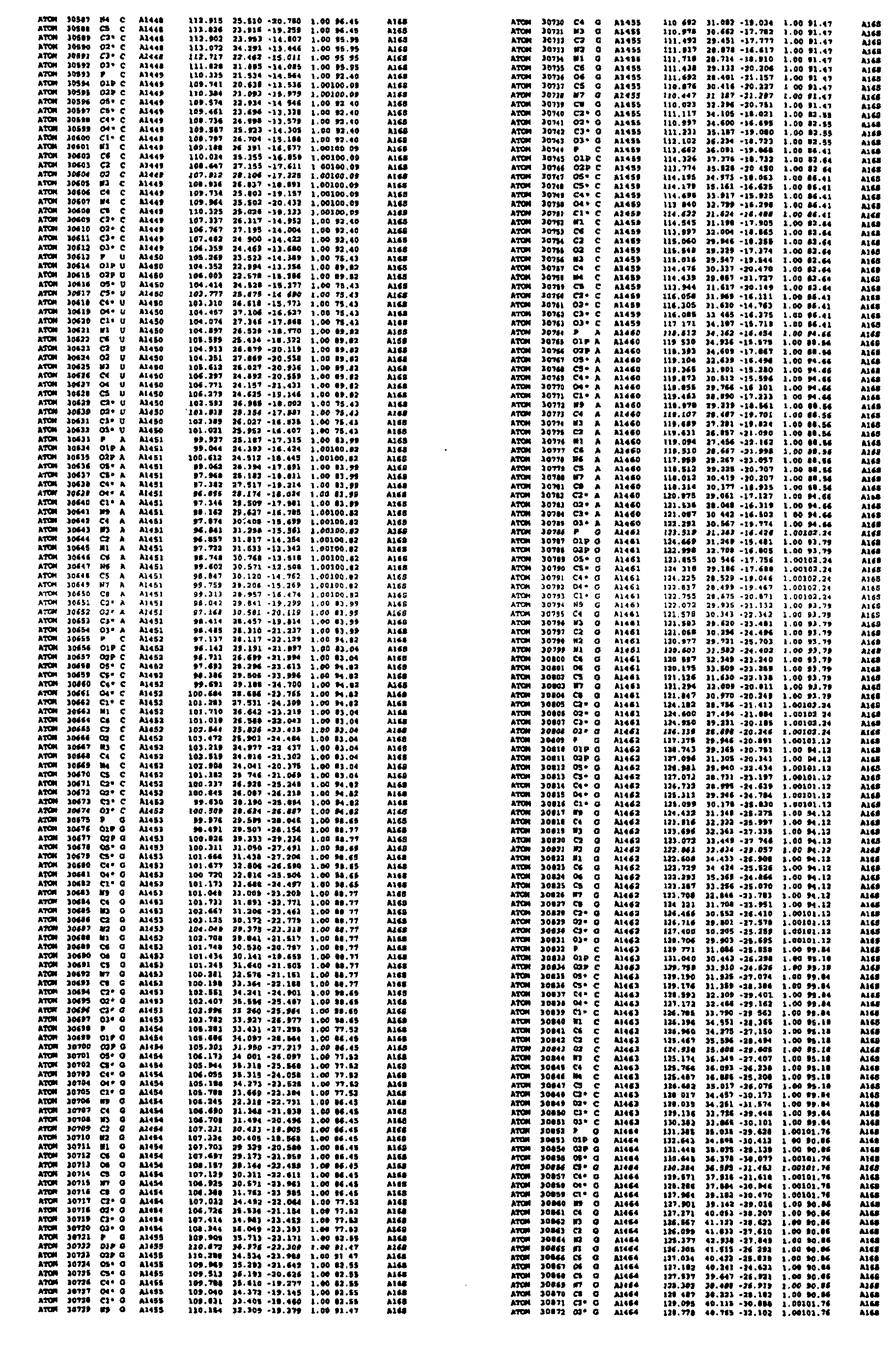

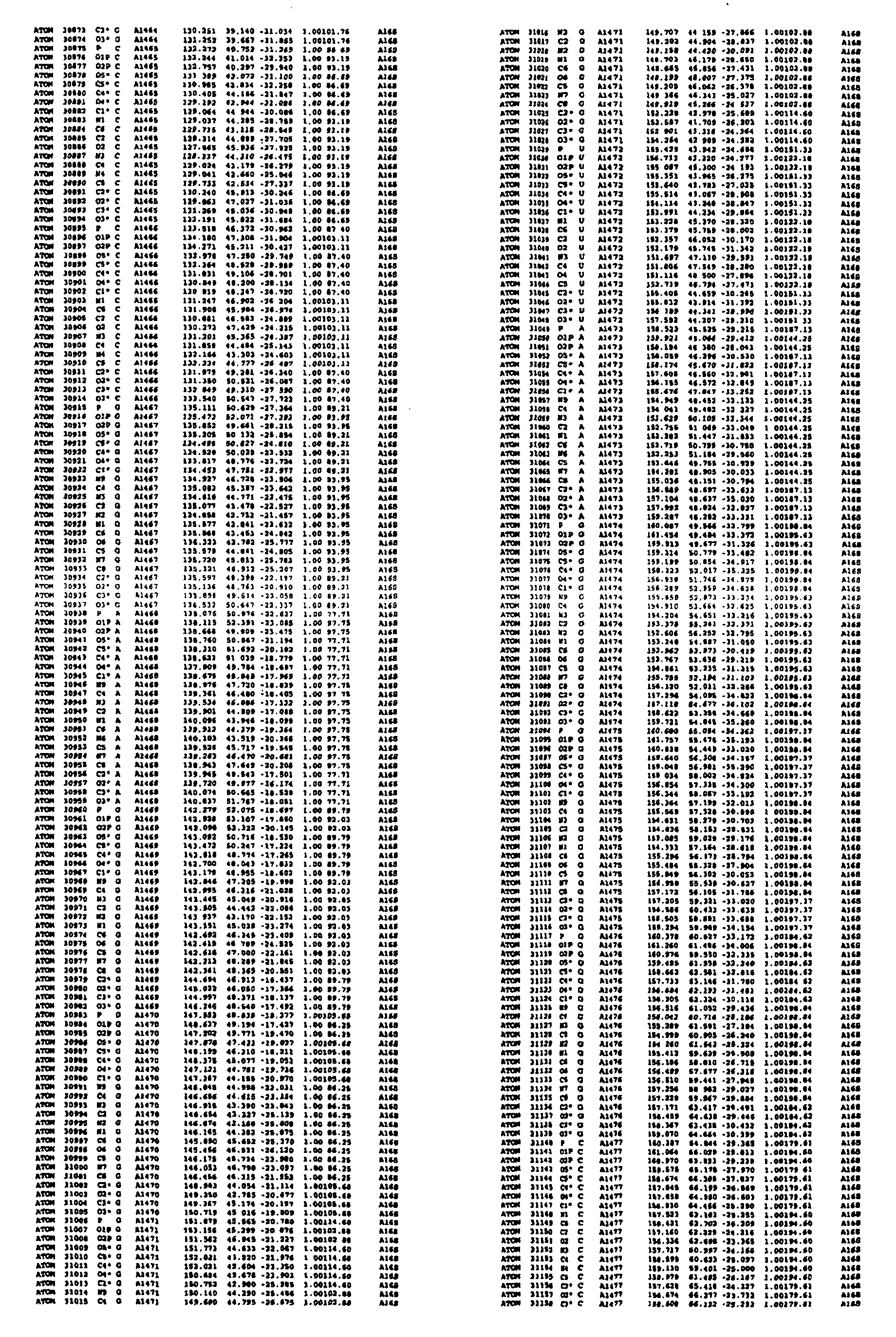

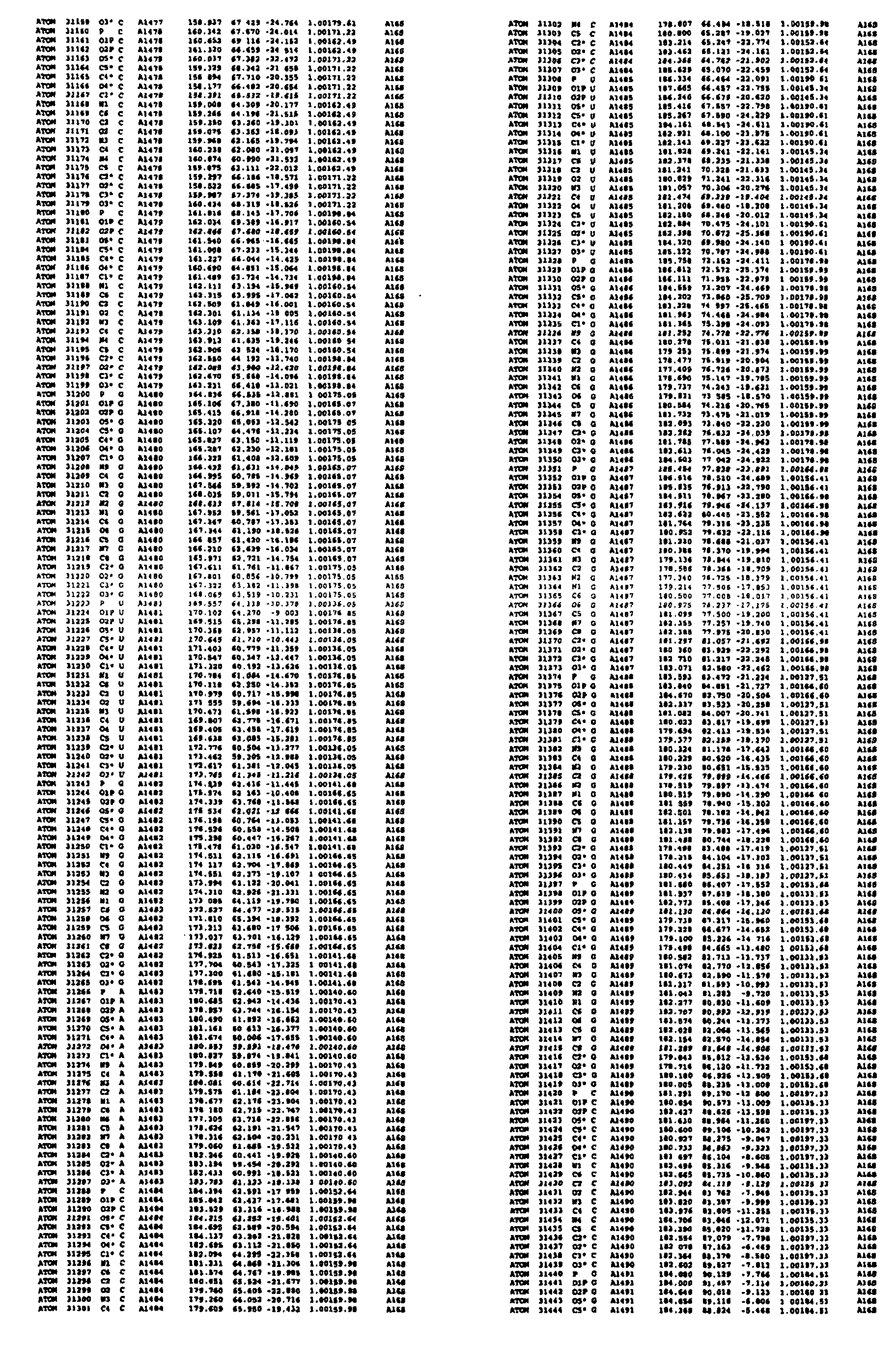

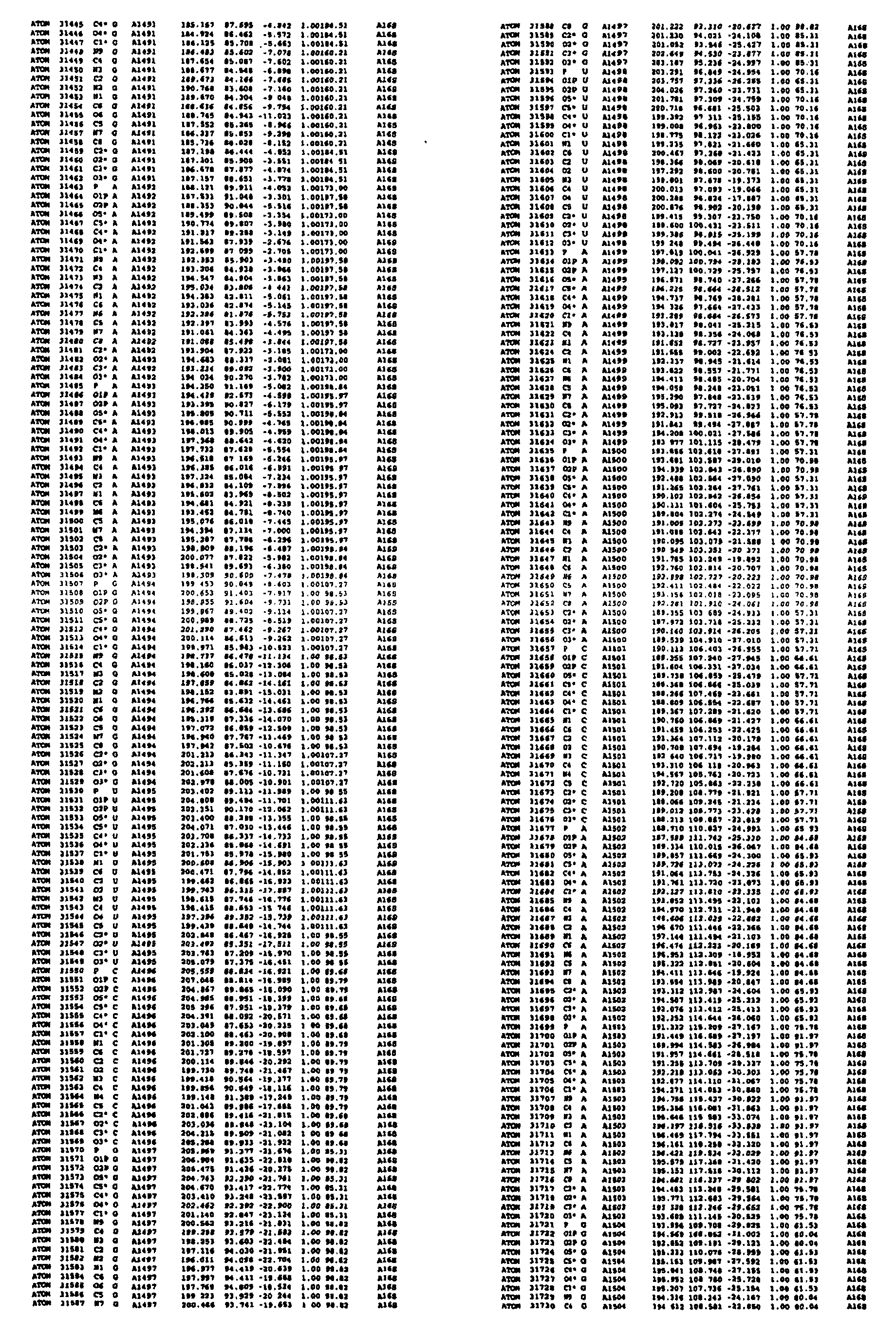

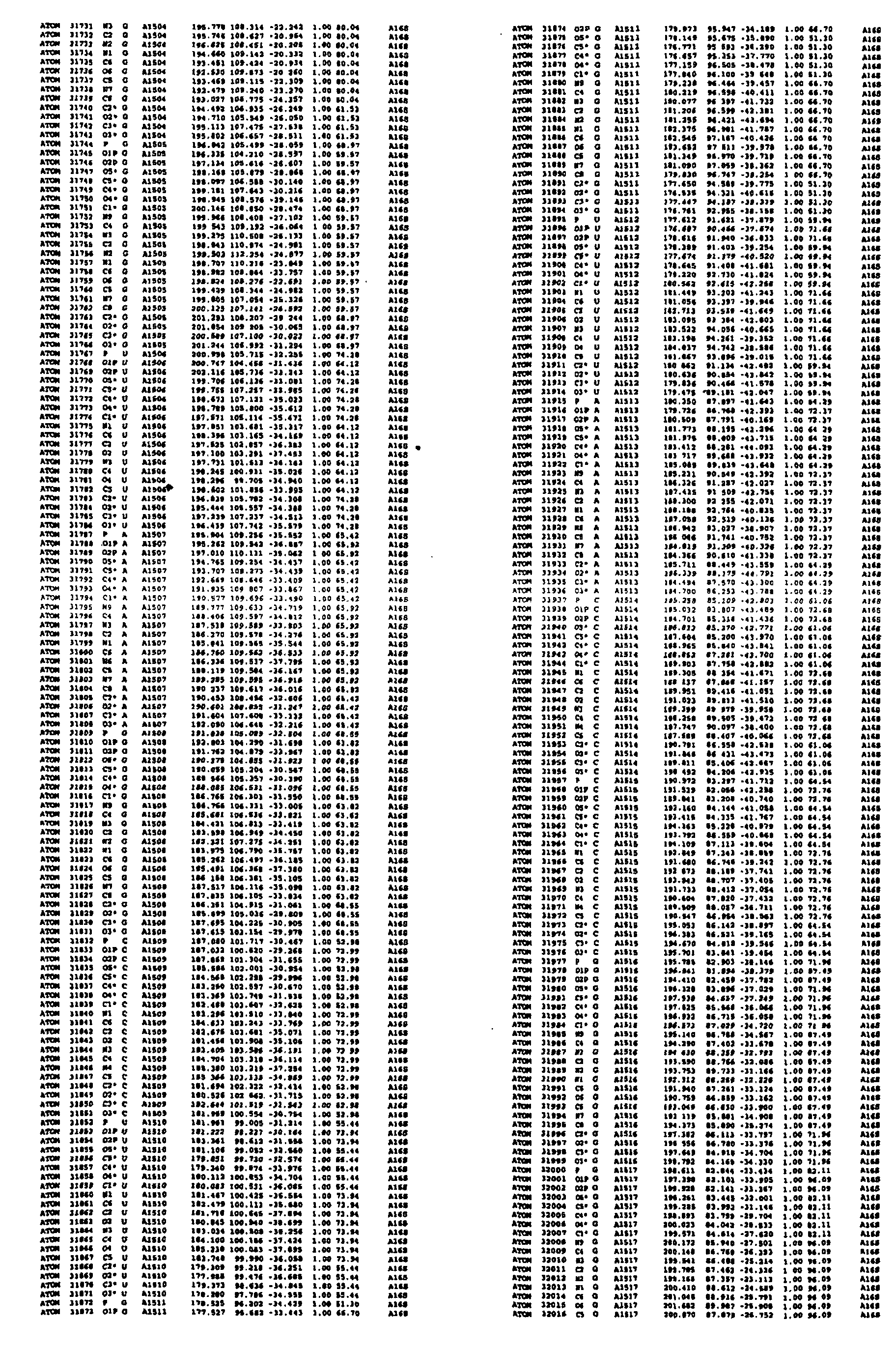

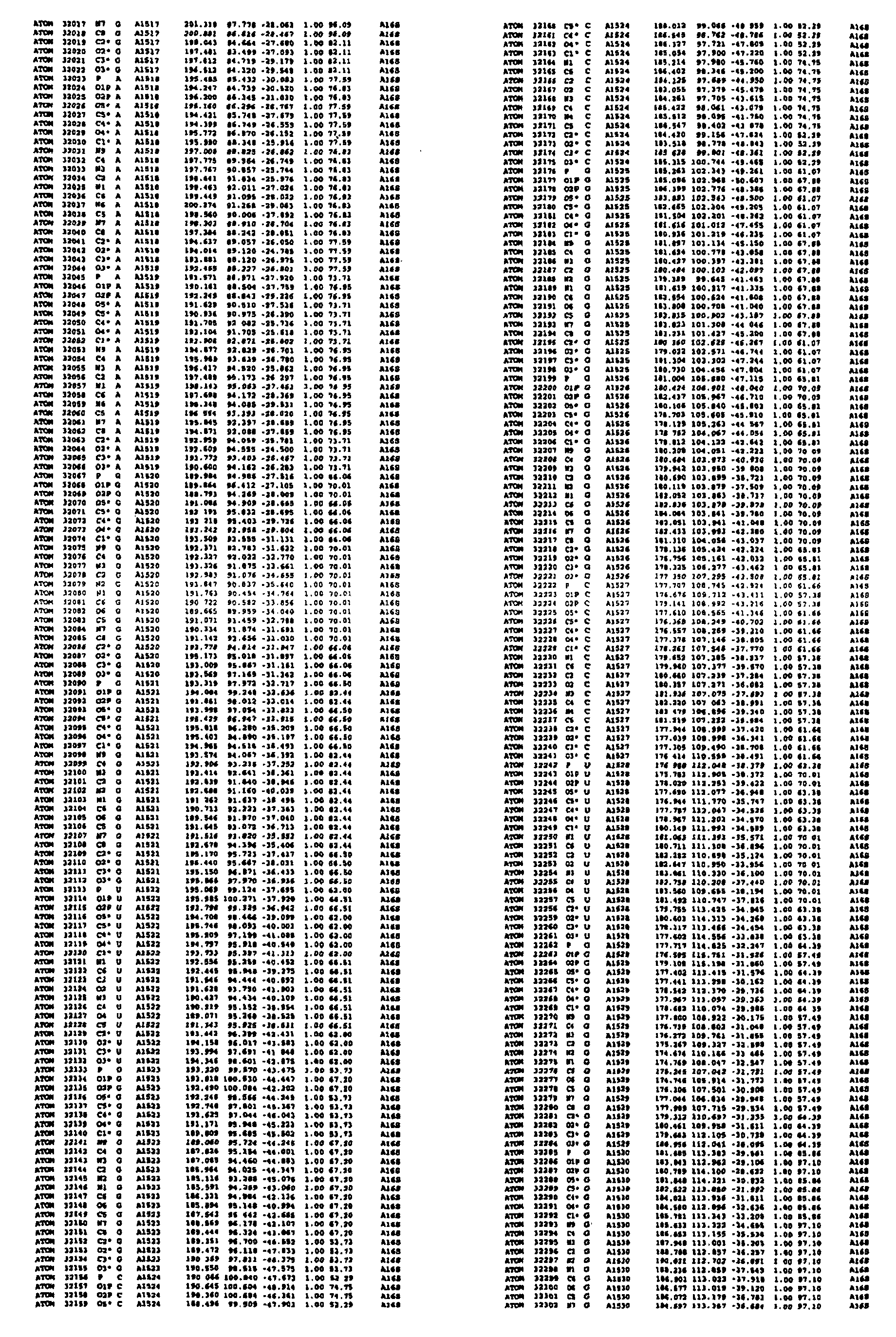

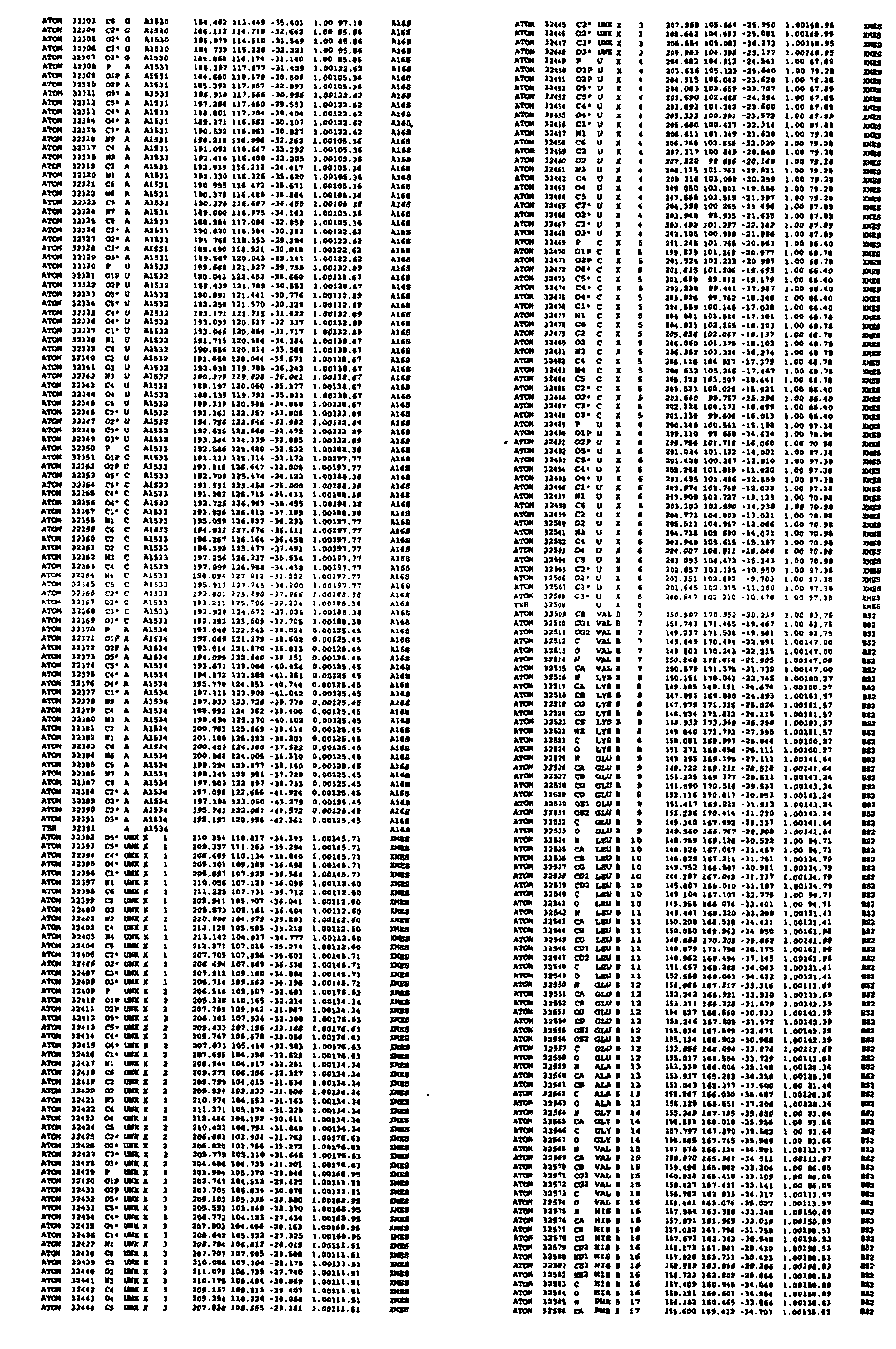

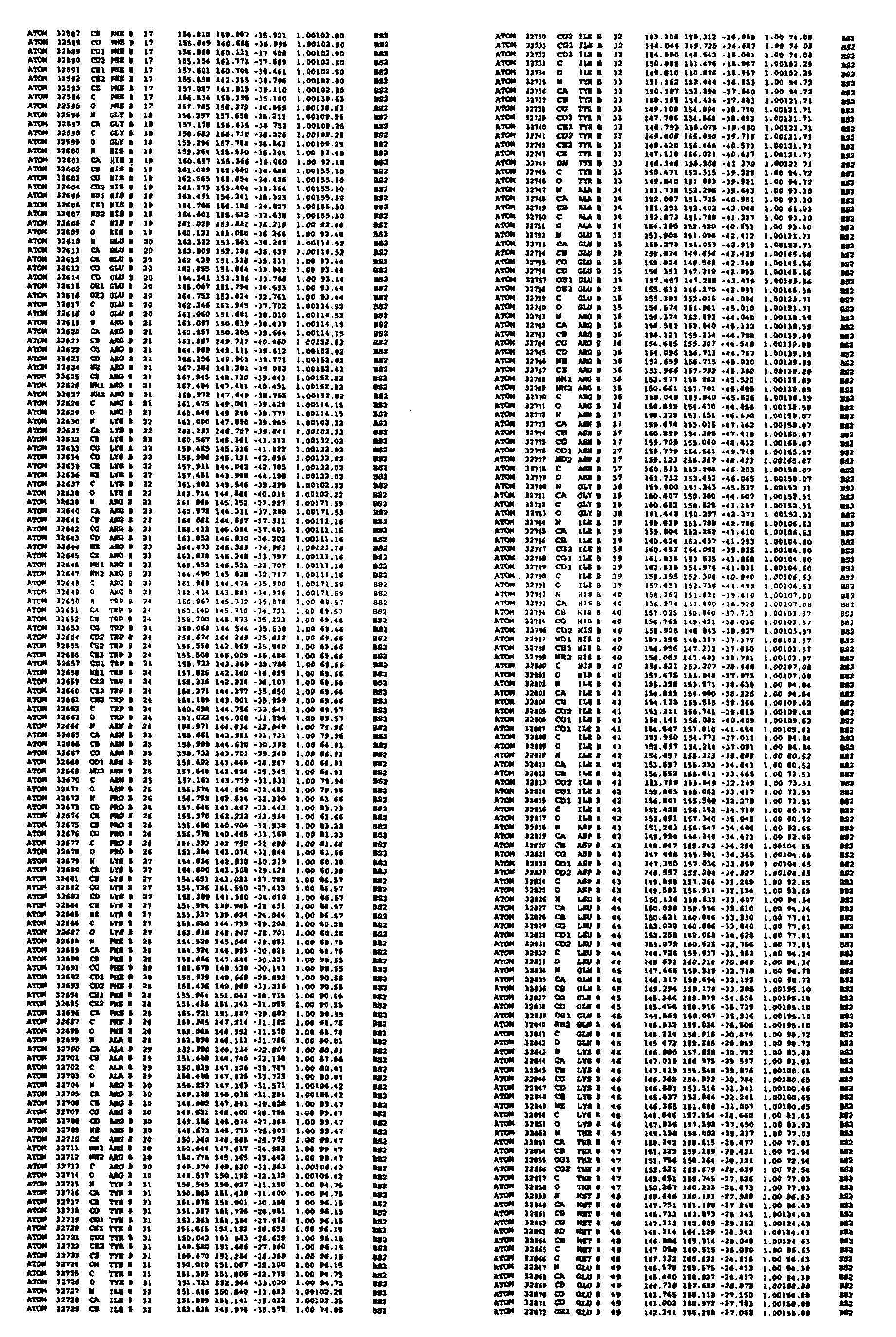

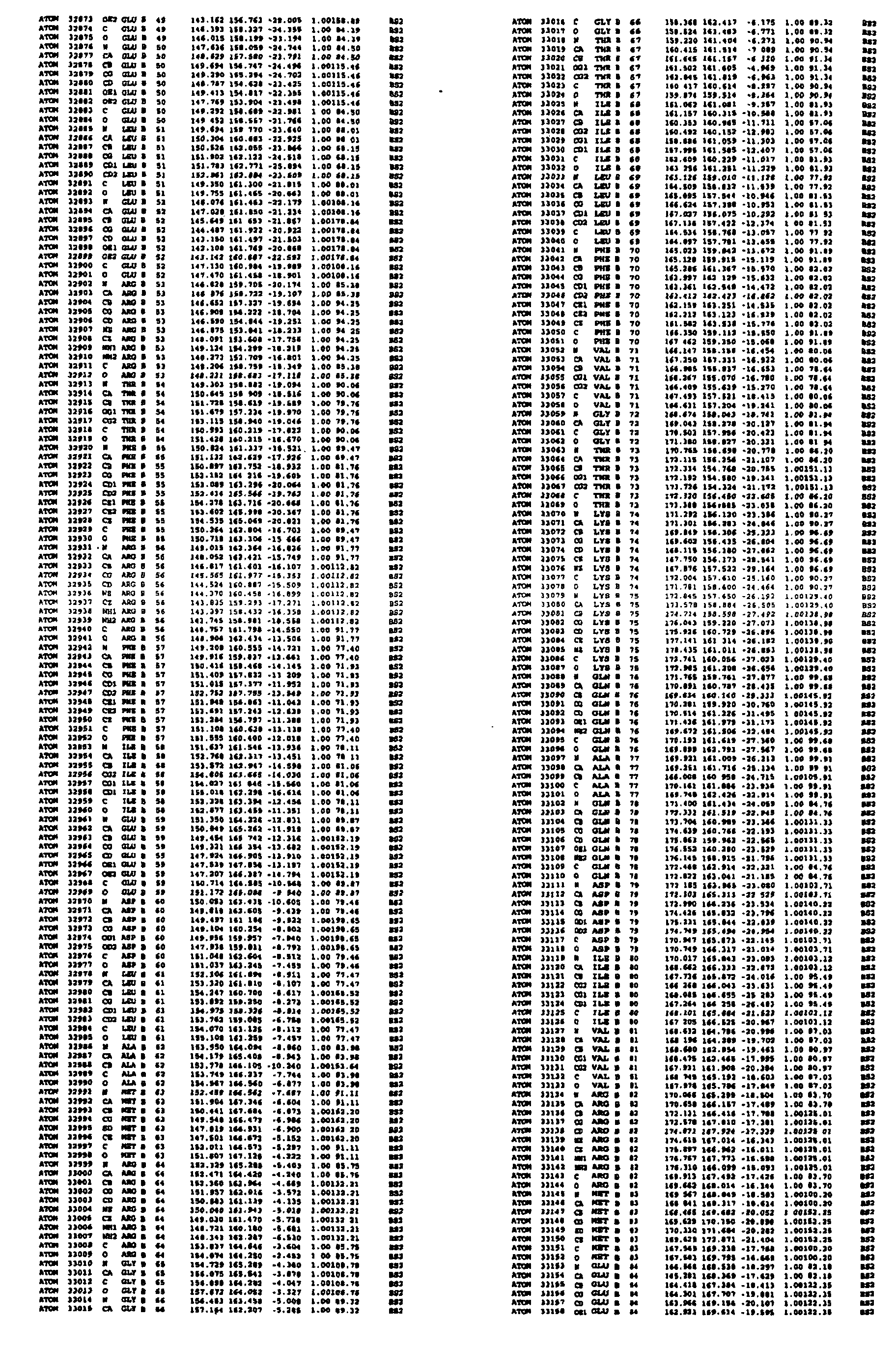

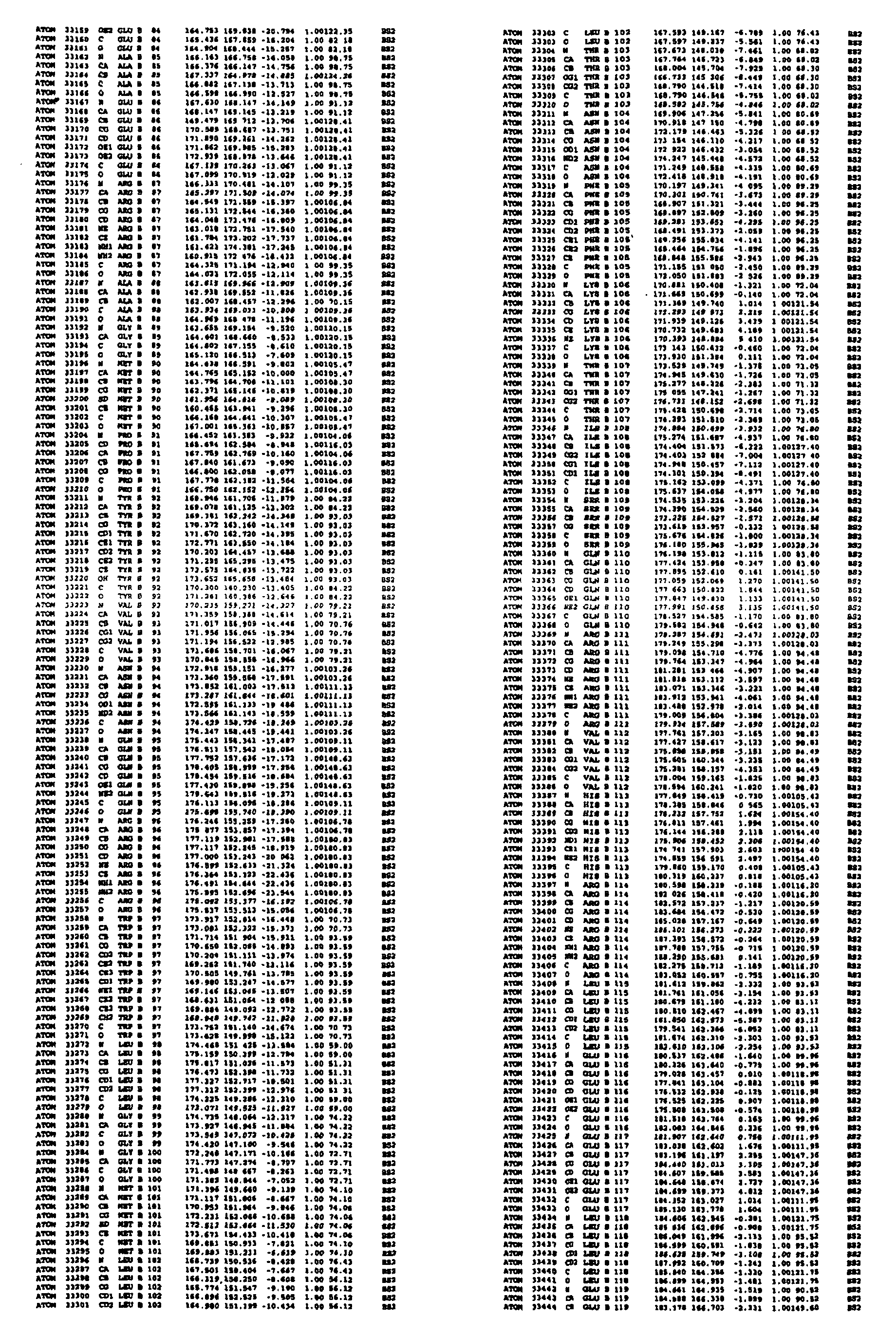

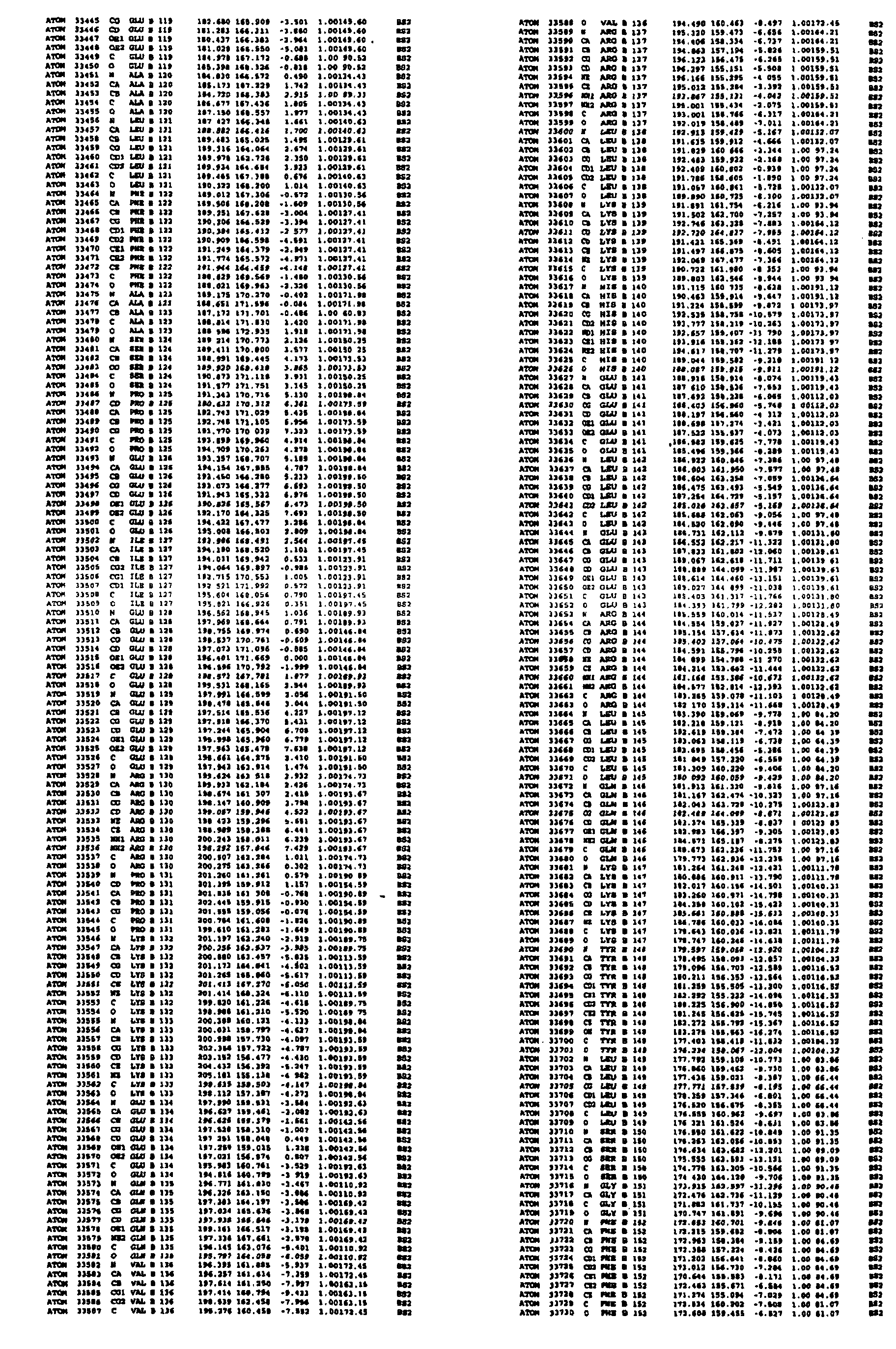

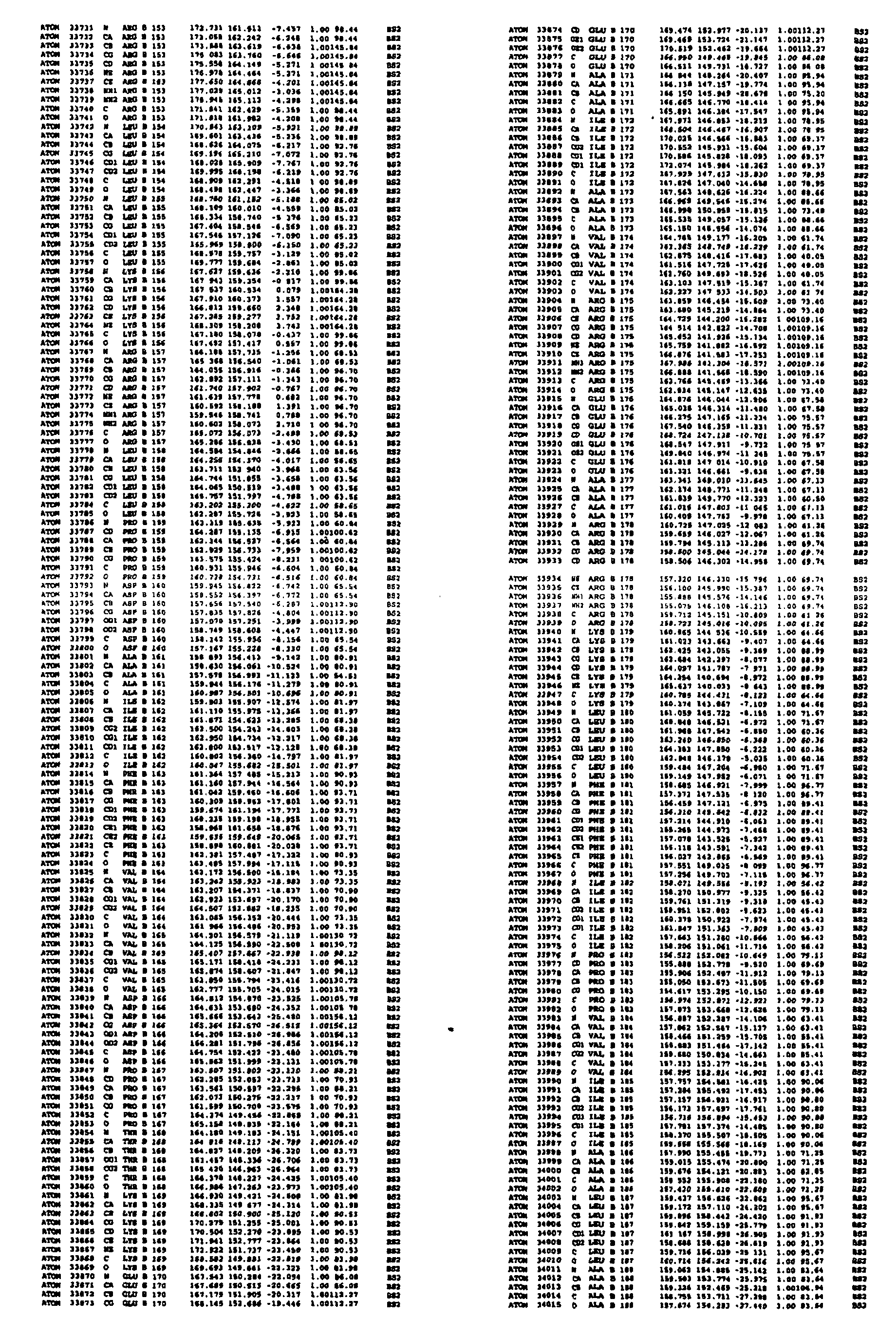

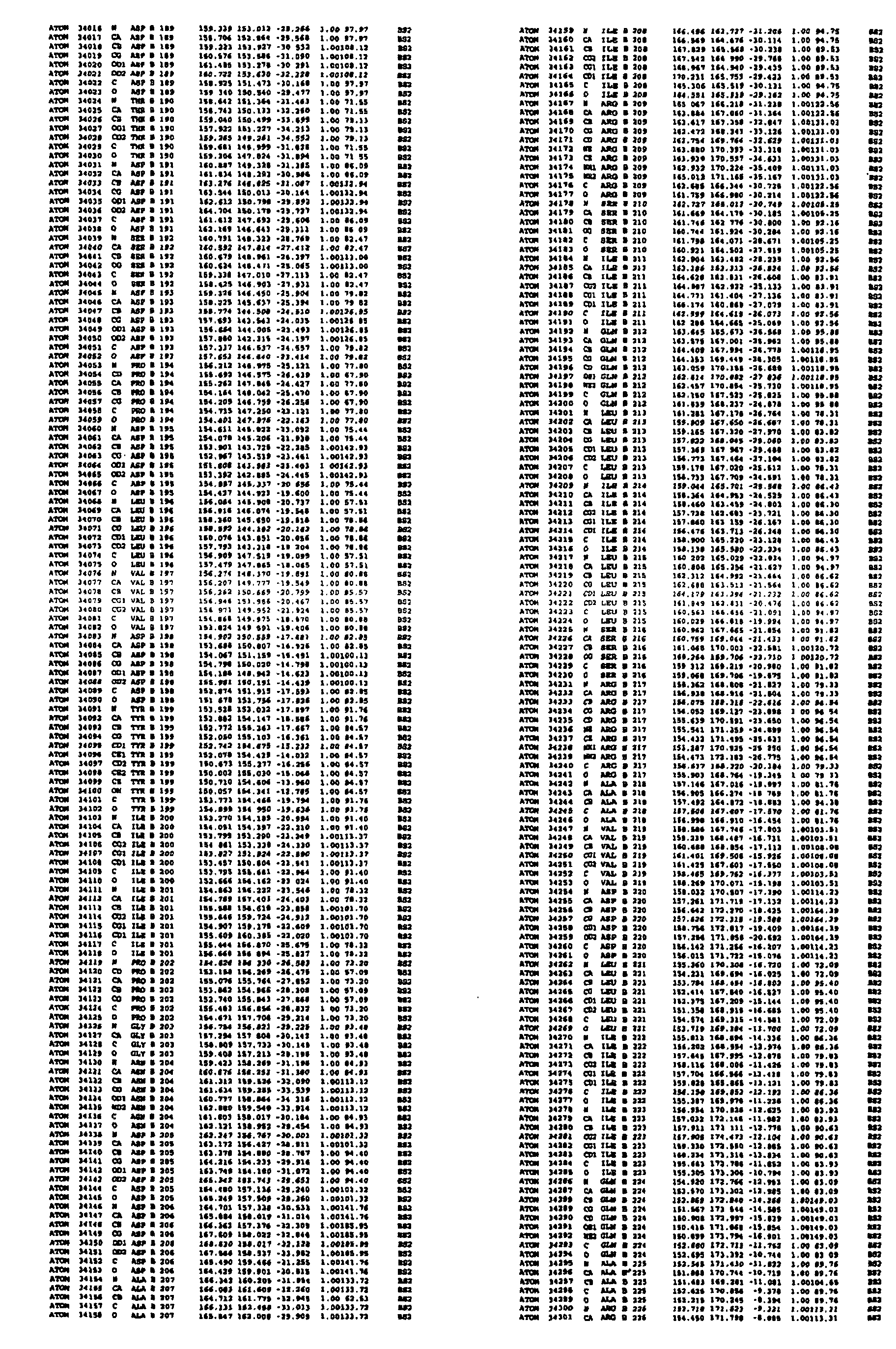

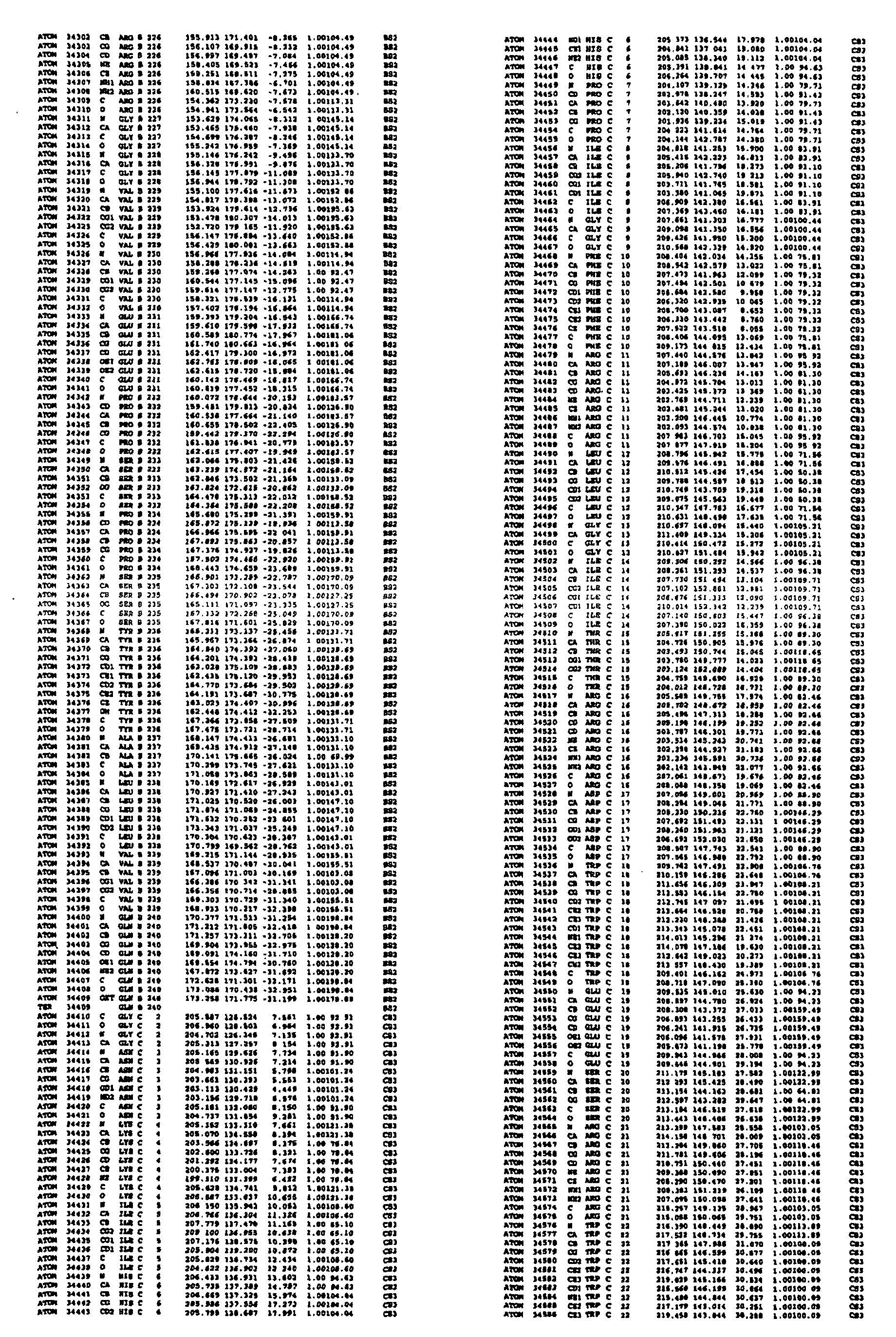

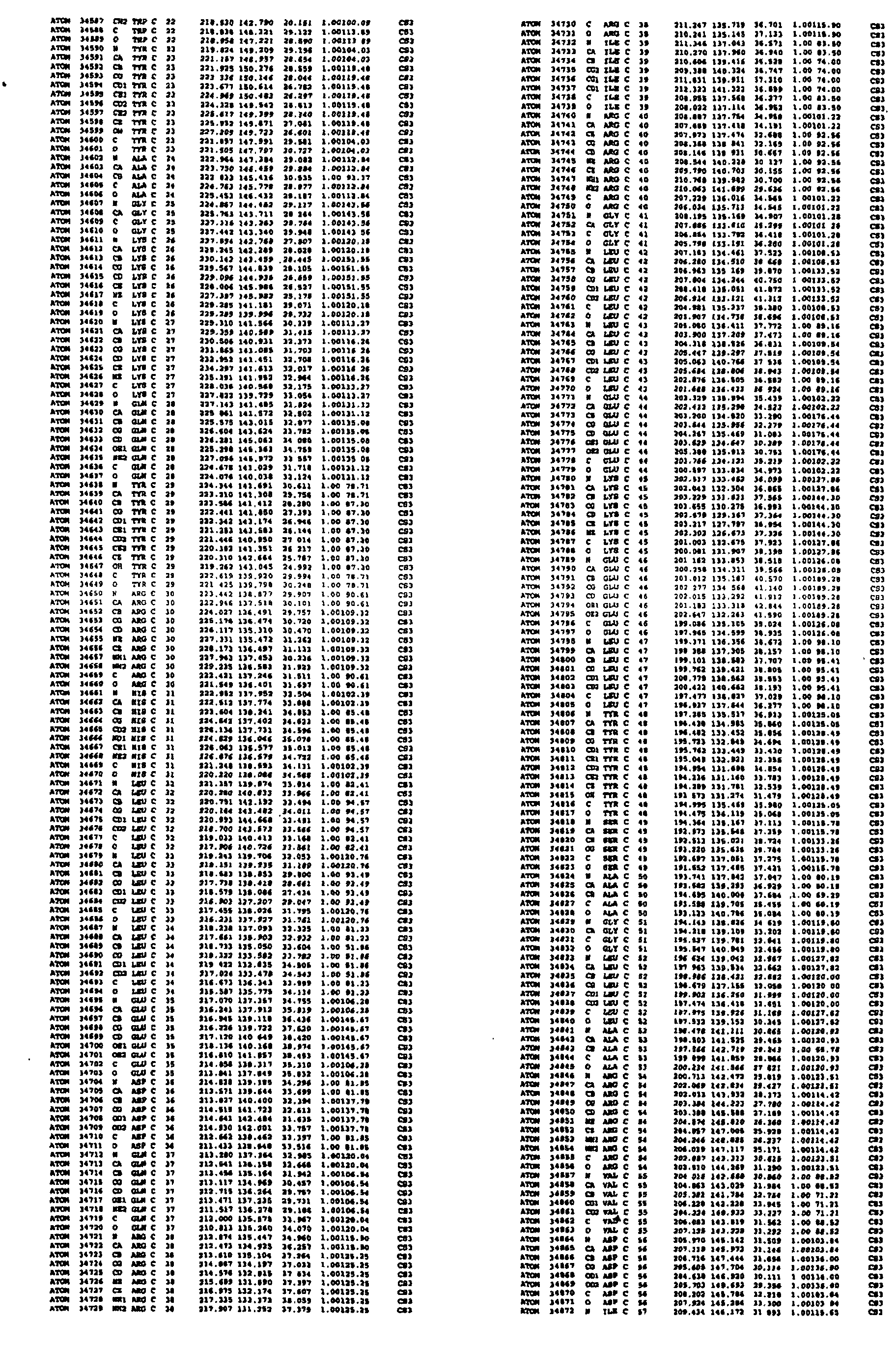

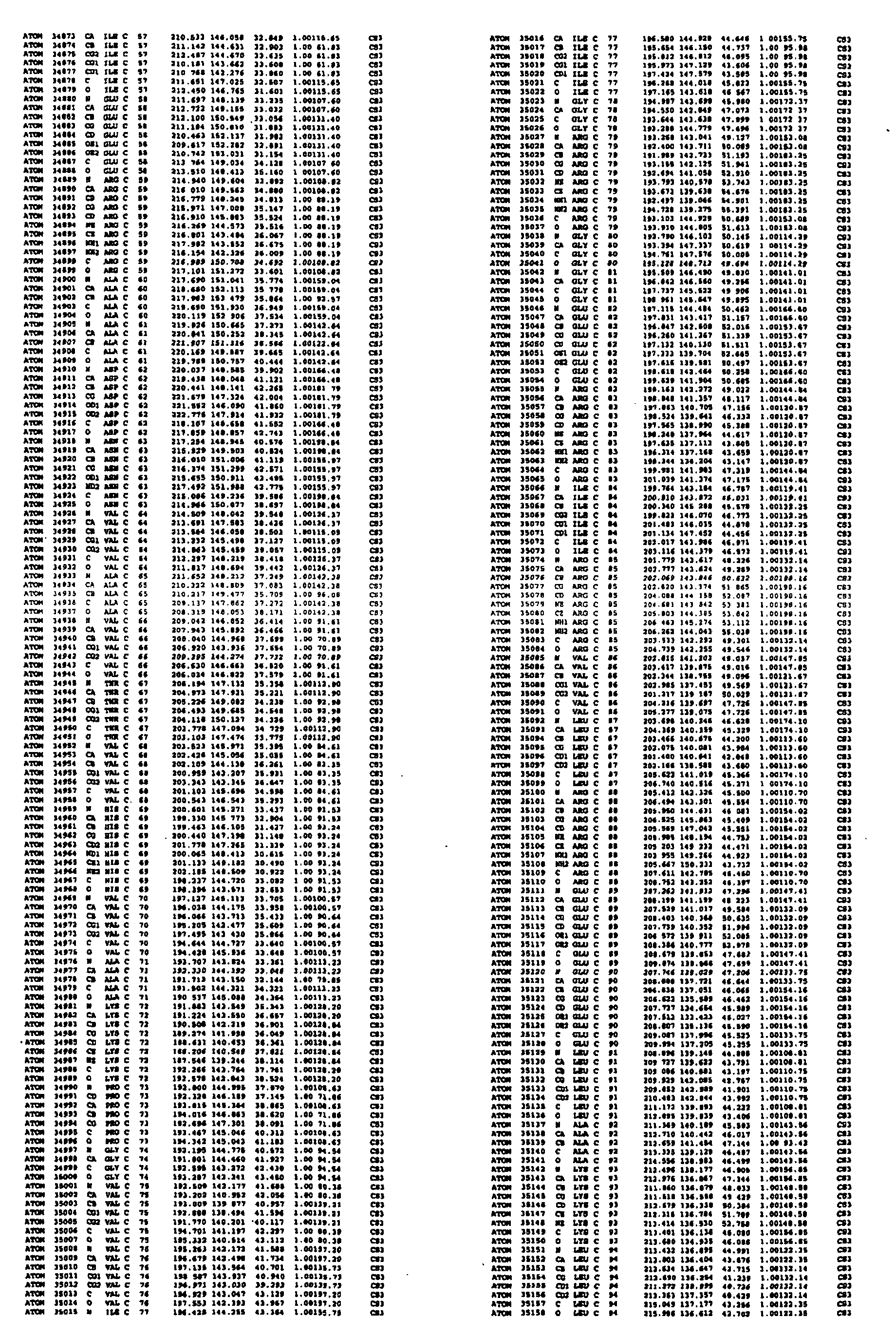

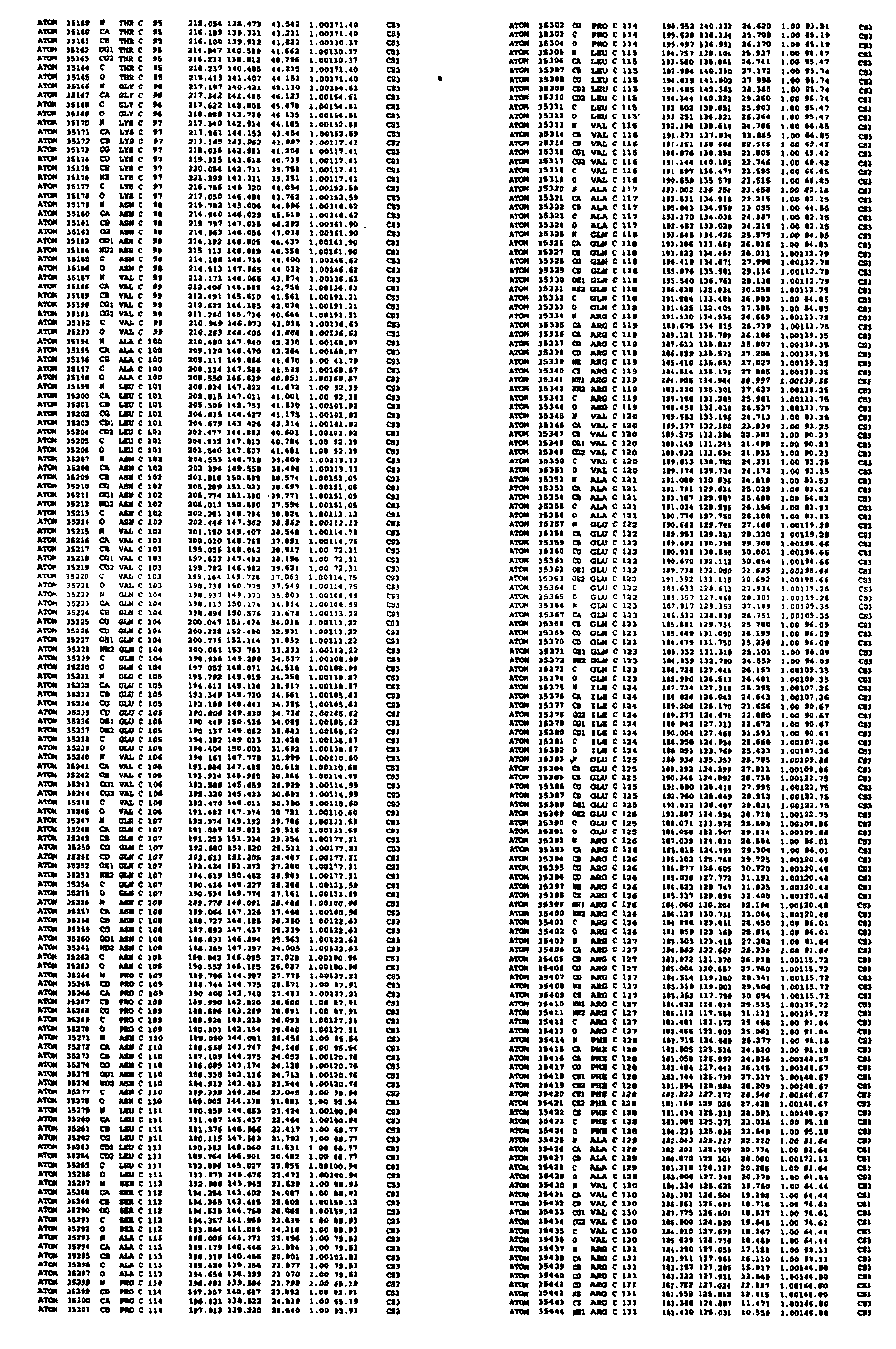

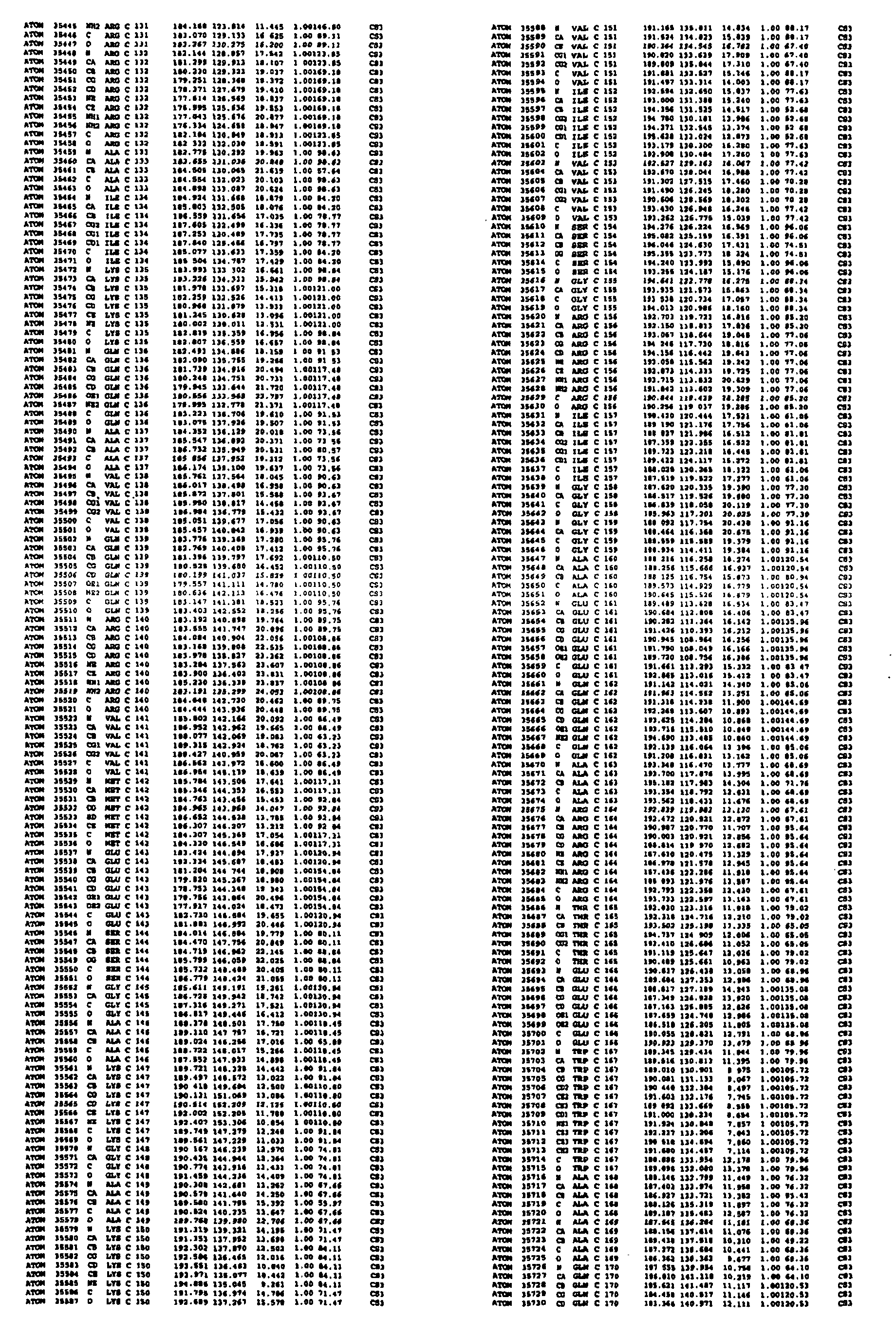

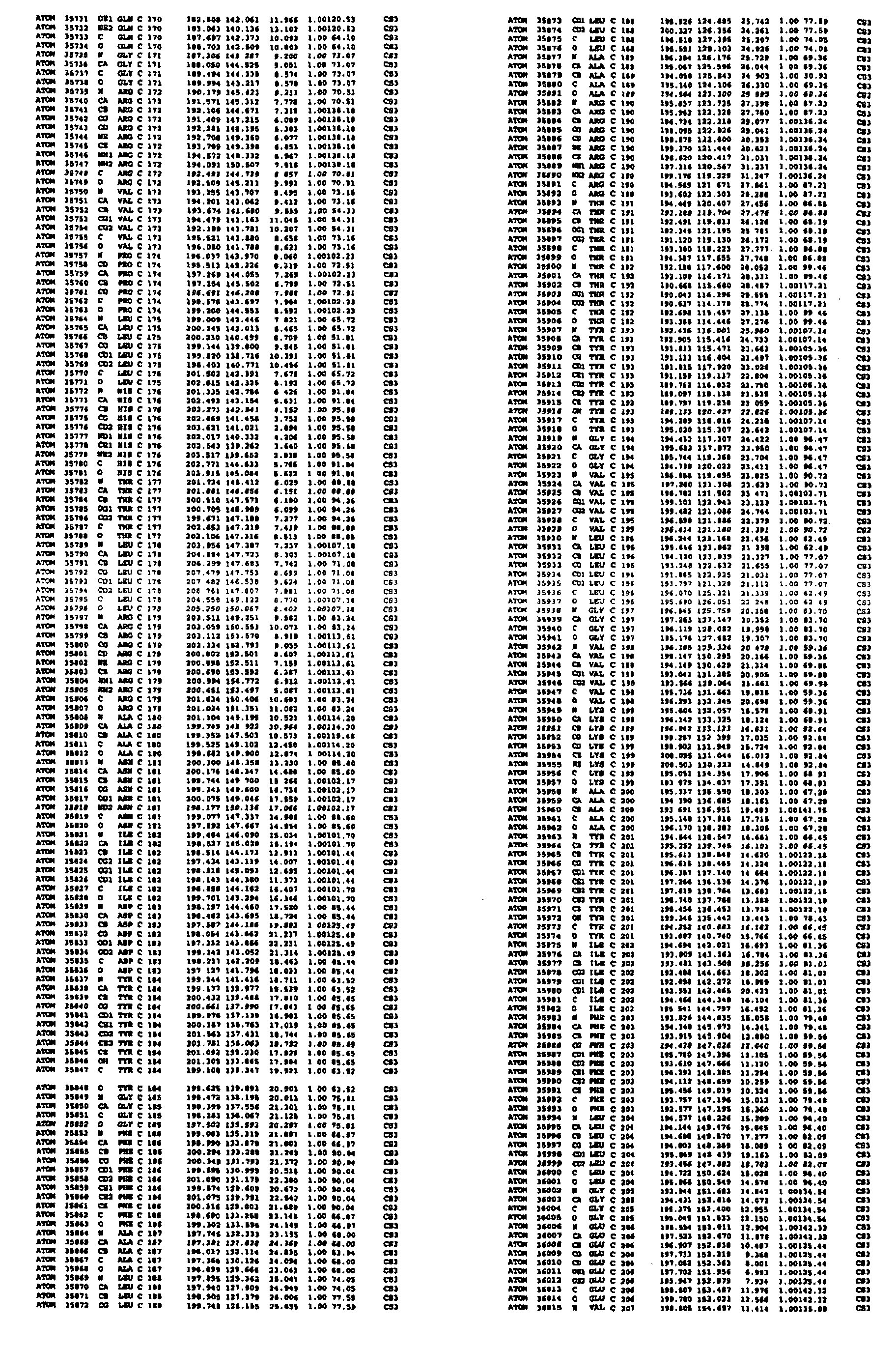

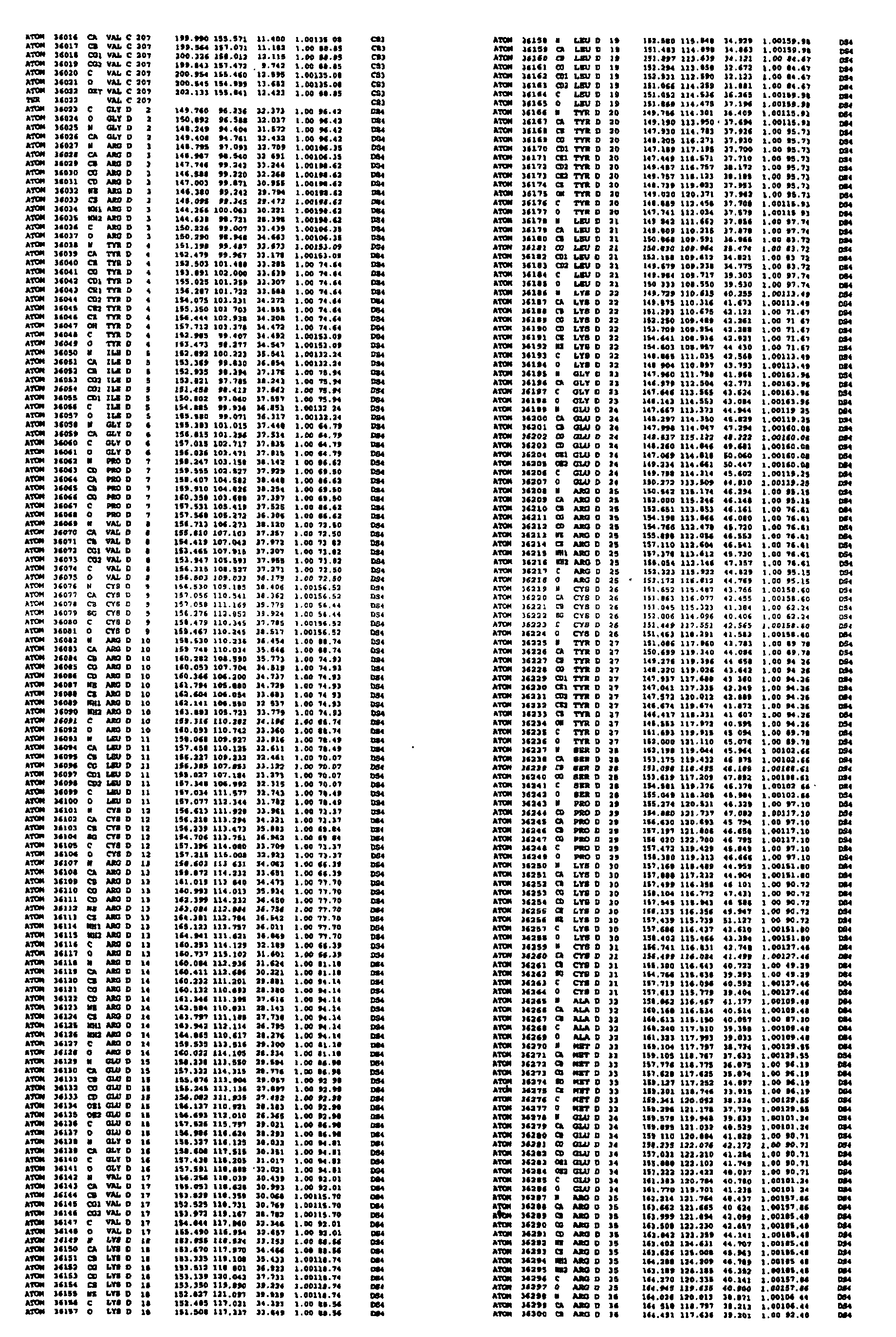

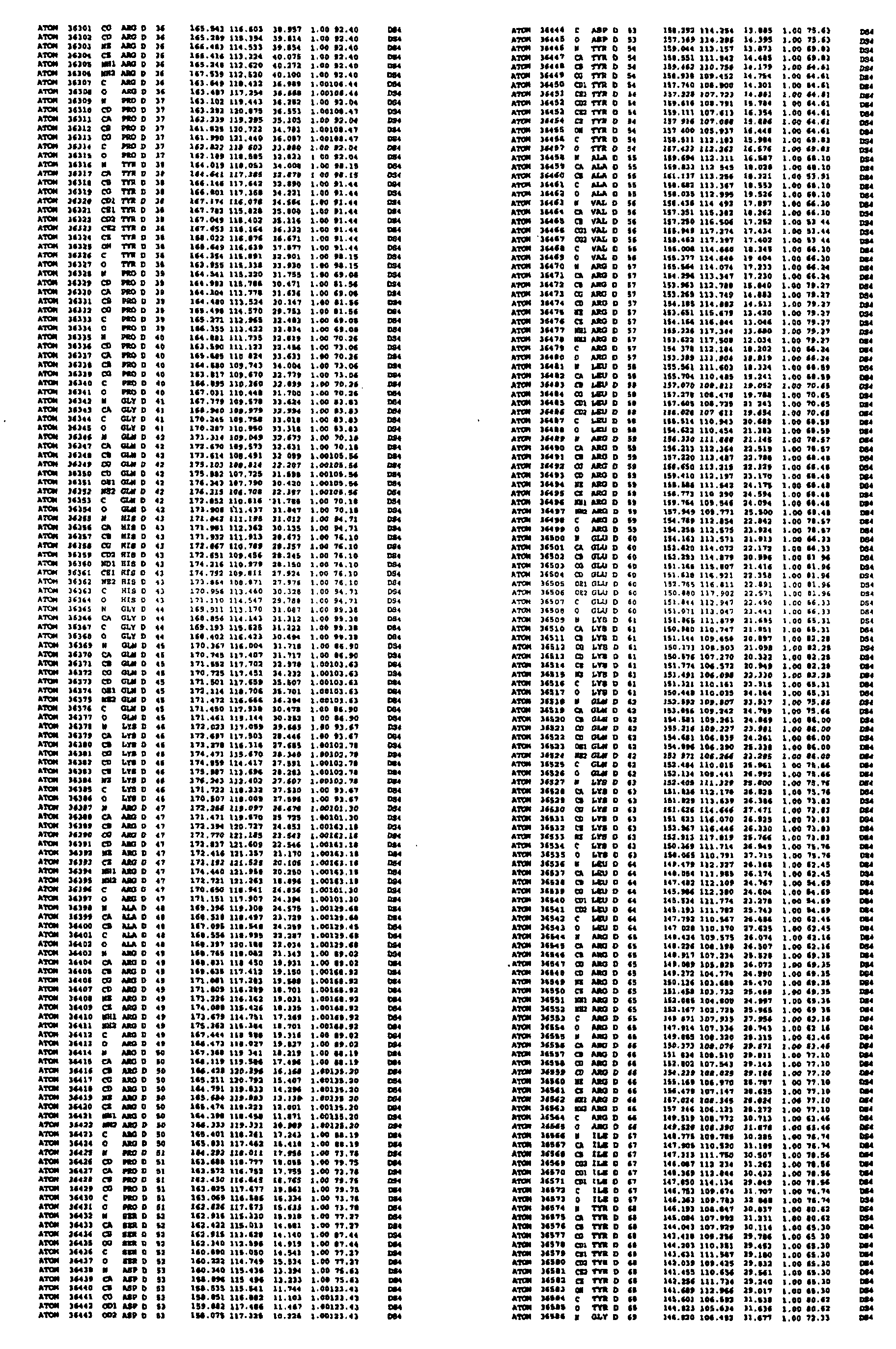

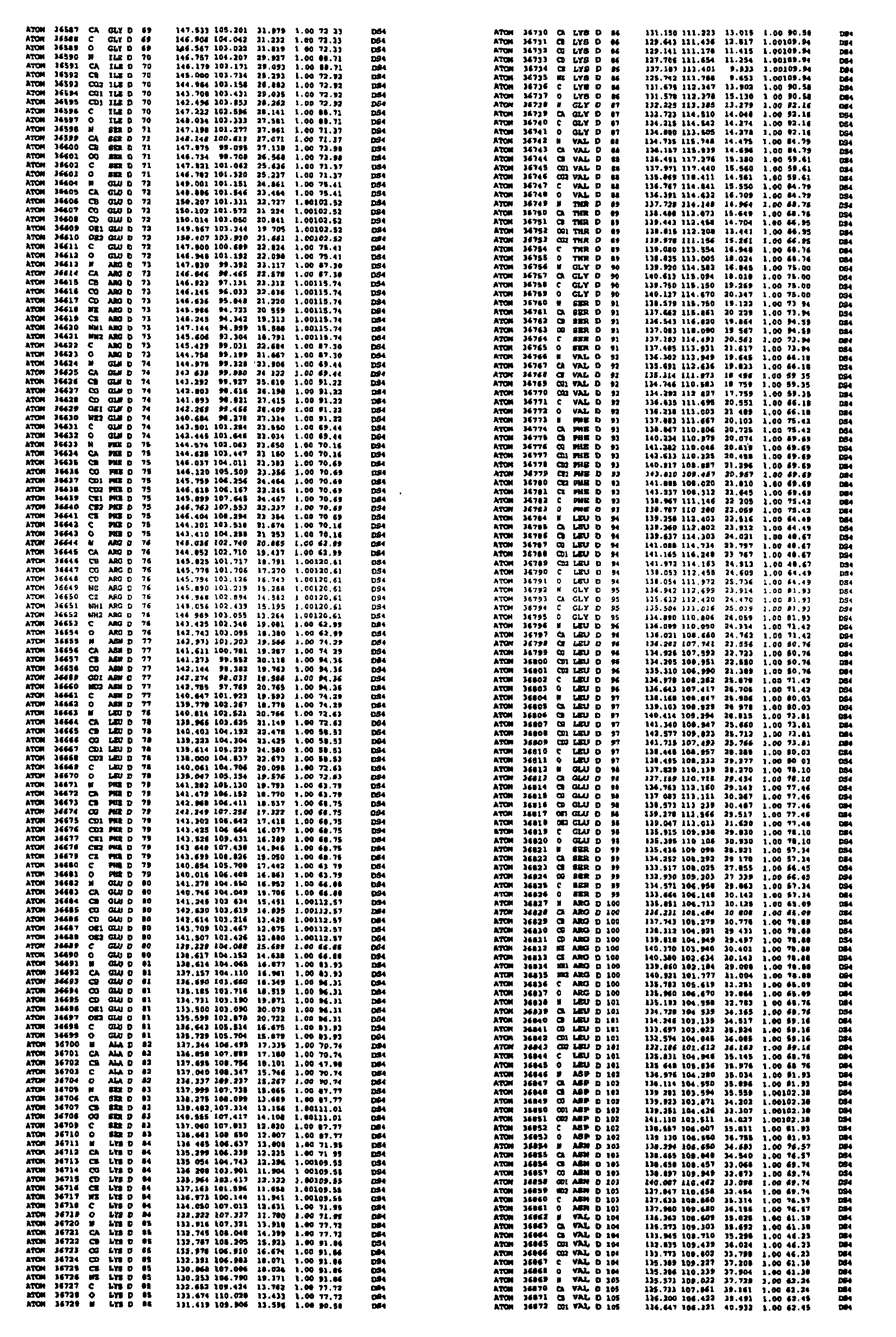

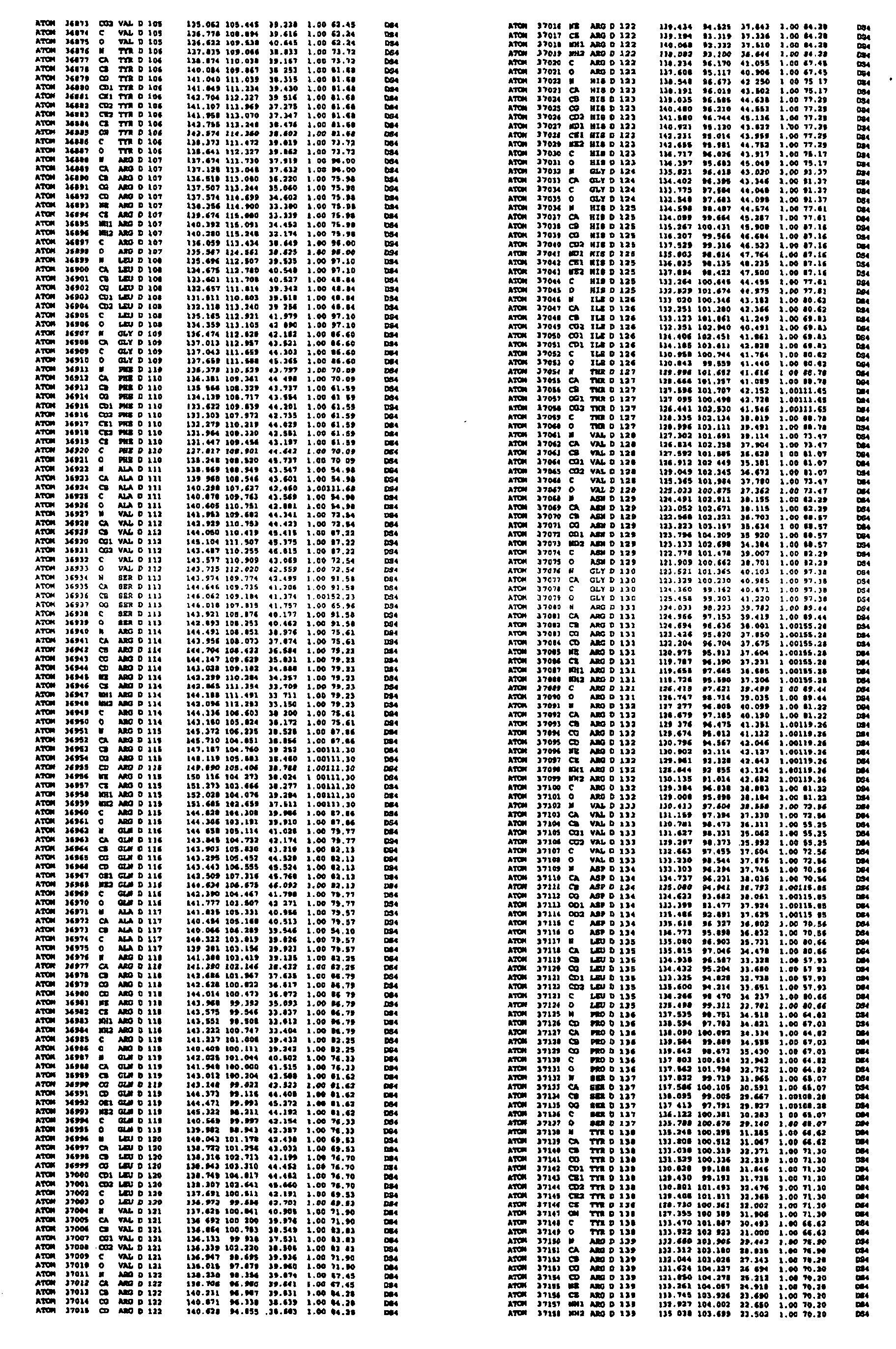

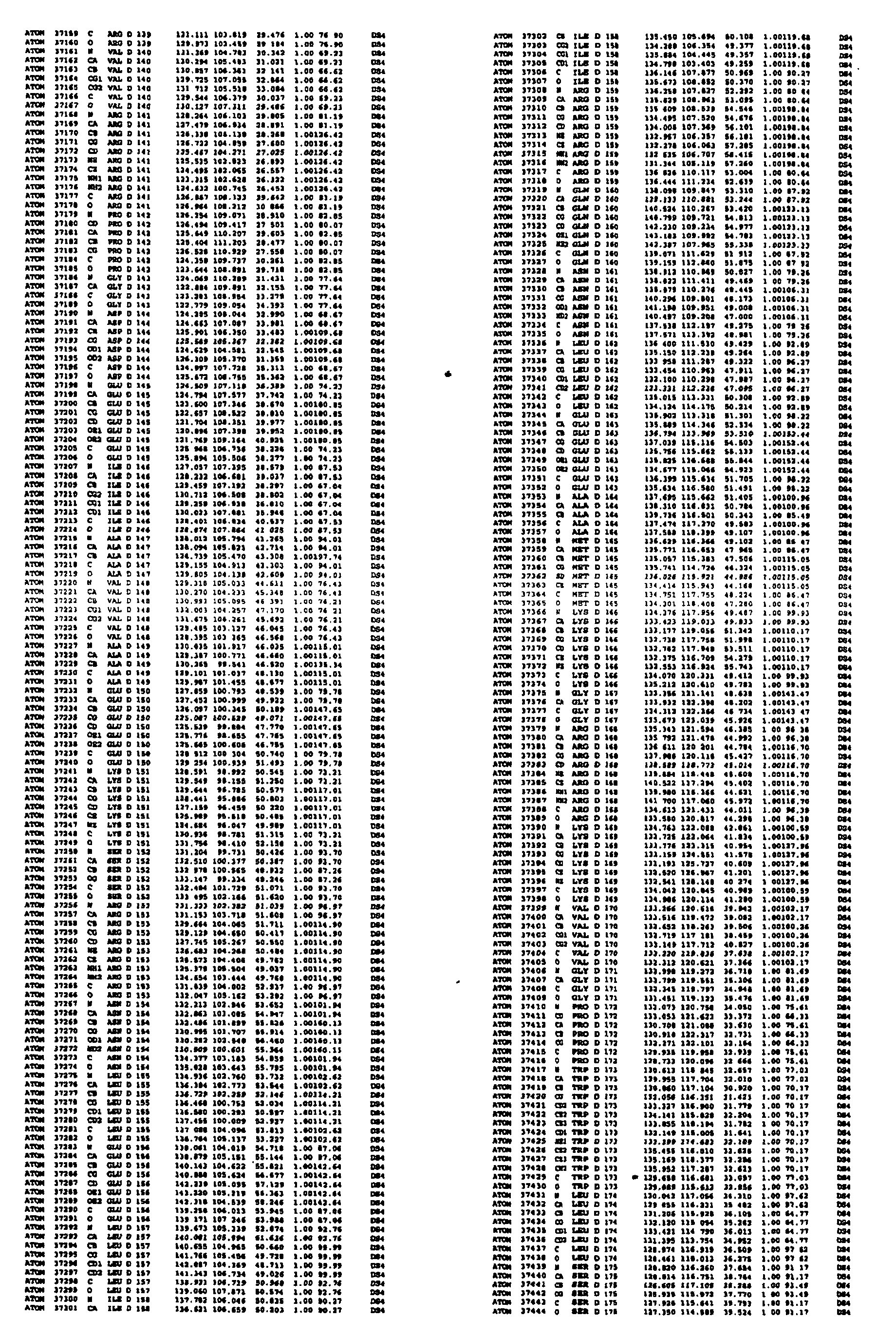

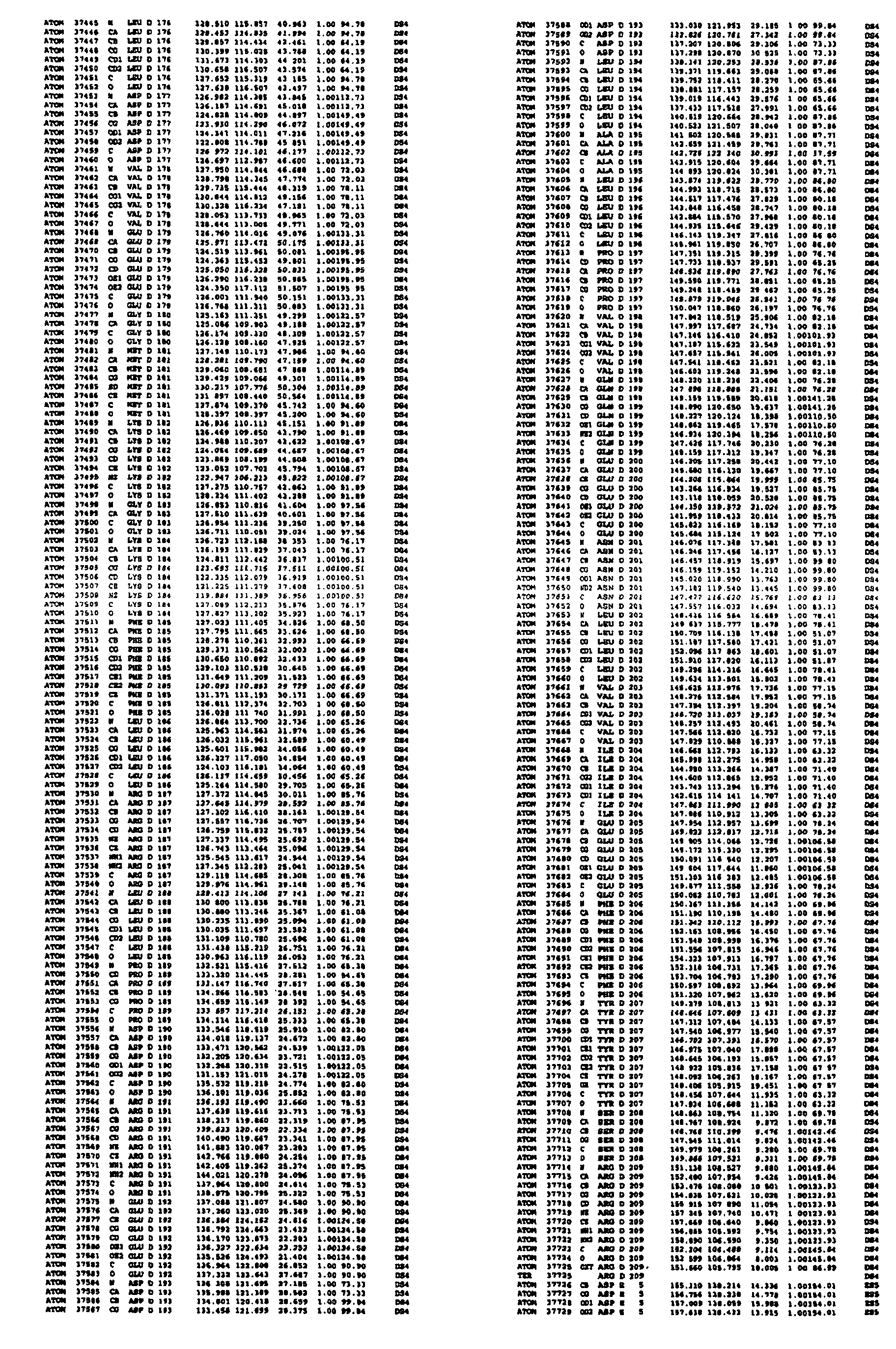

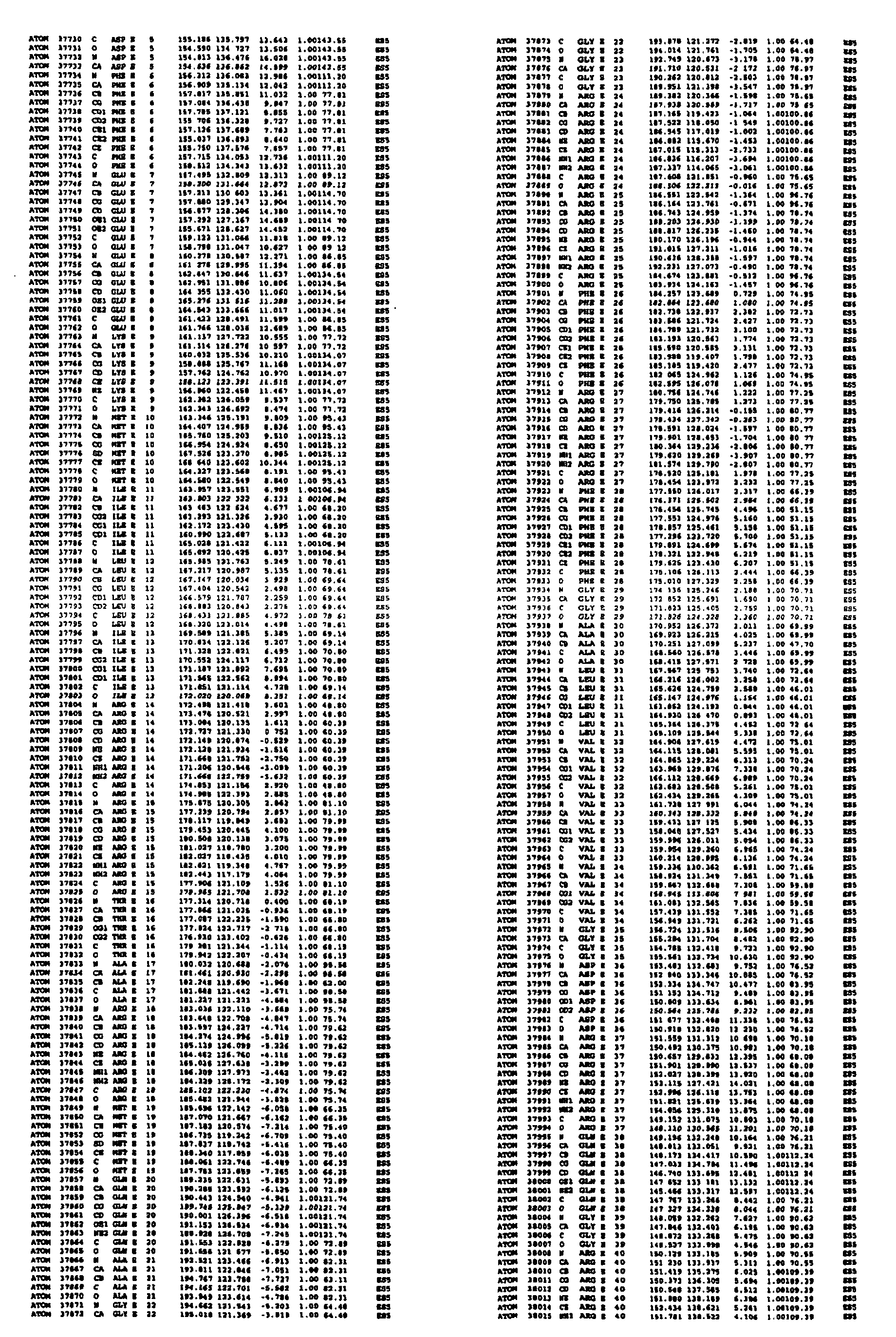

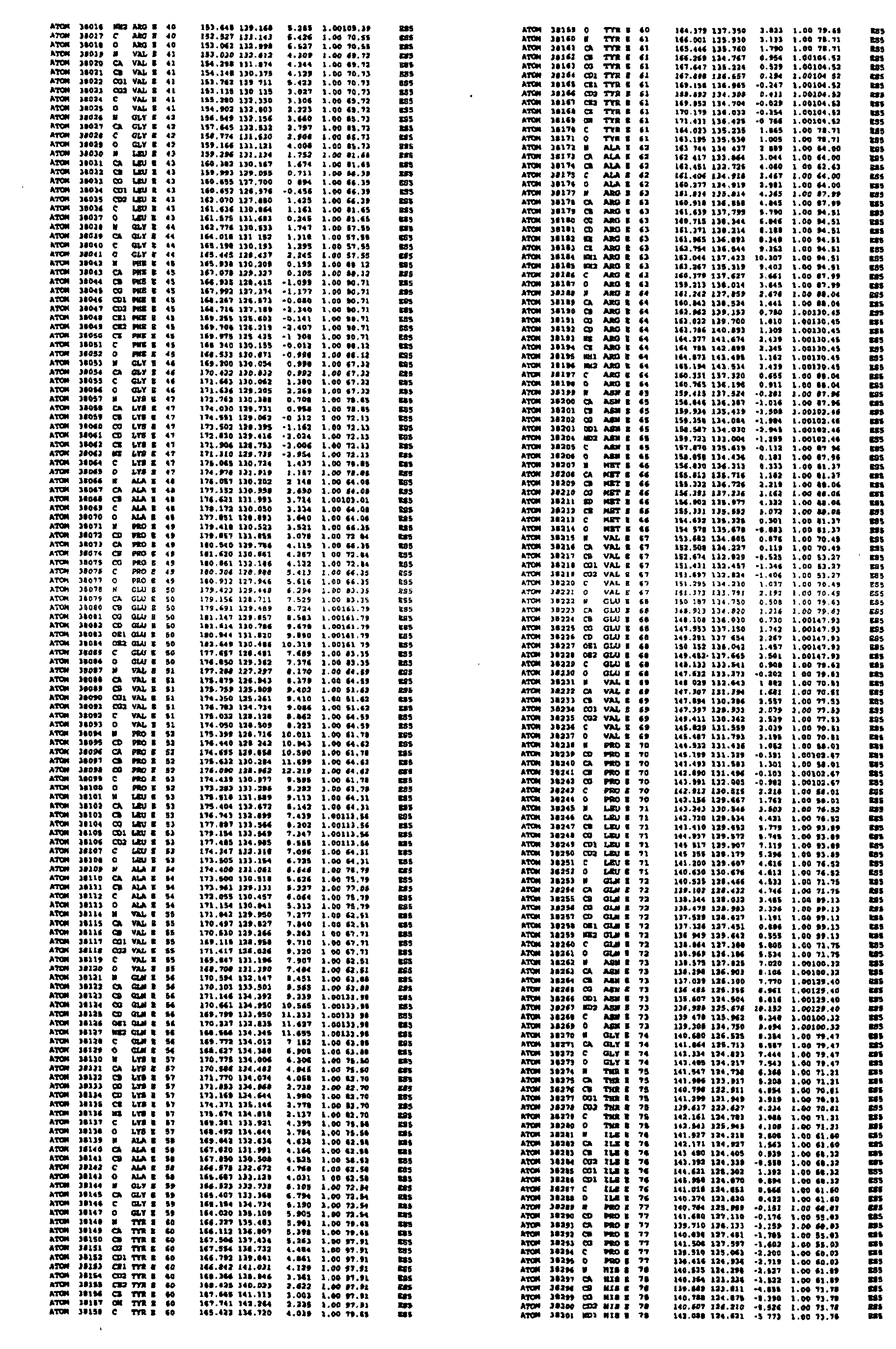

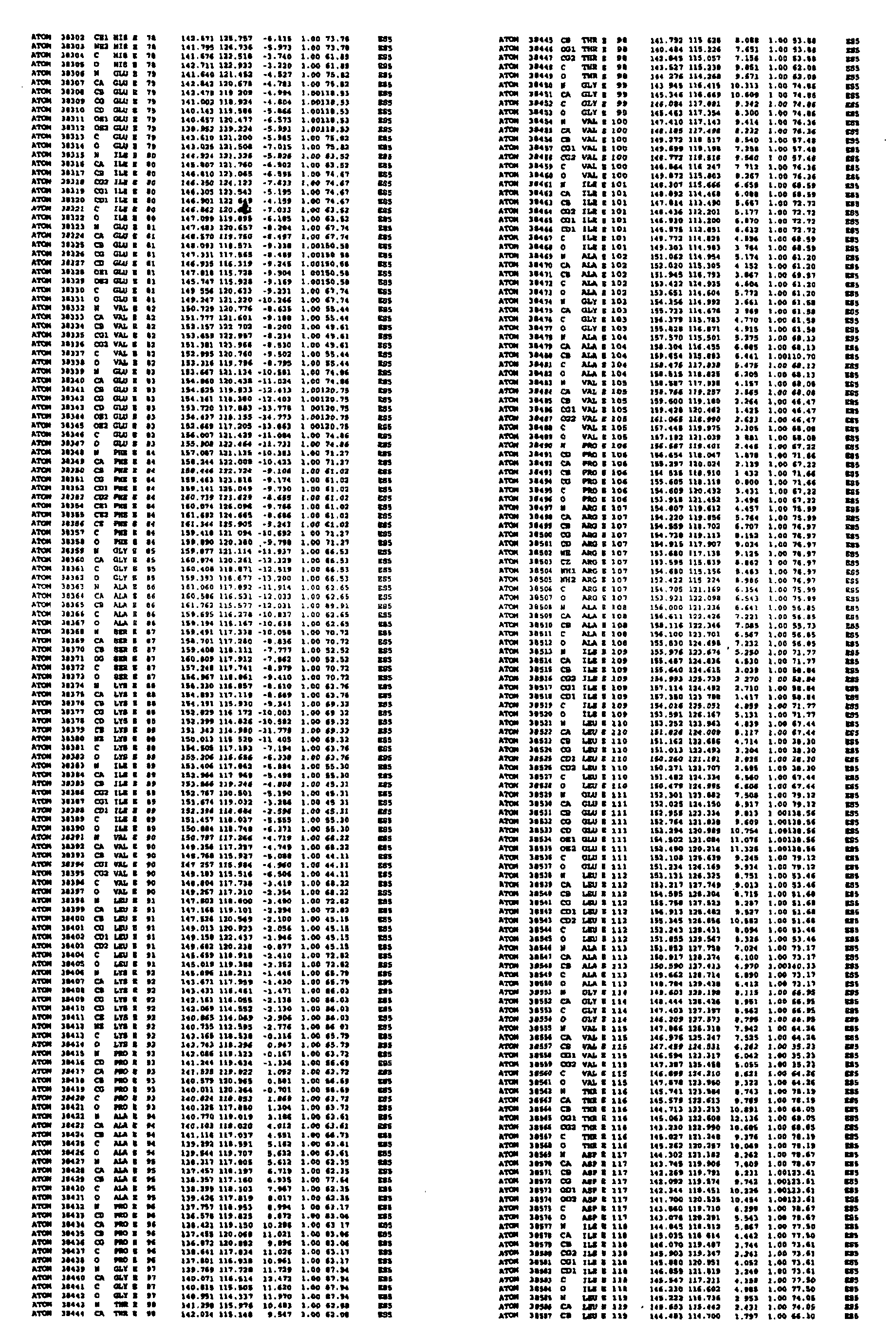

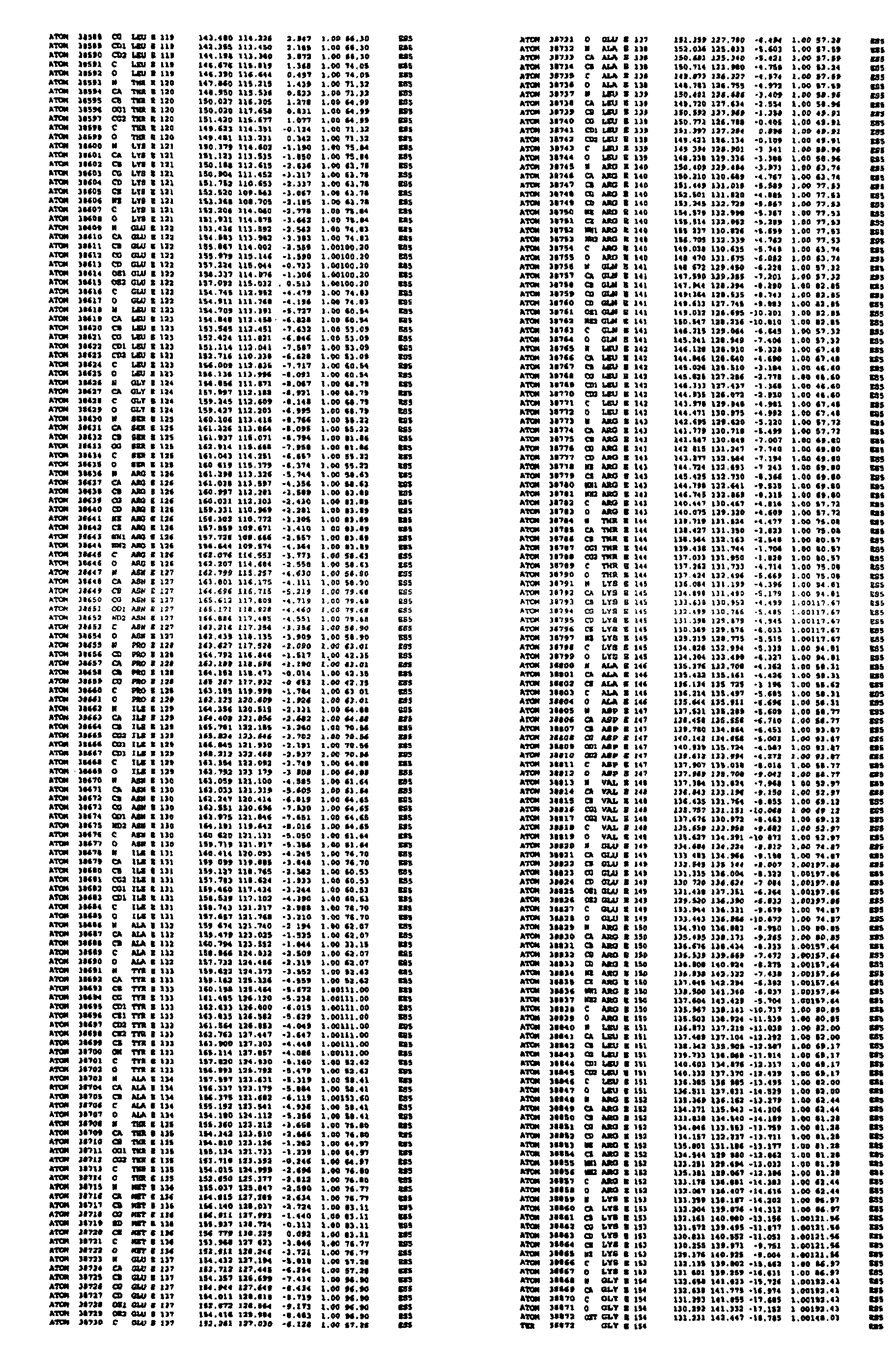

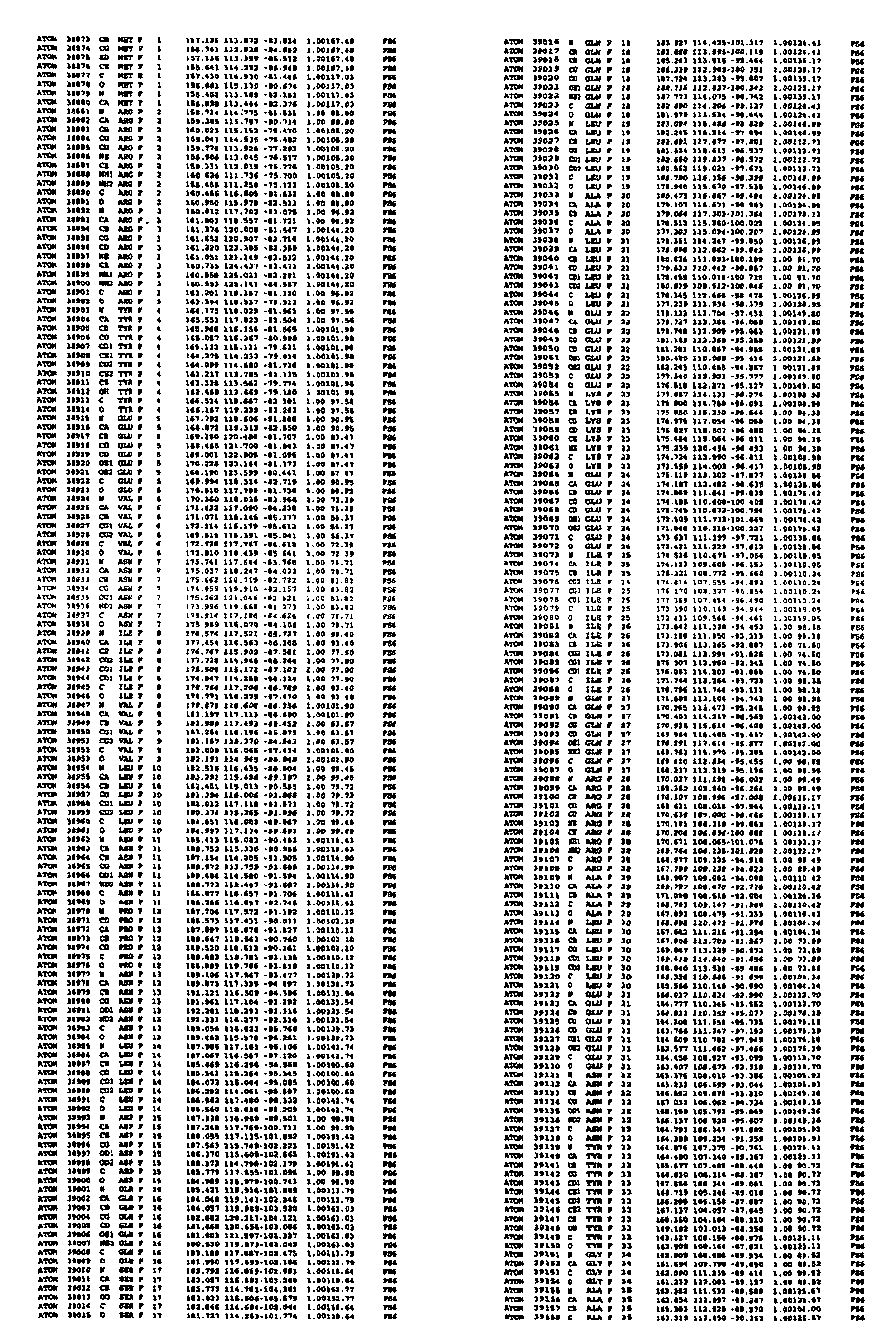

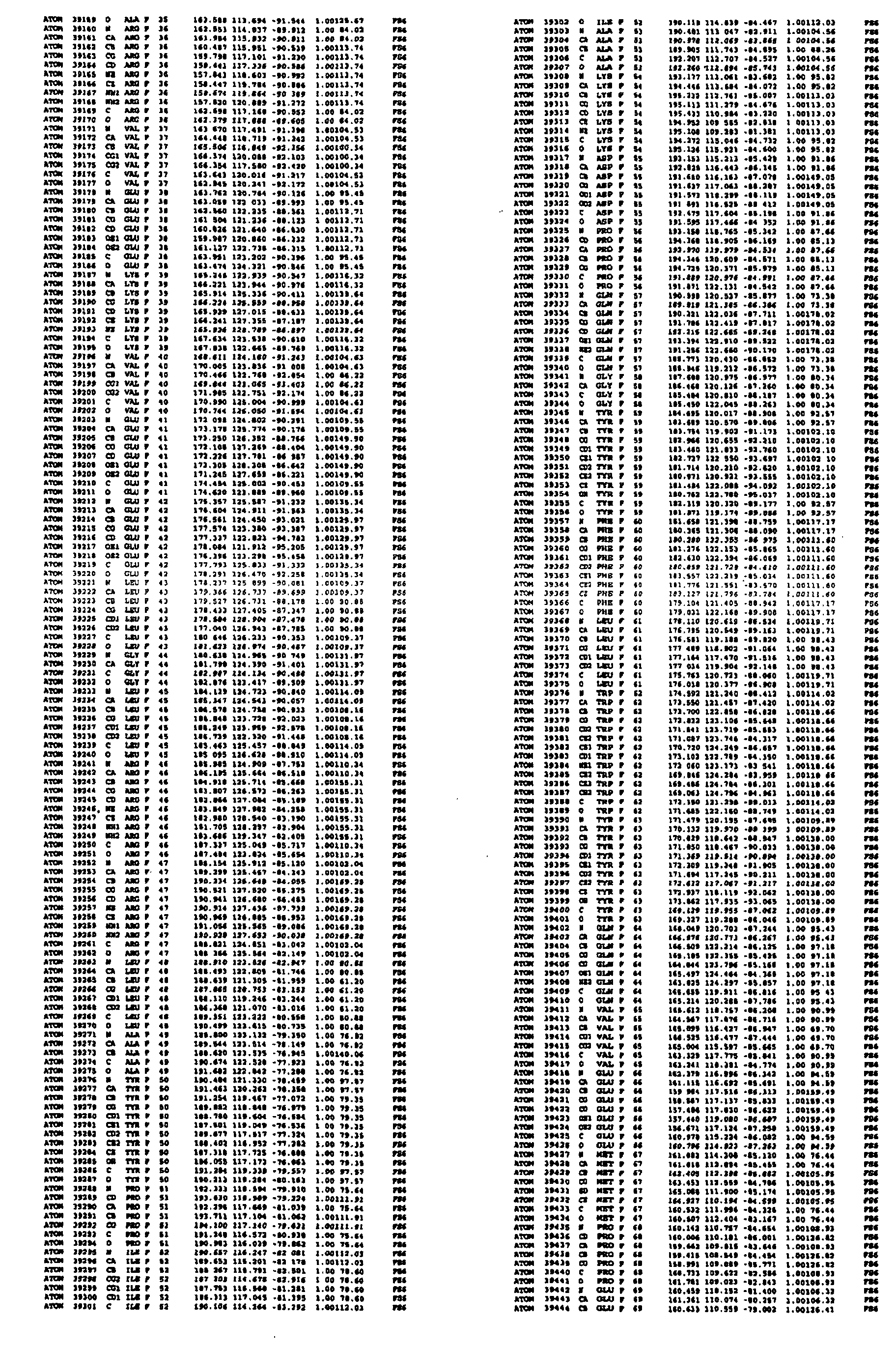

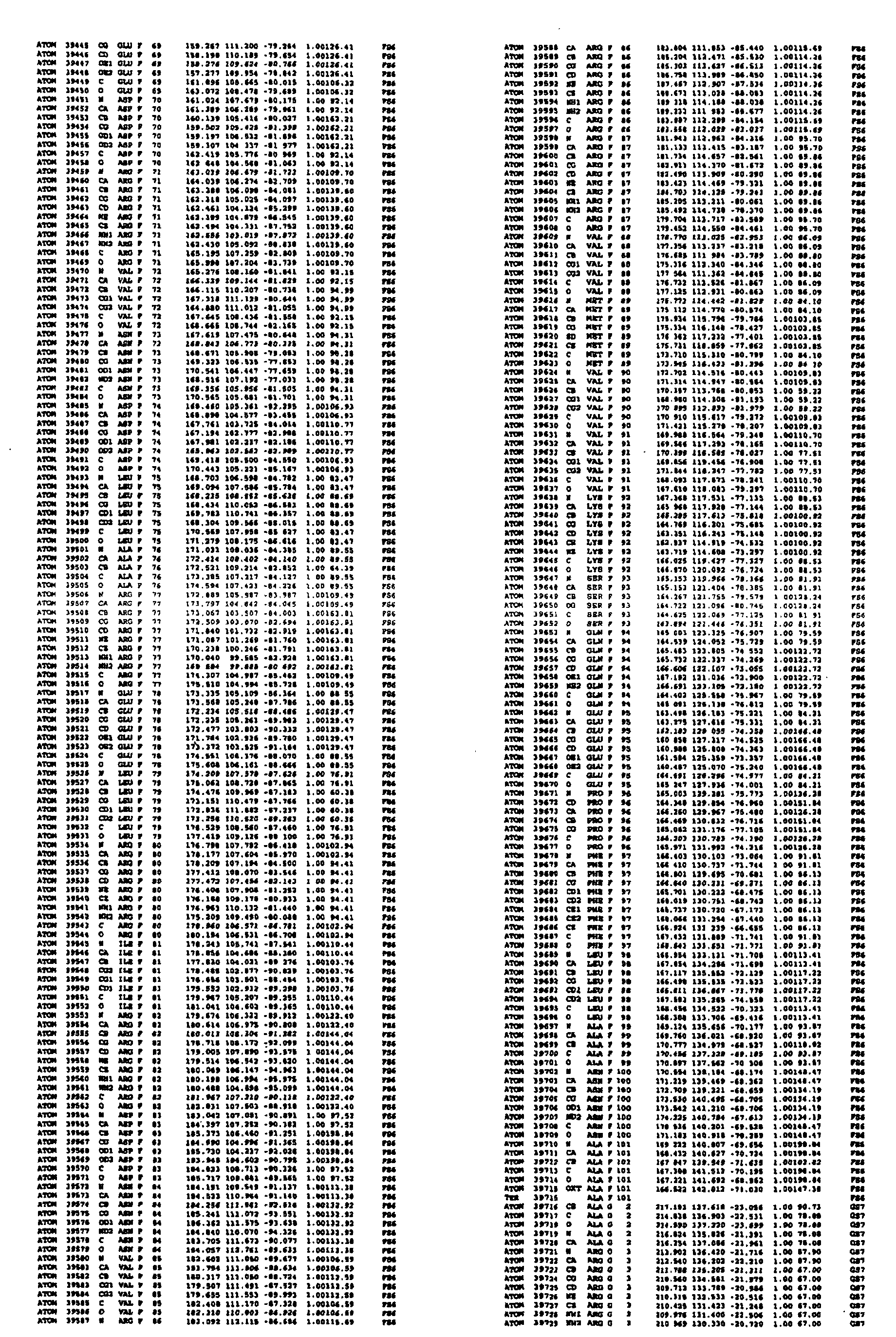

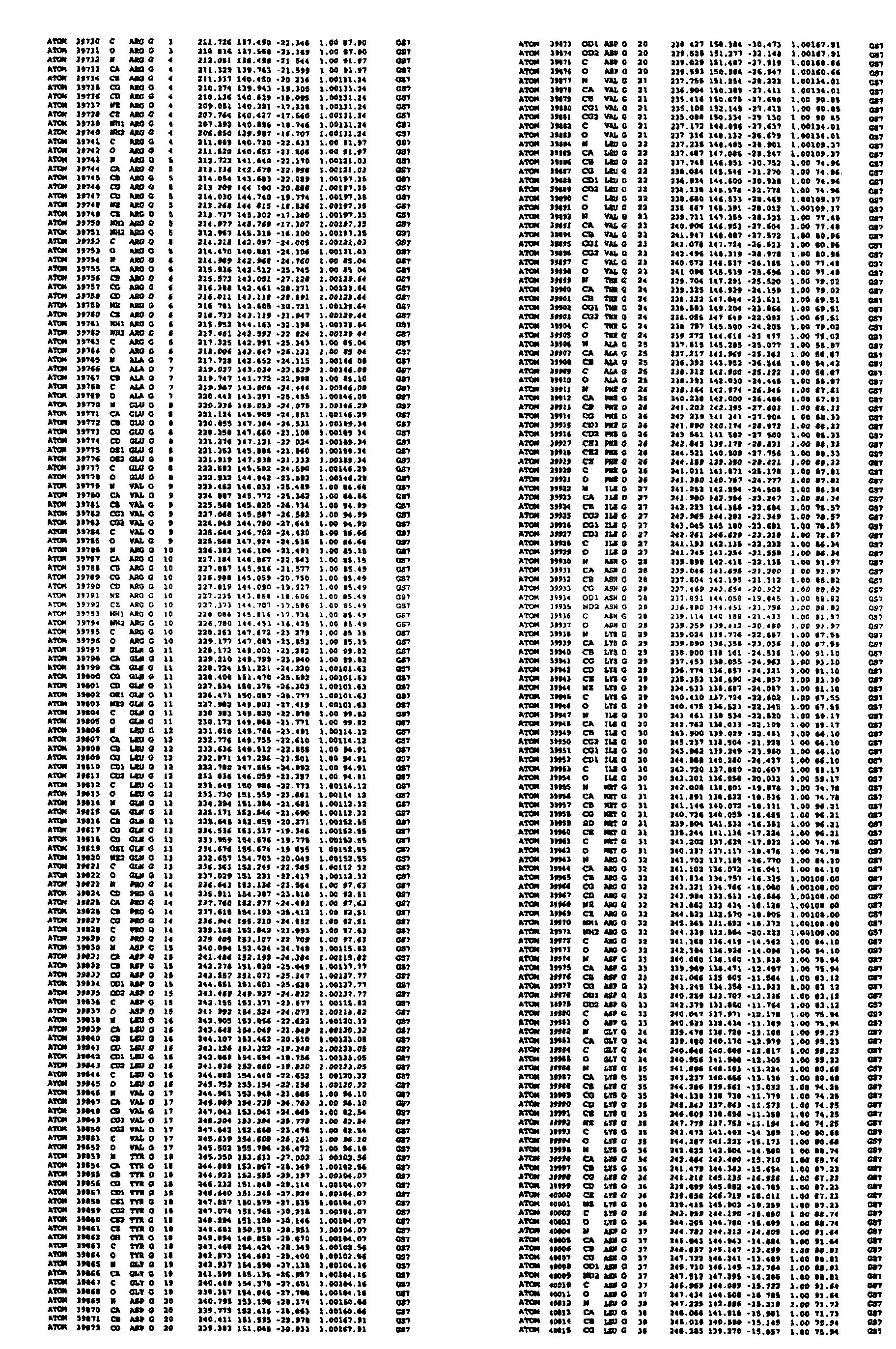

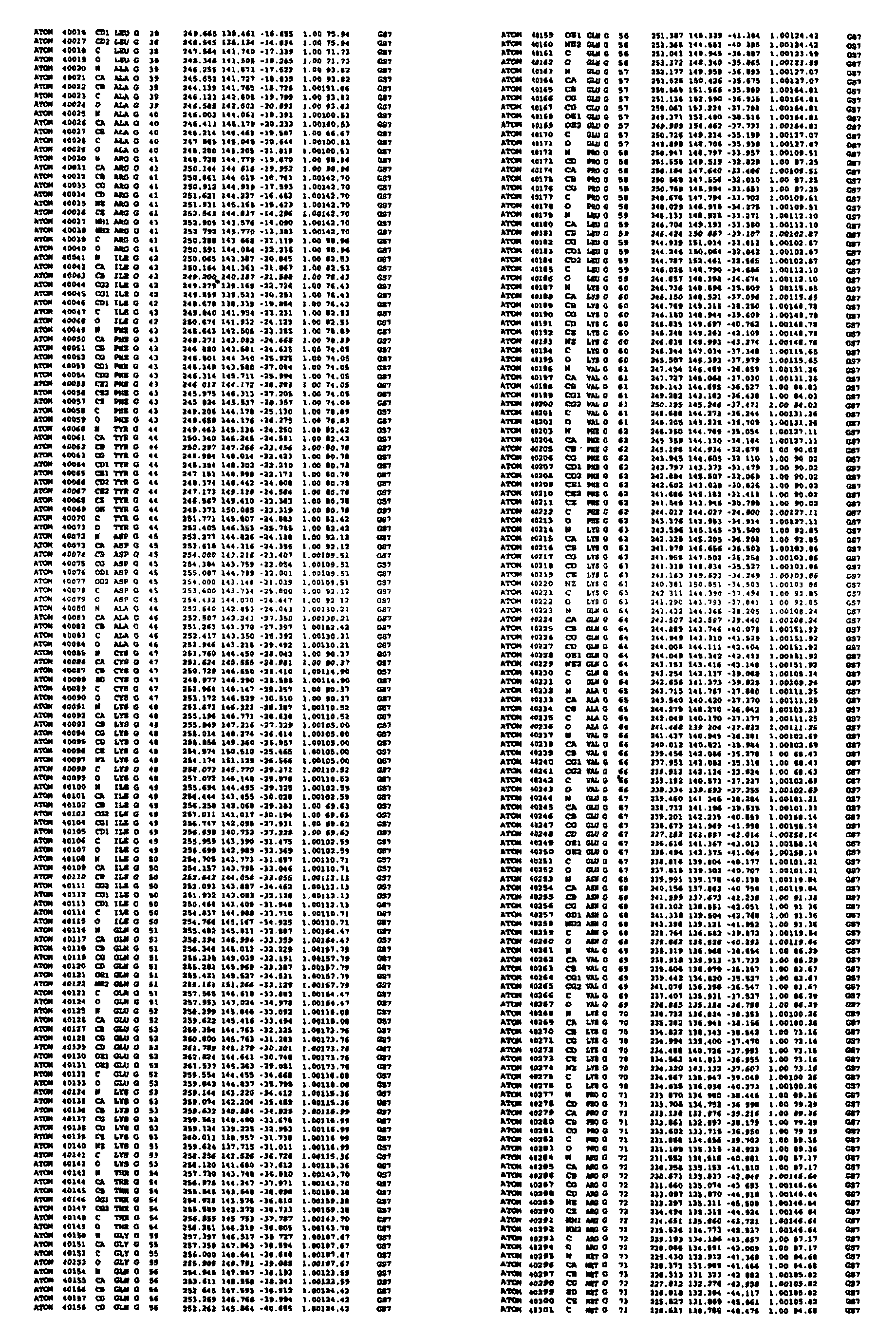

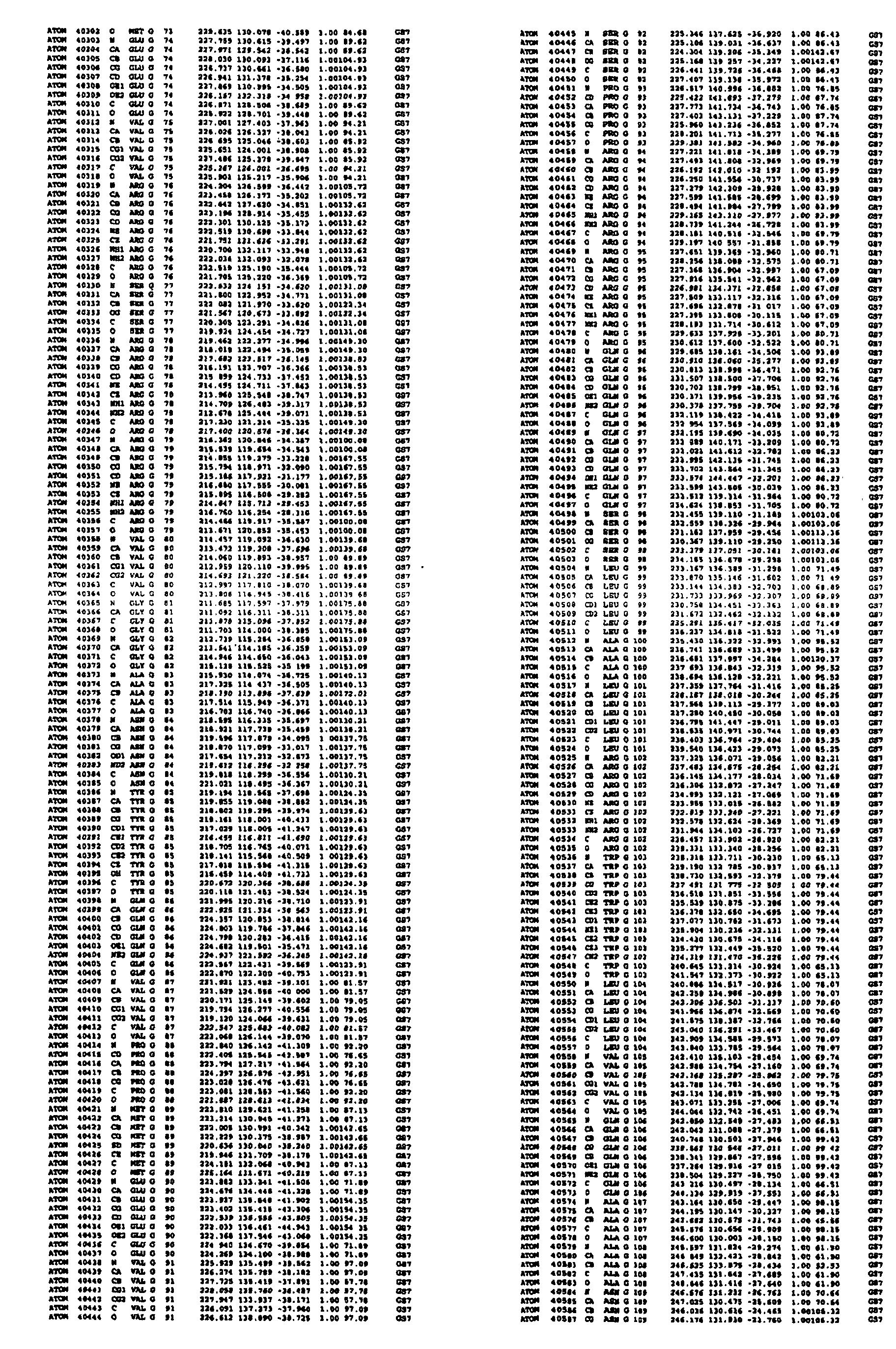

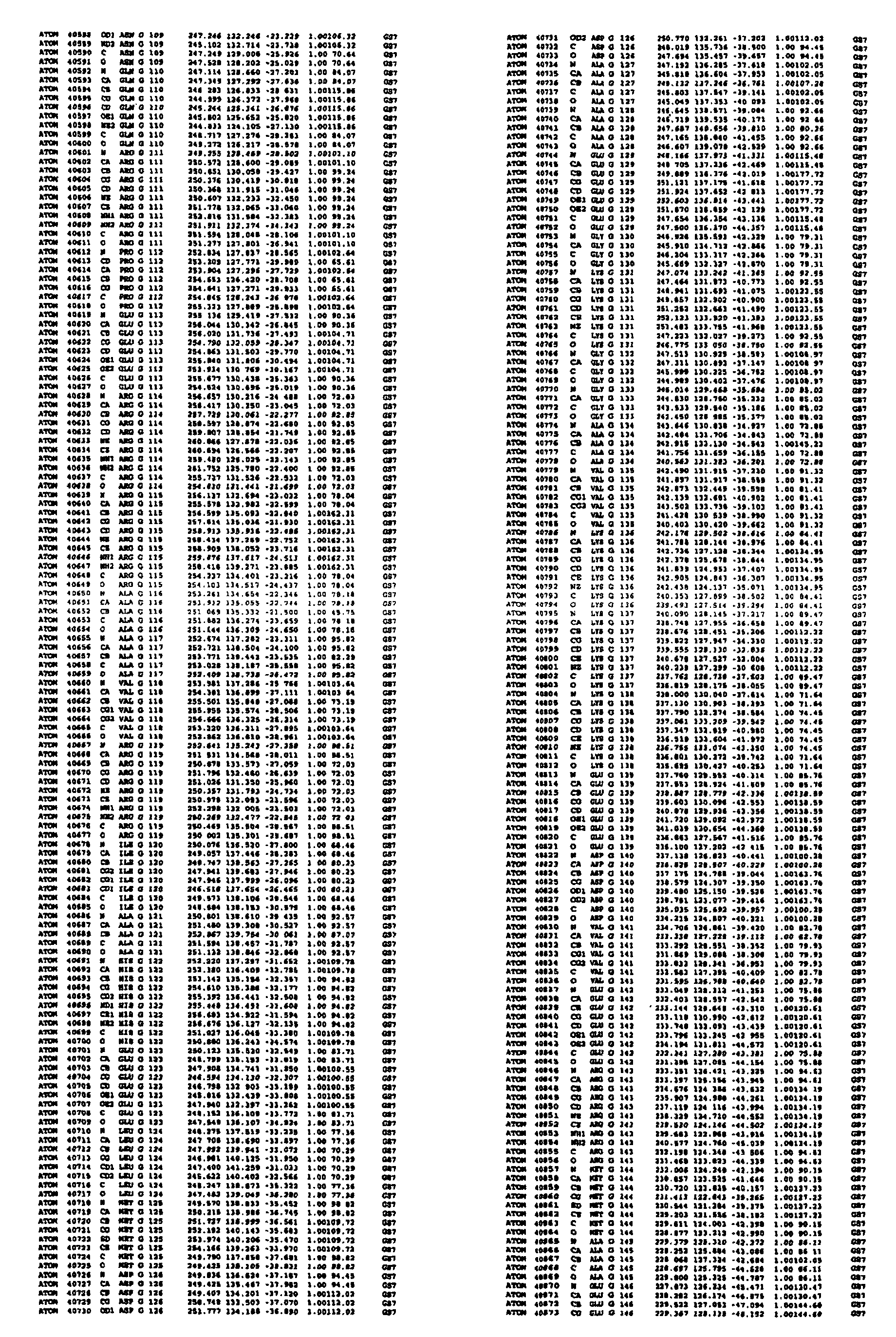

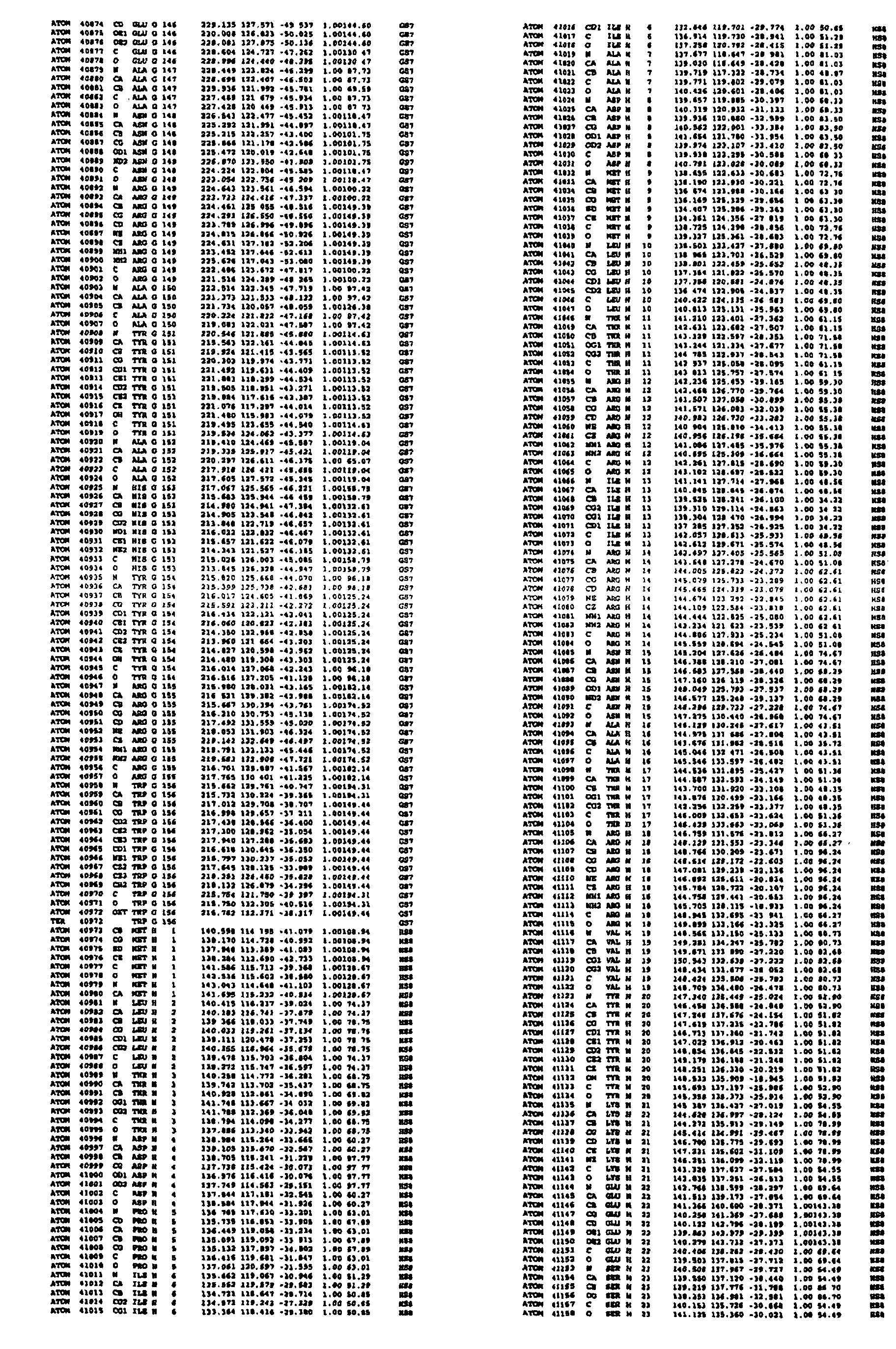

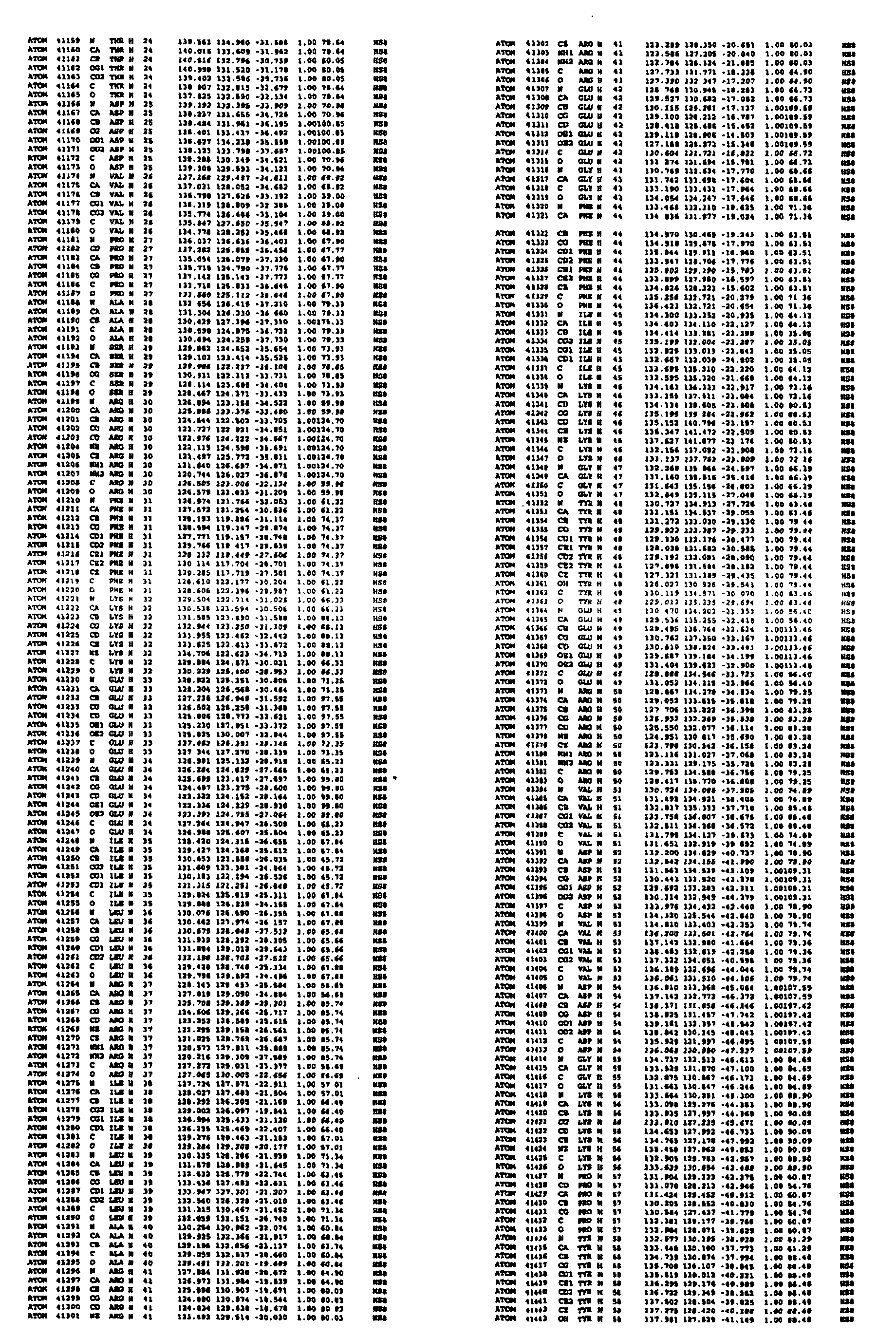

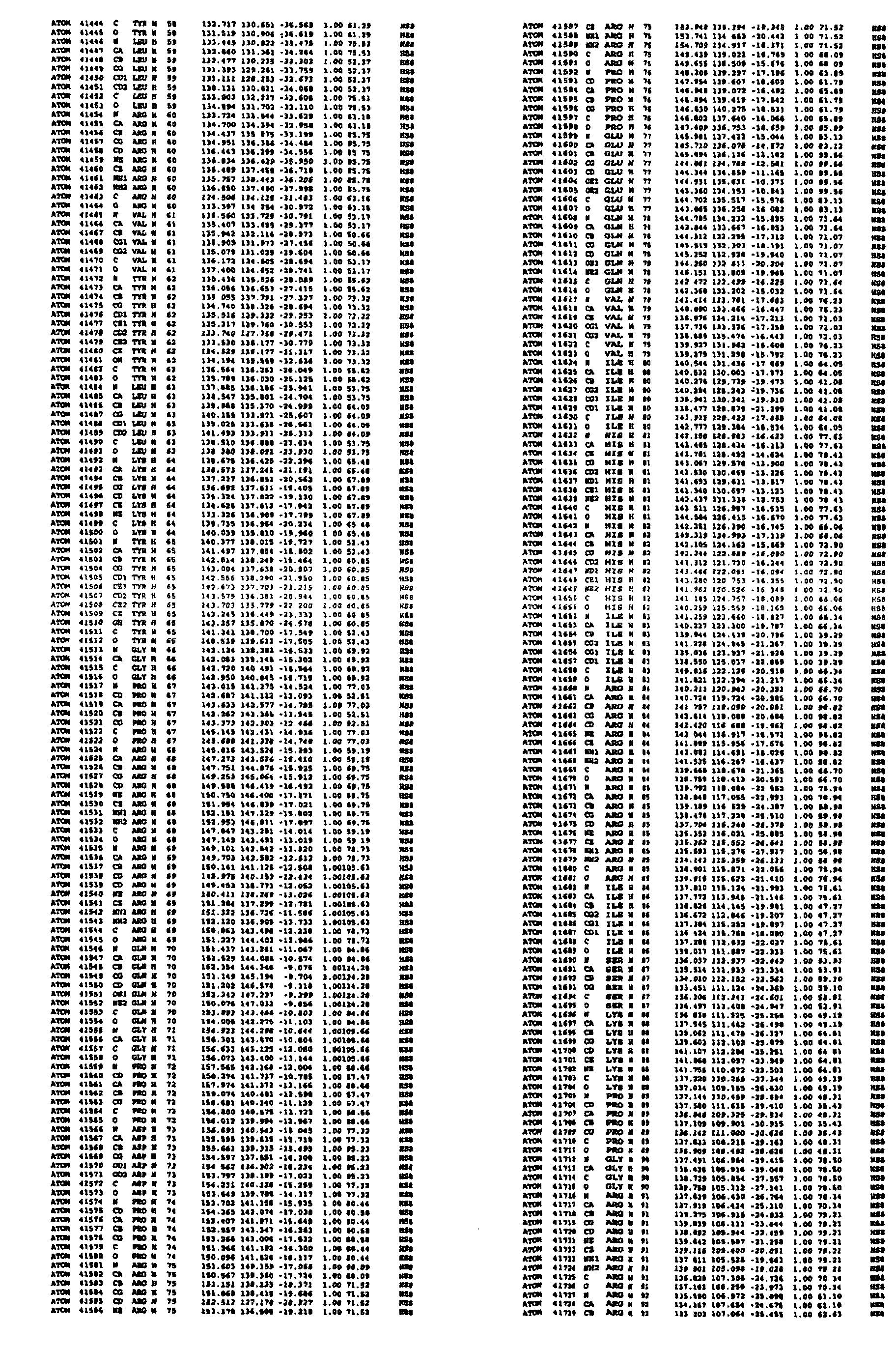

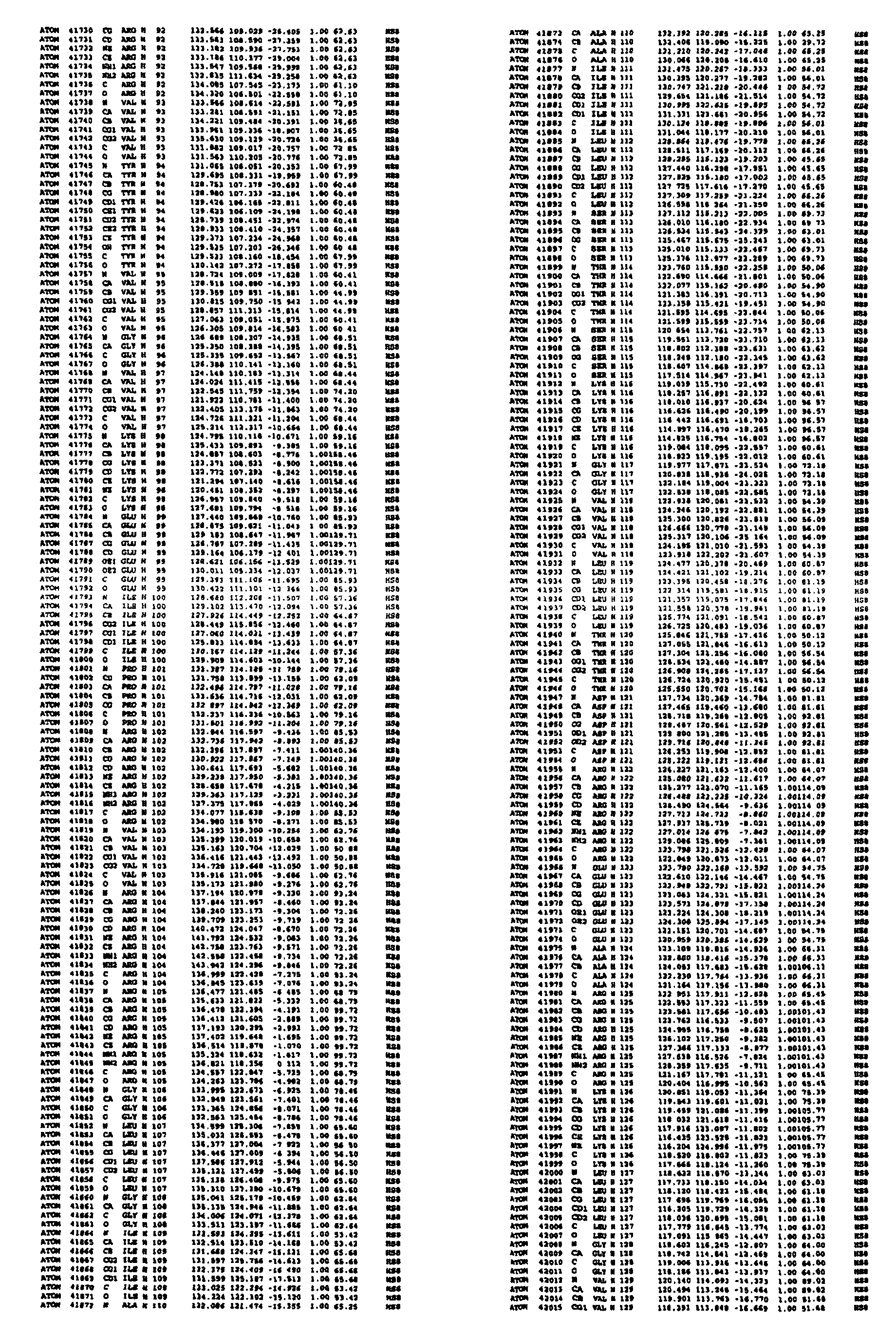

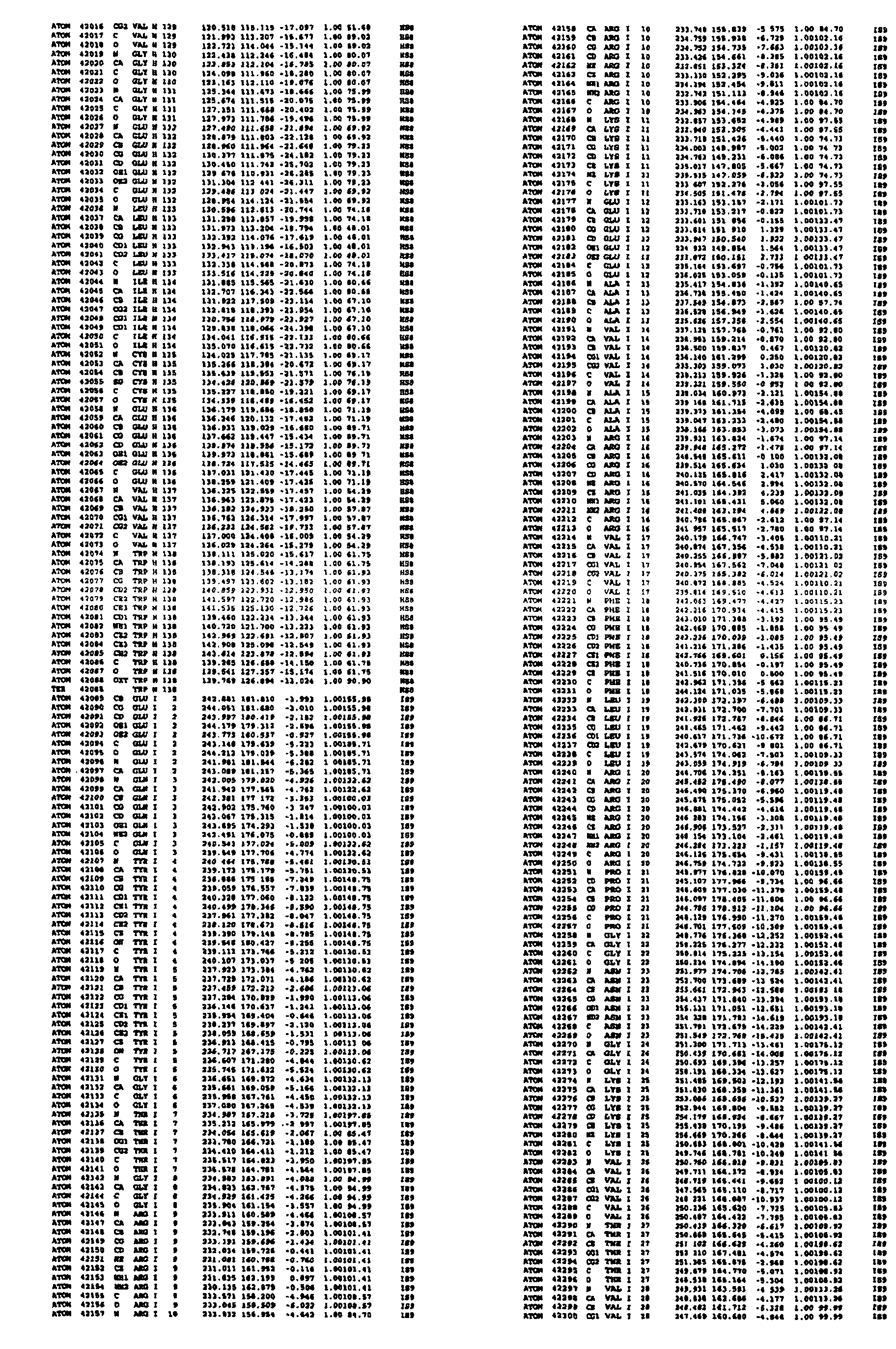

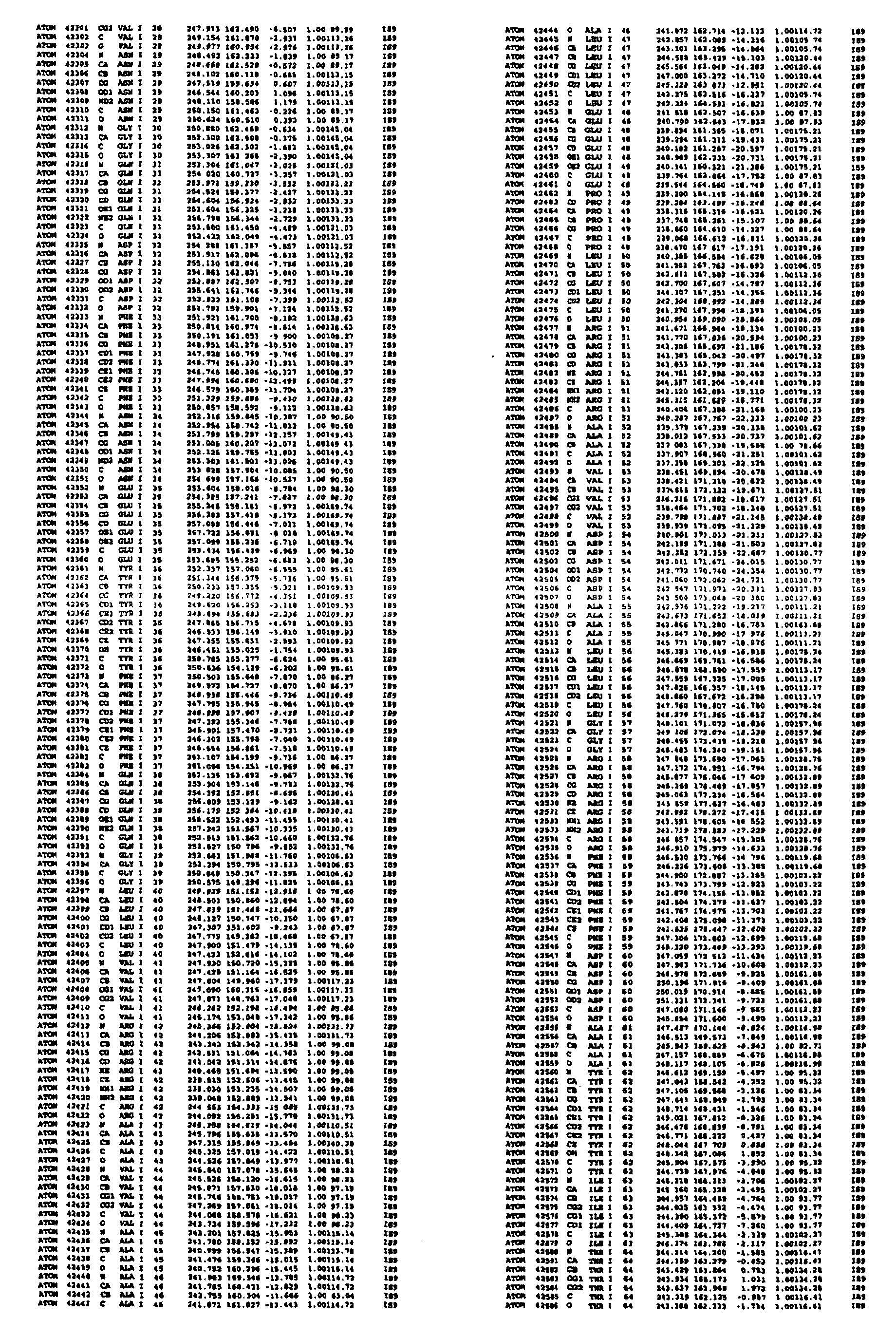

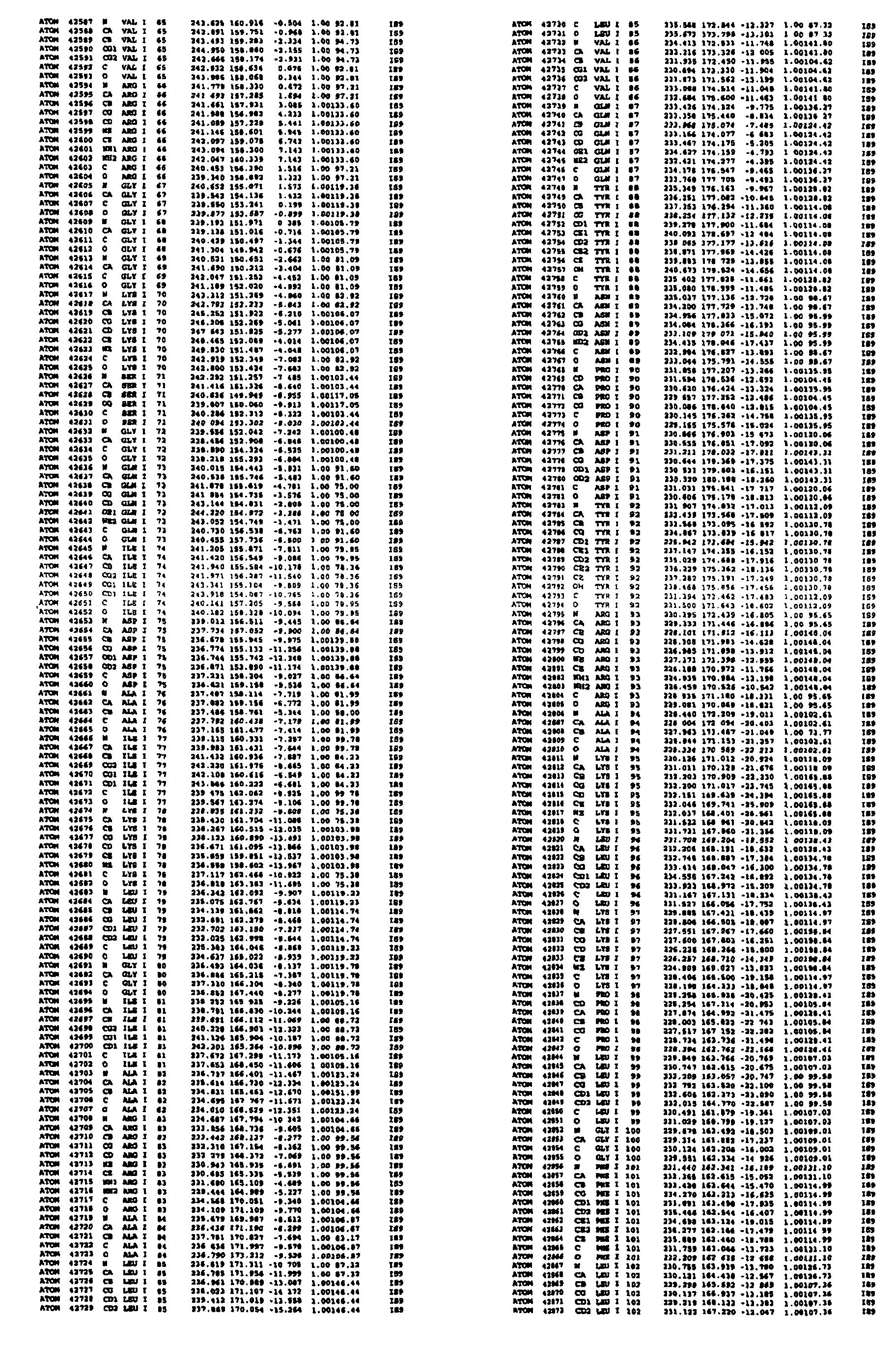

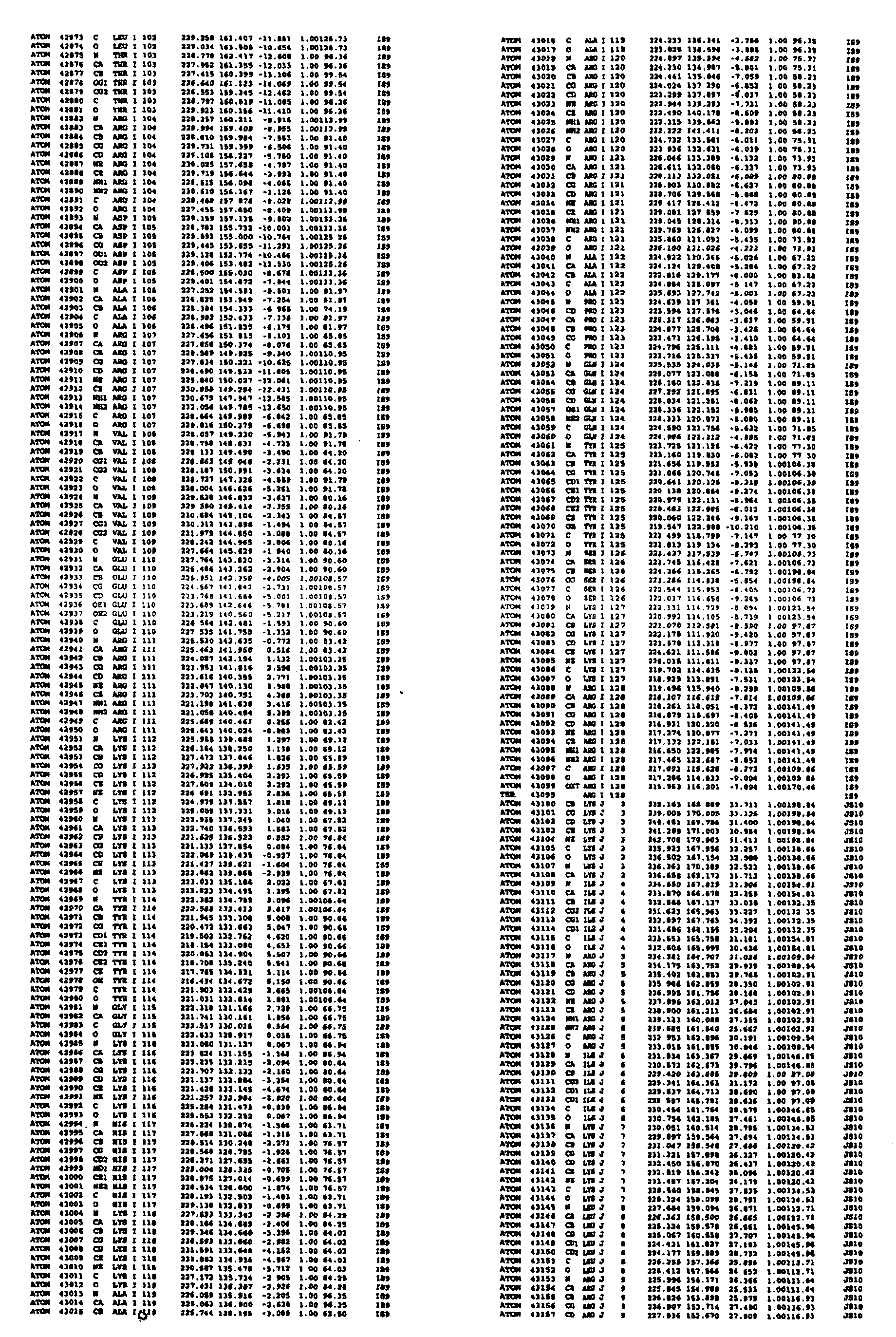

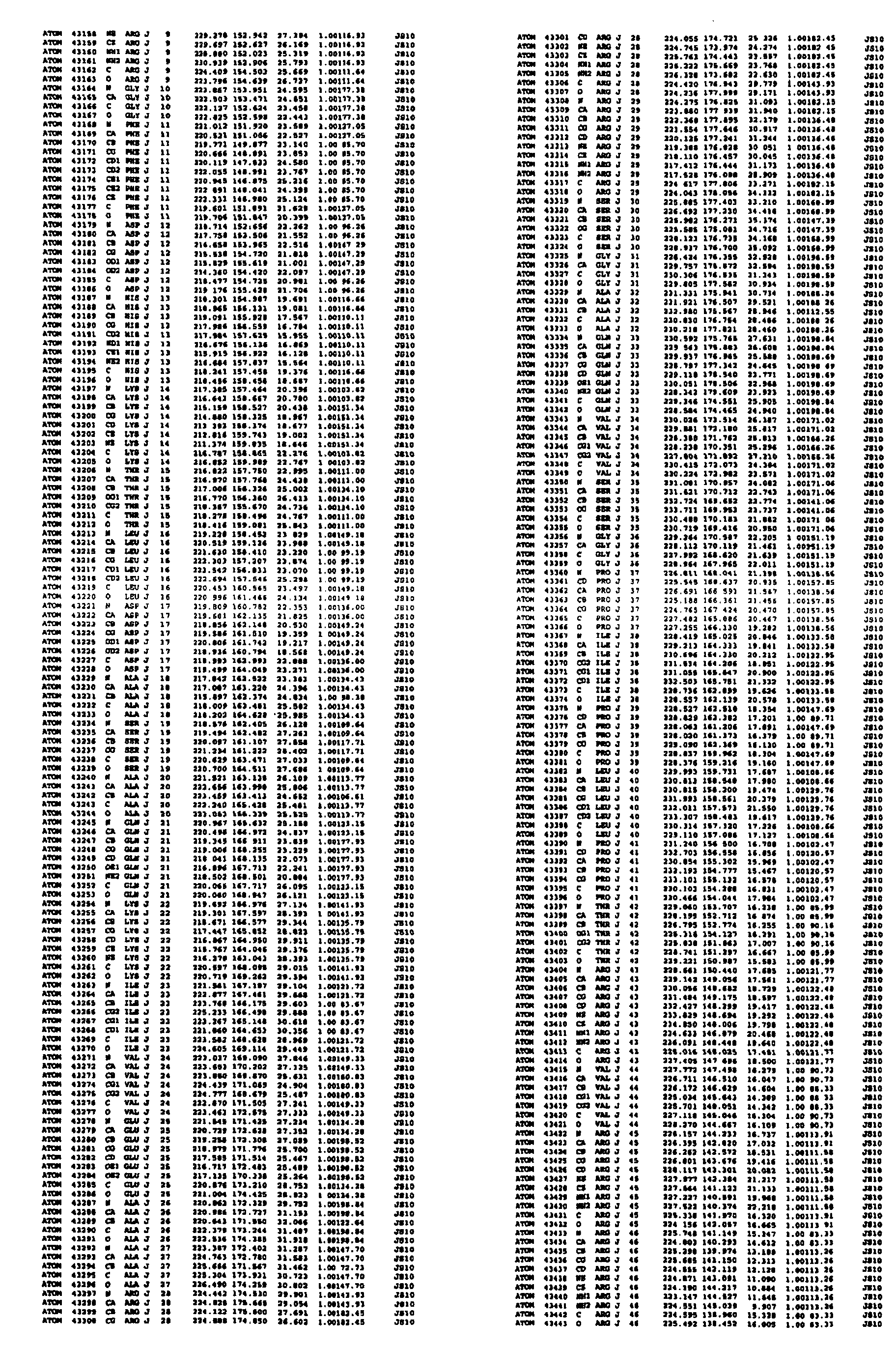

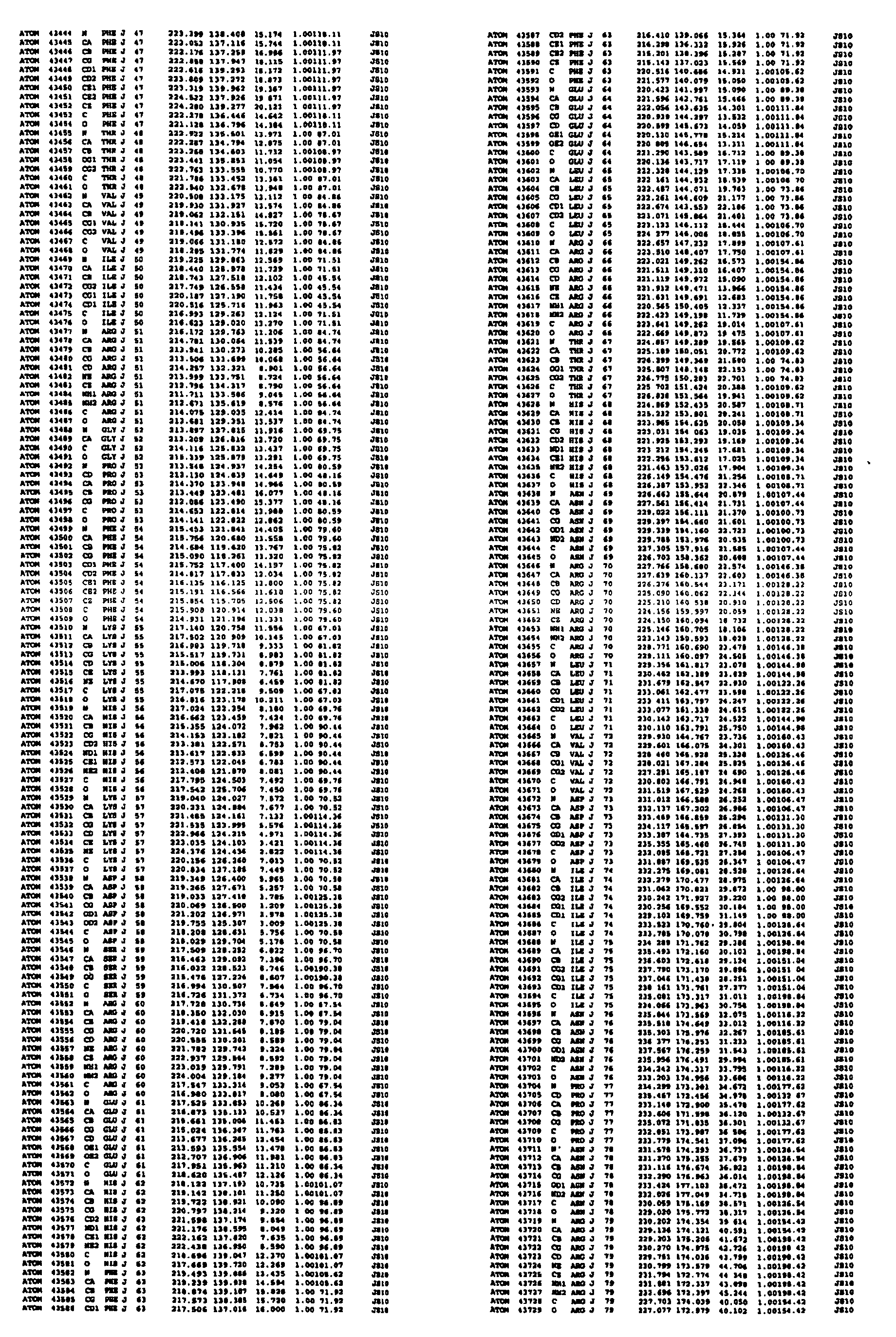

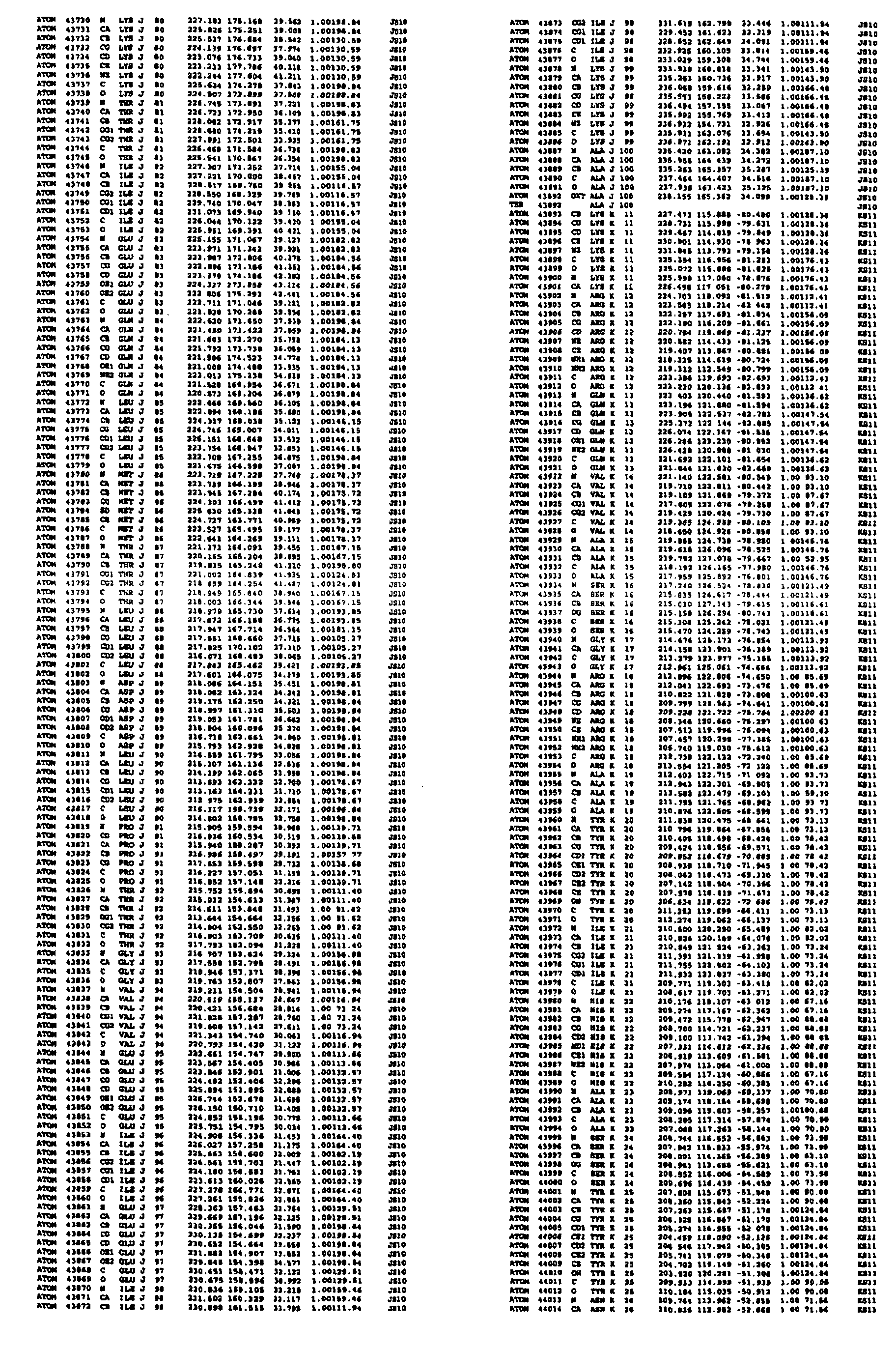

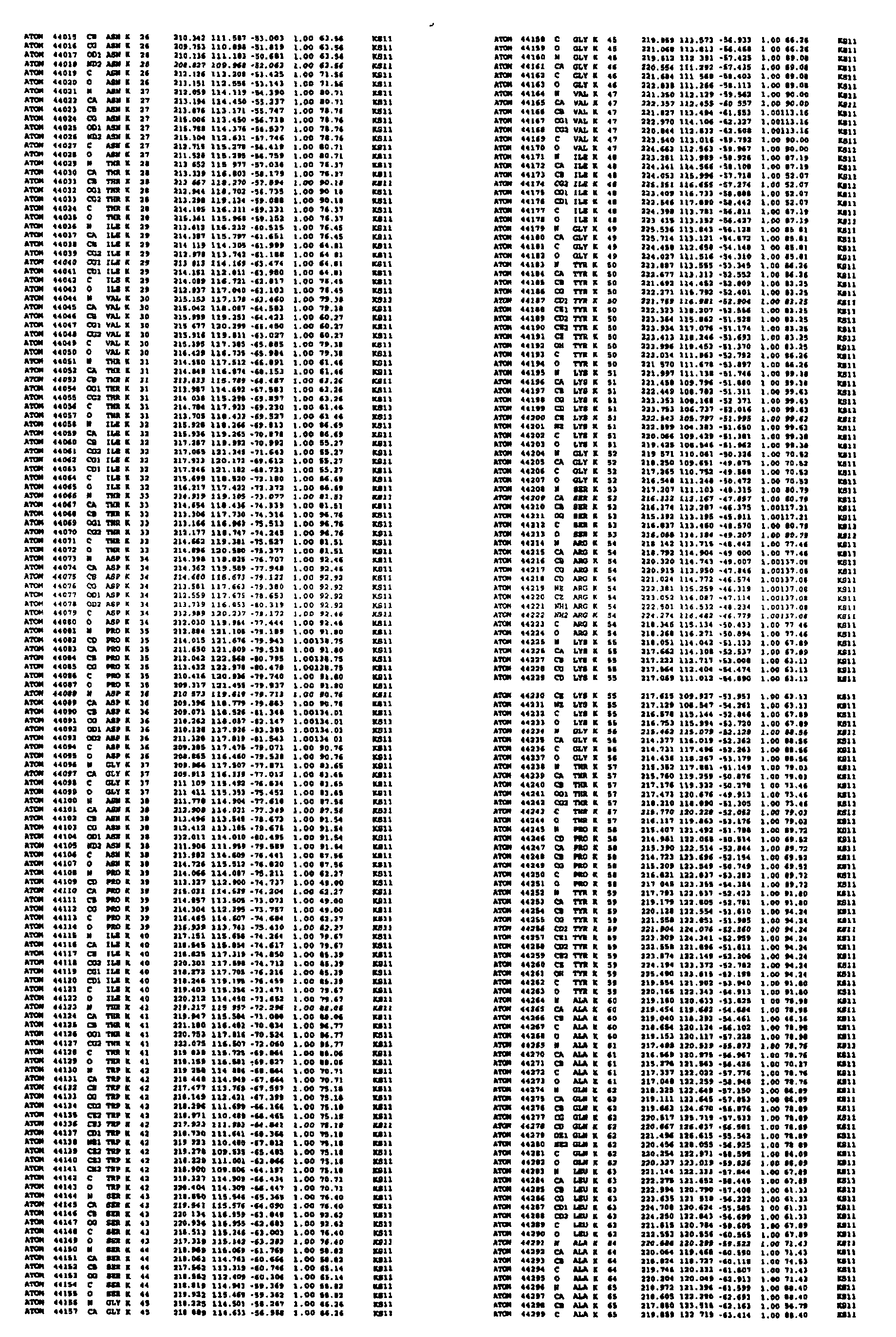

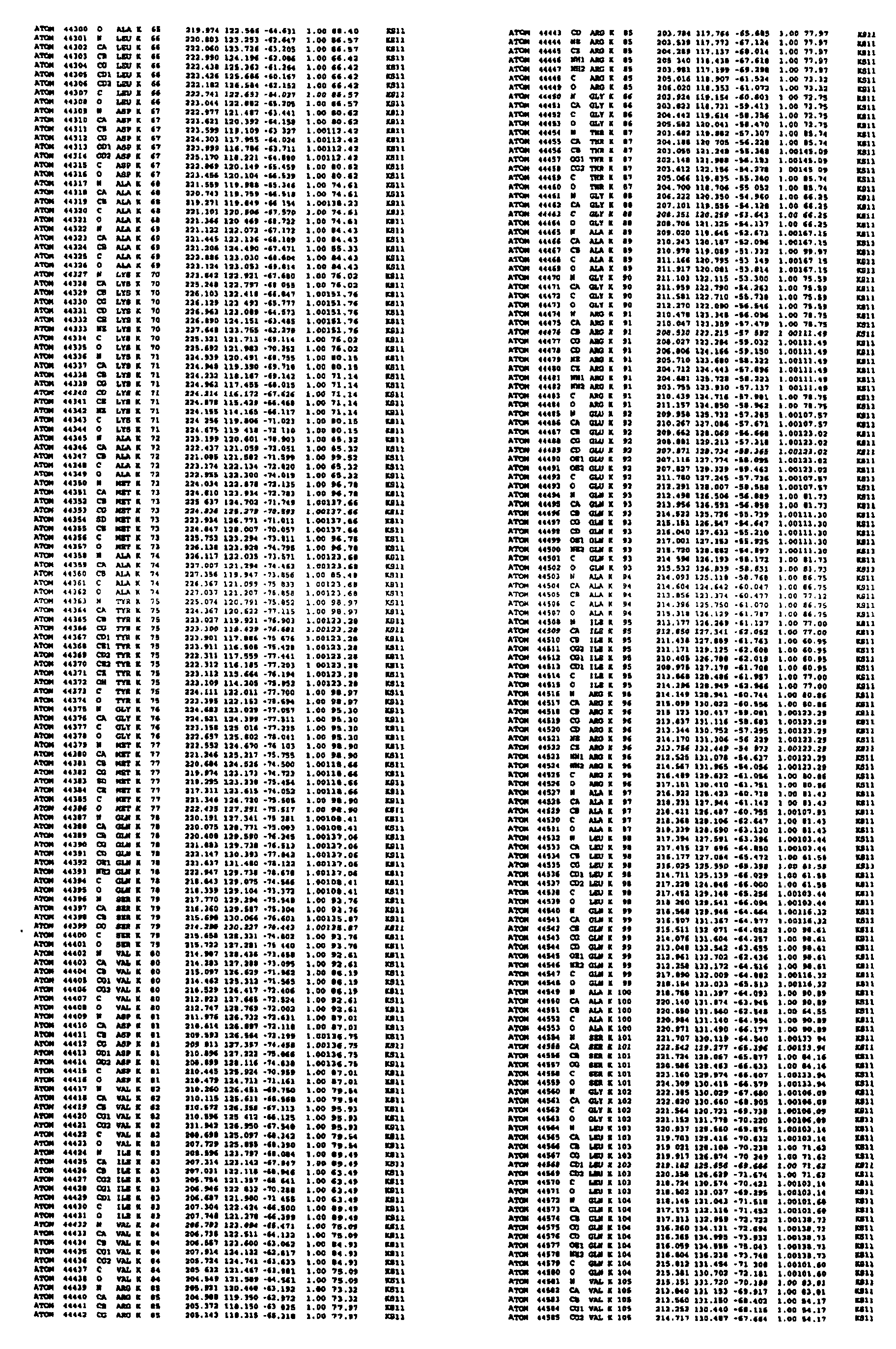

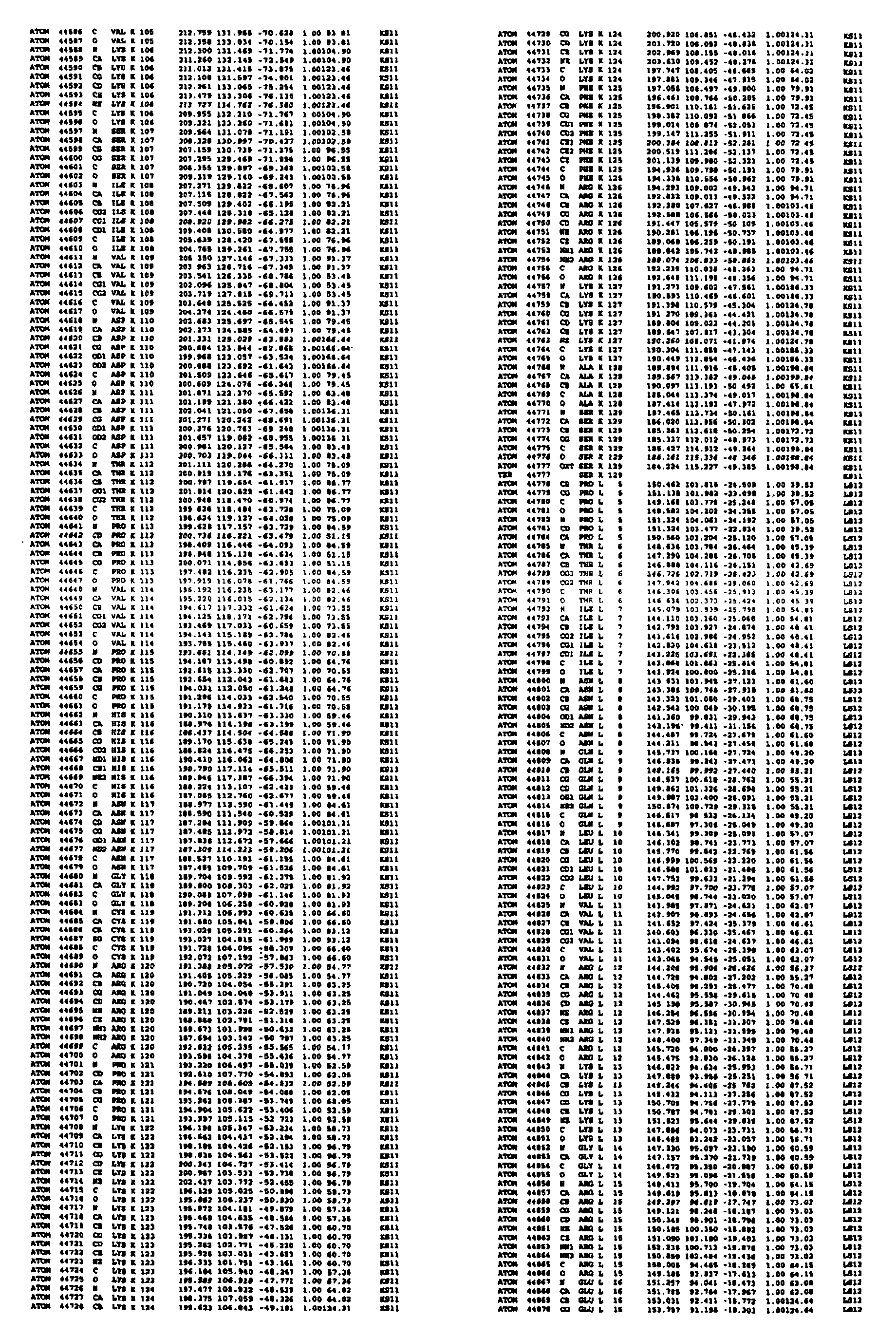

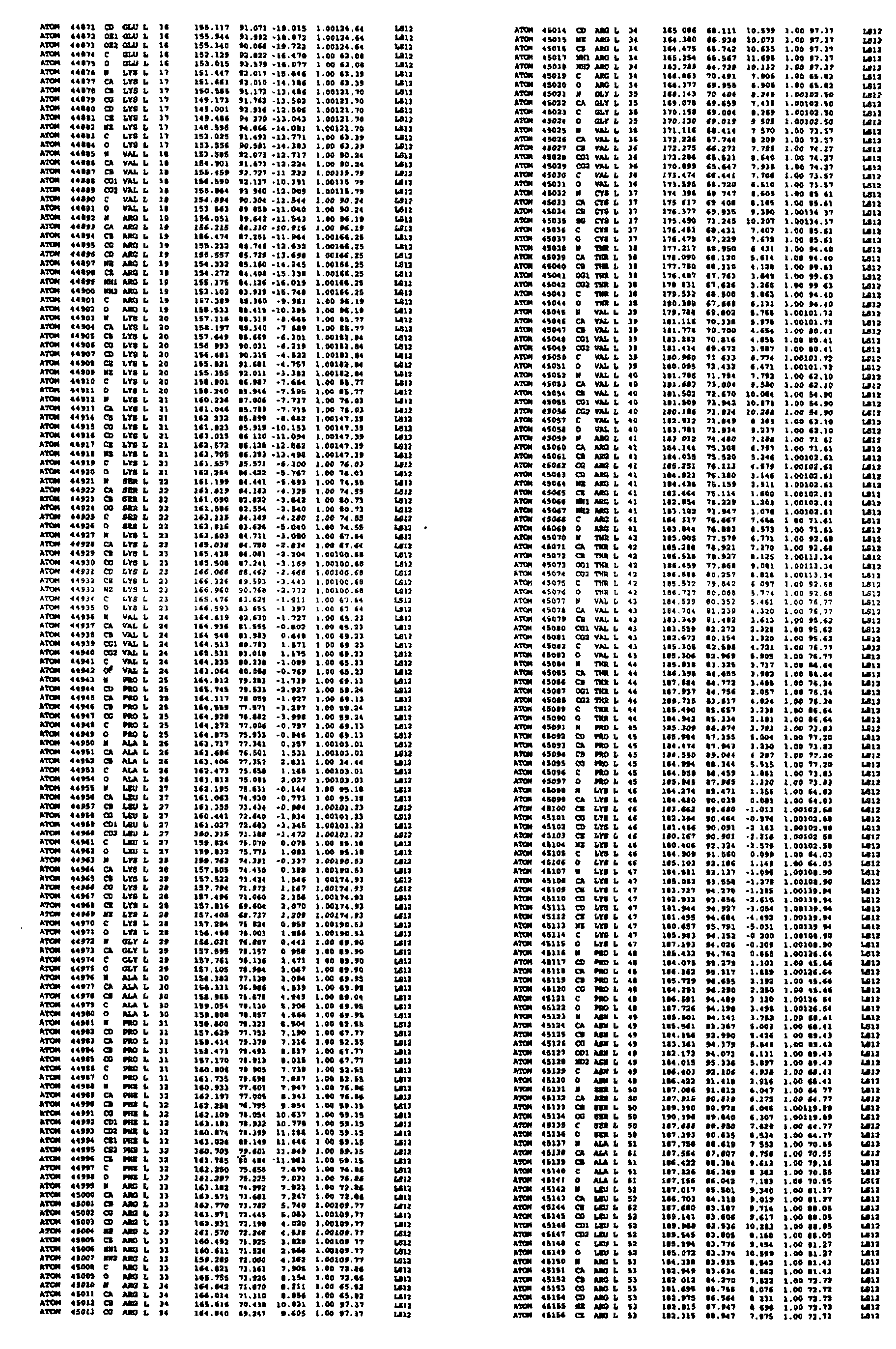

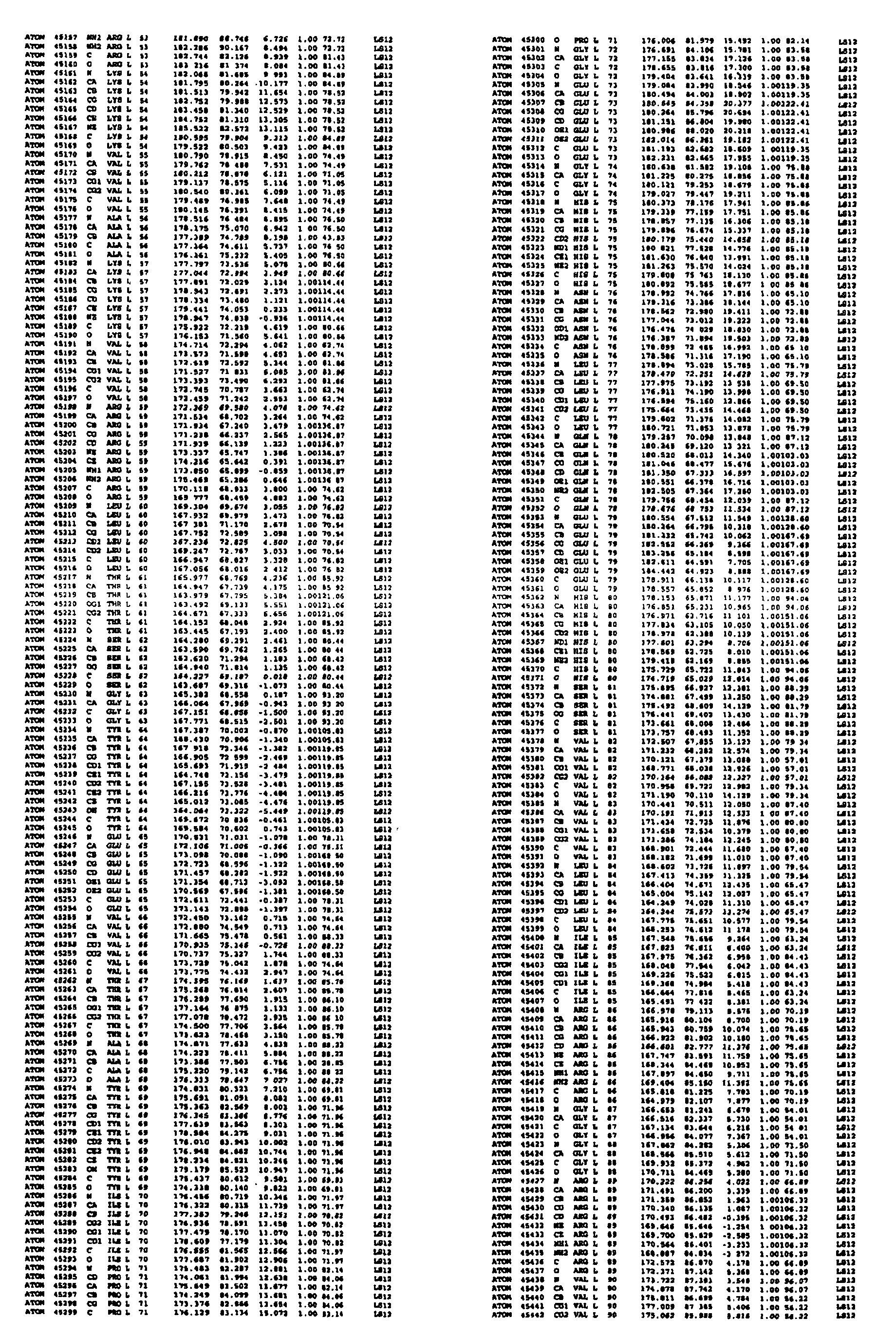

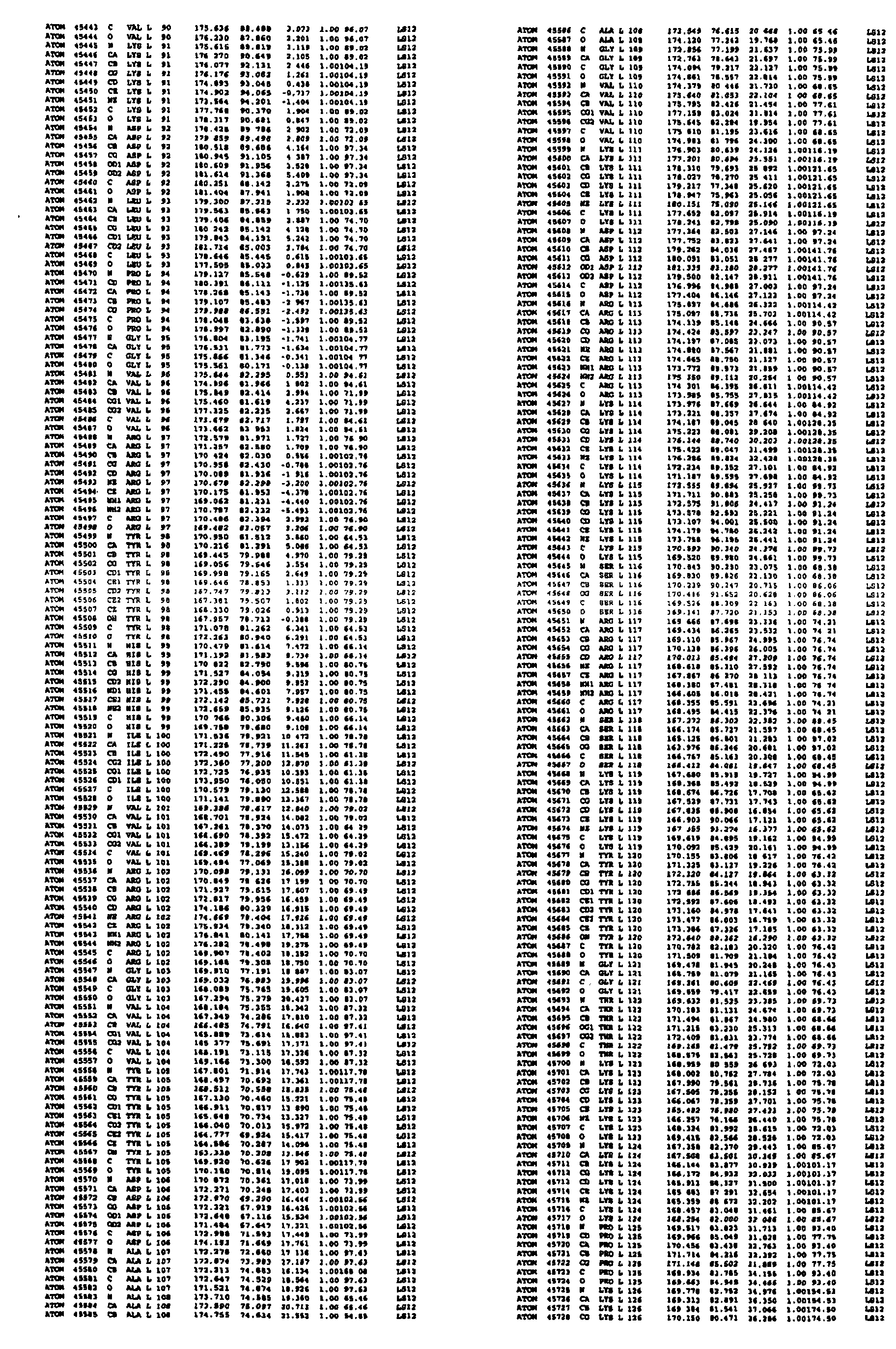

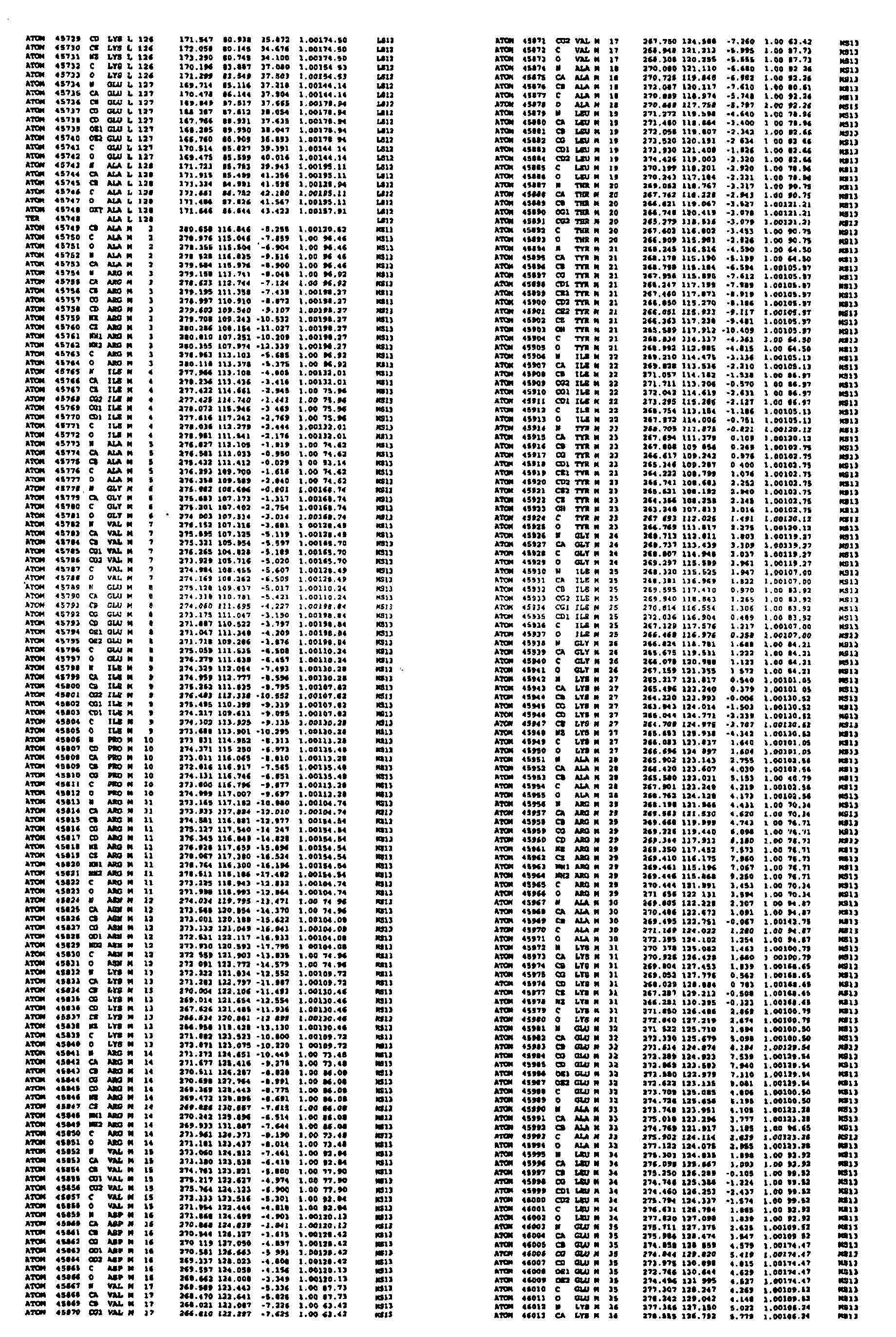

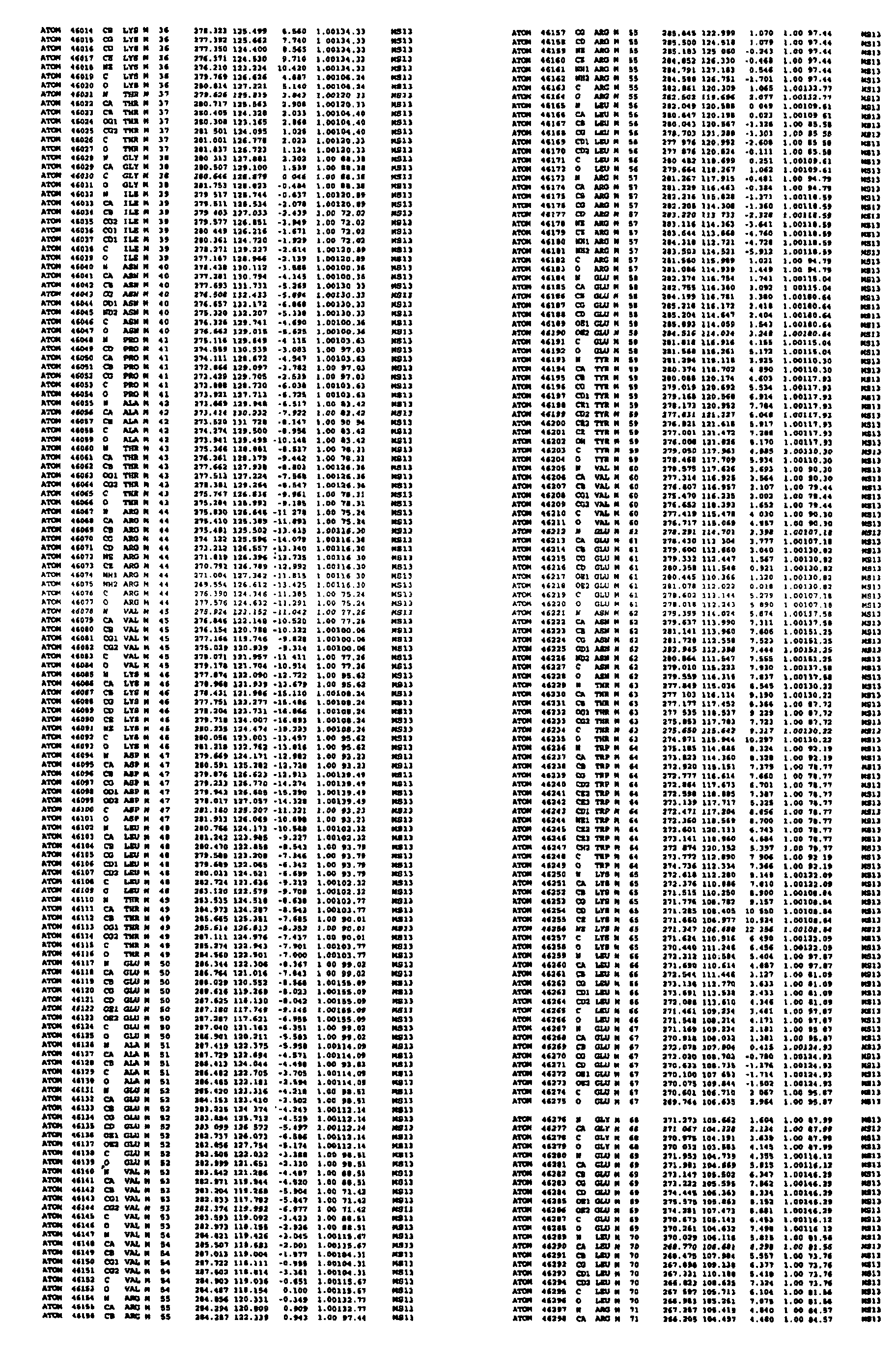

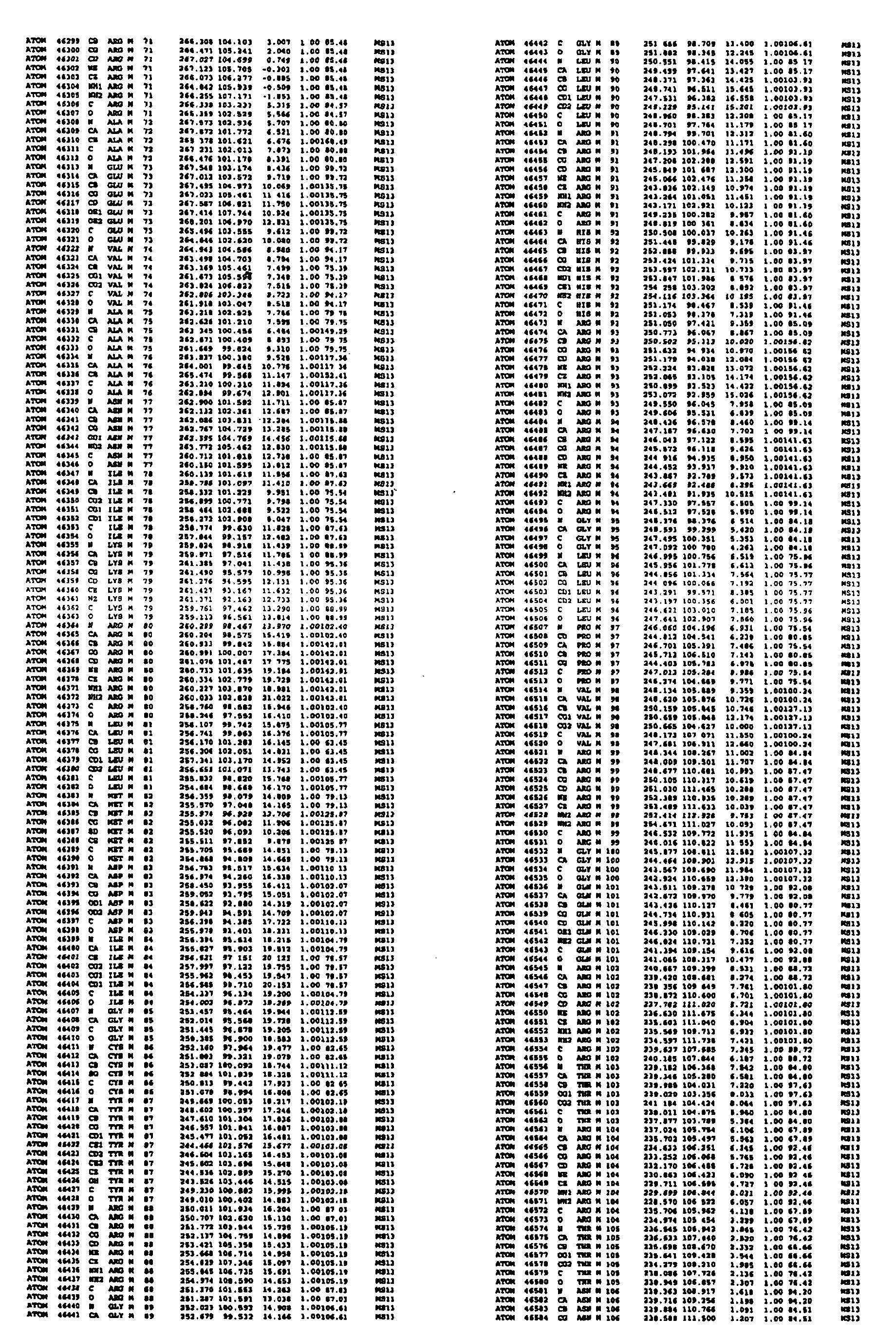

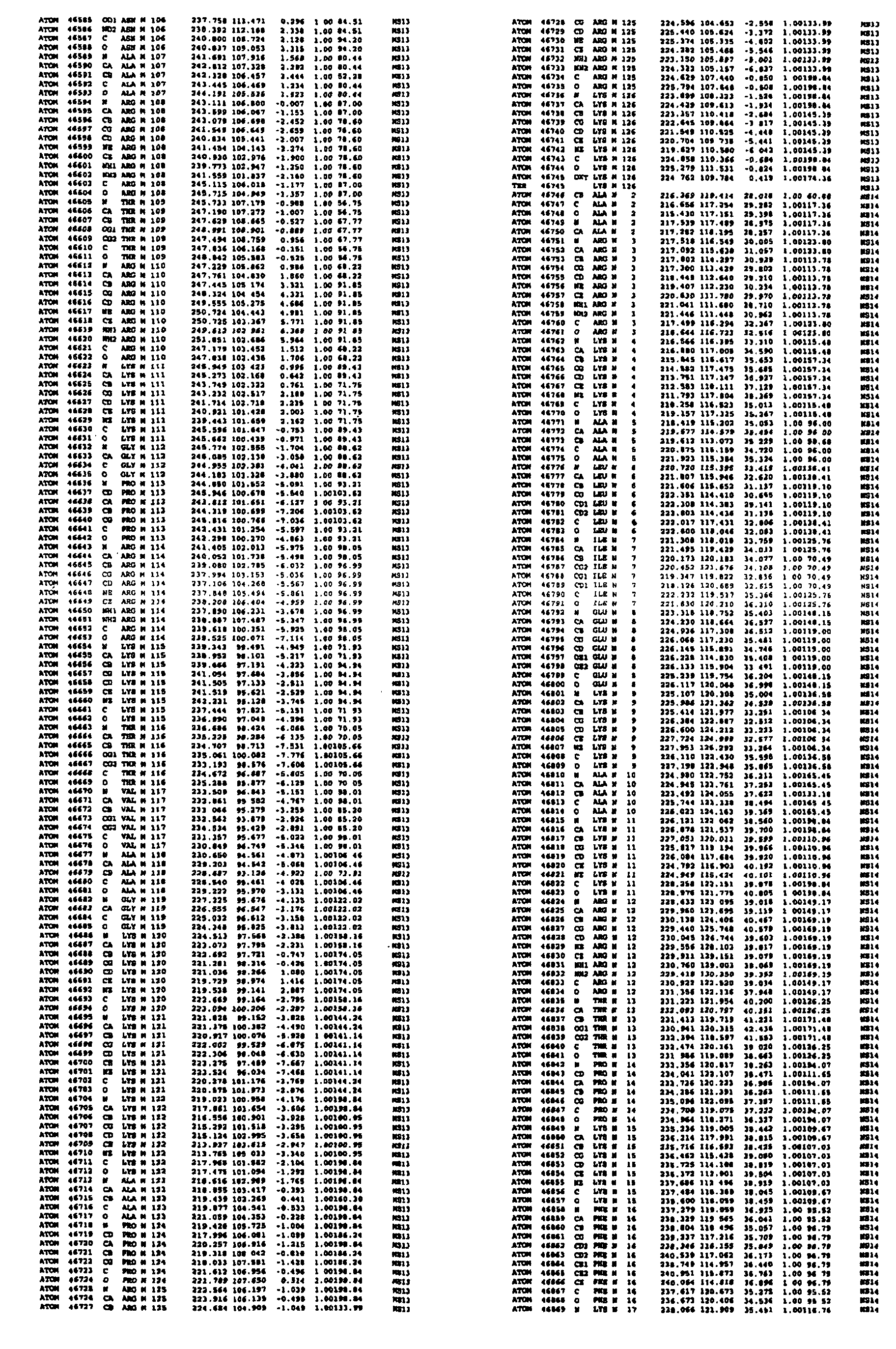

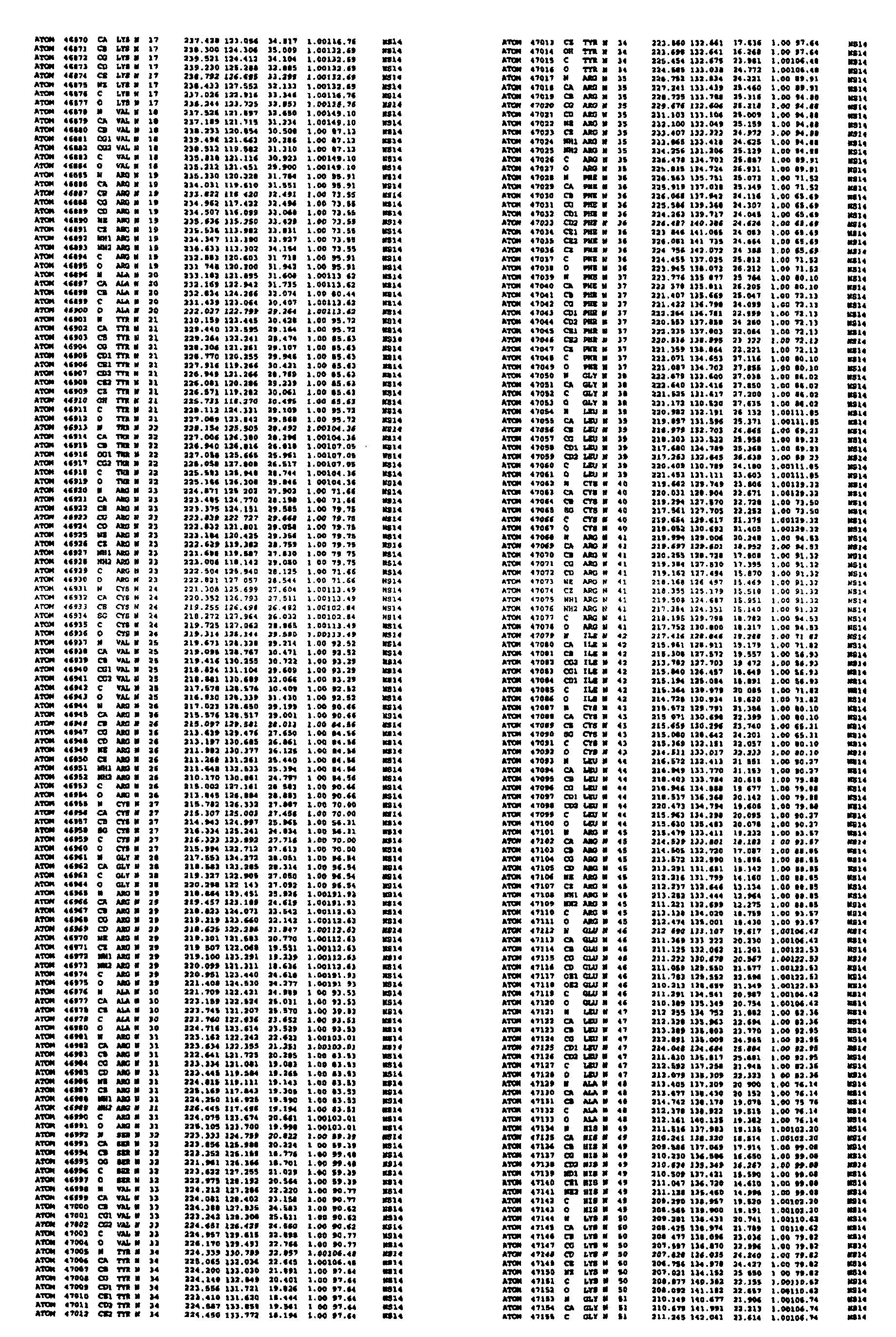

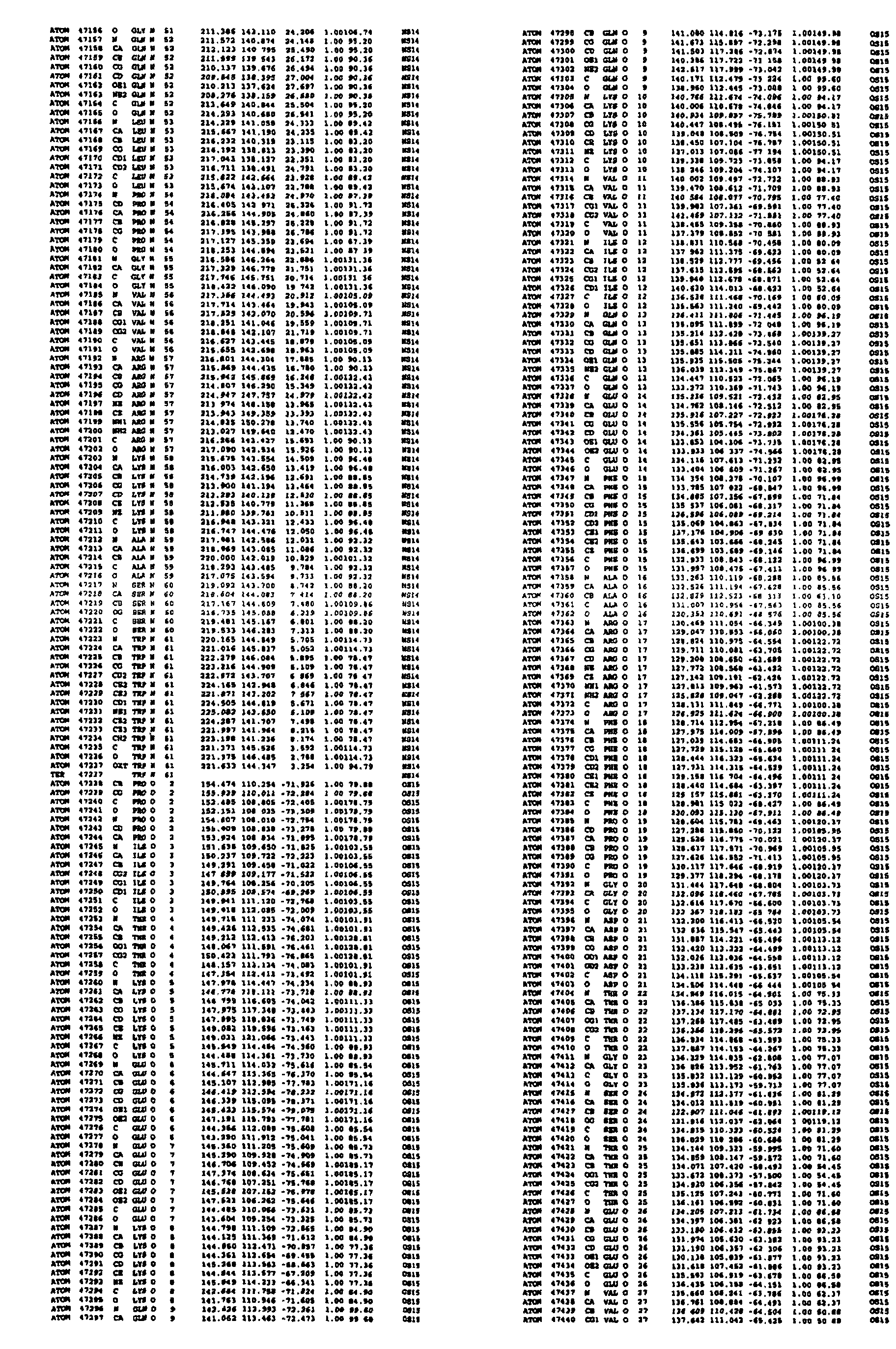

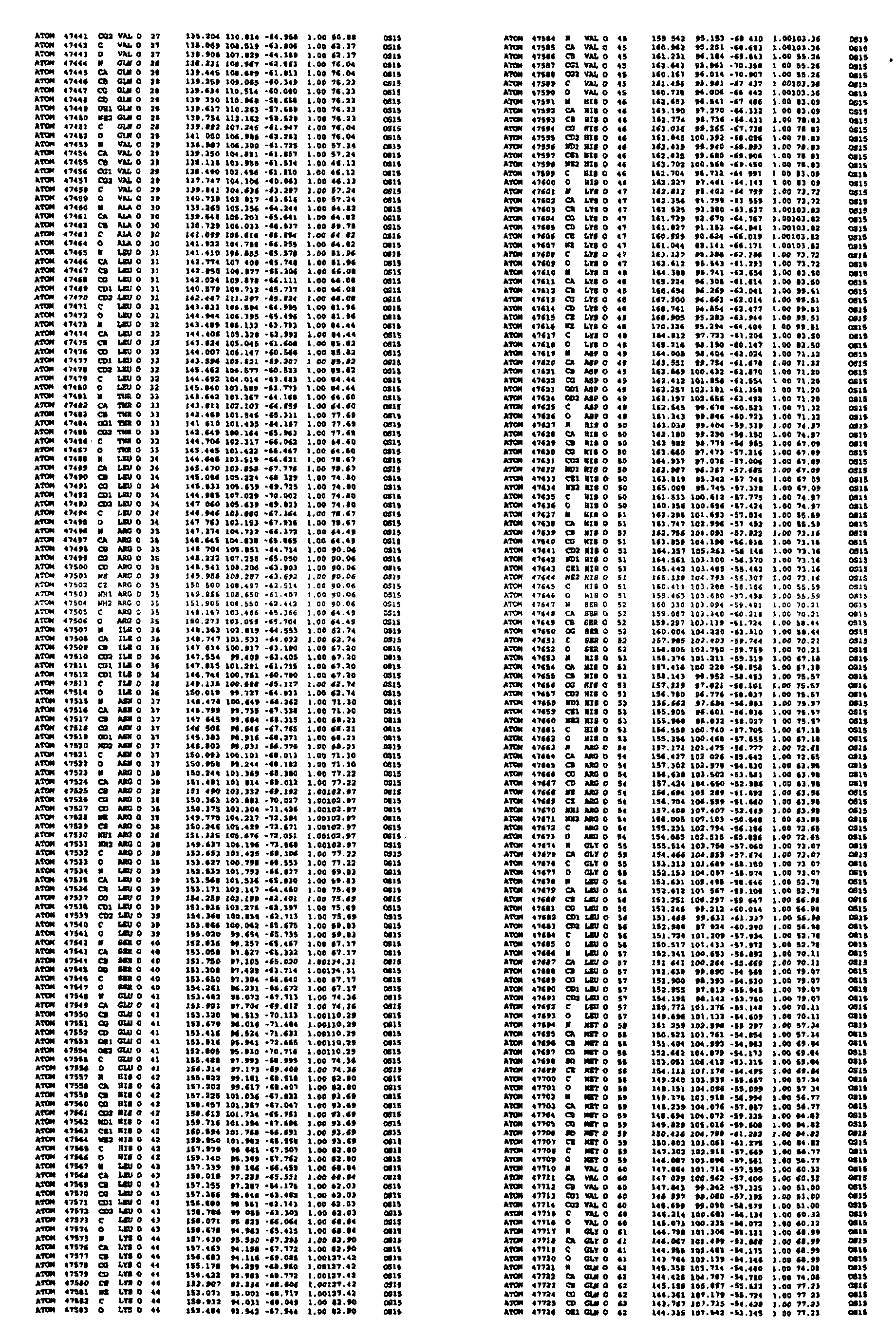

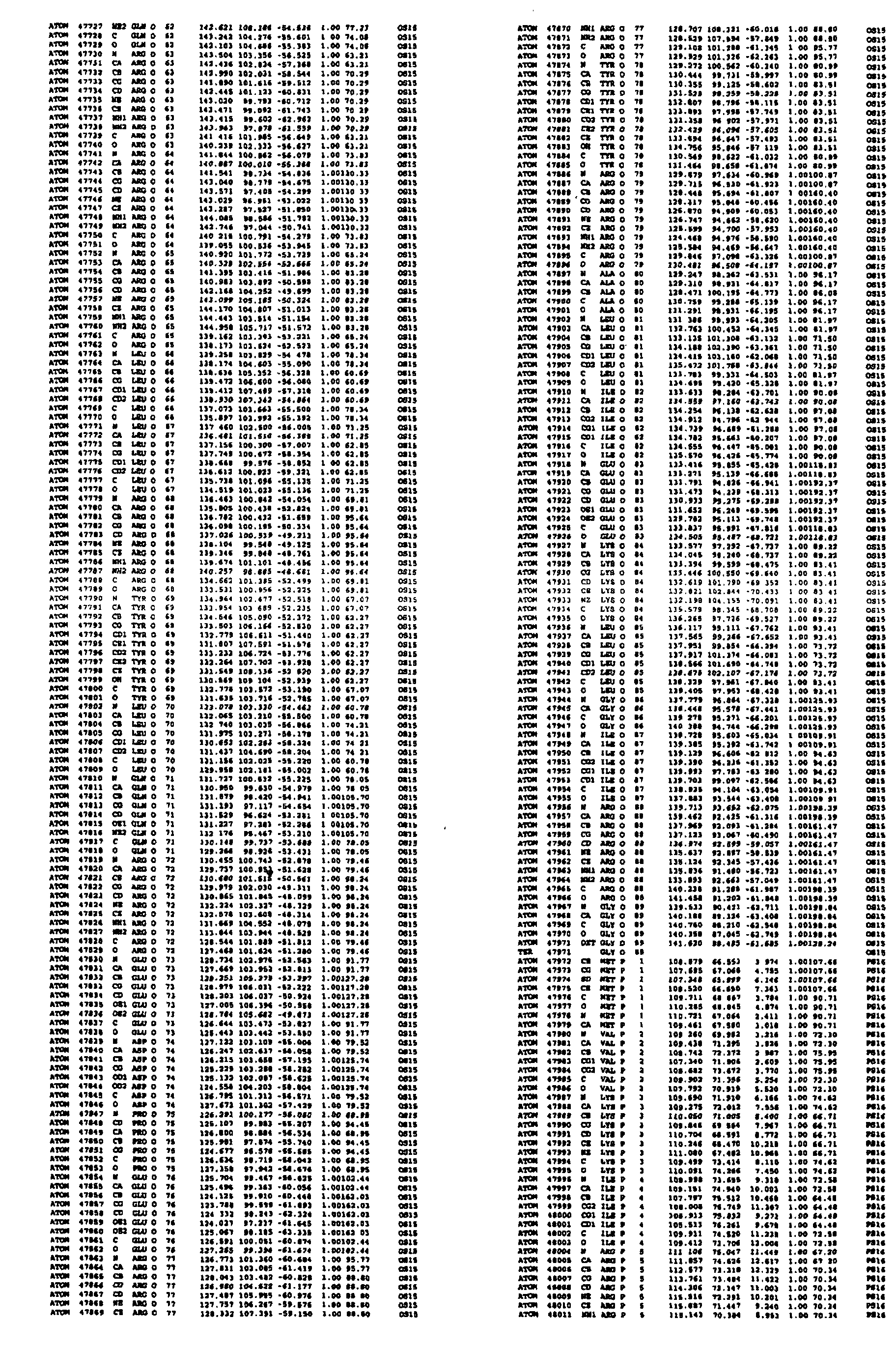

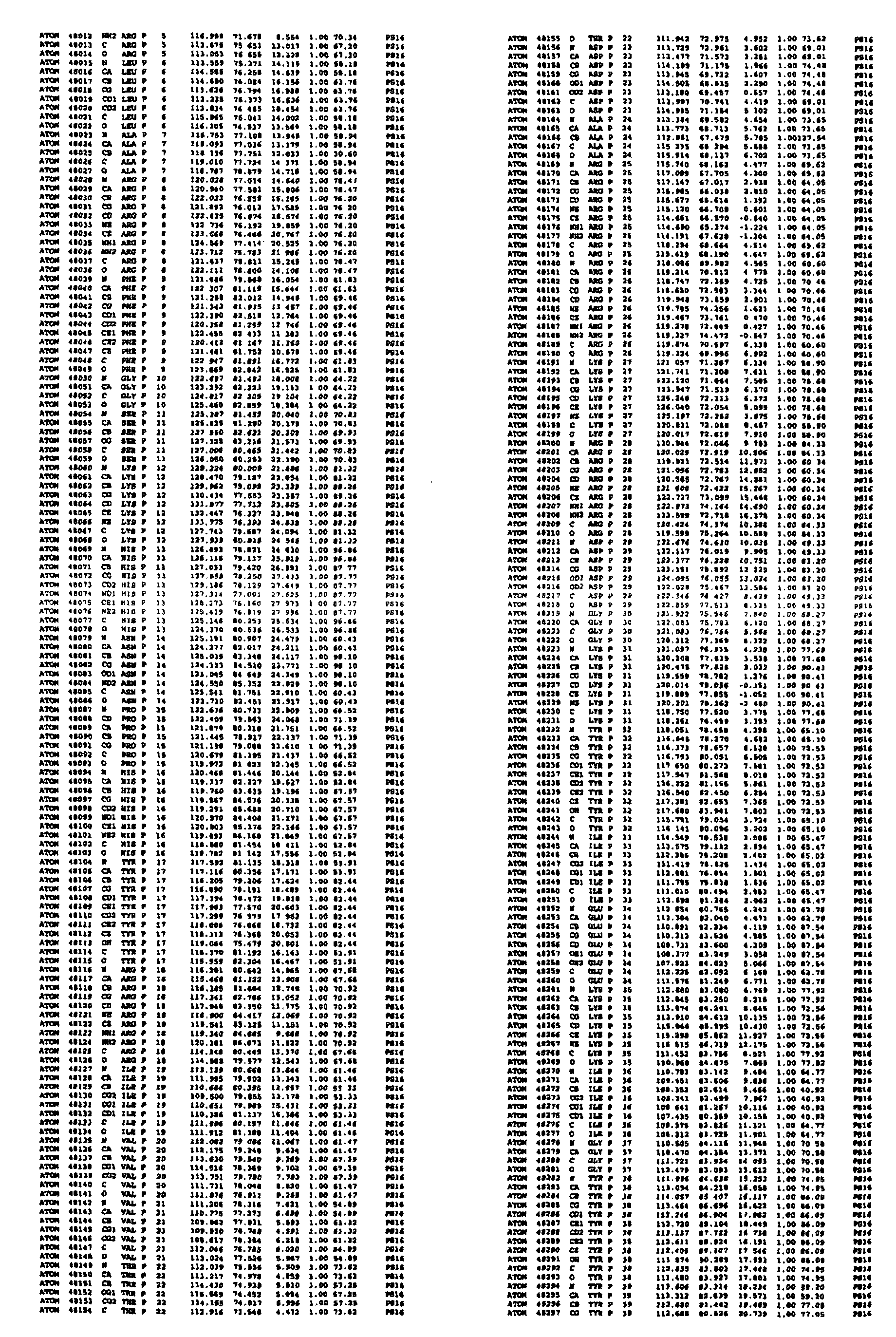

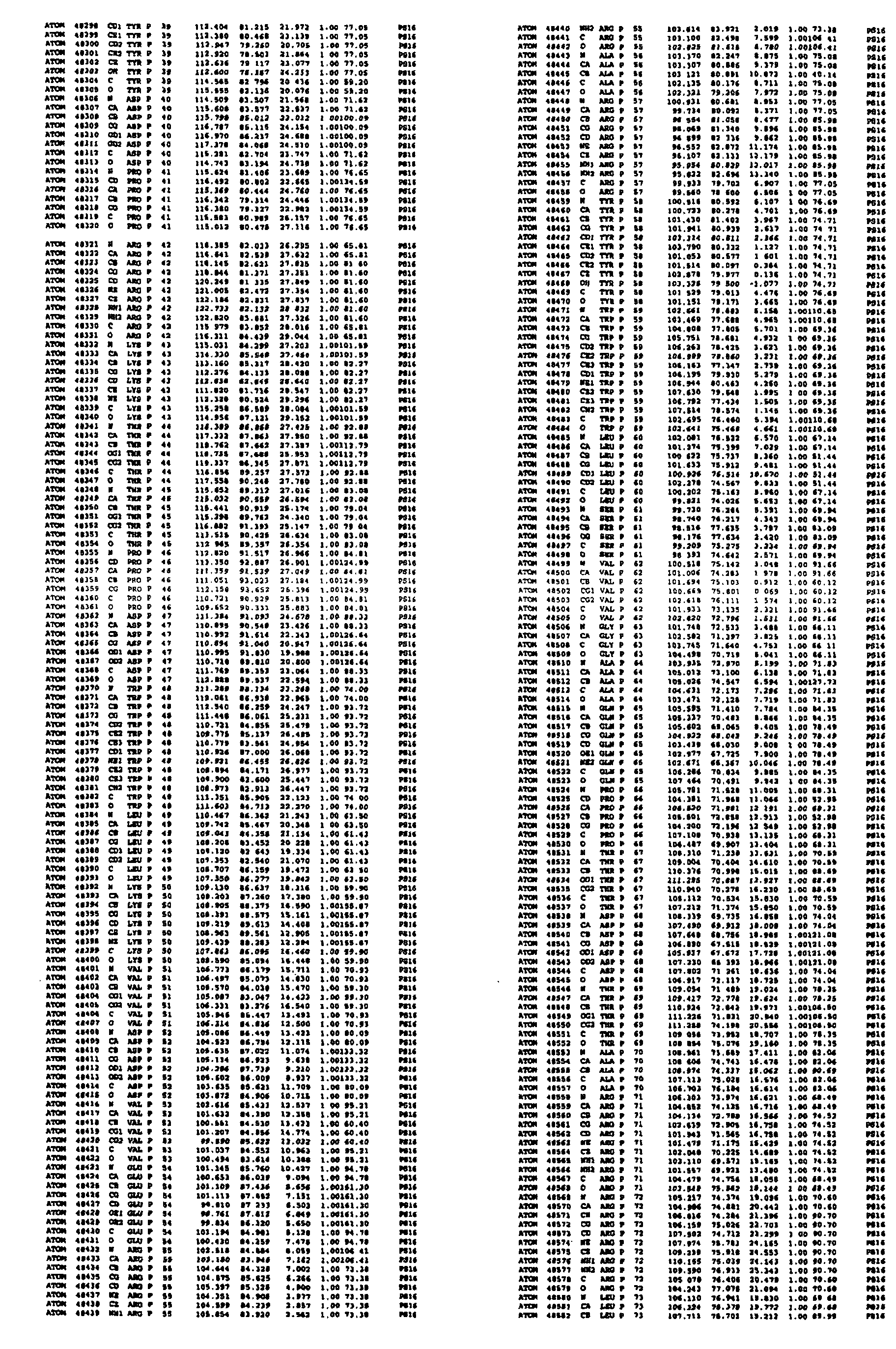

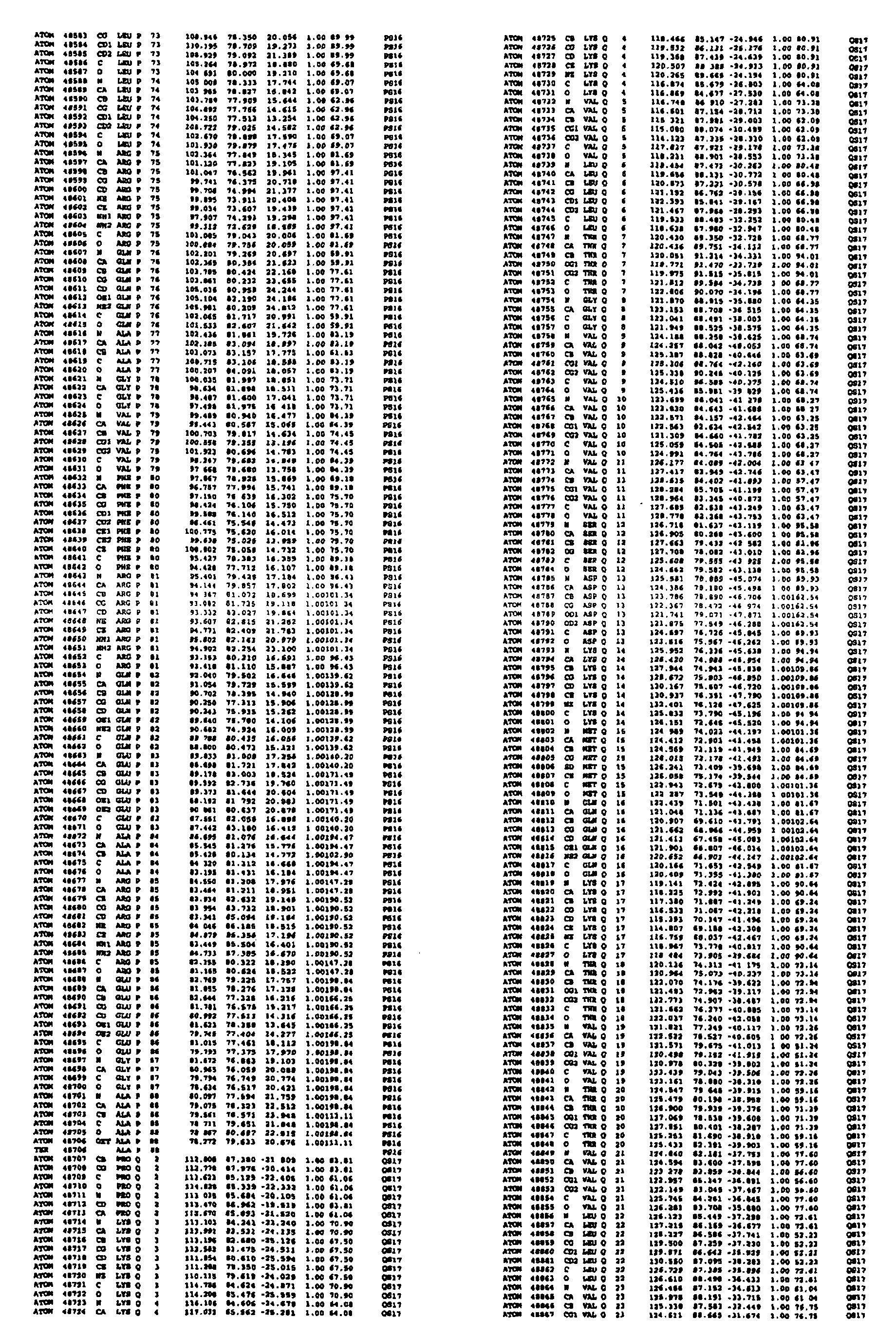

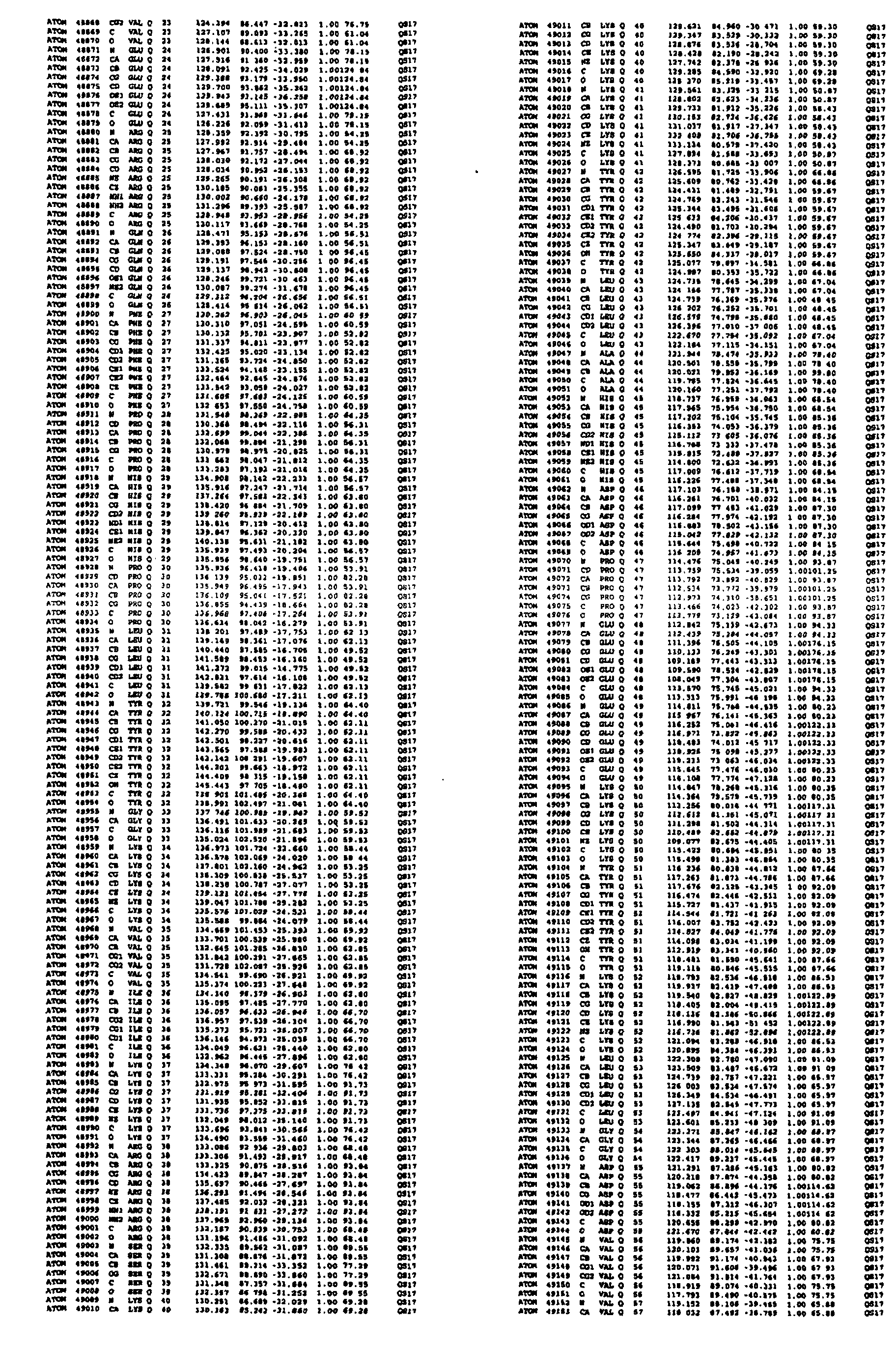

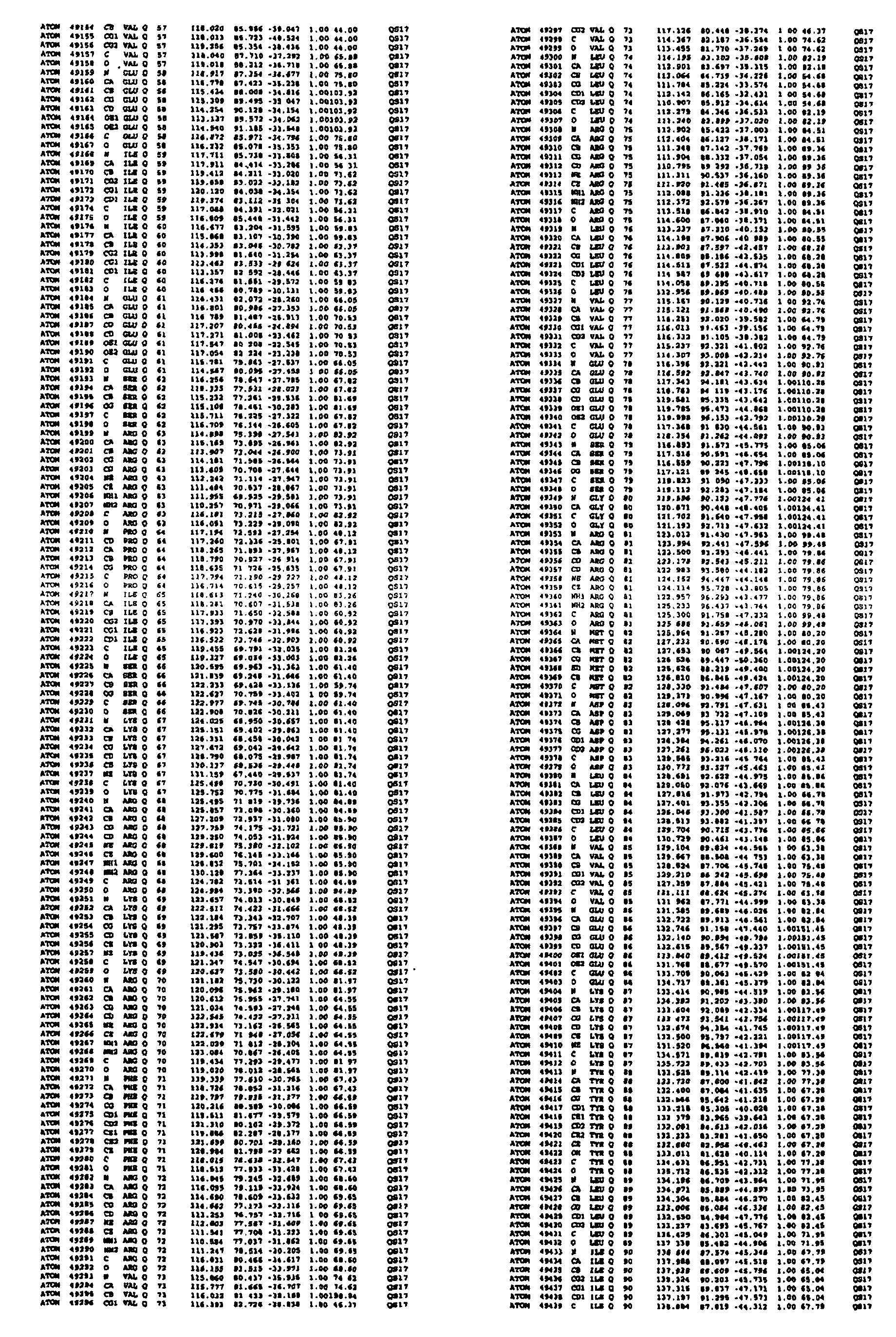

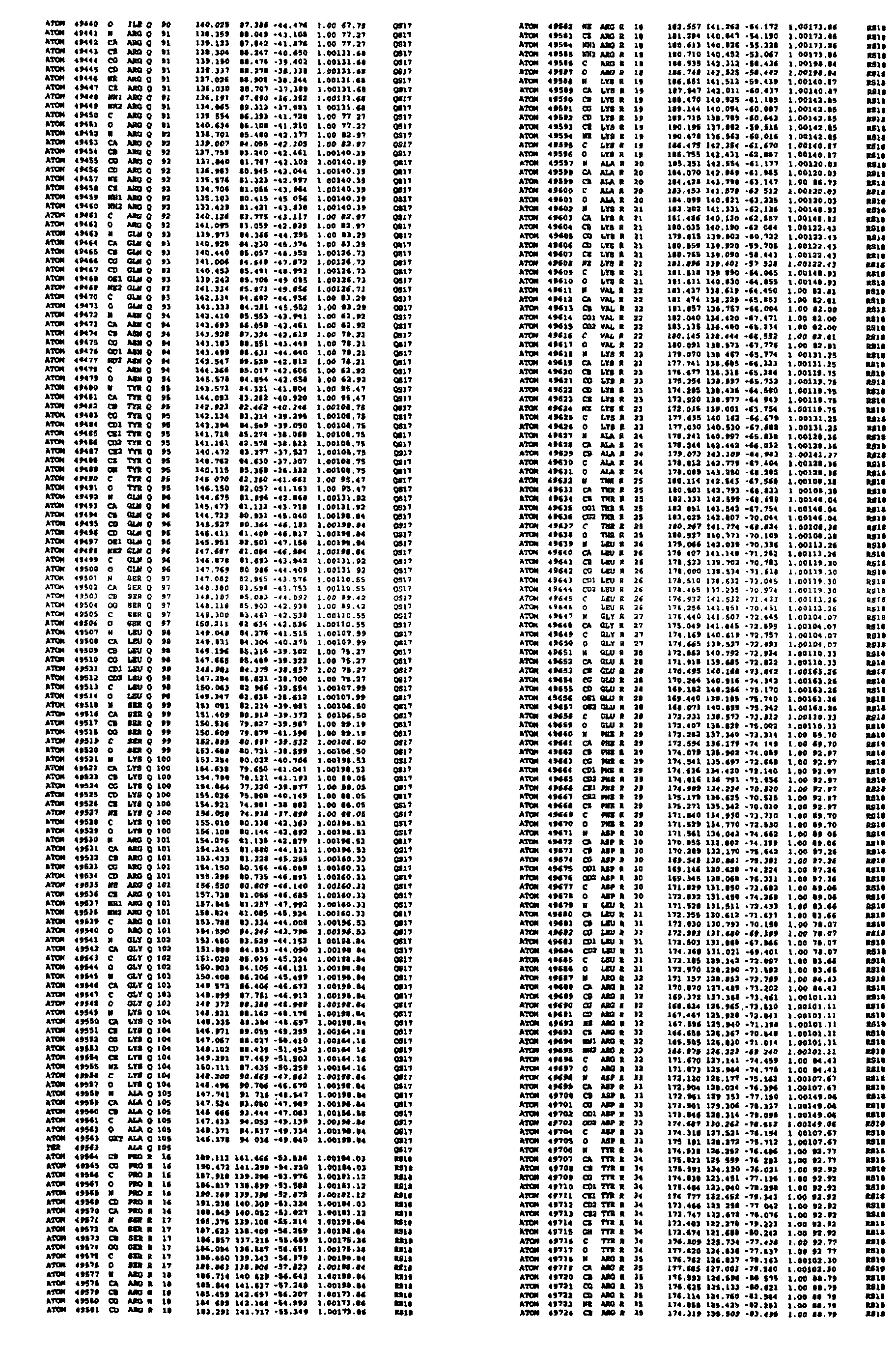

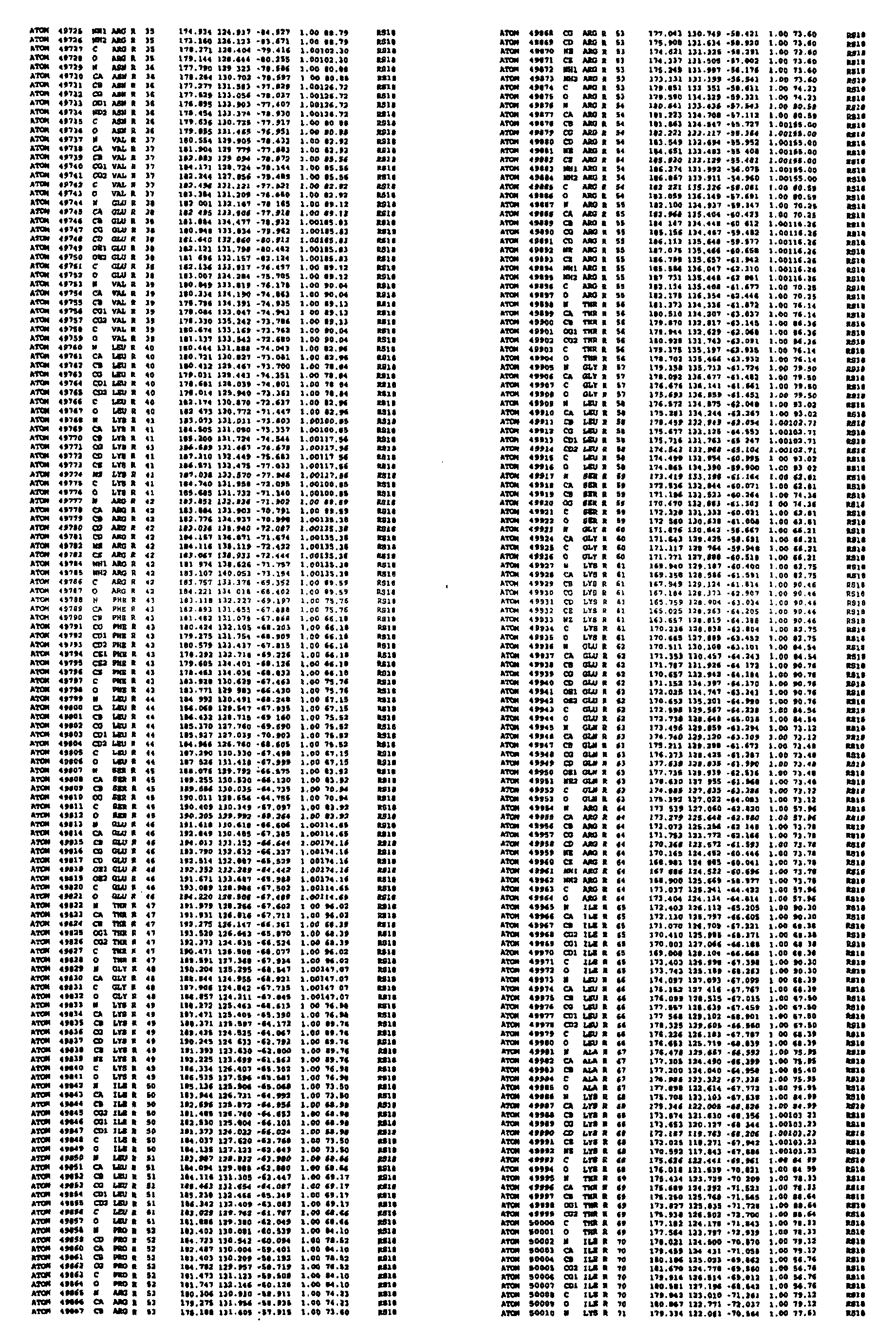

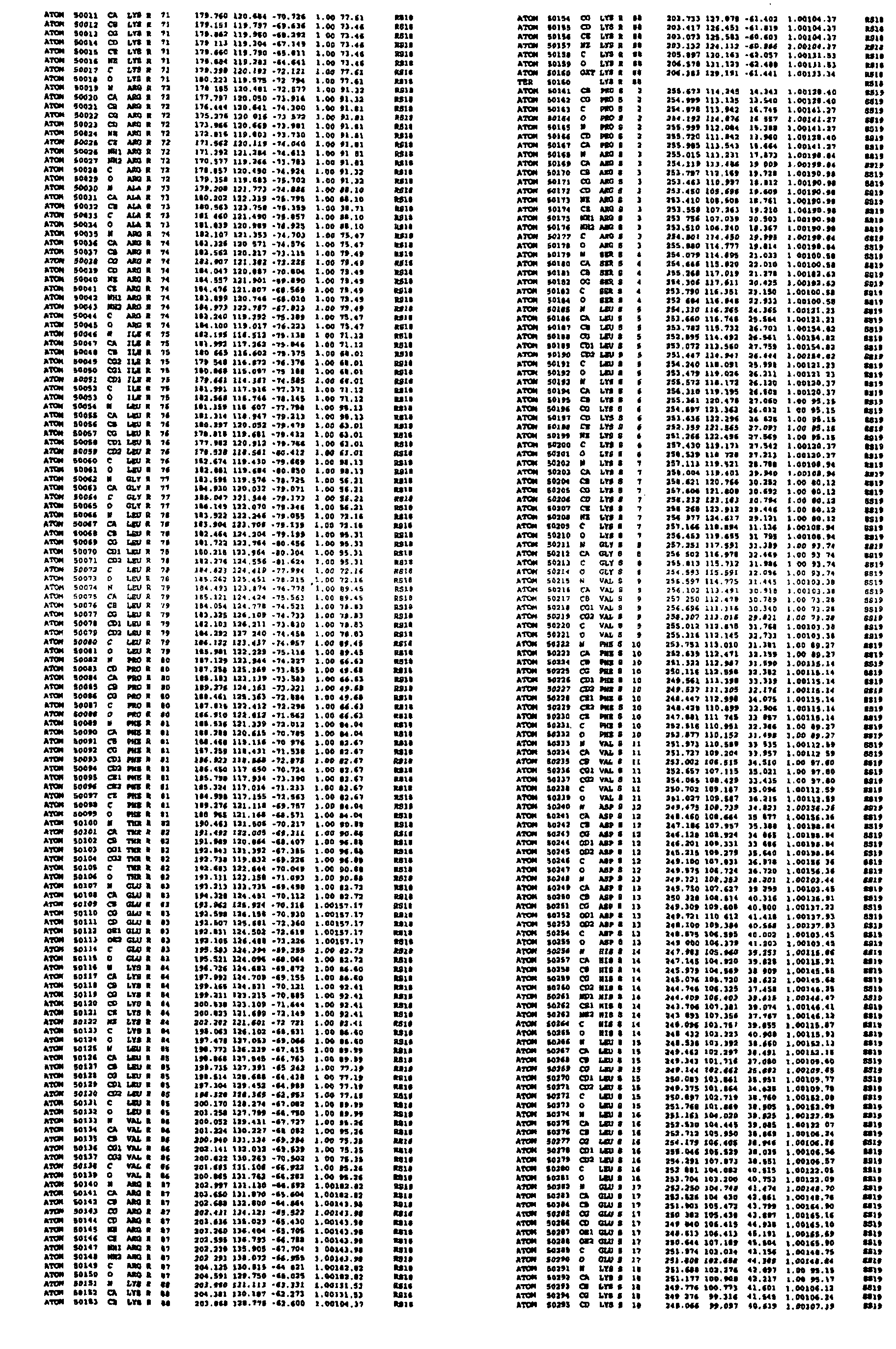

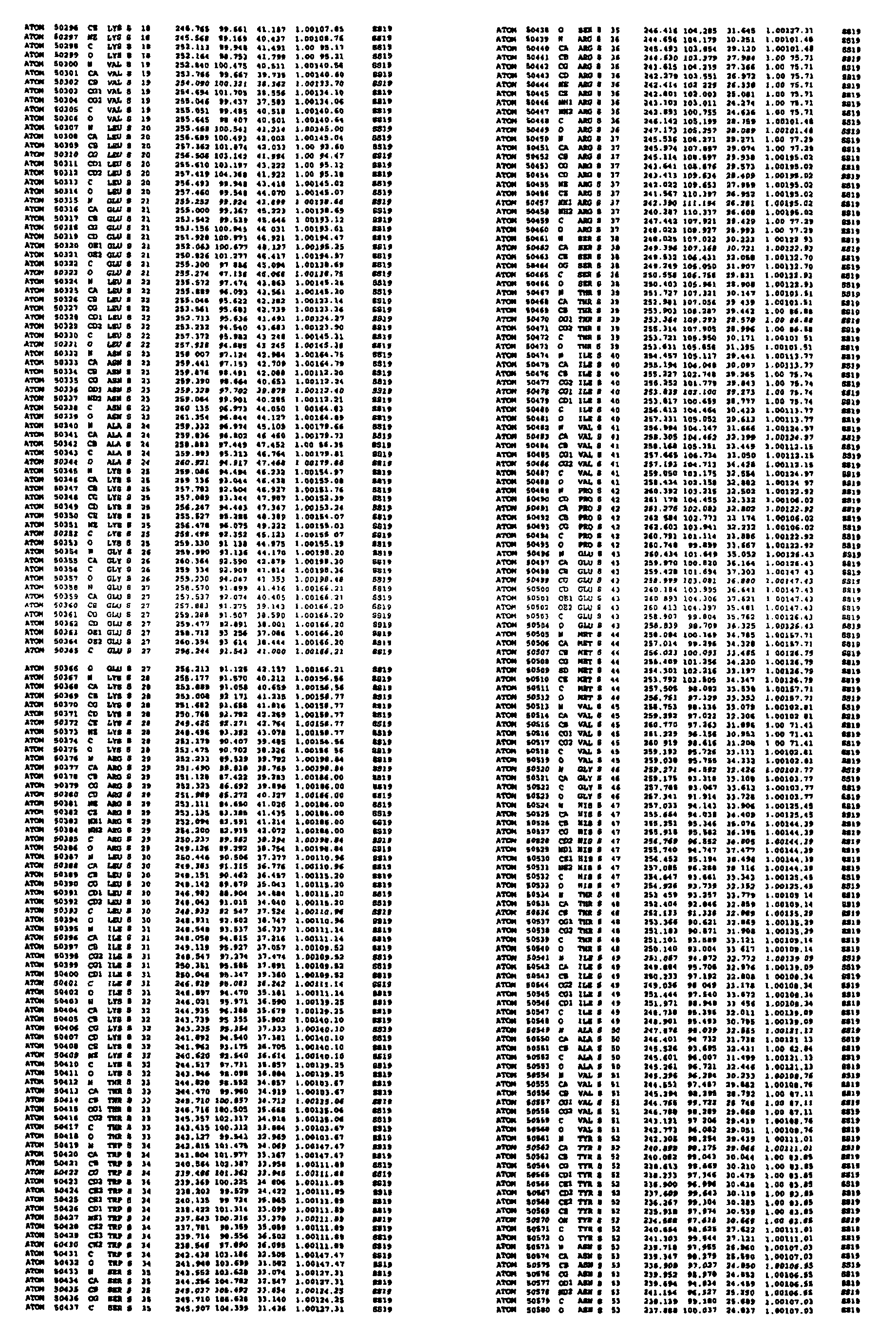

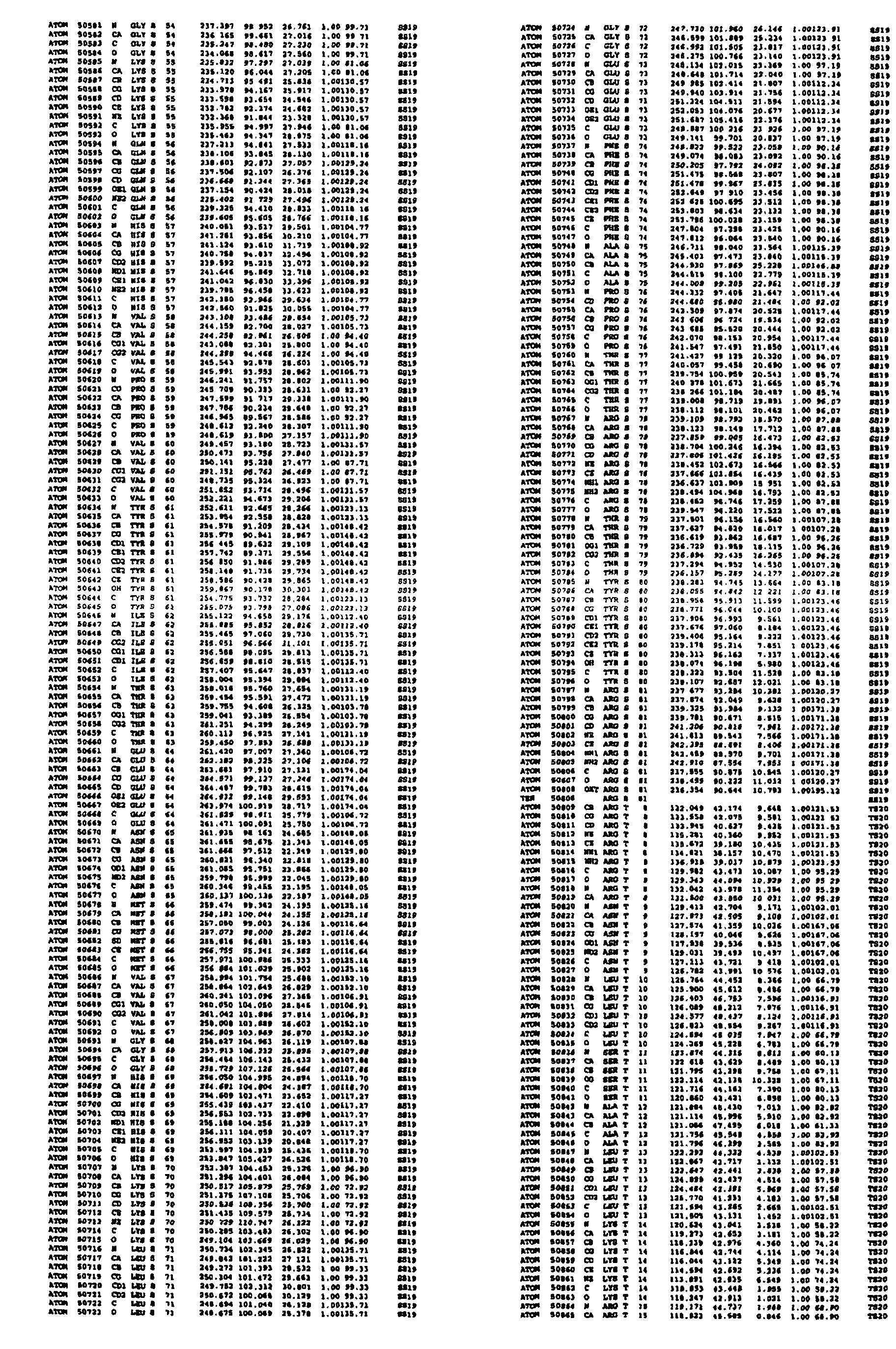

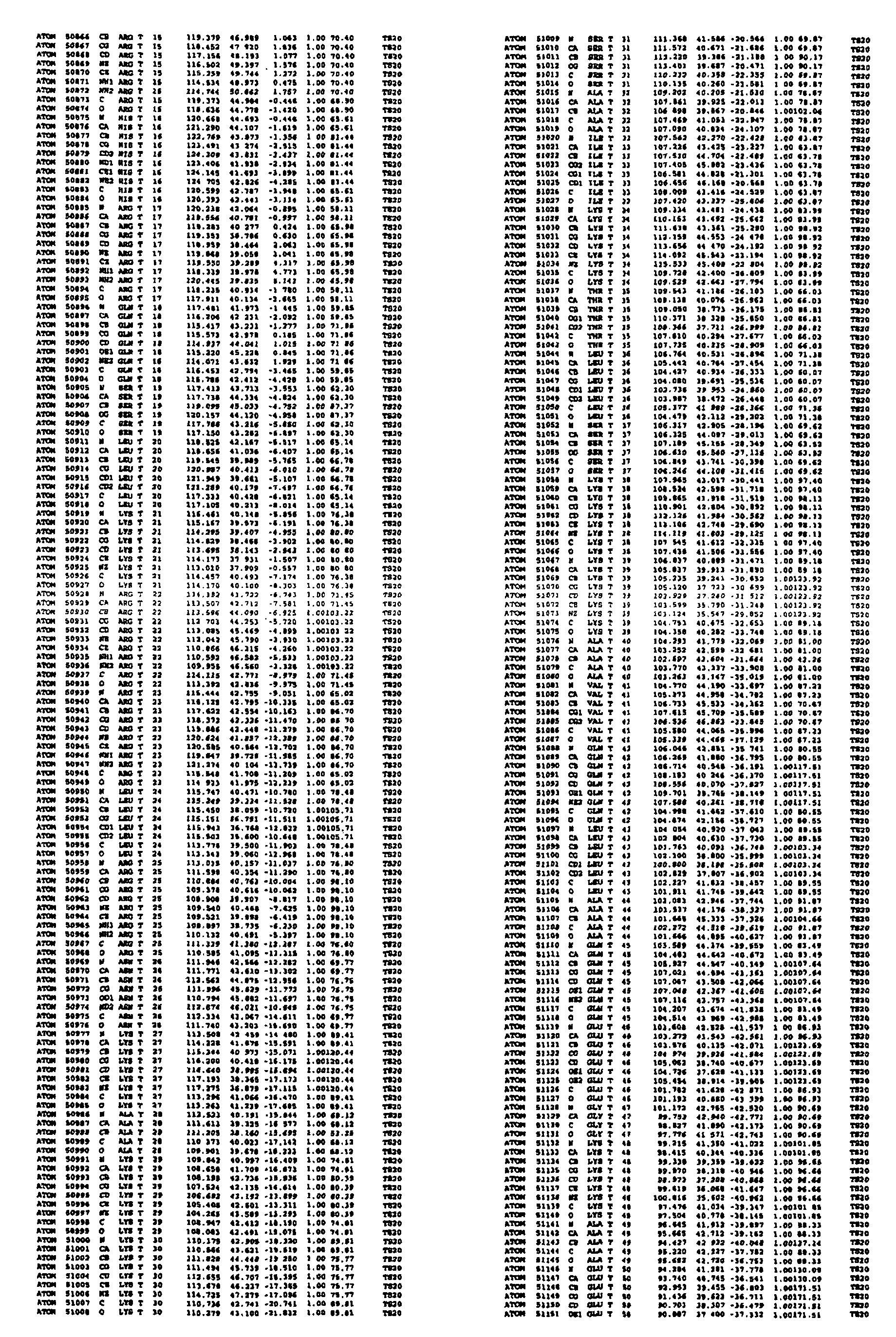

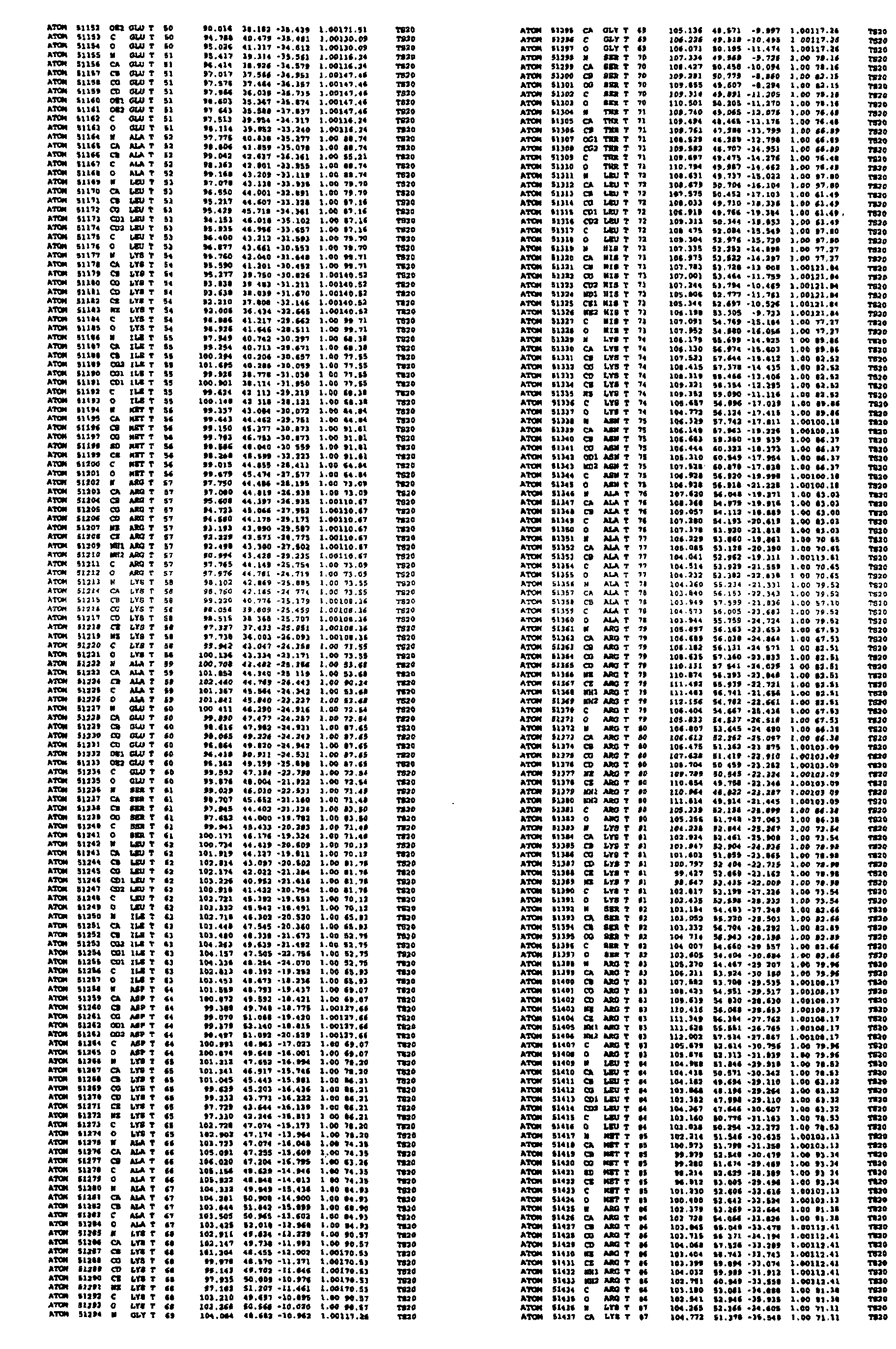

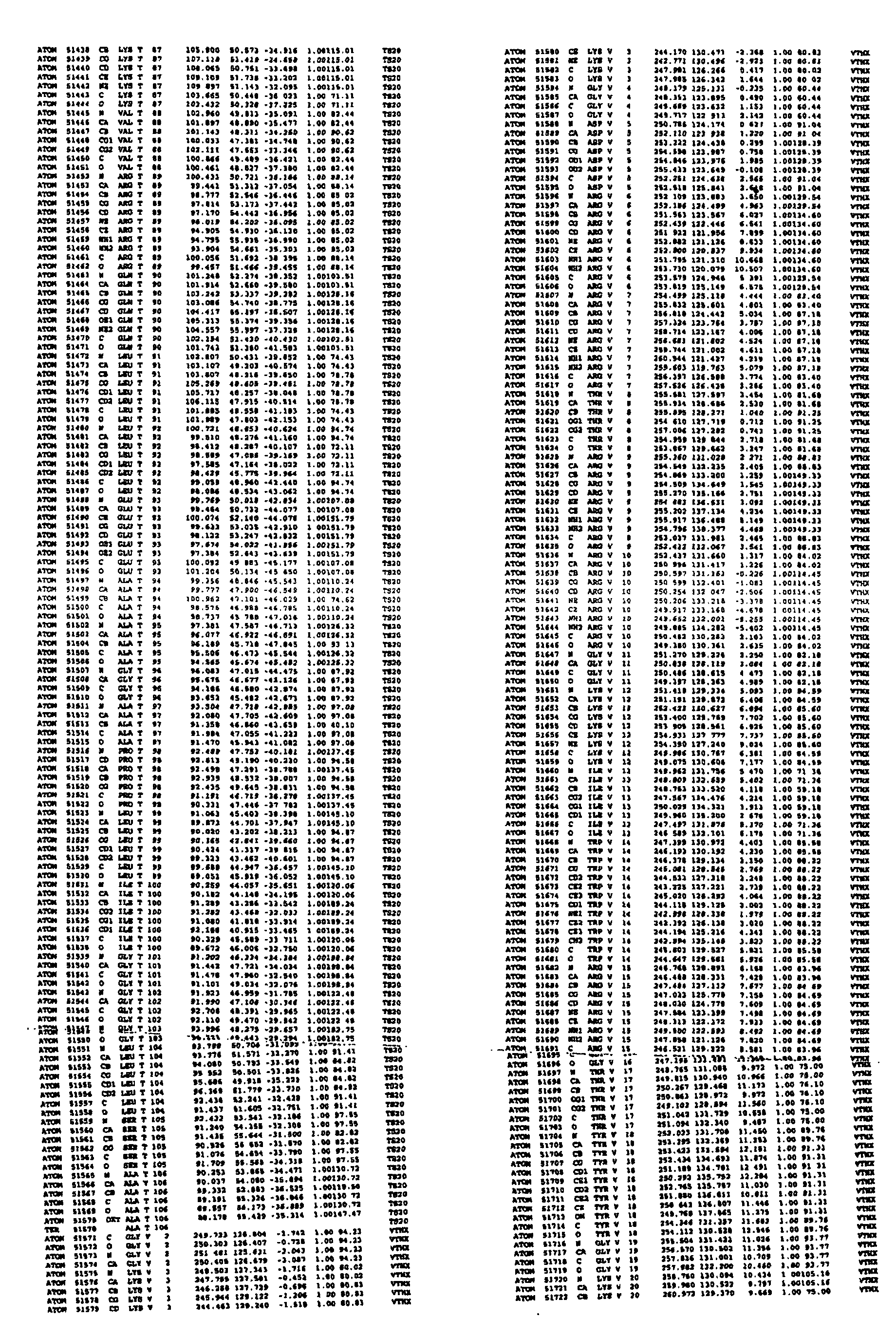

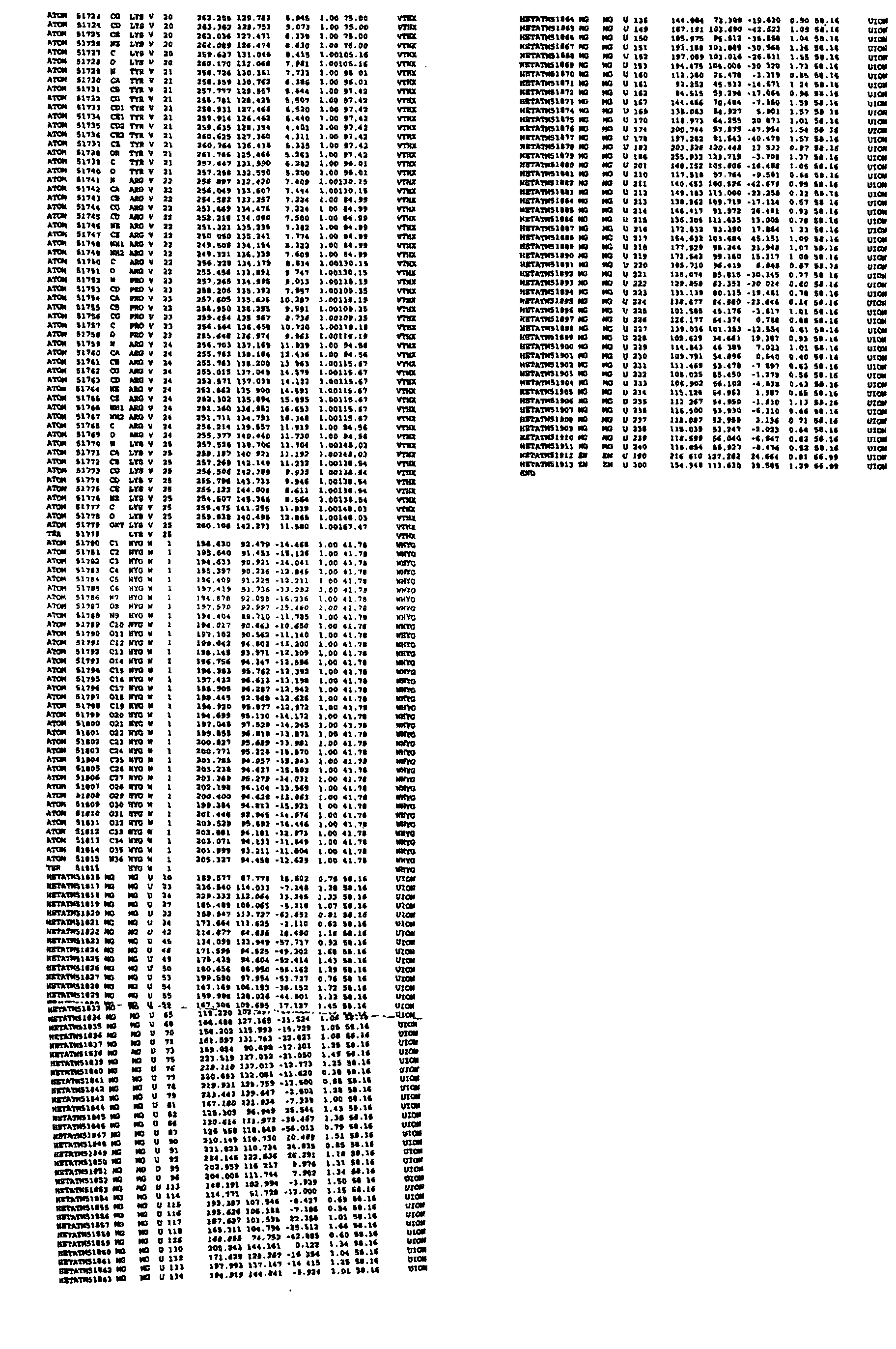

## Claims

1. A crystal of a 30S subunit bound to an antibiotic Z, (wherein Z is defined below), having a tetragonal space group P4₁2₁2 with unit cell dimensions, for each of the antibiotics Z, of:
| Z | a(Angstroms) | b(Angstroms) | c(Angstroms) |
|---|---|---|---|
| Paromomycin | 401.375 | 401.375 | 175.887 |
| Paromomycin | 401.2 | 401.2 | 176.4 |
| Streptomycin | 401.375 | 401.375 | 175.887 |
| Spectinomycin | 401.375 | 401.375 | 175.887 |
| Tetracycline | 401.158 | 410.158 | 176.944 |
| Pactamycin | 401.719 | 401.719 | 177.002 |
| Hygromycin B | 402.063 | 402.063 | 175.263 |

2. A crystal of a 30S subunit bound to the antibiotic paromomycin having a tetragonal space group P4₁2₁2 with unit cell dimensions of a = 401.4 Å, b = 401.4 Å, c = 175.9 Å.

3. A crystal of a 30S subunit bound to the antibiotic paromomycin having a tetragonal space group P4₁2₁2 with unit cell dimensions of a = 401.2 Å, b = 401.2 Å, c = 176.4 Å.

4. A crystal of a 30S subunit bound to the antibiotic Streptomycin having a tetragonal space group P4₁2₁2 with unit cell dimensions of a = 401.4 Å, b = 401.4 Å, c = 175.9 Å.

5. A crystal of a 30S subunit bound to the antibiotic Spectinomycin having a tetragonal space group P4₁2₁2 with unit cell dimensions of a = 401.4 Å, b = 401.4 Å, c = 175.9 Å.

6. A crystal of a 30S subunit bound to the antibiotic Tetracycline having a tetragonal space group P4₁2₁2 with unit cell dimensions of a = 401.2 Å, b = 401.2 Å, c = 177.0 Å.

7. A crystal of a 30S subunit bound to the antibiotic Pactamycin having a tetragonal space group P4₁2₁2 with unit cell dimensions of a = 401.7 Å, b = 401.7 Å, c = 177.0 Å.

8. A crystal of a 30S subunit bound to the antibiotic Hygromycin B having a tetragonal space group P4₁2₁2 with unit cell dimensions of a = 402.1 Å, b = 402.1 Å, c = 175.3 Å.

9. A crystal of a 30S ribosomal subunit bound to an antibiotic selected from the group paromomycin, streptomycin, spectinomycin, tetracycline, pactamycin and hygromycin B, having a resolution better (numerically less) than about 3 Å.

10. A crystal a 30S ribosomal subunit bound to an antibiotic having the structure defined by the co-ordinates of a table selected from the group of tables 1 to 4.

11. A computer-based method of rational drug design which comprises:
providing the structure of a 30S ribosomal subunit as defined by the coordinates of a table selected from the group of tables 1 to 4;
providing the structure of a candidate modulator molecule; and
fitting the structure of candidate to the structure of the 30S of said table.

12. A computer-based method for identifying a potential inhibitor of the 30S ribosome comprising the steps of:
a. employing a three-dimensional structure of 30S, or at least one sub-domain thereof, to characterise at least one active site, the three-dimensional structure being defined by atomic coordinate data according to a table selected from the group of tables 1 to 4; and
b. identifying the potential-inhibitor by designing or selecting a compound for interaction with the active site.

13. The method of claim 12 which further comprises:
c. obtaining or synthesising the potential inhibitor;
d. contacting the potential inhibitor with 30S to determine the ability of said inhibitor to interact with the 30S.

14. The method of claim 12 which further comprises:
c. obtaining or synthesising said potential ligand;
d. forming a complex of 30S and said potential ligand; and
e. analysing said complex by X-ray crystallography to determine the ability of said potential ligand to interact with 30S.

15. A computer-based method of rational drug design which comprises:
providing the coordinates of at least one atom of a table selected from the group of tables 1 to 4 of the 30S ribosome; providing the structure of a candidate inhibitor molecule;
fitting the structure of candidate to the coordinates of the 30S ribosome provided to obtain a result; and
comparing said result with a structure comprising the coordinates of the 30S ribosome provided and at least one atom from one antibiotic structure of said table.

16. The method of claim 15 wherein the coordinates comprise a subdomain of the 30S ribosome.

17. The method of claim 15 wherein the coordinates are of selected from at least one member of any one of the following groups of residues:
Group I: G1405, A1408, C1490, G1491, A1493, G1494 and U1495;
Group II: G1064, C1066, G1068 and C1192;
Group III: U14, C526, G527, A913, A914, C1490, G1941 and S12Lys45;
Group IV: A965, G966, G1053, C1054, C1195, U1196, G1197 and G1198;
Group V: U244, A892 and C893;
Group VI: G693, A694, C788, C795, C796, S7Gly81, and optionally U1540; and
Group VII: C1403, G1405, G1494, U1495, C1496 and U1498.

18. A computer system, intended to generate structures and/or perform rational drug design for the 30S ribosome or complexes of the 30S ribosome with a potential modulator, the system containing either (a) atomic coordinate data according to a table selected from the group of tables 1 to 4, said data defining the three-dimensional structure of 30S or at least one sub-domain thereof, or (b) structure factor data for 30S, said structure factor data being derivable from the atomic coordinate data of a table selected from the group of tables 1 to 4.

19. A computer readable media with either (a) atomic coordinate data according to a table selected from the group of tables 1 to 4 recorded thereon, said data defining the three-dimensional structure of the 30S ribosome, at least one atom or at least one sub-domain thereof, or (b) structure factor data for the 30S ribosome recorded thereon, the structure factor data being derivable from the atomic coordinate data of a table selected from the group of tables 1 to 4.
